# EUROPEAN PATENT APPLICATION

(11) **EP 1 553 179 A1**
(43) Date of publication of application: **13.07.2005**
(21) Application number: 03751299.3
(22) Date of filing: 01.10.2003
(51) Int. Cl.: C12N 15/09, C12N 15/12, C07K 14/47, C07K 16/18, C12N 1/15, C12N 1/19, C12N 1/21, C12N 5/10, C12P 21/02, C12Q 1/02, A61K 6/00, G01N 33/15, G01N 33/53, G01N 33/566, C07D 201/00

(54) **MOUSE-ORIGIN PROTEINS FORMING DOMAIN OR USE THEREOF**

(30) Priority: 01.10.2002 JP 2002289209
(71) Applicant: Riken, Wako-shi, Saitama 351-0198 (JP)
(72) Inventor: MAEDA, Takeshi, Yokohama Institute, RIKEN, Yokohama-shi, Kanagawa 230-0045 (JP); INOUE, Makoto, Yokohama Institute, RIKEN, Yokohama-shi, Kanagawa 2 30-0045 (JP); KIGAWA, Takanori, Yokohama Institute, RIKEN, Yokohama-shi, Kanagawa 230-0045 (JP); SHIROUZU, Mikako, Yokohama Institute, RIKEN, Yokohama-shi, Kanagawa 230-0045 (JP); HAYASHIZAKI, Yoshihide, Yokohama Institute, RIKEN, Yokohama-shi, Kanagawa 230-0045 (JP); YOKOYAMA, Shigeyuki, Yokohama Institute, RIKEN, Yokohama-shi, Kanagawa 230-0045 (JP); TAKAGI, Tetsuo, Yokohama Institute, RIKEN, Yokohama-shi, Kanagawa 230-0045 (JP); TANAKA, Akiko, Yokohama Institute, RIKEN, Yokohama-shi, Kanagawa 230-0045 (JP); HIROTA, Hiroshi, Yokohama Institute, RIKEN, Yokohama-shi, Kanagawa23 0-0045 (JP); SATOU, Kazuhito, Yokohama Institute, RIKEN, Yokohama-shi, Kanagawa 230-0045 (JP)
(74) Representative: Mackenzie, Andrew Bryan
(86) International application number: PCT/JP2003/012575
(87) International publication number: WO 2004/031384

(57) **Abstract**

The present invention is directed to obtaining a protein, which forms a SEA-like domain suitable for structural analysis, and analyzing the structural information thereof for drug development or the like. More specifically, the present invention provides a protein consisting of an amino acid sequence represented by SEQ ID NO :1 or a salt thereof; a protein having an amino acid sequence derived from an amino acid sequence represented by SEQ ID NO: 2 by deletion of 0 to 10 amino acid residues from the N-terminal and deletion of 0 to 10 amino acid residues from the C-terminal and having 120 to 139 amino acid residues, or a salt thereof; a method for manufacturing the proteins or salts thereof; a polynucleotide encoding the proteins or salts thereof; an antibody against the proteins; and a screening method using the proteins or salts thereof.

SEQUENCE LISTING

<110> RIKEN

<120> Polypeptide which composes human domain and the use
      thereof

<130> P02-0087PCT

<140>
<141>

<150> JP 2002-289209
<151> 2002-10-01

<160> 12

<170> PatentIn Ver. 2.1

<210> 1
<211> 119
<212> PRT
<213> mouse

<400> 1
Ser Ser Ser Ser Gln His Phe Asn Leu Asn Phe Thr Ile Thr Asn Leu
  1               5                  10                  15

Pro Tyr Ser Gln Asp Ile Ala Gln Pro Ser Thr Thr Lys Tyr Gln Gln

            20                  25                  30

Thr Lys Arg Ser Ile Glu Asn Ala Leu Asn Gln Leu Phe Arg Asn Ser
        35                  40                  45

Ser Ile Lys Ser Tyr Phe Ser Asp Cys Gln Val Leu Ala Phe Arg Ser
    50                  55                  60

Val Ser Asn Asn Asn Asn His Thr Gly Val Asp Ser Leu Cys Asn Phe
65                  70                  75                  80

Ser Pro Leu Ala Arg Arg Val Asp Arg Val Ala Ile Tyr Glu Glu Phe
                85                  90                  95

Leu Arg Met Thr His Asn Gly Thr Gln Leu Leu Asn Phe Thr Leu Asp
            100                 105                 110

Arg Lys Ser Val Phe Val Asp
        115

<210> 2
<211> 139
<212> PRT
<213> mouse

<400> 2
Ile Leu Leu Pro Ala Glu Ile Pro Thr Thr Ser Ser Ser Ser Gln His
1               5                   10                  15

Phe Asn Leu Asn Phe Thr Ile Thr Asn Leu Pro Tyr Ser Gln Asp Ile
            20                  25                  30

Ala Gln Pro Ser Thr Thr Lys Tyr Gln Gln Thr Lys Arg Ser Ile Glu
        35                  40                  45

Asn Ala Leu Asn Gln Leu Phe Arg Asn Ser Ser Ile Lys Ser Tyr Phe
    50                  55                  60

Ser Asp Cys Gln Val Leu Ala Phe Arg Ser Val Ser Asn Asn Asn Asn
65                  70                  75                  80

His Thr Gly Val Asp Ser Leu Cys Asn Phe Ser Pro Leu Ala Arg Arg
                85                  90                  95

Val Asp Arg Val Ala Ile Tyr Glu Glu Phe Leu Arg Met Thr His Asn
            100                 105                 110

Gly Thr Gln Leu Leu Asn Phe Thr Leu Asp Arg Lys Ser Val Phe Val
        115                 120                 125

Asp Gly Tyr Ser Gln Asn Arg Asp Asp Asp Val
    130                 135

<210> 3
<211> 357
<212> DNA

<213> mouse

<400> 3
agttccagtt ctcagcattt caacctgaac ttcaccatca ccaacctacc ctattcccaa 60
gacatagccc agccaagcac caccaaatac cagcaaacca aaagaagtat tgaaaatgcg 120
ctcaatcagc ttttcagaaa cagcagcatc aagagttact tttctgactg tcaagtatta 180
gccttcaggt ctgtctccaa caacaacaac cacacagggg tagattccct atgtaacttc 240
tcaccattgg ctcggcgagt agacagagtt gctatctatg aggaattcct tcgaatgacg 300
cacaatggta cccagttgct gaacttcaca ctggatagga agagtgtctt tgtggat 357

<210> 4
<211> 417
<212> DNA
<213> mouse

<400> 4
attcttctgc cagcagaaat accaacaacc agttccagtt ctcagcattt caacctgaac 60
ttcaccatca ccaacctacc ctattcccaa gacatagccc agccaagcac caccaaatac 120
cagcaaacca aaagaagtat tgaaaatgcg ctcaatcagc ttttcagaaa cagcagcatc 180
aagagttact tttctgactg tcaagtatta gccttcaggt ctgtctccaa caacaacaac 240
cacacagggg tagattccct atgtaacttc tcaccattgg ctcggcgagt agacagagtt 300
gctatctatg aggaattcct tcgaatgacg cacaatggta cccagttgct gaacttcaca 360
ctggatagga agagtgtctt tgtggatggg tattctcaaa acagagatga tgatgtg 417

<210> 5
<211> 132
<212> PRT

<213> Artificial Sequence

<220>
<223>配列番号１に示されるアミノ酸配列のＮ末端及びＣ末端にリンカー配列が
付加したアミノ酸配列を示す。

<400> 5
Gly Ser Ser Gly Ser Ser Gly Ser Ser Ser Ser Gln His Phe Asn Leu
1               5                   10                  15

Asn Phe Thr Ile Thr Asn Leu Pro Tyr Ser Gln Asp Ile Ala Gln Pro
            20                  25                  30

Ser Thr Thr Lys Tyr Gln Gln Thr Lys Arg Ser Ile Glu Asn Ala Leu
        35                  40                  45

Asn Gln Leu Phe Arg Asn Ser Ser Ile Lys Ser Tyr Phe Ser Asp Cys
    50                  55                  60

Gln Val Leu Ala Phe Arg Ser Val Ser Asn Asn Asn Asn His Thr Gly
65                  70                  75                  80

Val Asp Ser Leu Cys Asn Phe Ser Pro Leu Ala Arg Arg Val Asp Arg
                85                  90                  95

Val Ala Ile Tyr Glu Glu Phe Leu Arg Met Thr His Asn Gly Thr Gln
            100                 105                 110

Leu Leu Asn Phe Thr Leu Asp Arg Lys Ser Val Phe Val Asp Ser Gly

115                    120                    125

Pro Ser Ser Gly

    130

<210> 6

<211> 36

<212> DNA

<213> Artificial Sequence

<220>

<223> Description of Artificial Sequence:Primer

<400> 6

ccagcggctc ctcgggaagt tccagttctc agcatt                                36

<210> 7

<211> 38

<212> DNA

<213> Artificial Sequence

<220>

<223> Description of Artificial Sequence:Primer

<400> 7

ccagcggctc ctcgggaatt cttctgccag cagaaata                              38

<210> 8

<211> 36

<212> DNA

<213> Artificial Sequence

<220>

<223> Description of Artificial Sequence:Primer

<400> 8

cctgacgagg gccccgaatc cacaaagaca ctcttc                                36

<210> 9

<211> 38

<212> DNA

<213> Artificial Sequence

<220>

<223> Description of Artificial Sequence:Primer

<400> 9

cctgacgagg gccccgacac atcatcatct ctgttttg                              38

<210> 10

<211> 227

<212> DNA

<213> Artificial Sequence

<220>

<223> Description of Artificial Sequence:Primer

<400> 10

gctcttgtca ttgtgcttcg catgattacg aattcagatc tcgatcccgc gaaattaata 60

cgactcacta tagggagacc acaacggttt ccctctagaa ataattttgt ttaactttaa 120

gaaggagata tacatatgaa aggcagcagc catcatcatc atcatcacga ttacgatatc 180

ccaacgaccg aaaacctgta ttttcaggga tccagcggct cctcggg 227

<210> 11

<211> 187

<212> DNA

<213> Artificial Sequence

<220>

<223> Description of Artificial Sequence:Primer

<400> 11

cggggccctc gtcaggataa taattgattg atgctgagtt ggctgctgcc accgctgagc 60

aataactagc ataacccctt ggggcctcta aacgggtctt gaggggtttt ttgctgaaag 120

gaggaactat atccggataa cctcgagctg caggcatgca agcttggcga agcacaatga 180

caagagc 187

<210> 12

<211> 20

<212> DNA

<213> Artificial Sequence

<220>
<223> Description of Artificial Sequence:Primer

<400> 12
gctcttgtca ttgtgcttcg                                                    20

## Description

### Technical Field

The present invention relates to a mouse-derived protein forming a domain, a polynucleotide comprising the same, an antibody against the protein, and a method for screening an active compound using them.

### Background Art

Recently, genome nucleotide sequences of various model organisms, as represented by the human genome project, have been decoded one after another. "Structural genomics" is rapidly recognized as a new research area, and large-scale projects are progressing around the world. With respect to a large number of genes extracted from a mass of information about genomic sequences, structural genomics aims at systematic clarification of three-dimensional structure of a domain protein encoded by each gene and defining structure-function relationship.

In structural genomics, the number of proteins to be analyzed is considered to be 100,000 kinds, and it is not realistic to have a goal to determine the three-dimensional structures of all the proteins at present technical level.

Thus, it is first necessary to narrow down the number of target proteins to a reasonable number and select "representative structures". As a comprehensive three-dimensional structure analysis, projects having selected analysis targets from various viewpoints are initiated: for example, a) specifying relatively small sets of proteins as targets; b) specifying the kind of organisms having a small genome size such as hyperthermophilic archaebacterium, extreme thermophile, and mycoplasma; c) specifying life phenomena, such as proteins as disease-associated gene products, and proteins involved in signal transduction or gene expression.

In the flow of this research, as the first step, it is one goal to determine one or more representative three-dimensional structure regarding all the families, the number of which is predicted to be about 10, 000 (classified as family when amino acid sequences have about 30 to 35% homology). When one representative three-dimensional structure (basic structure) is obtained, structures of other proteins belonging to the same family can be analogized by modeling based on homology. In the flow of this research, as the first step, it is one goal to determine one or more representative three-dimensional structure regarding all the domain families, the number of which is predicted to be about 10, 000 (classified as family when amino acid sequences have about 30 to 35% homology). When one representative three-dimensional structure (domain) is obtained, structures of other domains belonging to the same family can be analogized by modeling based on homology.

In this project, attention is drawn to the type of three-dimensional structure or the topology (basic structure: fold) of a functional domain, and research to clarify the correlation with a function for a basic structural unit of protein receives attention.

A protein having a plurality of domains is formed by combining functional domains as parts, and thus it is frequent that one domain appears in various proteins with the combination of different domains. Further, even if homology is not detected on a primary sequence, it is not unusual that proteins have the same domain. Therefore, the number of domains must be much smaller than the number of protein families, and it is expected that individual domains are associated with molecular functions. The number of the domains is predicted to be about 10,000 to 20,000, and if the analysis targets are within this number order it is sufficiently possible to determine the three-dimensional structures of all the target proteins.

Thus obtained information regarding the three-dimensional structure and functions of protein provides new findings for elucidation of vital functions, and makes a dramatic progress in developing drugs etc. (e.g. development by rational drug design or virtual screening). Therefore, such information is very useful in the industry.

In the meantime, a SEA domain is found in sea urchin sperm protein, enterokinase, agrin, and mucin (MUC1) serving as a lung cancer marker protein, and conceivably involved in facilitating or controlling binding of a sugar chain adjacent to a protein.

SEA is an extracellular domain involved in O-glycosylation (see, e.g., Bork P. and Patthy L.Protein Science, vol. 4, pp. 1421-1425, (1995). Proteins having SEA have a common feature in that most of them are glycosilated. Furthermore, a protein well analyzed contains sugar chains bonded via an O-glycoside bond, such as heparan sulfate, which changes the molecular weight of the protein, significantly.

What is important herein is the following well-known fact applicable to various types of cancer cells, that is, the molecular weight of a sugar chain to be added to a protein increases specifically in a cancer cell. It has been reported that, expression of, for example, MUC1 having a SEA domain is augmented in lung cancer, brain tumor, and adenocarcinoma and a bulky sugar chain is observed (see, e.g., Croce Mv et. al., Pathol. Oncol. Res. Vol. 7(4) pp.284-291, (2001)). This protein has high adhesiveness due to the presence of a sugar chain and therefore, conceivably has a significant effect upon metastasis and malignant alteration of cancer. As an immunological therapy for cancer, a specific antigen to the protein has been used (see, e.g., Heukamp LC et. al., J. Immunother Vol.25 (1) pp. 45-56 (2002)).

However, the fact is that three-dimensional structure analysis of protein requires a lot of time, labor and cost. In structural genomic research aiming at comprehensive and systematic structure analysis, it is an important challenge to attain high throughput structure analysis.

For the analysis of a three-dimensional structure of protein, NMR method and X-ray crystallography are mainly used. To analyze a three-dimensional structure of protein using NMR, it is preferable that a sample has a molecular weight of about 20,000 or less (about 200 or less amino acid residues). In the case of analysis of the three-dimensional structure by X-ray analysis, the properties of proteins are limited due to preparation of crystals. When a protein is randomly cleaved to obtain a protein suitable for structure analysis and a cleavage site exists in an amino acid sequence having a β-sheet or α-helix structure, many proteins modify their physiologically significant structures, become a string-like shape not taking a structure, or aggregate. In this way, it is meaningless to analyze a three-dimensional structure of protein not having an original in vivo structure. Therefore, it is desirable to obtain a protein forming a significant domain for three dimensional analysis.

To express a protein having a domain suitable for structural analysis (hereinafter referred to as "a protein forming a domain"), information regarding the position of domain boundary is necessary. In general, such domain boundary is predicted using amino acid sequence homology or the like as a clue. Even if protein expression is conducted based on the amino acid sequence of the thus predicted domain region, there is very low probability that a protein forming a domain actually having a structure (folding) is obtained and thus domain expression is one of bottlenecks in structure analysis.

A protein forming a SEA-like domain of the present invention has not been obtained so far, and the structure information thereof is unknown. Thus, these cannot be used for the drug discovery.

### Disclosure of the Invention

In view of such circumstances, the present invention has been accomplished, and the present invention provides a protein described below, a production method thereof, a polynucleotide coding therefor, an antibody against the protein, and a screening method using them.
(1) A protein consisting of an amino acid sequence represented by SEQ ID NO: 1 or a salt thereof.
(2) A protein having an amino acid sequence derived from an amino acid sequence represented by SEQ ID NO: 2 by deletion of 0 to 10 amino acid residues from the N-terminal and deletion of 0 to 10 amino acid residues from the C-terminal and having 120 to 139 amino acid residues, or a salt thereof.
(3) A protein consisting of an amino acid sequence derived from an amino acid sequence of a protein represented by SEQ ID NO: 1 or 2 and having deletion substitution or addition of one or several amino acids and having a function substantially identical with the protein according to item (1) or (2), or a salt thereof.
(4) A polynucleotide containing a polynucleotide encoding the amino acid sequence of a protein according to any one of items (1) to (3).
(5) The polynucleotide according to item (4), containing a nucleotide sequence represented by SEQ ID NO: 3 or 4.
(6) An expression system containing a polynucleotide according to item (4) or (5).
(7) A recombinant vector containing a polynucleotide according to item (4) or (5).
(8) A transformant which is transformed with a polynucleotide according to item (4) or (5).
(9) An antibody against a protein according to any one of items (1) to (3) and/or a salt thereof.
(10) A pharmaceutical agent containing an antibody according to item (9).
(11) A method for producing a protein or a salt thereof according to any one of items (1) to (3), comprising a step of culturing a transformant of item (8) and producing the protein.
(12) A method for producing a protein or a salt thereof according to any one of items (1) to (3), characterized by using a cell-free protein synthesis system.
(13) A method for screening a substance interacting with a protein or a salt thereof according to any one of items (1) to (3) and/or a naturally existing protein or a salt thereof containing an amino acid sequence of a protein according to any one of items (1) to (3), comprising steps of bringing a candidate substance into contact with the protein or a salt thereof according to any one of items (1) to (3); and confirming whether the candidate substance interacts with the protein or a salt thereof.
(14) A method for assaying a protein or a salt thereof according to any one of items (1) to (3) using an antibody of item (9).
(15) A method for screening a substance interacting with a protein or a salt thereof according to any one of items (1) to (3) by using an assay method of item (14).
(16) A method for specifying a gene associated with a protein according to any one of items (1) to (3), comprising steps of expressing a protein according to any one of items (1) to (3) in a cell; and examining an expression status of the gene in the cell.
(17) A method for screening a compound interacting with a protein or a salt thereof according to any one of items (1) to (3) and/or a naturally existing protein or a salt thereof containing an amino acid sequence of a protein according to any one of items (1) to (3), comprising steps of determining an active site of the protein using information concerning a three-dimensional structure of the protein according to any one of items (1) to (3); and searching a compound interacting with the active site on a computer.
(18) The screening method according to item (17), wherein the information concerning a three-dimensional structure of the protein is three-dimensional structure information of a protein comprising amino acid residues from amino acid 8 to amino acid 126 among three-dimensional structure information described in any one of three-dimensional structure coordinate tables 1 to 20.
(19) The screening method according to item (17), wherein, among three-dimensional structure information described in three-dimensional structure coordinate table 1, a part of information corresponding to amino acid residues of ASN15, ASN17, PHE18, THR19, LEU67, ARG70, SER71, VAL72, SER73, ASN74, HIS78, GLY80, ASP82, ASP119, SER122, ASP126, SER127 is used.
(20) A method for screening a substance interacting with a protein or a salt thereof according to any one of items (1) to (3) and/or a naturally existing protein or a salt thereof containing an amino acid sequence of a protein according to any one of items (1) to (3), comprising the steps of preparing specified compound interacting with the active site as a candidate substance by a screening method according to any one of claims 17 to 19, and bringing the candidate substance into contact with a protein or a salt thereof according to any one of claims 1 to 3; and confirming whether the candidate substance has interaction with the protein or a salt thereof.
(21) A method for presuming a three-dimensional structure of a protein with an unknown structure, wherein homology modeling is conducted on the protein with an unknown structure comprising an amino acid sequence having 30% or more homology with an amino acid sequence of a protein according to any one of items (1) to (3), by using information concerning three-dimensional structure information of a protein having amino acid residues from amino acid 8 to amino acid 126 among three-dimensional structures of a protein described in any one of three-dimensional structure coordinate tables 1 to 20.
(22) A compound inhibiting cellular proliferation activity, characterized in that the compound is obtained by a method according to any one of items (13), (15), and (17) to (20).
(23) A cellular proliferation activity inhibitor comprising, as an active ingredient, at least one compound selected from the group consisting of the following compounds i):
   i) Compounds represented by items (a) to (e) or a salt thereof:
      (a) N-(1,3,5-trimethyl-1H-pyrazol-4-ylmethyl)-7-trifluoromethyl-5,6-dihydro-7a,8,11-triazacyclopenta[b]phenanthrene-9-carboxamide represented by the following structural formula (I): 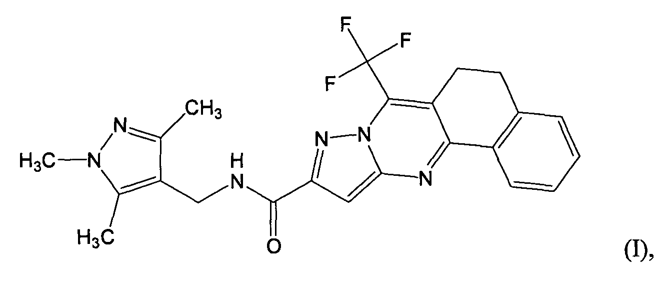
      (b) 1-[3-[4-(10,11-dihydro-dibenzo[b,f]thiepin-10-yl) piperazin-1-yl]propyl]-1,3-dihydrobenzimidazol-2-one represented by the following structural formula (II): 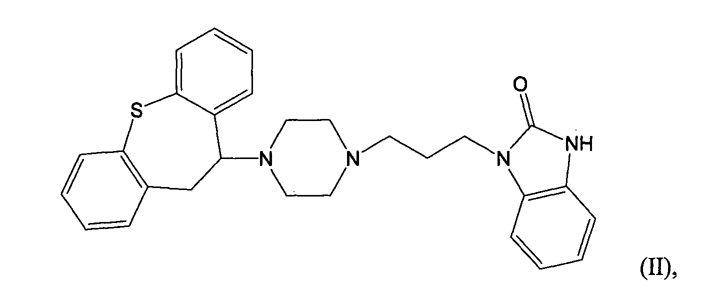
      (c) 2-(3,4-dimethylphenyl)-2-oxoethyl-2-(3,5-dioxo-4-aza-dibenzo [8,9,10,11]tricyclo[5,2,2,0^{2,6}]undecan-4-yl)acetate represented by the following structural formula (III): 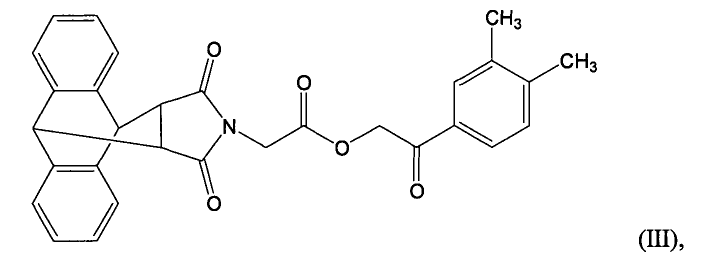
      (d) 2-[4-ethyl-5-(4-methylphenylamino)methyl-4H-[1,2,4] triazol-3-yl]sulfanyl-1-(phenothiazin-10-yl)-1-ethanone represented by the following structural formula (IV): 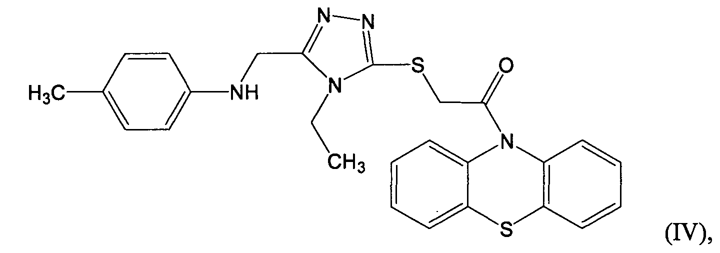 or
      (e) 1-(8-chloro-3-fluoro-10,11-dihydro-dibenzo[b,f] thiepin-10-yl)-4-[2-(1,3-dioxolan-2-yl)ethyl] piperazine represented by the following structural formula (V): 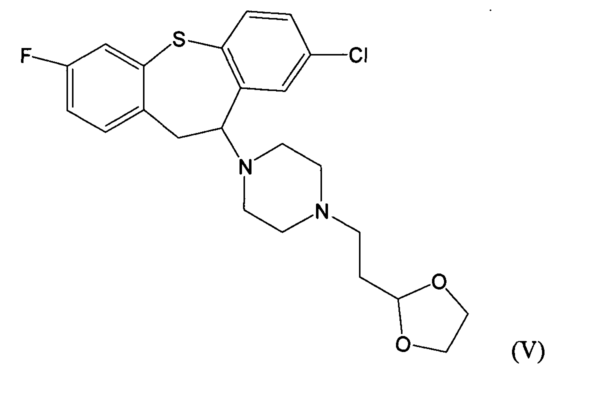
(24) The cellular proliferation activity inhibitor according to item (23) for inhibiting proliferation activity of a cancer cell derived from an ovarian cancer cell.
(25) A method for producing a pharmaceutical composition containing a compound of inhibiting a cellular proliferation activity, characterized in that the method comprises a step of blending a compound obtained by a method according to any one of items (13), (15), and (17) to (20) with a pharmaceutically acceptable carrier.

### Brief Description of the Drawings

Figure 1 is a photograph showing expression of a protein by a SDS gel (attached in place of a drawing) in which reference symbol A indicates expression of a protein having an amino acid sequence from amino acid residue 133 to 251 of a SEA-like domain 1110008I14 protein derived from a mouse; and reference symbol B indicates expression of a protein having an amino acid sequence from amino acid residue 123 to amino acid residue 261, which are observed by SDS gel;

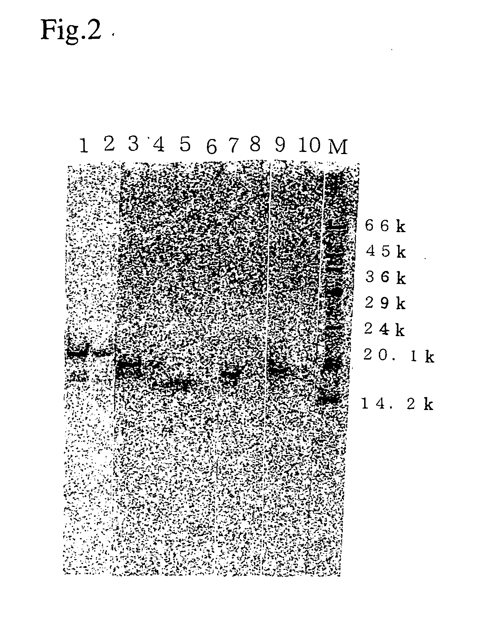

Figure 2 is a photograph of an SDS gel showing expression of a protein in Comparative Example (attached in place of a drawing) in which lanes of odd numbers indicate total amounts and lanes of even numbers indicate the supernatants thereof. The first and second lanes indicate the results of a polypeptide having amino acid residue 113 to 281 of a protein 1110008I14 derived from a mouse; the third and fourth lanes indicate the results of a polypeptide having amino acid residue 133 to 281; the fifth and sixth lanes indicate the results of a polypeptide having amino acid residue 143 to 251; the seventh and eighth lanes indicate the results of a polypeptide having amino acid residue 143 to 261; the ninth and tenth lanes indicate the results of a polypeptide having amino acid residue 143 to 271 of the same protein;
Figure 3 is a graph showing a one-dimensional magnetic resonance spectrum and ¹H-¹⁵NHSQC spectrum of a protein having an amino acid sequence of amino acid residue 133 to 251 of 1110008I14 protein;
Figure 4 is a graph showing a one-dimensional magnetic resonance spectrum and ¹H-¹⁵NHSQC spectrum of a protein having an amino acid sequence of amino acid residue 123 to 261 of 1110008I14 protein;
Figure 5 is a ribbon model (three dimensional structure) of a domain protein of the present invention; and
Figure 6 is a graph showing the measurement results of cellular proliferation.

### Best Mode for Carrying Out the Invention

### (Protein of the Invention)

A protein according to the present invention has a domain with a three-dimensional structure formed therein. More specifically, the present invention relates to a SEA-like domain protein represented by SEQ ID NO: 1 (an amino acid sequence from amino acid residue 133 to 251 of 1110008I14 protein).The 1110008I14 protein refers to one identified by DDBJ Accession No. AK003577.1 (see home page of RIKEN genomic sciences center: http://genome.gsc.riken.go.jp/, FANTOM DB clone ID. 1110008I14).

### (SEA (Sea urchin sperm protein, Enterokinase, Agrin) domain)

A SEA domain is found in sea urchin sperm protein, enterokinase, agrin, and mucin (MUC1) serving as a marker protein for lung cancer, and conceivably involved in facilitating or controlling binding of a sugar chain adjacent to a protein, as described above.

SEA is an extracellular domain involved in O-glycosylation (see, e.g., Bork P. and Patthy L., Protein Science, vol. 4, pp.1421-1425, (1995). Proteins having SEA have a common feature in that most of them are glycosilated. Furthermore, a protein well analyzed contains sugar chains bonded via an O-glycoside bond, such as heparan sulfate, which changes the molecular weight of the protein, significantly.

What is important herein is the following well-known fact applicable to various types of cancer cells, that is, a sugar chain to be added to a protein is bulky and specific to a cancer cell. It has been reported that, expression of, for example, MUC1 having a SEA domain is augmented in lung cancer, brain tumor, and adenocarcinoma, and a bulky sugar chain is observed(see, e.g., Croce Mv et. al., Pathol. Oncol. Res. Vol. 7(4) pp.284-291, (2001)). This protein has high adhesiveness due to the presence of a sugar chain and therefore, conceivably has a significant effect upon metastasis and malignant alteration of cancer. As an immunological therapy for cancer, a specific antibody to the protein has been used (see, e.g., Heukamp LC et. al., J. Immunother Vol. 25(1) pp. 45-56 (2002)).

The change of a sugar chain specifically observed in cancer is conceivably dependent upon the function of the SEA domain, which controls addition of a sugar chain. Therefore, if the three-dimensional structure of the SEA-like domain of the present invention is elucidated, it is possible to effectively design and develop a compound having an activity of inhibiting addition of a sugar chain based on the structural information thus analyzed, thereby developing a pharmaceutical agent inhibiting metastasis and malignant alteration of cancer.

Further, the present invention provides: a protein or a salt thereof comprising an amino acid sequence represented by SEQ ID NO:1, which further comprises by deletion, substitution or addition of one to several (1 to 9, preferably 1 to 5, more preferably 1 to 2) amino acids and which has a function substantially identical to that of the protein comprising the amino acid sequence represented by SEQ ID NO: 1.

It should be noted that the expression "having a function substantially identical to that of the protein of the present invention" means to have a similar kind of molecular function or the like as that of the protein of the present invention. Herein, examples of such molecular function include avidity to an interactive molecular and a sugar chain modification function.

### (Sequence of protein)

The sequencing of the protein of the present invention is conducted as follows.

That is, i) presuming a domain region having a function of interest based on known protein sequence information; ii) preparing domain candidate sequence patterns having a basic pattern as the amino acid sequence of the thus presumed domain; iii) expressing a protein of each sequence pattern, evaluating structural stability of the obtained protein forming the domain, using a protein having a good result as a protein of interest.

Accordingly, the protein of the present invention is empirically selected so as to have a stable structure when a part (part that functions as a SEA-like domain) of the full length protein is fragmented and expressed as a protein forming a domain. There exist domain candidates in the order of 100 at the stage of domain region prediction, but the number of domain candidates is narrowed down due to various factors and in fact carefully selected in the order of 10 to several tens. Use of thus selected proteins in three-dimensional structure analysis enables highly accurate and reliable structure analysis, because of their three-dimensional structure stability.

### (Presumption of domain region)

A method for presuming a domain region in the full length protein is not particularly limited, and any of the following methods can be used: for example, information science methods such as bioinformatics or computational science methods (see the specification of Japanese Patent Application No. 2001-309434), combination of deleted DNA library and GFP (see the specification of Japanese Patent Application No. 2001-062703), and experimental methods such as limited breakdown (proteolysis) by protease. By using more accurate methods, the efficiency to select a domain of interest from domain candidates is improved.

### (Production of domain candidate sequence pattern)

The above domain candidate sequence patterns are produced, for example, by extending or shortening the position of domain boundary to the N- or C- terminals on the basis of the above presumed domain region.

For example, prepared is a domain candidate sequence pattern having several new boundaries as the N-terminal, which are provided by extending, for example, one to tens of residues to the N-terminal or shortening, for example, one to tens of residues to the C-terminal, from the position of the amino acid residue in the domain boundary at the N-terminal of the presumed domain region. Note that the number of amino acid residues to be extended or shortened in the N-terminal direction and C-terminal direction is, for example, 1 to 200, preferably 1 to 100, and more preferably, 1 to 50.

Similarly, preferably produced in the same manner as the above N-terminal is a domain candidate sequence pattern having one or several kinds of domain boundaries as the C-terminal, which are selected in the domain boundary at the C-terminal of the presumed domain region.

### (Extending and shortening of domain boundary)

As a method for extending or shortening a domain boundary of the above presumed domain region, employed is, for example, a method for synthesizing individual PCR primers capable of producing cDNAs corresponding to the above domain candidate sequence patterns and performing the creation by PCR. In particular, 2-step PCR method described in Japanese Patent Application No. 2001-201356 is suitable.

### (Extraction of target domain information from domain candidate sequence pattern)

In order to select a target domain protein having actually stable three-dimensional structure from the above domain candidate sequence patterns, protein synthesis is performed using cDNA of domain candidate sequence pattern produced as mentioned above.

An expression system for the domain candidate sequence pattern is not particularly limited, and any of known expression systems are usable.

Next, it is determined whether the obtained protein actually has a stable three-dimensional structure, and when a protein confirmed to have such three-dimensional structure is used as a protein in the present invention.

Examples of indicators for the stability of three-dimensional structure of protein include: biochemical indicators such as an indicator whether a synthesized domain protein is detected as soluble protein by SDS gel electrophoresis, etc. and also detected as uniform band corresponding to a proper molecular weight; and spectroscopic methods having as an indicator fluorescence strength of GFP fused at the C-terminal side, NMR spectrum, and CD spectrum.

The conventional process of determining a protein sequence has problems in that, for example, (1) a target domain protein is not expressed in the above protein synthesis, or (2) though the protein is expressed, it causes aggregation or has low solubility, etc. The present inventors have overcome these problems and completed the present invention.

### (Confirmation of having stable three-dimensional structure)

Regarding the above NMR spectrum, the determination for folding of a protein forming a domain is shown below.

When a protein forming a domain is not folded in 1D spectrum, signals derived from methyl group proton such as Val, Leu, and Ile are observed around 0.8 ppm. However, when a protein is folded, the environment of methyl group proton is changed and signals are shifted to higher magnetic field side (around 0.7 ppm to -0.5 ppm).

The determination in ¹H-¹⁵NHSQC spectrum can be made by visual evaluation of the cross peak convergence degree and the uniformity of signal strength. In other words, when cross peaks are densely gathered, the status is considered not forming a three-dimensional structure. Conversely, when dispersed, the status is considered forming a stable three-dimensional structure. In this way, the stability of three-dimensional structure is evaluated.

### (Expression system)

A protein of the present invention can be produced from a common host cell, etc. including an expression system of the present invention by using a known method. Examples of the expression system include expression systems containing a polynucleotide of the present invention, host cells which contains such expression system and is produced by a known method, expression systems capable of producing a protein of the present invention by using a recombinant technology, and cell-free protein synthesis systems capable of producing a protein of the present invention.

### (Vector)

A recombinant vector of the present invention can be obtained by ligating (inserting) a gene sequence of the present invention into a proper vector. The vector for inserting the gene sequence of the present invention thereinto is not particularly limited as long as it can be replicated in a host cell. Examples thereof include plasmid DNAs and phage DNAs.

Specific examples of the plasmid DNAs include *E. coli*-derived plasmids (e.g. pRSET, pBR322, pBR325, pUC118, pUC119, pUC18, and pUC19), *Bacillus subtilis*-derived plasmids (e.g. pUB110 and pTP5), yeast-derived plasmids (e.g. YEp13, YEp24, and YCp50). Specific examples of the phage DNAs include λ phage (Charon4A, Charon21A, EMBL3, EMBL4, λgt10, λgt11, and λZAP). Further, animal viruses such as retrovirus and vaccinia virus and insect virus vectors such as baculovirus can be used.

To insert a gene of the present invention into a vector, a method is employed, which comprises first cleaving a purified DNA with a proper restriction enzyme and inserting the gene to a restriction enzyme site of a proper vector DNA or a multicloning site to ligate to the vector.

The gene of the present invention is incorporated into a vector so that the gene exhibits its function. Hence, in addition to a promoter and the gene of the present invention, an enhancer or the like including a cis-element, a splicing signal, a poly A addition signal, a selective marker, and a ribosome junction sequence (SD sequence) can be ligated into the vector of the present invention, if desired. Further, examples of the selective markers include a dihydrofolate reductase gene, an ampicillin resistance gene, and a neomycin resistance gene.

### (Transformant)

A transformant of the present invention can be obtained by introducing the recombinant vector of the present invention into a host so that a gene of interest can be expressed. Herein, a host is not particularly limited, as long as it can express DNA of the present invention. Examples thereof include bacterium belonging to genus Escherichia such as Escherichia coli, genus Bacillus such as Bacillus subtilis, genus Pseudomonas such as Pseudomonas putida, genus Rhizobium such as Rhizobium meliloti. Further, yeasts such as Saccharomyces cerevisiae and Schizosaccharomyces pombe, and animal cells such as COS cells and CHO cells can be used. Or insect cells such as Sf9 and Sf21 can be used.

When a bacteria such as E. coli is a host, it is preferable that the recombinant vector of the present invention comprises a promoter, a ribosome junction sequence, a gene of the present invention, and a transcription termination sequence while autonomously replicable in the bacteria. In addition, a gene to control the promoter may be contained.

Examples of E. coli include *E. coli* K12 and DH1 and examples of *Bacillus subtilis* include *Bacillus subtilis.* As a promoter, anyone can be used as long as it can be expressed in a host such as E. coli. Promoters derived from *E. coli* or phages such as trp promoter, lac promoter, λP_{L} promoter, and λP_{R} promoter can be used. Artificially designed and modified promoters such as tac promoter can be used. A method for introducing a recombinant vector into a bacteria is not particularly limited, as long as it is a method for introducing a DNA into bacteria. There are, for example, a method using calcium ion (Cohen, S.N. et al. (1972) Proc. Natl. Acad. Sci., USA 69, 2110-2114) and electroporation method.

When yeast is a host, Saccharomyces cerevisiae, Schizosaccharomyces pombe, and Pichia pastor is, for example, are used. In this case, a promoter is not particularly limited as long as it can be expressed in yeast. Examples thereof include gal1 promoter, gal10 promoter, a heat shock protein promoter, MFα1 promoter, PHO5 promoter, PGK promoter, GAP promoter, ADH promoter, and AOX1 promoter. A method for introducing a recombinant vector into a yeast is not particularly limited as long as the method can introduce a DNA into a yeast. Examples of the methods include electroporation method (Becker, D.M. et al. (1990) Methods. Enzymol., 194,182-187), spheroplast method (Hinnen, A. et al. (1978) Proc. Natl. Acad. Sci., USA 75, 1929-1933), and lithium acetate method (Itoh, H. (1983) J. Bacteriol. 153,163-168).

When an animal cell is a host, a monkey cell COS-7, Vero, Chinese hamster ovary cell (CHO cell), a mouse L cell, a rat GH3, and a human FL cell can be used. As a promoter, SRα promoter, SV40 promoter, LTR promoter, and CMV promoter can be used, and further an early gene promoter of human cytomegalovirus may be used. Examples of the methods for introducing a recombinant vector into an animal cell include electroporation method, calcium phosphate method, and lipofection method.

When an insect cell is a host, an Sf9 cell, an Sf21 cell or the like may be used. As a method for introducing a recombinant vector into an insect cell, calcium phosphate method, lipofection method, and electroporation method may be used, for example.

### (Antibody)

Using the protein of the present invention as an antigen, an antibody against the antigen can be prepared.

### [Production of a polyclonal antibody against the protein of the present invention]

An animal is immunized using the aforementioned antigen. In the case of a rabbit, a dose per animal of an antigen is 100 to 500 µg using, for example, an adjuvant. As the adjuvant, Freund's complete adjuvant (FCA), Freund's incomplete adjuvant (FIA), aluminum hydroxide adjuvant, etc. are used.

Immunization is carried out by administration to mammalians (e.g. non-human mammalians such as a rat, a mouse, and a rabbit). Administration is conducted intravenously, hypodermically, or intraperitoneally. In addition, immunization interval is not particularly limited, and it may be several-day to several-week interval, preferably 2- to 3-week interval. At such interval, an animal is immunized preferably 1 to 10 times, more preferably 2 to 3 times. After 6 to 60 days from final immunization, antibody titer is measured. On a day when the greatest antibody titer is exhibited, blood is collected to obtain antiserum. The antibody titer is measured by ELISA (enzyme-linked immunosorbent assay), RIA (radioimmuno assay), or the like.

When purification of an antibody is needed from antiserum, the purification can be conducted by properly selecting a well-known method such as ammonium sulfate precipitation method, ion exchange chromatography, gel filtration, and affinity chromatography, or combination thereof.

### [Production of a monoclonal antibody against the protein]

An animal is immunized using the aforementioned antigen. If necessary, an adjuvant (commercially available Freund's complete adjuvant, Freund's incomplete adjuvant, etc.) may be mixed in the same manner as above to perform immunization effectively.

Immunization is carried out by administration to mammalians of a human or a non-human (e.g. a rat, a mouse, and a rabbit). A dose per mouse of an antigen is 50 µg. Administration is conducted mainly intravenously, hypodermically, or intraperitoneally. Further, immunization interval is not particularly limited, and it may be several-day to several-week interval, preferably 2- to 3-week interval, and immunization is performed preferably one to ten times, more preferably two to three times. Then, antibody-producing cells are collected after final immunization. As an antibody-producing cell, there are a spleen cell, a lymph node cell, a peripheral blood cell, or the like, but a spleen cell is preferable.

### [Cell fusion]

To obtain a hybridoma, cell fusion of an antibody producing cell and a myeloma cell is performed. As a myeloma cell to be fused with an antibody producing cell, an established cell line can be used, which is generally available and derived from an animal such as a mouse. Preferably used is a cell line which has drug selectivity, and has properties whereby it is unable to survive in HAT selective medium (containing hypoxanthine, aminopterin, and thymidine) without the fusion but able to survive only with the fusion with an antibody producing cell. Specific examples of myeloma cells include mouse myeloma cell lines such as P3X63-Ag.8.U1(P3U1), P3/NSI/1-Ag4-1, and Sp2/0-Ag14.

Next, cell fusion of the above myeloma cell with an antibody producing cell is performed. In an animal cell culture medium such as DMEM and RPMI-1640 medium without inclusion of serum, antibody producing cells and myeloma cells are mixed in the ratio of 15:1 to 25:1. Fusion reaction is performed in the presence of a cell fusion accelerator such as polyethylene glycol, or by electric pulse treatment (e.g. electroporation).

### [Selection and cloning of hybridoma]

From cells treated by cell fusion, a hybridoma of interest is selected. For example, the treated cells are cultured in a medium containing hypoxanthine, aminopterin and thymidine, and growing cells are obtained as a hybridoma.

Next, screening is performed to determine whether an antibody of interest exists in a culture supernatant of increased hybridoma. The screening of hybridoma may be performed, without particular limitation, in a conventional manner. For example, a part of culture supernatant grown as hybridoma in a well is collected, and screened by ELISA (enzyme-linked immunosorbent assay), RIA (radioimmuno assay) or the like. Cloning of fused cells is performed preferably by limiting dilution method or the like, and finally a hybridoma of a monoclonal antibody producing cell is established.

### [Collection of monoclonal antibody]

As a method for collecting monoclonal antibodies from established hybridoma, a conventional cell culture method or the like may be employed. In the cell culture method, preferably the hybridoma is cultured for 3 to 10 days under ordinary culture conditions (e.g. 37°C, 5% CO₂ concentration) in an animal cell culture medium such as RPMI-1640 or MEM media containing 10% bovine fetus serum, and antibodies are collected from resultant culture supernatant.

In the above antibody collection method, antibodies can be purified, if necessary, by properly selecting a well-known method such as ammonium sulfate precipitation method, ion exchange chromatography, affinity chromatography, and gel chromatography, or combination thereof.

### (Production of the protein of the present invention)

The protein of the present invention can be obtained by culturing a transformant and collecting the protein from the culture. The term "culture" means, in addition to a culture supernatant, any of a cultured cell, a cultured fungus body, and a matter of crushed cell or fungus body. "A method for culturing a transformant of the present invention" is performed according to a conventional method used for culturing a host.

As a culture medium for culturing the transformant obtained by using microorganisms such as E. coli and yeast as a host, any of natural medium and synthetic medium may be used as long as it contains carbon source, nitrogen source, mineral, etc. which can be utilized as resource by the microorganisms and it effectively cultures the transformant. As the carbon source, used are carbohydrates such as glucose, fructose, sucrose and starch, organic acids such as acetic acid and propionic acid, and alcohols such as ethanol and propanol. As the nitrogen source, used are ammonium salts of inorganic or organic acids such as ammonia, ammonium chloride, ammonium sulfate, ammonium acetate, and ammonium phosphate, or other nitrogen-containing compounds as well as peptone, meat extract, and corn steep liquor. As inorganic matters, monopotassium phosphate, dipotassium phosphate, magnesium phosphate, magnesium sulfate, sodium chloride, ferrous sulfate, manganese sulfate, copper sulfate, and calcium carbonate may be used.

Culture is carried out preferably at 37°C under aerobic conditions such as shaking culture or aerobic culture with stirring for 6 to 24 hours. During the culture period, pH is kept at 7.0 to 7.5. The pH is adjusted preferably by using inorganic or organic acid, alkali solution, etc. During the culture, antibiotics such as ampicillin and tetracycline may be added to the culture medium if necessary.

When a microorganism is cultured which has transformed with an expression vector having used an inducible promoter as a promoter, an inducer may be added to the medium if necessary. For example, when a microorganism transformed with an expression vector having used lac promoter is cultured, isopropyl-β-D-thiogalactopyranoside (IPTG), etc. may be added to the medium. When a microorganism transformed with an expression vector having used trp promoter is cultured, indole acrylic acid (IAA) may be added to the medium.

As a medium for culturing a transformant obtained by using an animal cell as a host, RPMI1640 or DMEM media, which are commonly used, or these media having bovine fetus serum added thereto may be used. Culture is carried out at 37°C for 1 to 30 days in the presence of 5% CO₂. During the culture period, antibiotics such as kanamycin, penicillin etc. may be added to the medium.

After the culture, protein is extracted preferably by crushing a fungus body or a cell when the protein is produced in a fungus body or a cell. Further, the protein of the present invention is produced outside a fungus body or a cell, the culture medium is used as it is or preferably the fungus body or cell is removed by centrifugation, etc. Thereafter, the protein of the present invention can be isolated and purified from the above culture medium by using either alone or proper combination of biochemical methods commonly used for isolation and purification of protein, such as ammonium sulfate precipitate, gel chromatography, ion exchange chromatography, affinity chromatography, etc. During or after this purification process, the tag sequence, which was used for purification by protease treatment can be removed.

### (Method for producing a domain protein using a cell-free protein synthesis system)

Using a cell-free protein synthesis system, the present invention provides a method for producing: a protein comprising an amino acid sequence represented by SEQ ID NO: 1; and a protein comprising an amino acid sequence derived from the amino acid sequence represented by SEQ ID NO:2 by deletion of 0 to 10 amino acid residues from the N-terminal and deletion of 0 to 10 amino acid residues from the C-terminal and having 120 to 139 amino acid residues.

A cell-free protein synthesis system is a system in which proteins are synthesized in vitro by using a cell extract. "A cell-free protein synthesis system" includes both a cell-free translation system for synthesizing proteins on ribosome through reading of information of mRNA, and a system including both a cell-free transcription system for synthesizing RNA using DNA as the template and a cell-free translation system. Since a cell-free protein synthesis system can modify a system easily, it has an advantage to easily construct an expression system suitable for a target protein. Further, a cell-free protein synthesis system is described in detail in Japanese Patent Publication No. 2000-175695.

### [Cell extract]

A crude cell extract may be an extract from eukaryotic or prokaryotic cell in a state of high protein synthesis activity such as bacteria (e.g. *E. coli),* fungi (e.g. budding yeast), wheat germ, rabbit reticulocyte, murine L-cell, Ehrlich ascetic cancer cell, HeLa cell, and CHO cell (Clemens, M.J., Transcription and translation- a practical approach, (1984), pp. 231-270, Henes, B.D. and Higgins, S.J. eds., IRL Press, Oxford).

A crude cell extract preferably contains a component required for protein synthesis such as ribosome and tRNA. For preparation of a crude extract, a method described, for example, in Pratt, J.M. et al., transcription and translation - a practical approach, (1984), pp. 179-209, Henes, B.D. and Higgins, S.J. eds., IRL Press, Oxford, can be used. More specifically, the preparation can be conducted by crushing with a French press (Pratt, mentioned above) or crushing with glass beads. A preferable cell extract is *E*. *coli* S30 cell extract. S30 extract can be prepared from E. coli BL21 Codon Plus strain in accordance with generally known methods such as a method of Pratt et al. (above mentioned), or S30 extract commercially available from Promega or Novagen can be used. The cell extract derived mainly from *E. coli,* wheat germ, and rabbit reticulocyte.

### (Dialyzer)

A dialyzer which enables shaking or agitating while having internal and external dialysates isolated from each other via a dialysis membrane. Examples of a small-scale reaction apparatus include Dispo Dialyzer (registered trademark) (manufactured by Spectrum) and Slidealyzer (registered trademark) (manufactured by Pierce).

Further, examples of a large-scale reaction apparatus include Spectra/Por (registered trademark) dialysis tube (manufactured by Spectrum).

### (Internal dialysate)

In addition to a concentrated cell extract such as E. coli S30, an internal dialysate of a cell-free protein synthesis system may contain DNA or RNA (mRNA and the like) encoding the target proteins, ATP (adenosine 5'-triphosphate), GTP (guanosine 5'-triphosphate), CTP (cytidine 5'-triphosphate), UTP (uridine 5'-triphosphate), buffer solutions, salts, amino acids, RNase inhibitors, antibacterial agents, RNA polymerase if necessary (in a case where DNA is used as template), and tRNA.

In addition, it can contain ATP regenerating systems such as combinations of phosphoenolpyruvate and pyruvate kinase, or creatine phosphate and creatine kinase, polyethyleneglycol (for example, PEG#8000), 3',5'-cAMP, folic acids, RNase inhibitors, and reducing agents (for example, dithiothreitol). On the other hand, an external dialysate (that is, protein synthesis substrate solution) can use the same composition of the internal dialysate excluding cell extract, RNase inhibitors, DNA or RNA, and RNA polymerase. For example, it may contain buffer solutions, ATP, GTP, CTP, UTP, salts, amino acids, and antibacterial agents. The concentration of added components can be determined arbitrarily.

### [Buffer solution]

As the buffer solution, buffer agent such as Hepes-KOH and Tris-OAc can be used, for example. Examples of the salts include acetates (for example, ammonium salts, magnesium salts, and the like) and glutamate salts. Examples of the antibacterial agents include sodium azide and ampicillin. Examples of the amino acids include 20 kinds of amino acids that construct proteins. In a case where DNA is used as a template, RNA polymerase is added to the reaction system, and a commercially available enzyme such as T7 RNA polymerase can be used.

The internal dialysate is put inside the dialysis membrane, and the external dialysate is put outside the membrane. By shaking or stirring of the closed system in which substances can transfer through the membrane in dependence on the cutoff molecular weight, a target protein thus produced can be collected from the internal or external dialysates. For the reaction conditions such as temperature, stirring rate, and so forth, any condition can be applied depending on the kind of proteins. In the case of protein synthesis, the temperature to be applied is usually approximately 25 to 50°C, preferably 37°C. However, the temperature for cell-free protein synthesis system using a fungus extract derived from *Thermus thermophilus* may exceed 50°C.

Further, the shaking rate or stirring rate may be low, and, for example, 100 to 200 rpm can be applied. While observing the production of the target protein, the reaction period can be properly determined.

In the cell-free protein synthesis system, it is desirable to exchange the external dialysate for a fresh external dialysate when the reaction rate is reduced. Moreover, the use of a dialysis membrane with a cutoff molecular weight of more than 10,000 Da, preferably more than approximately 50,000 Da, enables higher output of the proteins.

### (Purification of protein)

Since the quantity and the number of kinds of mixed contaminants are extremely small, compared with the isolation from living cells, purification of the produced proteins can be achieved with relative ease. Depending on the properties of the proteins, conventionally known purification methods can be used either alone or, if necessary, in combination. Common techniques can be used, such as ammonium sulfate or acetone precipitation, acid extraction, anion or cation exchange chromatography, hydrophobic interaction chromatography, affinity chromatography, gel filtration chromatography, HPLC, electrophoresis, and chromatofocusing. During or after this purification process, a tag sequence used for purification can be removed by protease treatment. Identification and quantitative determination of the produced proteins can be achieved by activity assay, immunological assay, spectroscopic measurement, amino acid analysis, and the like, and, if necessary, comparing with a standard sample.

### (Screening method)

As a screening method of the present invention, there is a method for screening a compound having interaction with a protein of the present invention, which comprises a process of bringing a candidate substance into contact with the protein or a salt thereof, and a process of confirming whether the protein interacts with the candidate substance. Here, the expression "having interaction" means to inhibit or strengthen molecular function and/or physiological activity of the protein by combining the compound with the protein. In this screening method, the protein is brought into contact with the candidate substance, and it is determined whether molecular function and/or physiological activity of the protein is changed.

### [Searching for an interactive substance using NMR]

When NMR is used to search for an interactive substance, the presence of interaction can be determined based on the existence of signal changes of the protein before and after addition of the candidate substance. In other words, when an interactive substance candidate interacts with the protein, it is expected that a chemical shift value, a line width, the number, etc. of NMR signals derived from the vicinity of interactive substance interactive site of the protein may be changed, and thus the presence of interaction can be determined by detecting such changes.

In particular, ¹⁵N-labeled protein is prepared with relative ease, and ¹H-¹⁵N-HSQC spectrum obtained therefrom has relatively high resolution and sensitivity. Further, the spectrum is less affected by NMR signals derived from added interactive substance, and thus it is very useful.

### (Assay method)

The protein of the present invention can be assayed by using, for example, an antibody of the present invention. Examples of a method for assaying a protein using an antibody include sandwich immunoassay, competitive method, immunometric method, and nephelometry method. Further, it is also detectable using labels such as radioisotope, an enzyme, and a fluorescent material.

### (Screening method using assay method)

The antibody of the present invention is specifically combined with the protein of the present invention, and thus it can be used for screening a compound having interaction with the protein of the present invention. As a screening method therefor, known screening methods are usable.

In addition, according to the assay method of the protein of the present invention using the antibody of the present invention, diseases involving the protein of the present invention can be prevented and diagnosed.

### (Three-dimensional structure analysis)

The three-dimensional structure of the protein can be analyzed by NMR structure analysis, X-ray structure analysis, etc.

### (NMR)

A sample used for NMR is not particularly limited, but a sample in which ¹²C or ¹⁴N in the protein is labeled with a stable isotope, ¹³C or ¹⁵N nuclear is used preferably (multi-nuclear and multi-dimensional NMR measurement). Stable-isotope labeling of a protein is a common technique, and is described in Clore, G.M.& Gronenborn, A.M., Science, 252, p.1390-1399, 1991, or the like. In particular, analysis using a protein sample having a main chain labeled with ¹⁵N uniform stable isotope is easily and preferably carried out. In addition, a protein having the skeleton of the main chain labeled with at least two or more kinds of isotopes of ¹³C, ¹⁵N, and ²H may be used (National Publication of International Patent Application No. 2001-514239).

As NMR measurement, it is preferable to measure ¹⁵N-¹H spin coupling constant by observing IPAP-HSQC spectrum, etc. The term "IPAP-HSQC spectrum" is a measurement method for reading ¹⁵N-¹H spin coupling constant effectively by simultaneously observing two HSQC spectra of level and reverse phases, and adding both spectra thereby to prevent overlapping of the signals.

Chemical shift attribution is performed by two or more kinds of NMR methods. For example, 2D, DOQ-COSY, TOCSY, NOESY, and HSQC are well known as two dimensional NMR, and HNCO, HCACO, HNCA, HCA(CO)N, HN(CO)CA, HNHB, CBCANH, H(CA)NH, HBHA(CO)NH, HCCH-COSY, HCANH, HCCH-TOCSY, HCACON, ¹⁵N-NOESY-HSQC, ¹³C-NOESY-HSQC are well known as multi dimensional NMR. A general technique of NMR is known, and described in, for example, "NMR of Protein" (Yoji Arata, Kyoritsu Shuppan, Co., Ltd., 1996); "Basic Biochemical Experiment Method Vol. 3, Protein I, Detection and Structure Analysis Method edited by Japan Biochemical Society" Chapter 18, Three-dimensional structure analysis by NMR (Tokyo Kagaku Dozin, Co., Ltd., Feb. 2001); Takashi Ito et al., Journal Vol. 21 of Japan Agrochemical Society, pp. 450-459, 1996; and Toshiyuki Tanaka, Chemistry and Industry Vol. 49, No. 2, pp. 155-158, 1996.

When NMR is used for three-dimensional structure analysis, it is a common method that the distance between protons is estimated in accordance with the scale of nuclear Overhauser effect between individual protons of the protein, and based on the distance information, the three-dimensional structure is determined. It is possible to obtain a three-dimensional structure with precision by adding information concerning a chemical shift value, a scalar coupling value, a residual dipolar coupling value, hydrogen bond or the like.

Any known program for structure analysis from NMR data is usable. Examples for chemical shift attribution include NMR Pipe, PIPP, Capp, Felix, NMR View, and XEASY. Examples as three dimensional calculation softwares include X-PLOR, CNS, DYANA, and DYNAMO.

### (X-ray crystallography)

When X-ray crystallography is used for three-dimensional structure analysis, an electron density map is calculated based on an X-ray diffraction image of crystallized protein, and the three-dimensional structure is determined. In other words, the protein is crystallized and mono-colored X-ray is applied to the crystal, and based on the obtained X-ray diffraction image, the three-dimensional structure of the protein is clarified (Blundell, T.L. and Johnson, L.N., PROTEIN CRYSTALLOGRAPHY, pp. 1-565, (1976) Academic Press, New York).

### (Screening method based on three-dimensional structure information)

Next, the present invention provides, using information concerning three-dimensional structure of the aforementioned protein, a method for screening a compound having interaction with the protein or a salt thereof, which comprises a process of determining an active site of the protein, and a process of searching for the compound having interaction with the active site on a computer.

### (in silico screening)

Regarding drug design based on three-dimensional structure of a molecule, there are many reviews including Drug Development, Vol. 7 "Molecular Design" (Hirokawa Shoten). Specifically, screening is first conducted by a computer on the library (e.g. about 150,000 kinds) of low molecular compounds (1000 or less molecular weight) stored in a relational database such as Oracle by use of a flexible ligand binding simulation software such as FlexiDock and FlexX. The three-dimensional structure of a chemical compound of this library is designated by a program such as CONCORD, and it is possible to select a substance that can be inserted into an active site. Among the selected substances, a compound that is fit into the active site more precisely is visually selected by using a simulation program such as Insight II or MOE.

As computer softwares used in a series of the above processes, any commercially available one, such as FlexiDock, Tripos Inc.FlexX, Tripos Inc.CONCORD, Tripos Inc.Oracle, Oracle Corp.Insight II, Molecular Simulations Inc.MOE, Chemical Computing Group Inc. can be used.

Another method is to design candidate compounds including unknown substances by a computer. As such method, the following methods are known: a method for searching for a compatible compound by aligning a chemical group such as methyl and ethyl in an active site; and a method of aligning an atom in an active site by a computer program.

### (Wet screening)

To select a major candidate compound having interaction with the protein of the present invention, the candidate compound obtained by in silico screening is brought into contact with the protein of the present invention and the molecular function or physiological activity of the protein of the invention is determined. Further, based on the three-dimensional structure data of the candidate compound and the protein of the invention, the candidate compound is modified so as to have more desirable structure.

The selected compound is synthesized and actually interacted with the protein for screening. With respect to a compound that has changed the activity of the protein, further testing relating to in vitro activity, in vivo dynamics, or toxicity is performed by animal tests.

### (Drug containing an interactive substance)

A substance interacting with the protein of the invention can be used as a preventive and/or therapeutic agent to diseases involving the protein. Such drug can be orally or parentally administered to the whole body or locally.

When the drug of the invention is orally administered, it may be prepared in any type of formulation such as a tablet, a capsule, a granule, powder, a pill, trochiscus, internal use liquor, a suspension, an emulsion, and syrup, and may be prepared as a dried product which is dissolved again at administration. Further, when the drug of the invention is parenterally administered, a formulation such as an intravenous injection (including intravenous drip), intramuscular injection, intraperitoneal injection, subcutaneous injection, and suppositories may be selected. In the case of formulations for injection, the drug may be provided in the form of an ample with a unit dose or a container for large volume administration.

These formulations can be produced by conventional methods by properly selecting an excipient, an extender, a binder, a wetting agent, a disintegrator, a lubricant, a surfactant, a dispersant, a buffer, a preservative, a solubilizing agent, an antiseptic, a corrective, an analgesic agent, a stabilizing agent, and an isotonic agent, which are commonly used for formulation.

The above various formulations may contain a pharmaceutically acceptable carrier or additive. Examples of these carriers and additives include water, pharmaceutically acceptable organic solvents, collagen, polyvinyl alcohol, polyvinyl pyrrolidone, carboxyvinyl polymer, sodium alginate, water-soluble dextran, carboxymethyl starch sodium, pectin, xanthan gum, gum Arabic, casein, gelatin, agar, glycerol, propylene glycol, polyethylene glycol, Vaseline, paraffin, stearyl alcohol, stearic acid, a human serum albumin, mannitol, sorbitol, and lactose. Additives to be used are selected from the above-mentioned property or in combination according to the type of formulation of the invention.

A dose of the drug of the invention can be varied depending on the age of a recipient, administration path, and the number of administration times, and thus it can be changed in a wide range. In this case, the effective dose of the protein of the invention and the effective dose to be administered in combination with suitable diluent and pharmacologically usable carried are selected in the range of 0.01 mg to 1,000 mg per 1 kg of the body weight for one time, and the administration is conducted preferably once to several times per day for one day or more.

### (Description of Sequences)

The sequence numbers of the description indicate the following sequences.

### [Sequence listing free text]

[SEQ ID NO: 1] represents an amino acid sequence of amino acid residue 133 to 251 of 1110008I14 protein derived from a mouse.
[SEQ ID NO: 2] represents an amino acid sequence of amino acid residue 123 to 261 of 1110008I14 protein derived from a mouse.
[SEQ ID NO: 3] represents a cDNA sequence encoding the protein represented by SEQ ID NO: 1.
[SEQ ID NO: 4] represents a cDNA sequence encoding the protein represented by SEQ ID NO: 2.
[SEQ ID NO: 5] represents an amino acid sequence represented by SEQ ID NO: 1 plus an amino acid sequence of linkers added to the N-terminal and C-terminal thereof.
[SEQ ID NO: 6] represents the nucleotide sequence of 5' primer 1 used in the primary PCR employed in an expression vector construction process in synthesizing a protein having an amino acid sequence represented by SEQ ID NO: 1.
[SEQ ID NO: 7] represents the nucleotide sequence of 5' primer 1 used in the primary PCR employed in an expression vector construction process in synthesizing a protein having an amino acid sequence represented by SEQ ID NO: 2.
[SEQ ID NO: 8] represents the nucleotide sequence of 3' primer 1 used in the primary PCR employed in synthesizing a protein having an amino acid sequence represented by SEQ ID NO: 1.
[SEQ ID NO: 9] represents the nucleotide sequence of 3' primer 1 used in the primary PCR employed in synthesizing a protein having an amino acid sequence represented by SEQ ID NO: 2.
[SEQ ID NO: 10] represents the nucleotide sequence of 5' primer 2 used in the secondary PCR employed in the expression vector construction process above.
[SEQ ID NO: 11] represents the nucleotide sequence of 3' primer 2 used in the secondary PCR.
[SEQ ID NO: 12] represents a nucleotide sequence of universal primer U2 used in the secondary PCR.

### [Examples]

The present invention will be described in detail by showing Examples below, but the scope of the present invention is not limited by them.

### I) Presumption of domain

The presumption of a domain was conducted in the following manner.

First, when a region having homology with a sequence contained in protein database SCOP (Version 1.55) was detected from query sequences, such region was predicted as a domain. BLASTP was used as homology detection method, and it was determined to be homologous when E-value had 0.1 or less hits.

### <SCOP method>

Next, when a region having homology with a sequence profile contained in protein motif database PFAM (version 6.5) was detected from query sequences, such region was predicted as a domain. HMMER was used as homology detection method, and it was determined to be homologous when E-value had 0.1 or less hits.

### <PFAM method>

When a region having homology with any of consensus sequences contained in protein motif database ProDom (a version obtained through the Web January, 2001) was detected from query sequences, such region was predicted as a domain. BLASTP was used as homology detection method, and it was determined to be homologous when E-value had 0.1 or less hits. <ProDom method>

Further, homology search on query sequences was conducted by BLASTP in protein sequence data set (NCBI-nr), and when E-value had 0.1 or less hits, such homologous regions were grouped and predicted as domain. <NR method>

Moreover, homology search on query sequences was conducted by BLASTP in protein sequence data set (NCBI-nr), and the frequency of detecting homology was calculated. Peaks and troughs were used to indicate high frequency parts and low frequency parts, and one peak was predicted as domain so that a trough part is a domain boundary. <PASS method>

Regions (residual regions) that were not detected as domain by using any of these five methods were predicted as domain. <No Hit method>

Regarding the above six domain prediction methods, when a hit region was overlapped, high priority was given to SCOP method, and followed by PFAM method, ProDom method, NR method, PASS method, and No Hit method in this order. When the deviation of the domain boundary includes 30 residues or more and such residues are present in more length in the N- or C-terminal side in accordance with definition by lower priority method, though a hit region was overlapped, such differential sequence was predicted as other domain. <Differential domain boundary setting method>

At this time, among domain regions presumed by any of the above methods, regions having one or more Low-Complexity regions (sequence region with low complexity) or having the full length of less than 30 residues were removed.

In accordance with such a procedure, a domain of 1110008I14 protein used in this Example was presumed by a PFAM method. As a result, it was presumed that a region from amino acid residue 133 to 261 is a domain region. Note that this region is devoid of a Low-Complexity region (a sequence region low in complexity) and has a length of not less than 30 residues in total. As a result of such a final bio informatics, the region is presumed as the domain.

Based on the data of the bio informatics, various studies were carried out. As a result, it was found that the region from amino acid residue 133 to 251 of 1110008I14 protein actually constitutes the SEA-like domain.

### II. Preparation of construct and synthesis of domain protein

A construct was prepared systematically by extending or shortening the N and C-terminals of the putative domain region of the amino acid sequence of 1110008I14 protein, by several residues relative to the amino acid sequence of the aforementioned putative domain region.

More specifically, the domain border is shifted by 10 residues from amino acid residue 133 of 1110008I14 protein, to obtain 4 patterns of domain constructs starting from amino acid residue 113, 123, 133 and 143. On the other hand, the domain border is shifted by 10 residues from amino acid residue 261 of 1110008I14 protein, to obtain 4 patterns of domain constructs starting from amino acid residue 251, 261, 271 and 281. In combination of these, 16 sequence patterns were prepared in total.

Subsequently, all 16-pattern constructs are expressed and expression states were evaluated by SDS gel electrophoresis.

### (Example 1)

In this example, among constructs described above, 1110008I14 protein (133-251) comprising a SEA like domain suitable for three-dimensional structure analysis was studied. Results of the study are described.

### <Construction of expression vector>

### (1) First PCR

Using a recombinant *E. coli* culture solution containing a plasmid wherein cDNA (DDBJ Accession No. AK003577.1, home page of RIKEN GENOMIC SCIENCES CENTER: http://wwwgenome.gsc.riken.go.jp/, FANTOM DB clone ID. 1110008I14) encoding 1110008I14 protein was cloned in a plasmid pBluescriptII SK+, PCR was performed by use of 5'-primer 1 (SEQ ID NO: 6) and 3'-primer 1 (SEQ ID N0:8). The composition of the PCR reaction solution and the program are shown in Tables 1 and 2, respectively.

**[Table 1]**

| Composition of reaction solution for first PCR | | | |
|---|---|---|---|
| Composition | Concentration | Amount added | Final concentration |
| Template plasmid | (×1/10) | 3 µL | (×3/200) |
| 5'-primer 1 | 0.25 µM | 4 µL | 0.05 µM |
| 3'-primer 1 | 0.25 µM | 4 µL | 0.05 µM |
| dNTPs(Toyobo) | 2 mM | 2 µL | 0.2 mM |
| Expand HiFi buffer solution (containing 15 mM magnesium chloride) (Roche) | (10×) | 2 µL | (1×) |
| Sterile distilled water | | 4.85 µL | |
| DNA polymerase (Roche) | 3.5 U/µL | 0.15 µL | 0.02625 U/µL |
| Total amount | | 20 µL | |

**[Table 2]**

| Progam for first PCR | | |
|---|---|---|
| STEP1 | 94°C | 30 sec |
| STEP2 | 60°C | 30 sec |
| STEP3 | 72°C | 2 min |
| STEP4 | GOTO 1 for 9 times | |
| STEP5 | 94°C | 30 sec |
| STEP6 | 60°C | 30 sec |
| STEP7 | 72°C | 2 min+ sec/cycle |
| STEP8 | GOTO 5 for 19 times | |
| STEP9 | 72°C | 7 min |
| STEP10 | 4°C | |

### (2) Secondary PCR

Next, second PCR was performed using the first PCR product obtained in the foregoing reaction, 5'-primer 2 (SEQ ID NO:10) having His tag sequence in the downstream of T7 promoter sequence, 3'-primer 2 (SEQ ID NO:11) having a T7 terminator sequence, and universal primer-U2 (SEQ ID NO:12). The program was the same as the above first PCR. The composition of the PCR reaction solution is shown in Table 3.

**[Table 3]**

| Composition of reaction solution for second PCR | | | |
|---|---|---|---|
| Composition | Concentration | Amount added | Final concentration |
| First PCR product (template) | (×1/5) | 5 µL | (× 1/20) |
| 5'-primer 2 | 2 µM | 0.5 µL | 0.05 µM |
| 3'-primer 2 | 2 µM | 0.5 µL | 0.05 µM |
| Universal primer U2 | 100 µm | 0.2 µL | 1 µL |
| dNTPs (Toyobo) | 2 mM | 2 µL | 0.2 mM |
| Expand HiFi buffer solution (containing 15 mM magnesium chloride) (Roche) | (10×) | 2 µL | (1×) |
| Sterile distilled water | | 9.65 µL | |
| DNA polymerase (Roche) | 3.5 U/µL | 0.15 µL | 0.02625 U/µL |
| Total amount | | 20 µL | |

As a result, a linear double stranded DNA fragment was amplified, which can express a fusion protein of His tag sequence and 1110008I14 protein (133-251) under the control of T7 promoter.

### (3) Cloning

The DNA fragment obtained by the above second PCR reaction was cloned in a vector pPCR2.1 (Invtrogen) with TOPO TA-cloning kit (Invtrogen), and thereby an expression vector P011109-16 was constructed.

### <Synthesis of ¹⁵N-labeled SEA-like domain by cell-free protein synthesis reaction using dialysis>

E. coli S30 extract was prepared from *E. coli* BL21 codon plus strain according to a method of Zubay et al. (Annu. Rev. Geneti. 7, 267-287, 1973).

A cell-free protein synthesis reaction using dialysis was performed by using 3 ml of a reaction solution, 30 ml of external dialysis fluid at 30°C overnight. Formulas of the reaction solution and the external dialysis fluid are shown in Table 4 and Table 5, respectively.

**[Table 4]**

| Composition of reaction solution | |
|---|---|
| Composition | Final concentration |
| Hepes-KOH (pH 7.5) | 58 mM |
| DTT | 1.8 mM |
| ATP | 1.2 mM |
| CTP | 0.8 mM |
| GTP | 0.8 mM |
| UTP | 0.8 mM |
| Creatine phosphate | 80 mM |
| Creatine kinase | 0.25 mg/mL |
| Polyethylene glycol (average molecular weight 8000) | 4.0% |
| 3',5'-cAMP | 0.64 mM |
| L(-)-5-formyl-5,6,7,8-tetrahydroforic acid | 68 µM |
| *E. coli* total tRNA | 175 µg/mL |
| Potassium glutamate | 210 mM |
| Ammonium acetate | 27.5 mM |
| Magnesium acetate | 10.7 mM |
| [¹⁵N] labeled amino acid mixture | 3 mg/mL |
| L-[¹⁵N] cystein | 1 mM |
| L-[¹⁵N] tryptophan | 1 mM |
| L-[¹⁵N] glutamine | 1 mM |
| L-[¹⁵N] asparagine | 1 mM |
| Sodium azide | 0.05% |
| T7 RNA polymerase | 66.6 µg/mL |
| S30 extract | 30% |
| Template DNA (P011109-16) | 1 µg/mL |

**[Table 5]**

| Composition of external dialysate | |
|---|---|
| Composition | Final concentration |
| Hepes-KOH (pH 7.5) | 58 mM |
| DTT | 1.8 mM |
| ATP | 1.2 mM |
| CTP | 0.8 mM |
| GTP | 0.8 mM |
| UTP | 0.8 mM |
| Creatine phosphate | 80 mM |
| Creatine kinase | 0.25 mg/mL |
| Polyethylene glycol (average molecular weight 8000) | 4.0% |
| 3',5'-cAMP | 0.64 mM |
| L(-)-5-formyl-5,6,7,8-tetrahydroforic acid | 68 µM |
| Potassium glutamate | 210 mM |
| Ammonium acetate | 27.5 mM |
| Magnesium acetate | 10.7 mM |
| [¹⁵N]-labeled amino acid mixture | 3 mg/mL |
| L-[¹⁵N] cysteine | 1 mM |
| L-[¹⁵N] tryptophan | 1 mM |
| L-[¹⁵N] glutamine | 1 mM |
| L-[¹⁵N] asparagine | 1 mM |
| Sodium azide | 0.05% |

### <Determination of expression status by SDS gel electrophoresis>

After the termination of the synthesis reaction, SDS gel electrophoresis was performed by a conventional method, and the expression status of the obtained protein was determined.

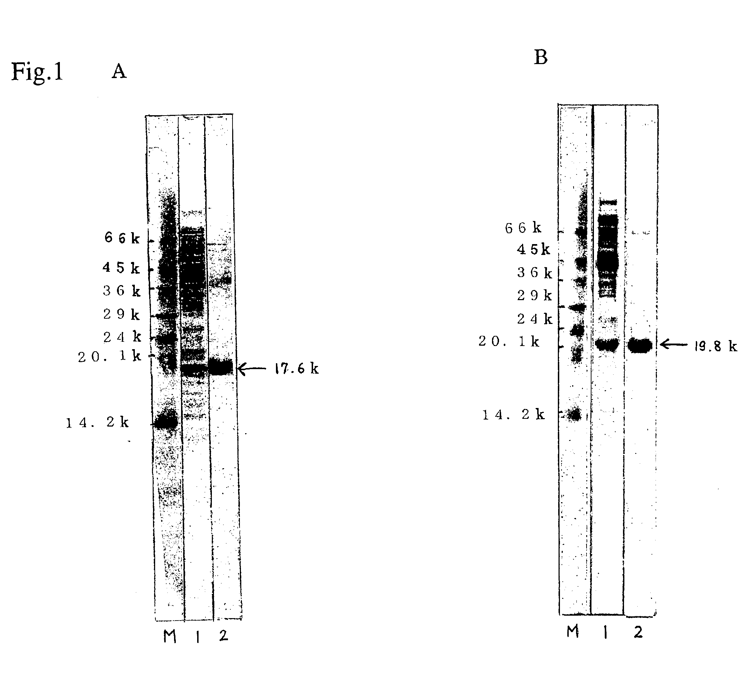

Results thereof are shown in Fig. 1A. According to Fig. 1A, it was confirmed that 1110008I14 protein (133-251) was expressed. An amino acid sequence that forming the region of a SEA-like domain of this 1110008I14 protein (133-251) is represented by SEQ ID NO: 1.

### (Examples 2)

5'-primer 1 and 3'-primer 1 used in the first PCR in Example 1 were as shown by SEQ ID NO:7 and SQ ID NO:9, respectively, and except that, an expression vector was constructed in the same manner as Example 1. The obtained linear double stranded DNA fragment was used as a template DNA for protein synthesis.

After completion of the synthesis reaction, the expression status of the protein was checked by SDS gel electrophoresis in the same manner as mentioned above.

The results are shown in Figure 1B. As is confirmed from Figure 1B, 1110008I14 protein (123-261) was expressed. The amino acid sequence of the SEA-like domain region in Example 2 is represented by SEQ ID NO: 2.

### [Comparative Examples 1 to 5]

Among constructs produced in the above manner, expression vectors were constructed as in Example 1 regarding polypeptide a (comparative example 1) having an amino acid sequence of 113th to 281st amino acid residues of 1110008I14 protein, polypeptide b (comparative example 2) having an amino acid sequence of 133rd to 281st amino acid residues, polypeptide c (comparative example 3) having an amino acid sequence of 143rd to 251st amino acid residues, polypeptide d (comparative example 4) having an amino acid sequence of 143rd to 261st amino acid residues, and polypeptide e (comparative example 5) having an amino acid sequence of 143rd to 271st amino acid residues. The obtained linear double stranded DNA fragments were used as template DNAs for protein synthesis. At this time, instead of primers used for the first PCR in Example 1, primers having respective DNA sequences properly designed for these comparative examples were used.

Then, the expression statuses of the proteins were determined by SDS gel electrophoresis as described above.

Results thereof are shown in Fig. 2. In comparative examples 1 and 2, Fig. 2 clearly shows that there appeared almost no bands at position corresponding to the molecular weights MWa and MWb (MWa=23.1 kDa, MWb=20.9 kDa) obtained from the amino acid sequences of respective polypeptides a and b, and it was confirmed that proteins of interest were not expressed.

Further, in comparative examples 3 to 5, bands were not able to be found in supernatants at positions corresponding to the molecular weights MWc, MWd, and MWe that were calculated from amino acid sequences of respective polypeptides c, d, and e (MWc = 16.5 kDa, MWd = 17.6 kDa, MWe = 18.7 kDa, respectively), and thus it was obvious that a soluble protein was not obtained.

### III. Structural stability evaluation

### <Purification of ¹⁵N labeled SEA-like domain>

Subsequently, the proteins of interest in the above Examples 1 and 2 that were determined to be in a good expression status were purified.

To purify ¹⁵N labeled protein domain, the affinity of histidine tag and nickel was utilized. The operation was conducted at 4°C. First, after the termination of synthesis reaction, 3 ml of the reaction solution was diluted with 4.2 ml of washing buffer solution [50 mM sodium phosphate (pH 8.0)/300 mM sodium chloride/10 mM imidazole], collected and centrifuged at 1960 g for 5 minutes to remove precipitates. Next, the obtained supernatant was passed through 0.8 ml of Ni-NTA resin (QIAGEN) for adsorption, and passed through 9.6 ml of washing buffer solution to thereby remove contaminants. Finally, the resultant product was passed through 4 ml of elution buffer solution [50 mM sodium phosphate (pH 8.0)/300 mM sodium chloride/500 mM imidazole], and thereby the sample was liberated from the resin. According to the above procedures, 0.88 mg of purified sample was obtained.

### <Sample preparation for structural stability evaluation>

To make the purified sample a solvent suitable for NMR measurement, substitution by 20 mM sodium phosphate (pH 6.0)/100 mM sodium chloride solution was conducted. Thereafter, the sample was concentrated to 0.25 ml (sample concentration: 0.28 mM). For the above operations, an ultrafilter (VIVASPIN 2; SARTORIUS) was used. Finally, 0.03 ml of heavy water was added, thereby obtaining a sample for structural stability evaluation.

### <Three-dimensional structural stability determination by NMR measurement>

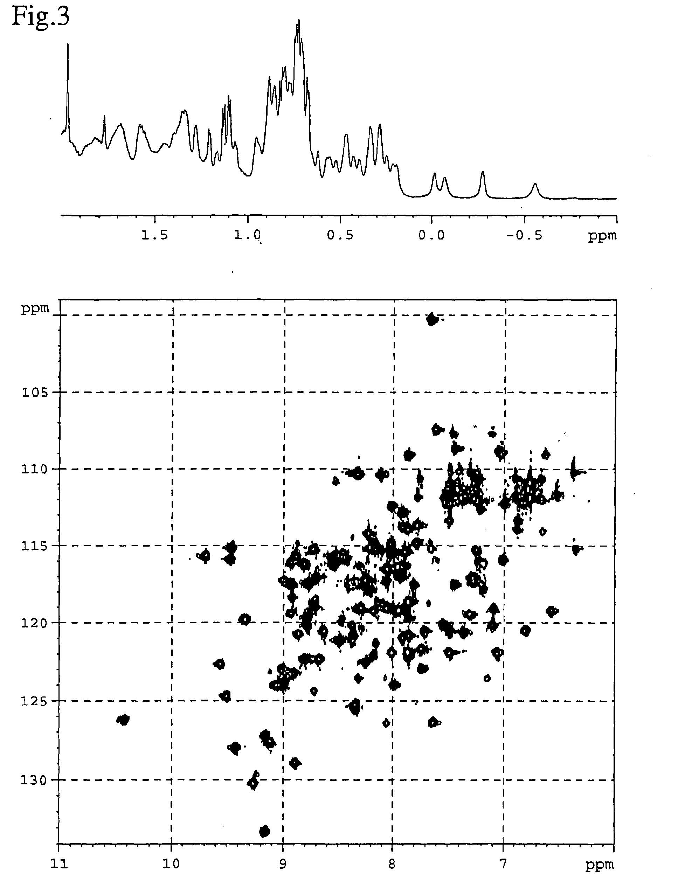

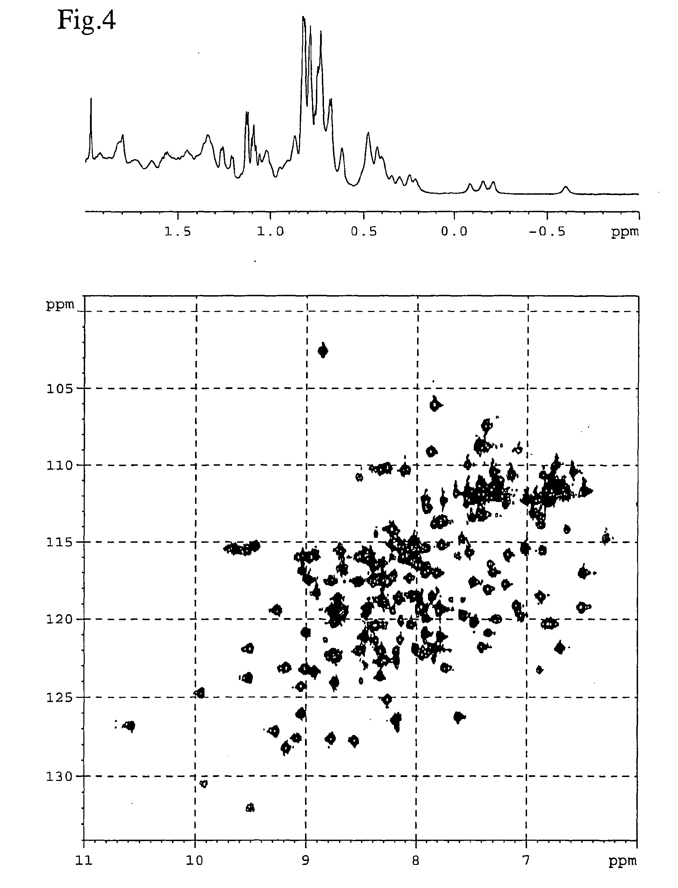

As a sample tube for NMR measurement, a symmetrical microtube (for 5 mm probe) manufactured by Shigemi, Inc. was used. The NMR measurement was conducted by a 600 MHz-NMR instrument (Avance 600 manufactured by Bruker) at 25°C. For the evaluation, one-dimensional spectrum of ¹H (hereinafter abbreviated as 1D spectrum), and ¹H-¹⁵N two-dimensional HSQC spectrum (hereinafter abbreviated as ¹⁵N-HSQC spectrum) were used, and the conditions therefor were shown in Table 6. Results of Examples 1 and 2 are shown in Figs. 3 and 4, respectively.

**[Table 6]**

| NMR measurement conditions | | | | |
|---|---|---|---|---|
| Spectrum | Accumulated times | Center frequency | Spectrum width | Data point number |
| 1D | 128 | ¹H: 2822 Hz | ¹H: 8013 Hz | ¹H: 8192 |
| ¹⁵N HSQC | 16 | ¹H: 2822 Hz ¹⁵N: 7085 Hz | ¹H: 8013 Hz ¹⁵N: 2190 Hz | ¹H:2048 ¹⁵N: 128 |

According to the results of NMR measurement in Figs. 3 and 4, signals shifted to higher magnetic filed were recognized at a high magnetic field side (around 0.7 ppm to -0.5 ppm) of methyl region of 1D spectrum. Further, signals of removed amide proton were recognized in the region 7 ppm to 9 ppm in ¹⁵N-HSQC spectrum.

Since the appearance of these signals is characteristic to a protein forming a stable three-dimensional structure, it was determined that the resultant respective 1110008I14 protein (133-251)(Example 1) and 1110008I14 protein (123-261)(Example 2) formed a stable three-dimensional structure.

As mentioned above, we presumed a structural element (domain) having a structure and function of a protein from the full-length protein by a computer and prepared various types of constructs by extending or shortening the domain boundary toward the N- or C-terminals from the above putative domain region. Subsequently, the protein is expressed and the state of expression of the protein actually obtained was checked by SDS gel electrophoresis. Furthermore, whether each domain has a structure or not was checked by NMR such as HSQC spectrum. As a result, it was confirmed herein that the position of a domain having a folding structure in the amino acid sequence of a protein is accurately determined.Moreover, use of such a protein having a small molecular weight enables three-dimensional structure analysis with ease and accuracy, which is considered to be difficult for a full-length protein.

### IV. Determination of three-dimensional structure of SEA-like domain by NMR method

A SEA-like domain having a stable three-dimensional structure obtained in the manner described above was subjected to the three-dimensional structure analysis by ¹³C¹⁵N multi-nuclear and multi-dimensional NMR method.

### <Preparation of sample for ¹³C¹⁵N three-dimensional analysis>.

A protein represented by SEQ ID NO: 5 in which all carbon nuclei and nitrogen nuclei were completely replaced with stable isotope ¹³C and ¹⁵N, respectively, was prepared in the same manner as in preparation of the ¹⁵N sample by use of the cell-free protein synthesis system. The protein represented by SEQ ID NO: 5 used herein was prepared by adding the amino acid sequences of linkers for cloning respectively to the N-terminal and C-terminal of a protein represented by SEQ ID NO: 1. Even if these linker amino acid sequences are added, the three dimensional structure would not be affected. Therefore, it can be considered that the three dimensional structure of the protein represented by SEQ ID NO: 1 is automatically and substantially analyzed by analyzing the three-dimensional structure of the protein represented by SEQ ID NO: 5.

The obtained high purity preparation was concentrated to a concentration of about 1.5 mmol/l using a protein concentrator with an ultrafilter membrane for a high-speed centrifuge, and thereafter diluted 10 times with preparation buffer solution for NMR analysis. Then, the preparation was concentrated back to the former concentration in the same manner.

These concentration and dilution processes were repeated three times, and the buffer solution for purifying preparation was completely substituted by the preparation buffer solution for NMR analysis. The used preparation buffer solution for NMR analysis was composed of 20 mmol/l sodium phosphate, 100 mmol/l sodium chloride, 1 mmol/l dithiothreitol, 0.02% sodium azide and pH thereof was 6.0. After complete substitution by the preparation buffer solution for NMR analysis, the final concentration of the preparation was about 1.5 mmol/l.

The obtained preparation was put to a test tube for NMR measurement with an outer diameter of 5 mm, and then preserved at 25°C for 2 hours for stabilization.

### <Two-dimensional and three-dimensional NMR measurement>

For NMR experiment, DRX600 and DRX800 manufactured by Swiss Bruker were used. All the measurements were conducted at 25° C.

In NMR measurement having a purpose of main chain signal attribution, a two dimensional spectrum of ¹H-¹⁵N HSQC, and three dimensional spectrum of HNCO, HN(CA)CO, HNCACB, CBCA(CO)NH, H(CCCO)NH, C(CCCO)NH, and ¹⁵N-edited NOESY were measured.

In addition, in NMR measurement having a purpose of side chain signal attribution, two dimensional spectrum of ¹H-¹³C HSQC TOCXY, NOESY, DQF-COSY and three dimensional spectra: HCCH-TOCSY, ¹³C-edited NOESY for aliphatic side chain; and ¹³C-edited NOESY for aromatic side chain were measured.

### <Analysis of measurement data>

The measurement data were subjected to Fourier transform using work stations Octane2 and Origin3800 manufactured by Silicon Graphics, Inc. in America, and respective two dimensional and three dimensional spectra were obtained.

Based on the obtained spectrum data, Cα and Cβ of carbon nucleus at α and β positions as main chain signals of amino acid residue; C' of carbon nucleus of carbonyl group; Hα and Hβ of hydrogen nucleus at α and β positions; HN of hydrogen nucleus of amino group; and N of nitrogen nucleus of amide group were attributed by a chain attribution method. In this method, a signal having a chemical shift value identical to that of Cα signal of an adjacent residue on HN(CO)Ca was searched for on HNCA, and the linkage with Cα signal of a residue adjacent to itself was clarified.

In the chemical shift attribution method used herein, signals corresponding to chemical shift values of Cα and Cβ signals of adjacent residues on CBCA(CO)NH are detected on HNCACB, and the continuity of Cα and Cβ signals between itself and the adjacent residues is clarified.

This process was repeated, and thereby all Cα and Cβ signals were continuously attributable, except that signals were not observed due to proline residue or from any cause.

By conducting the same procedure, C' signal was attributed by HNCO and HN(CA)CO. Further, using the obtained spectrum data measured for main chain attribution information and side chain attribution, attributions of carbon, nitrogen, and hydrogen nuclei at γ and after, namely, γ, δ, ε, ζ, and η positions were conducted. According to the above procedures, attribution data of signals regarding to almost all the amino acid residues was obtained. Furthermore, distance restriction data was obtained from NOE peaks found on ¹⁵N-edited NOESY spectrum, ¹³C-edited NOESY spectrum for an aliphatic side chain, and ¹³C-edited NOESY spectrum for aromatic side chain. From the chemical shift values, obtained during main chain attribution, of Cα, Cβ, C', Hα, Hβ, HN, and N signals, φ and ψ angle data were obtained using a software TALOS, which predicted with high precision φ and ψ angles, dihedral angles of polypeptide main chain. Based on these signal attribution data, distance limitation data, φ, ψ angle data, NOE attribution and a domain structure were calculated using ARIA-CNS, a software for NOE complete automatic attribution and protein three-dimensional structure calculation. For the calculation, 2072 NOE from ¹³C-edited NOESY and 1493 NOE from ¹⁵N-edited NOESY. Based on the obtained three-dimensional structure, NOE group which did not meet provided distance limit was compared and reviewed, and then optimized.

This process was repeated, and finally calculation was conducted using all the angle limits and 2277 distance limits, thereby obtaining 20 energetically stable three-dimensional structures. In these structures, the convergence of amino acid residues forming two dimensional structures was 0.450 Å relative to atom group of main chains and 0.920 Å relative to all atom group including side chains except hydrogen atom.

The obtained three-dimensional structure coordinates are shown in the following three-dimensional structure coordinate tables 1 to 20.

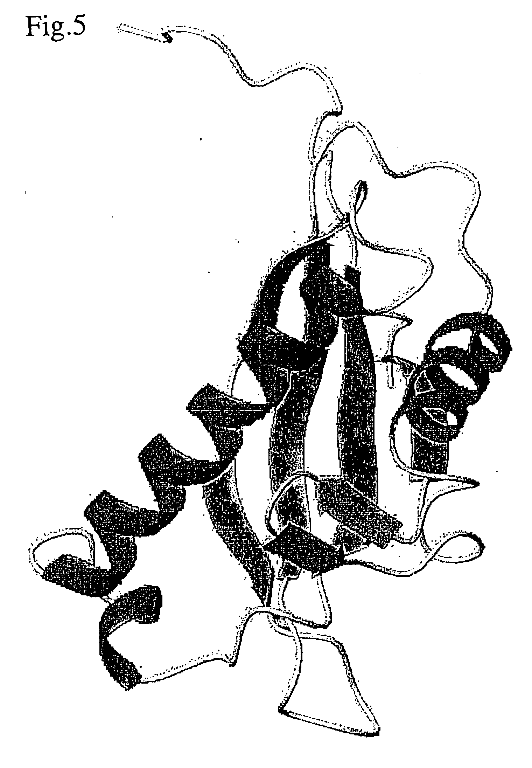

The following three-dimensional structure coordinate is described in accordance with the format of protein data bank (PDB). ATOM of the first column indicates that this column is a column for atomic coordinate; the second column indicates the order of atoms; the third column indicates the distinction of atom in amino acid residues, etc.; the fourth column indicates amino acid residues, etc.; the fifth column indicates the number or the like of amino acid corresponding SEQ ID NO: 5; the sixth, seventh, and eighth columns indicate coordinates of atom (unit: Å, in the order of a, b and c axes); the ninth column indicates occupancy of that atom (anytime this figure is 1.00 in the invention); the tenth column indicates temperature factor of that atom. The final line indicates the final line of this table. Further, Fig. 5 shows a ribbon diagram of a three-dimensional structure shown in the three-dimensional structure coordinate 1 that is the most stable structure.

### Three-Dimensional Structure Coordinate 1

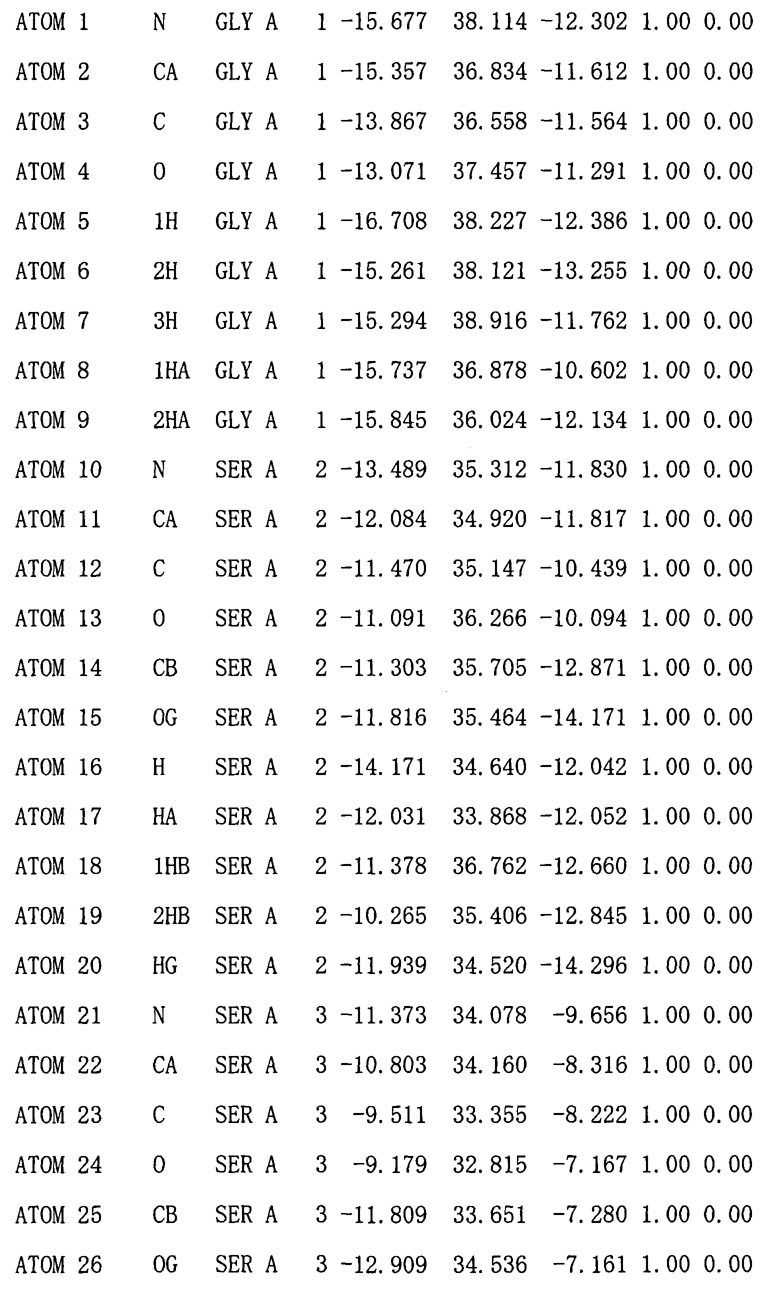

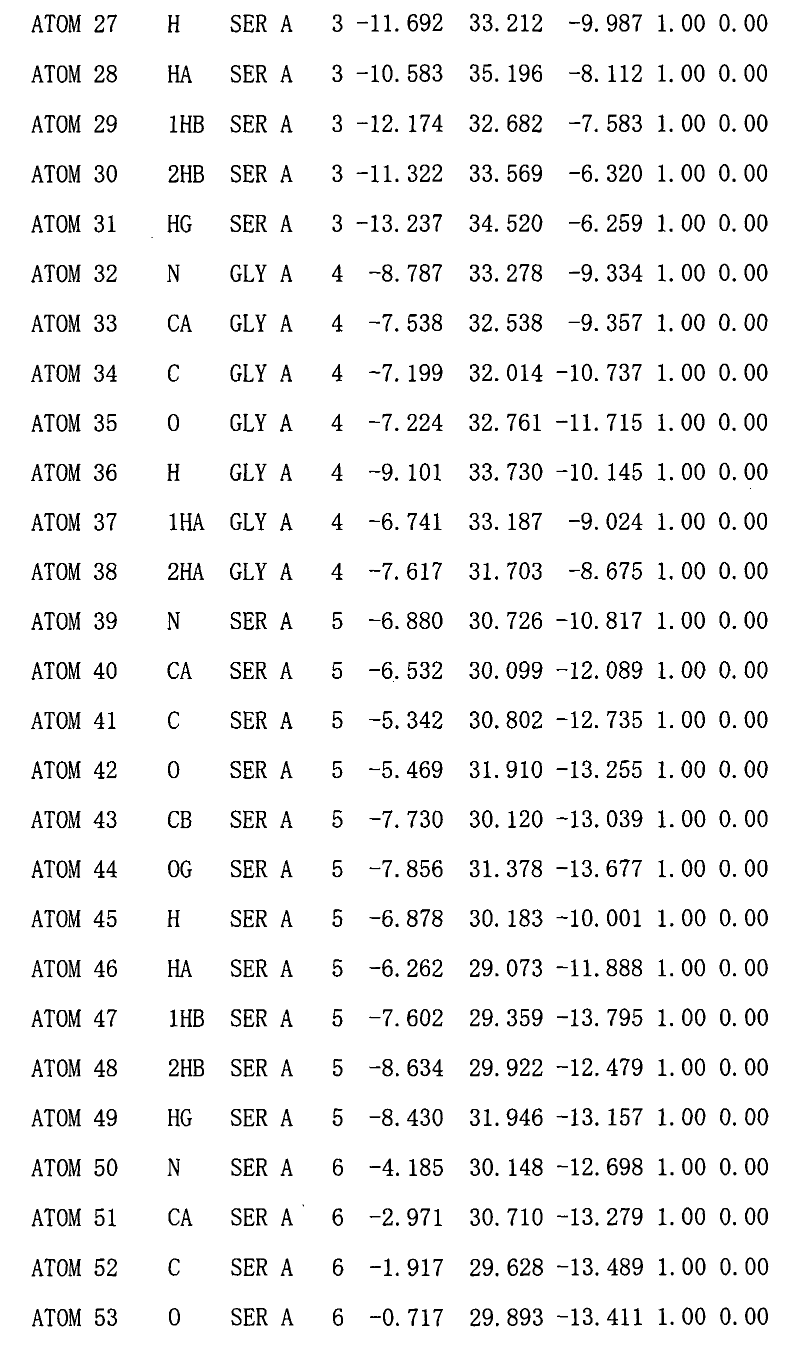

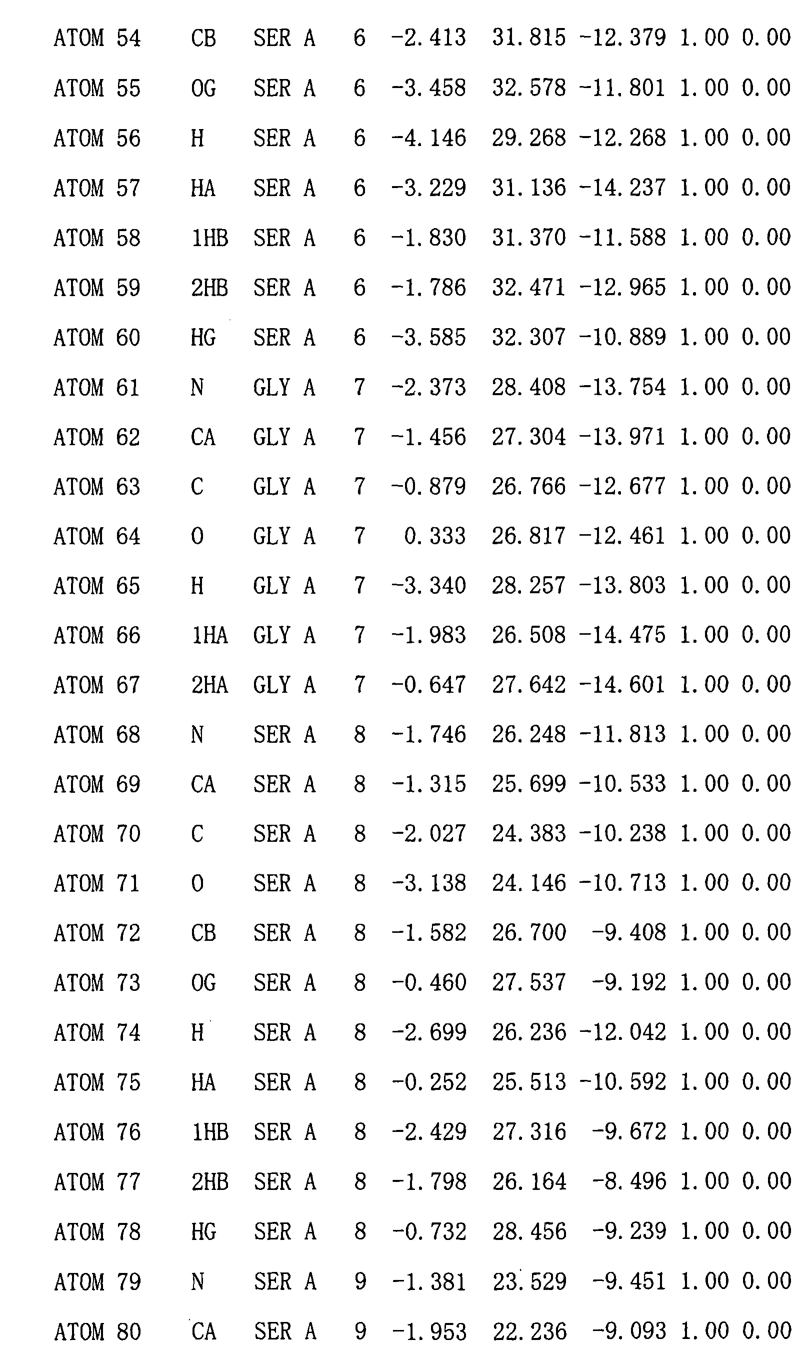

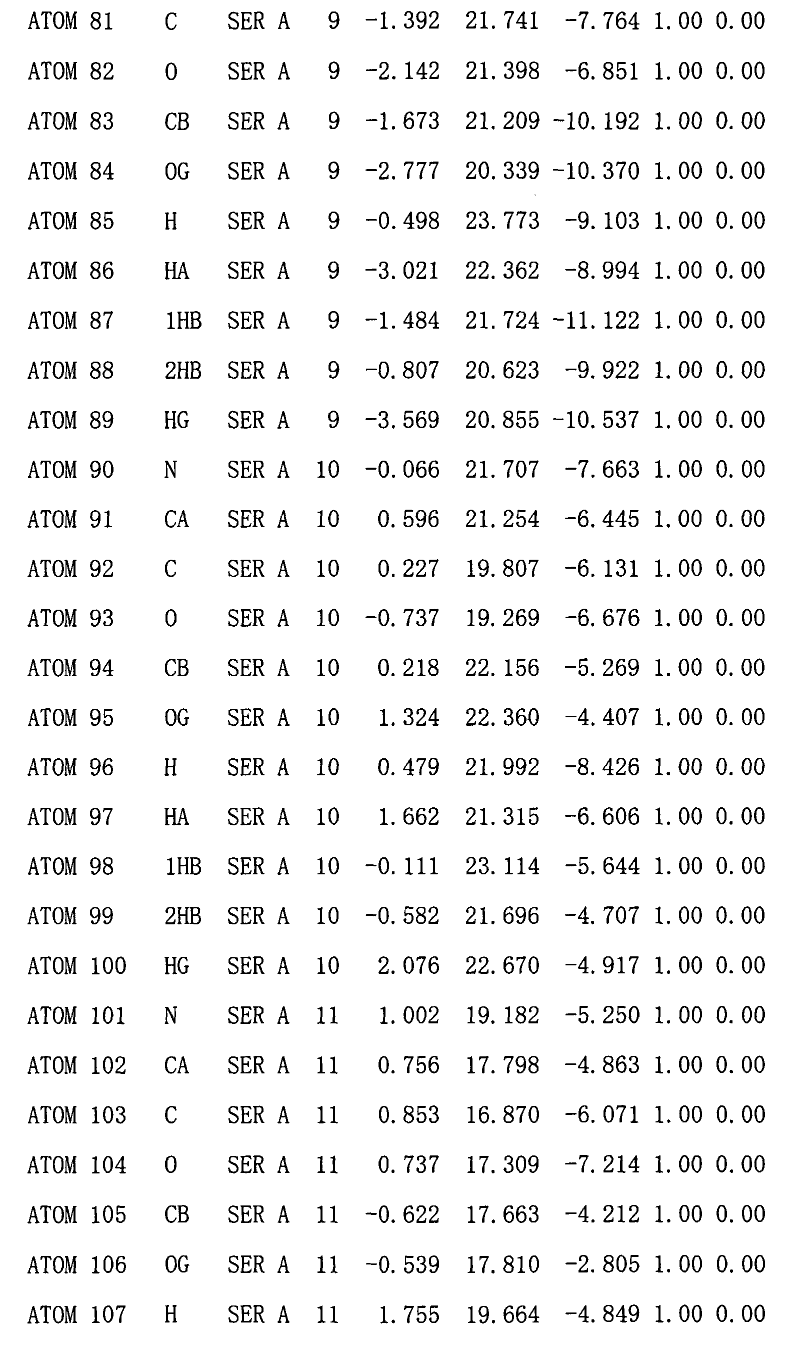

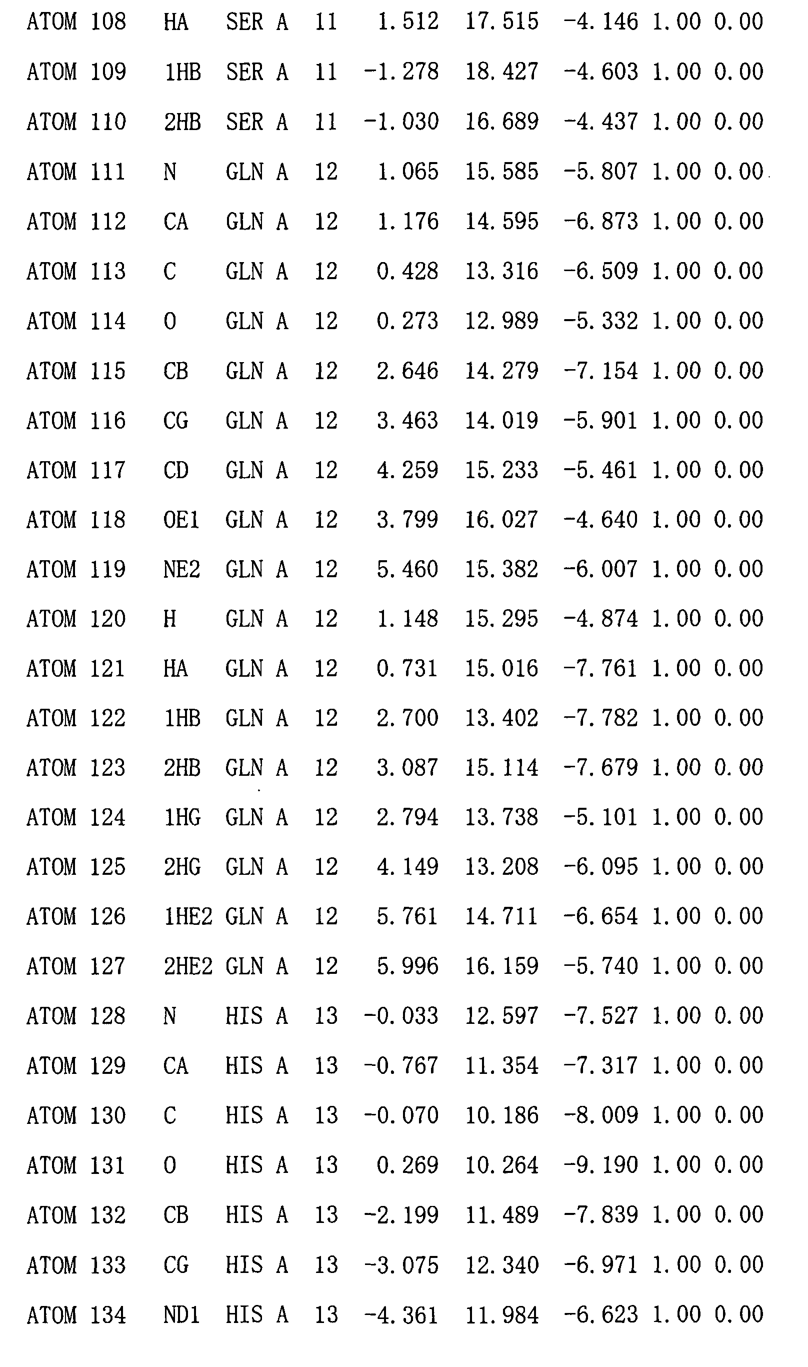

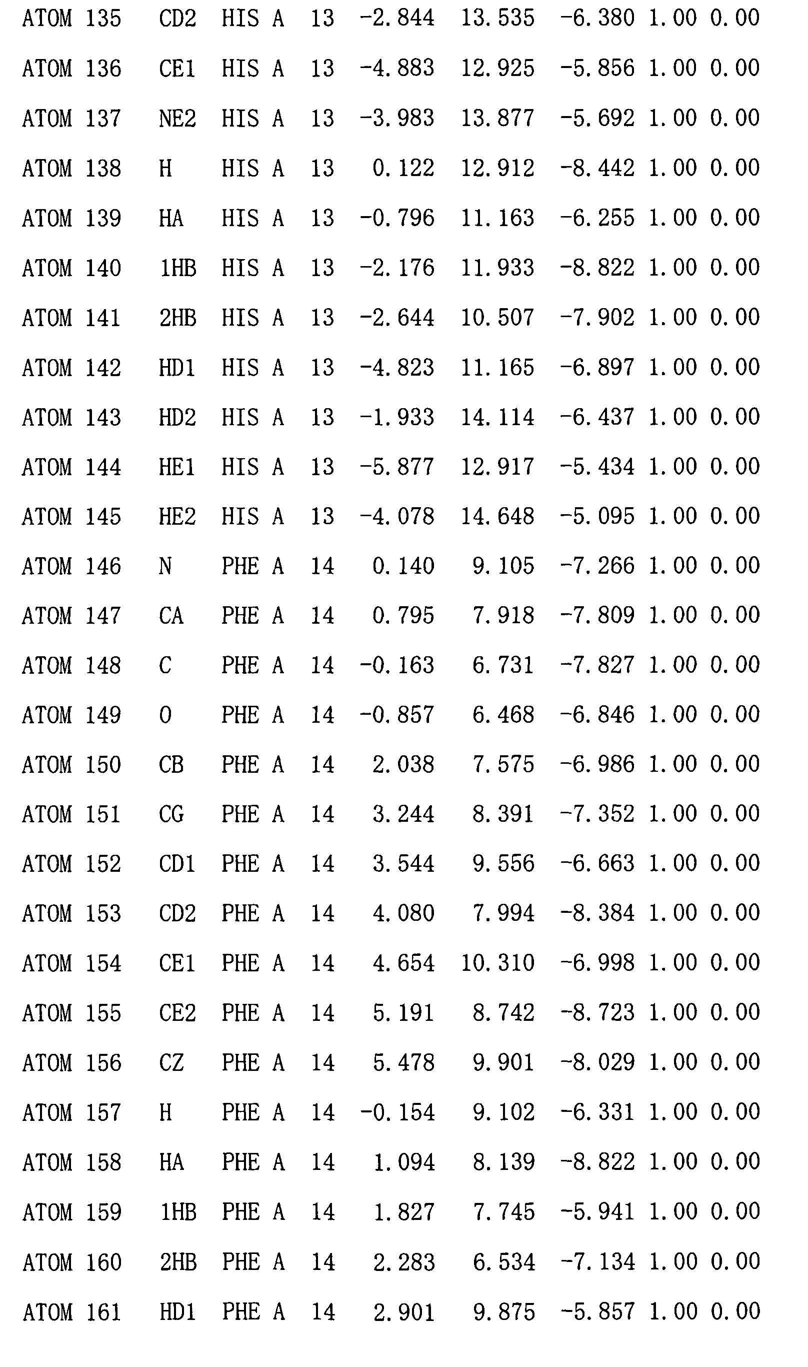

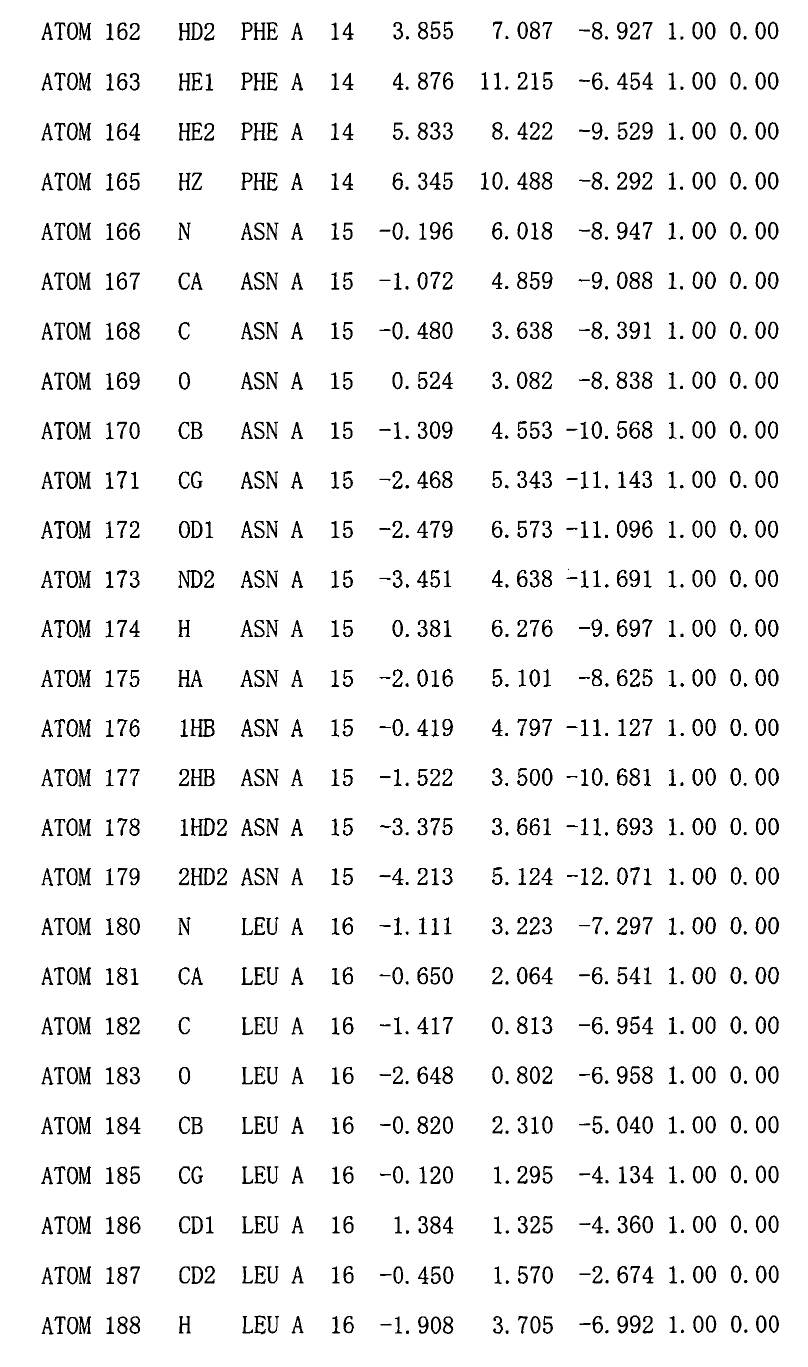

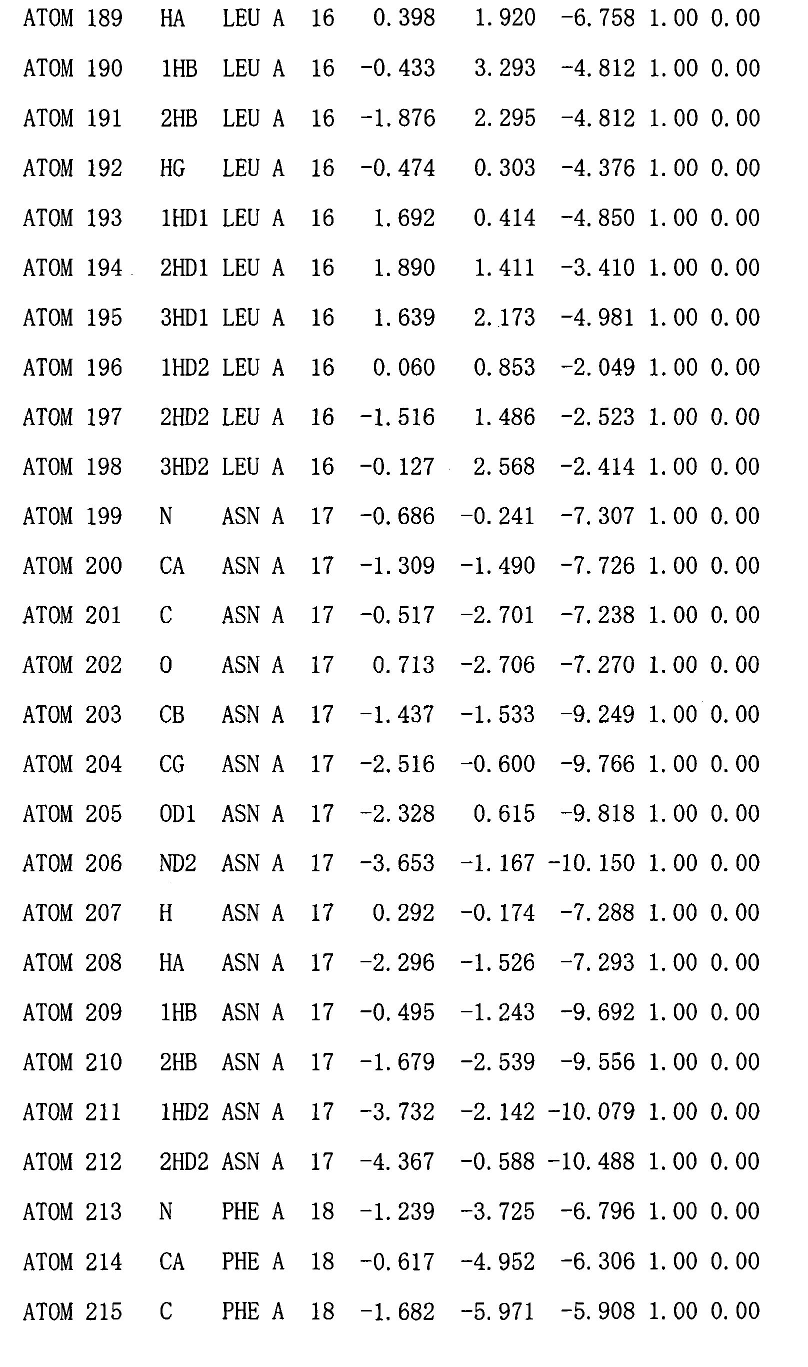

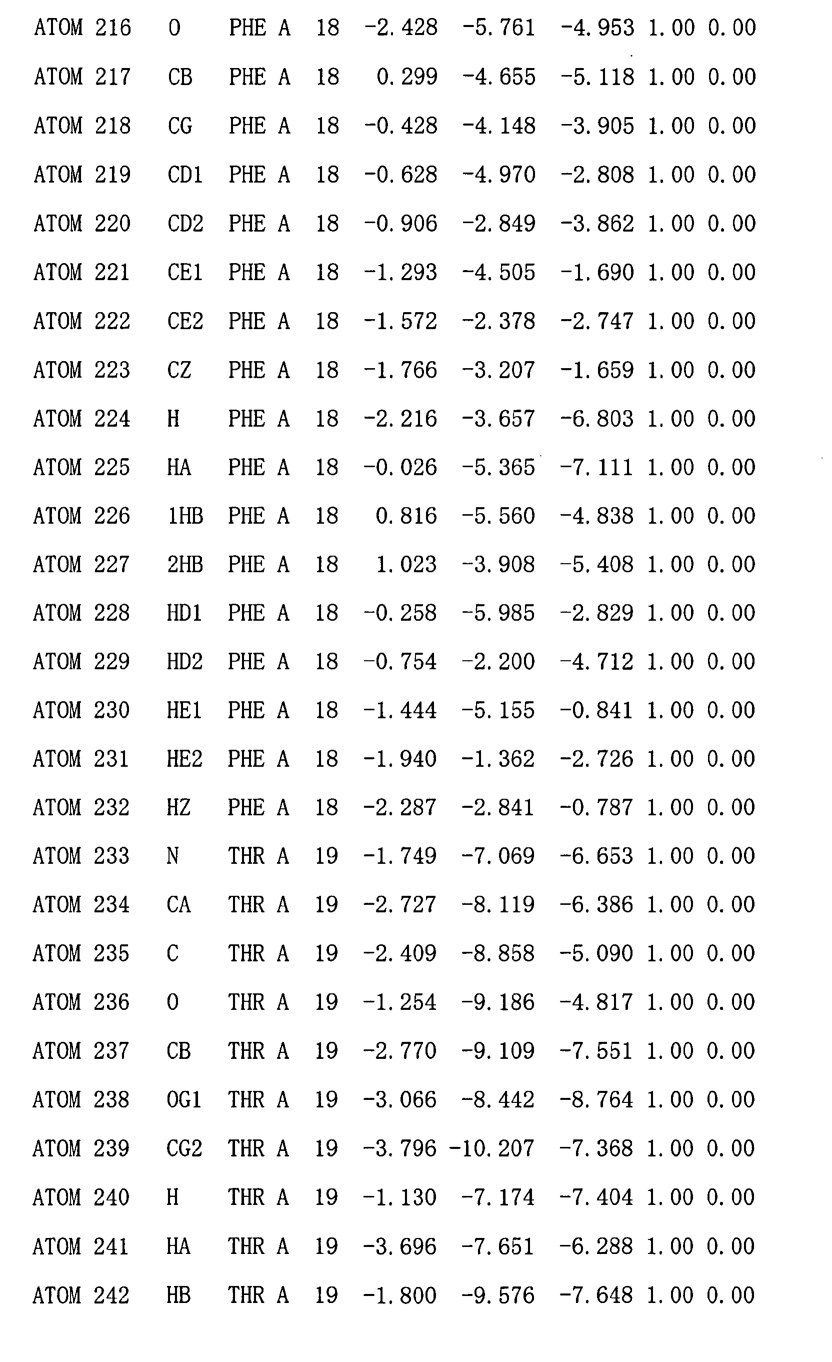

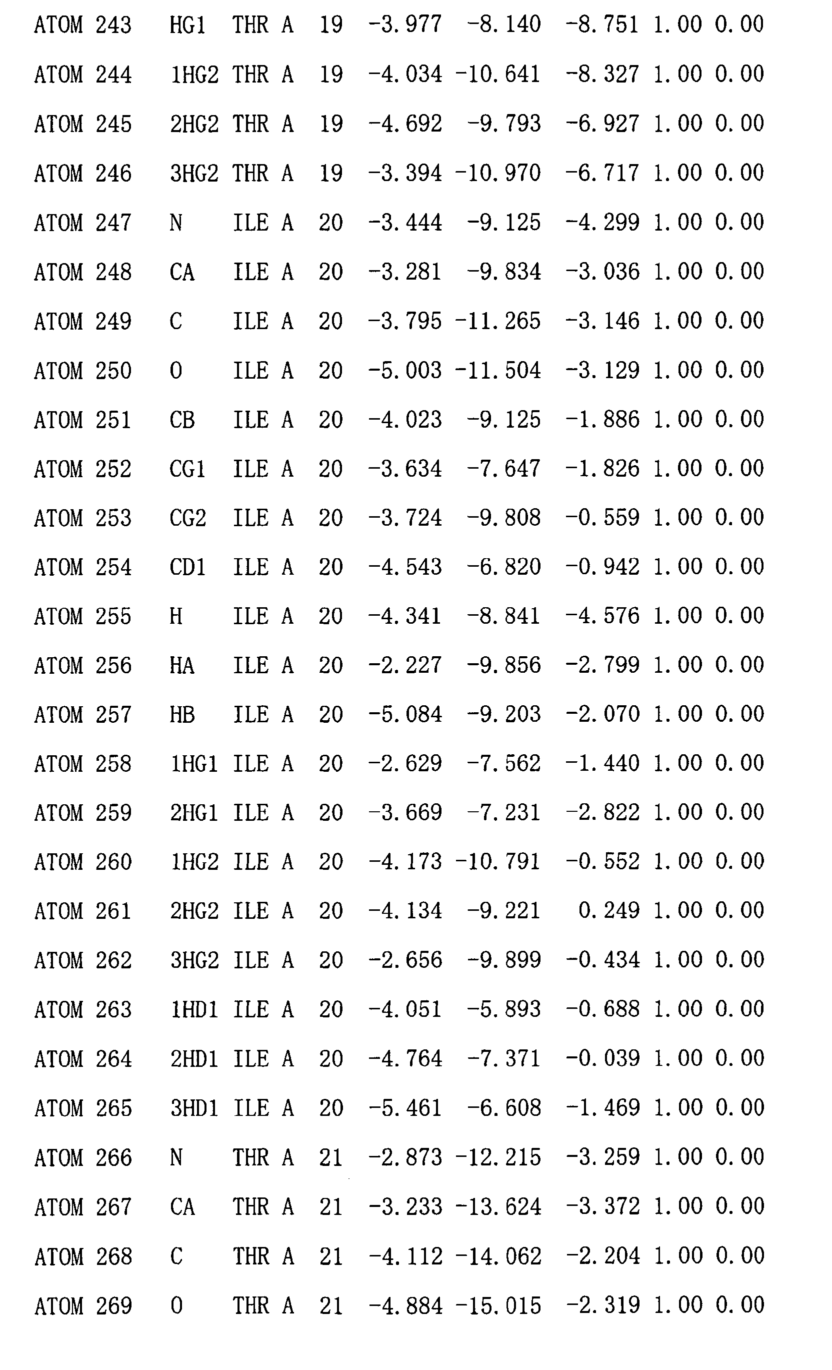

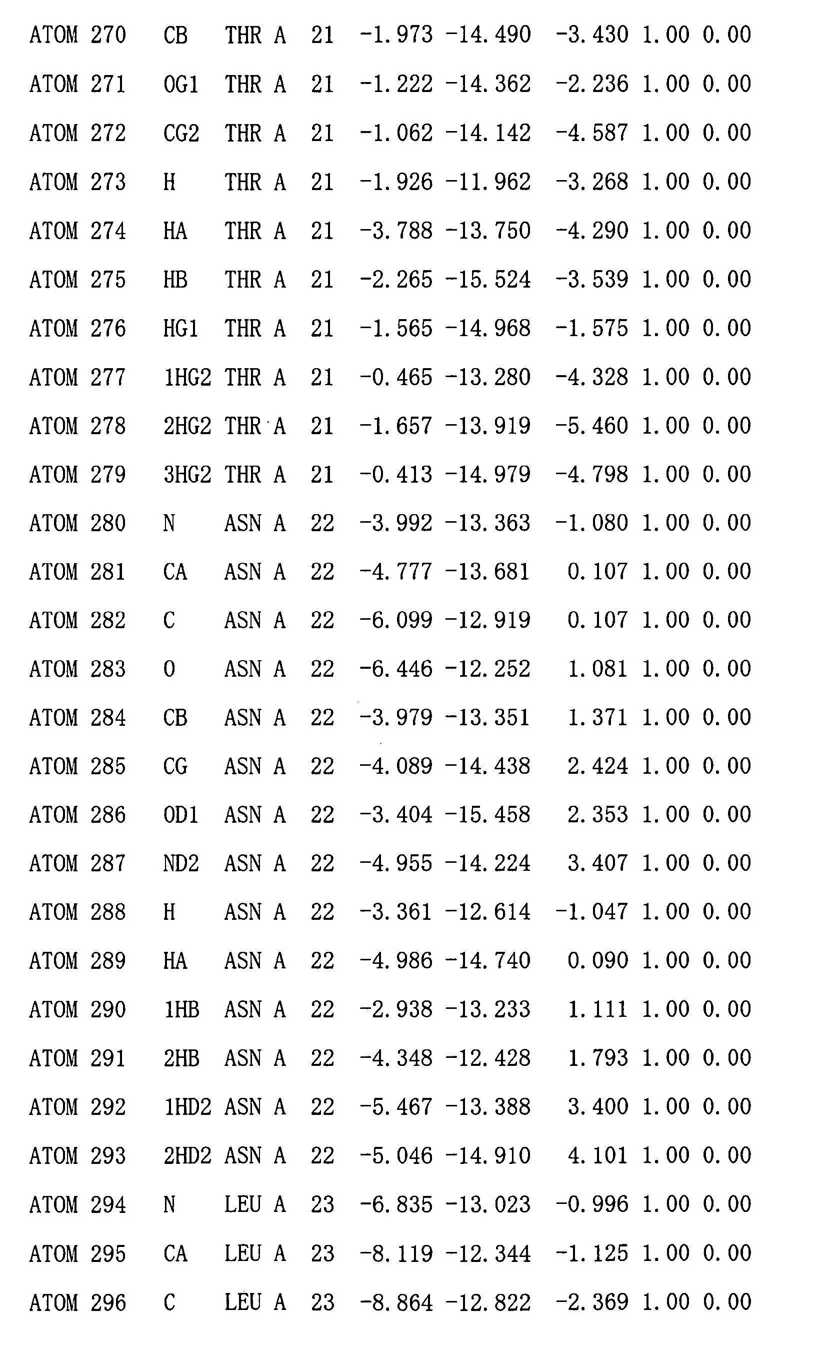

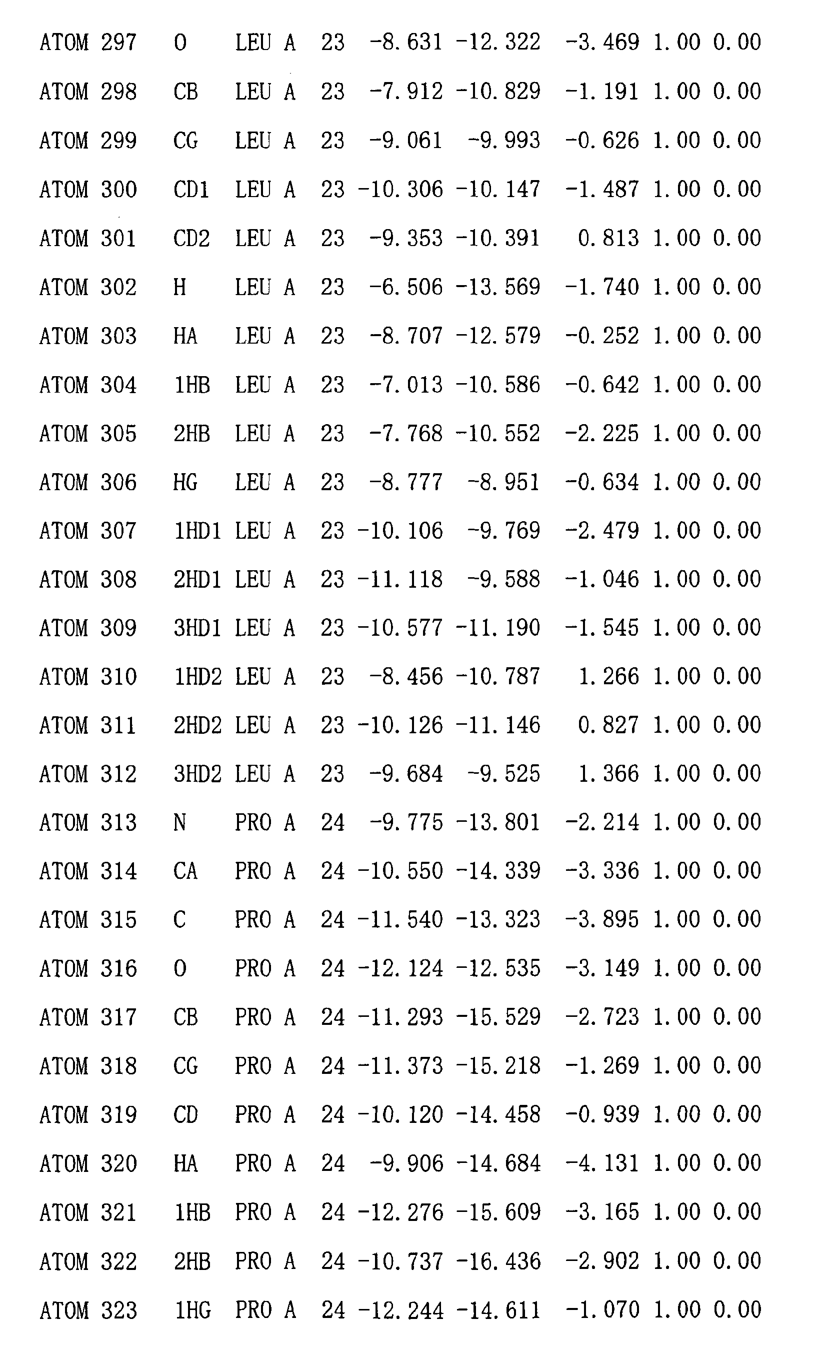

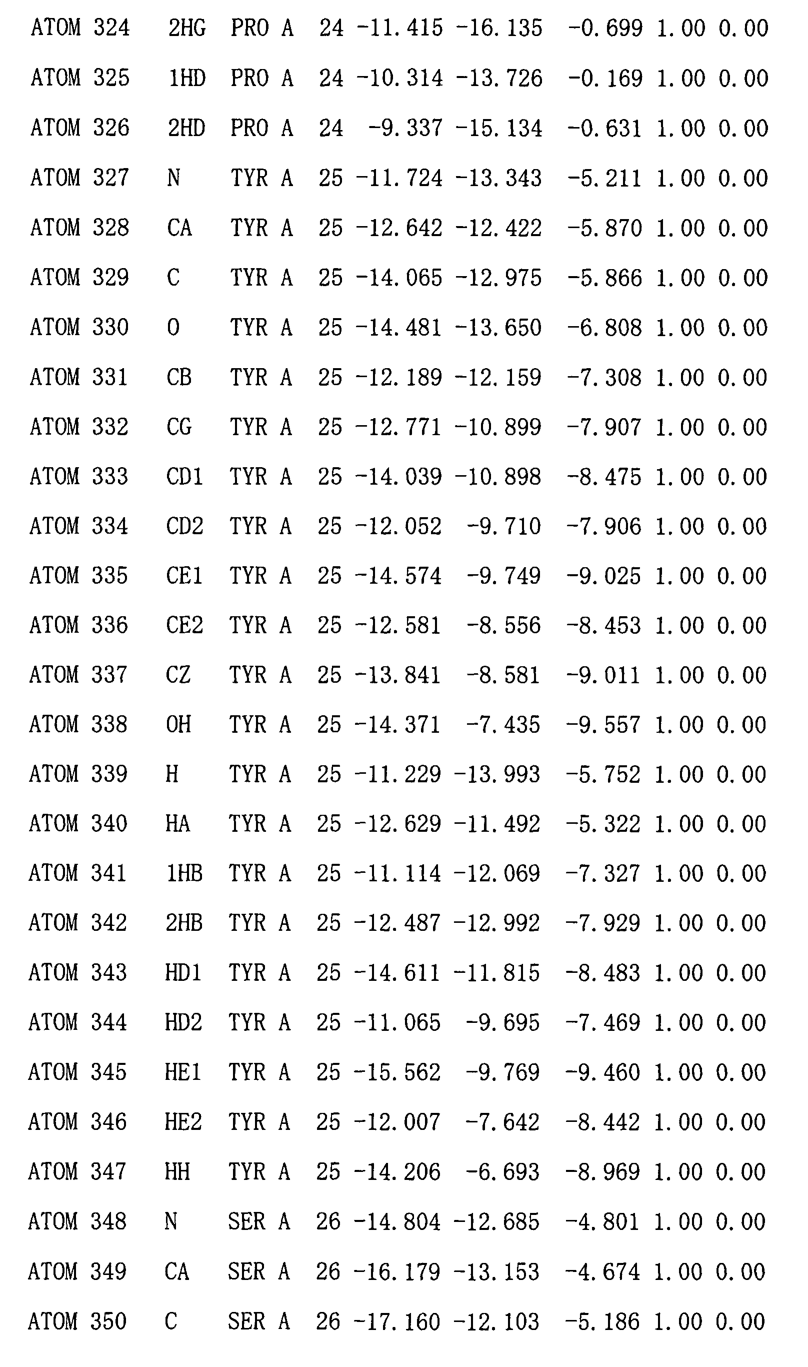

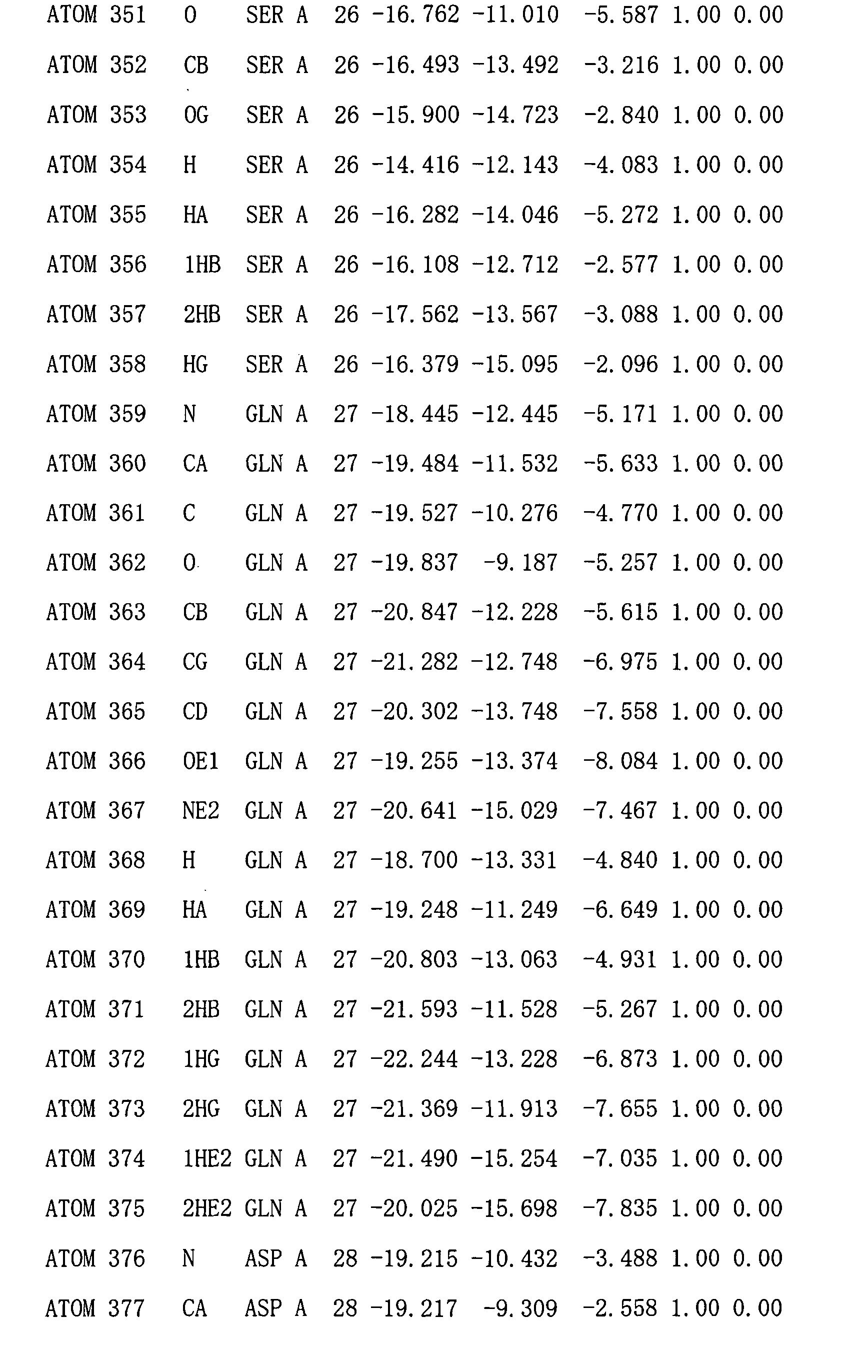

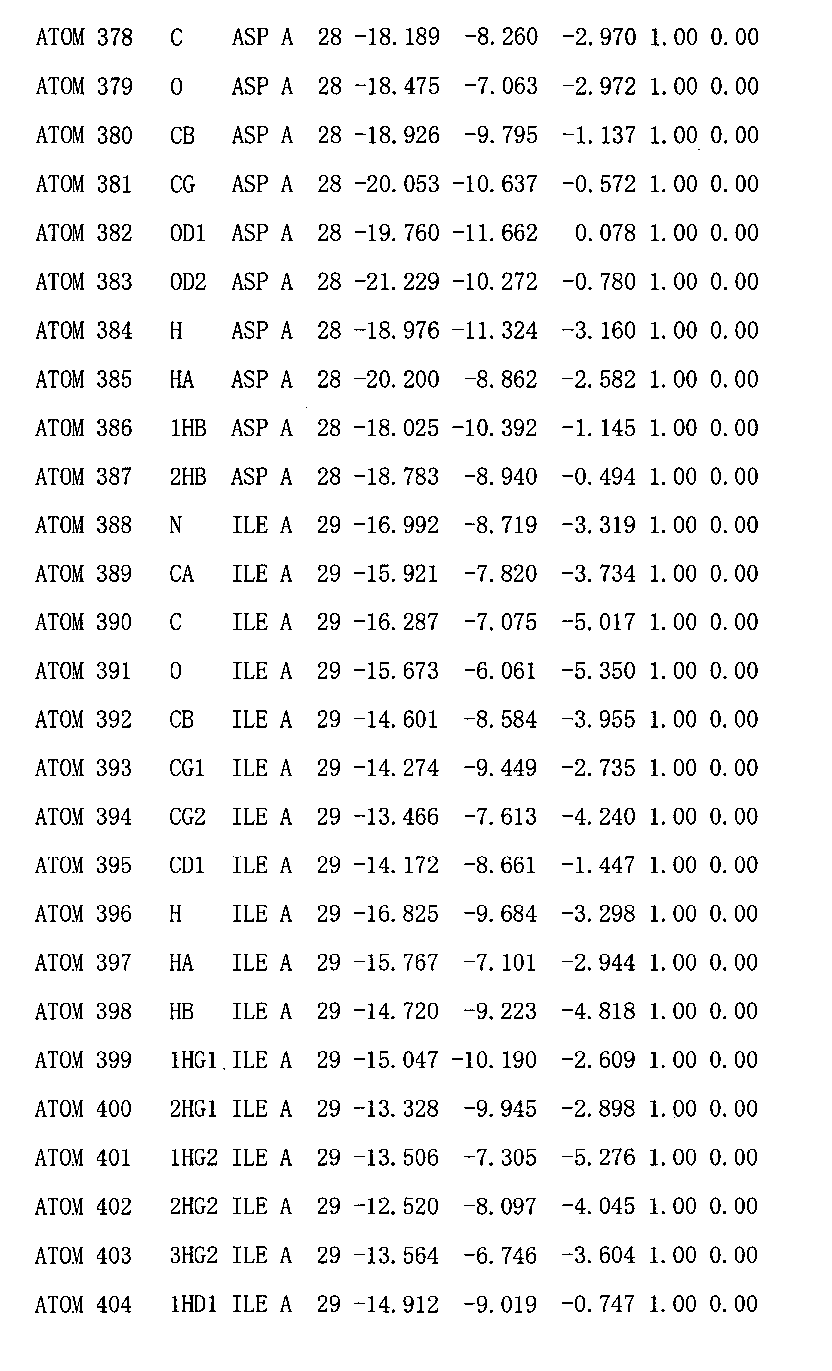

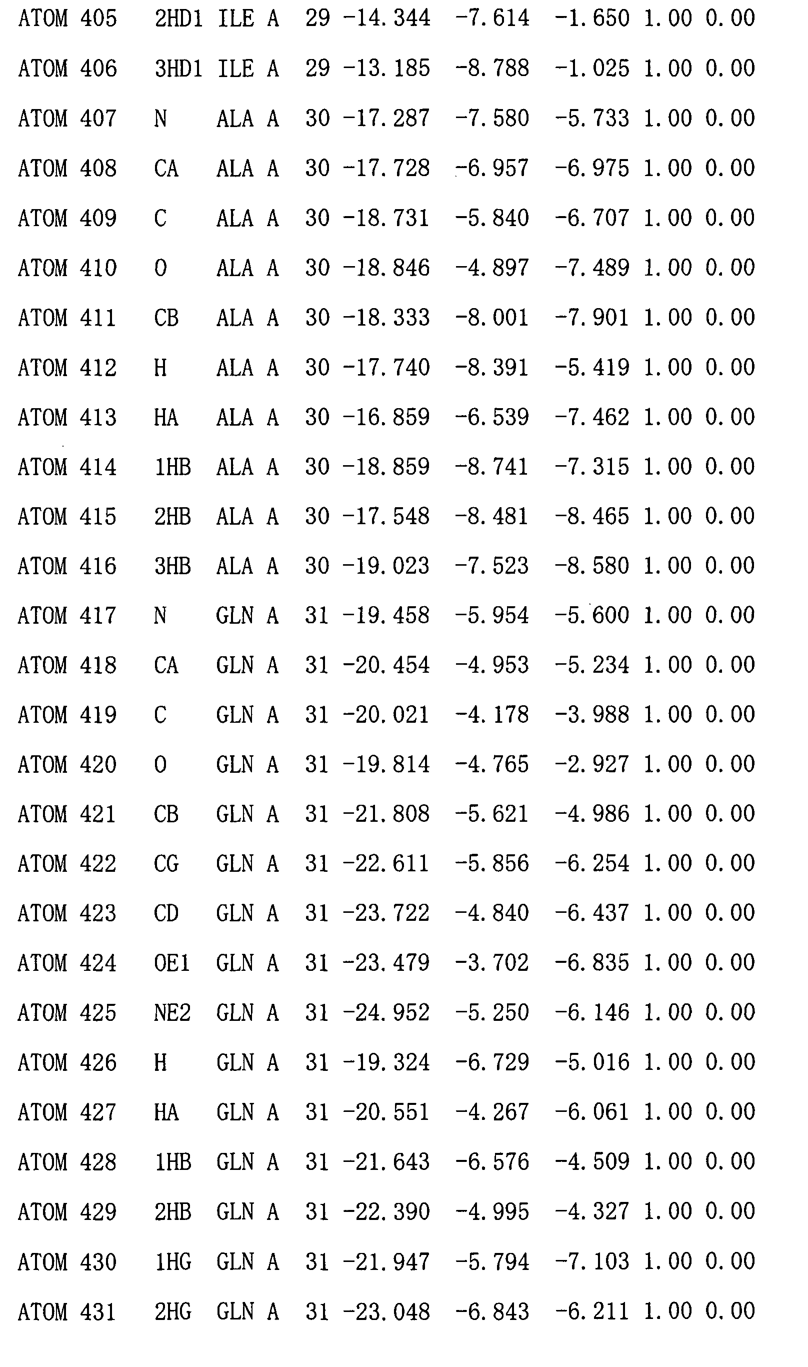

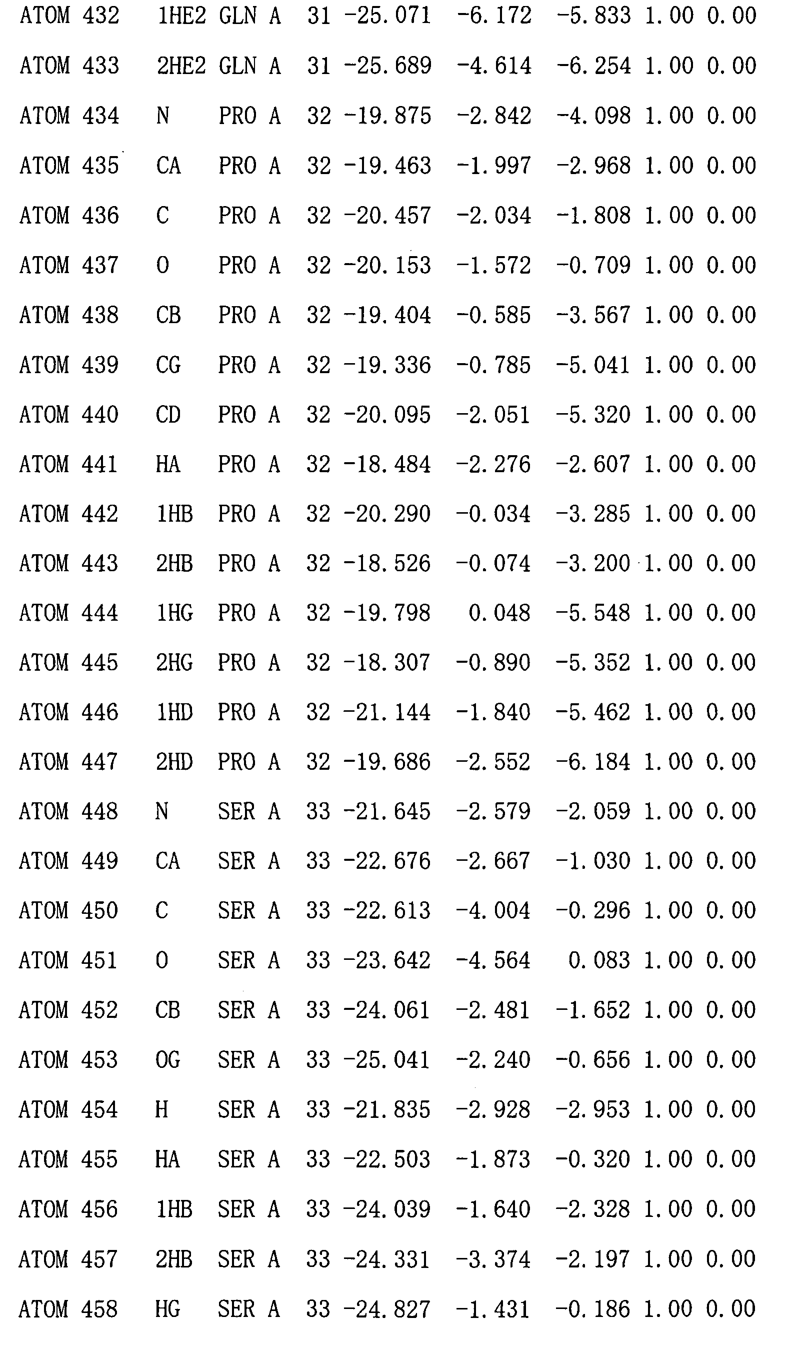

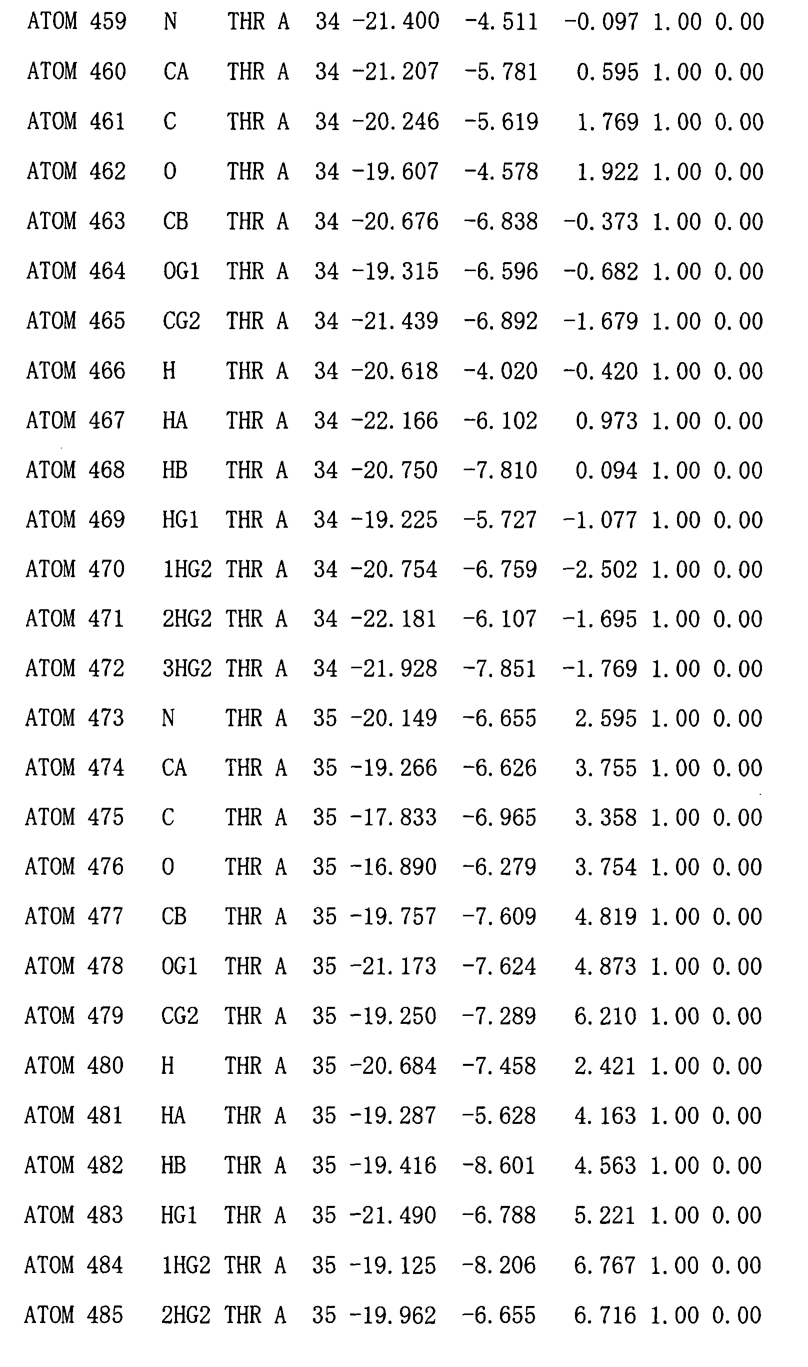

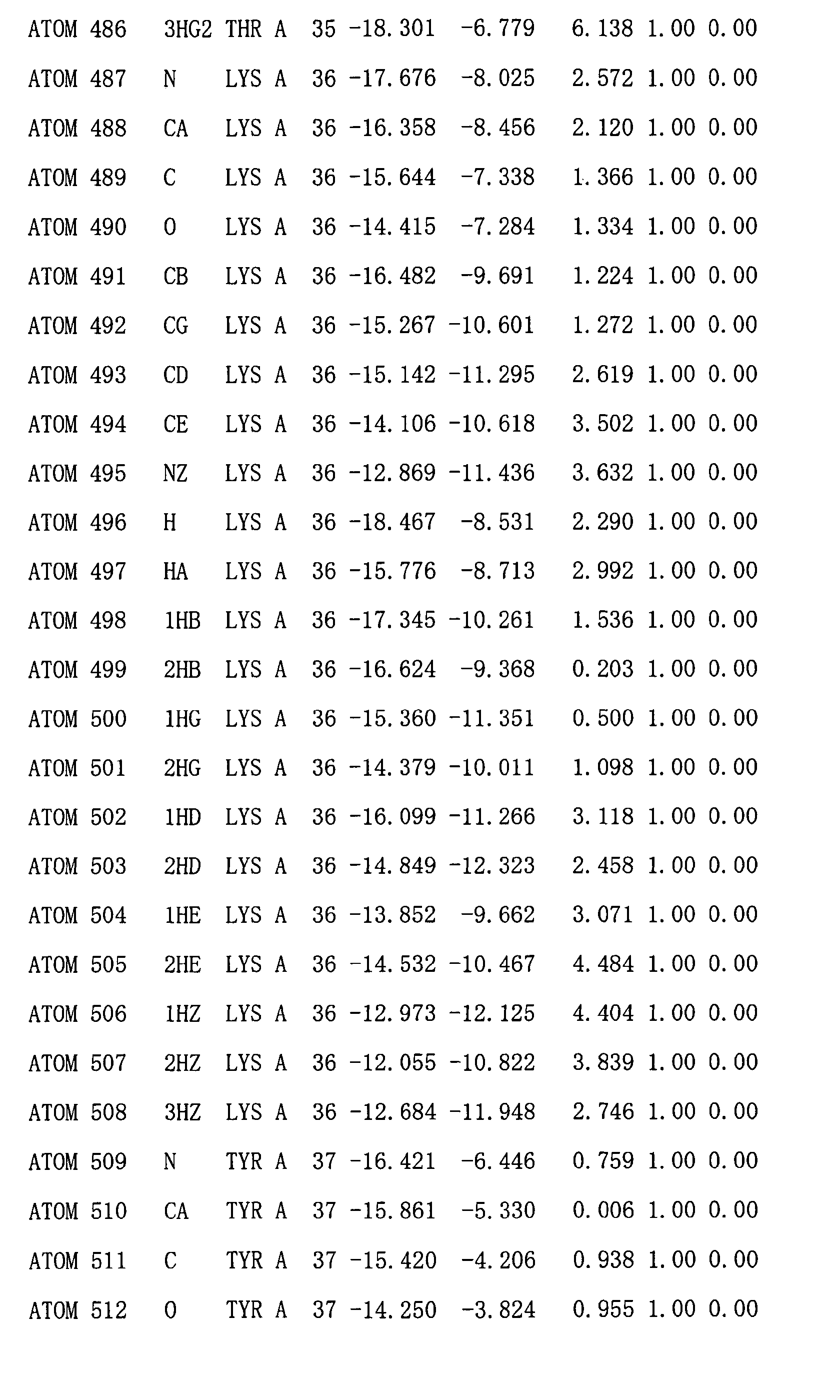

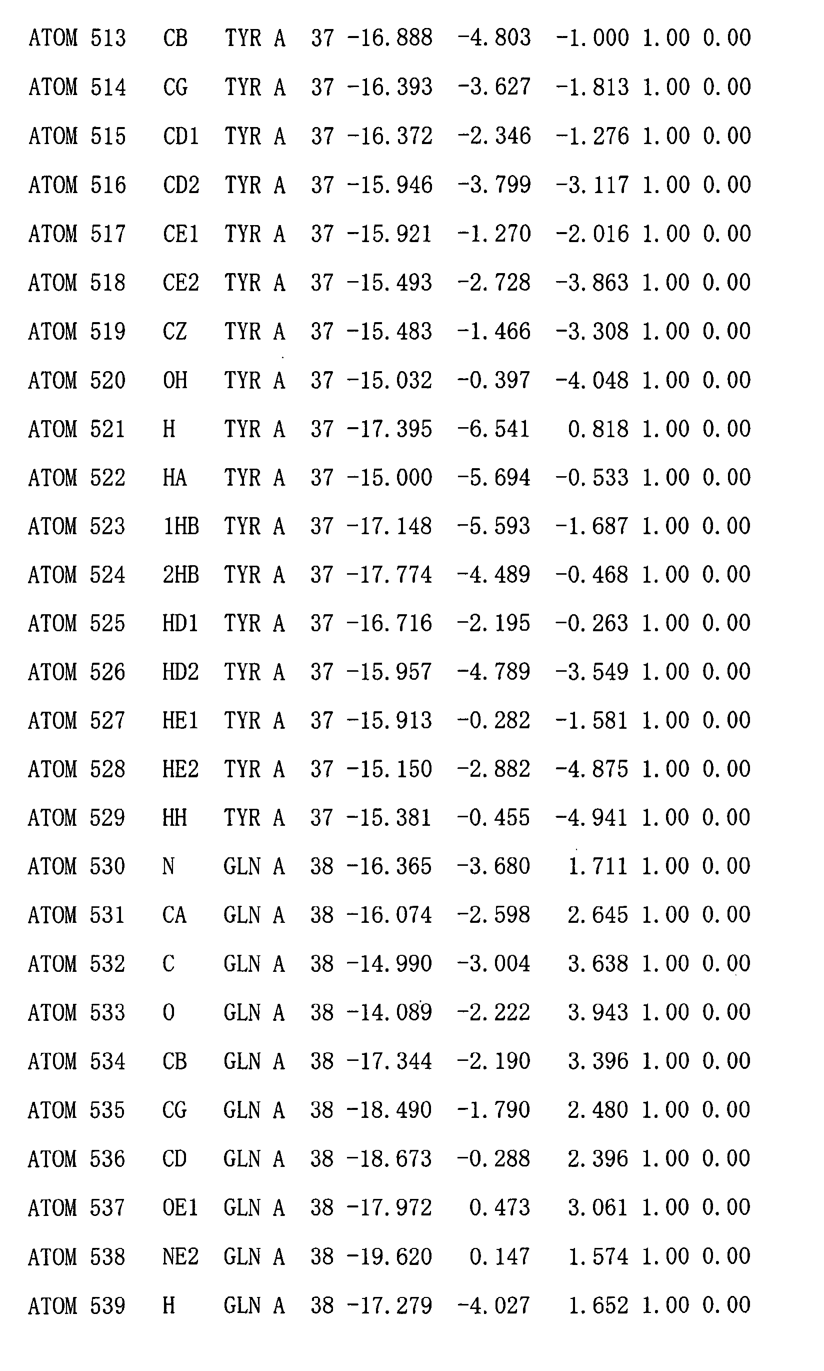

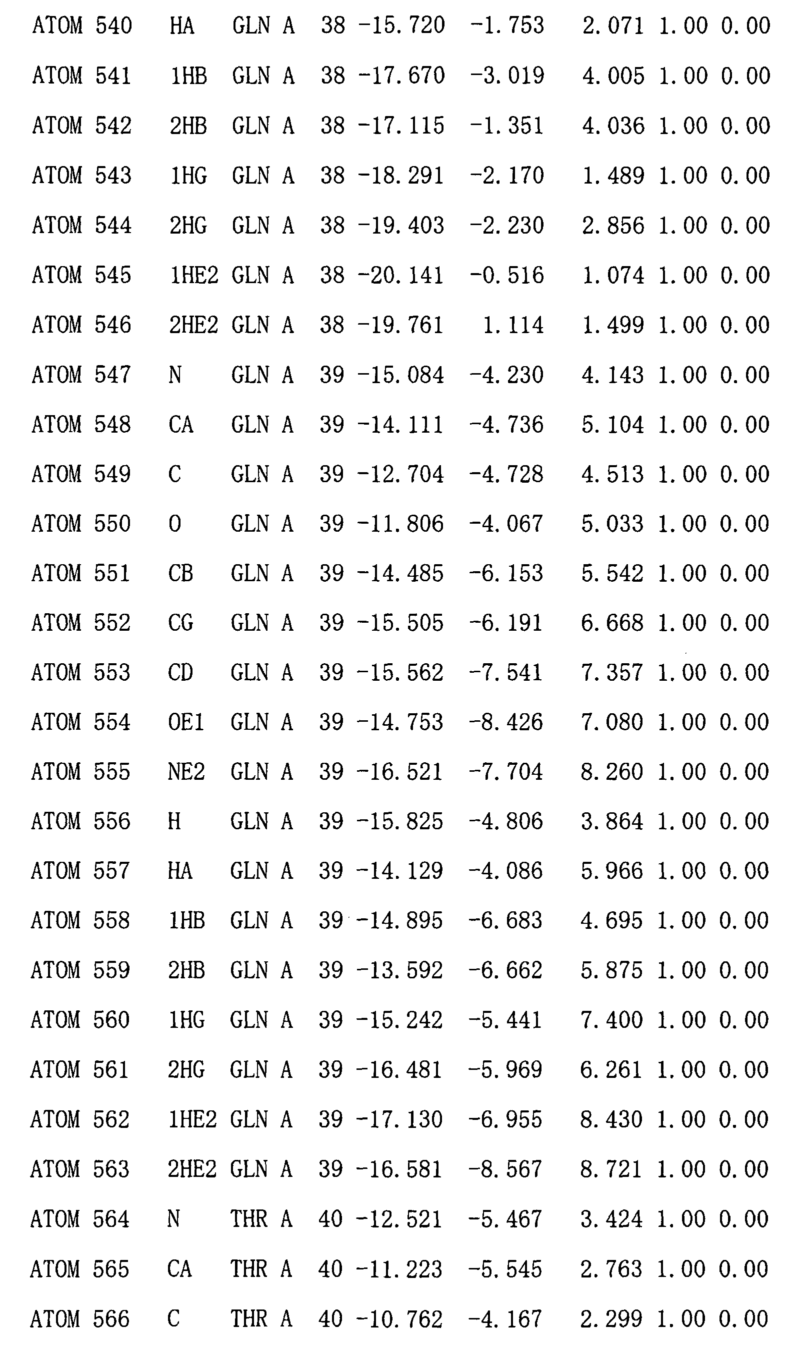

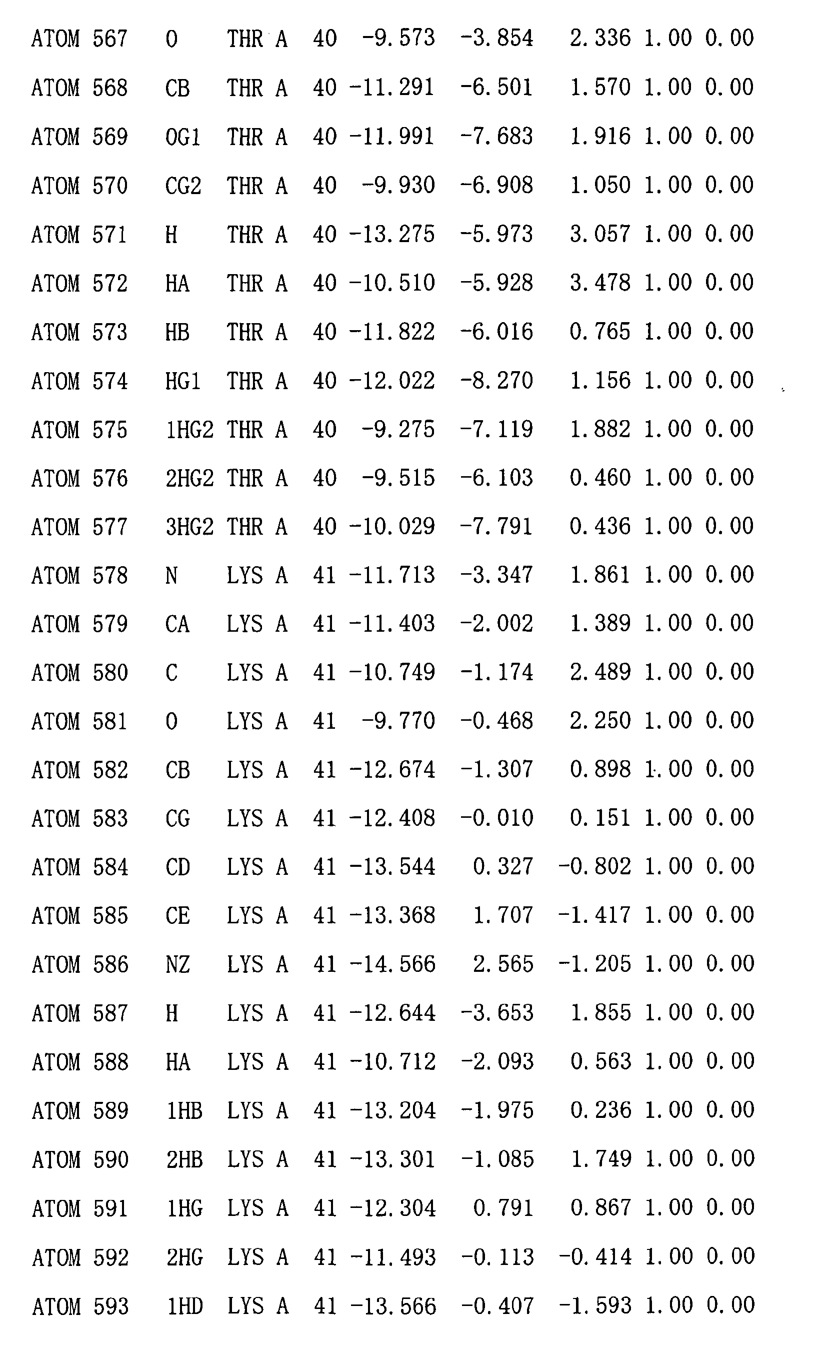

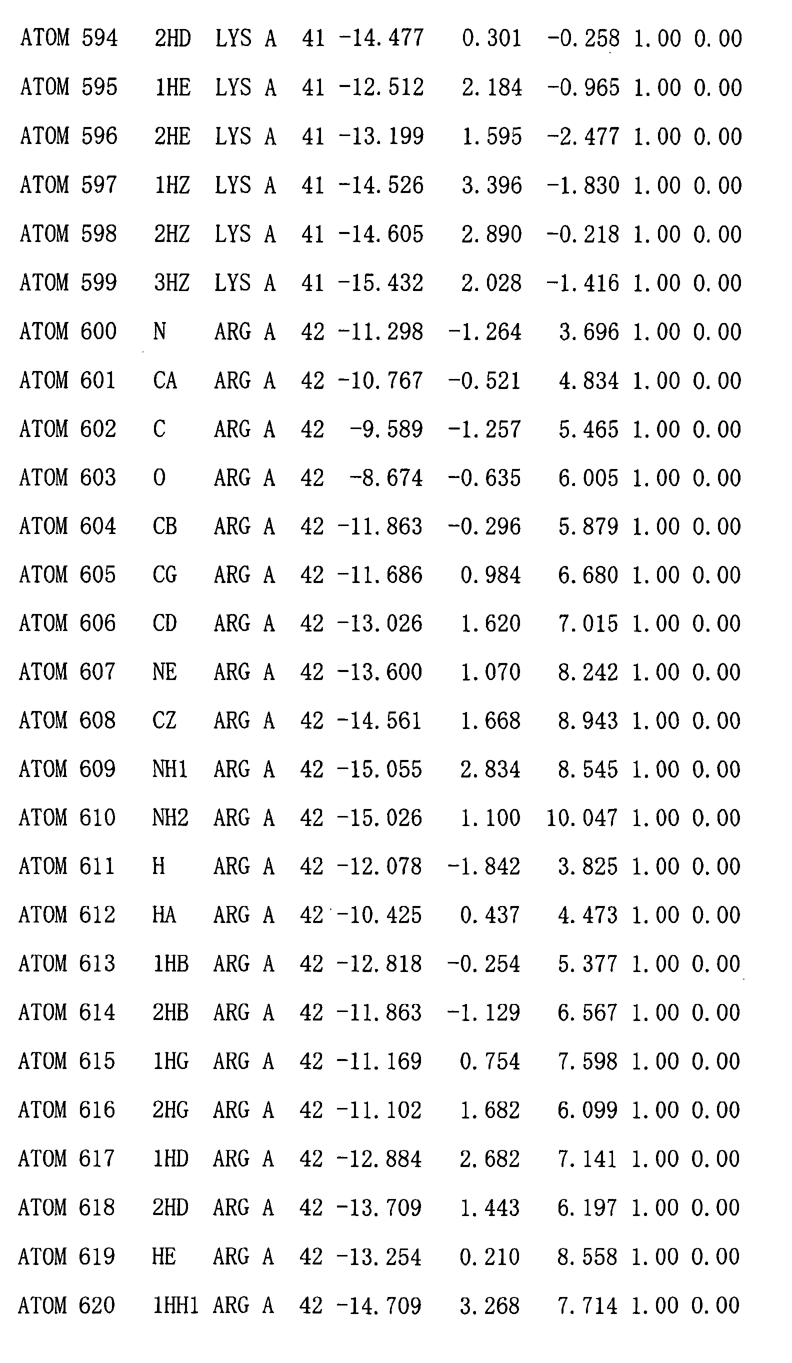

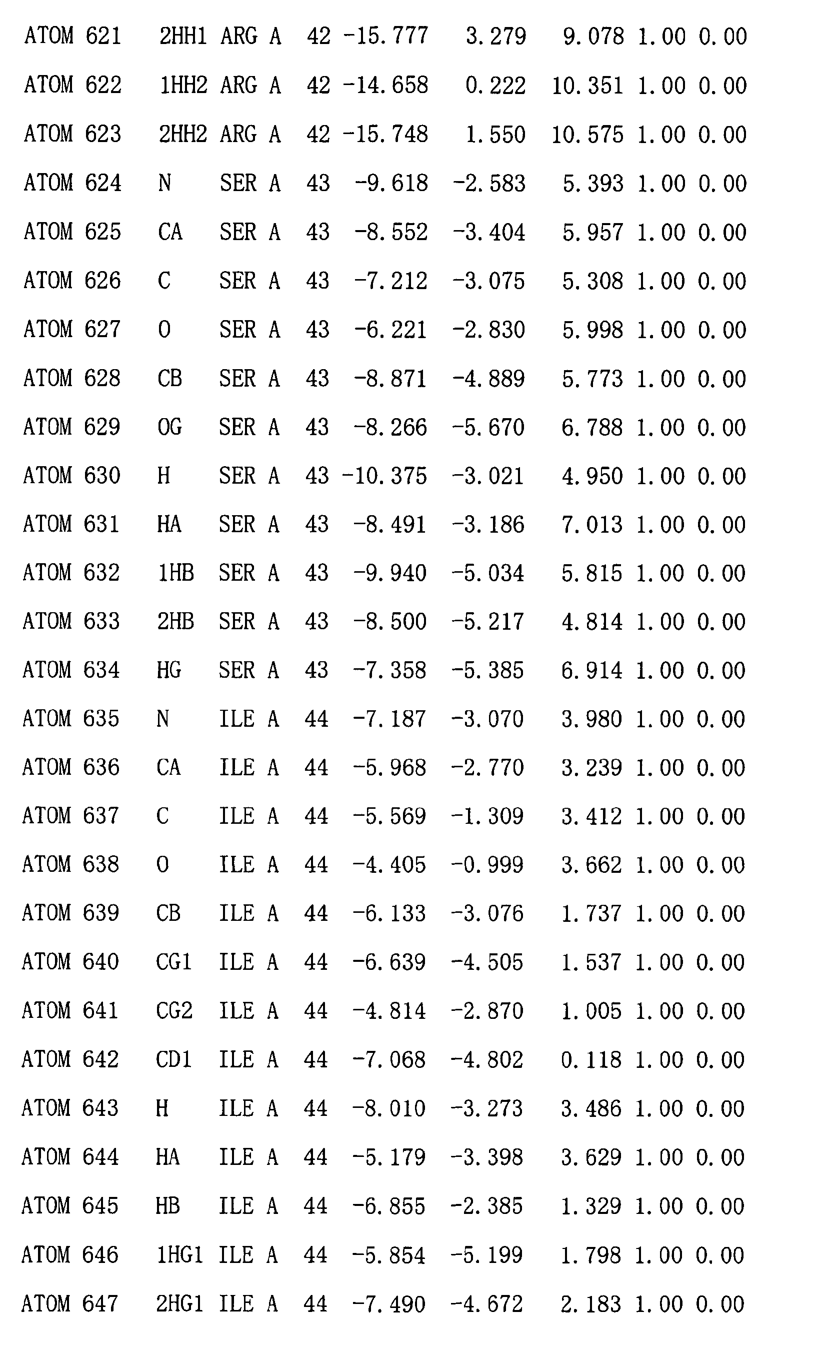

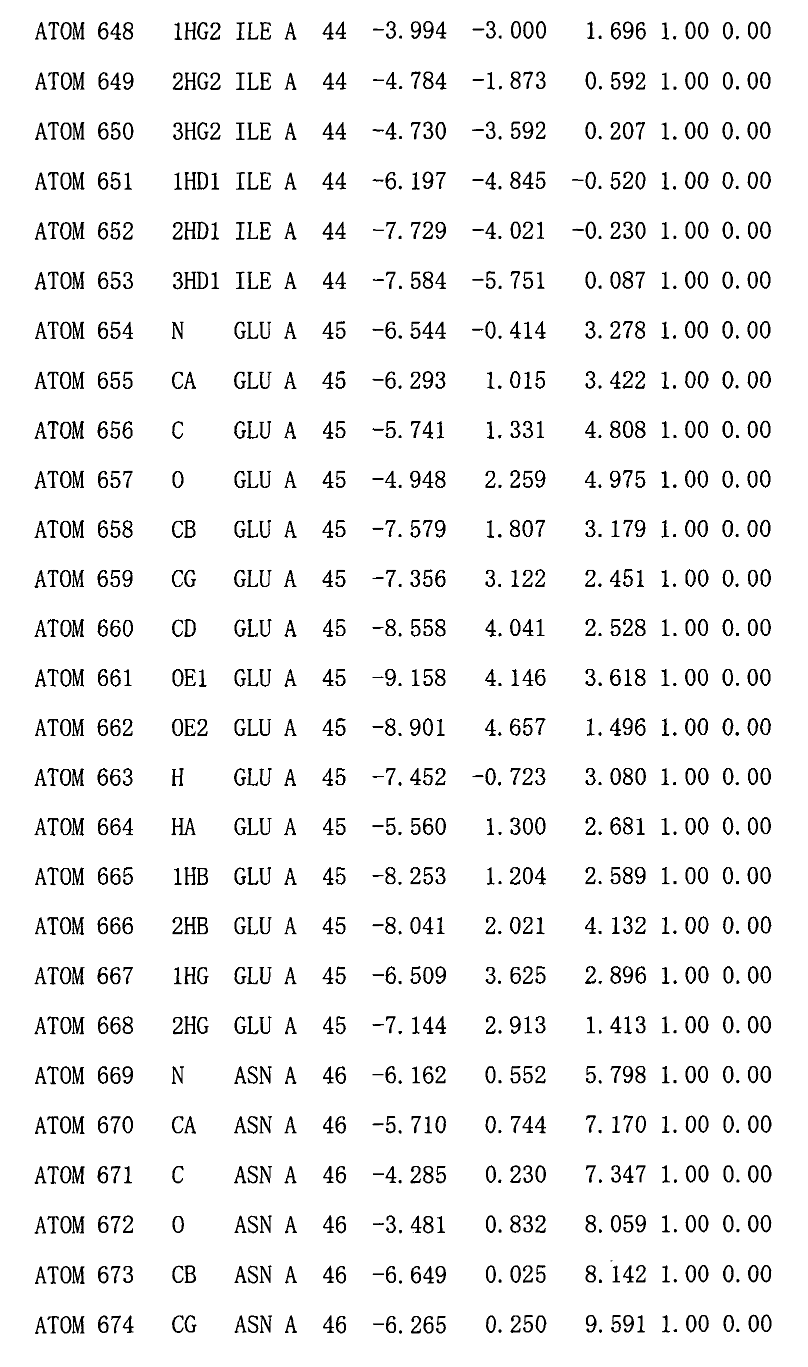

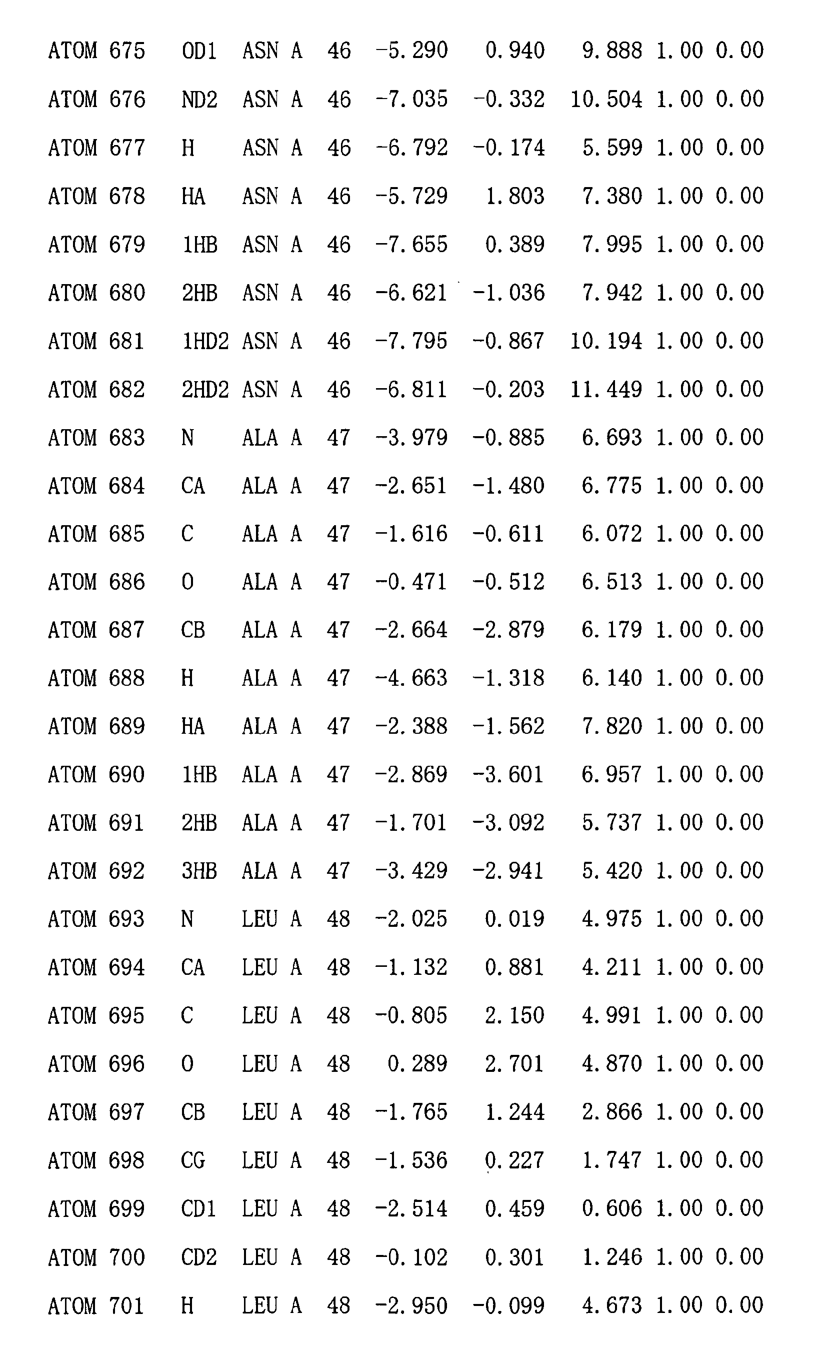

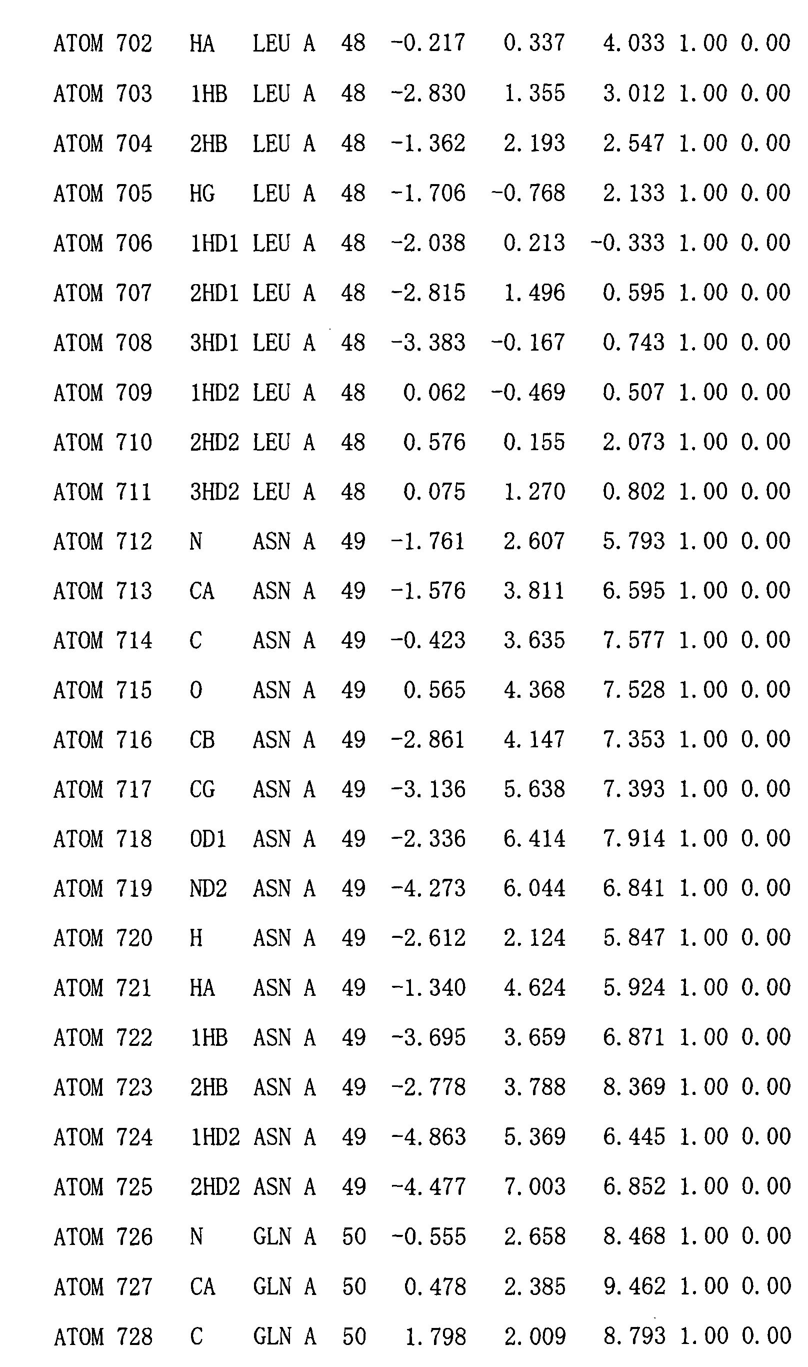

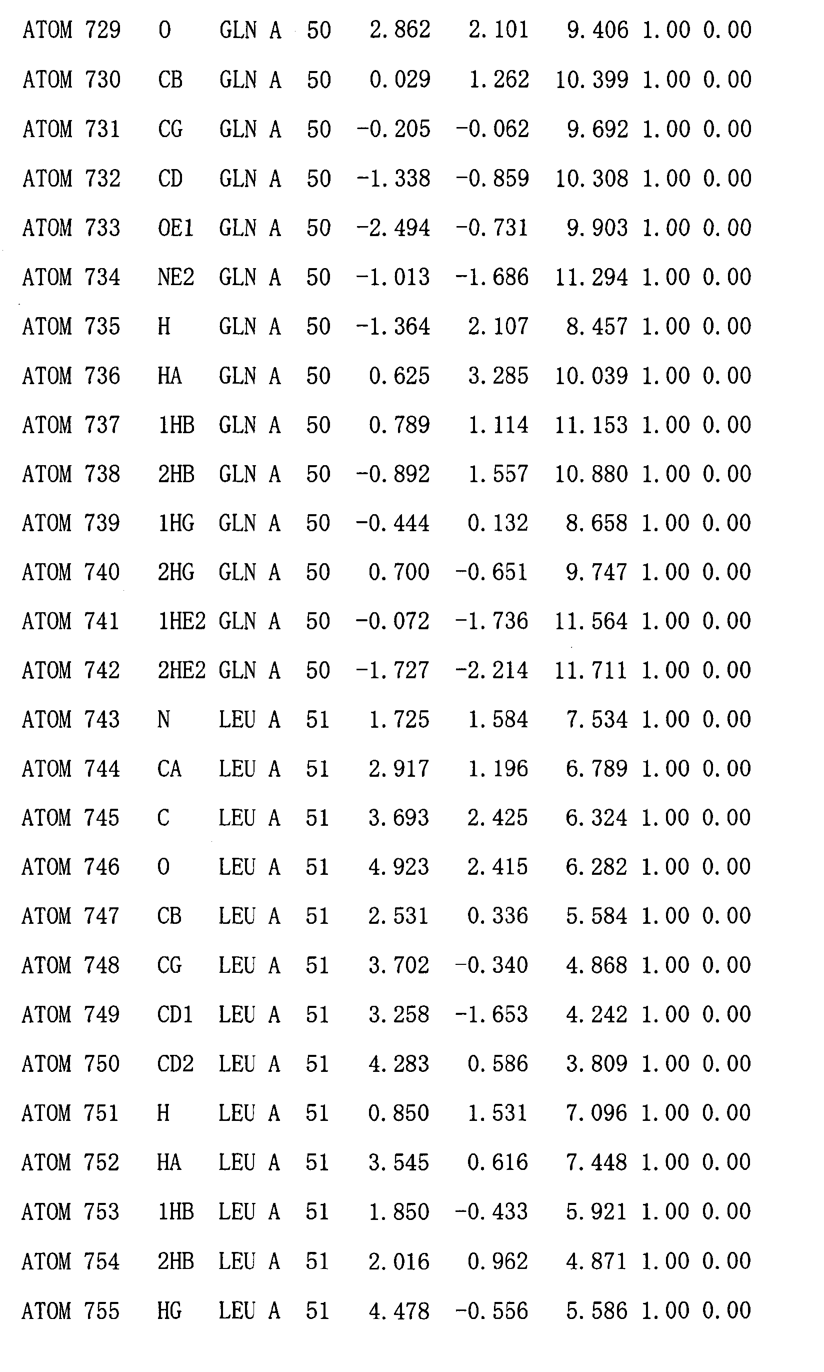

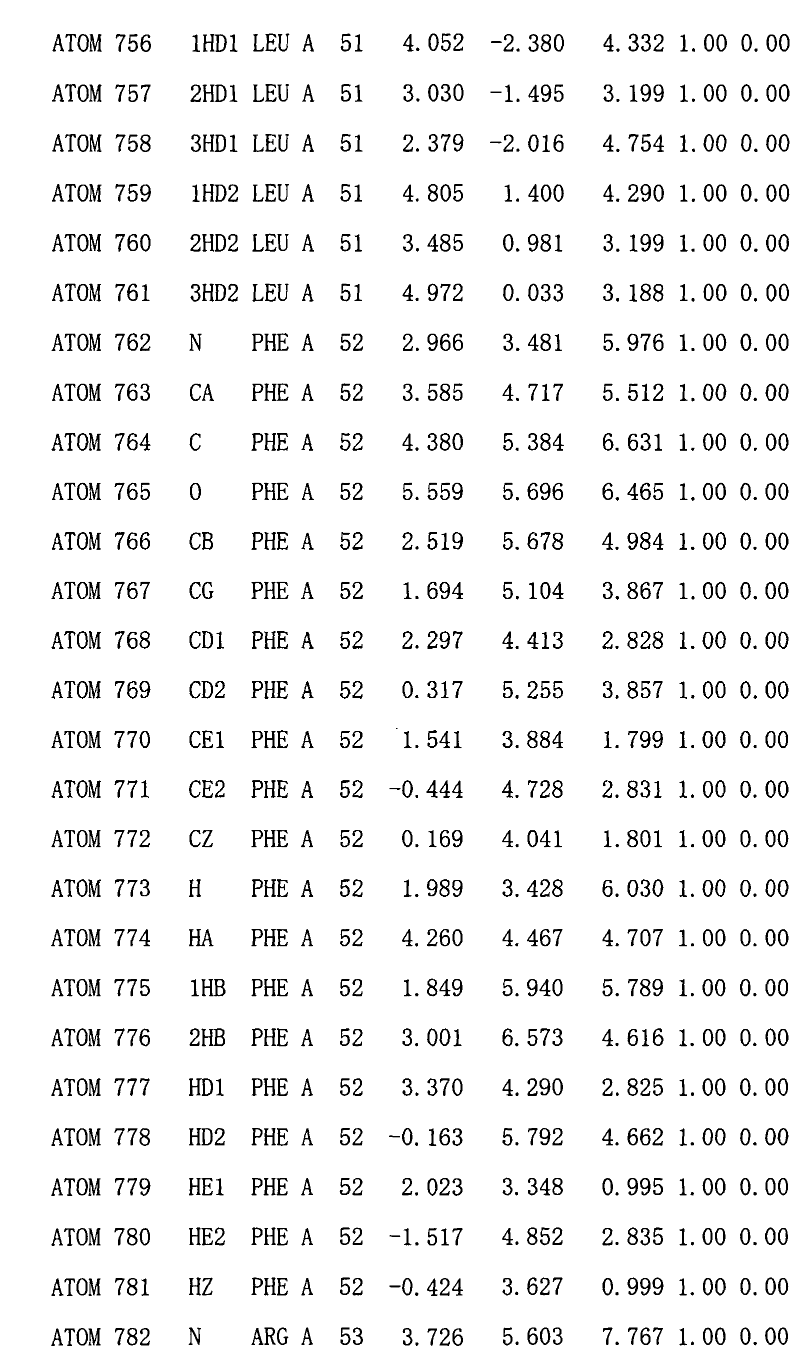

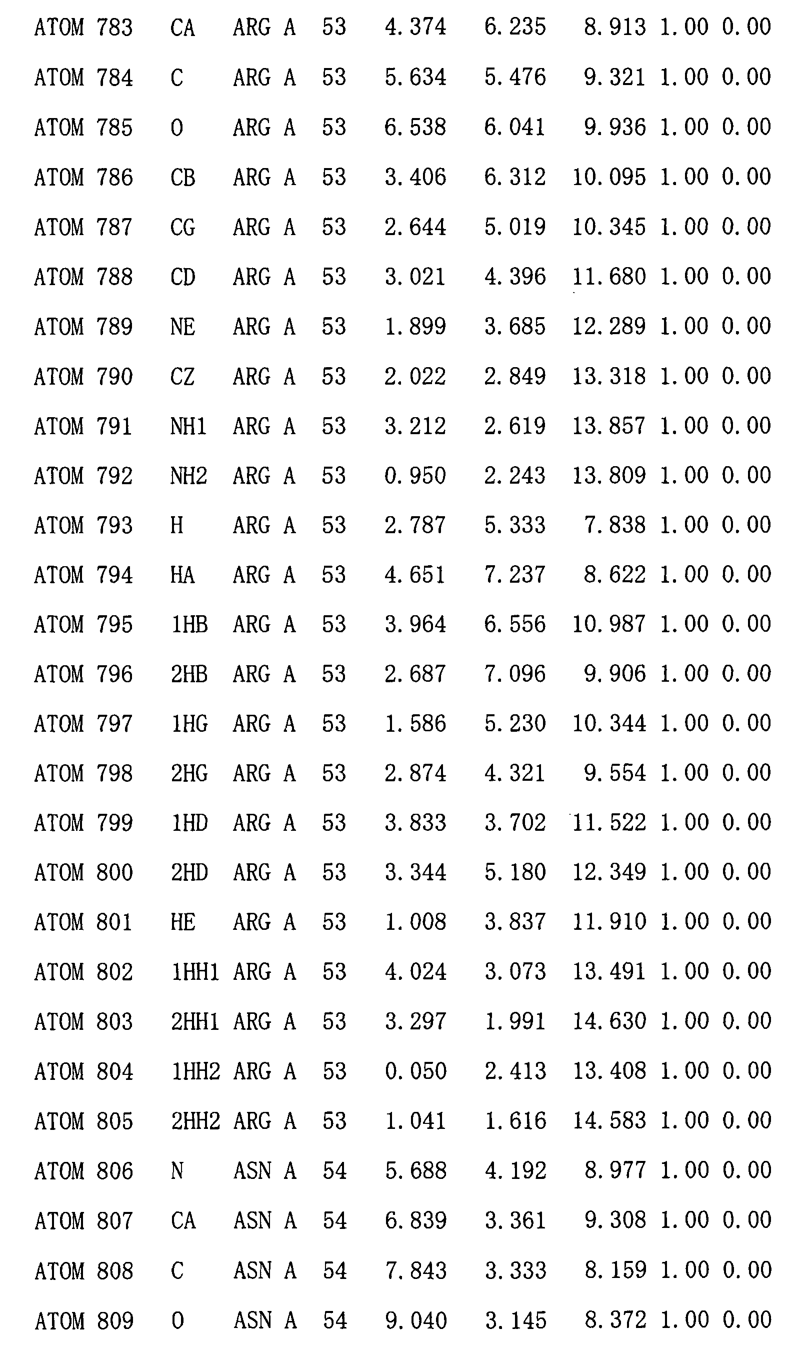

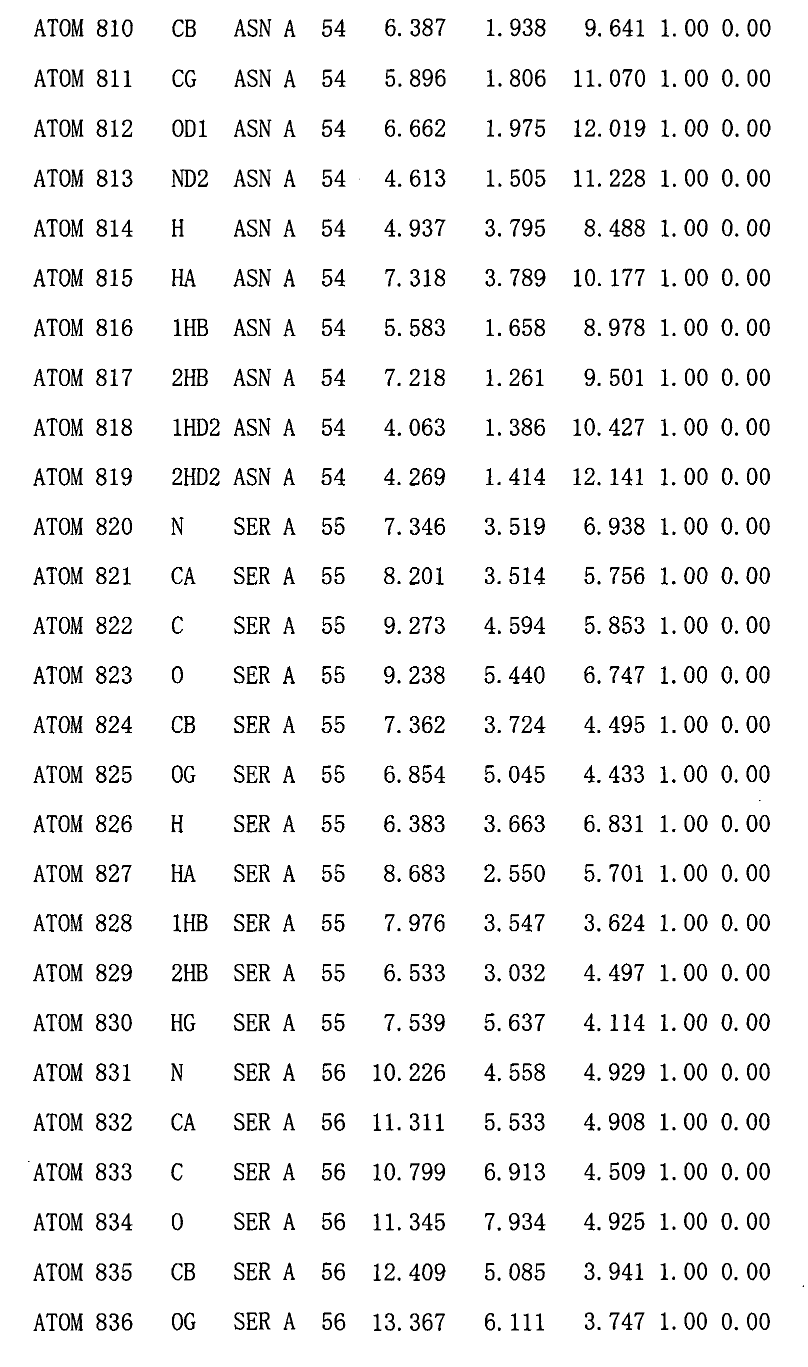

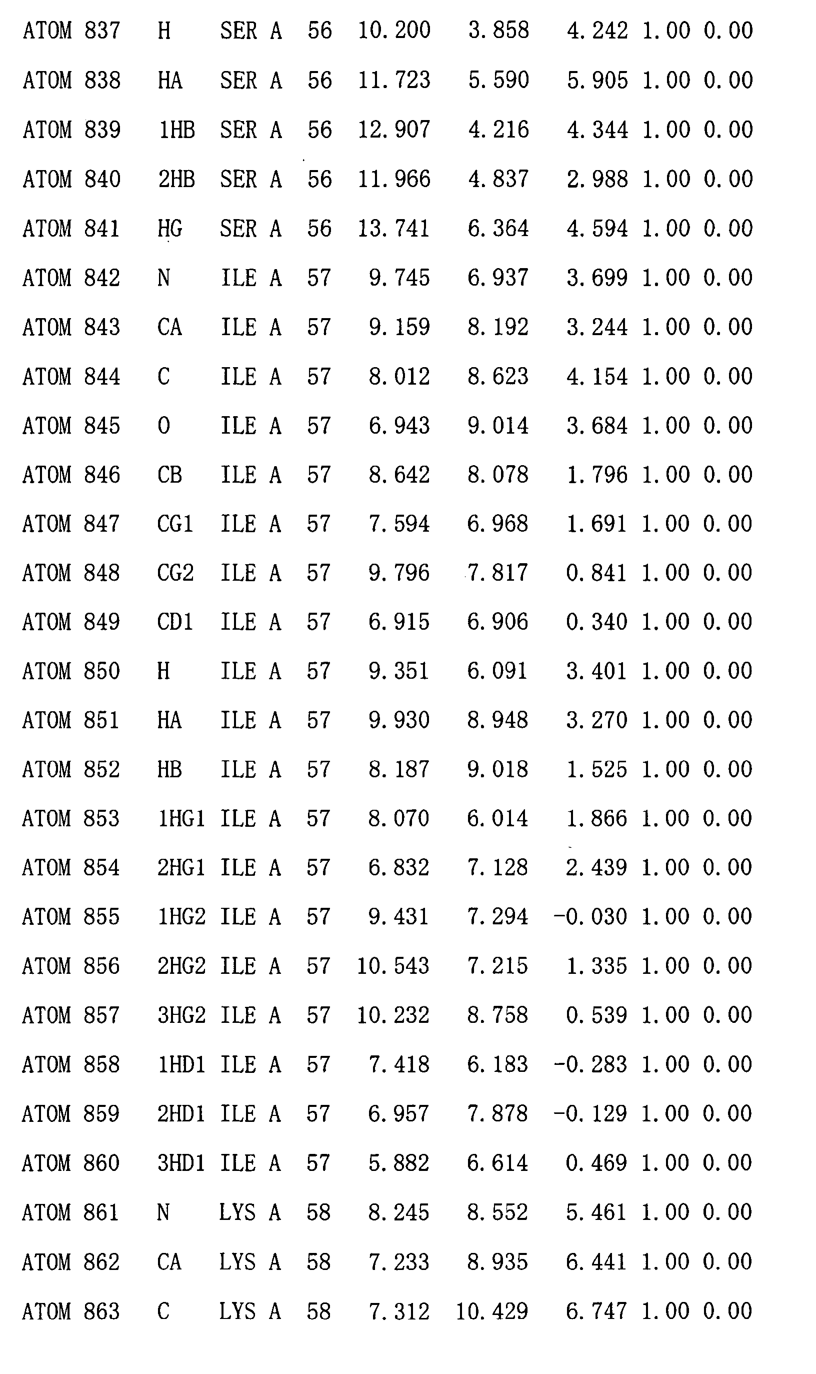

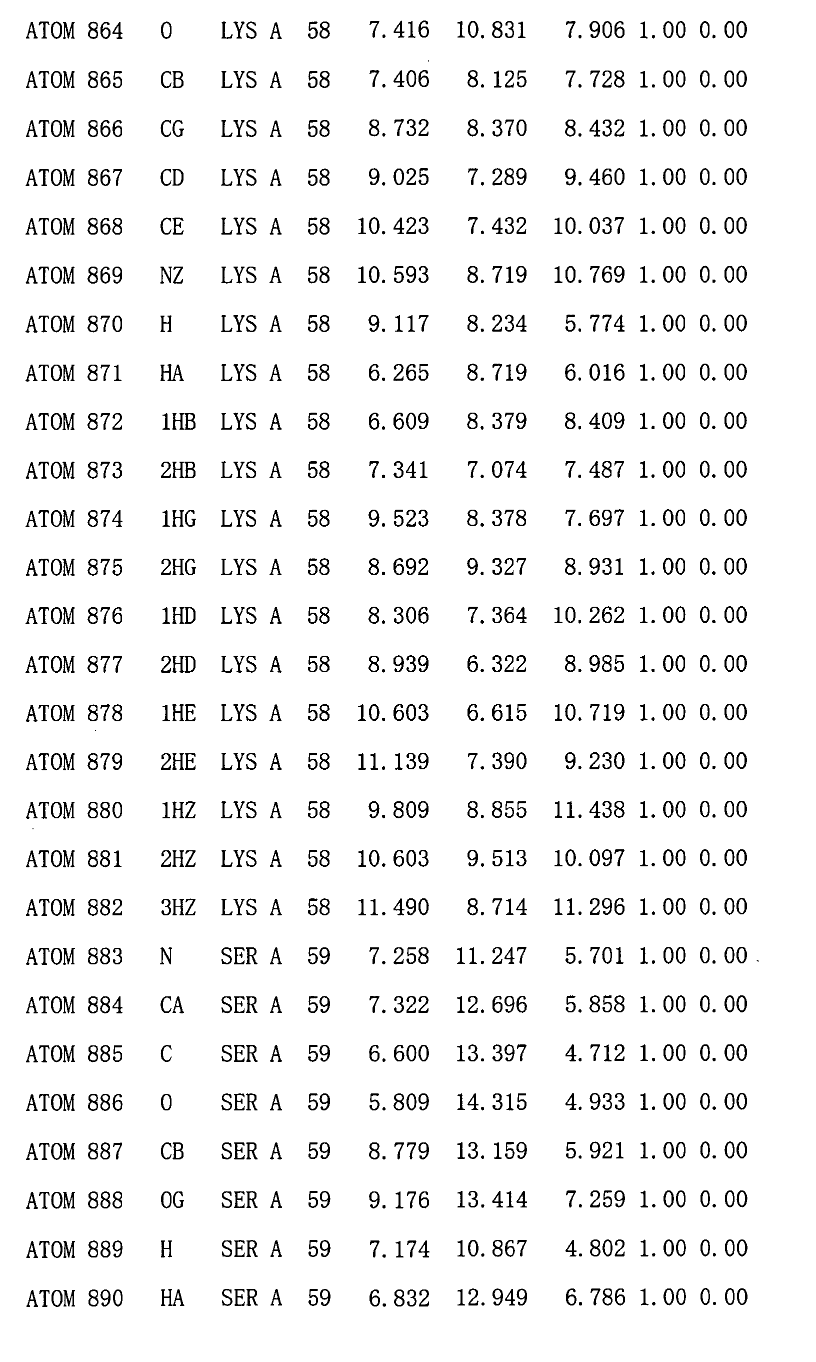

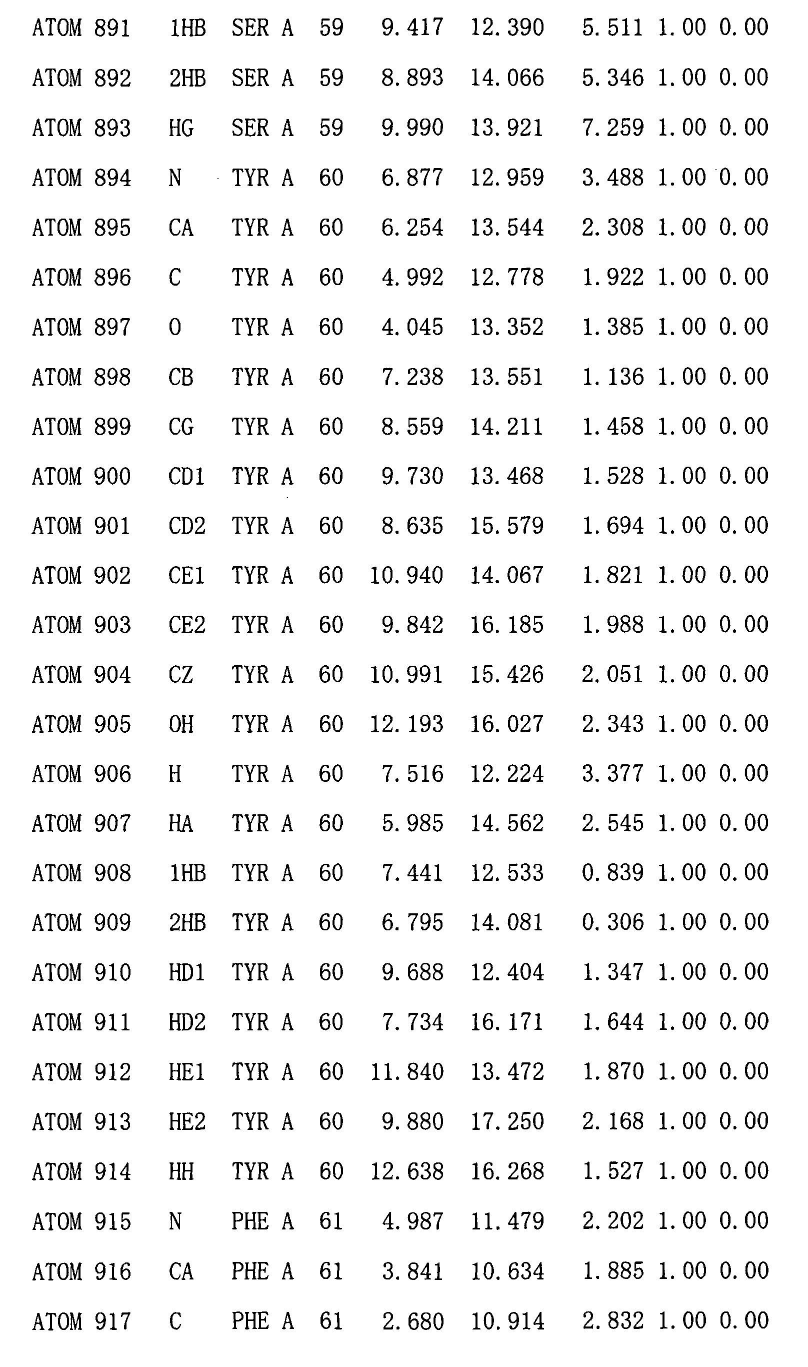

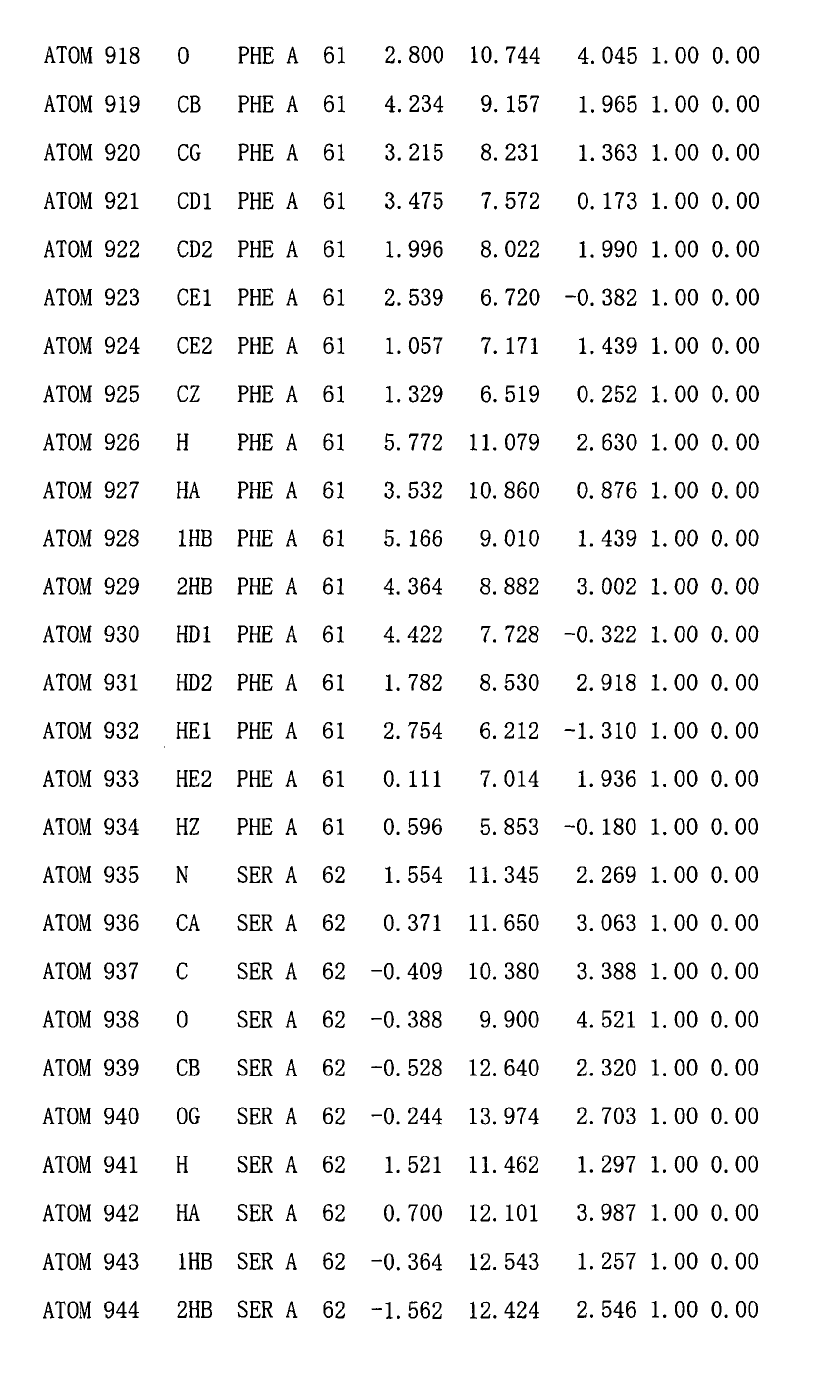

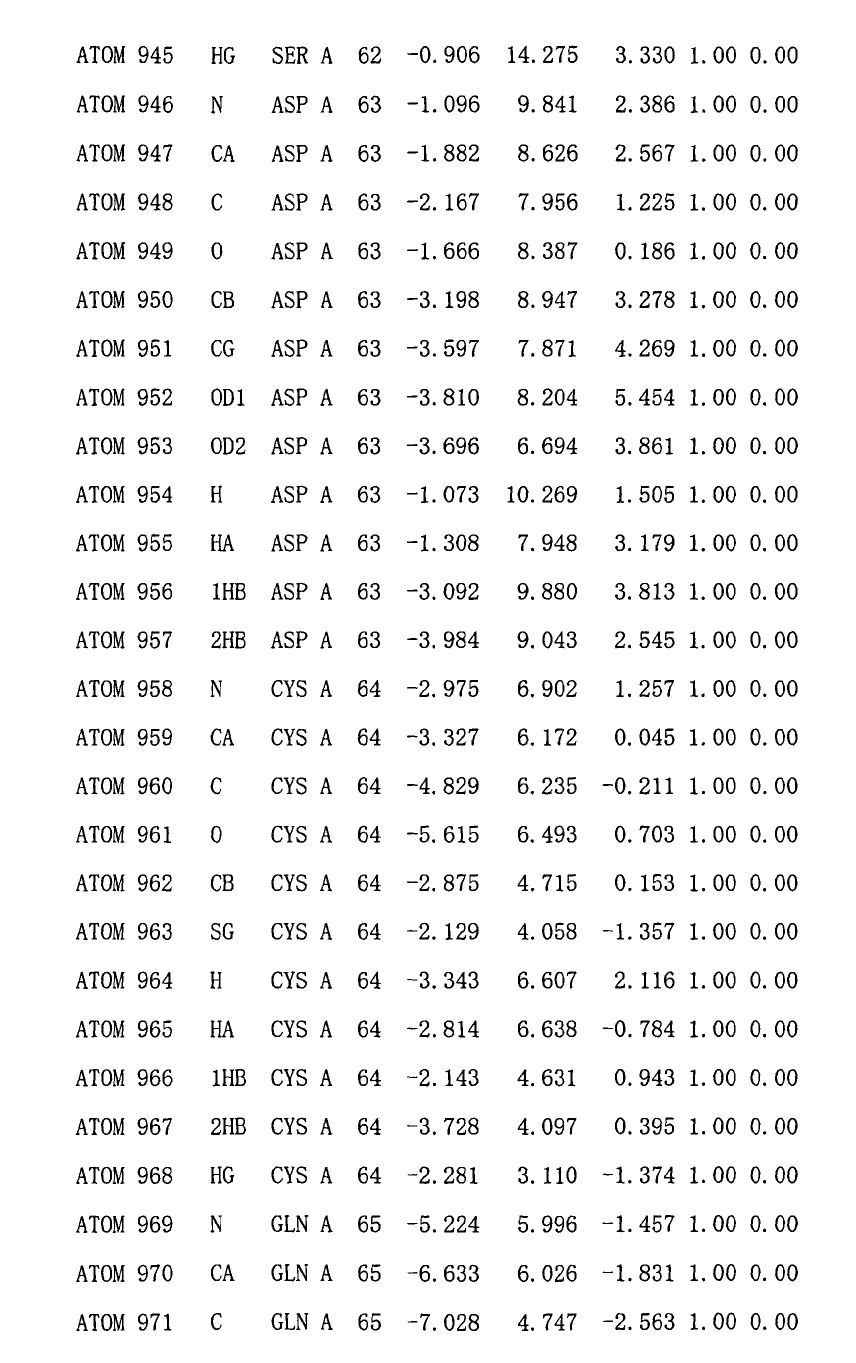

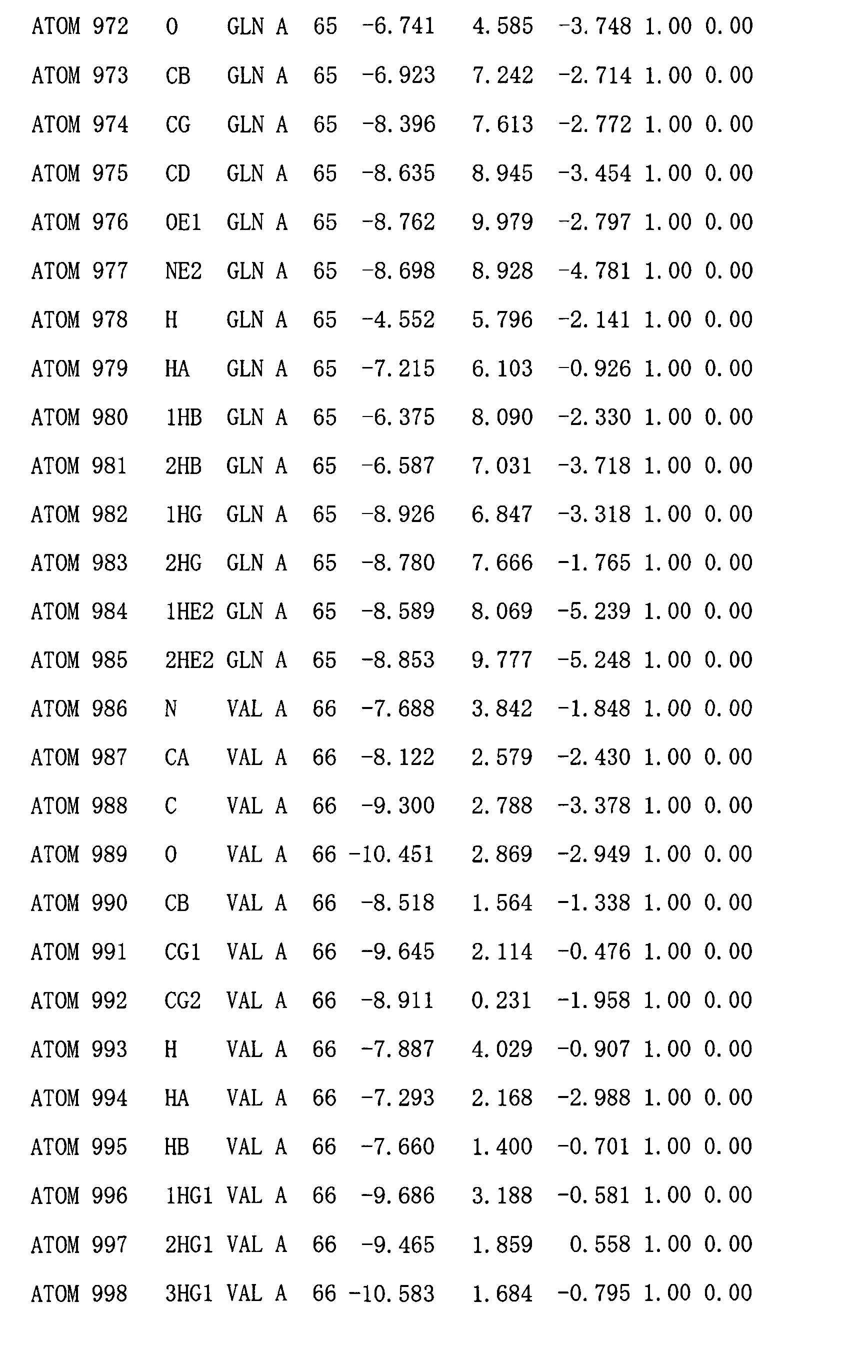

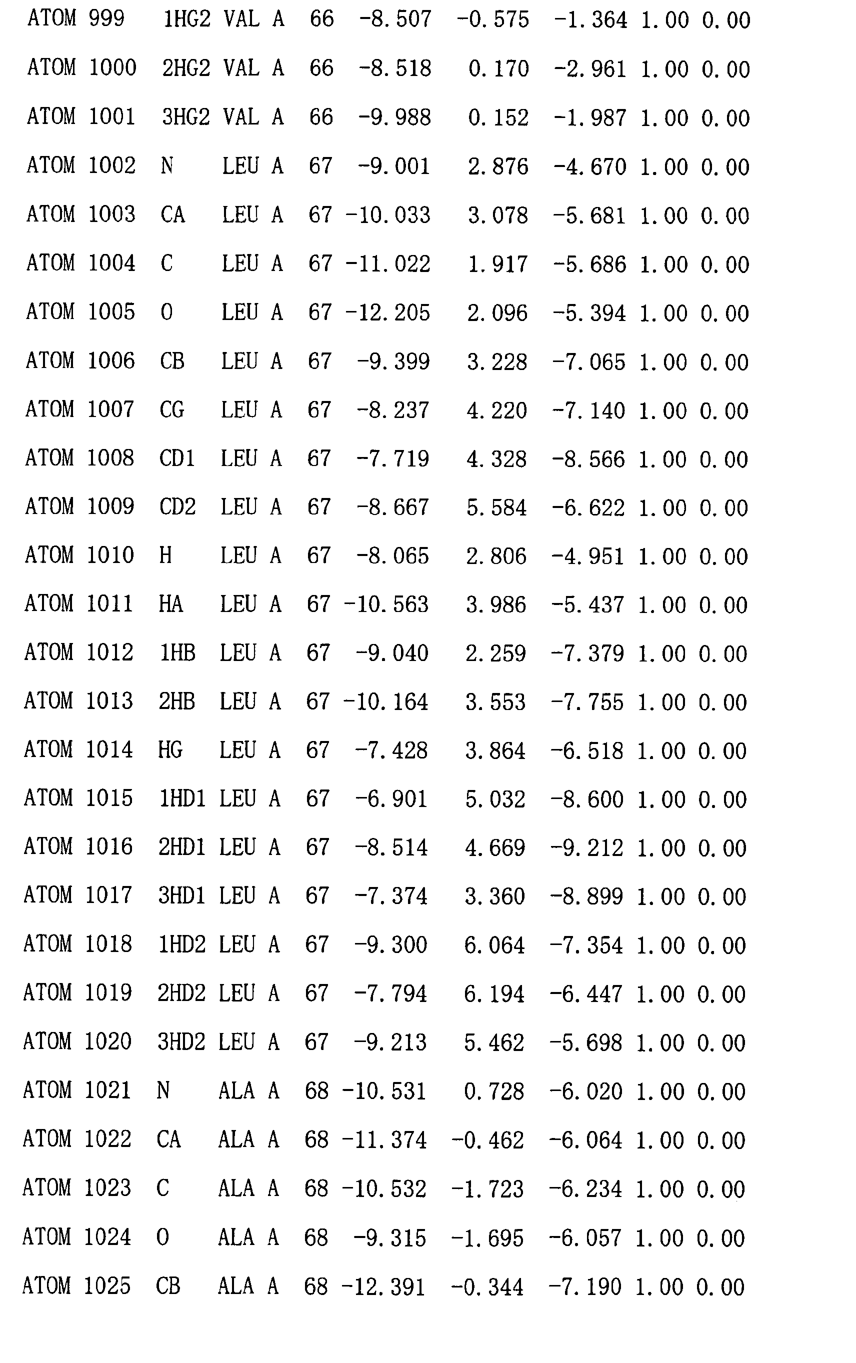

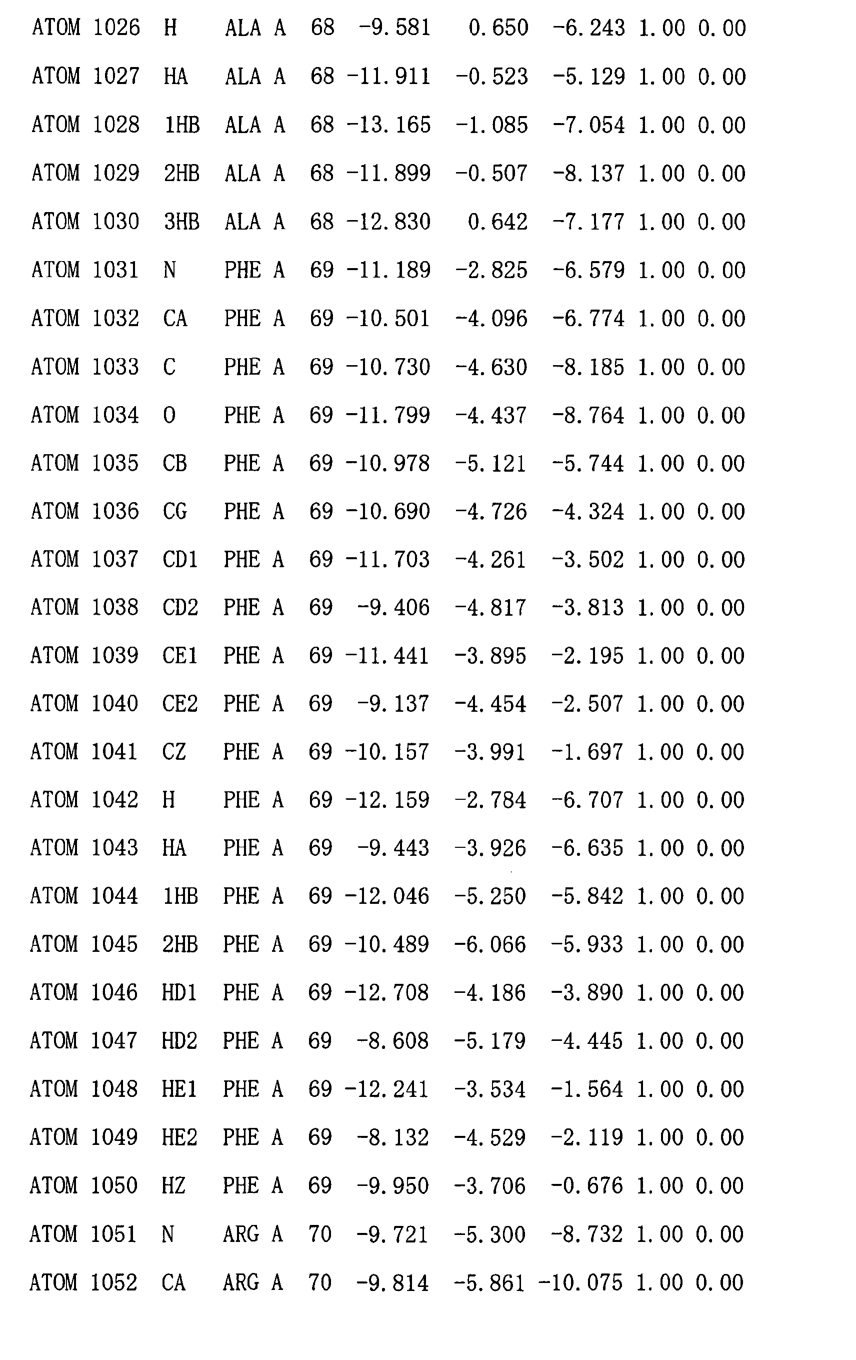

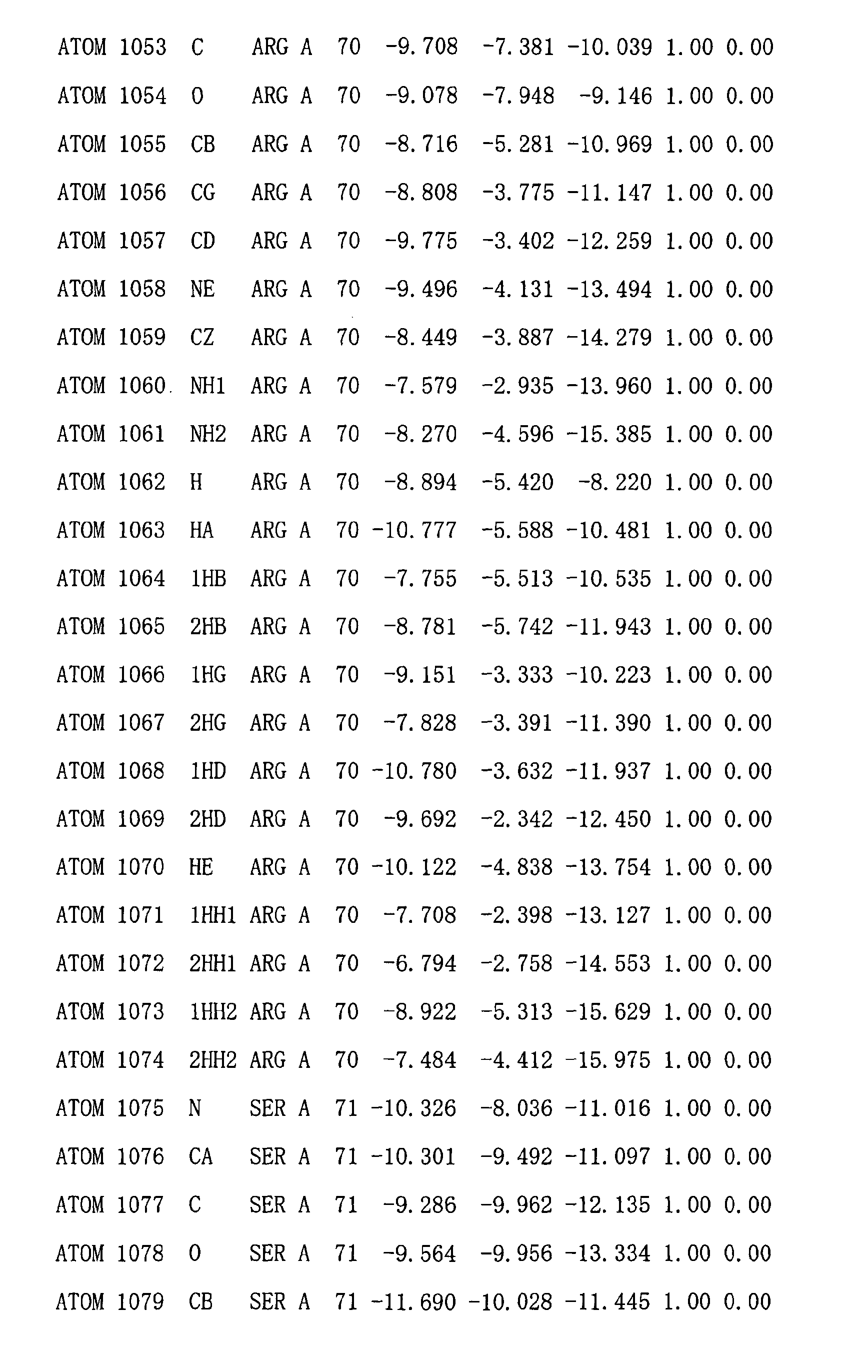

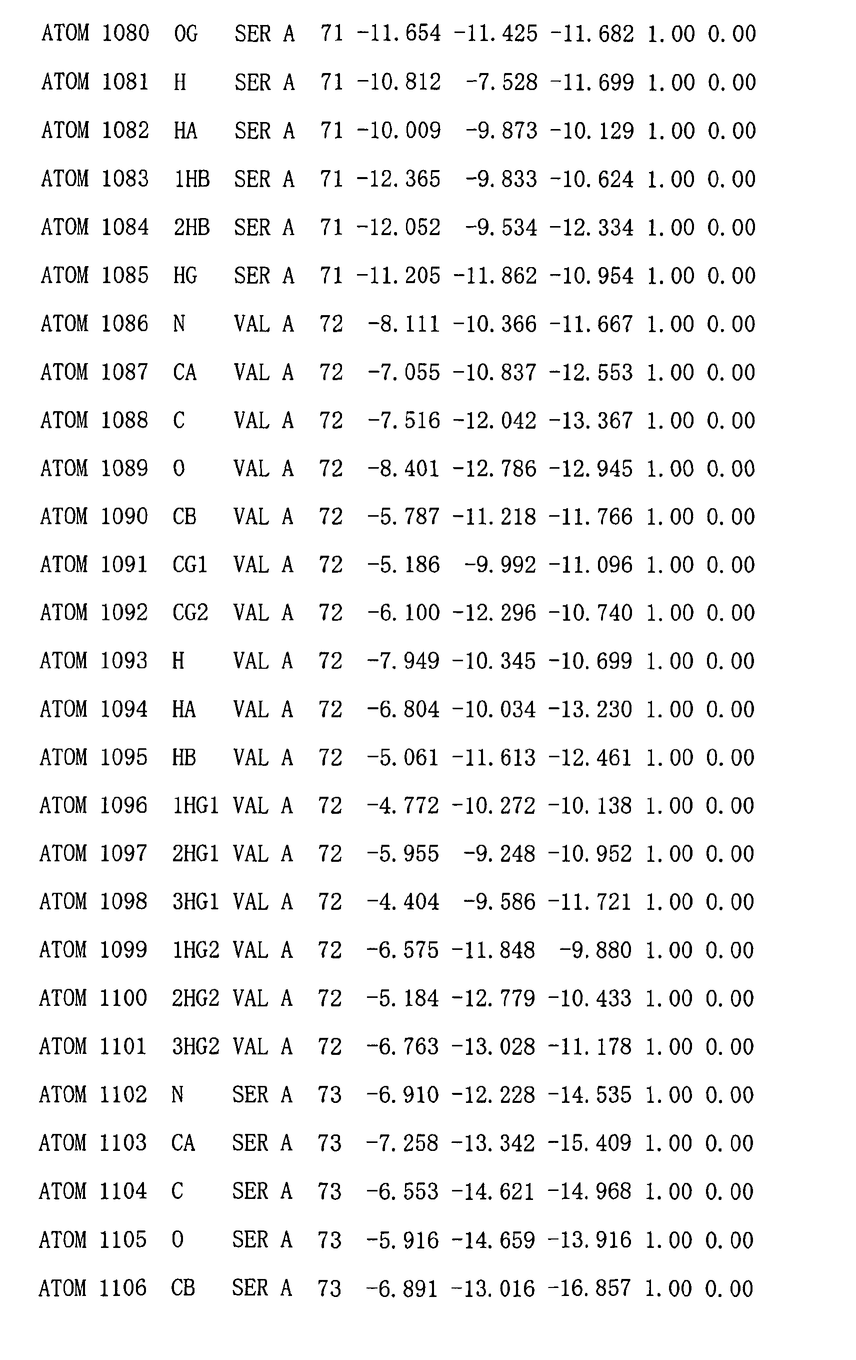

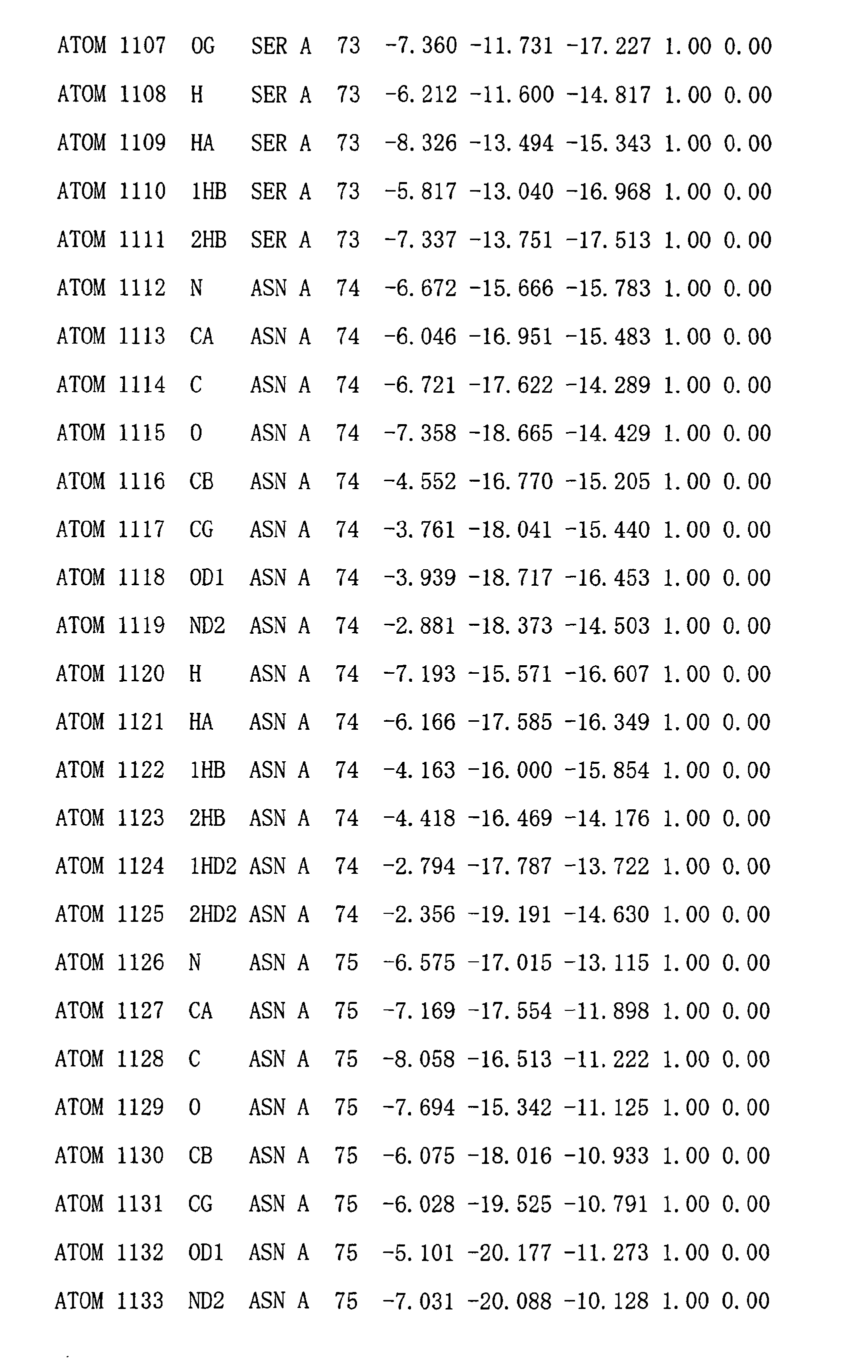

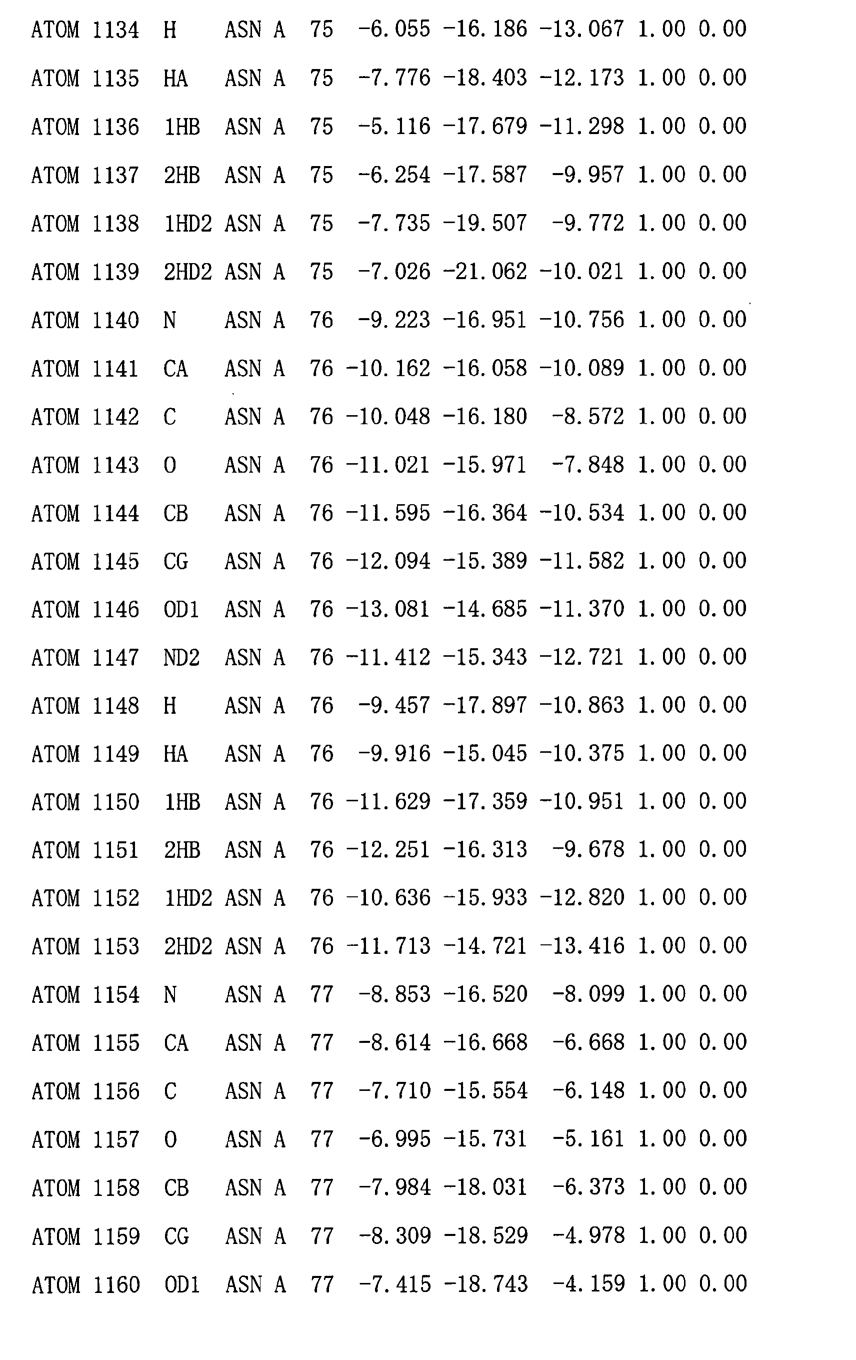

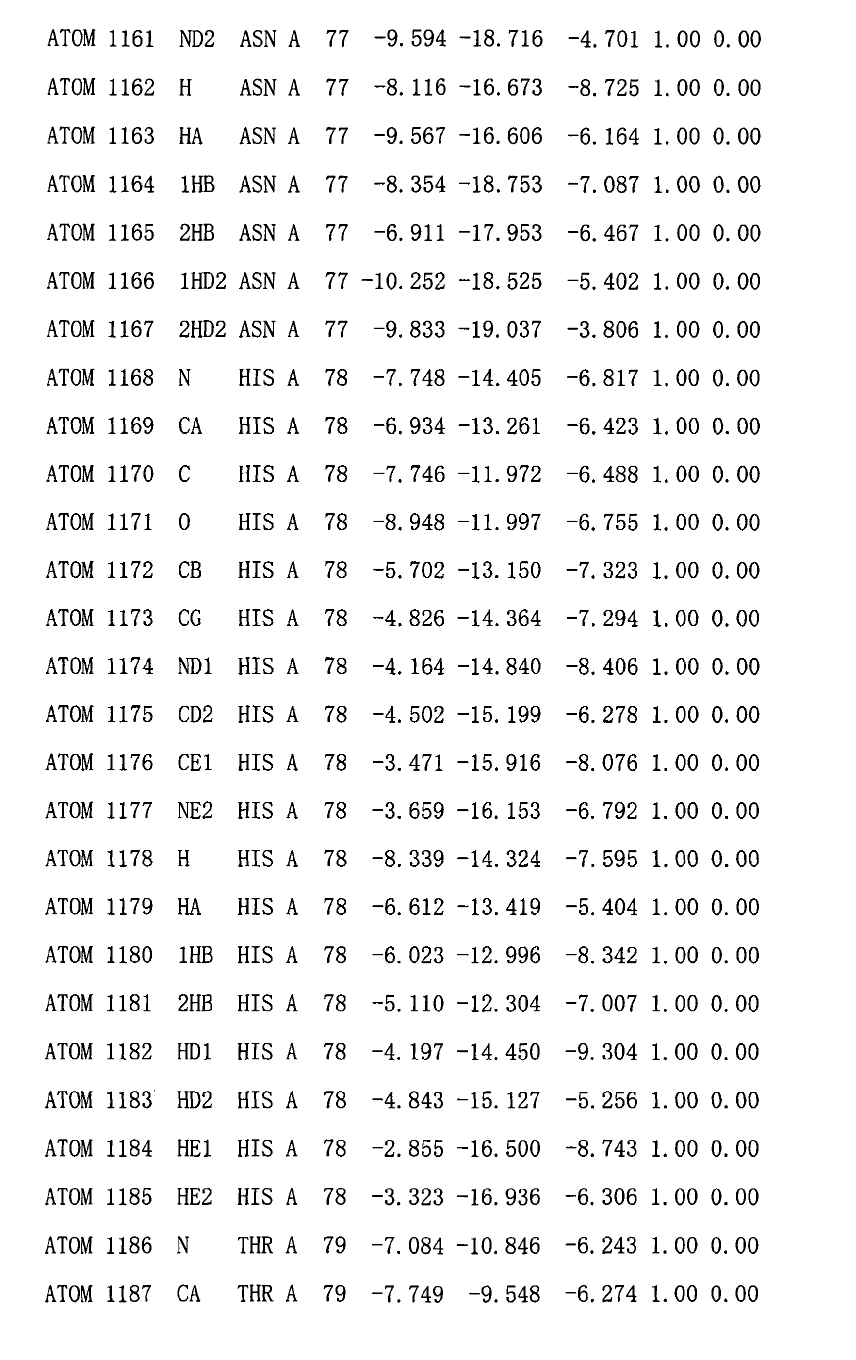

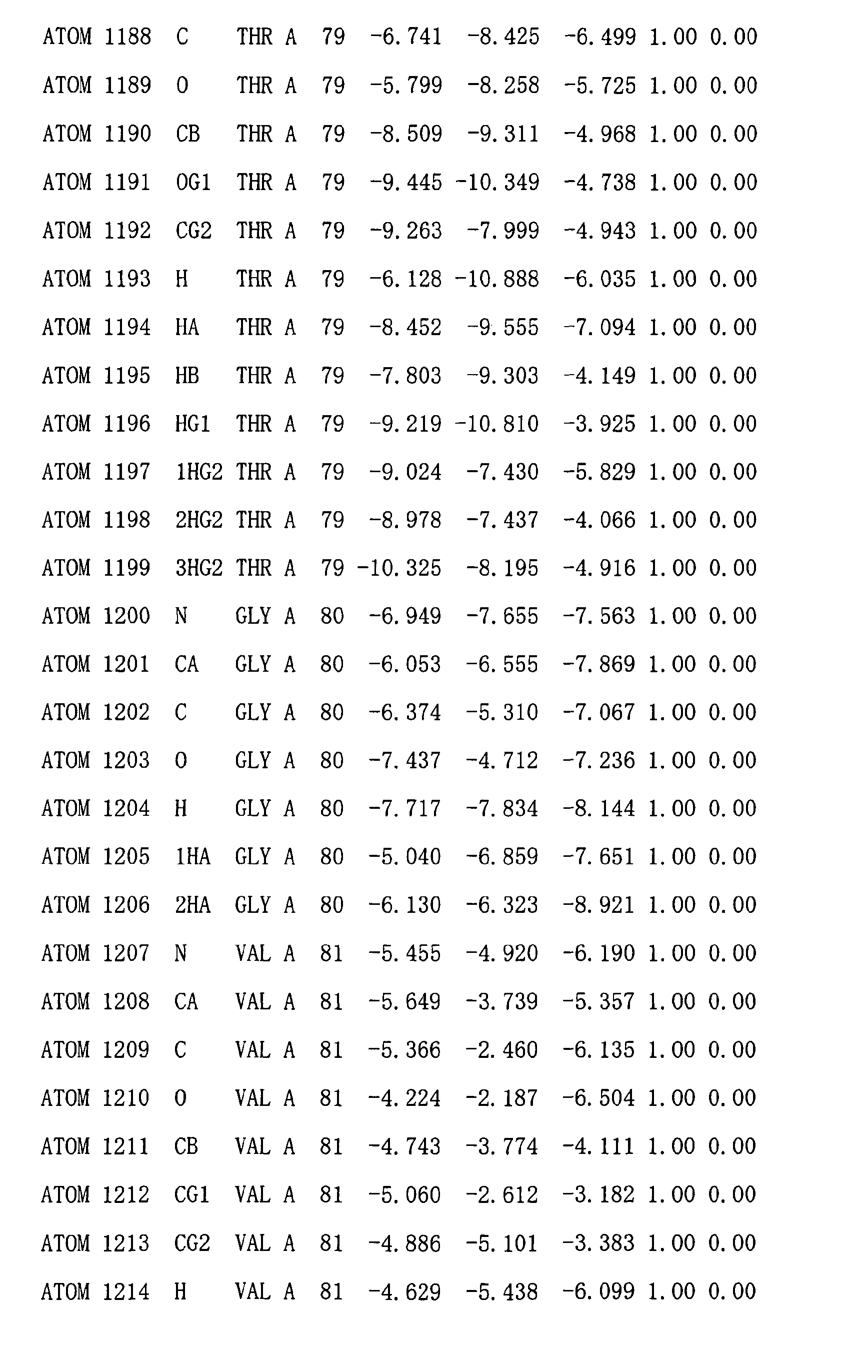

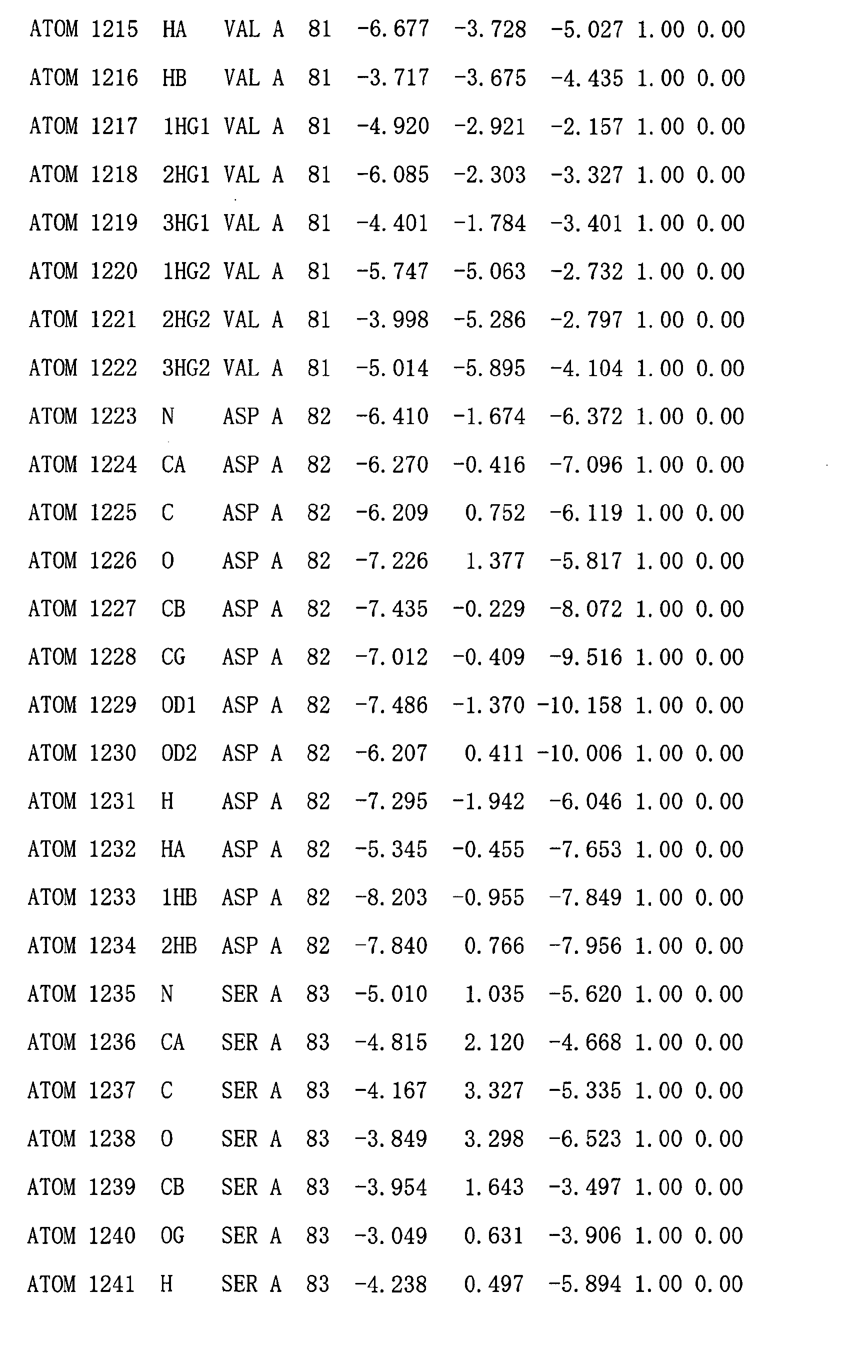

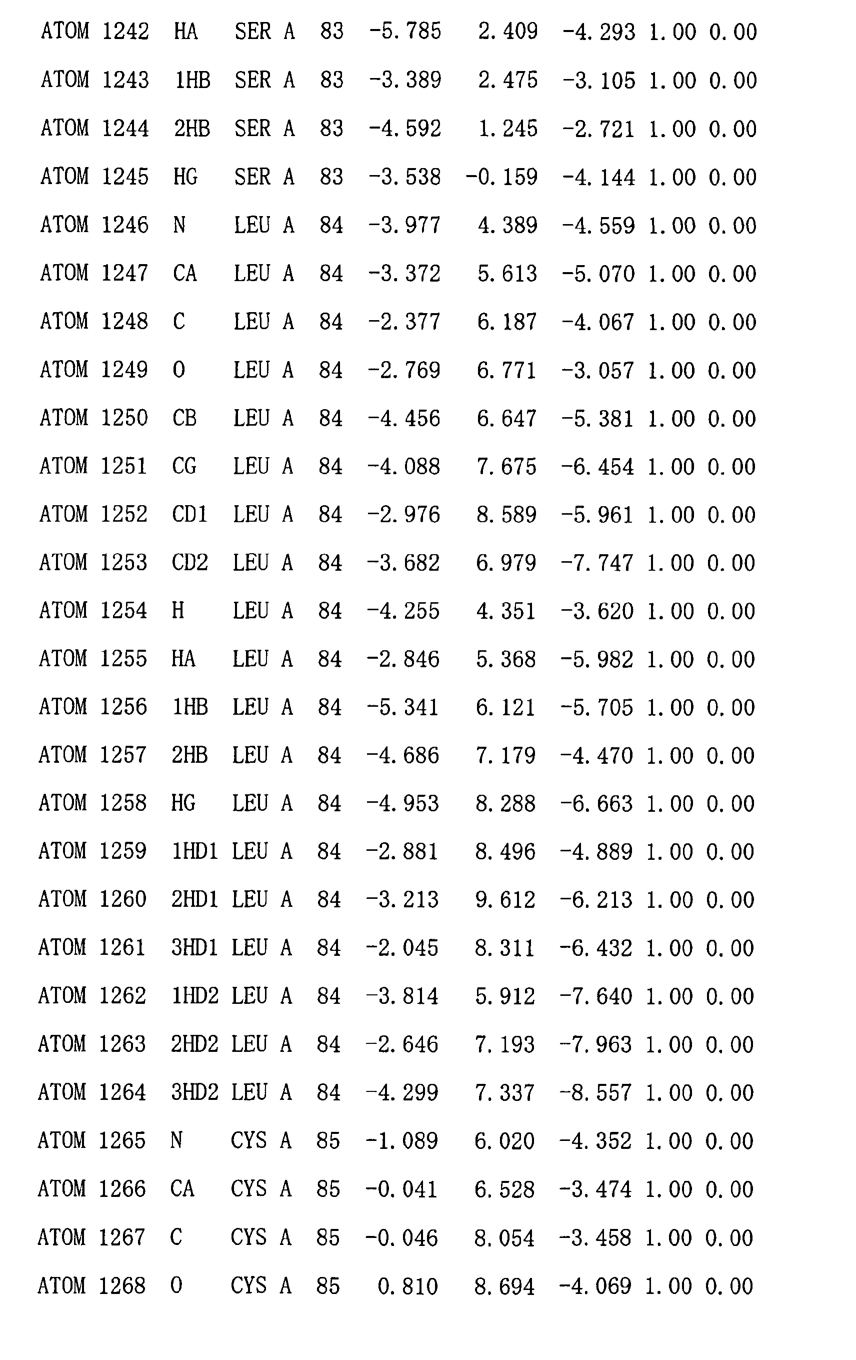

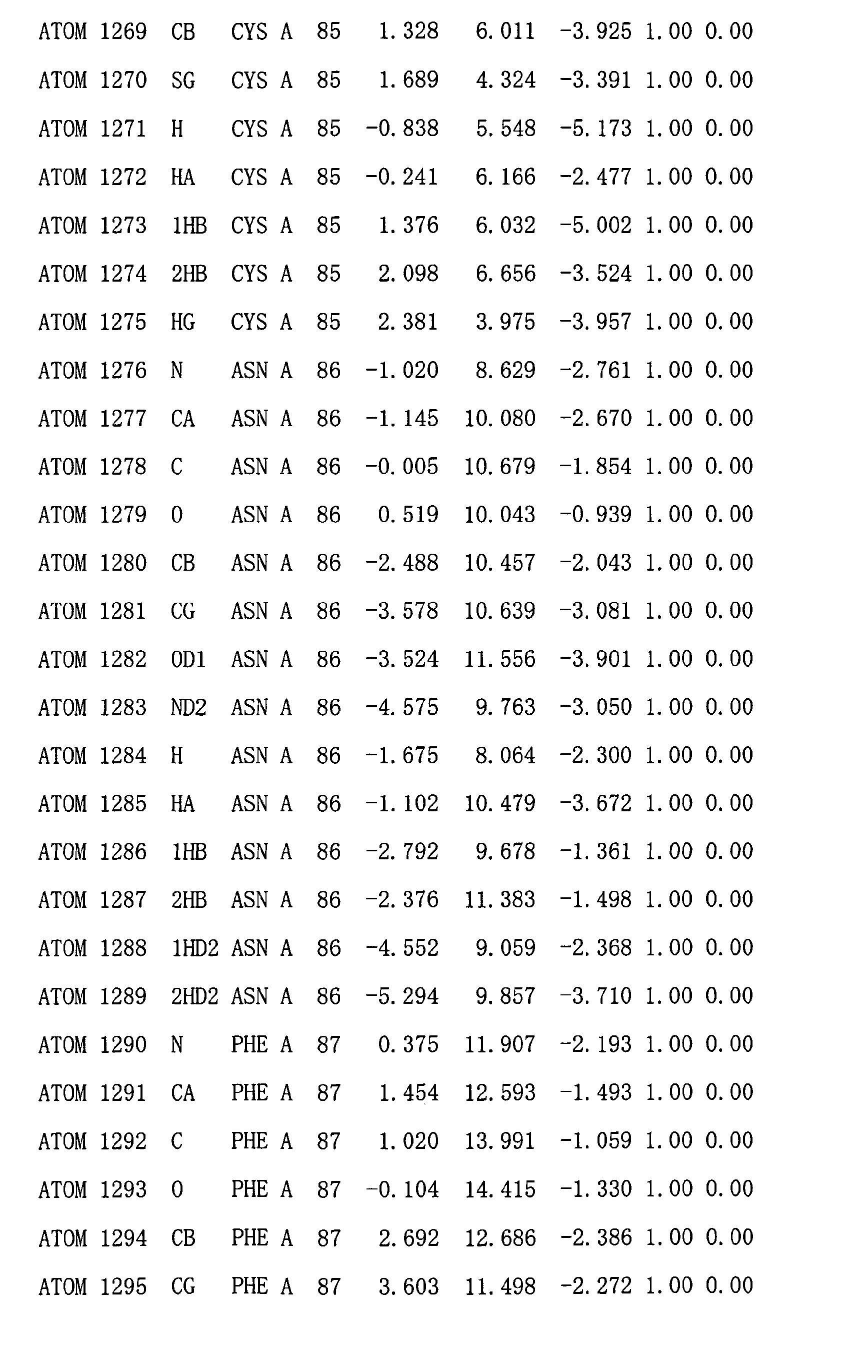

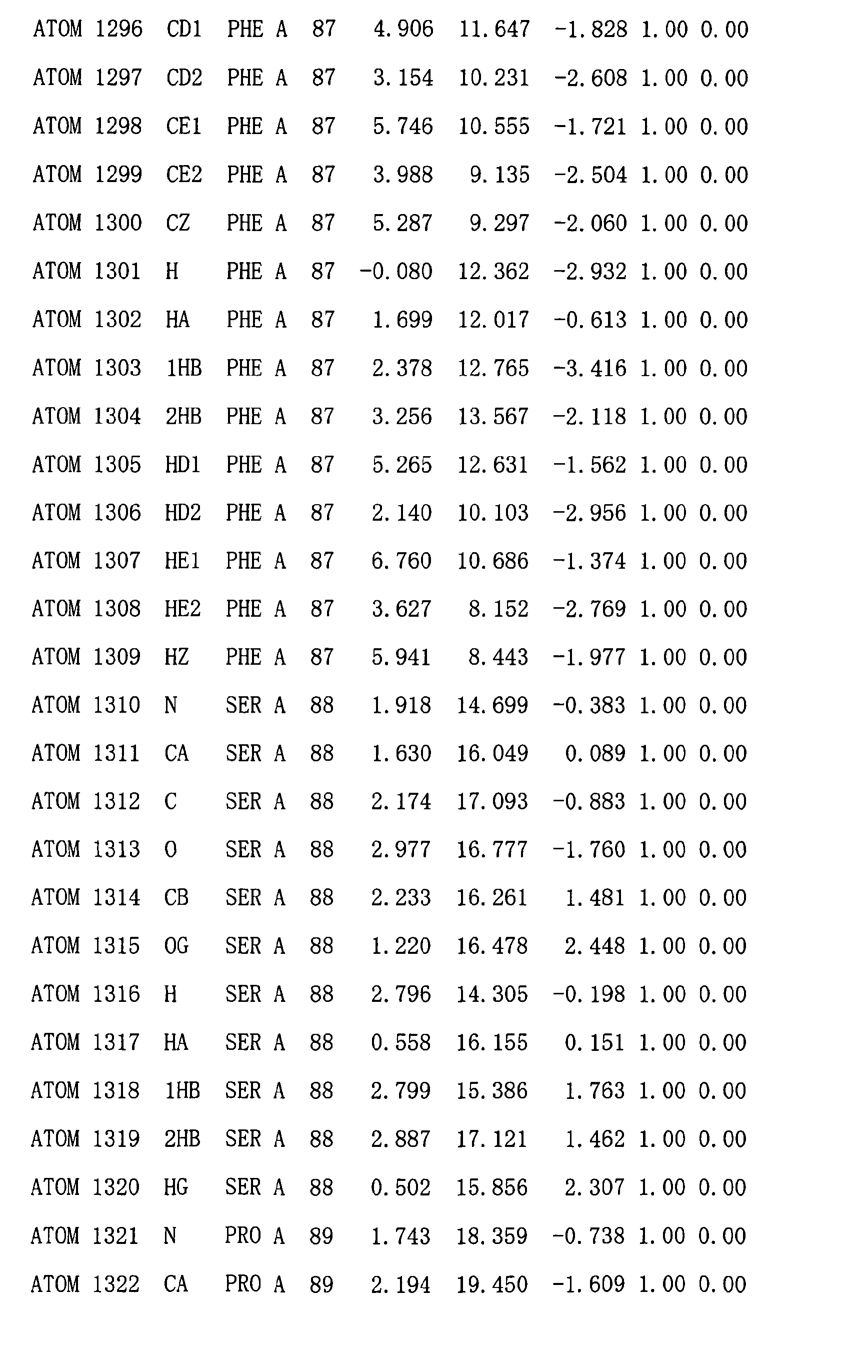

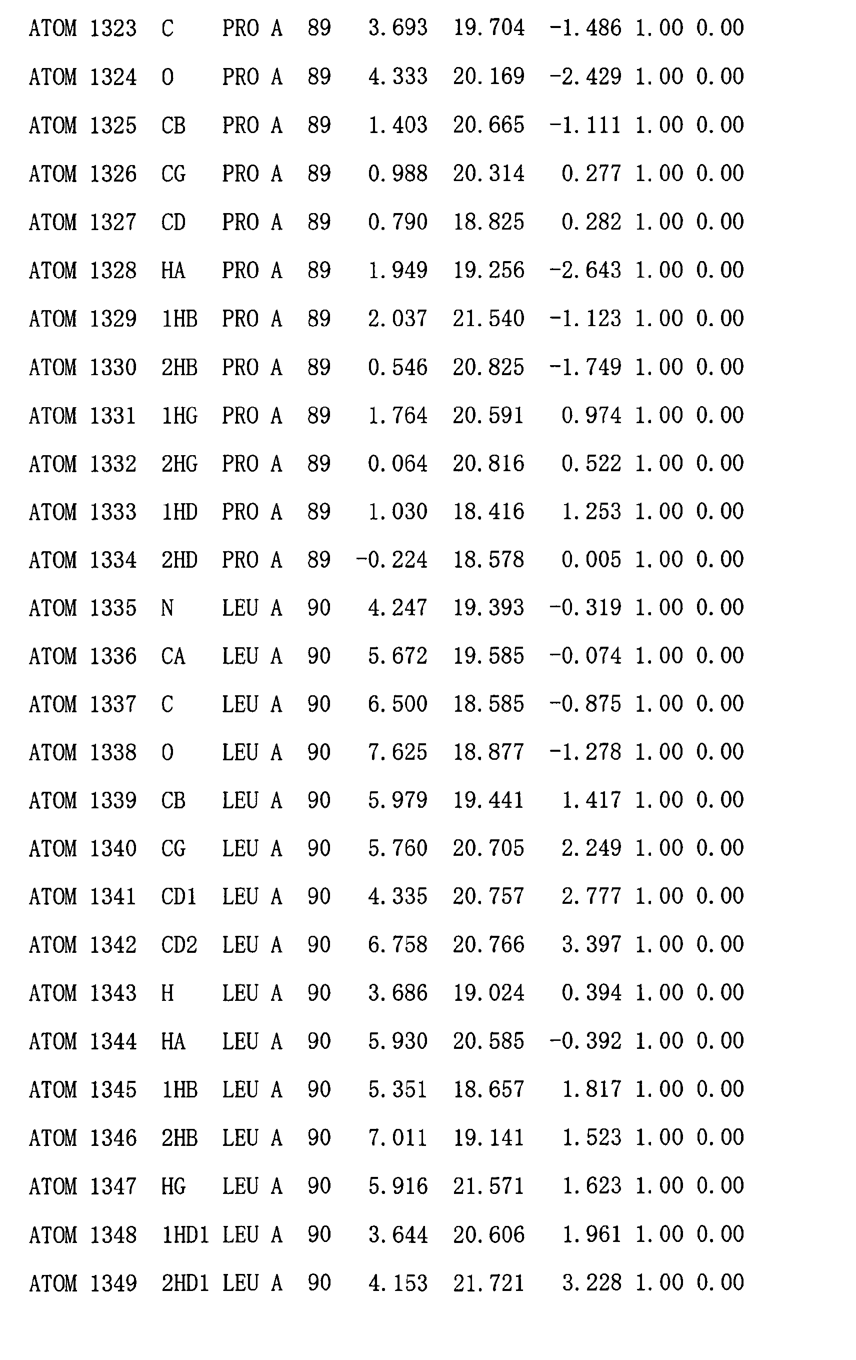

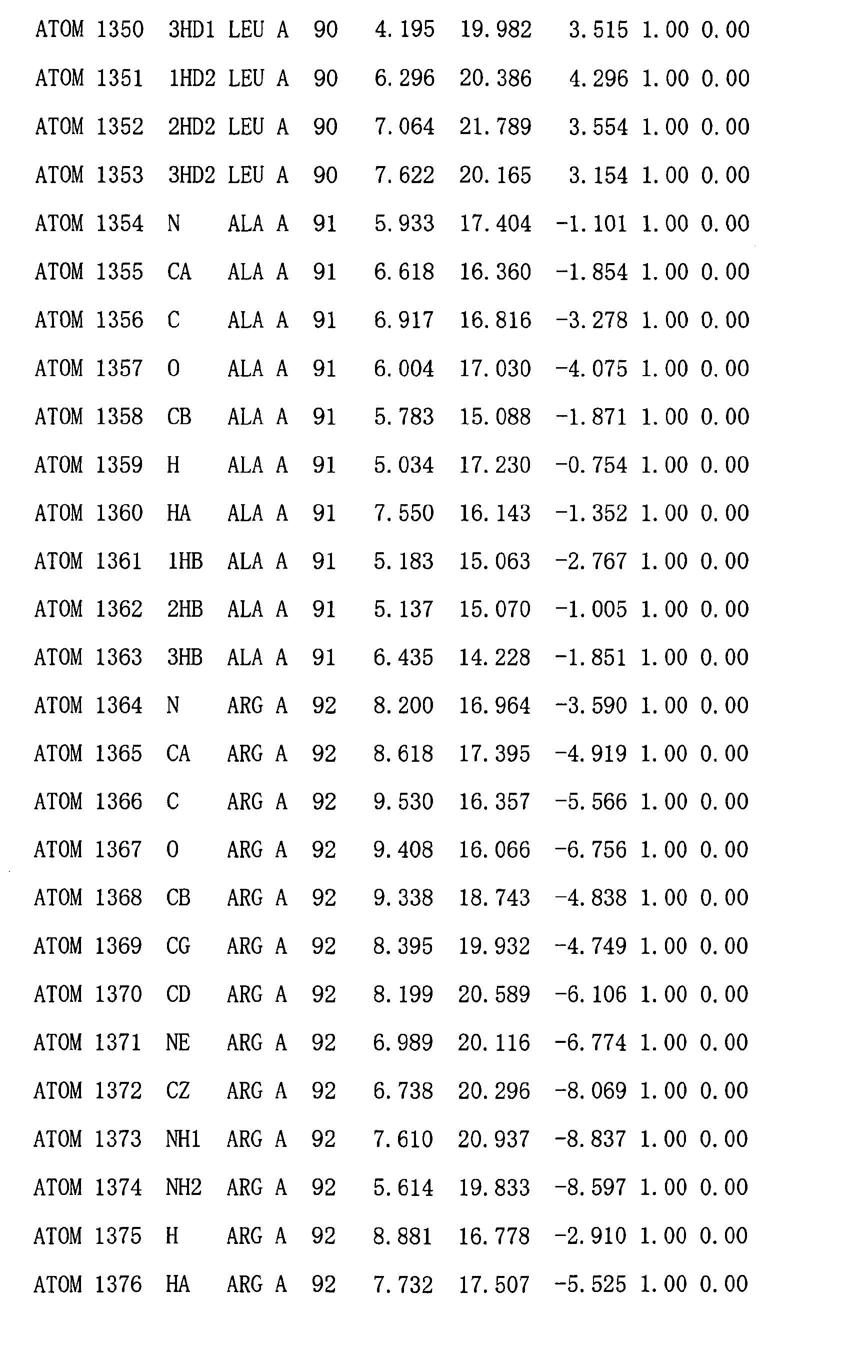

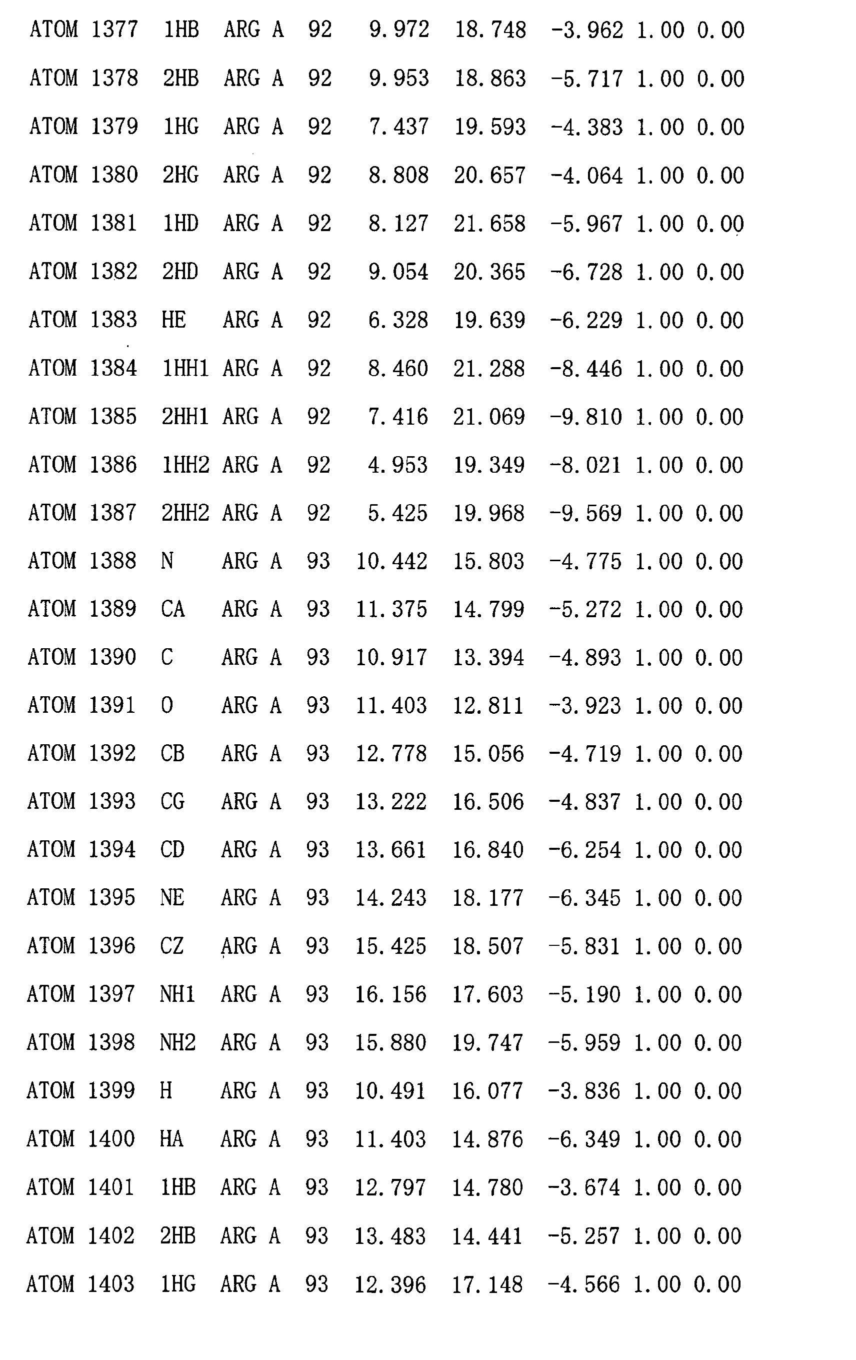

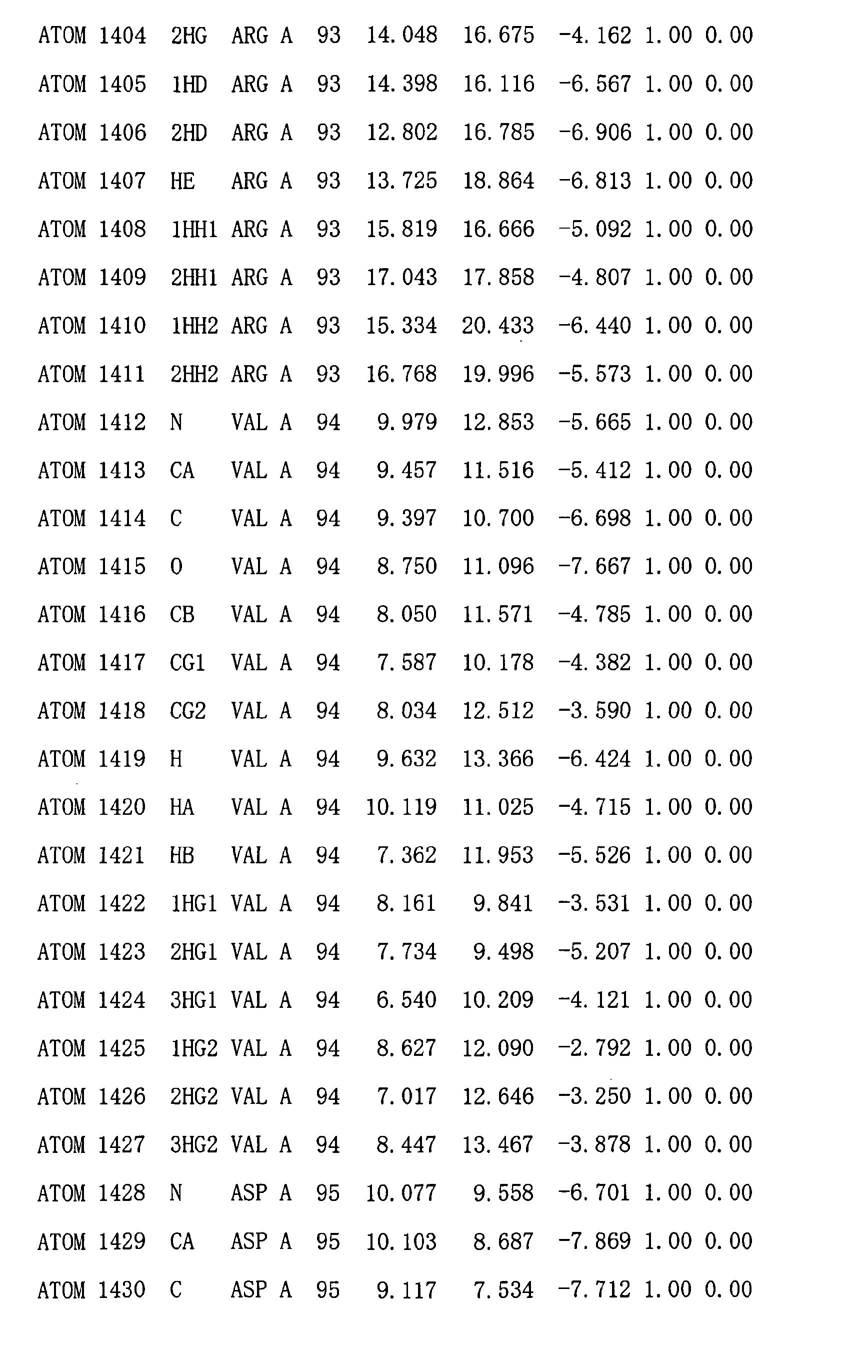

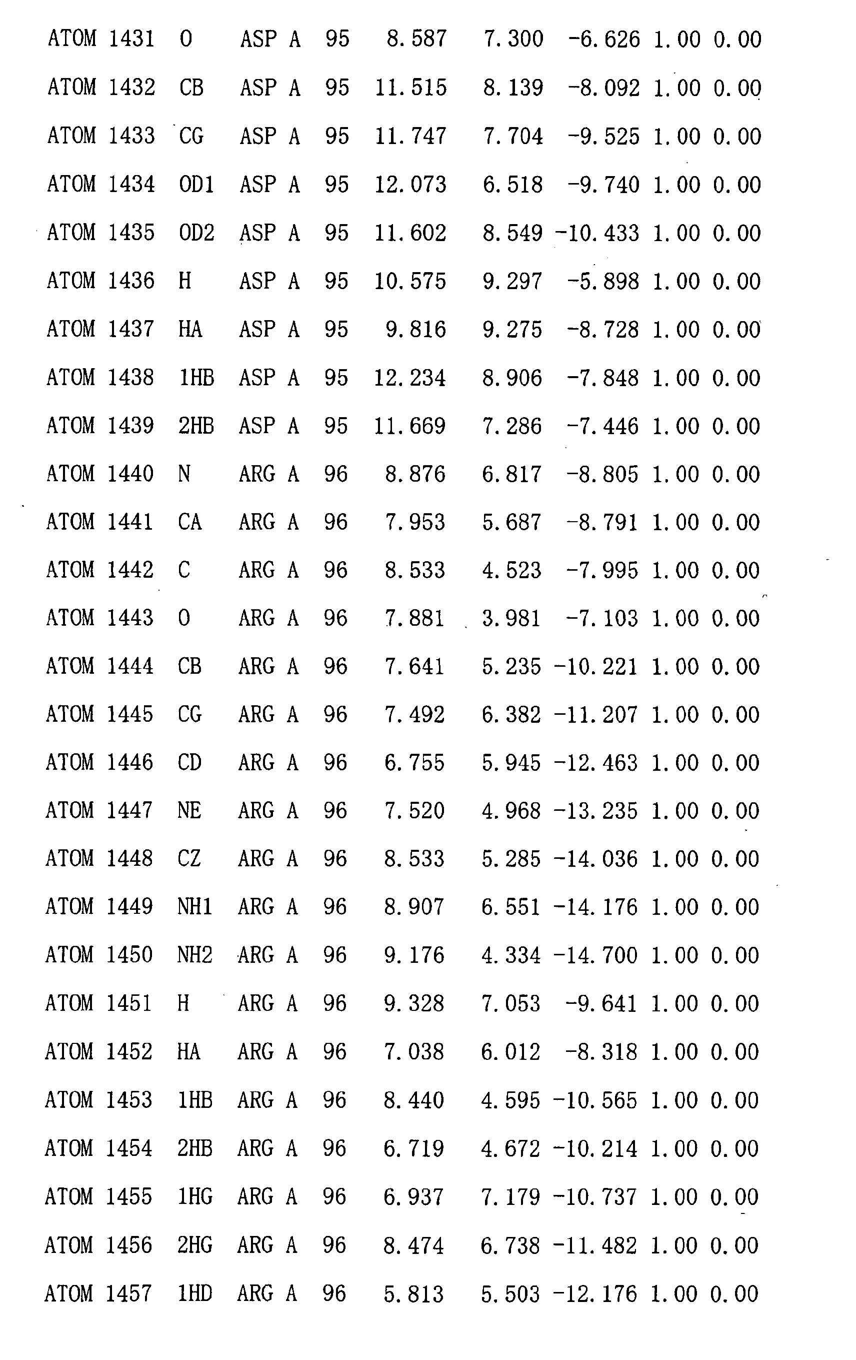

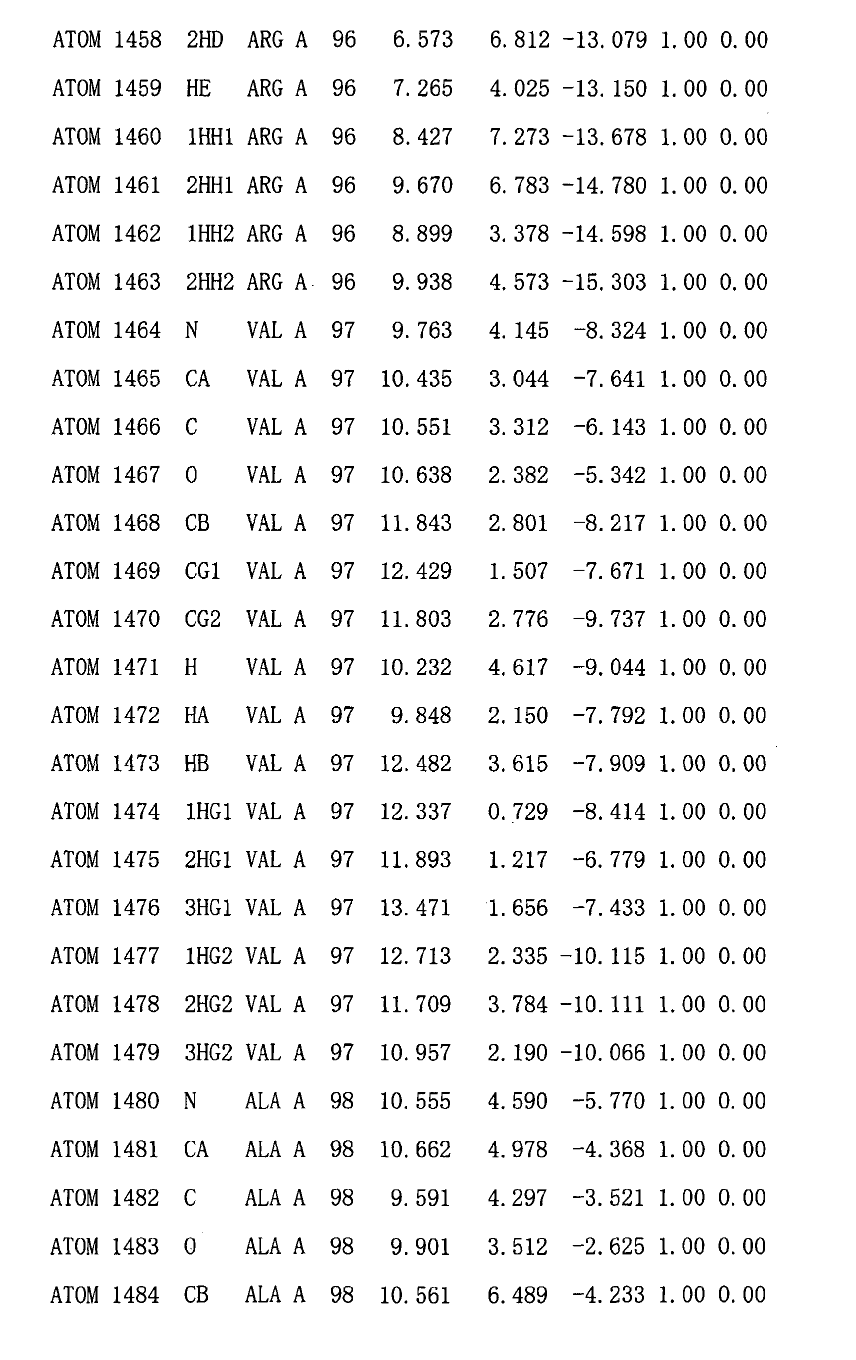

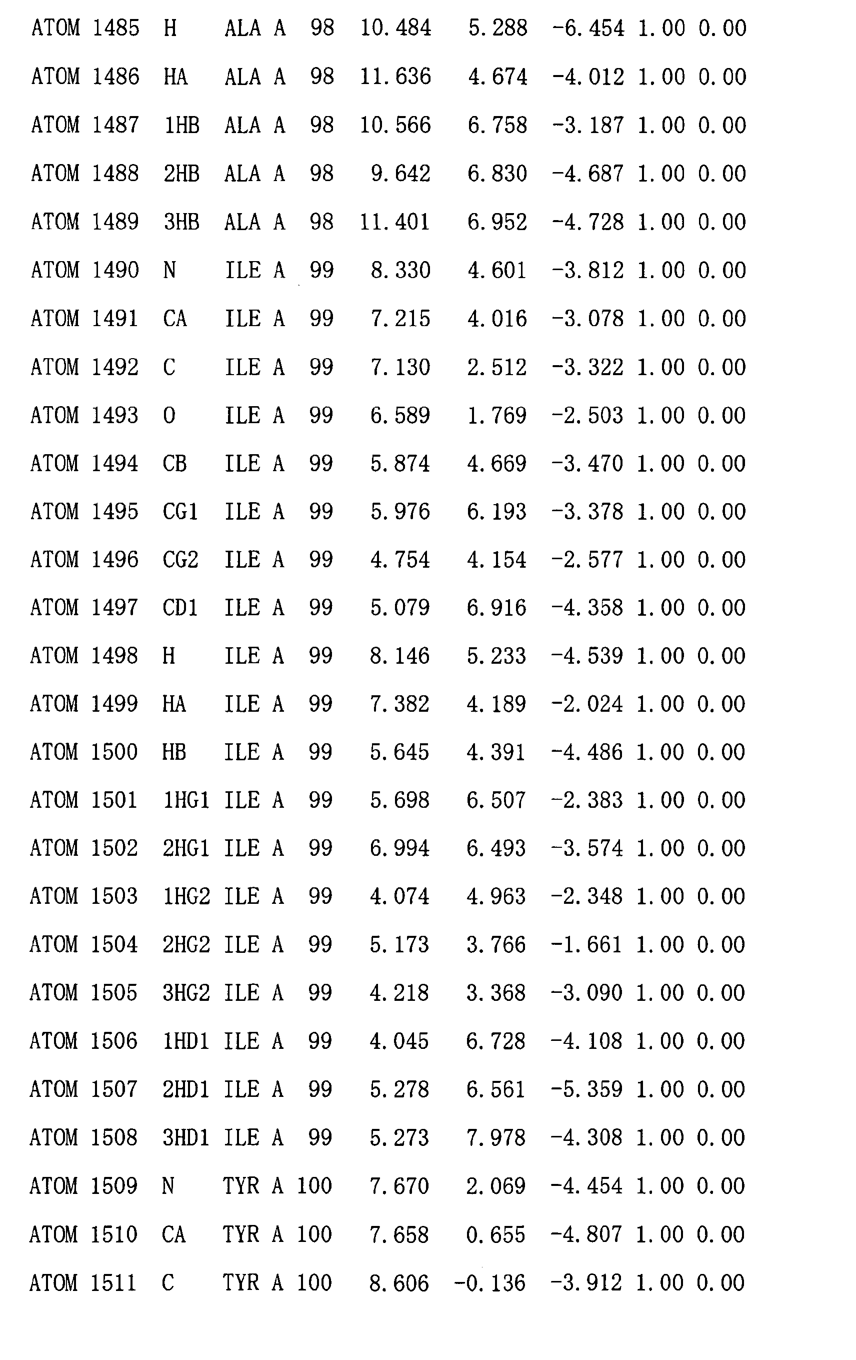

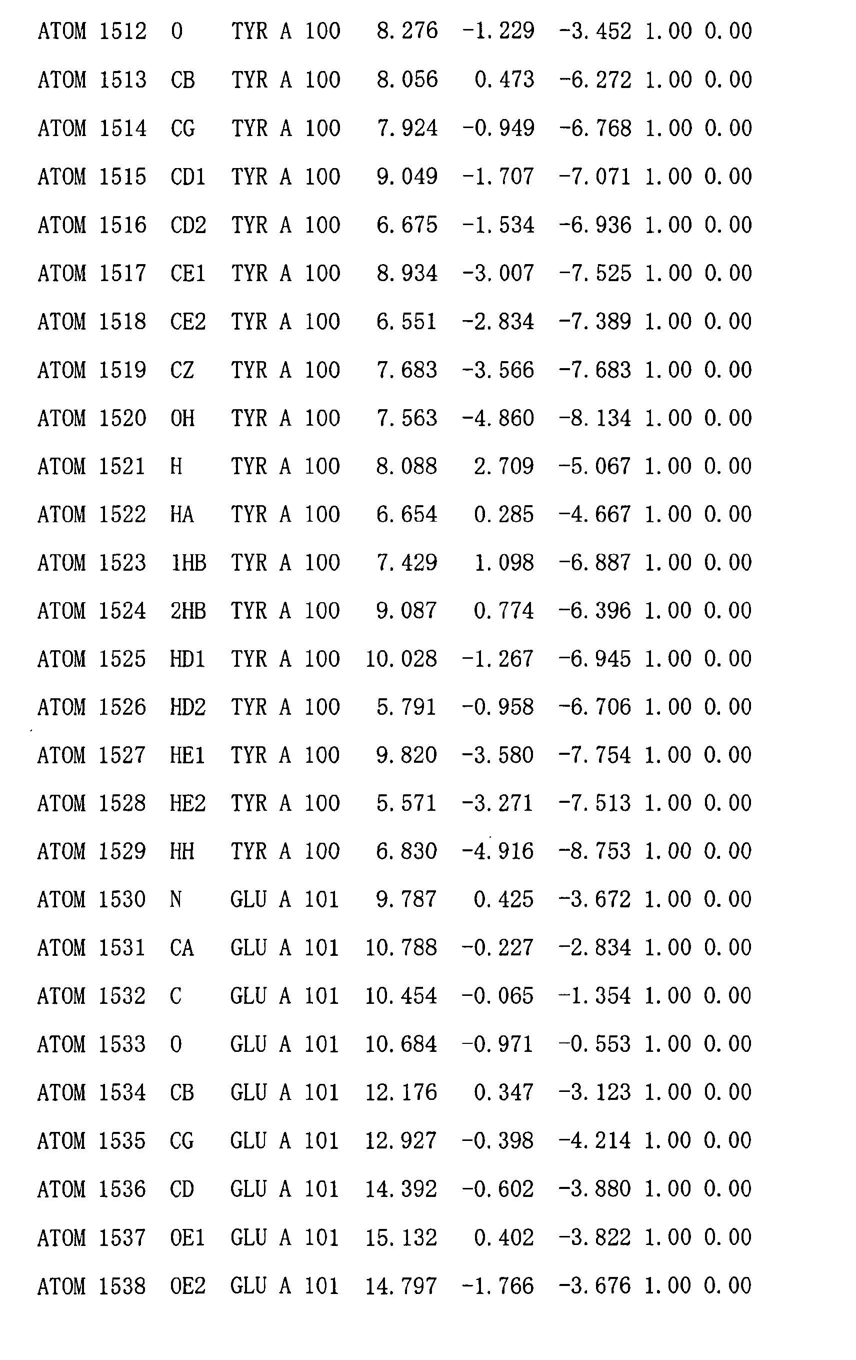

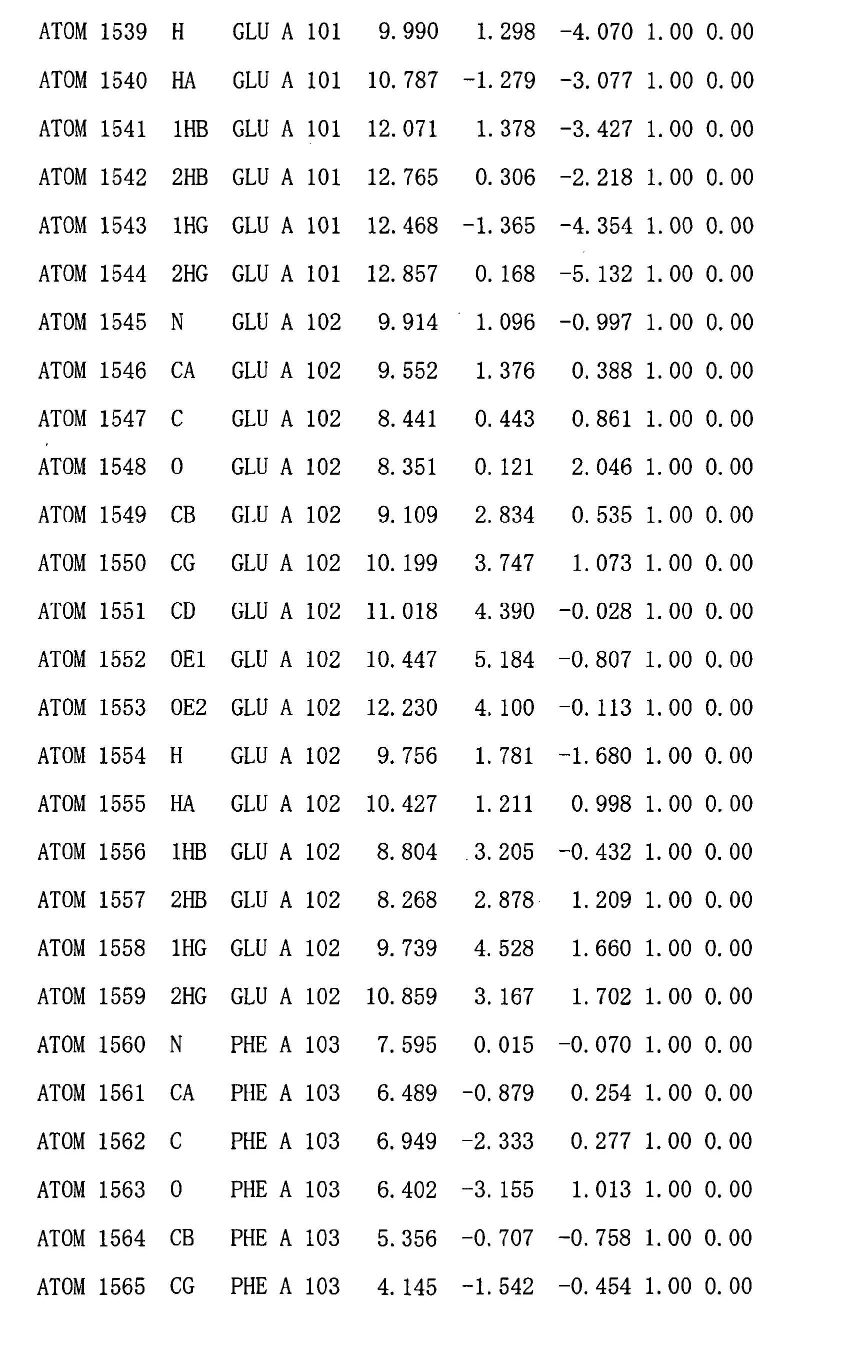

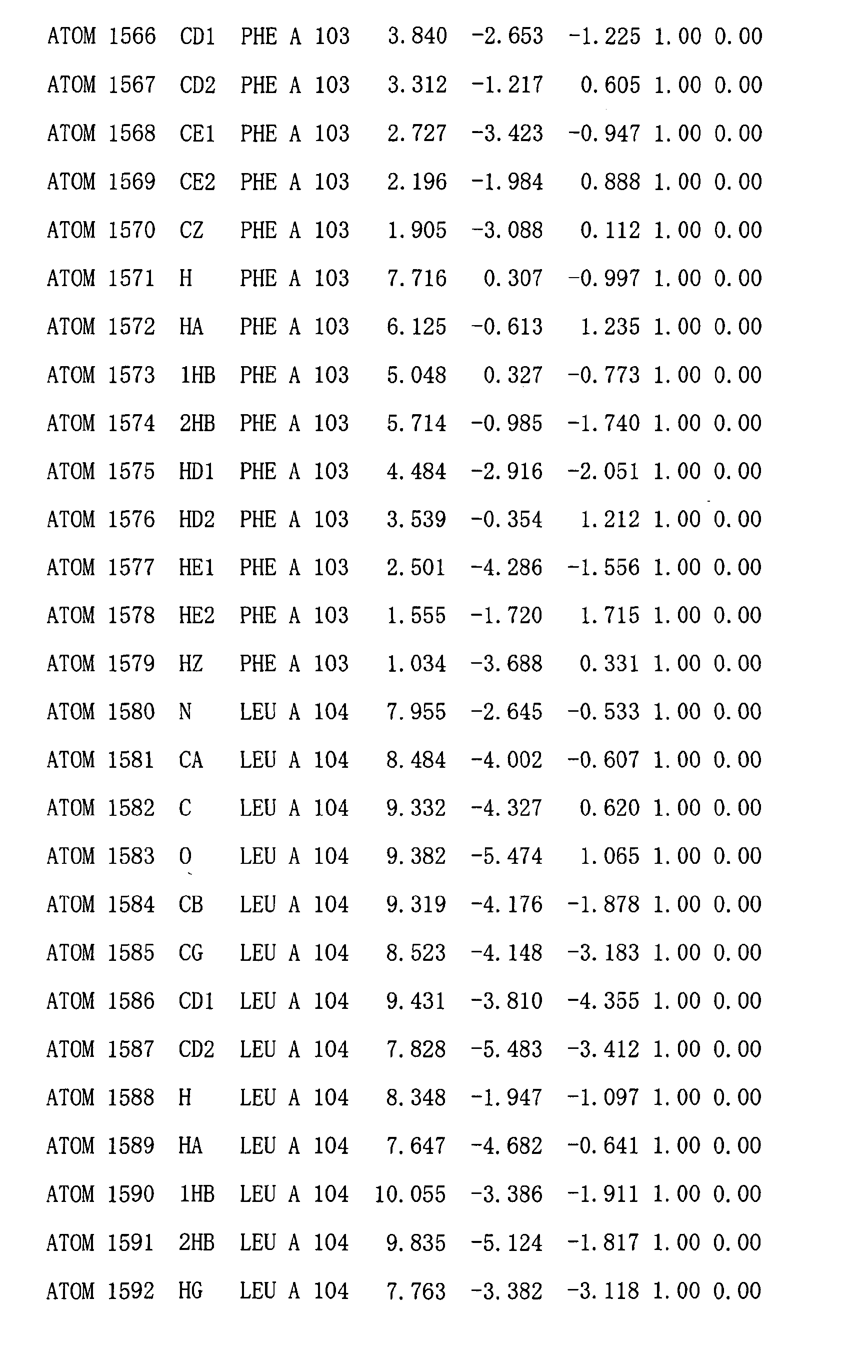

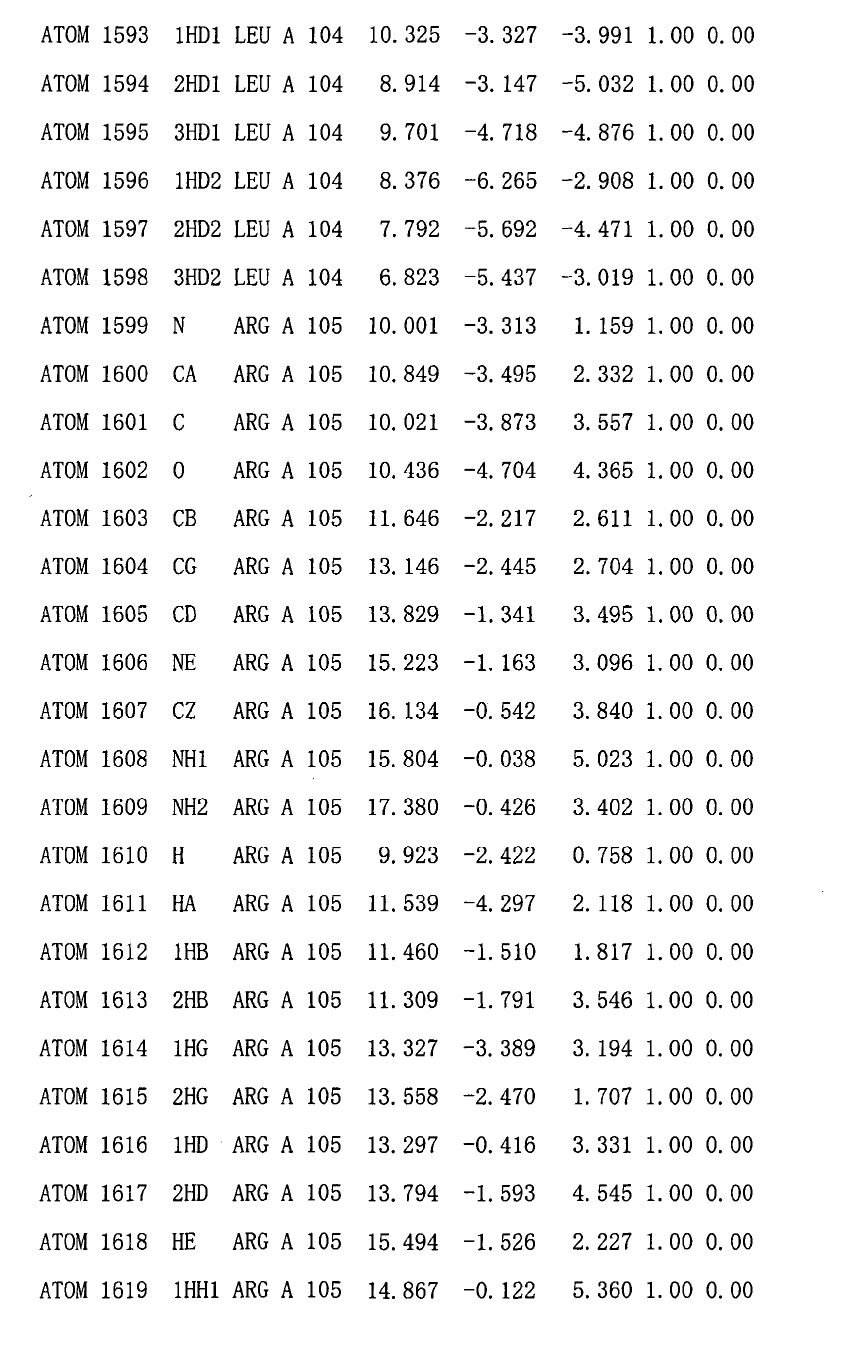

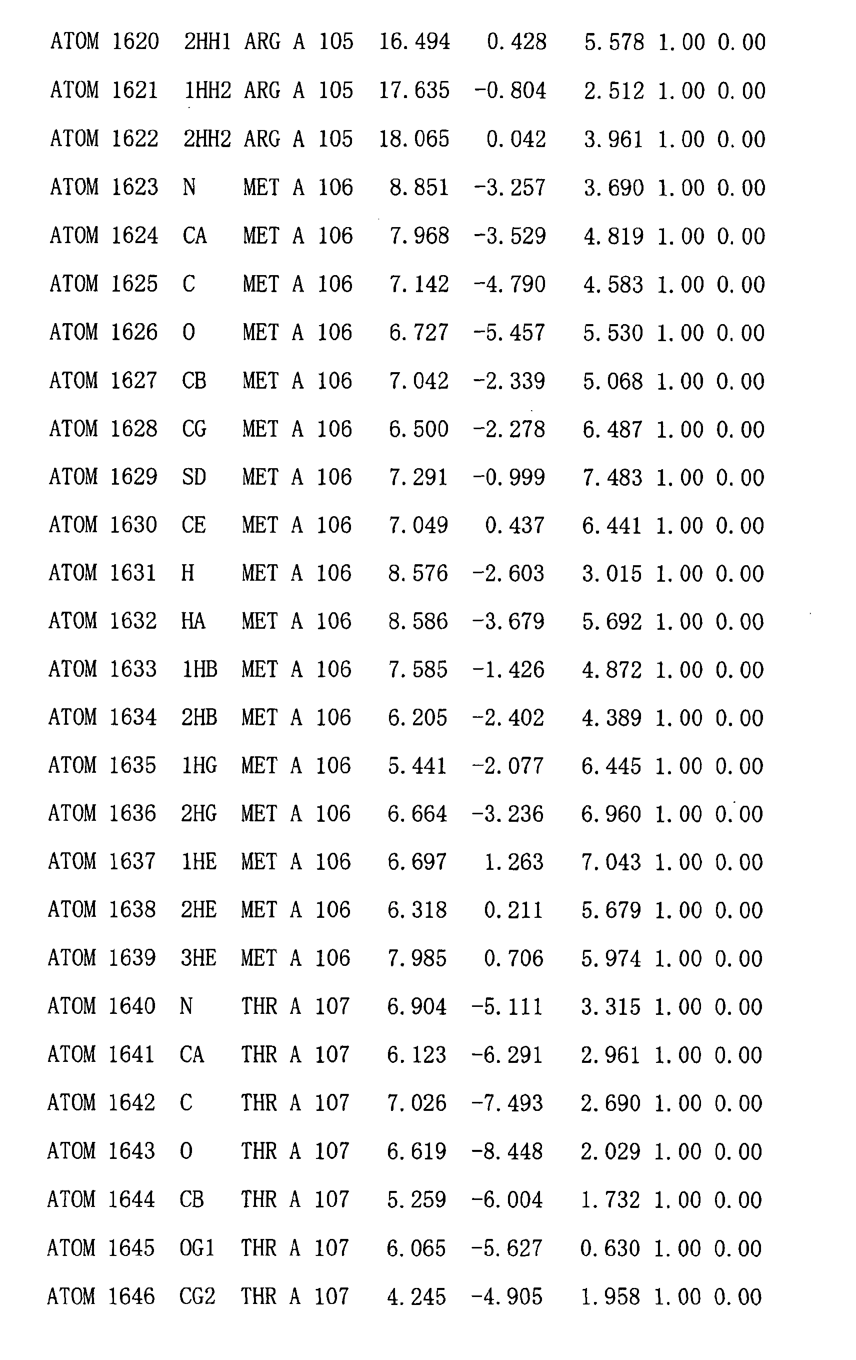

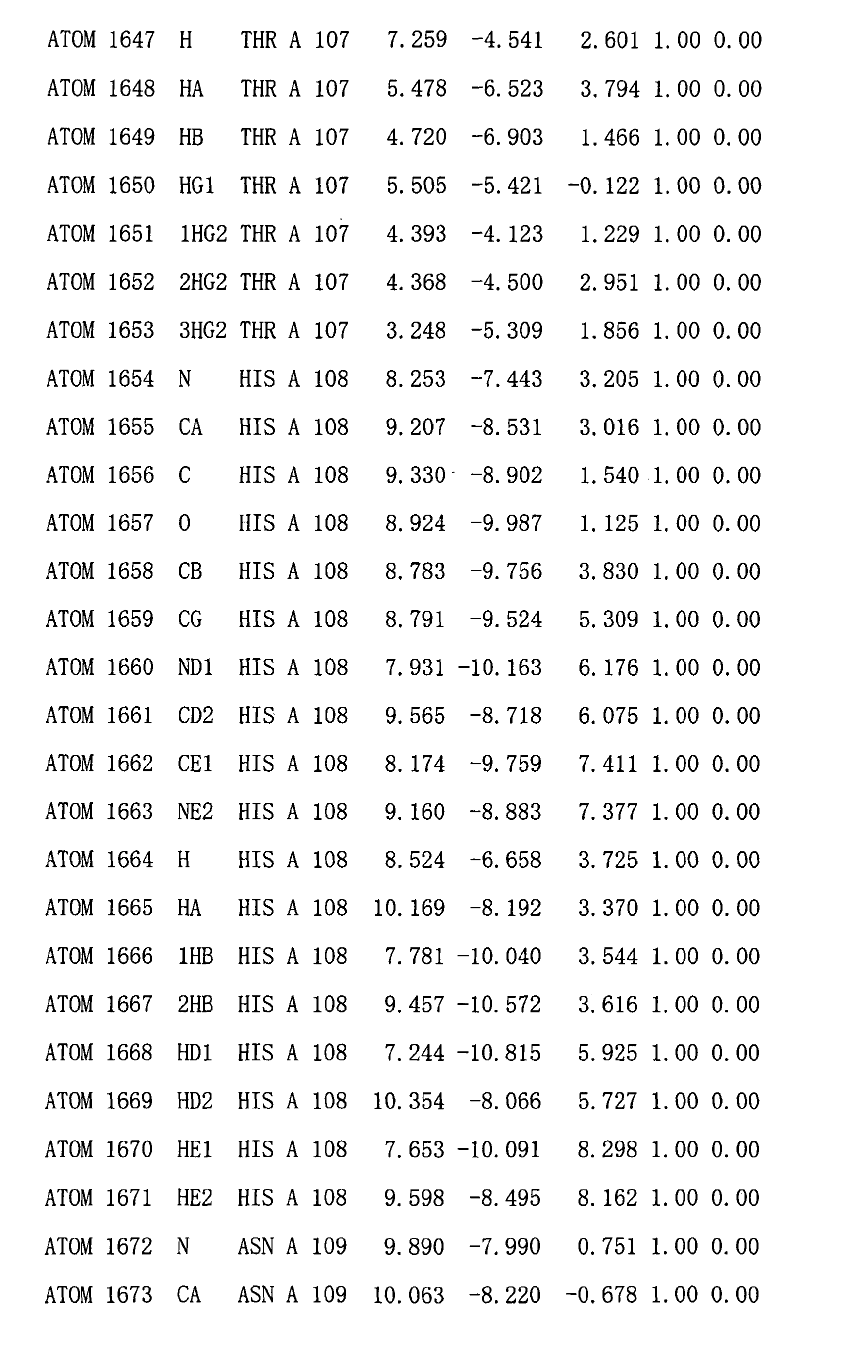

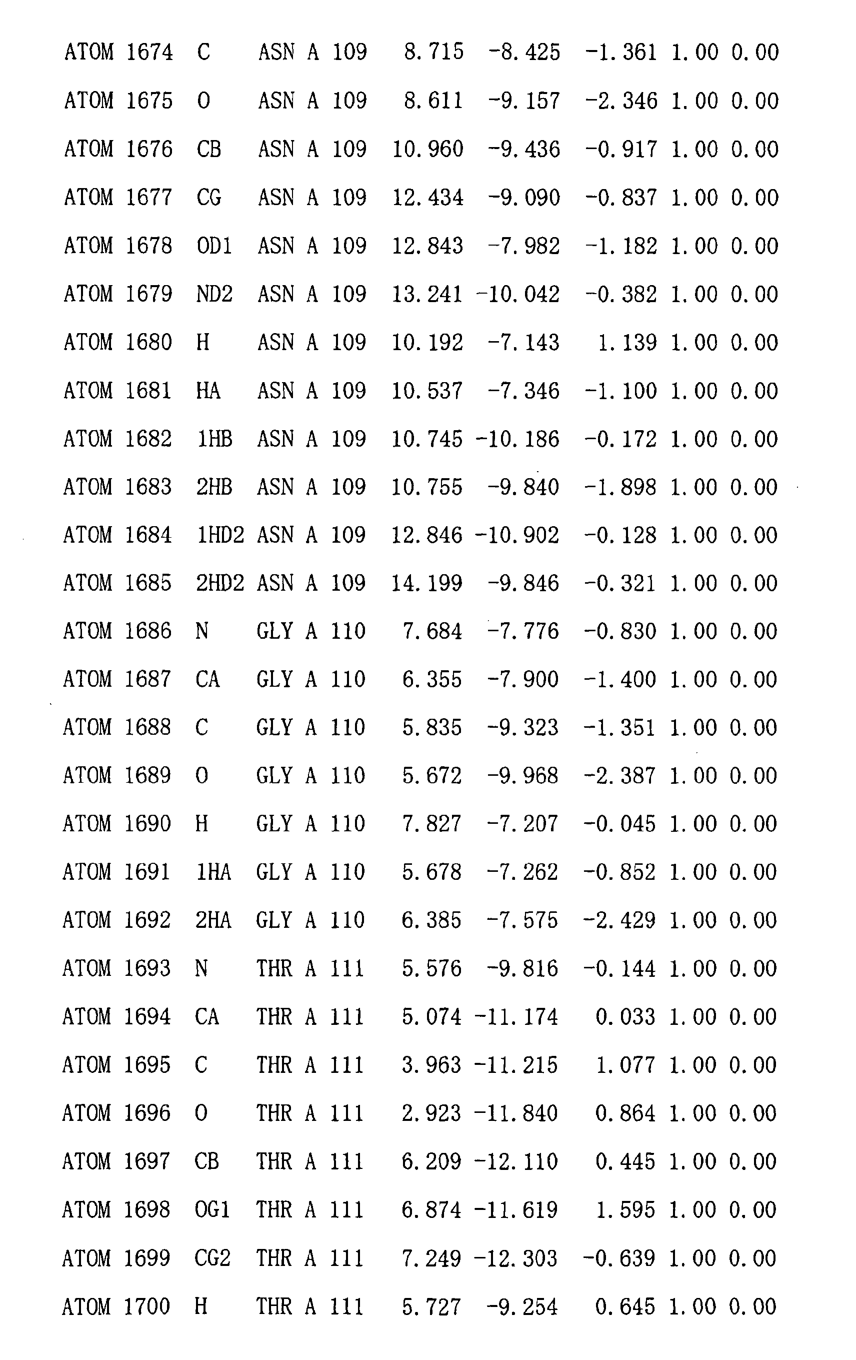

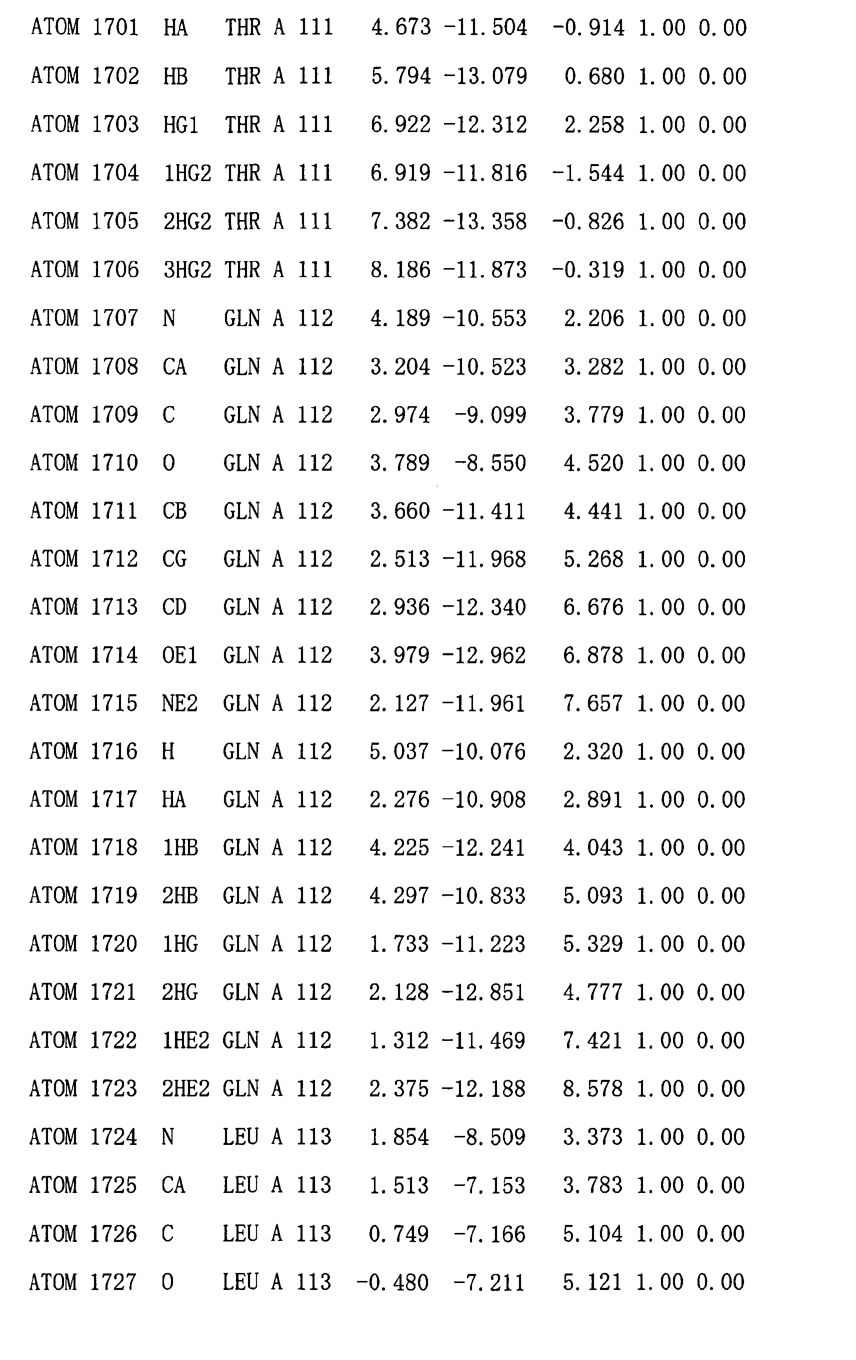

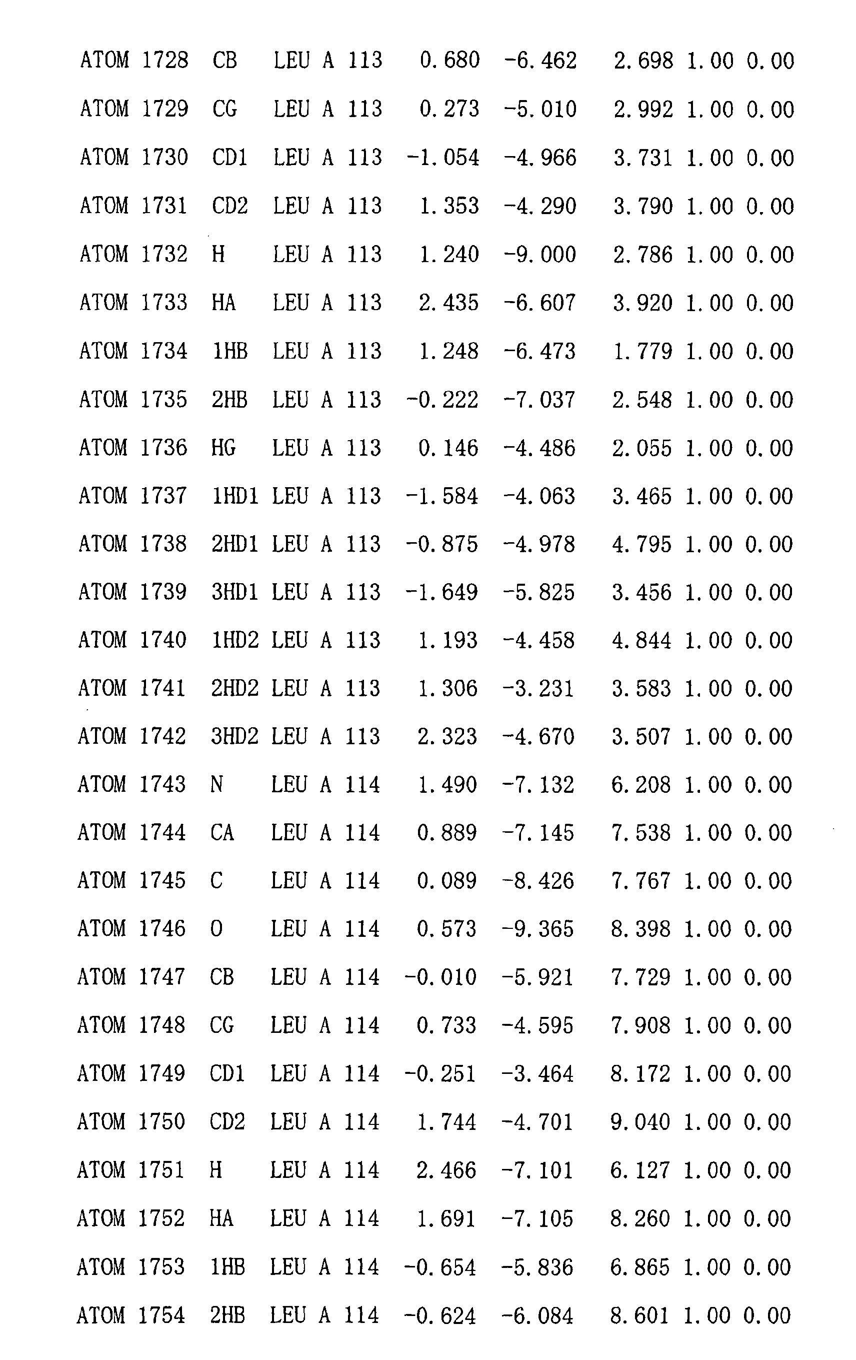

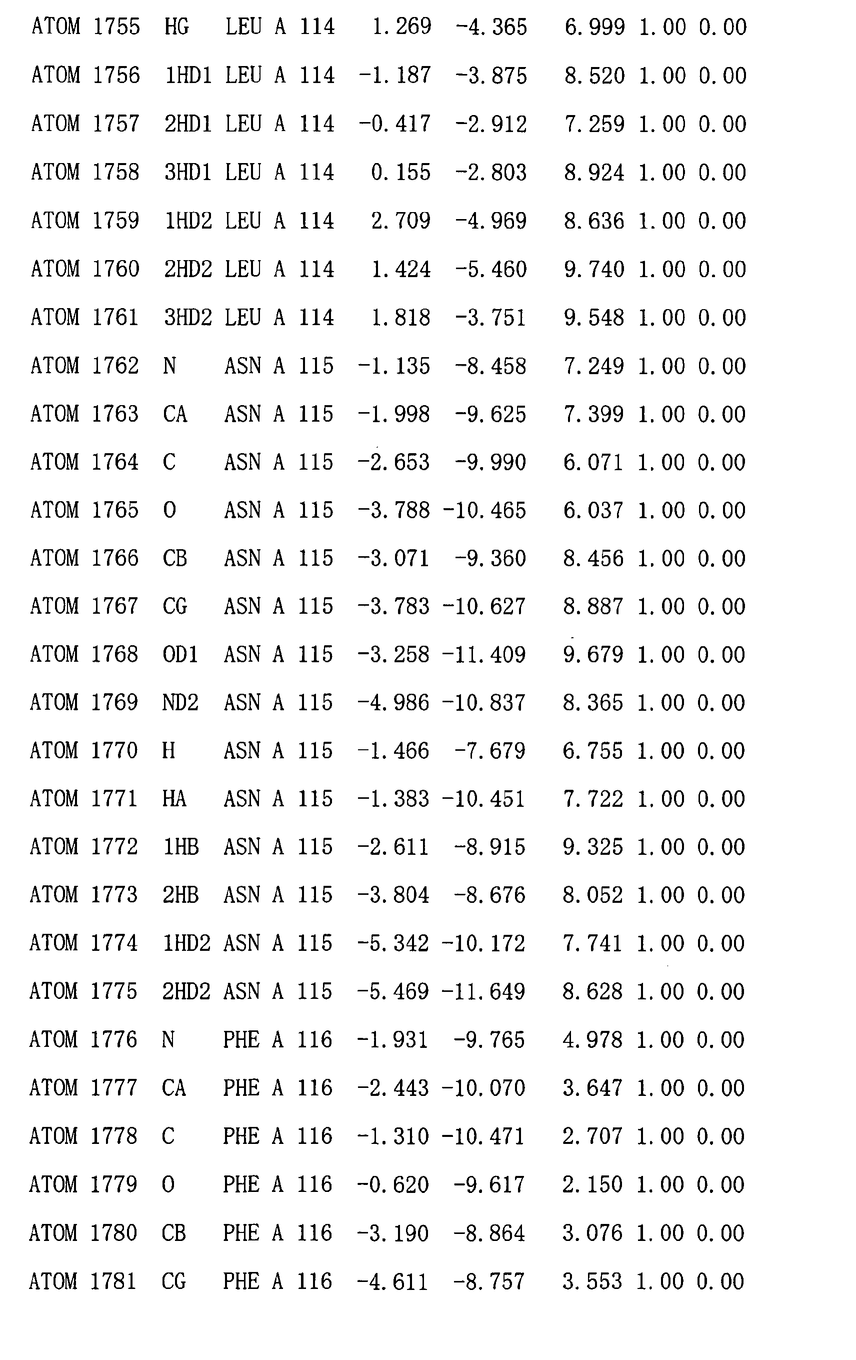

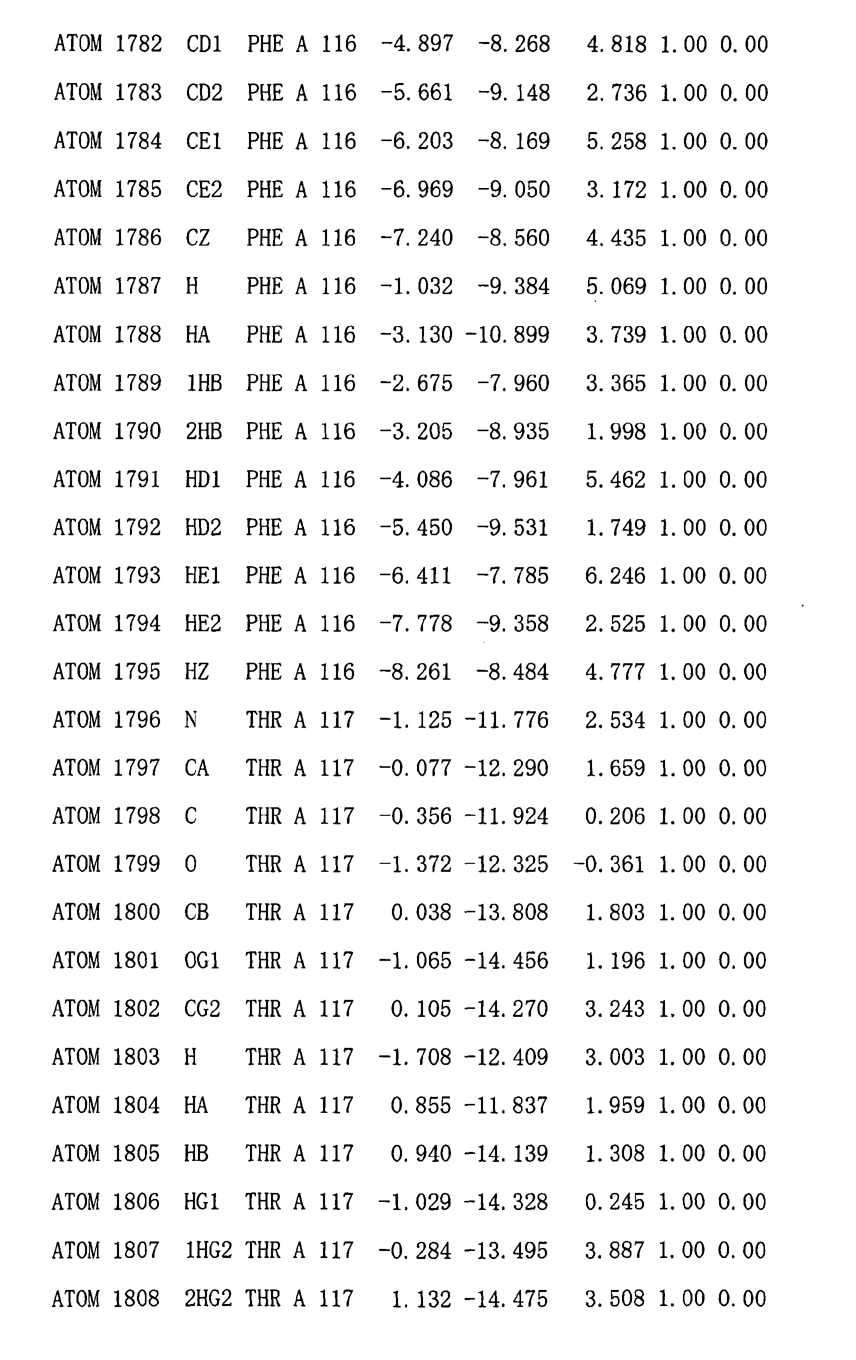

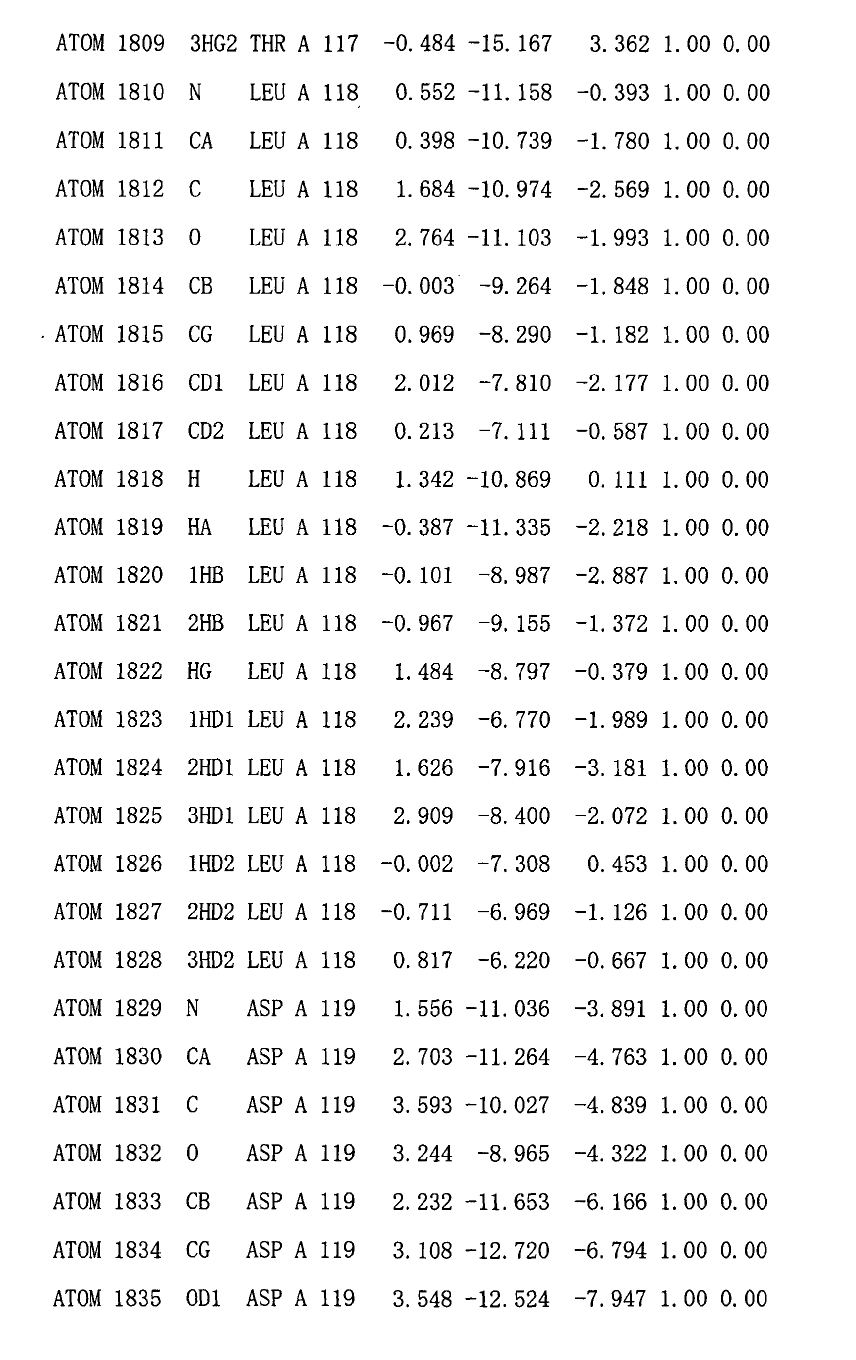

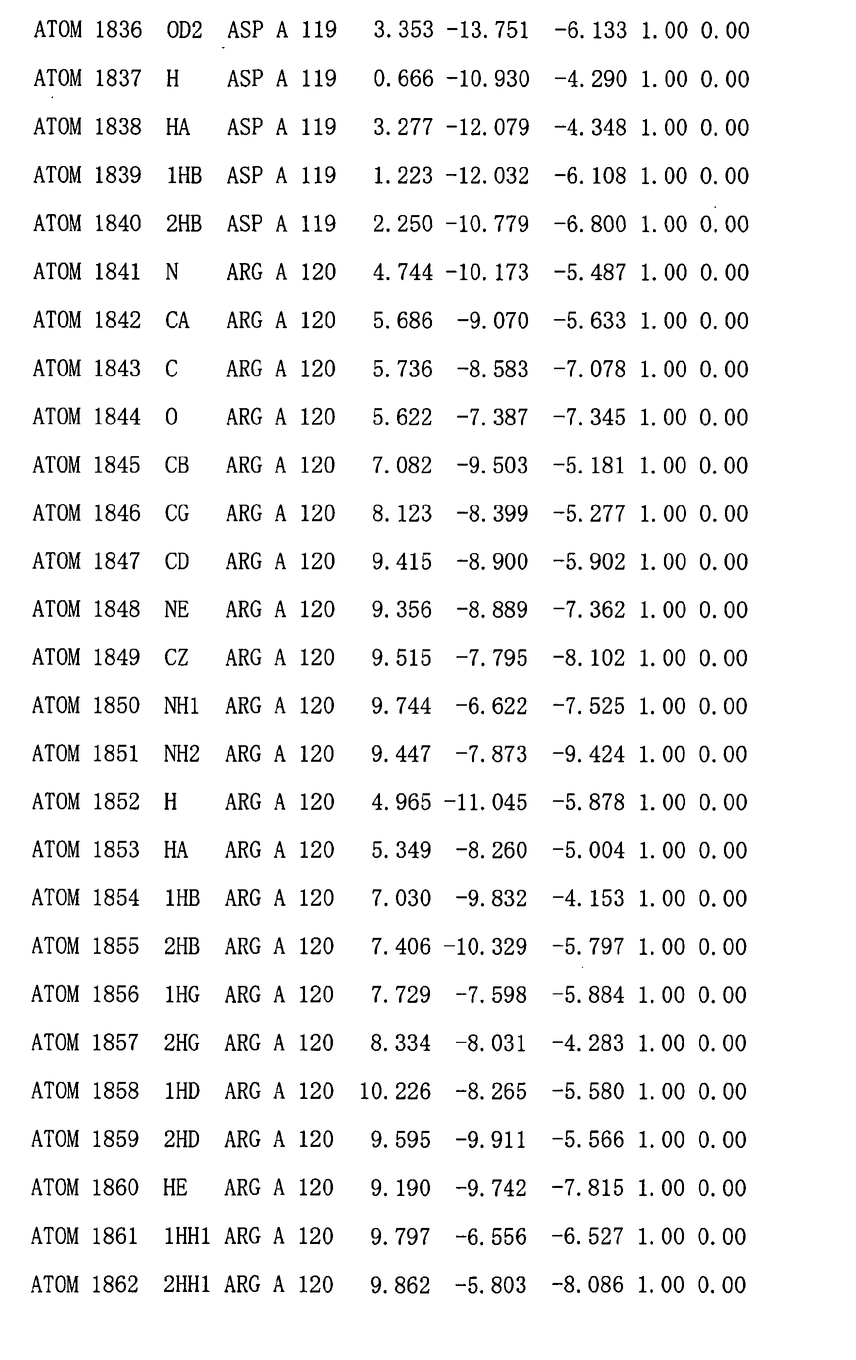

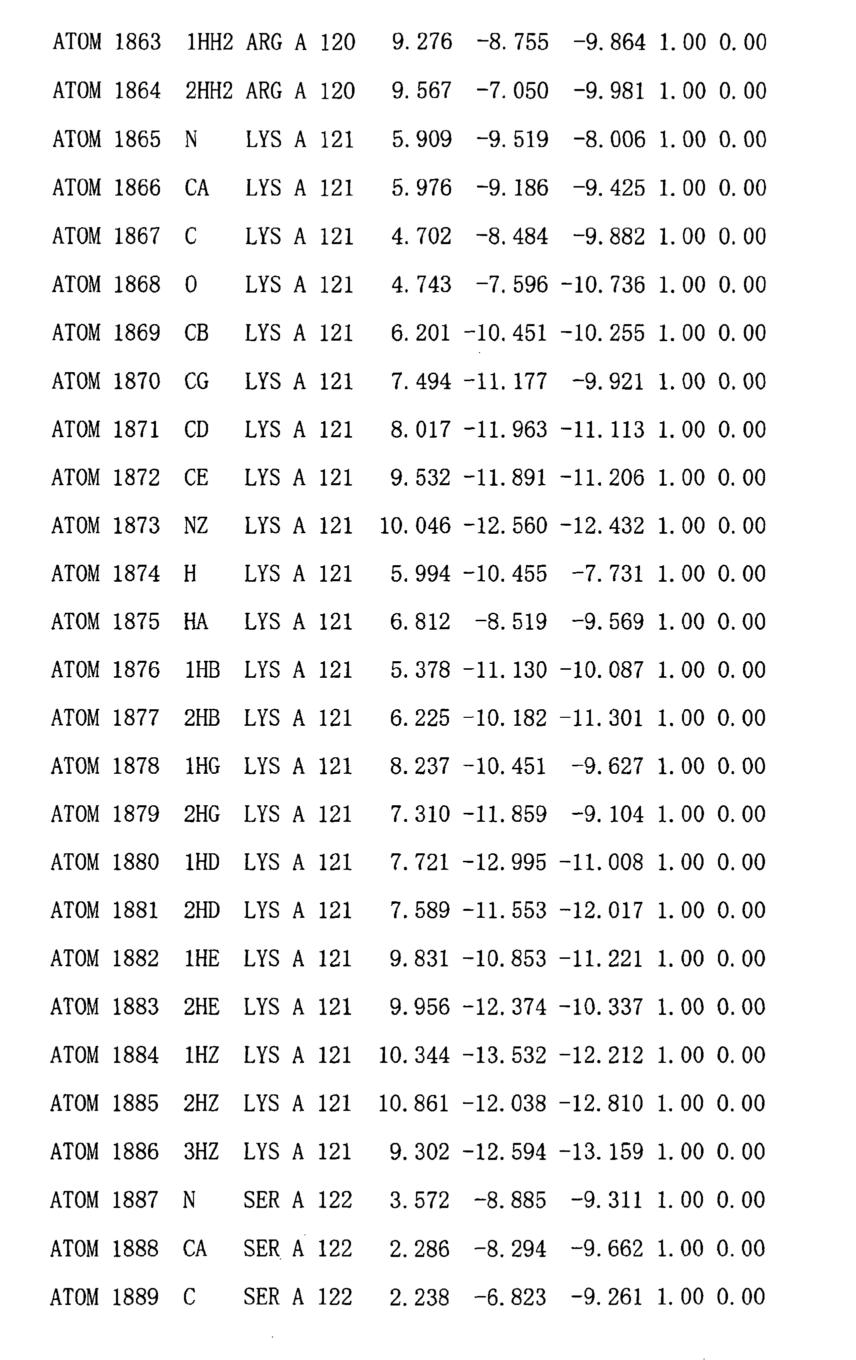

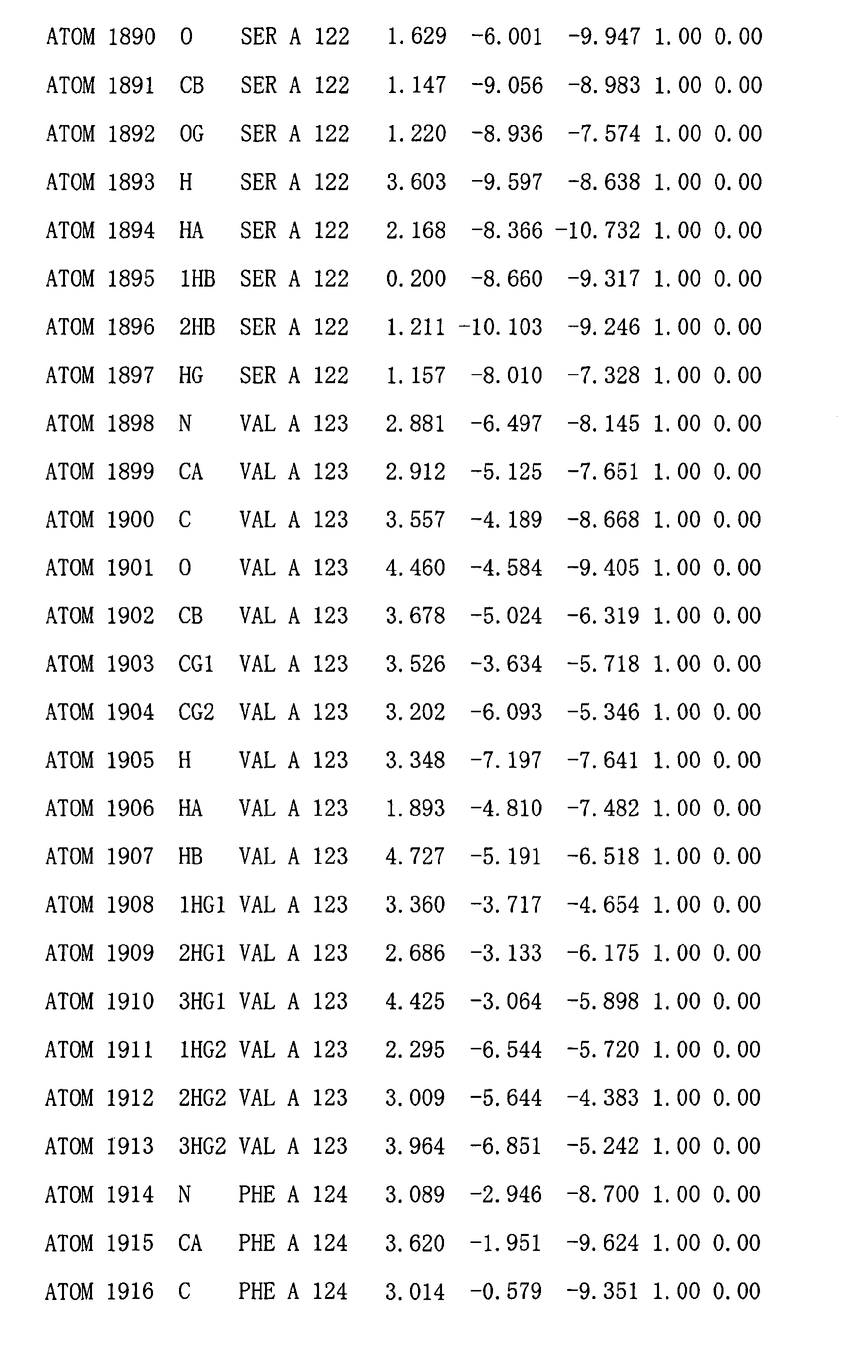

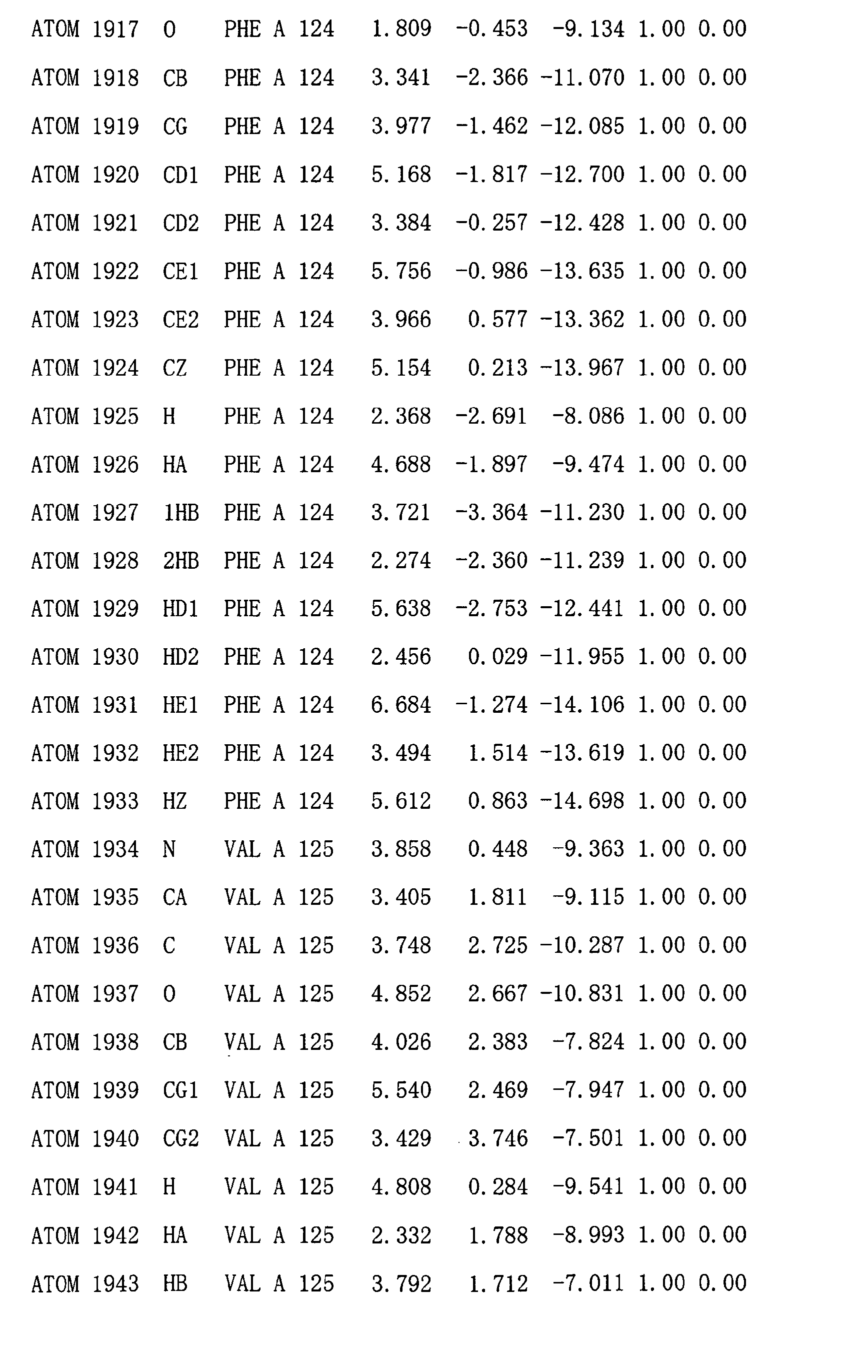

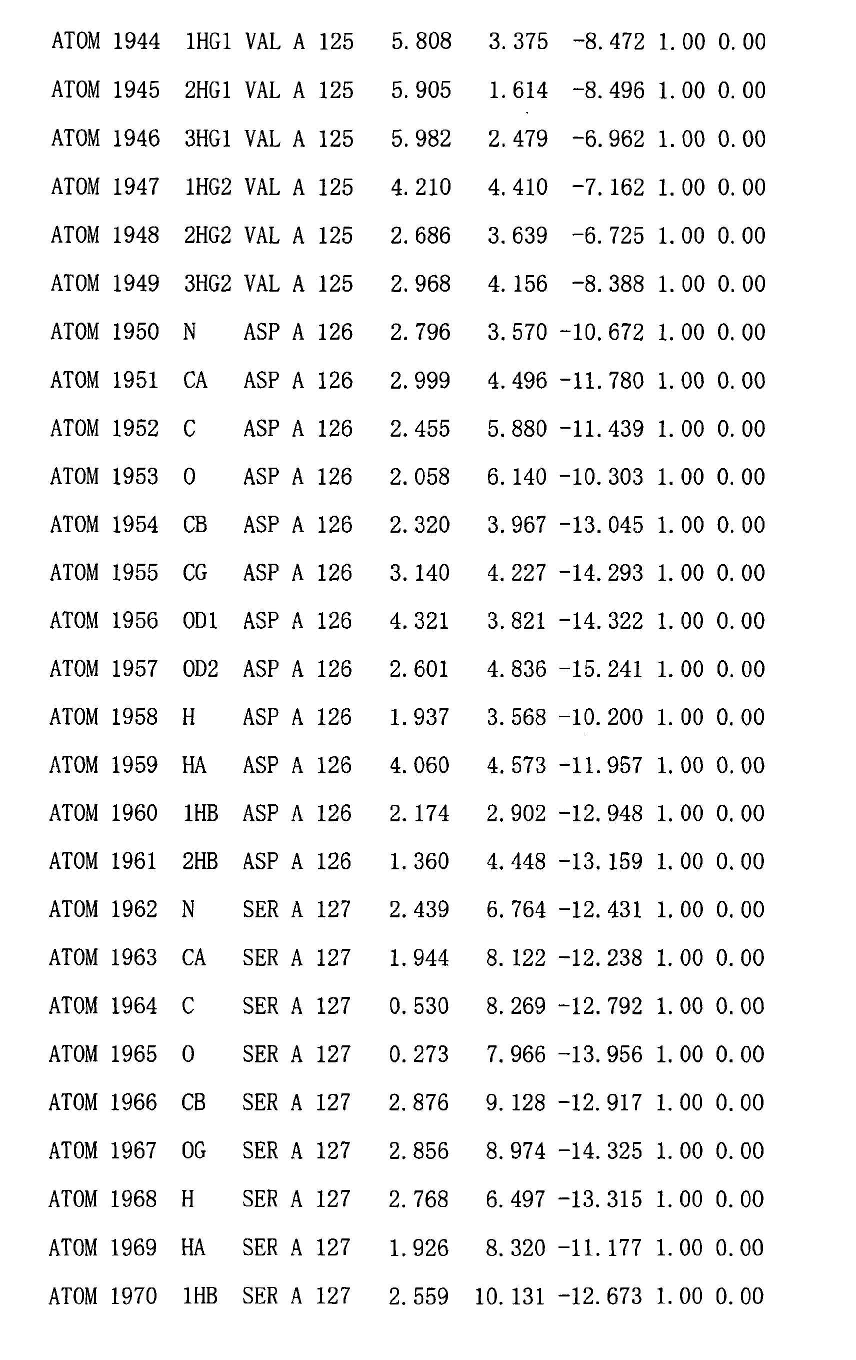

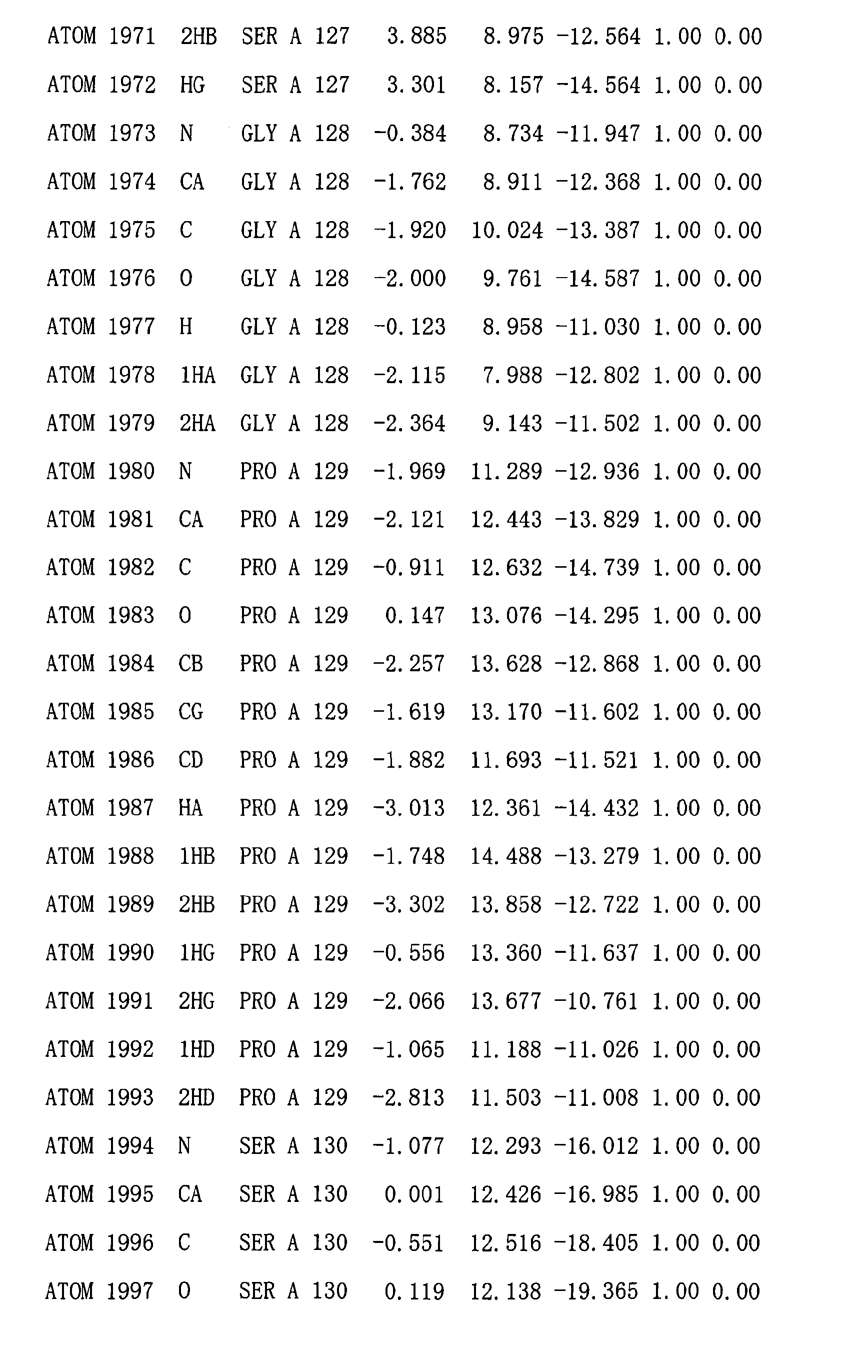

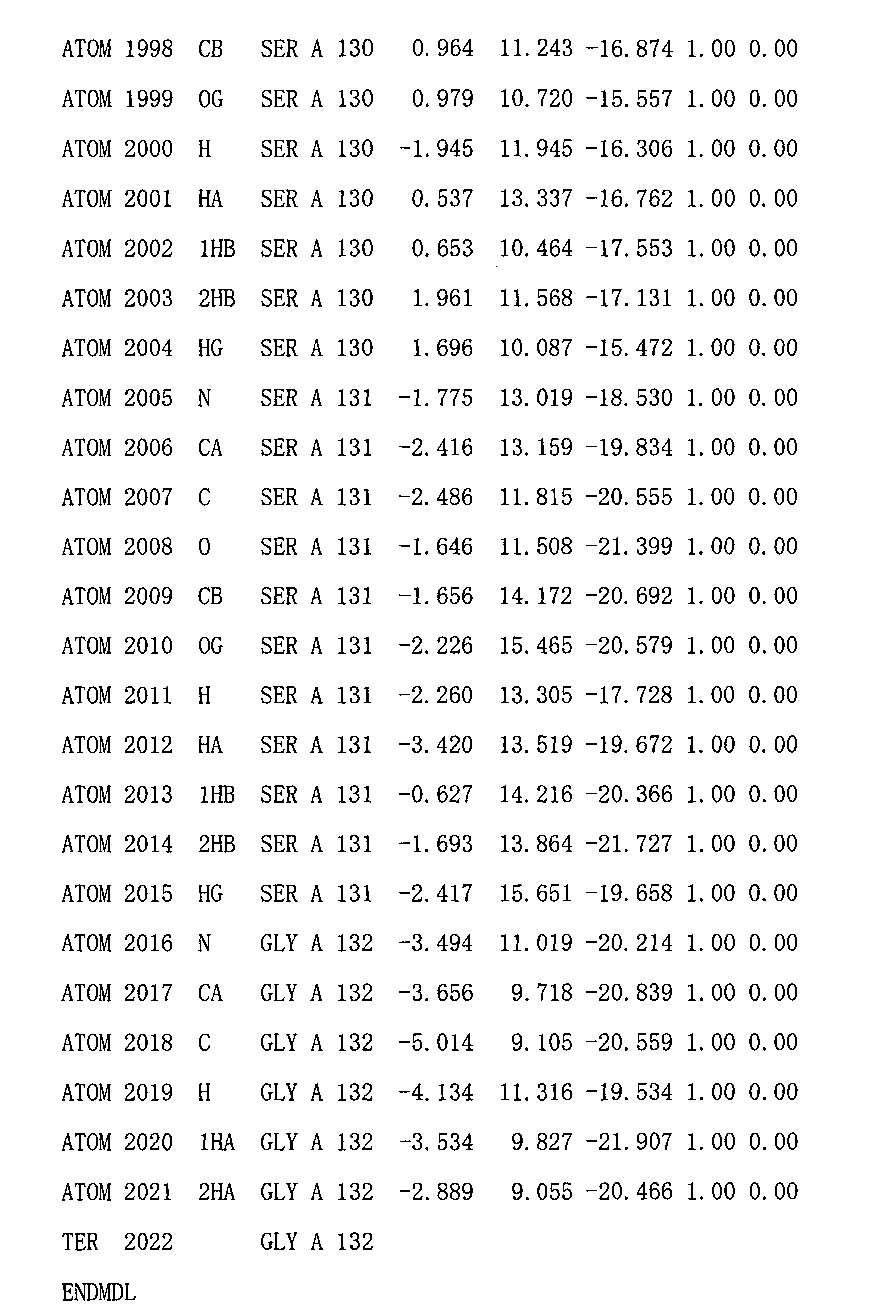

### Three-Dimensional Structure Coordinate 2

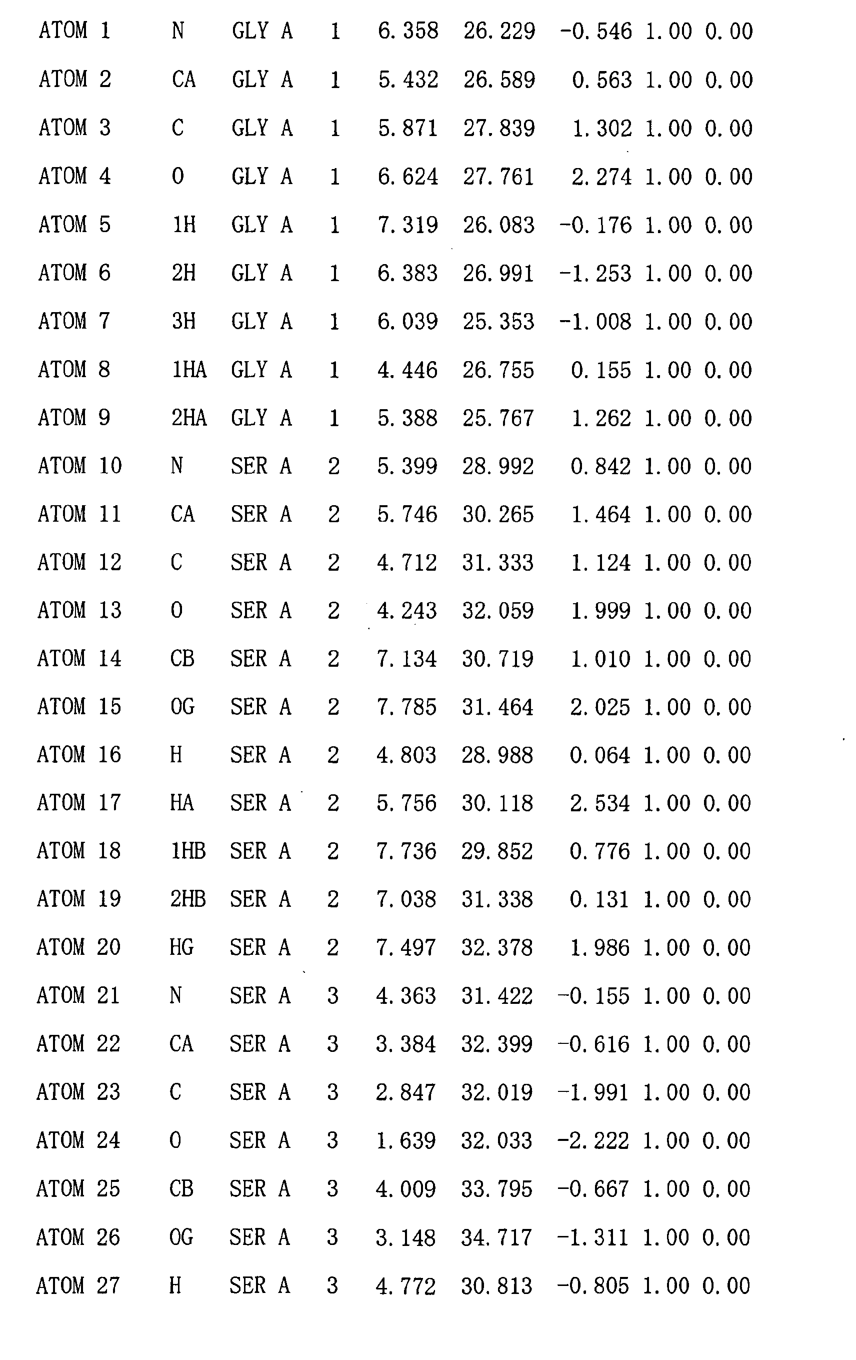

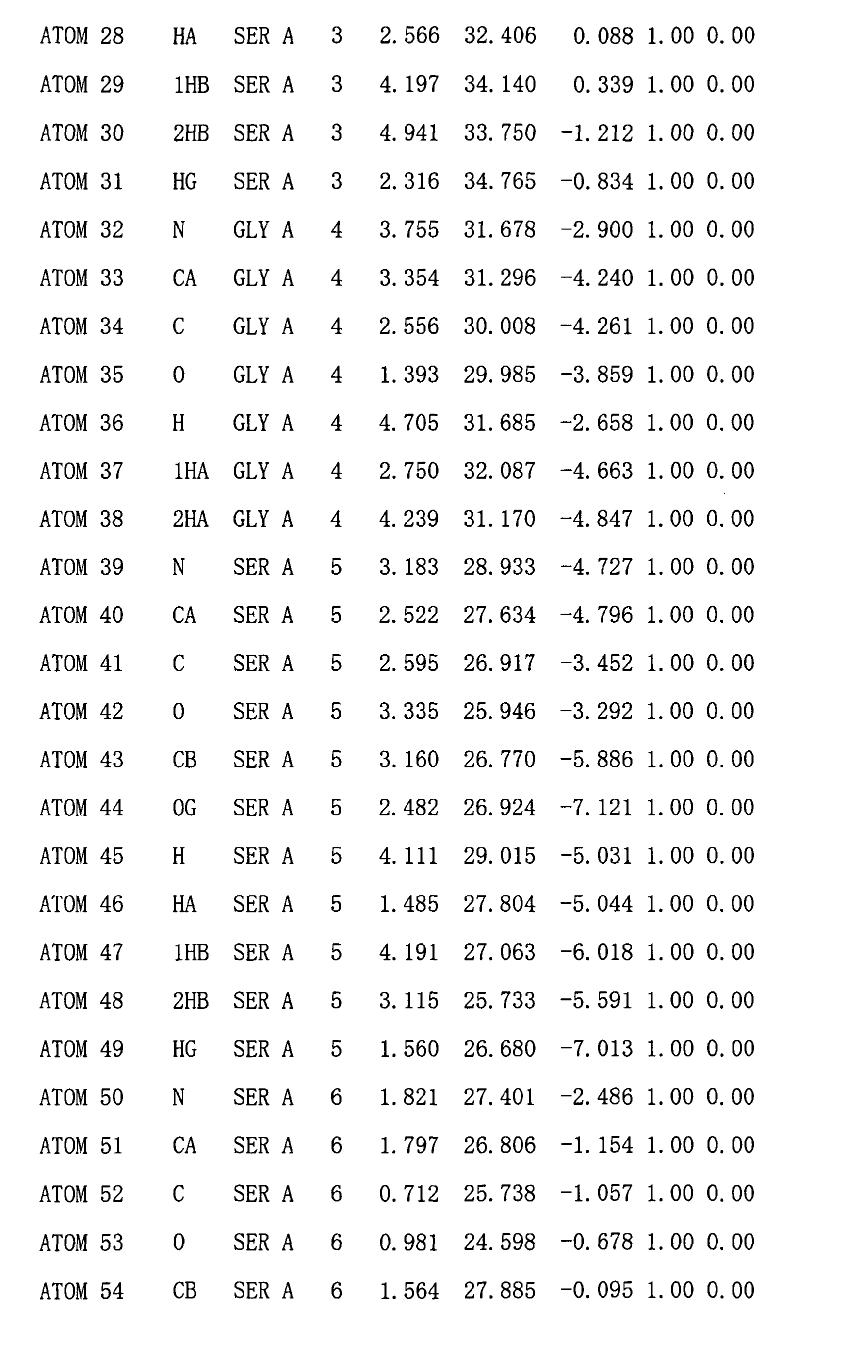

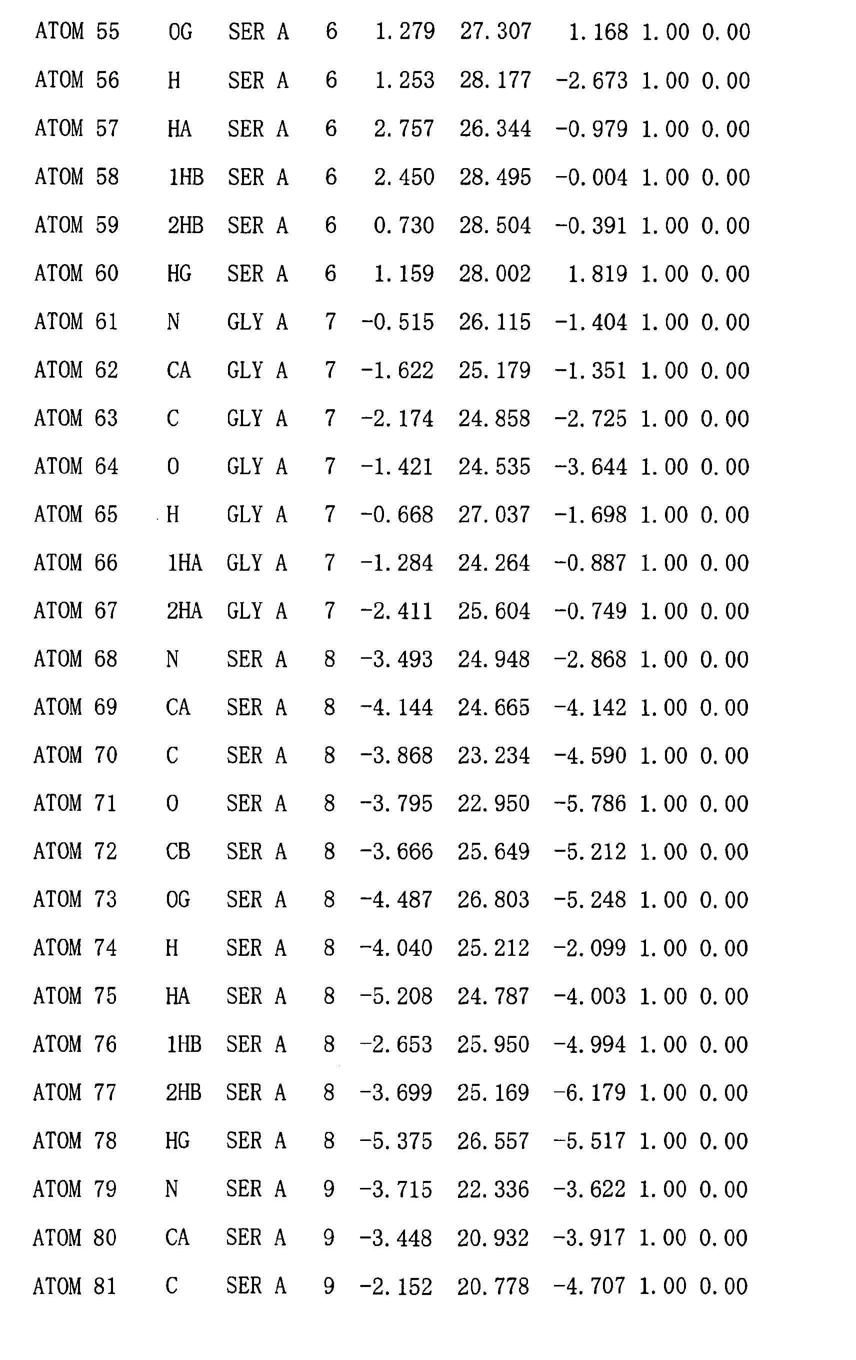

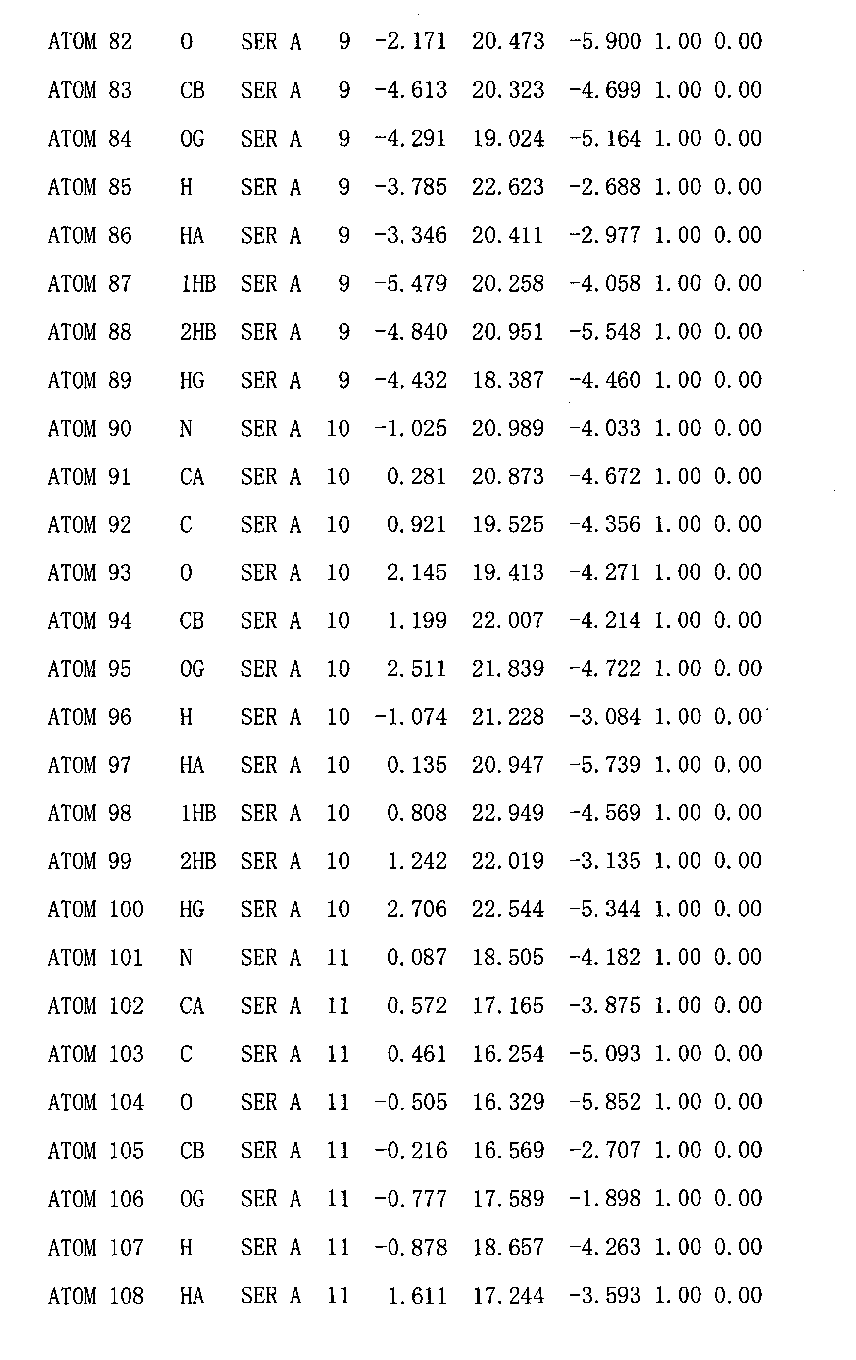

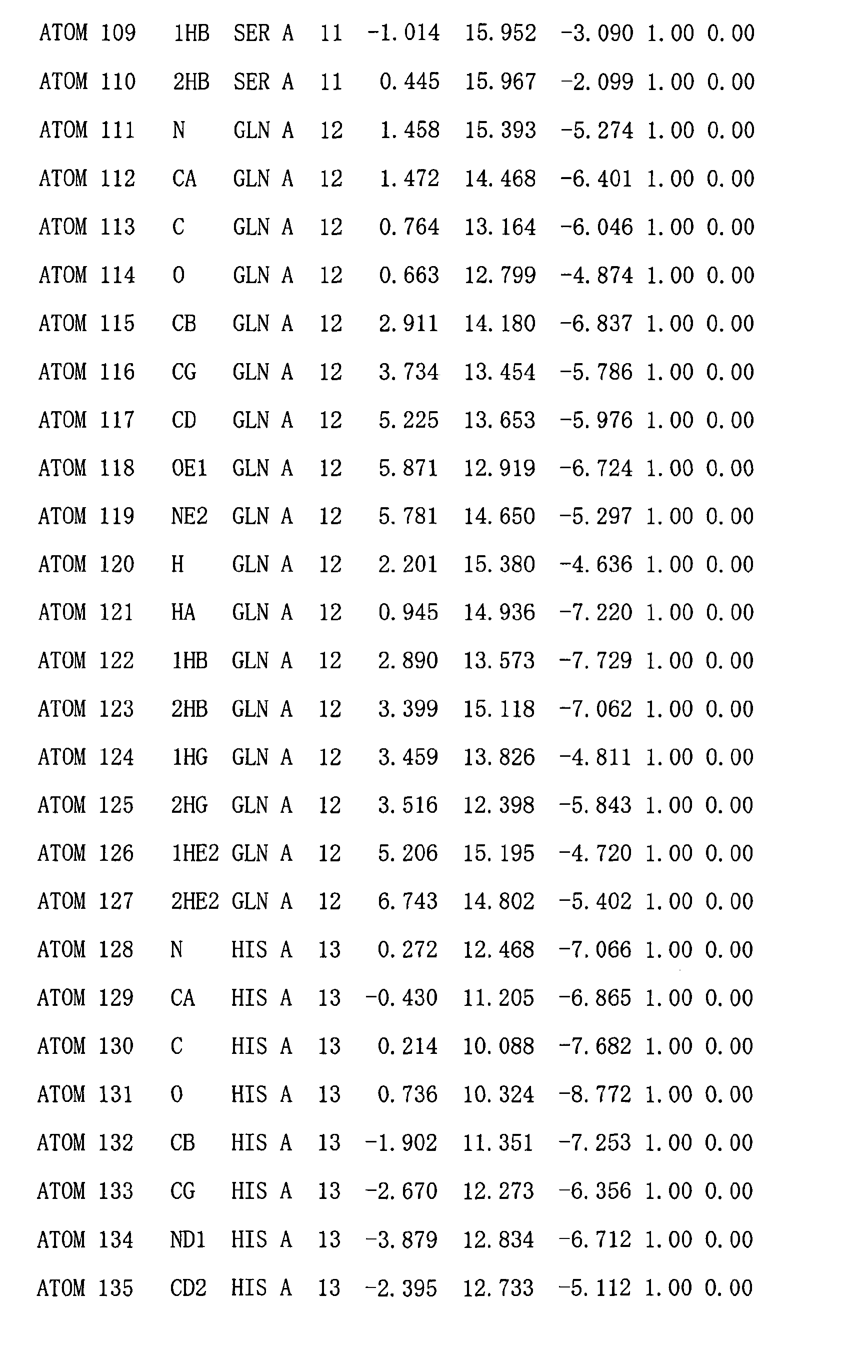

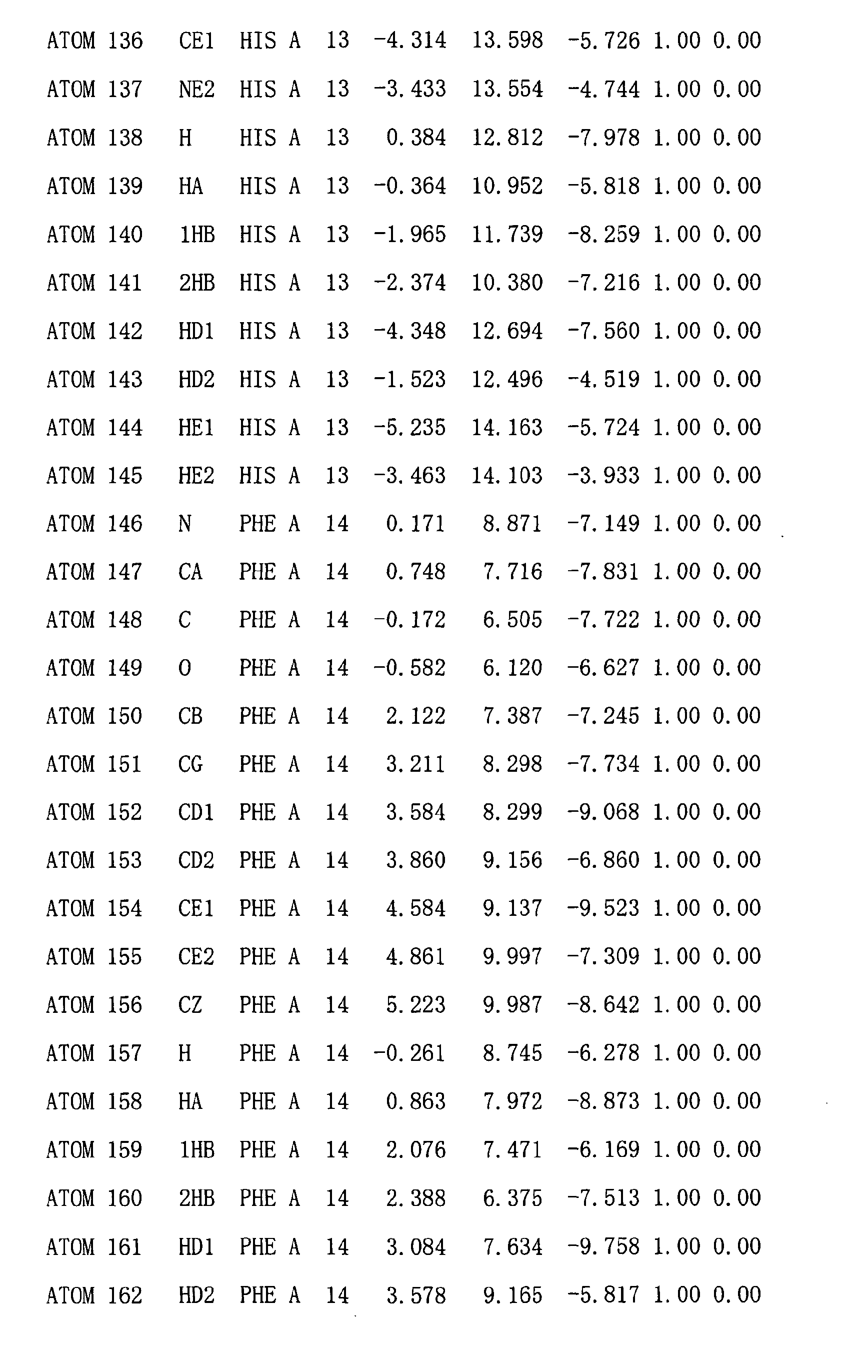

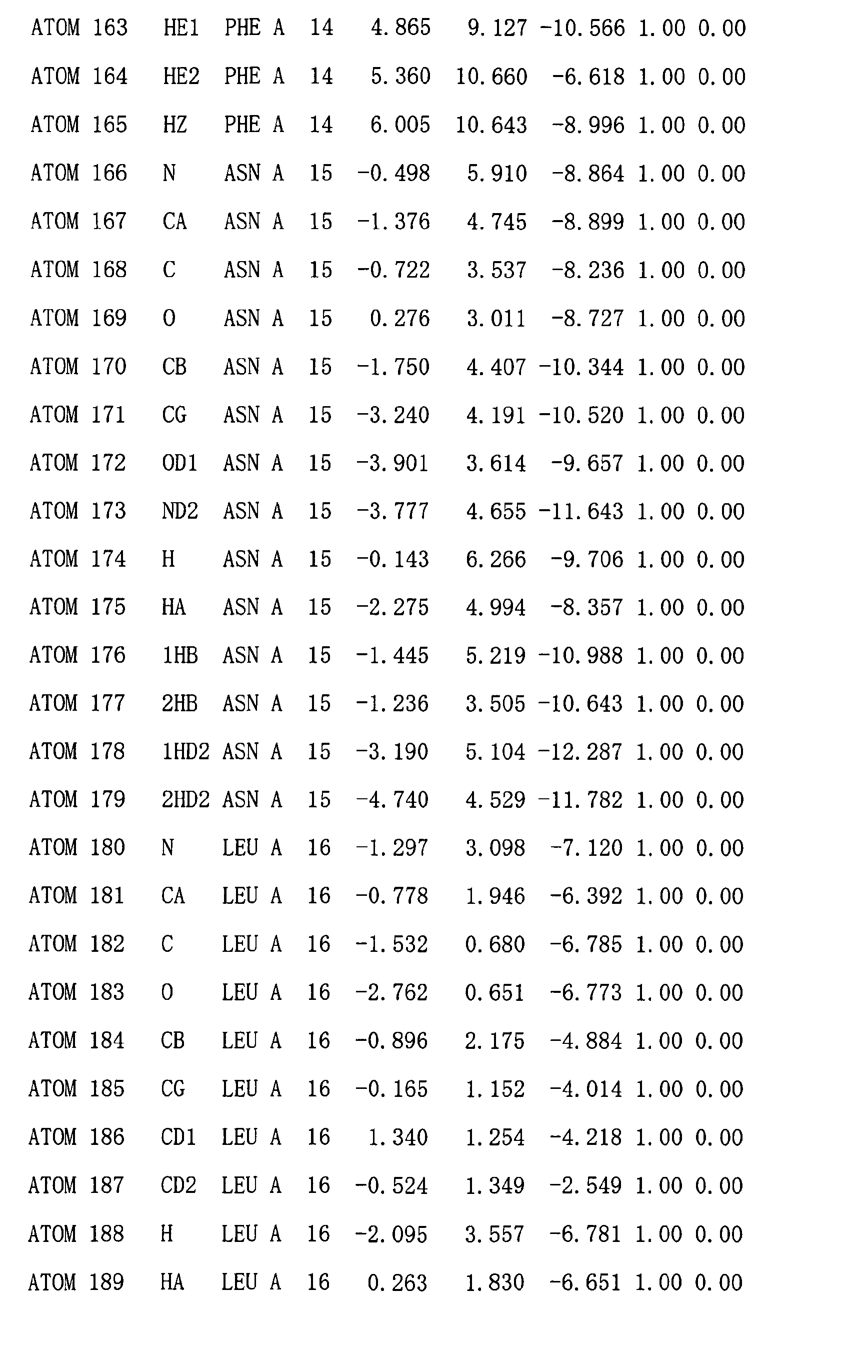

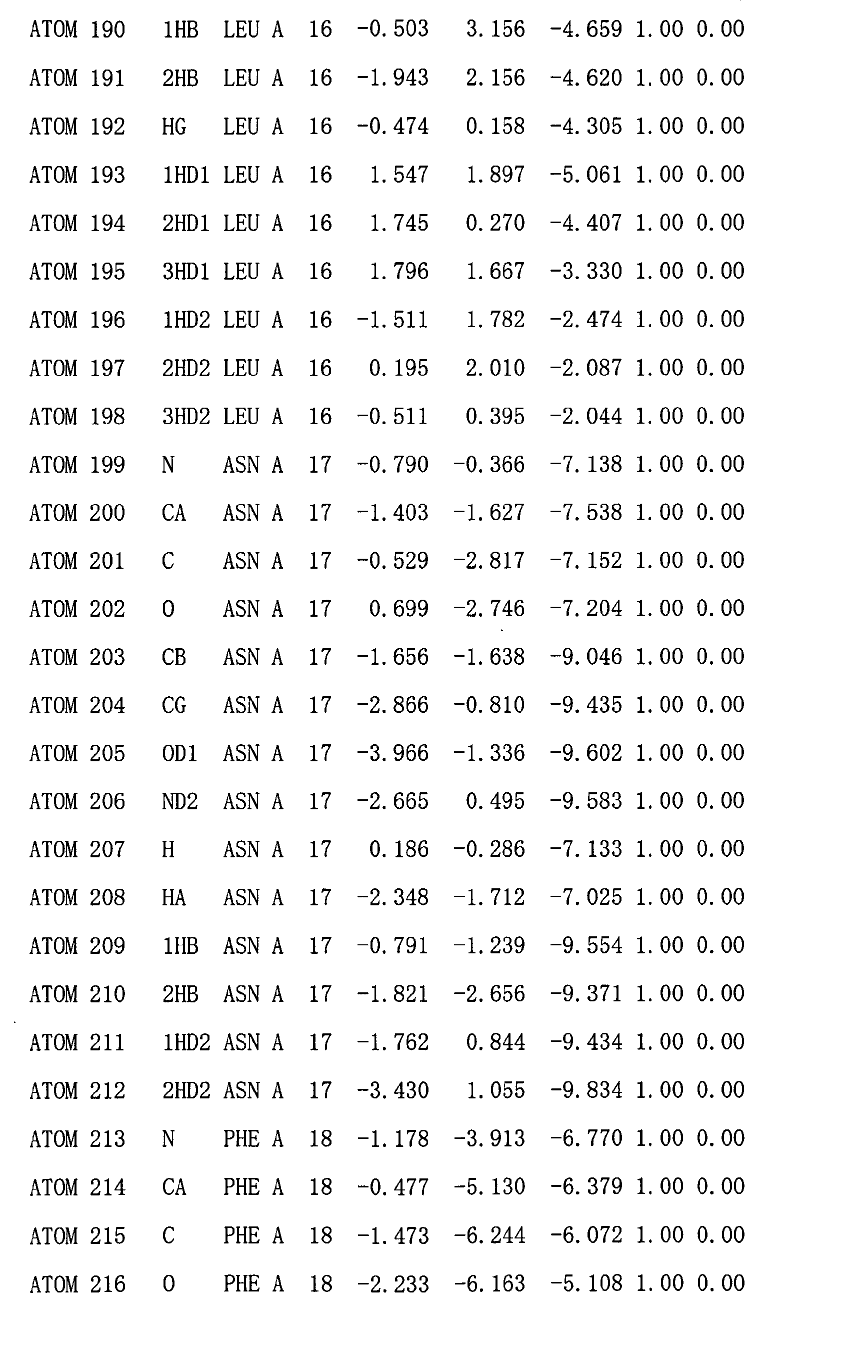

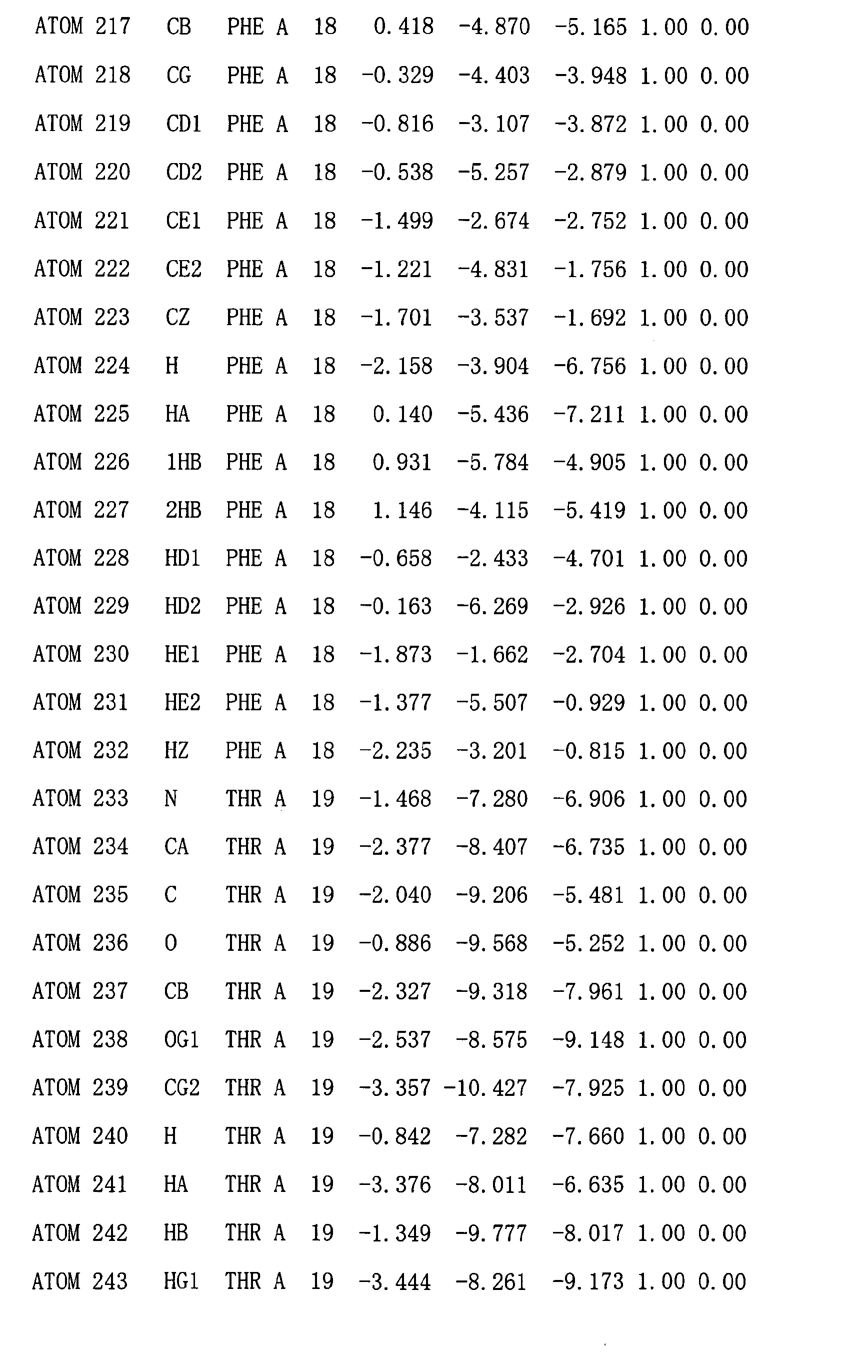

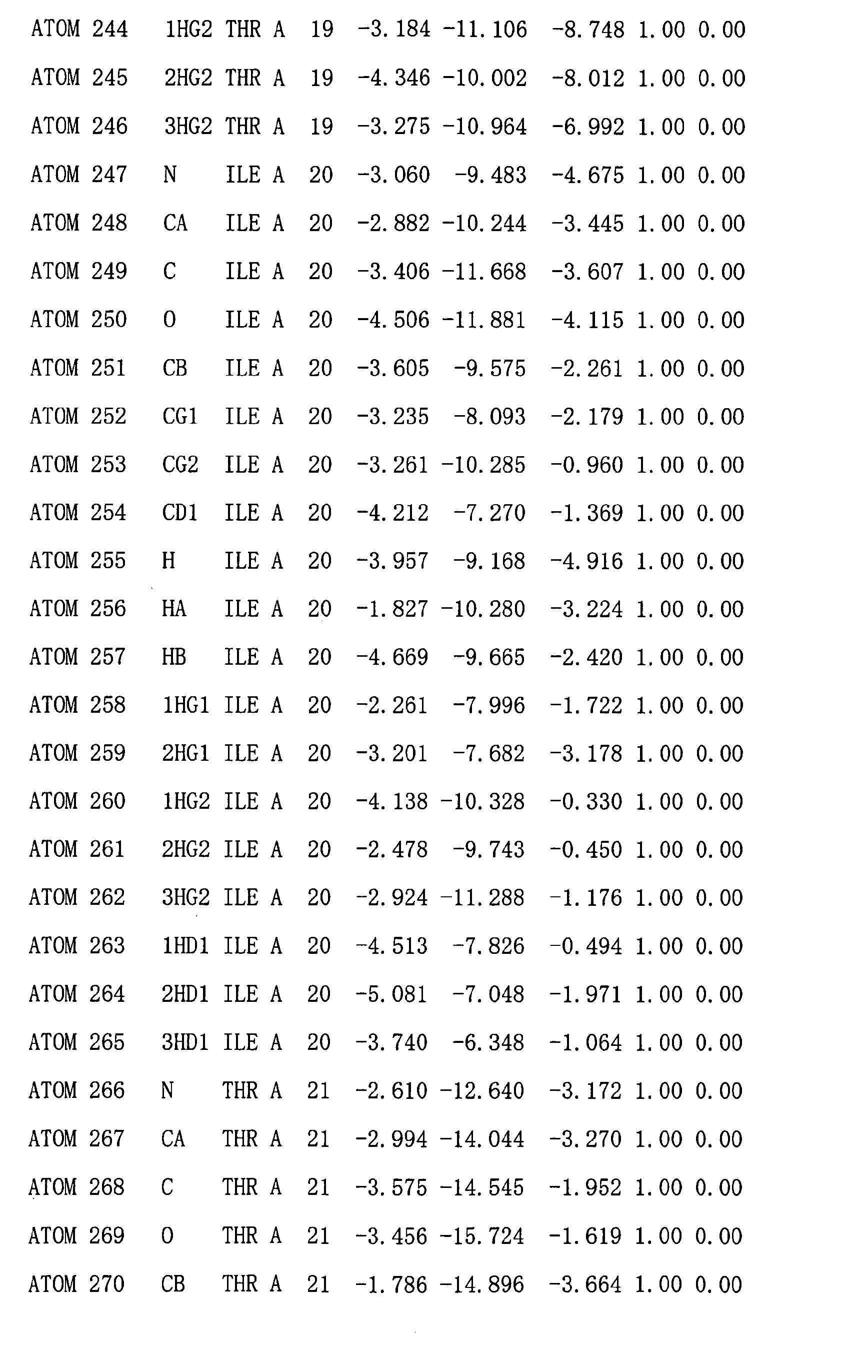

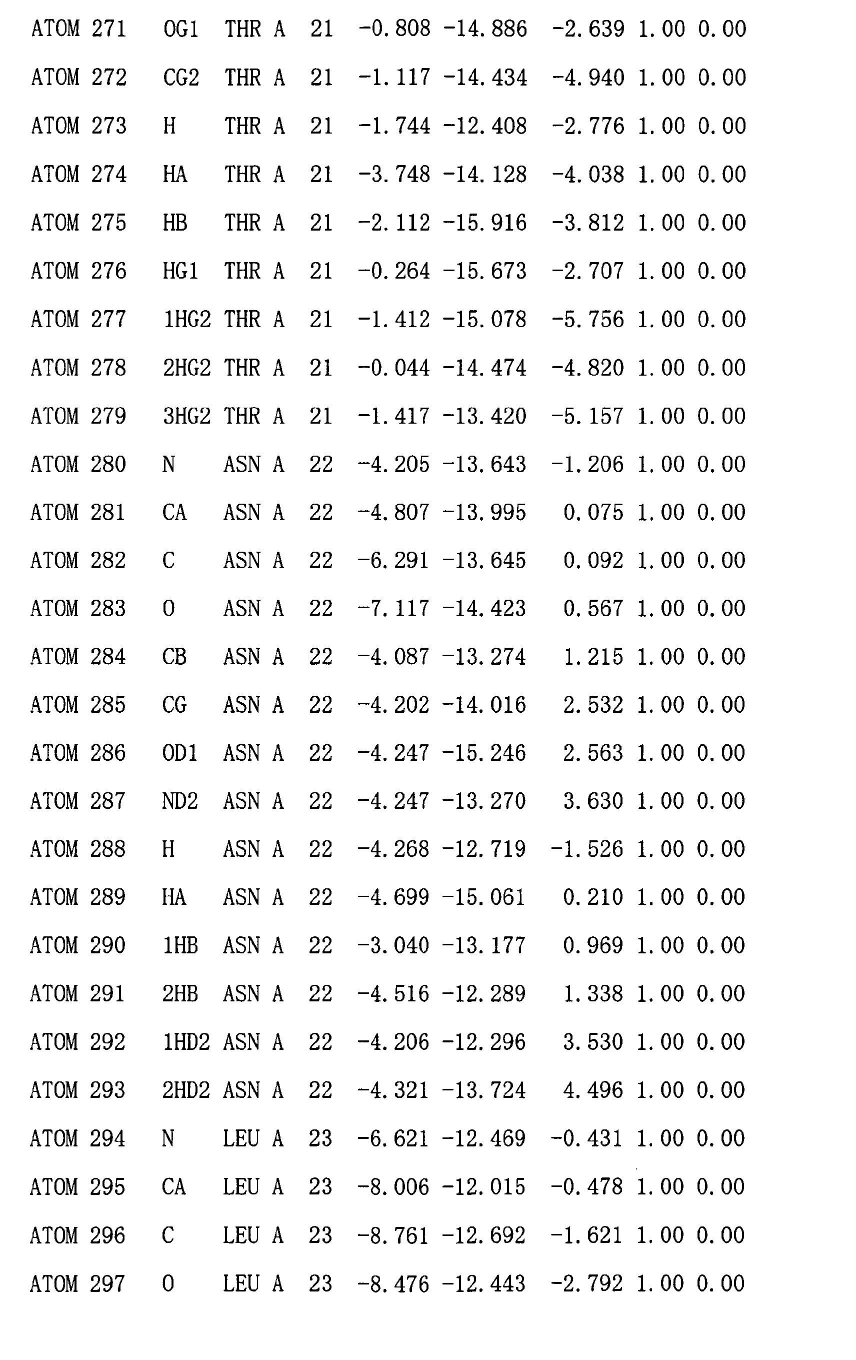

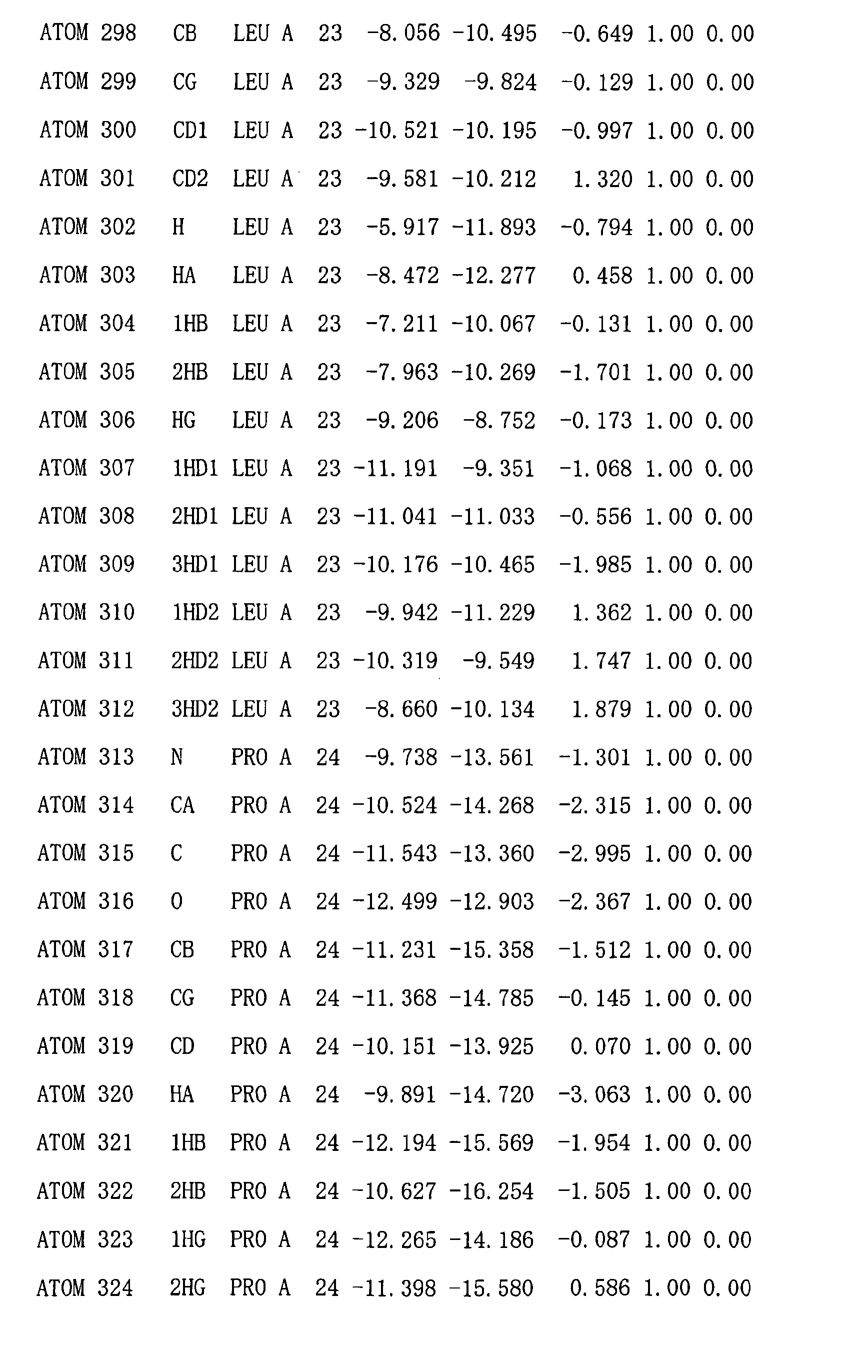

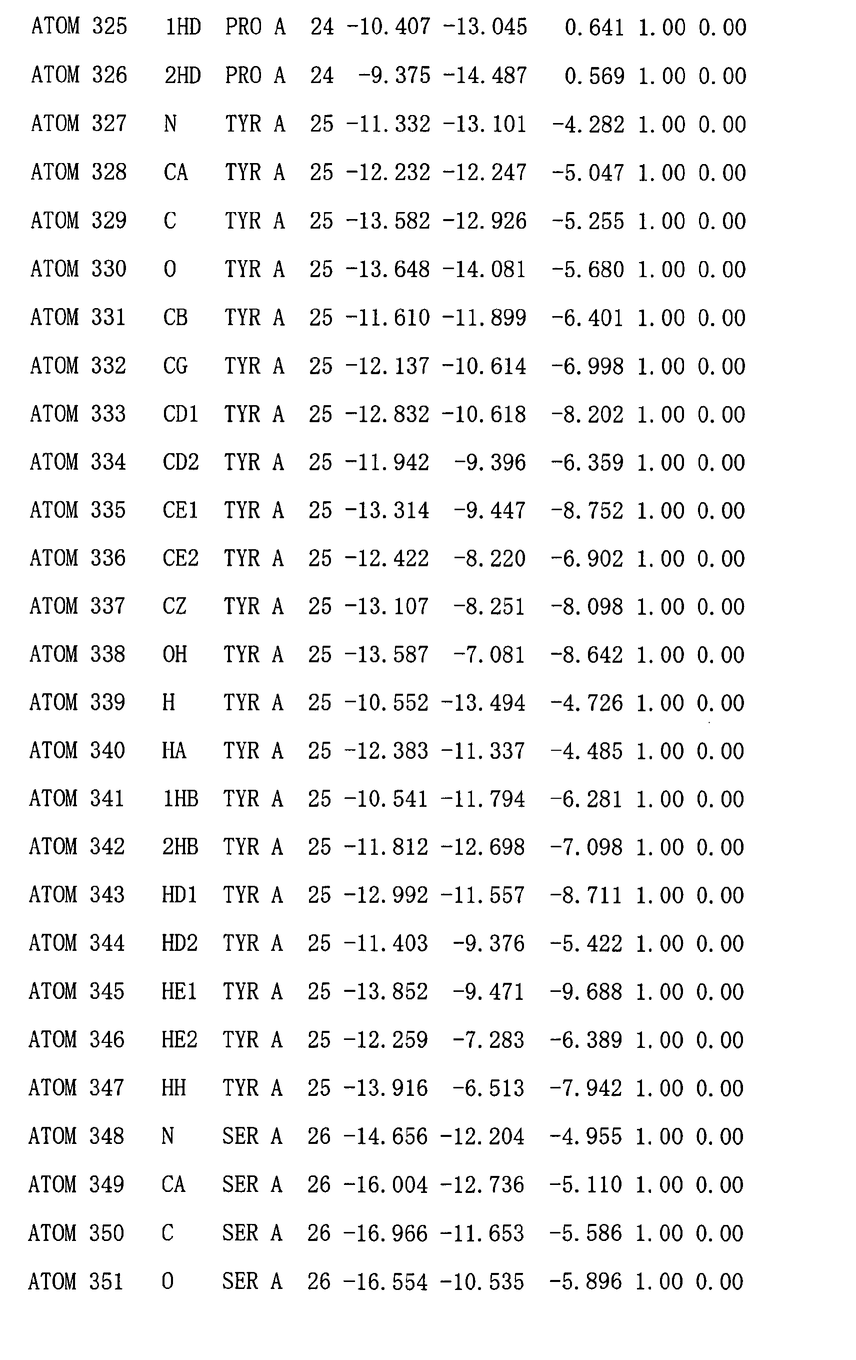

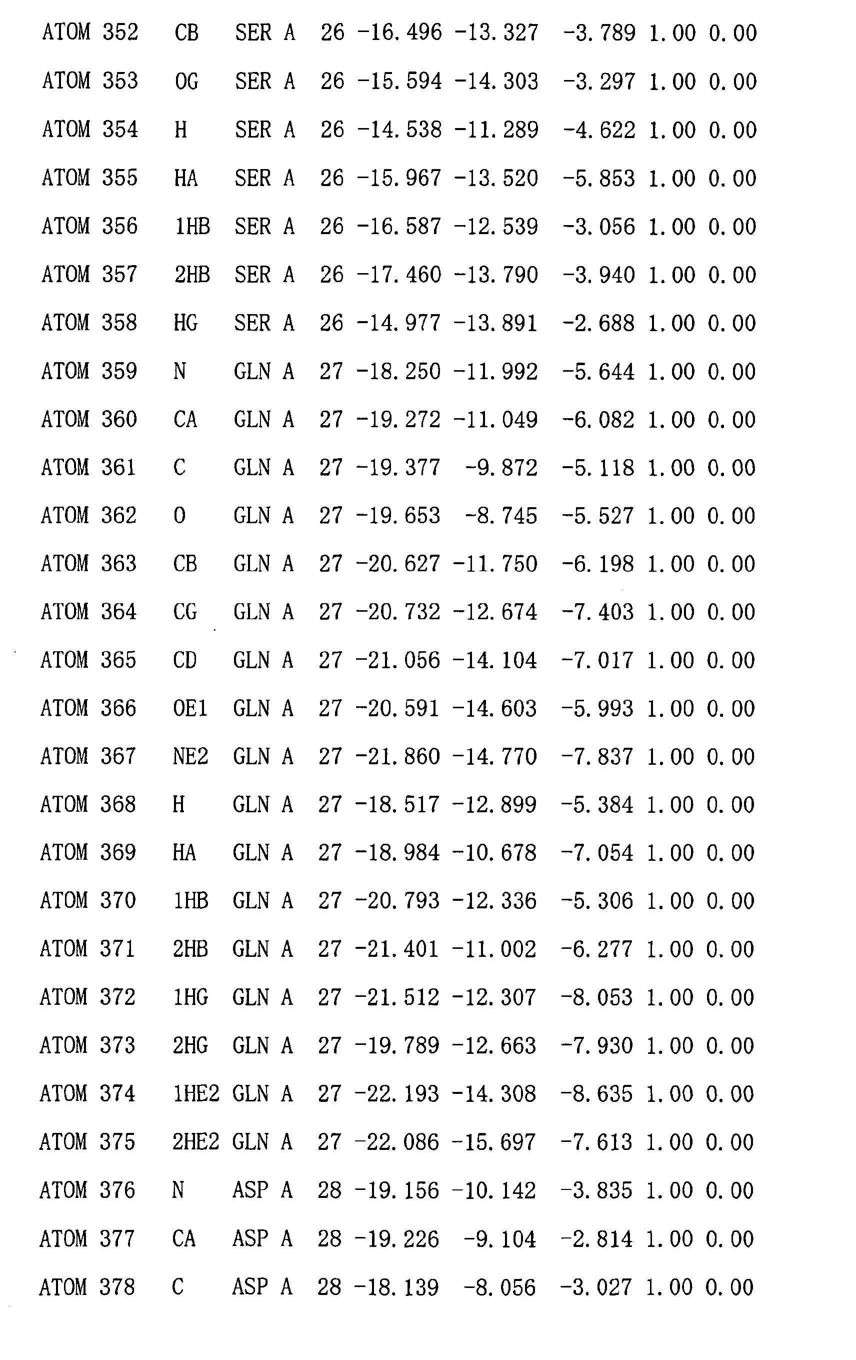

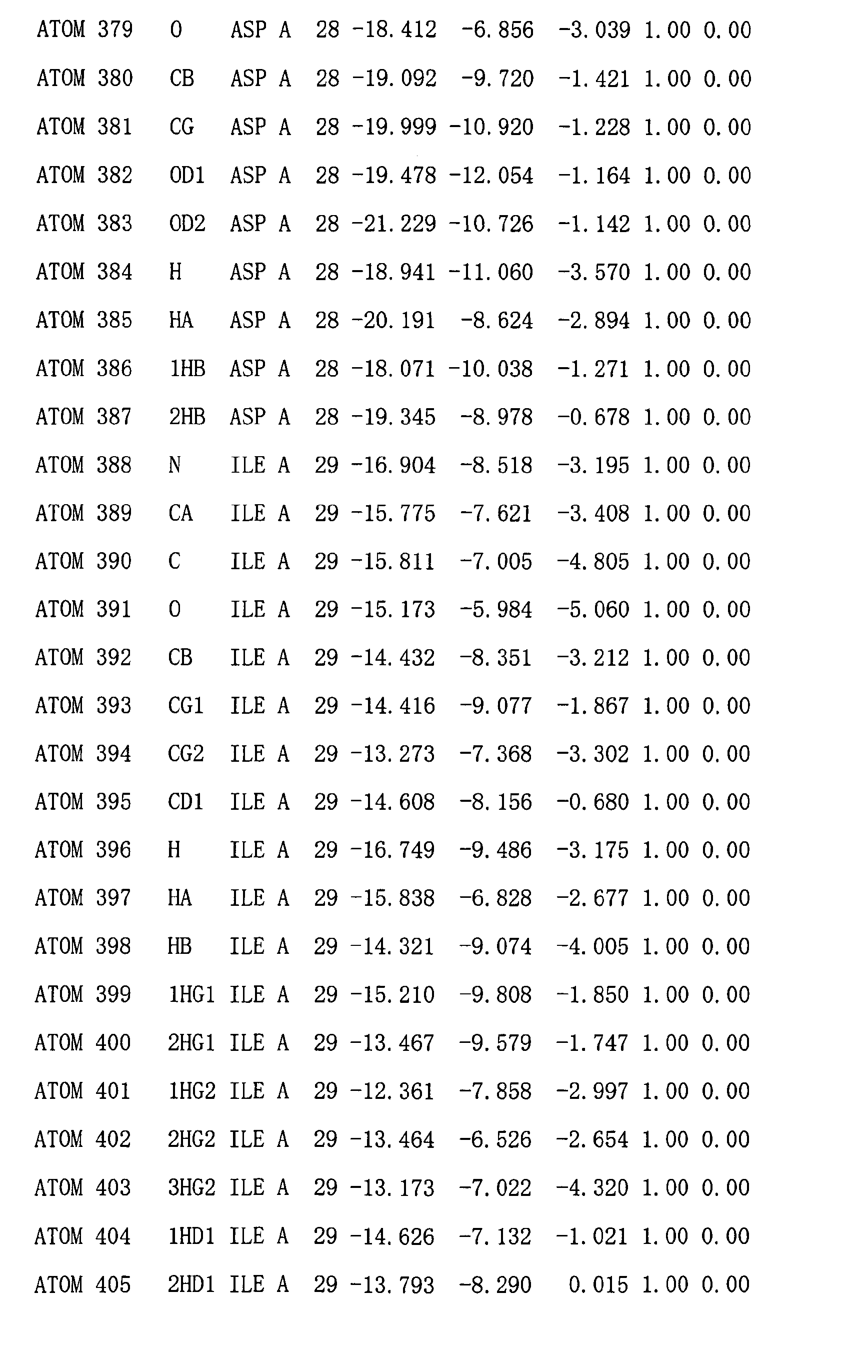

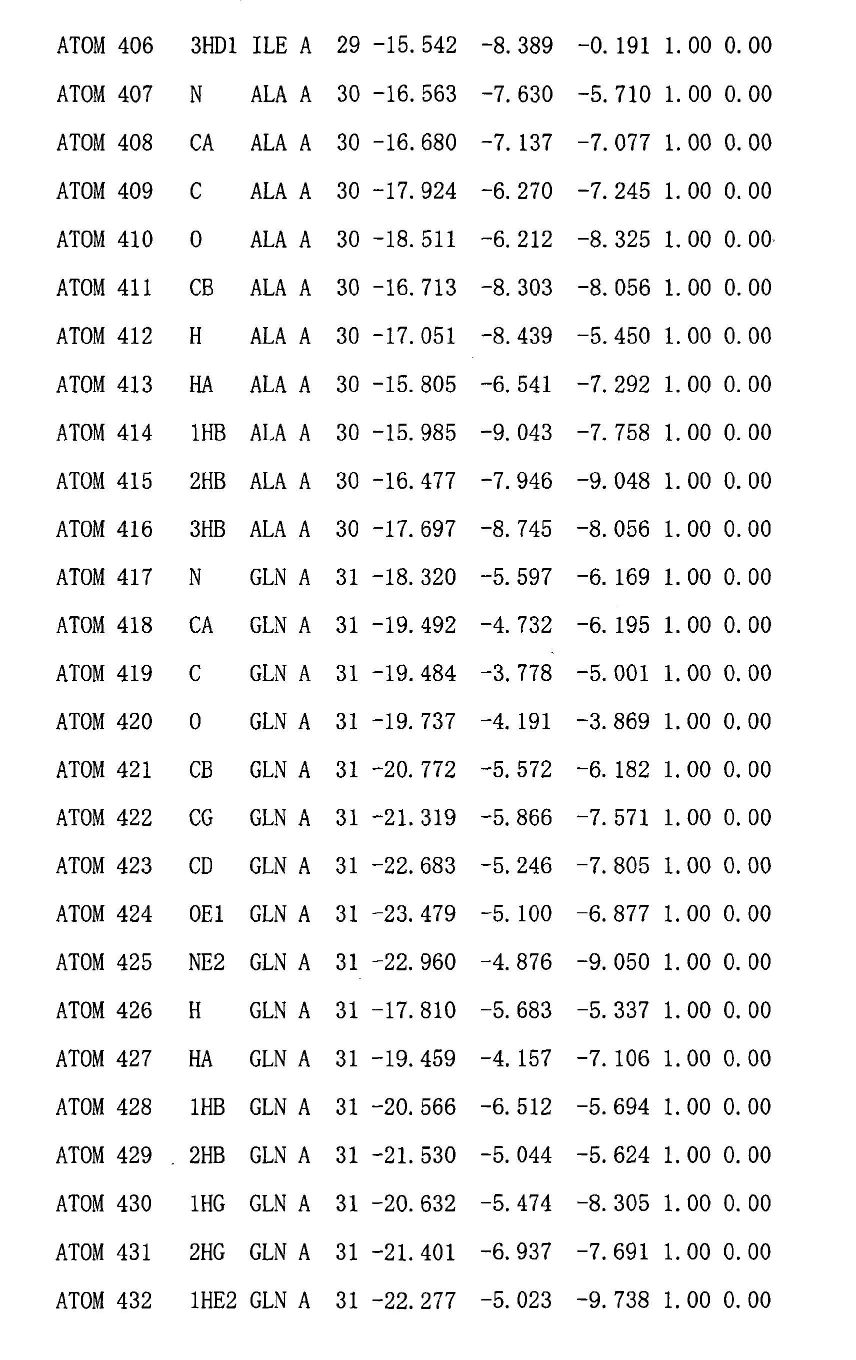

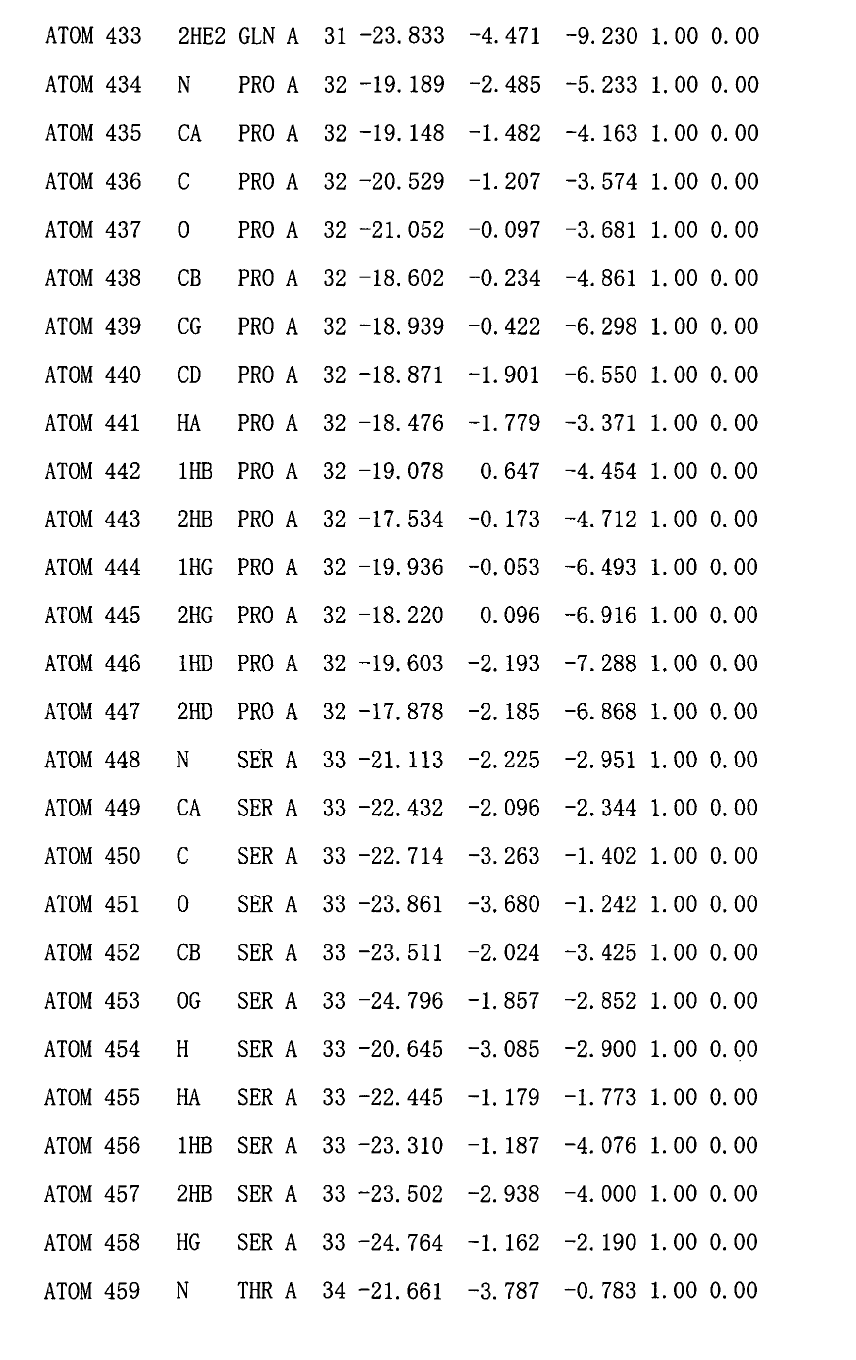

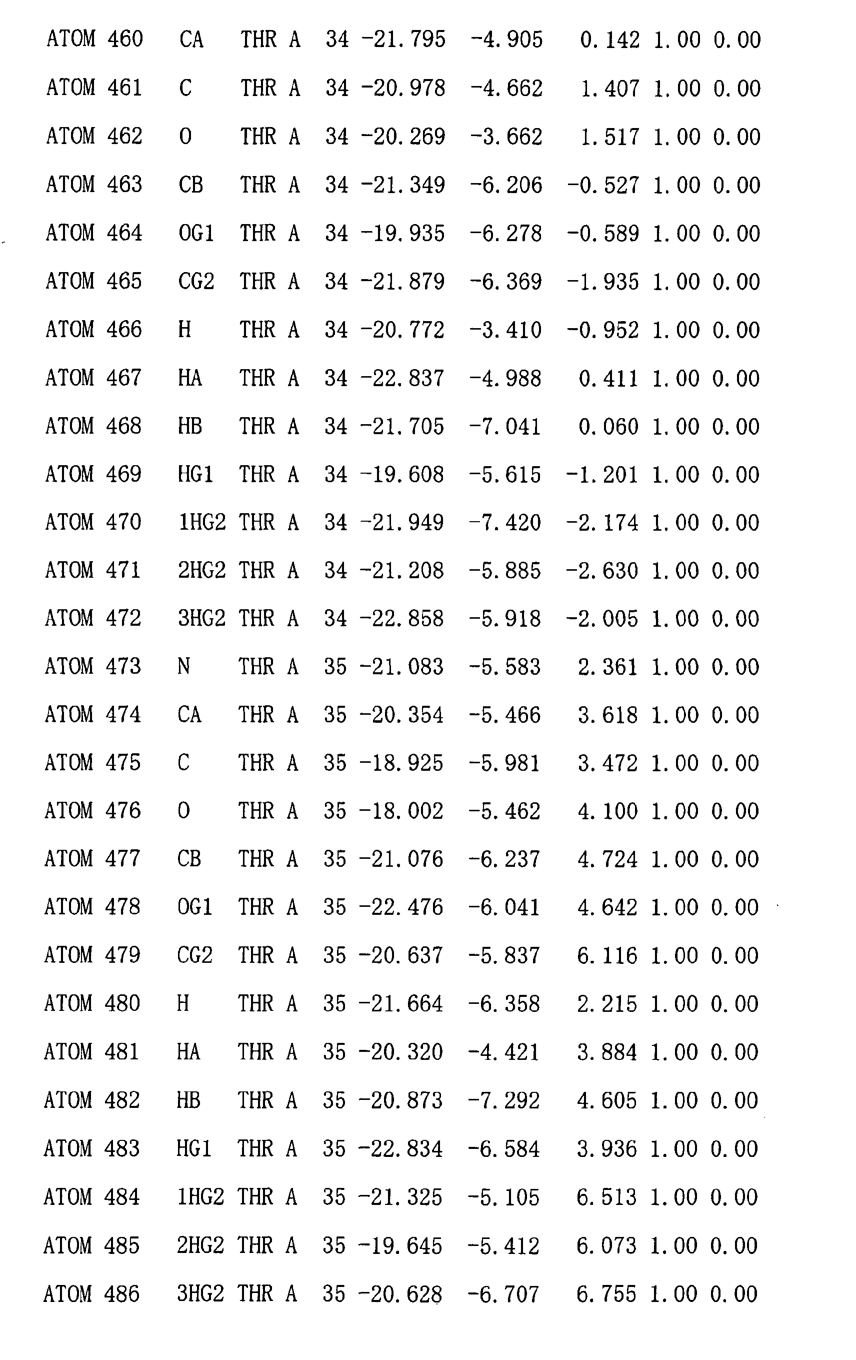

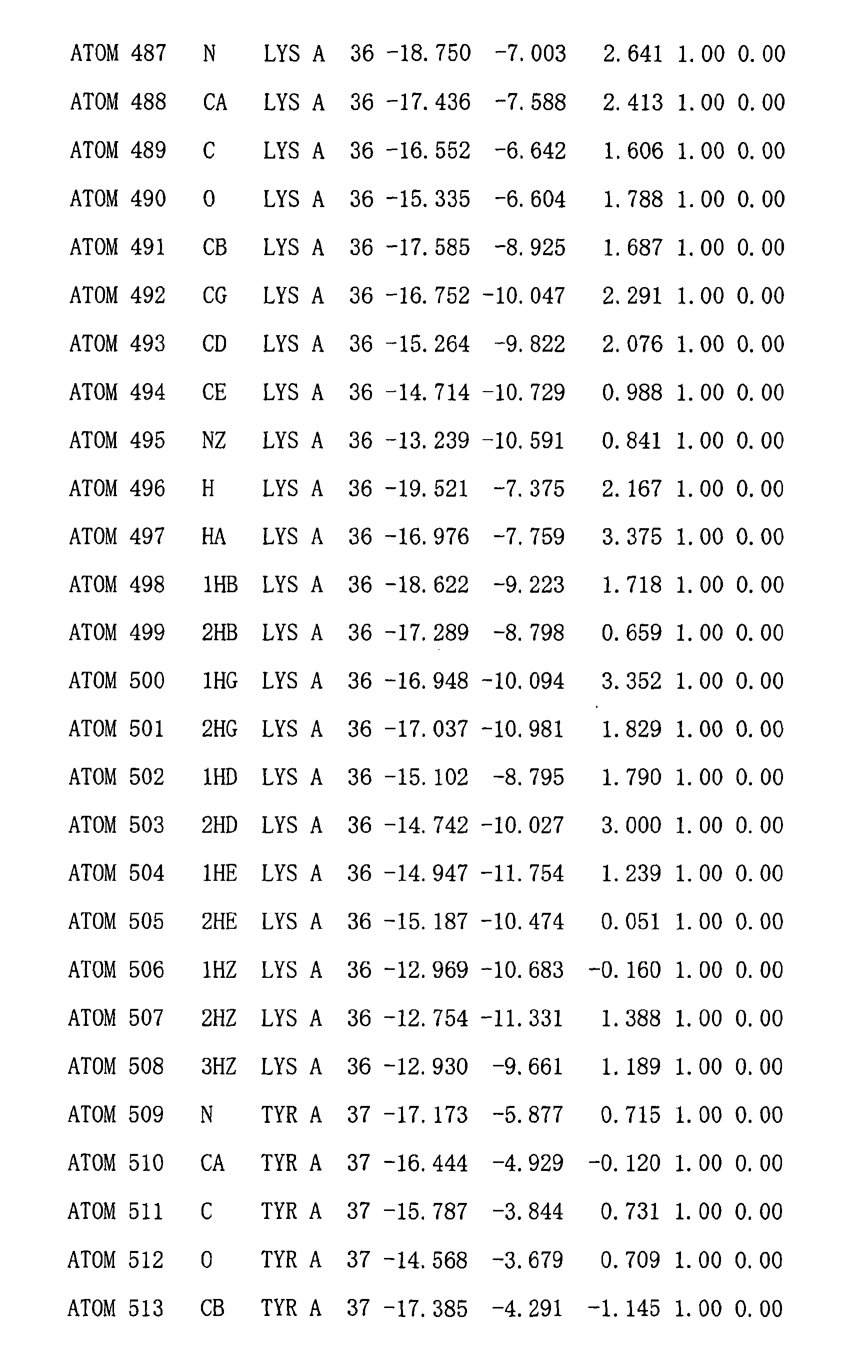

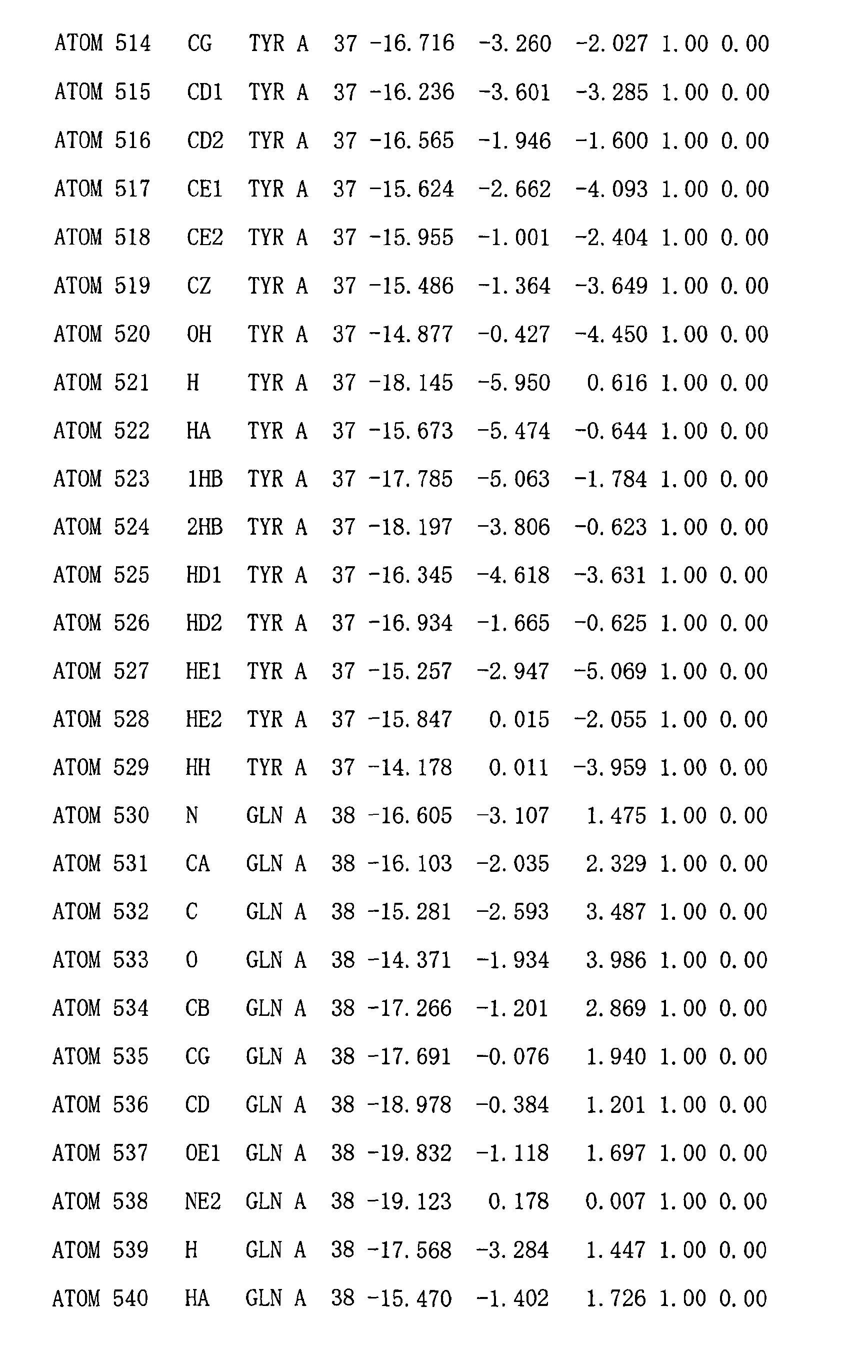

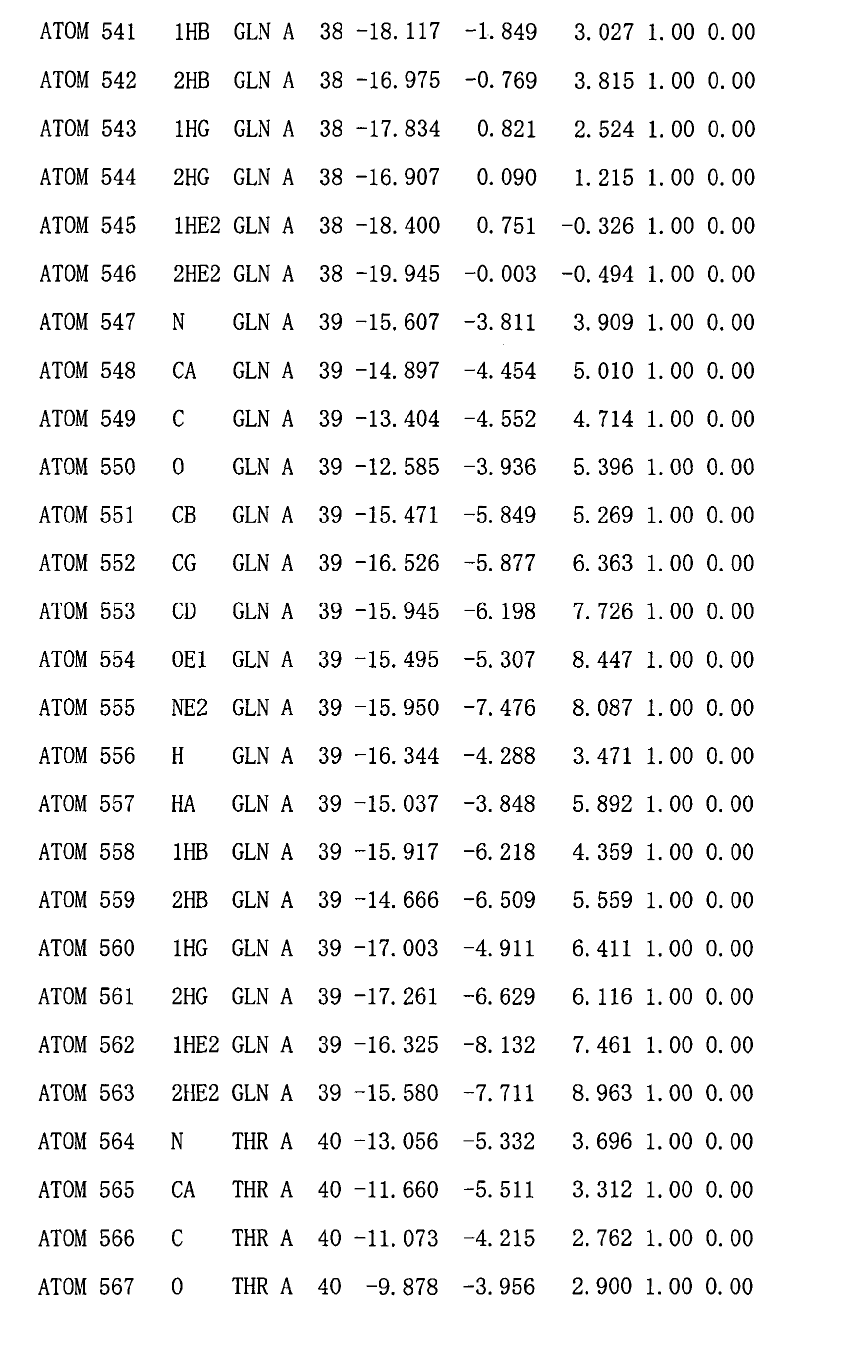

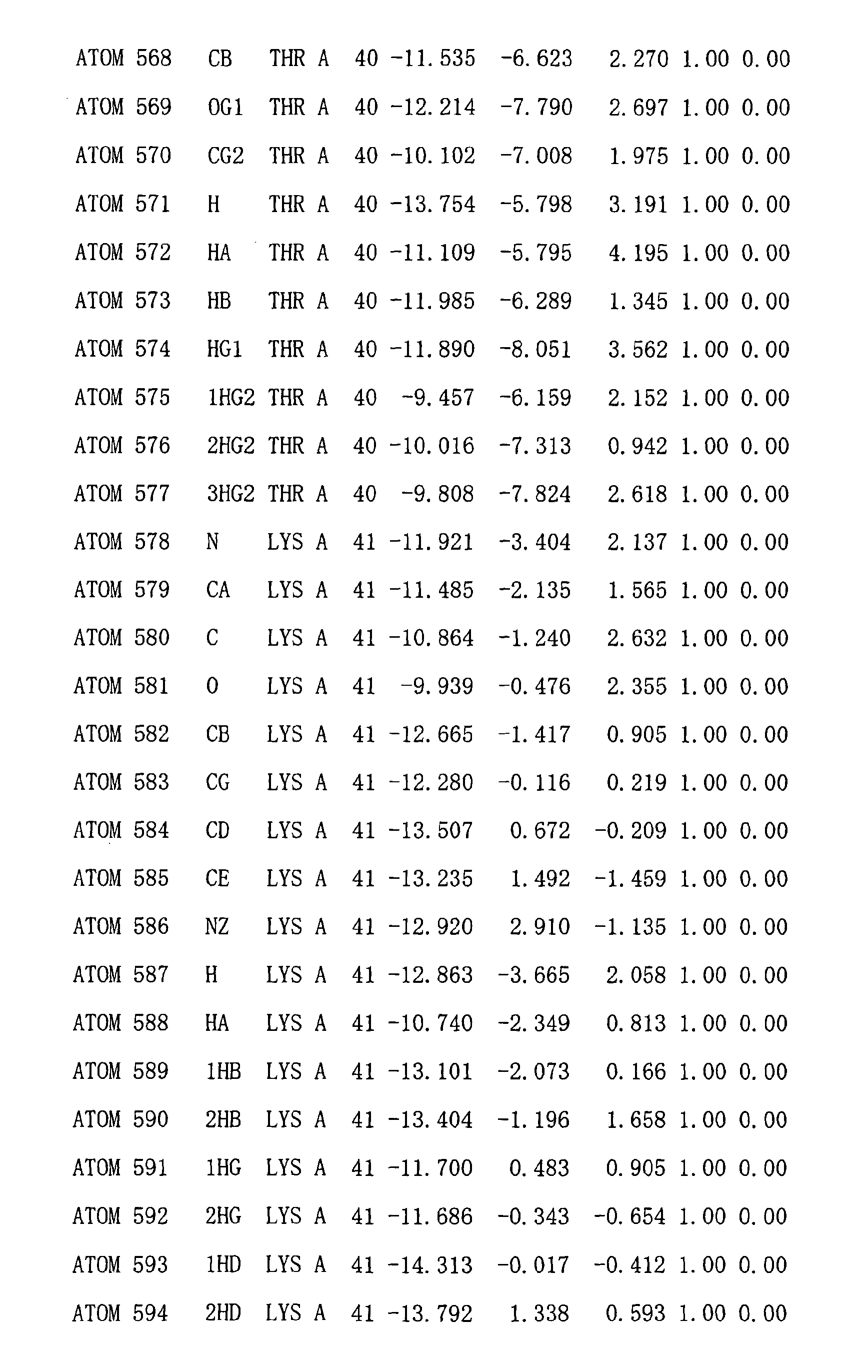

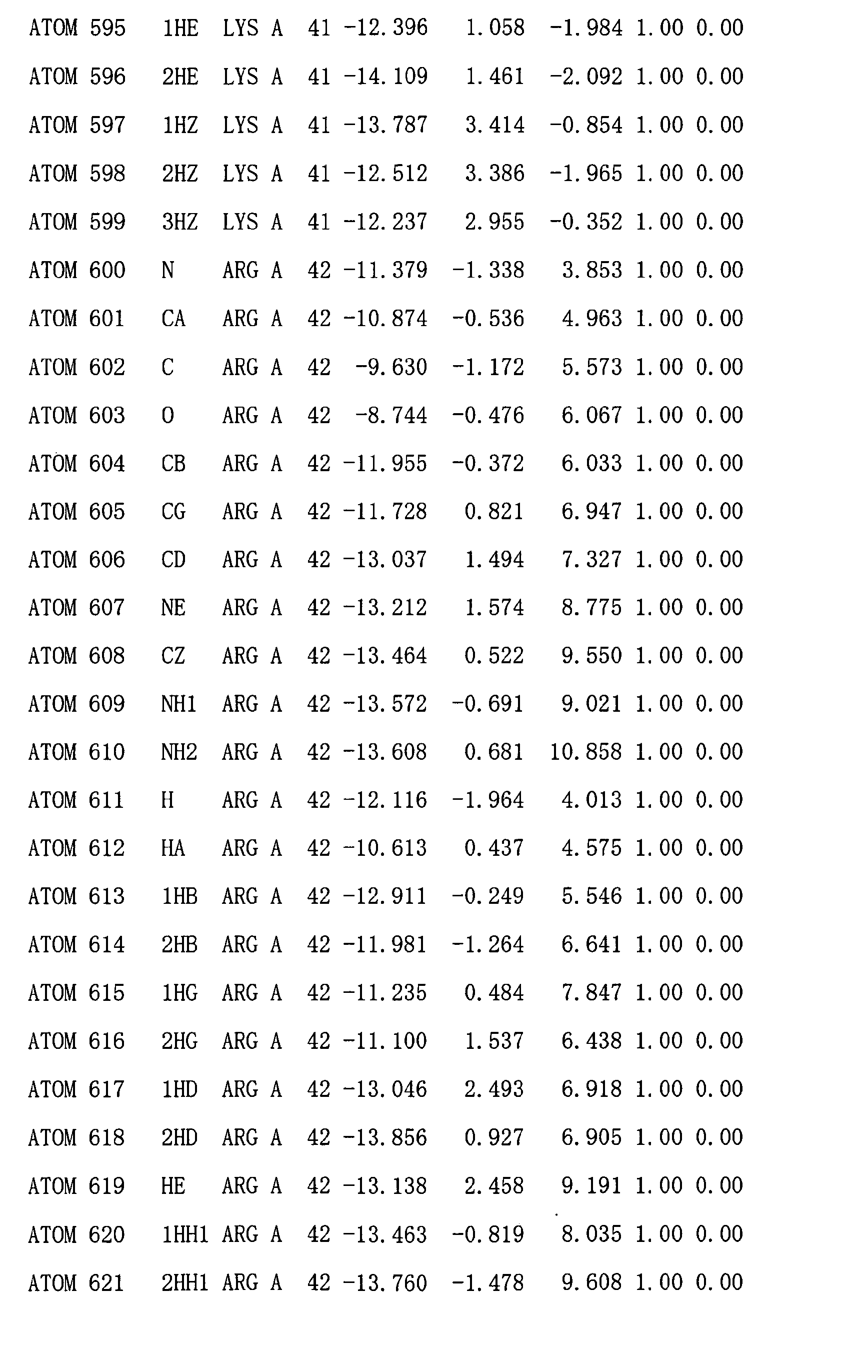

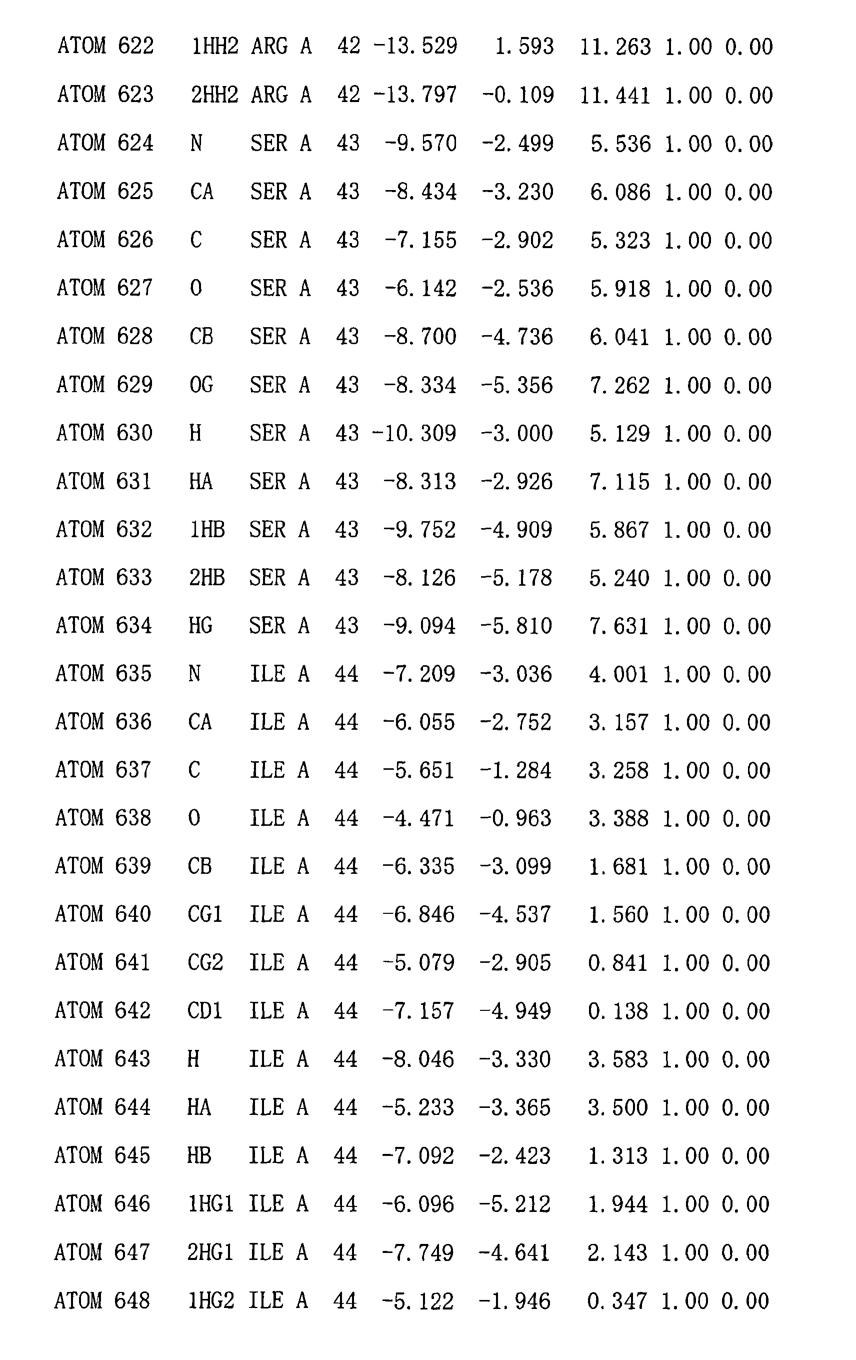

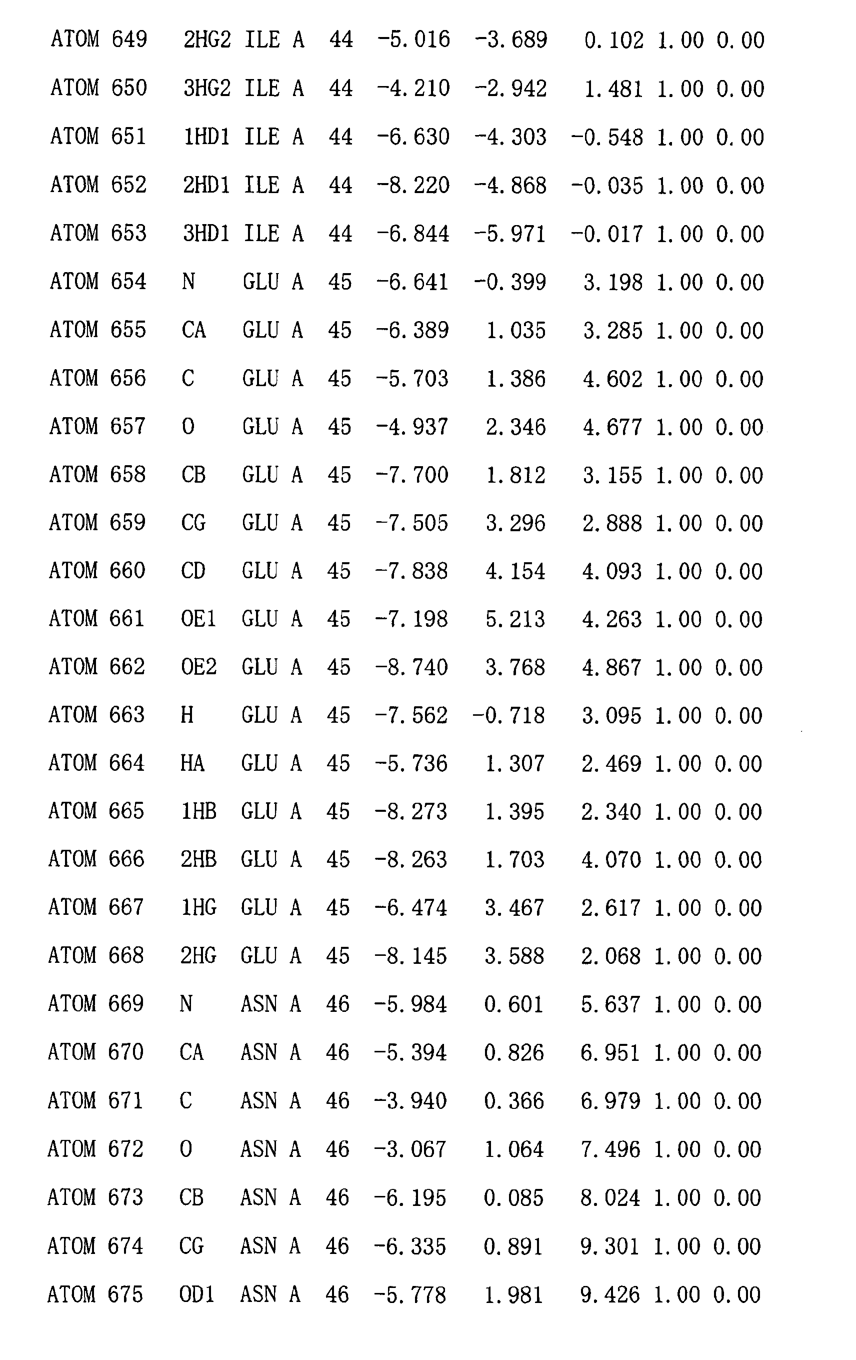

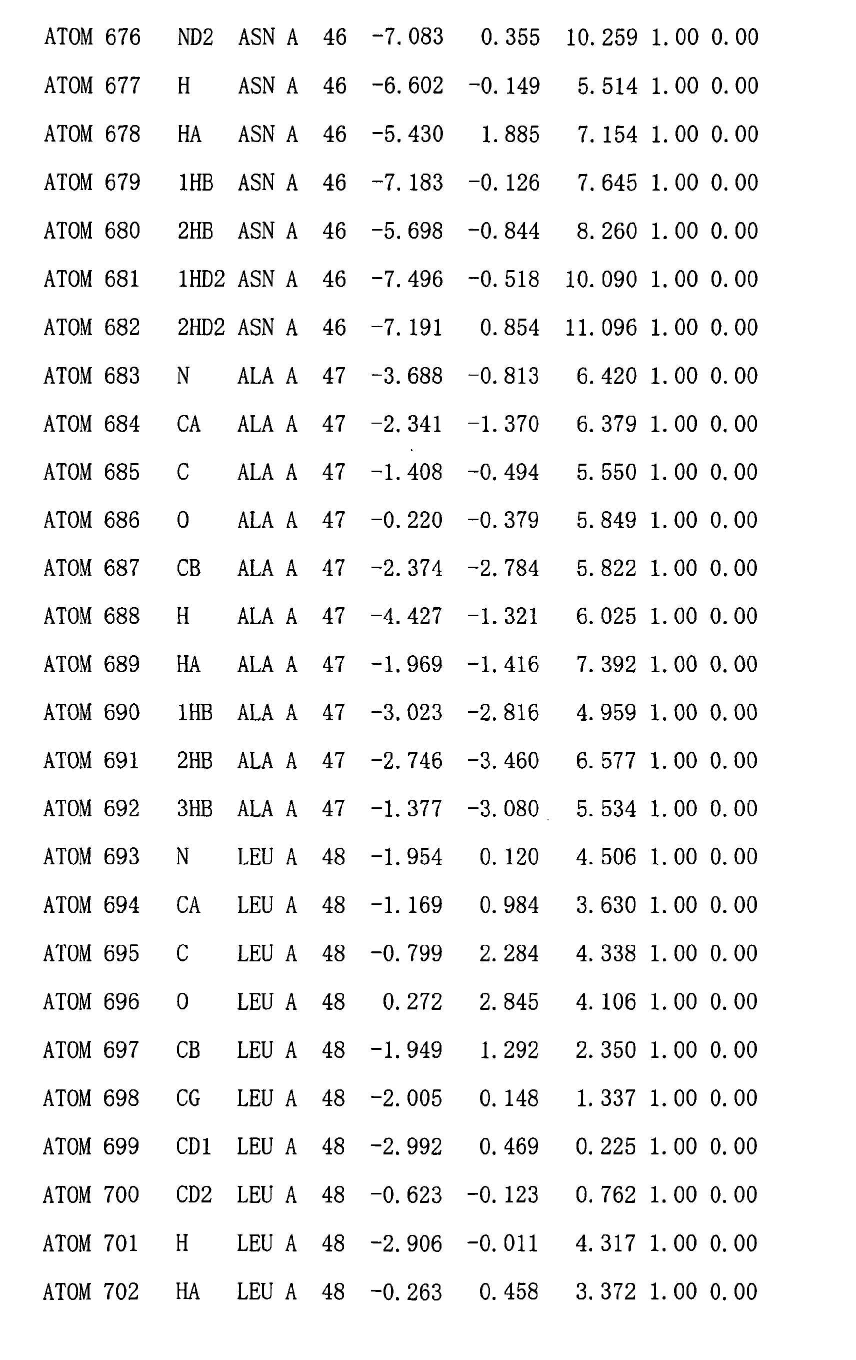

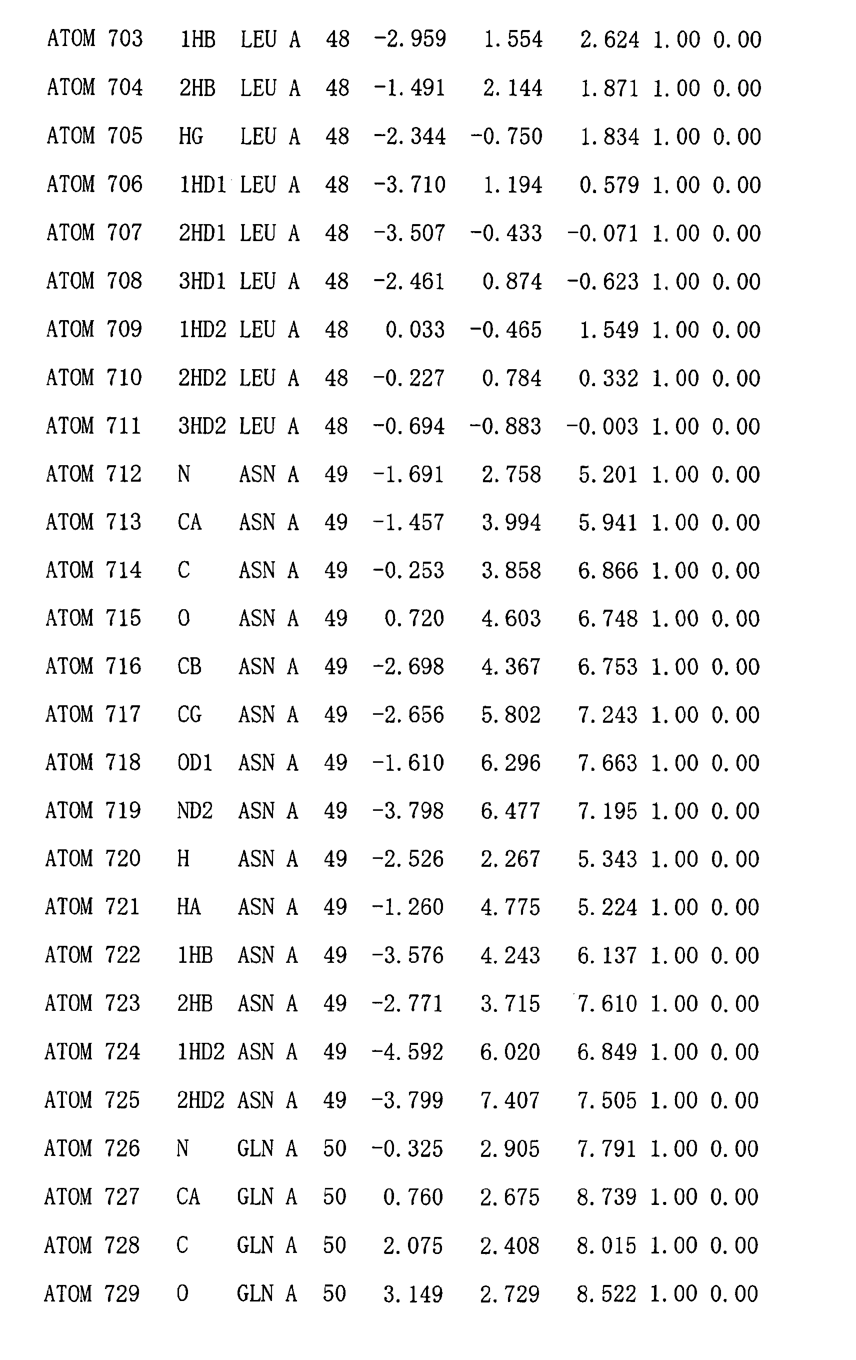

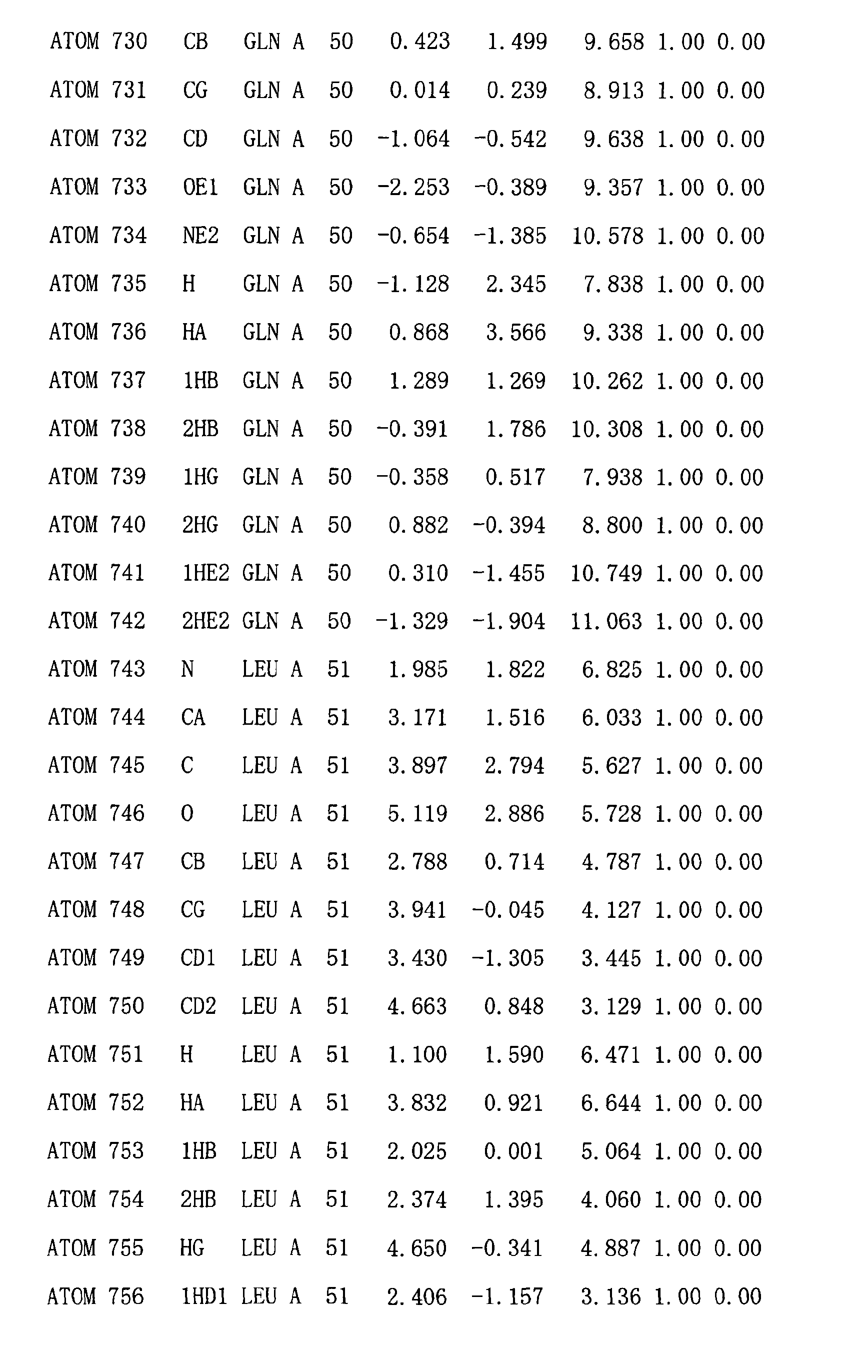

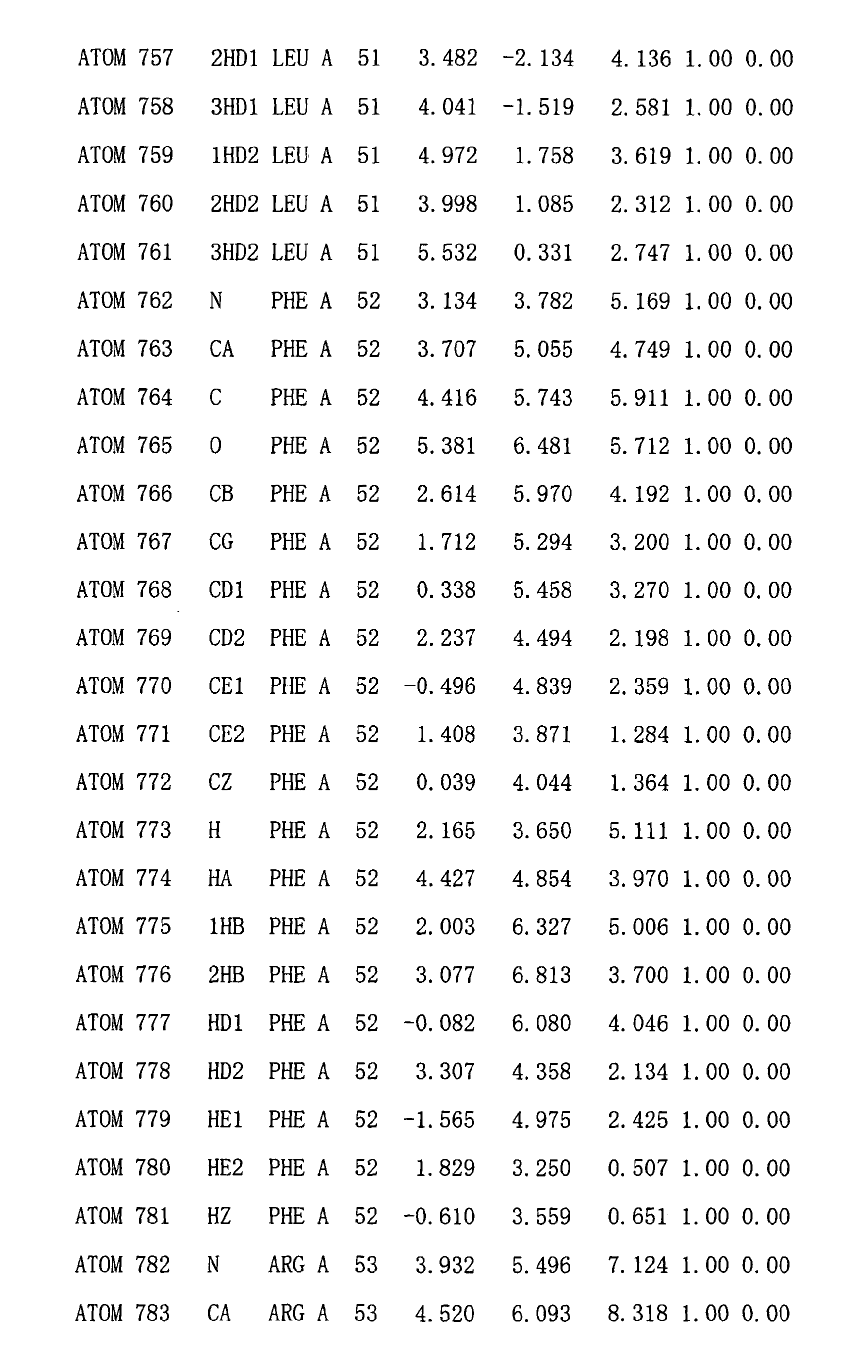

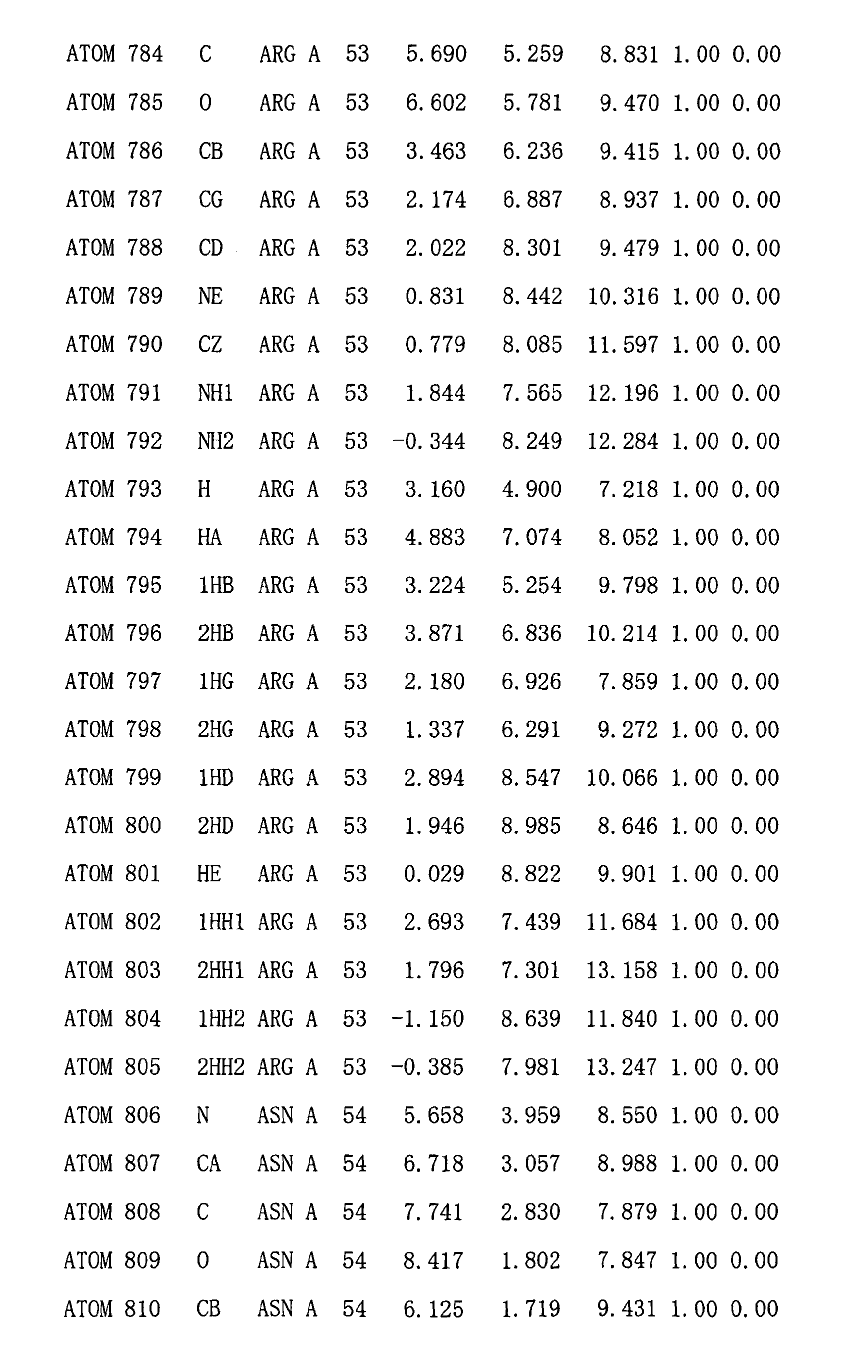

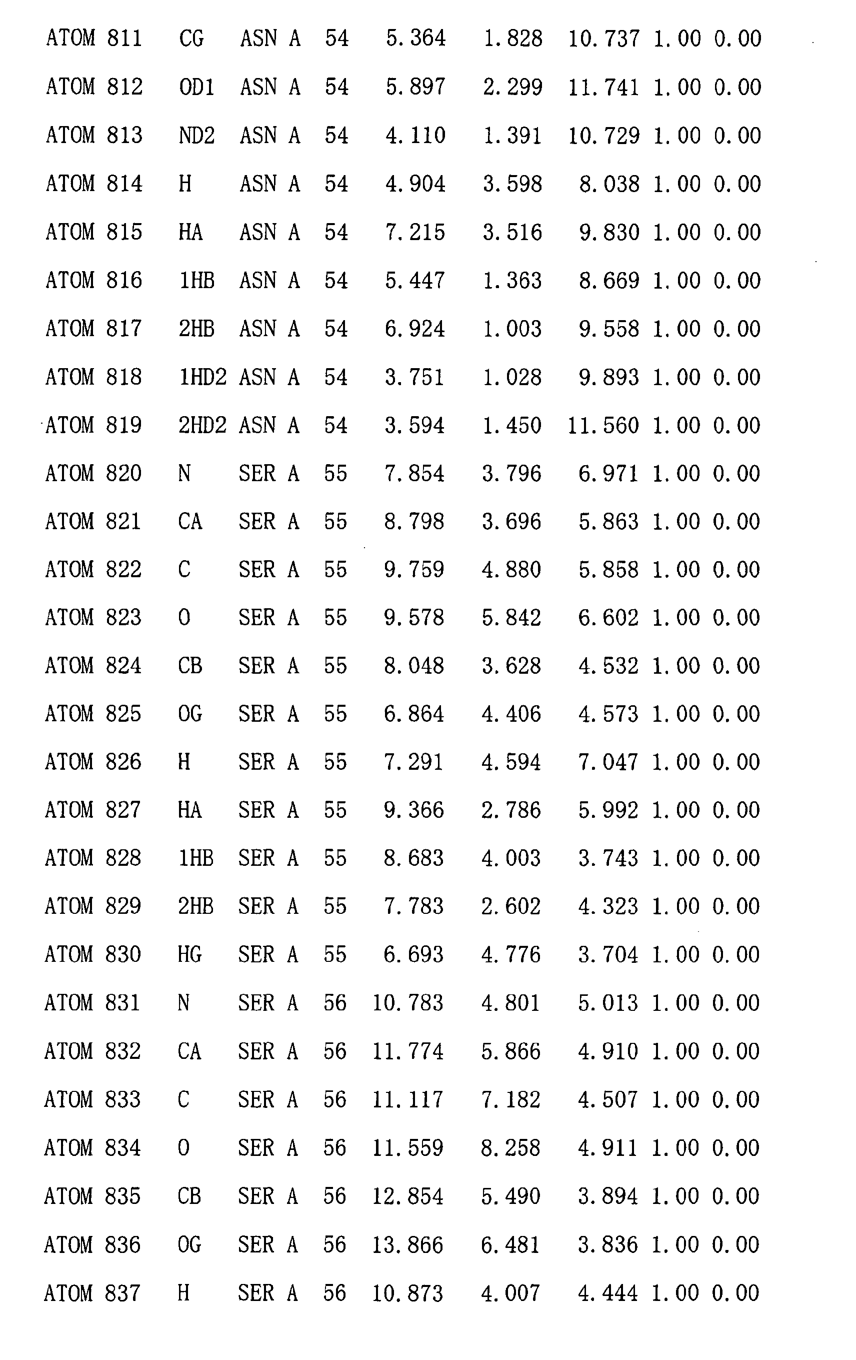

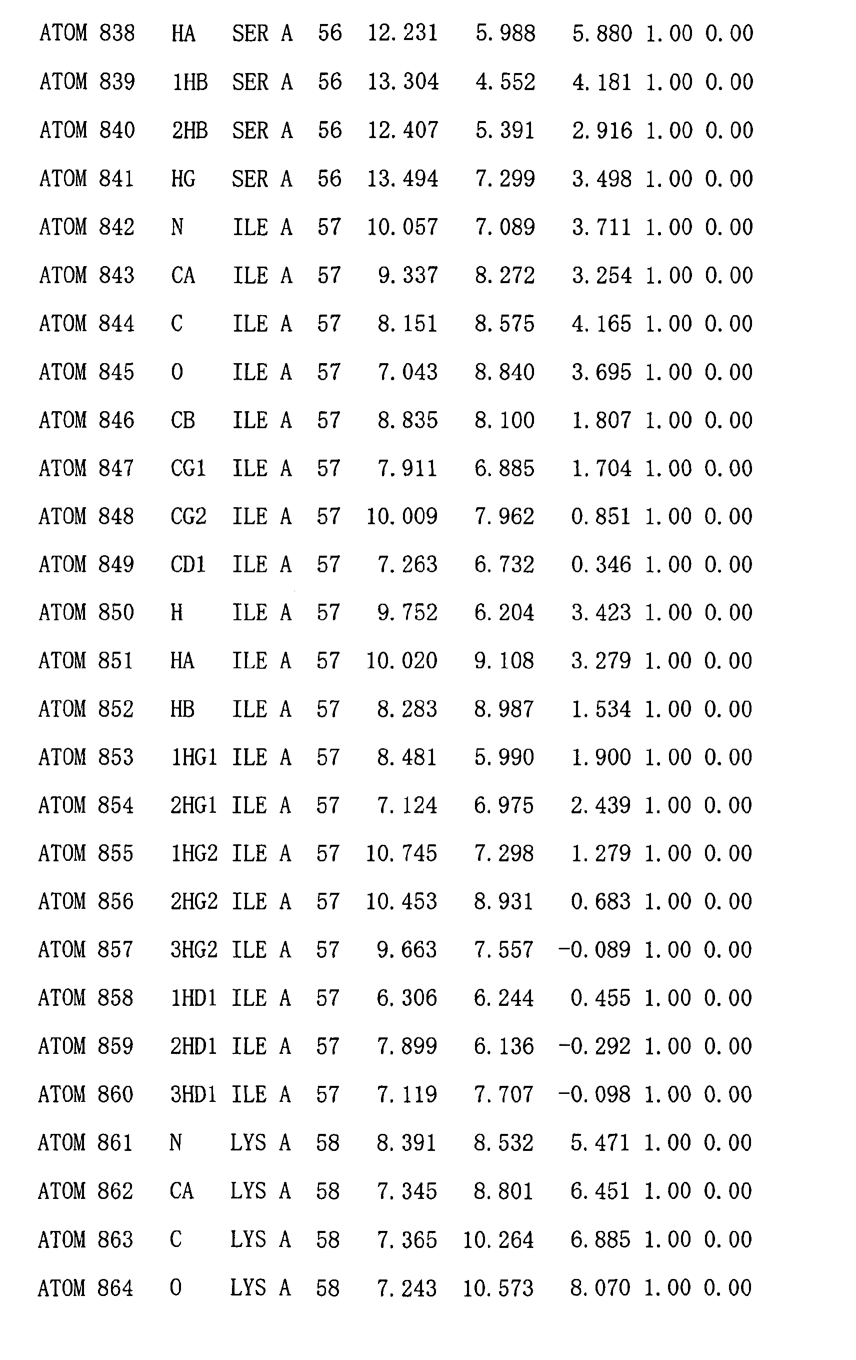

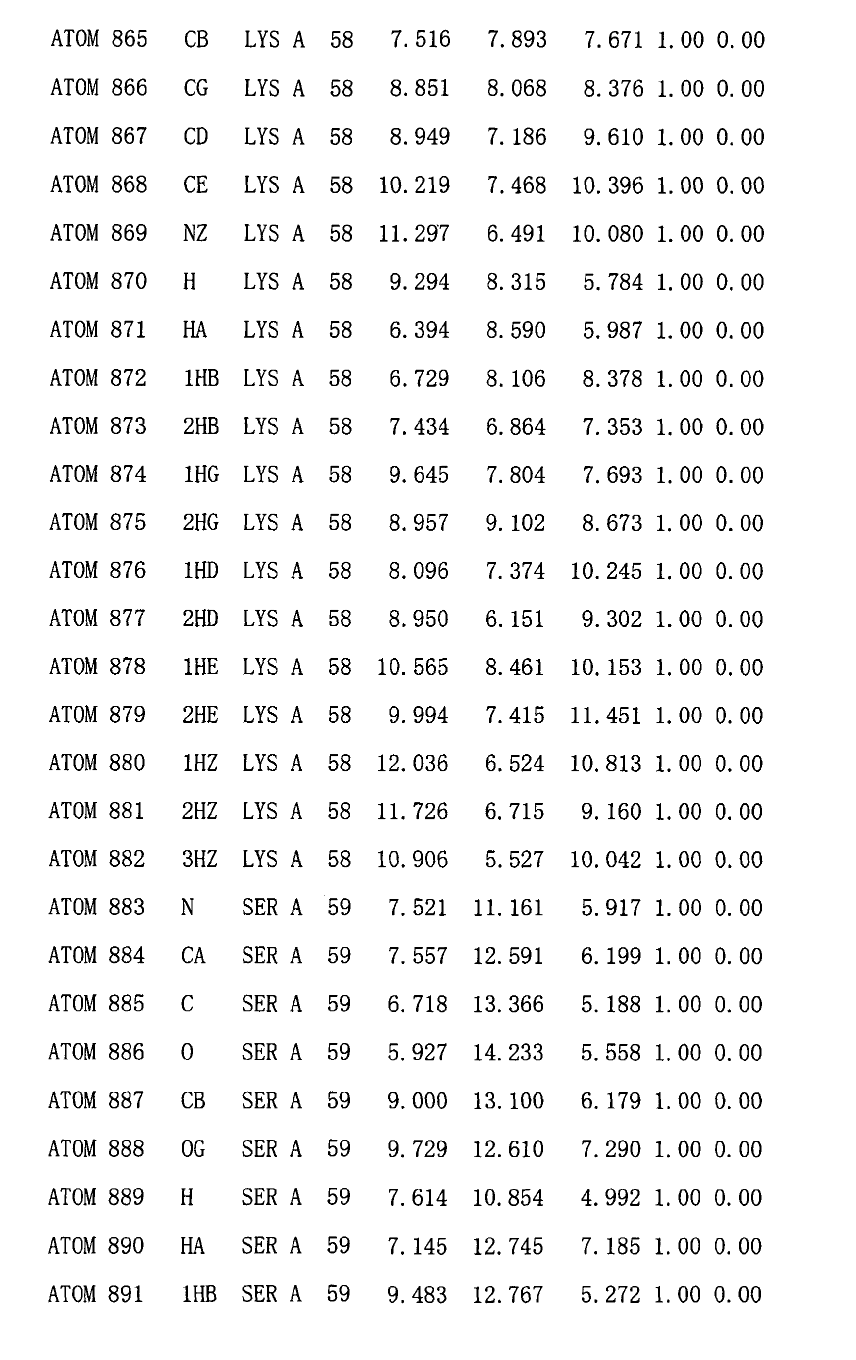

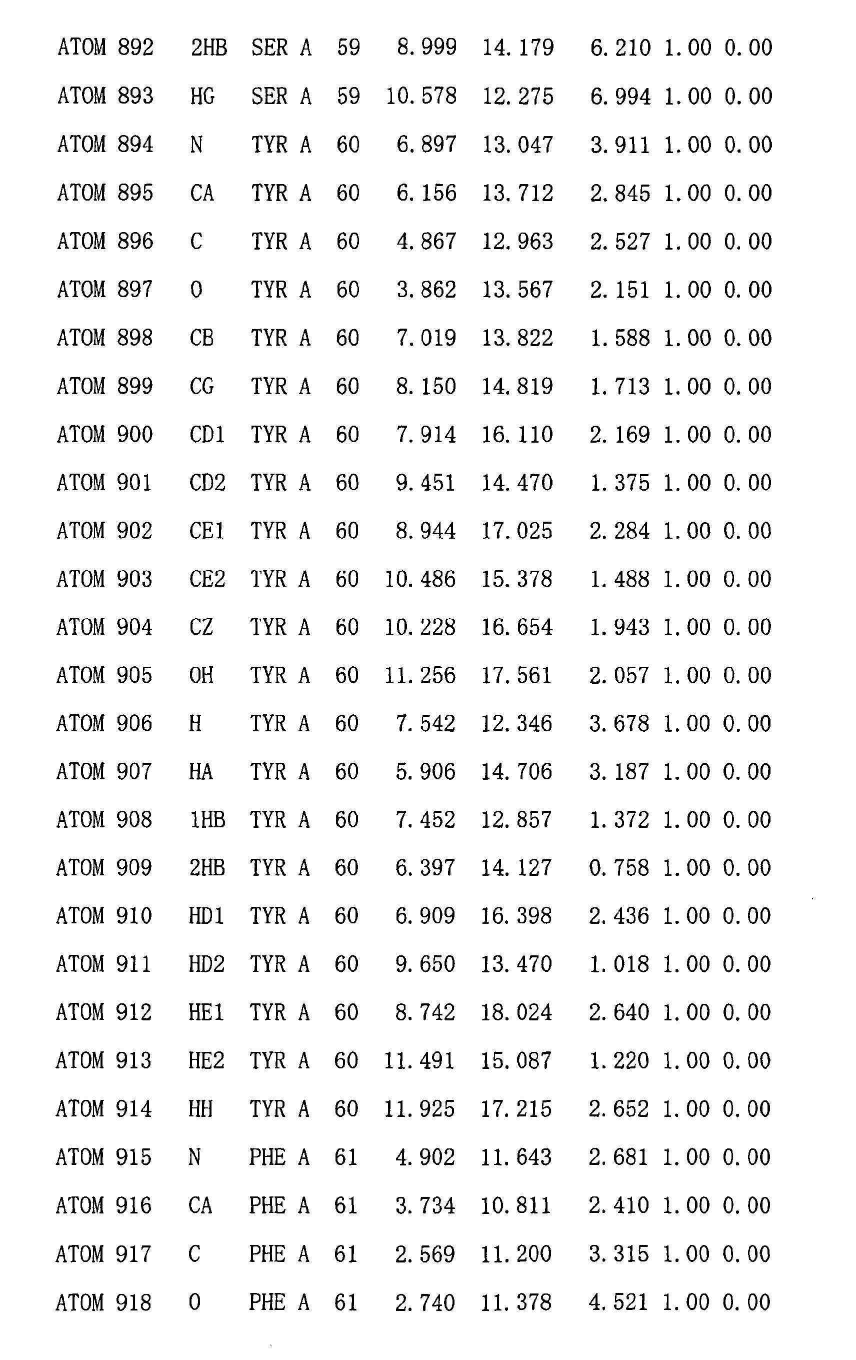

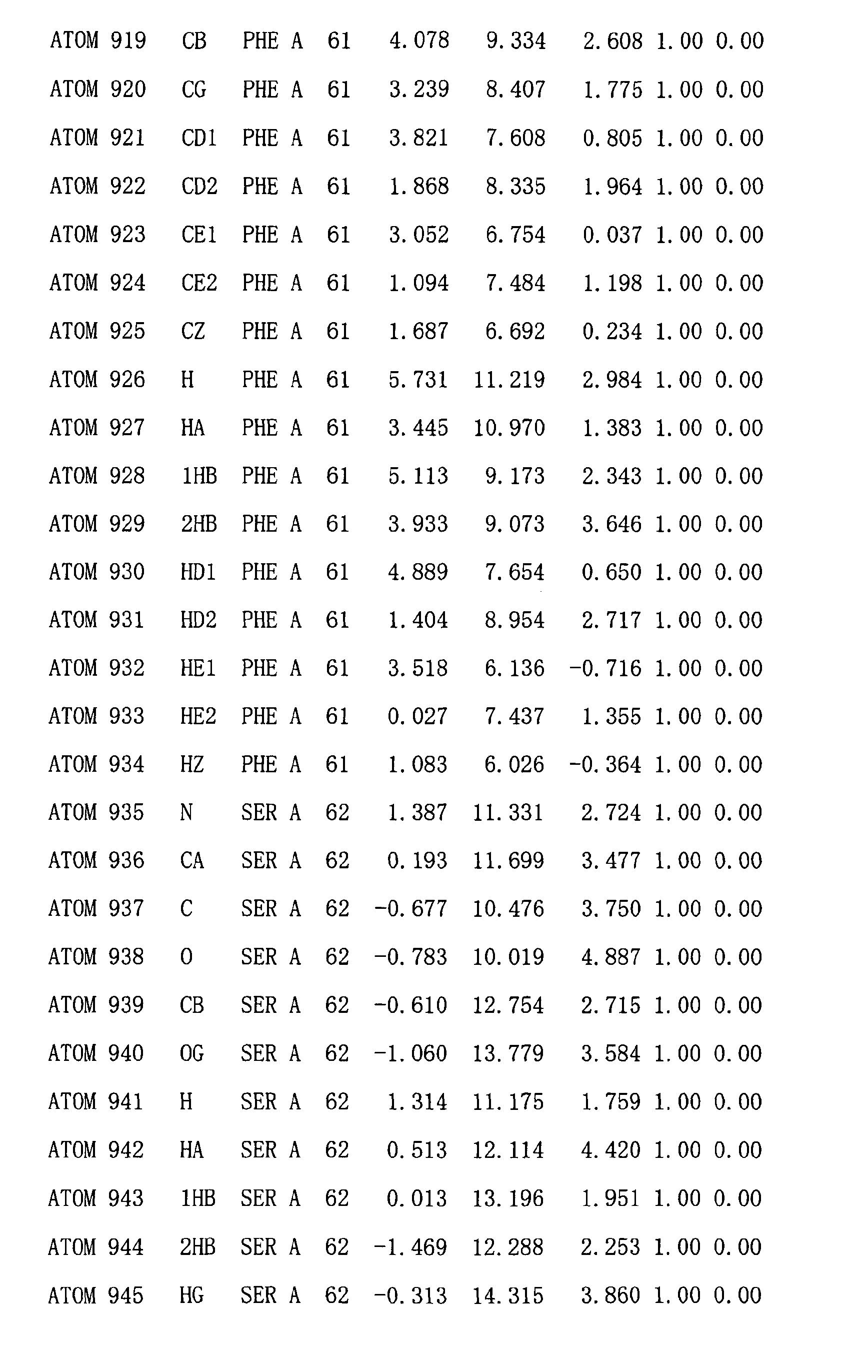

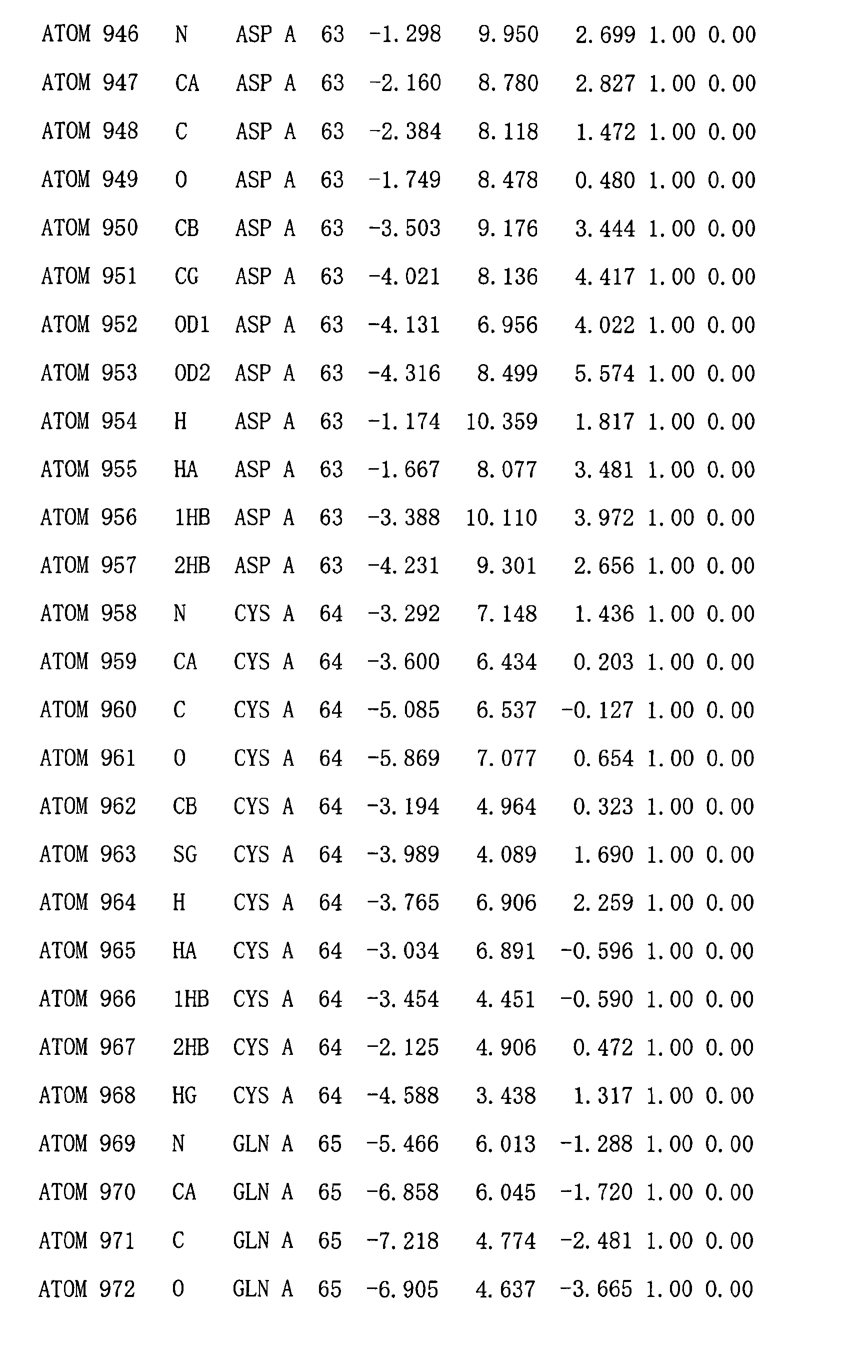

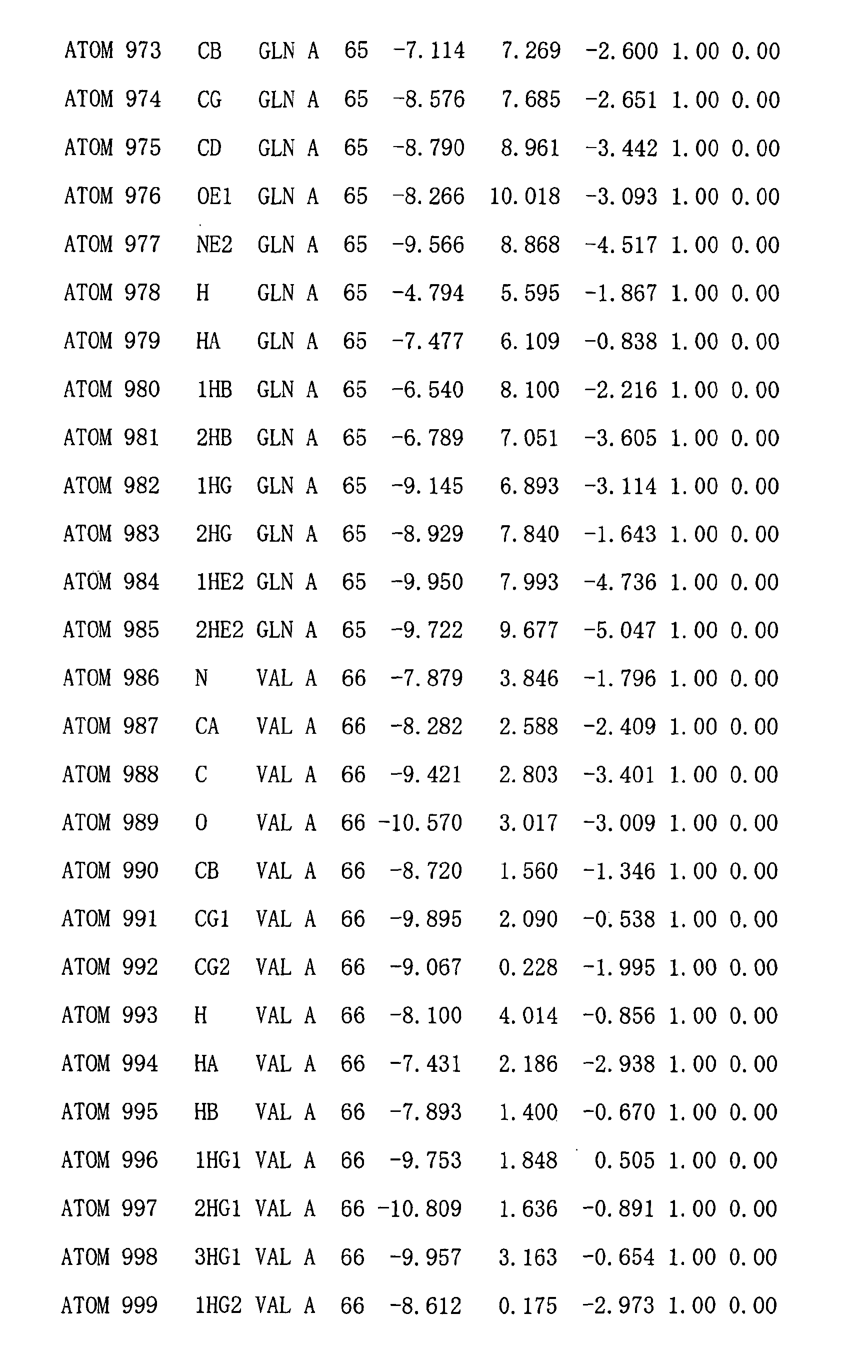

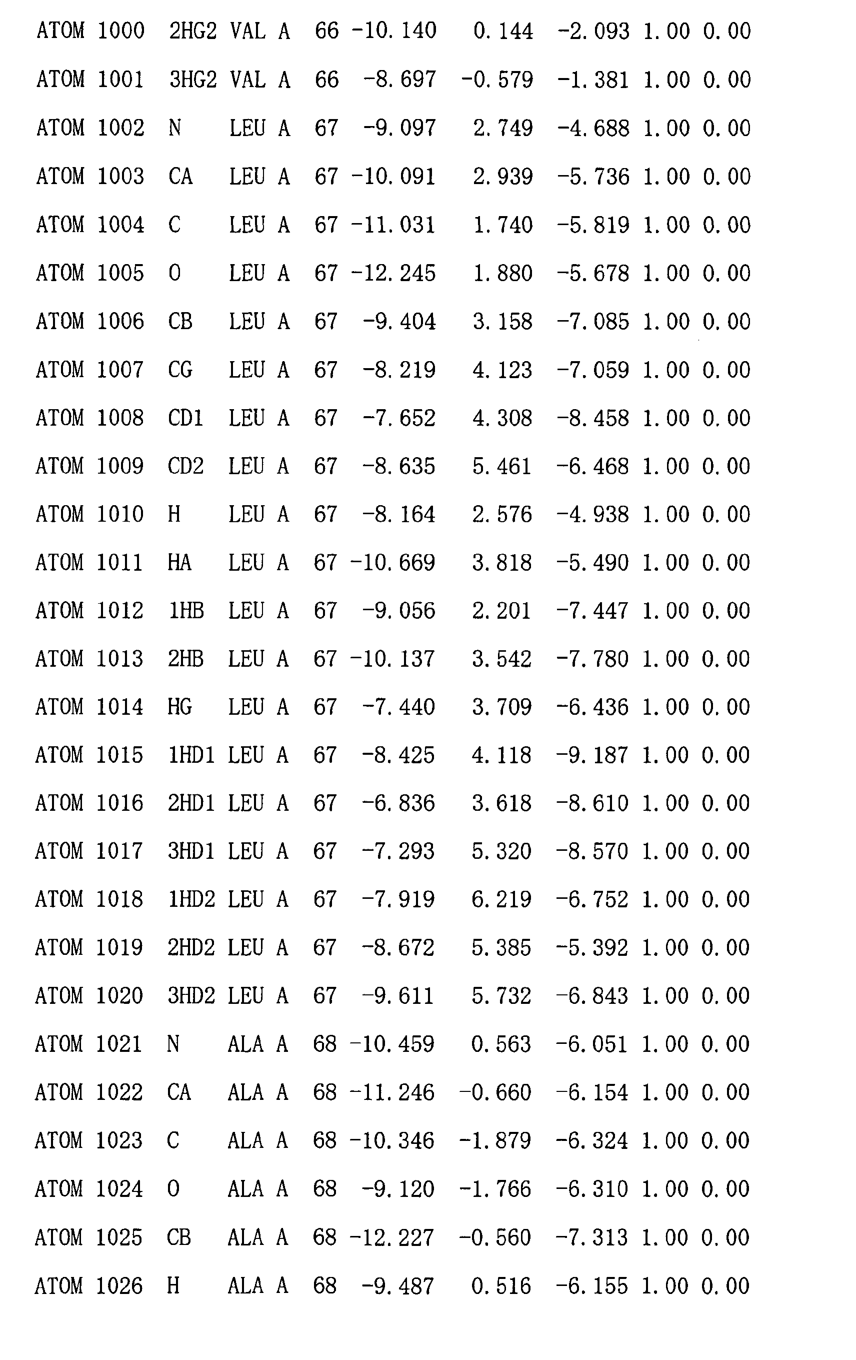

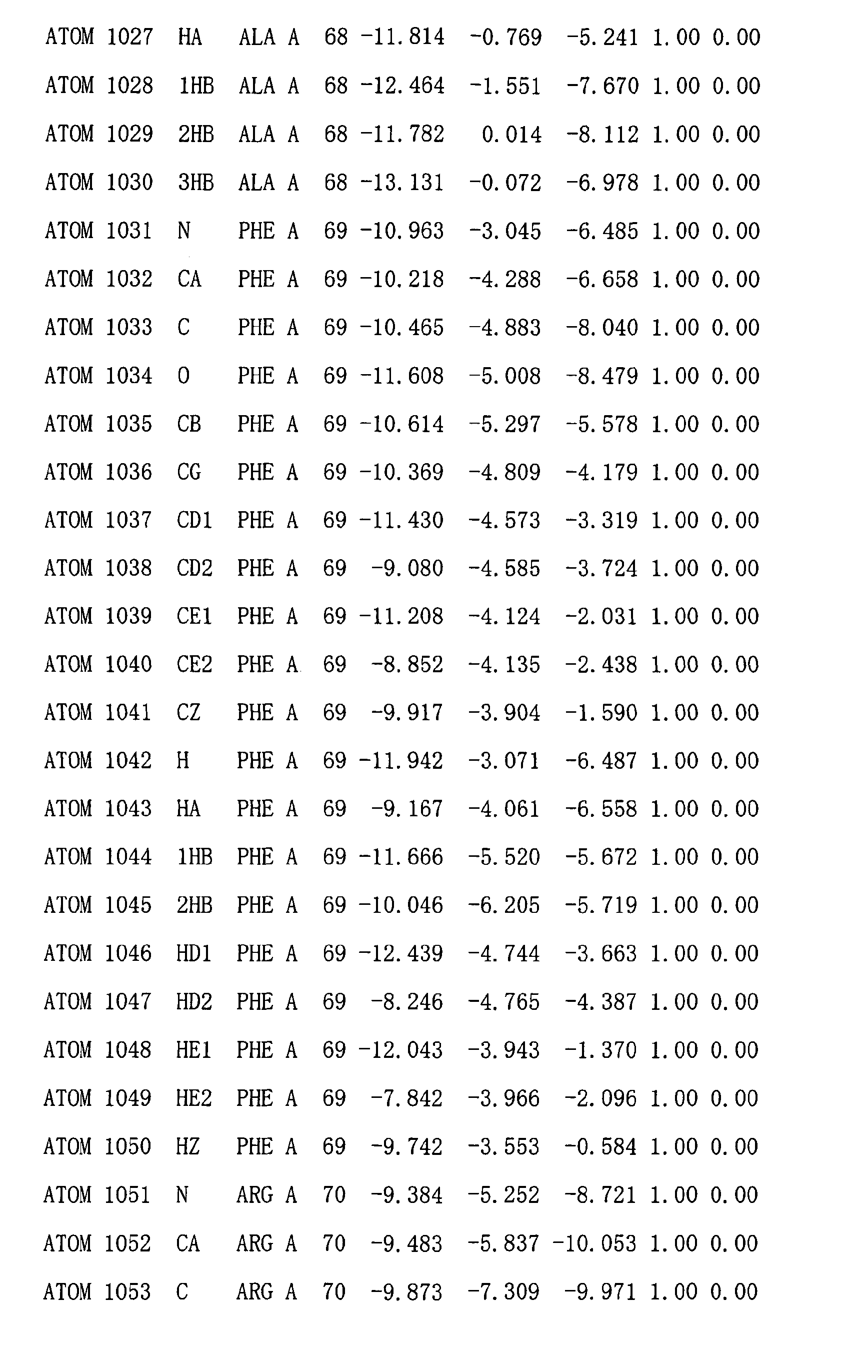

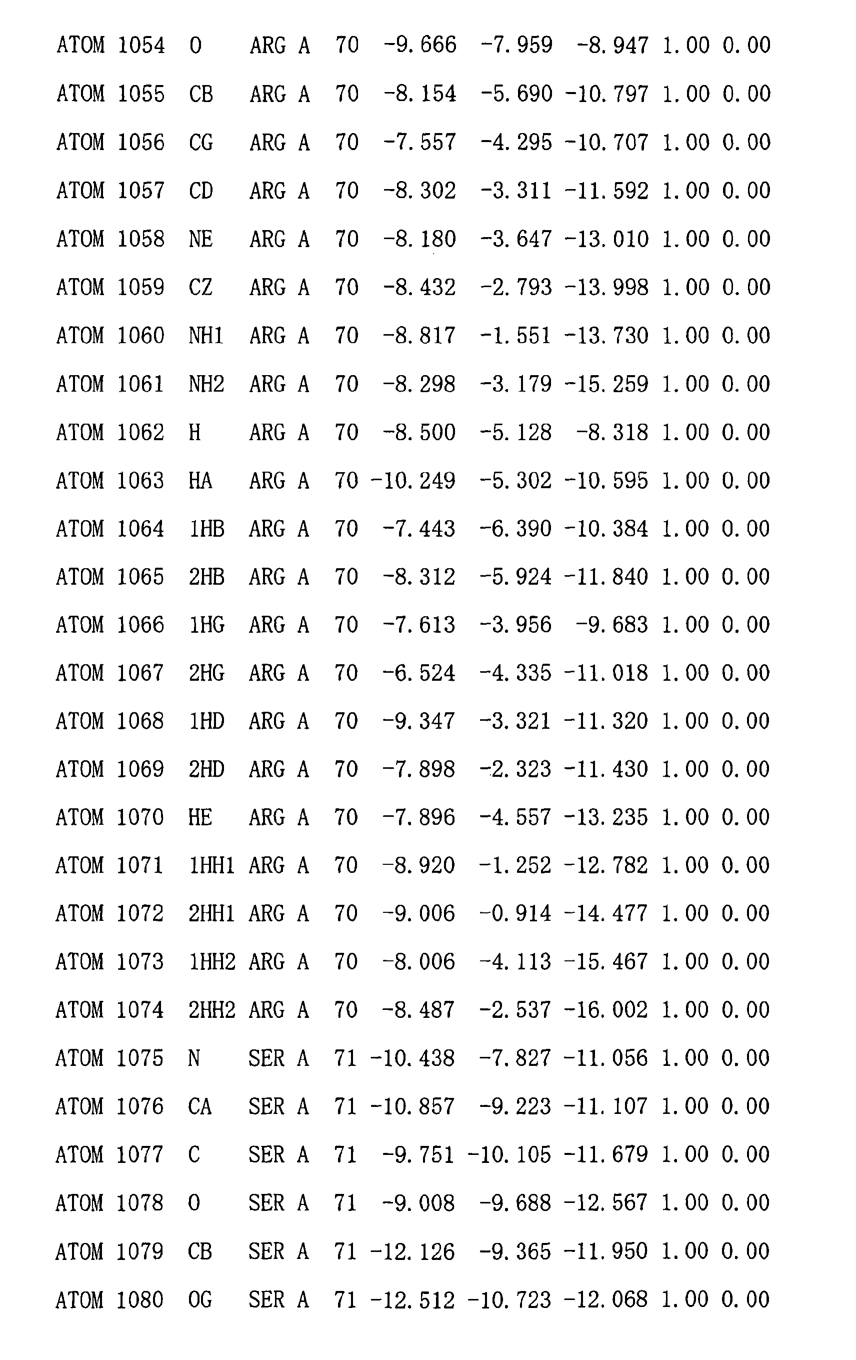

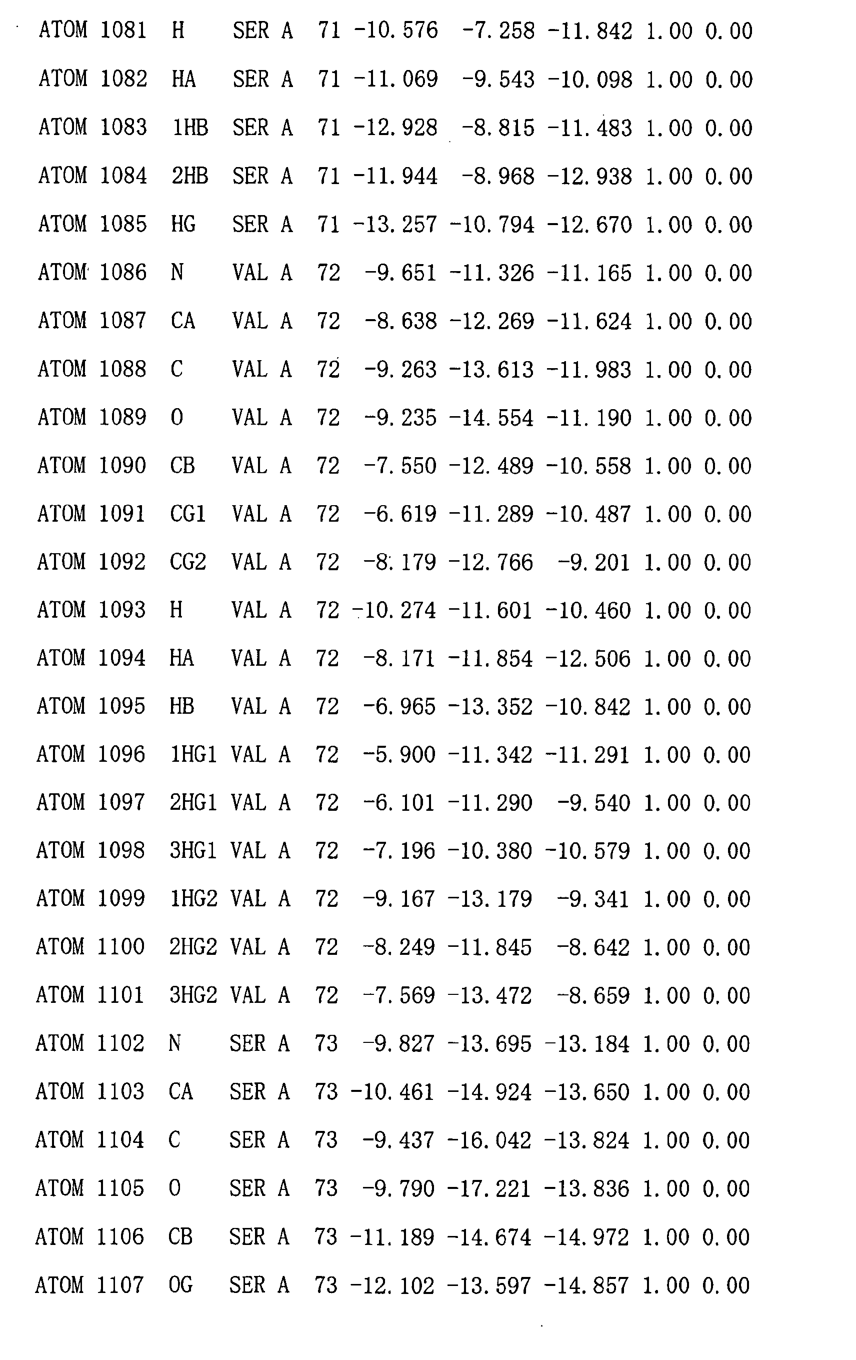

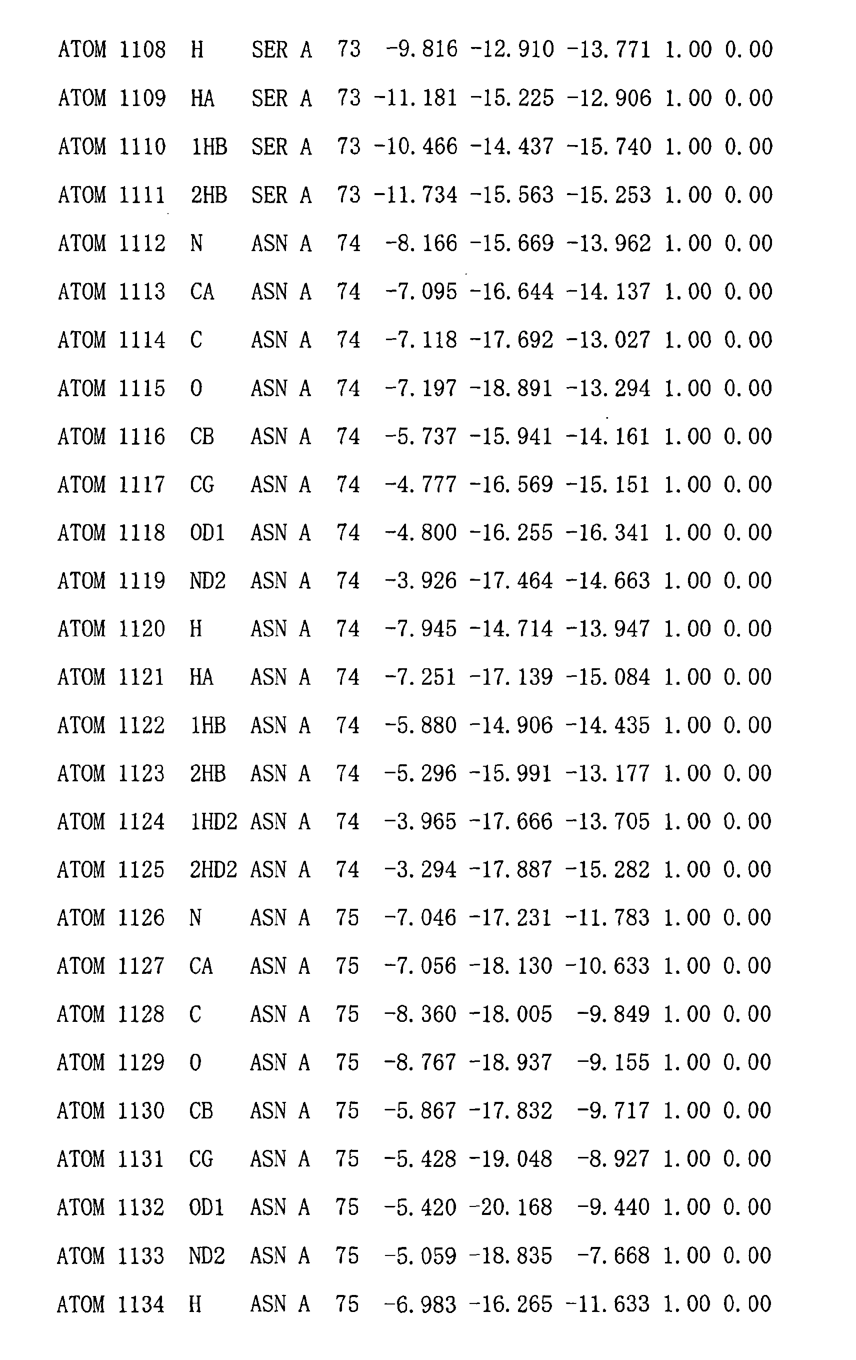

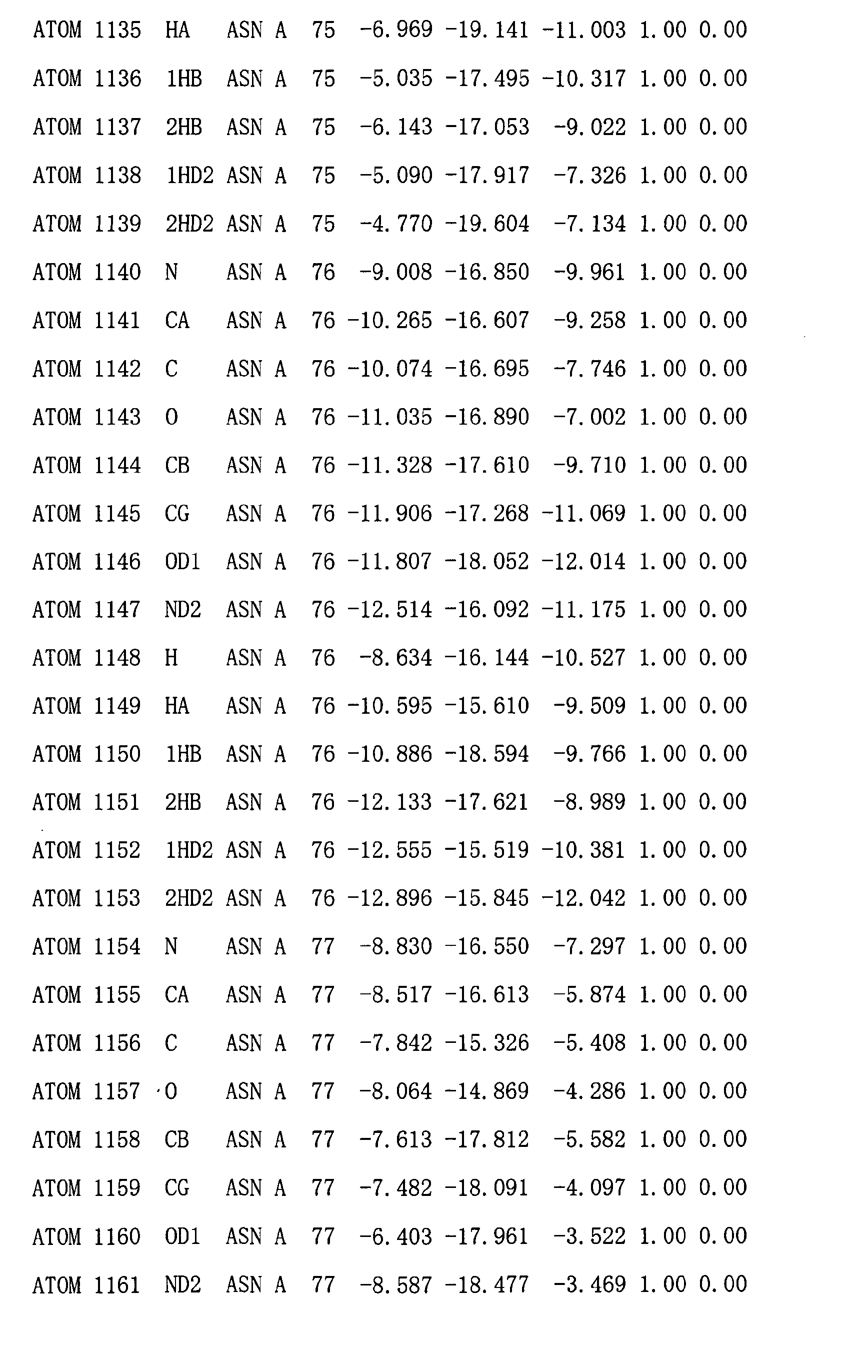

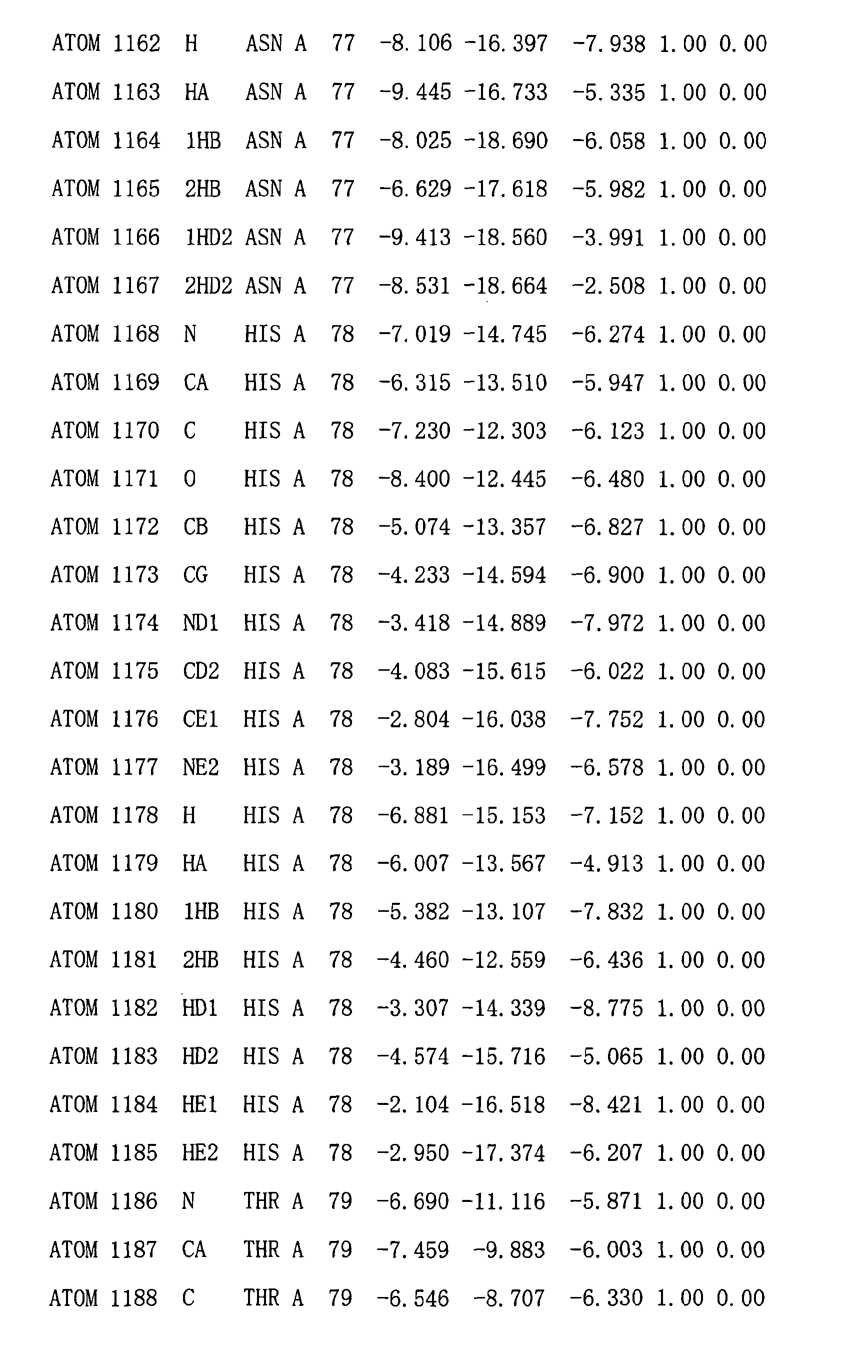

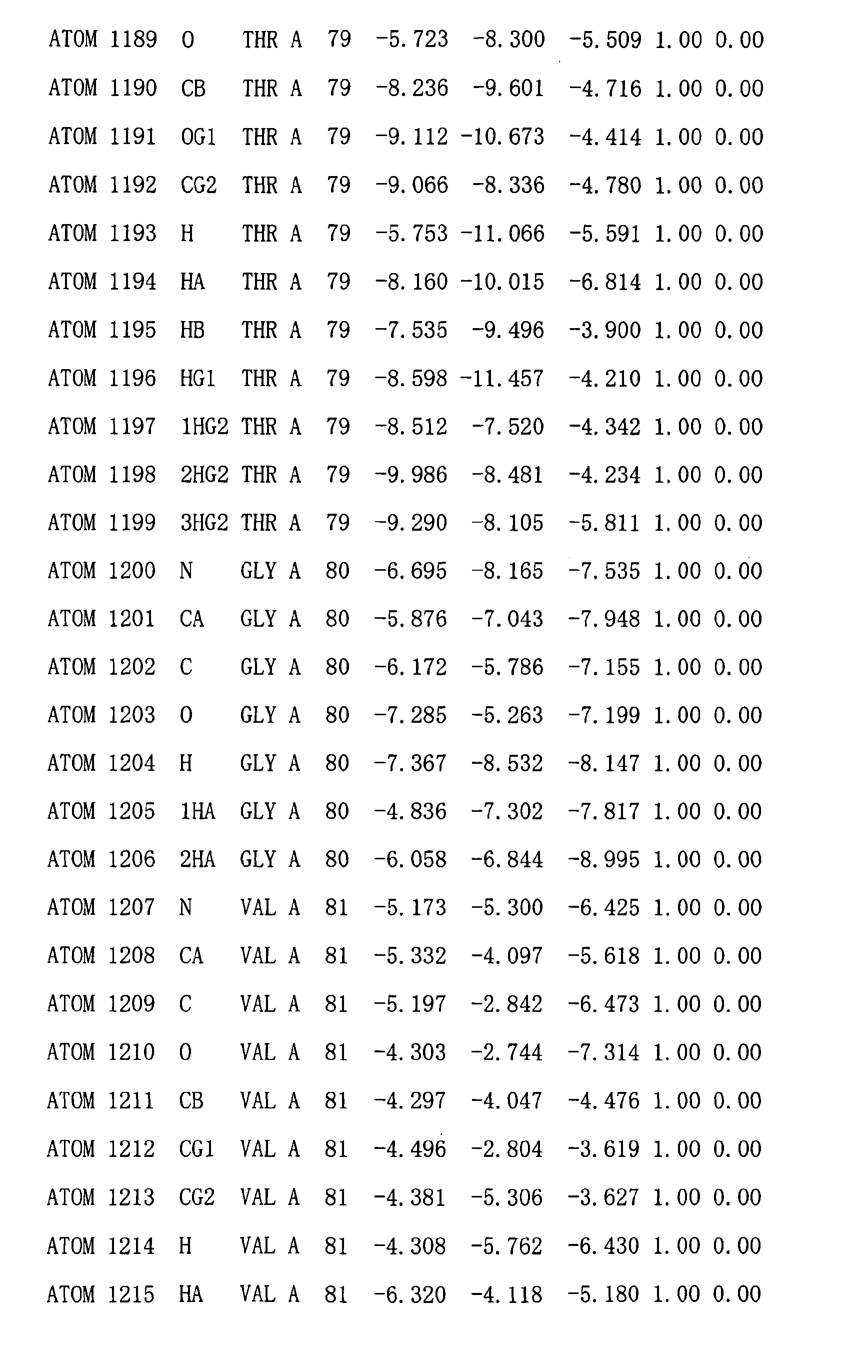

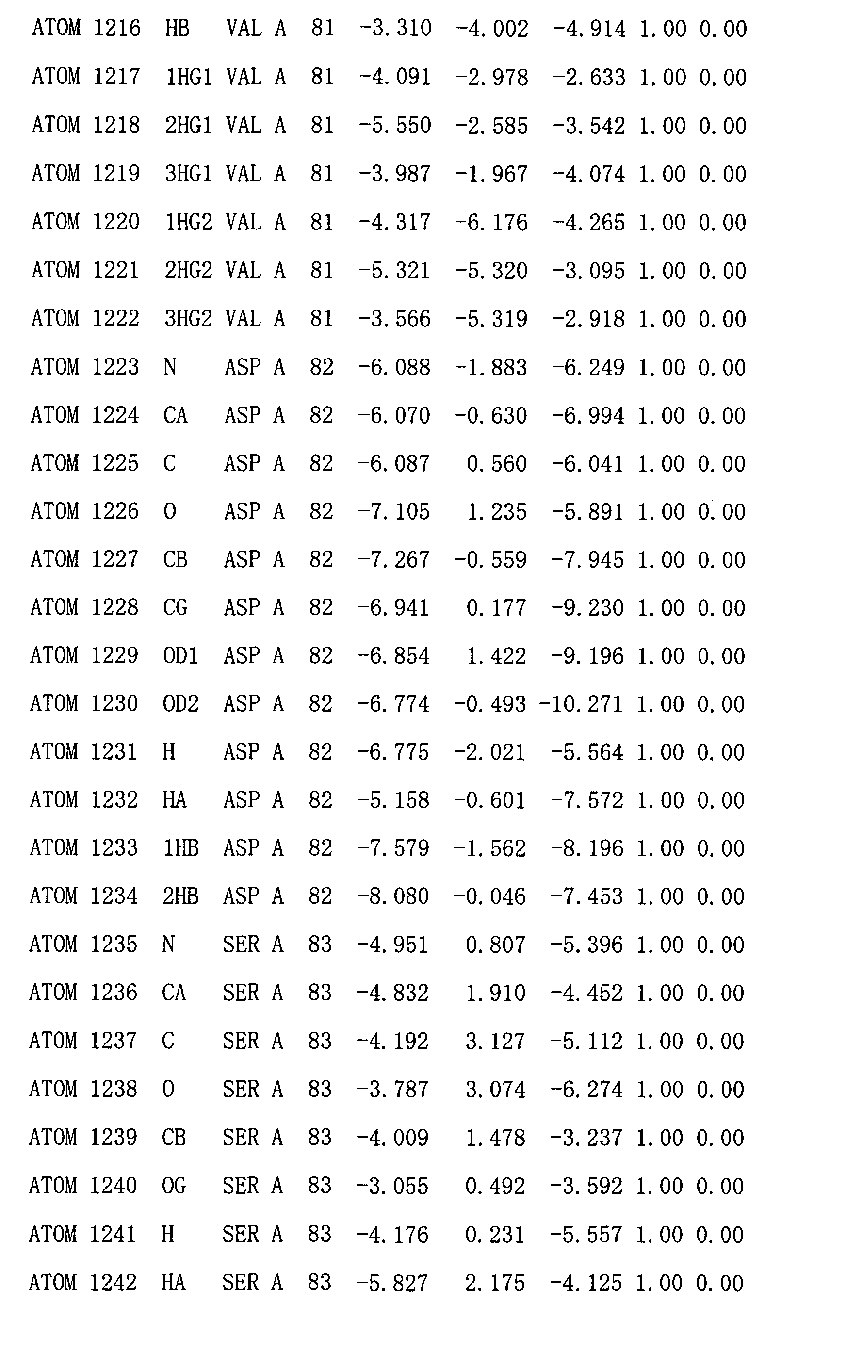

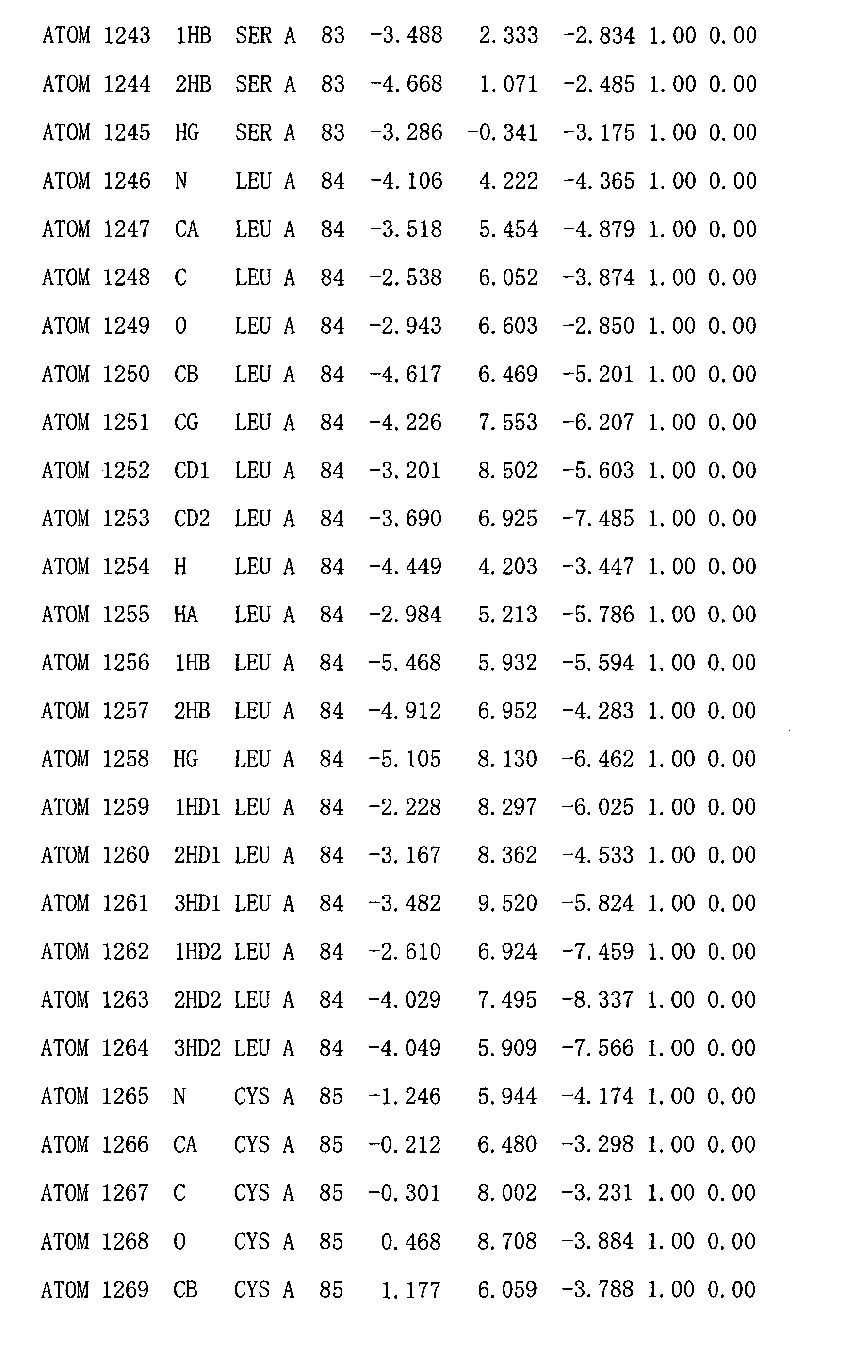

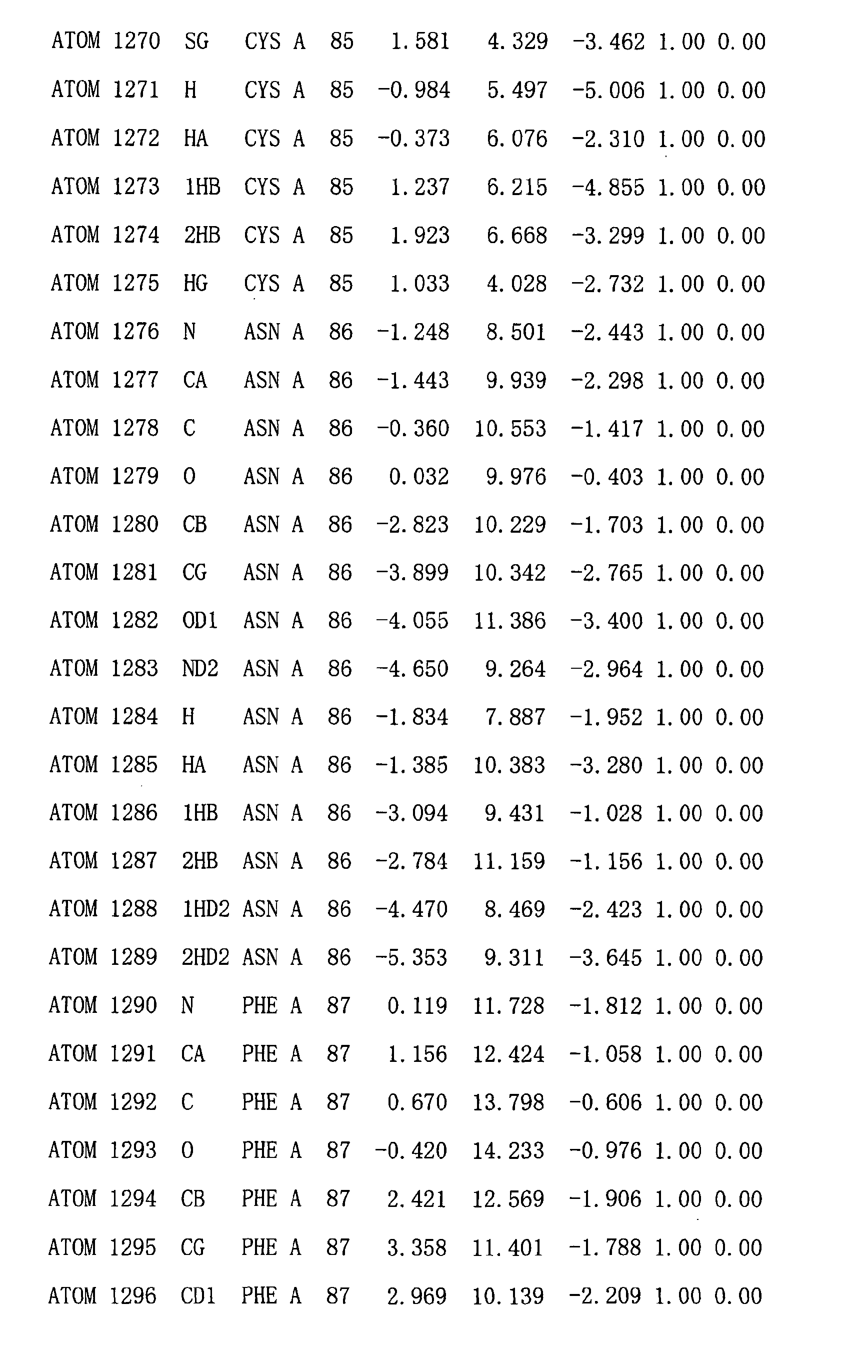

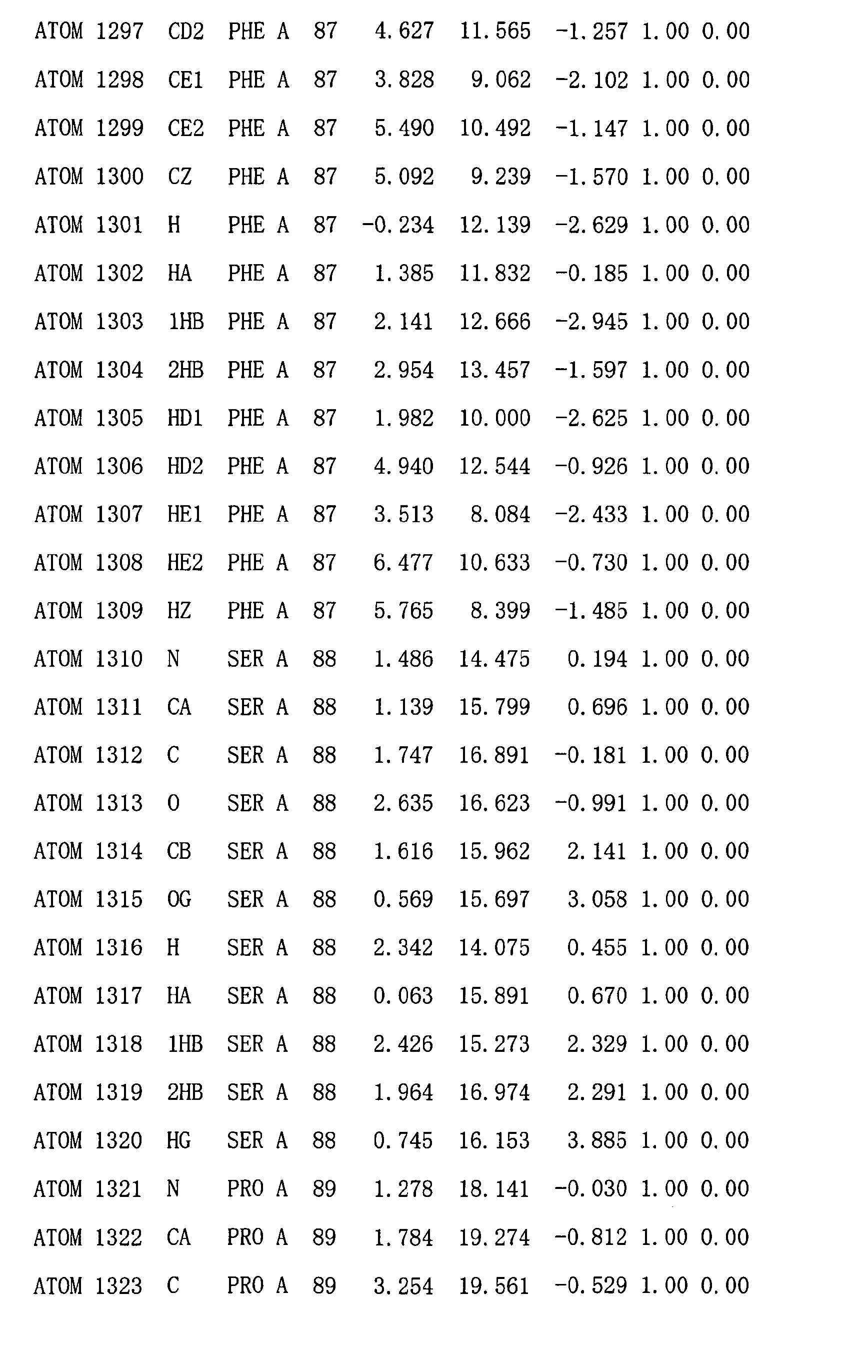

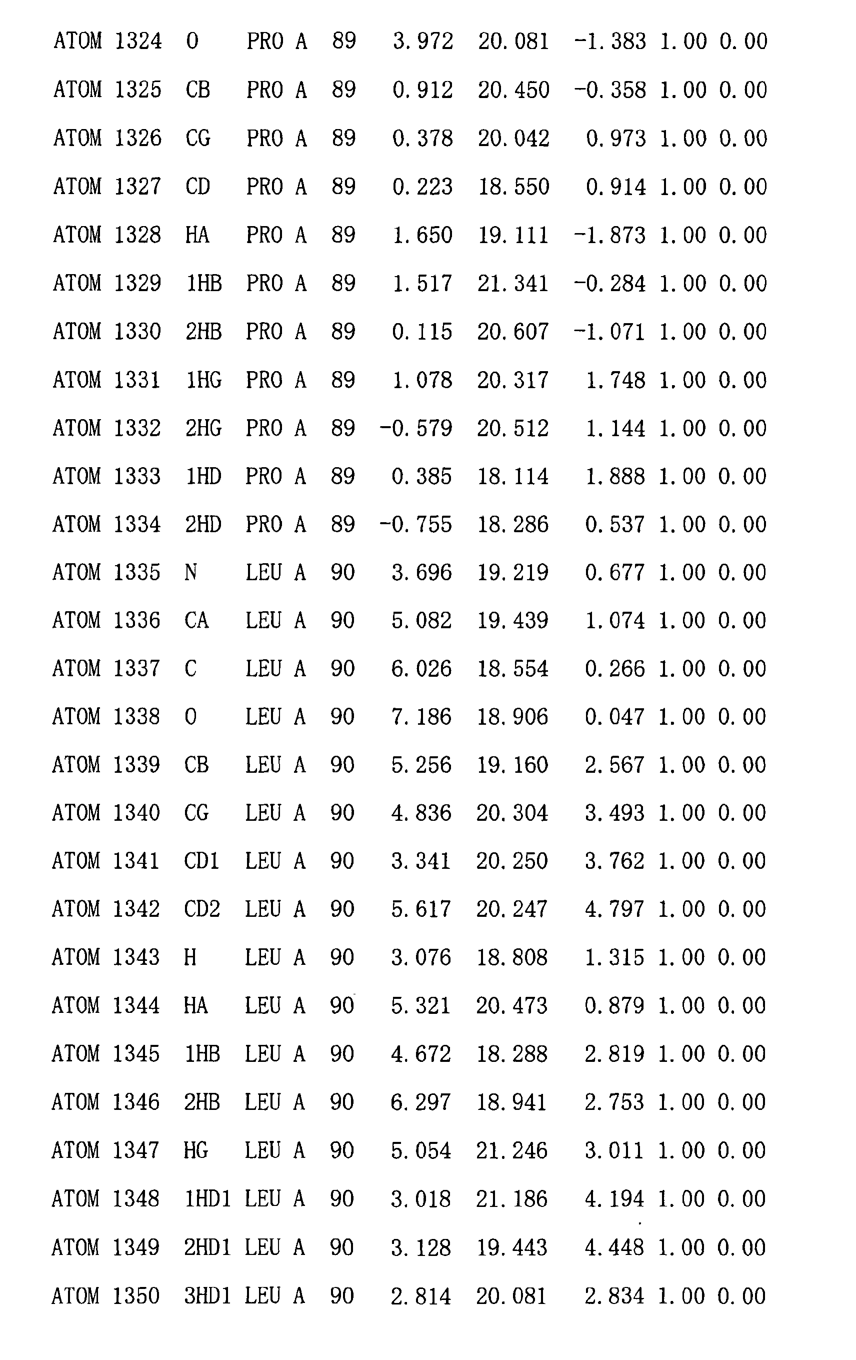

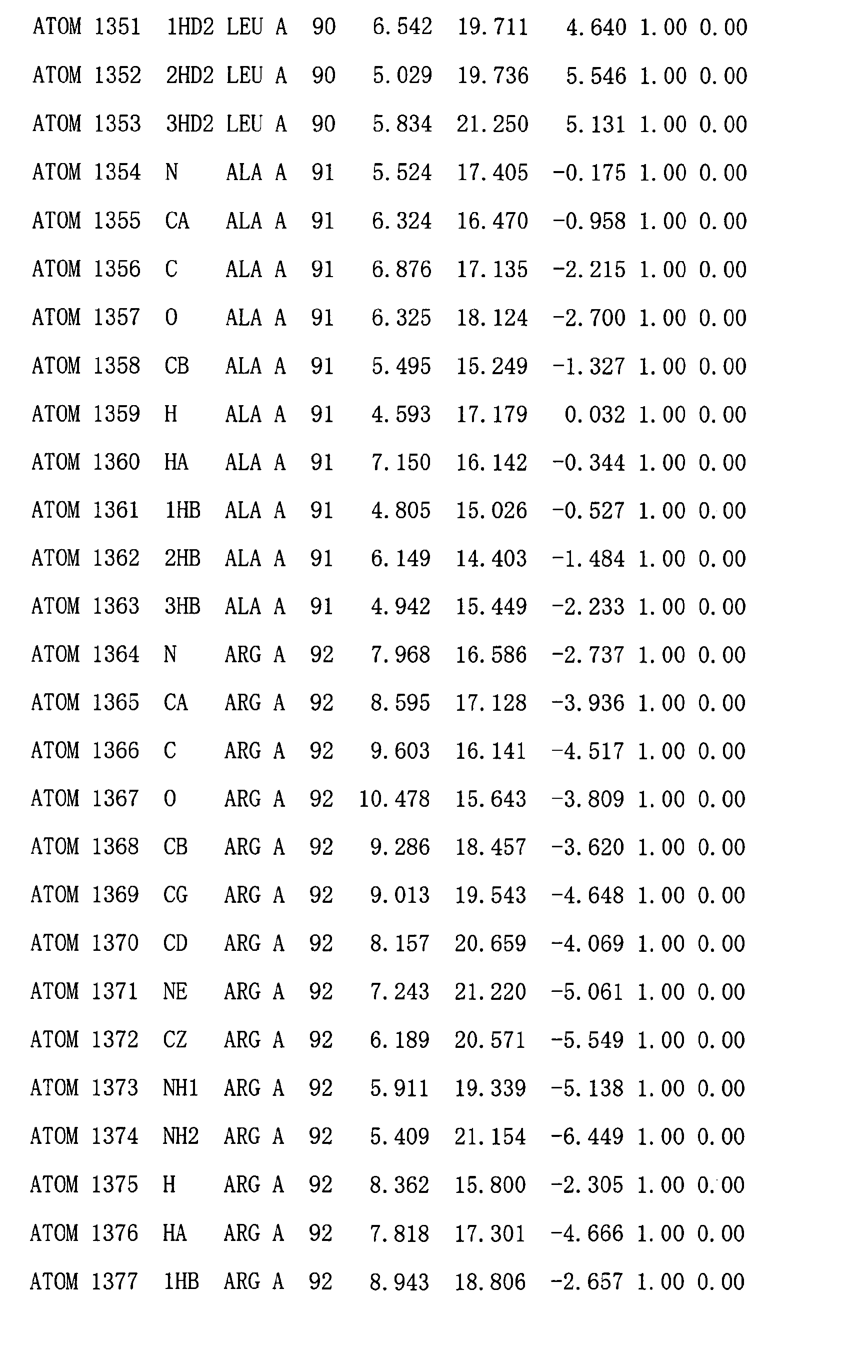

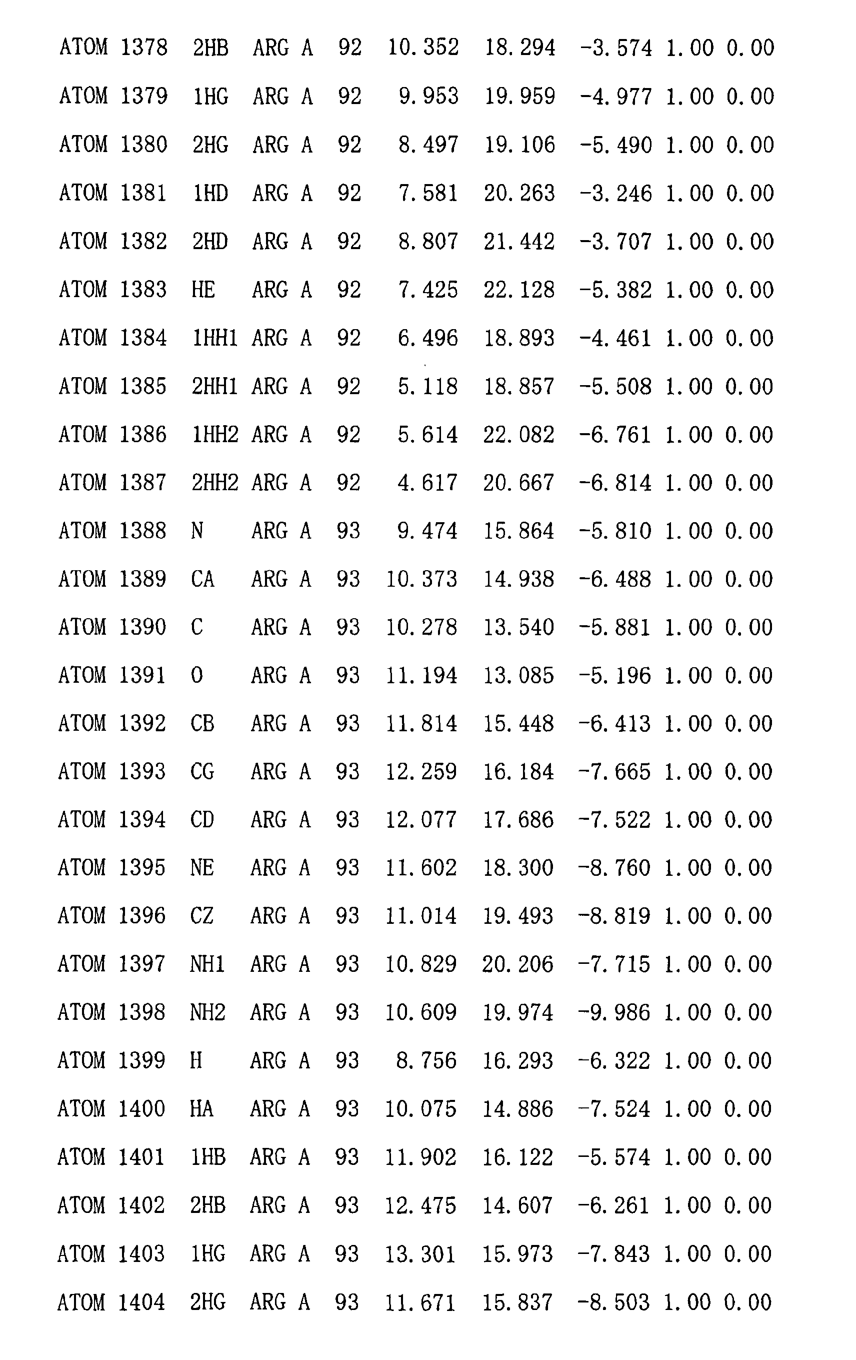

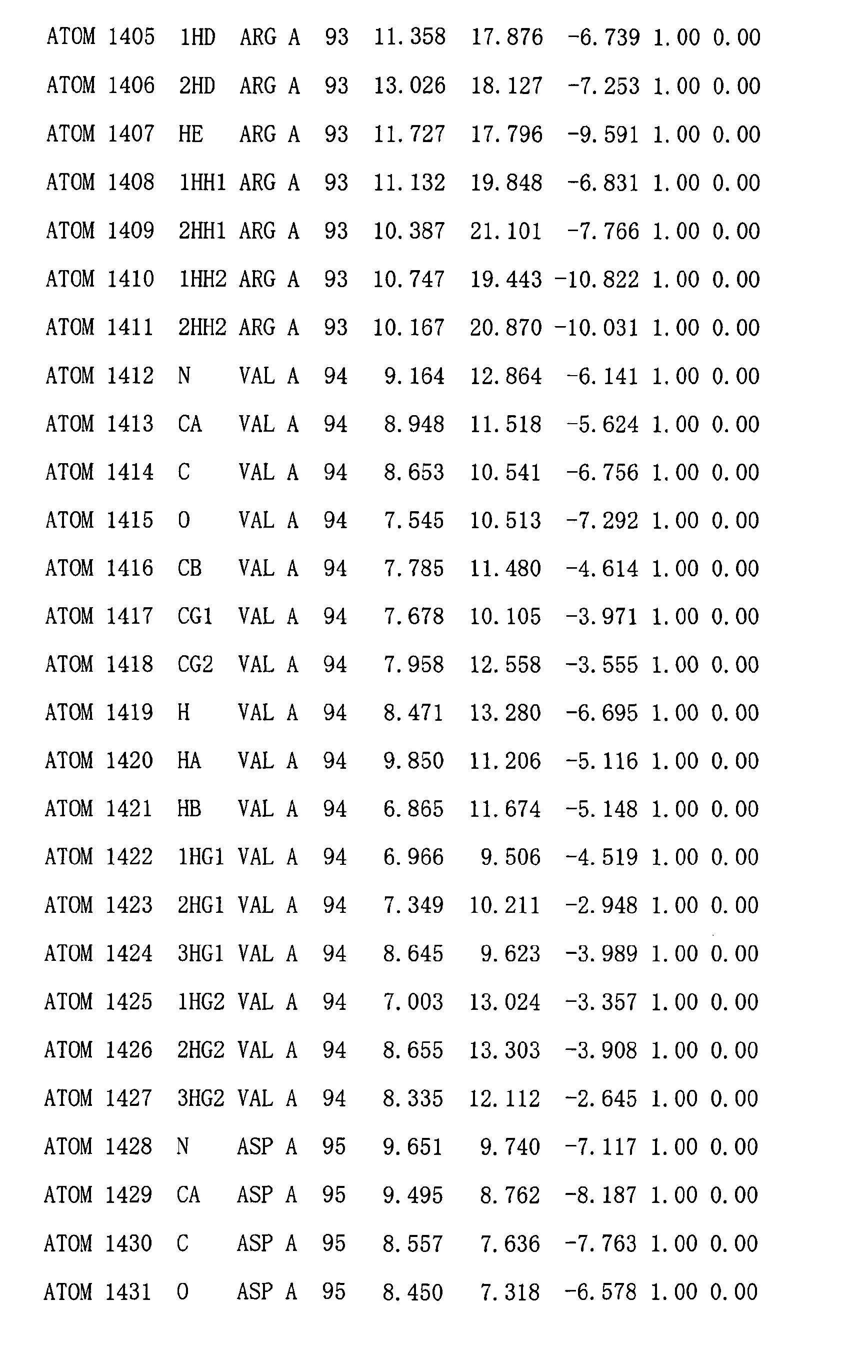

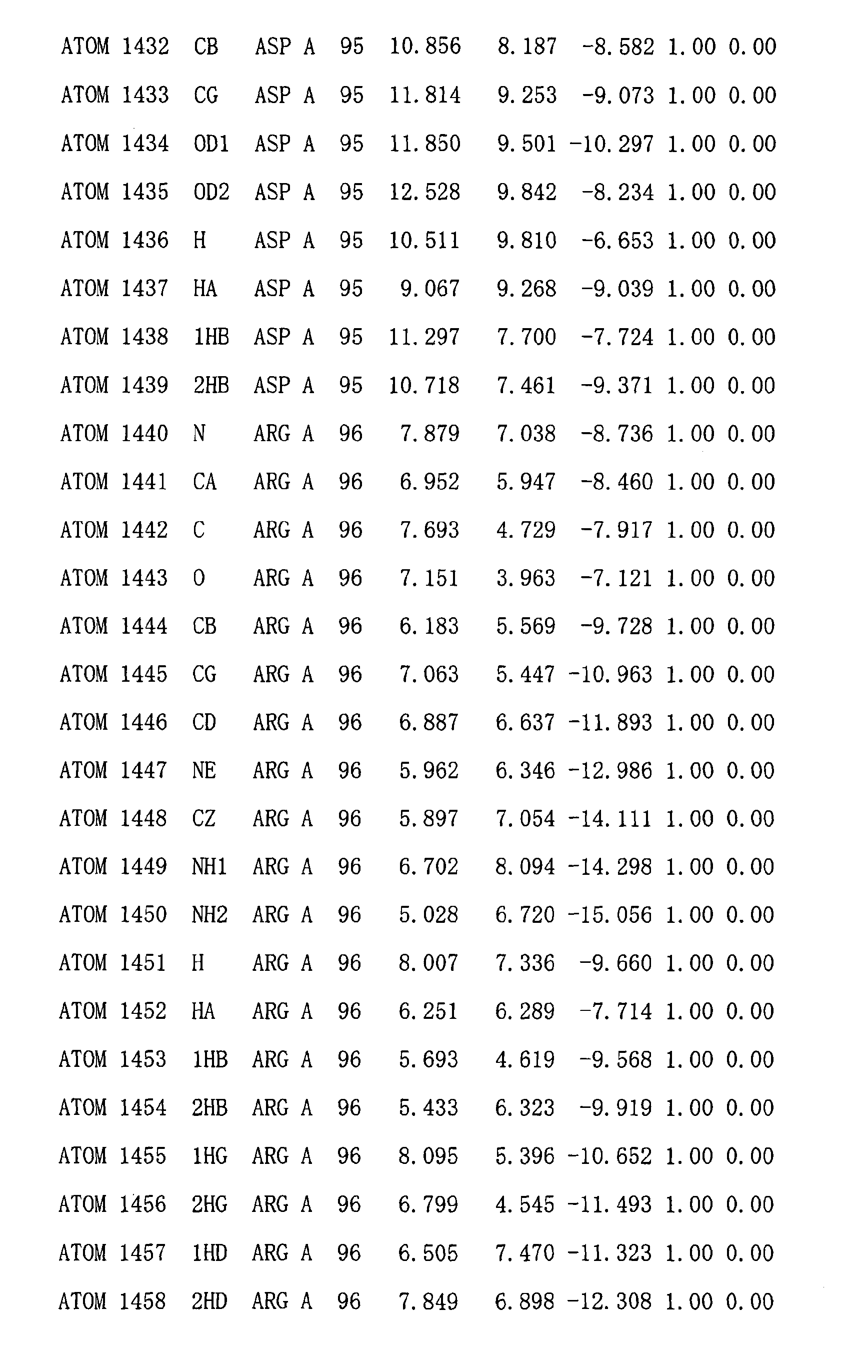

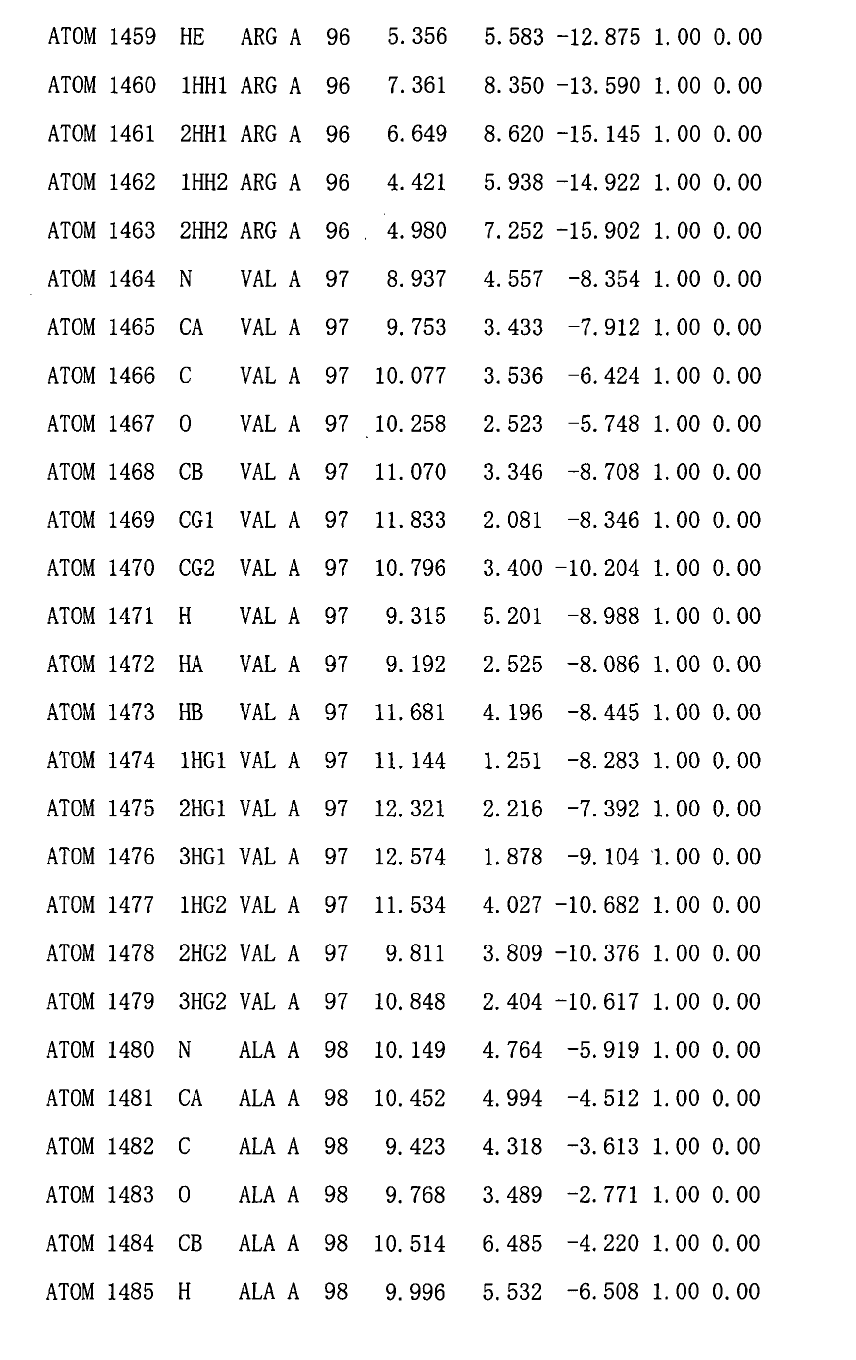

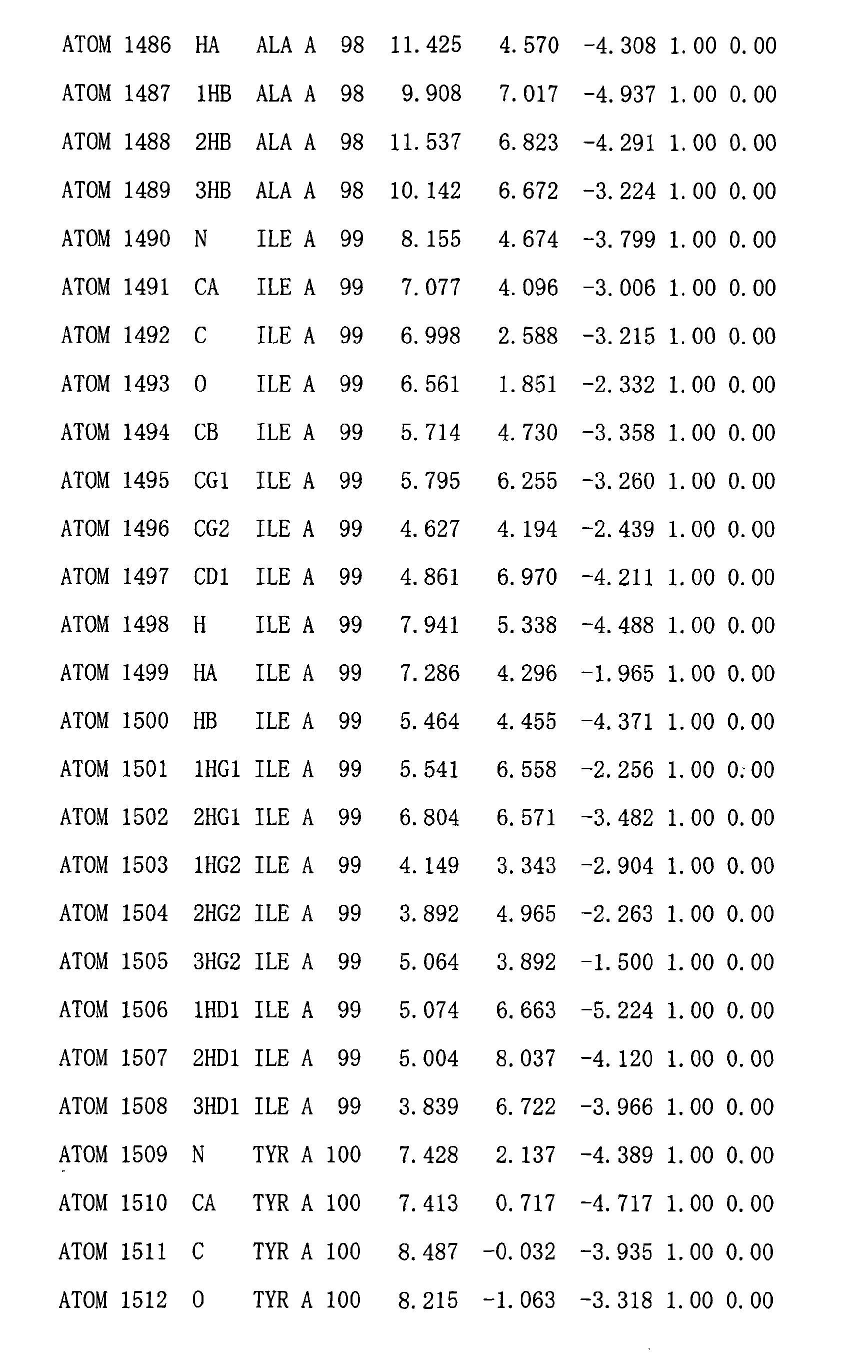

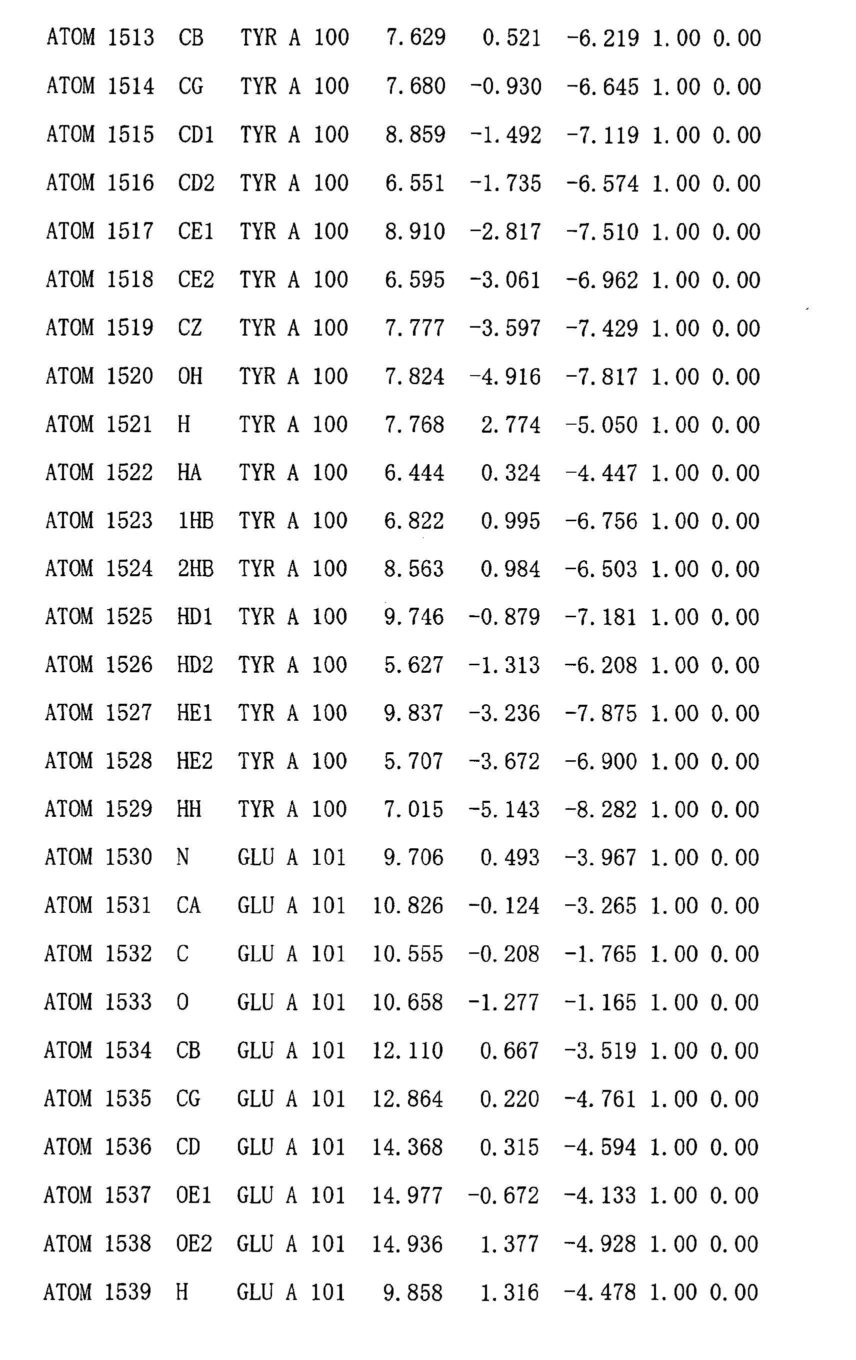

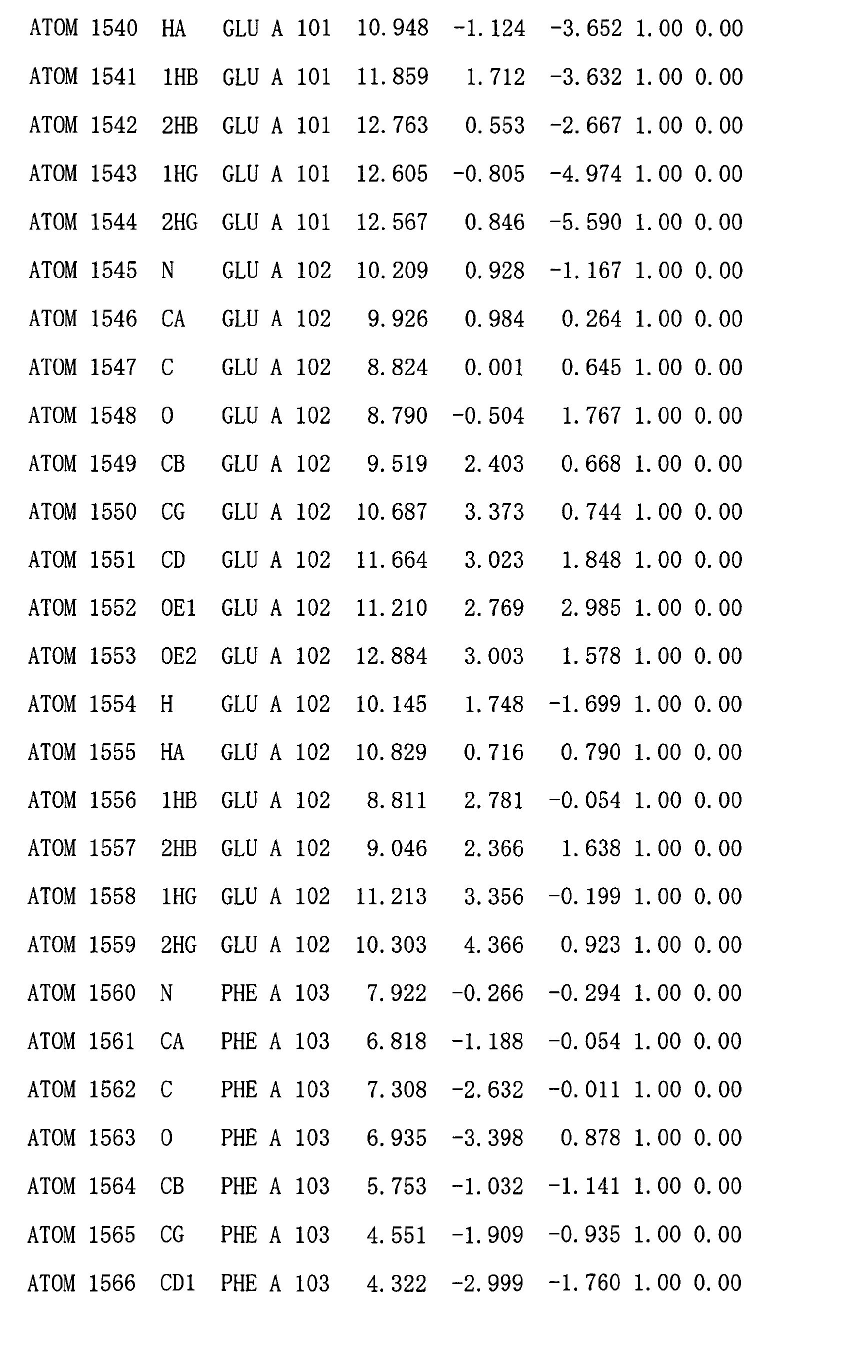

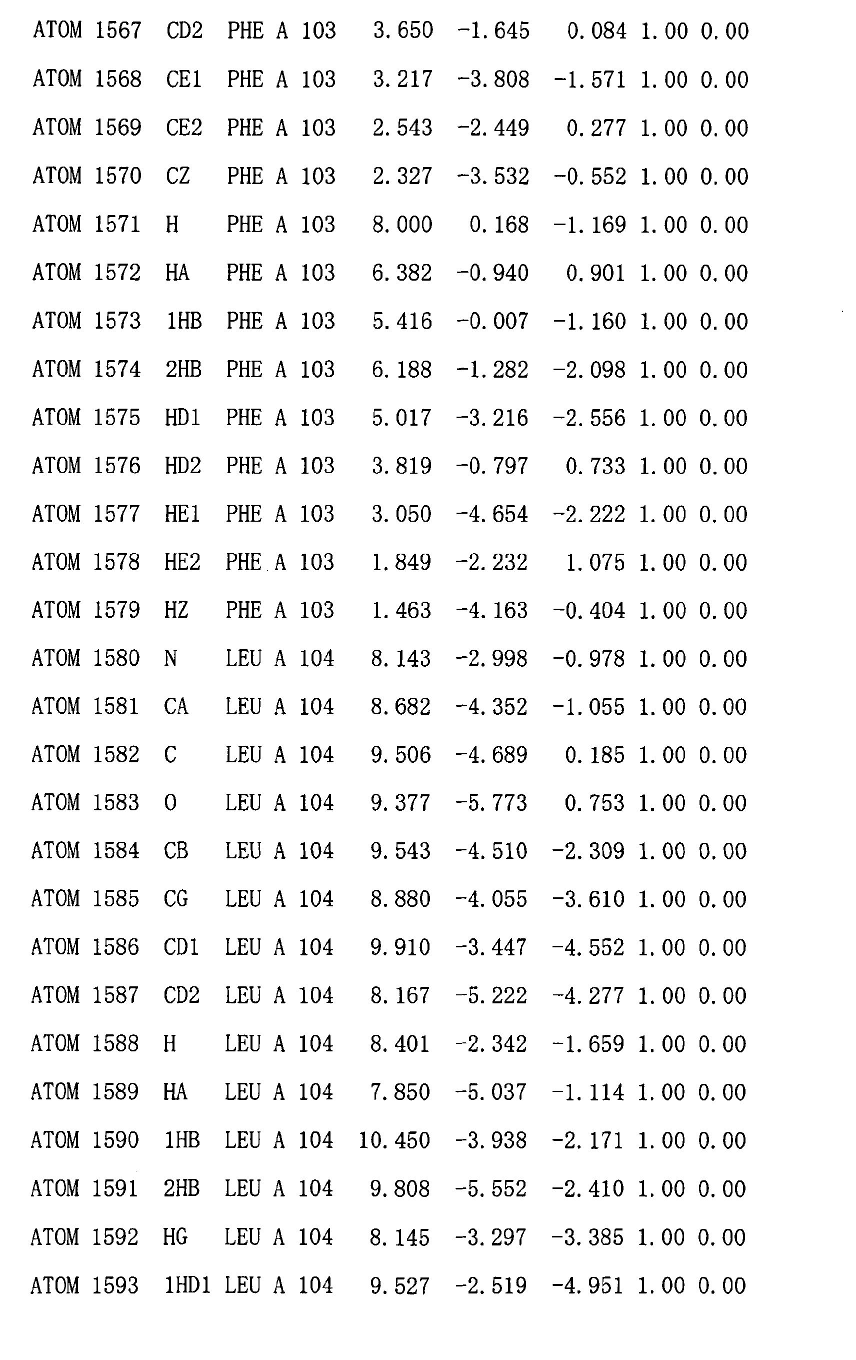

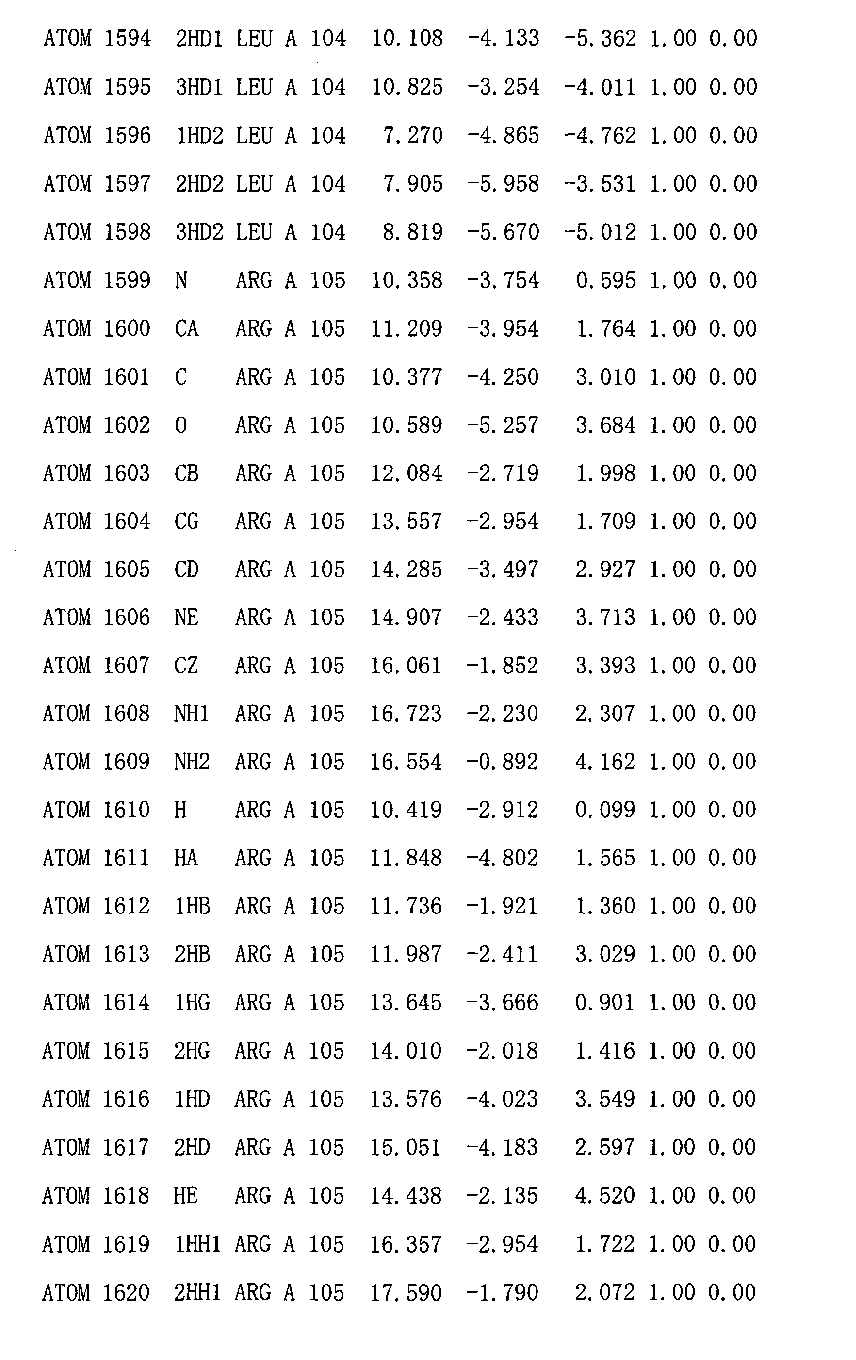

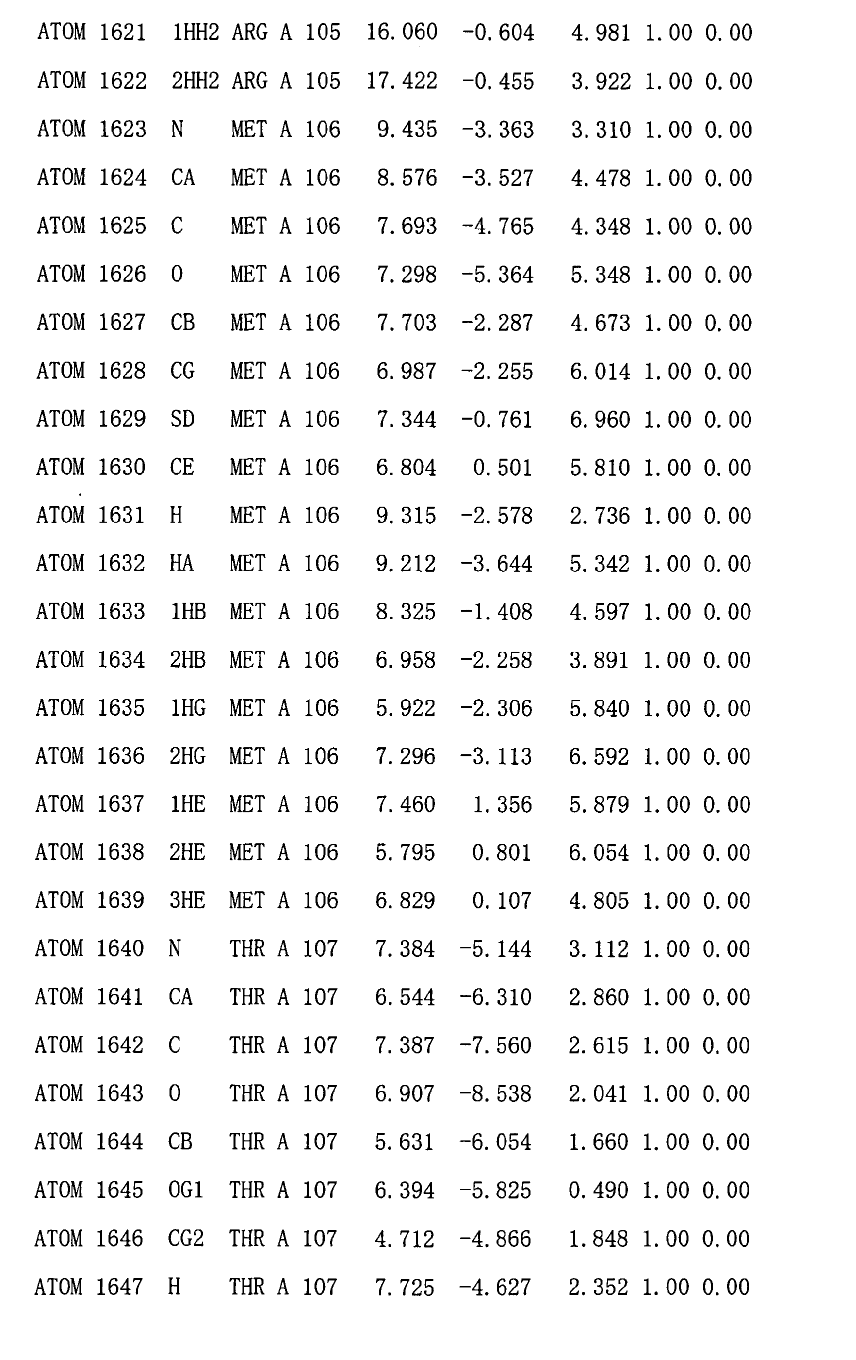

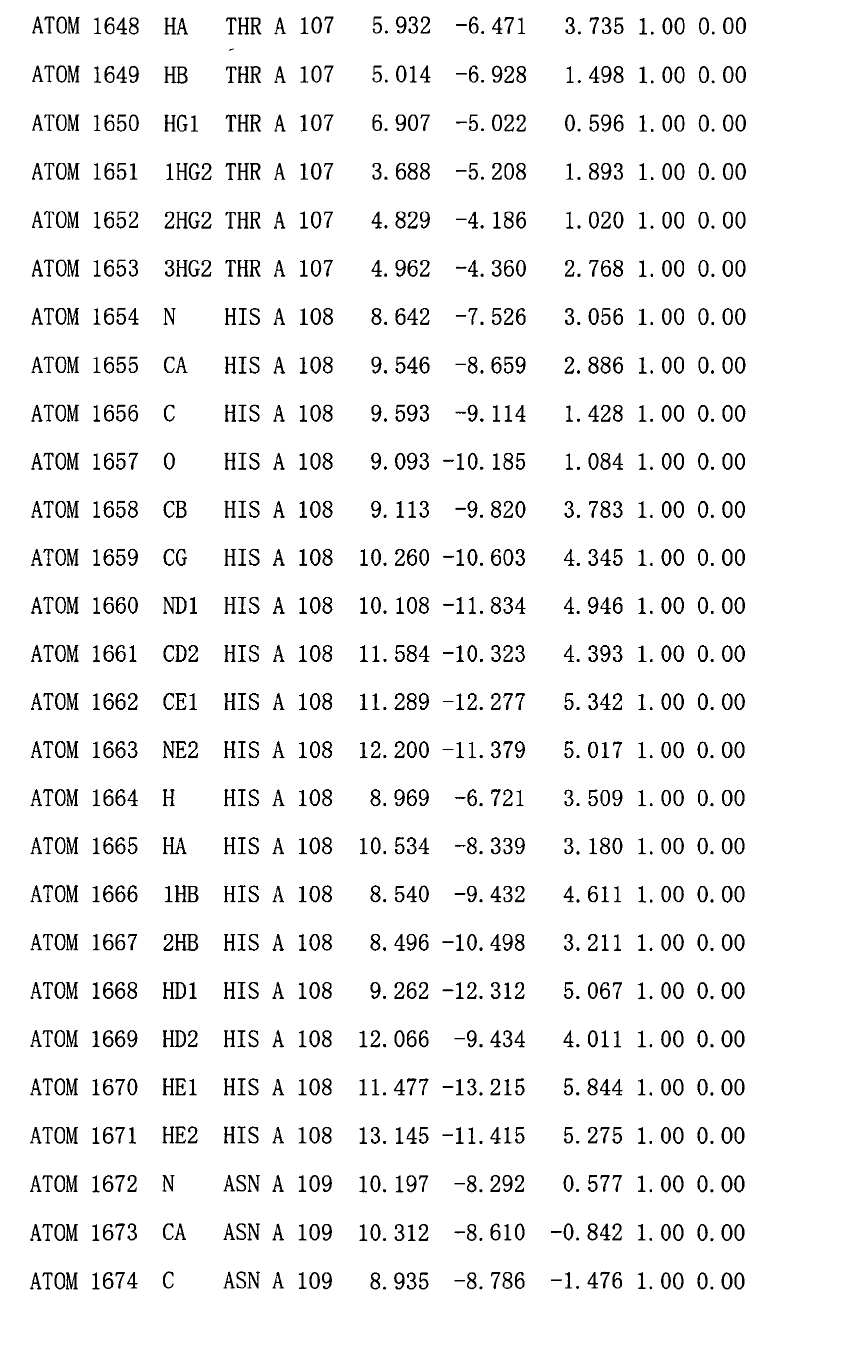

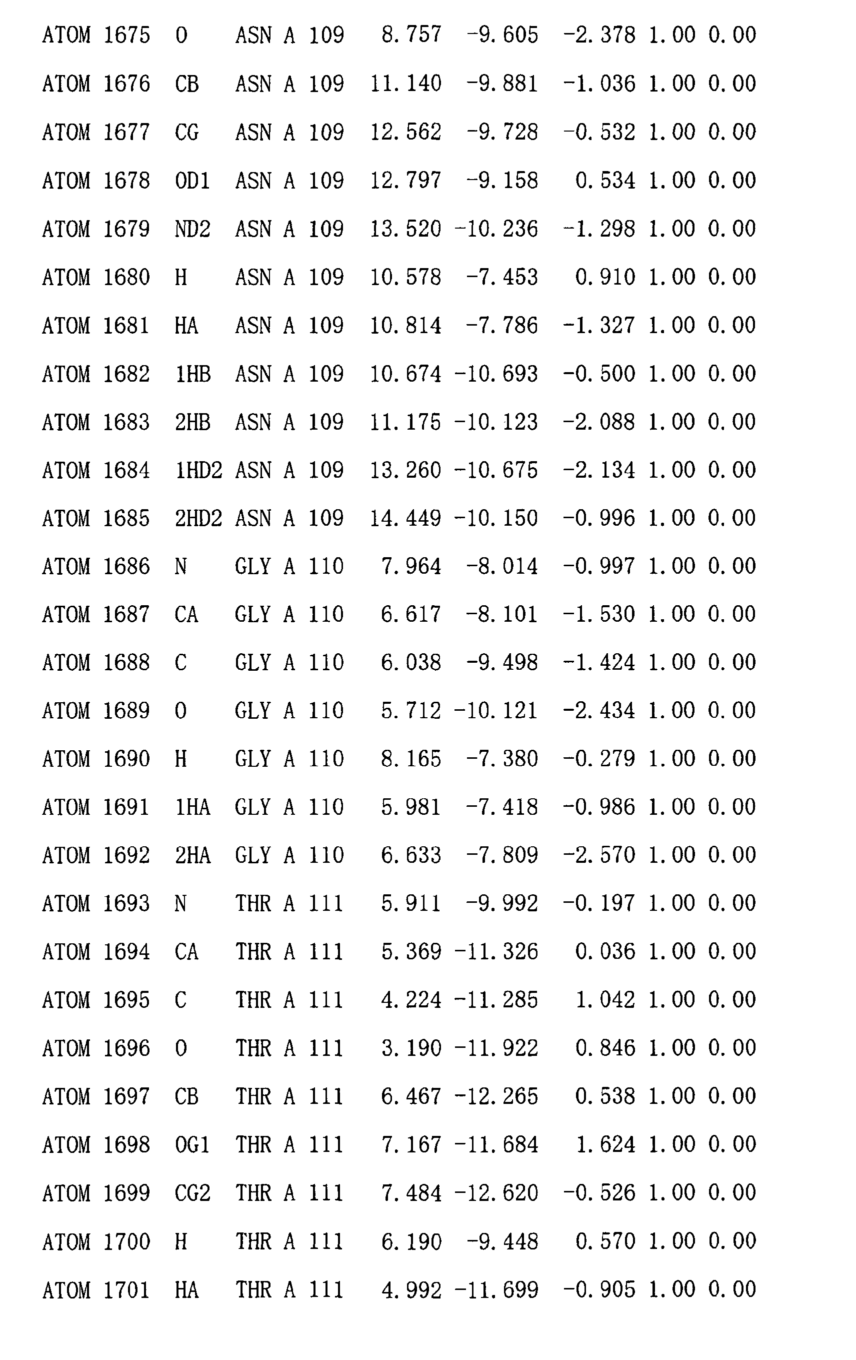

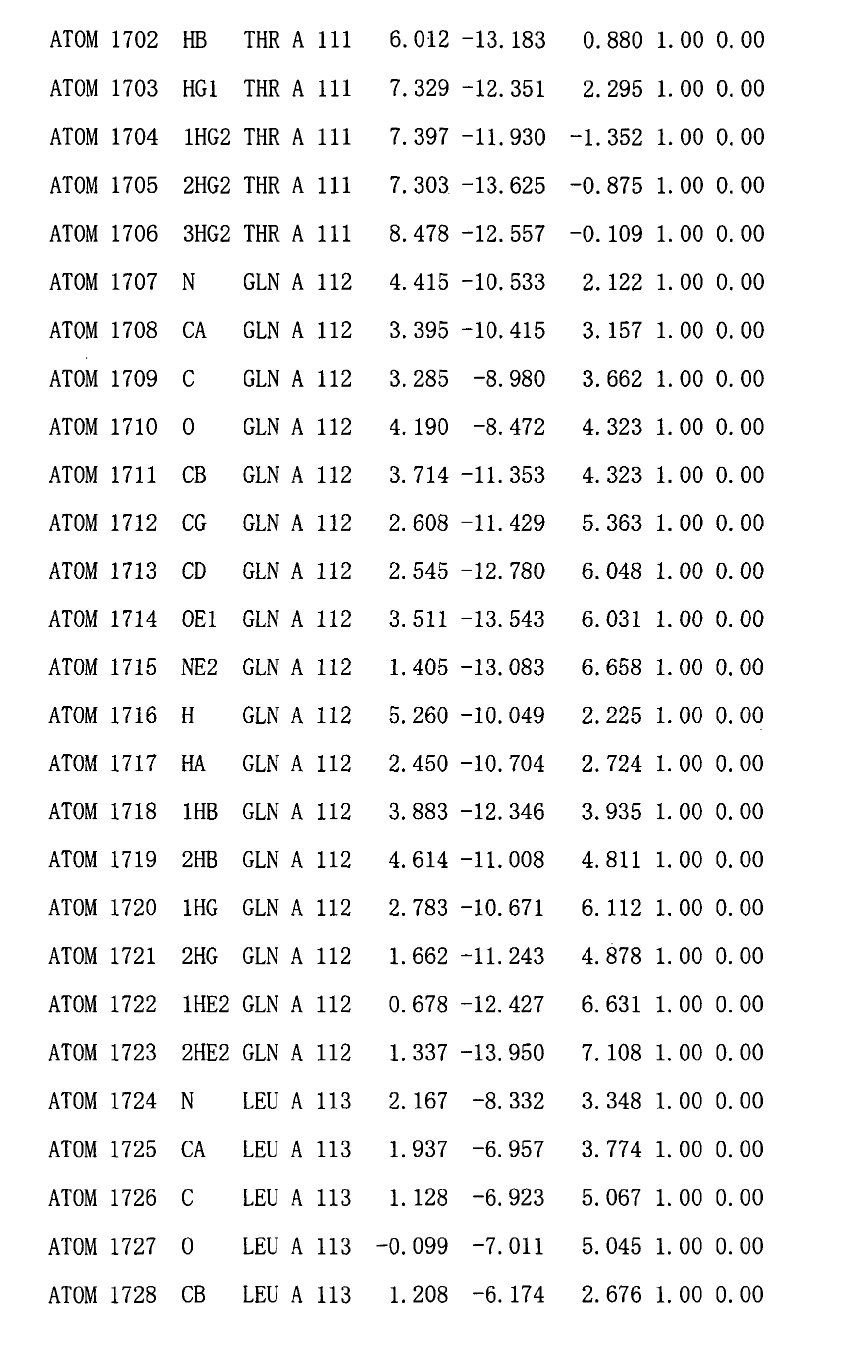

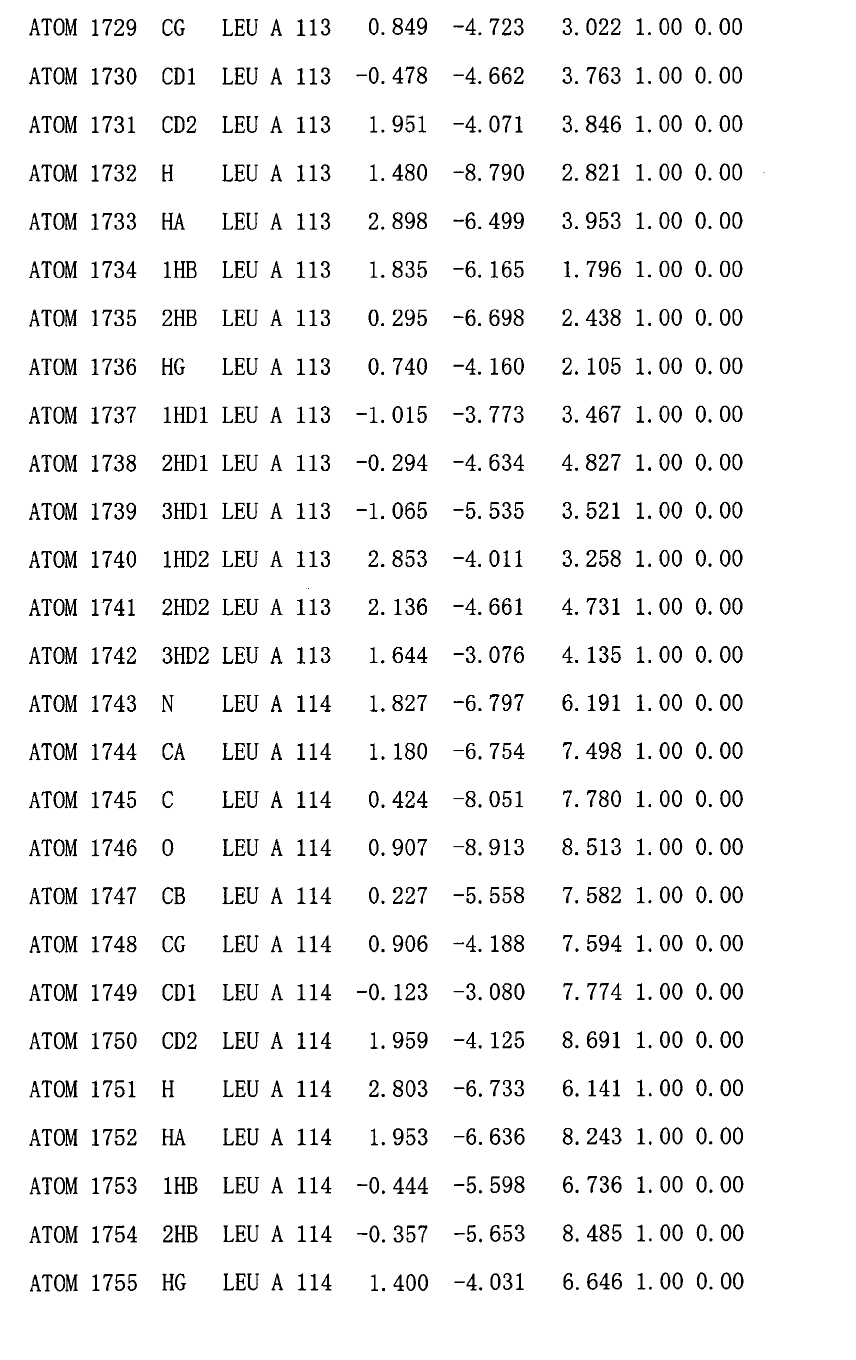

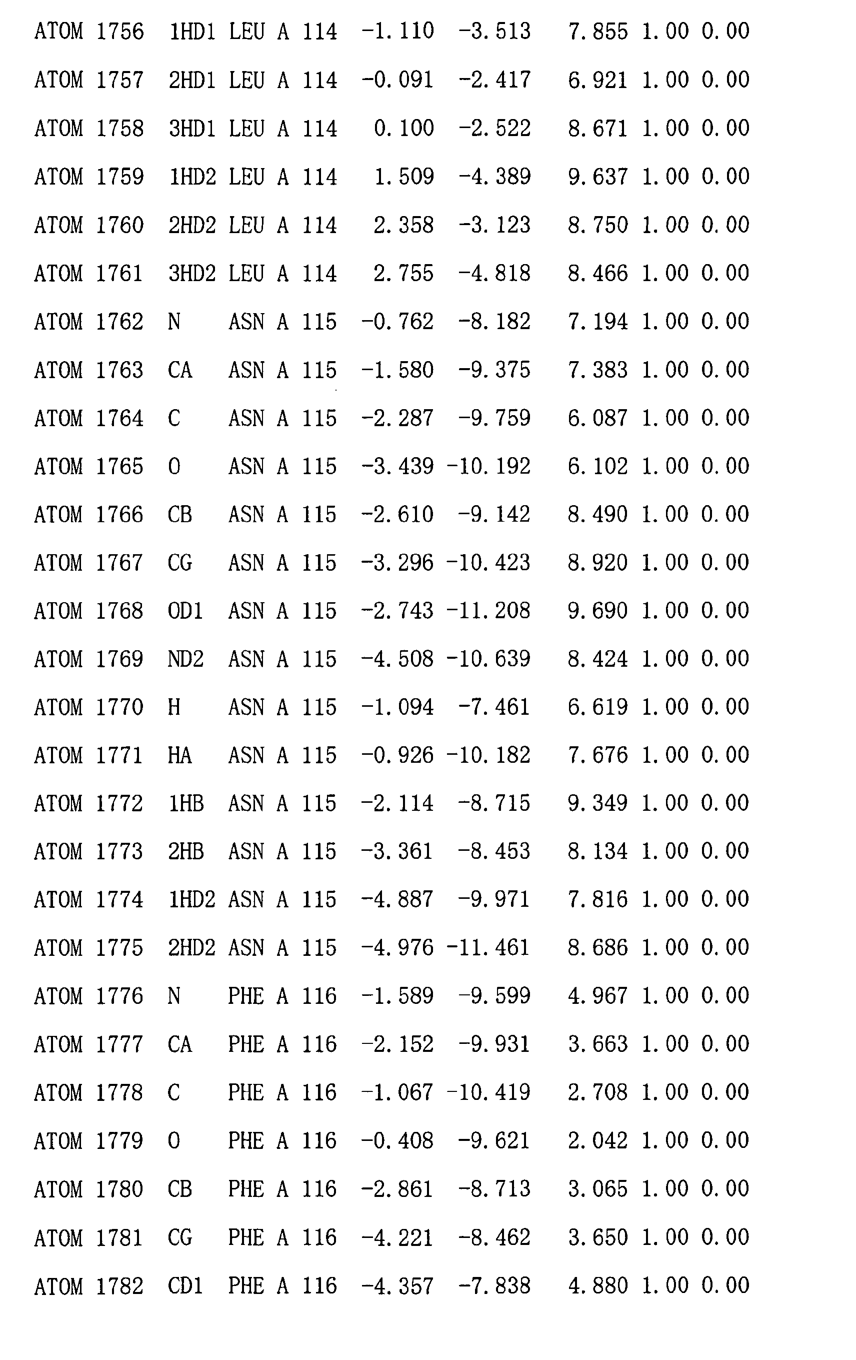

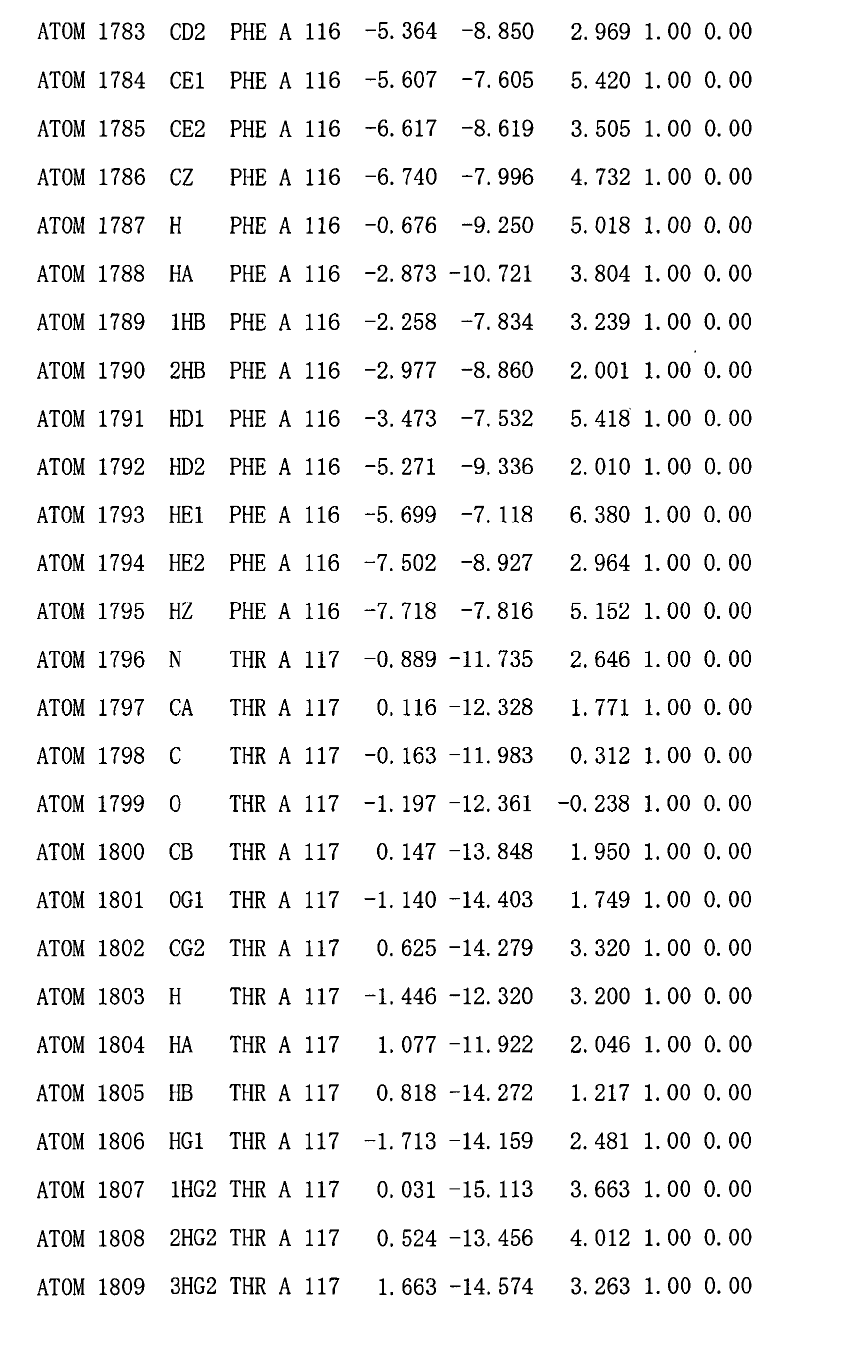

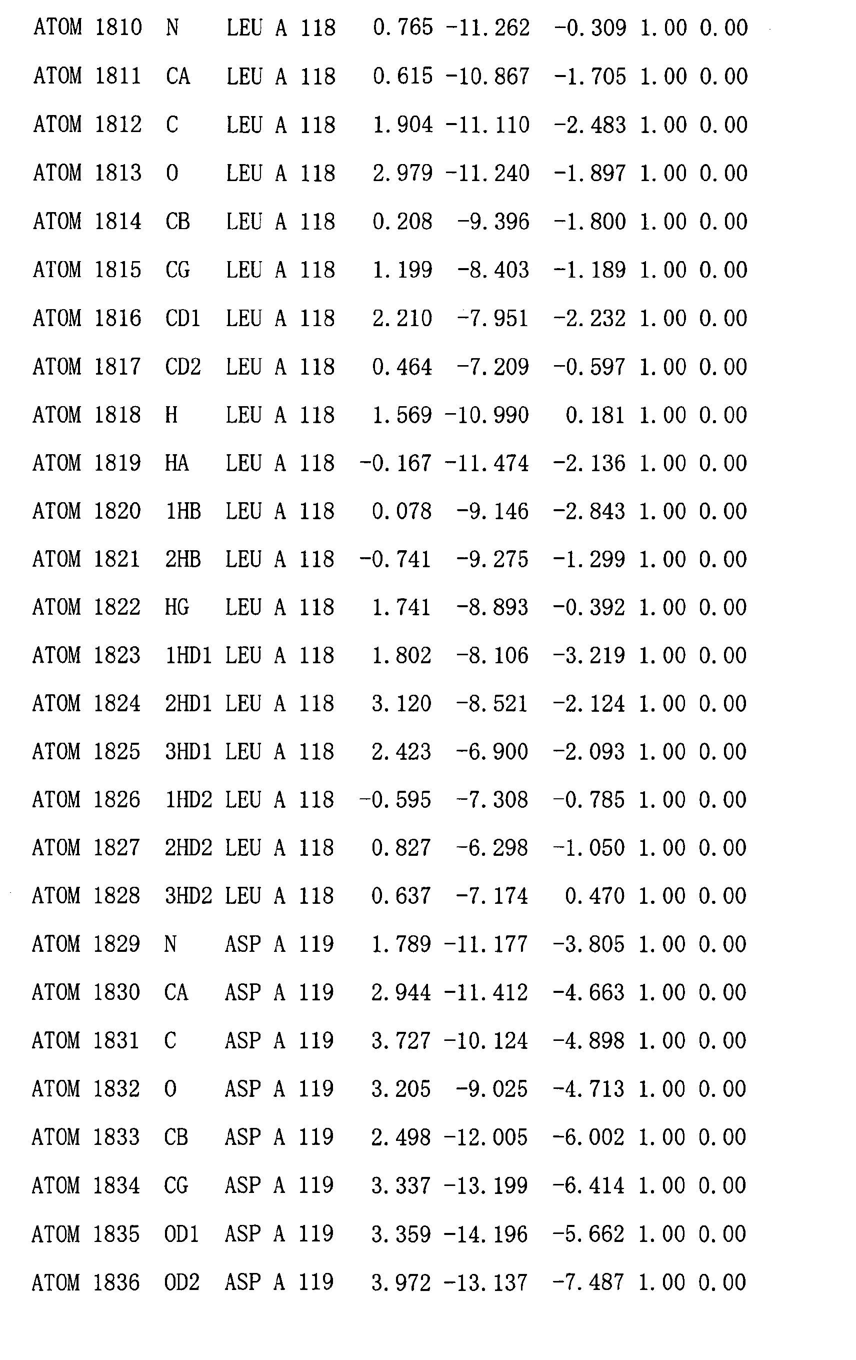

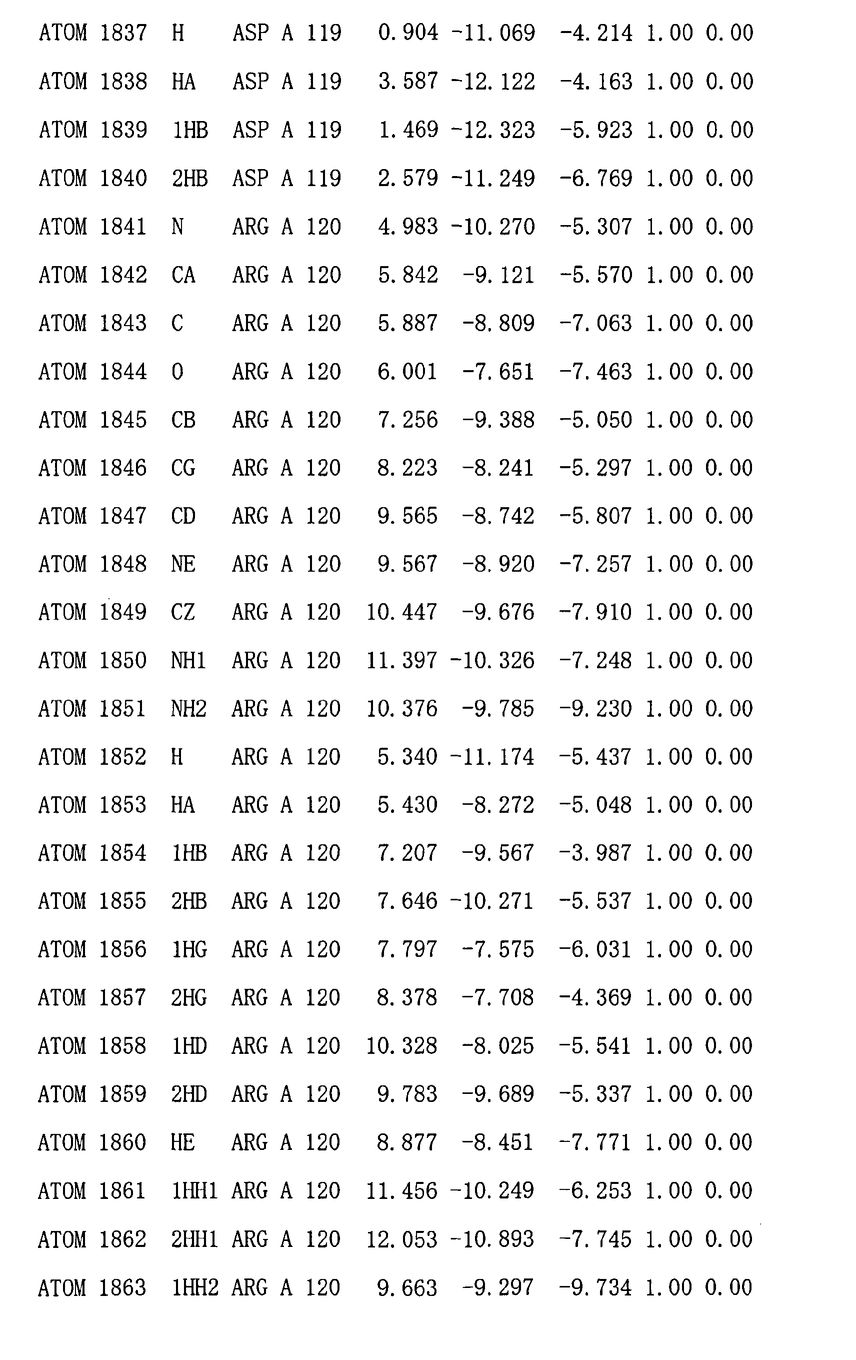

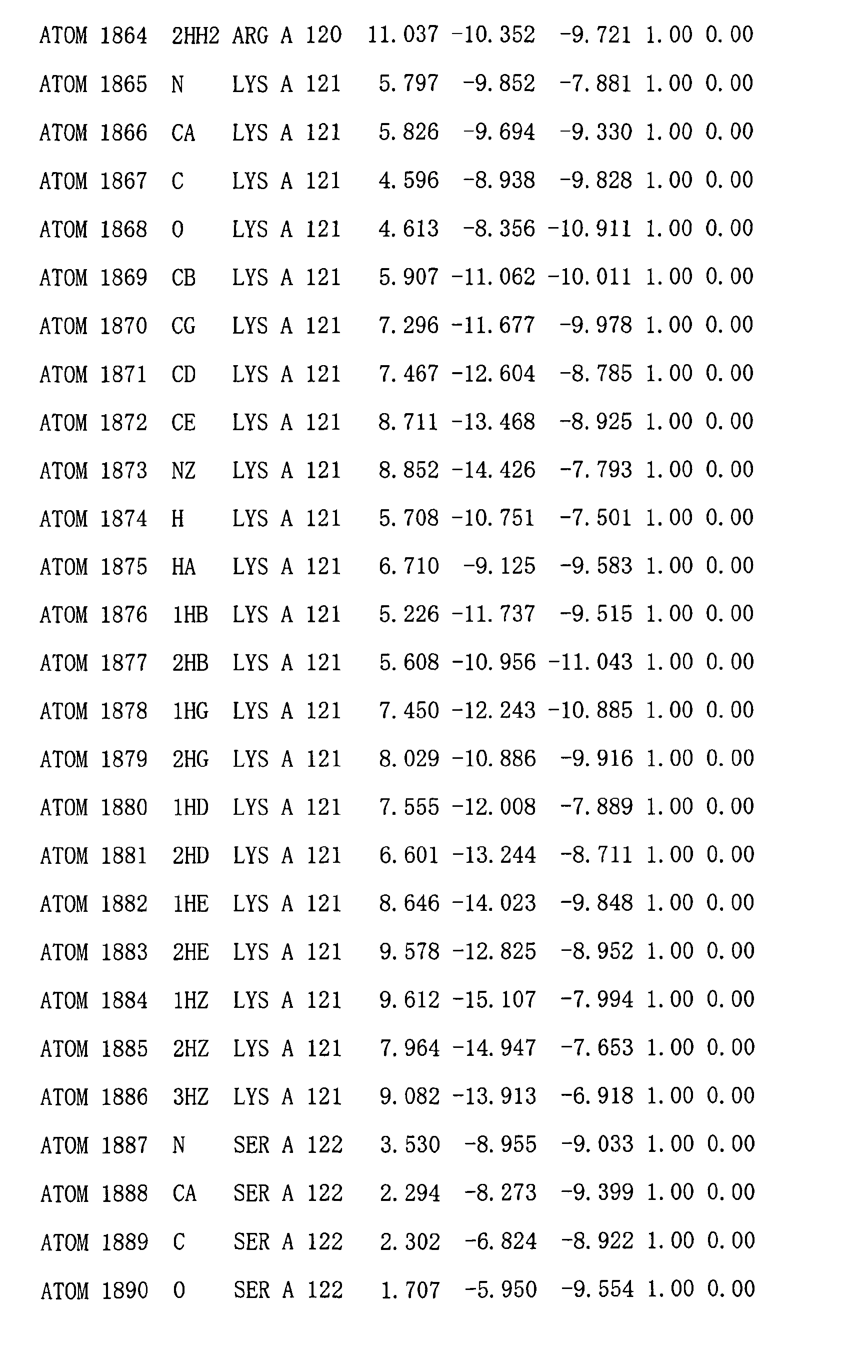

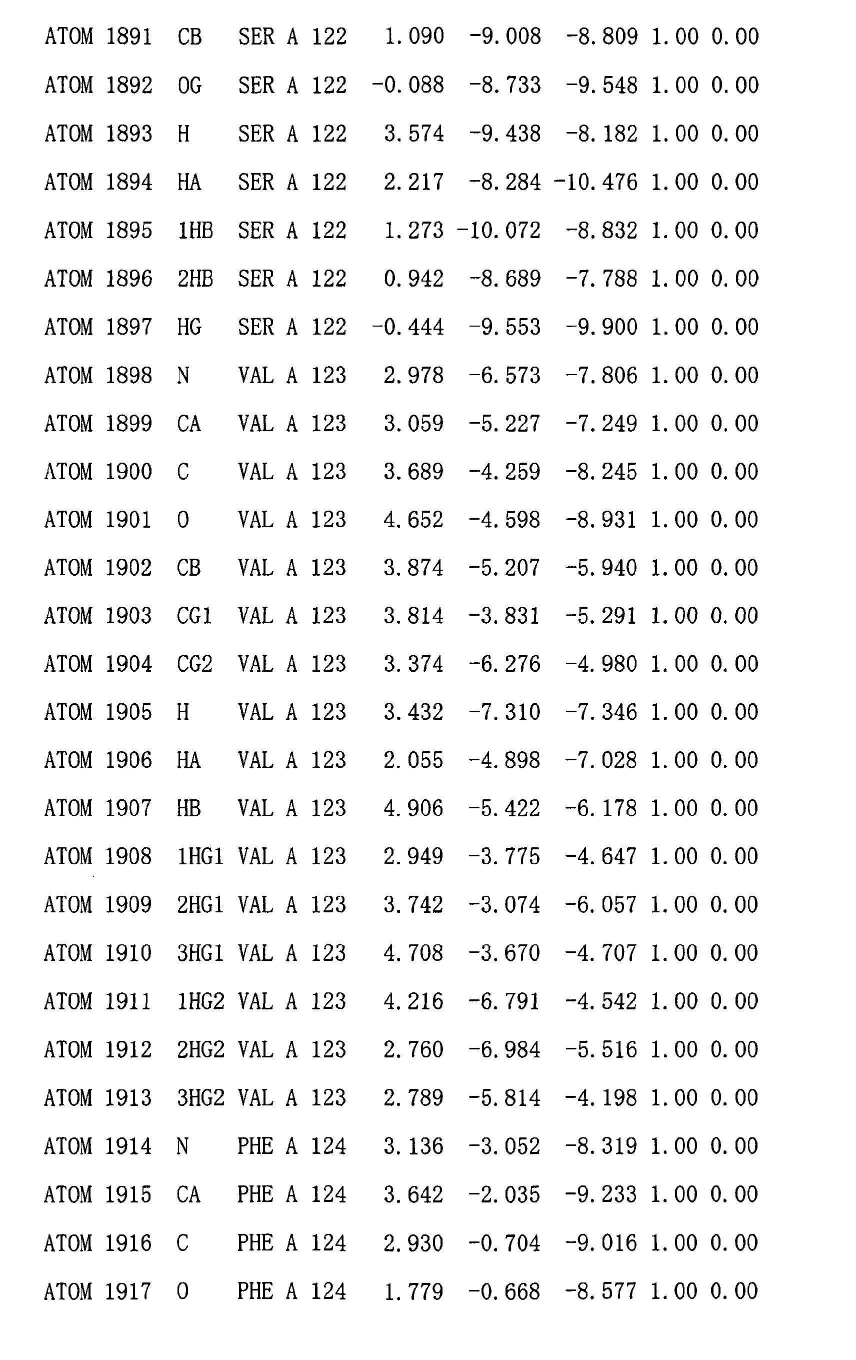

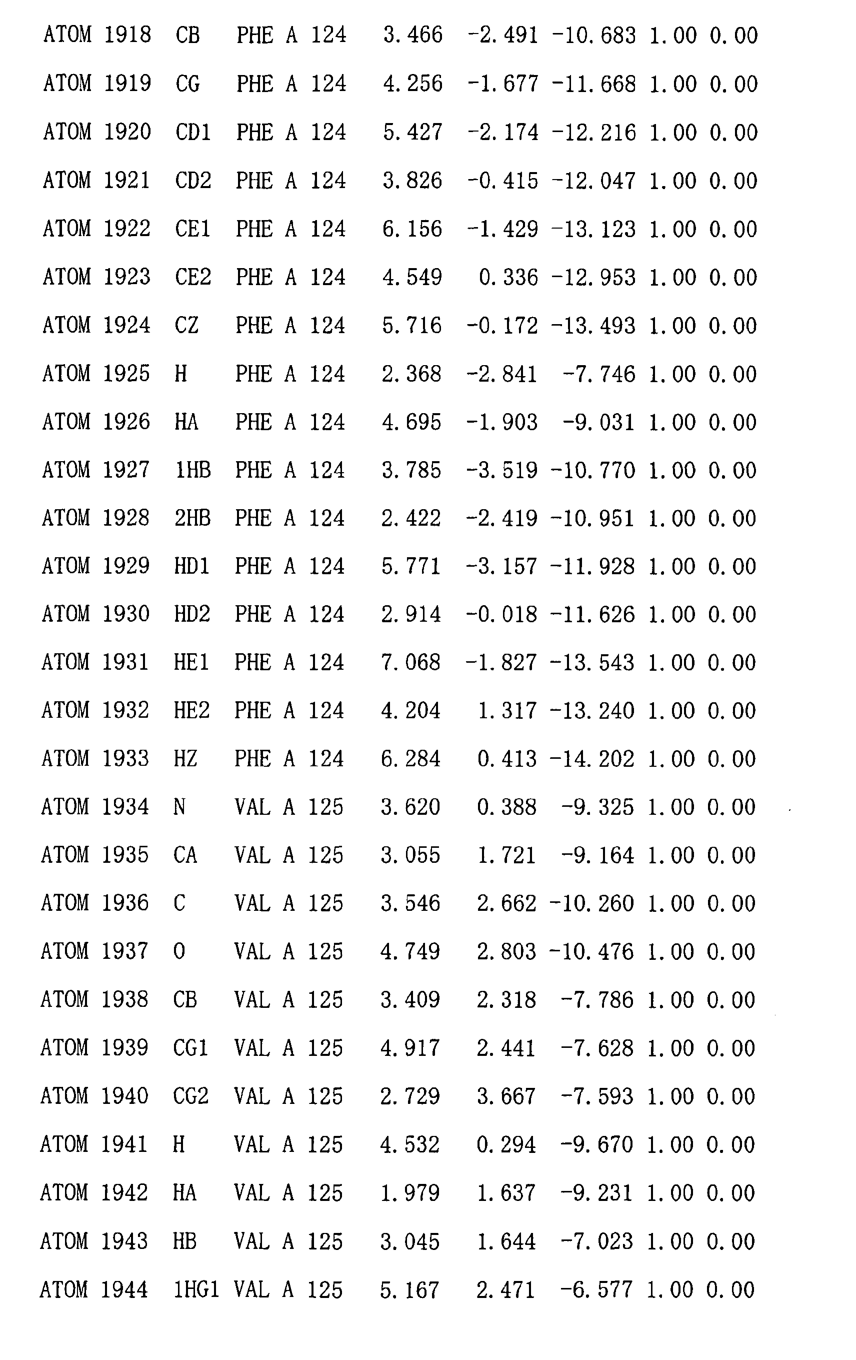

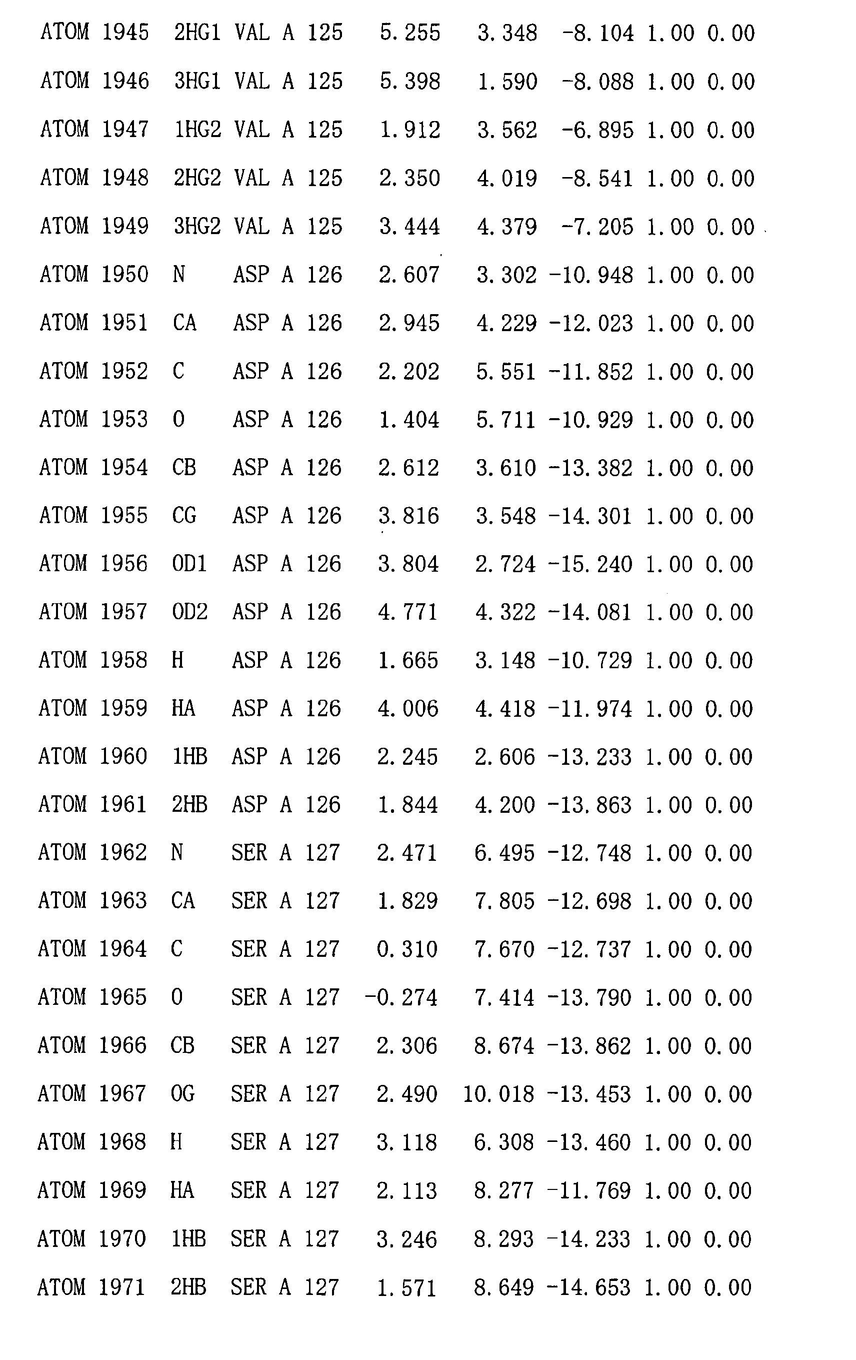

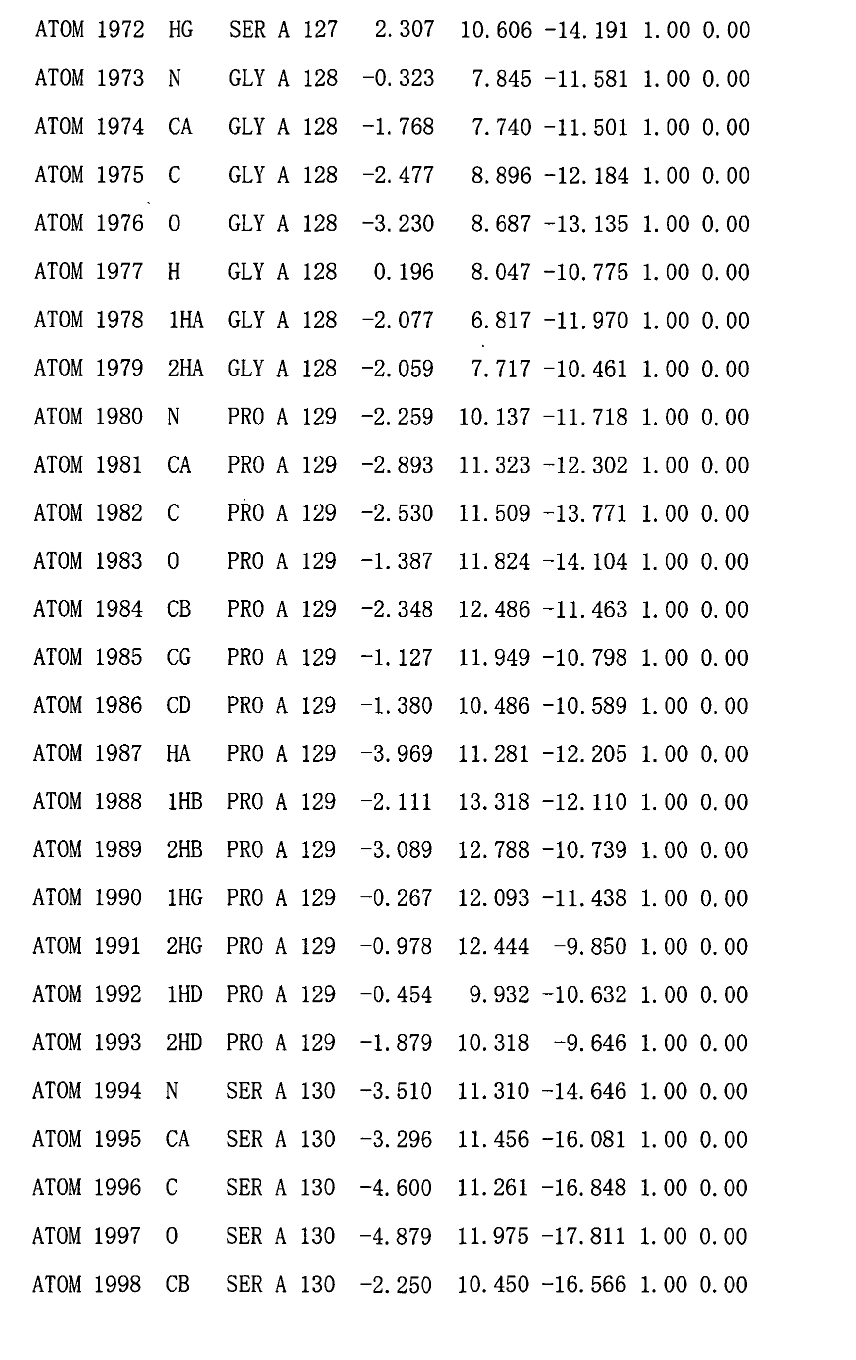

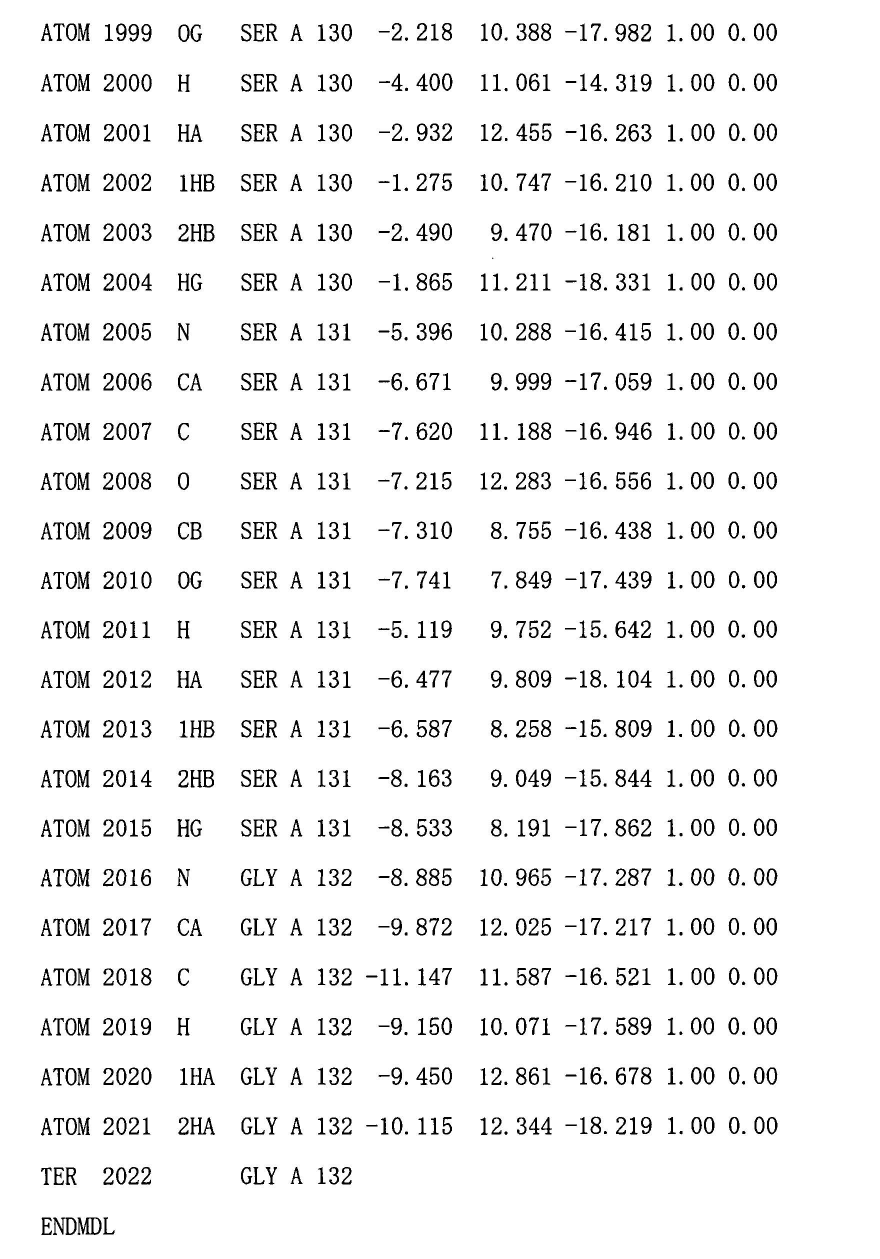

### Three-Dimensional Structure Coordinate 3

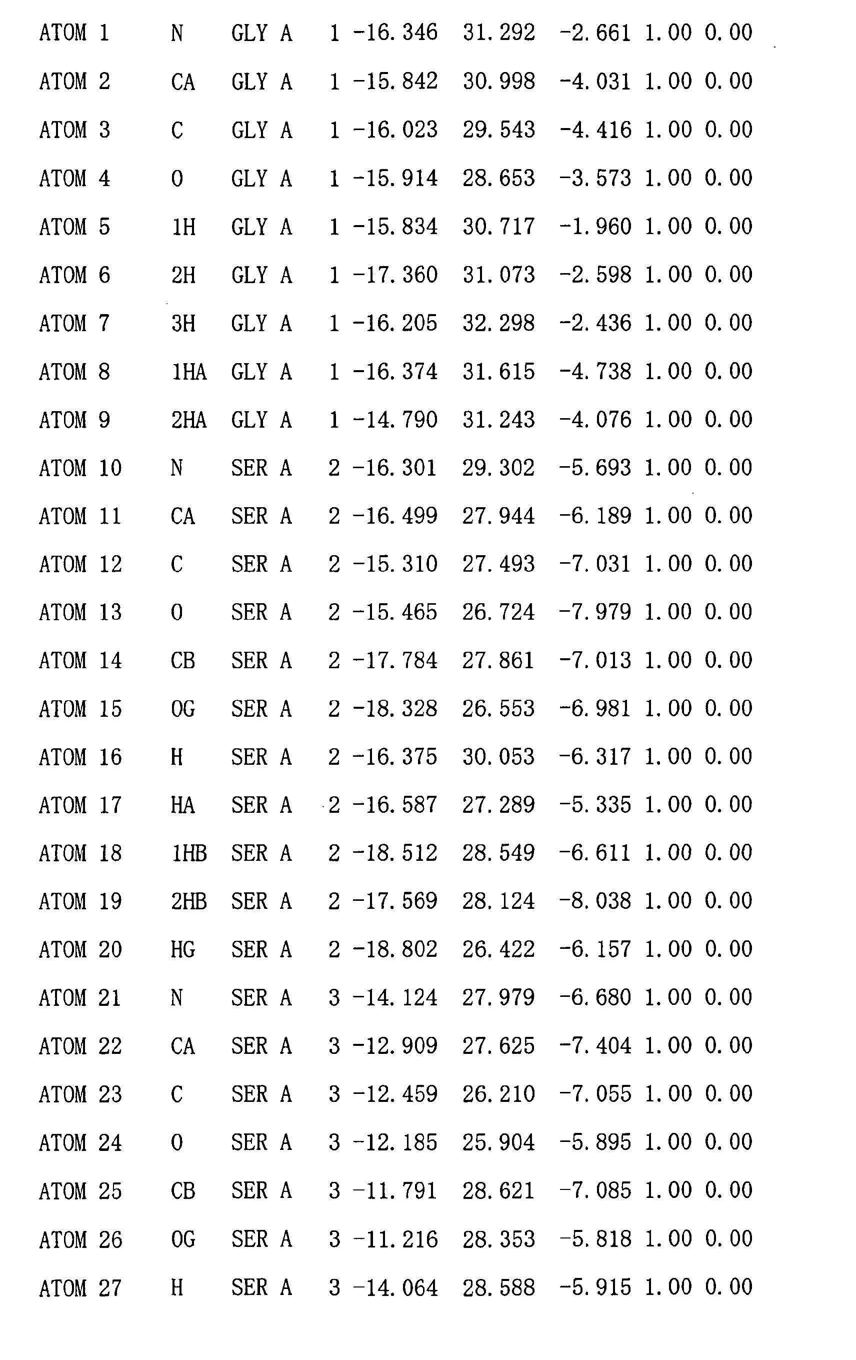

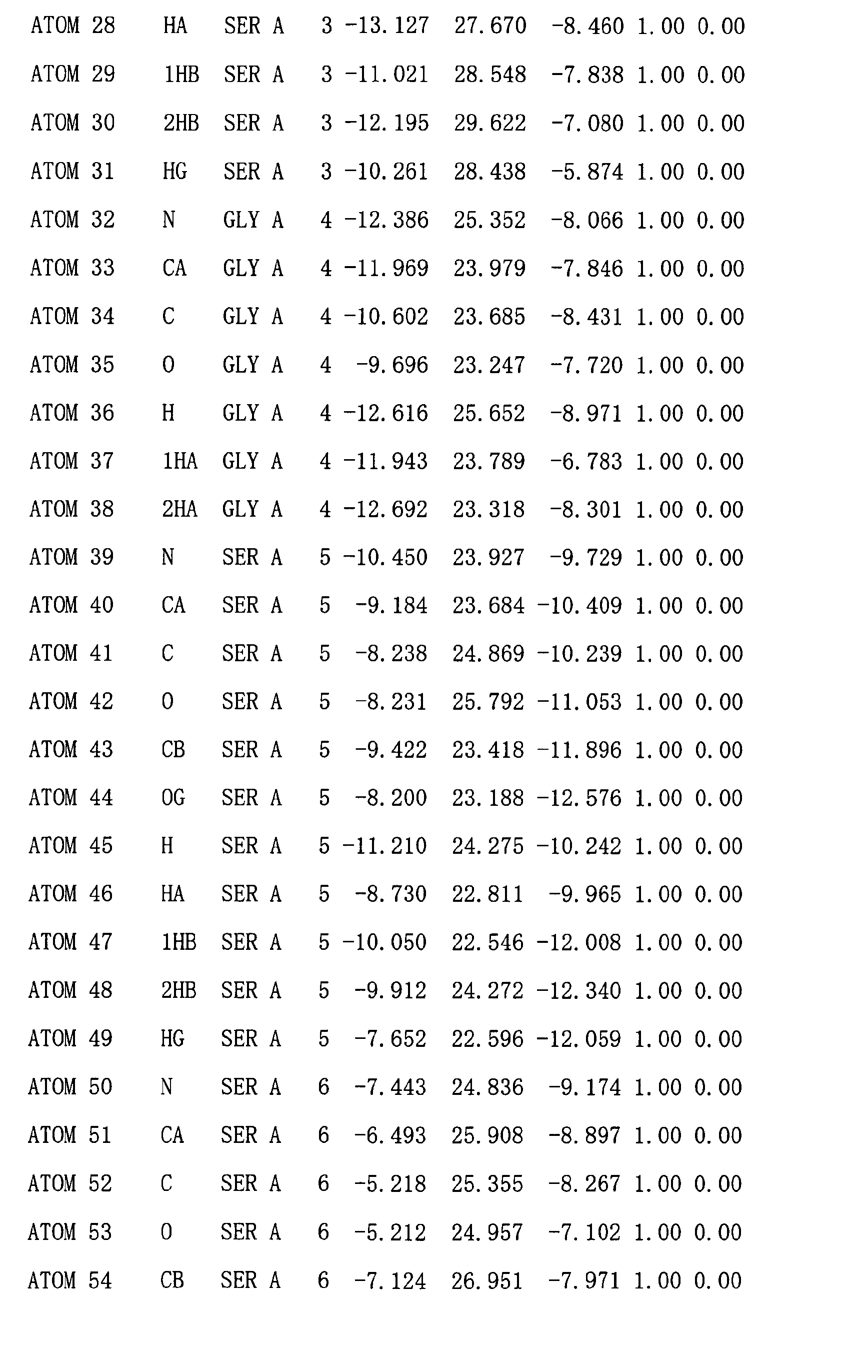

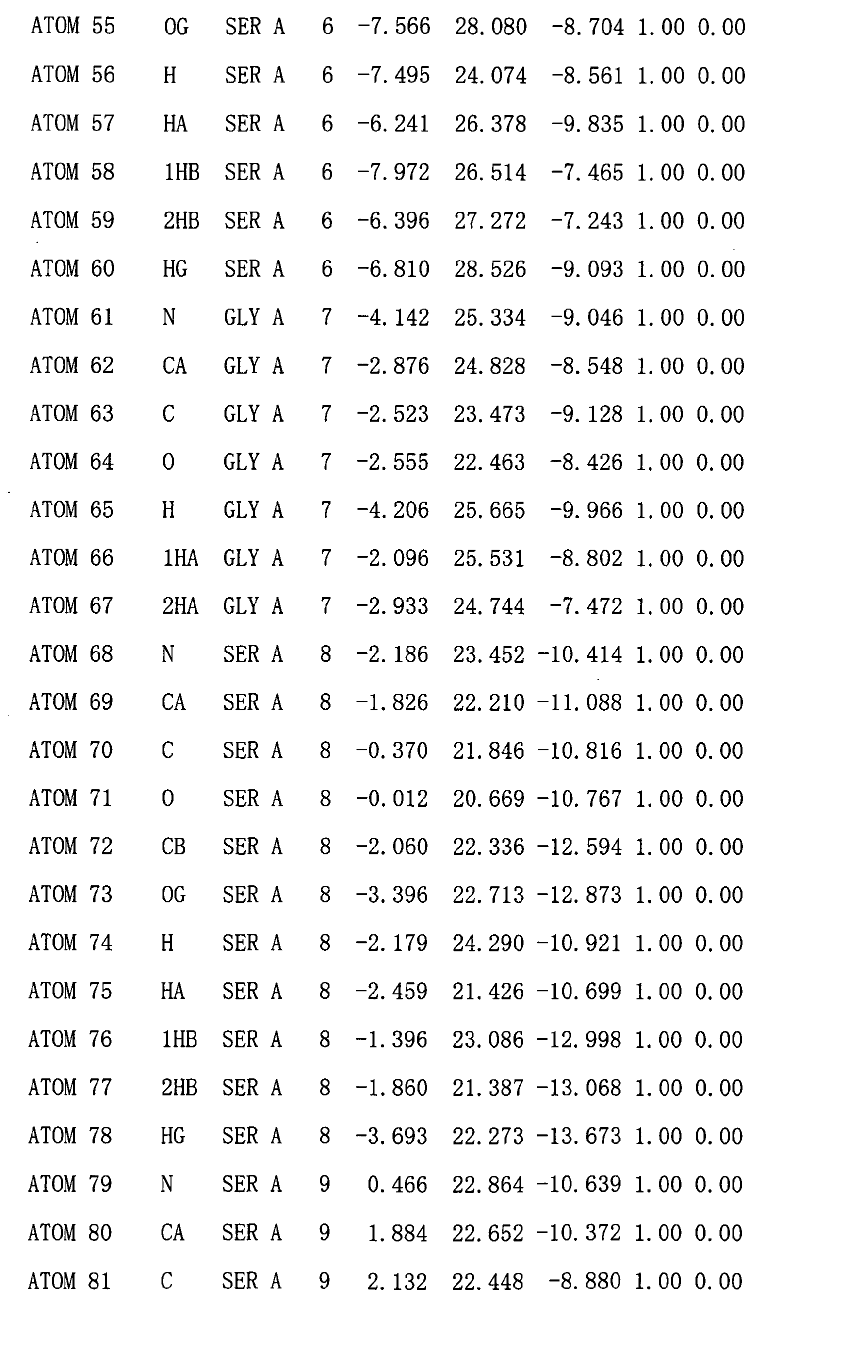

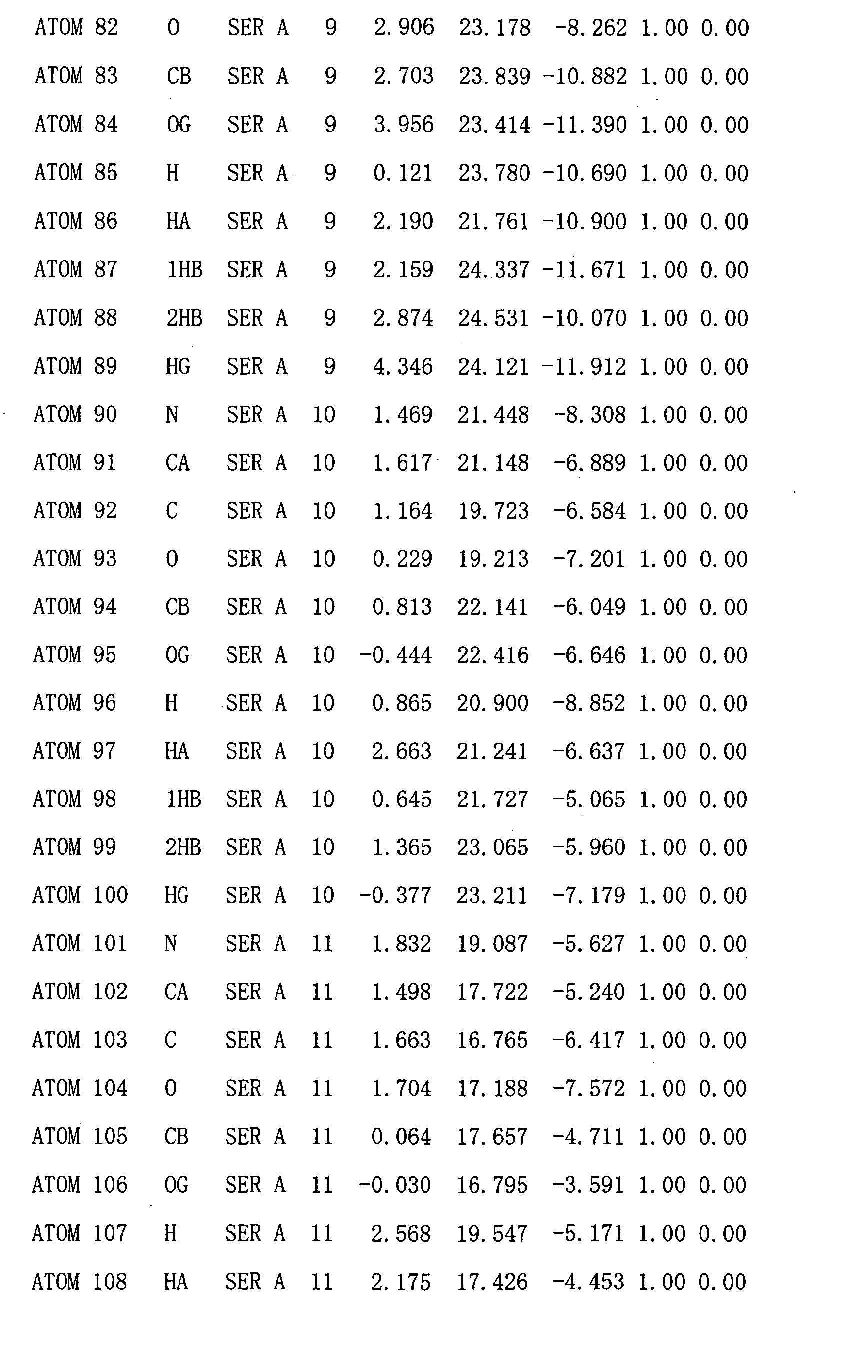

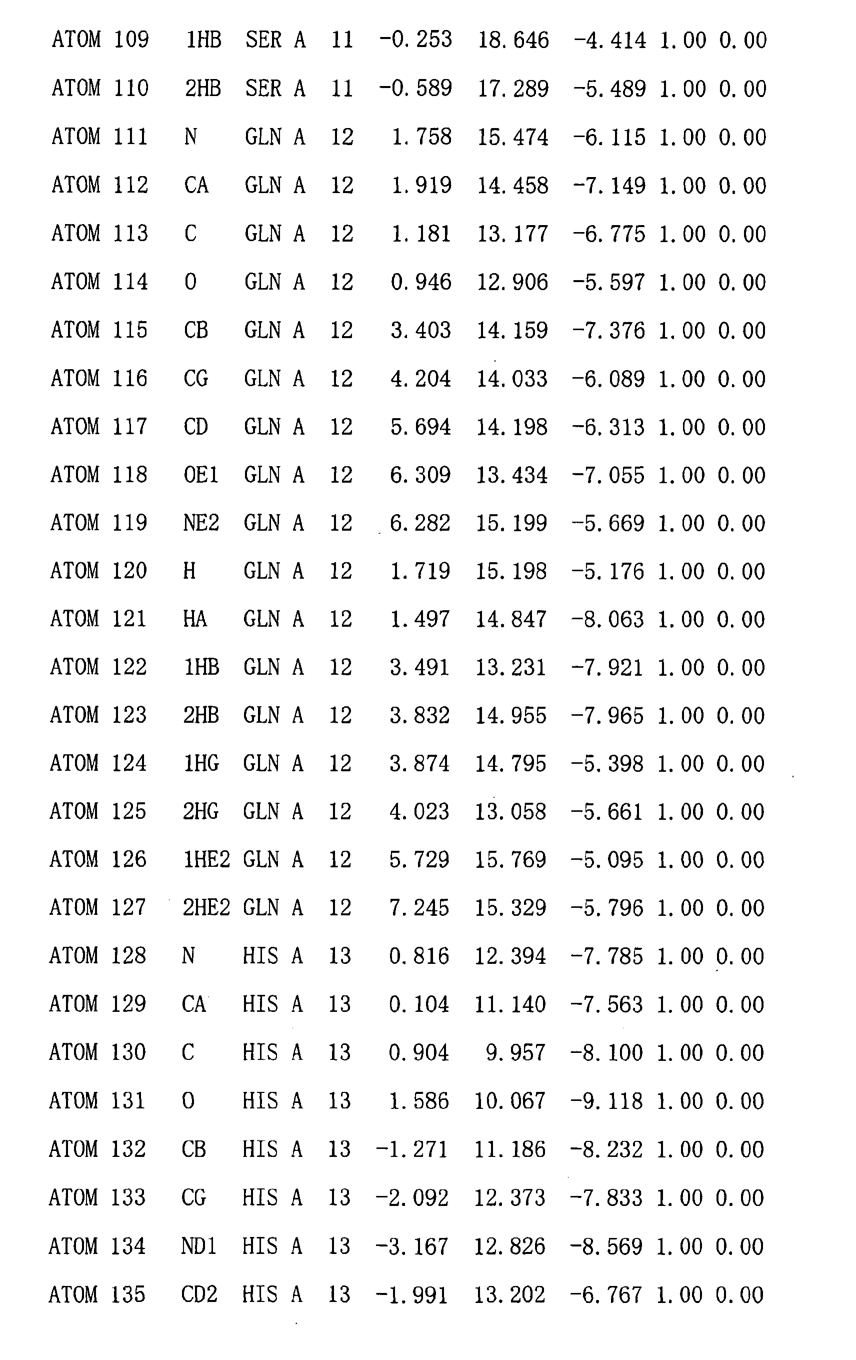

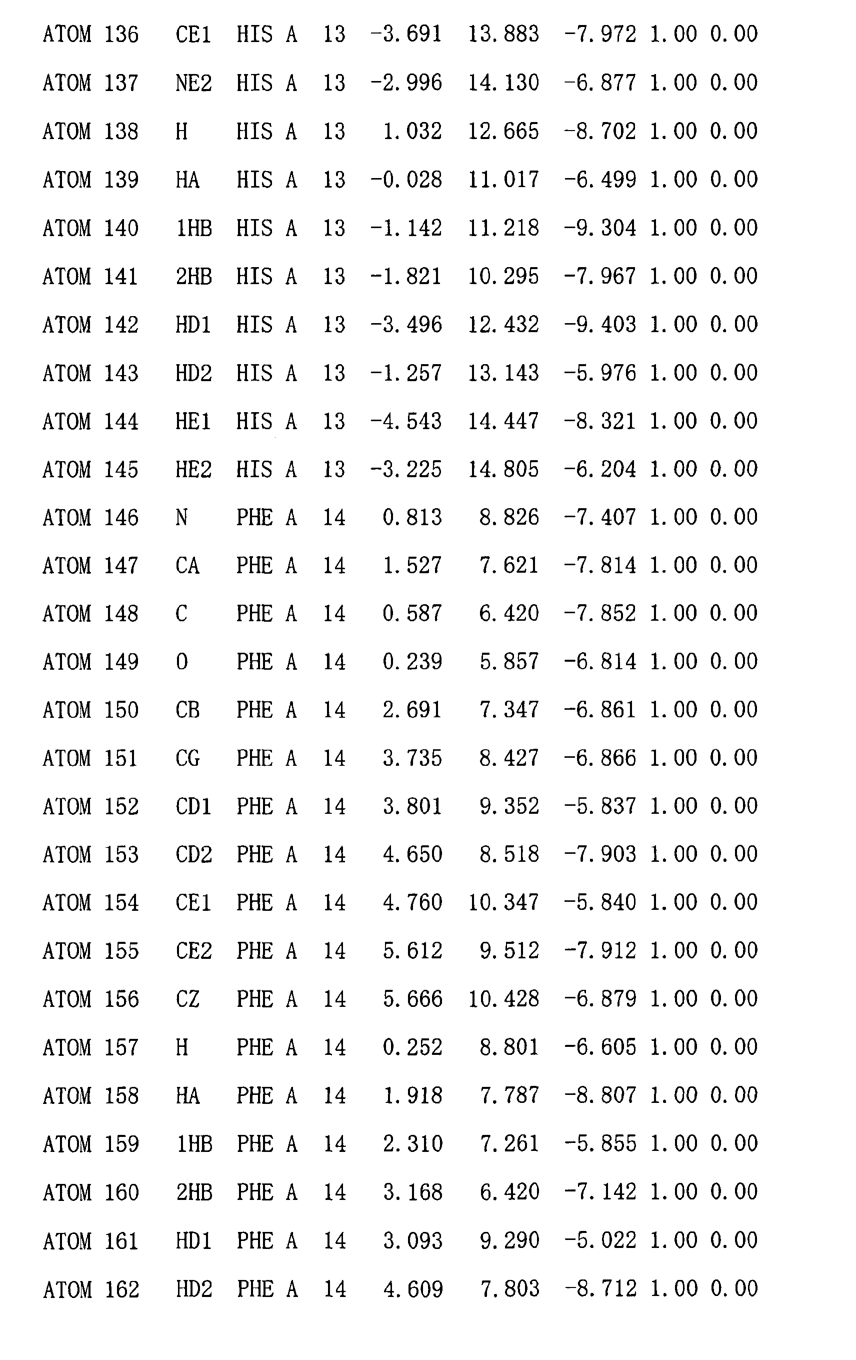

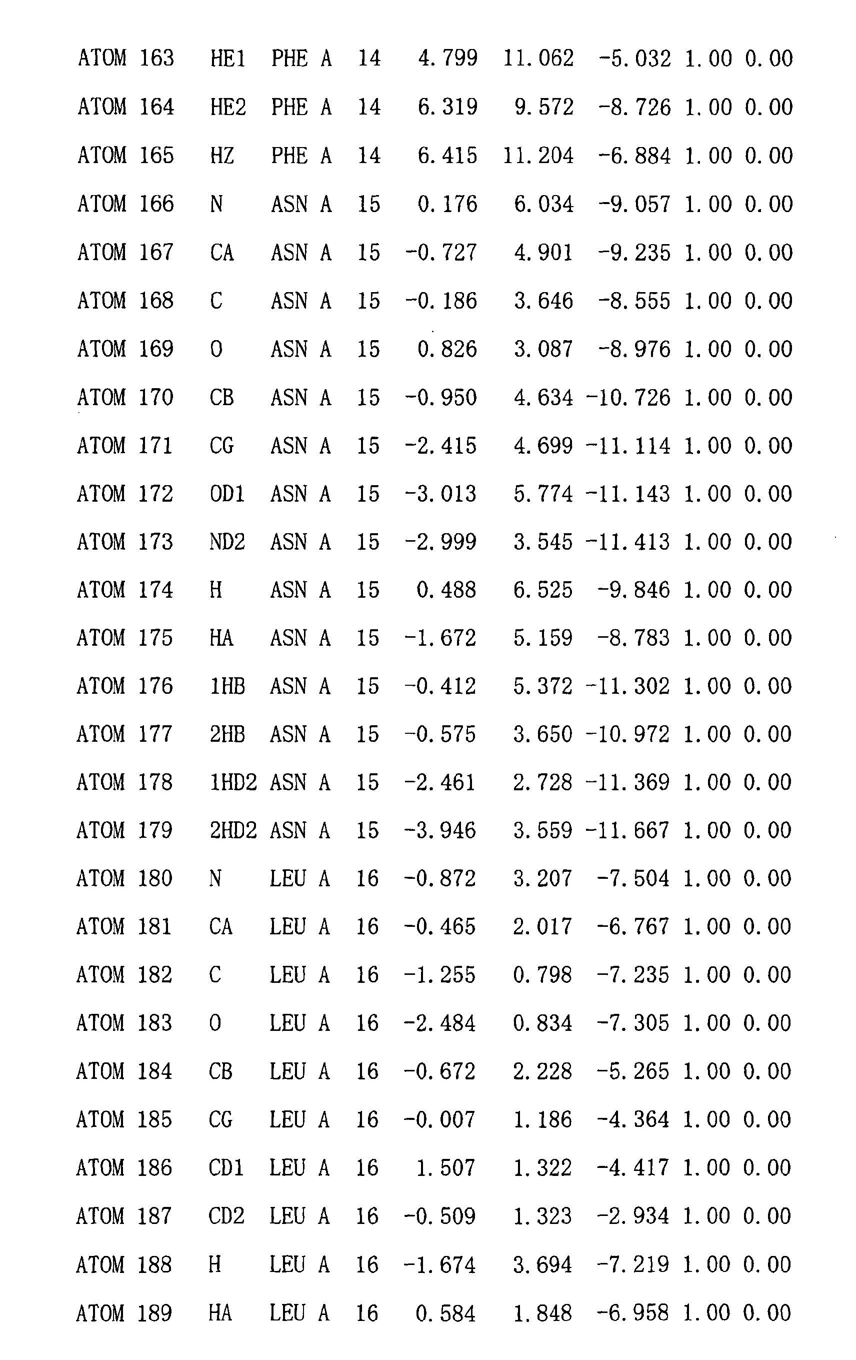

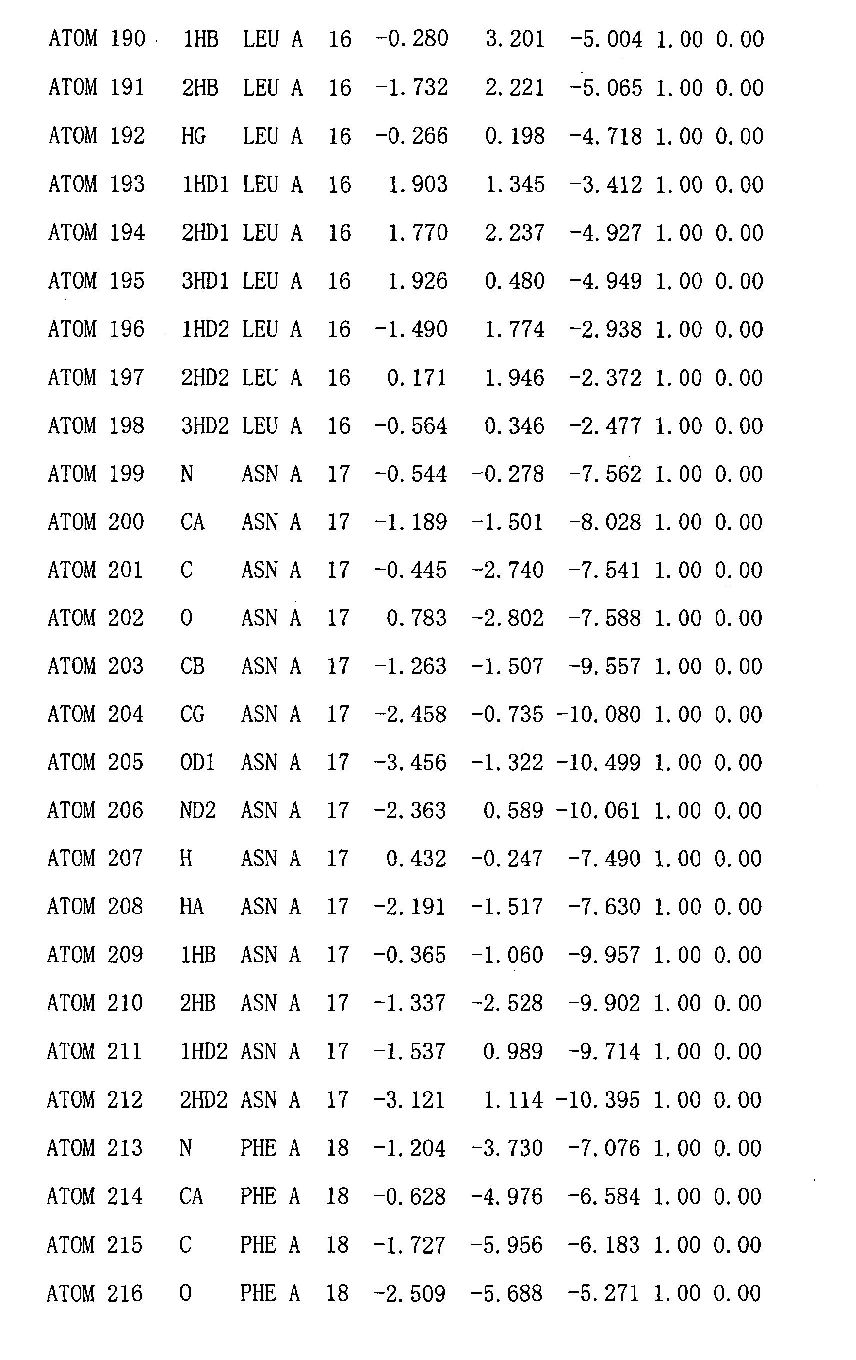

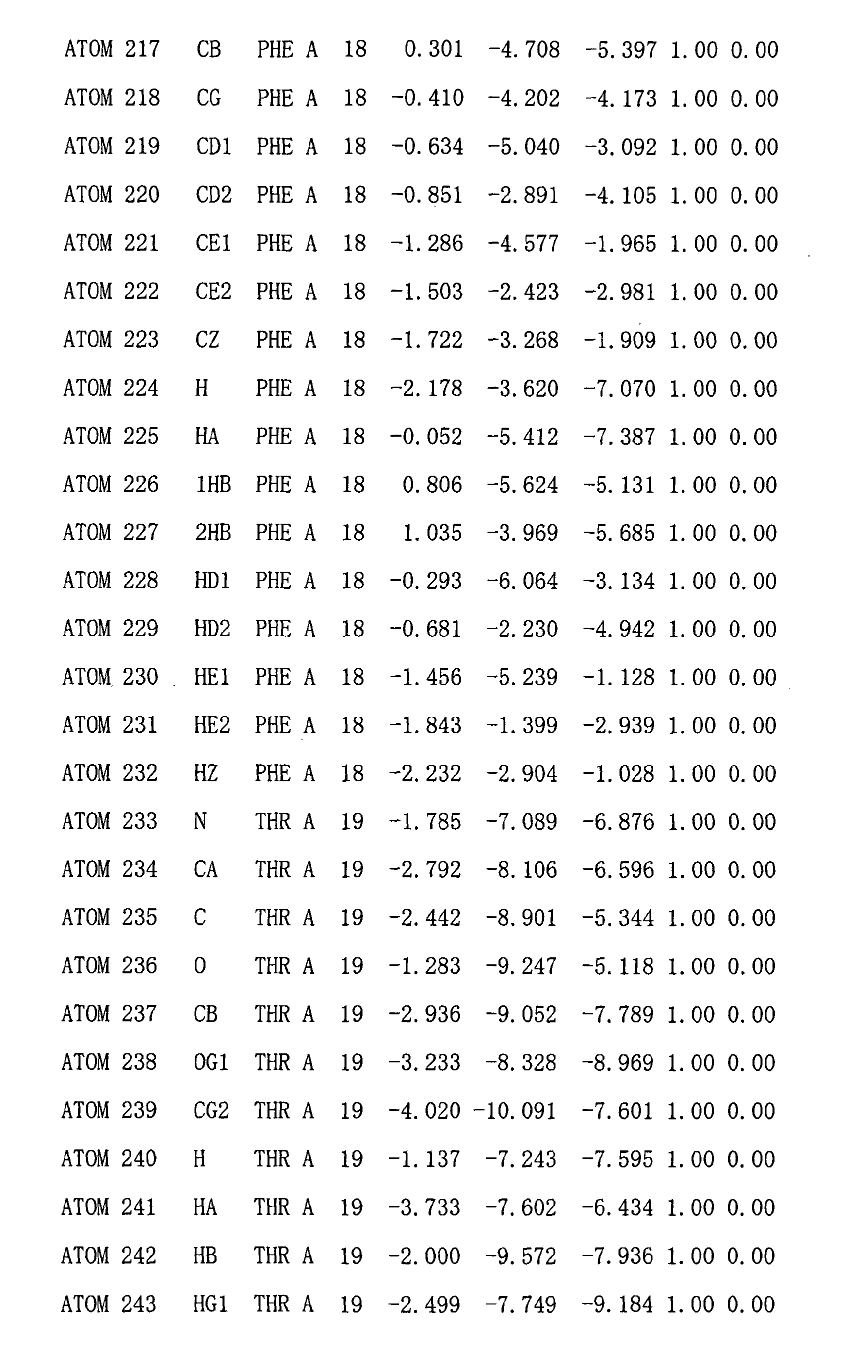

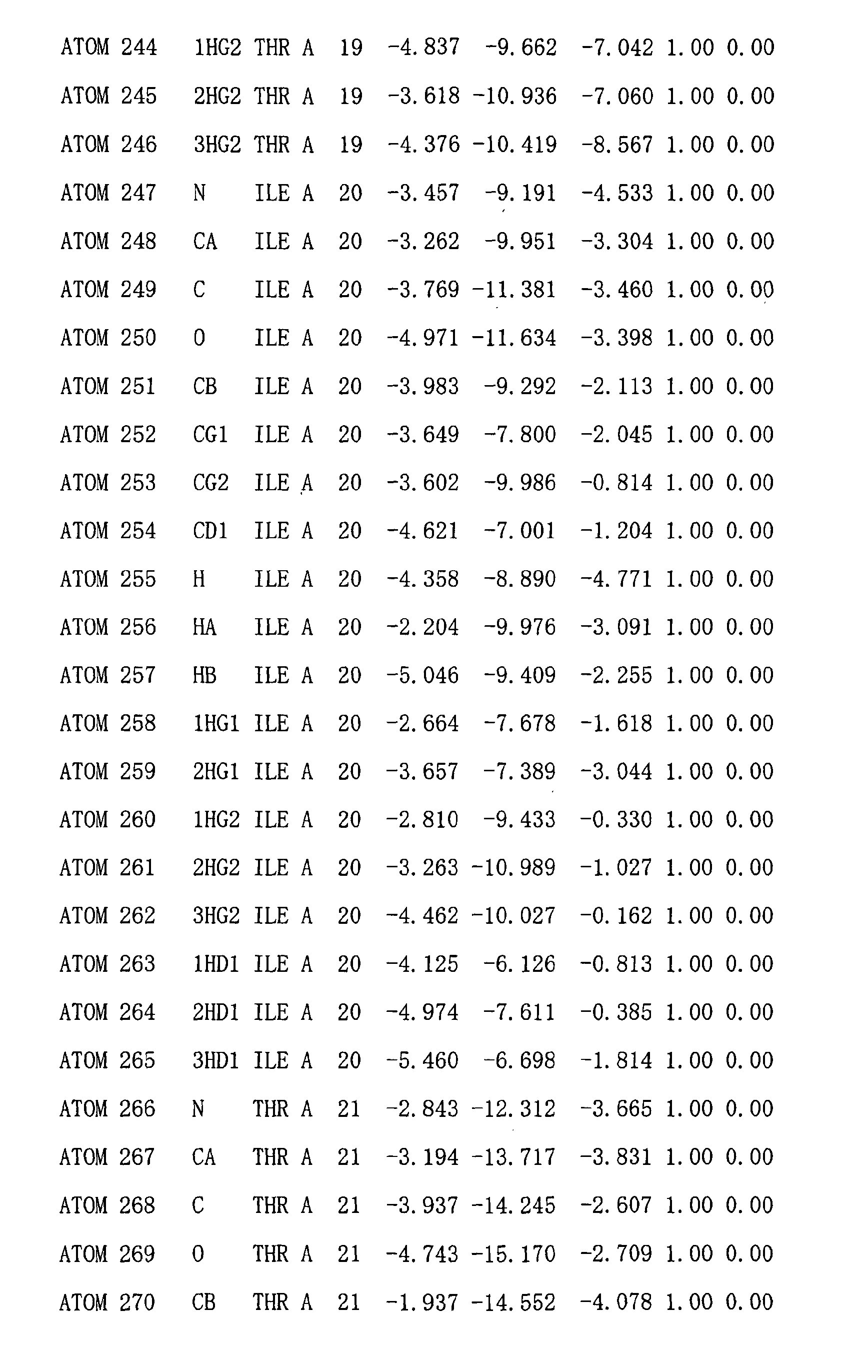

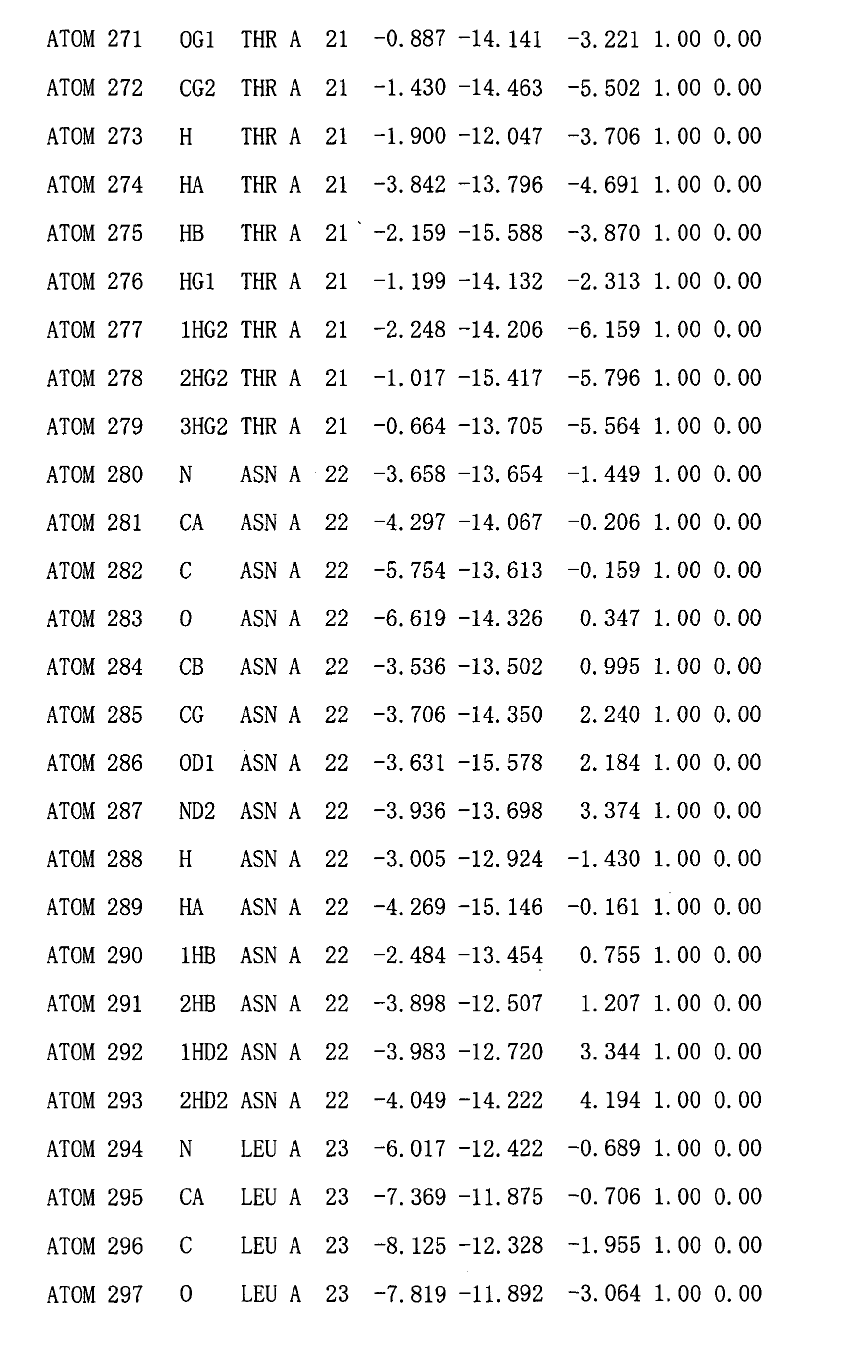

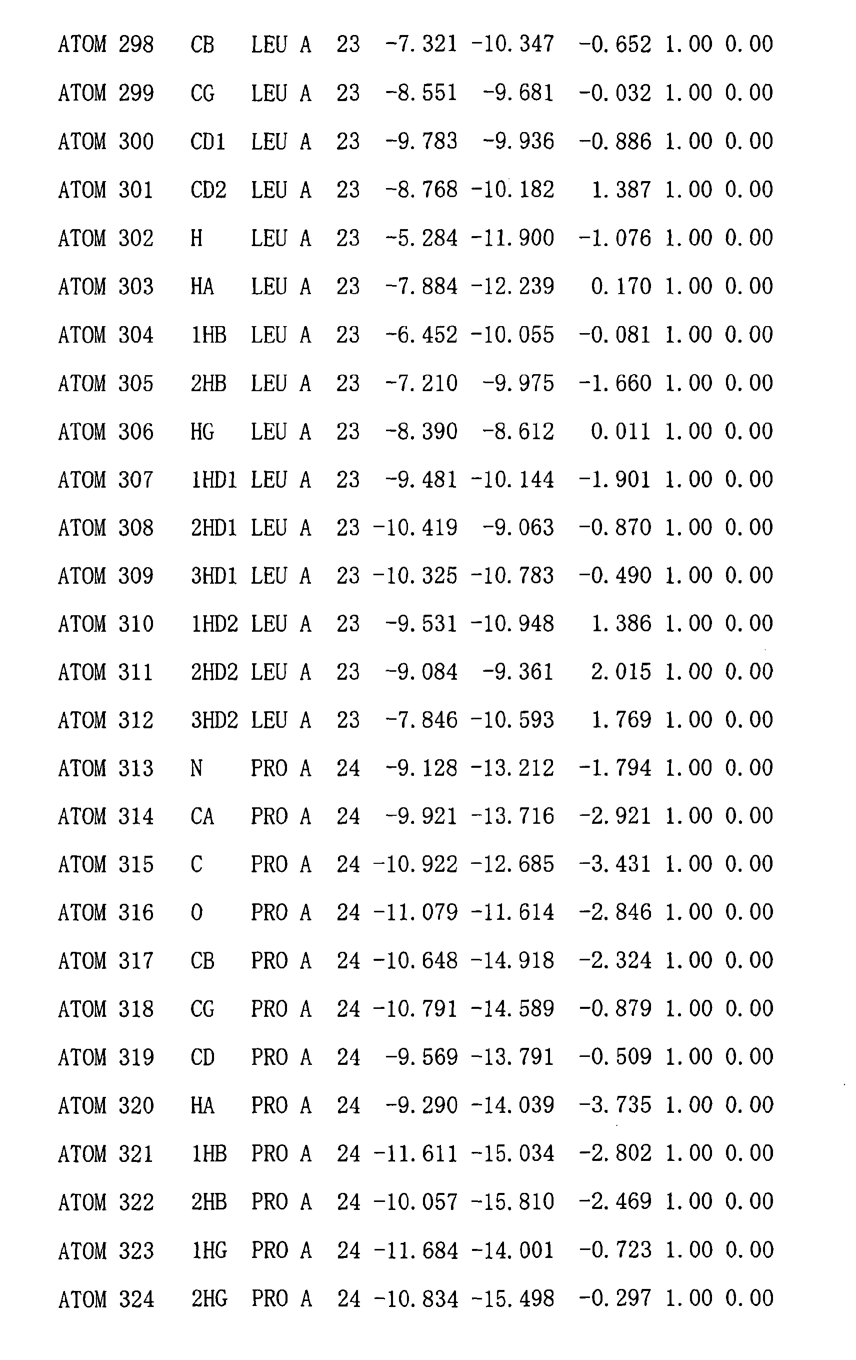

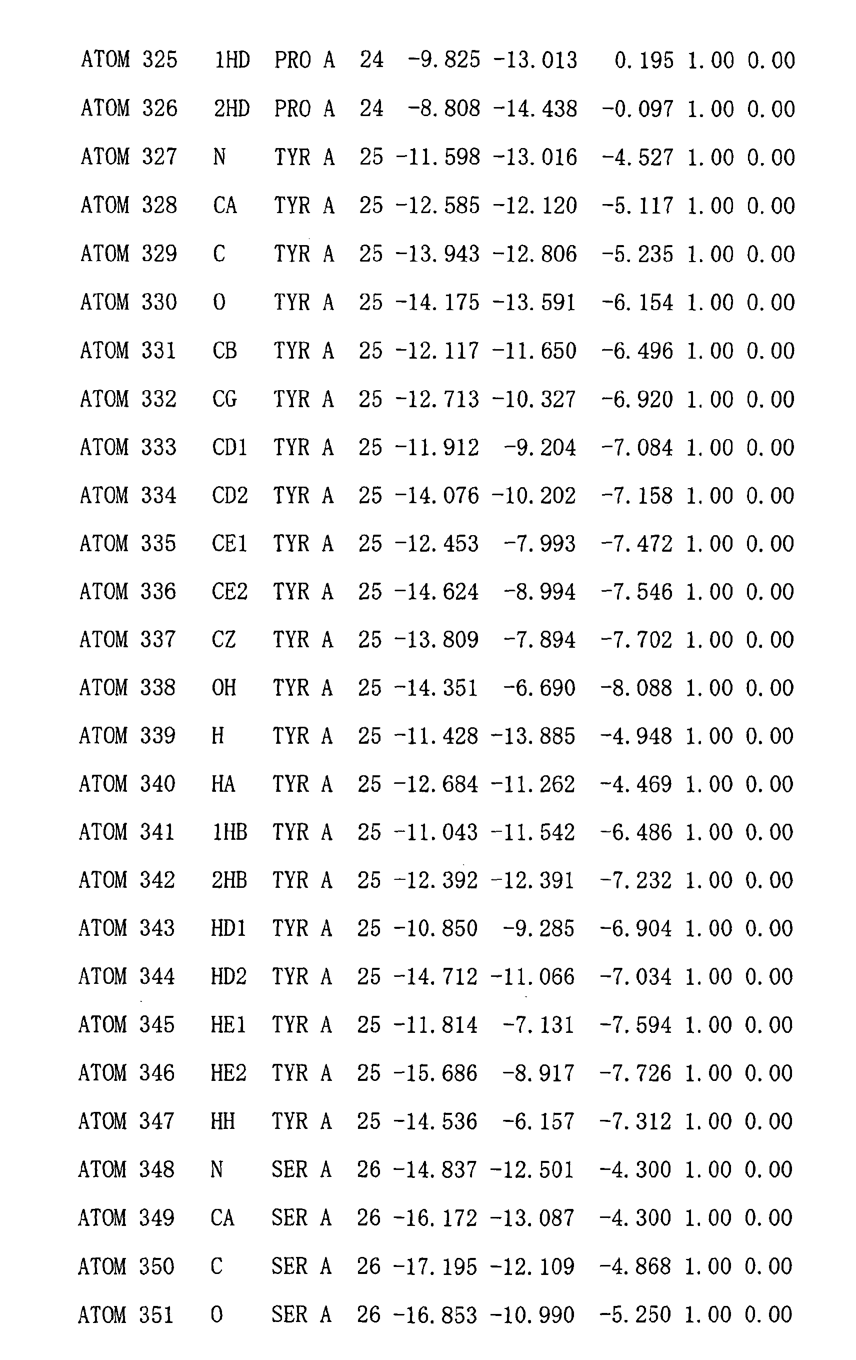

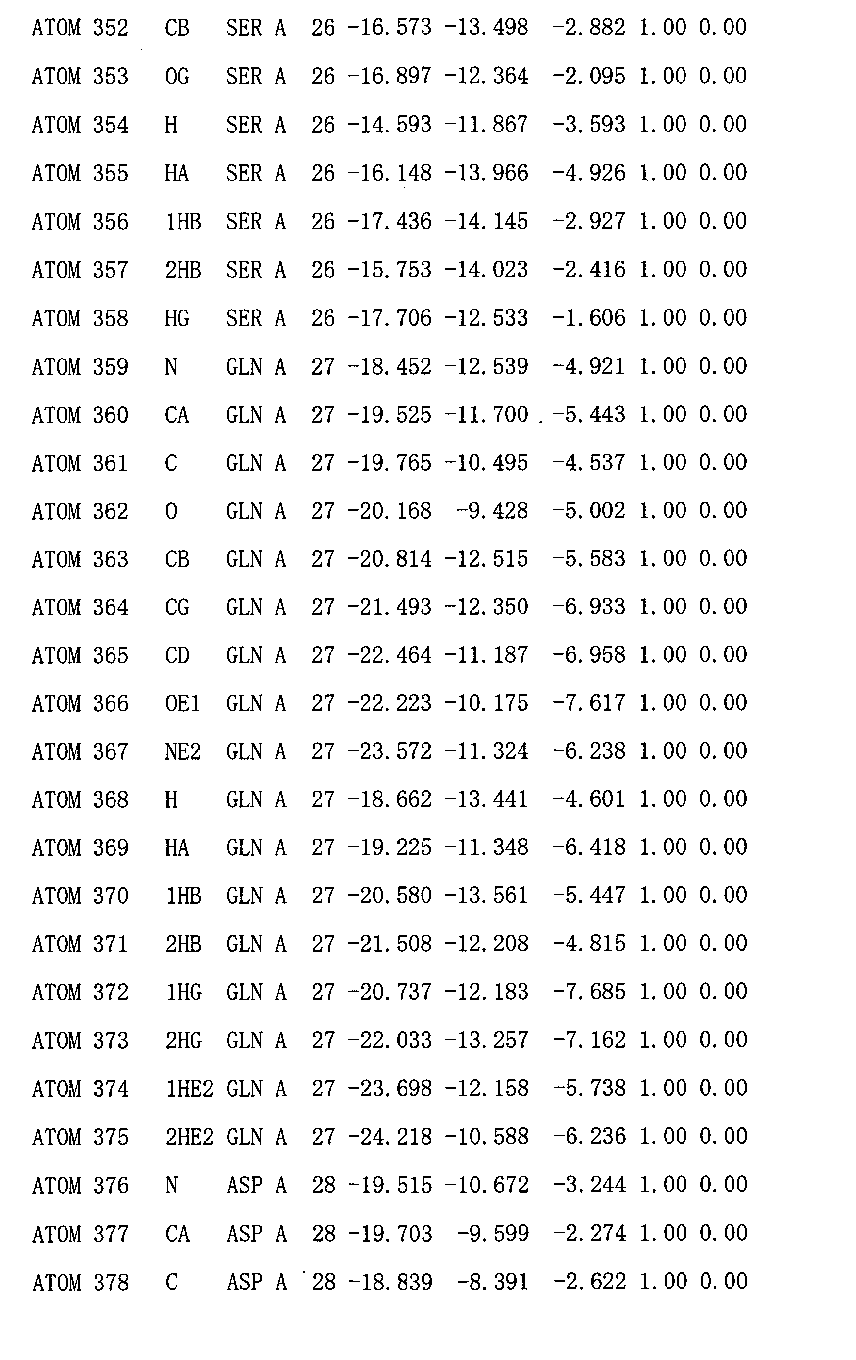

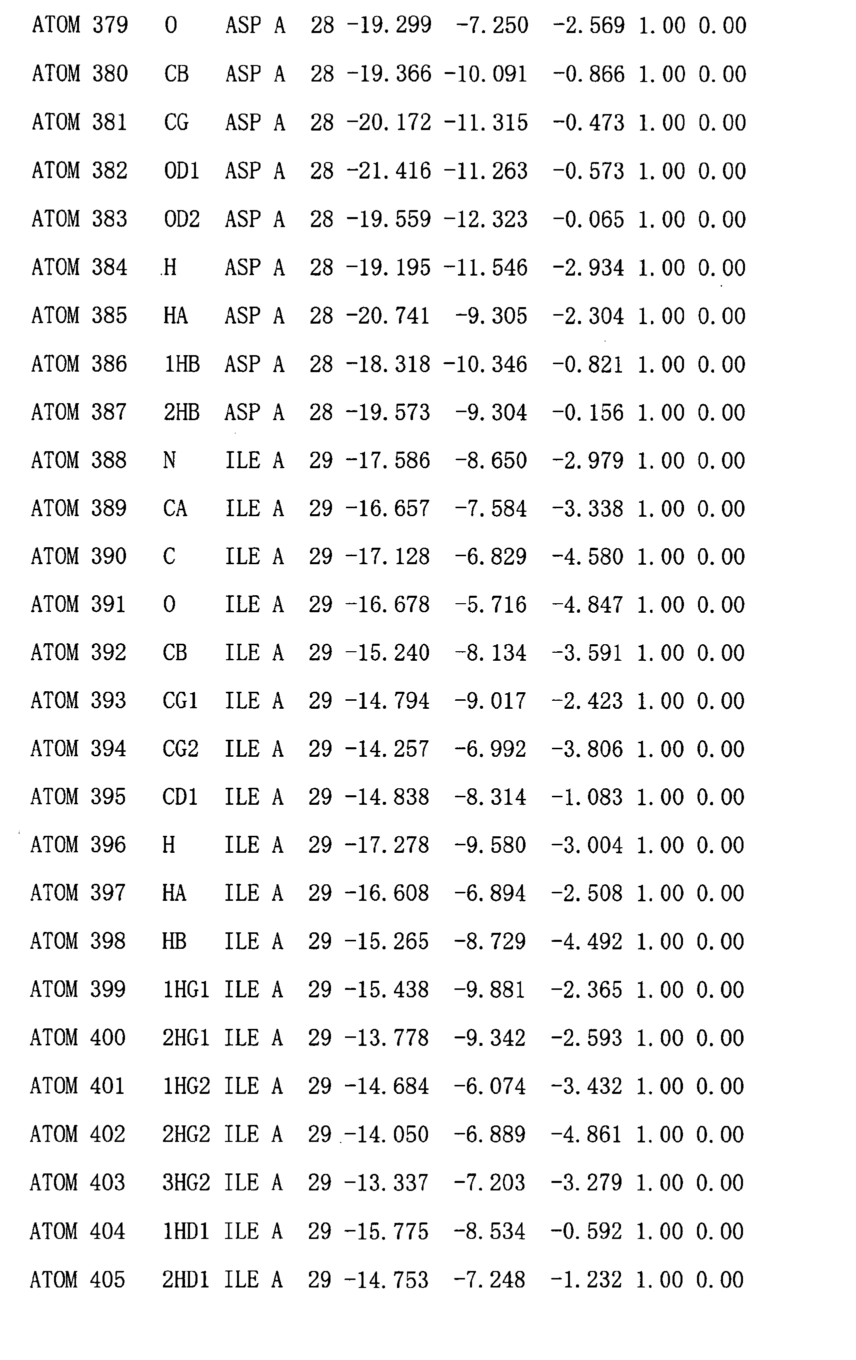

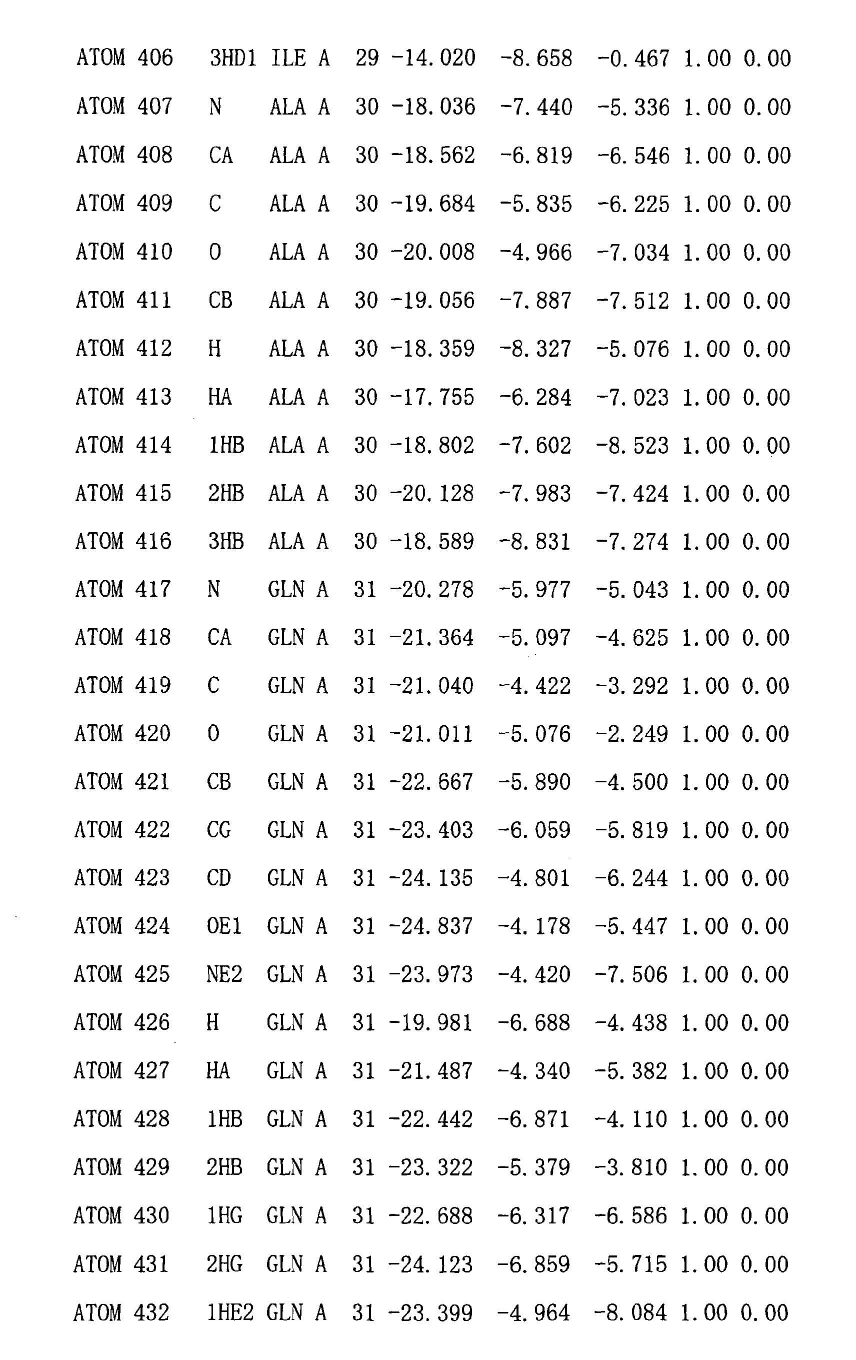

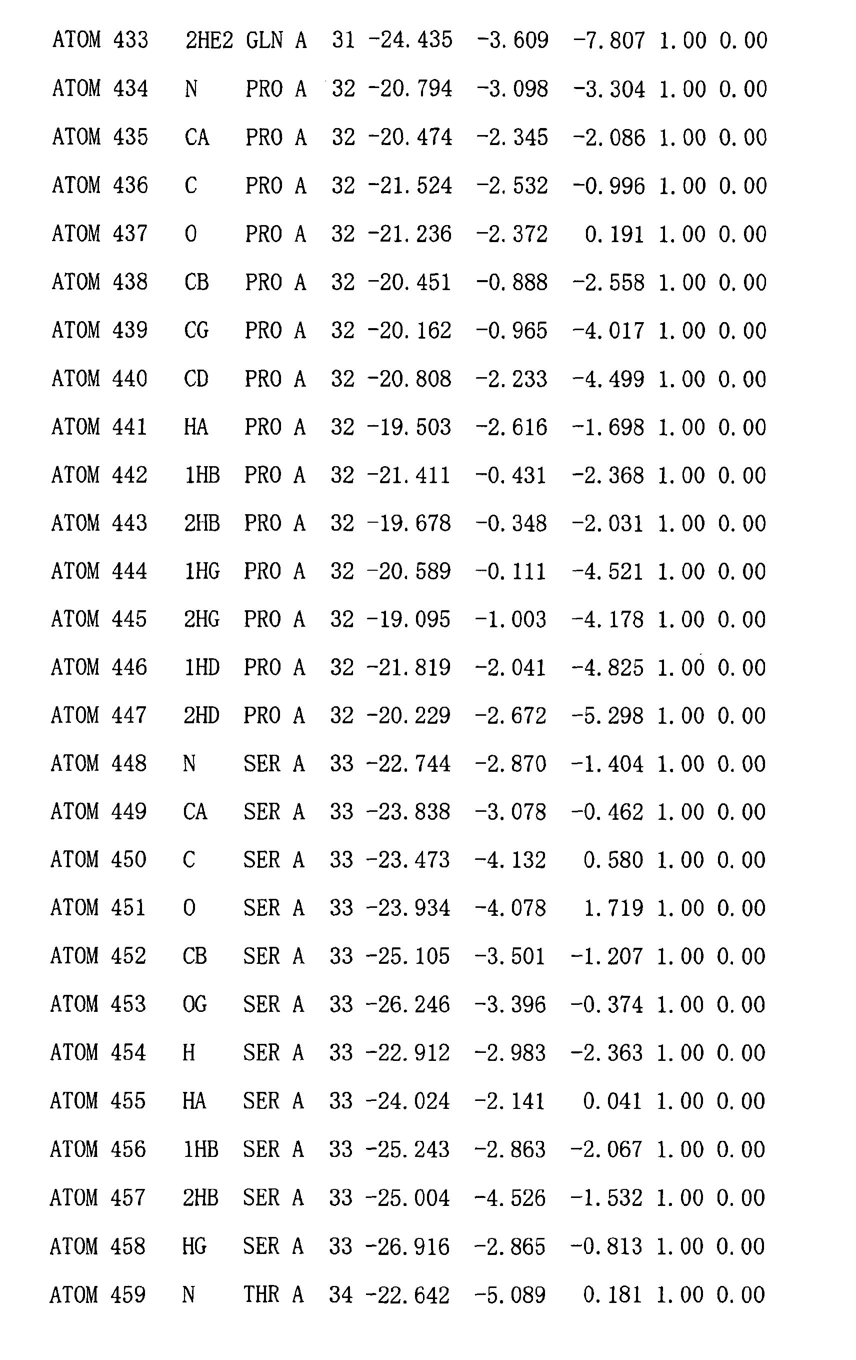

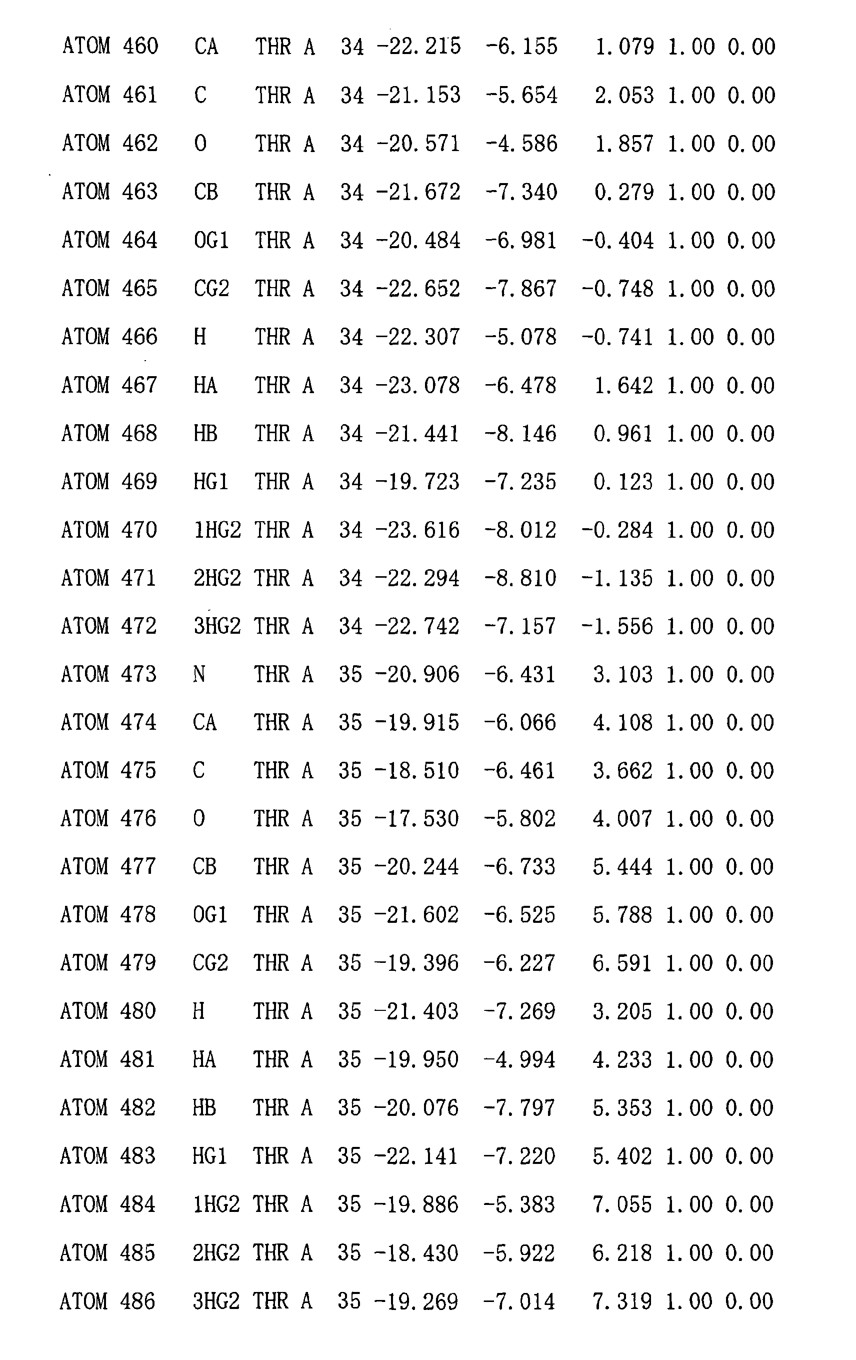

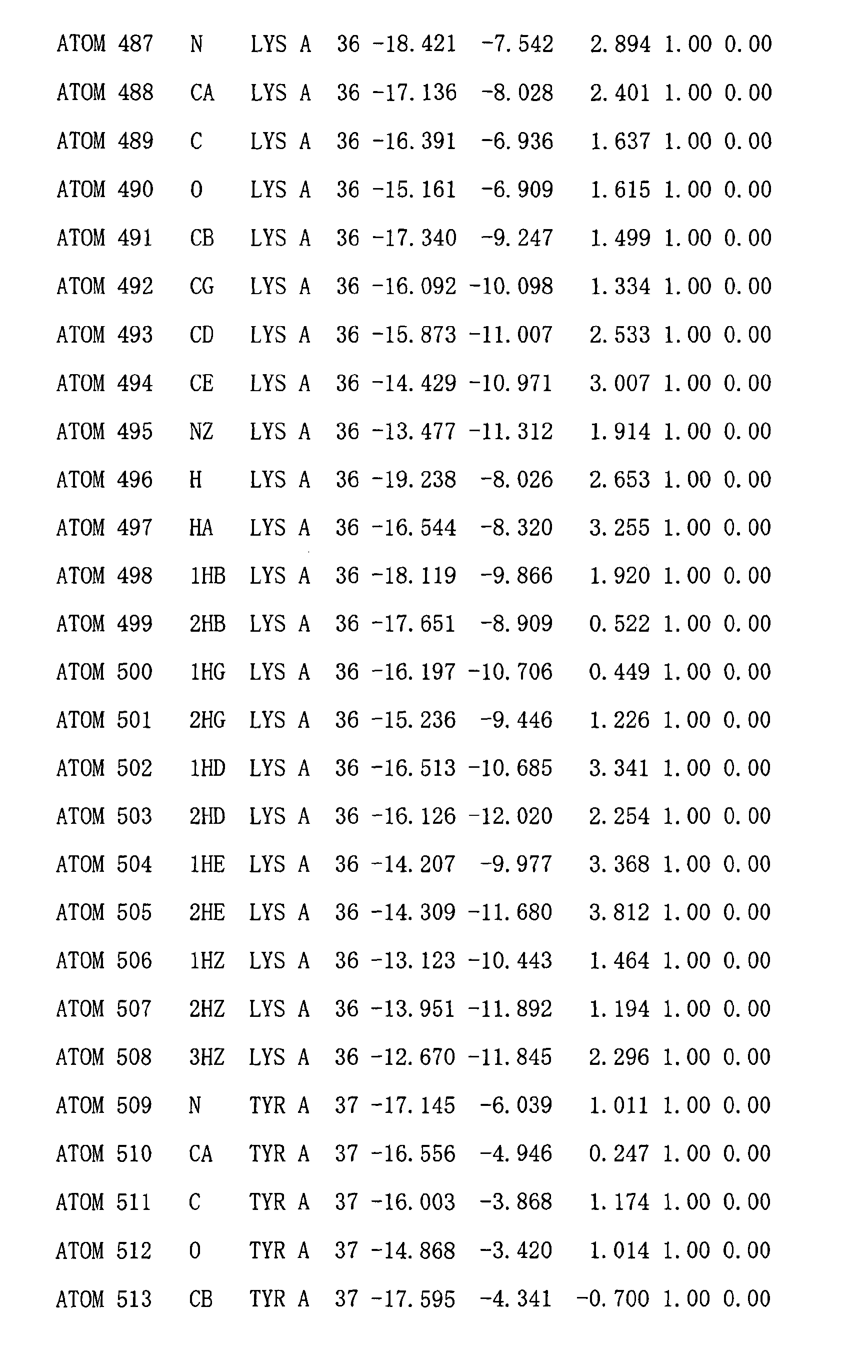

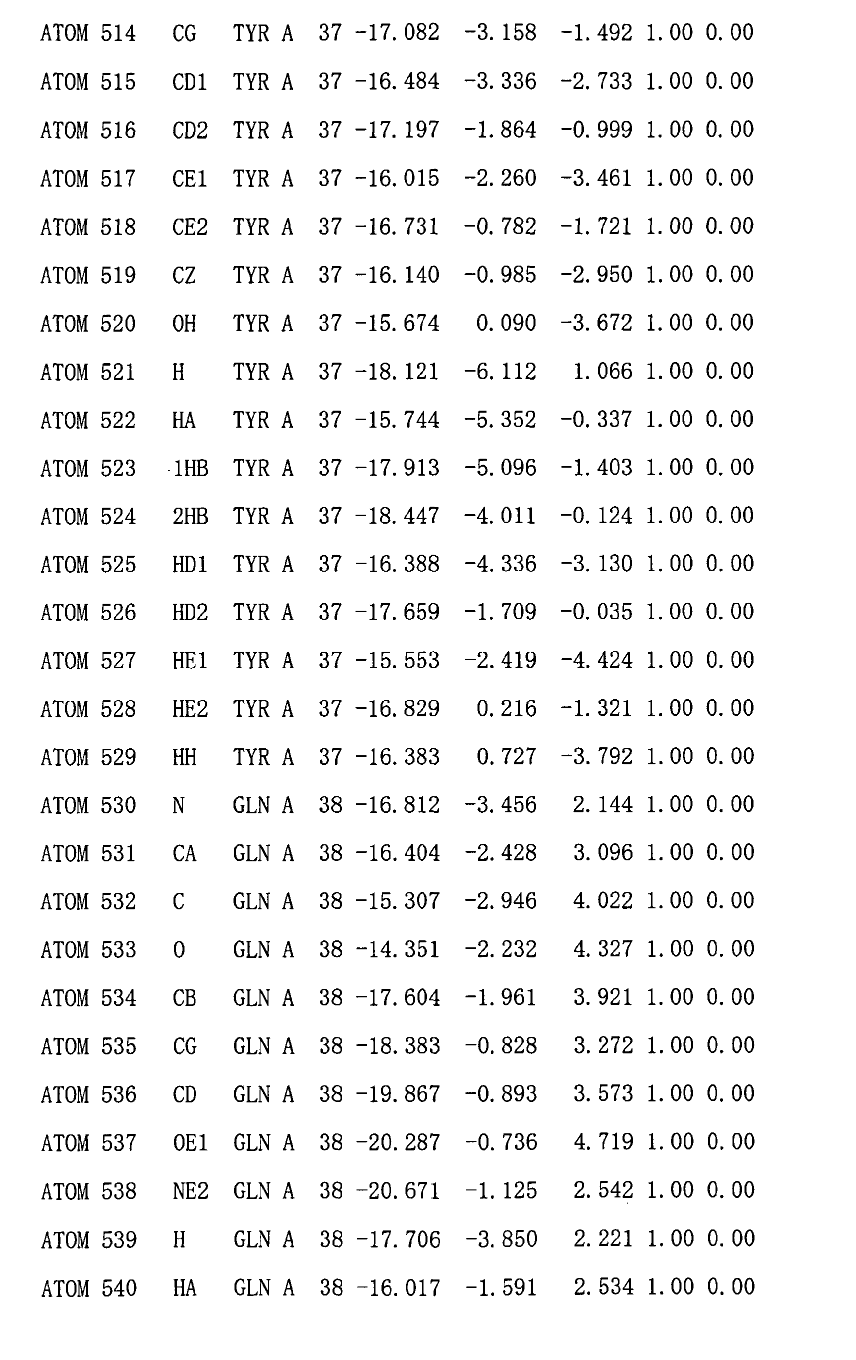

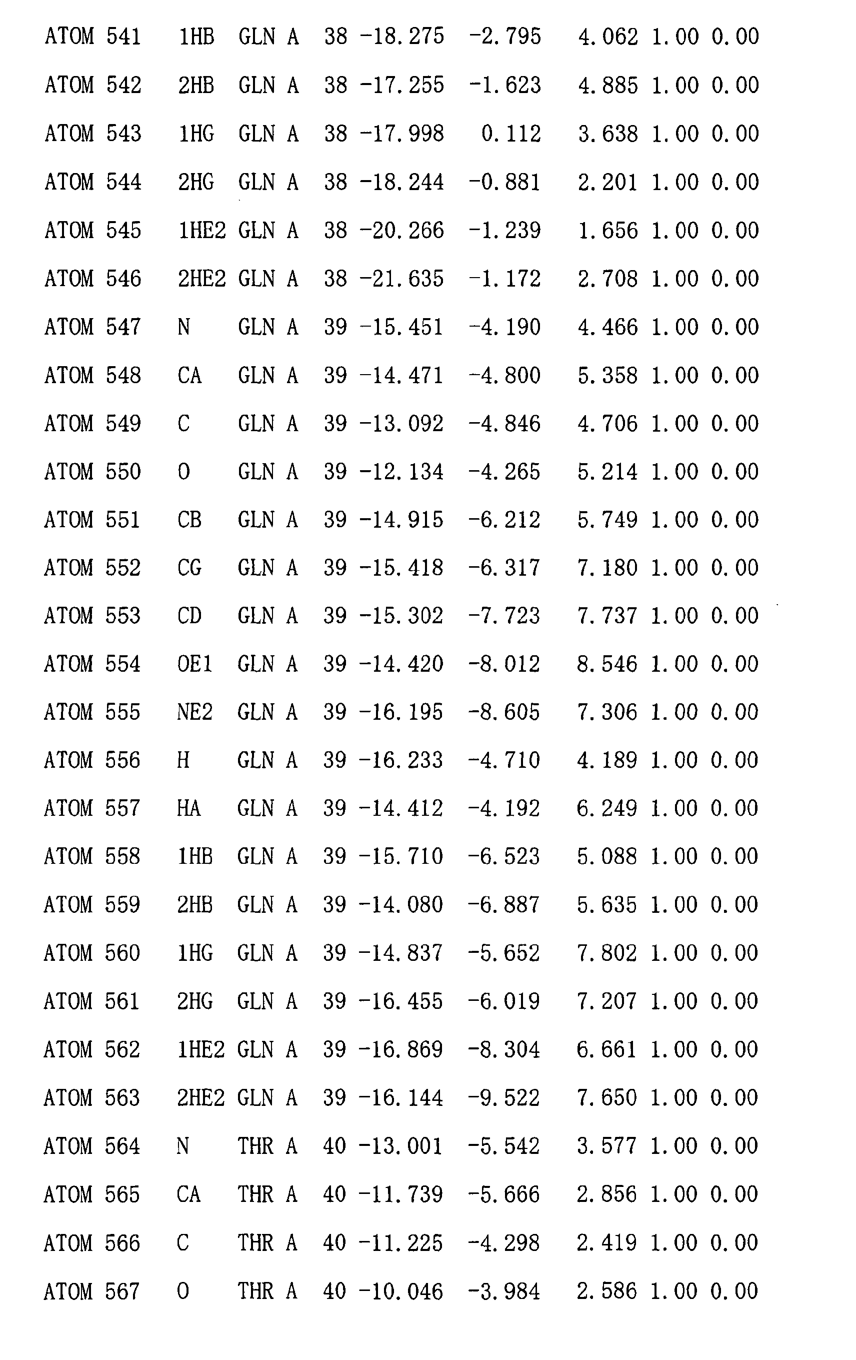

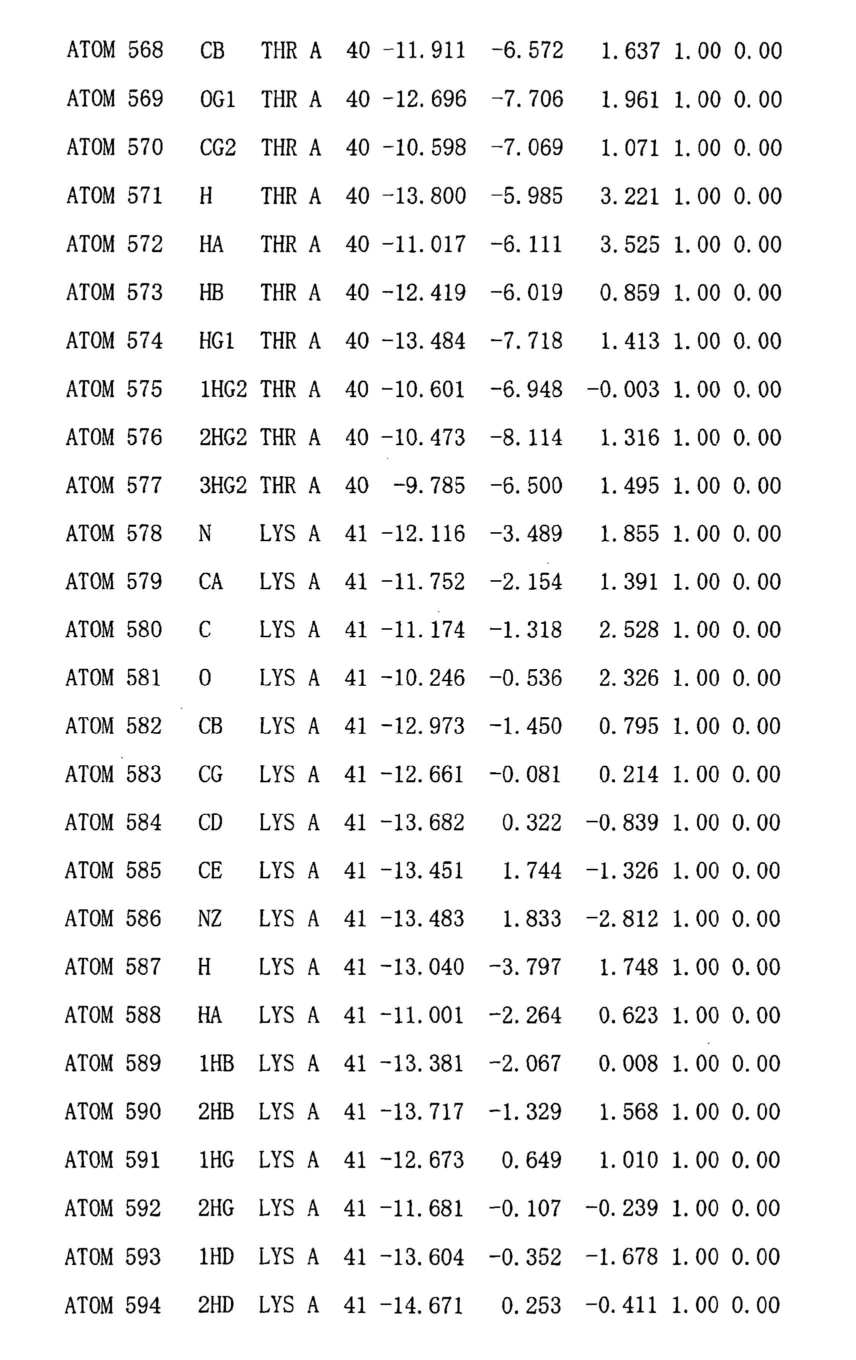

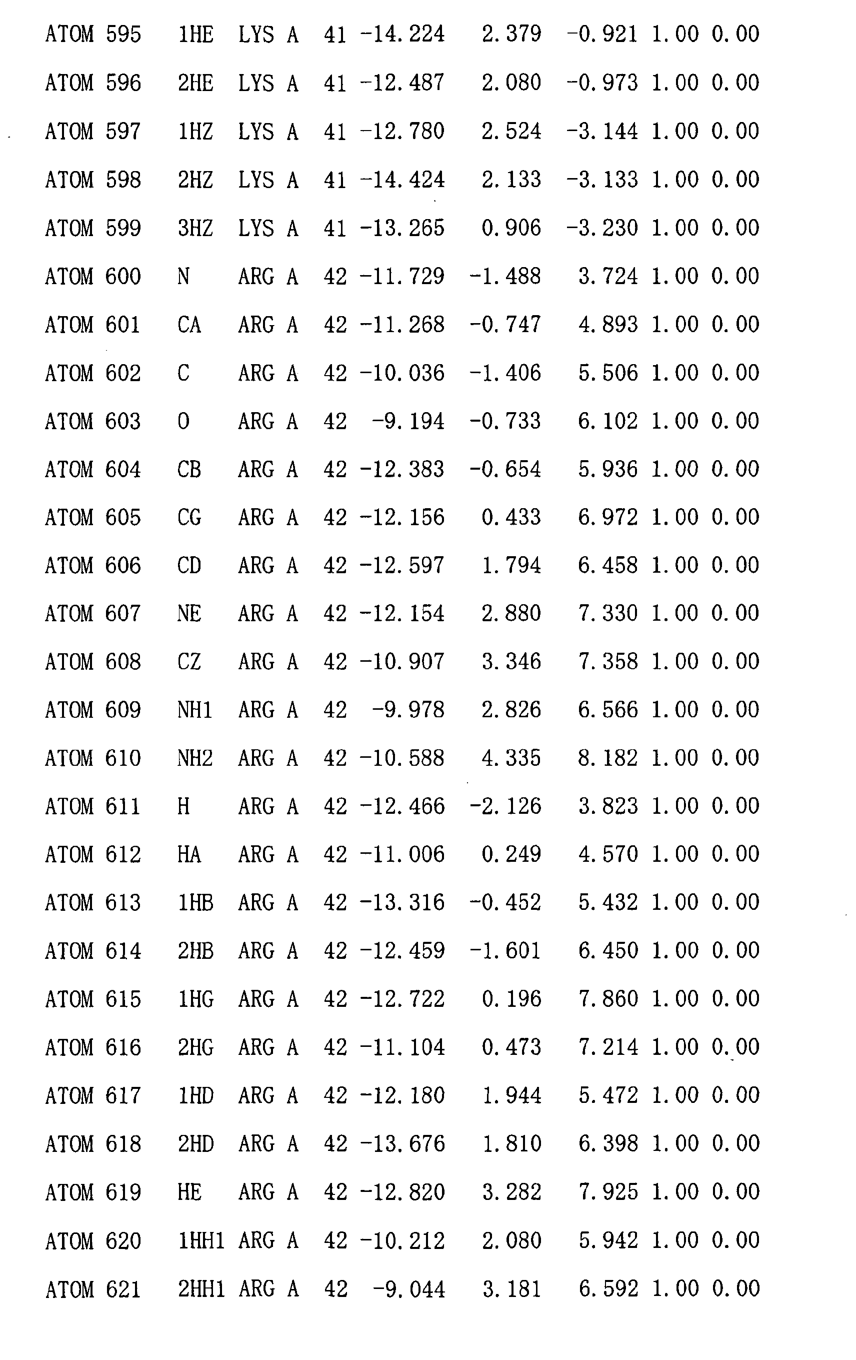

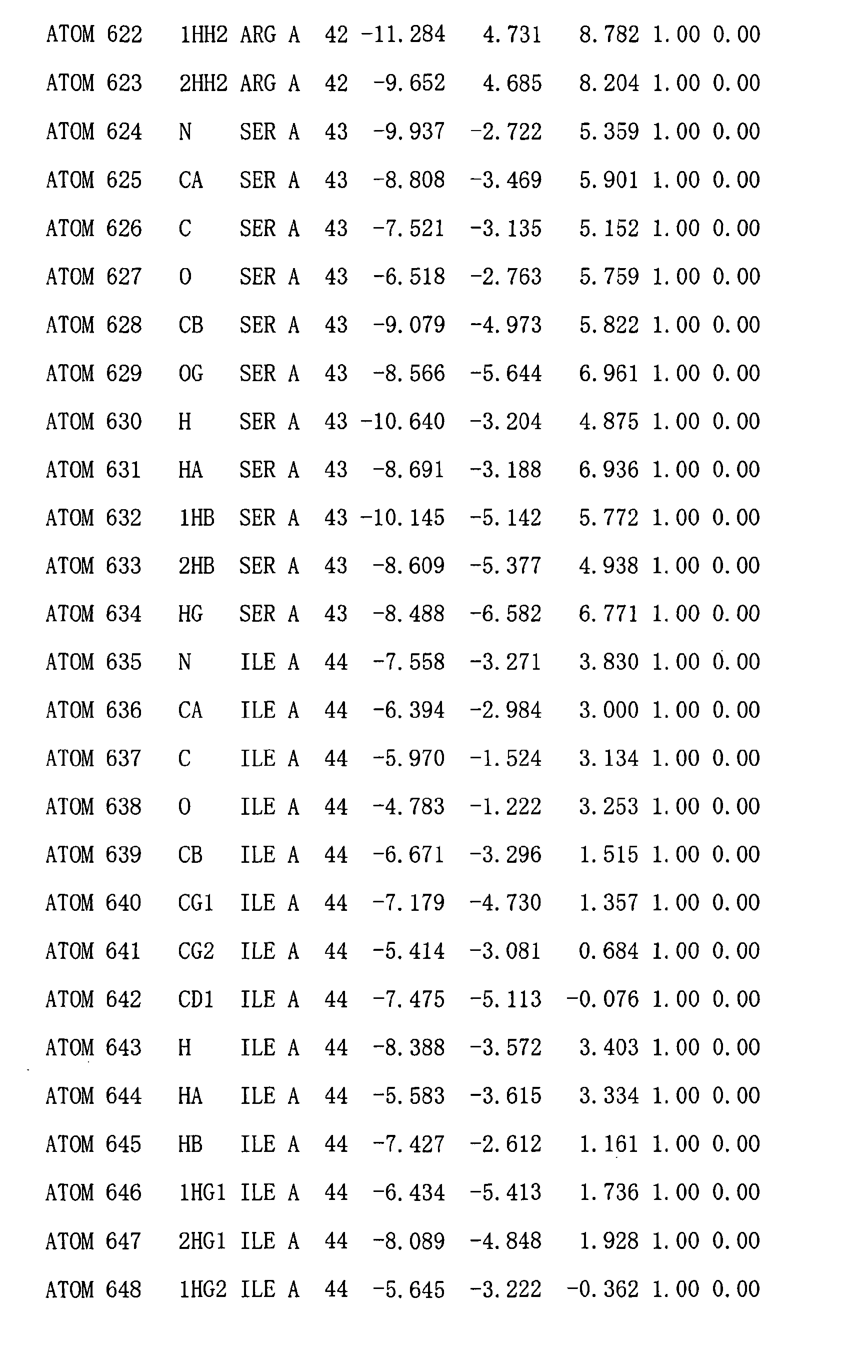

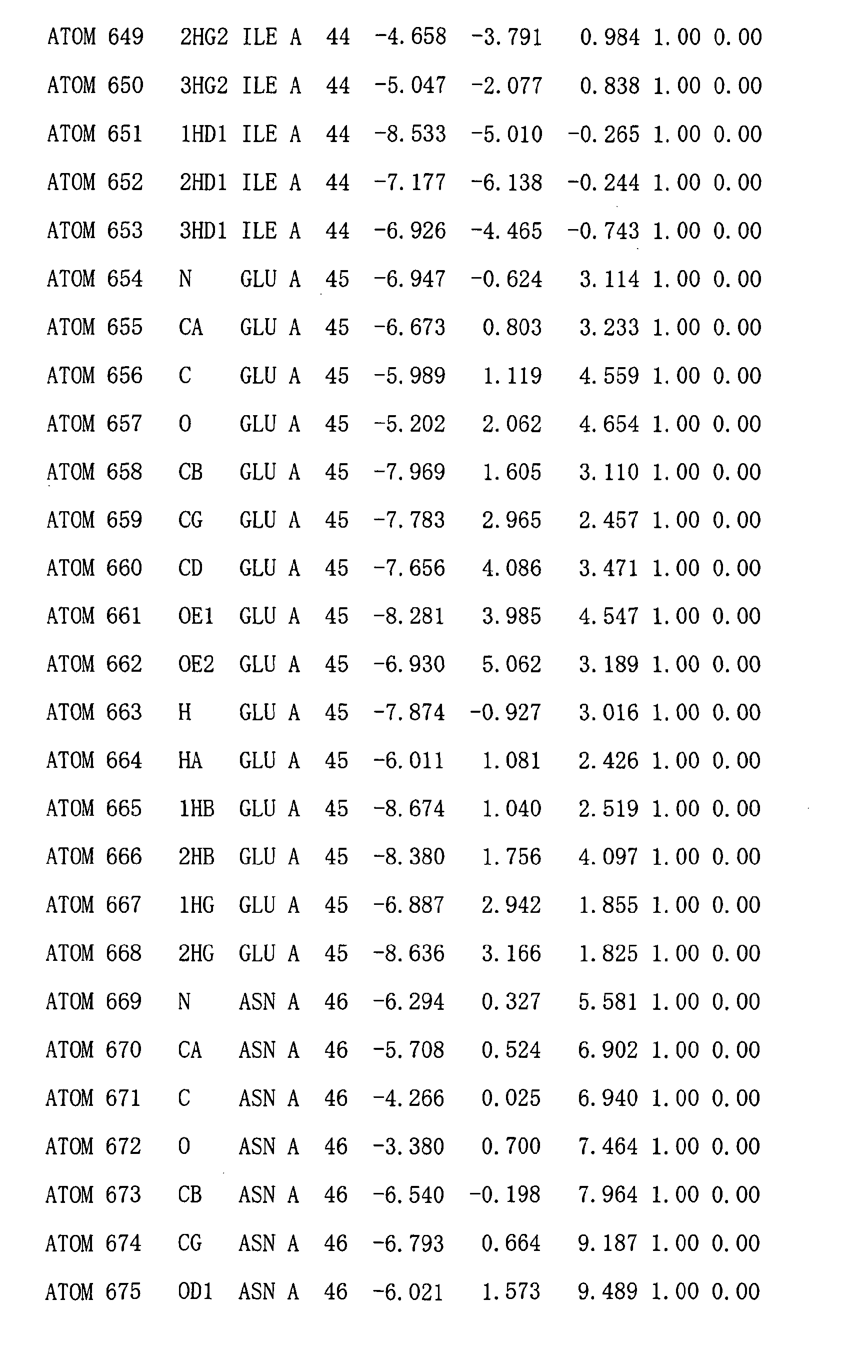

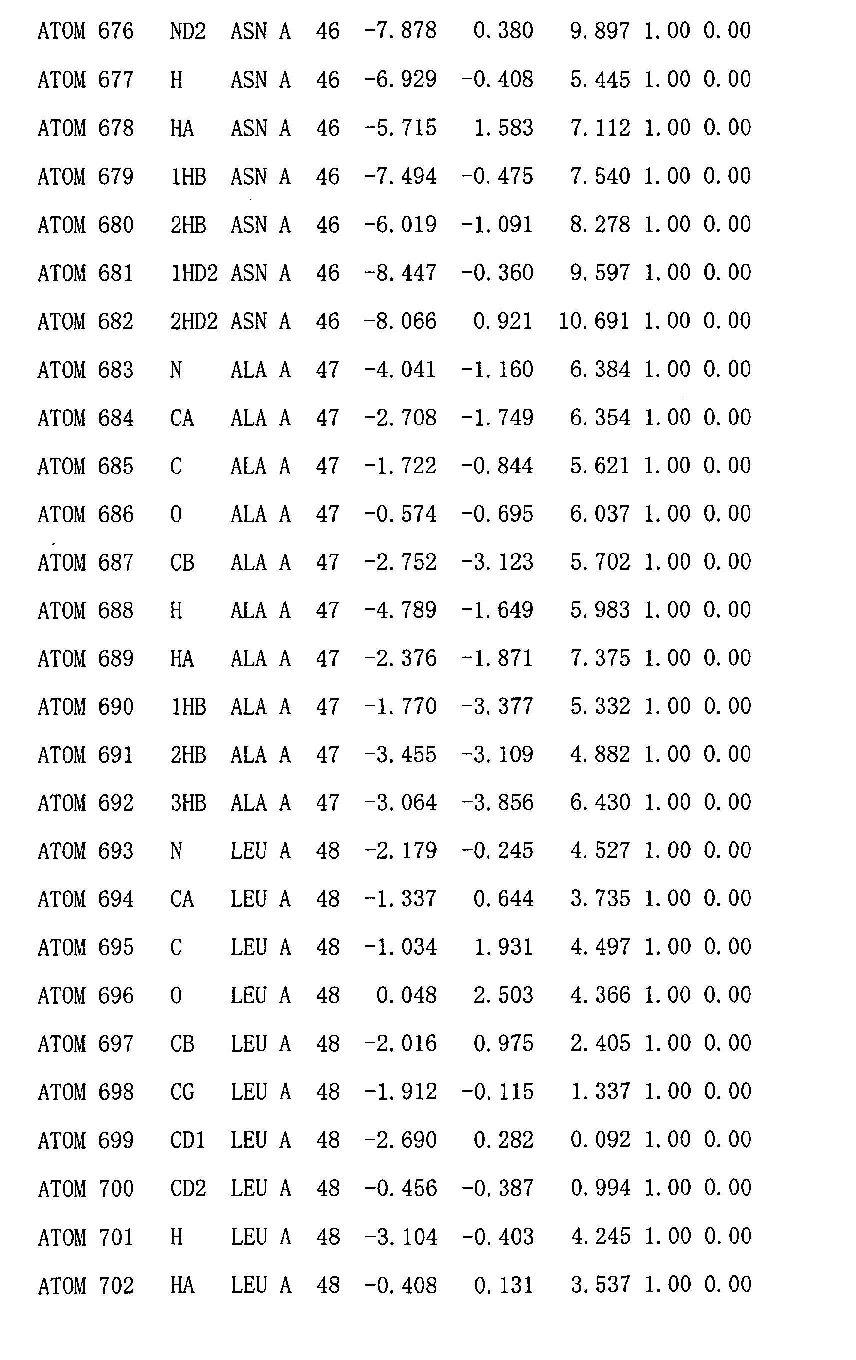

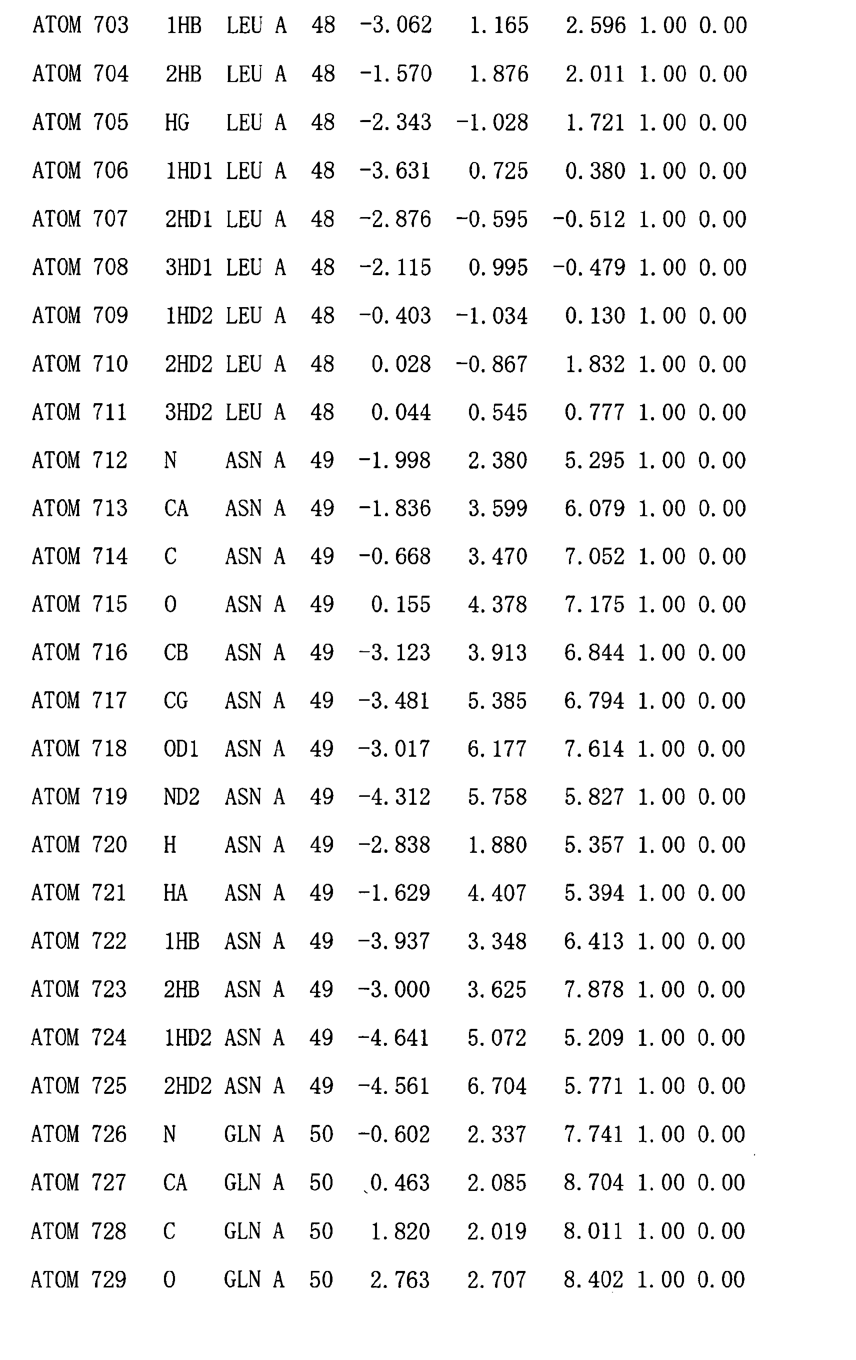

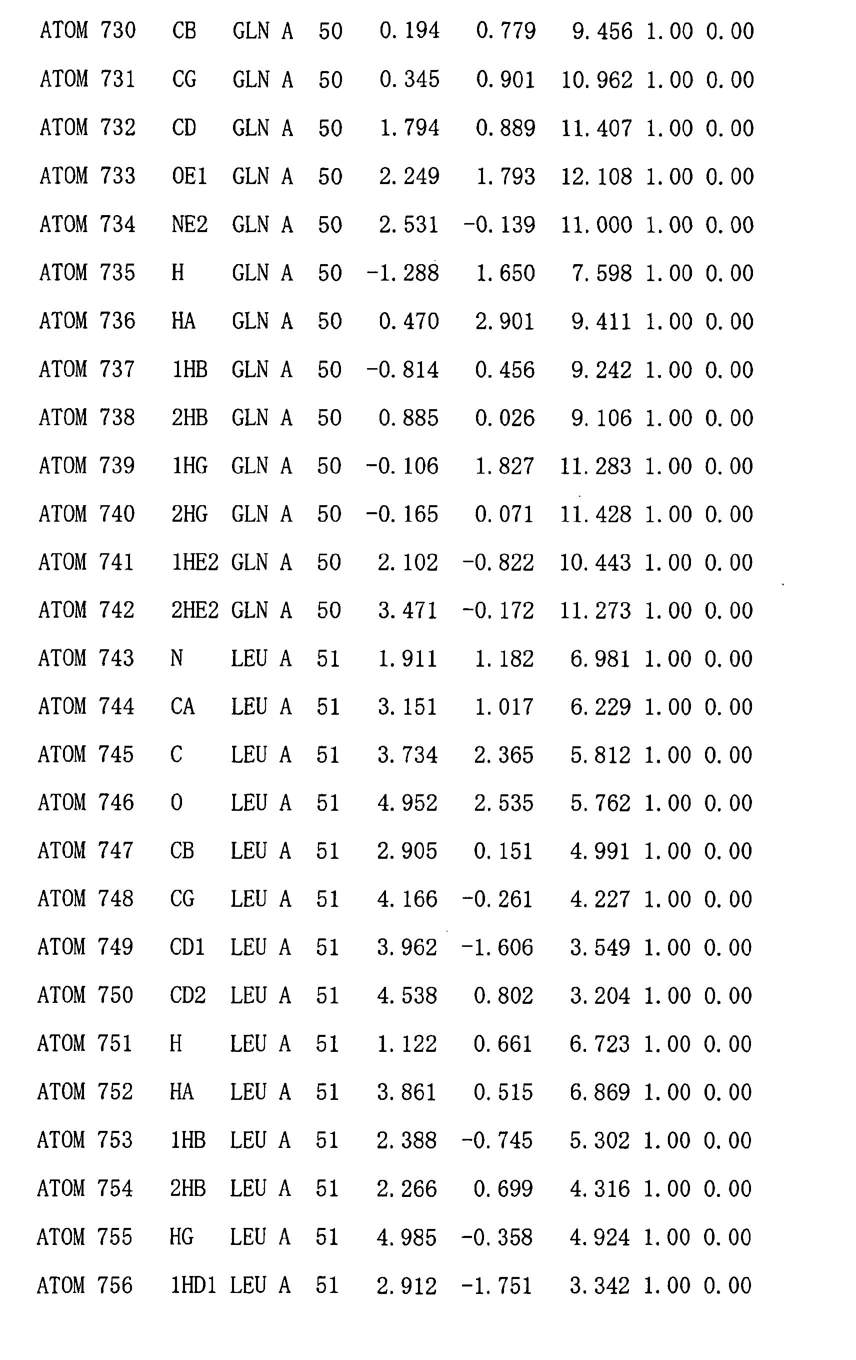

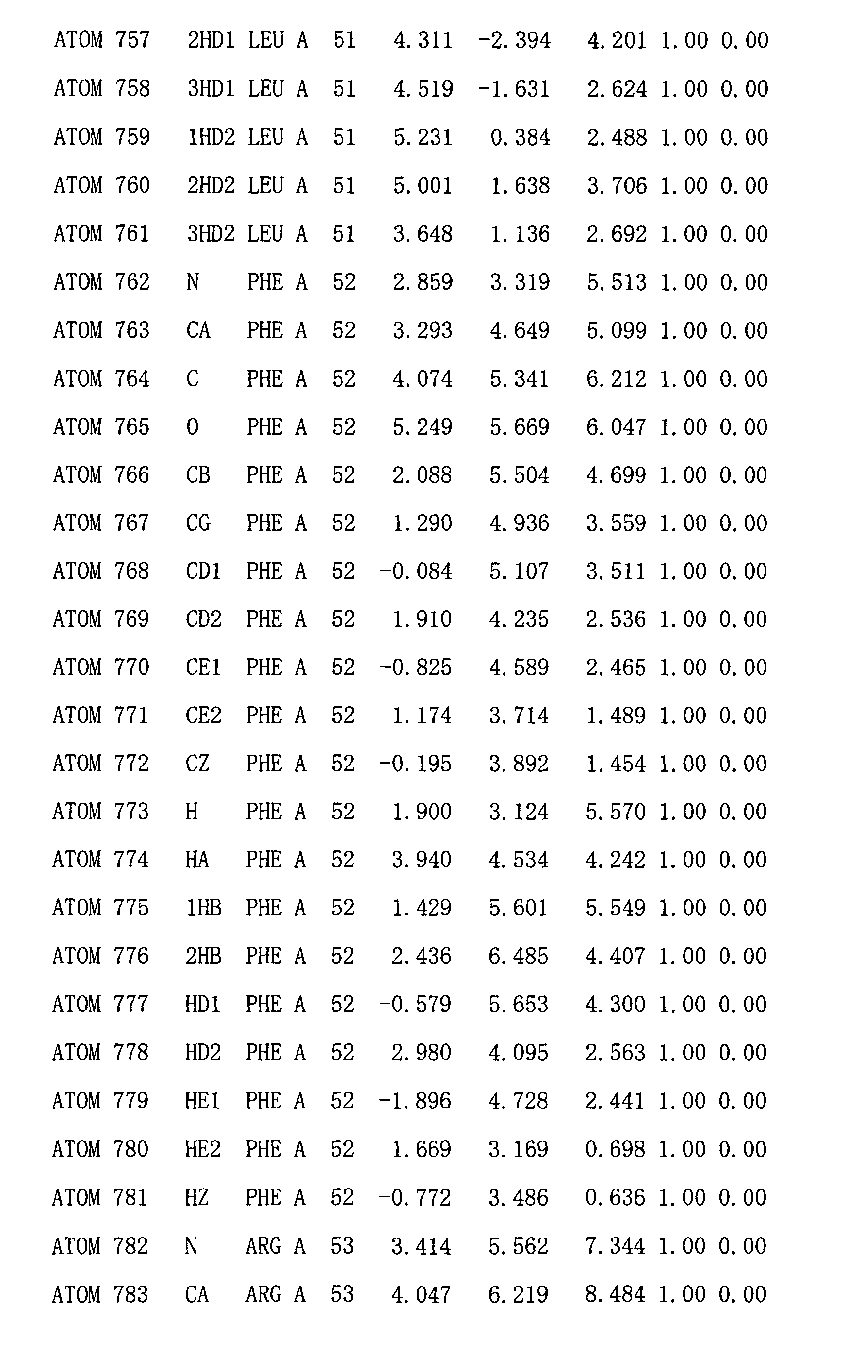

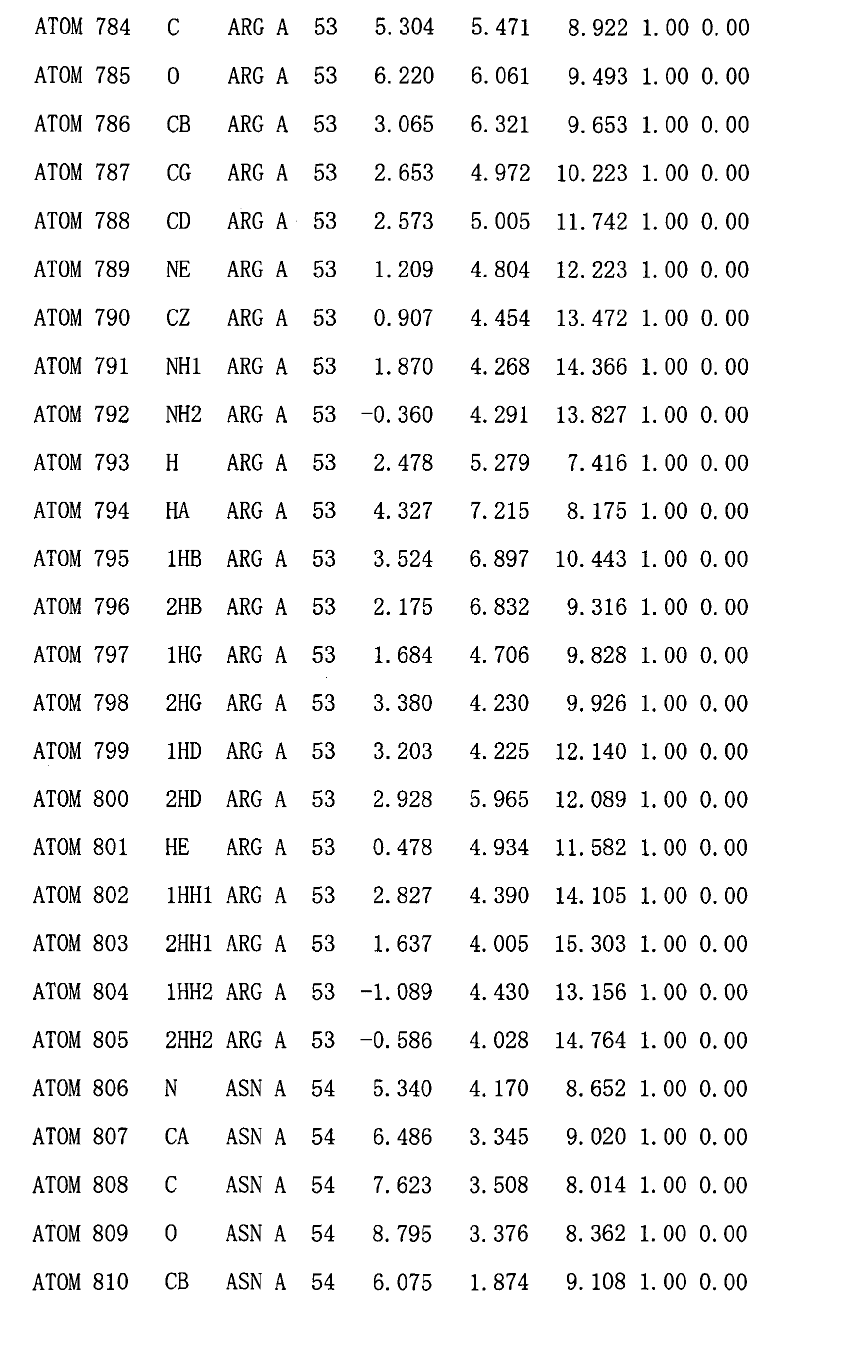

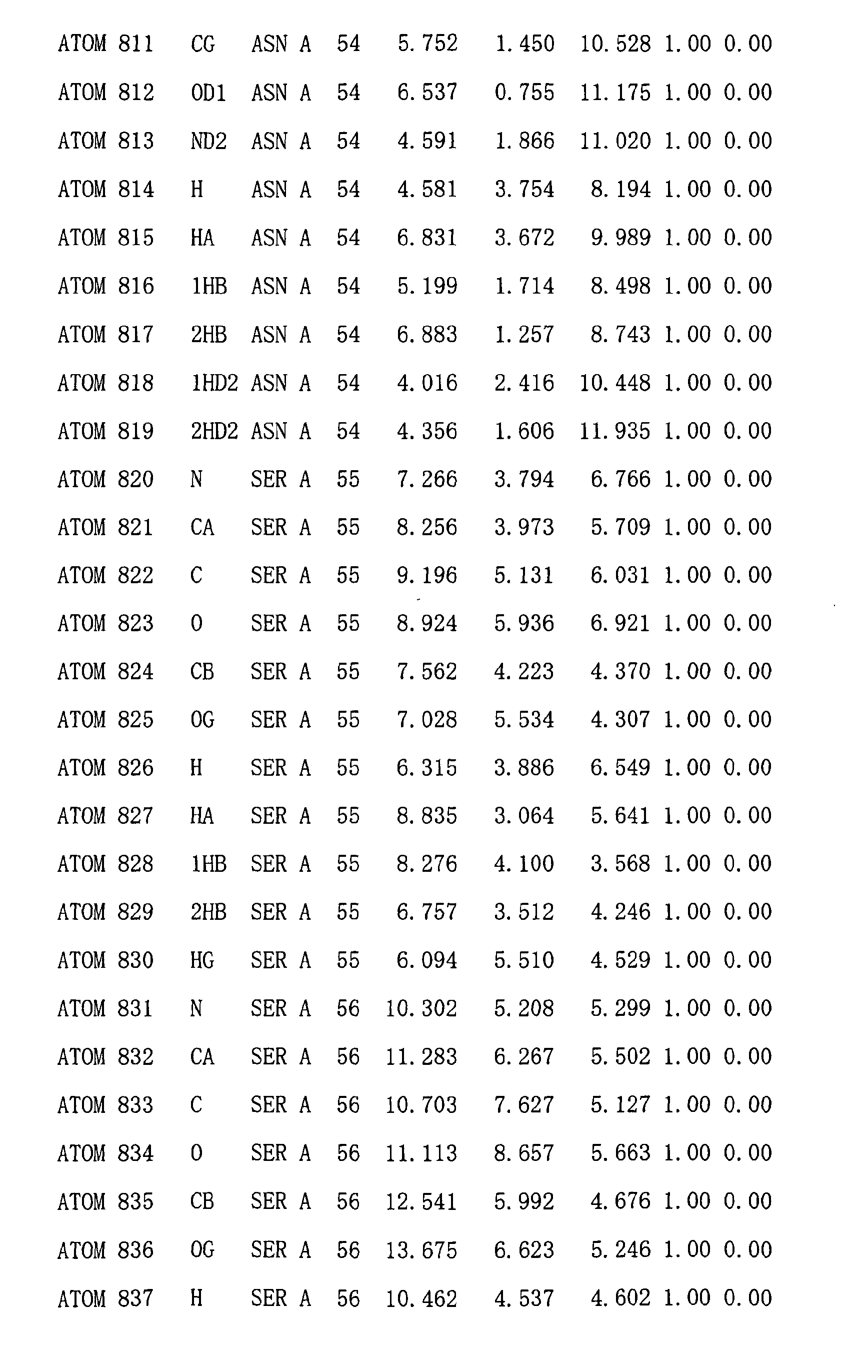

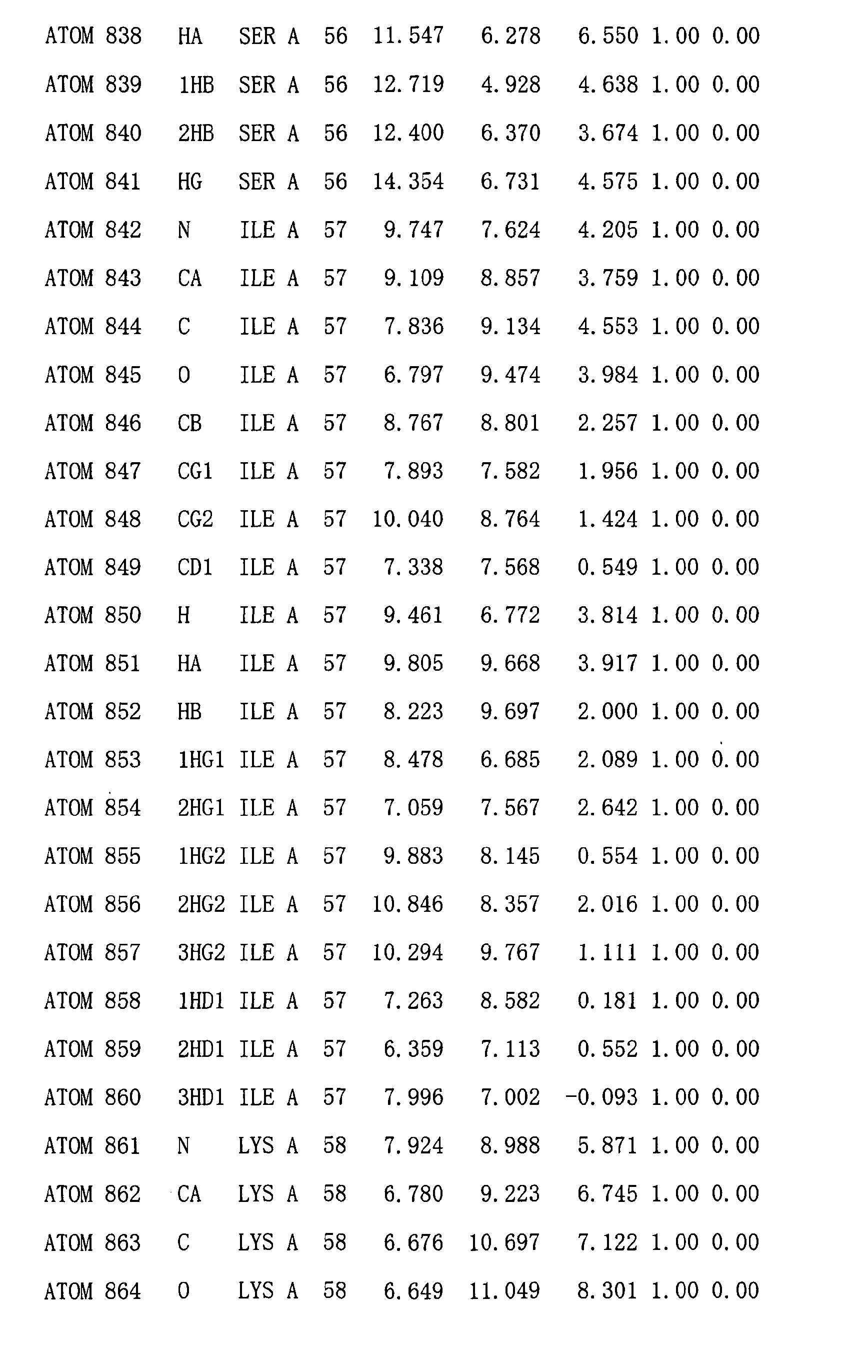

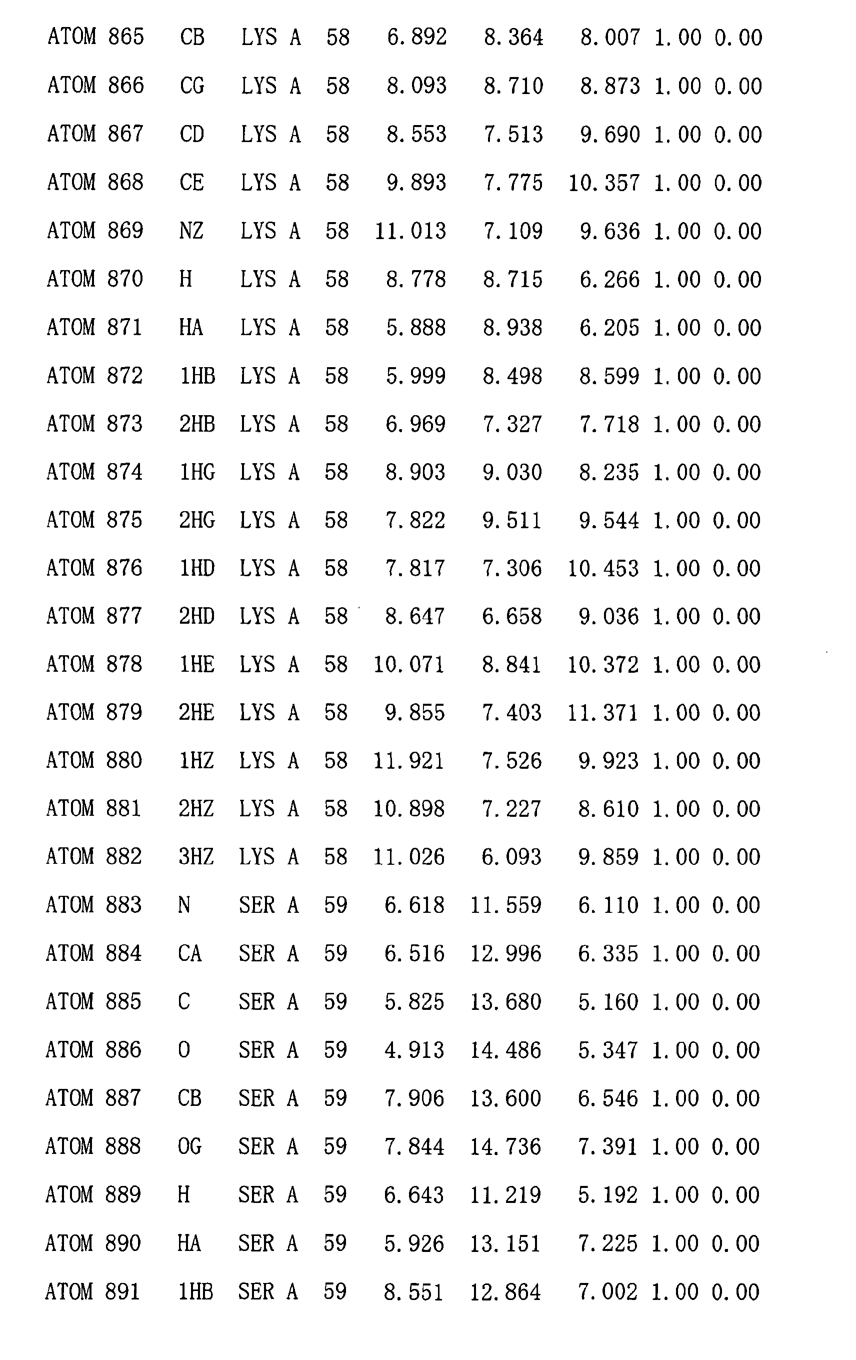

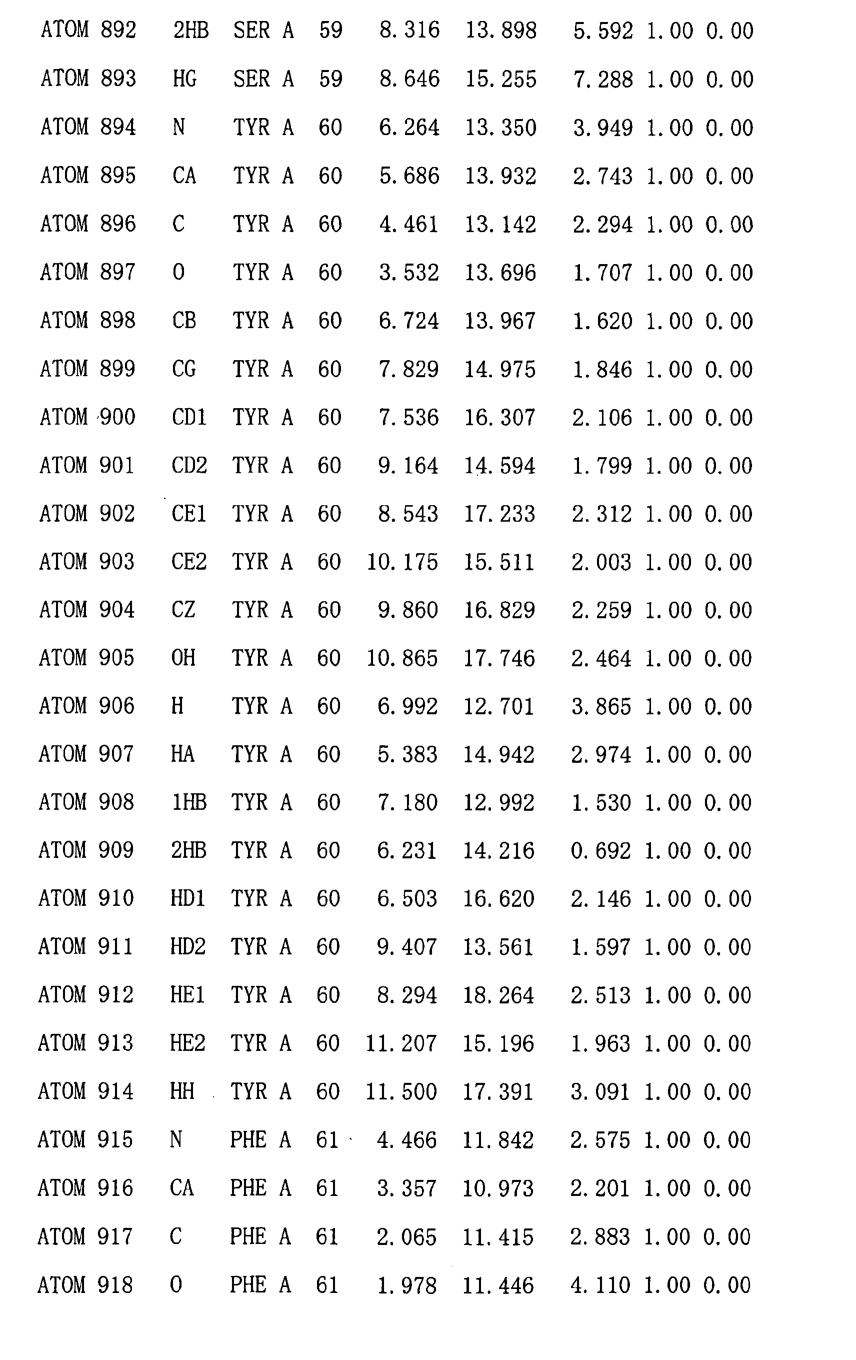

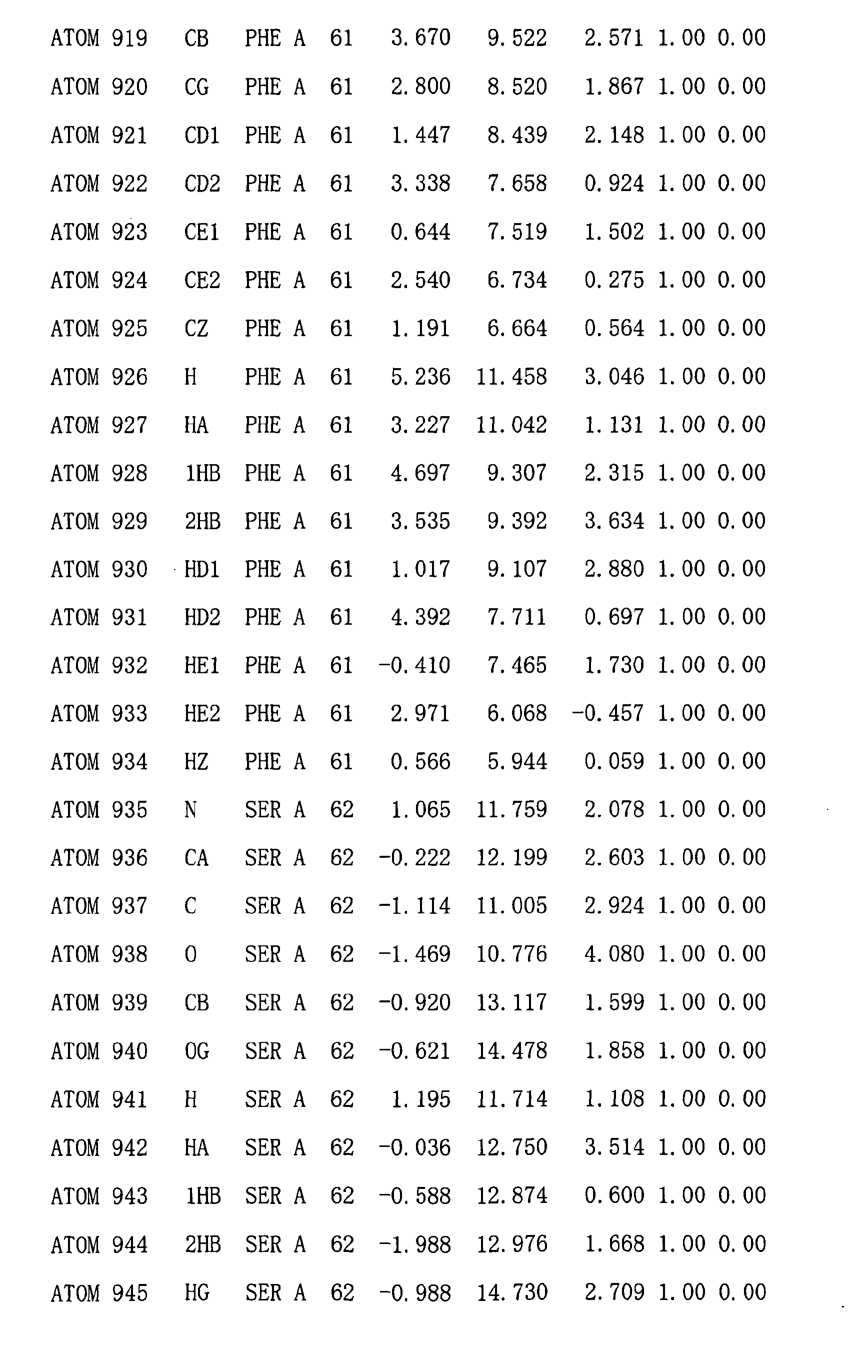

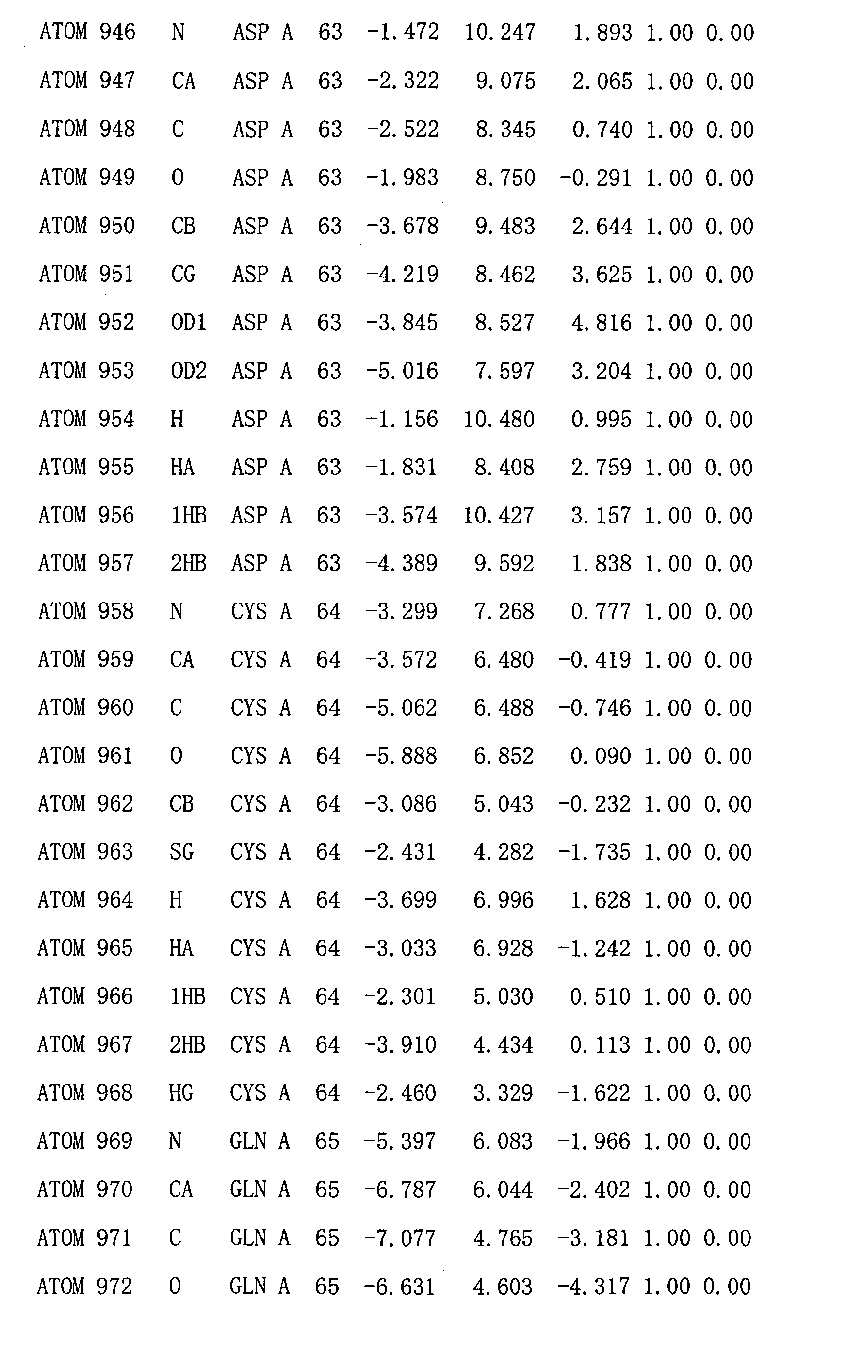

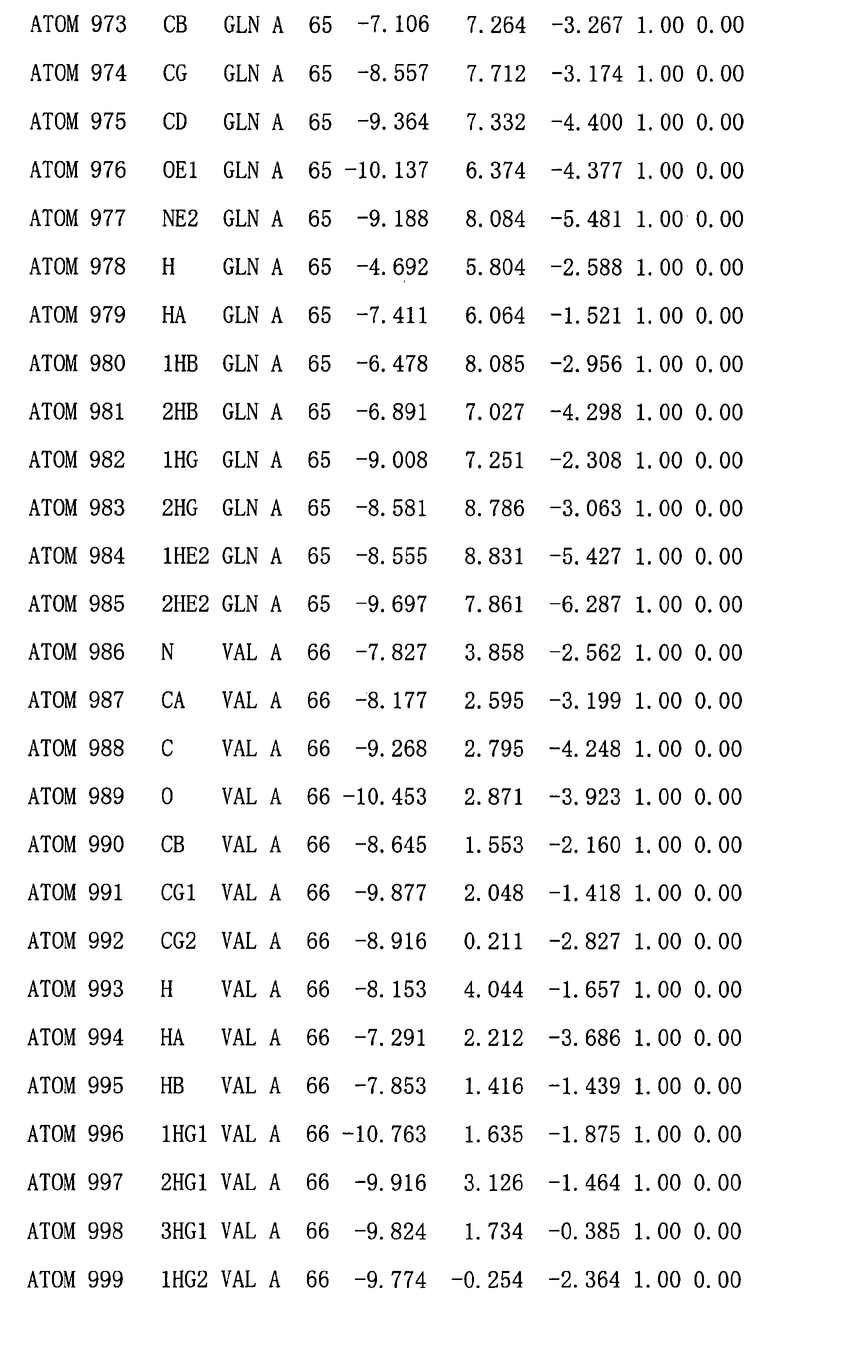

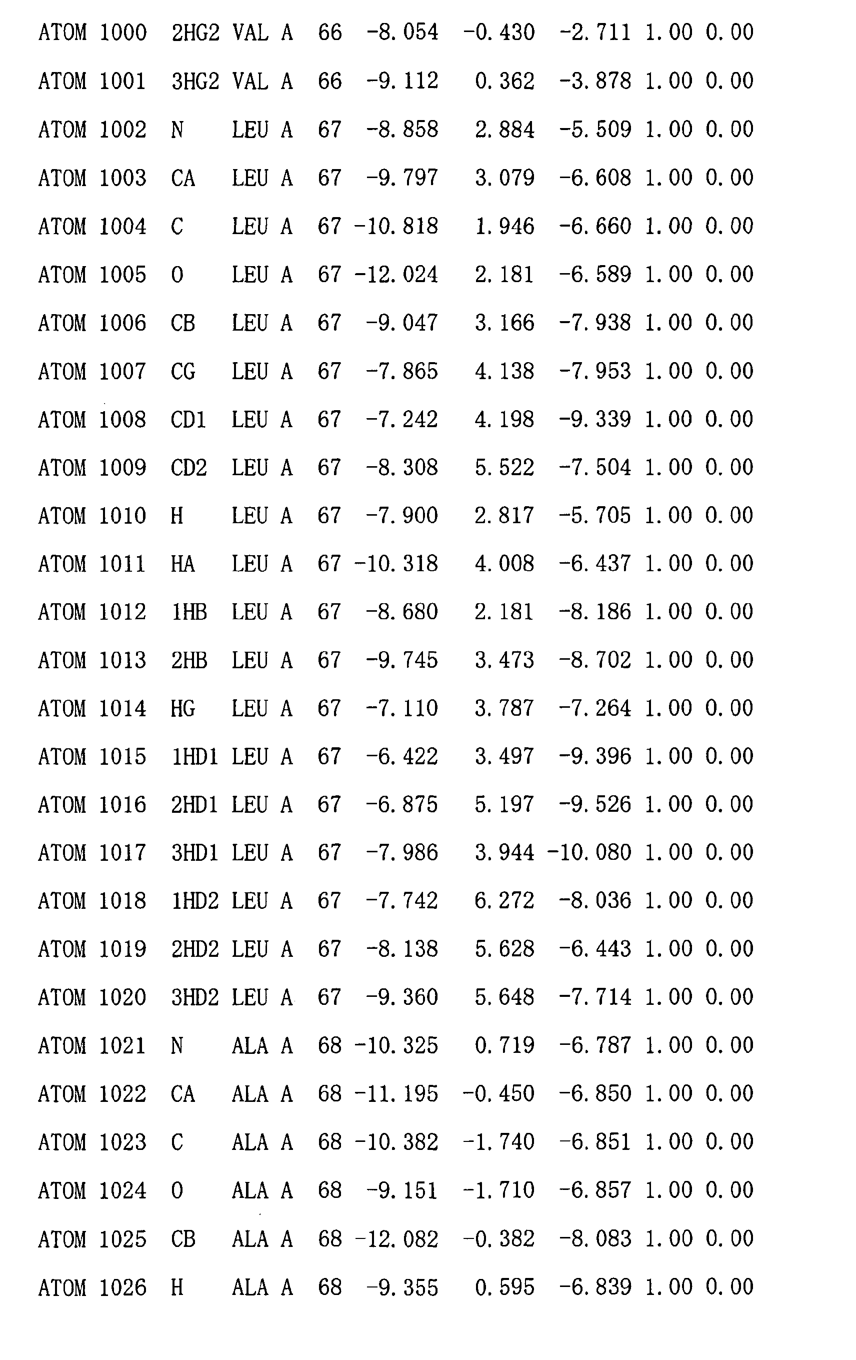

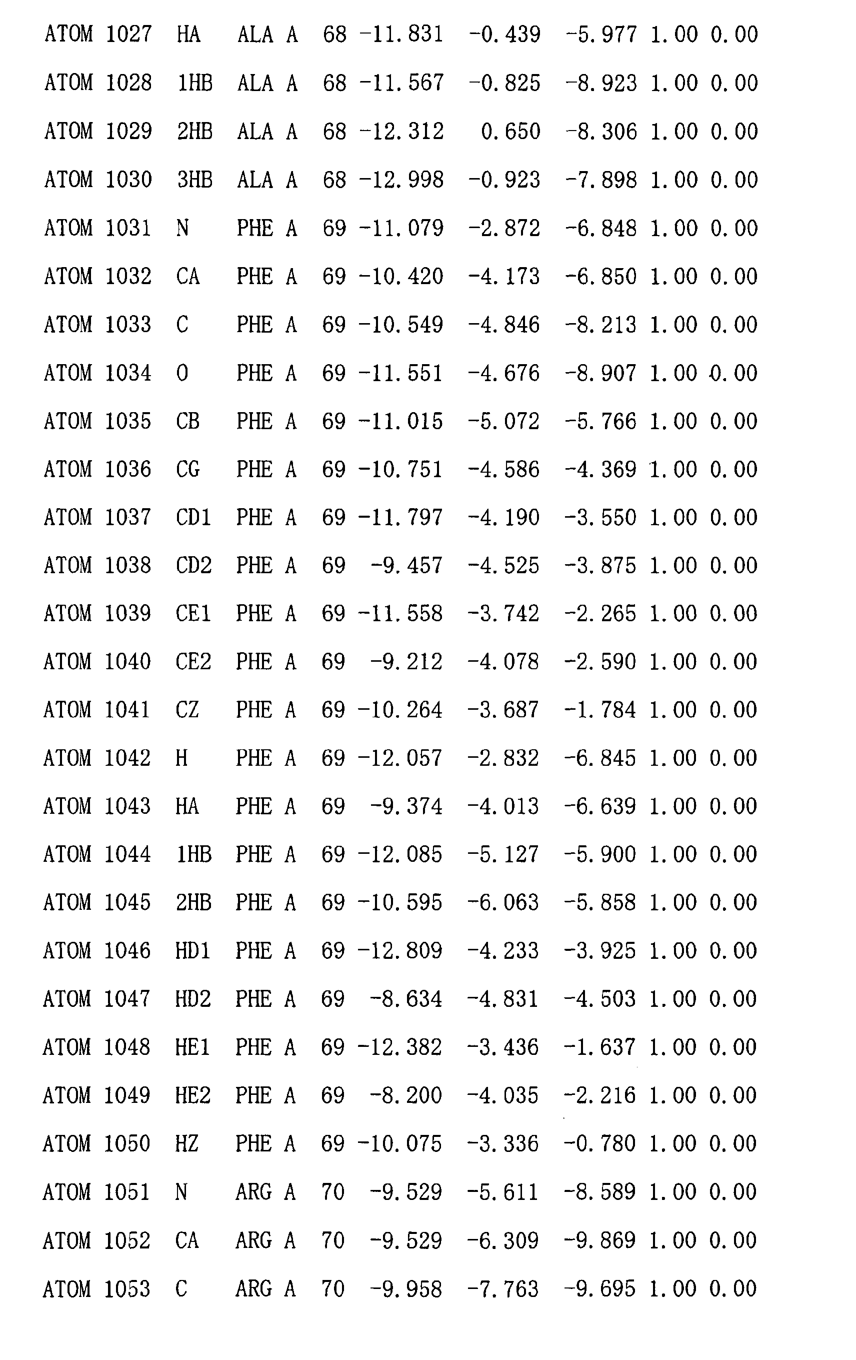

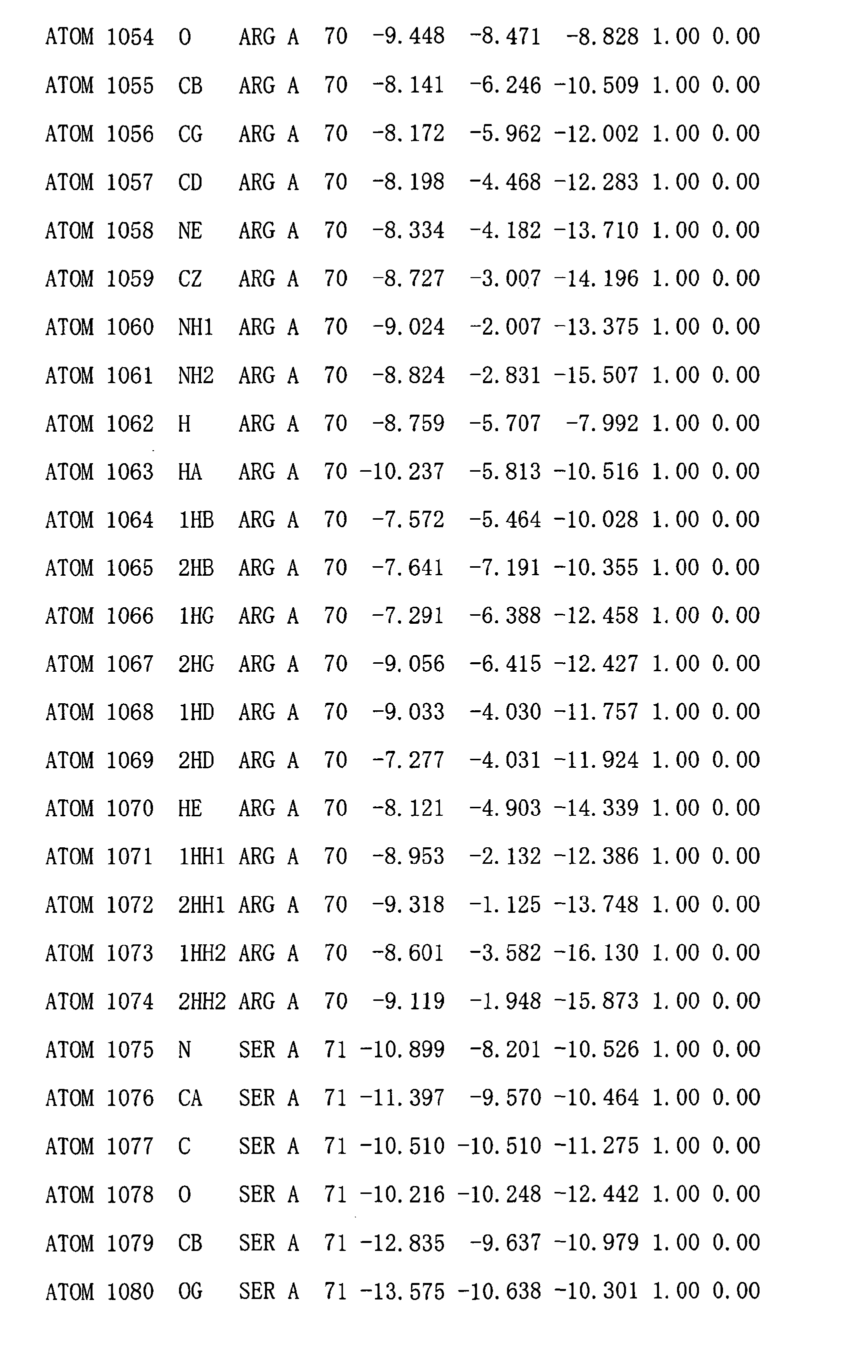

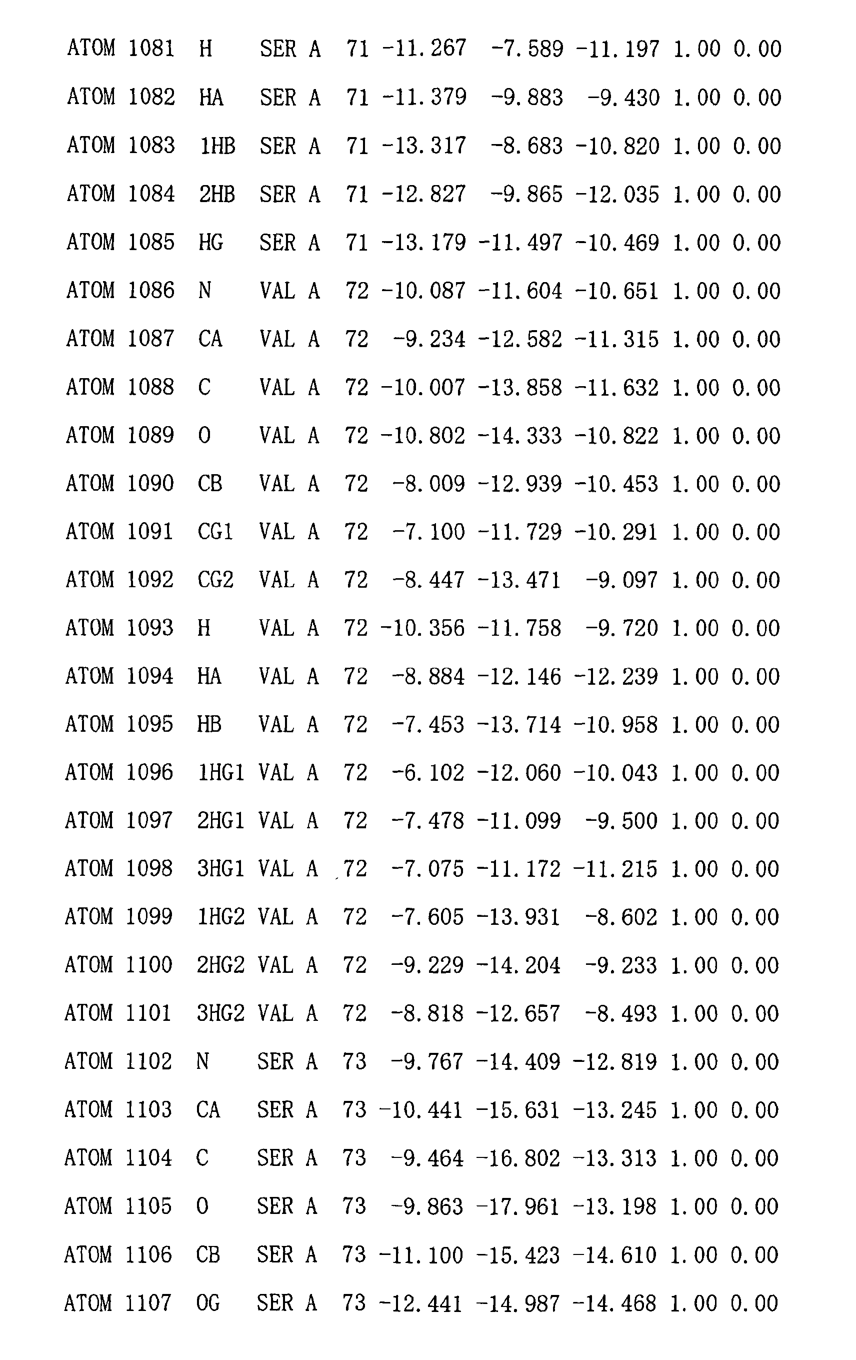

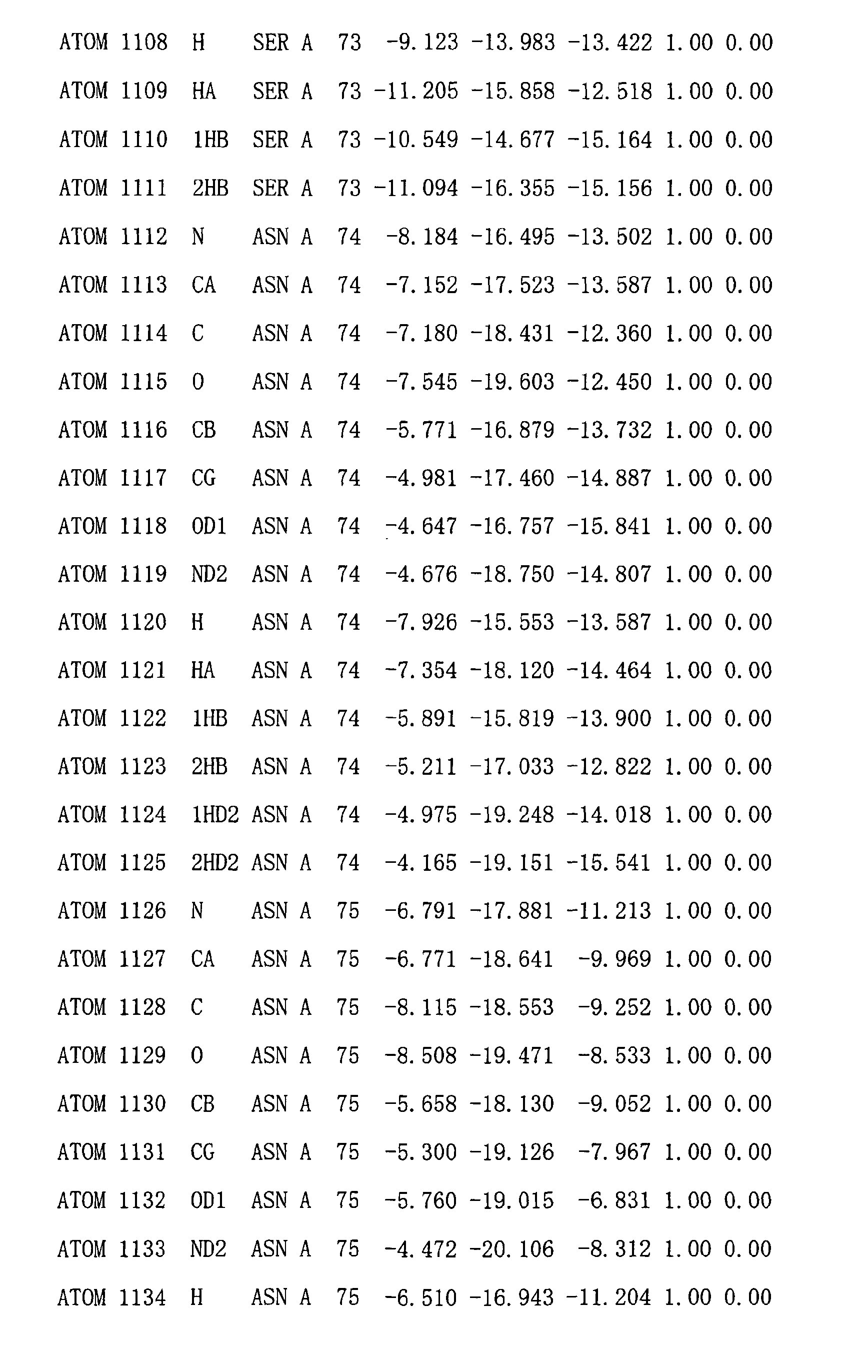

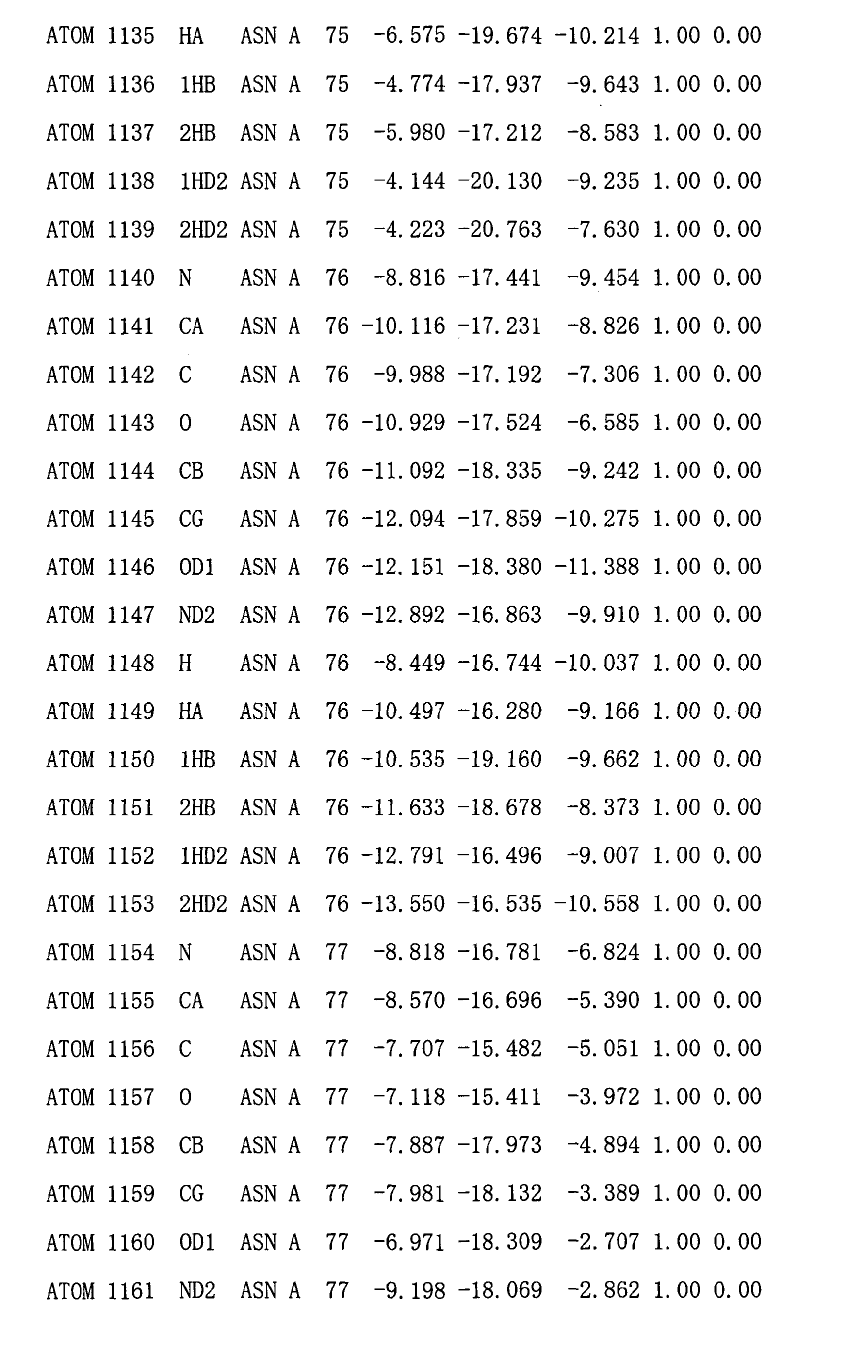

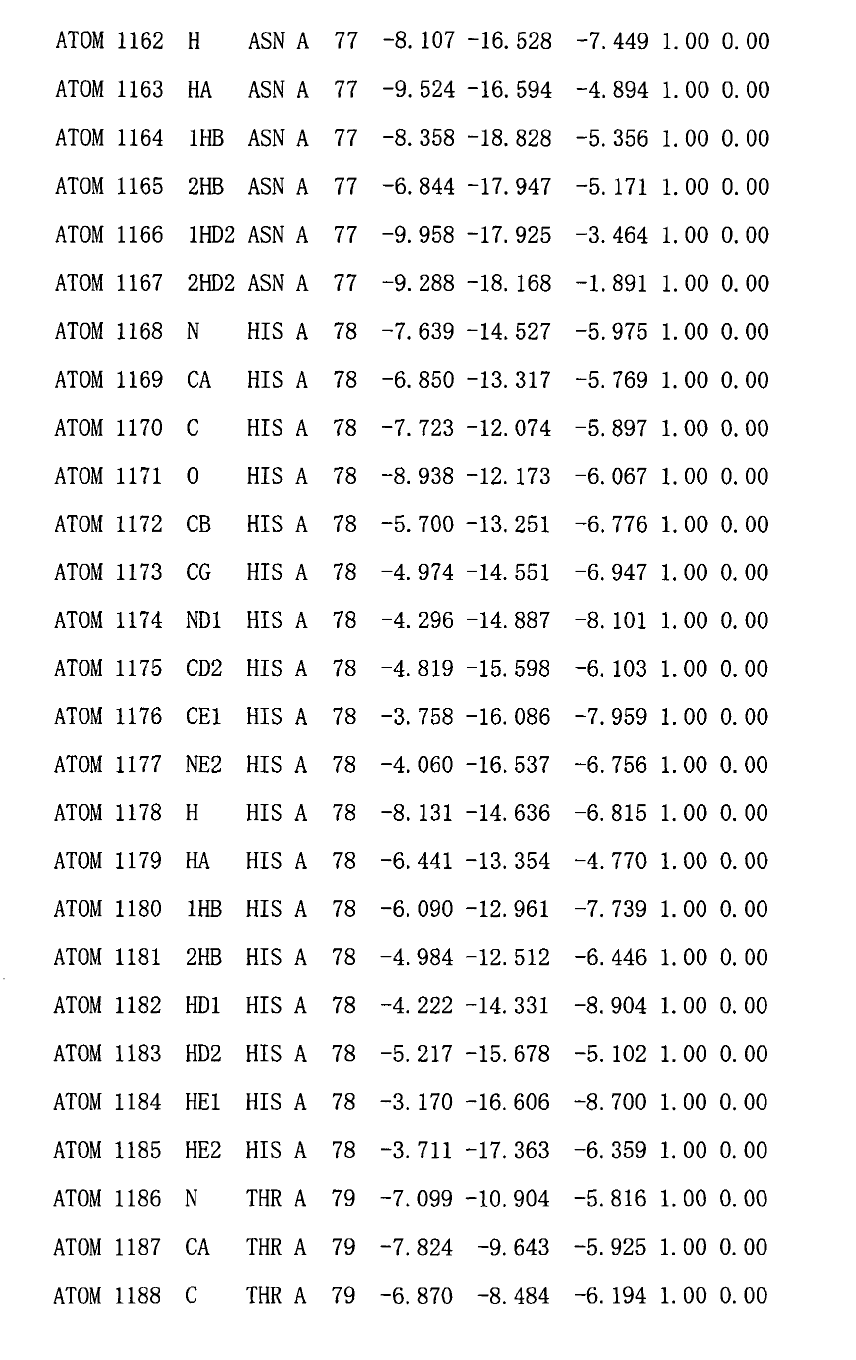

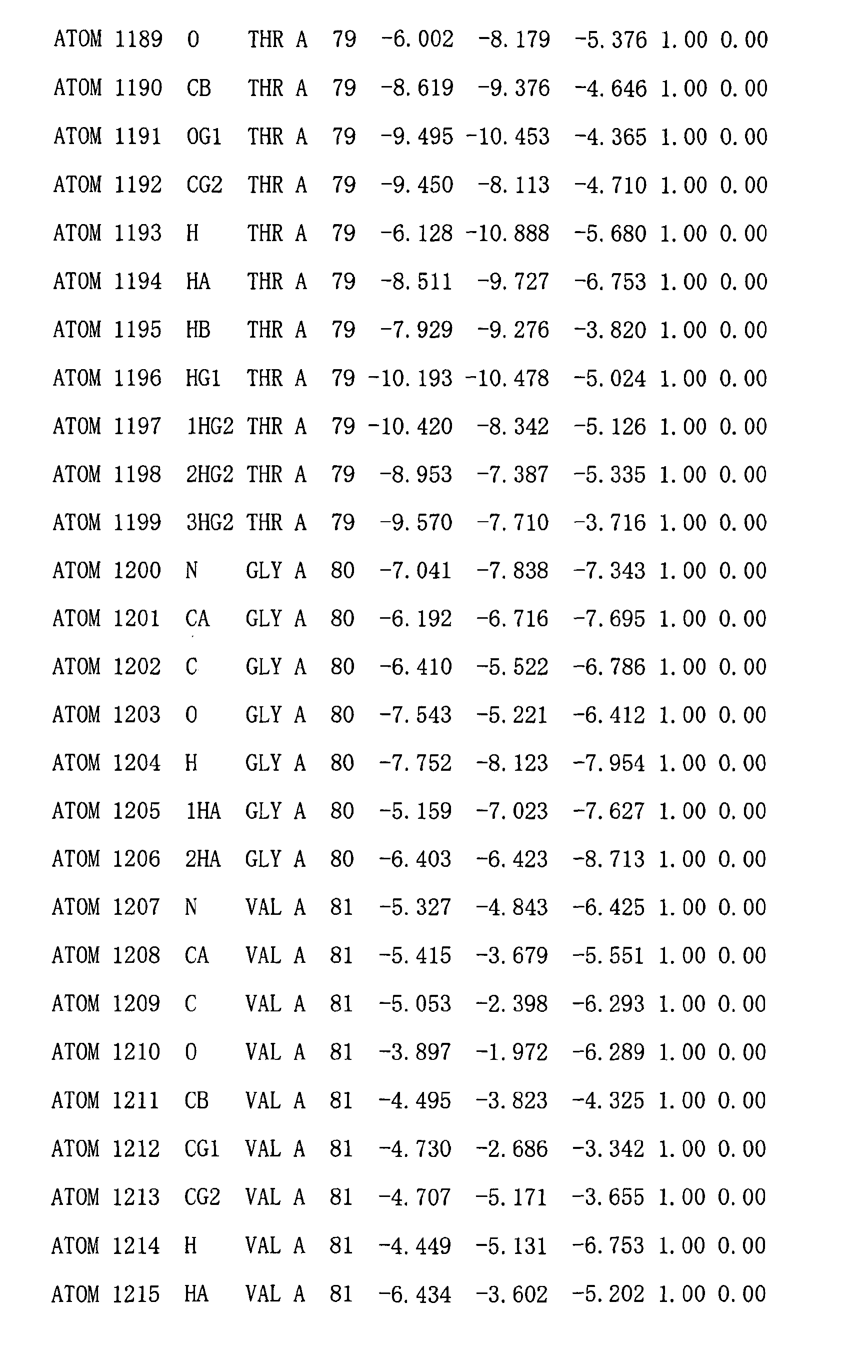

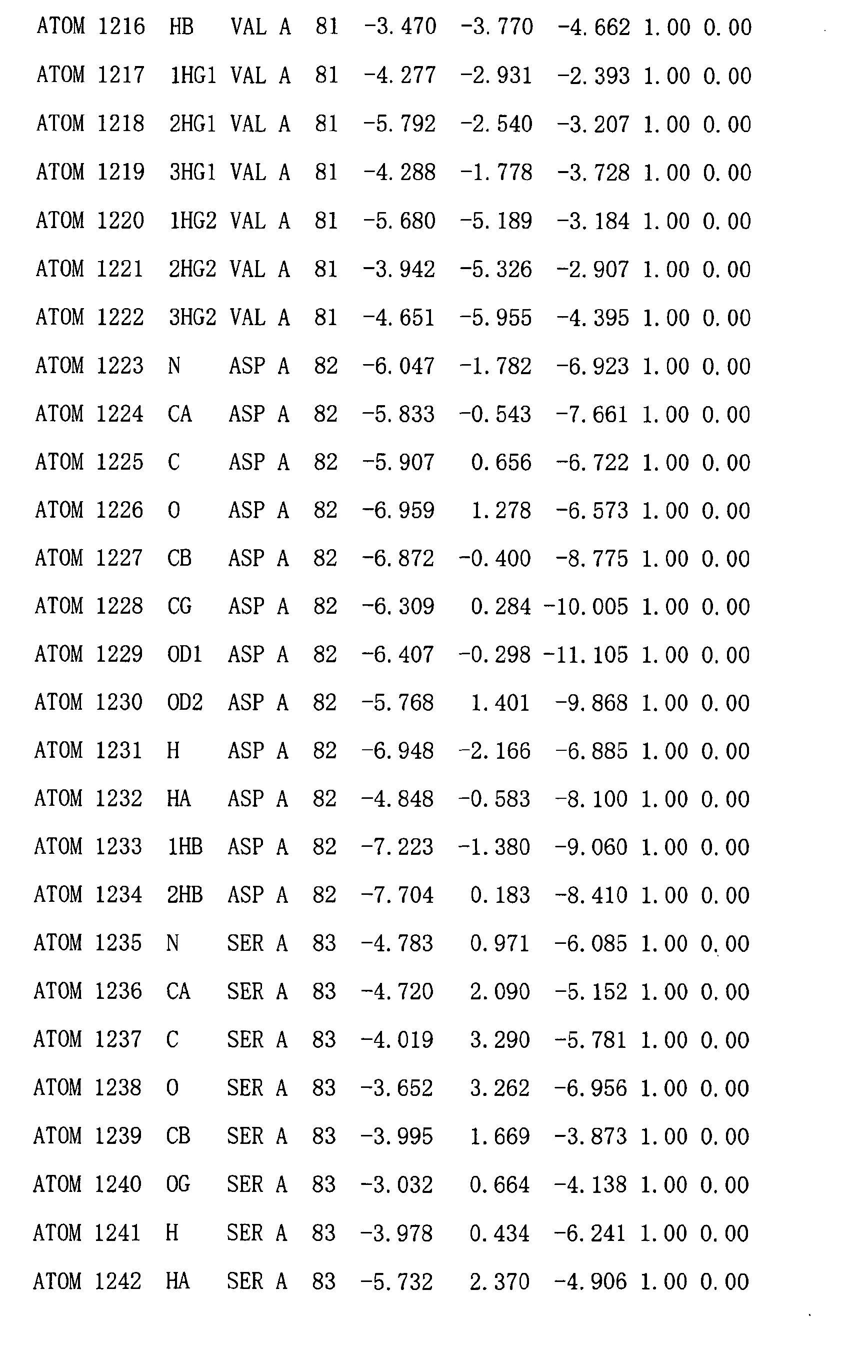

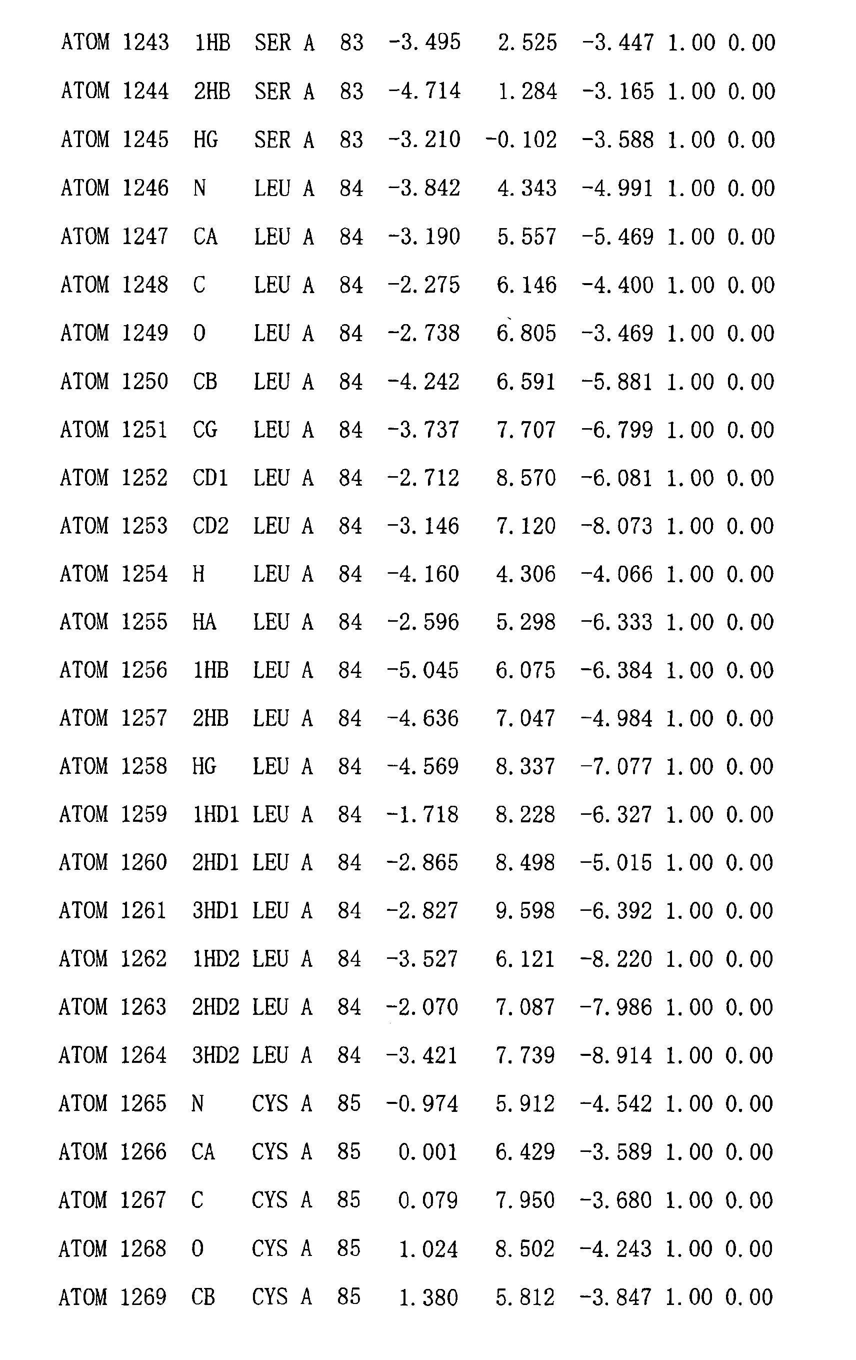

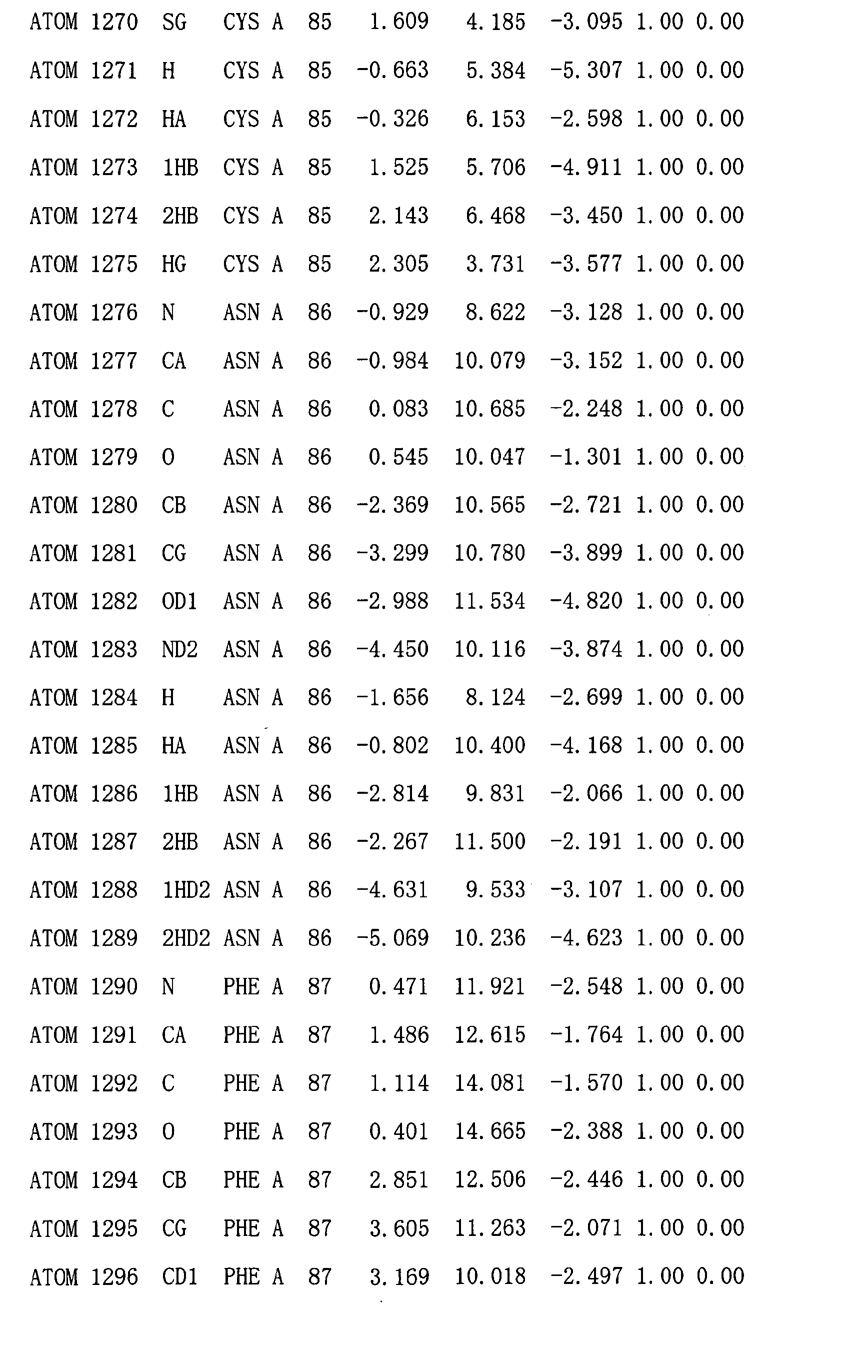

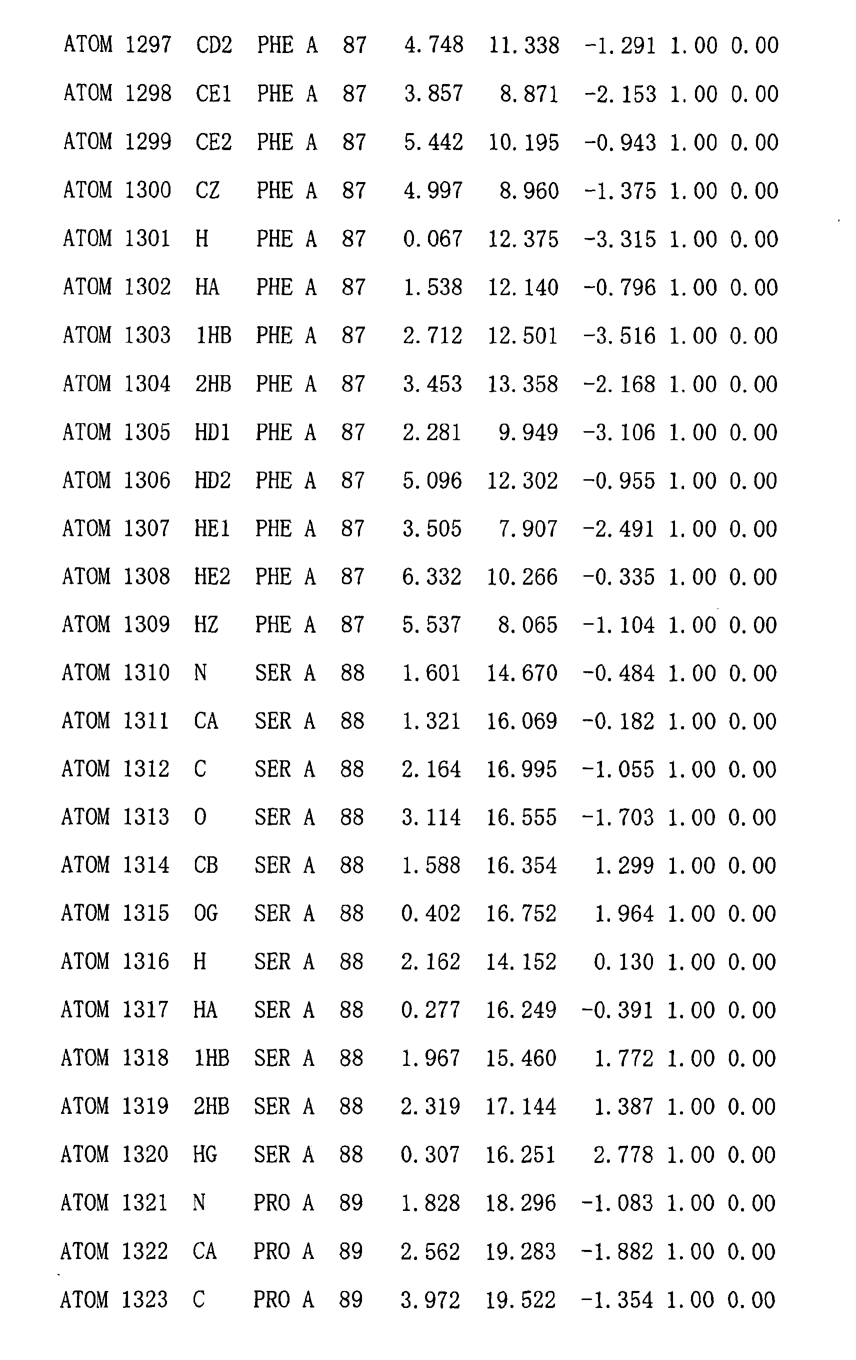

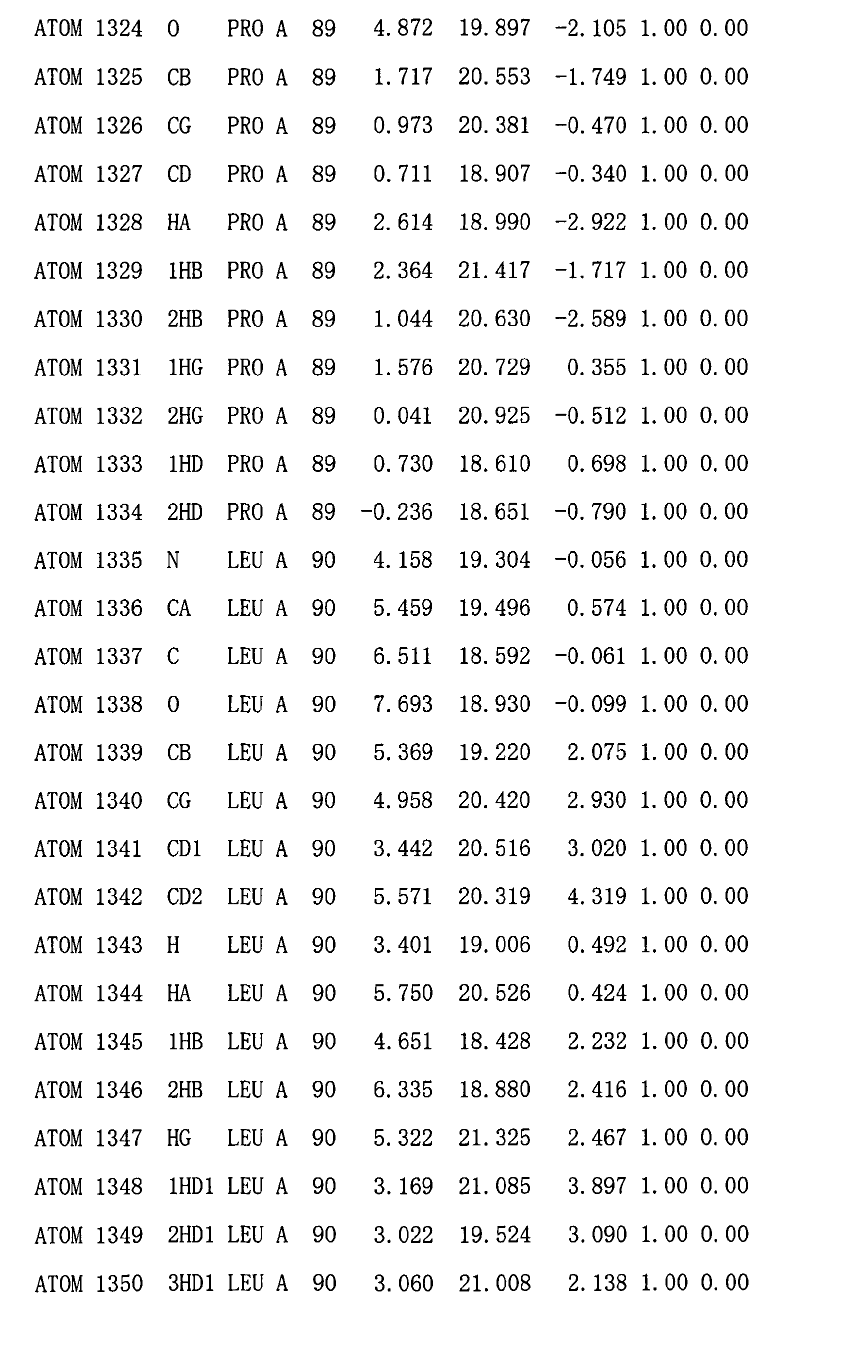

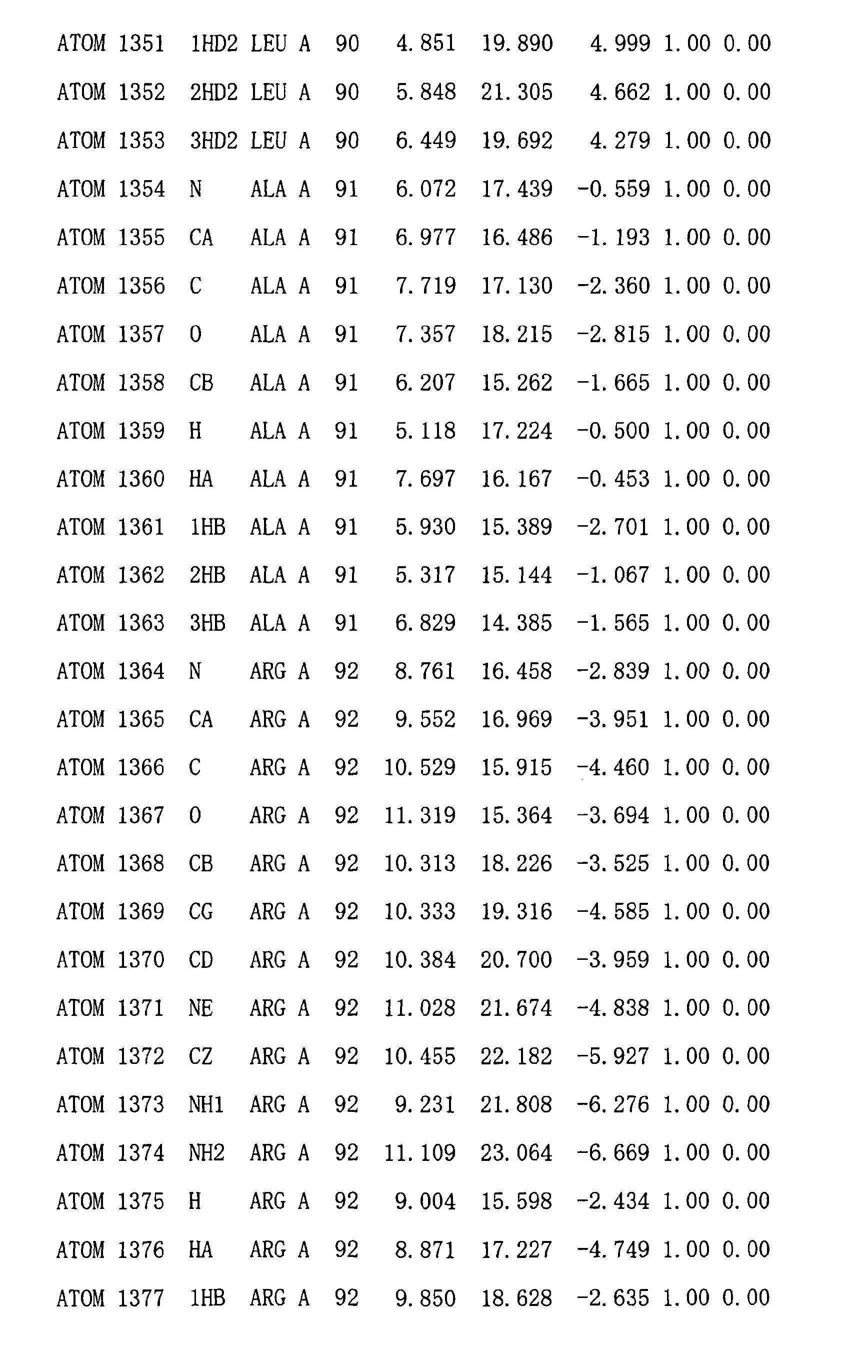

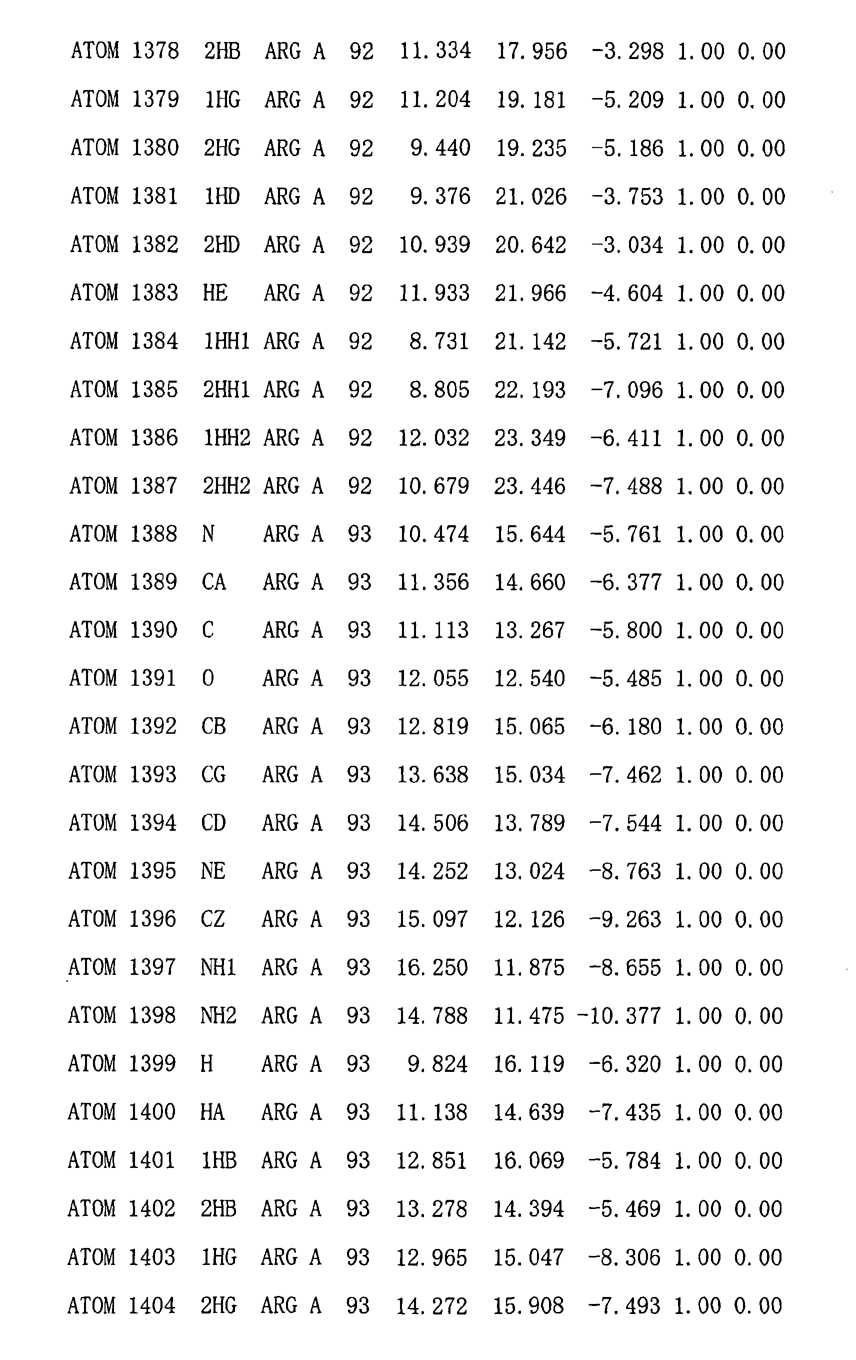

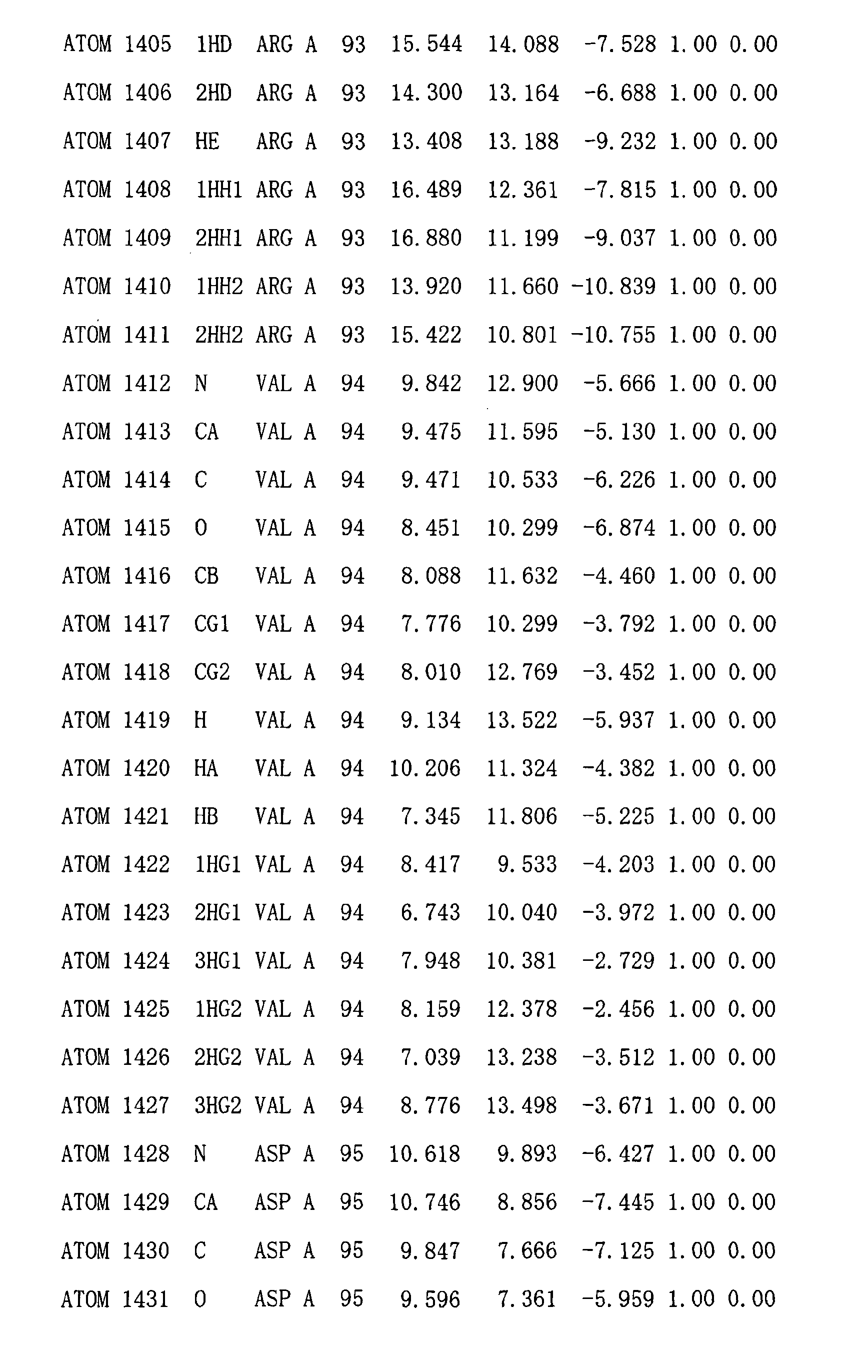

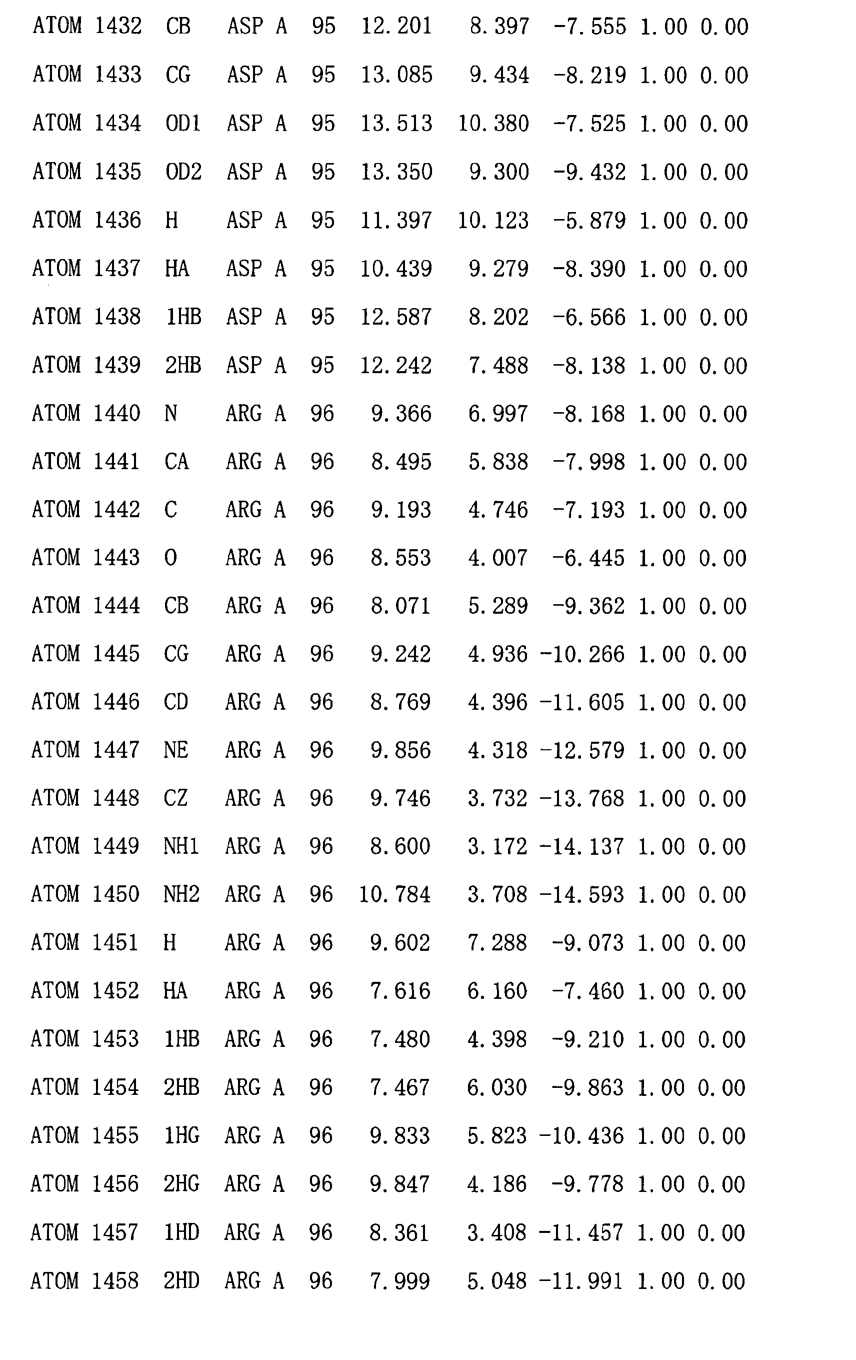

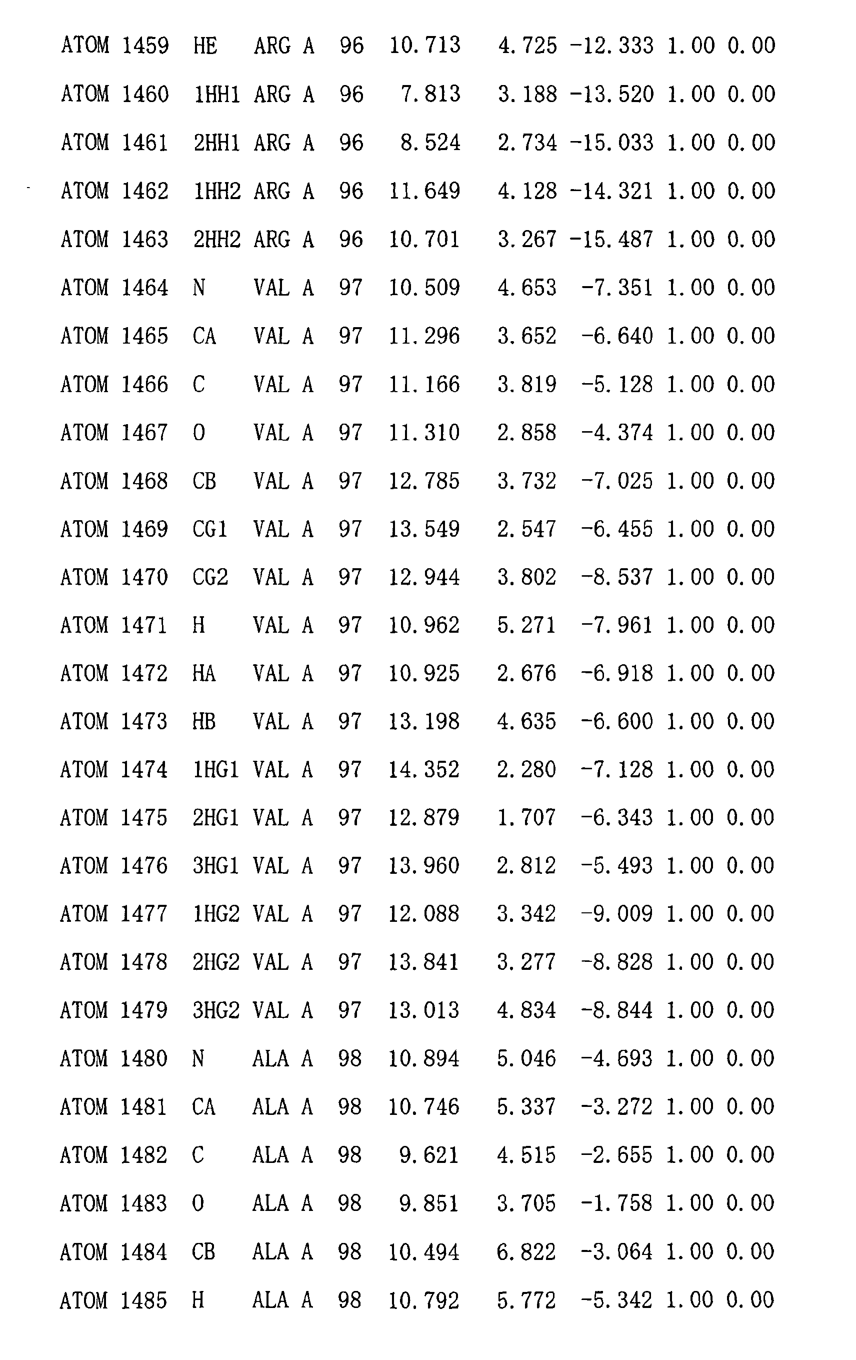

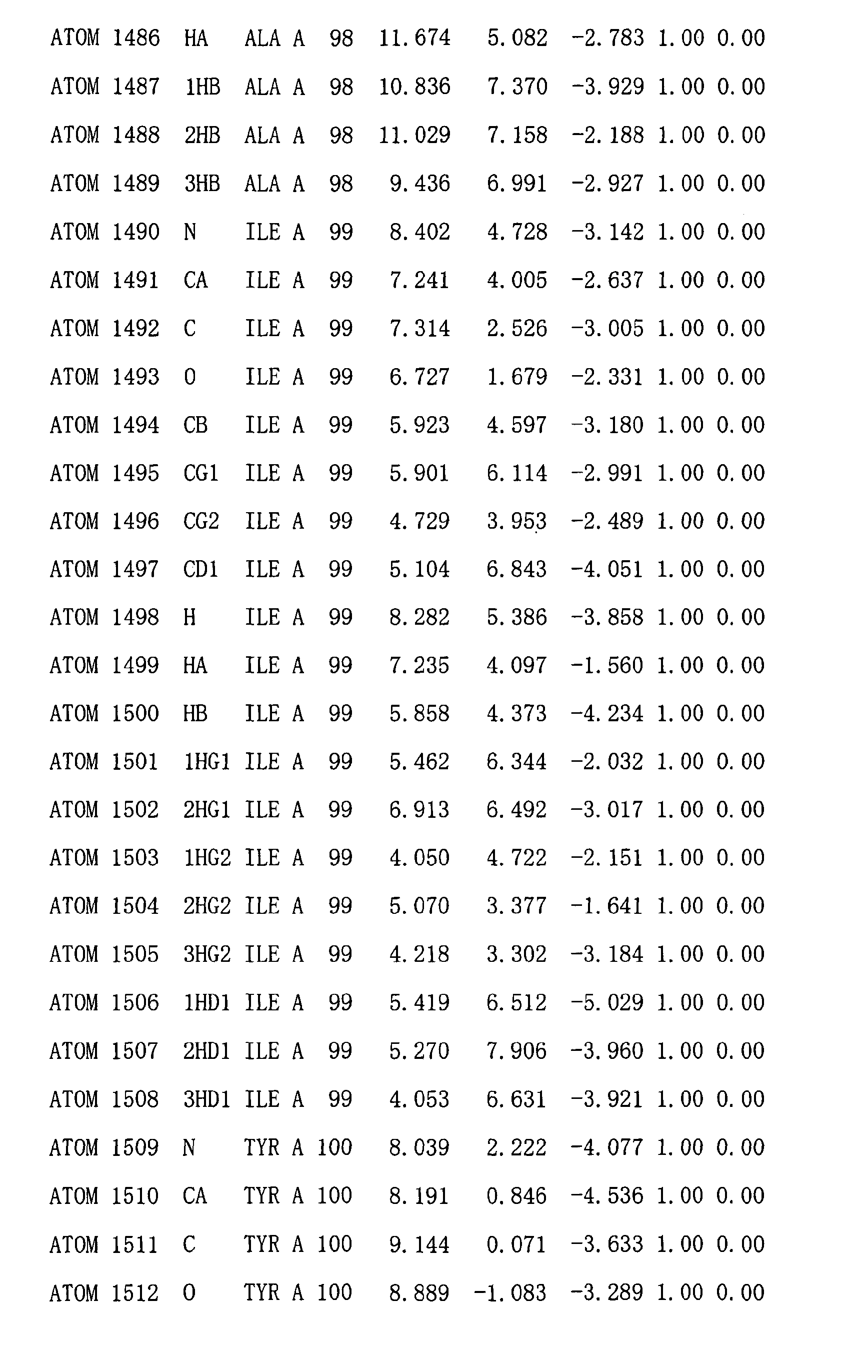

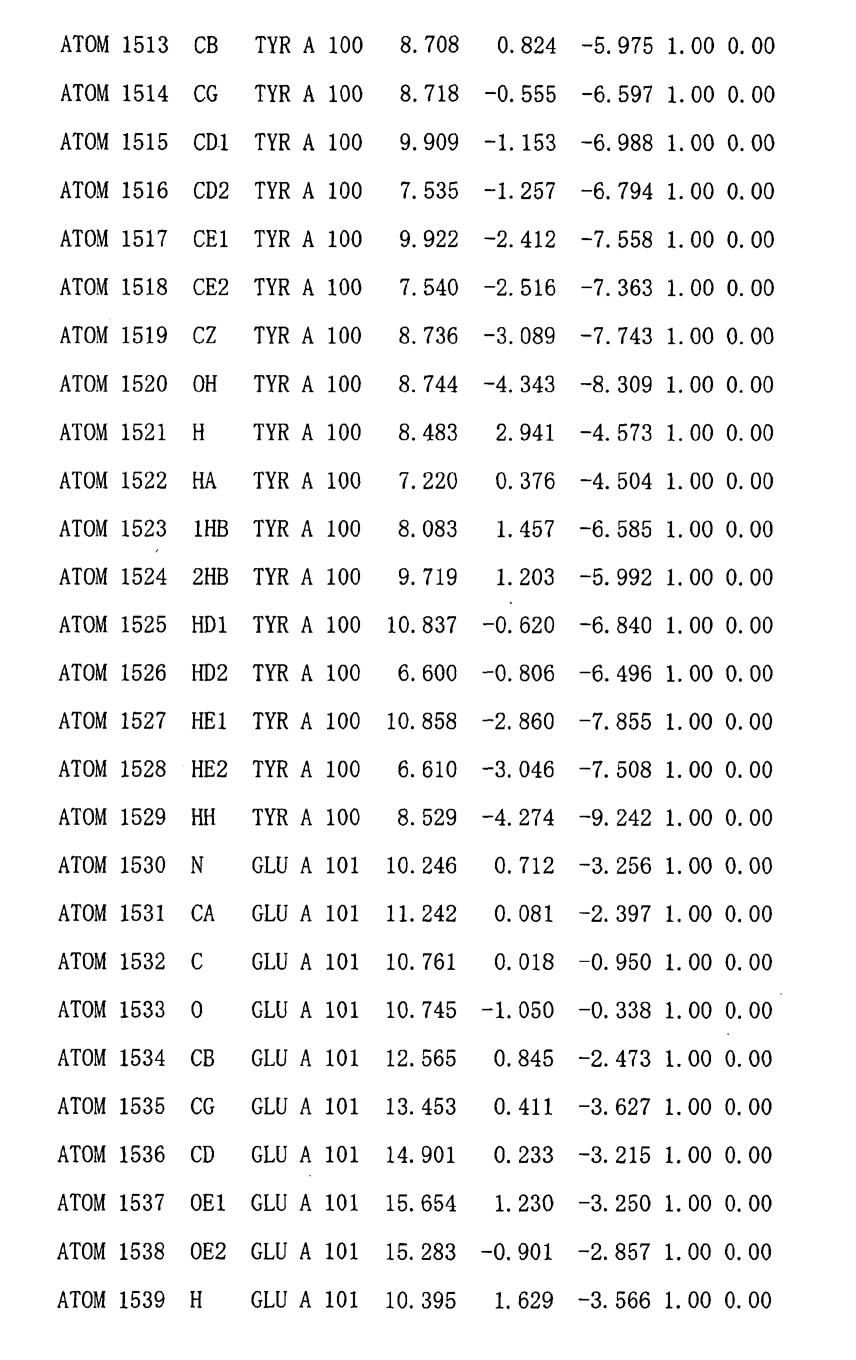

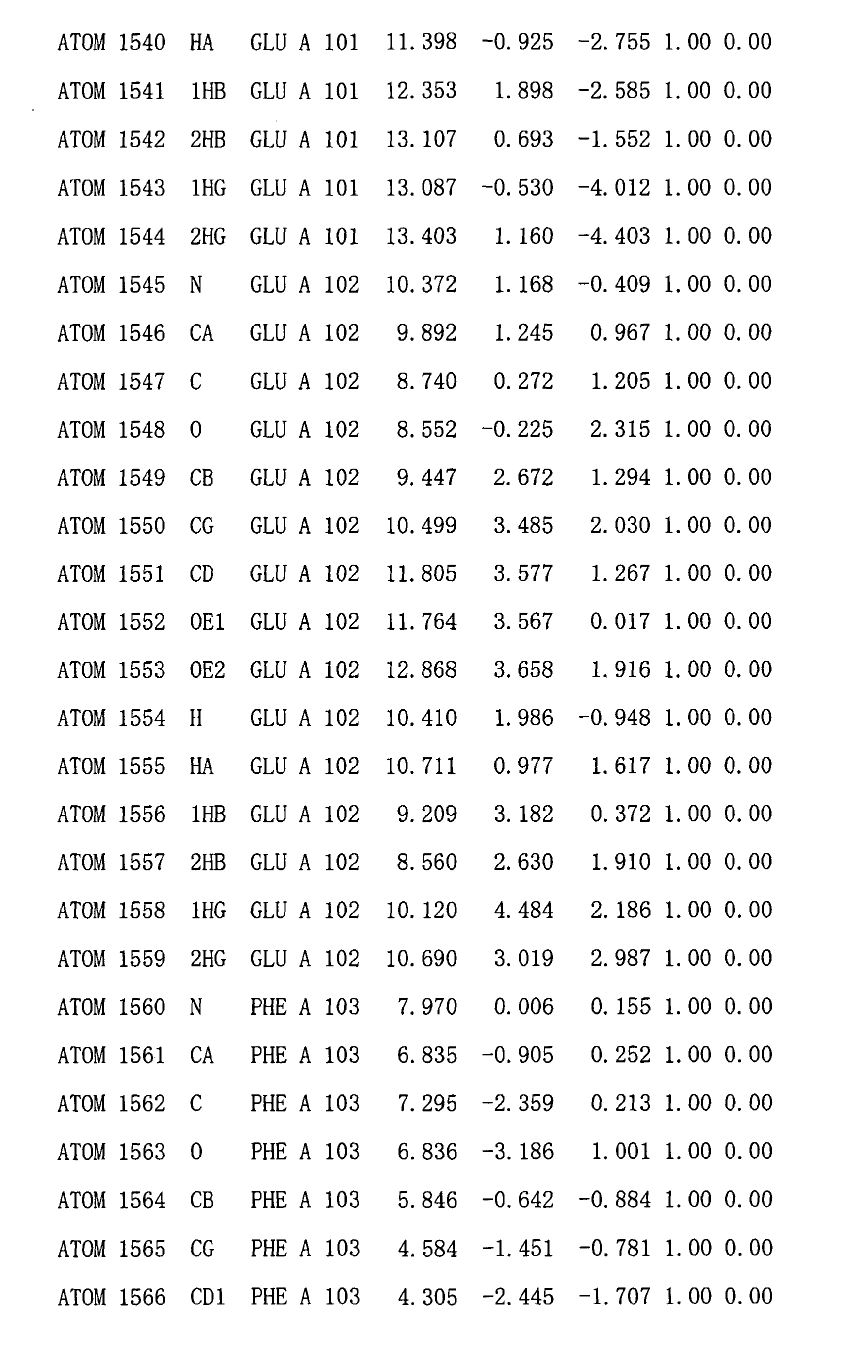

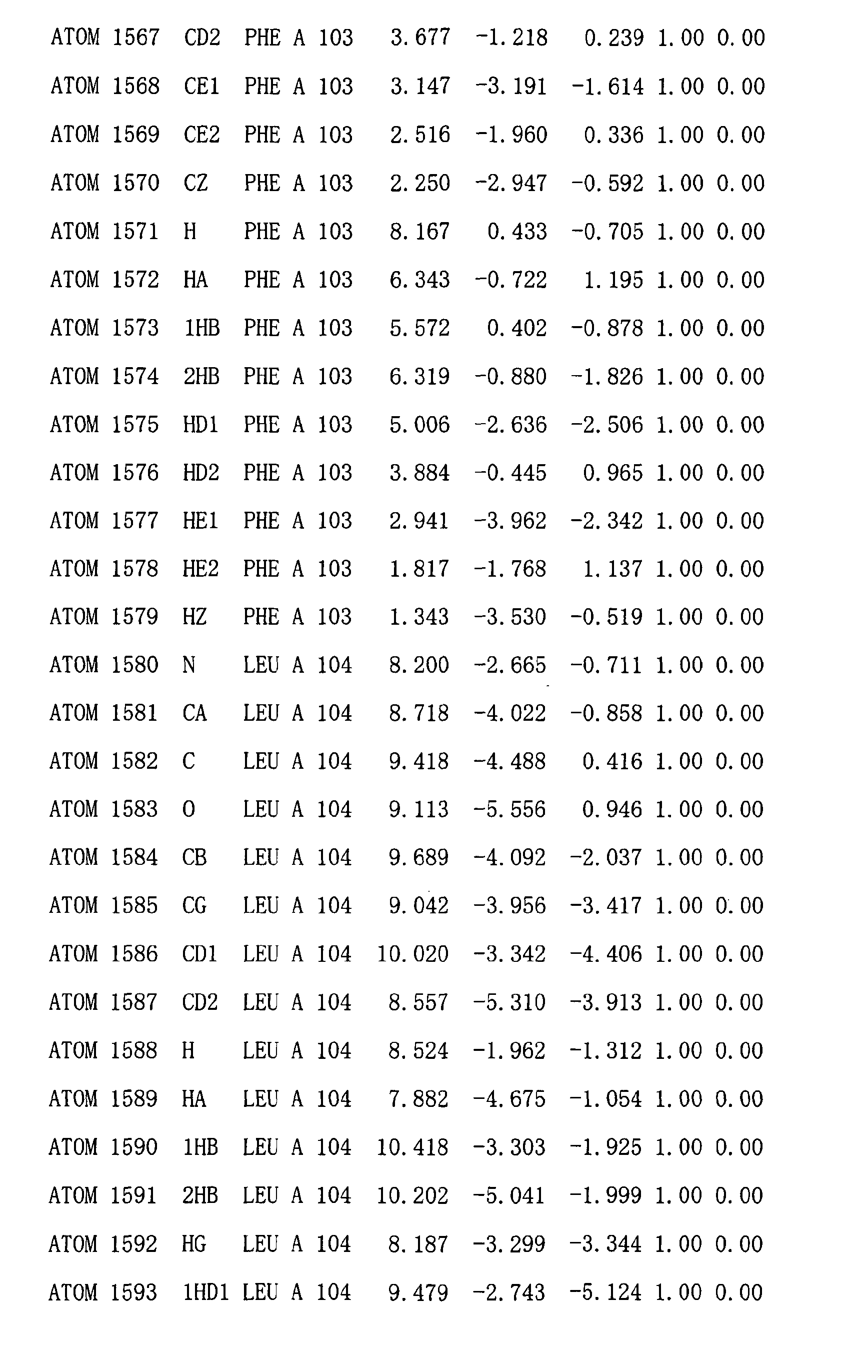

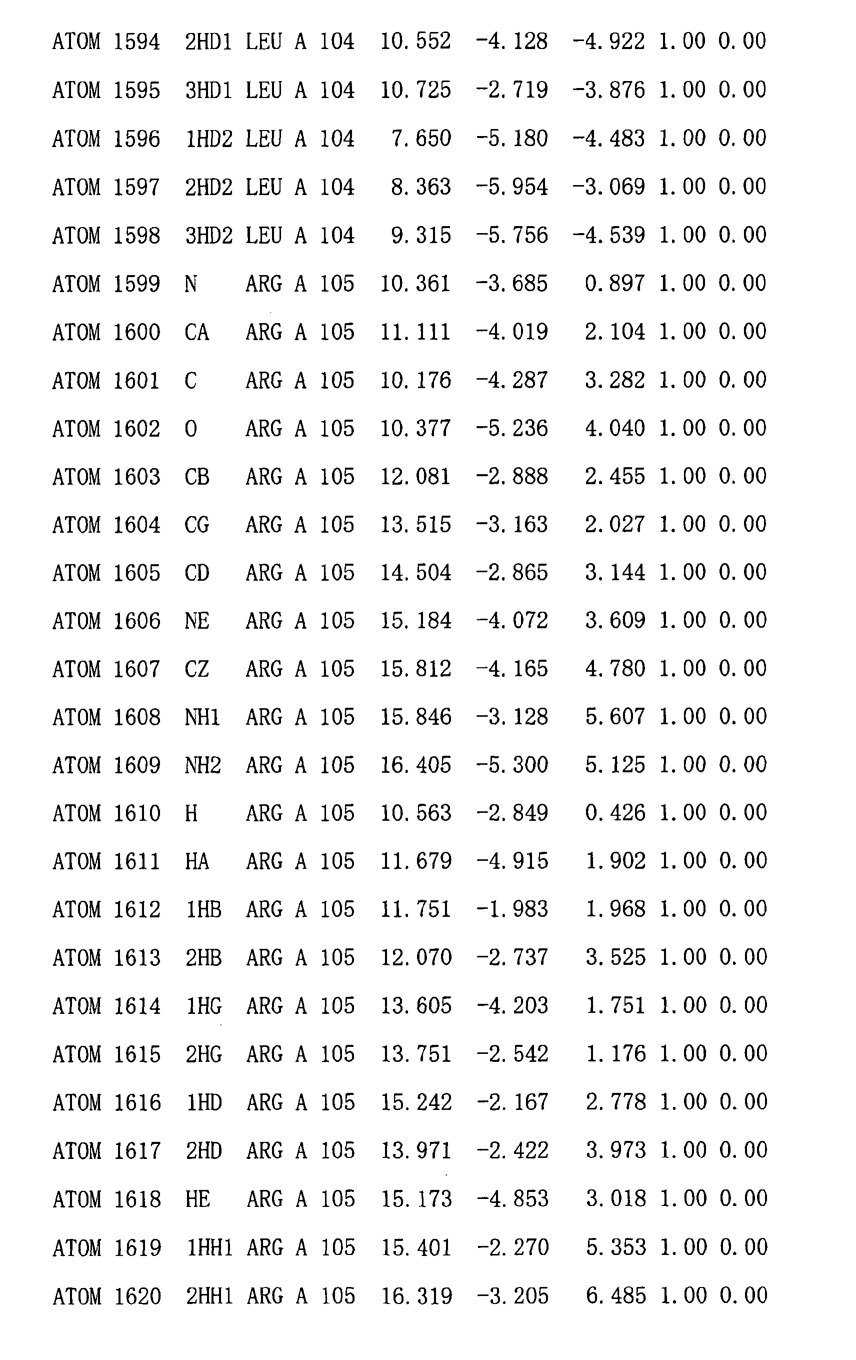

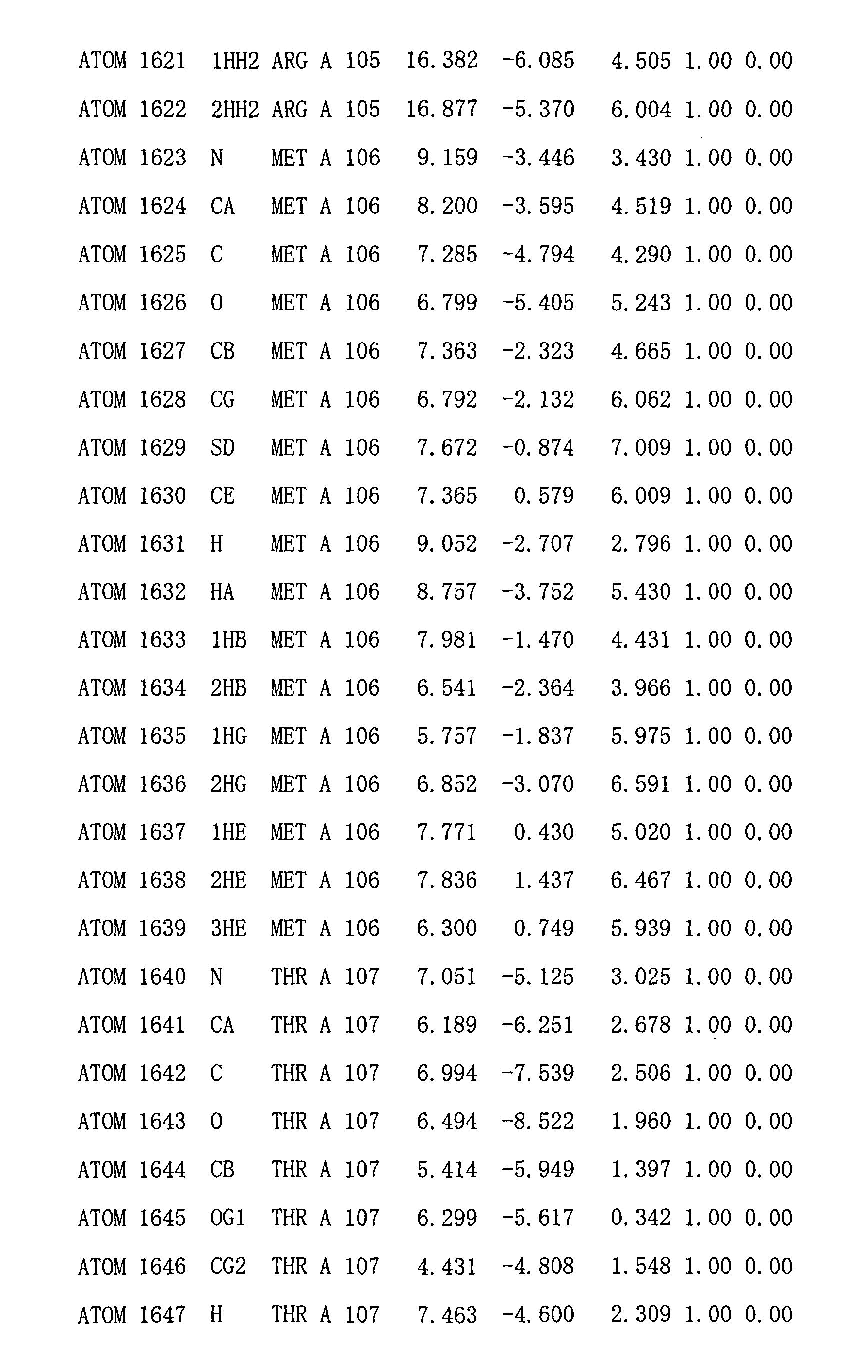

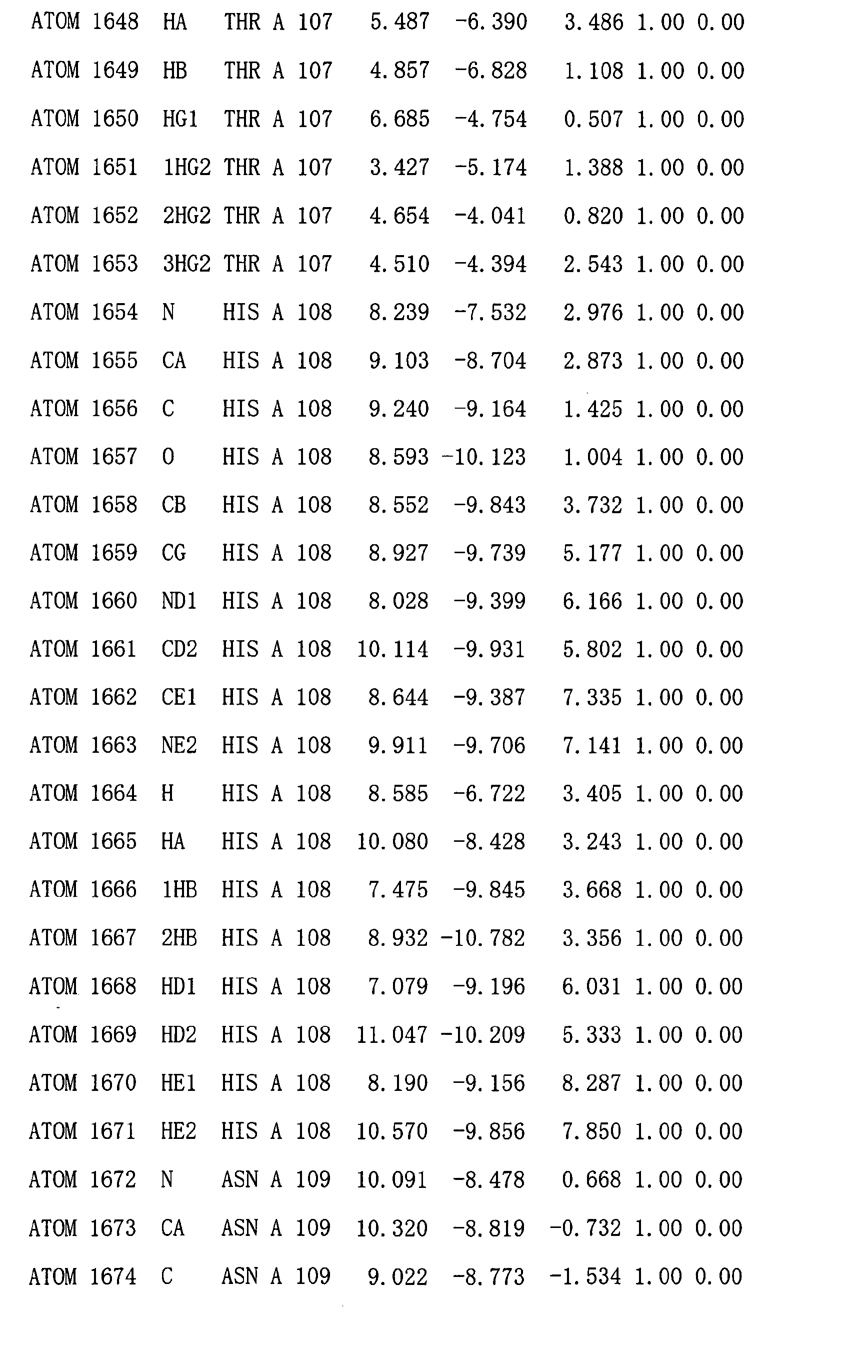

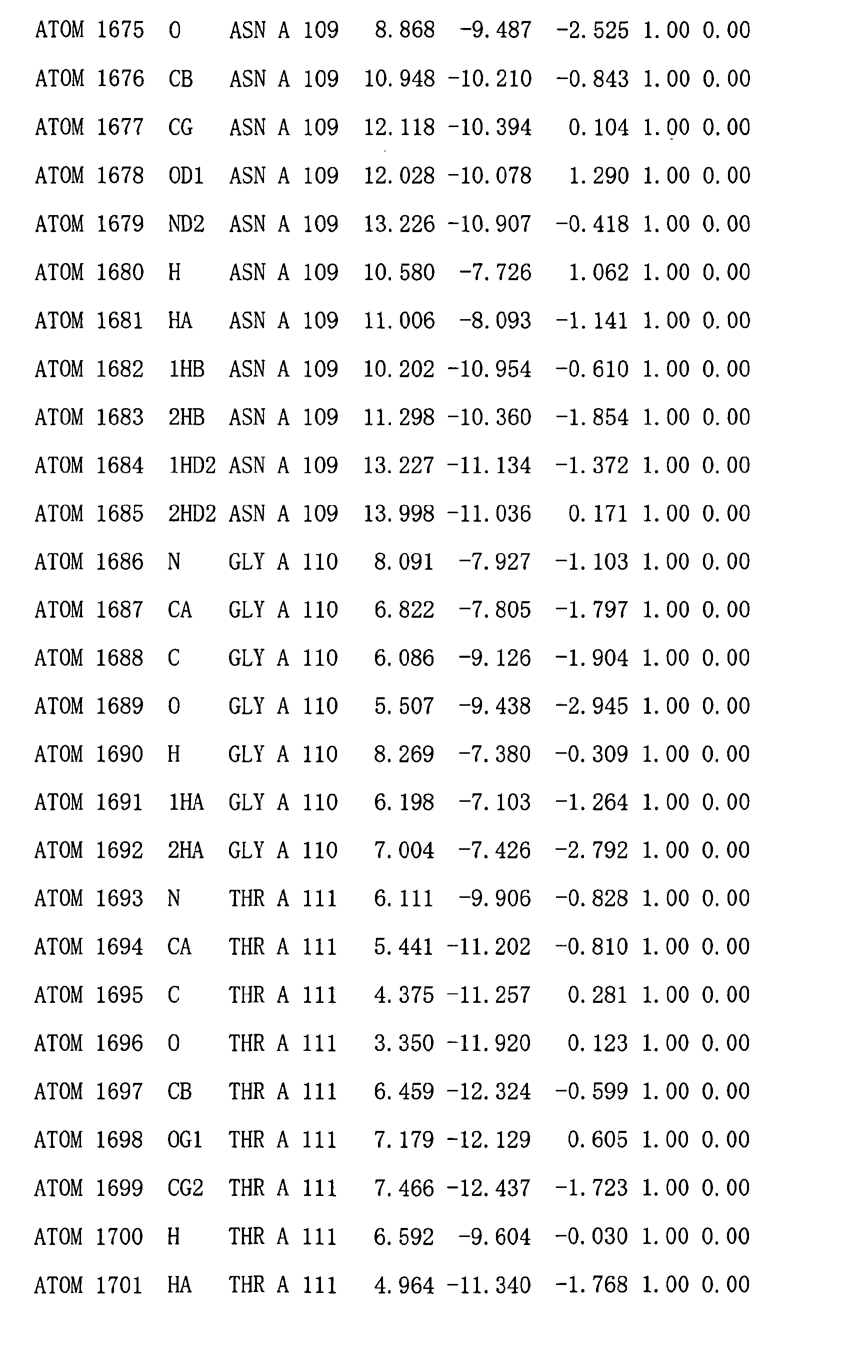

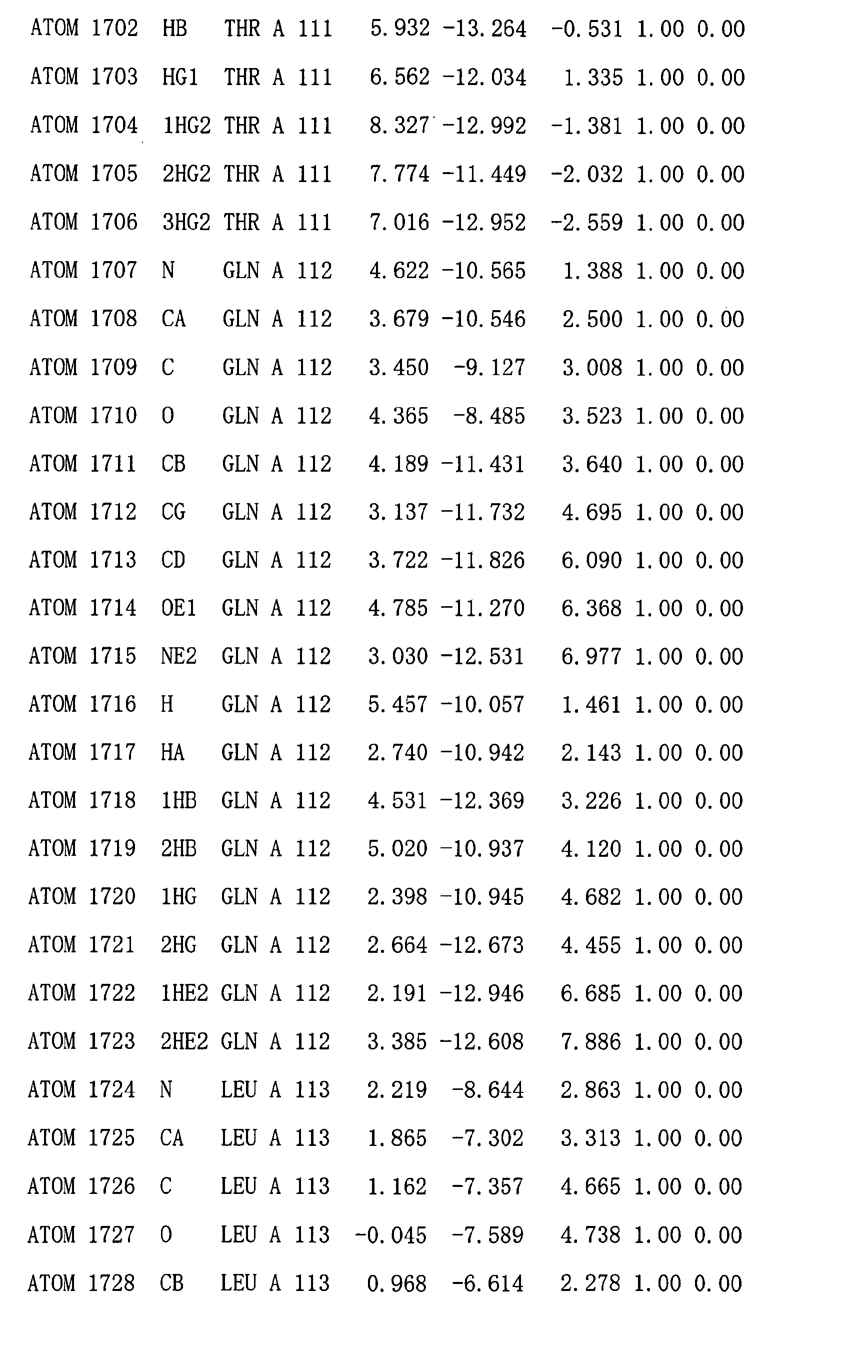

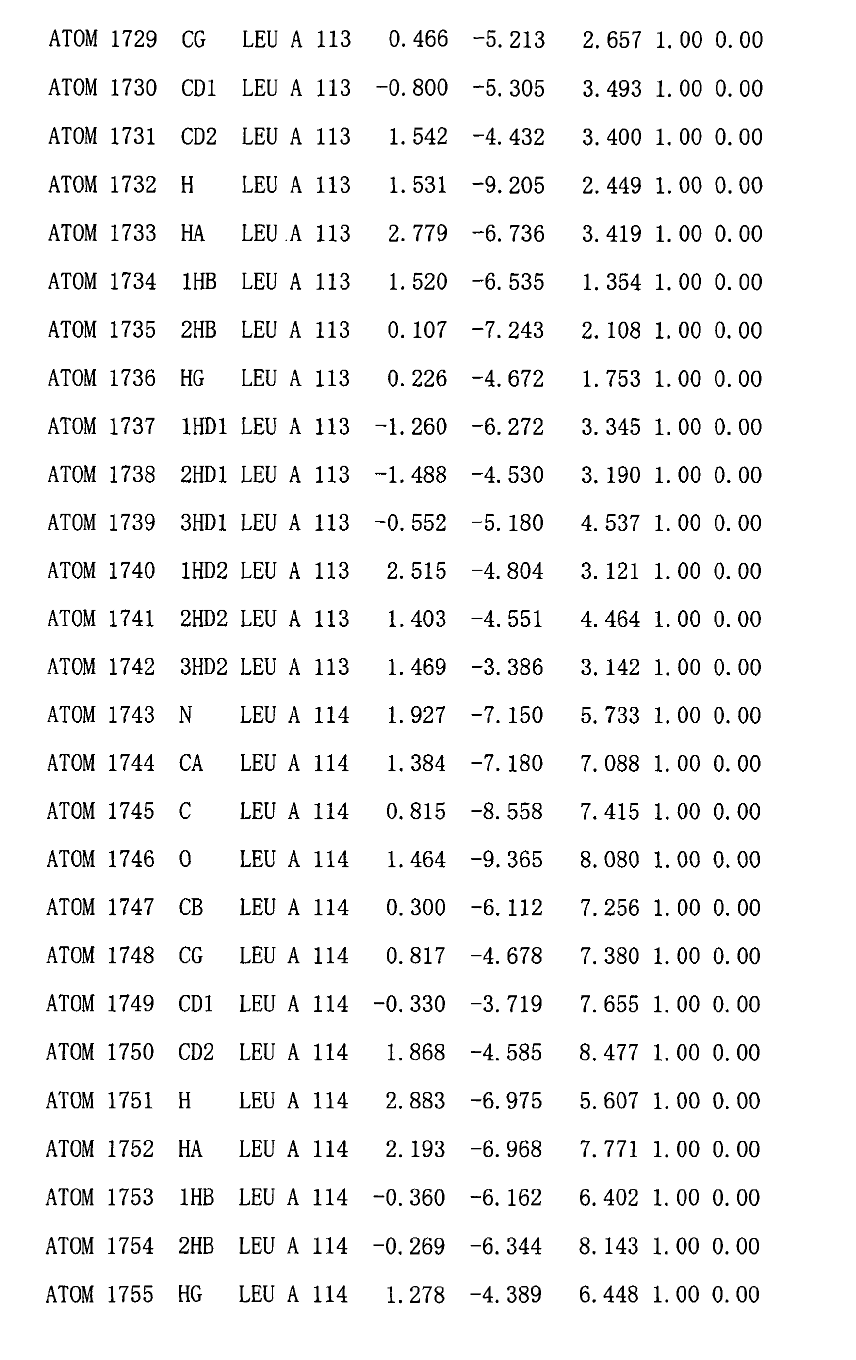

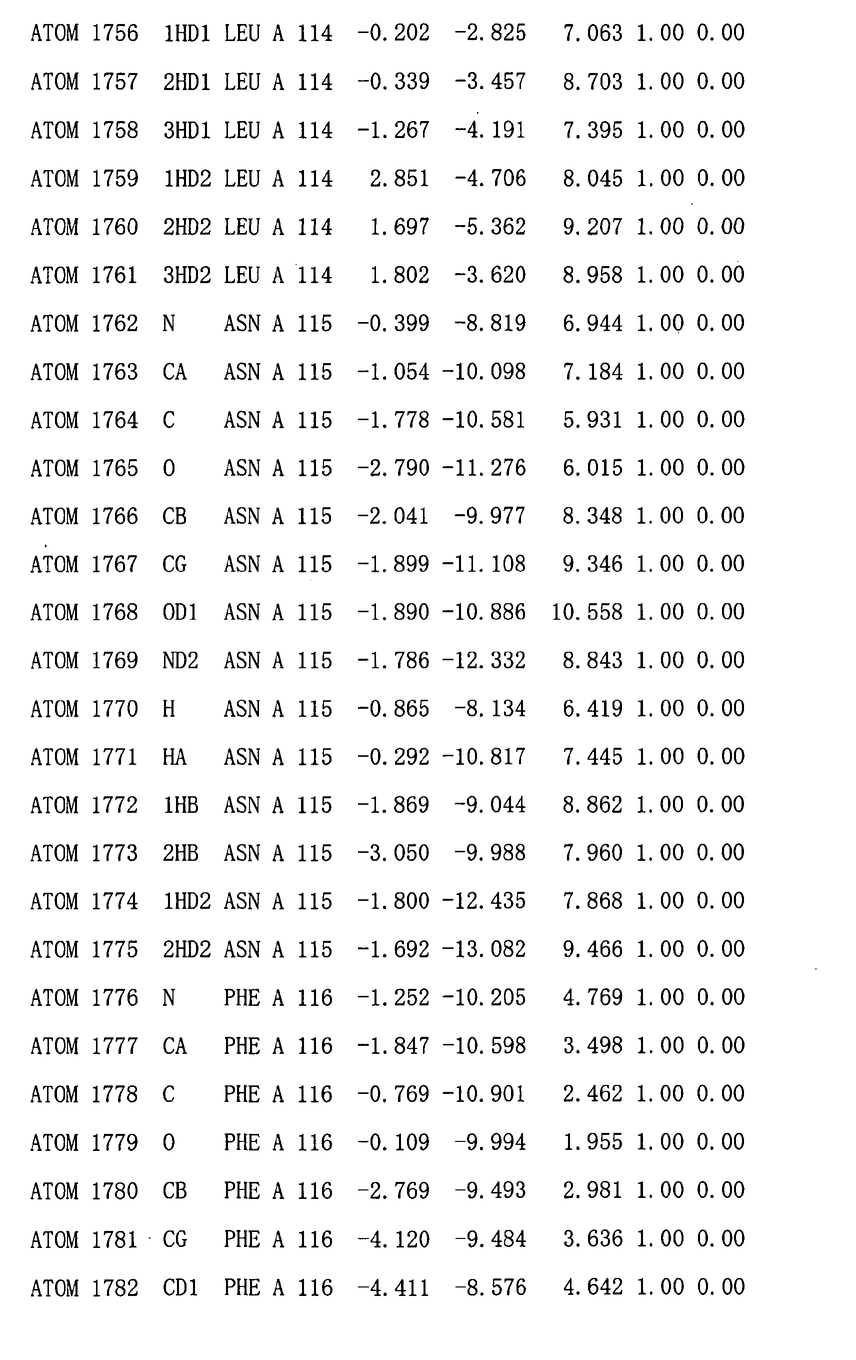

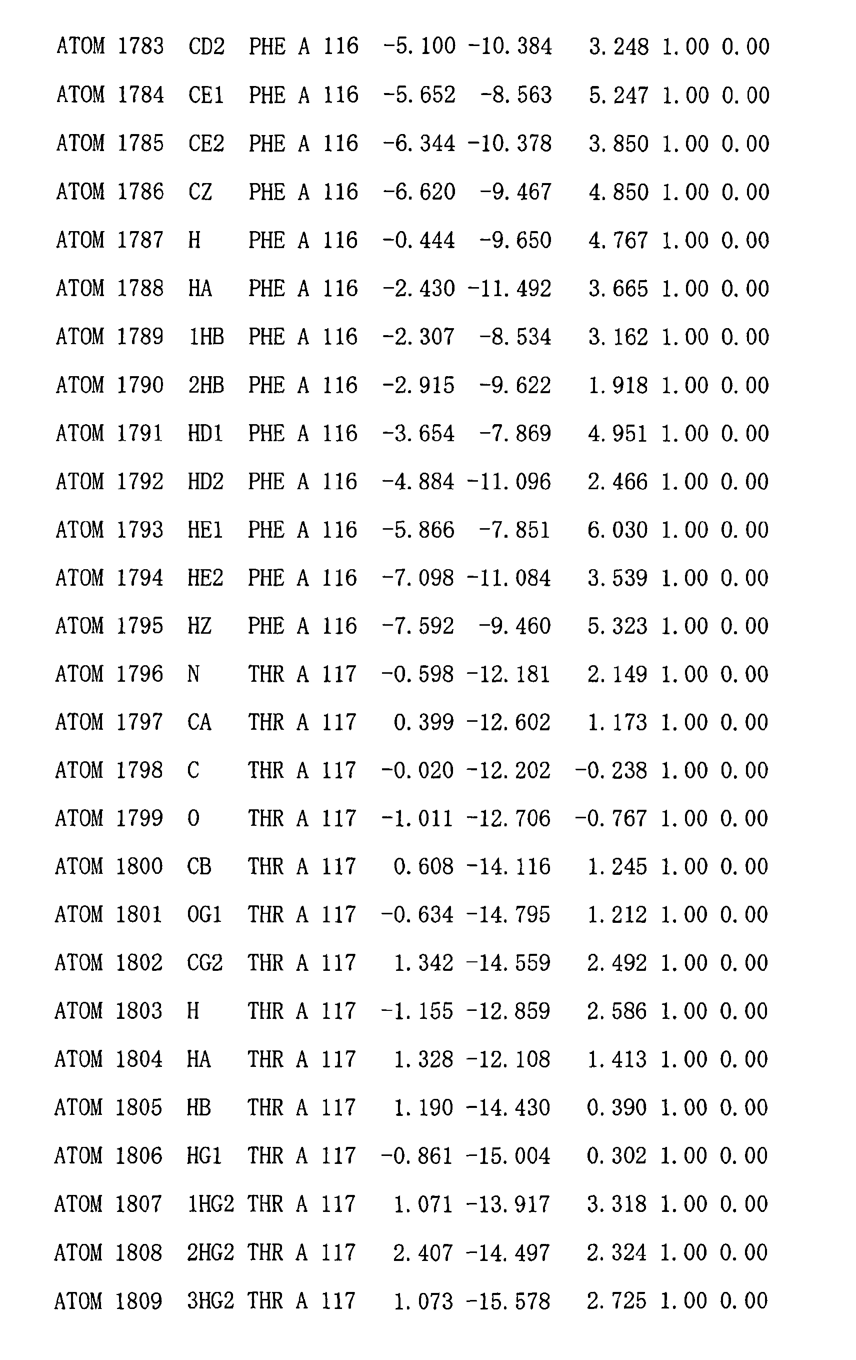

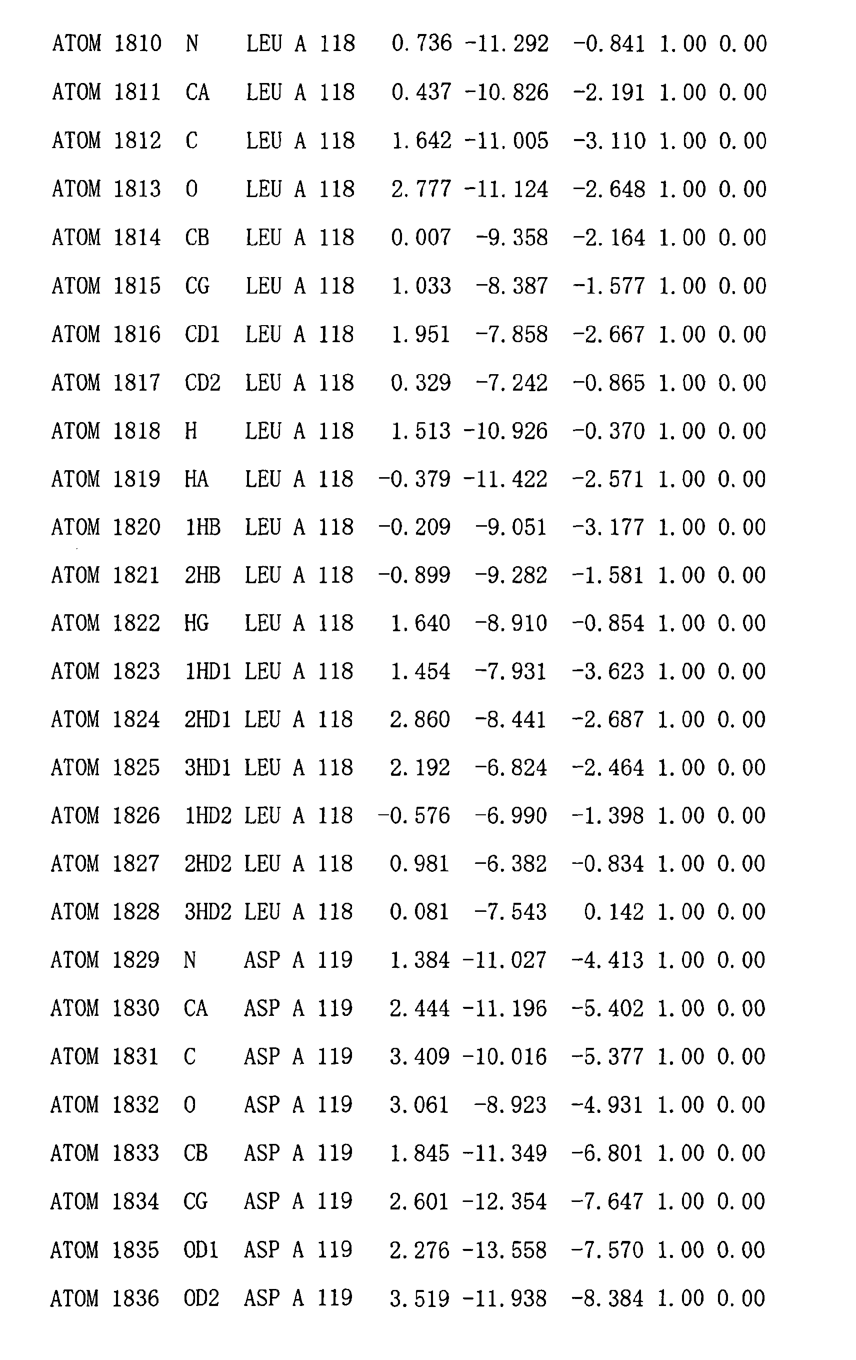

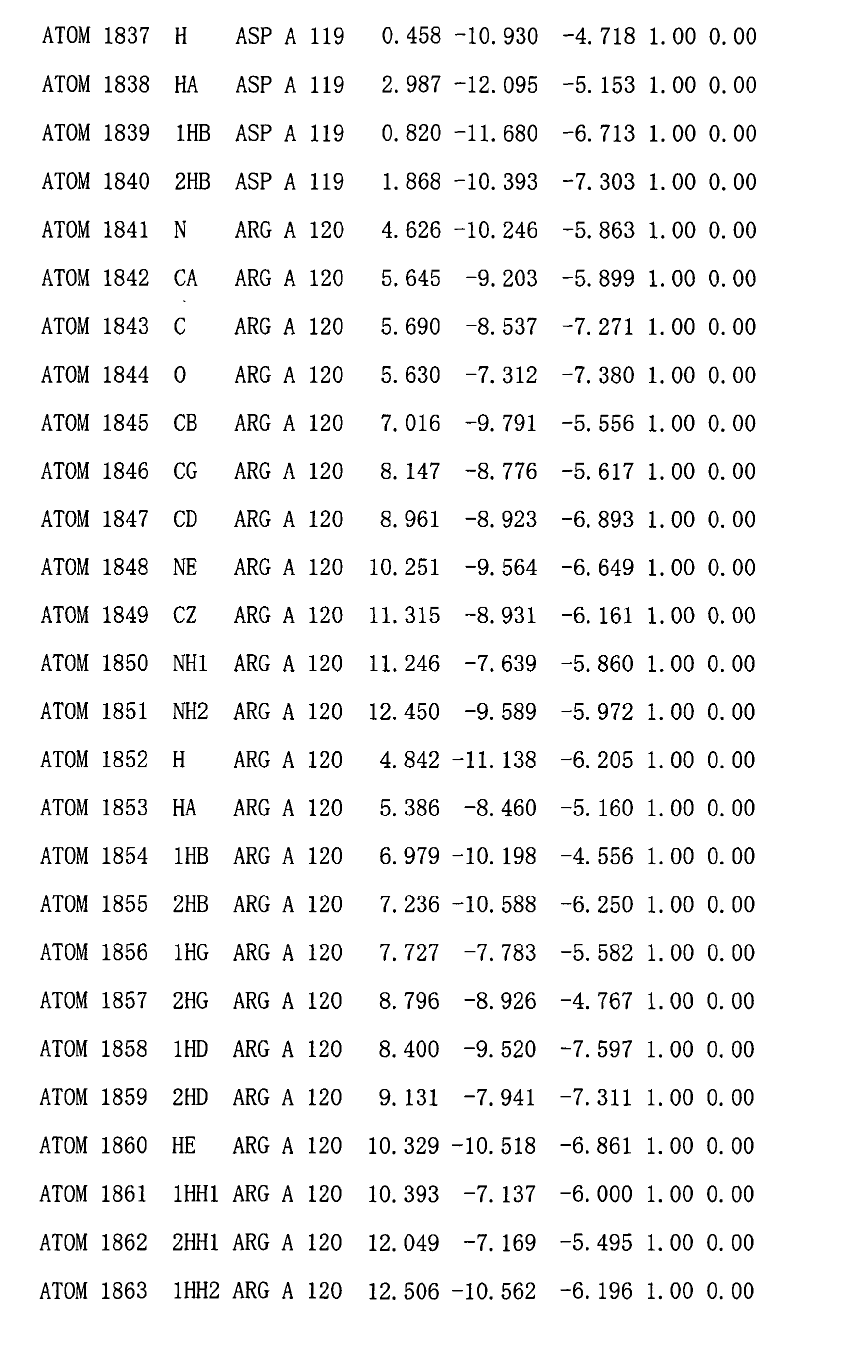

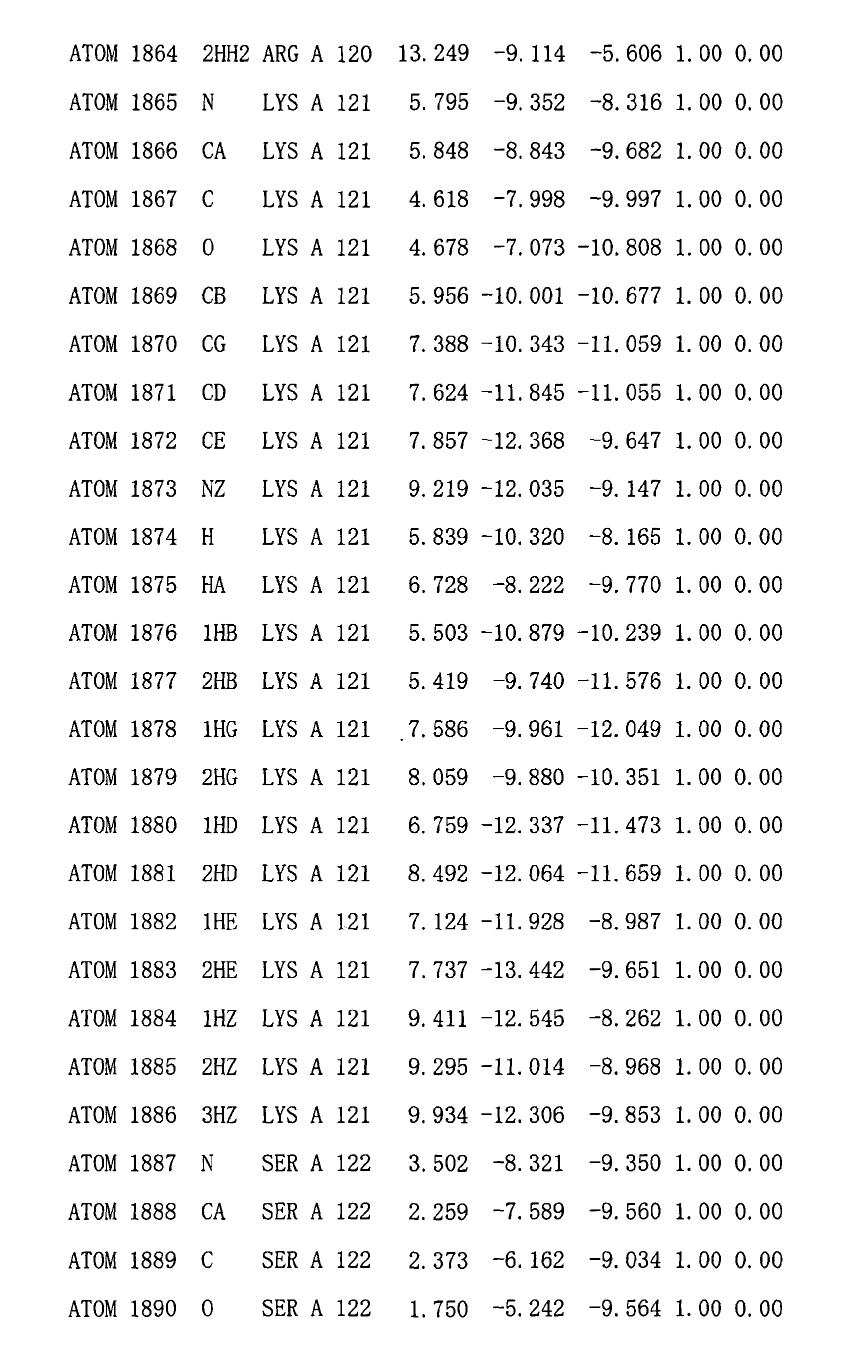

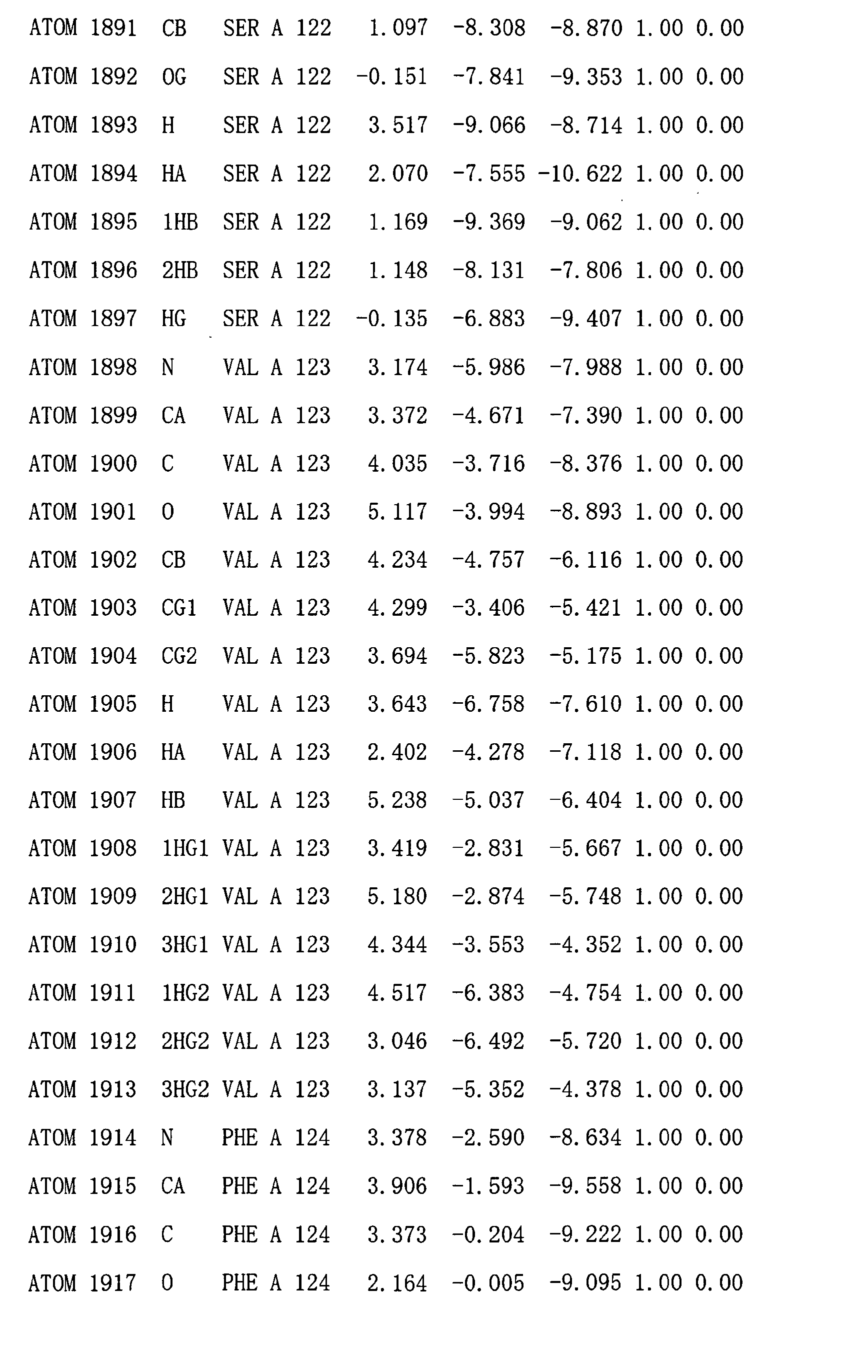

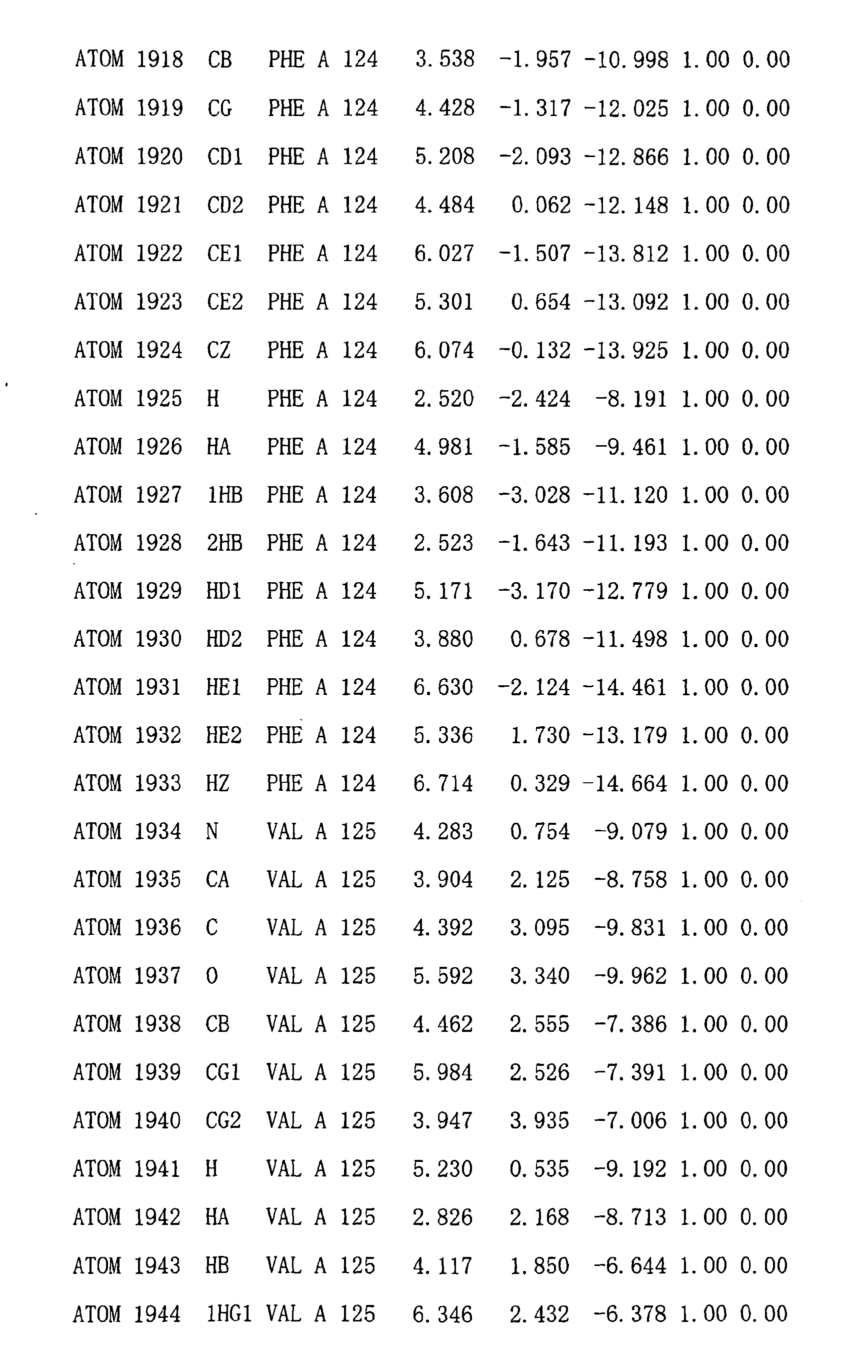

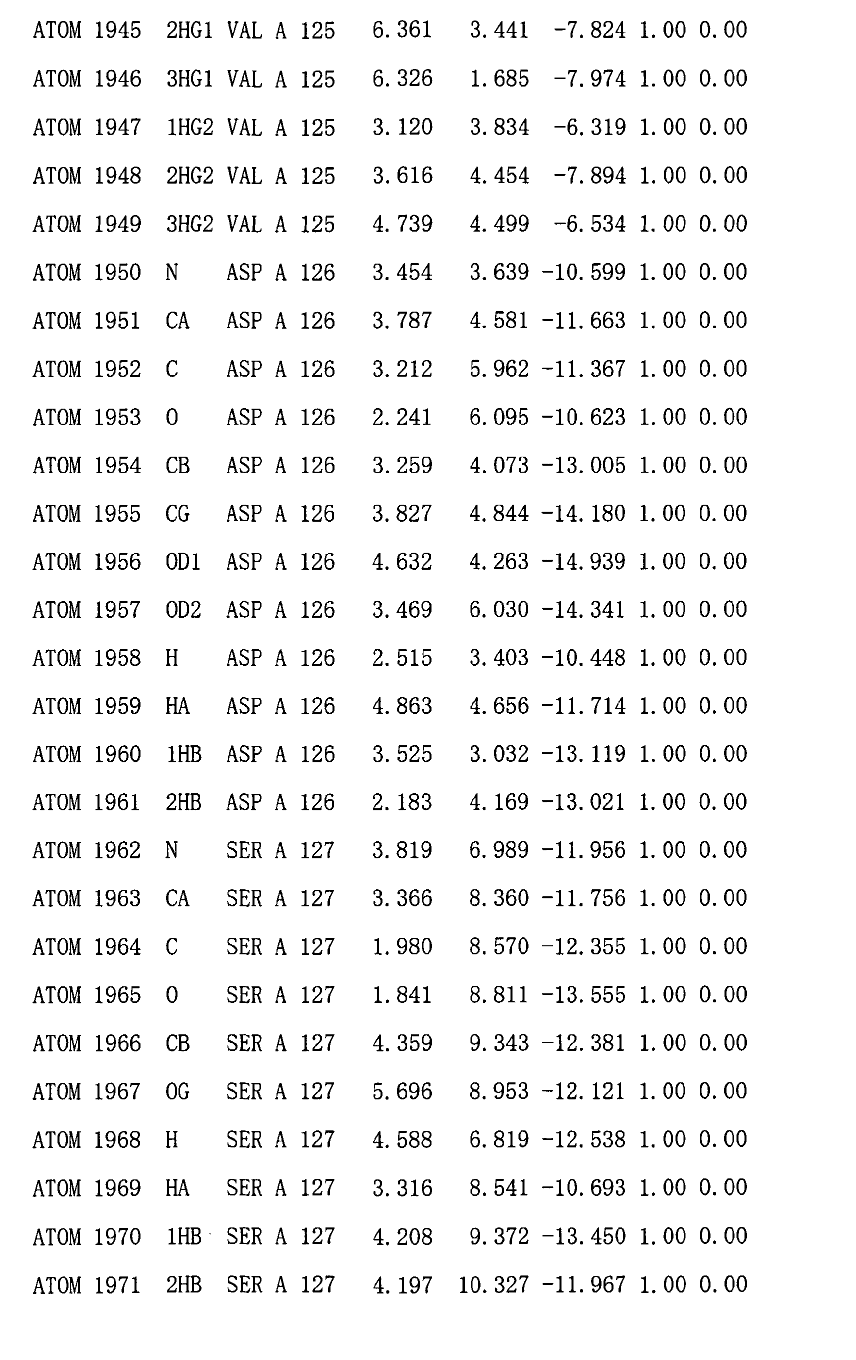

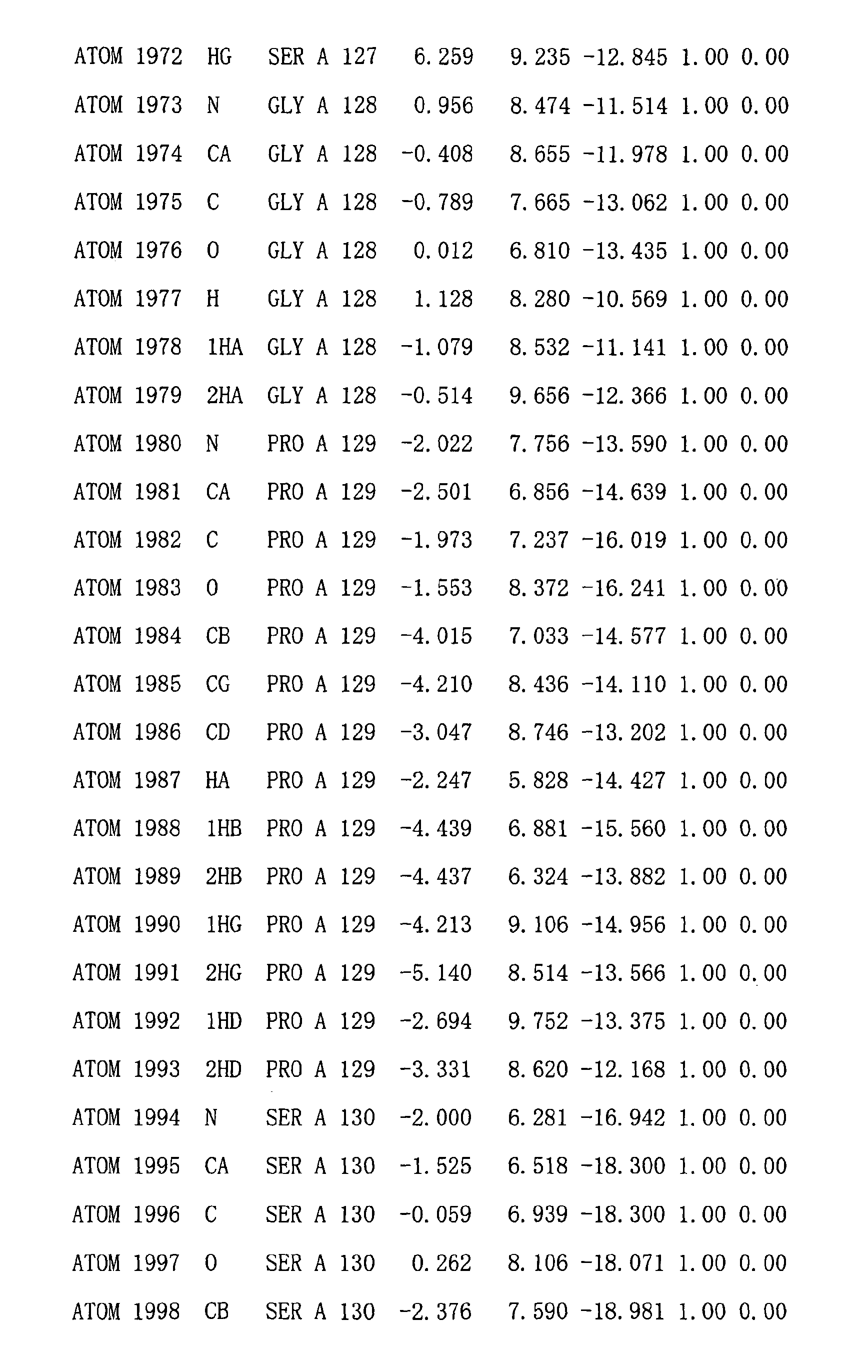

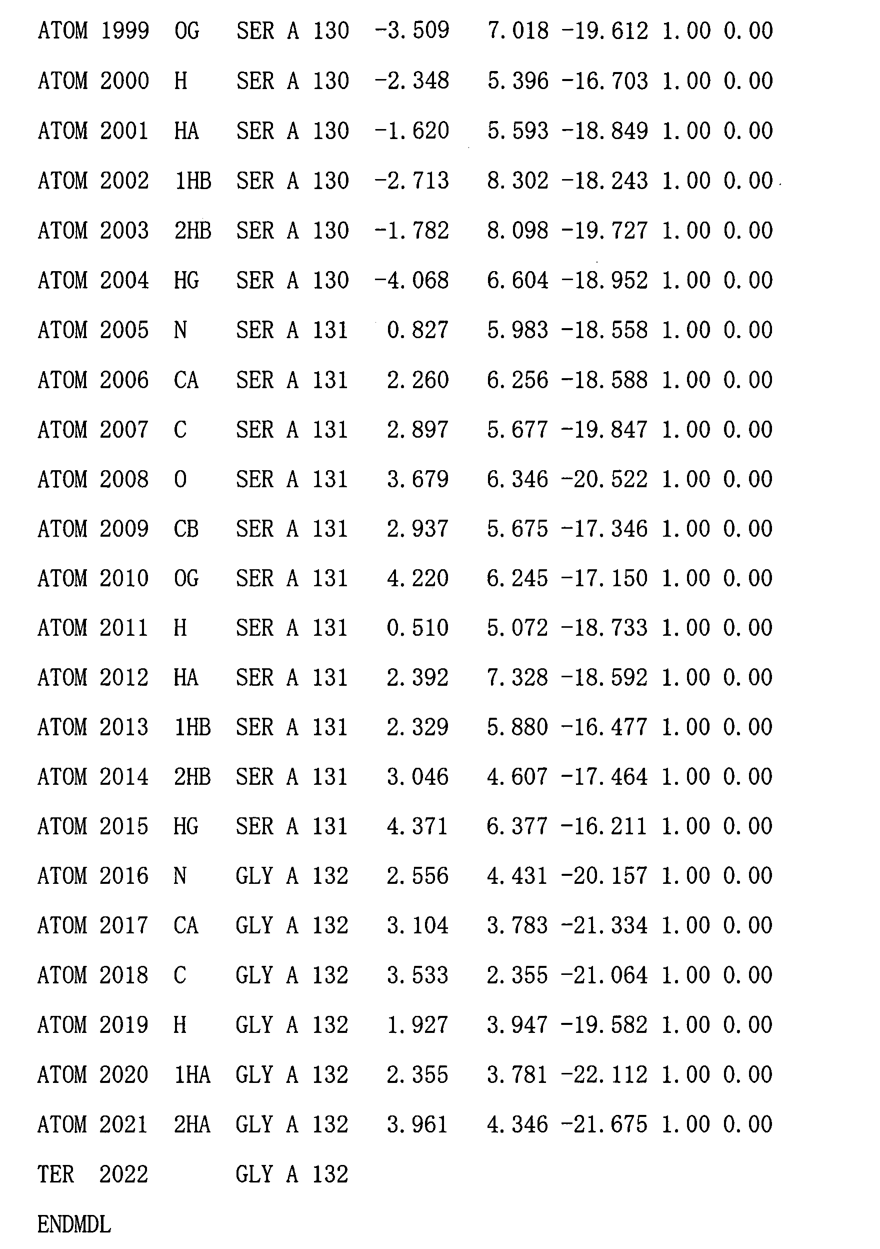

### Three-Dimensional Structure Coordinate 4

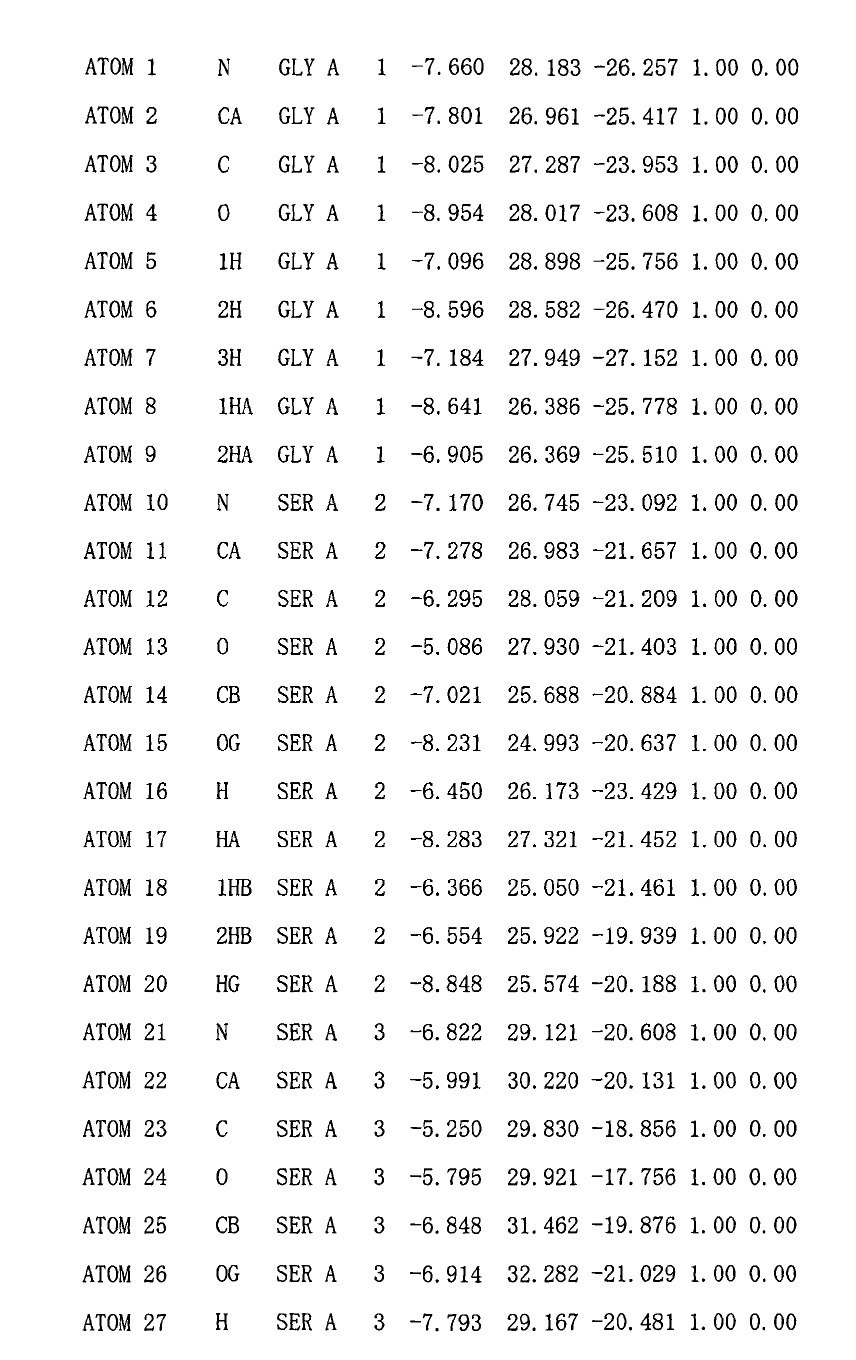

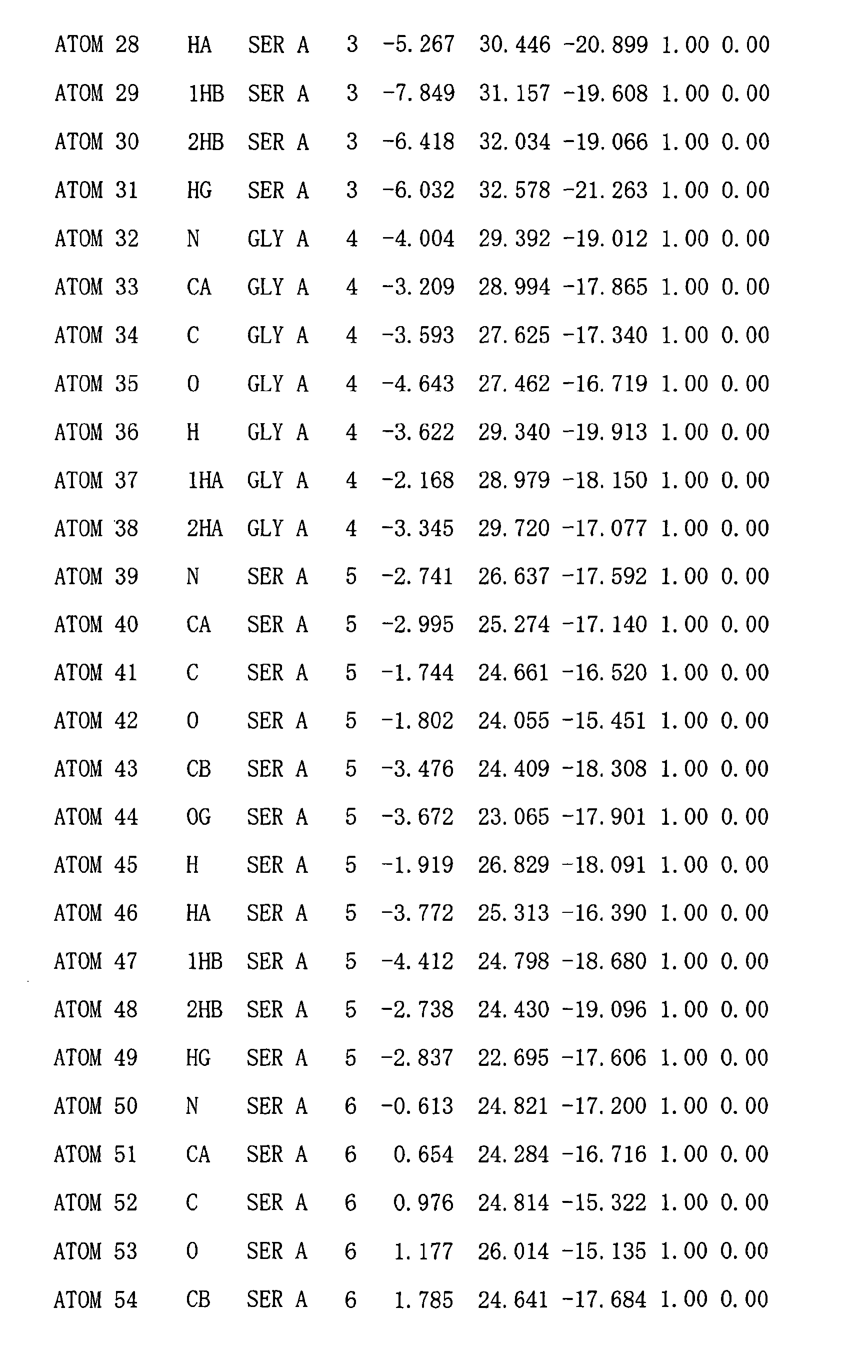

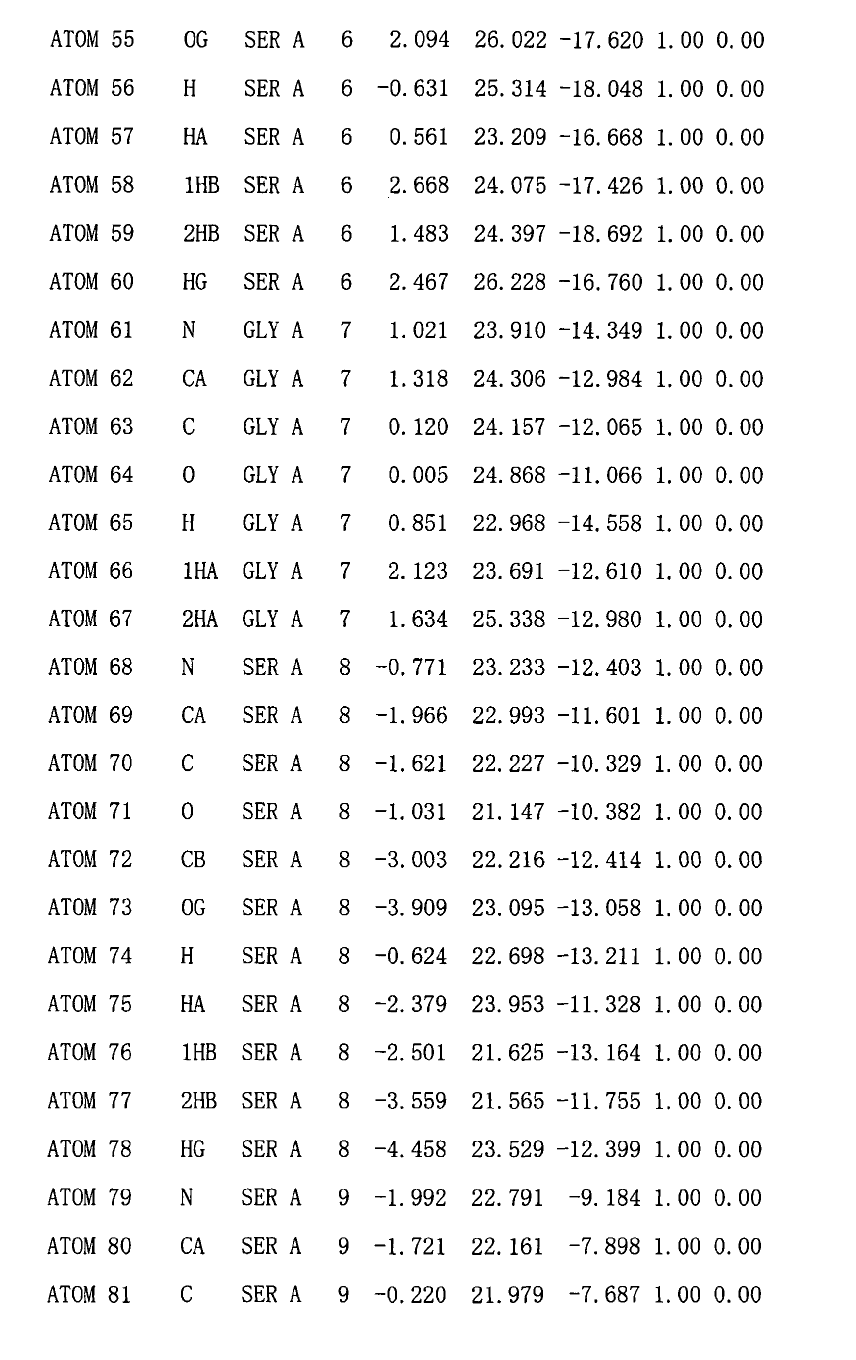

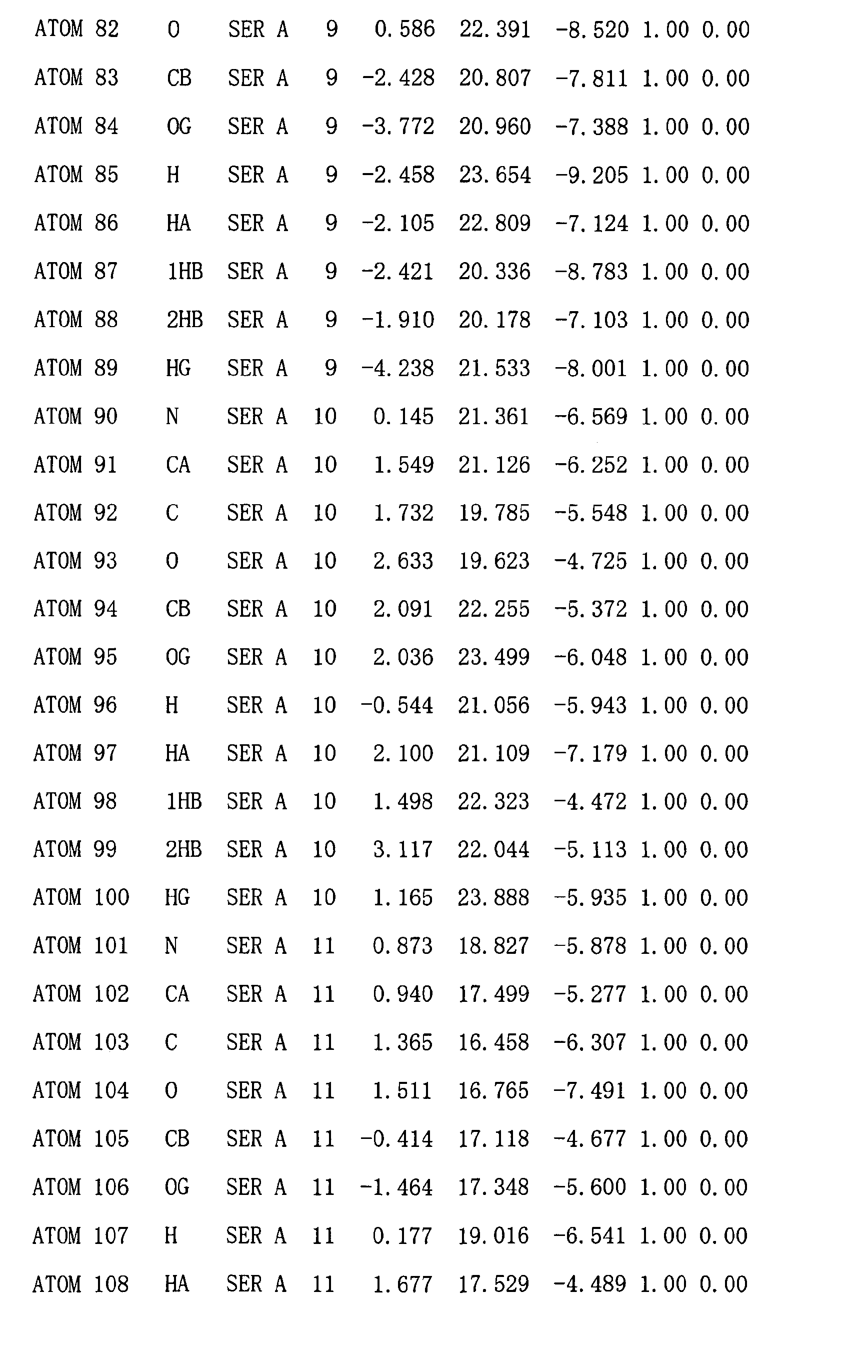

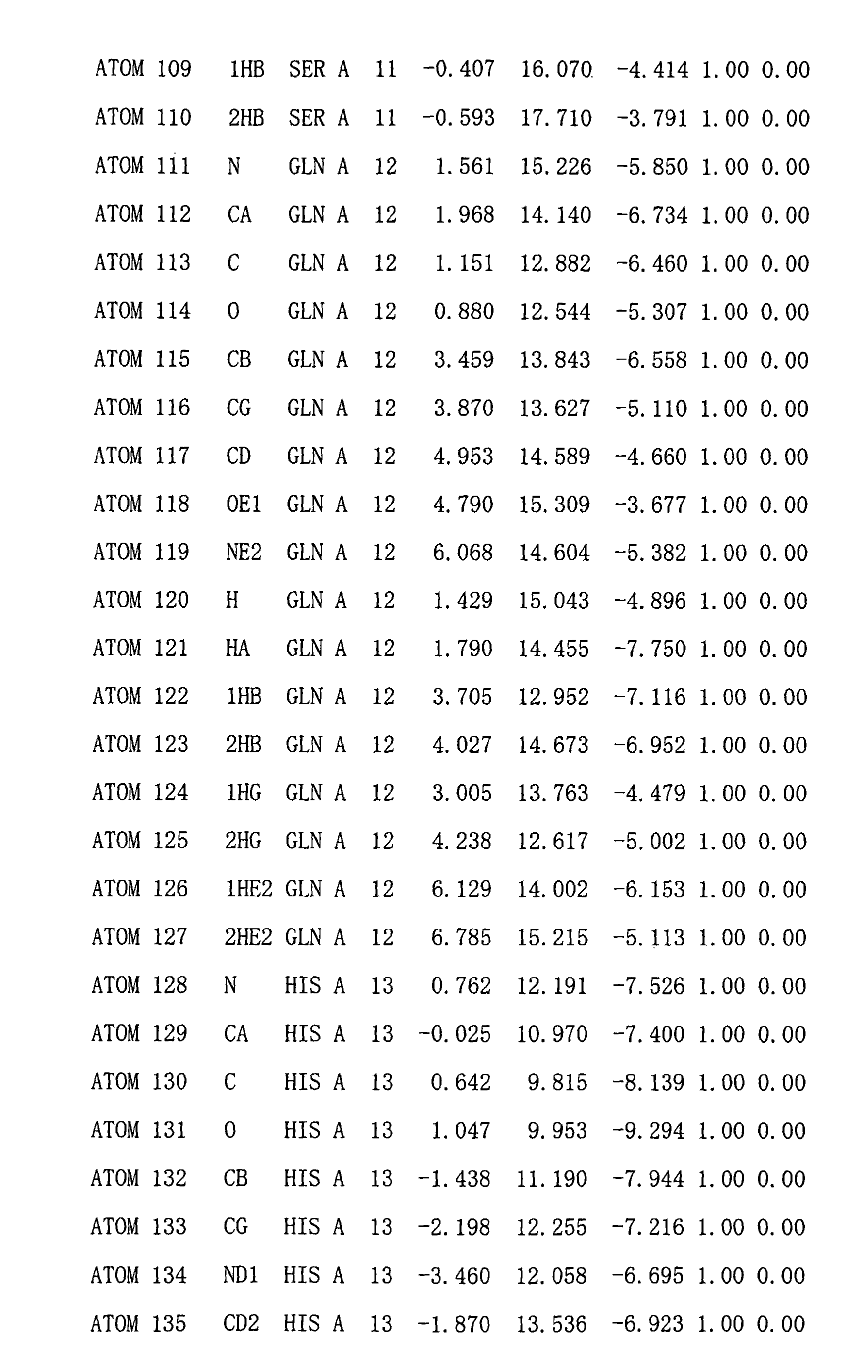

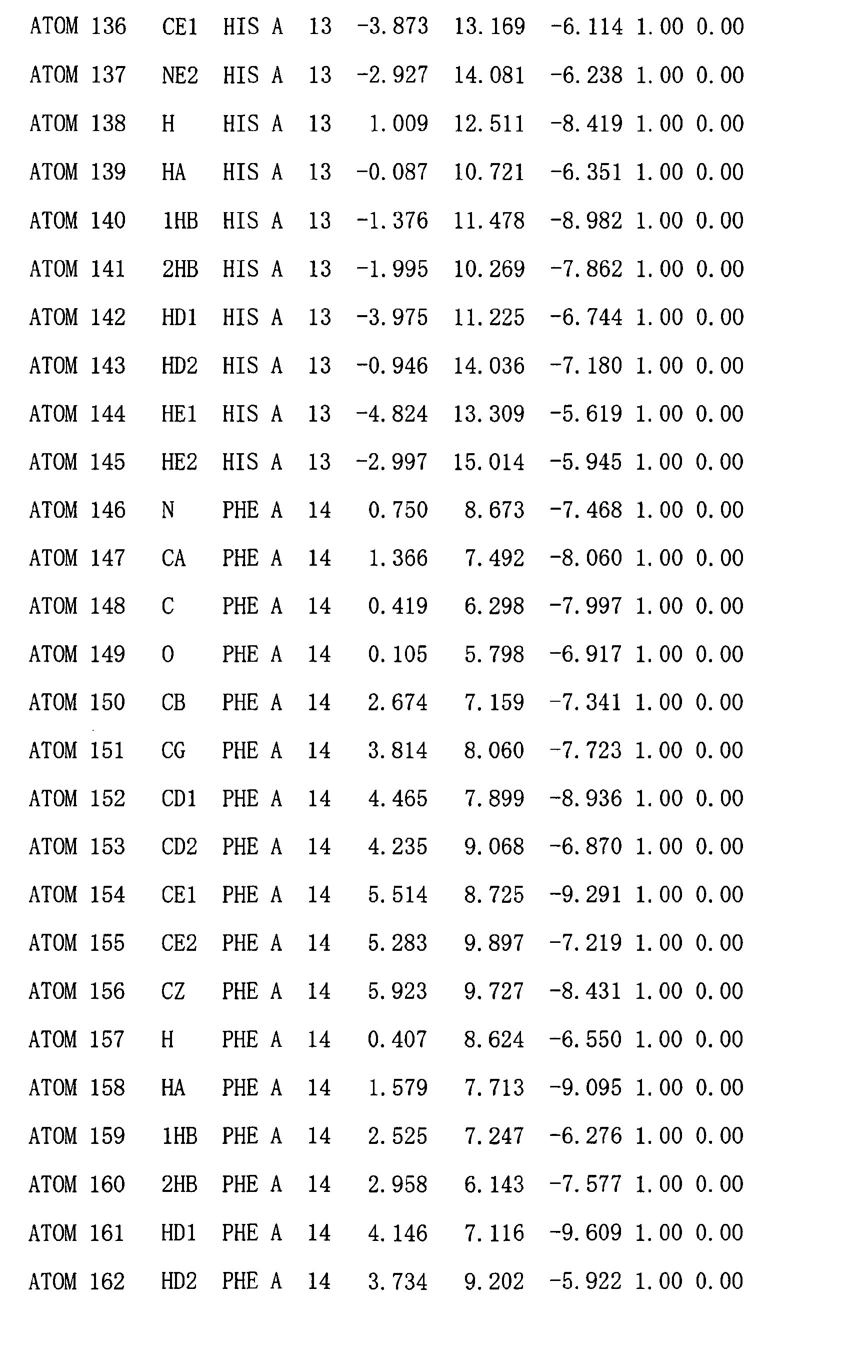

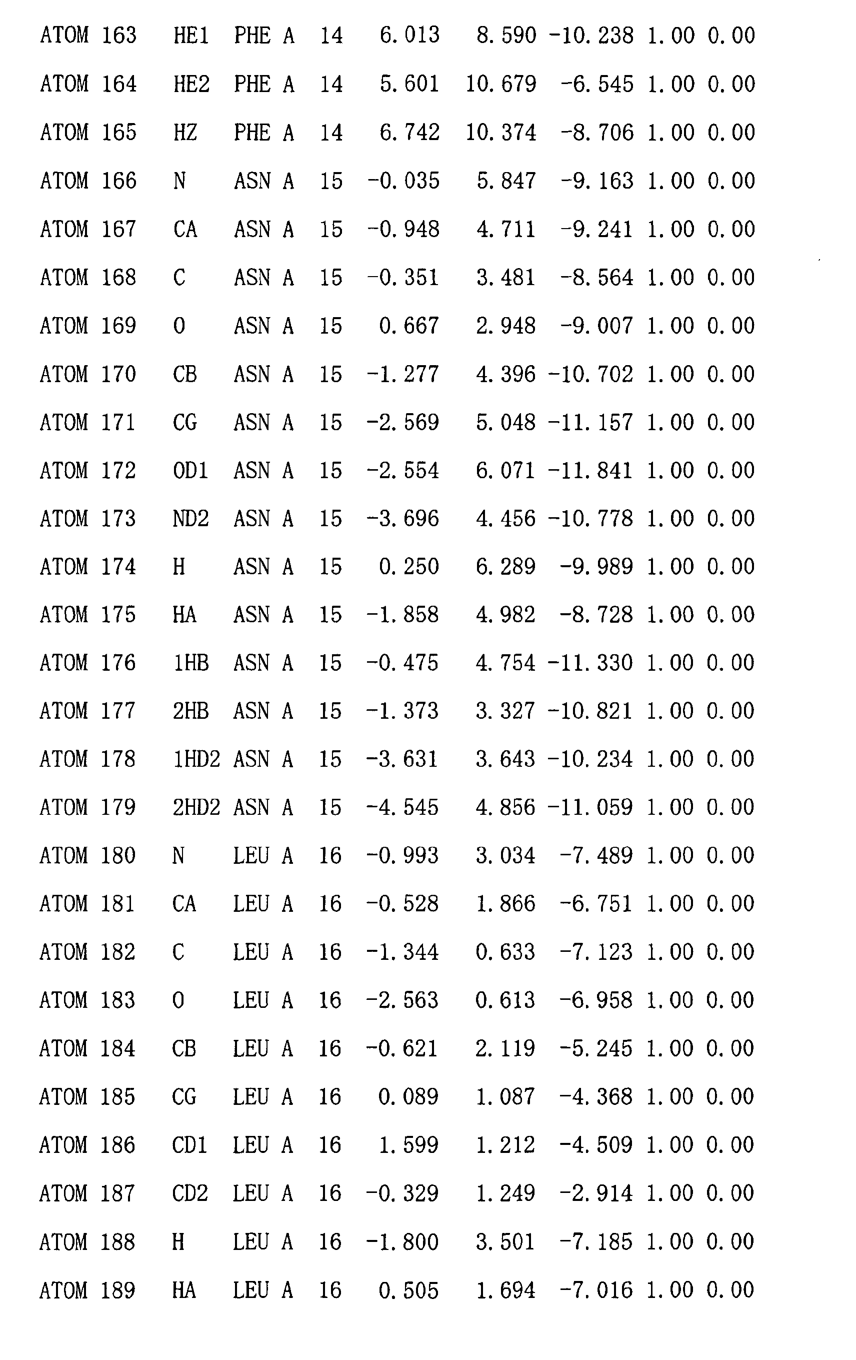

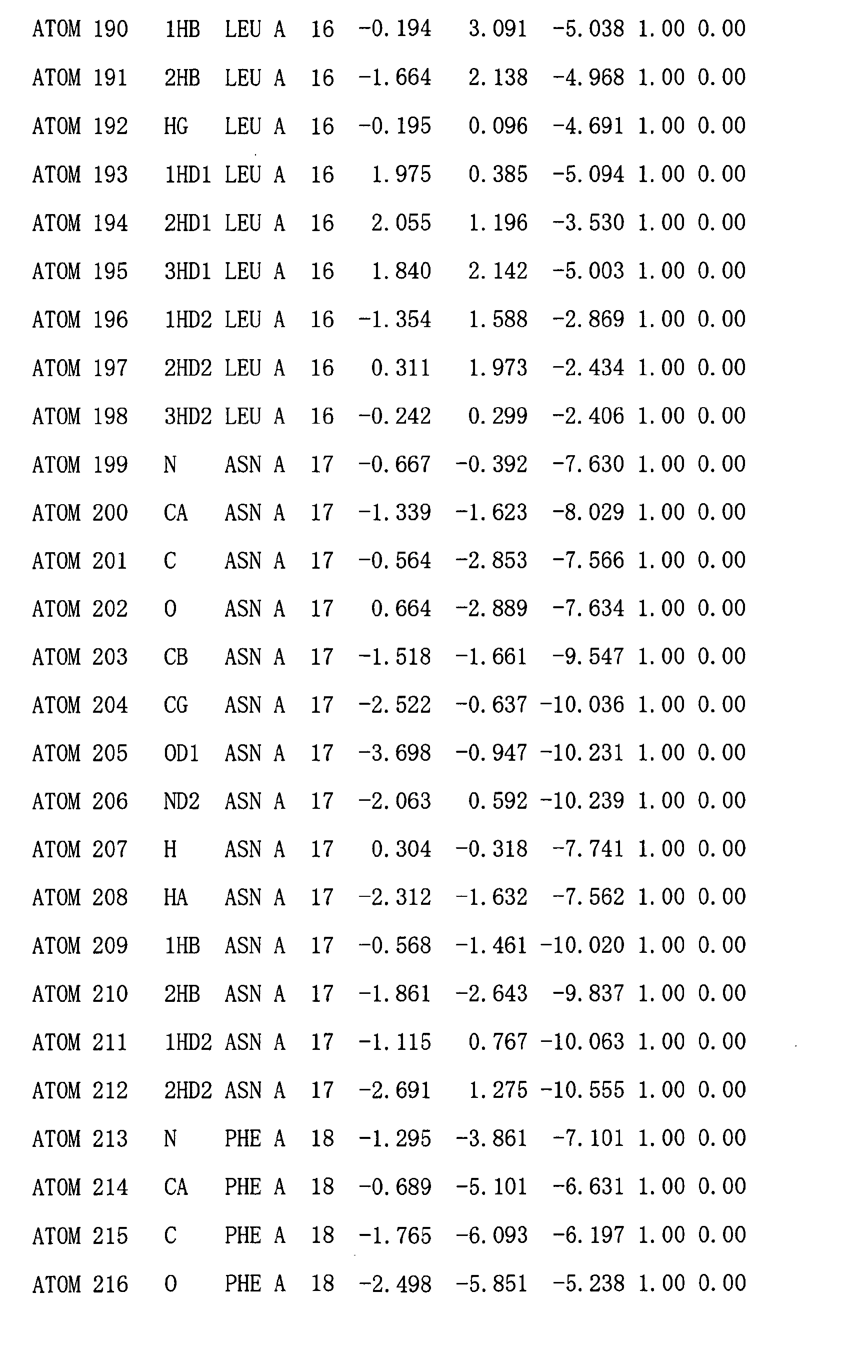

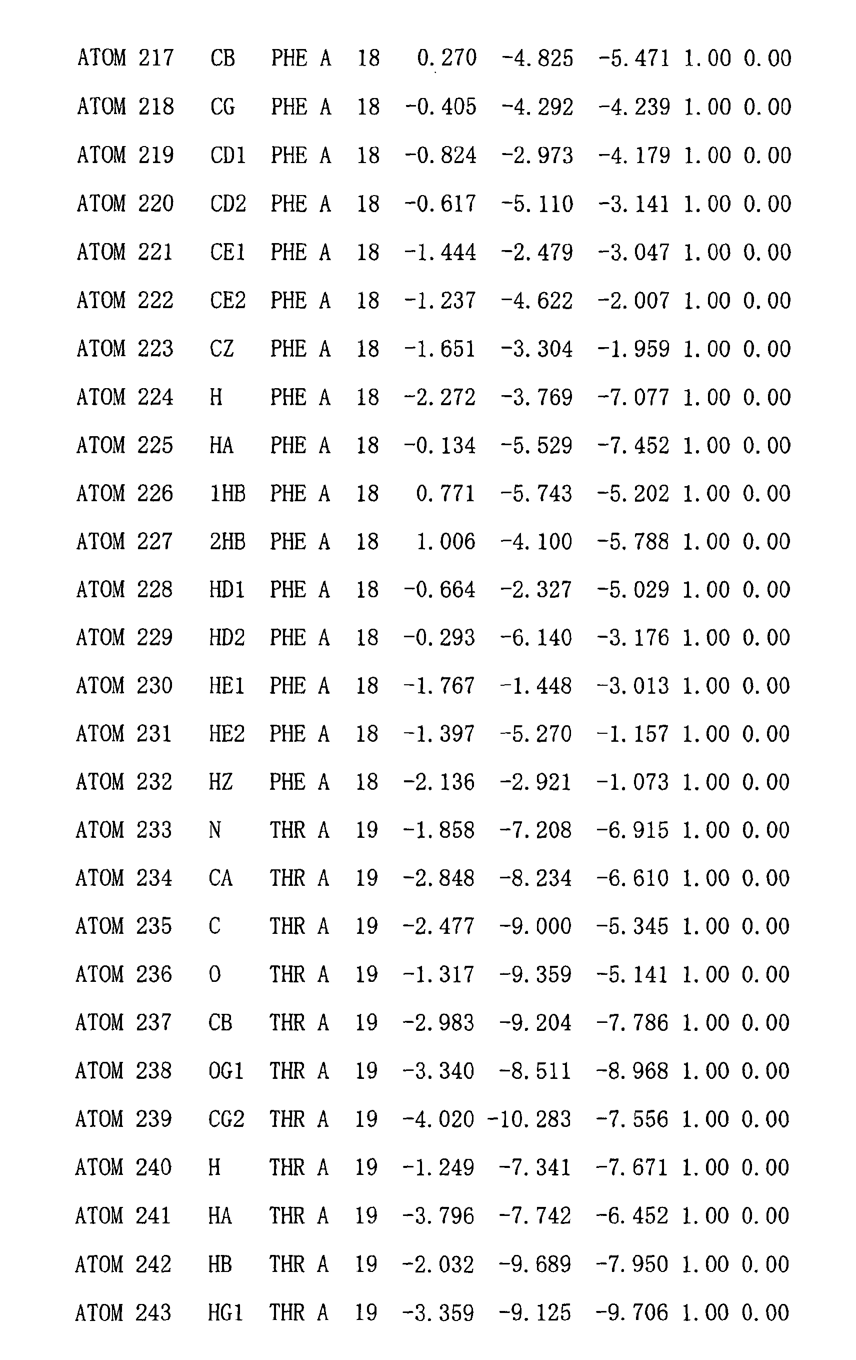

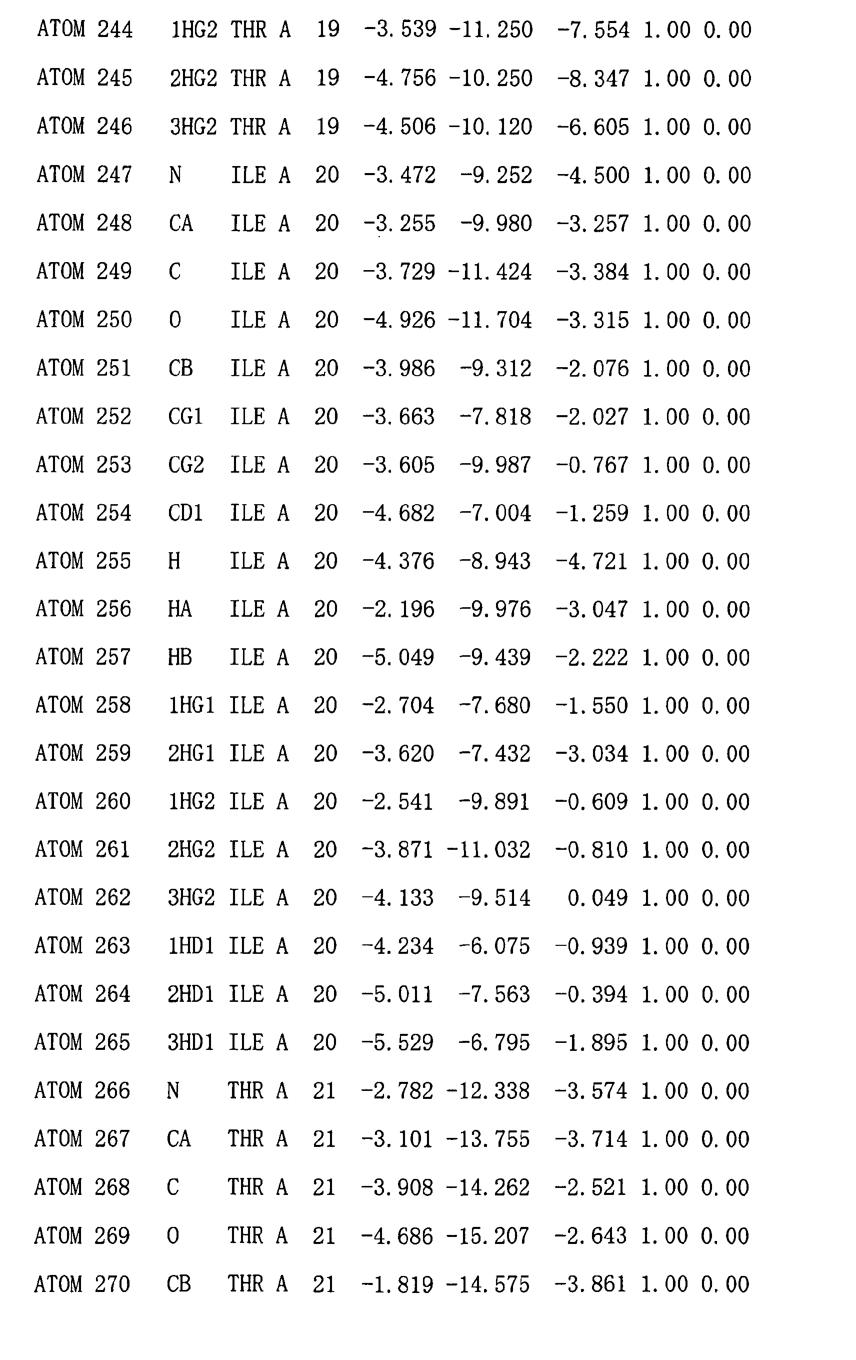

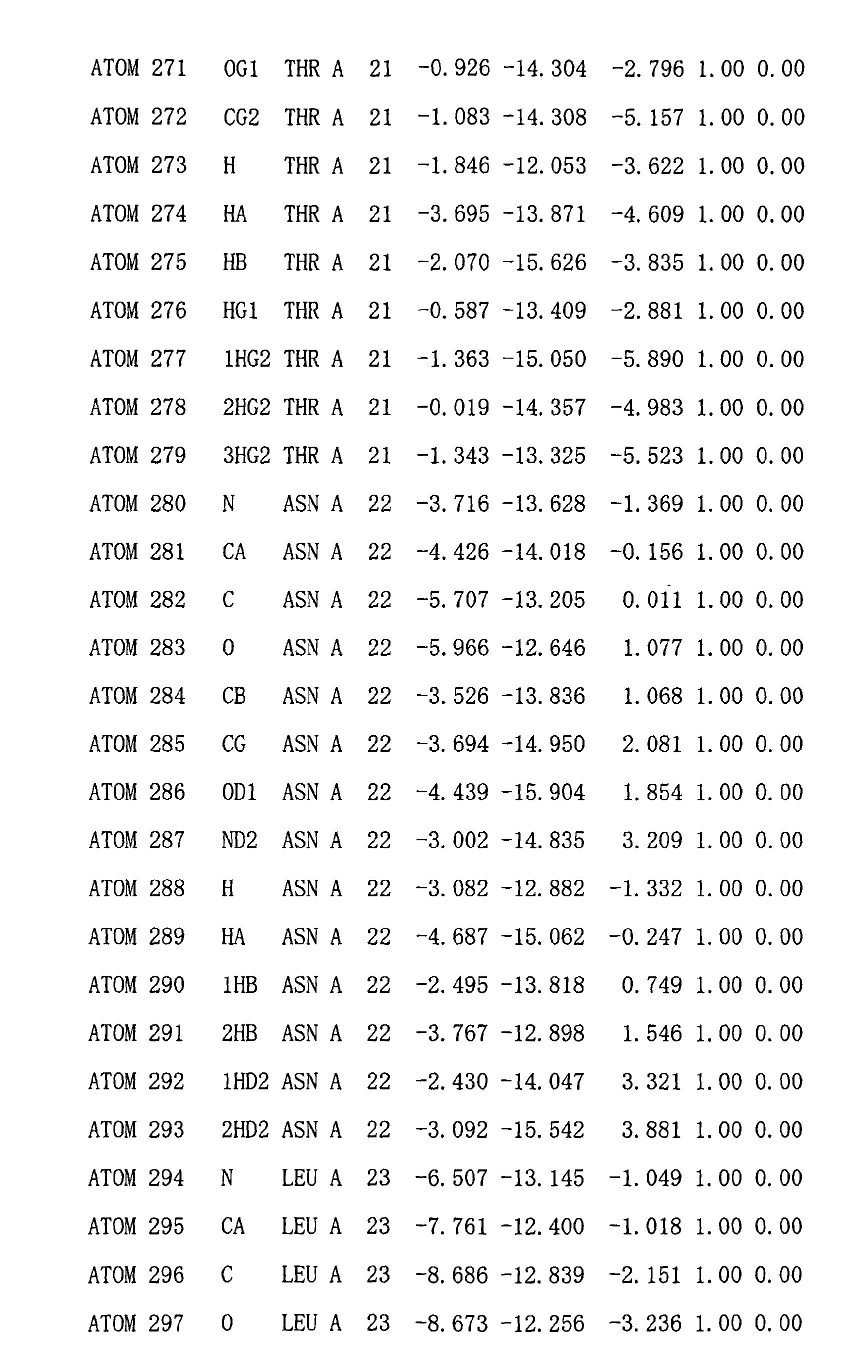

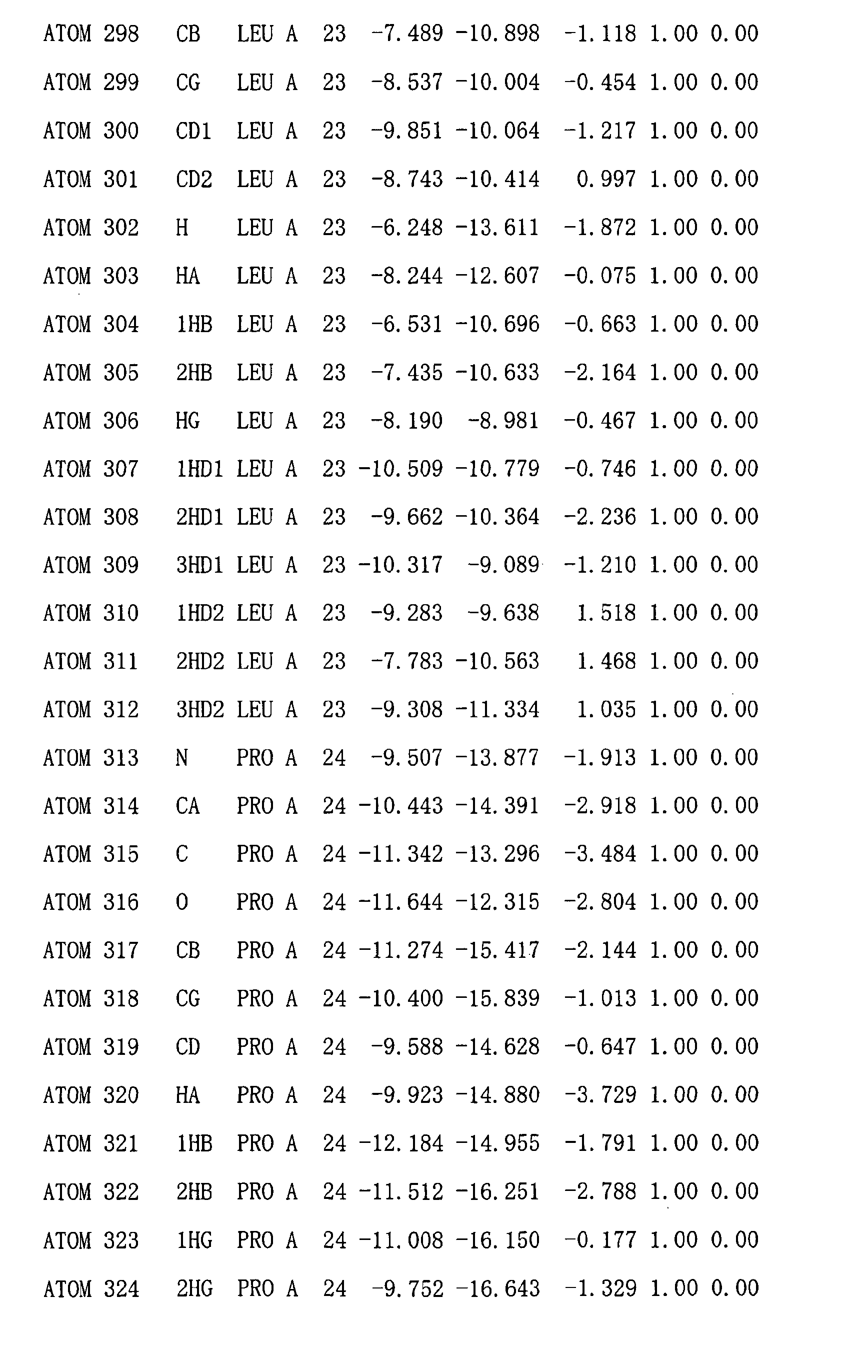

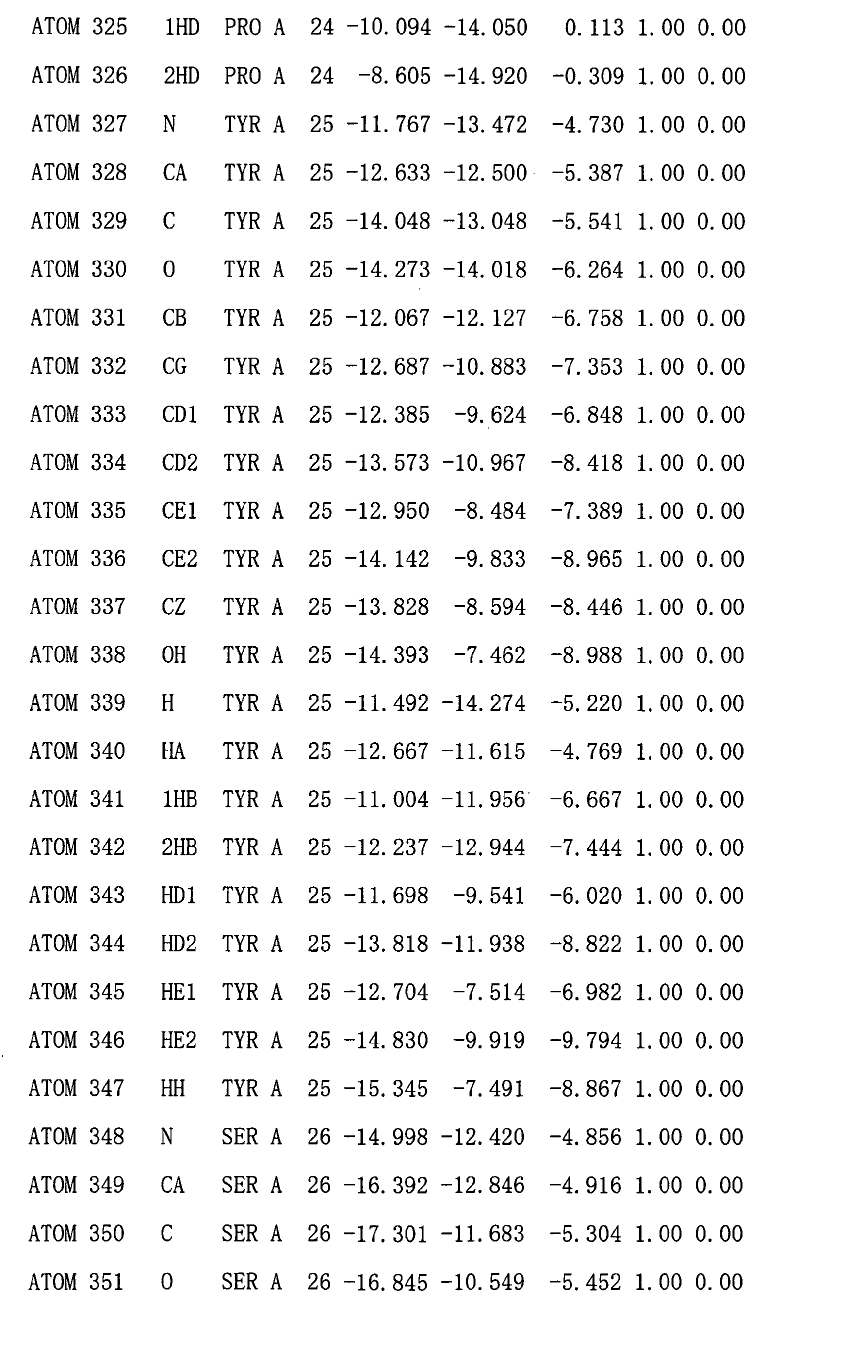

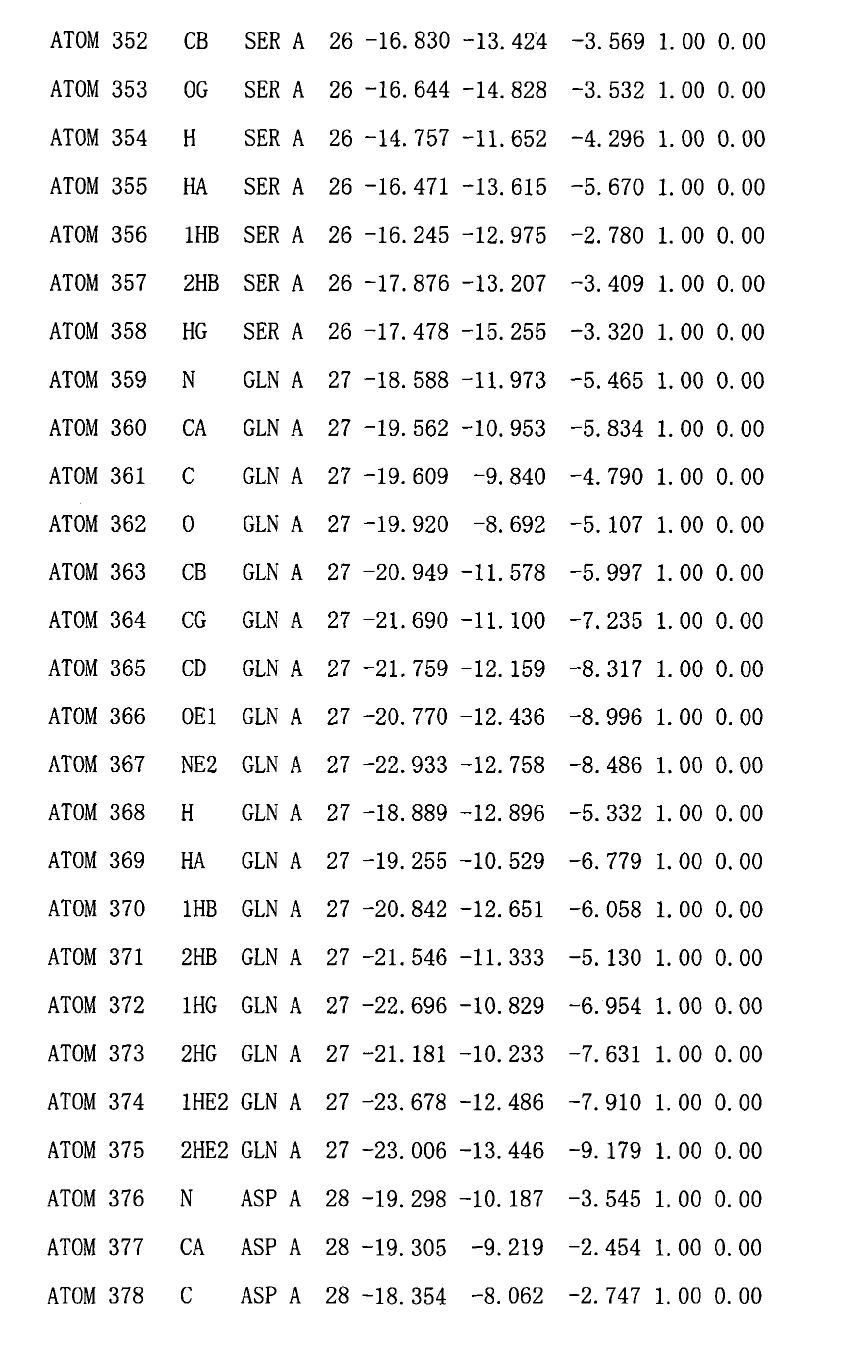

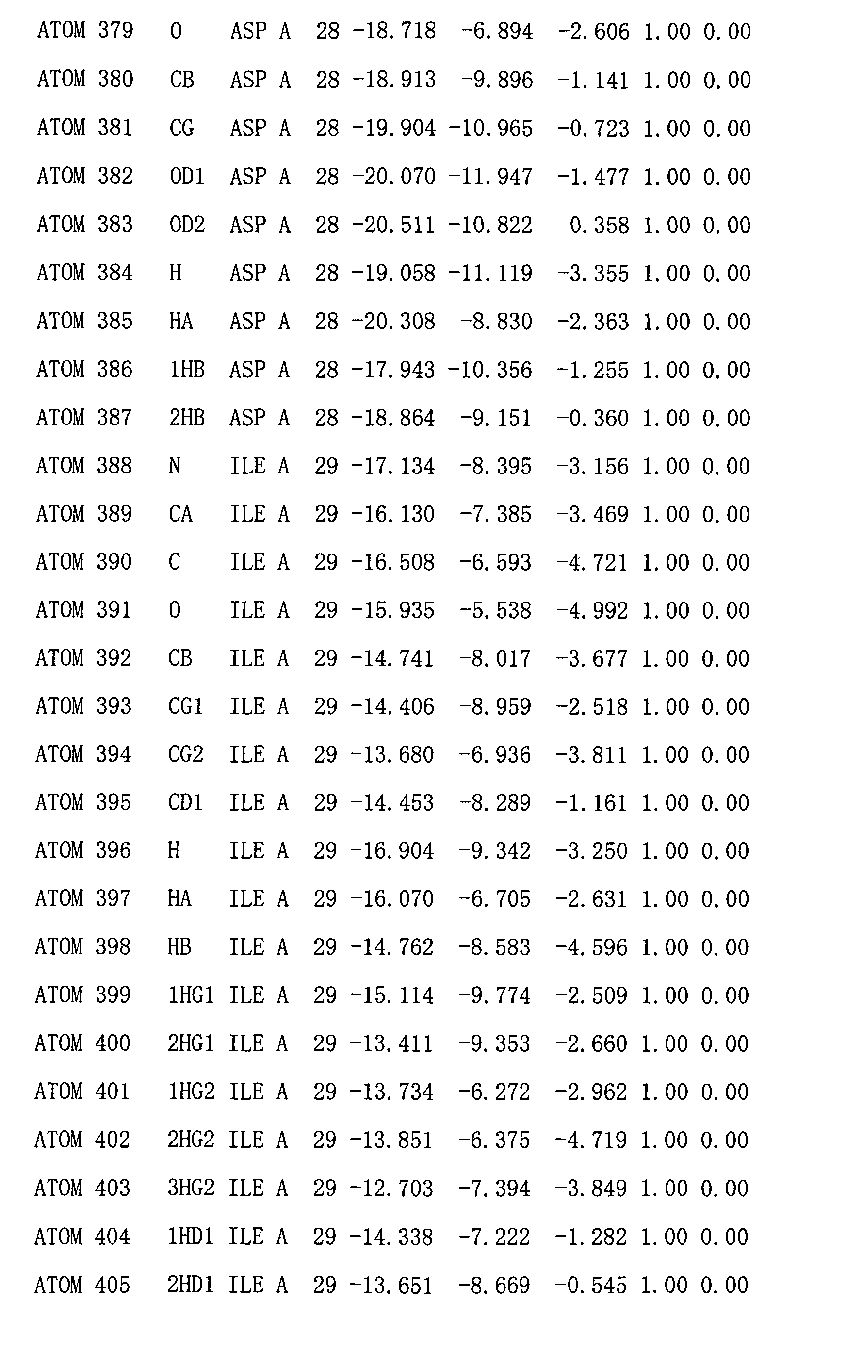

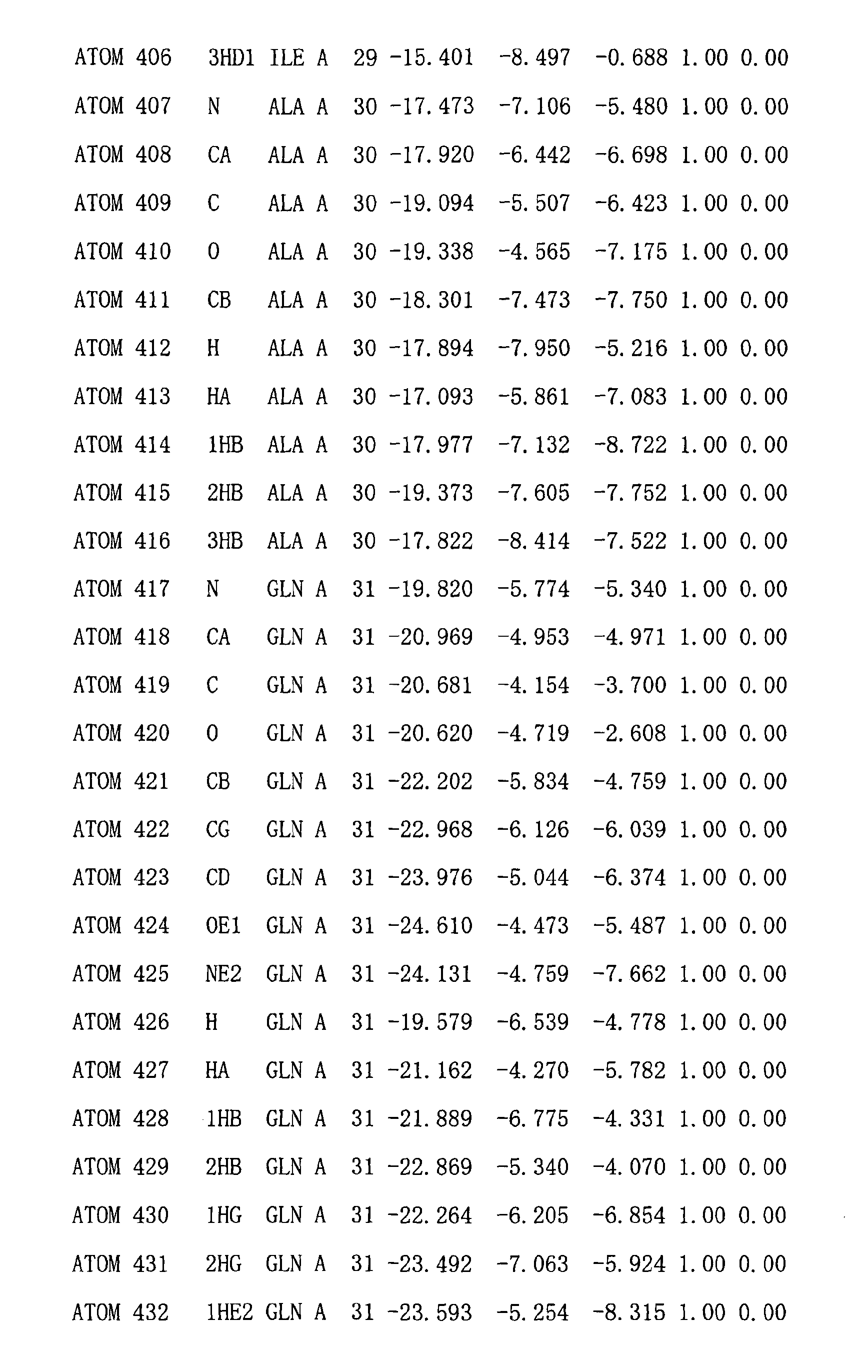

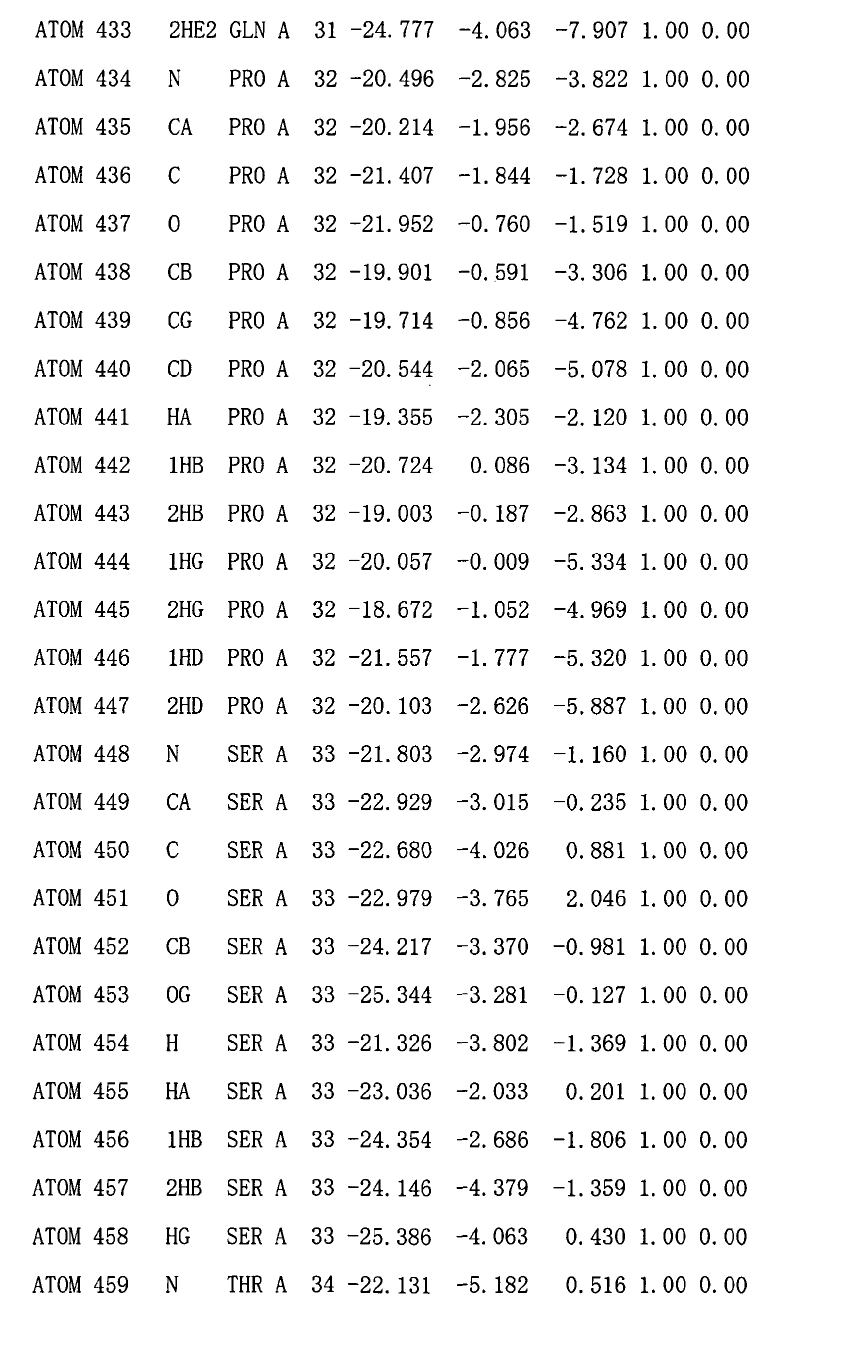

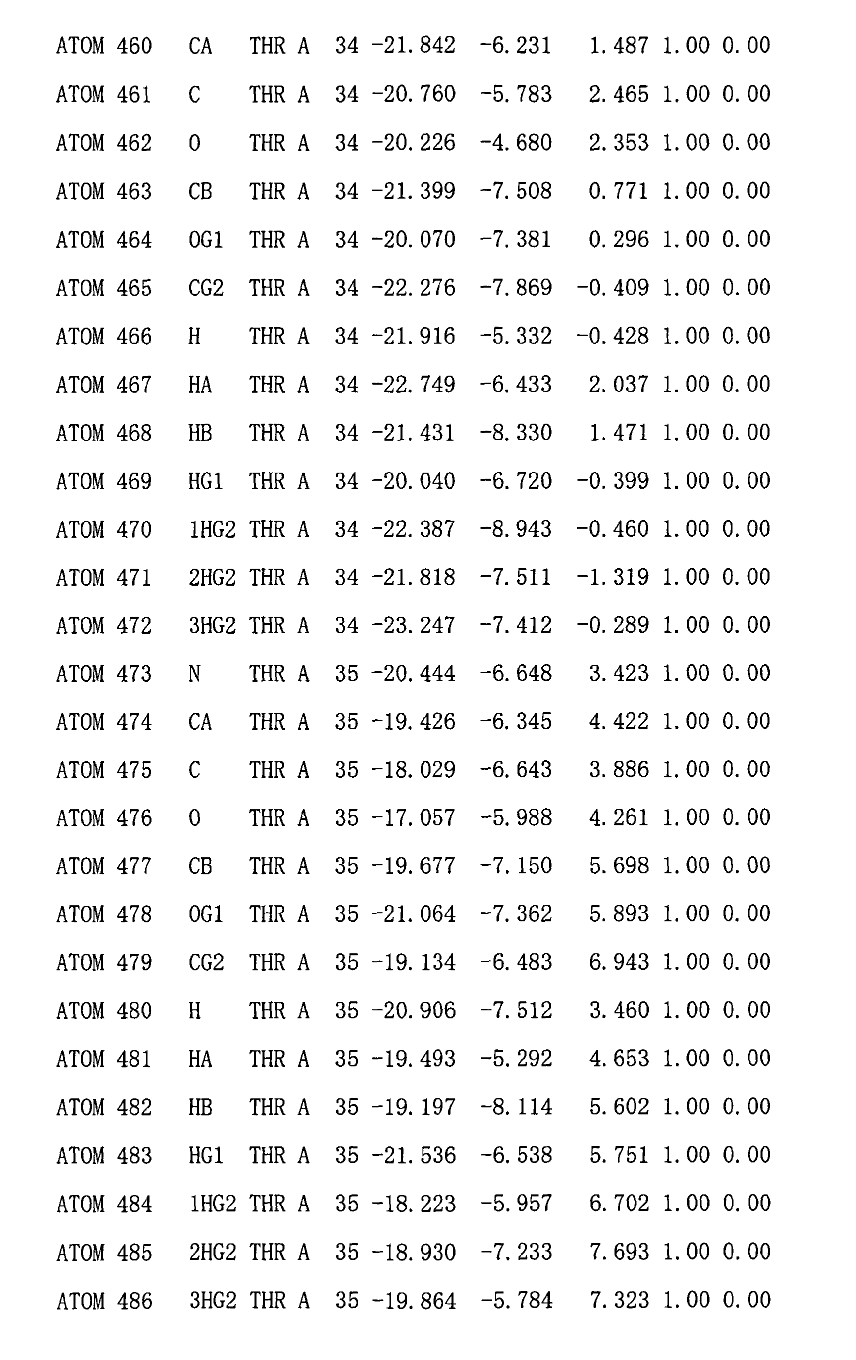

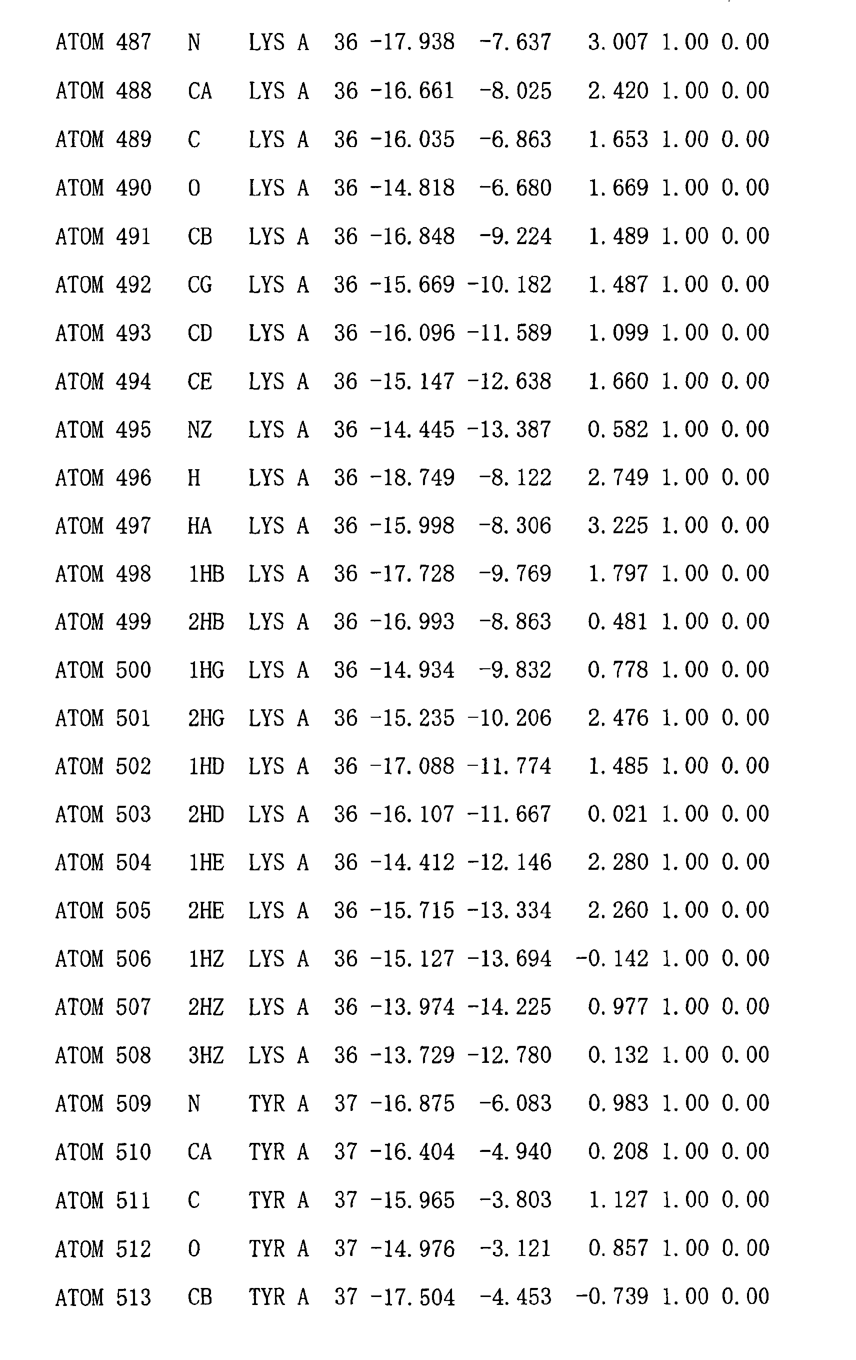

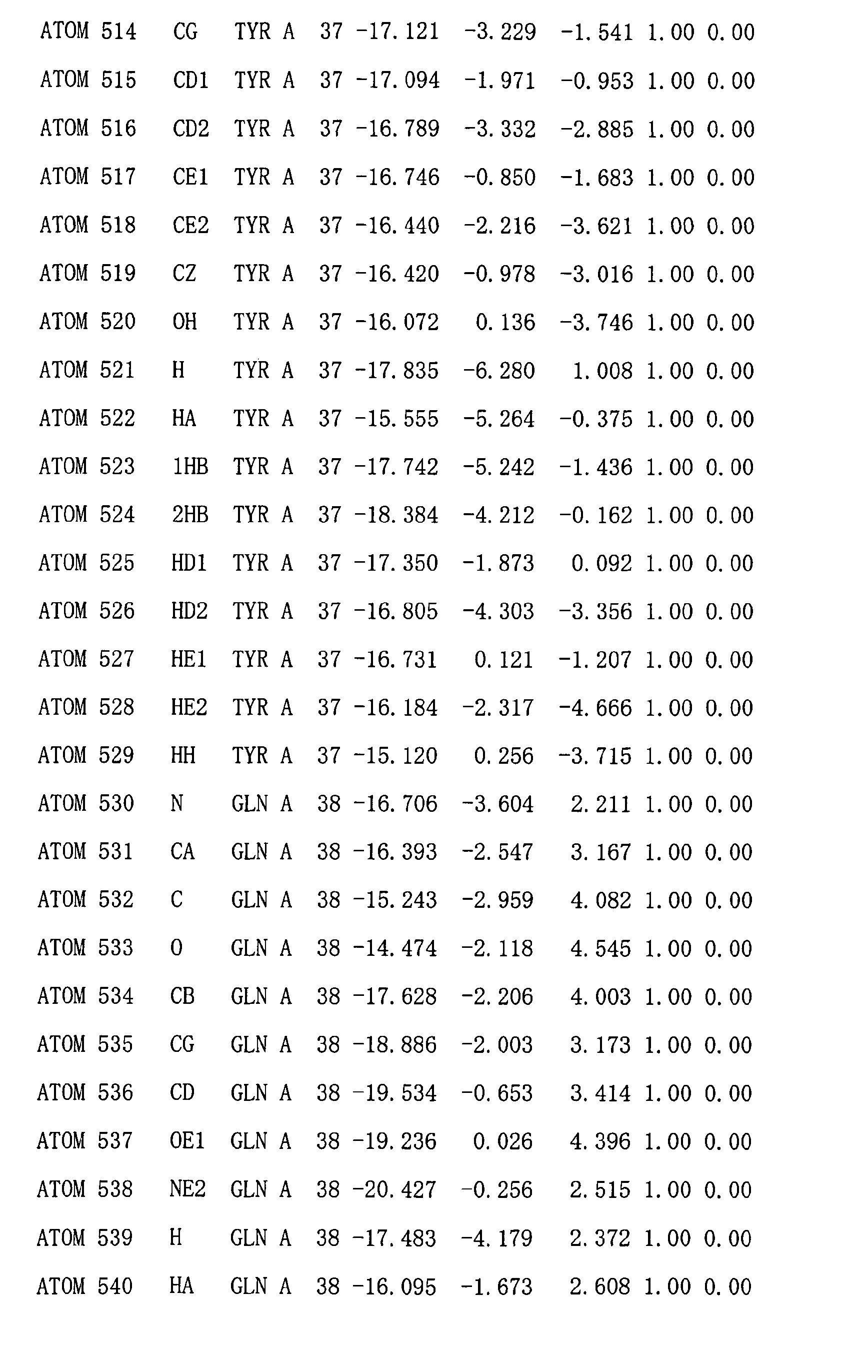

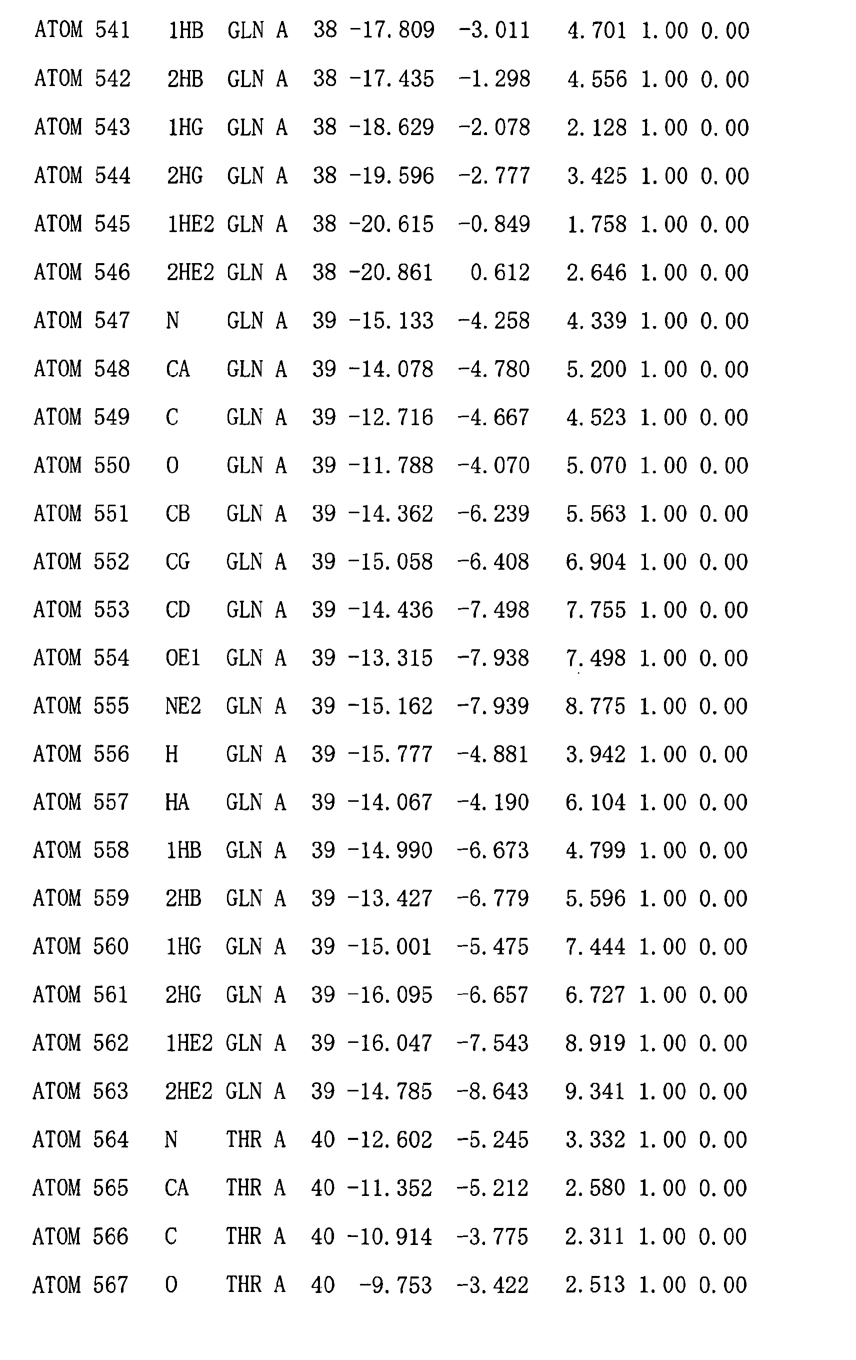

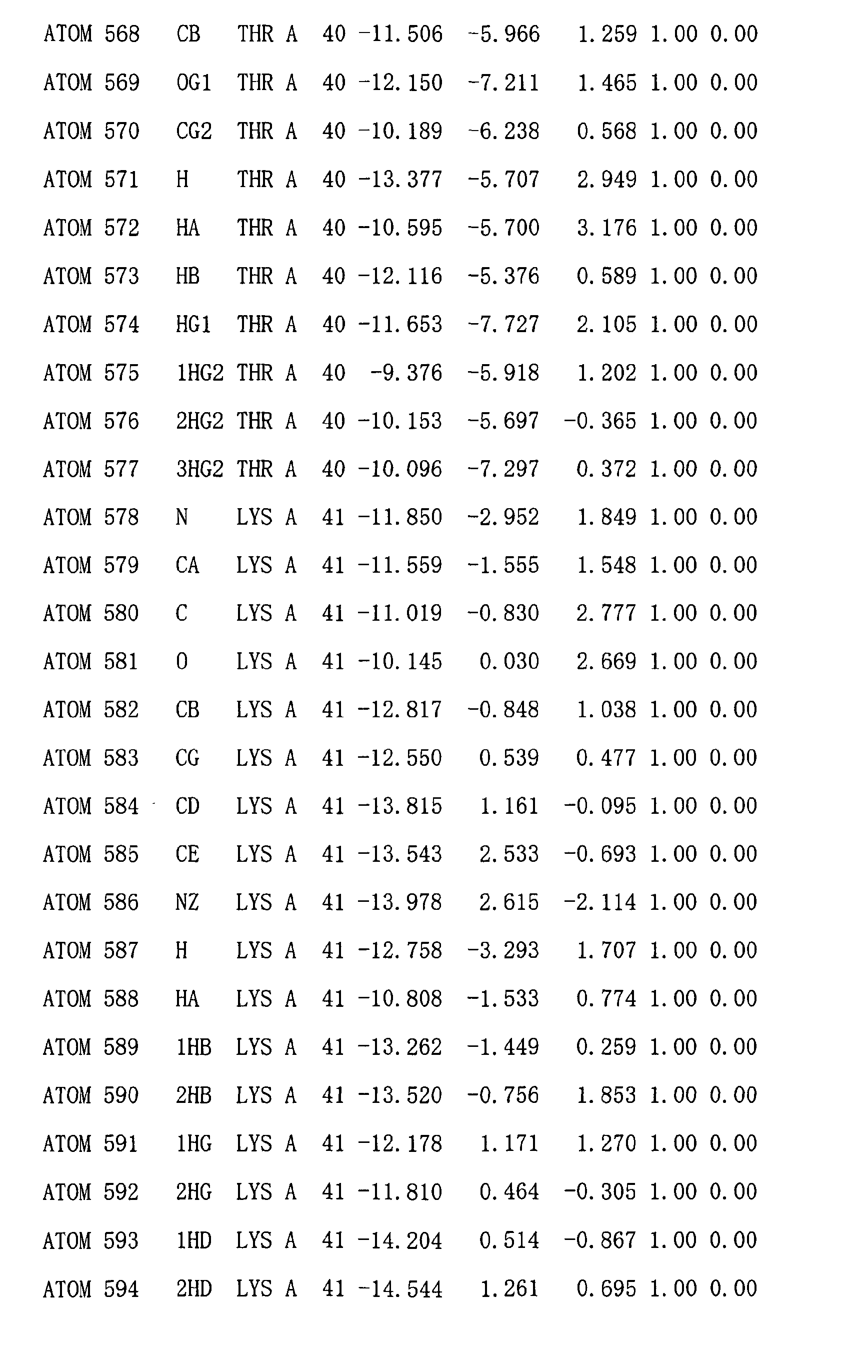

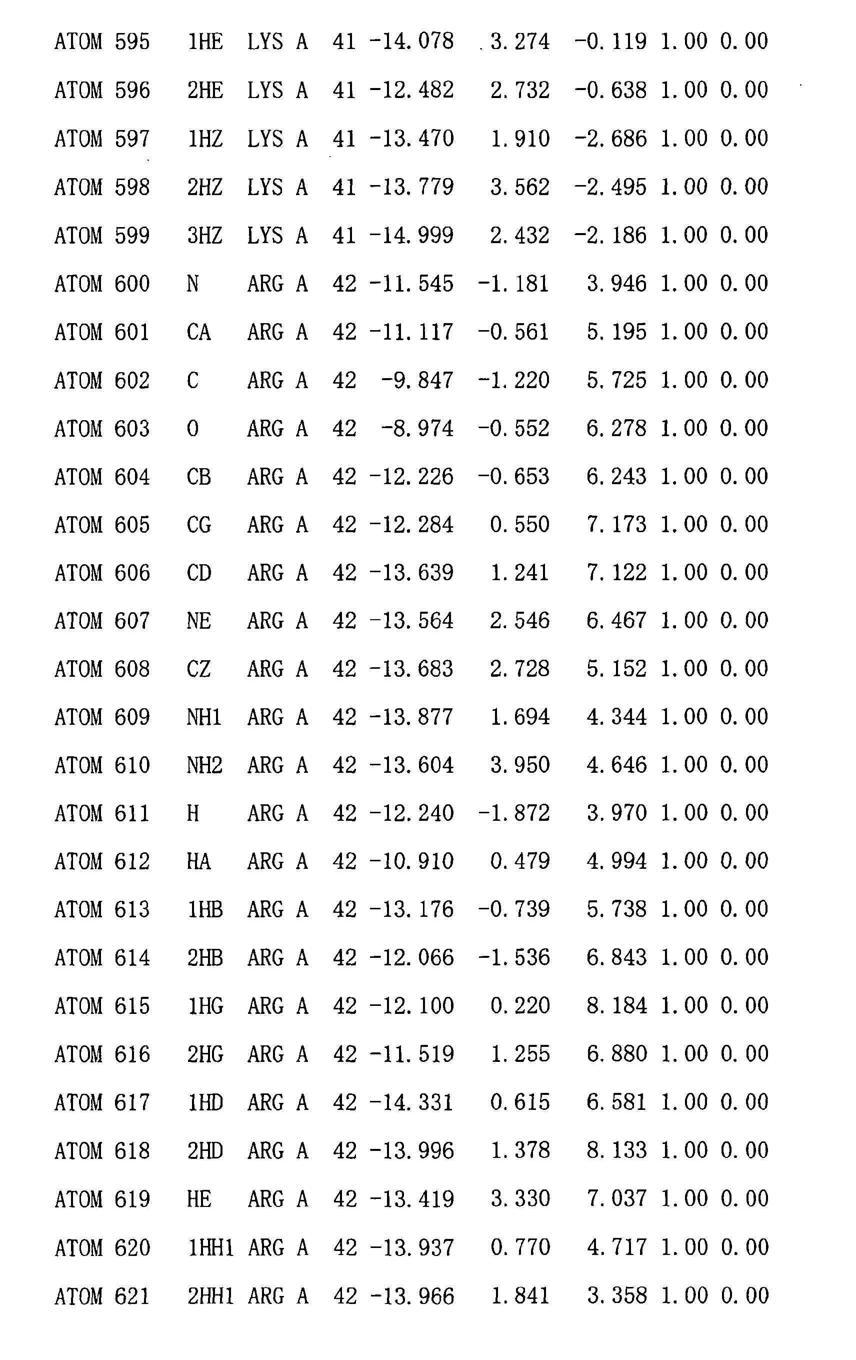

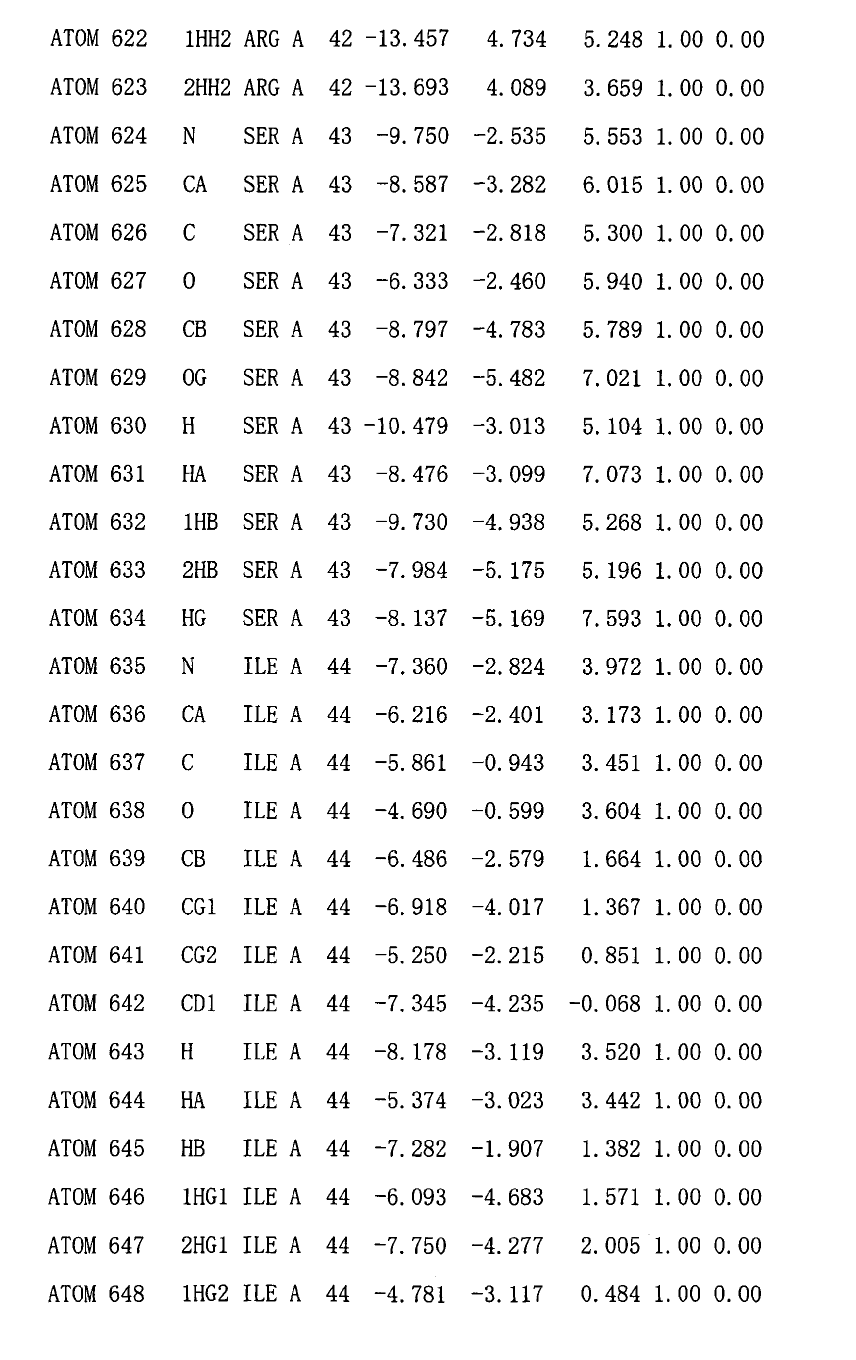

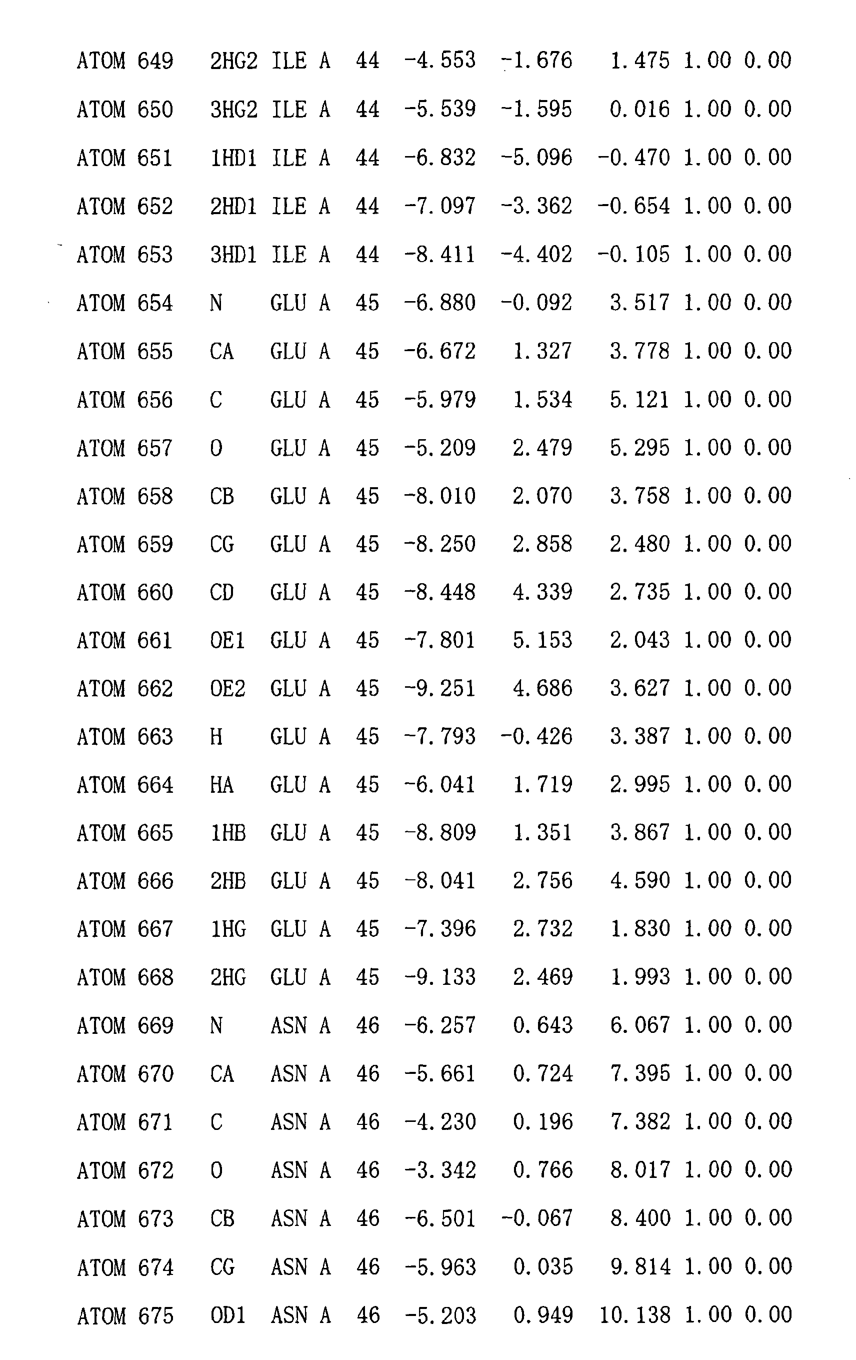

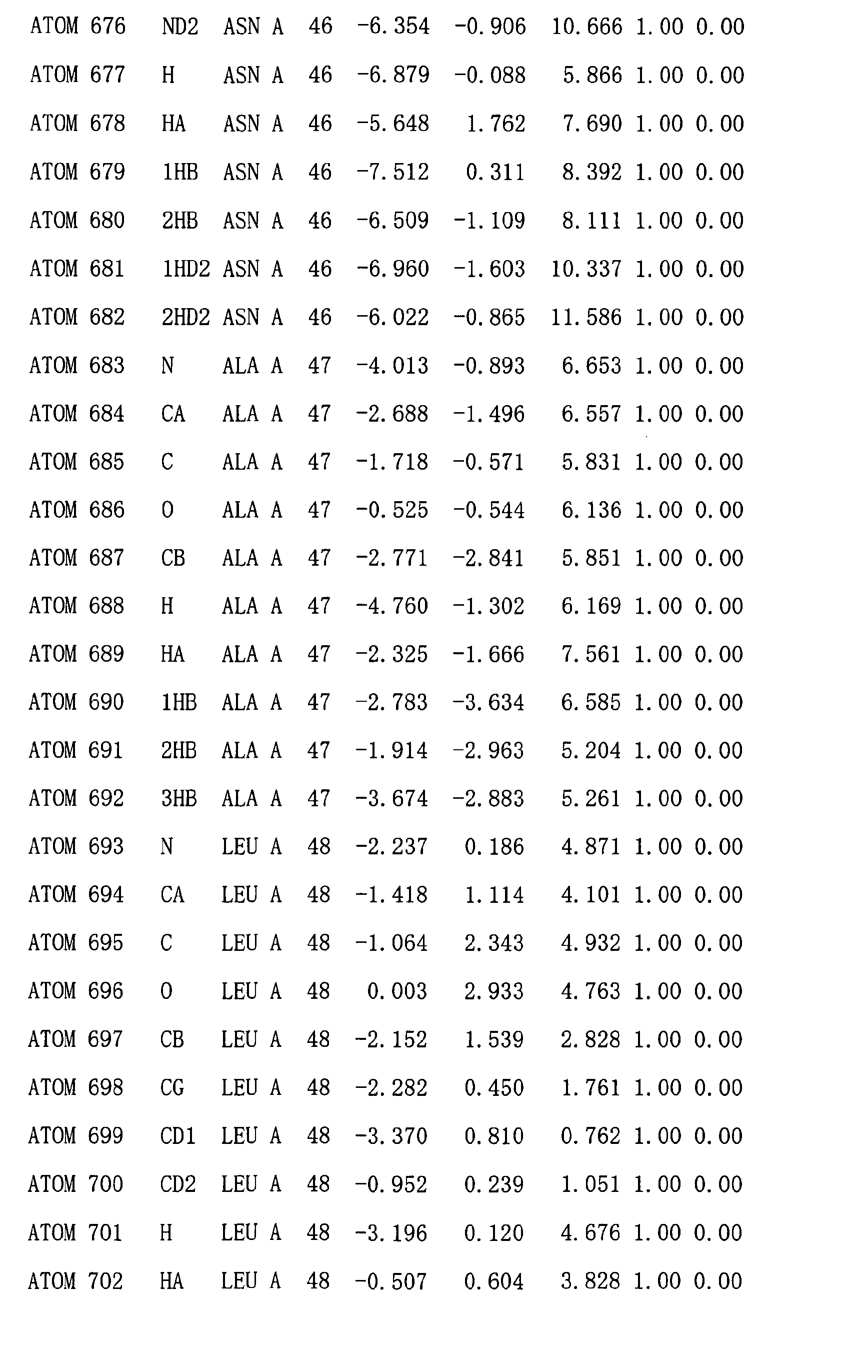

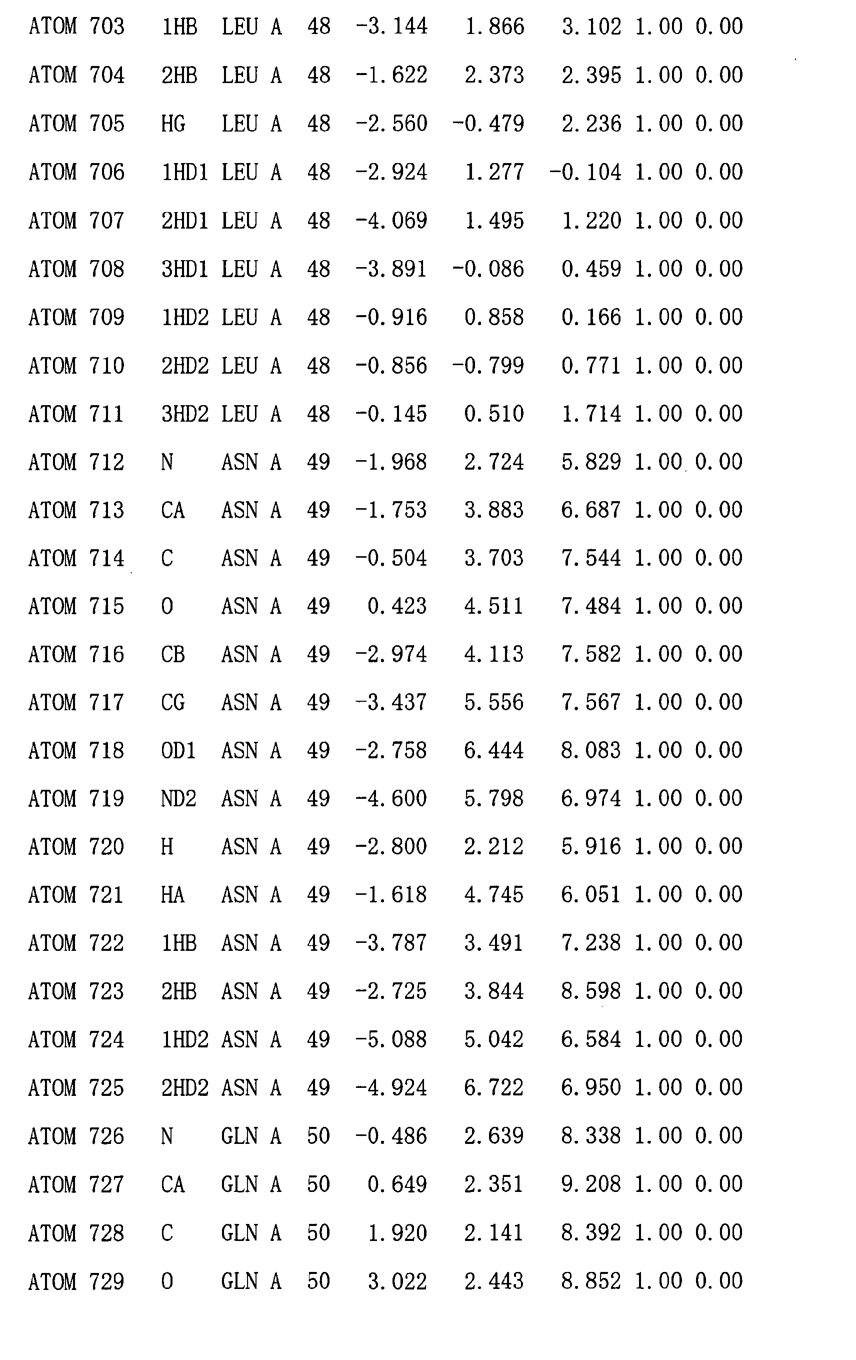

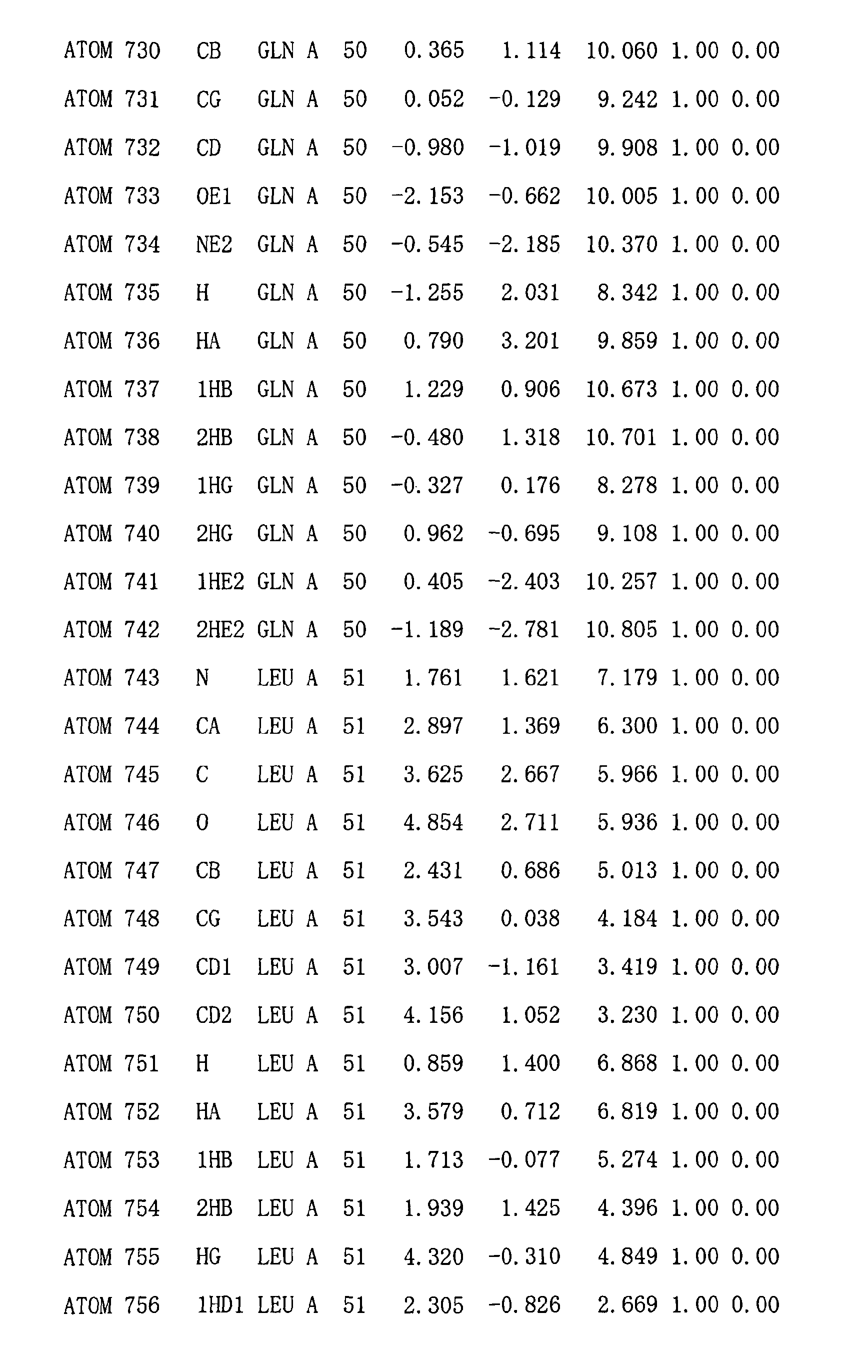

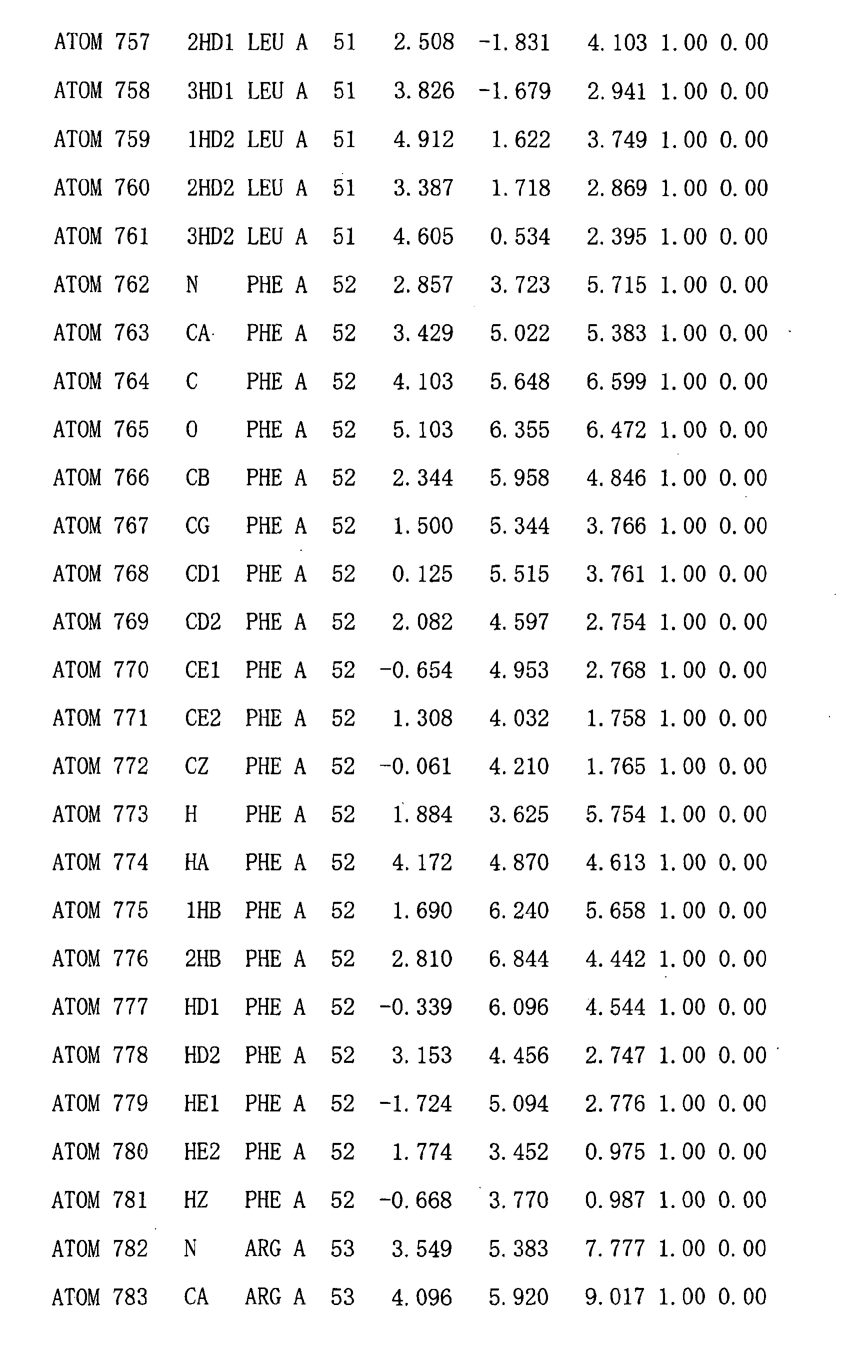

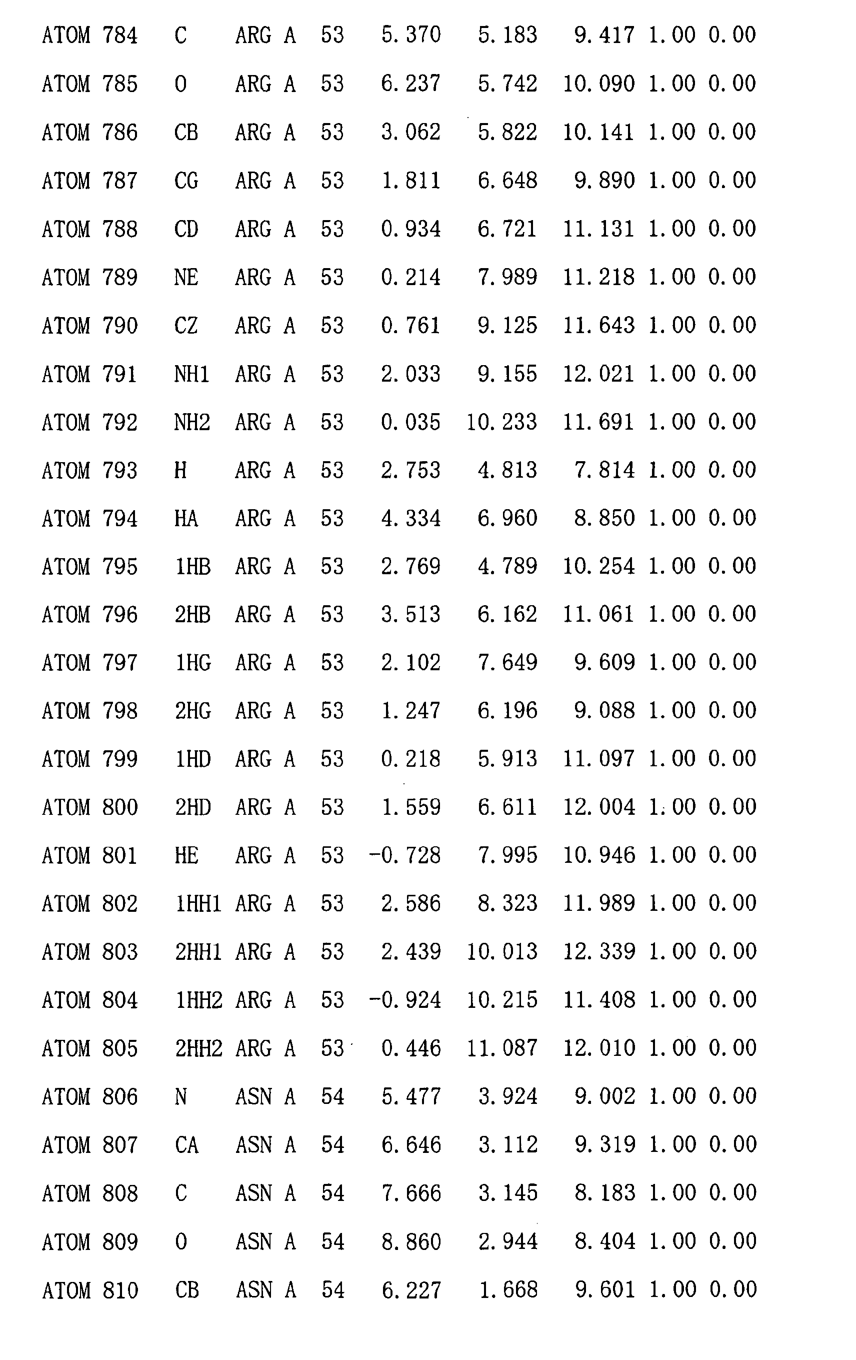

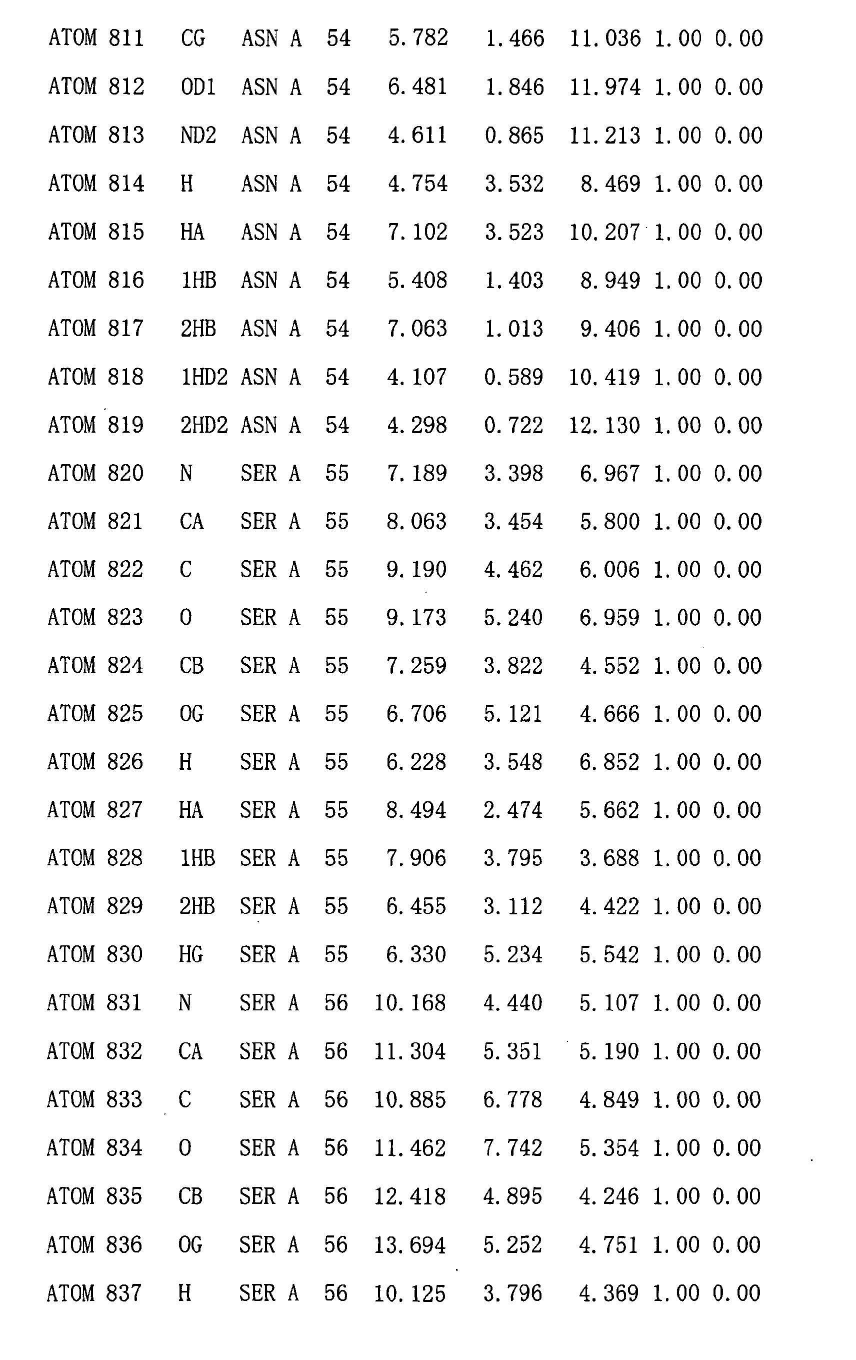

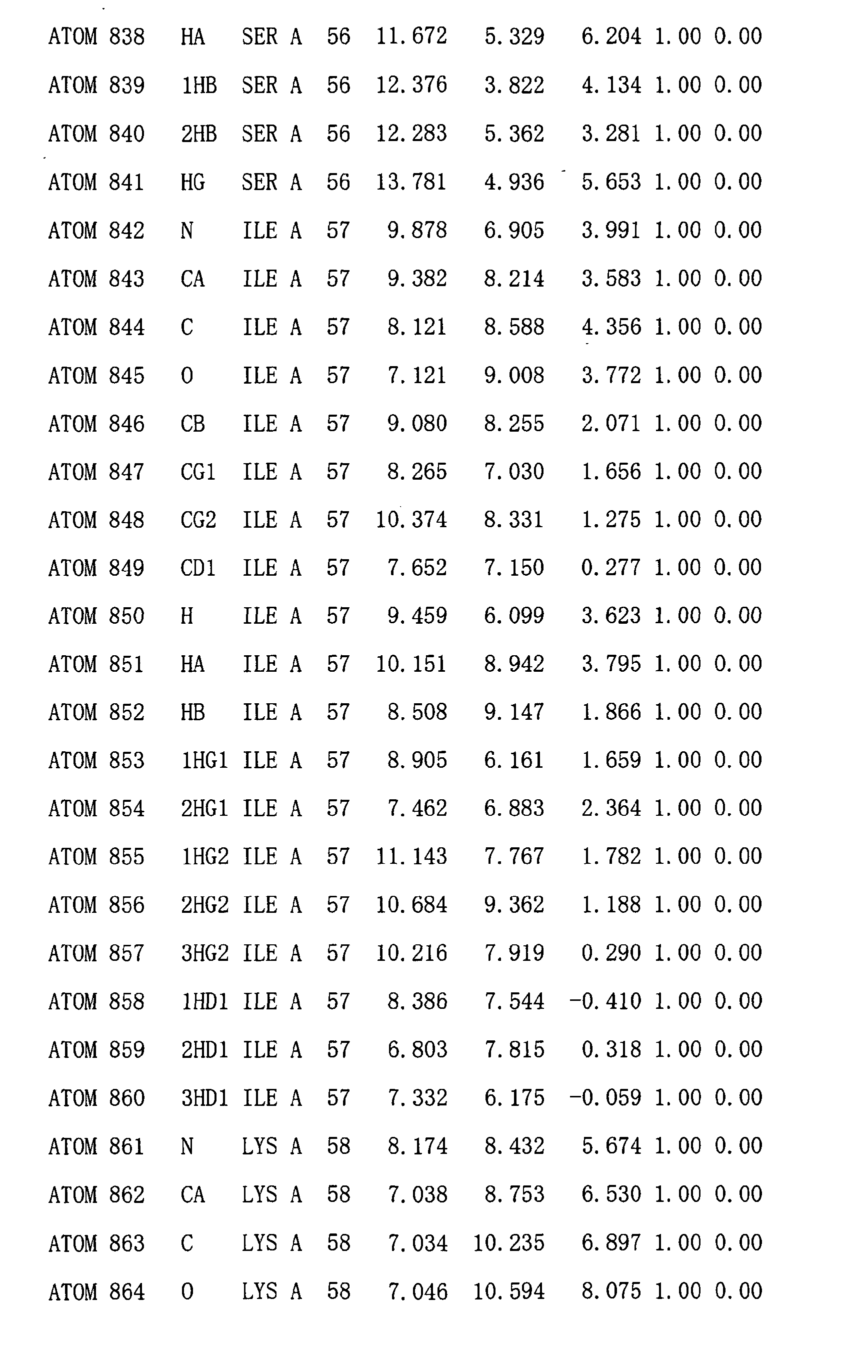

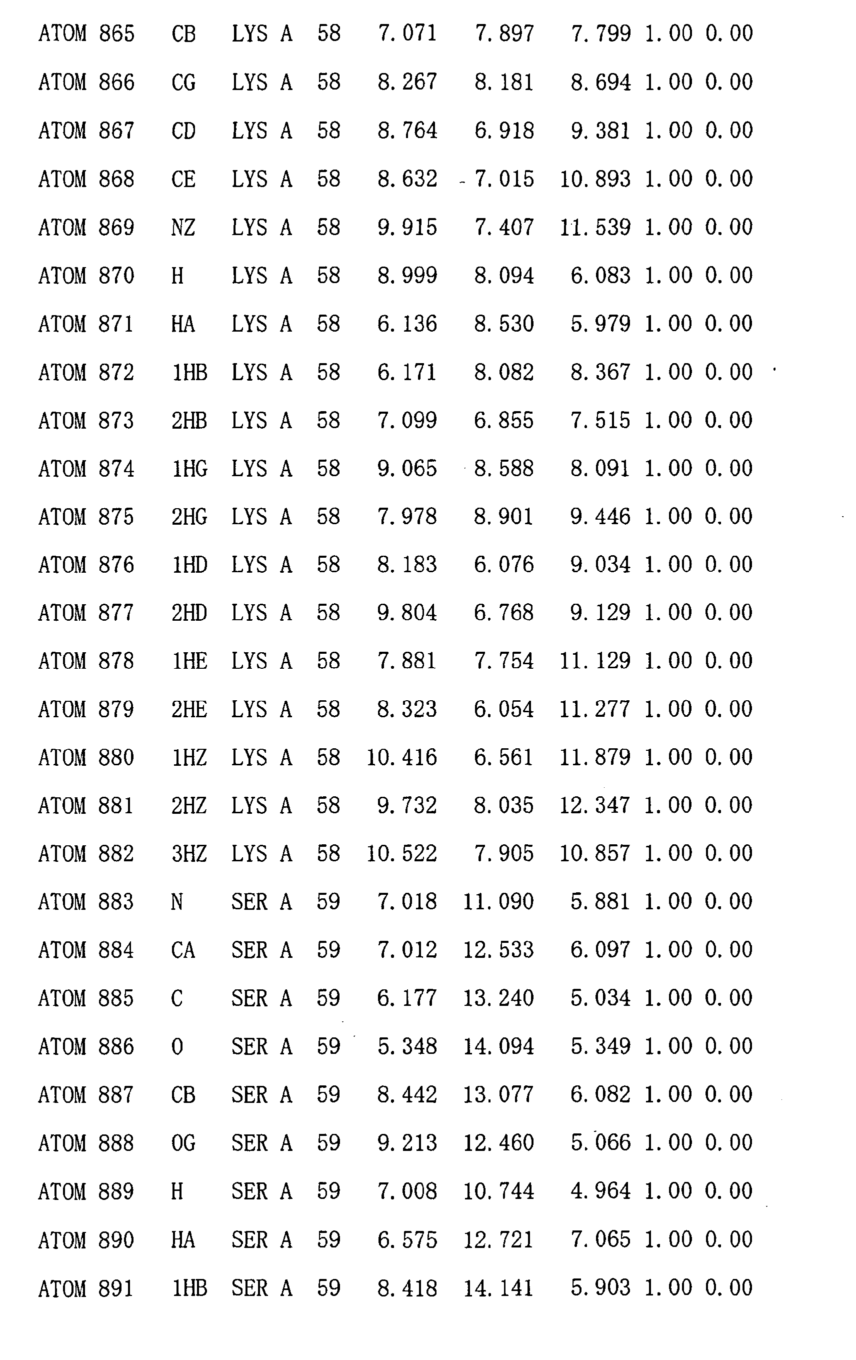

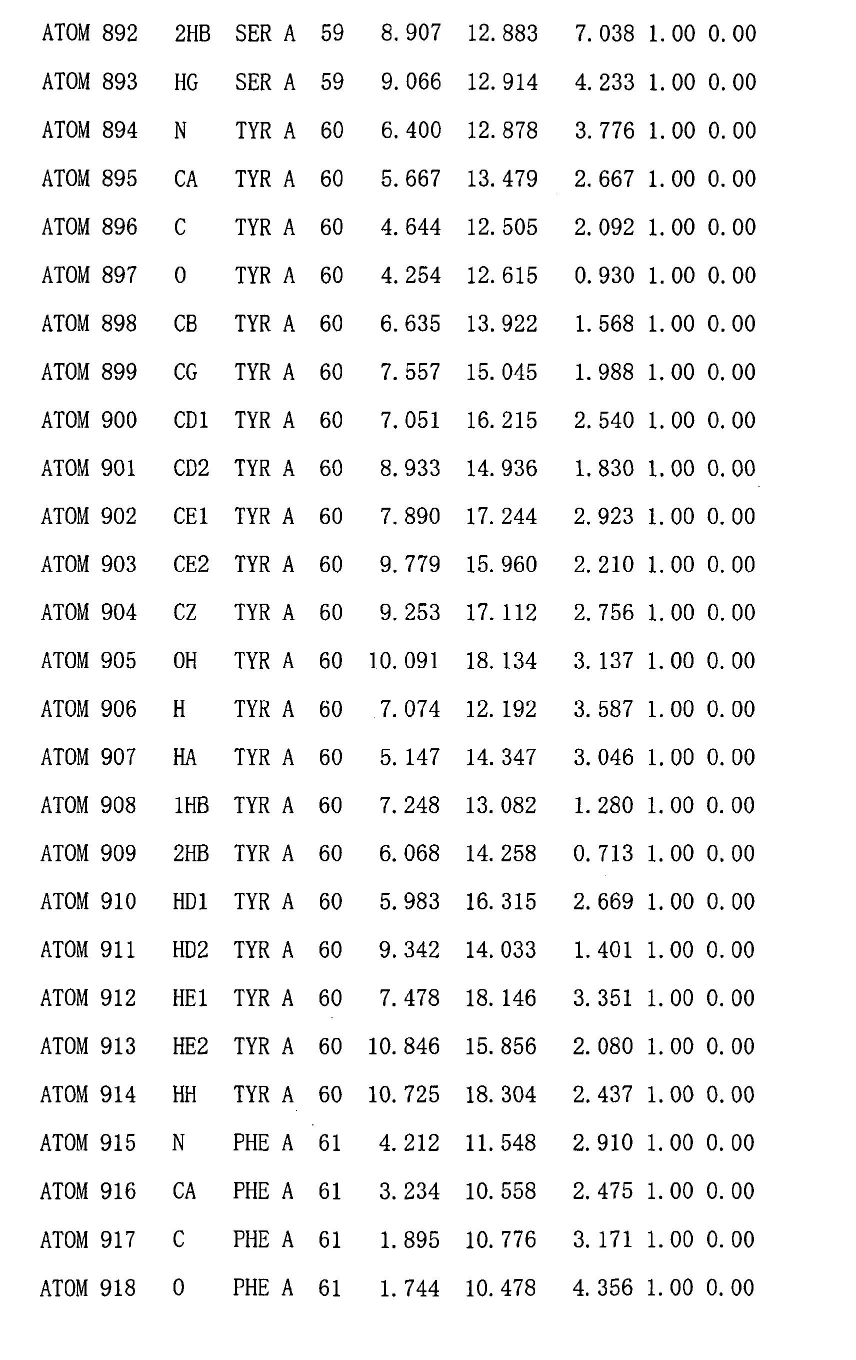

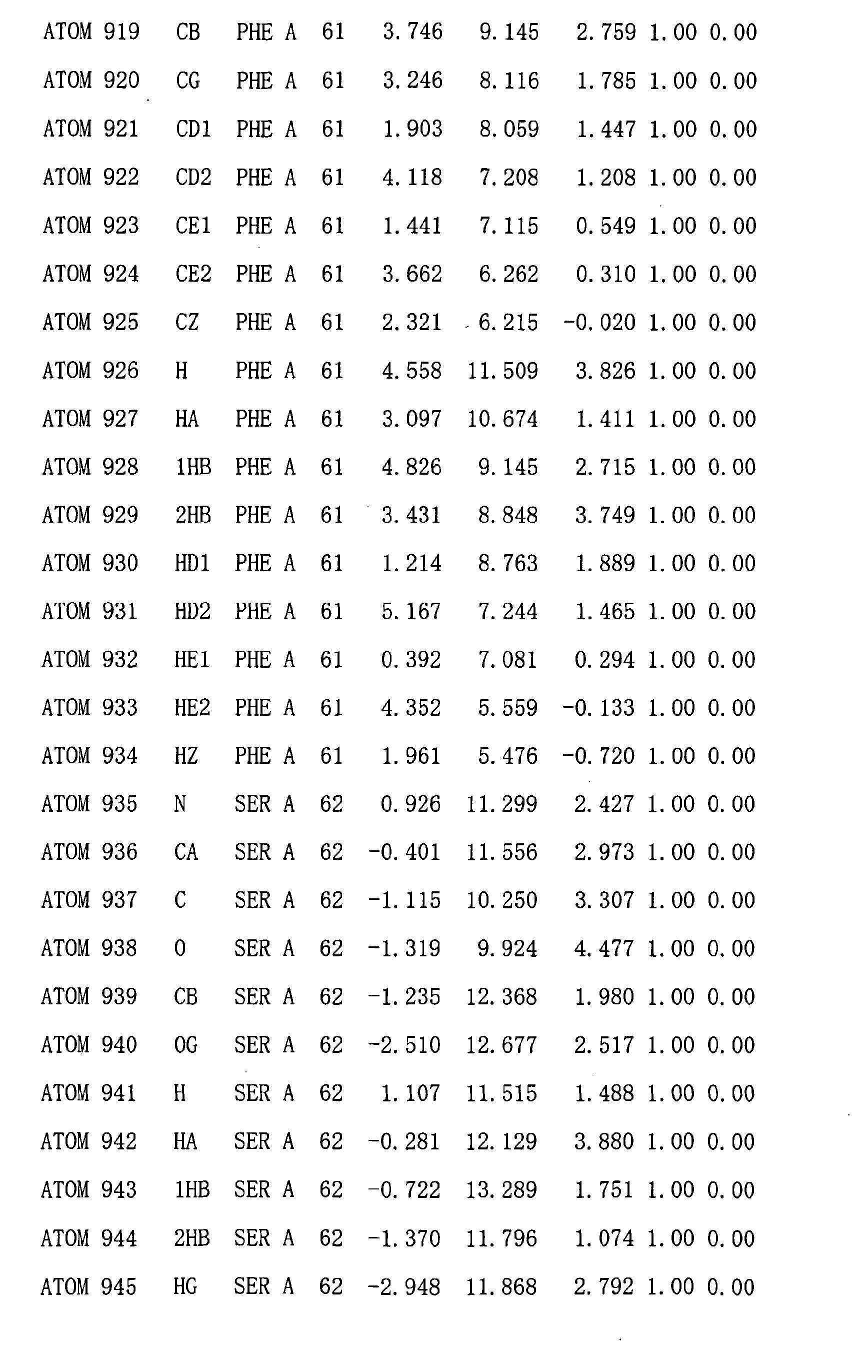

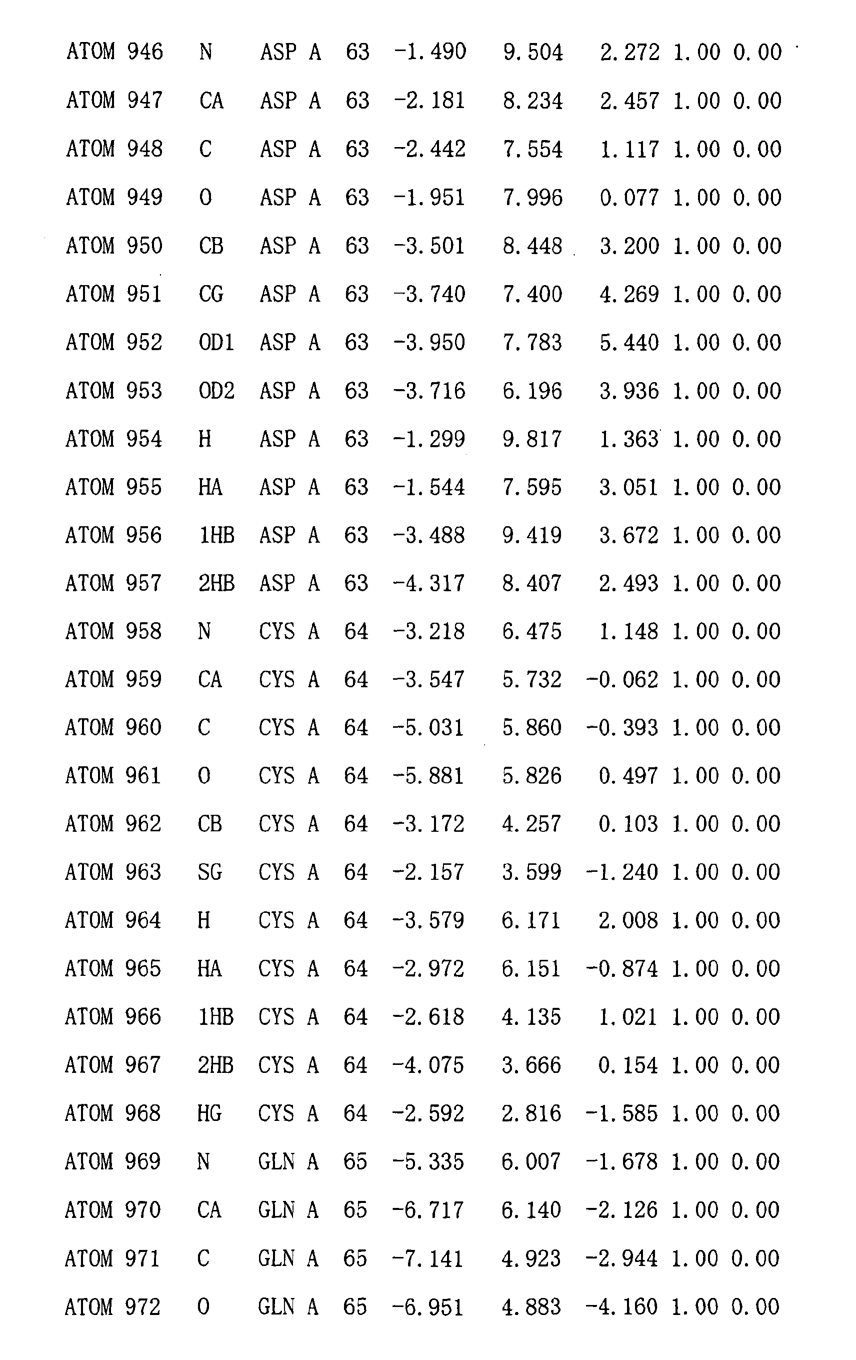

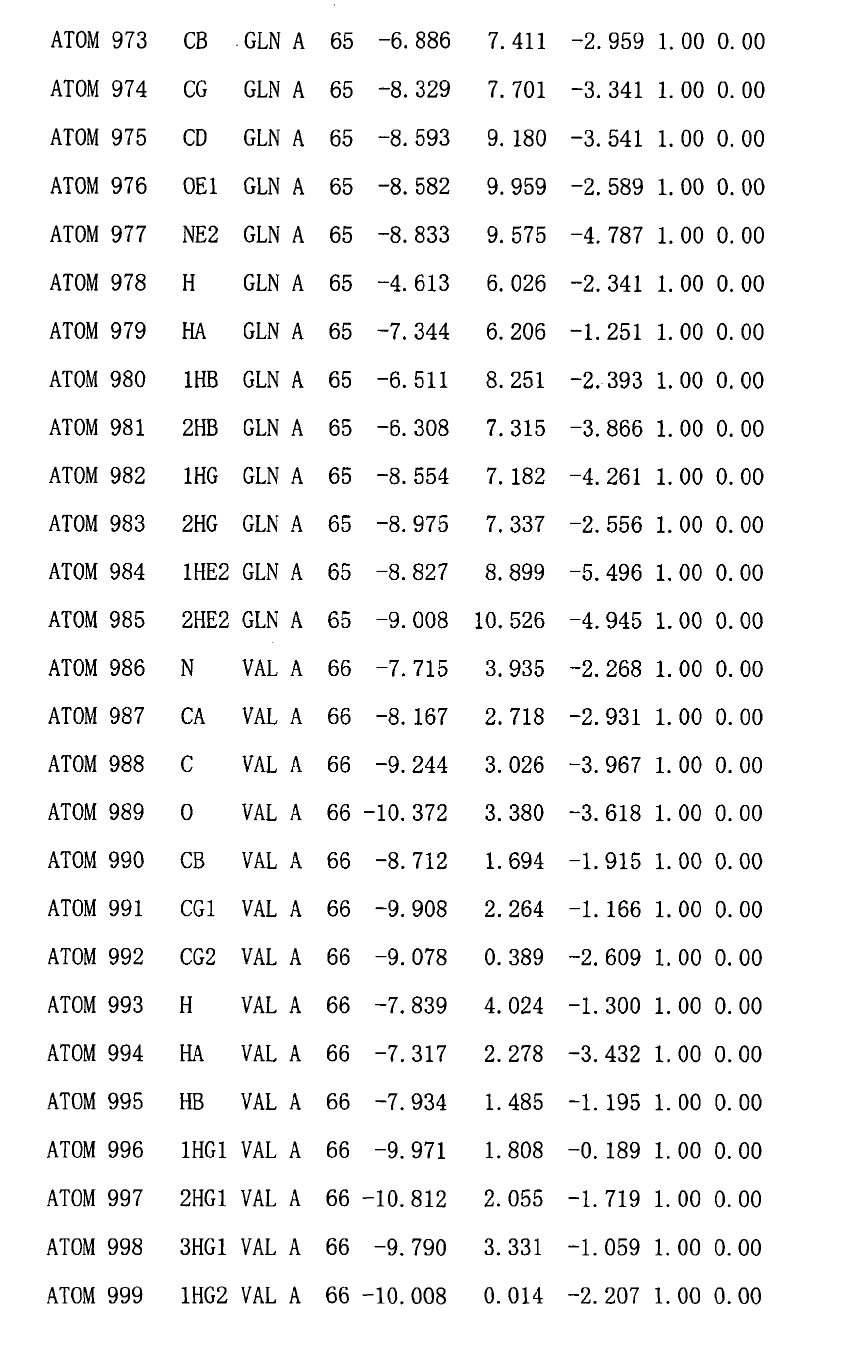

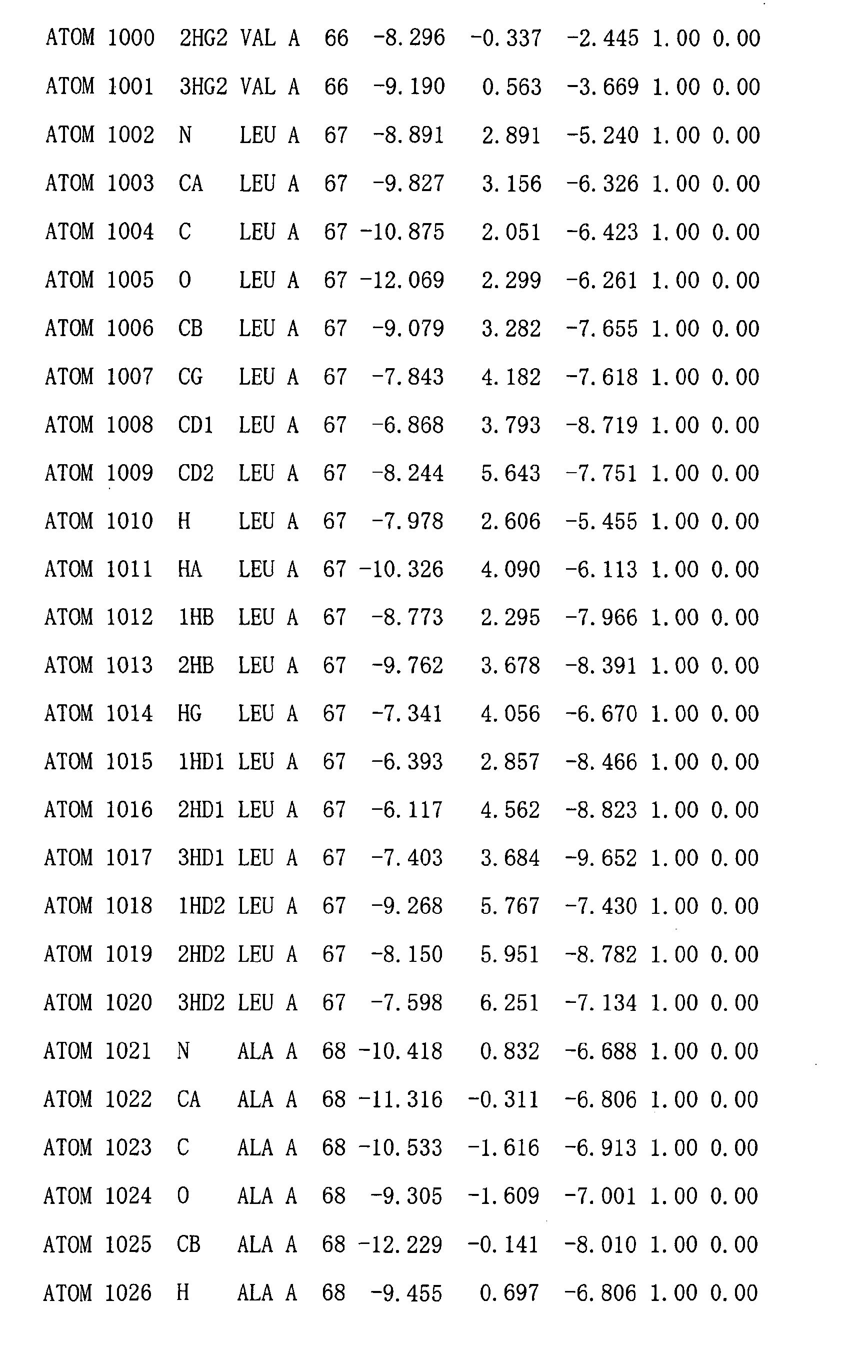

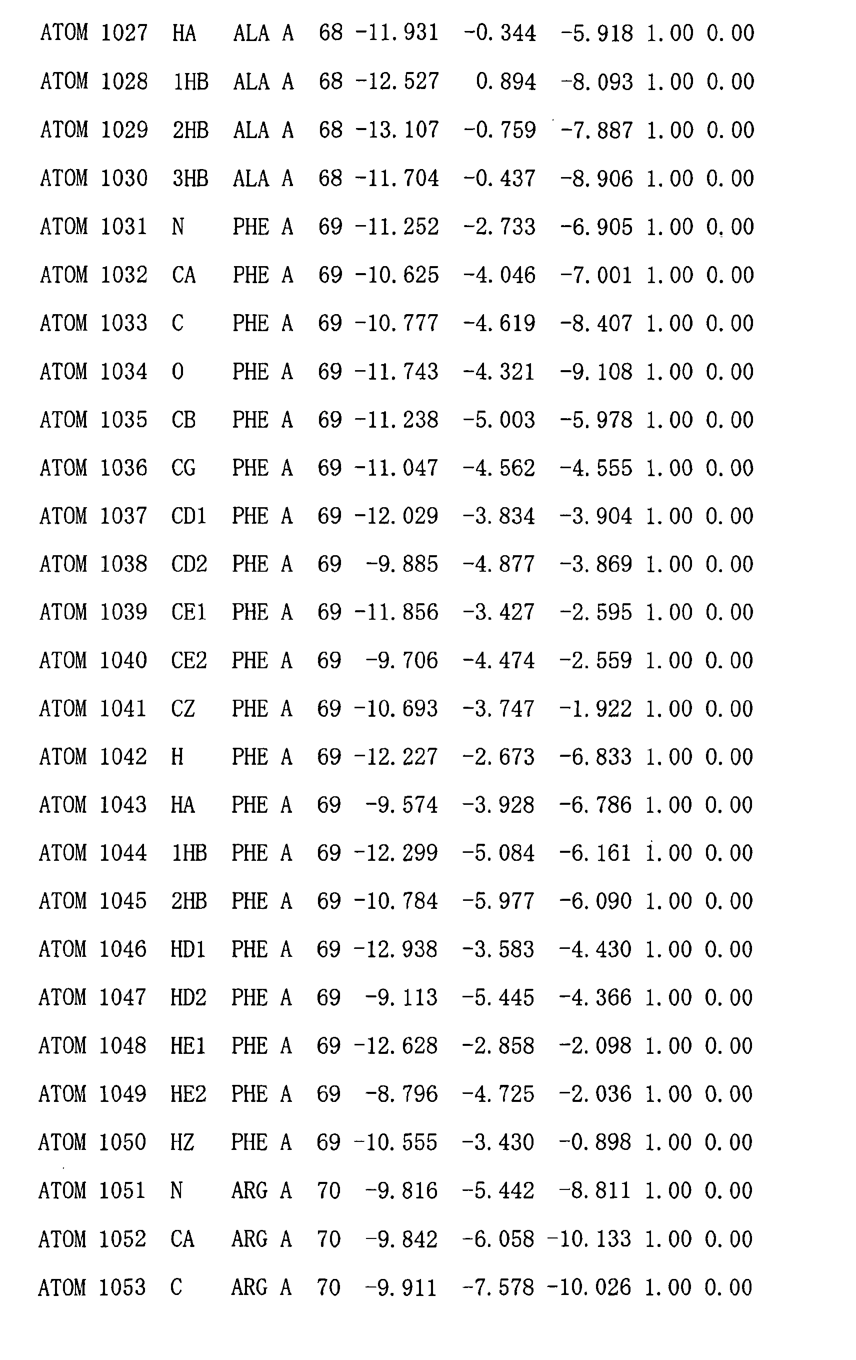

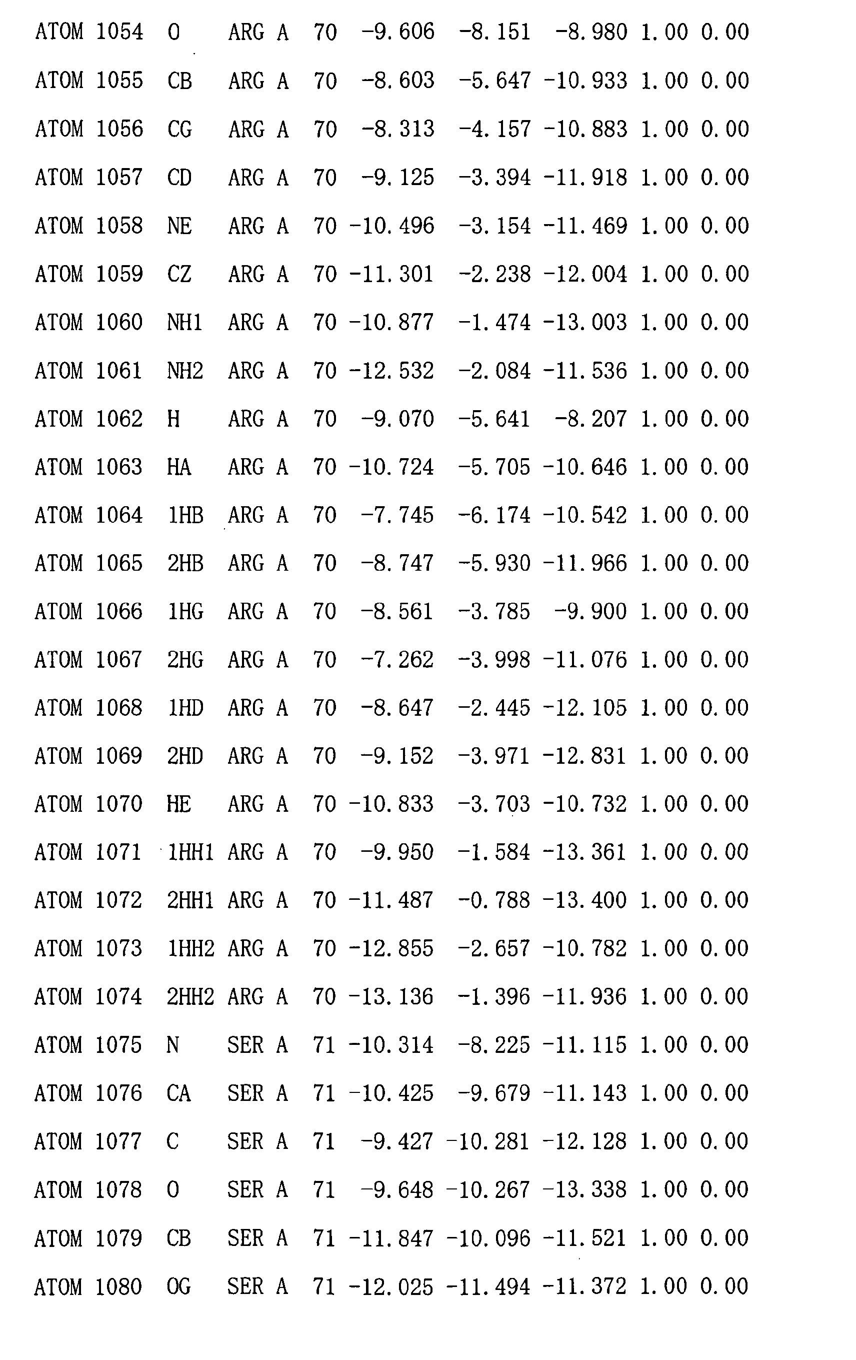

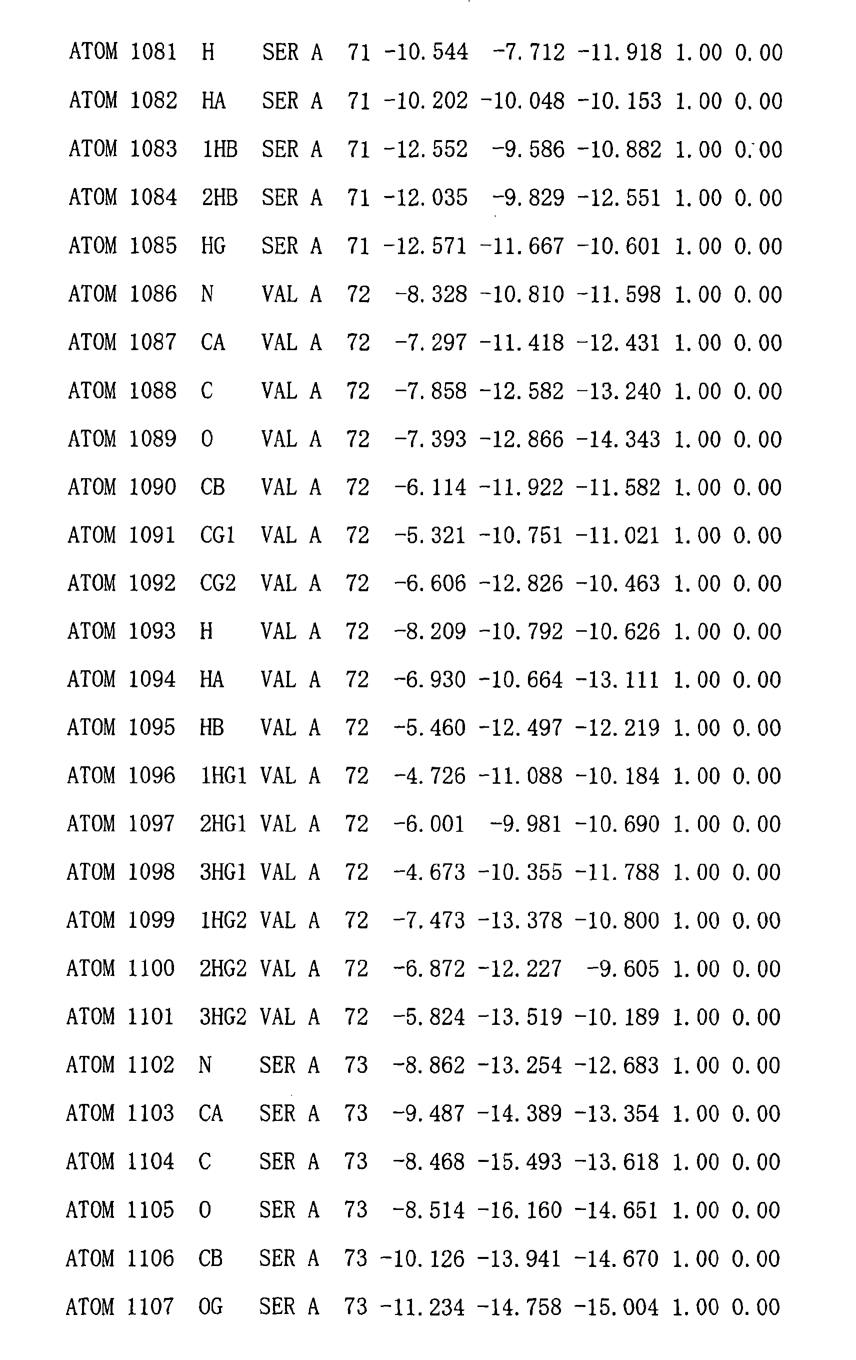

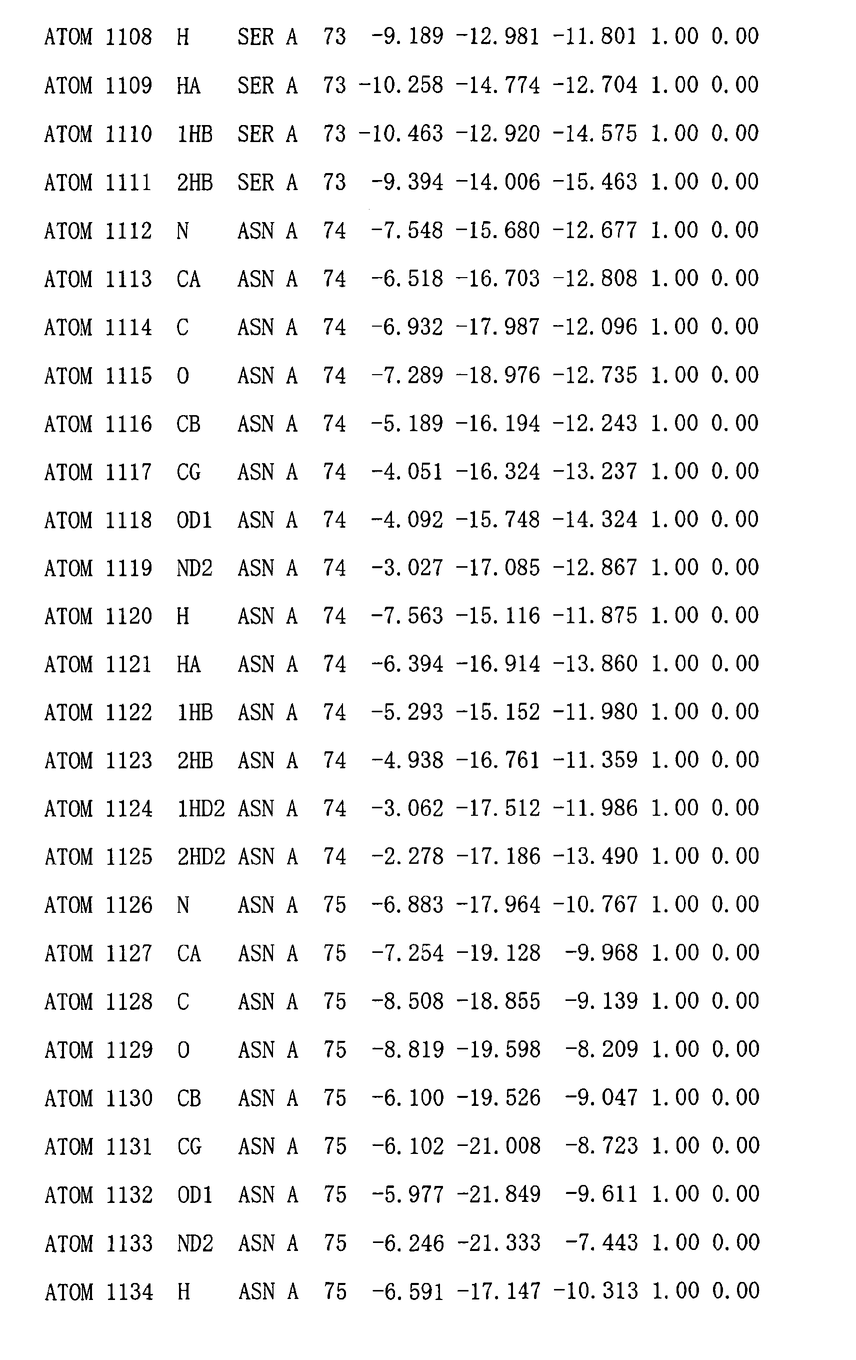

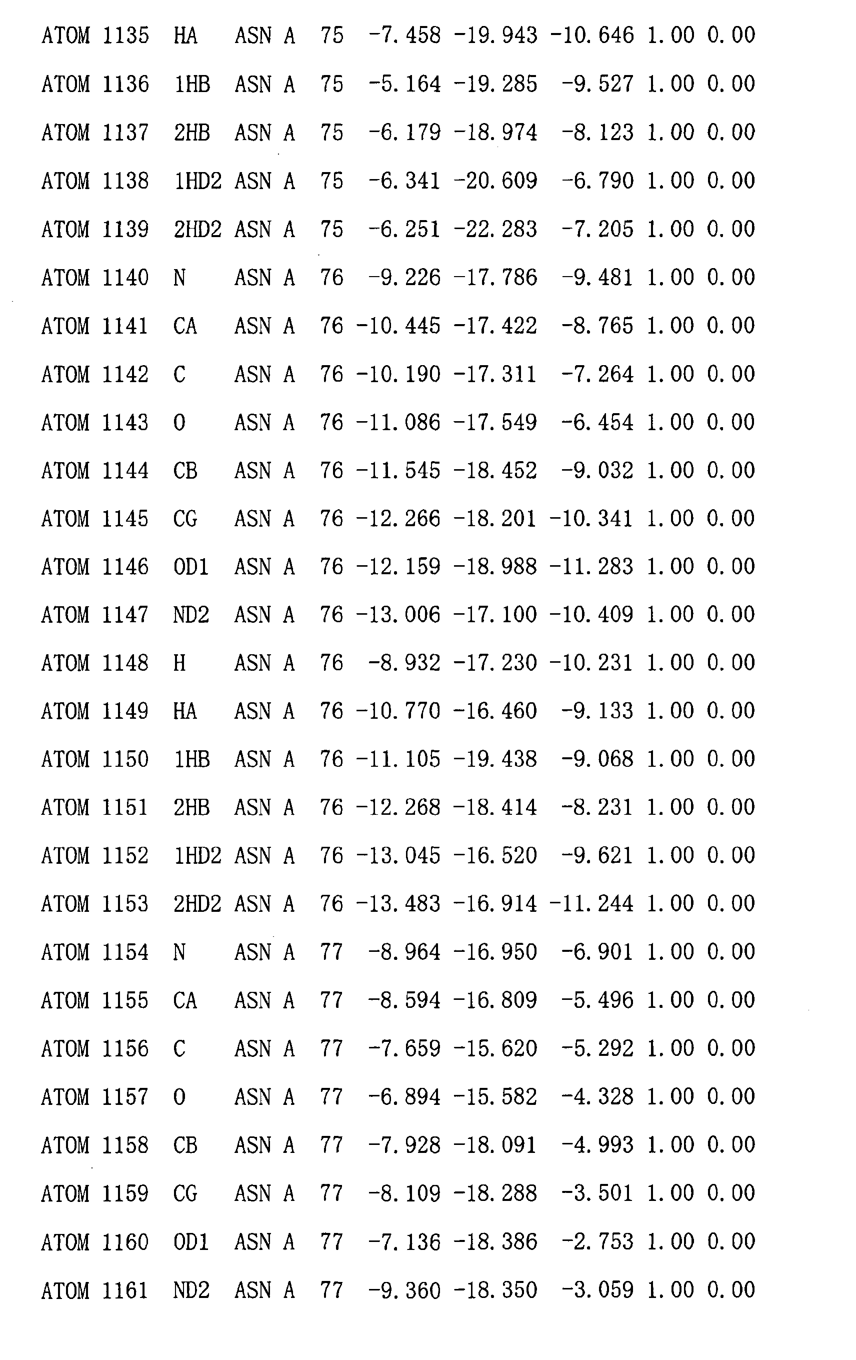

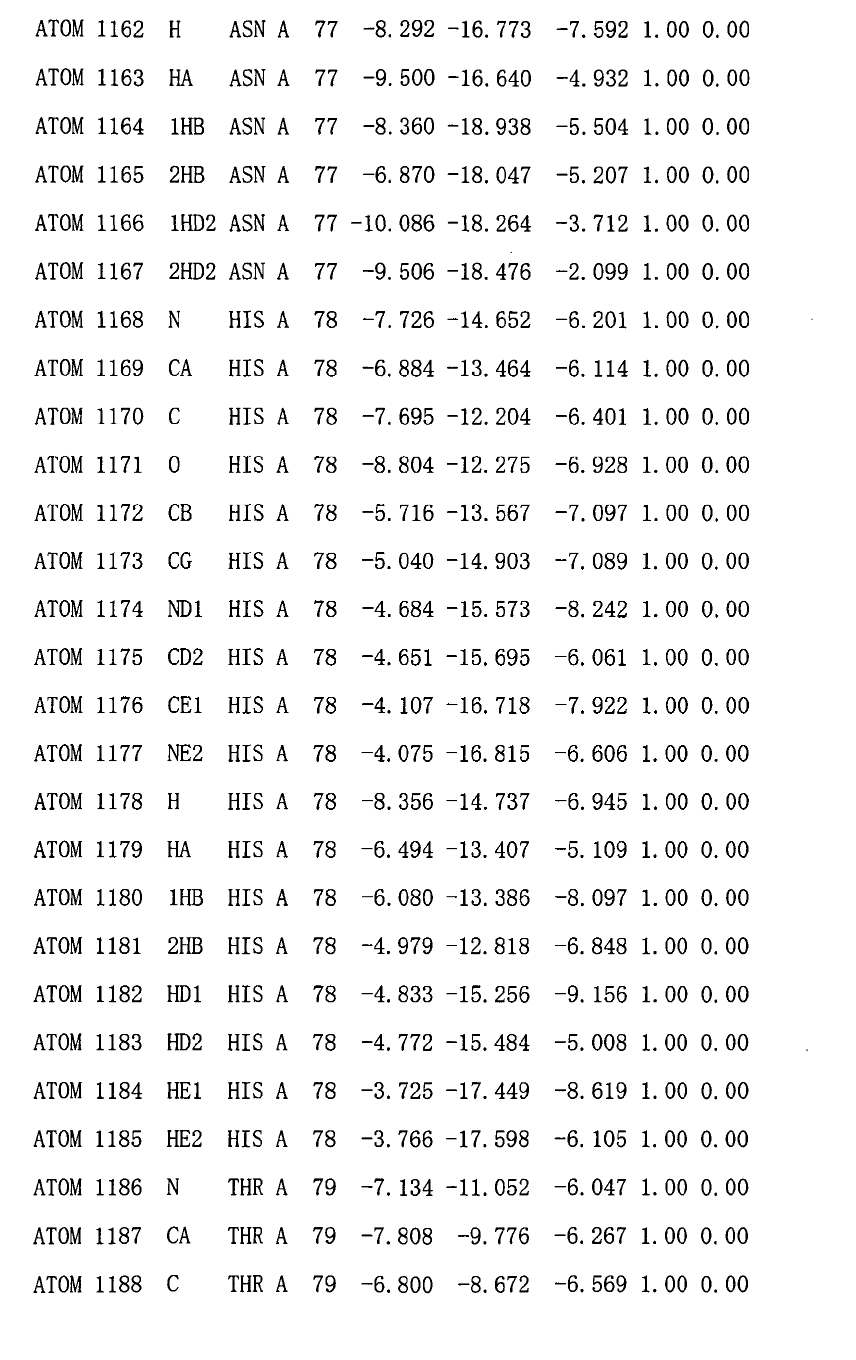

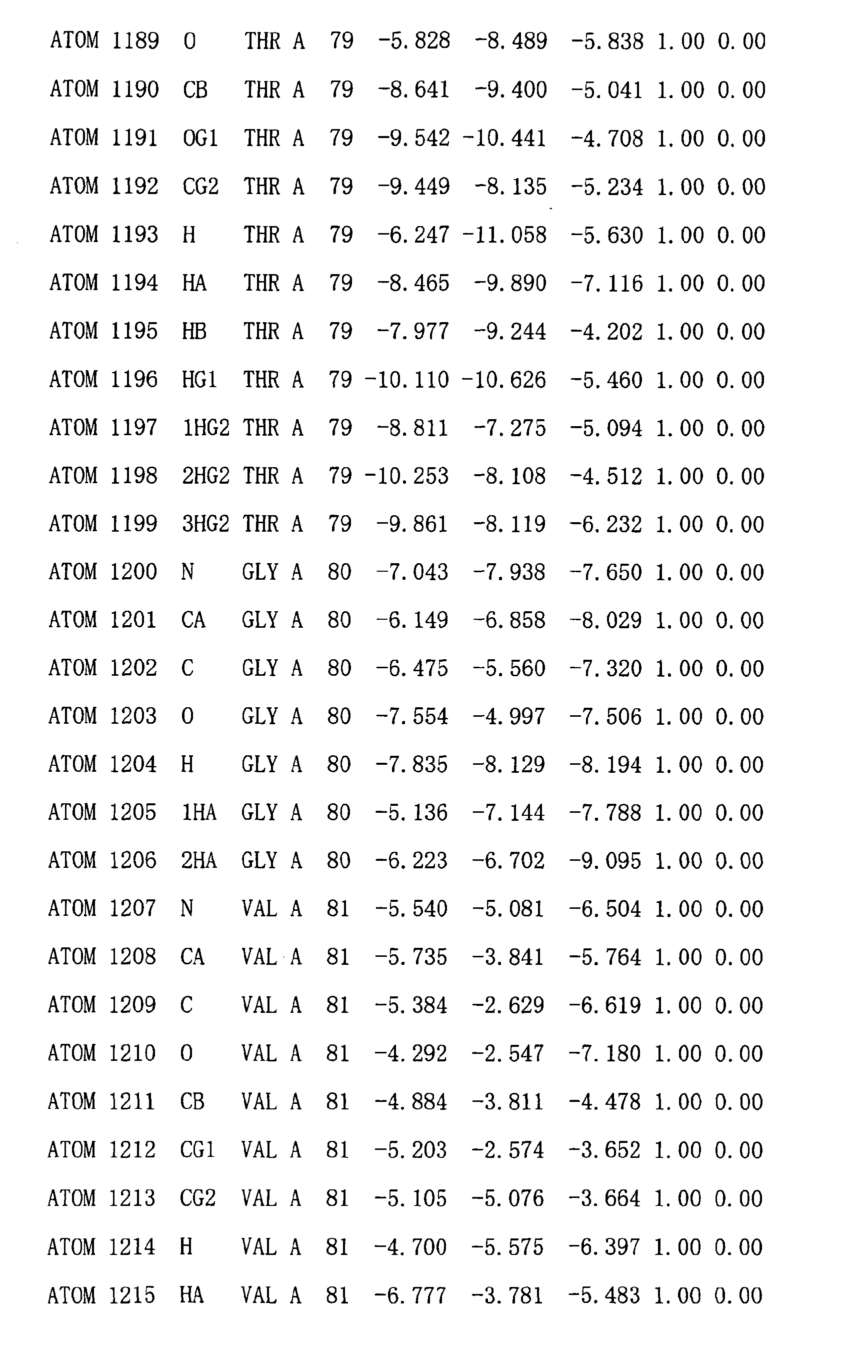

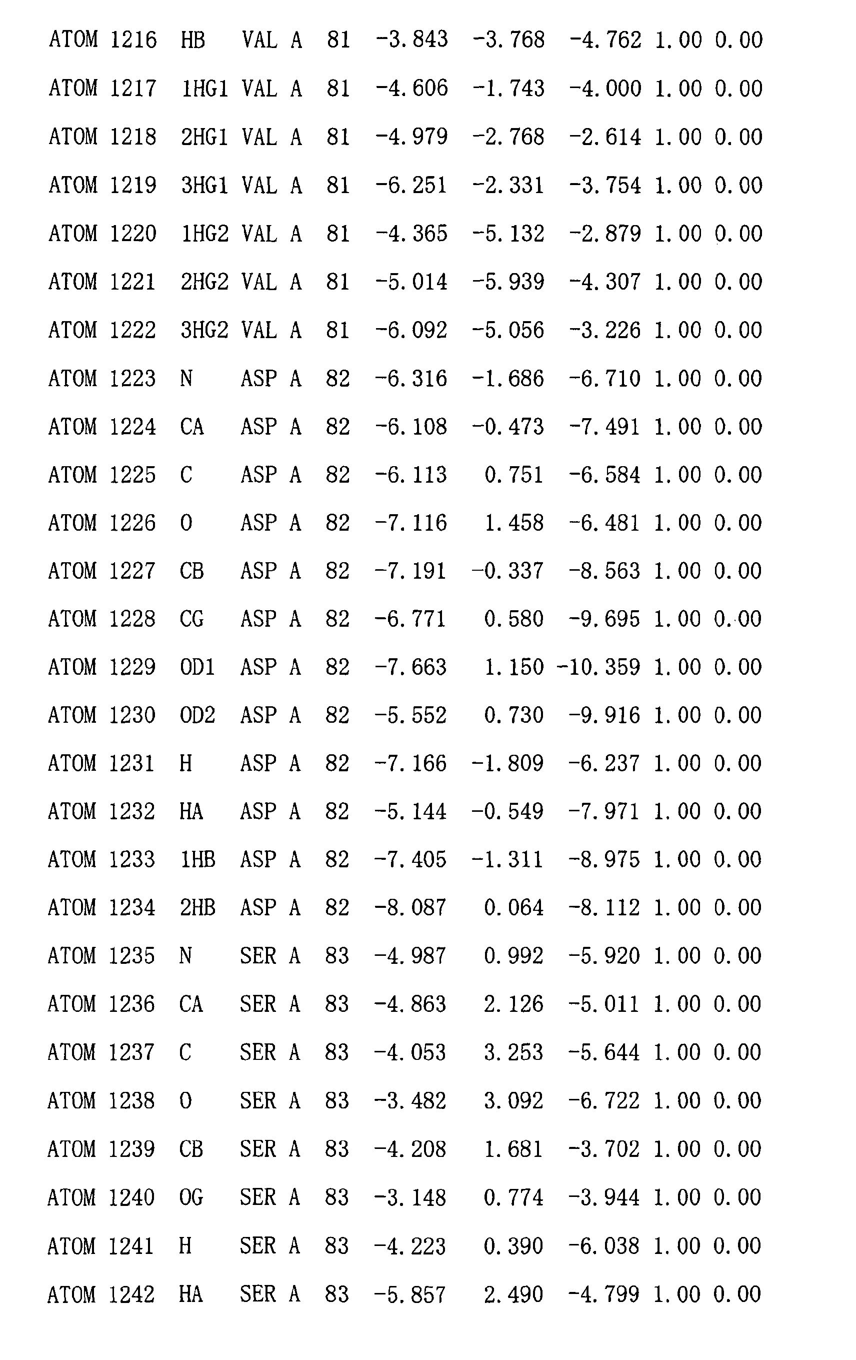

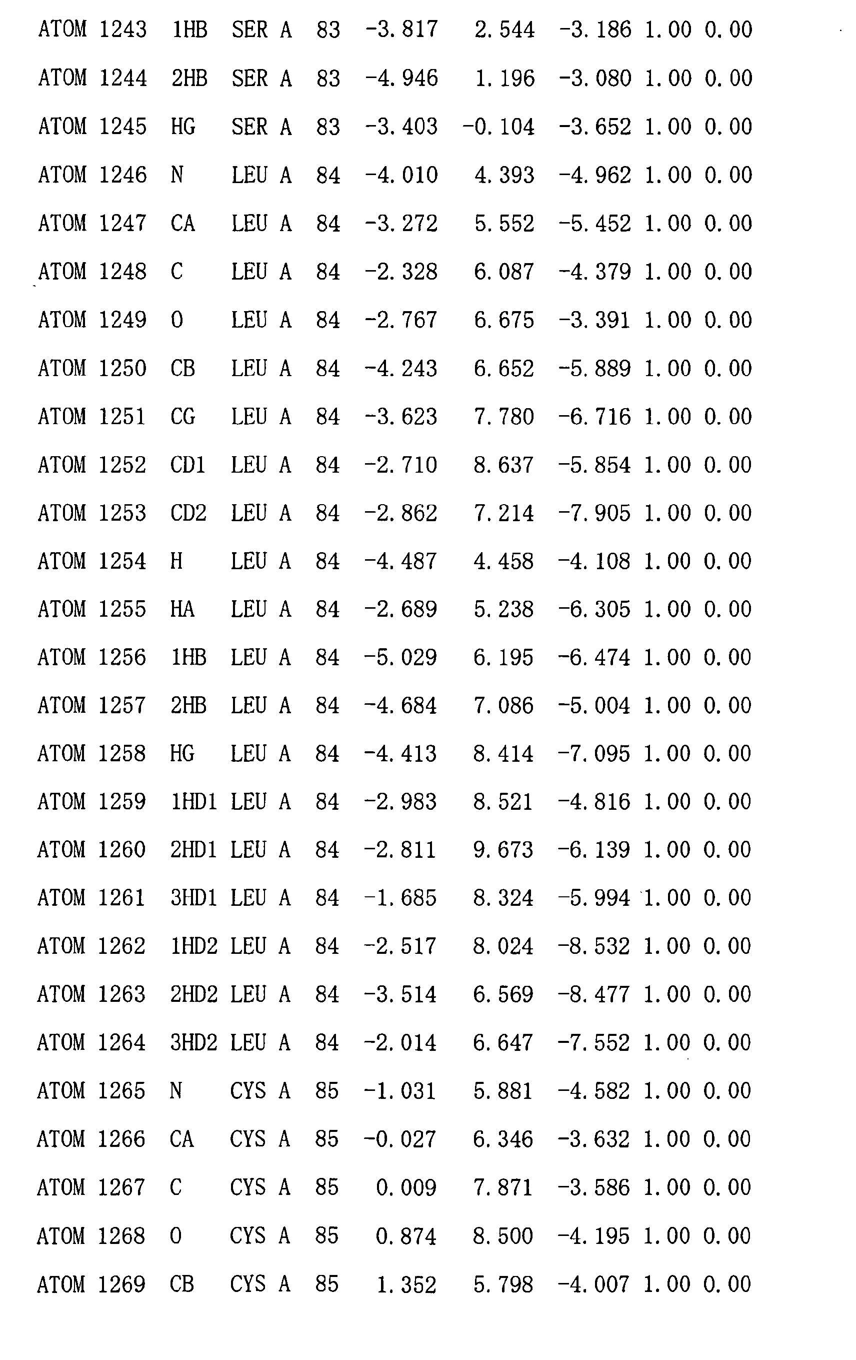

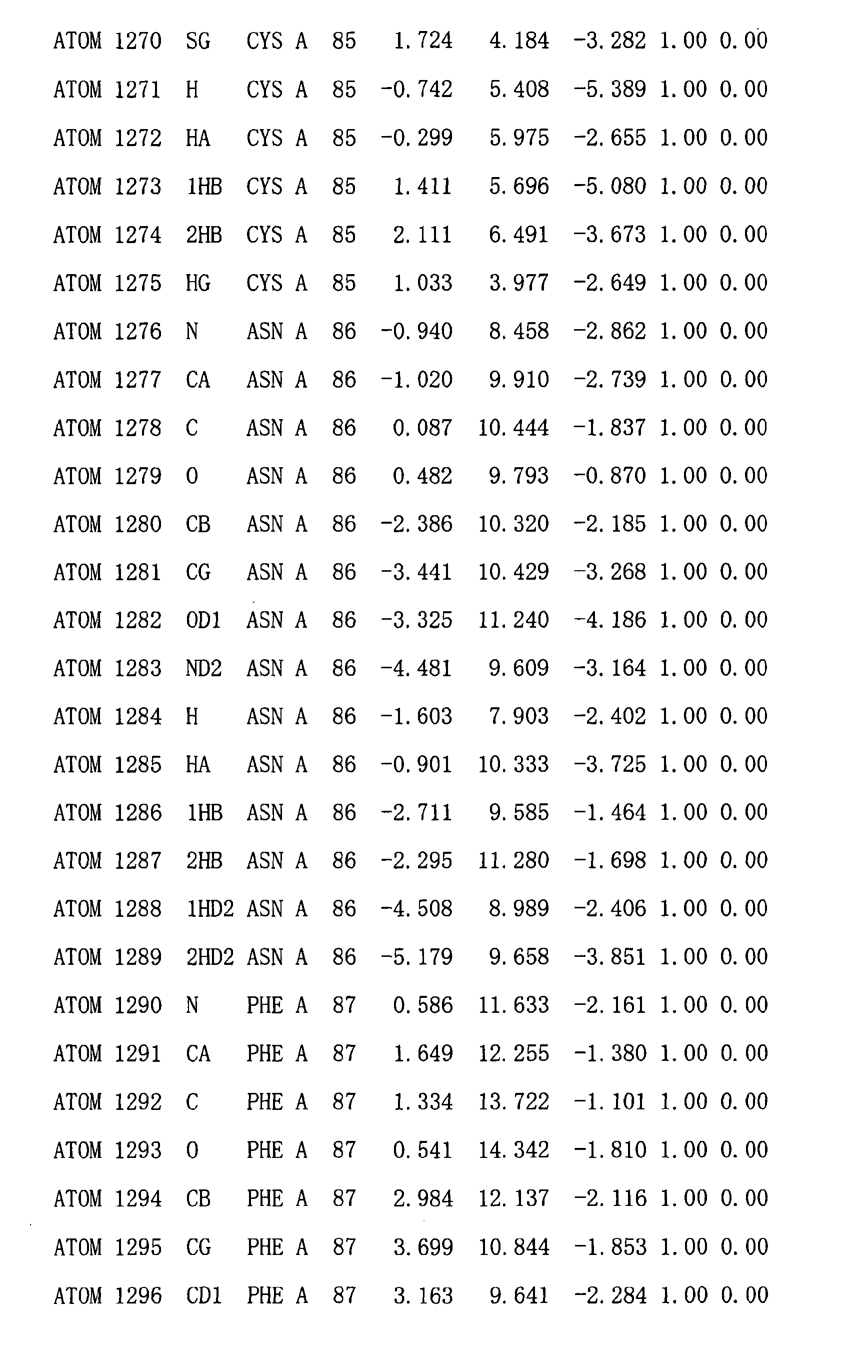

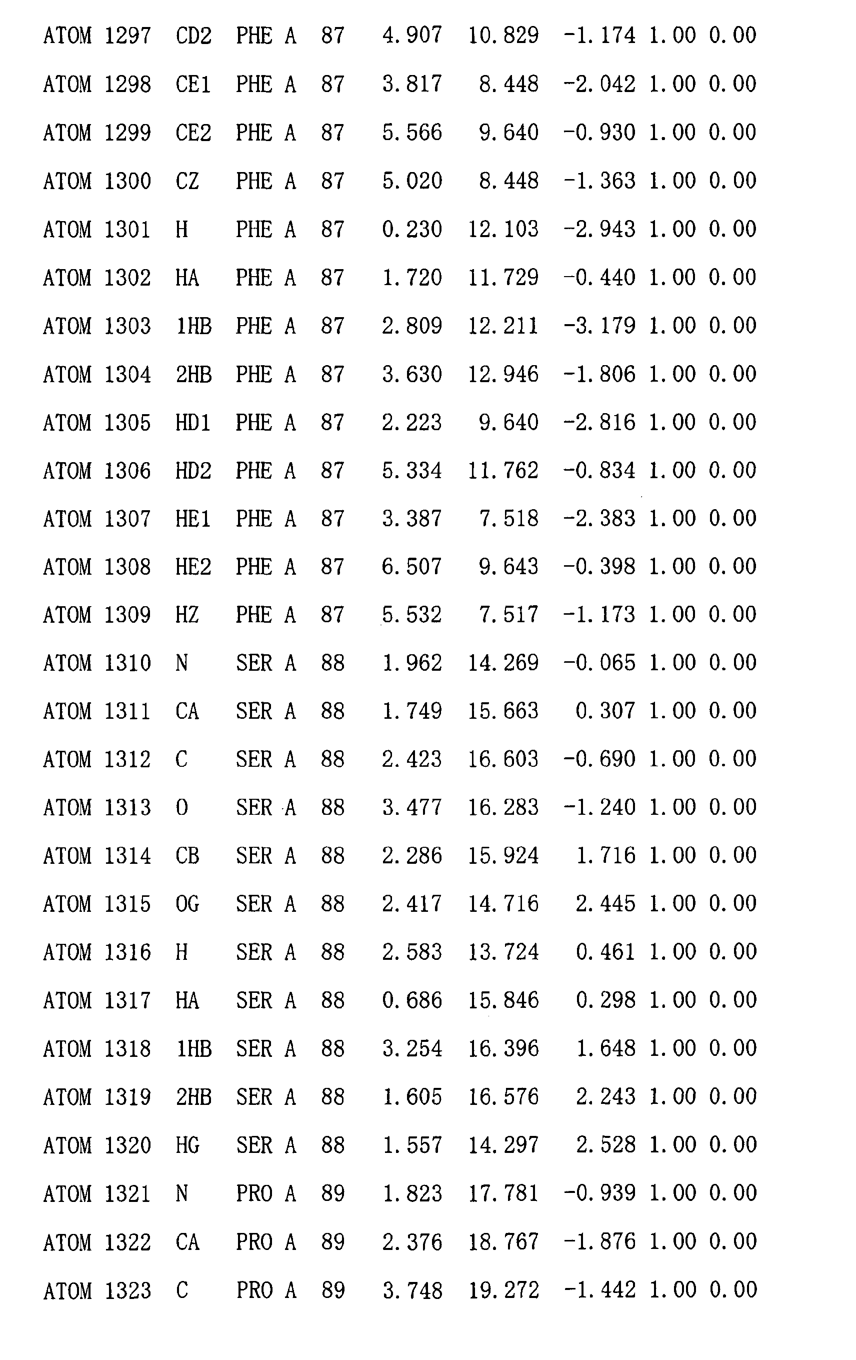

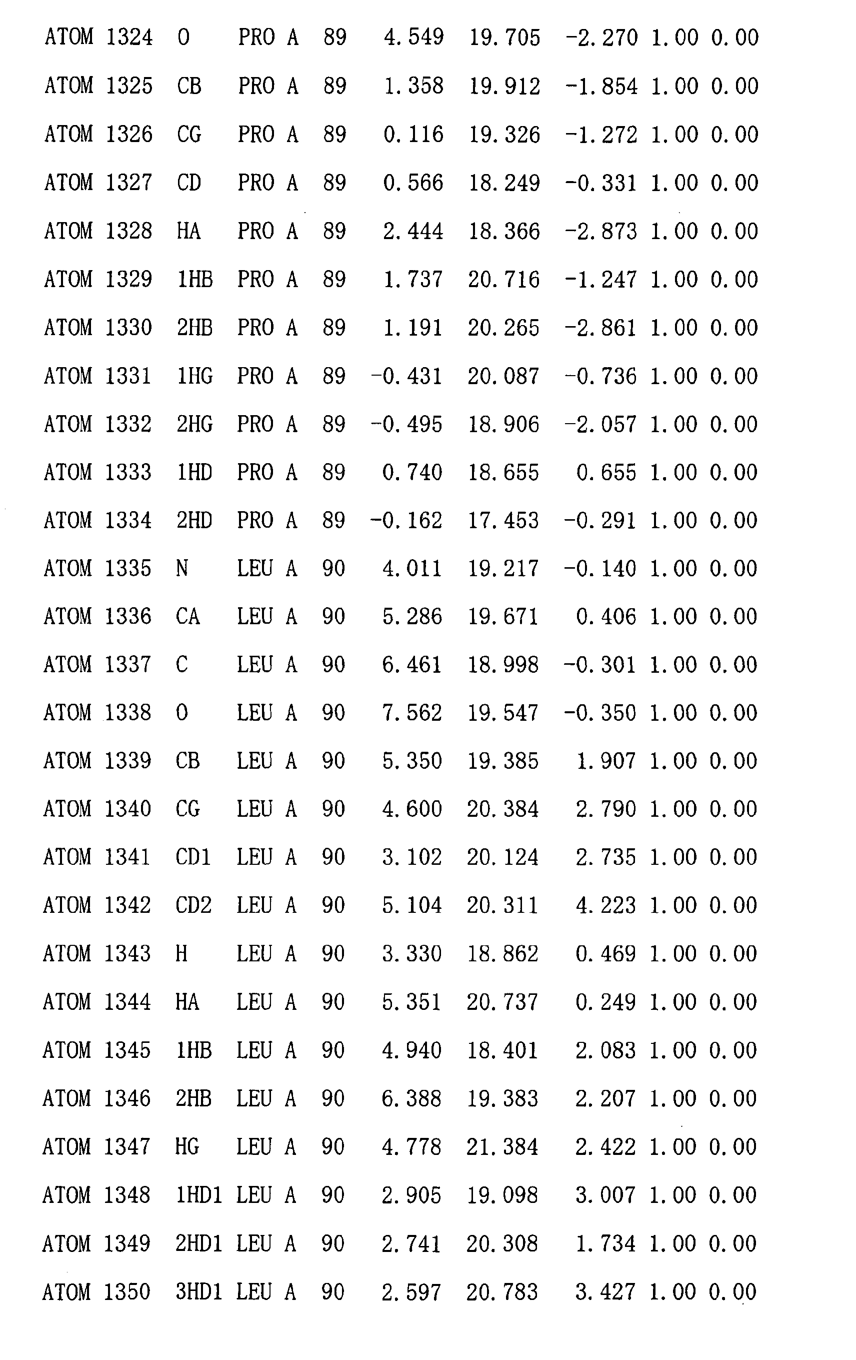

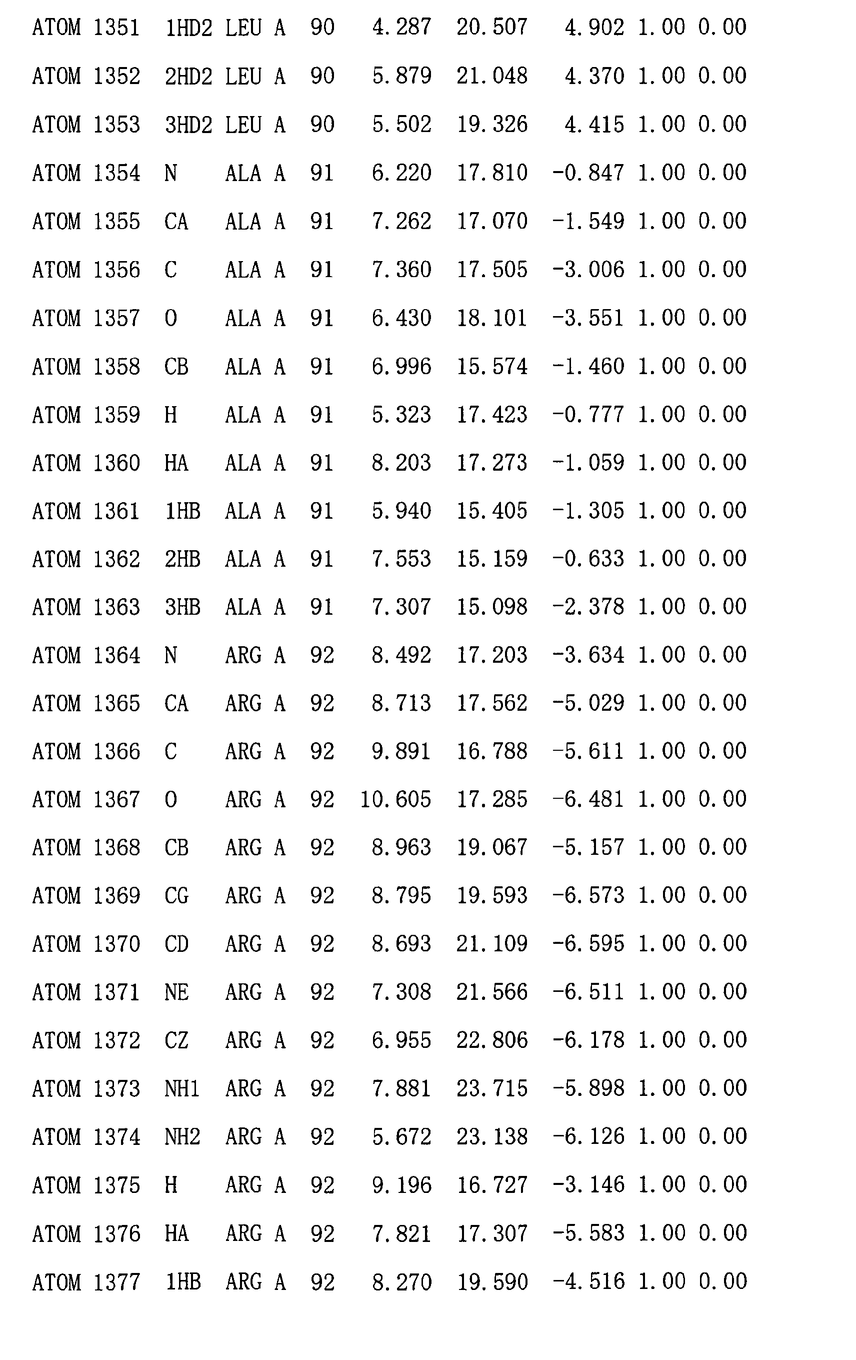

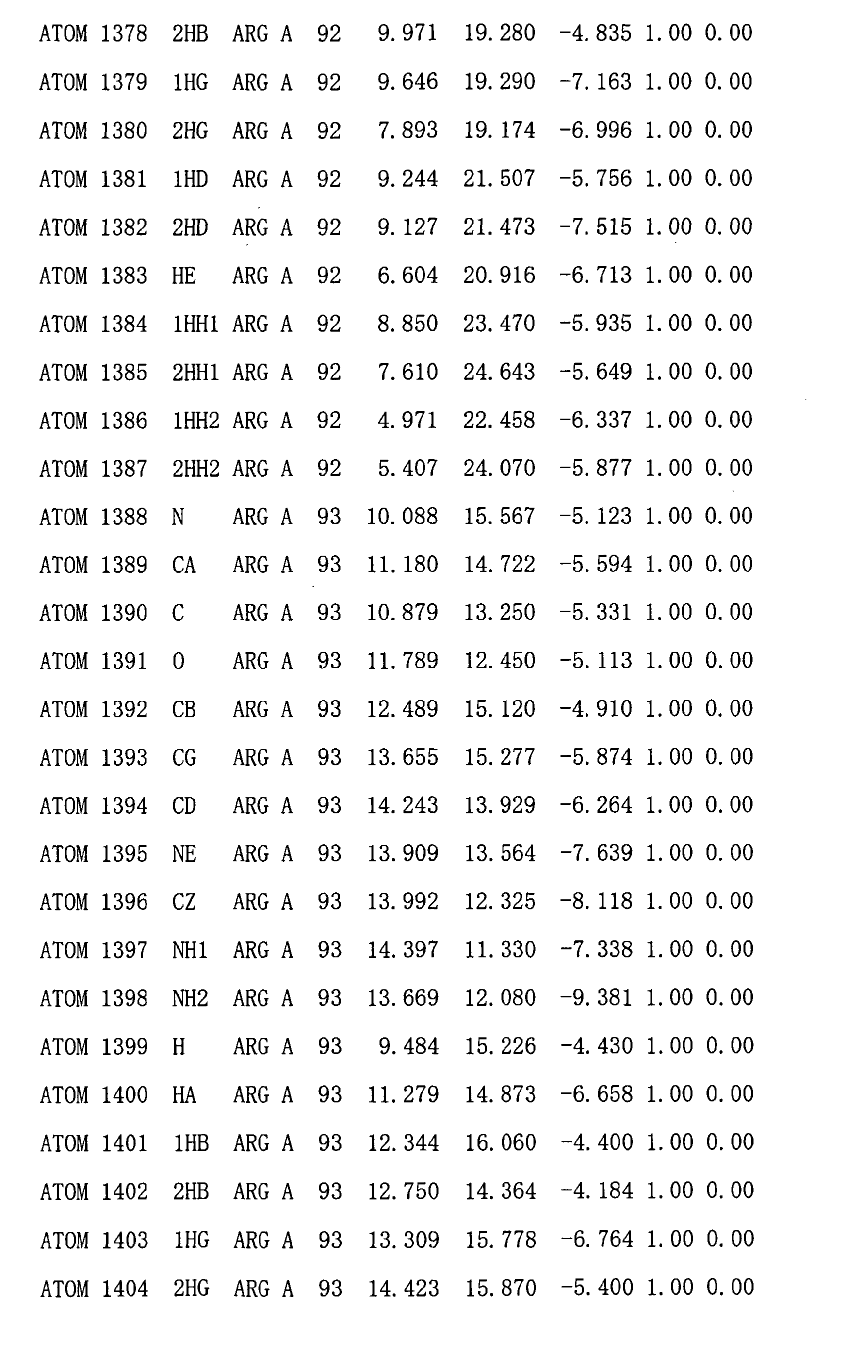

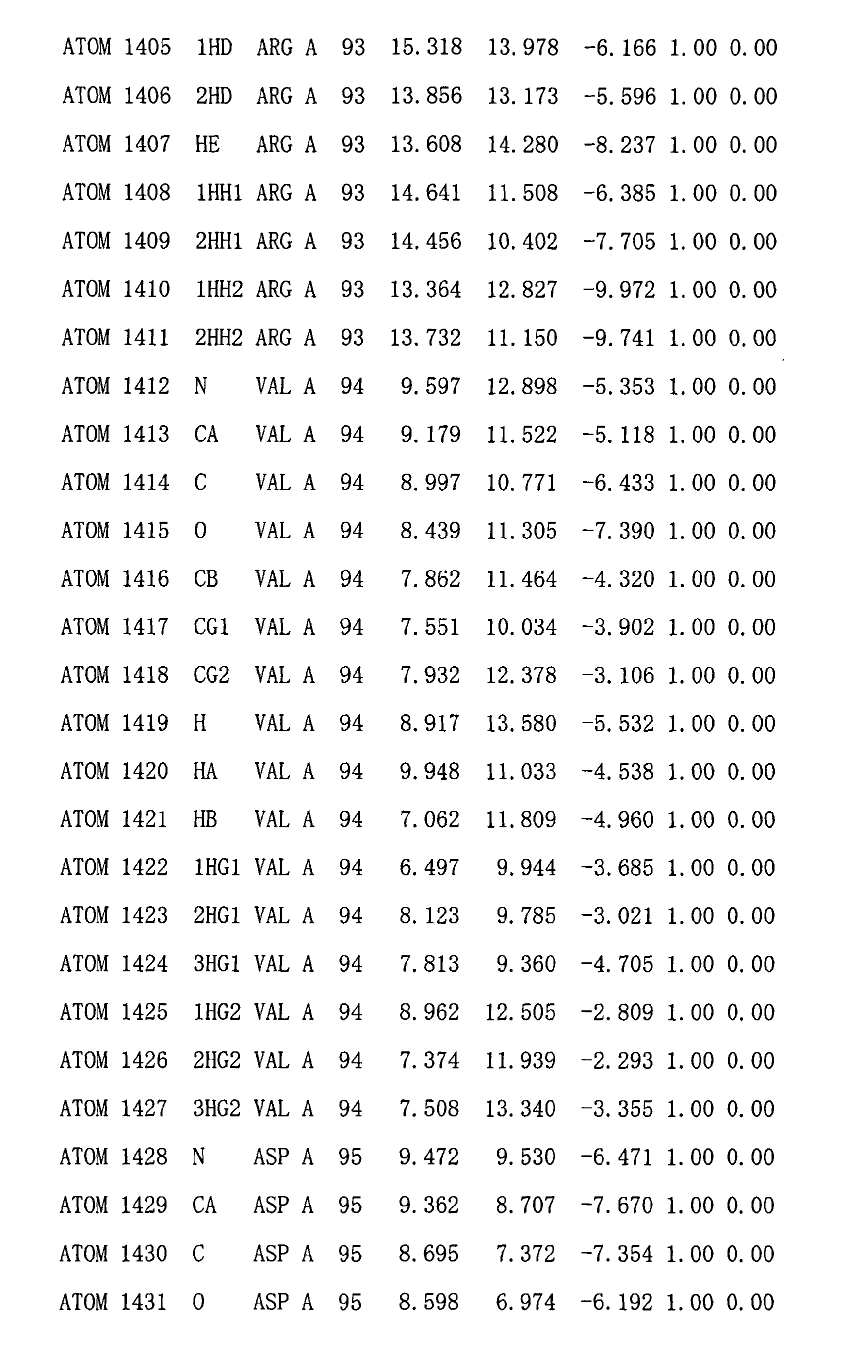

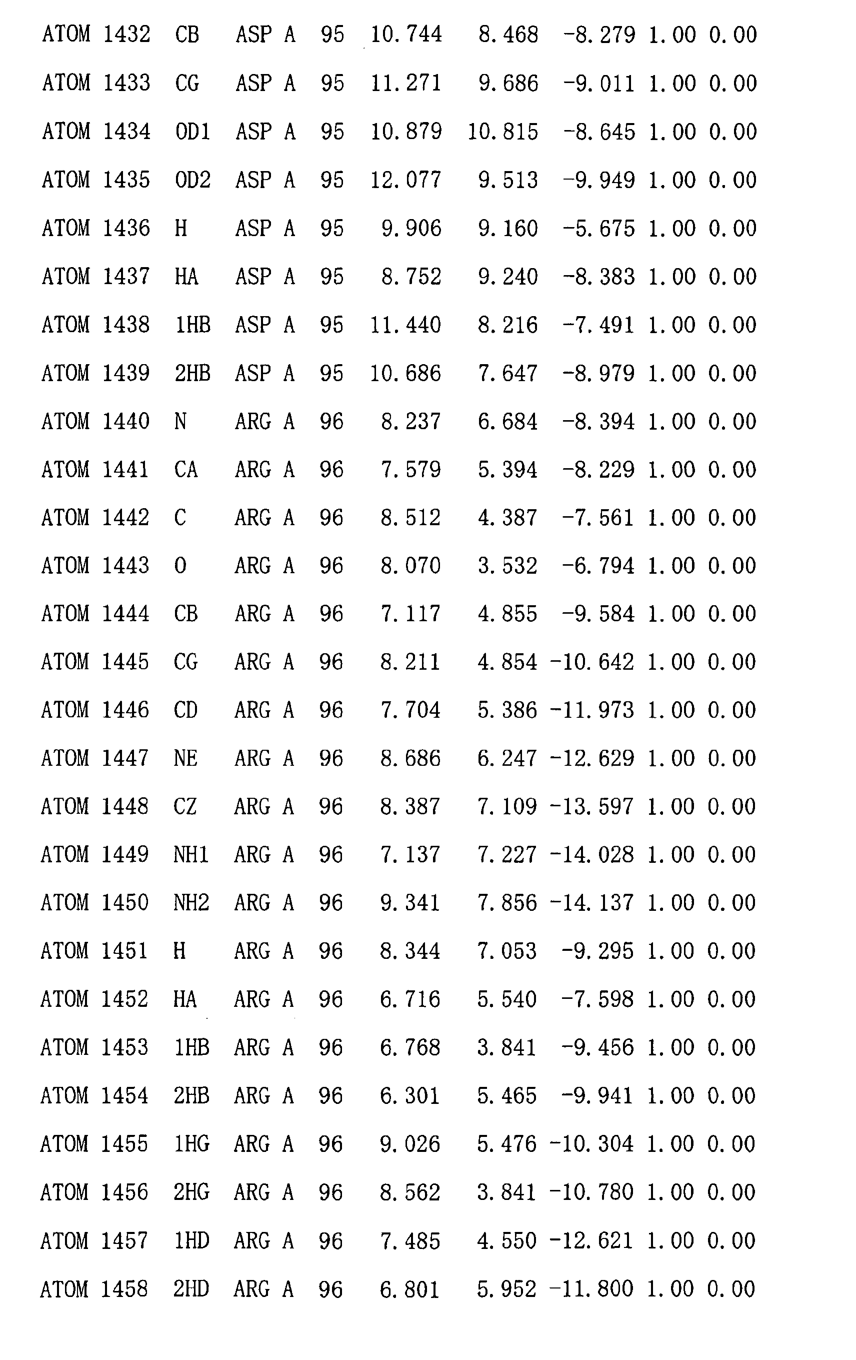

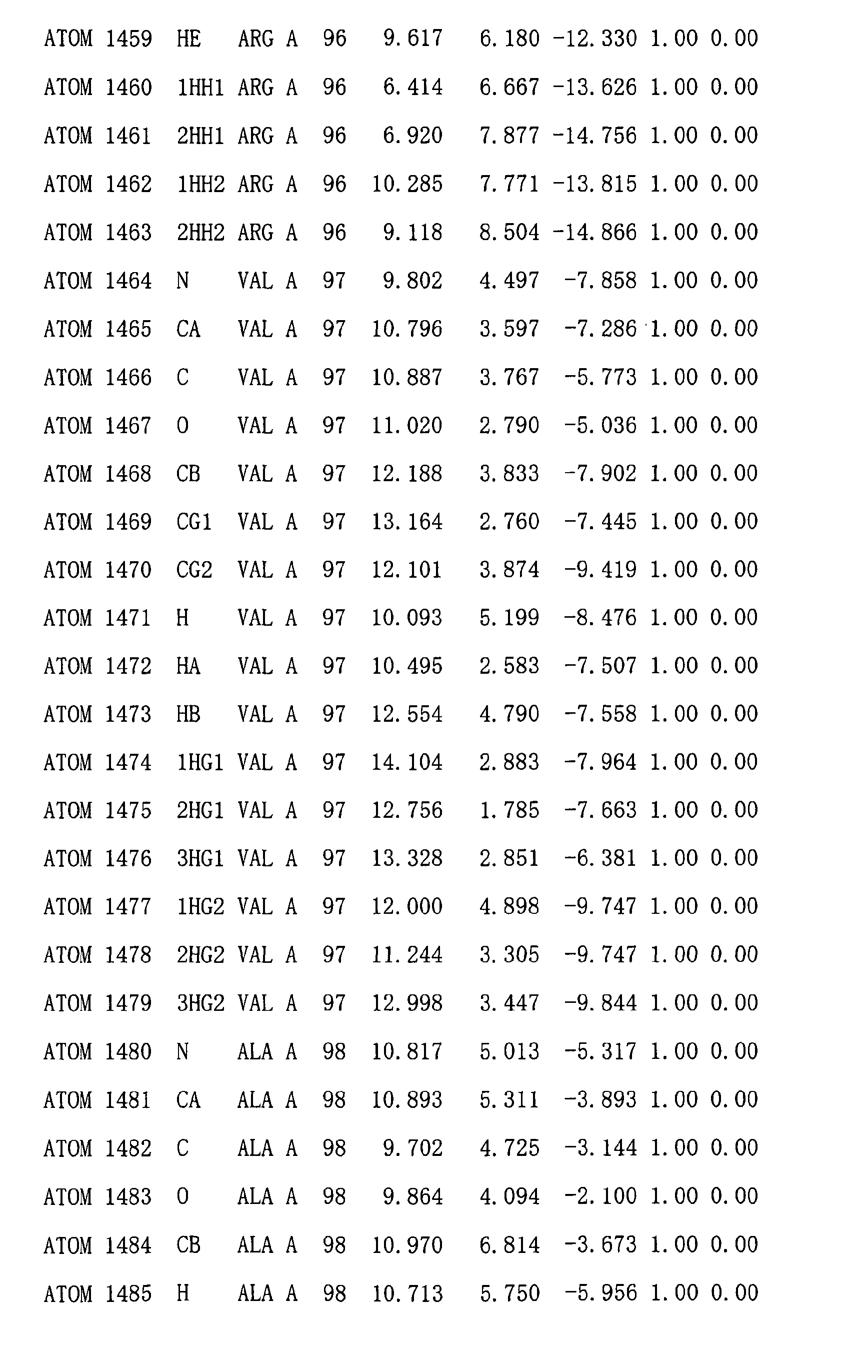

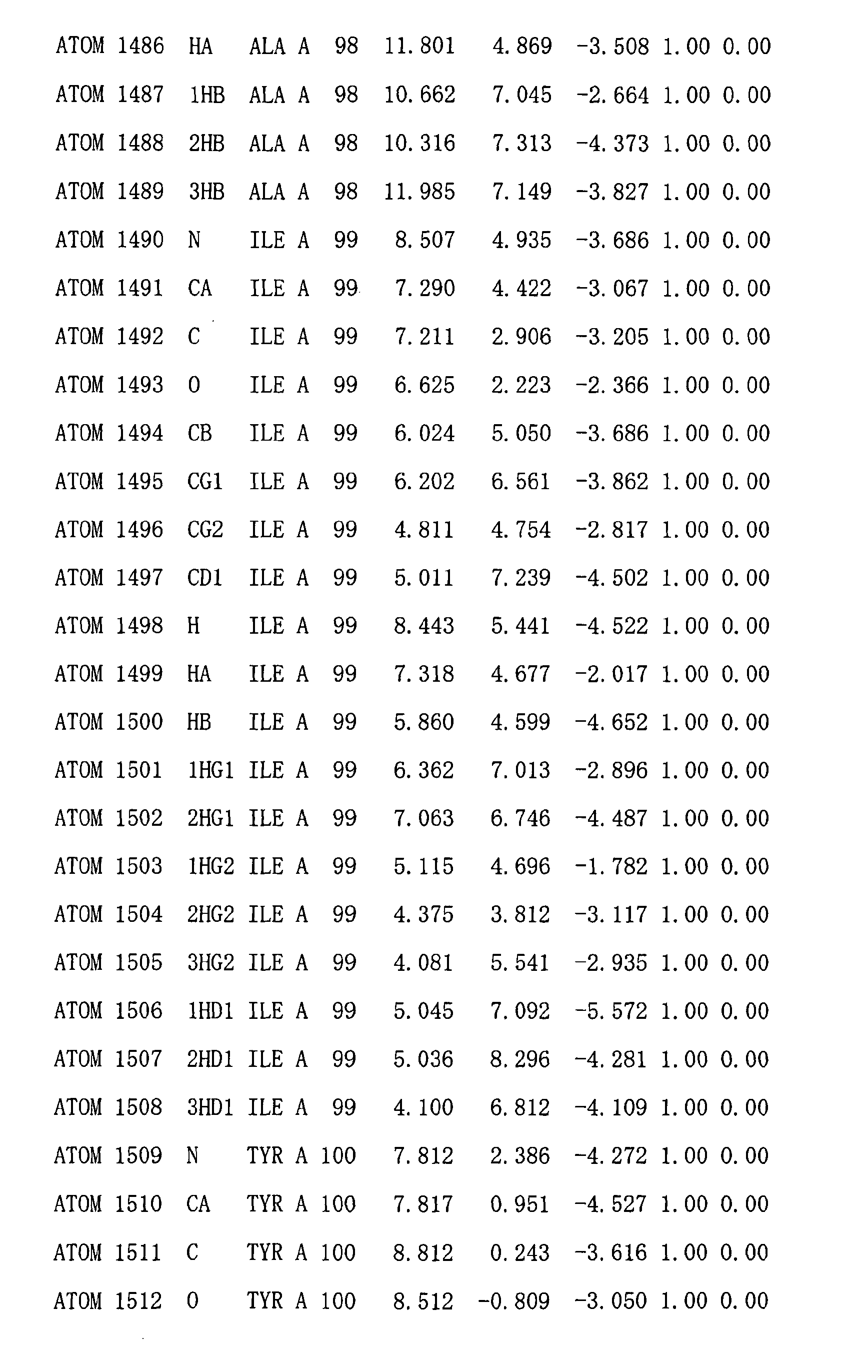

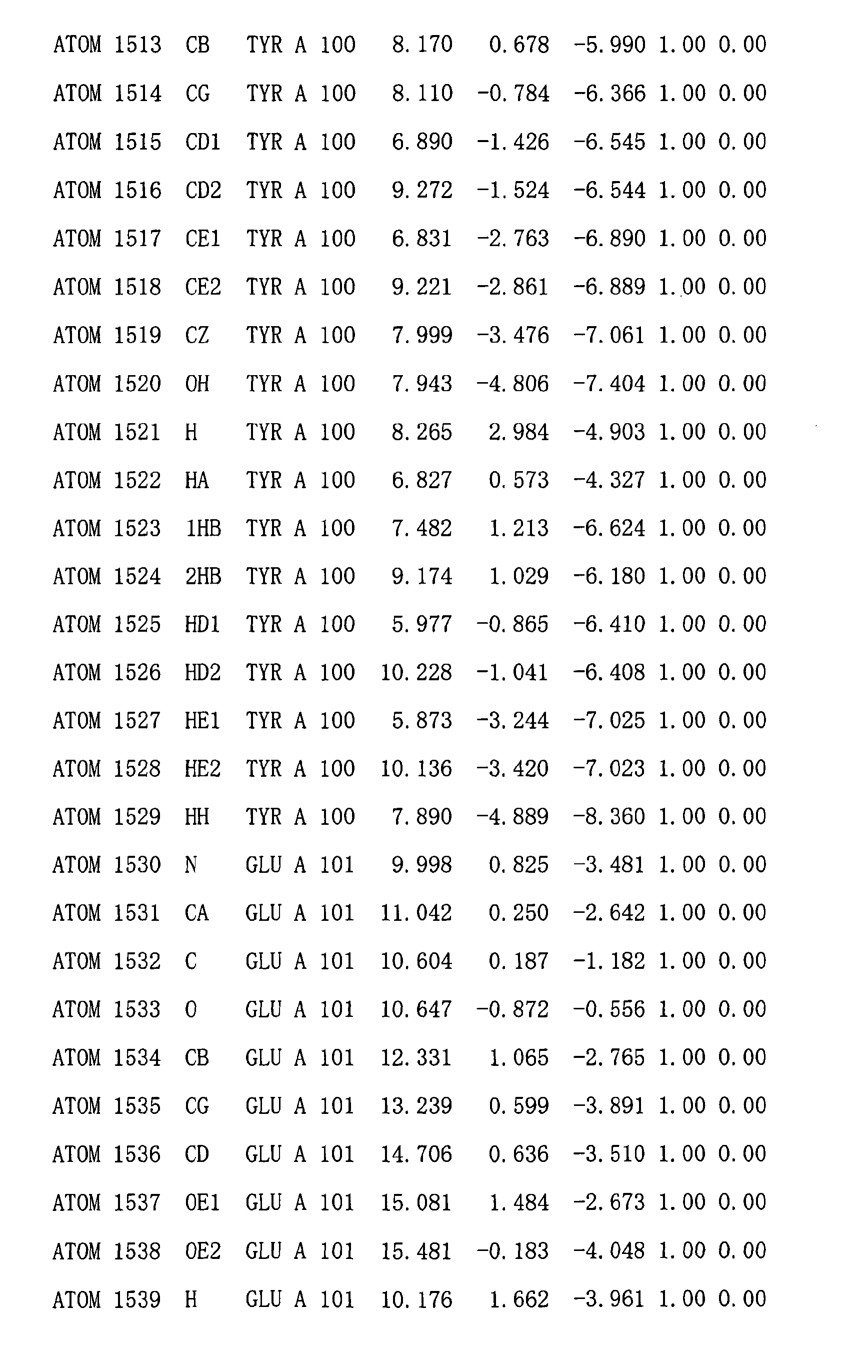

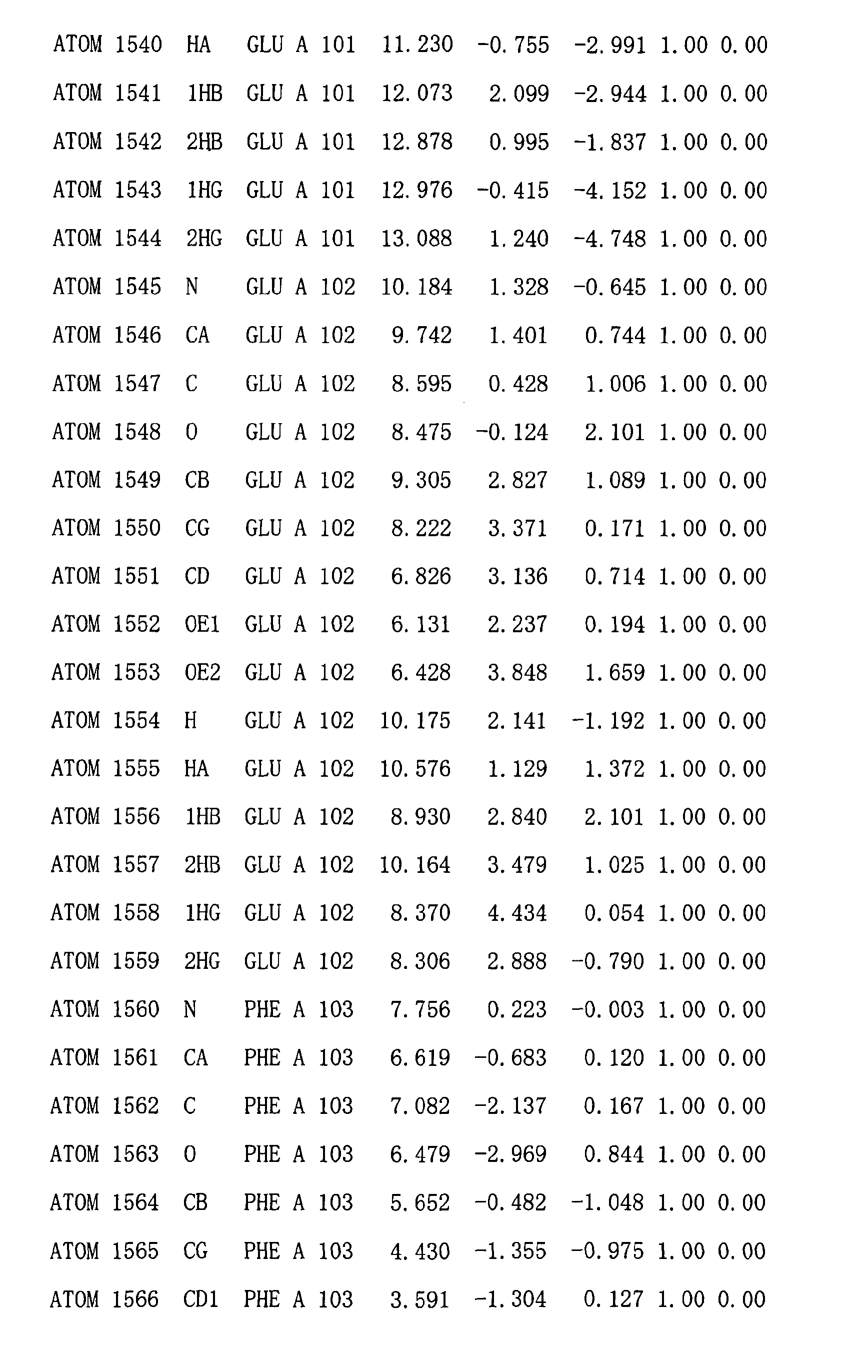

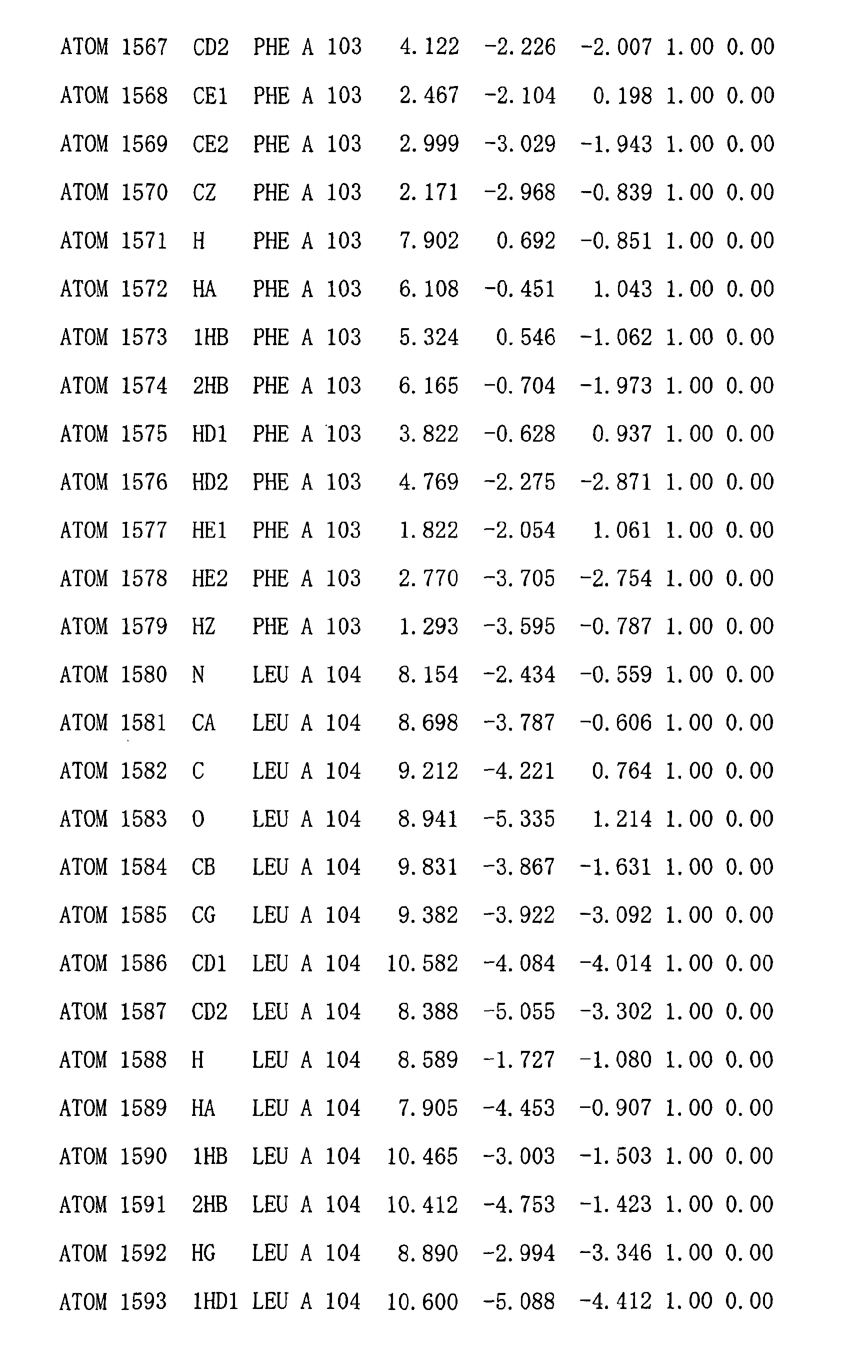

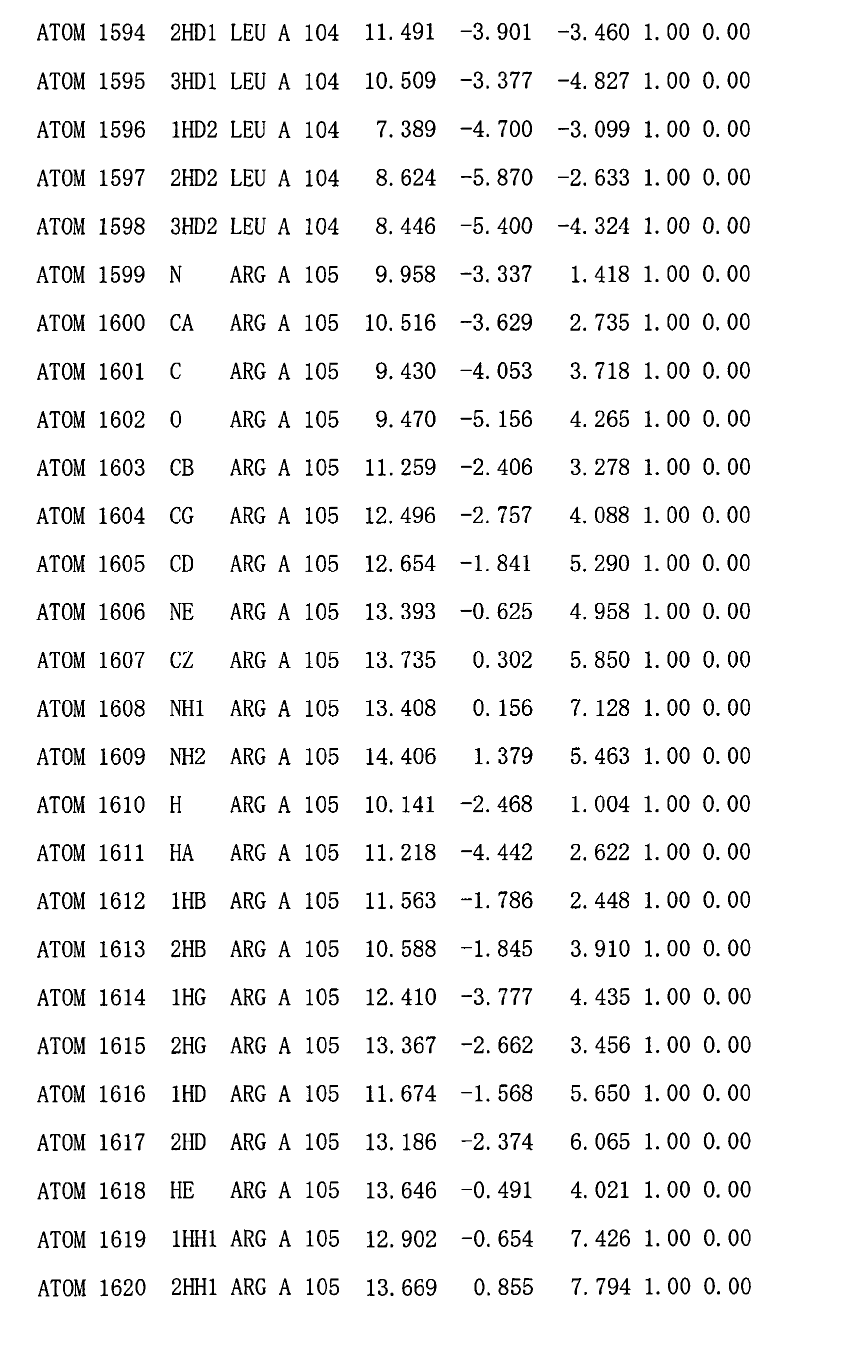

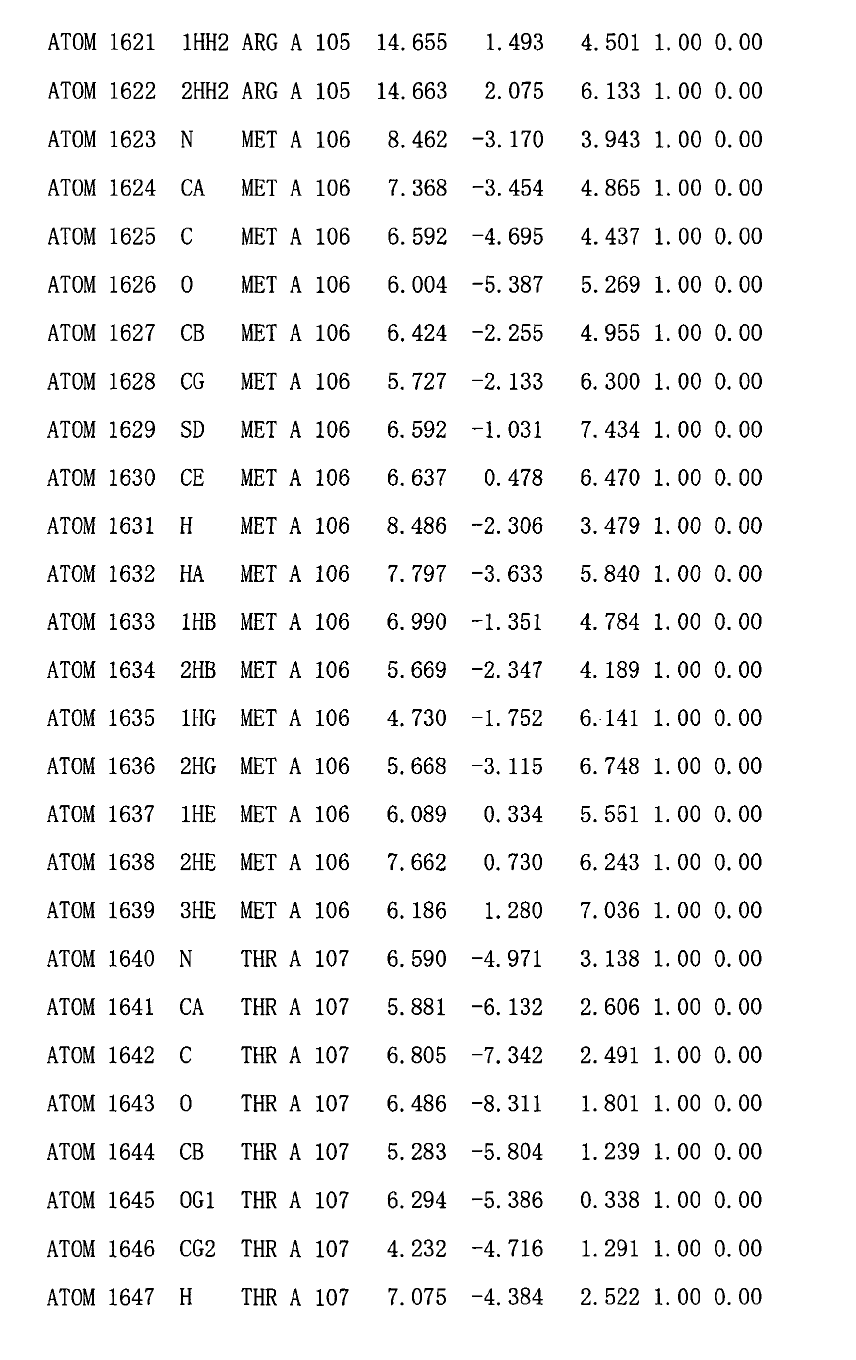

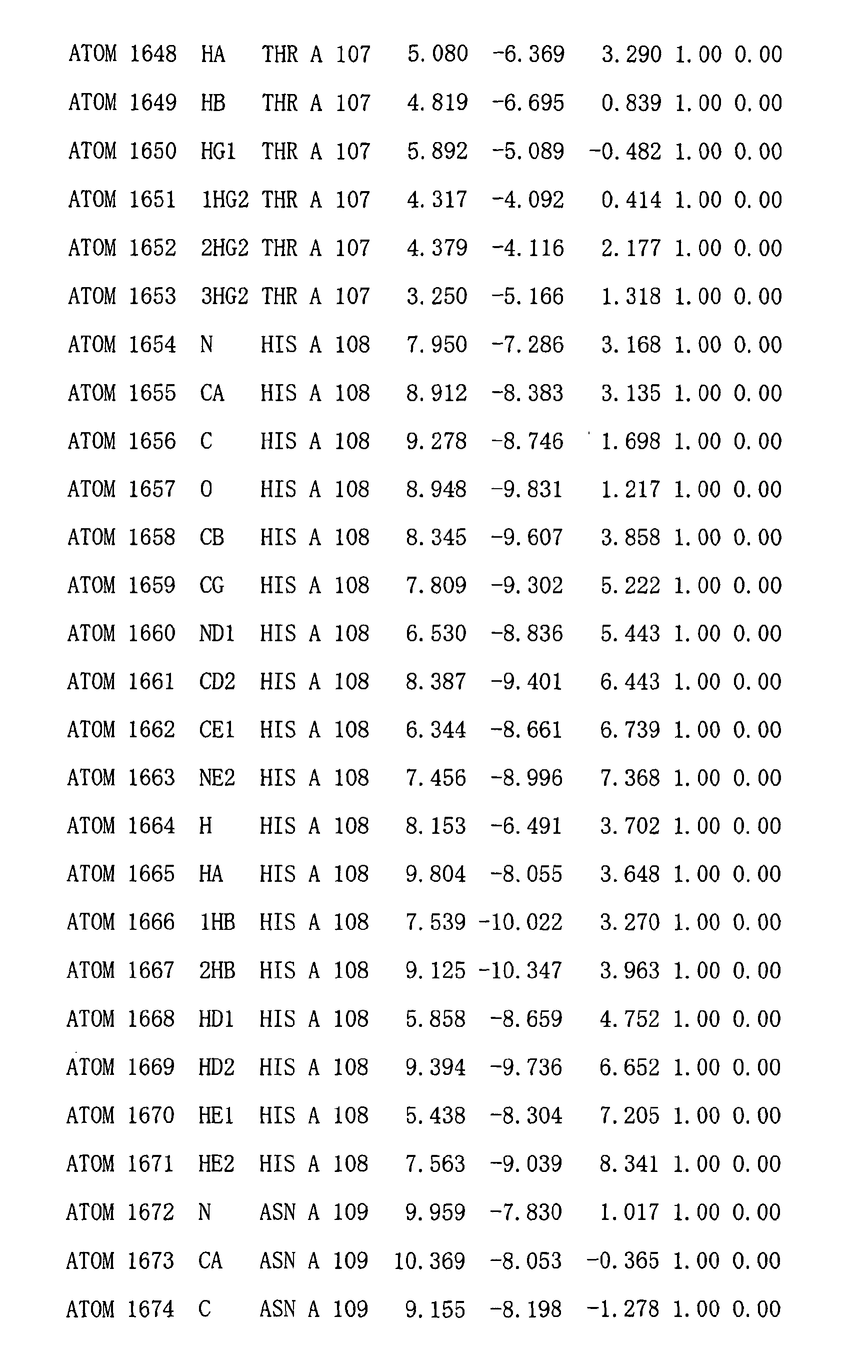

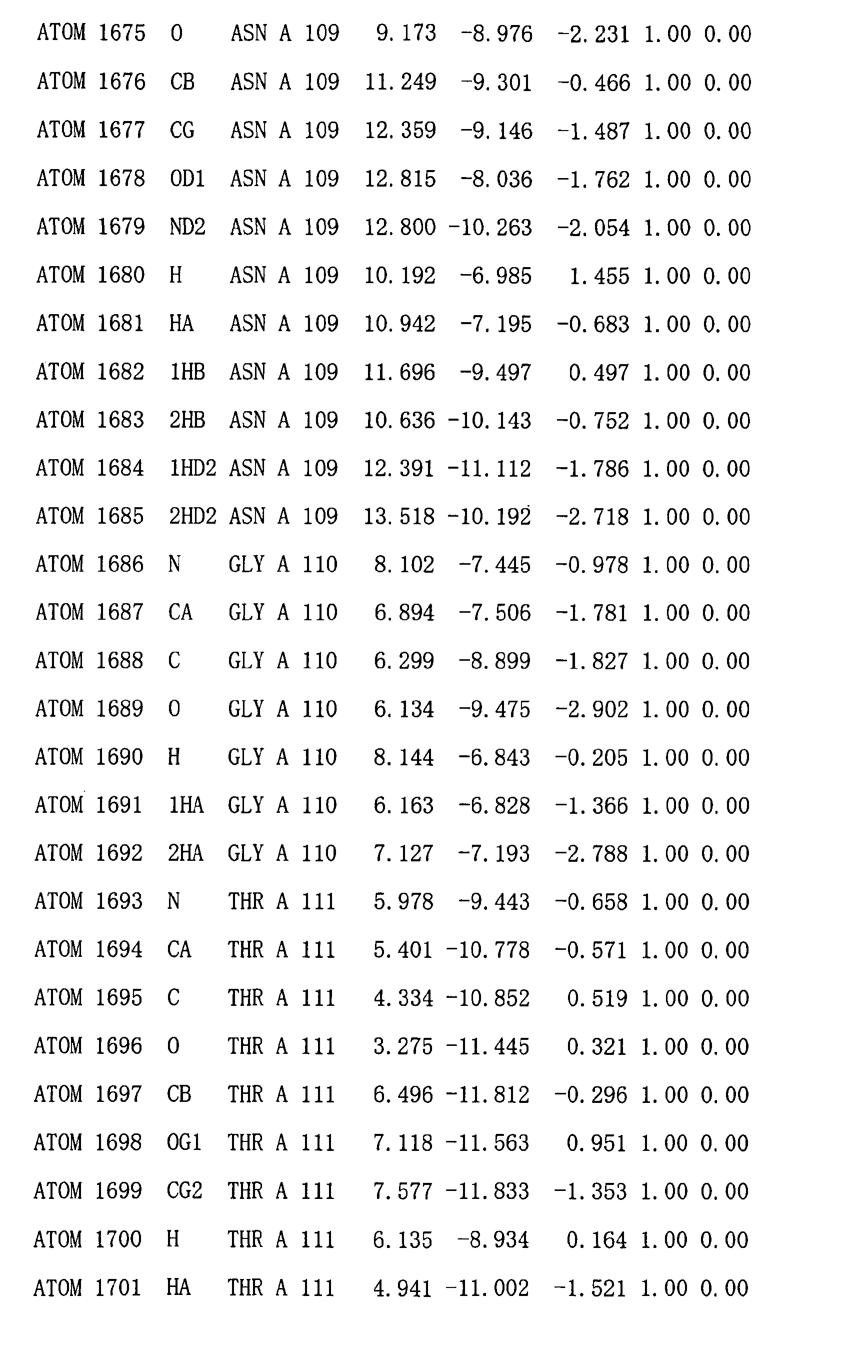

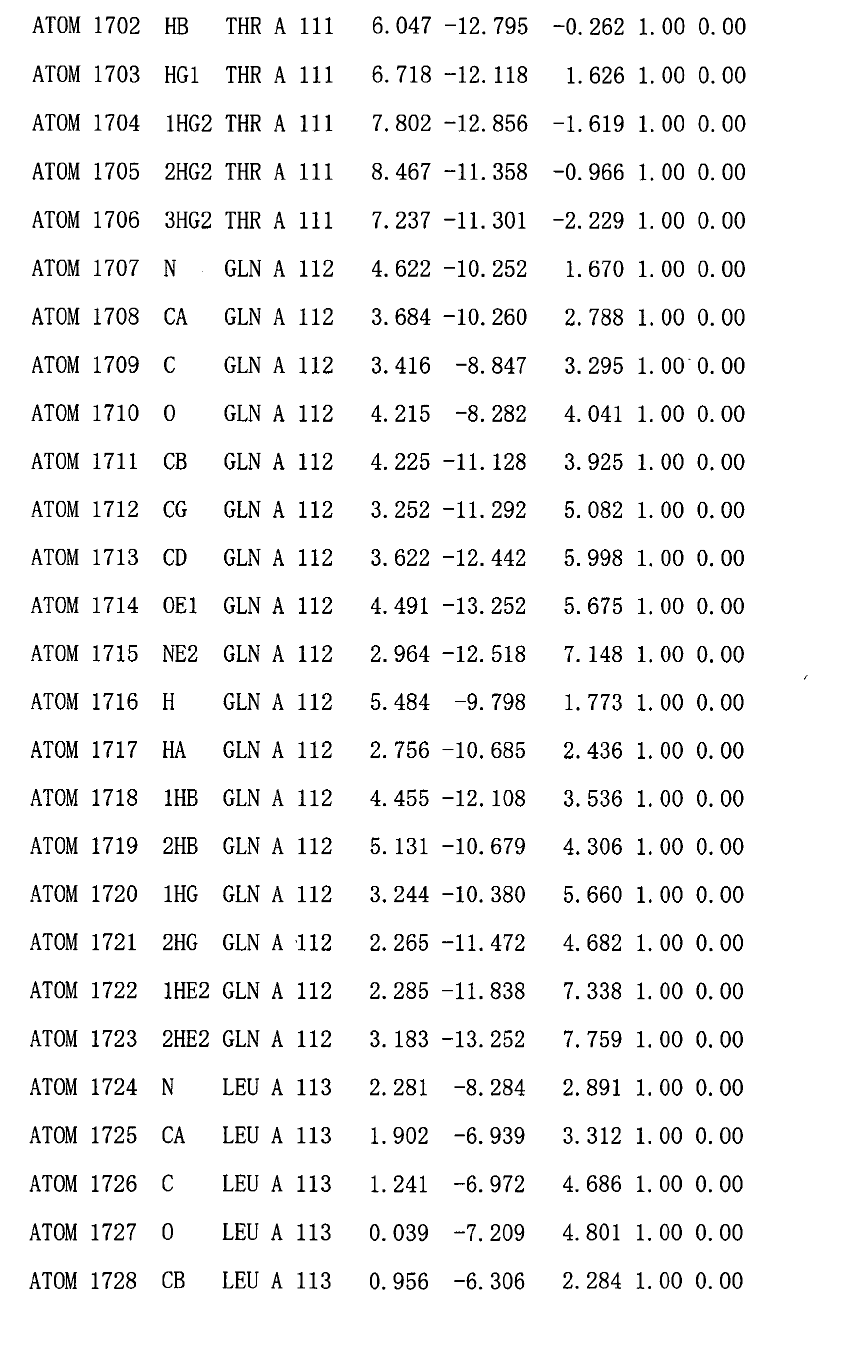

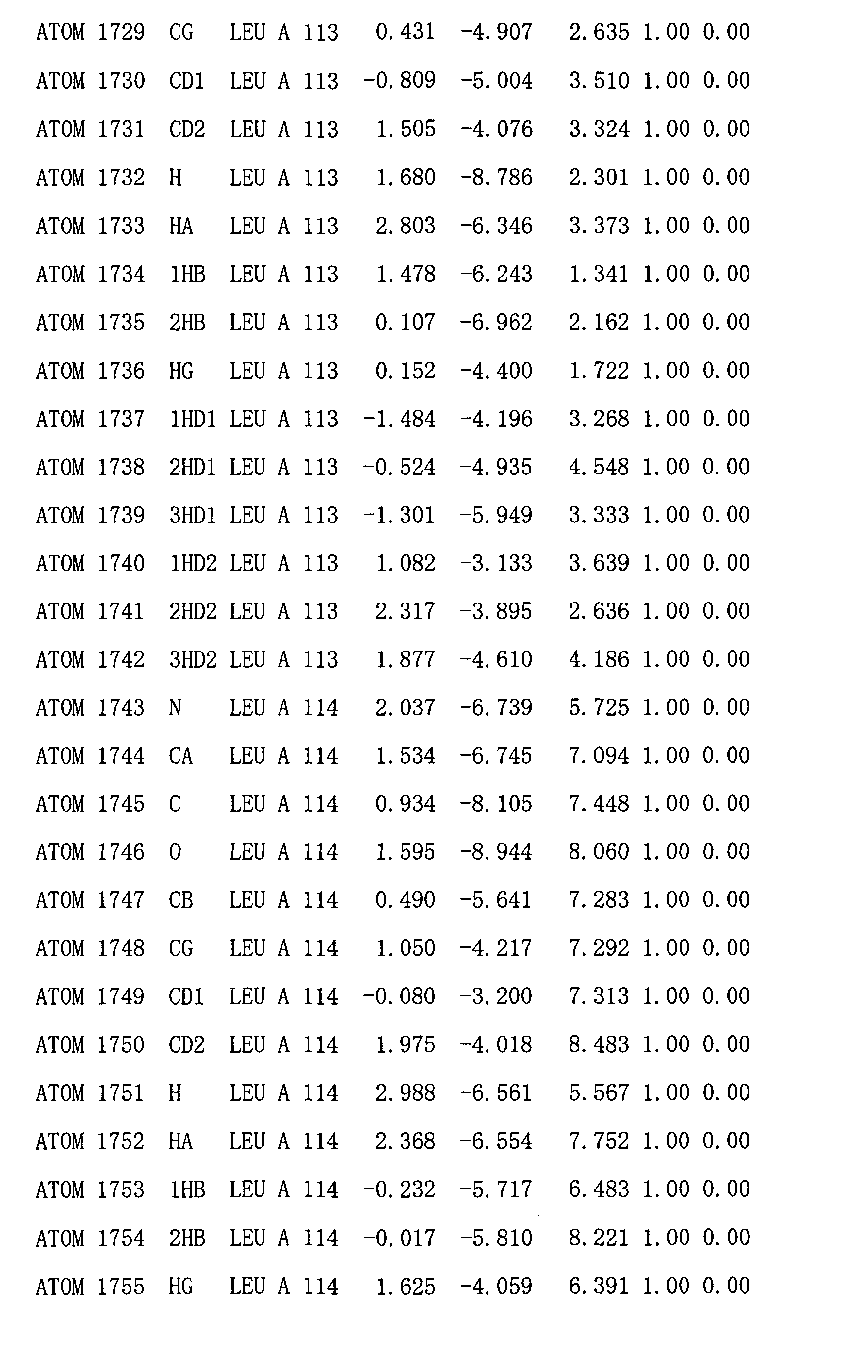

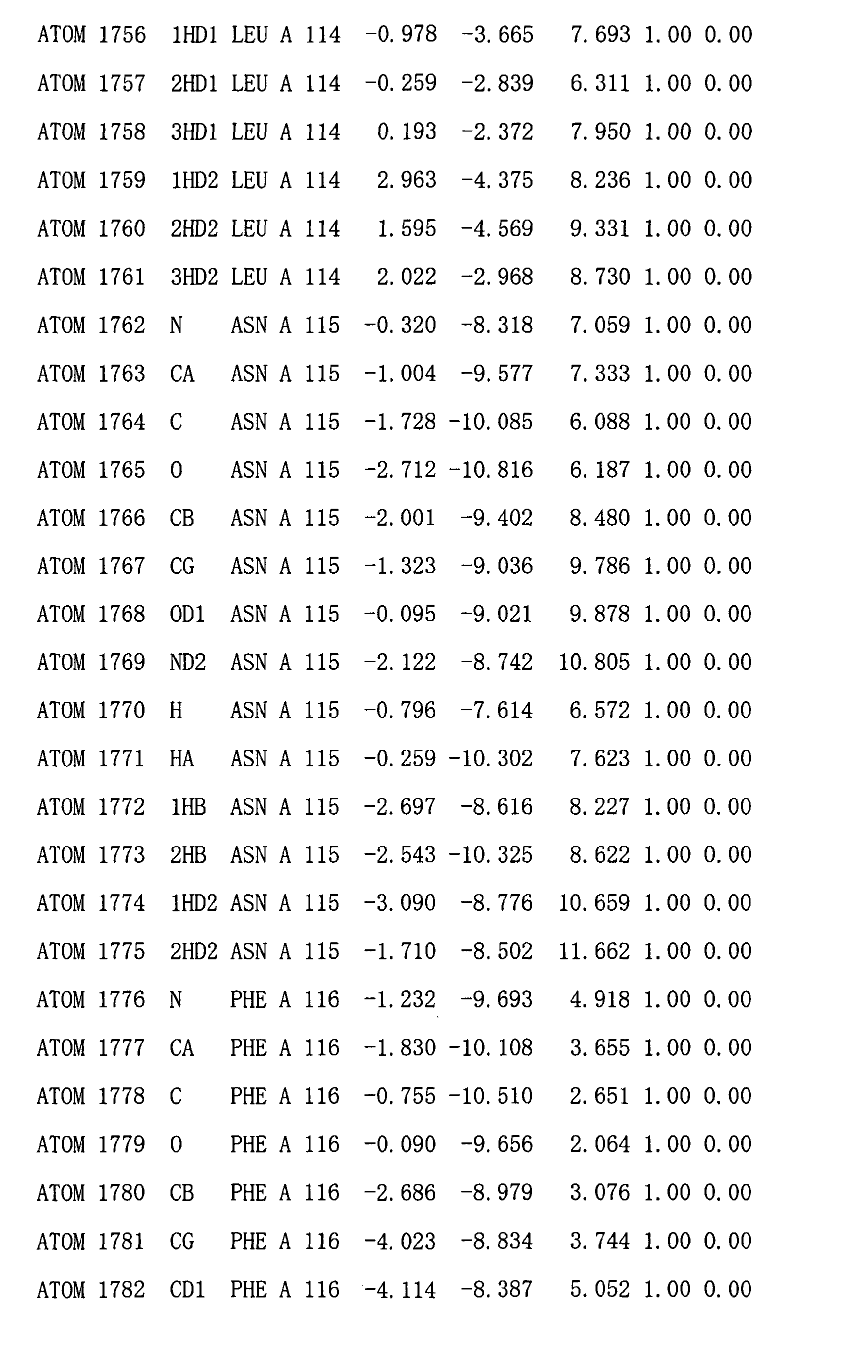

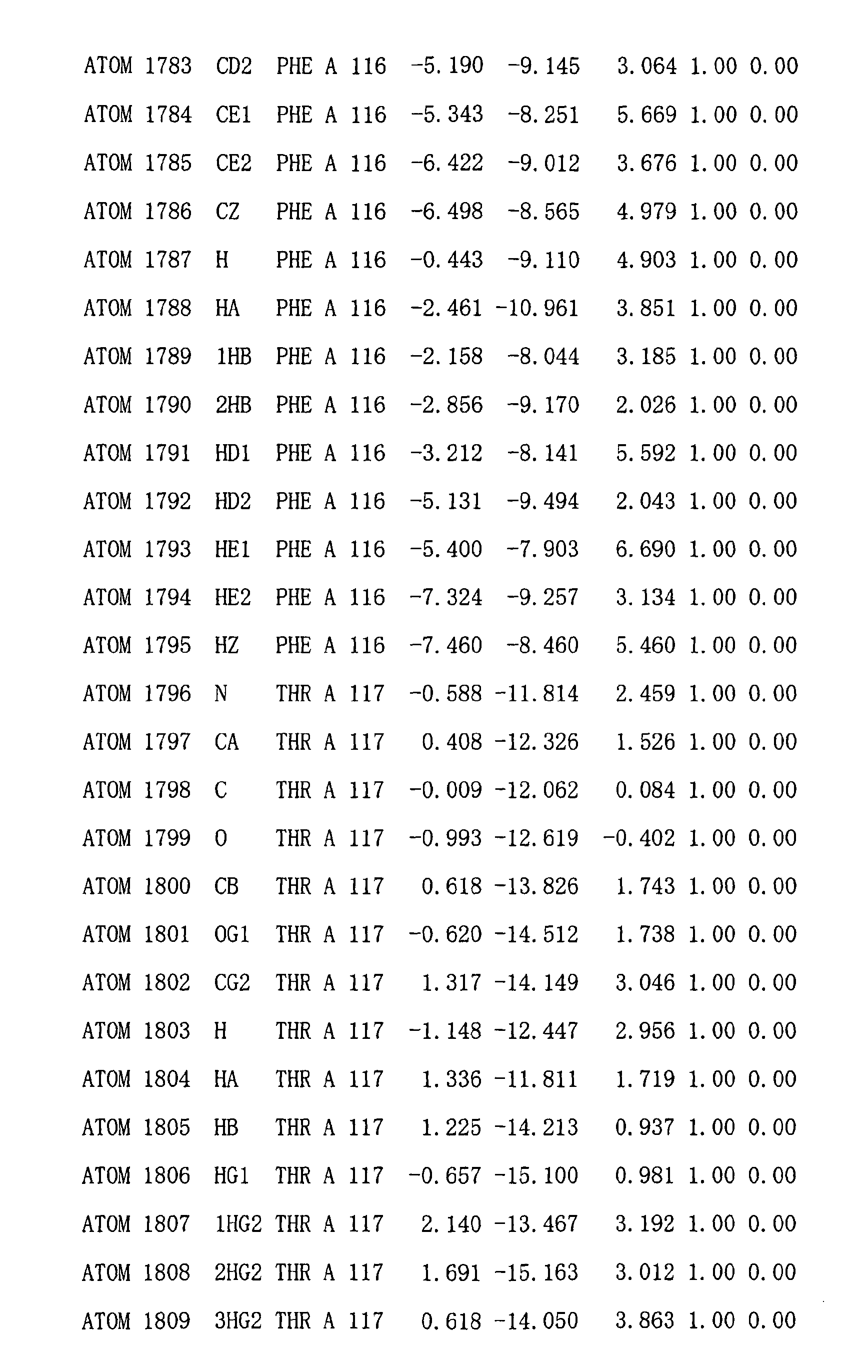

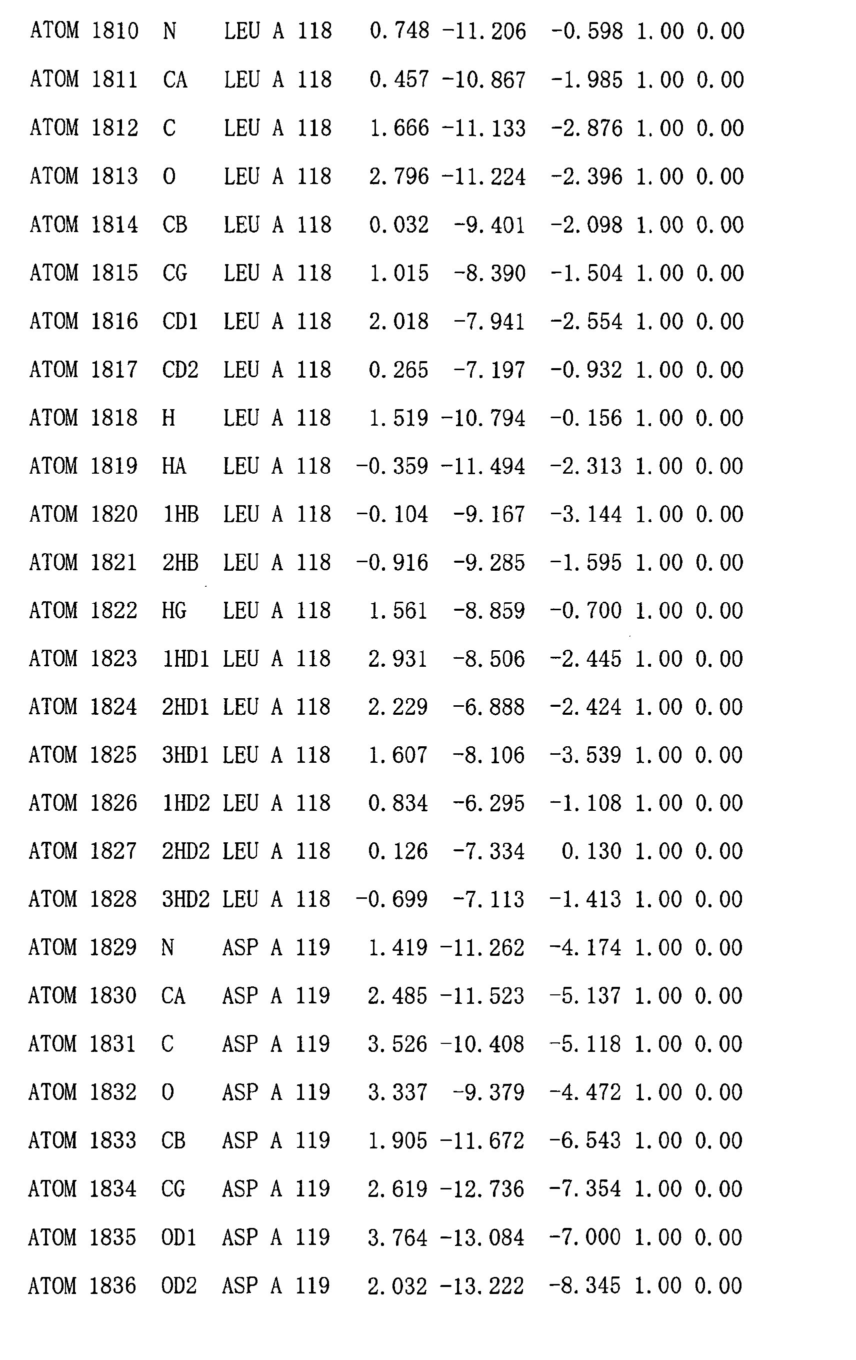

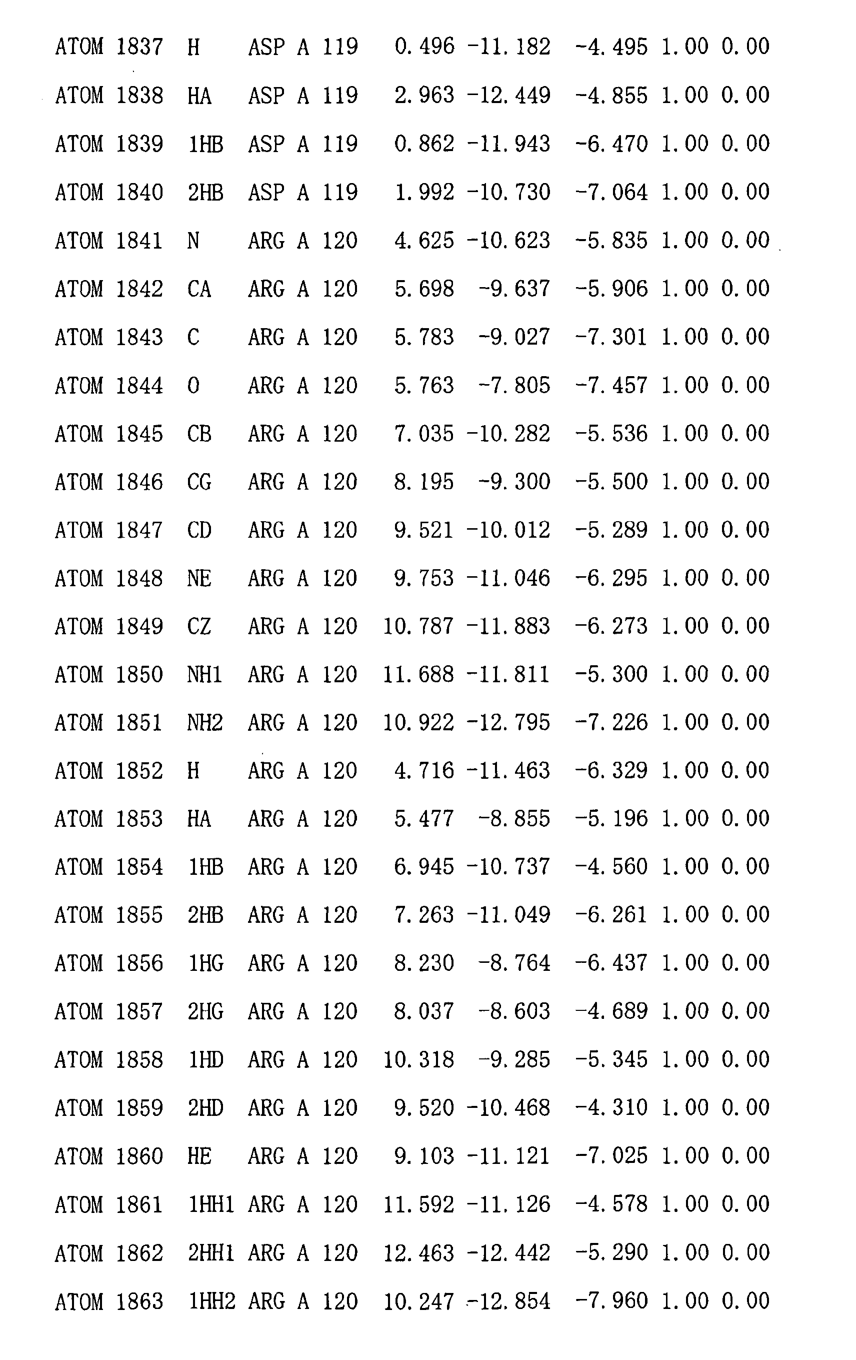

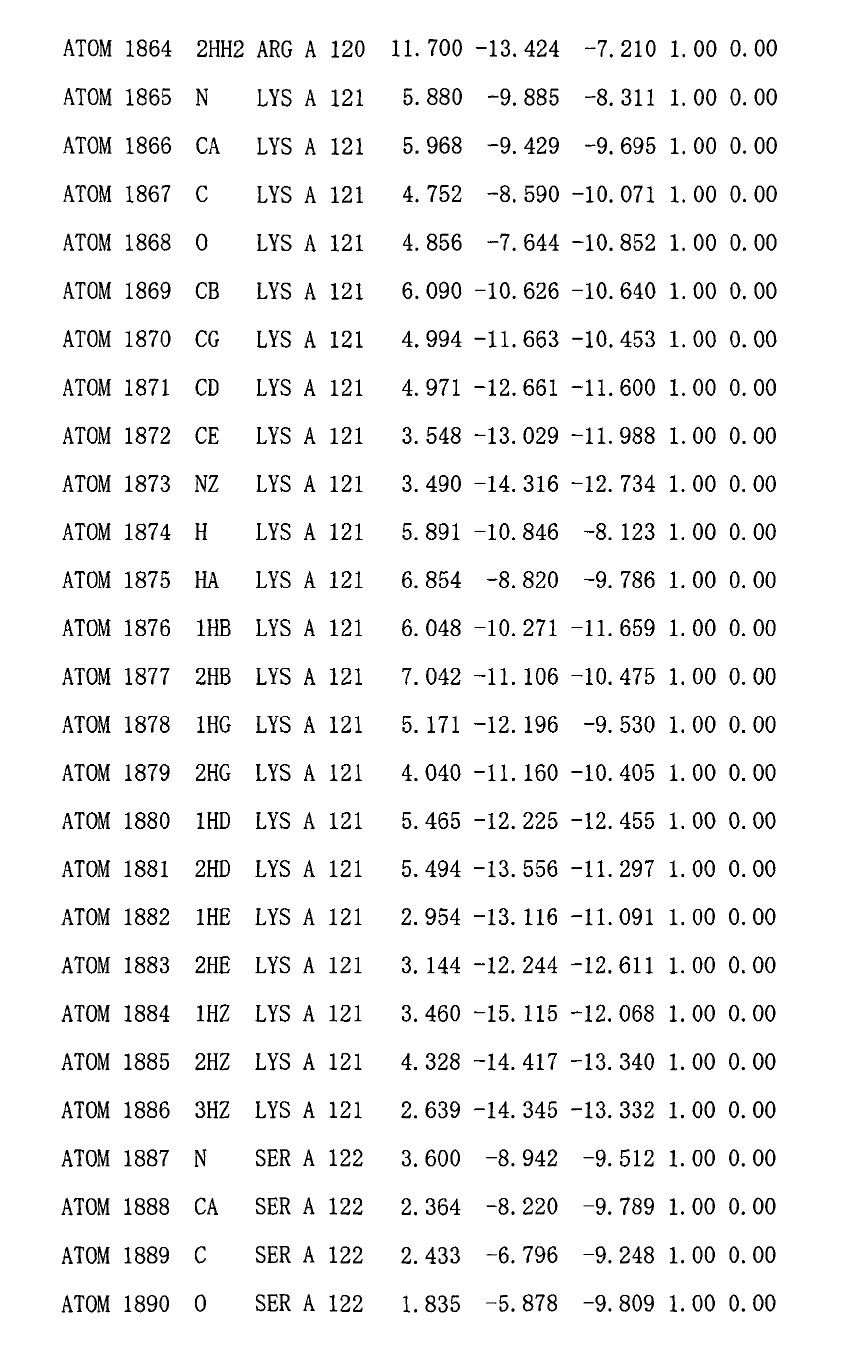

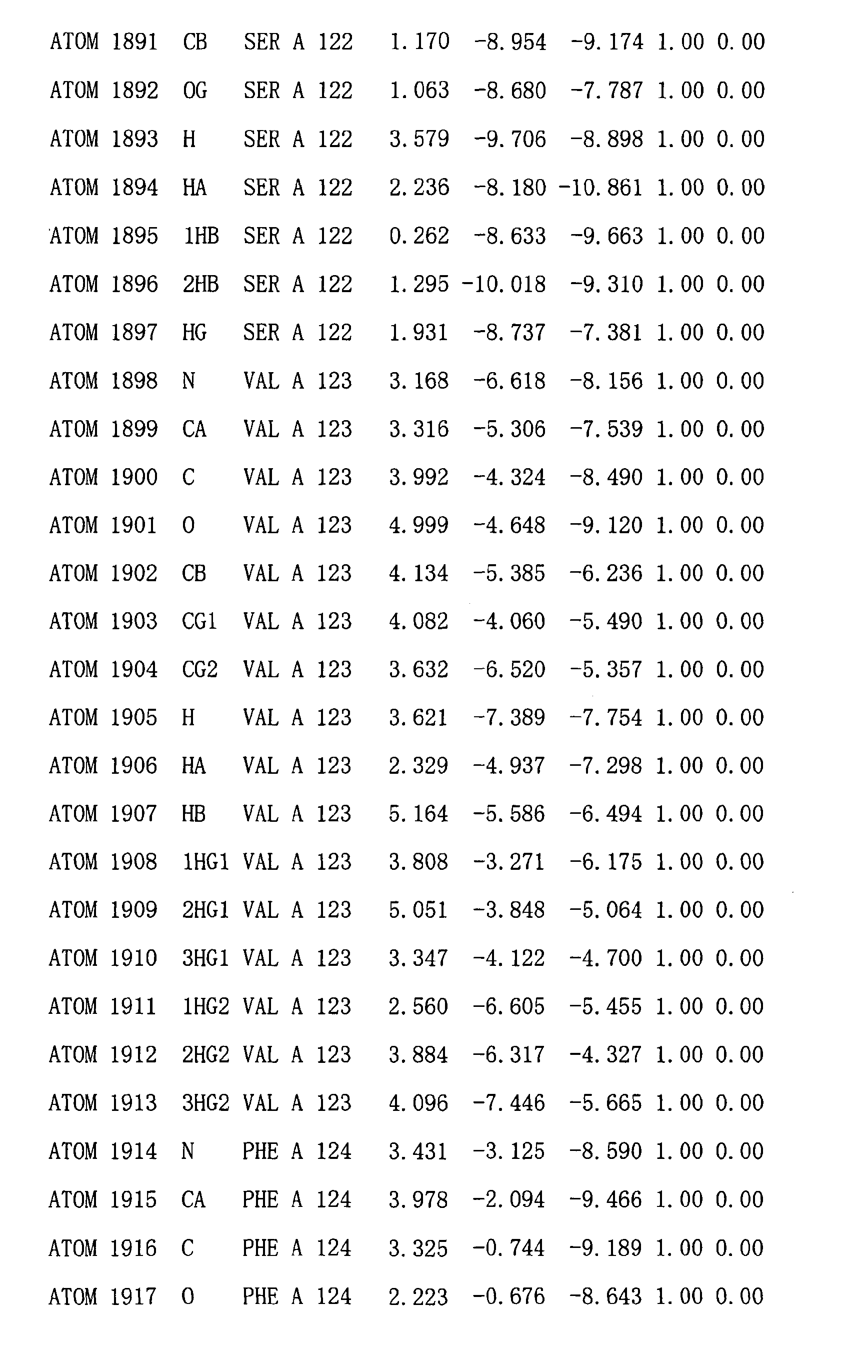

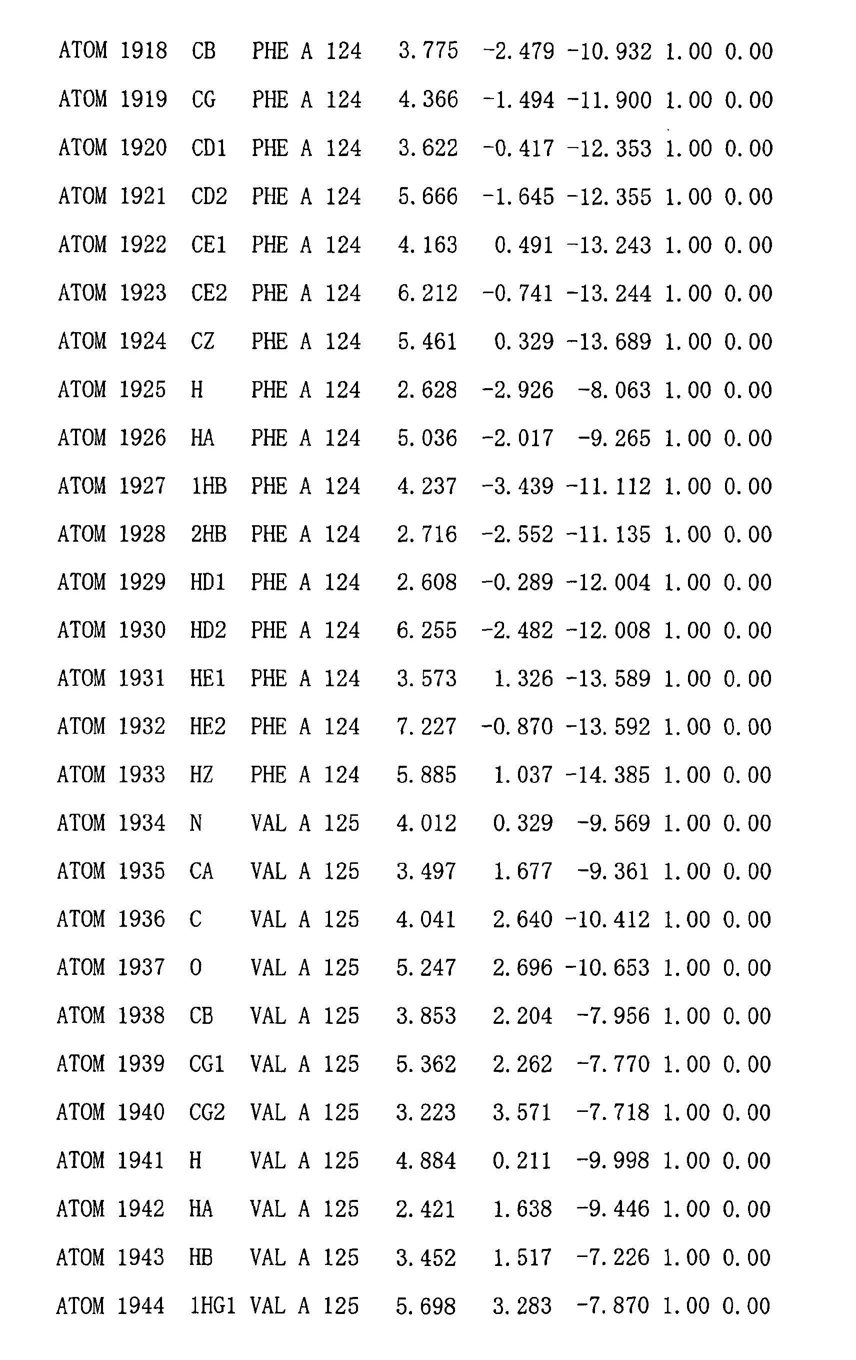

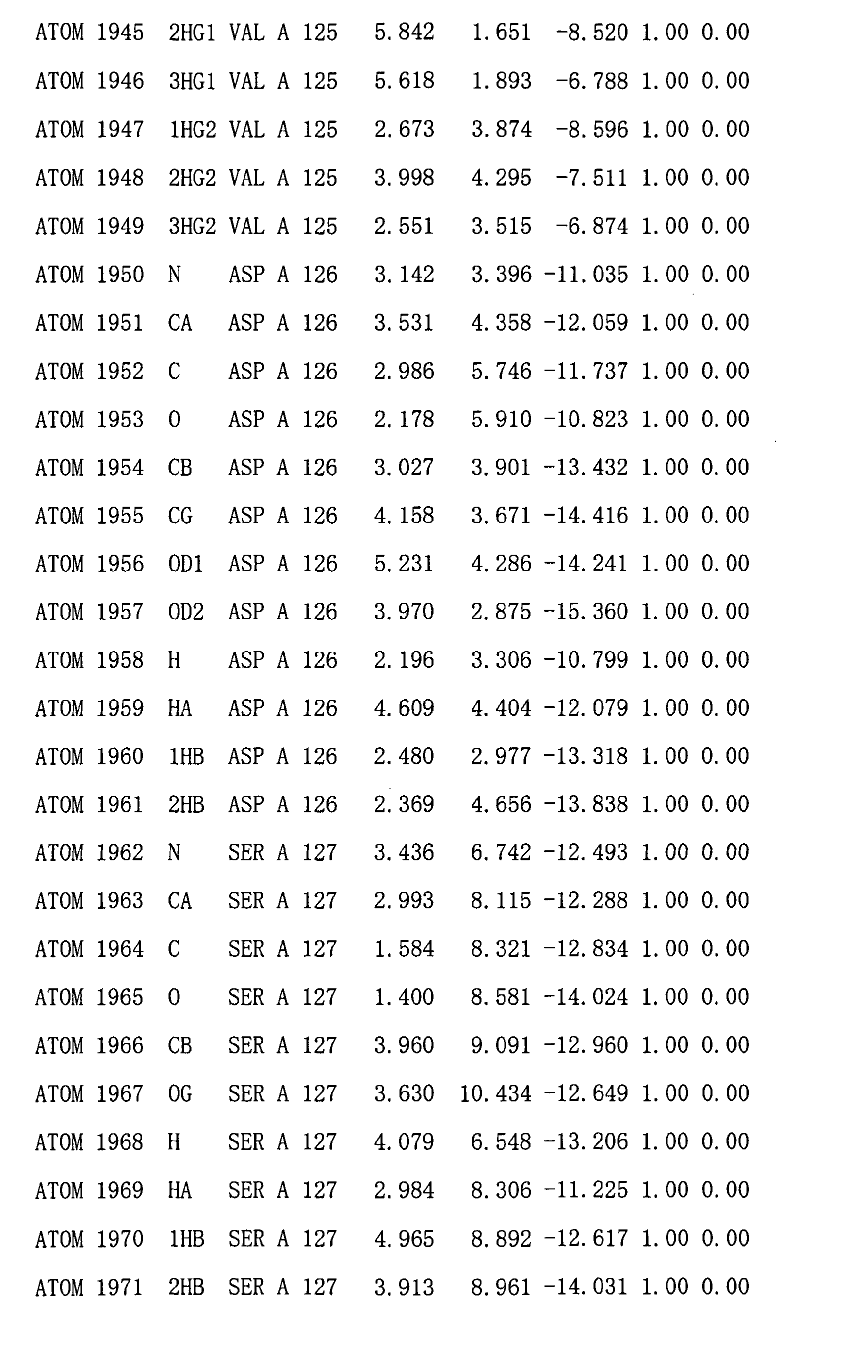

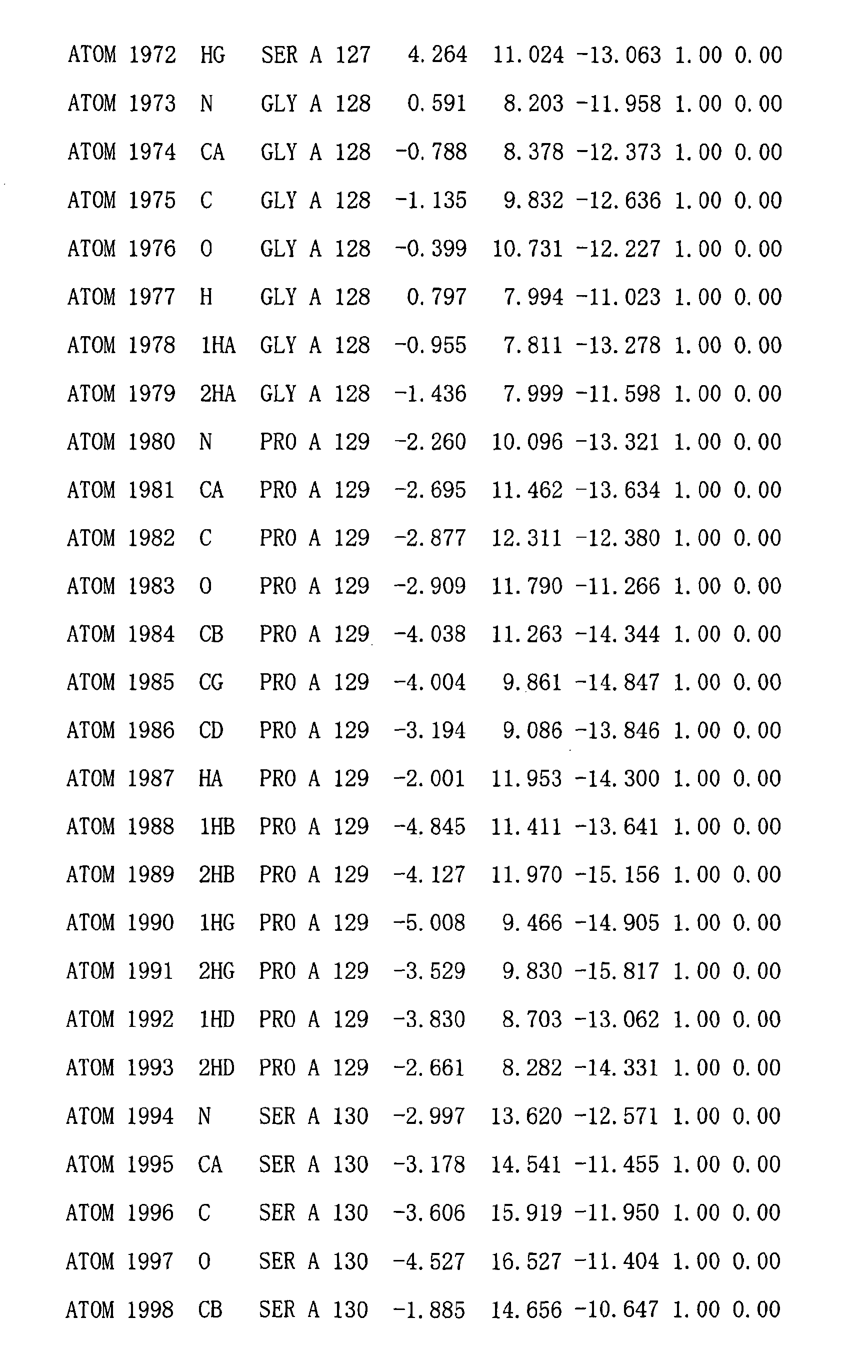

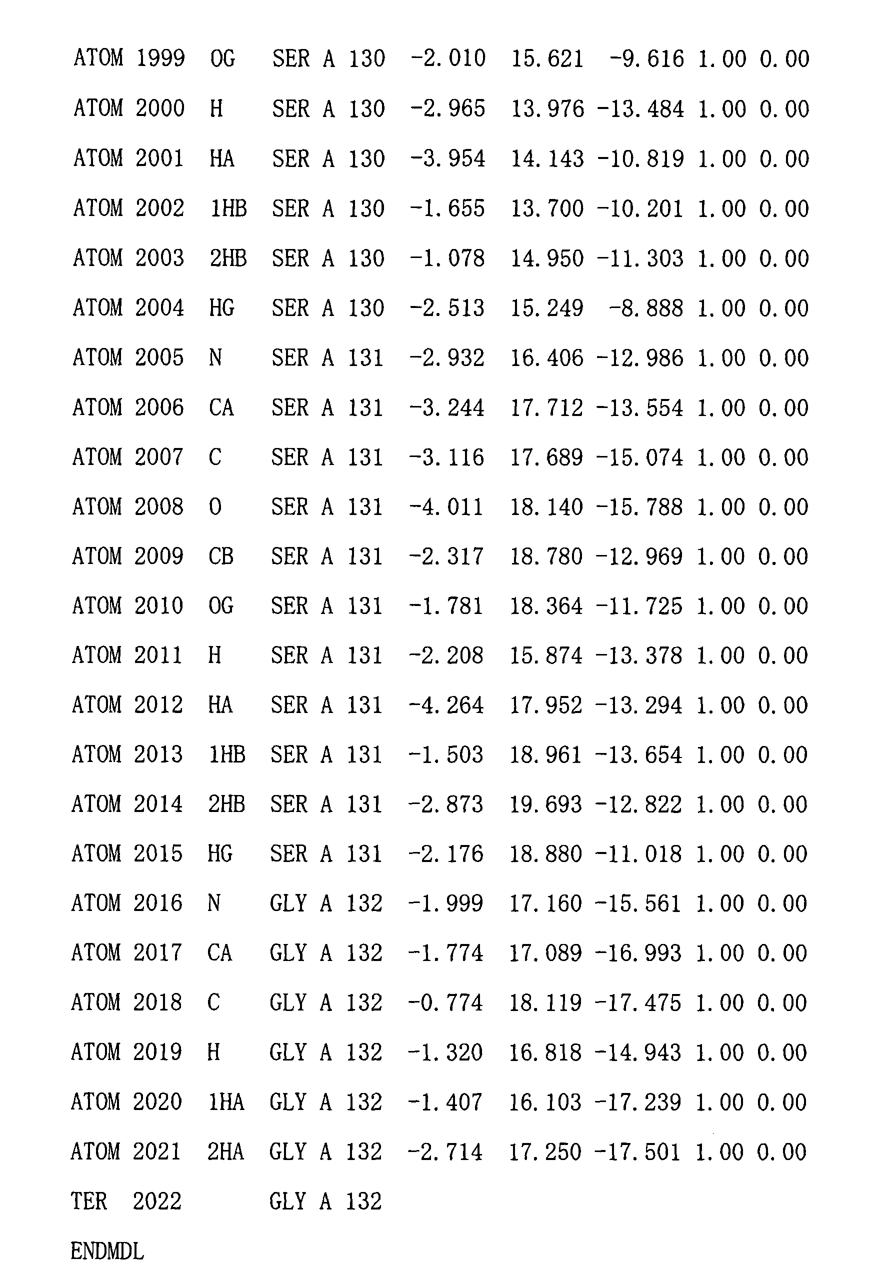

### Three-DimensionalStructure Coordinate 5

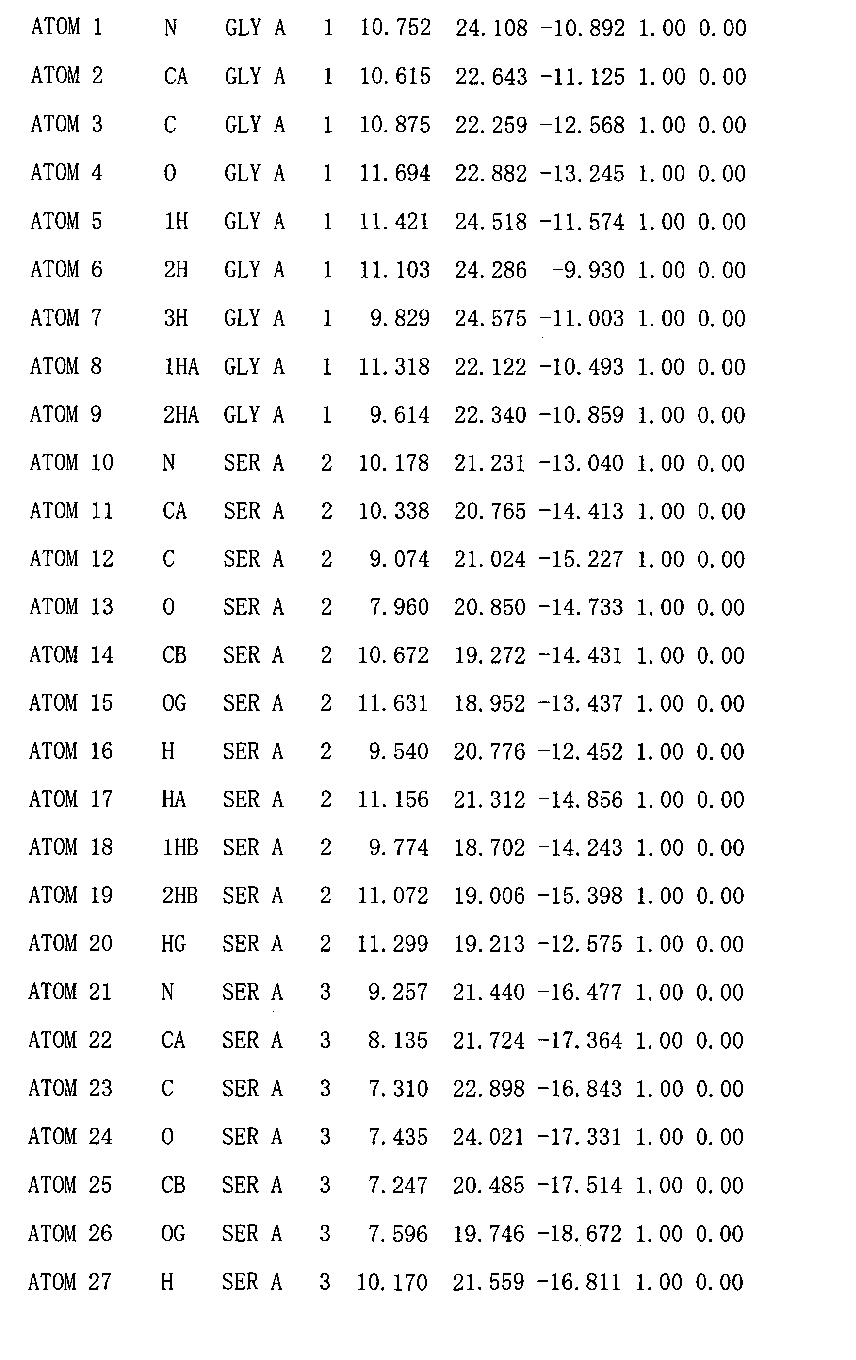

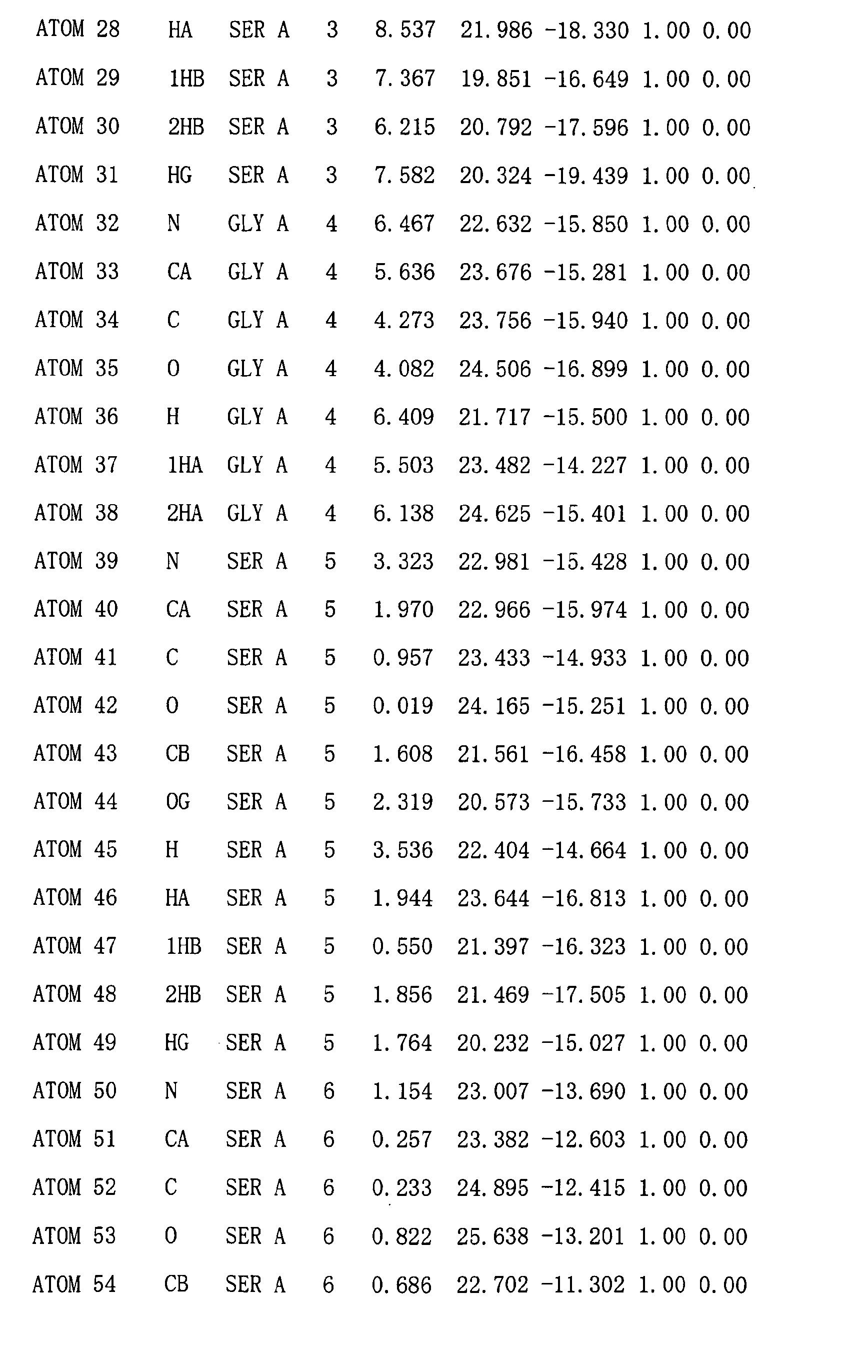

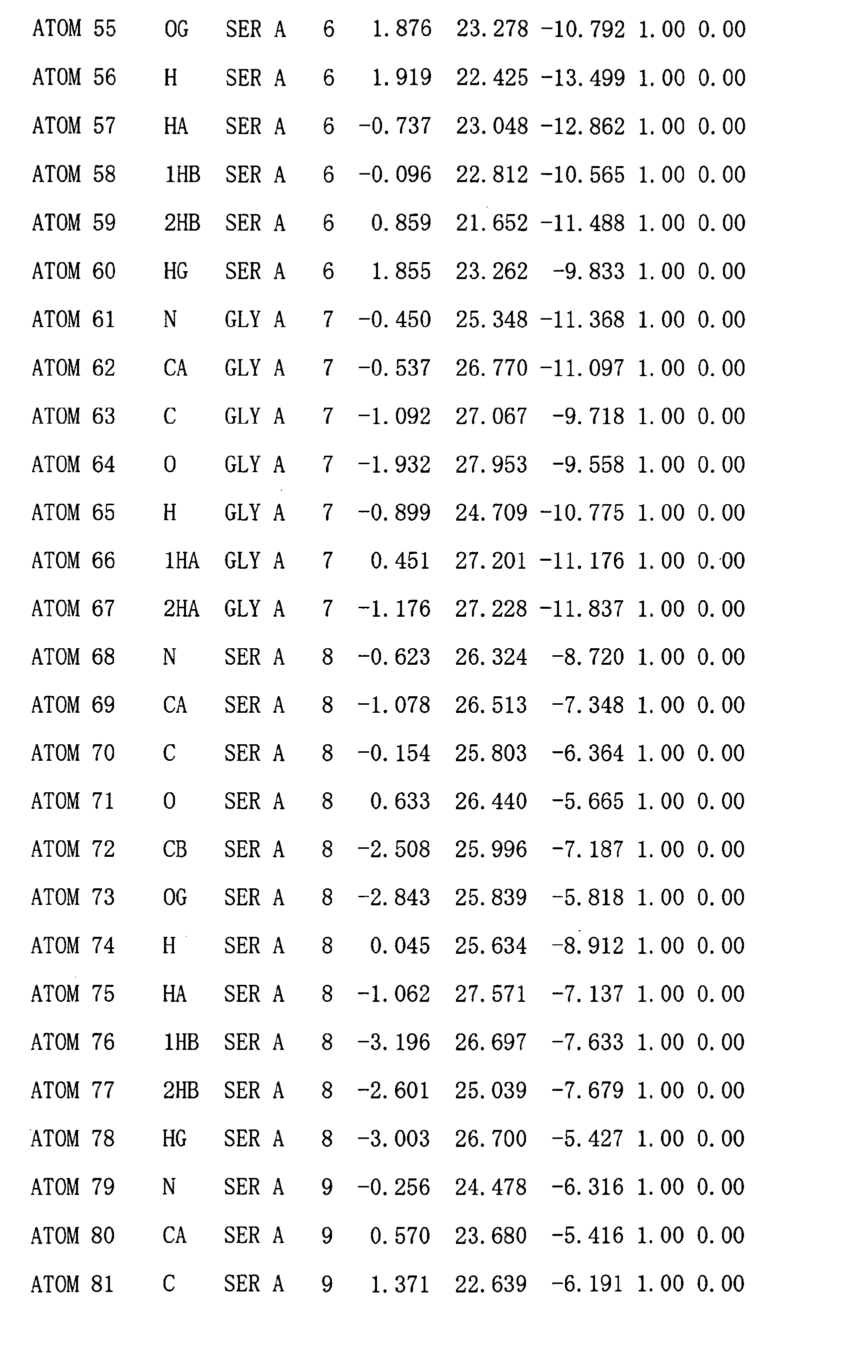

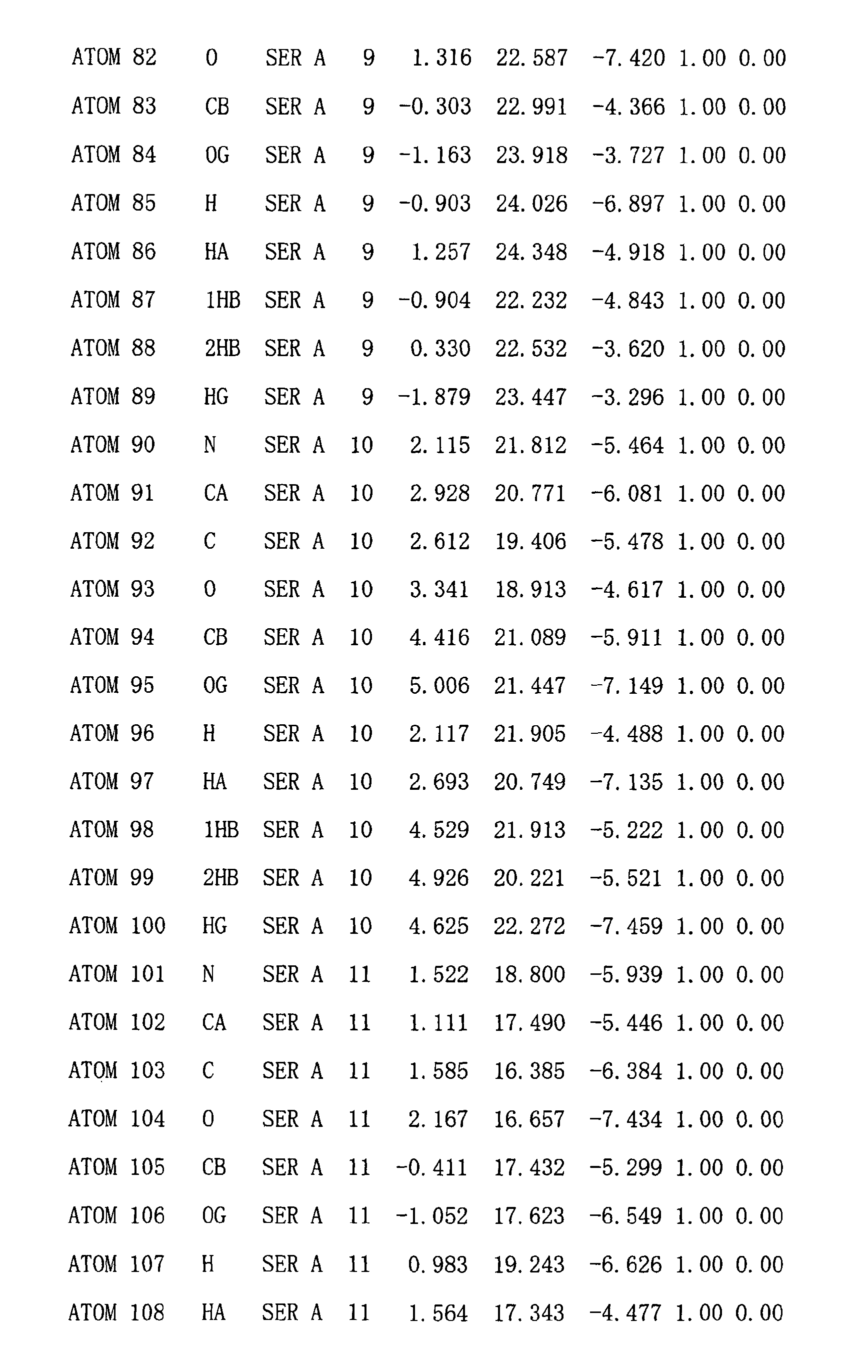

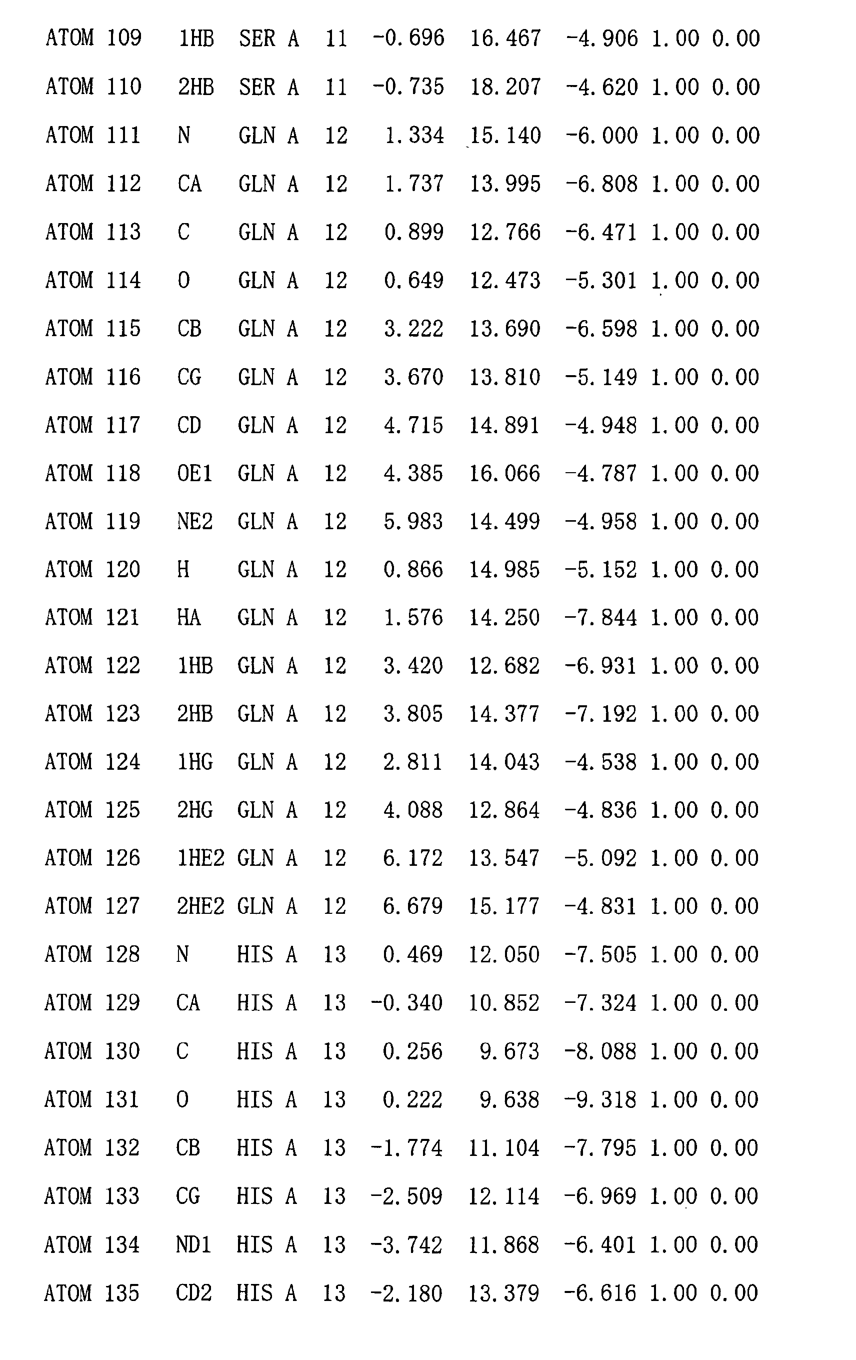

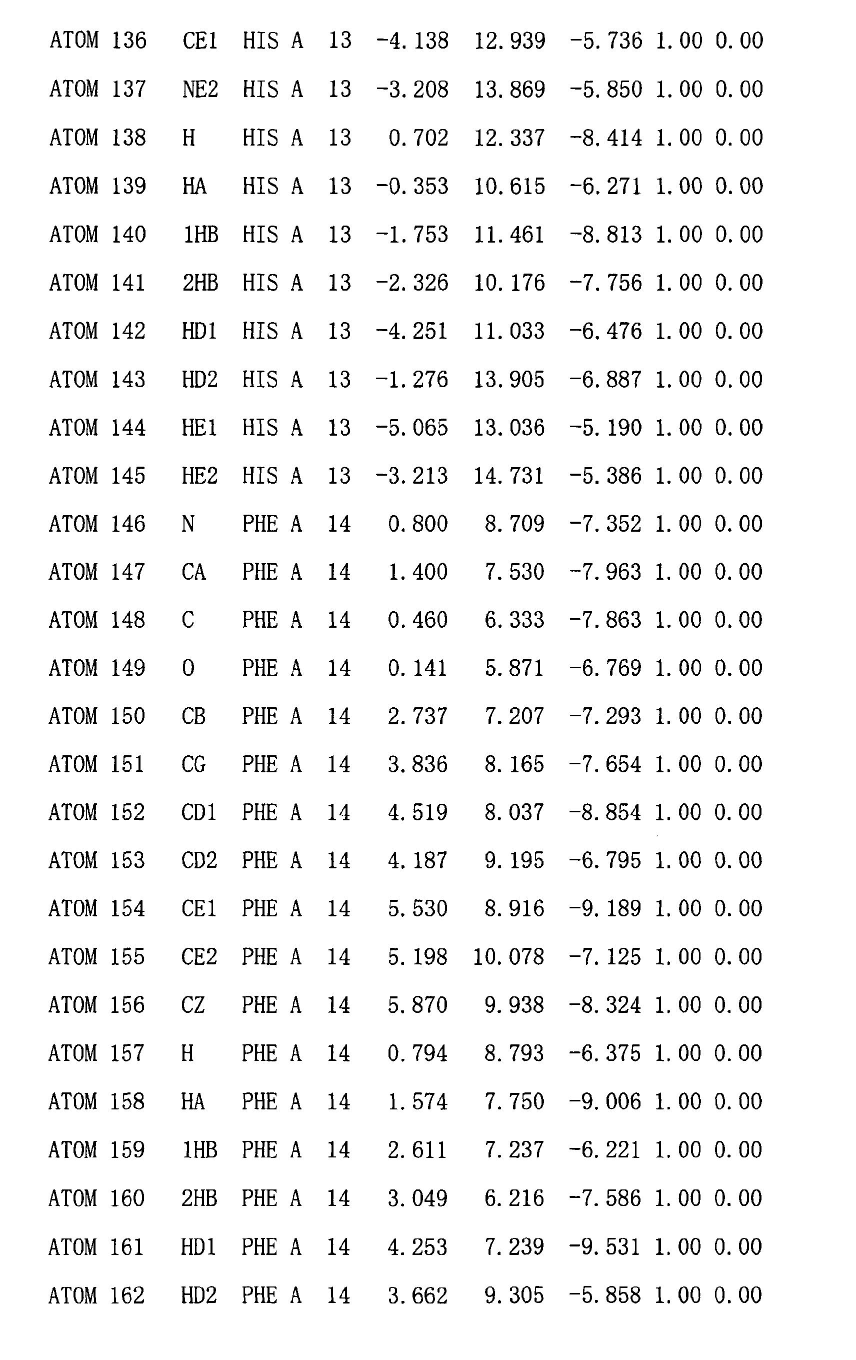

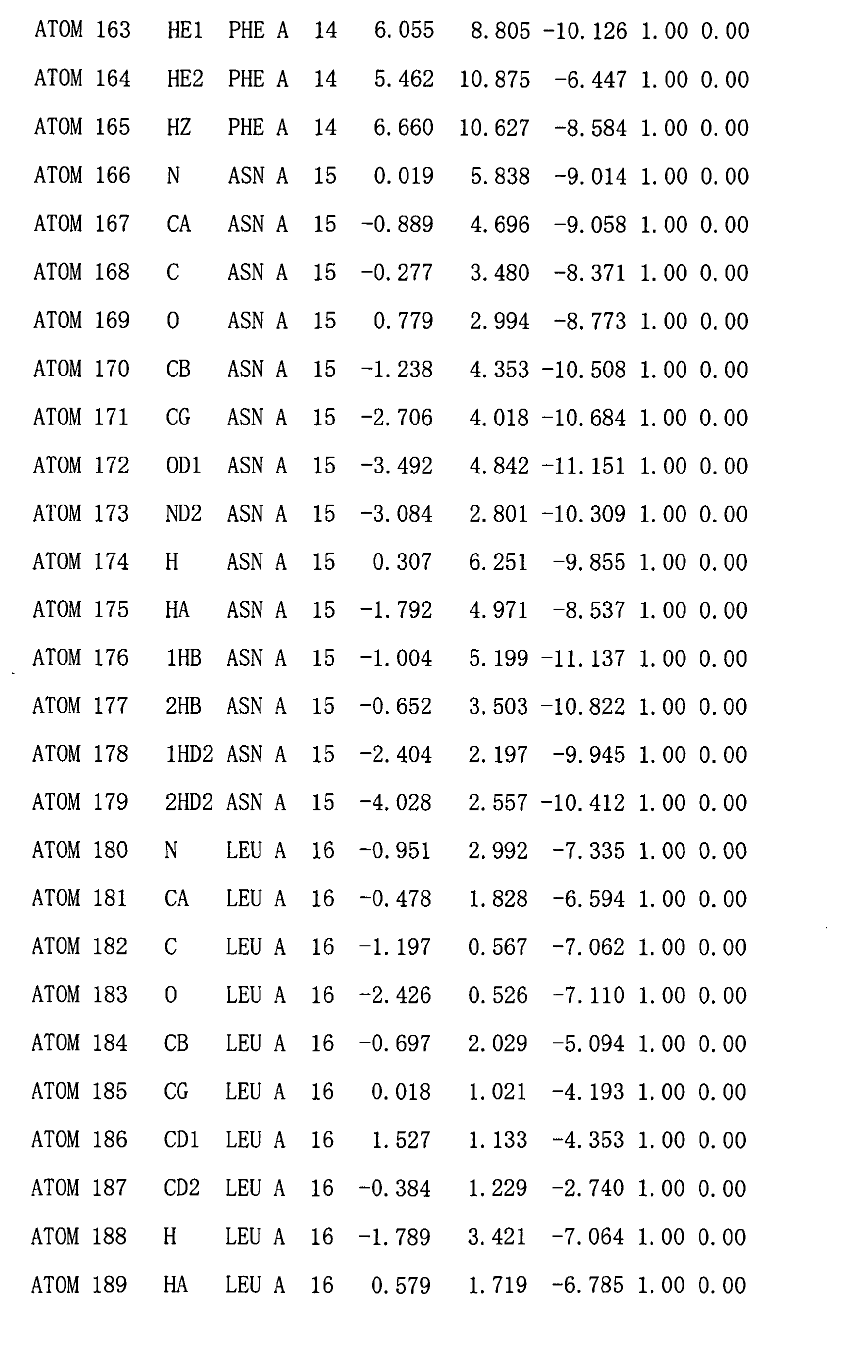

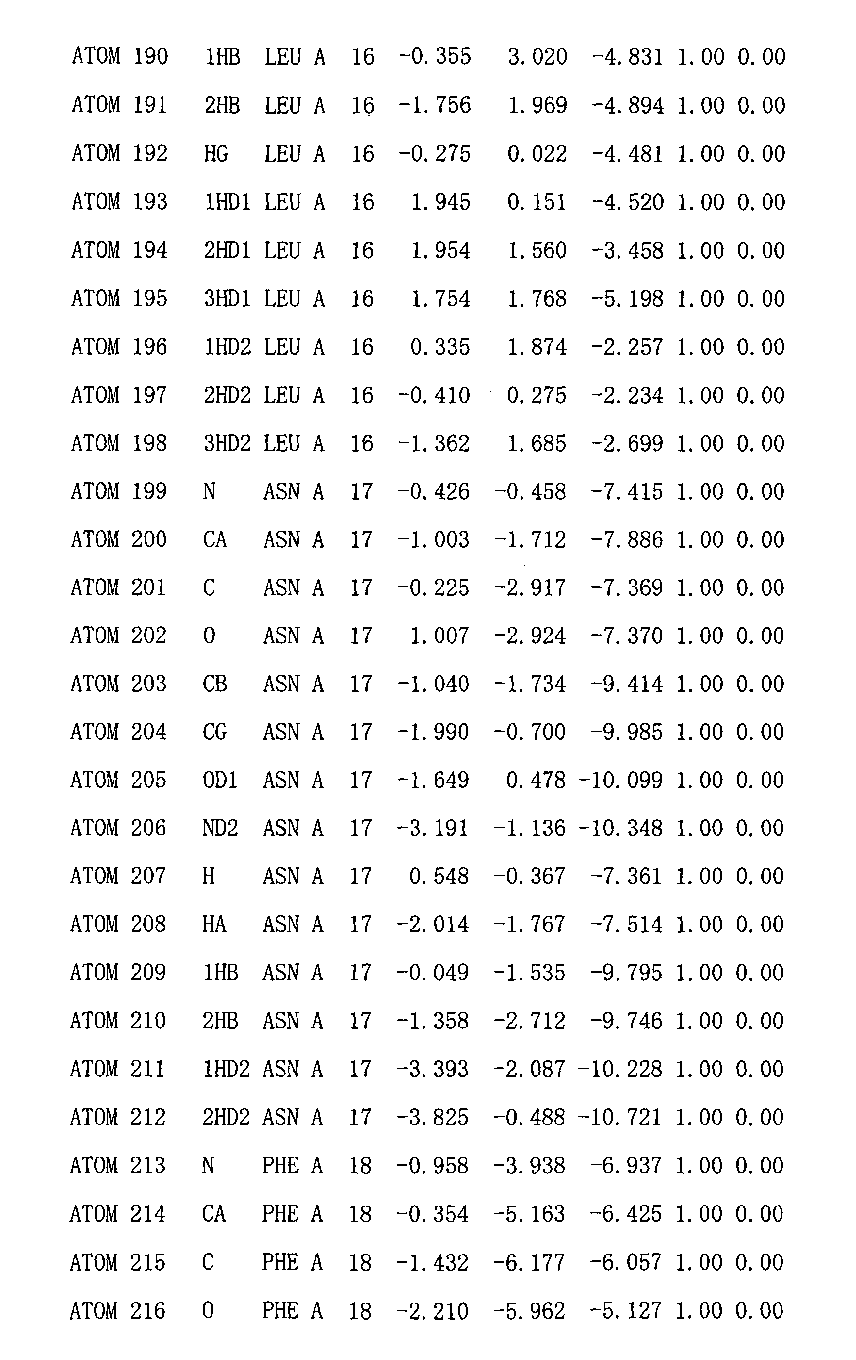

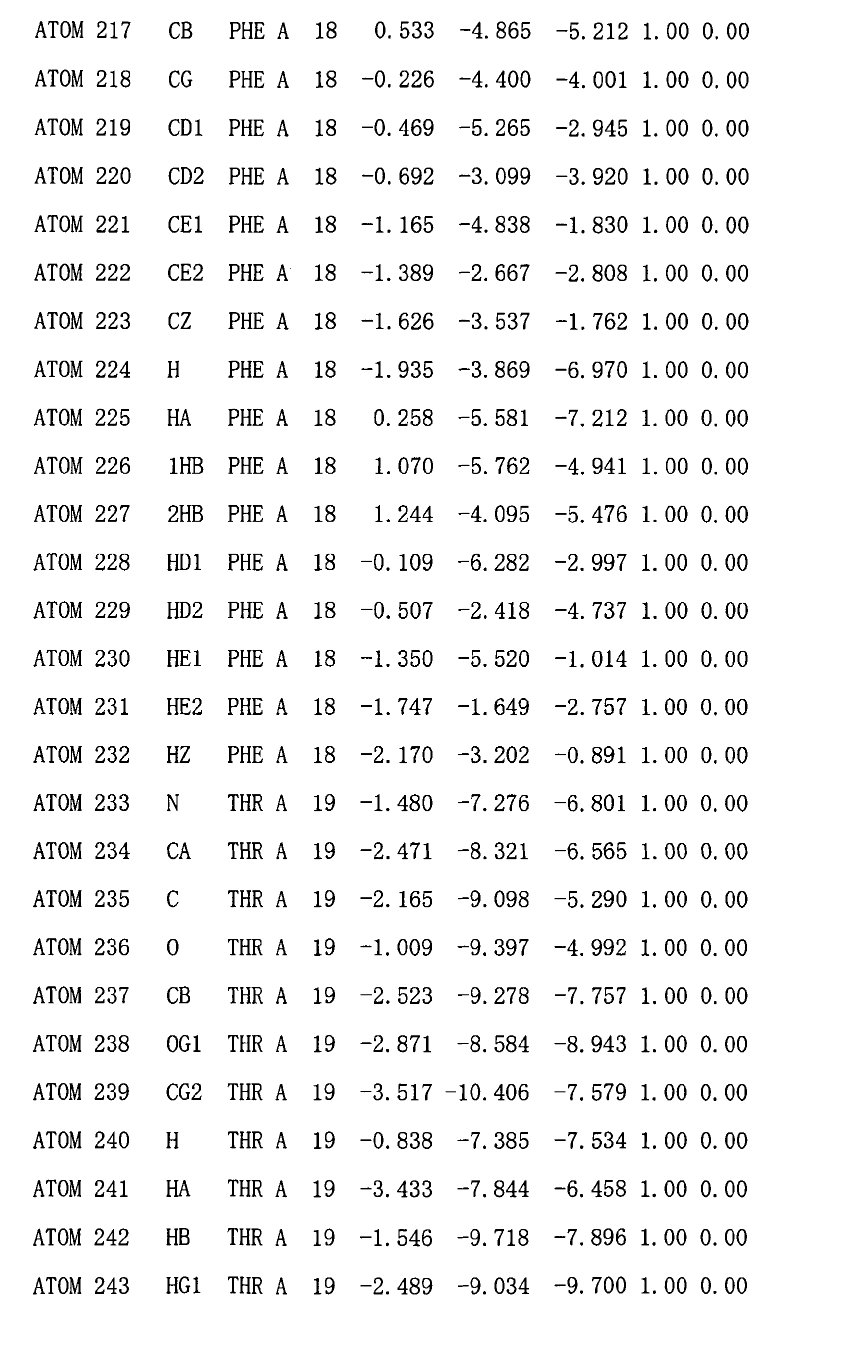

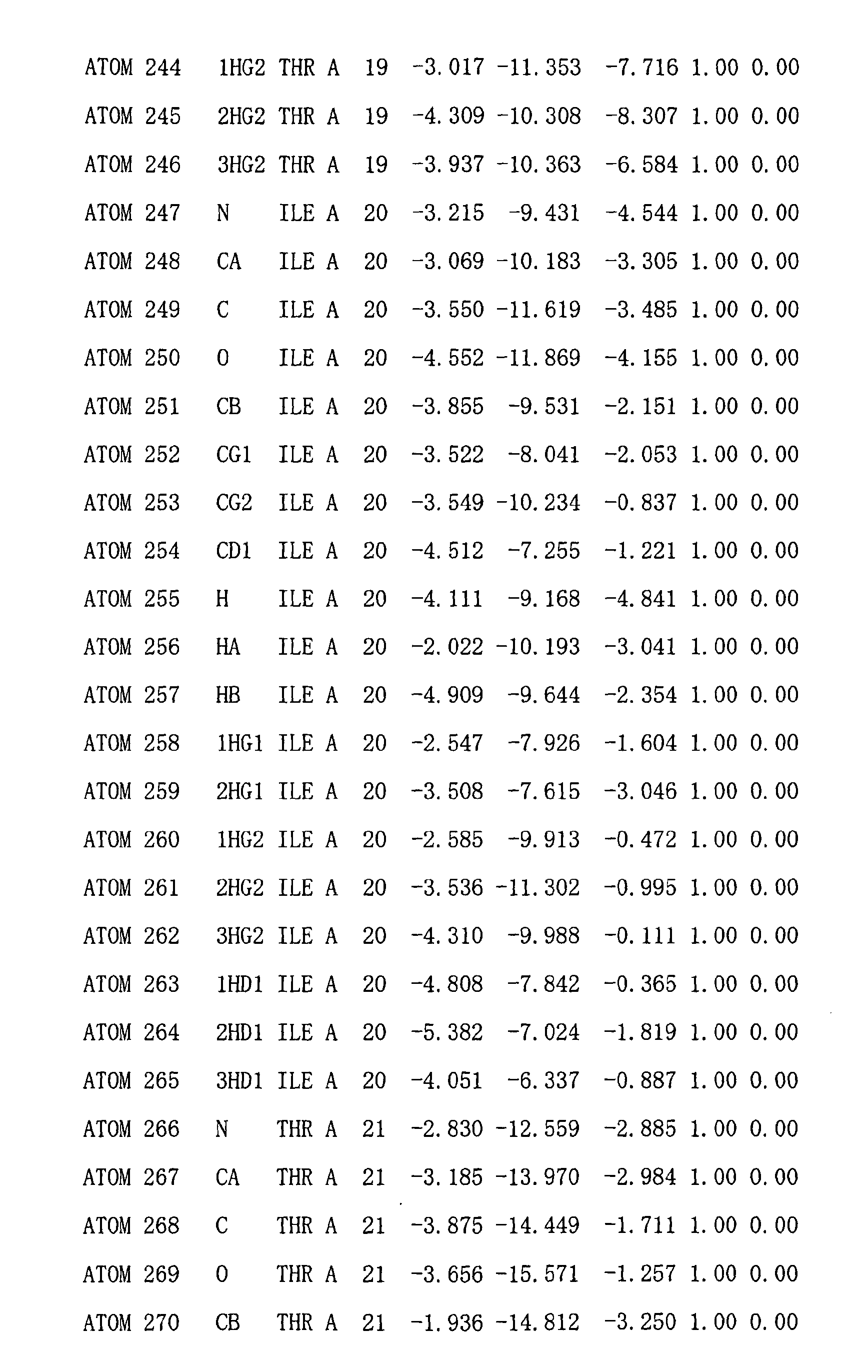

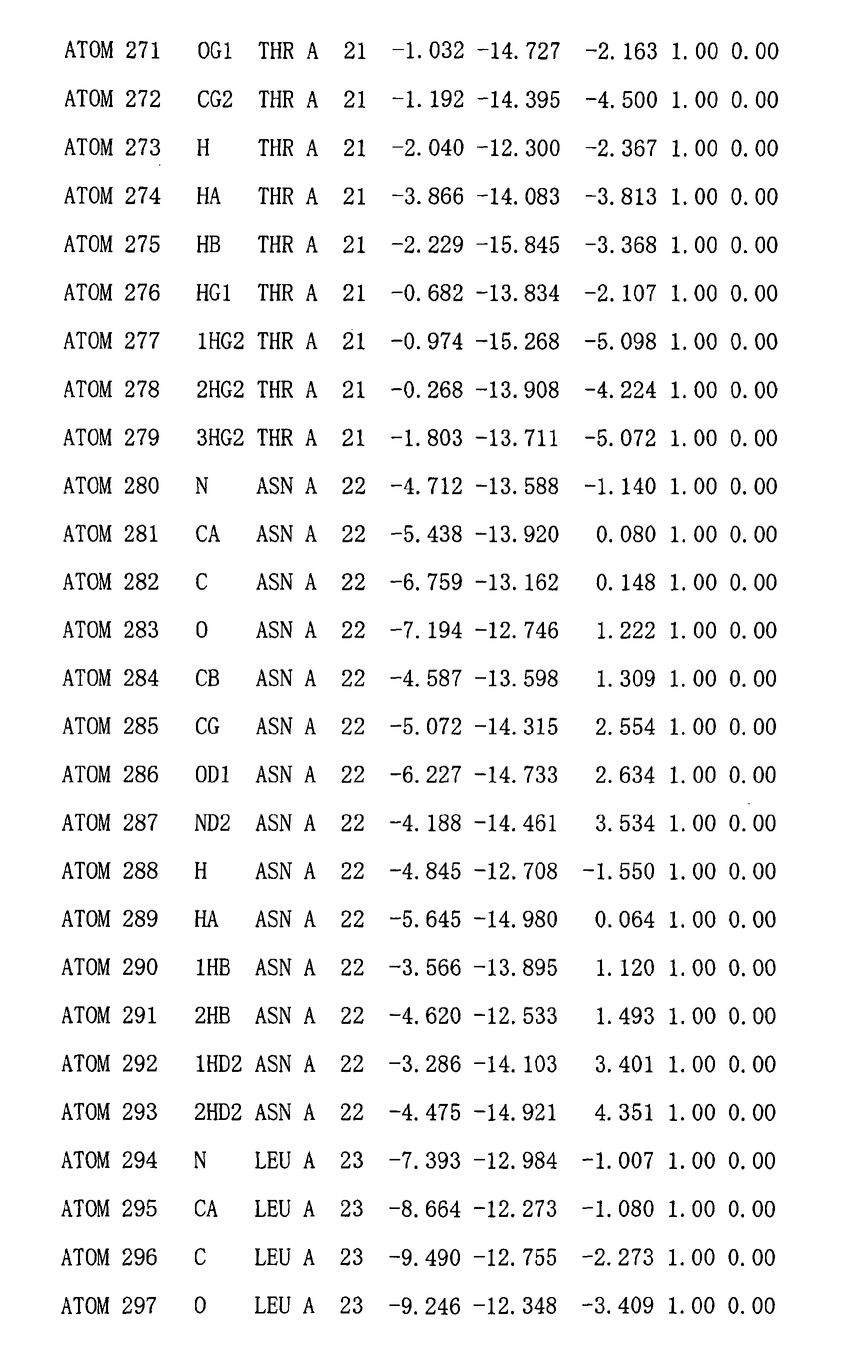

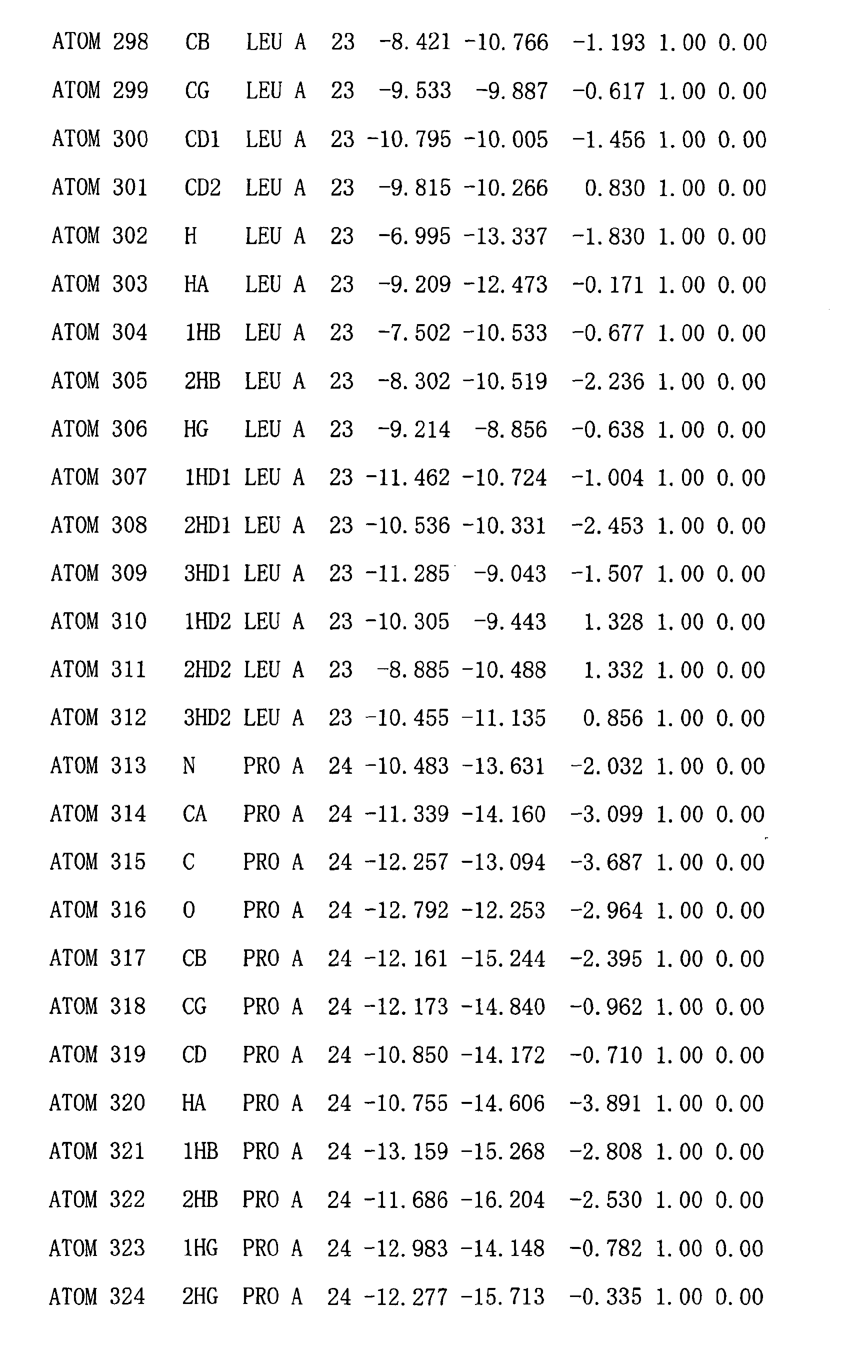

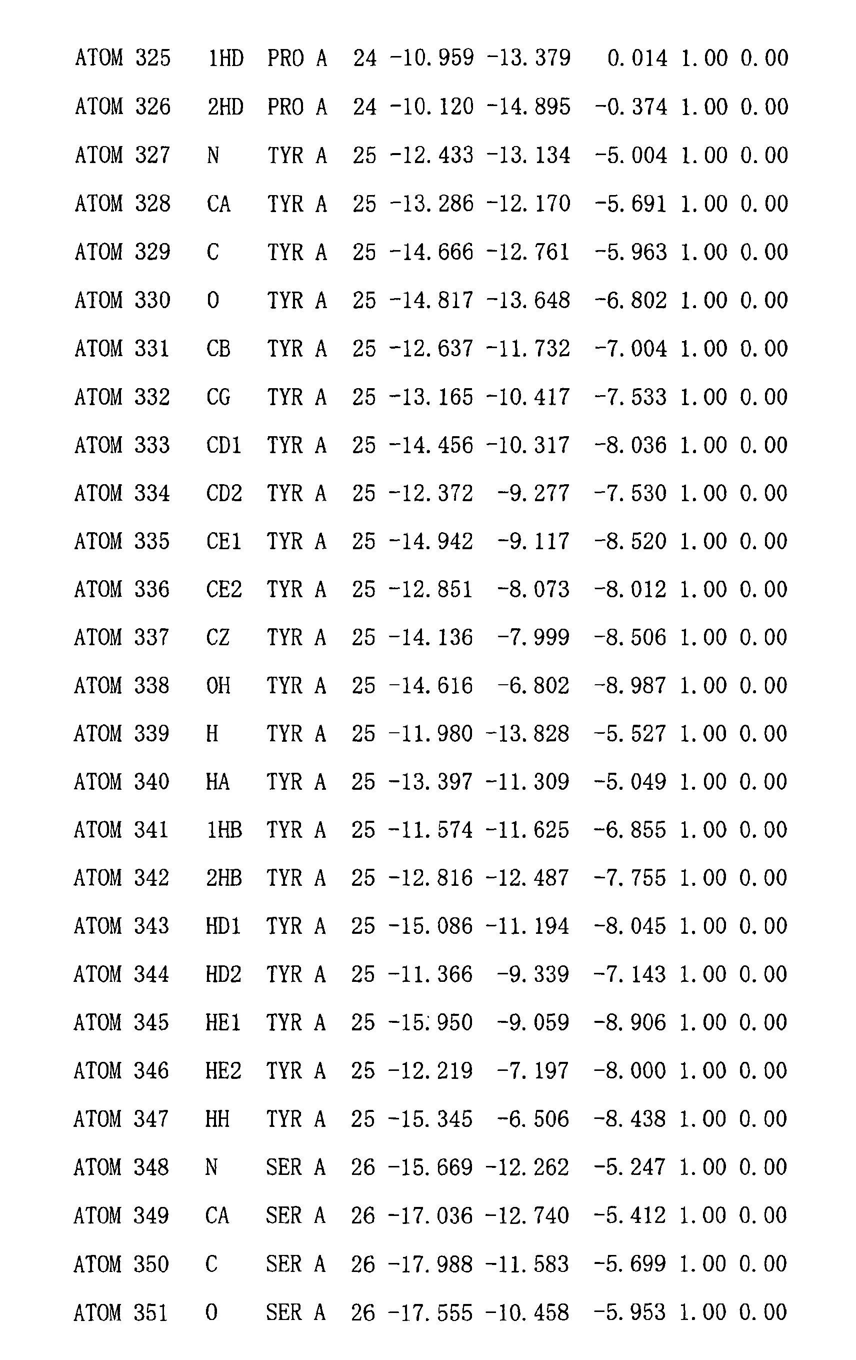

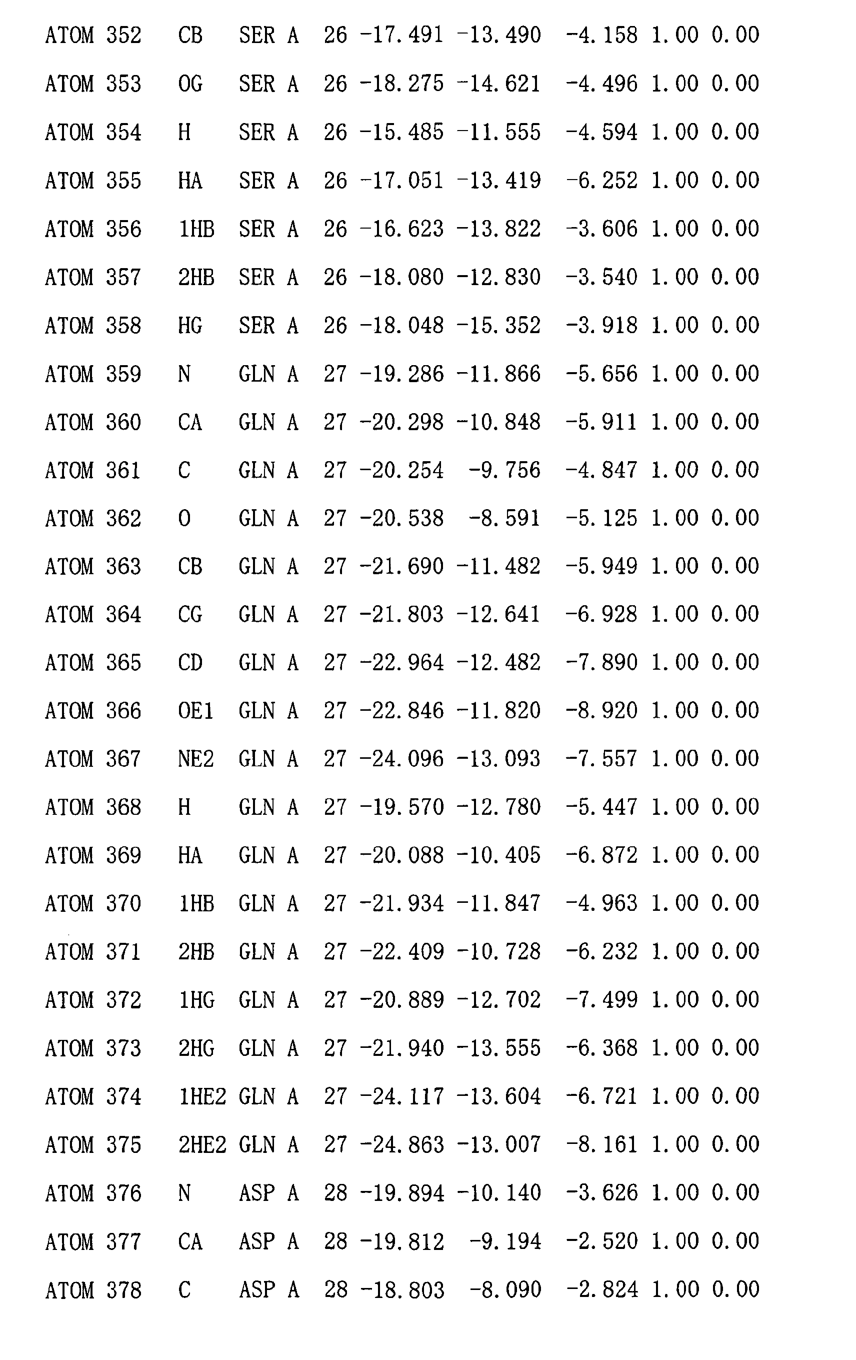

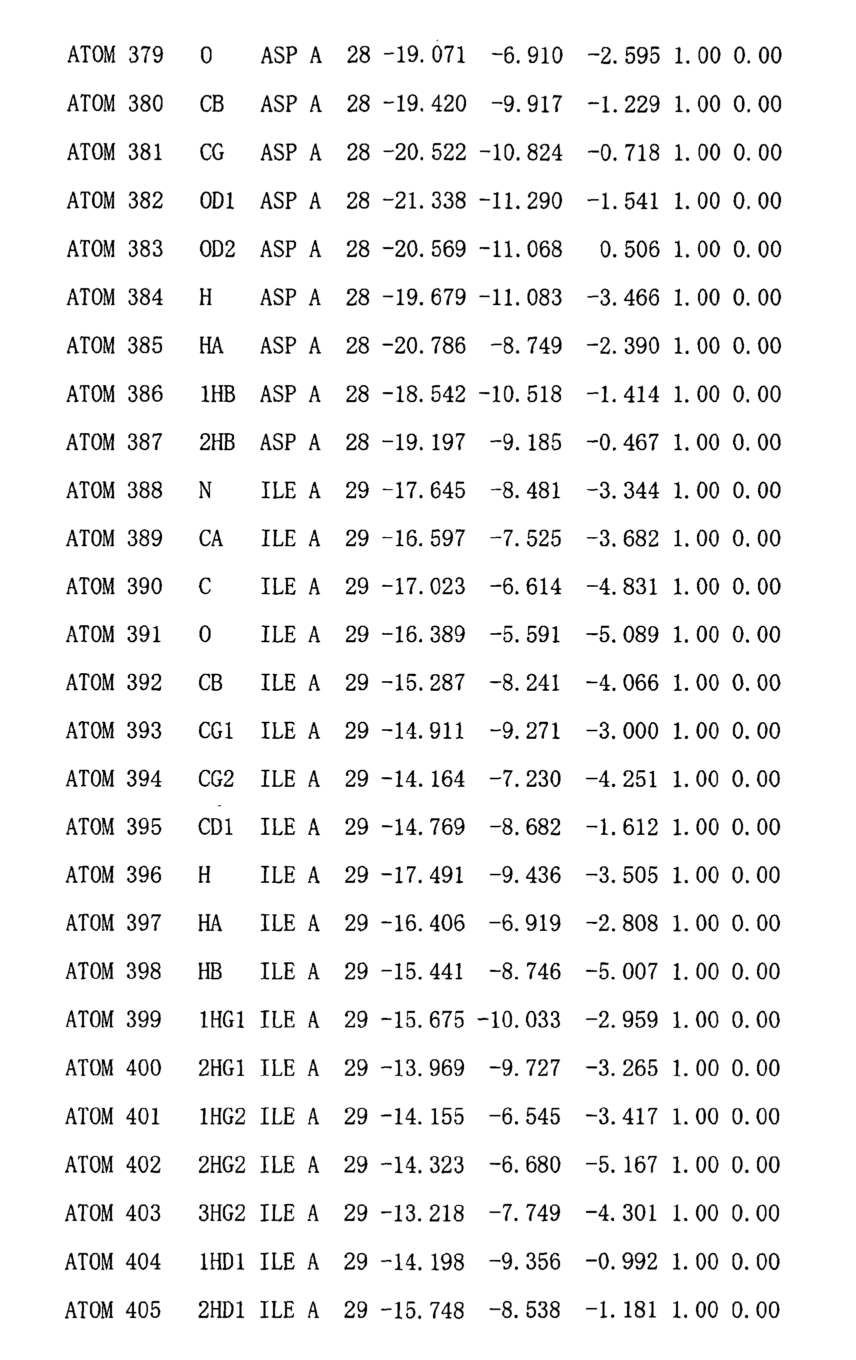

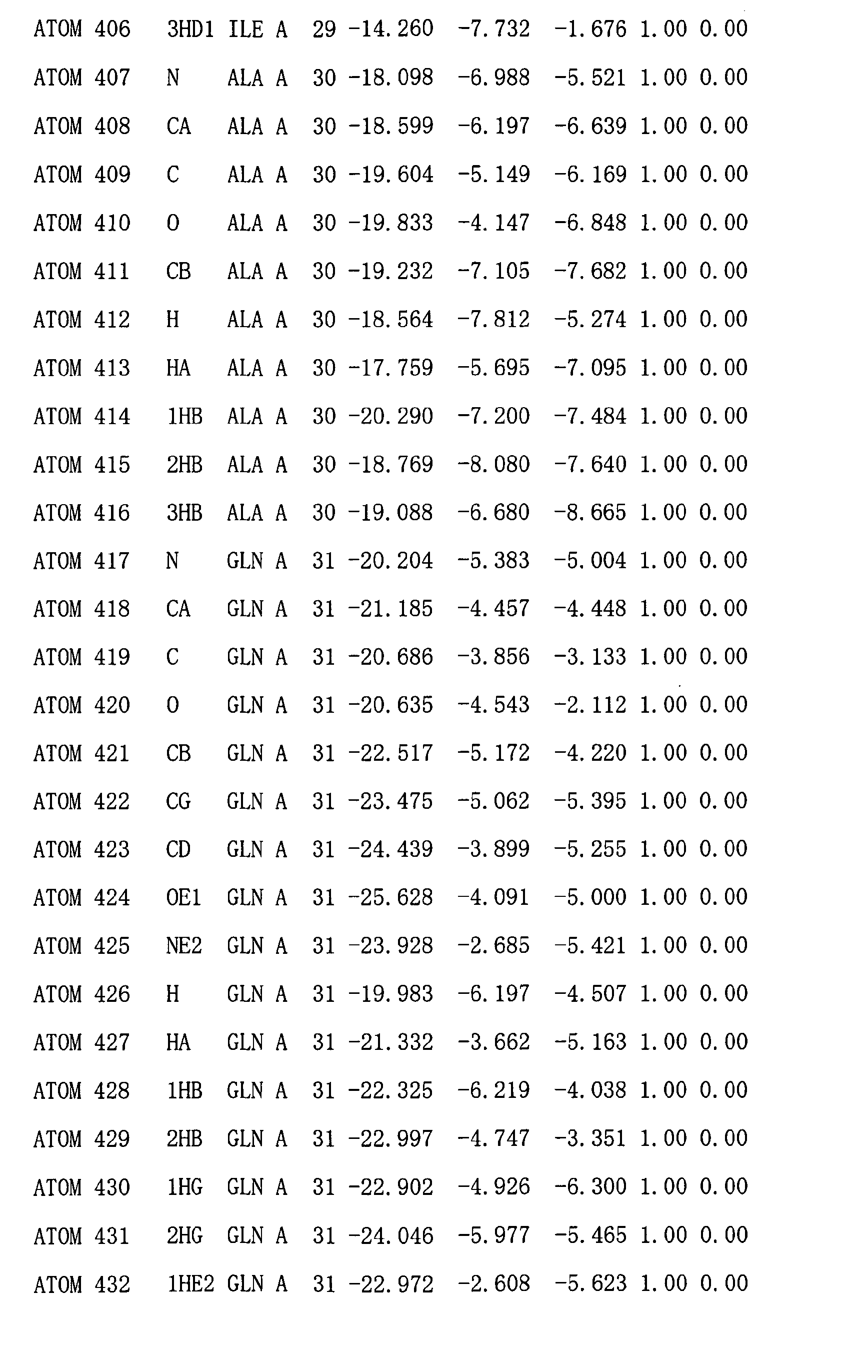

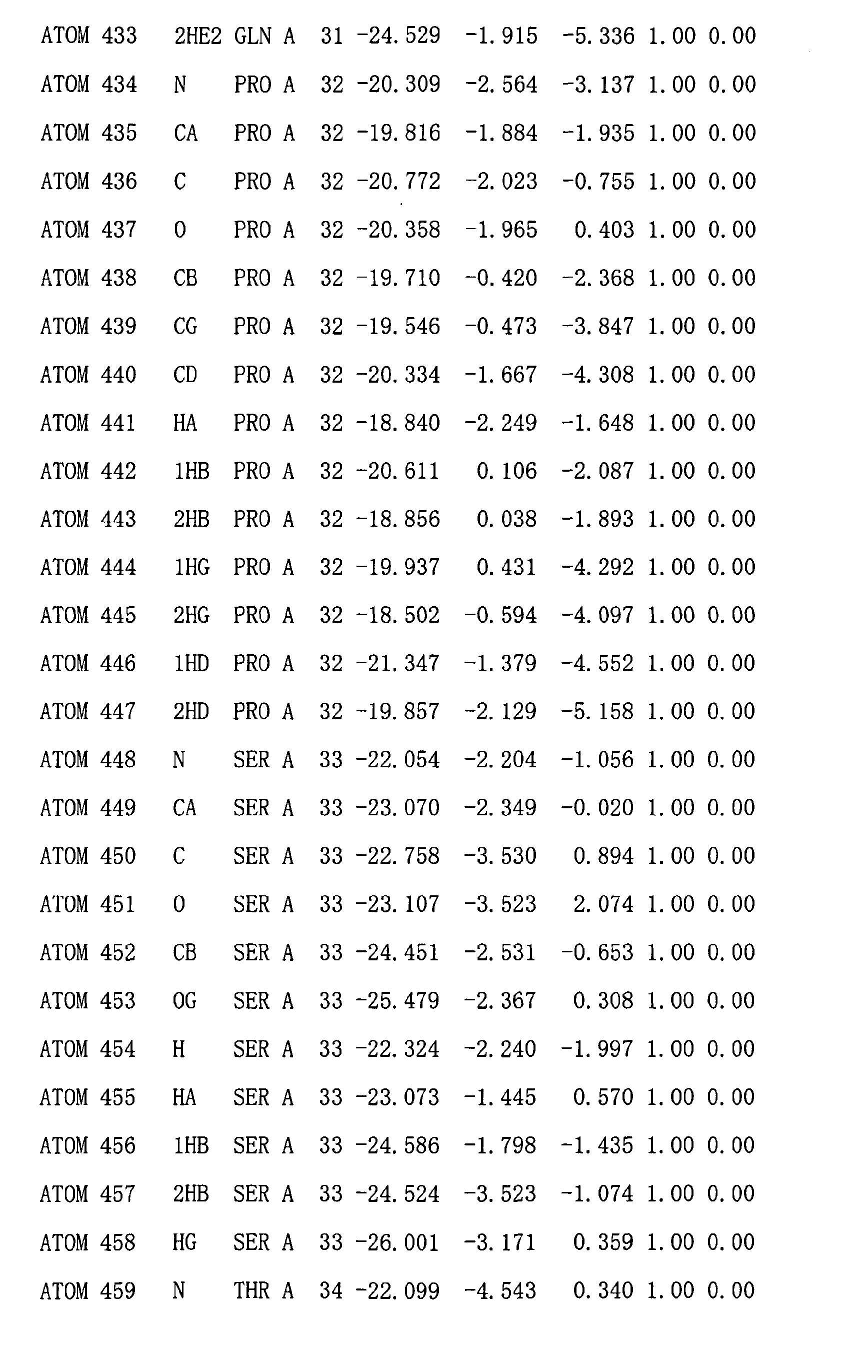

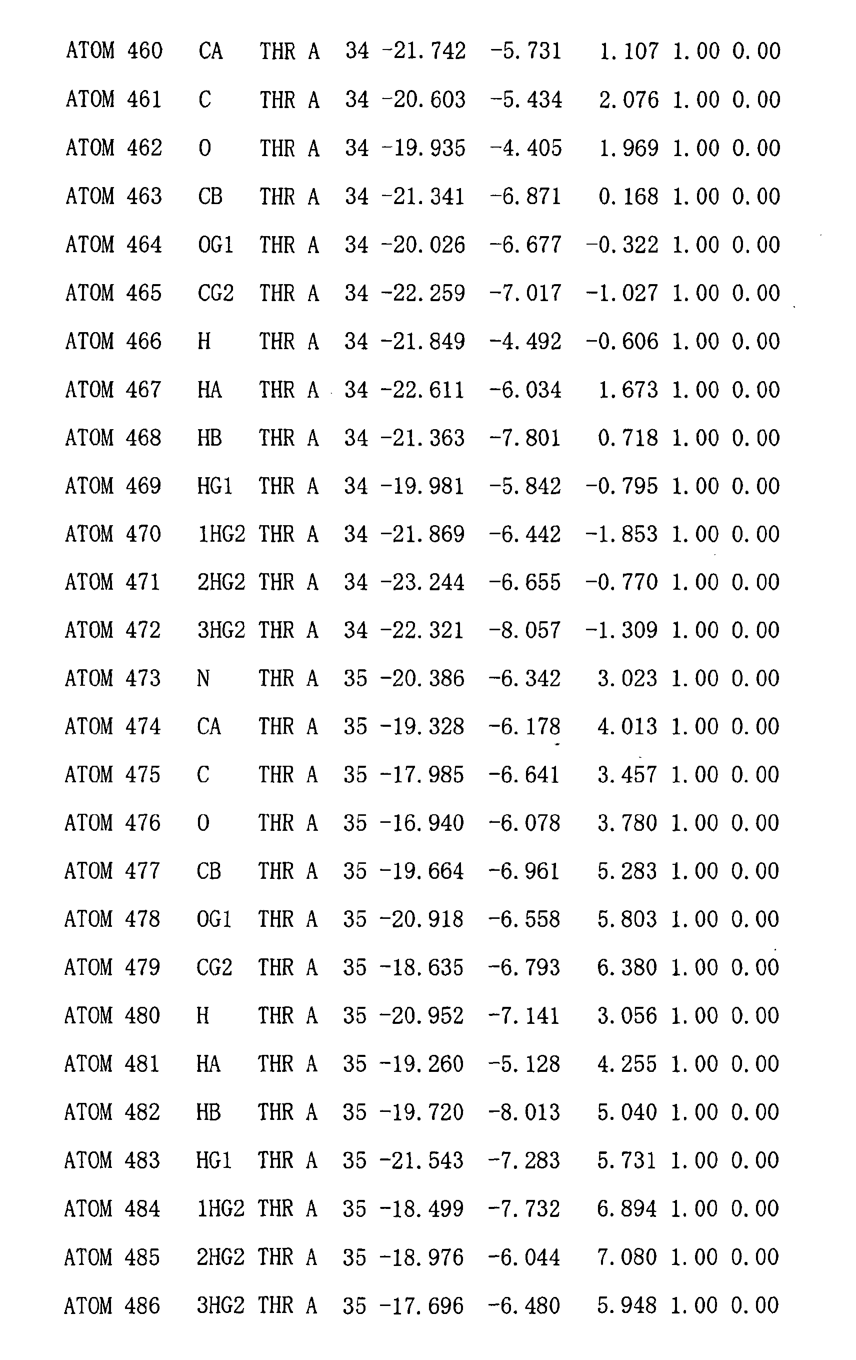

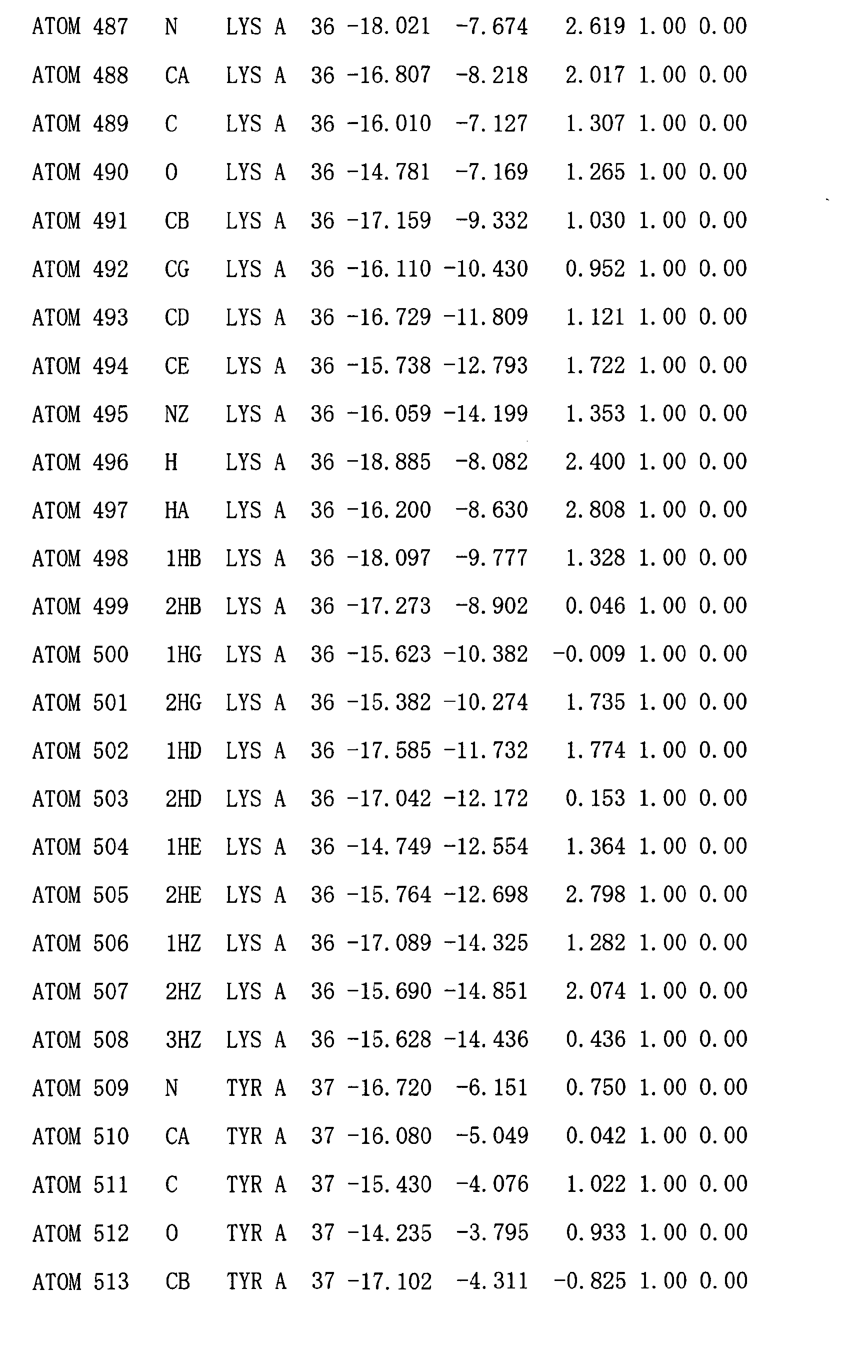

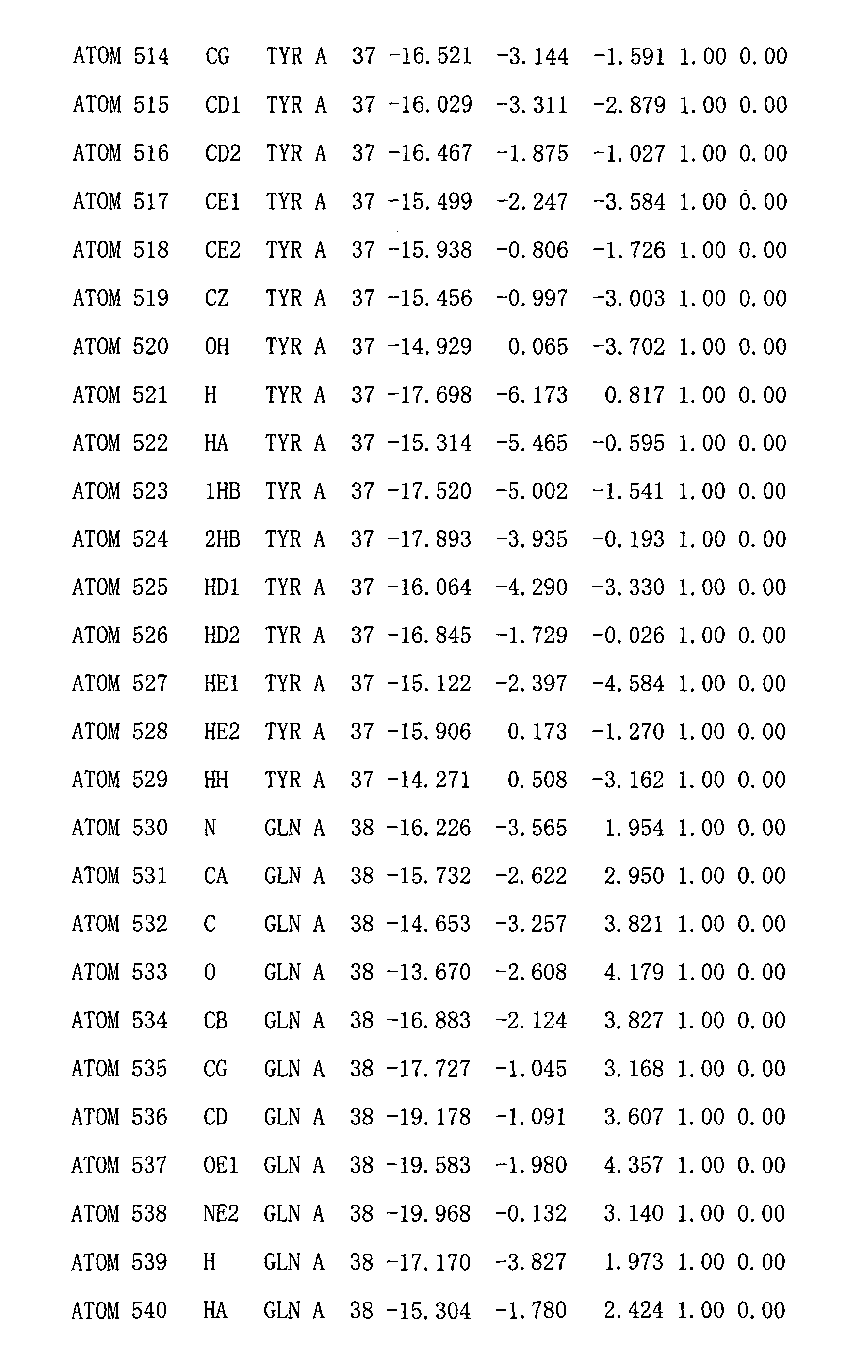

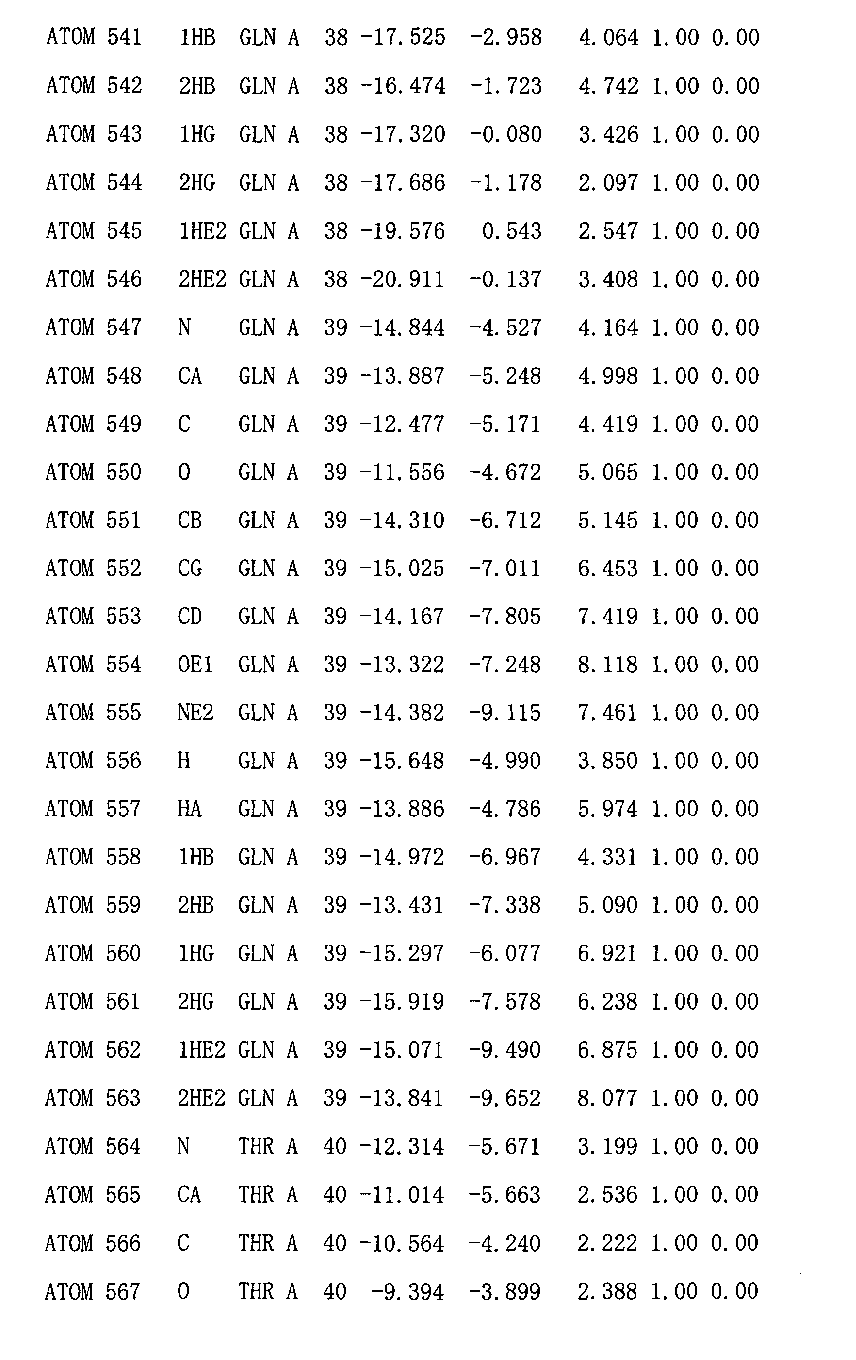

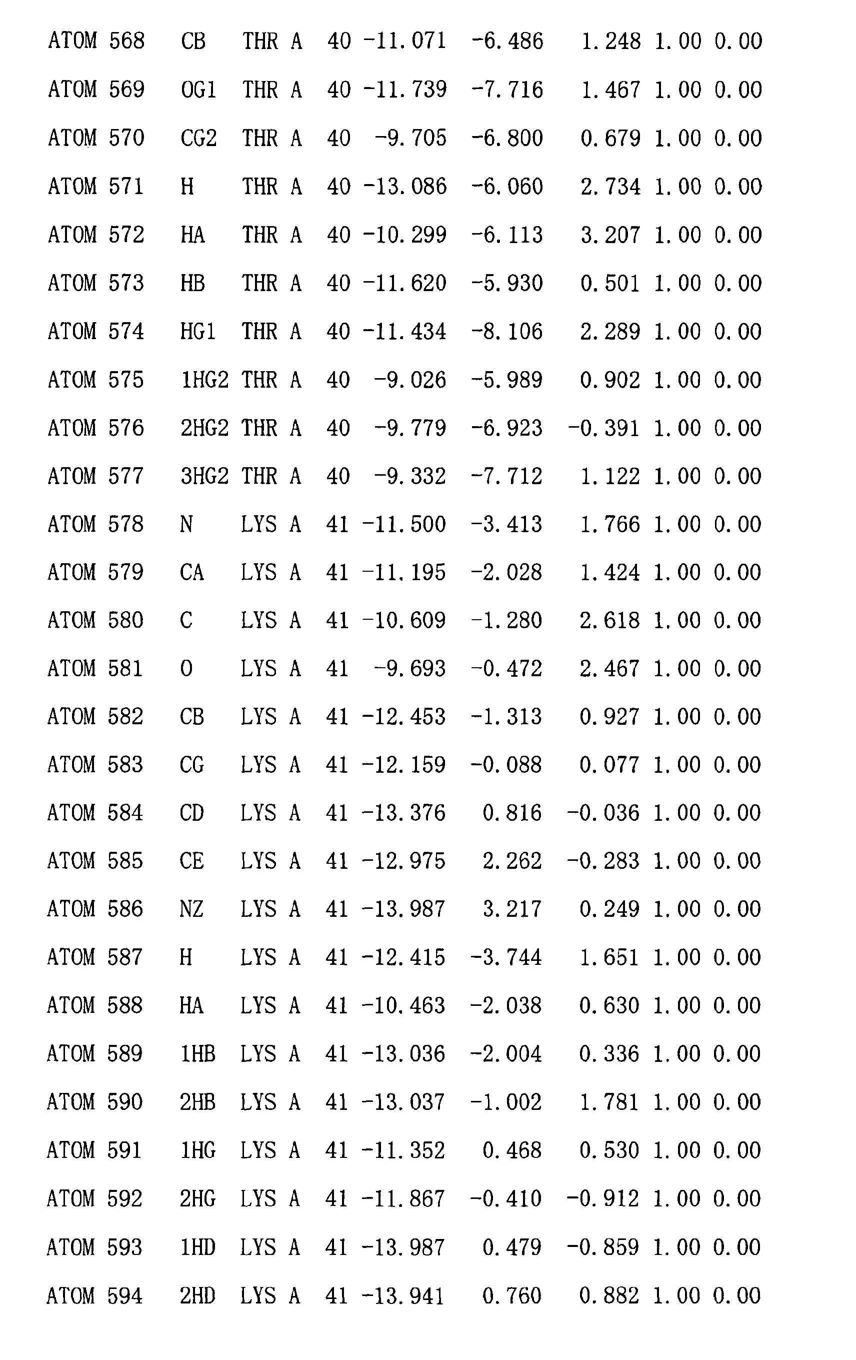

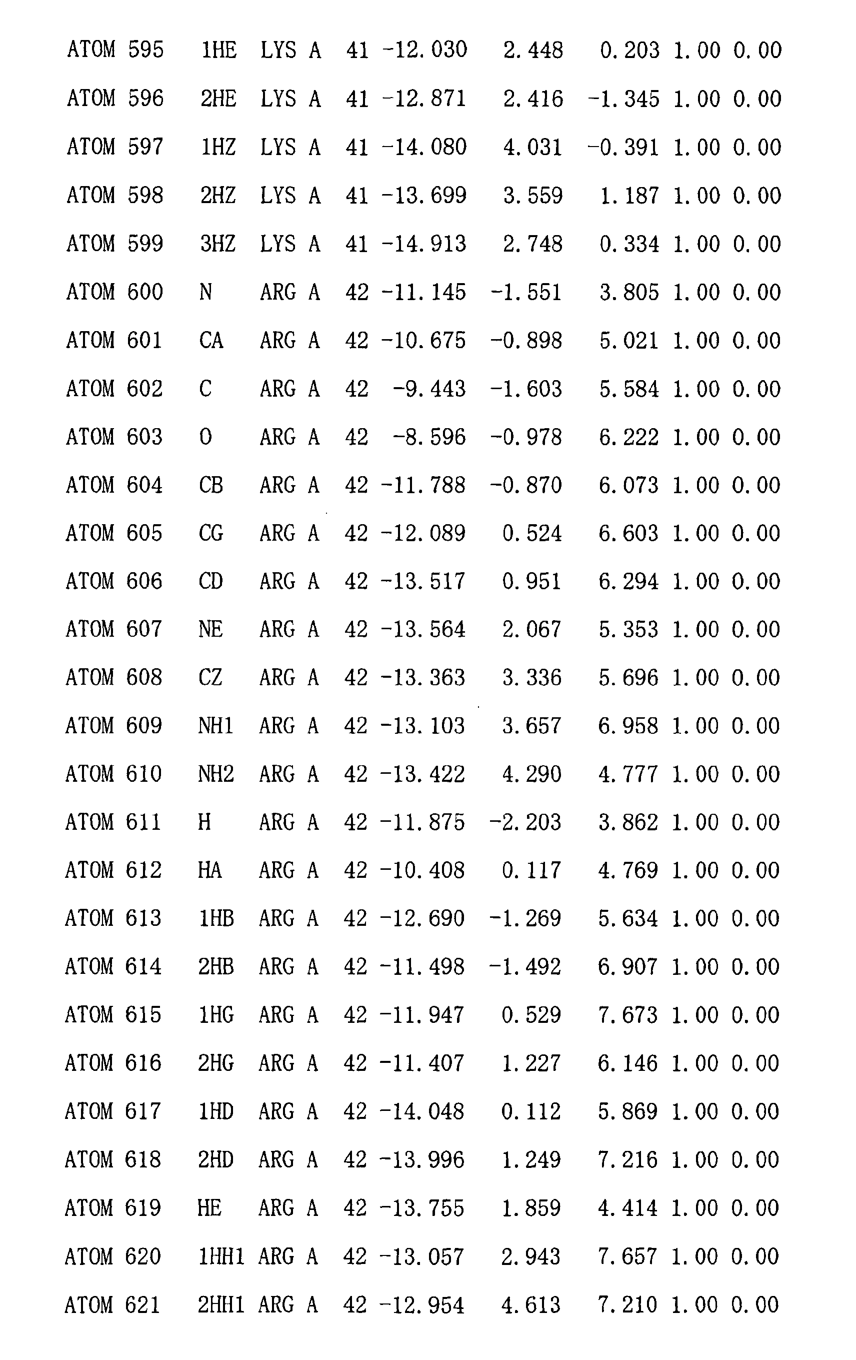

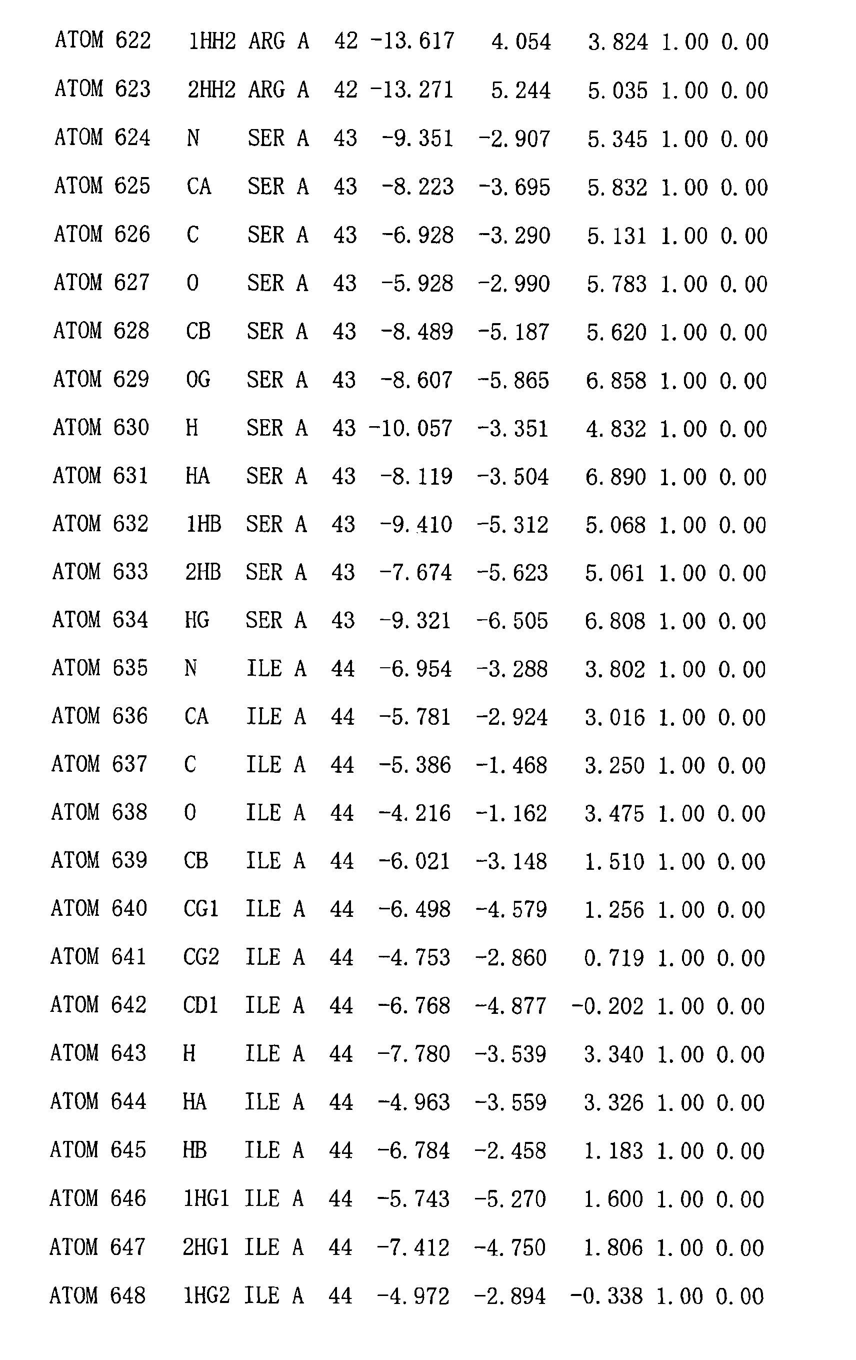

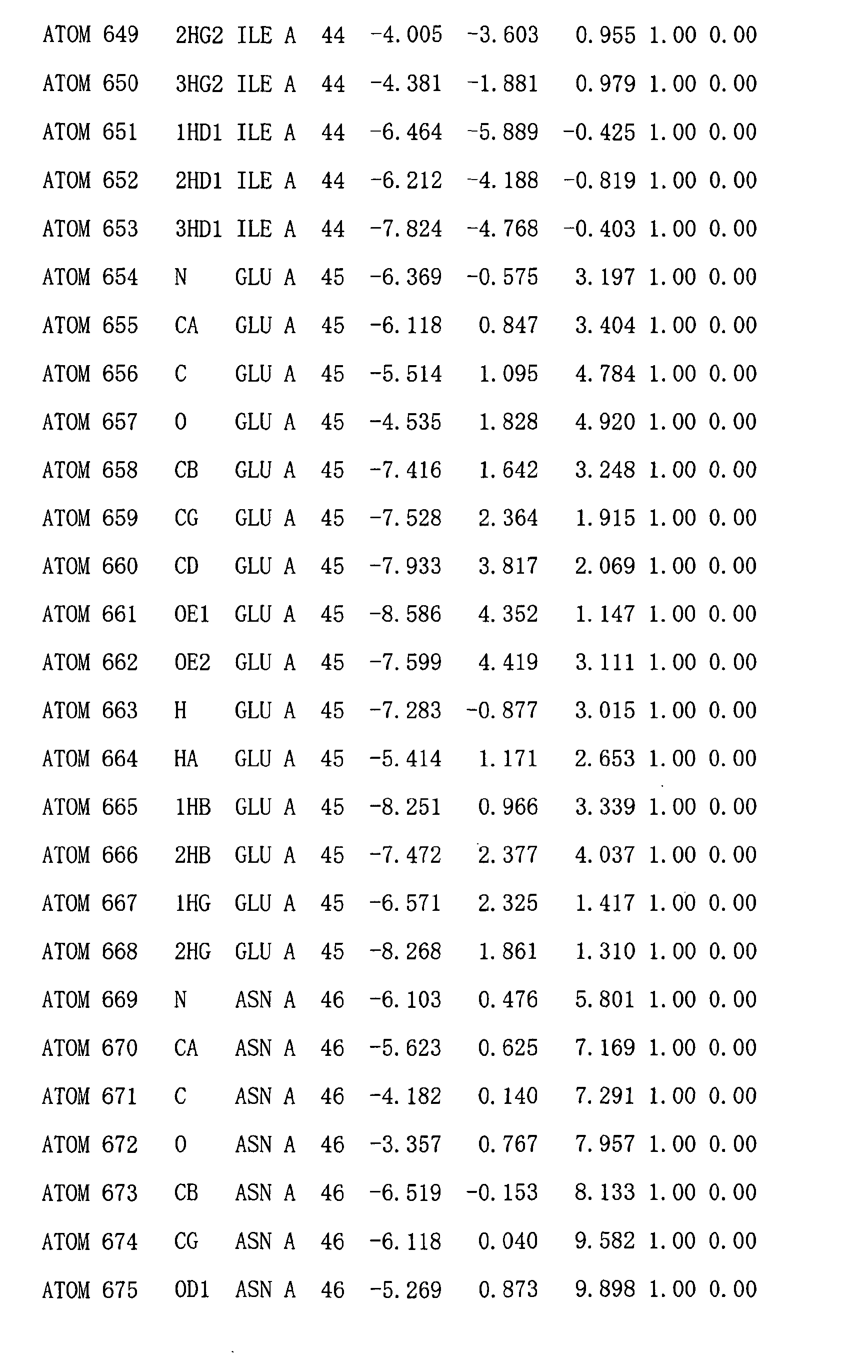

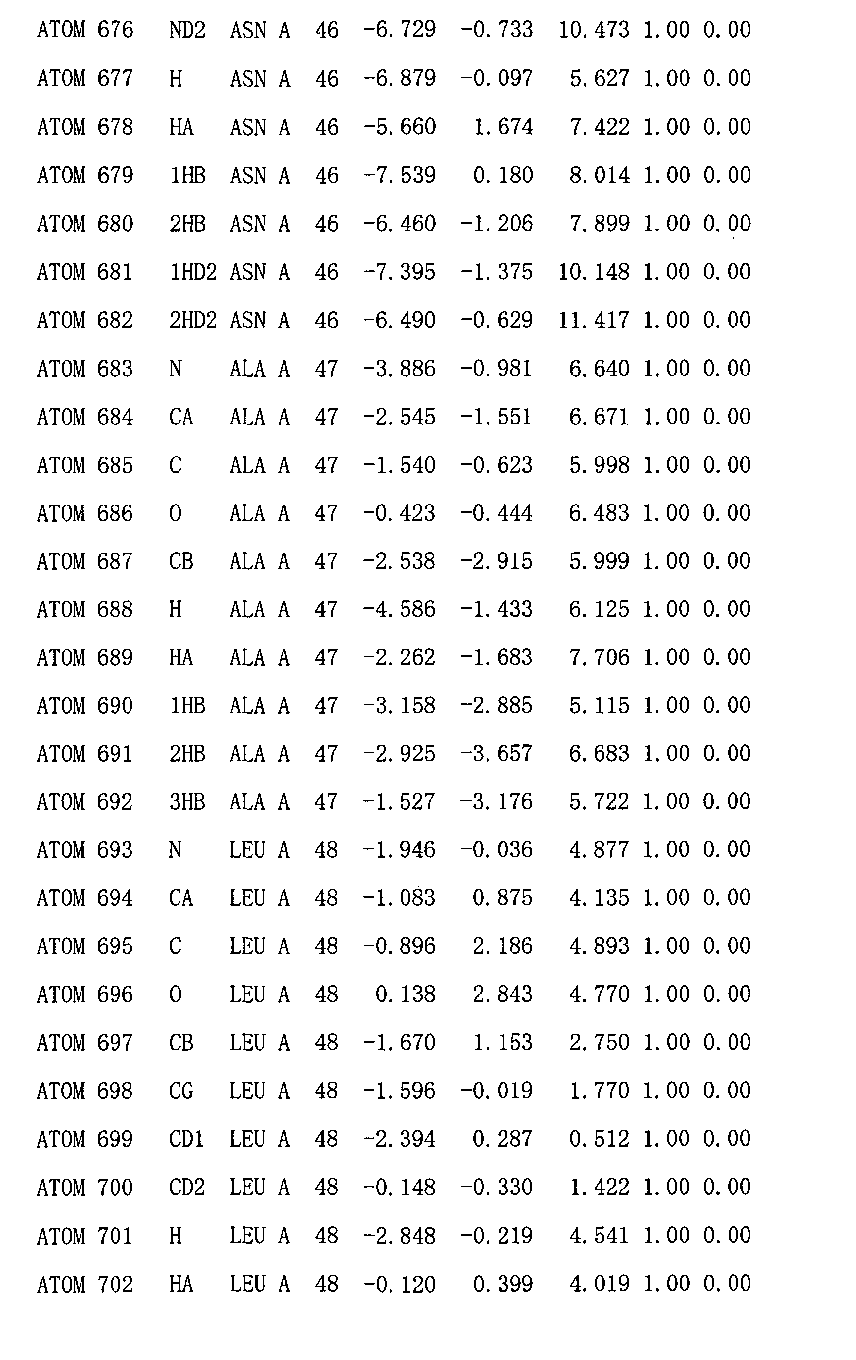

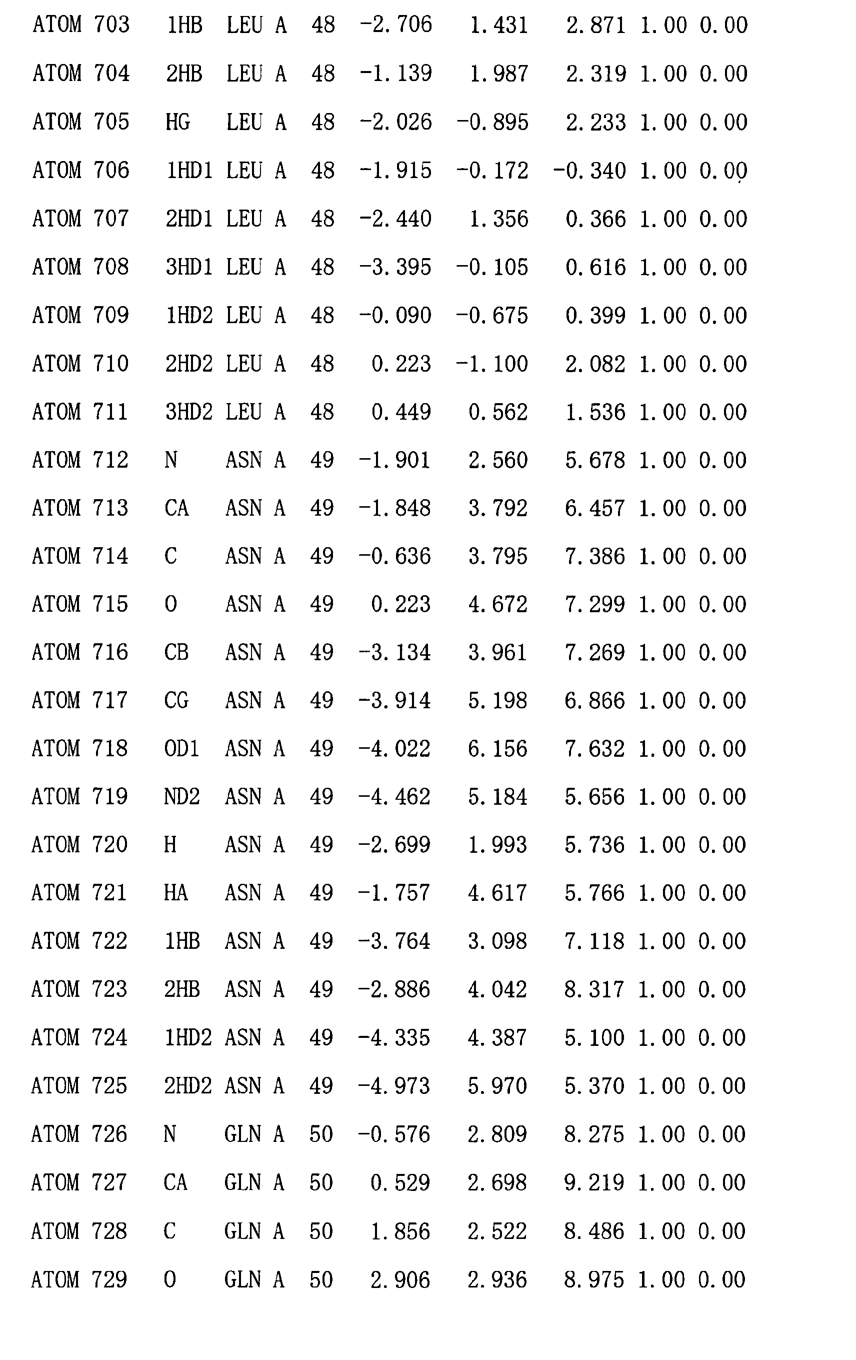

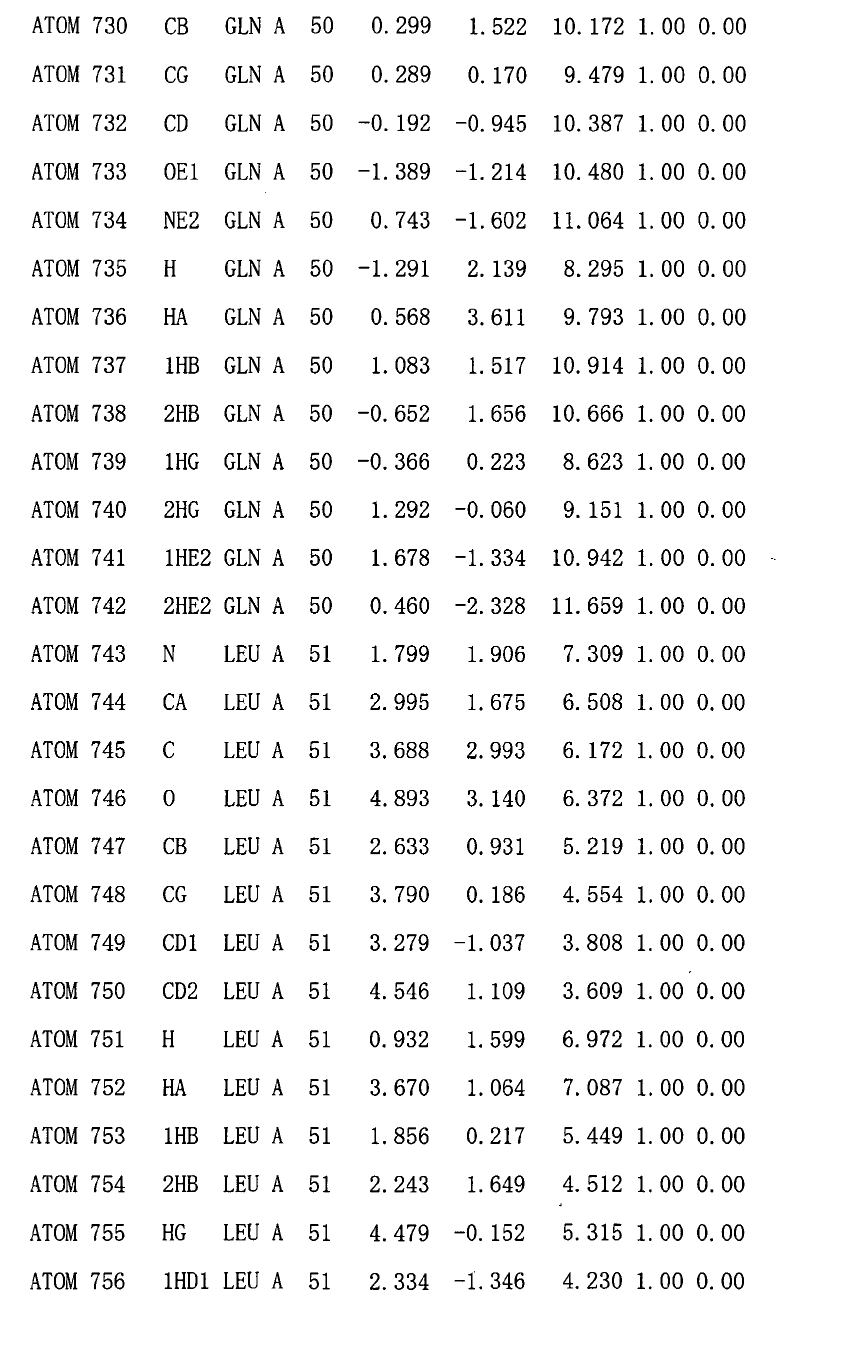

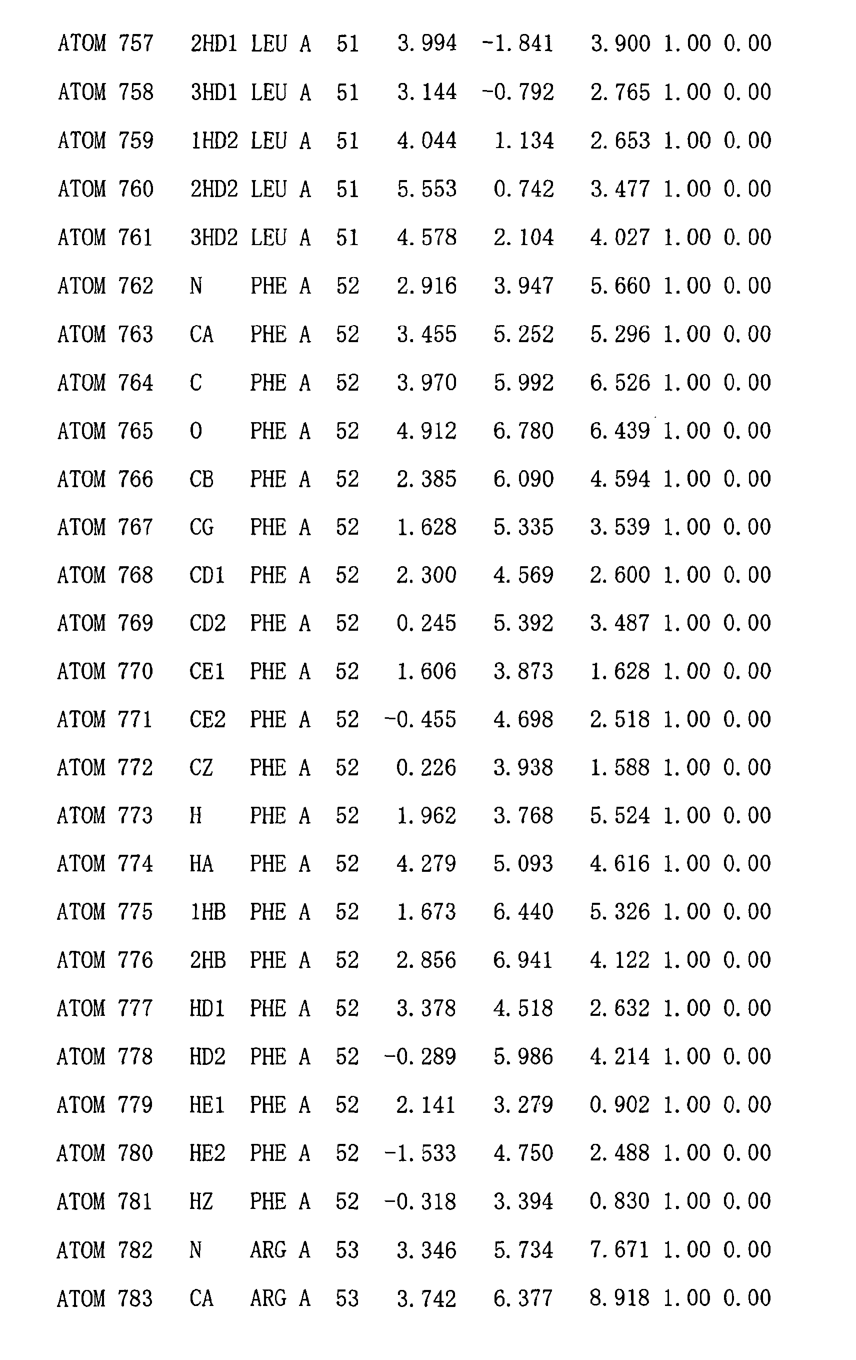

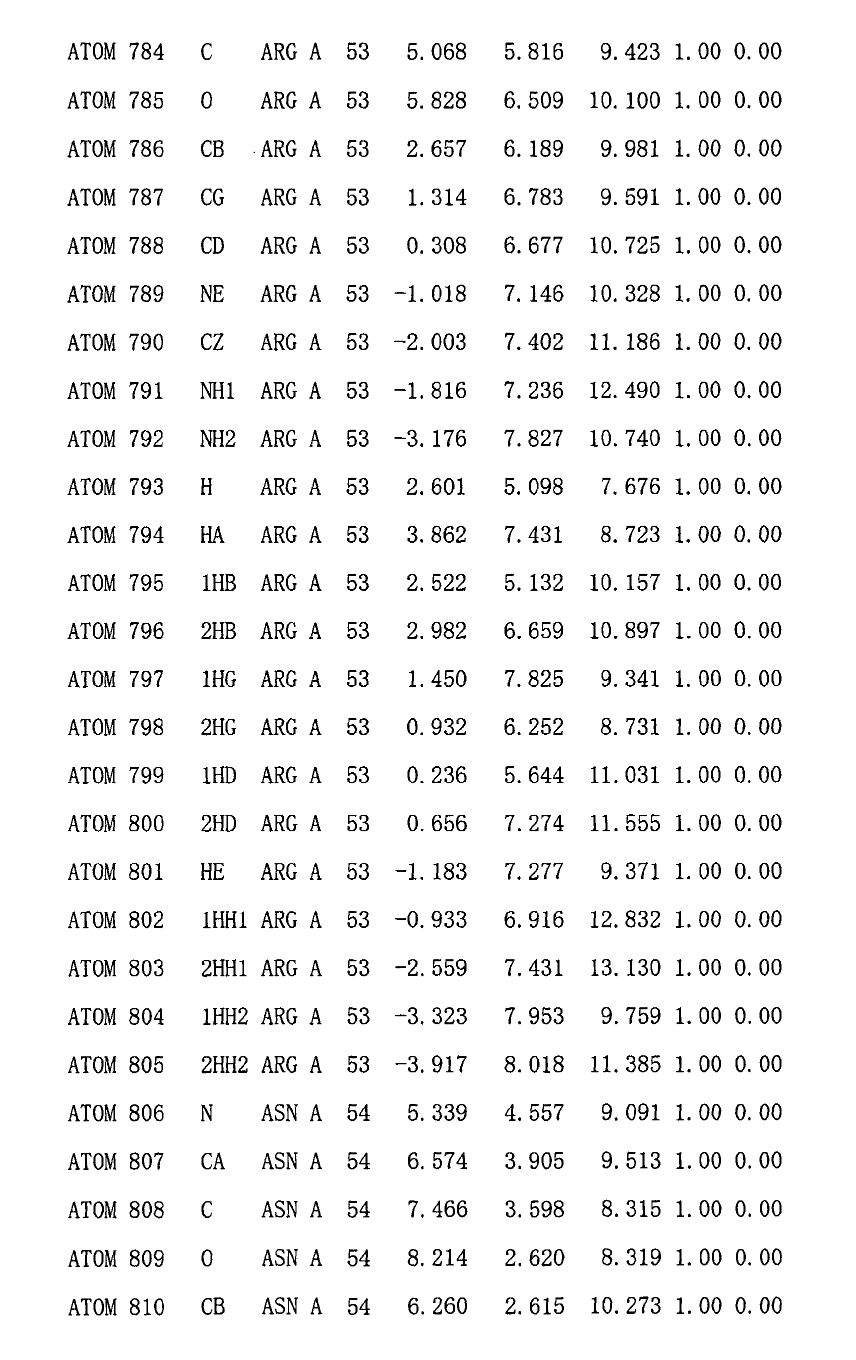

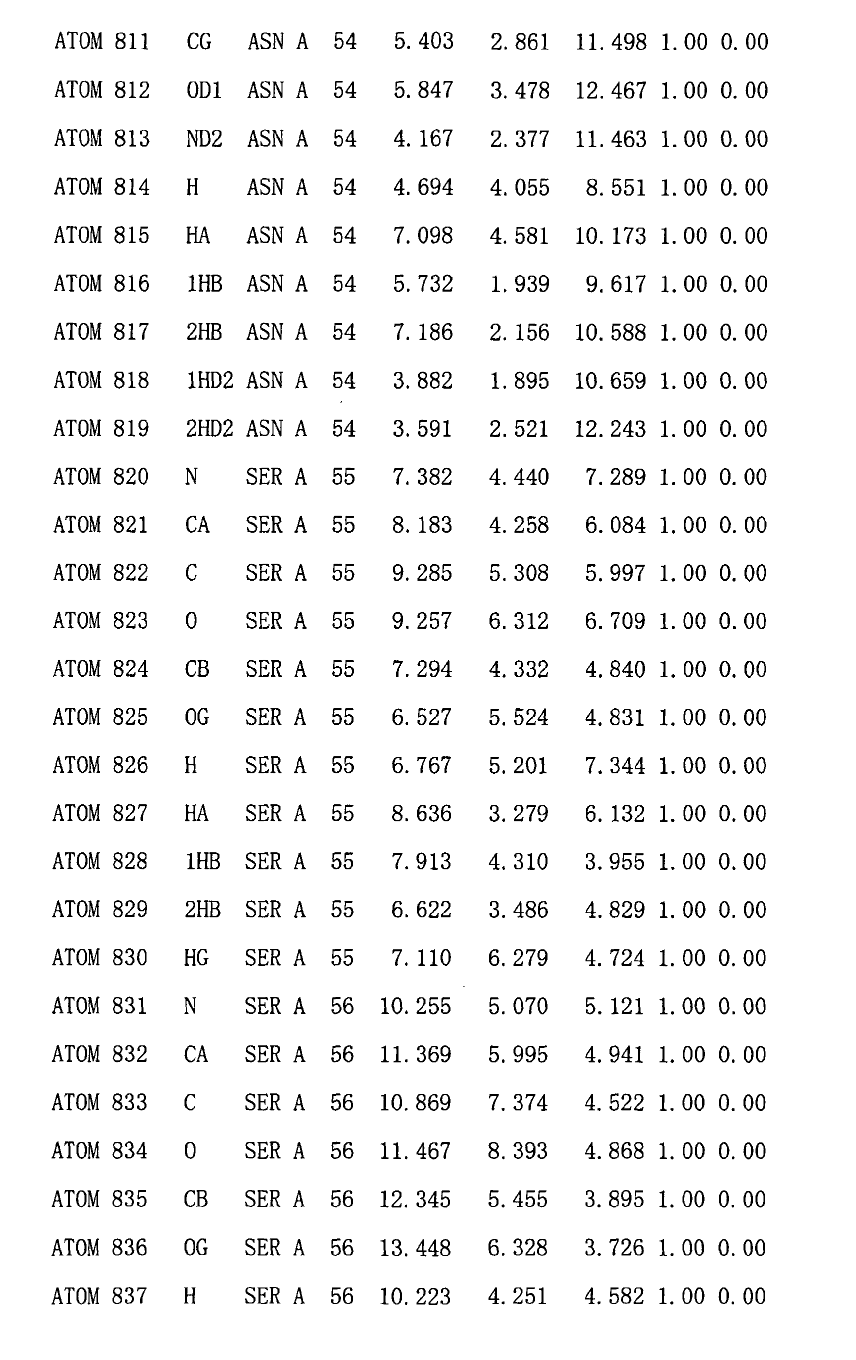

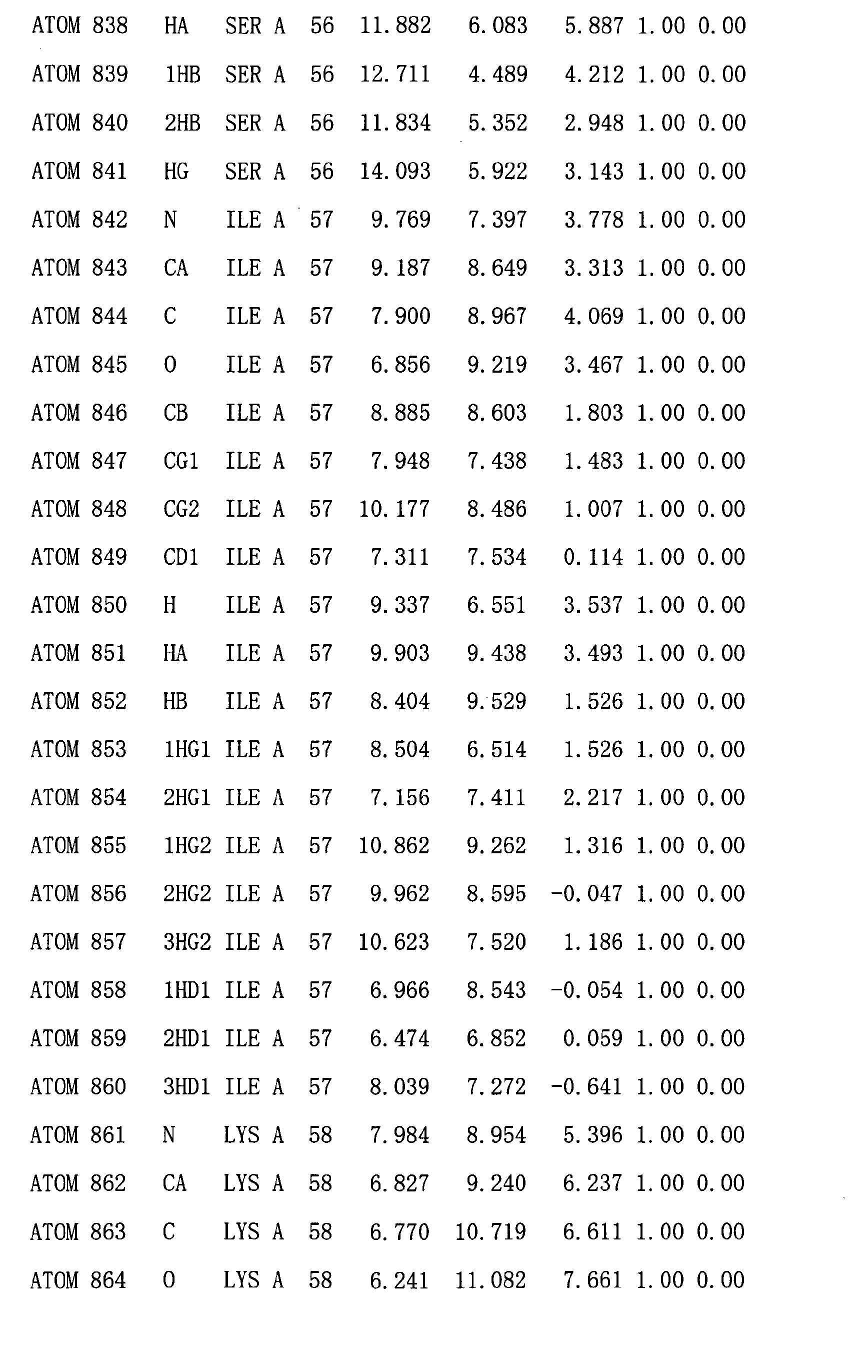

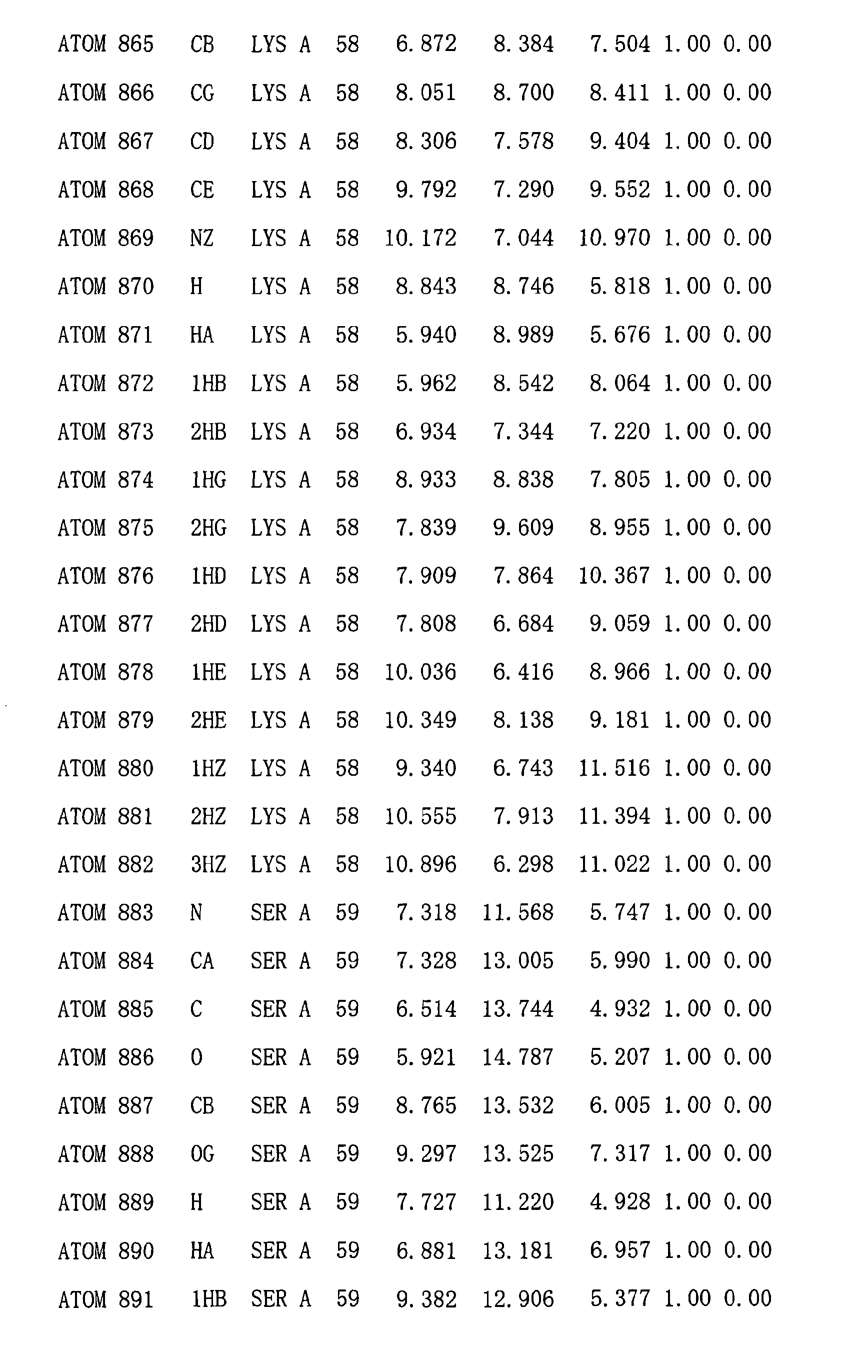

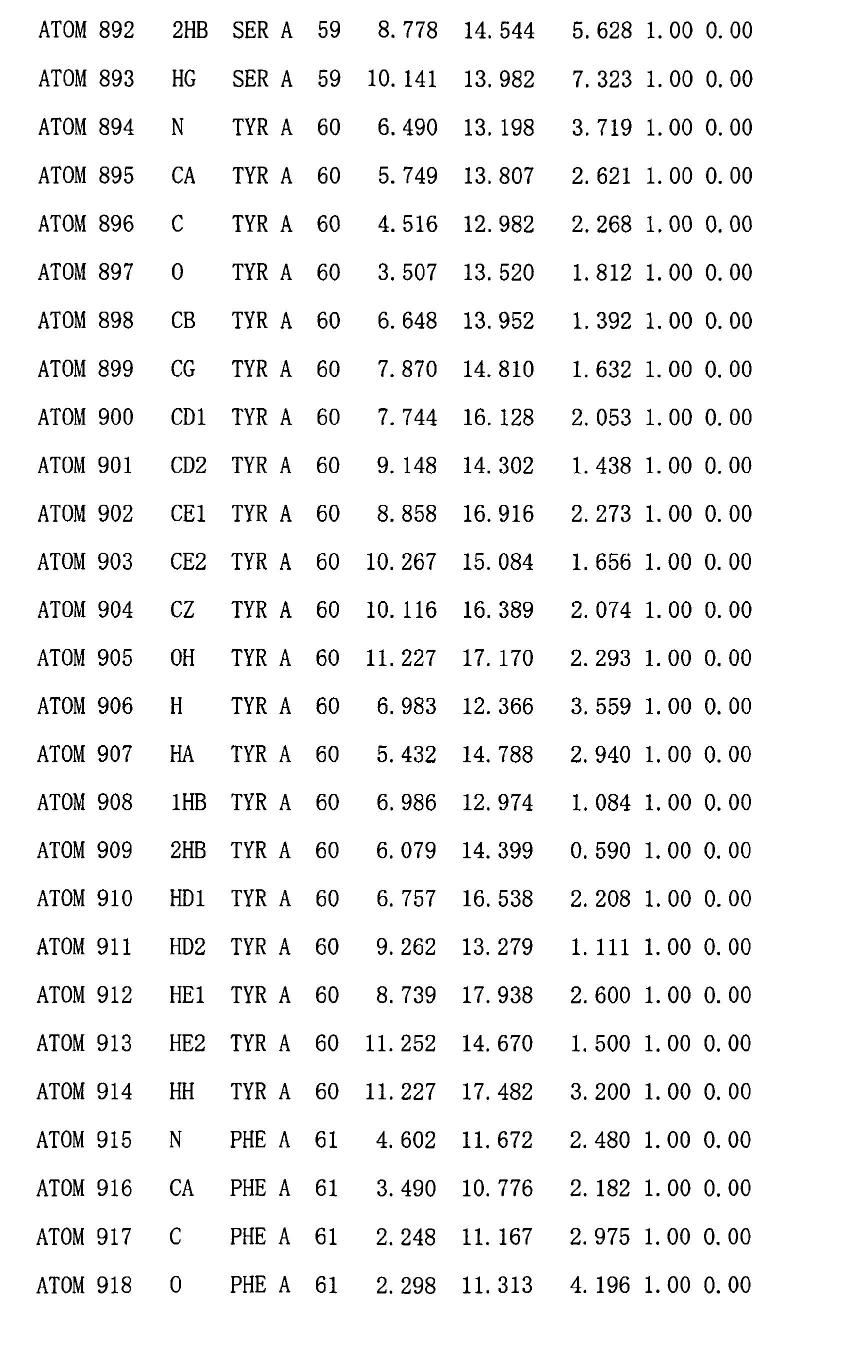

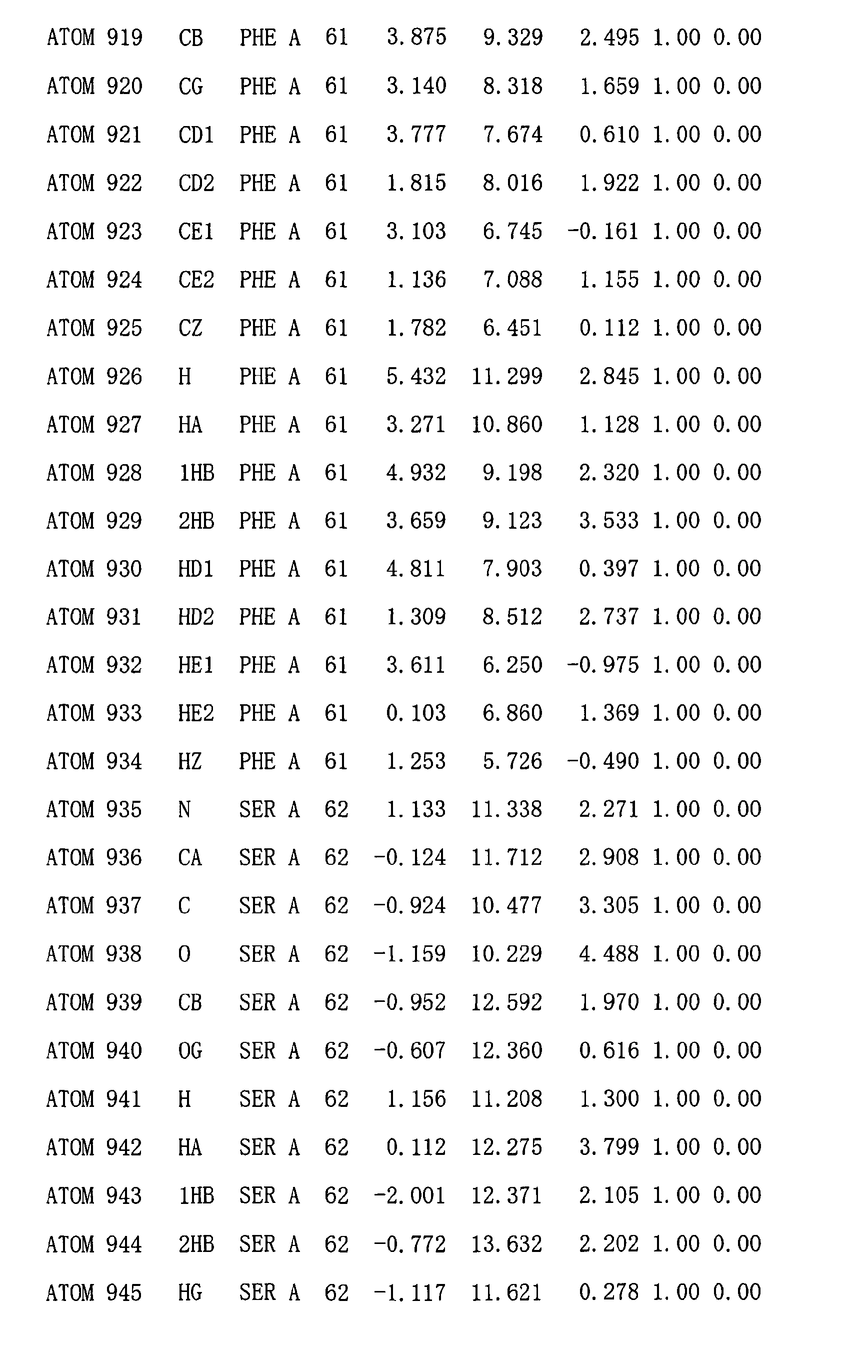

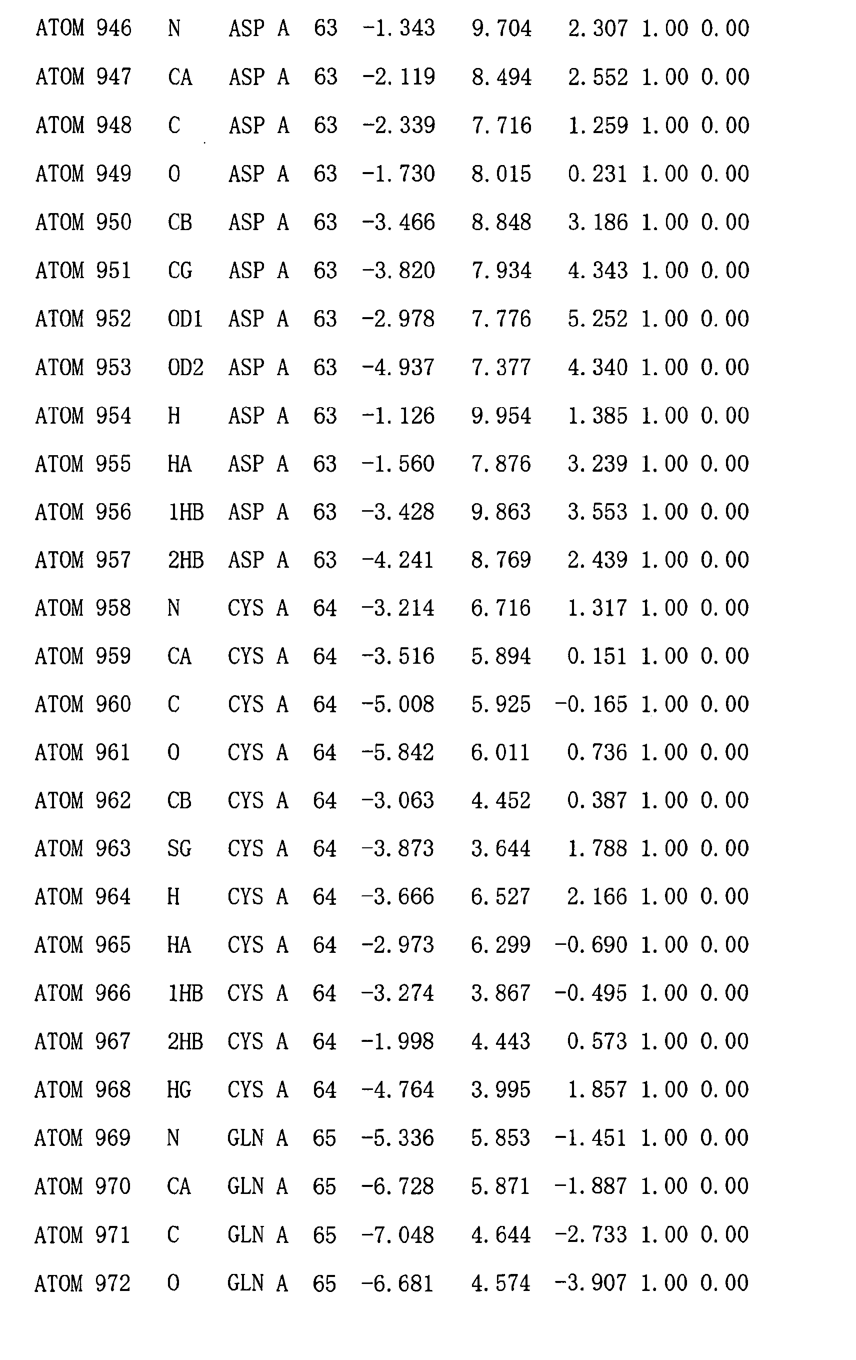

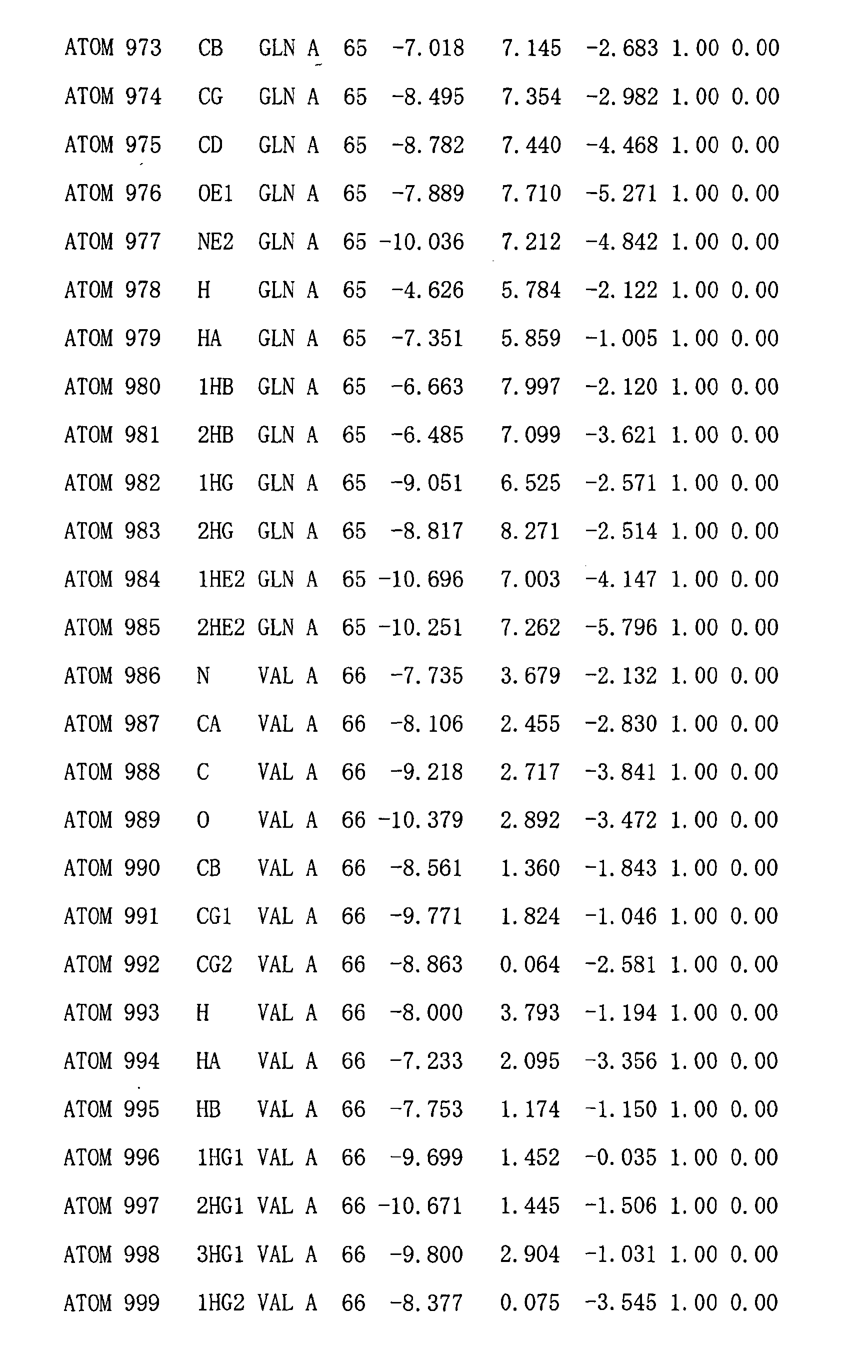

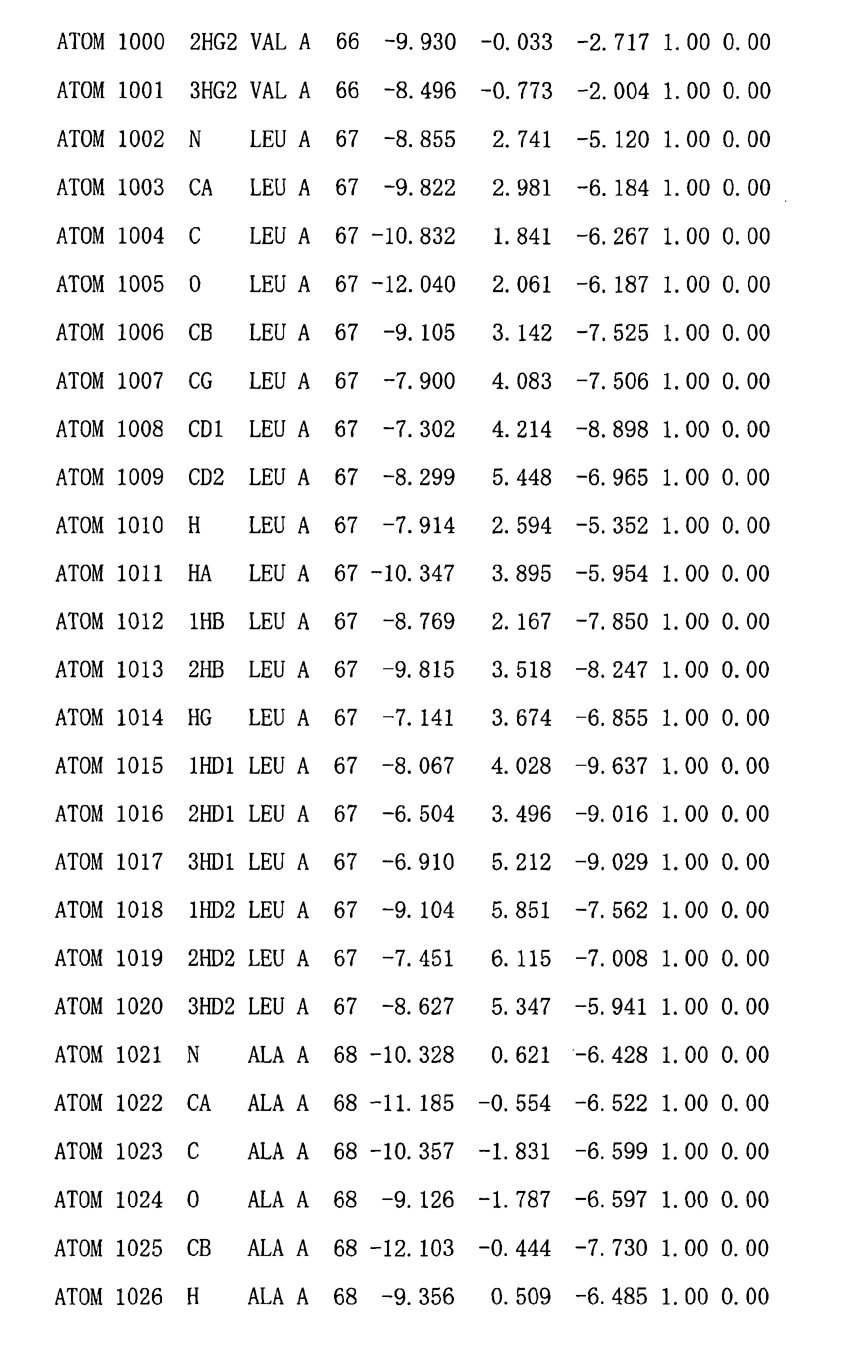

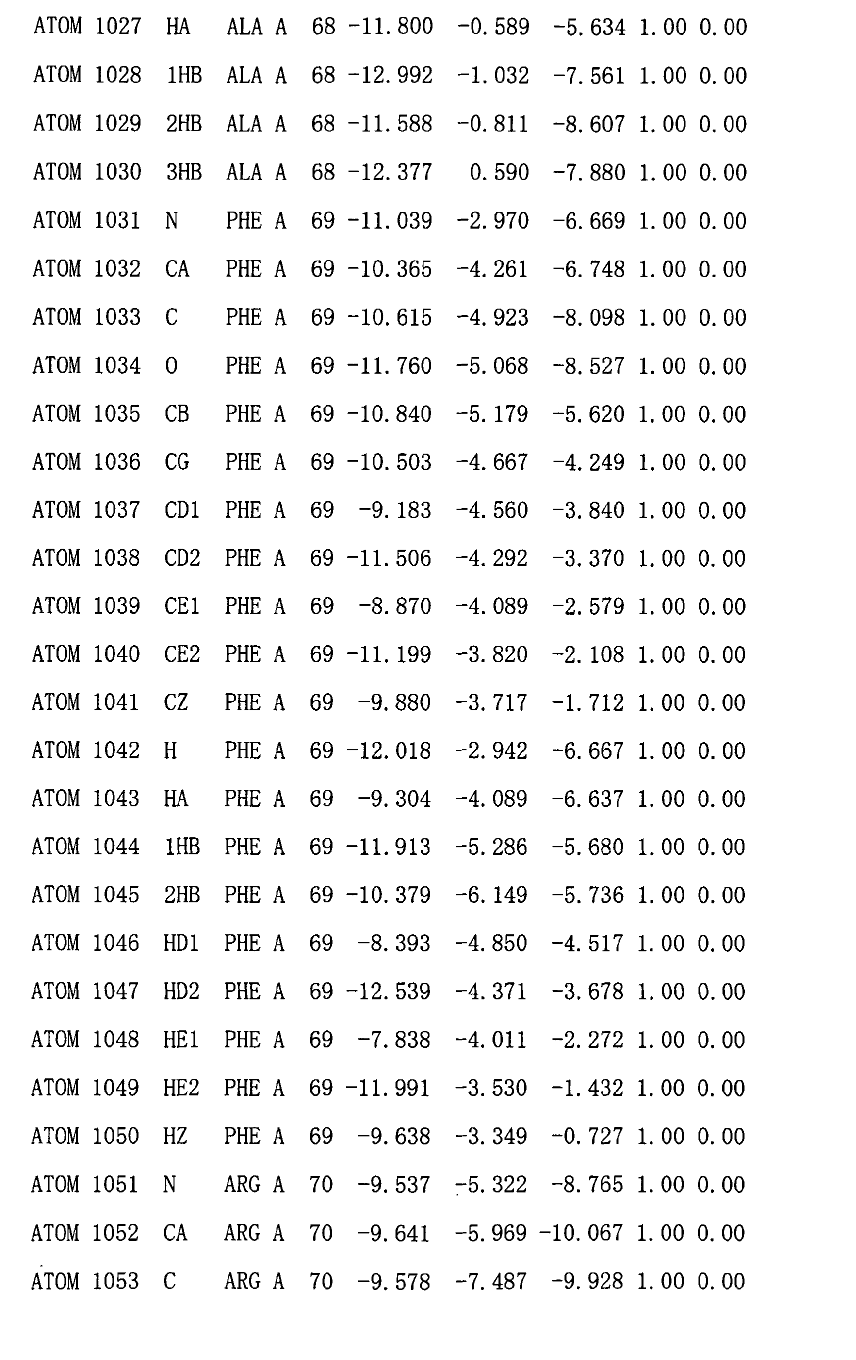

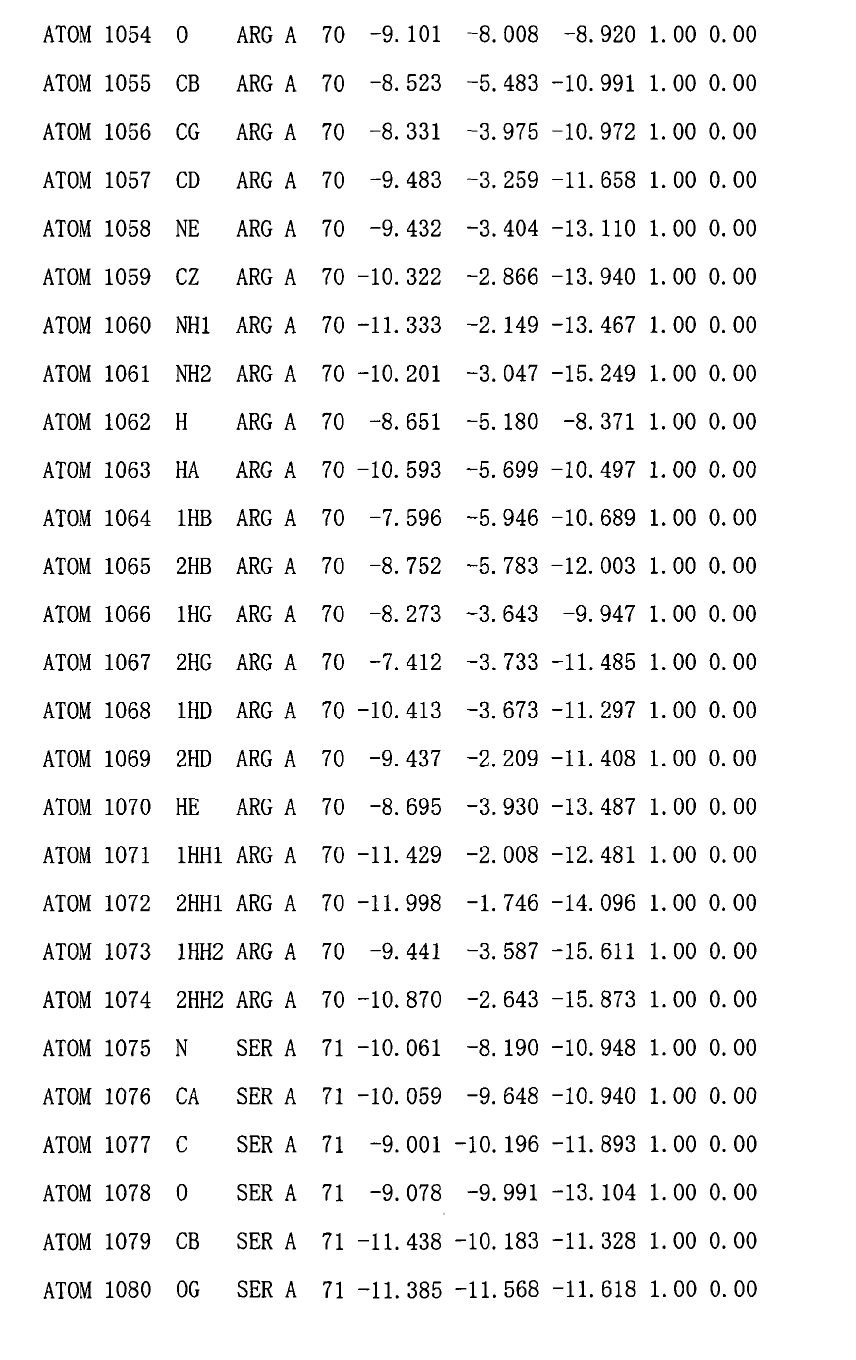

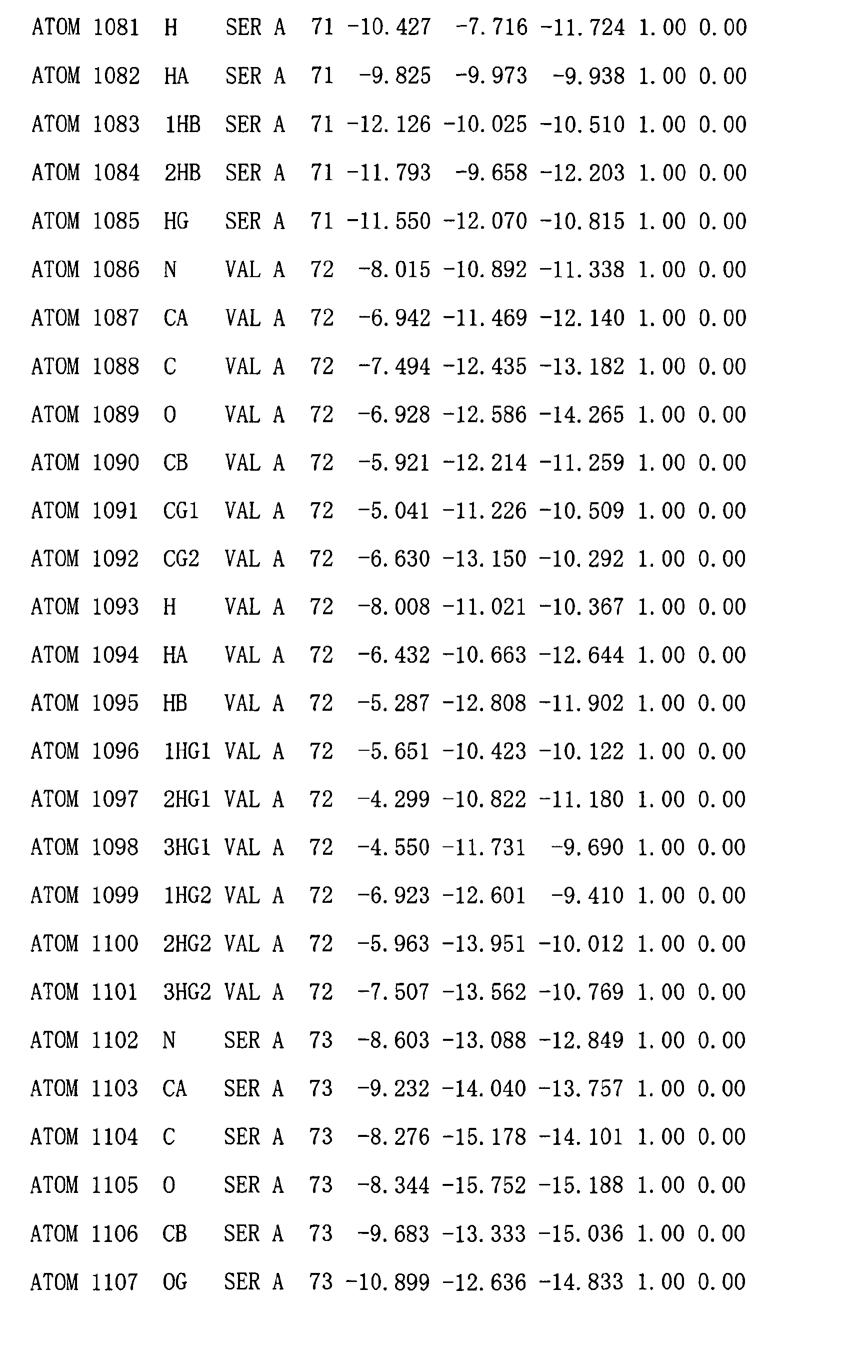

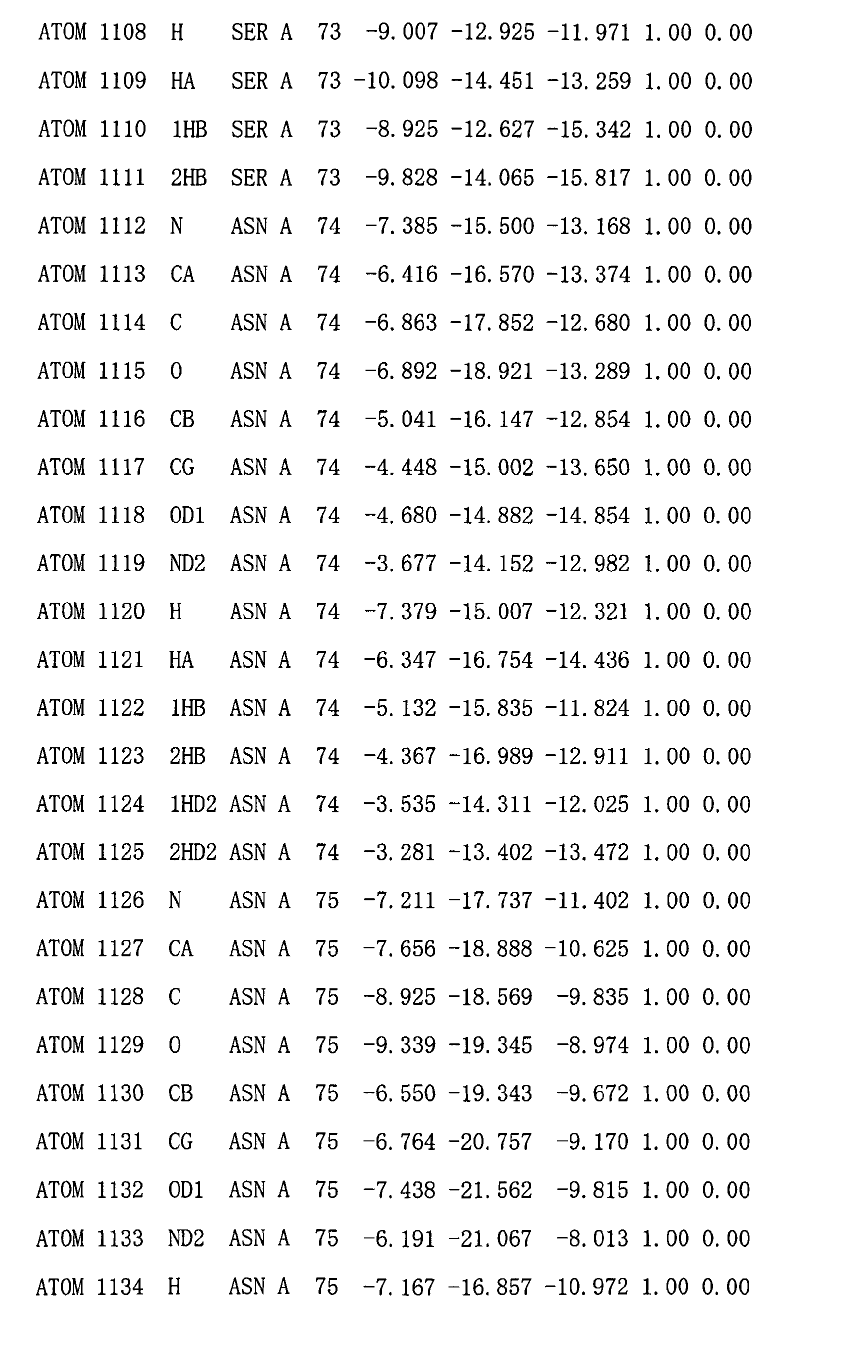

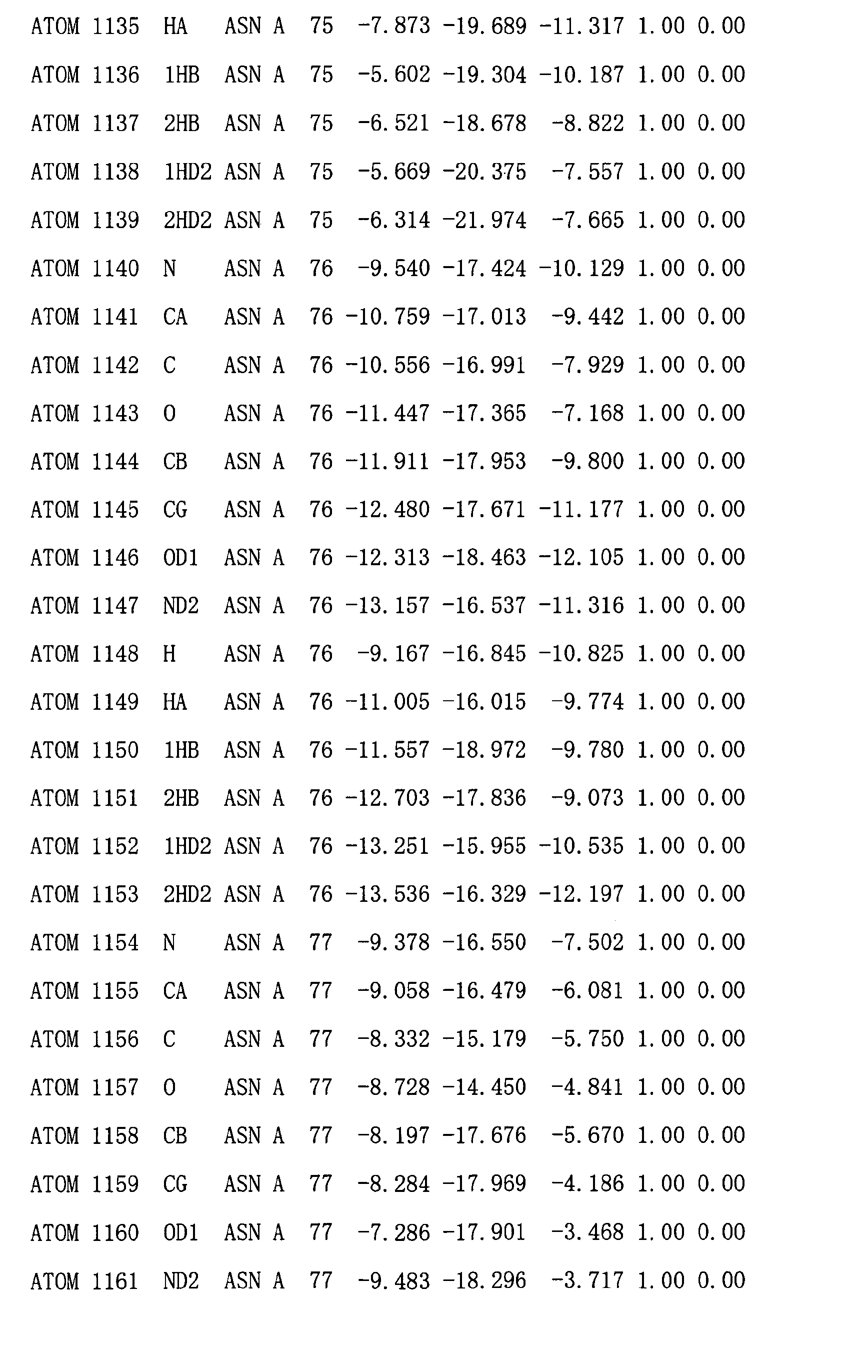

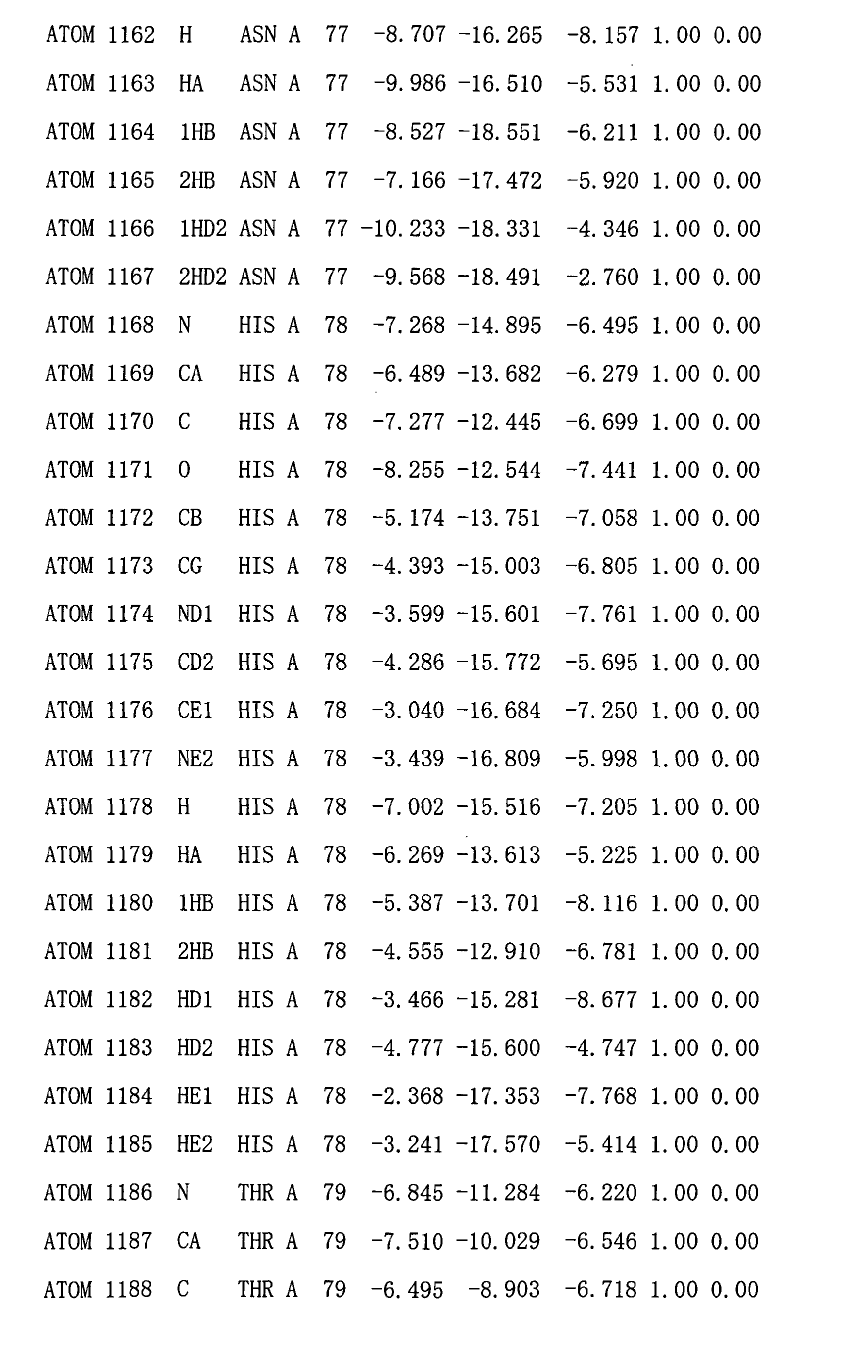

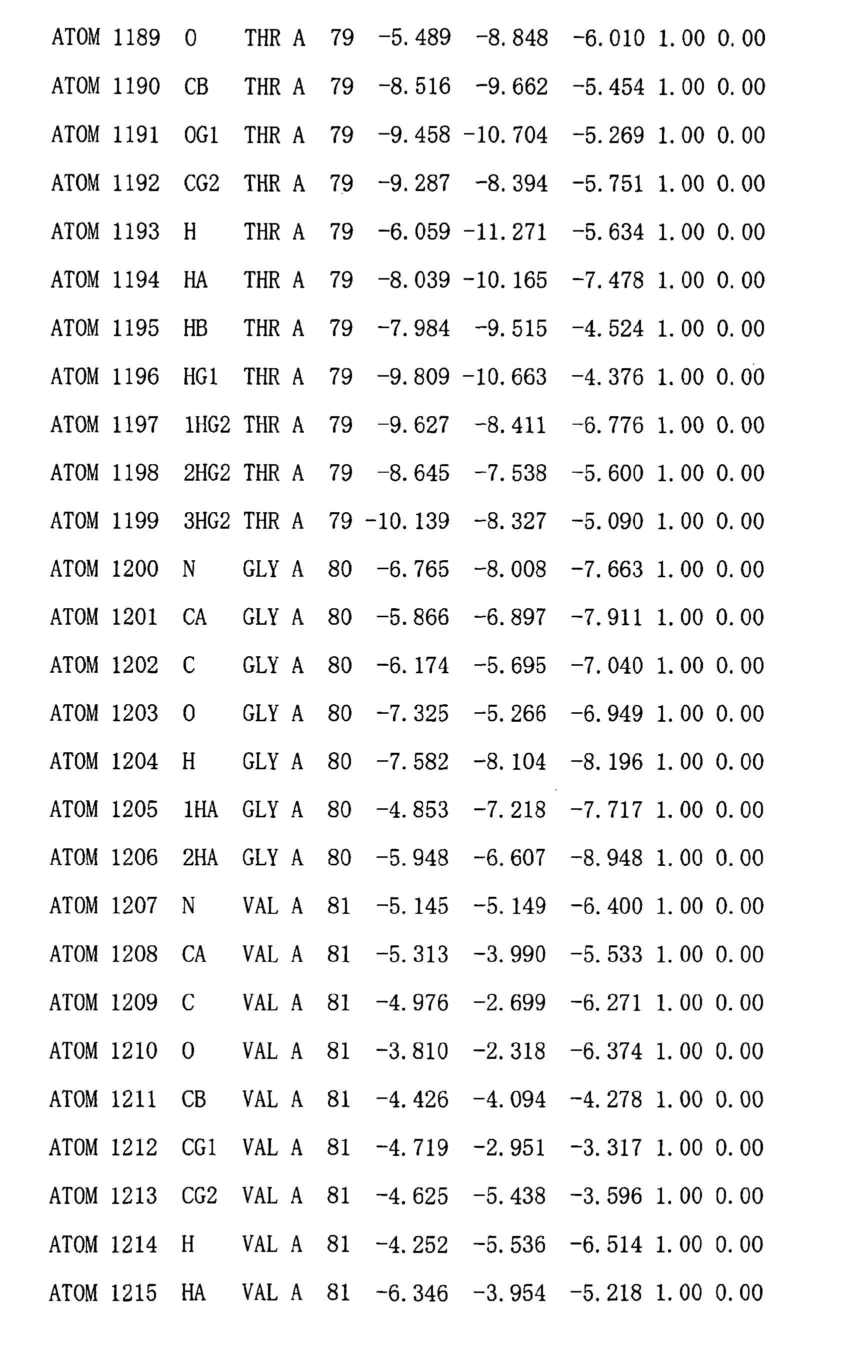

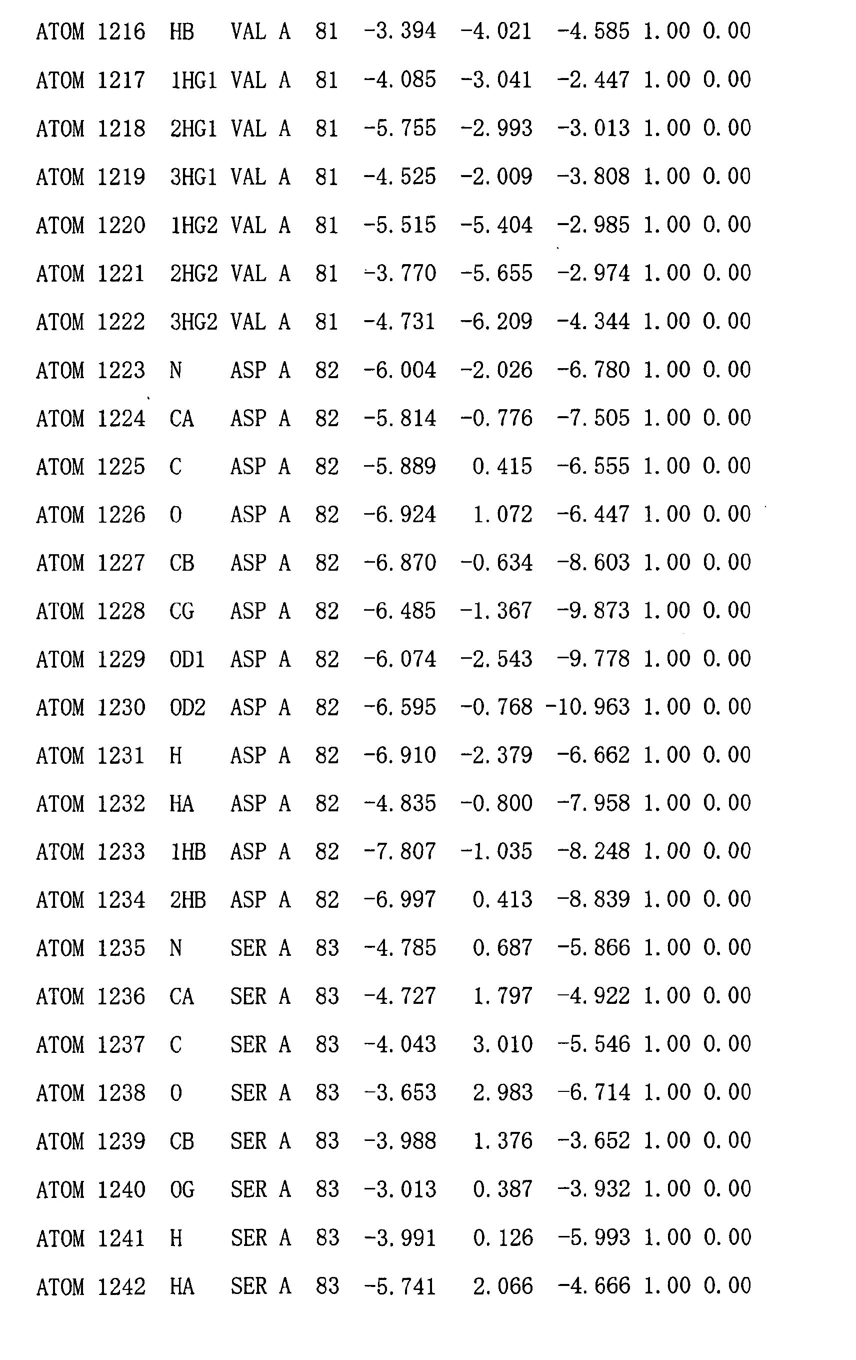

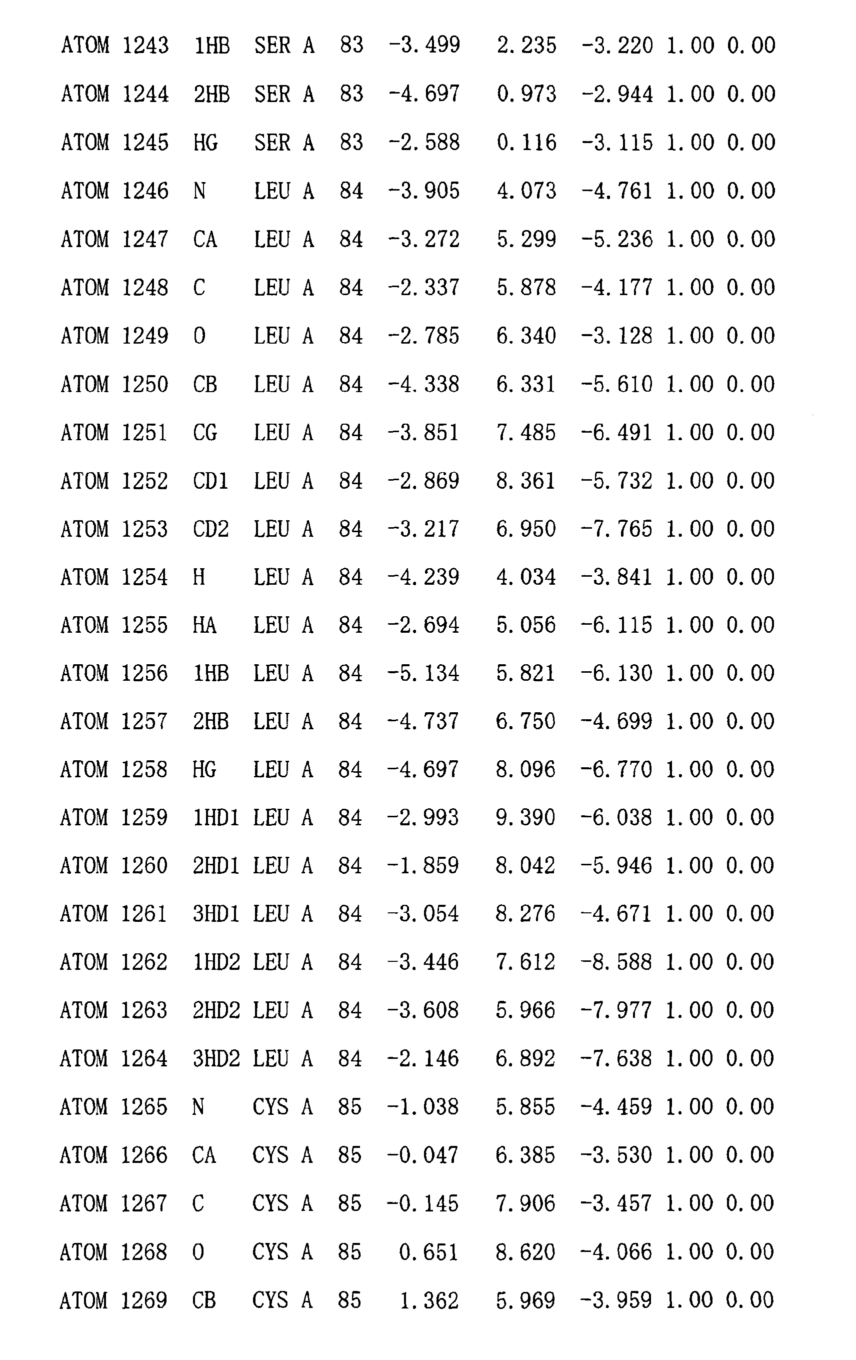

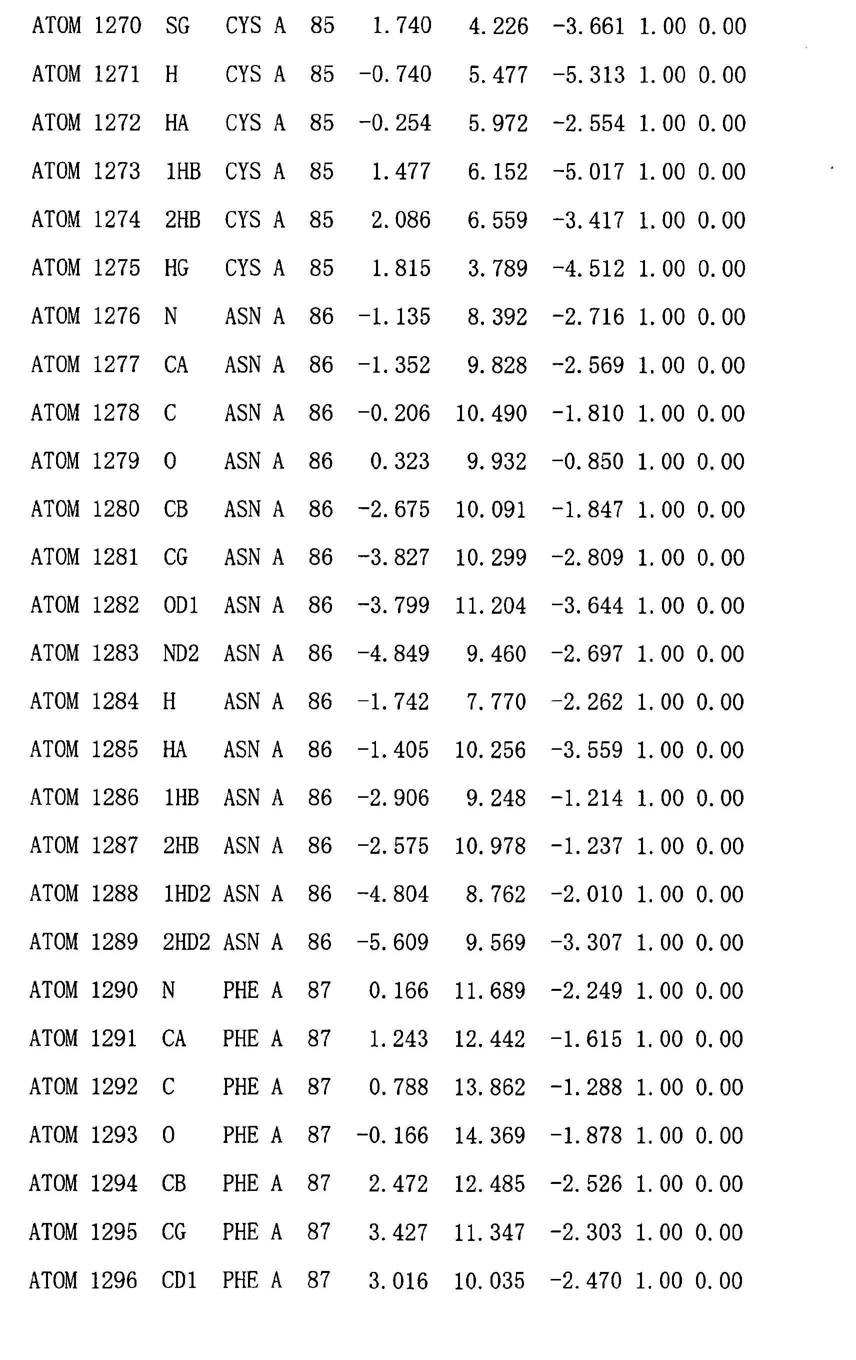

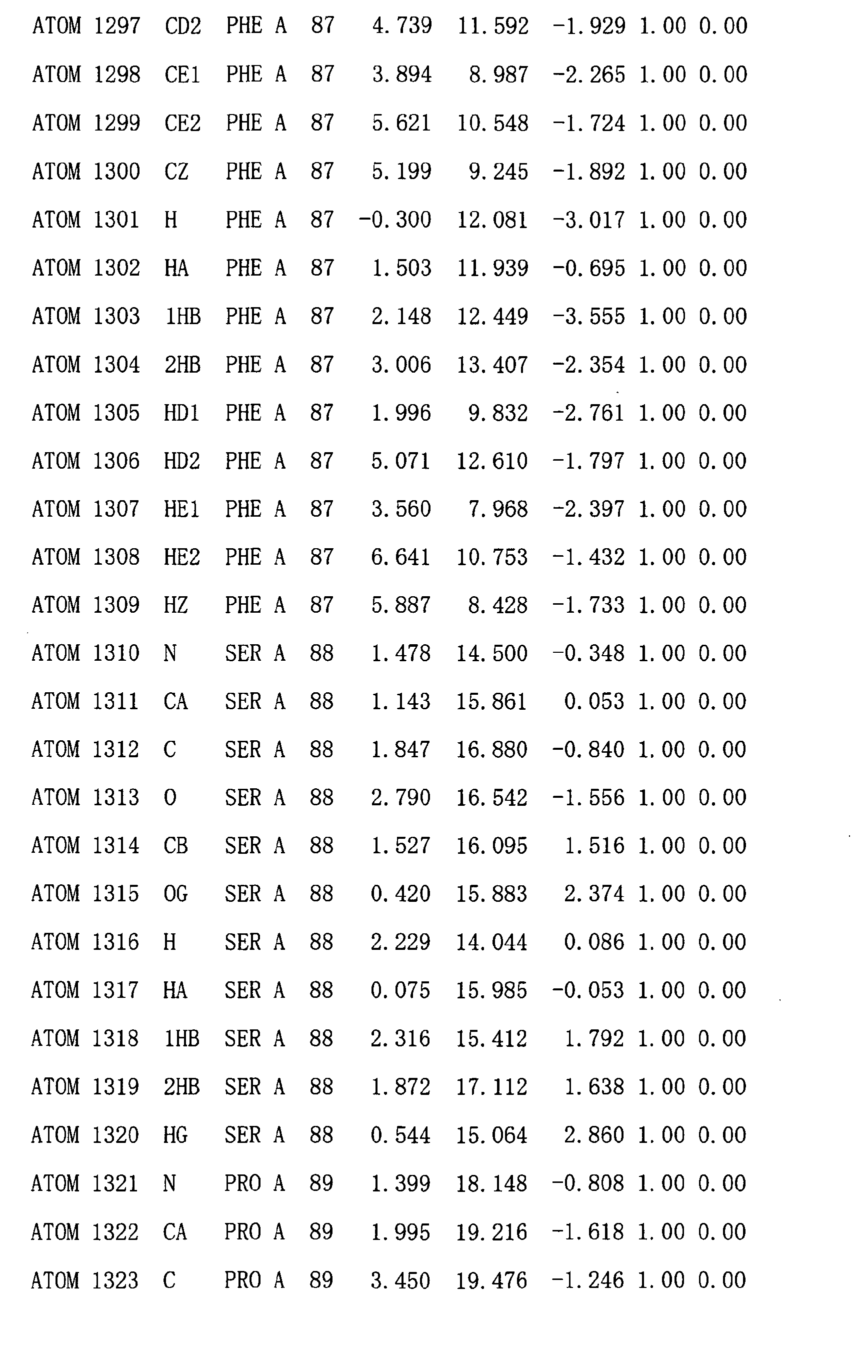

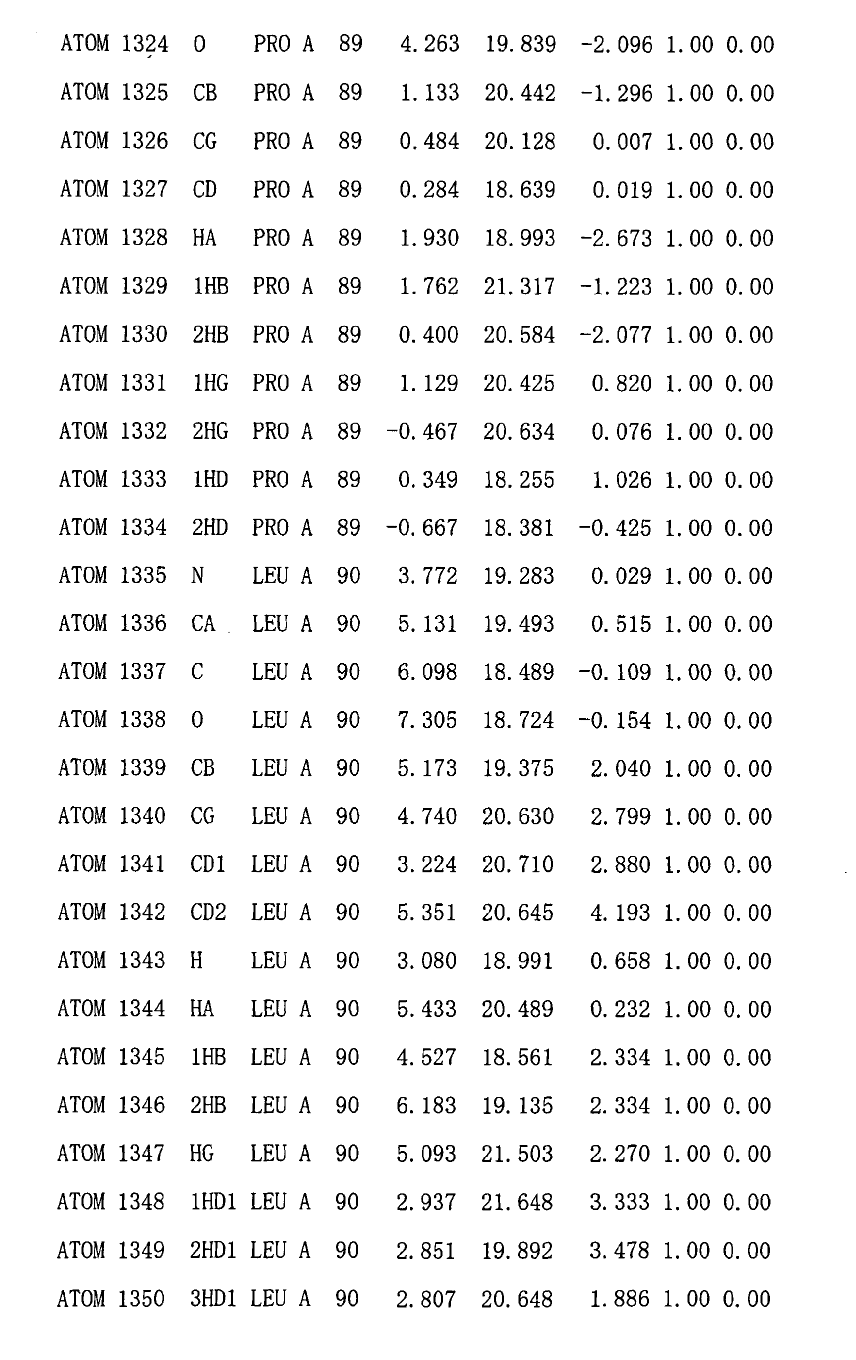

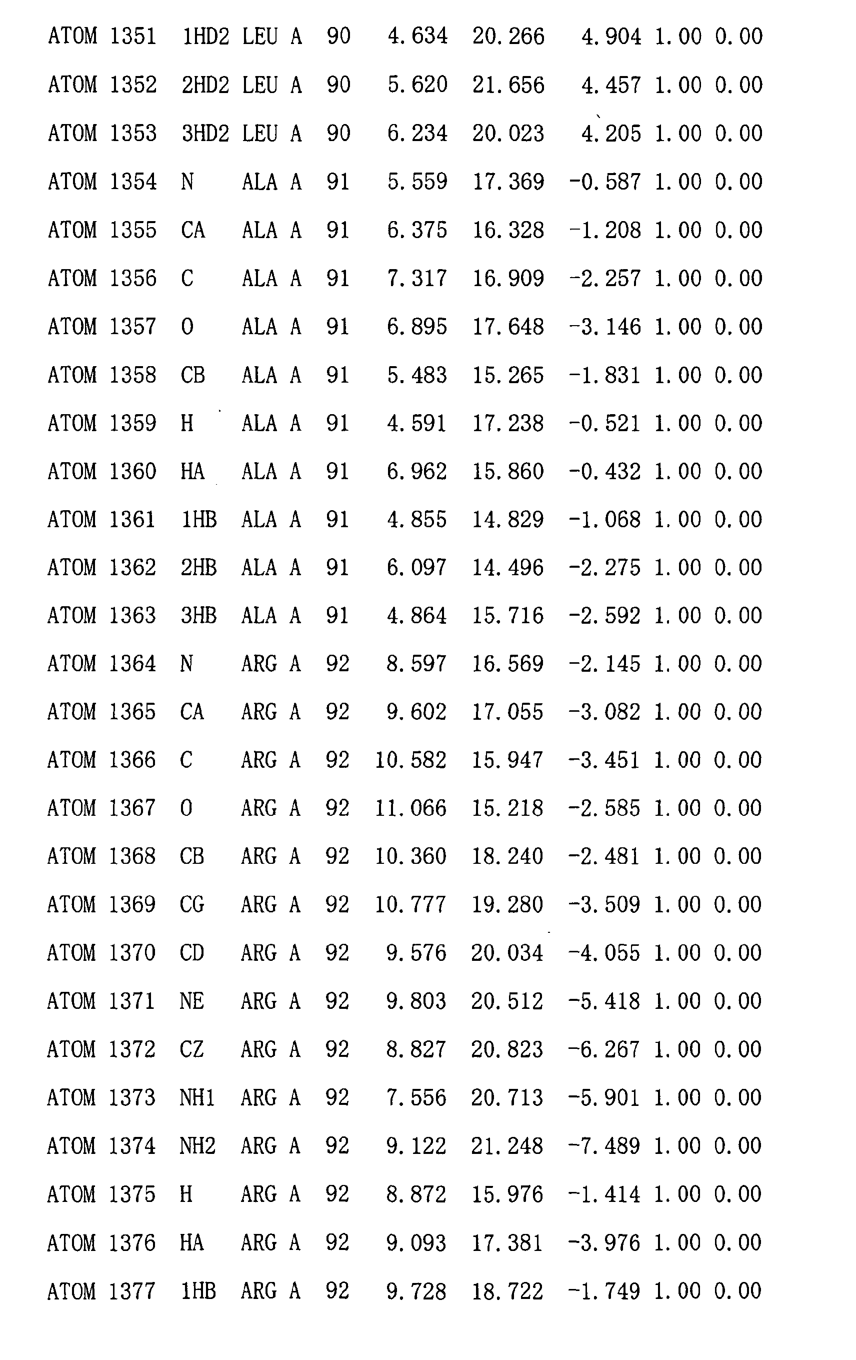

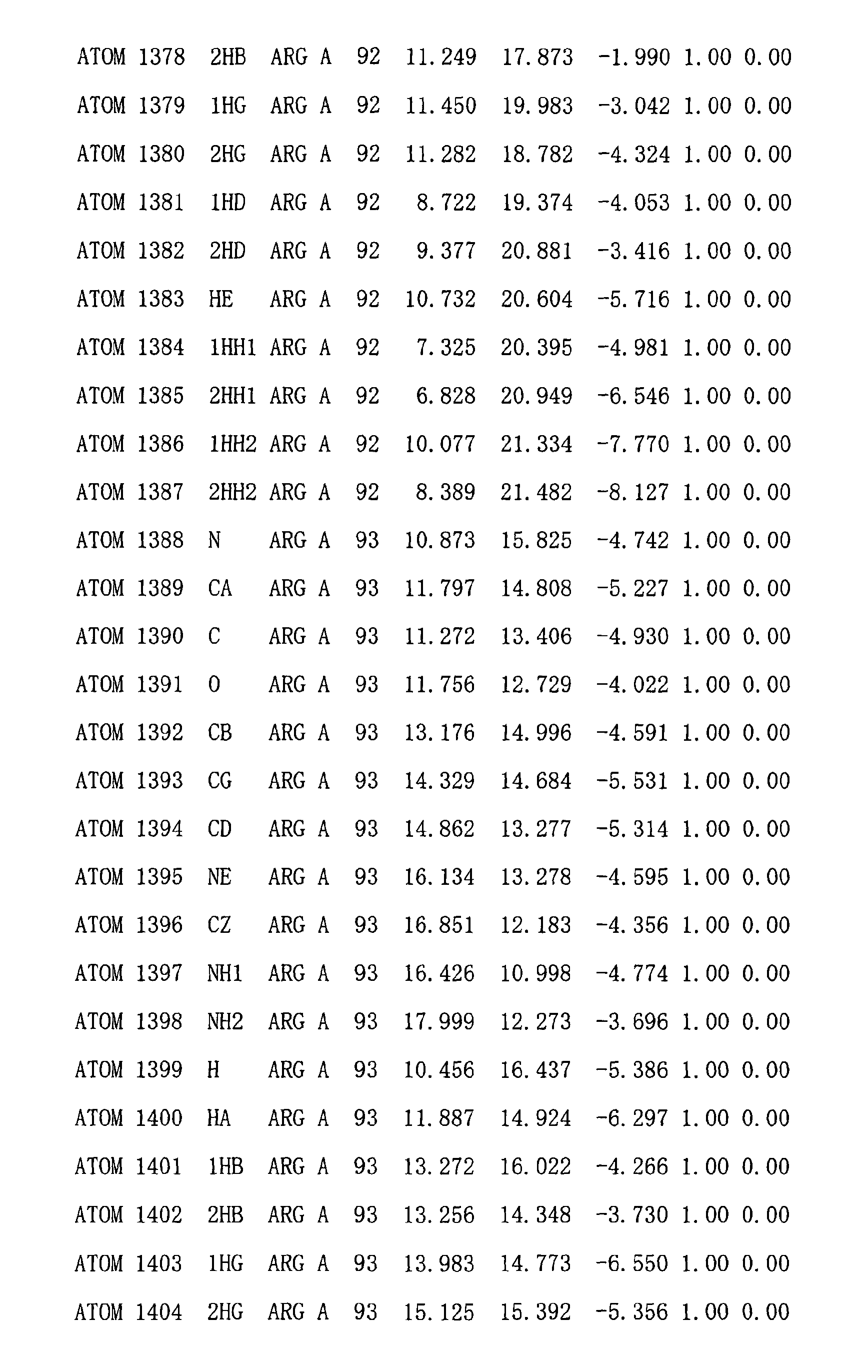

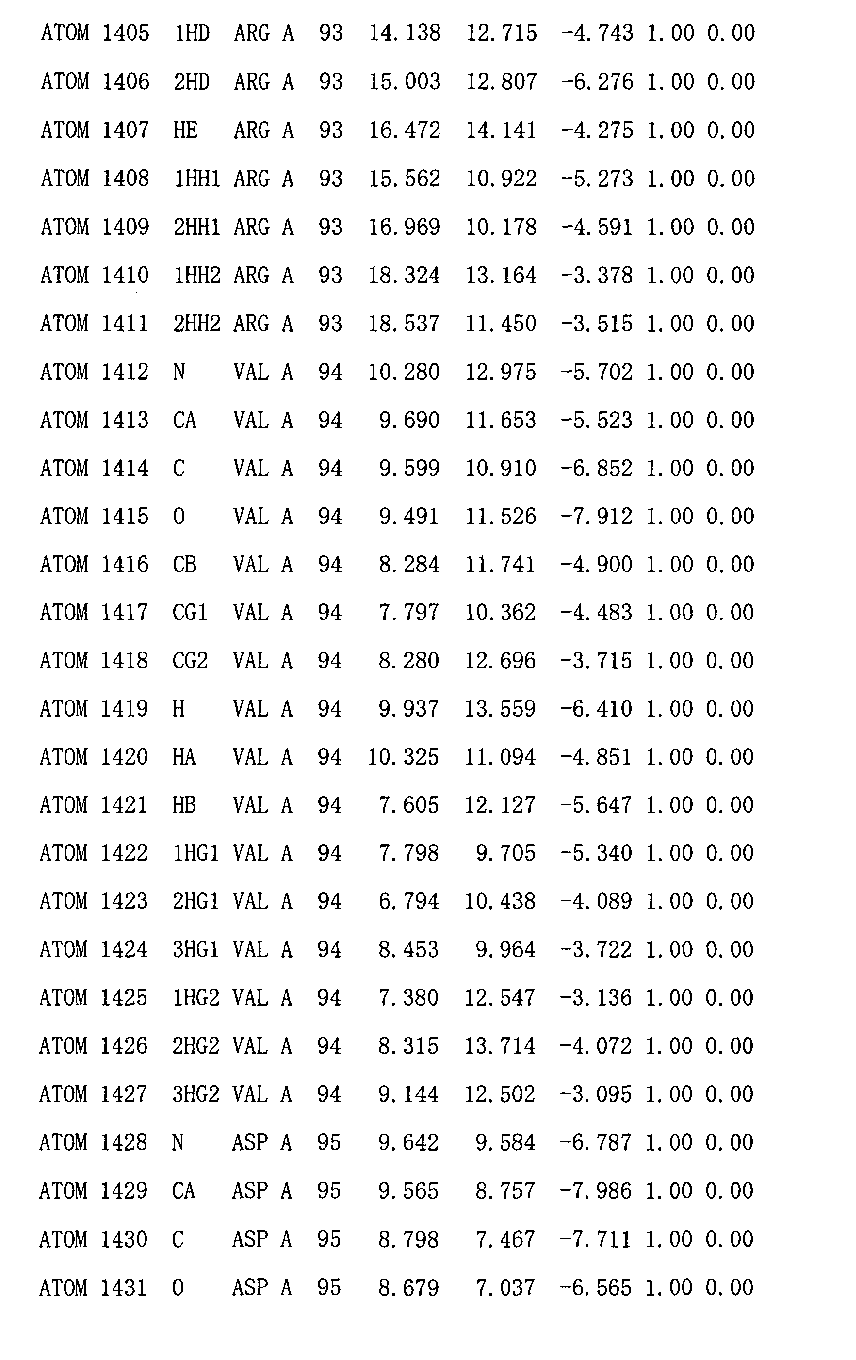

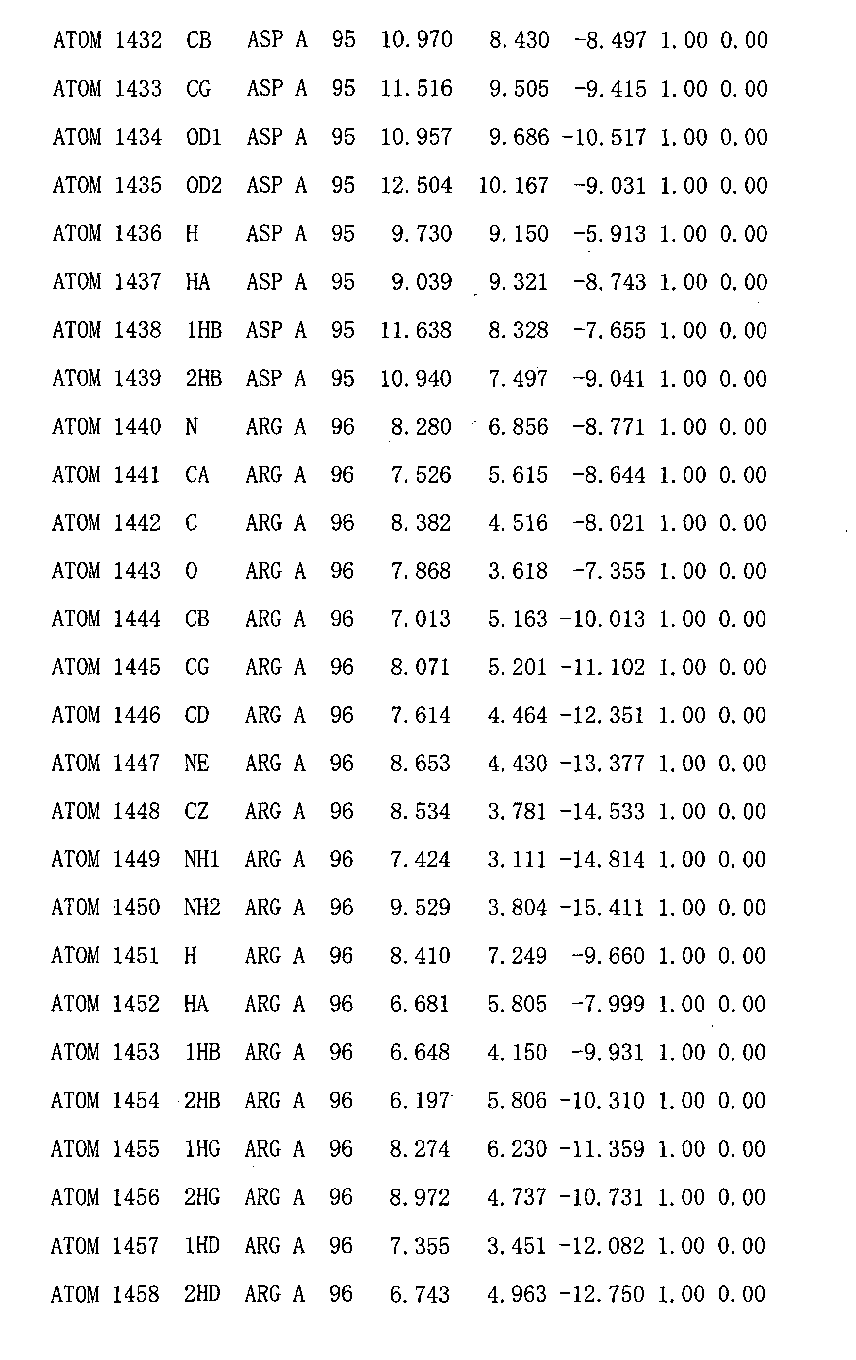

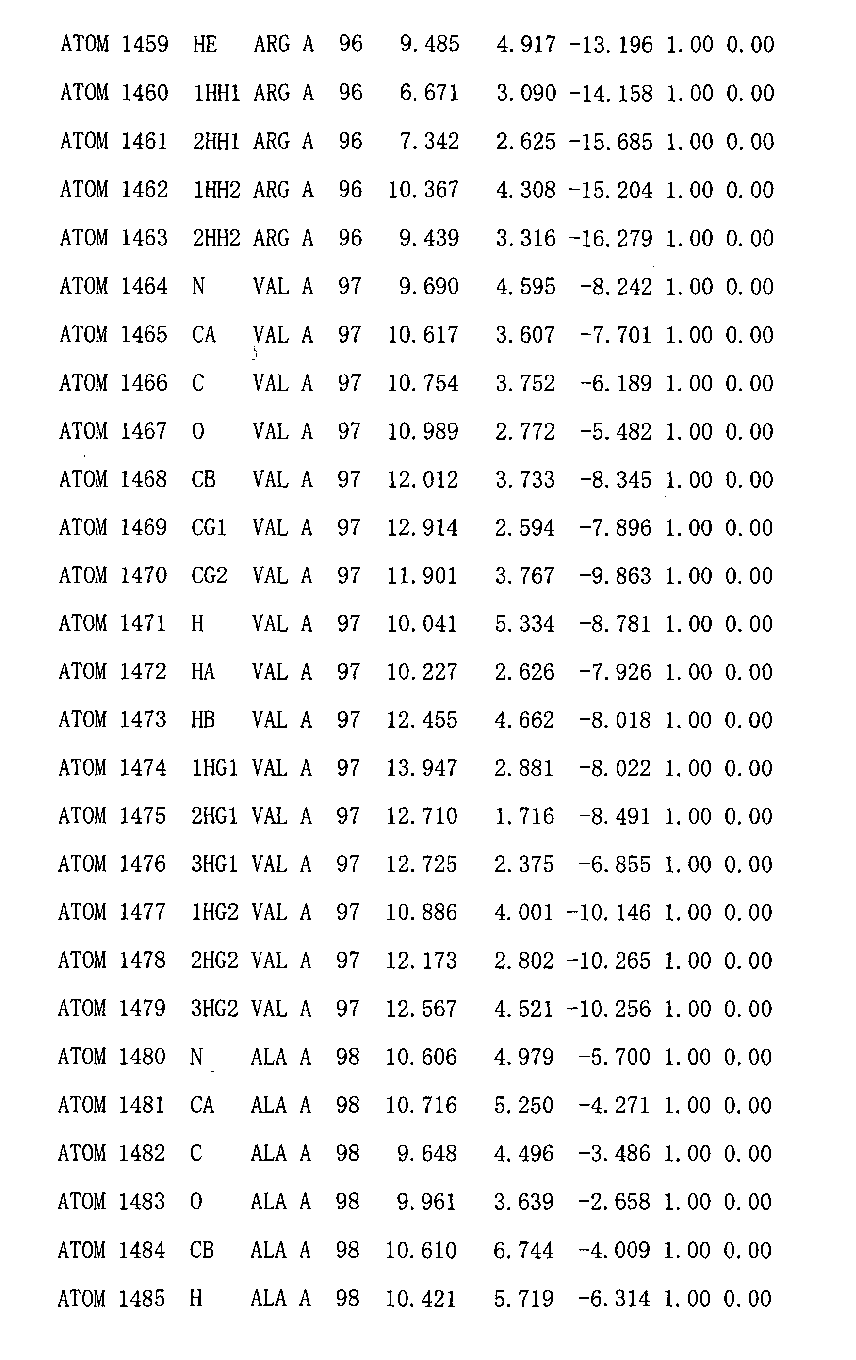

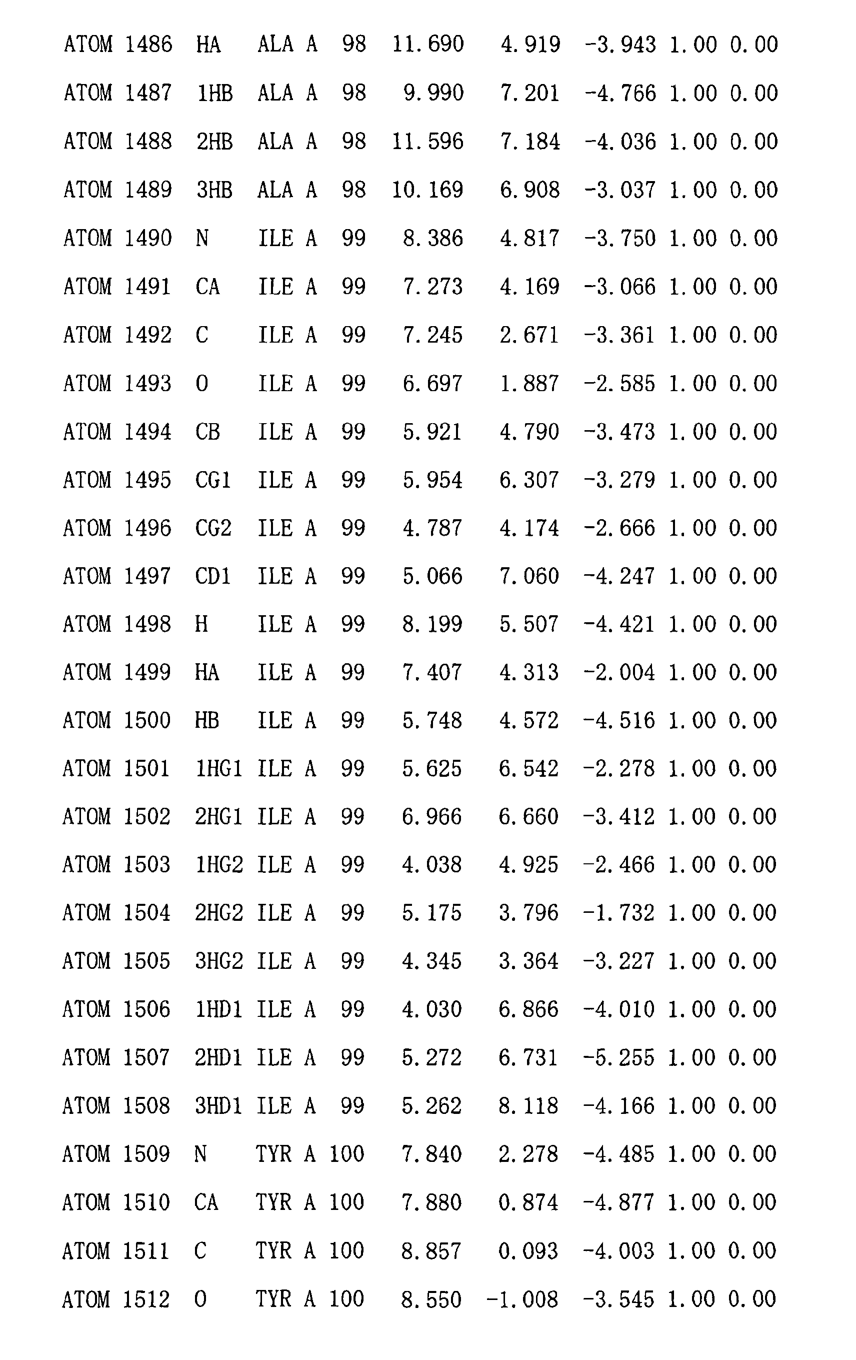

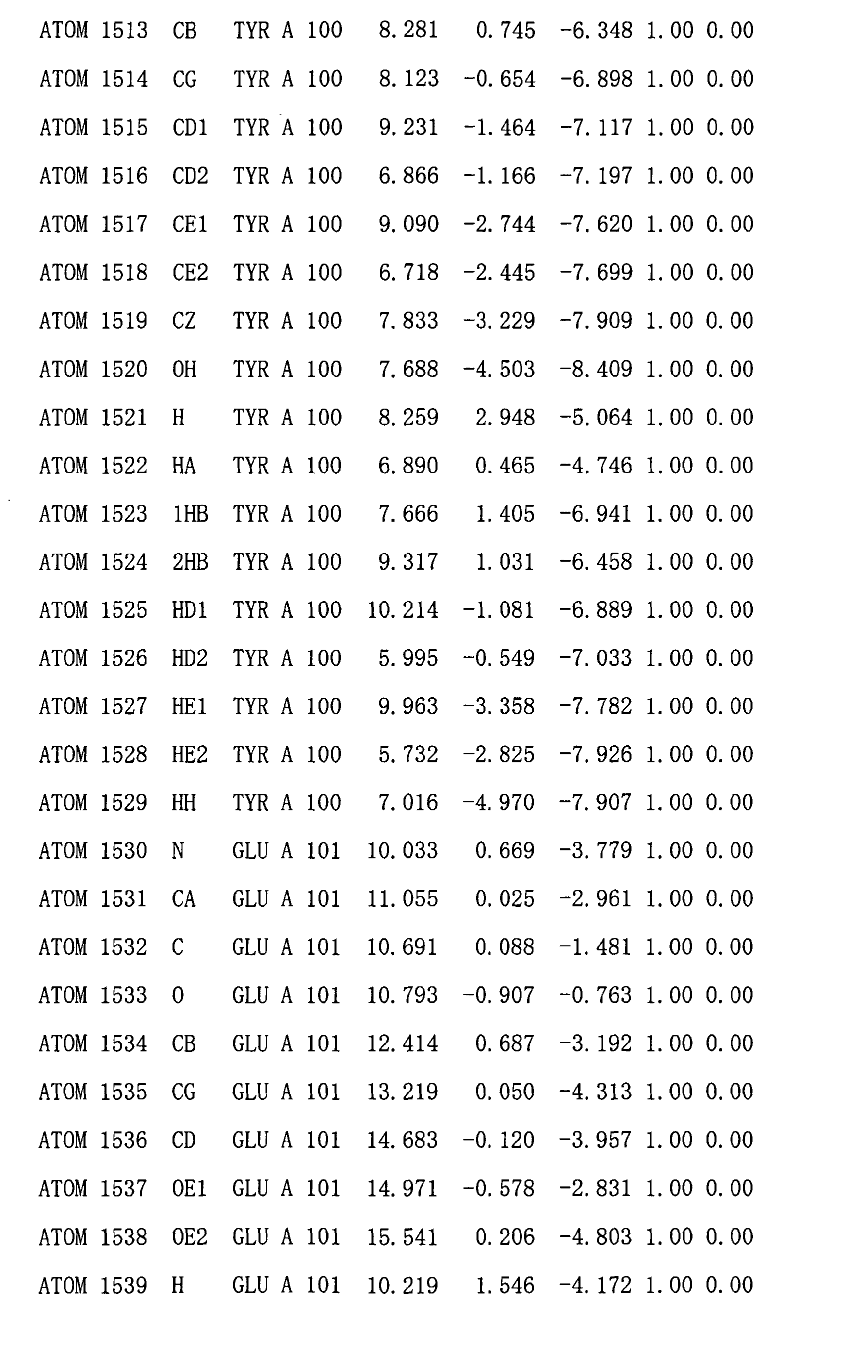

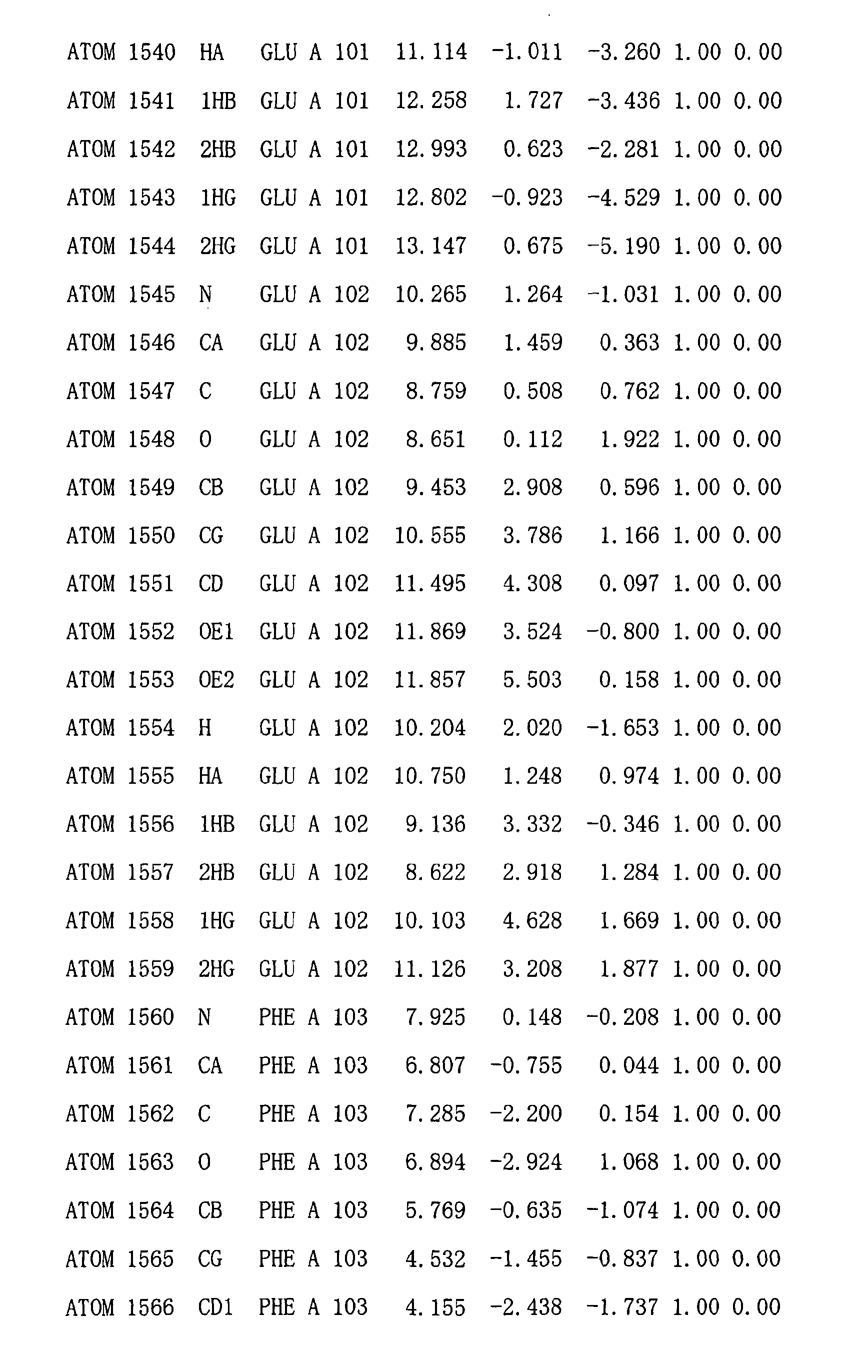

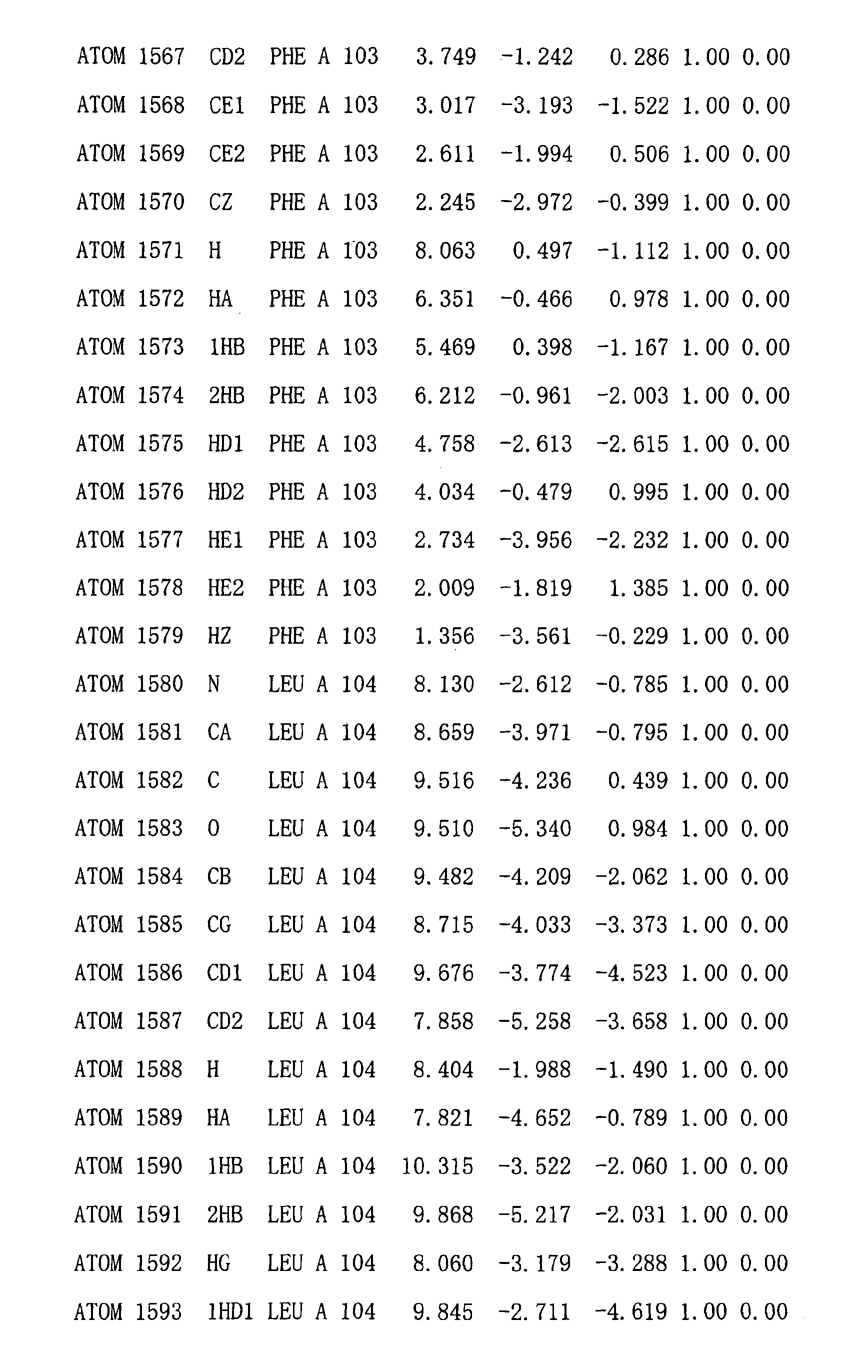

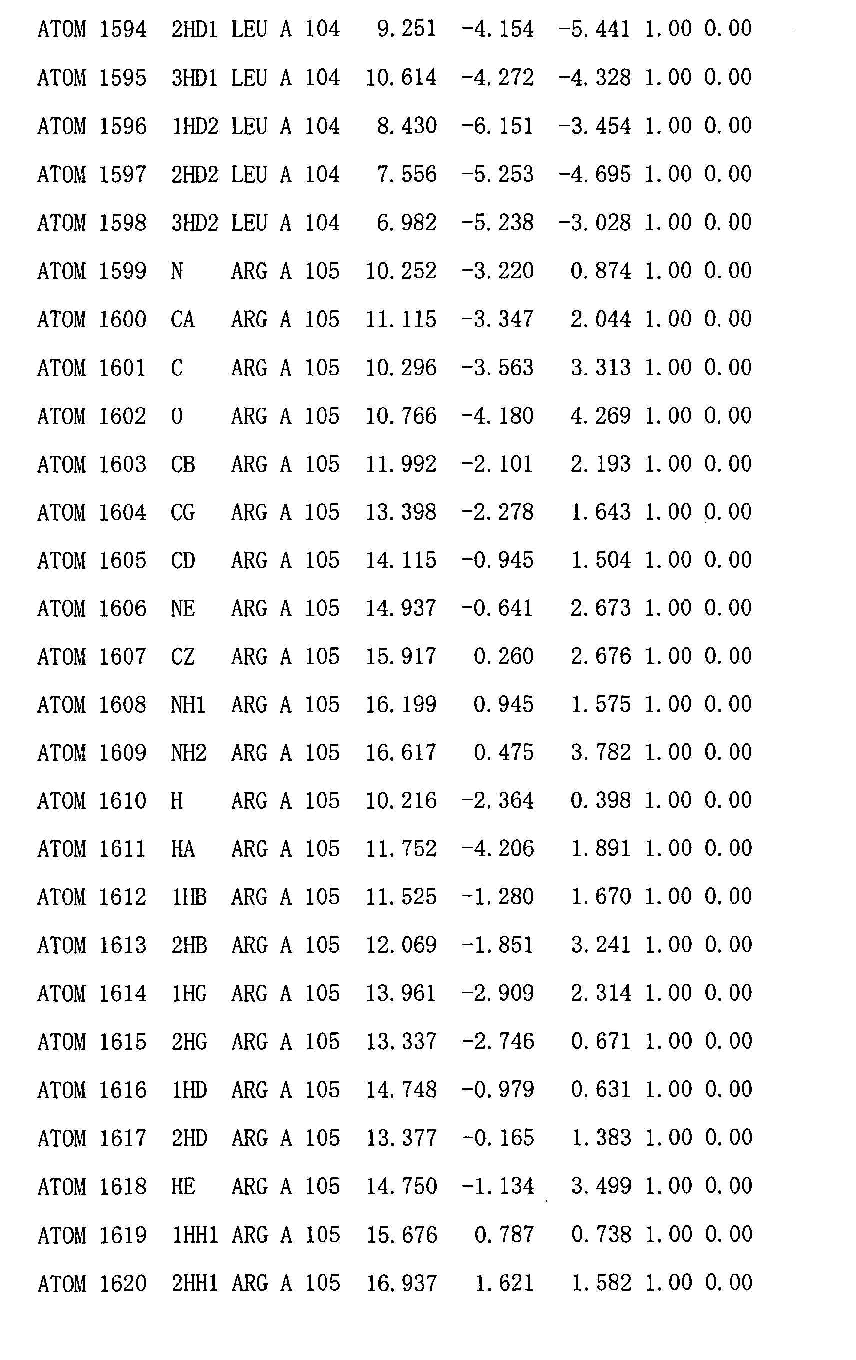

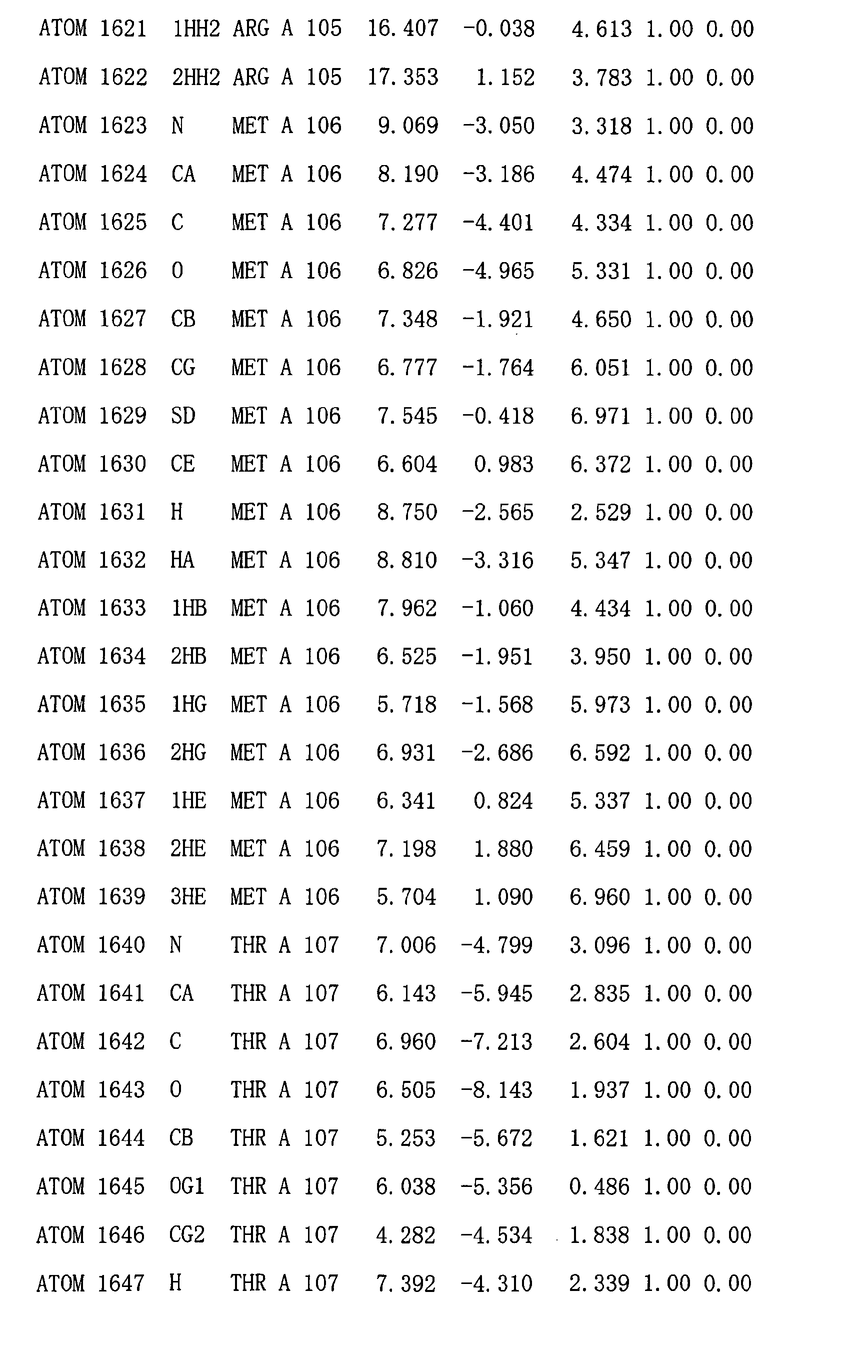

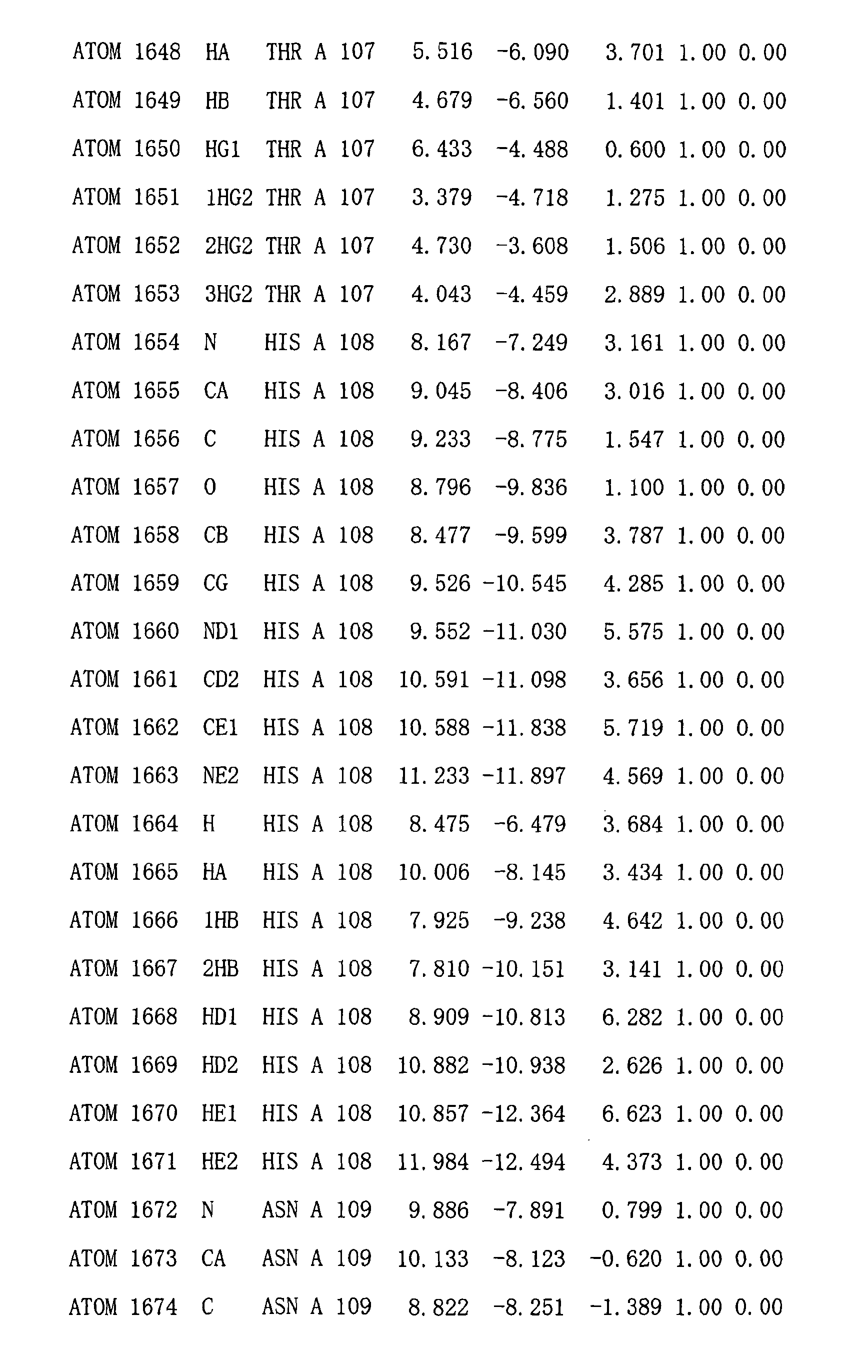

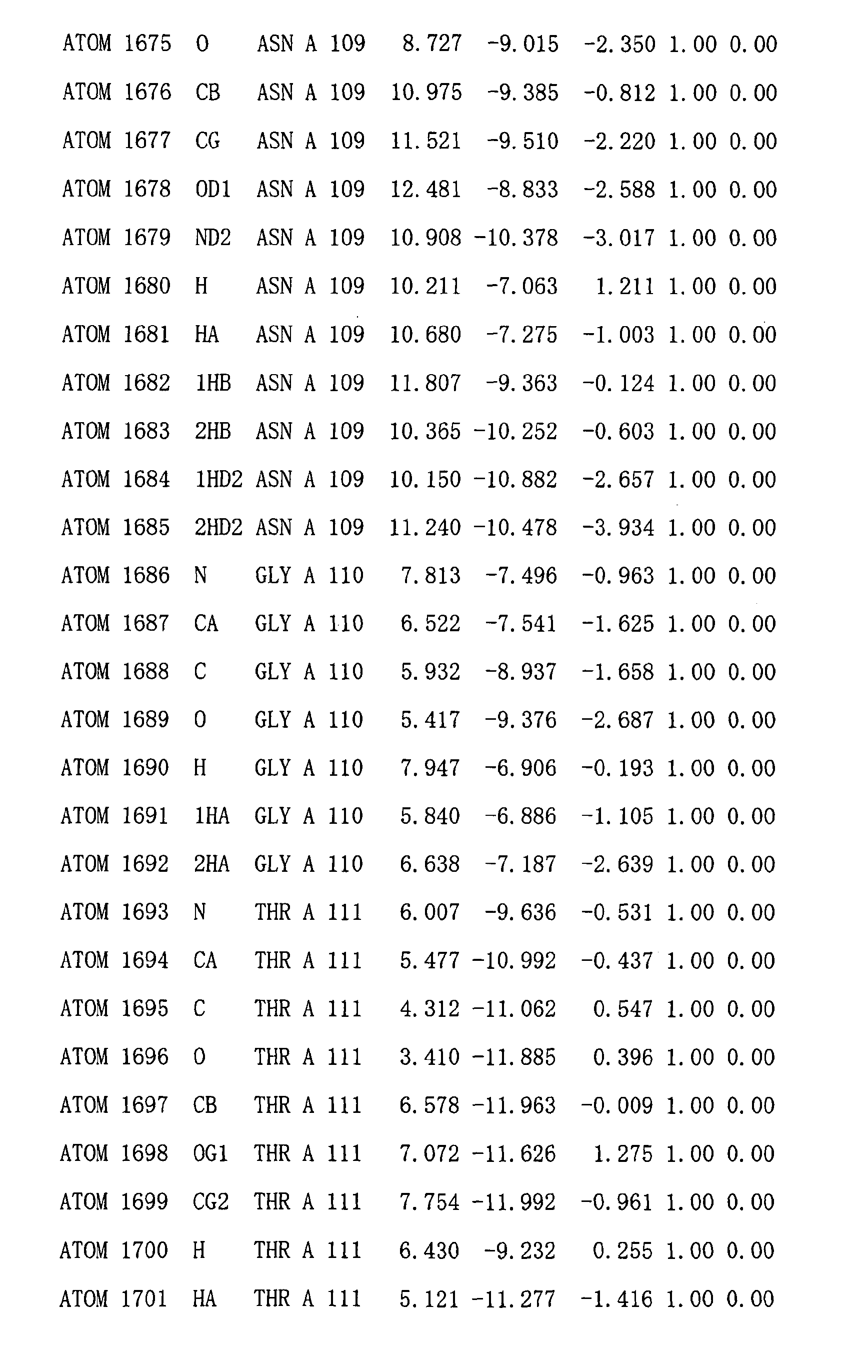

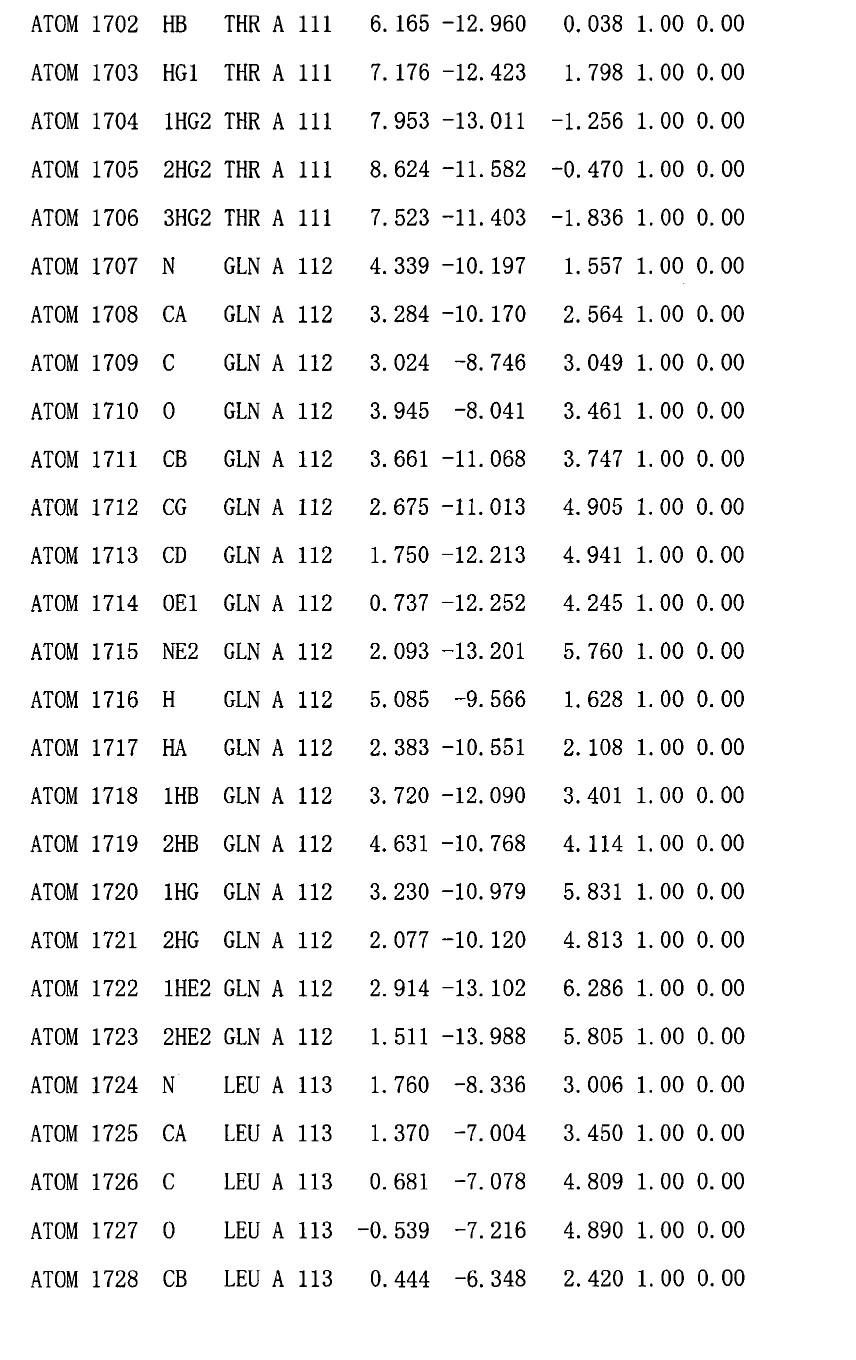

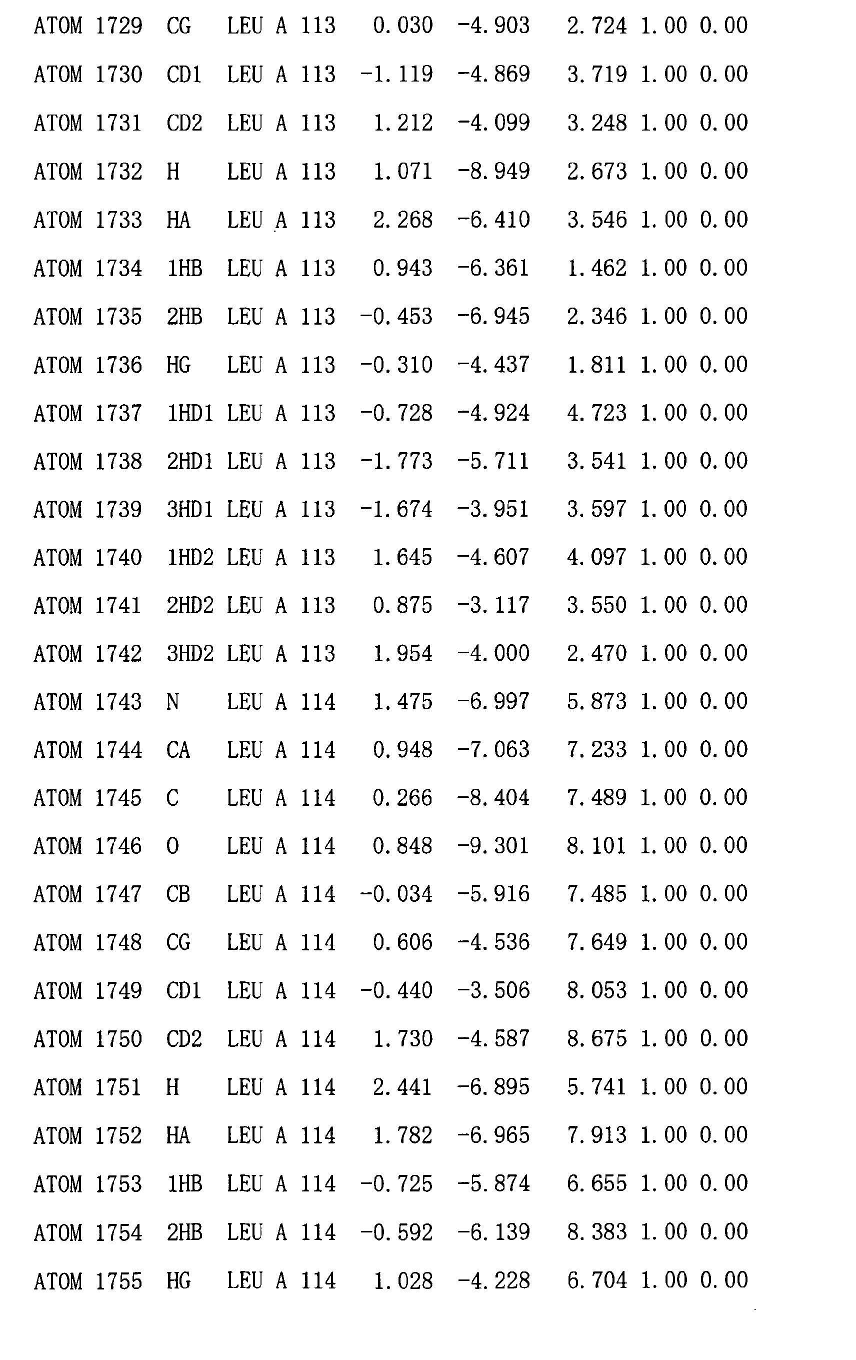

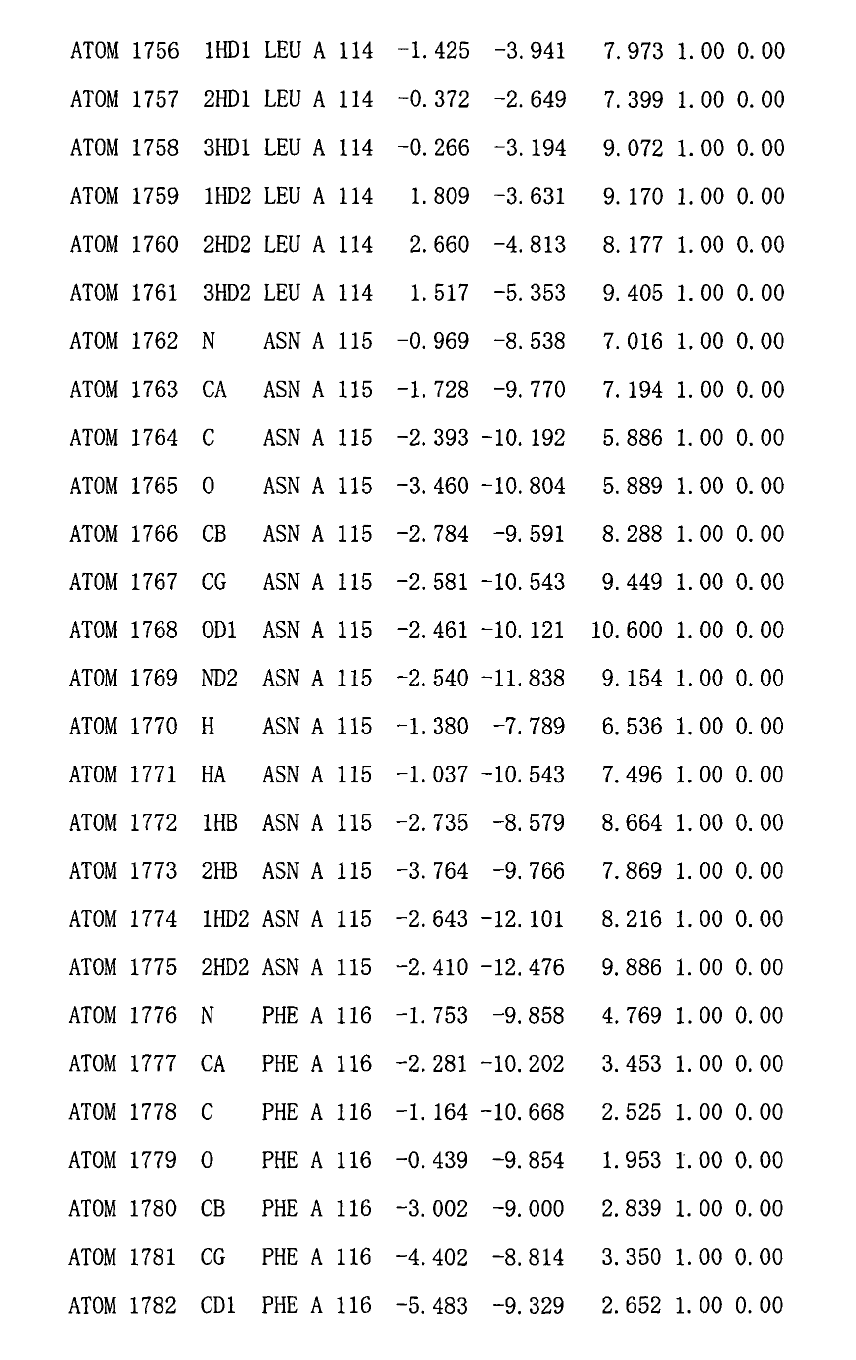

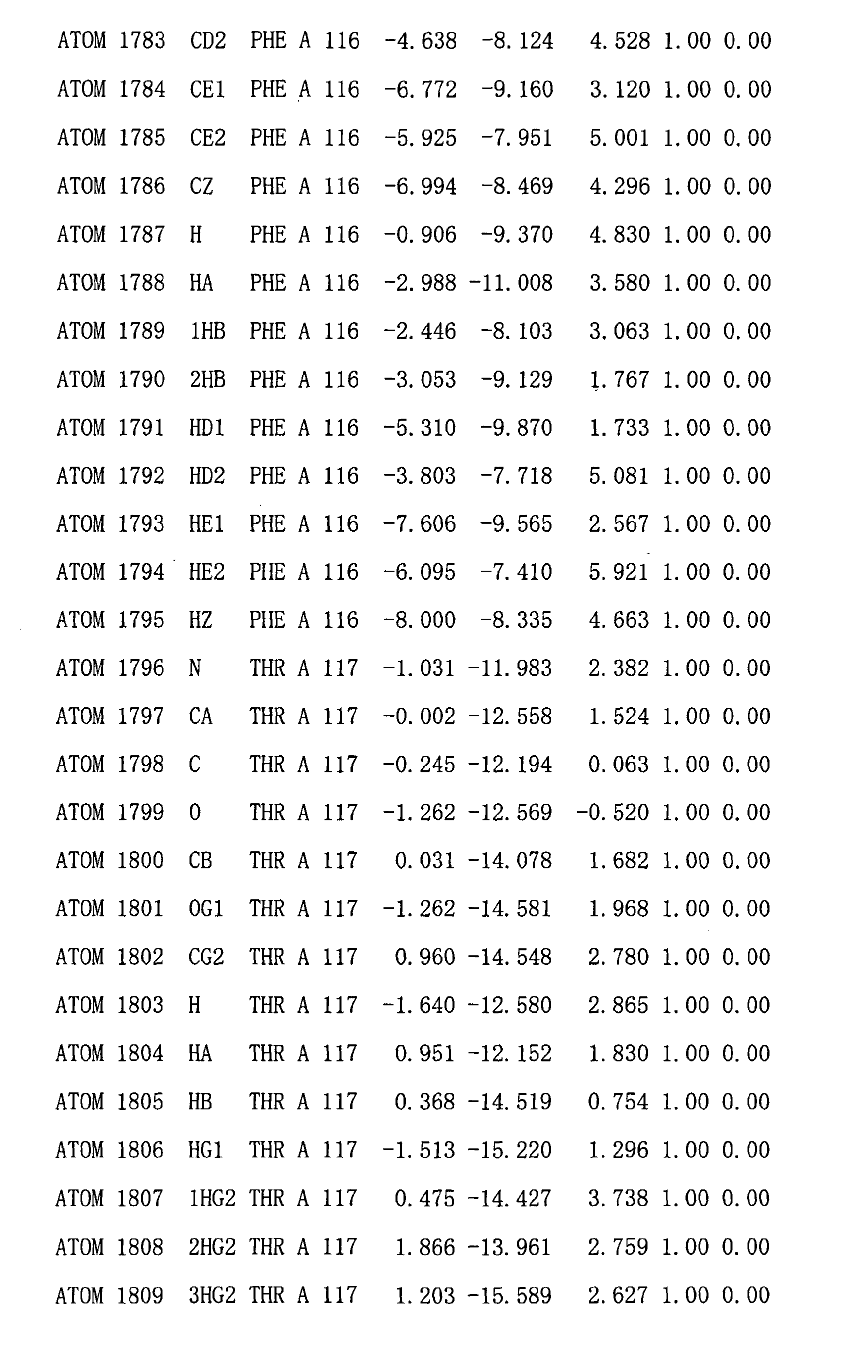

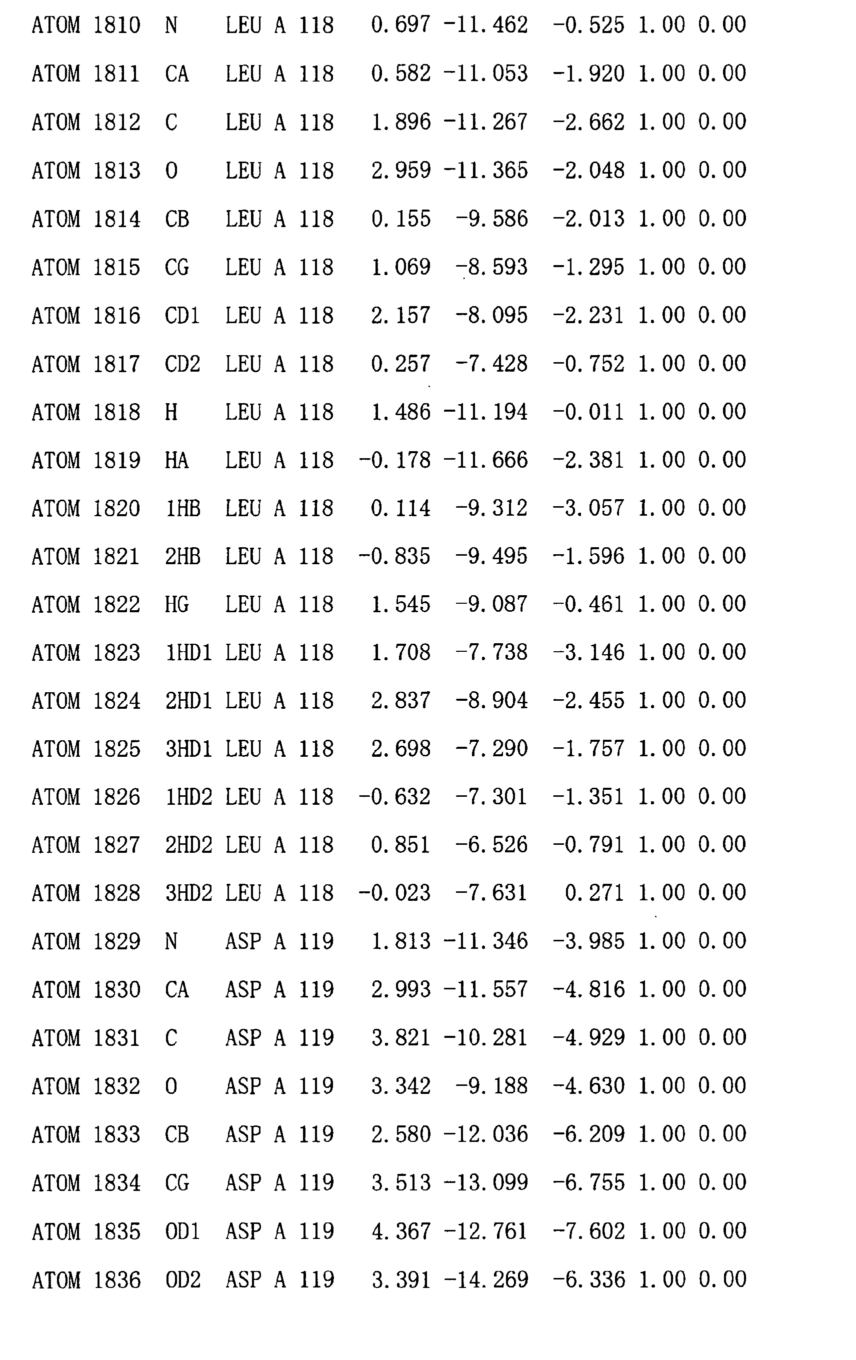

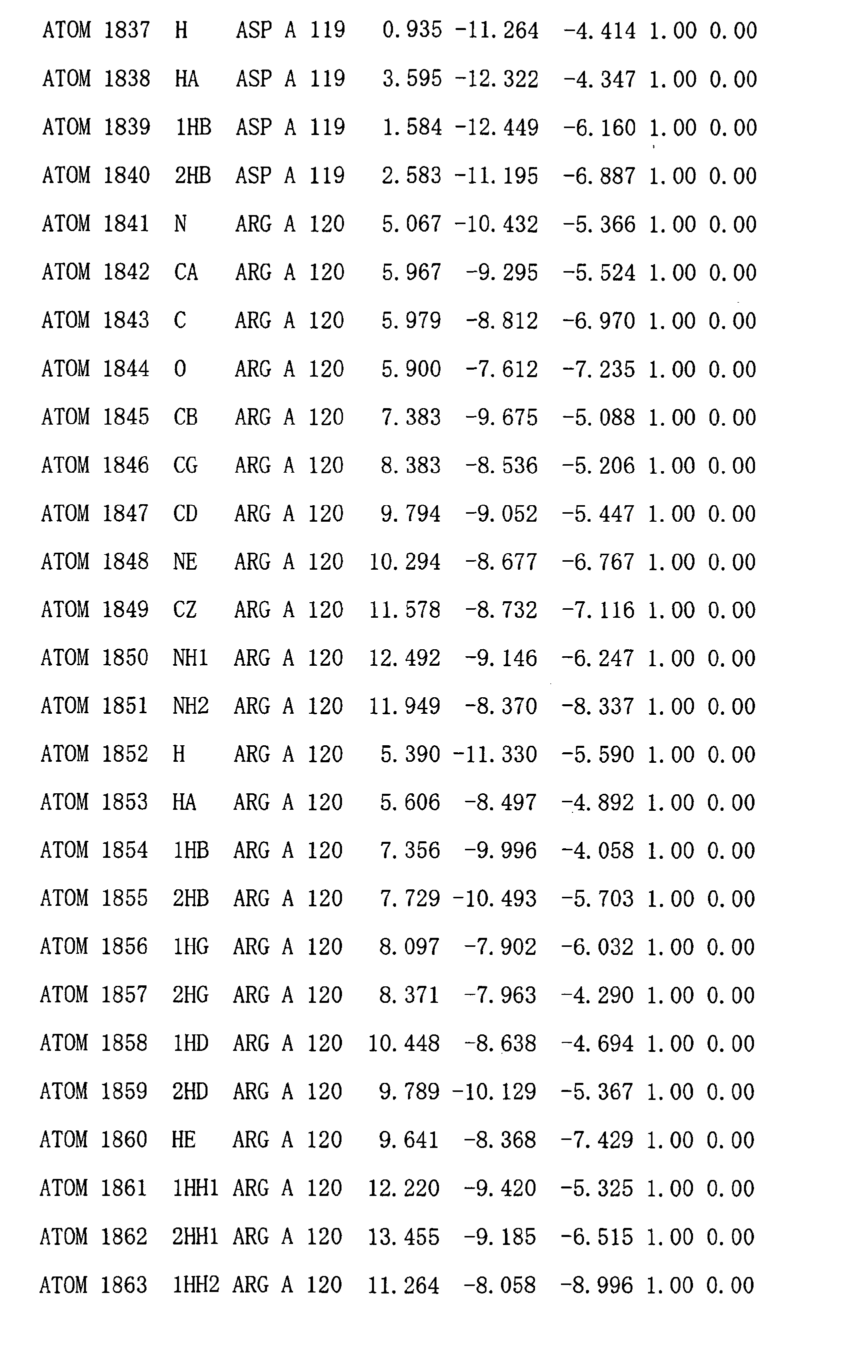

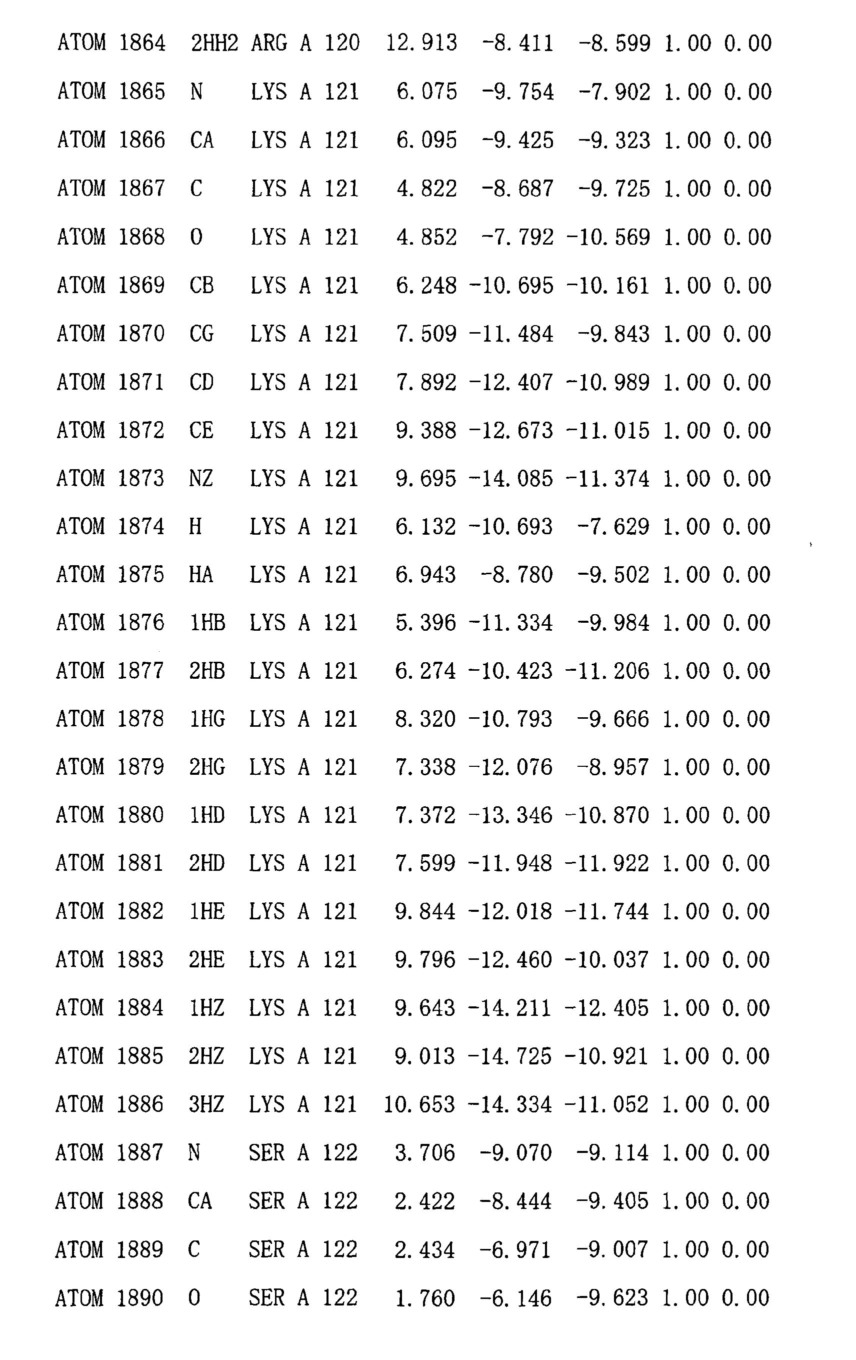

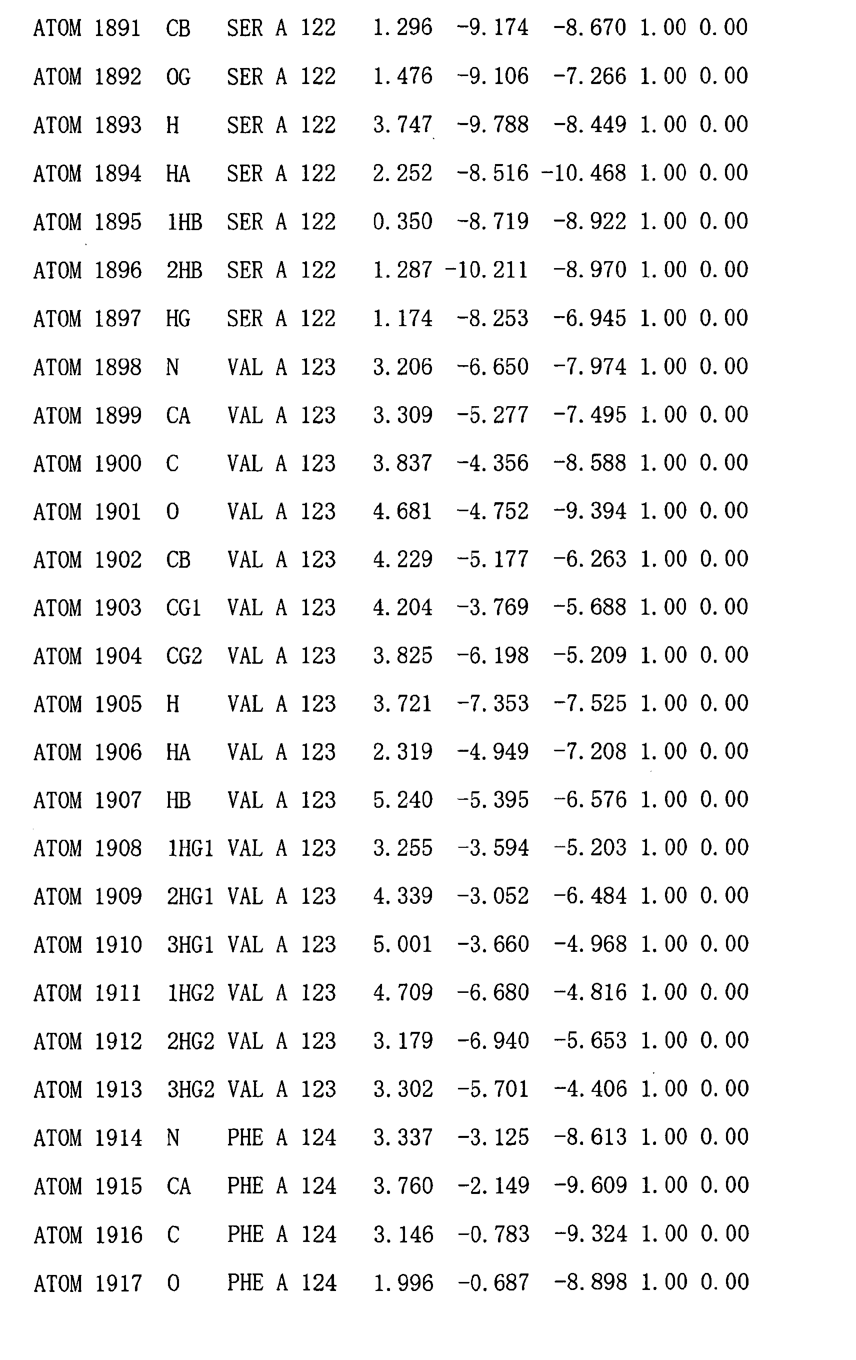

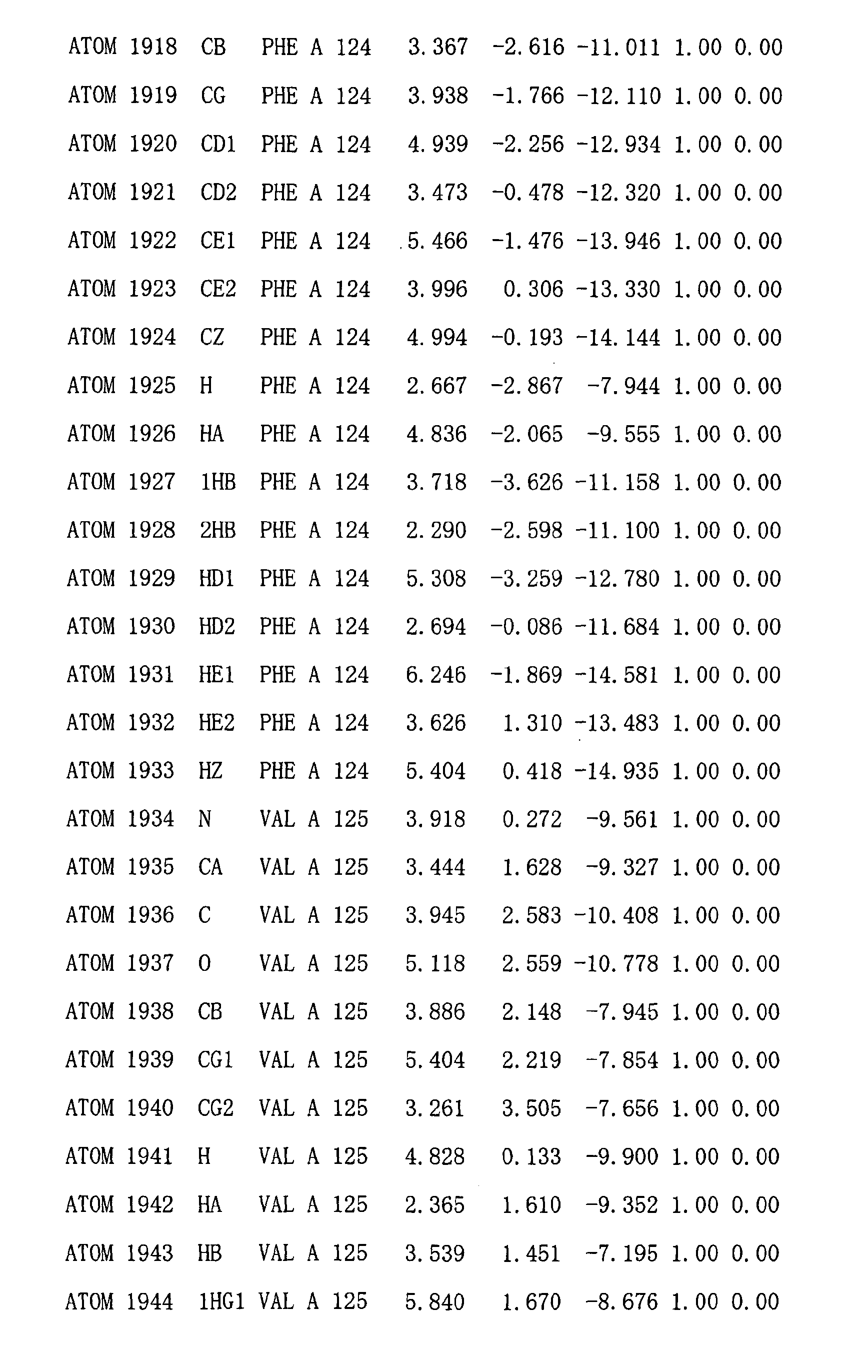

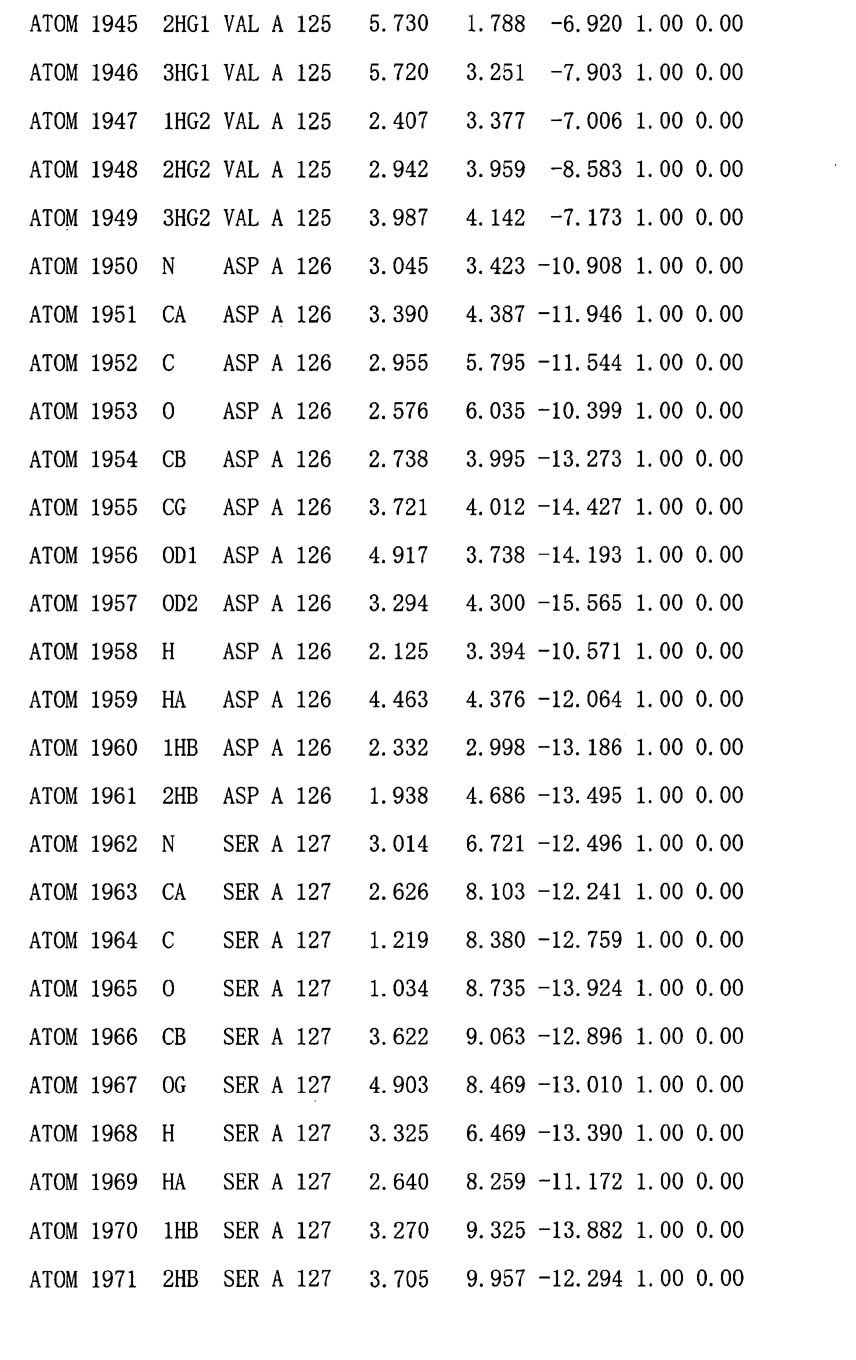

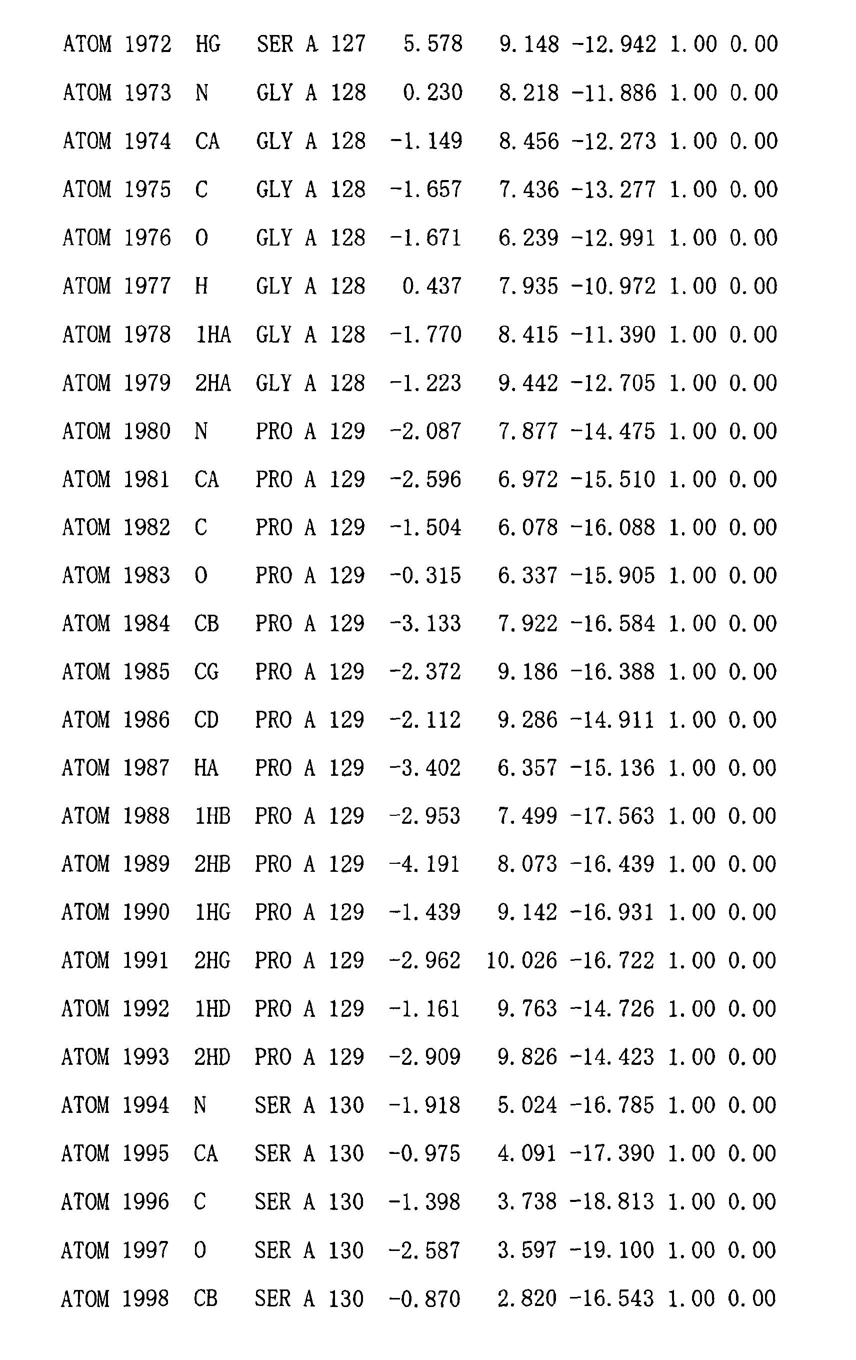

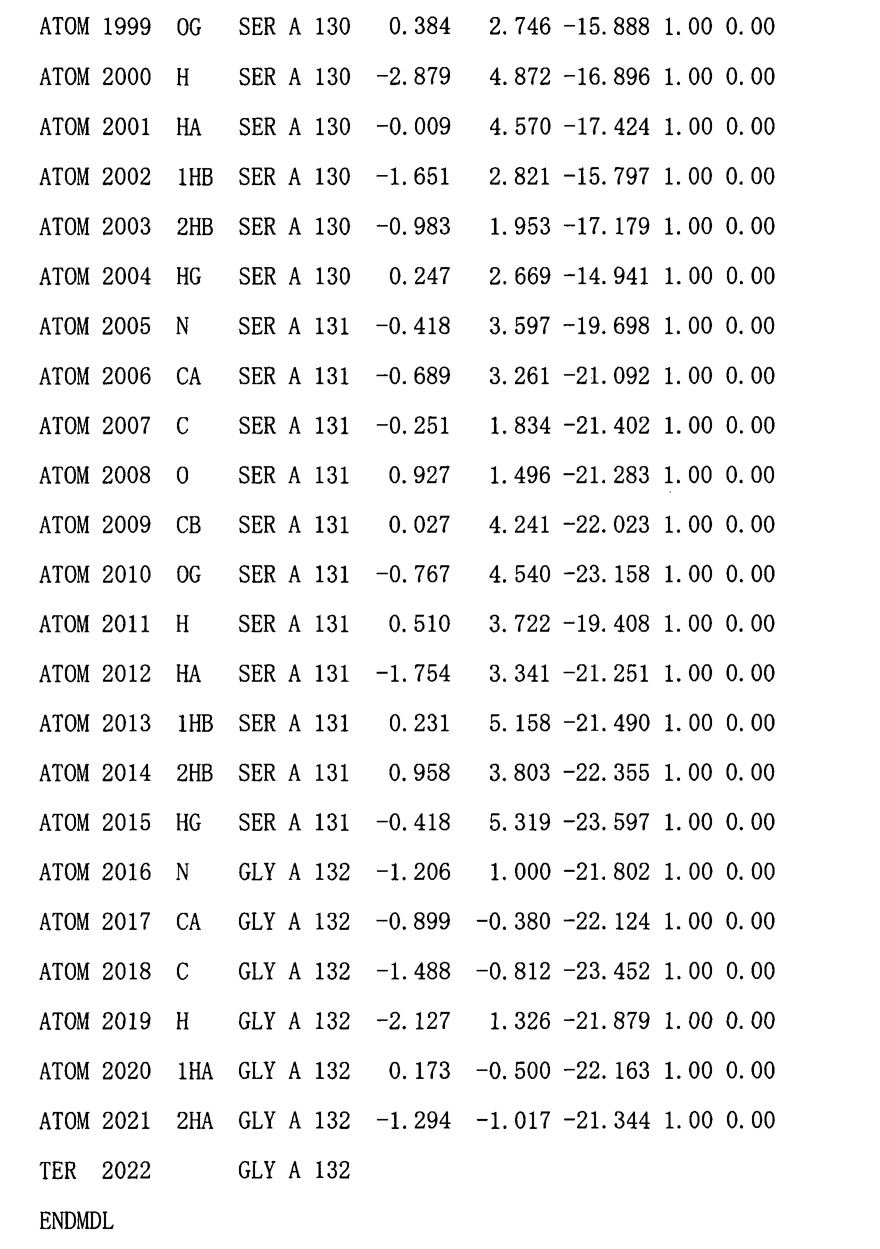

### Three-Dimensional Structure Coordinate 6

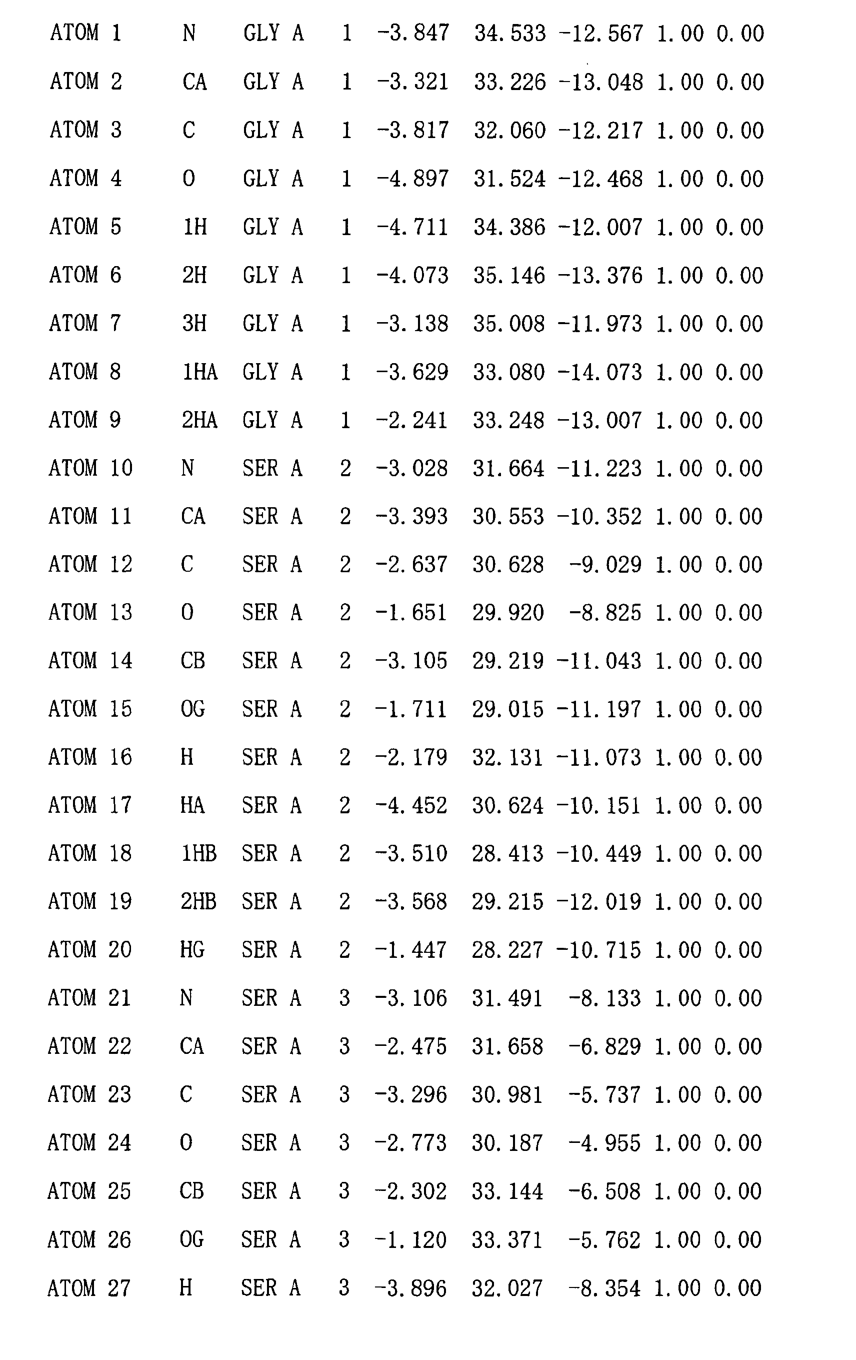

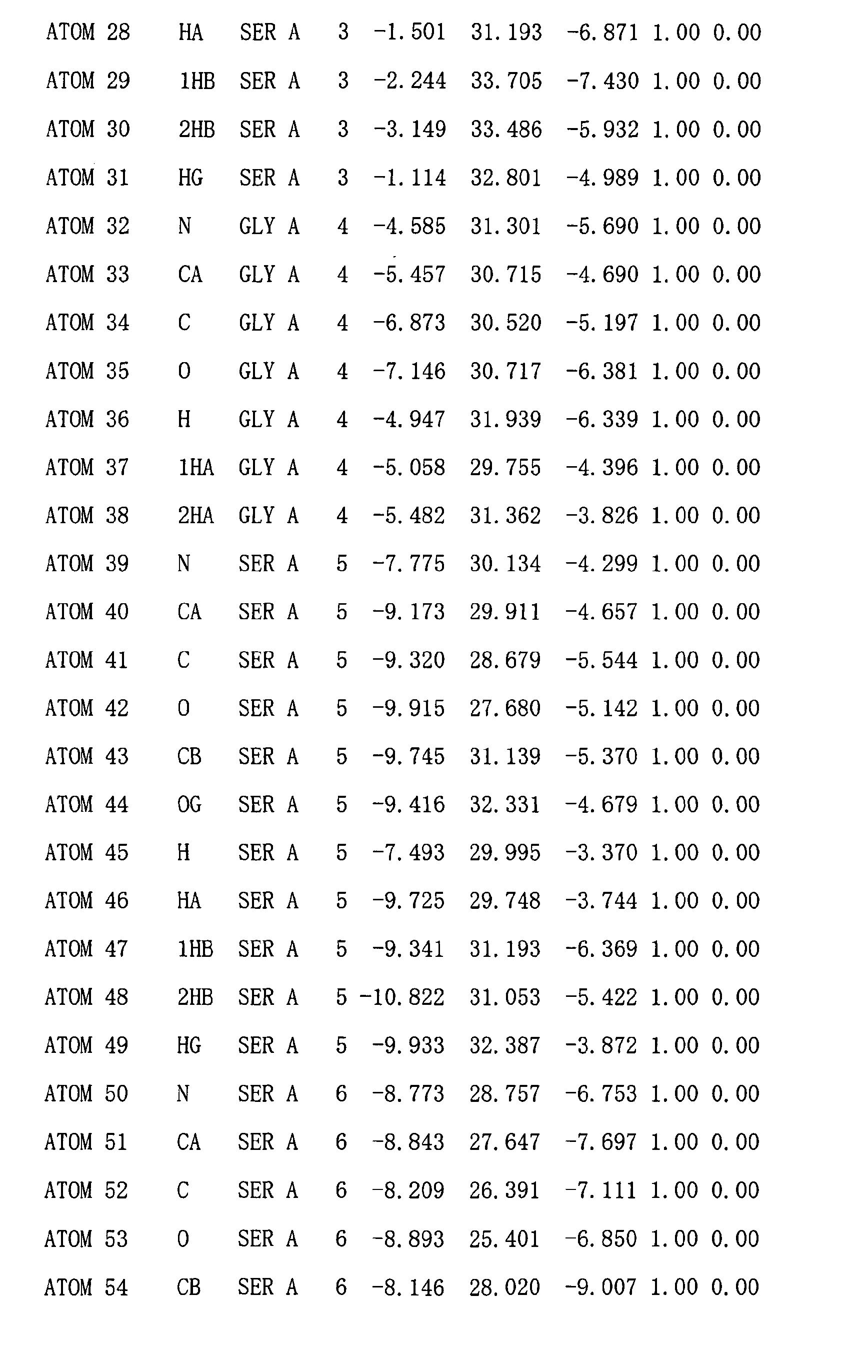

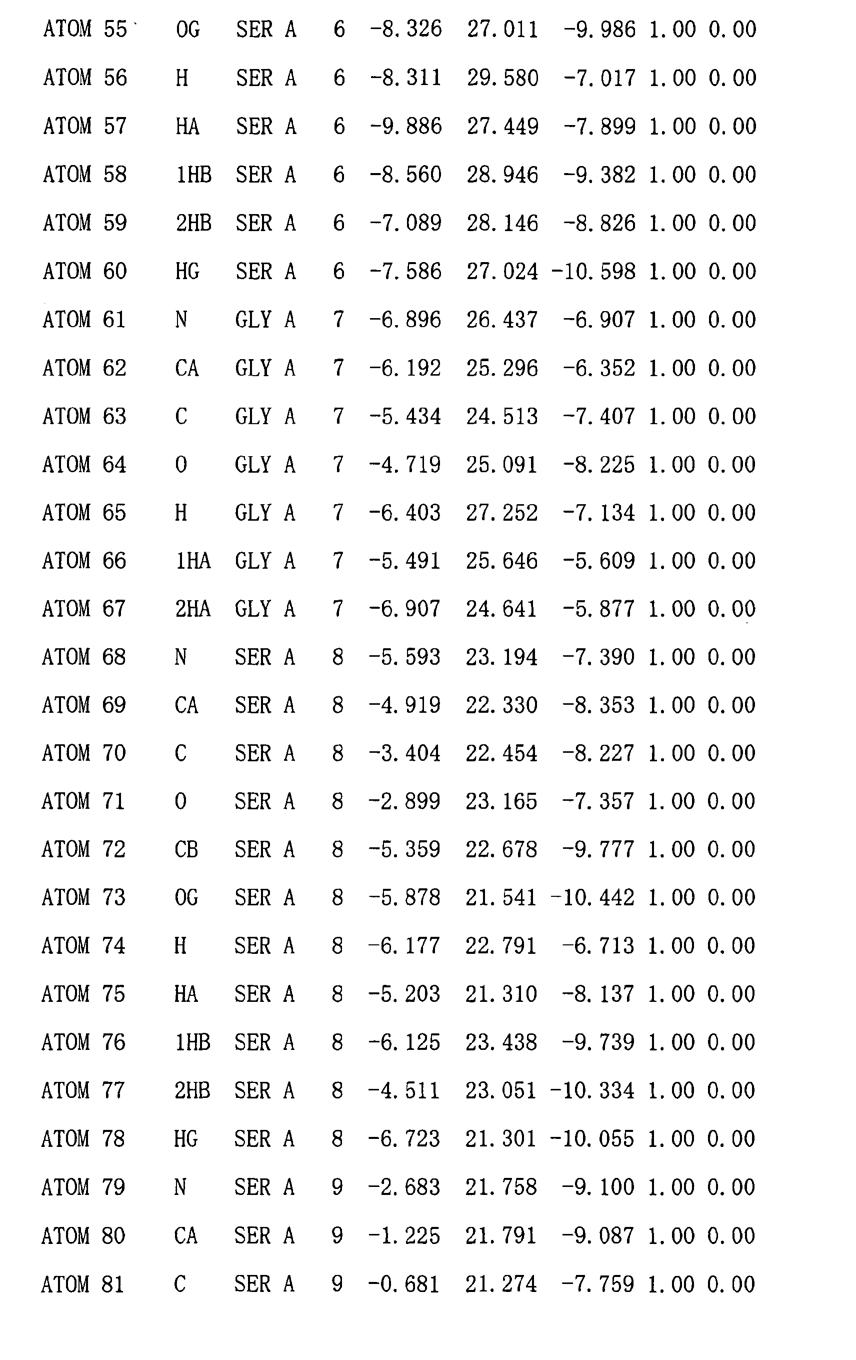

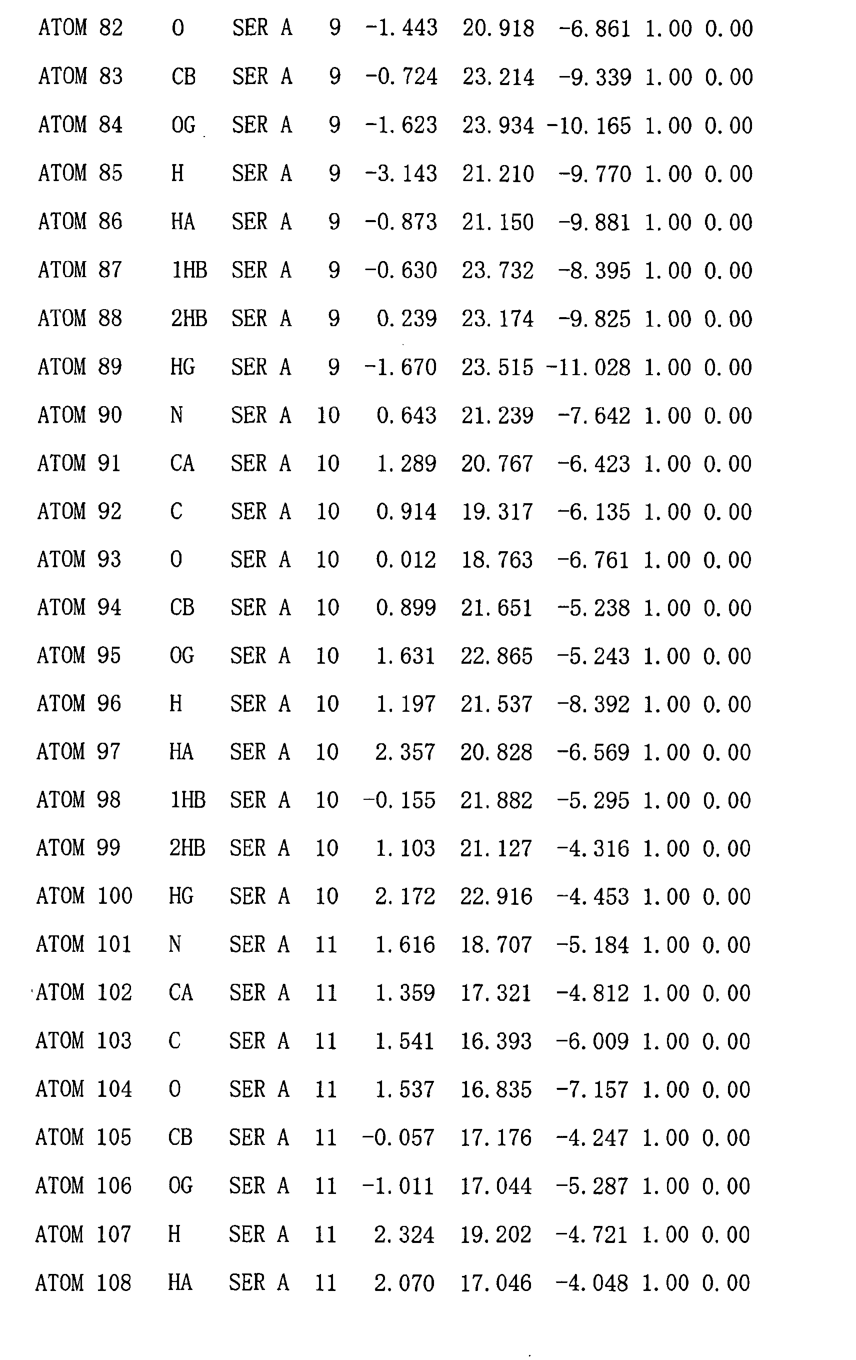

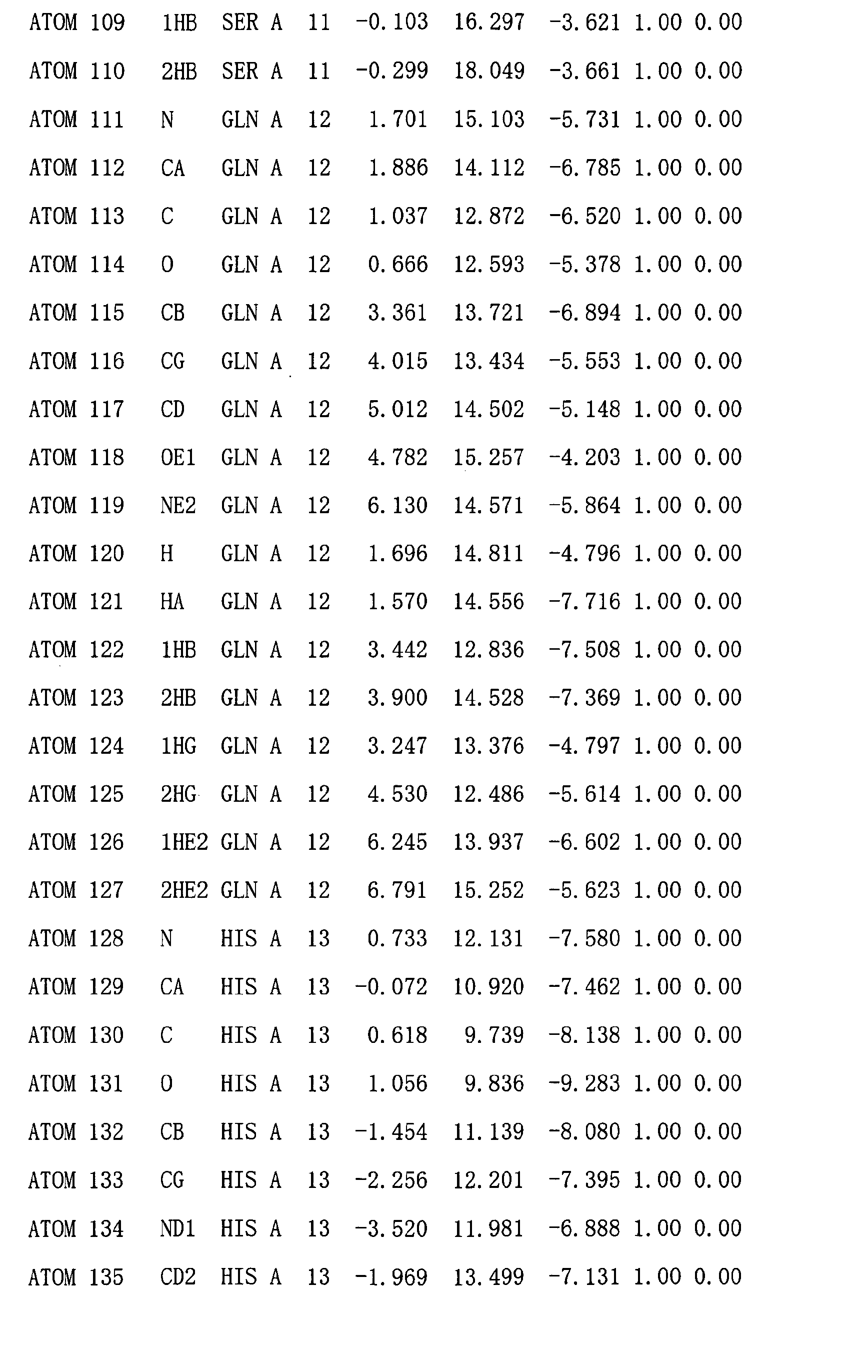

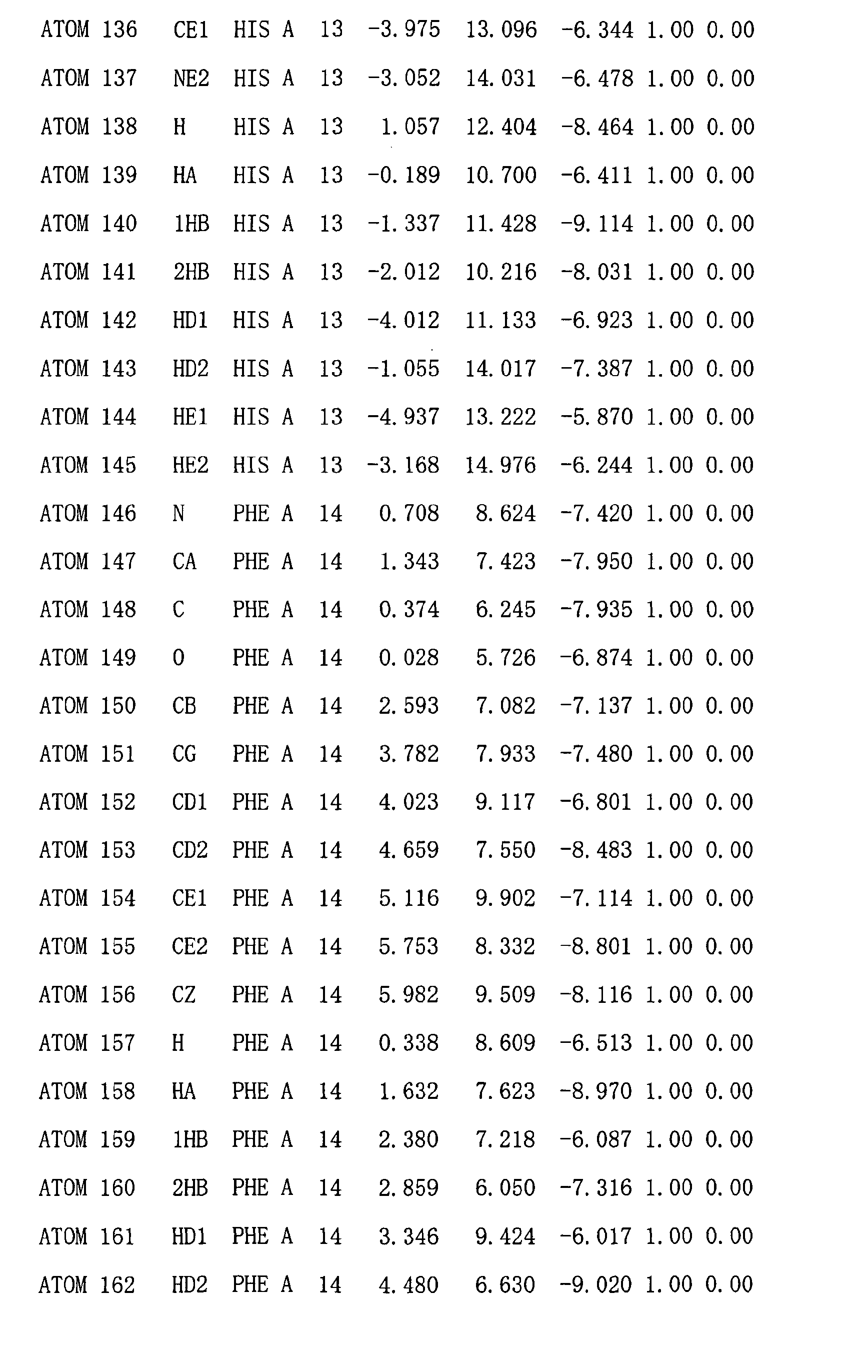

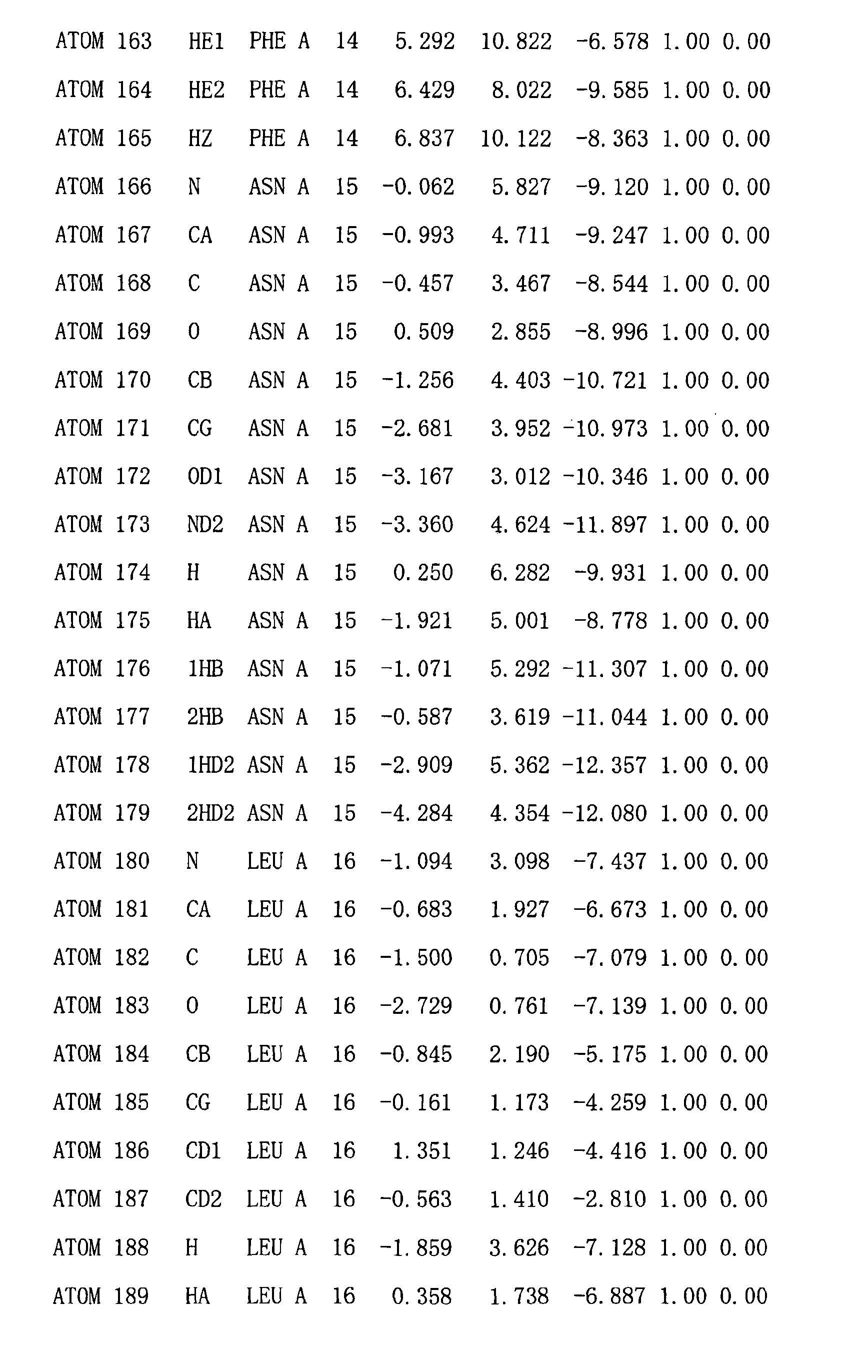

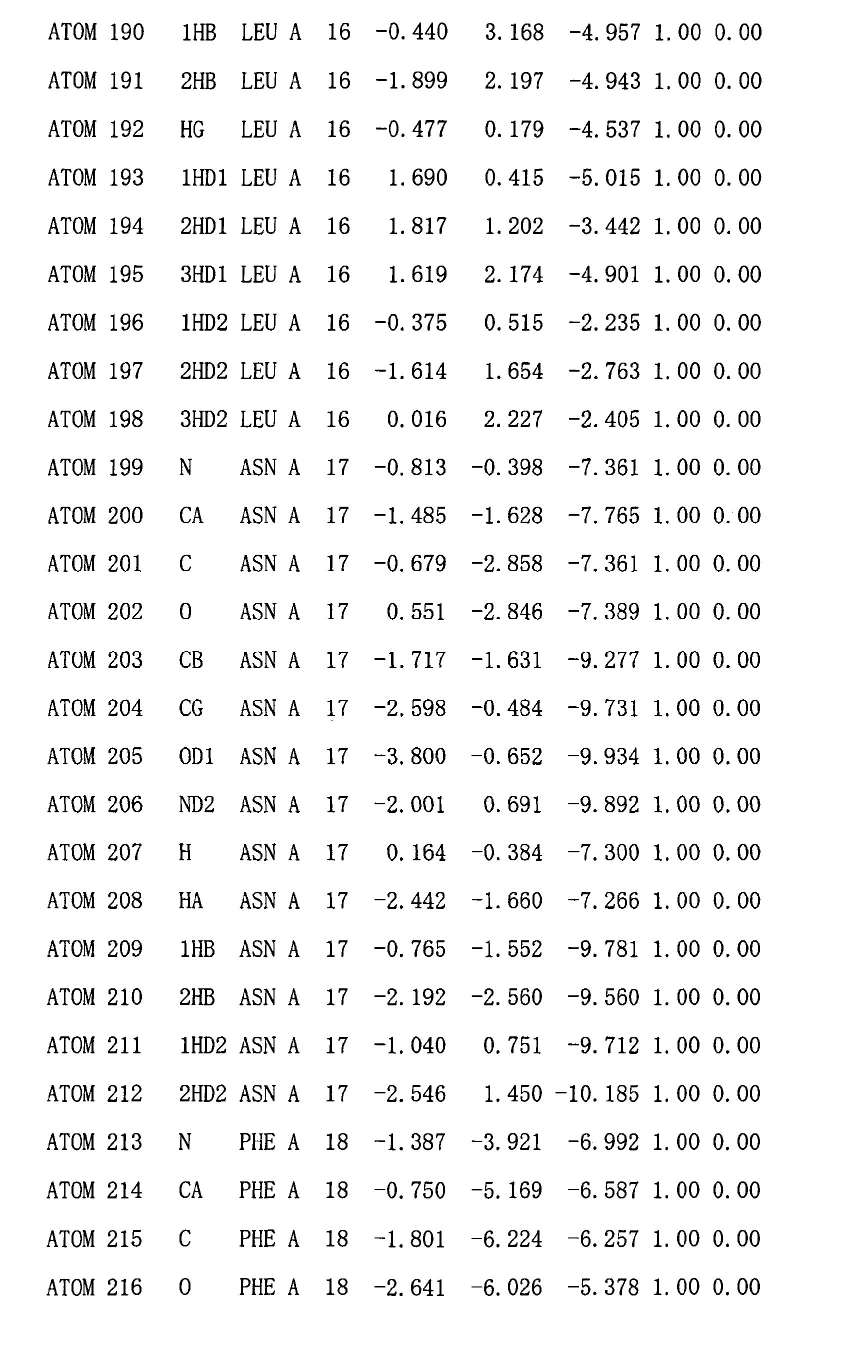

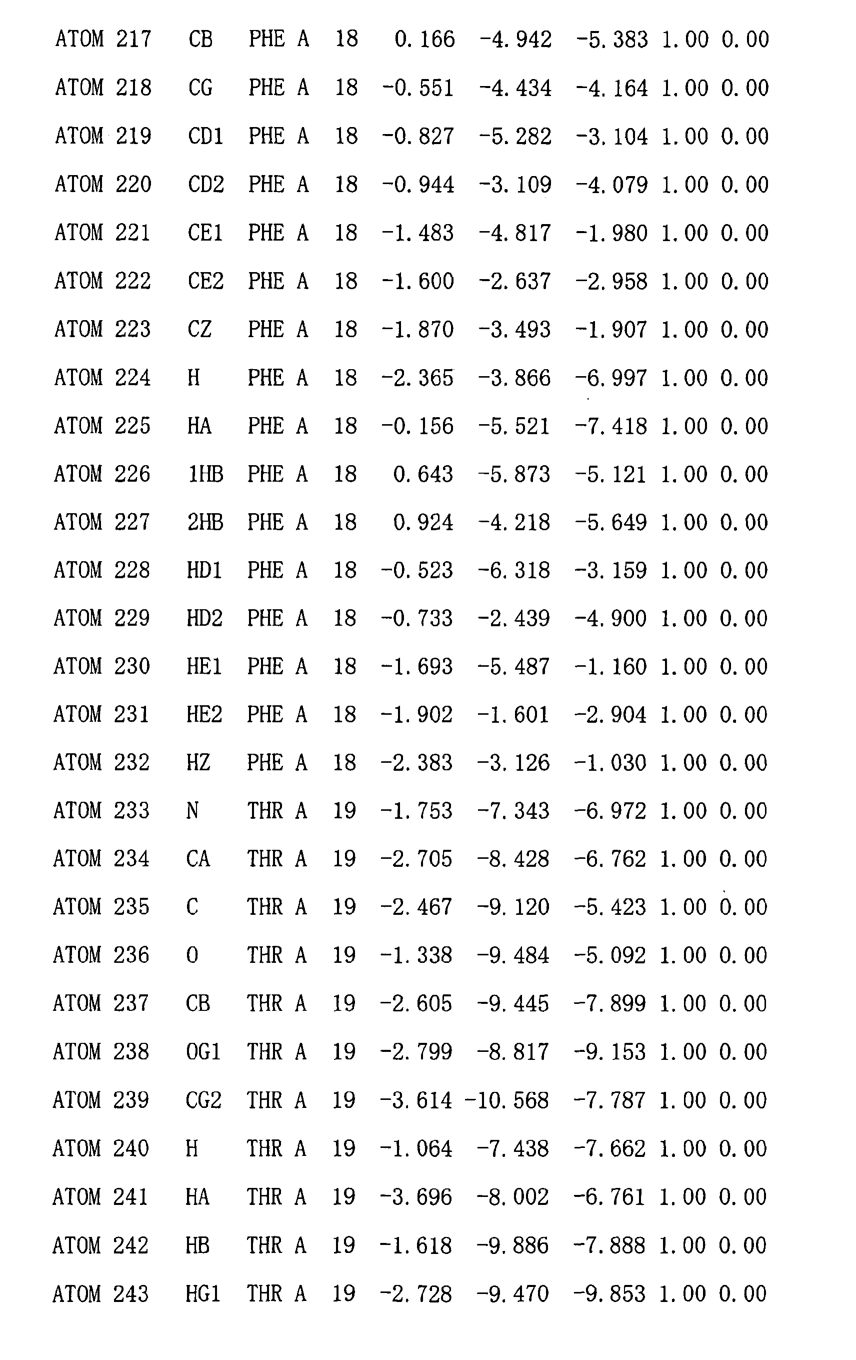

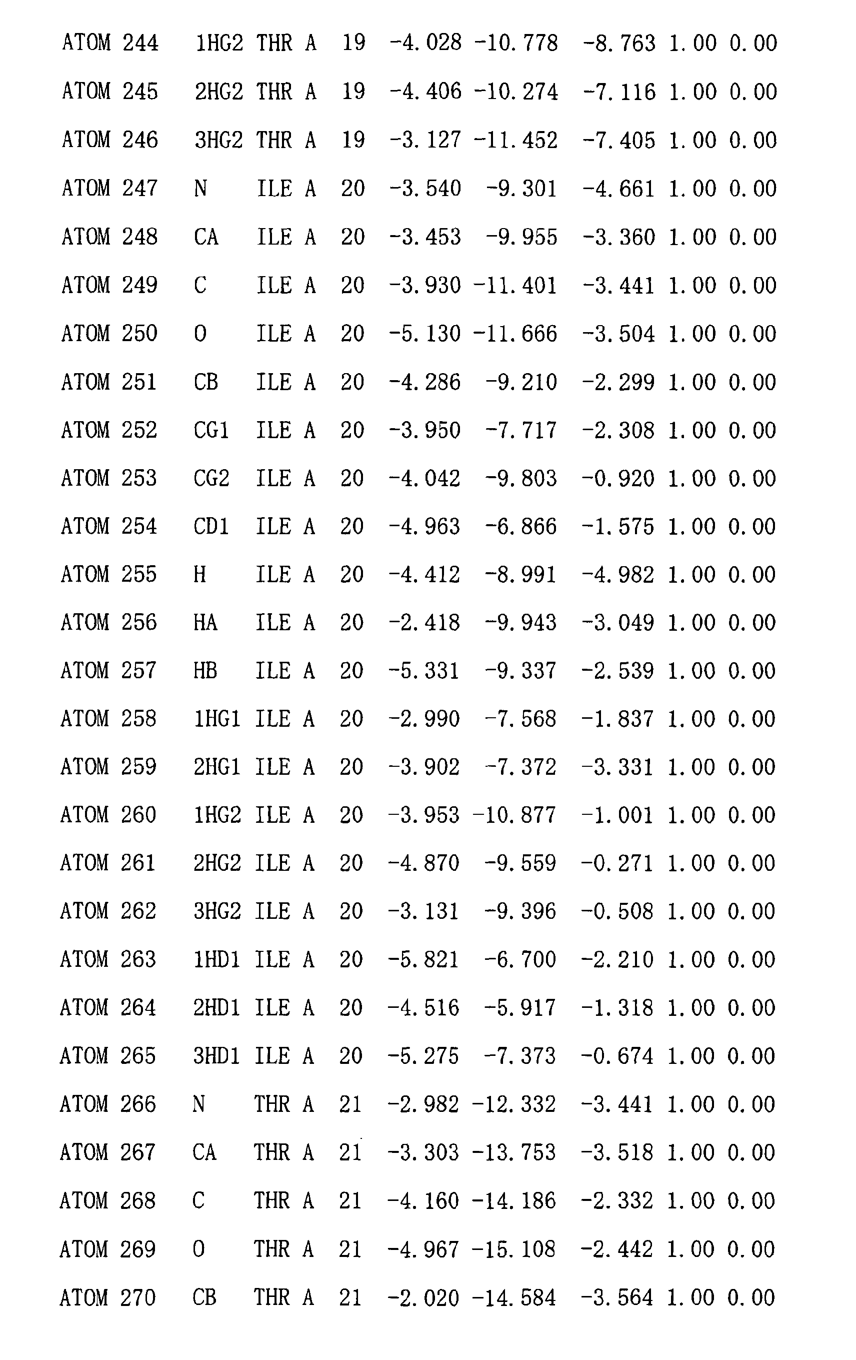

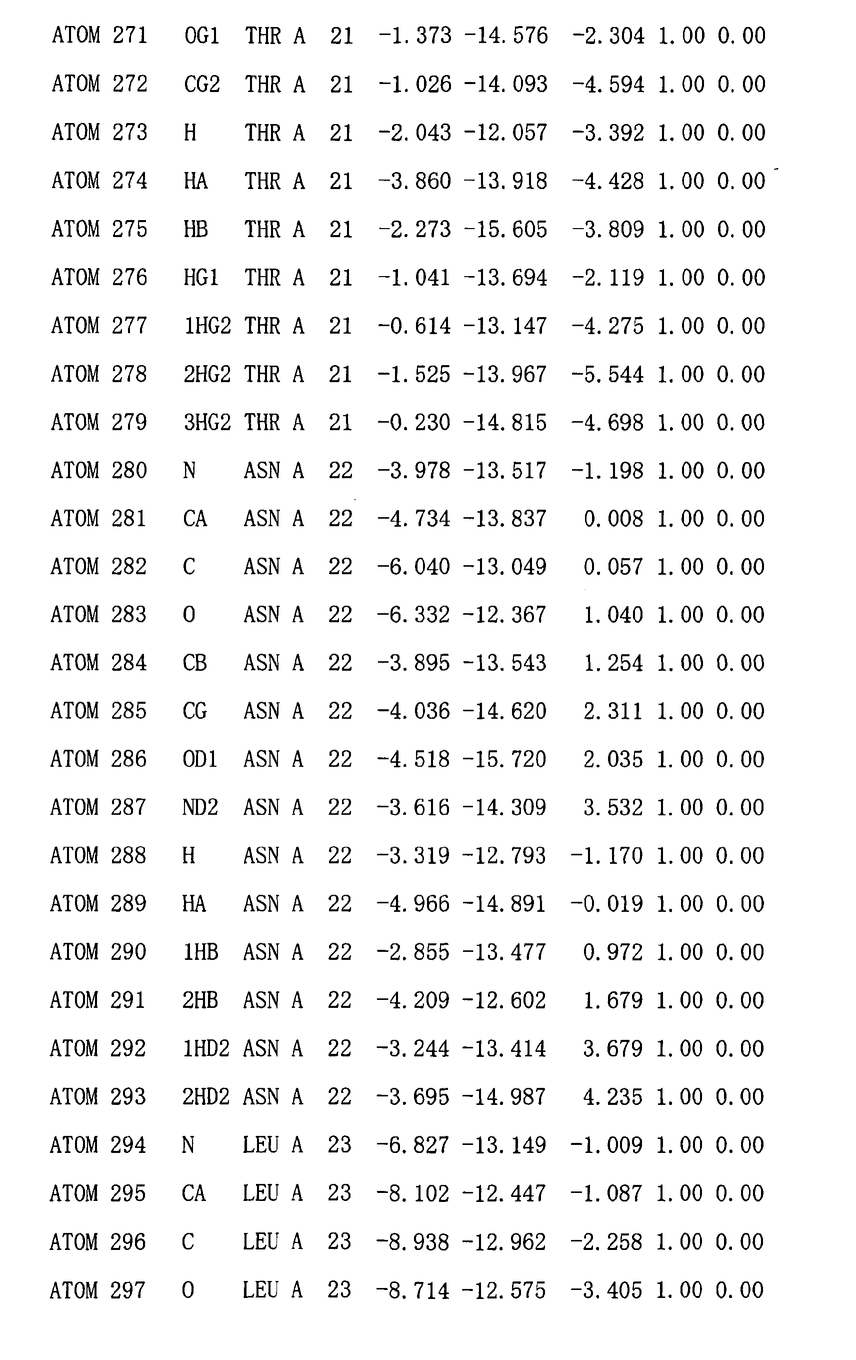

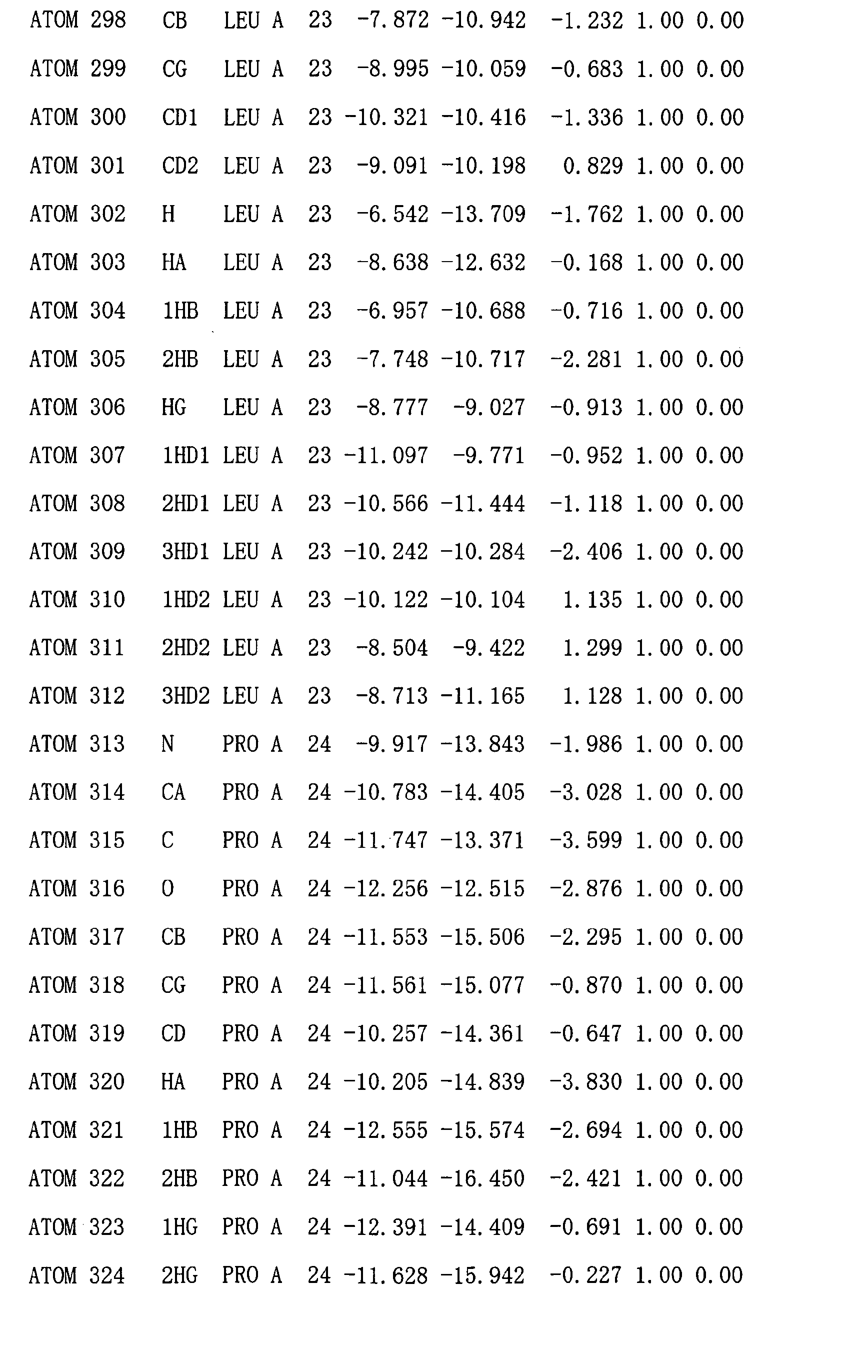

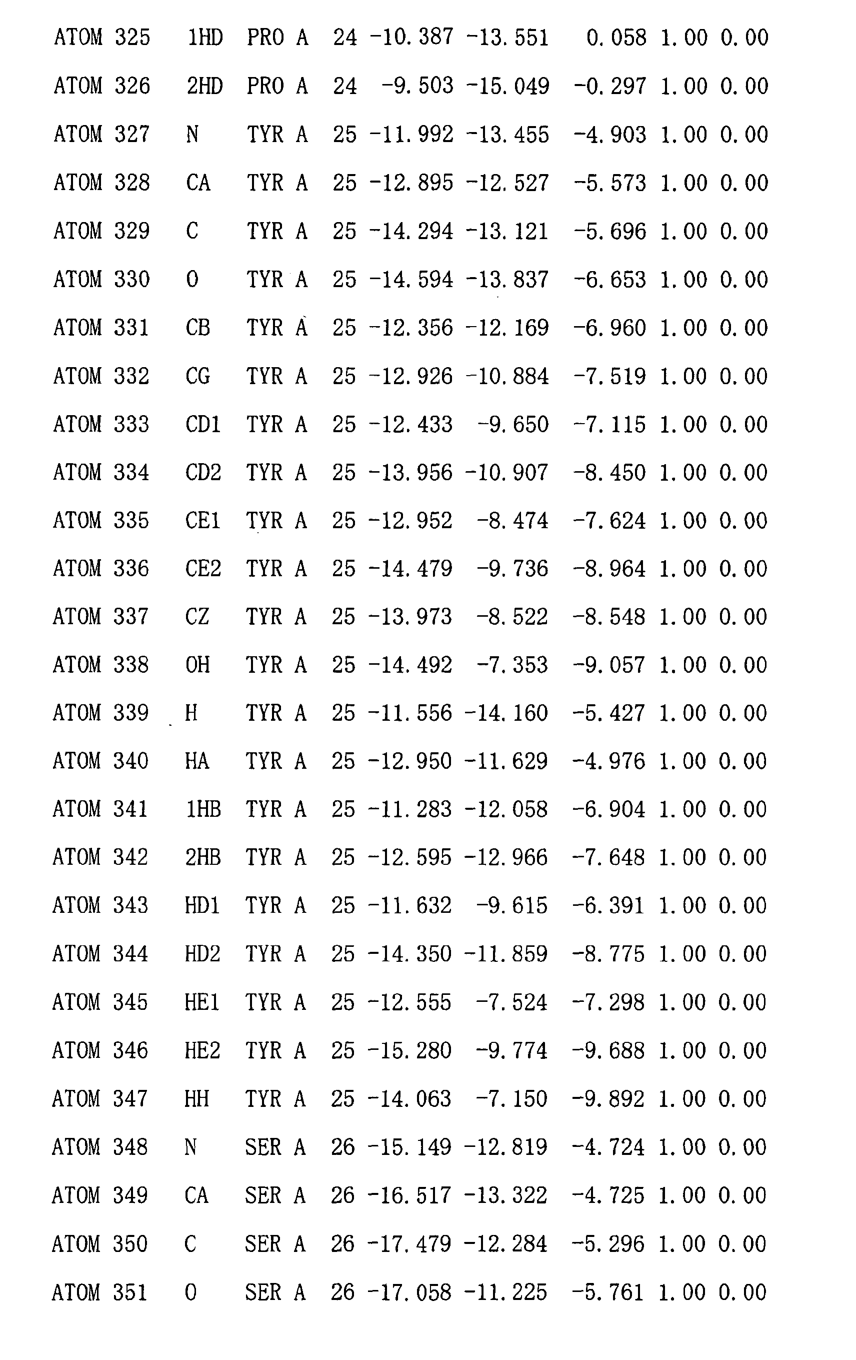

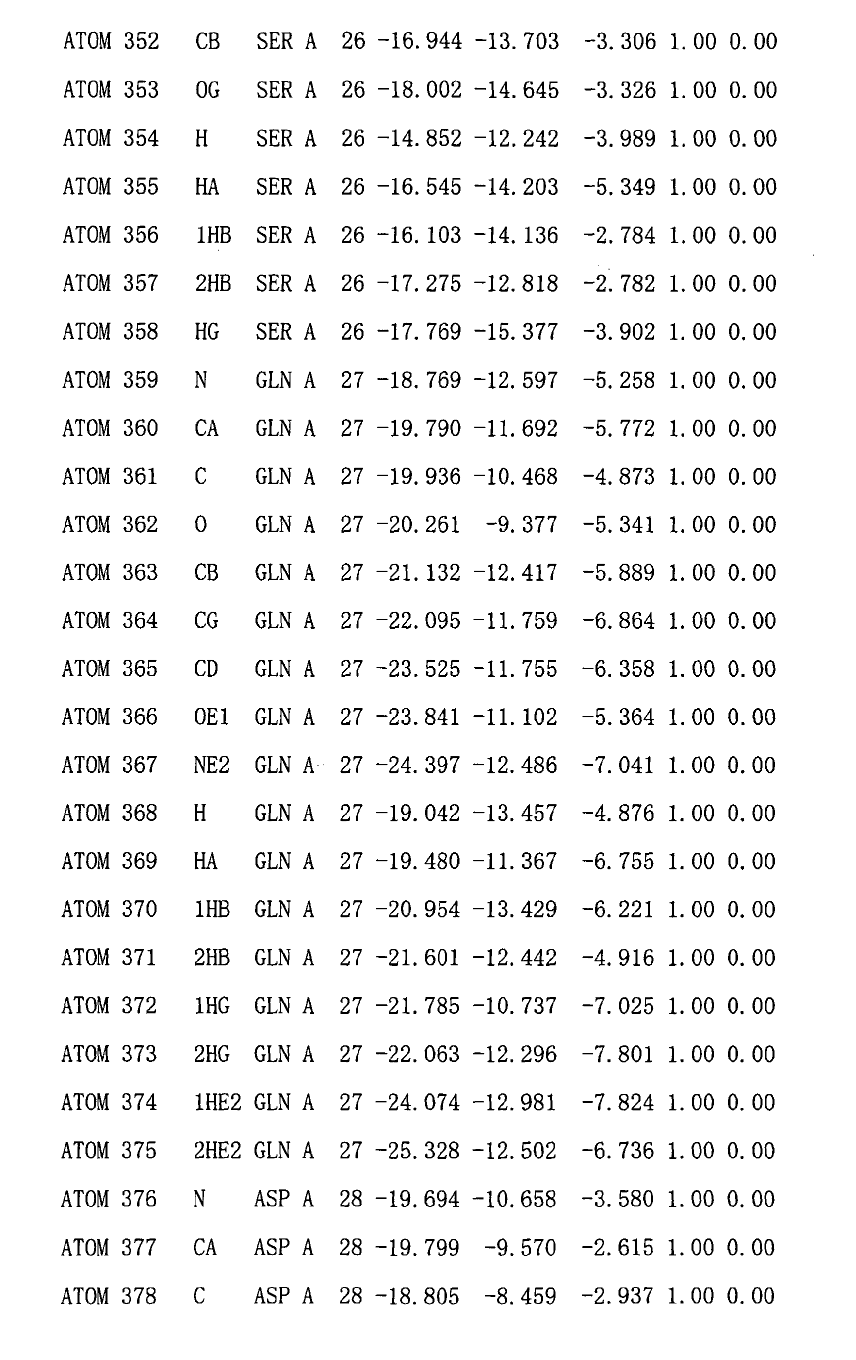

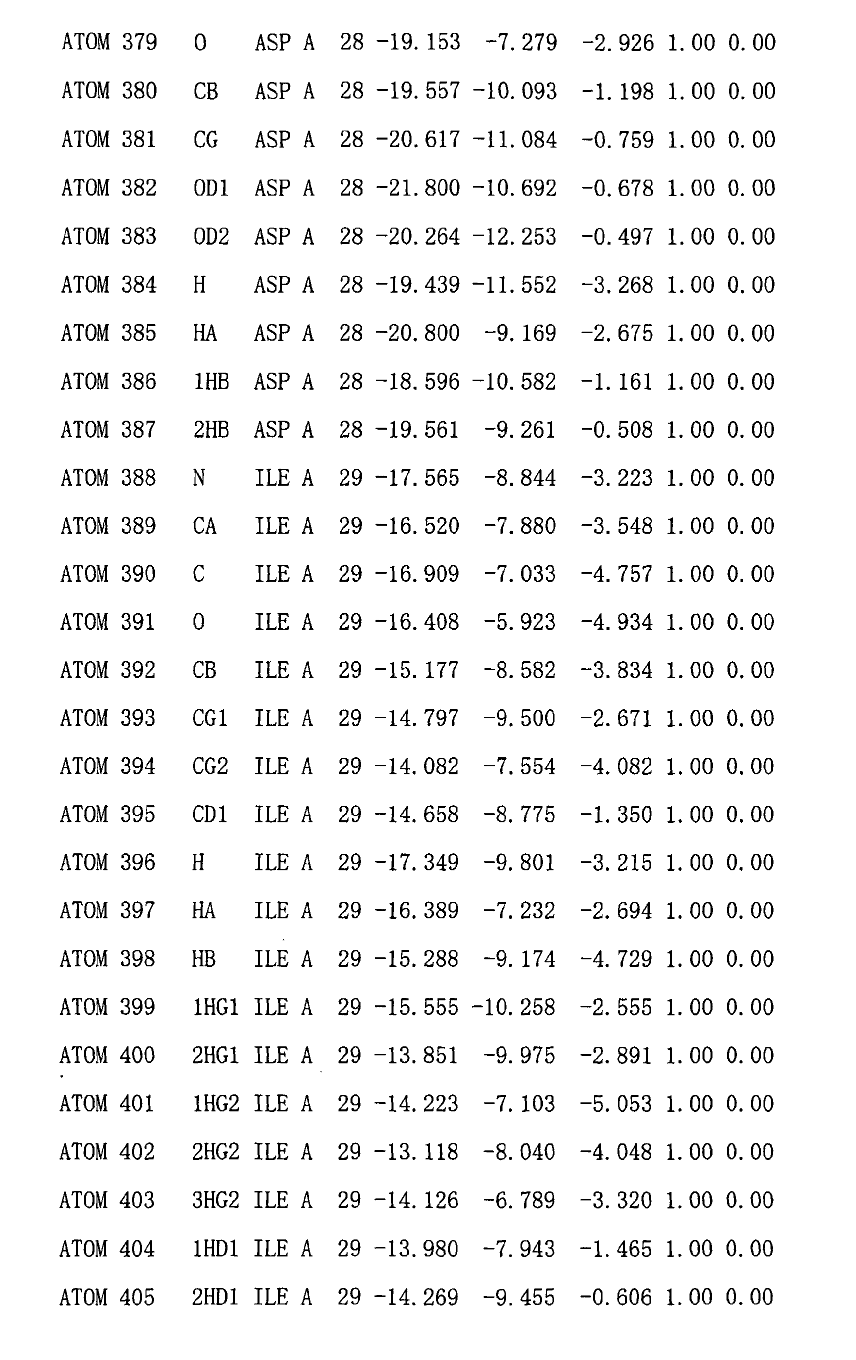

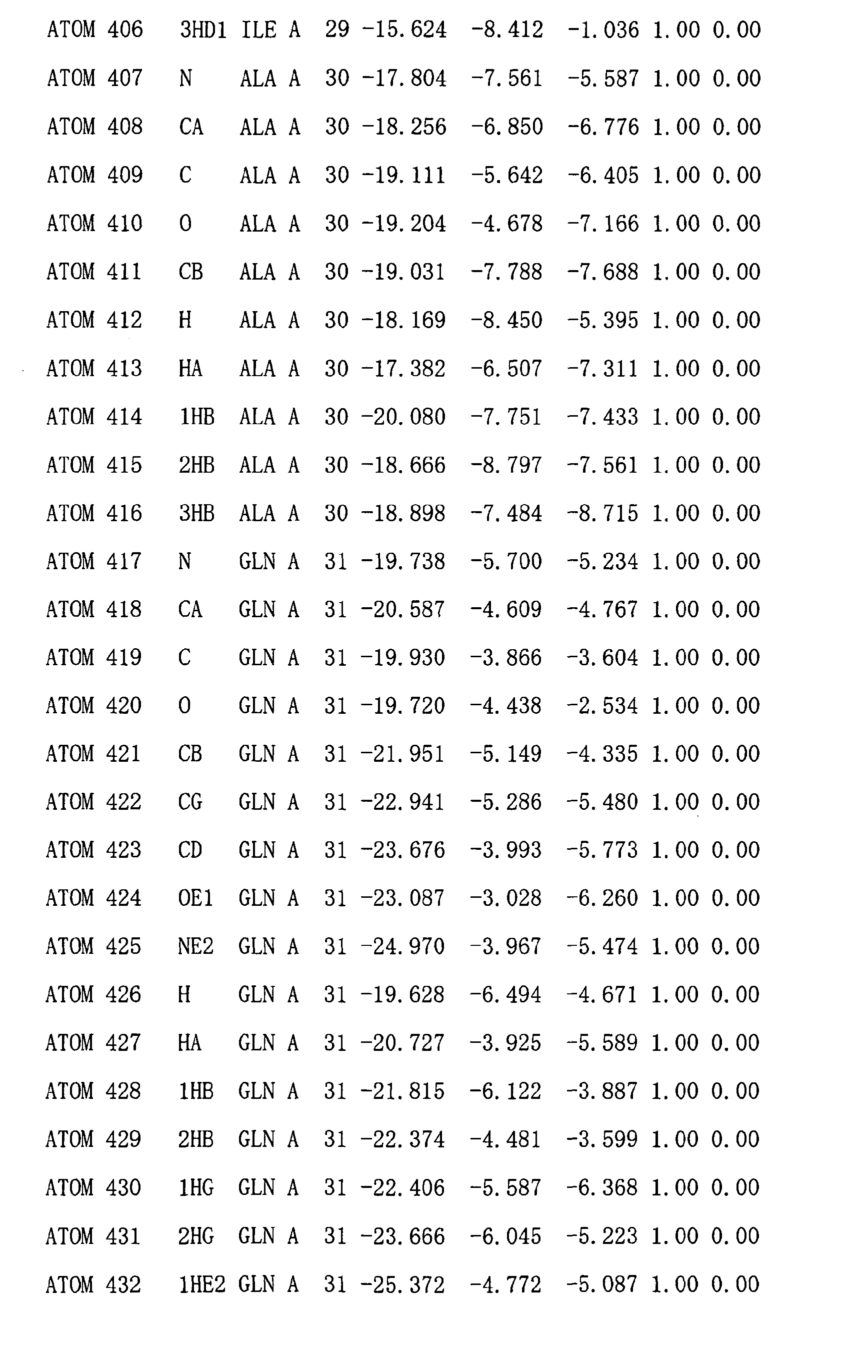

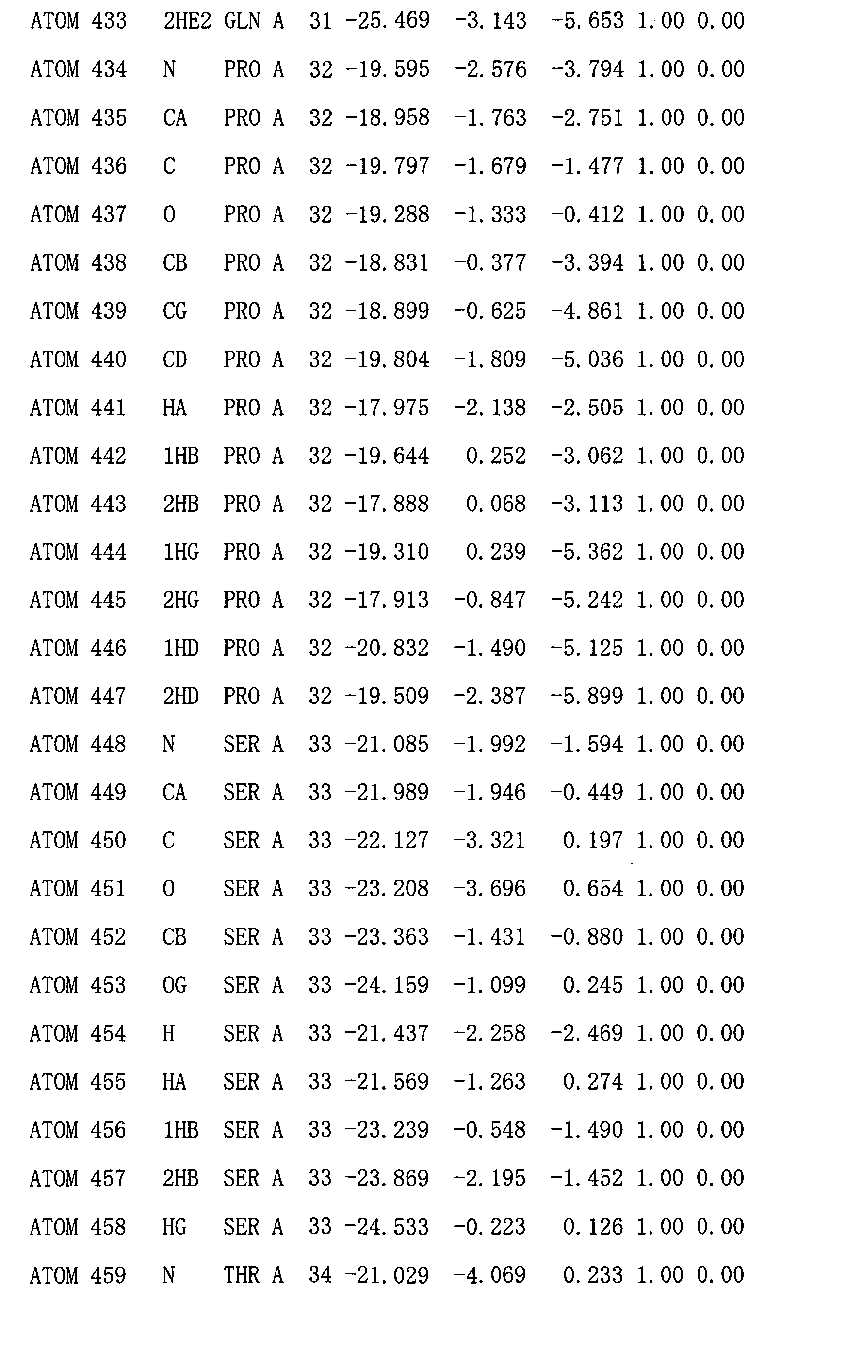

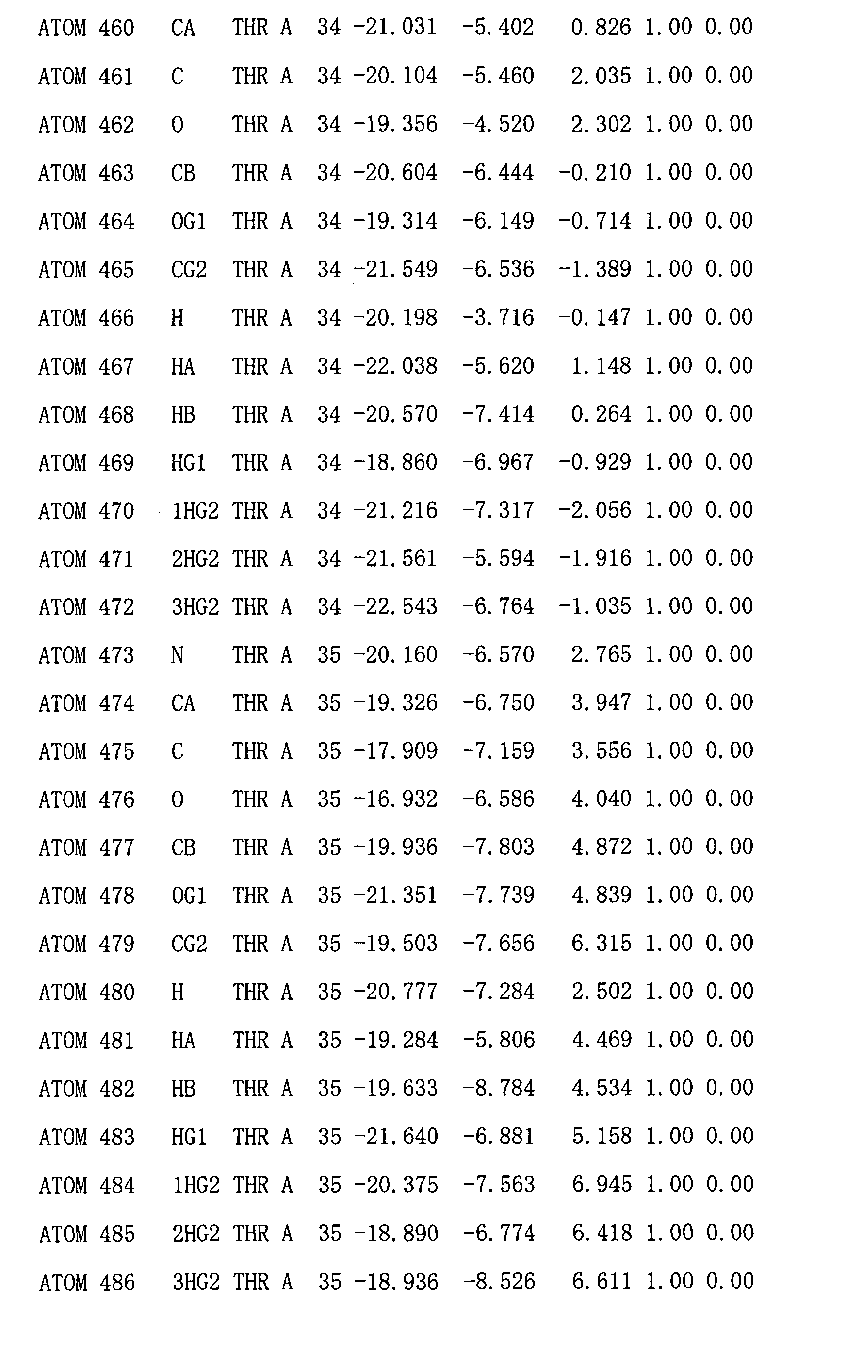

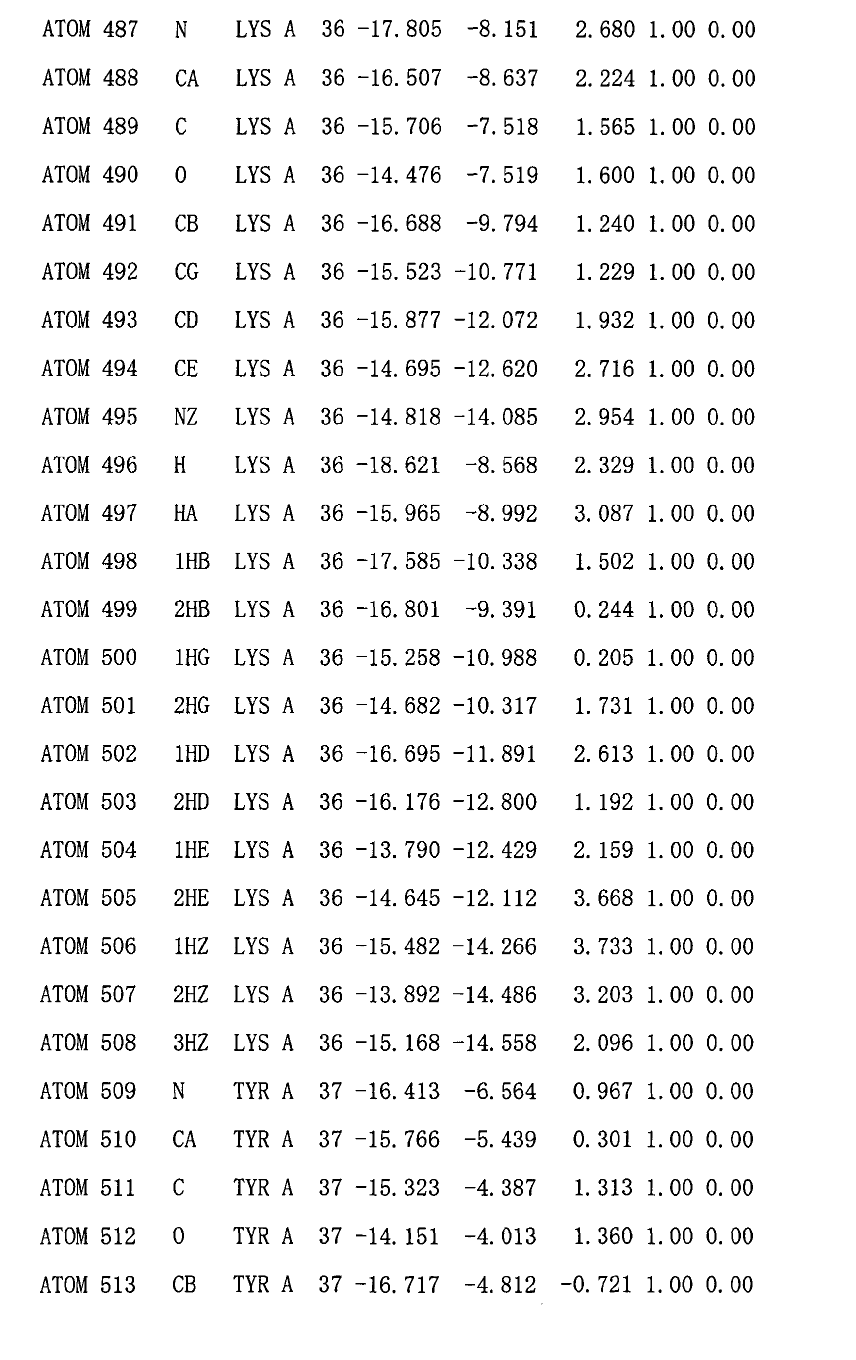

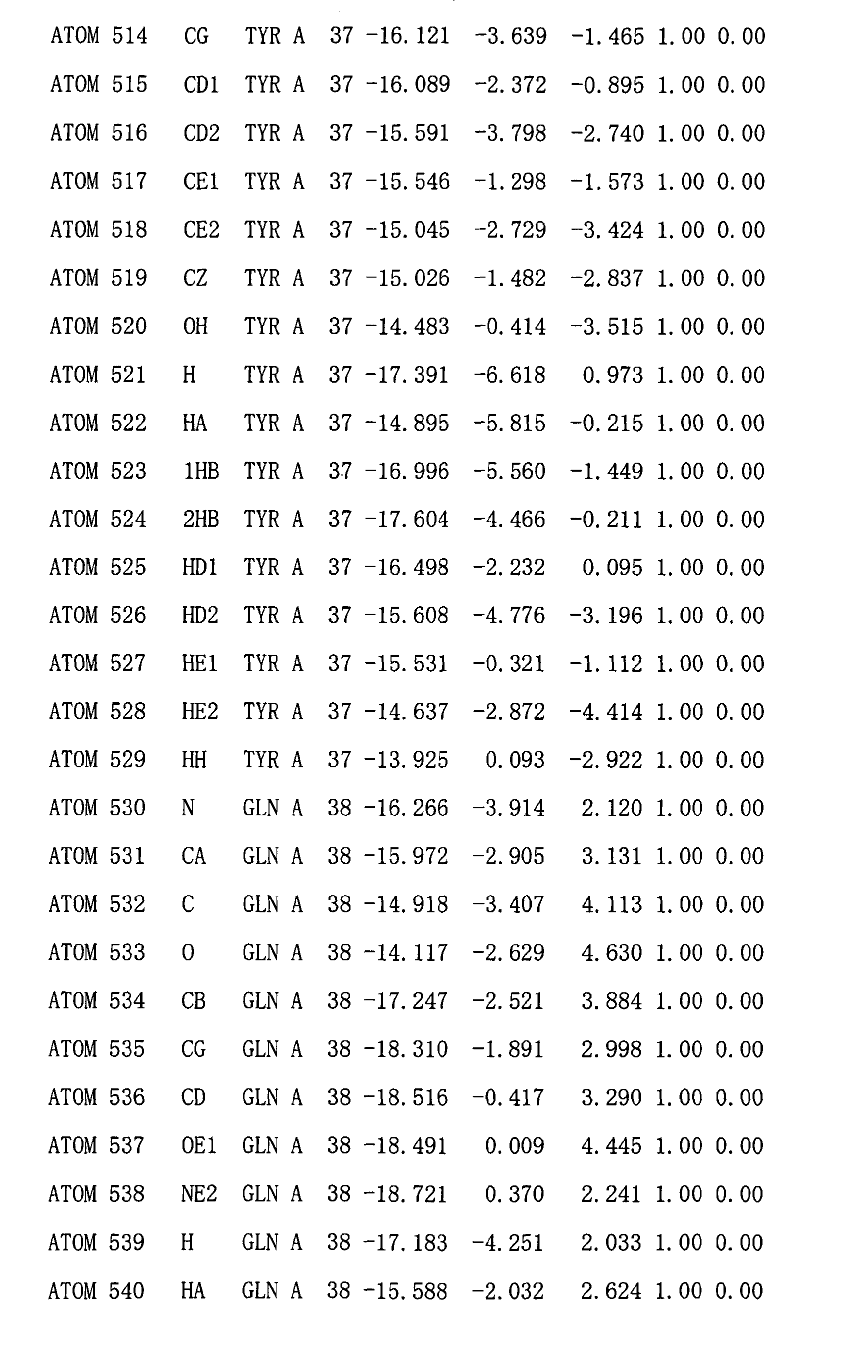

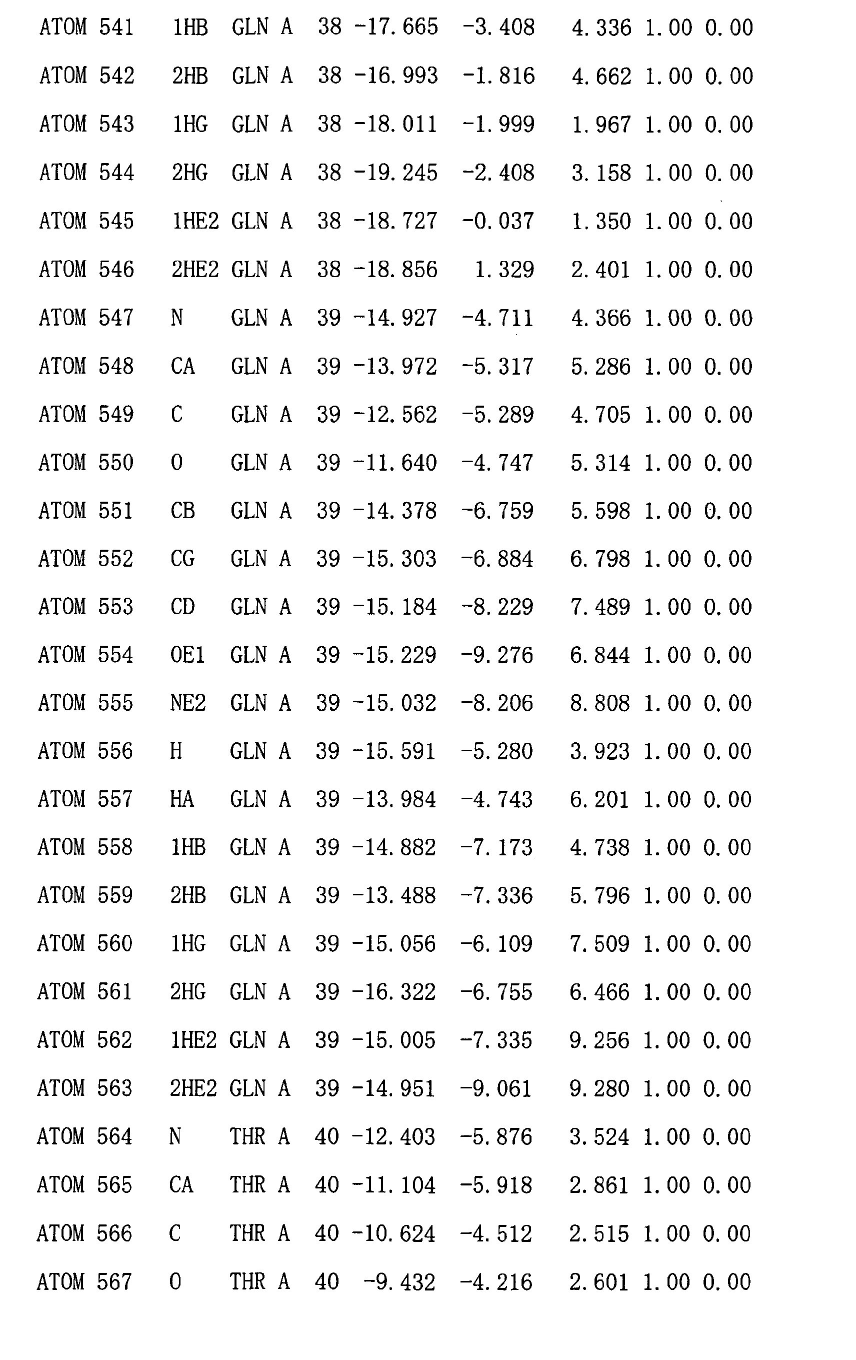

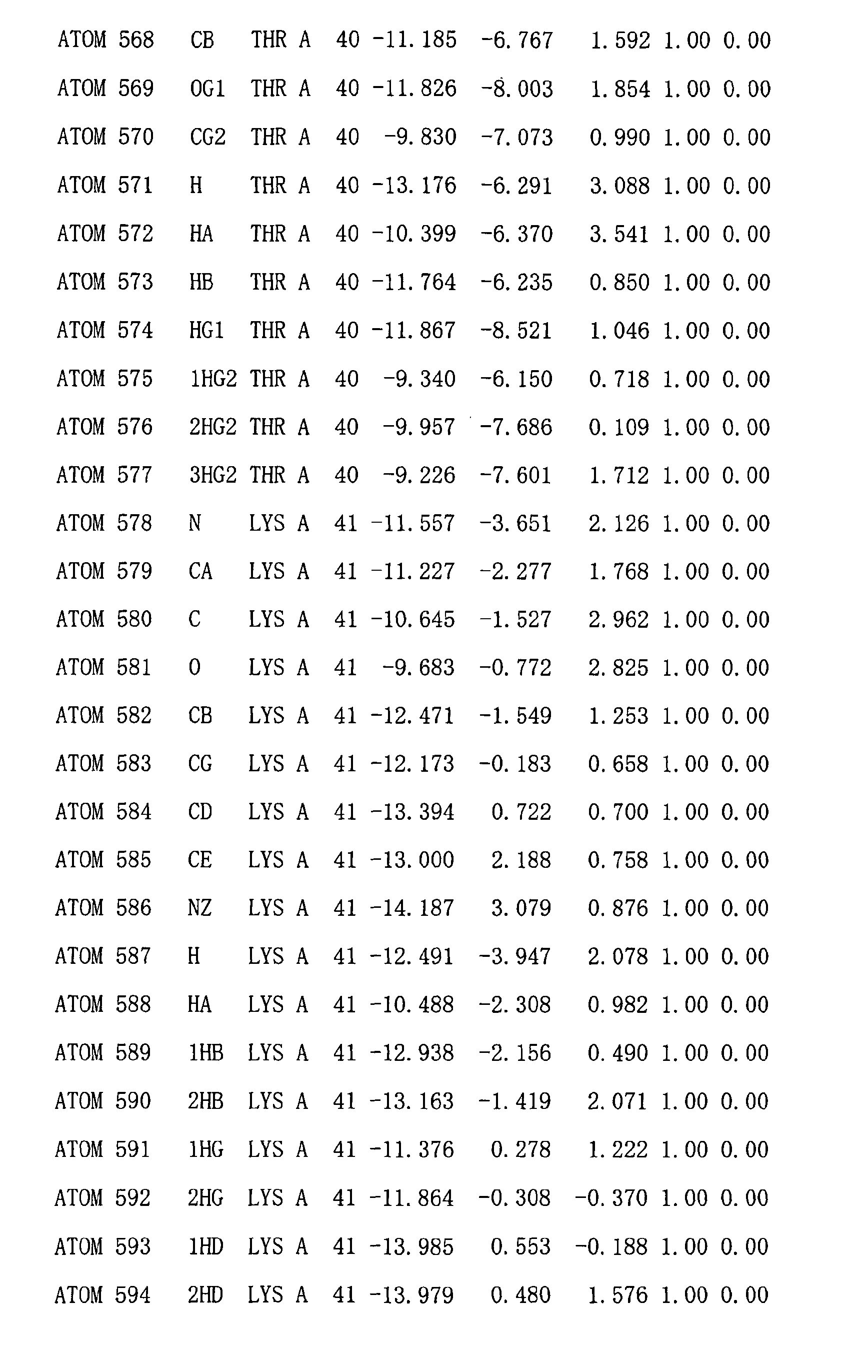

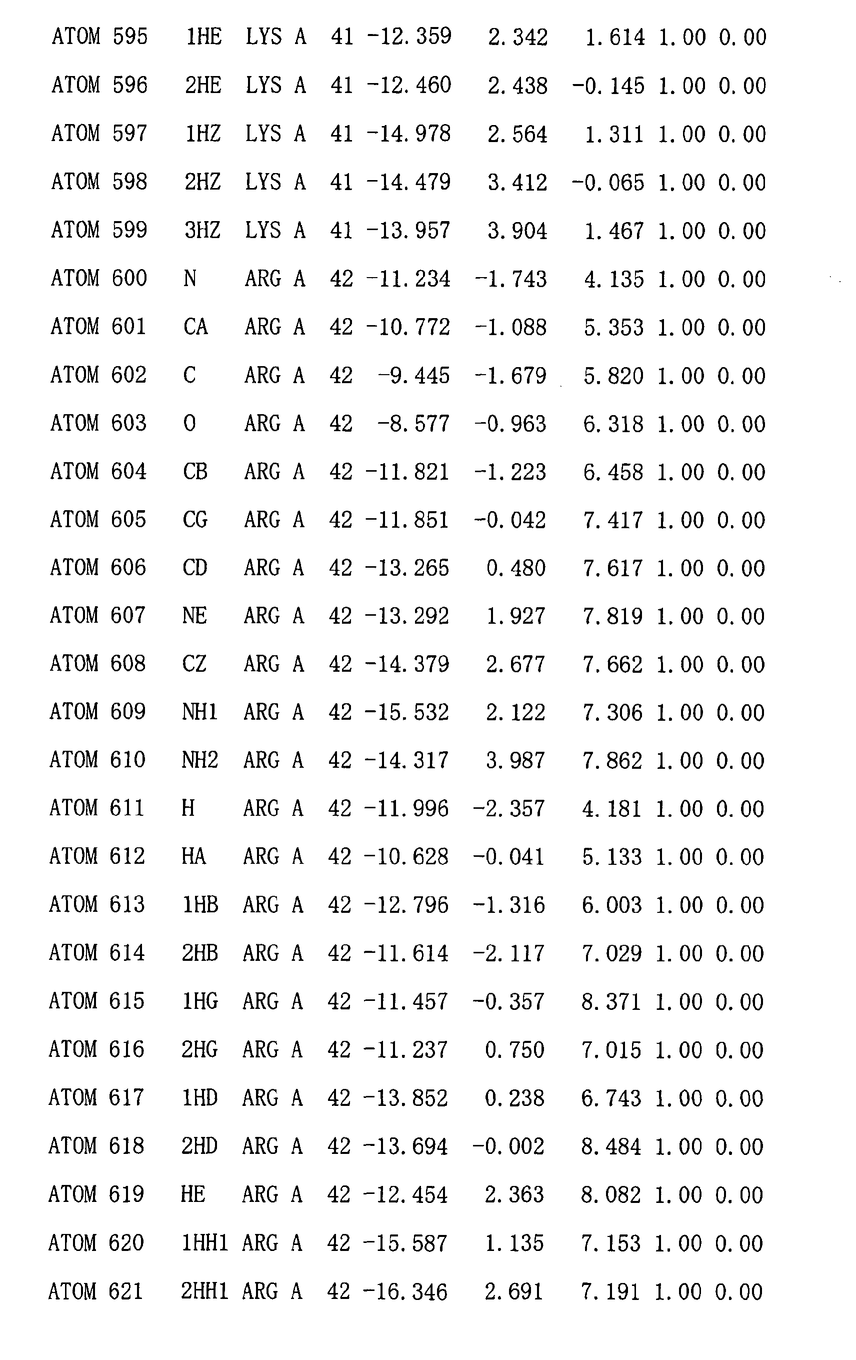

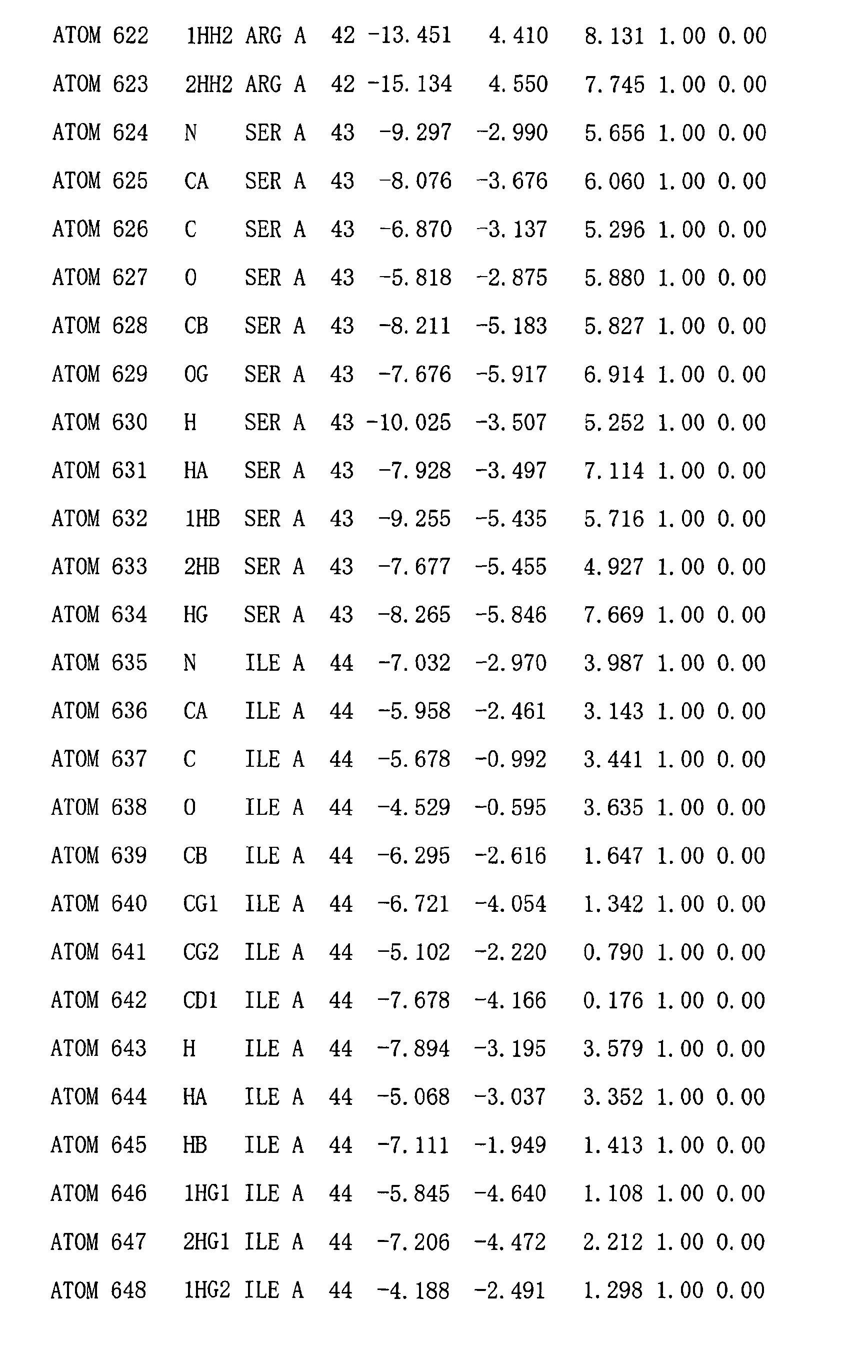

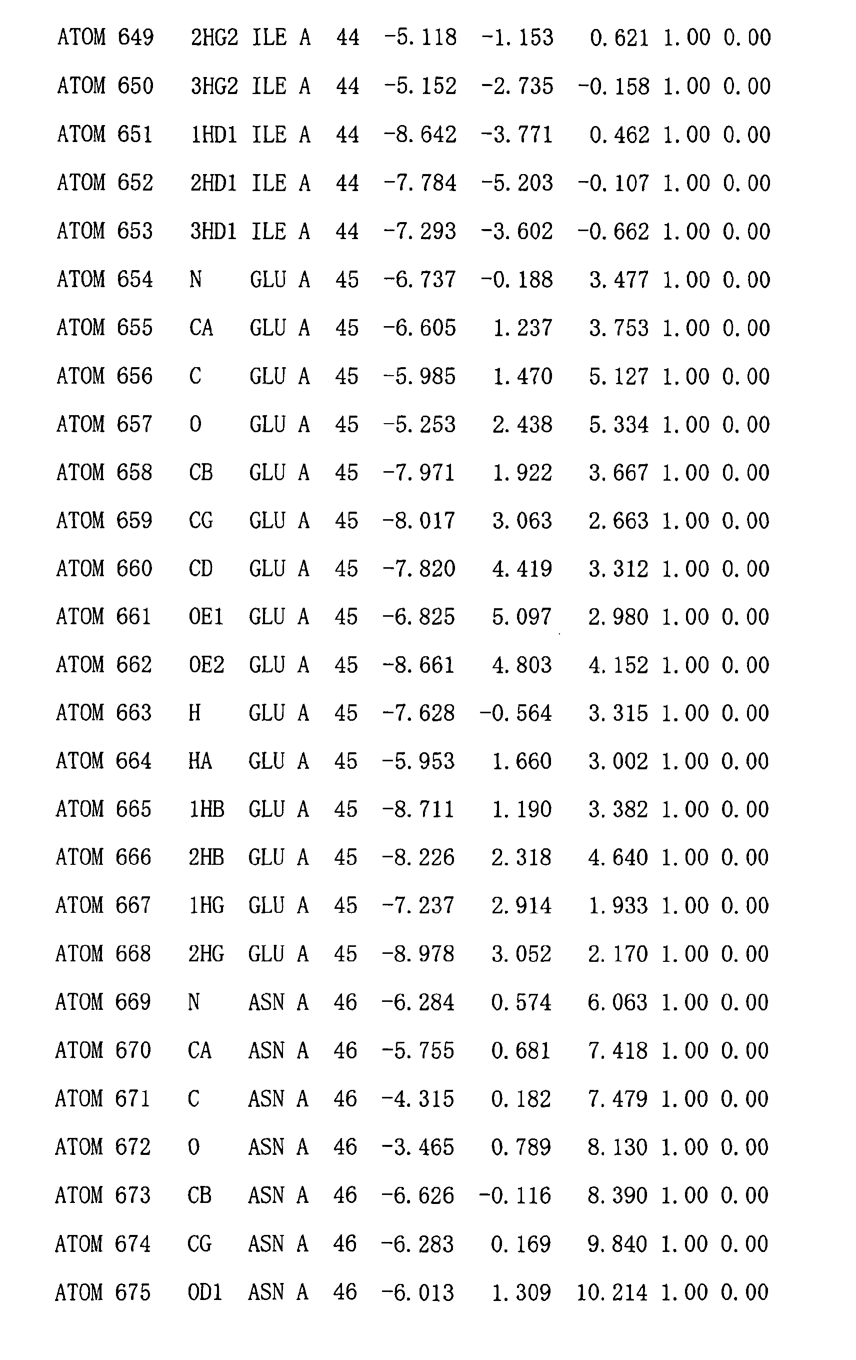

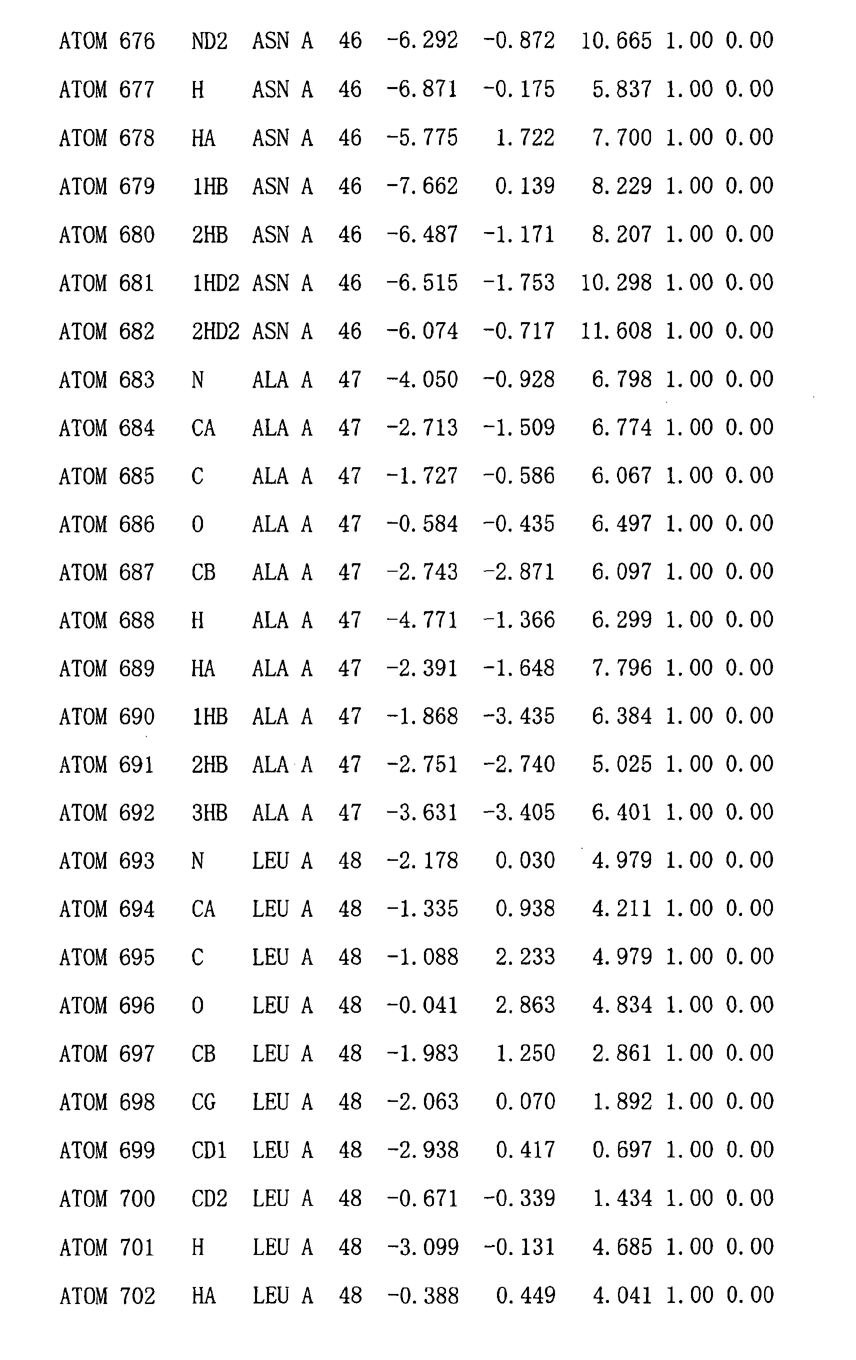

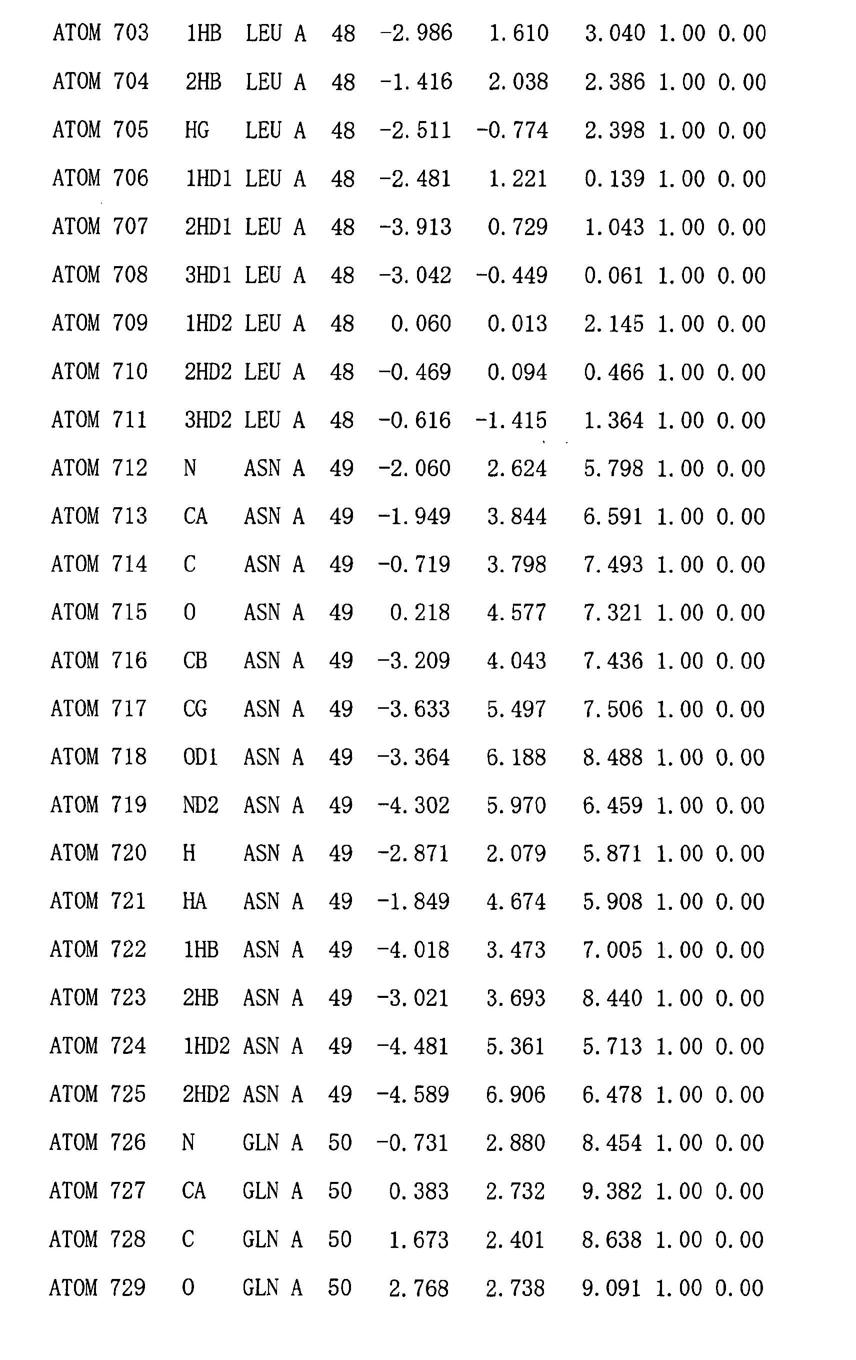

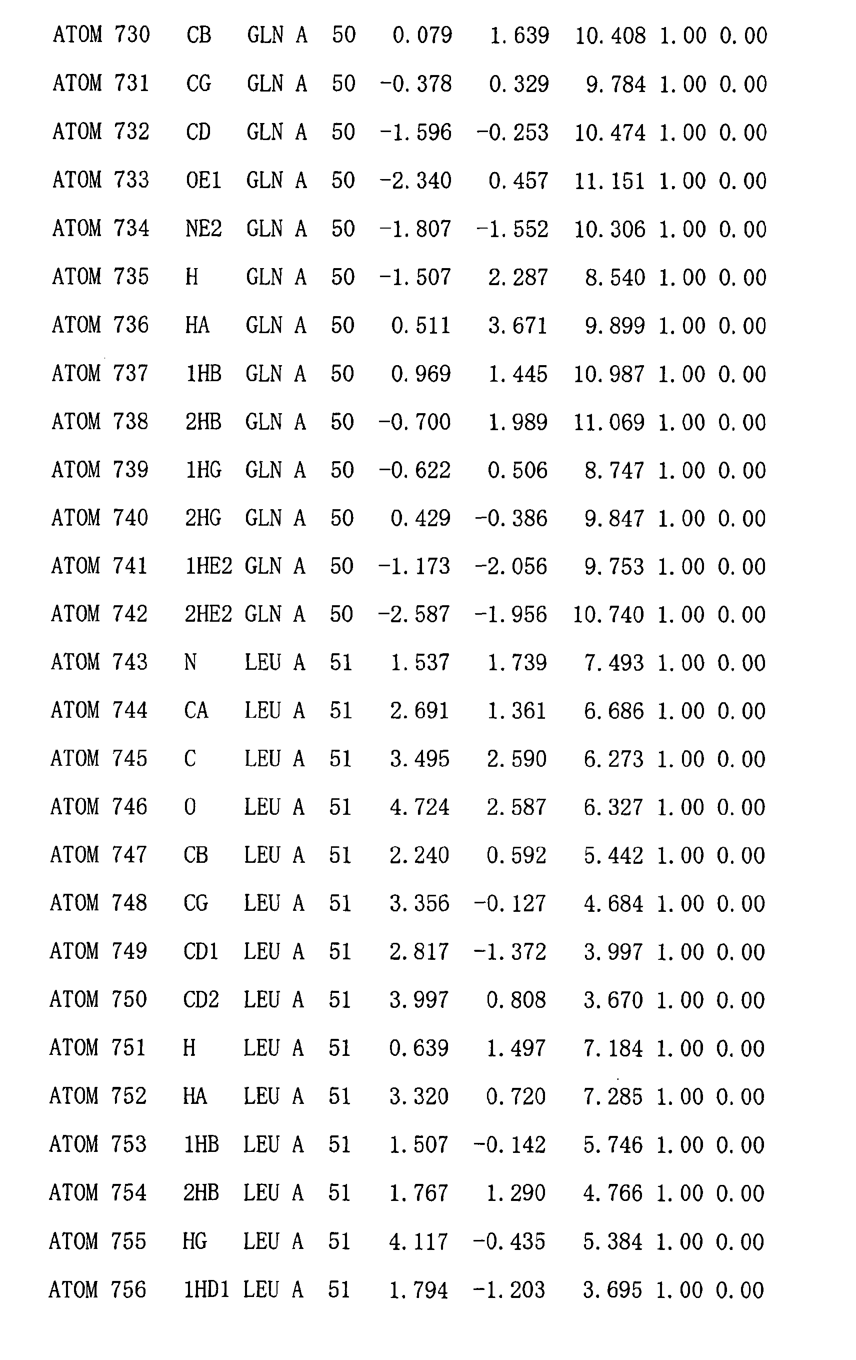

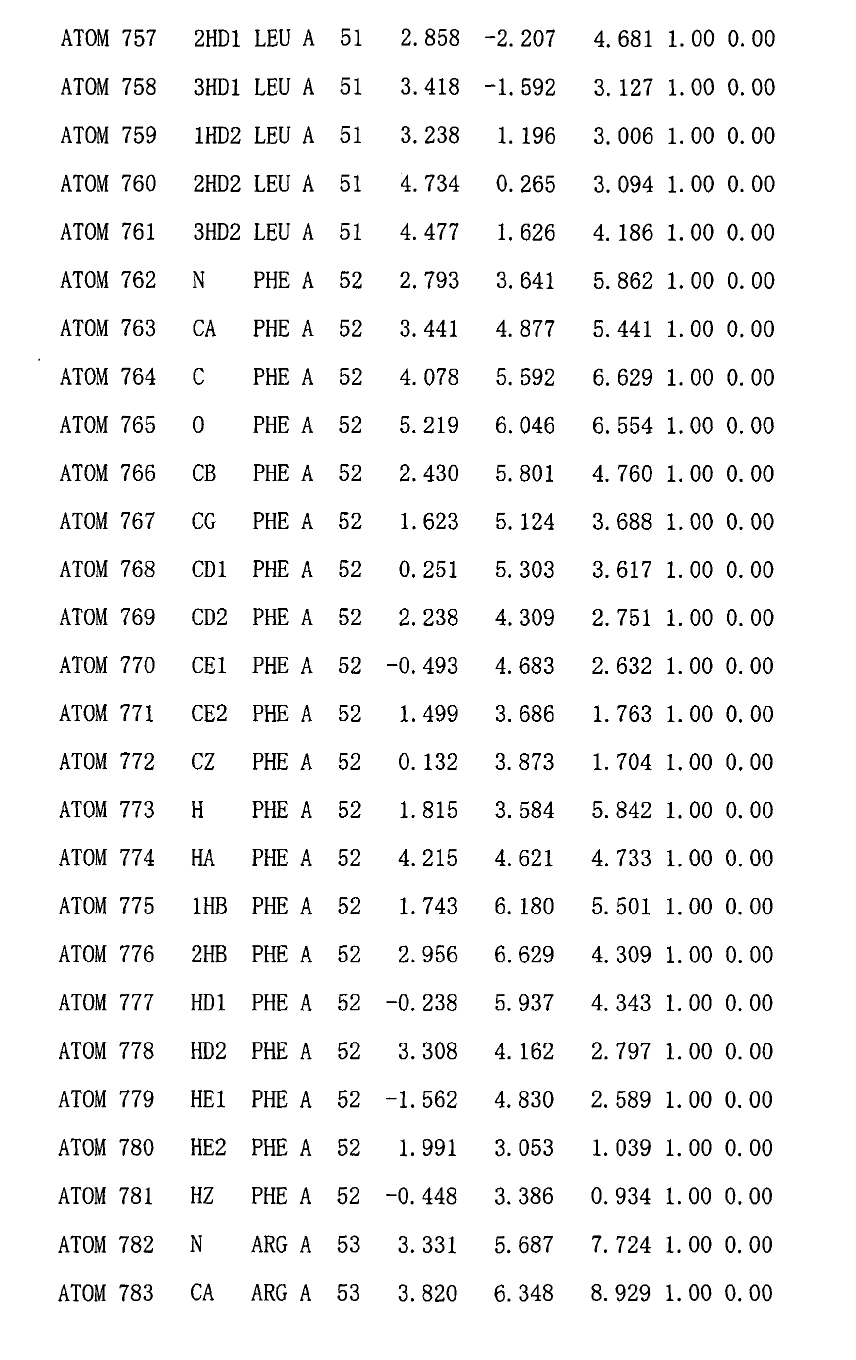

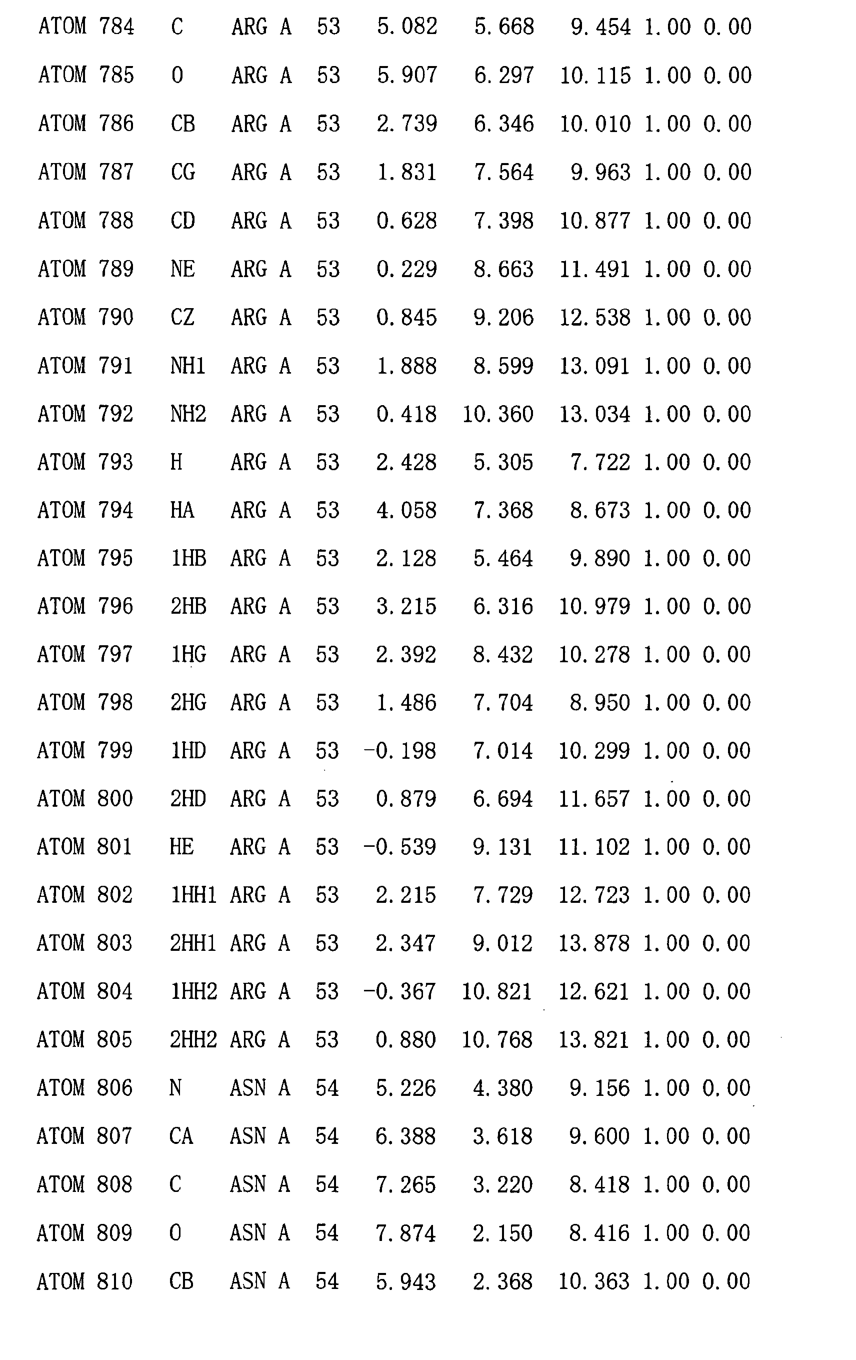

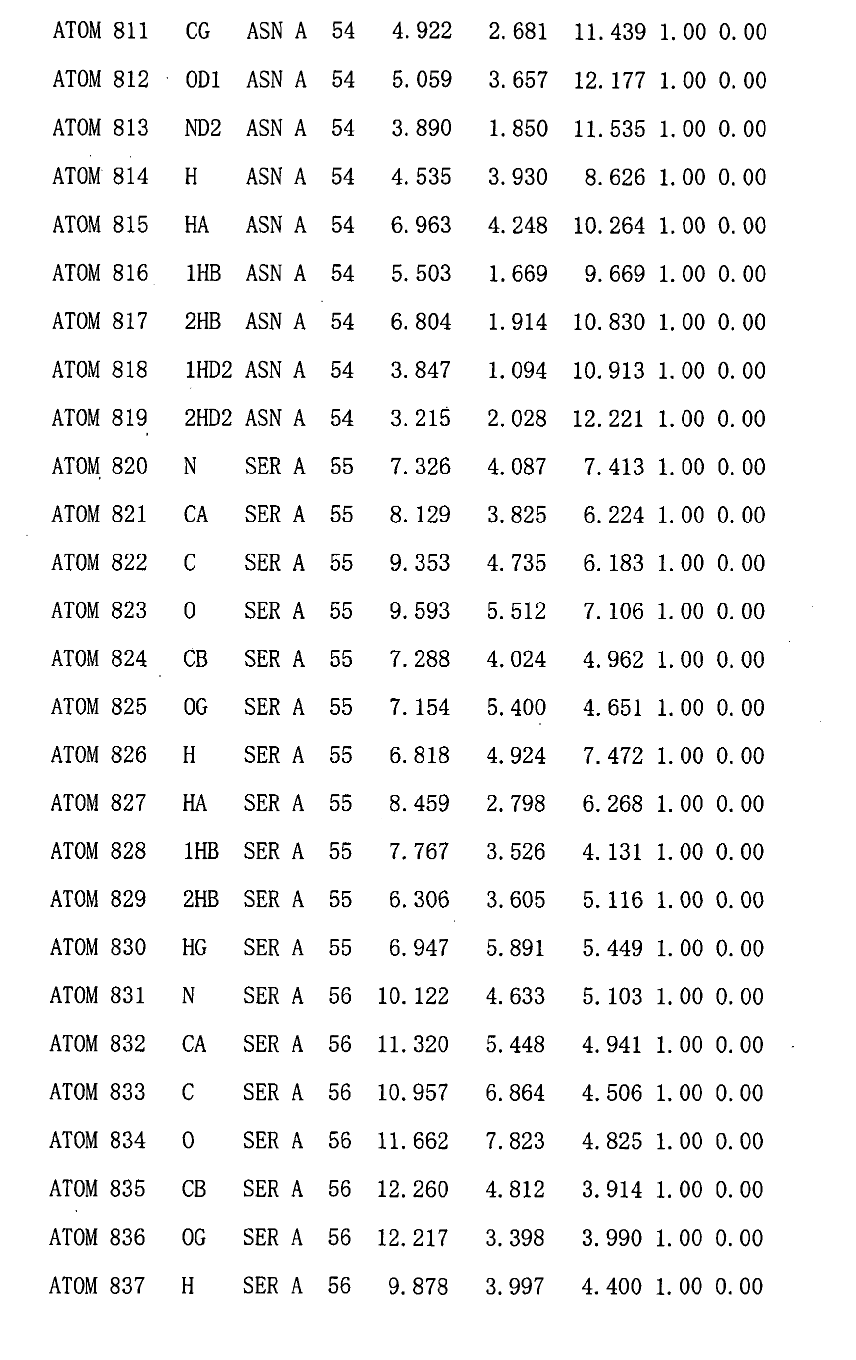

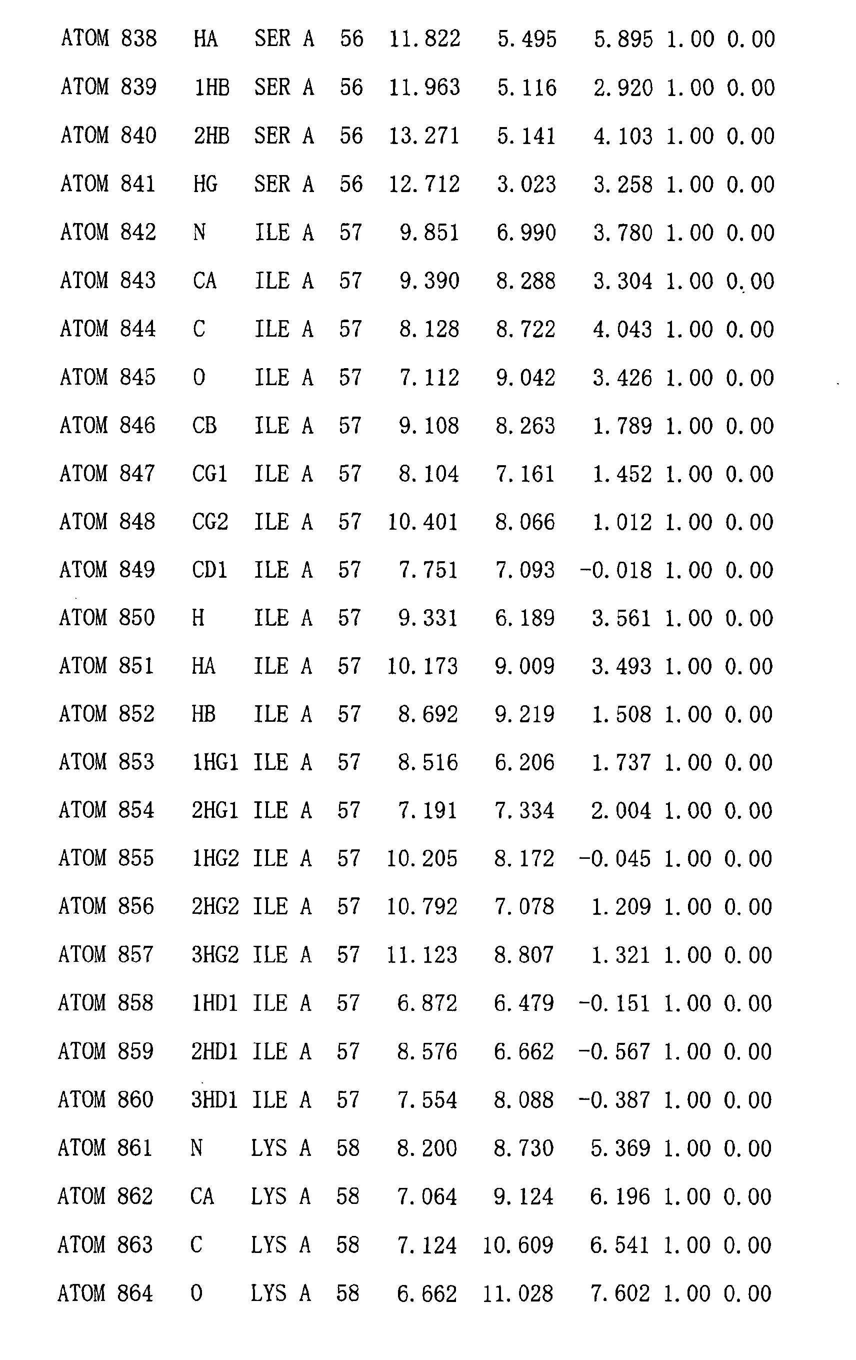

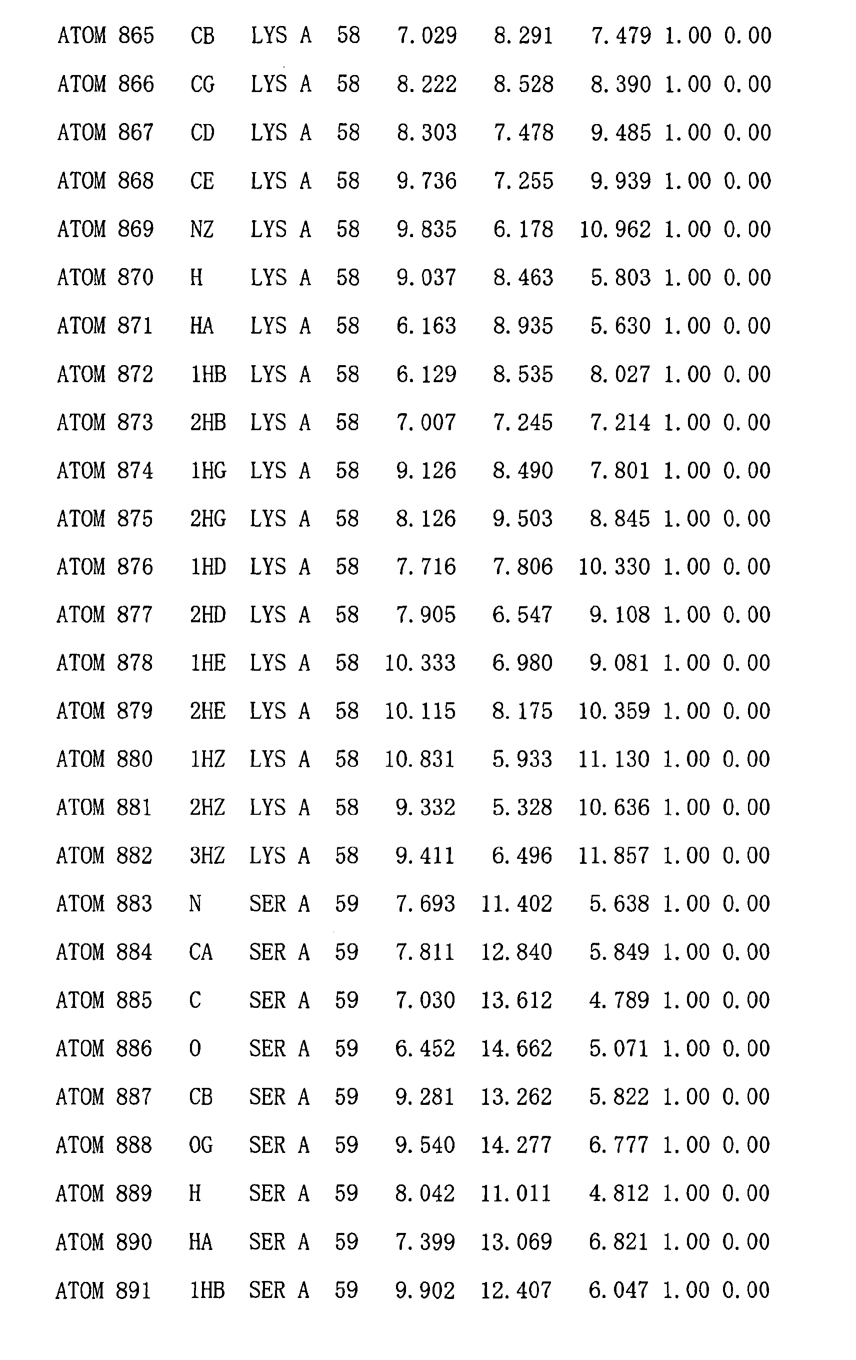

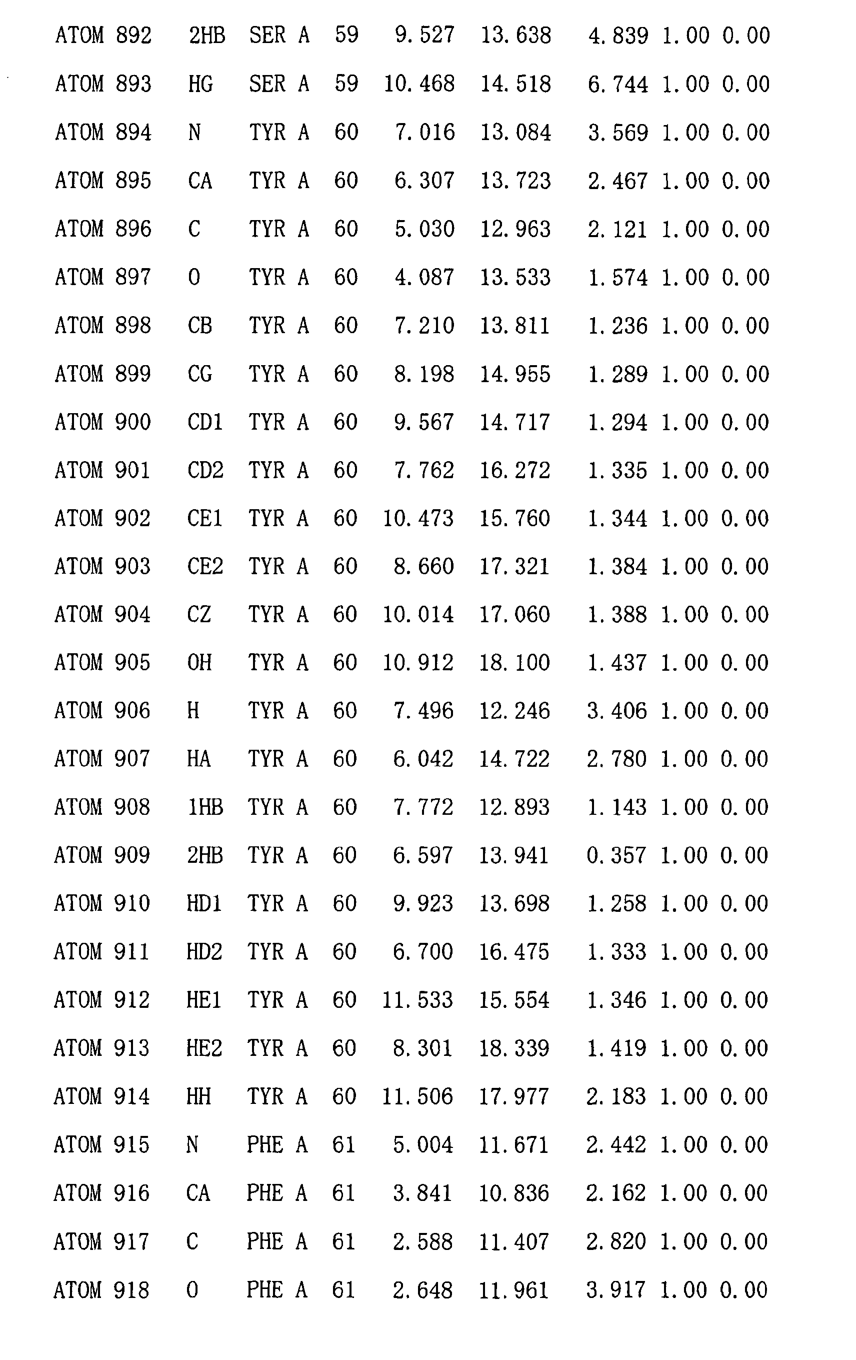

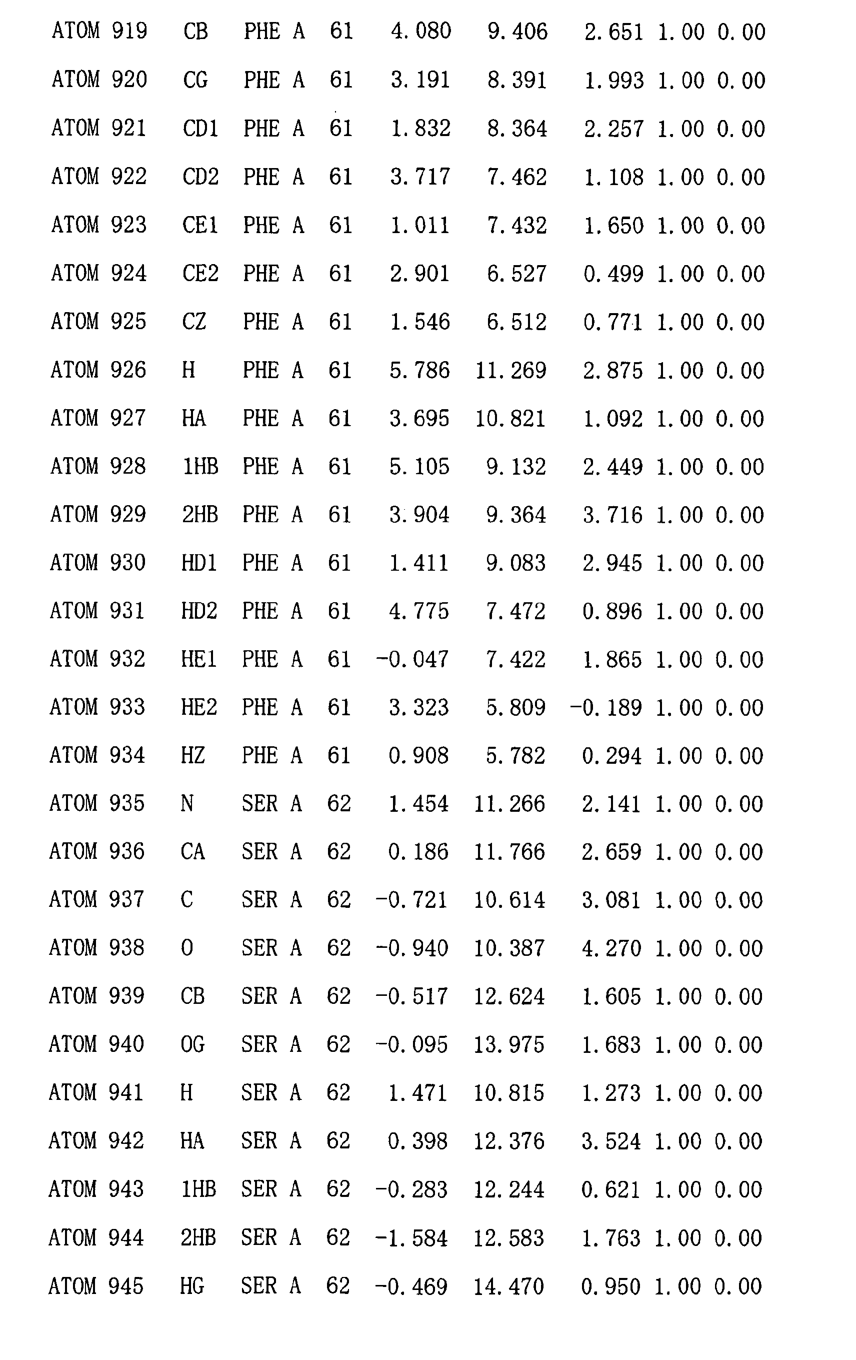

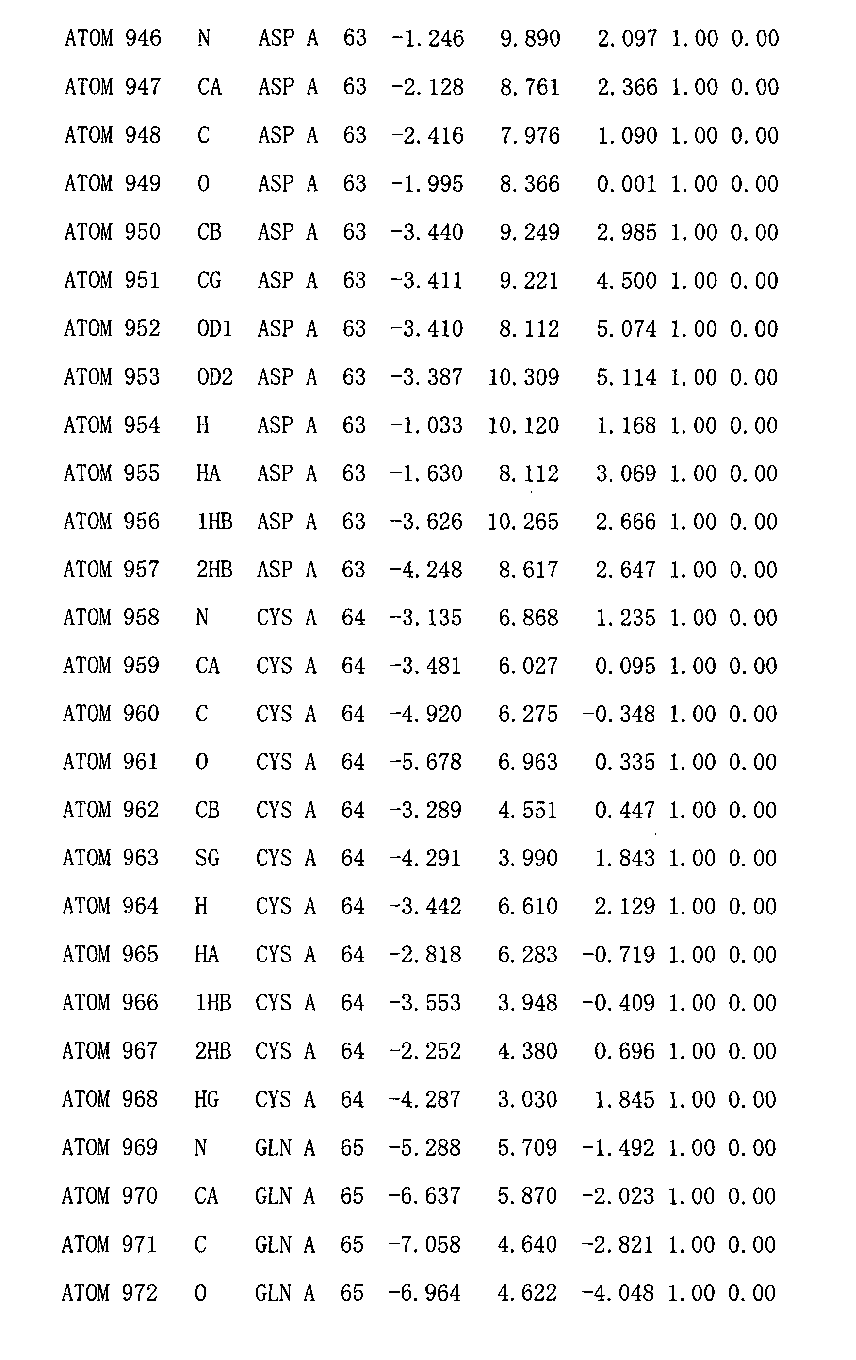

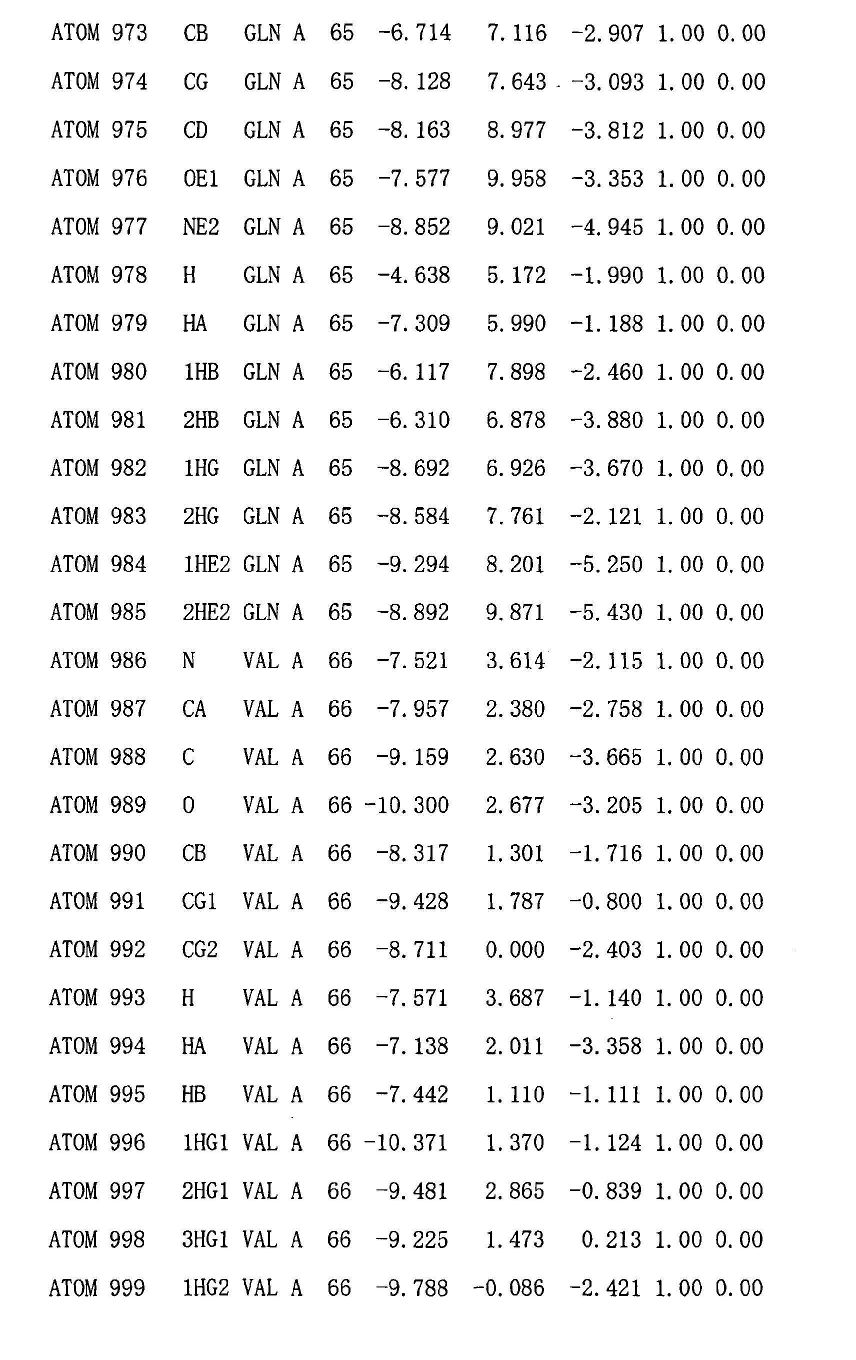

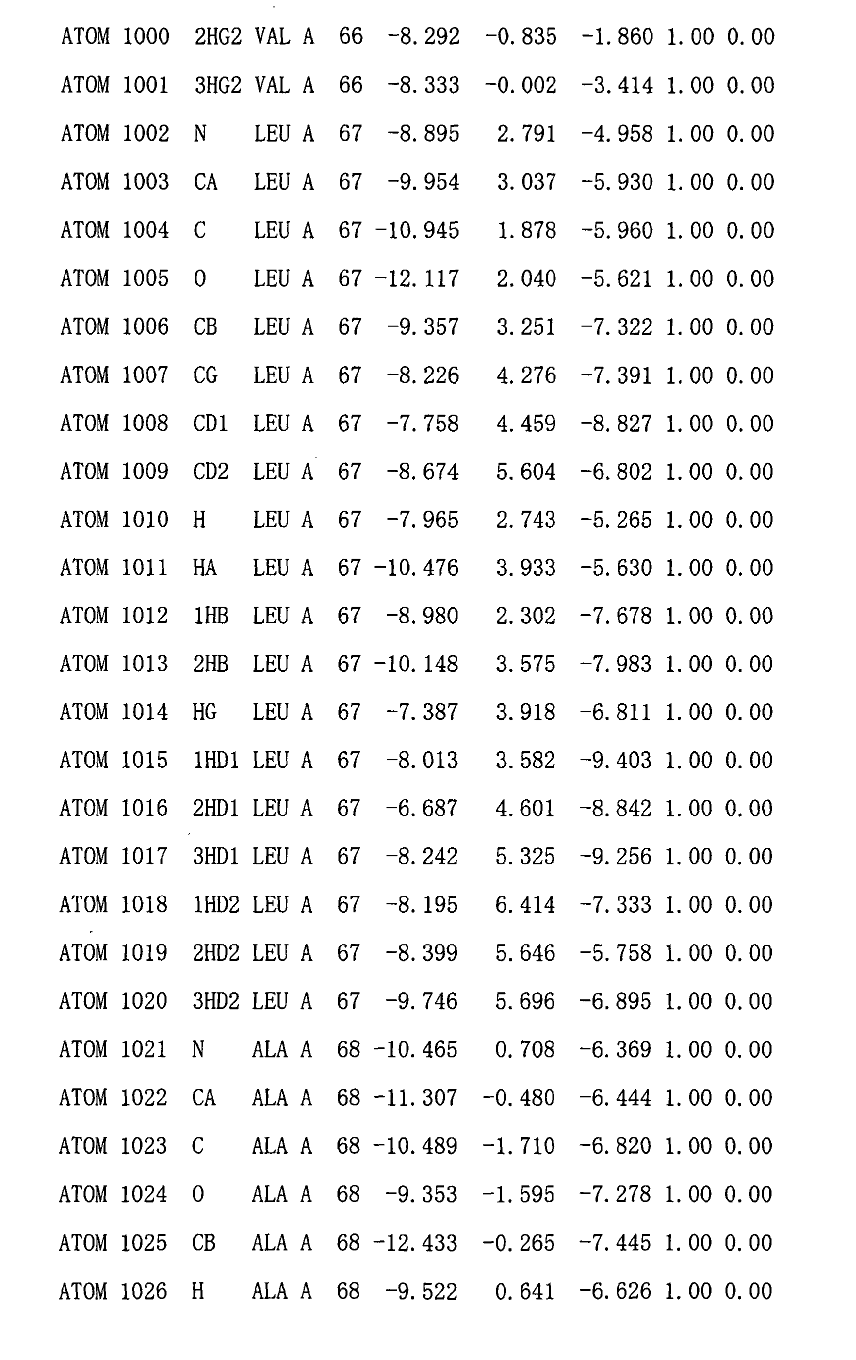

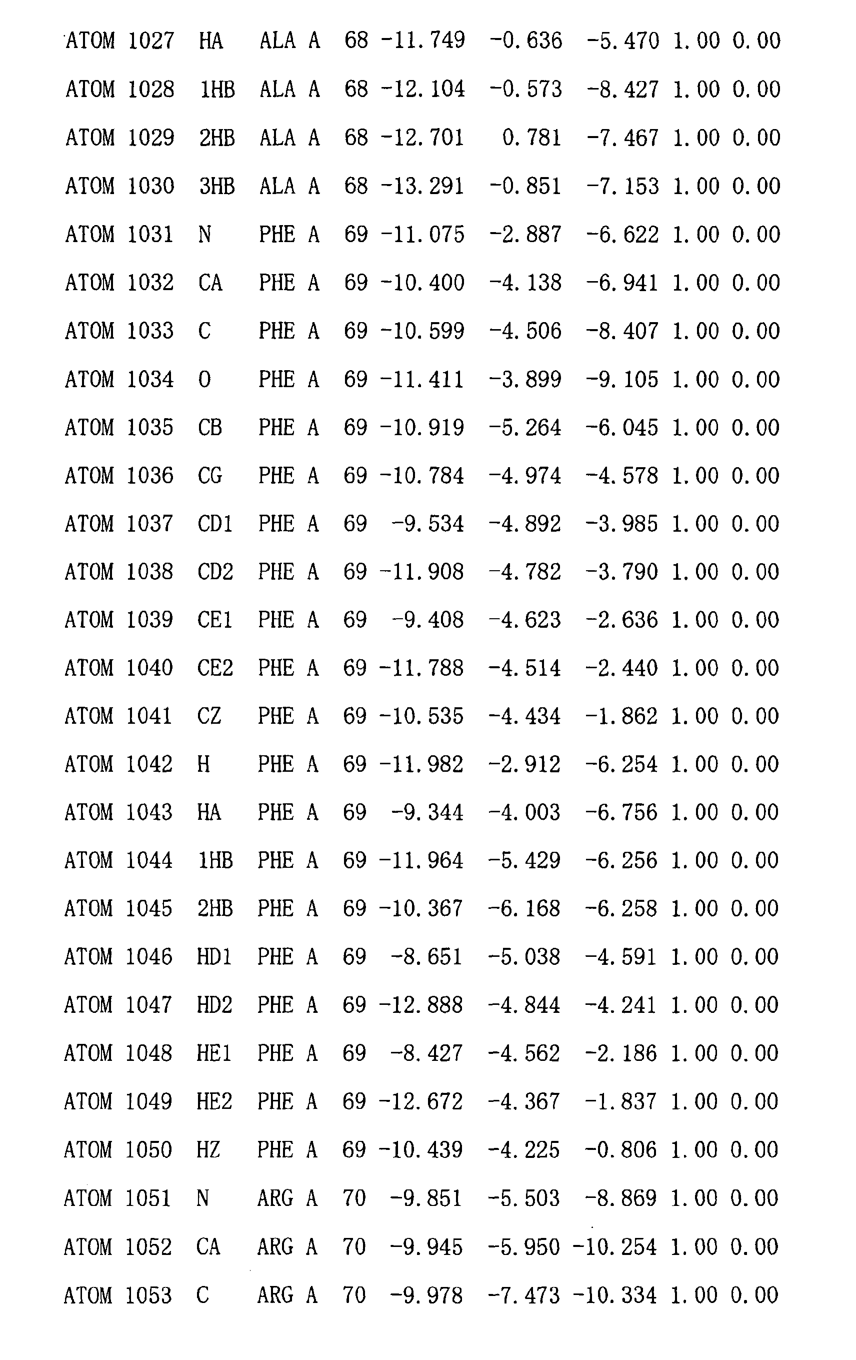

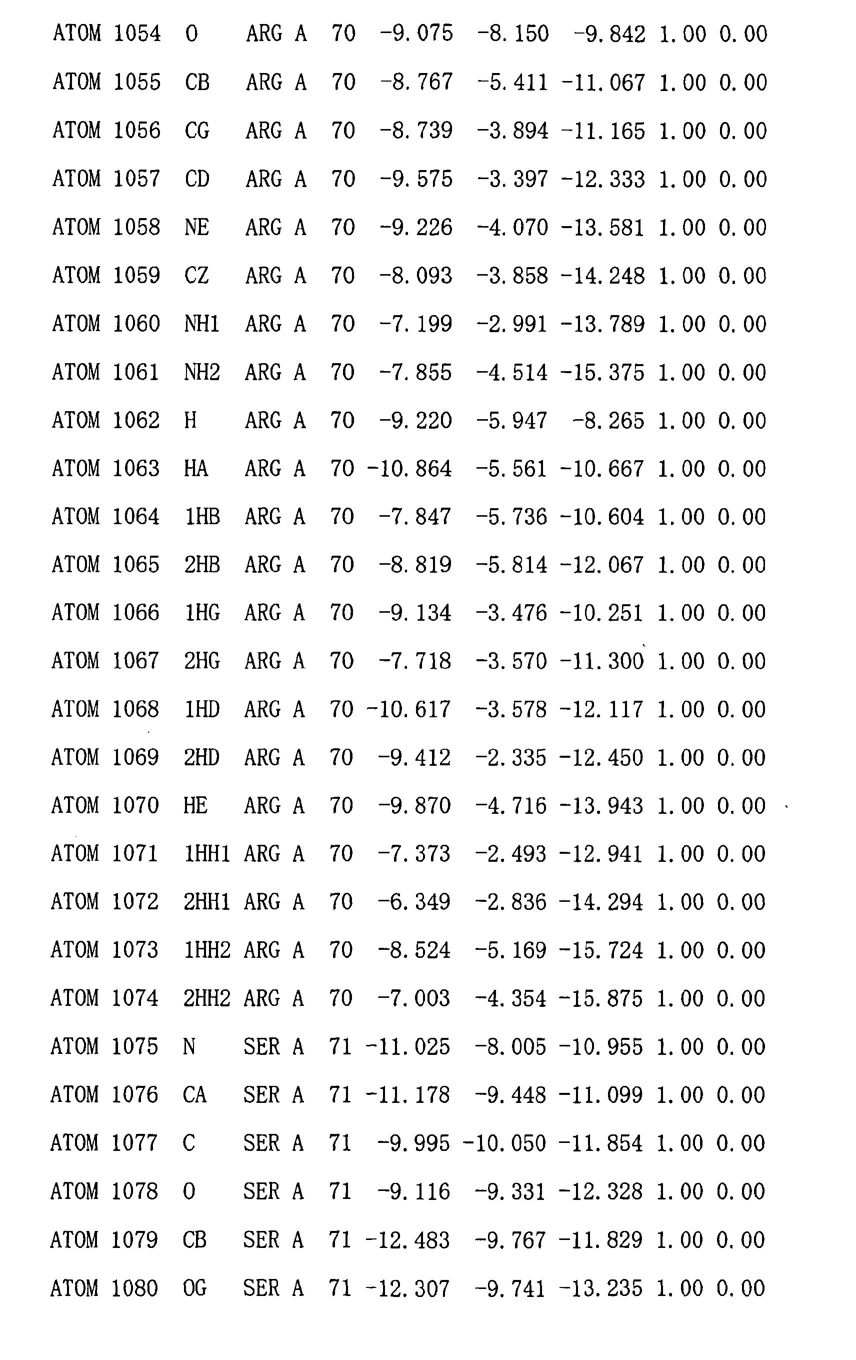

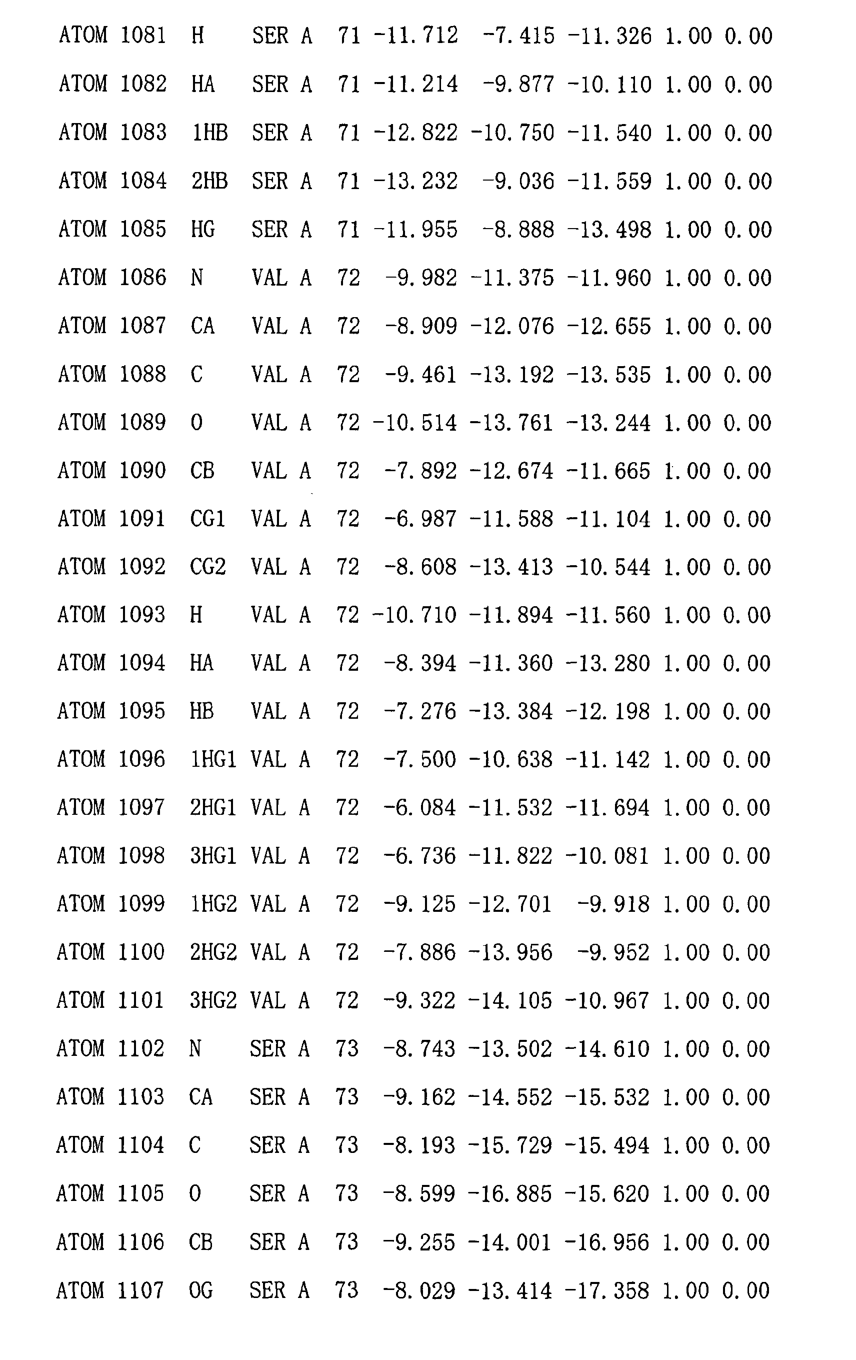

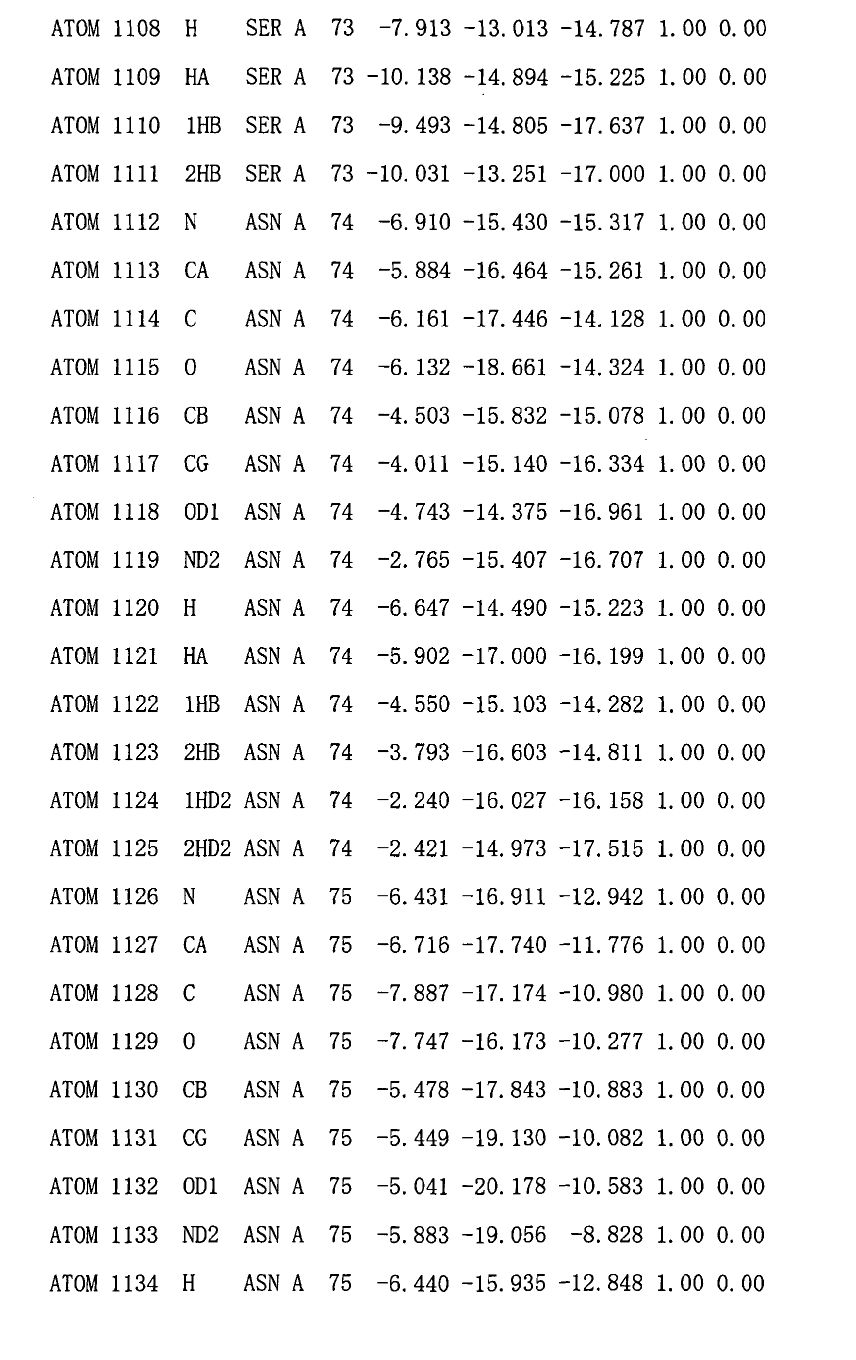

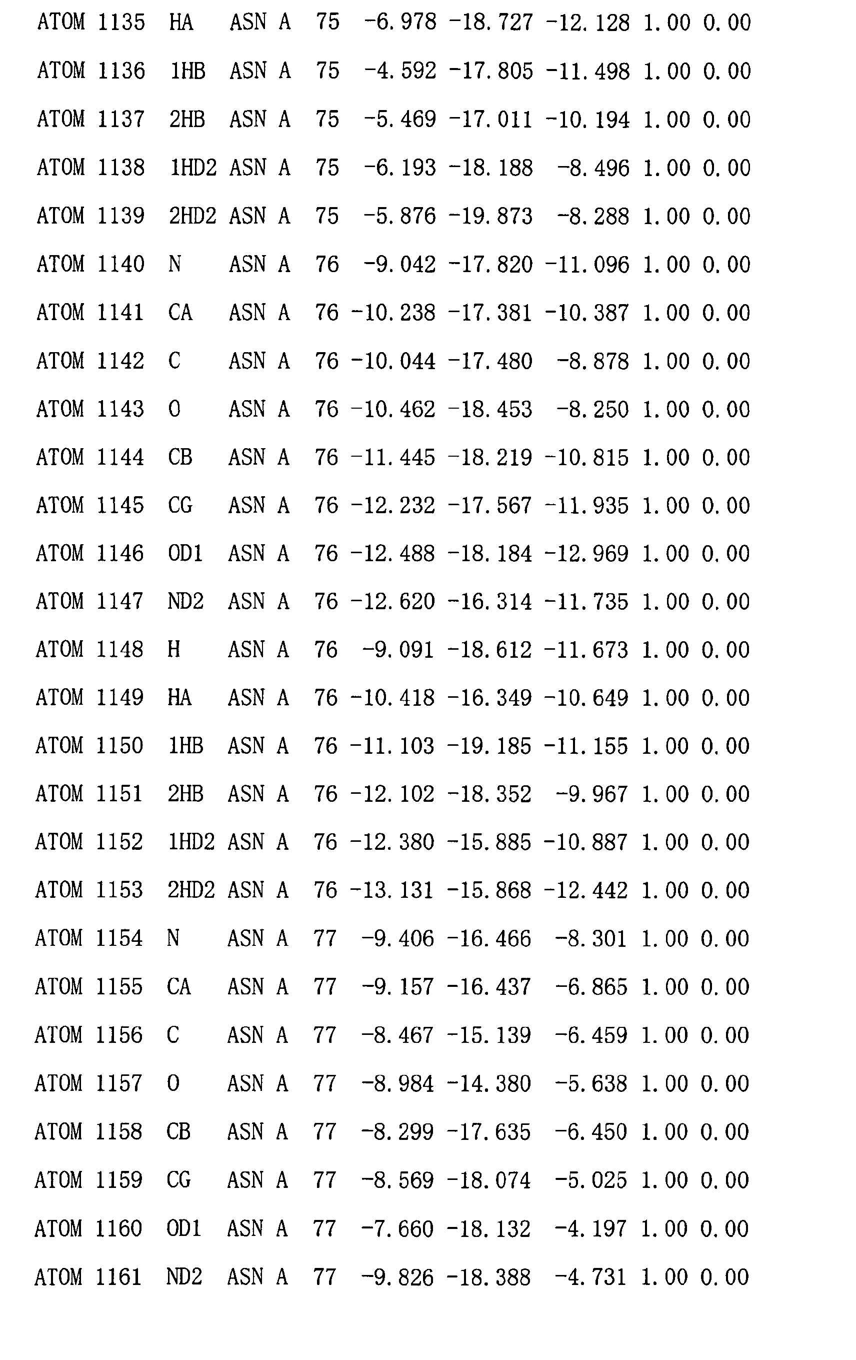

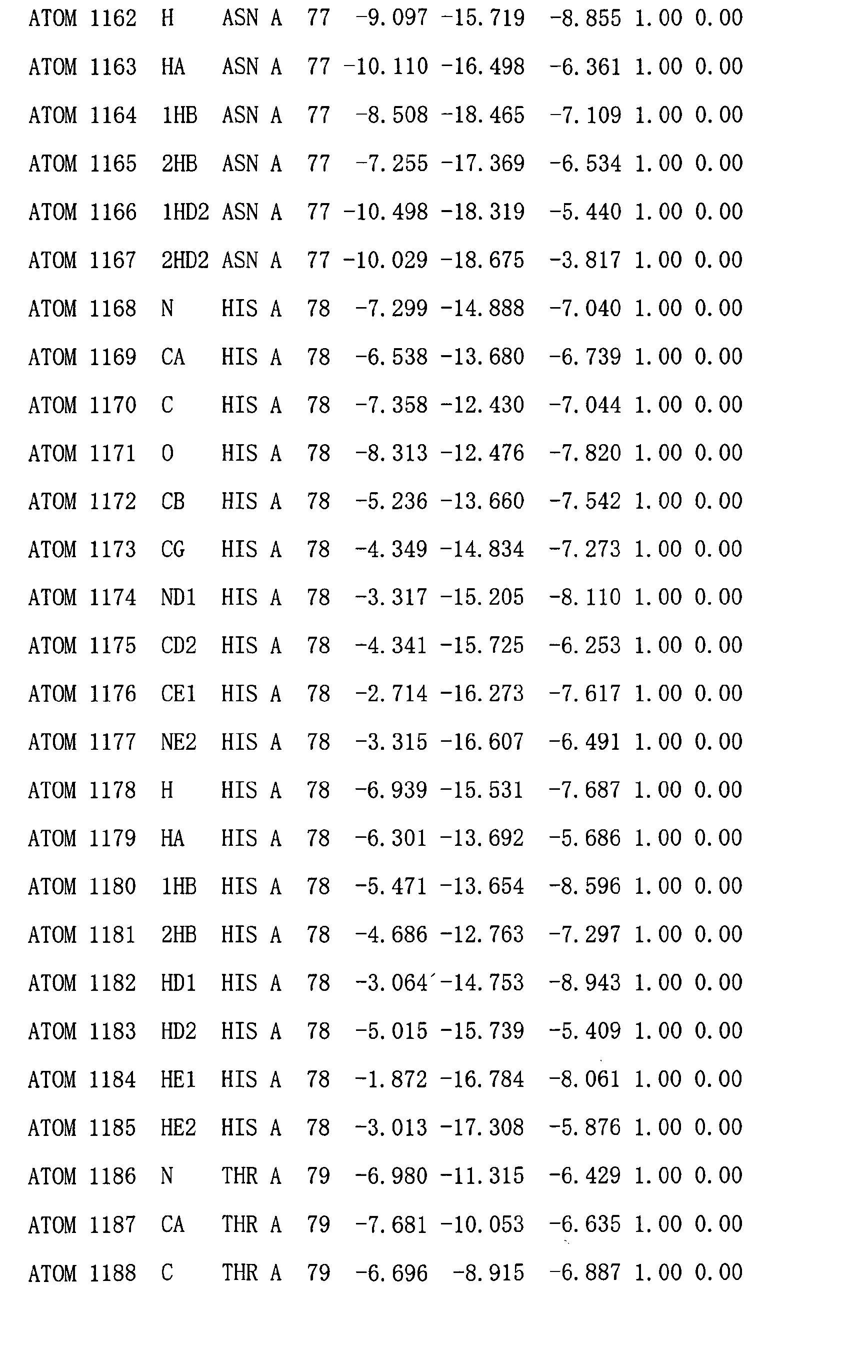

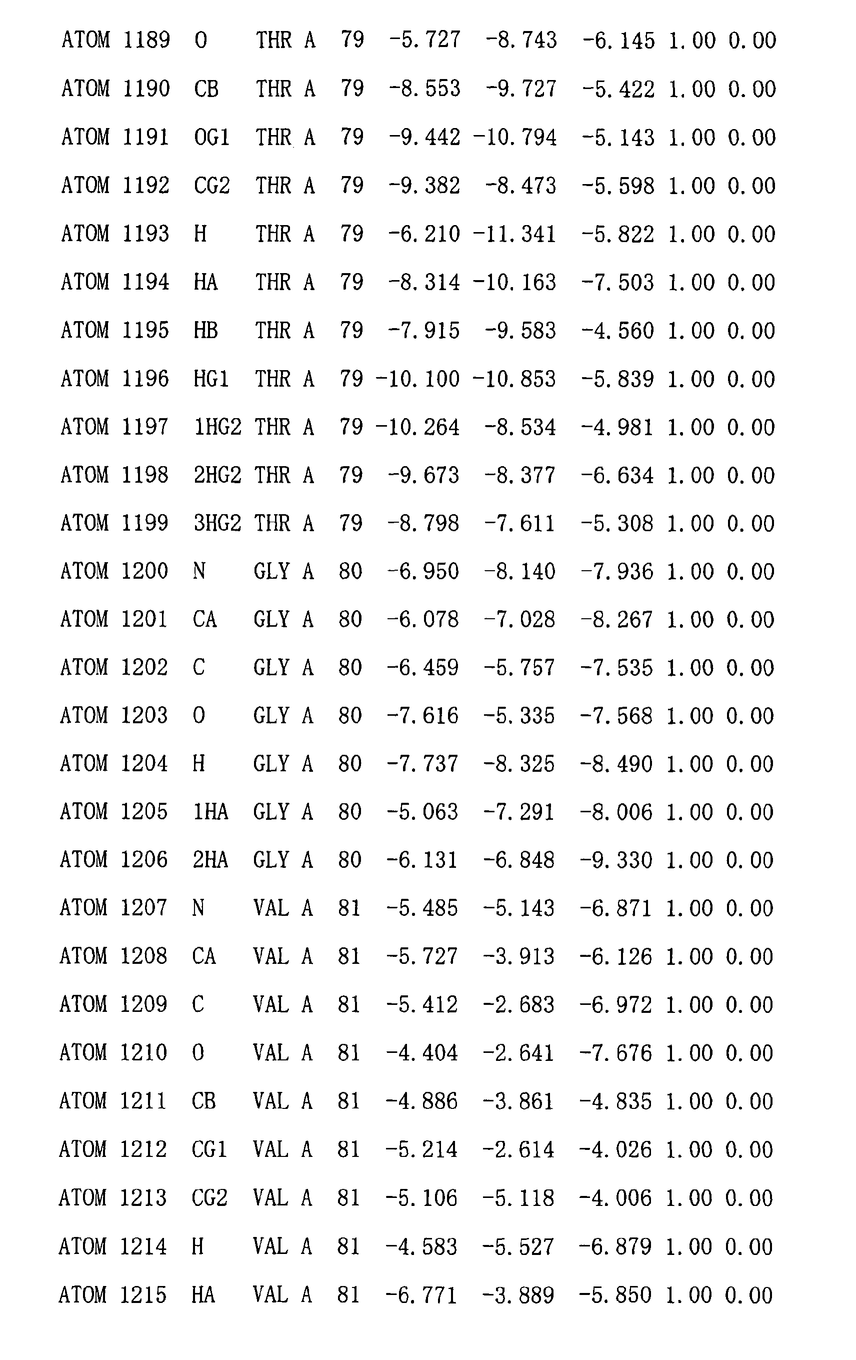

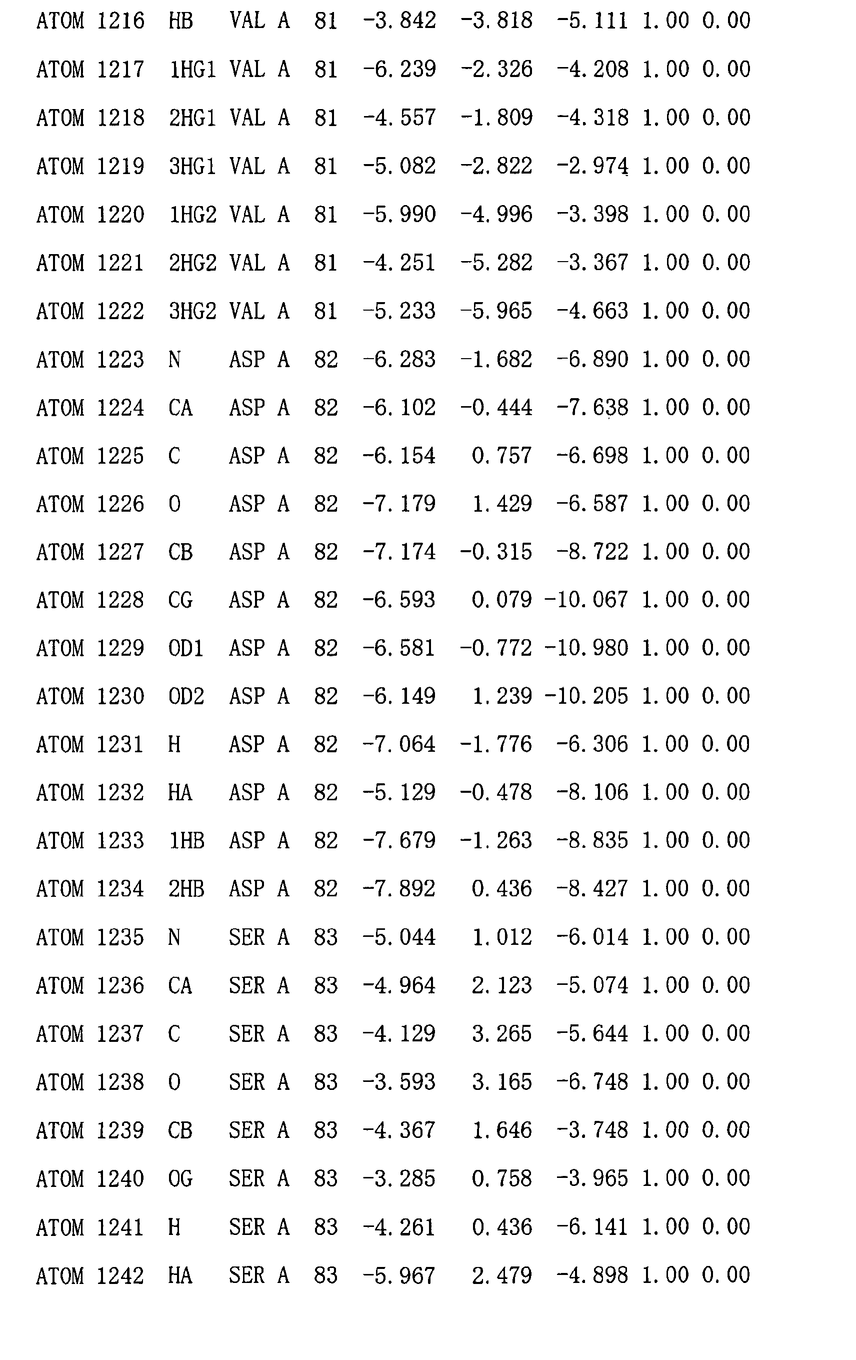

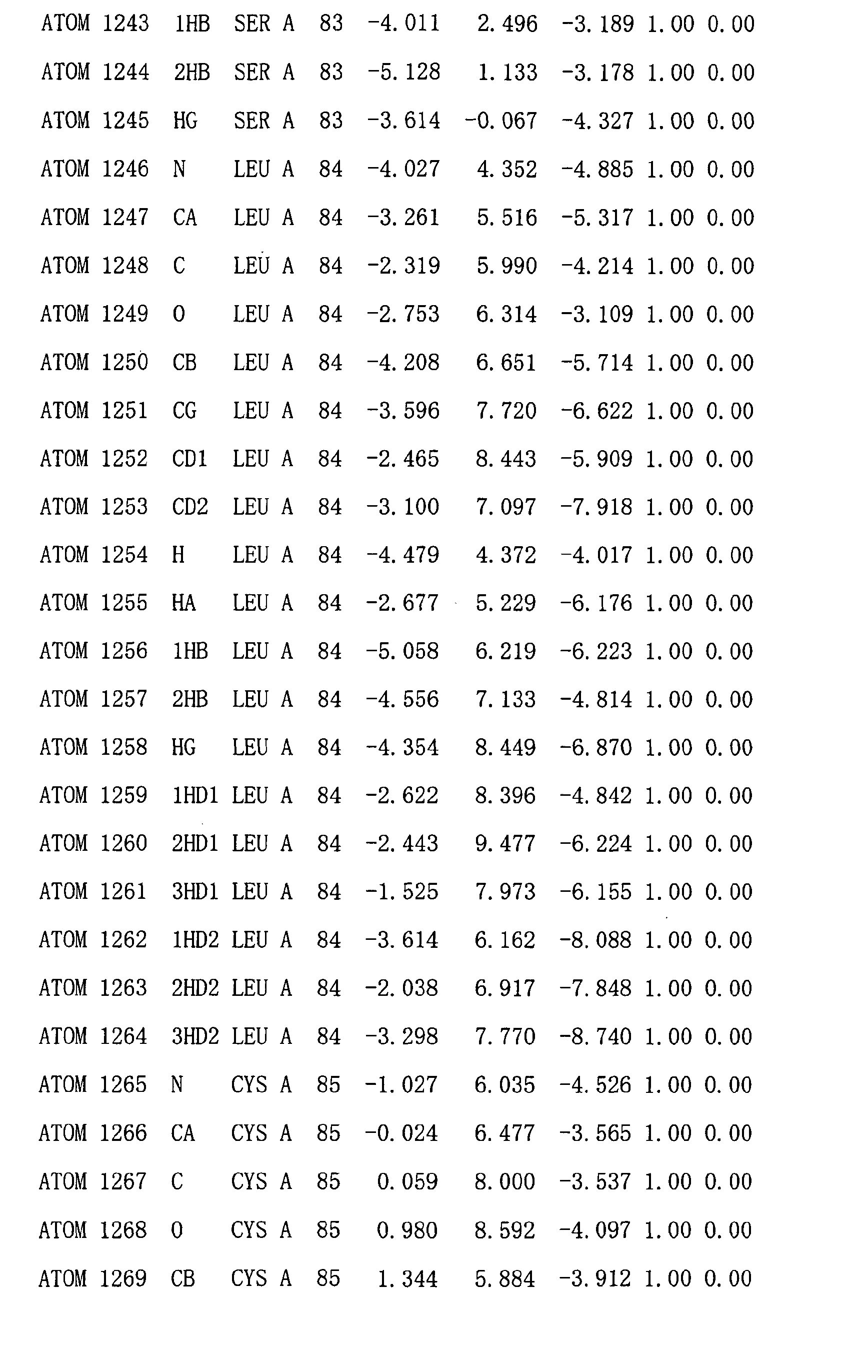

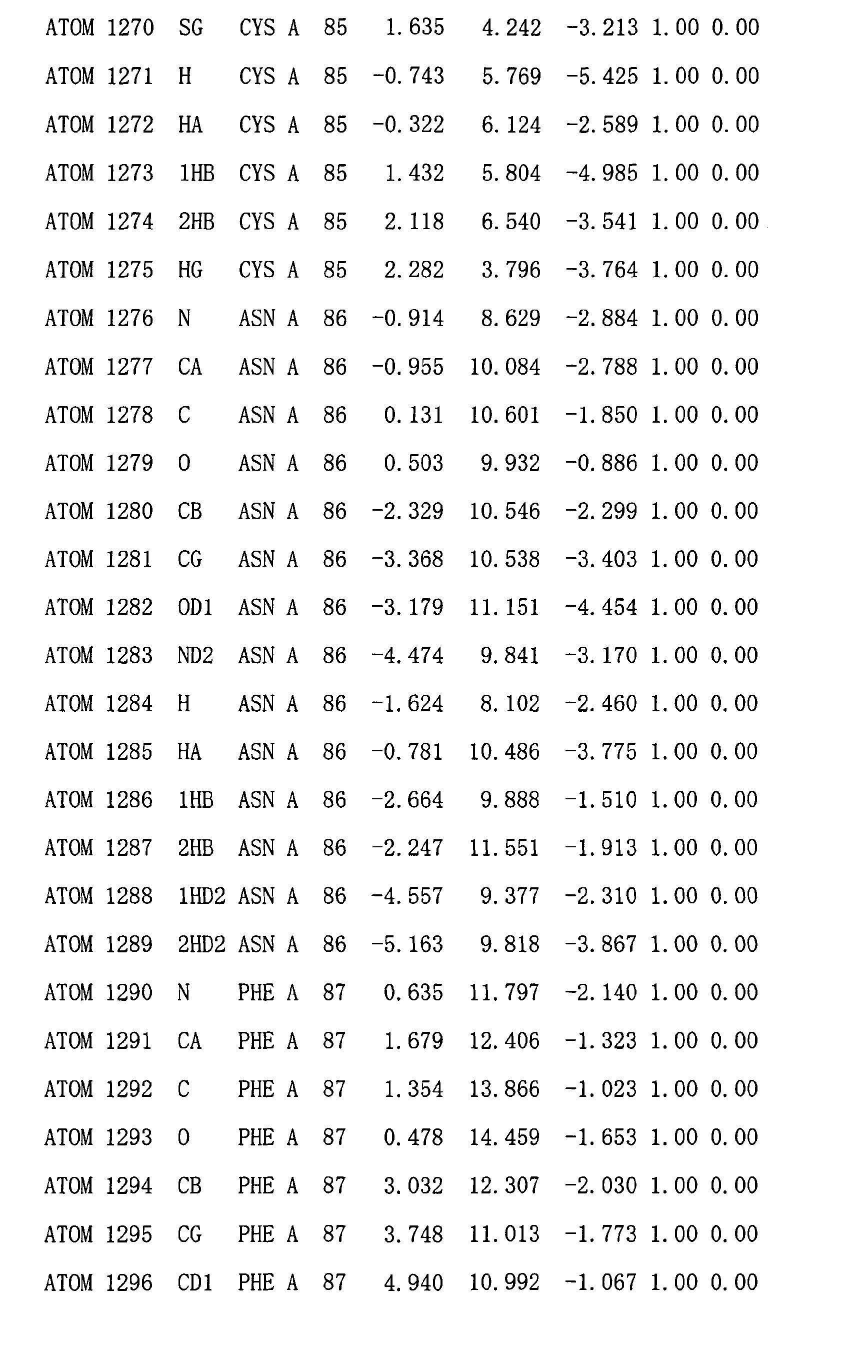

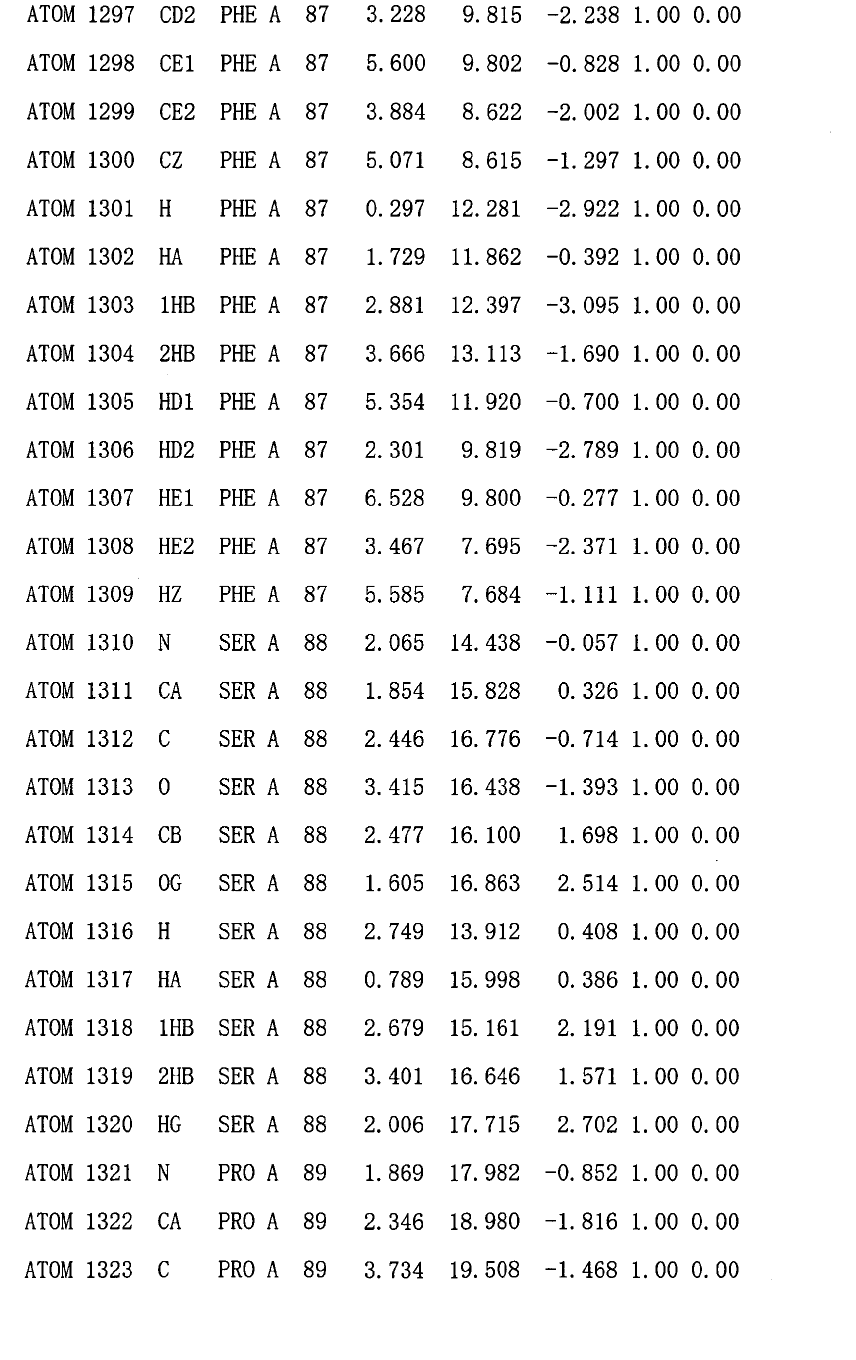

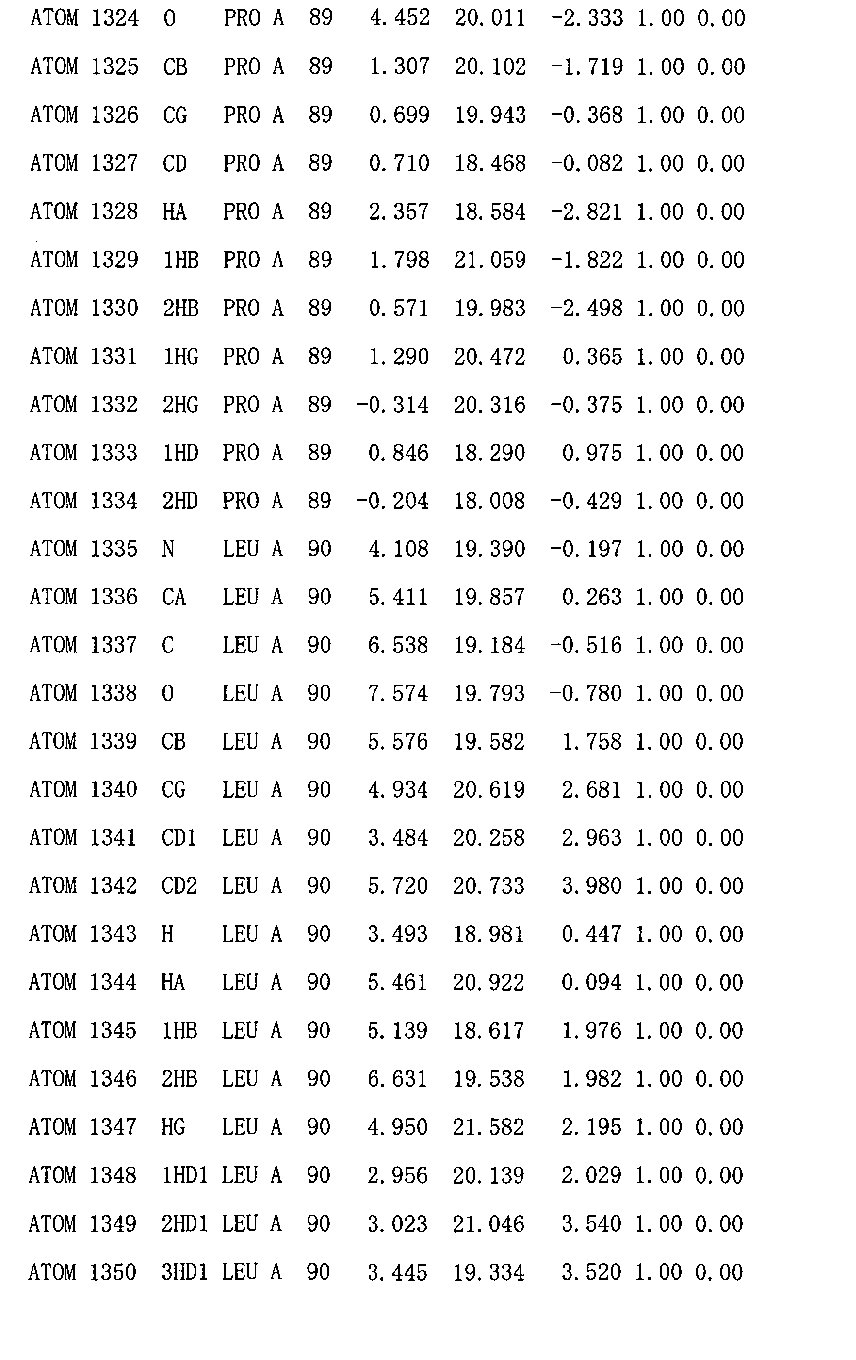

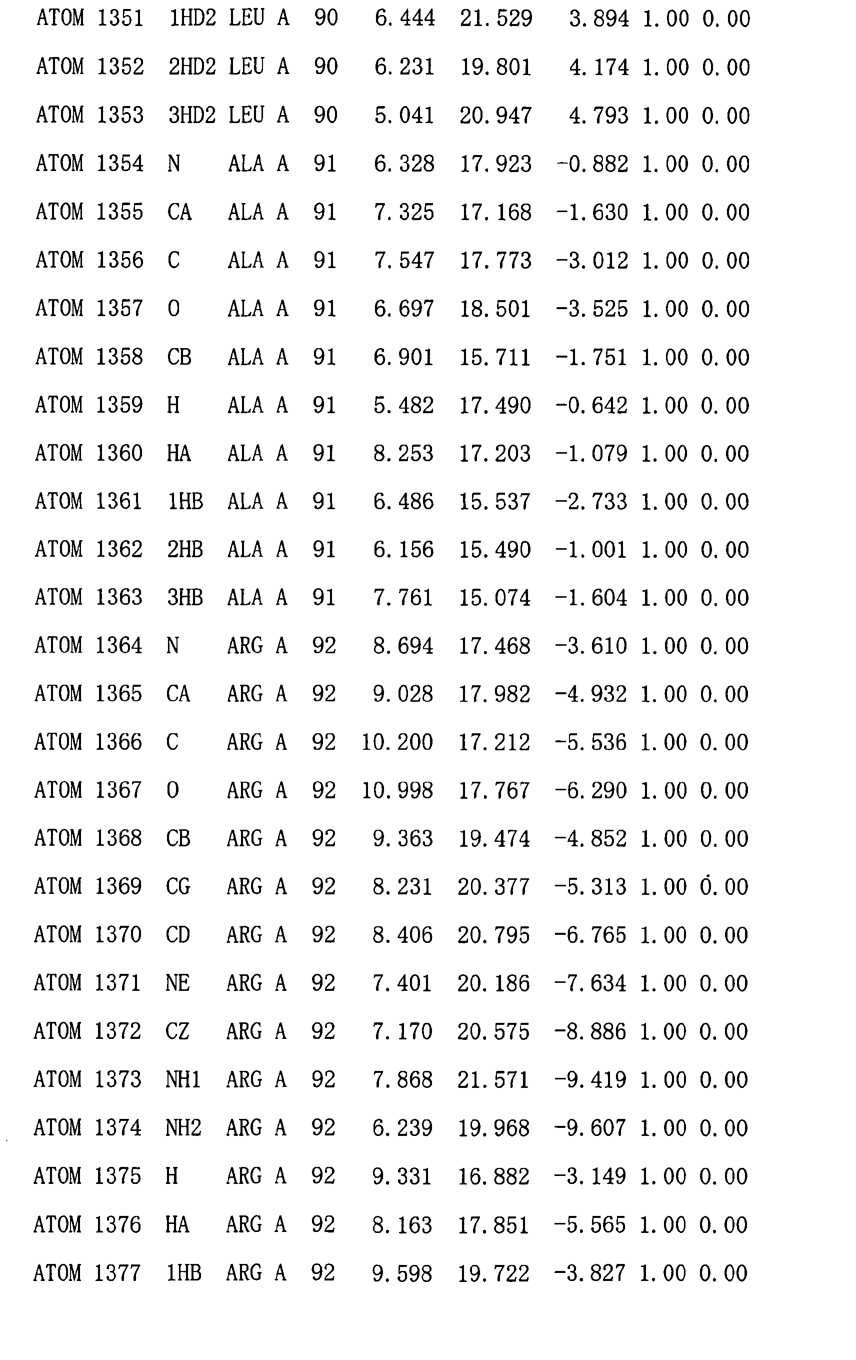

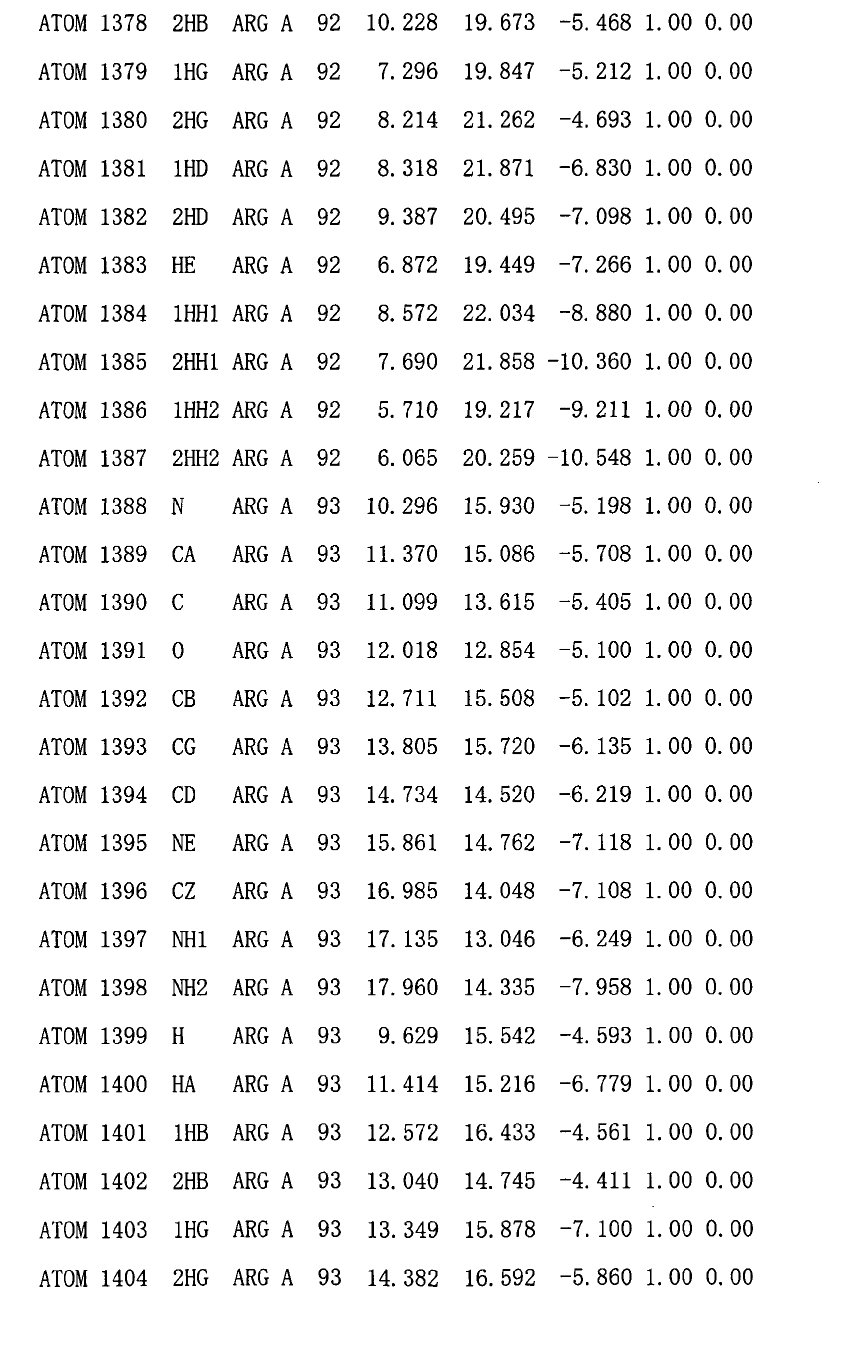

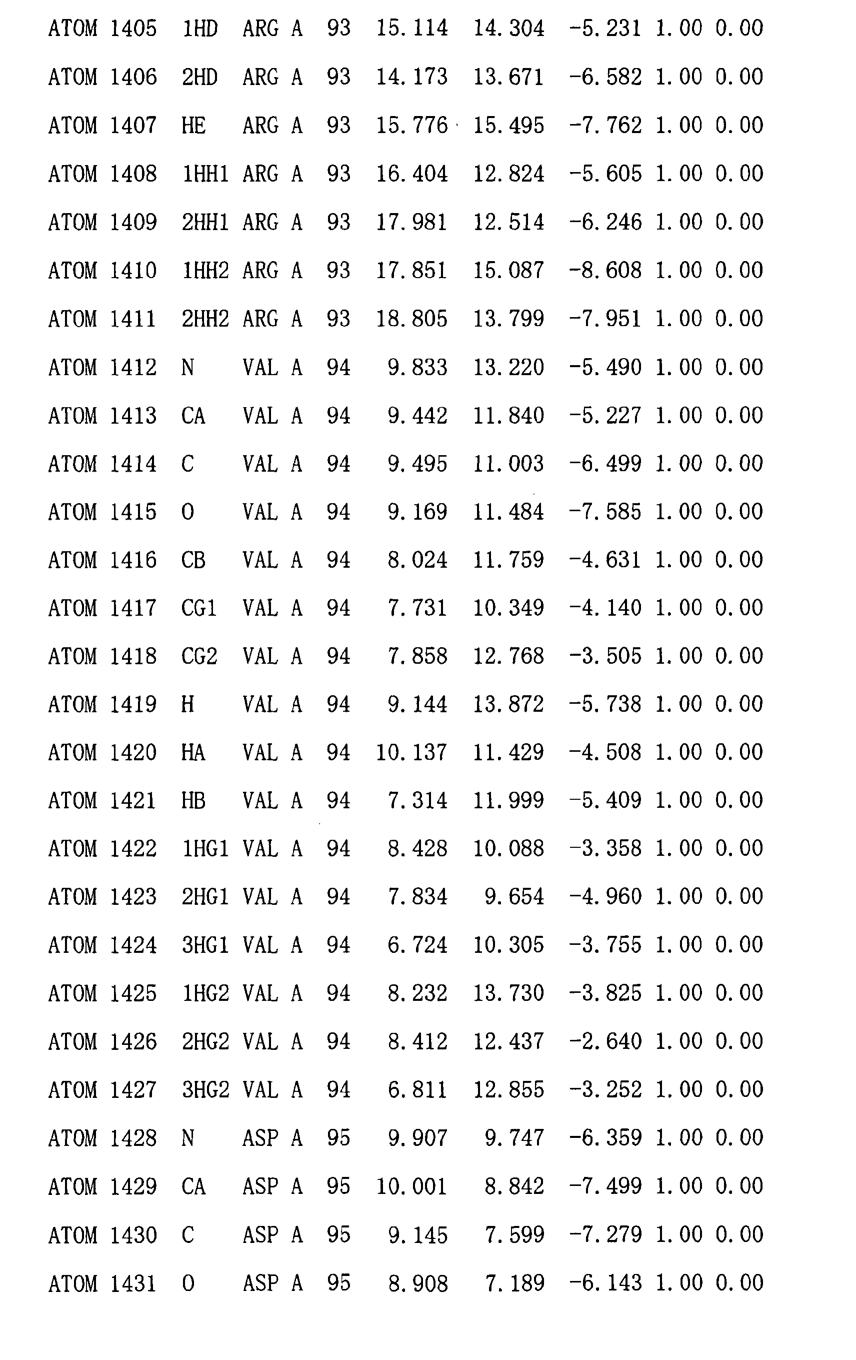

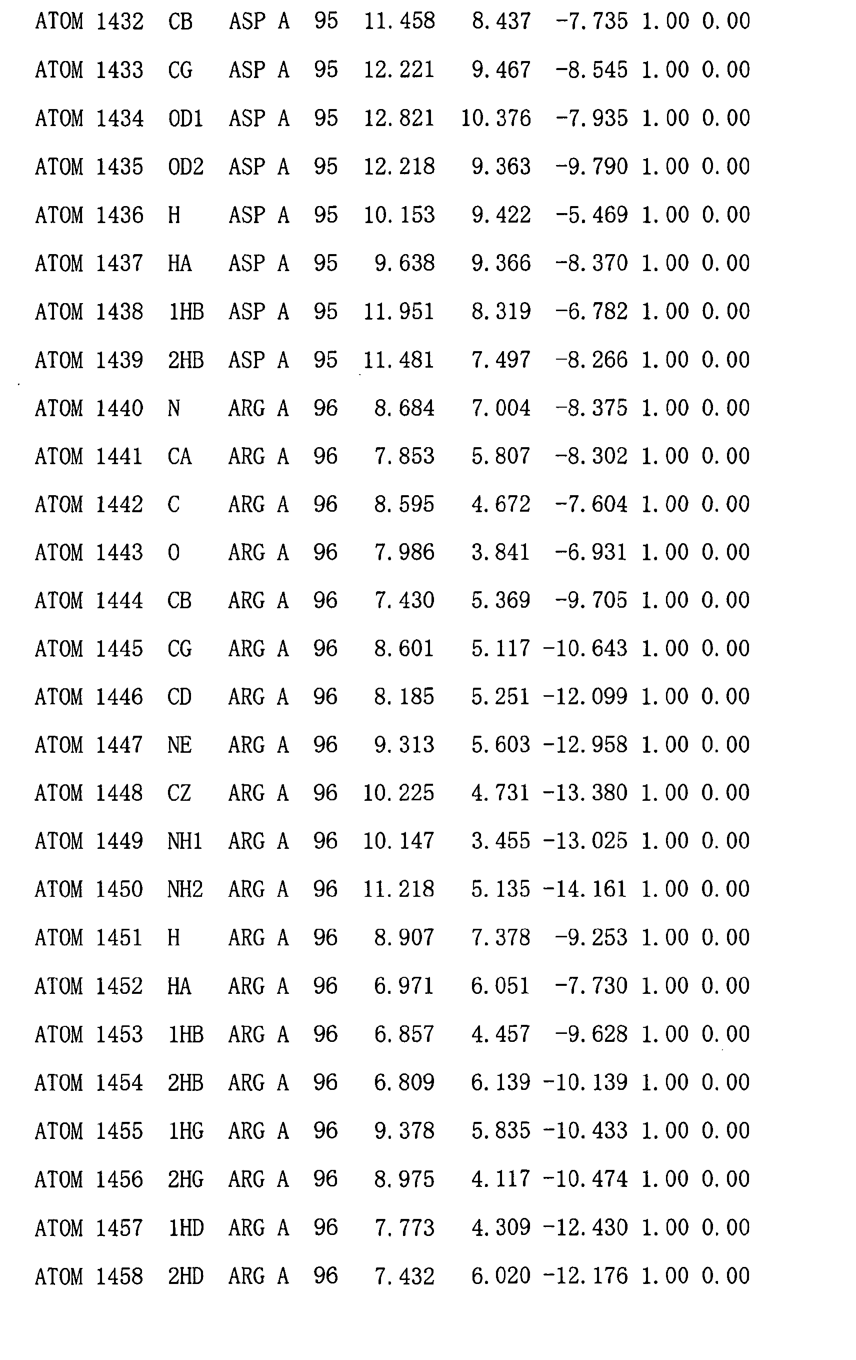

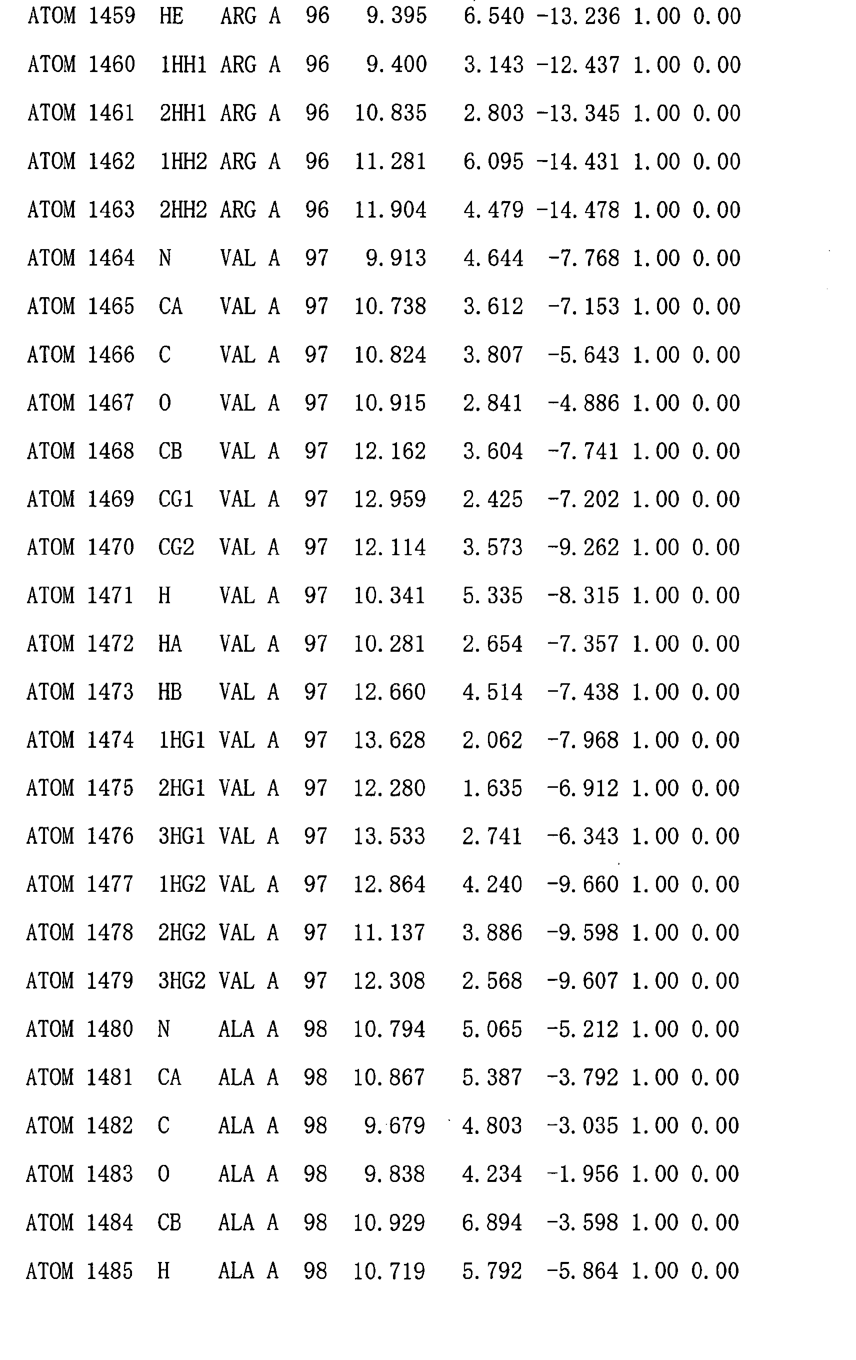

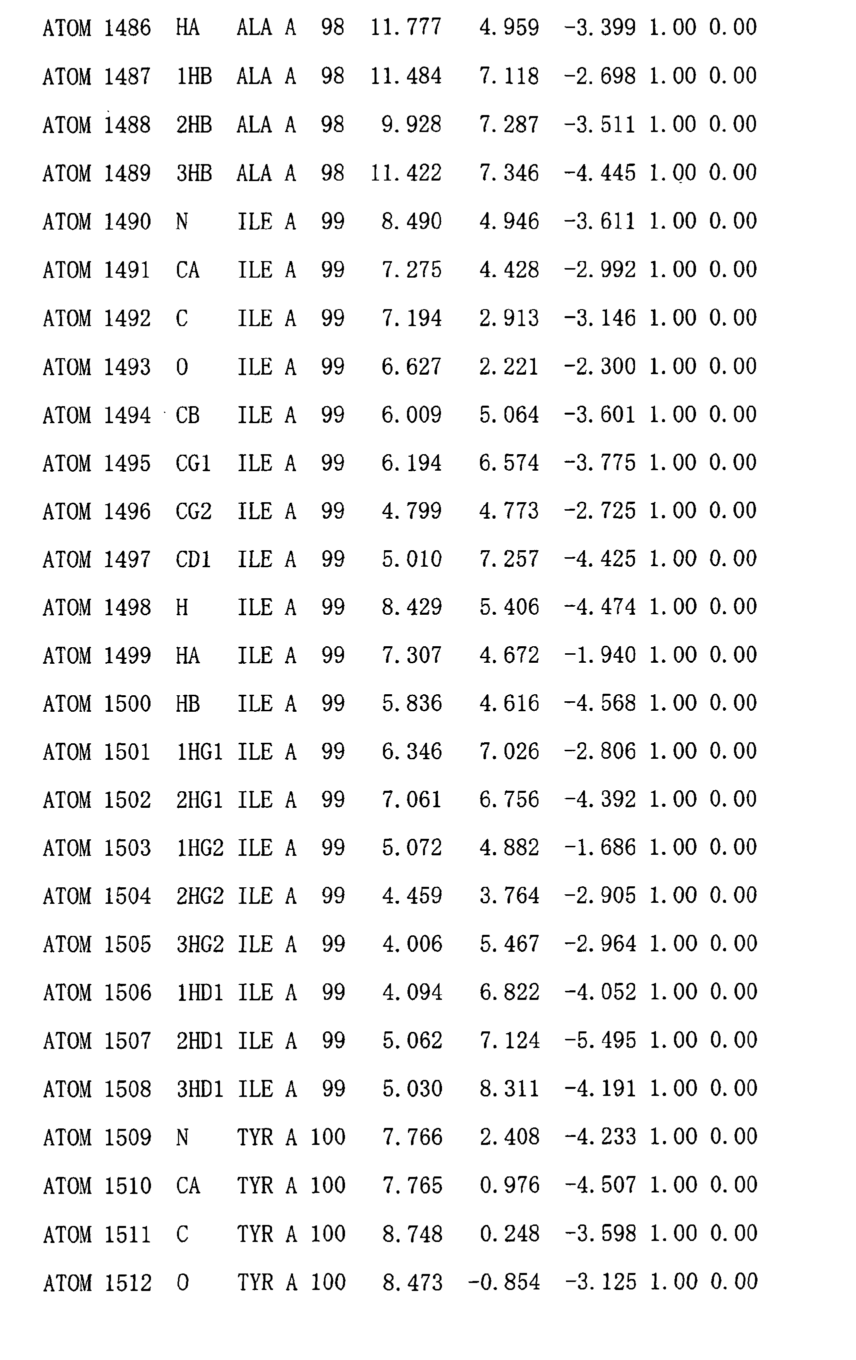

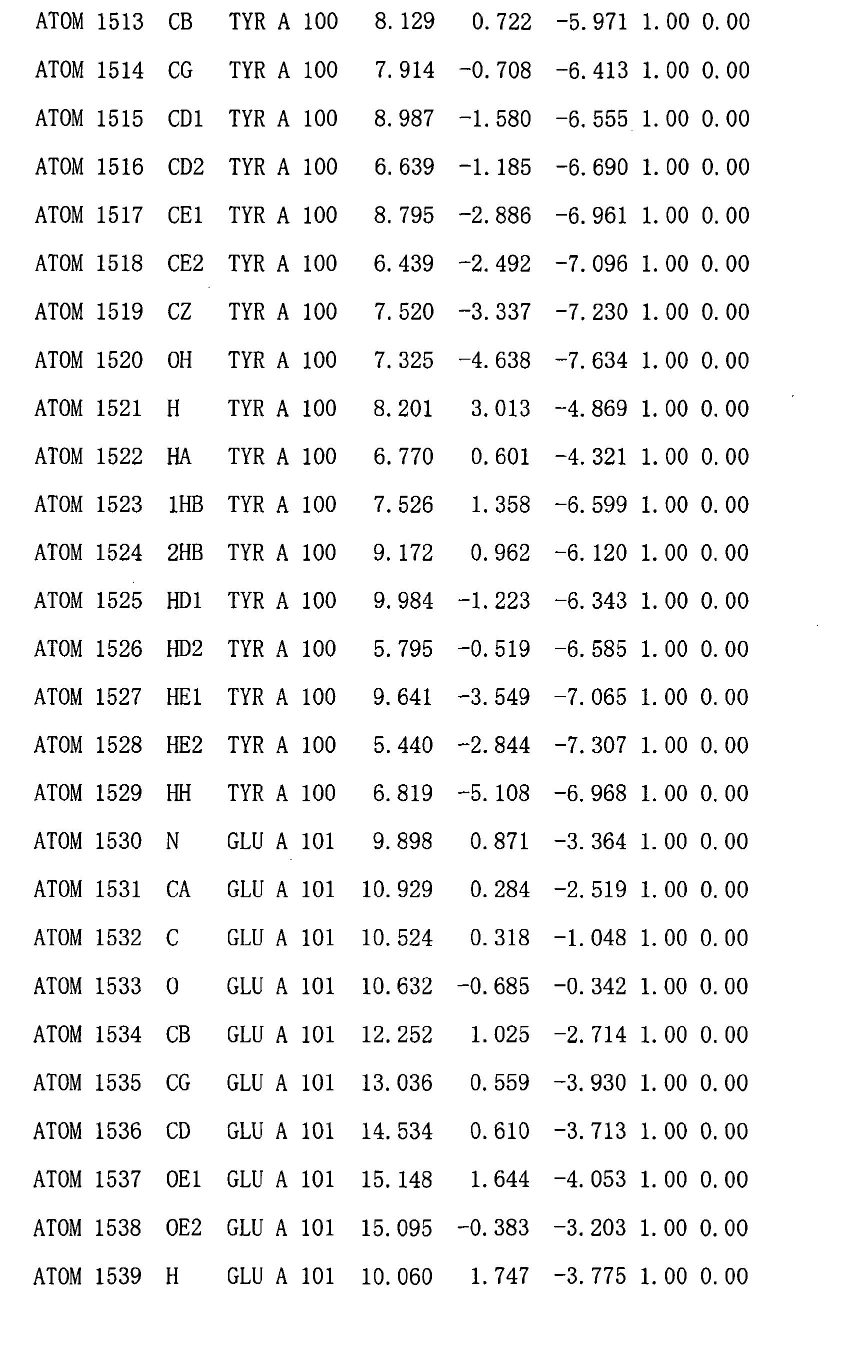

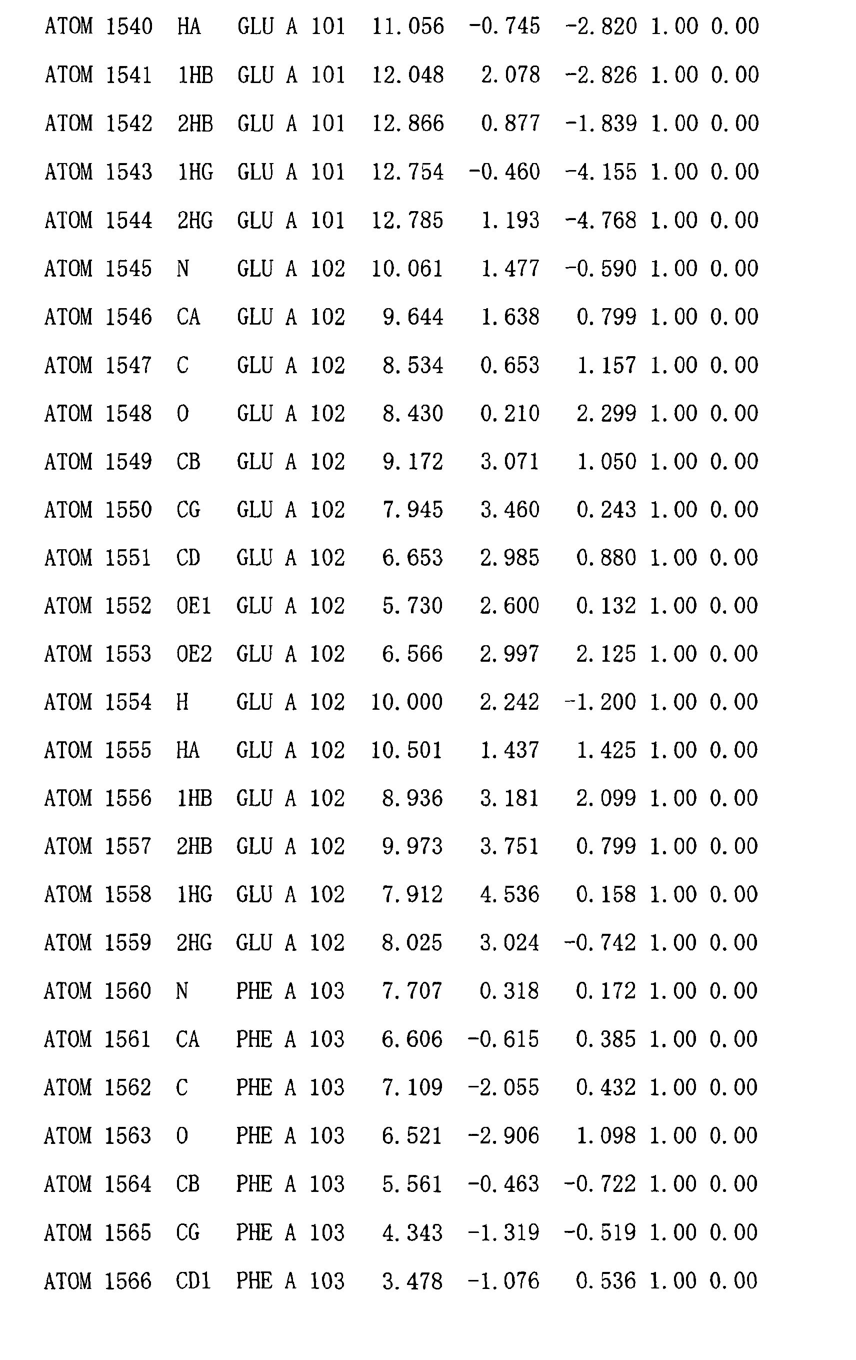

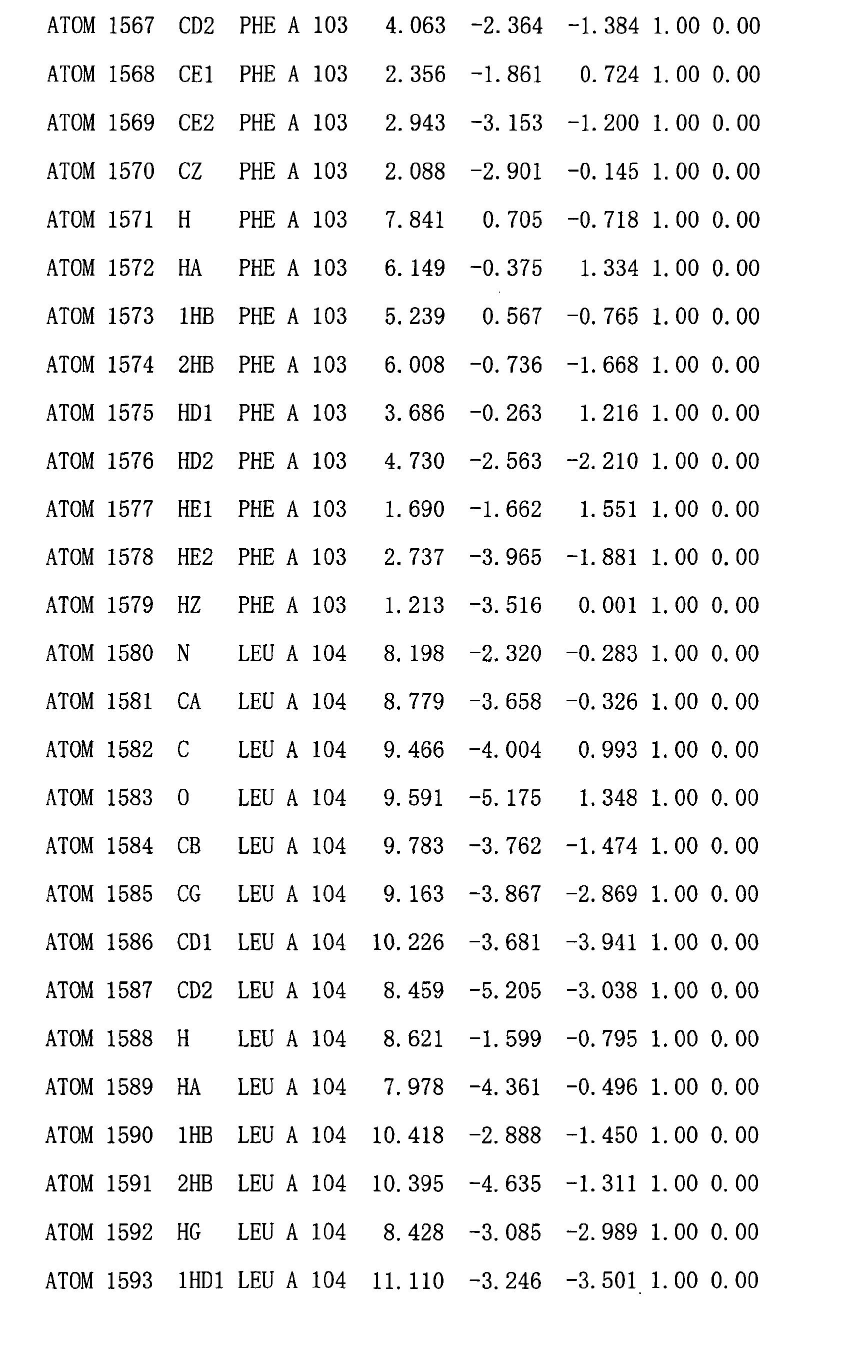

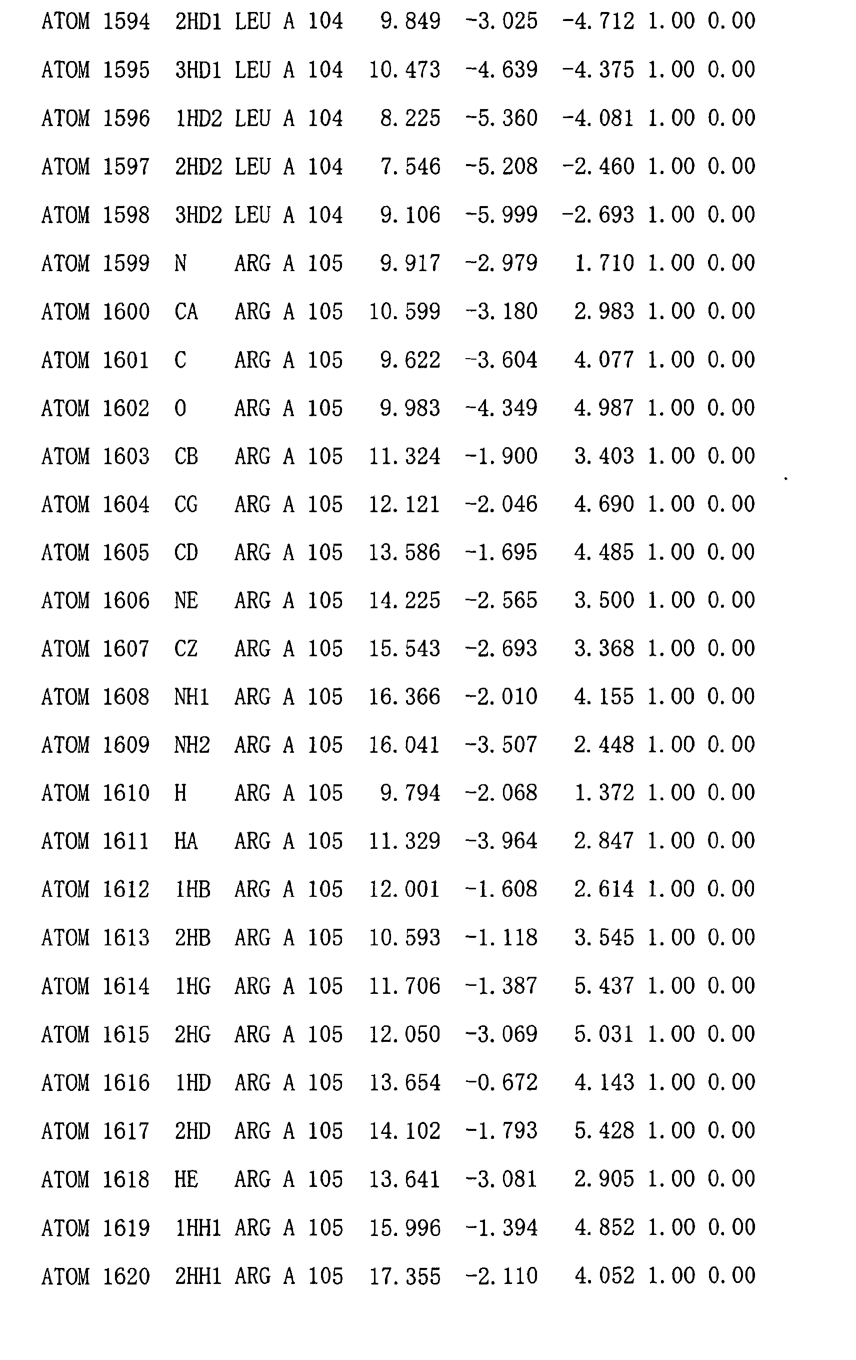

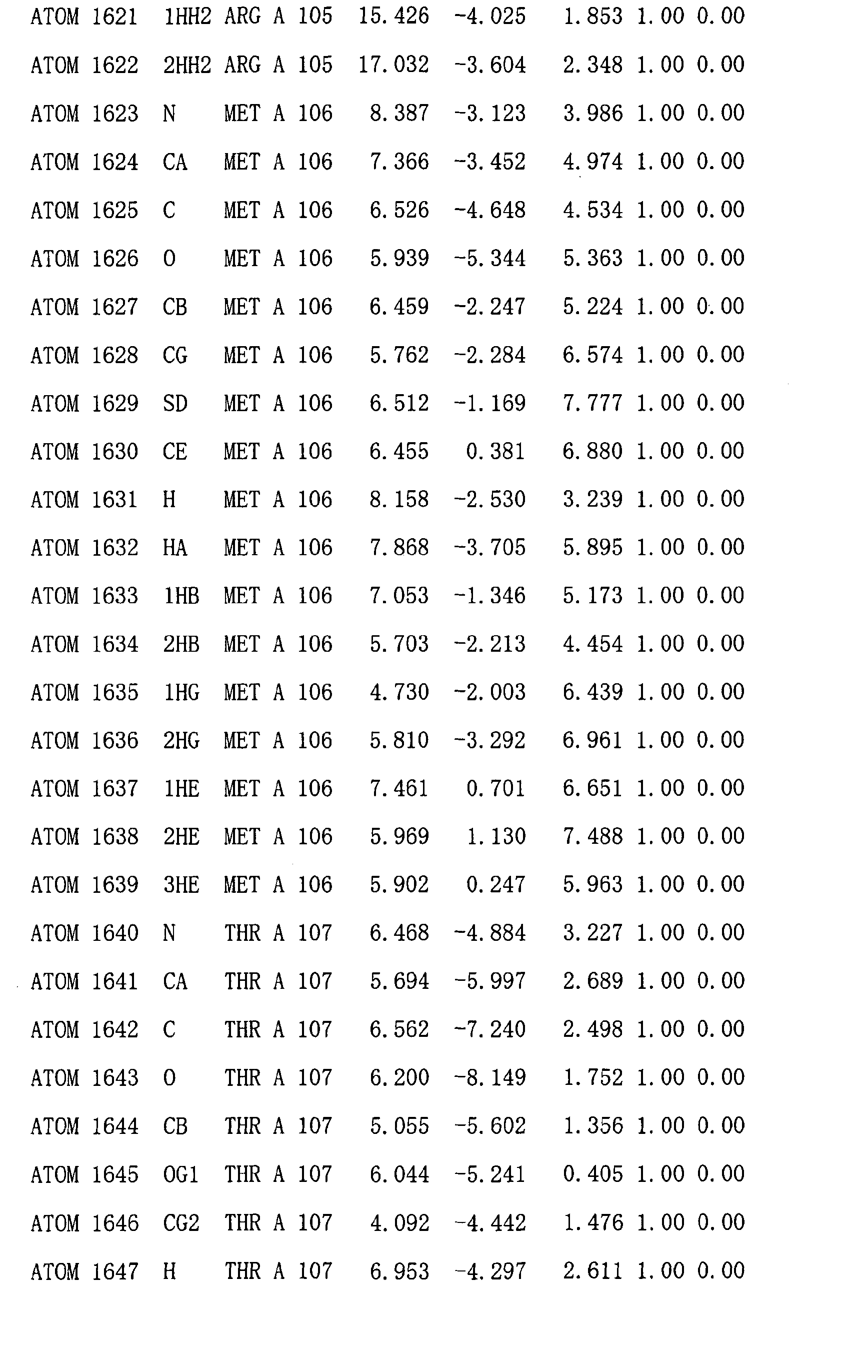

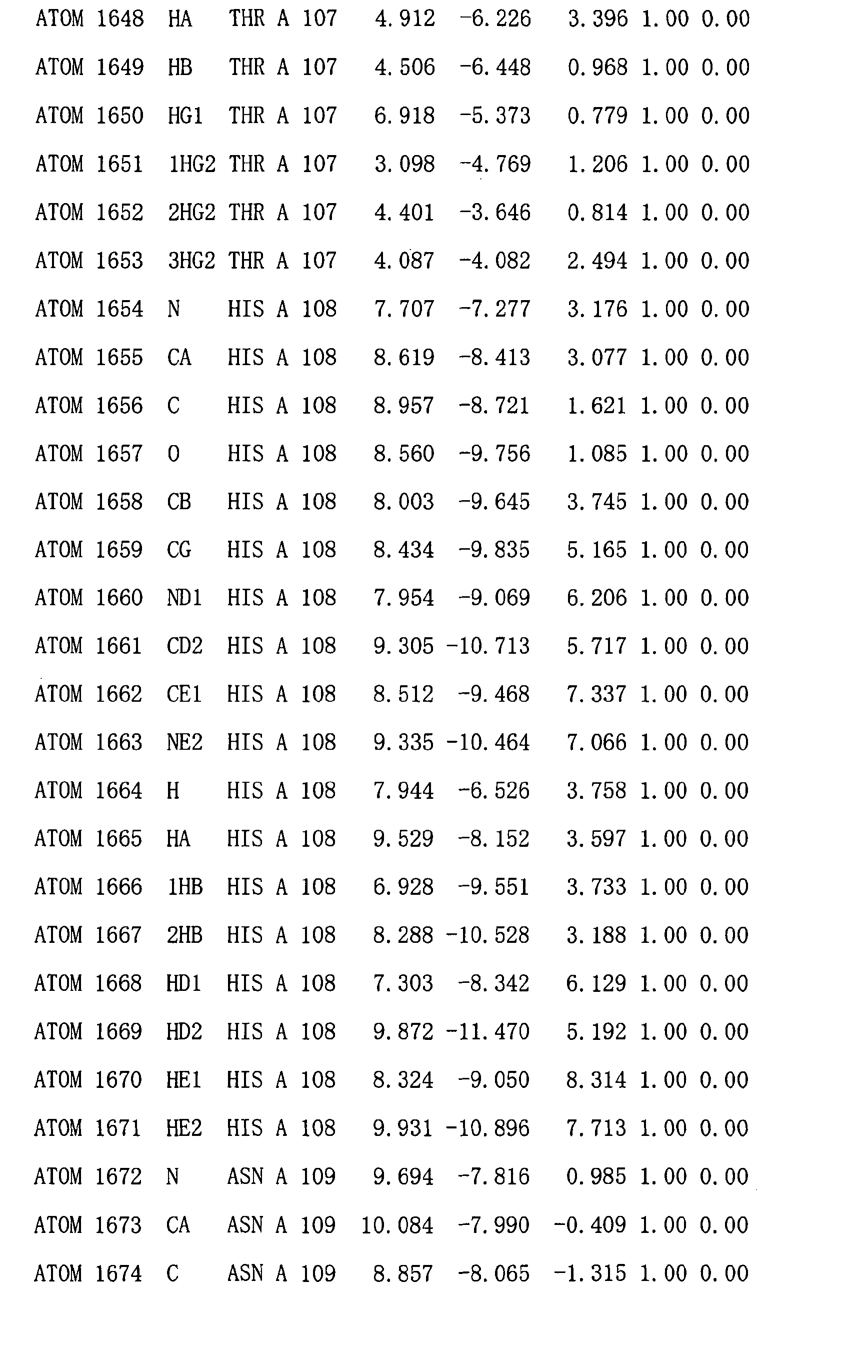

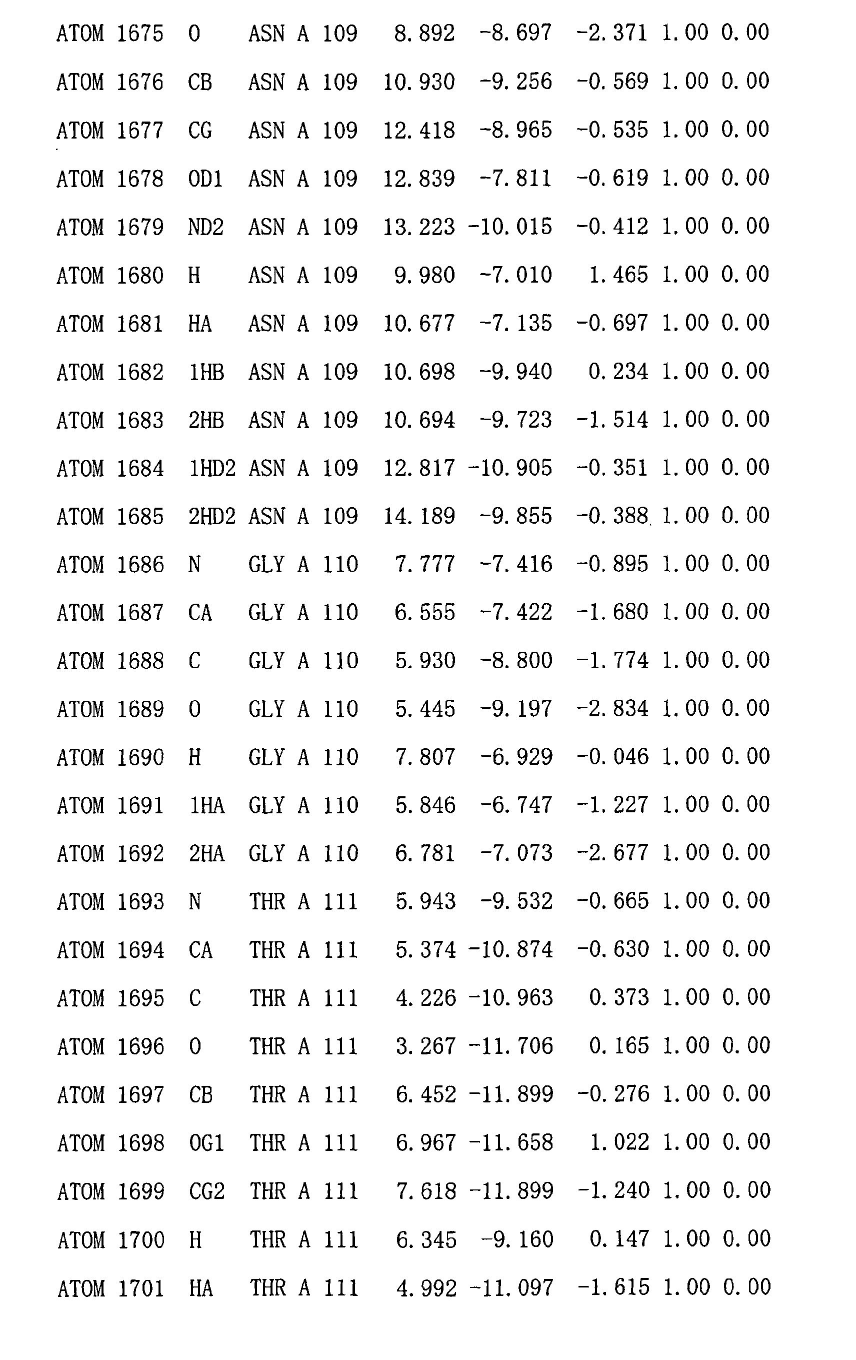

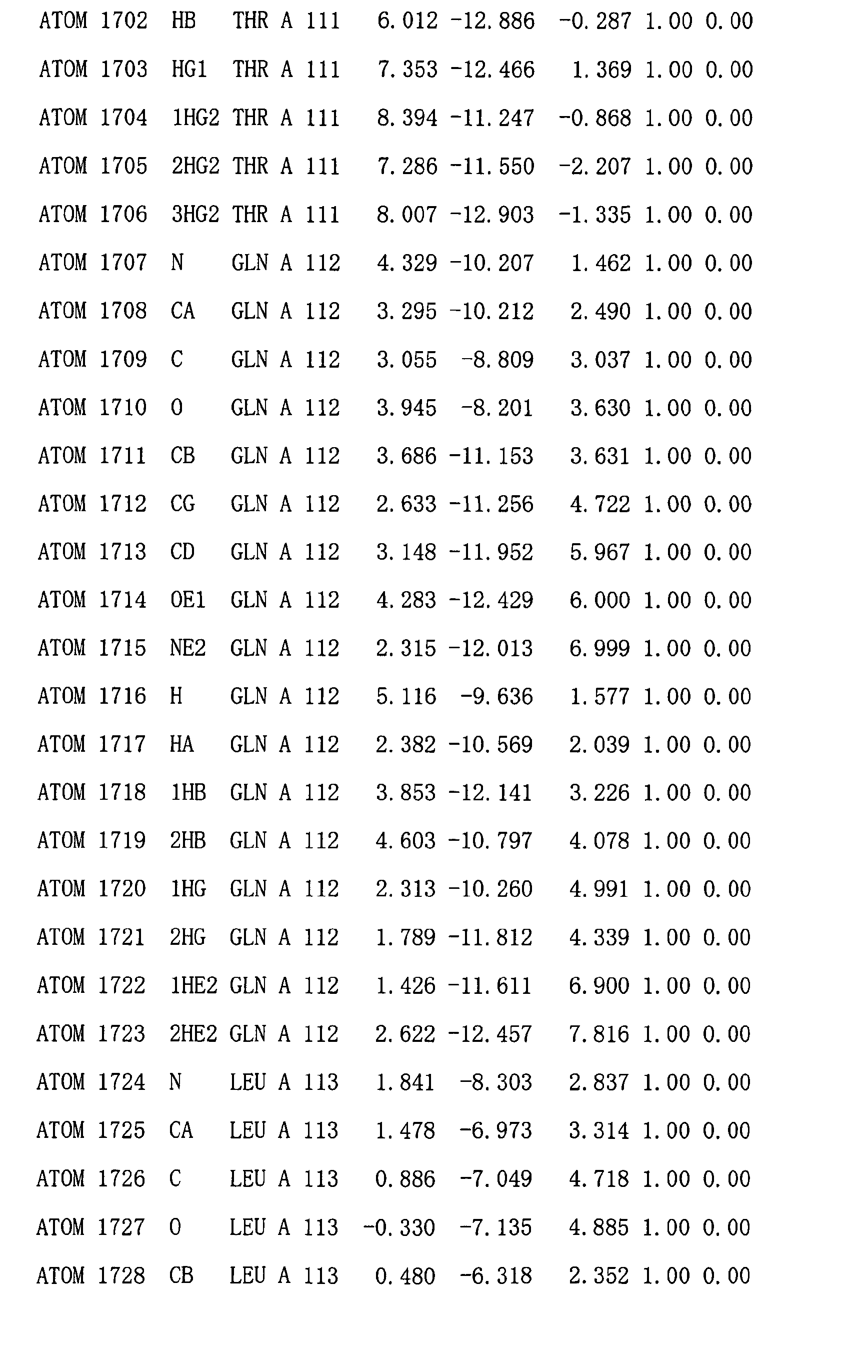

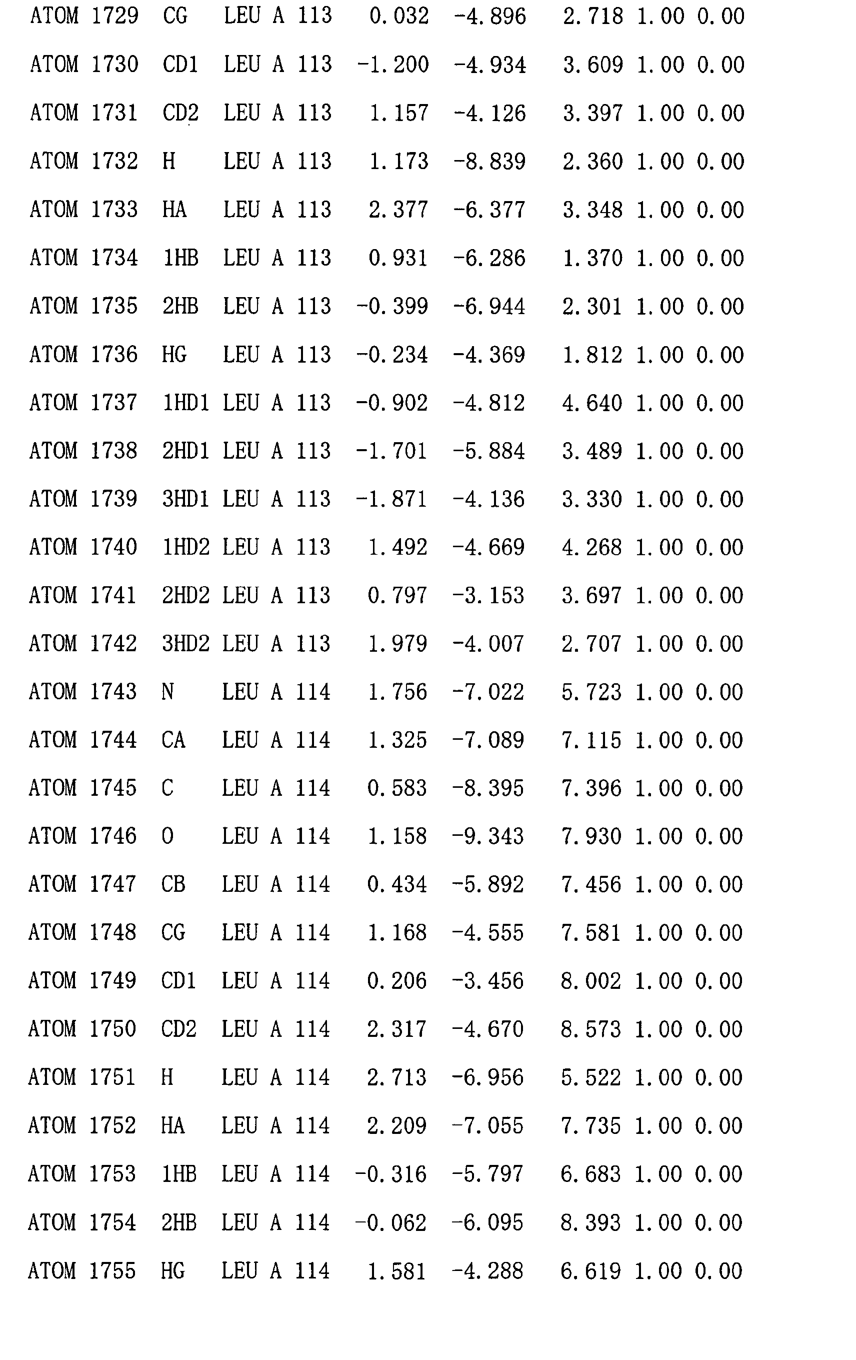

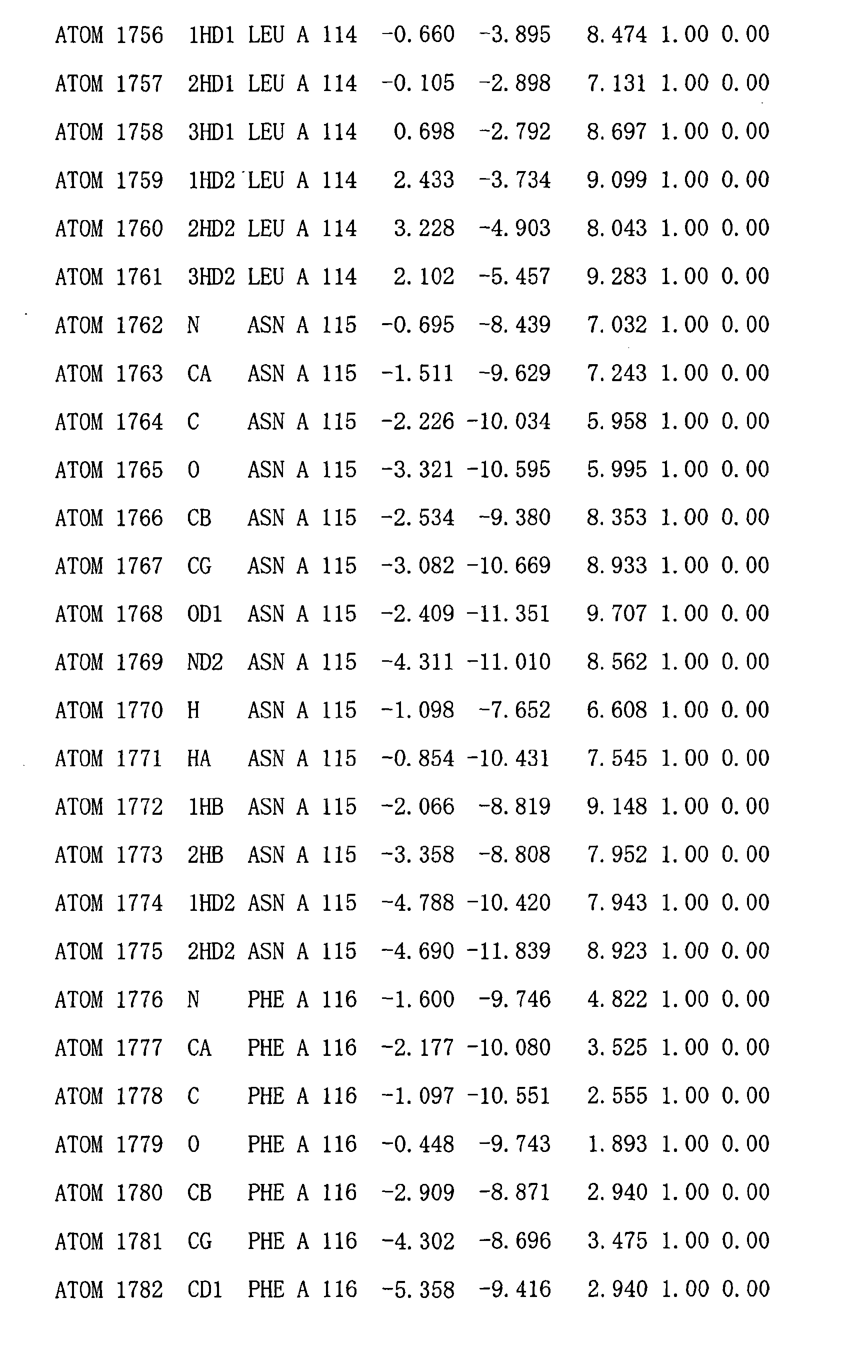

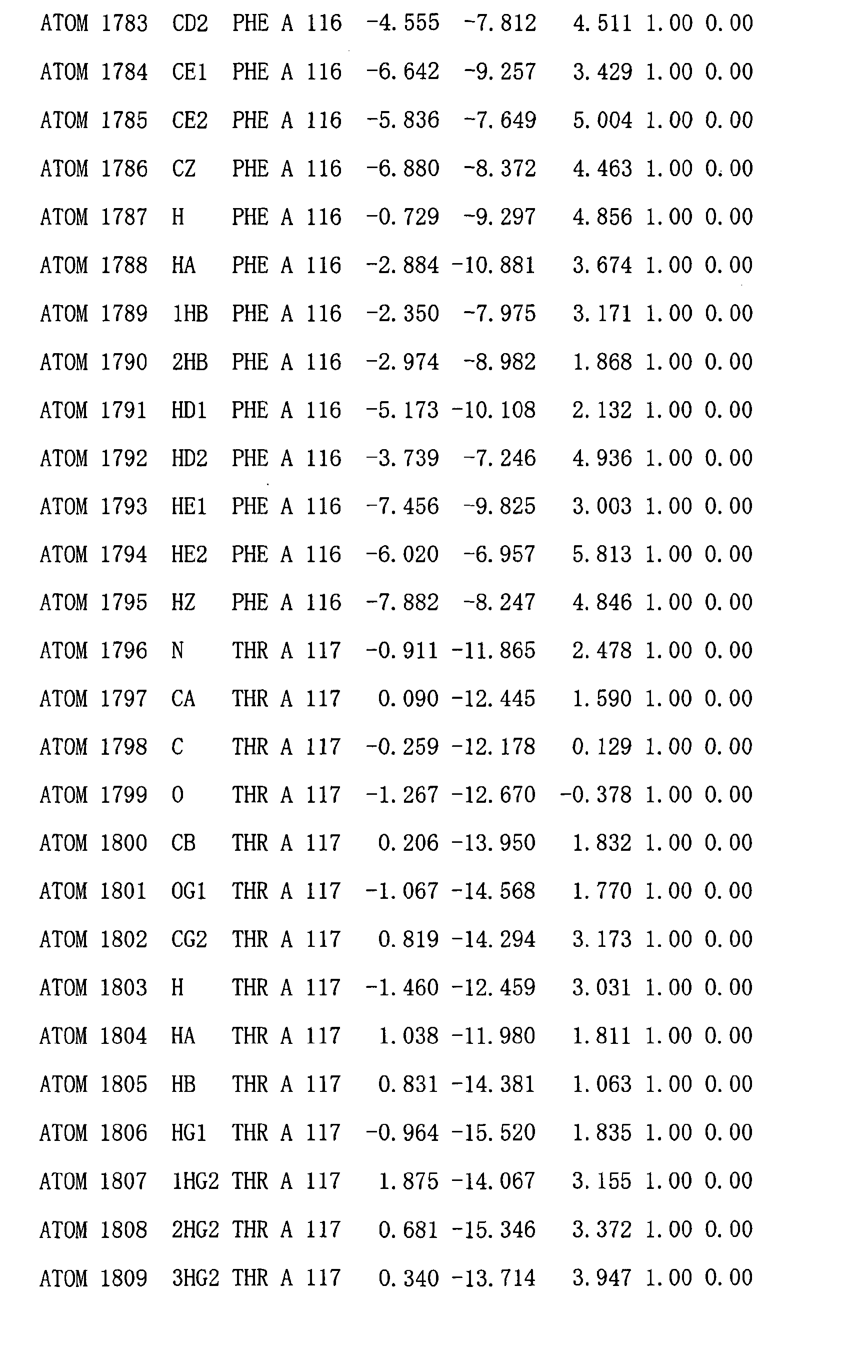

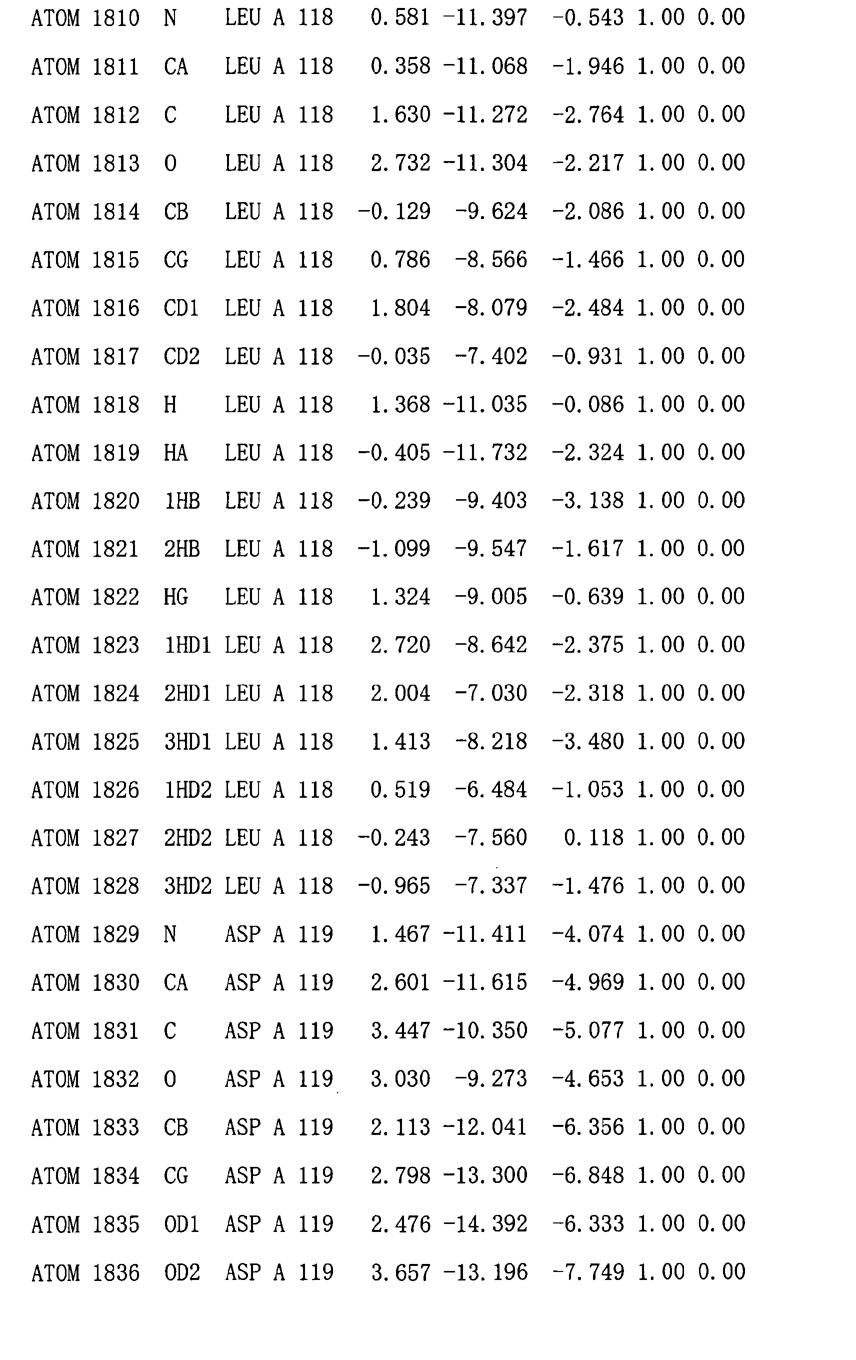

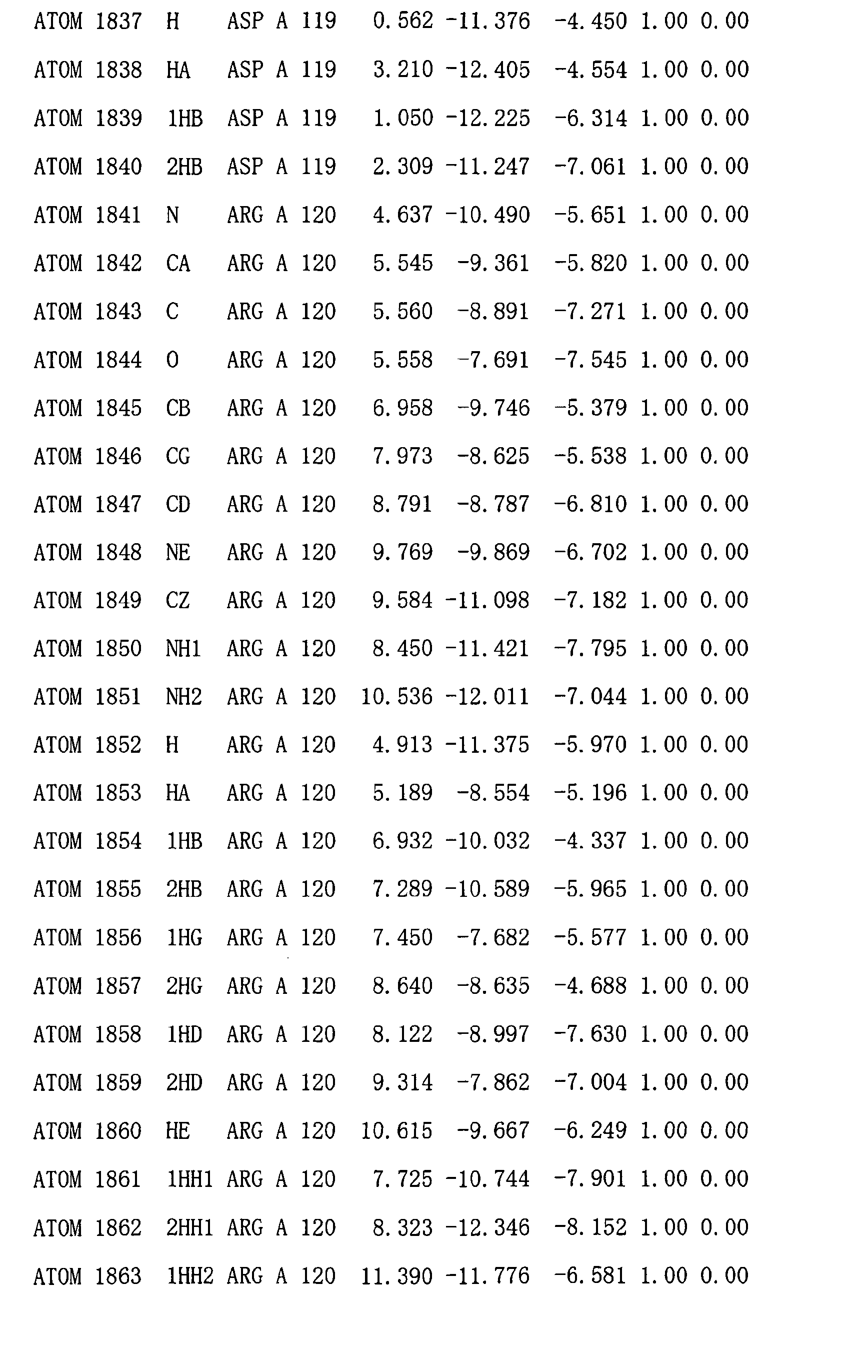

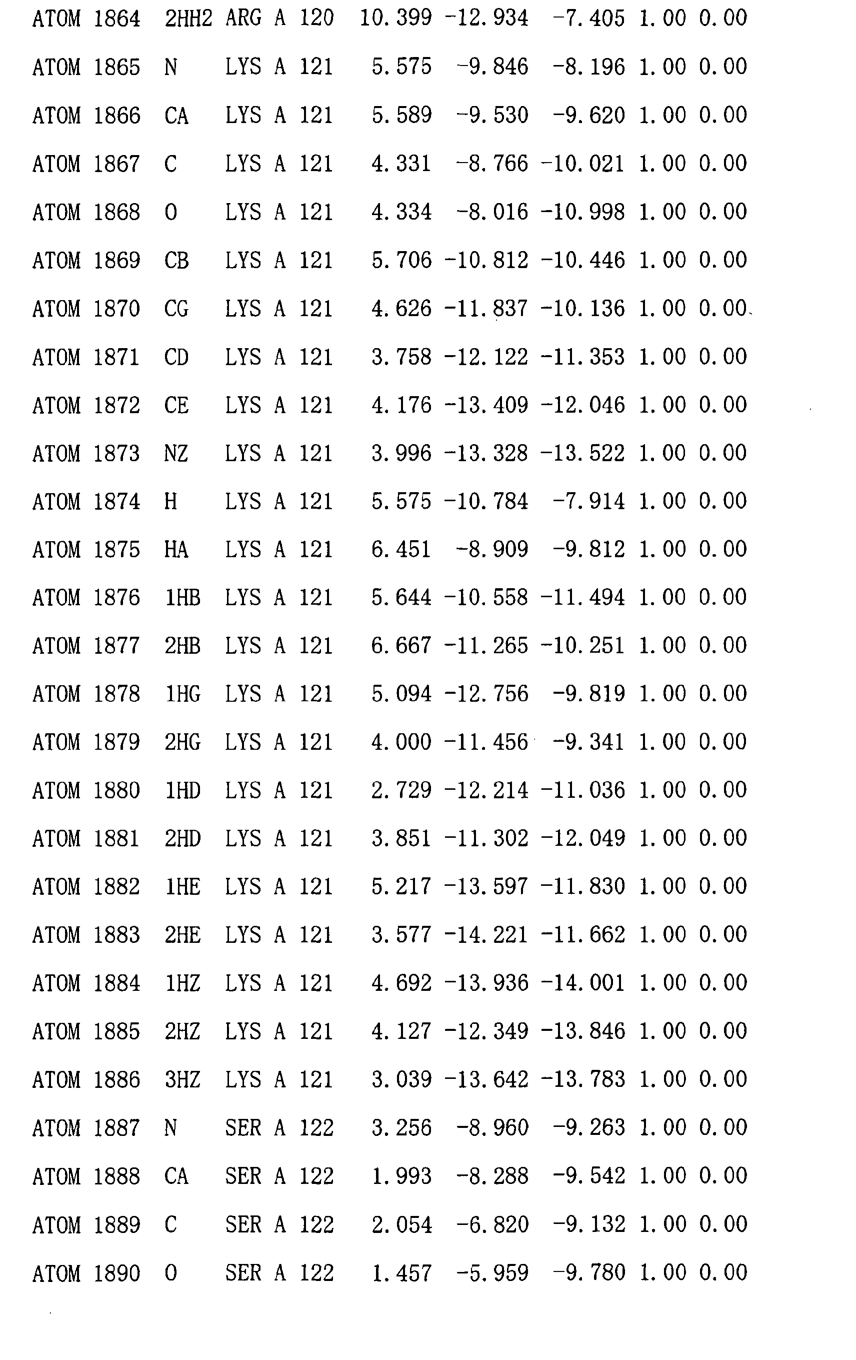

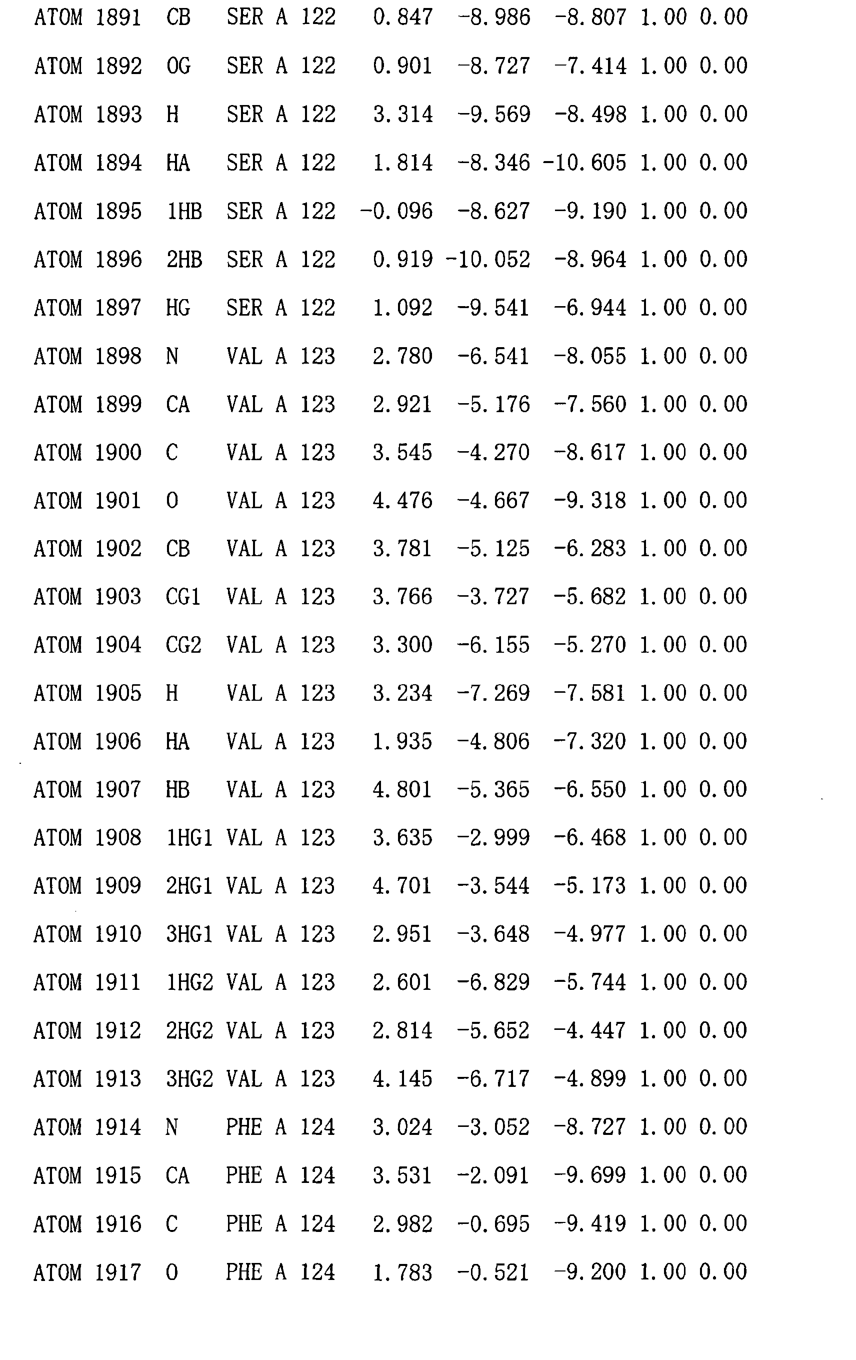

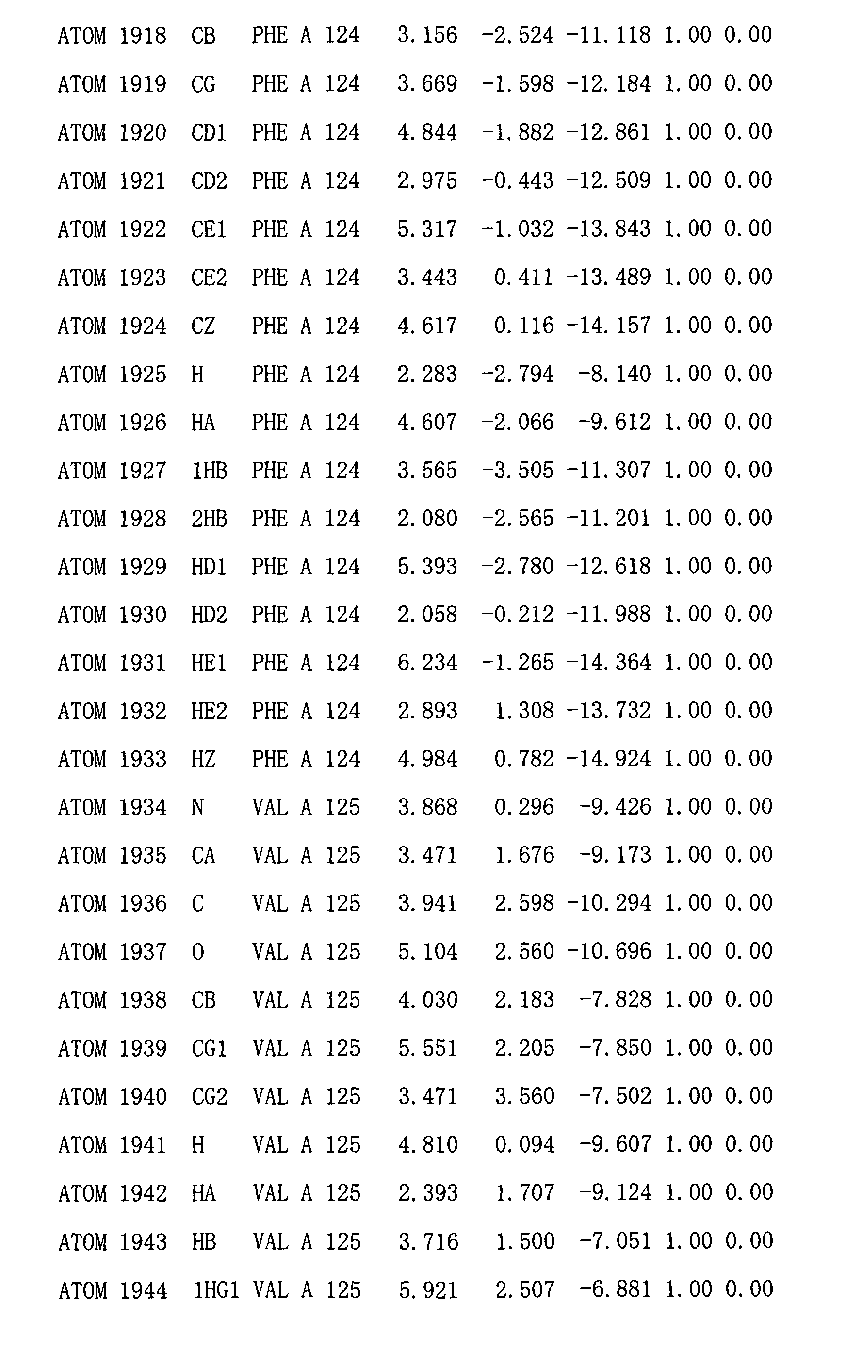

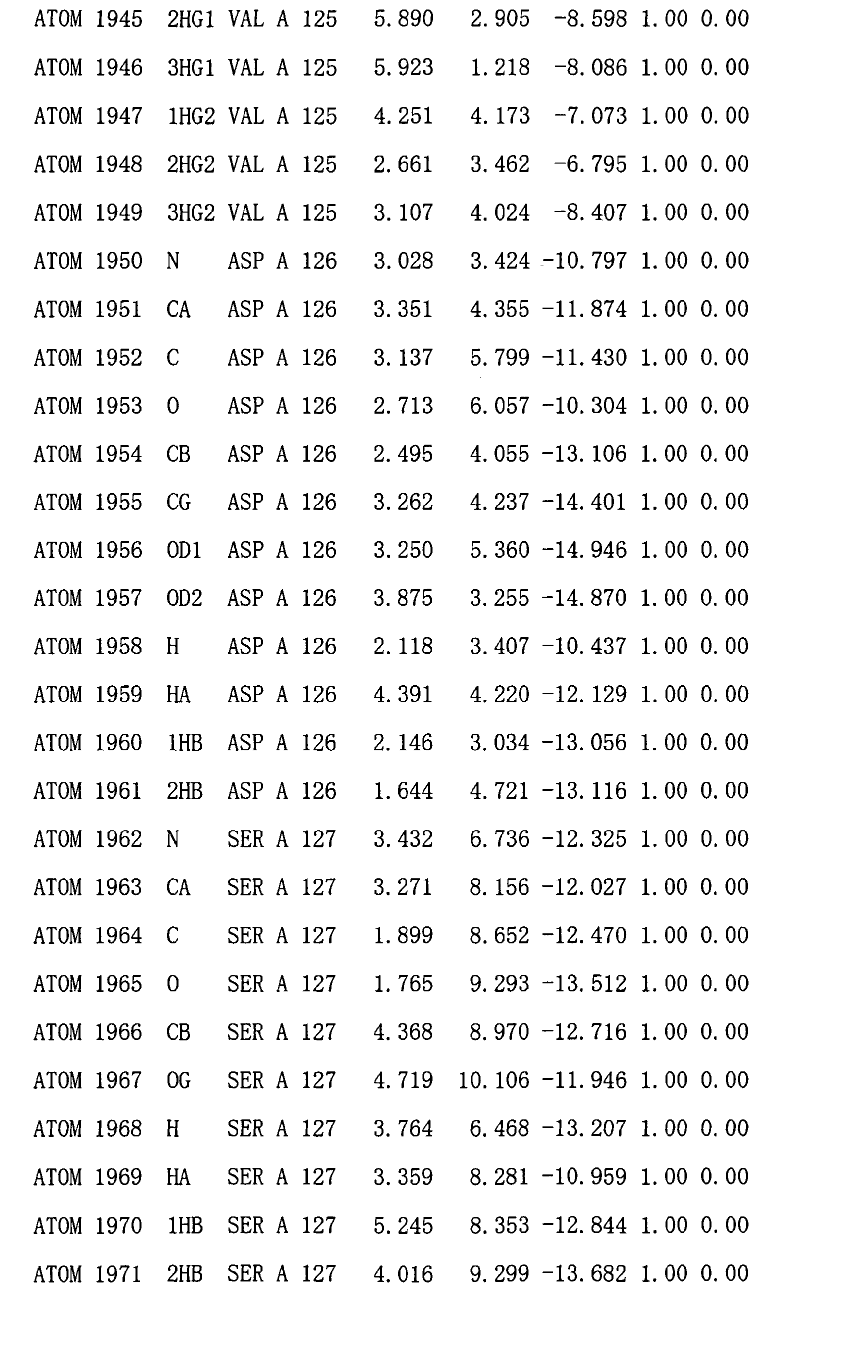

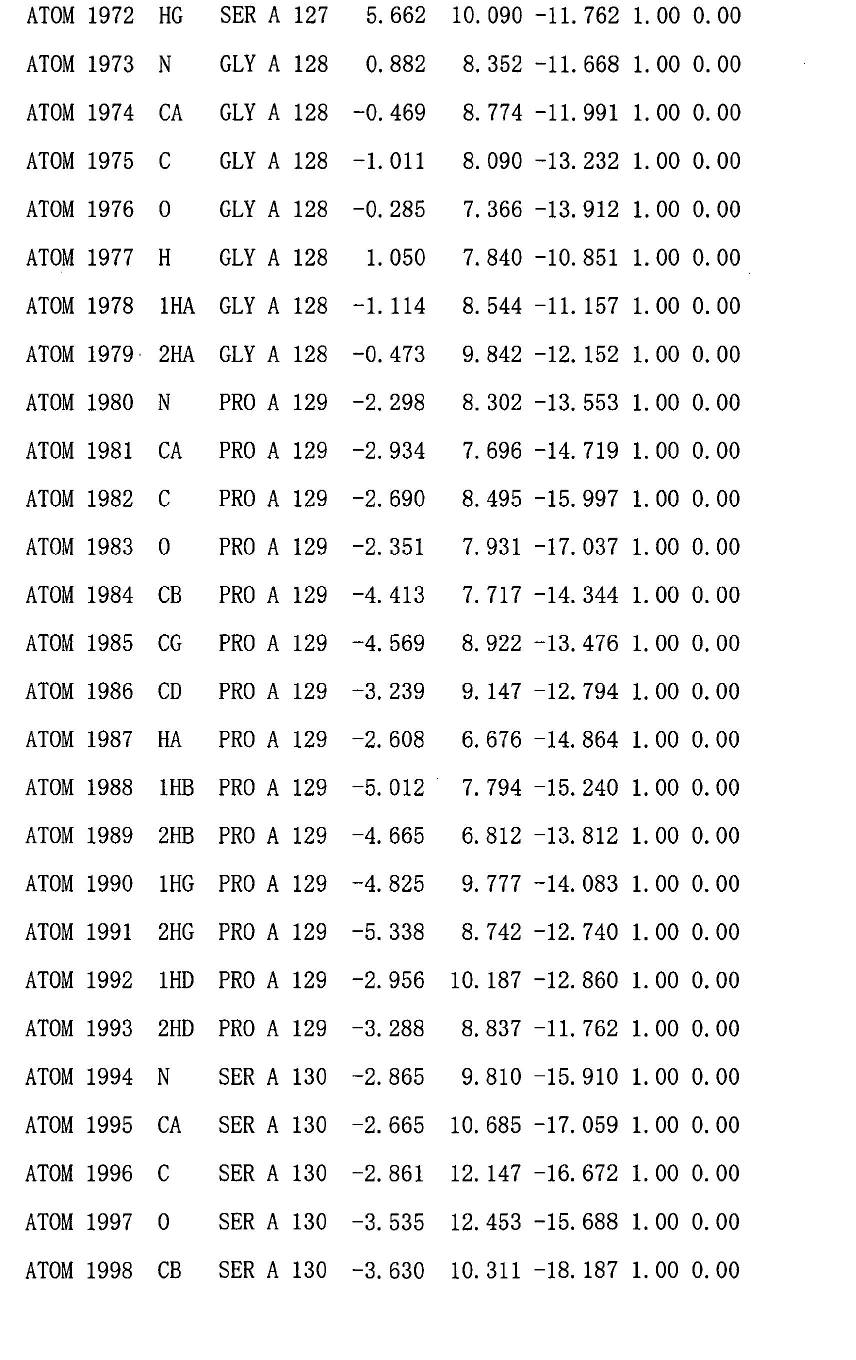

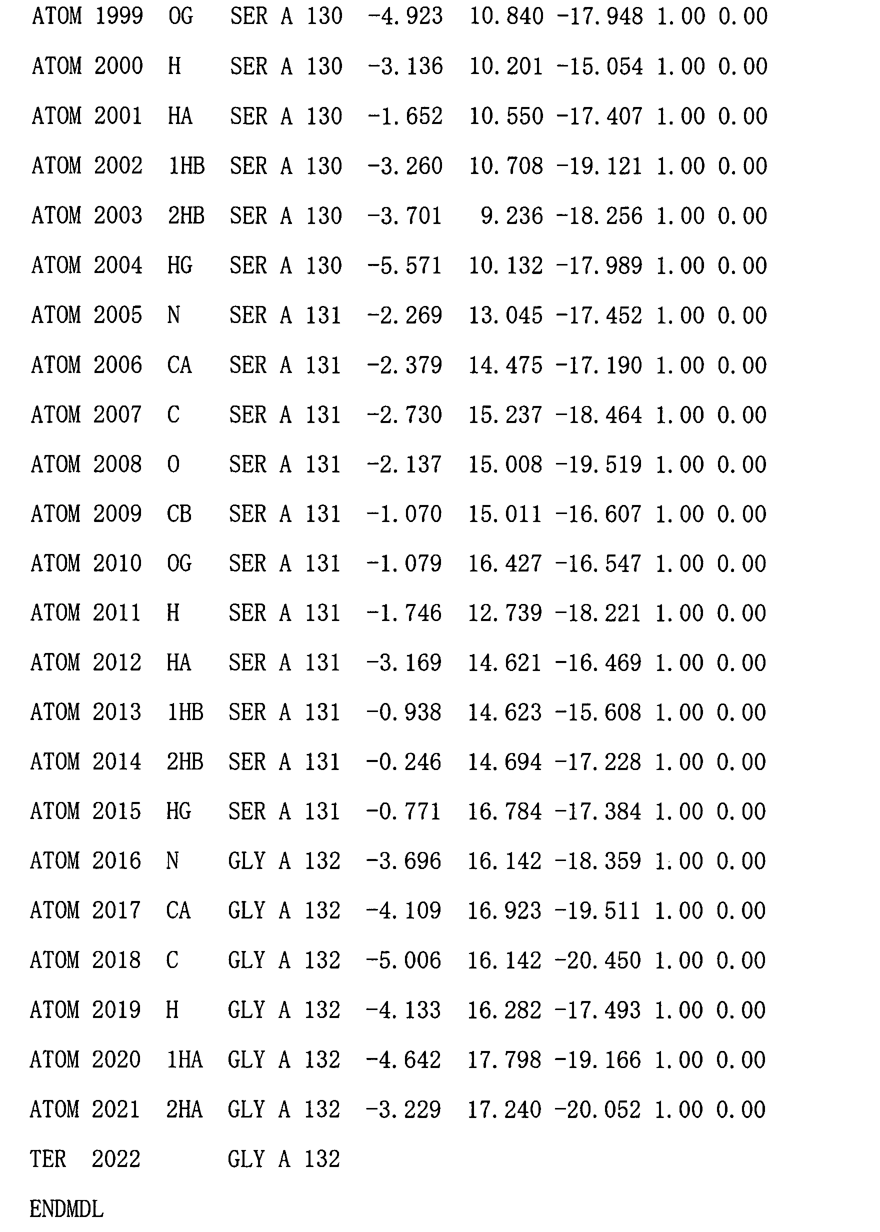

### Three-Dimensional Structure Coordinate 7

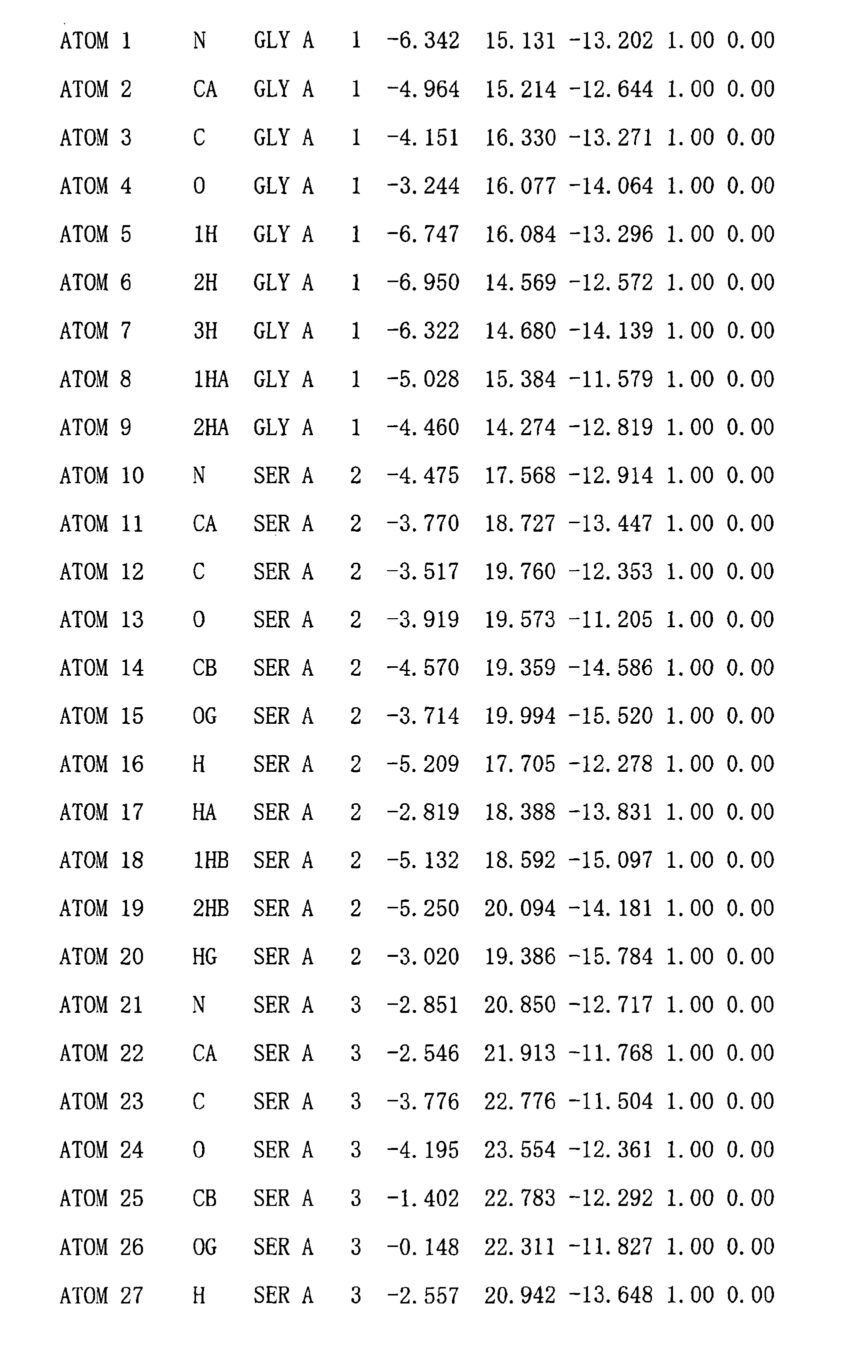

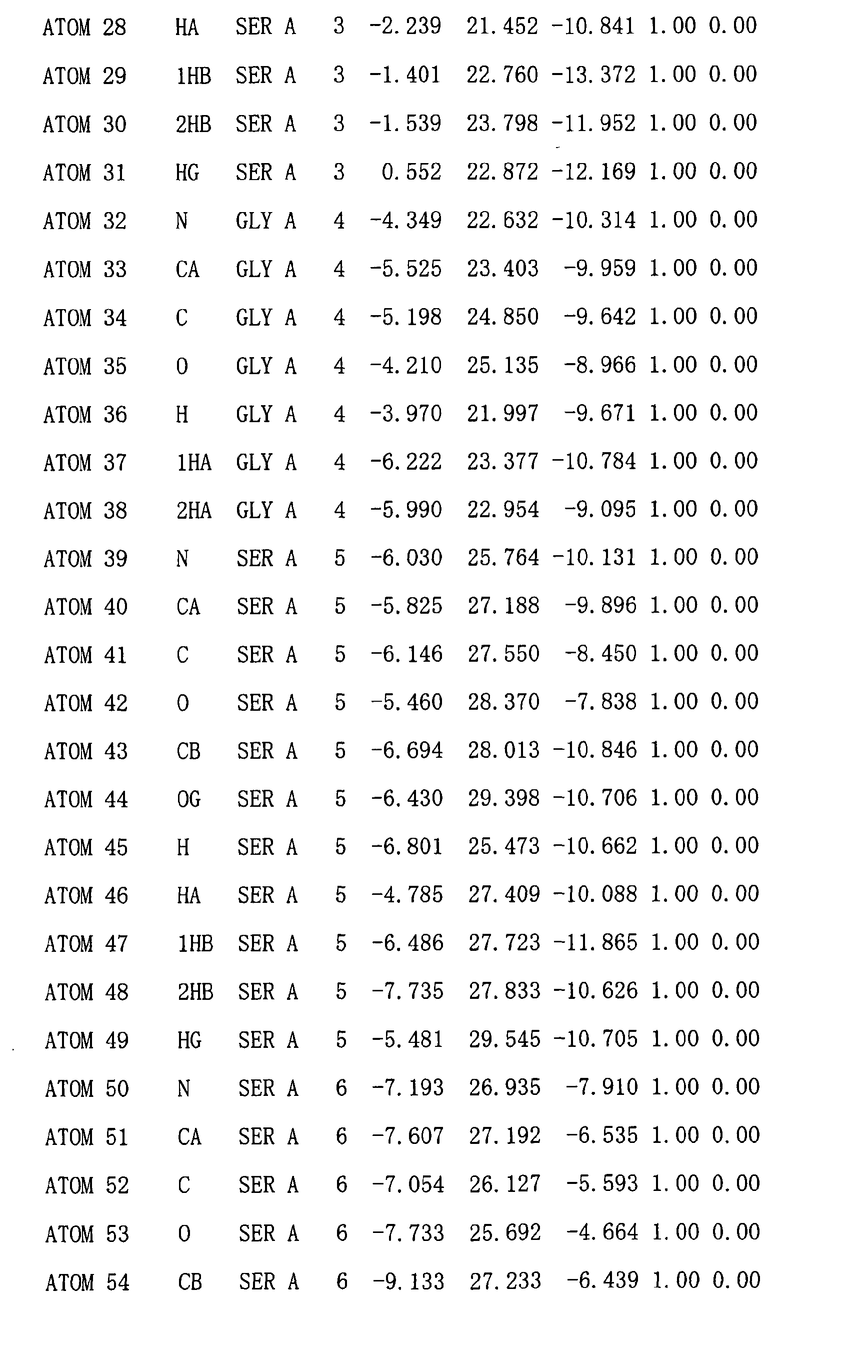

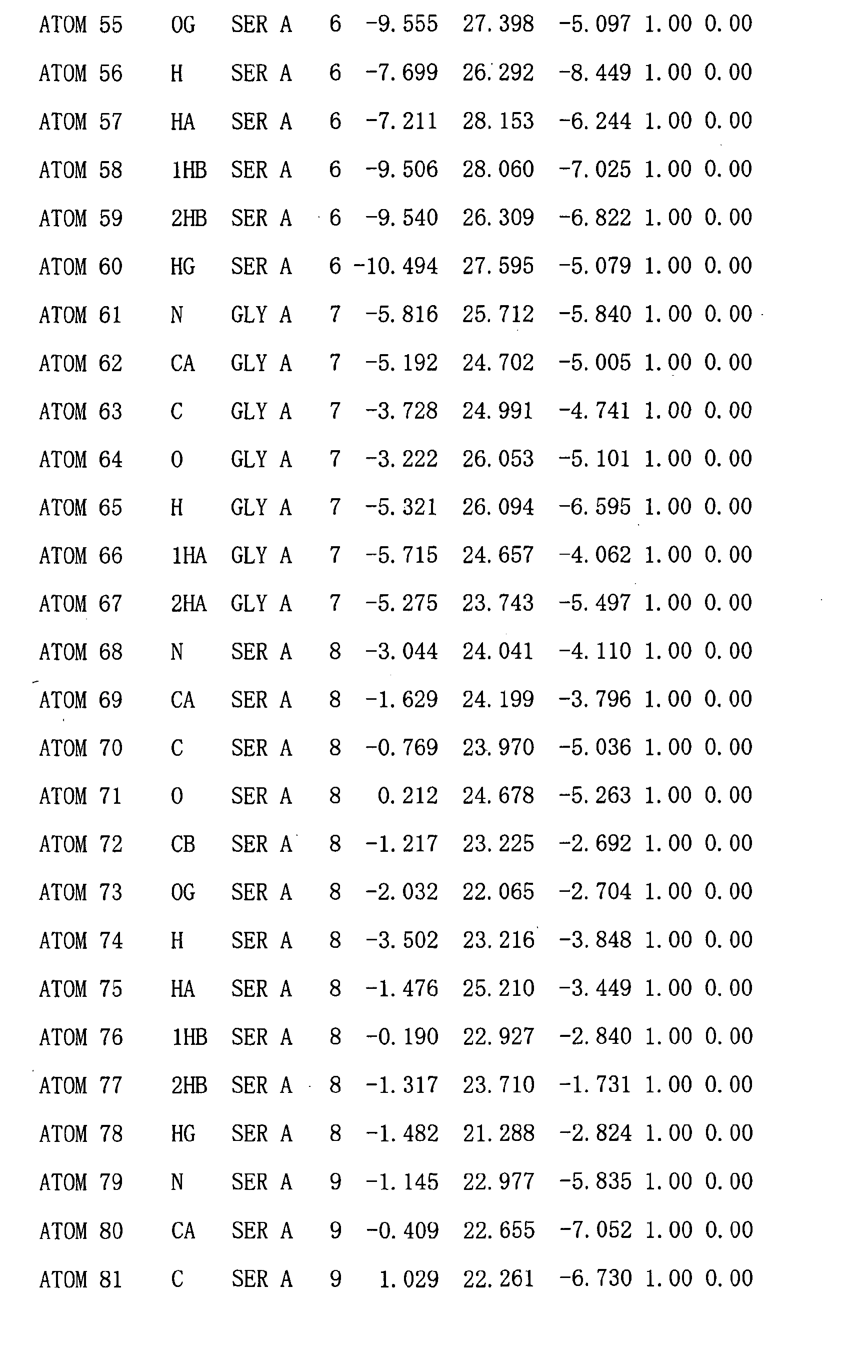

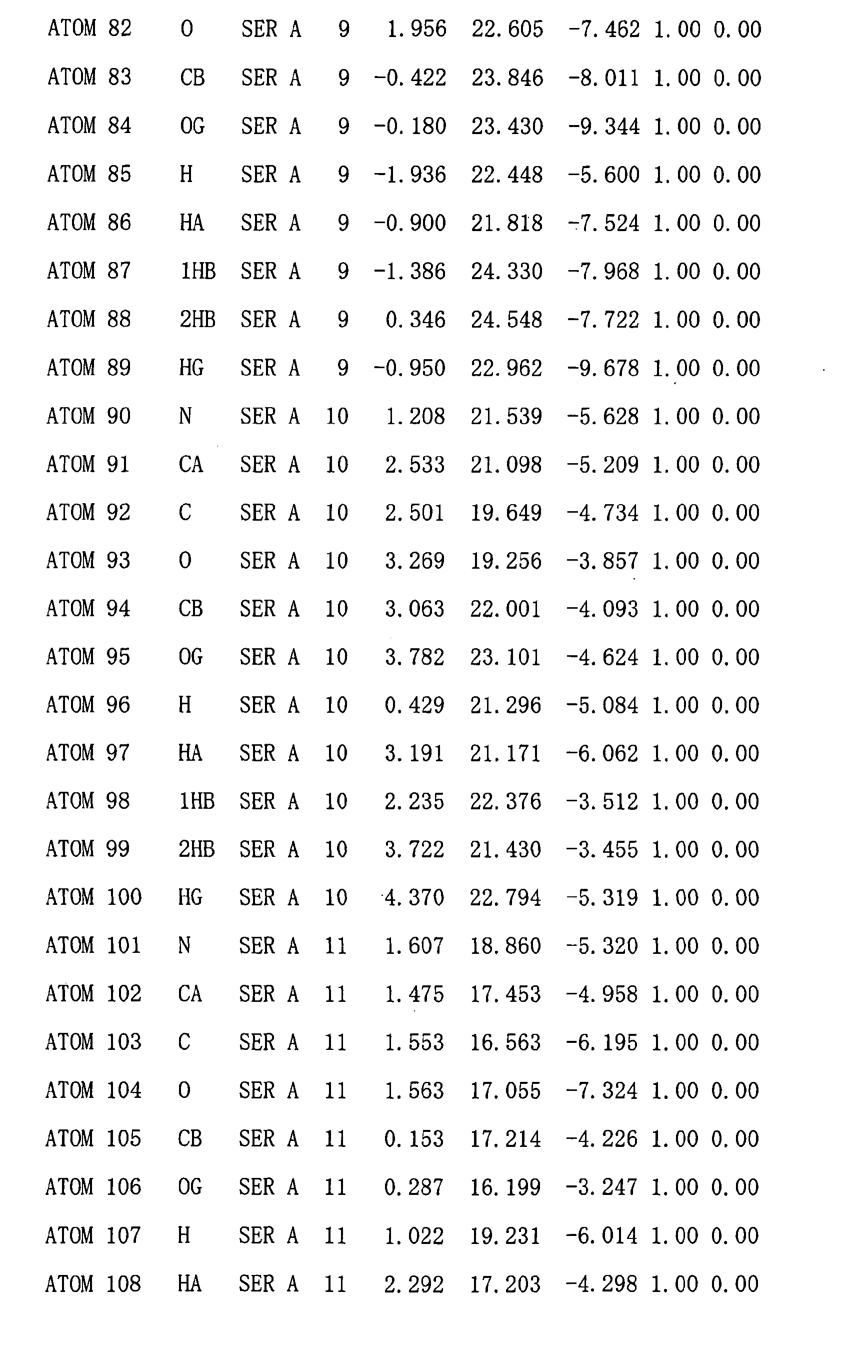

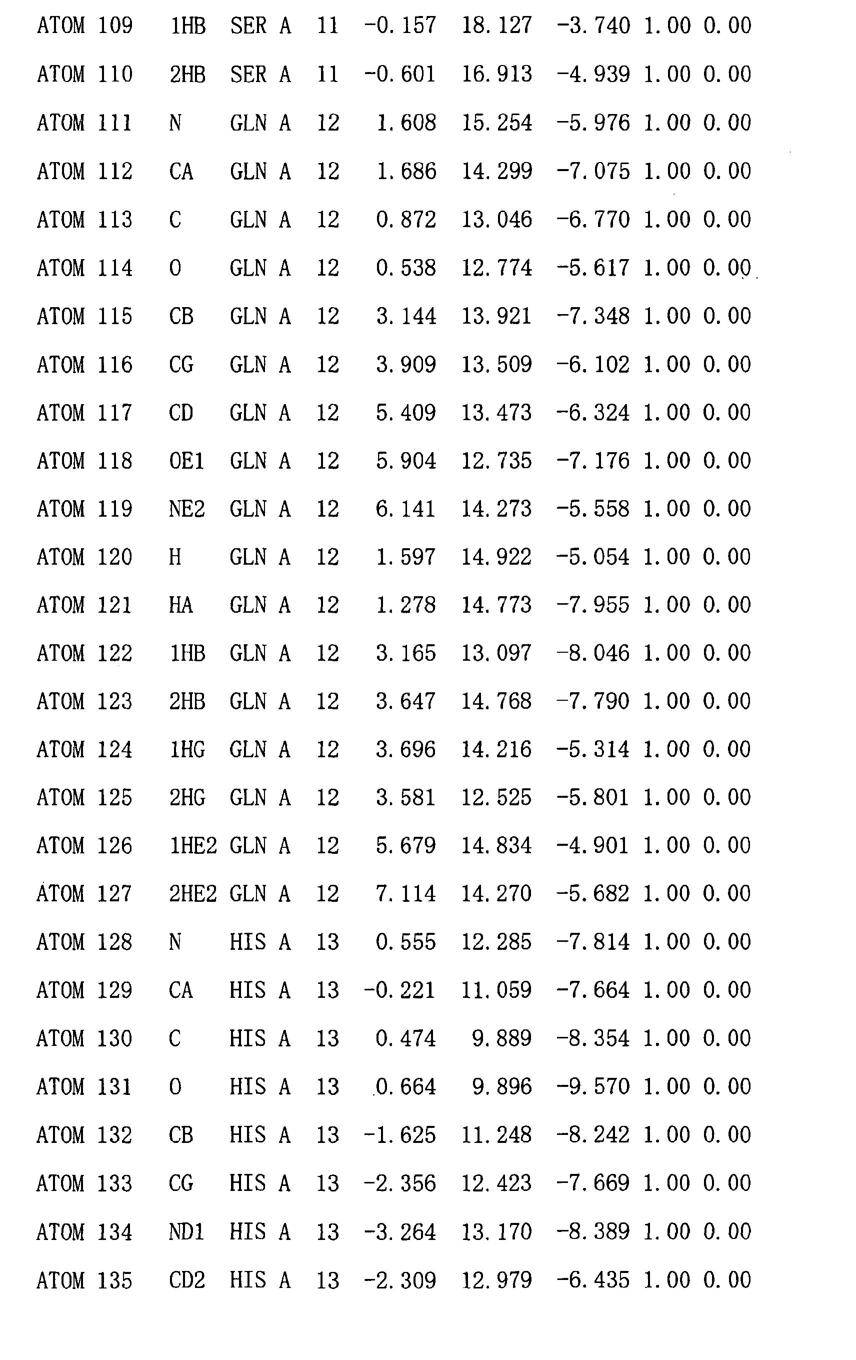

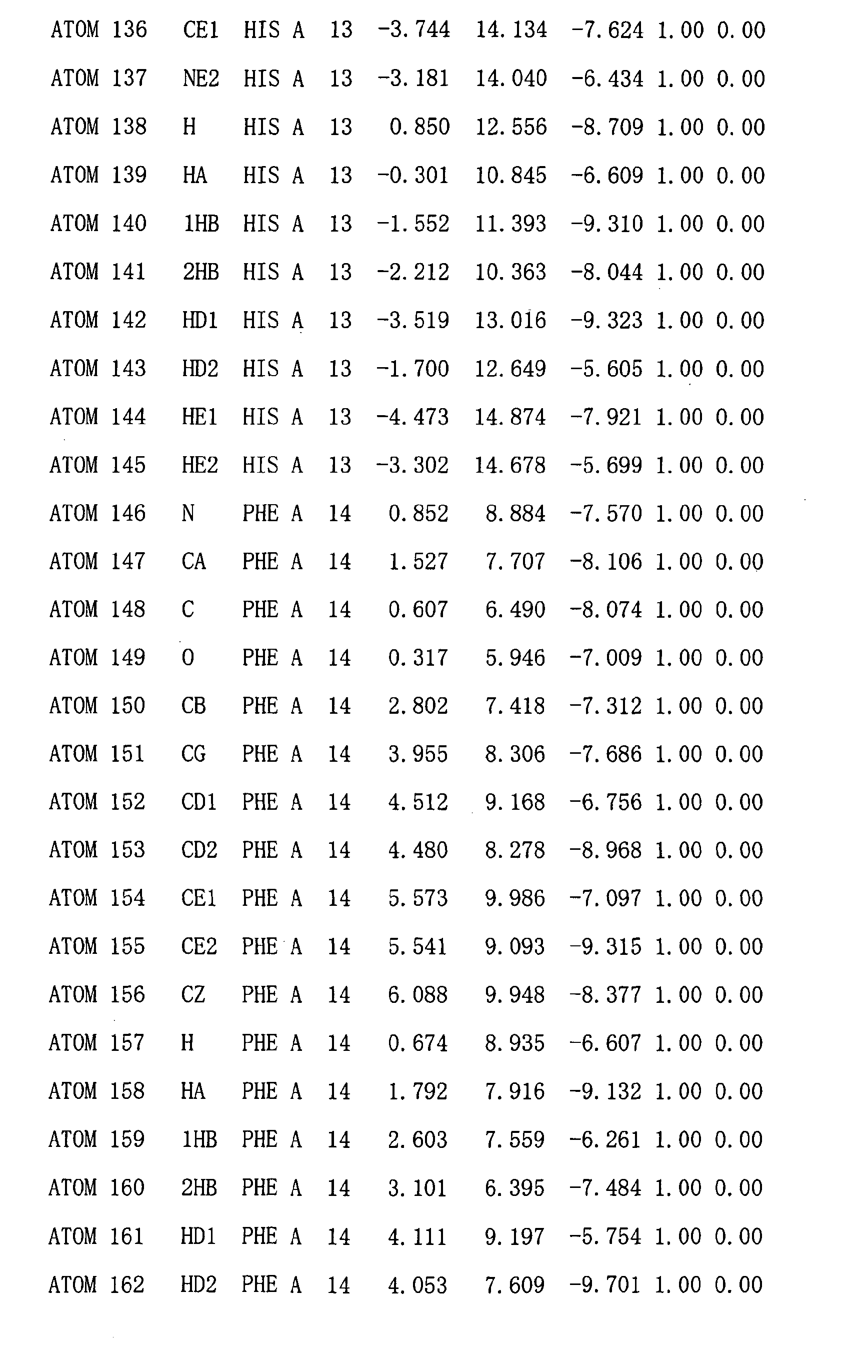

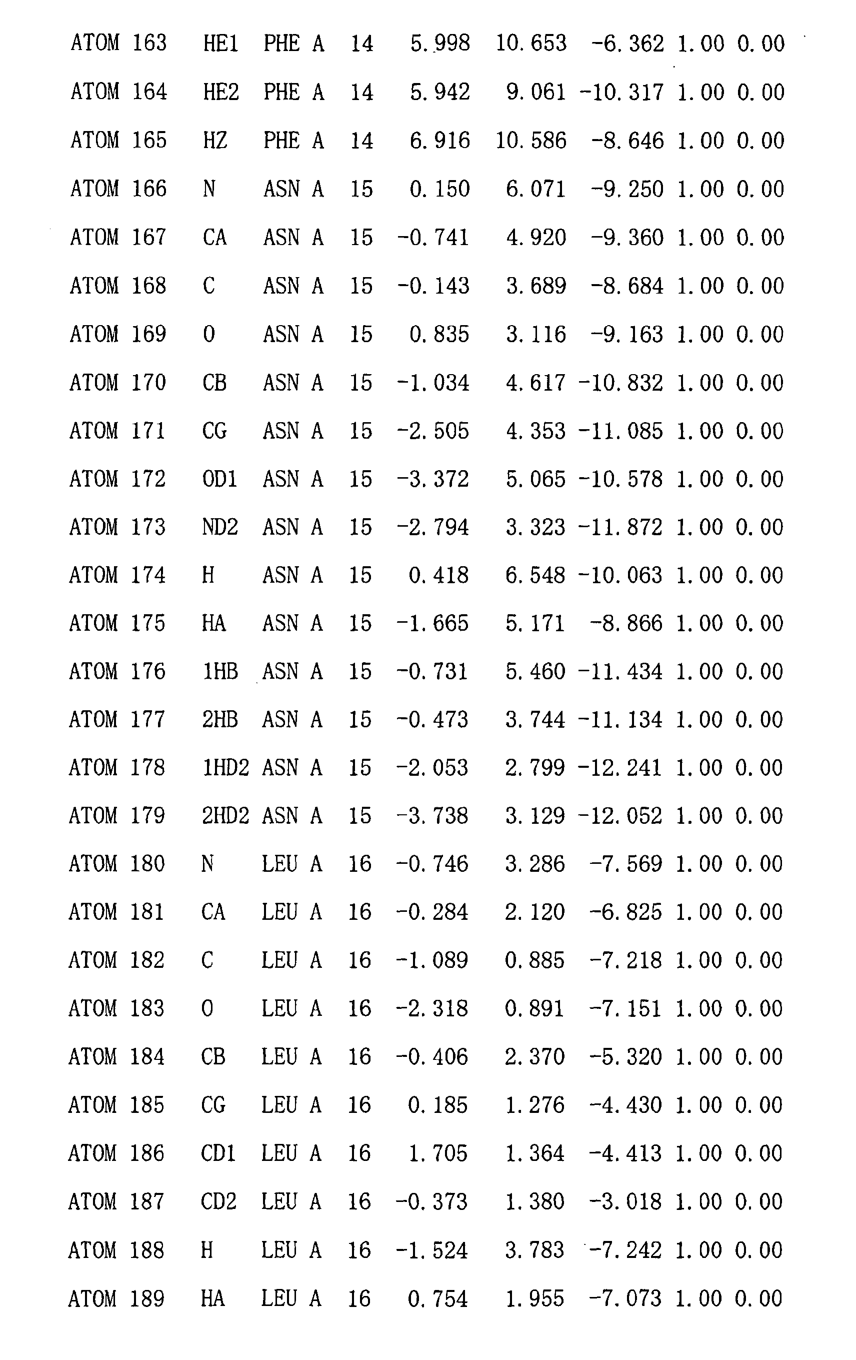

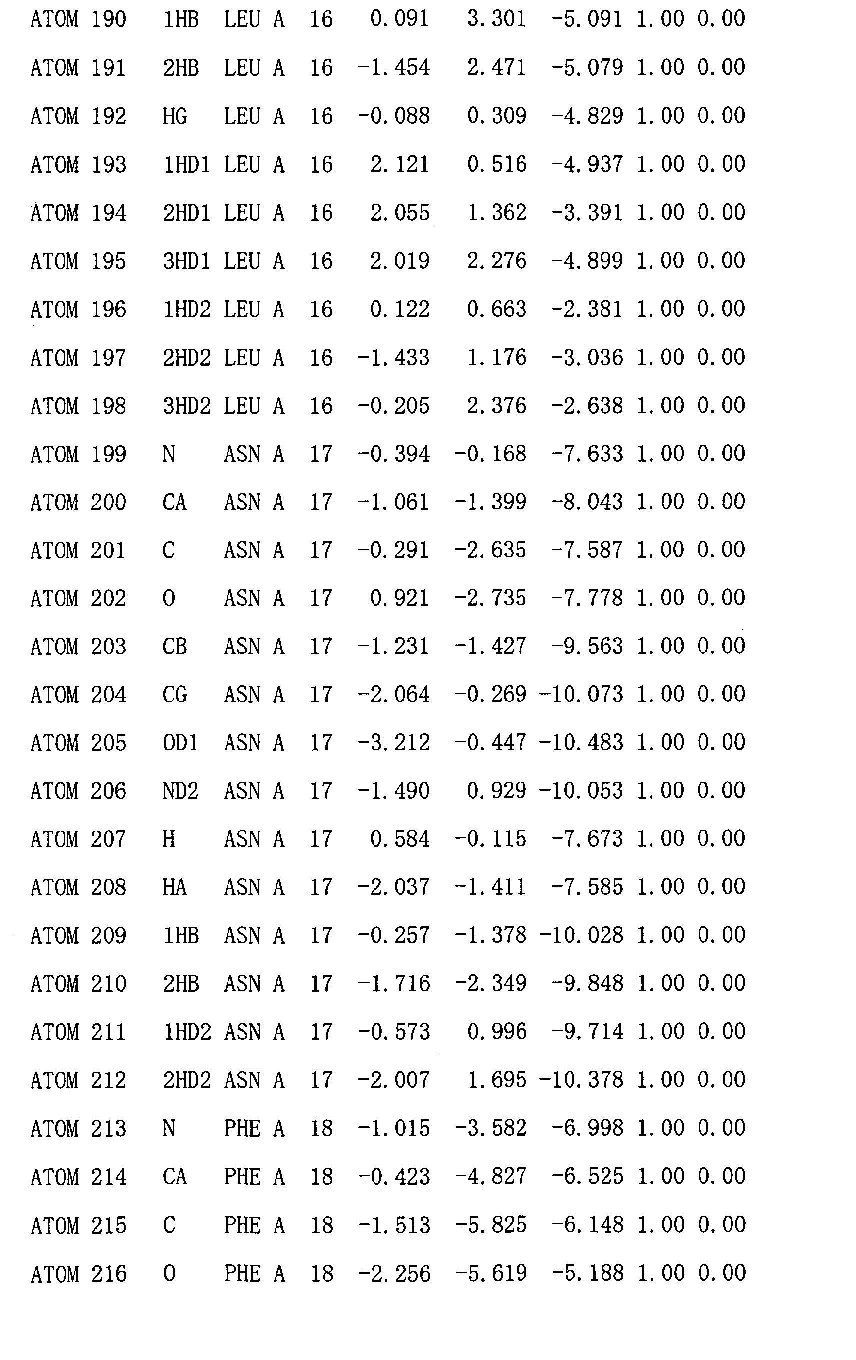

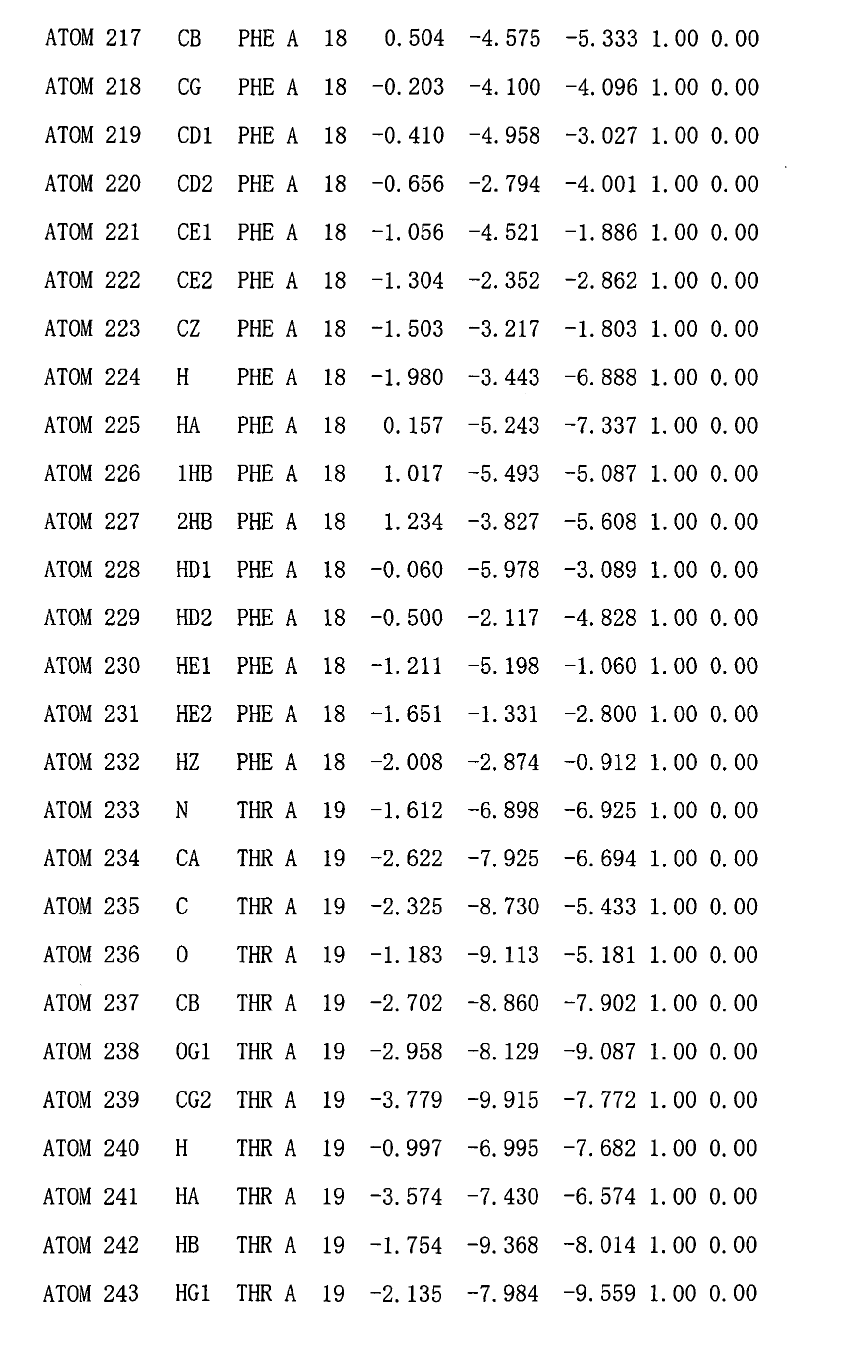

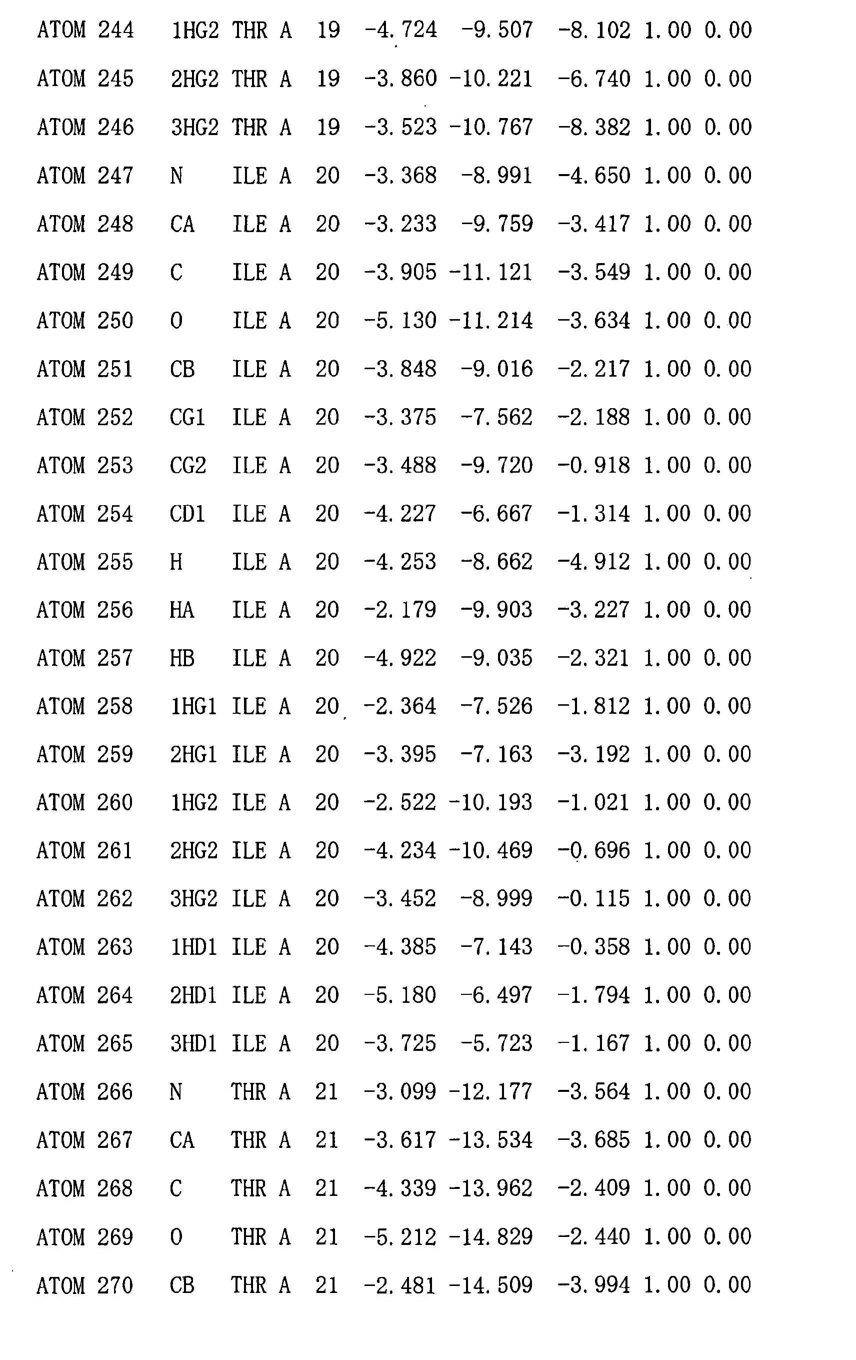

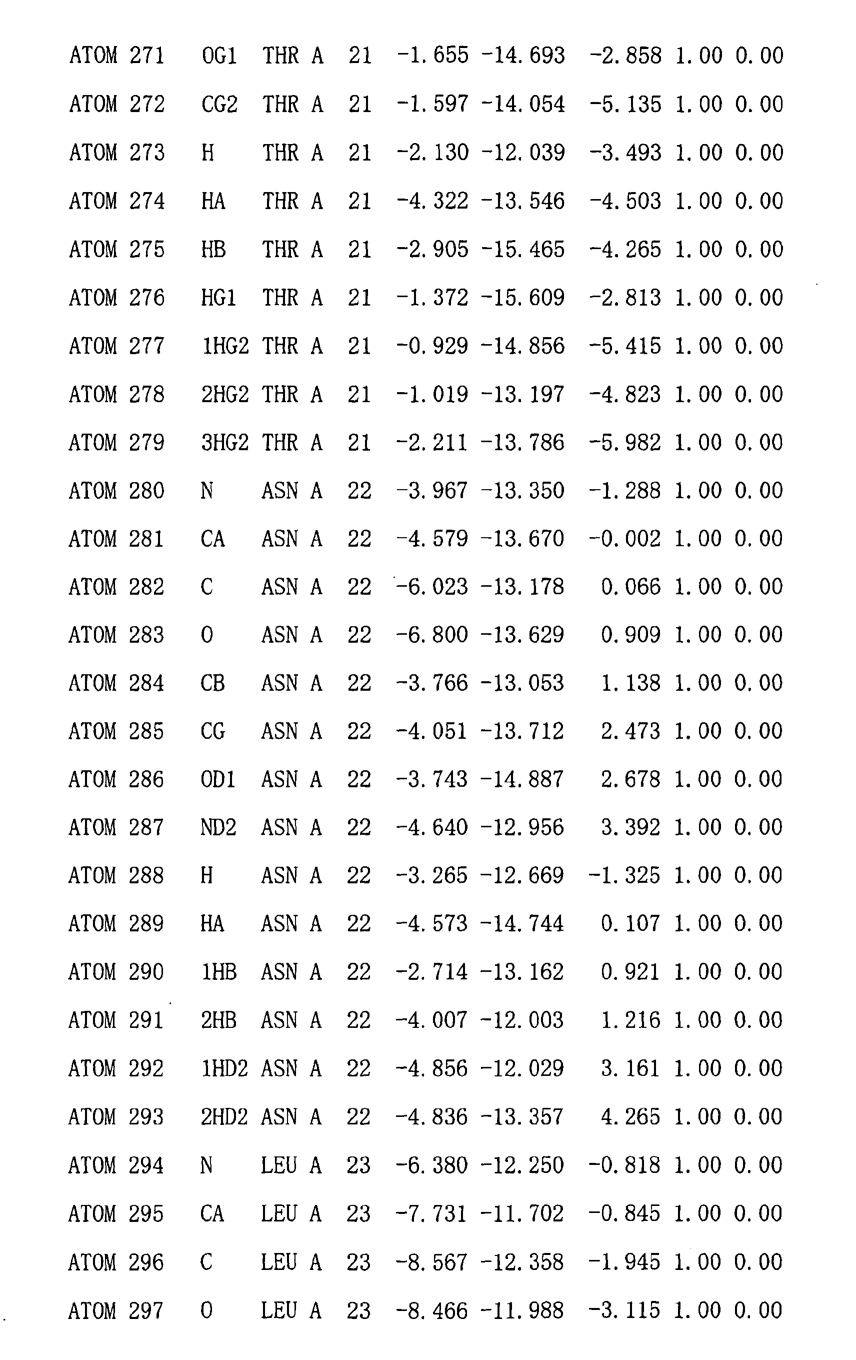

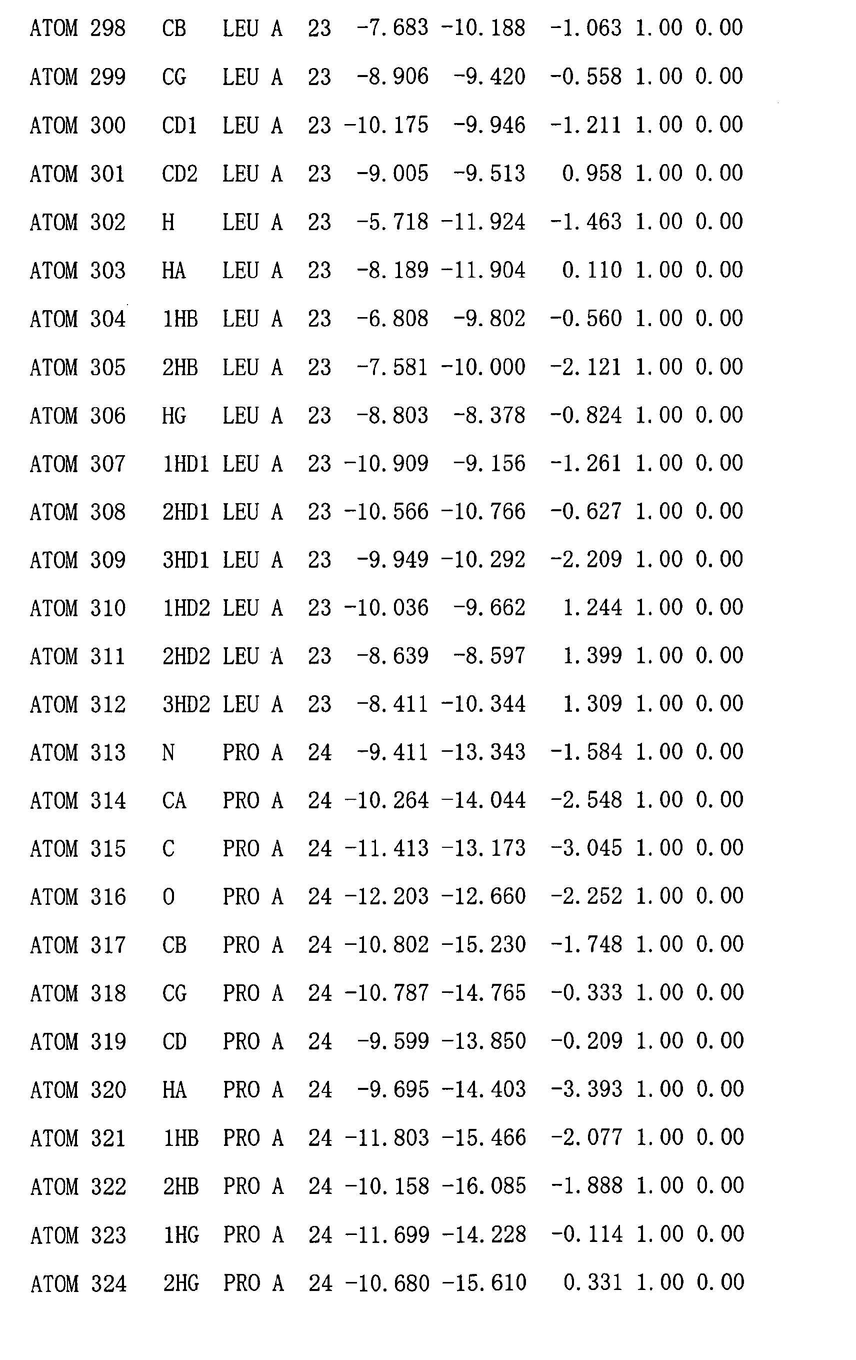

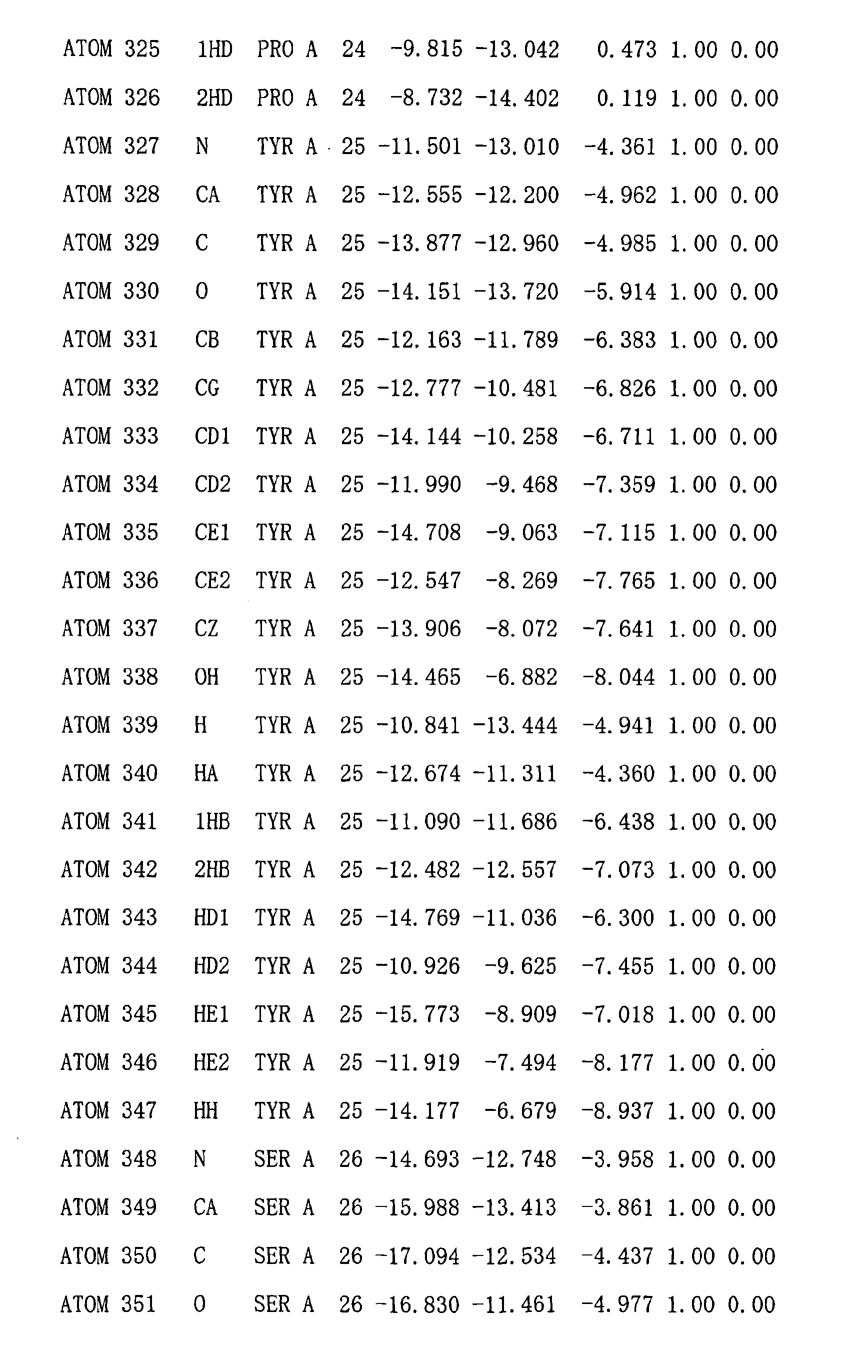

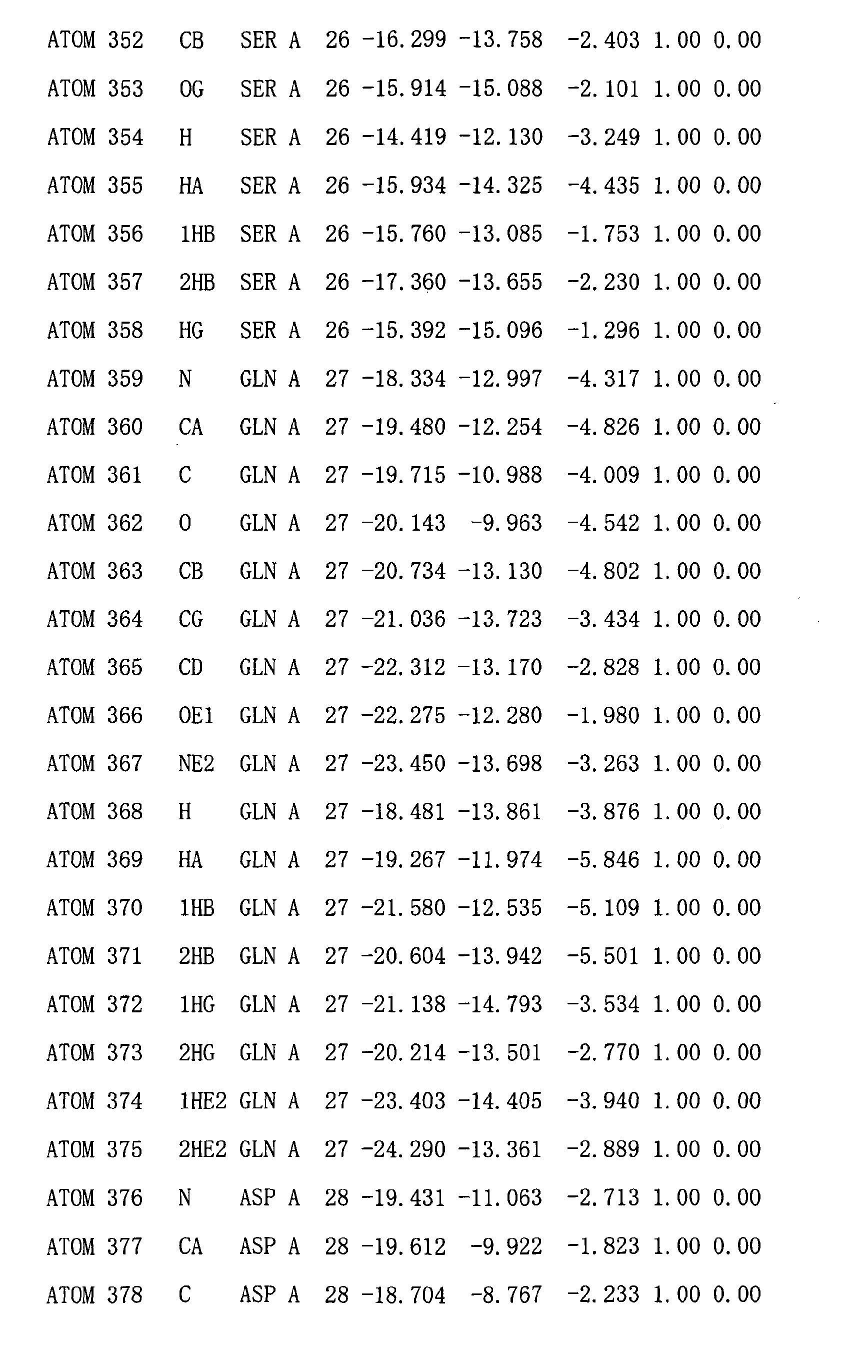

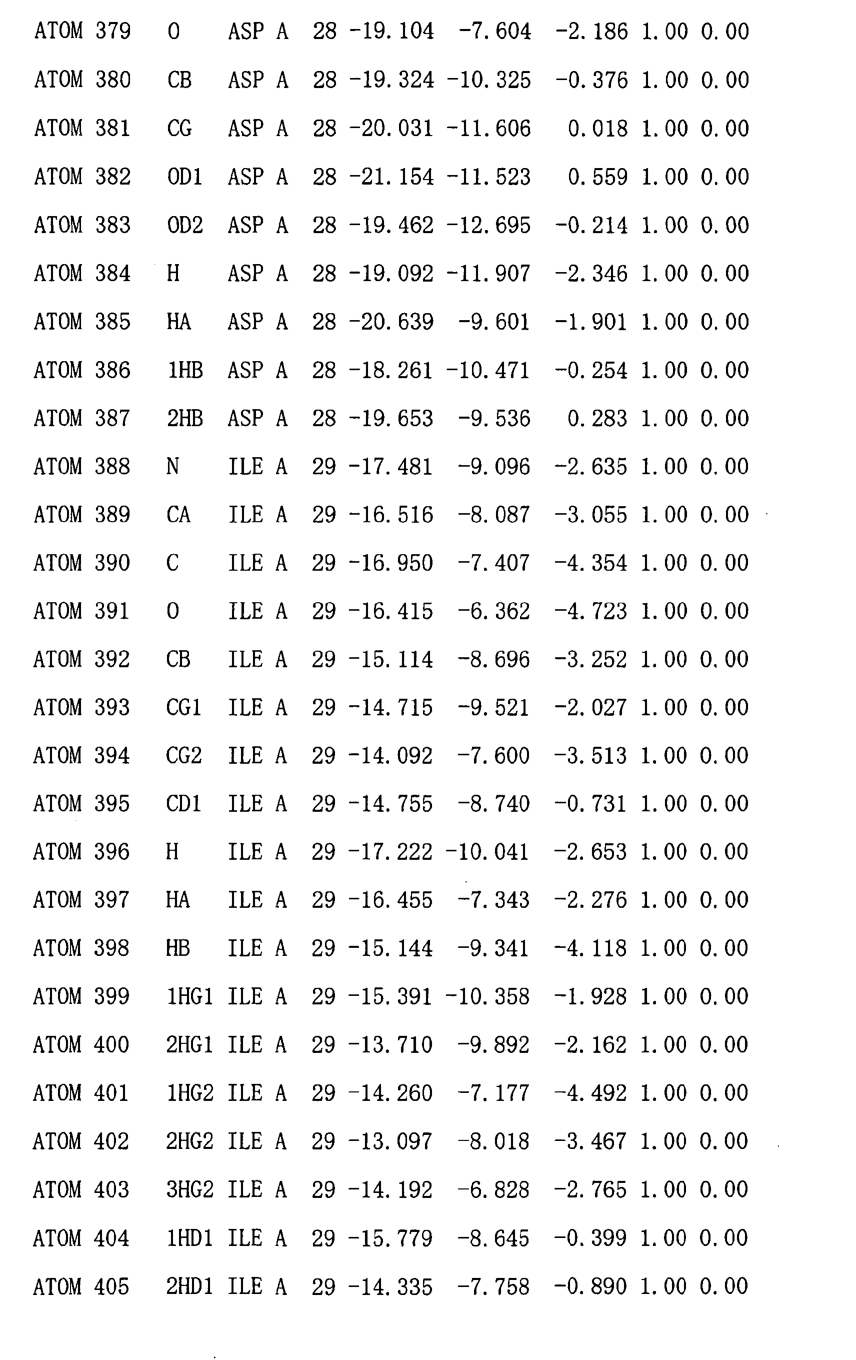

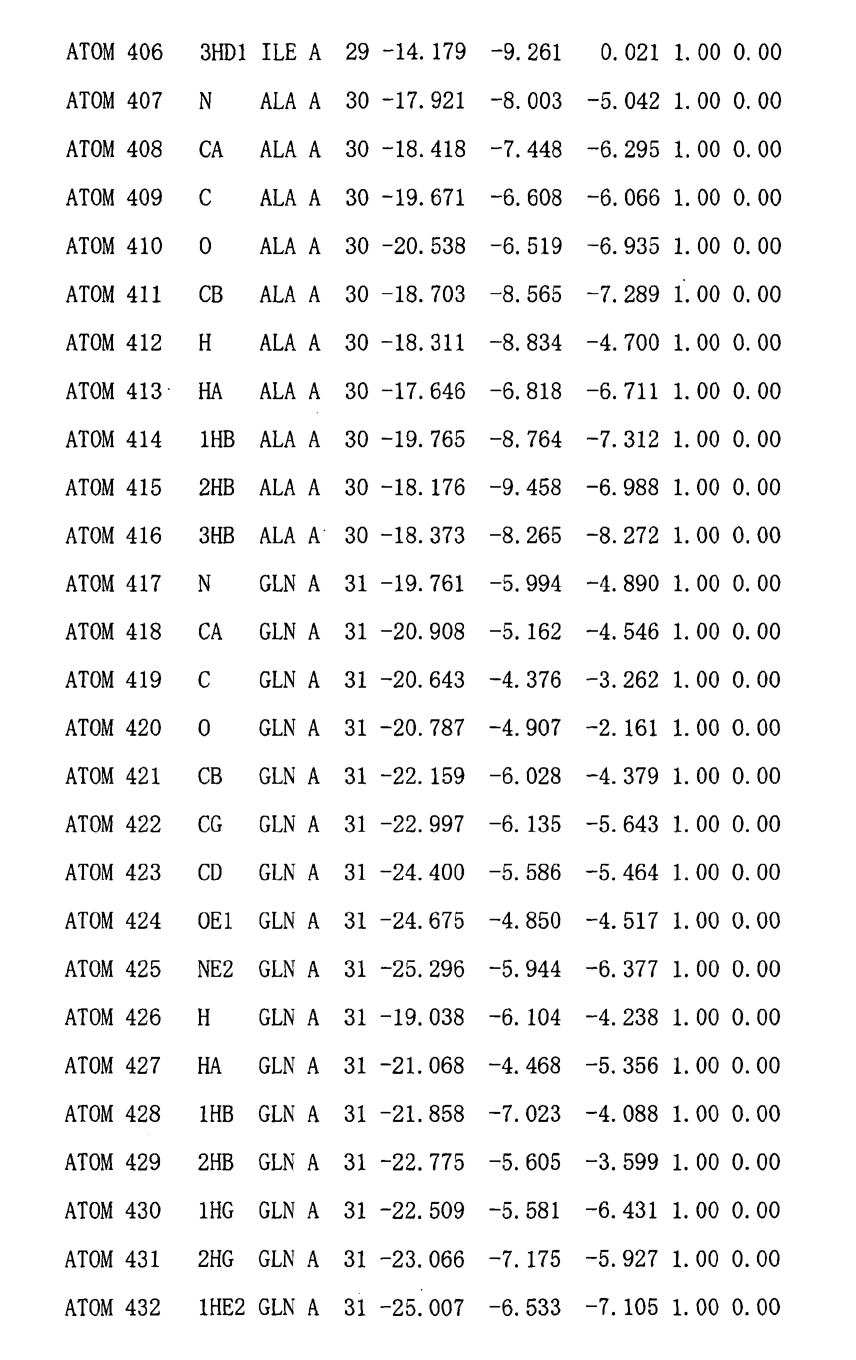

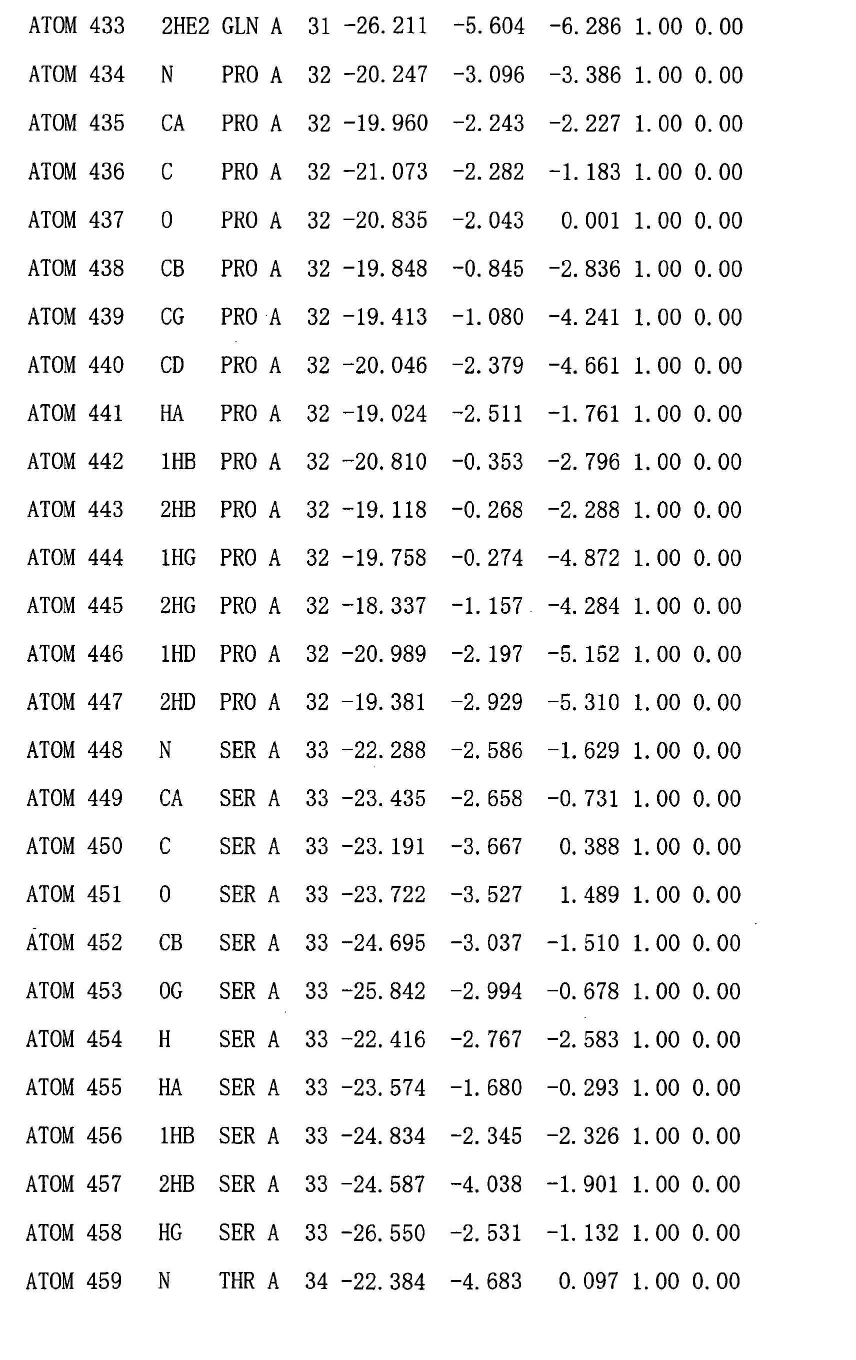

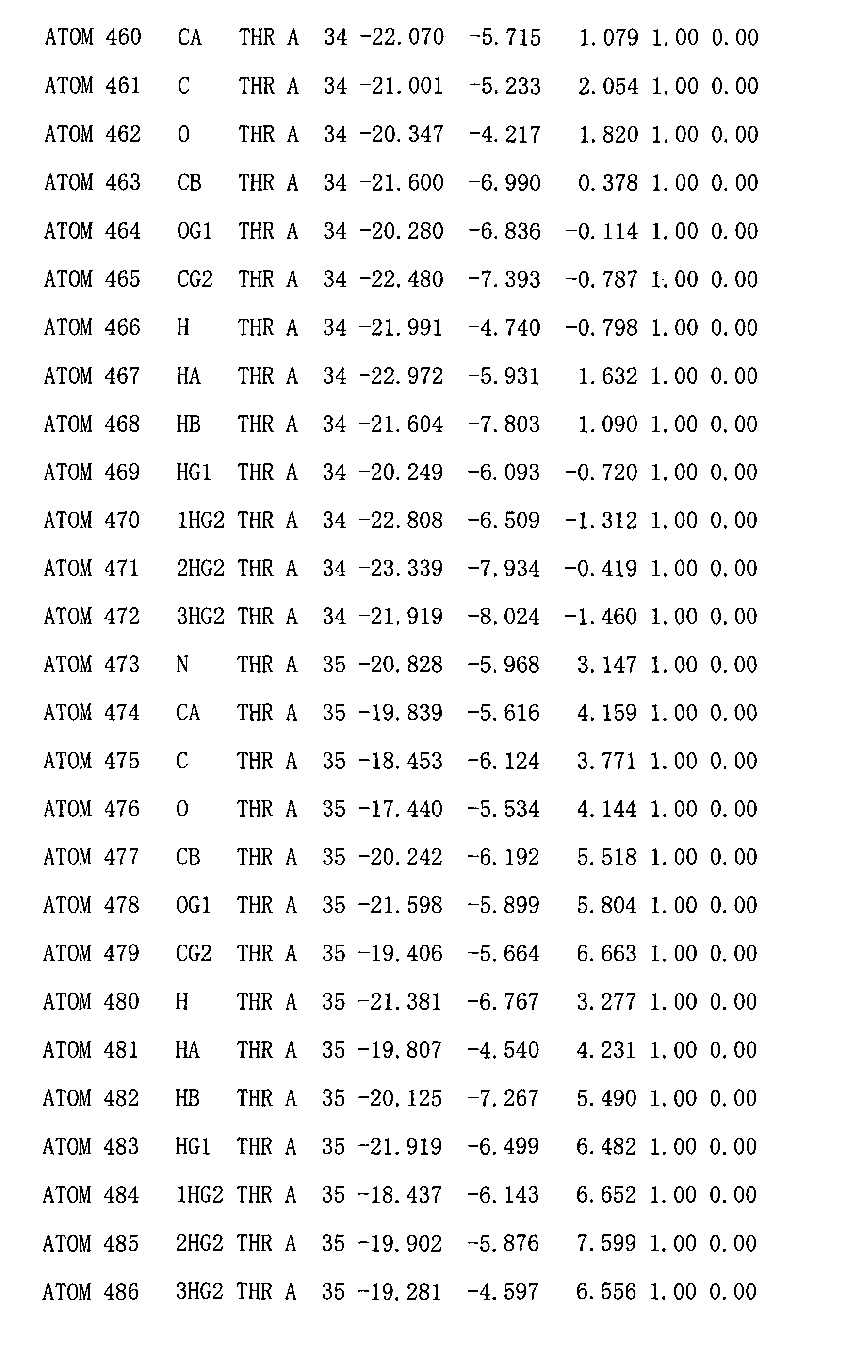

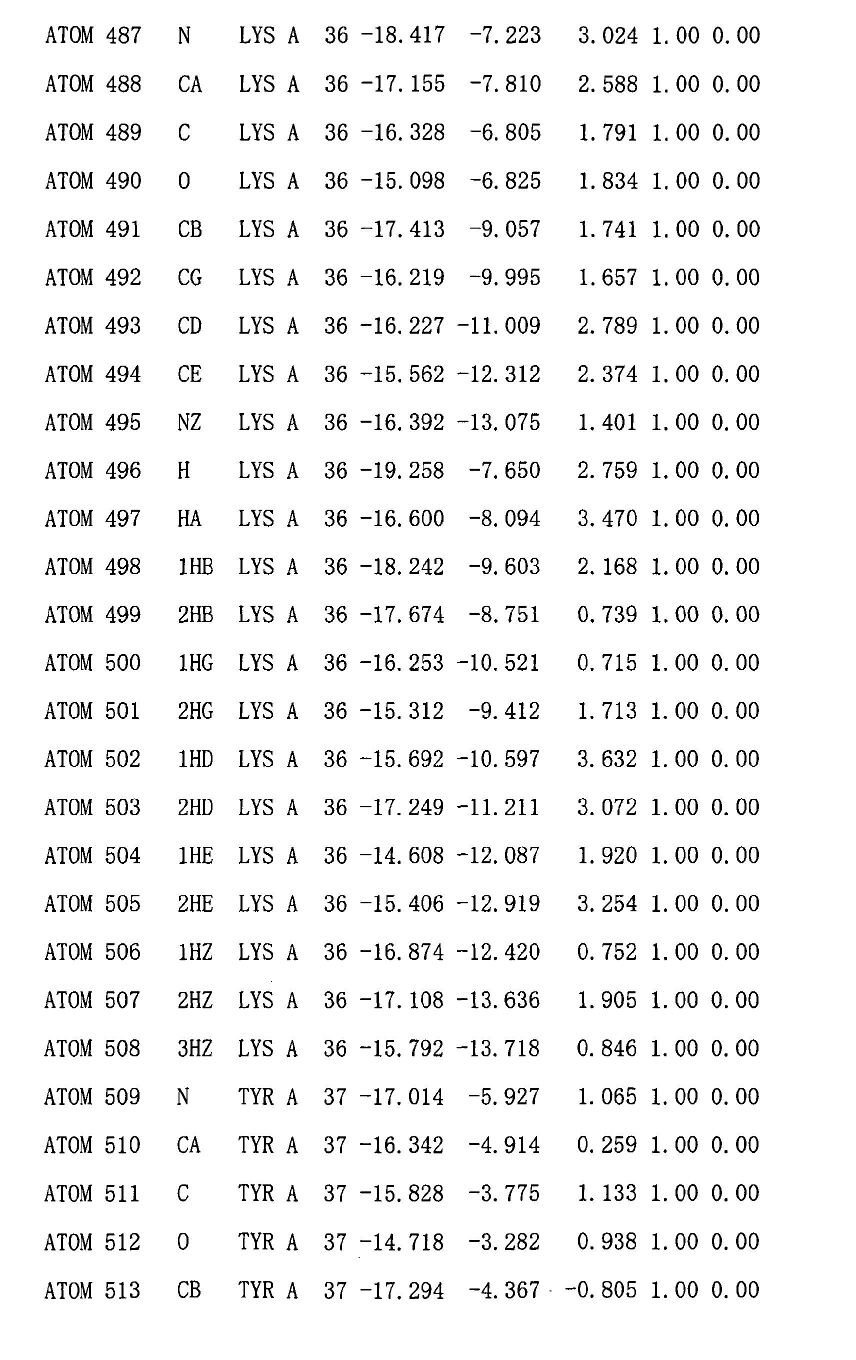

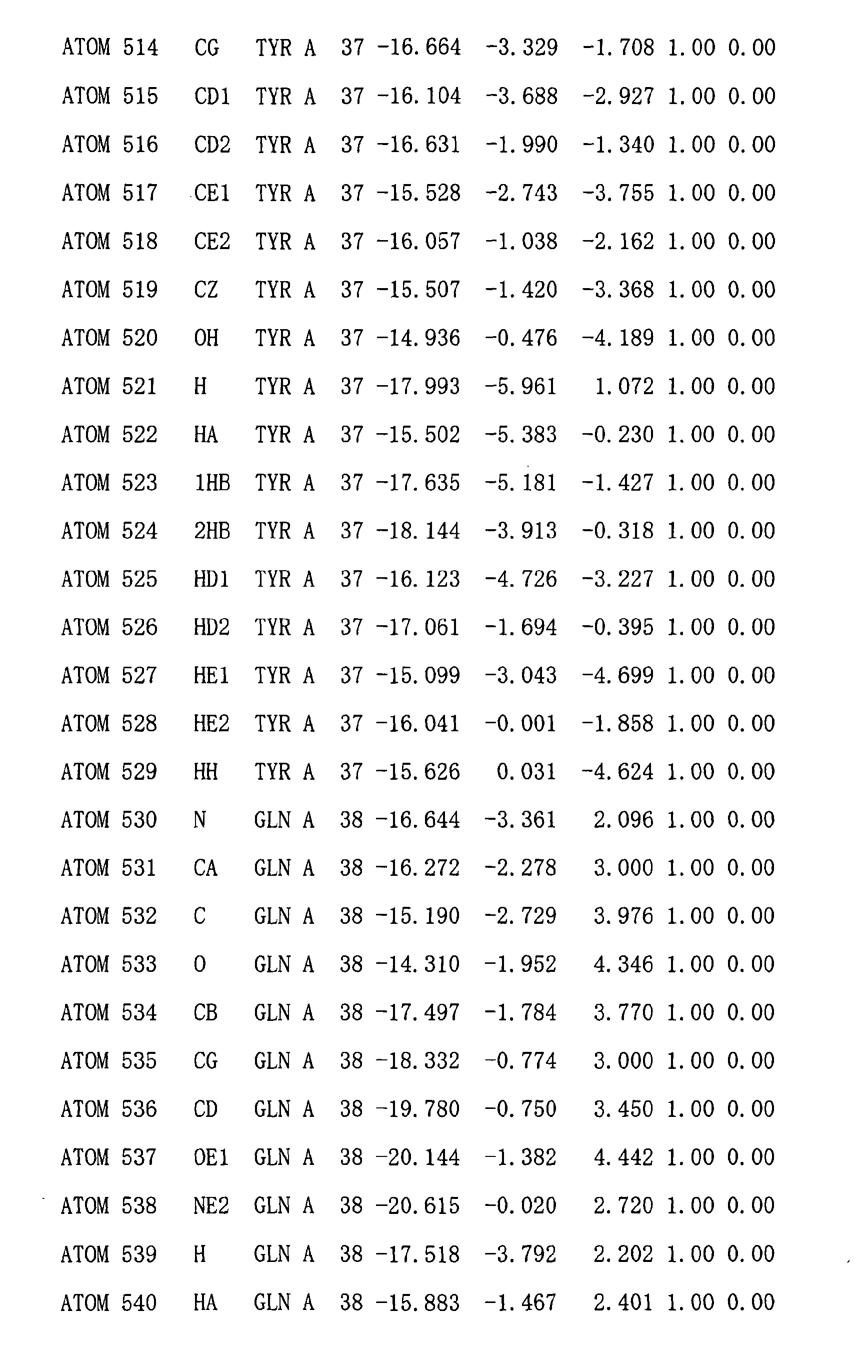

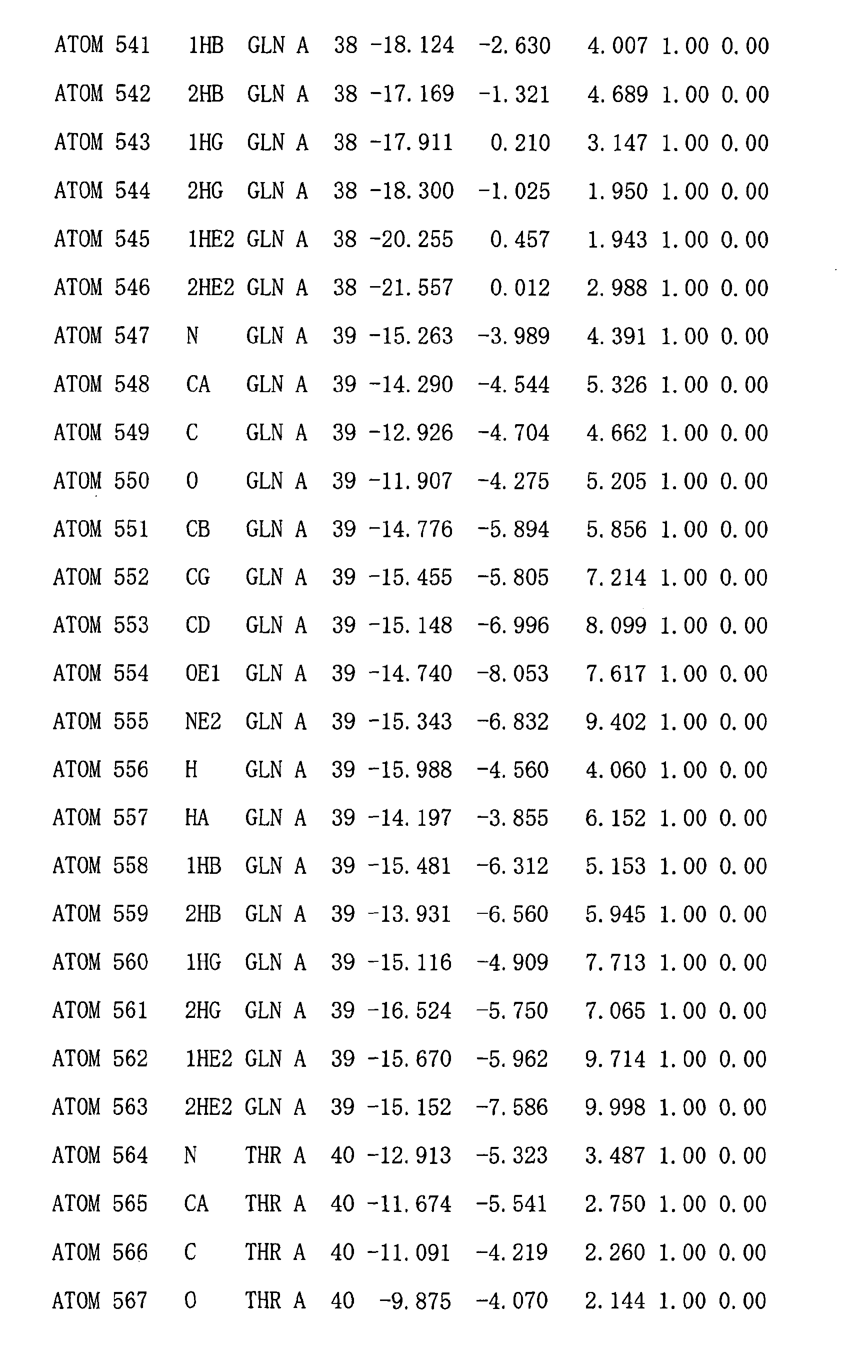

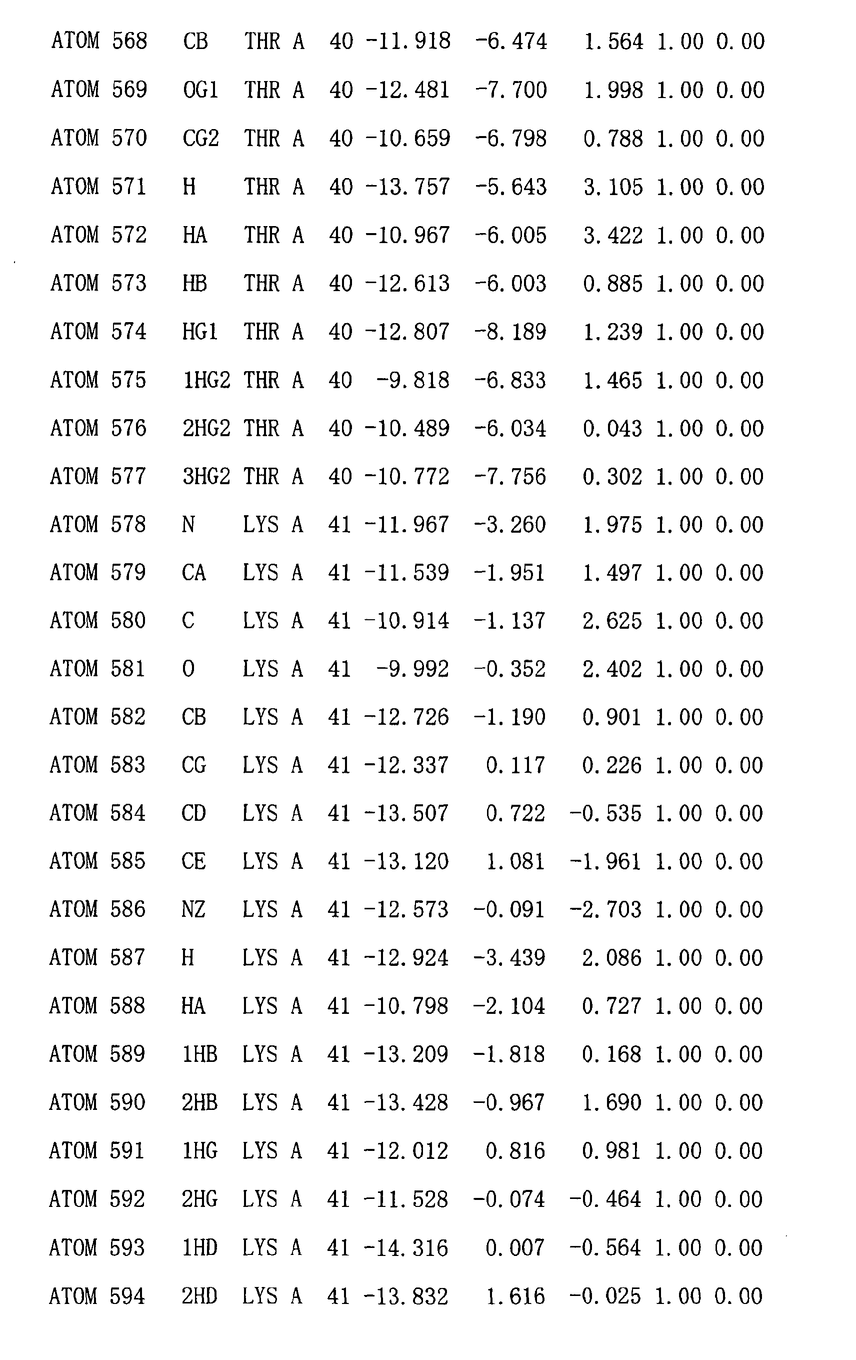

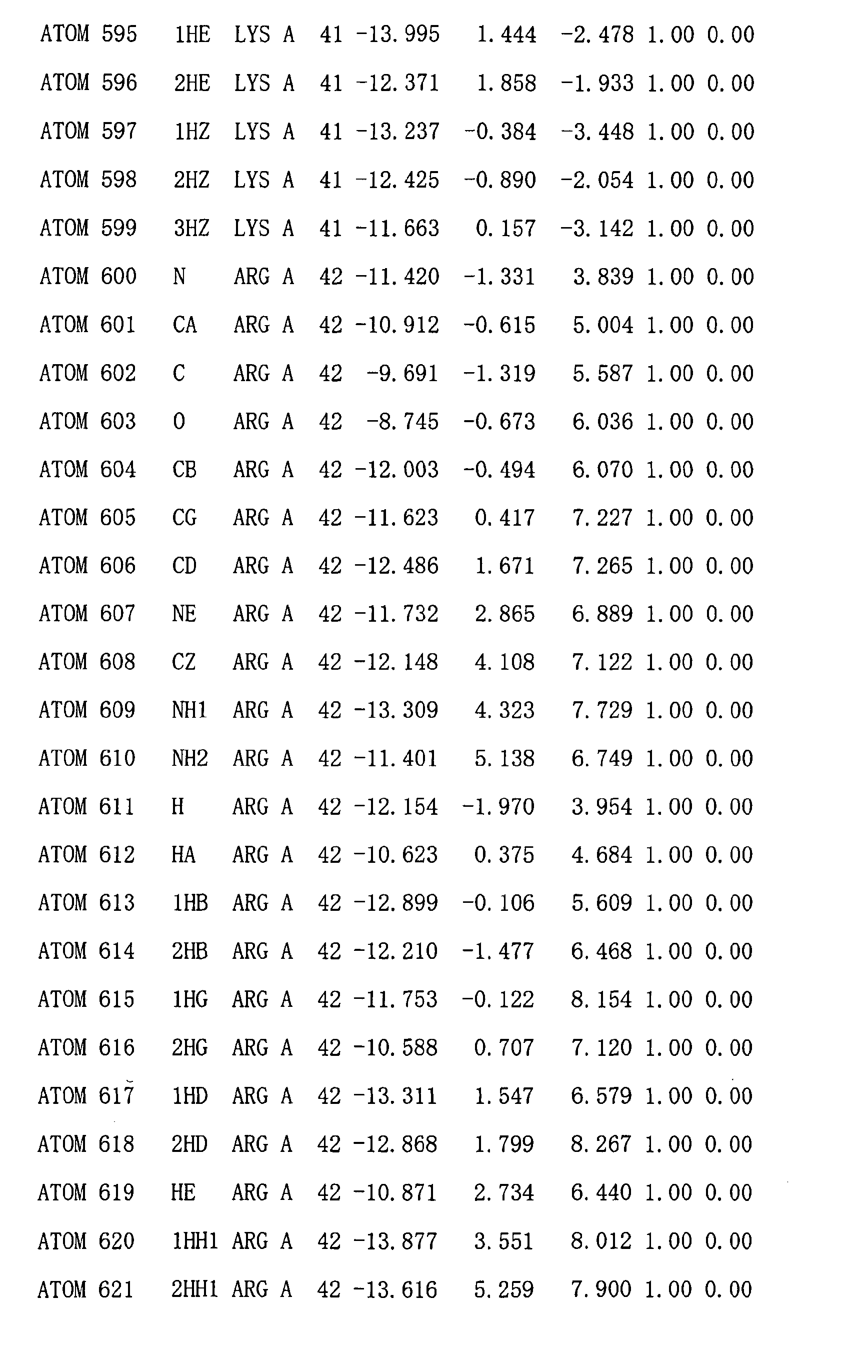

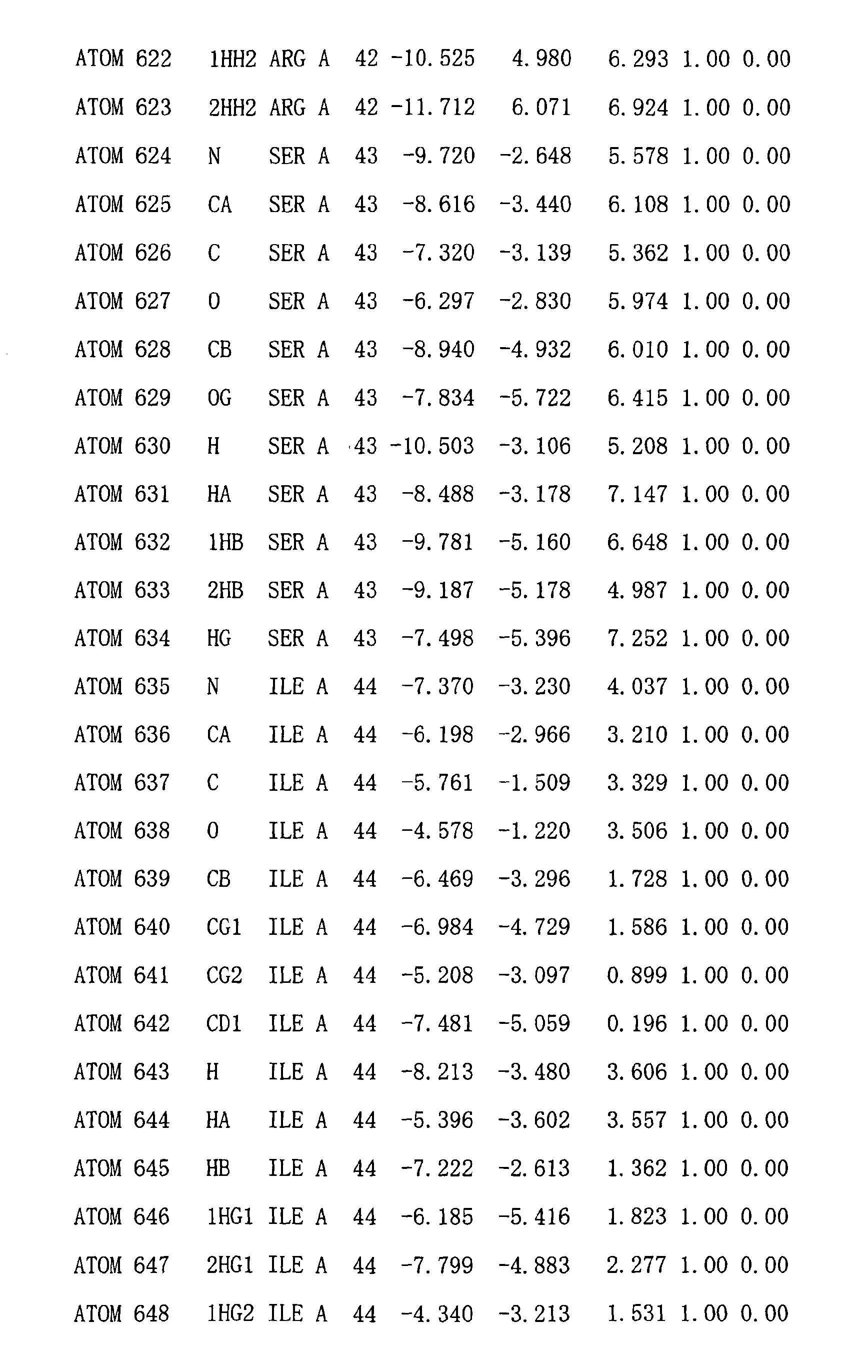

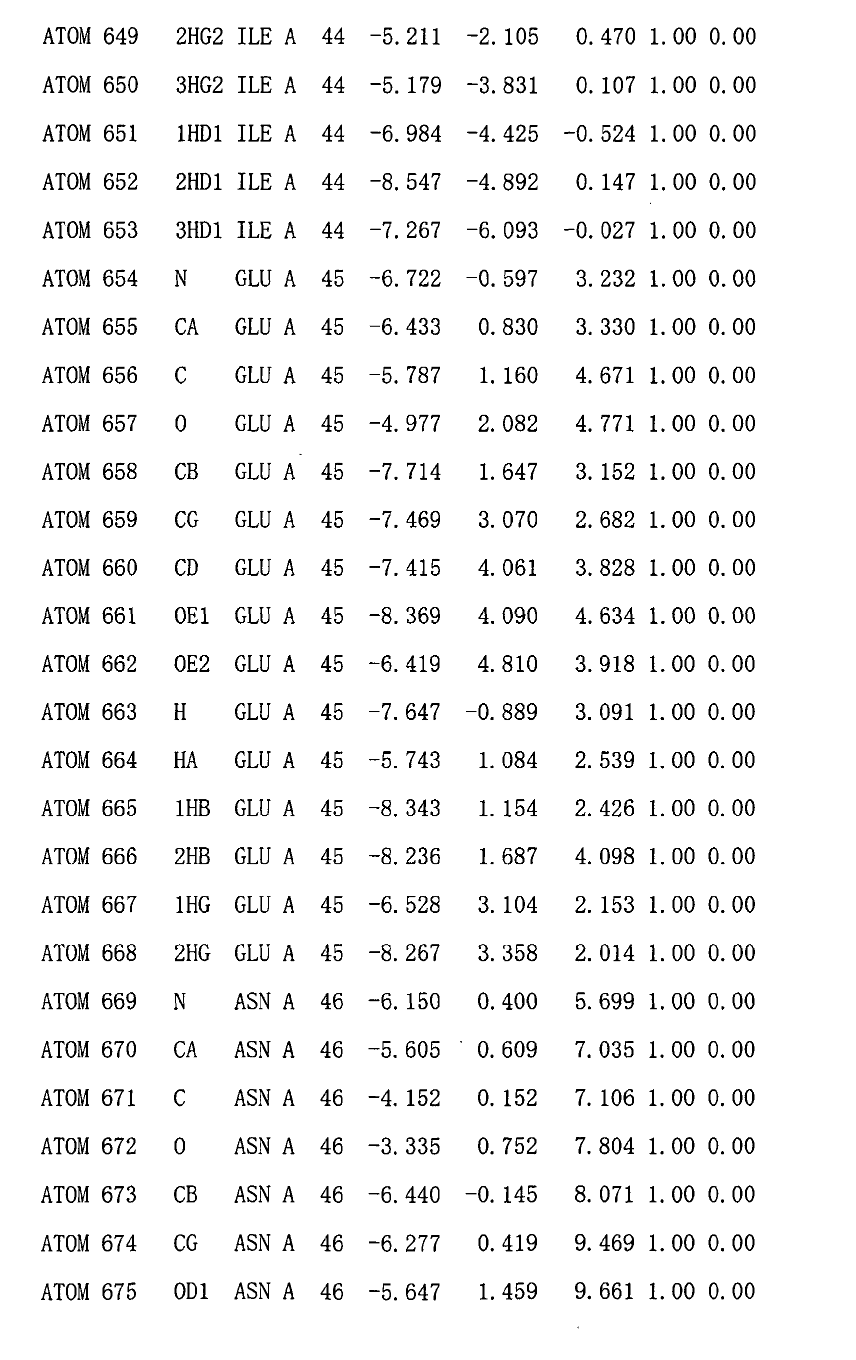

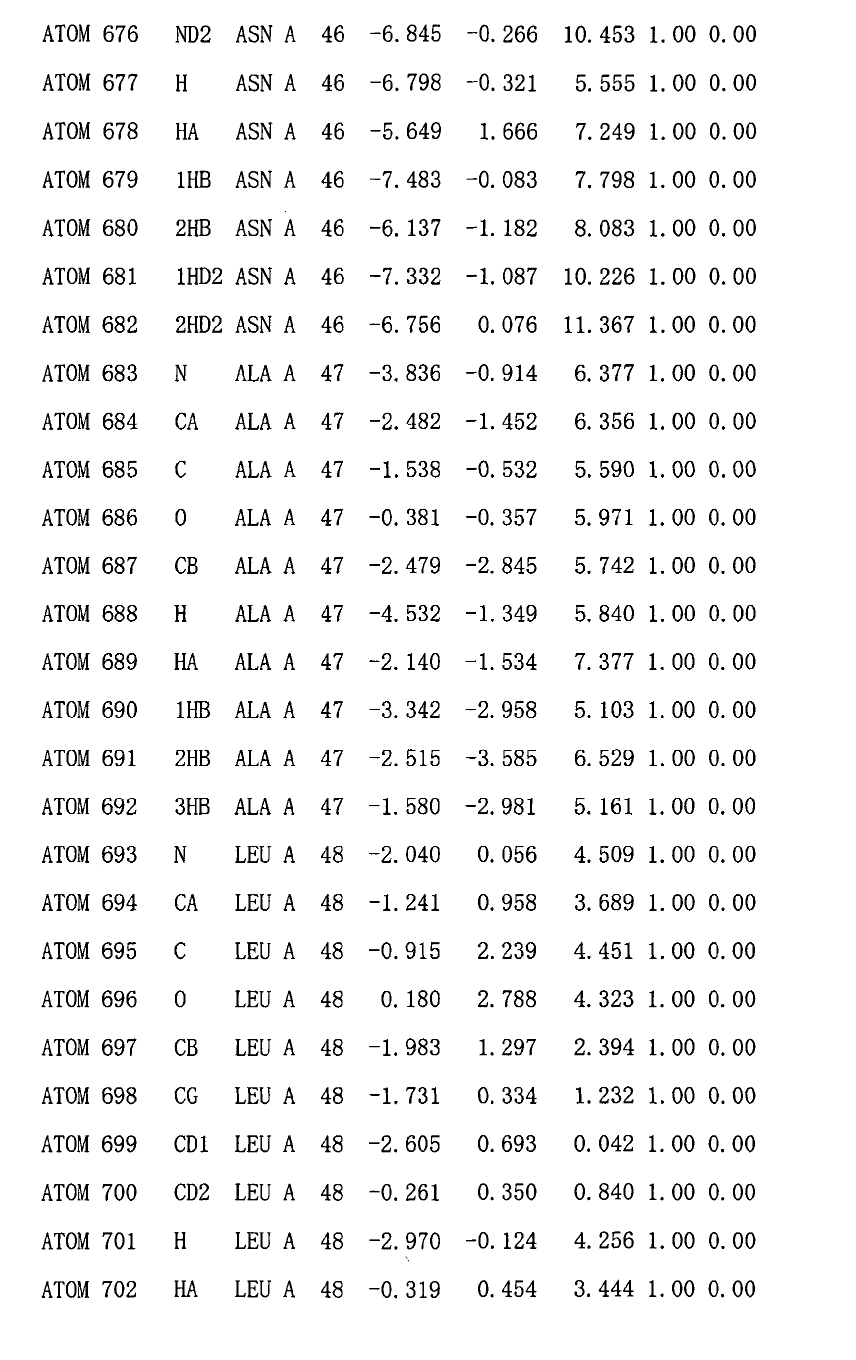

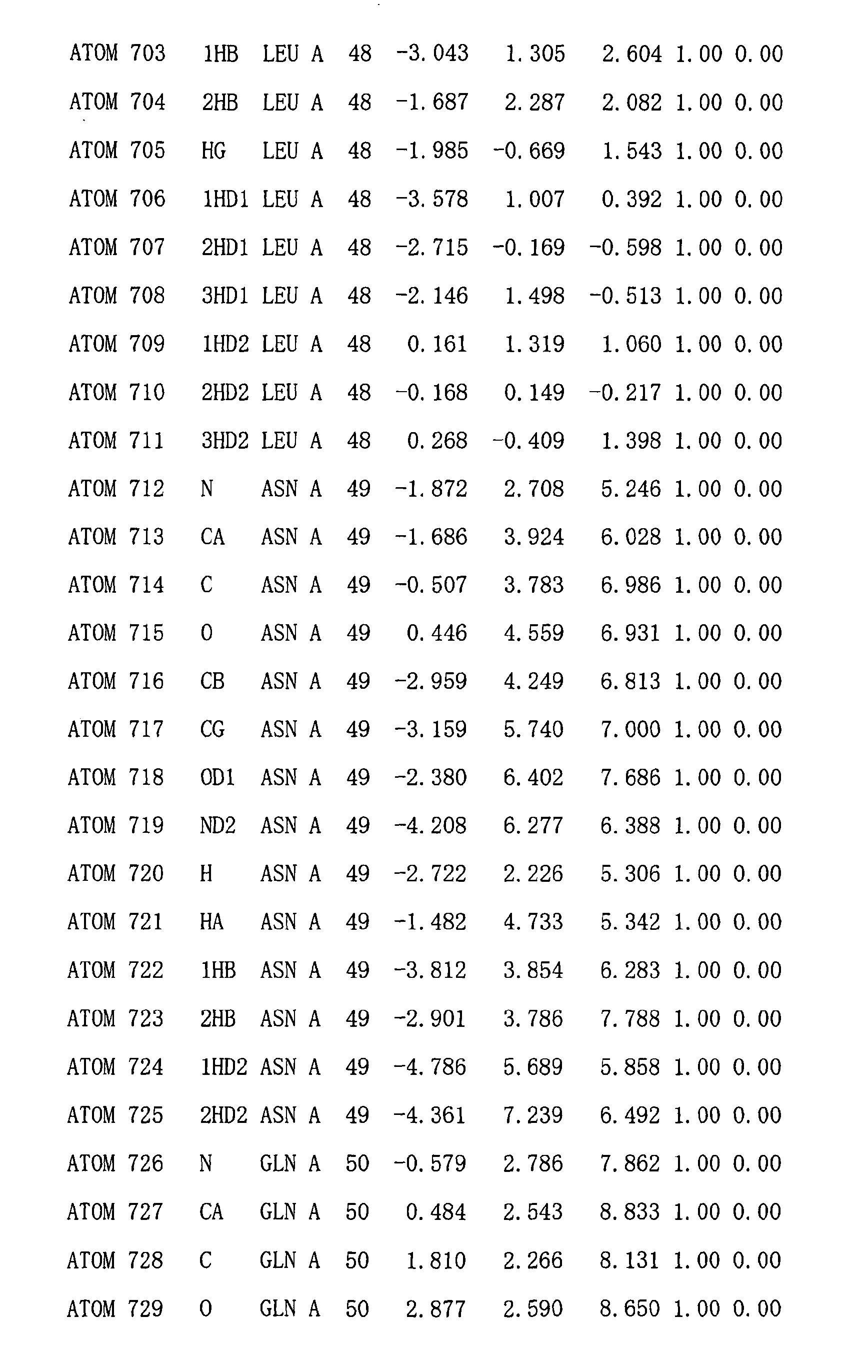

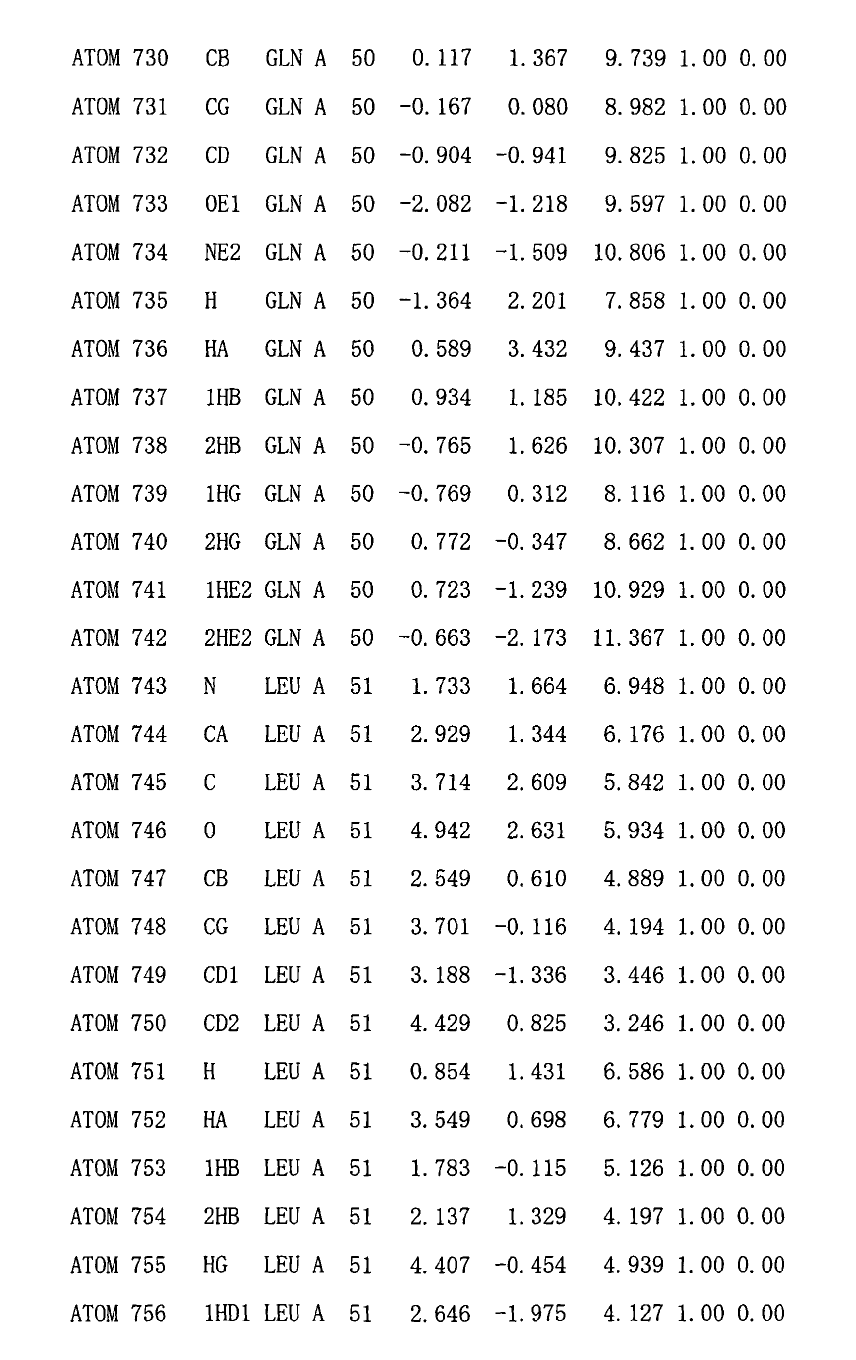

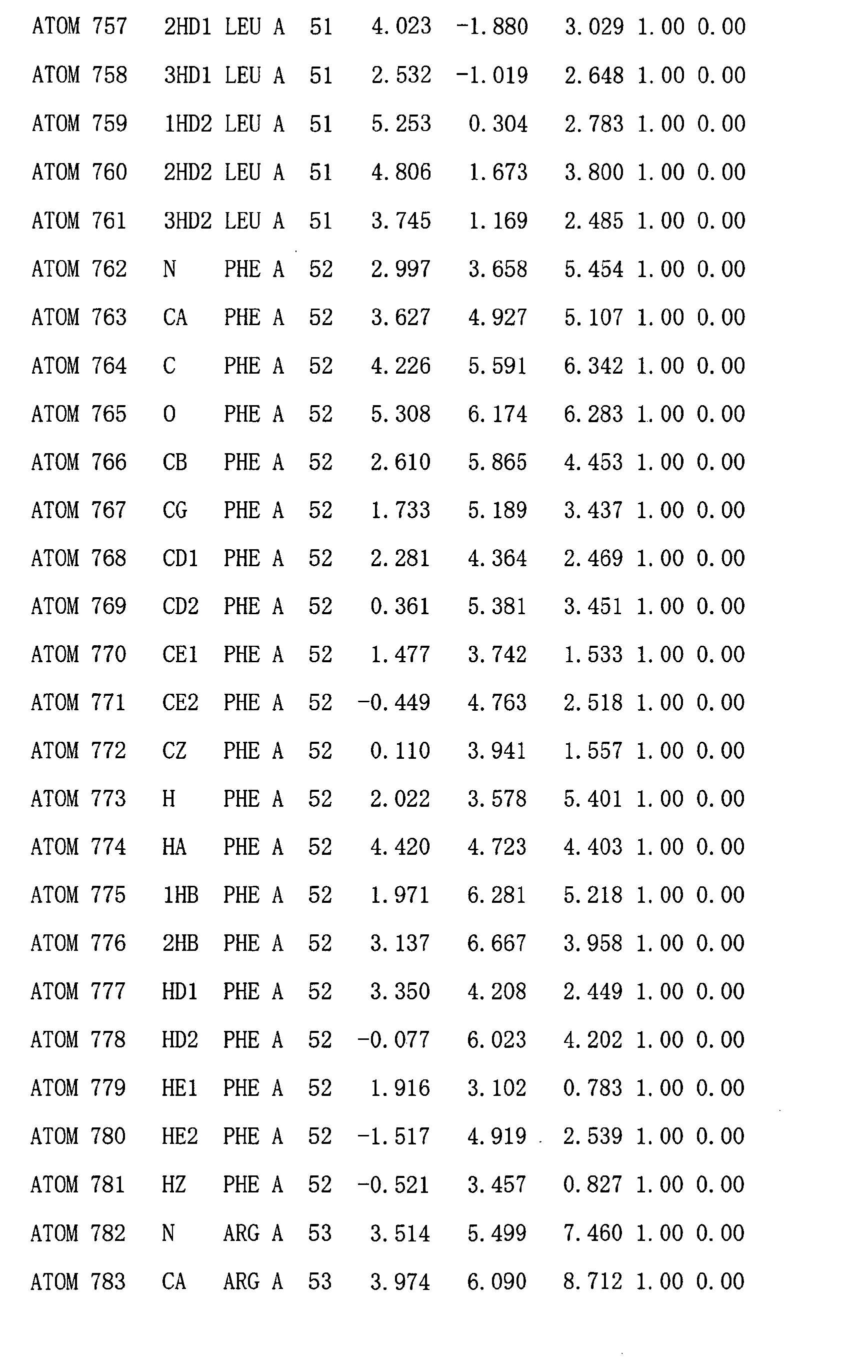

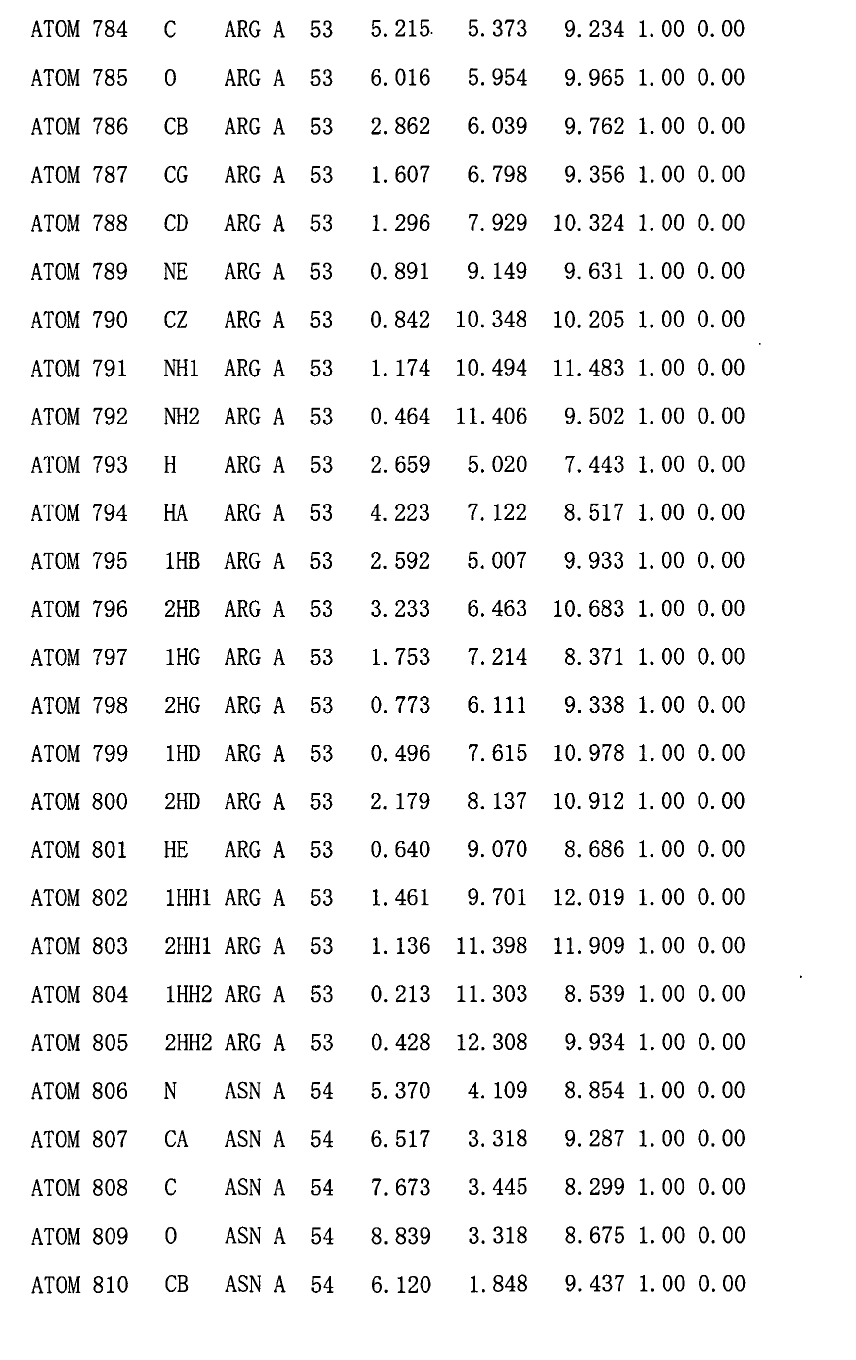

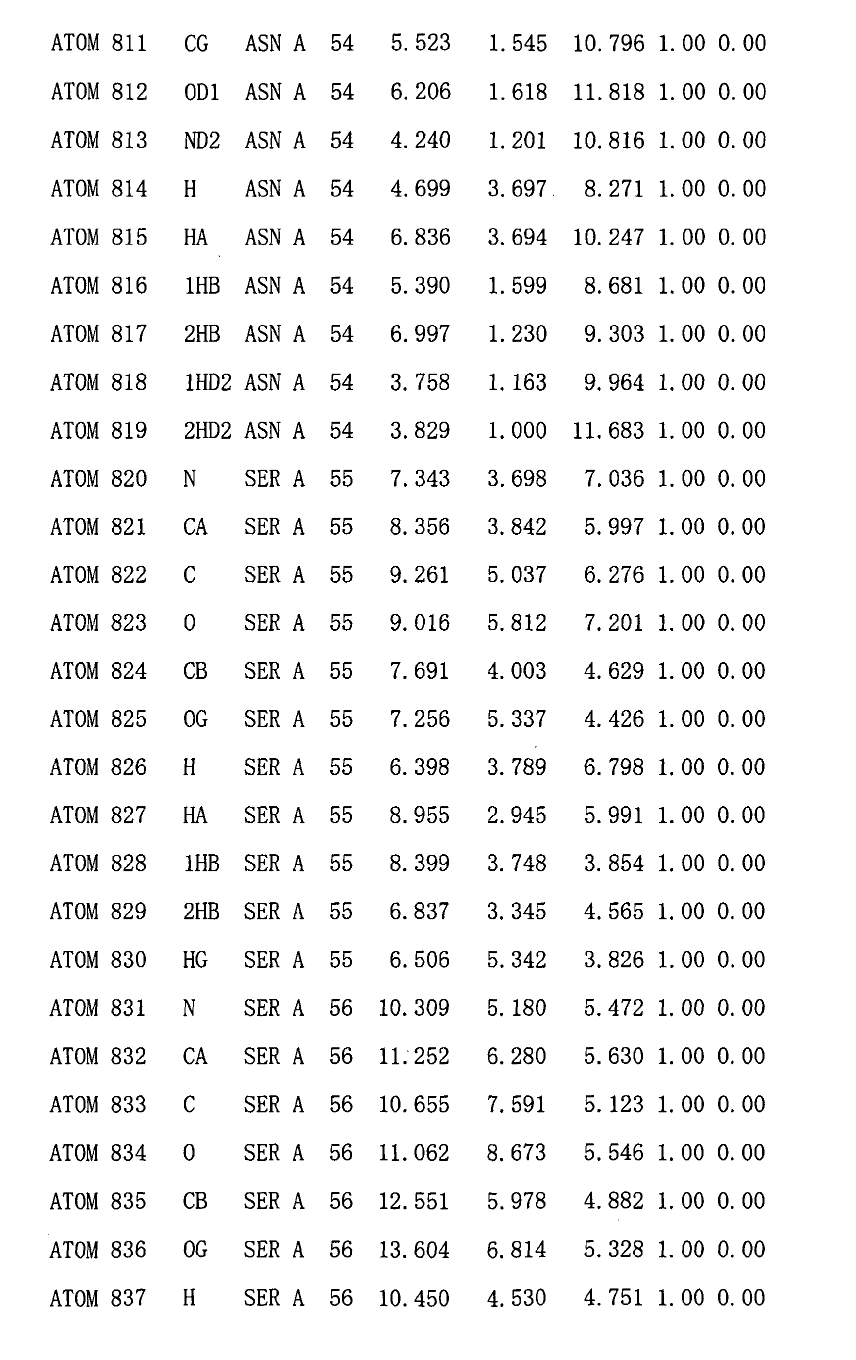

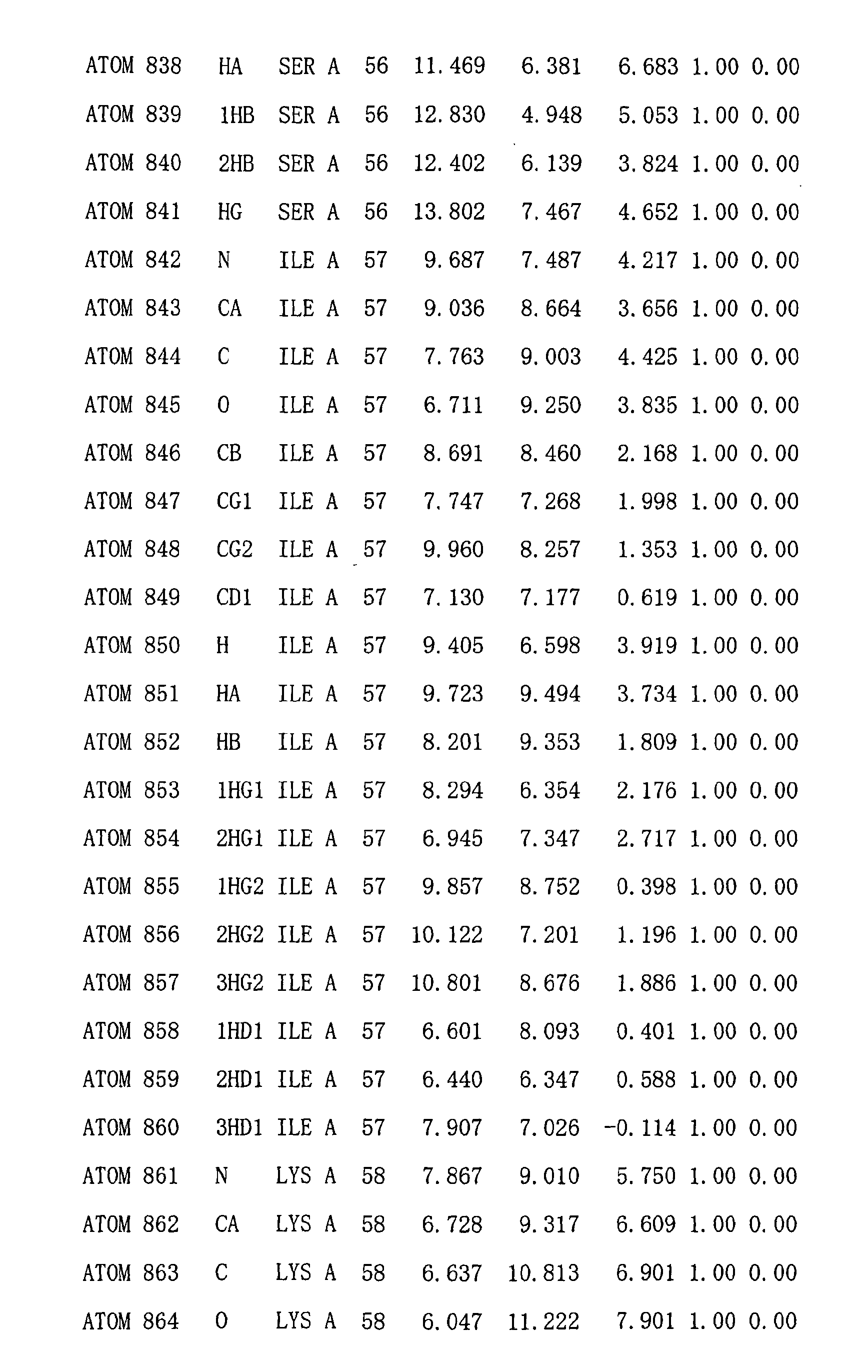

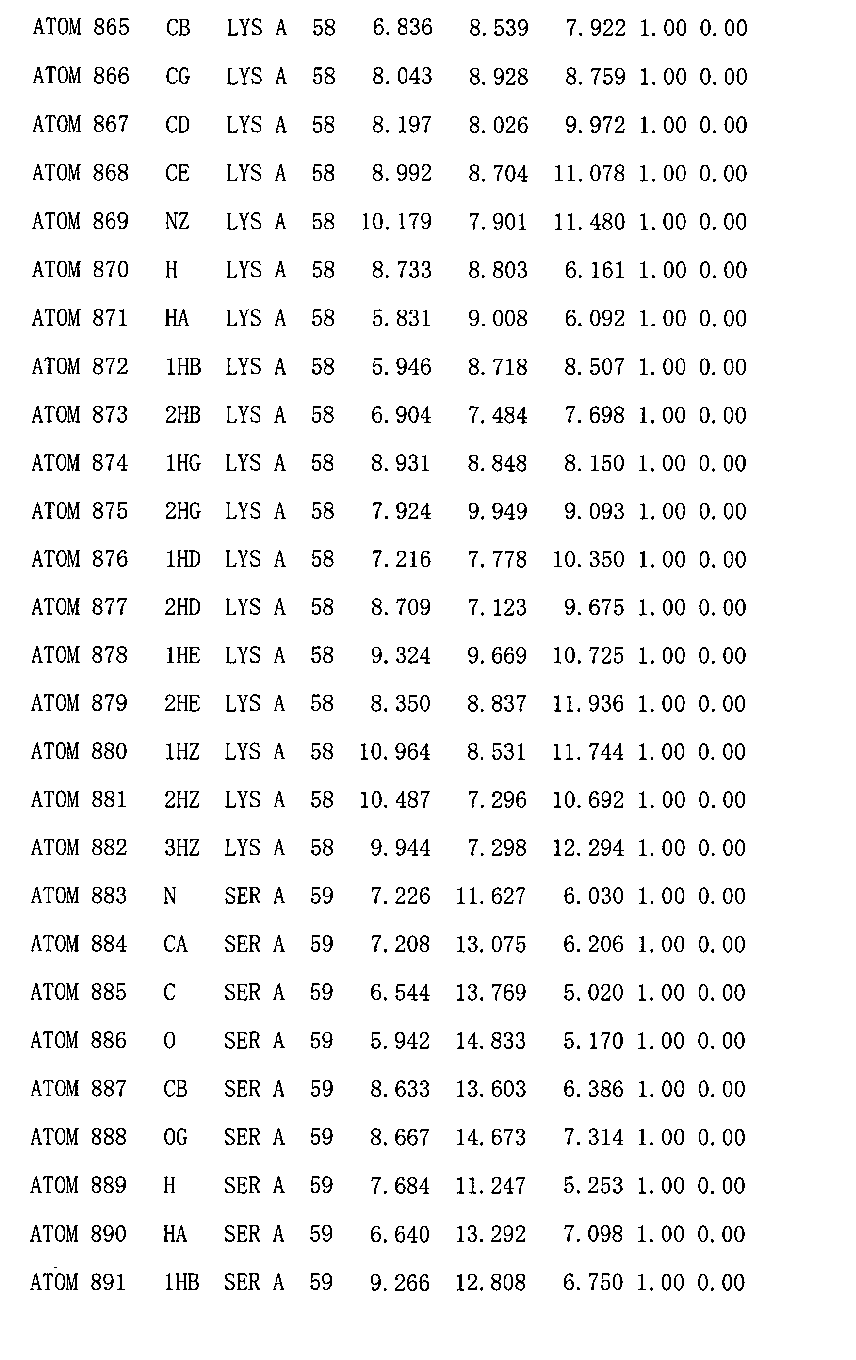

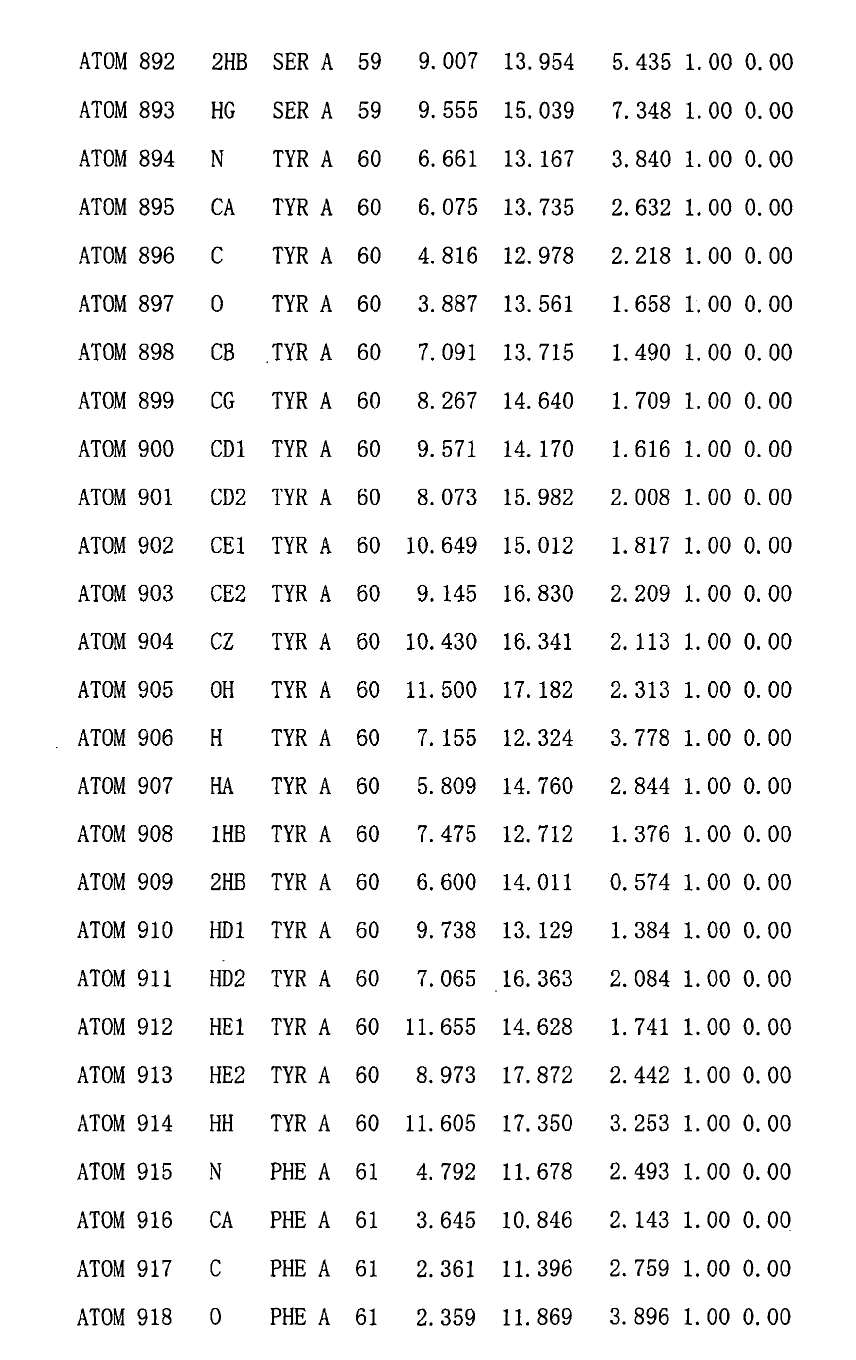

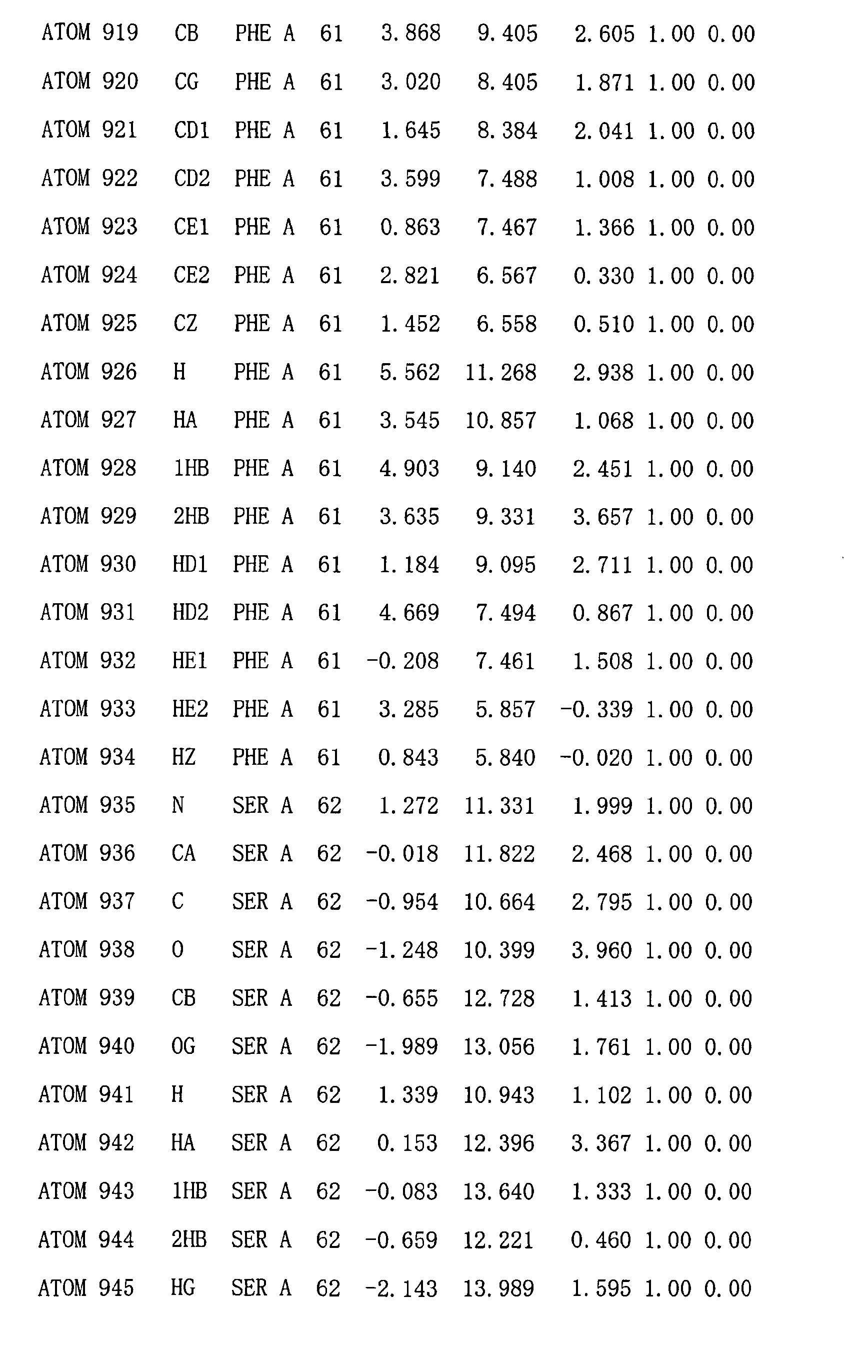

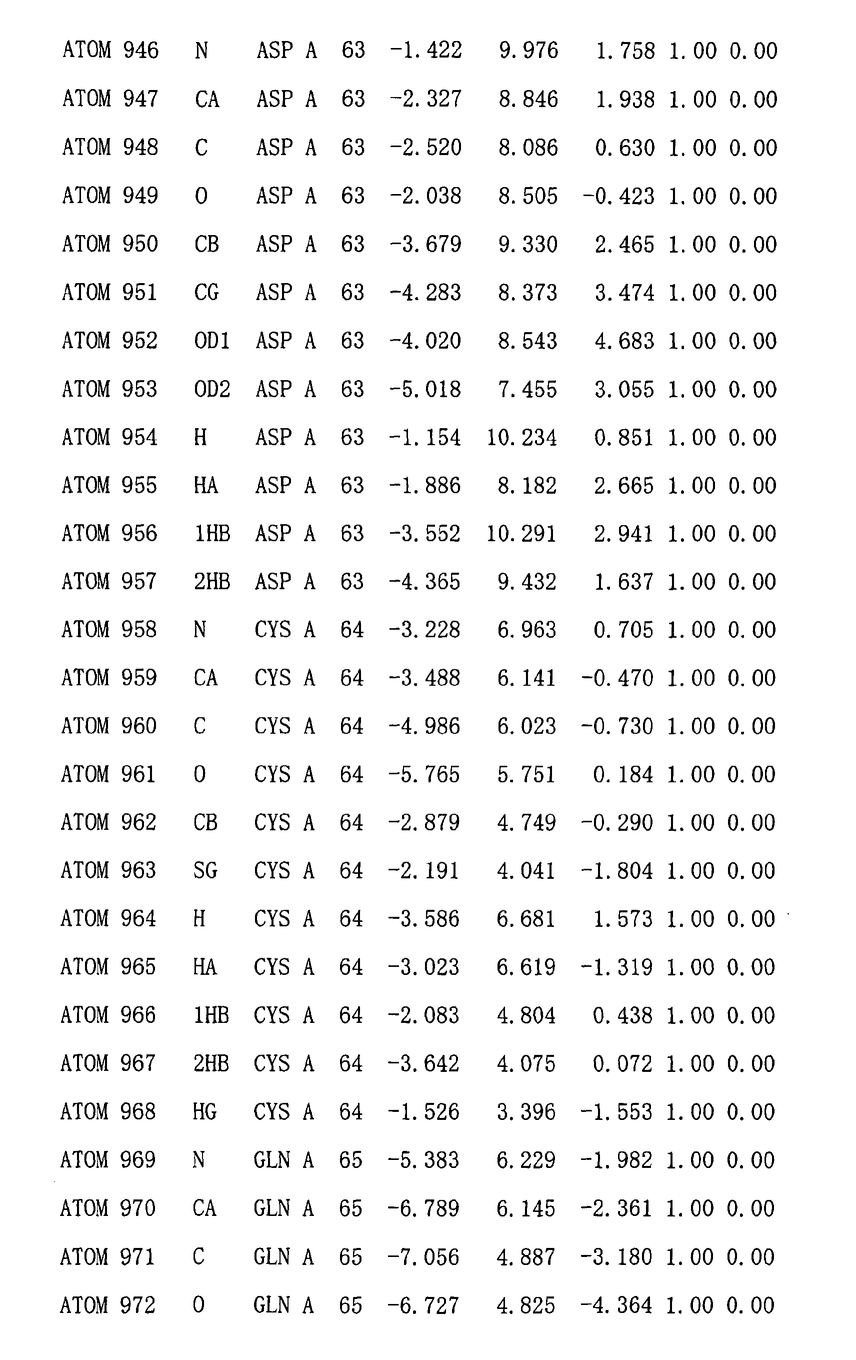

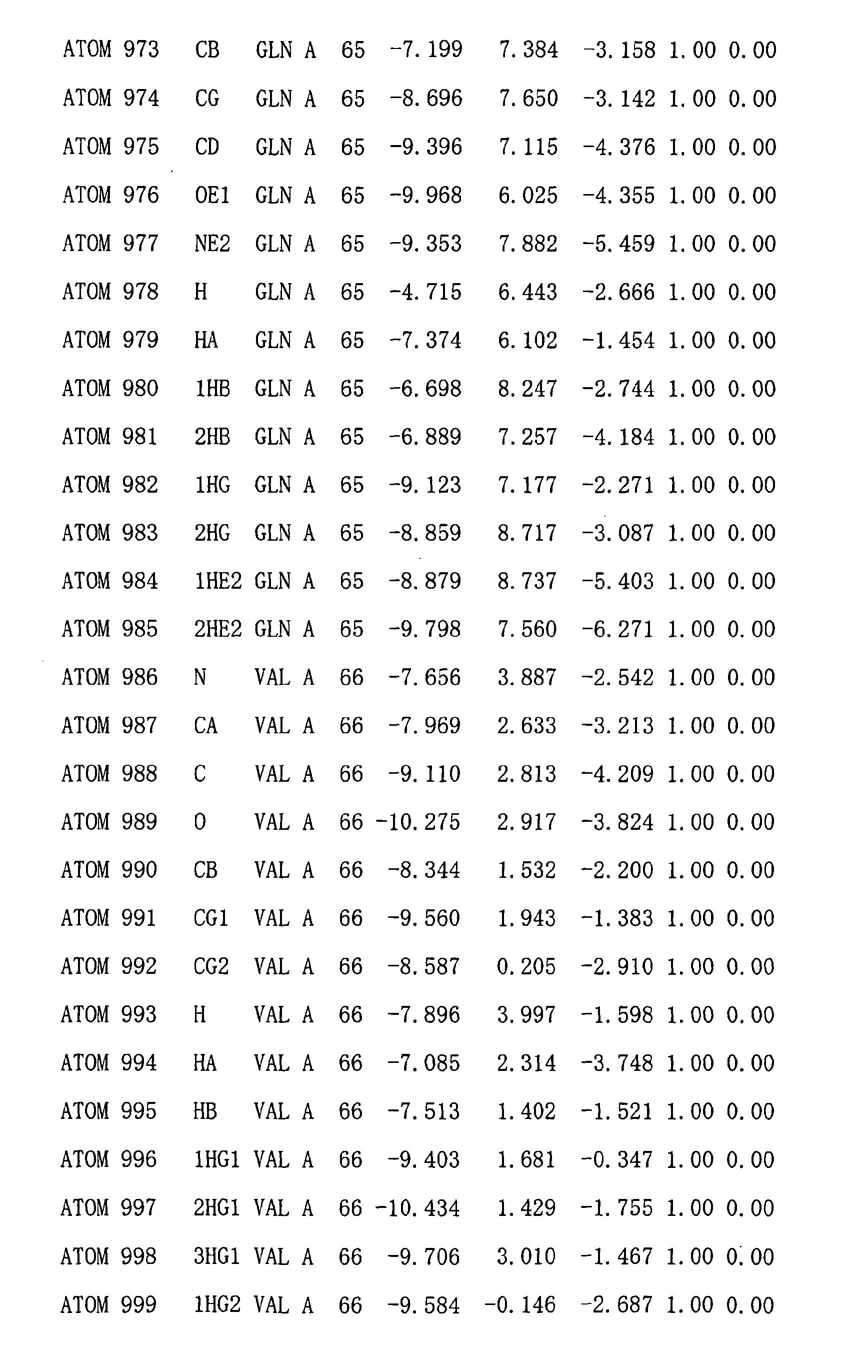

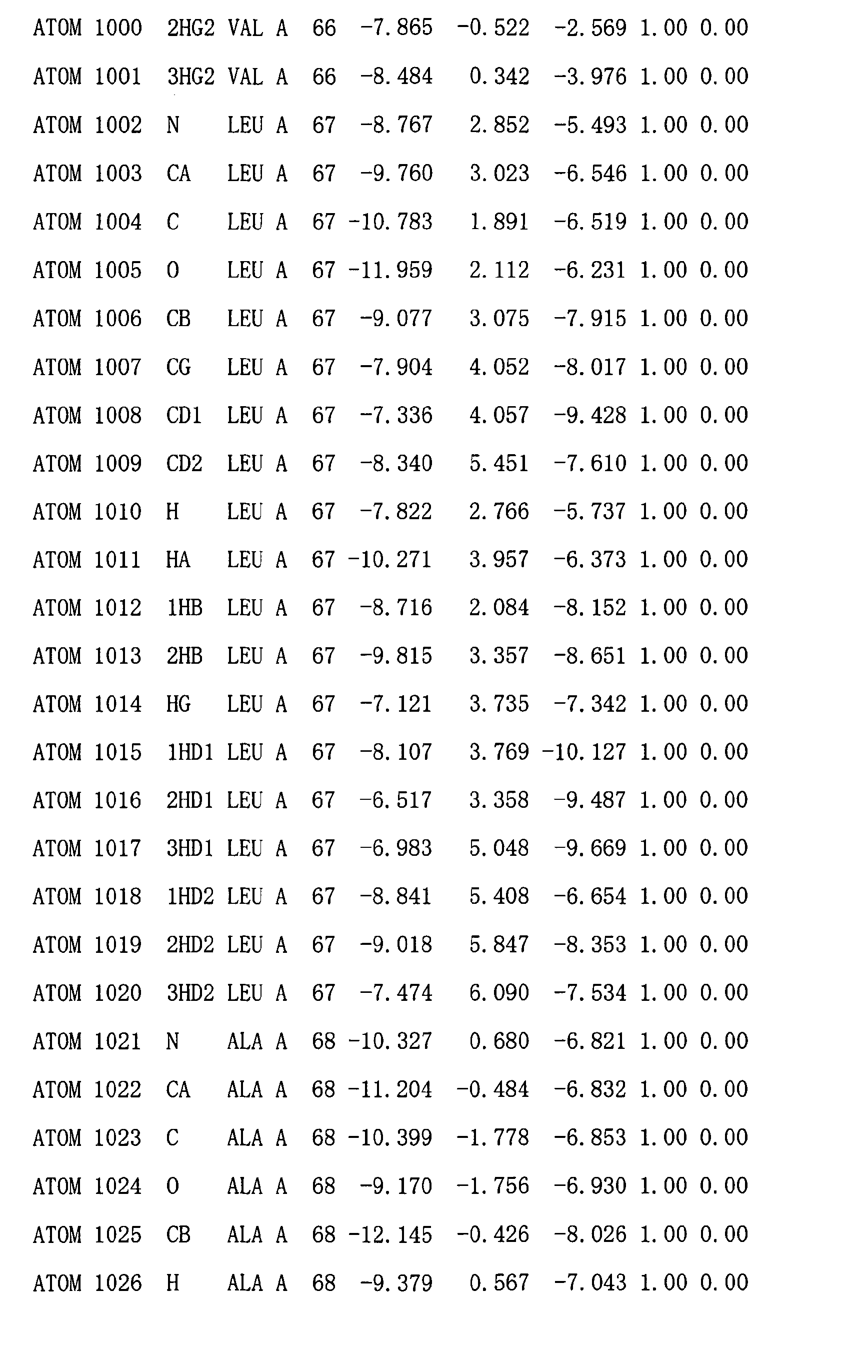

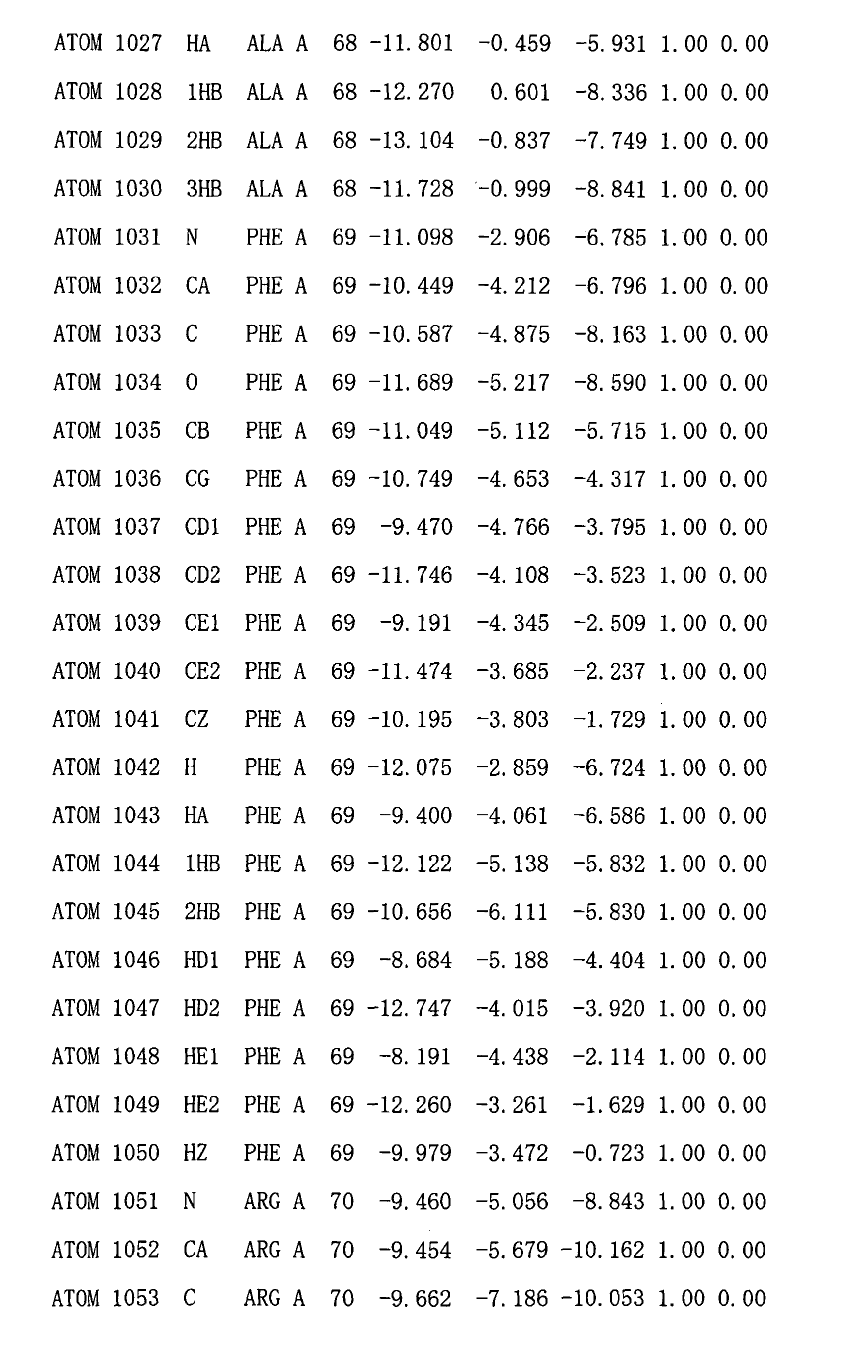

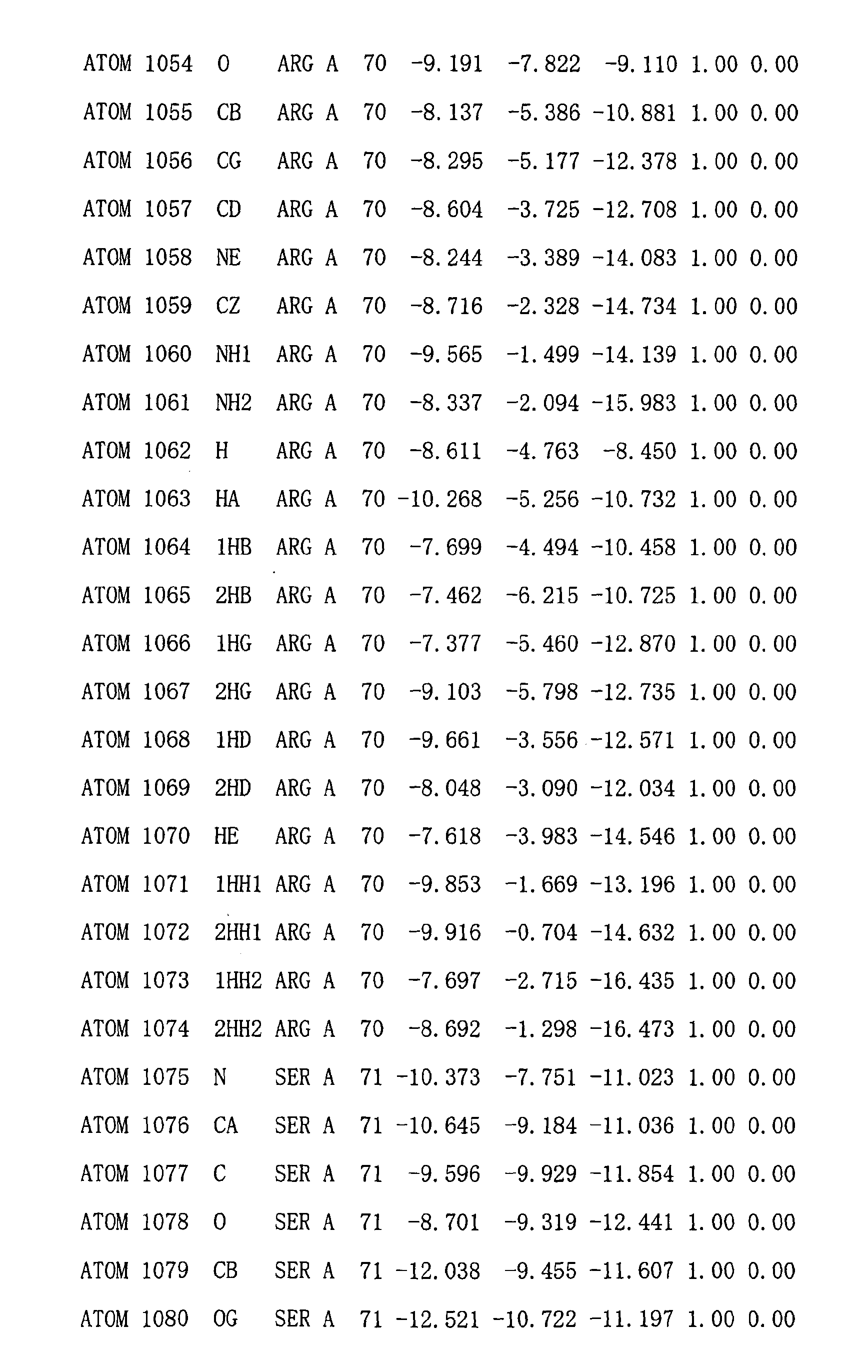

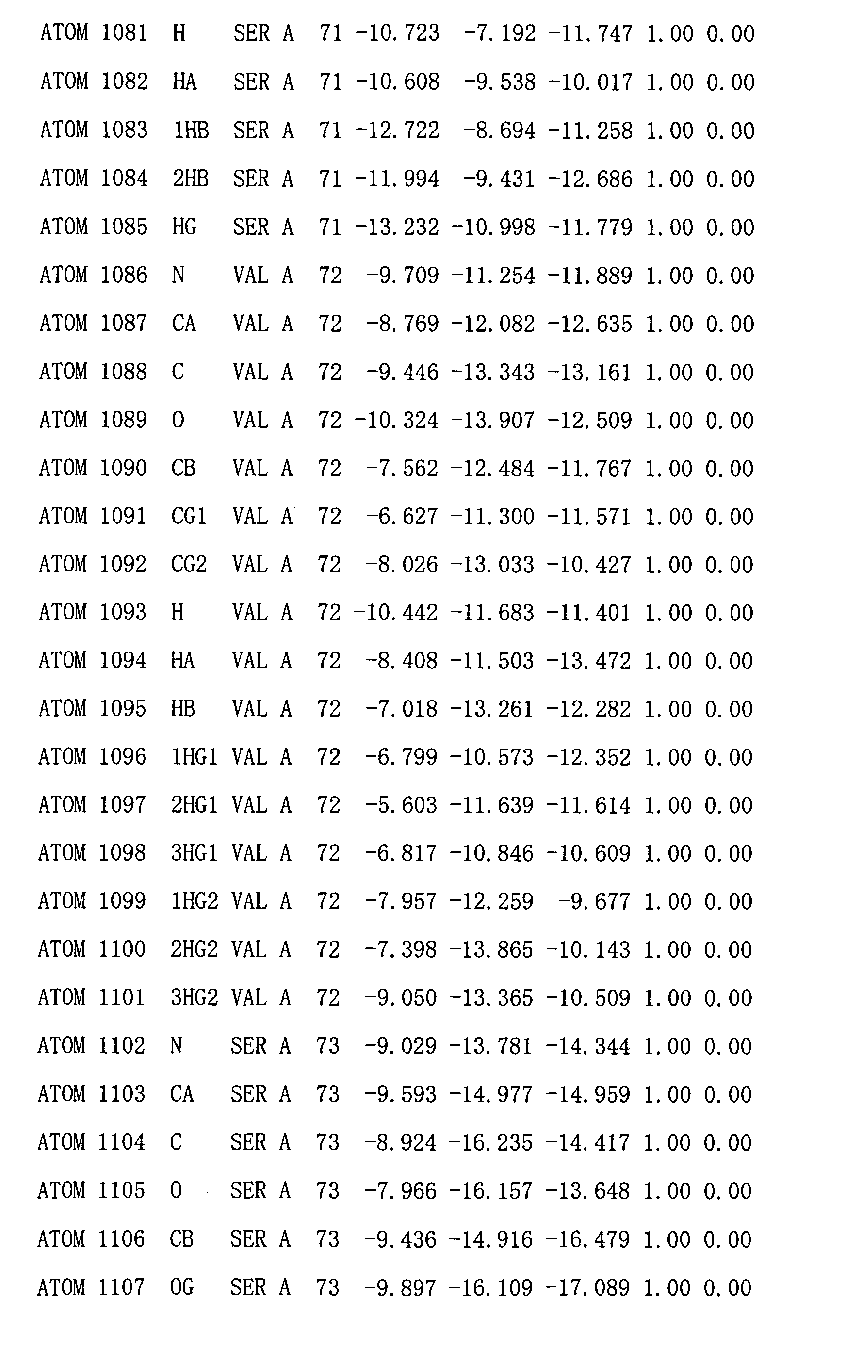

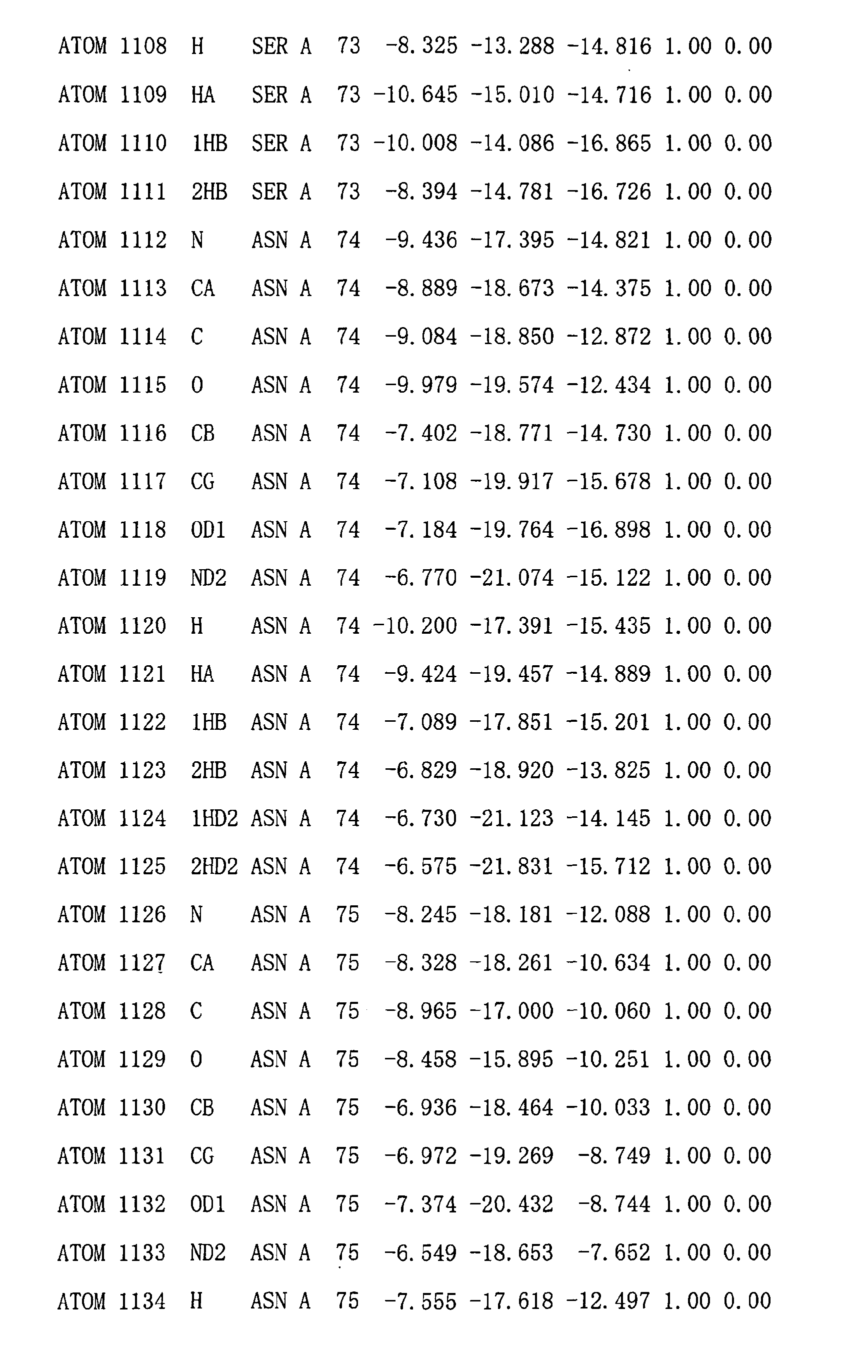

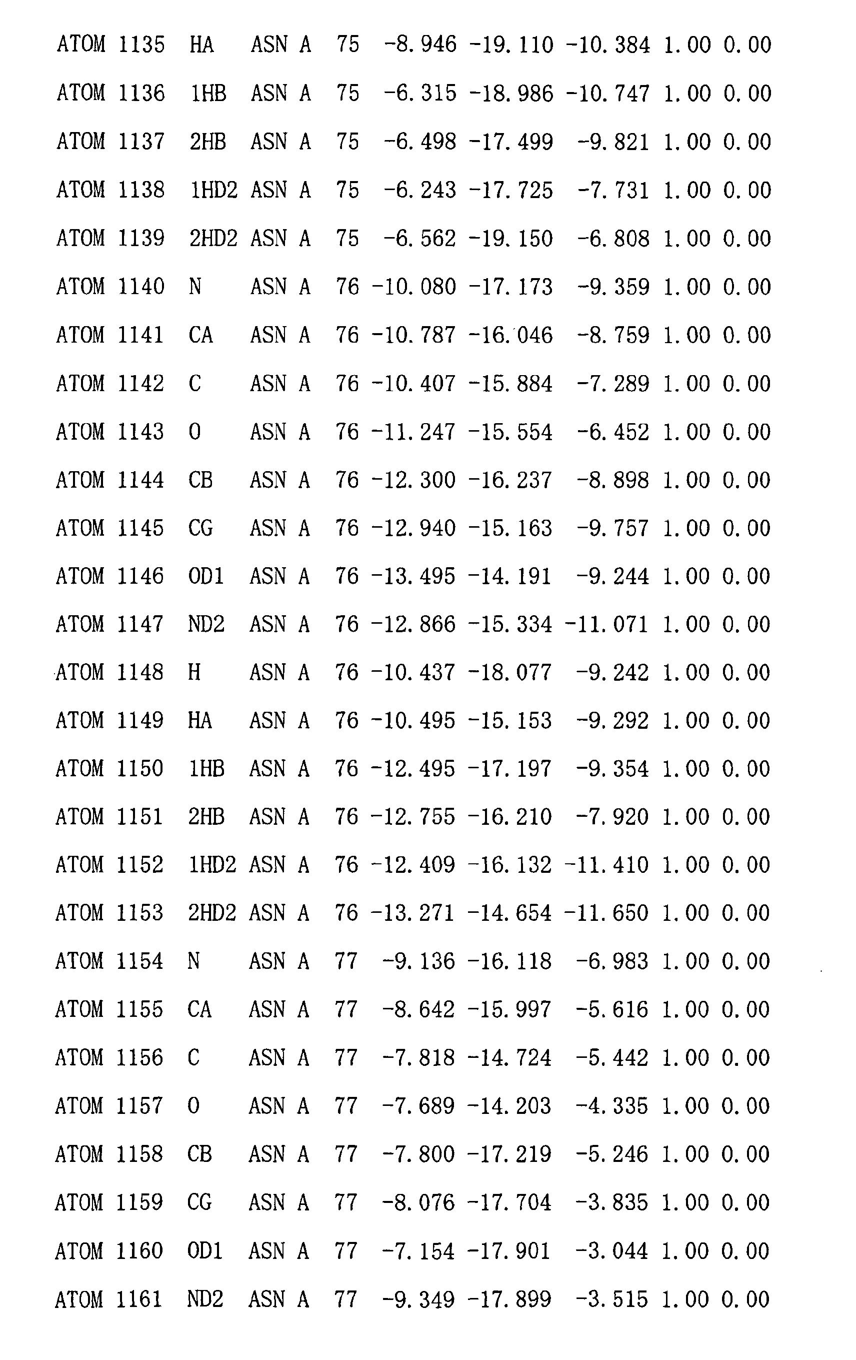

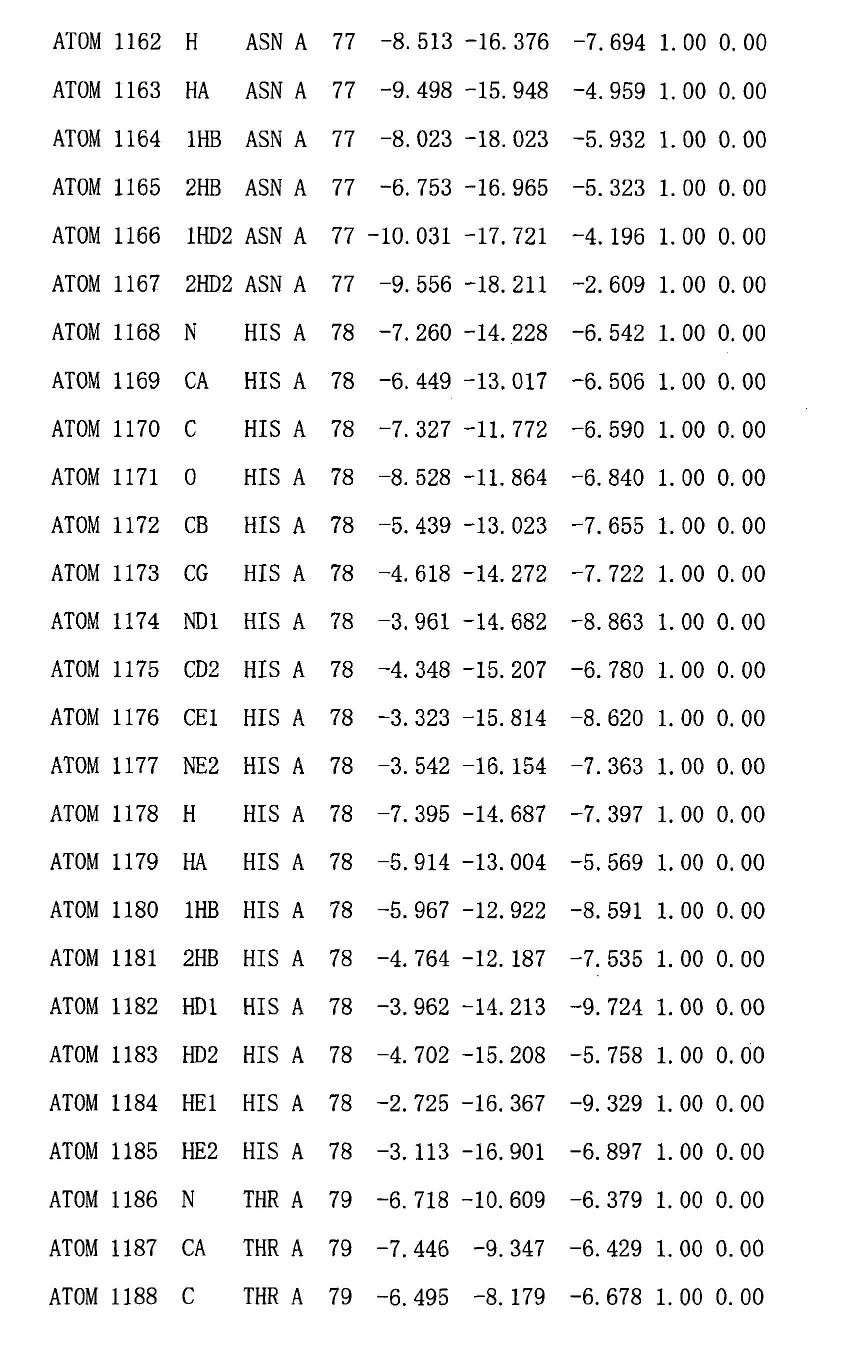

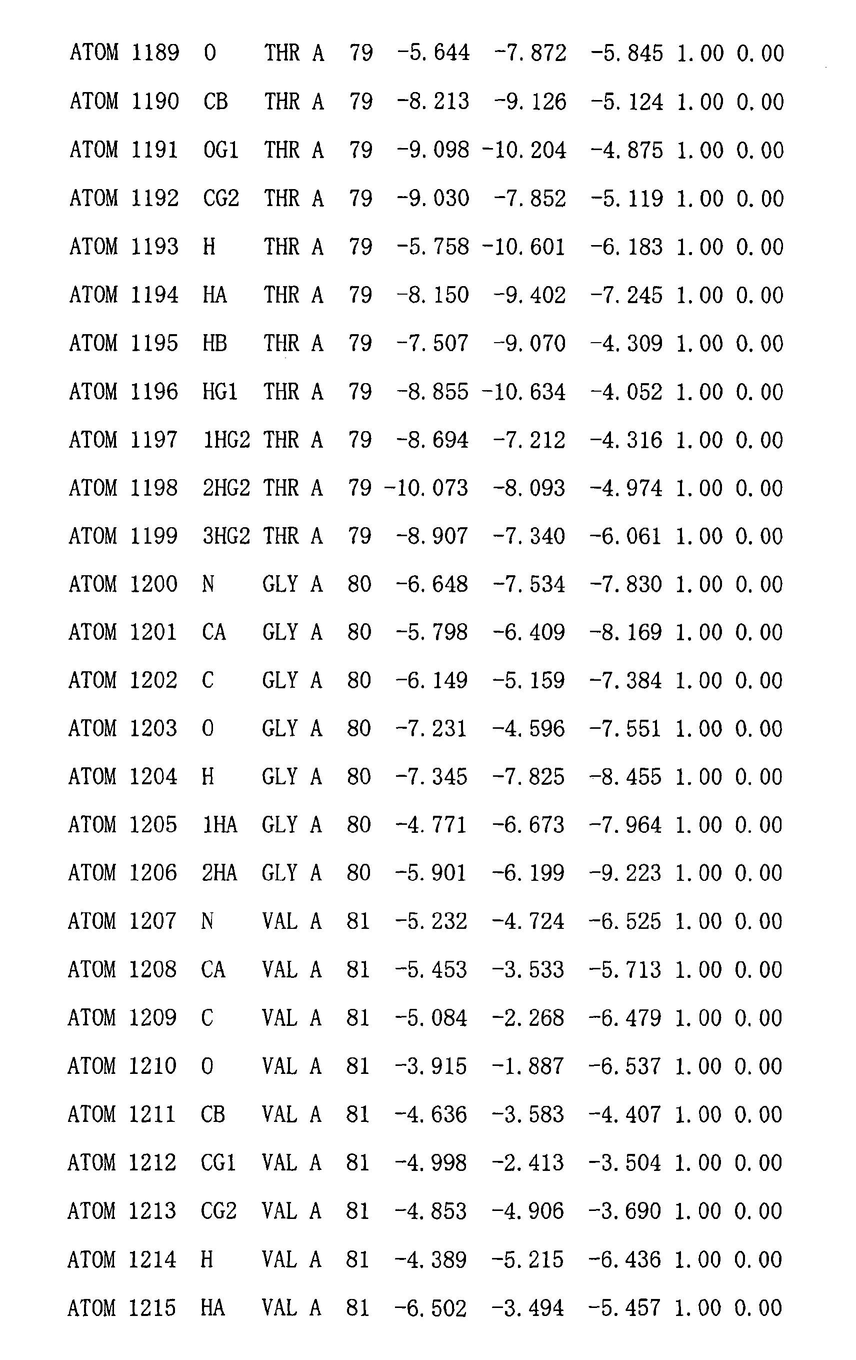

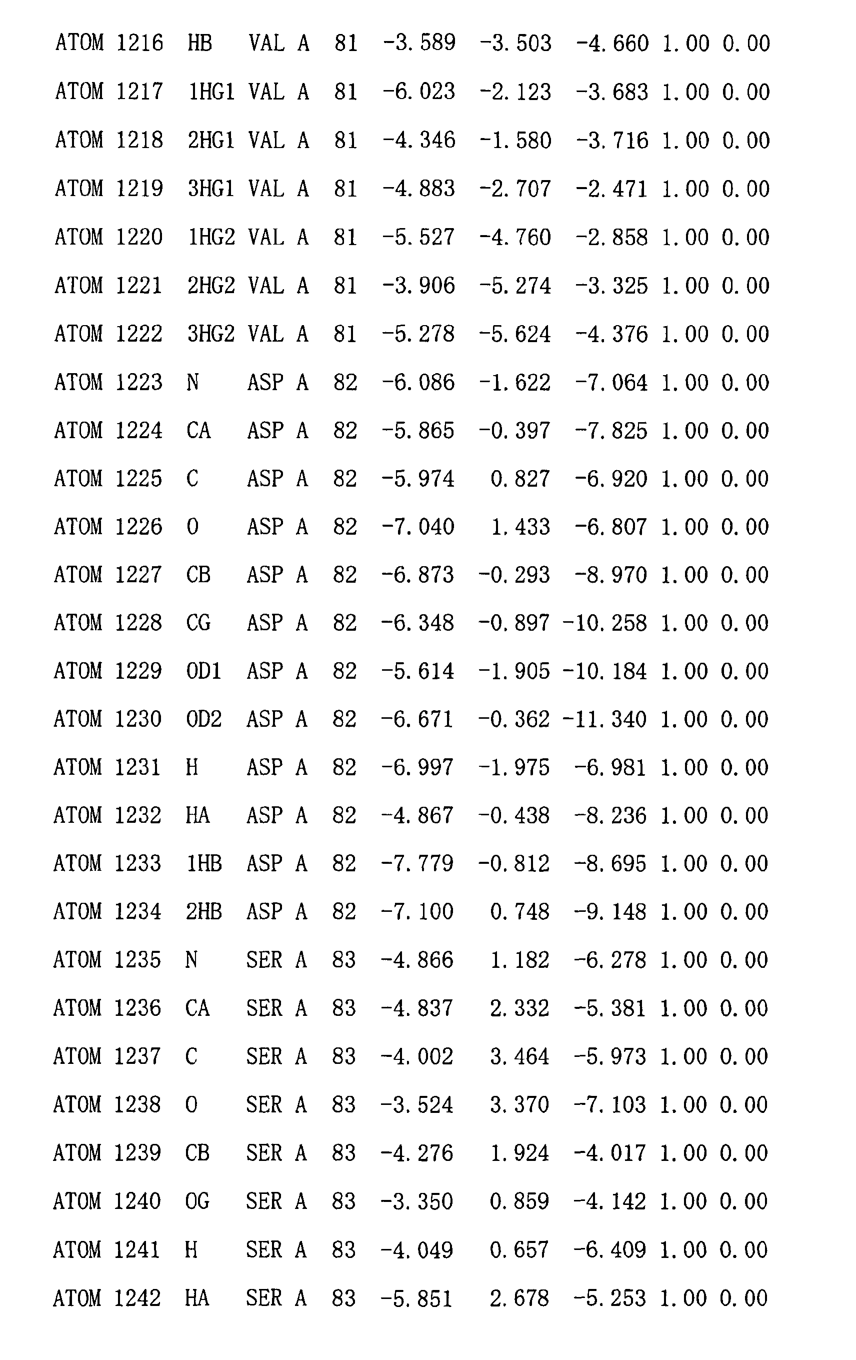

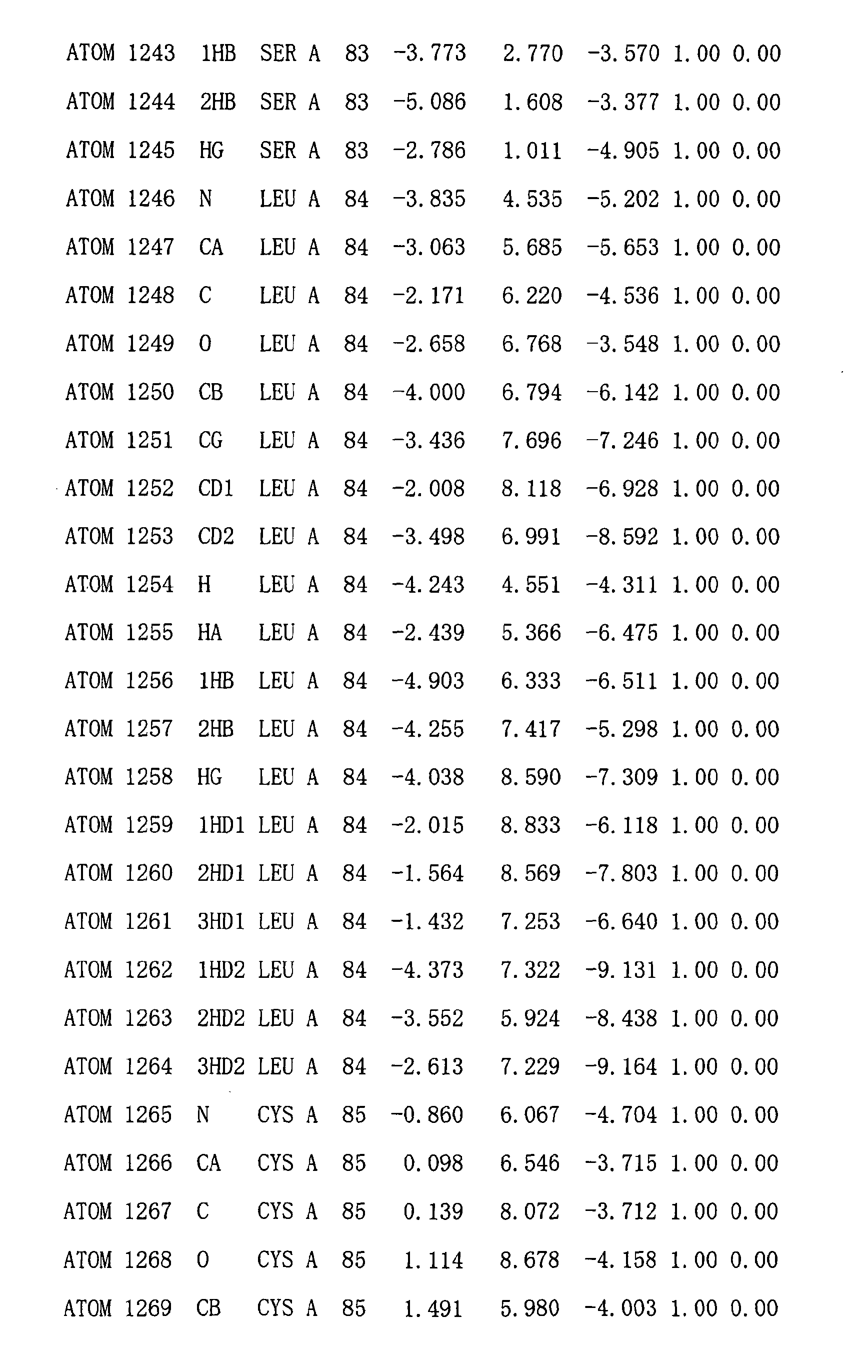

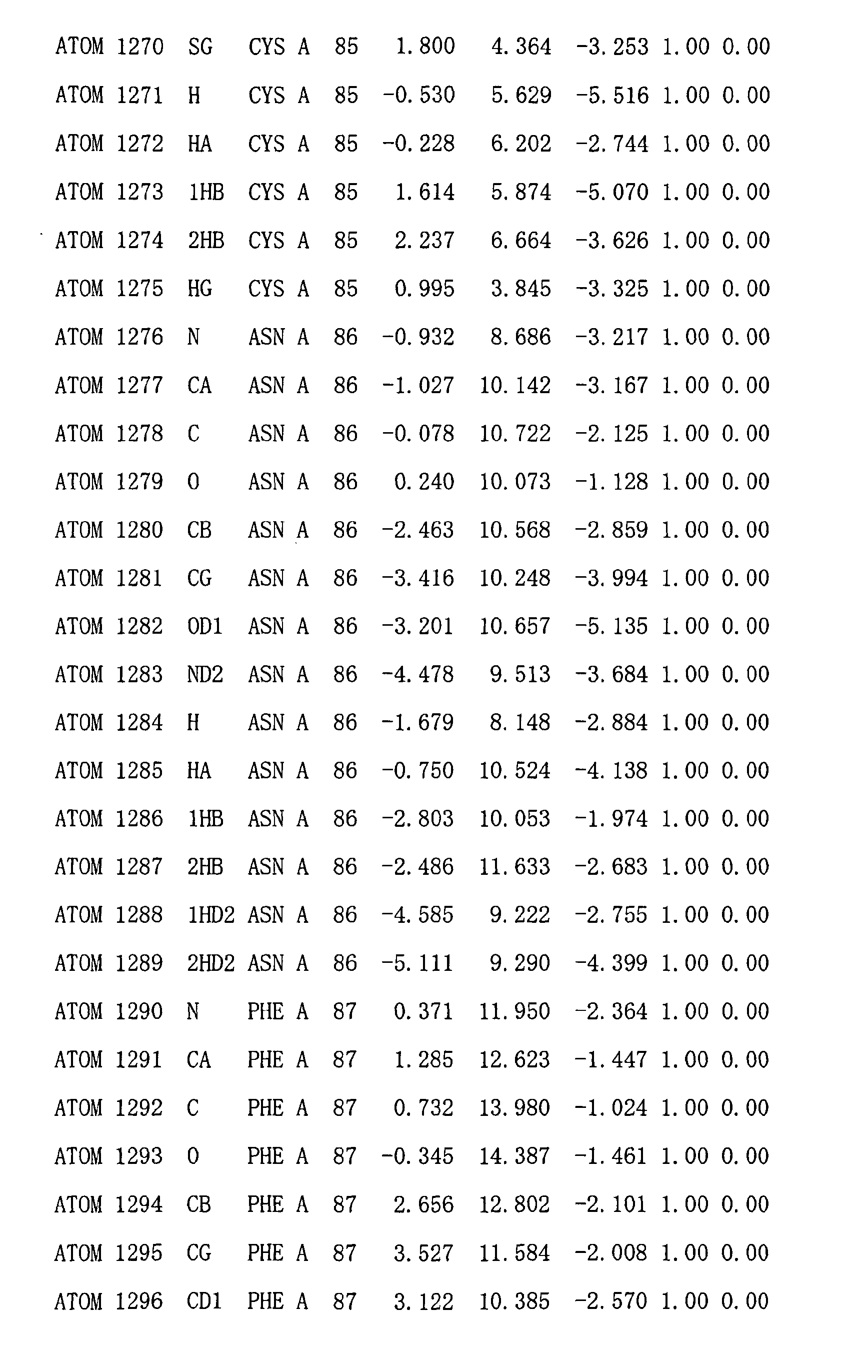

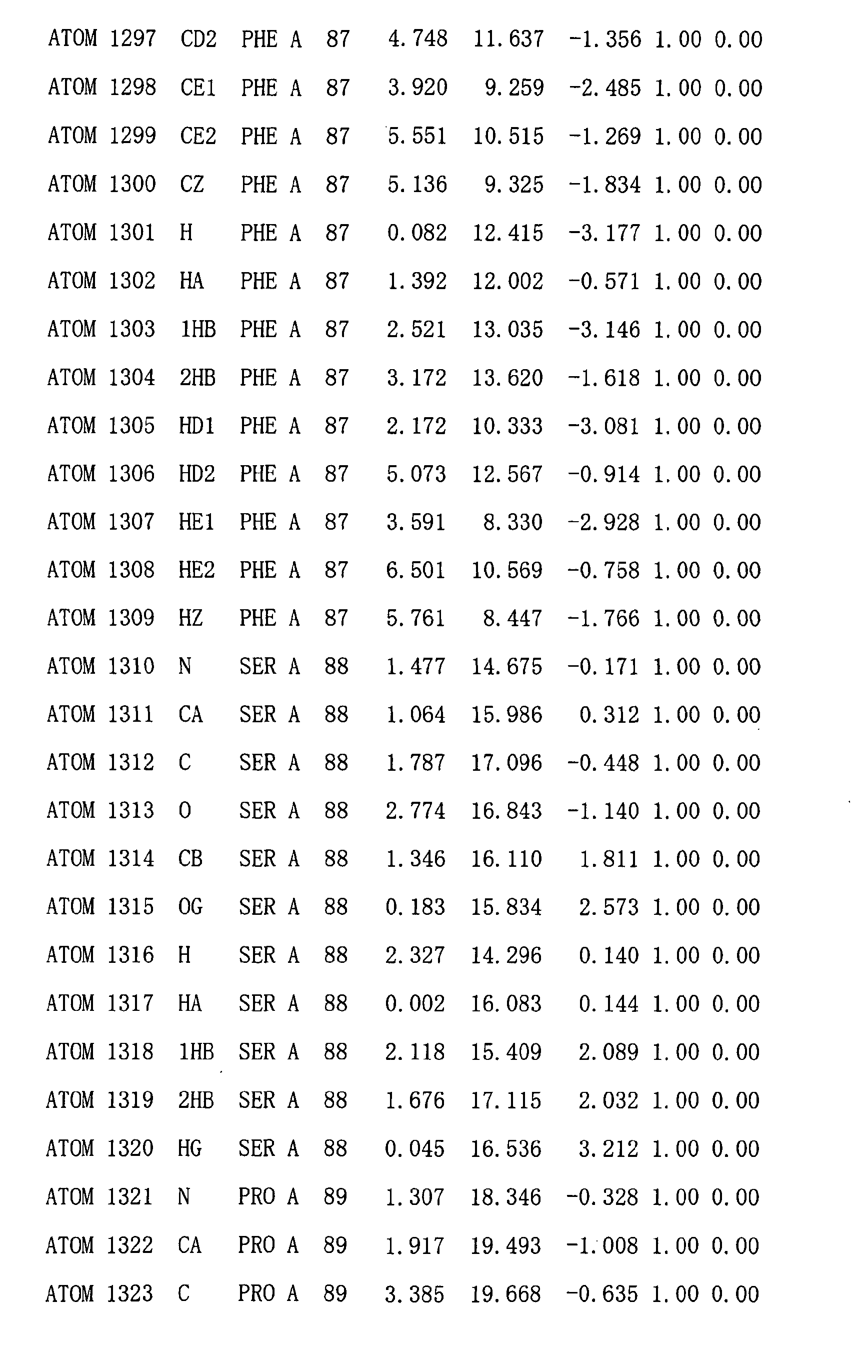

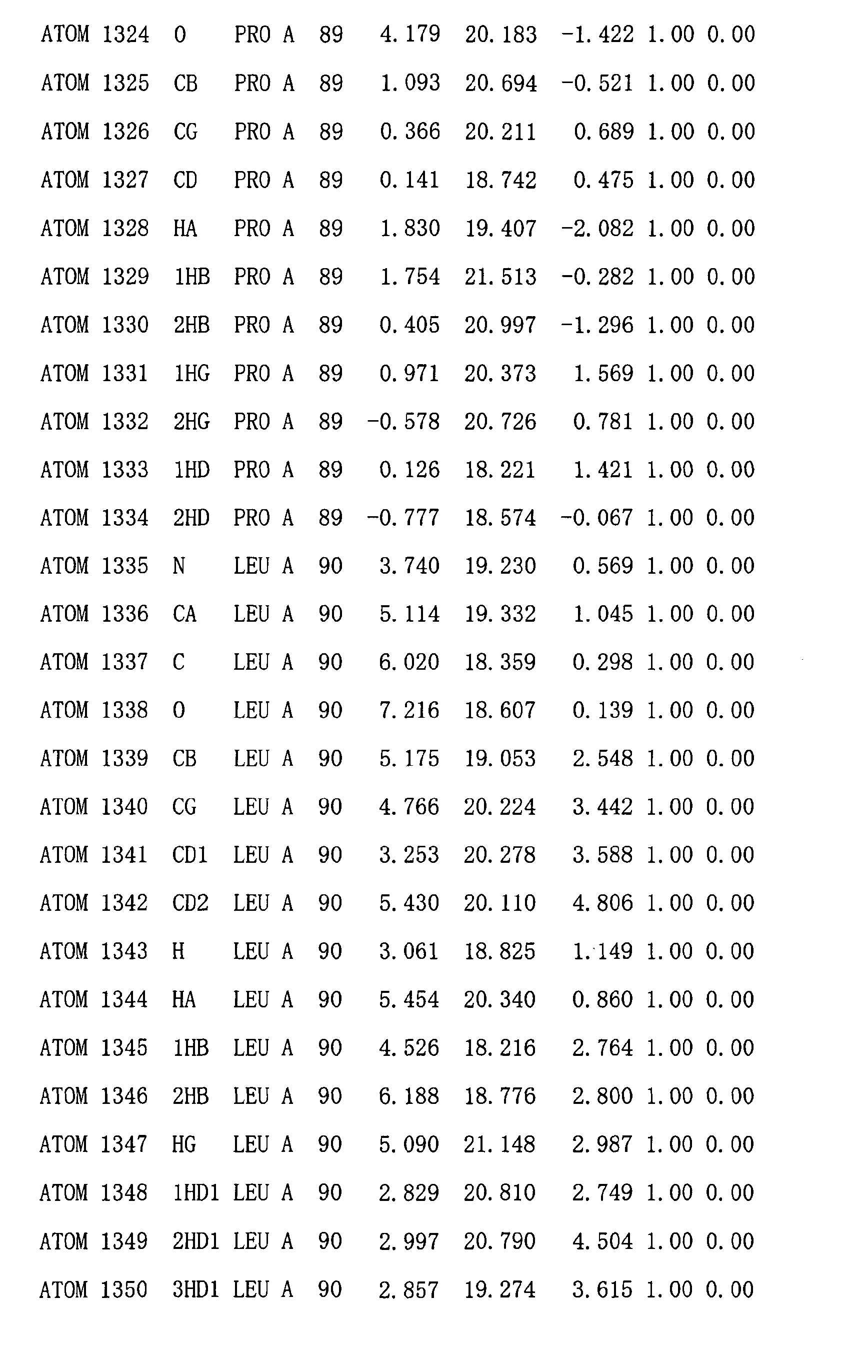

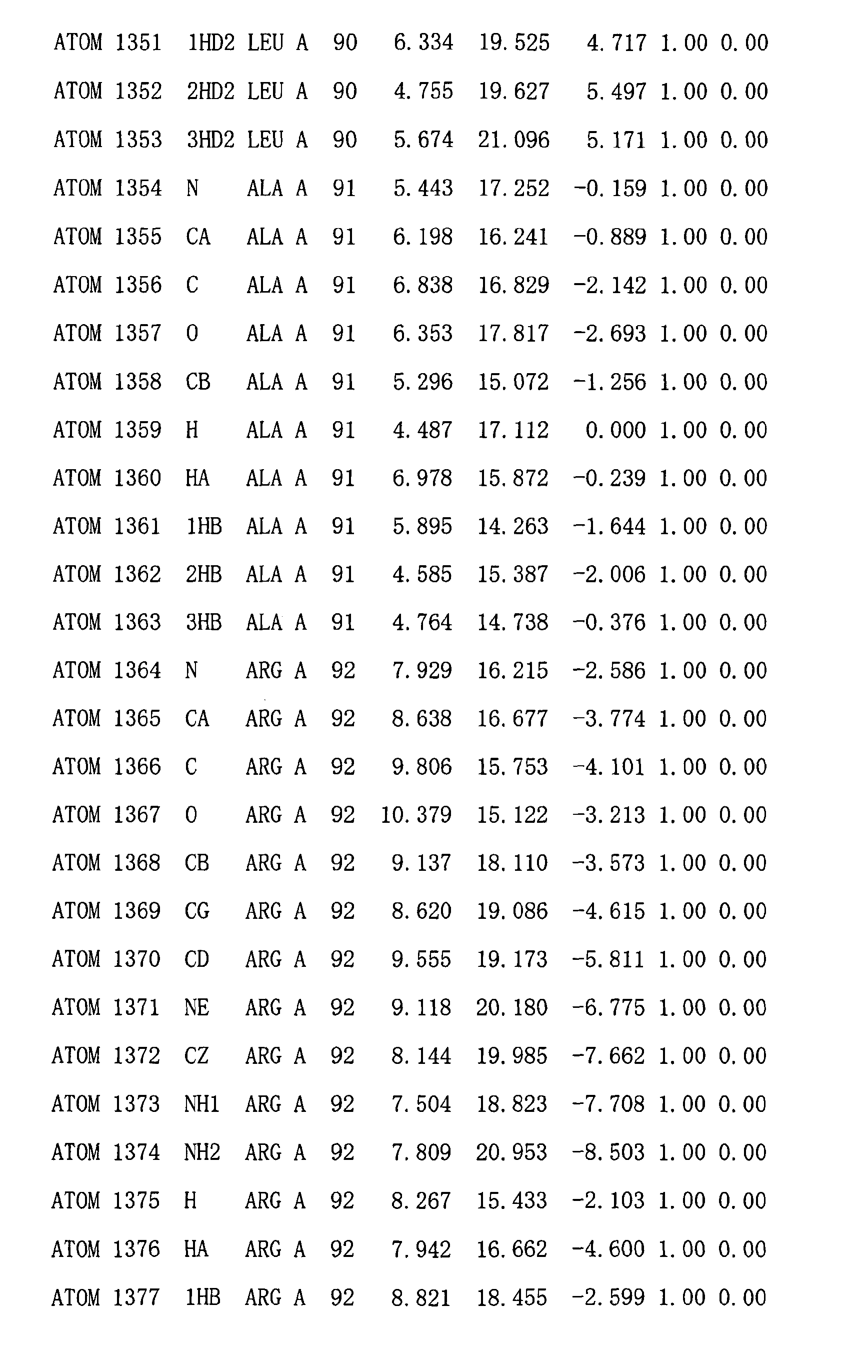

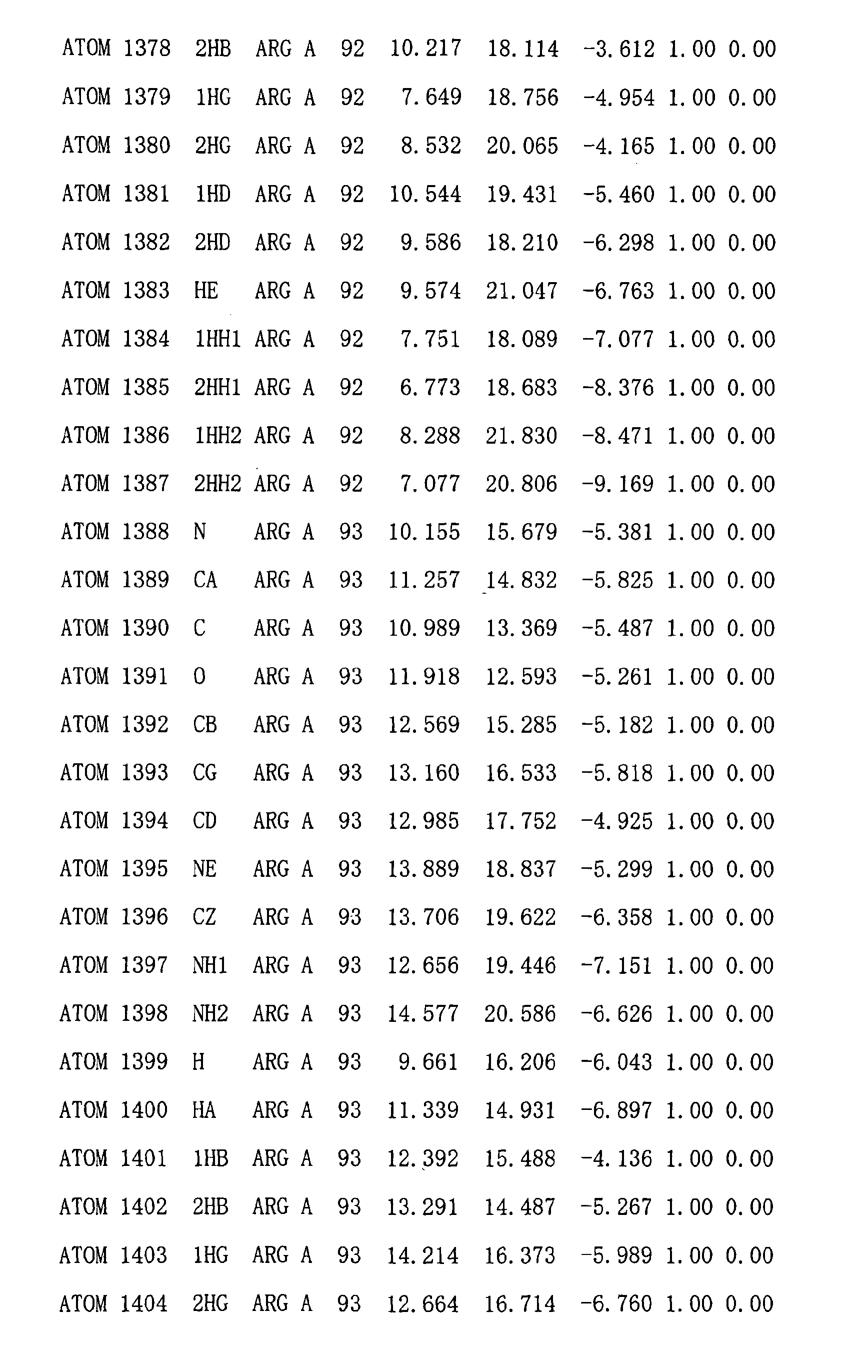

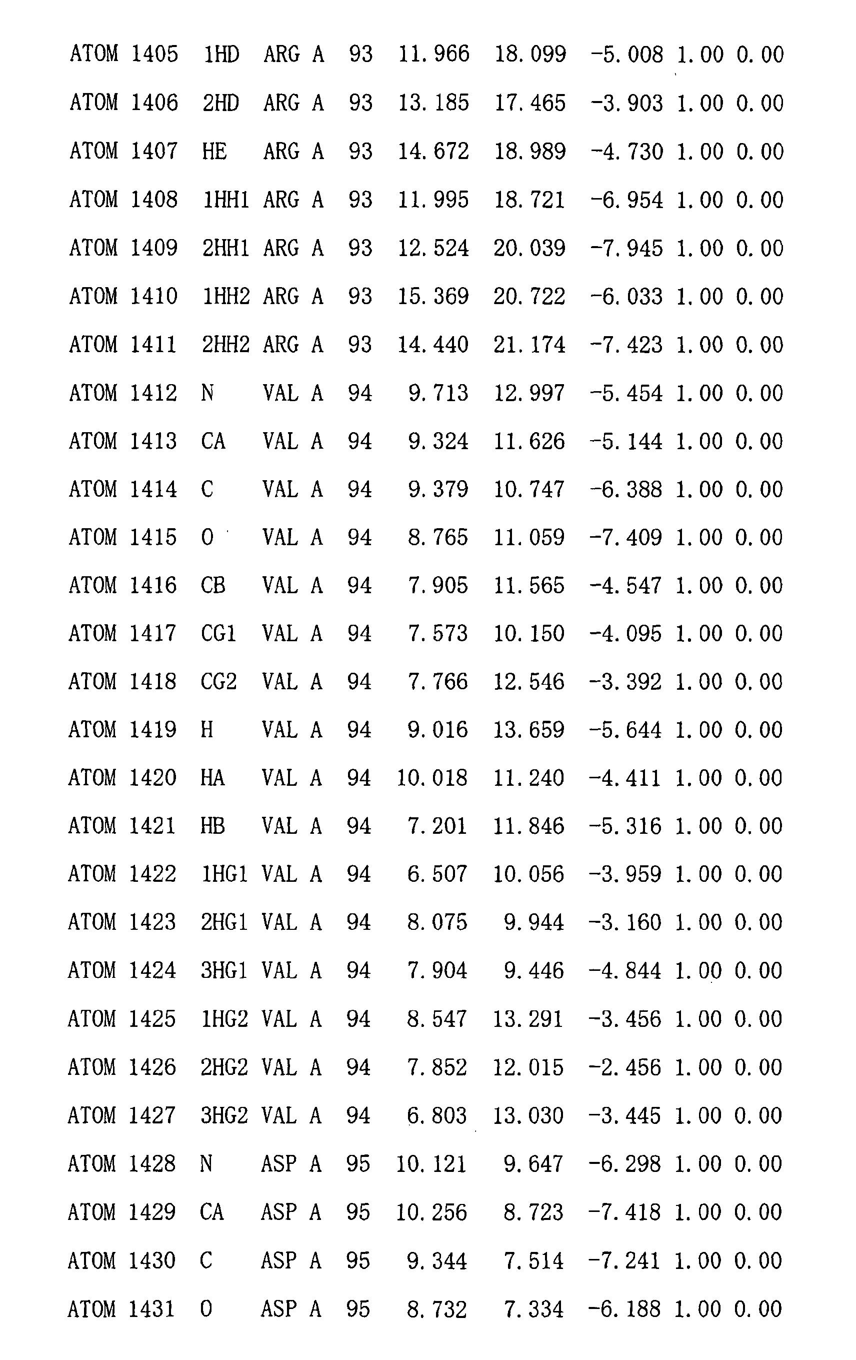

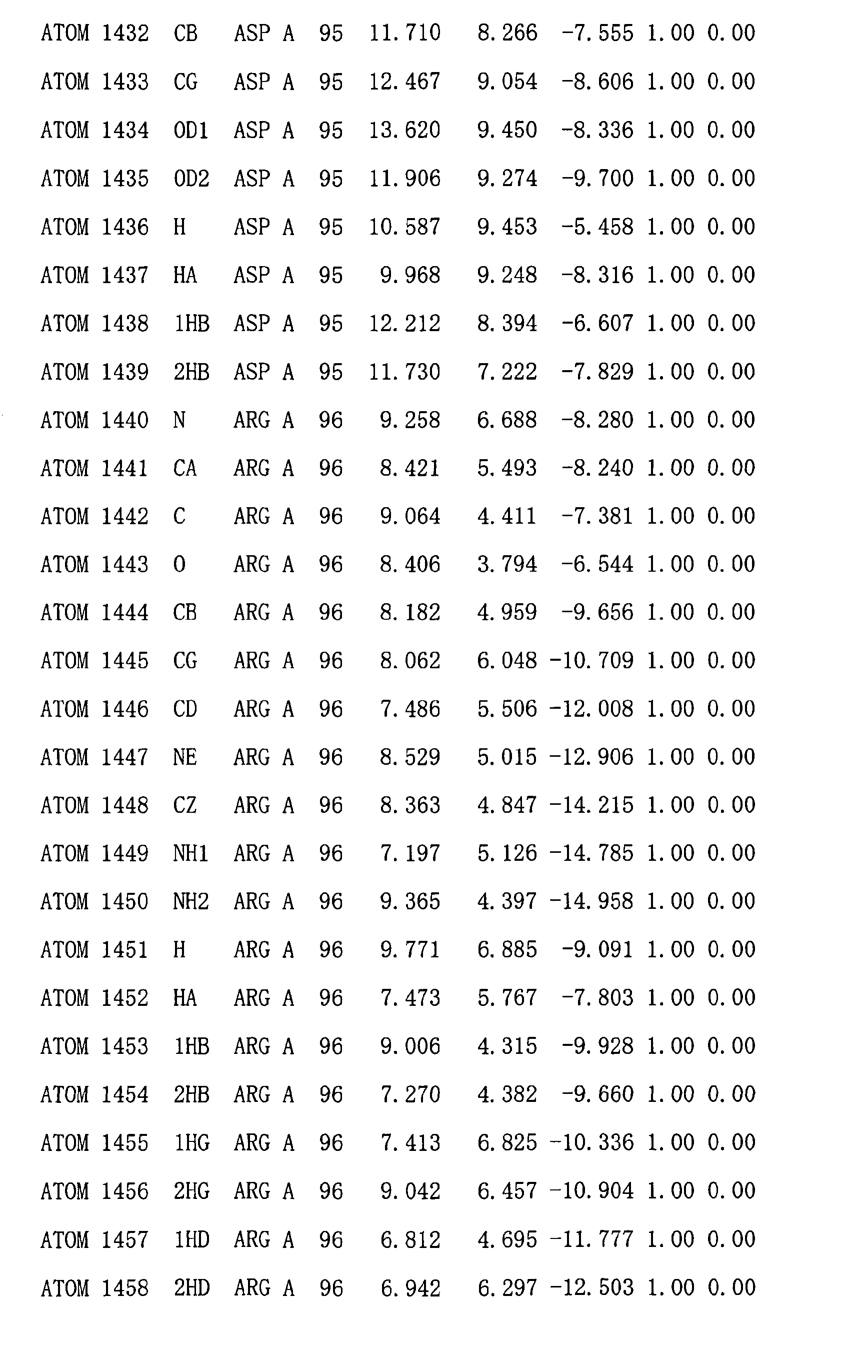

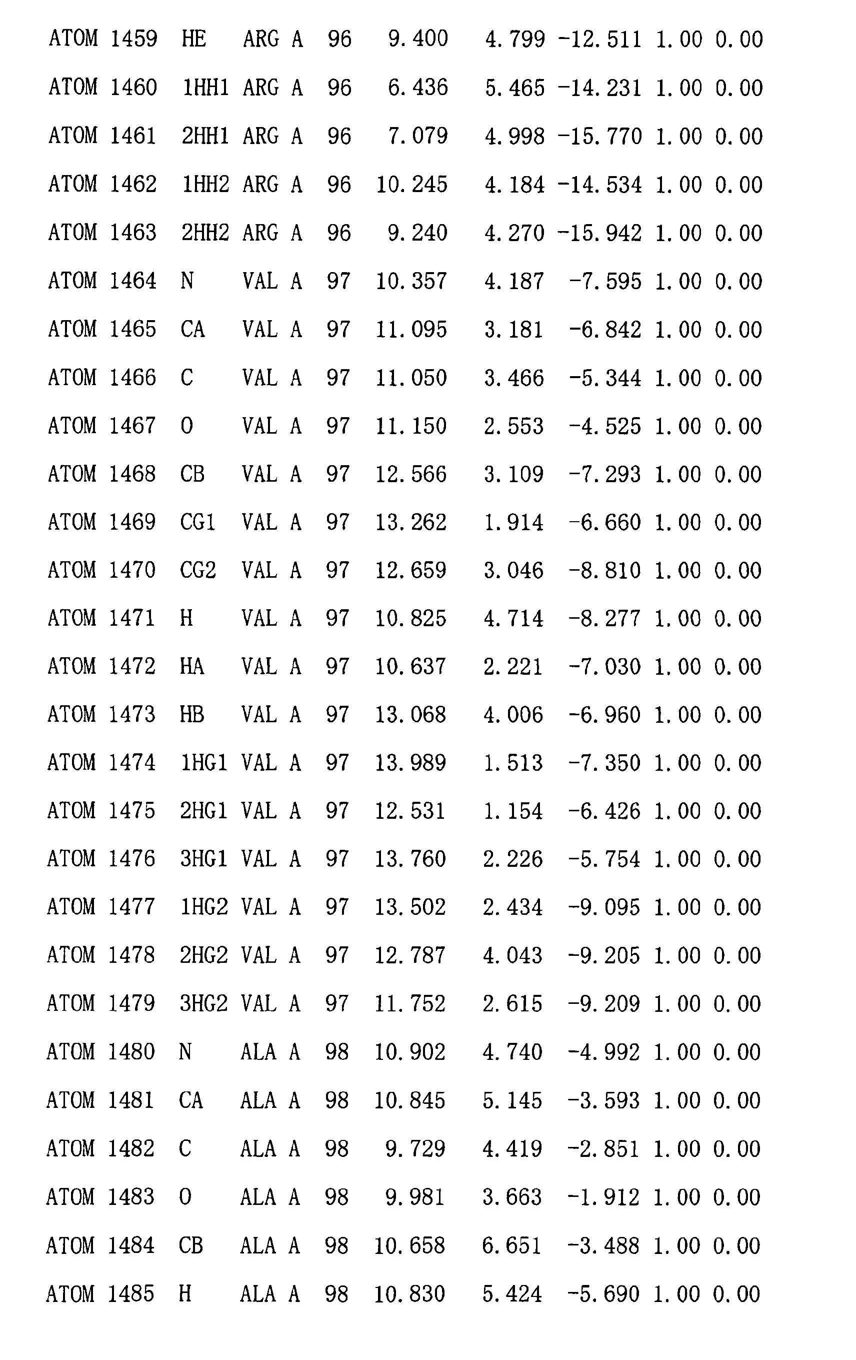

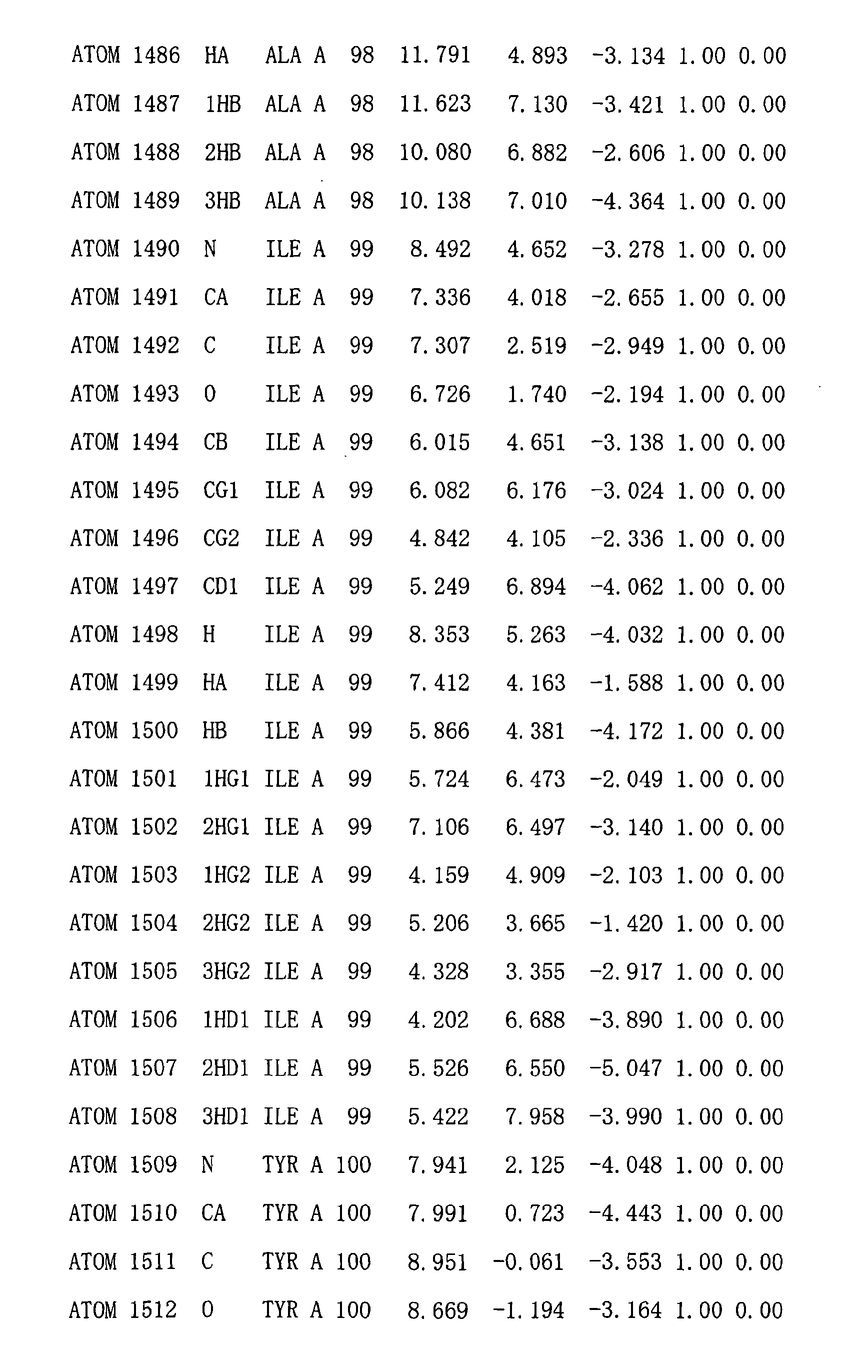

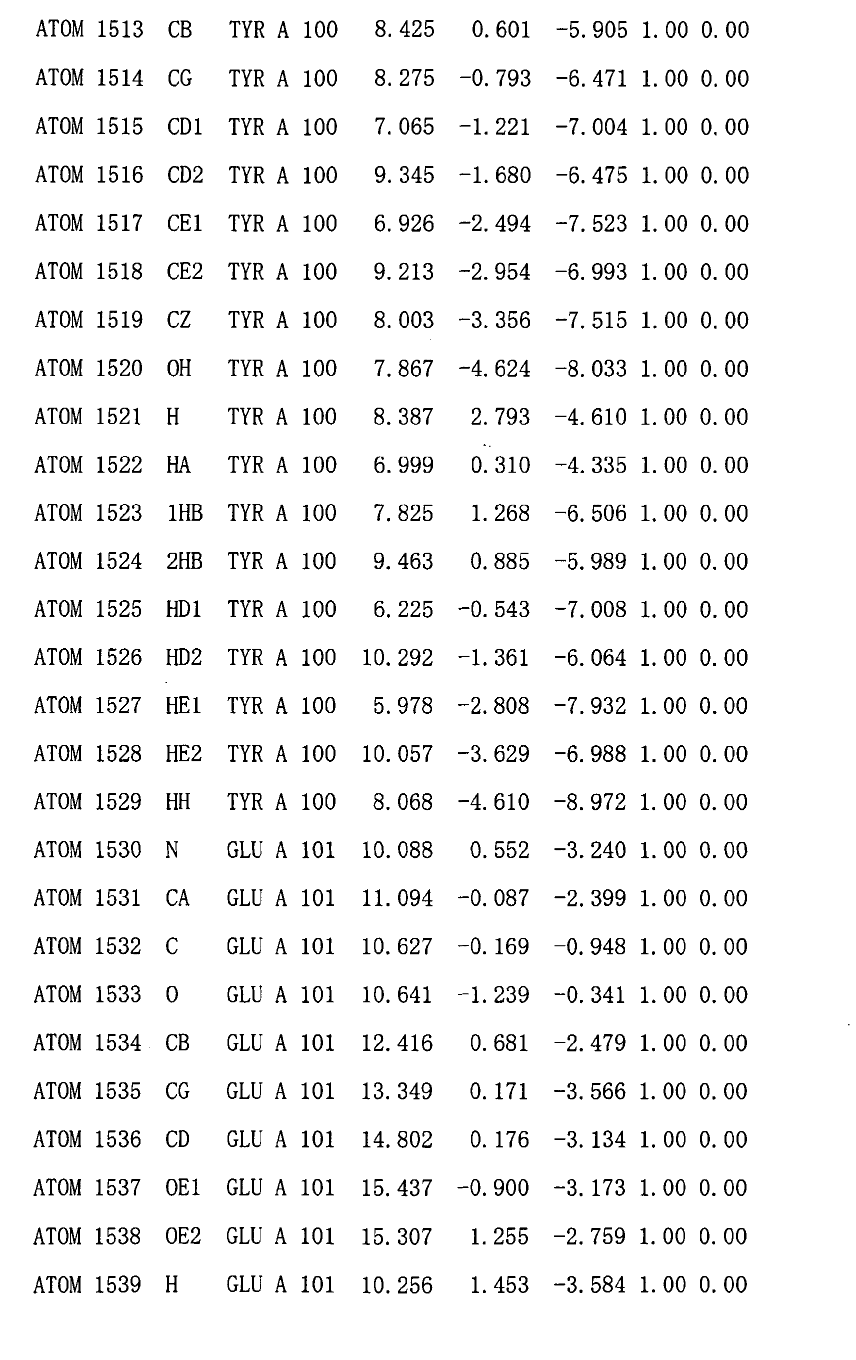

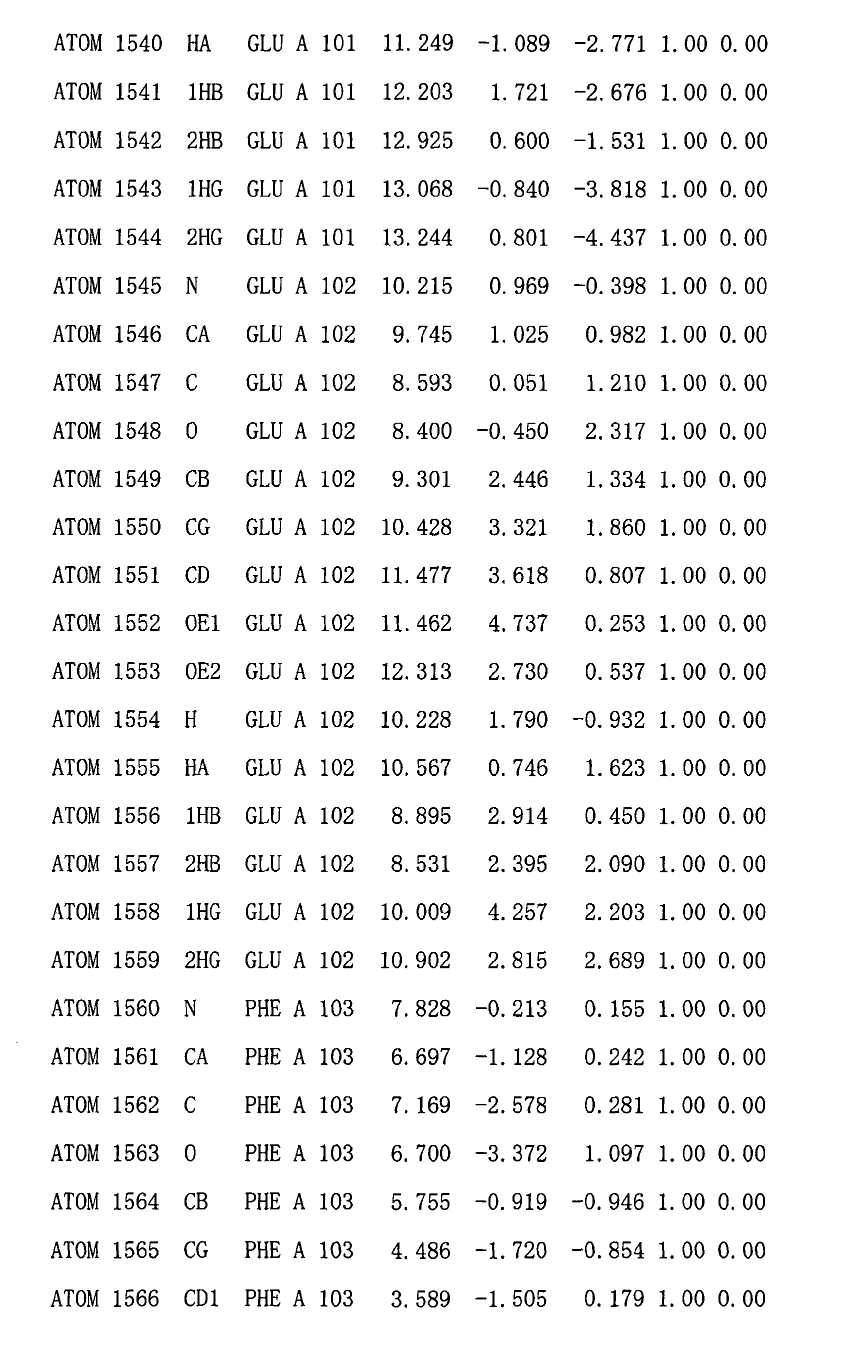

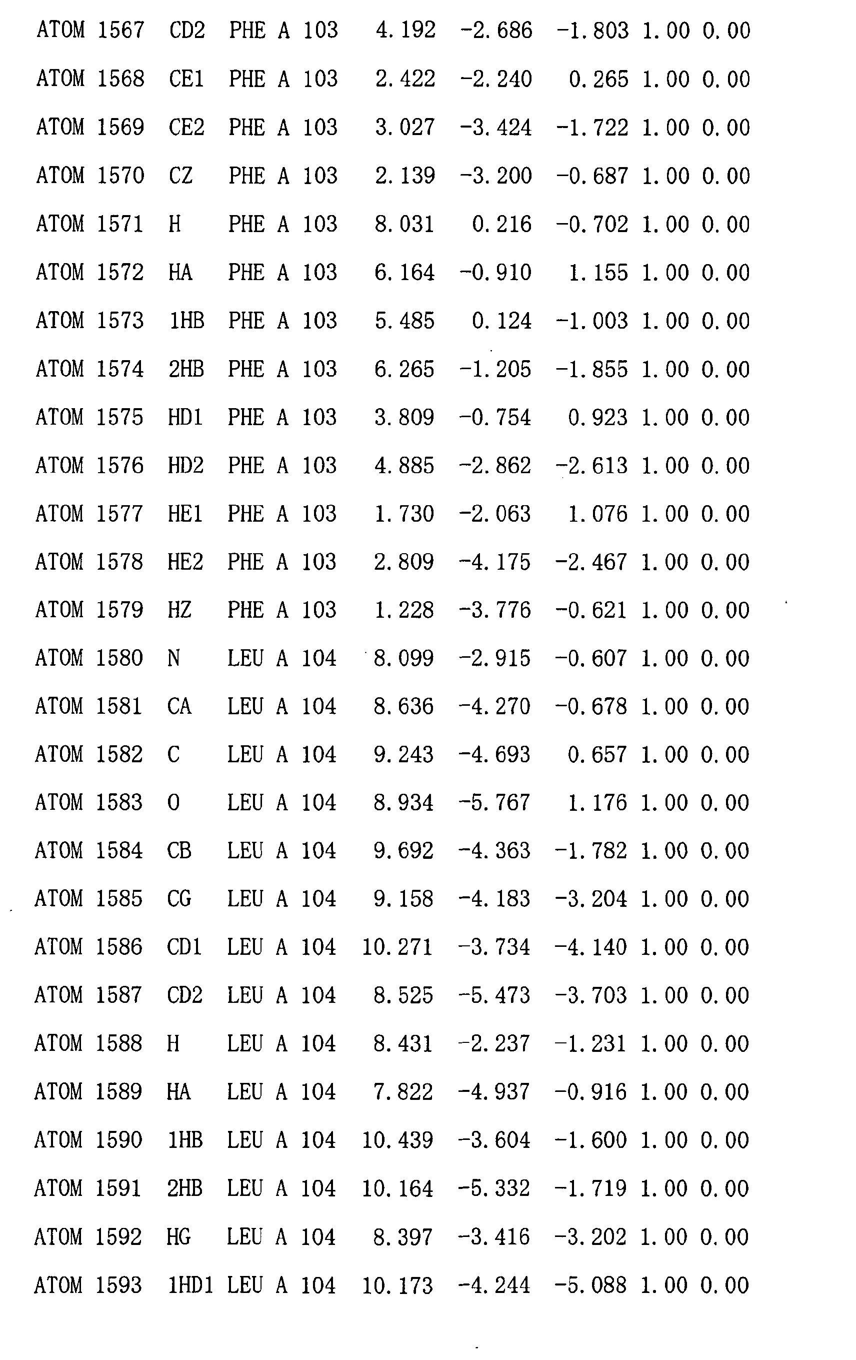

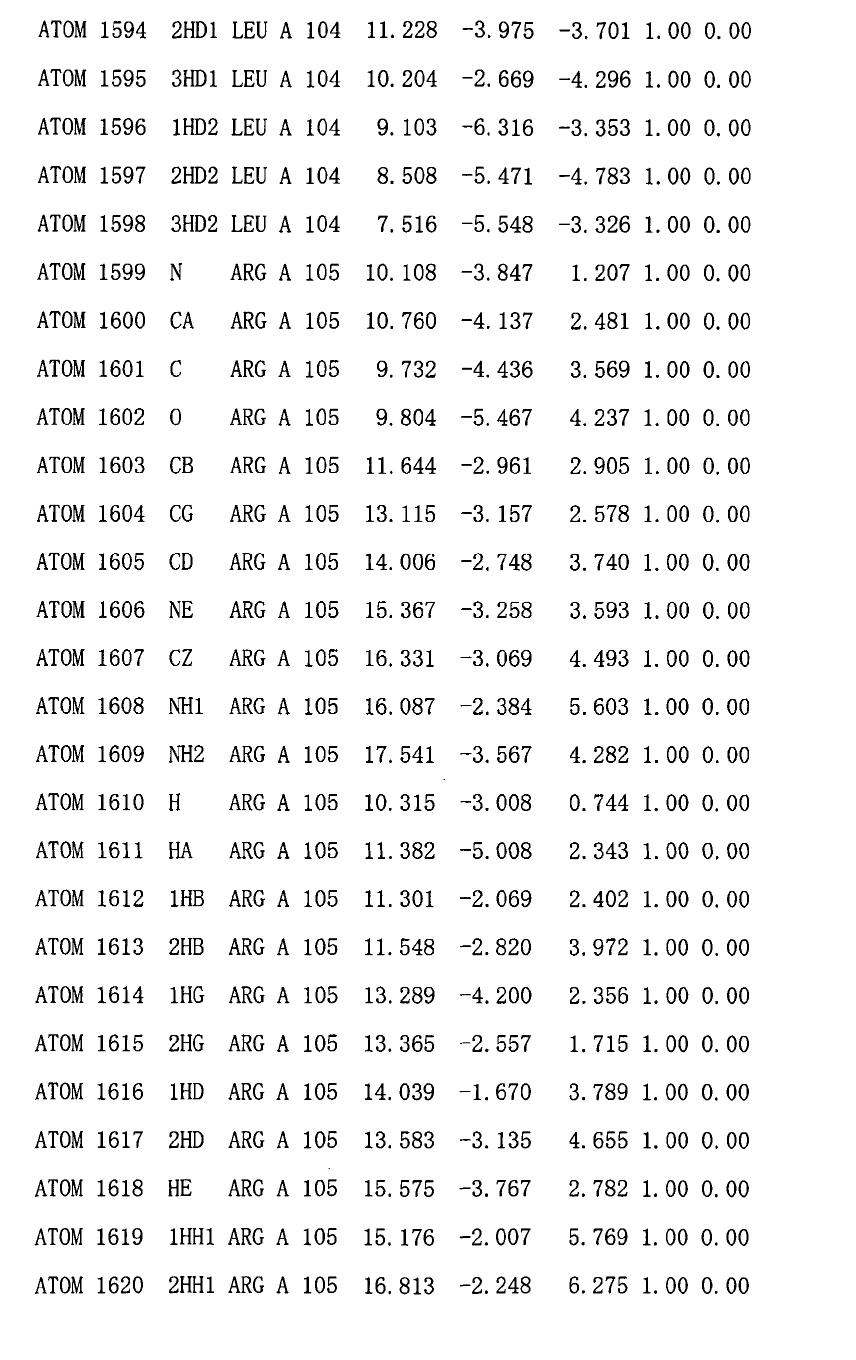

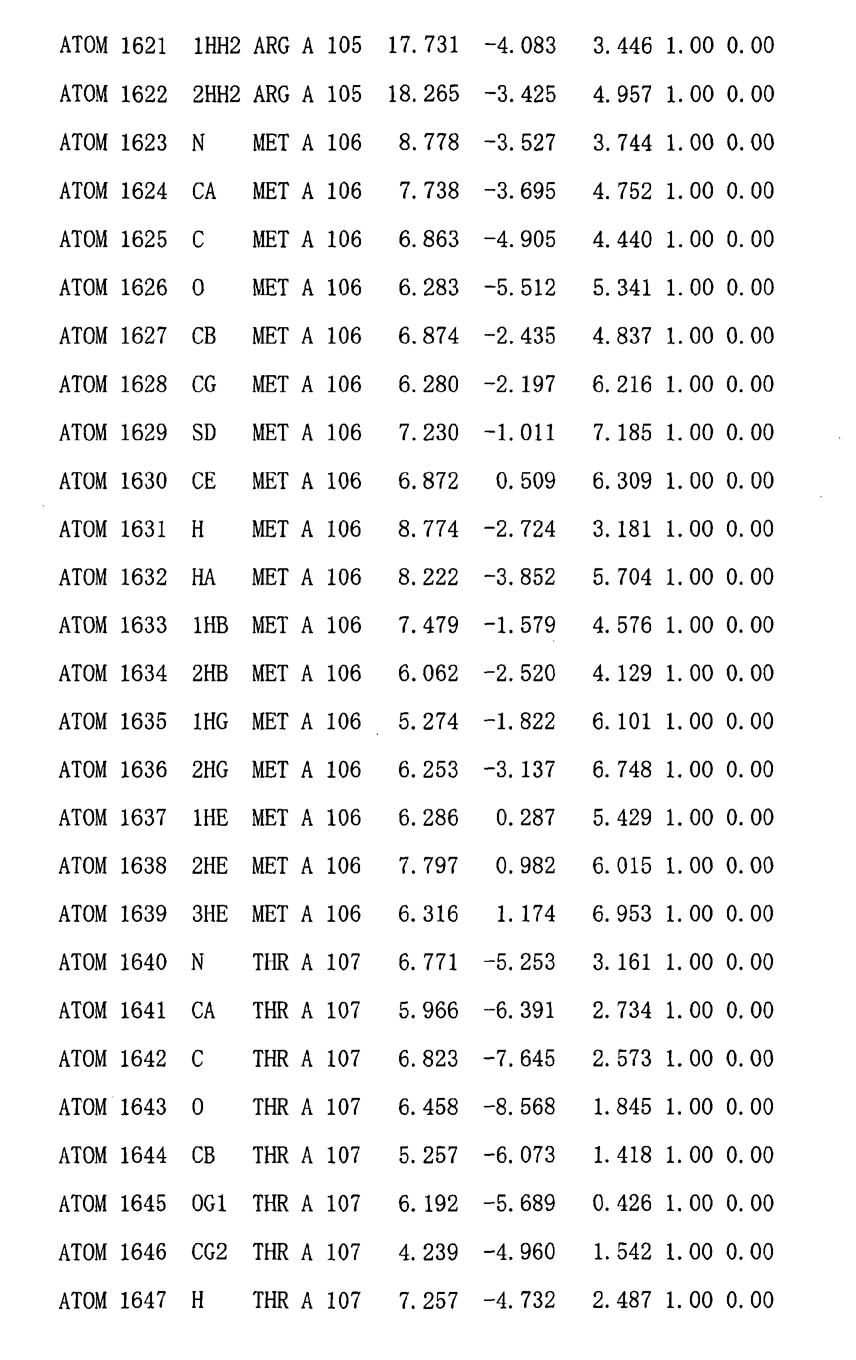

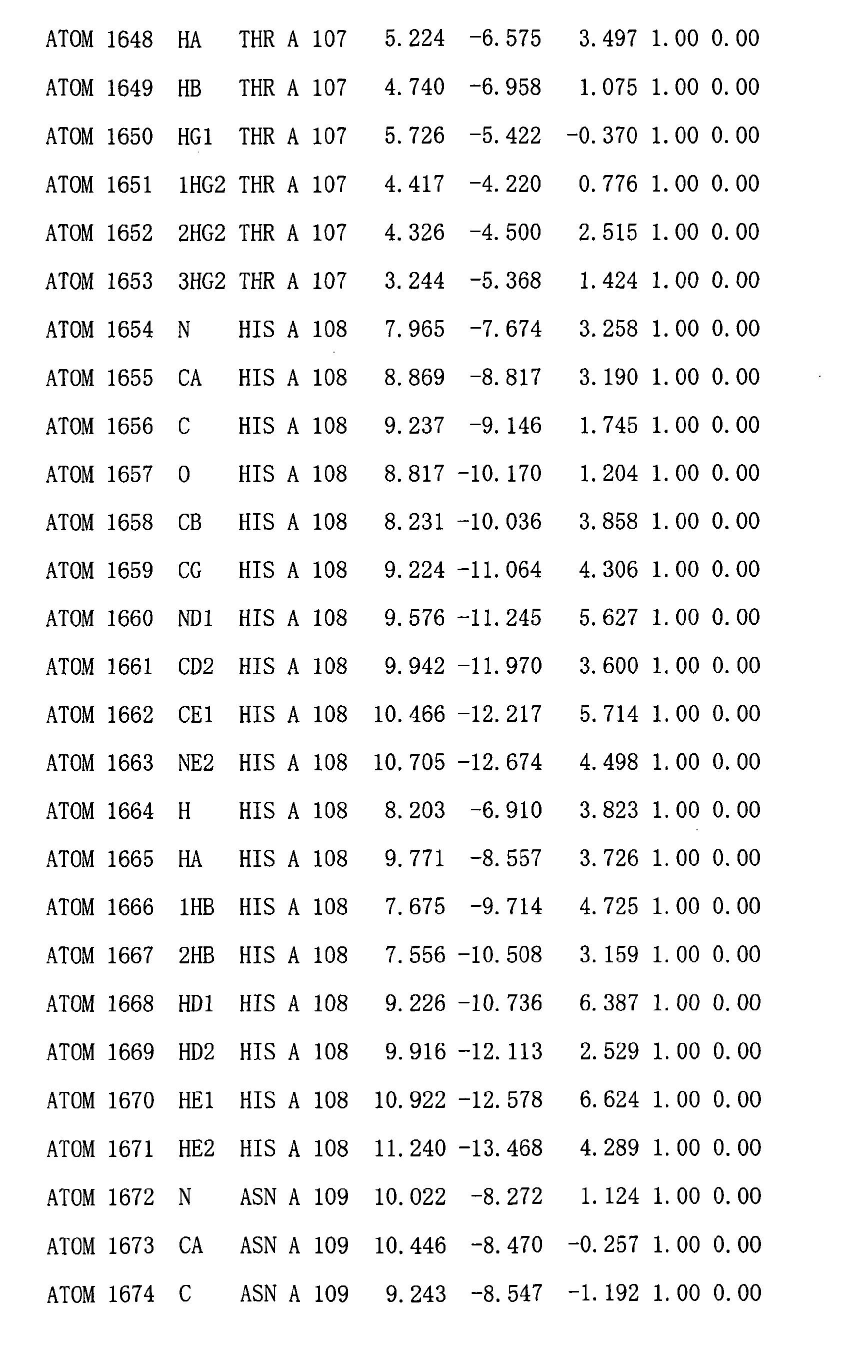

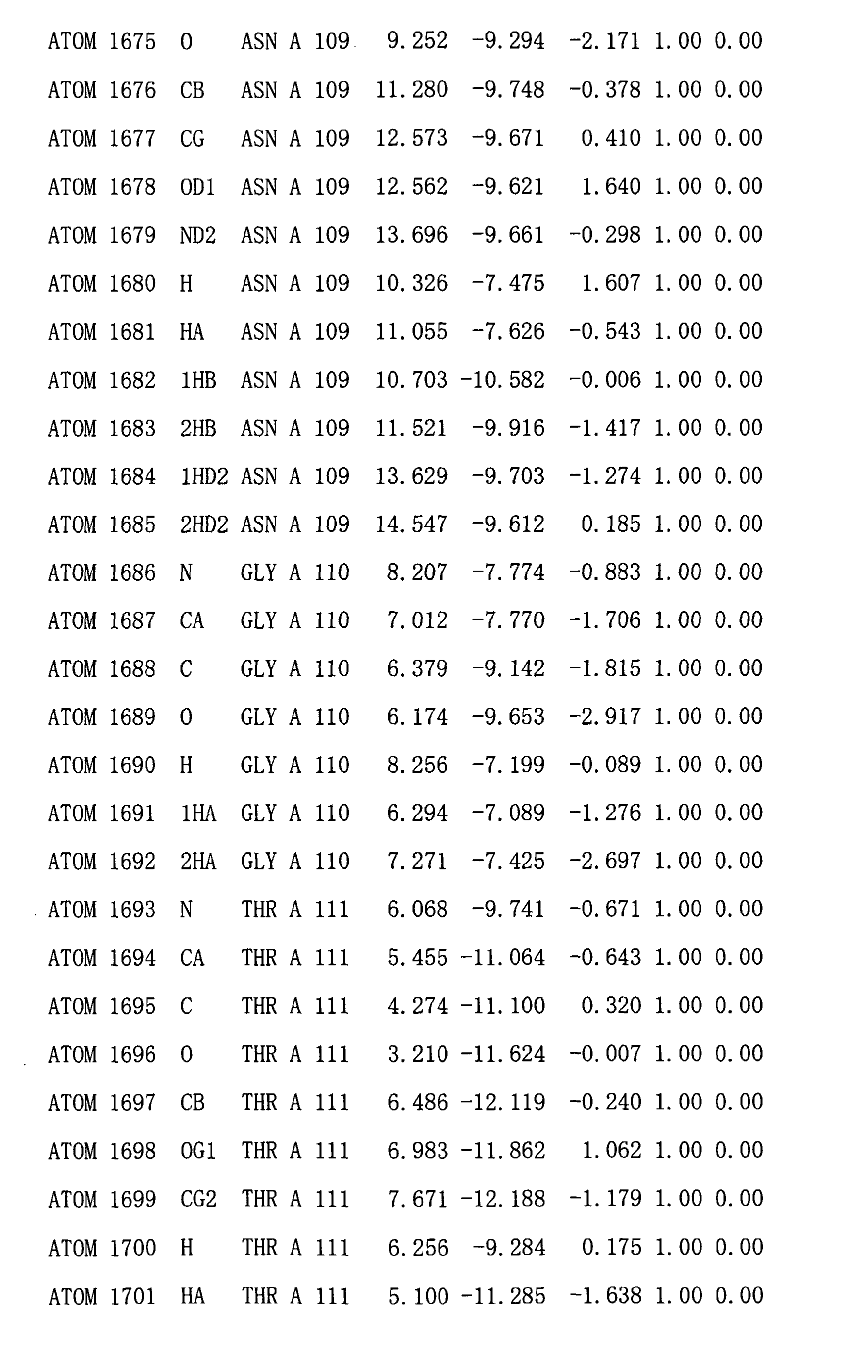

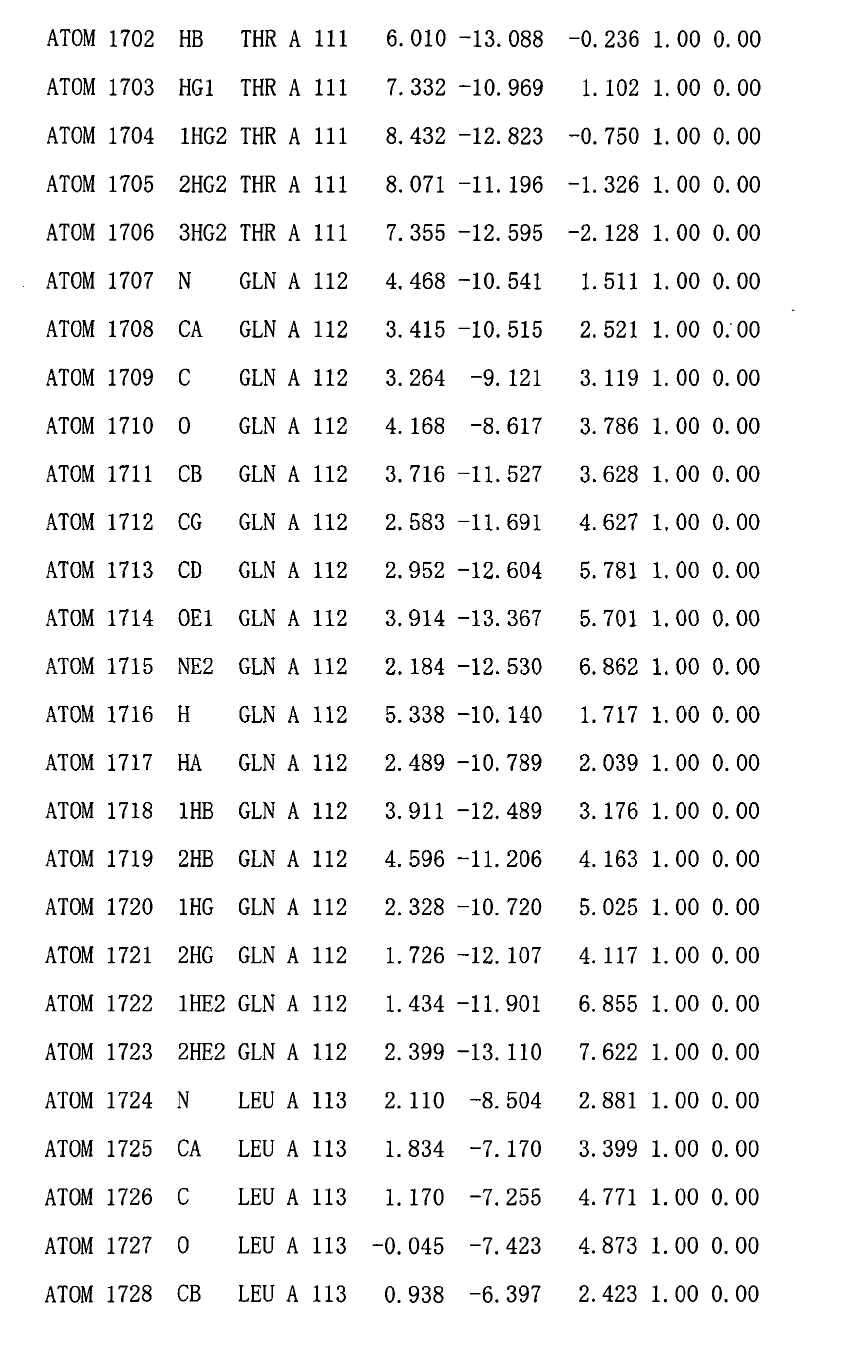

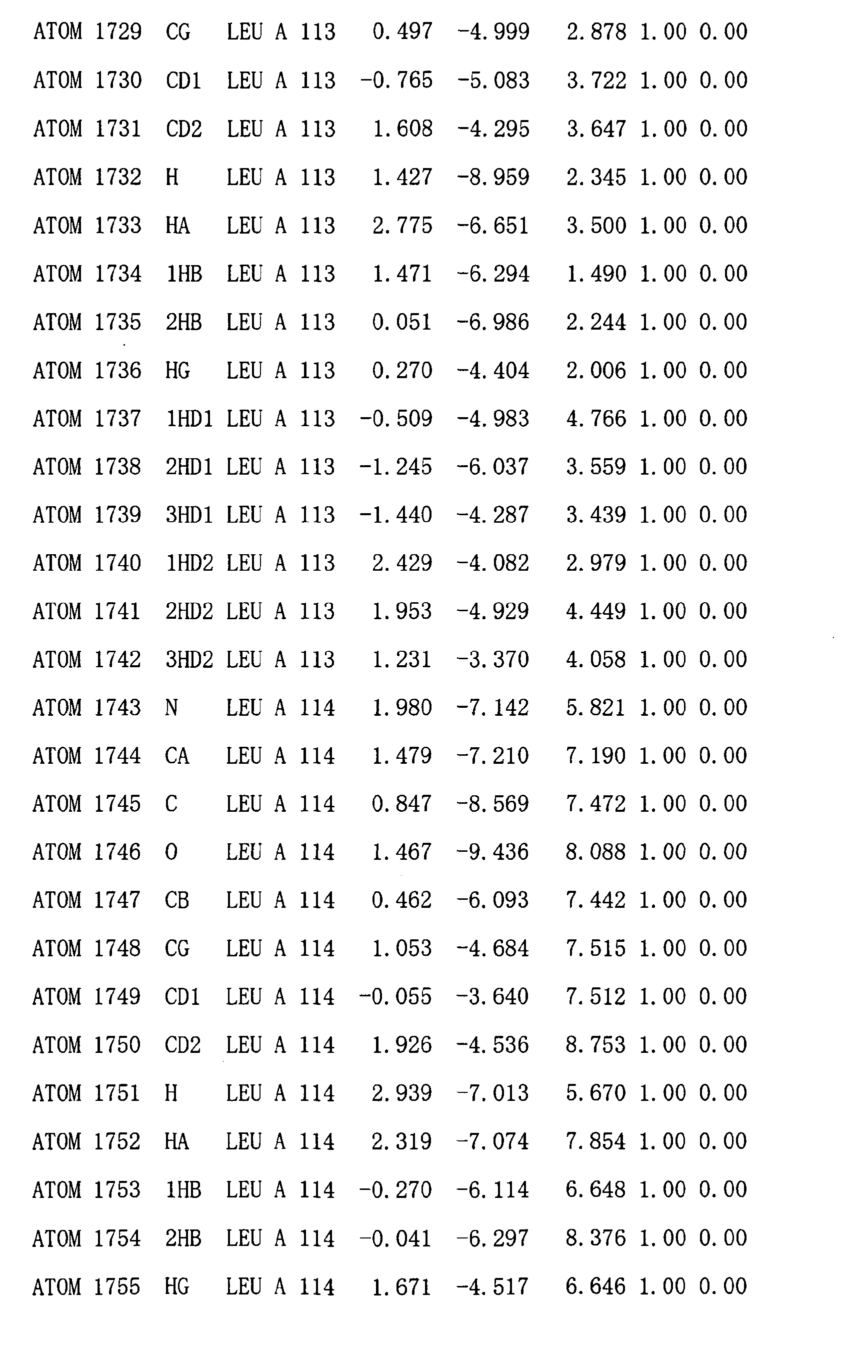

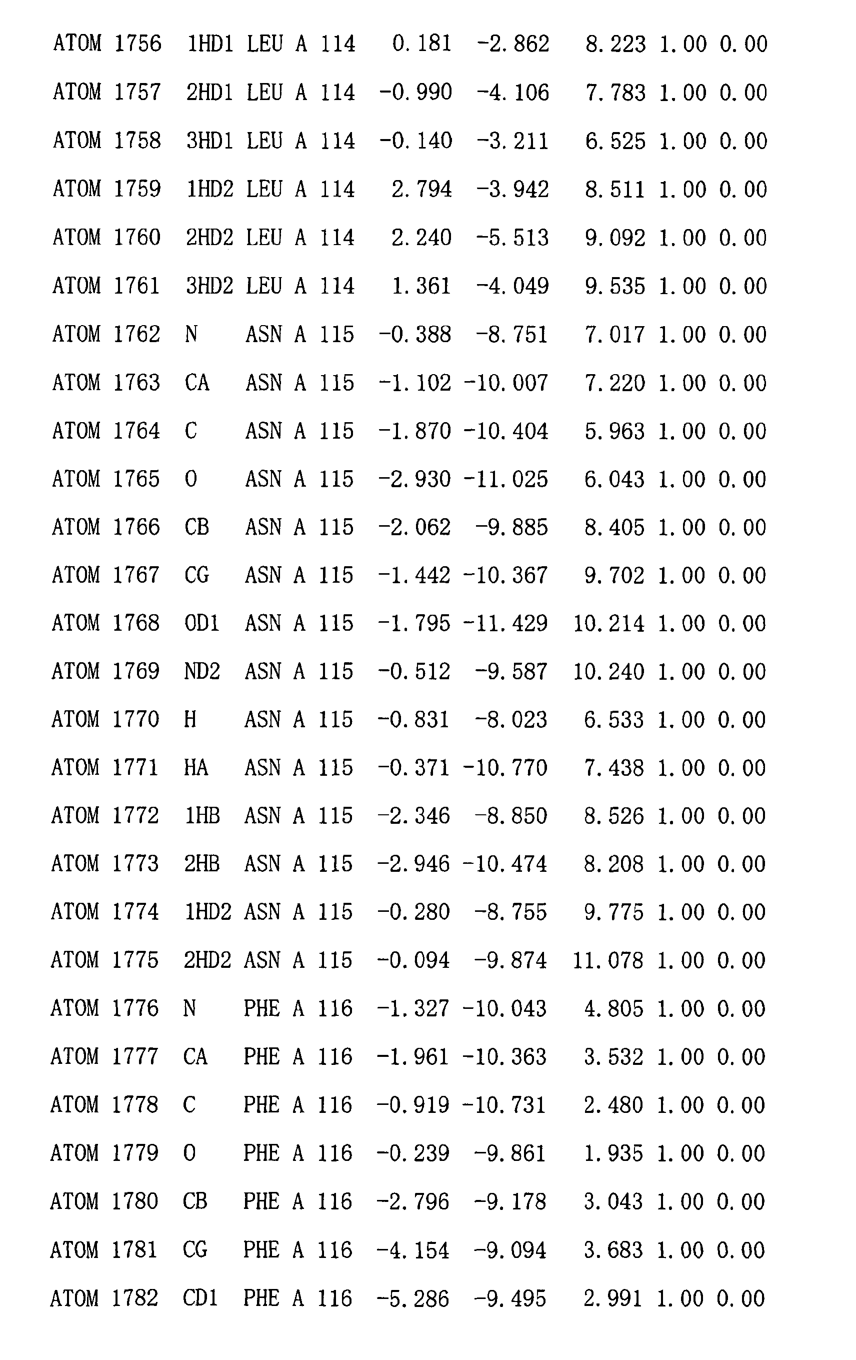

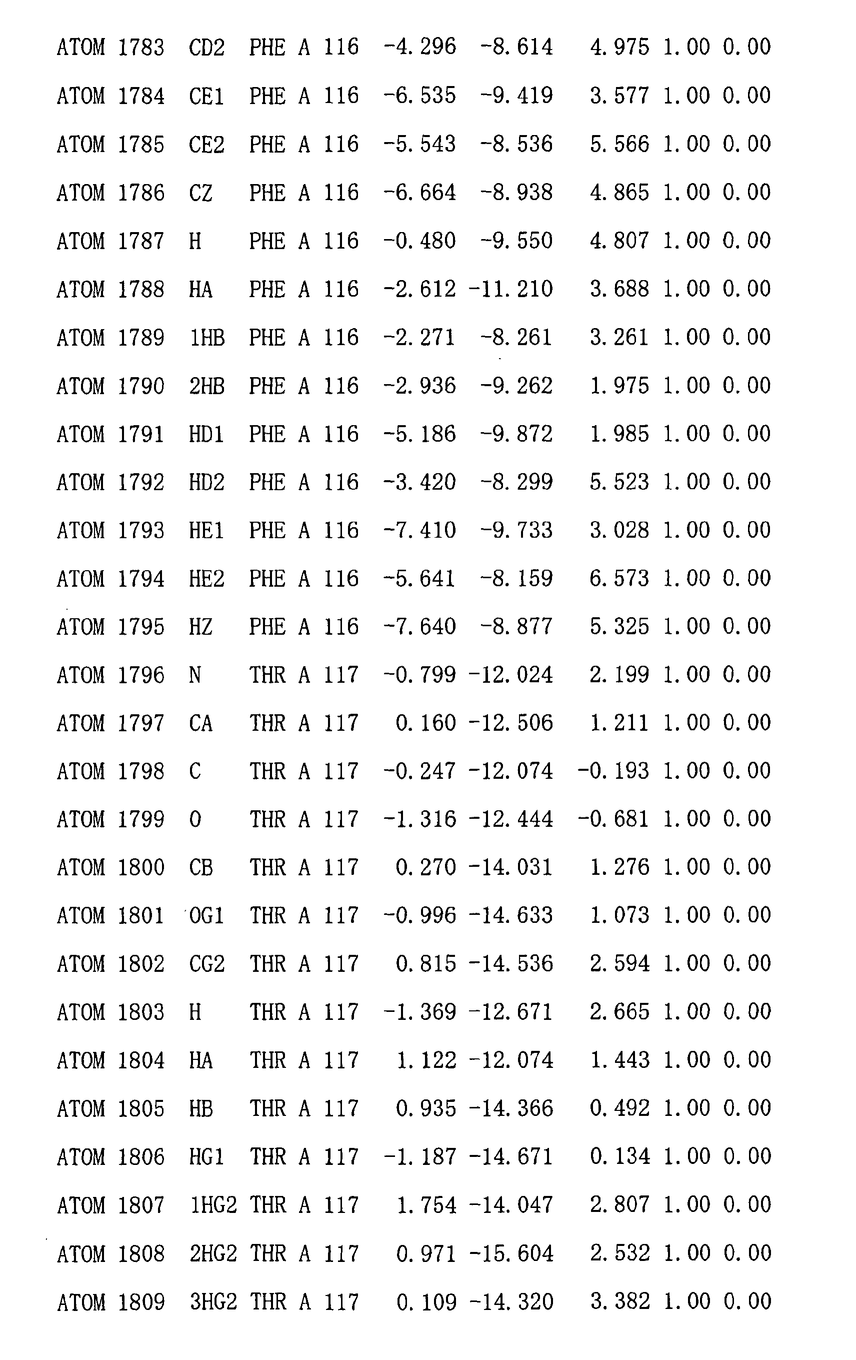

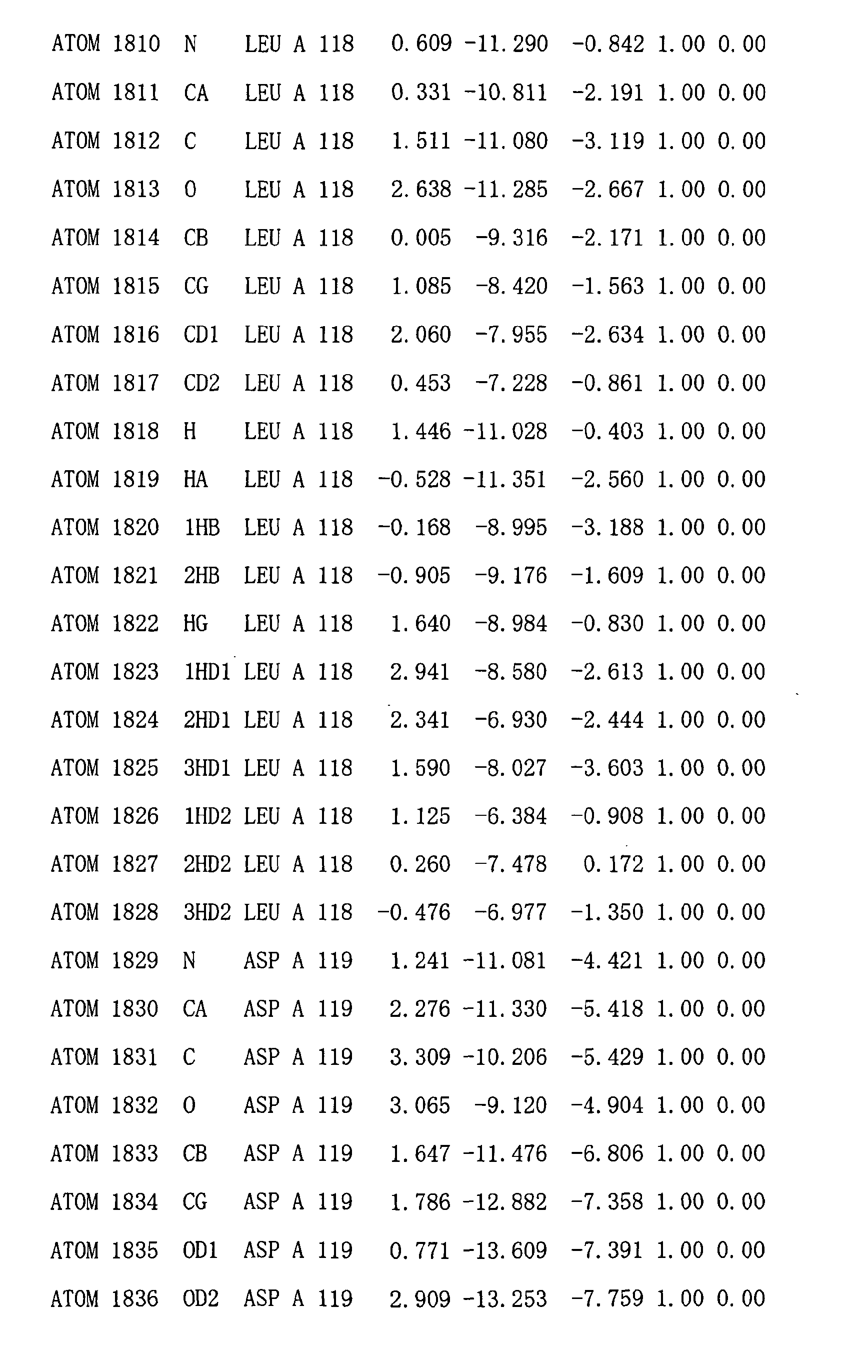

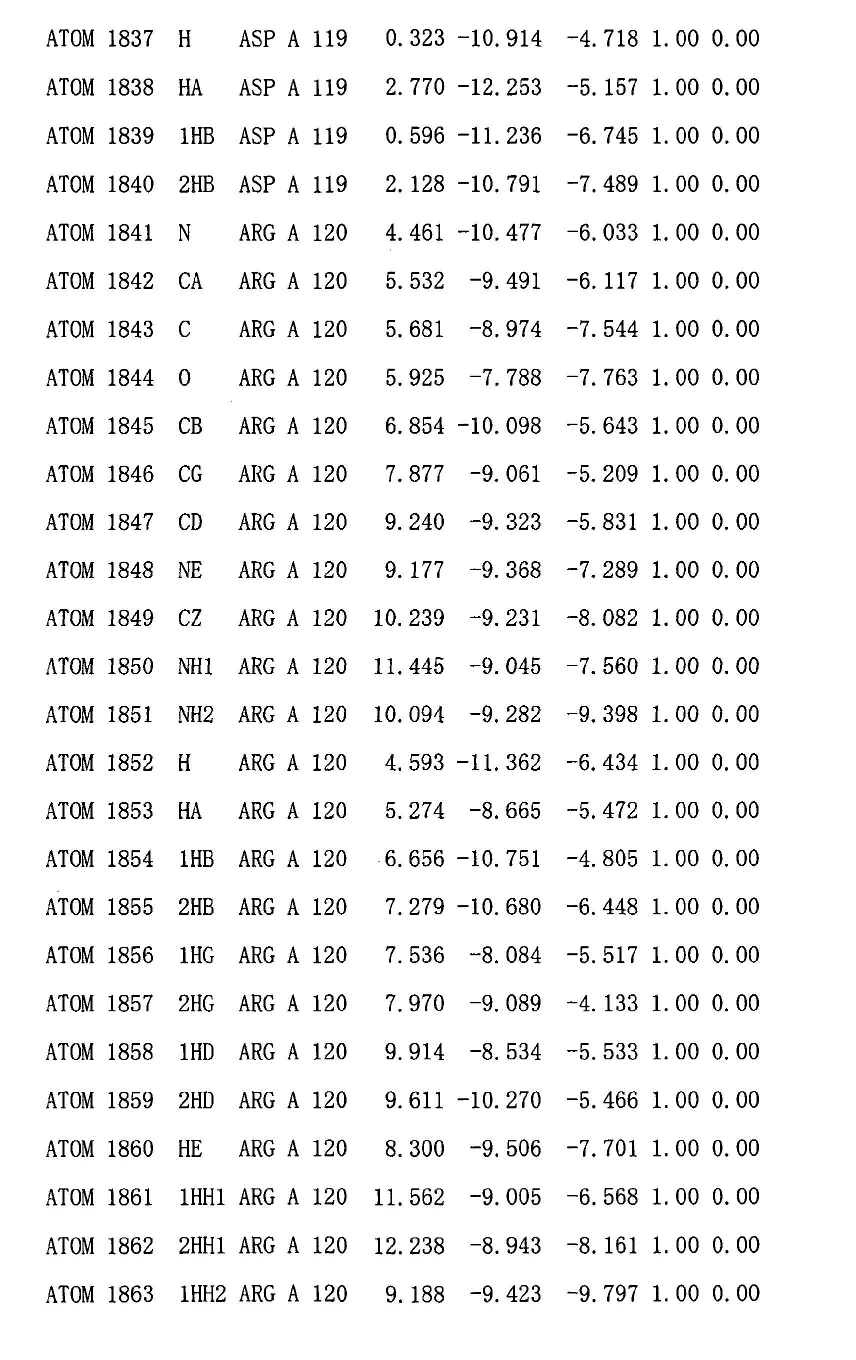

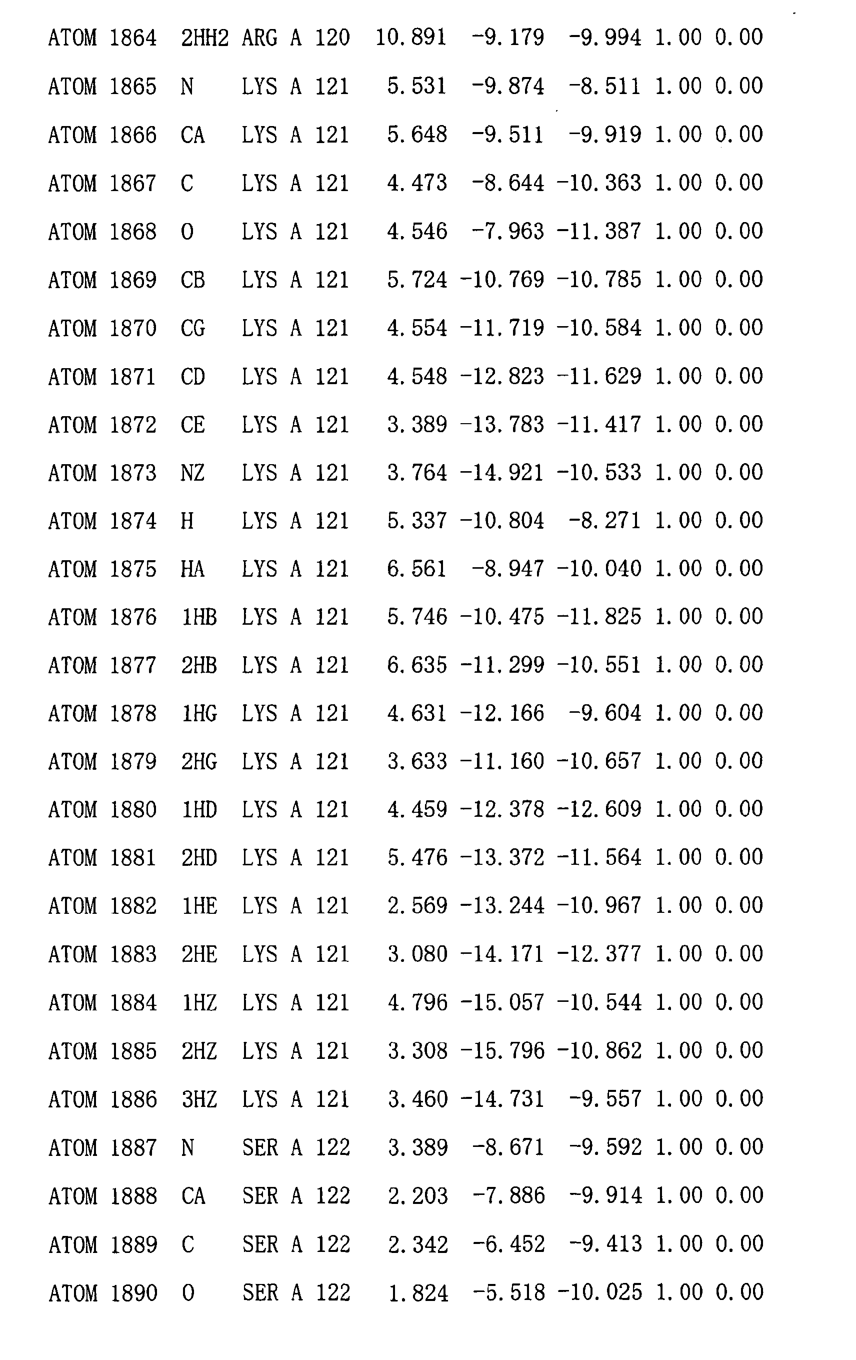

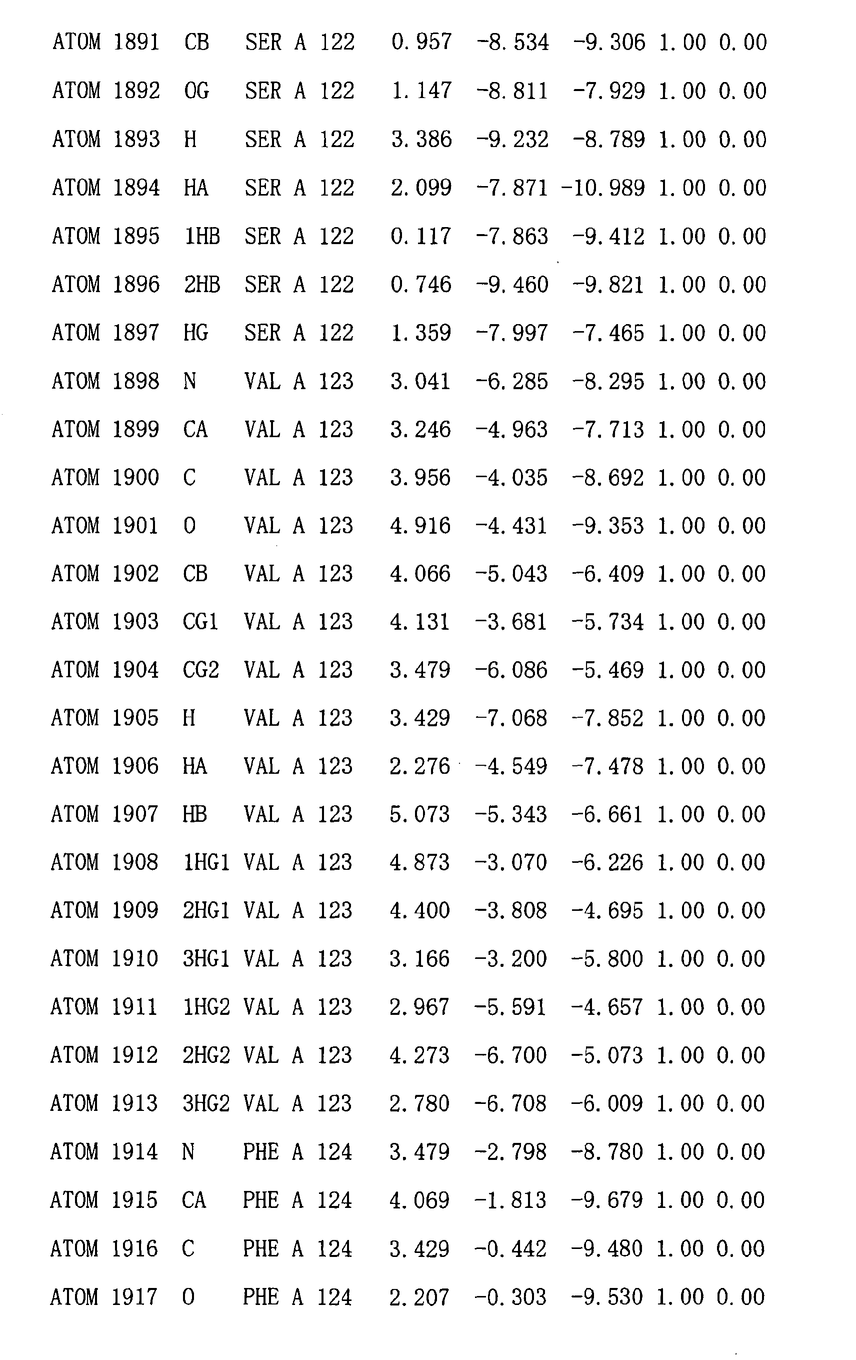

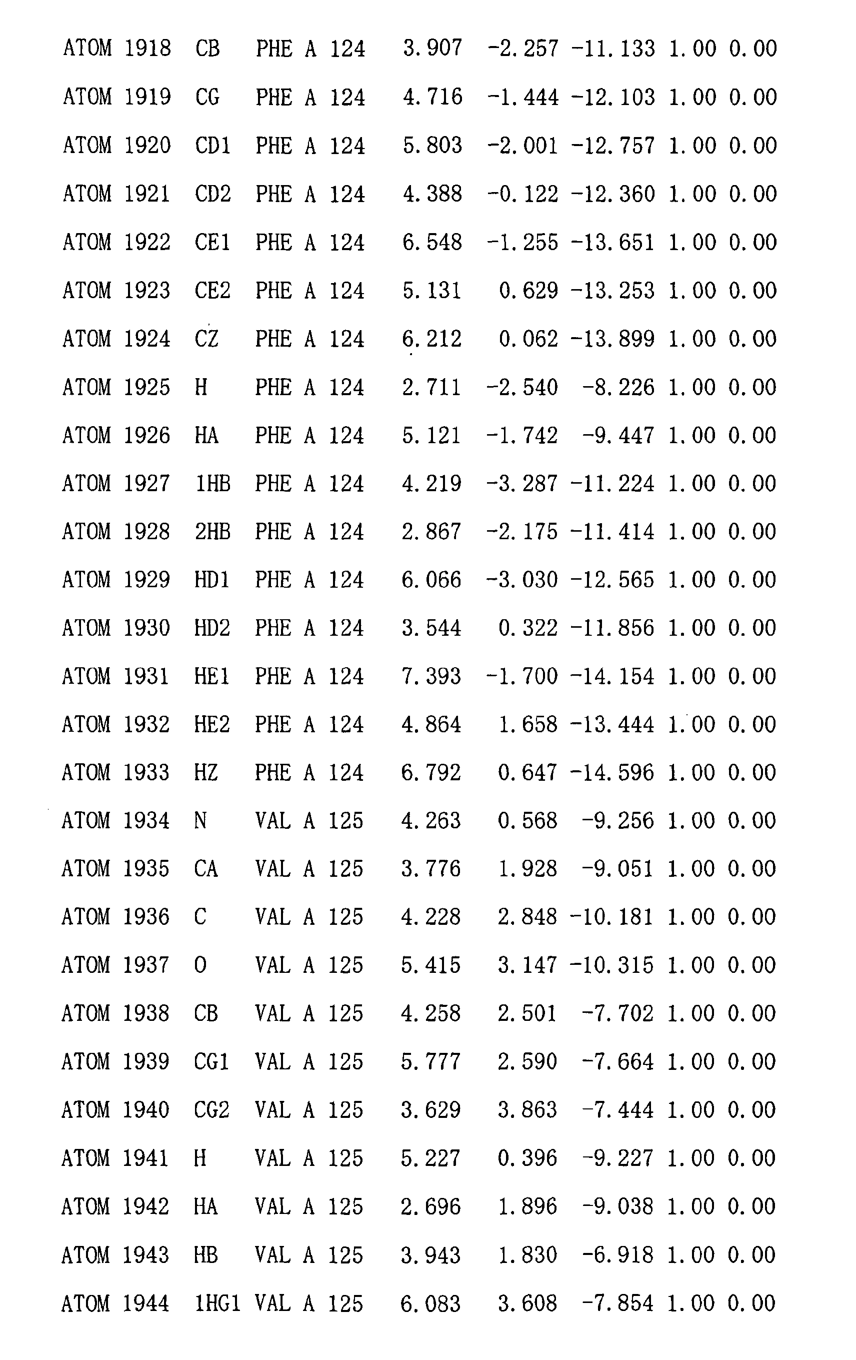

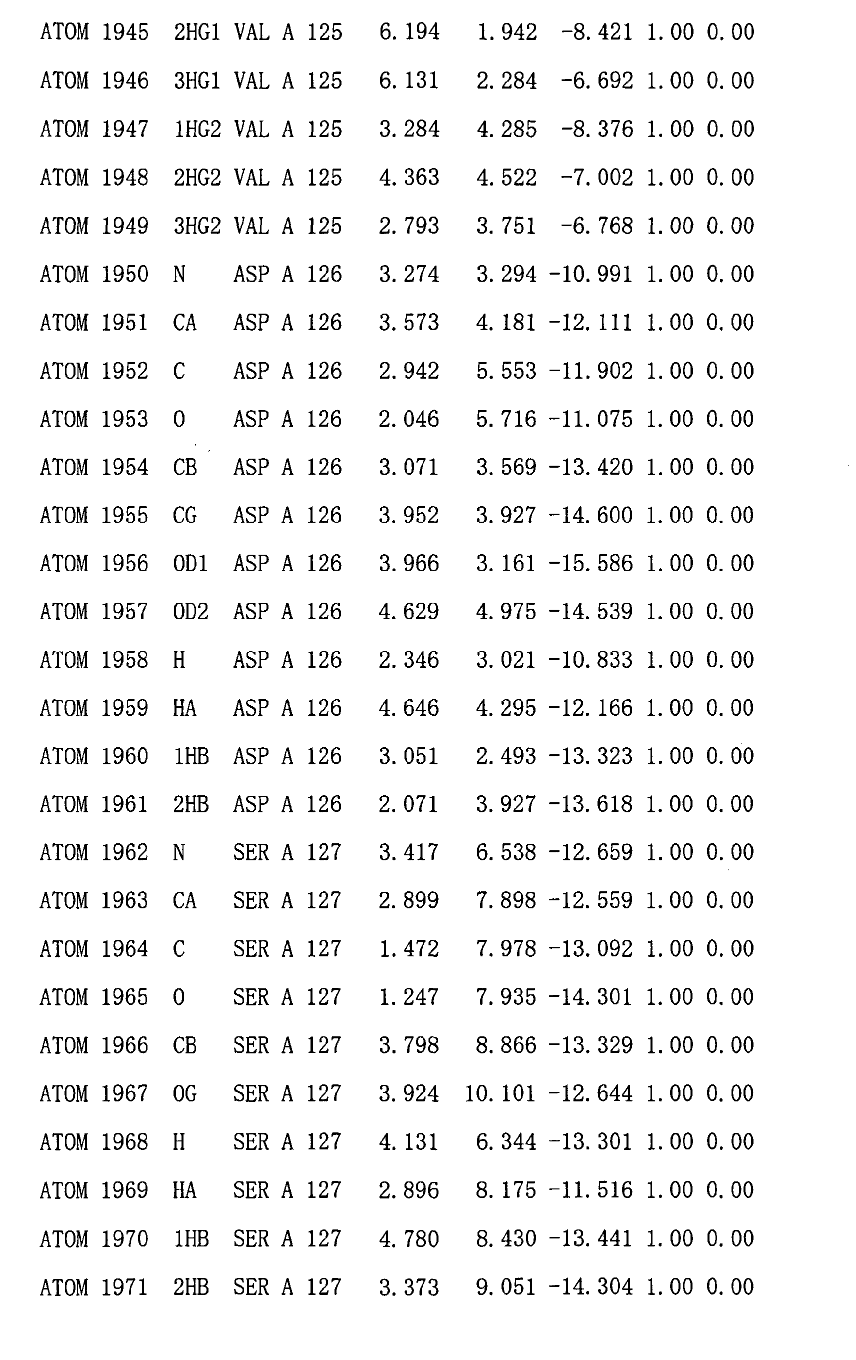

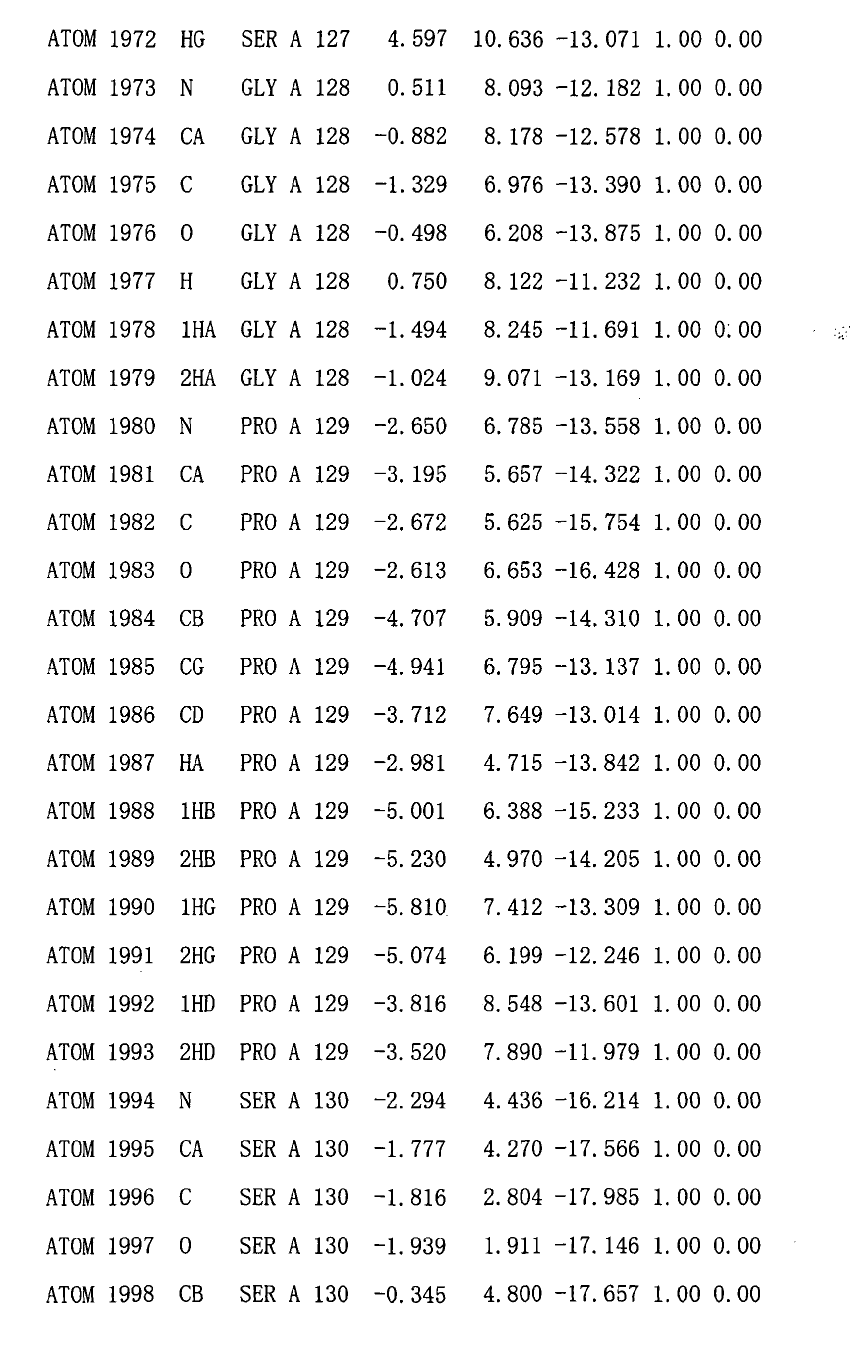

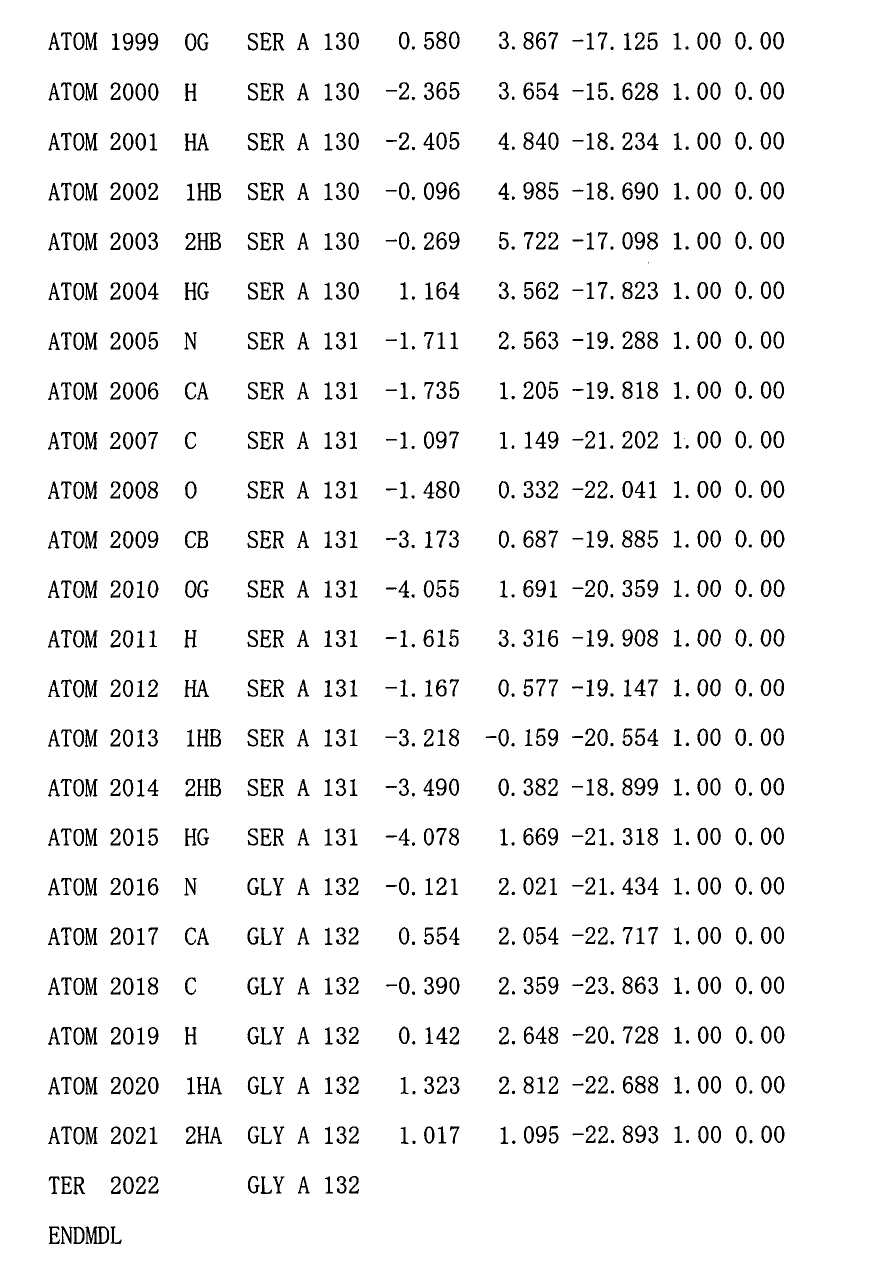

### Three-Dimensional Structure Coordinate 6

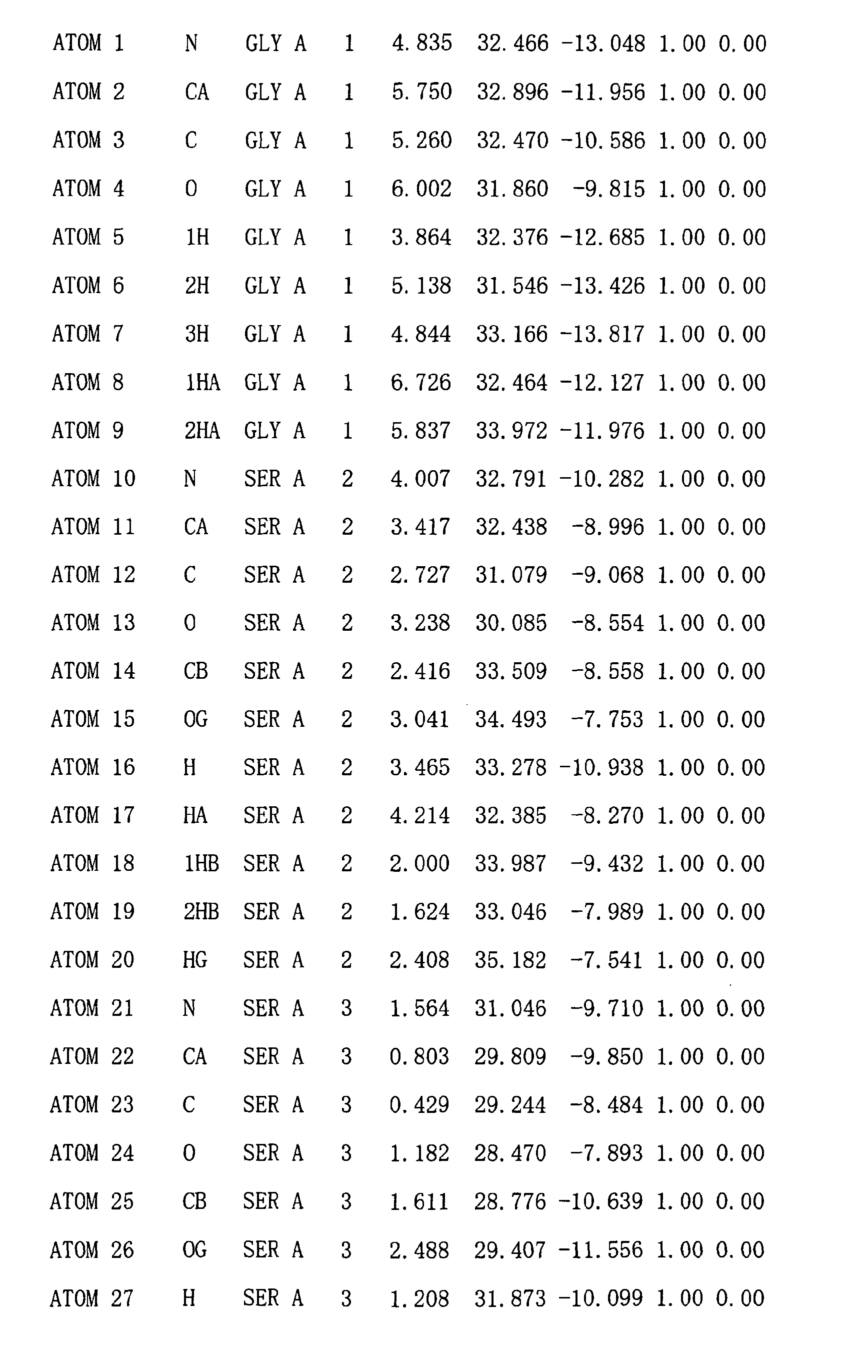

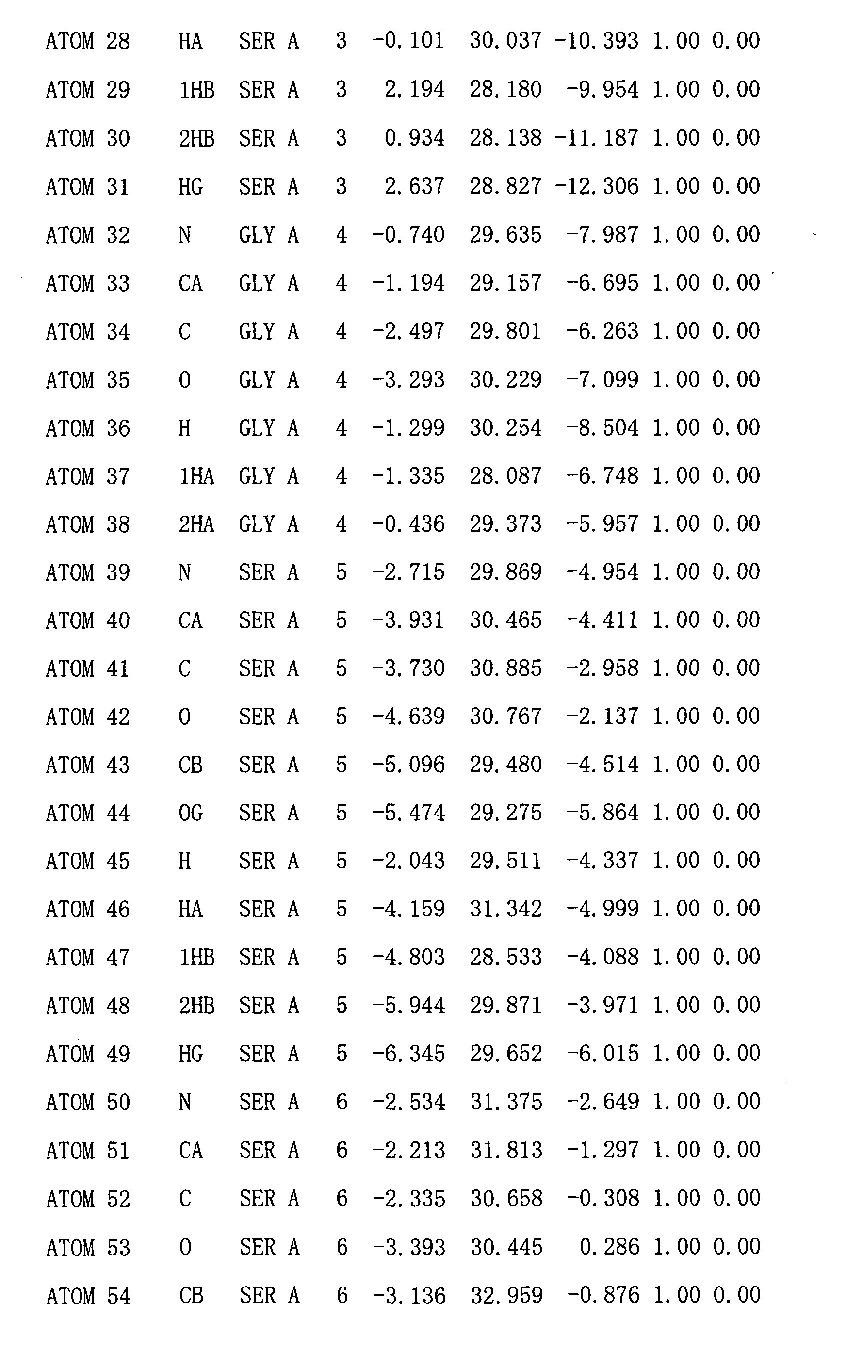

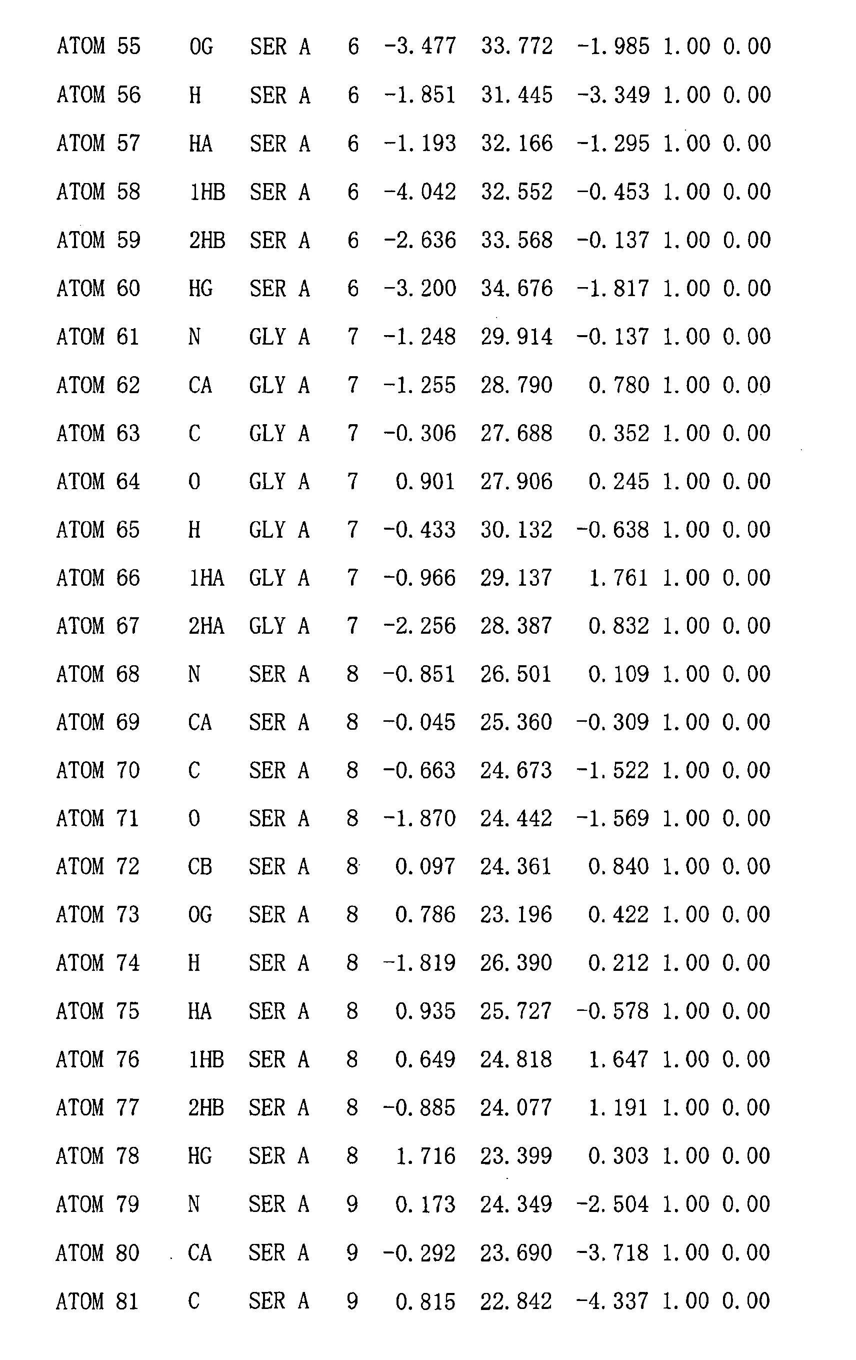

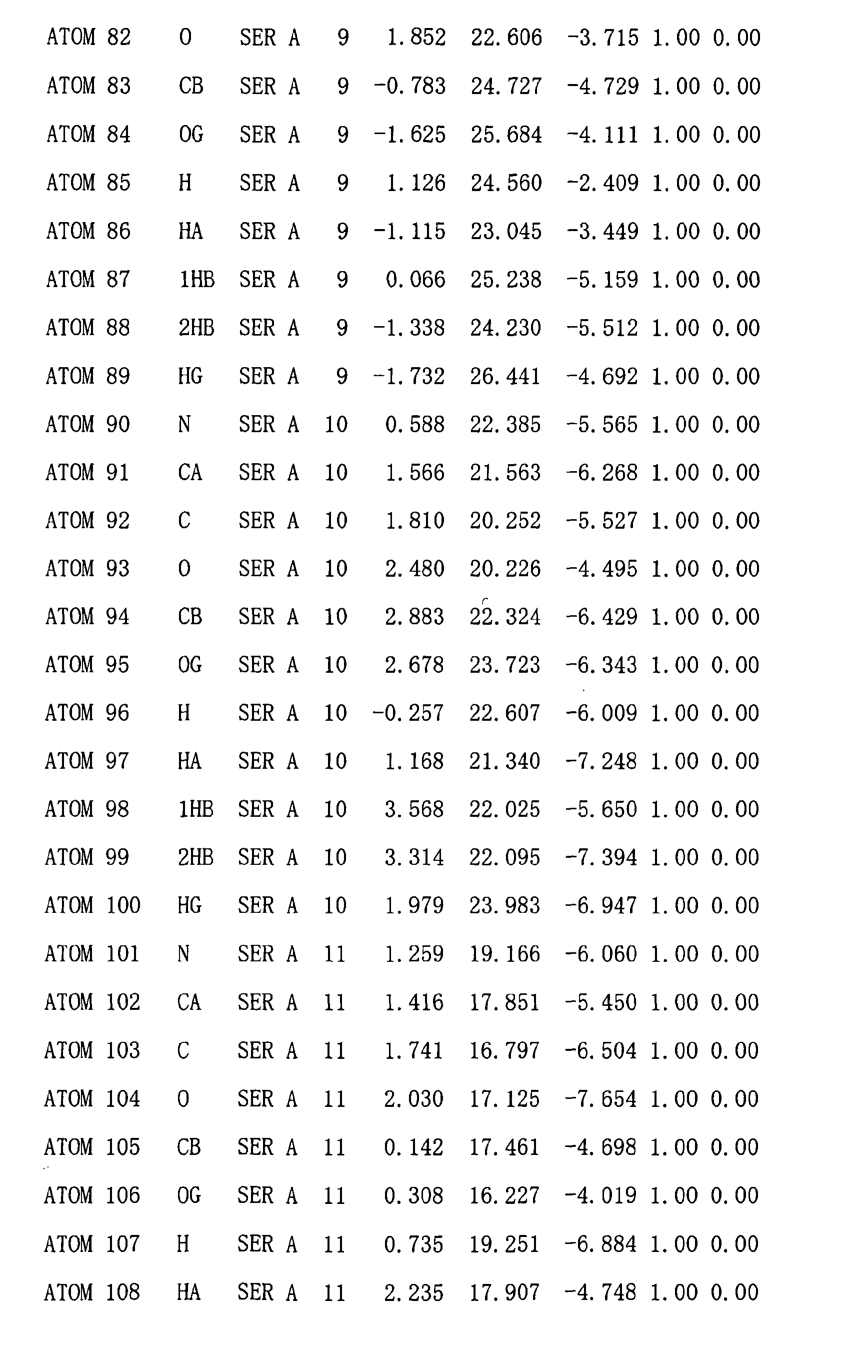

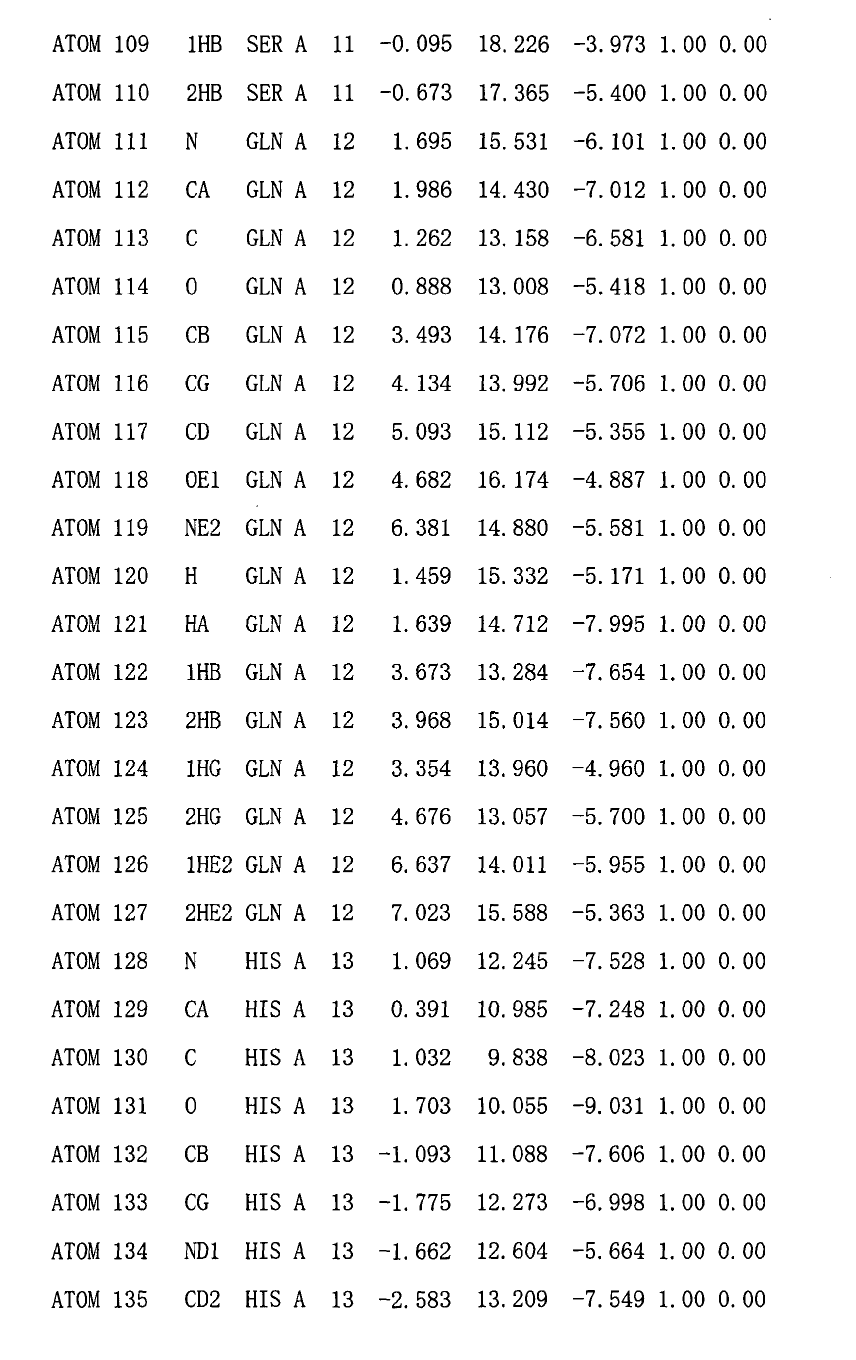

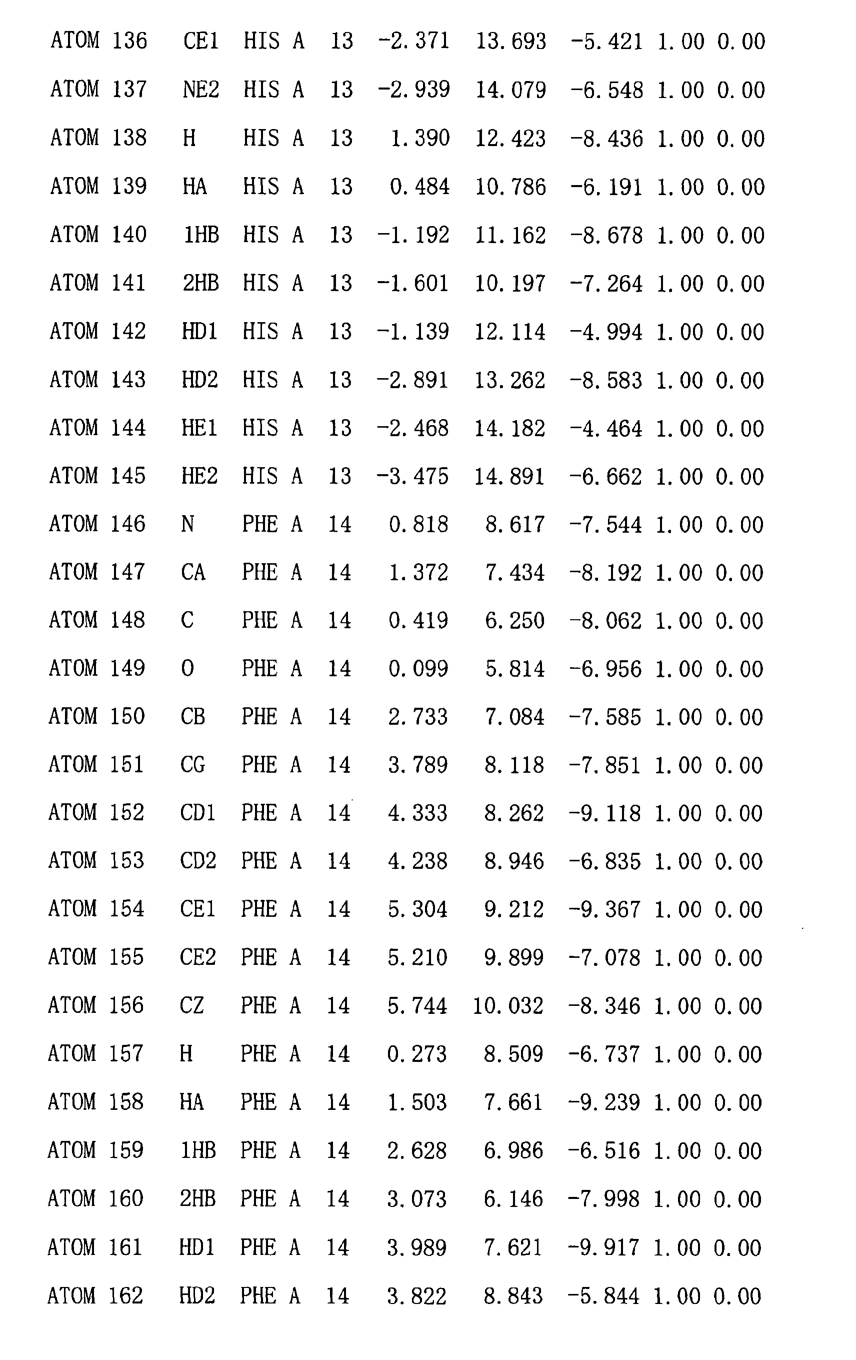

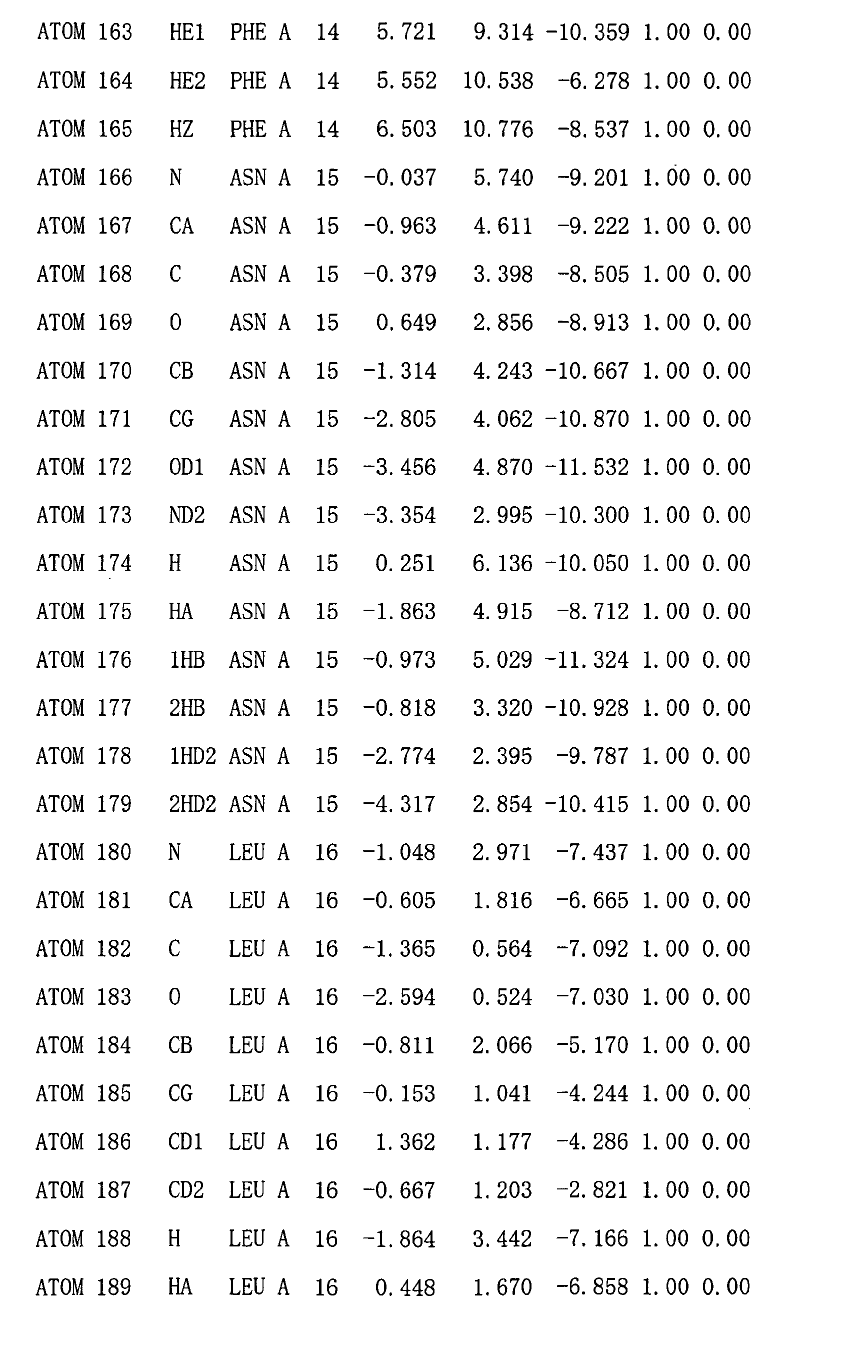

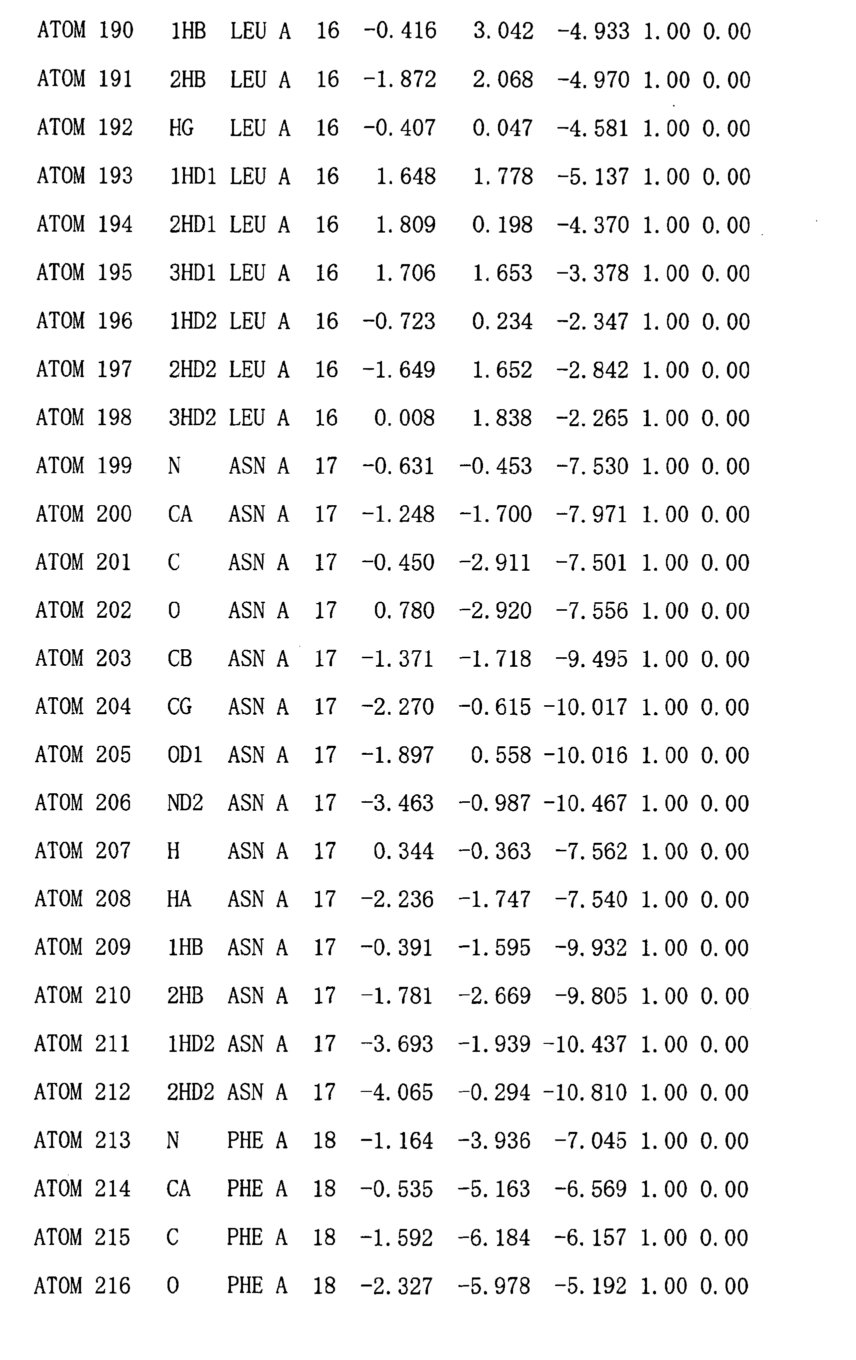

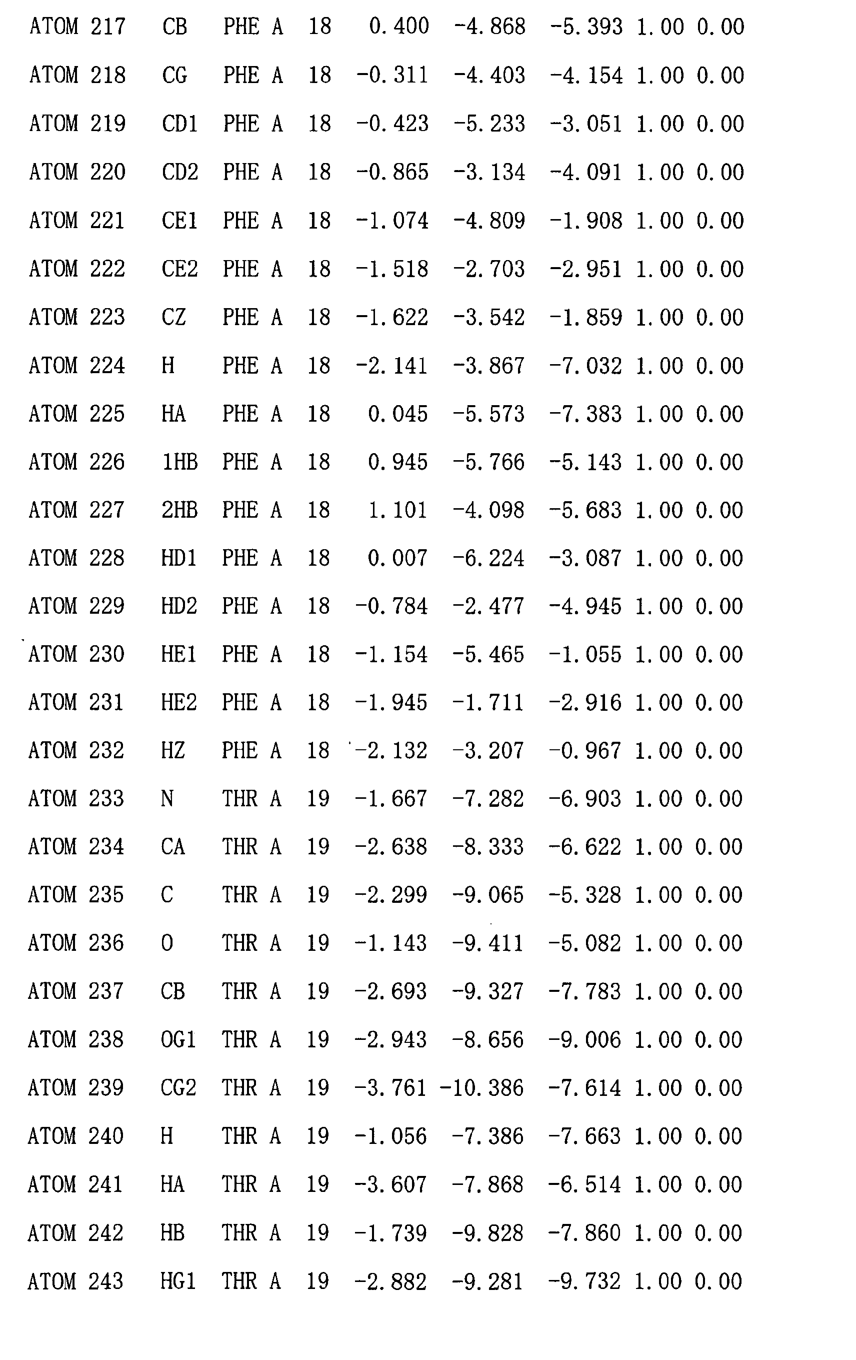

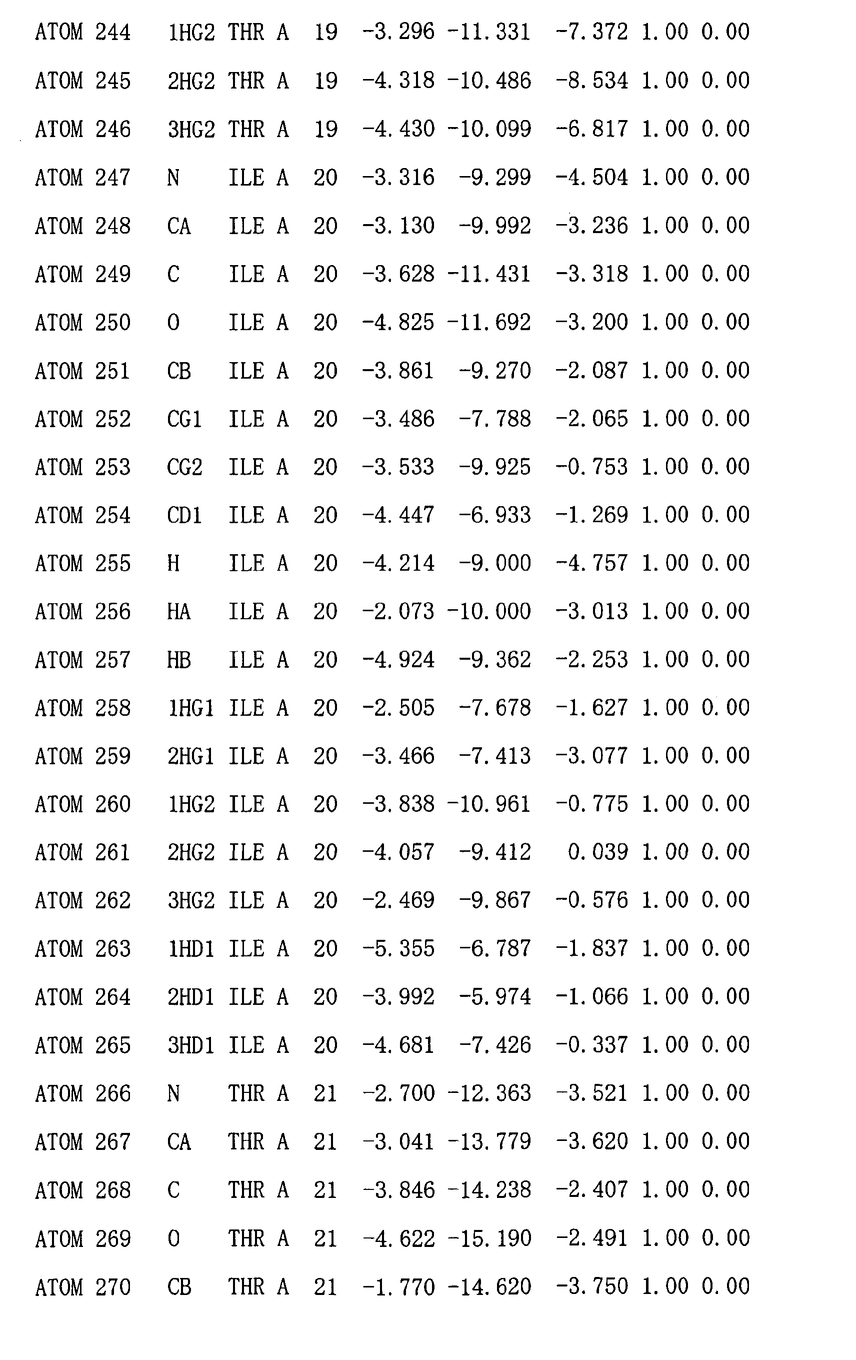

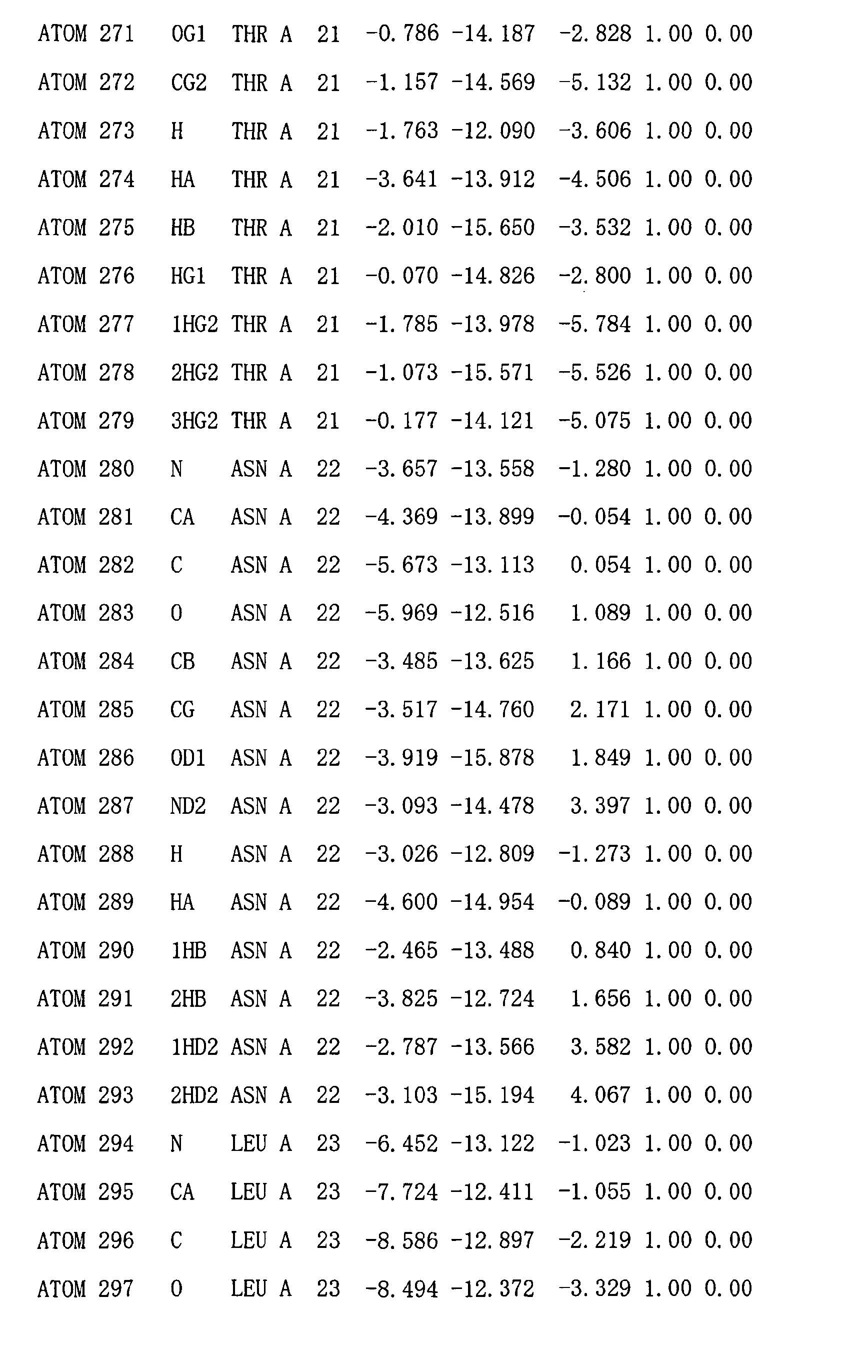

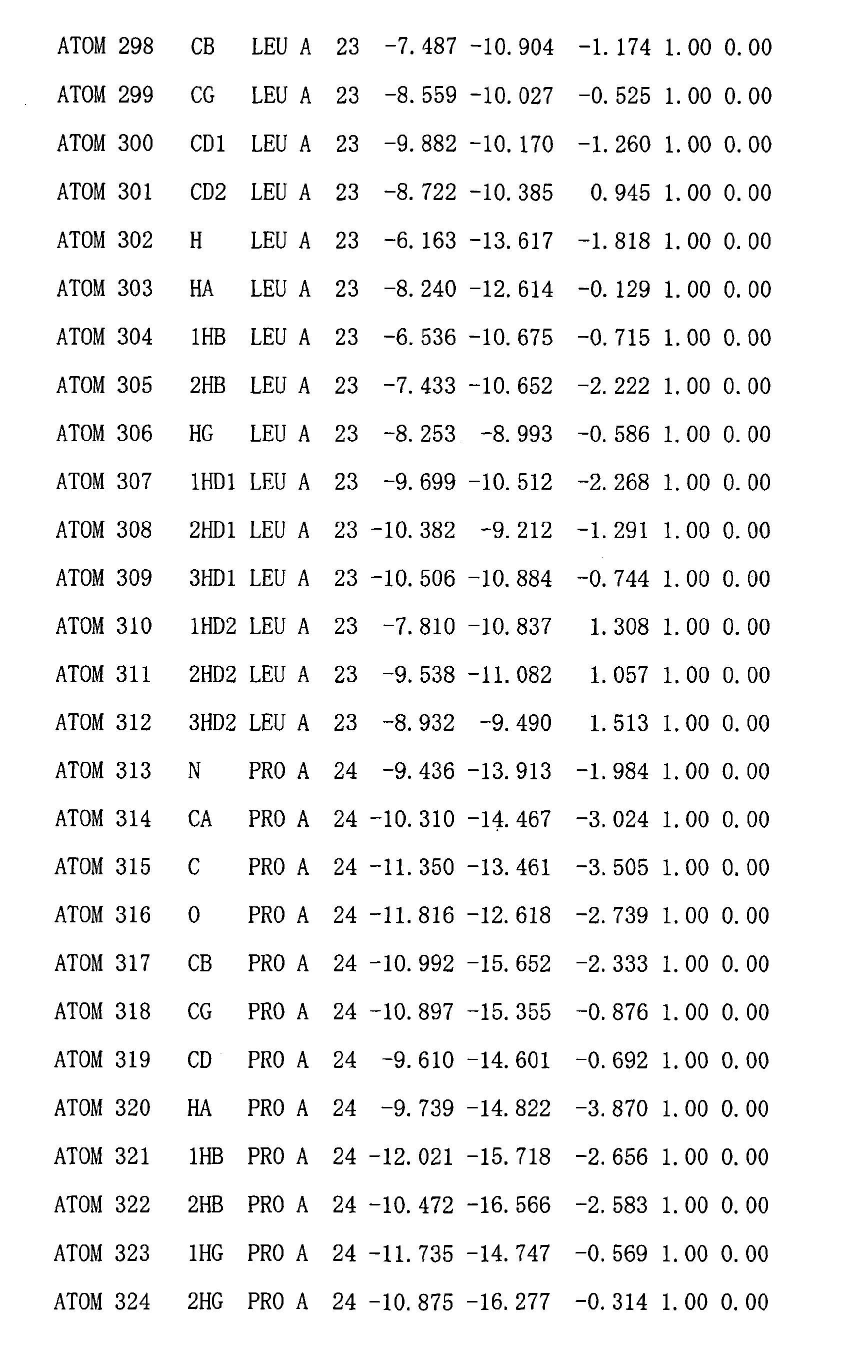

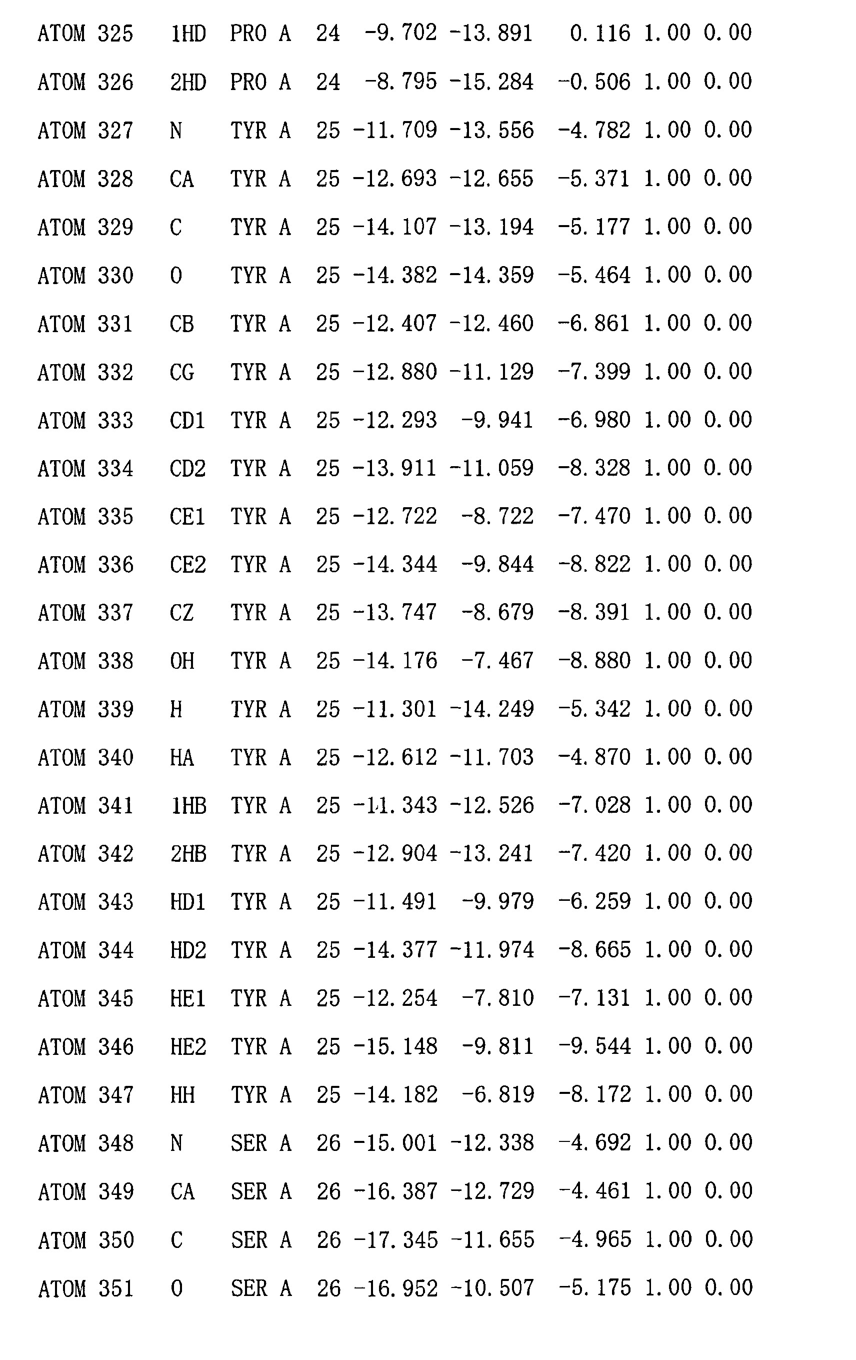

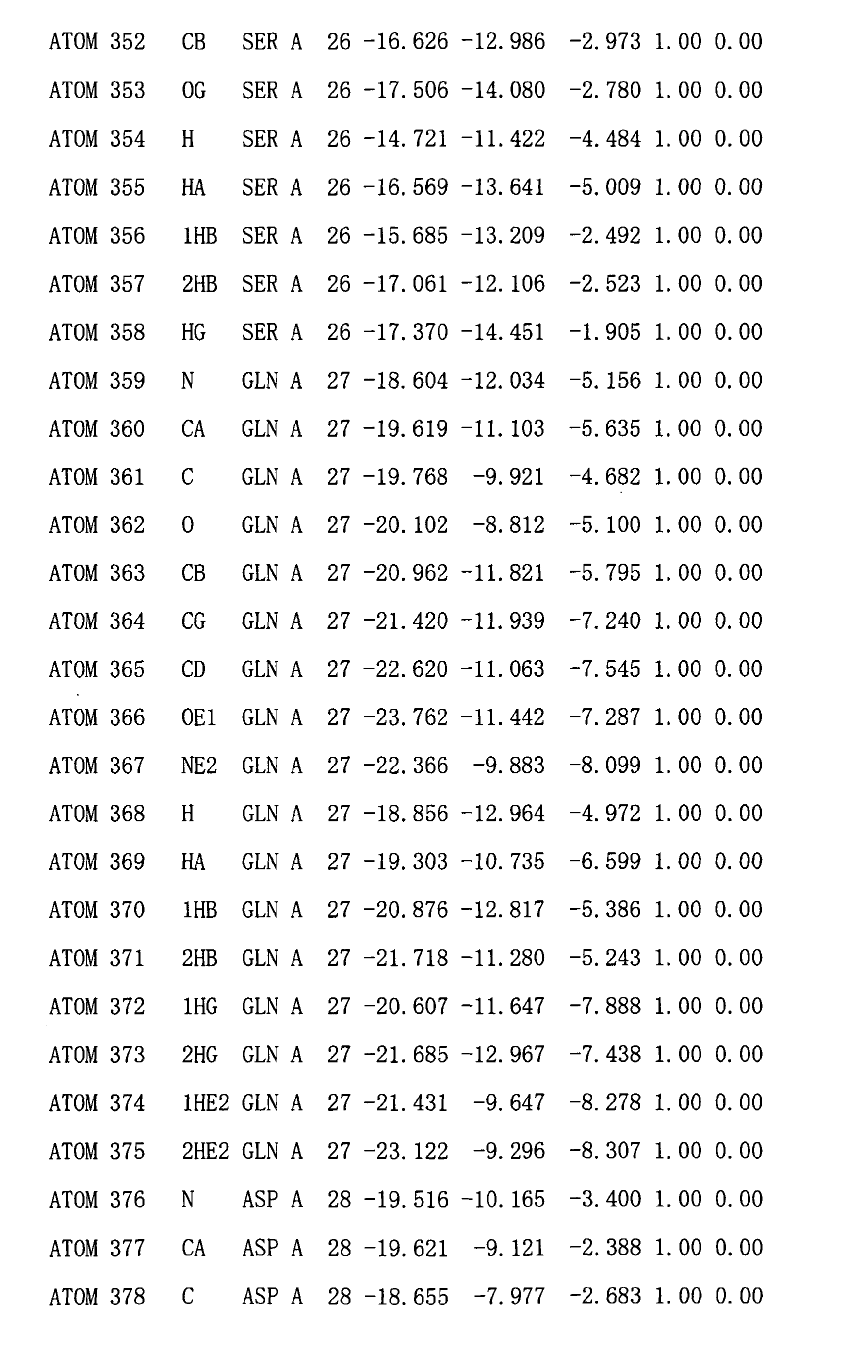

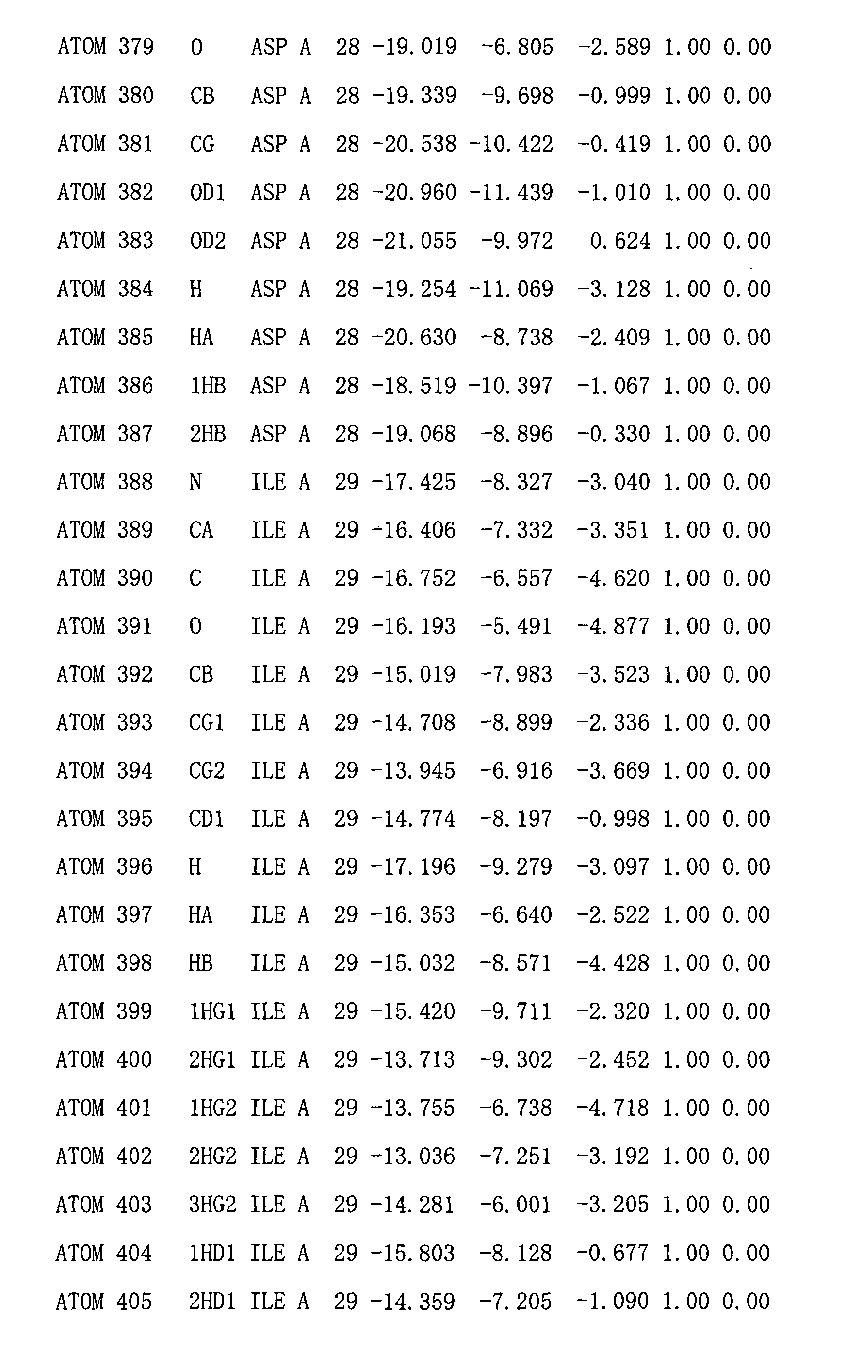

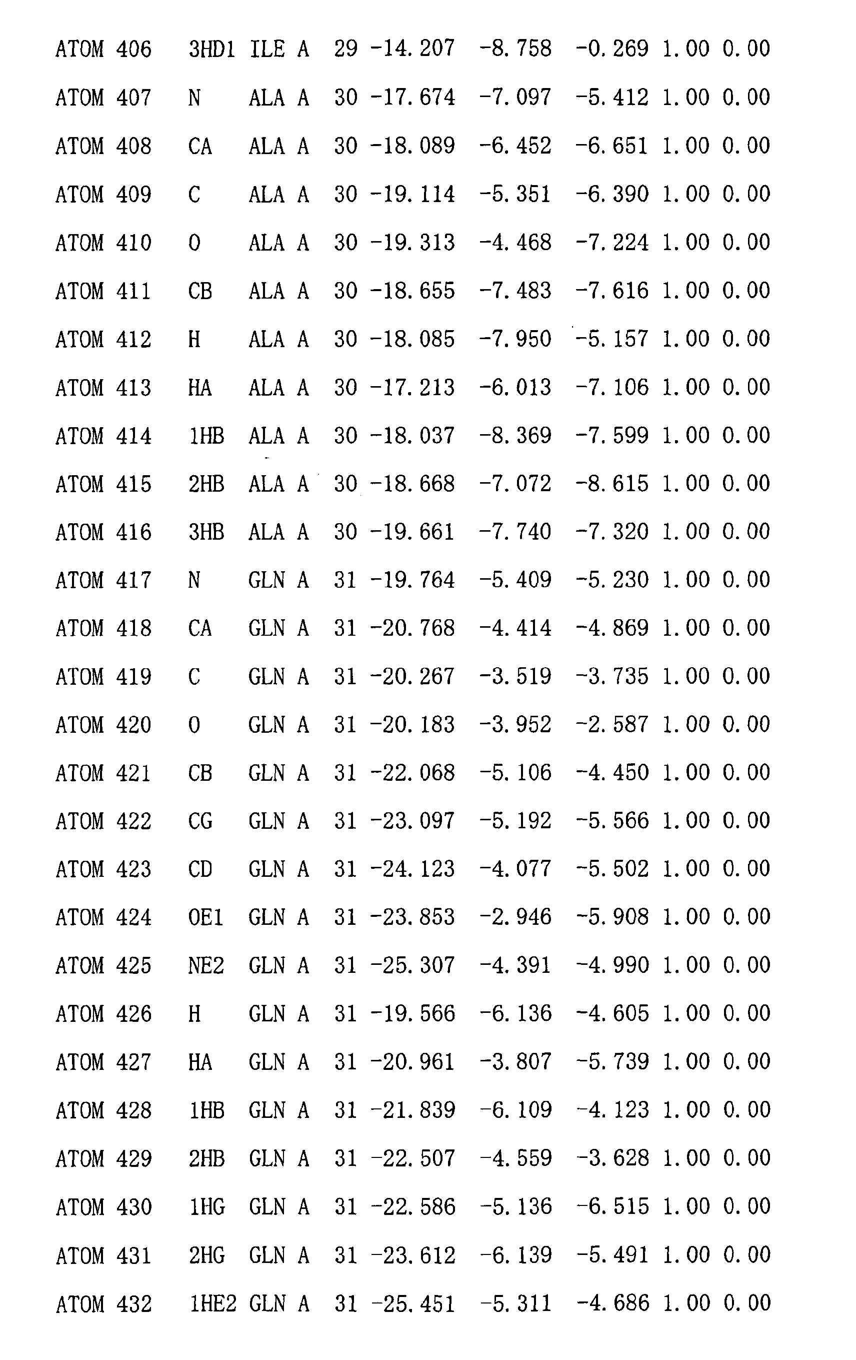

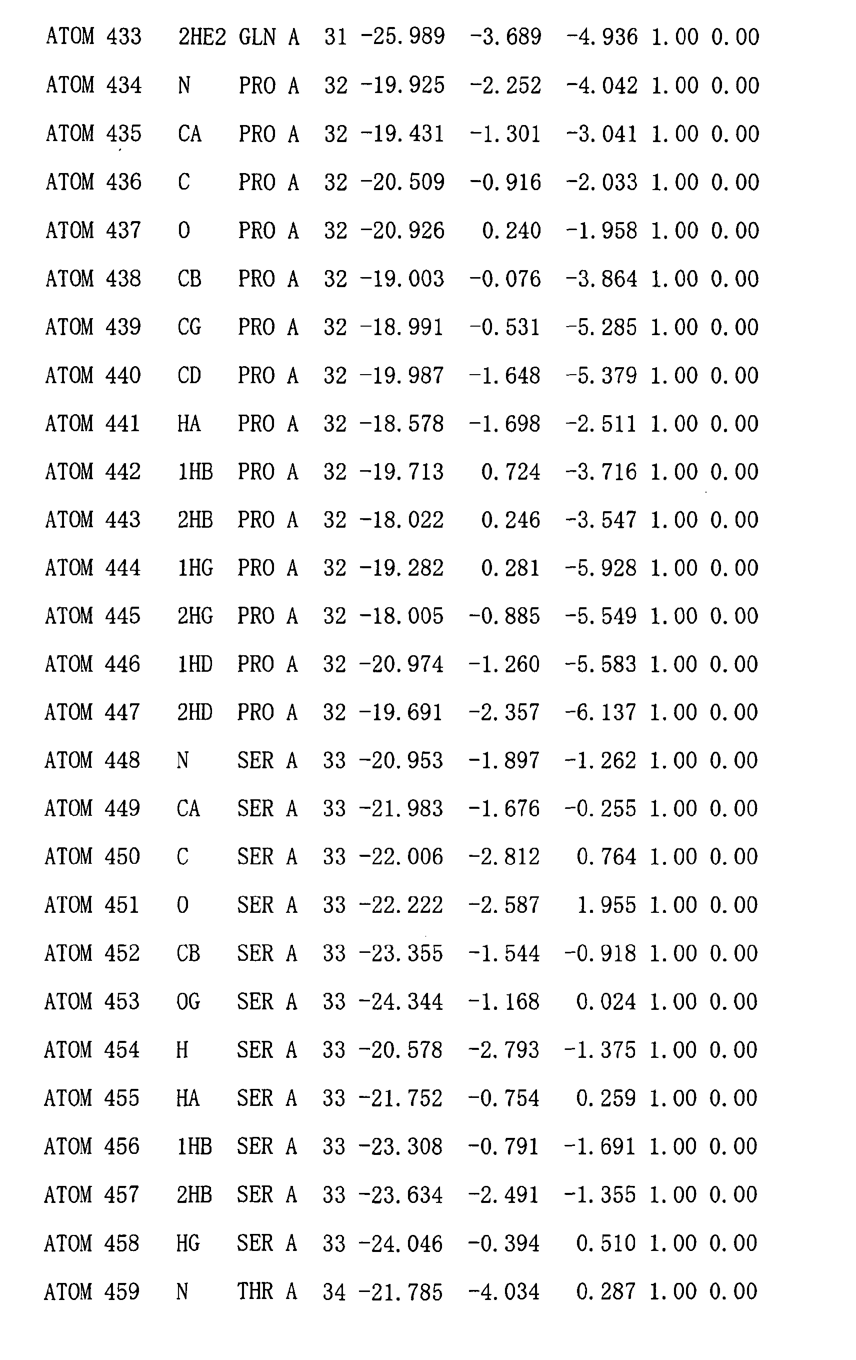

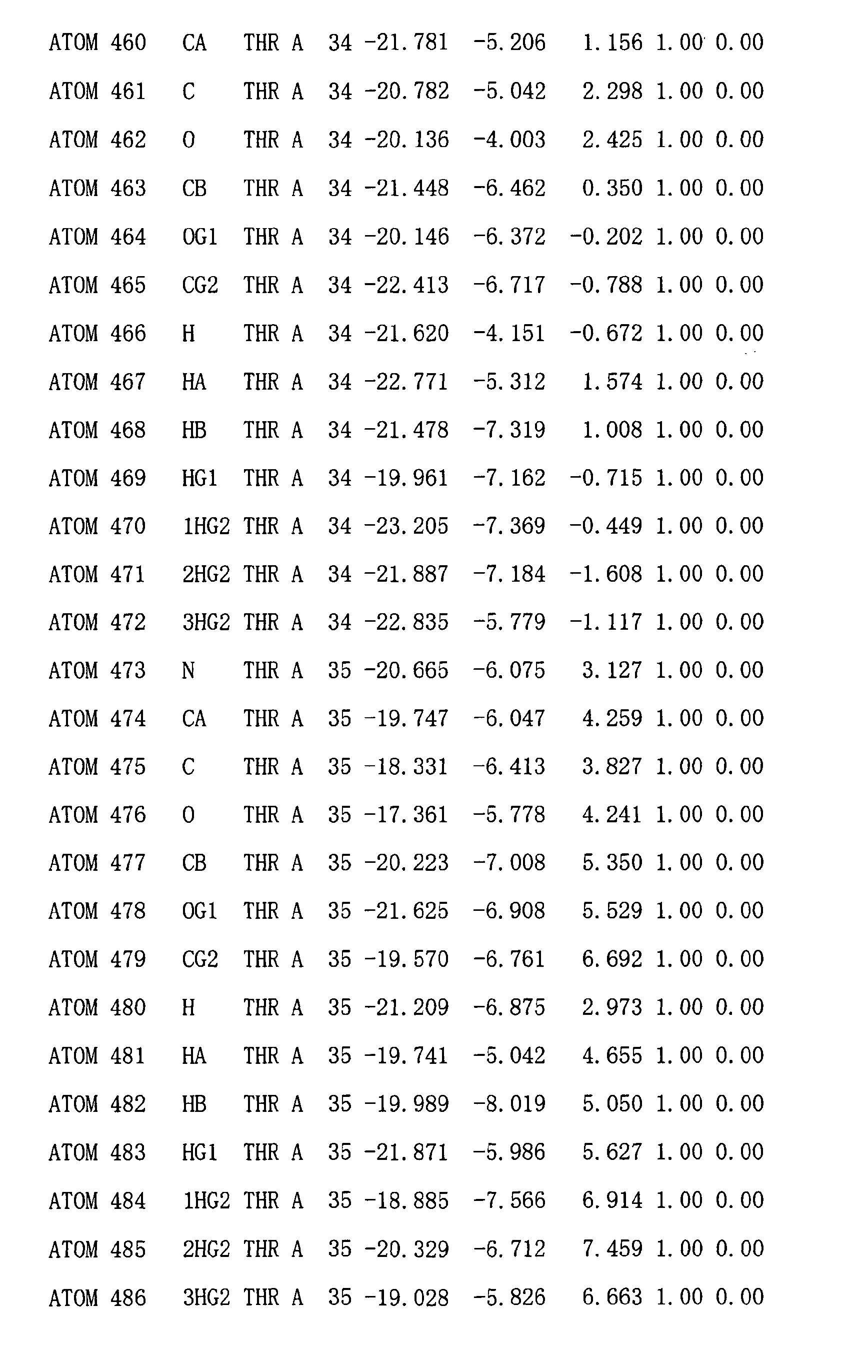

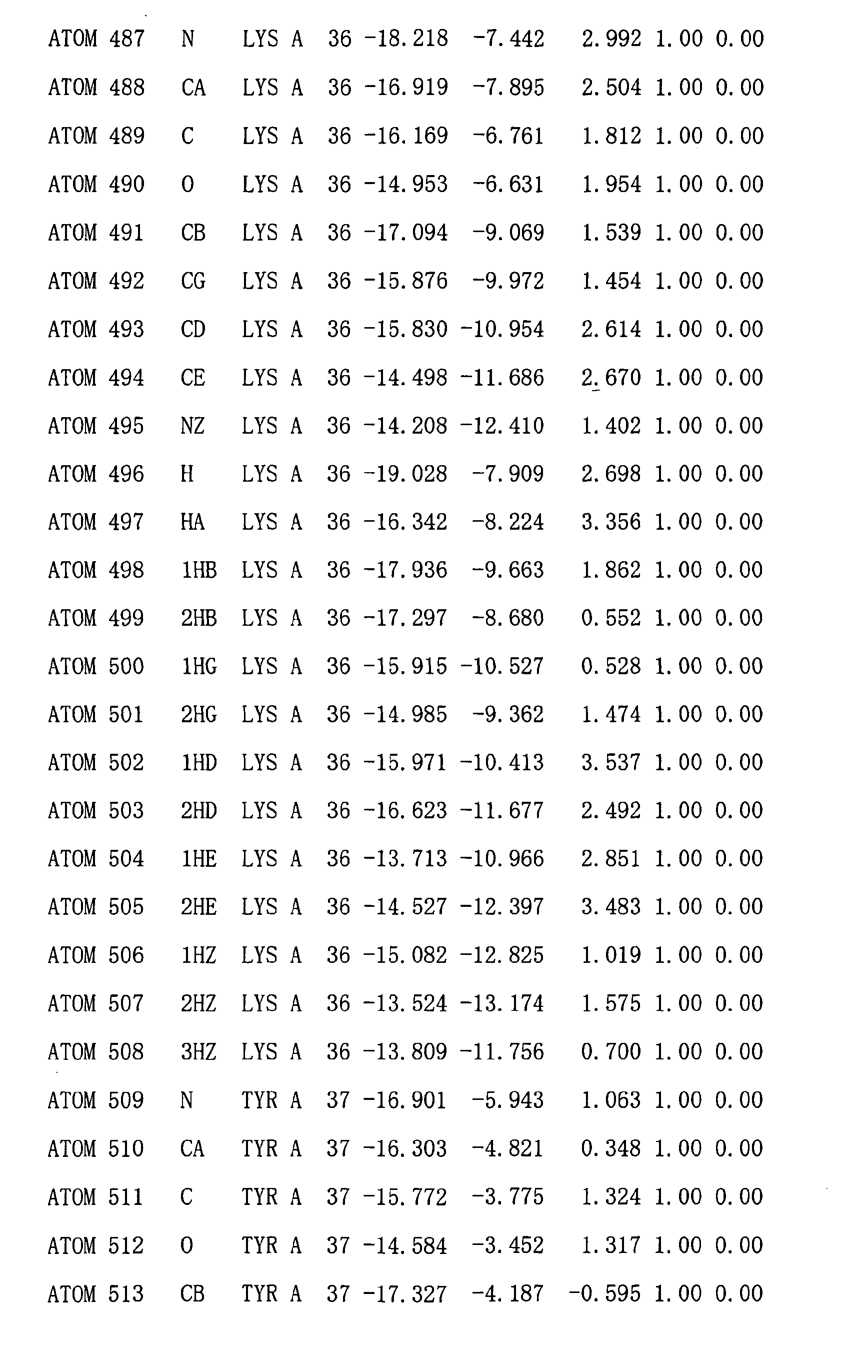

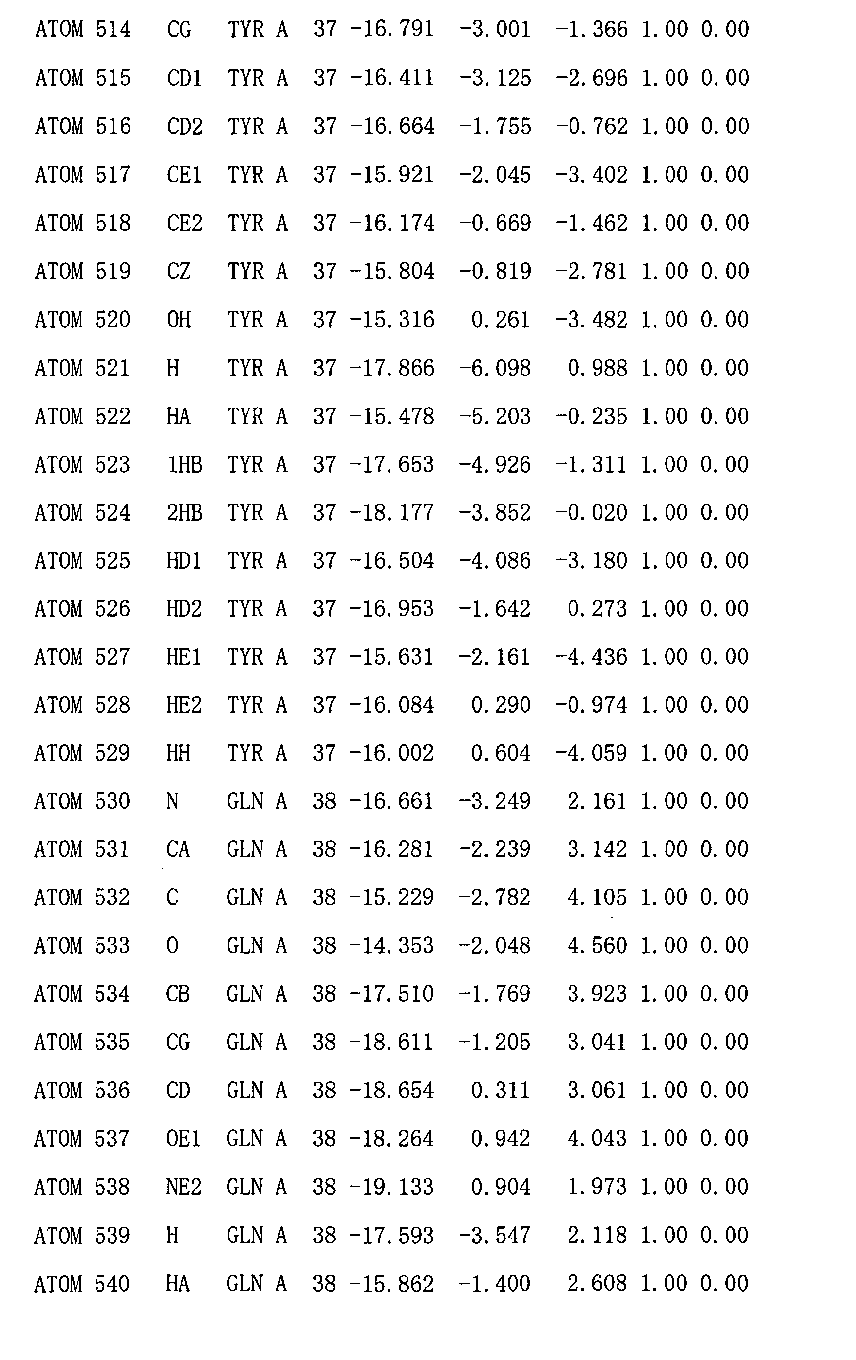

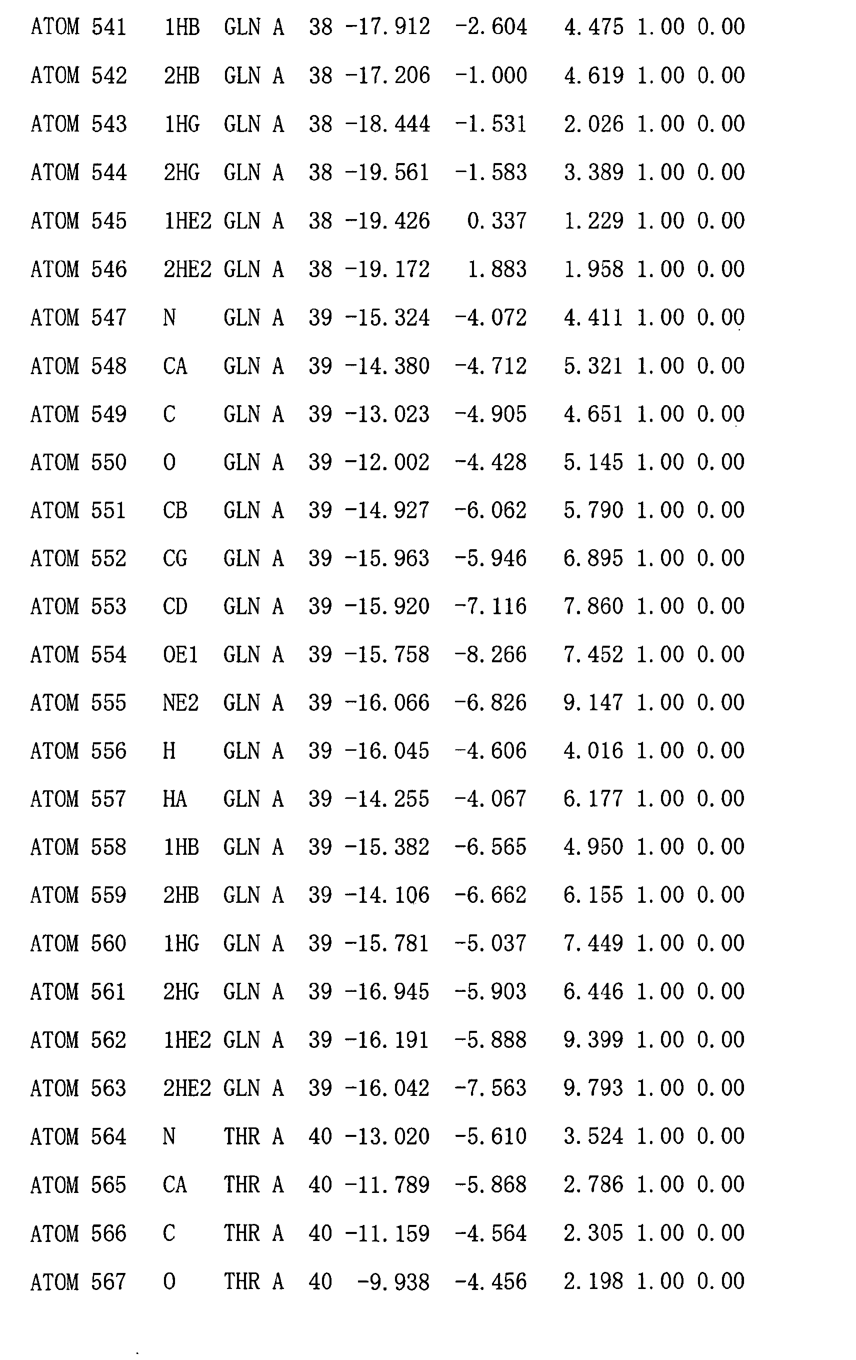

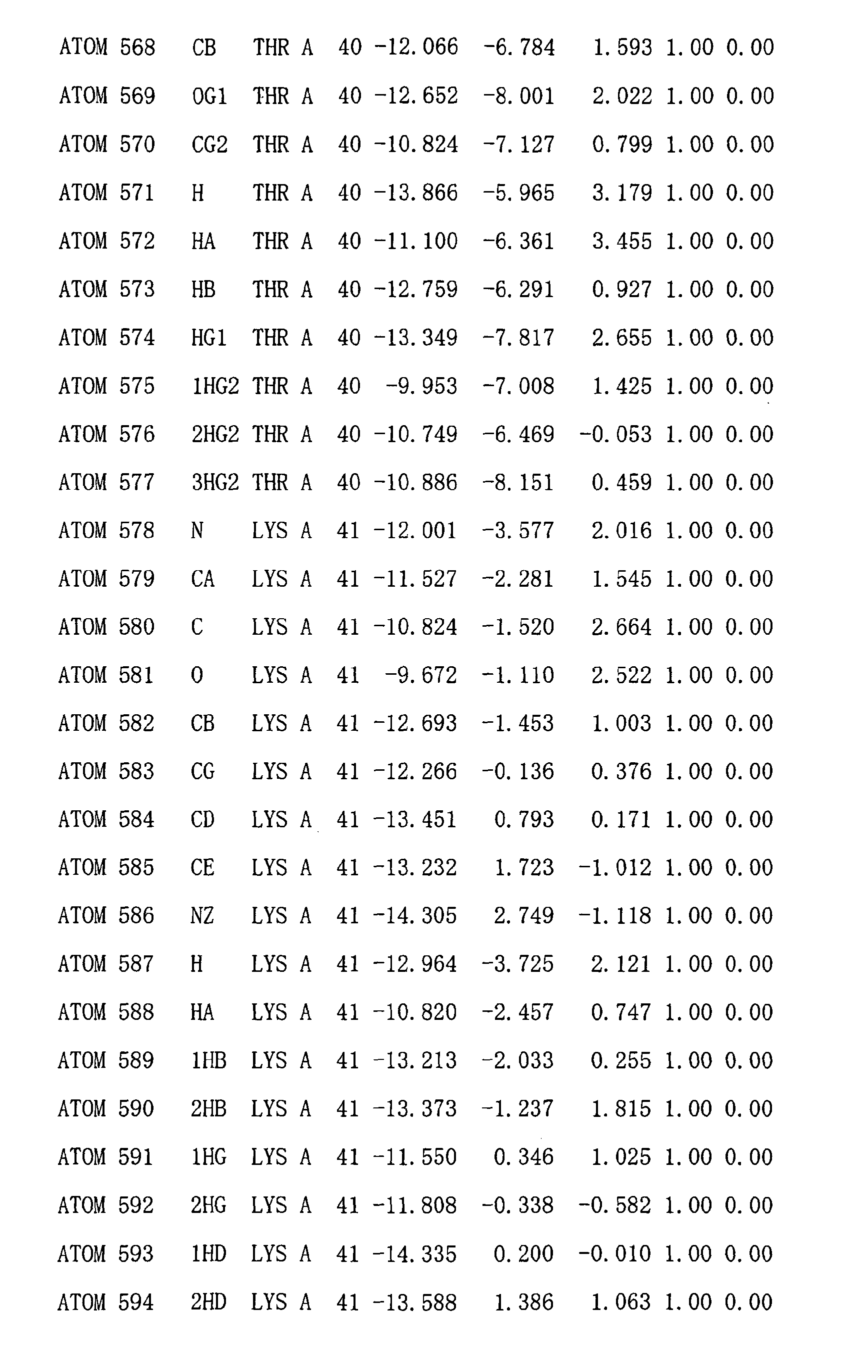

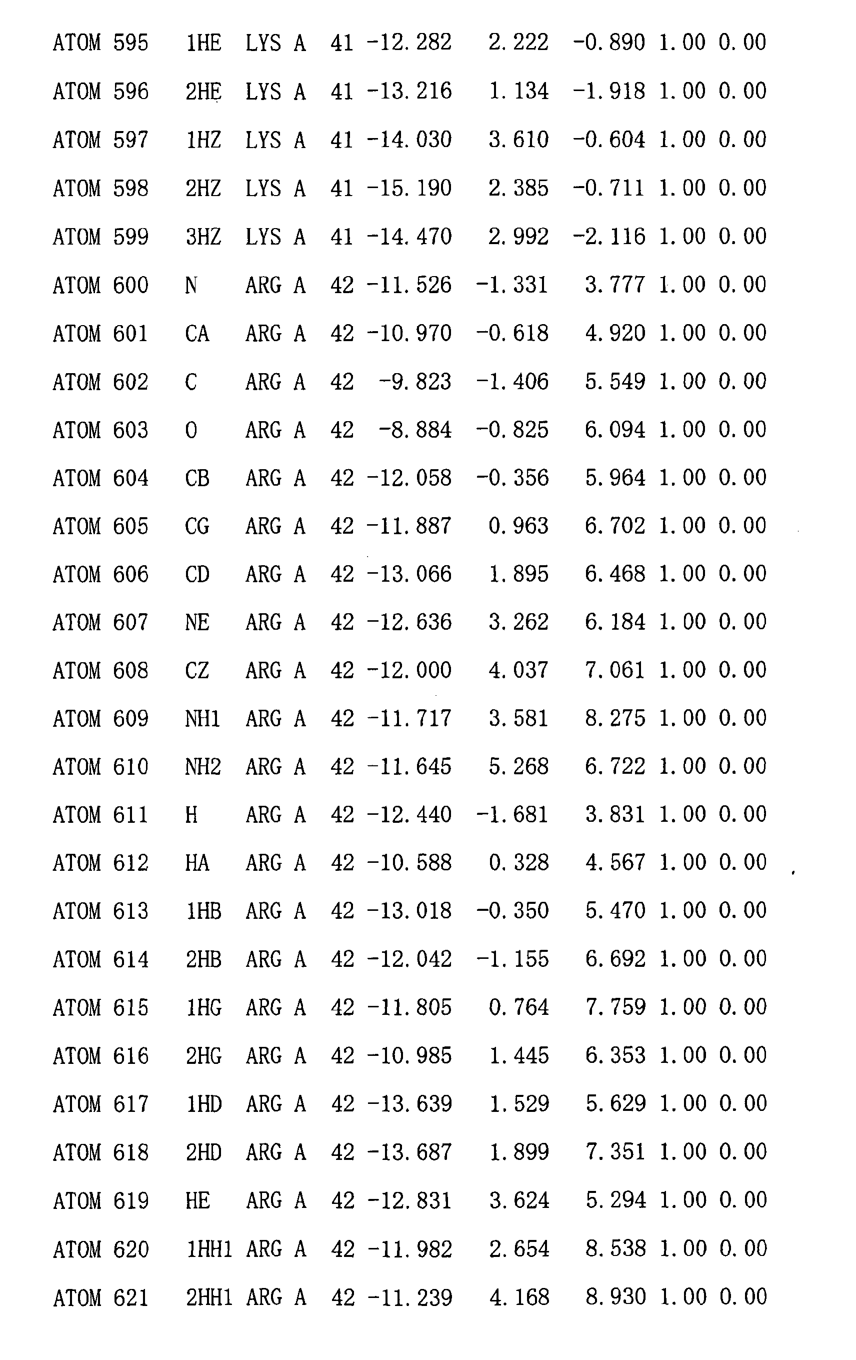

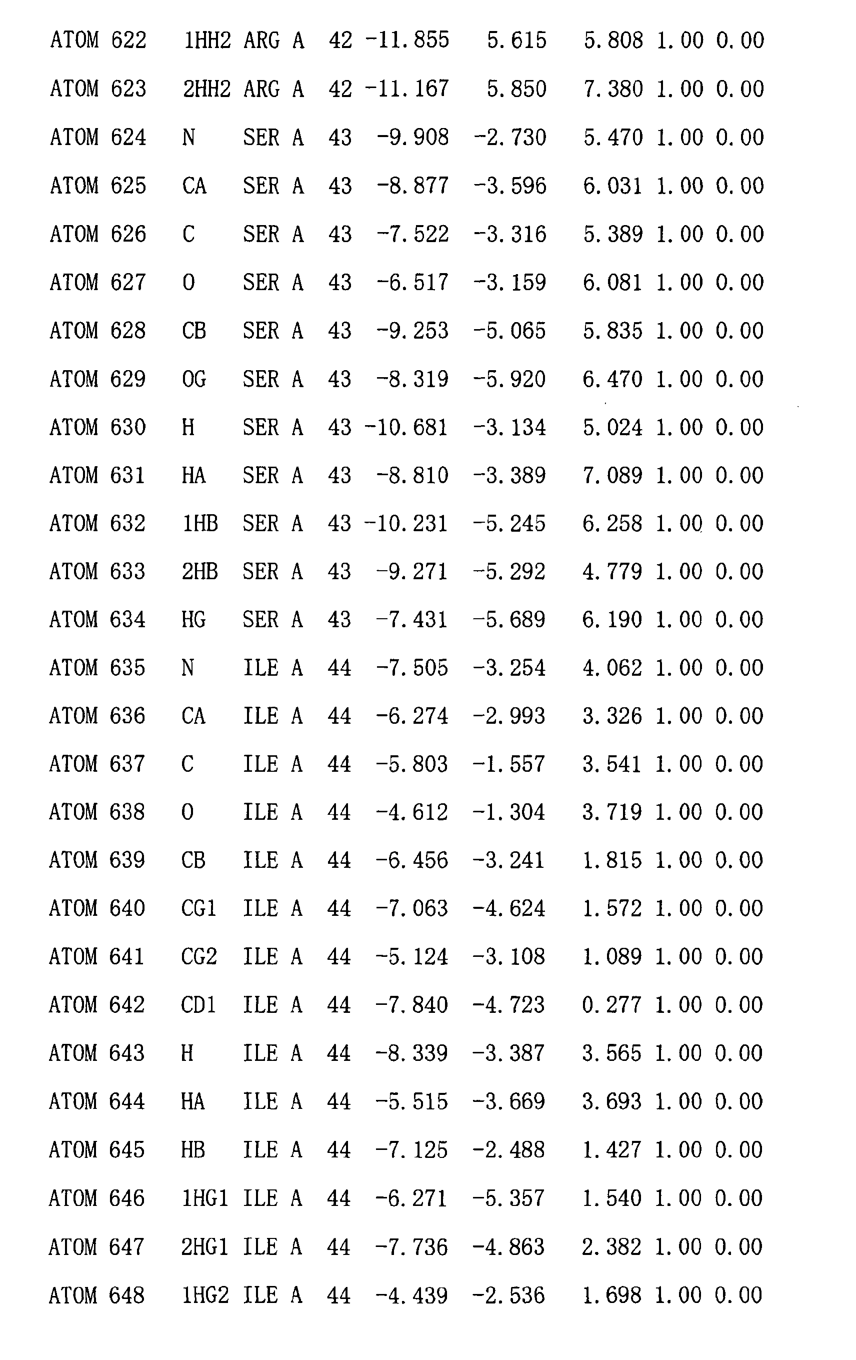

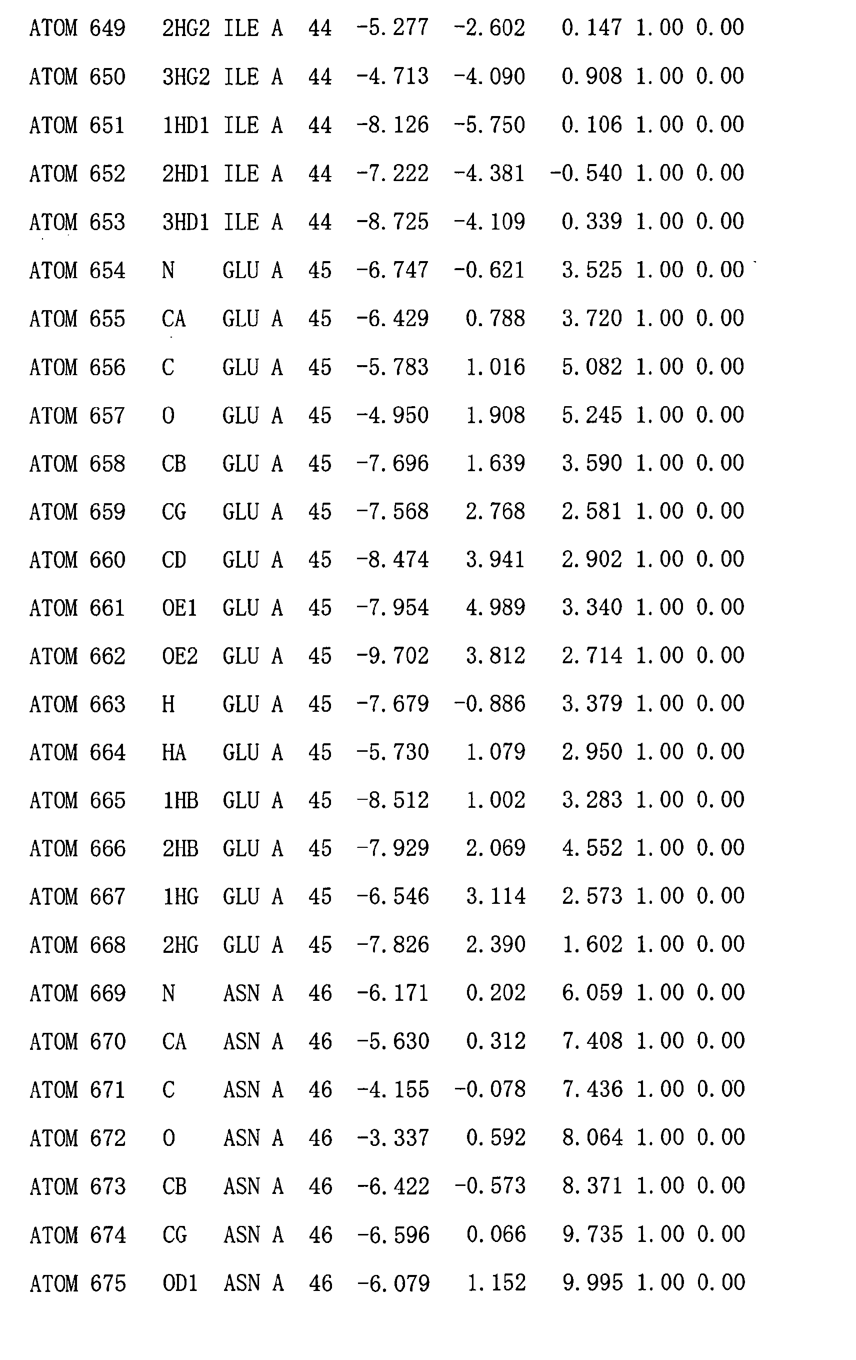

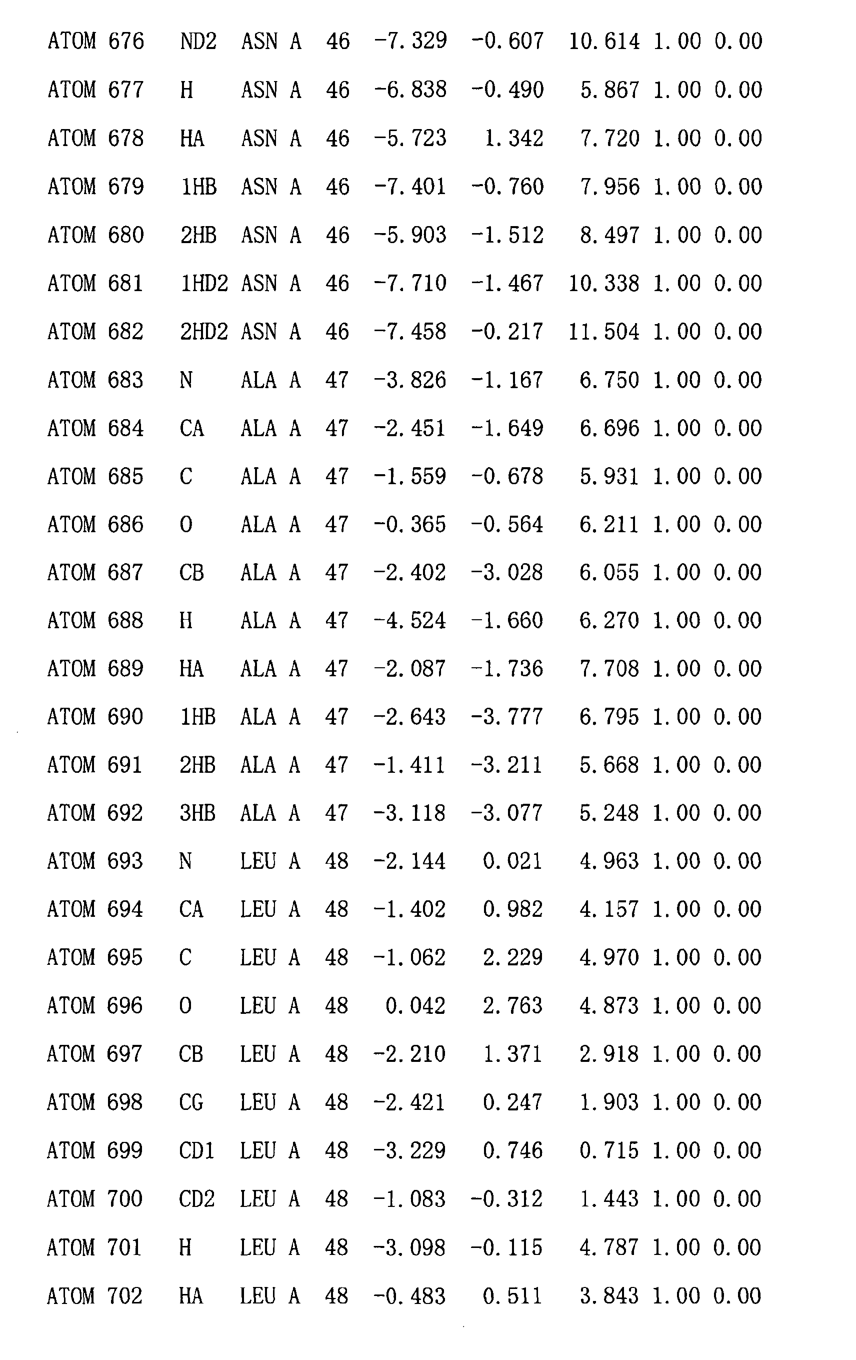

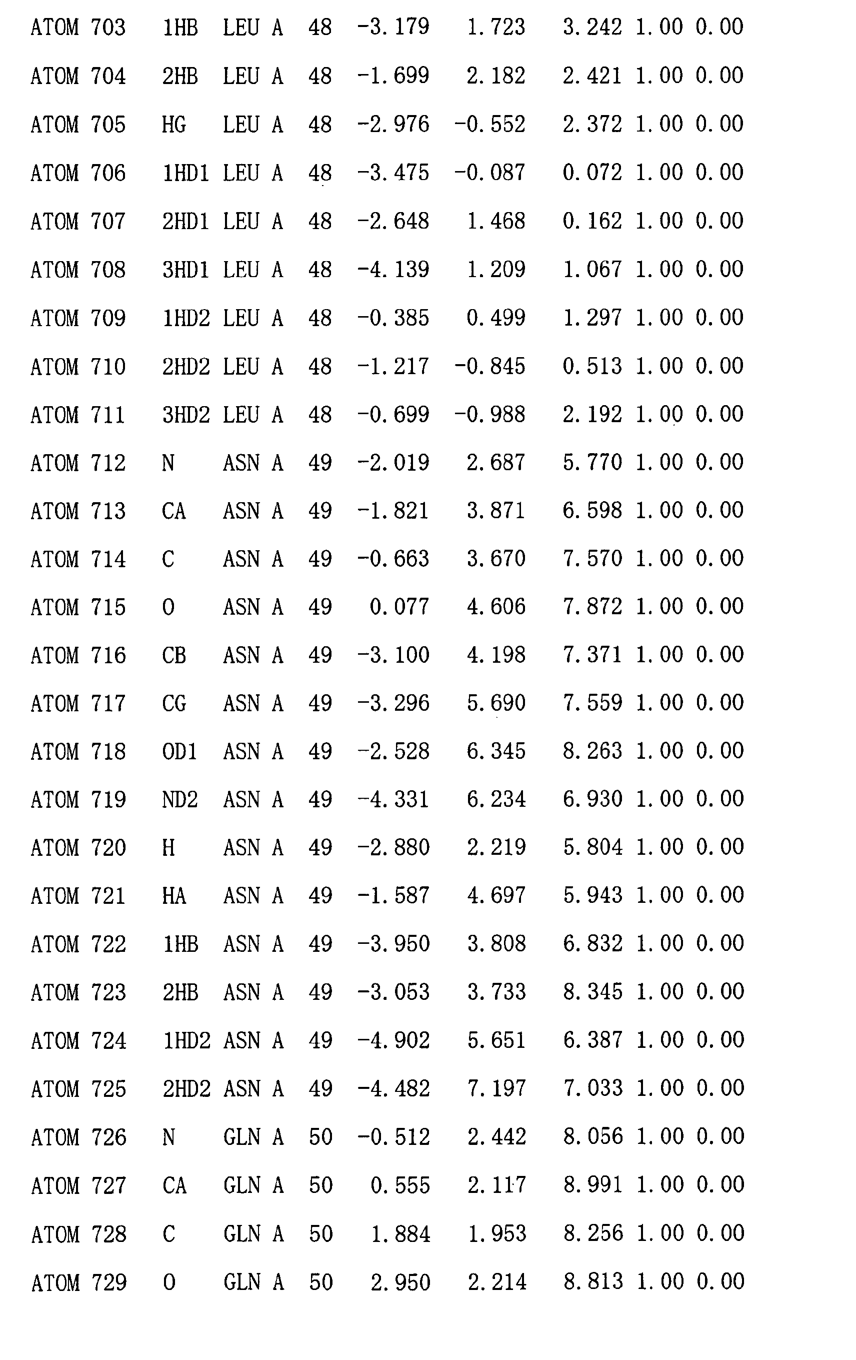

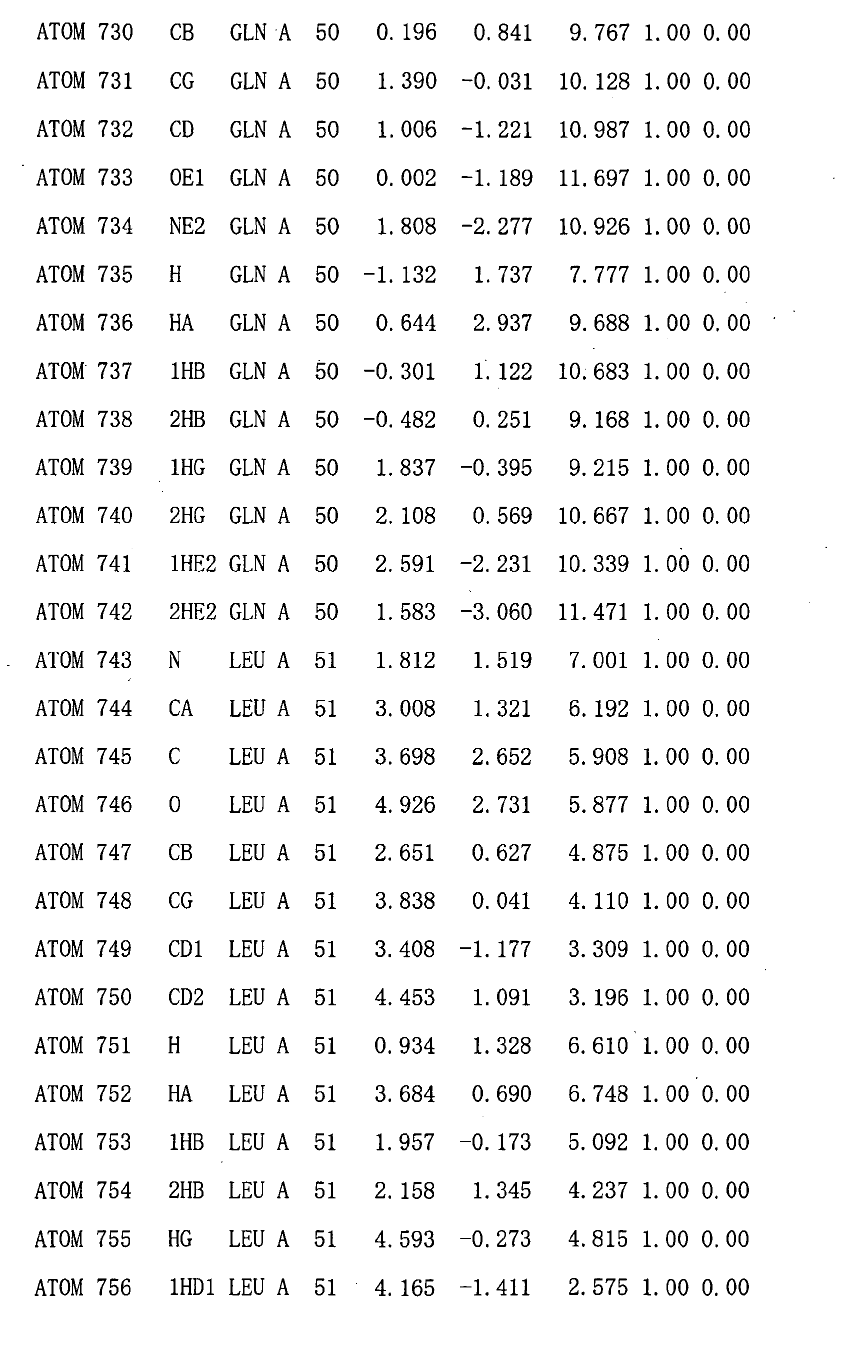

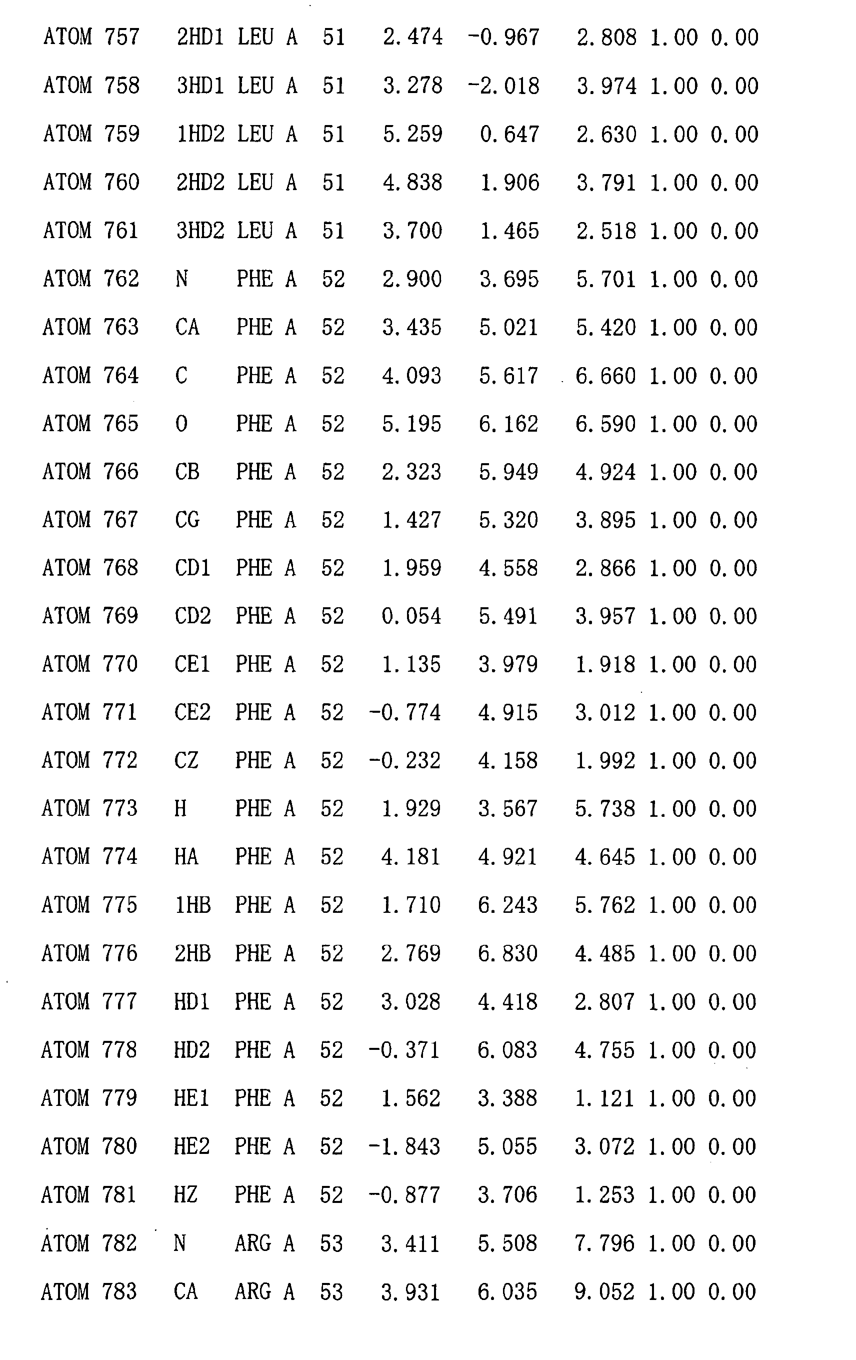

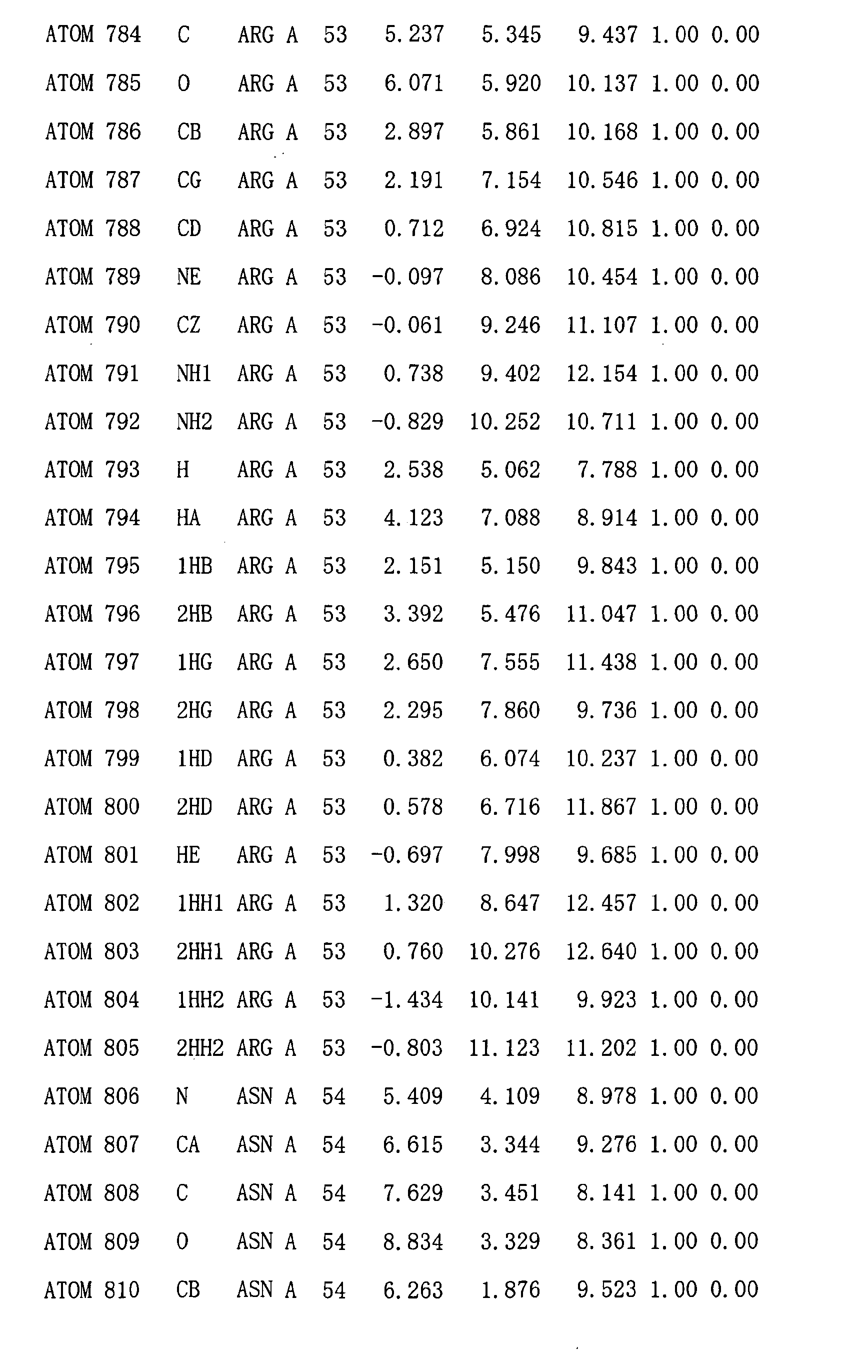

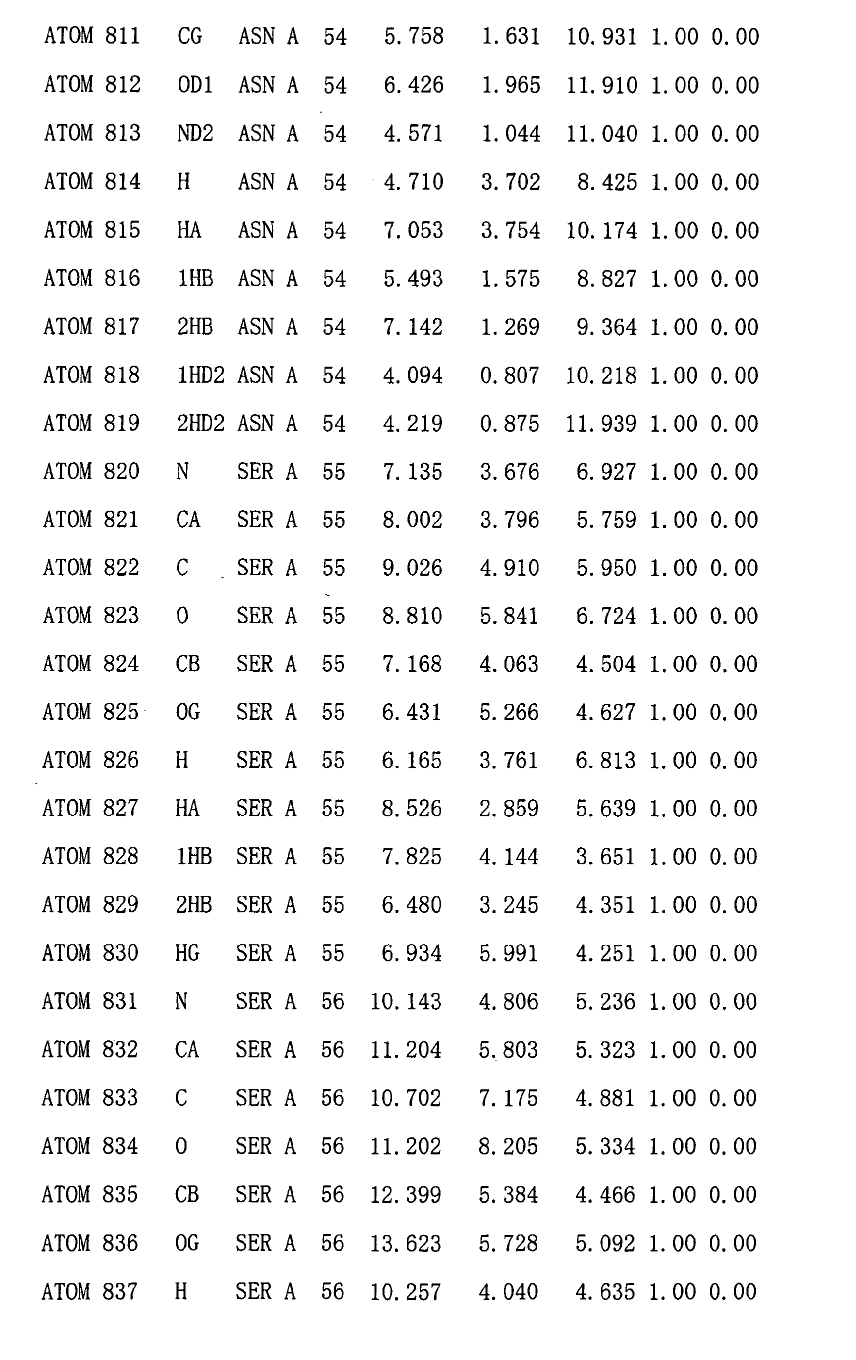

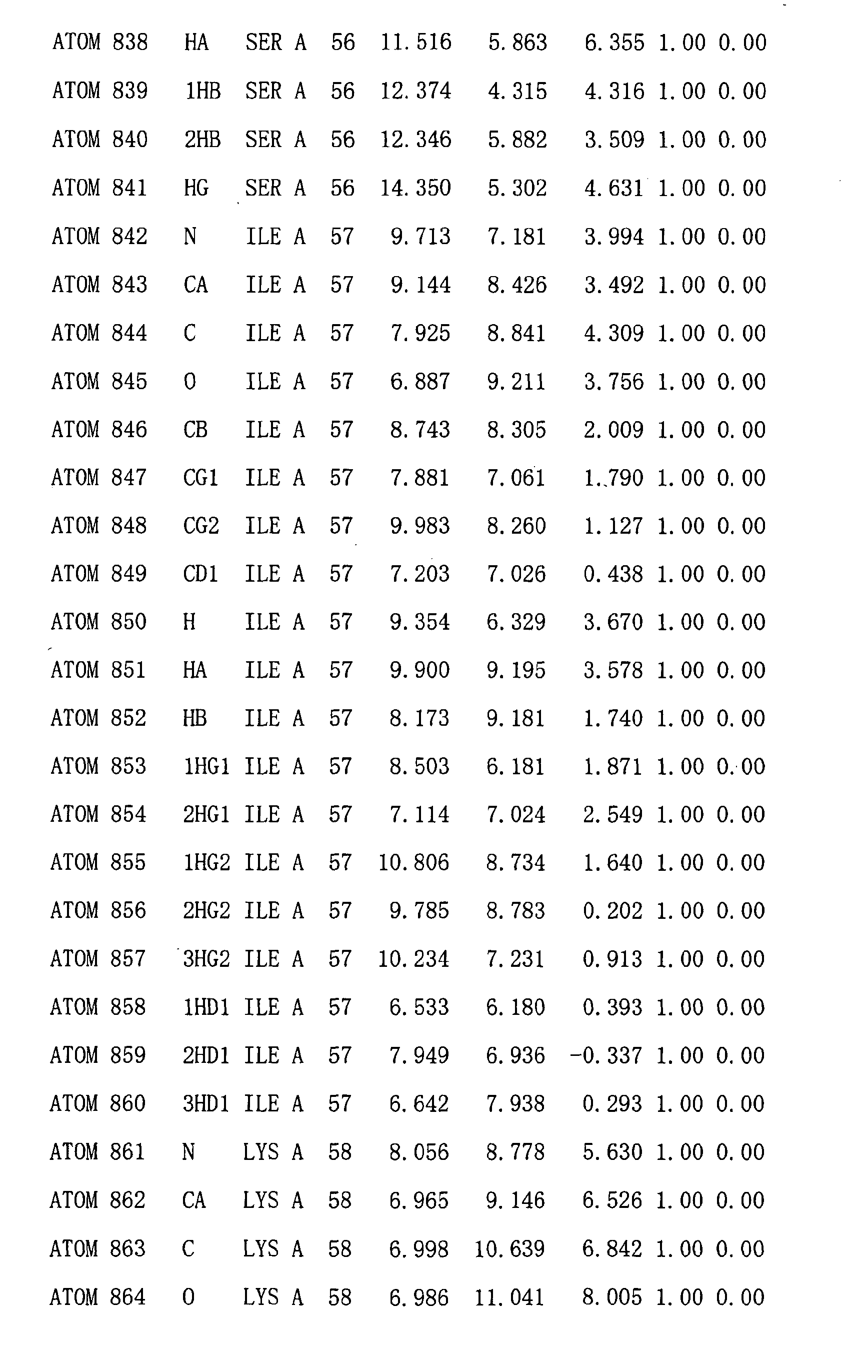

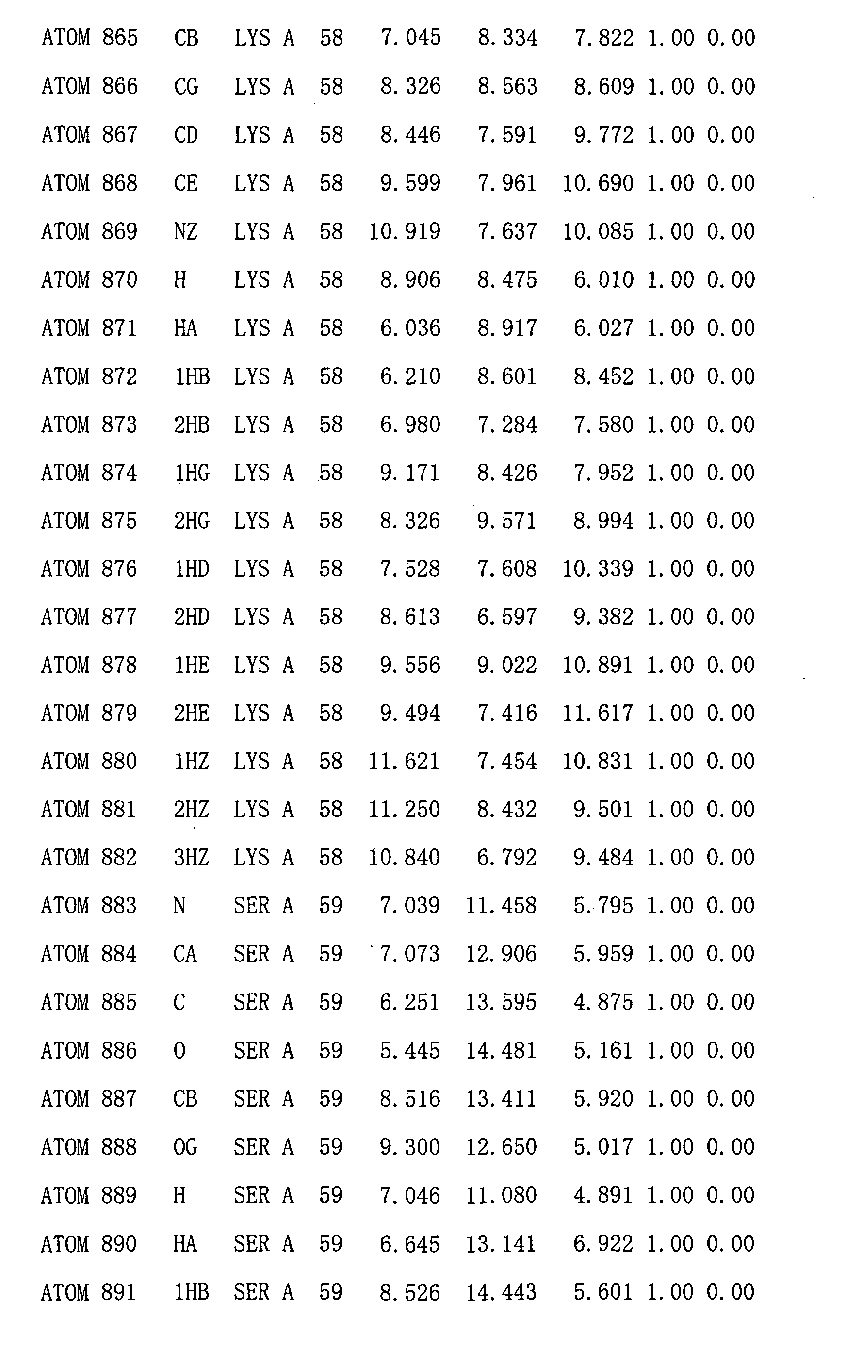

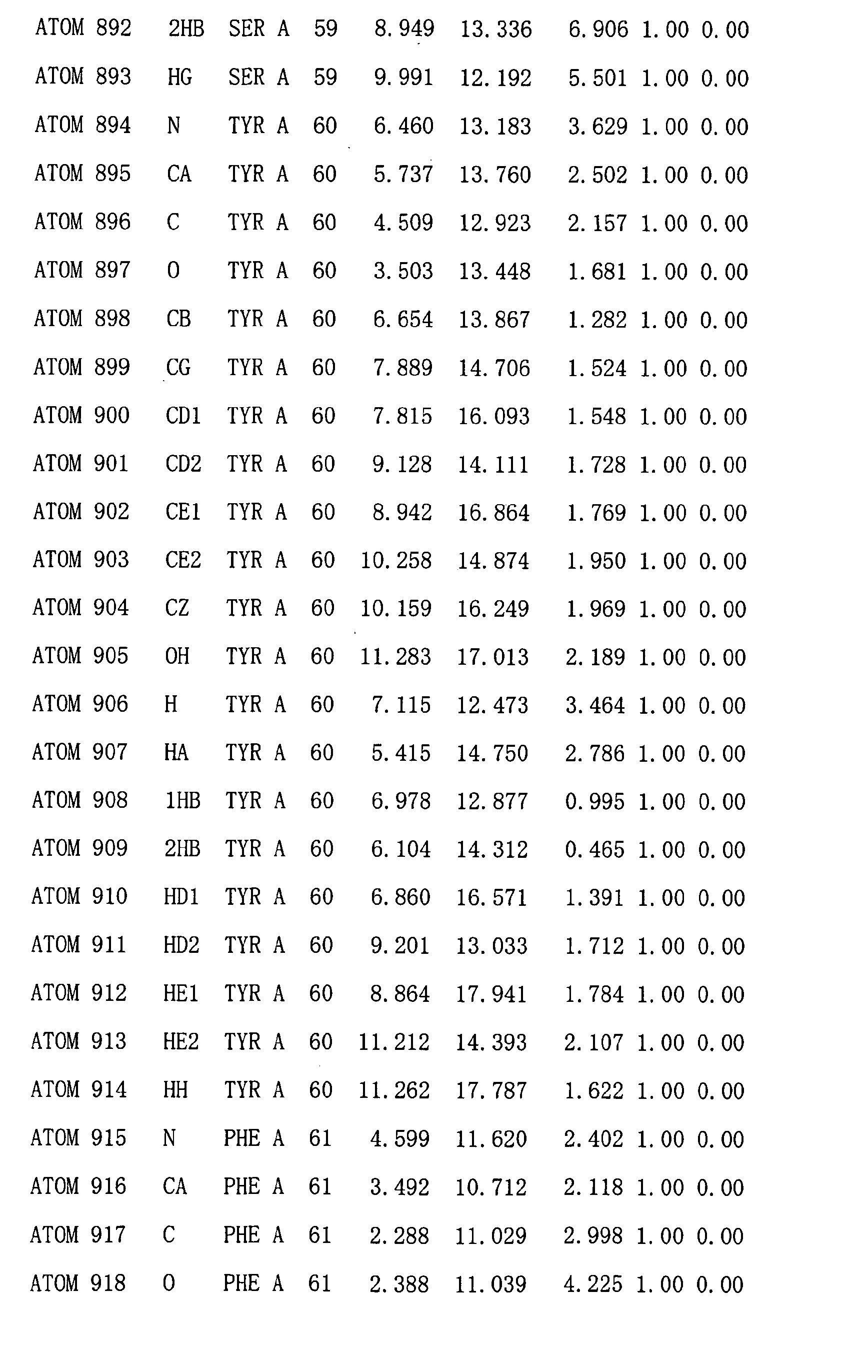

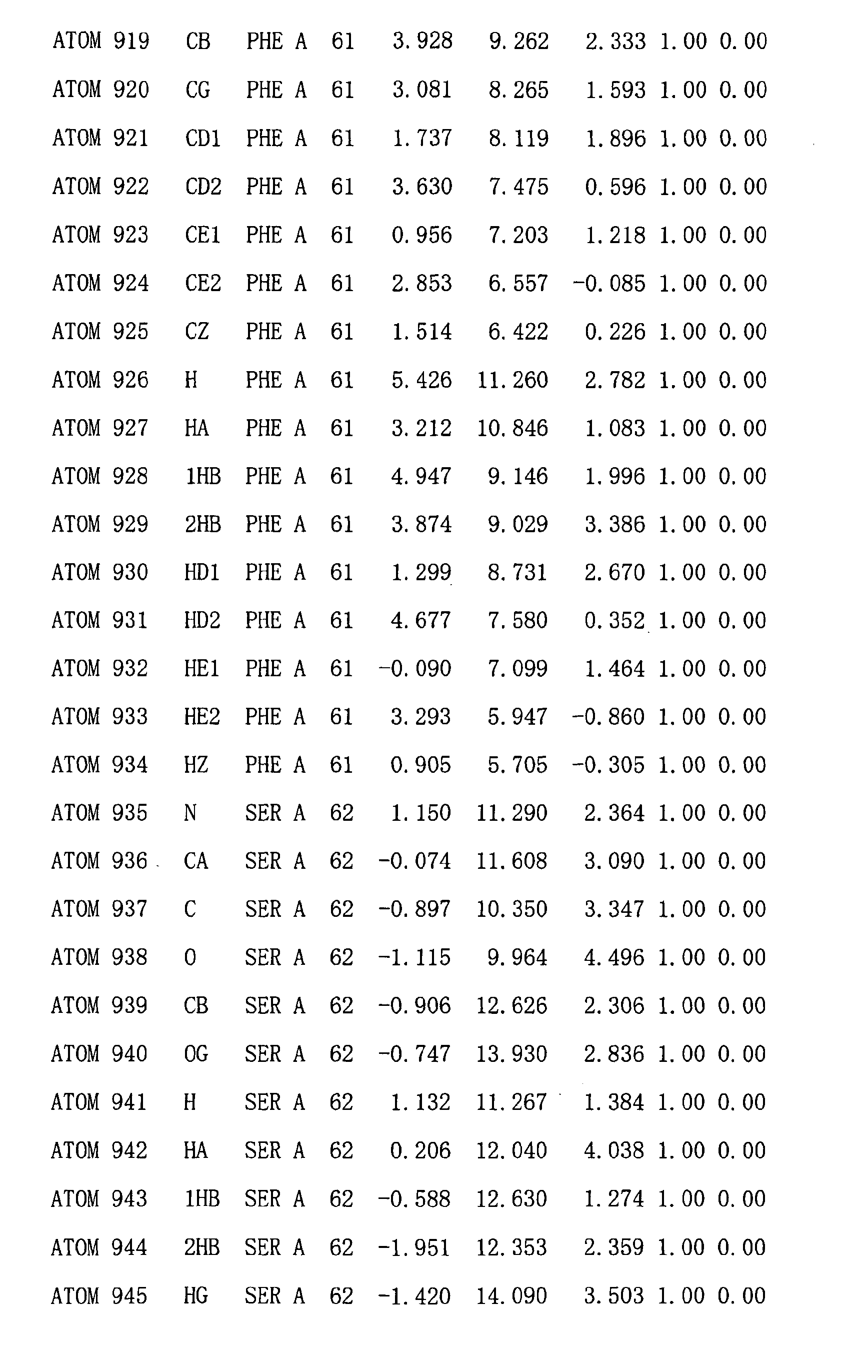

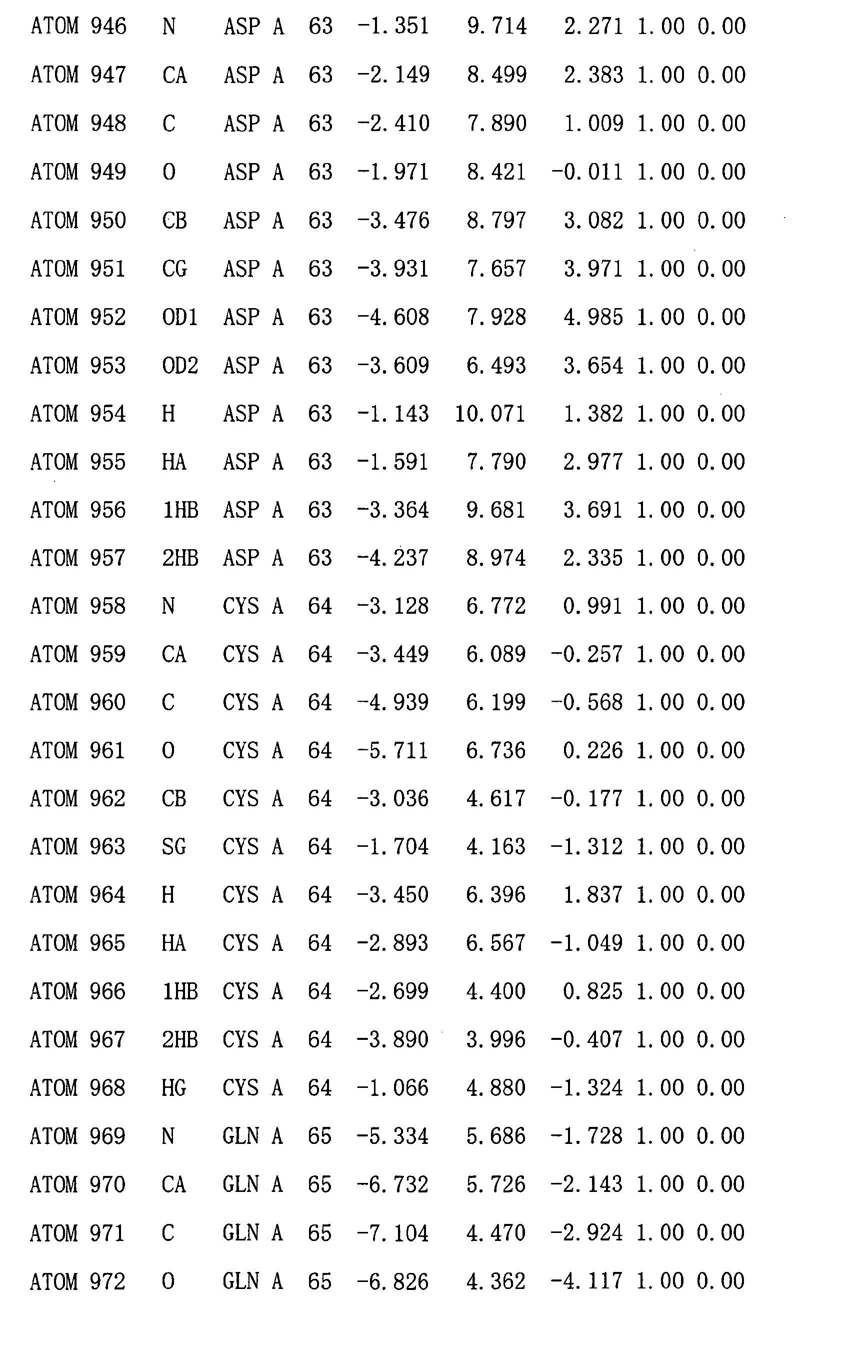

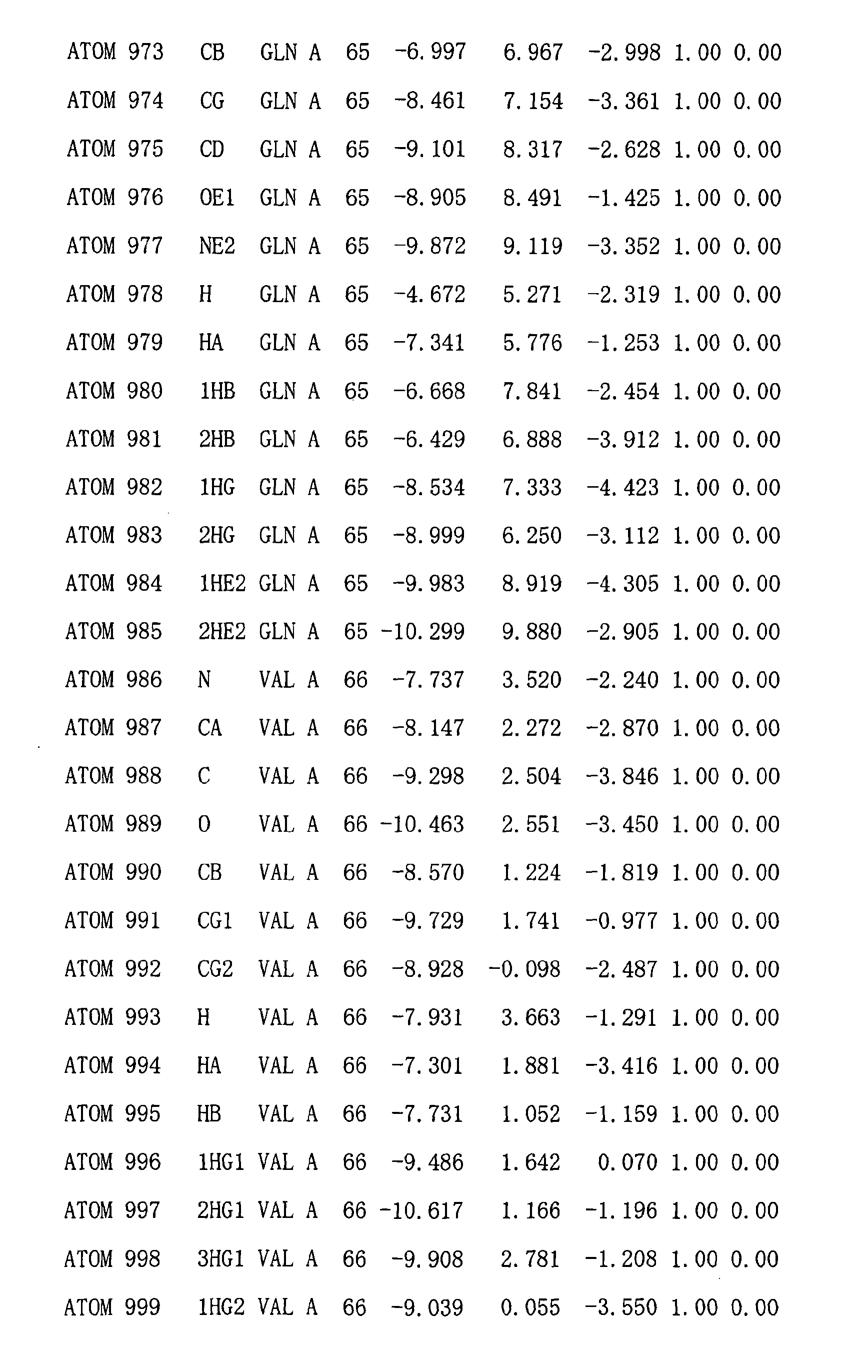

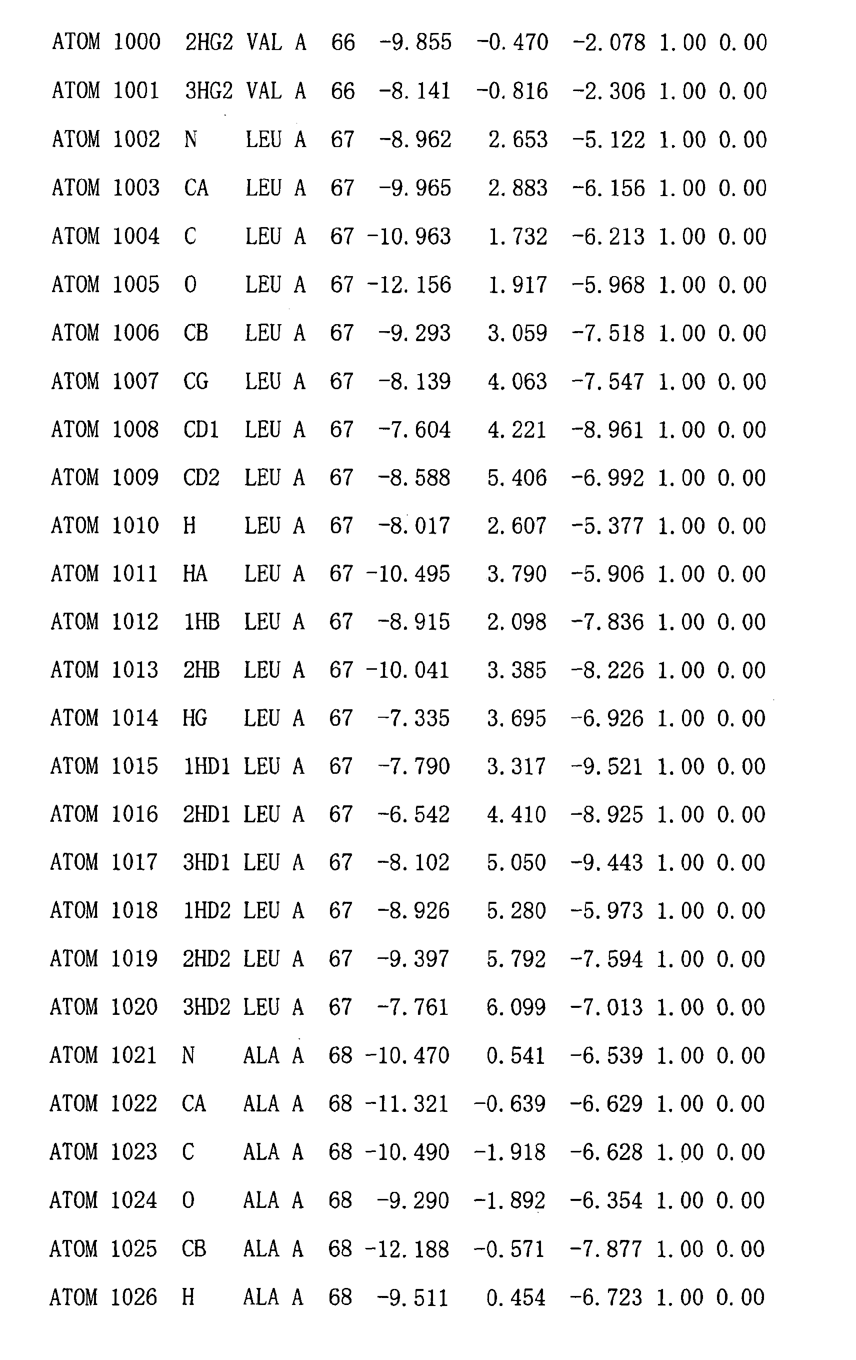

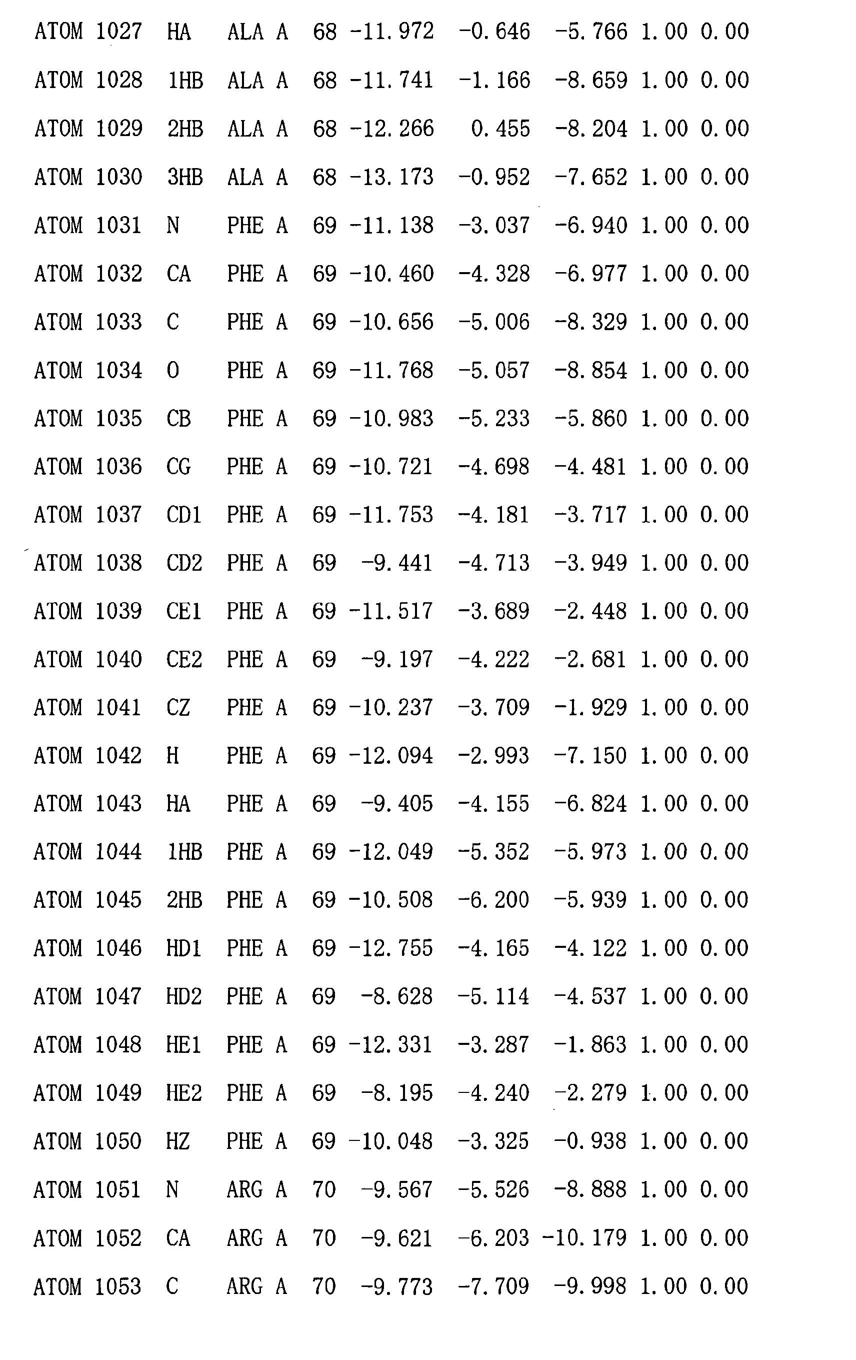

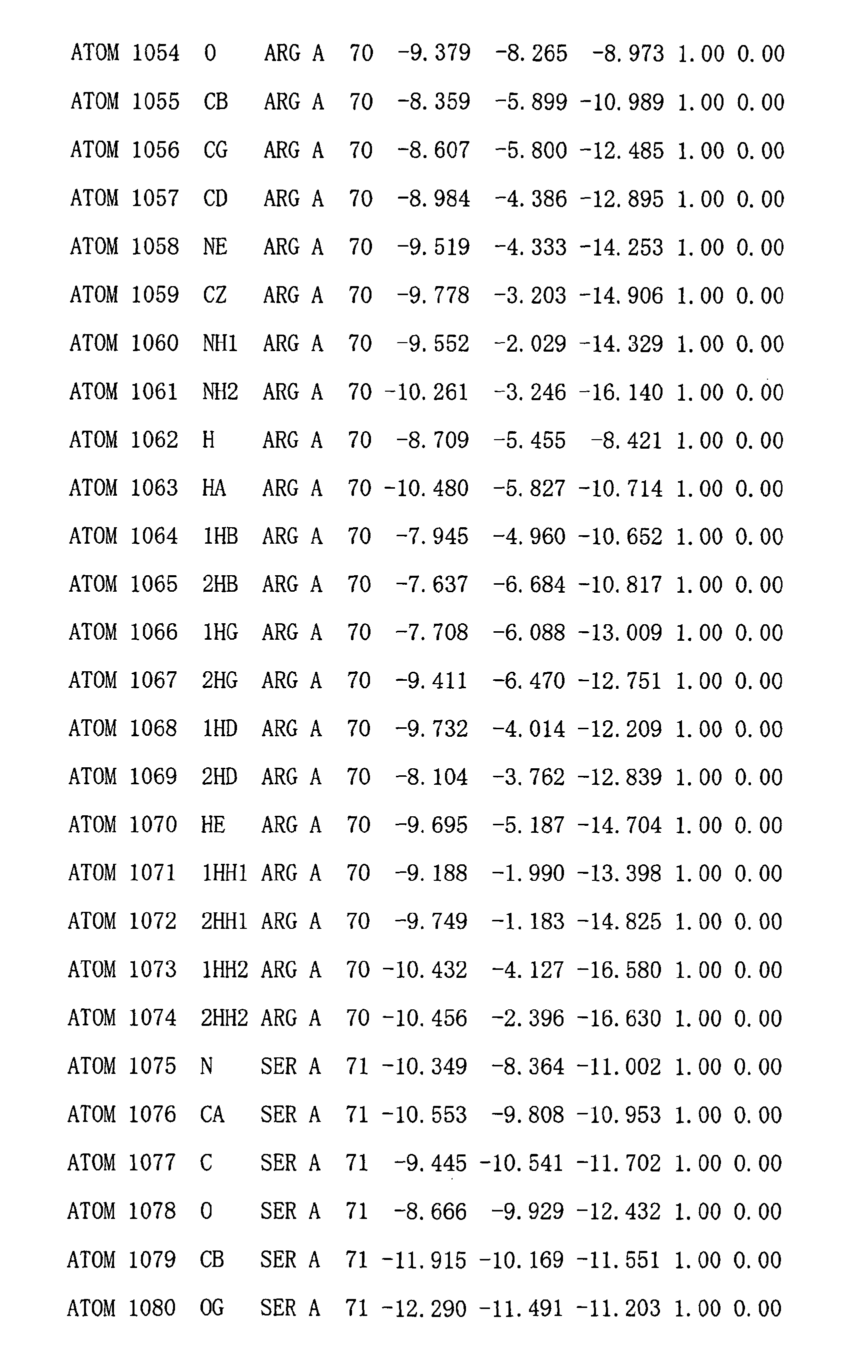

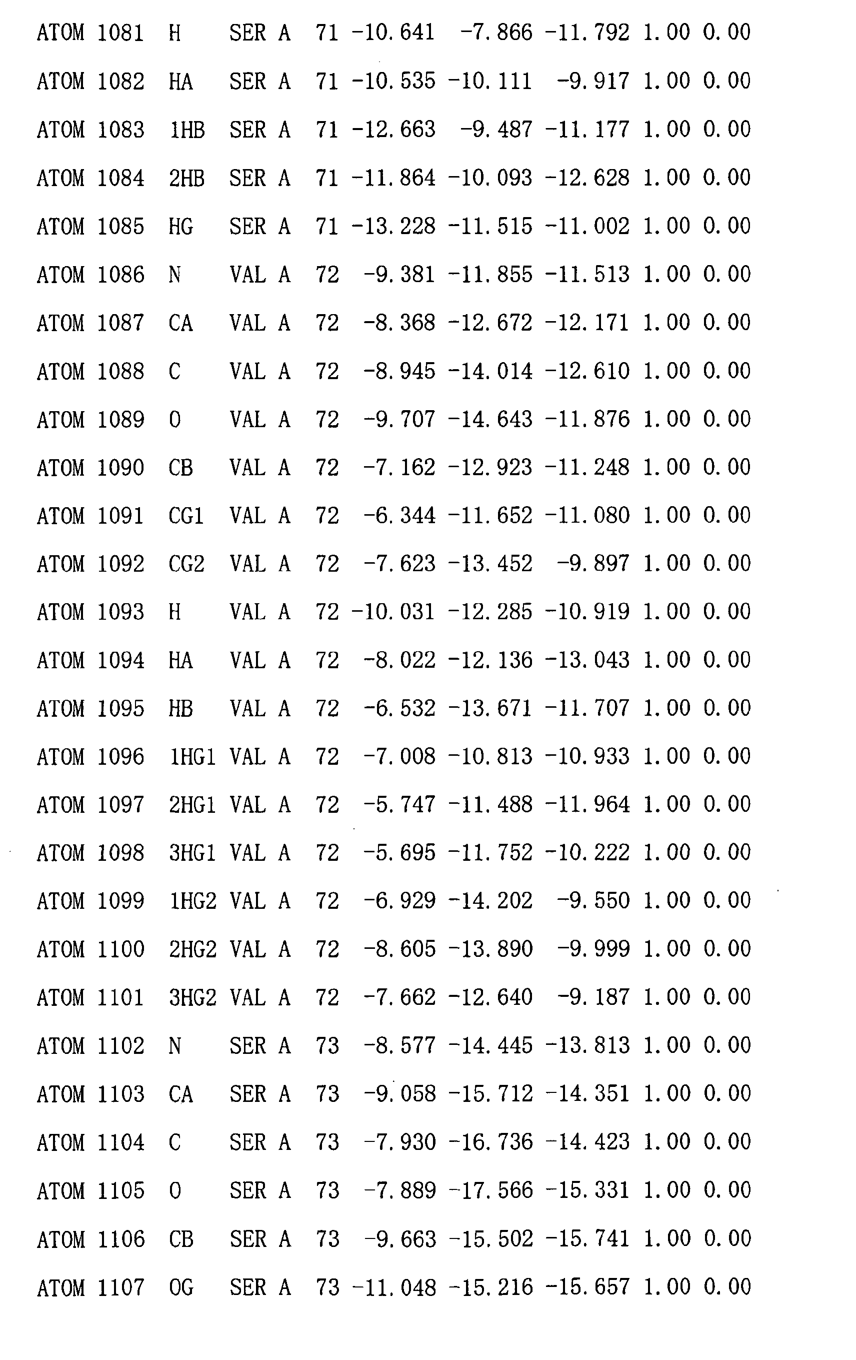

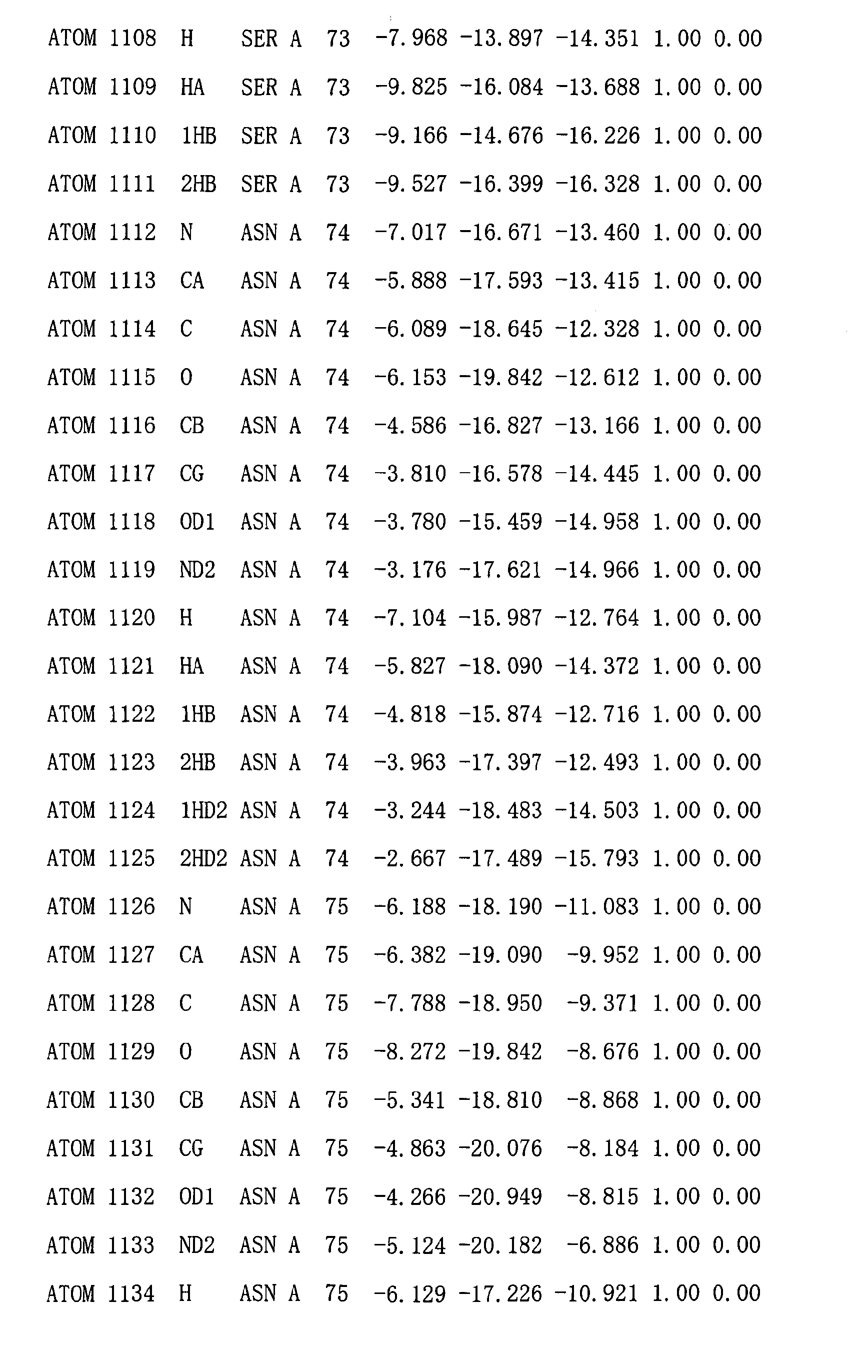

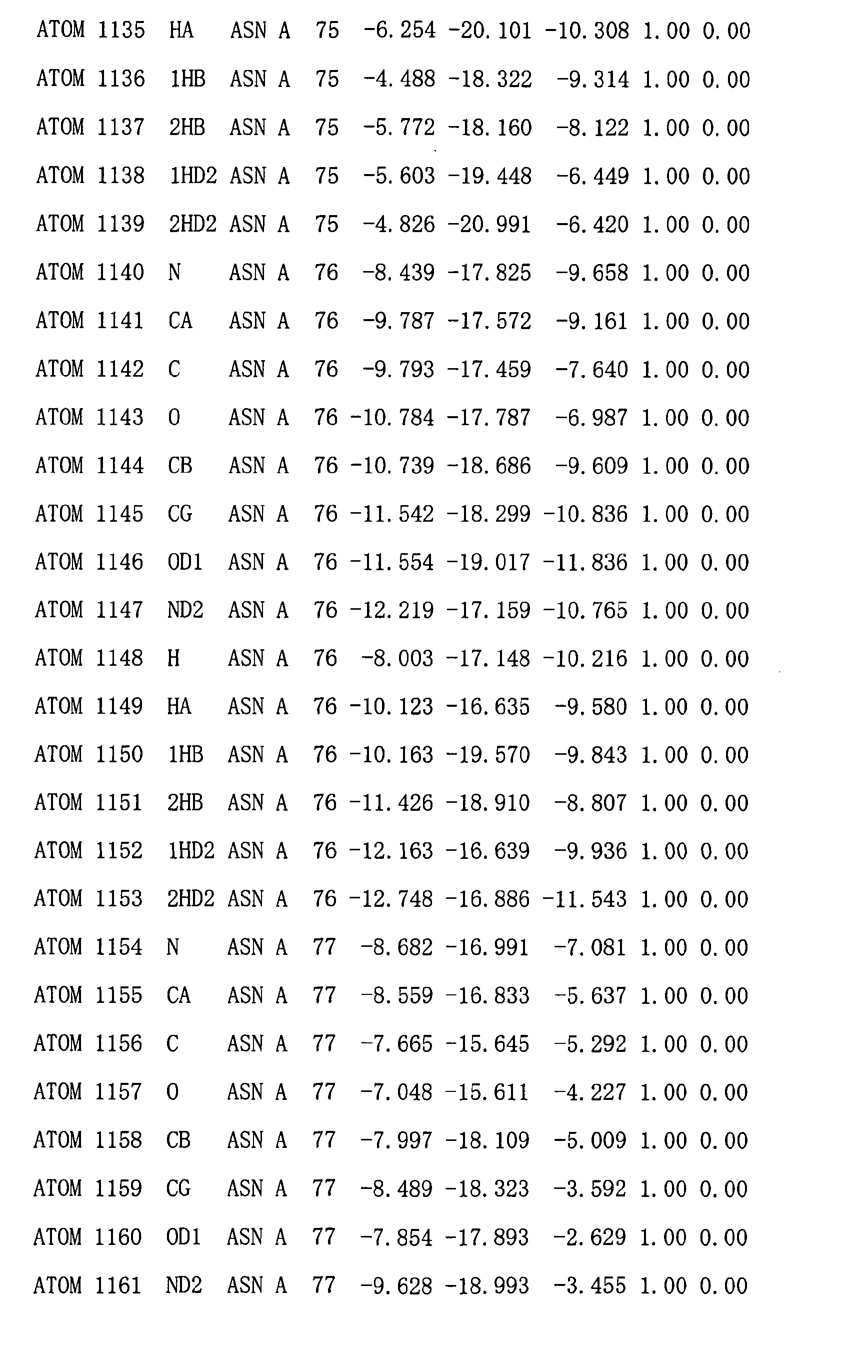

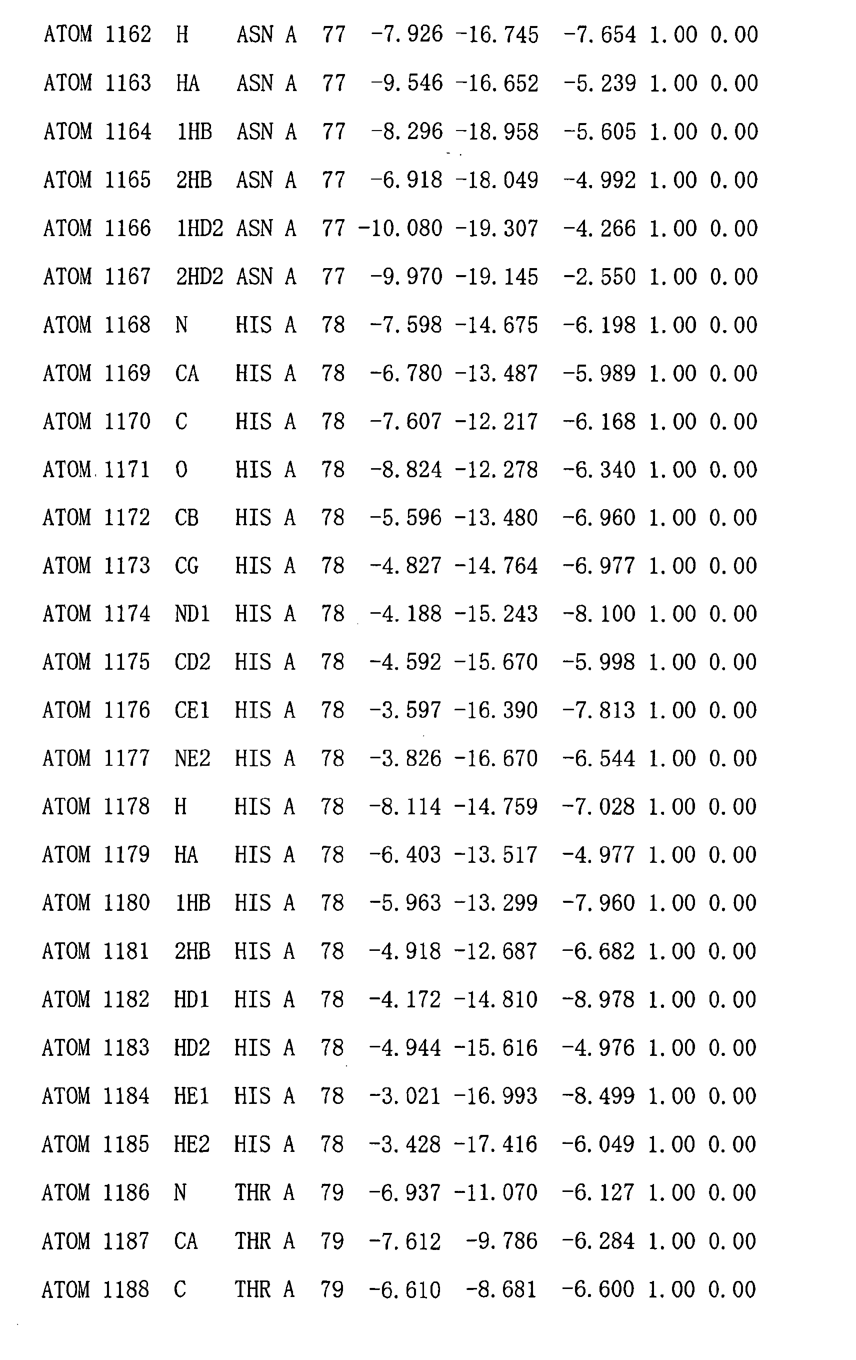

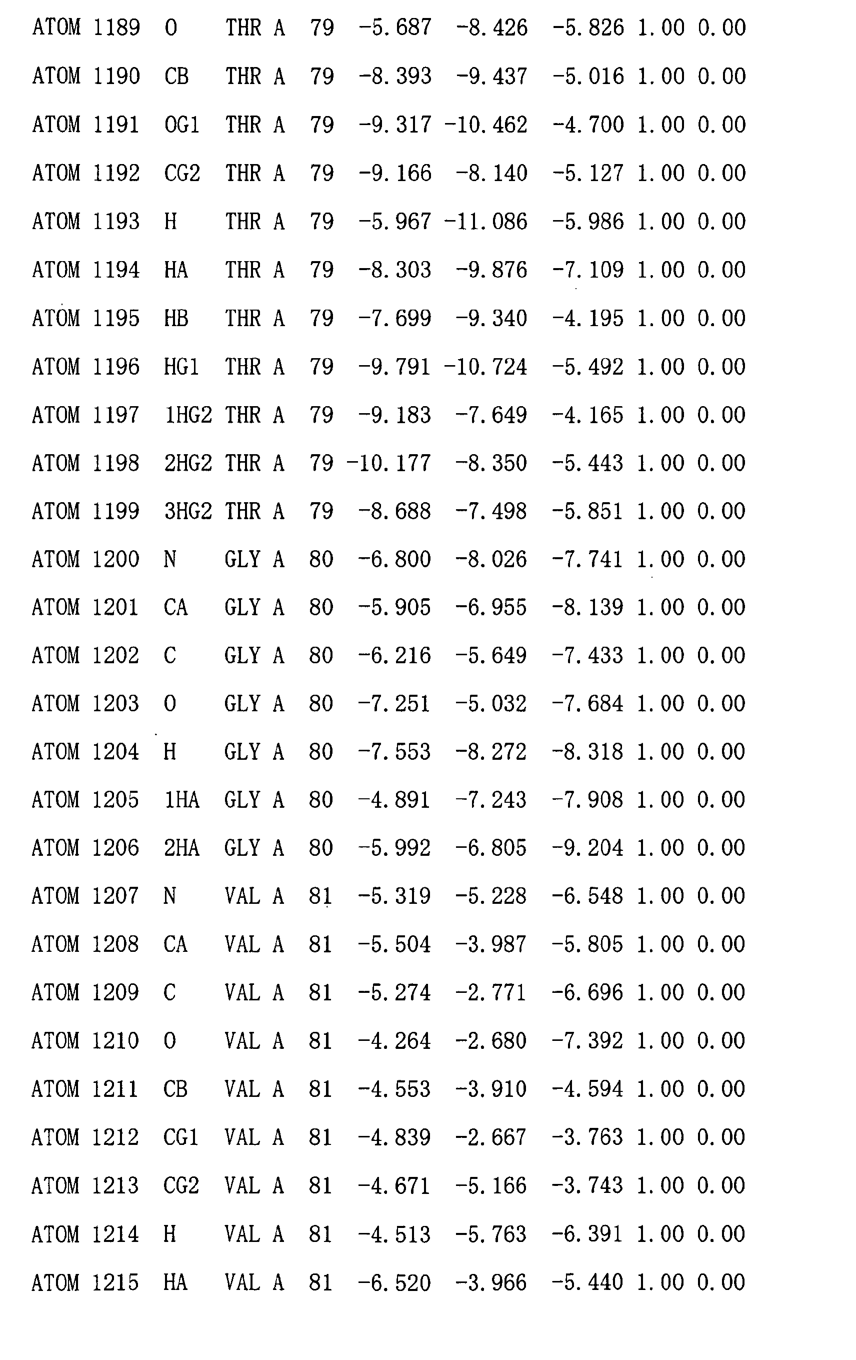

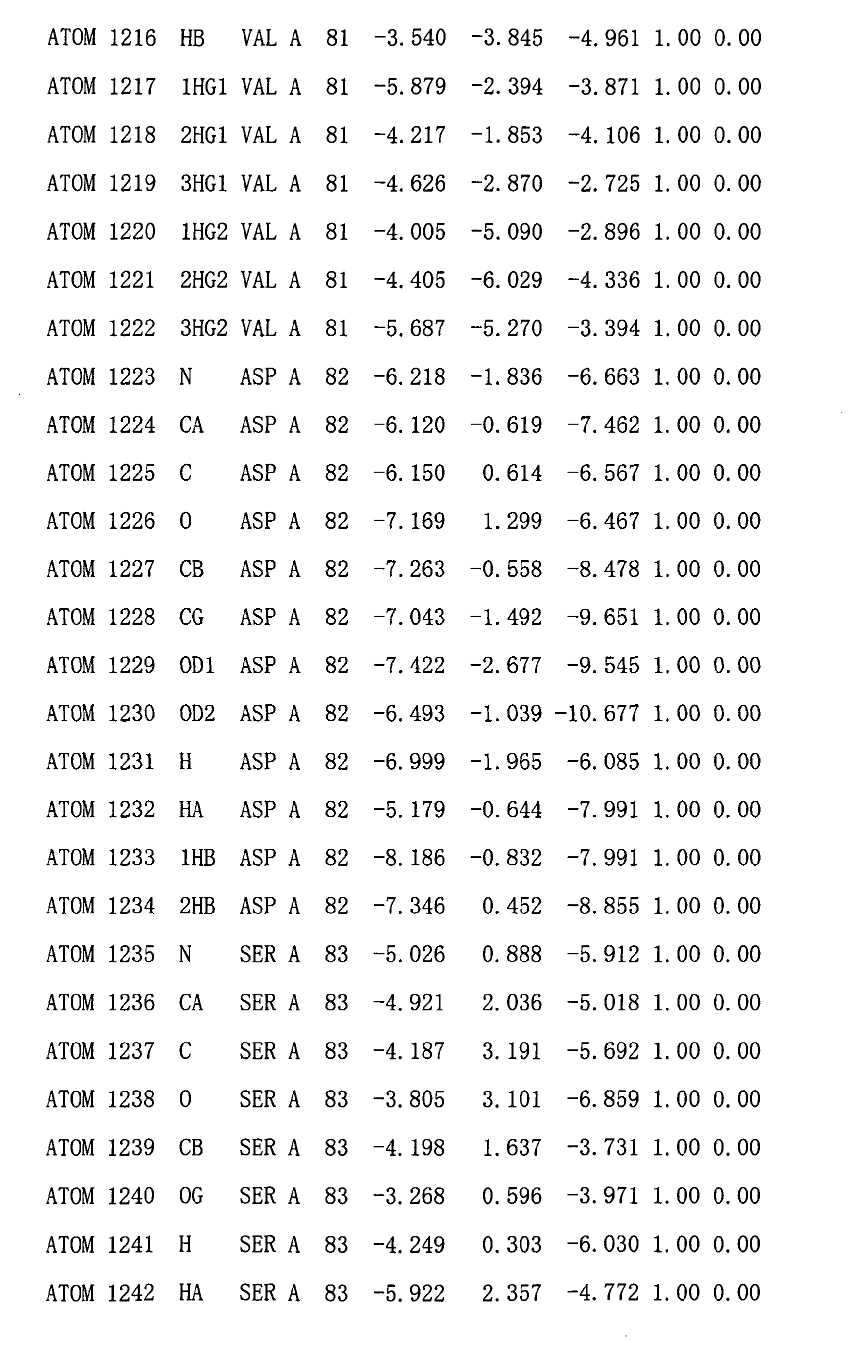

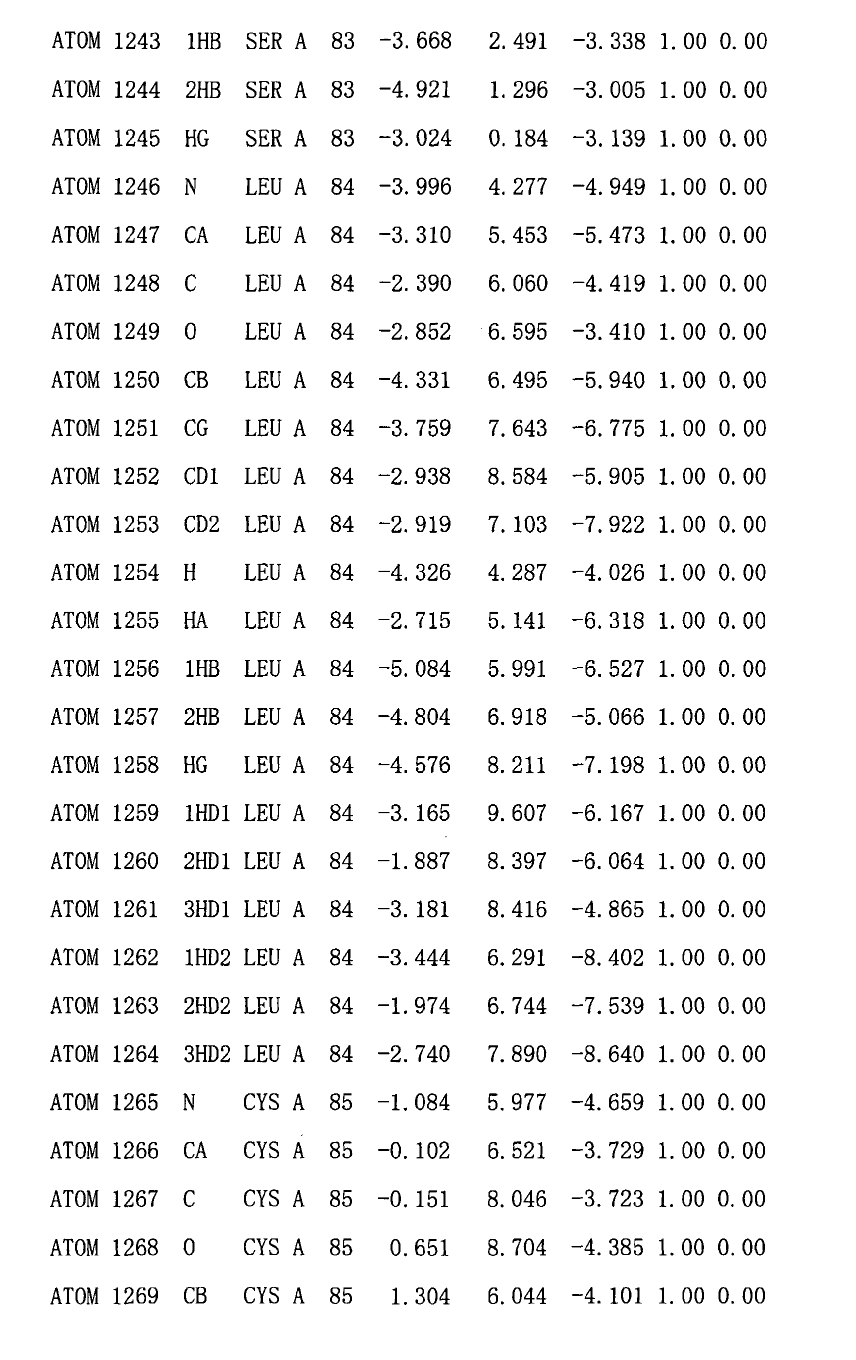

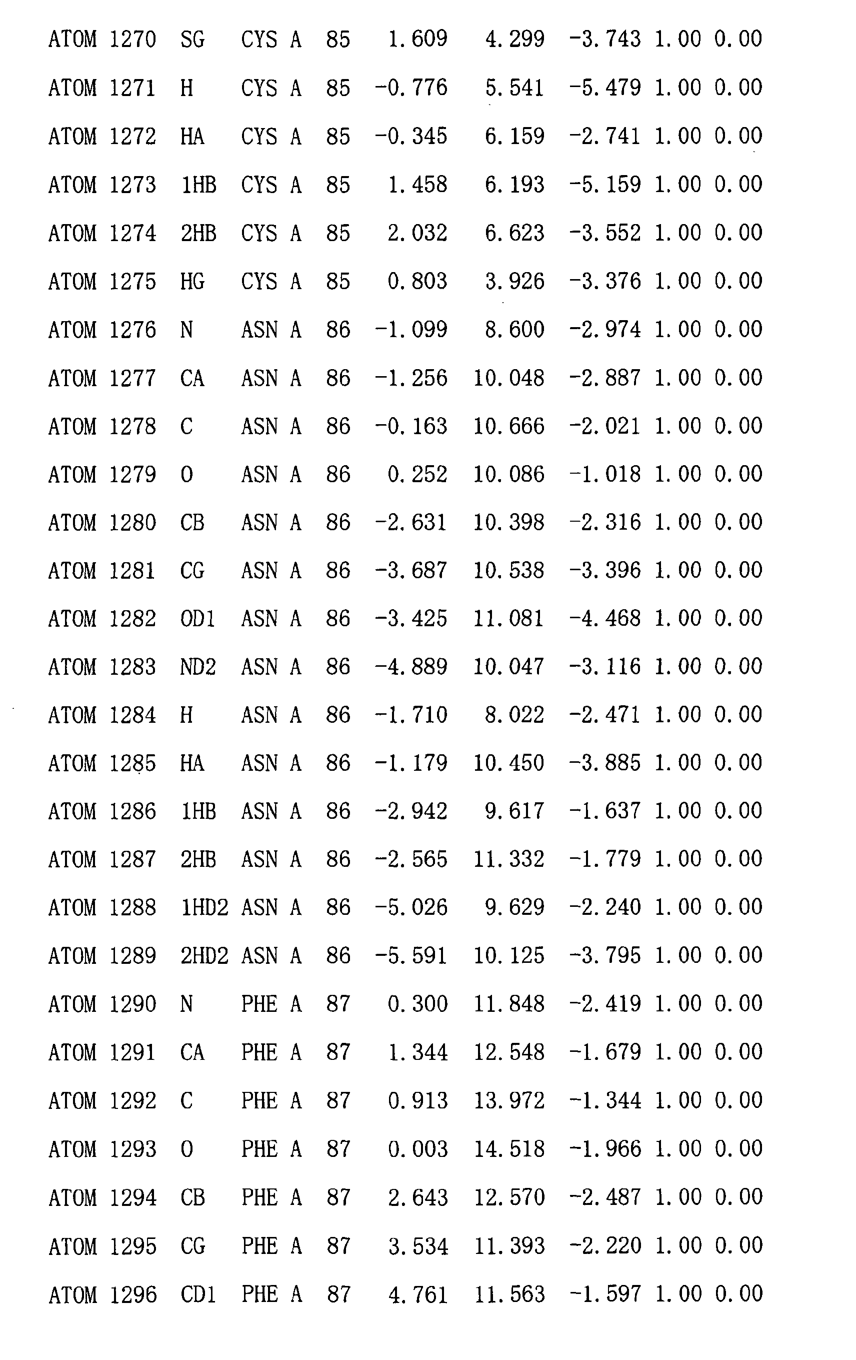

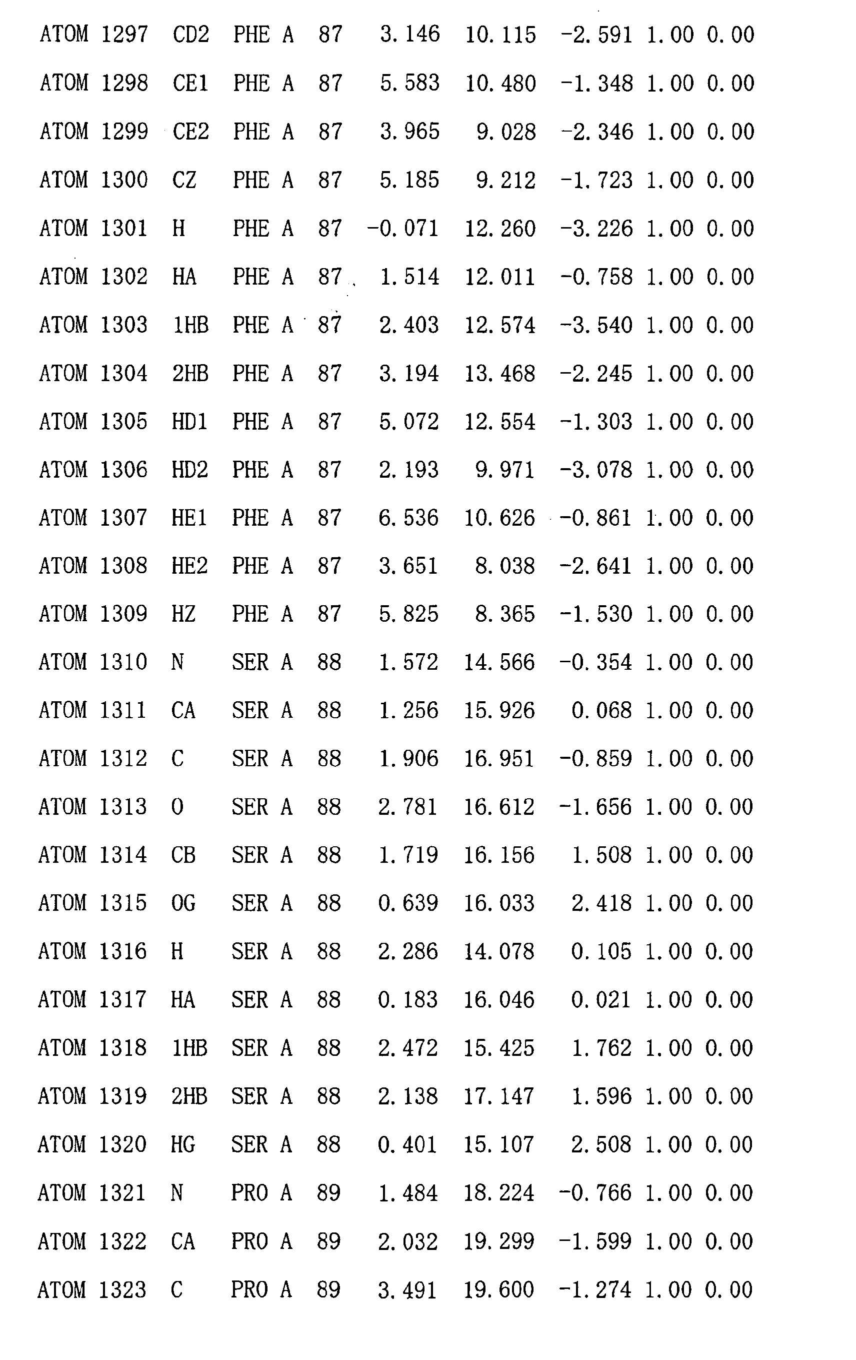

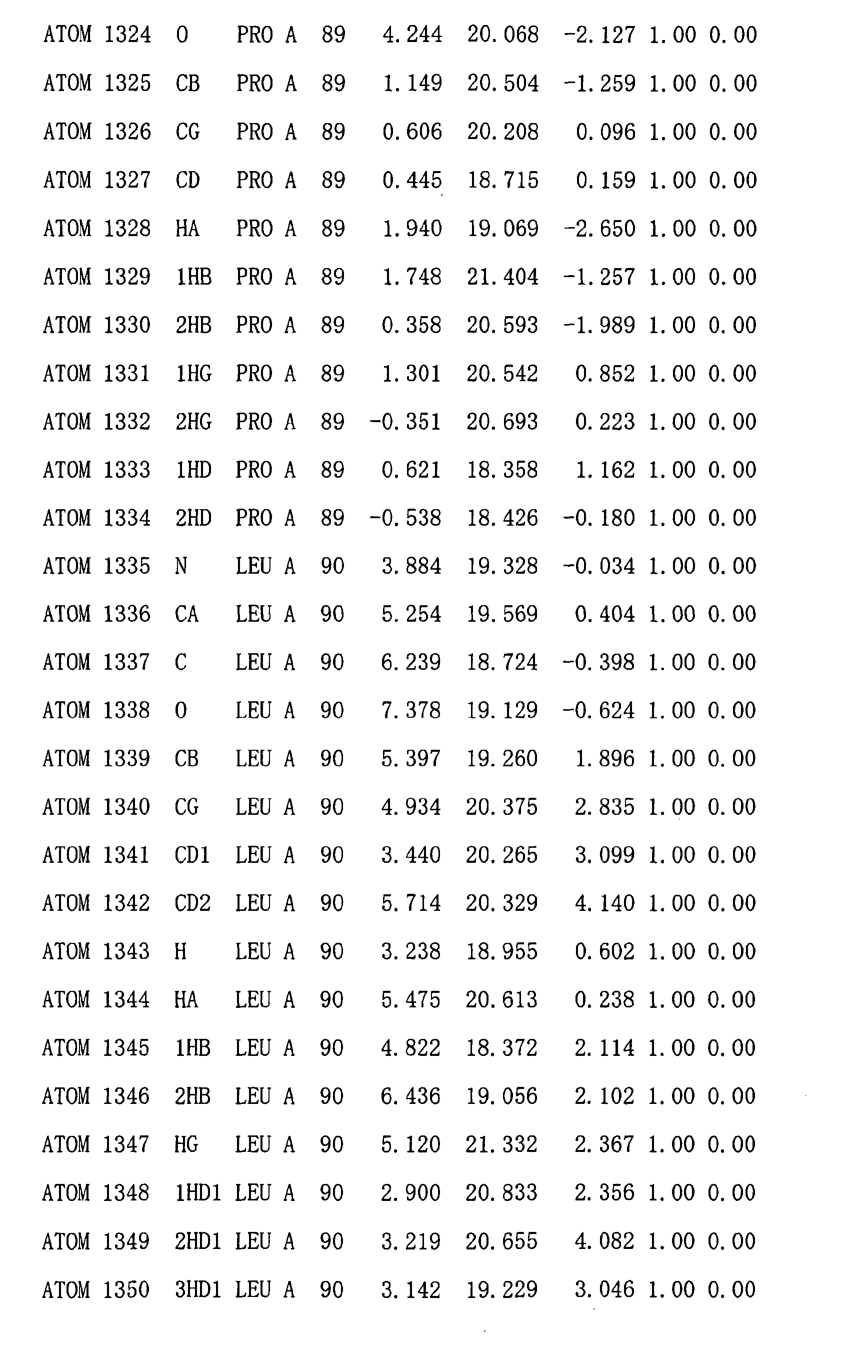

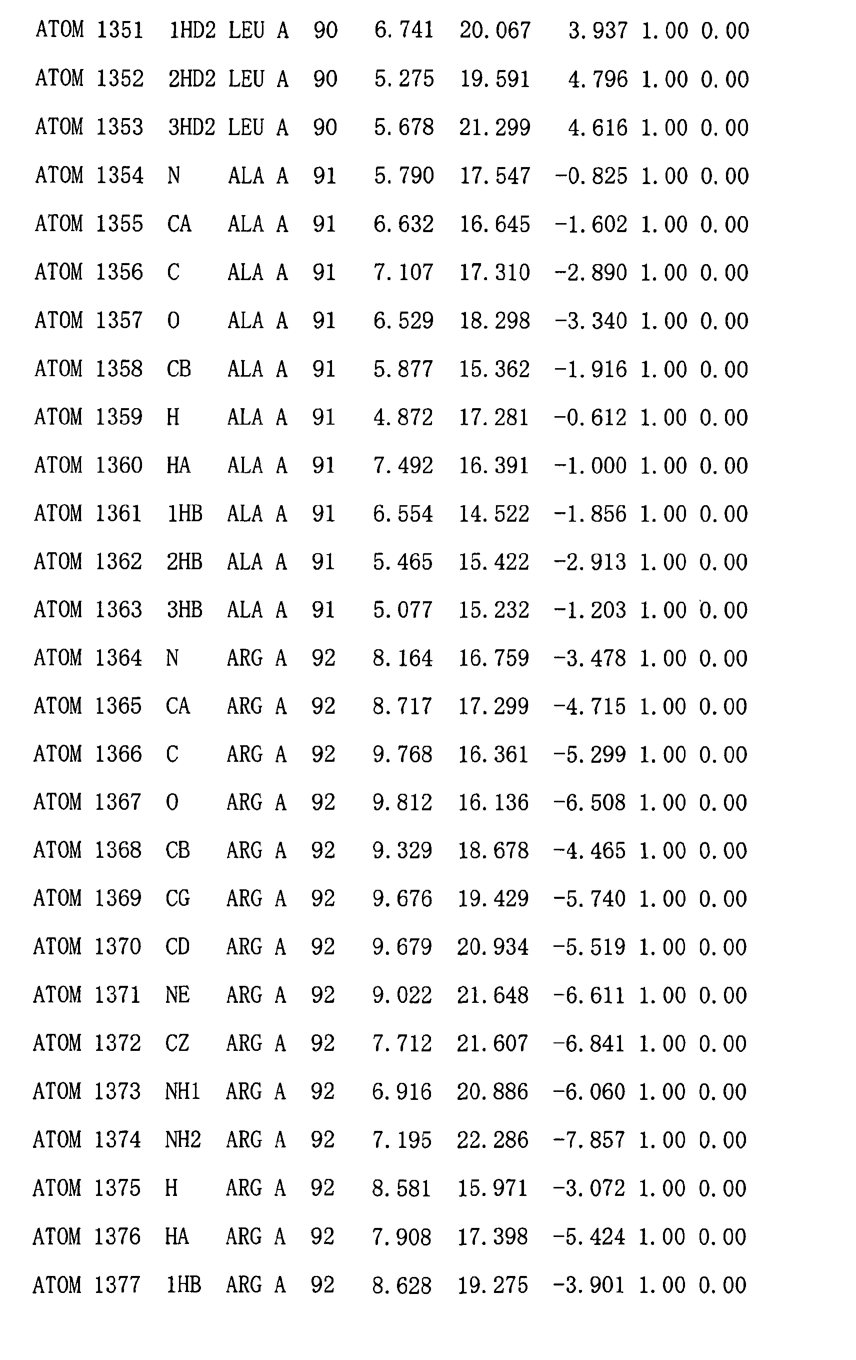

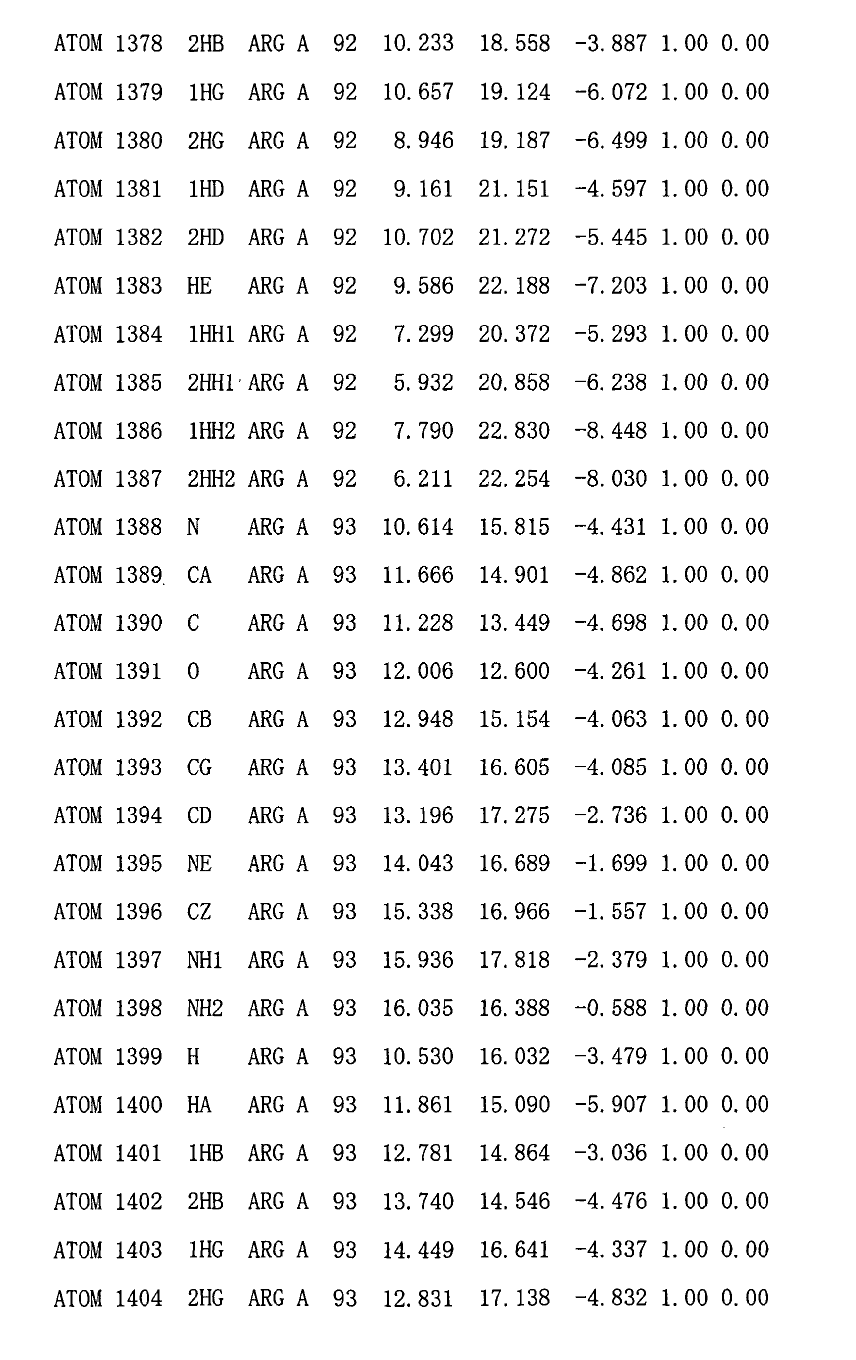

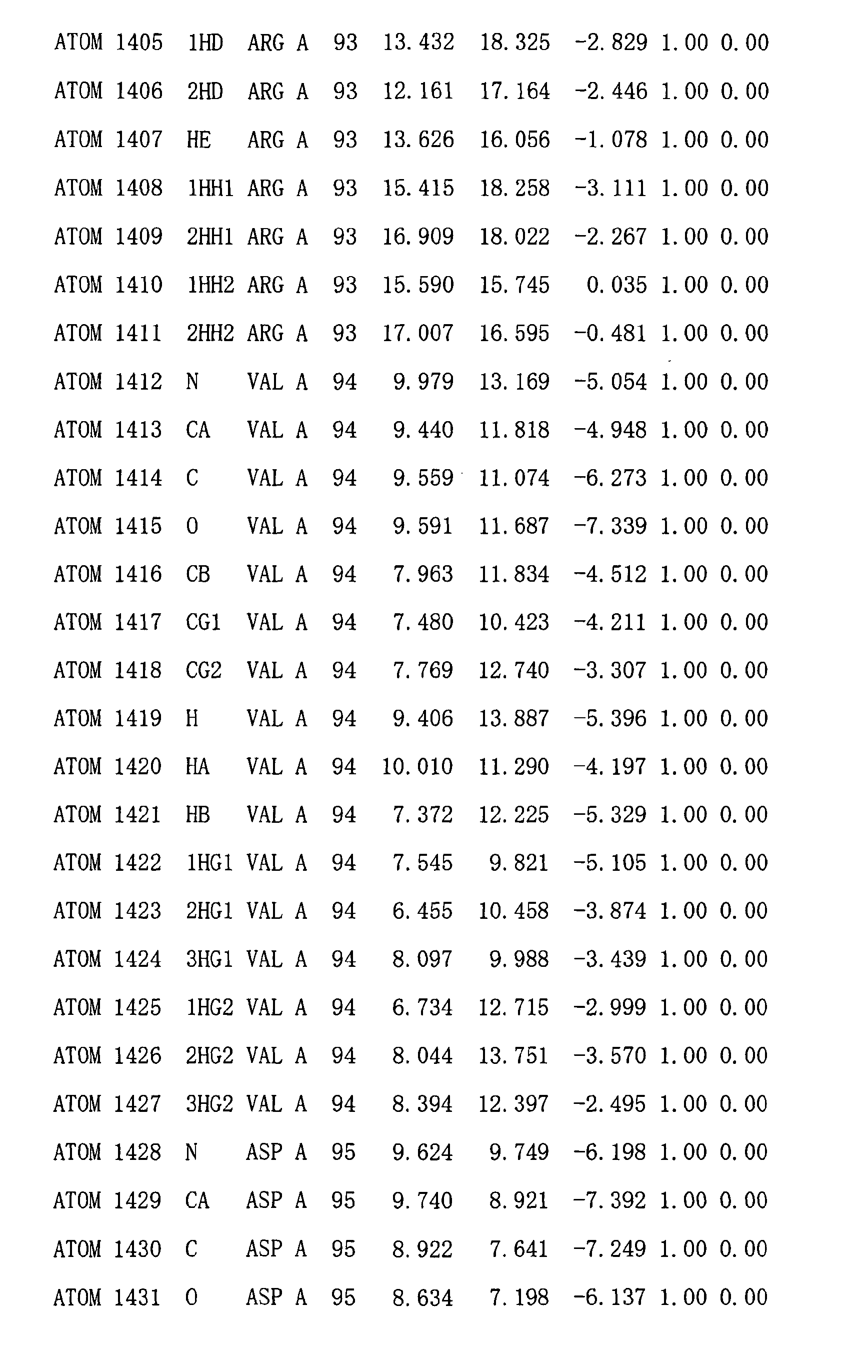

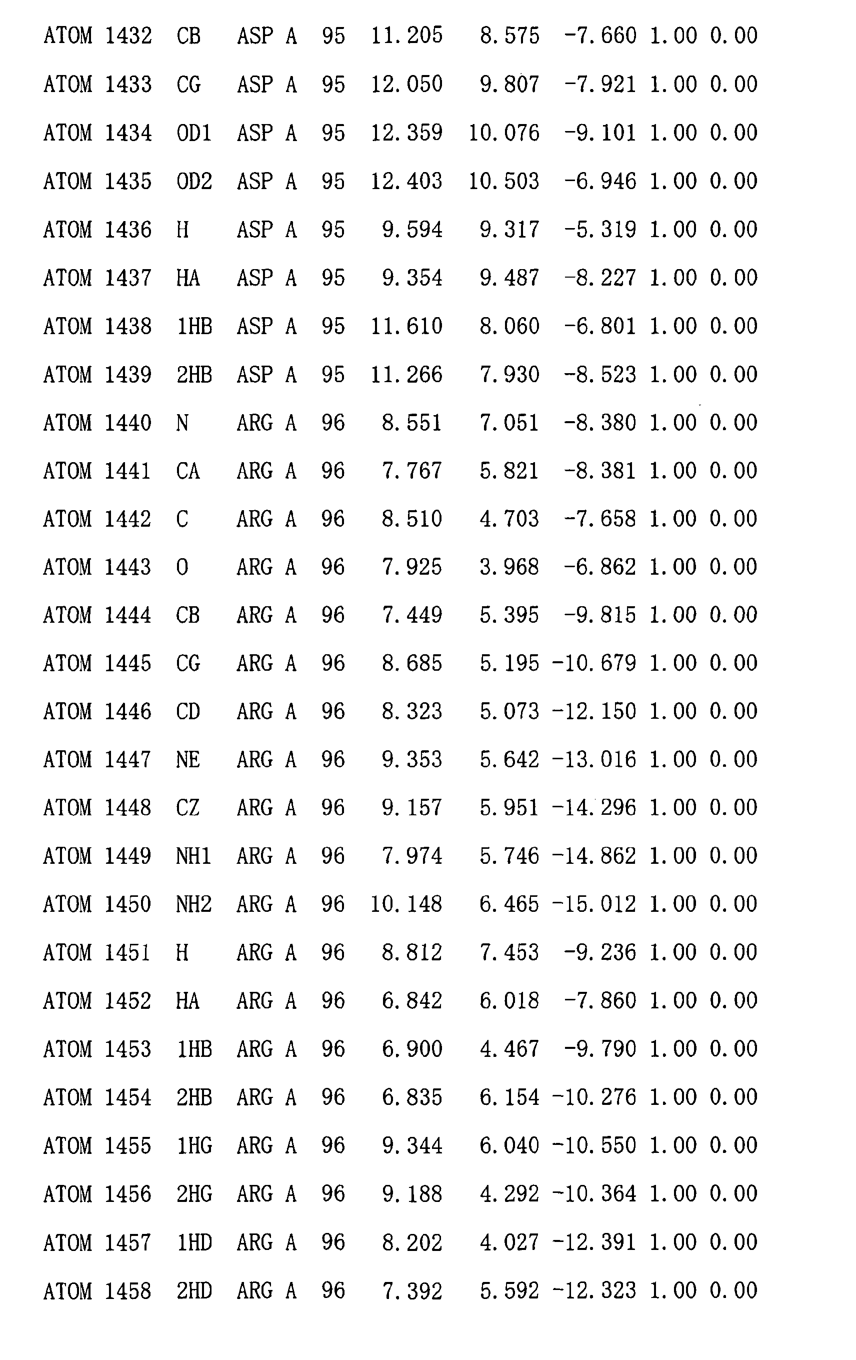

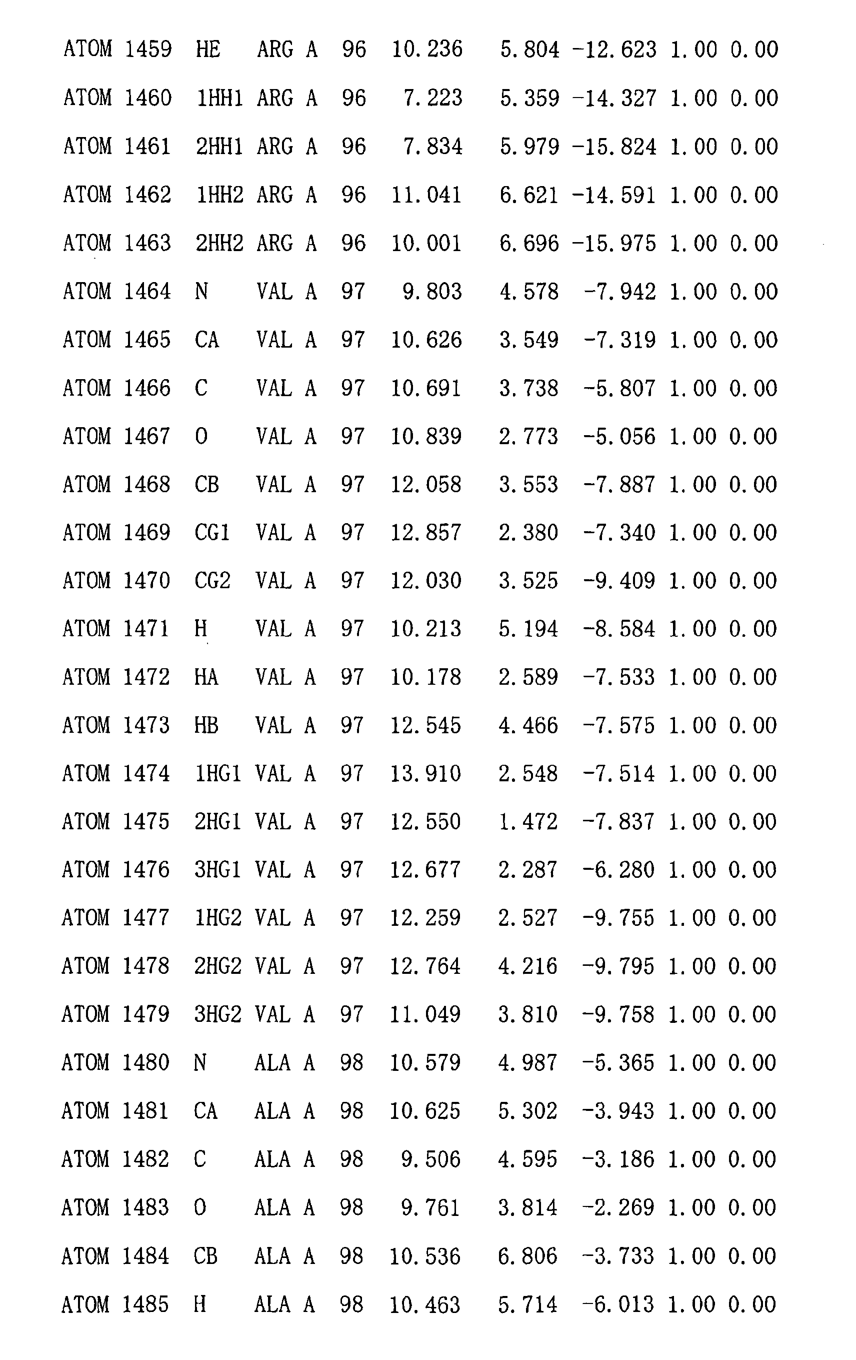

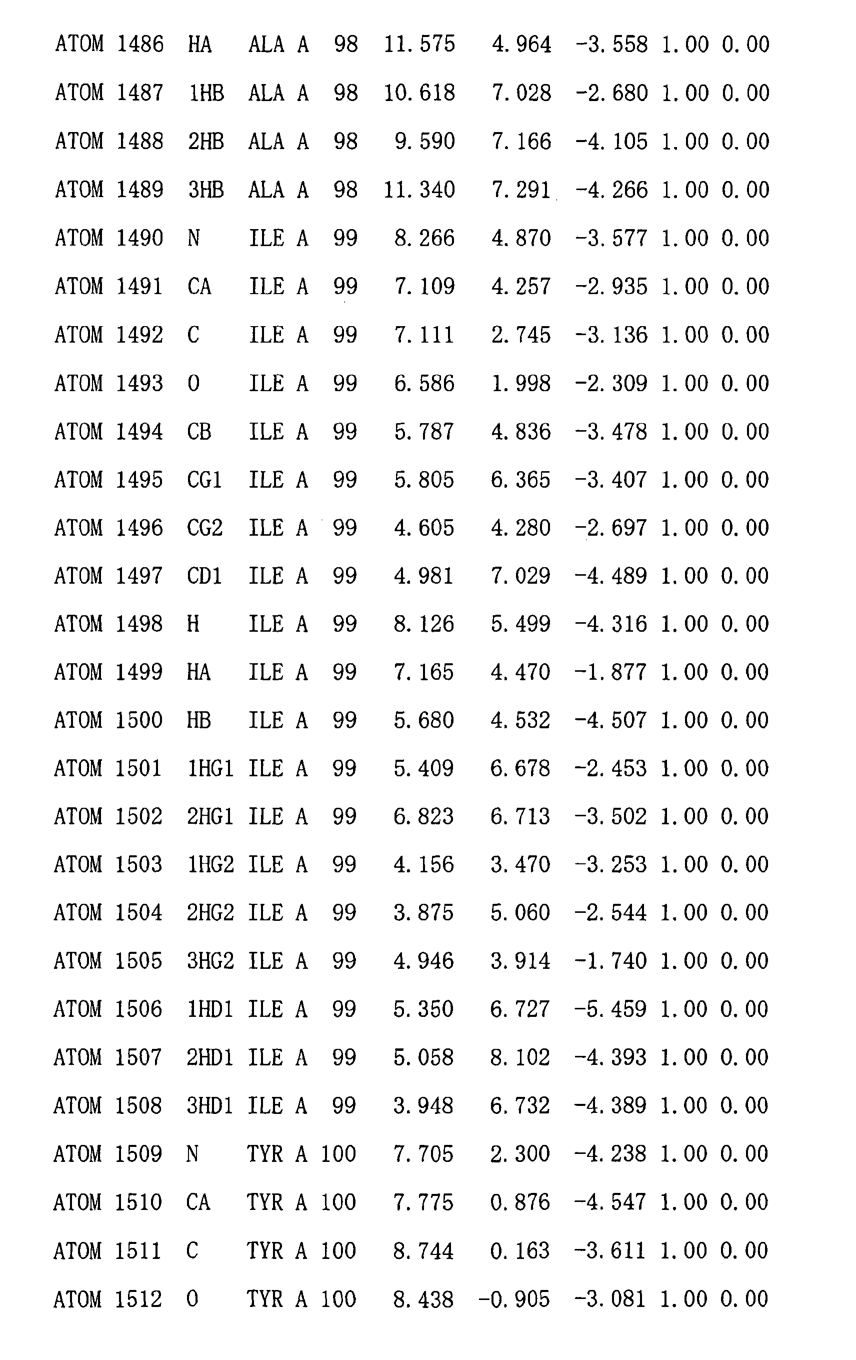

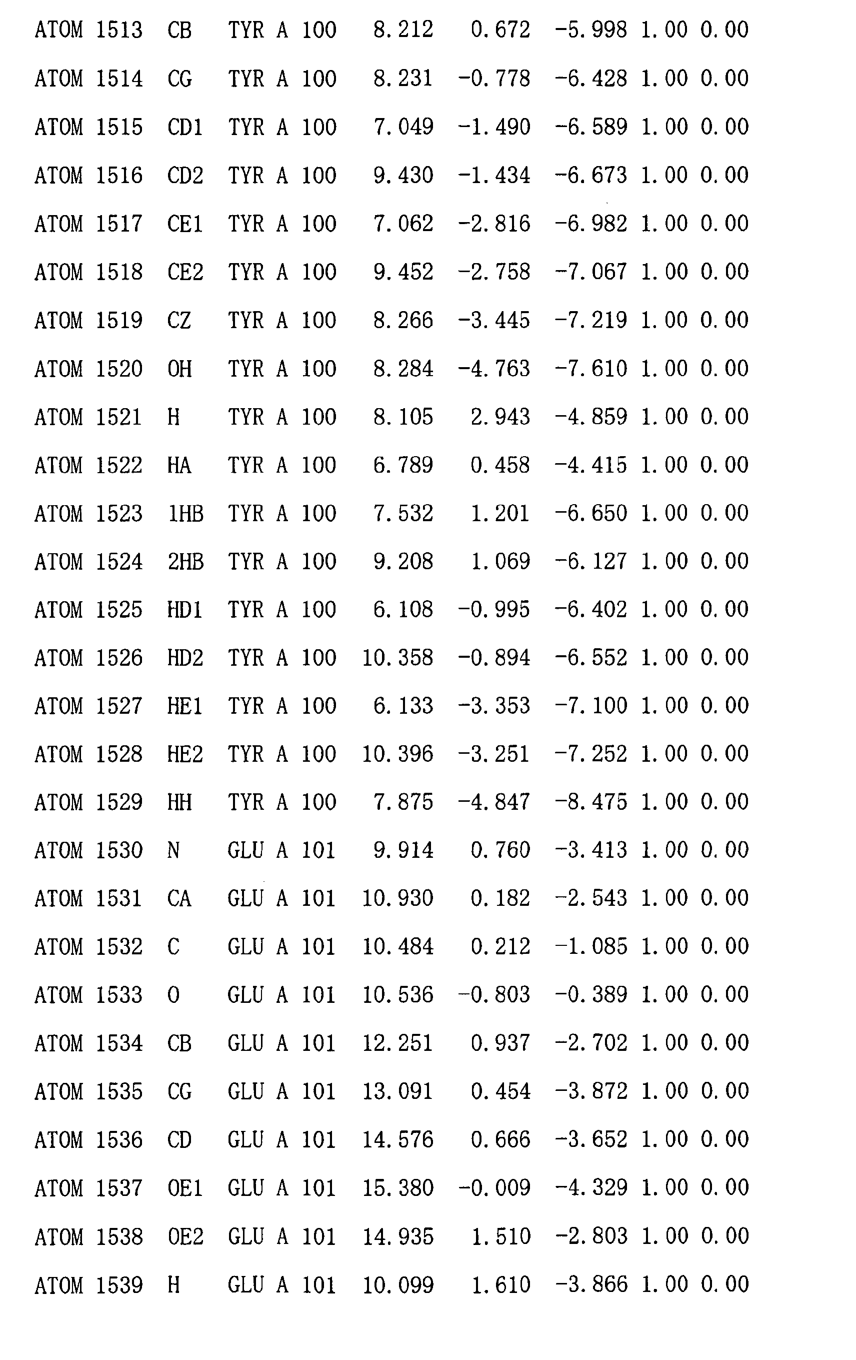

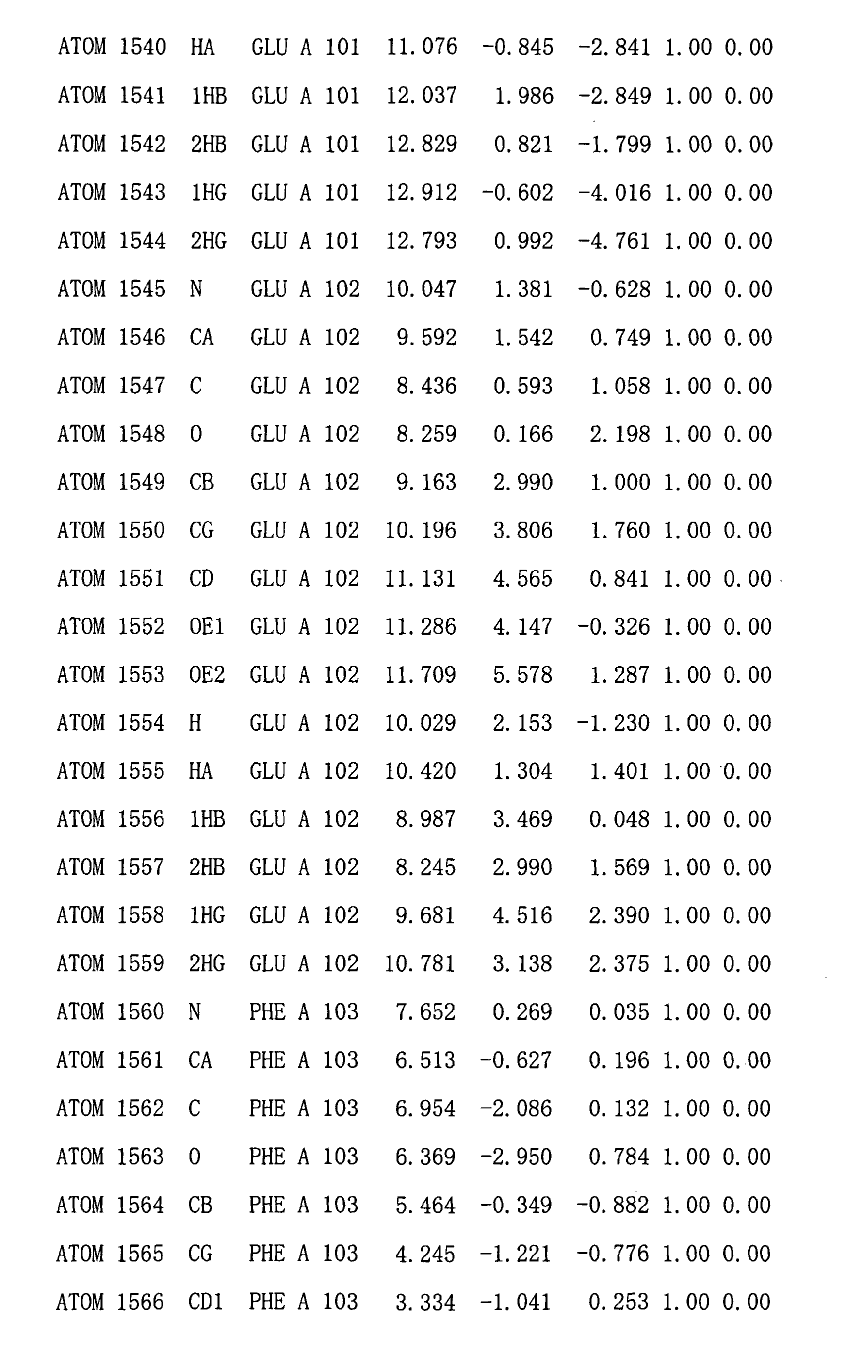

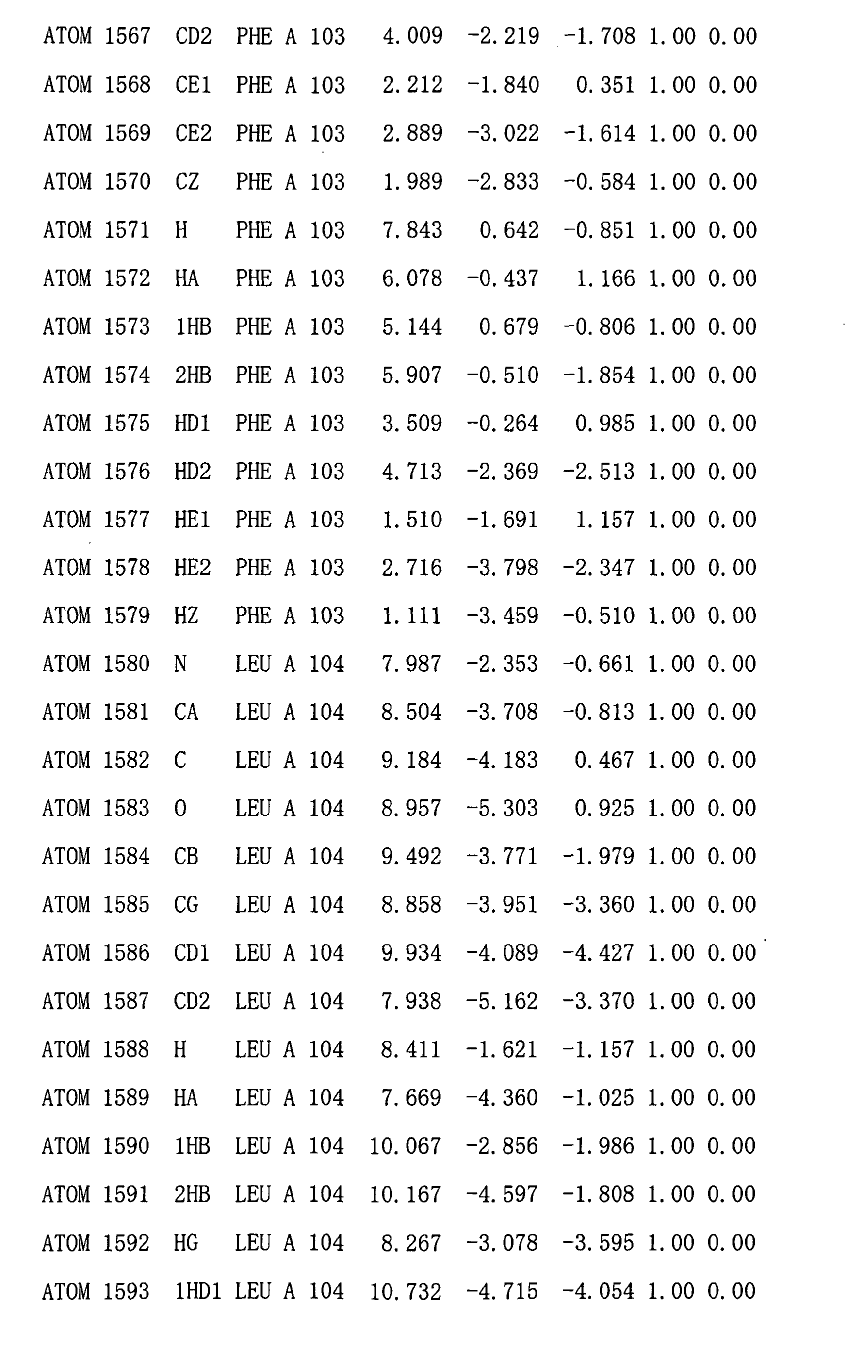

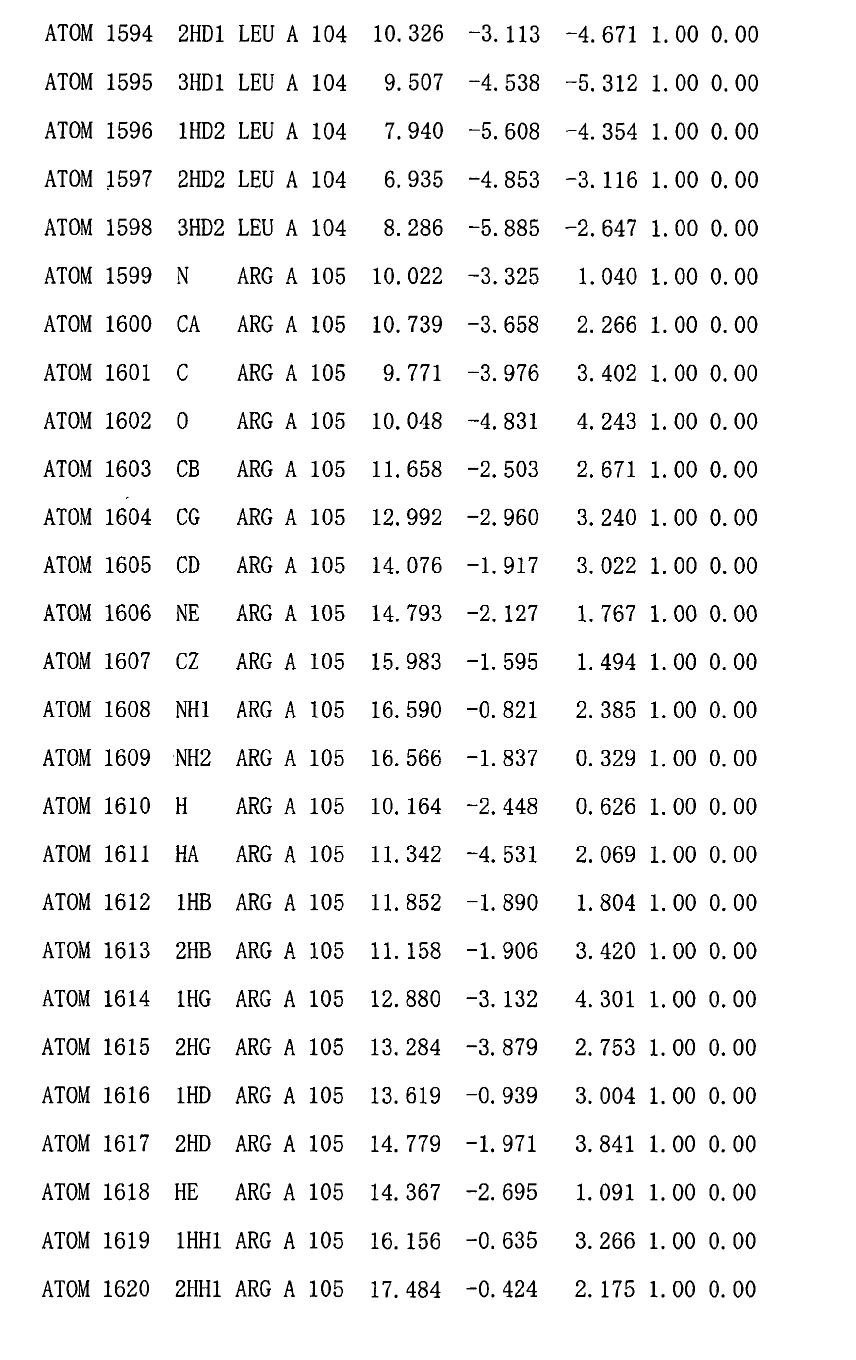

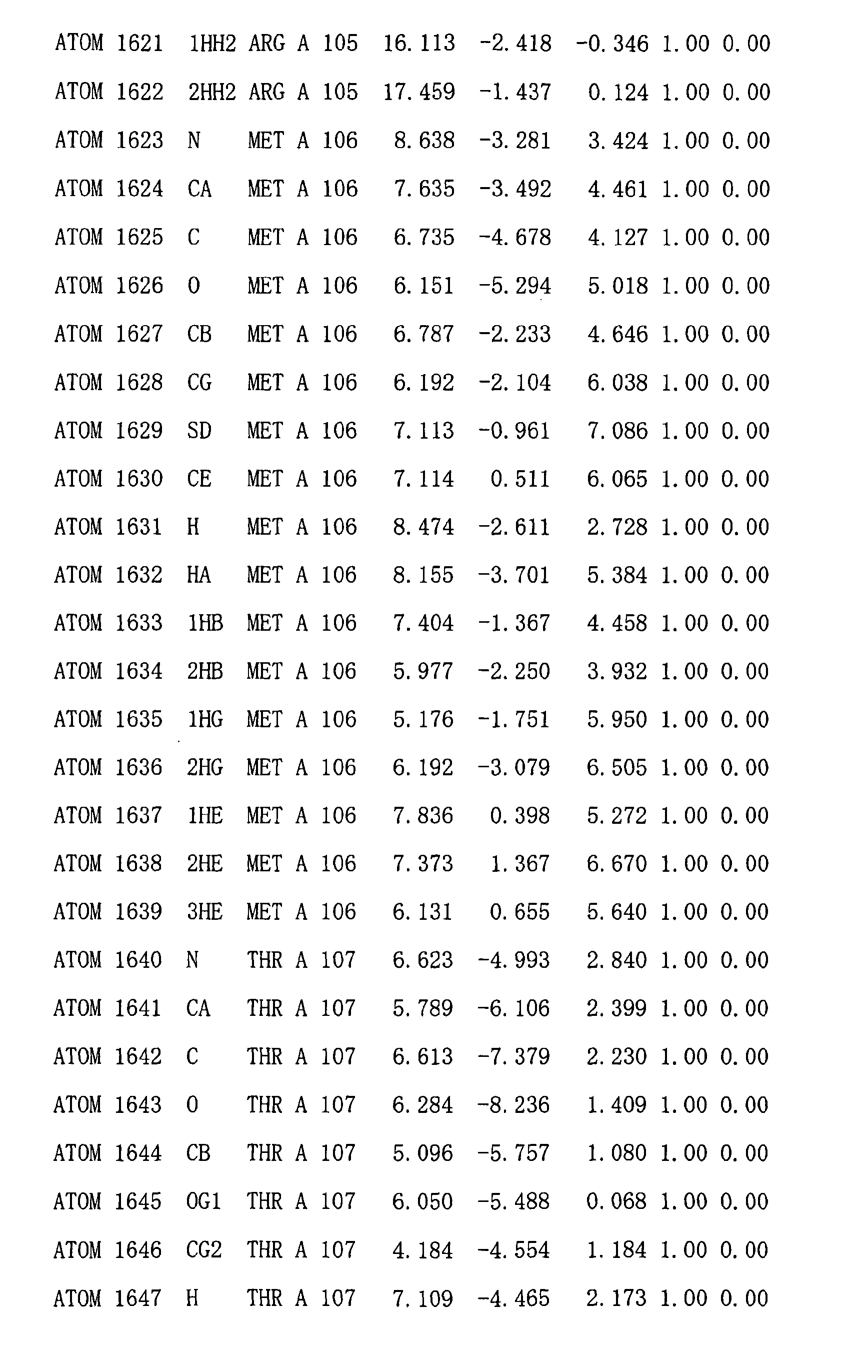

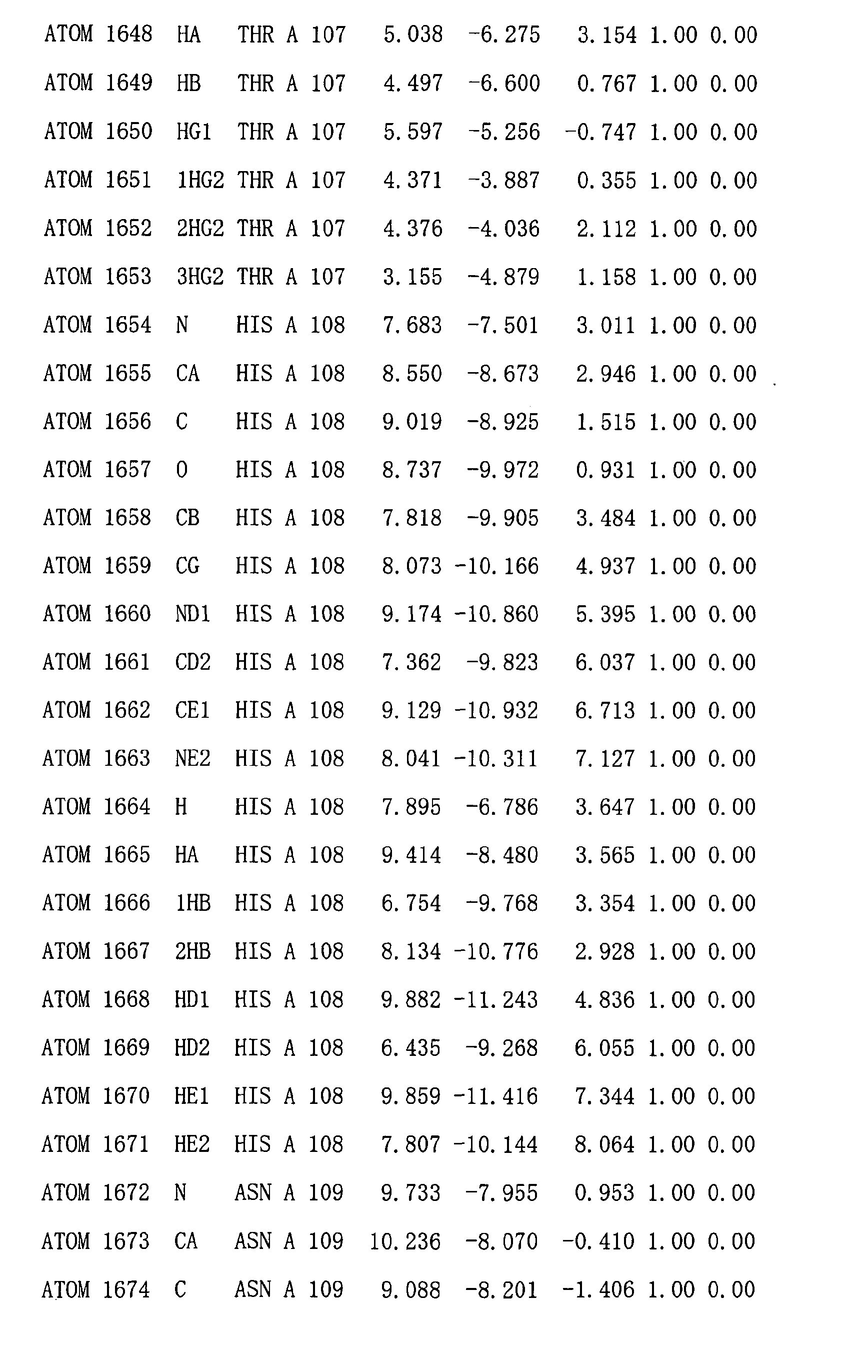

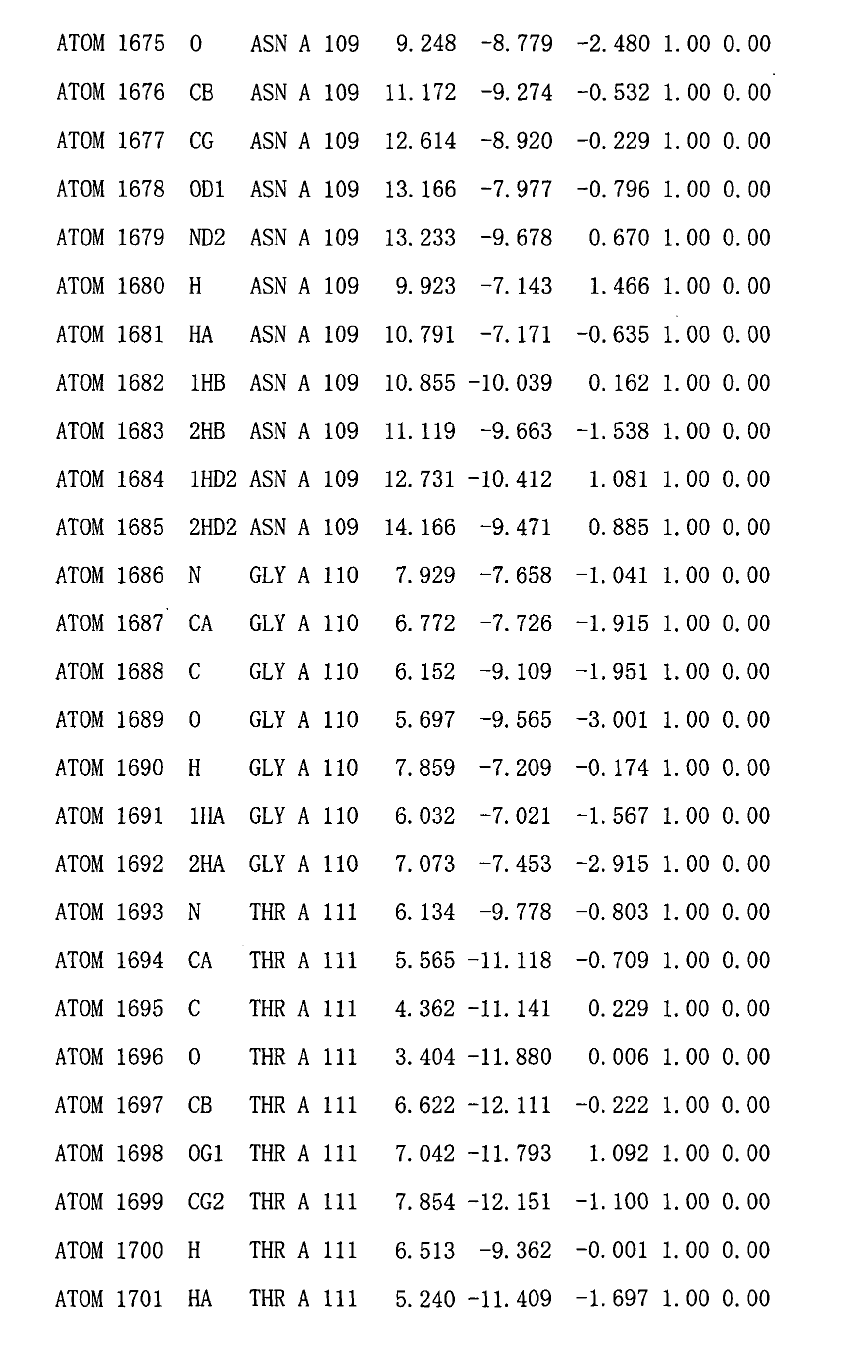

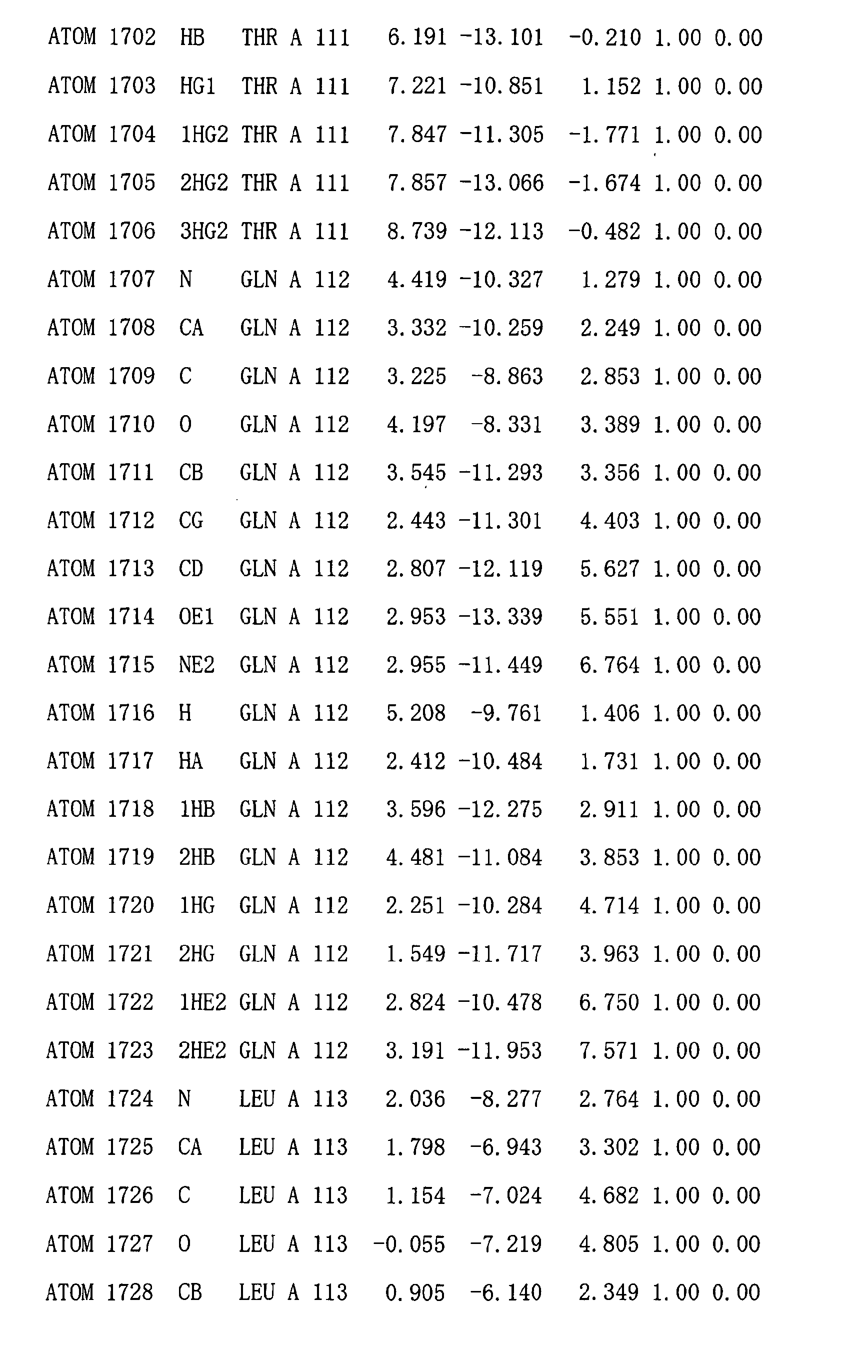

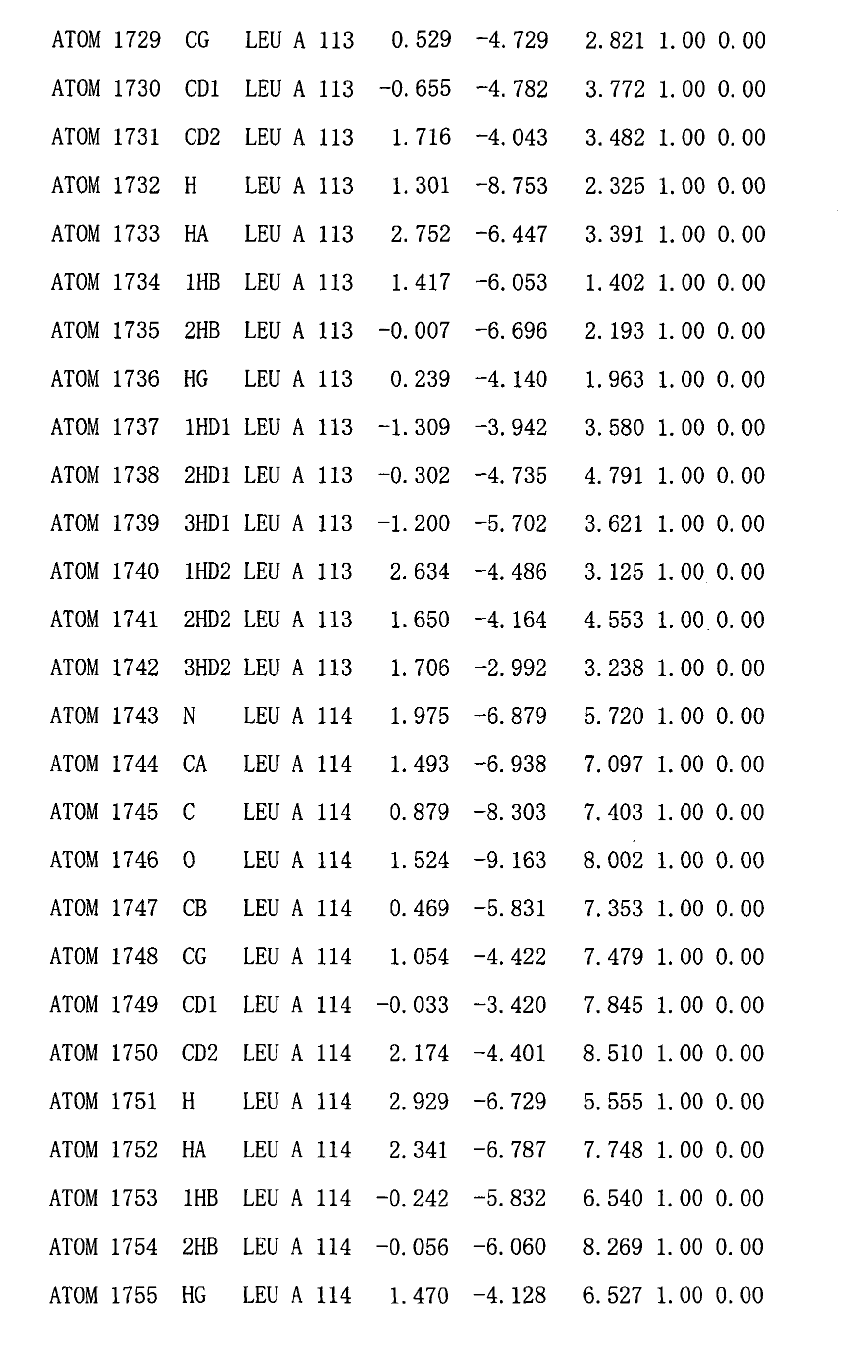

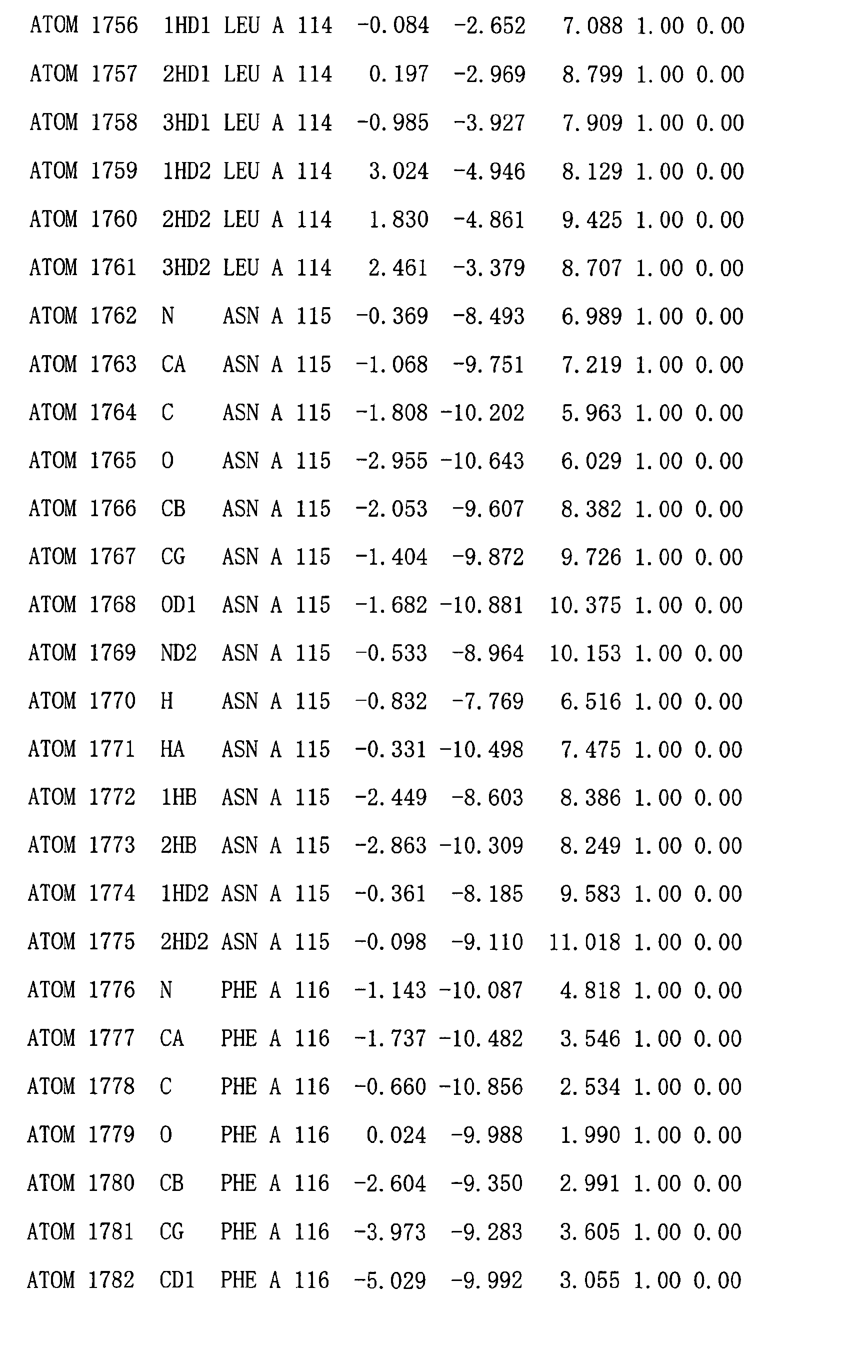

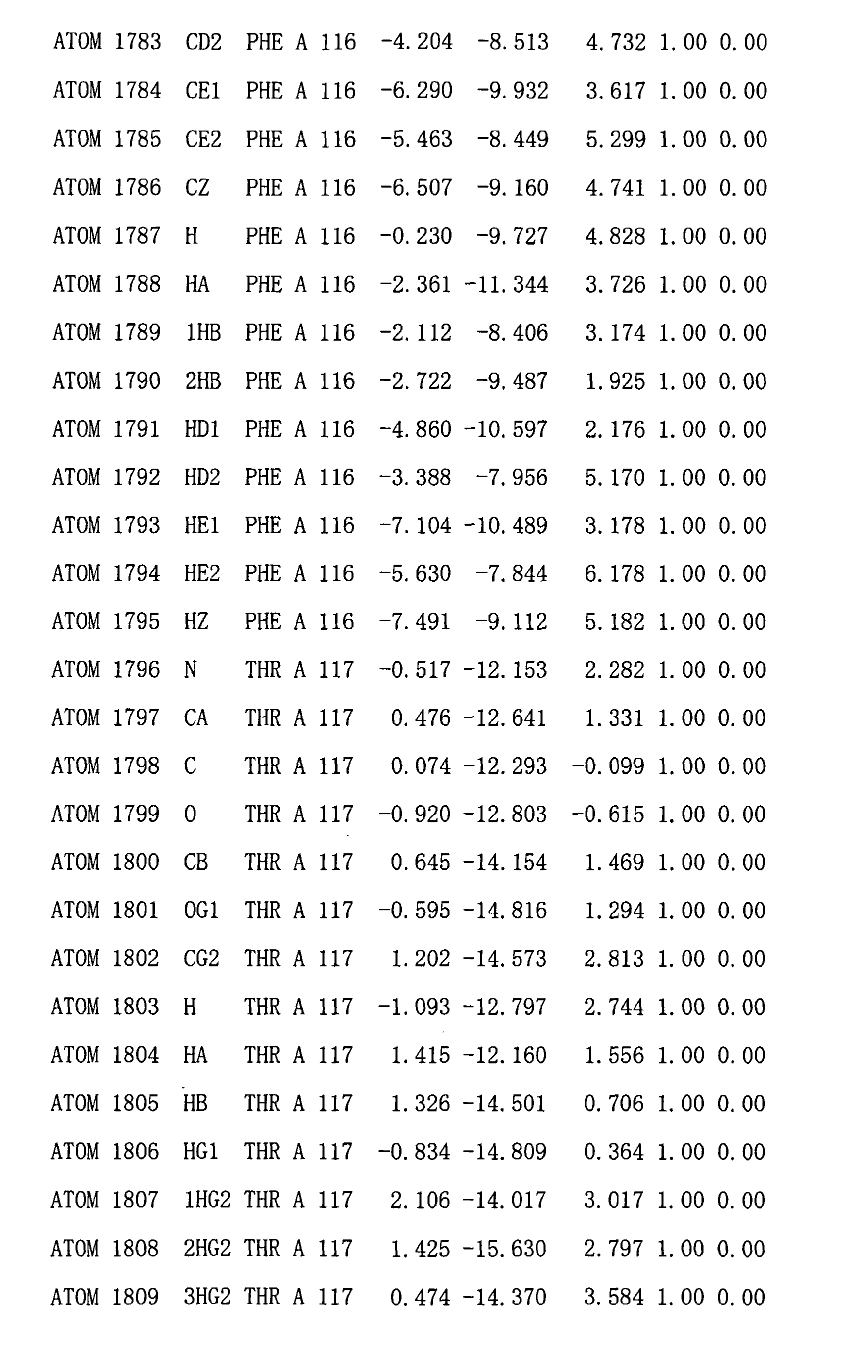

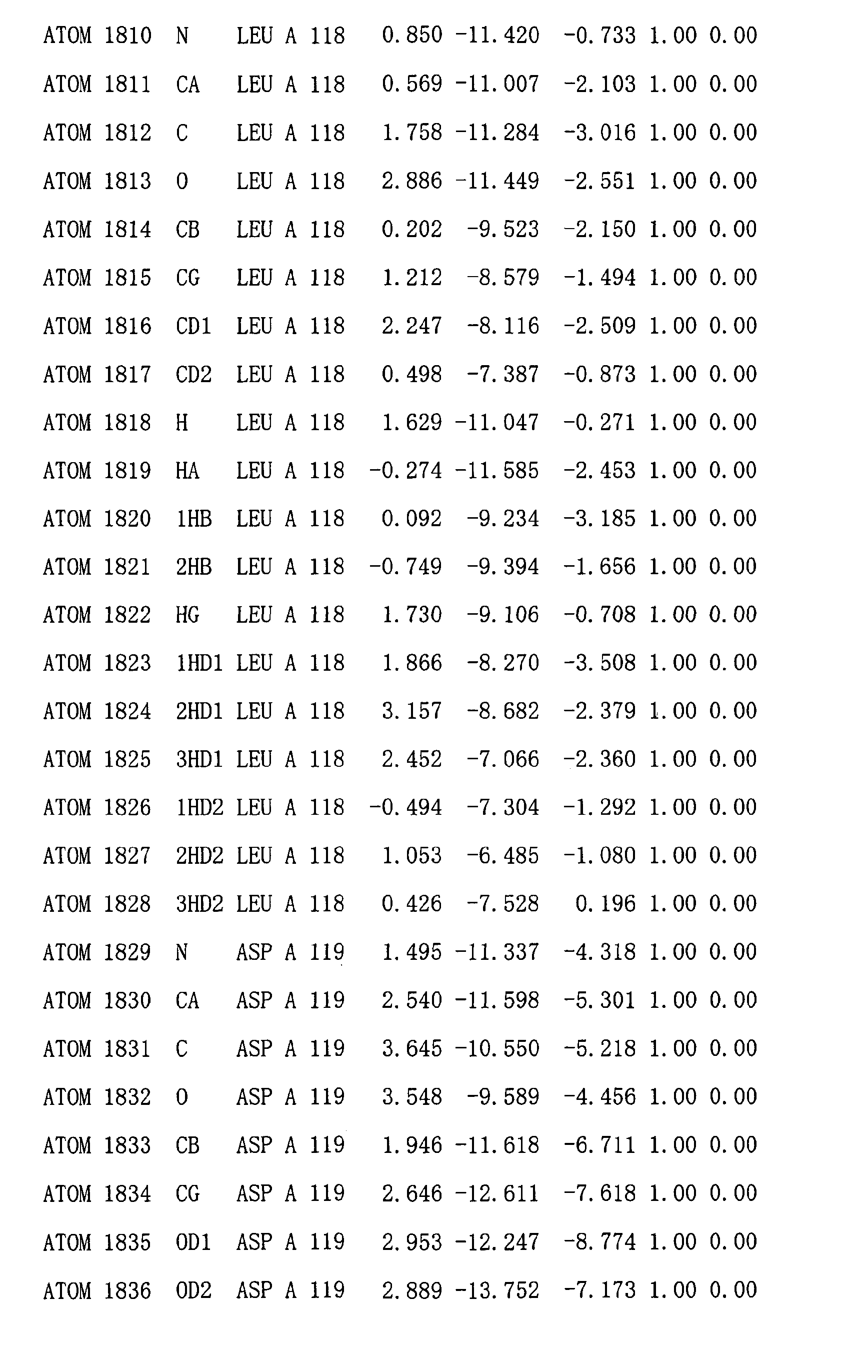

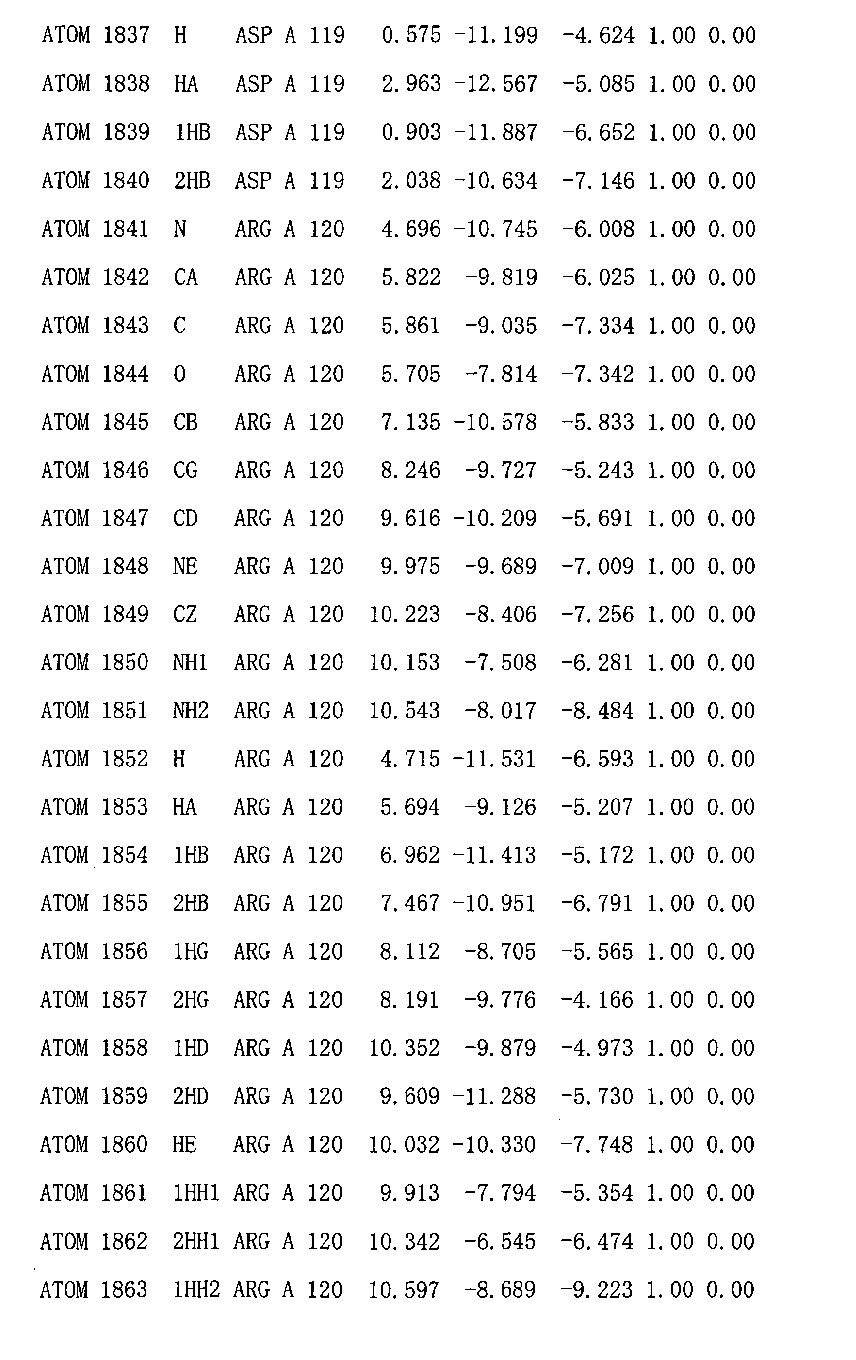

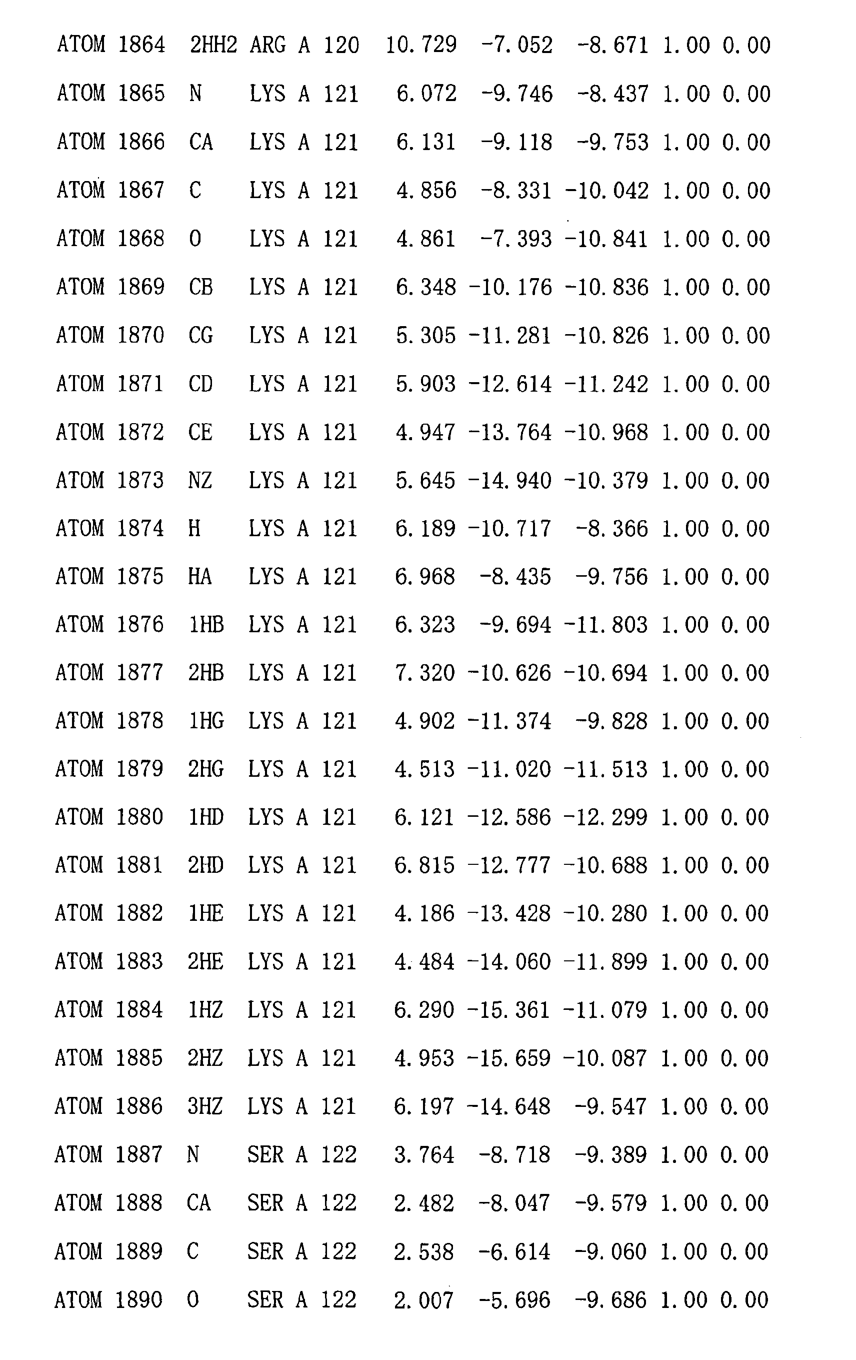

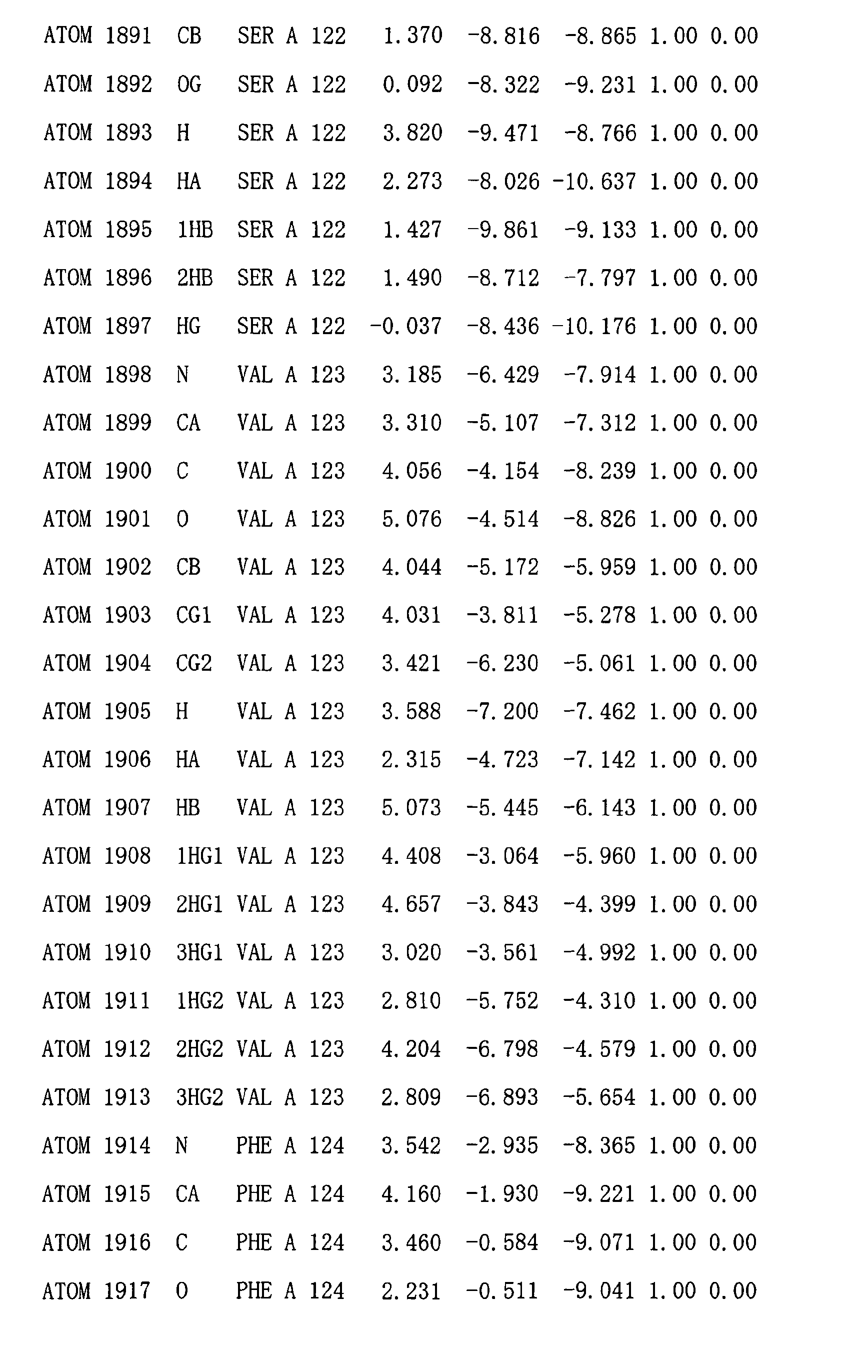

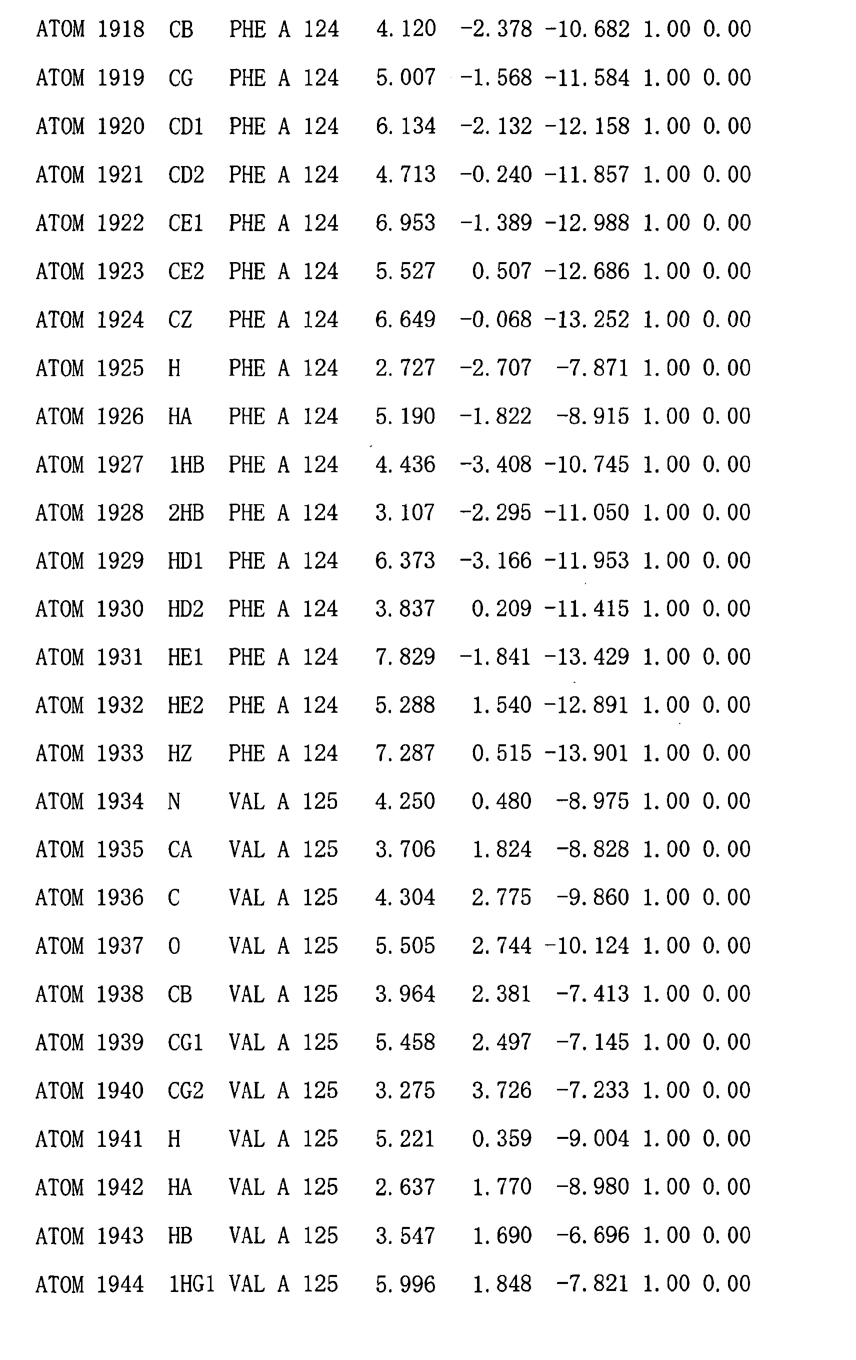

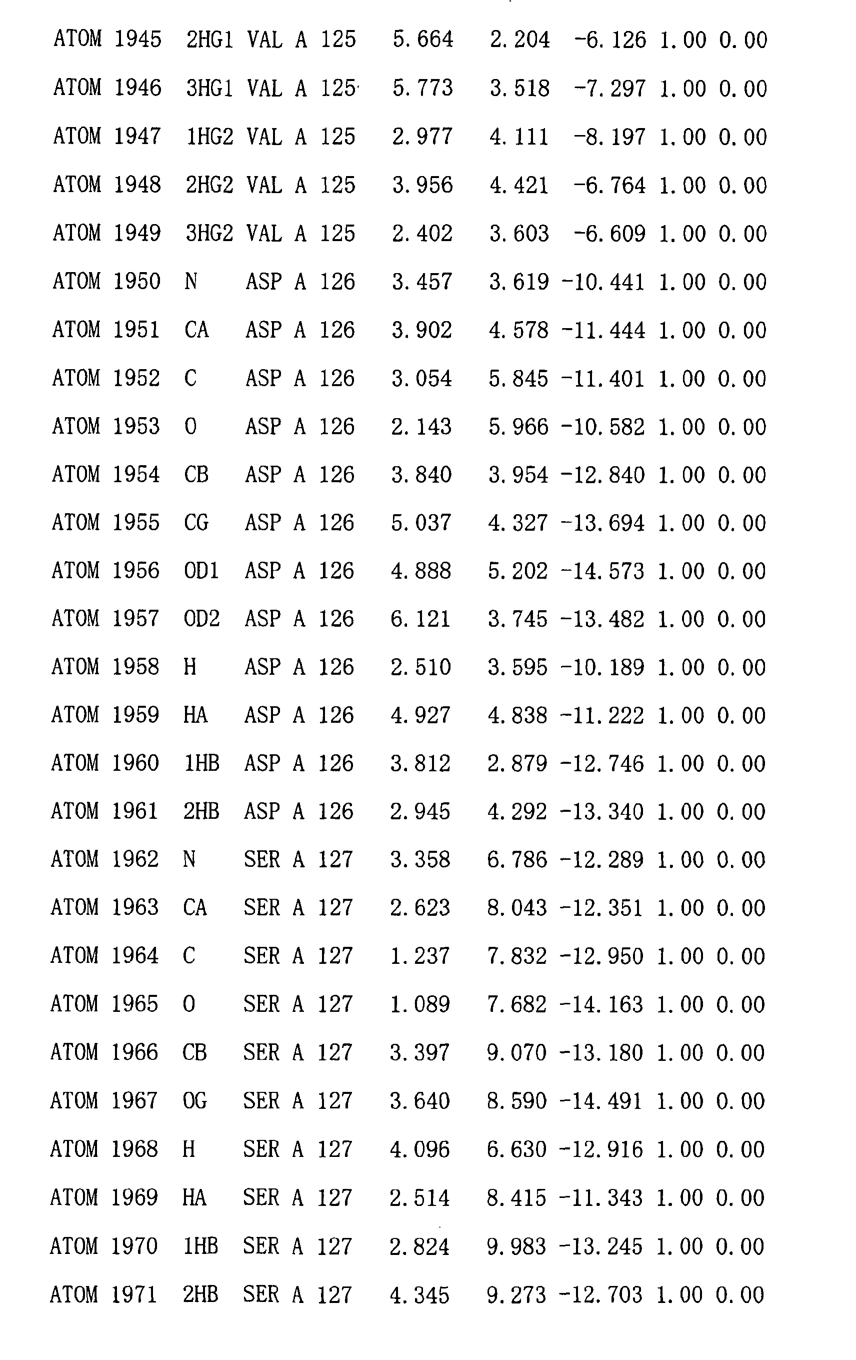

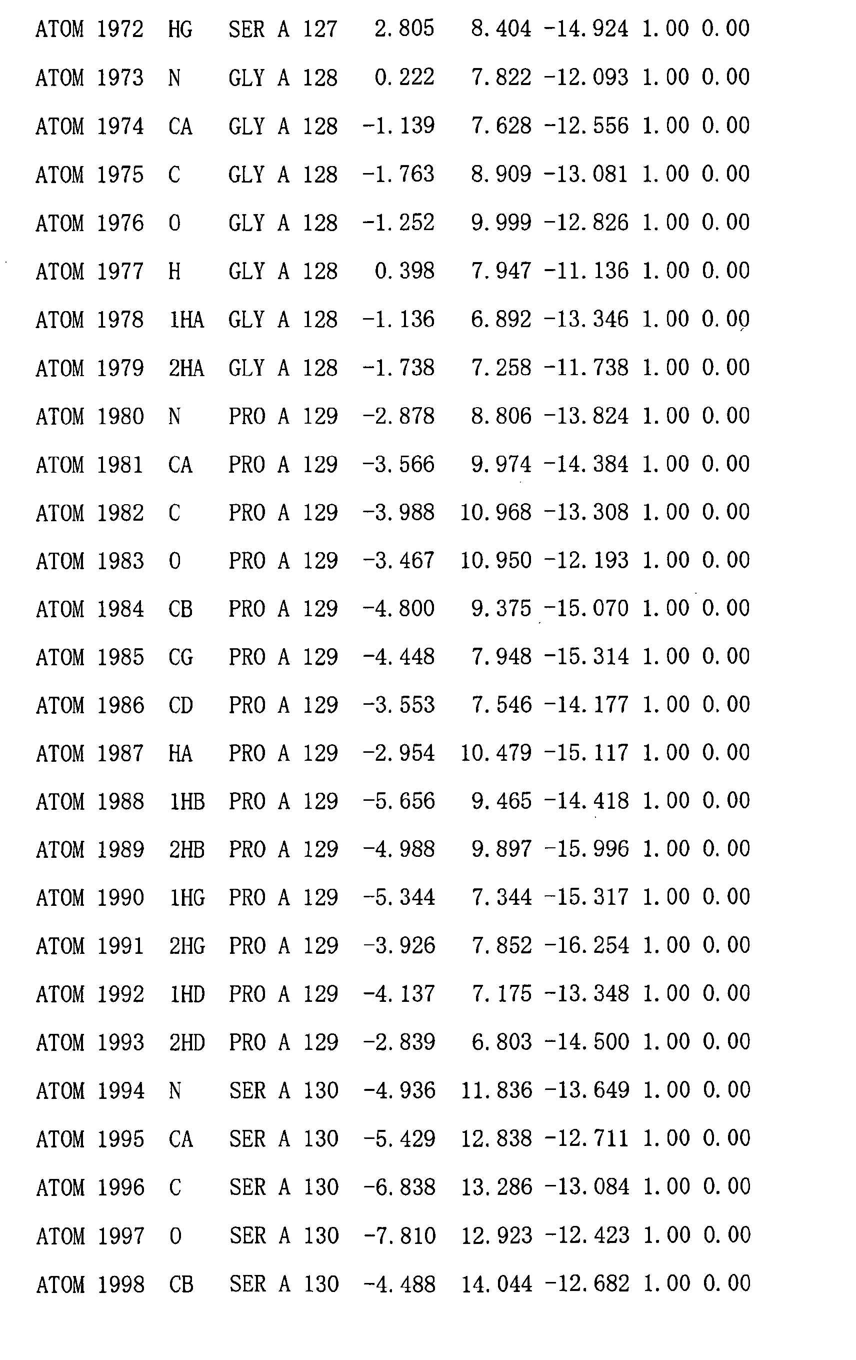

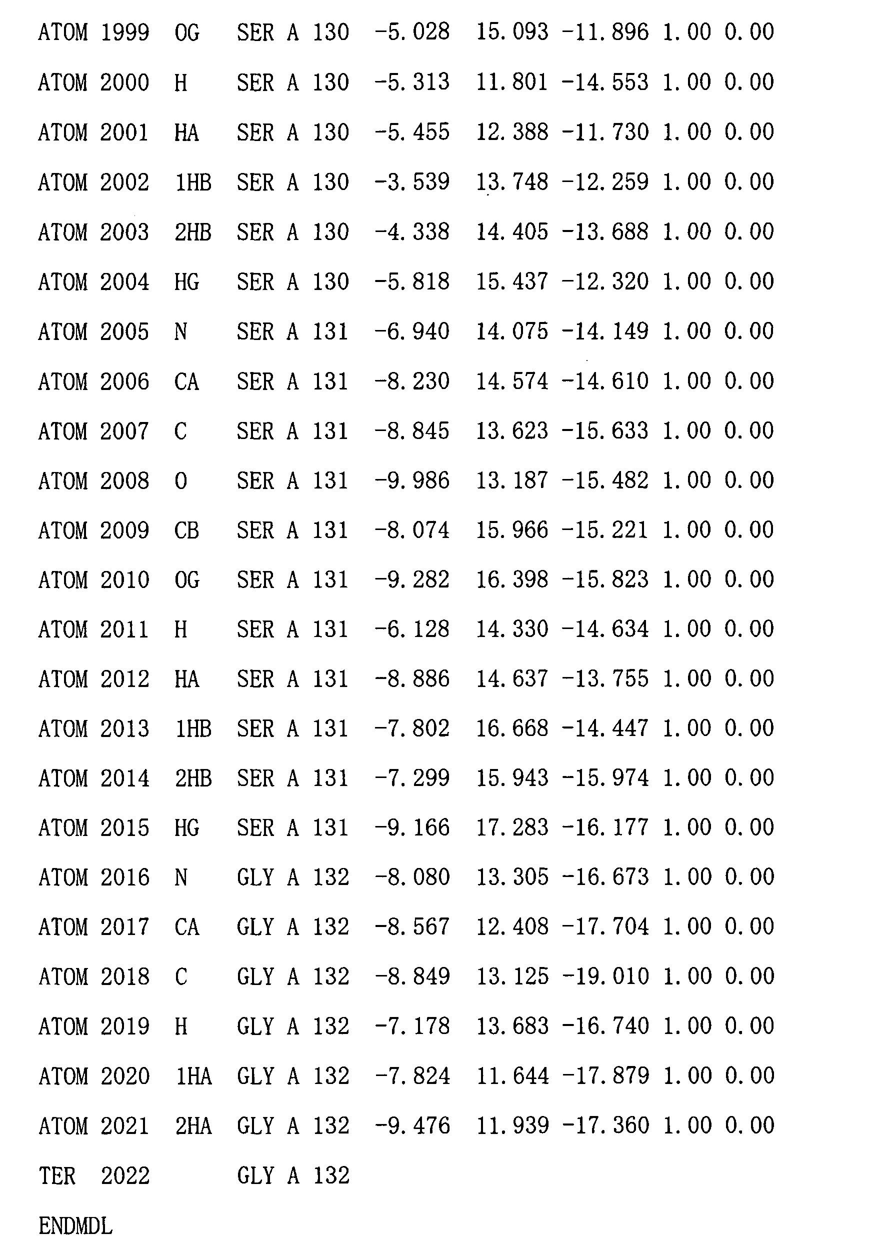

### Three-Dimensional Structure Coordinate 9

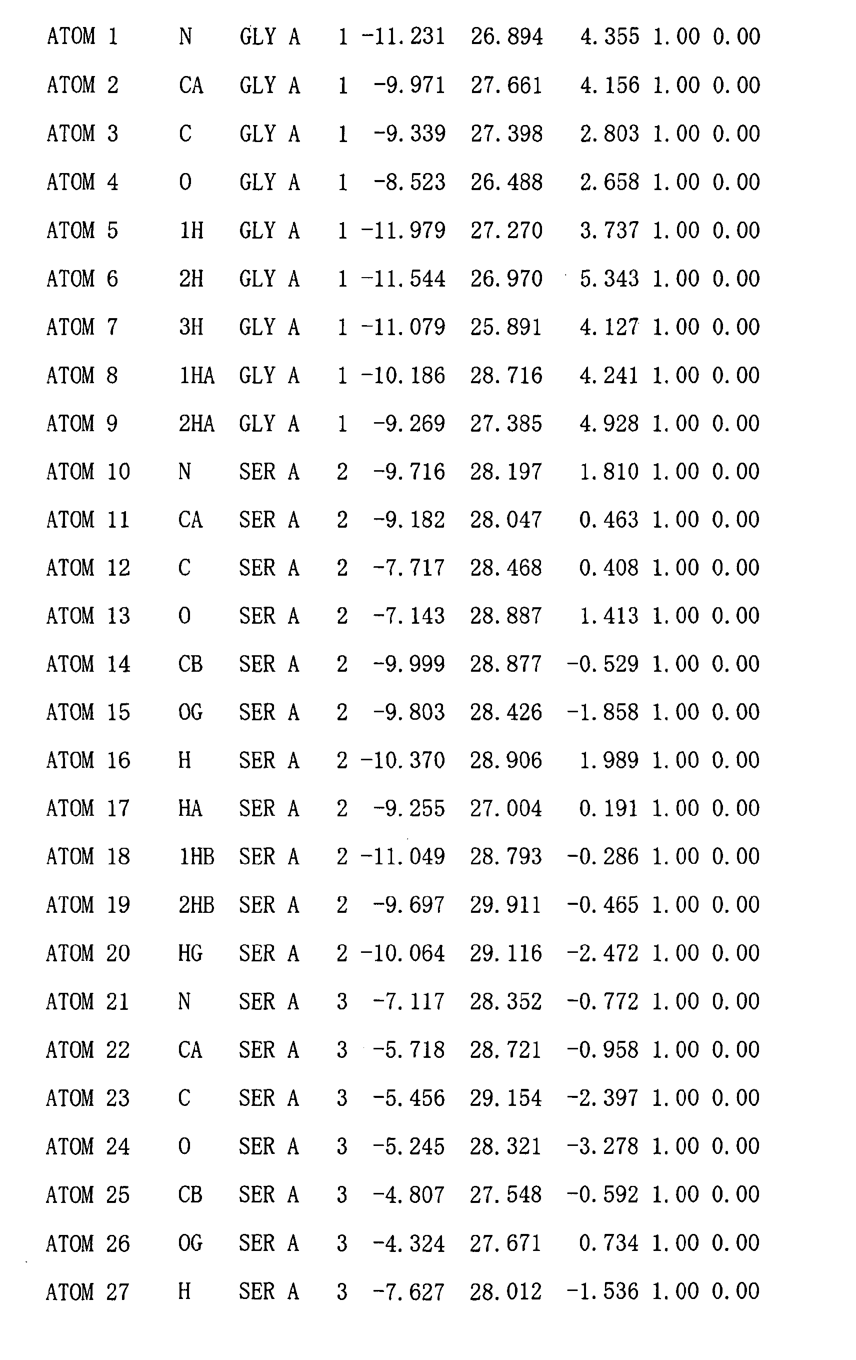

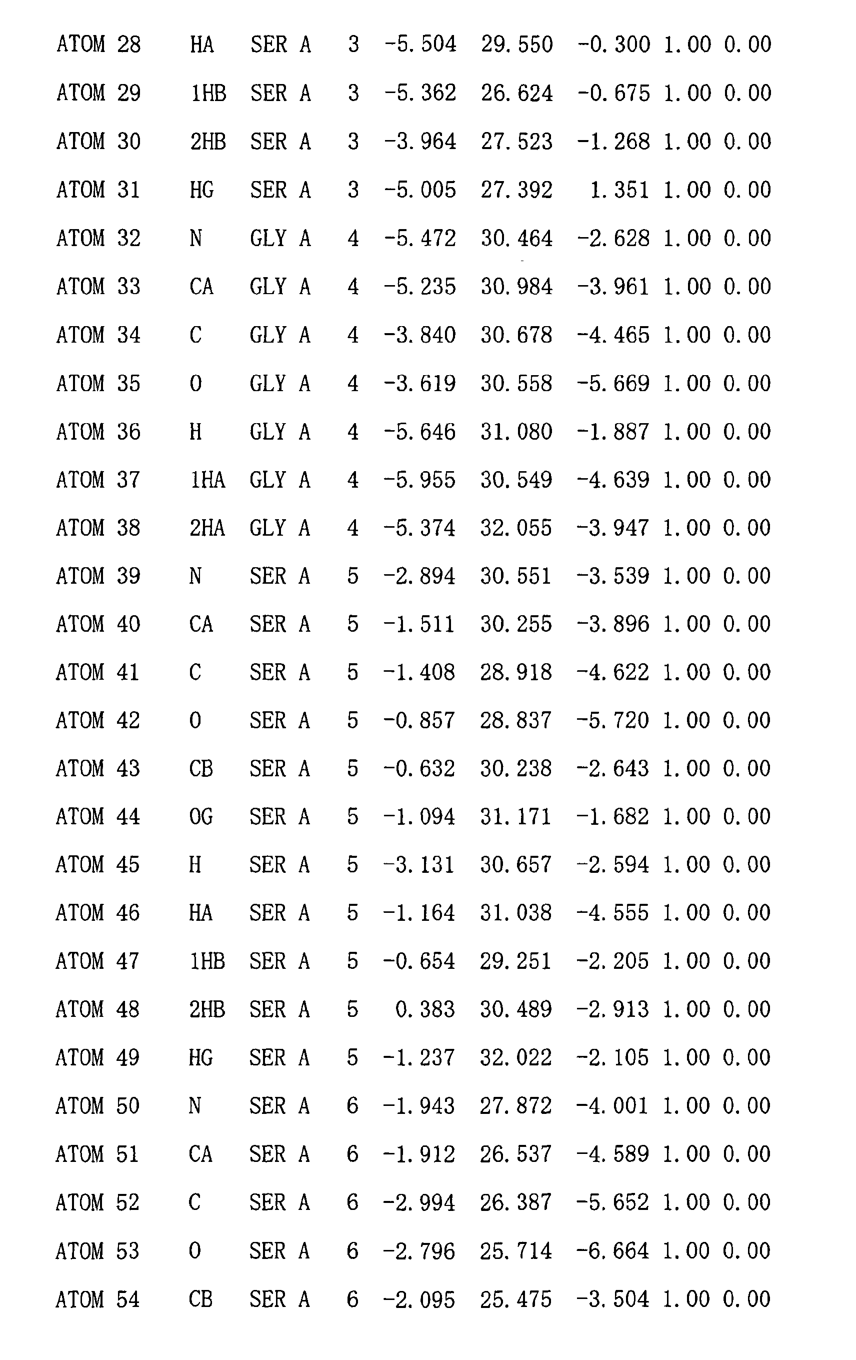

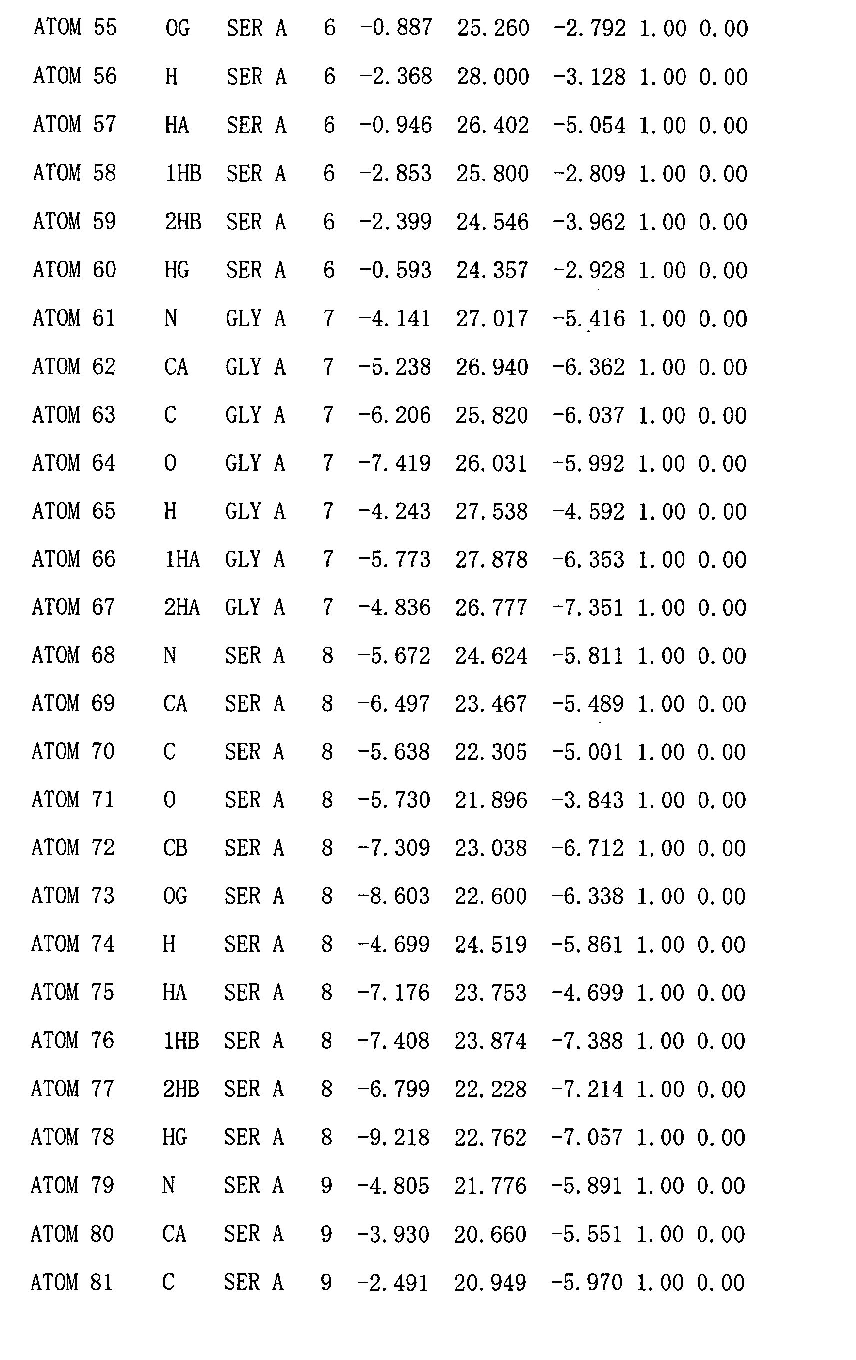

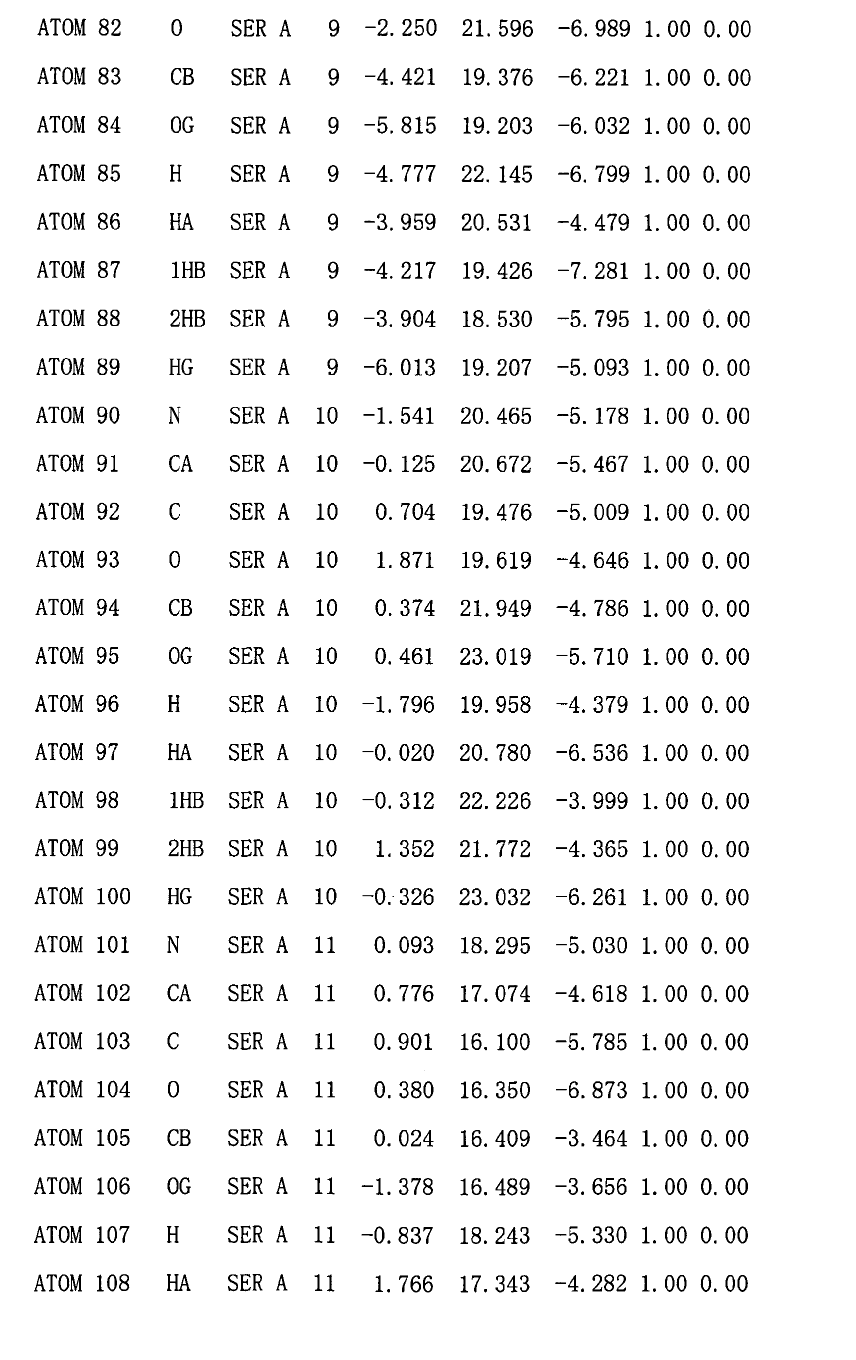

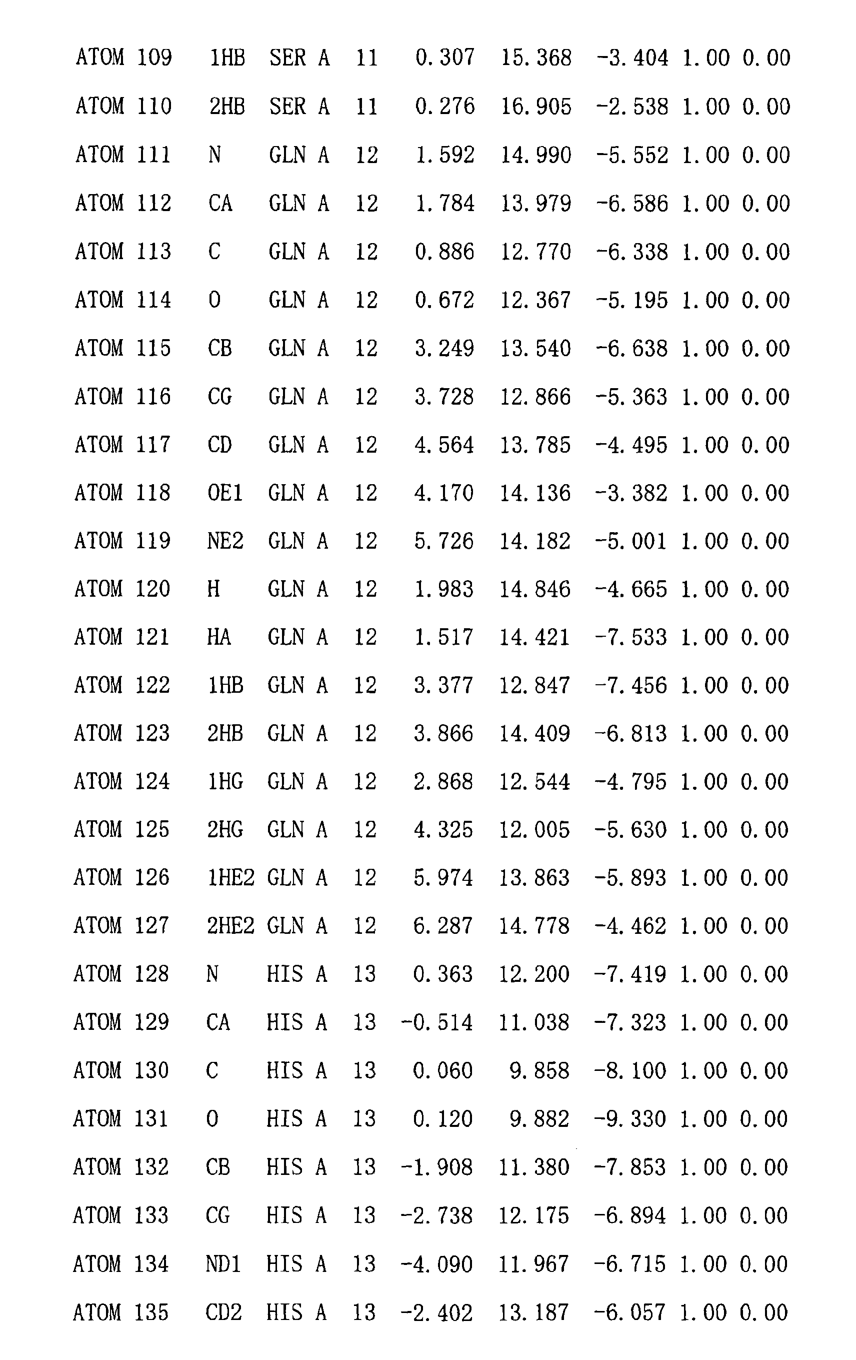

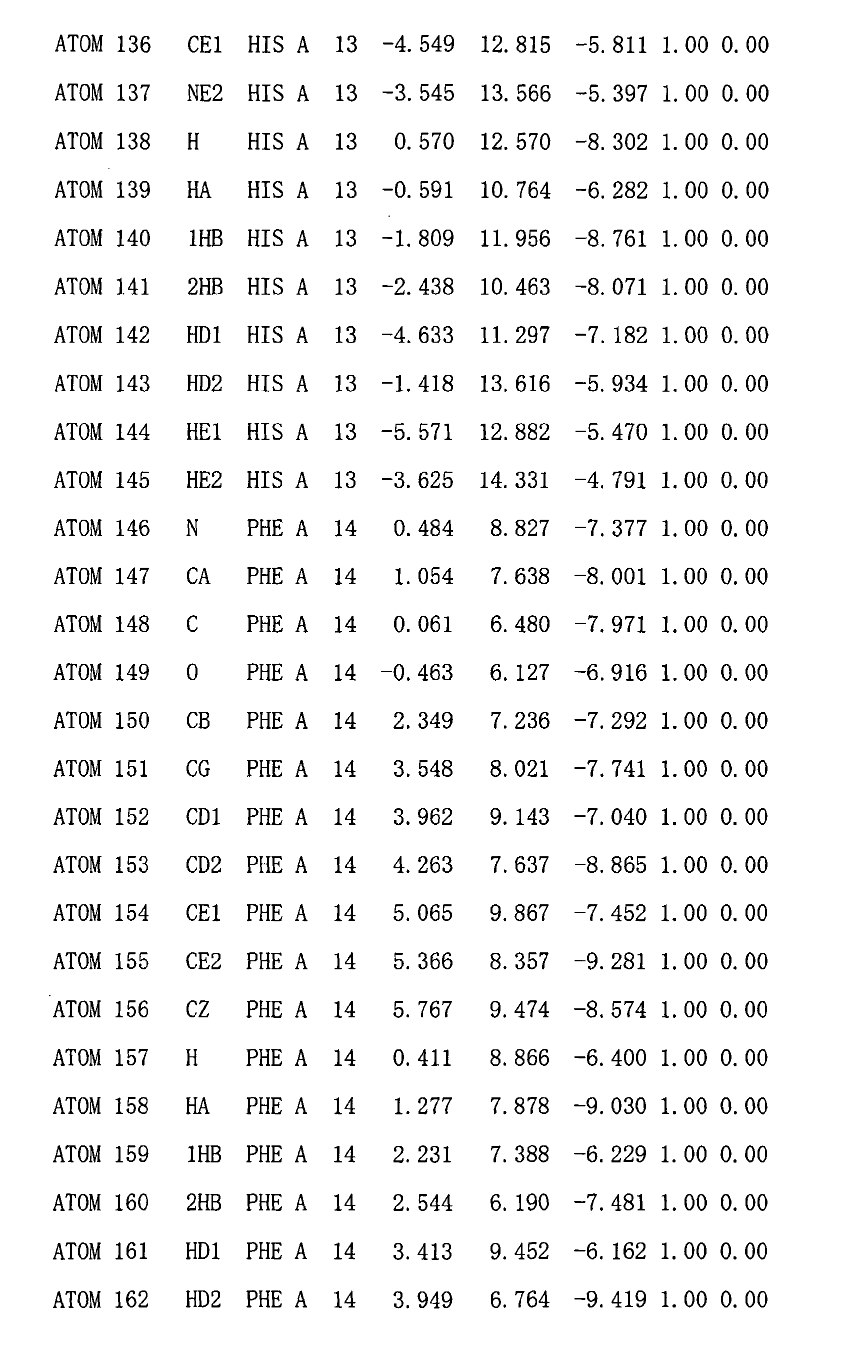

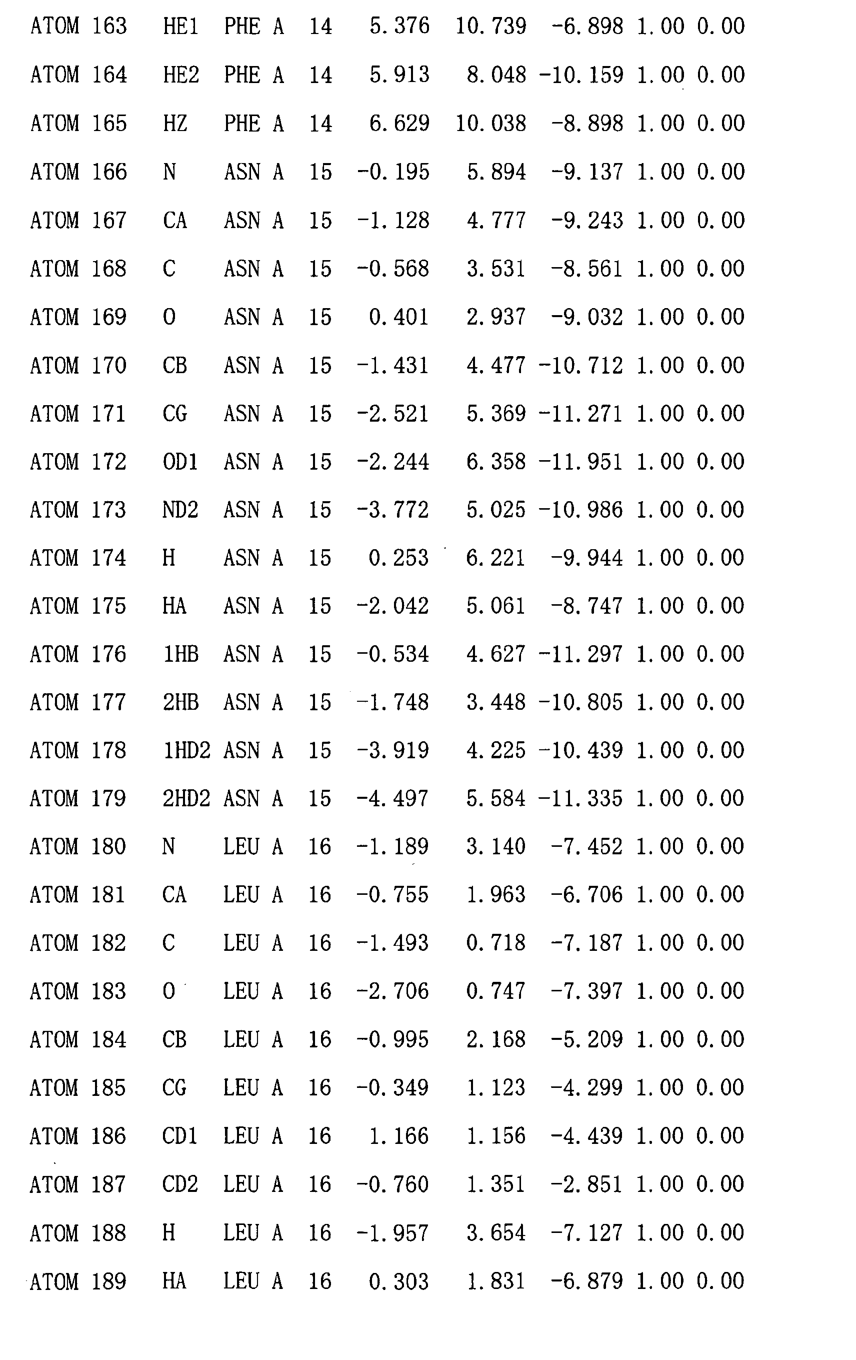

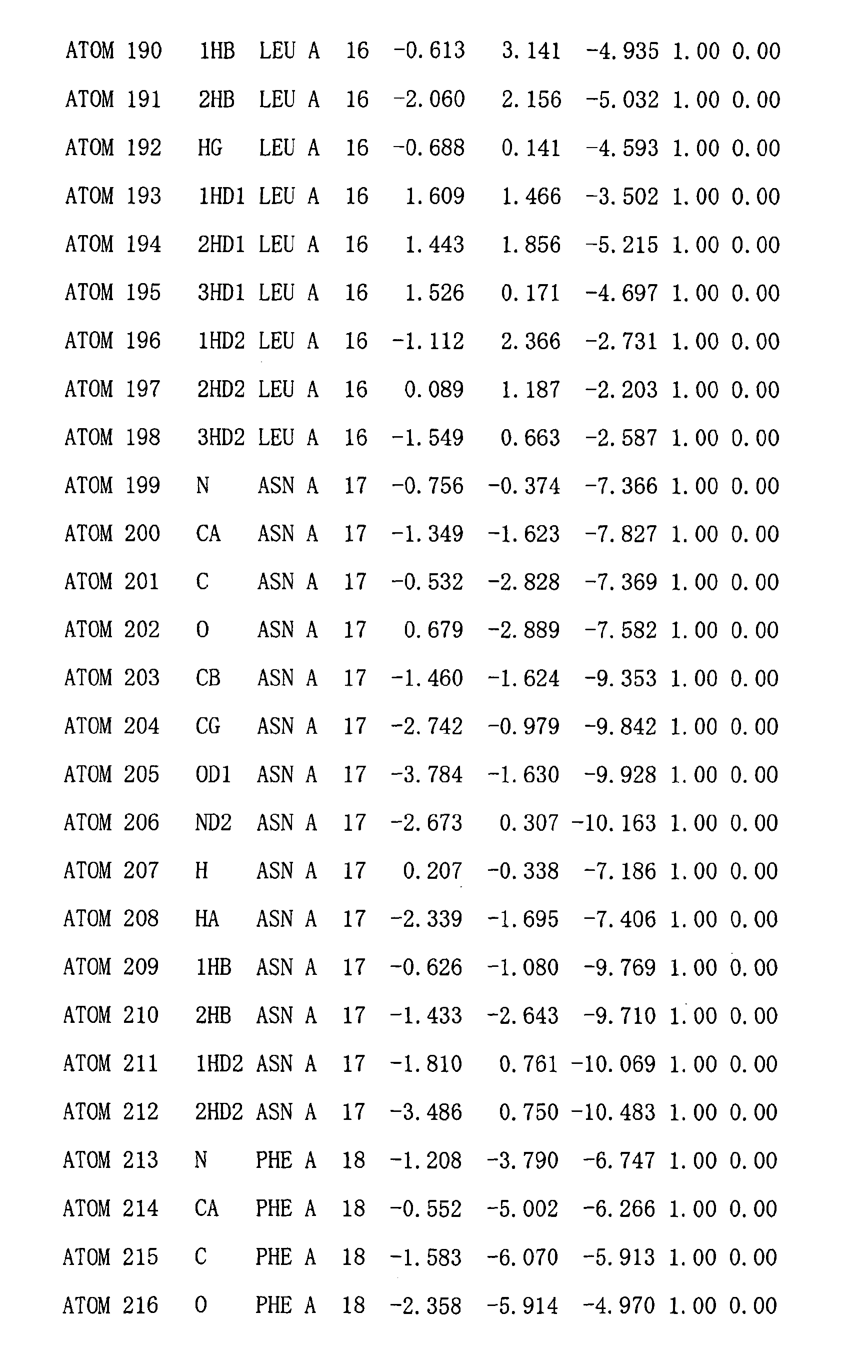

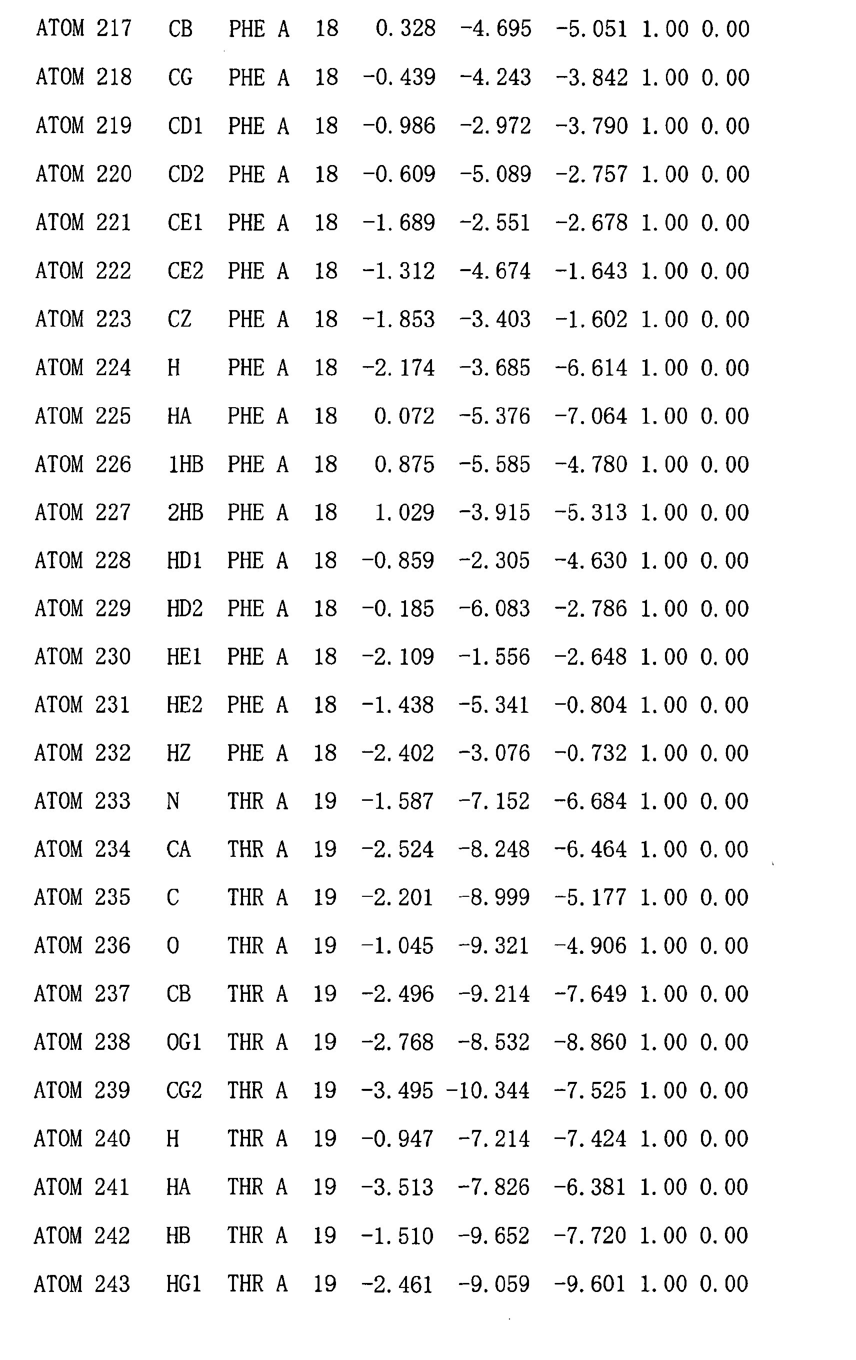

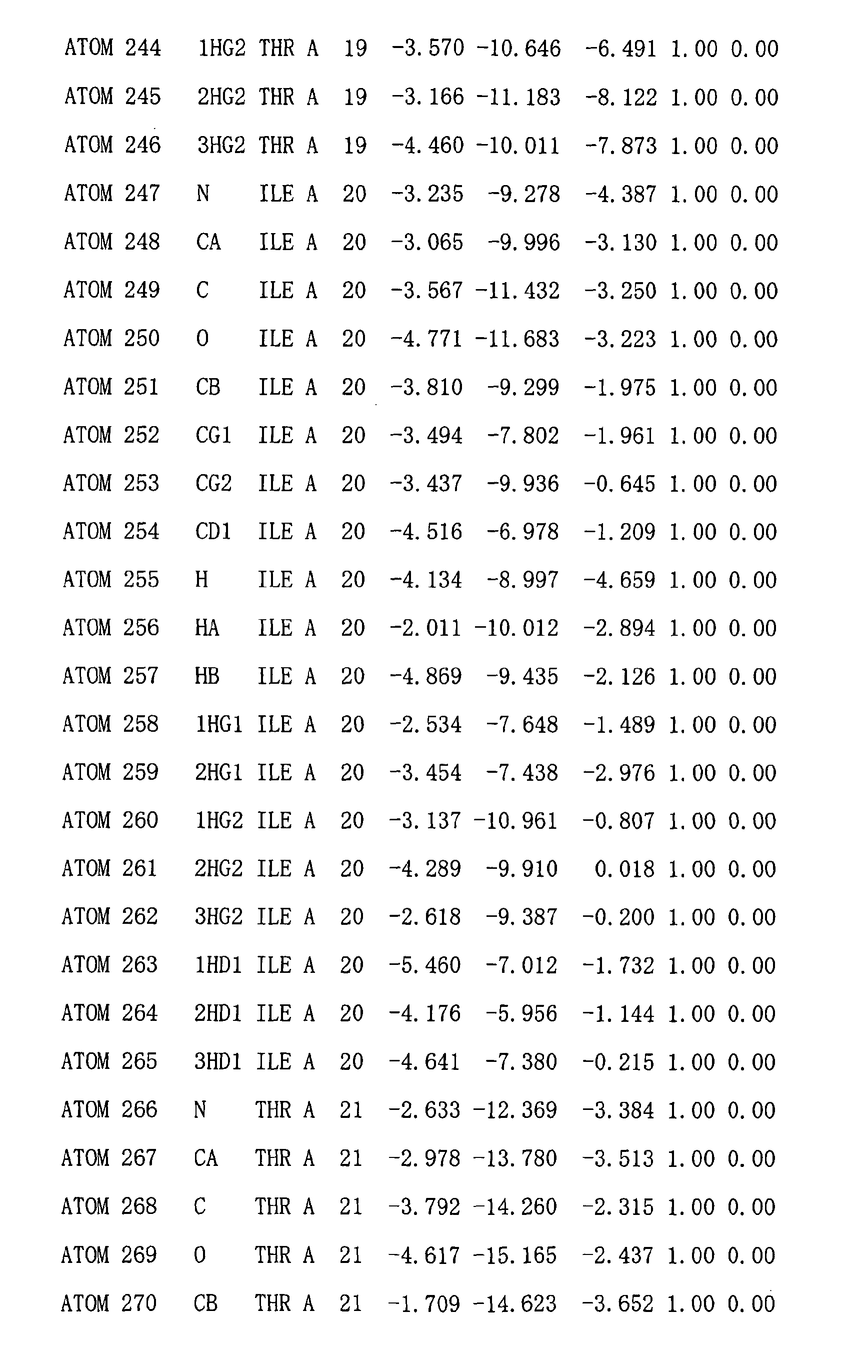

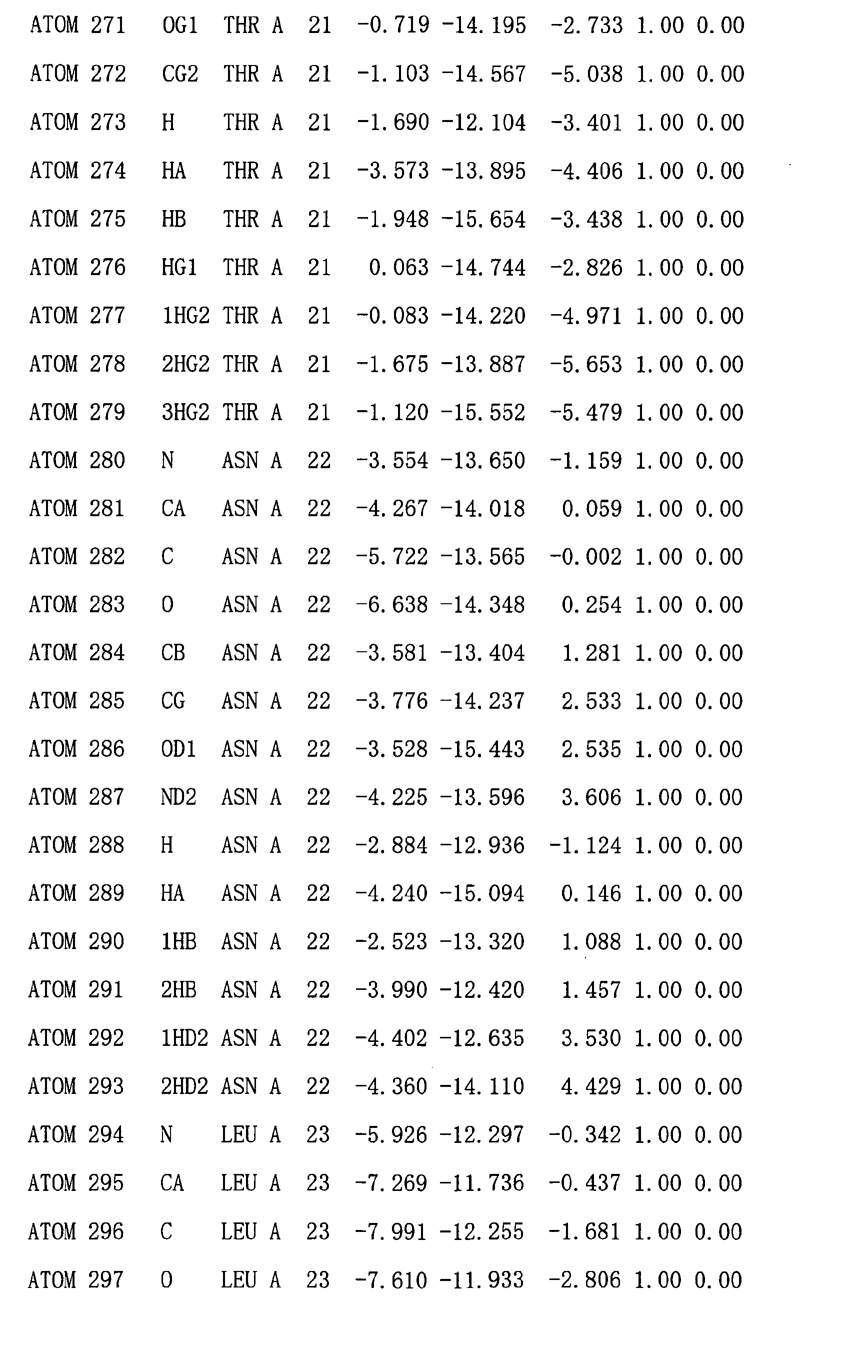

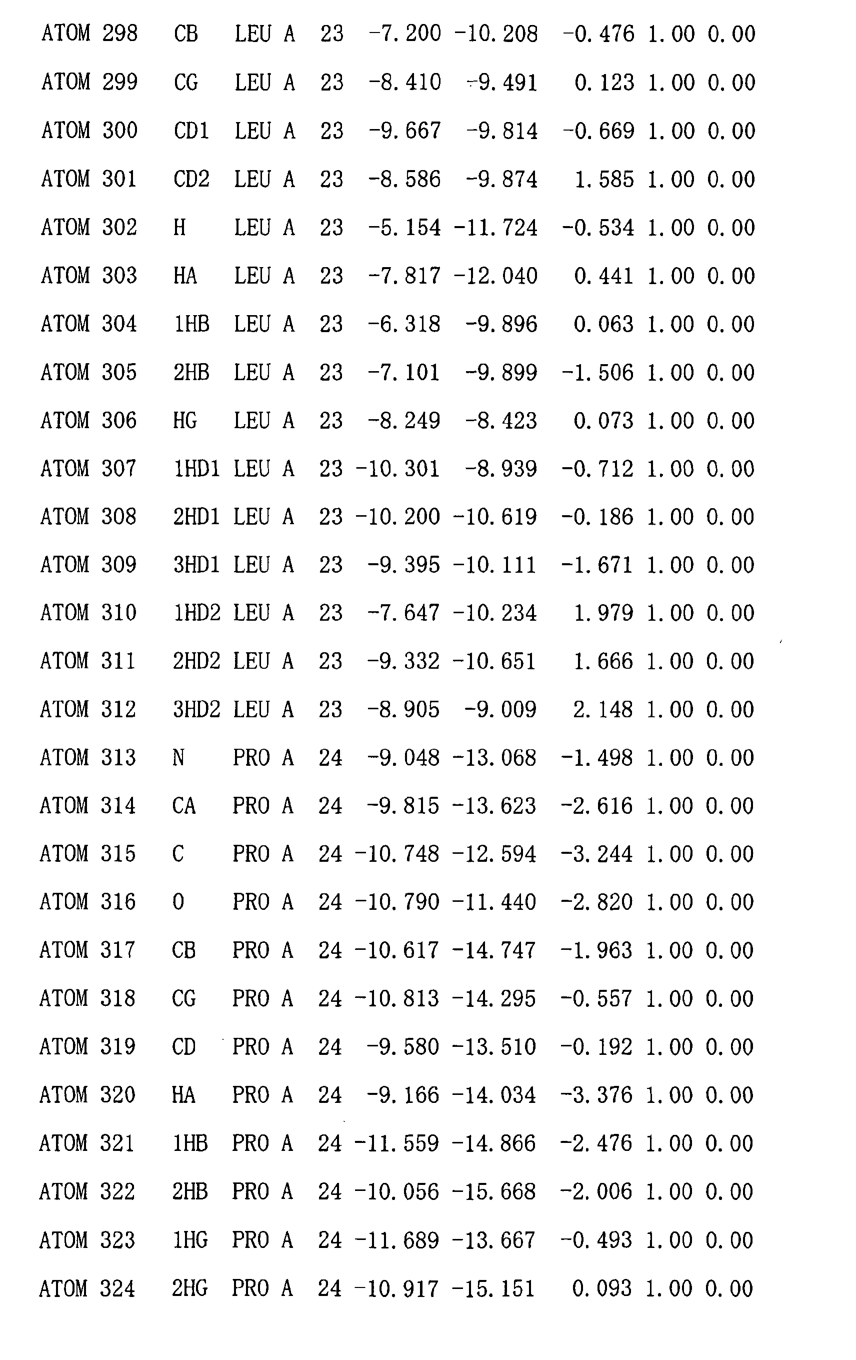

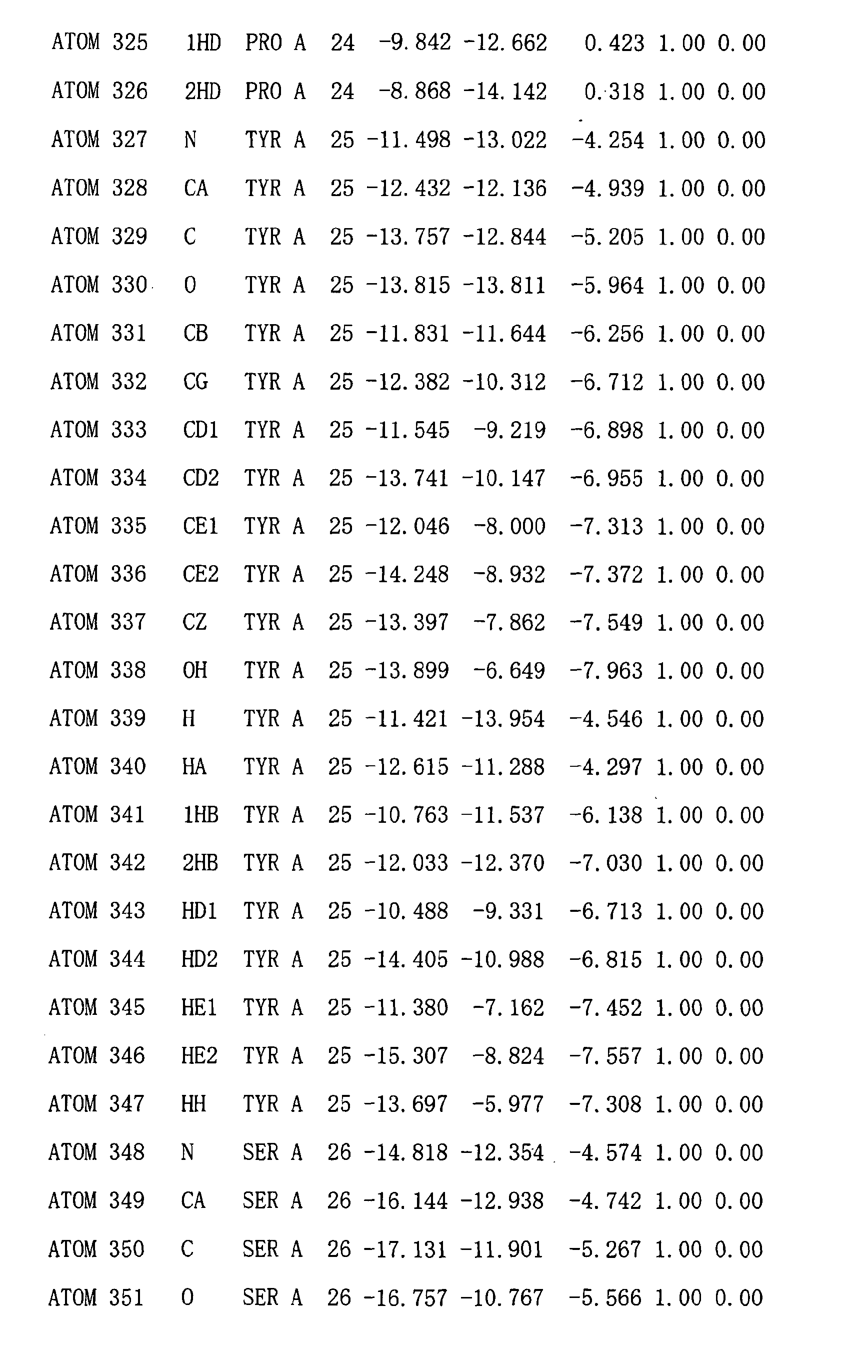

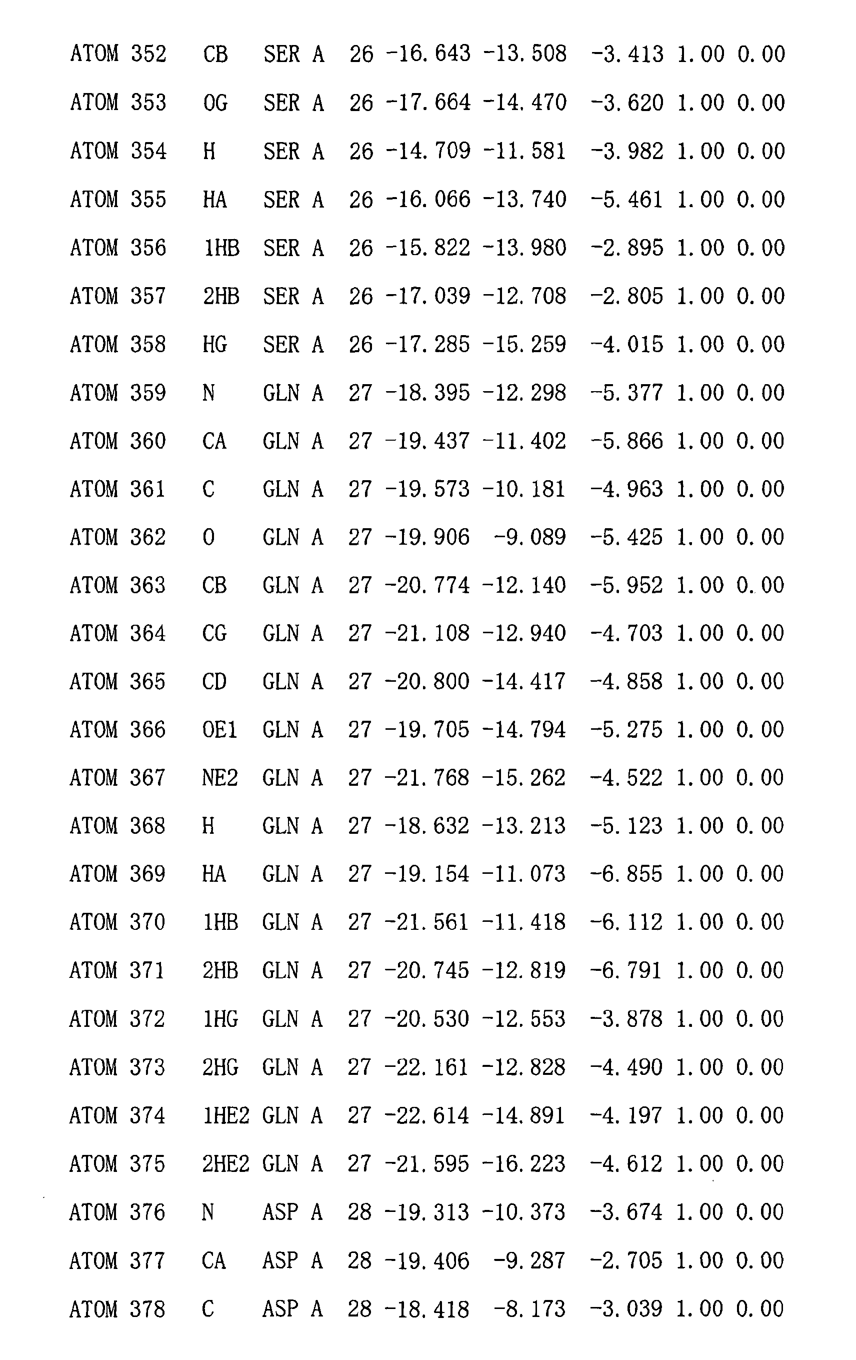

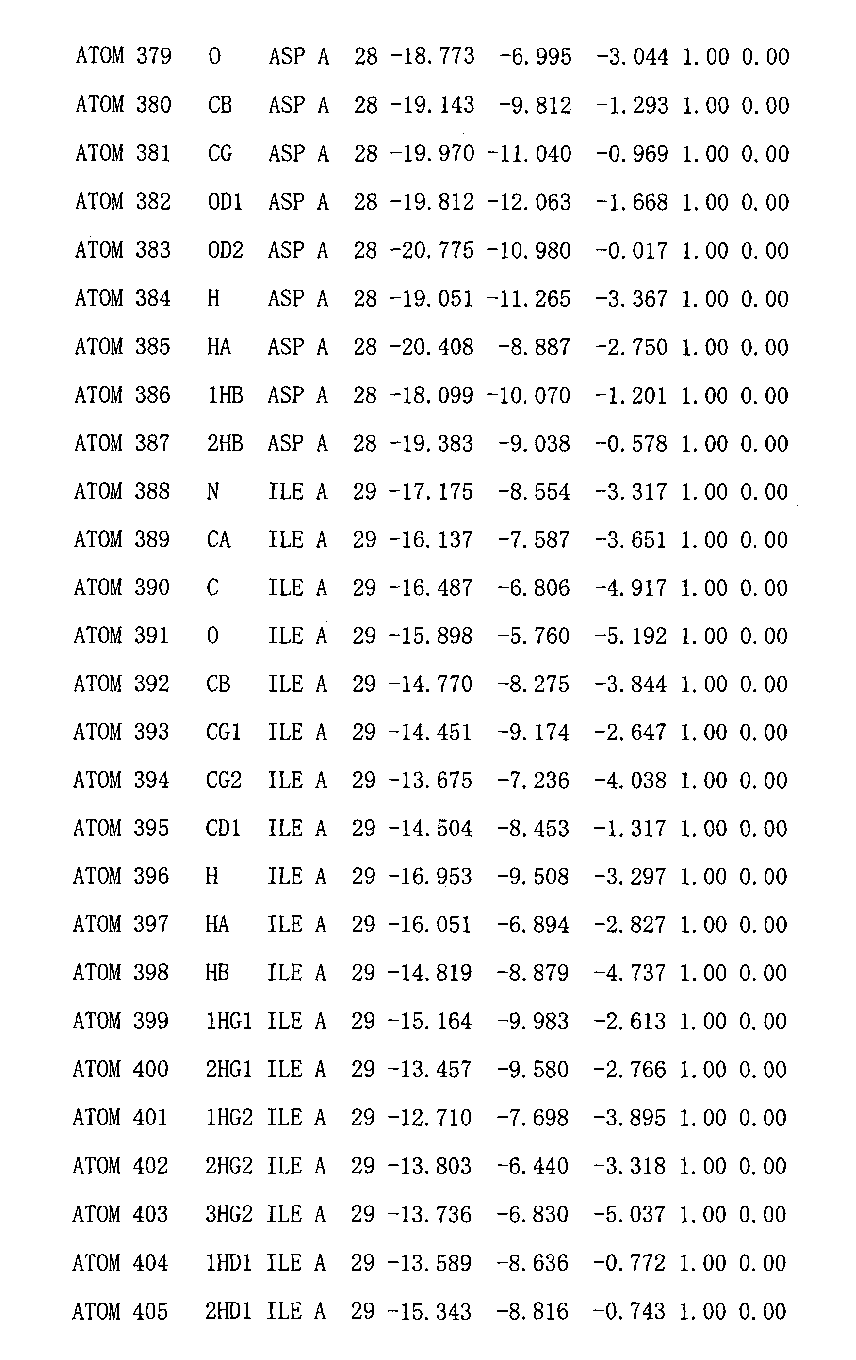

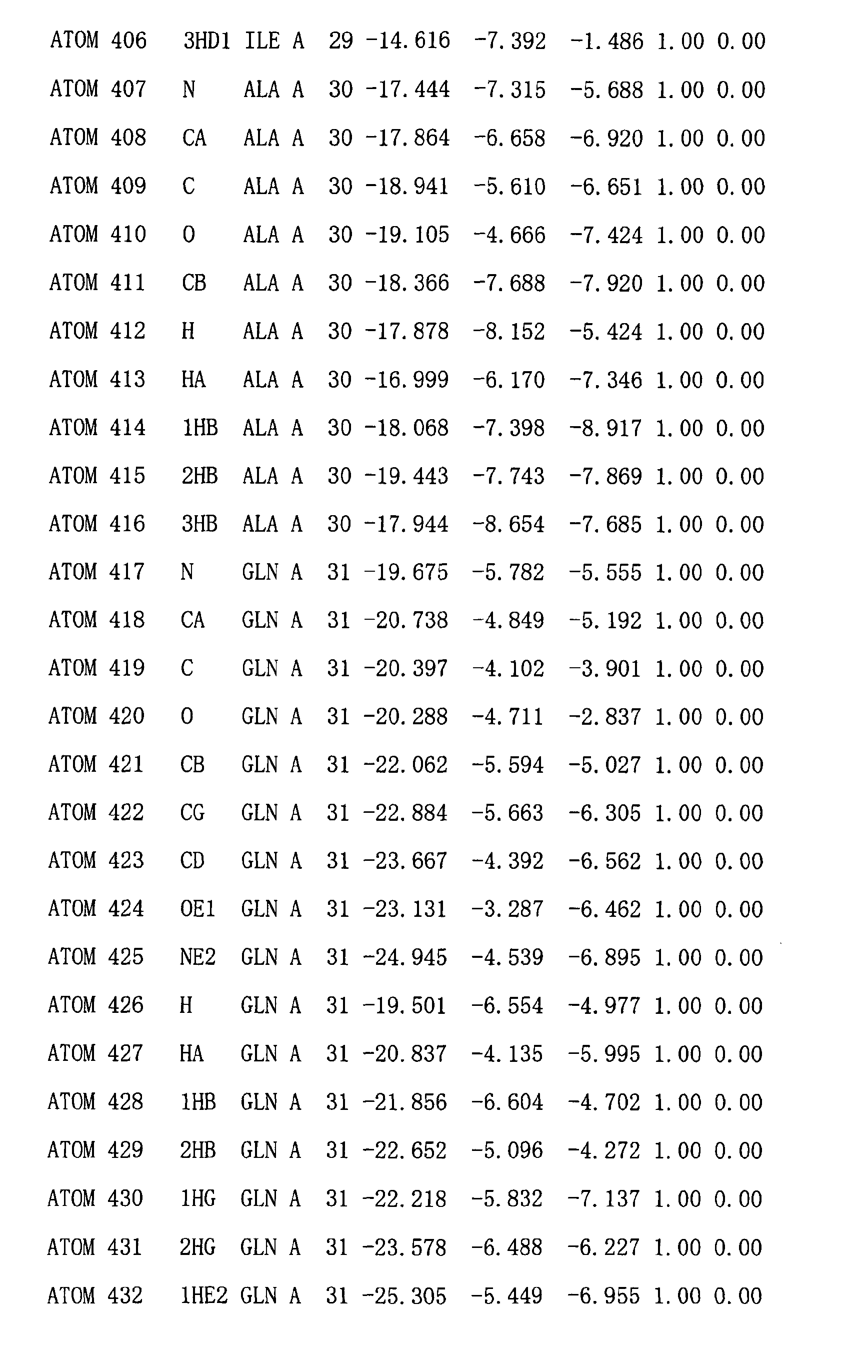

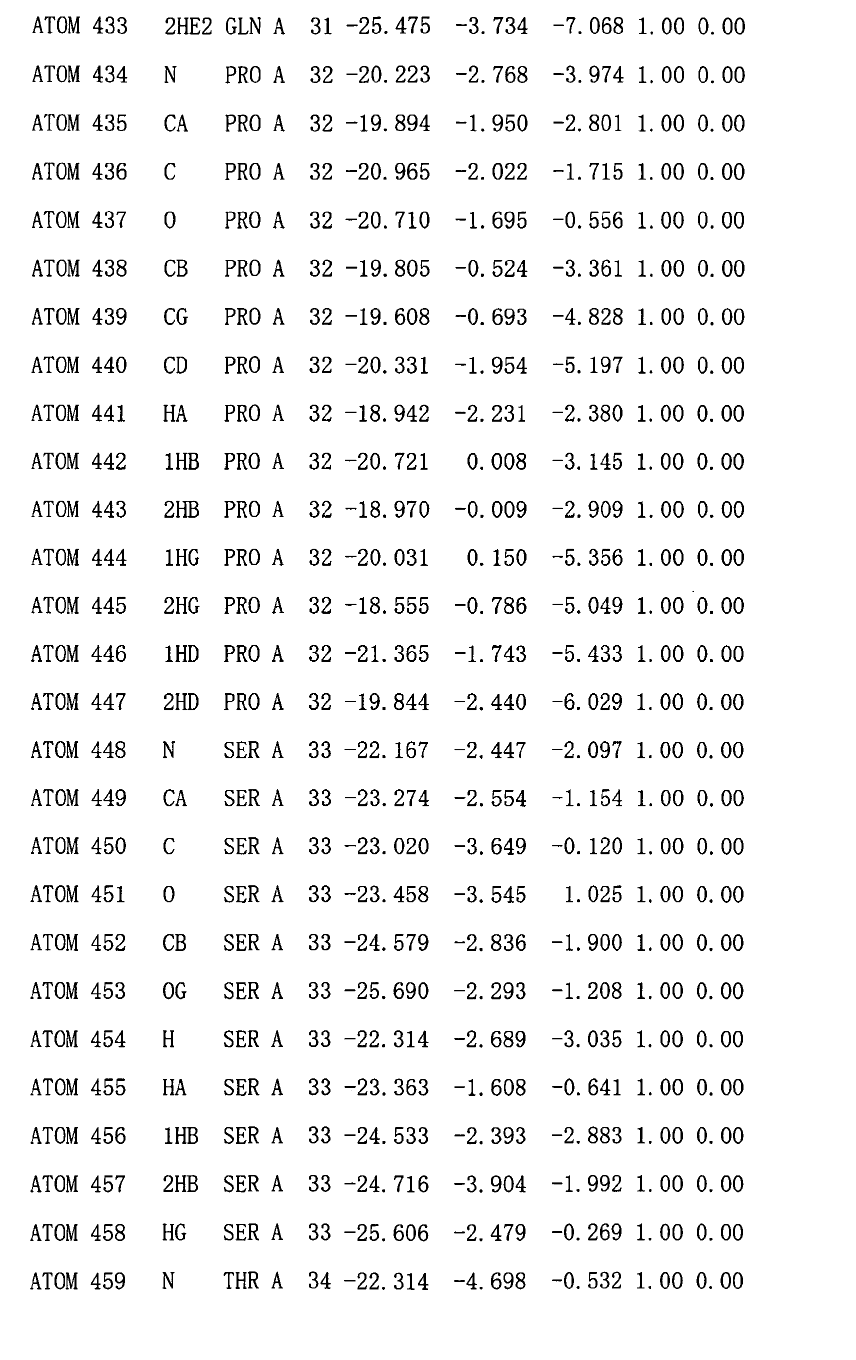

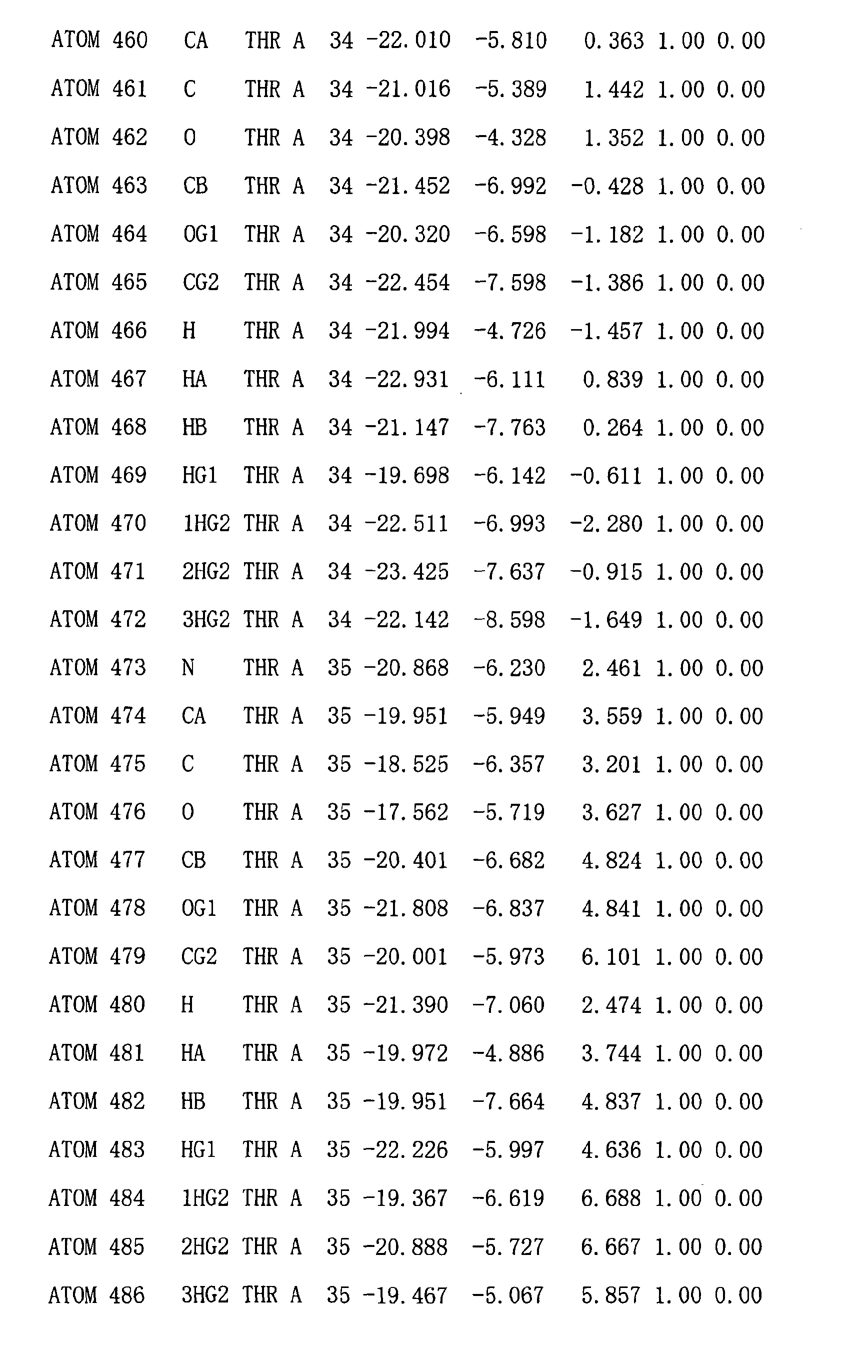

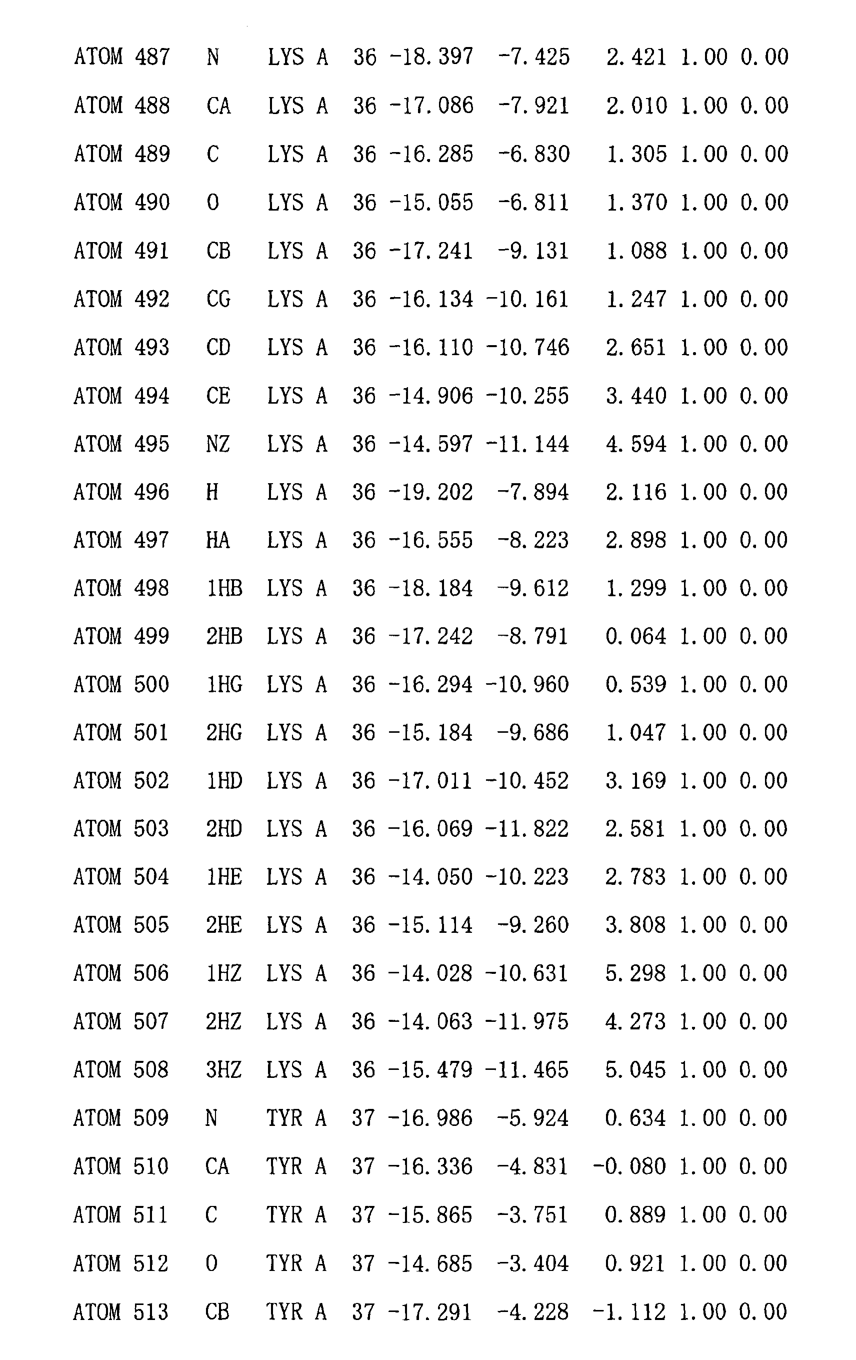

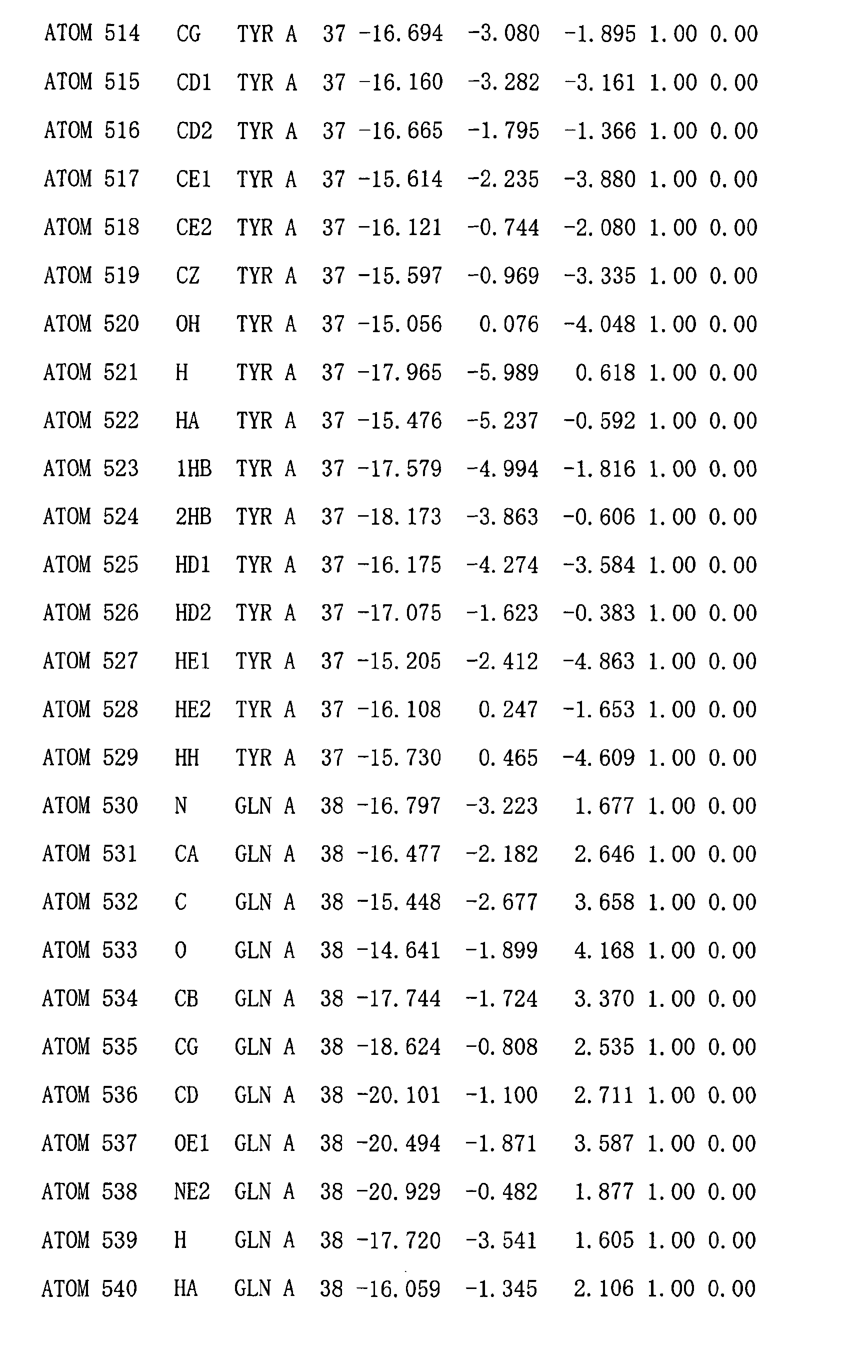

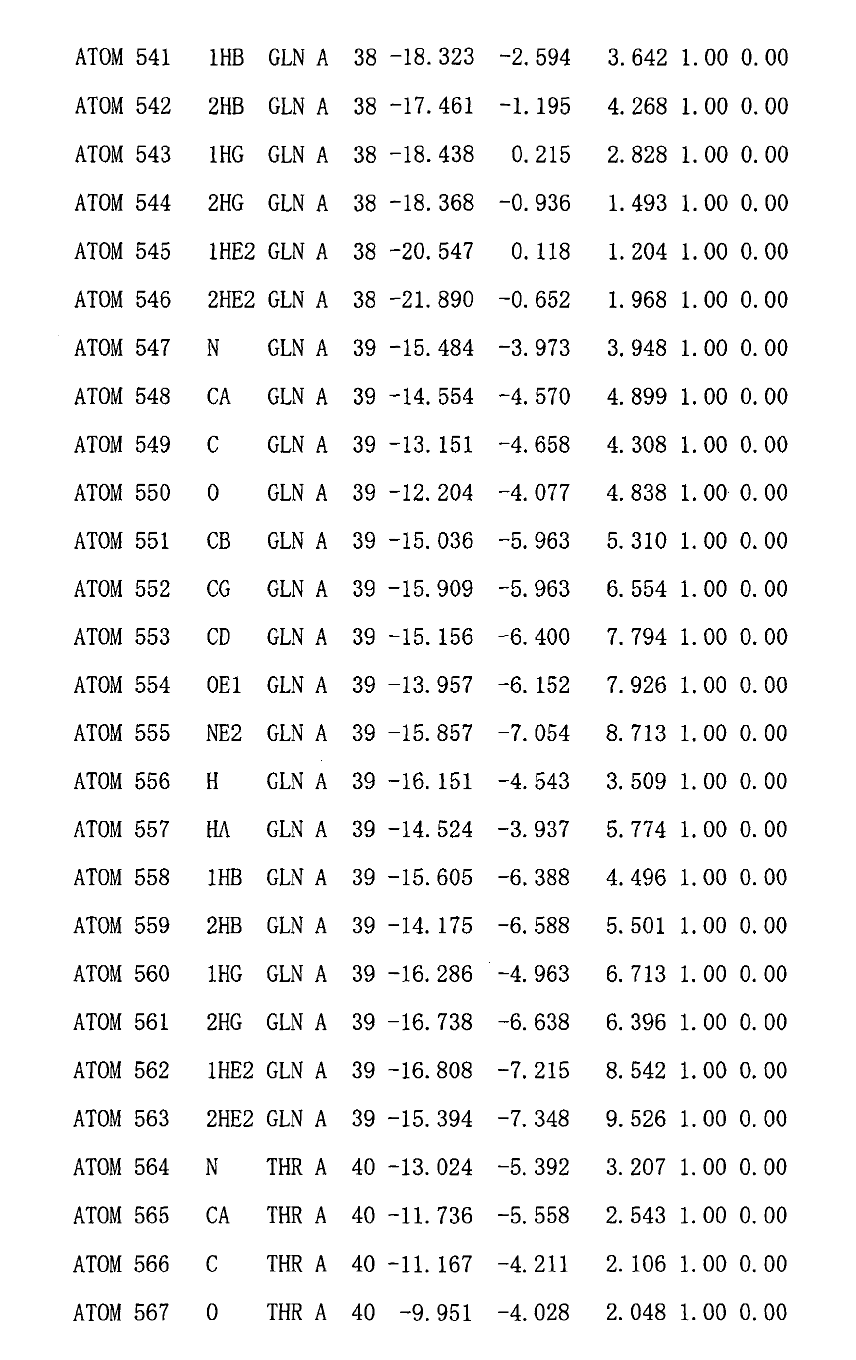

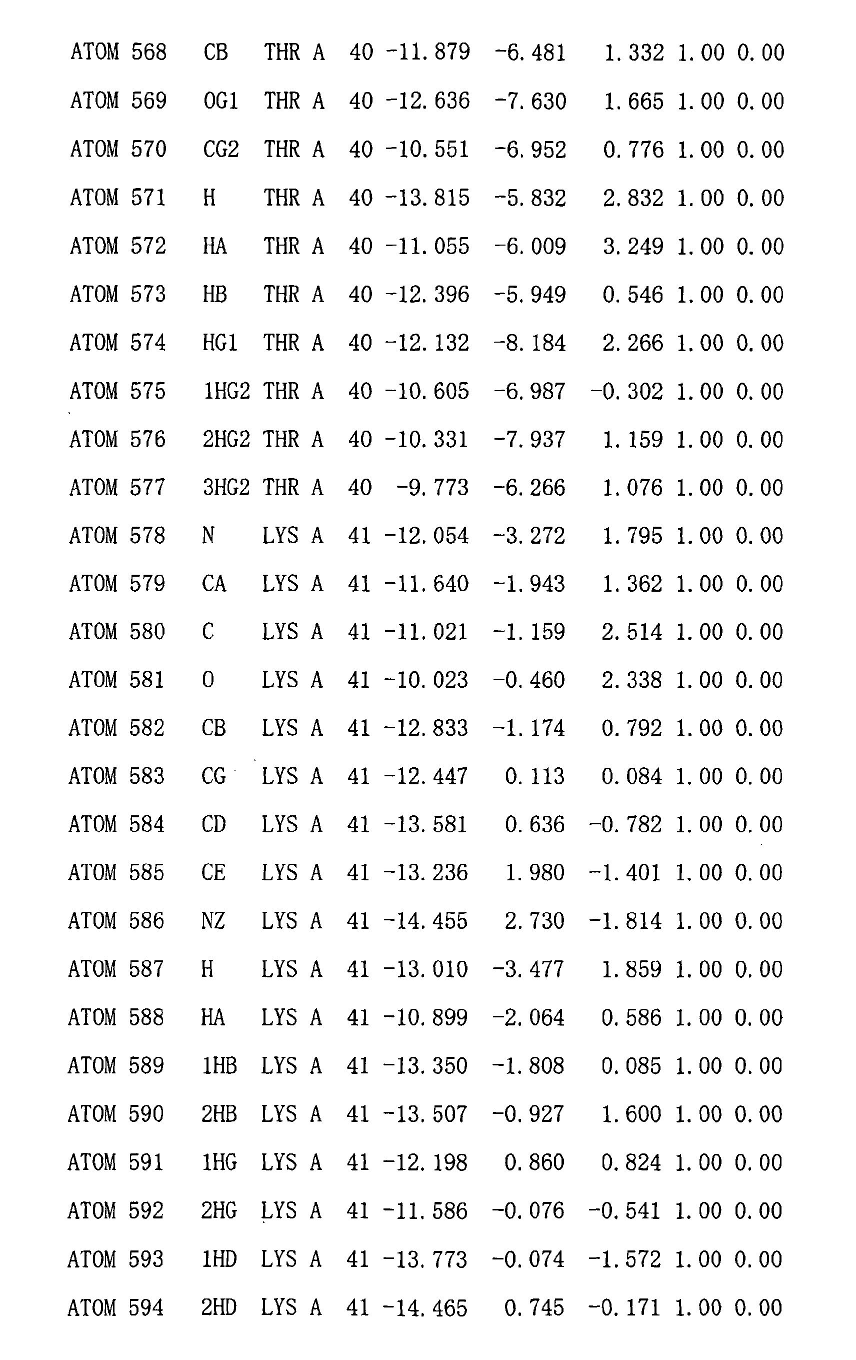

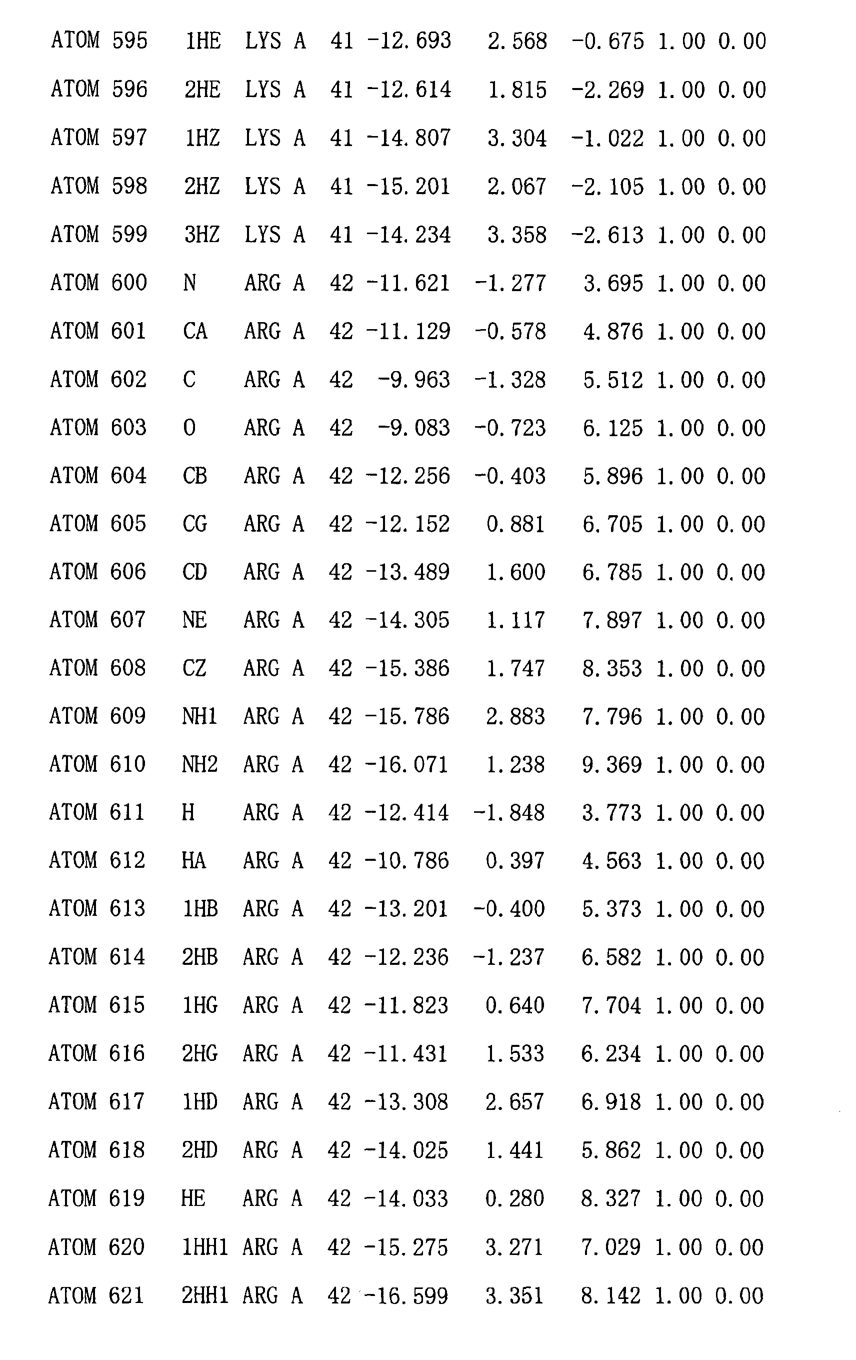

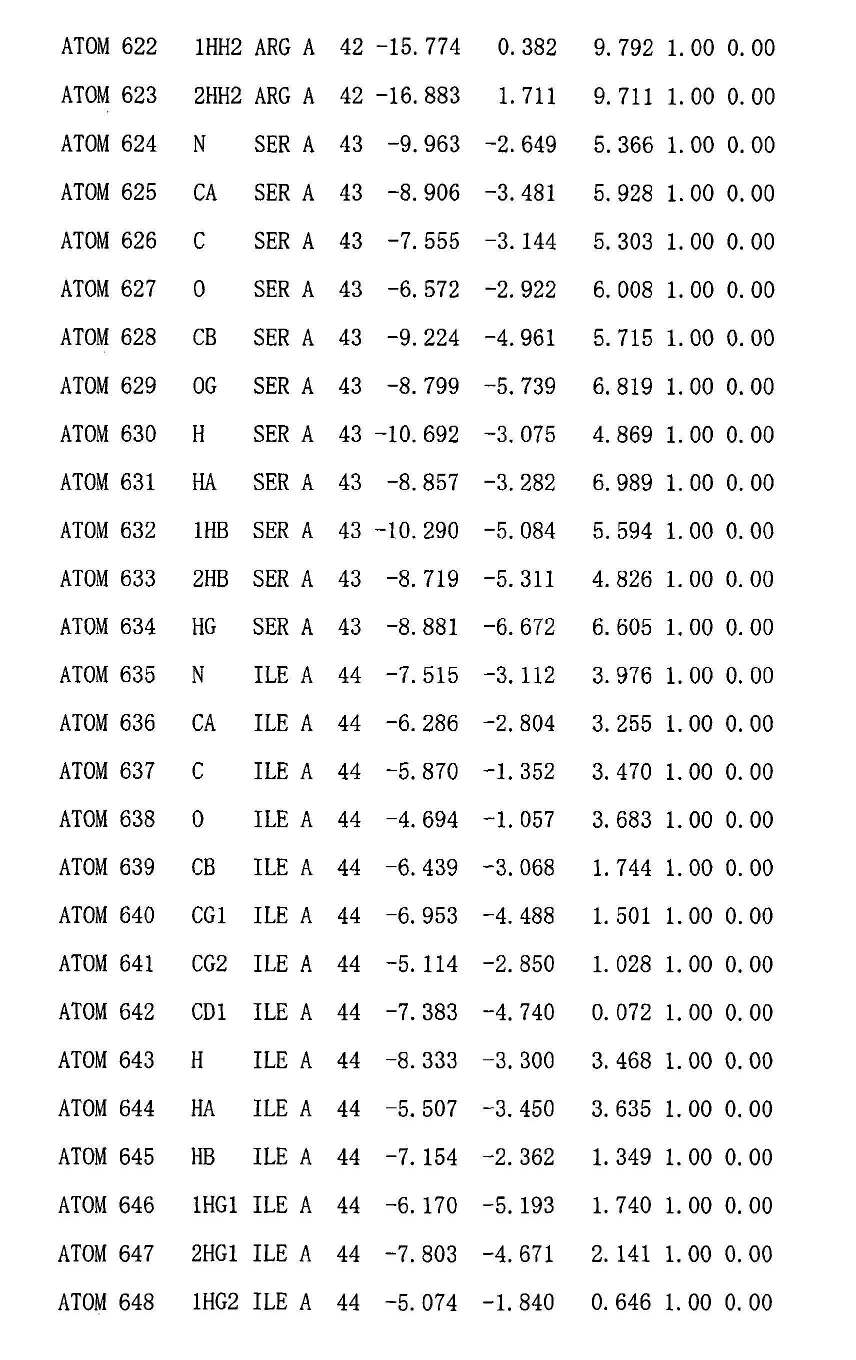

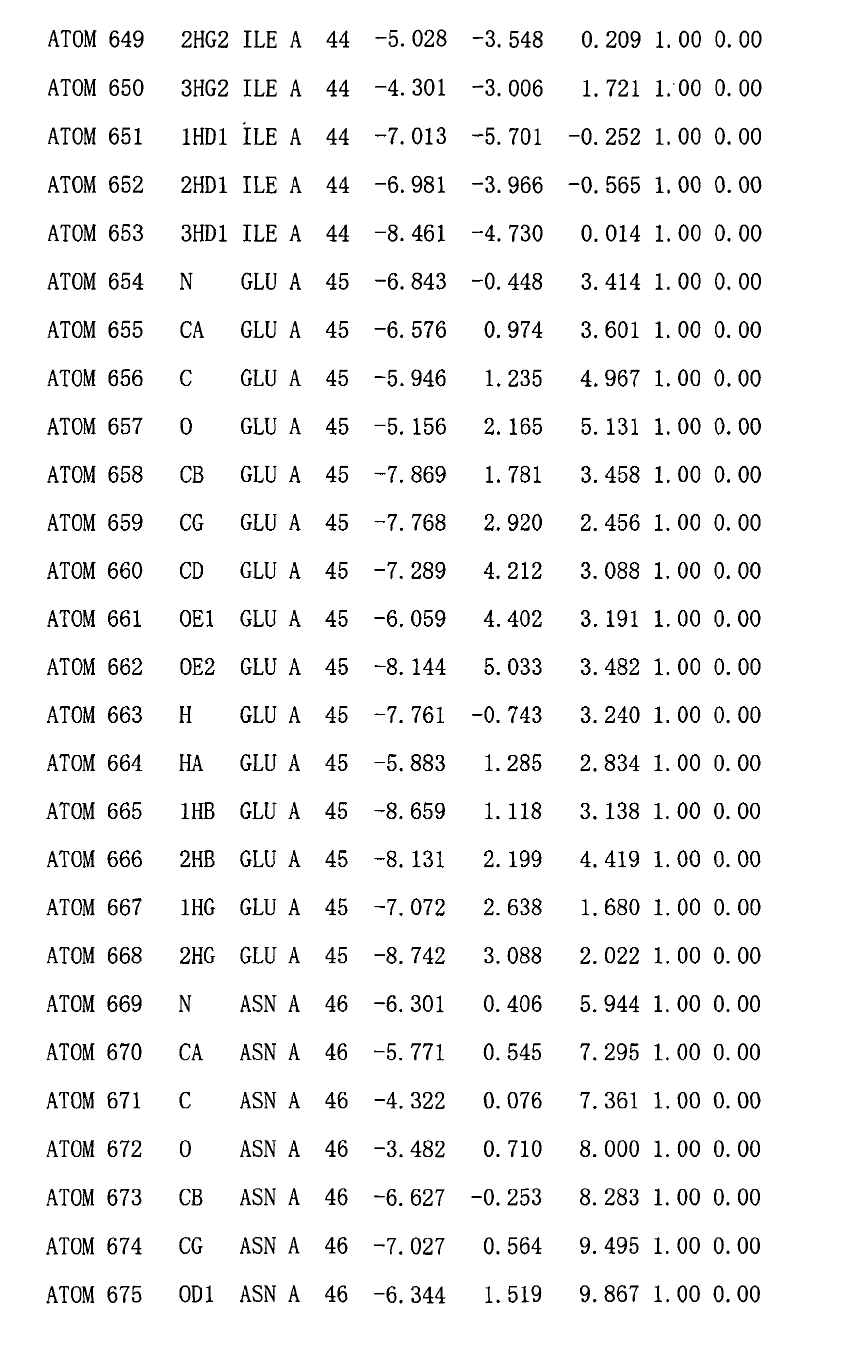

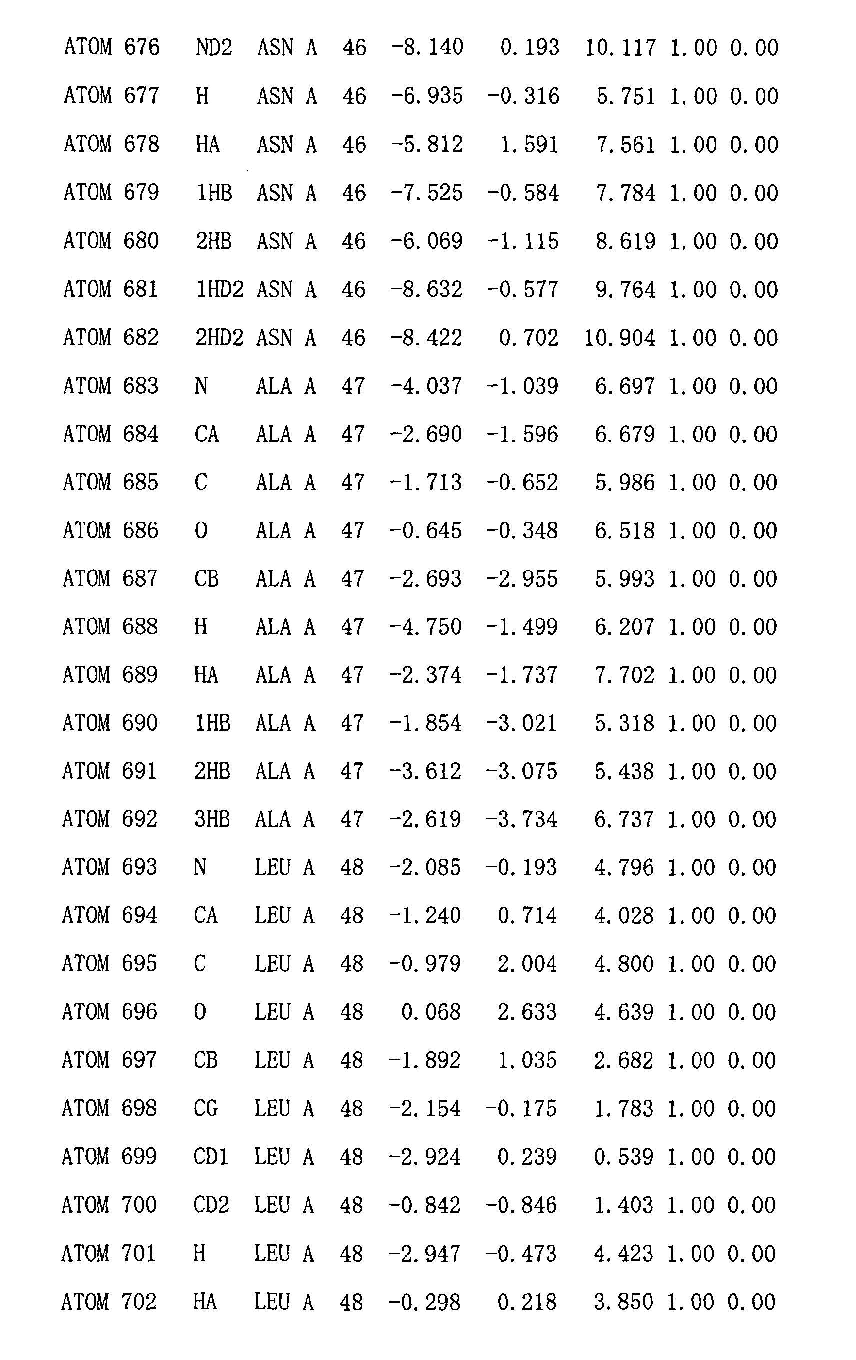

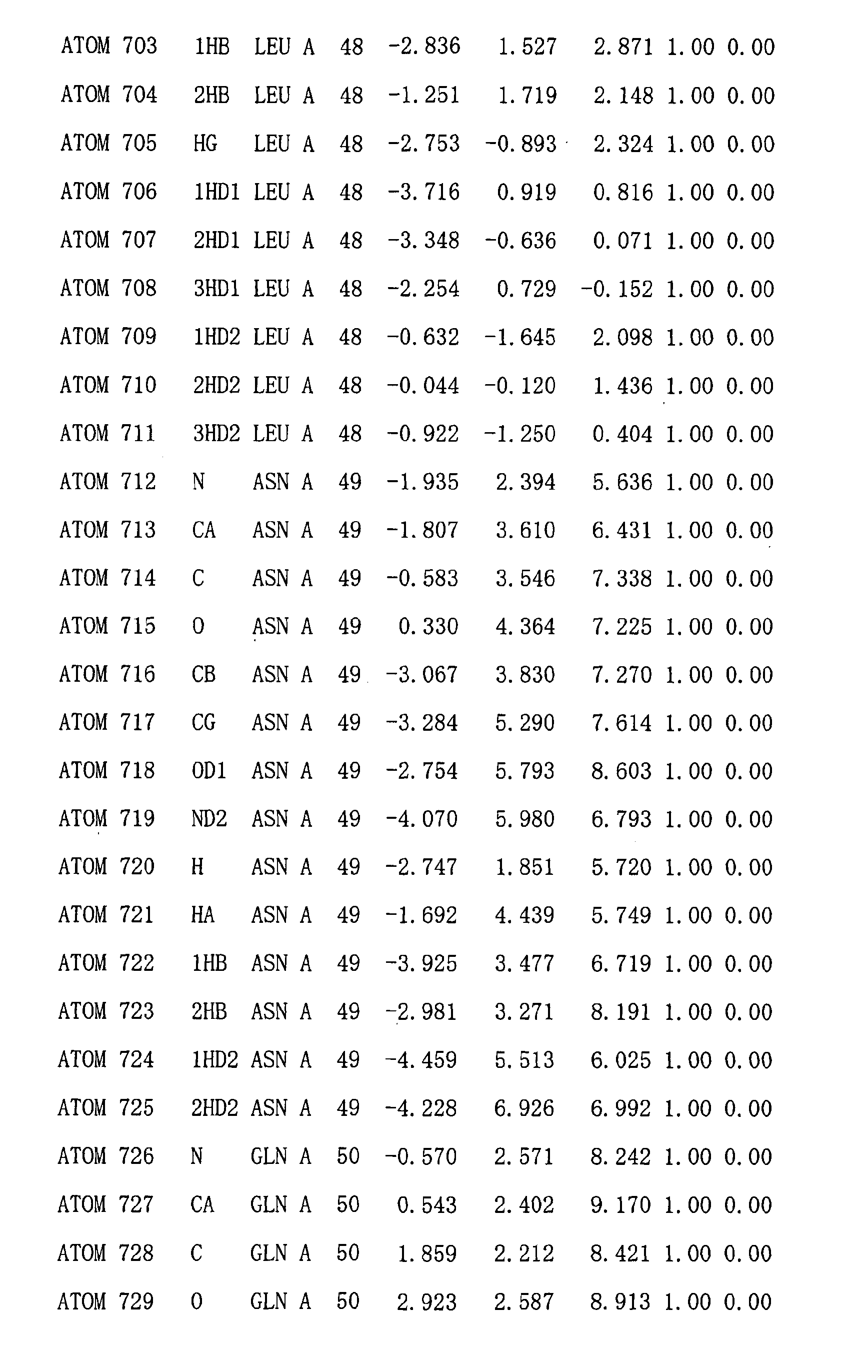

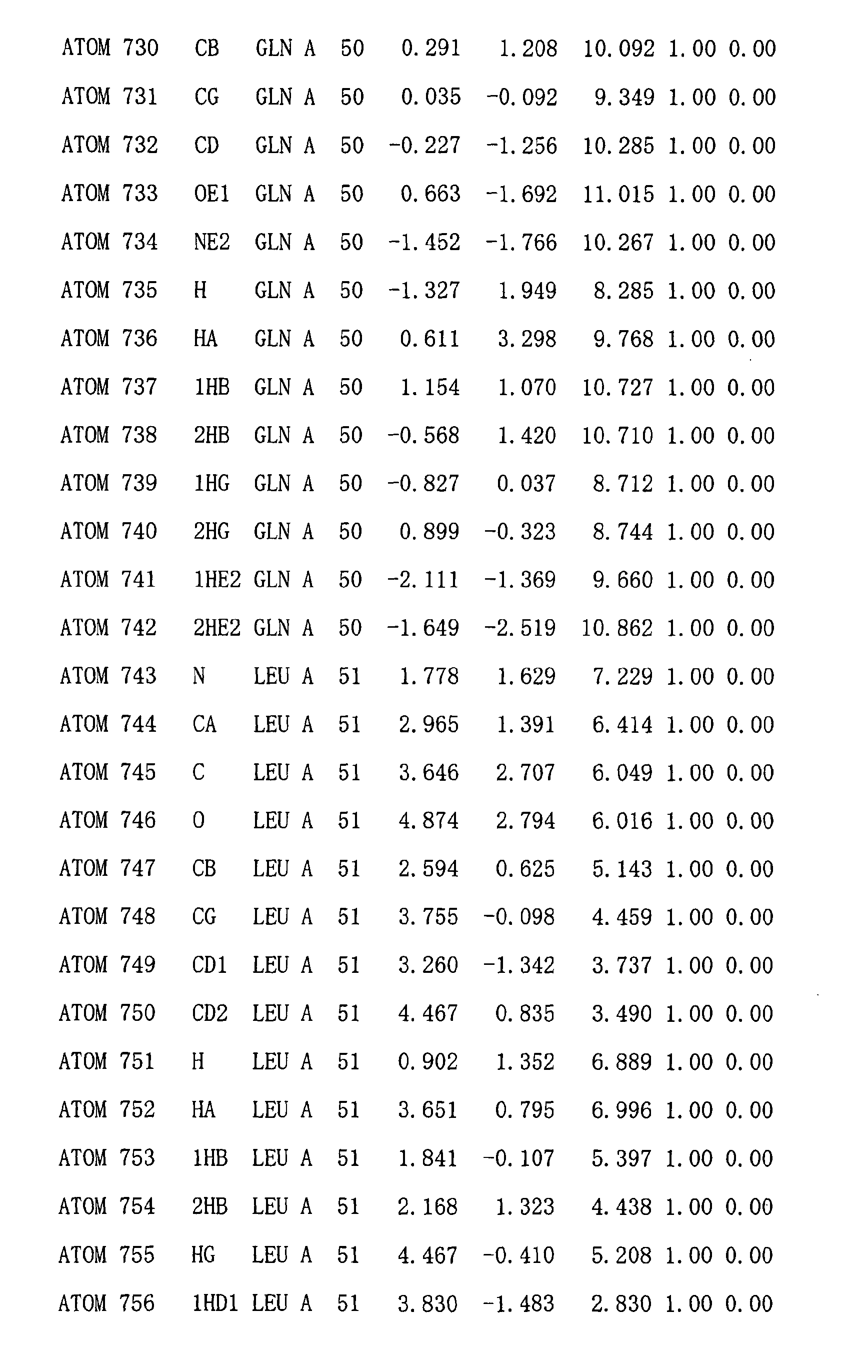

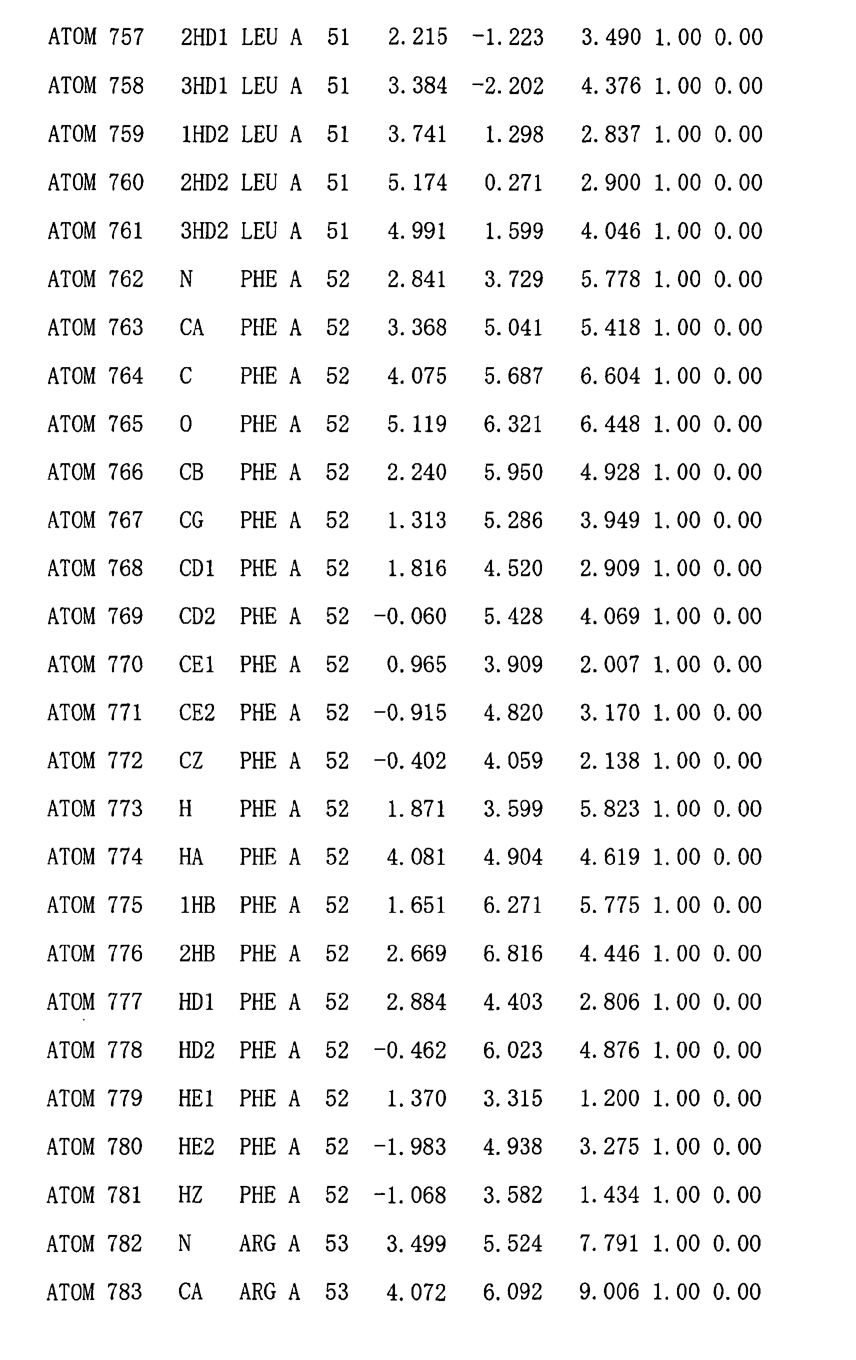

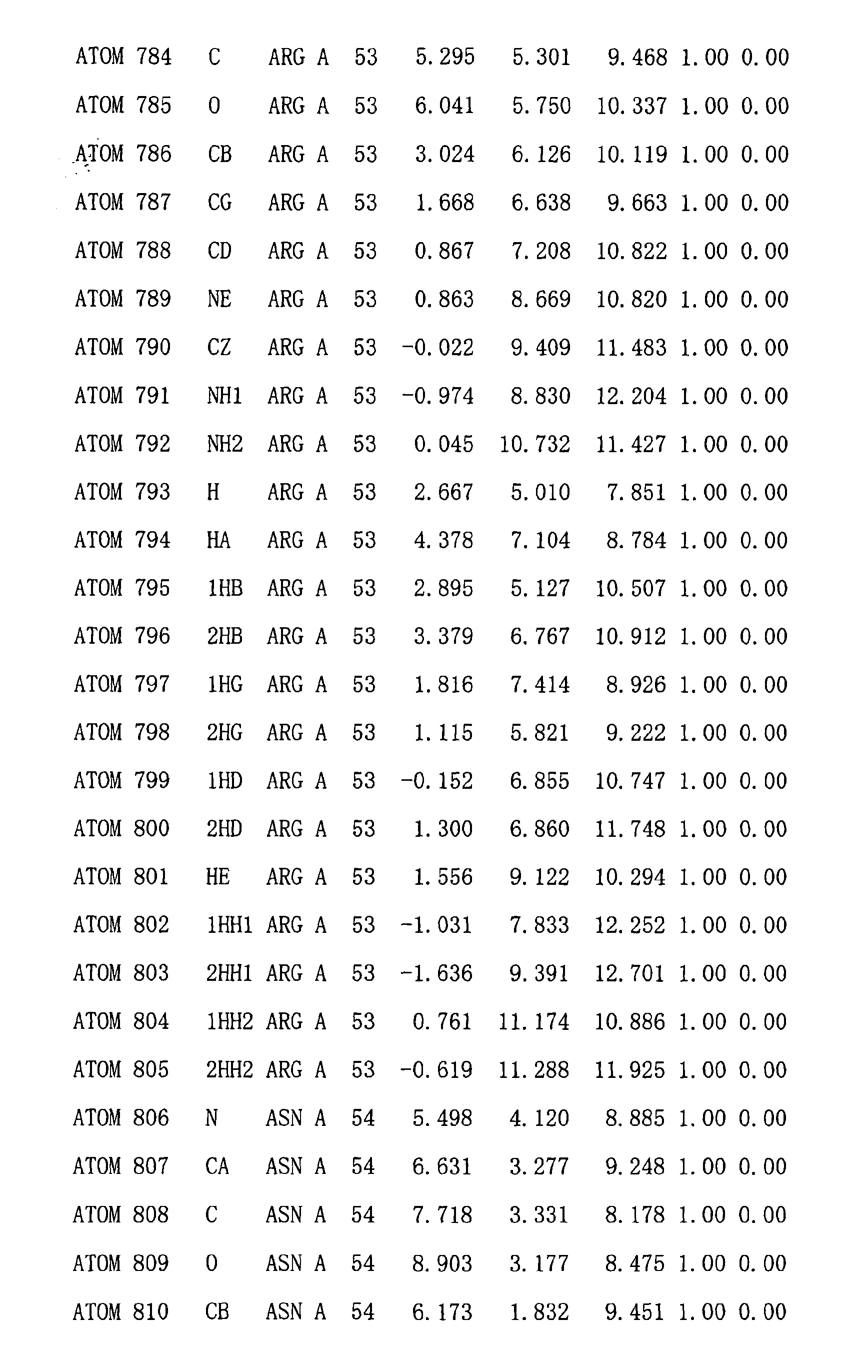

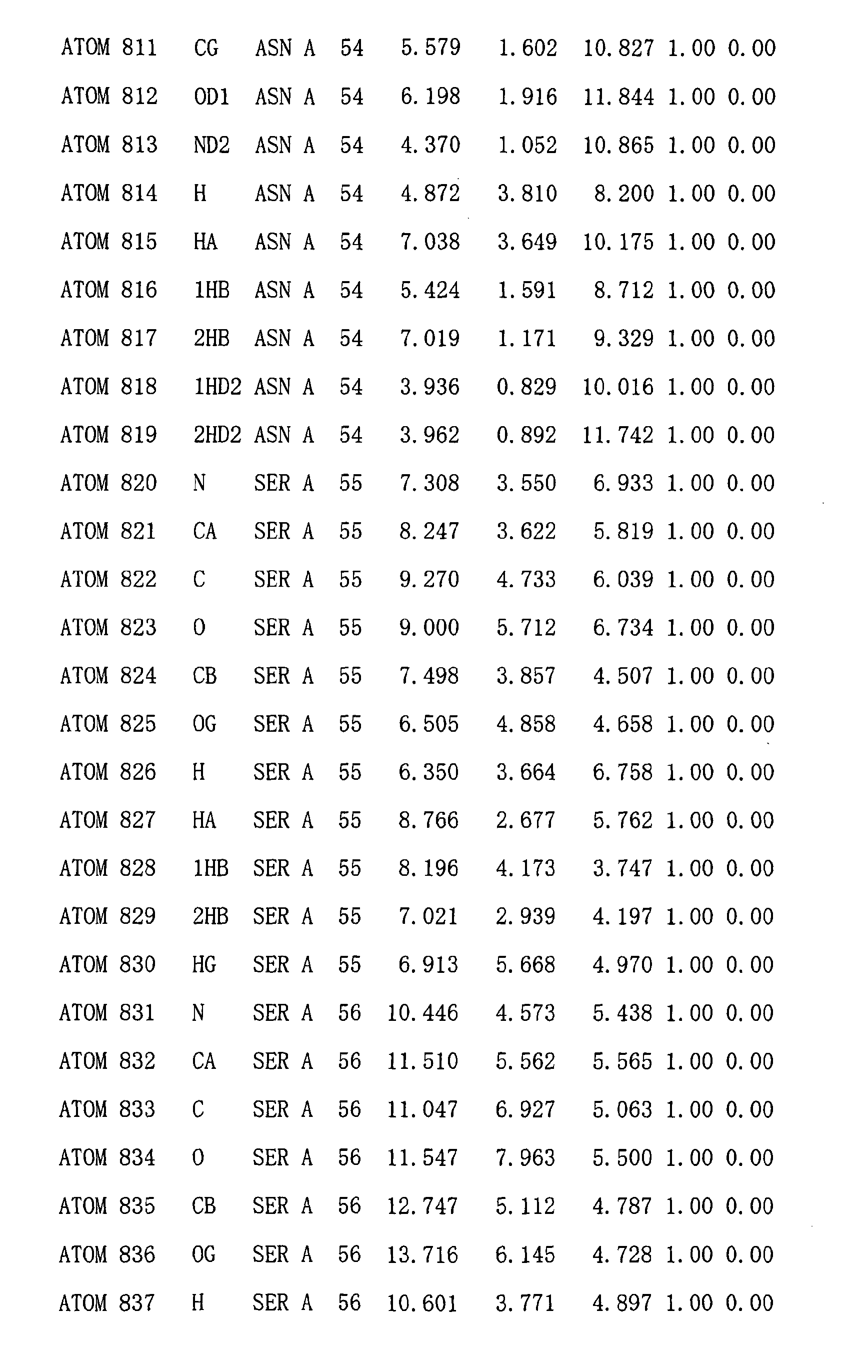

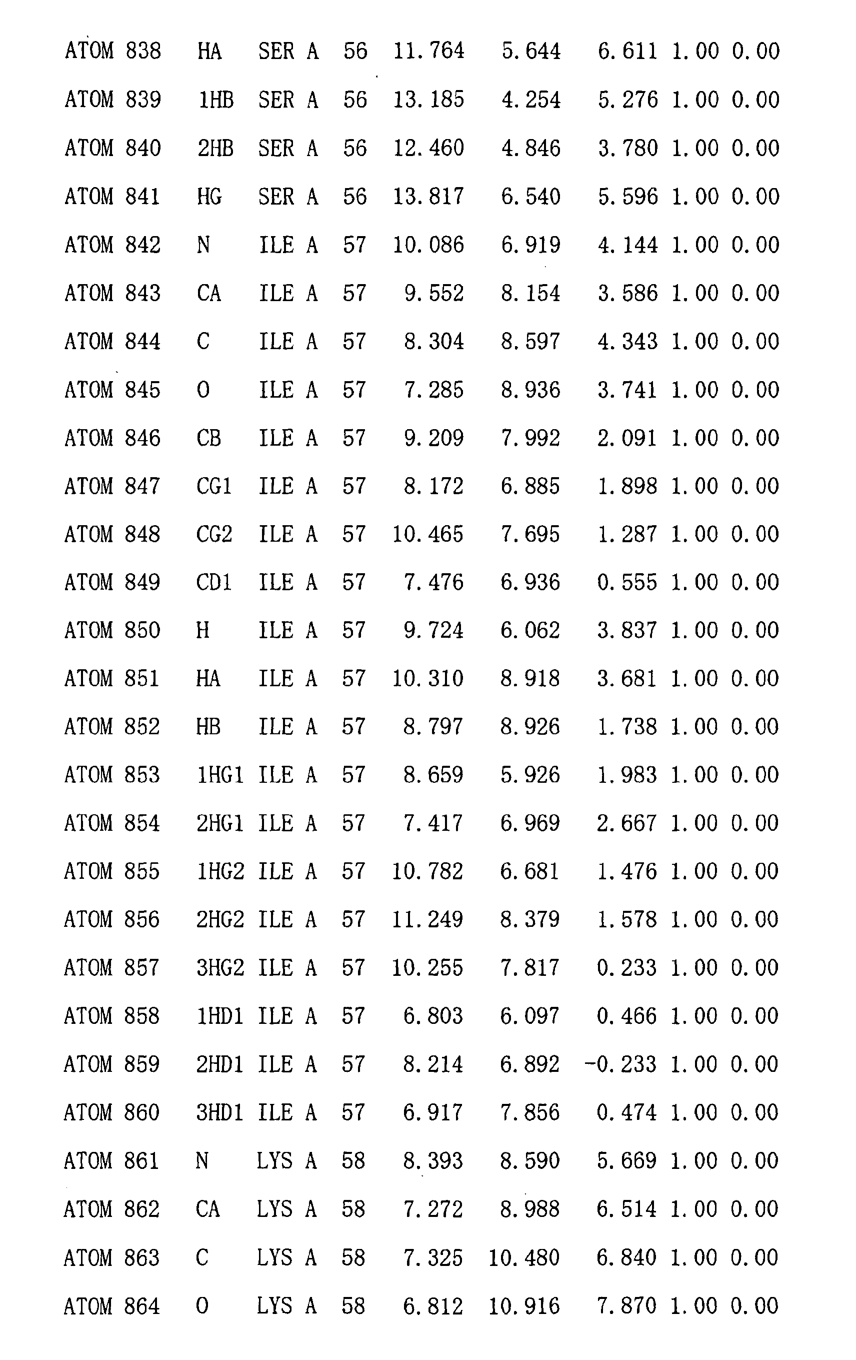

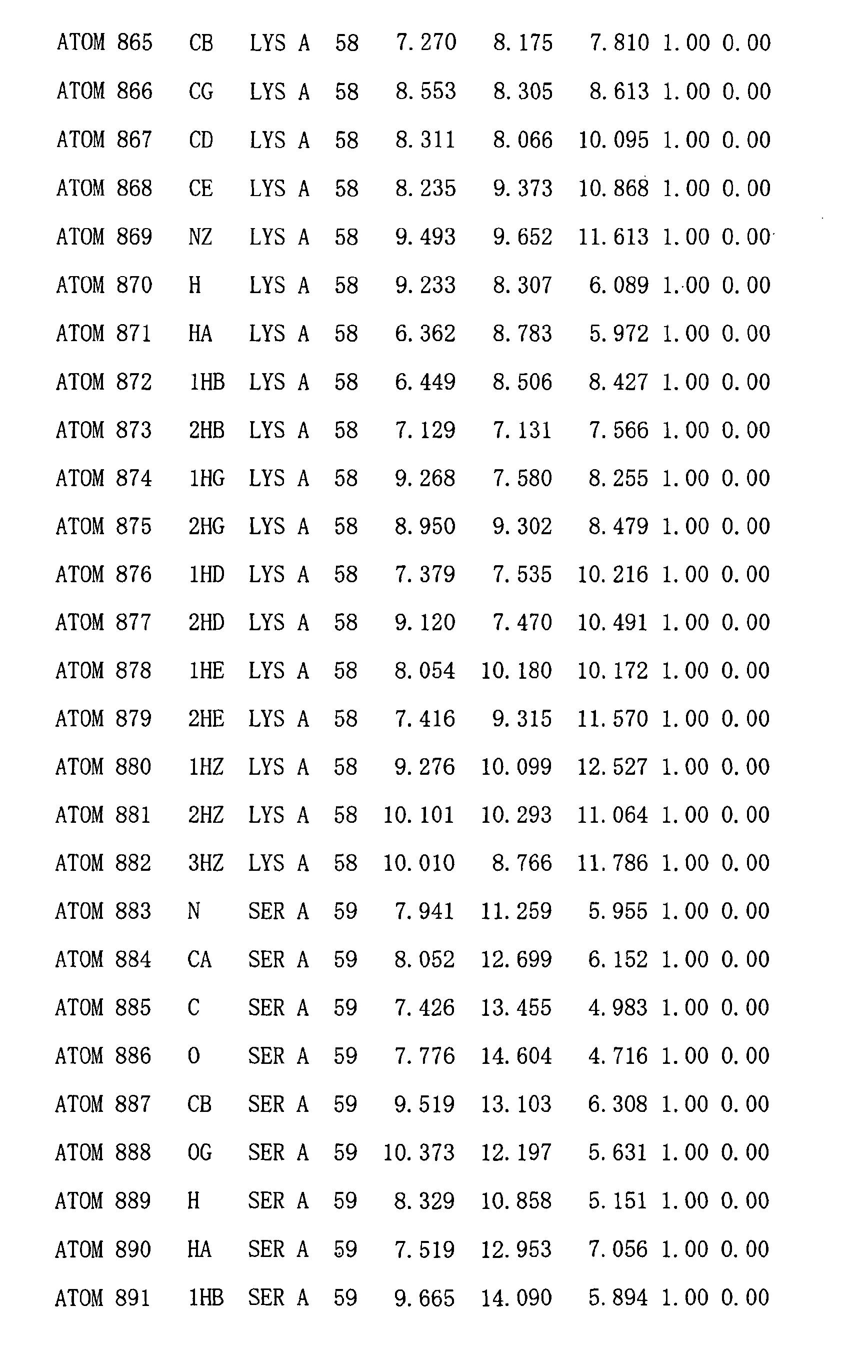

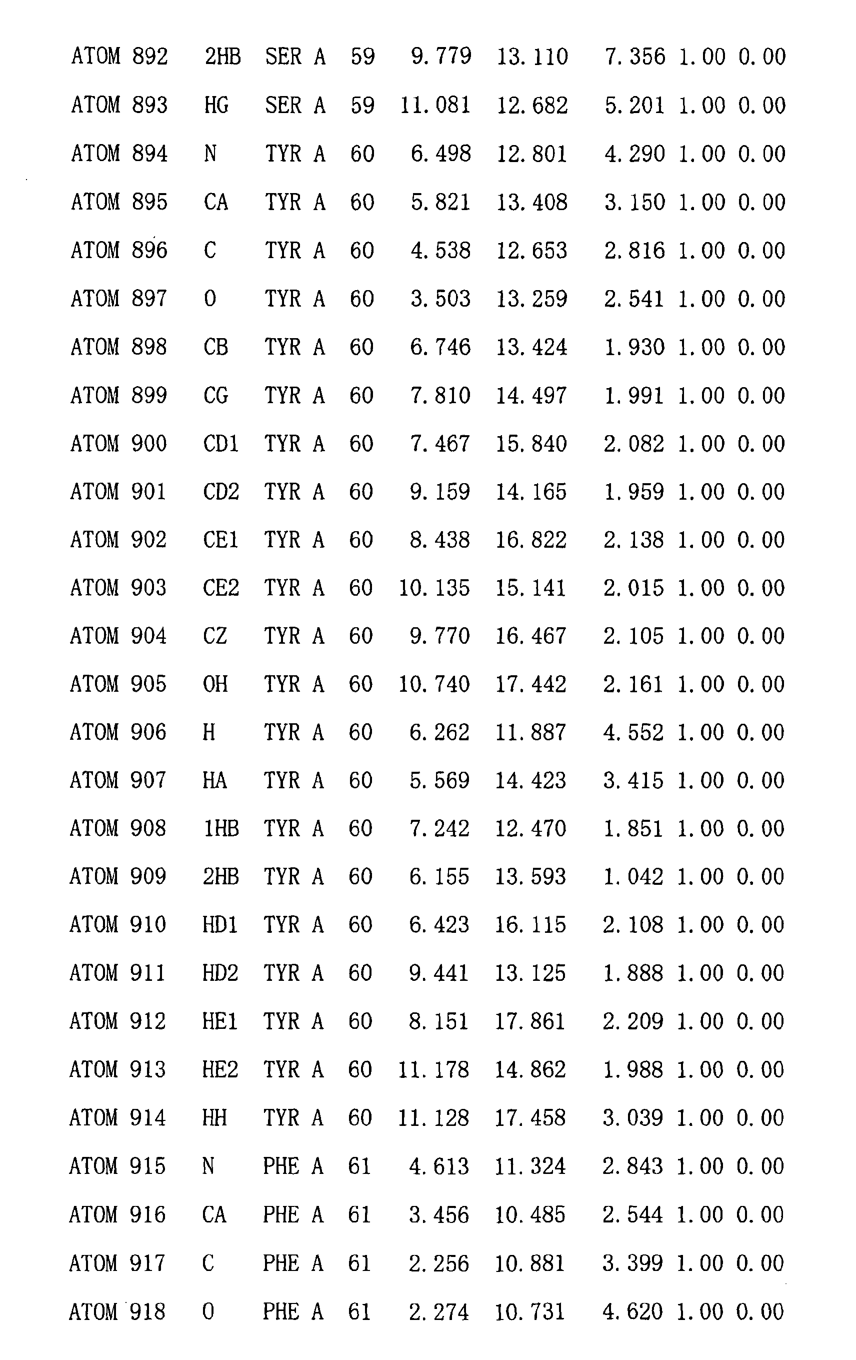

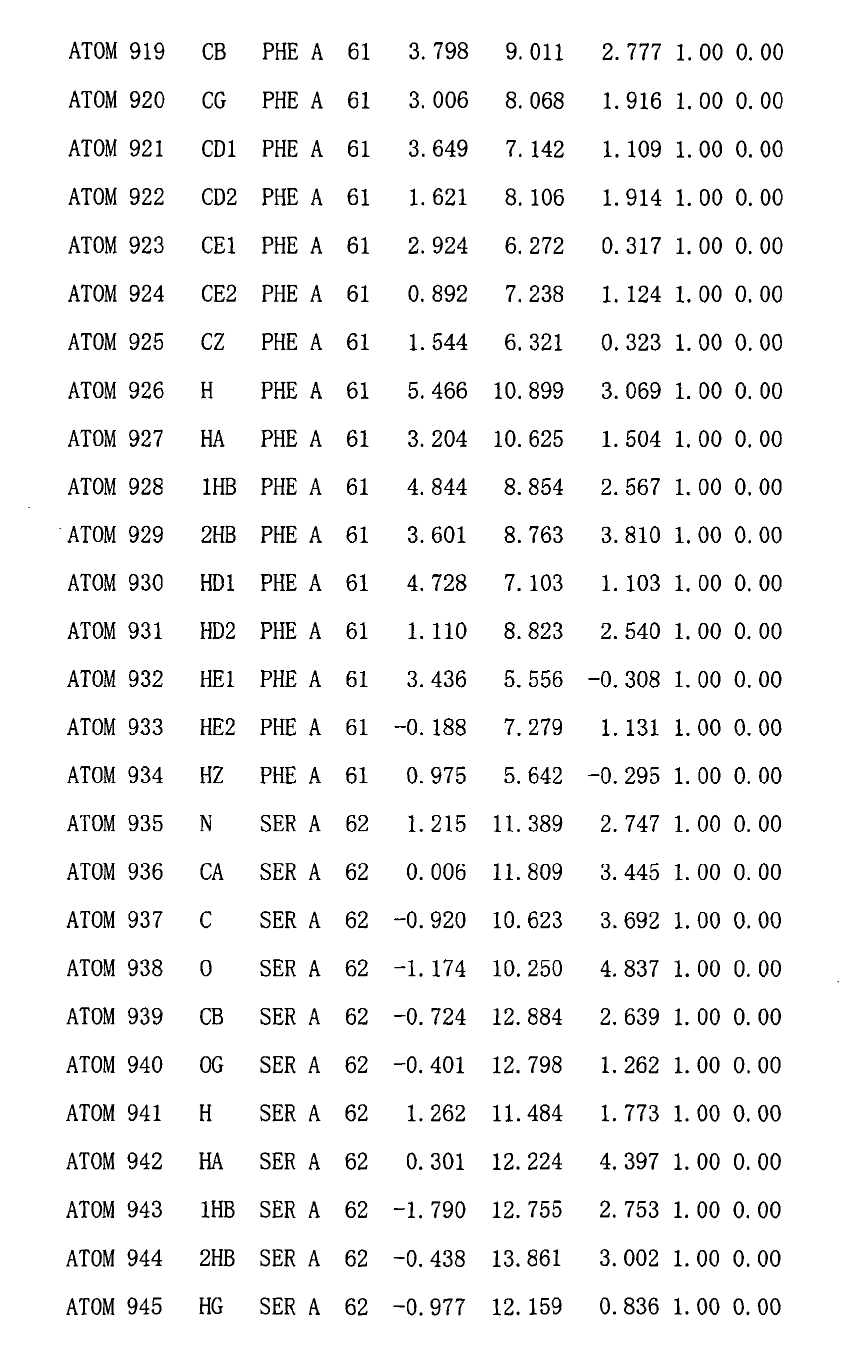

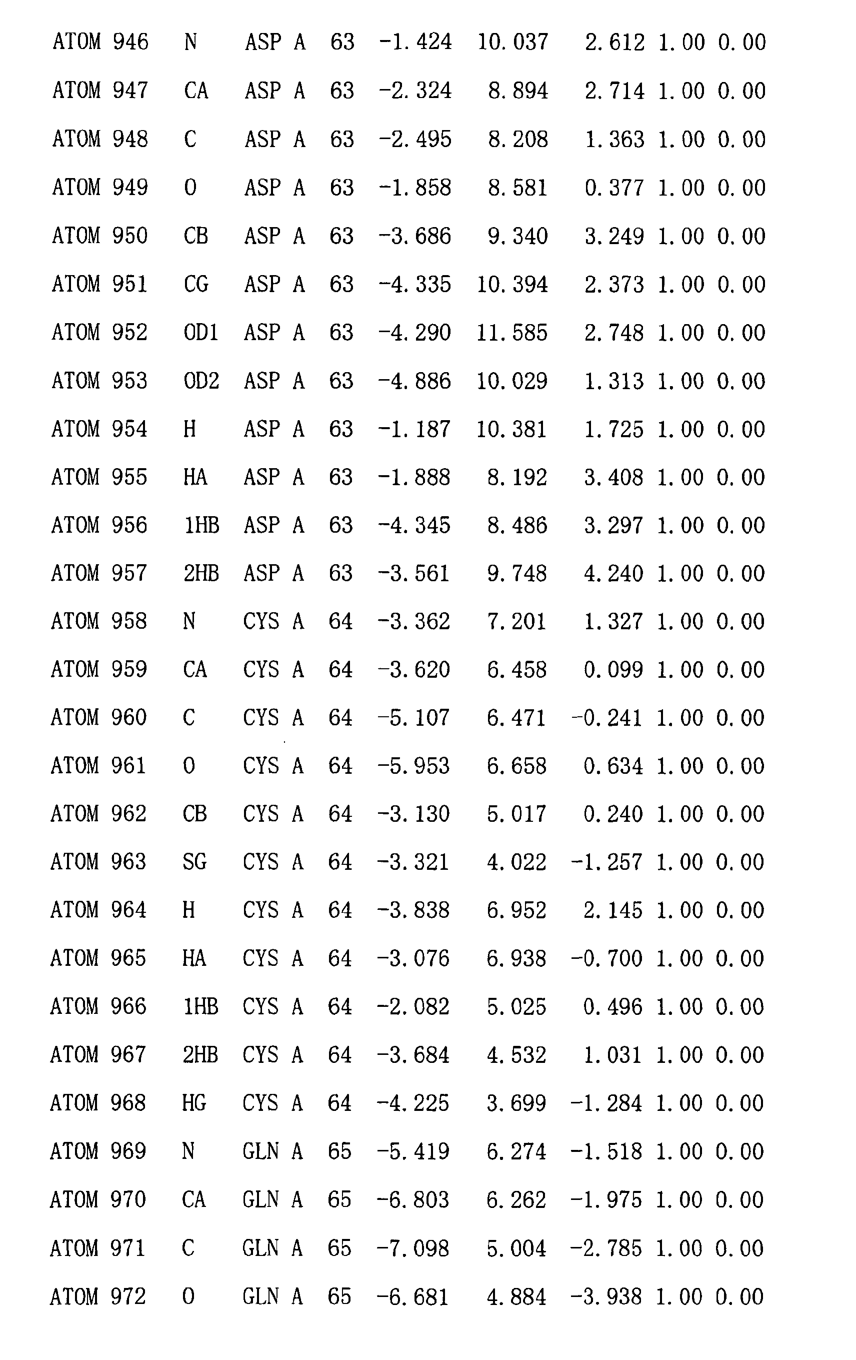

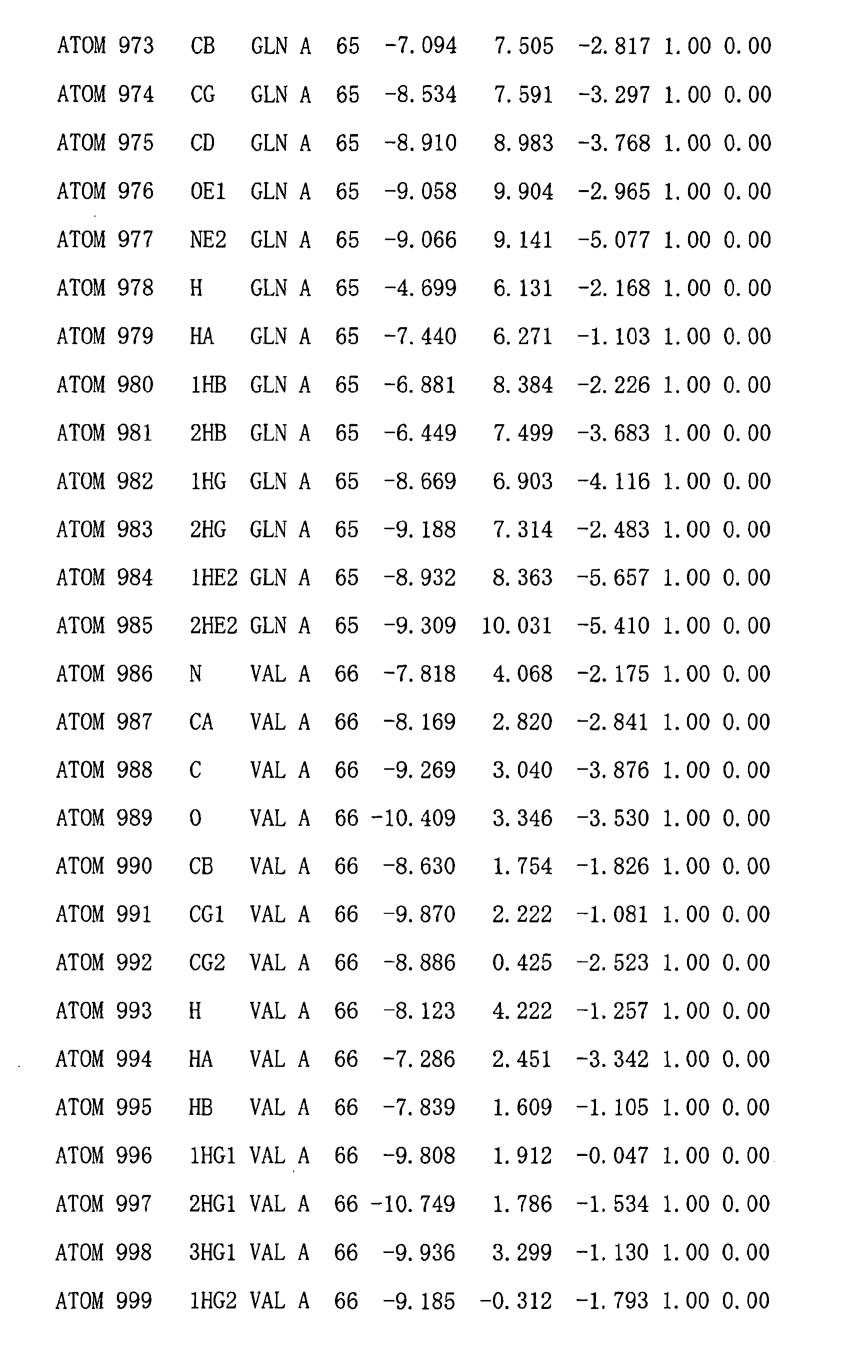

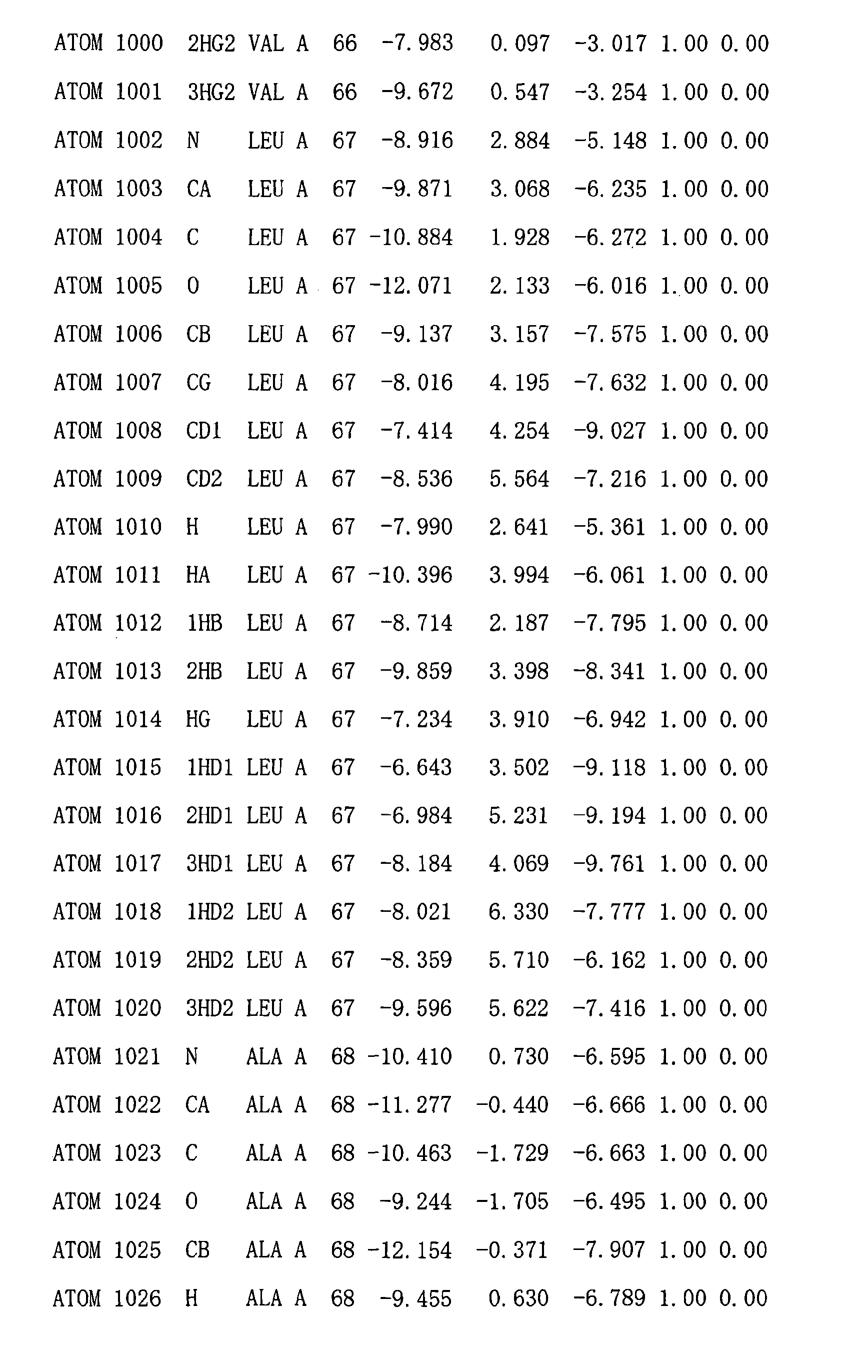

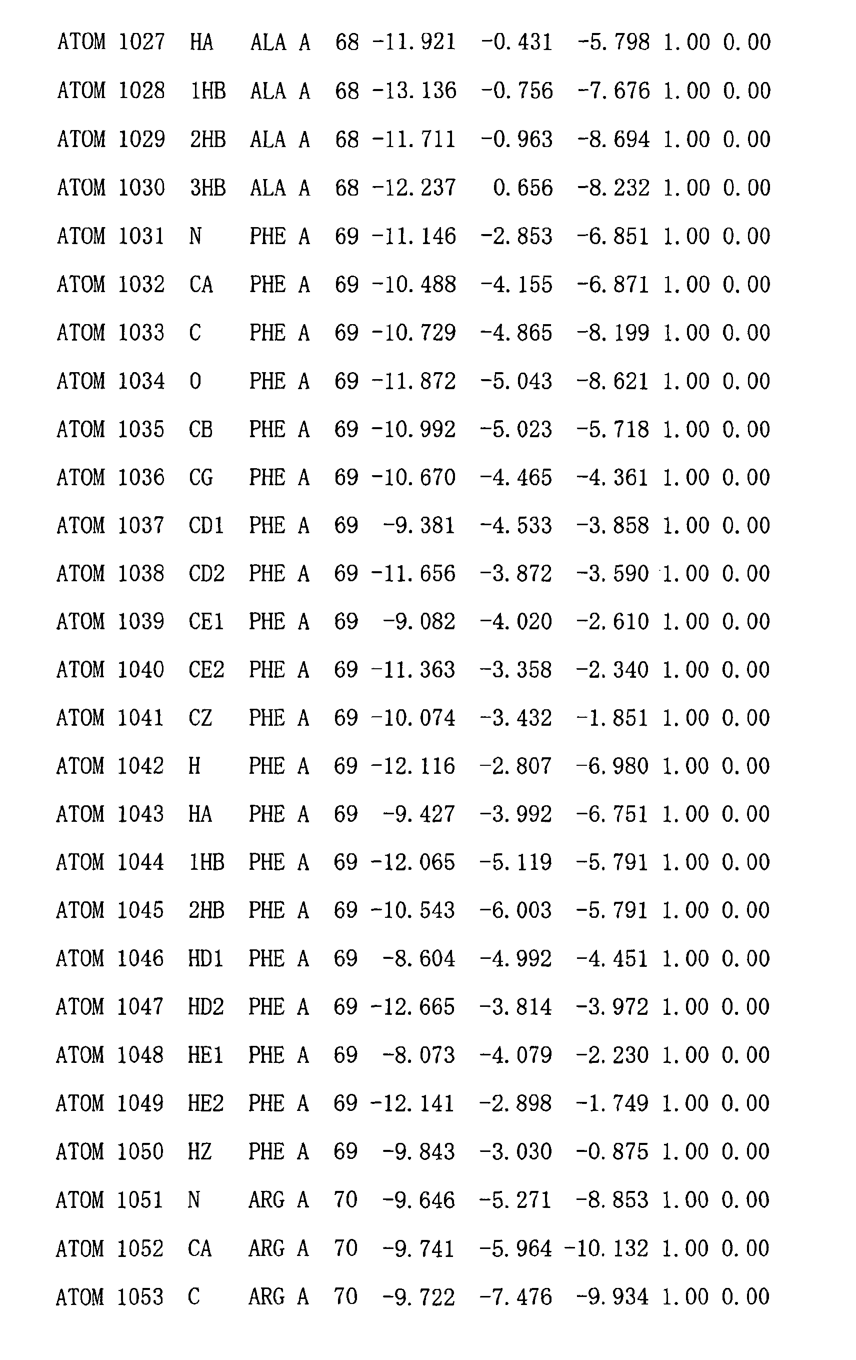

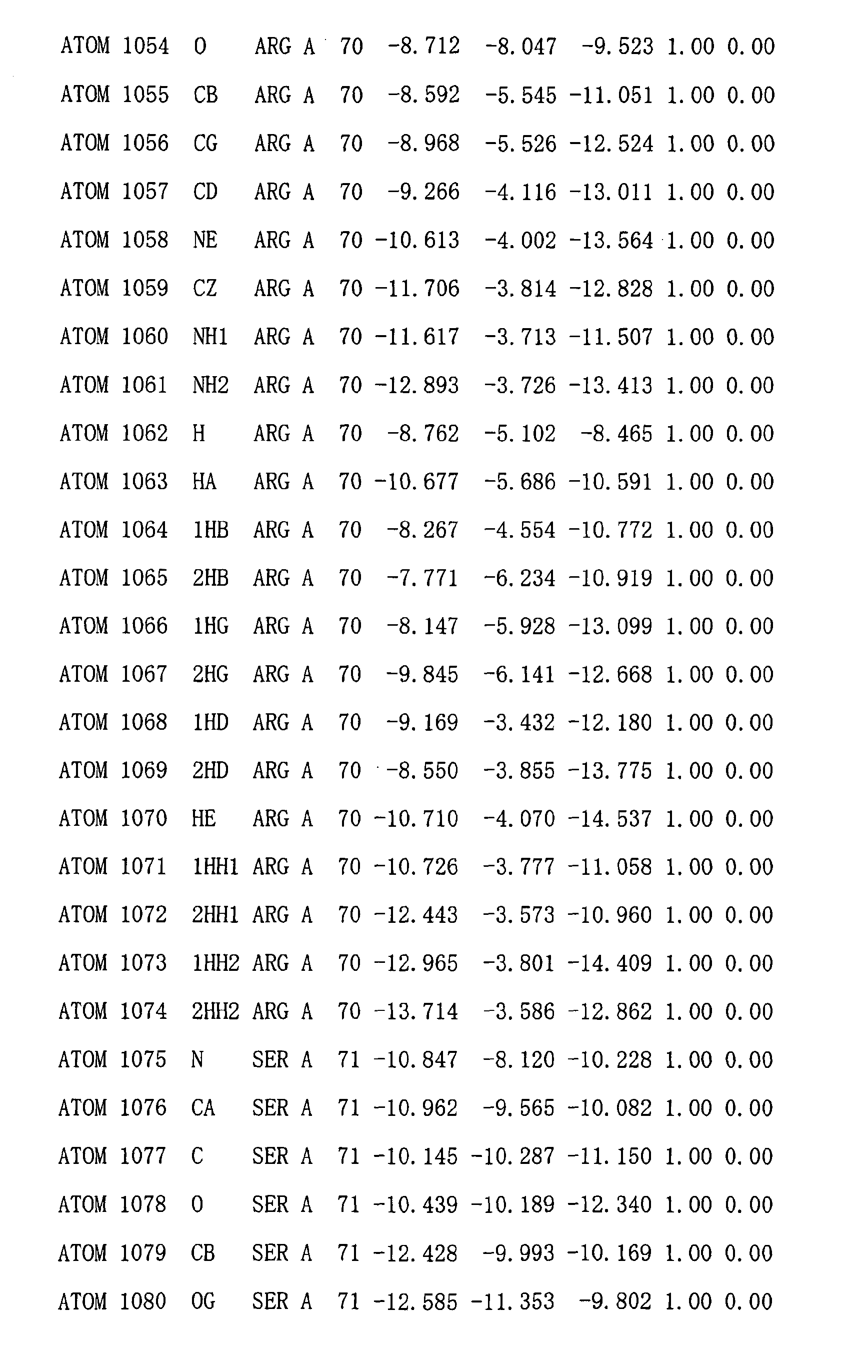

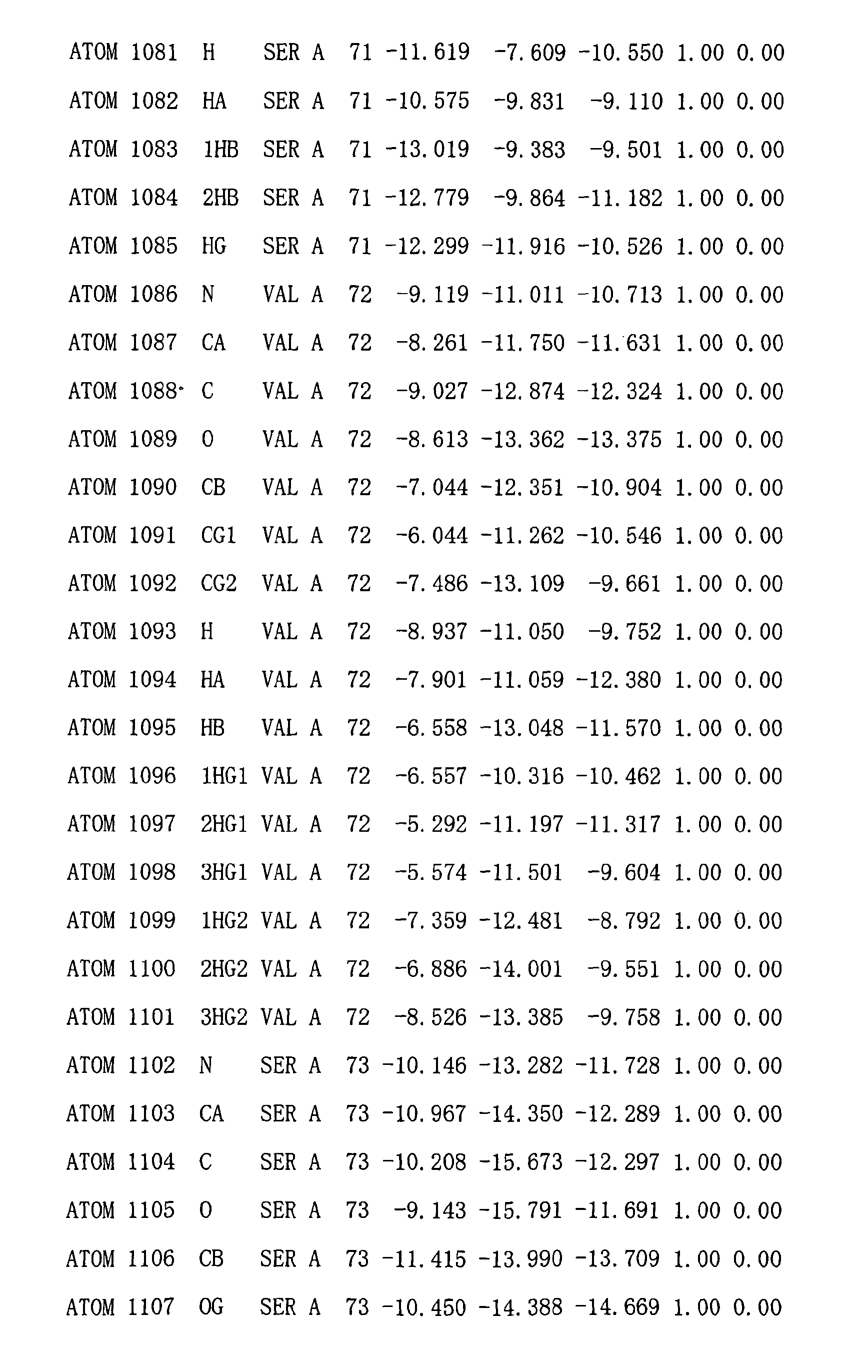

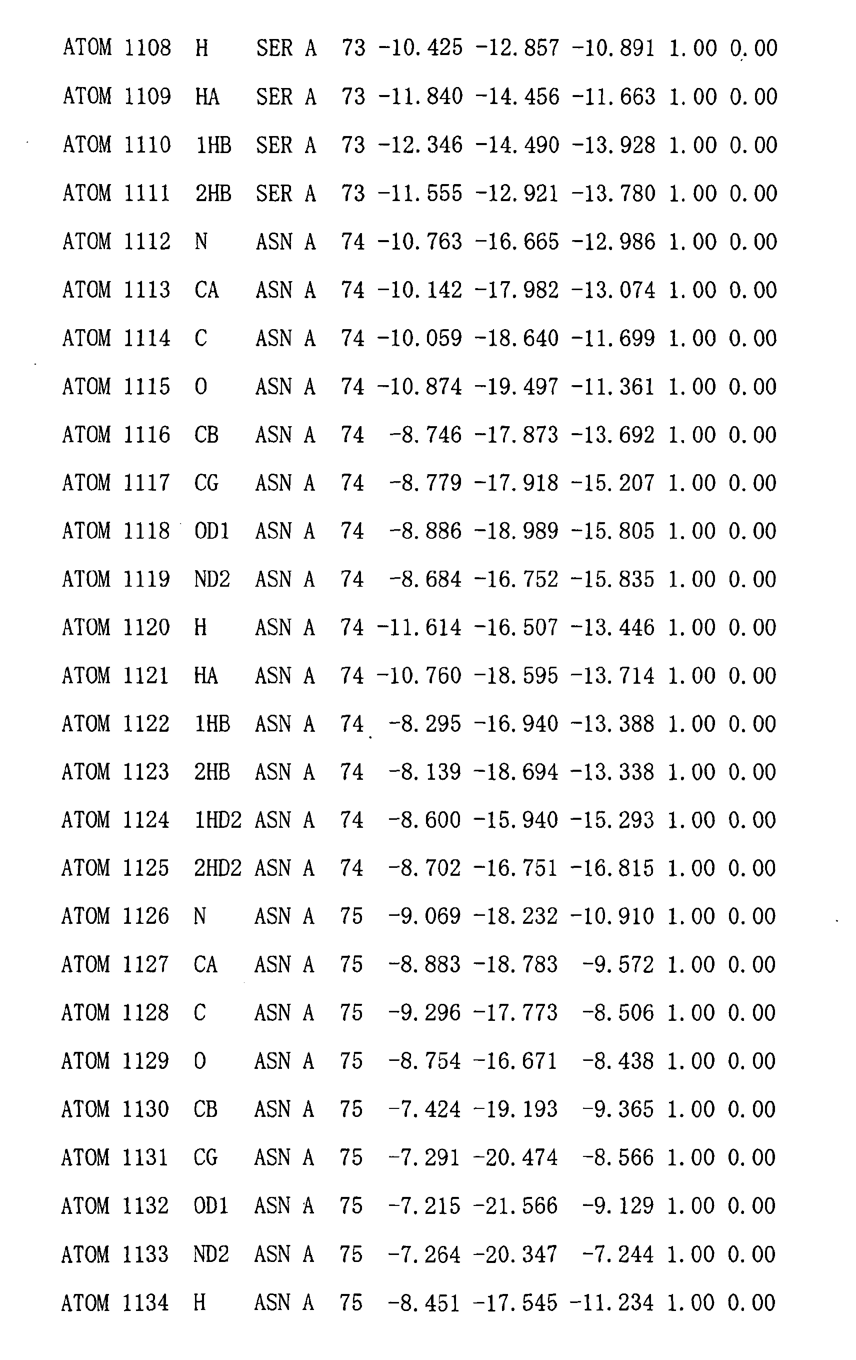

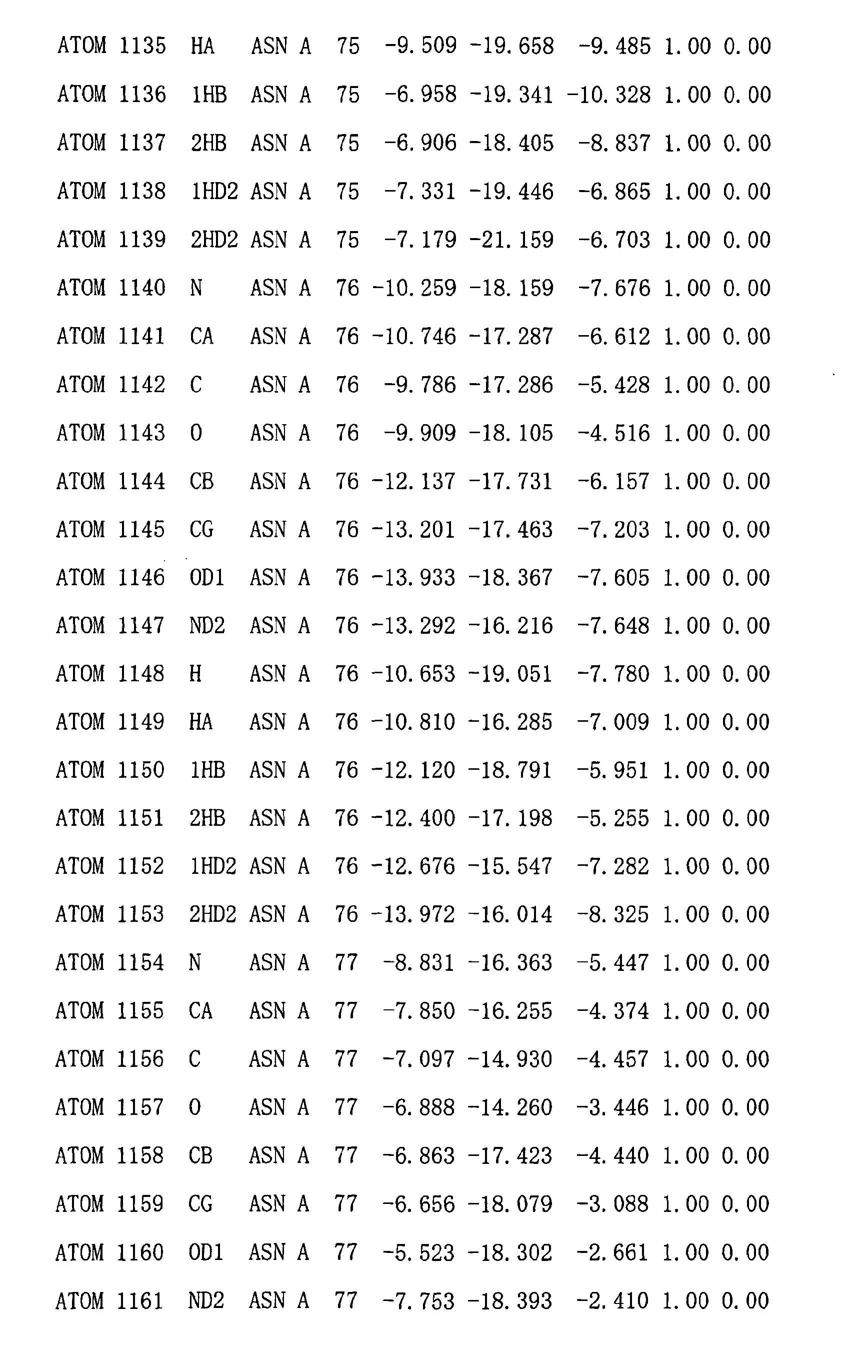

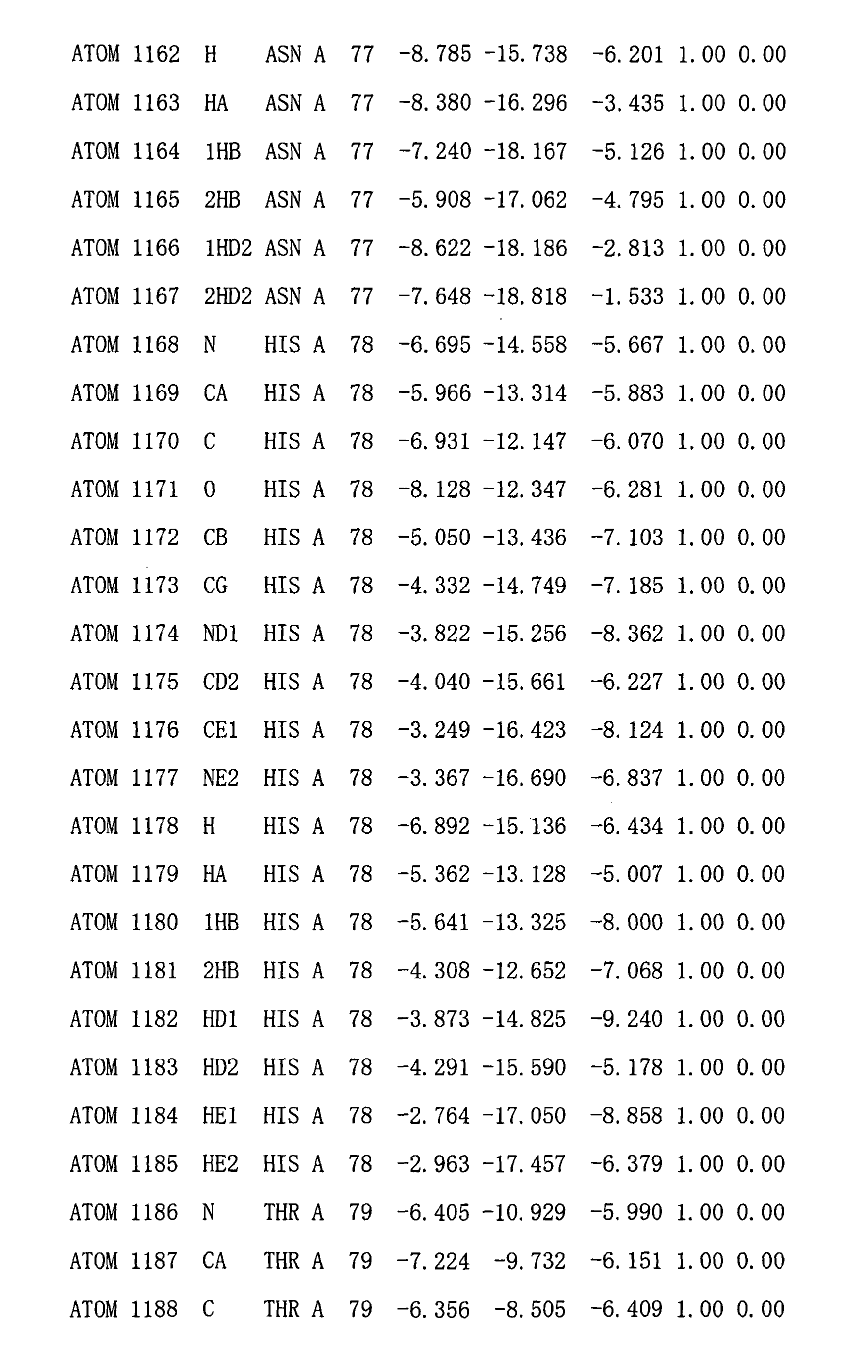

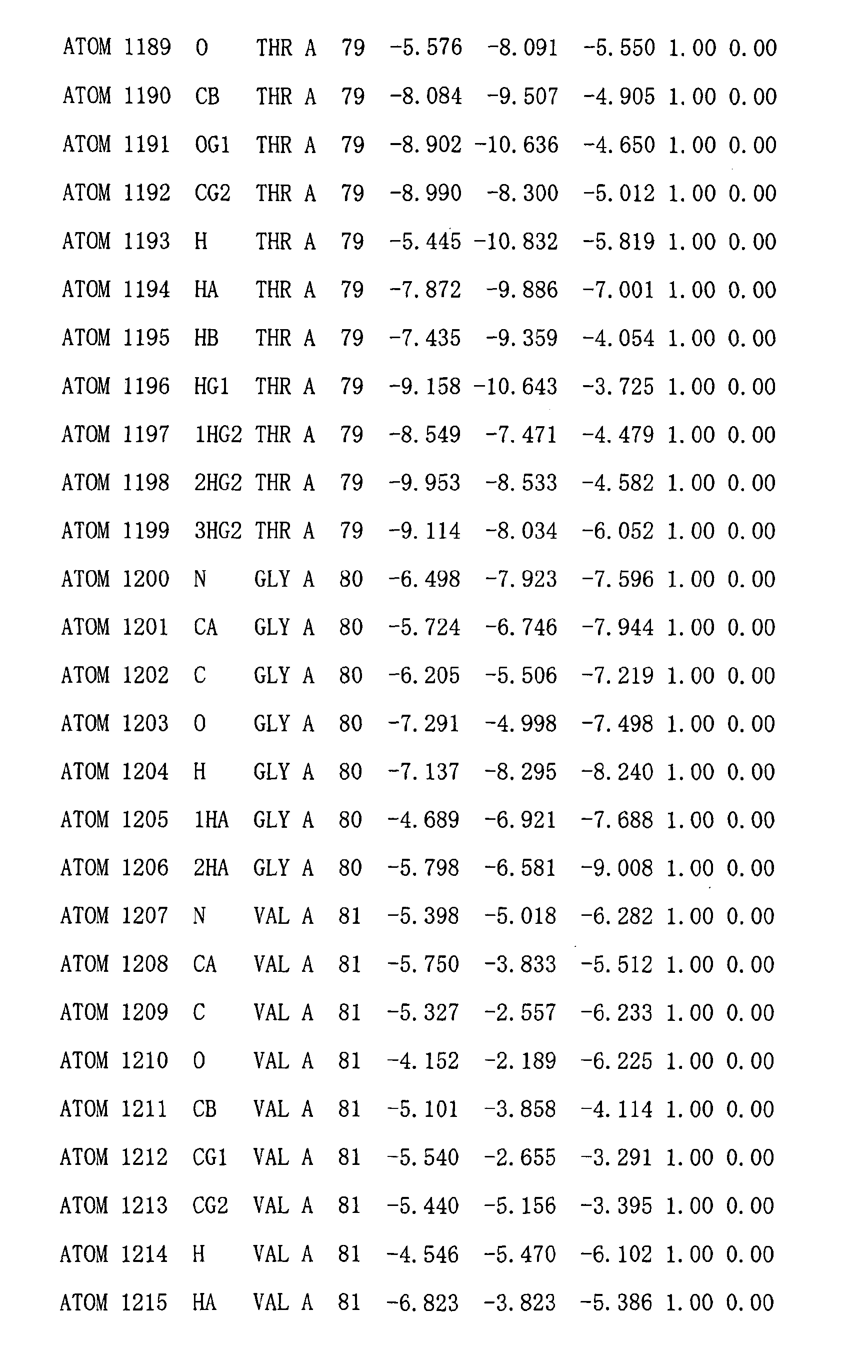

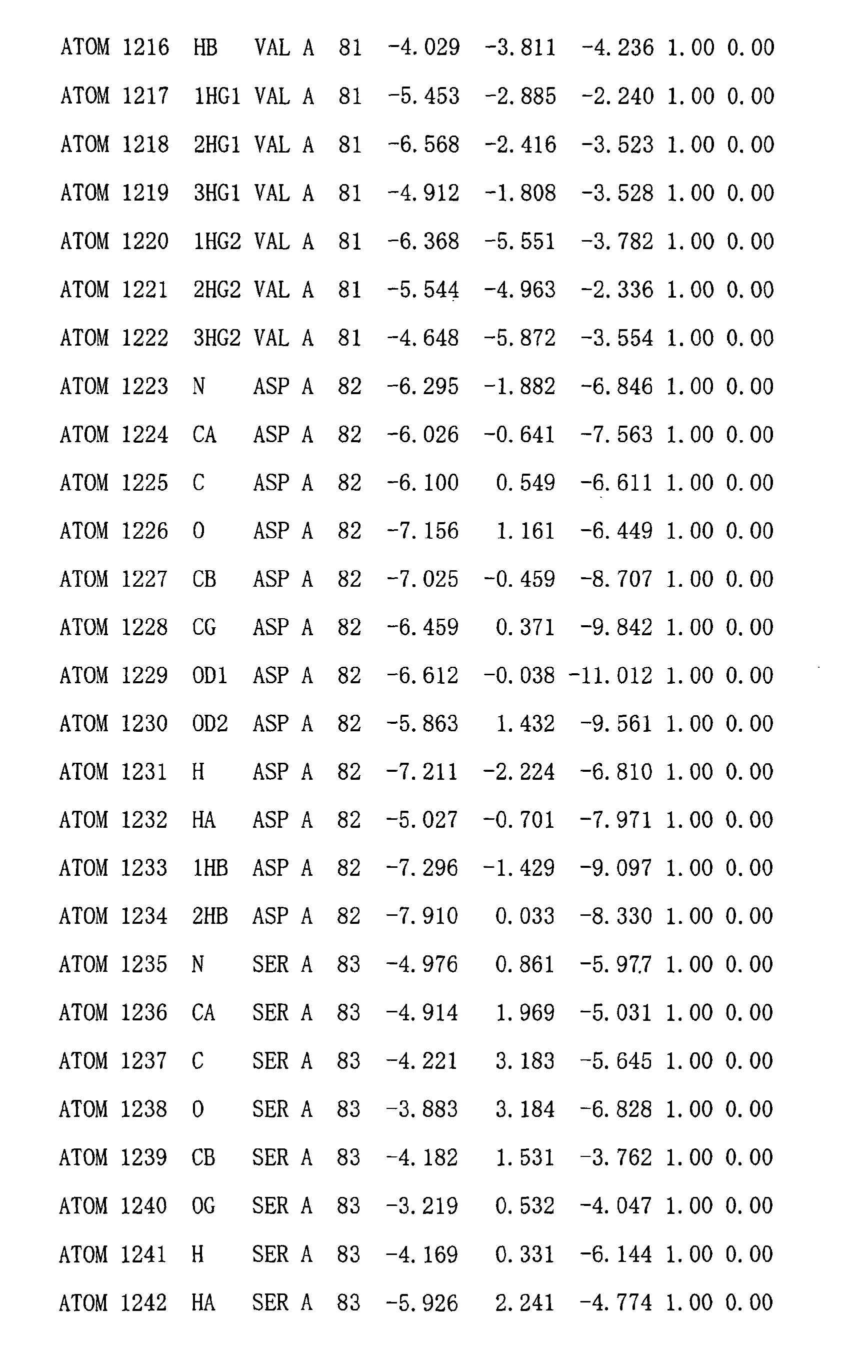

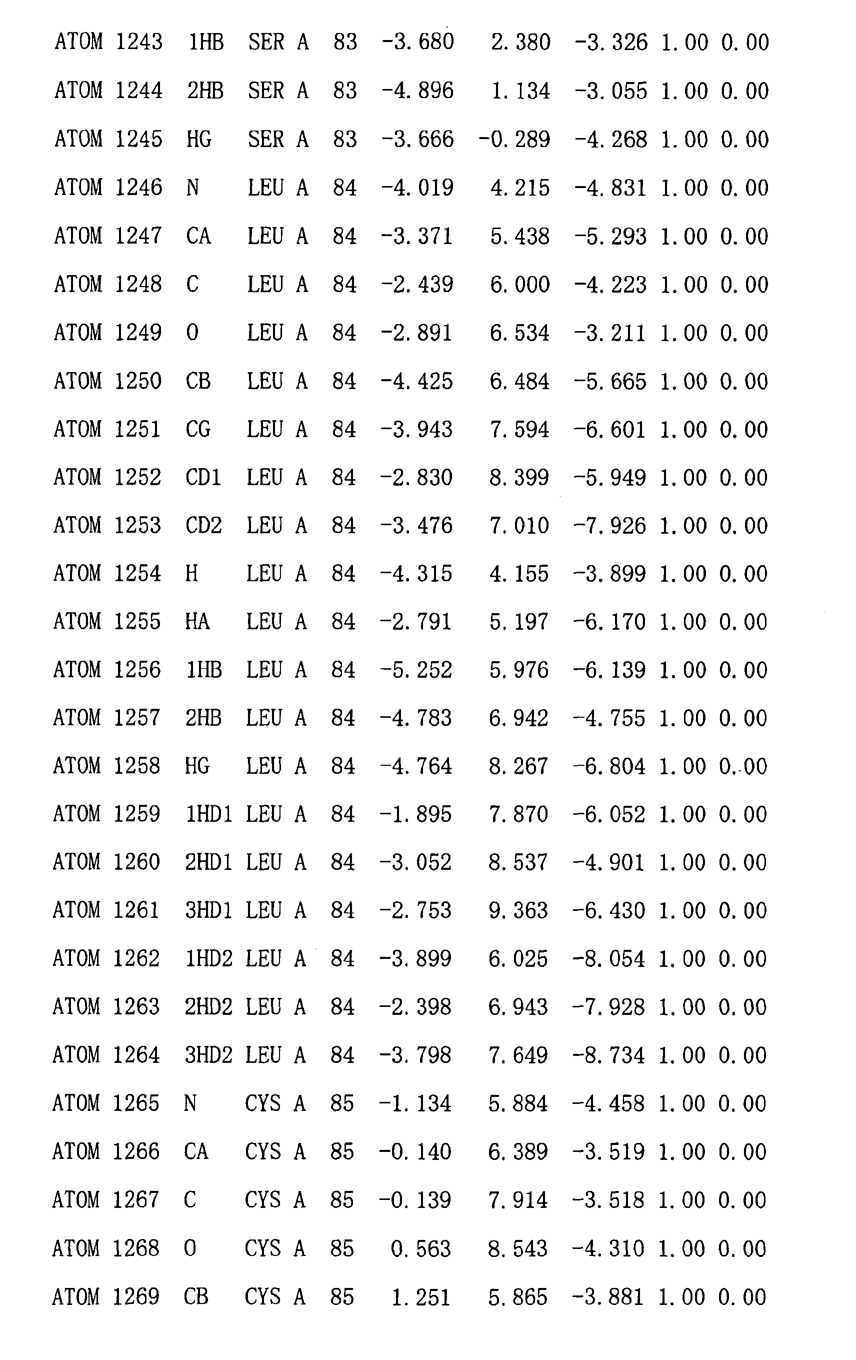

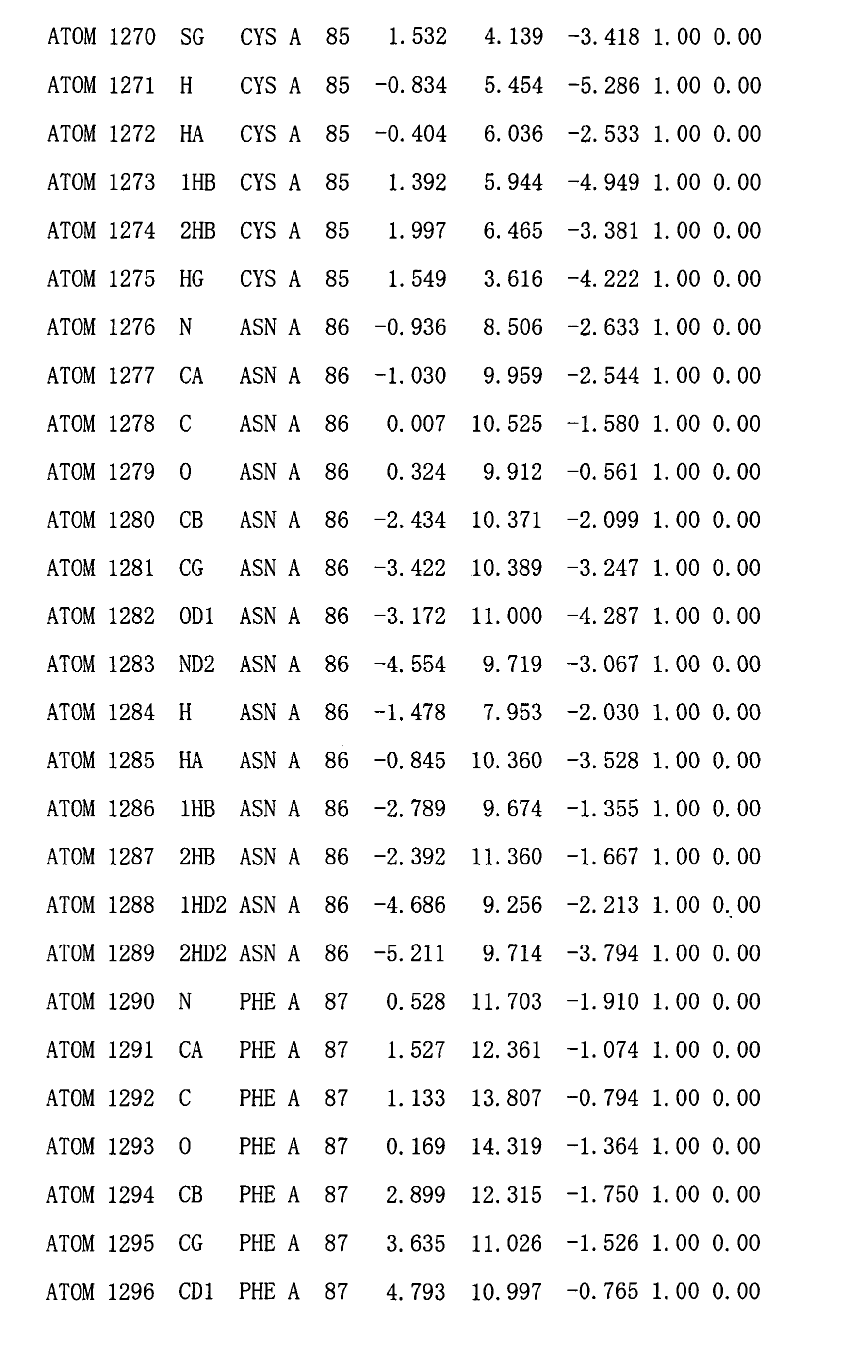

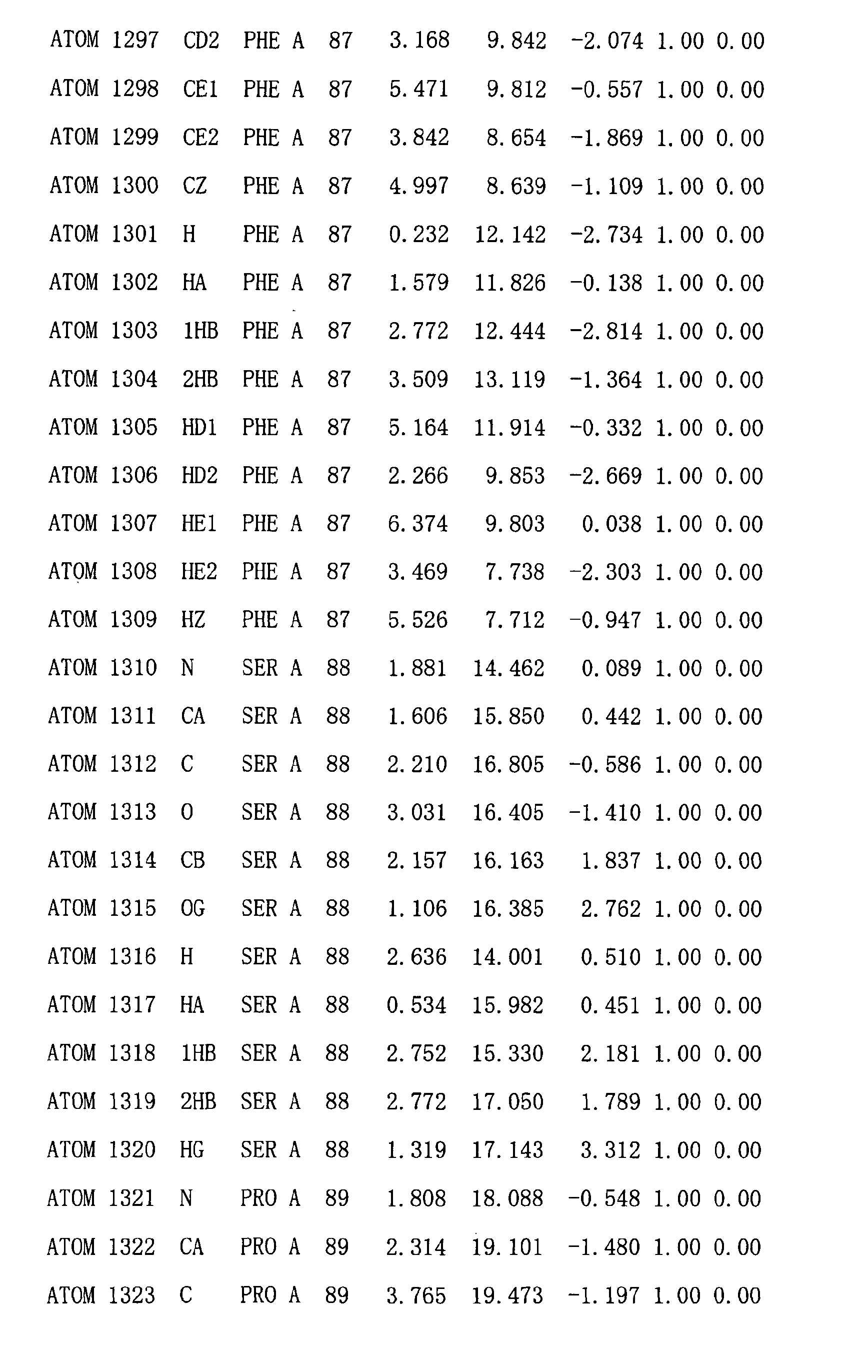

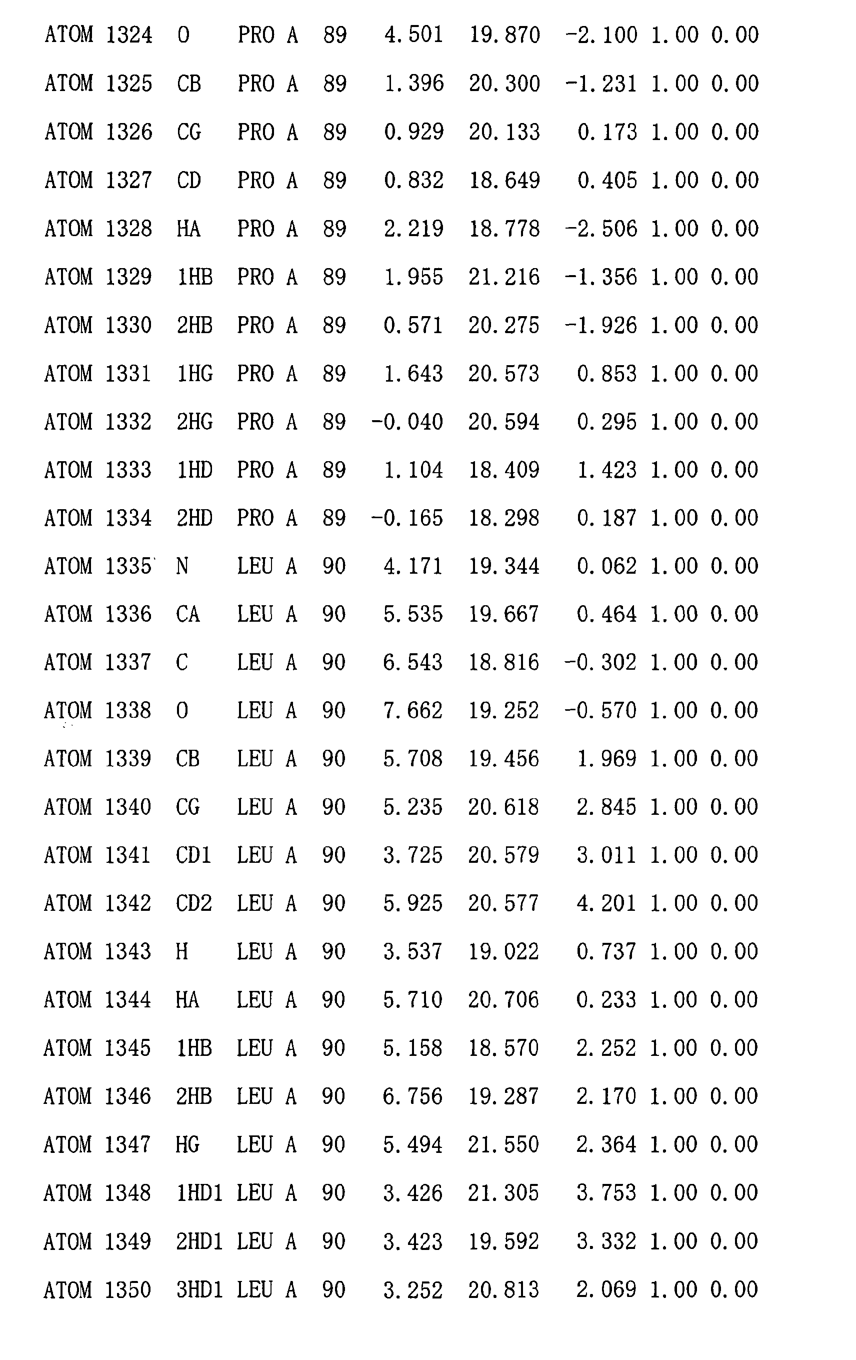

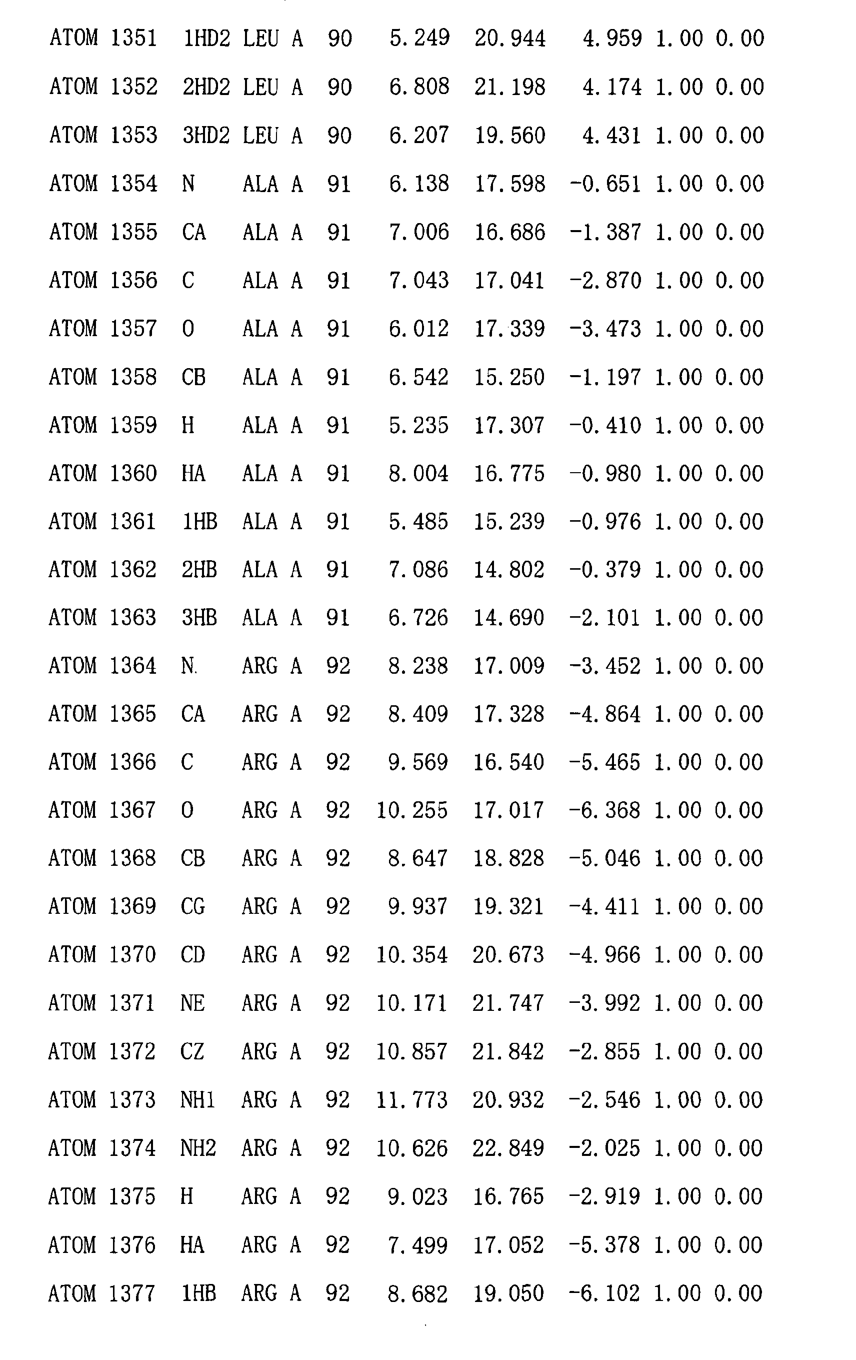

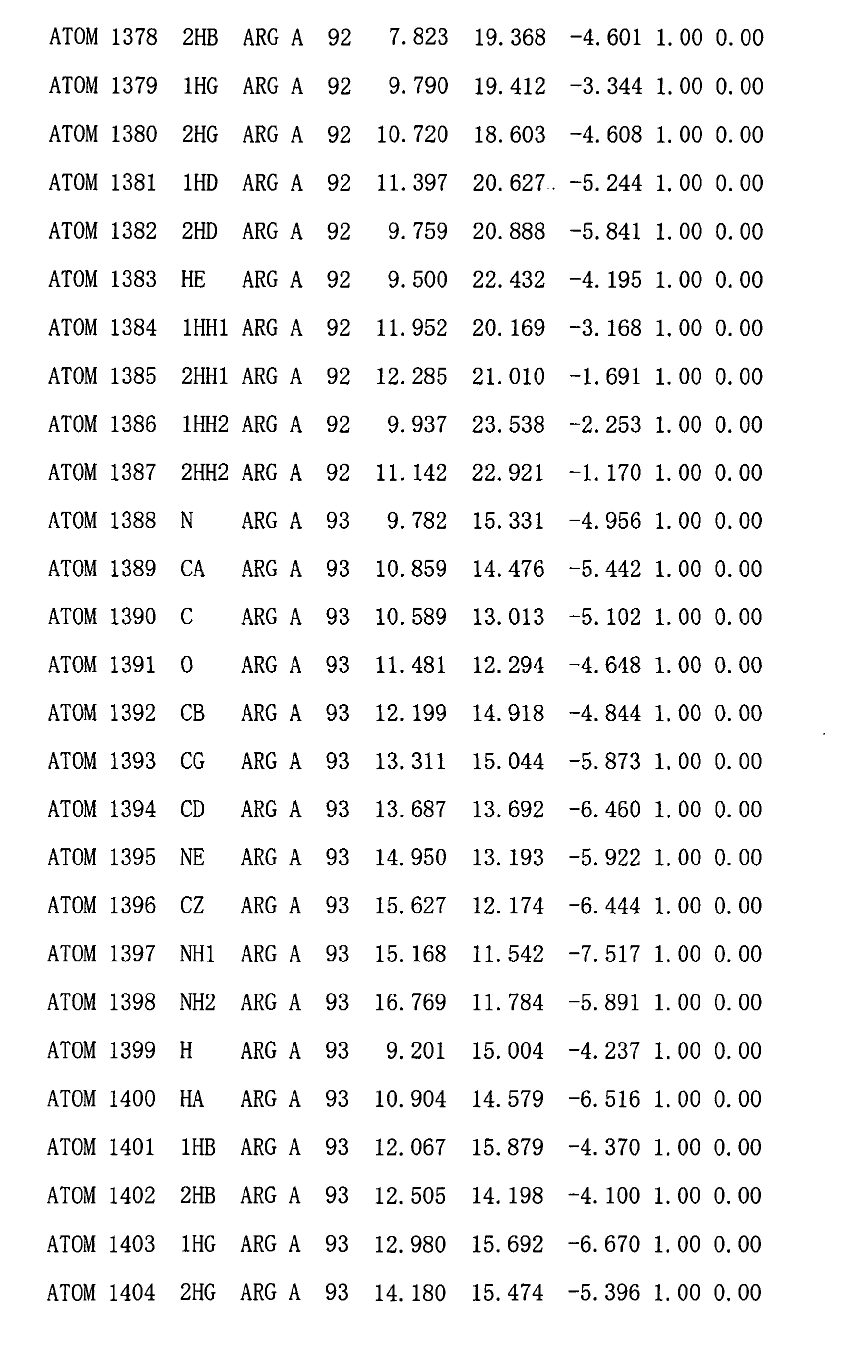

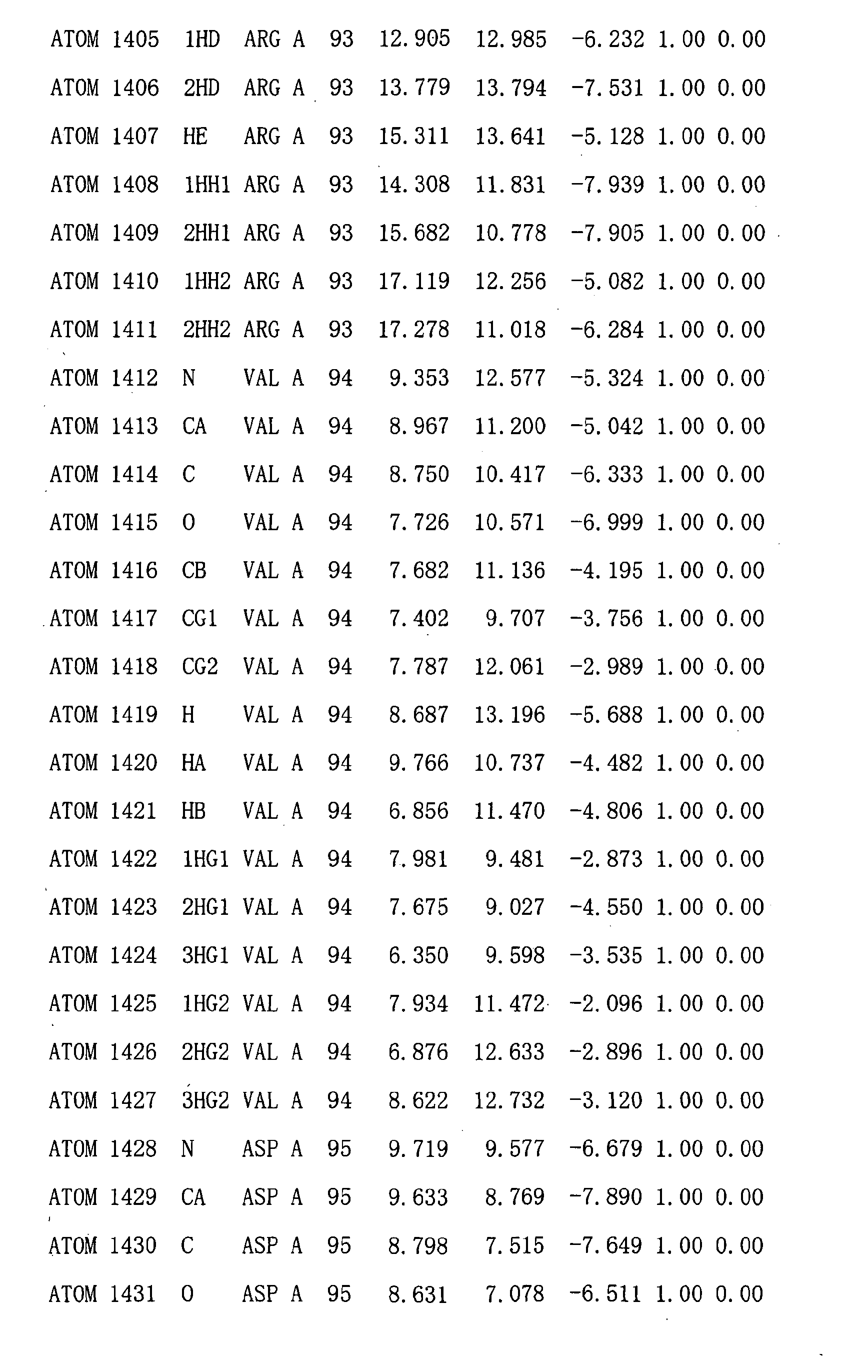

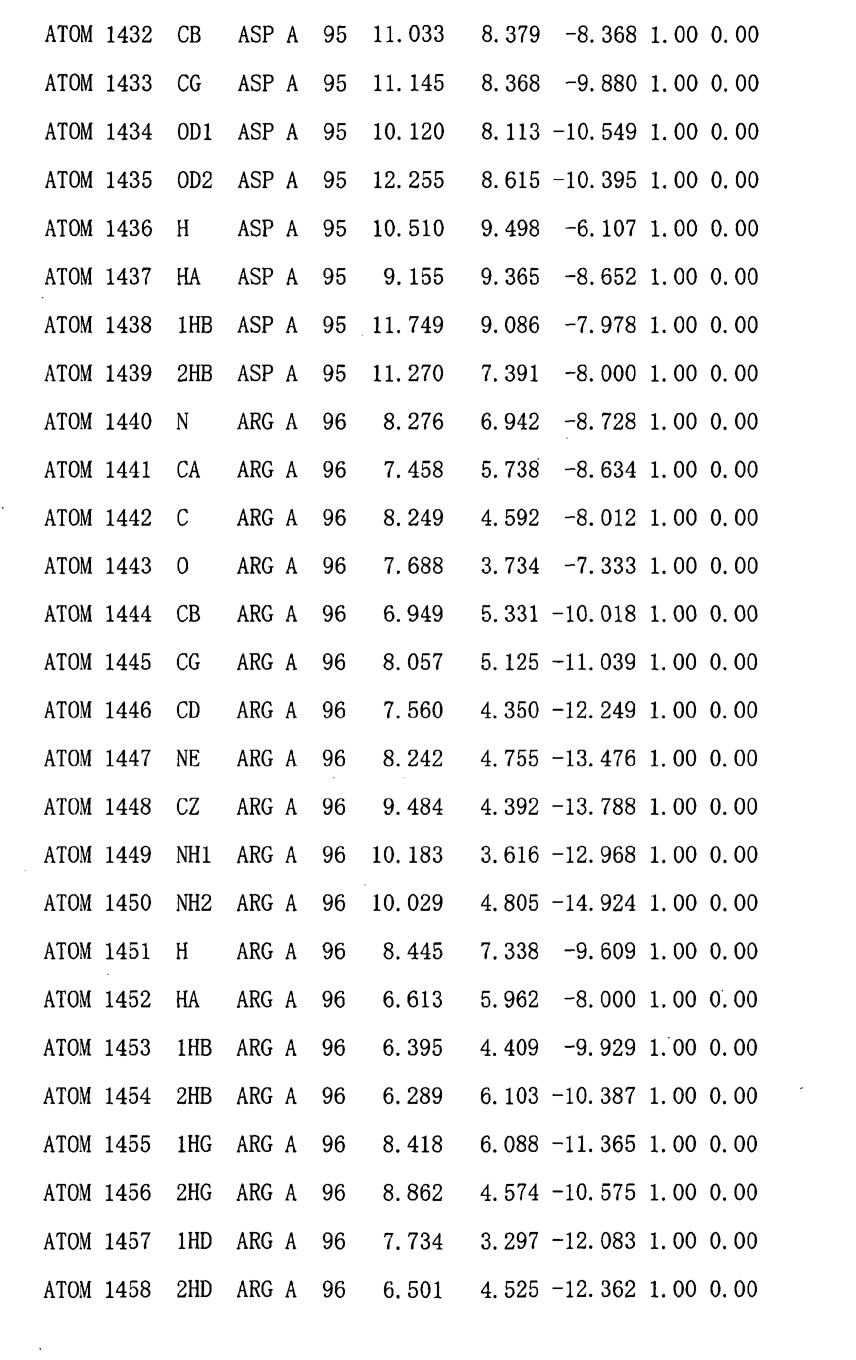

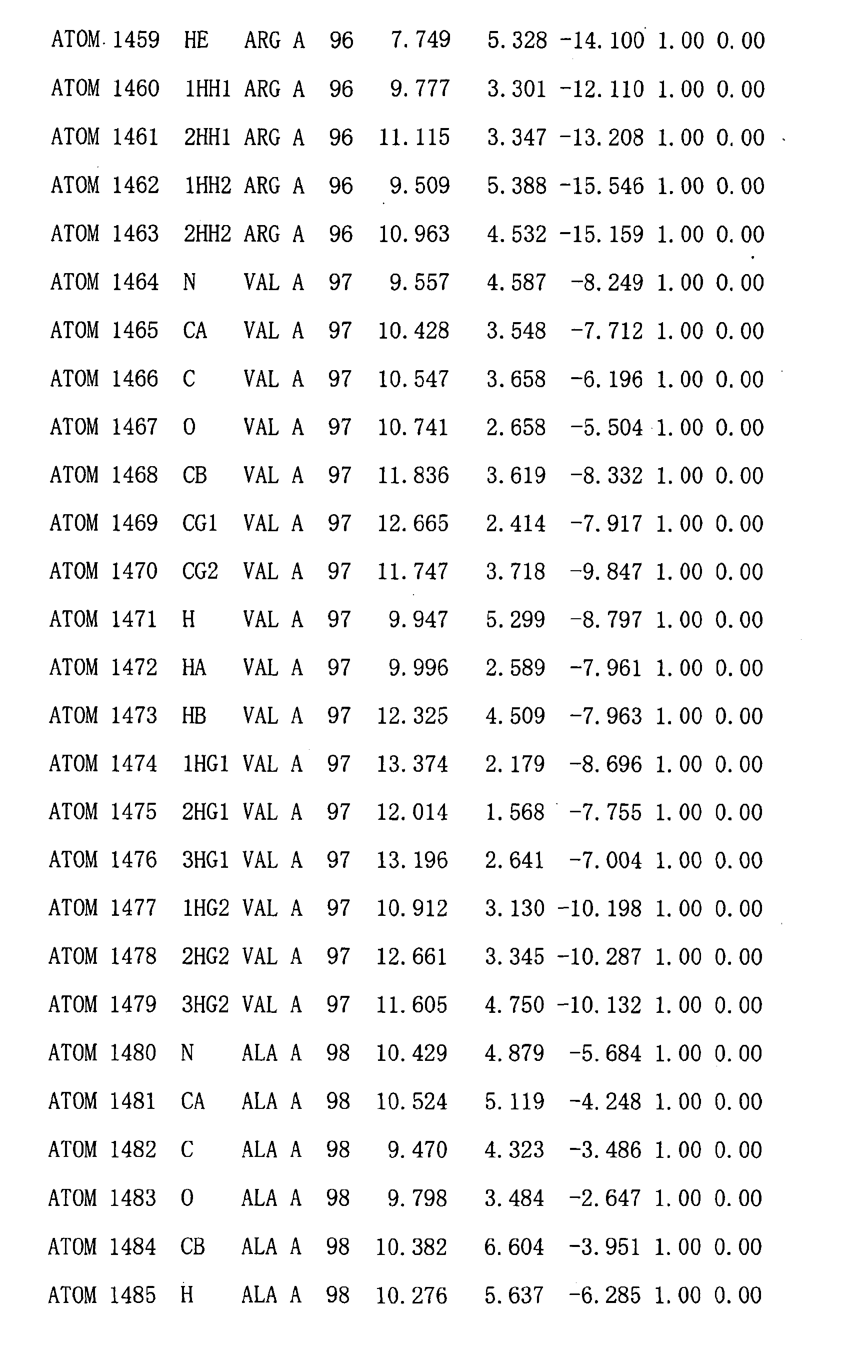

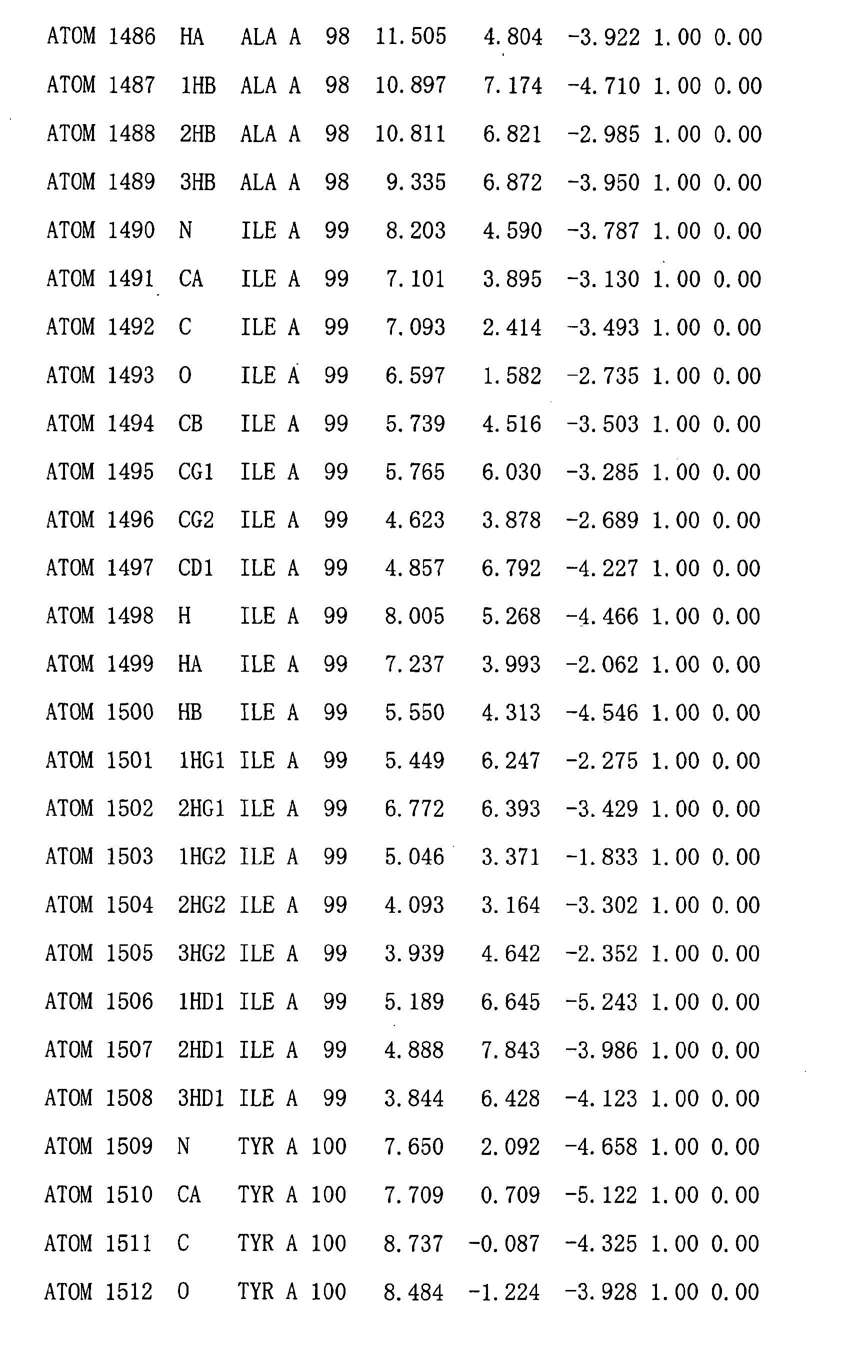

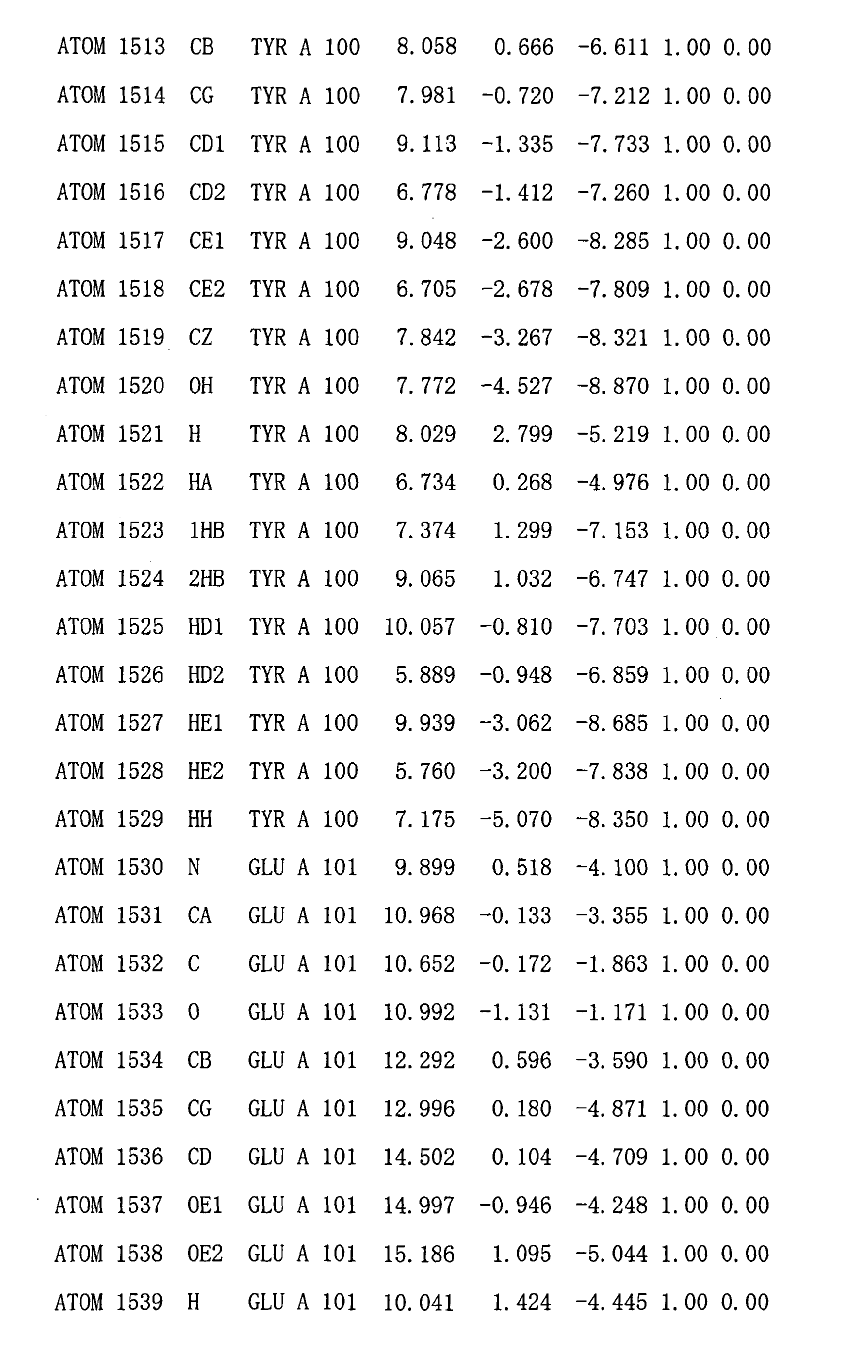

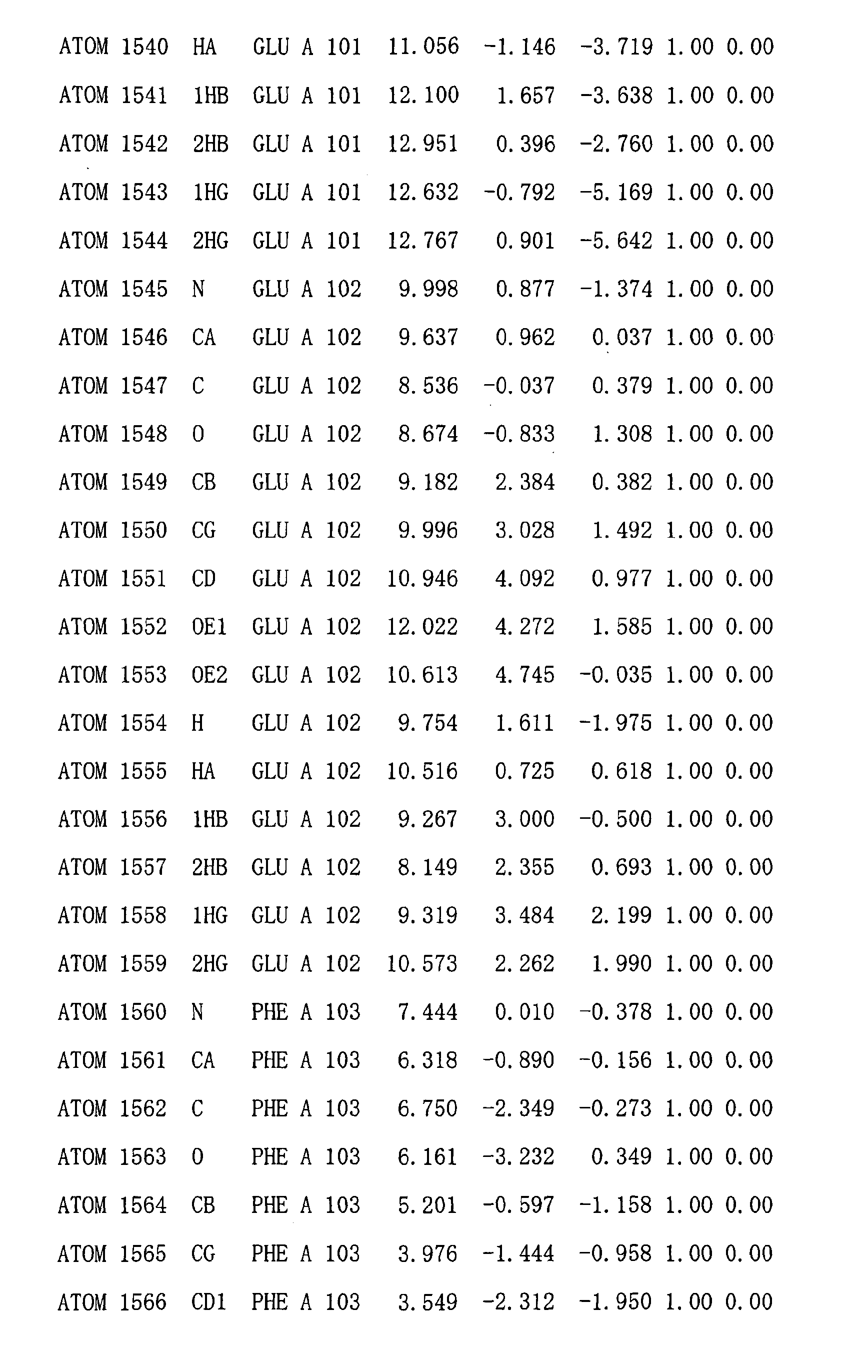

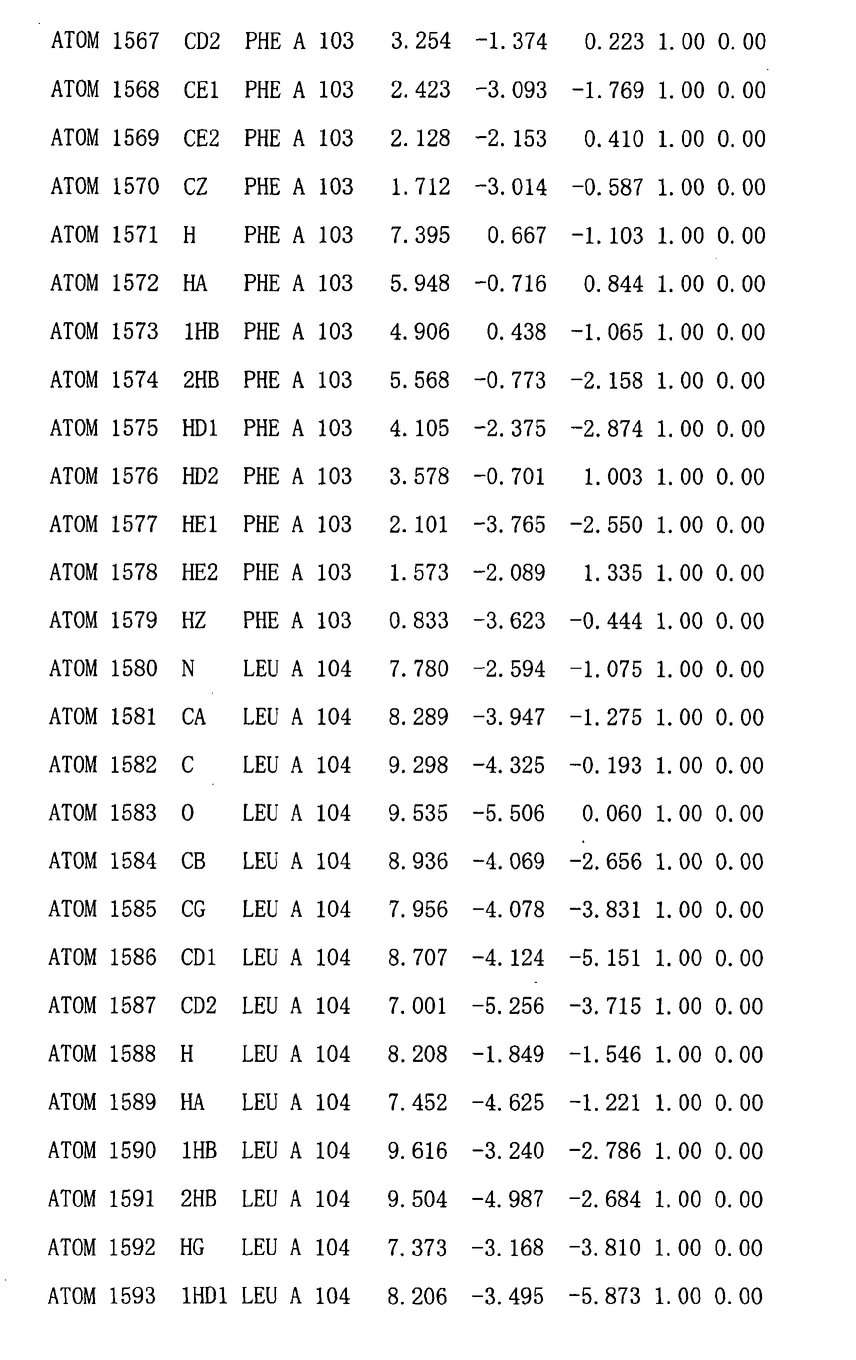

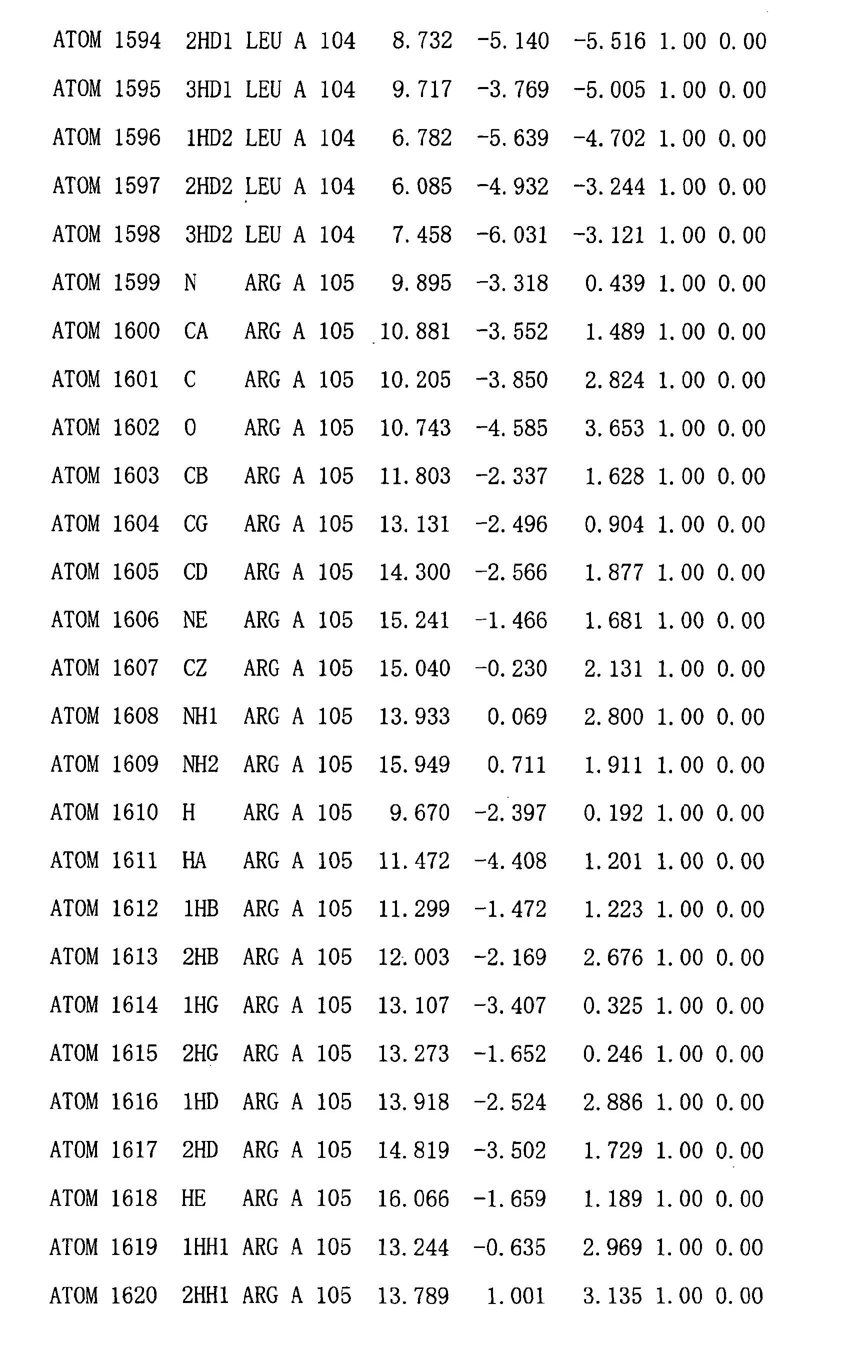

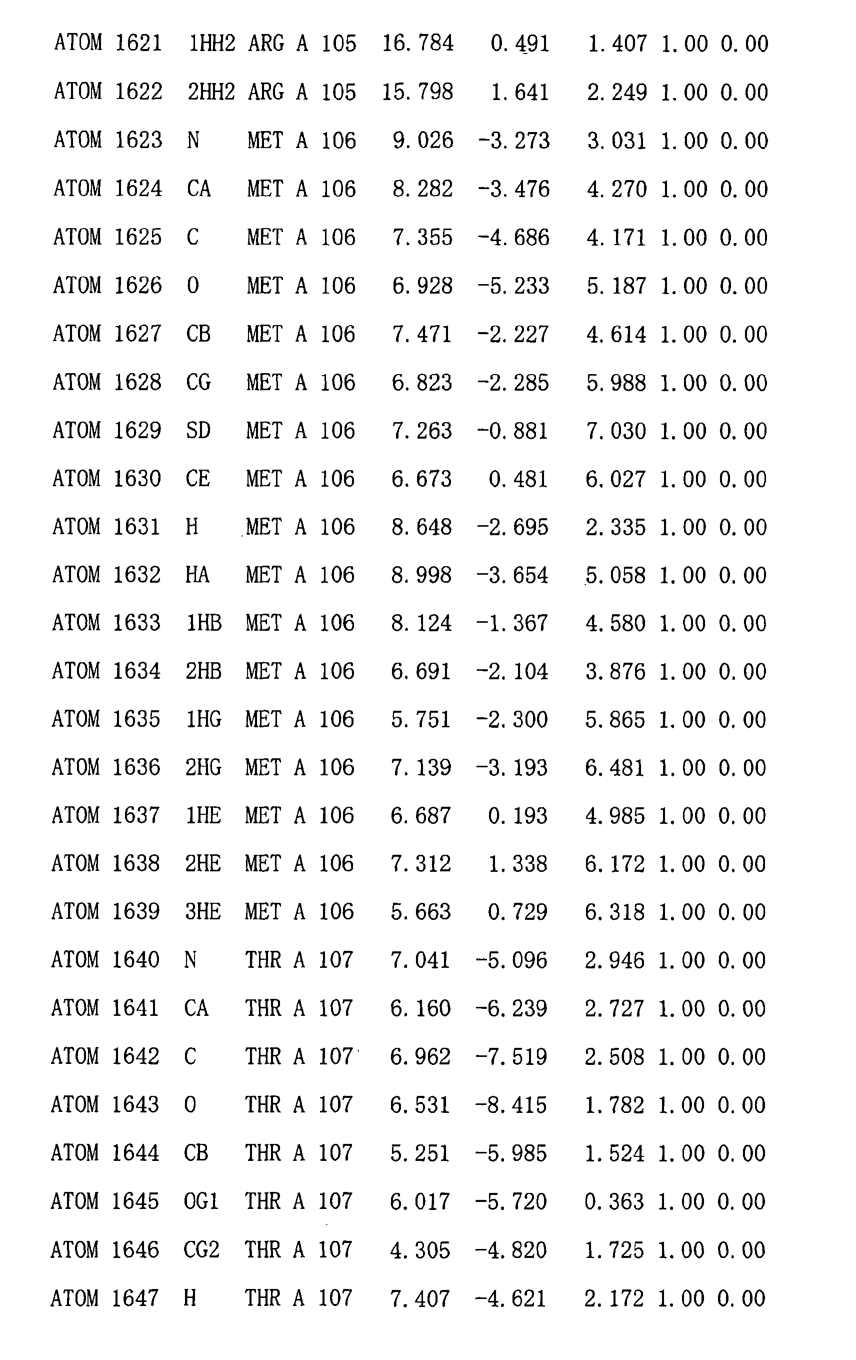

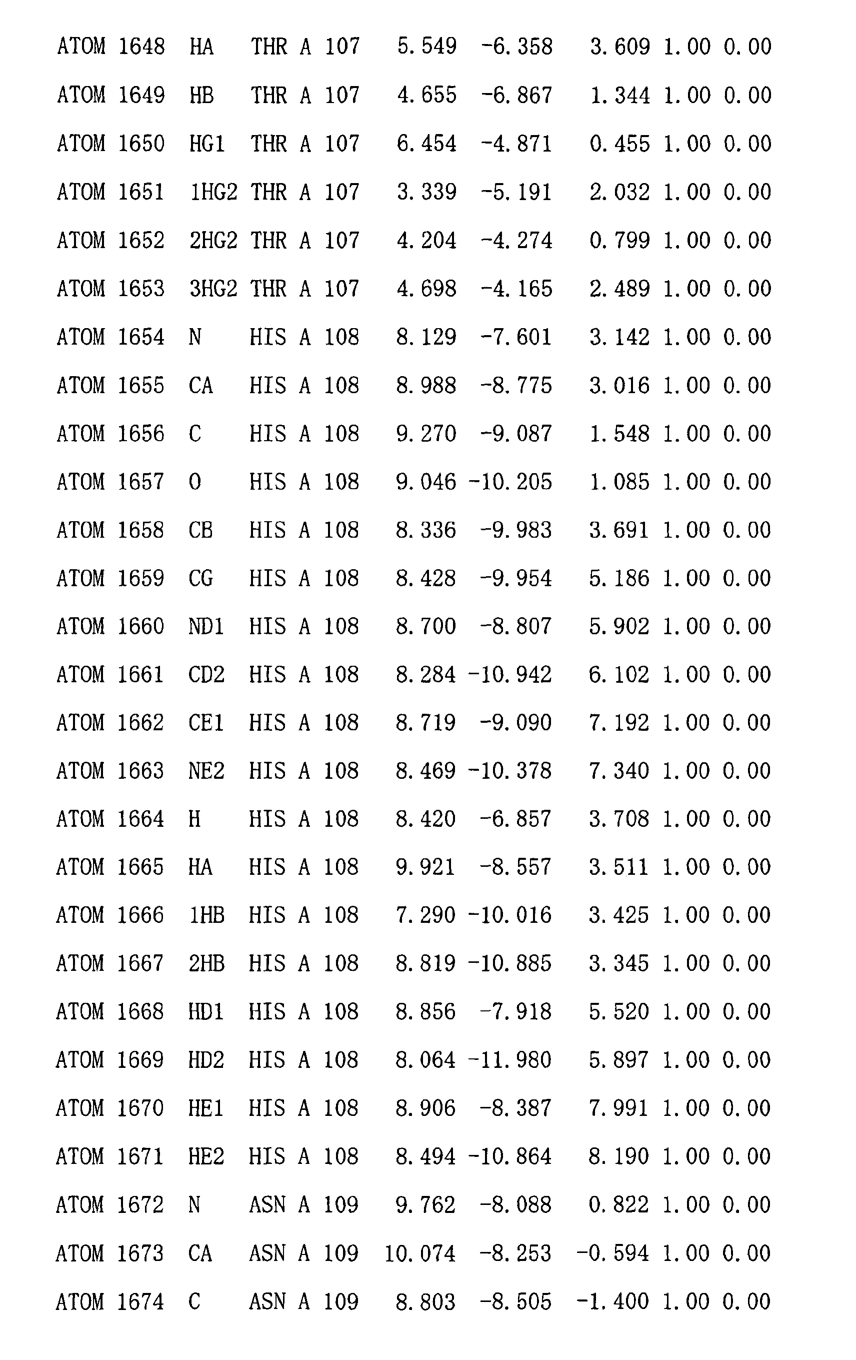

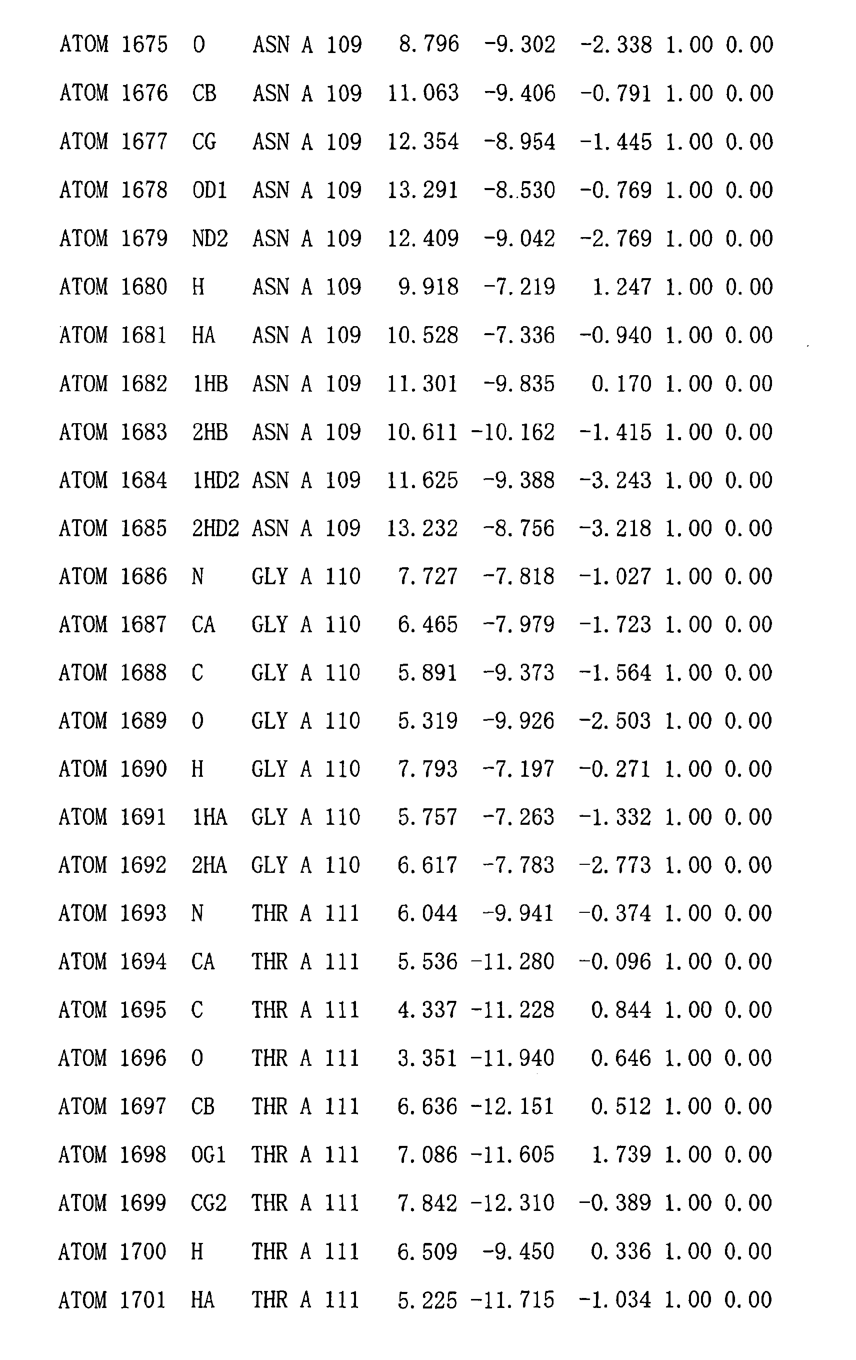

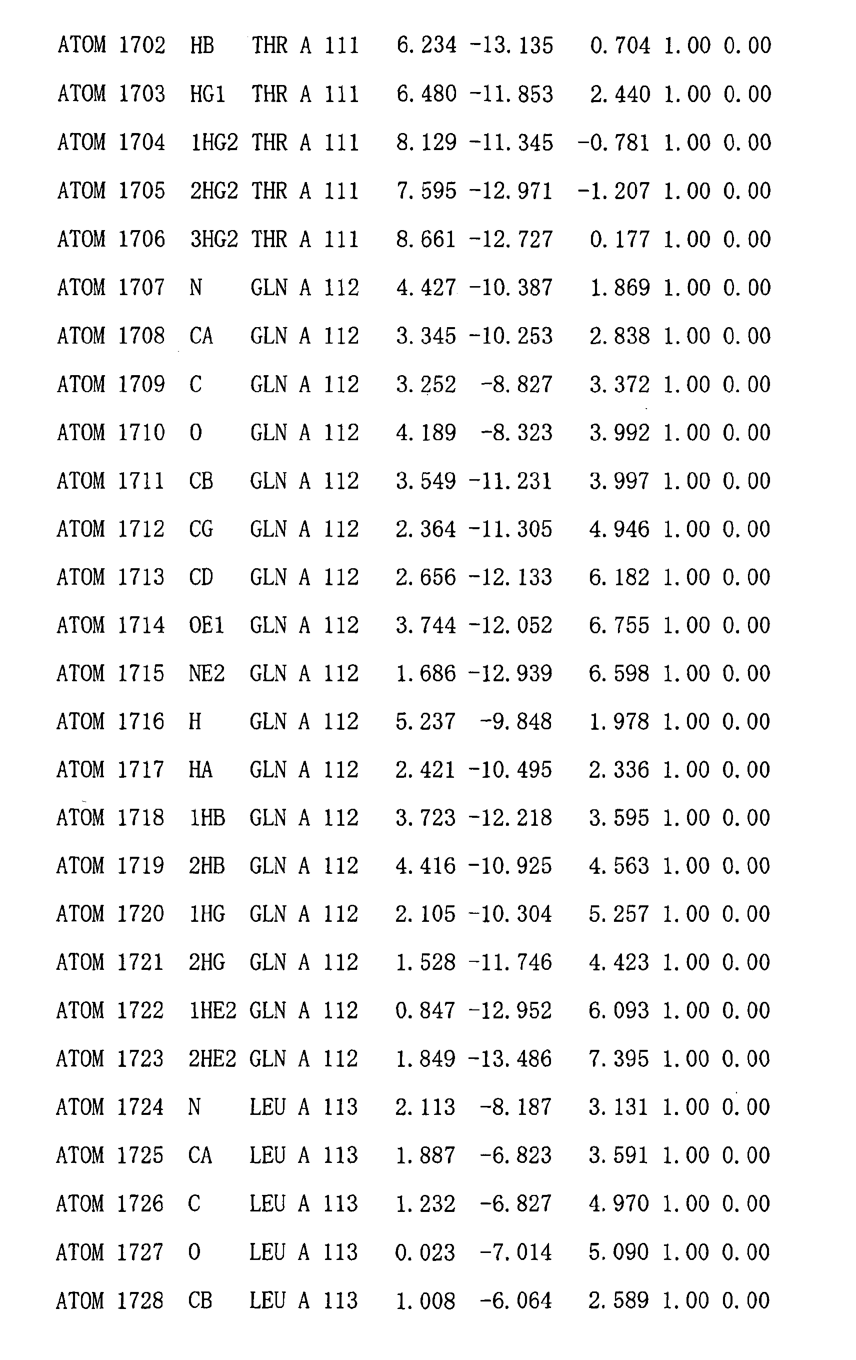

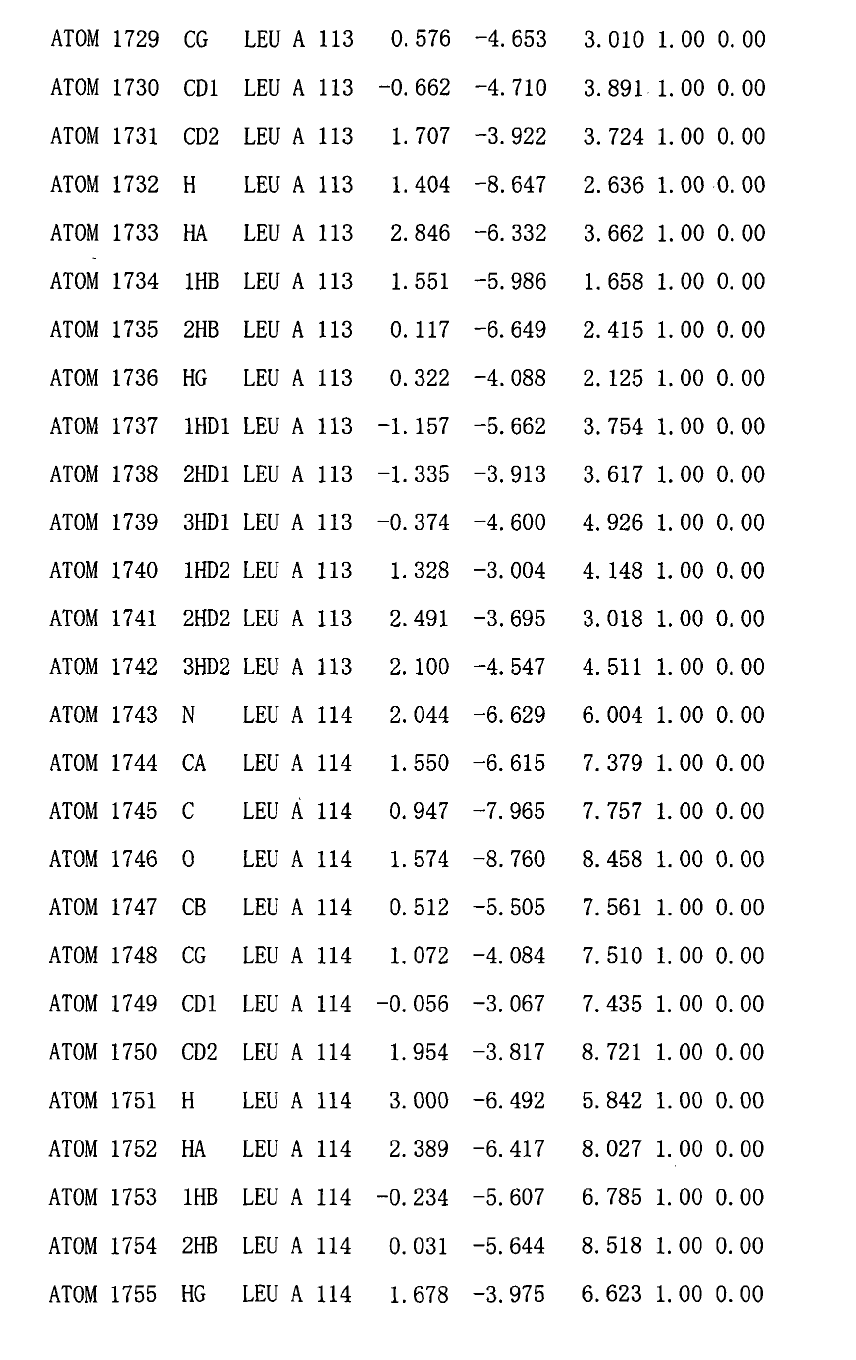

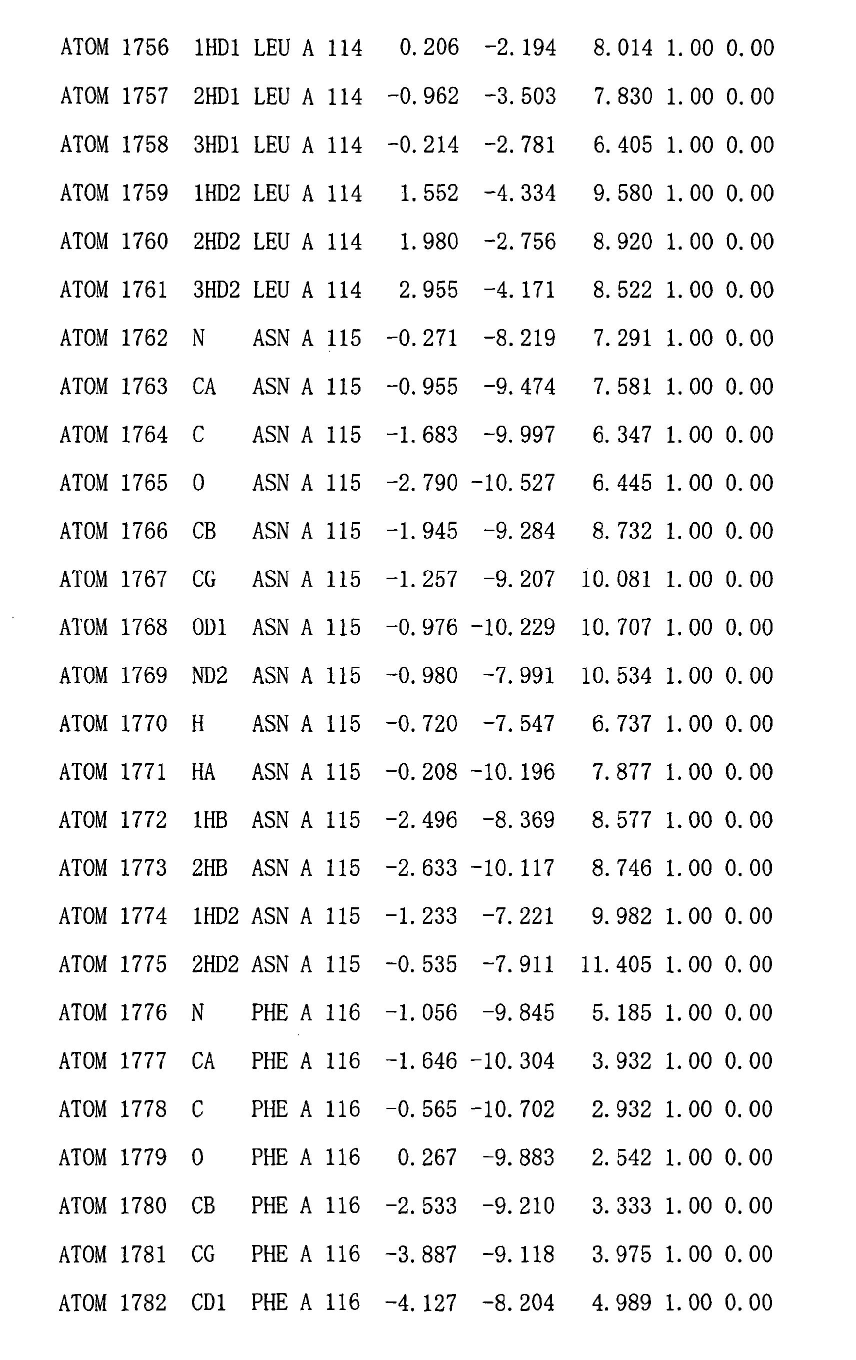

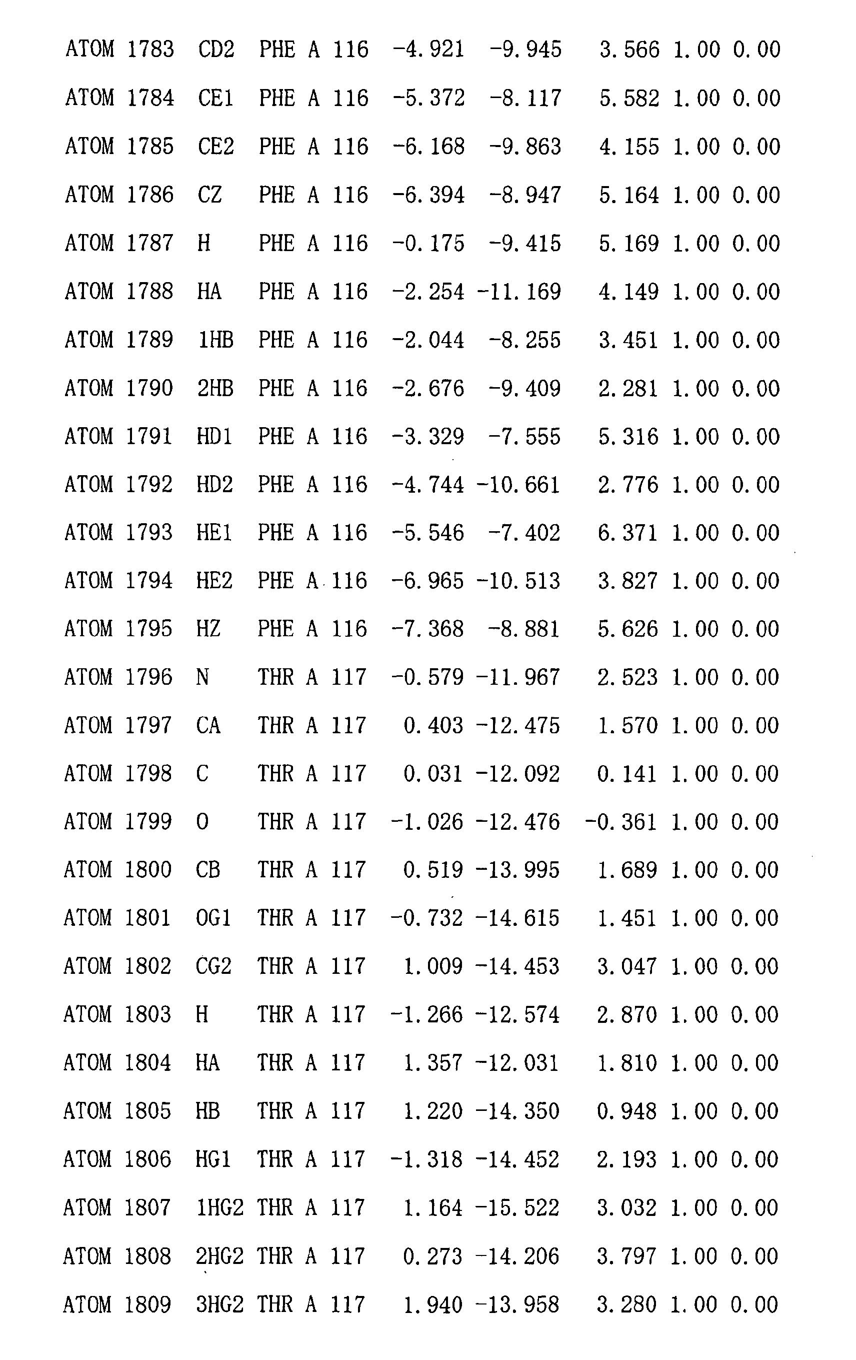

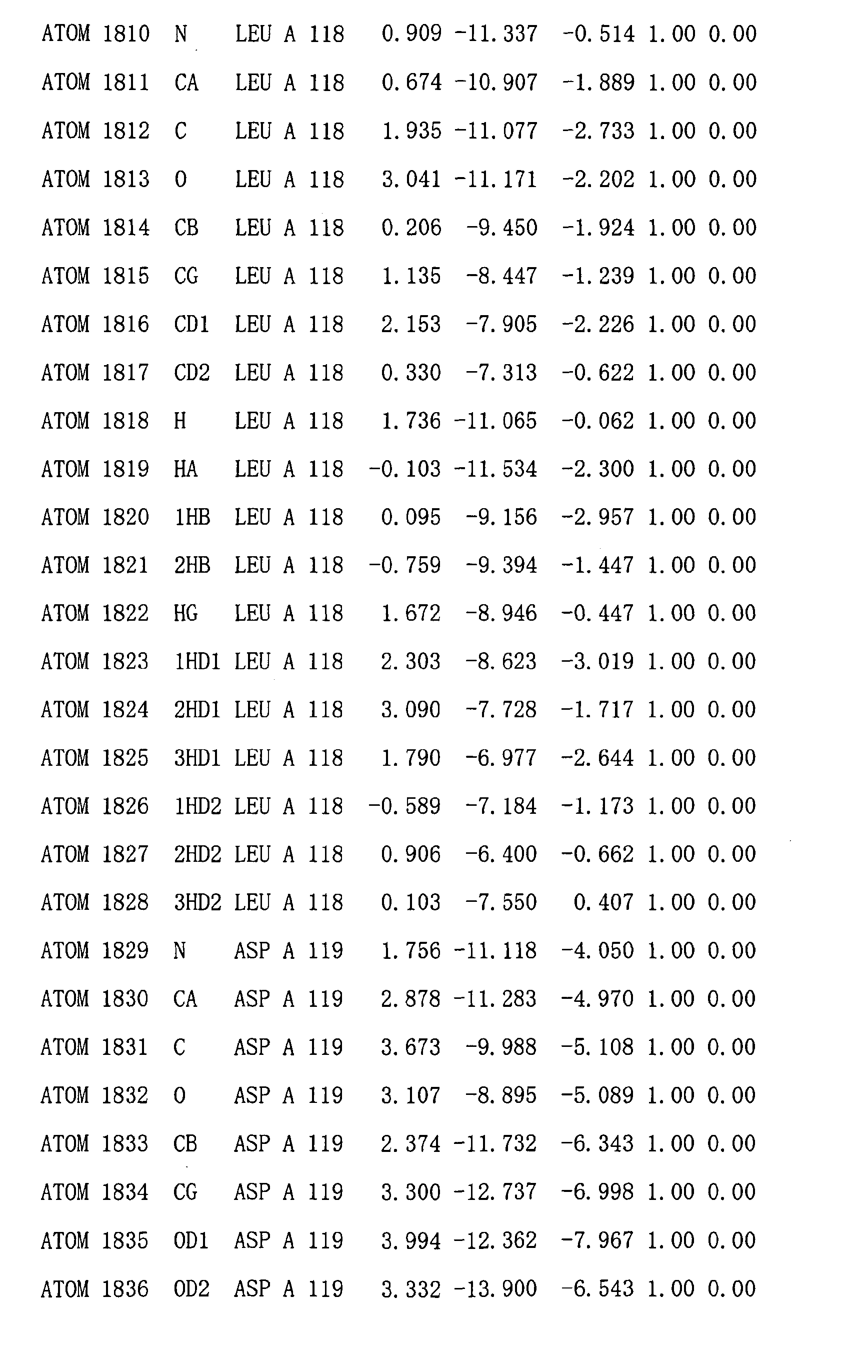

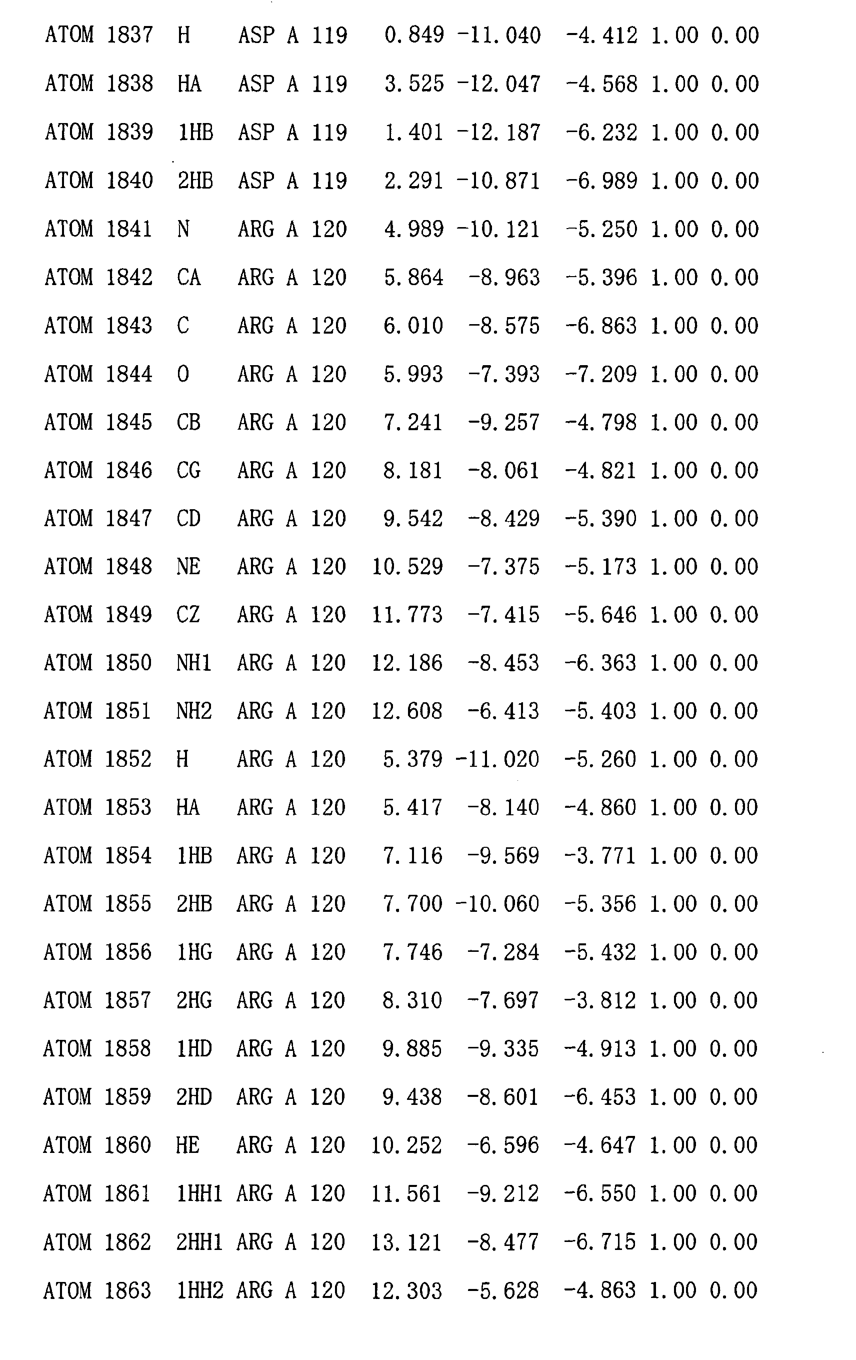

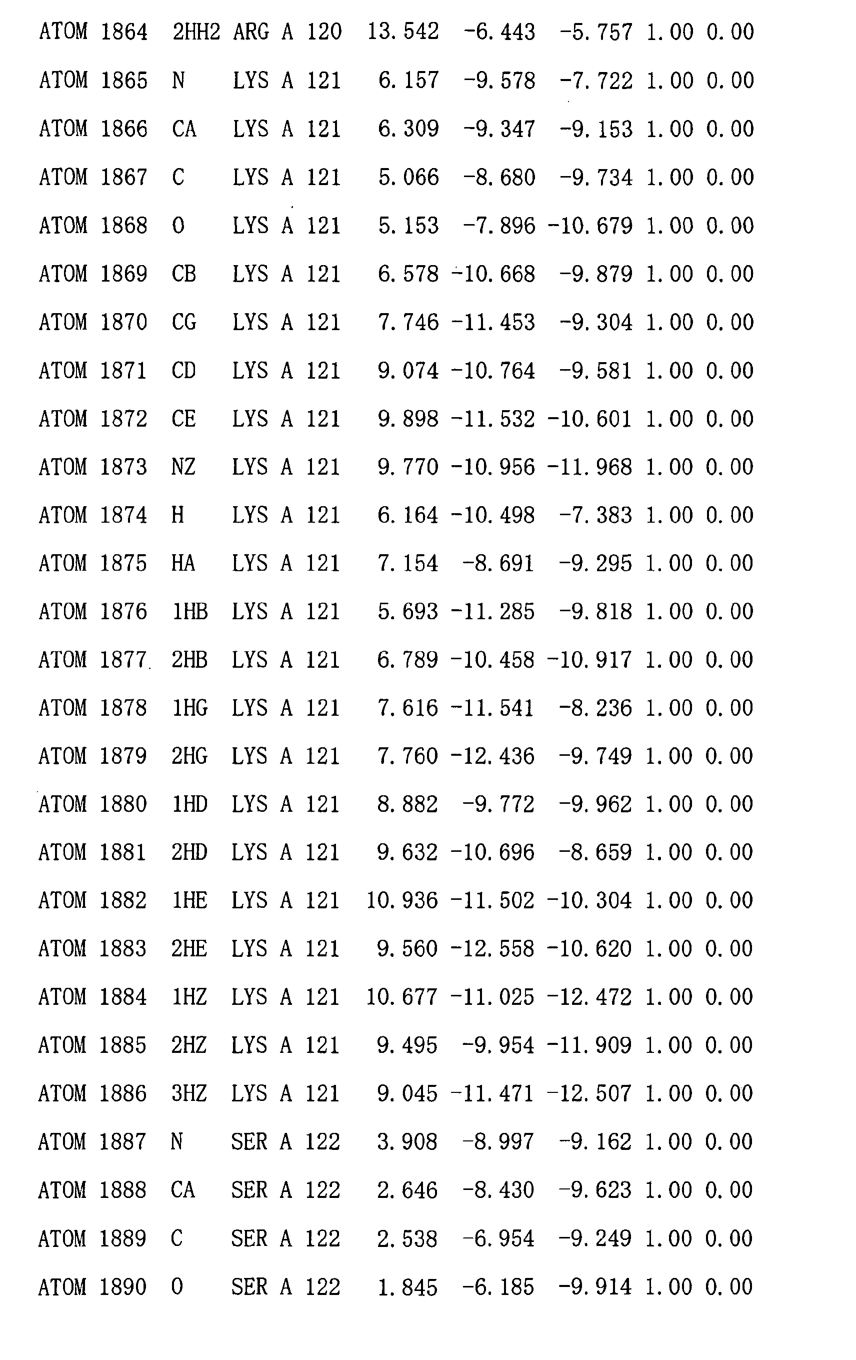

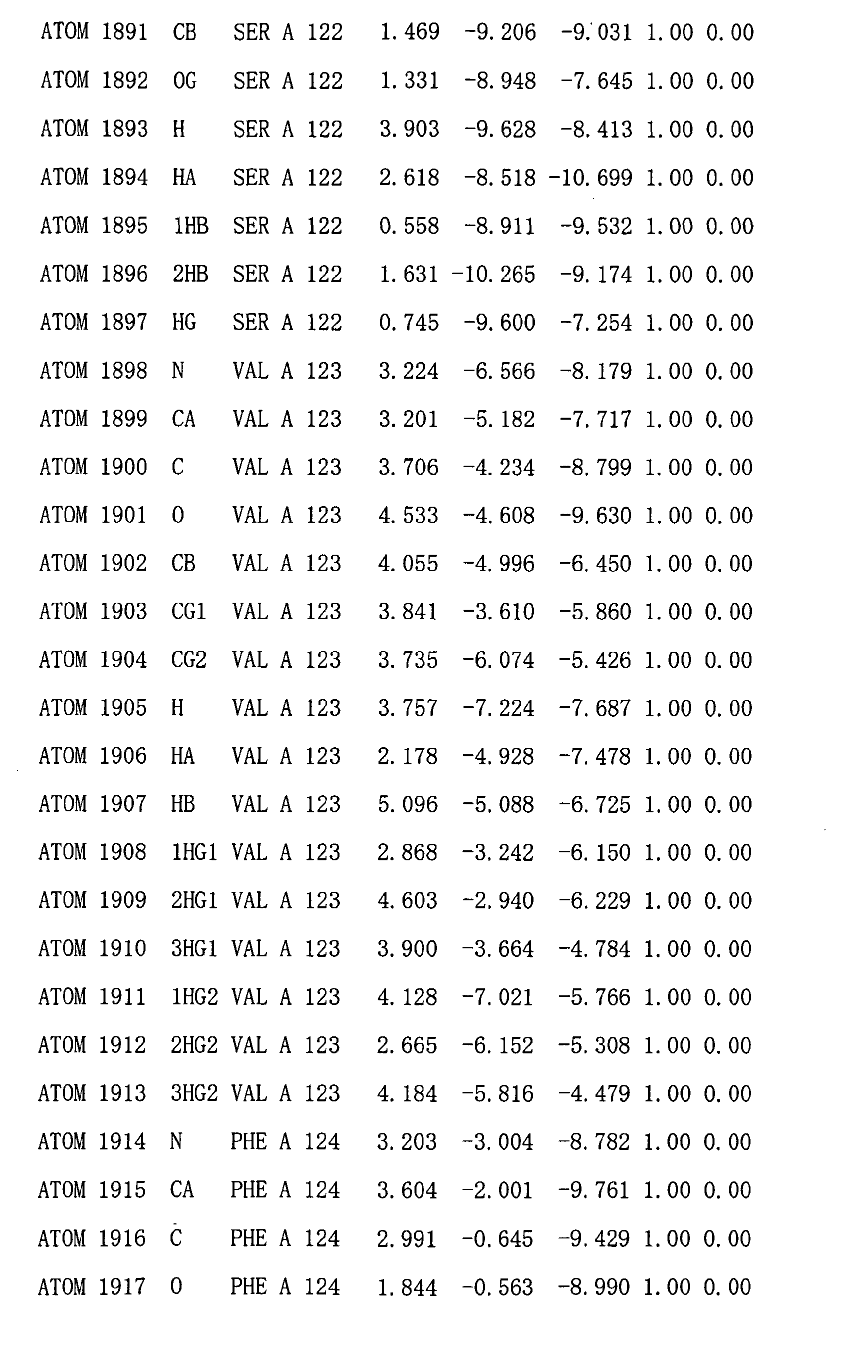

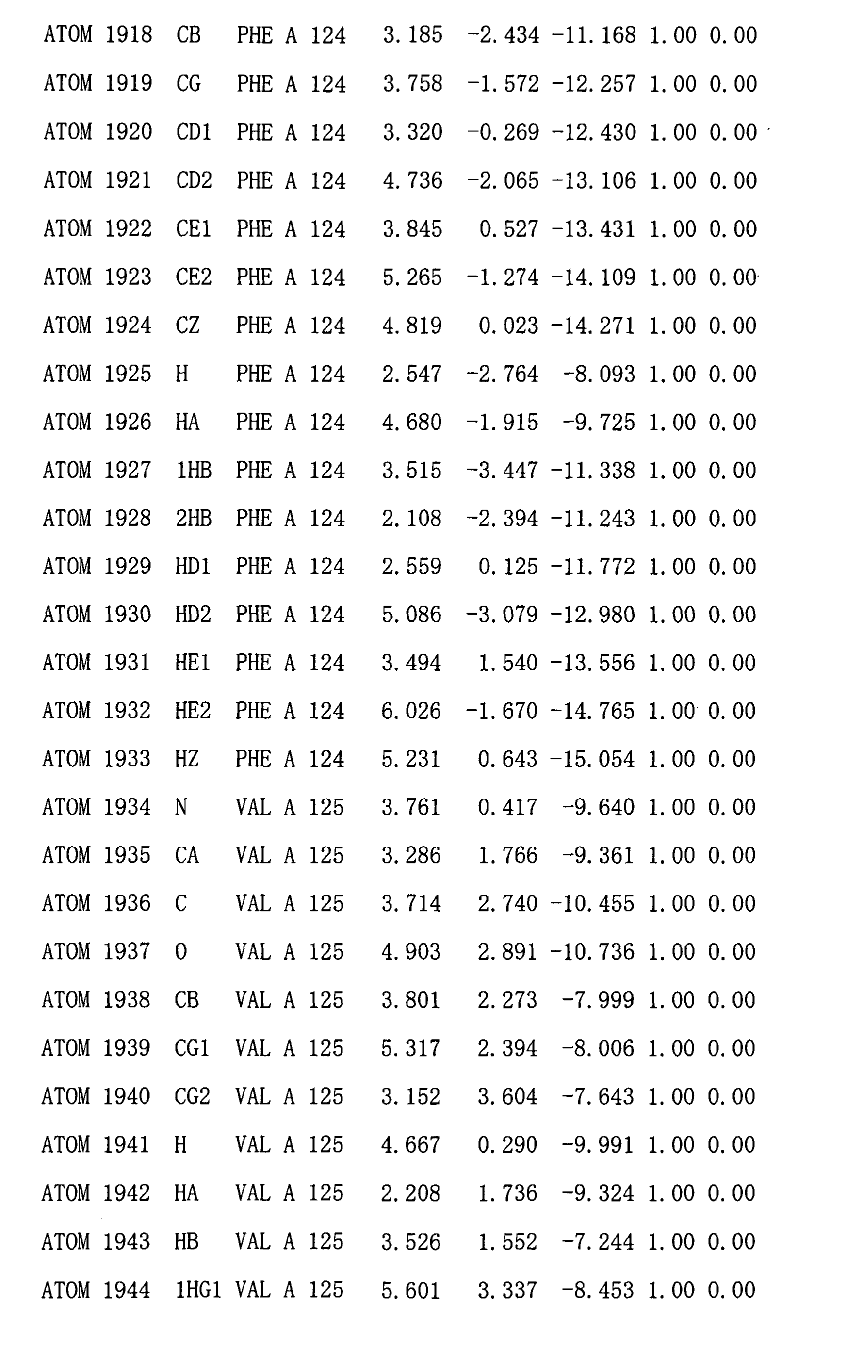

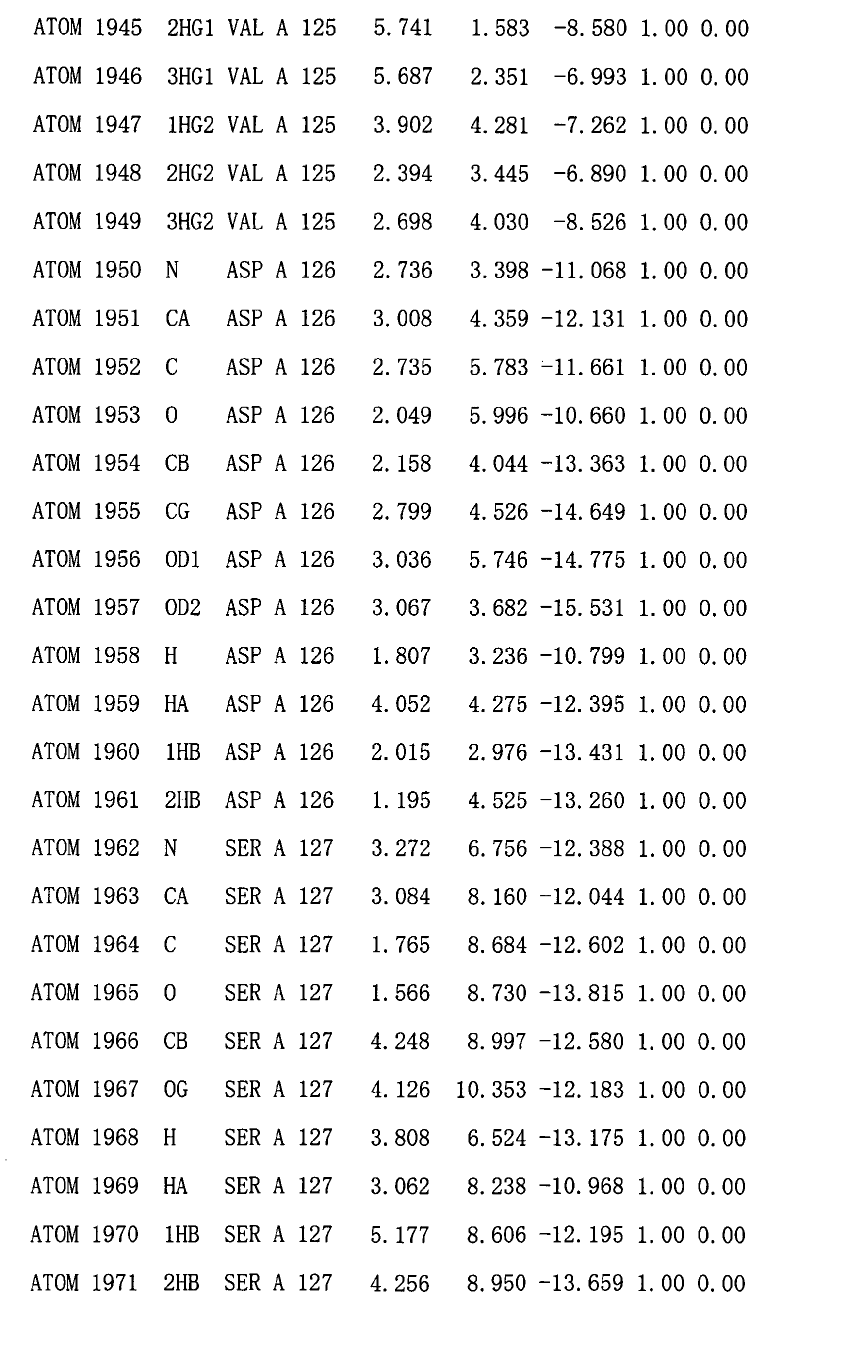

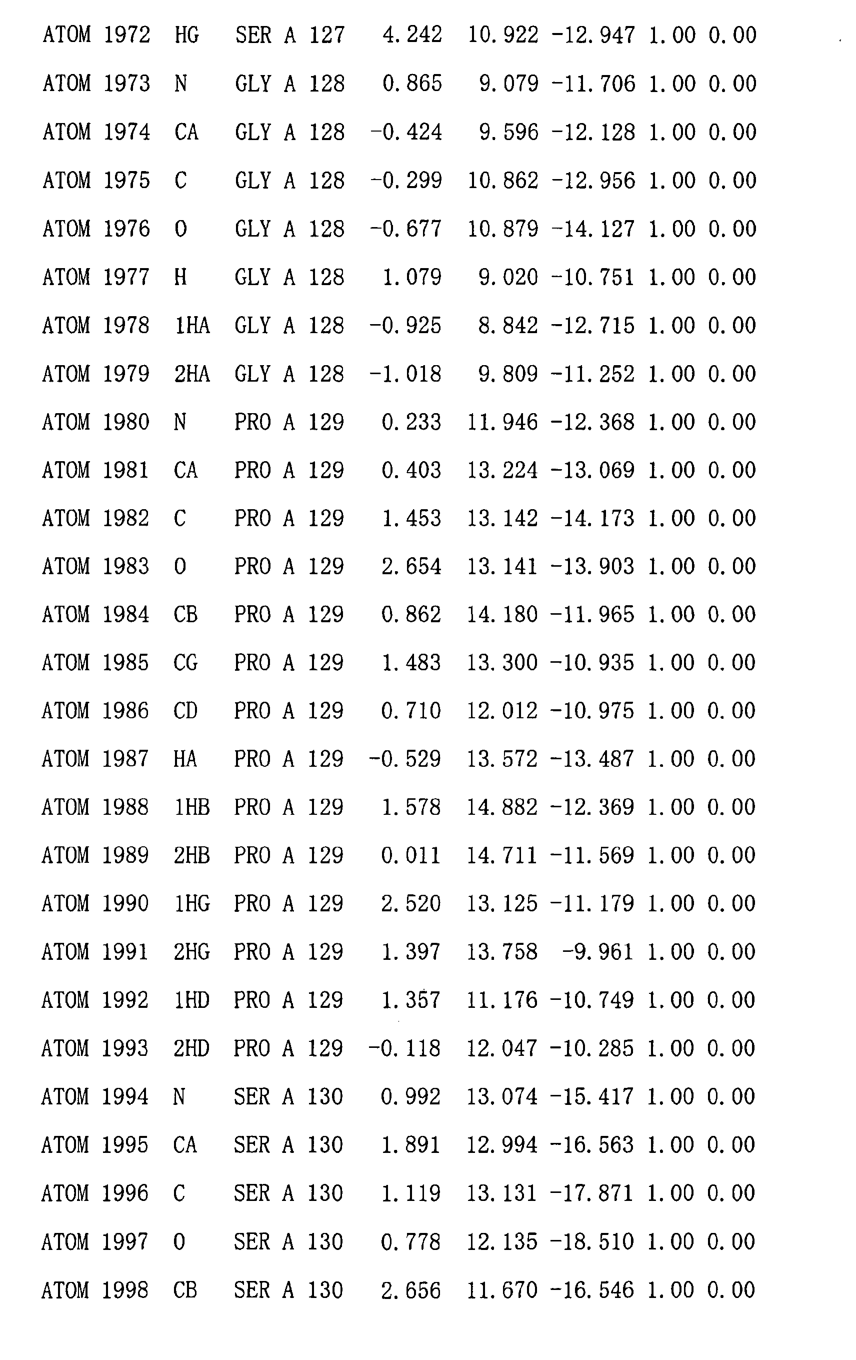

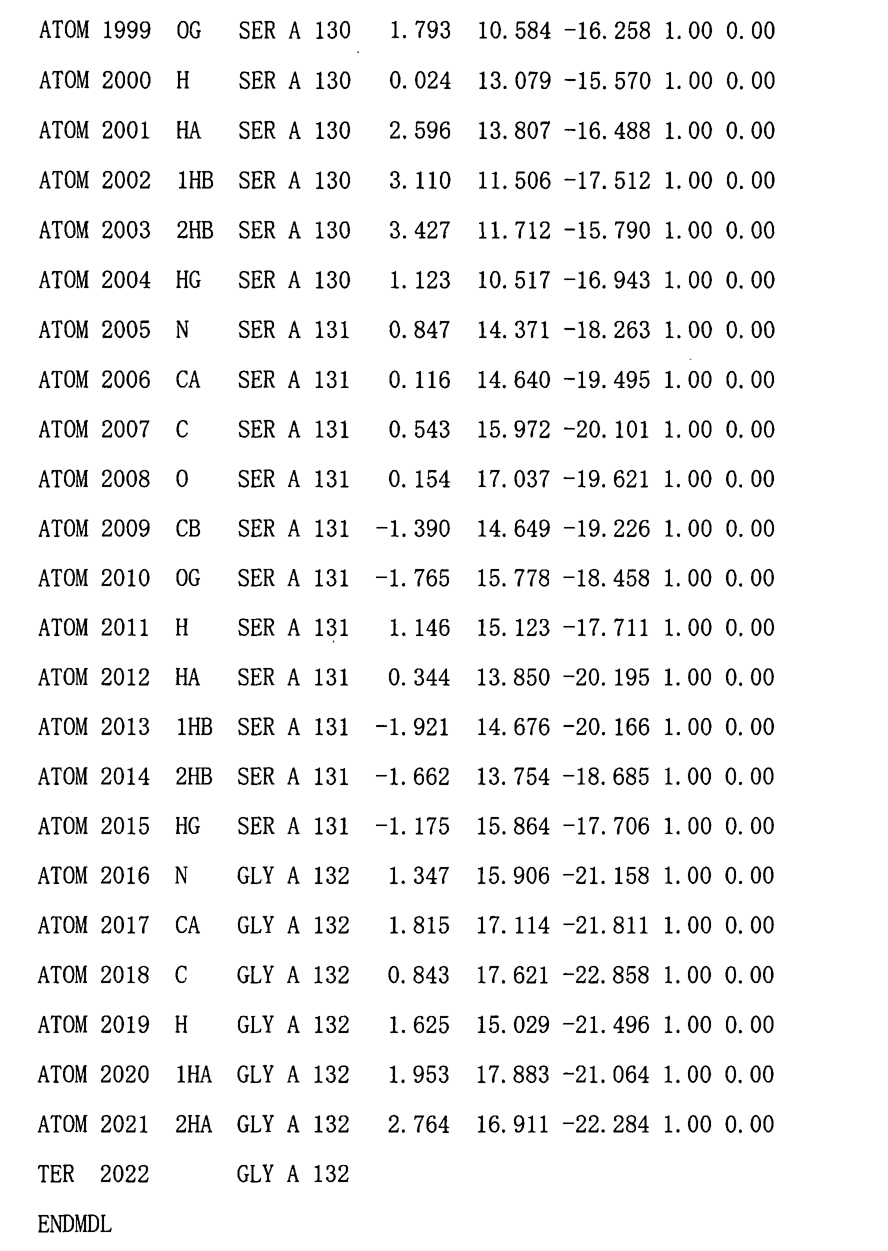

### Three-Dimensional Structure Coordinate 10

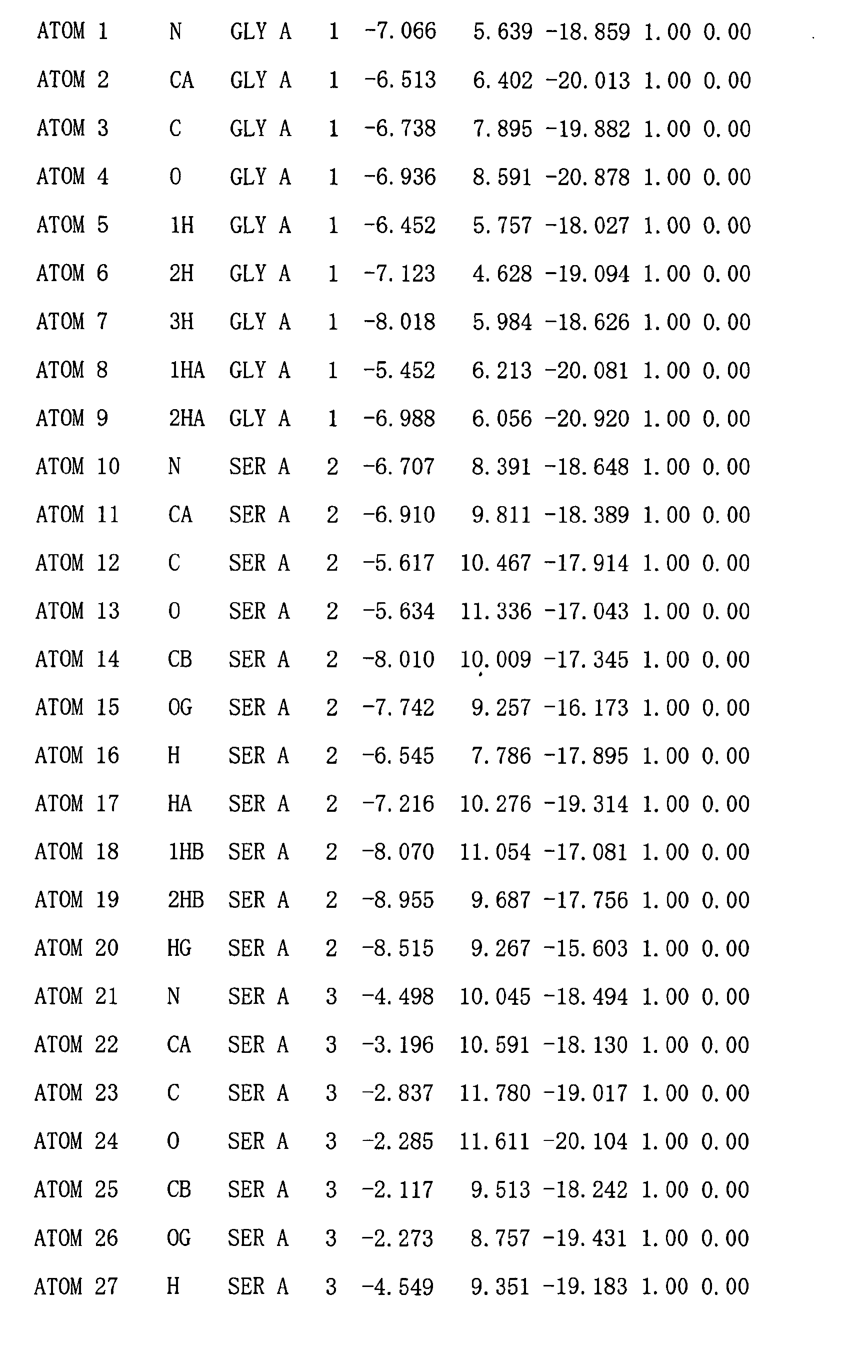

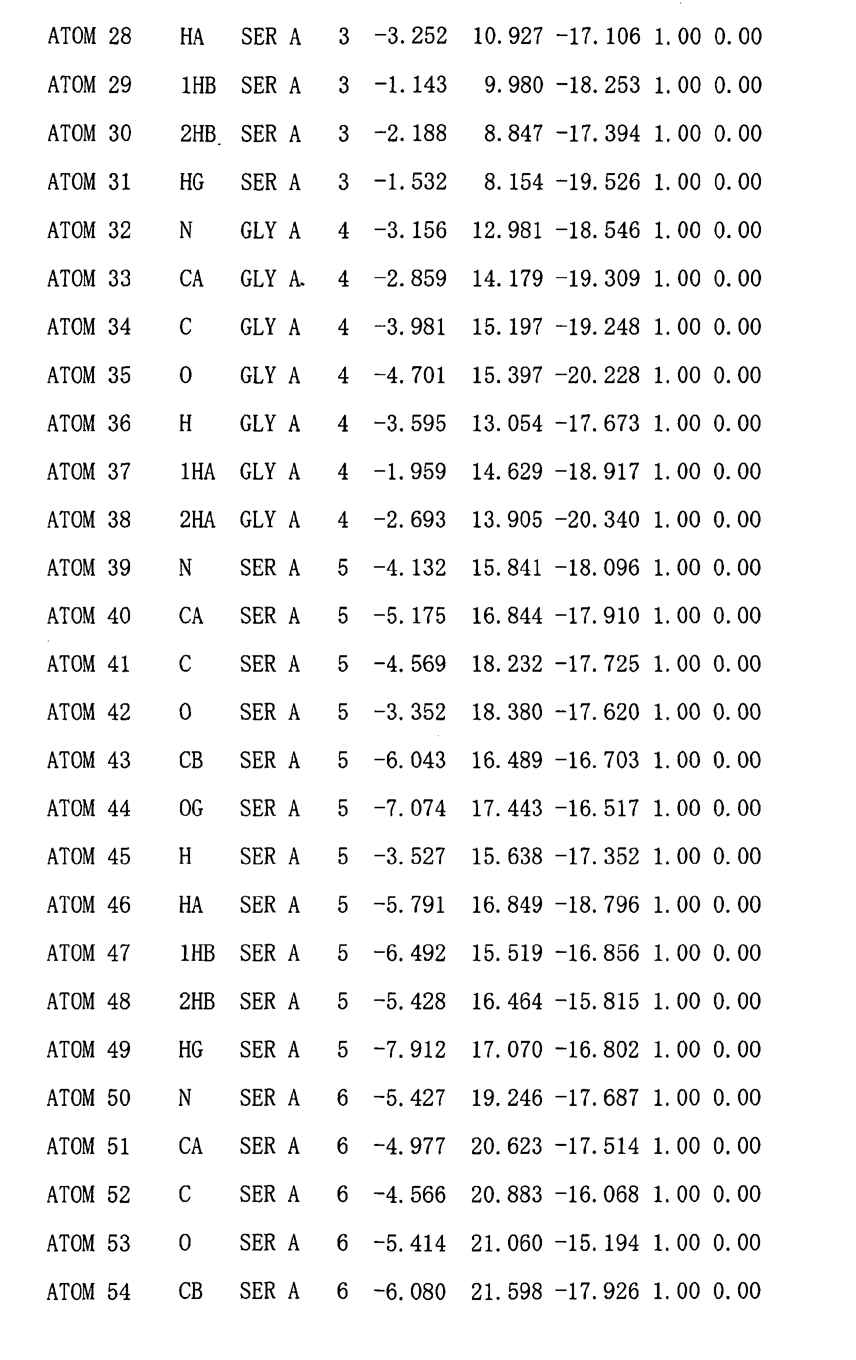

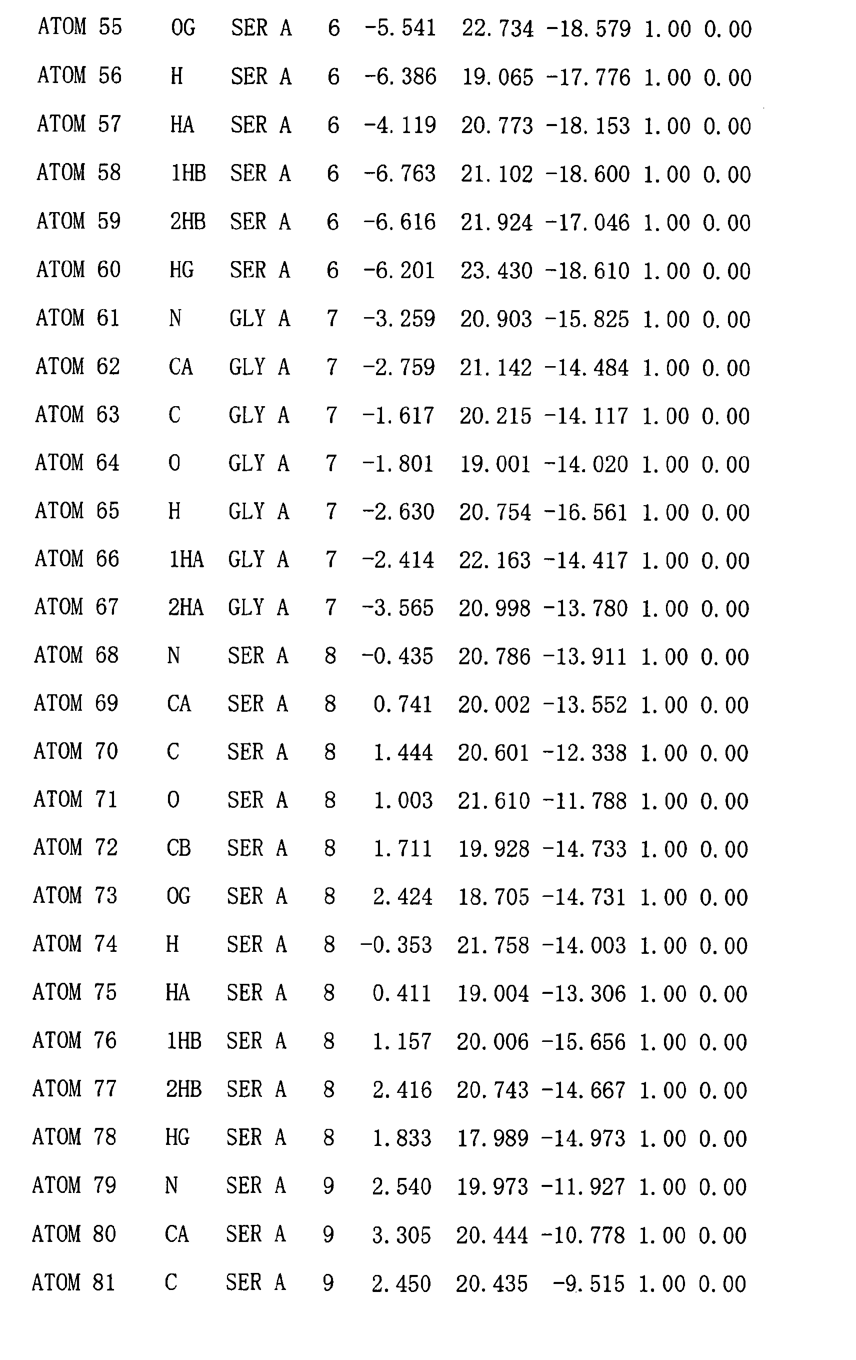

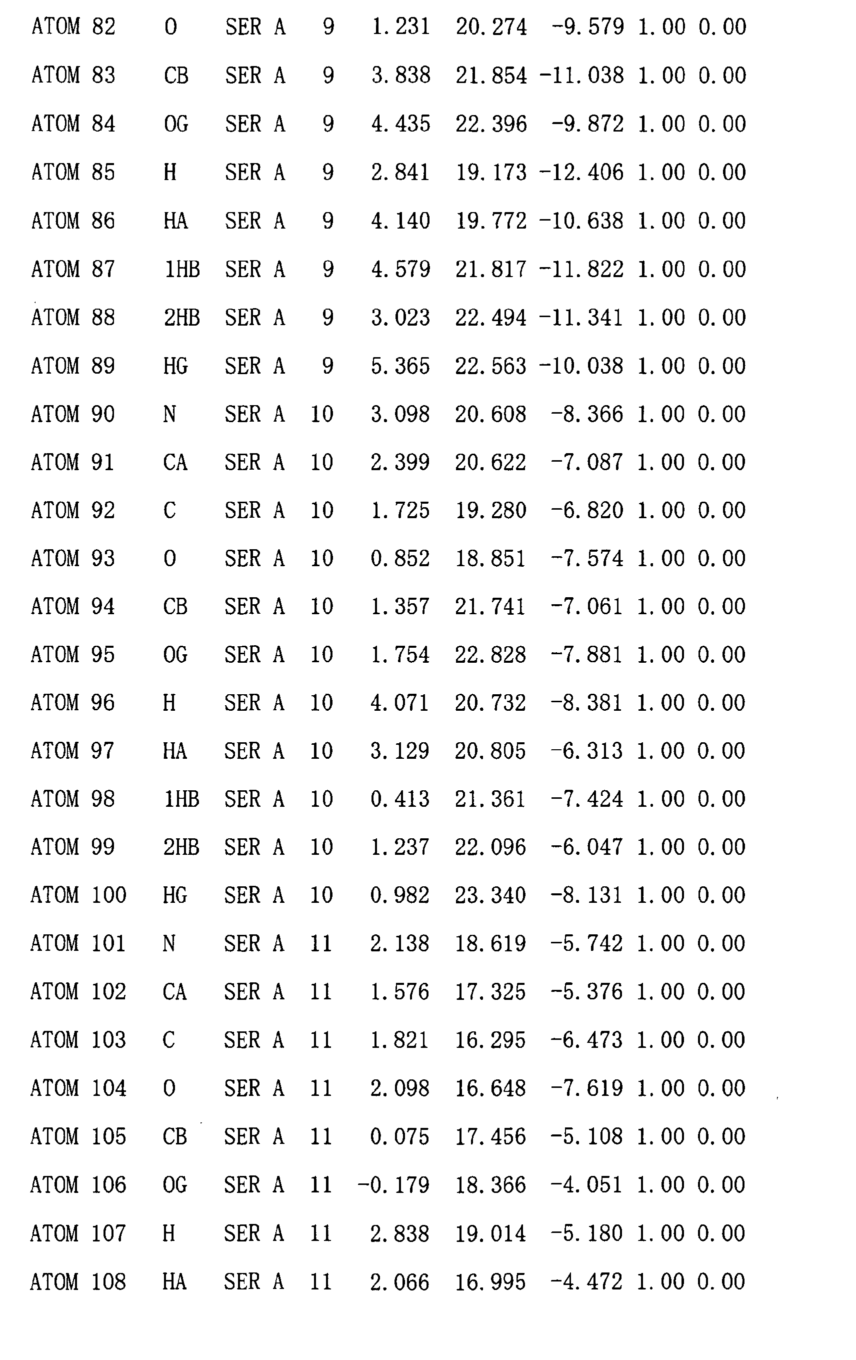

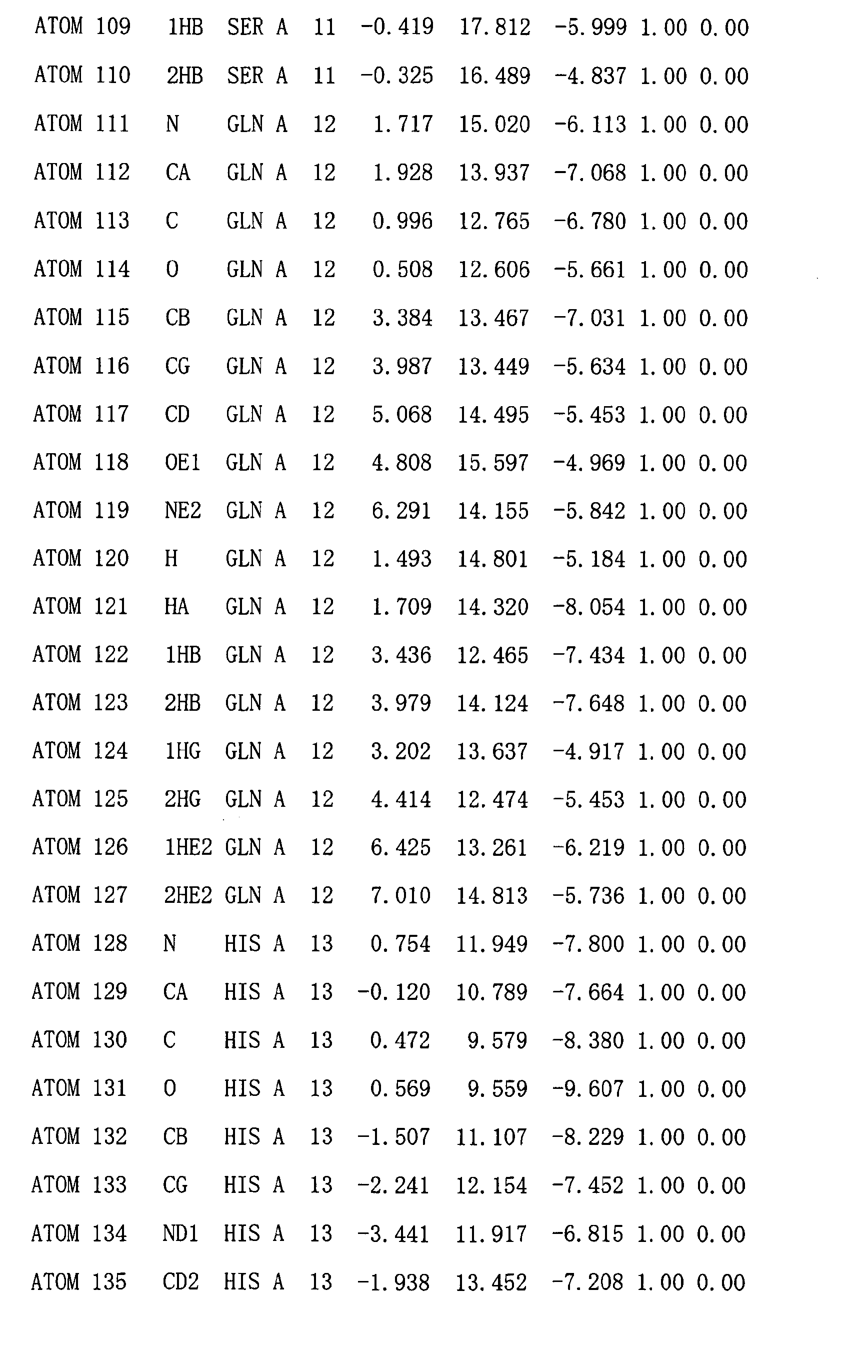

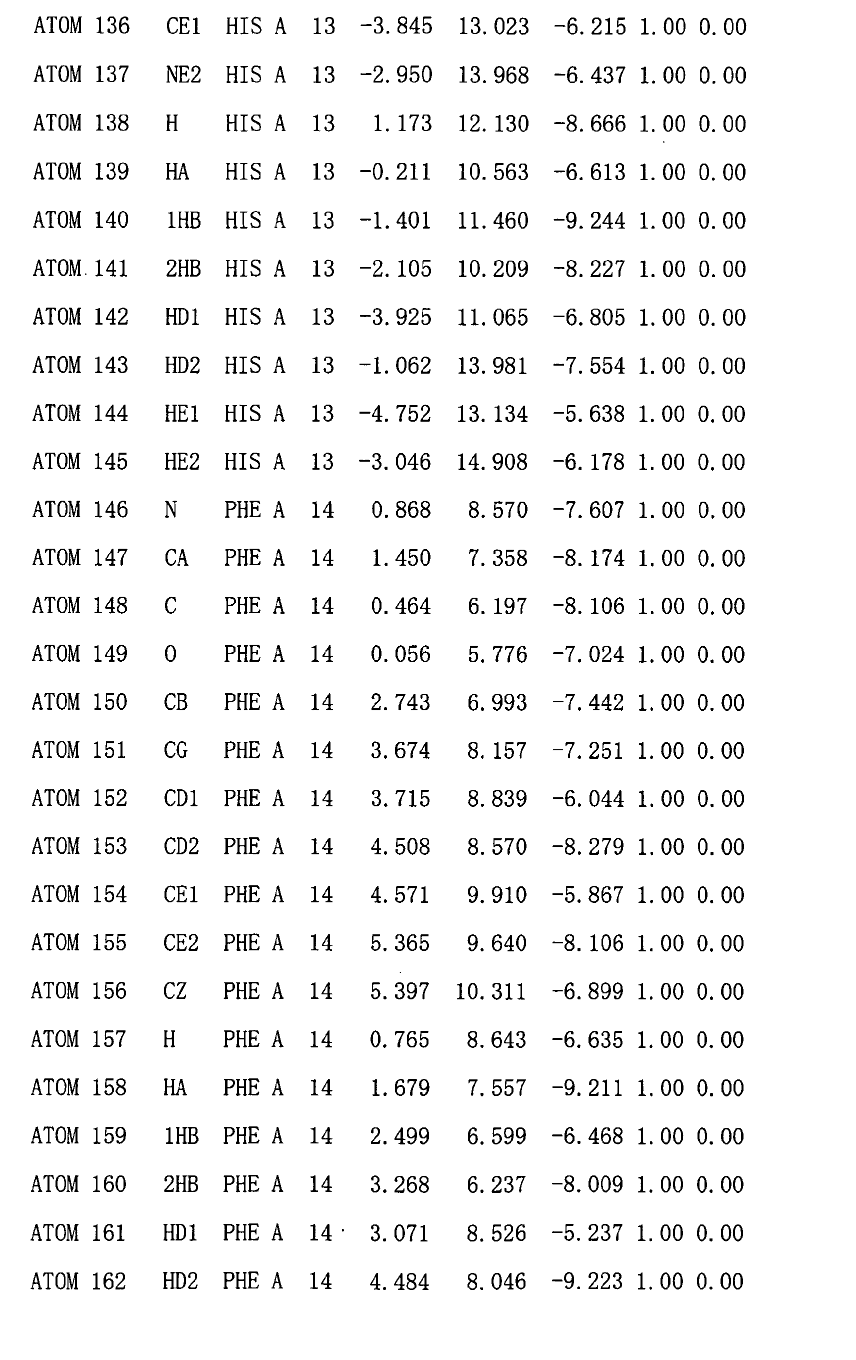

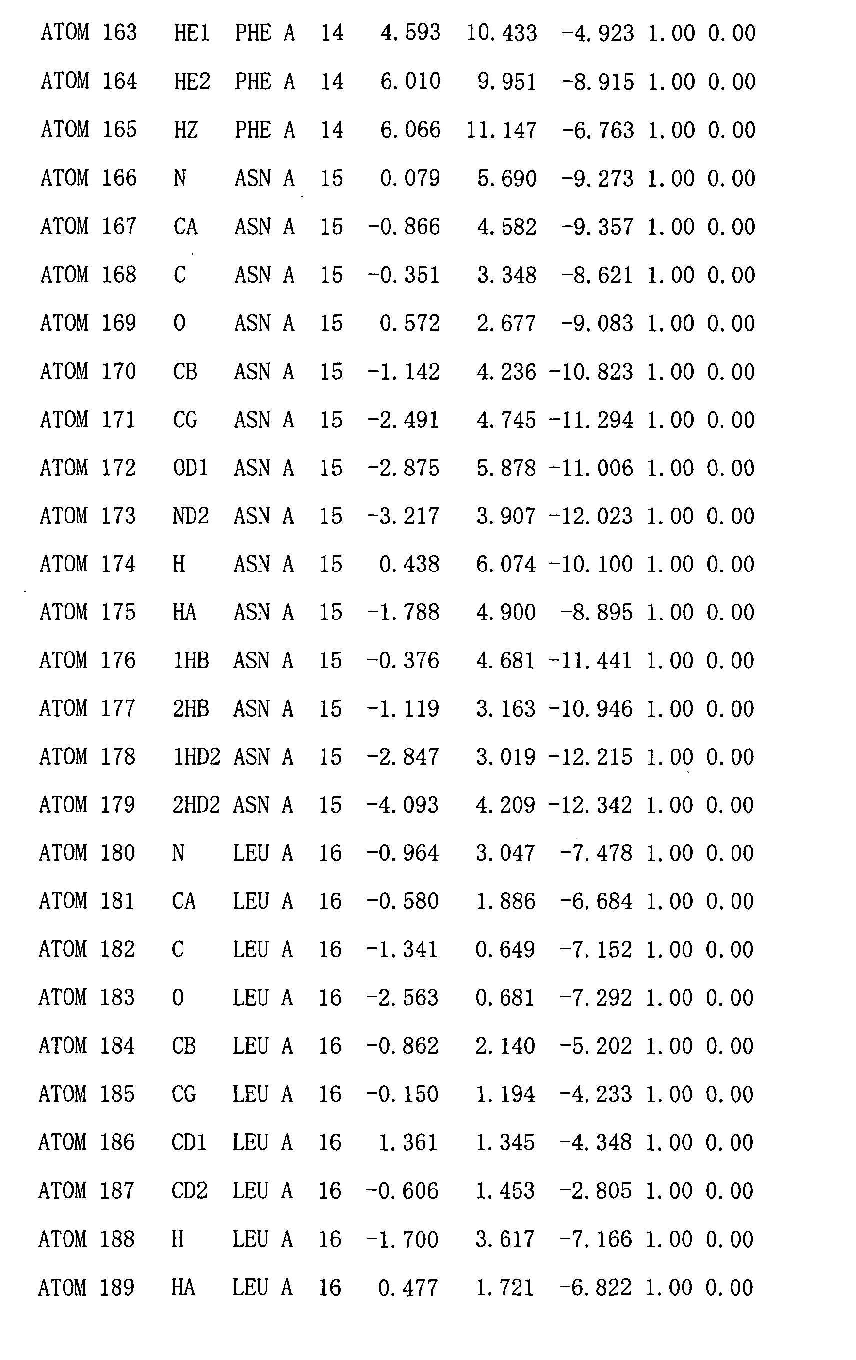

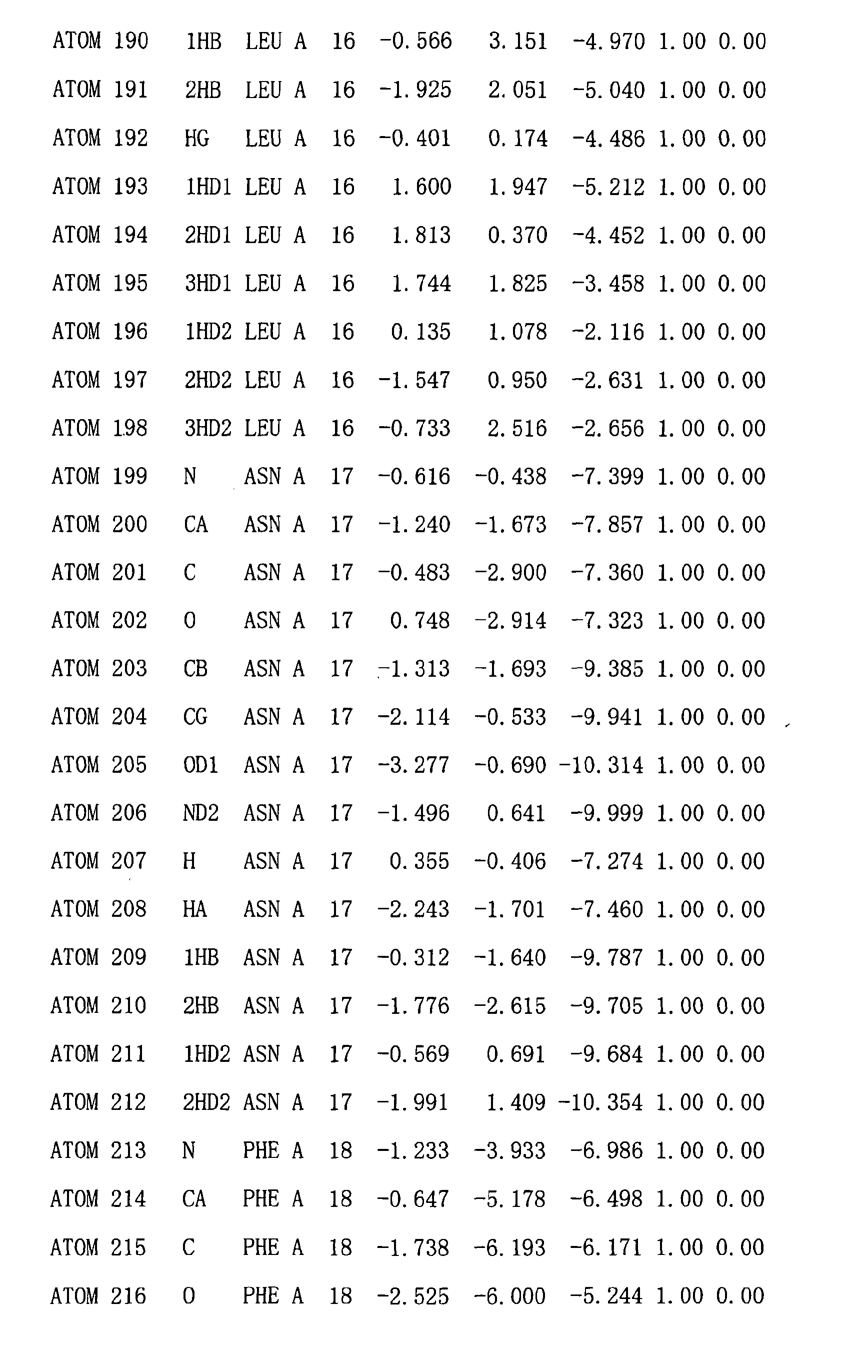

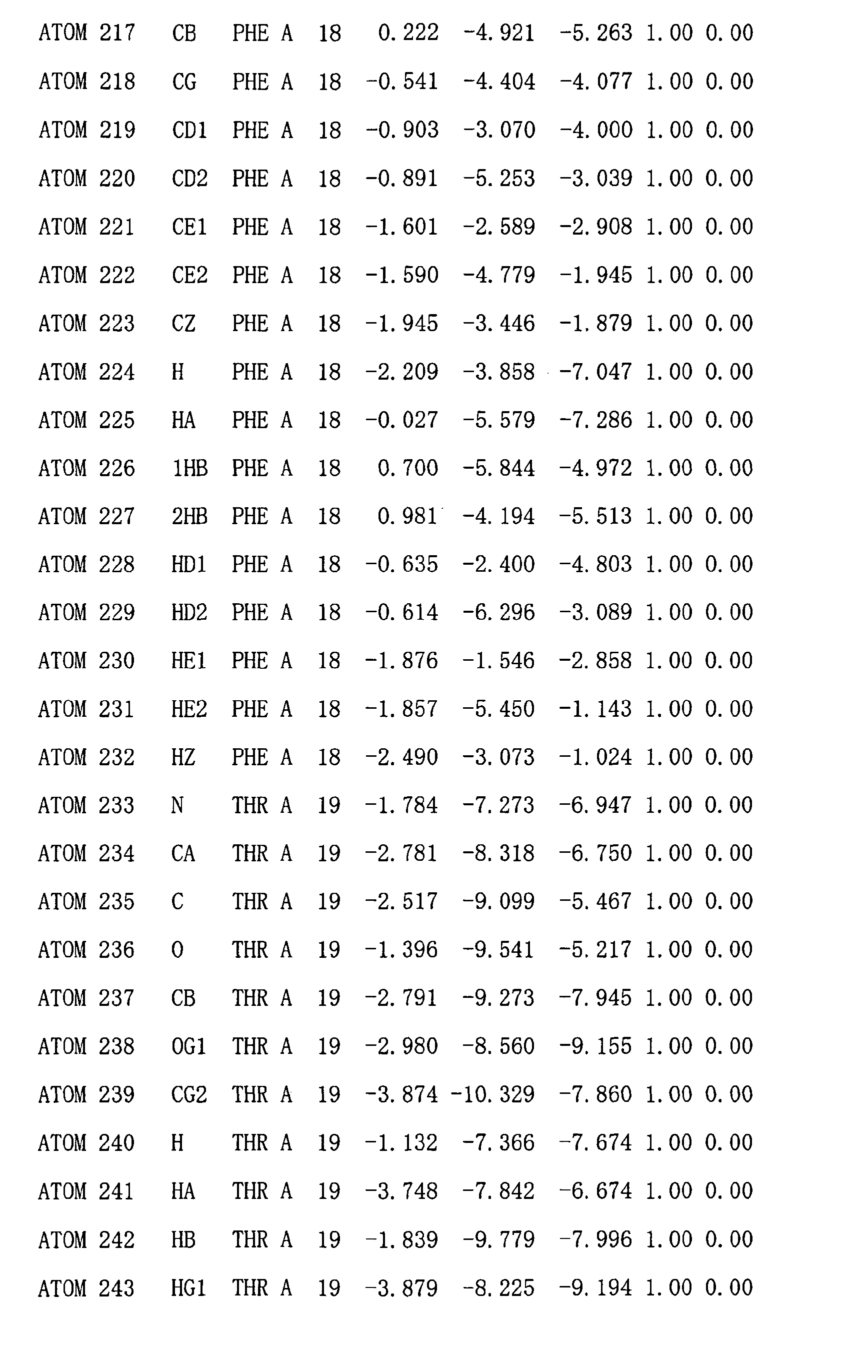

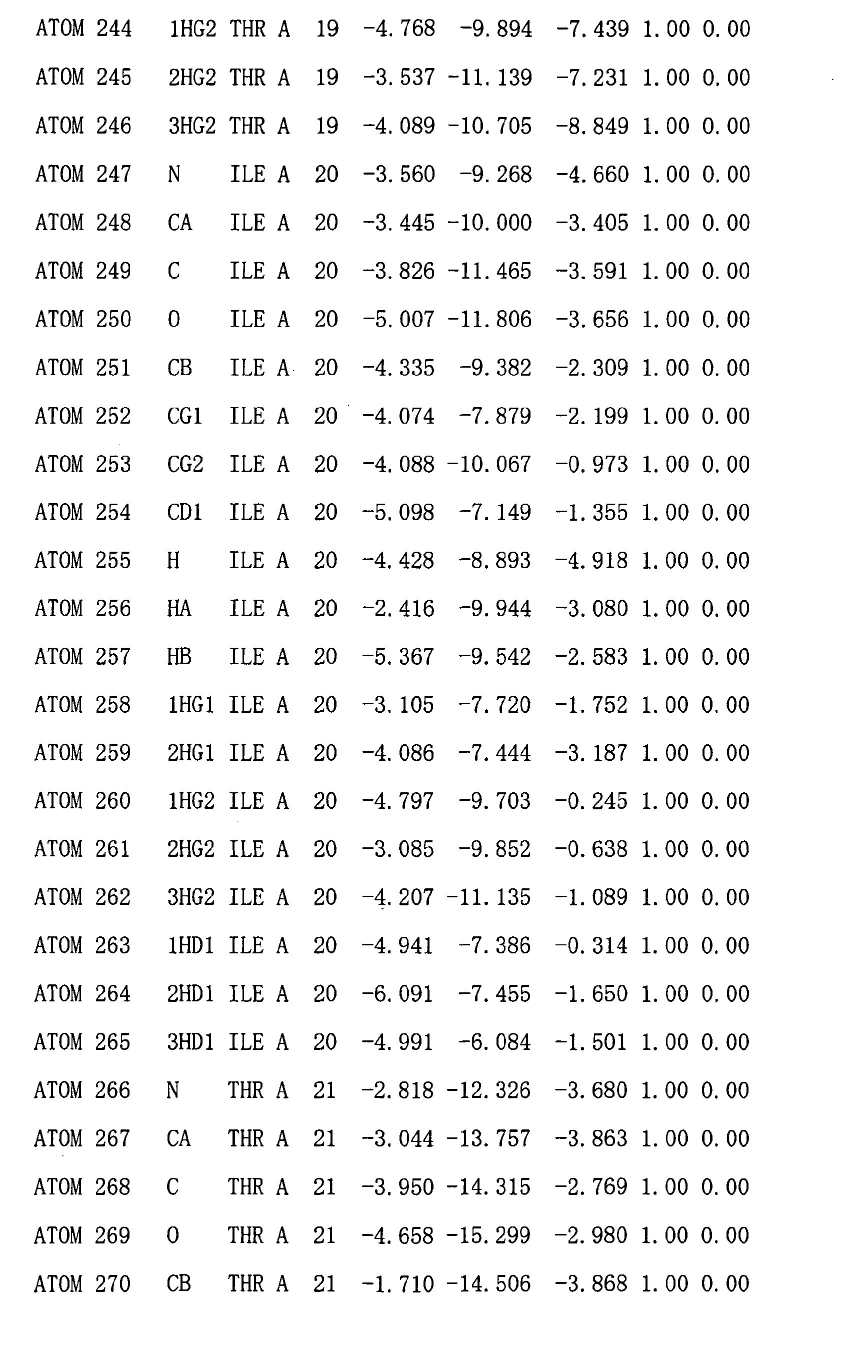

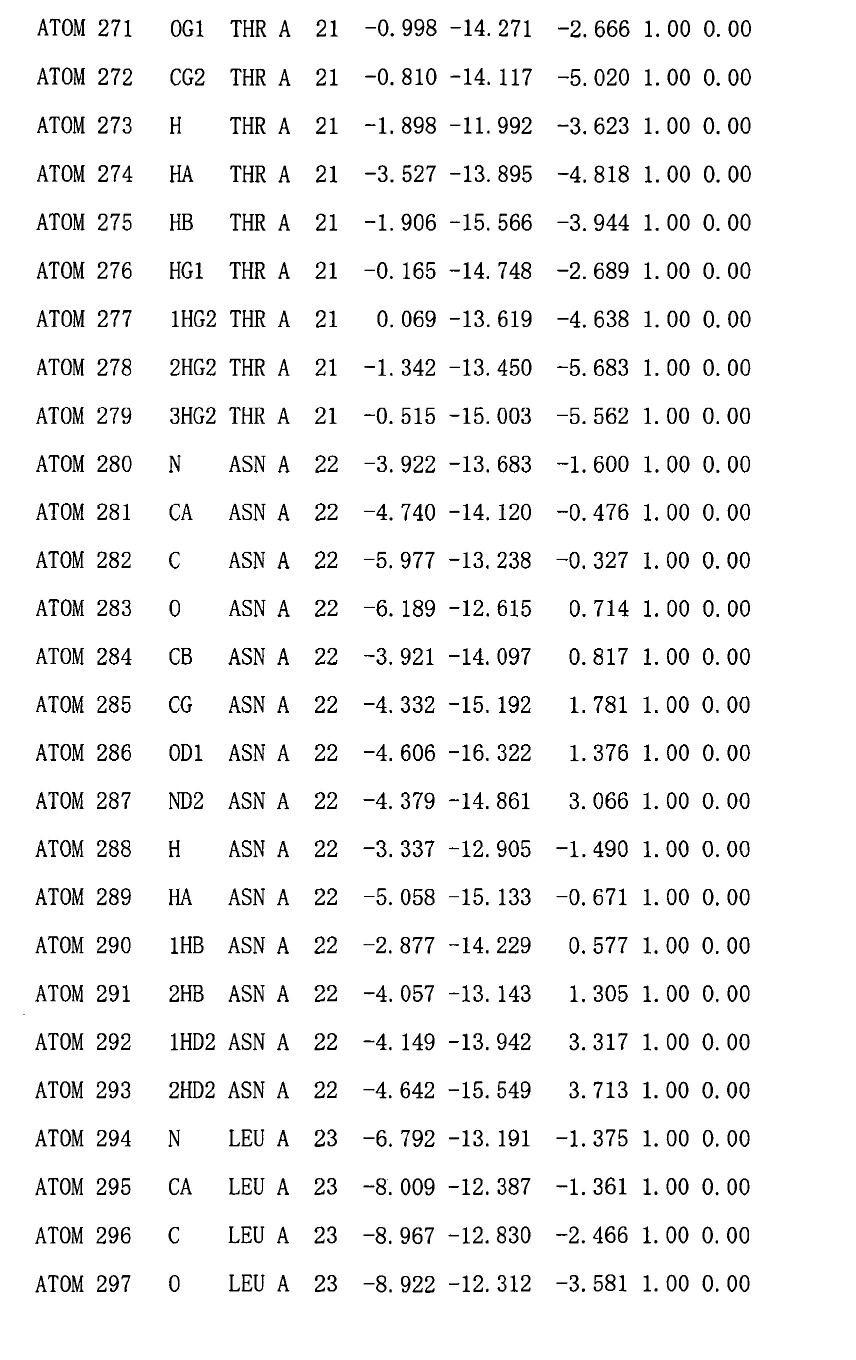

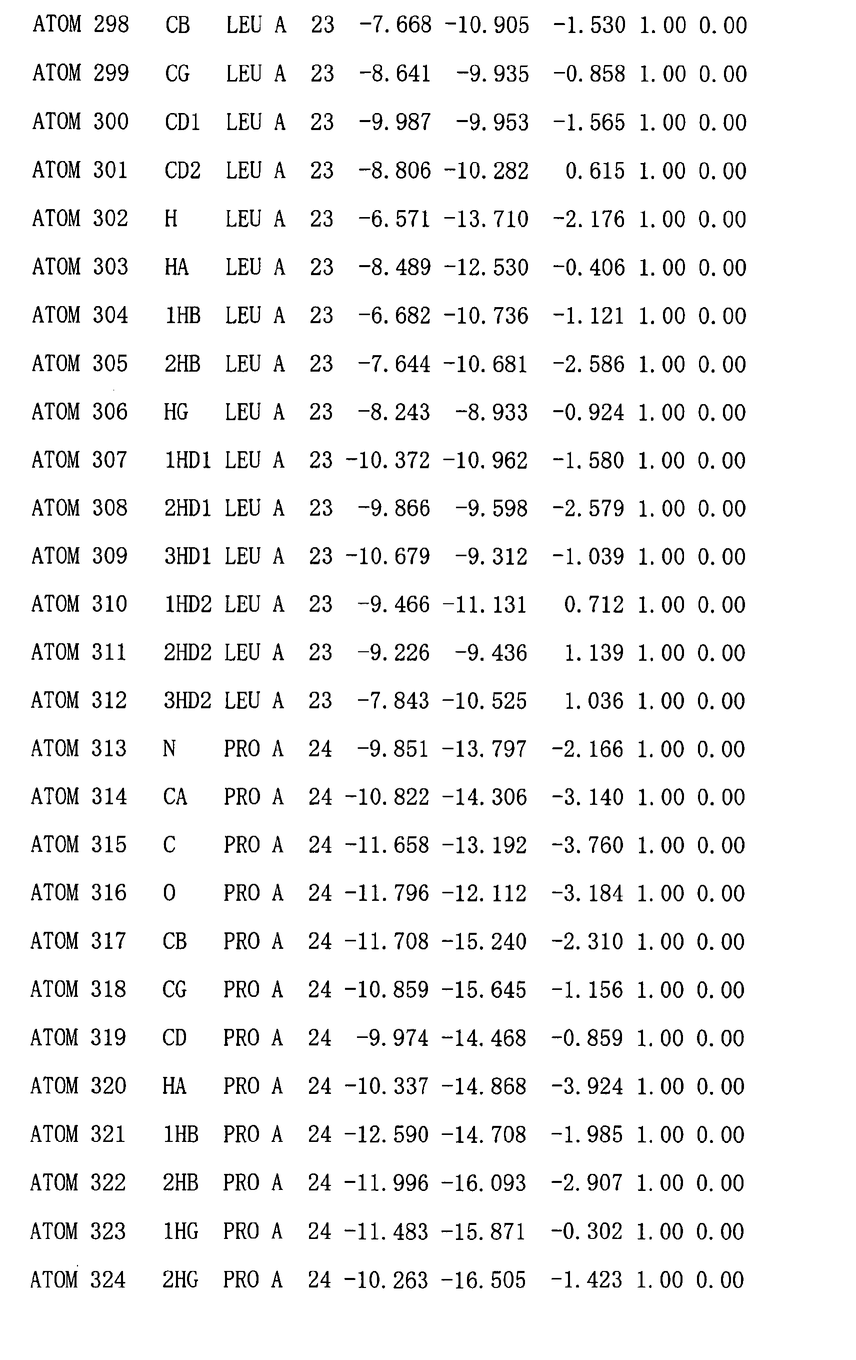

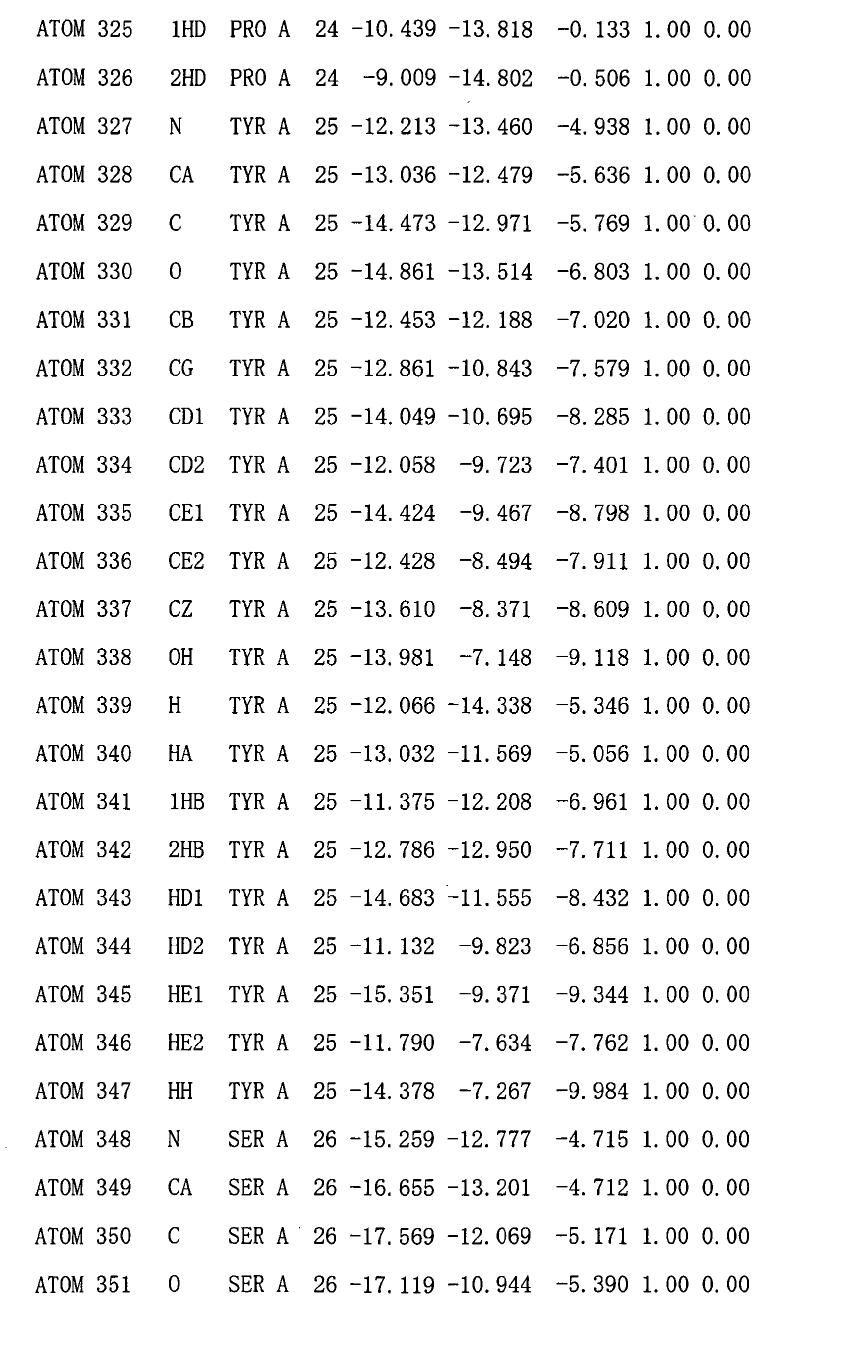

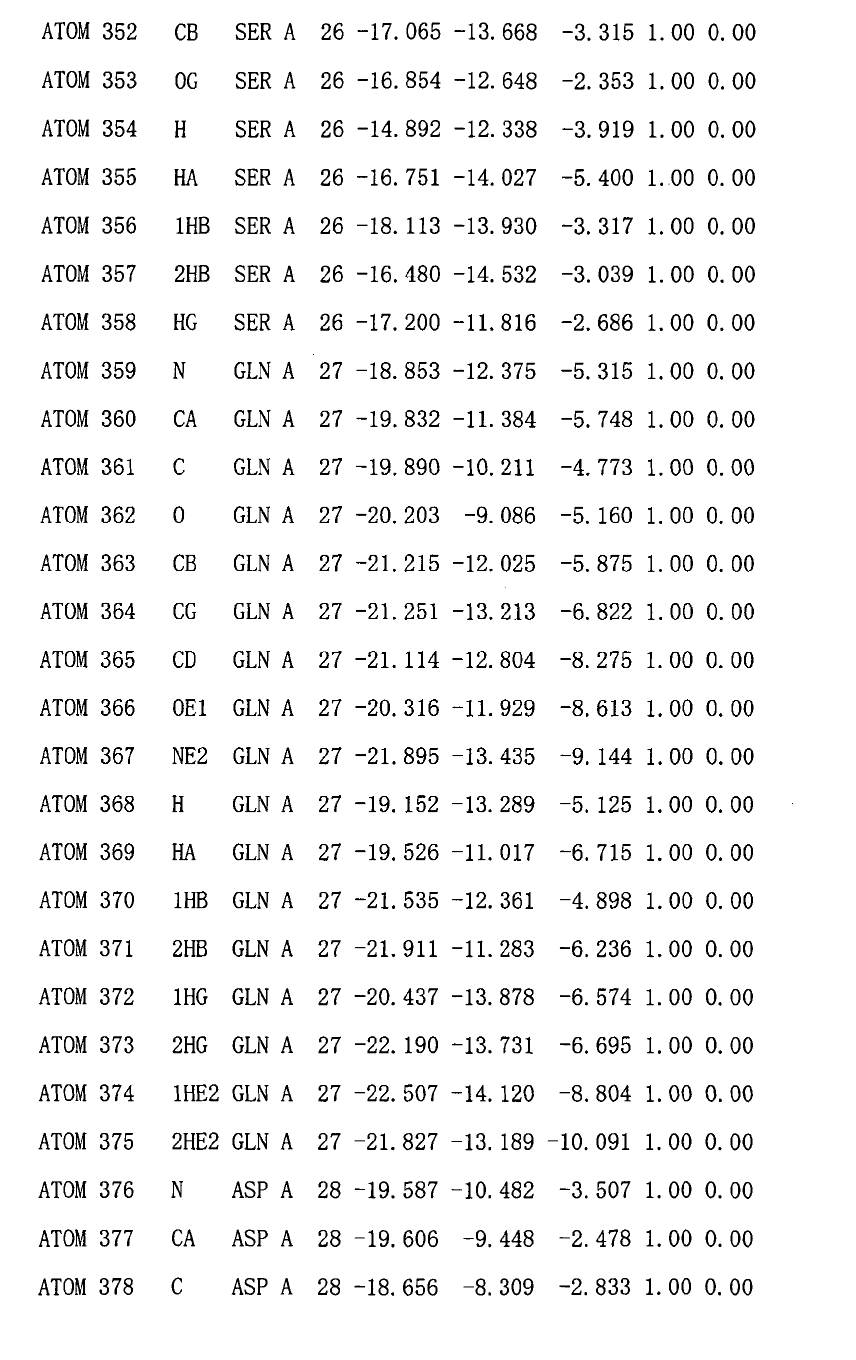

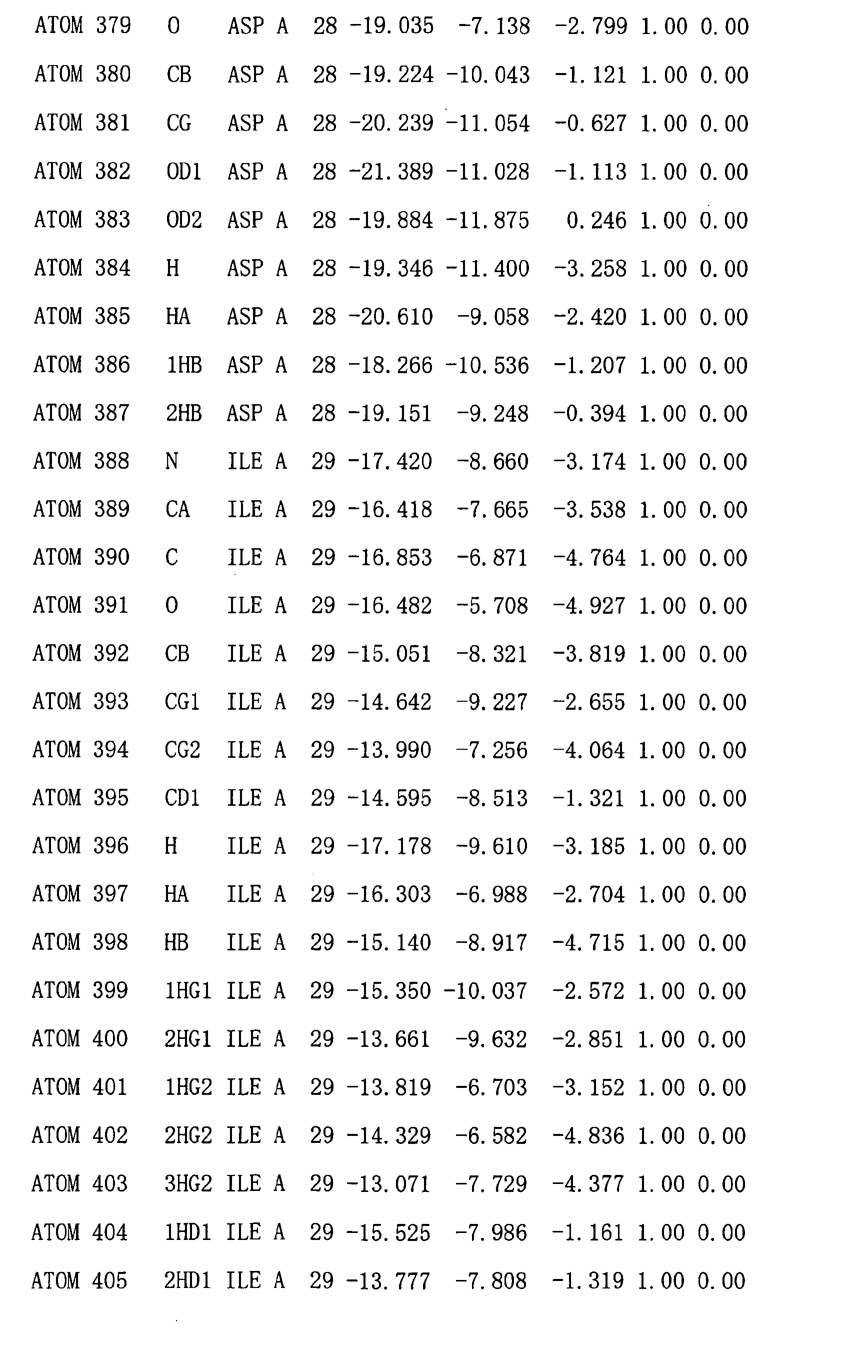

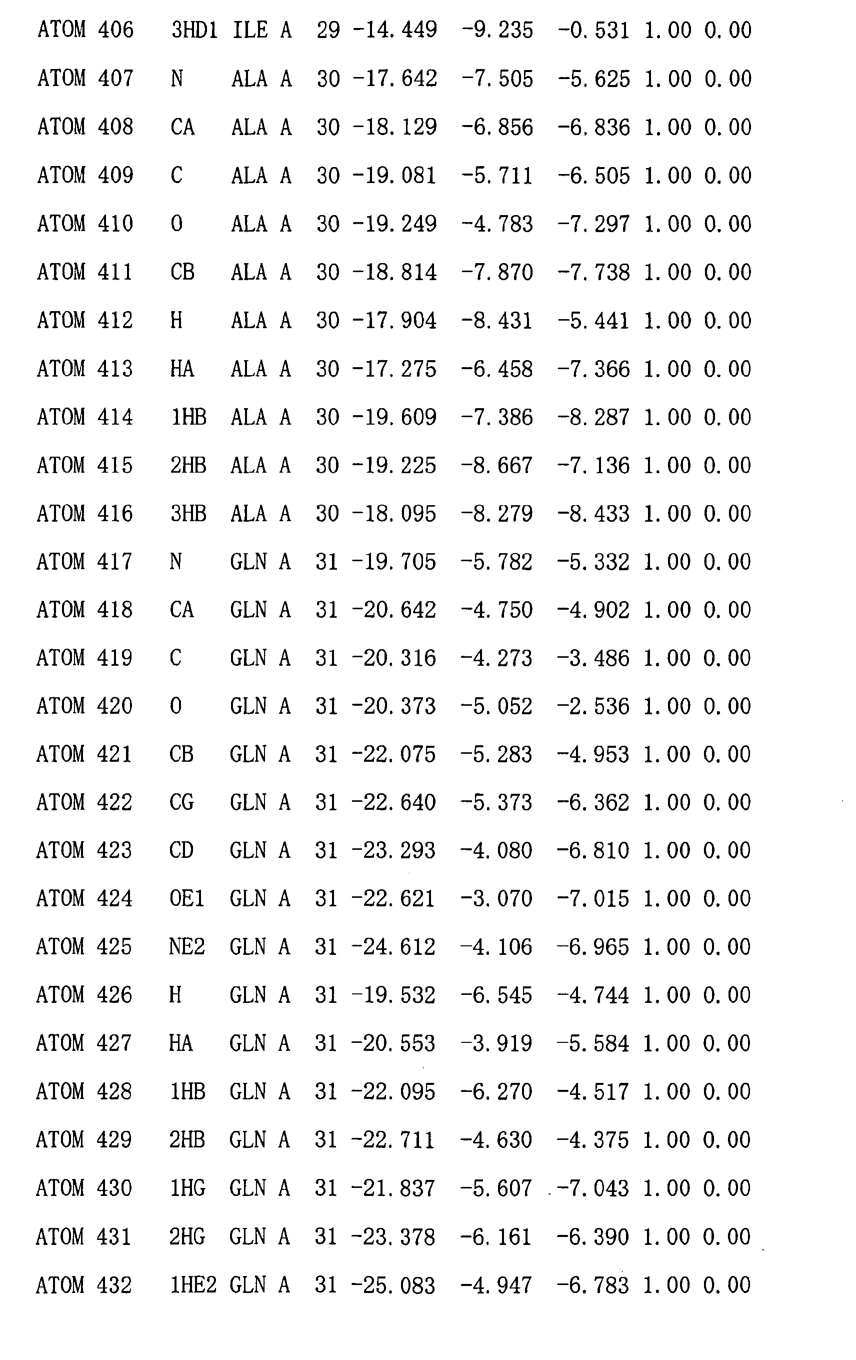

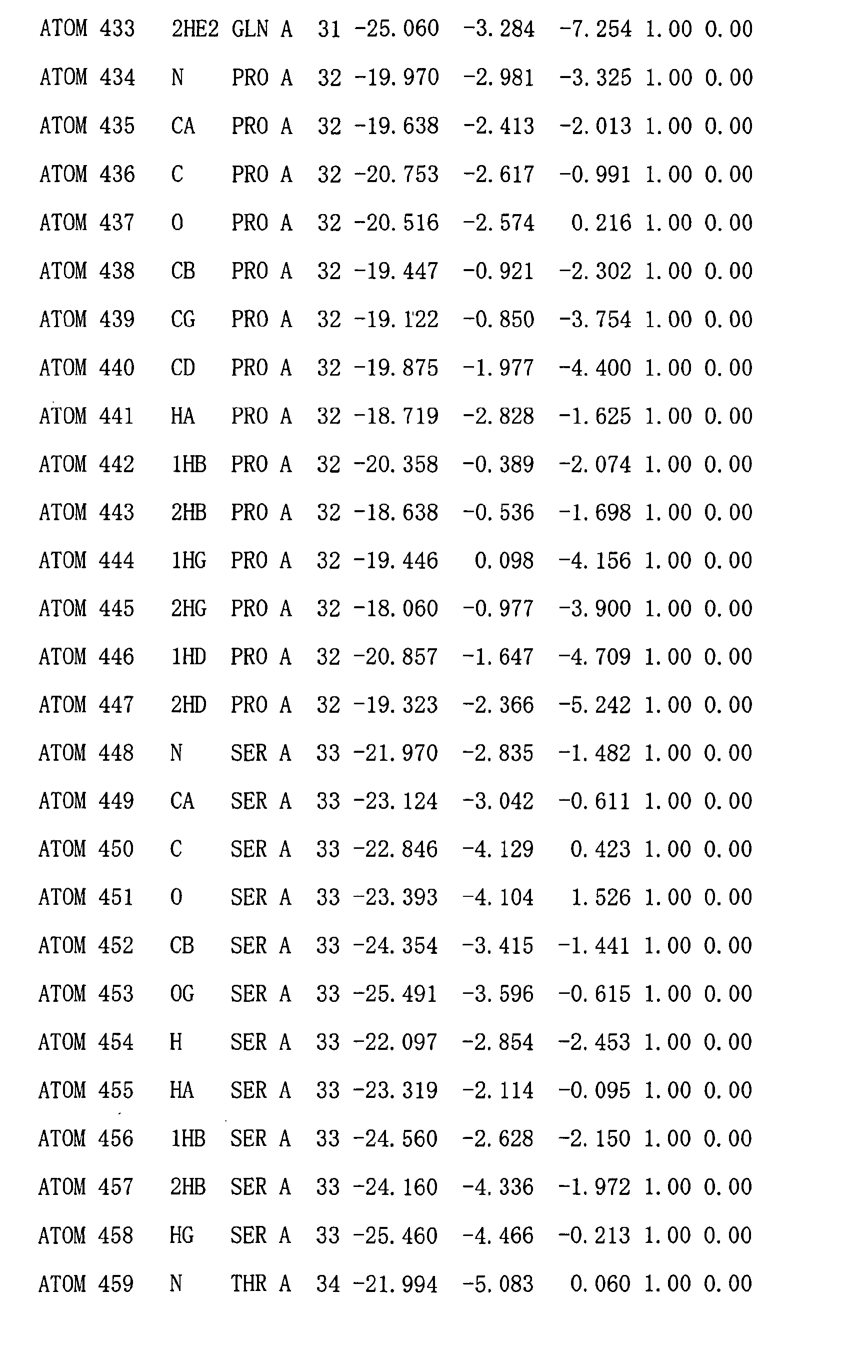

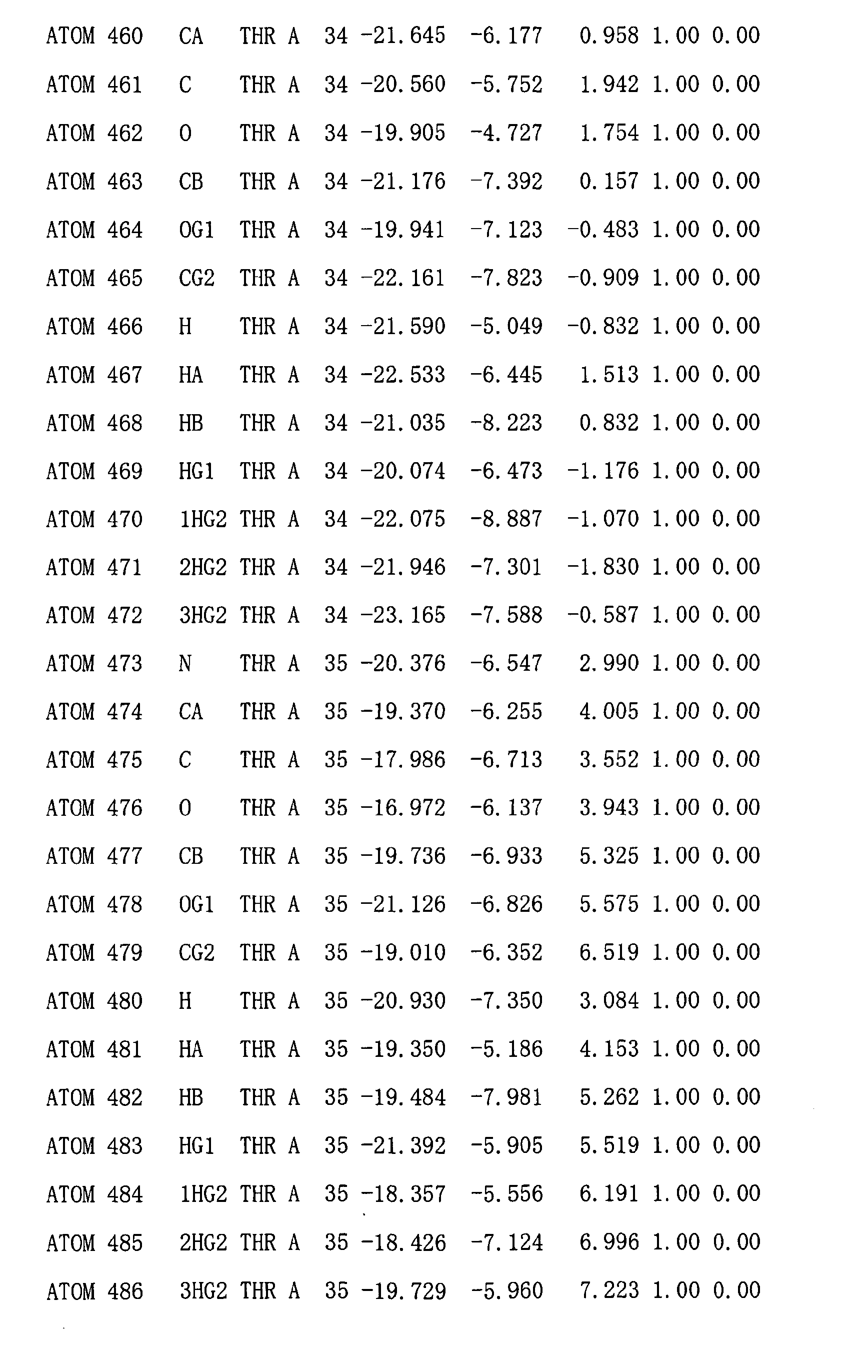

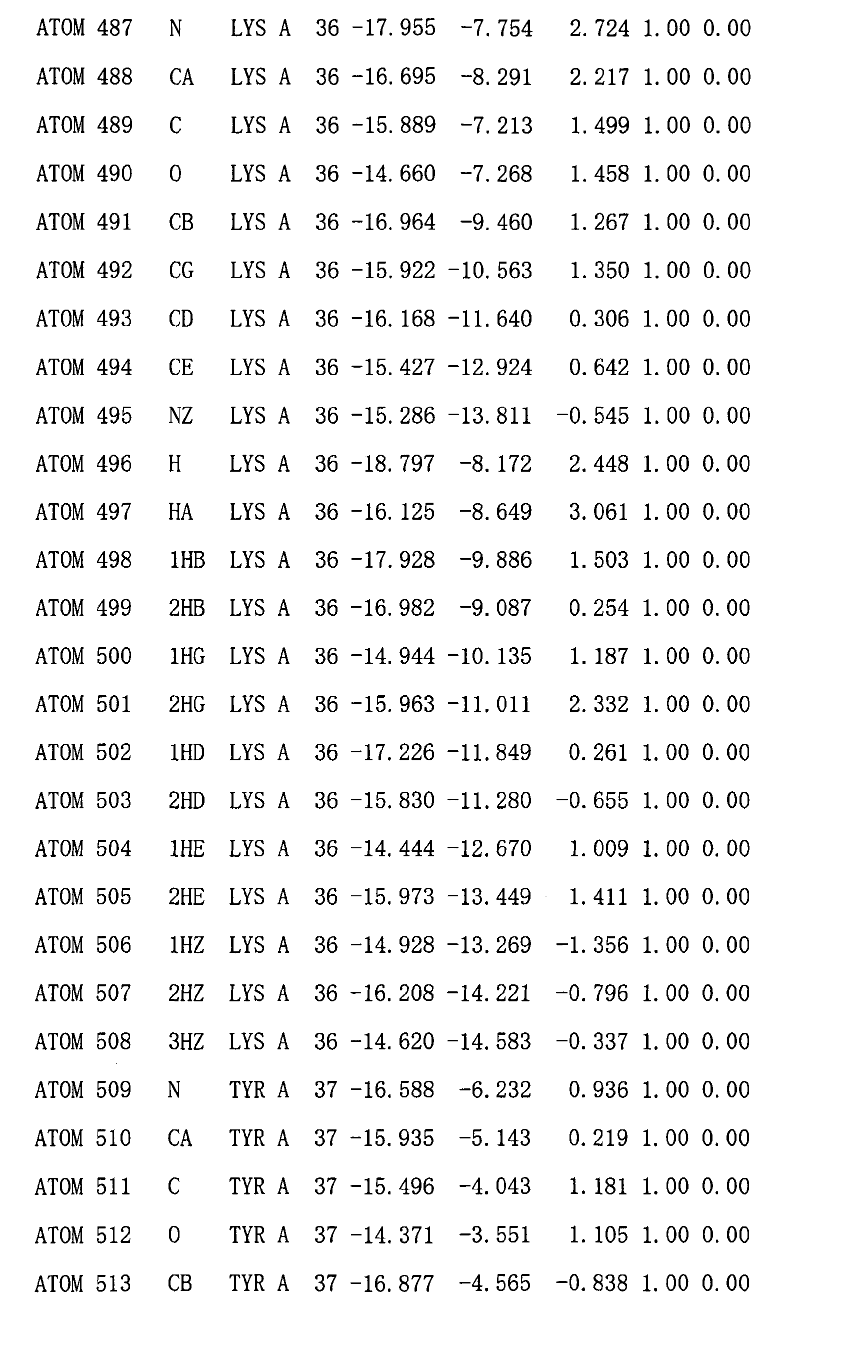

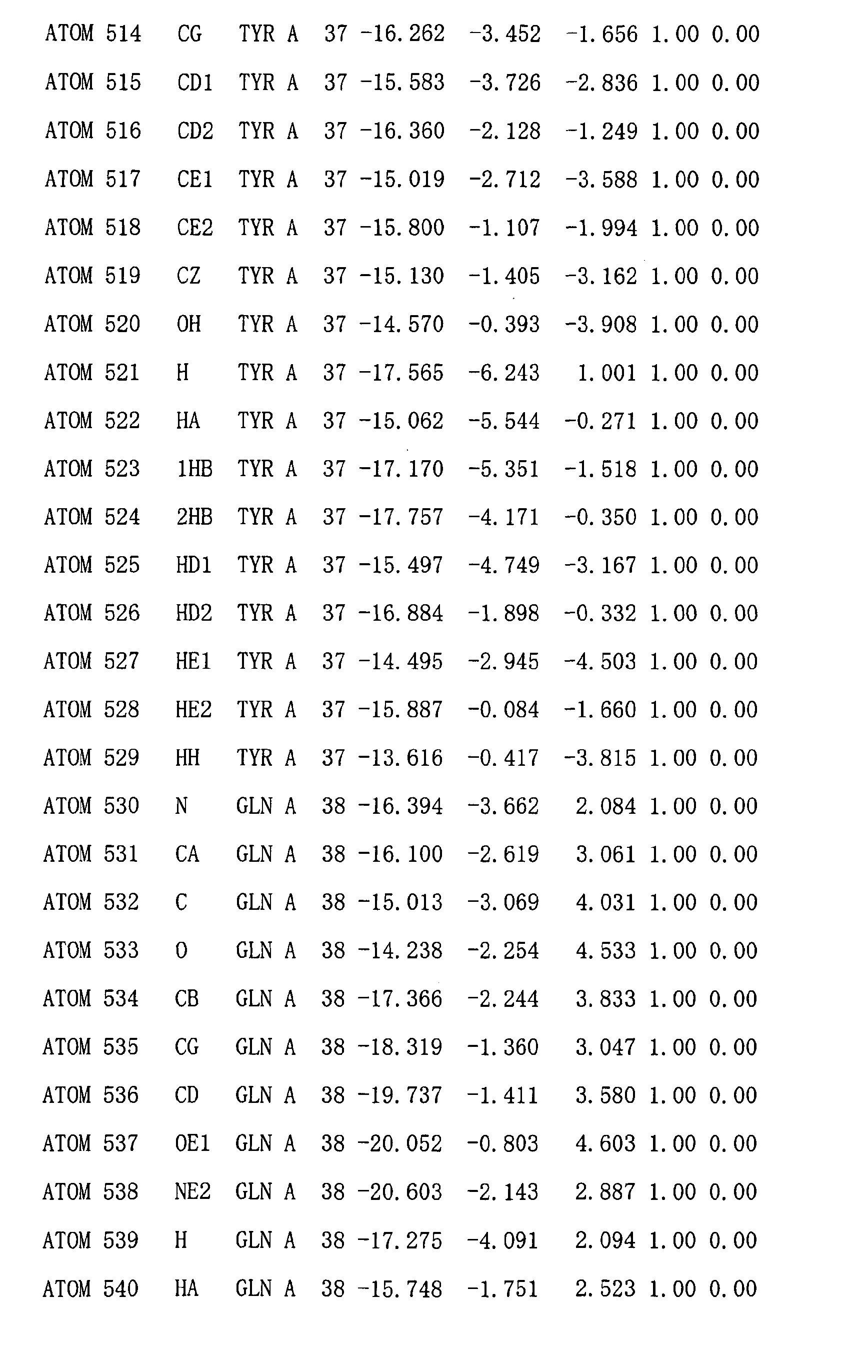

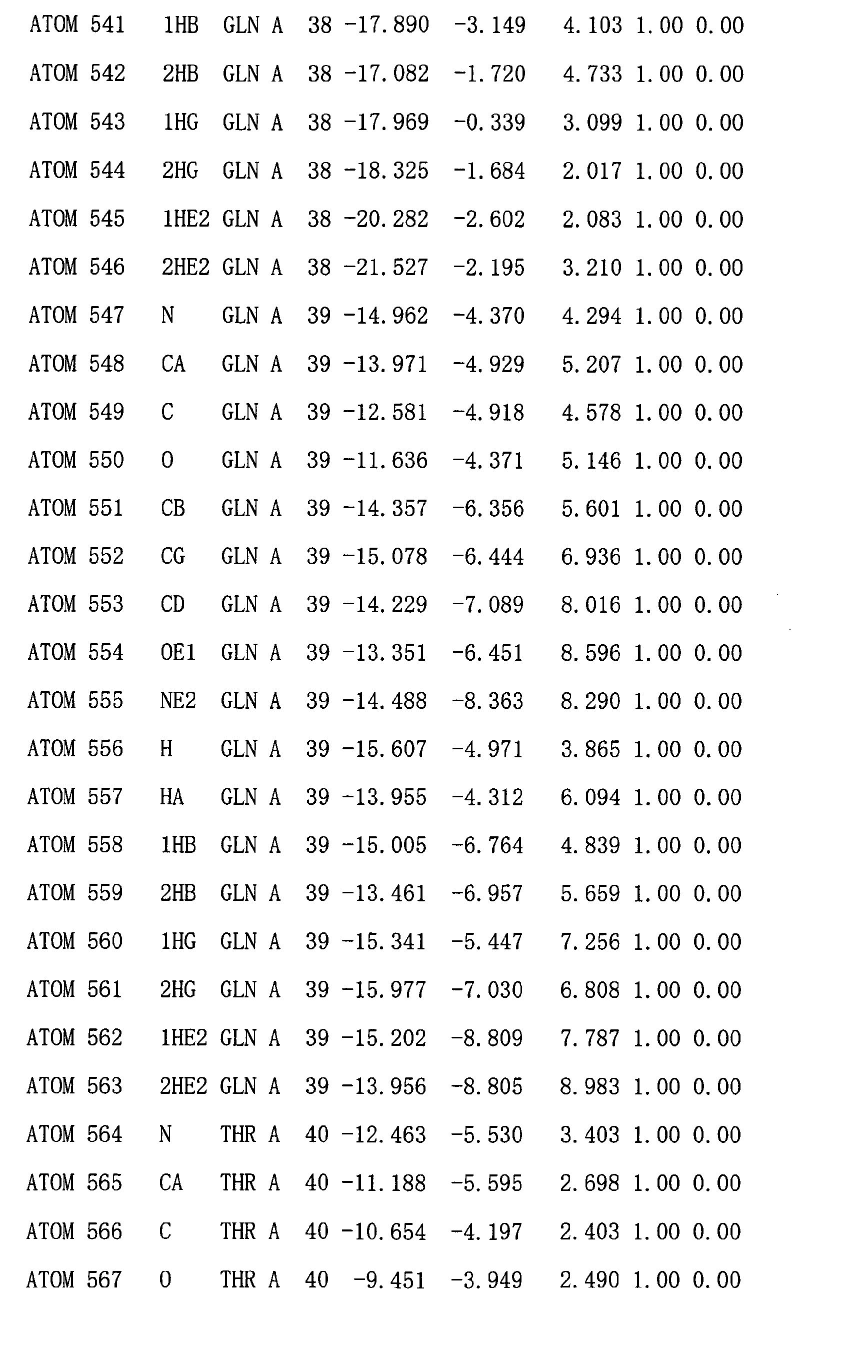

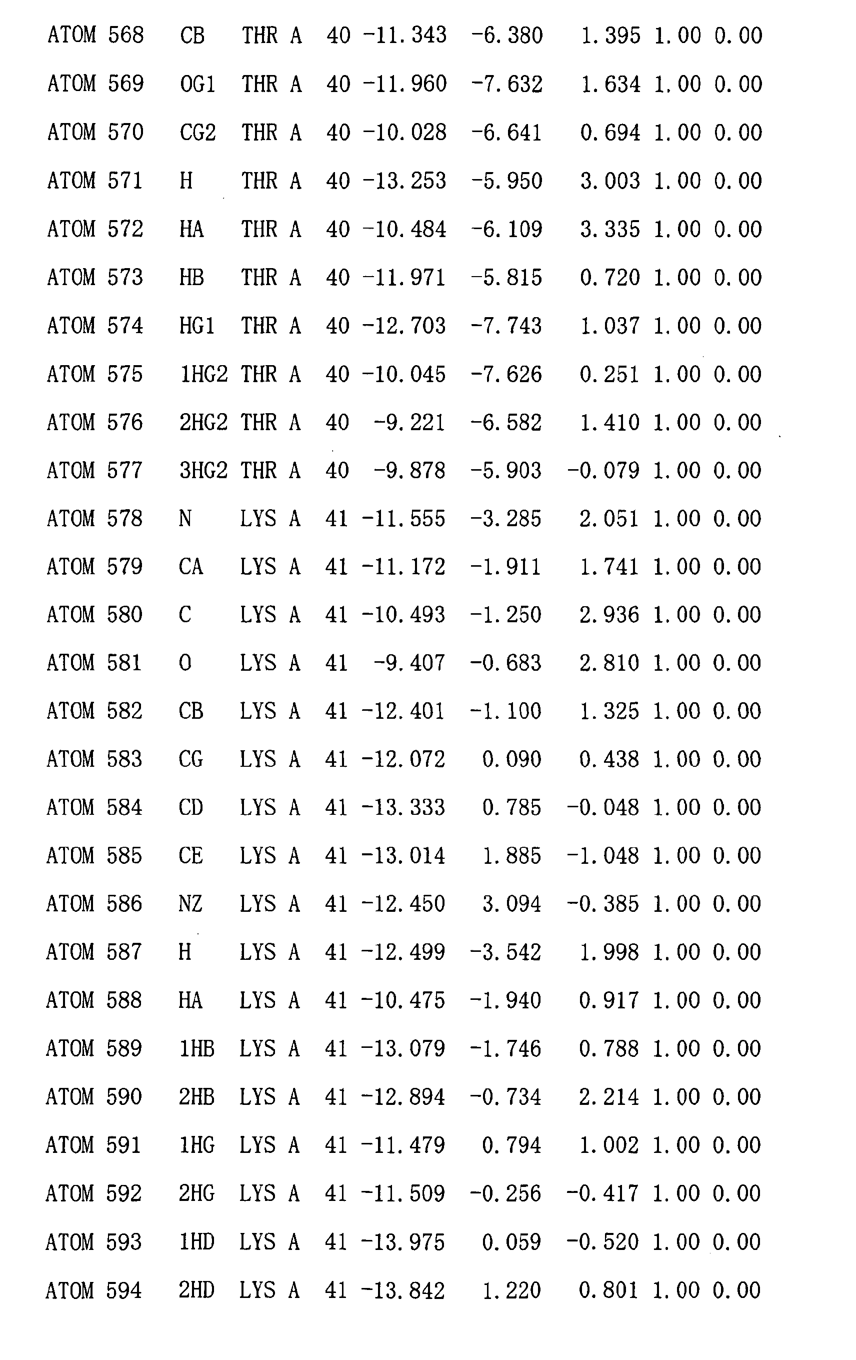

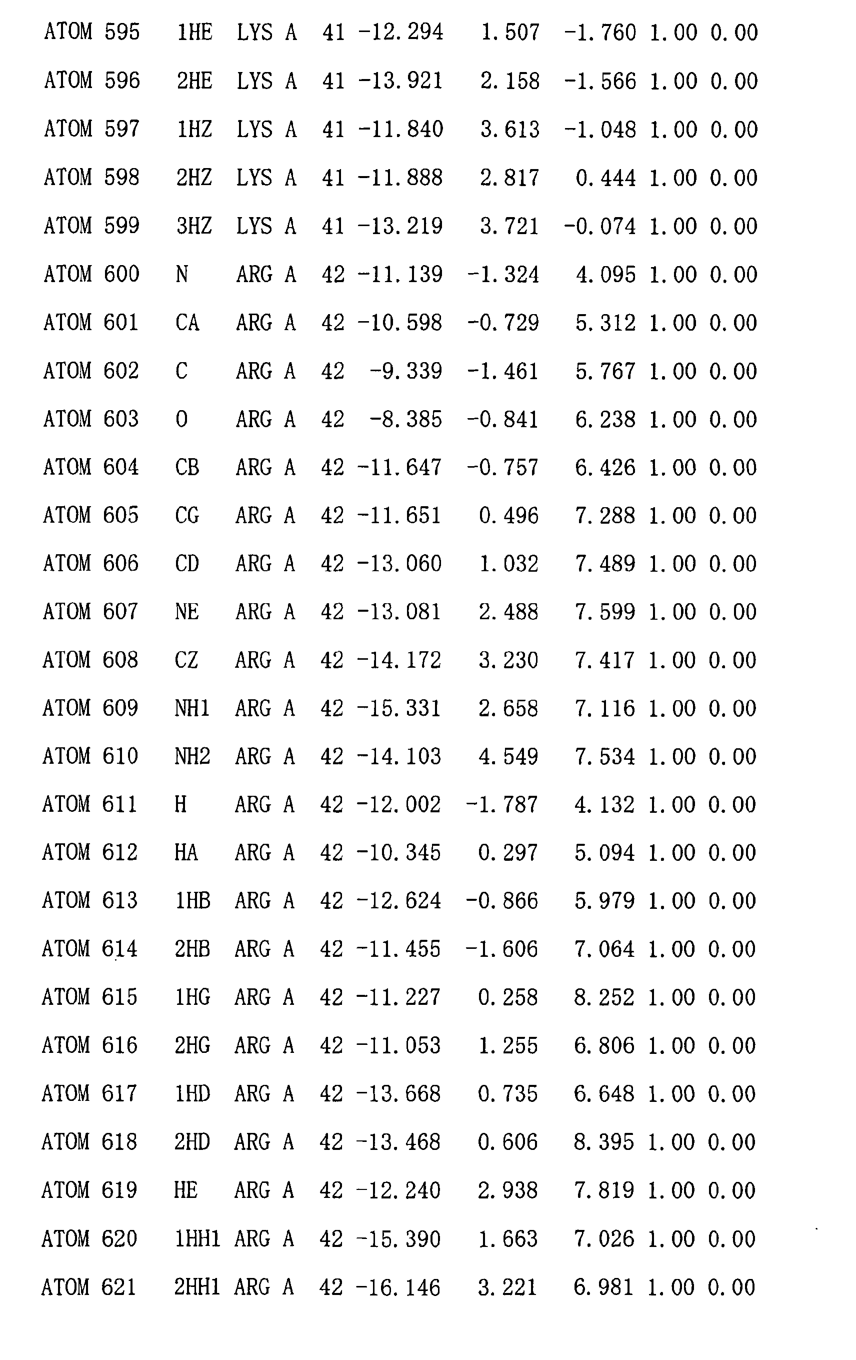

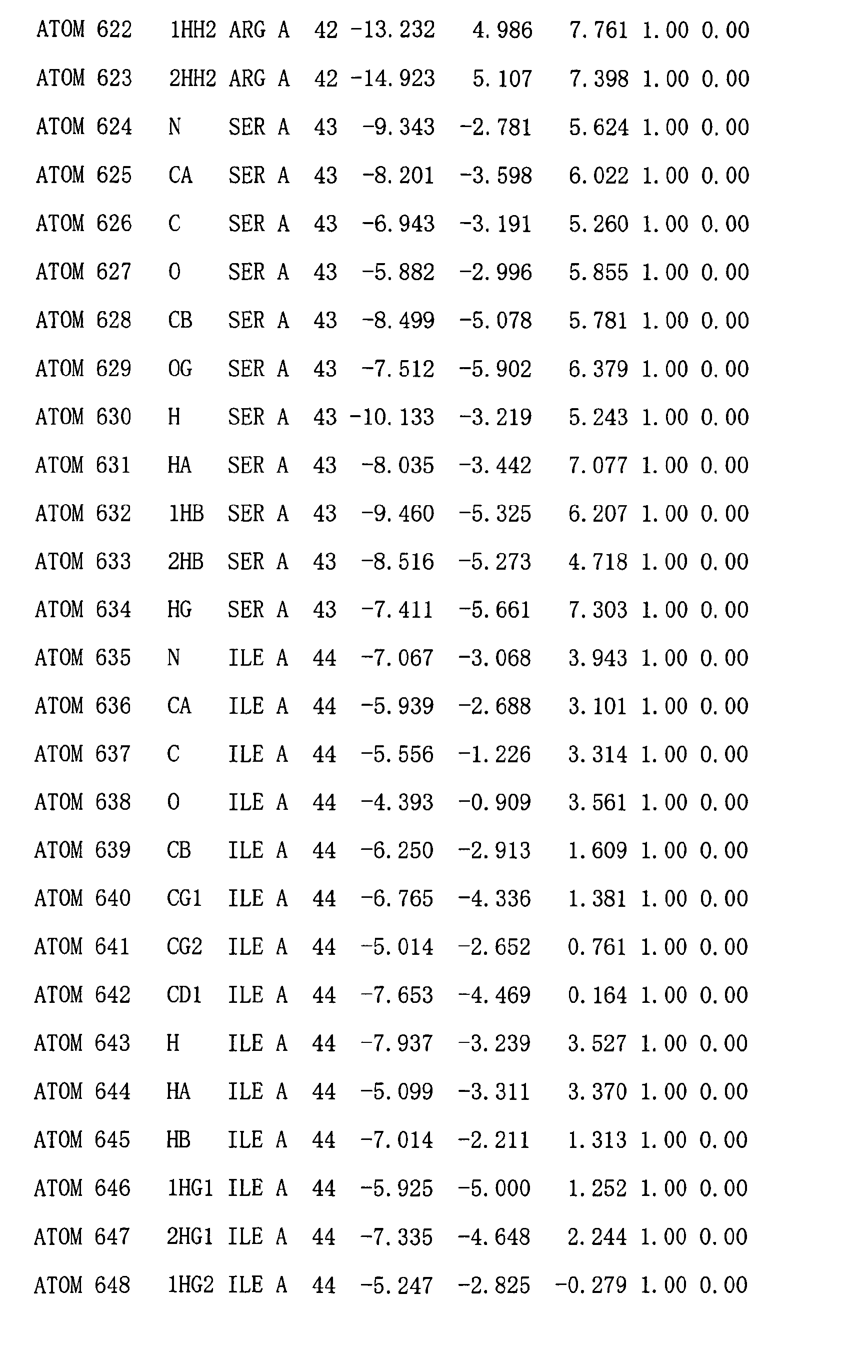

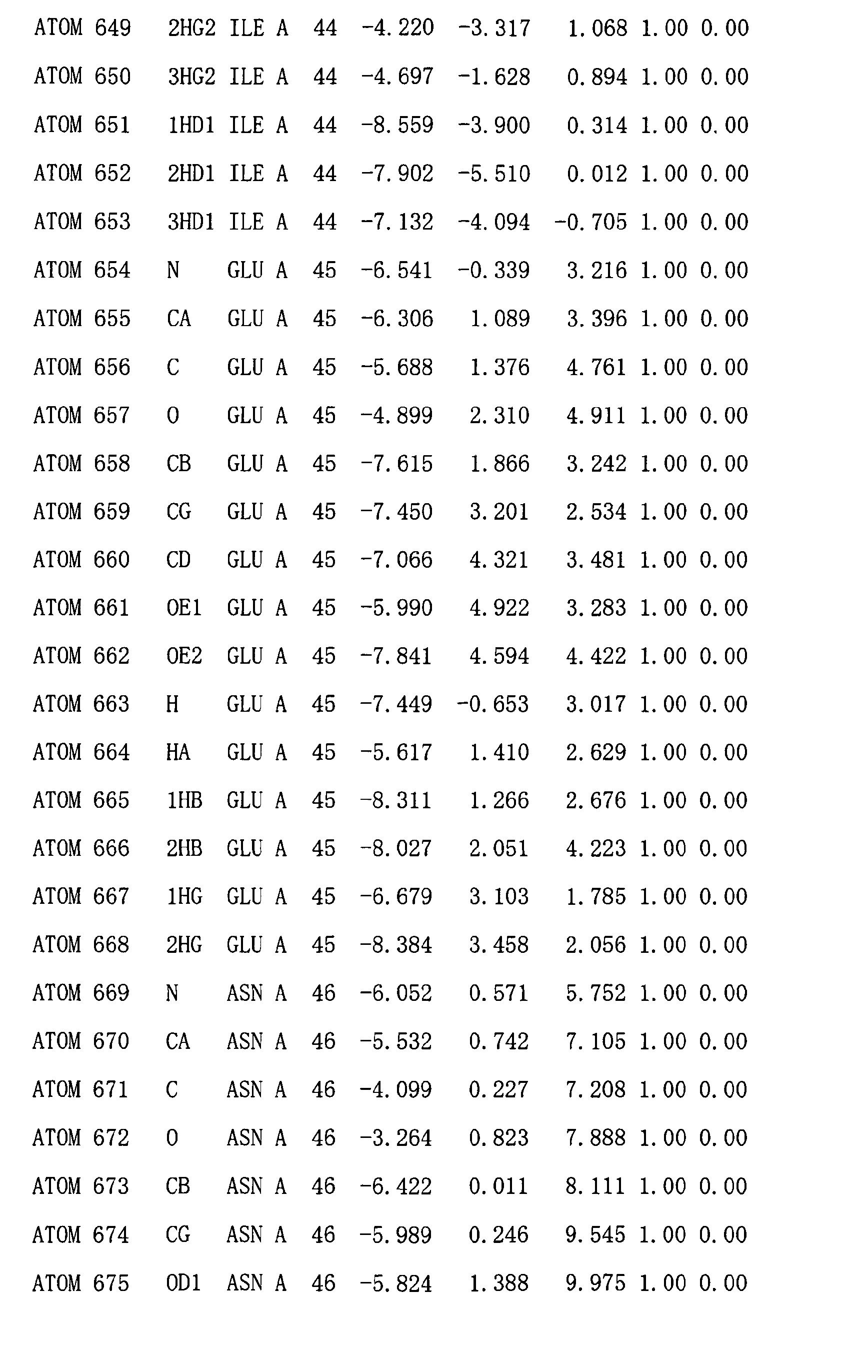

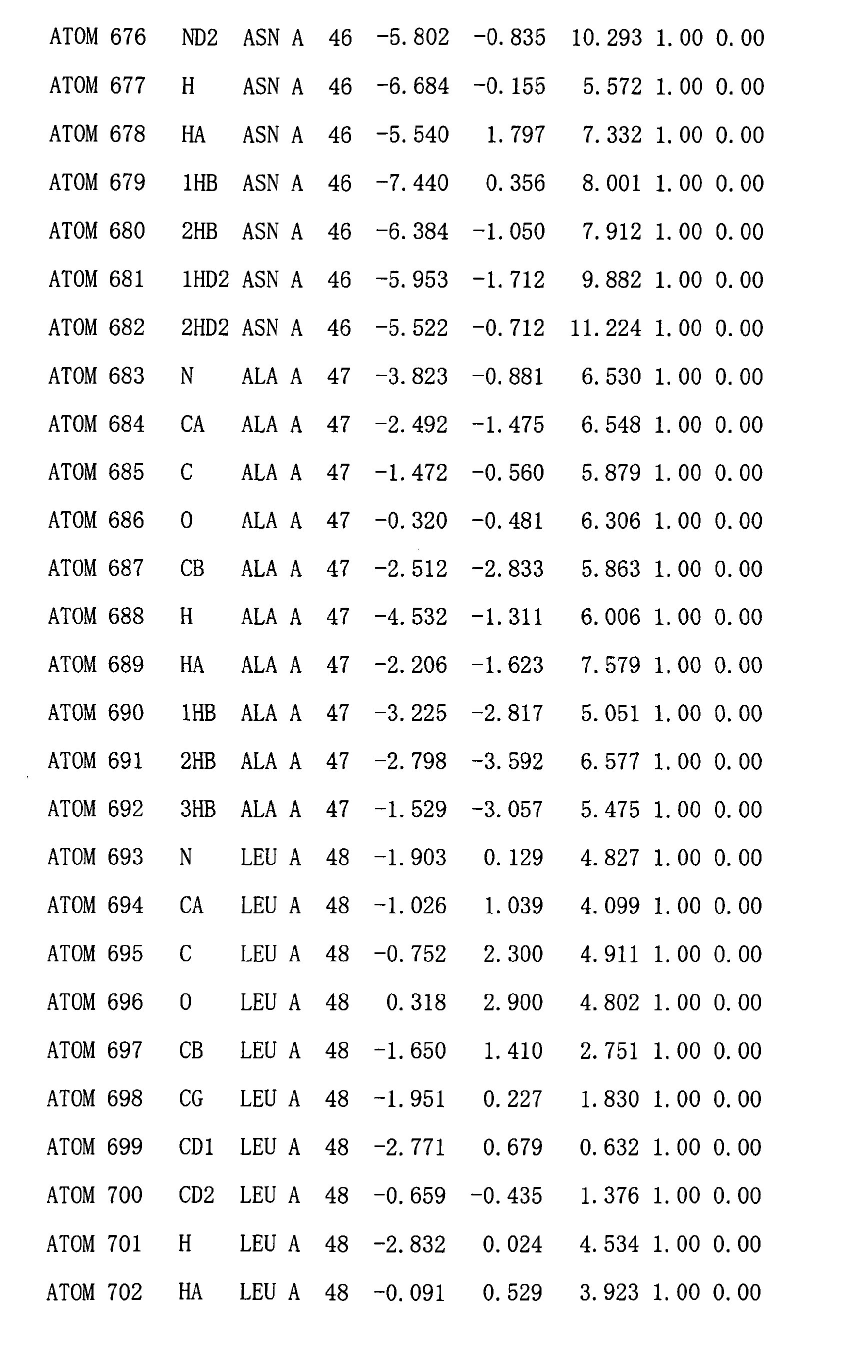

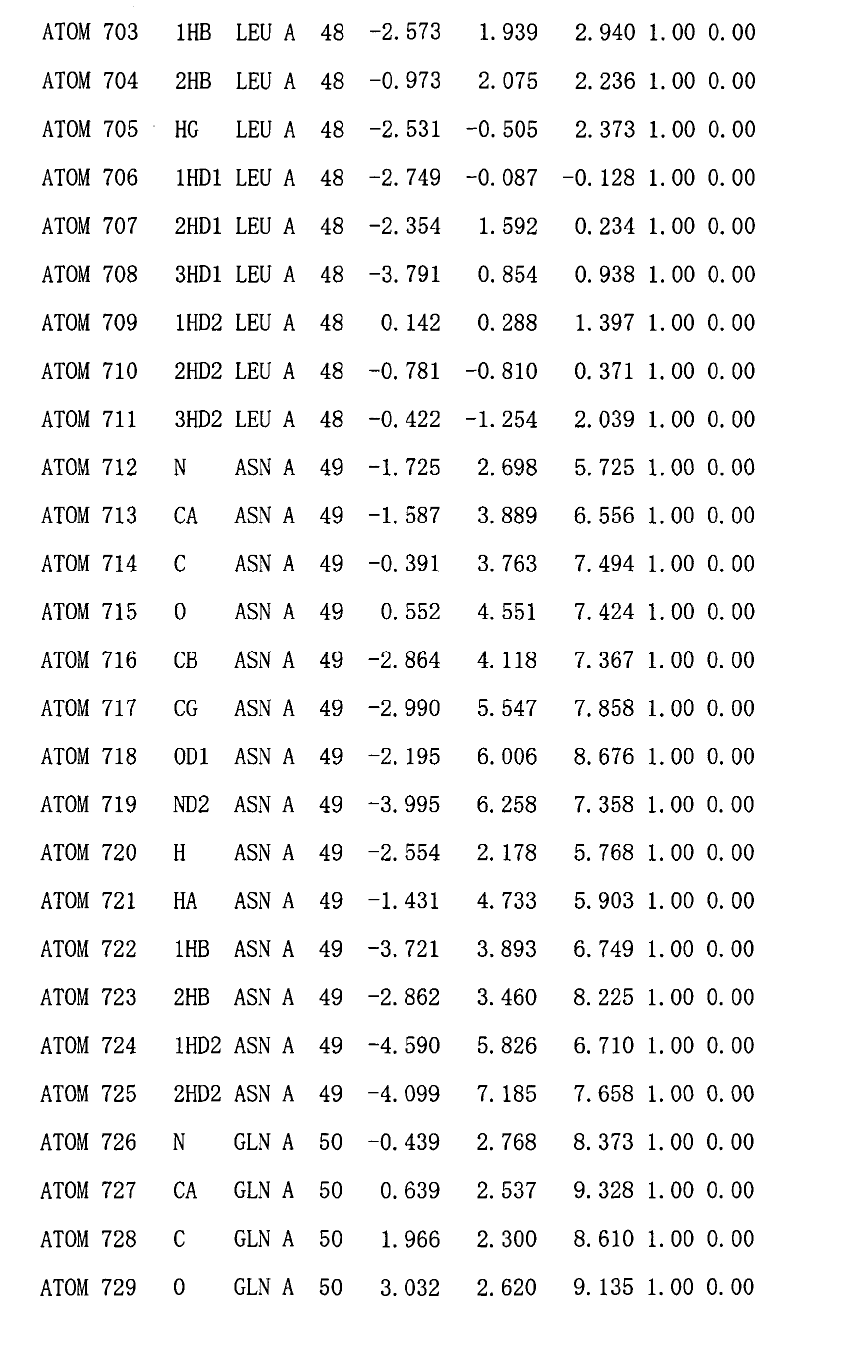

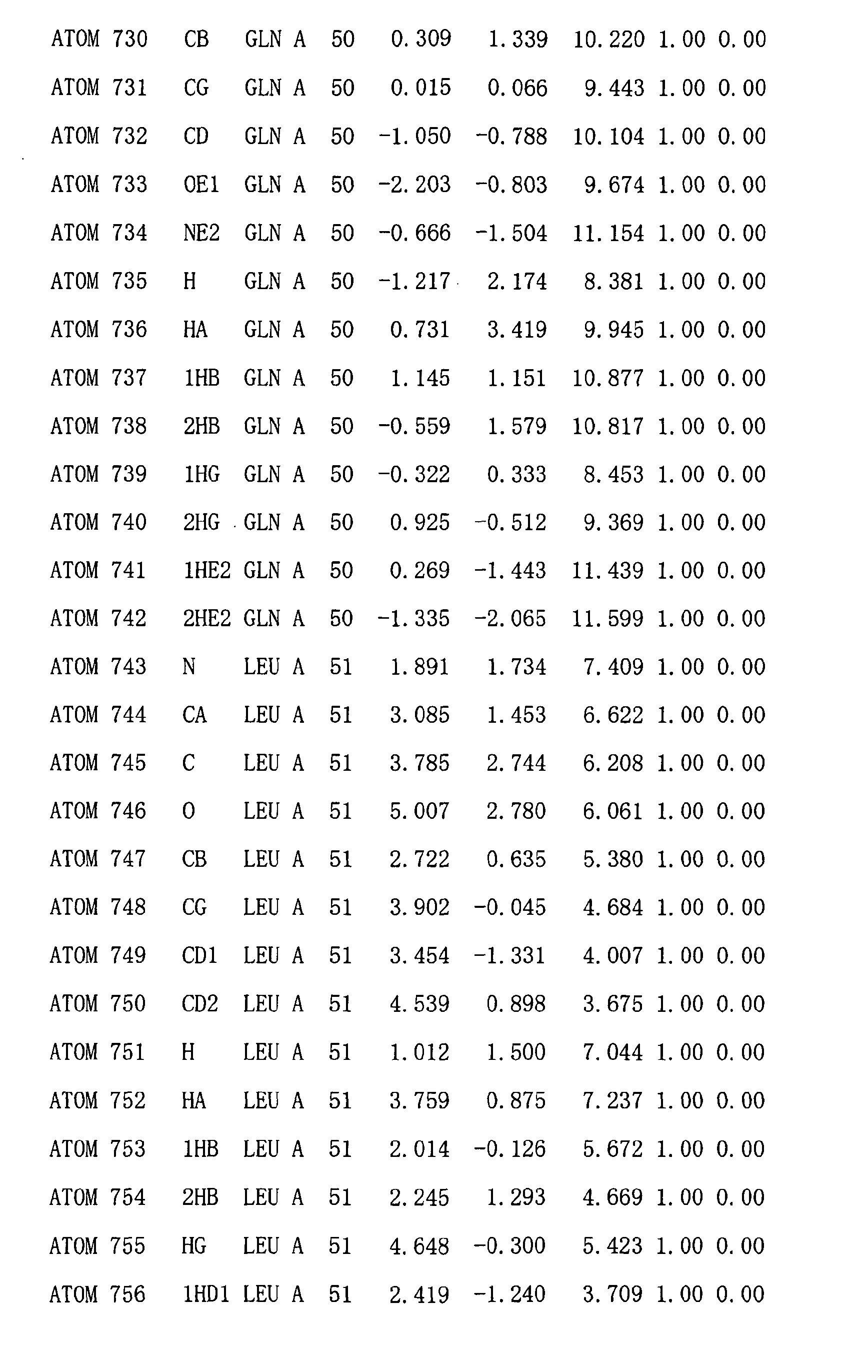

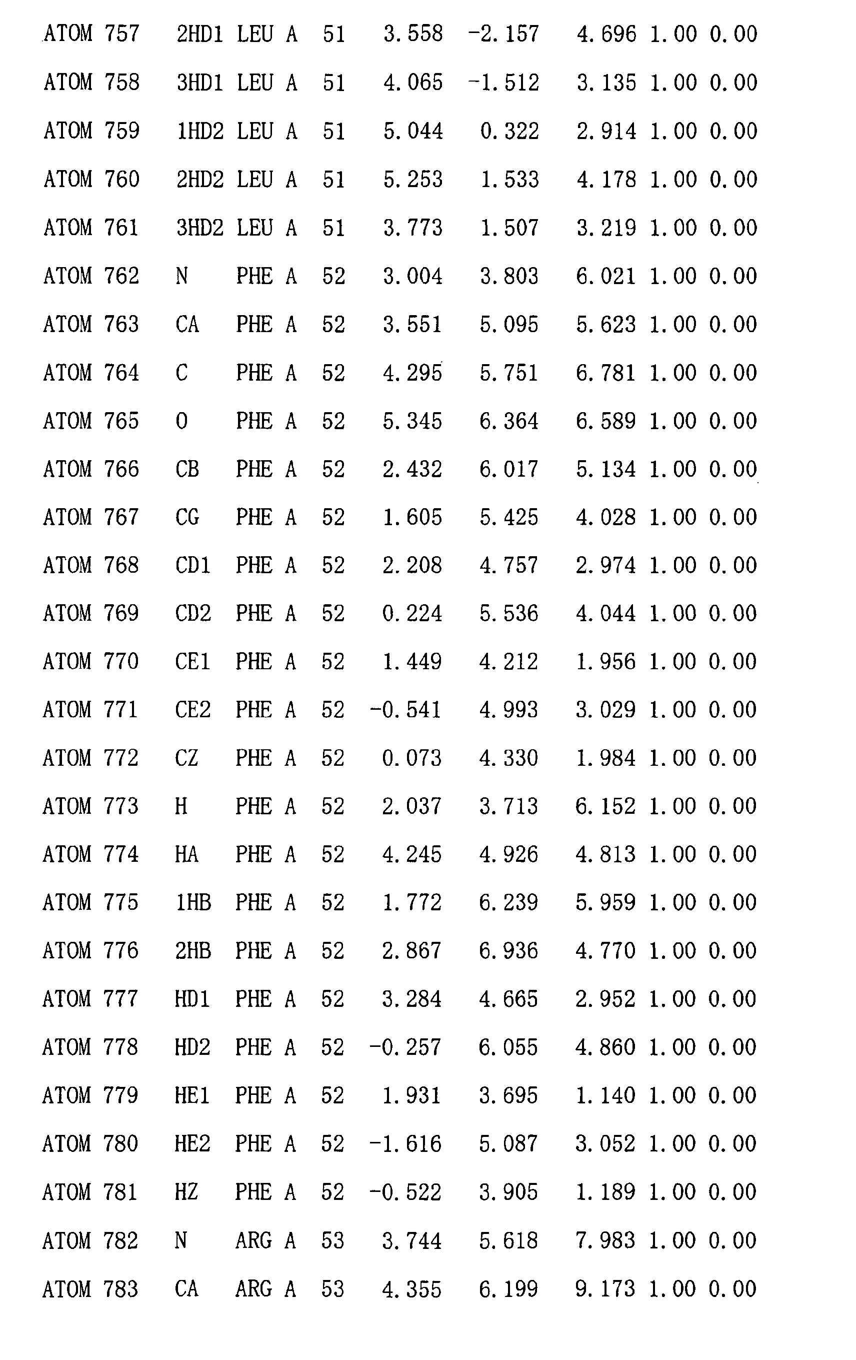

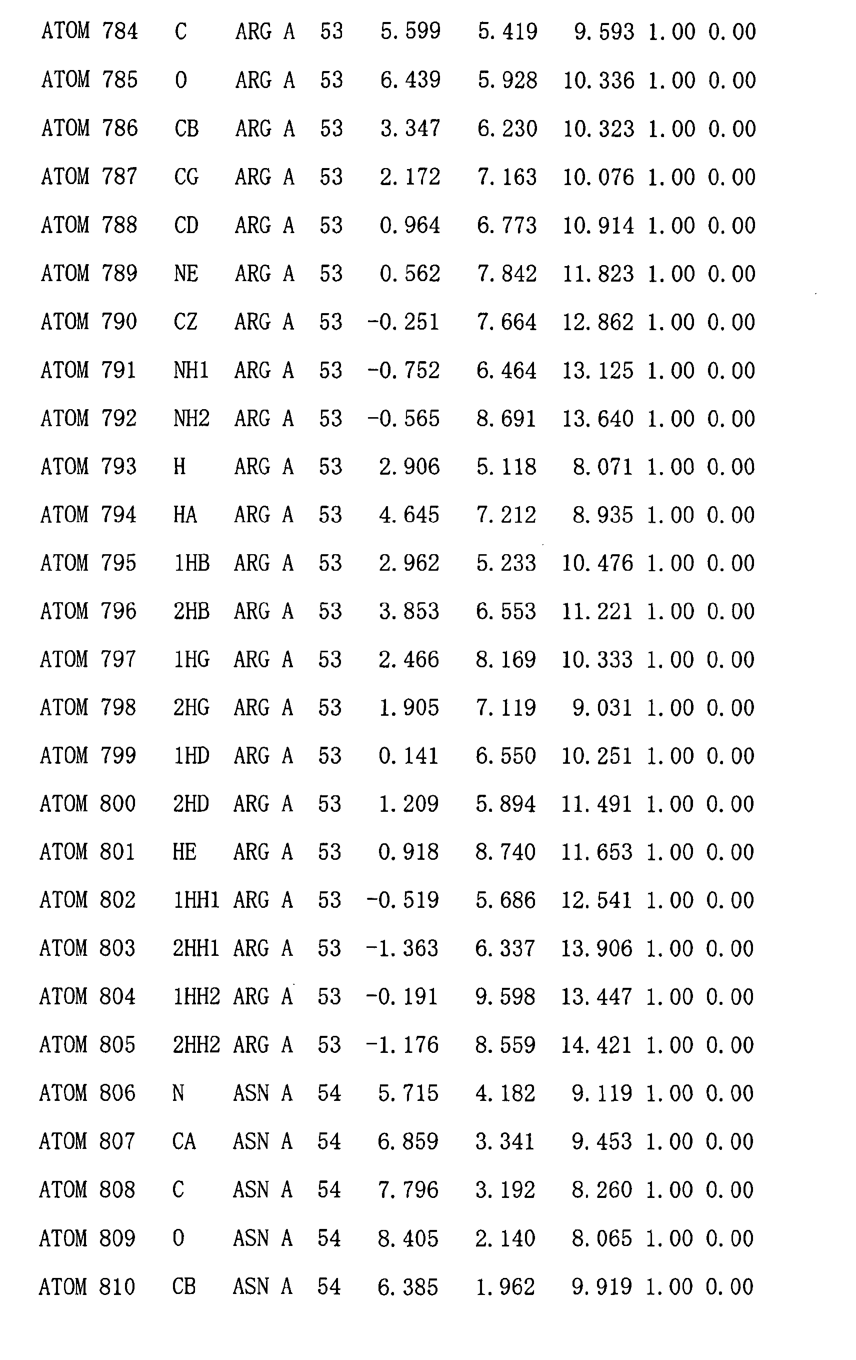

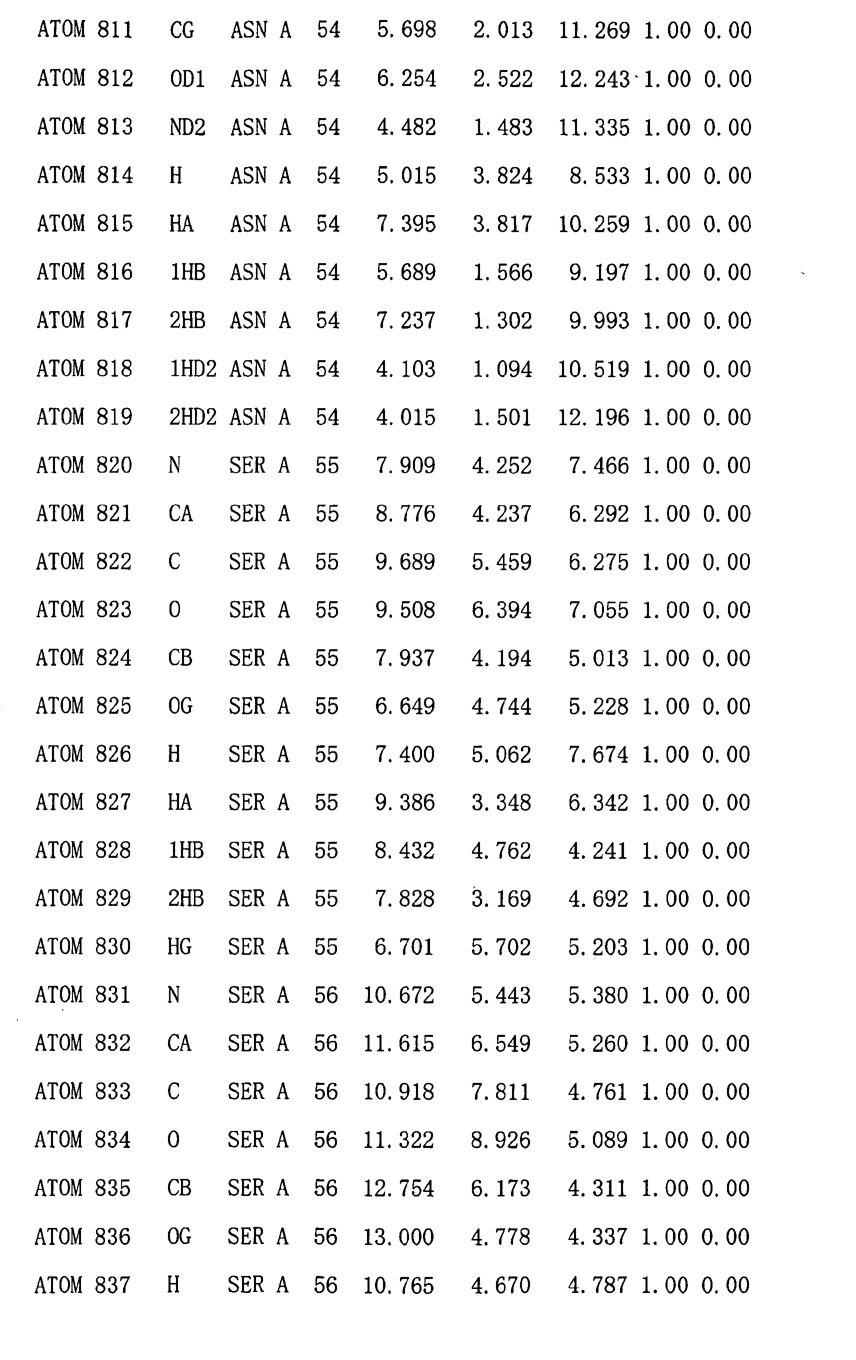

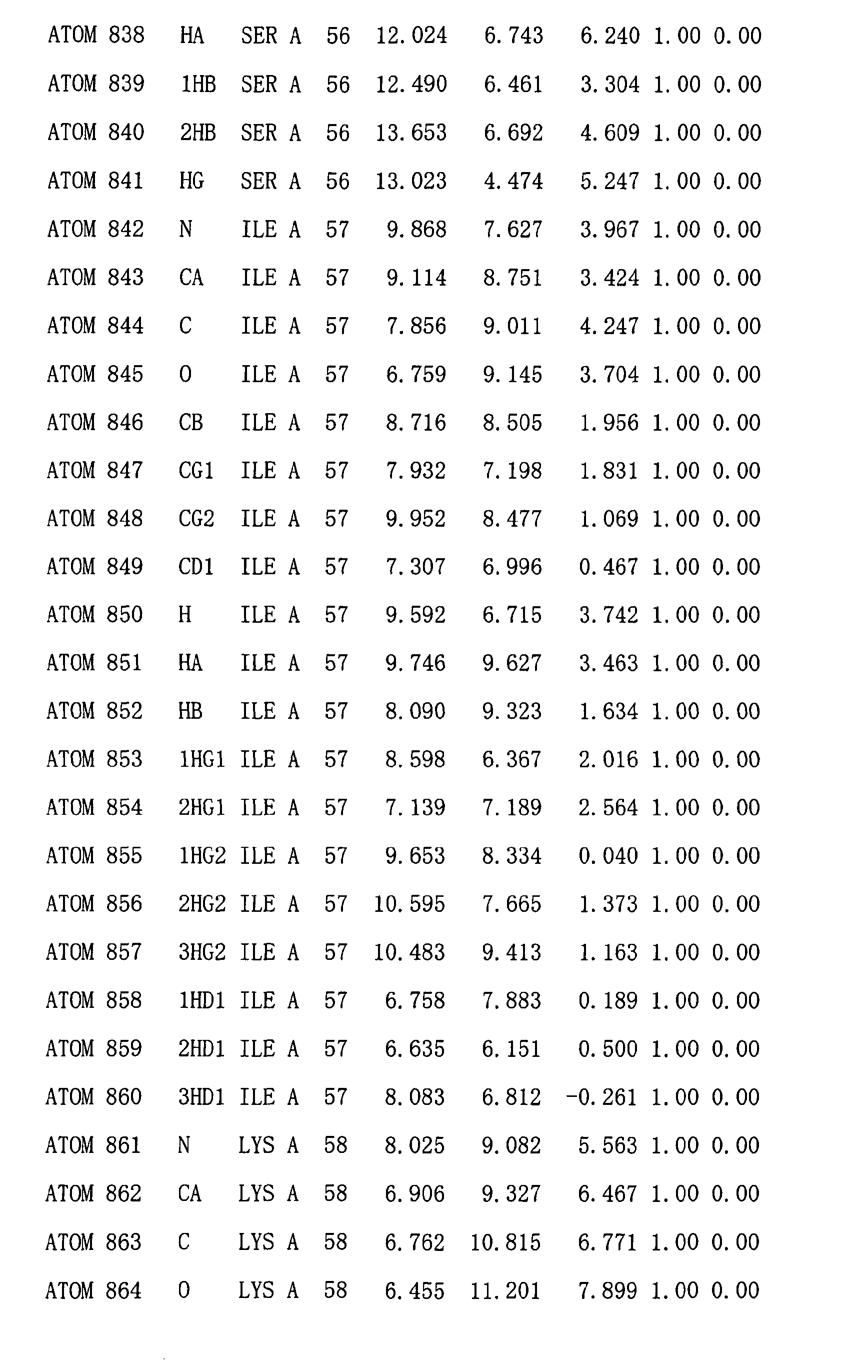

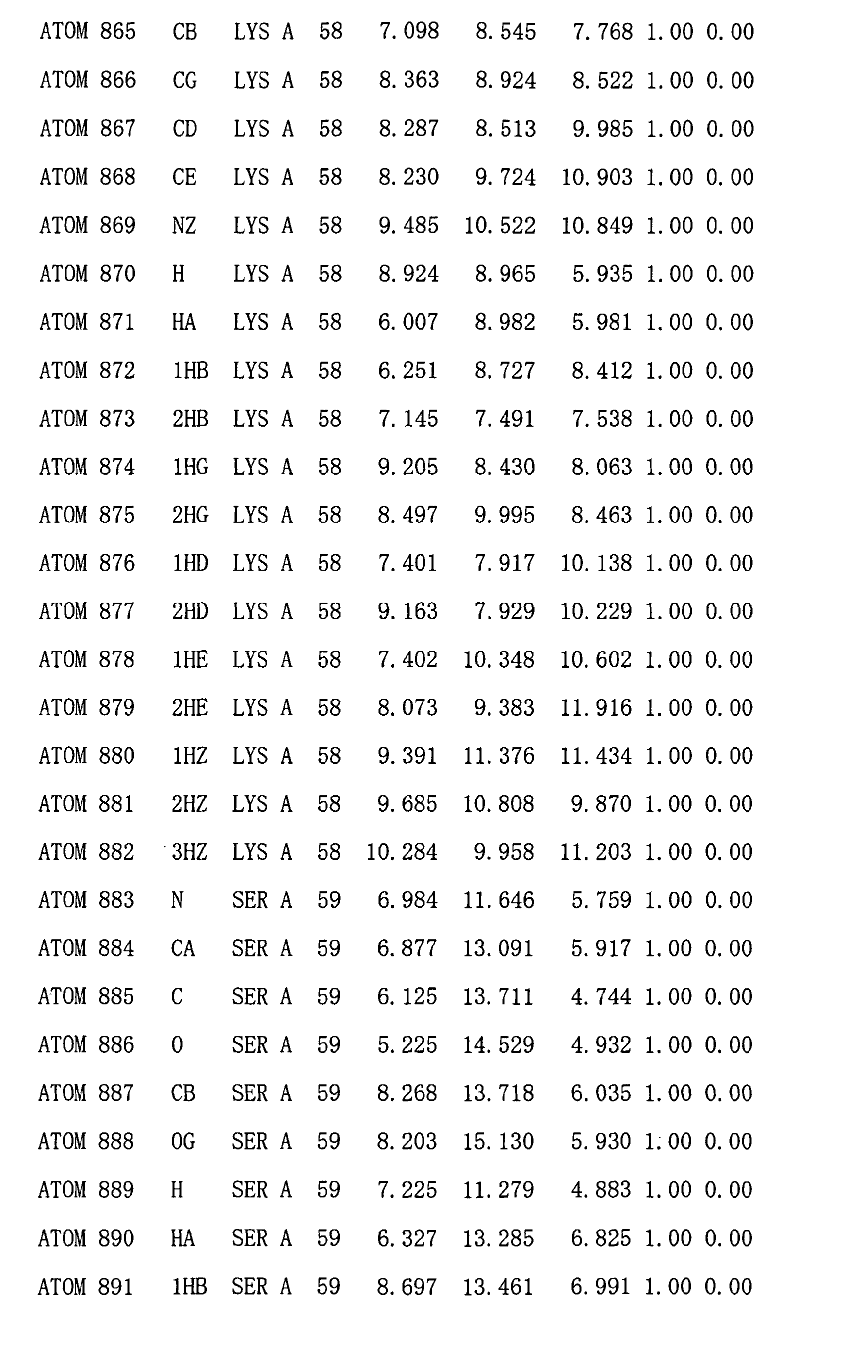

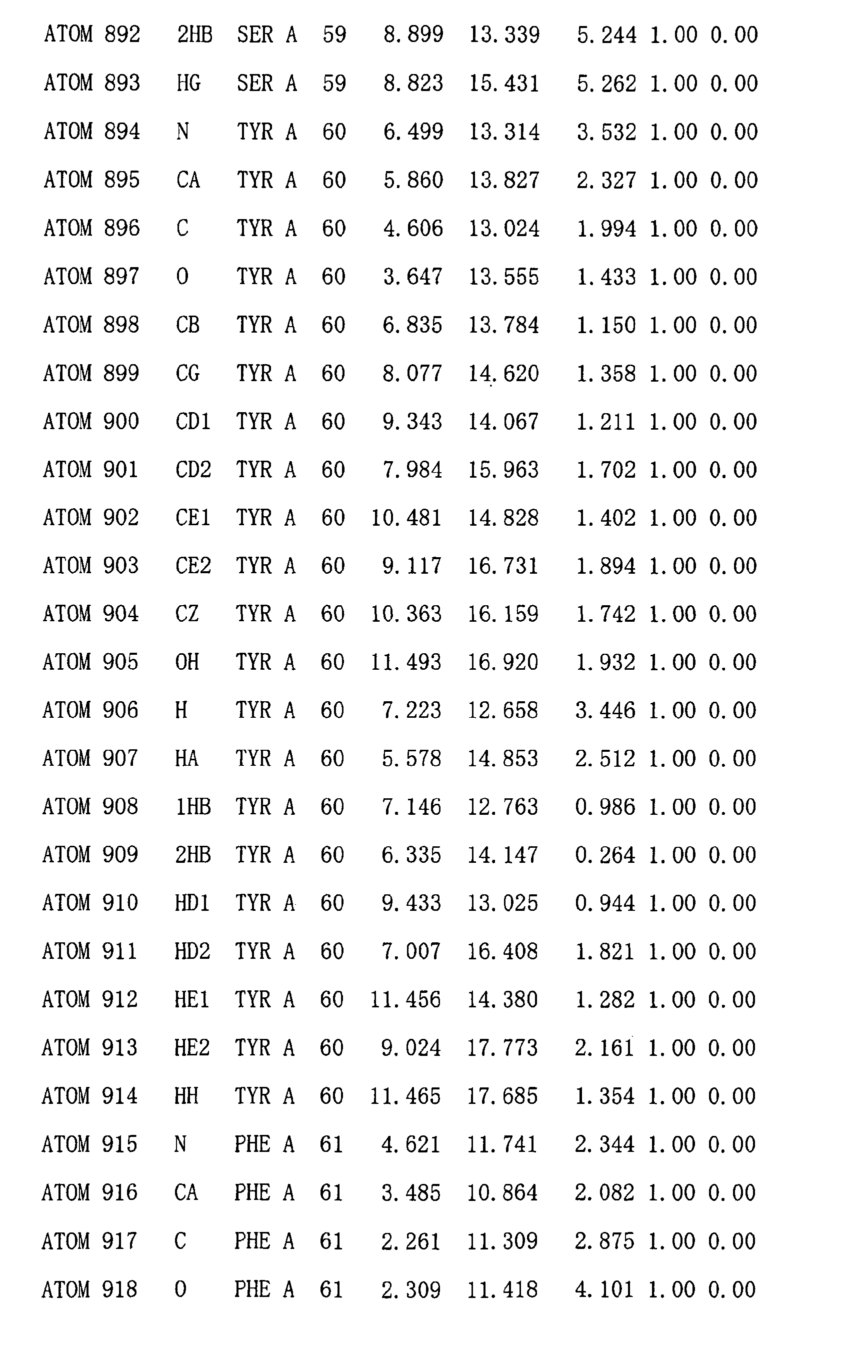

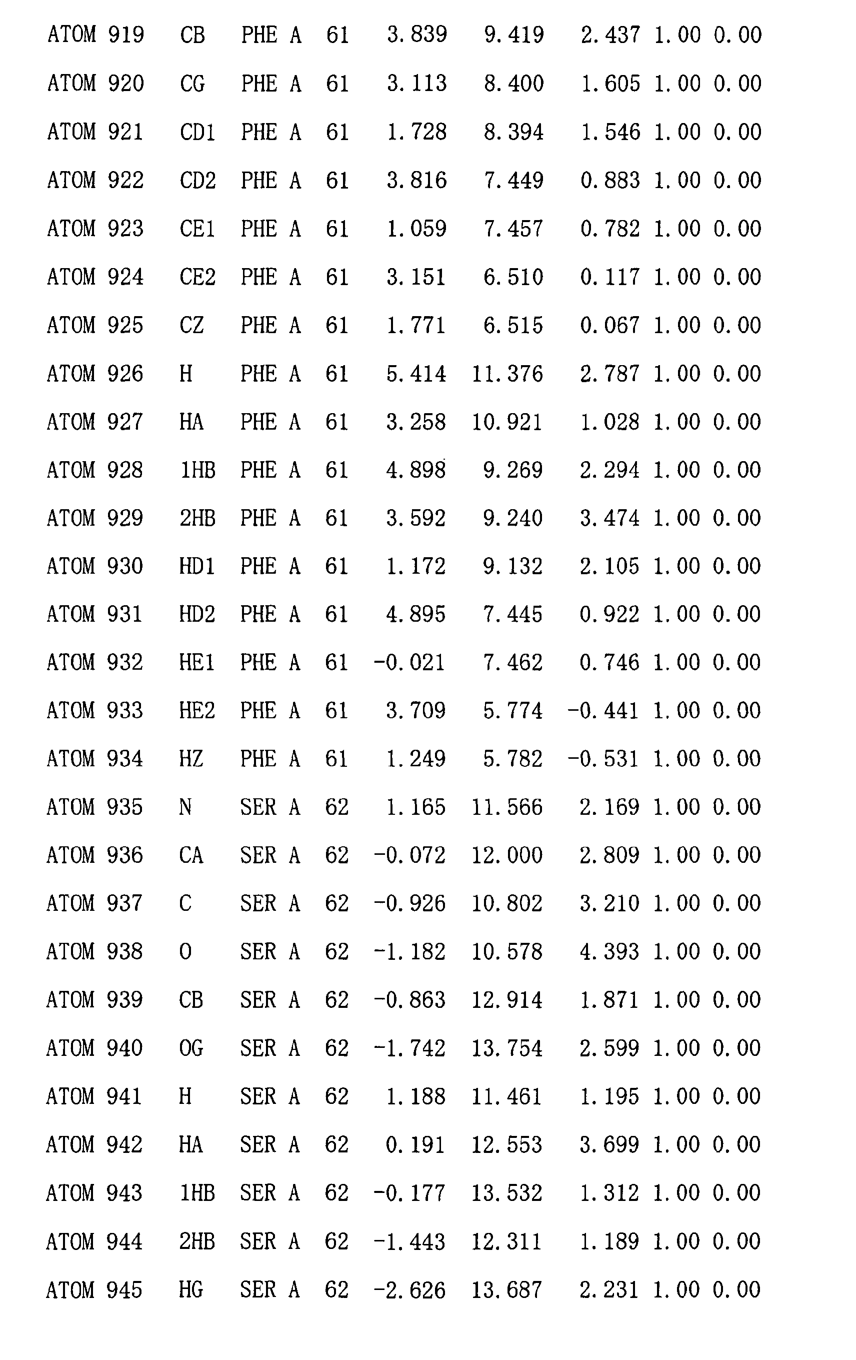

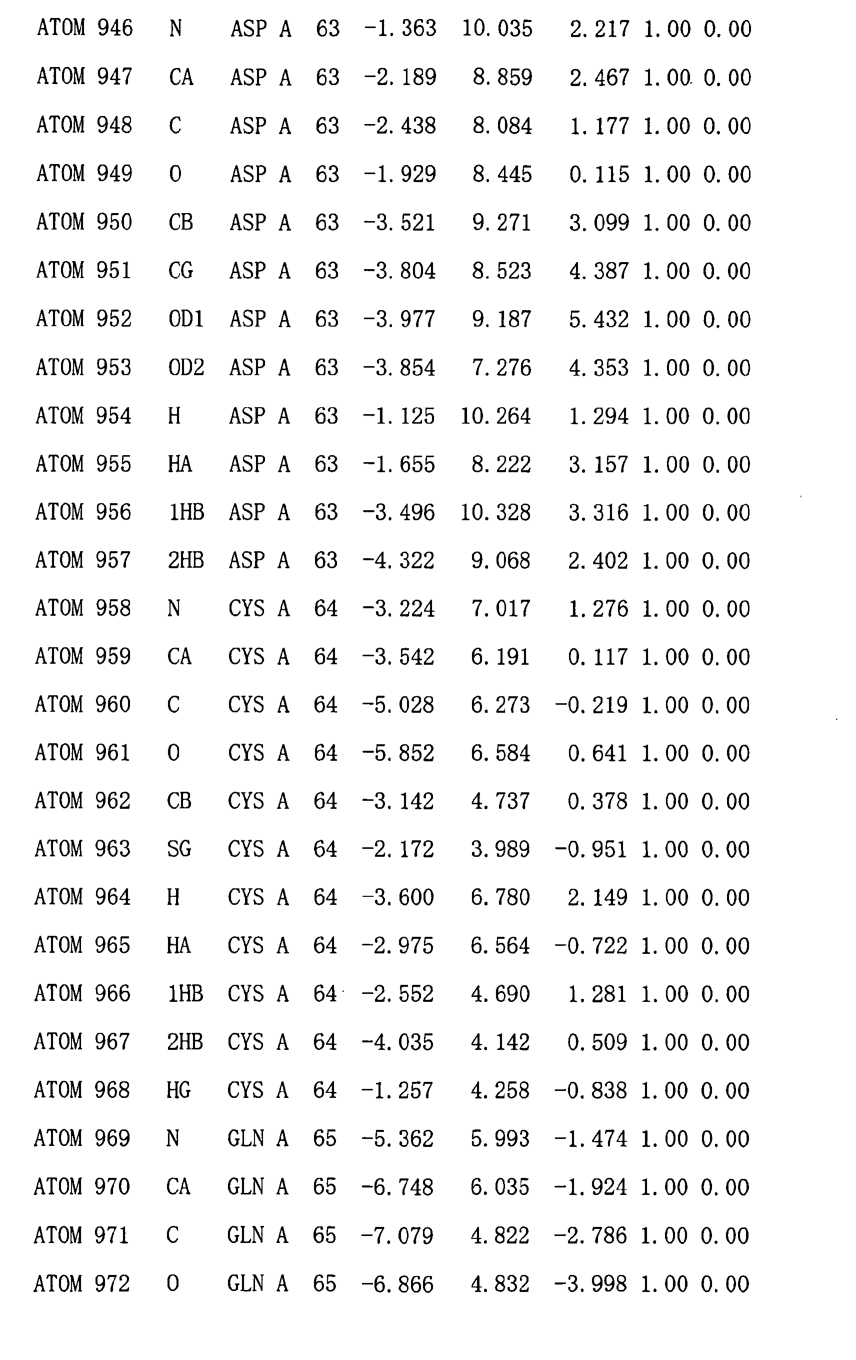

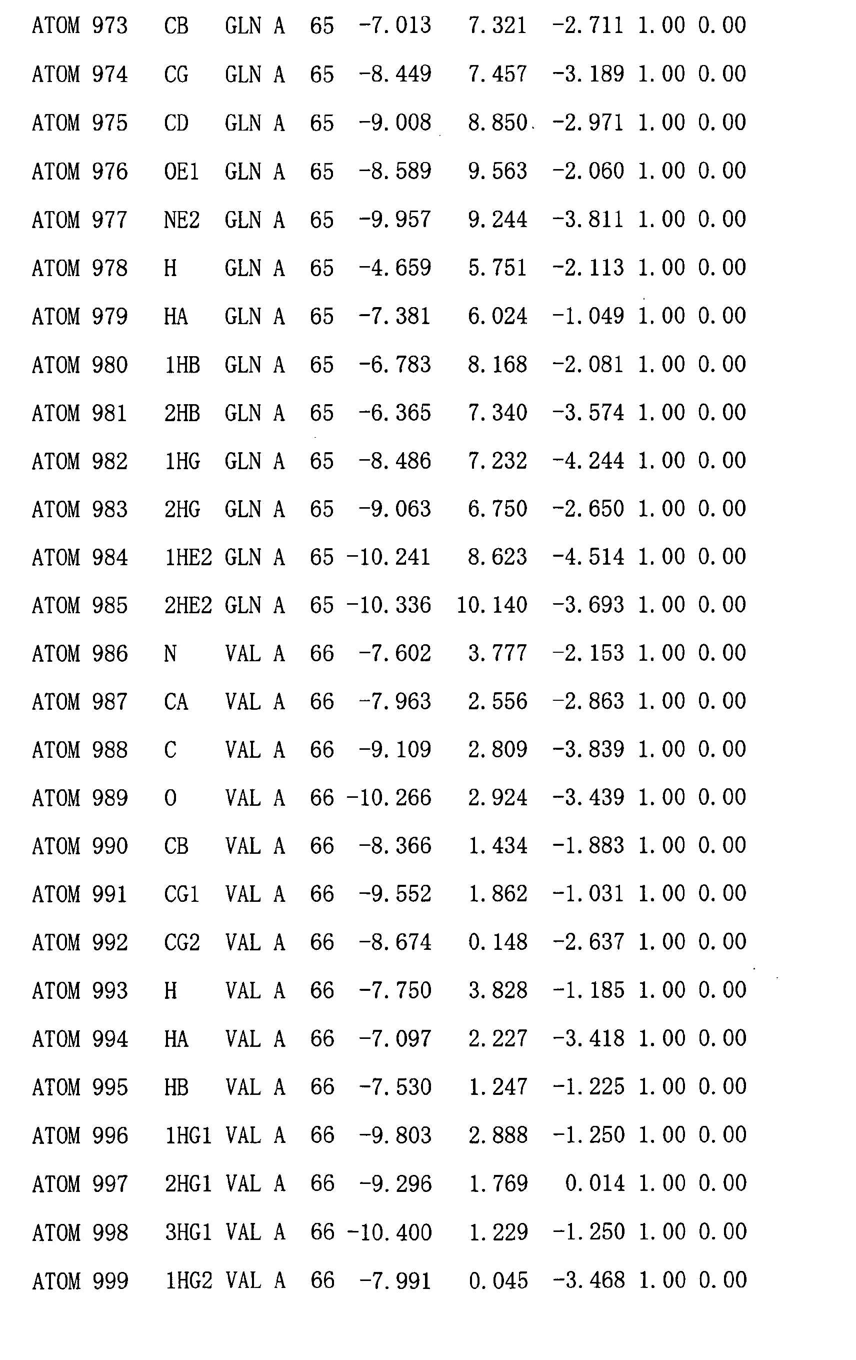

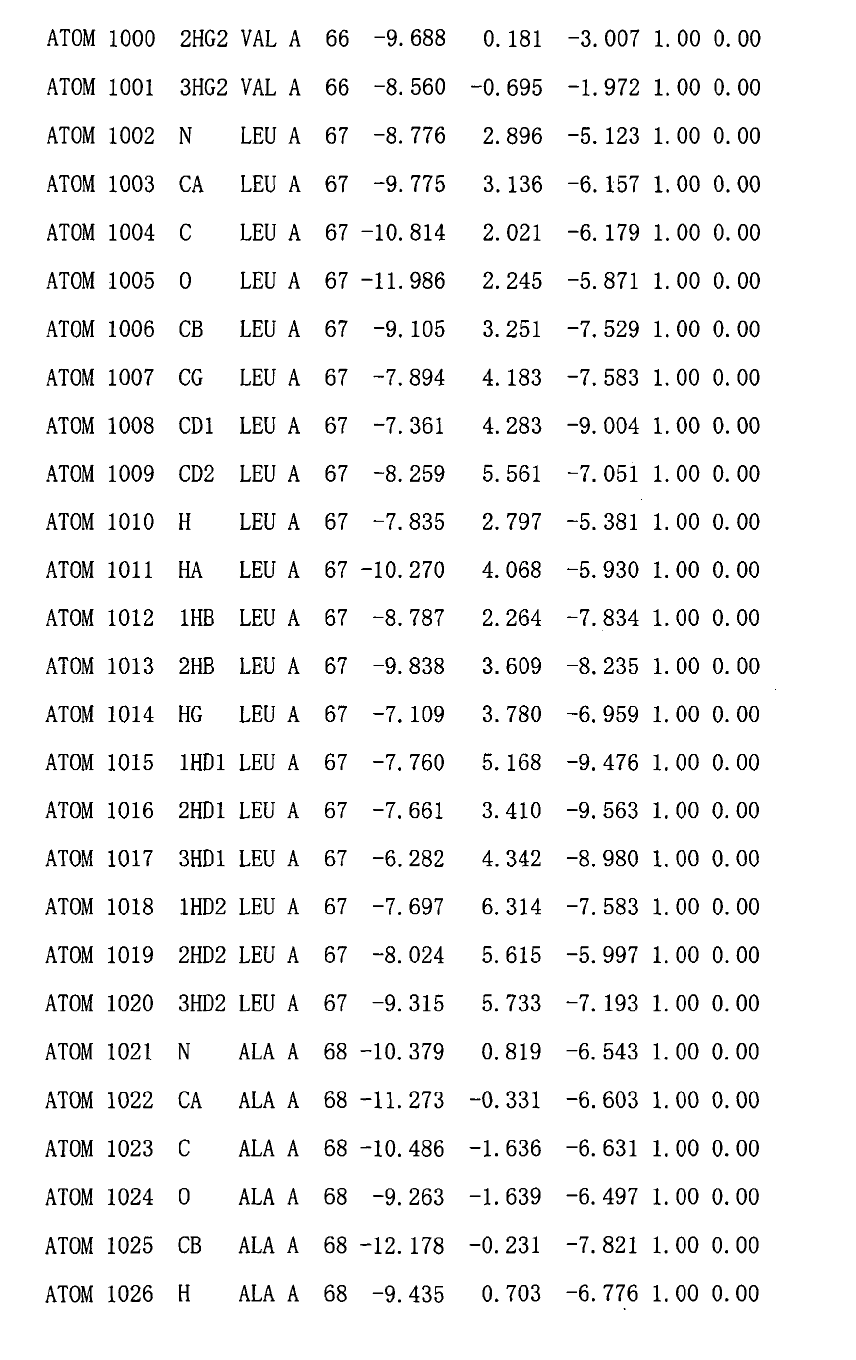

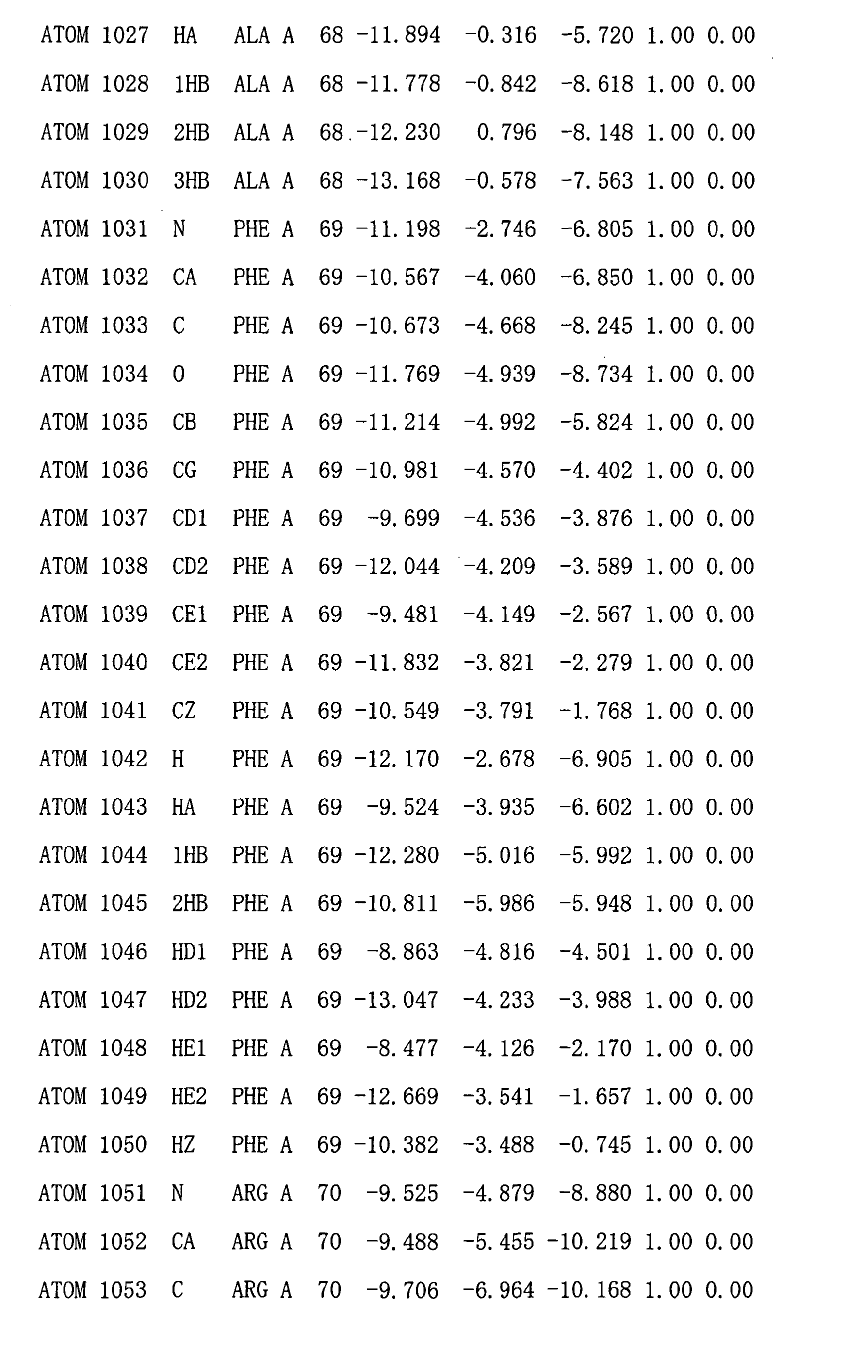

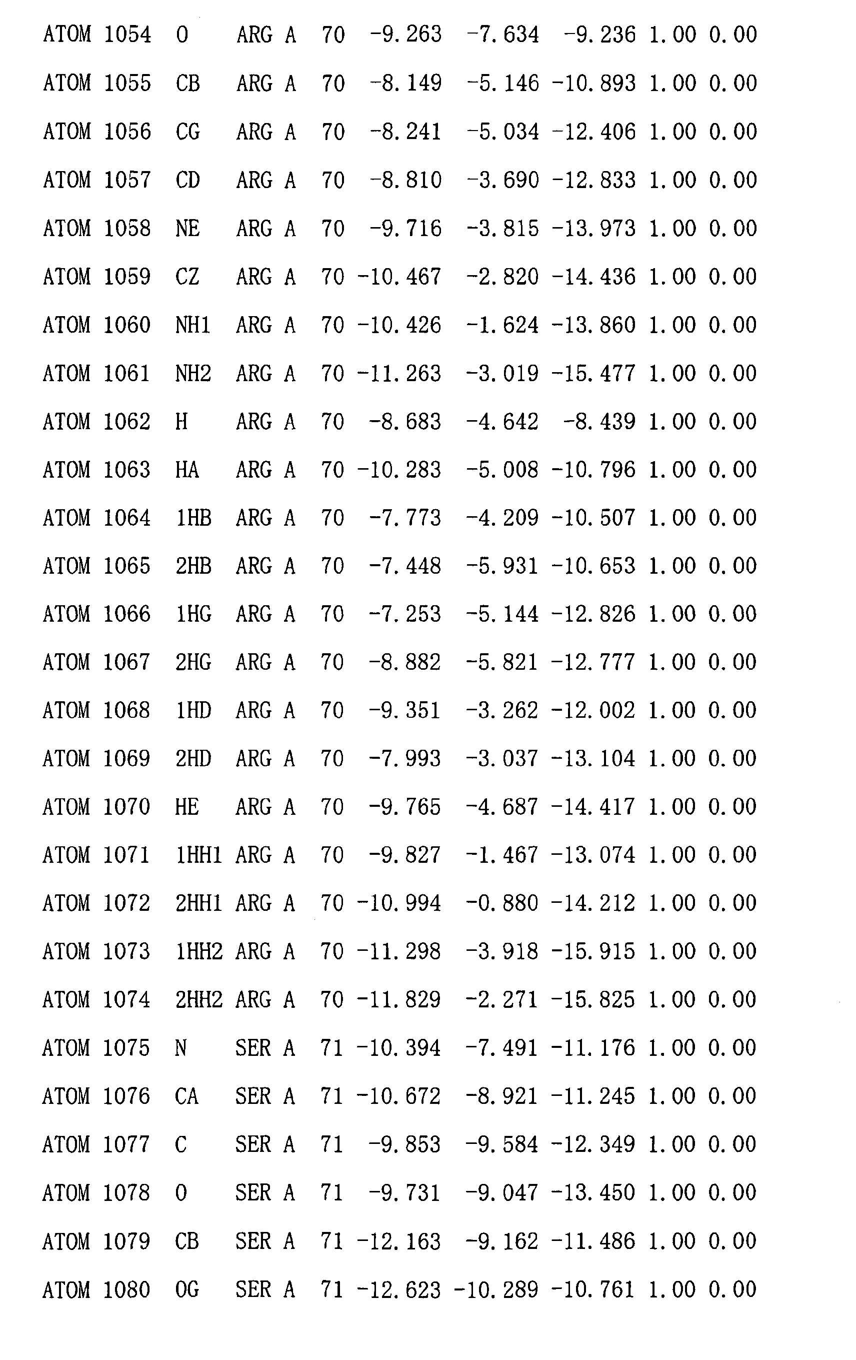

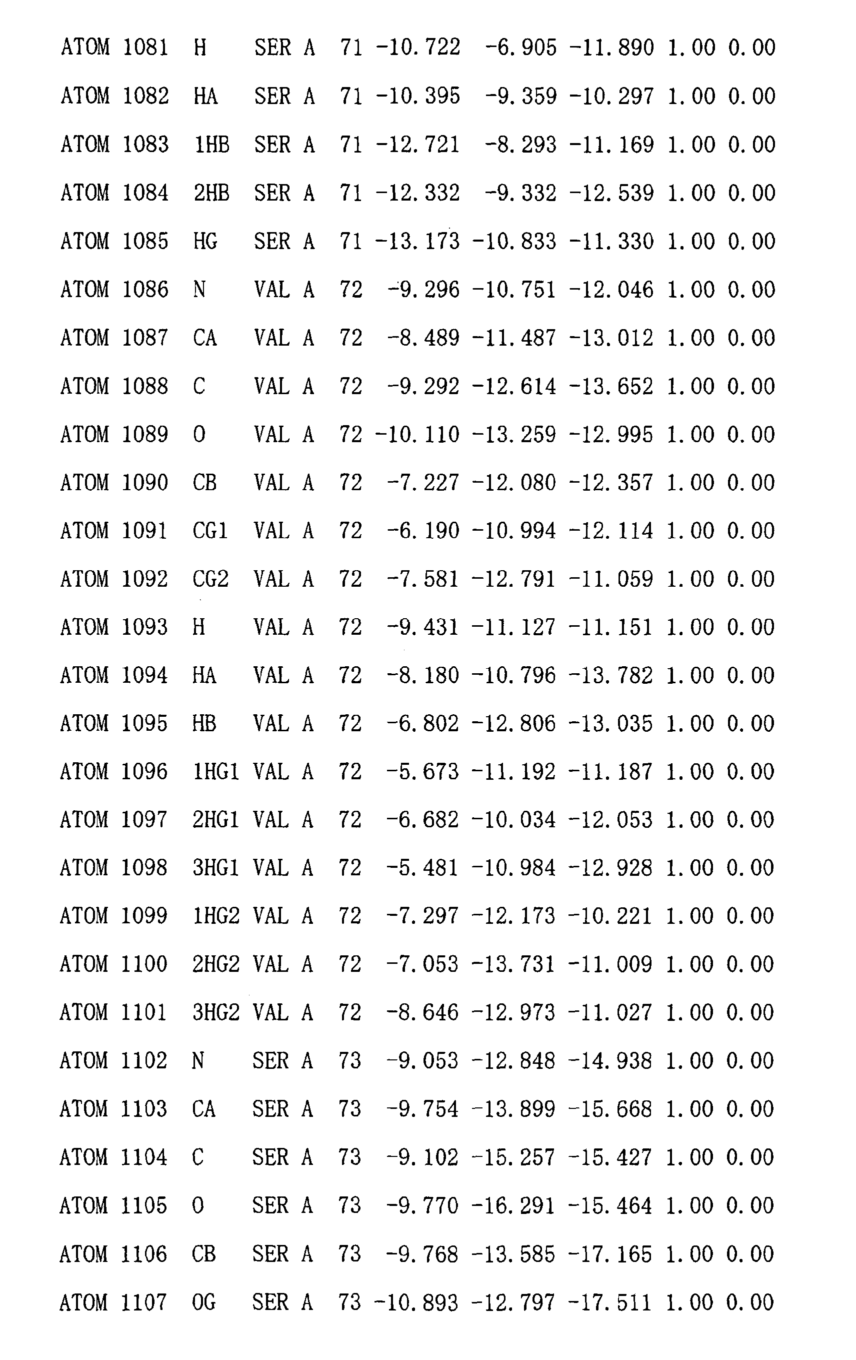

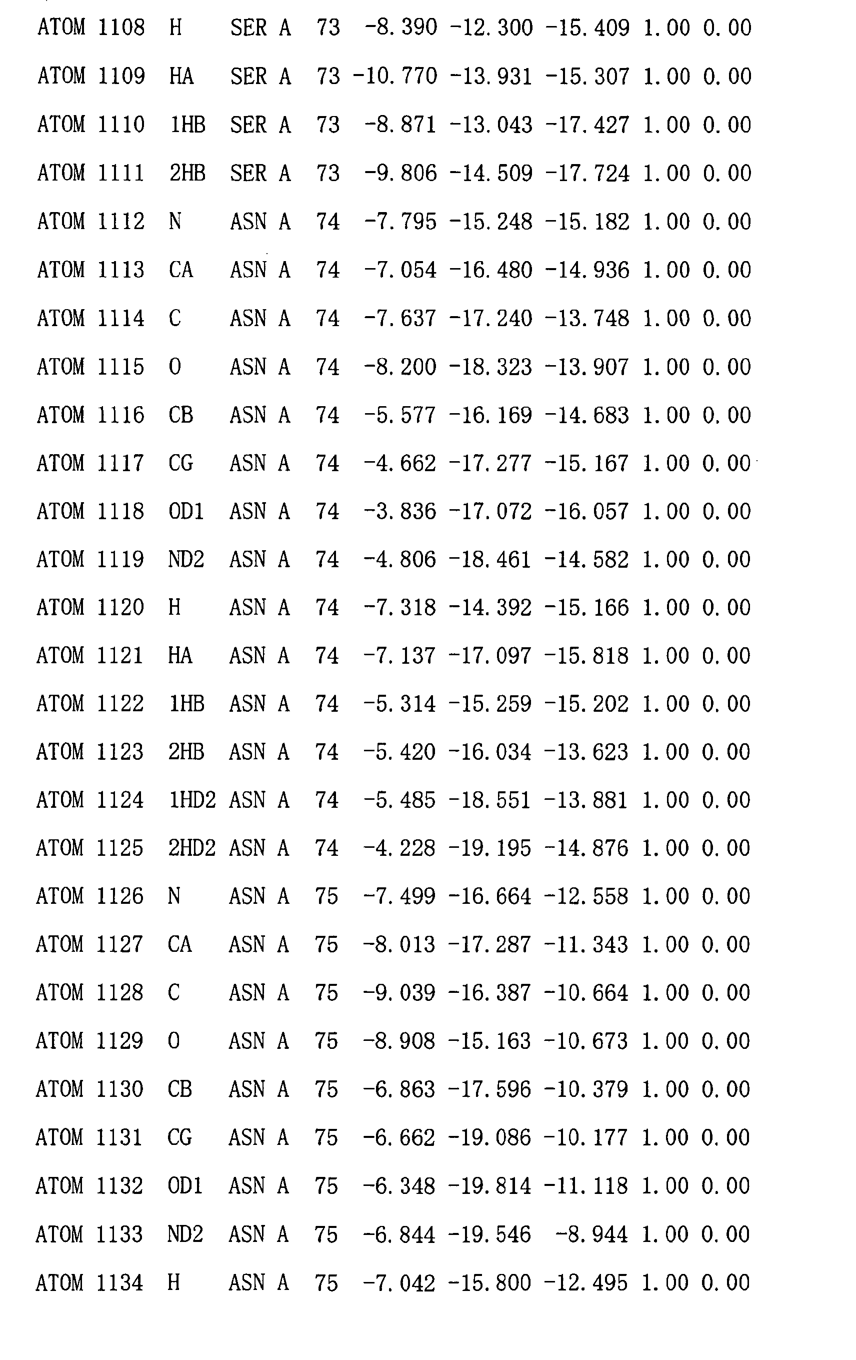

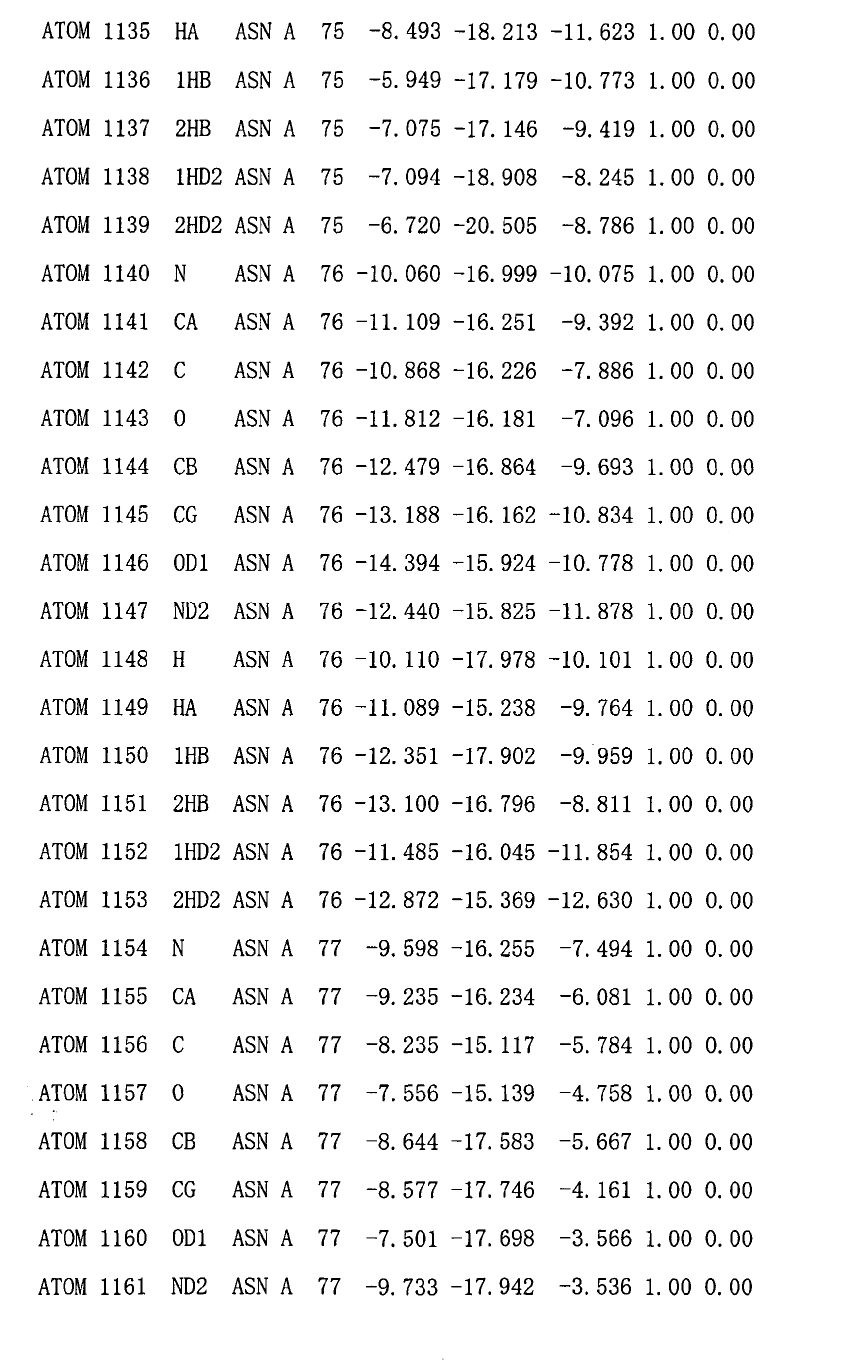

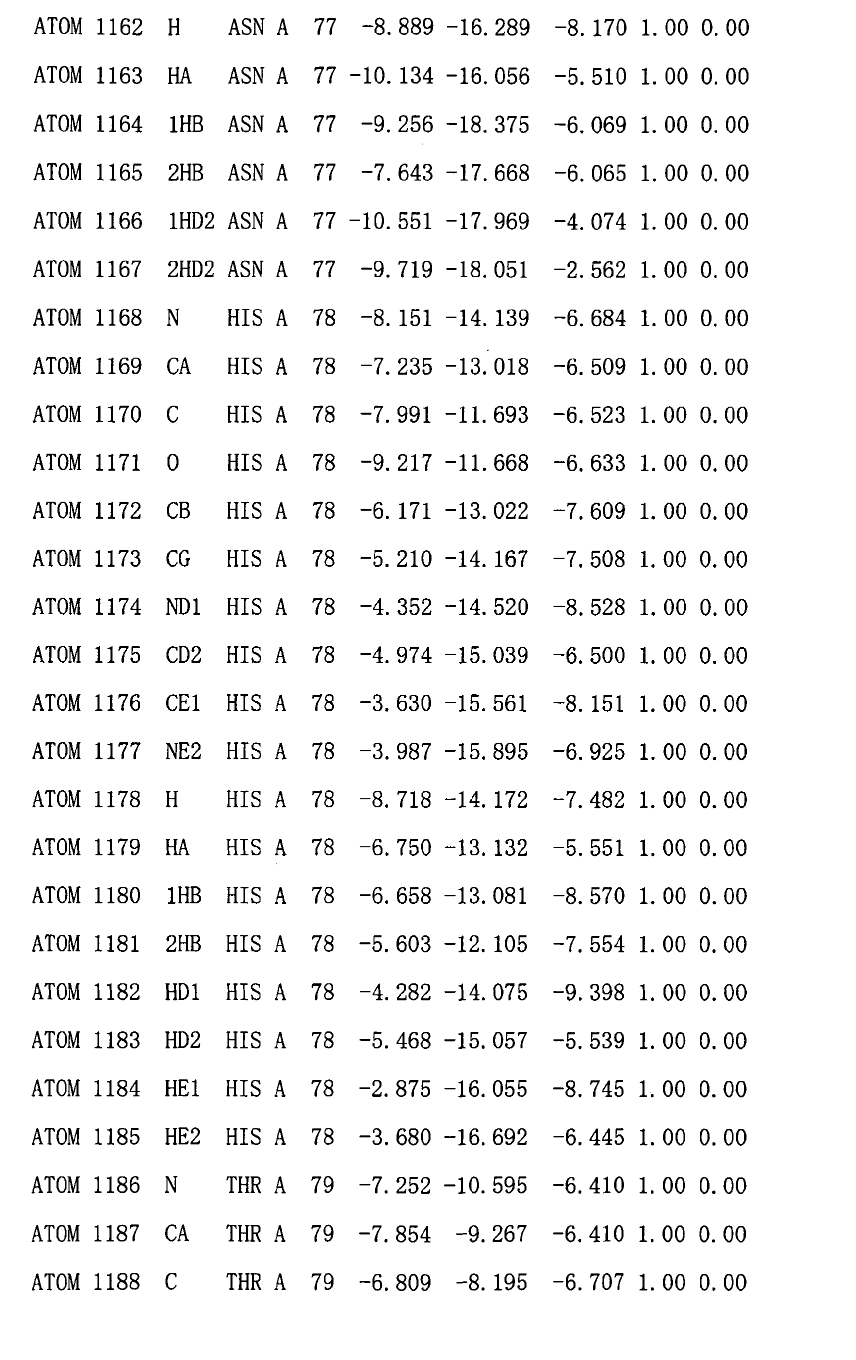

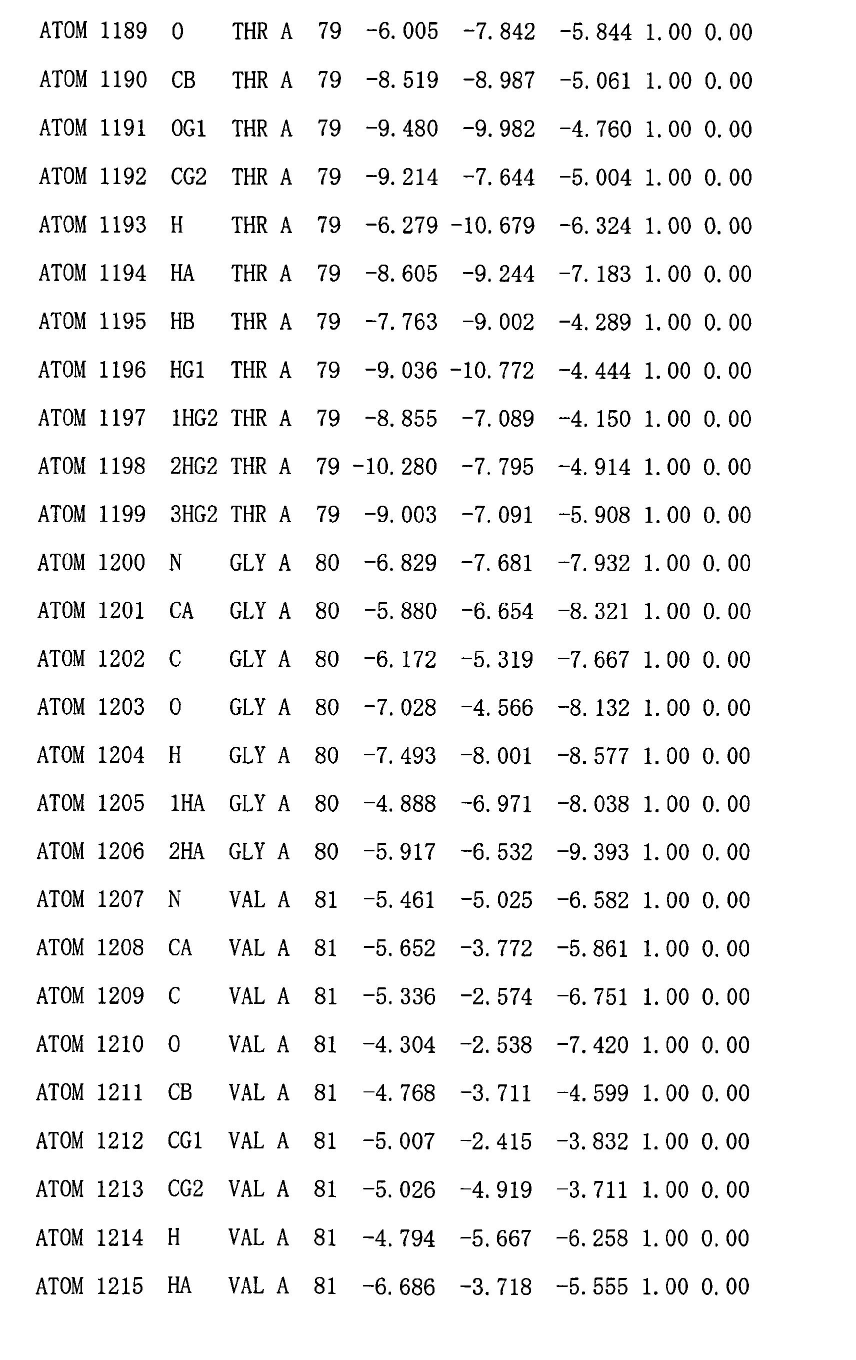

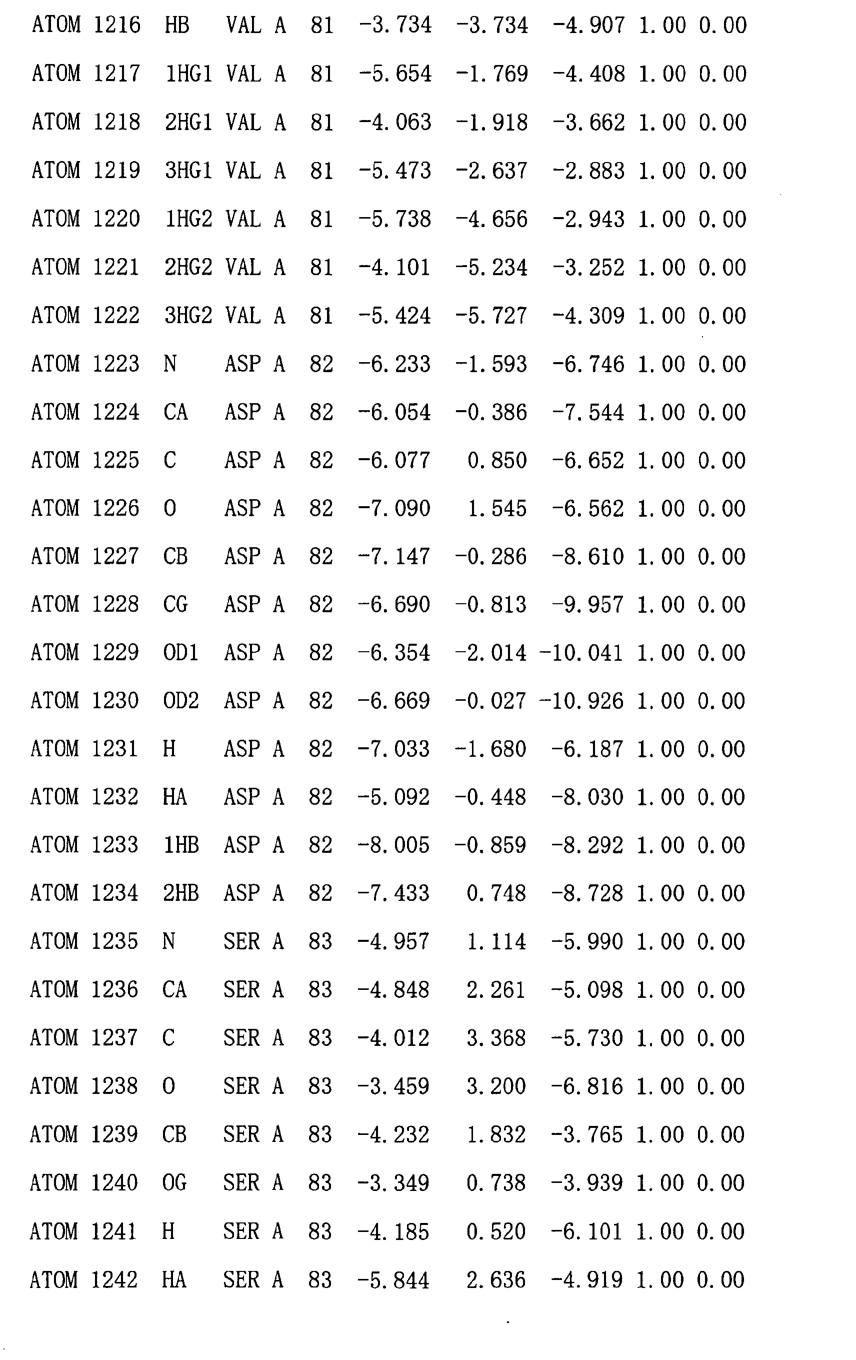

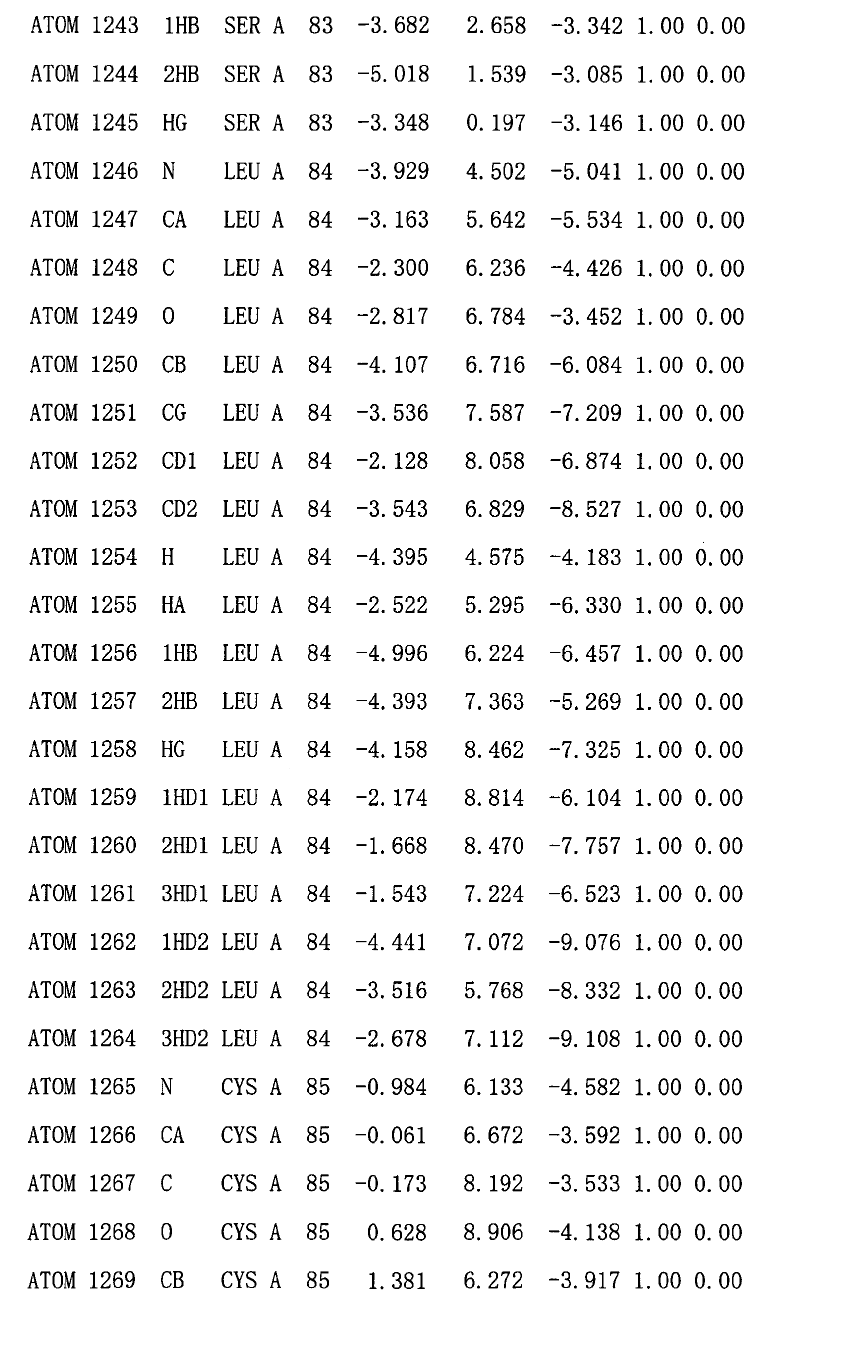

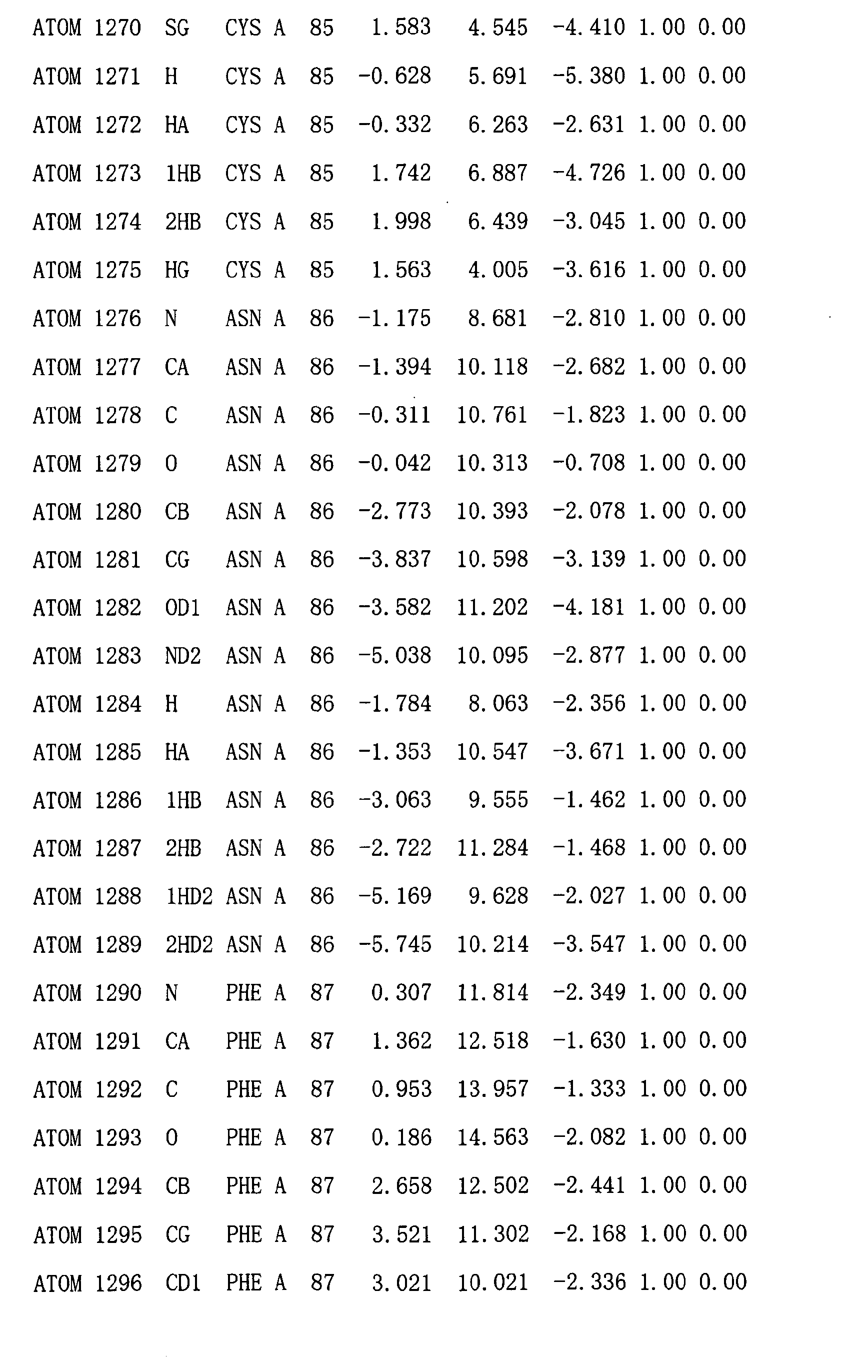

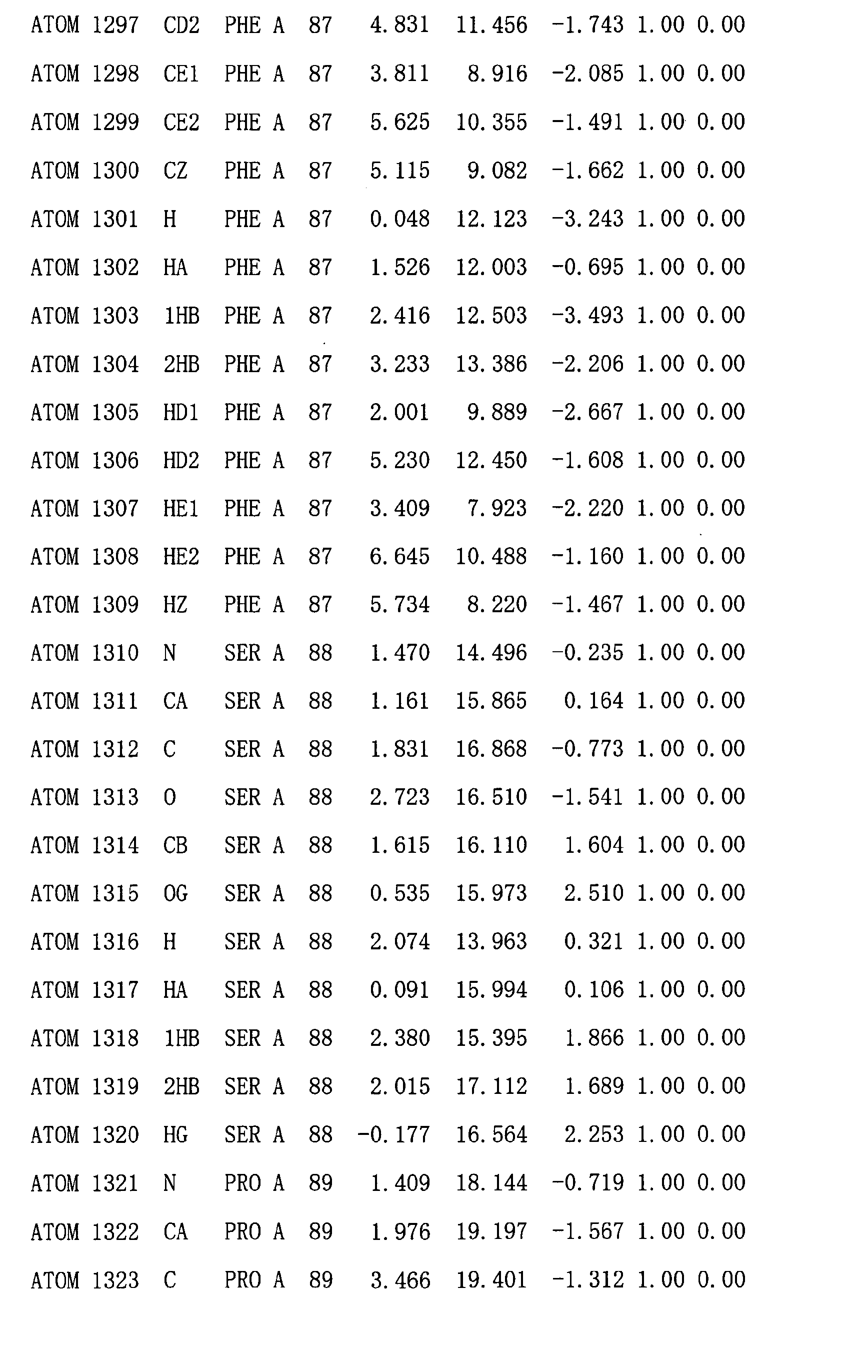

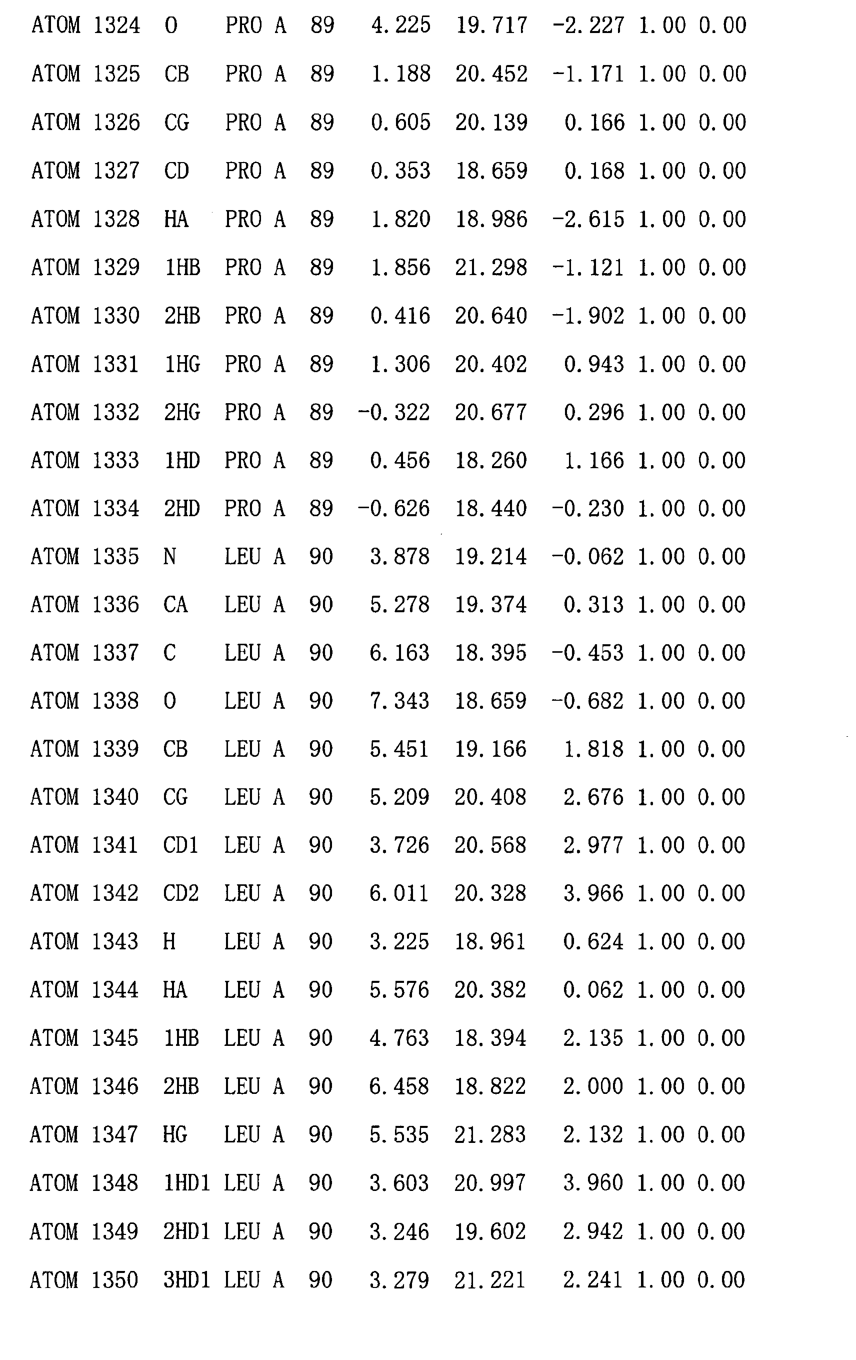

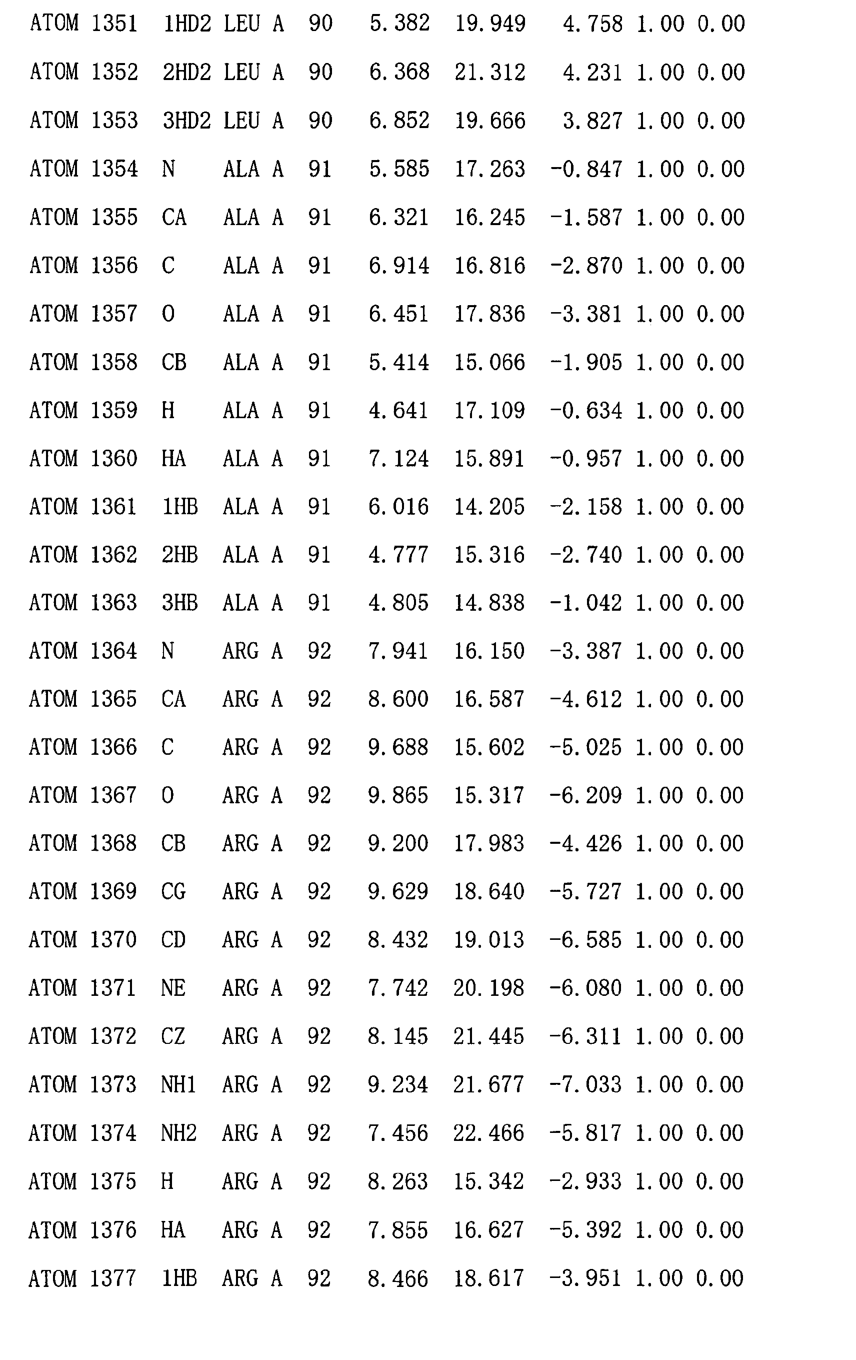

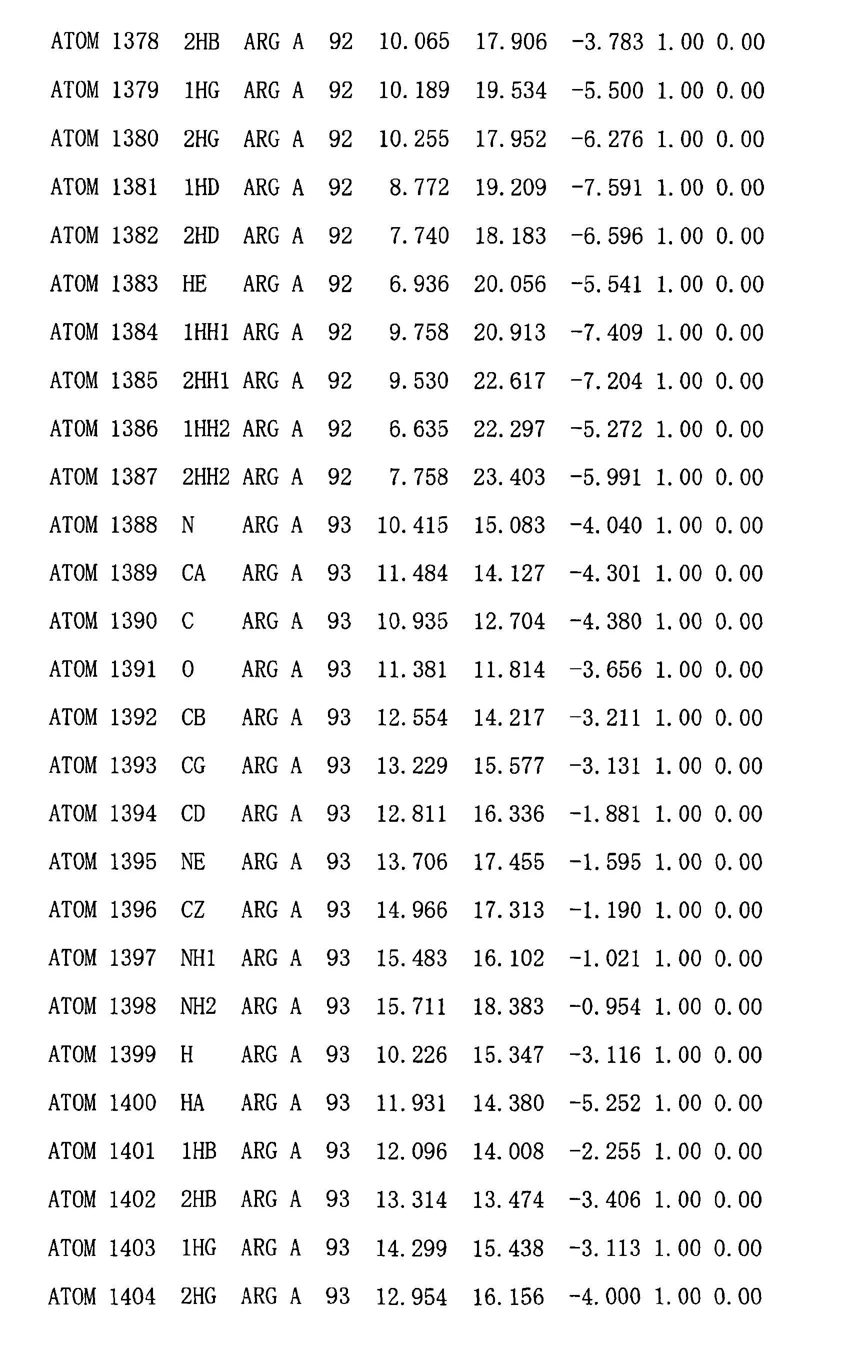

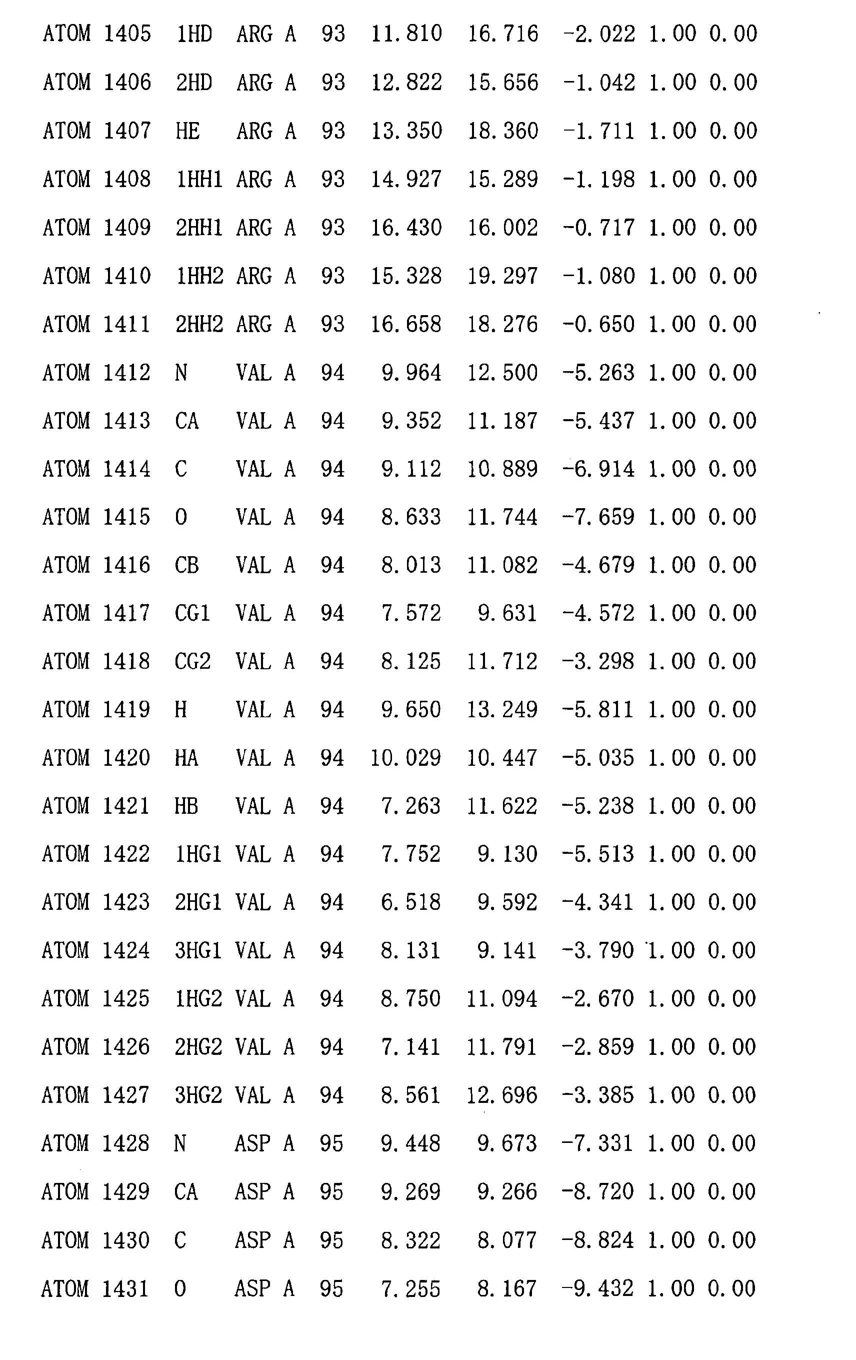

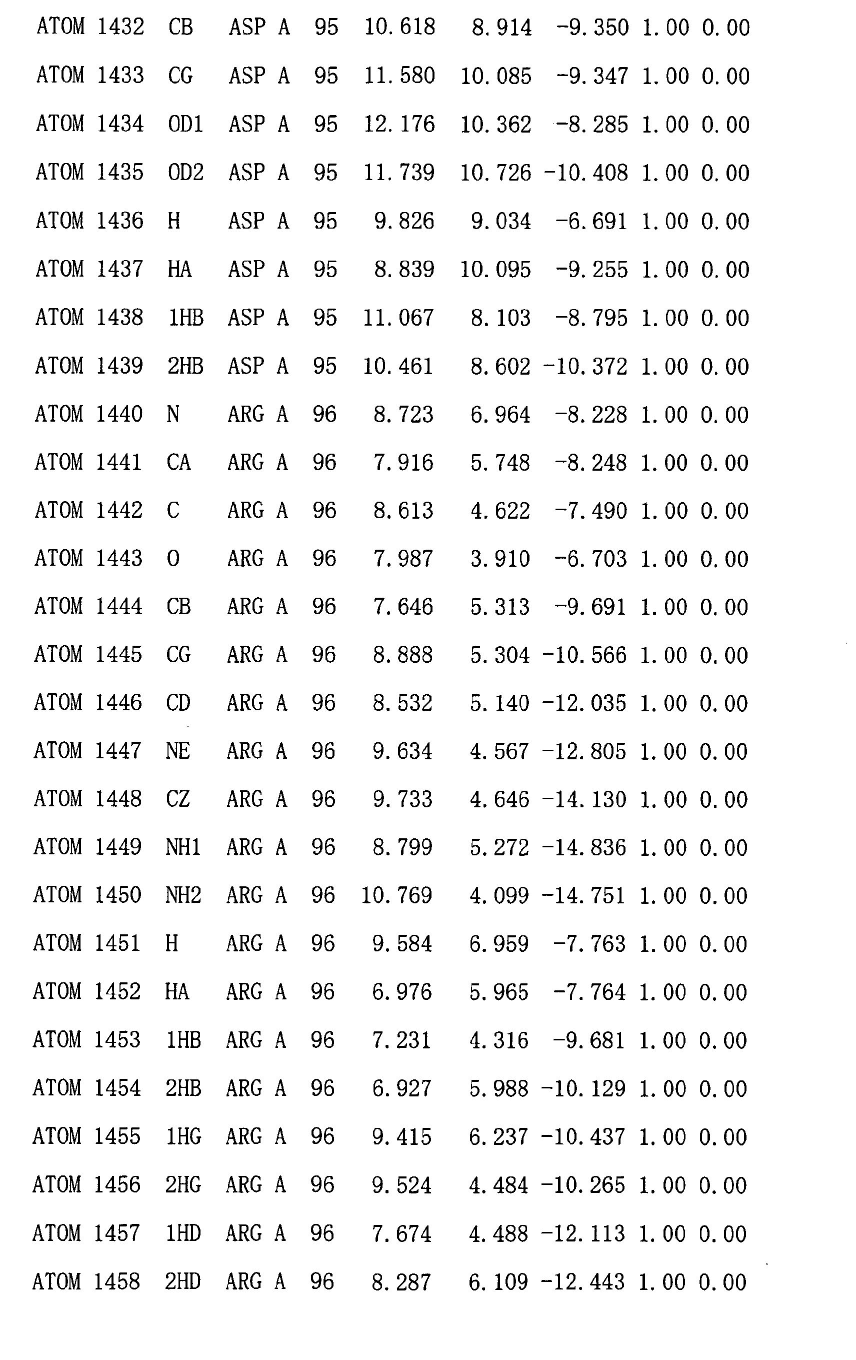

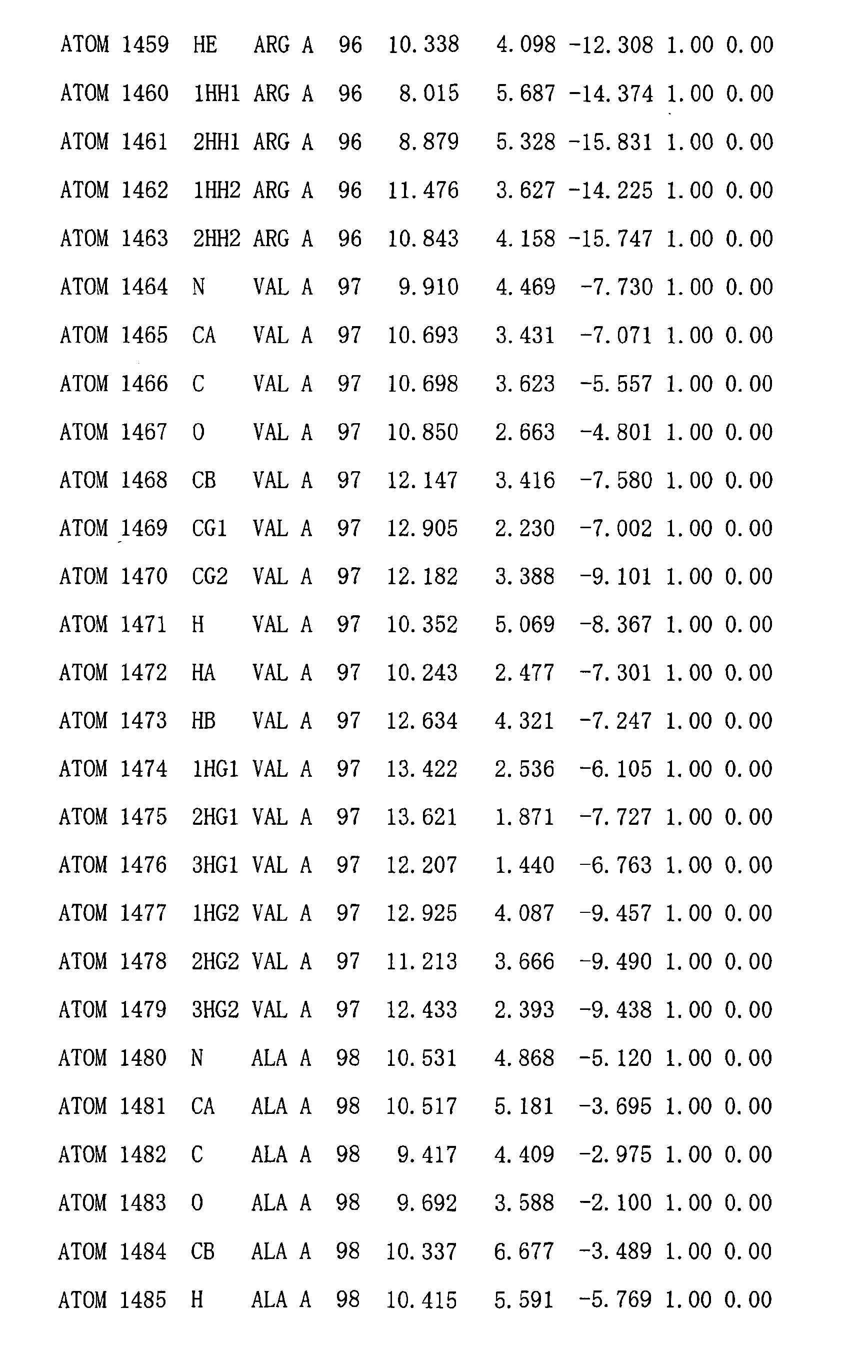

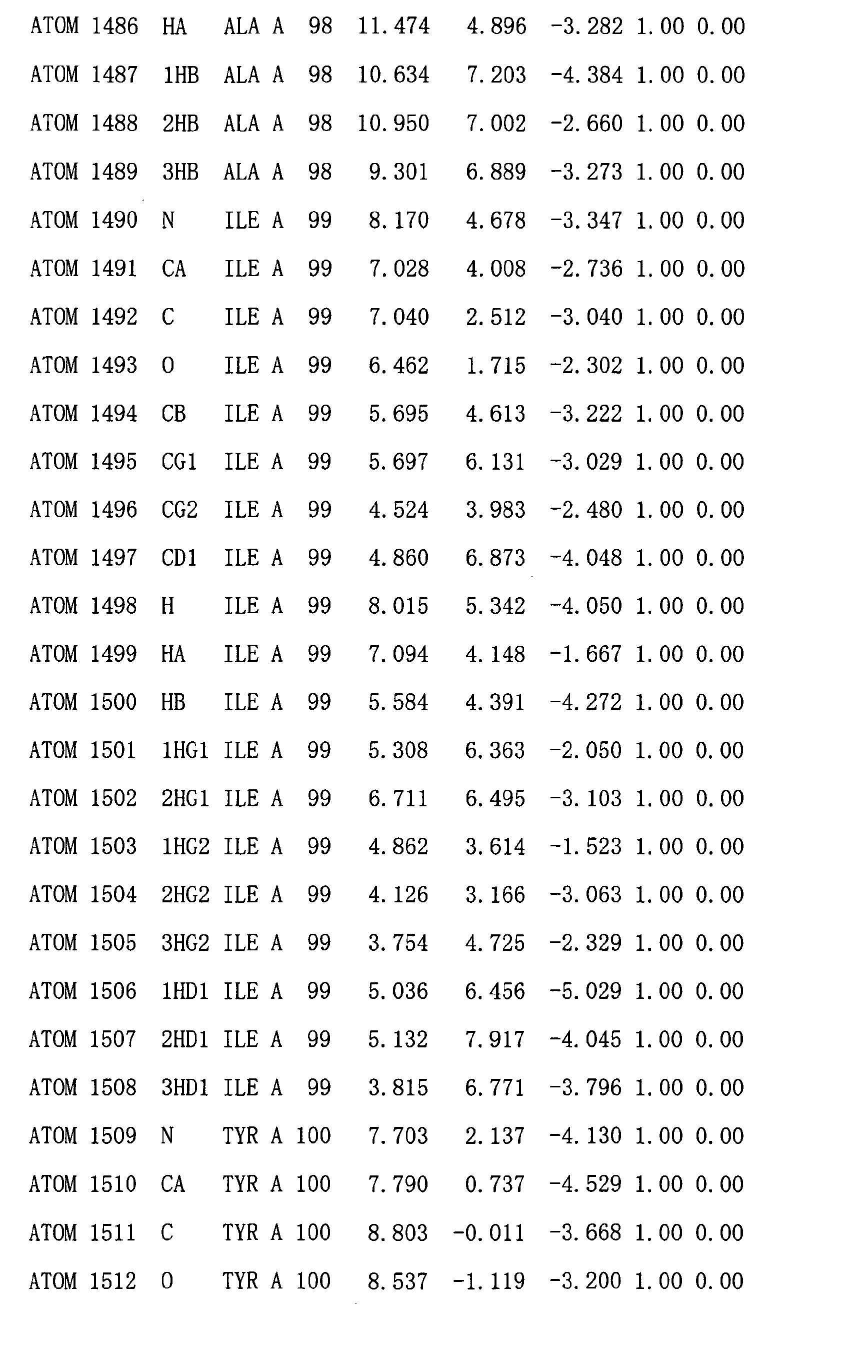

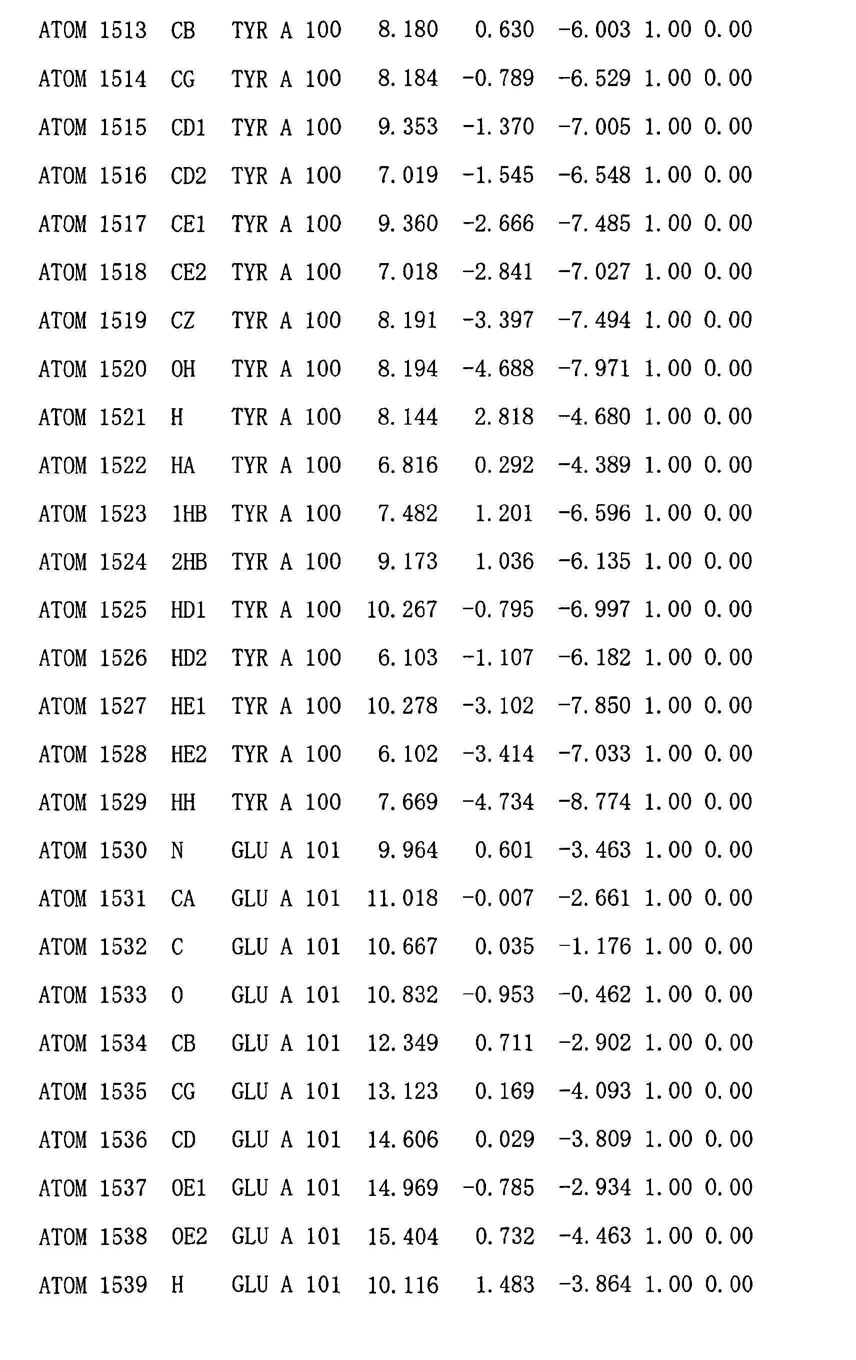

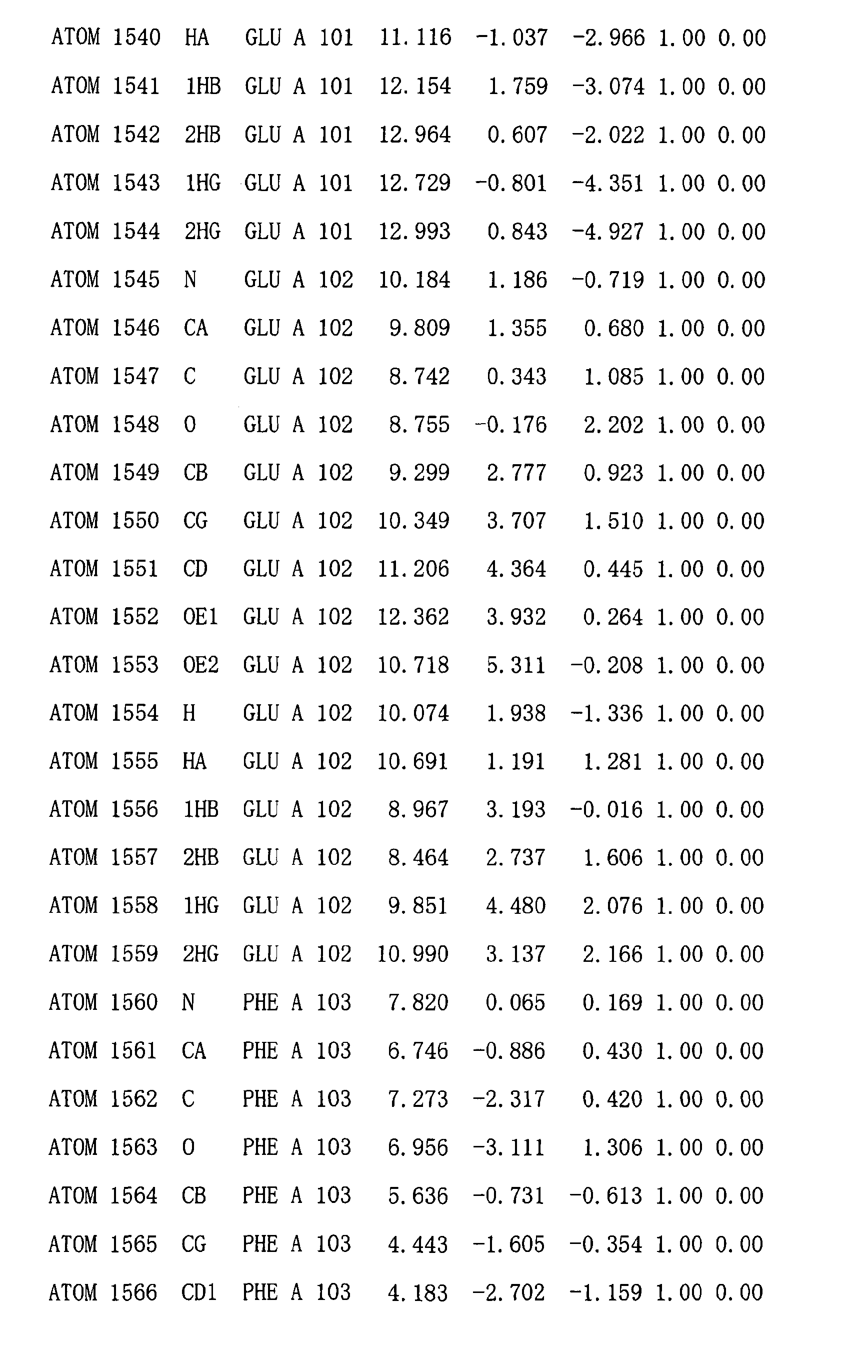

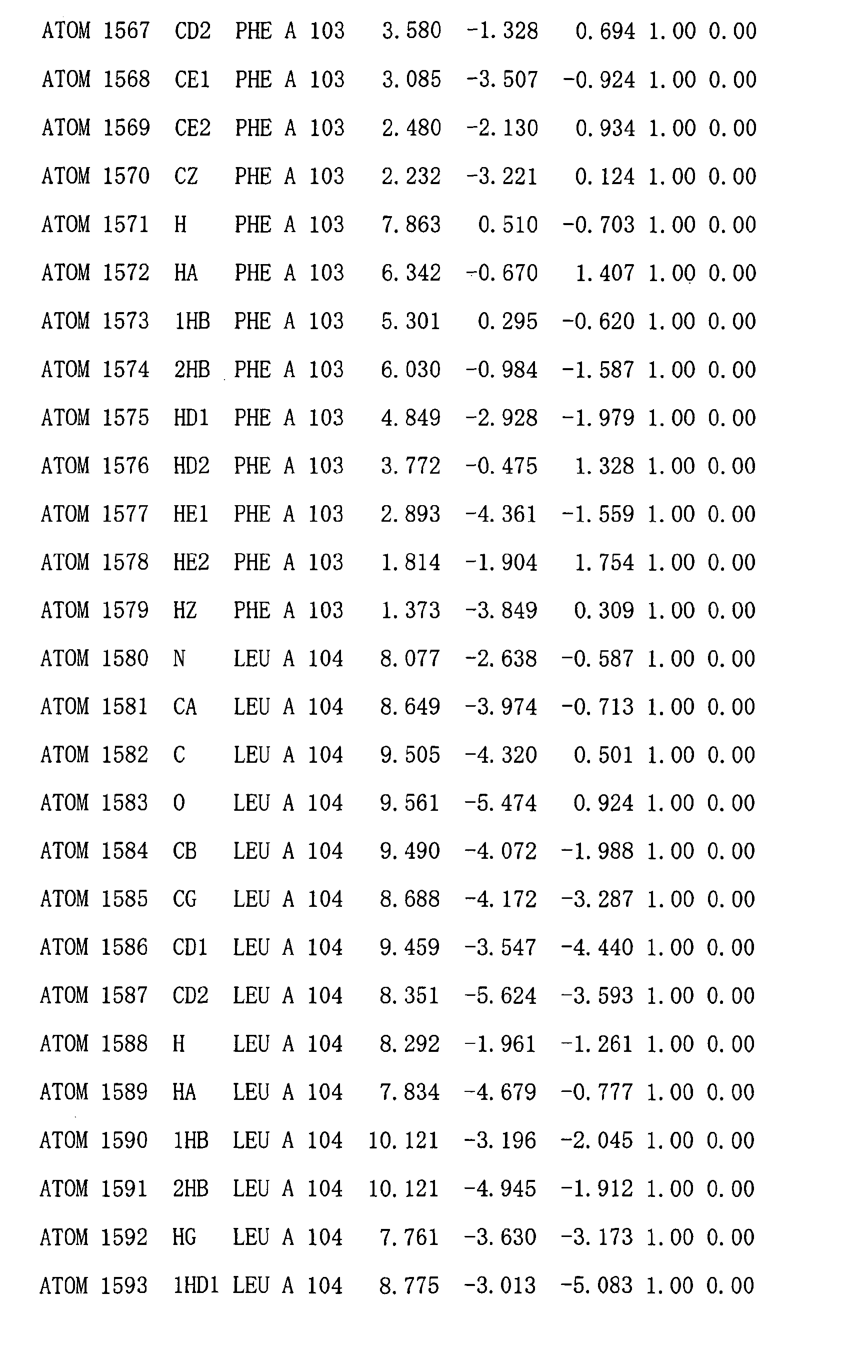

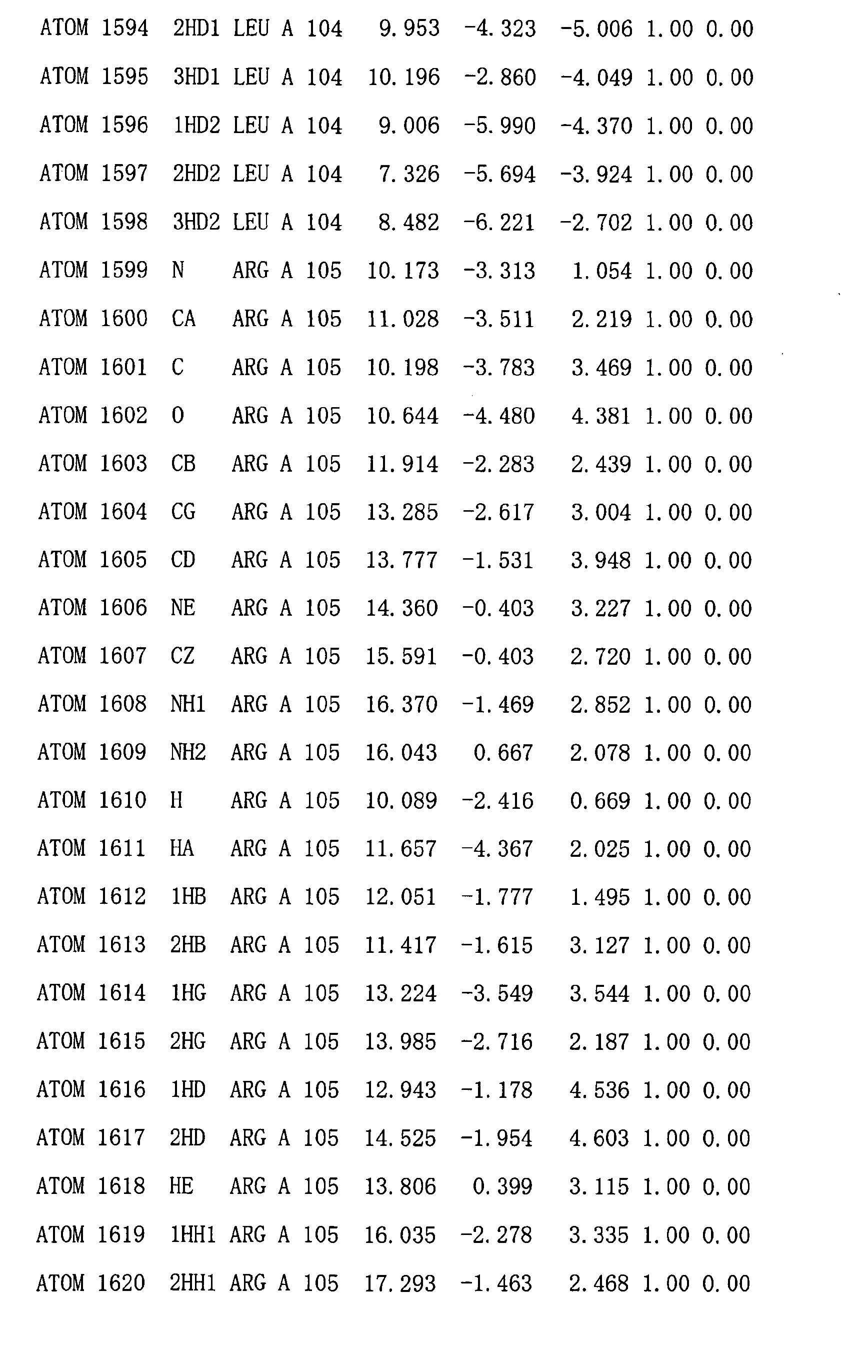

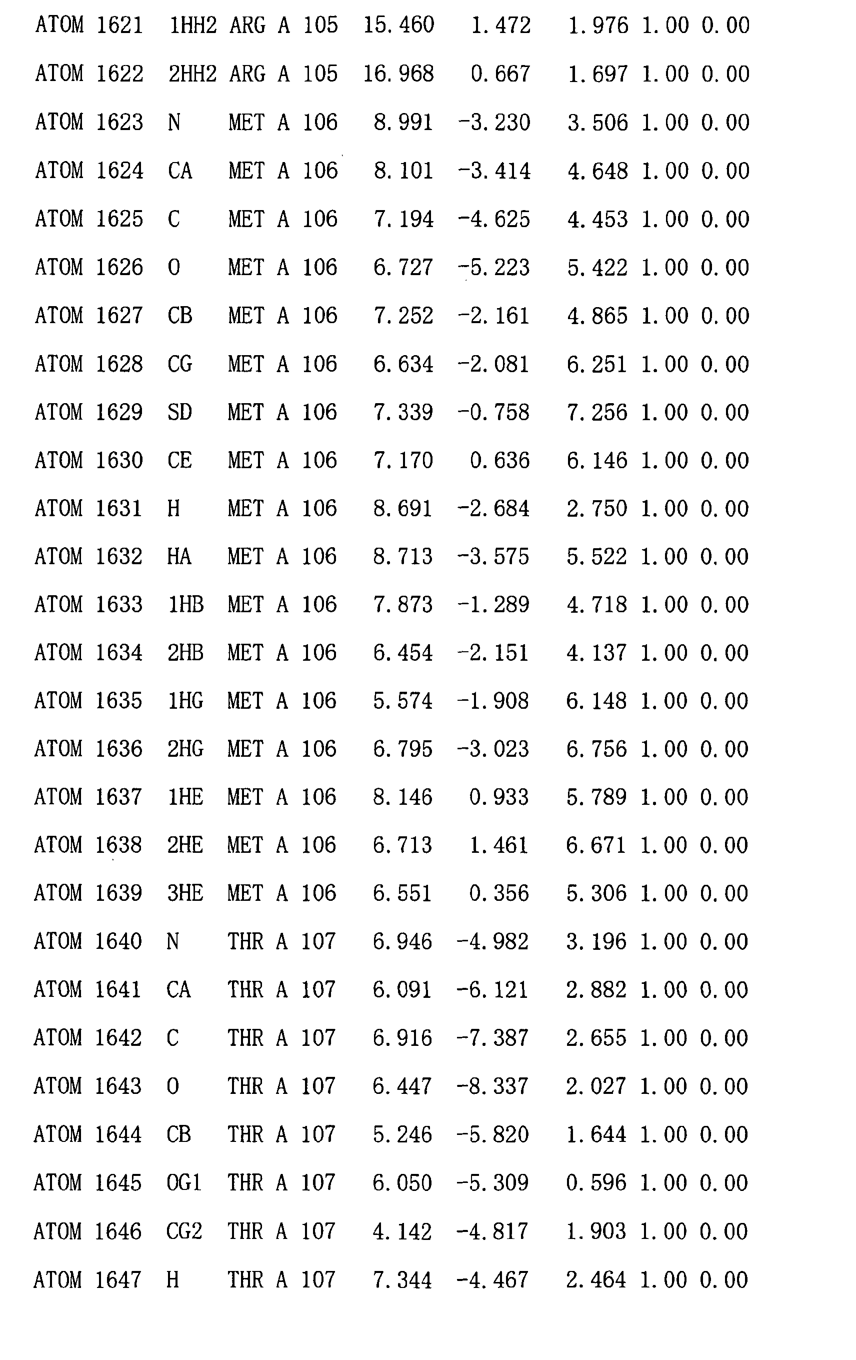

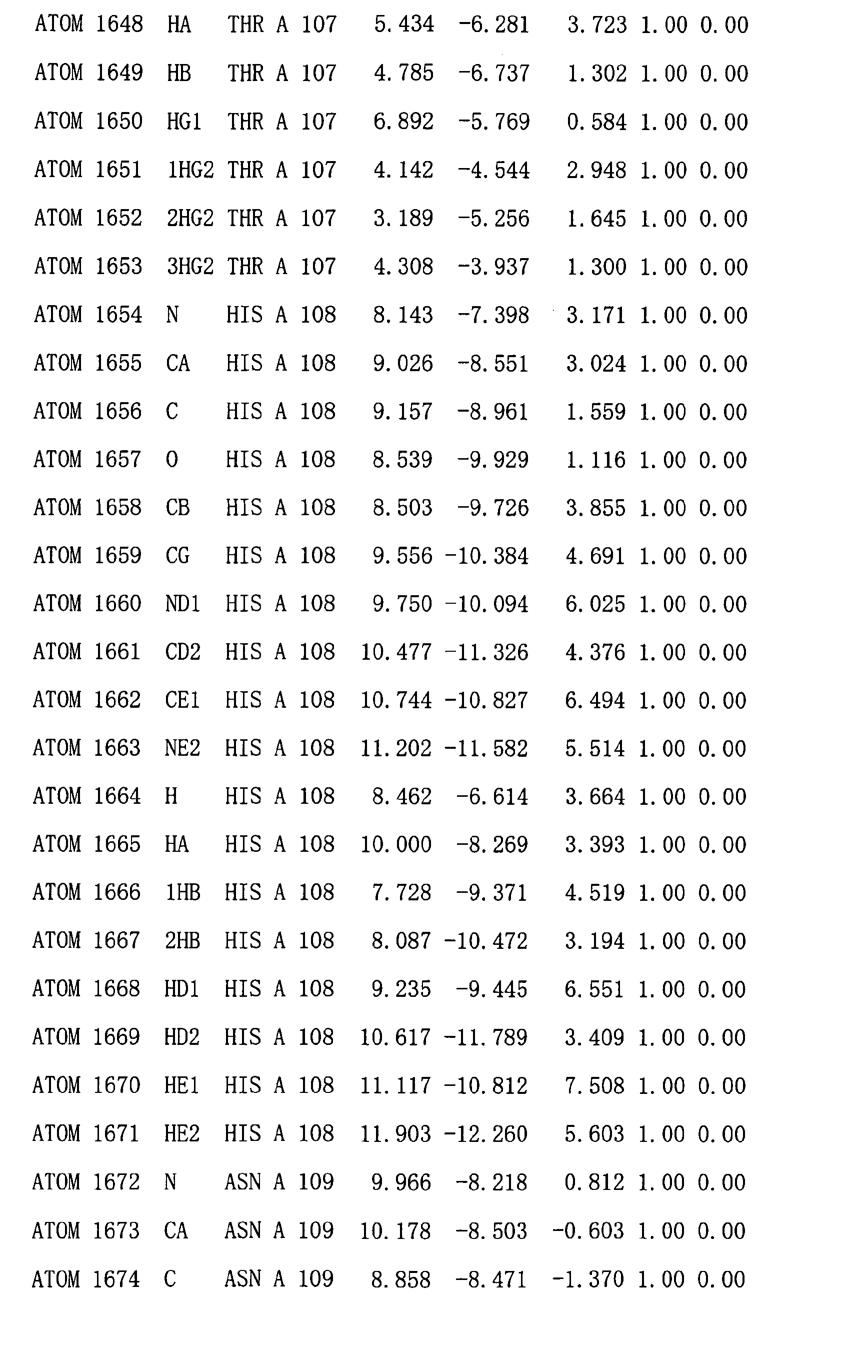

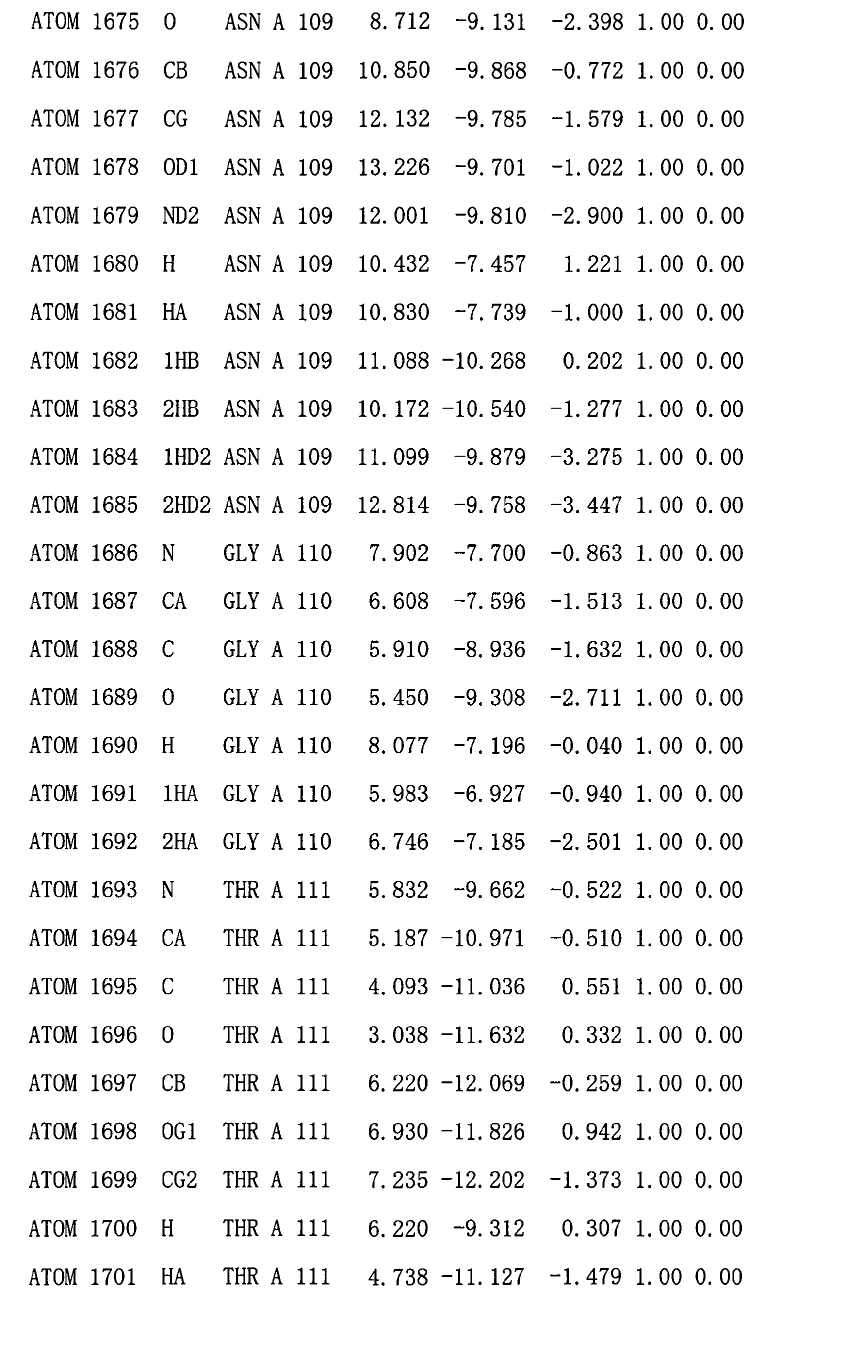

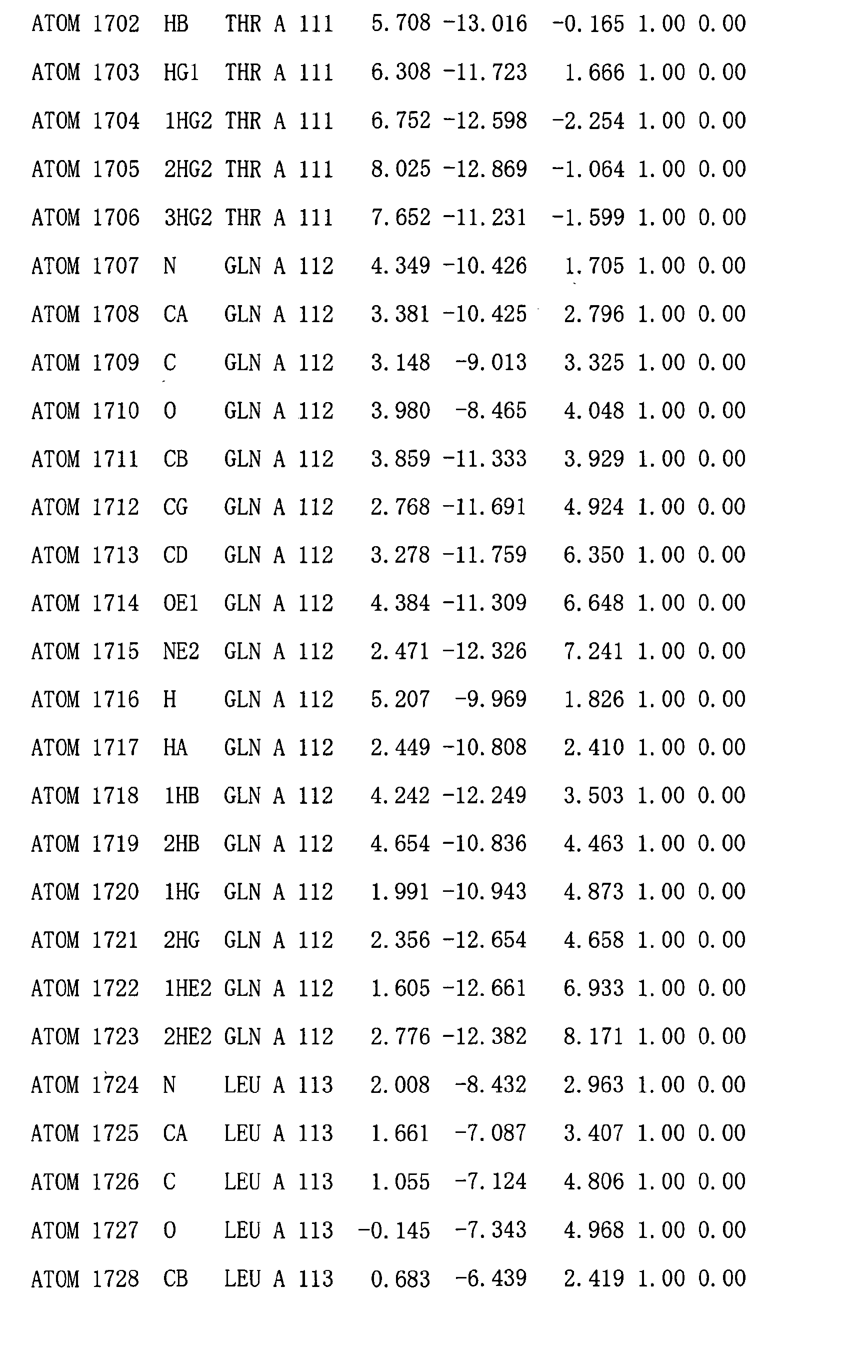

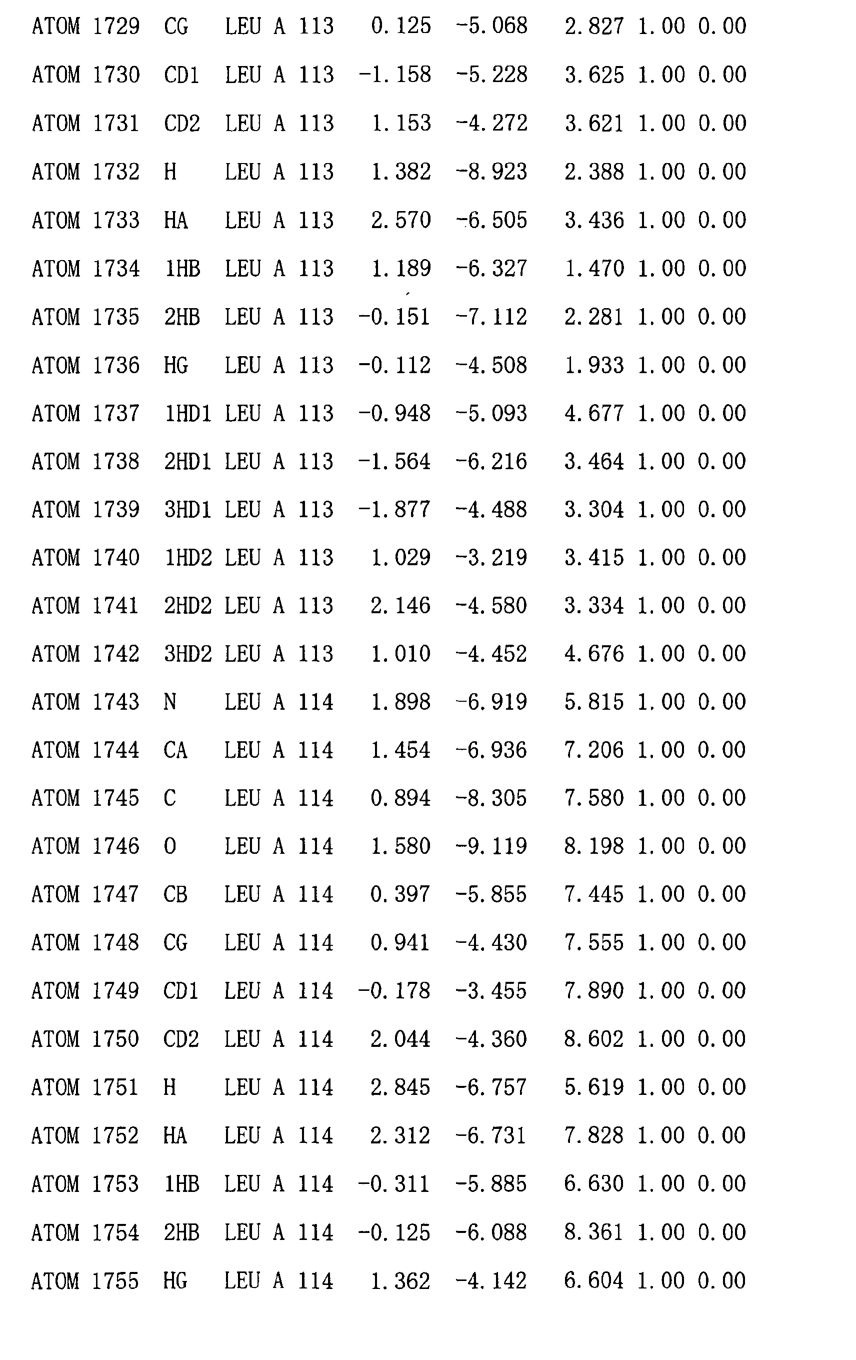

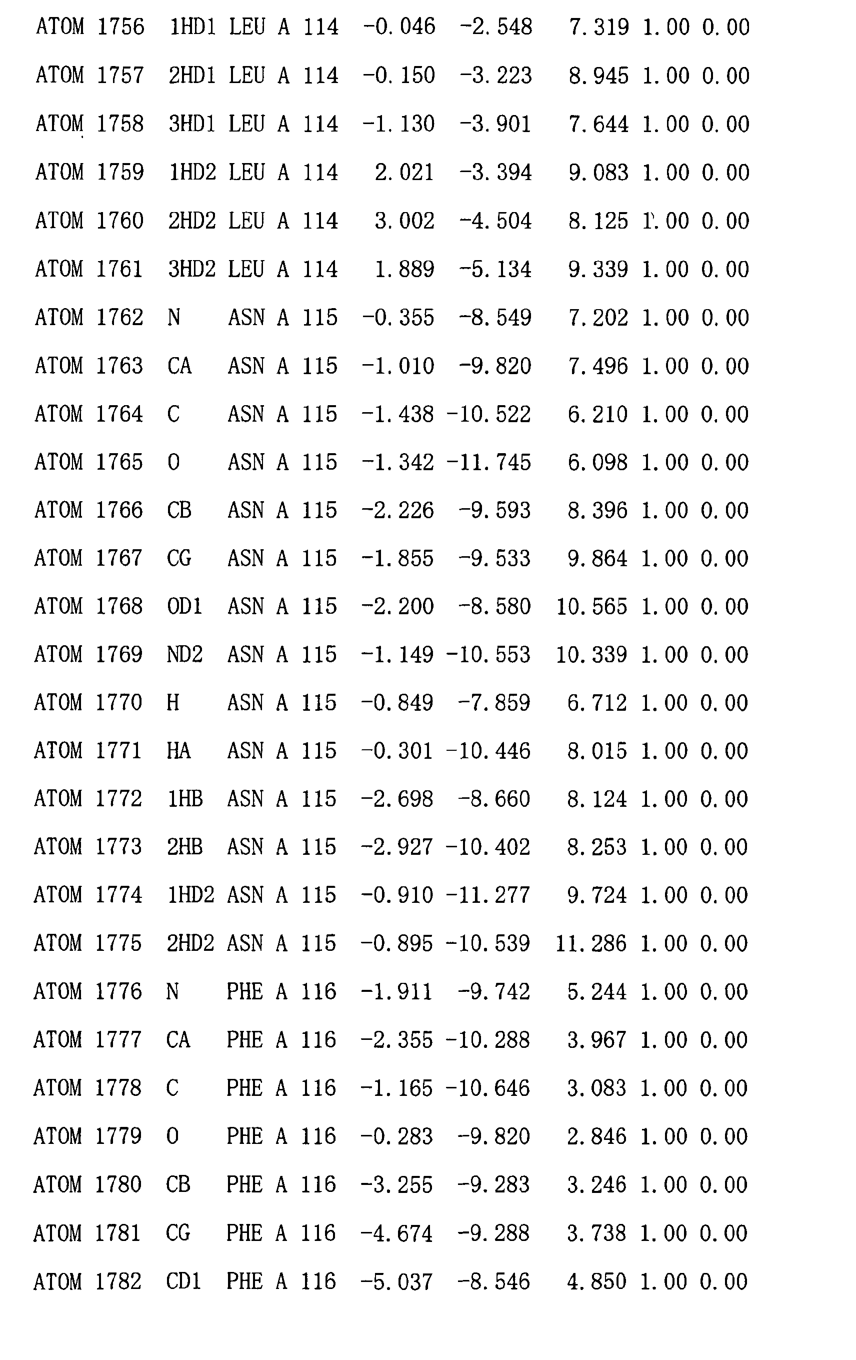

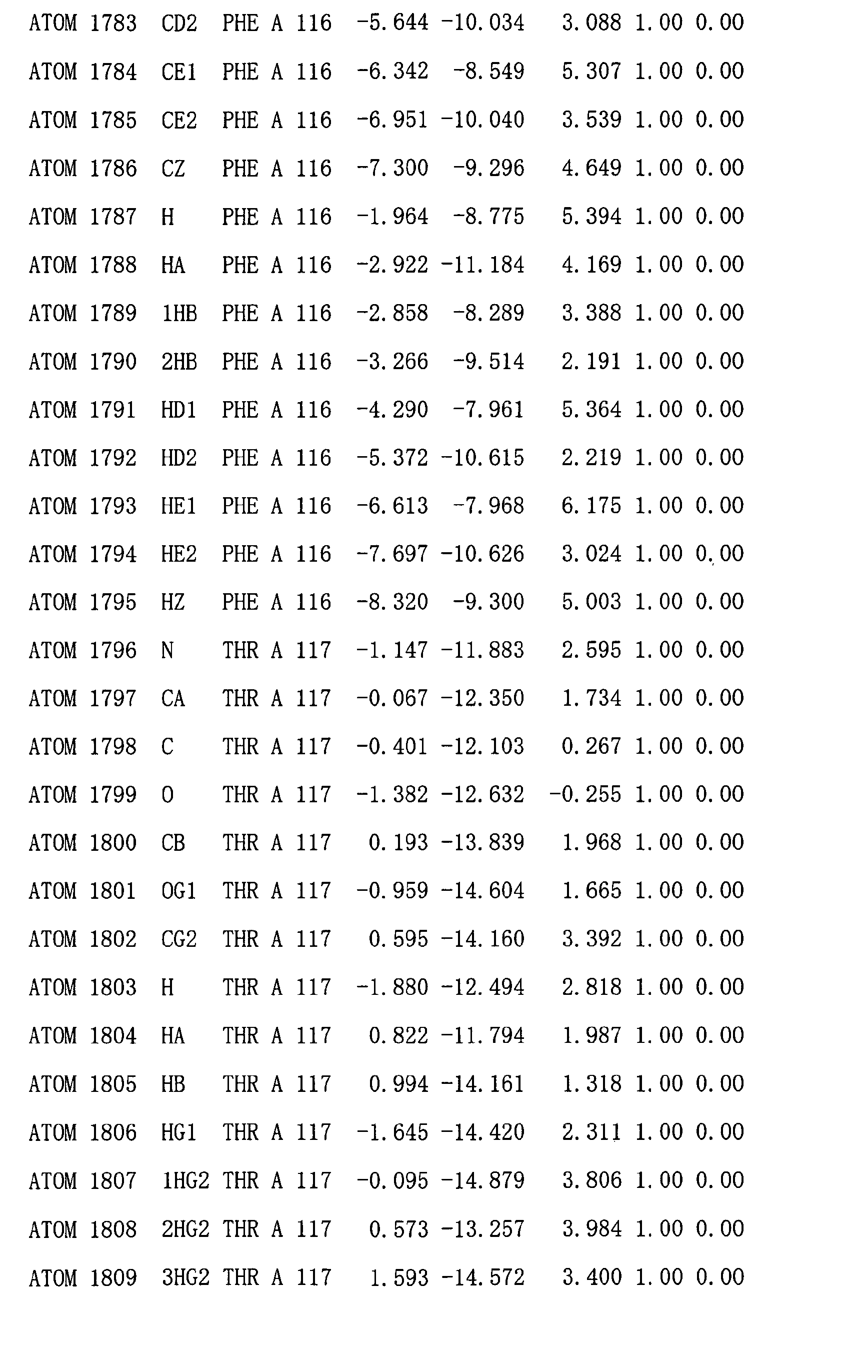

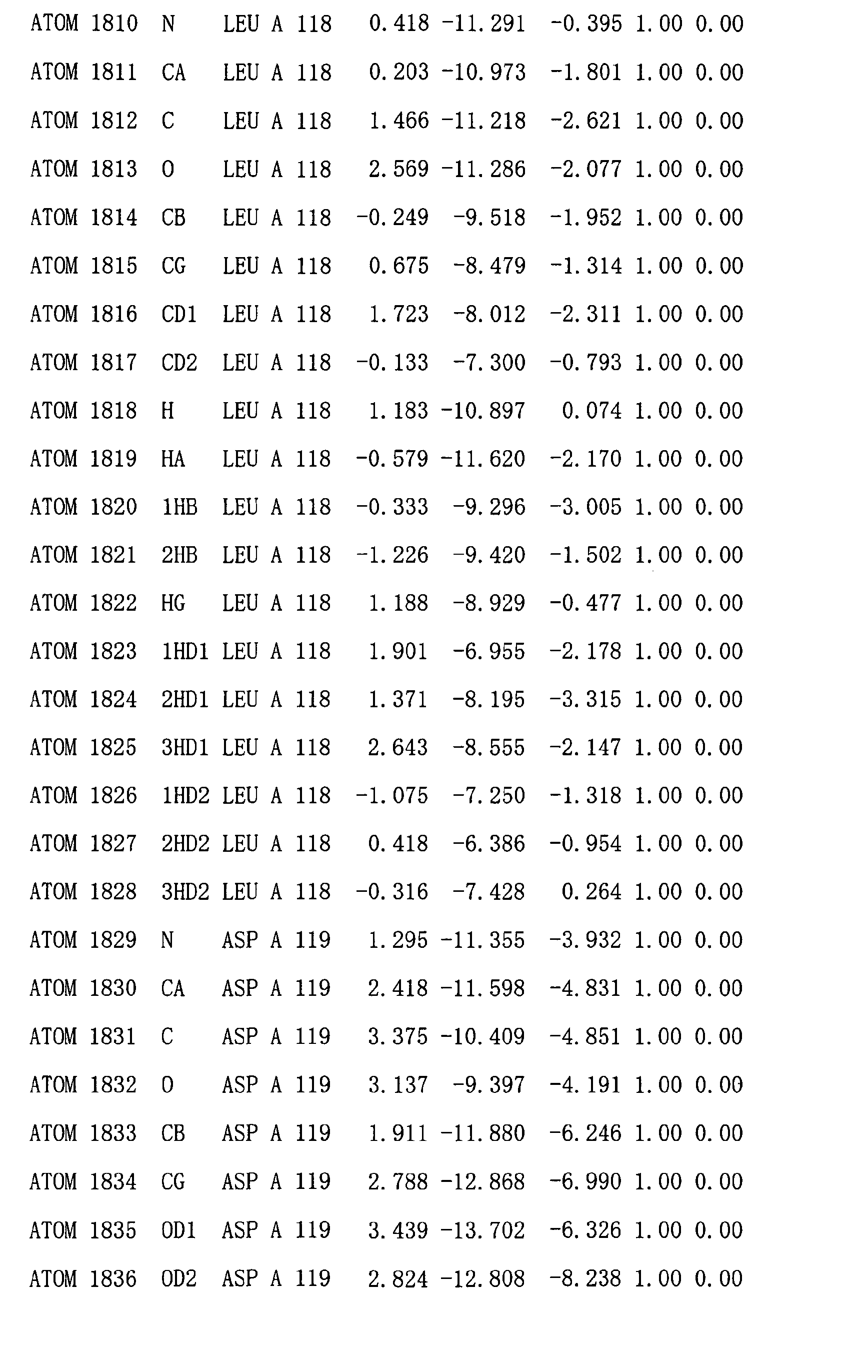

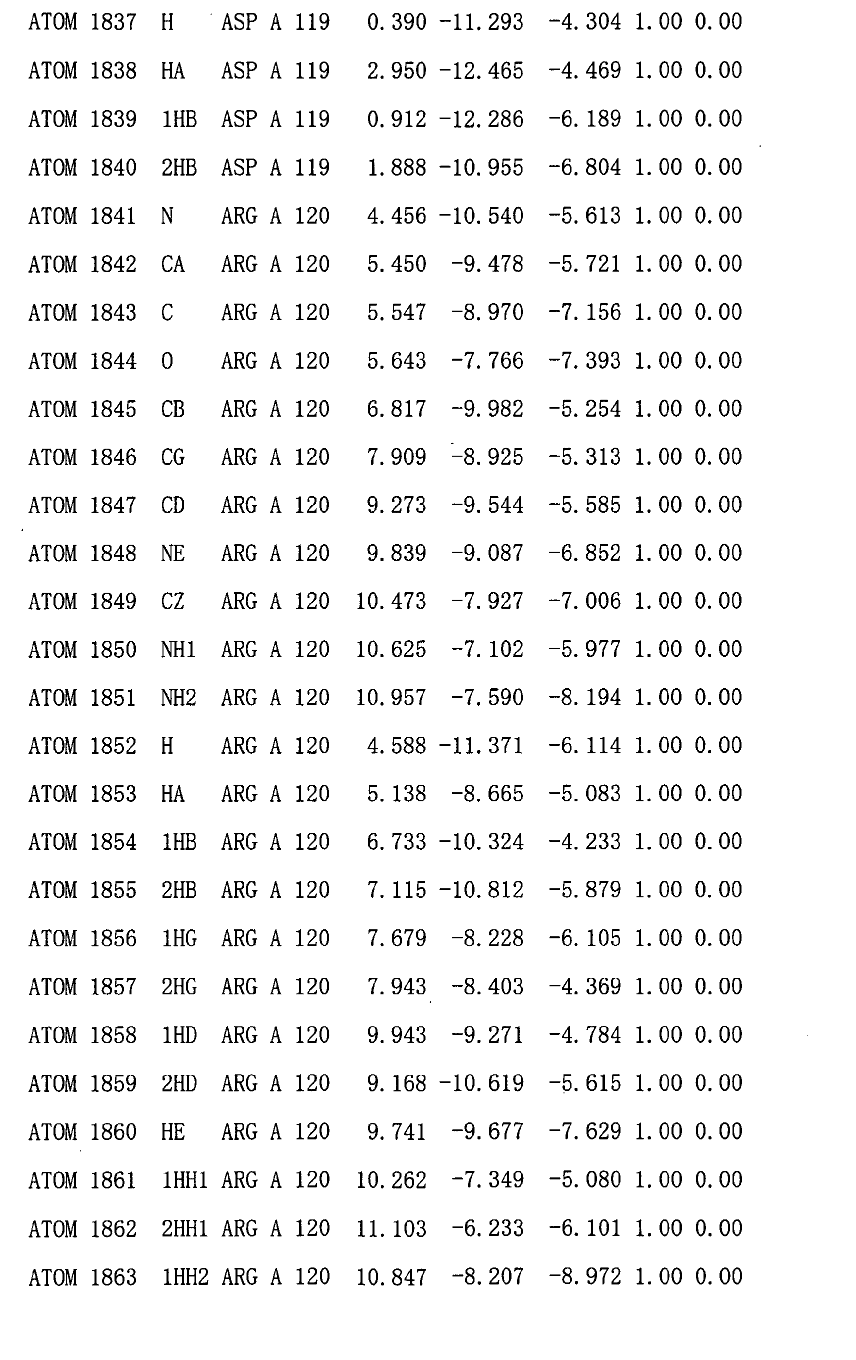

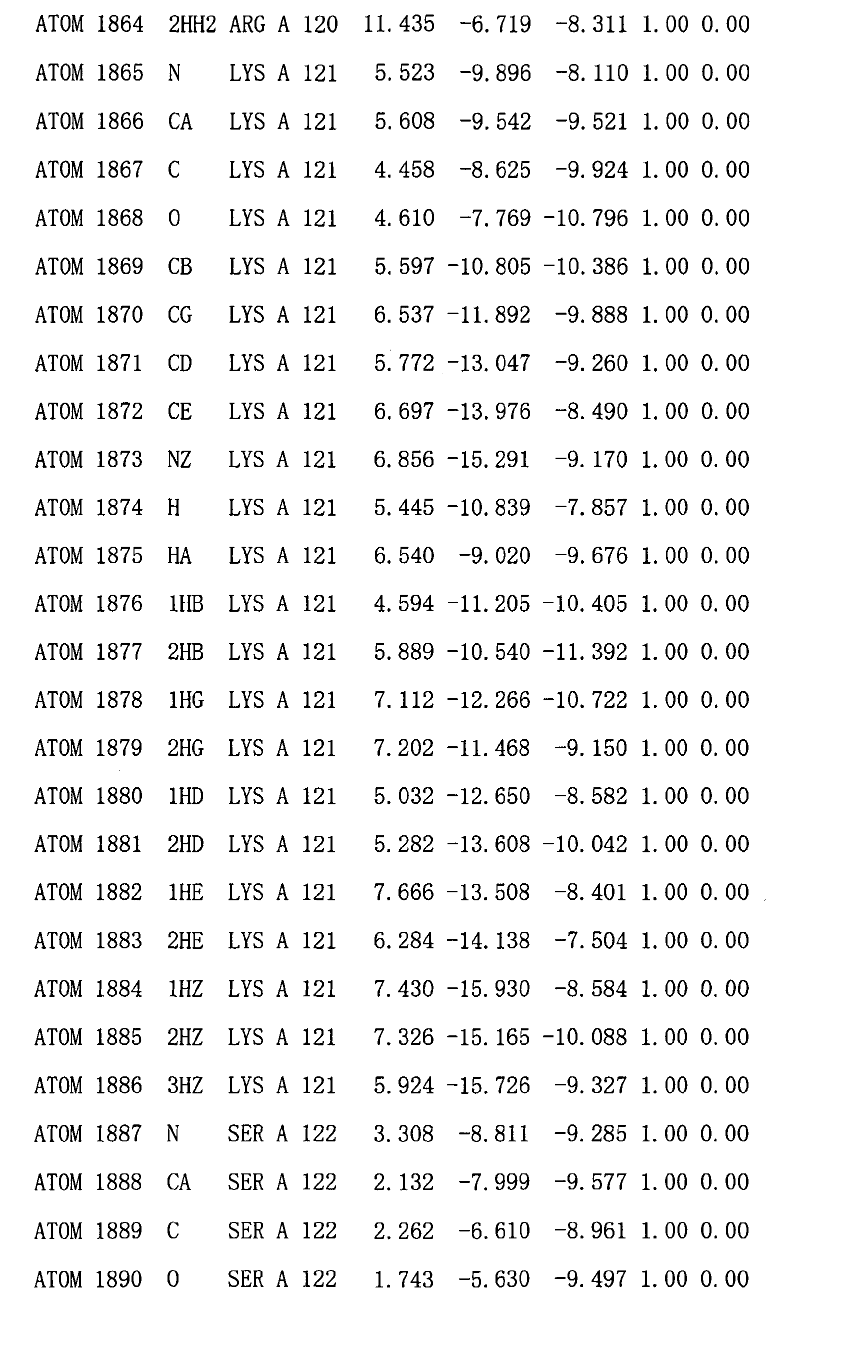

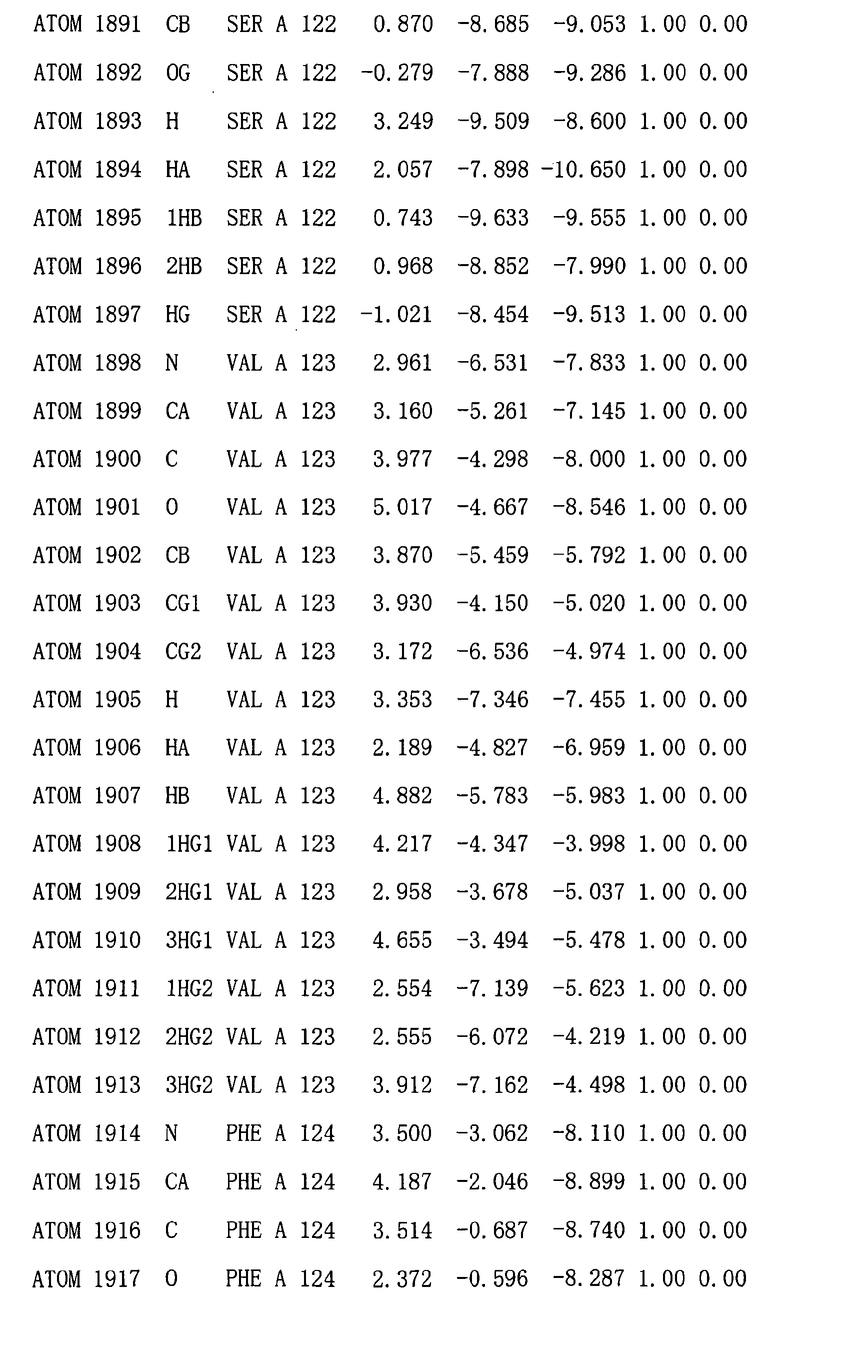

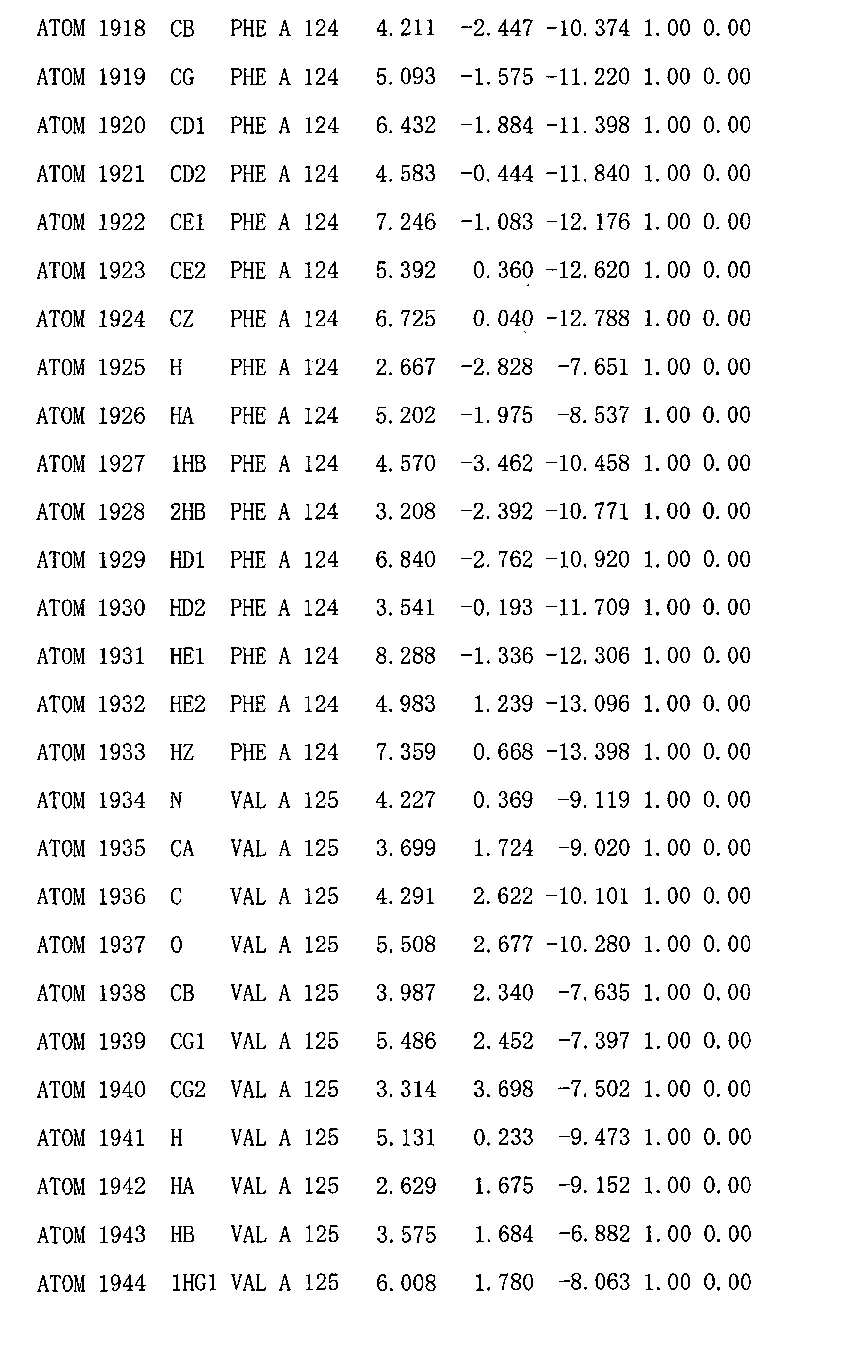

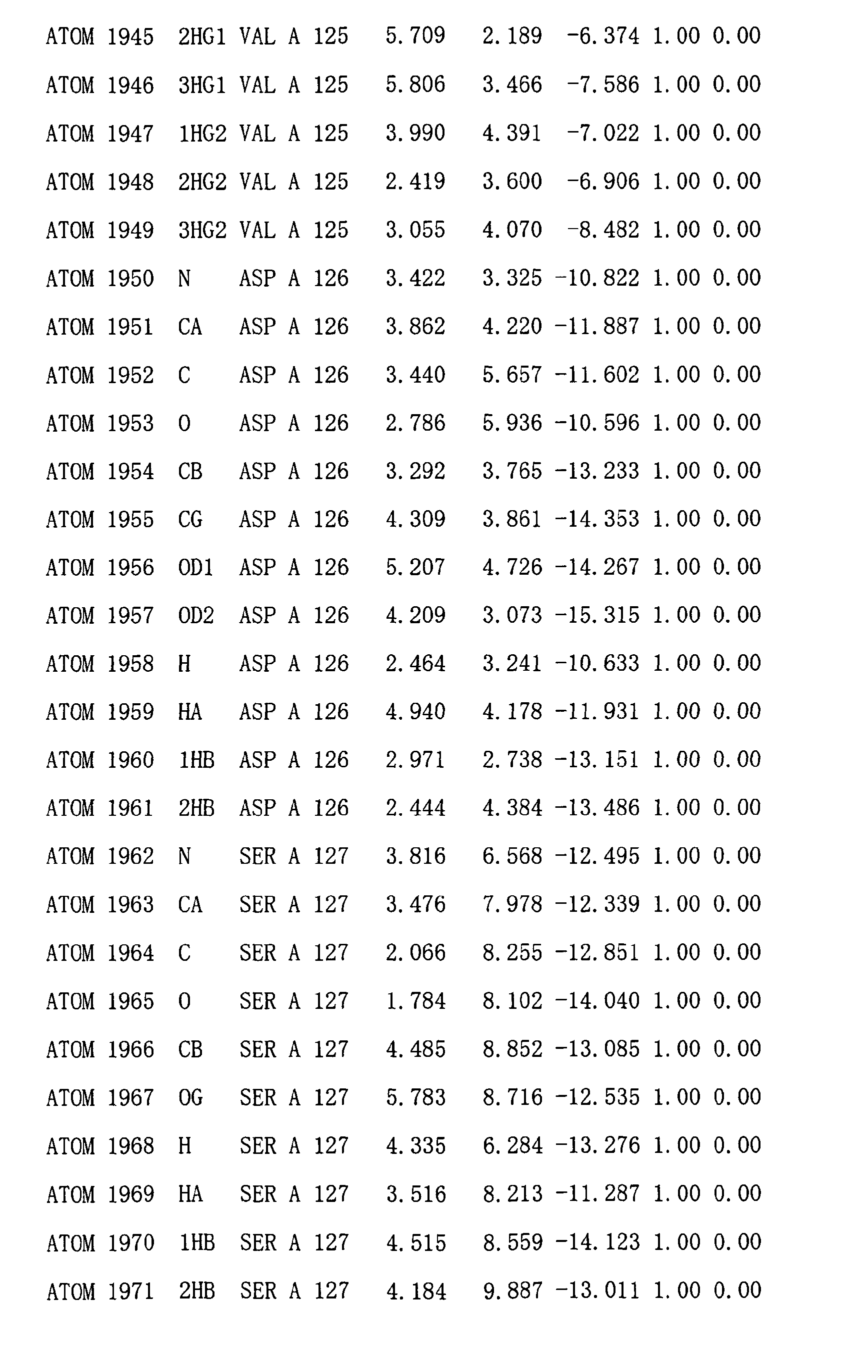

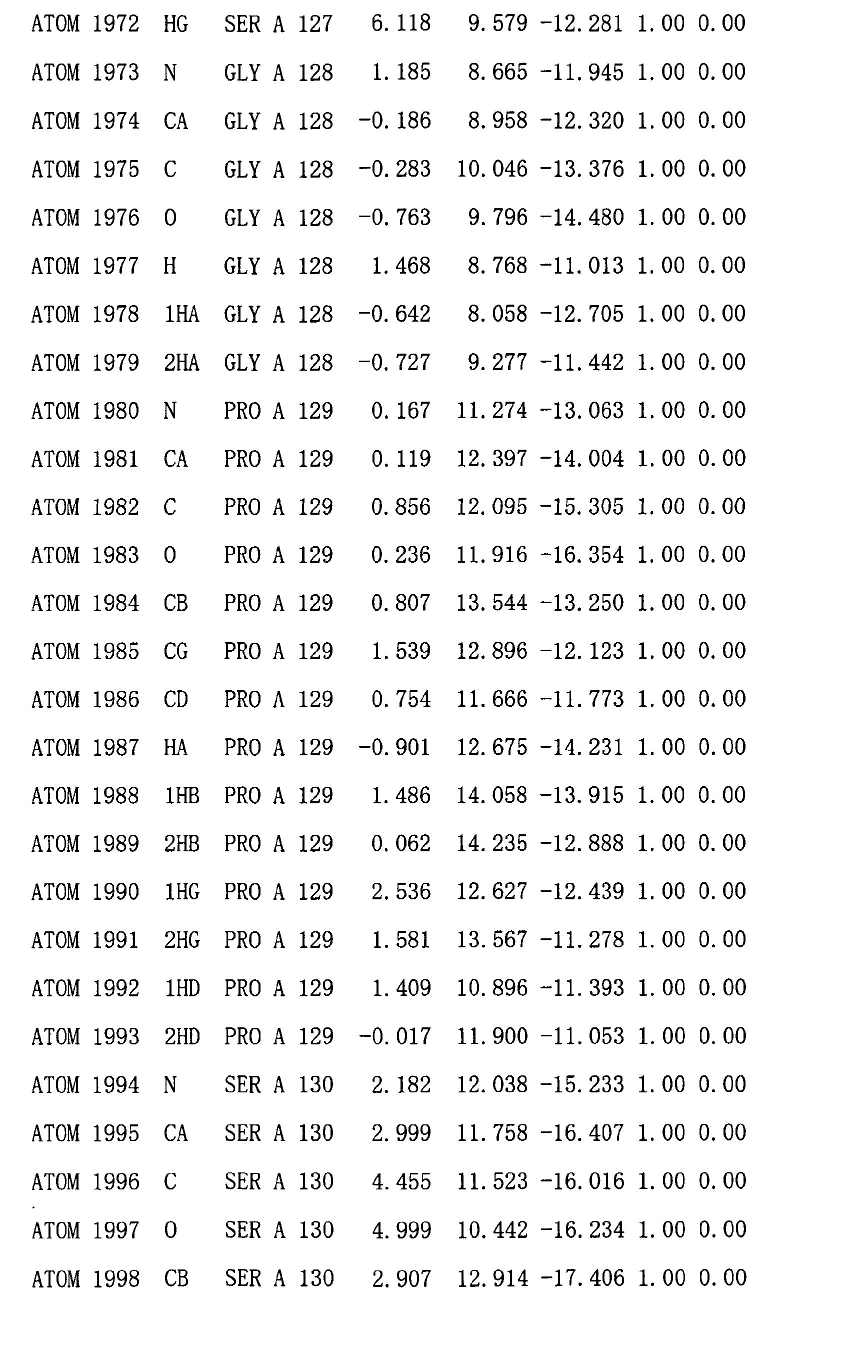

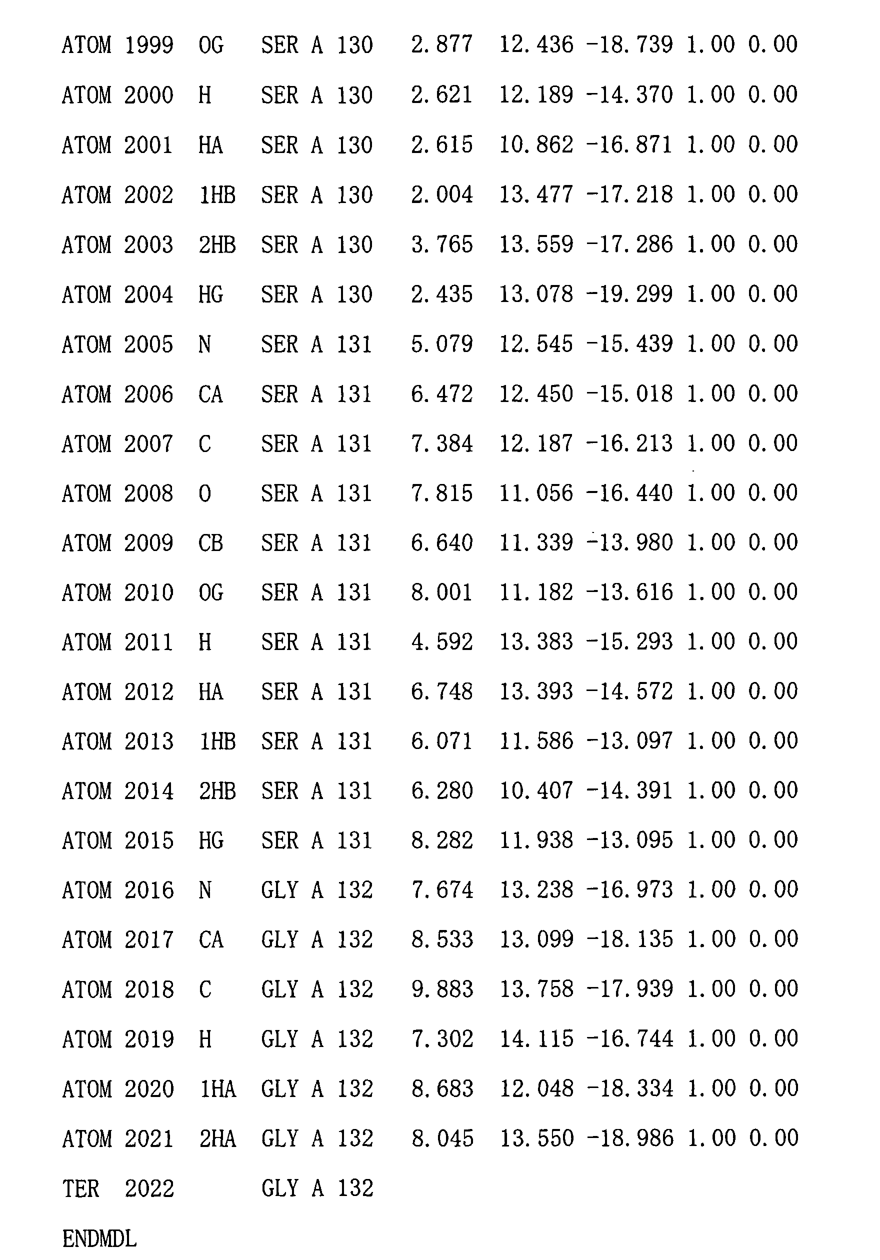

### Three-Dimensional Structure Coordinate 11

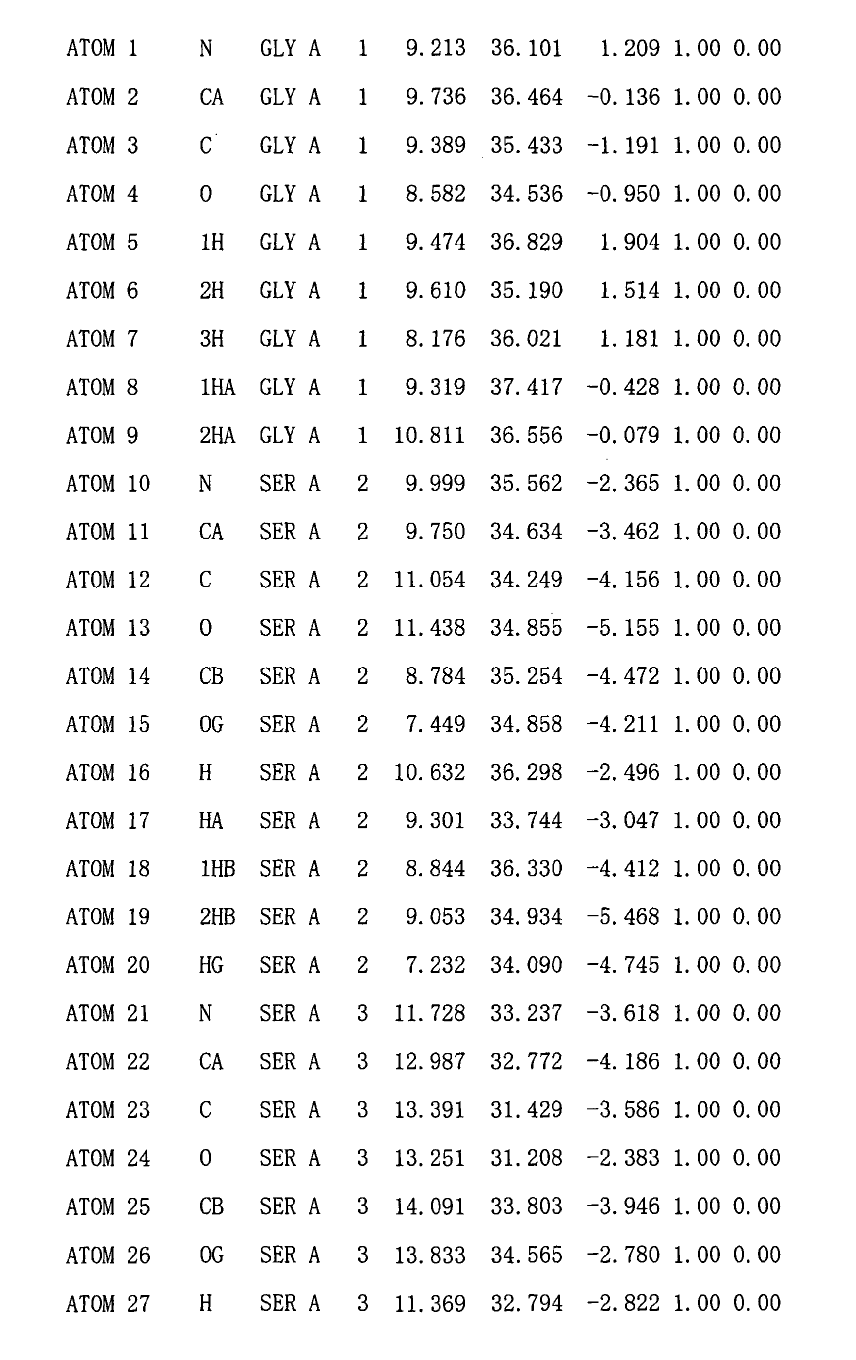

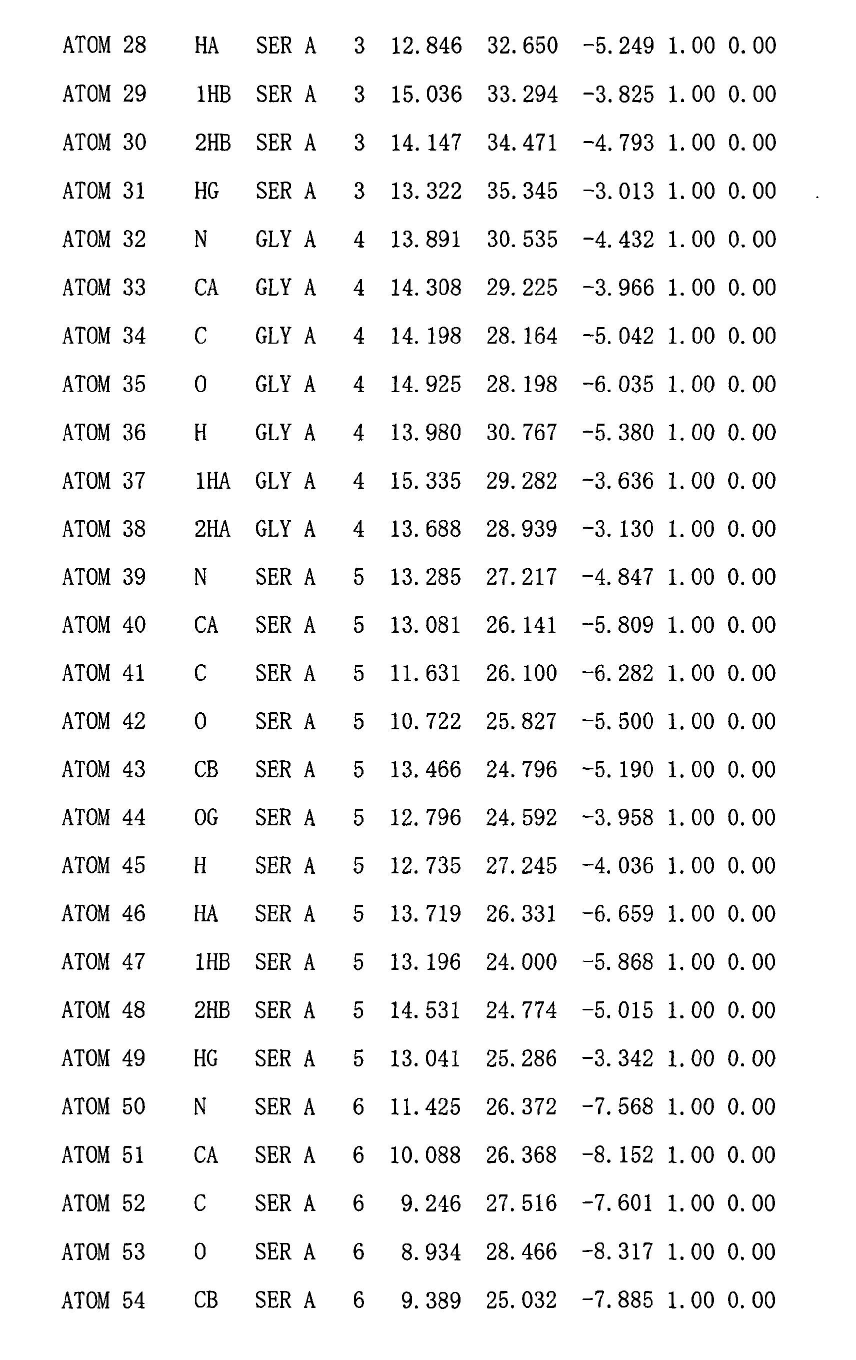

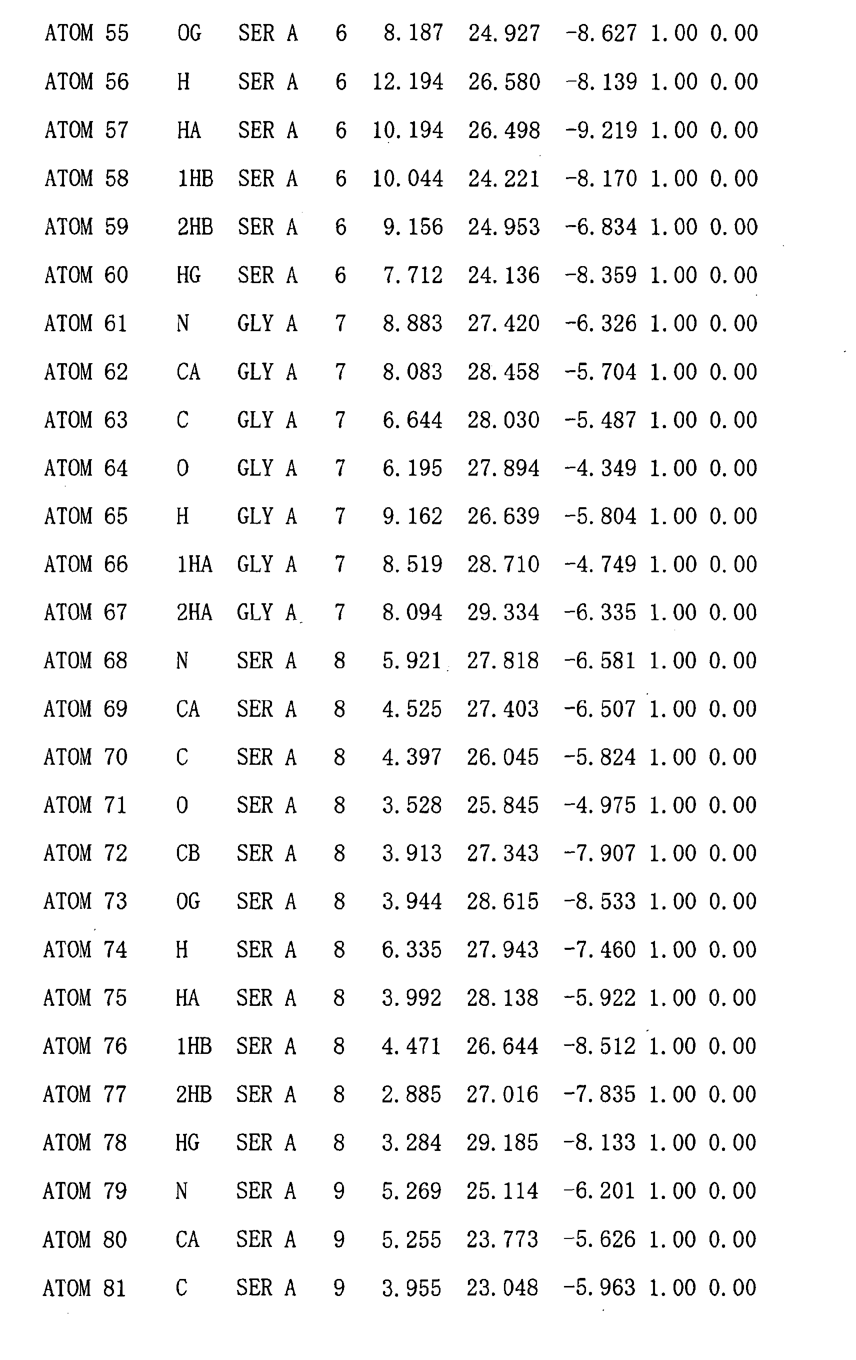

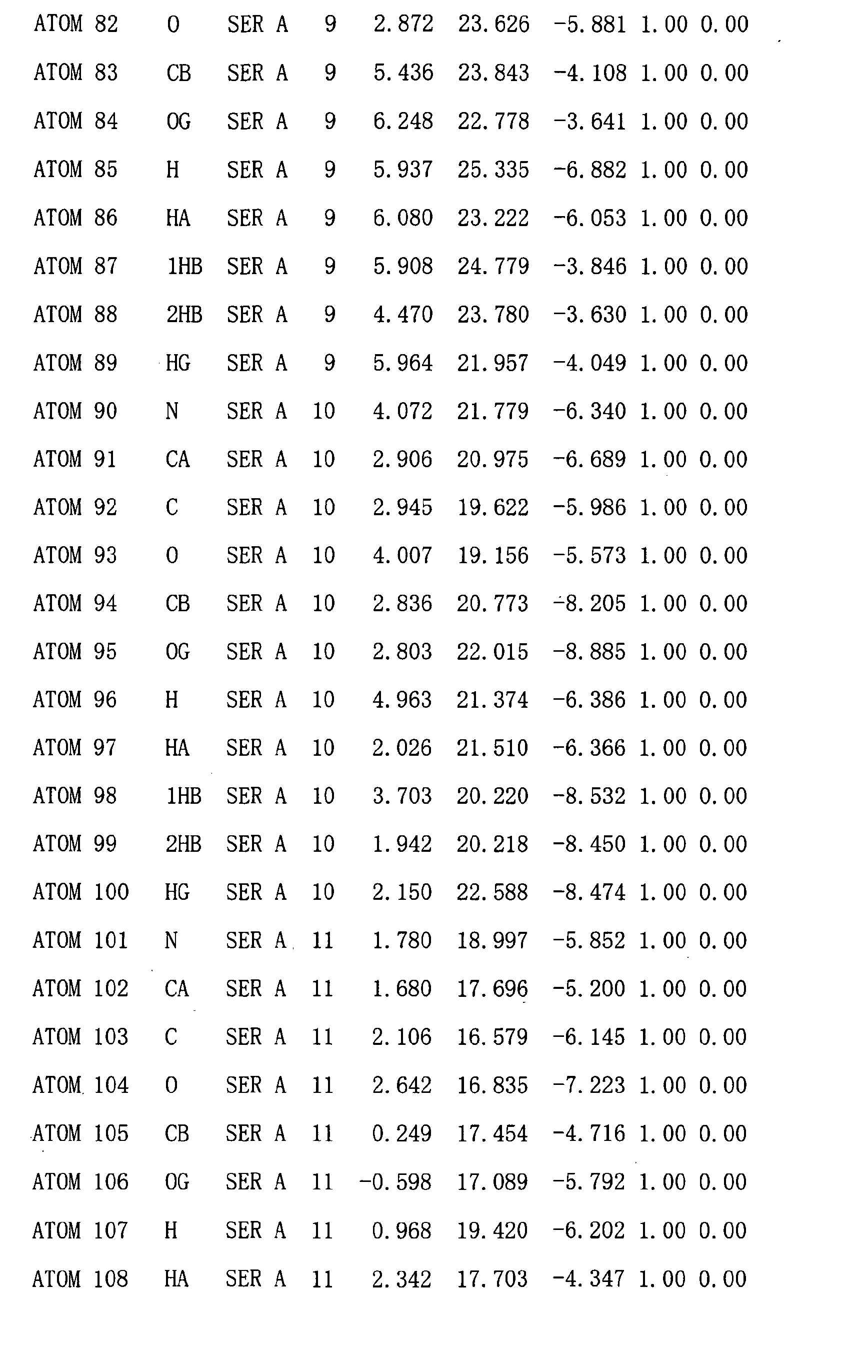

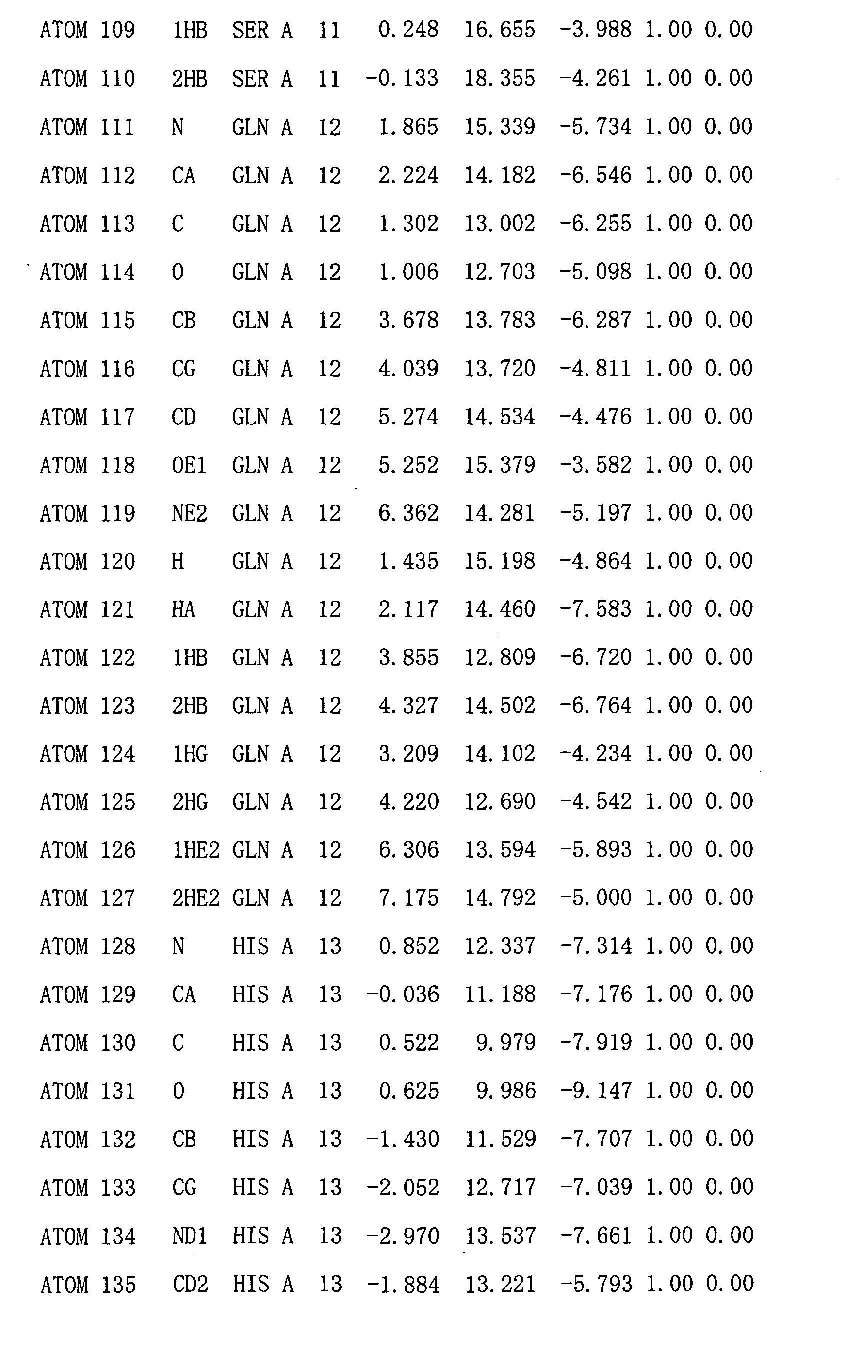

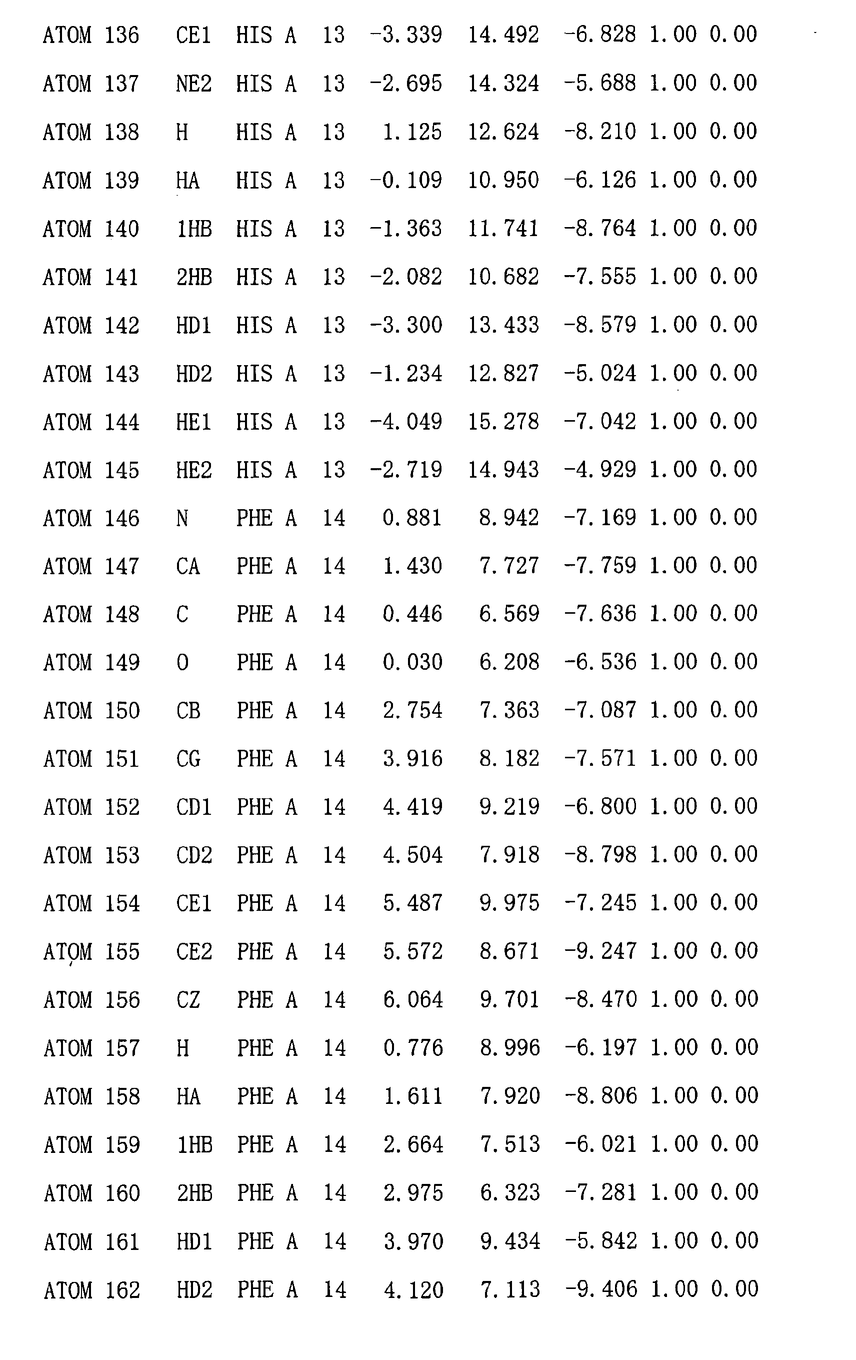

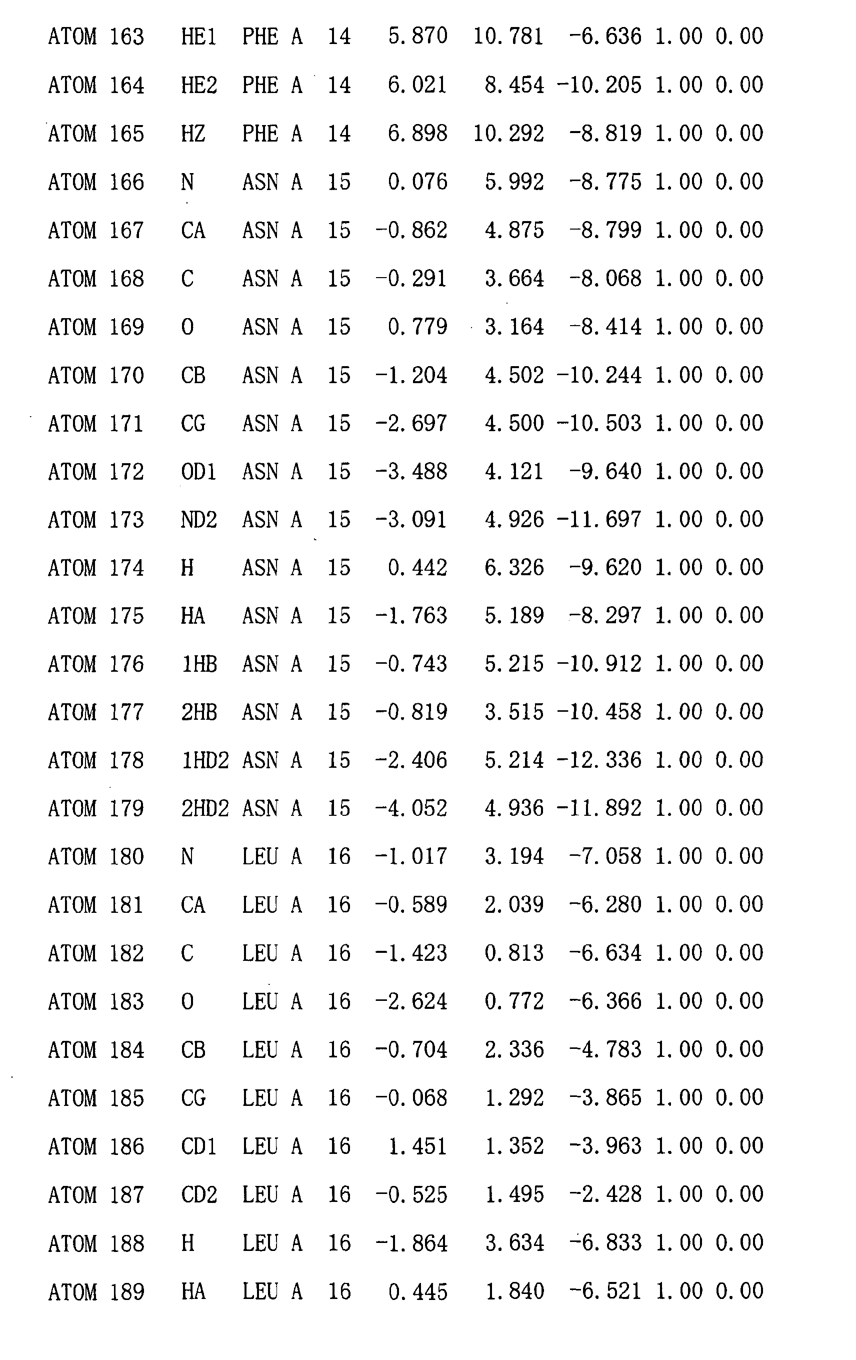

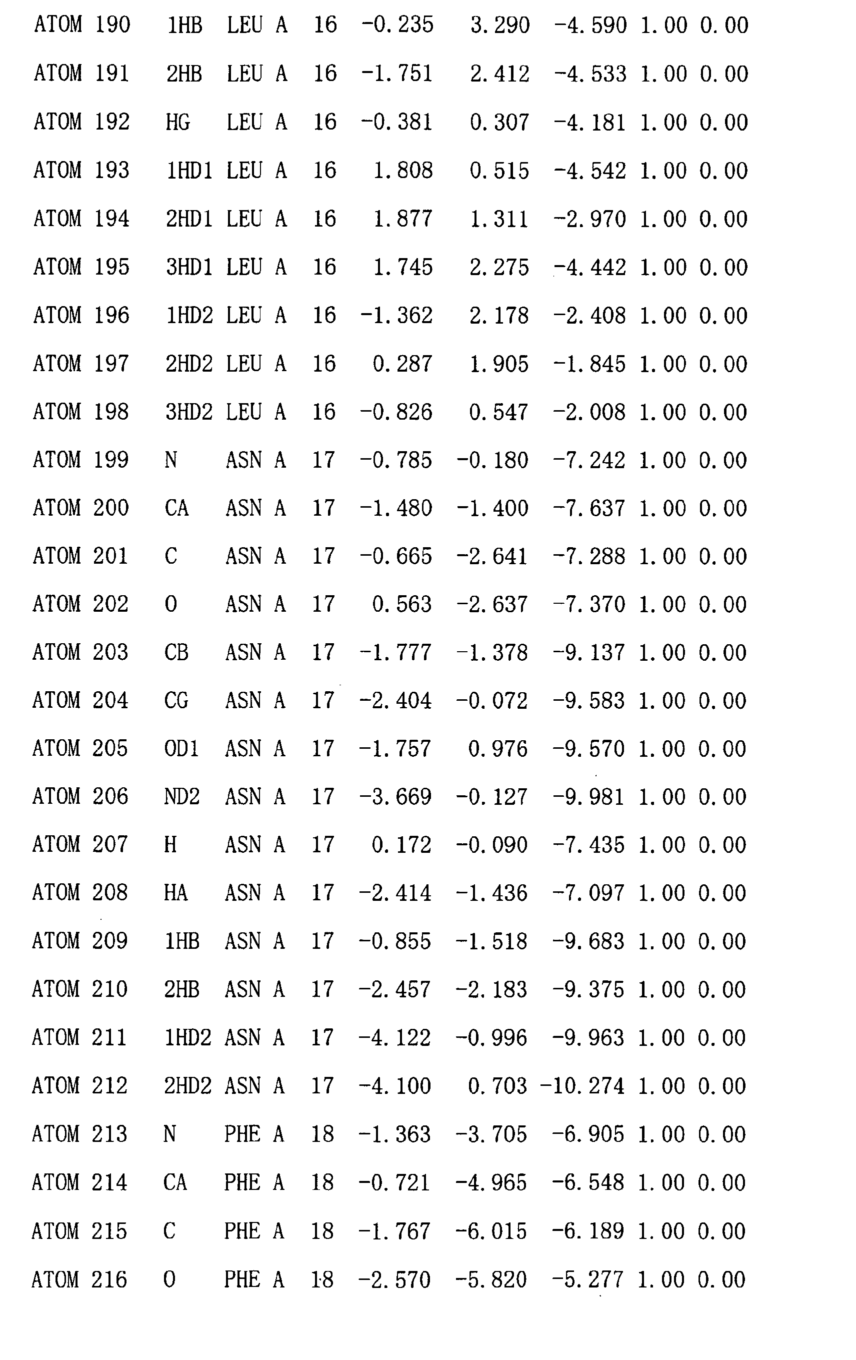

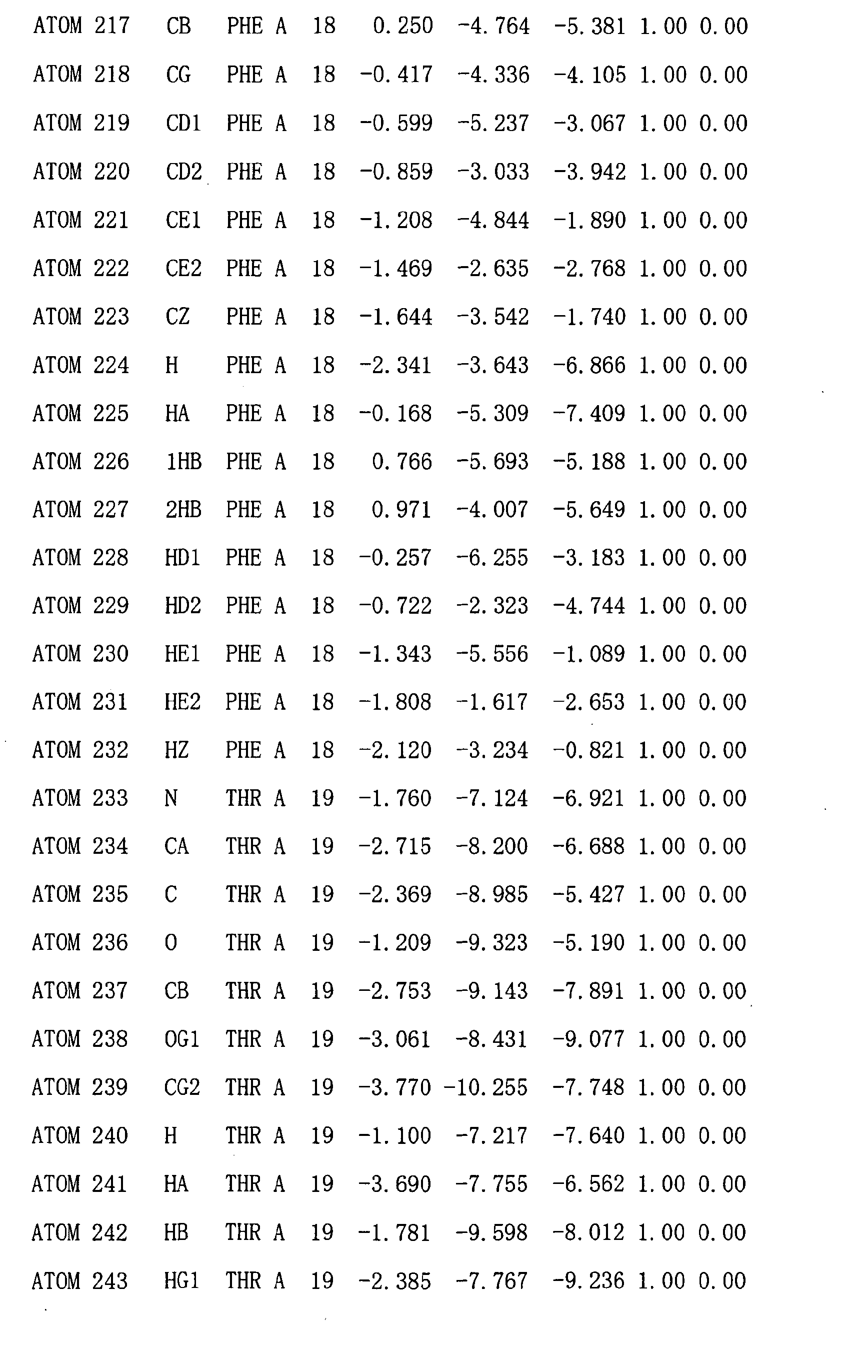

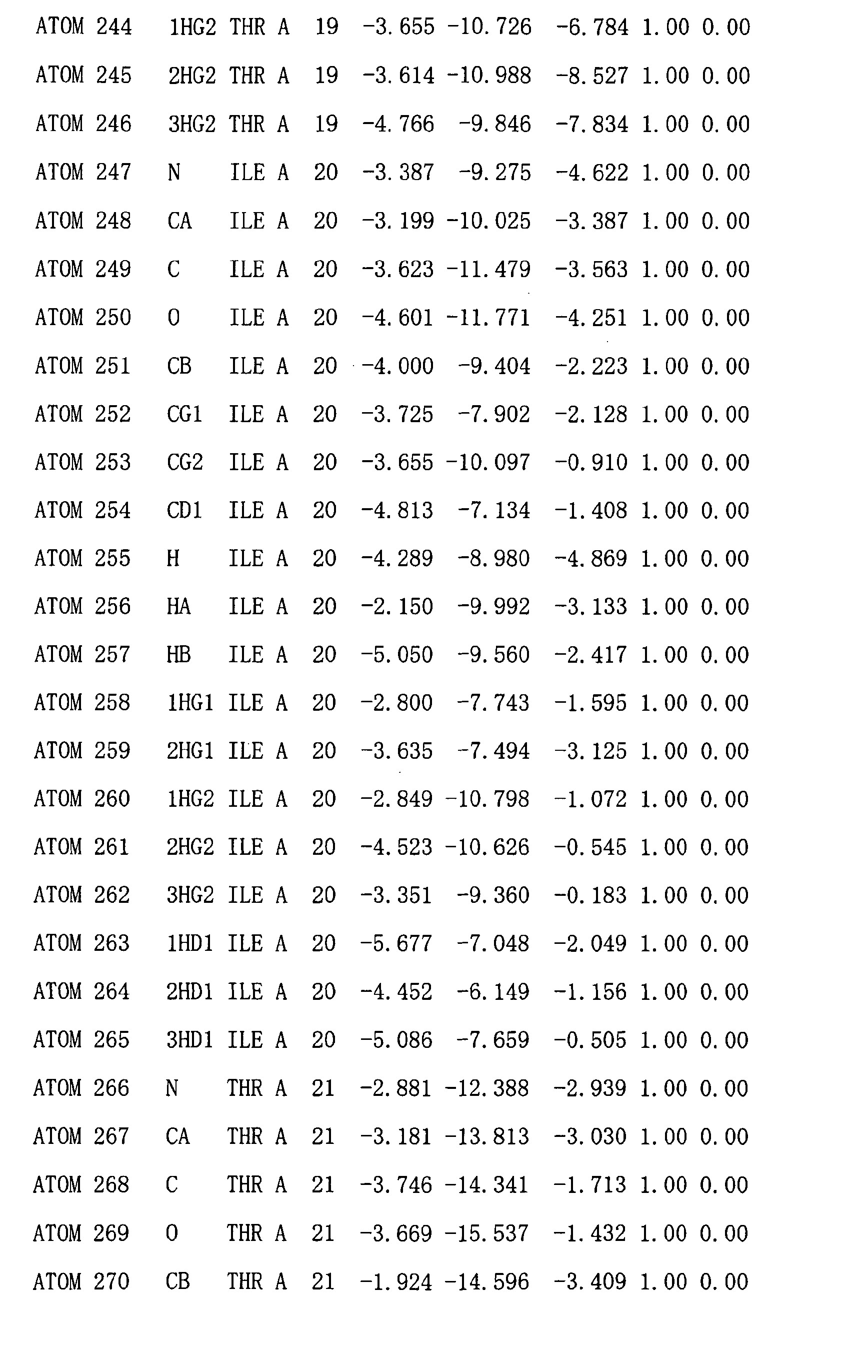

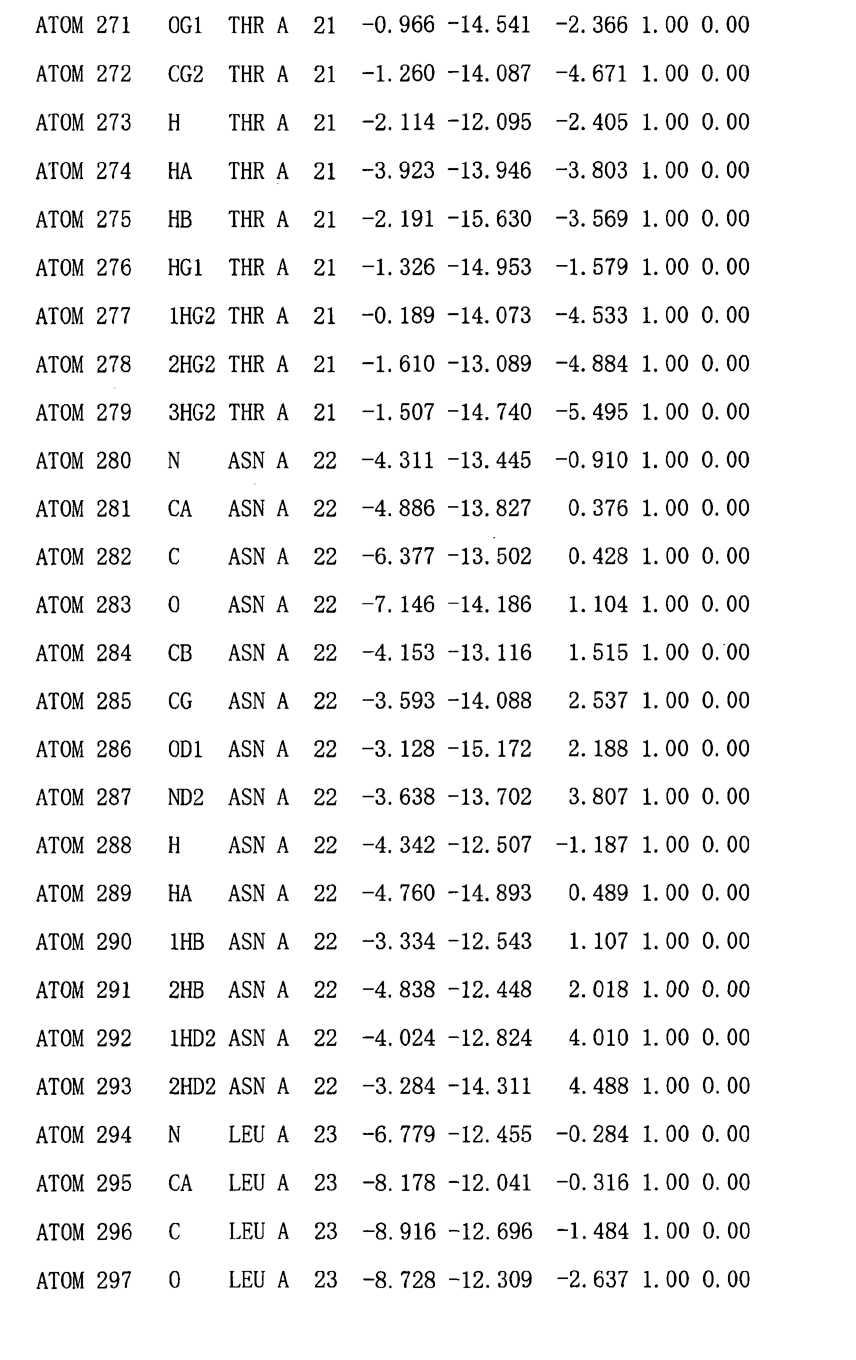

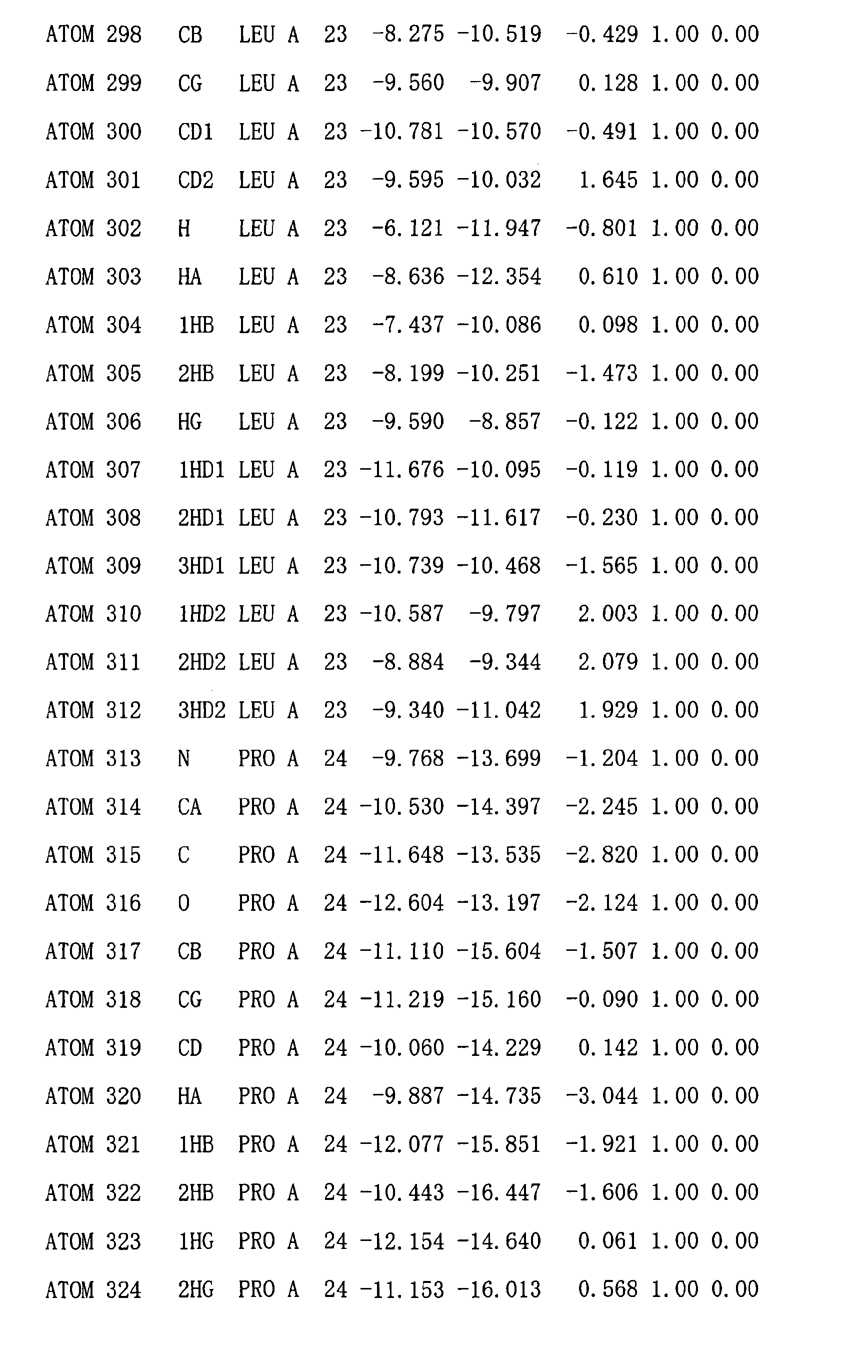

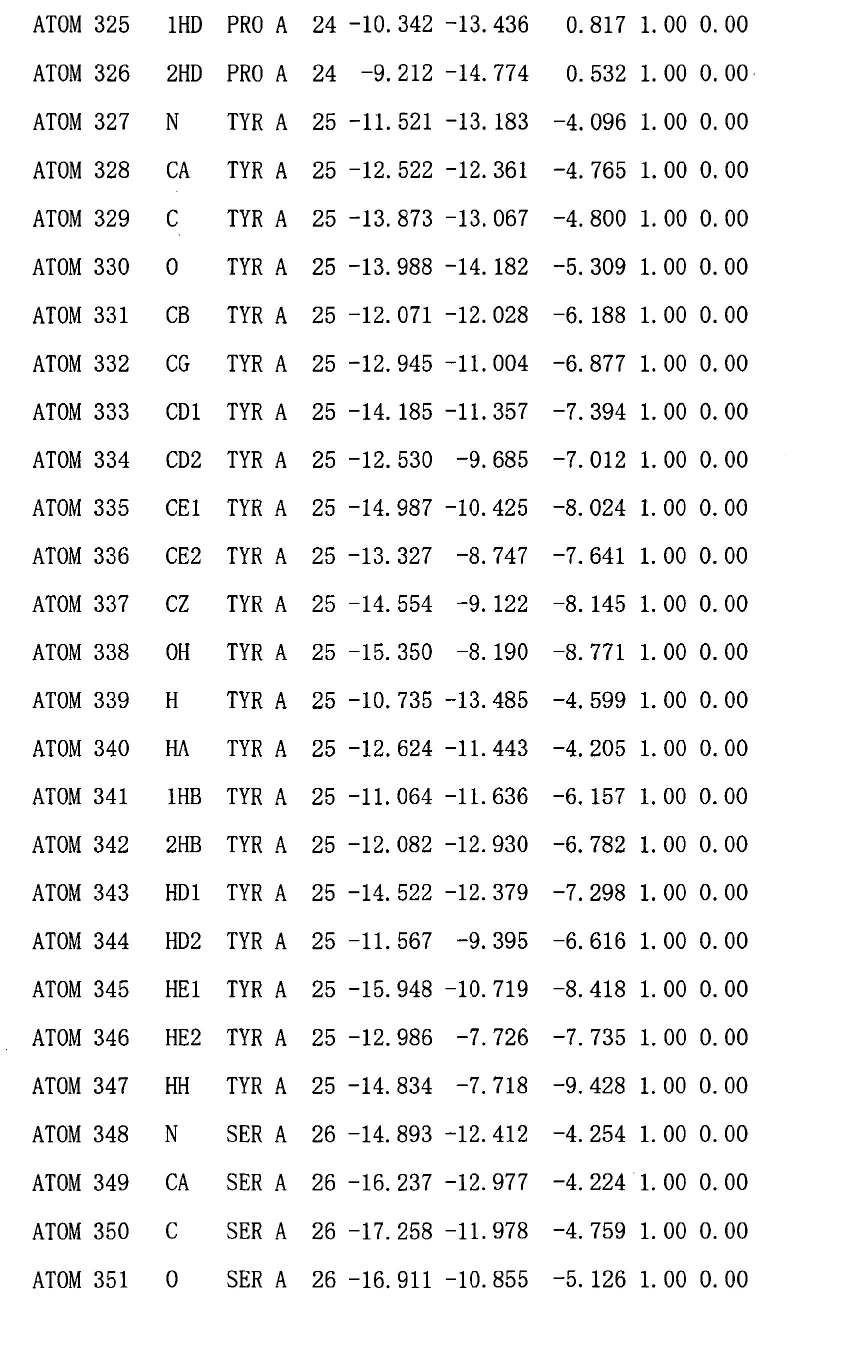

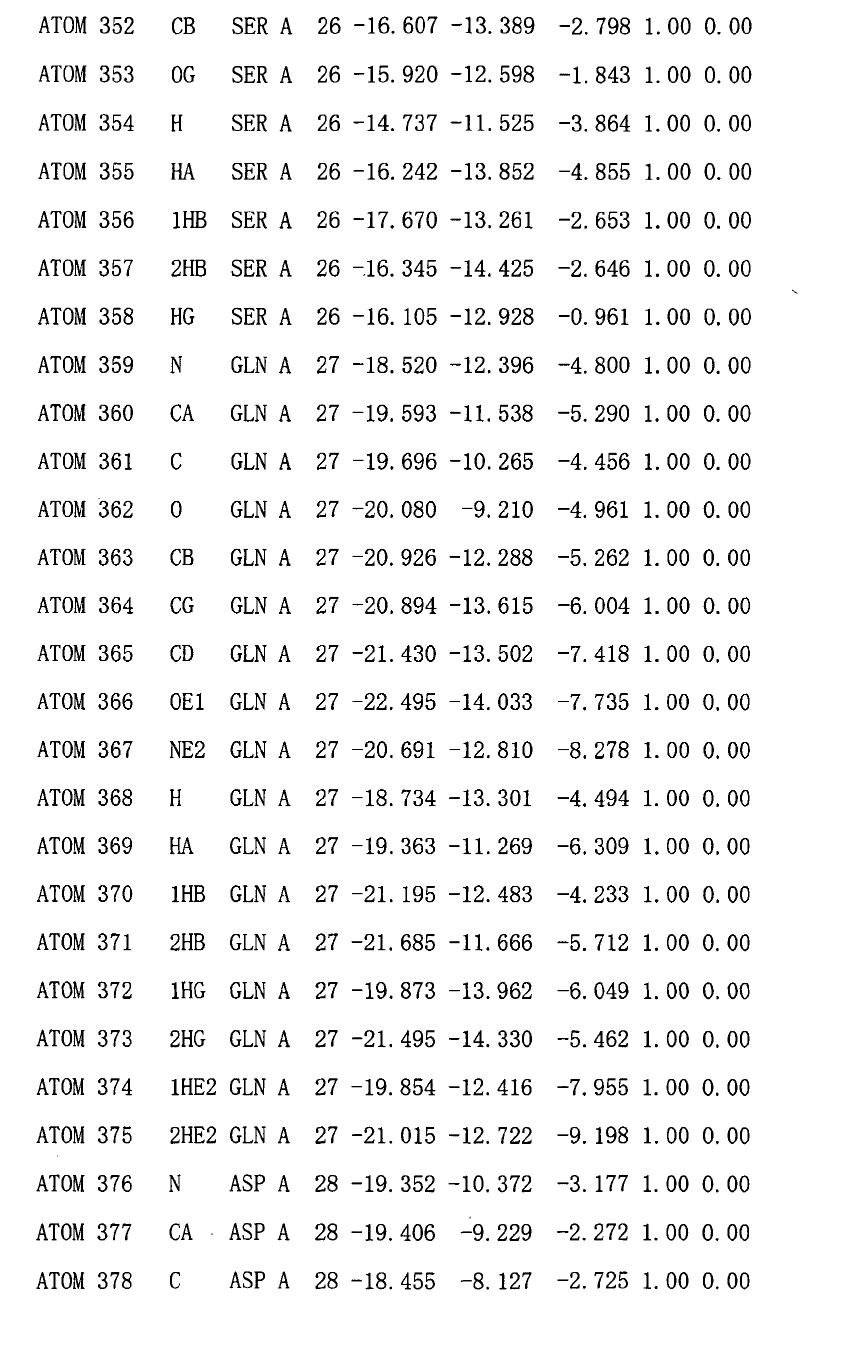

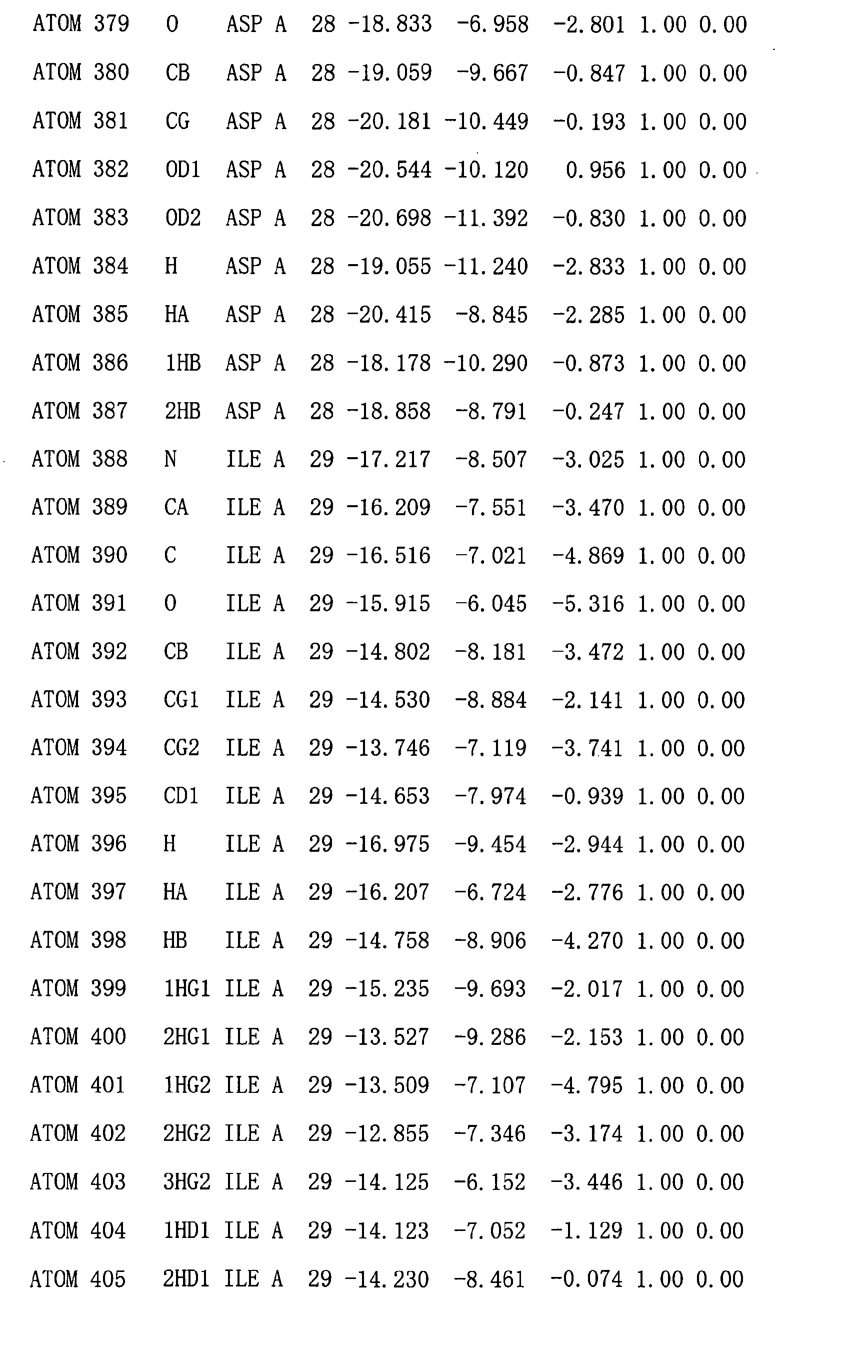

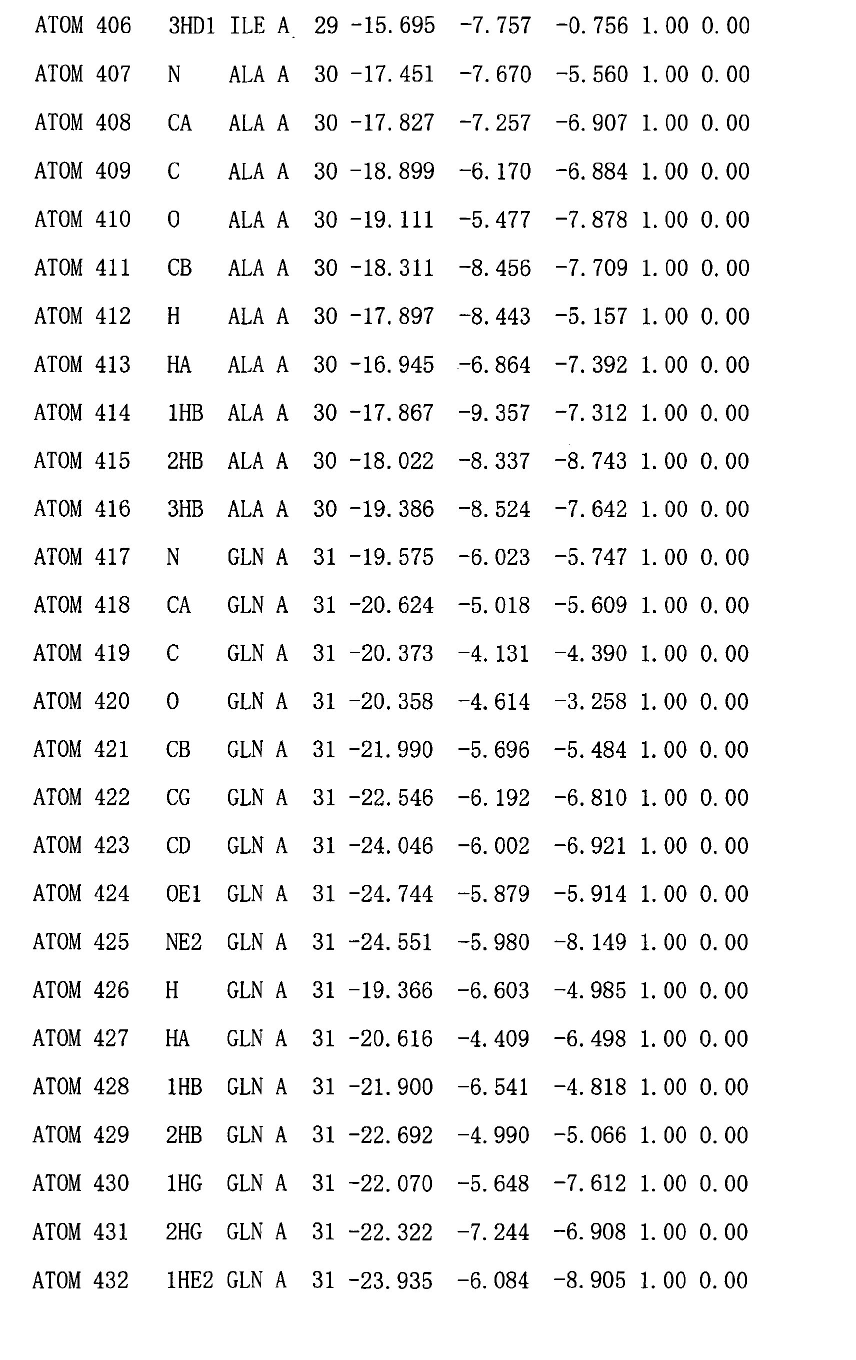

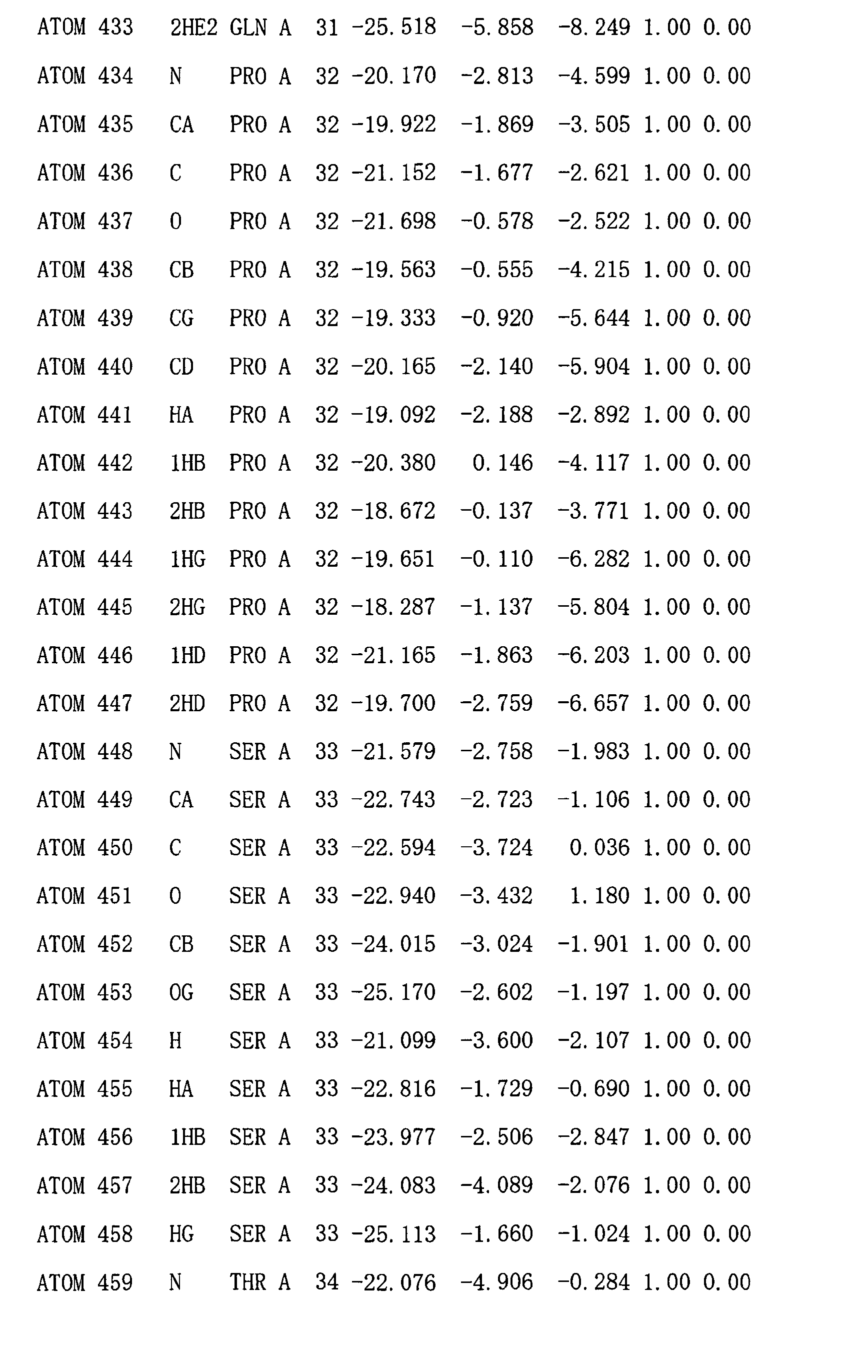

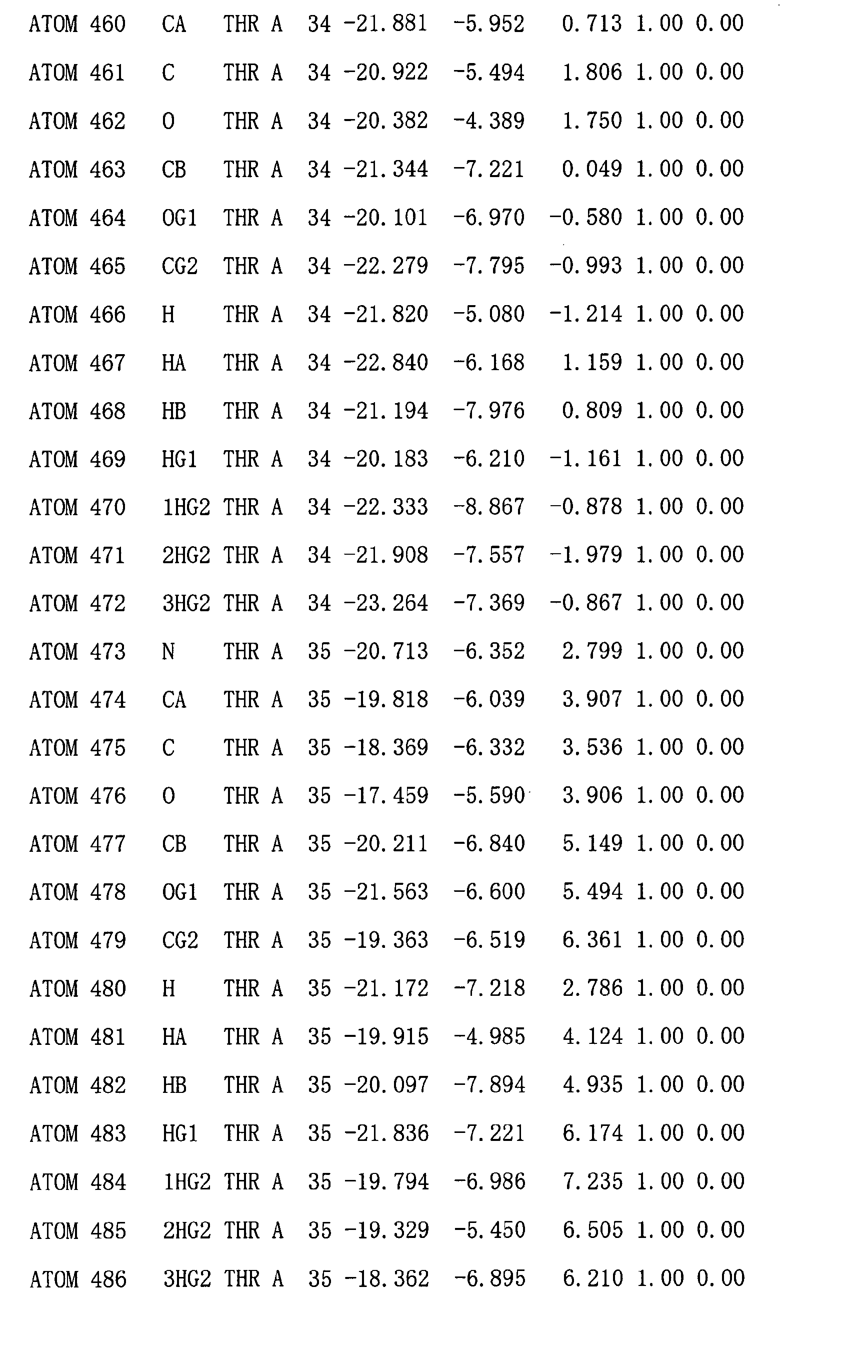

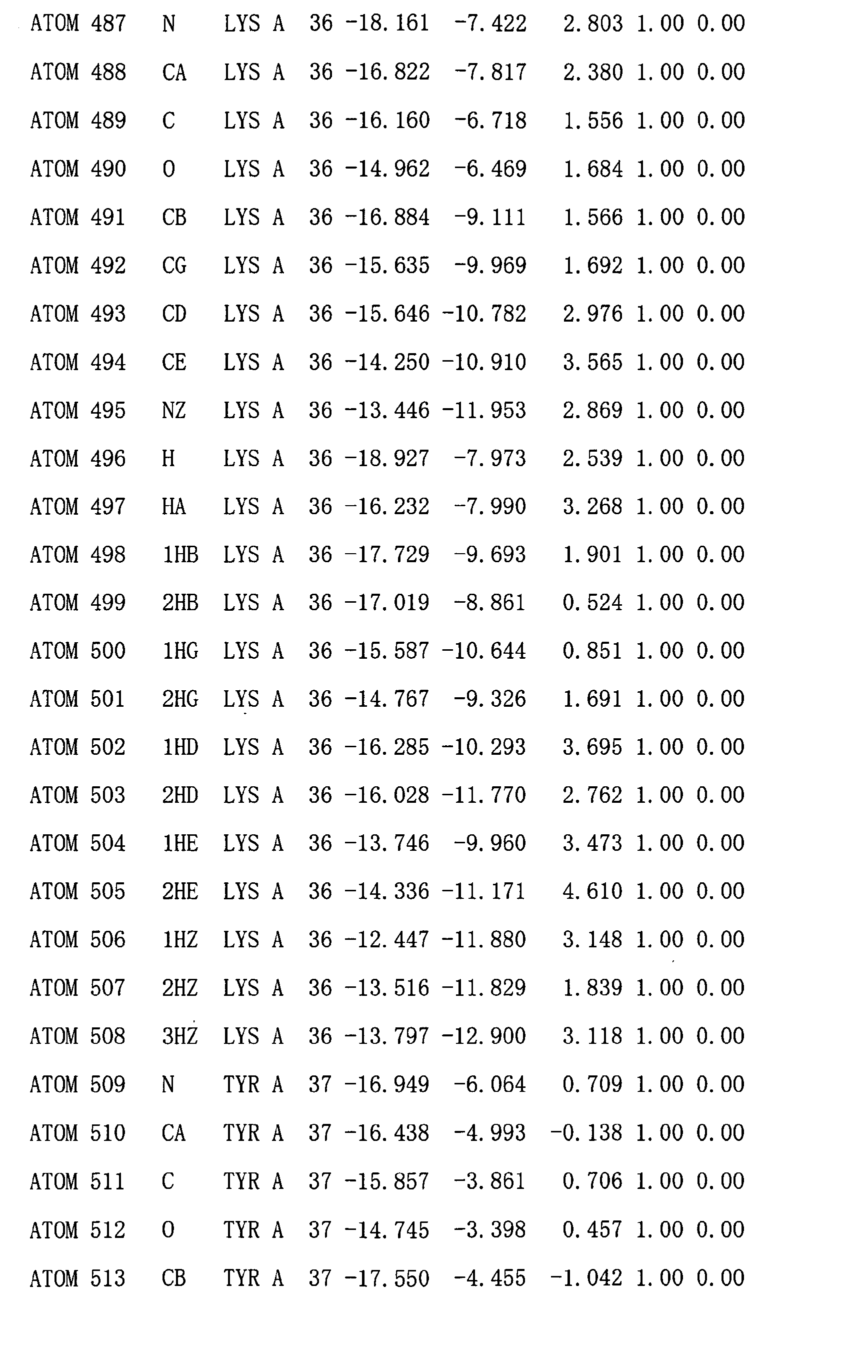

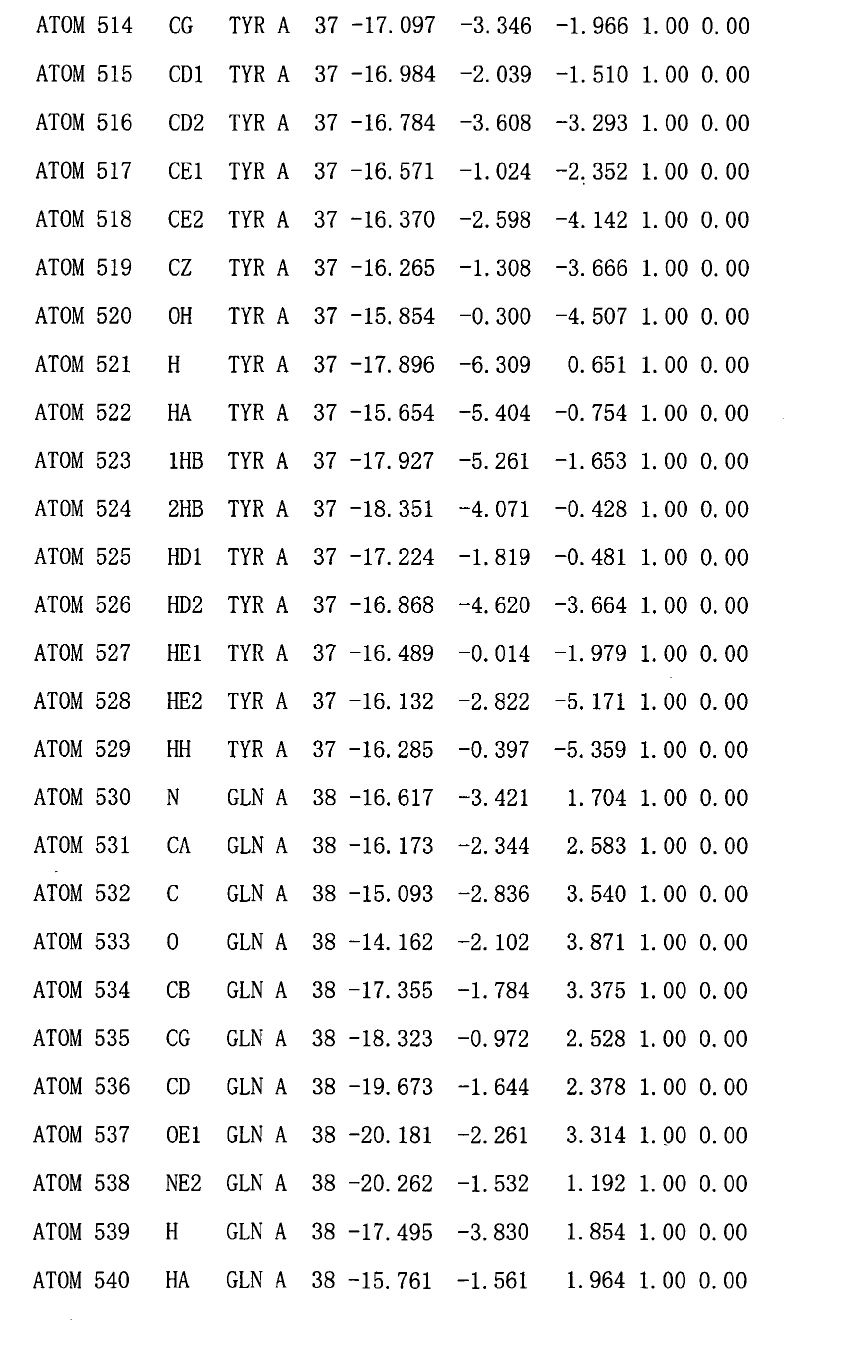

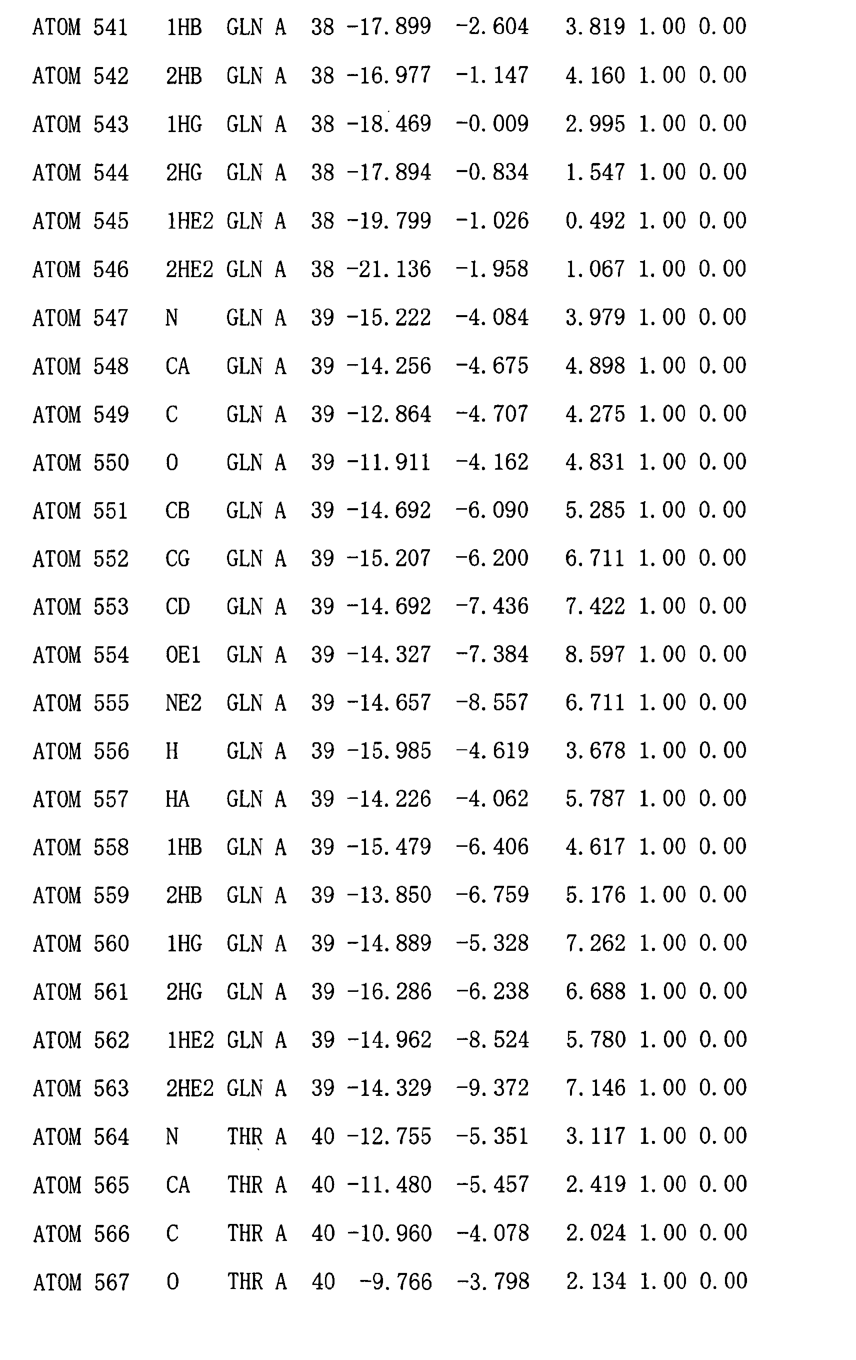

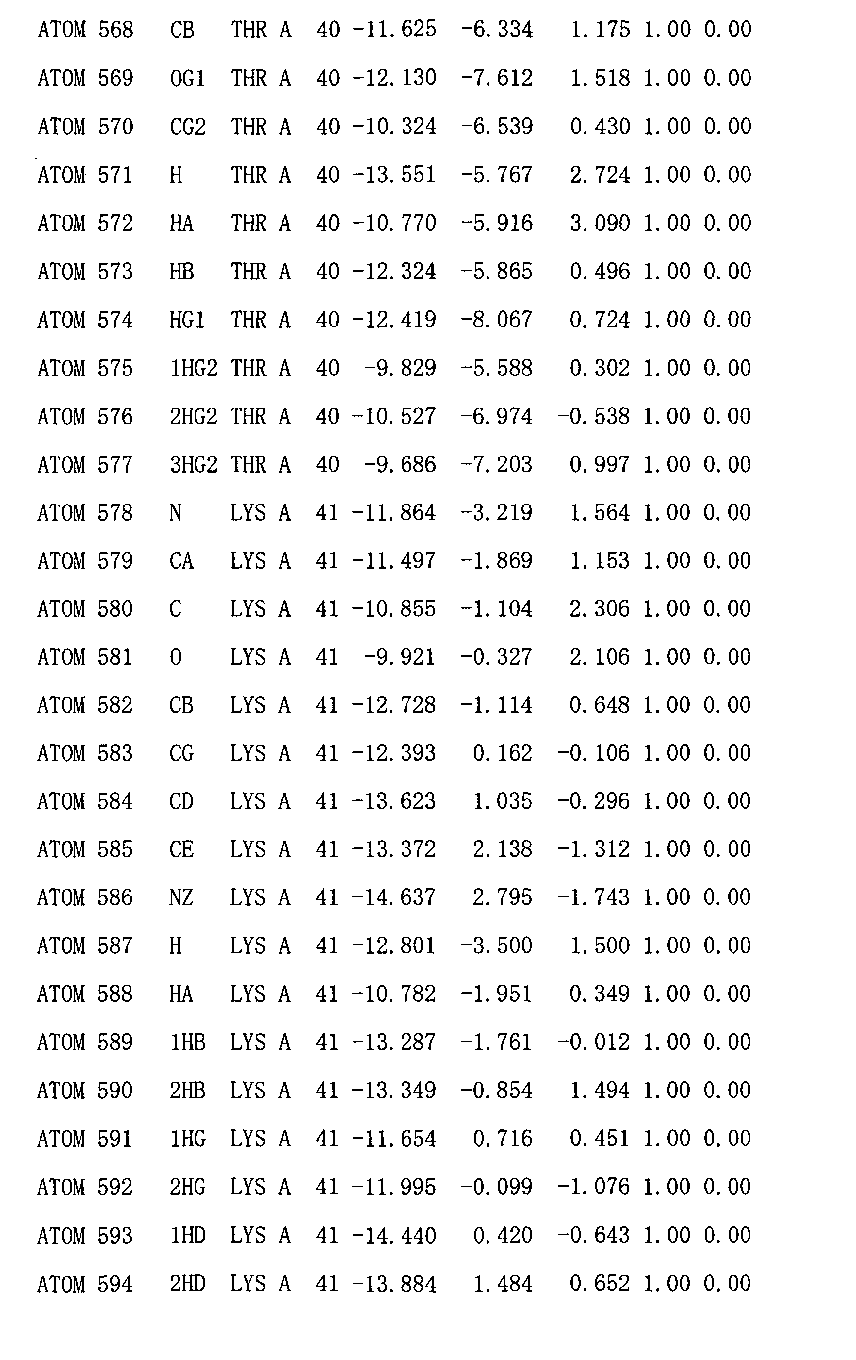

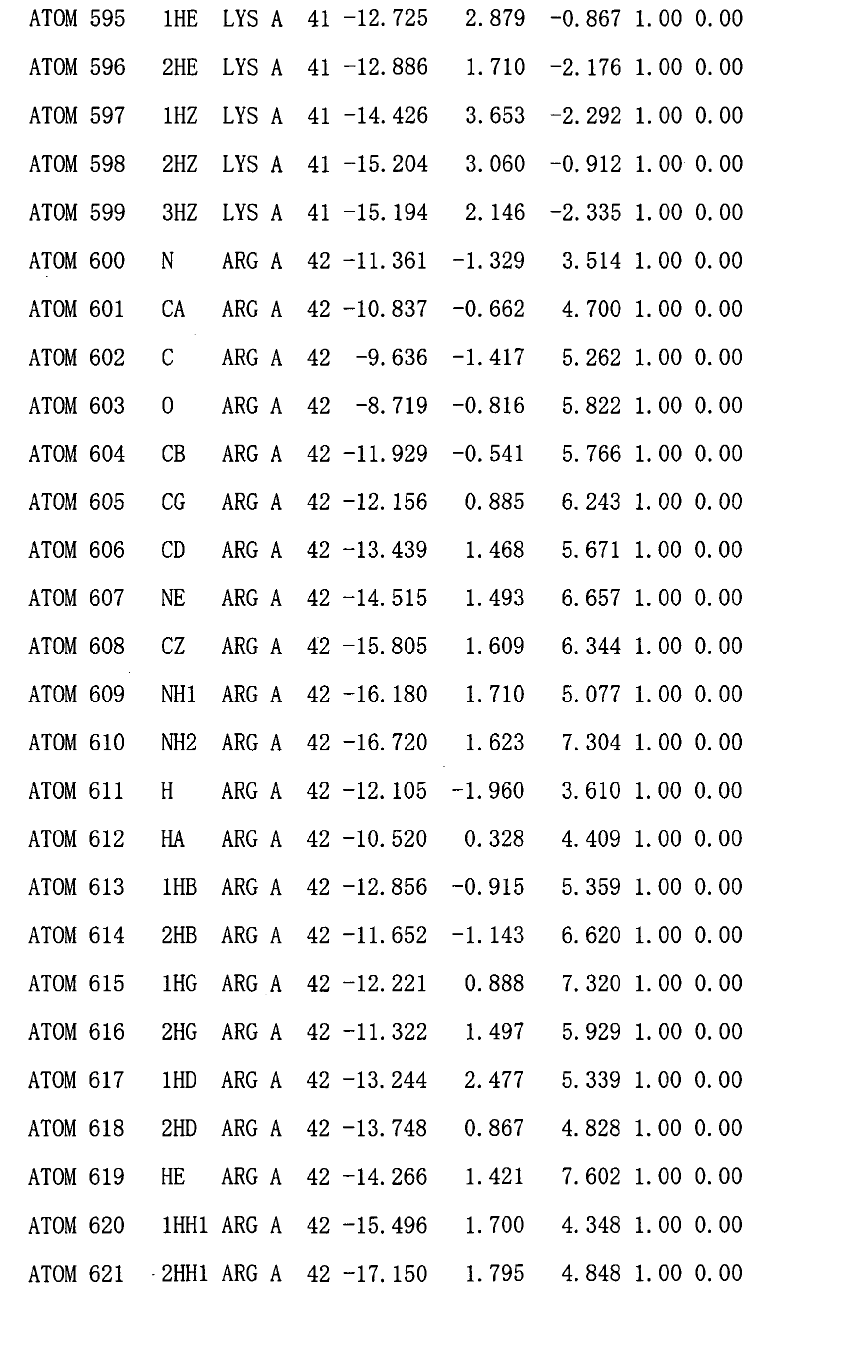

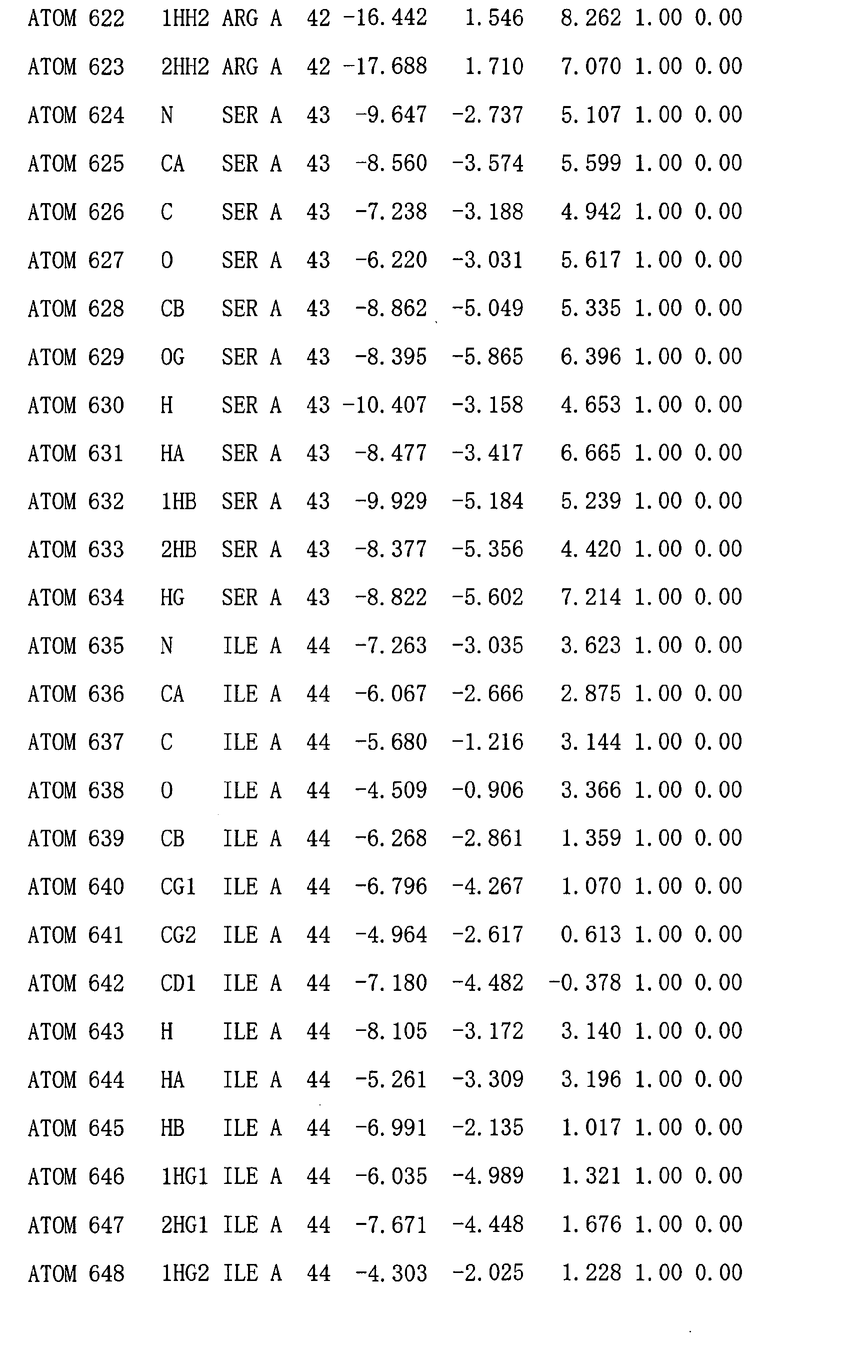

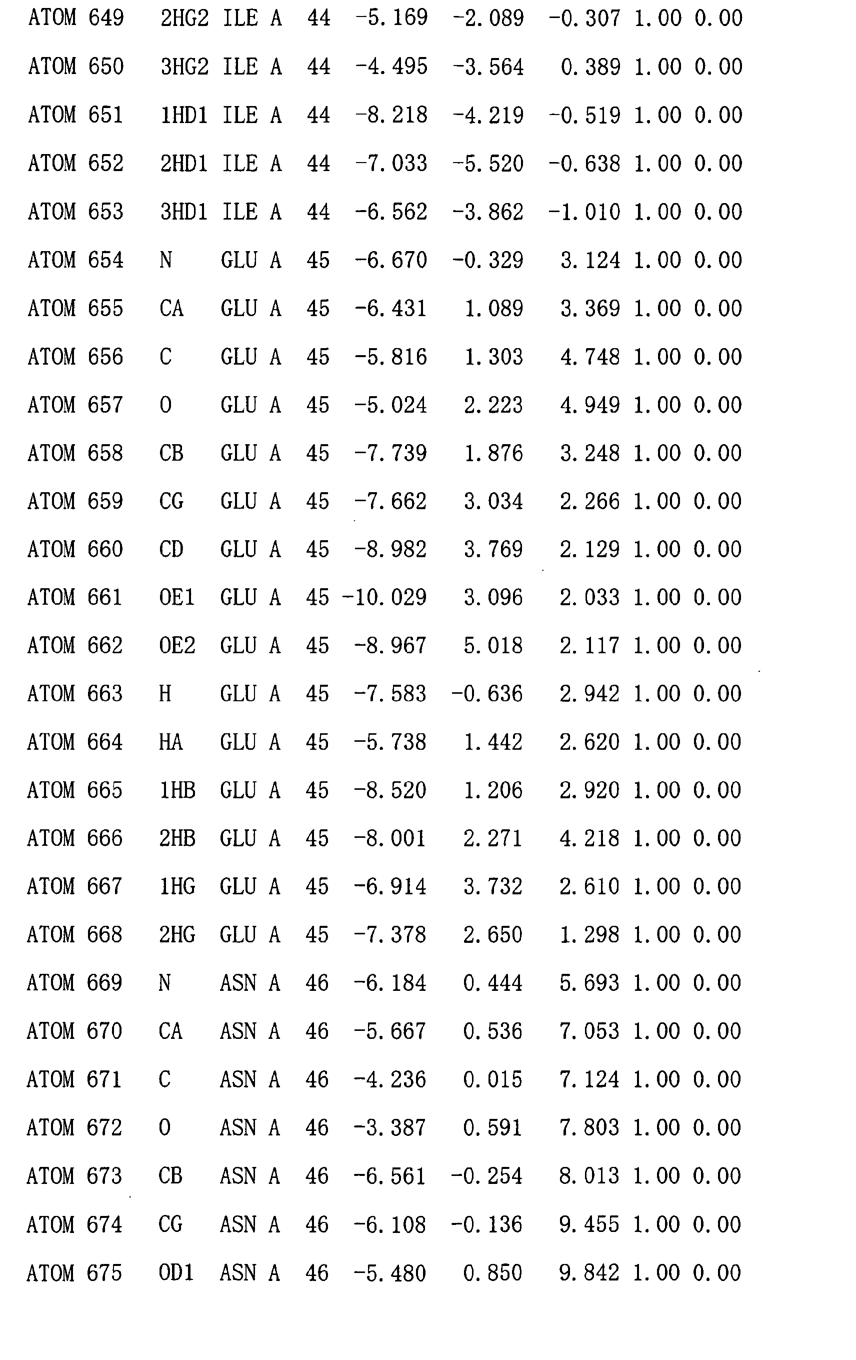

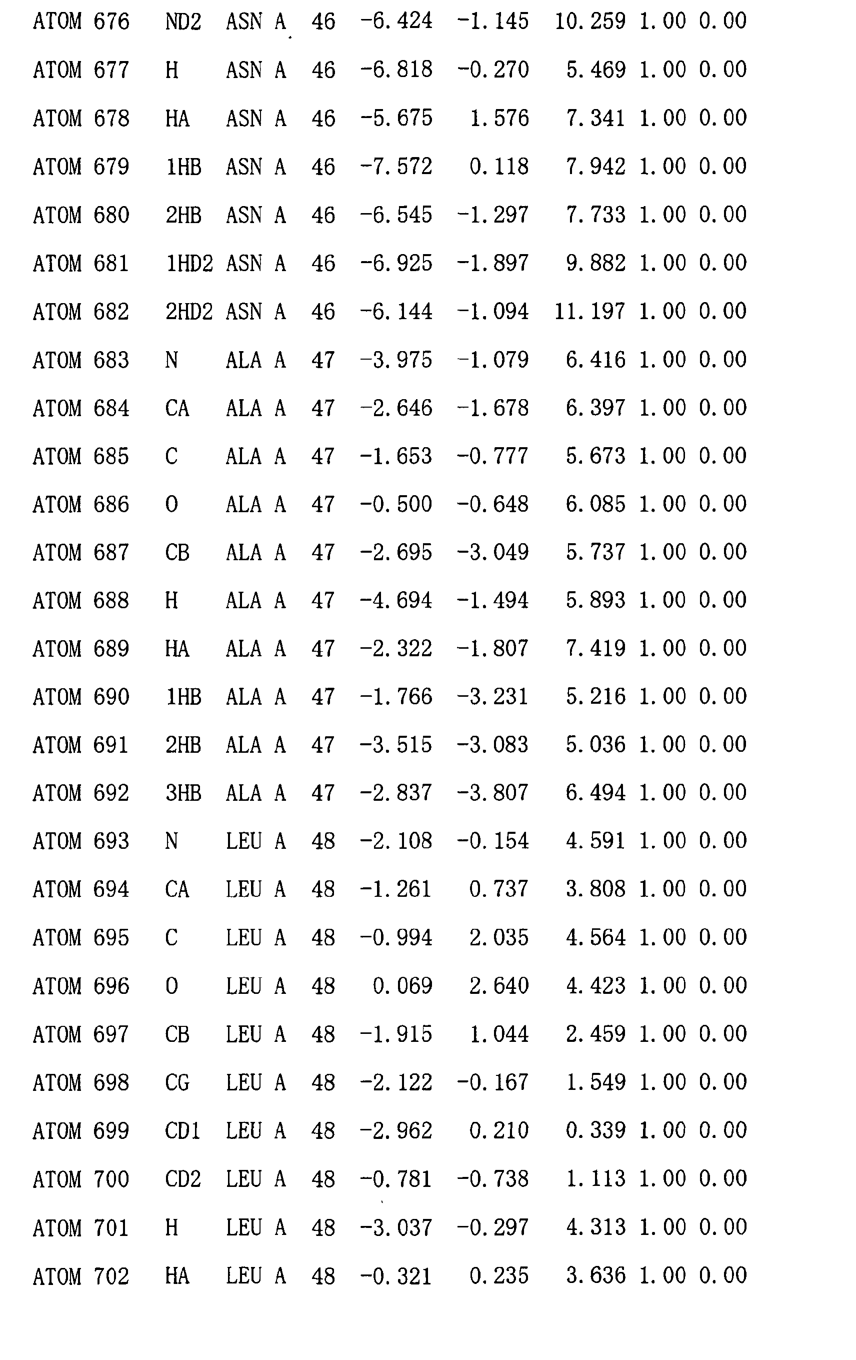

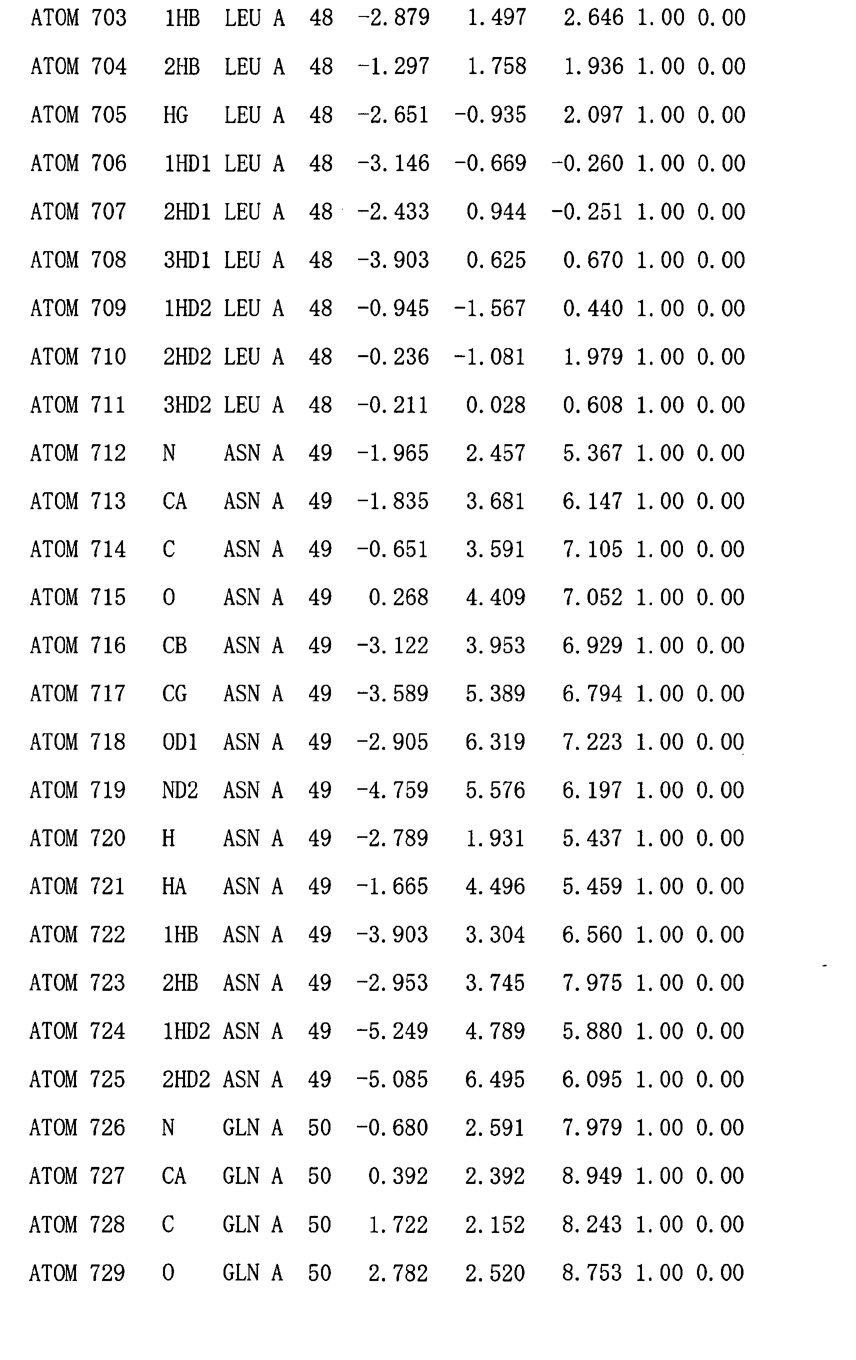

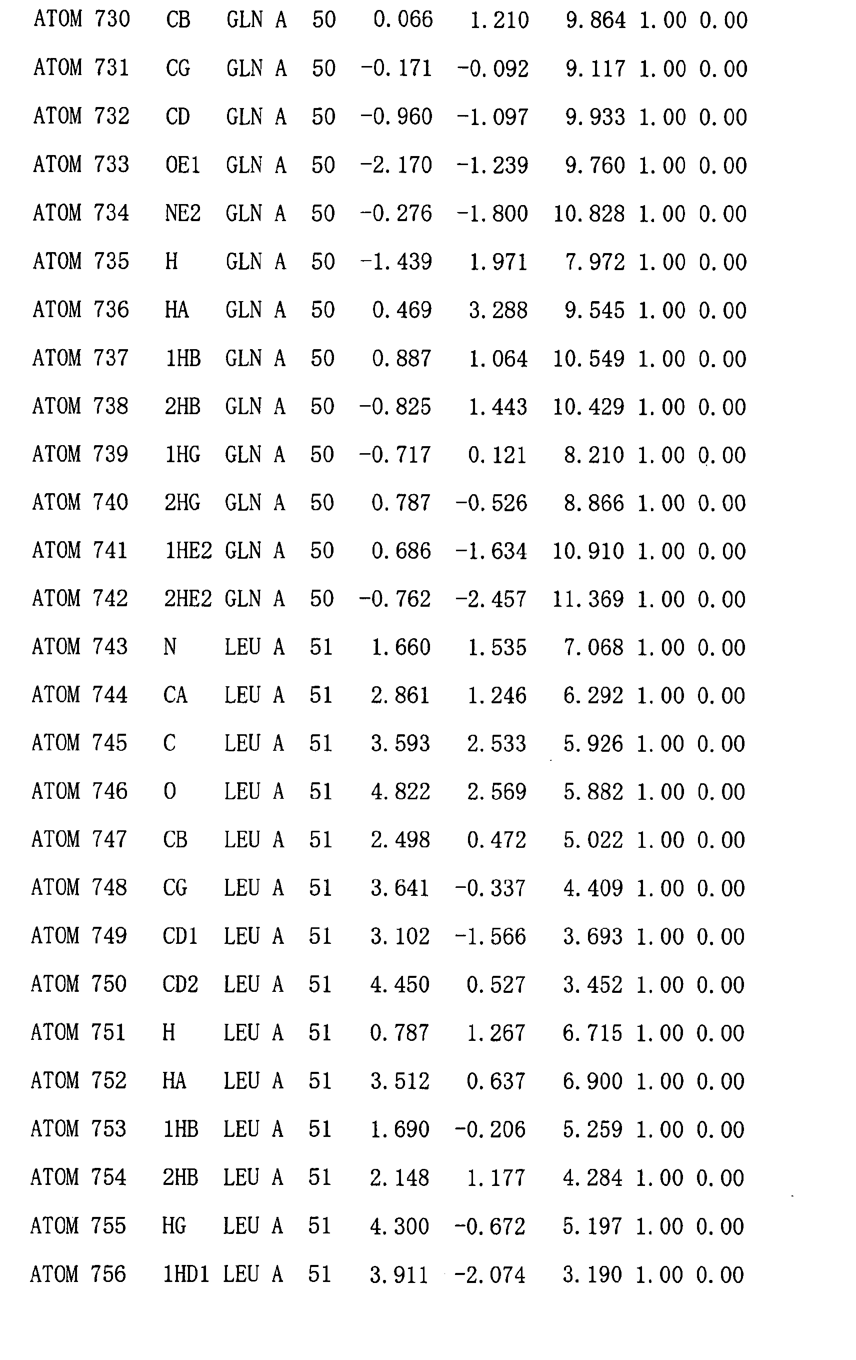

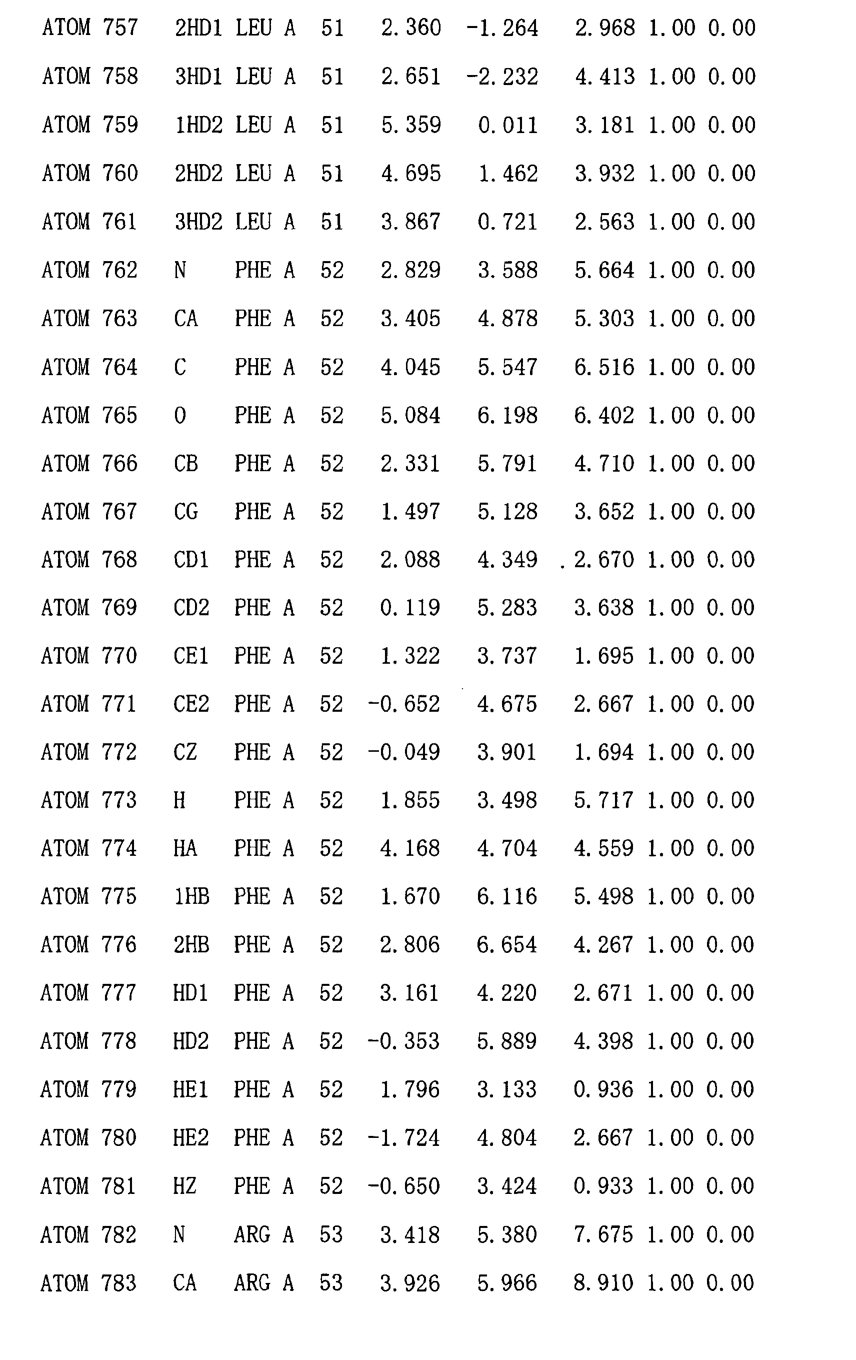

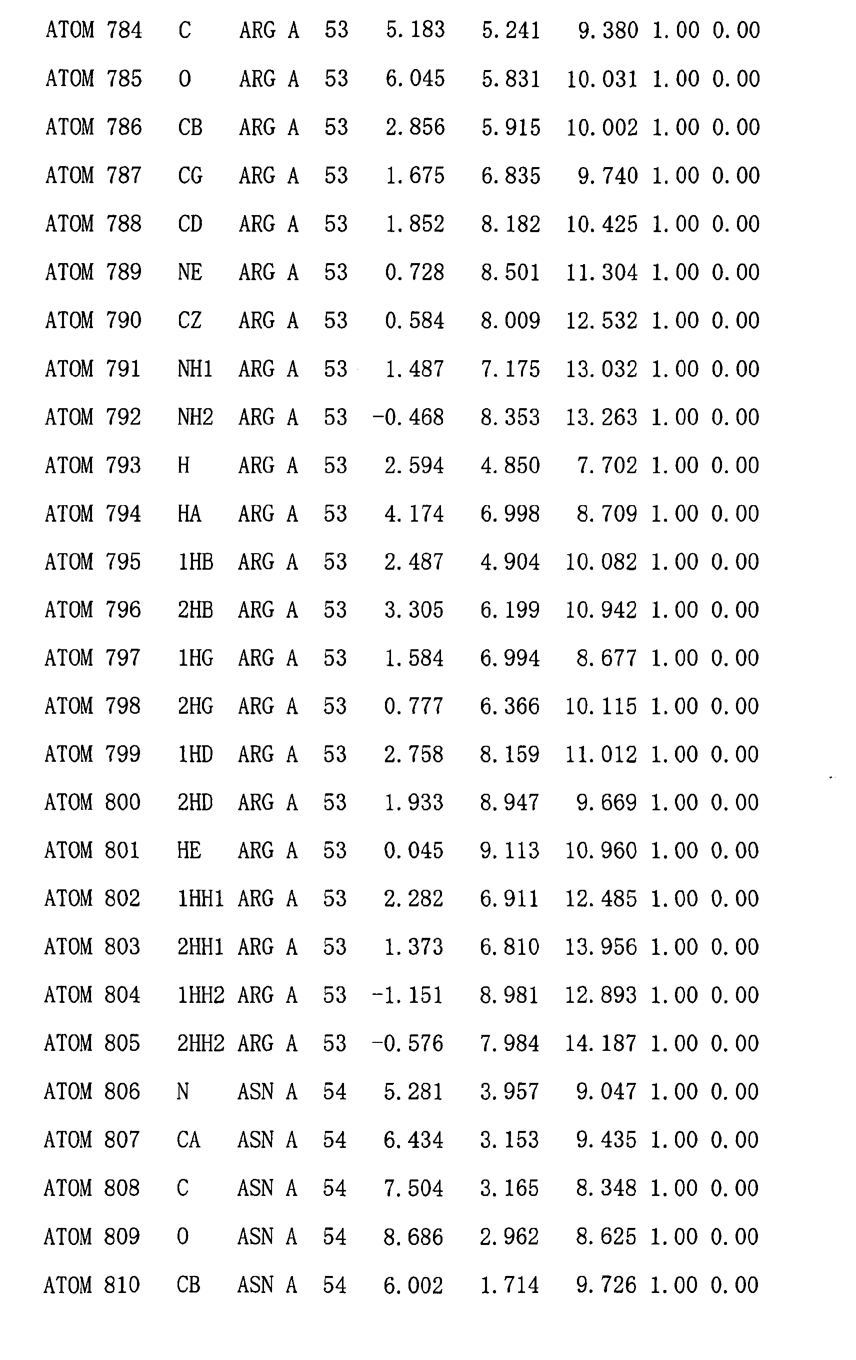

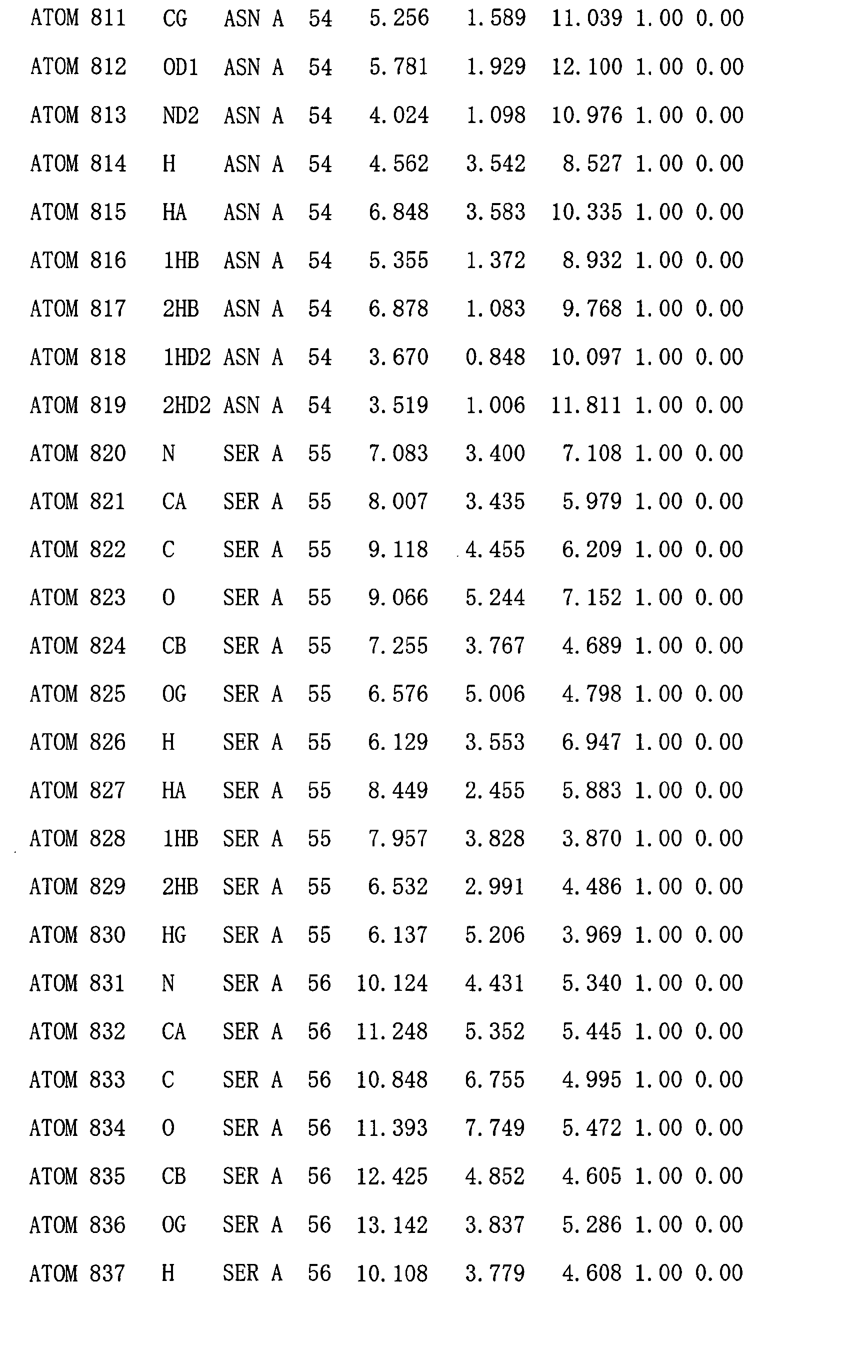

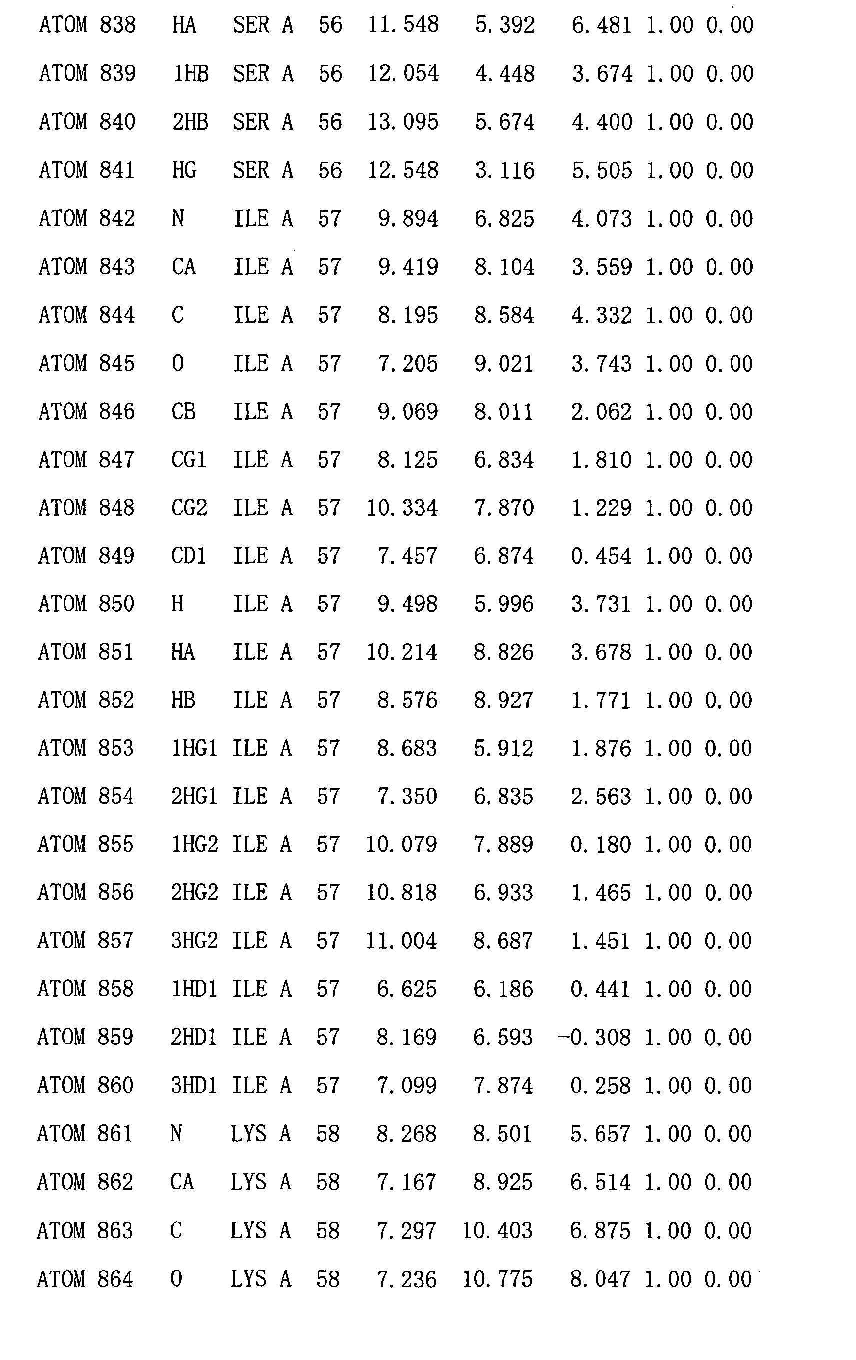

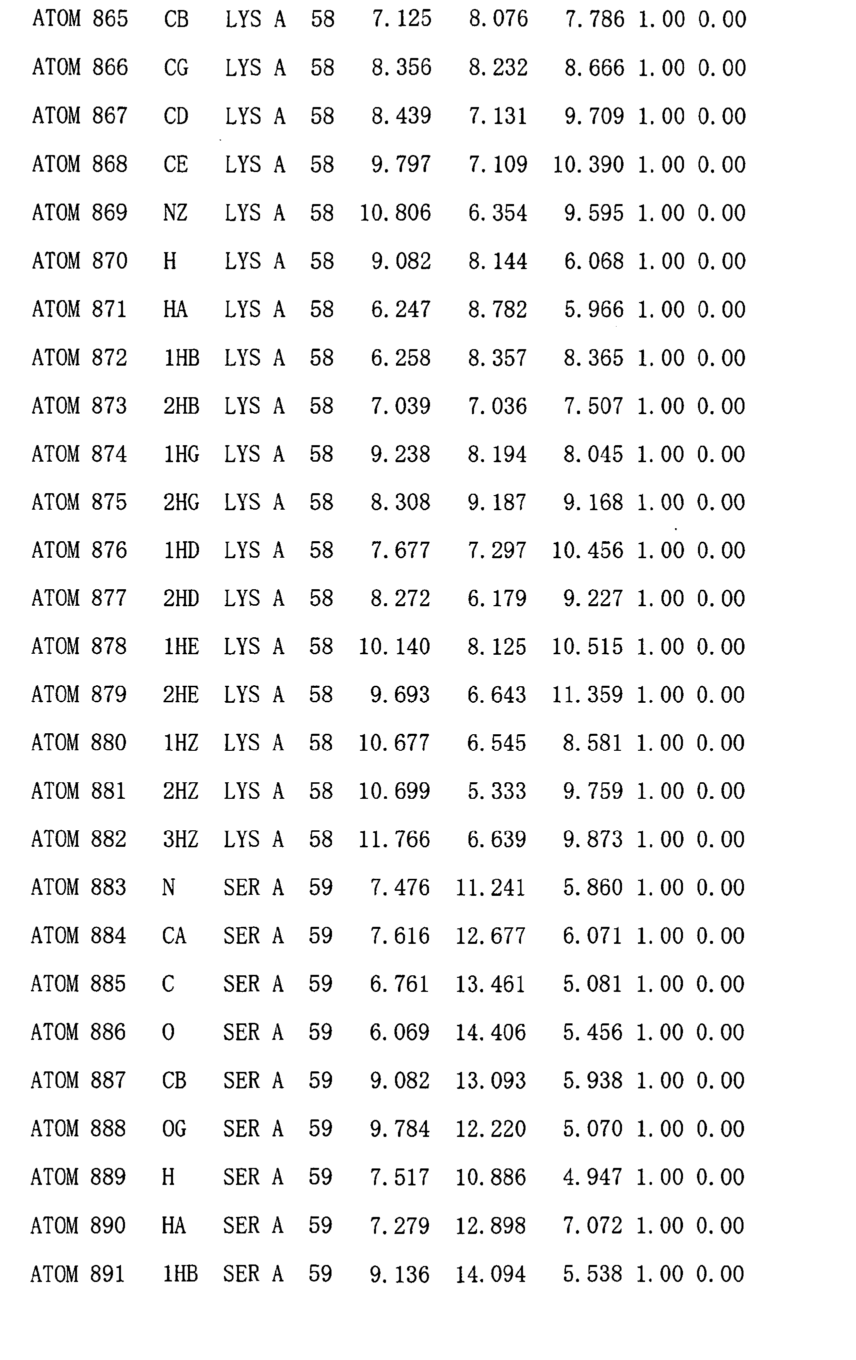

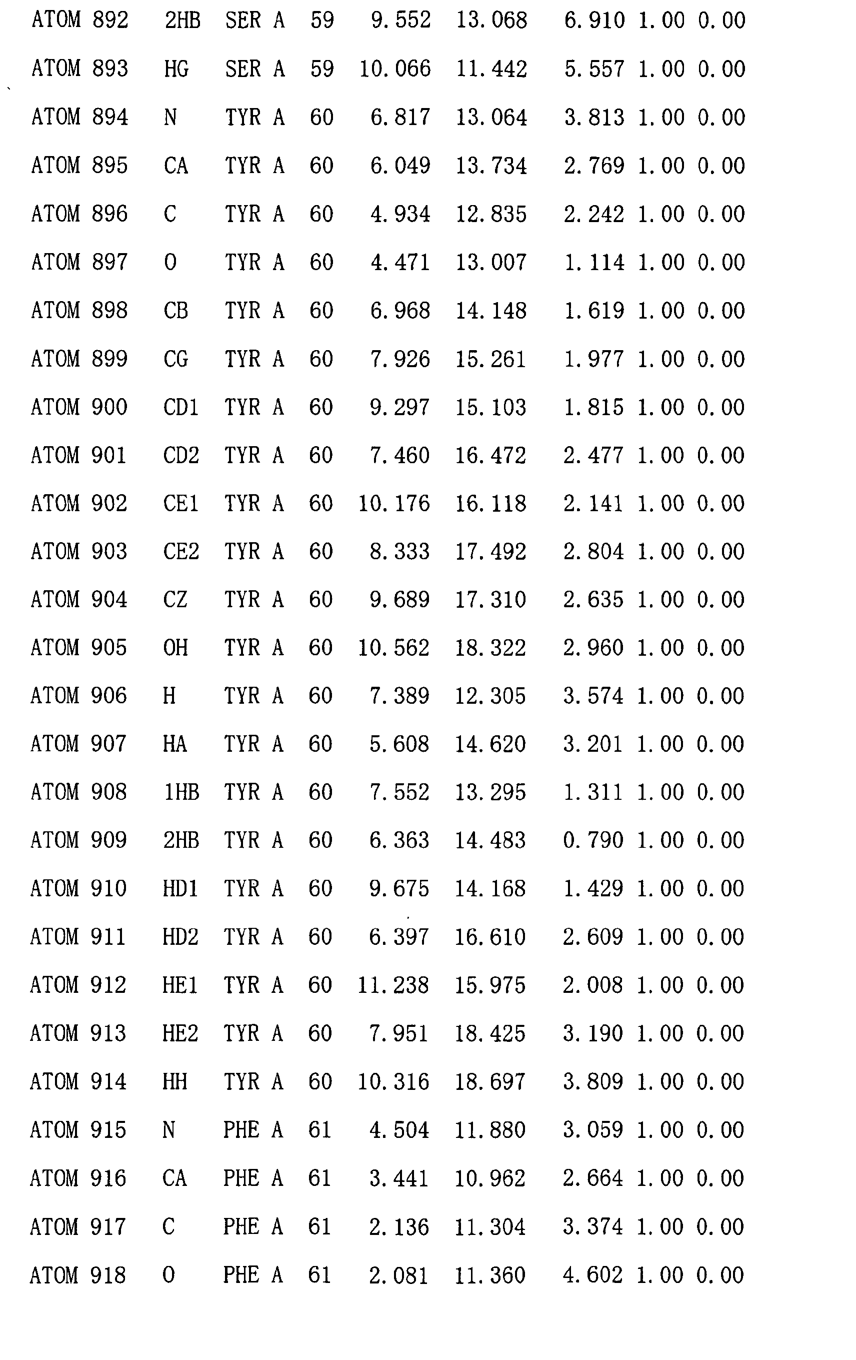

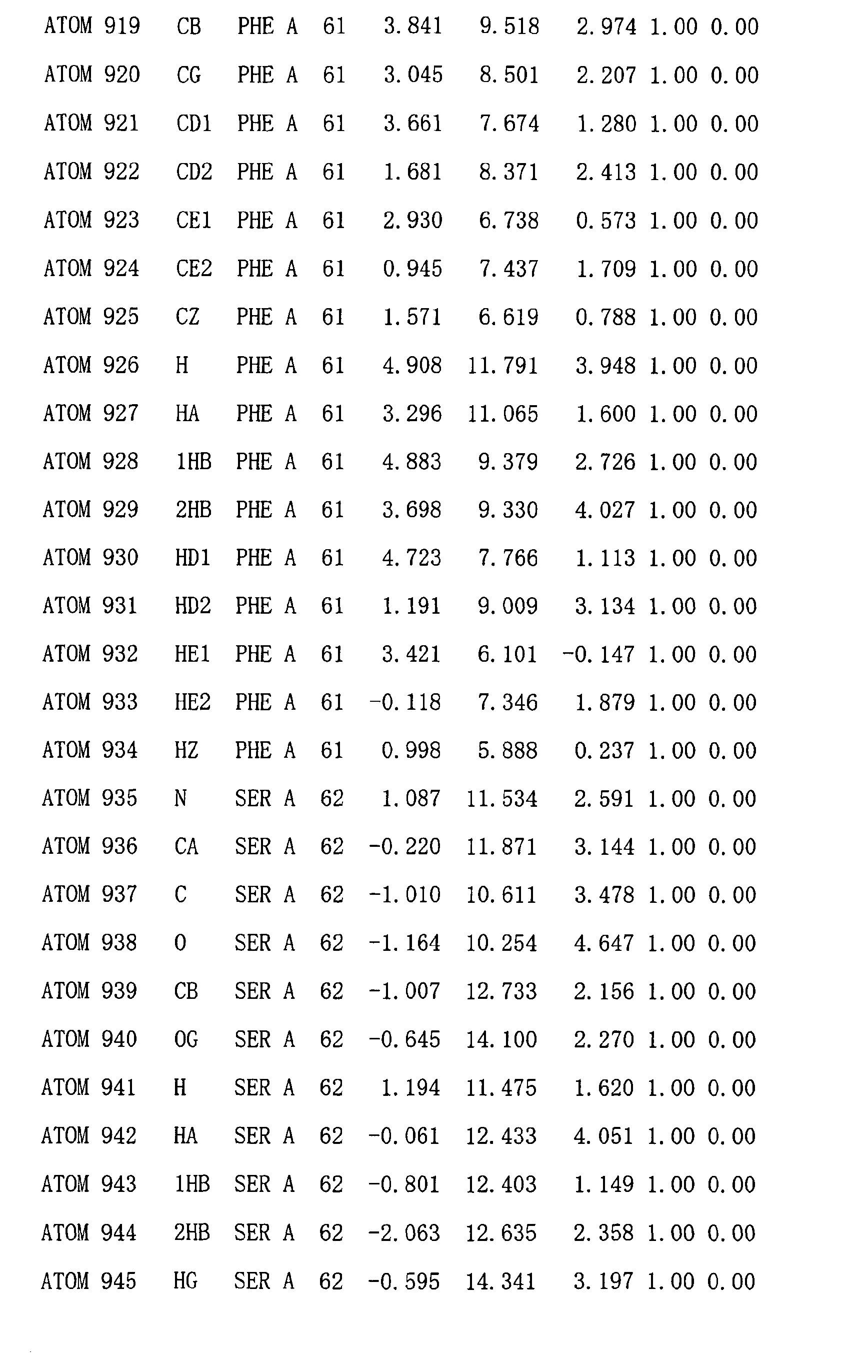

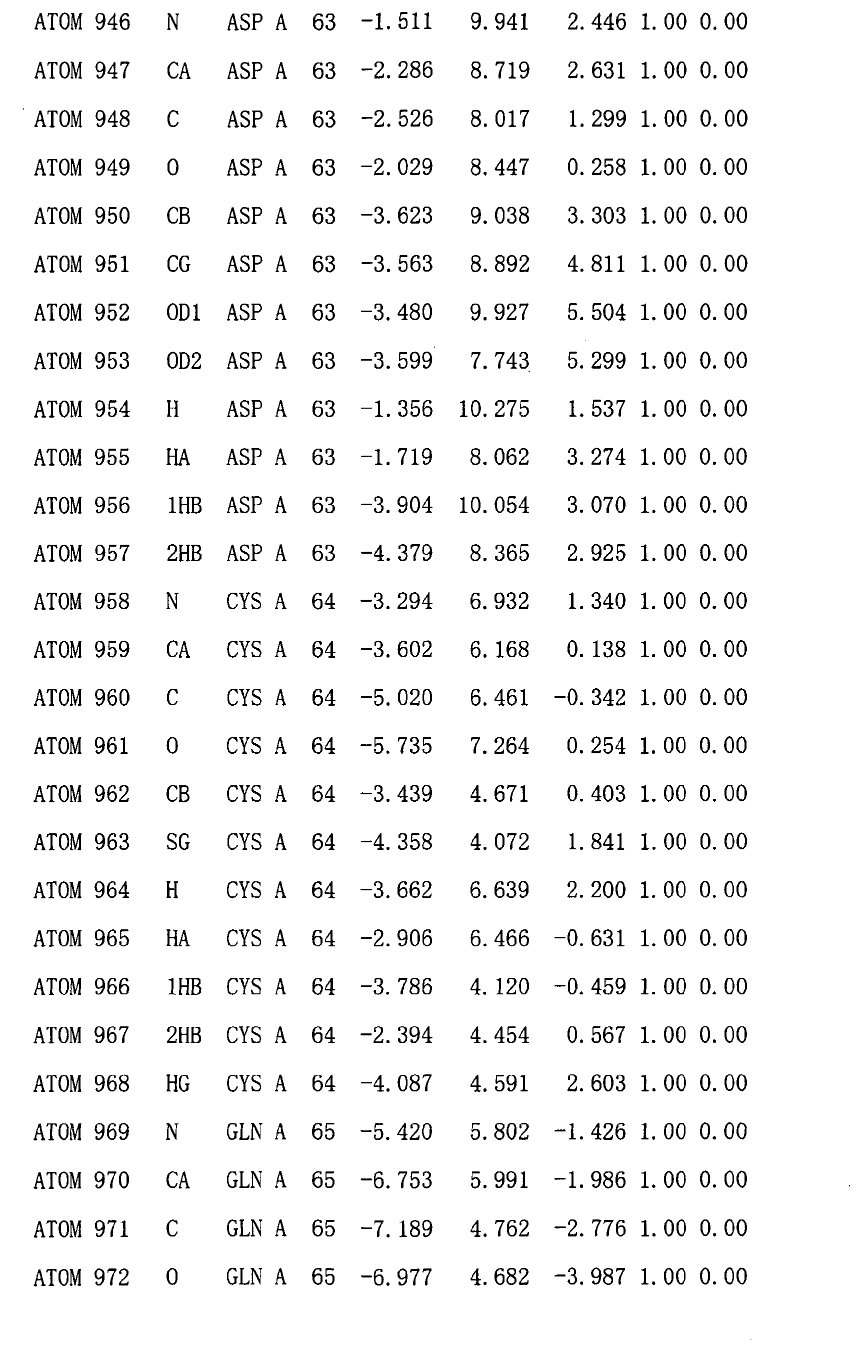

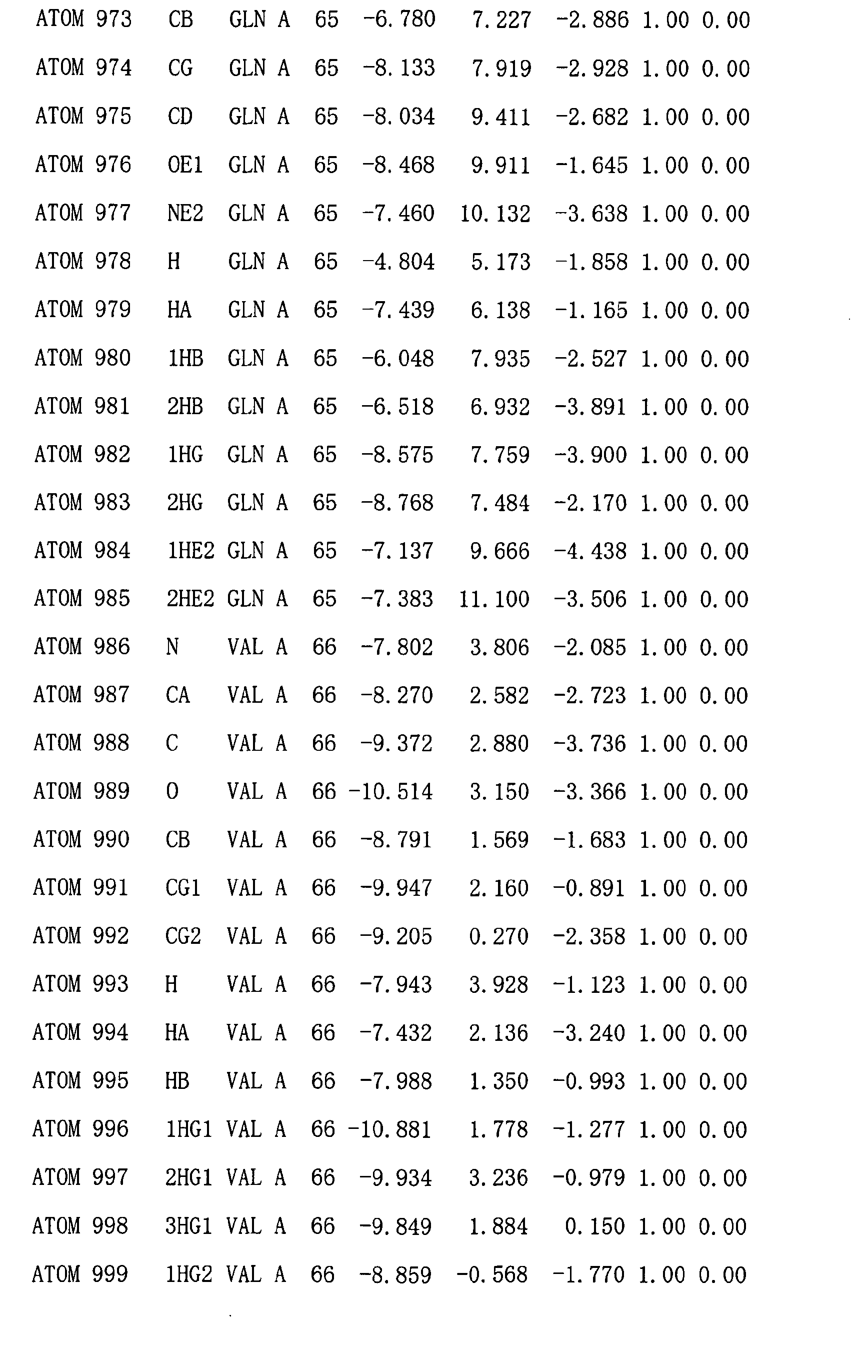

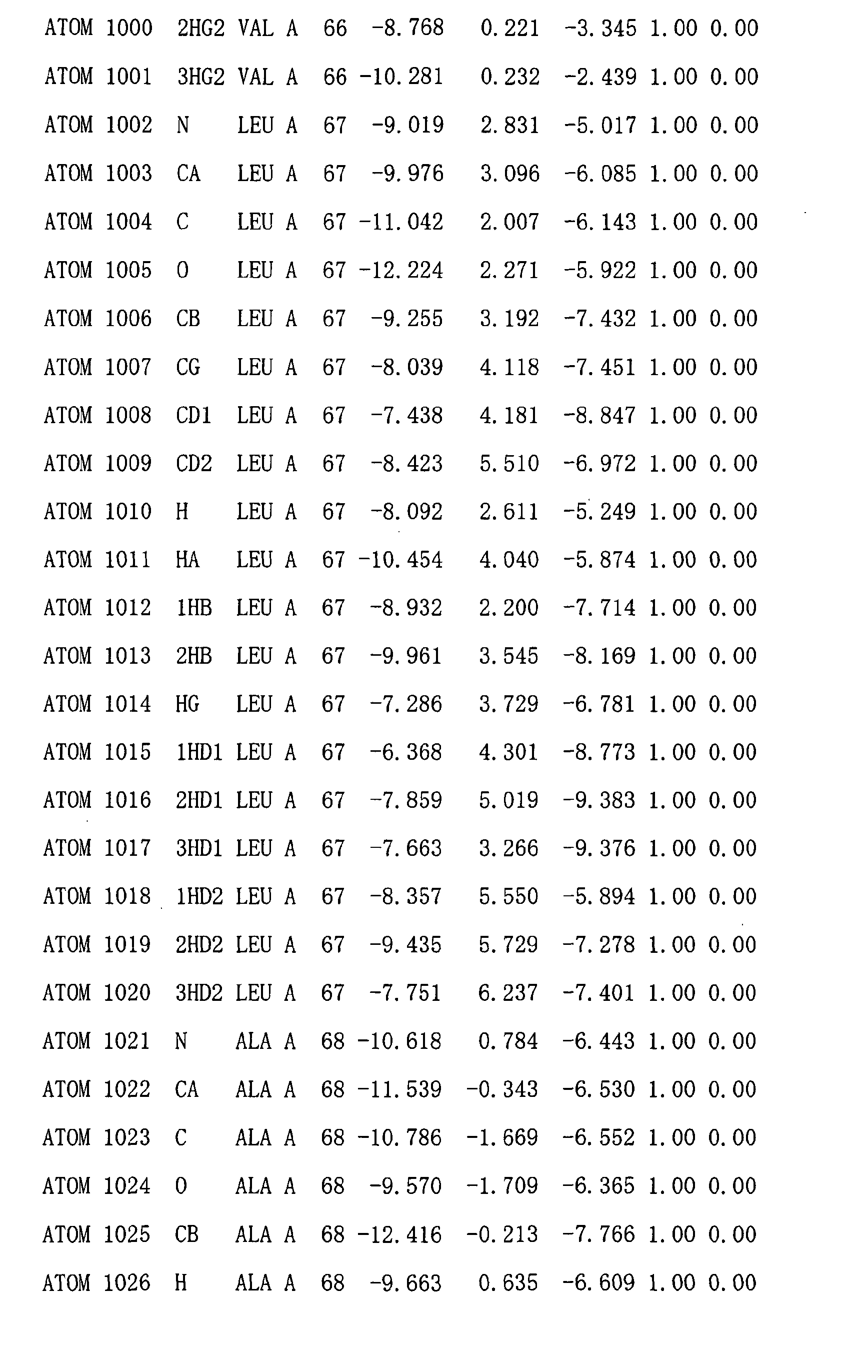

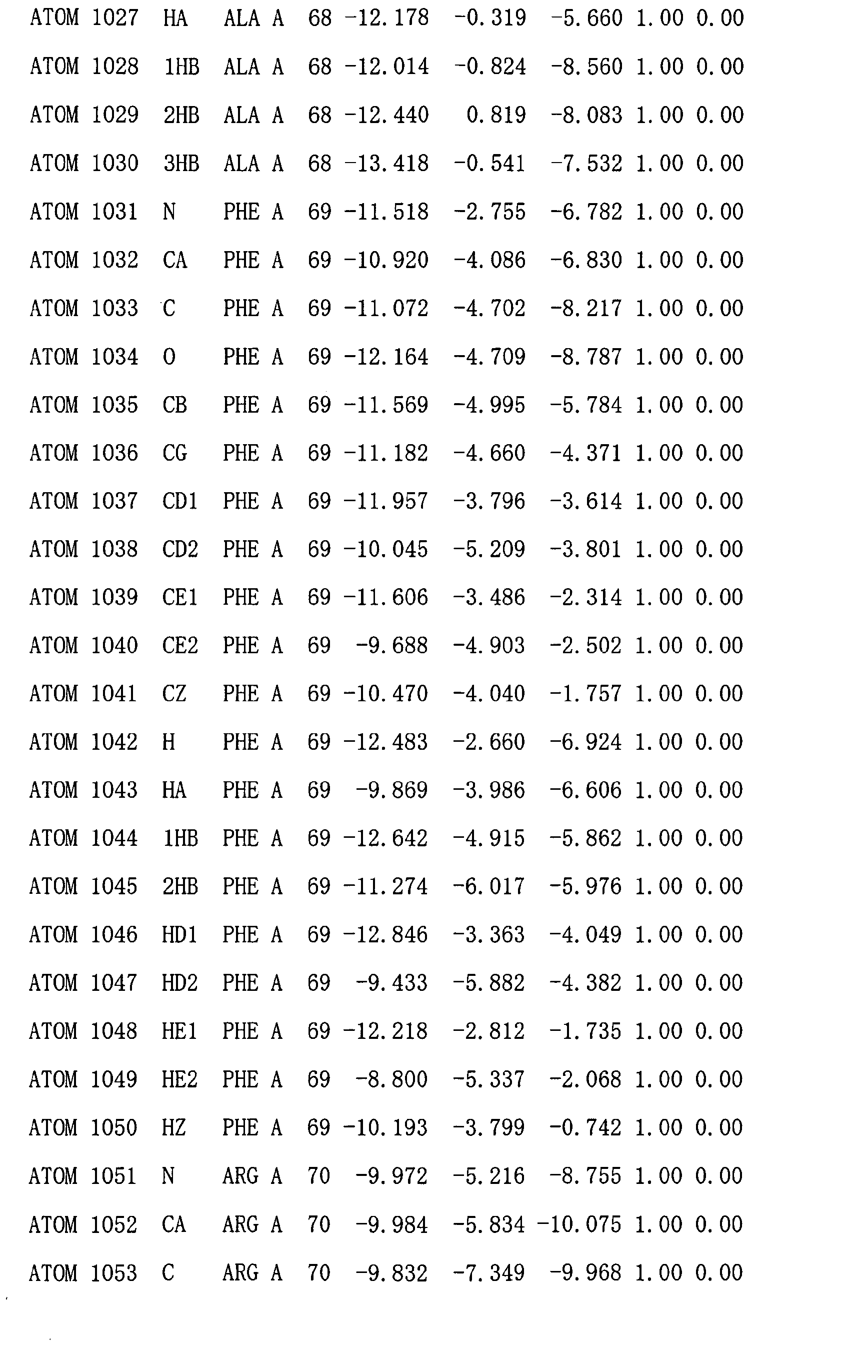

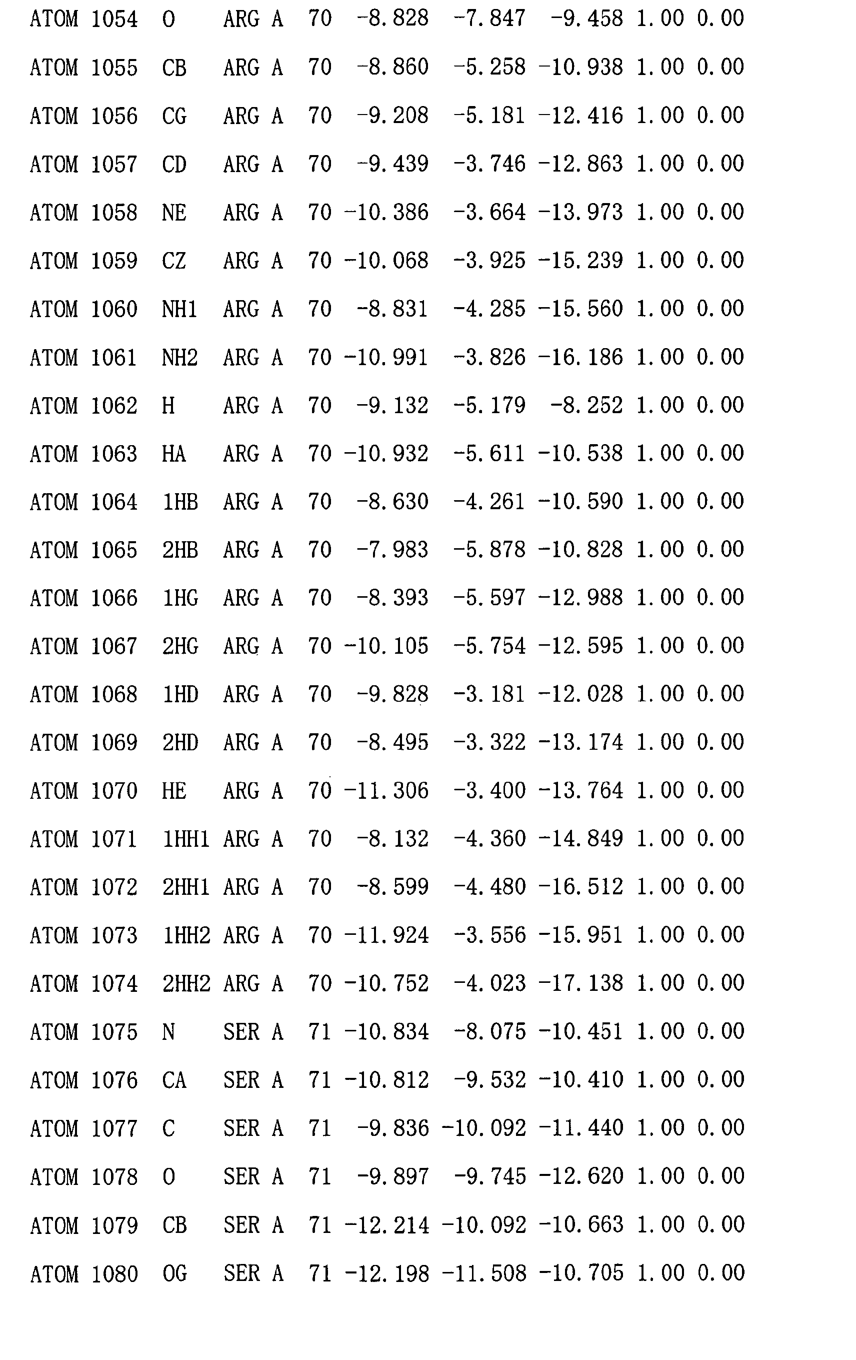

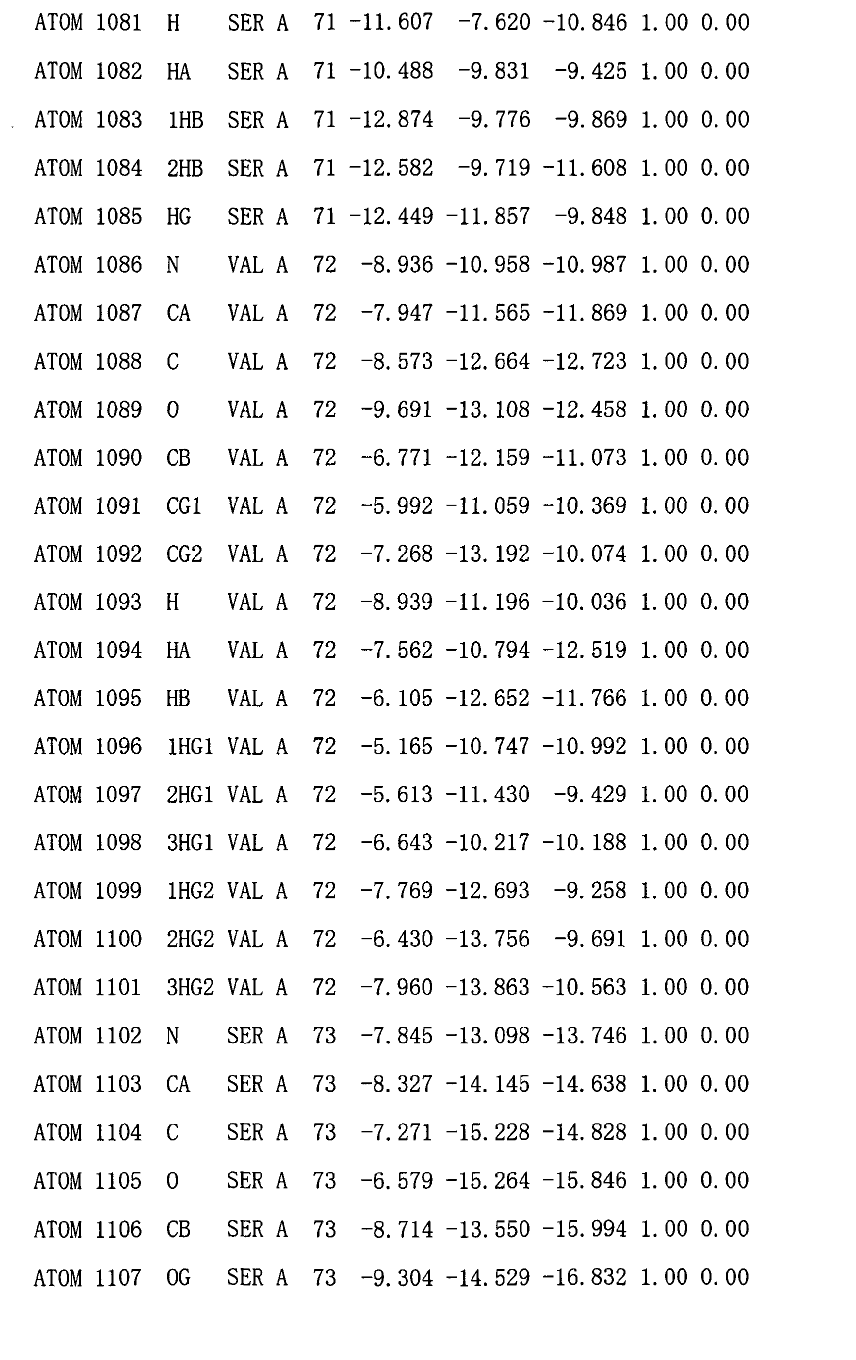

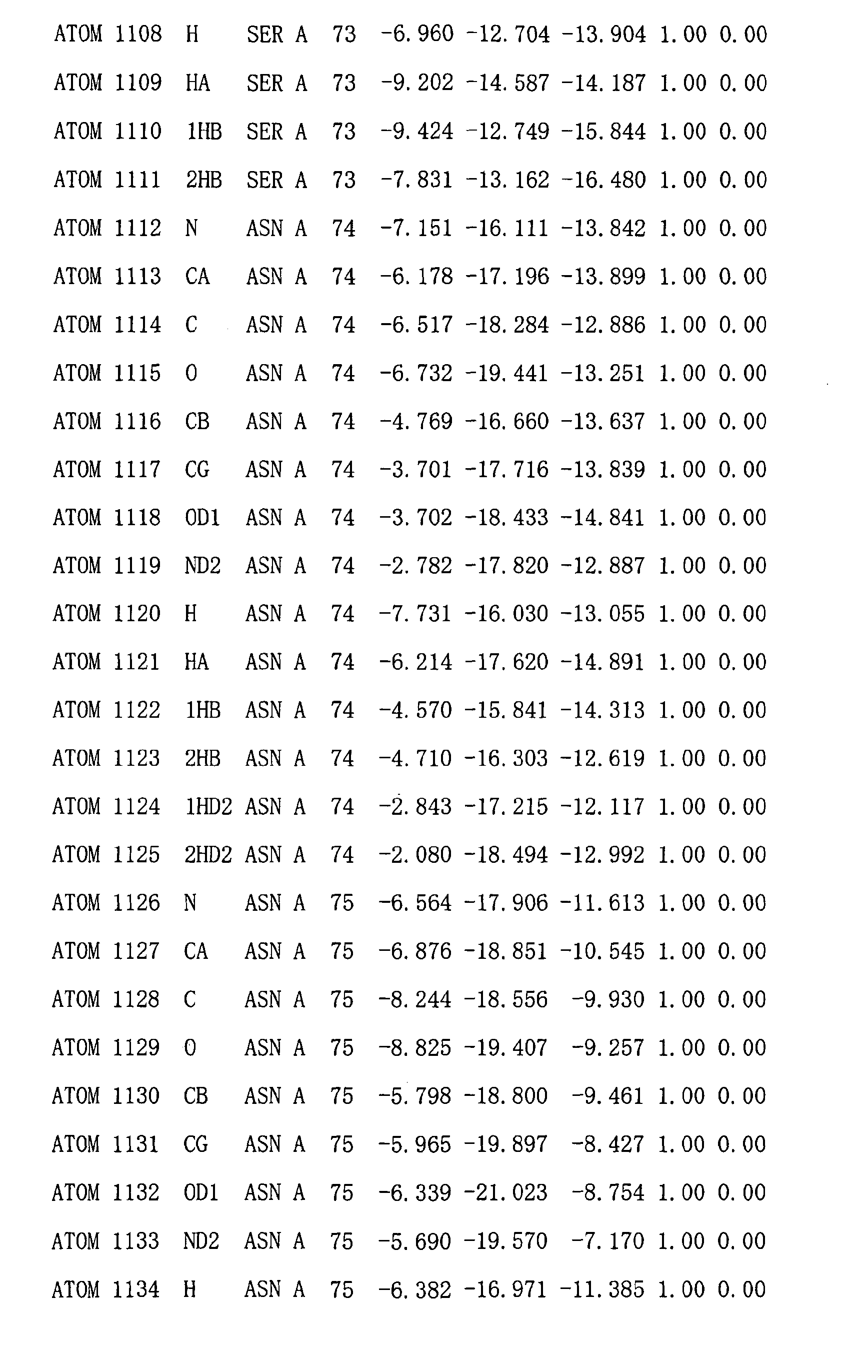

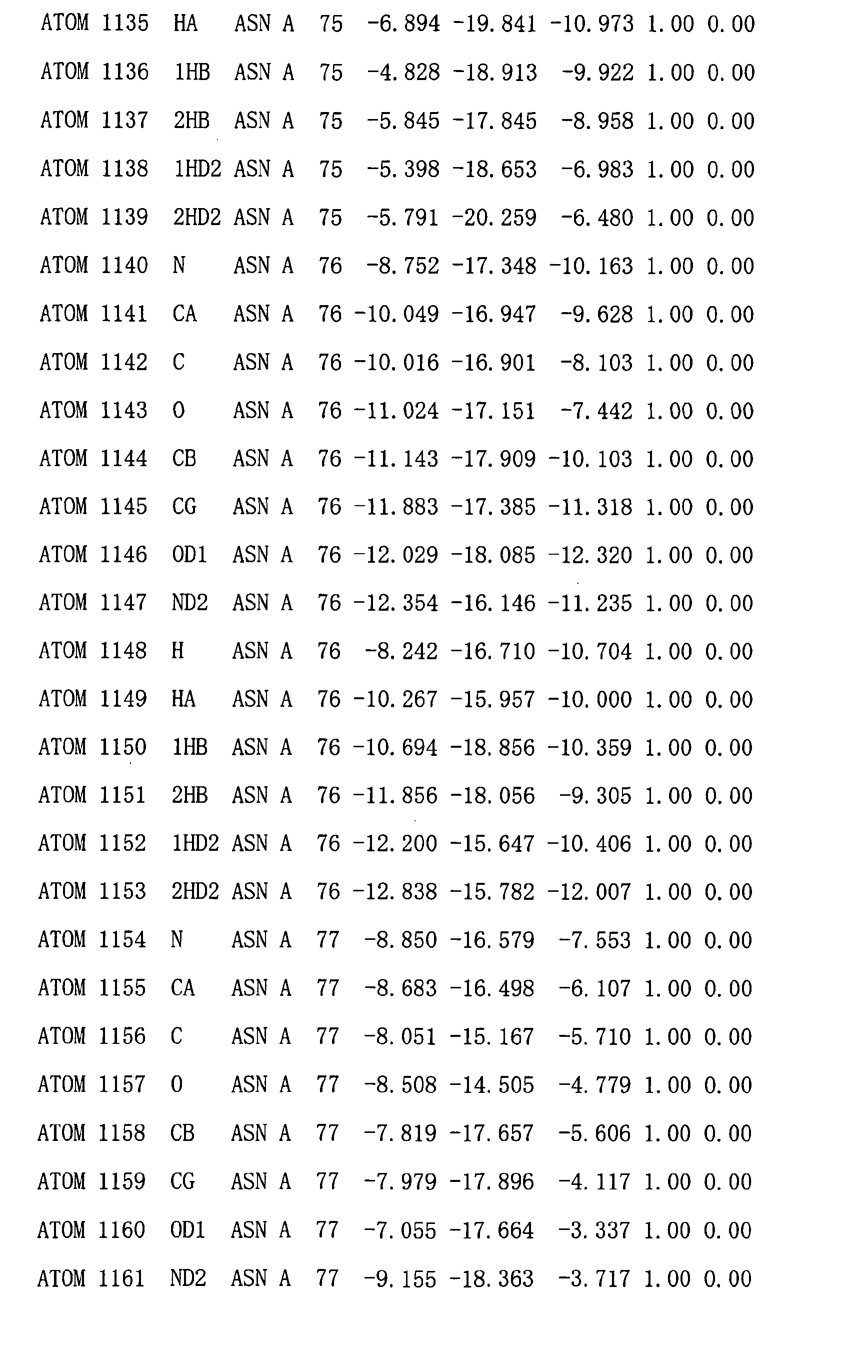

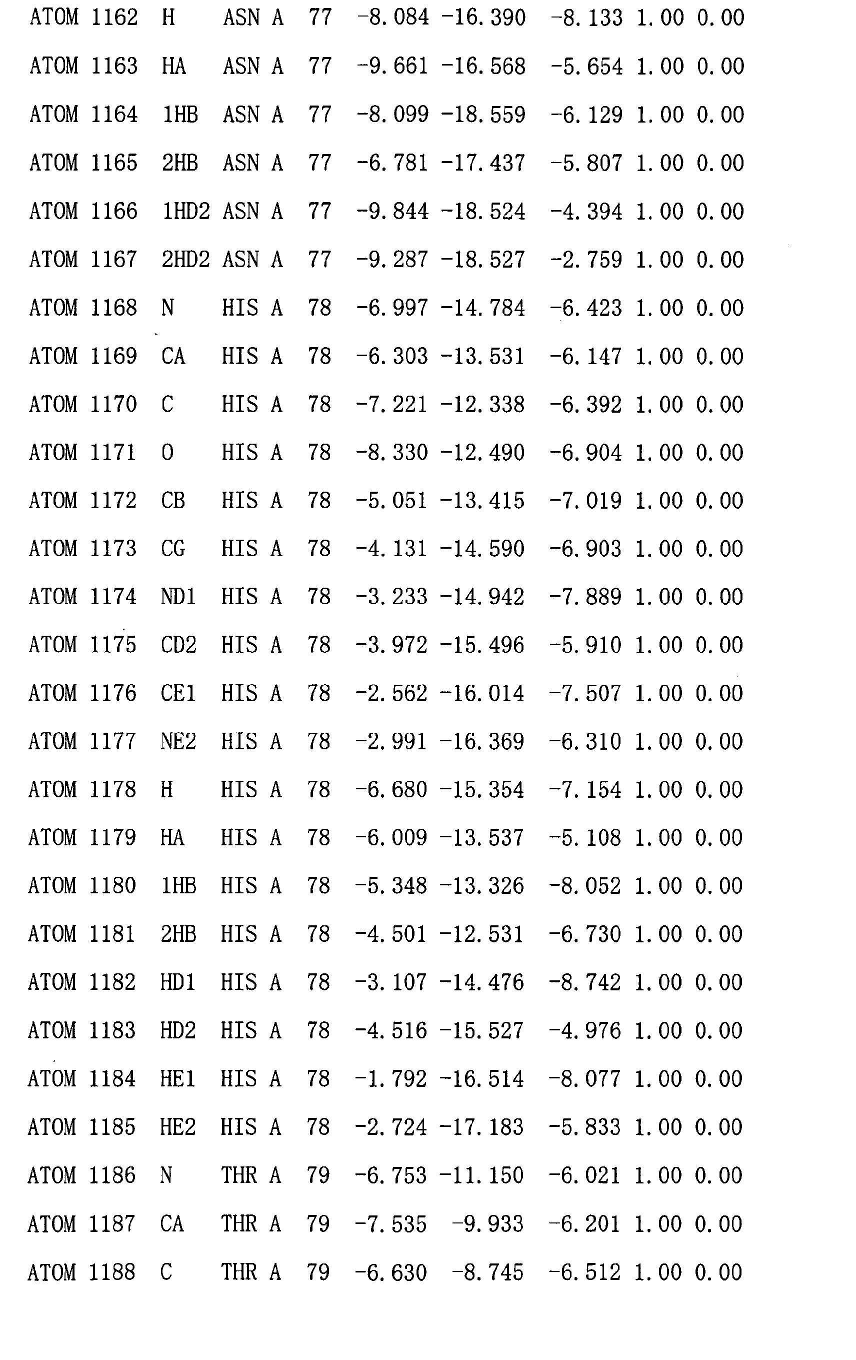

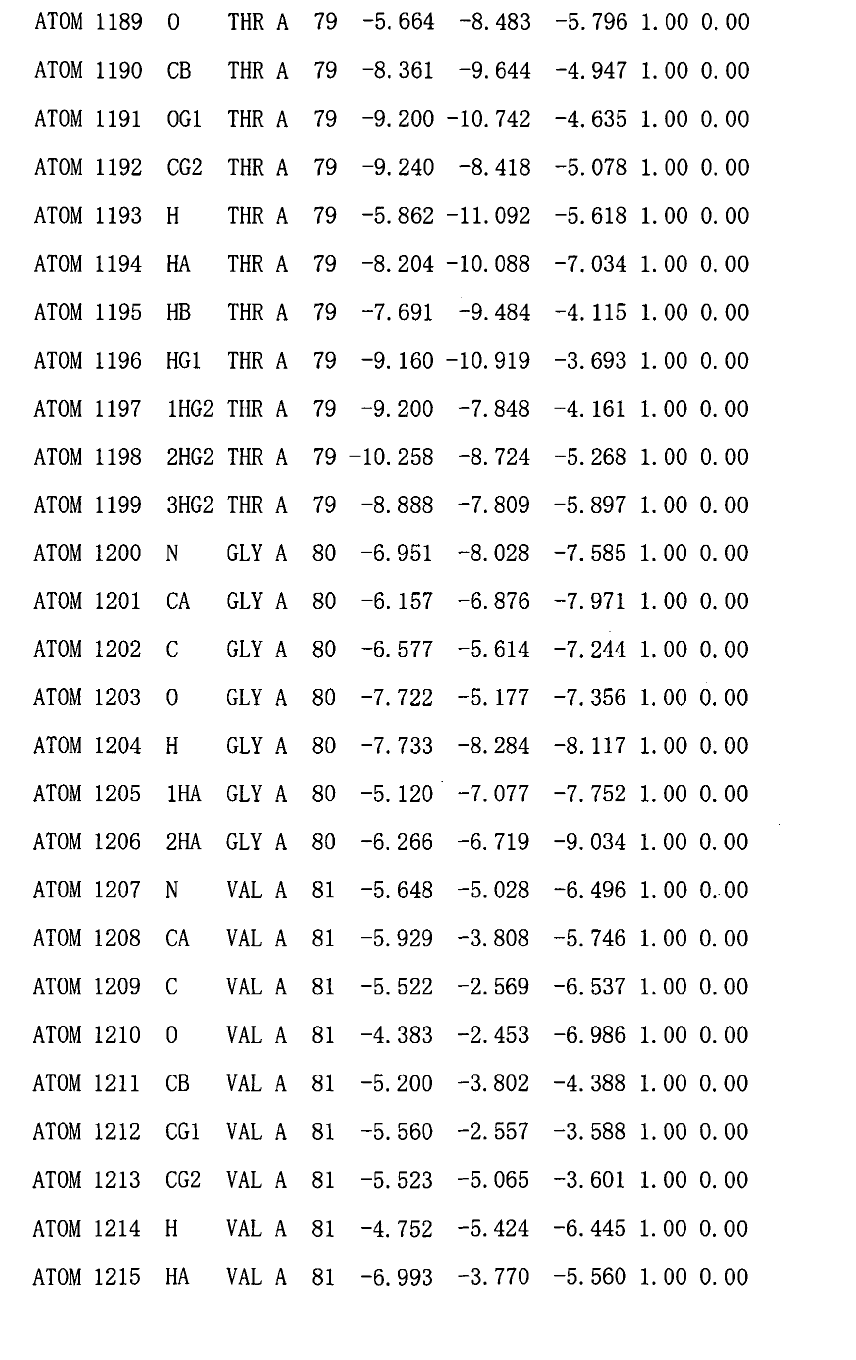

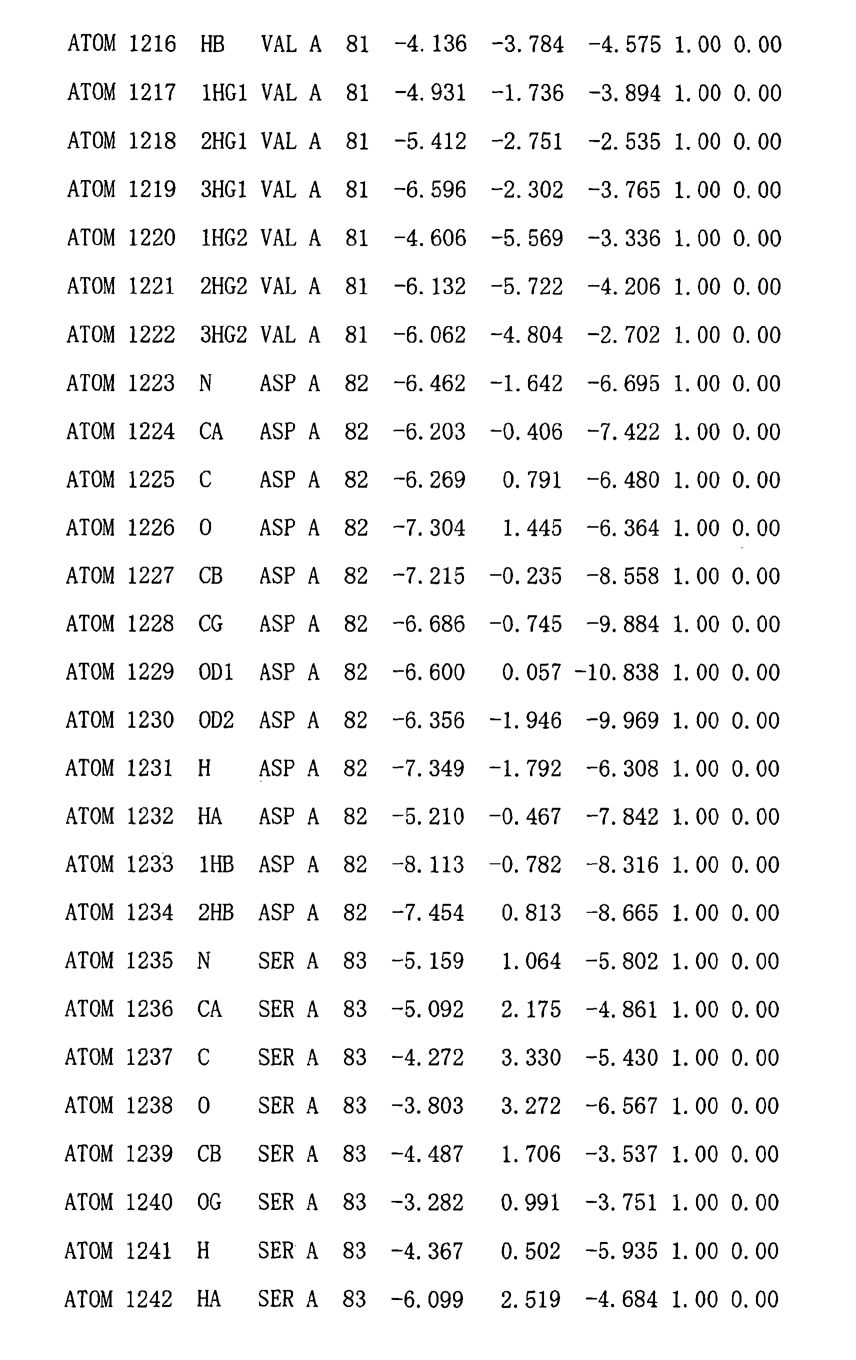

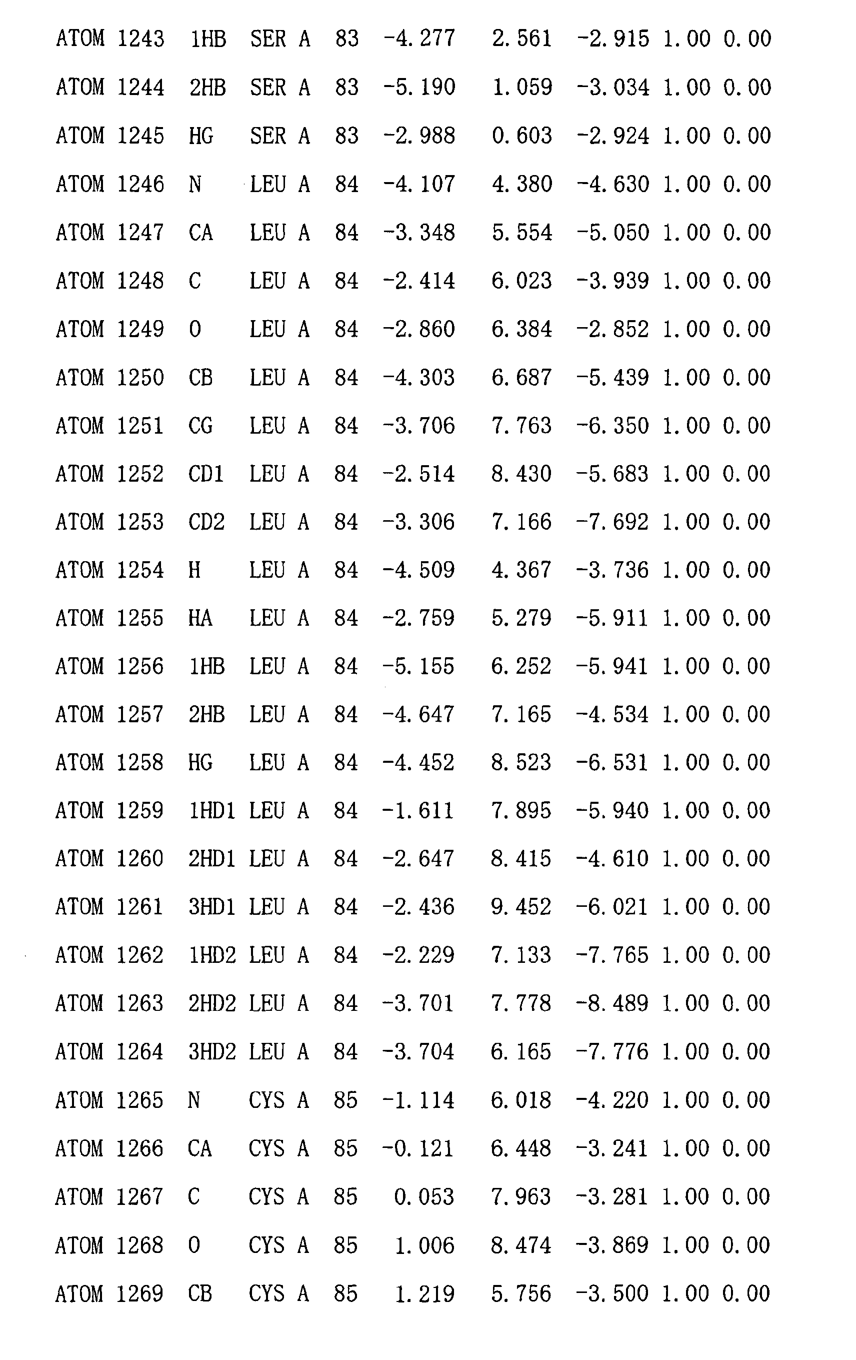

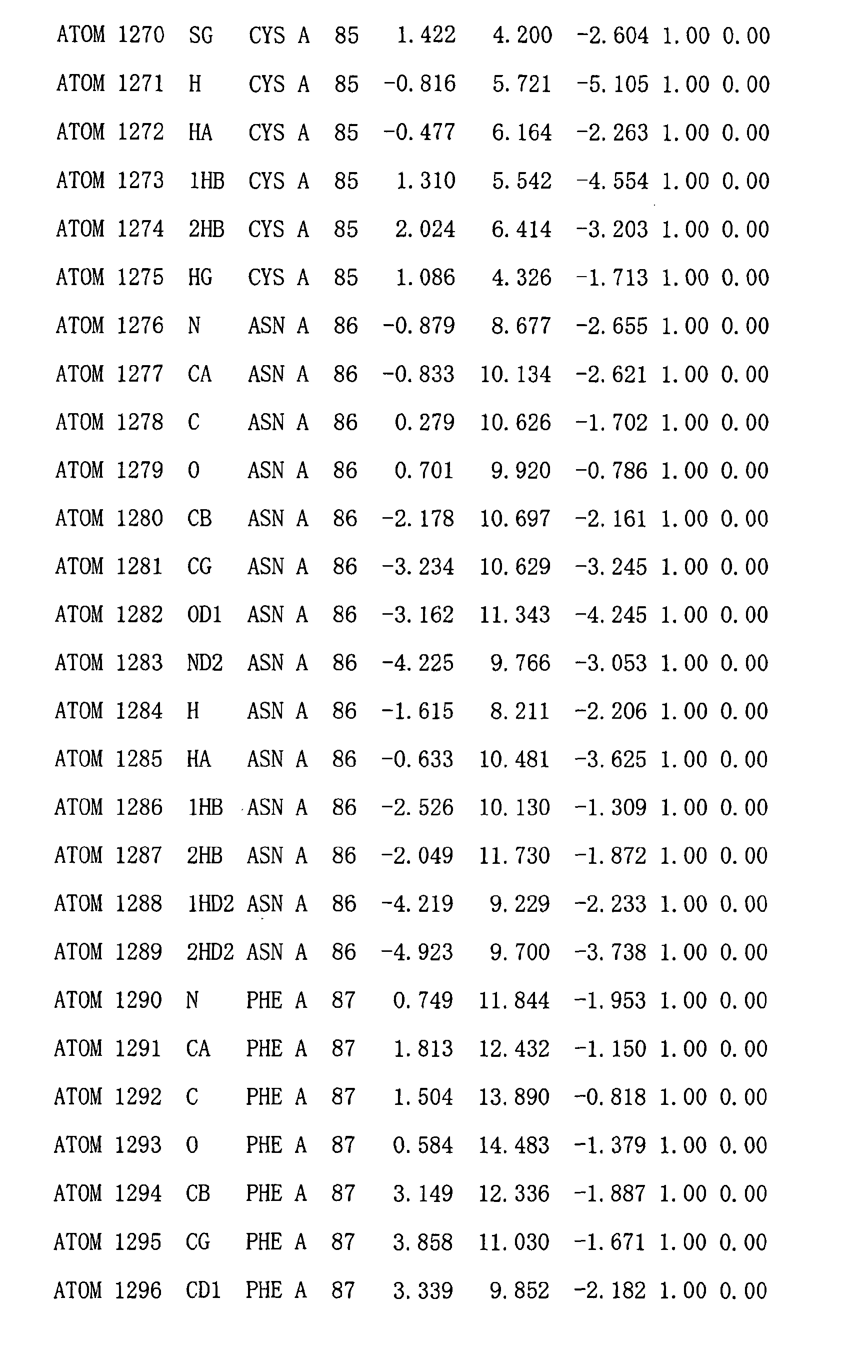

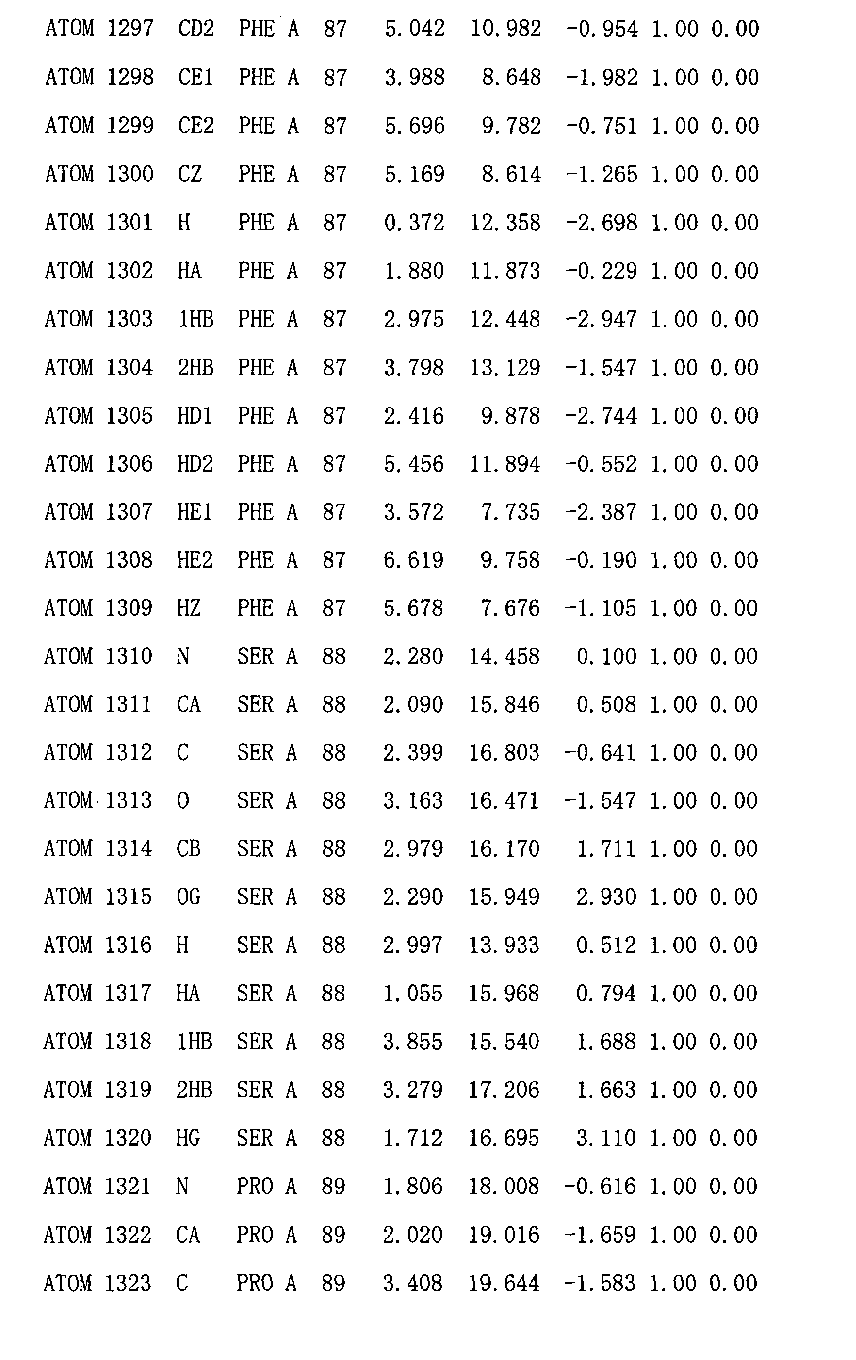

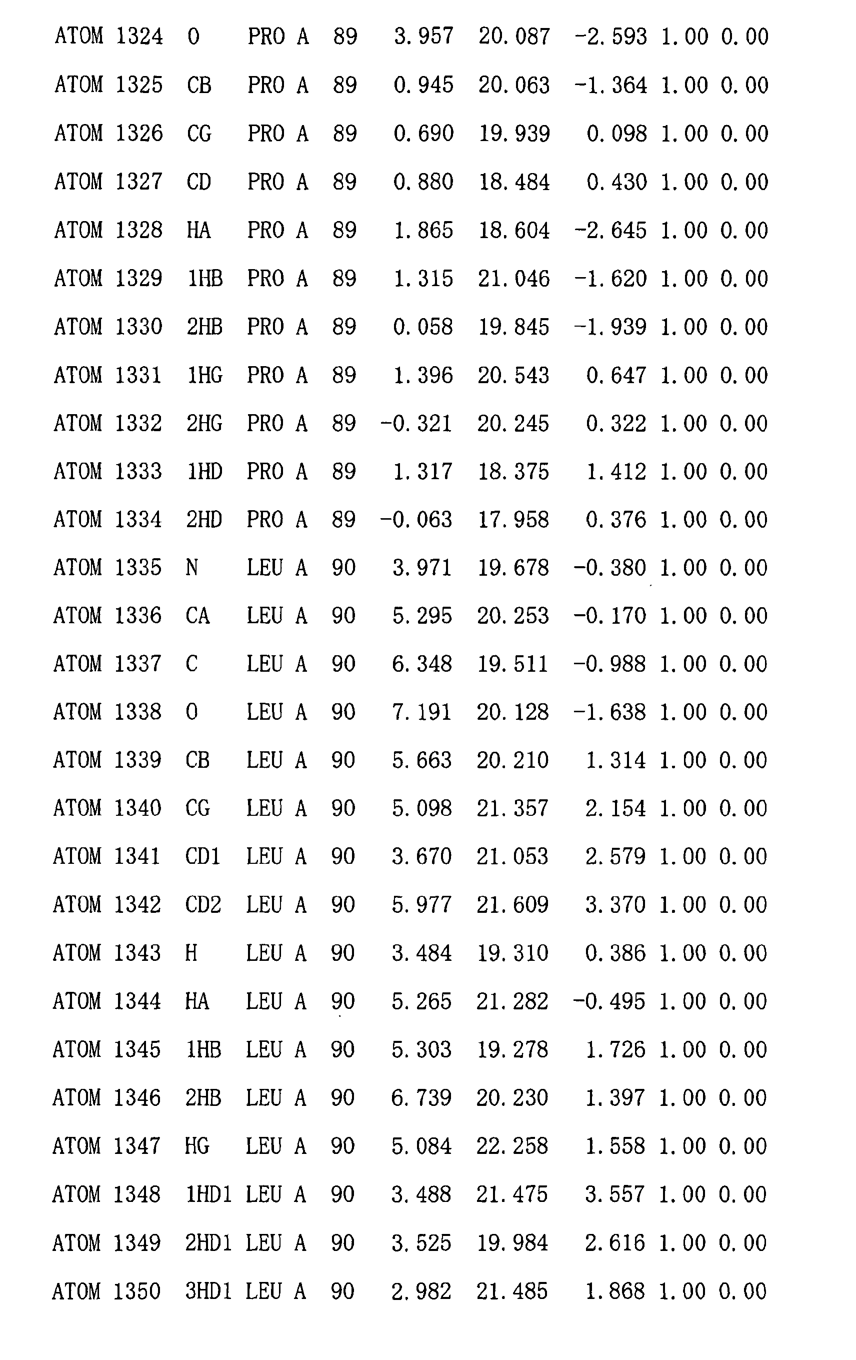

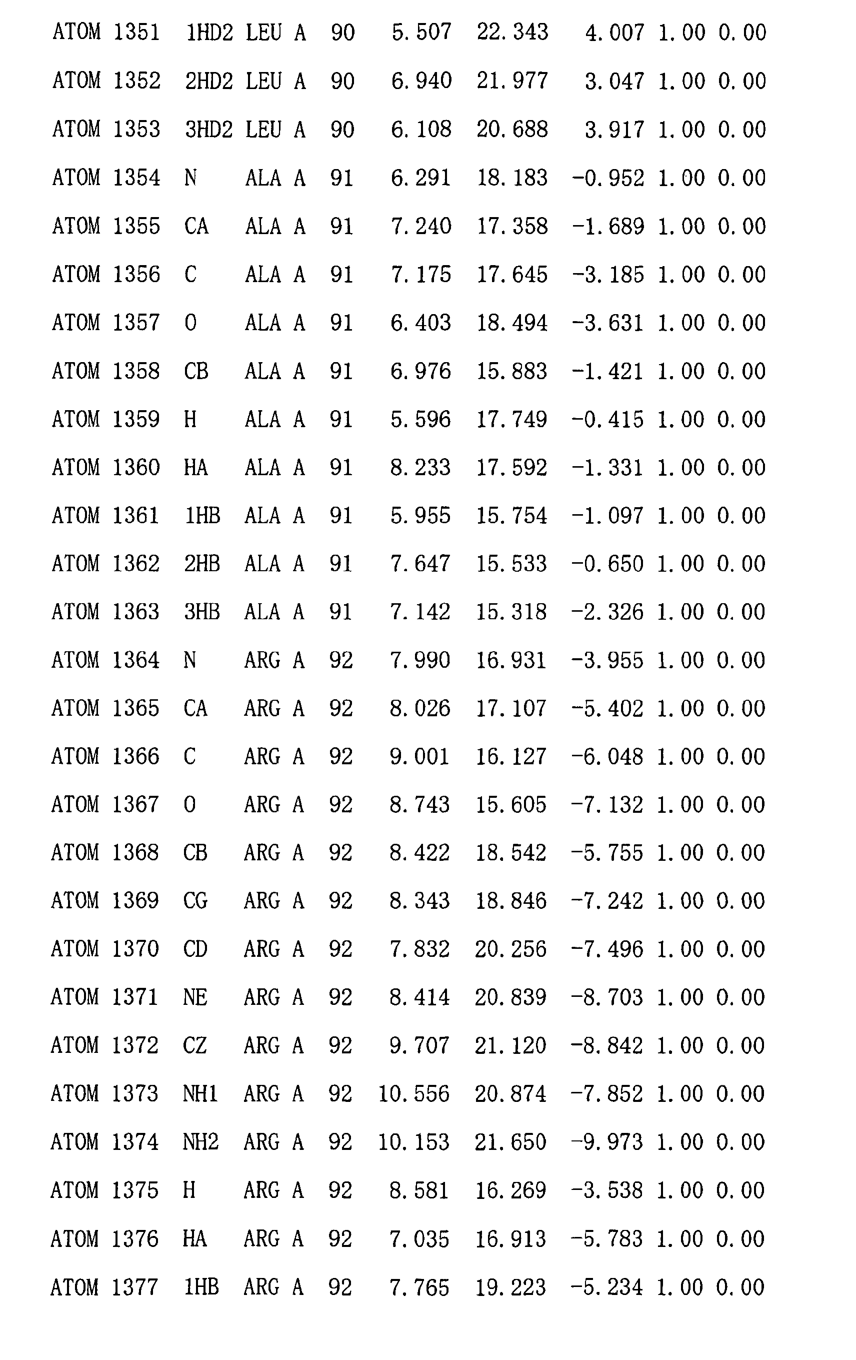

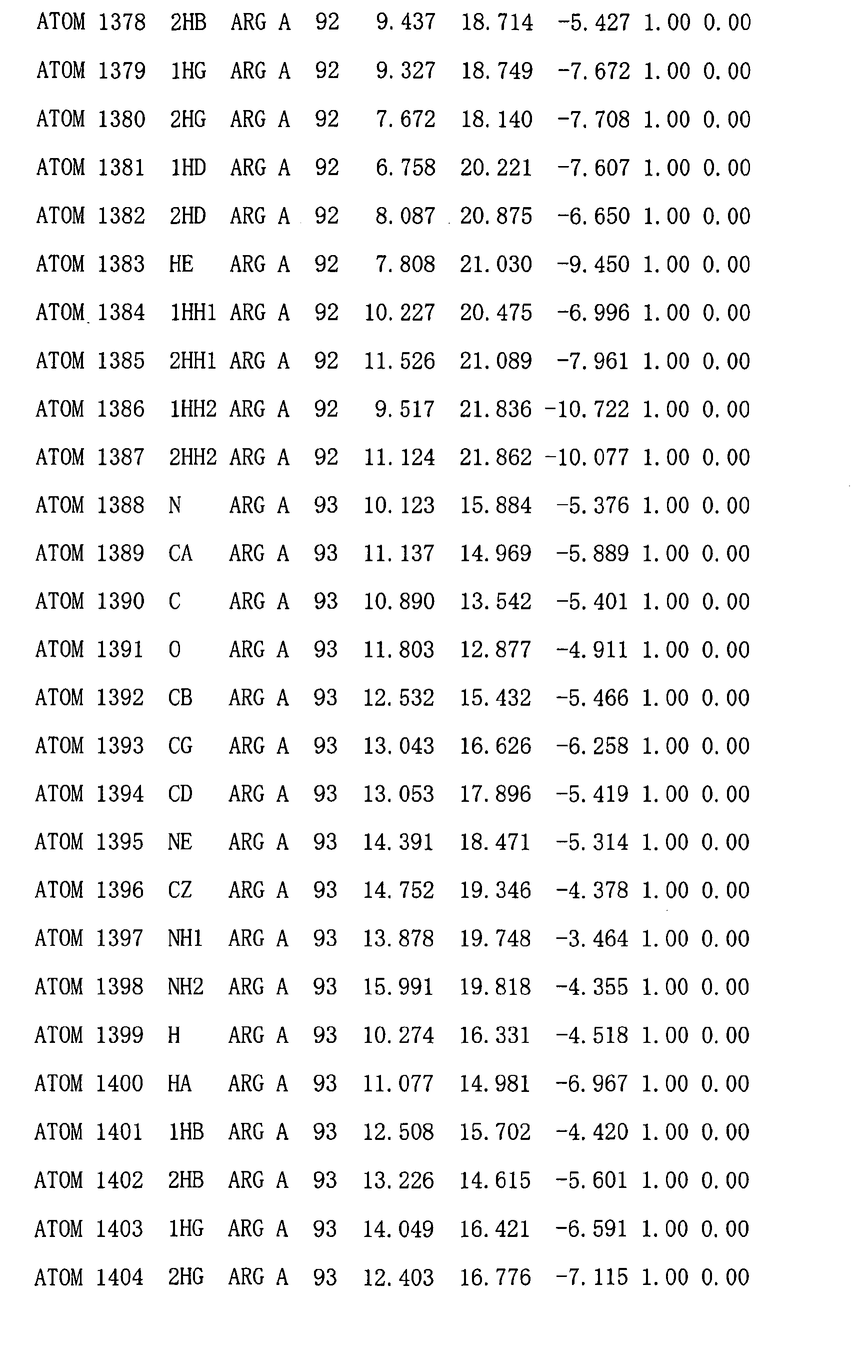

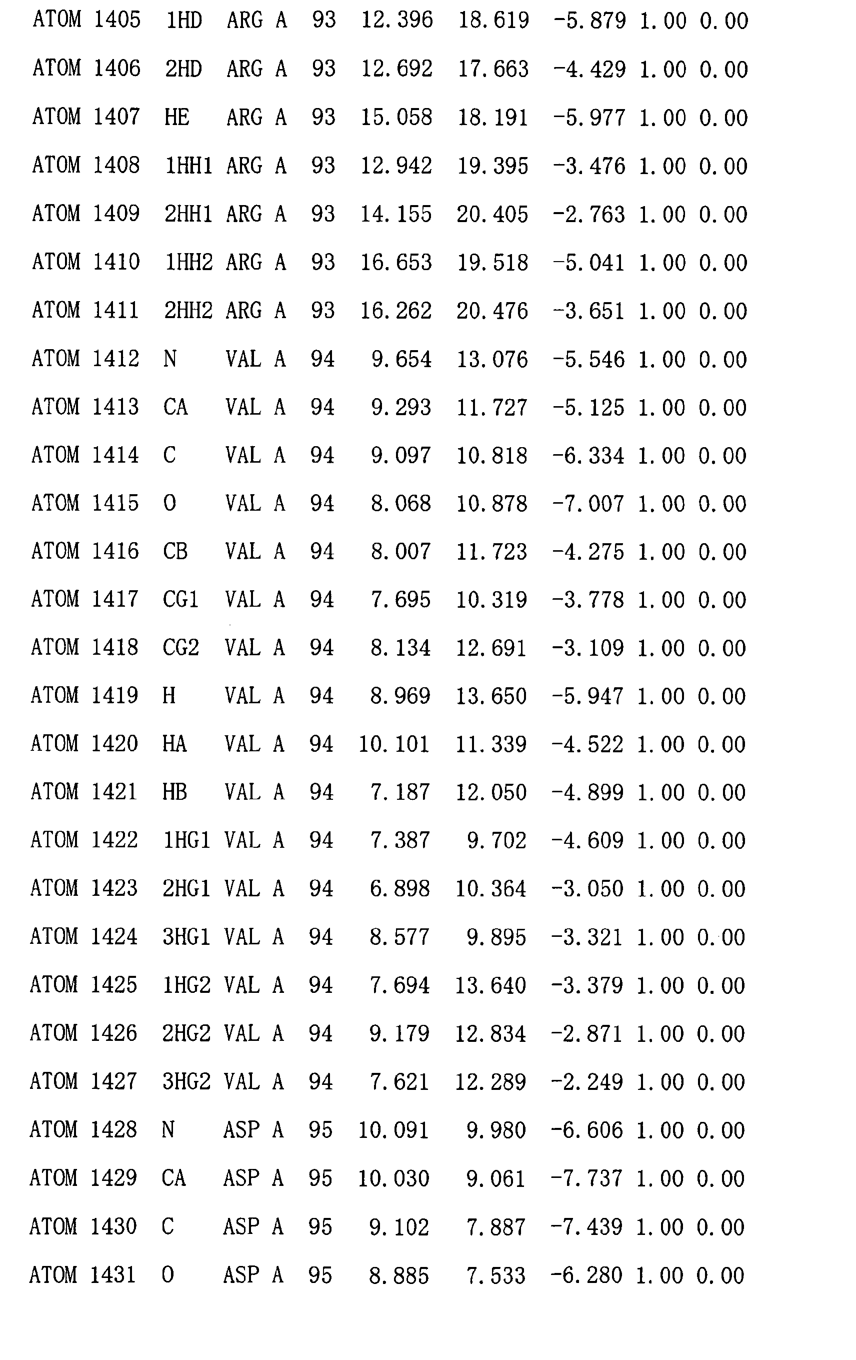

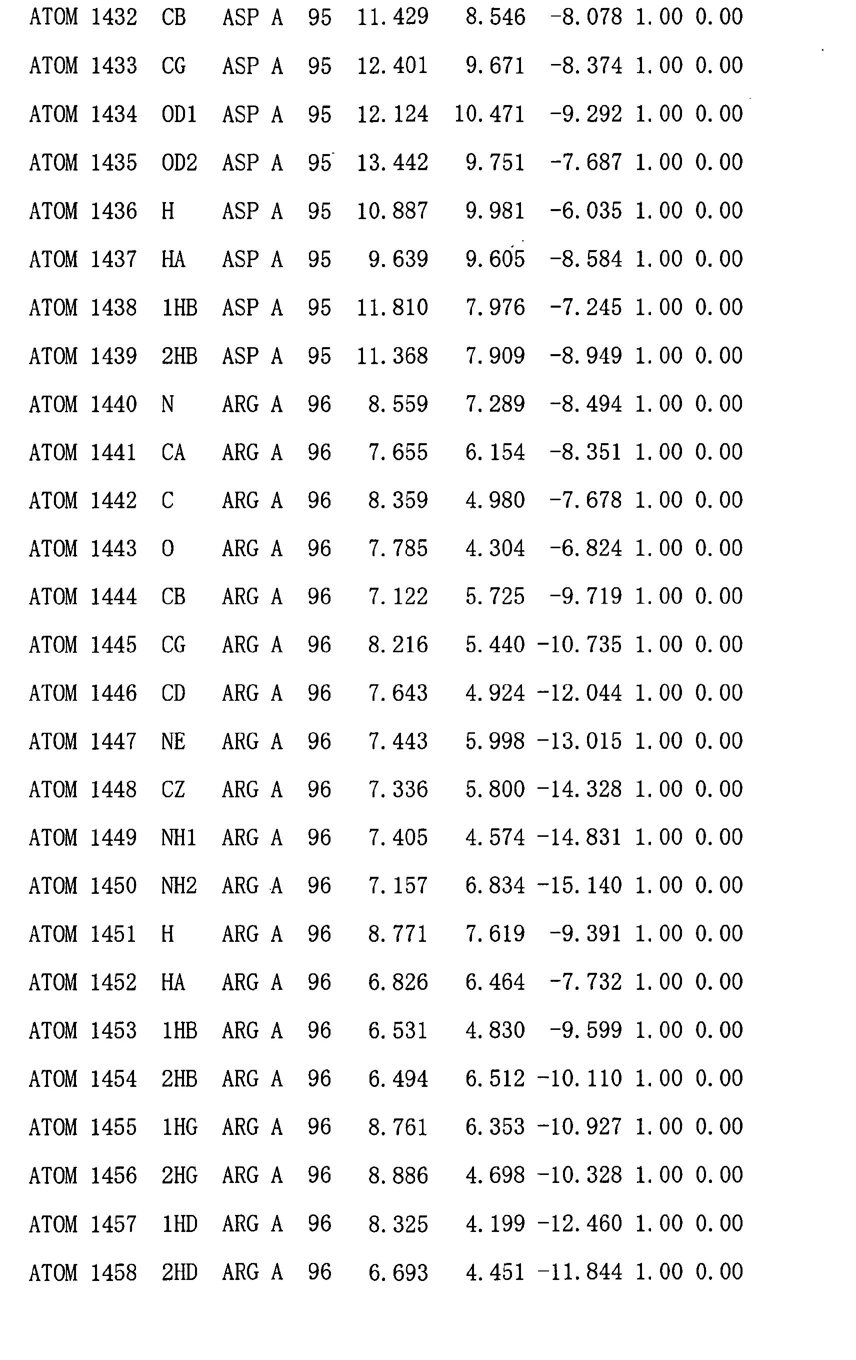

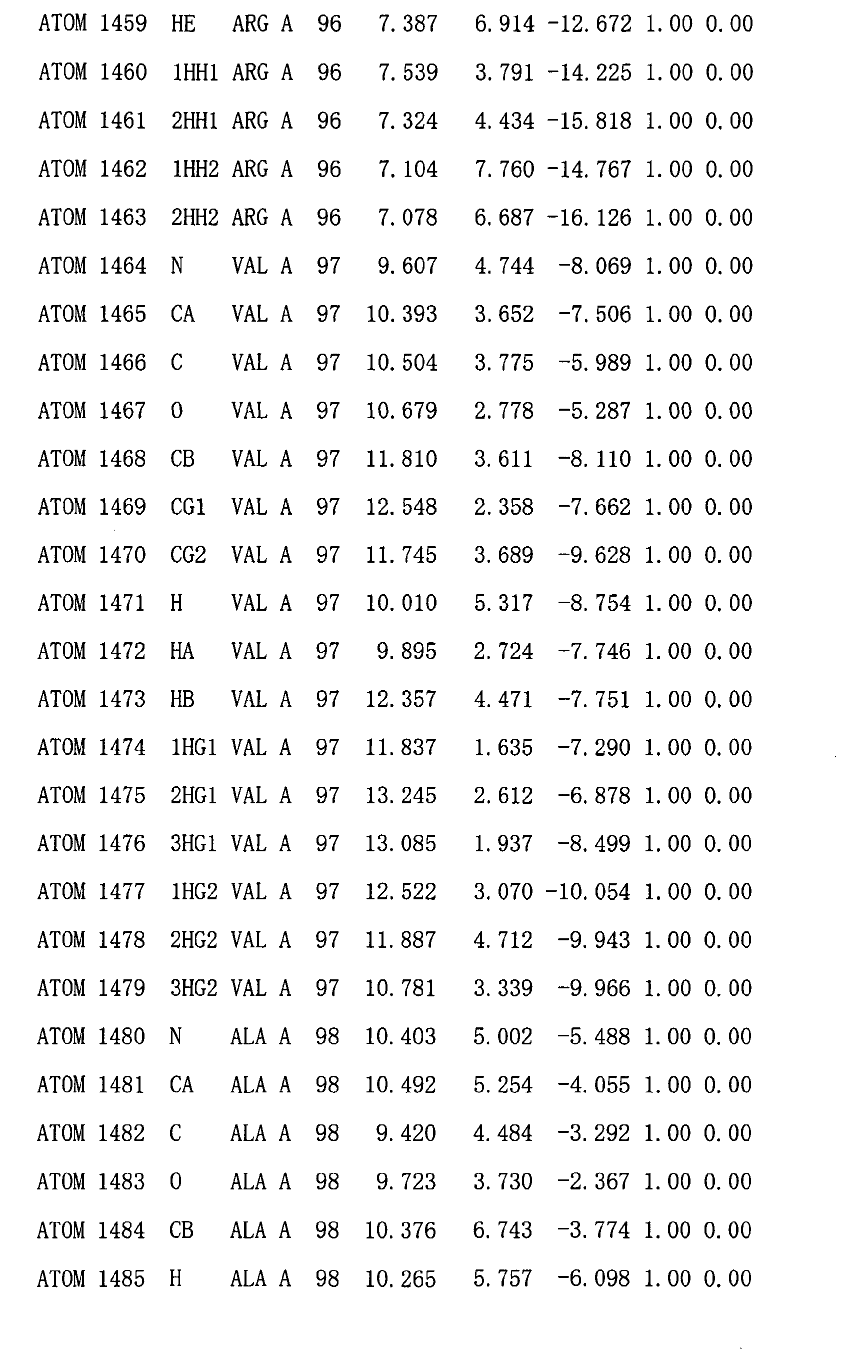

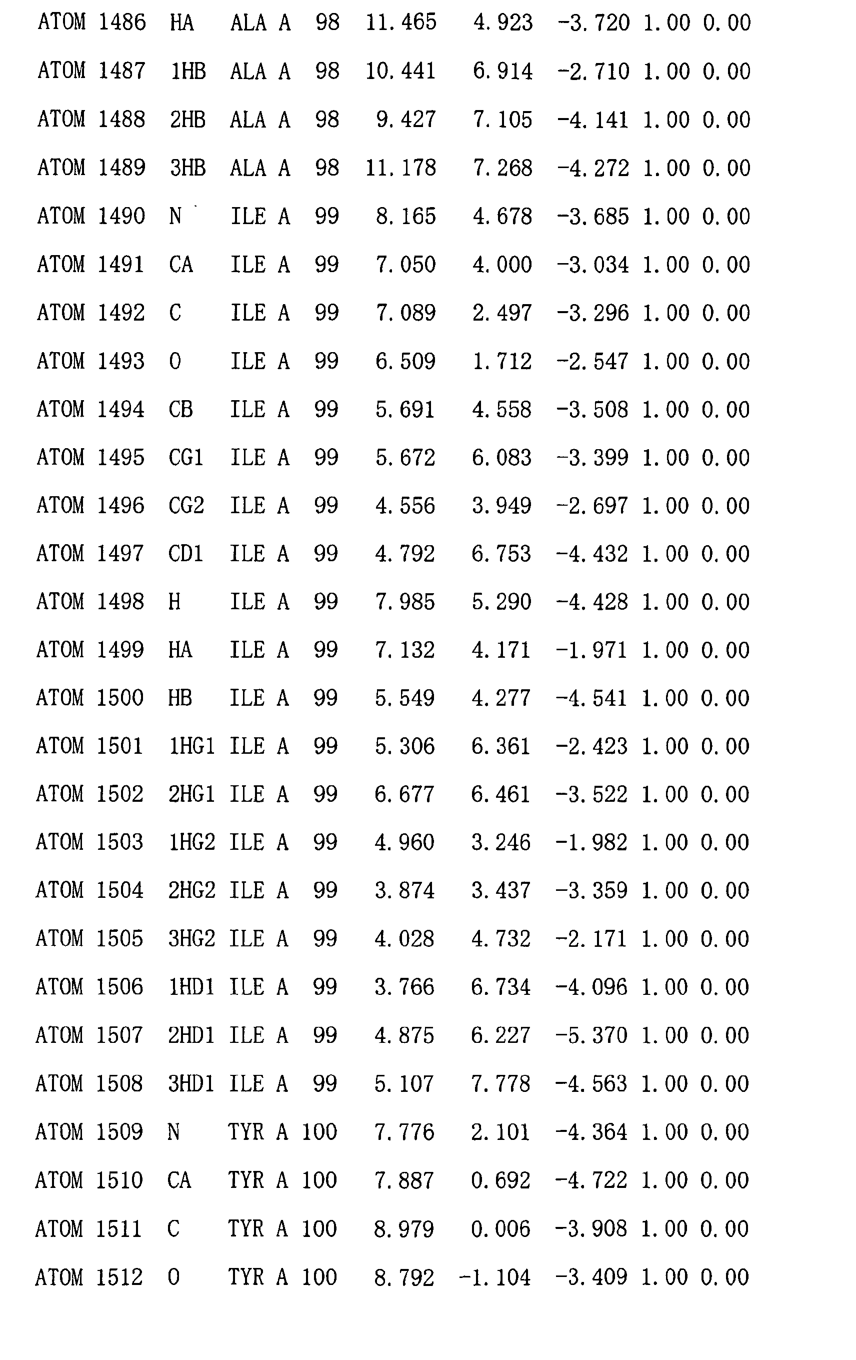

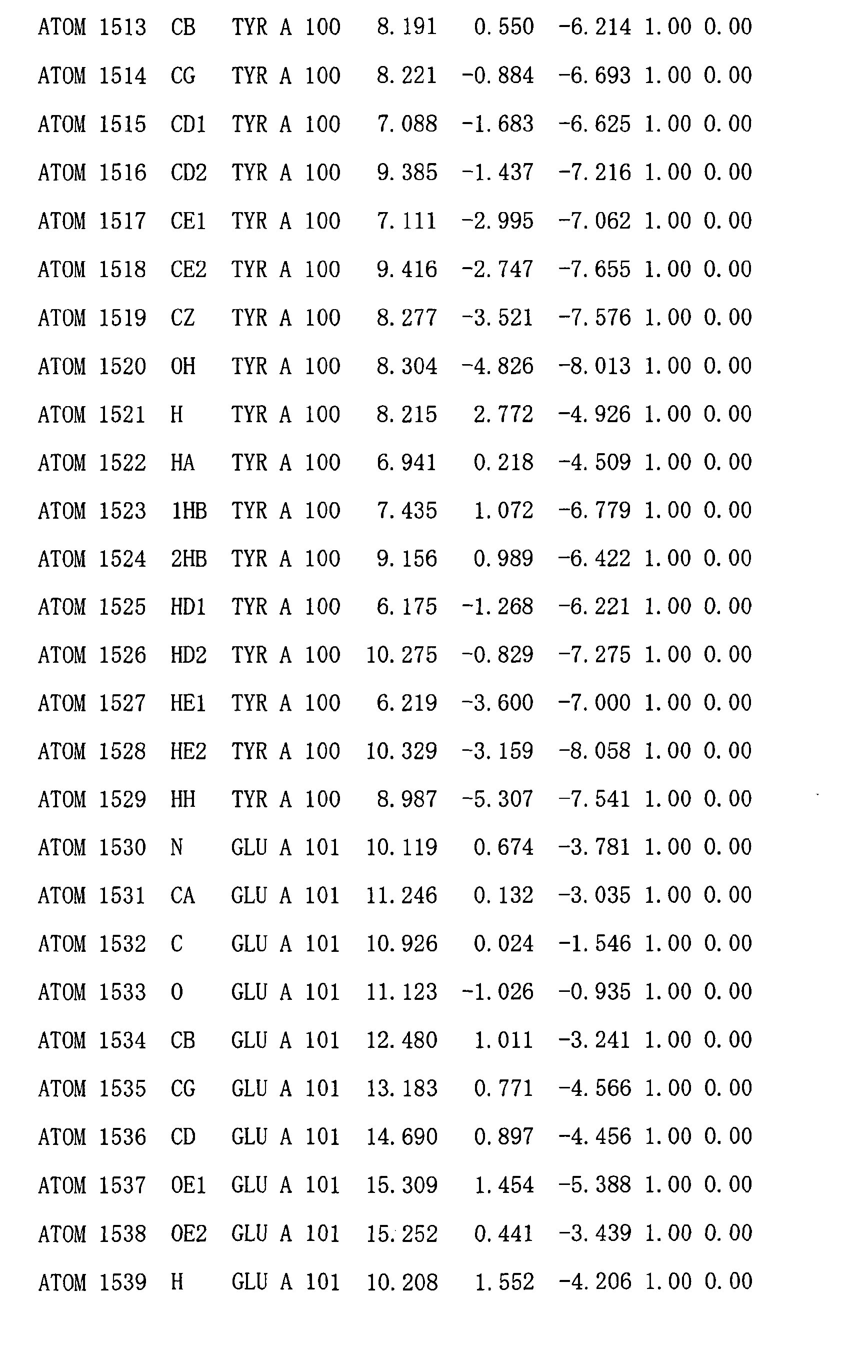

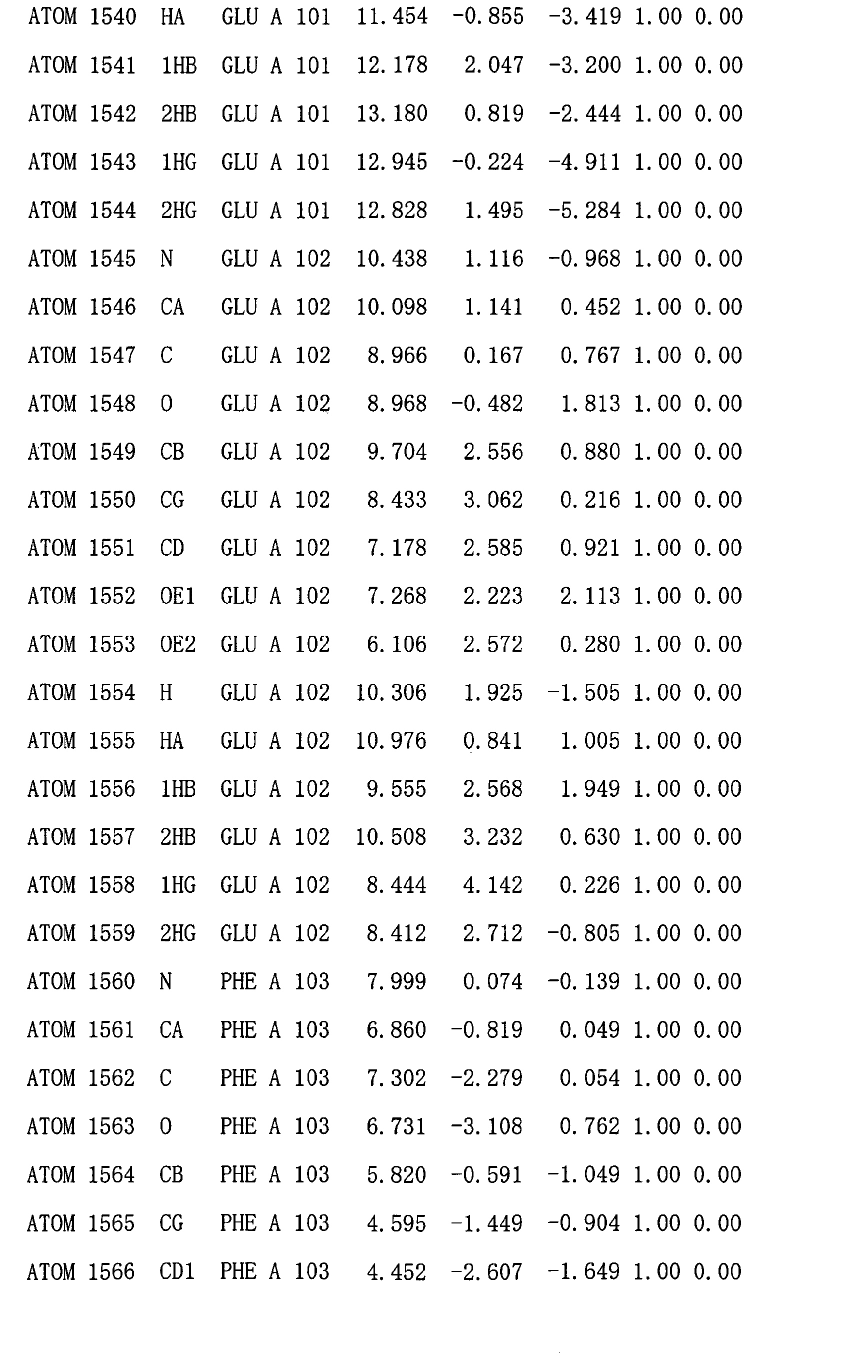

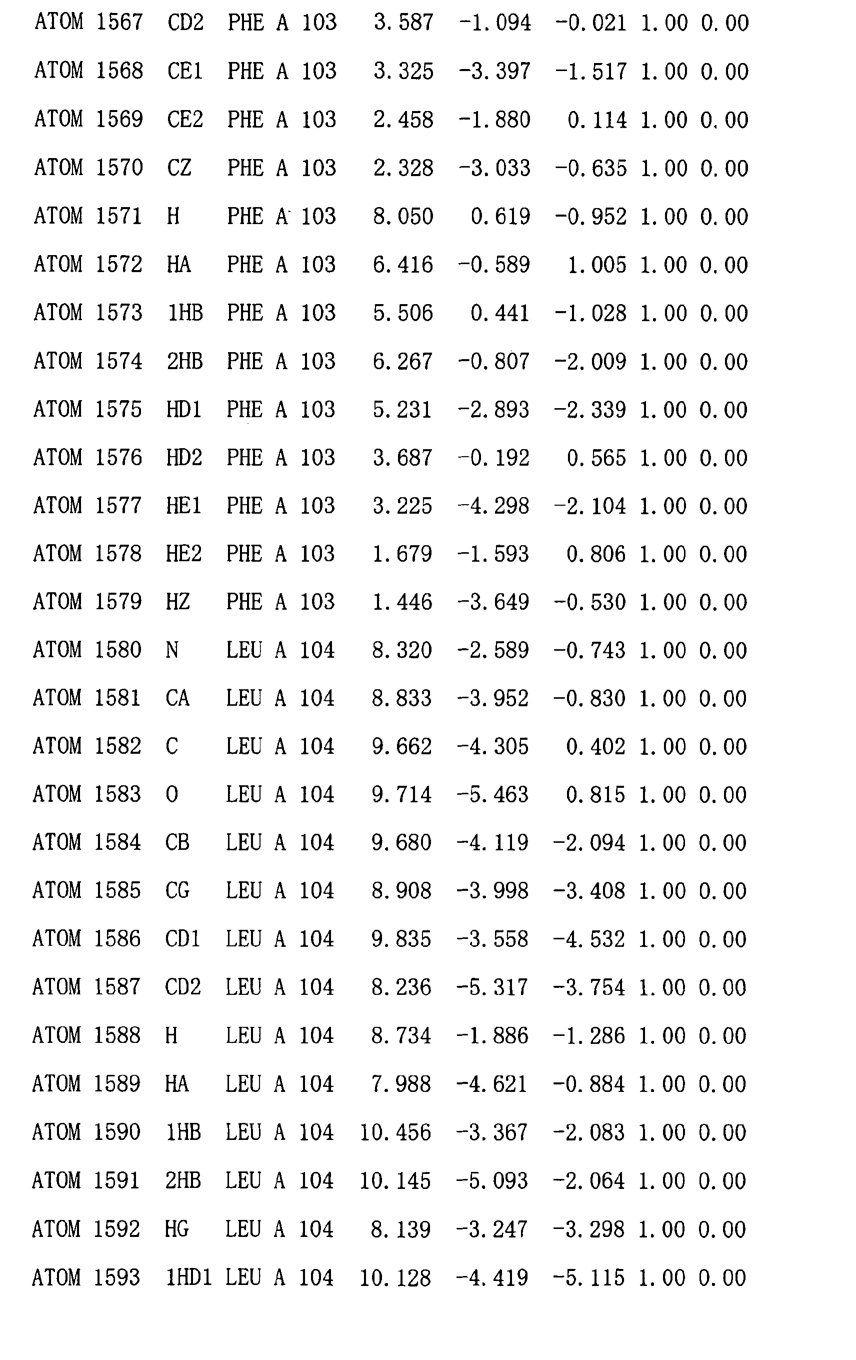

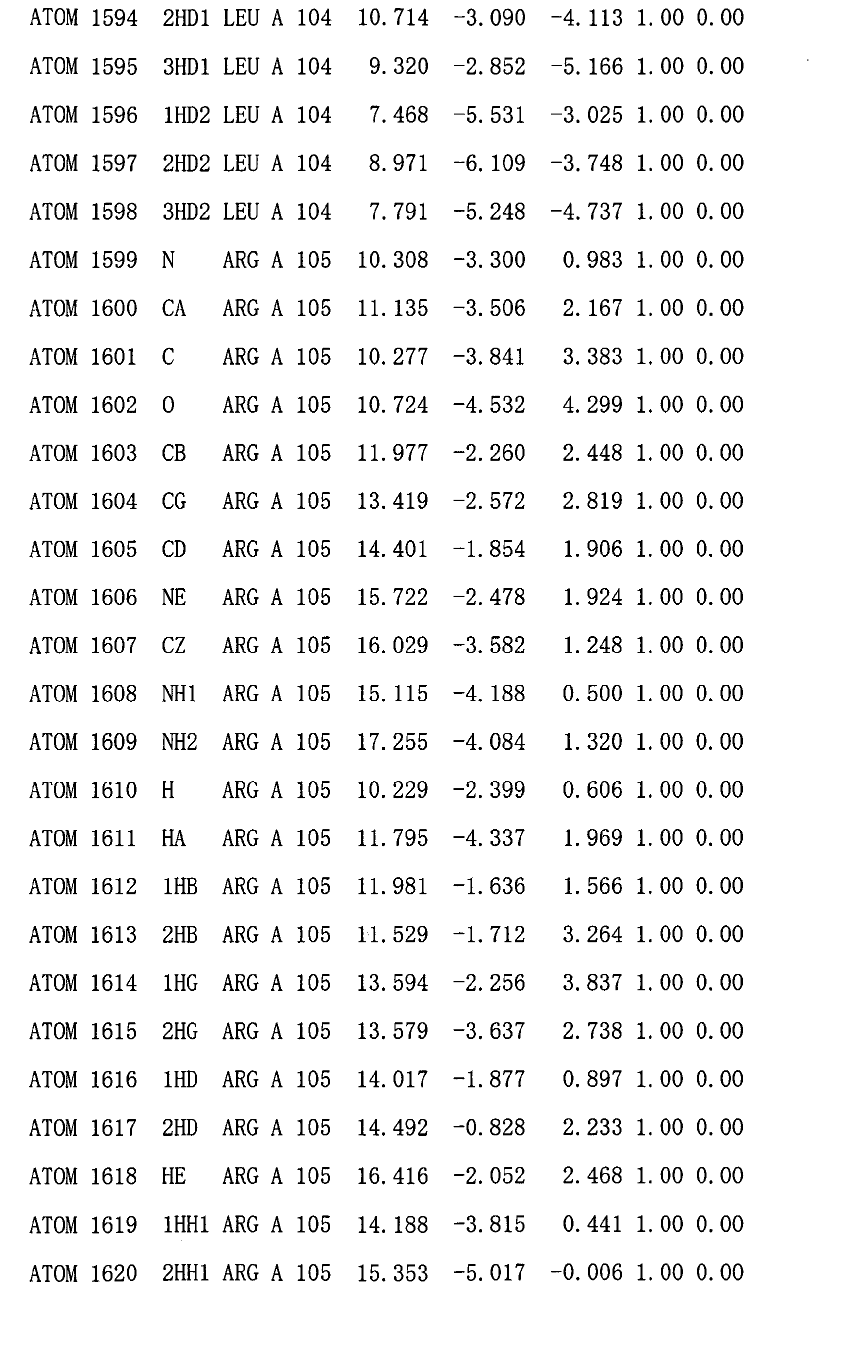

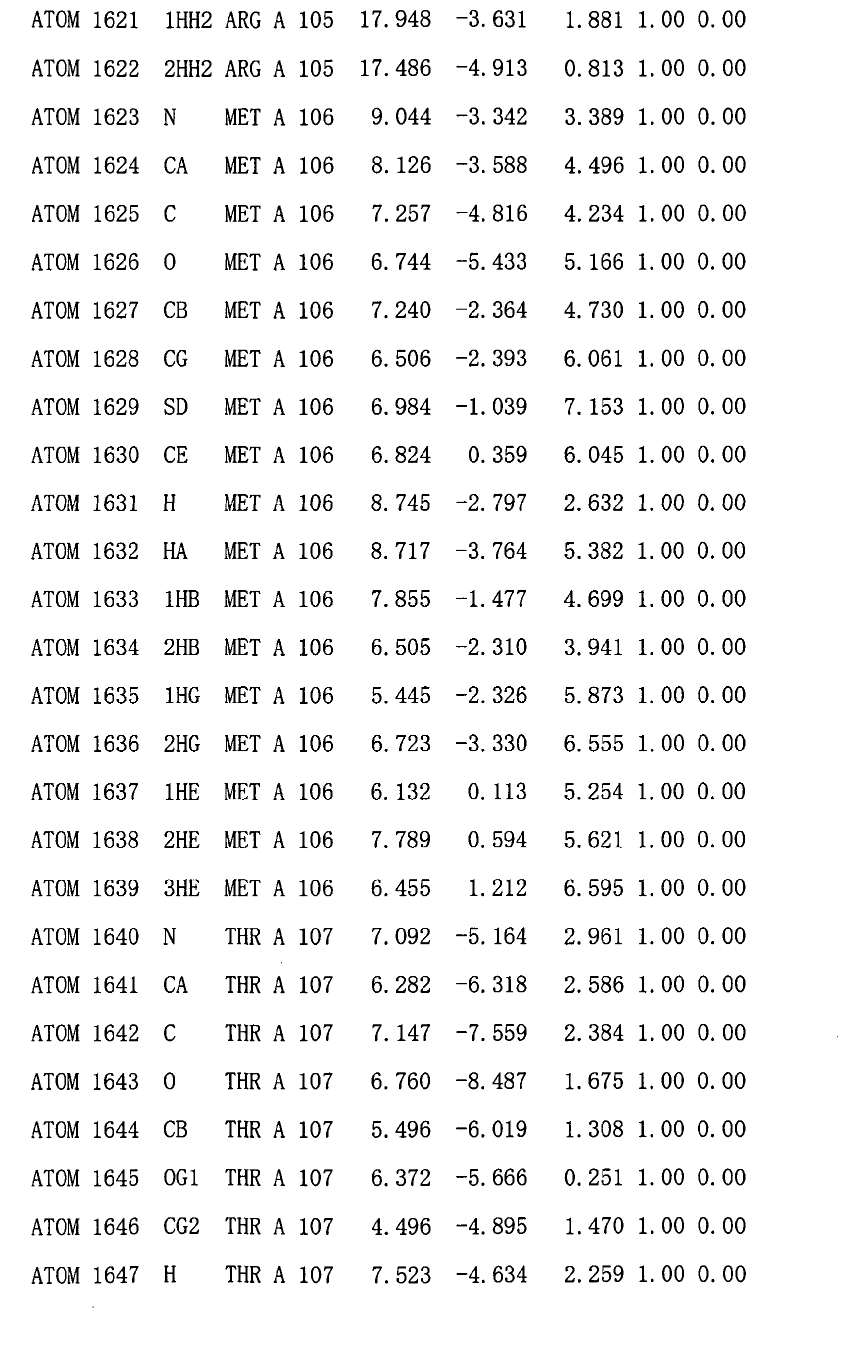

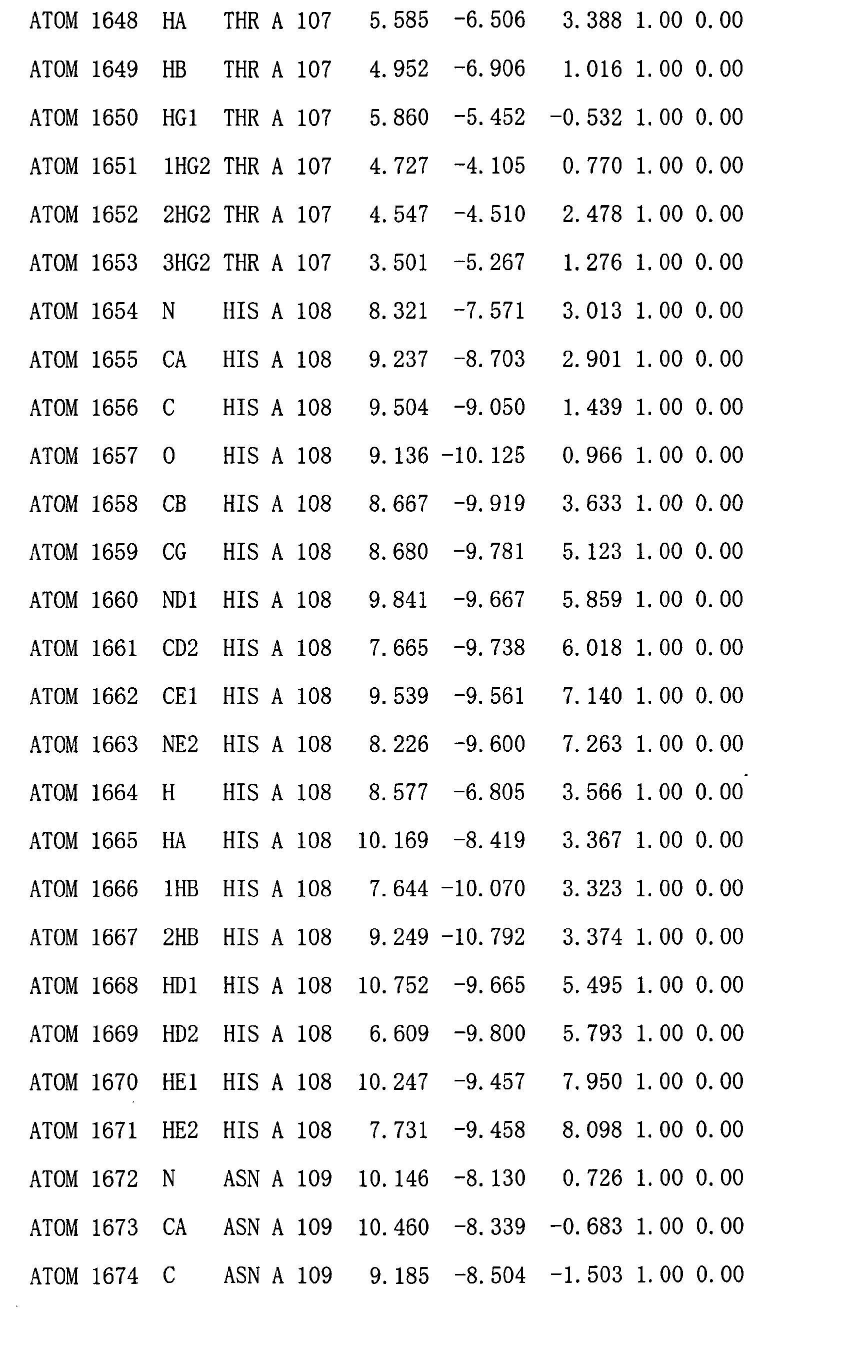

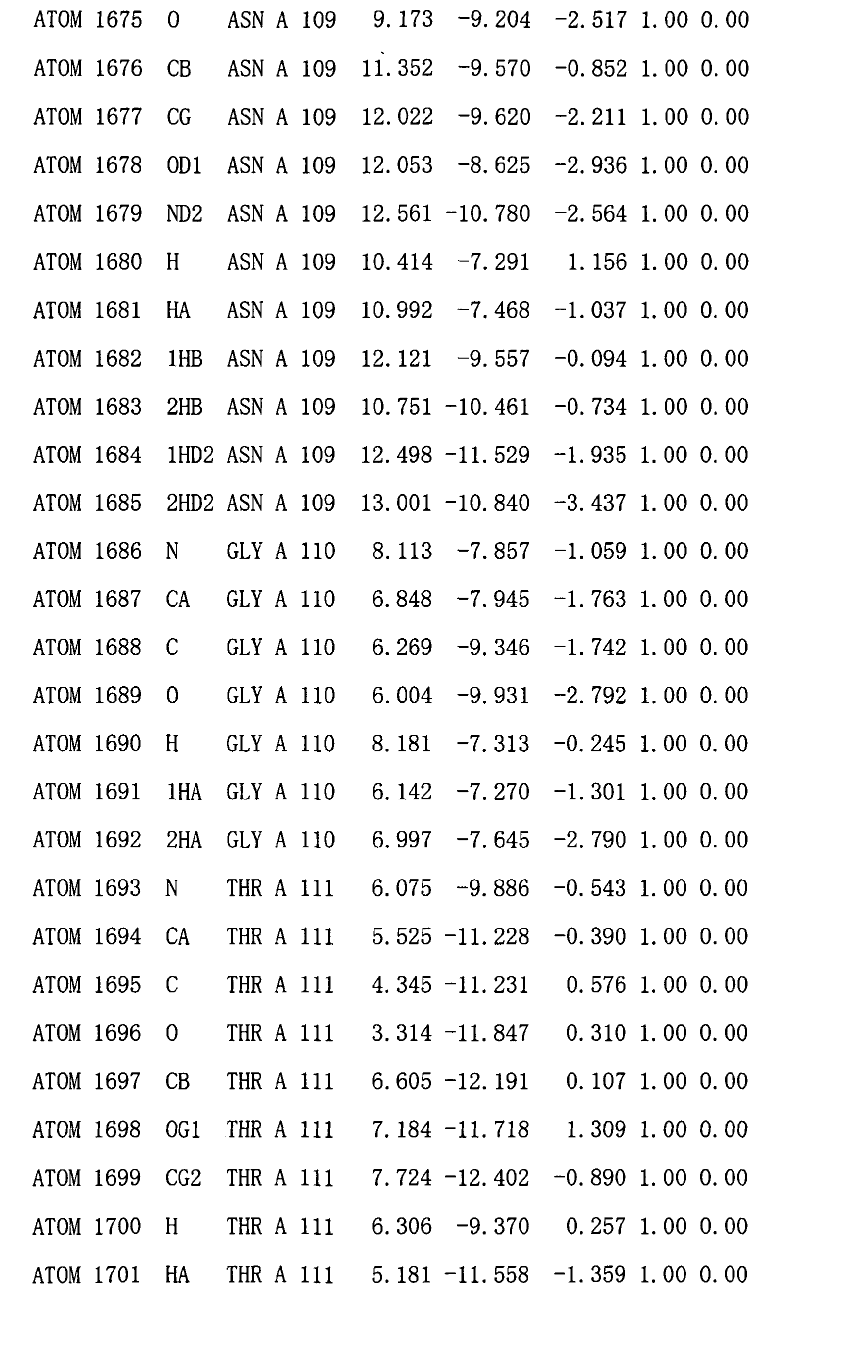

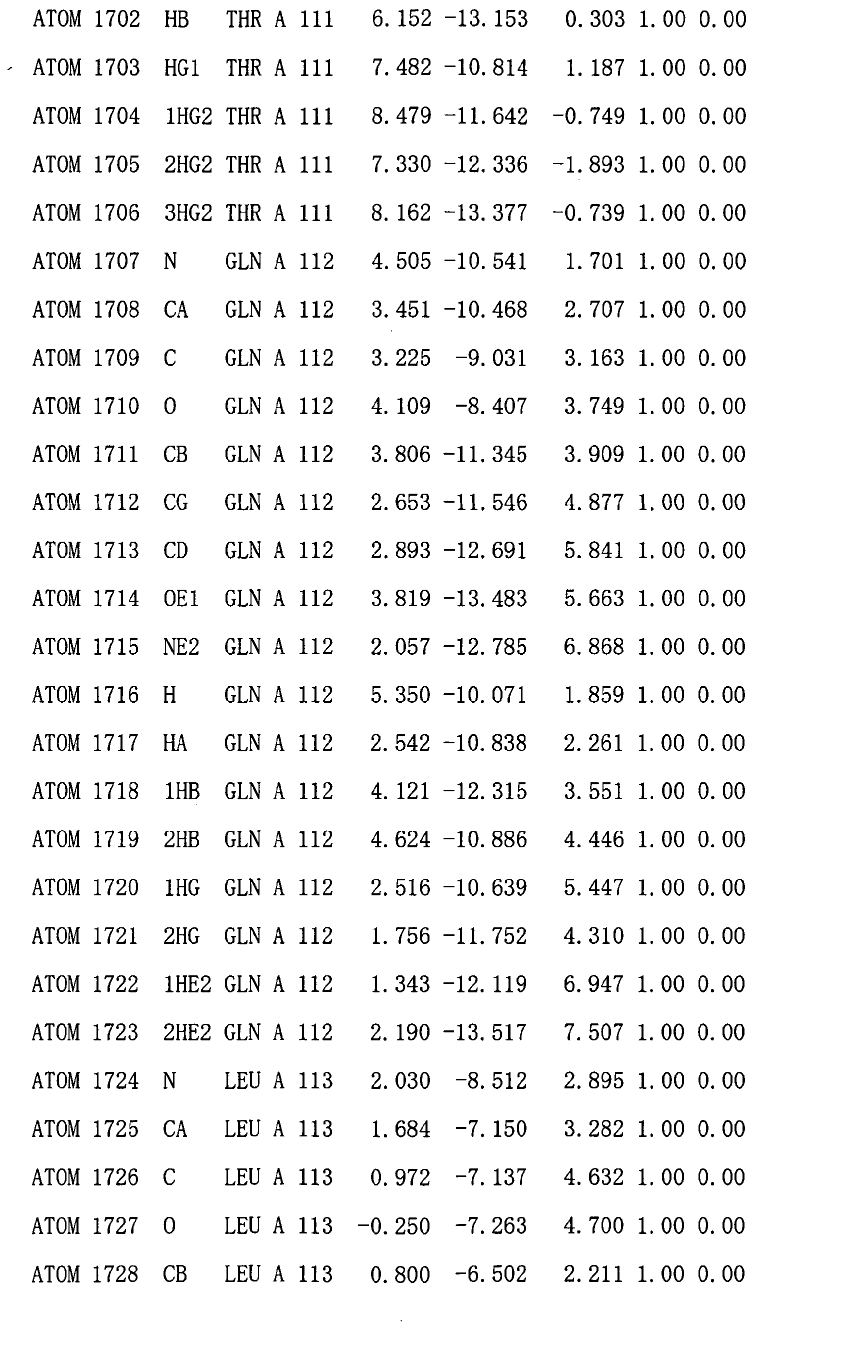

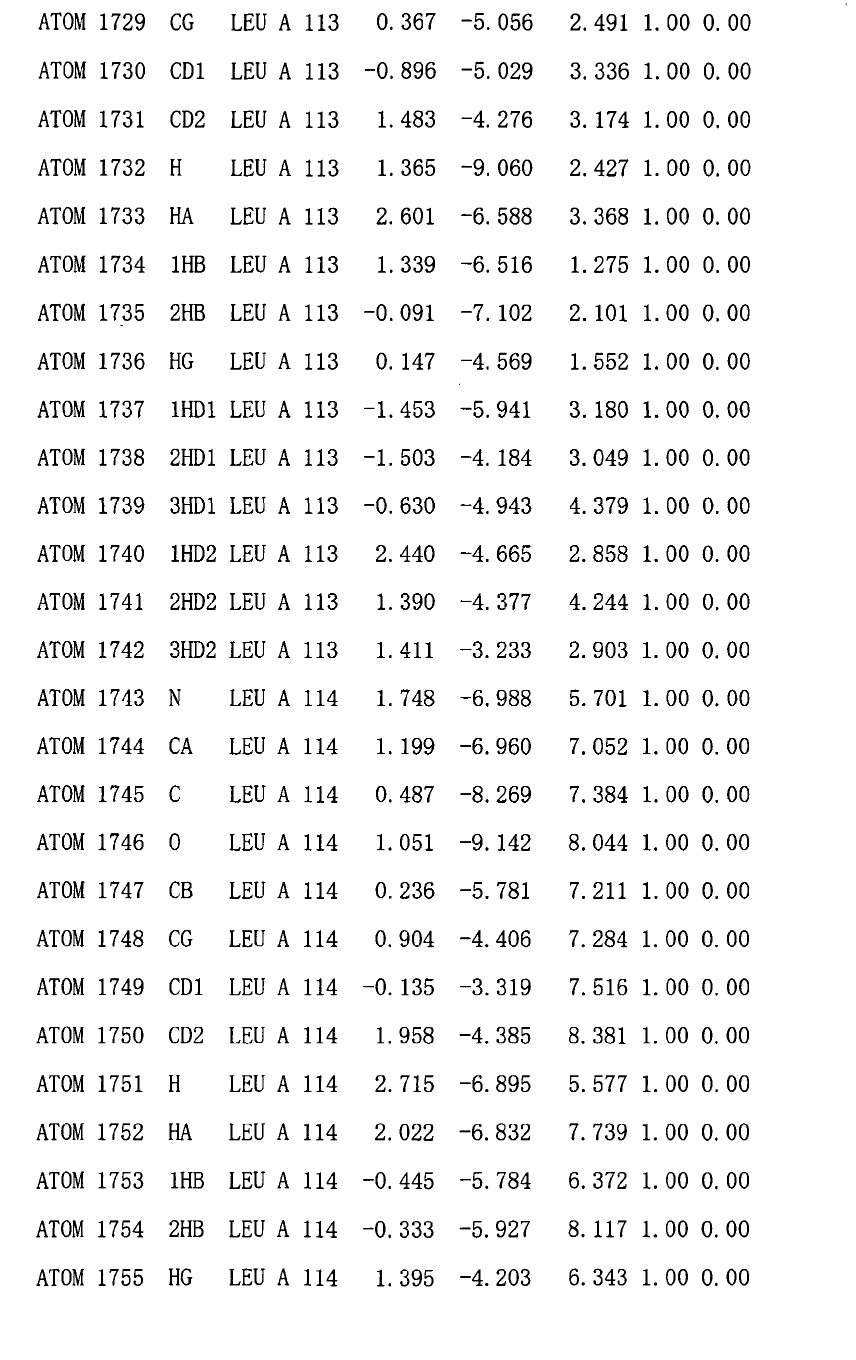

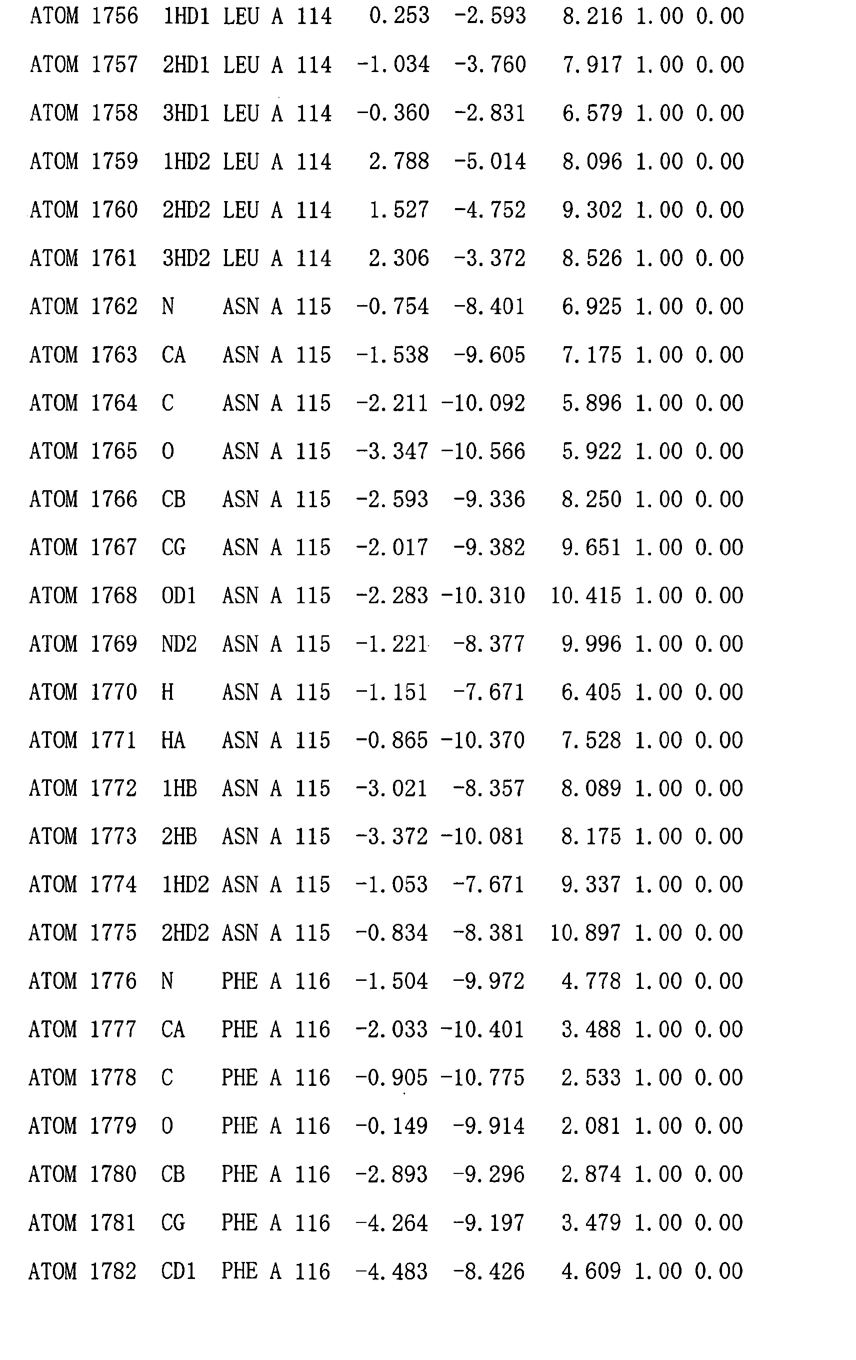

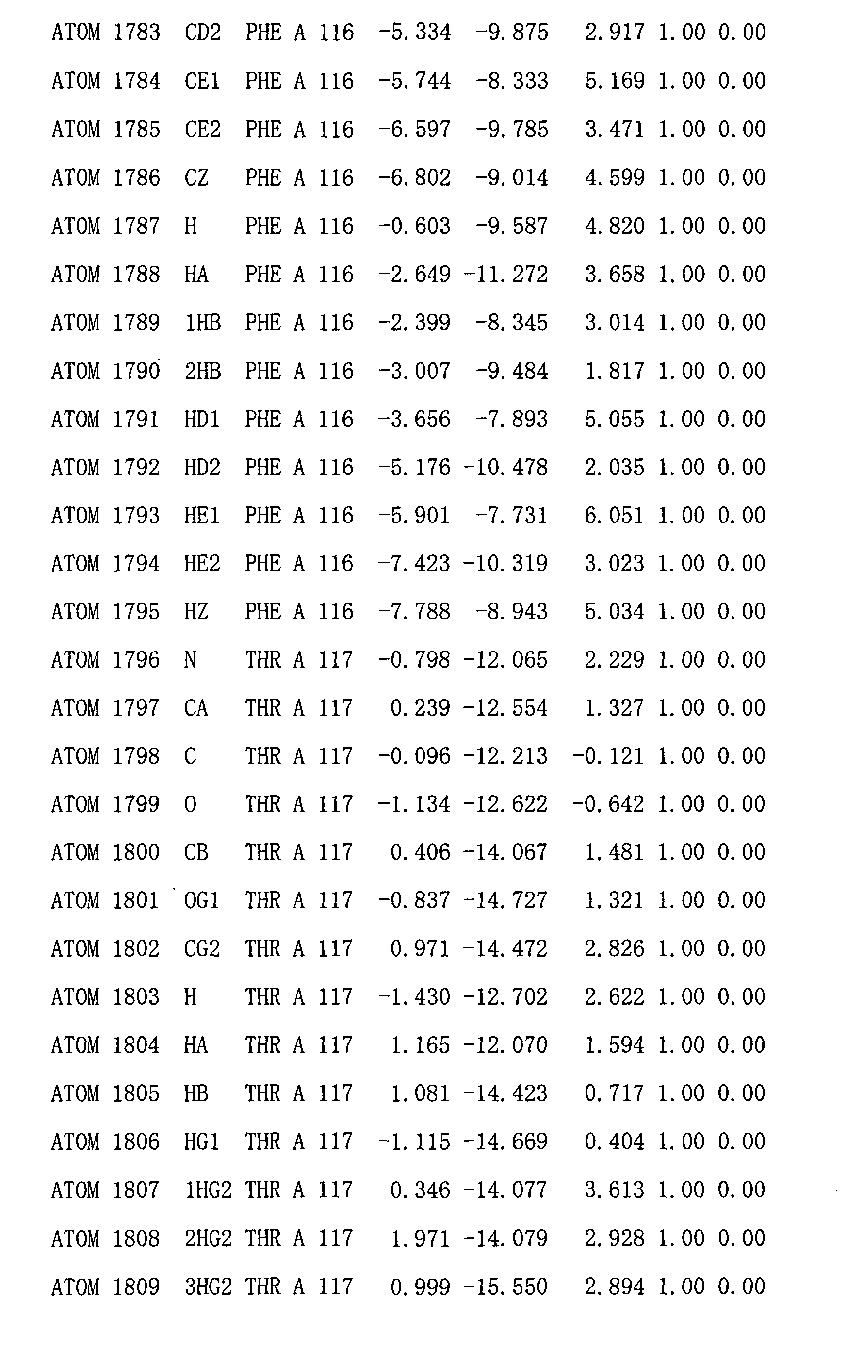

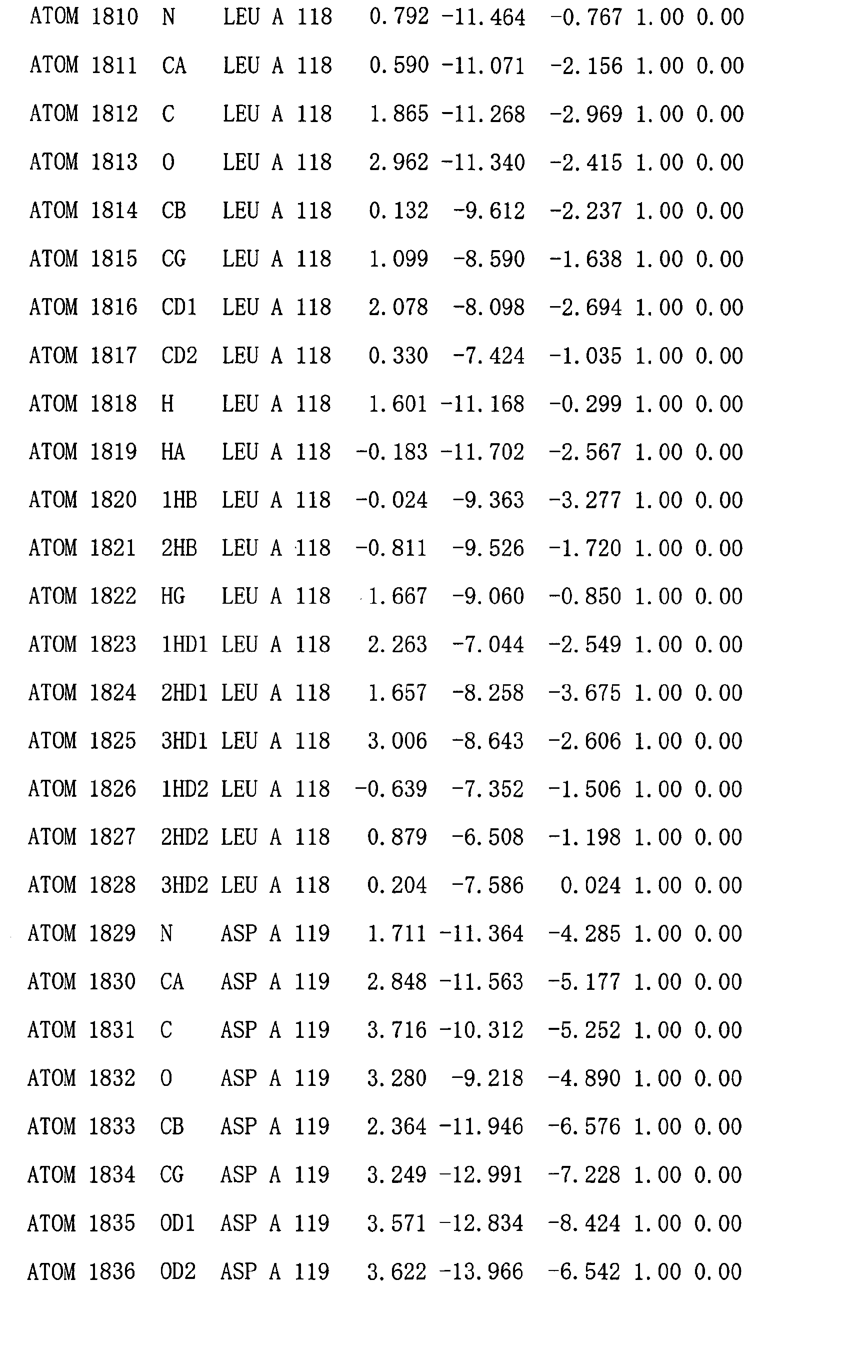

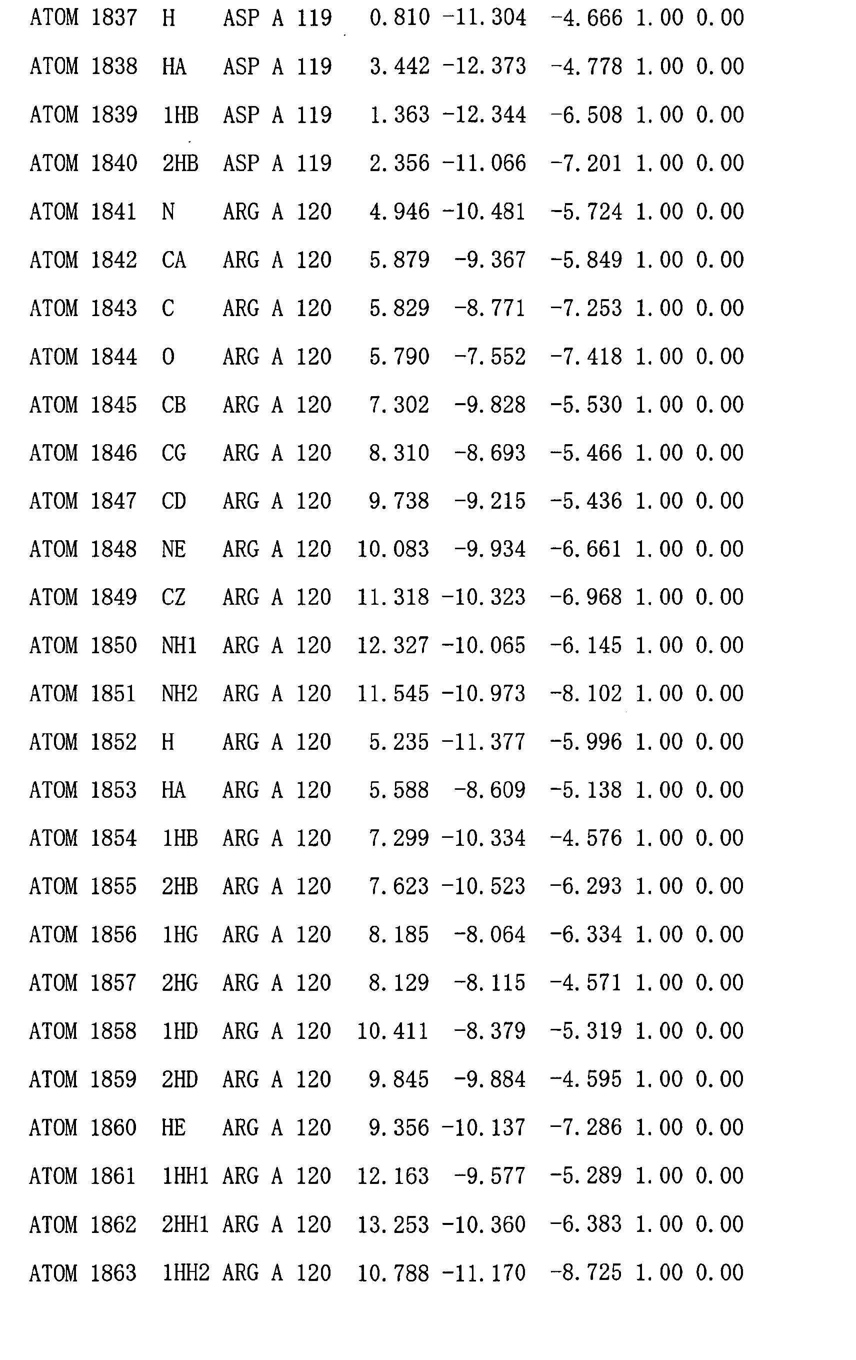

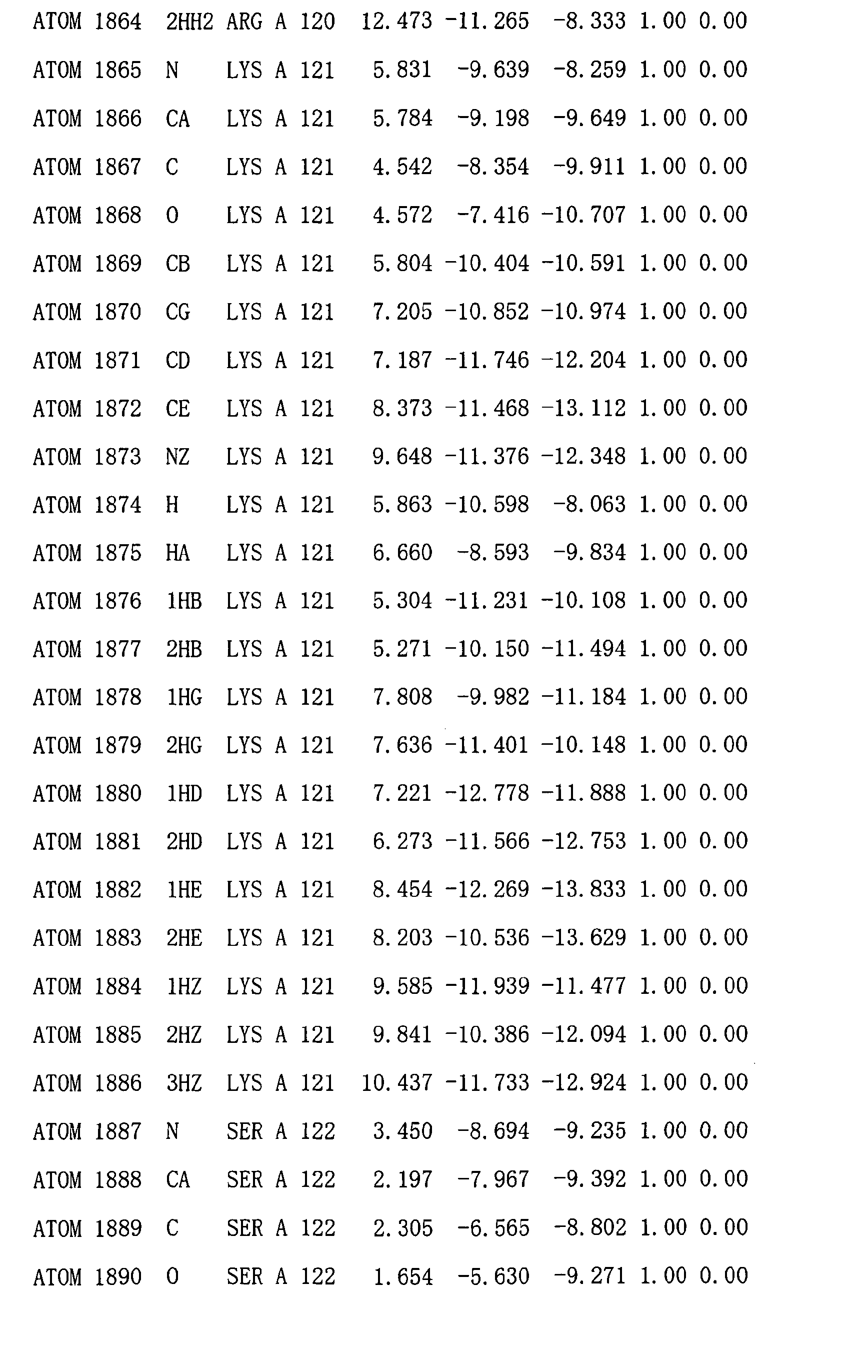

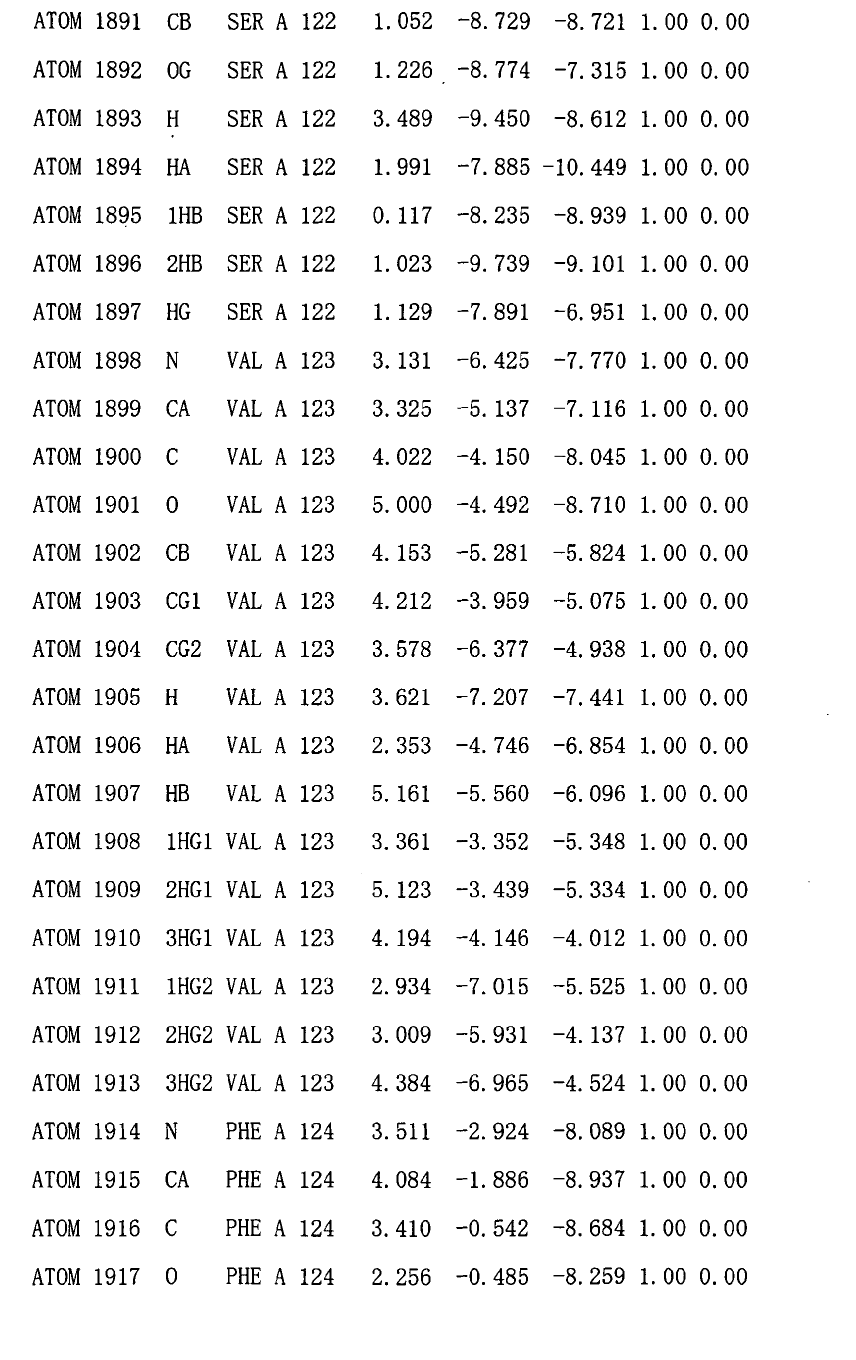

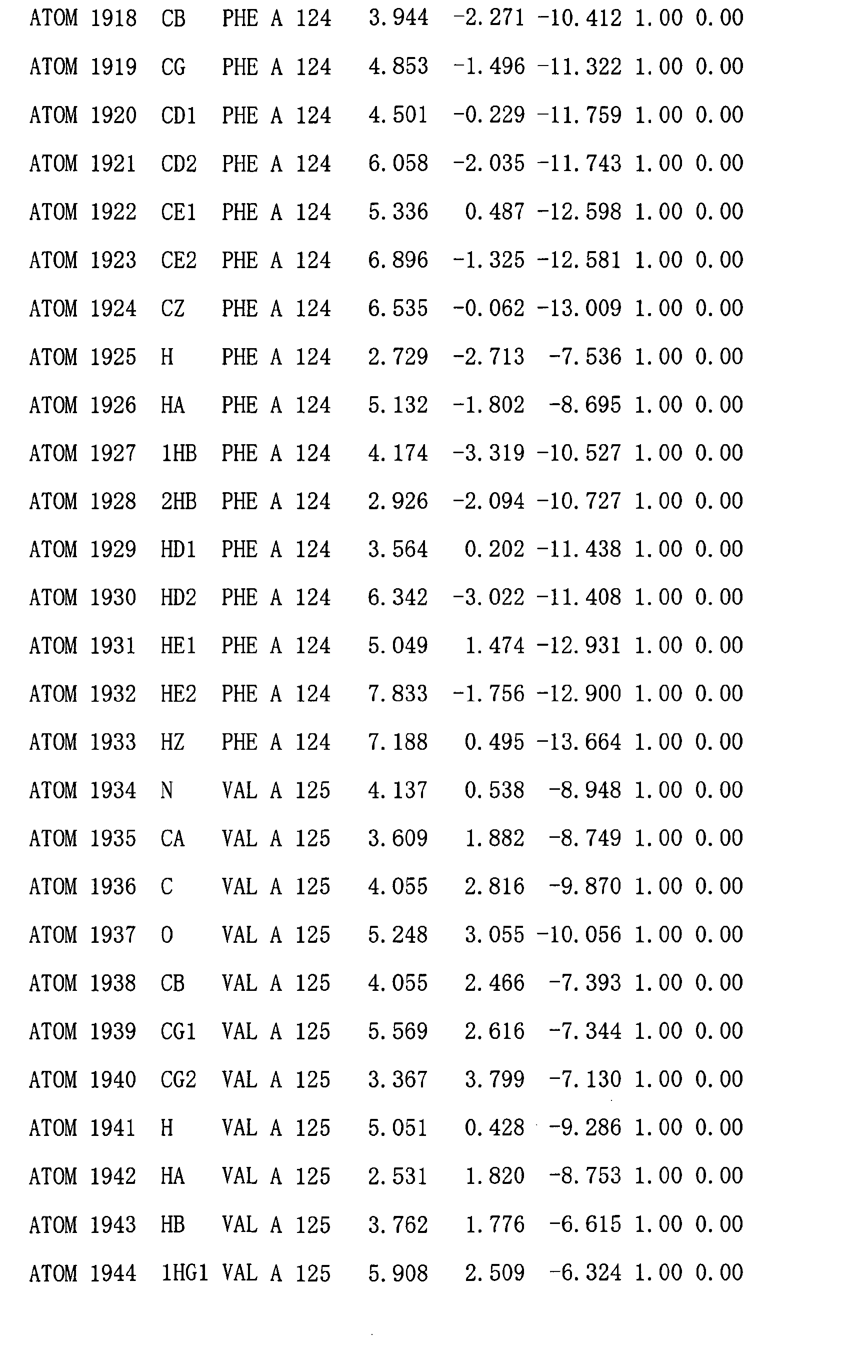

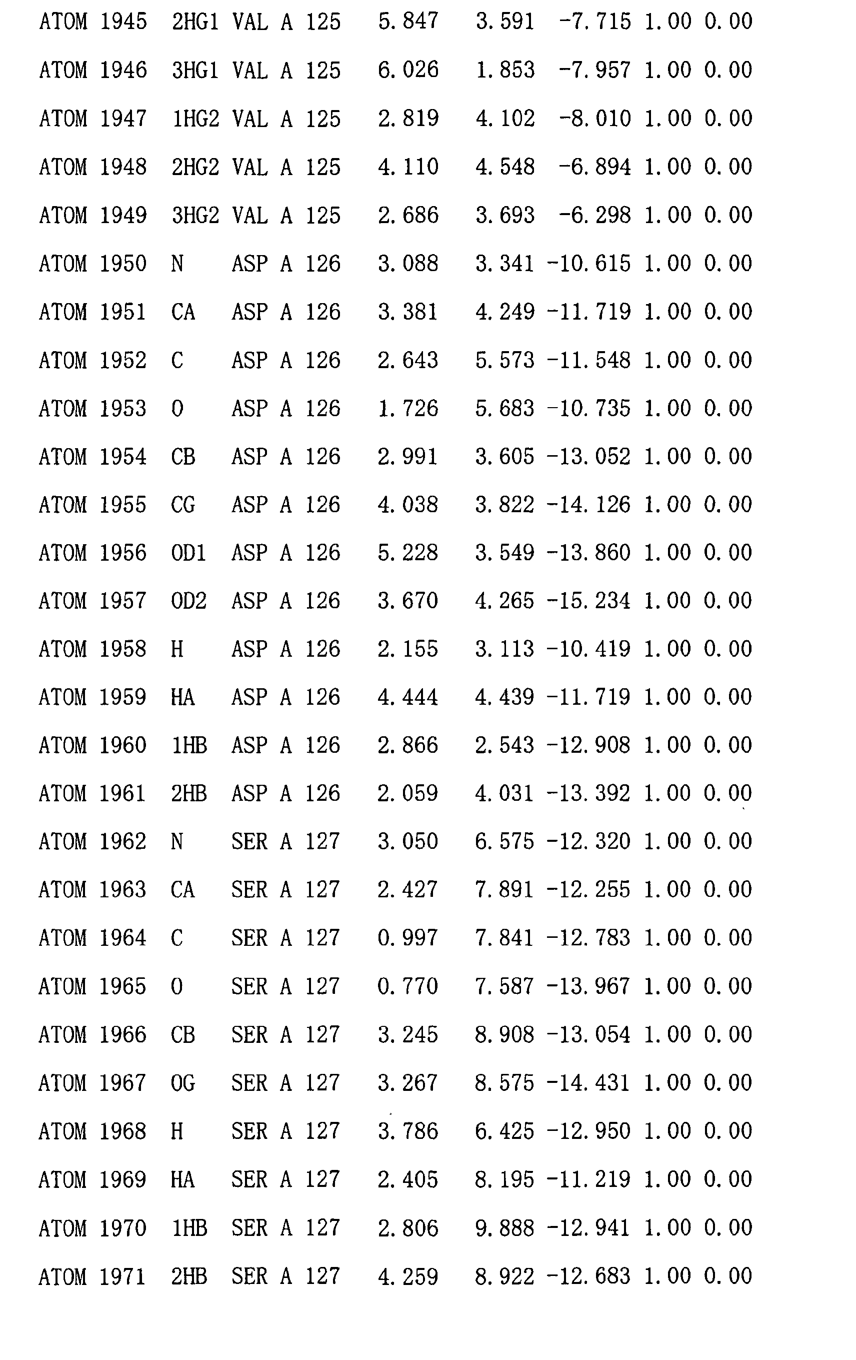

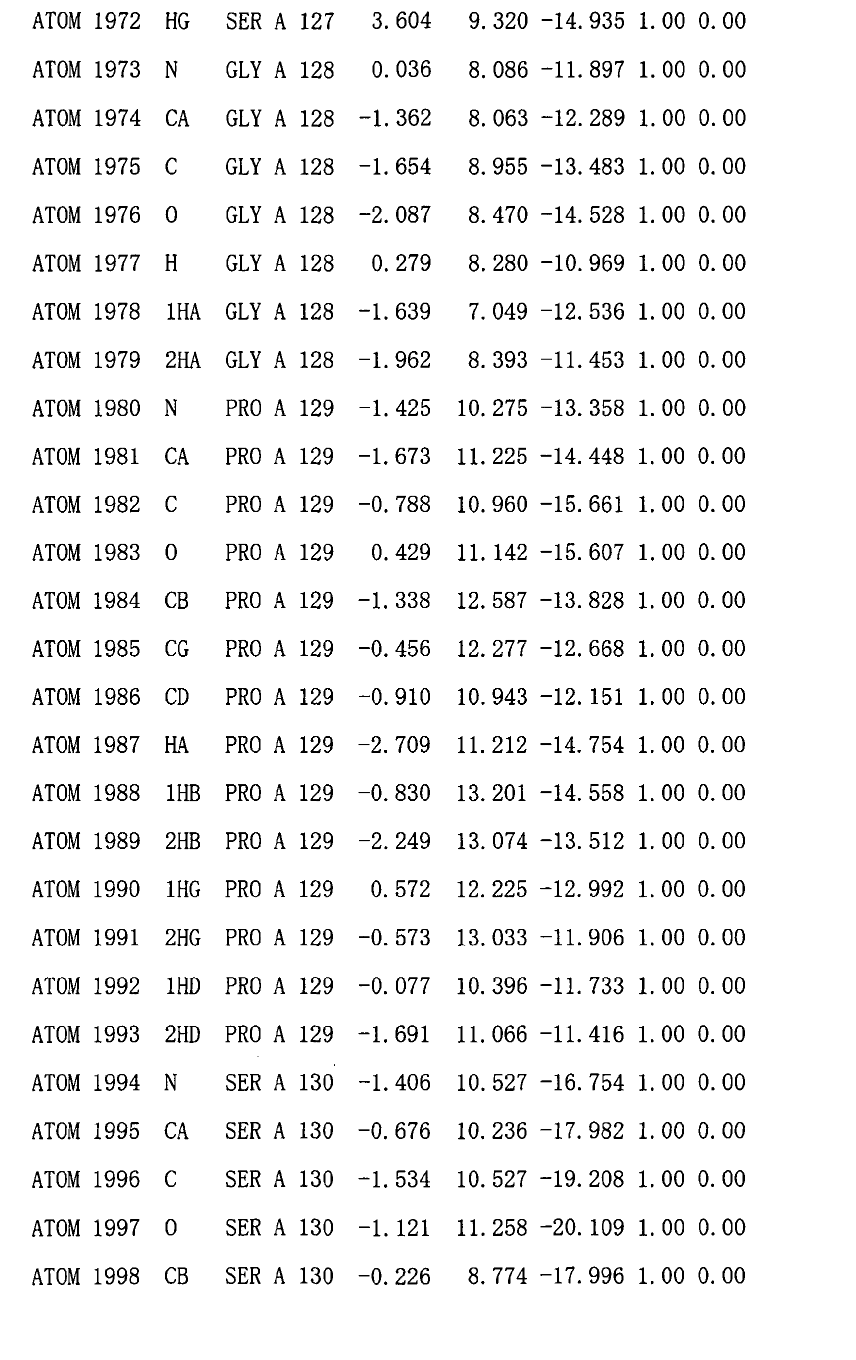

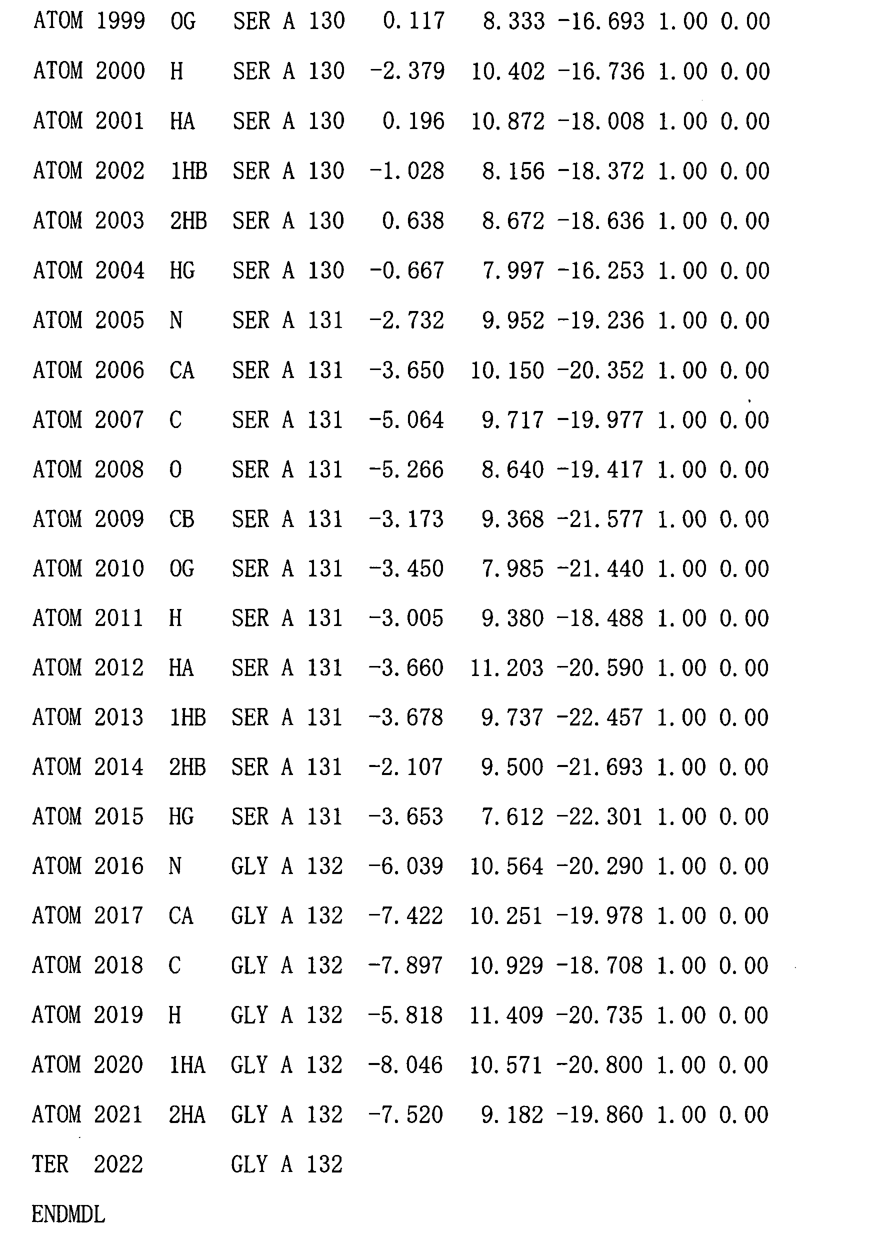

### Three-Dimensional Structure Coordinate 12

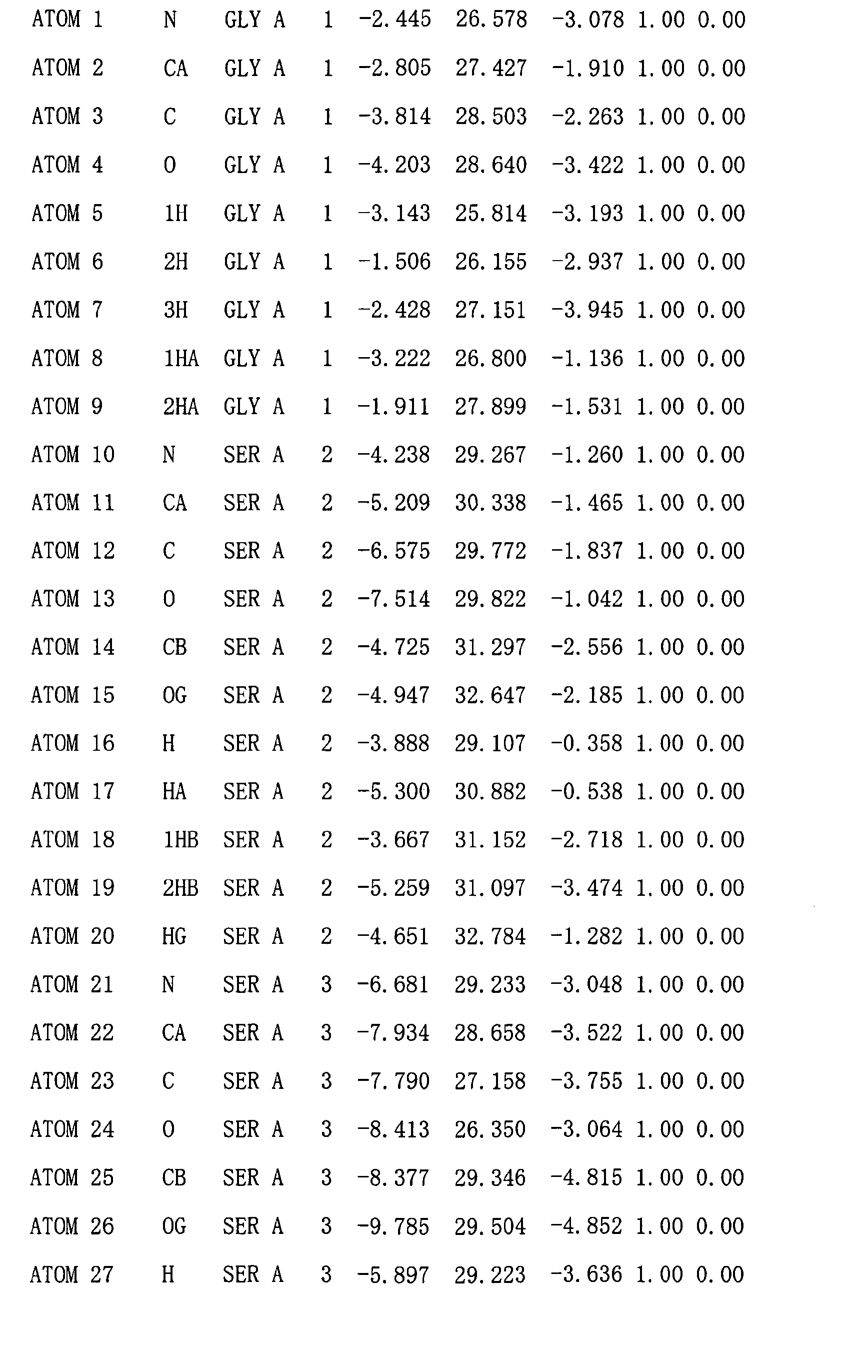

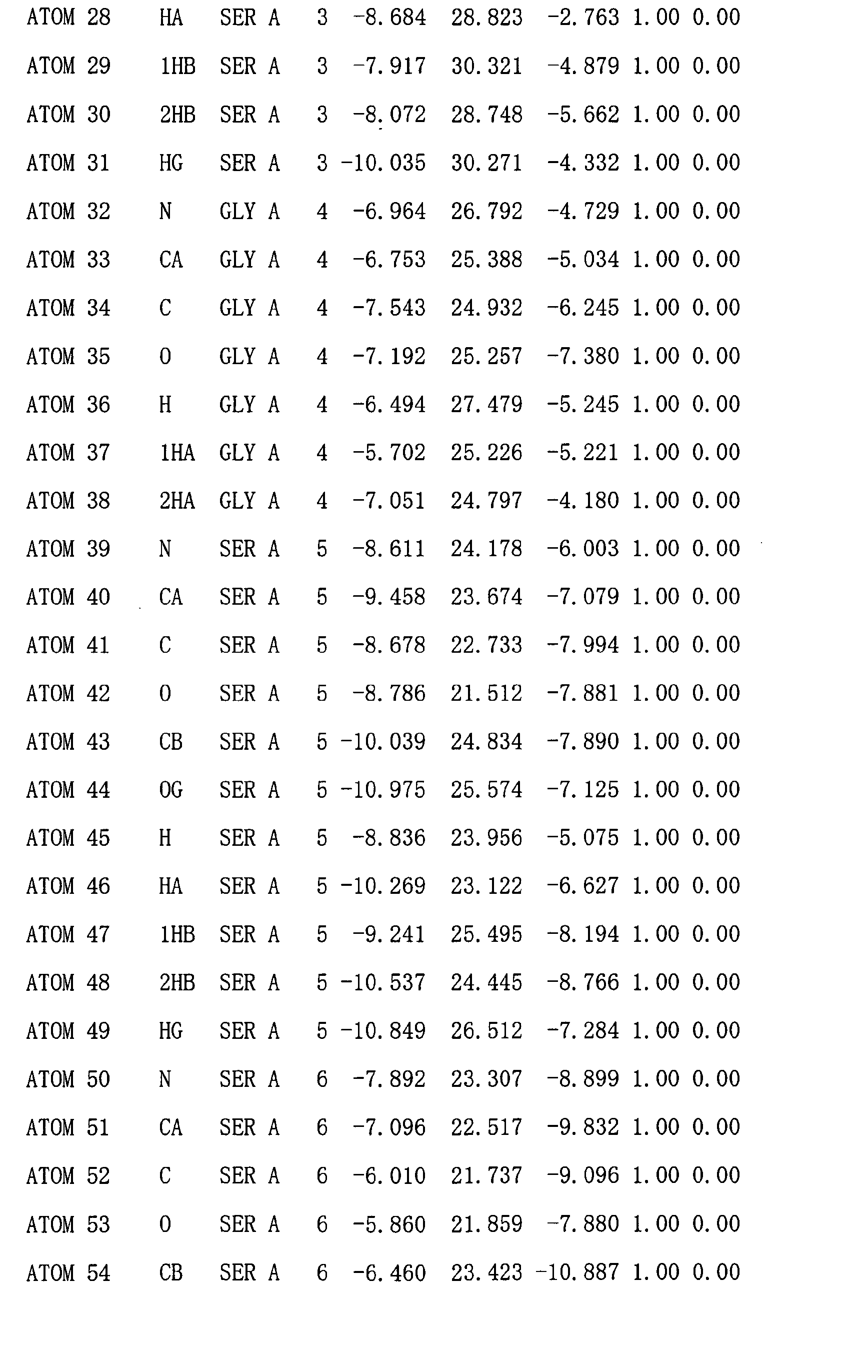

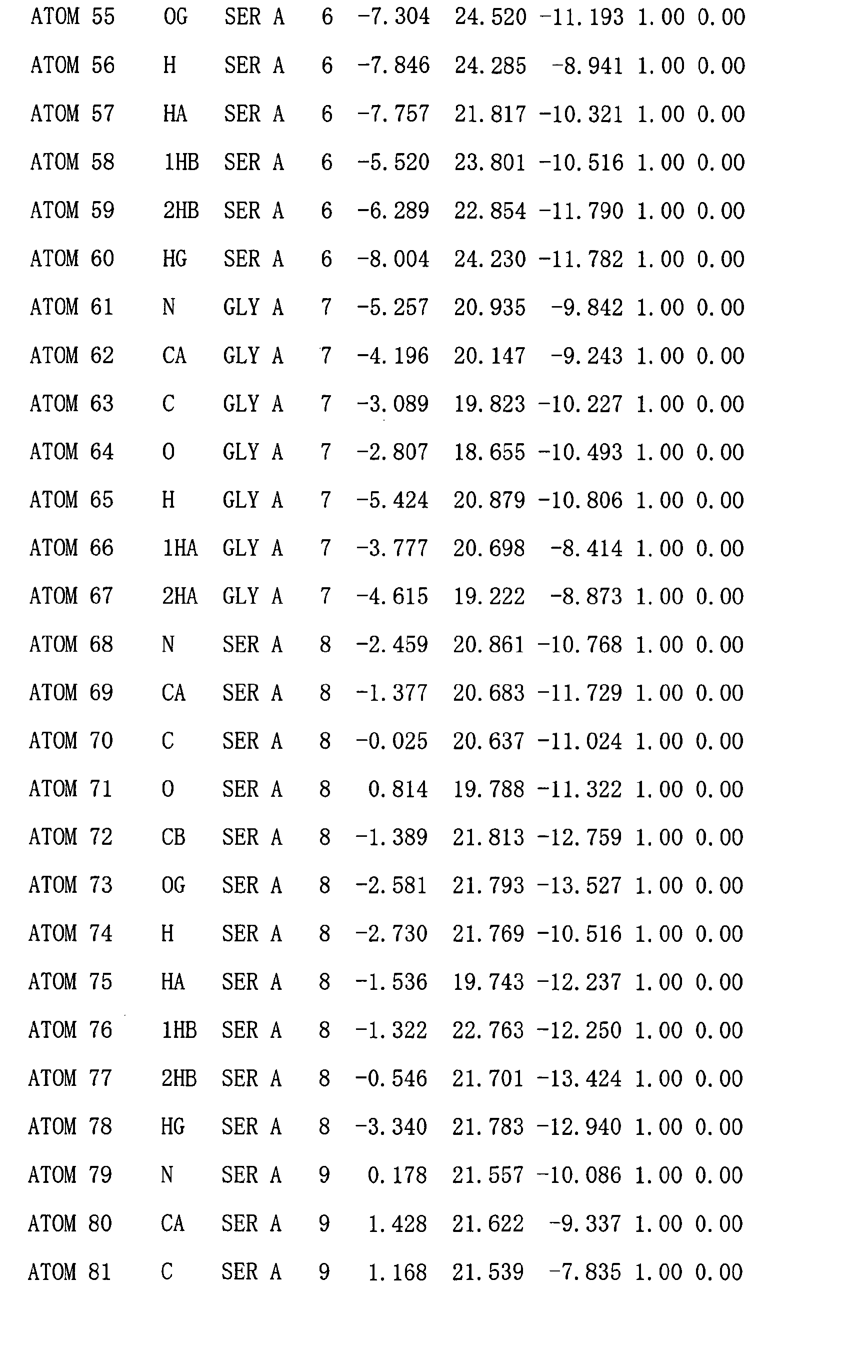

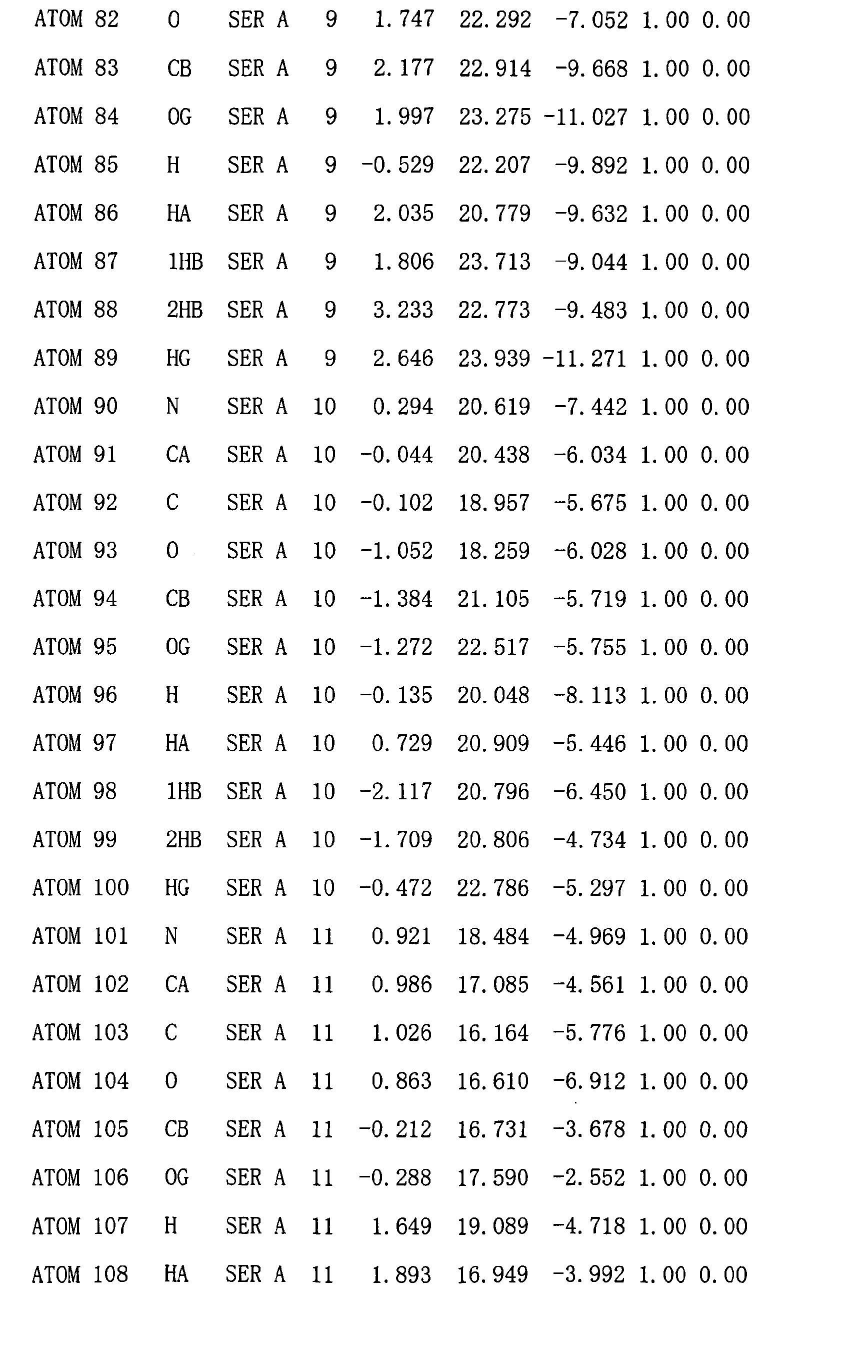

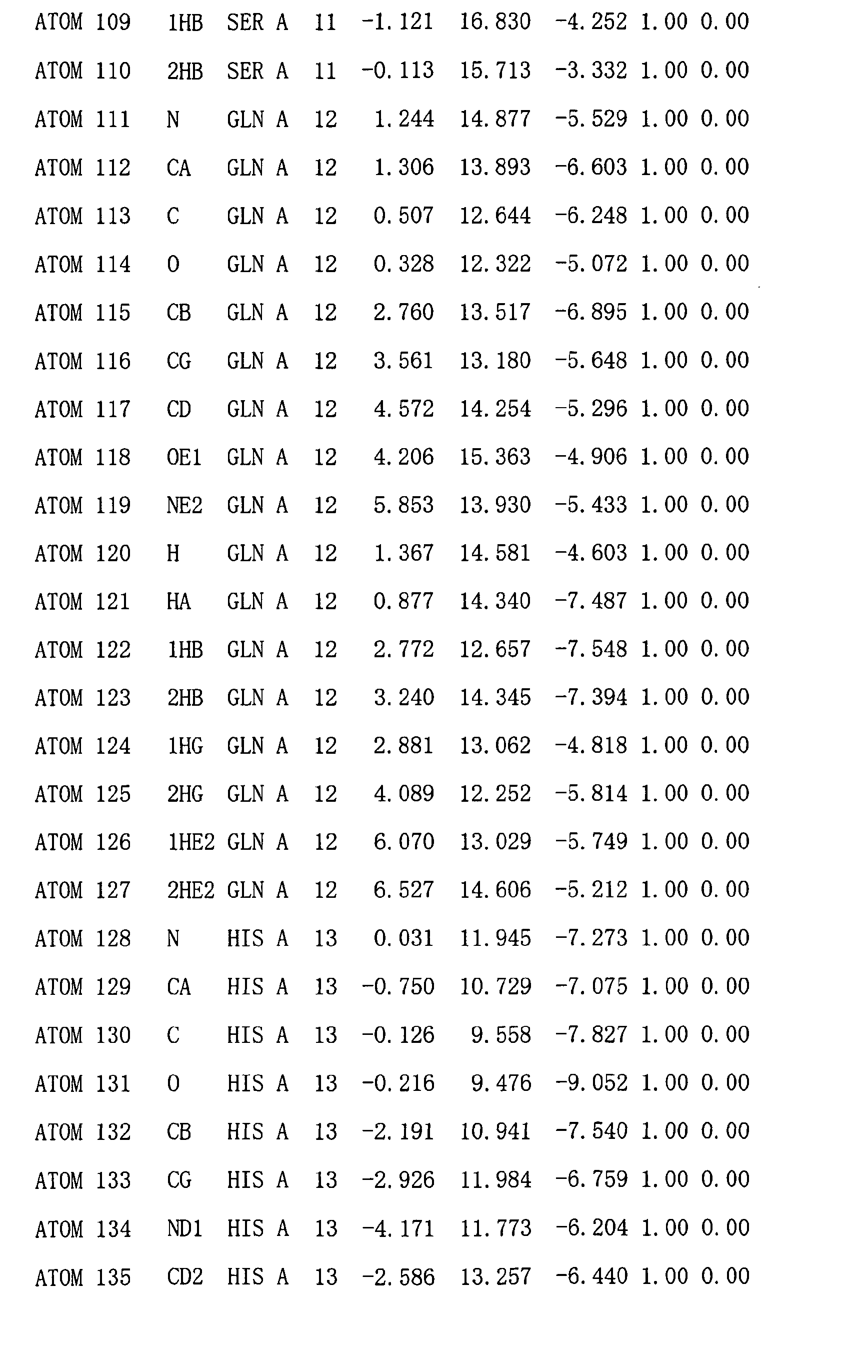

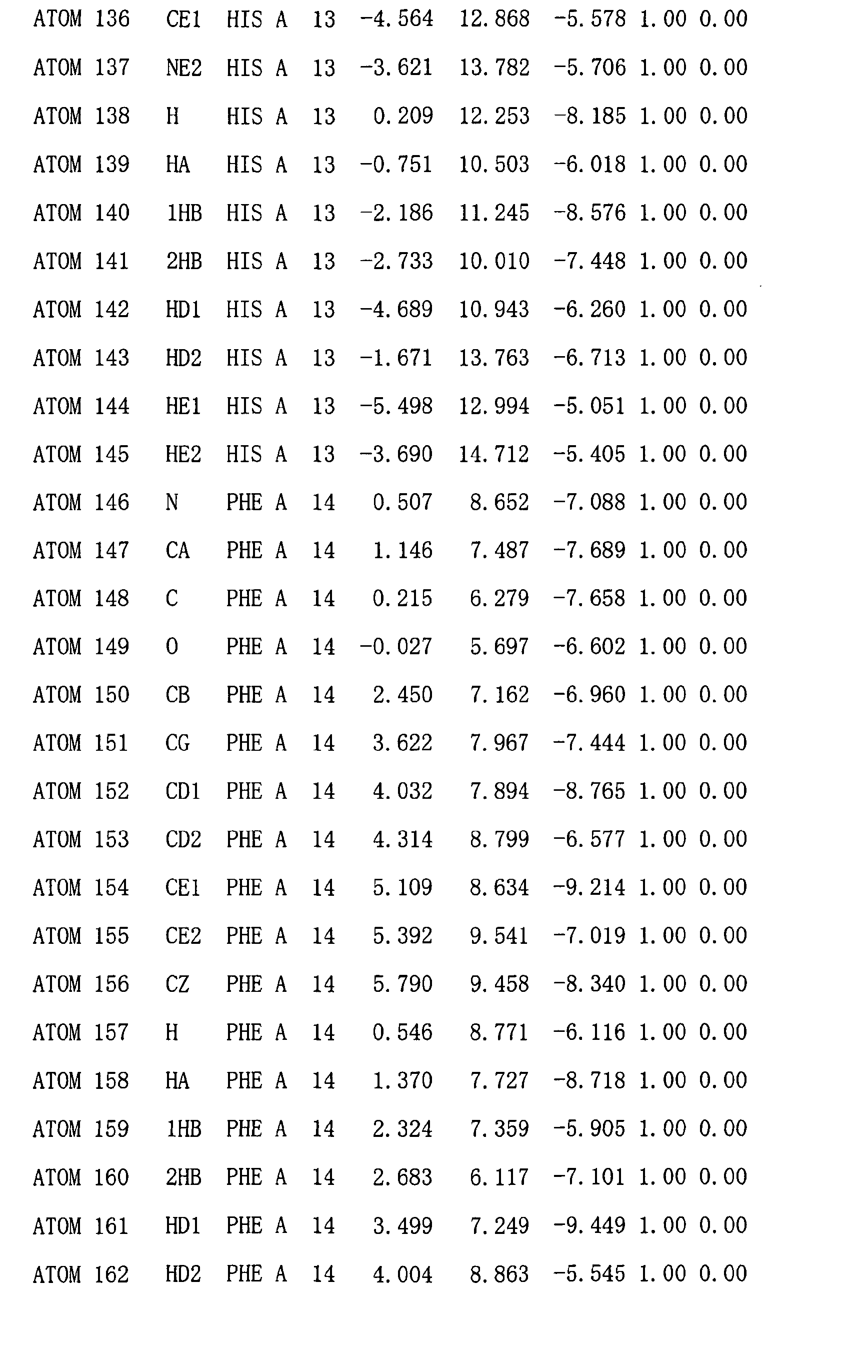

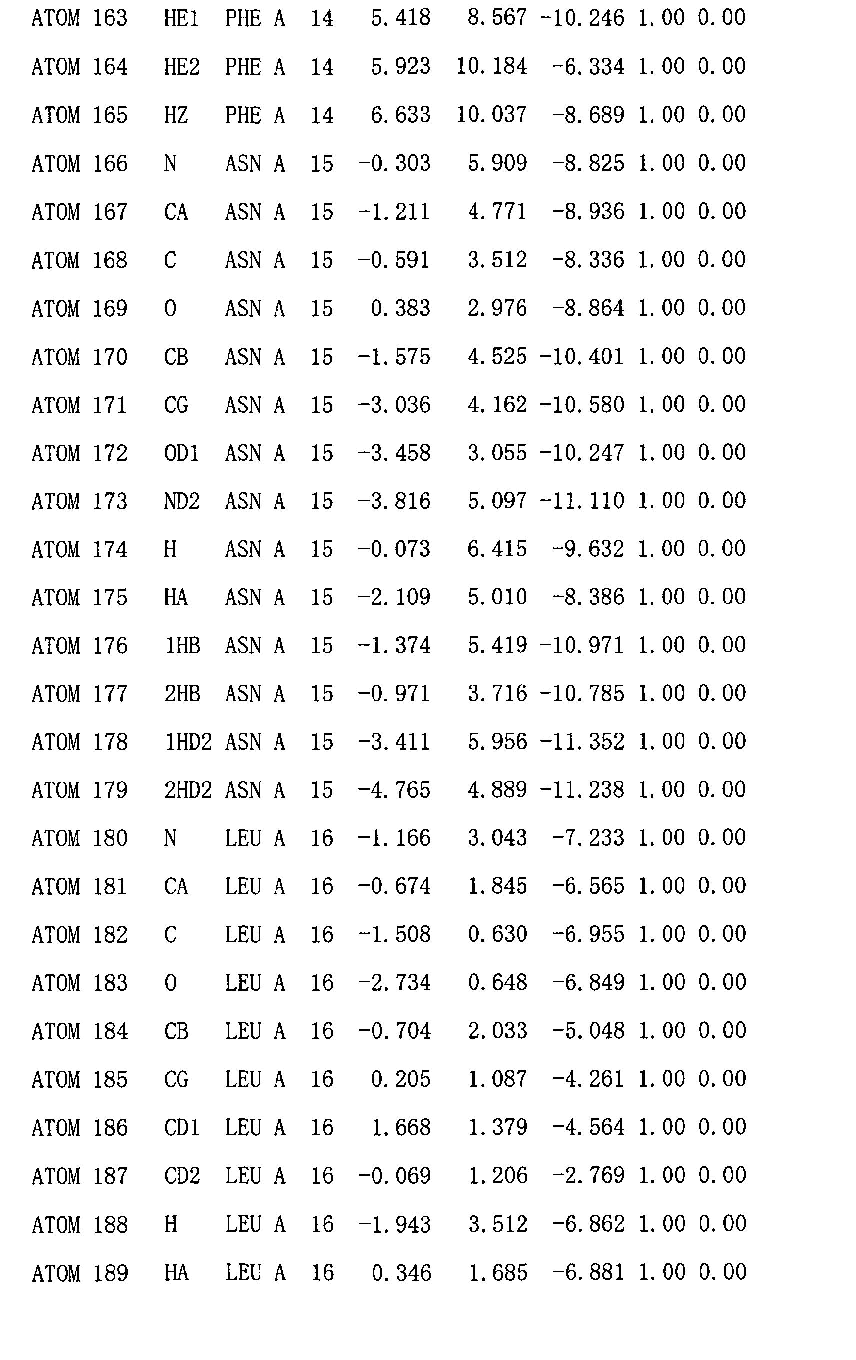

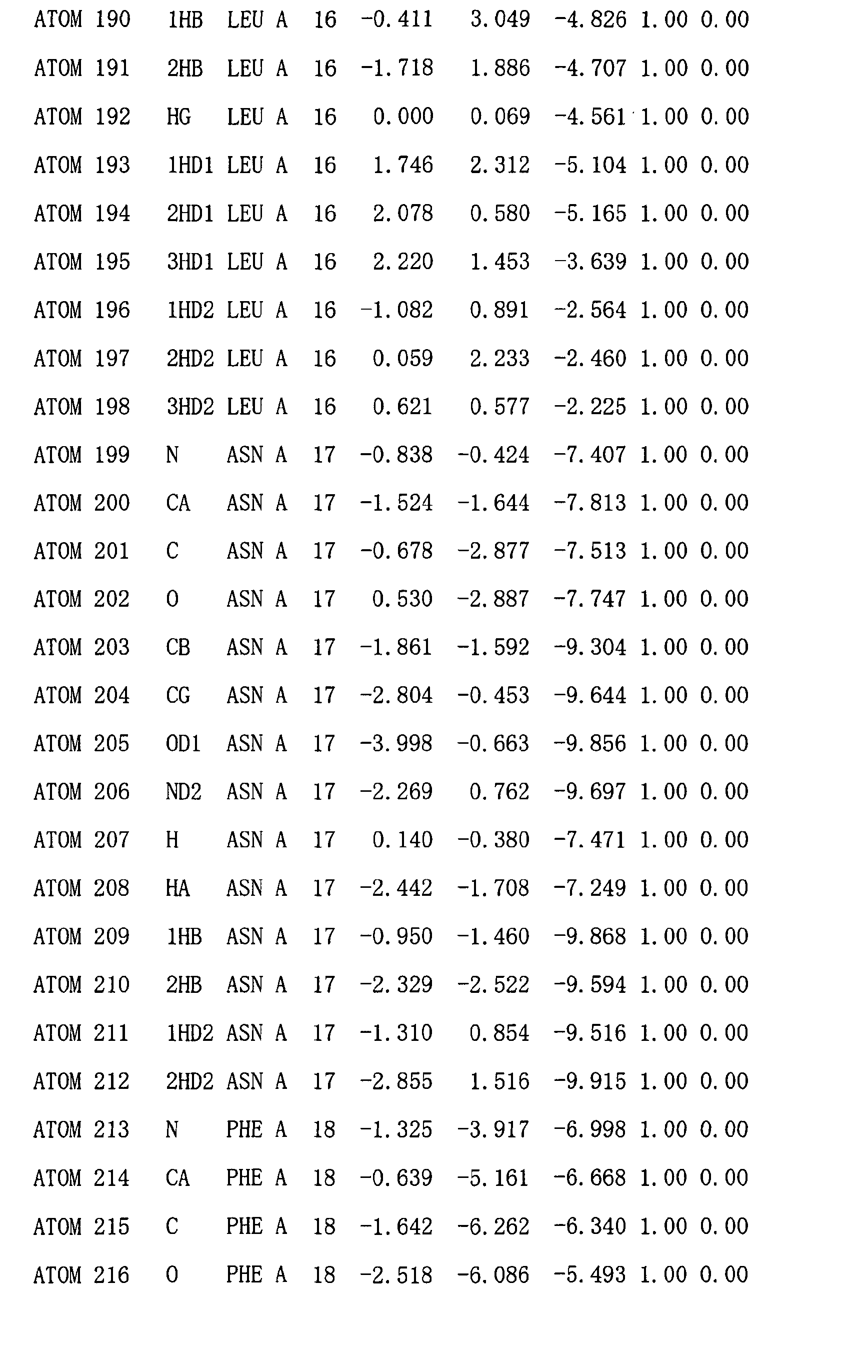

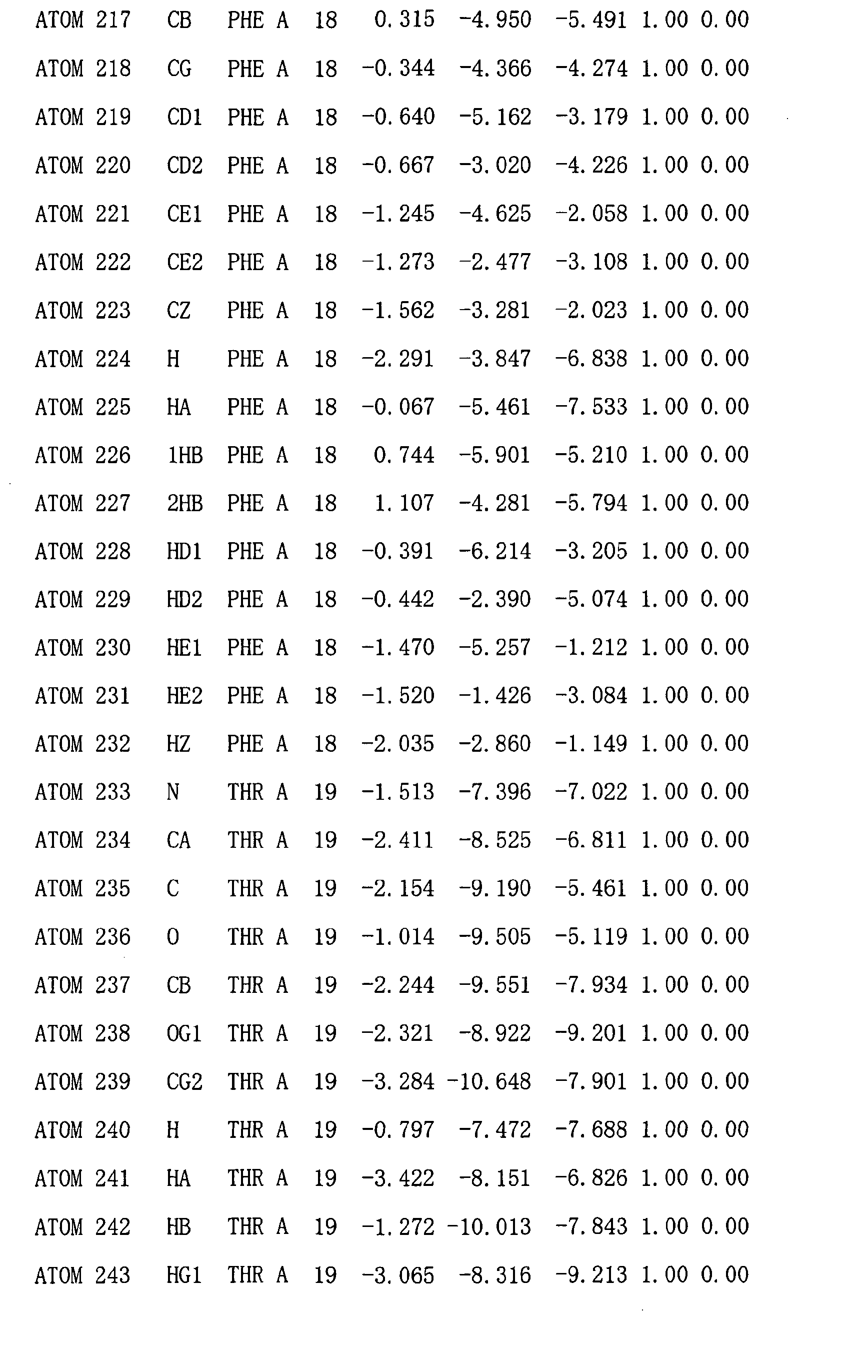

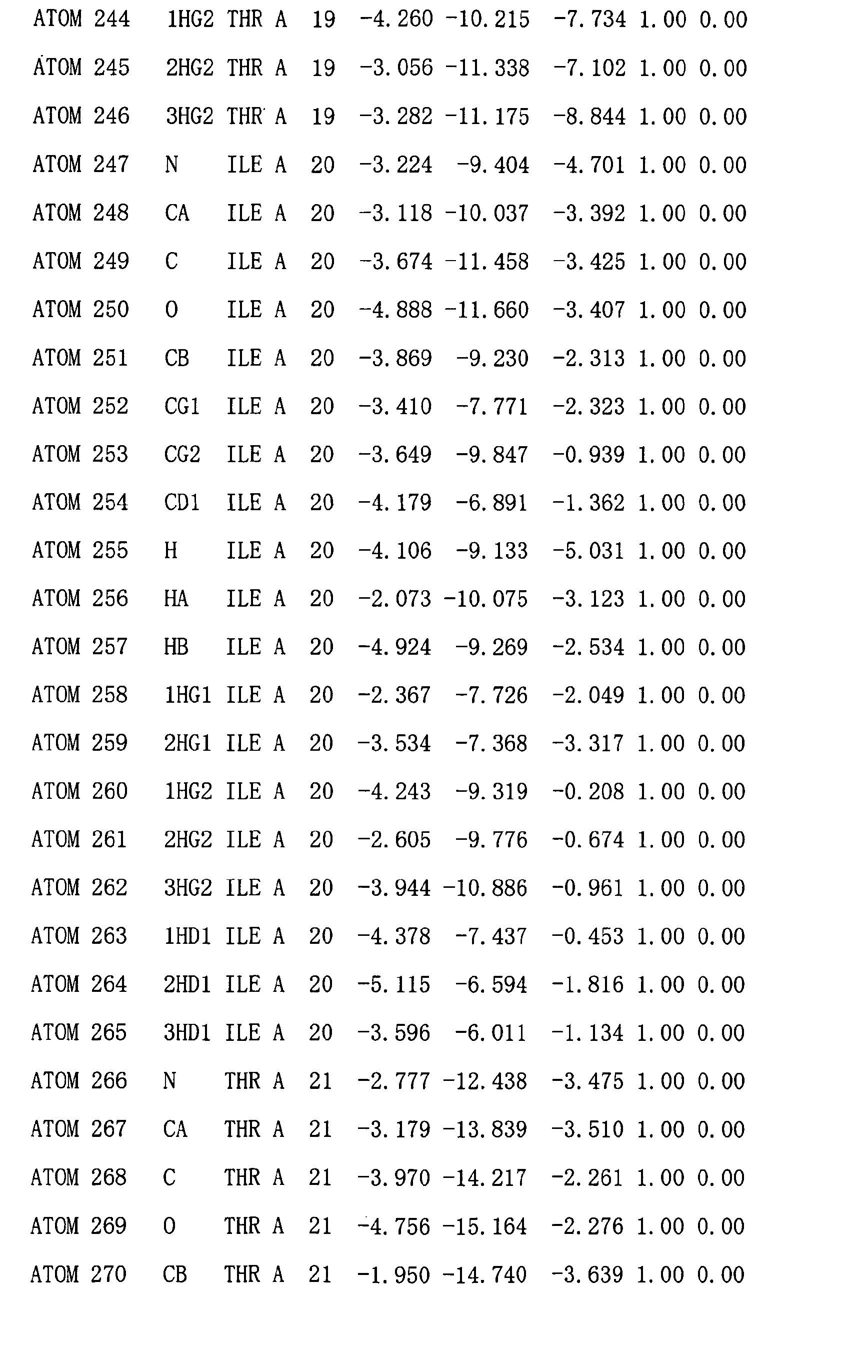

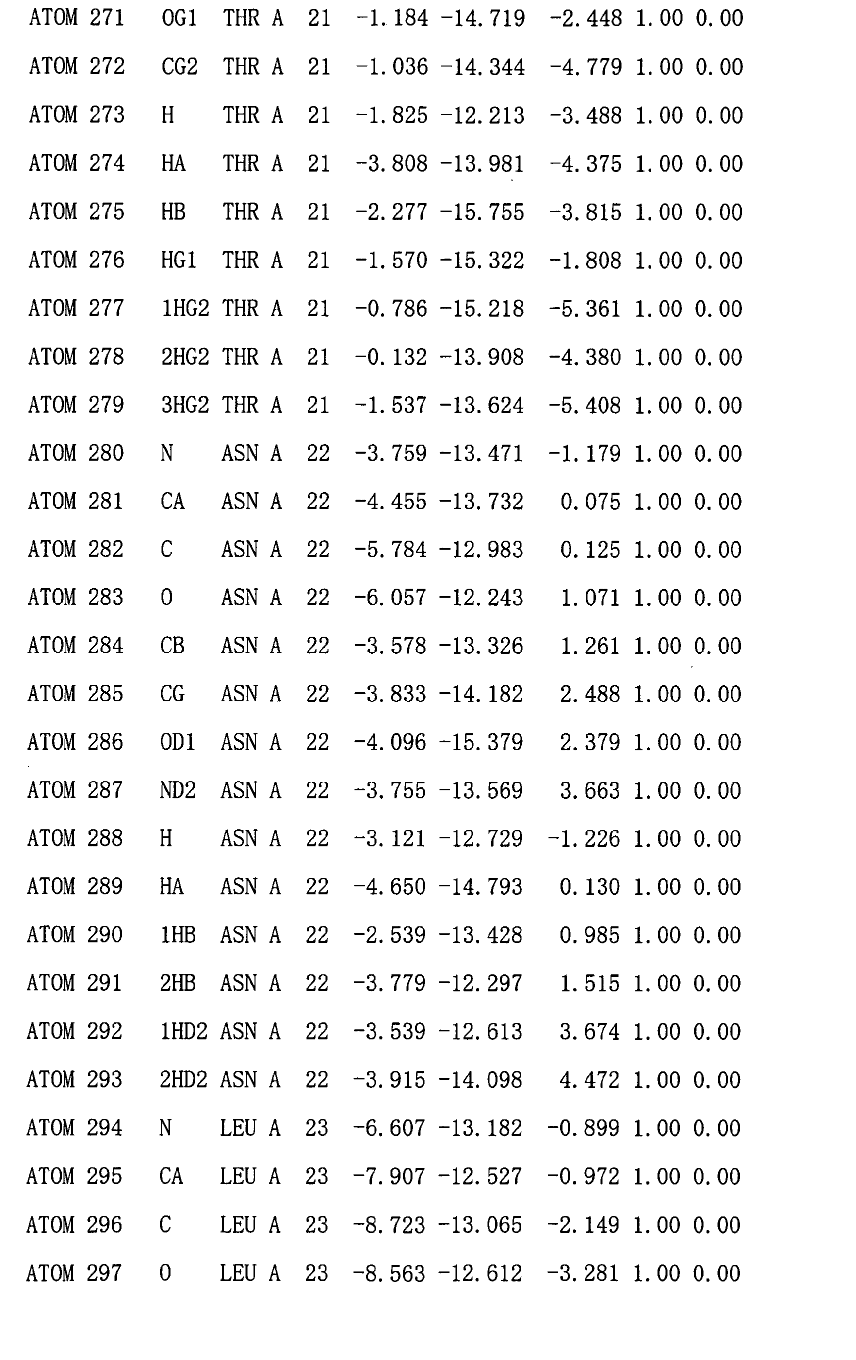

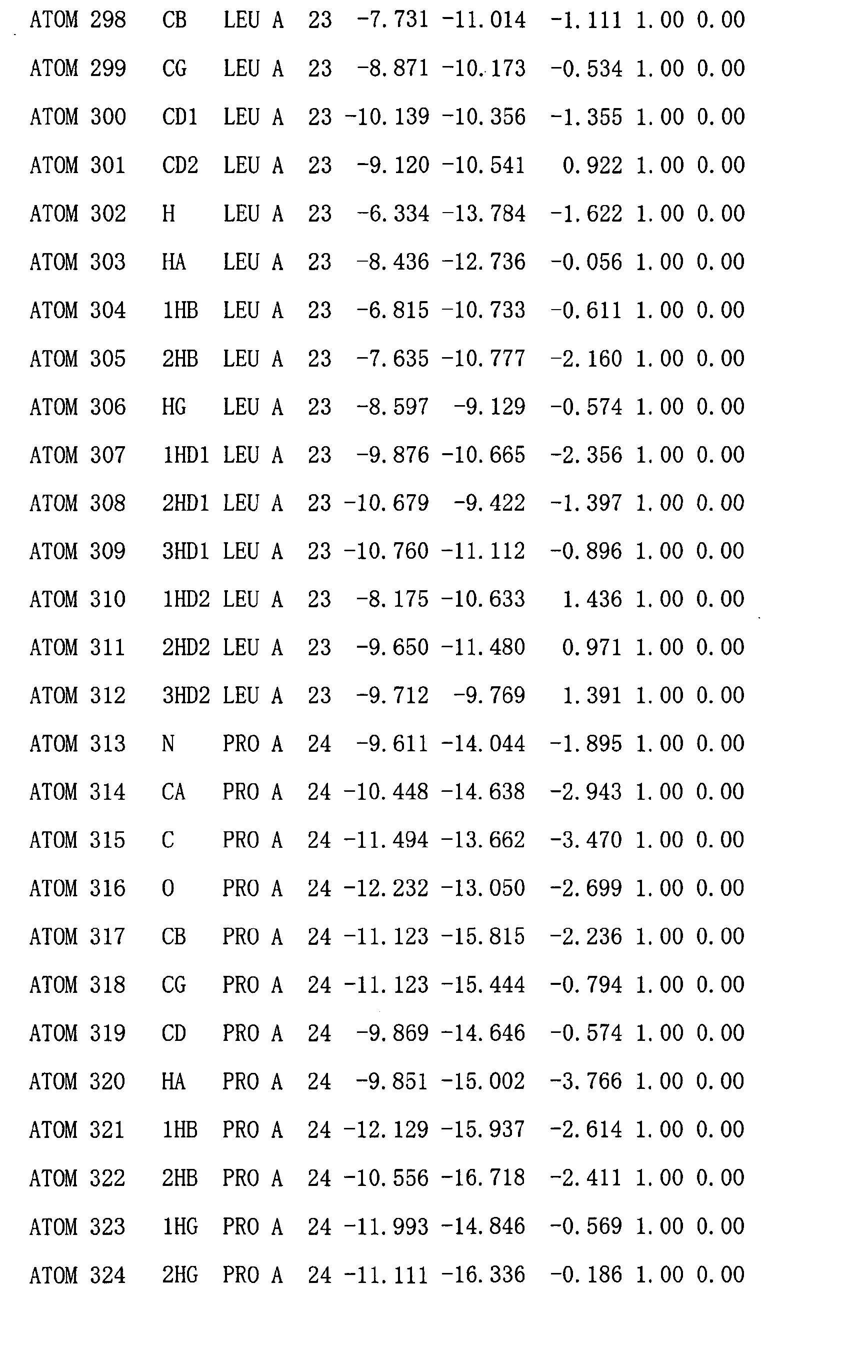

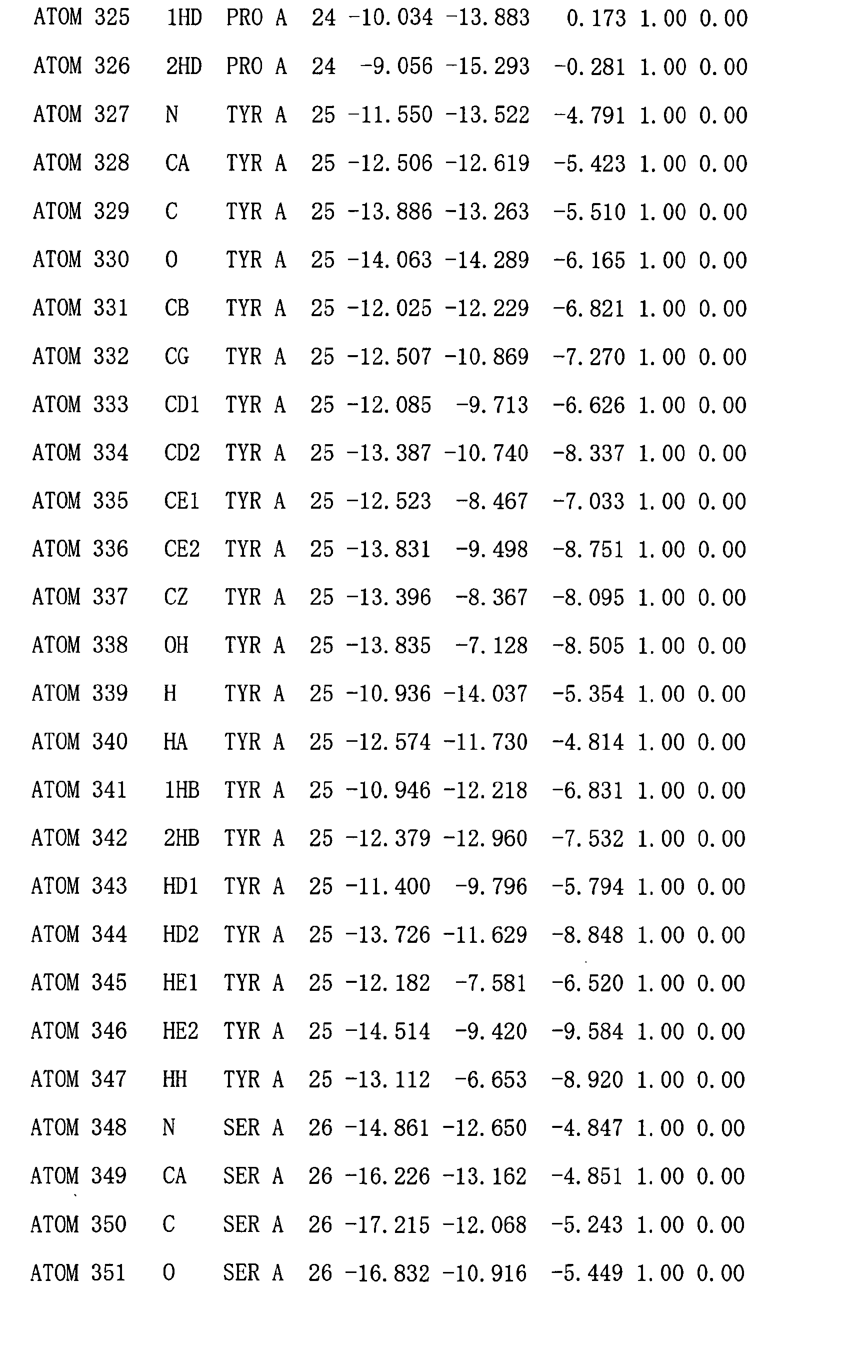

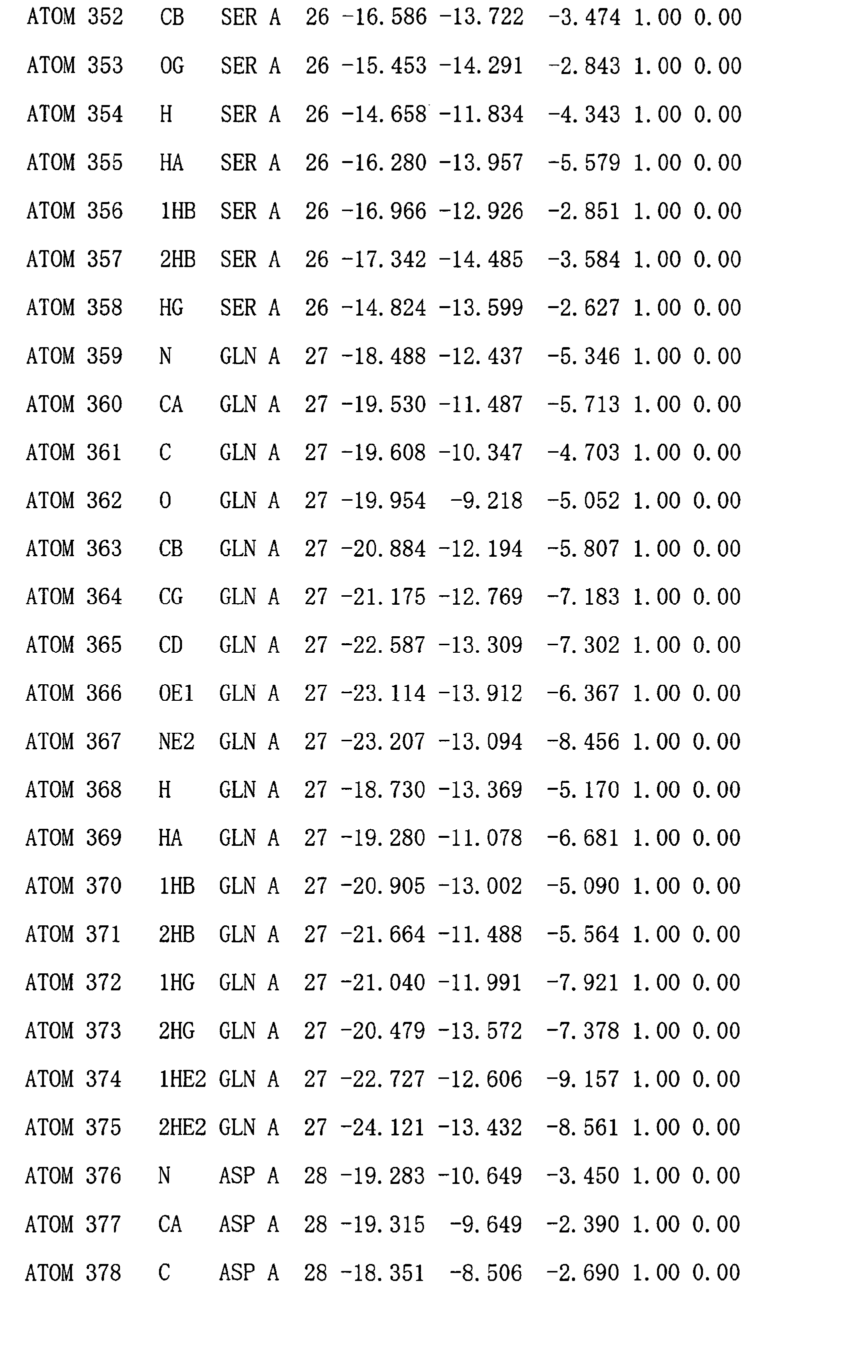

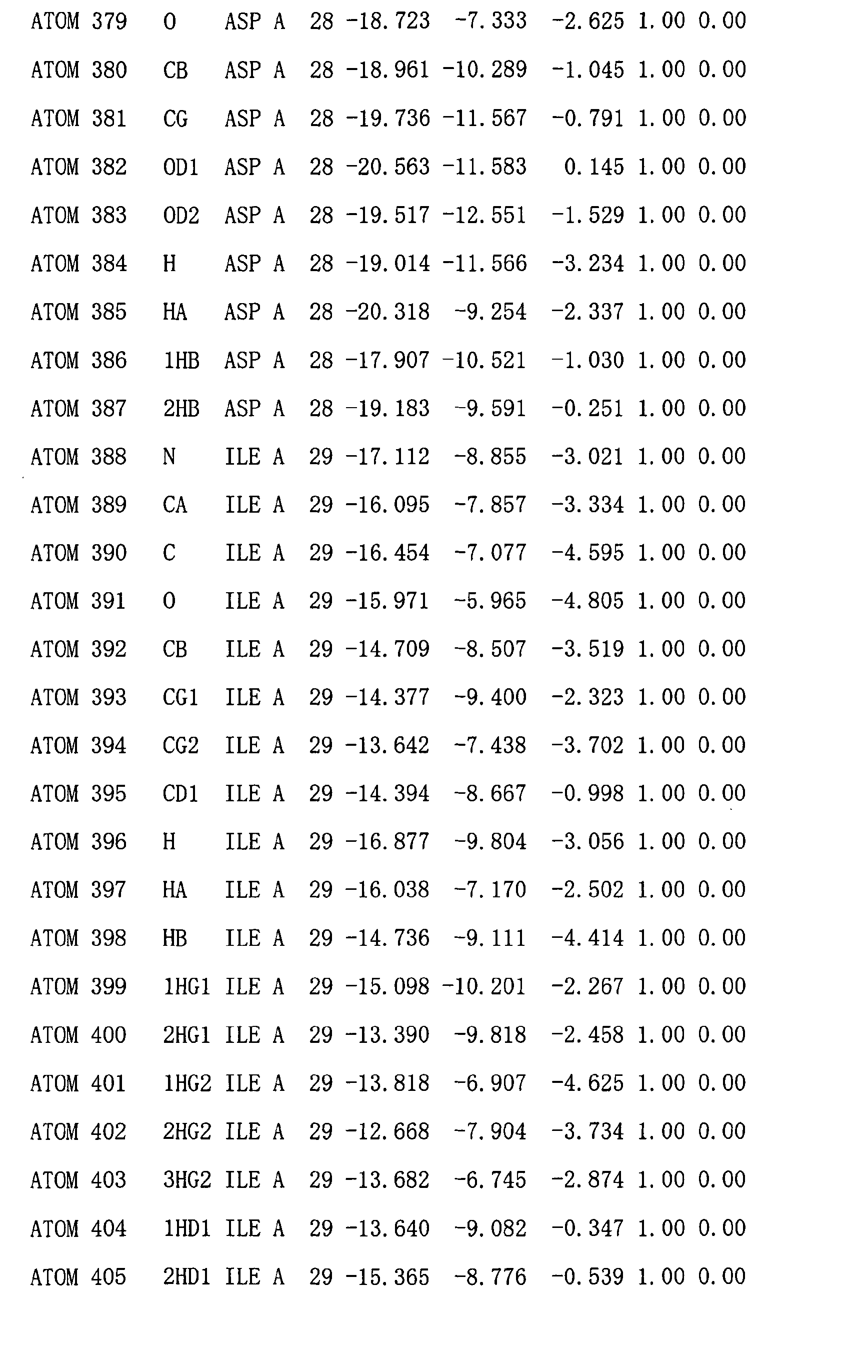

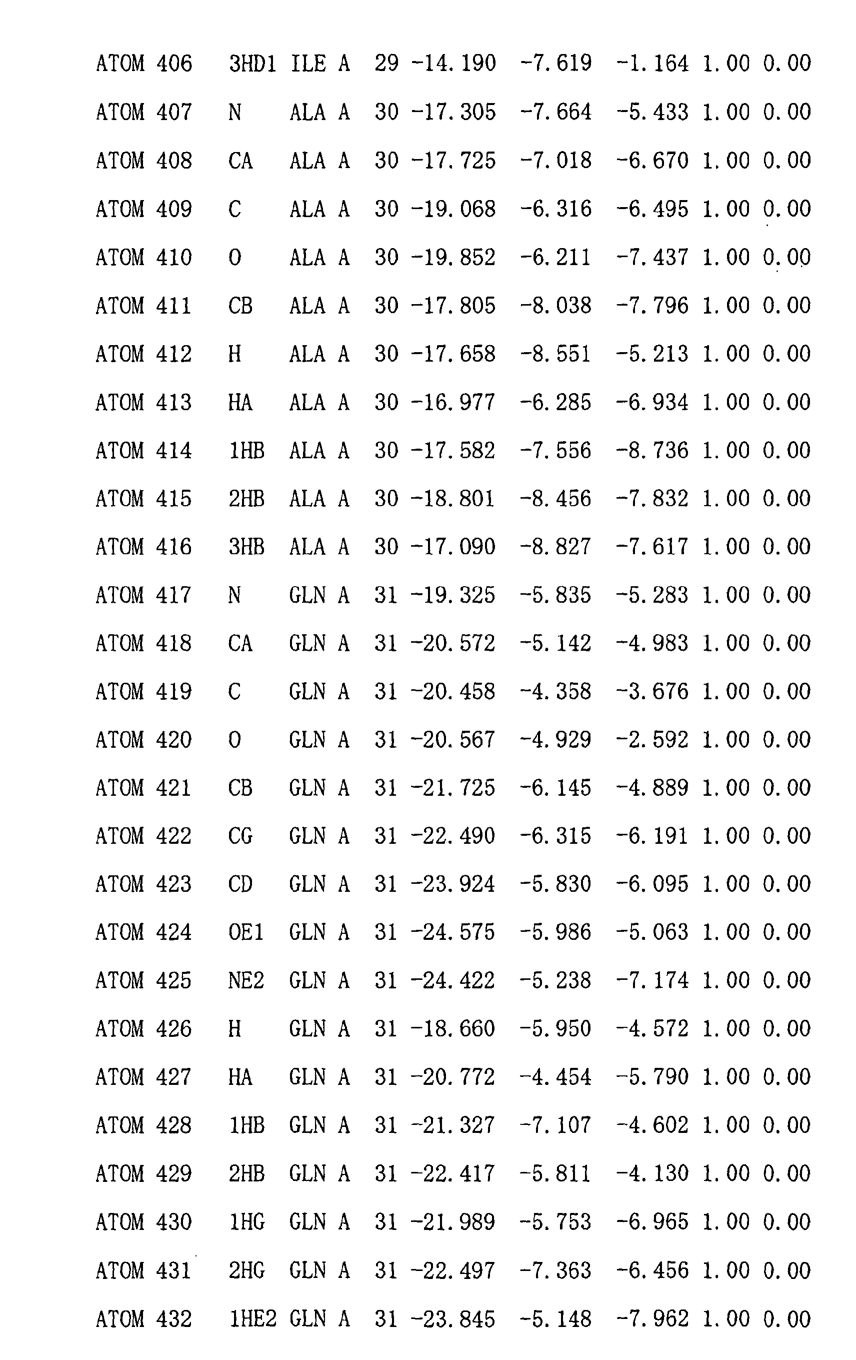

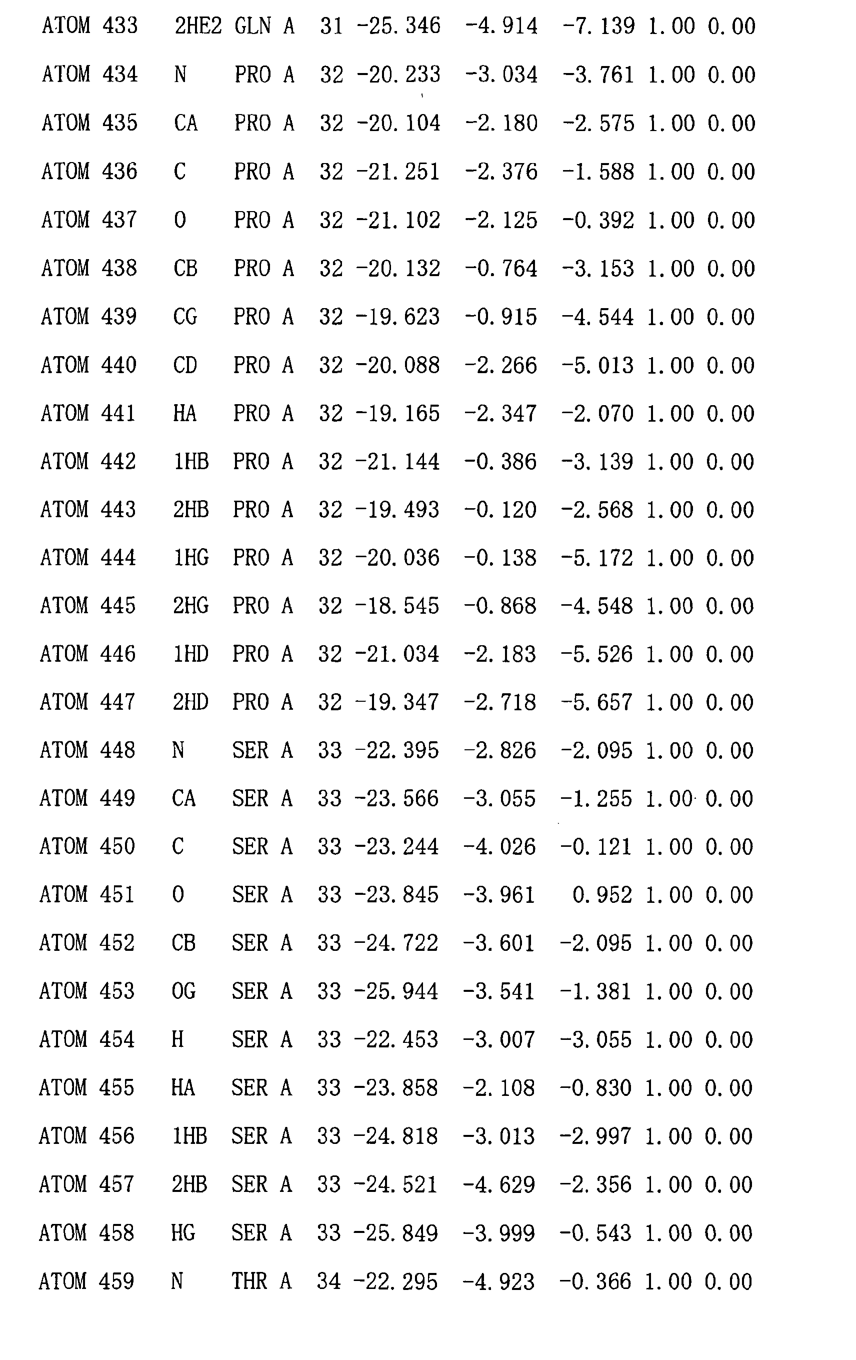

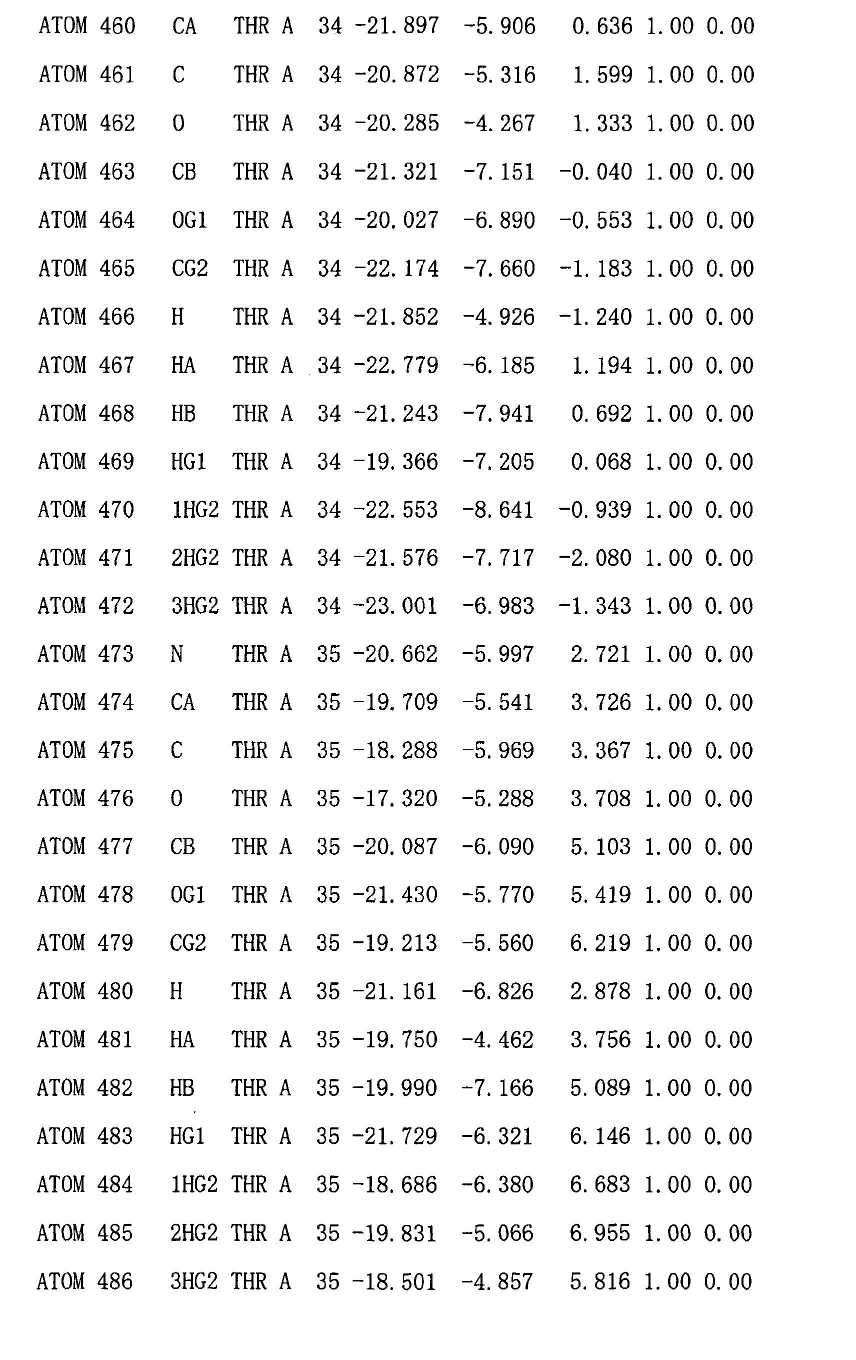

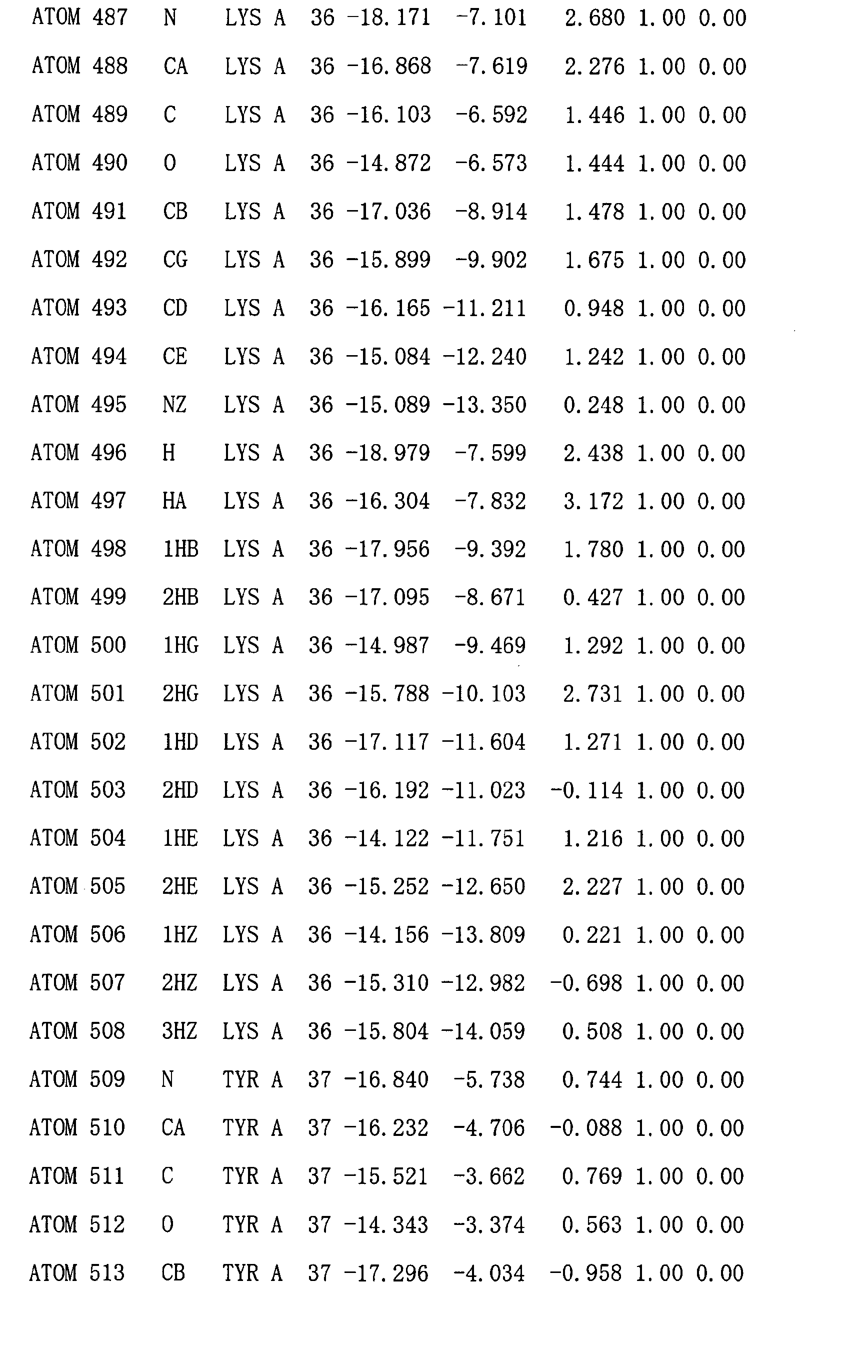

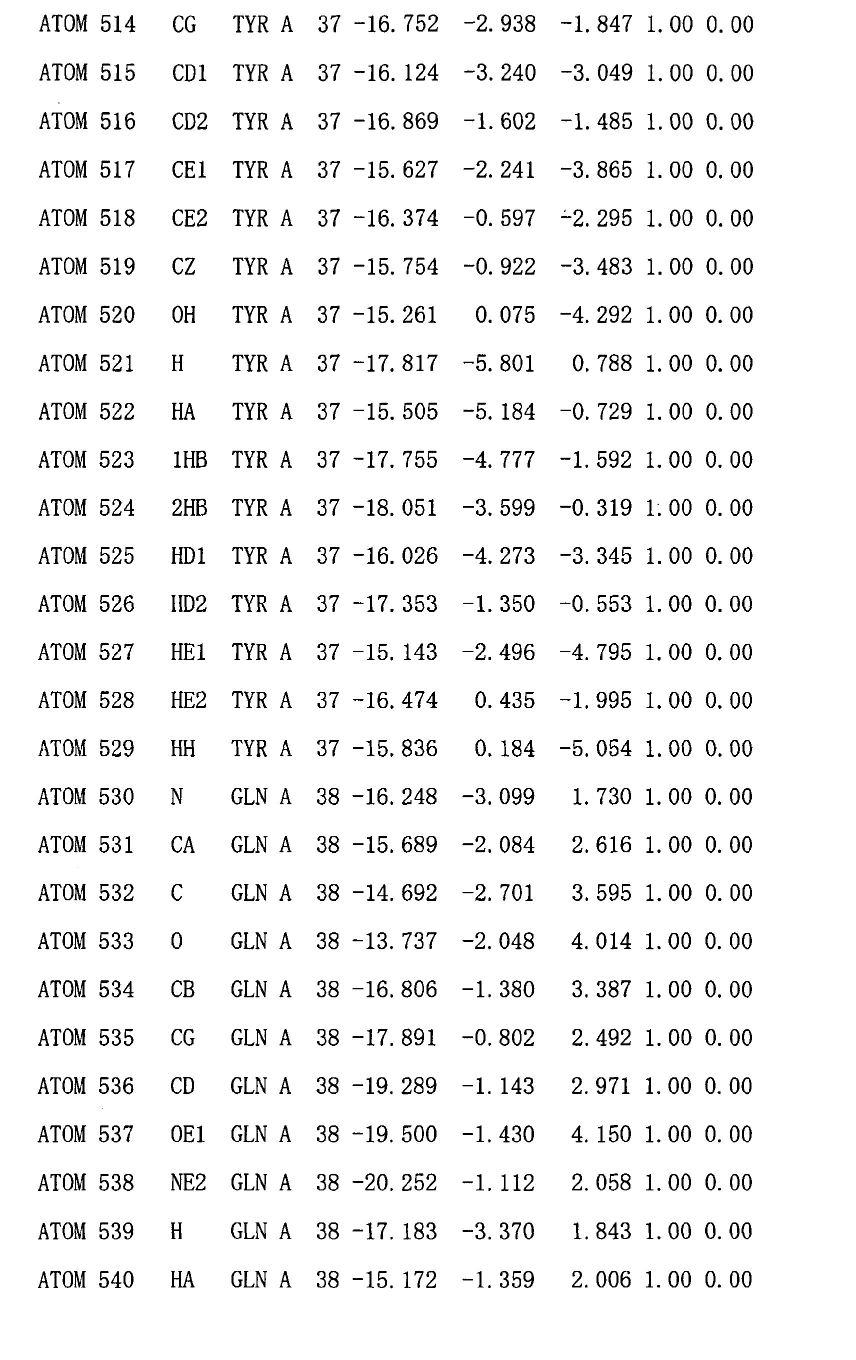

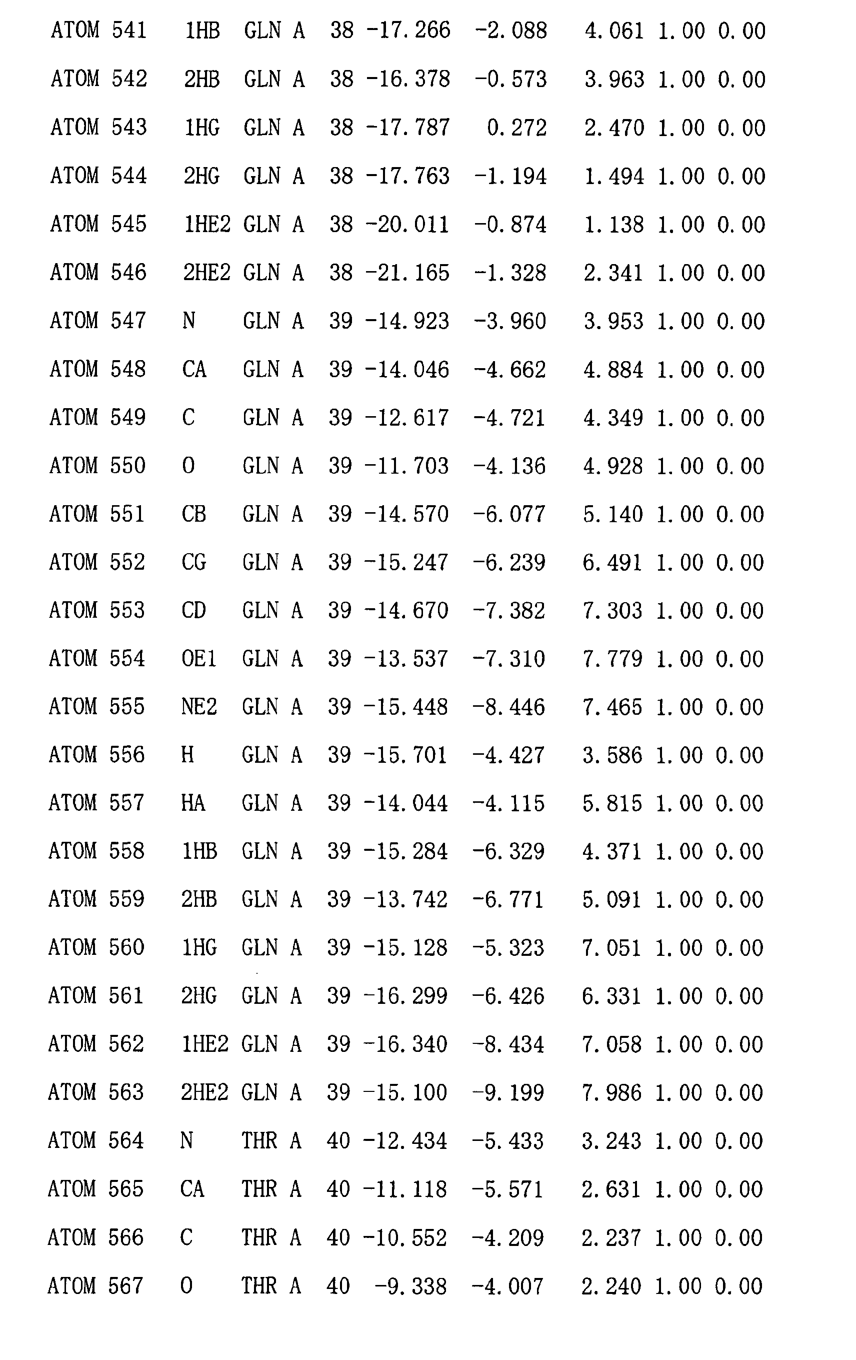

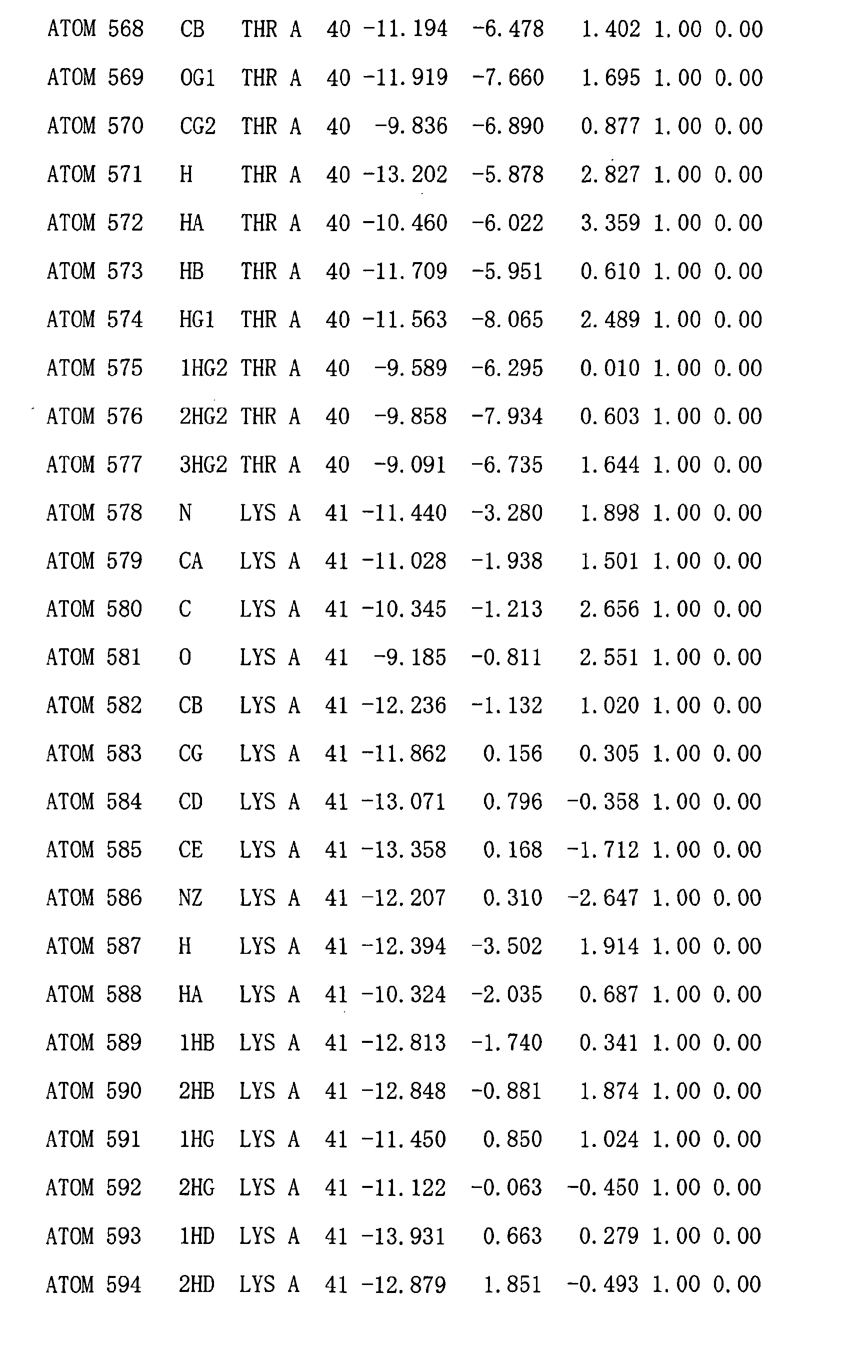

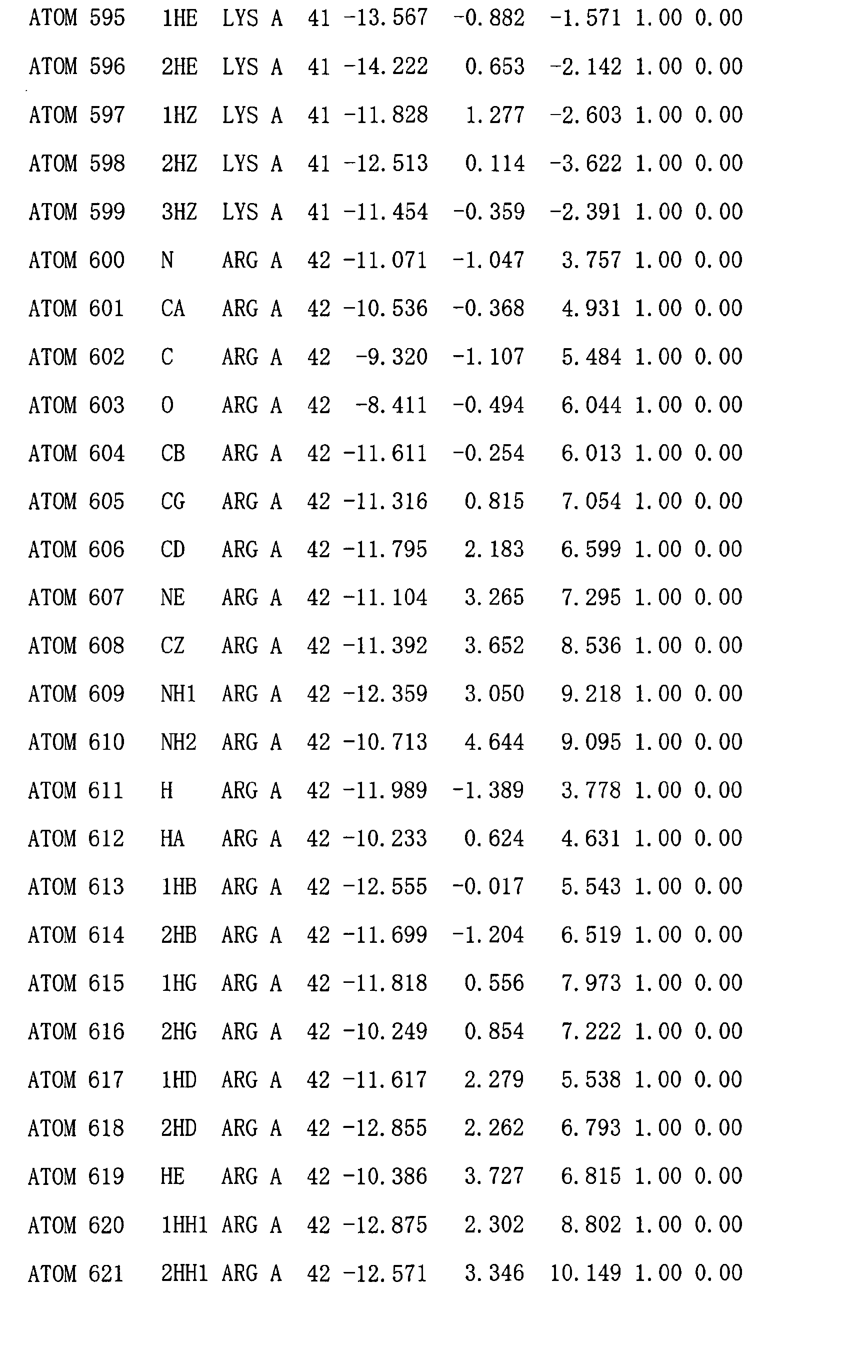

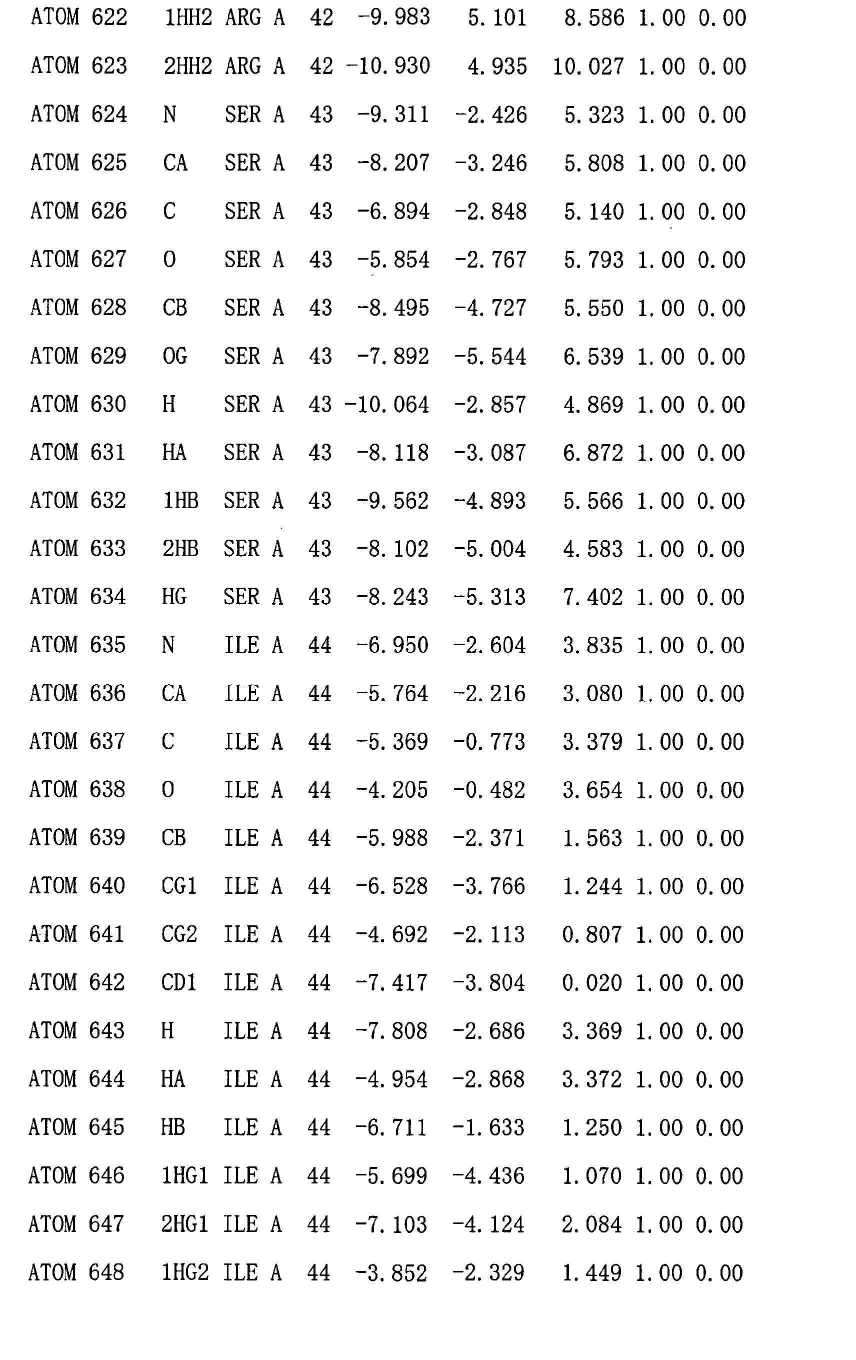

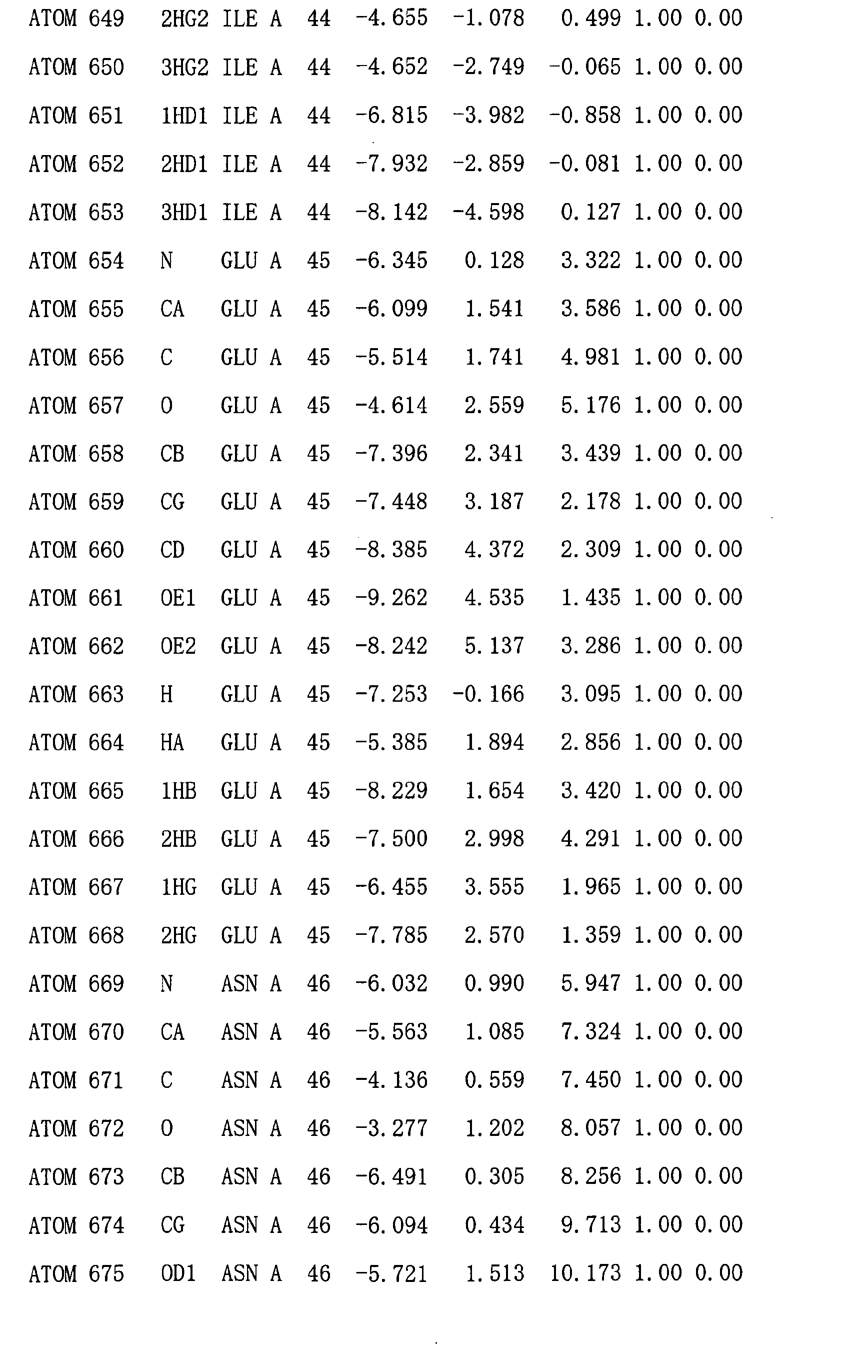

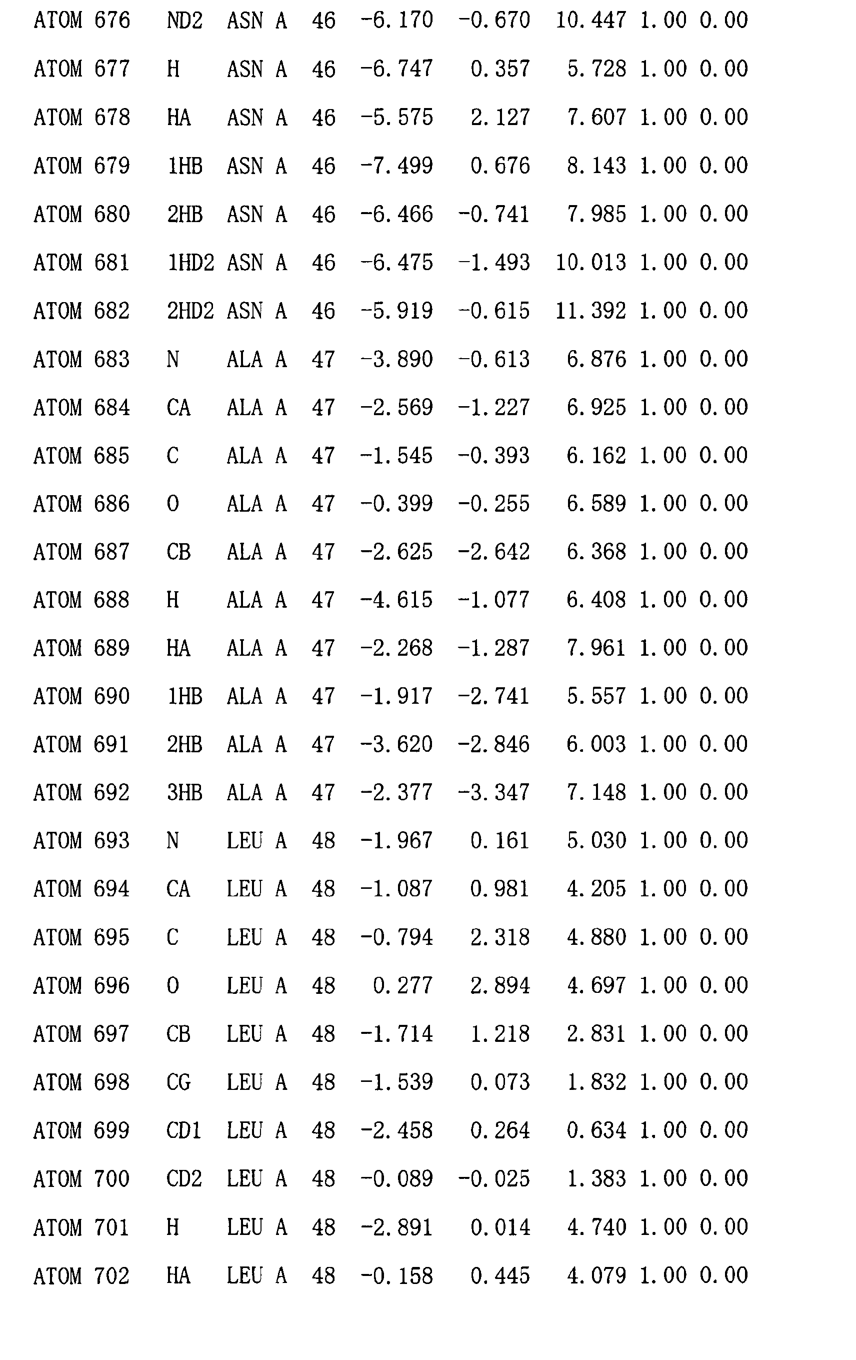

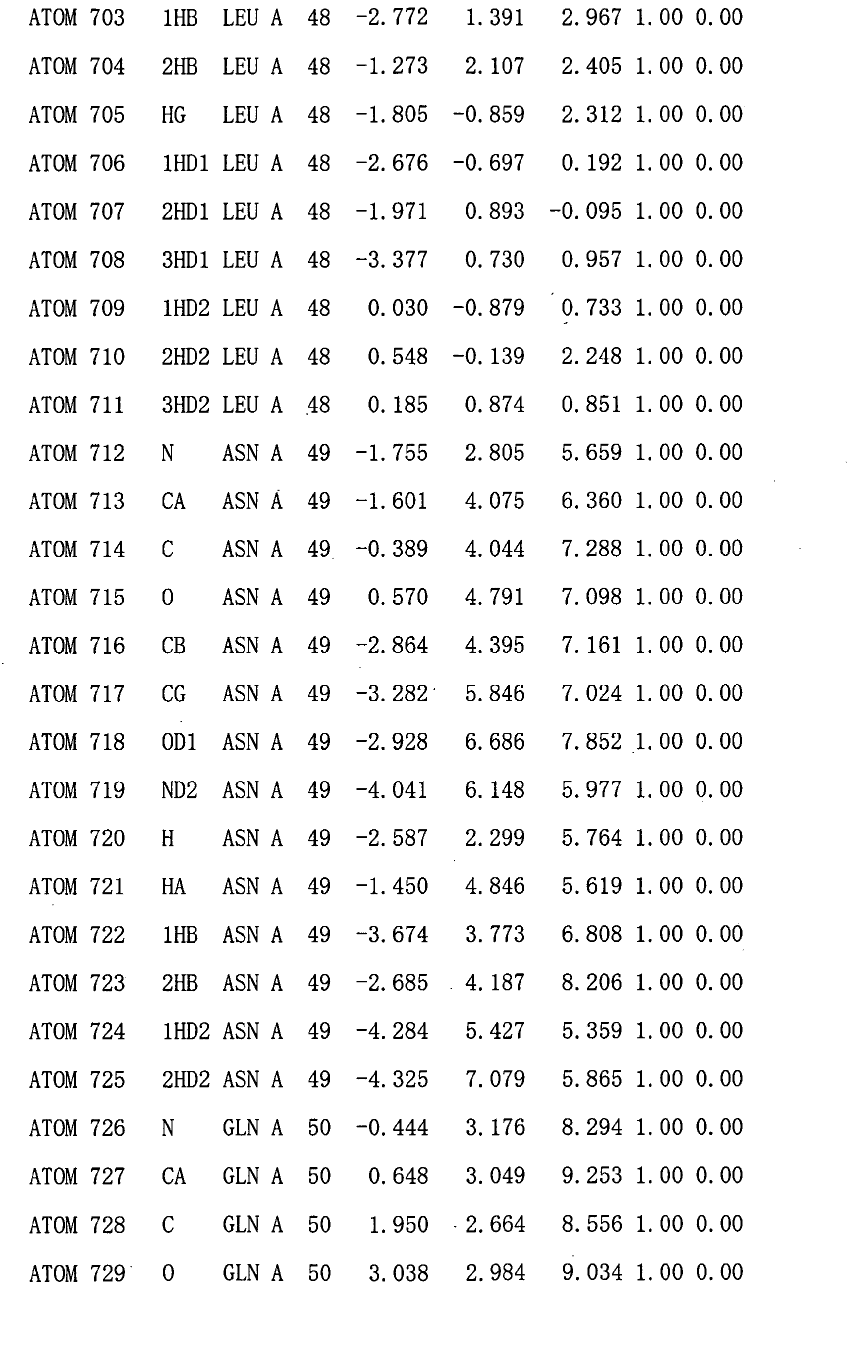

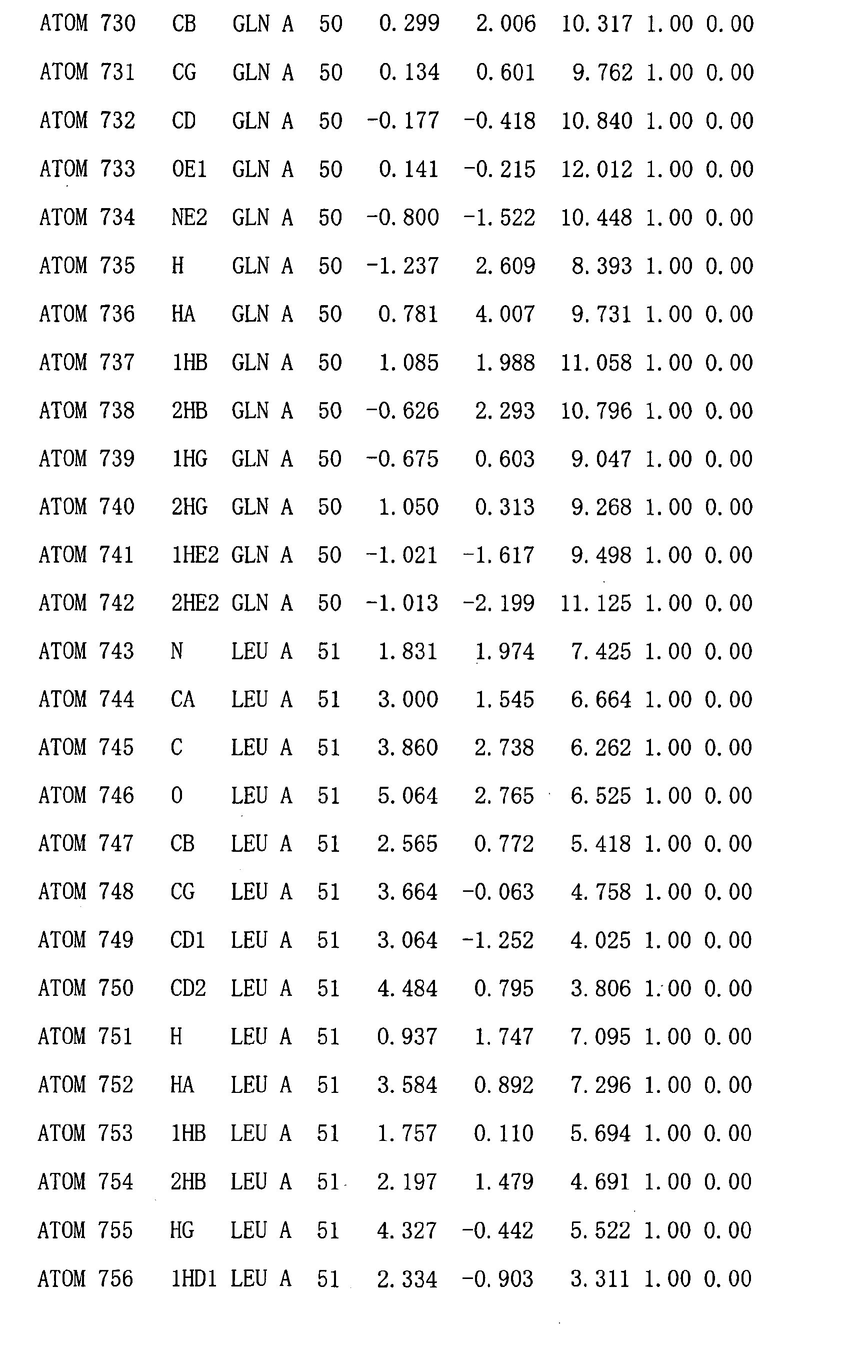

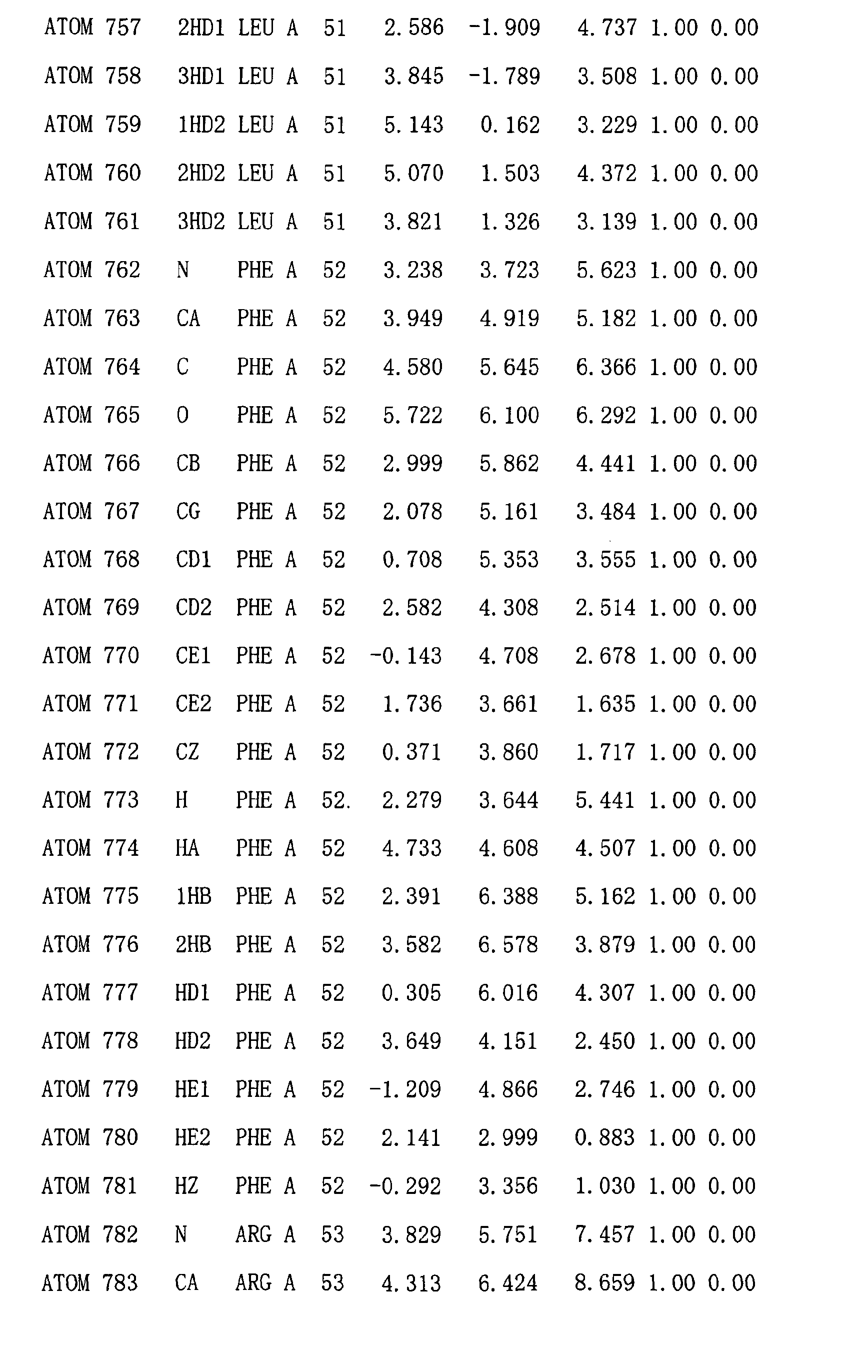

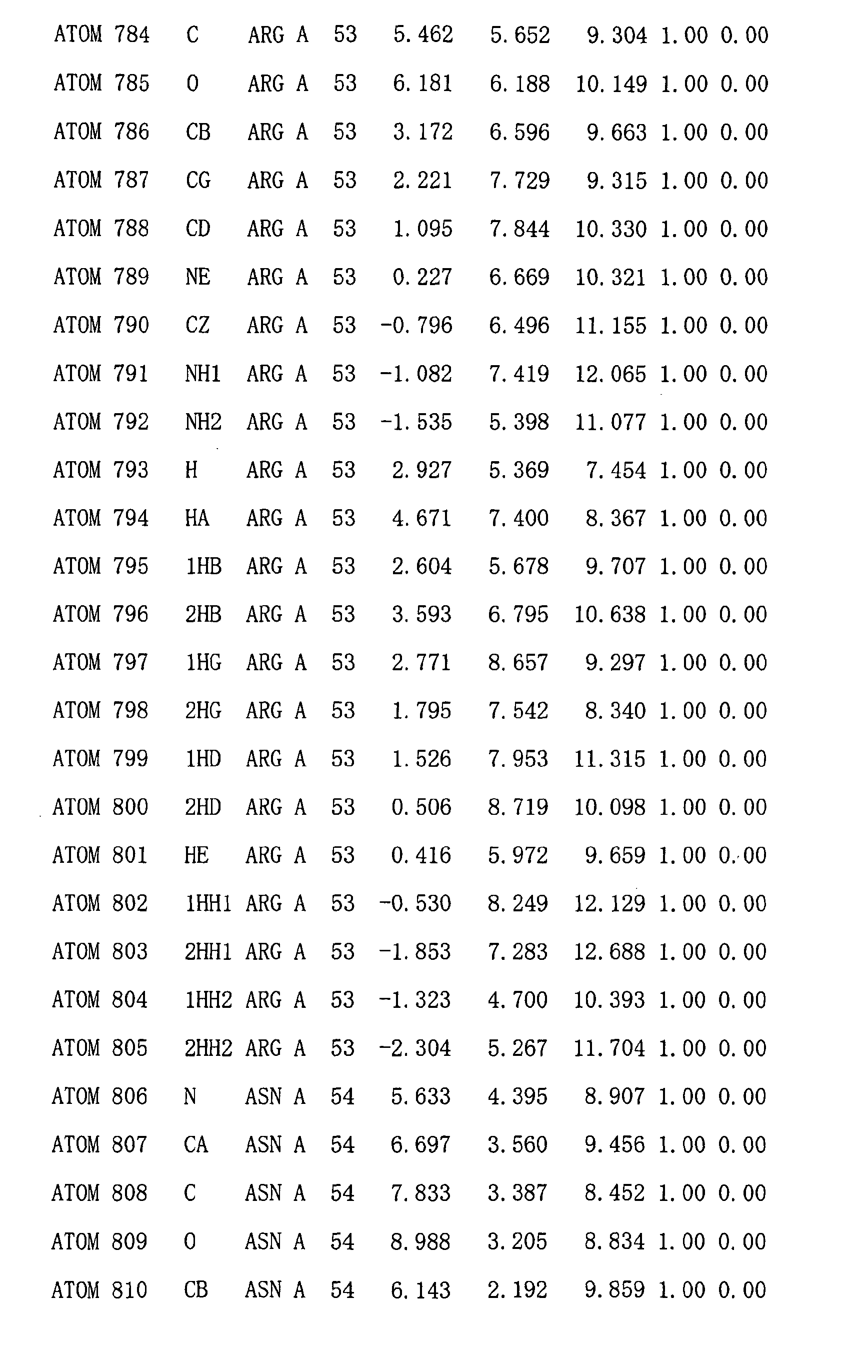

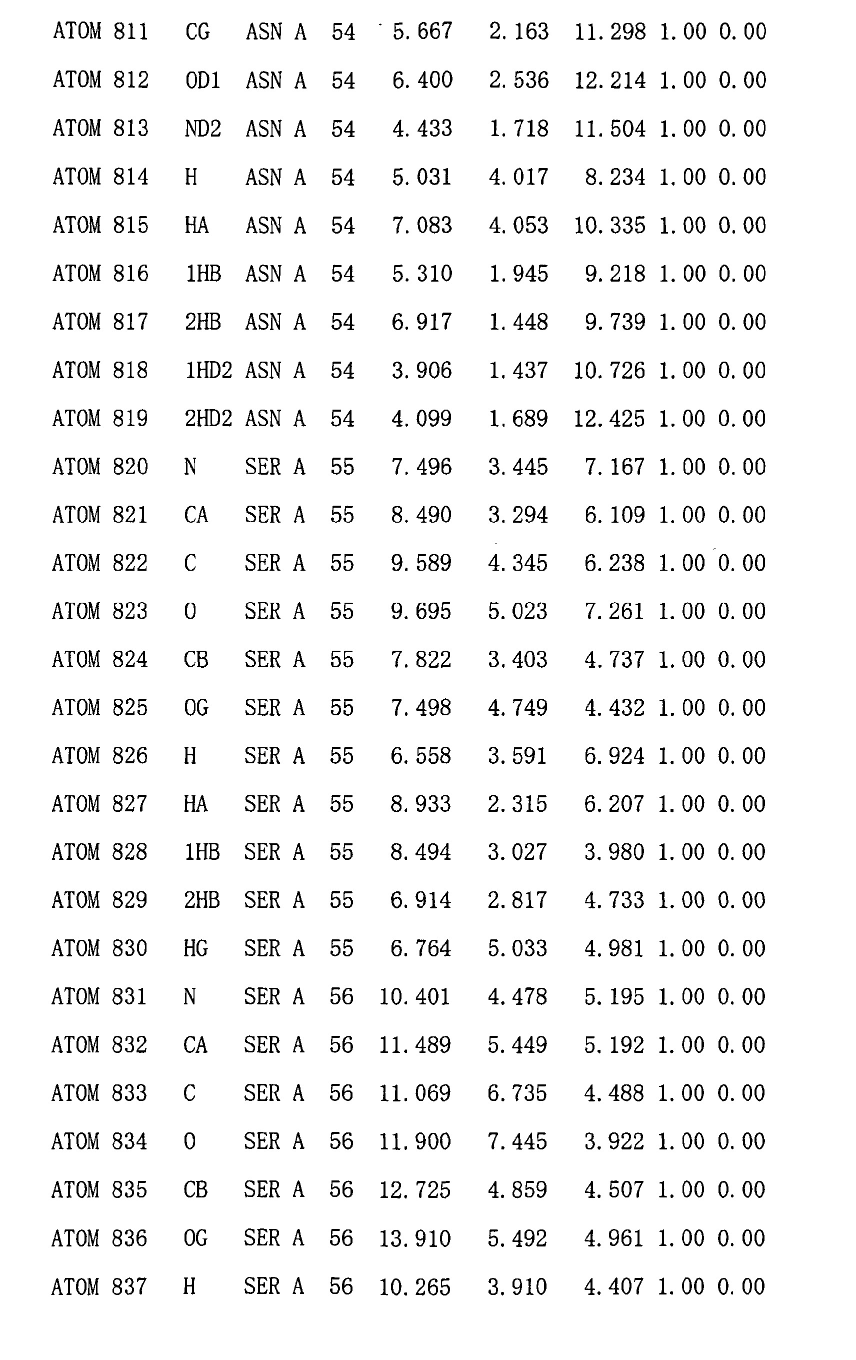

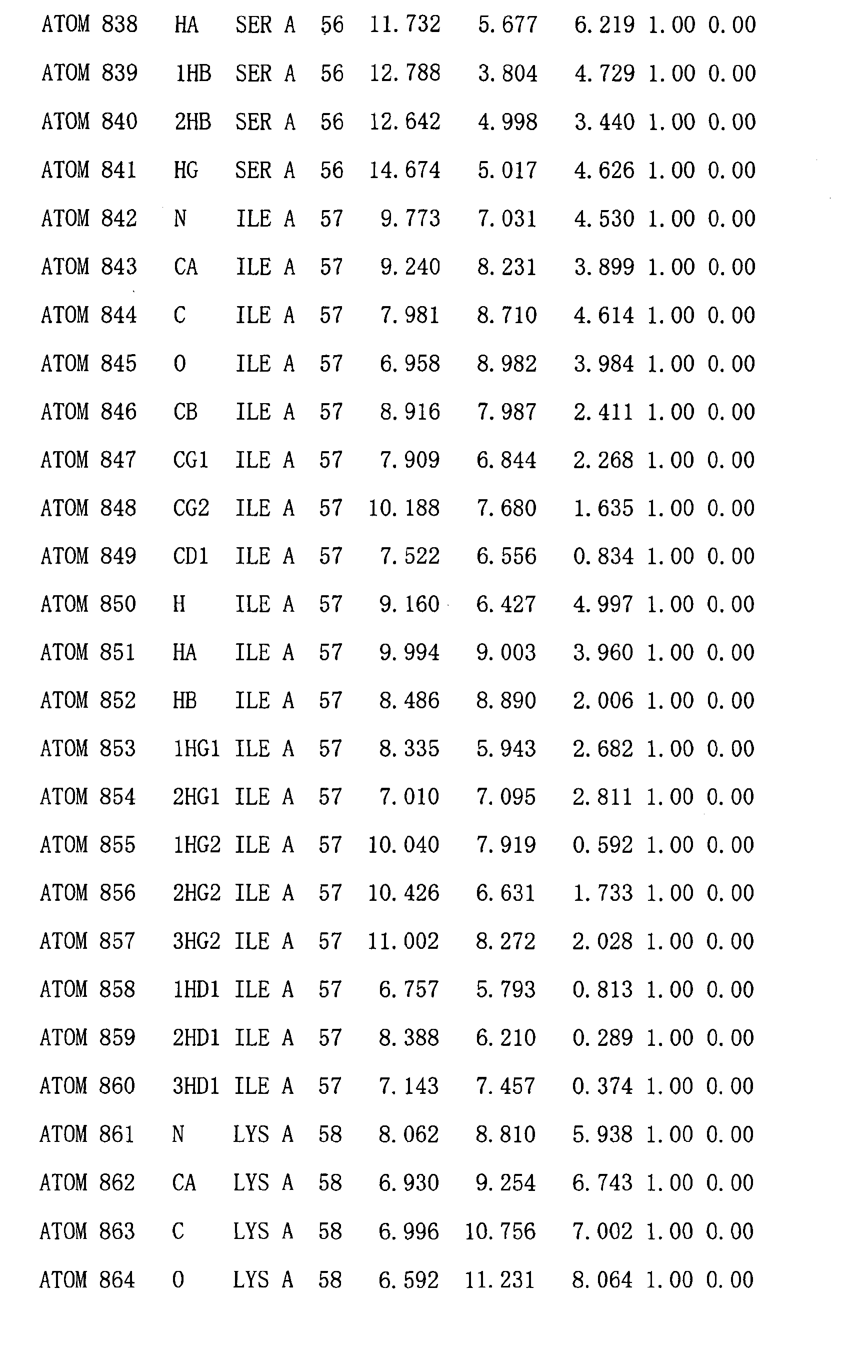

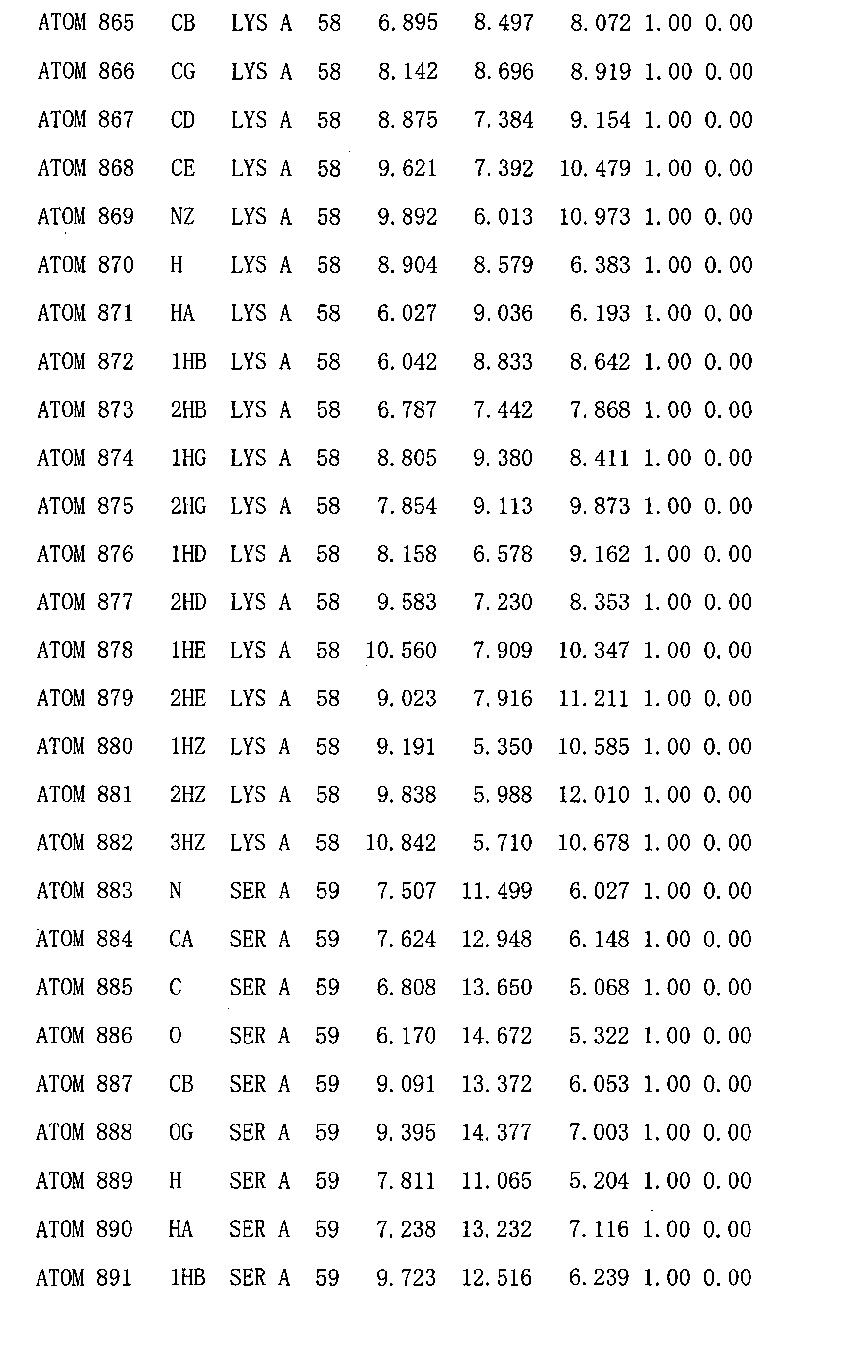

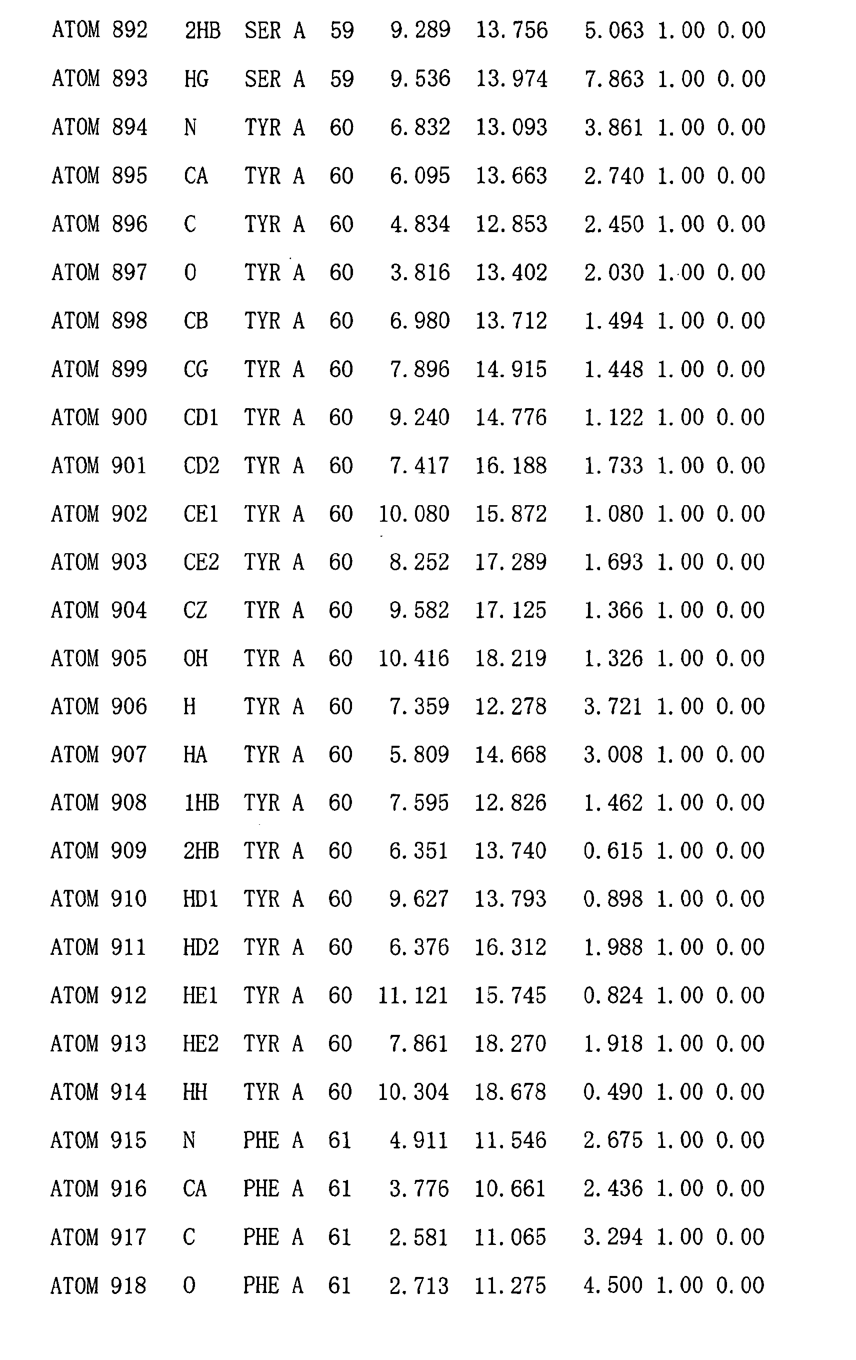

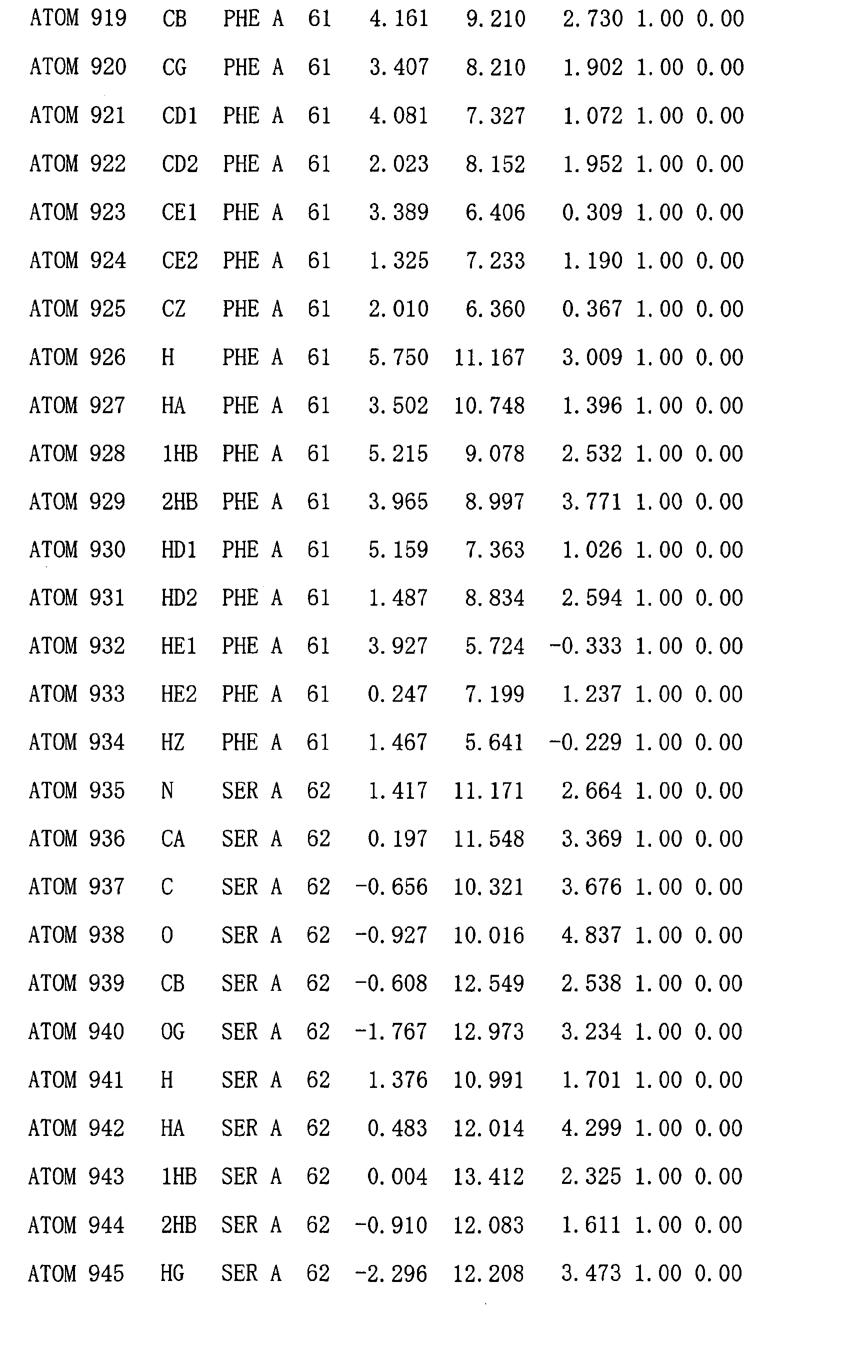

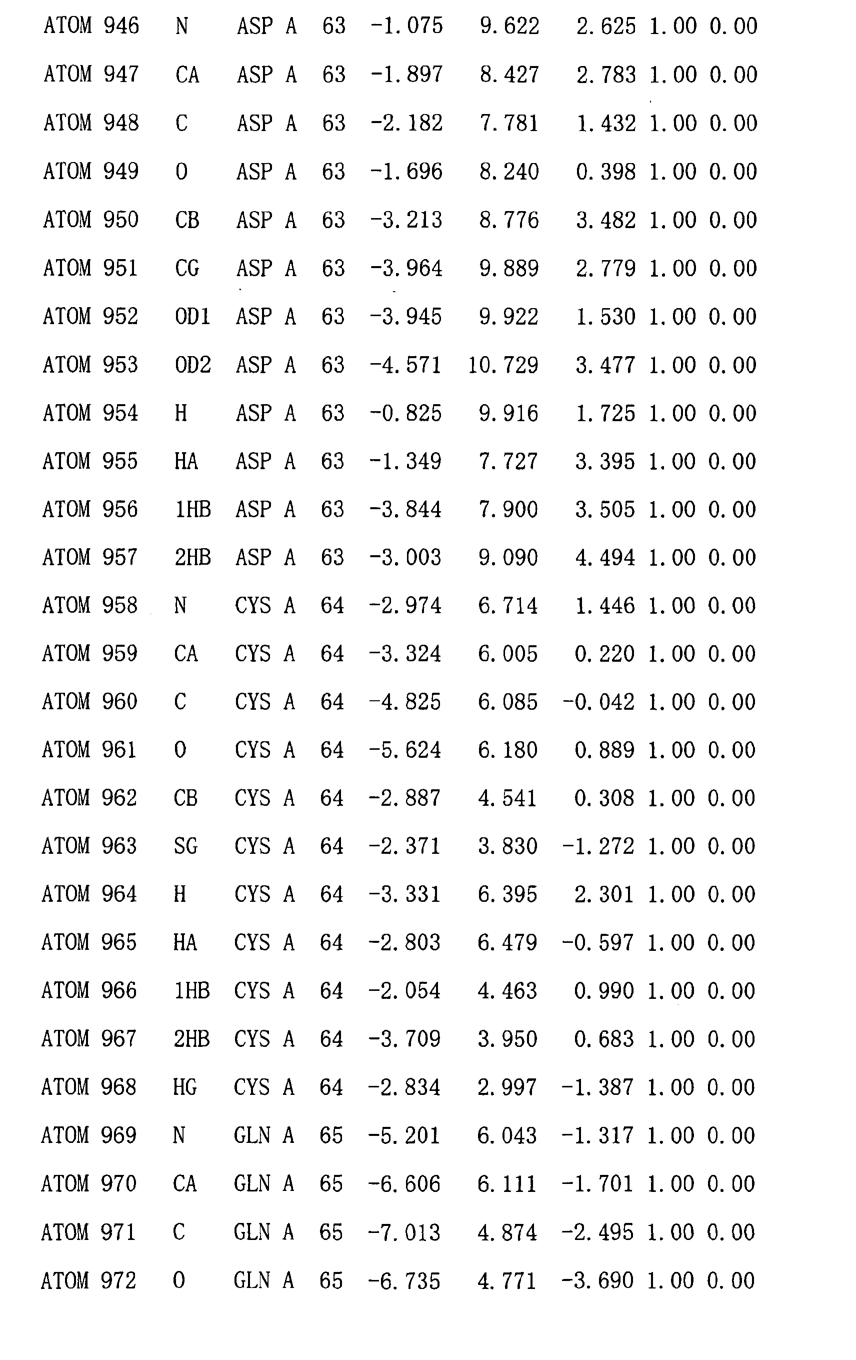

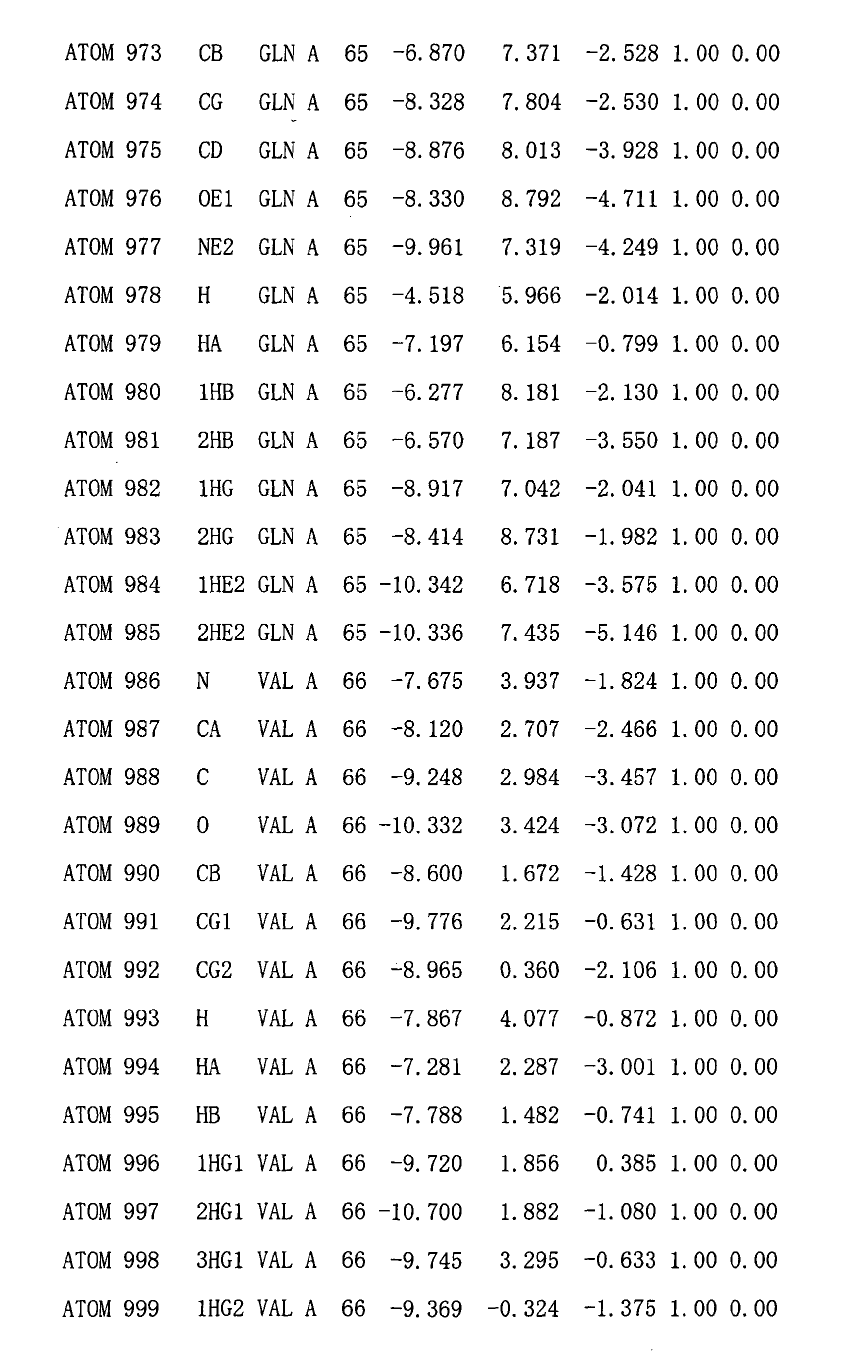

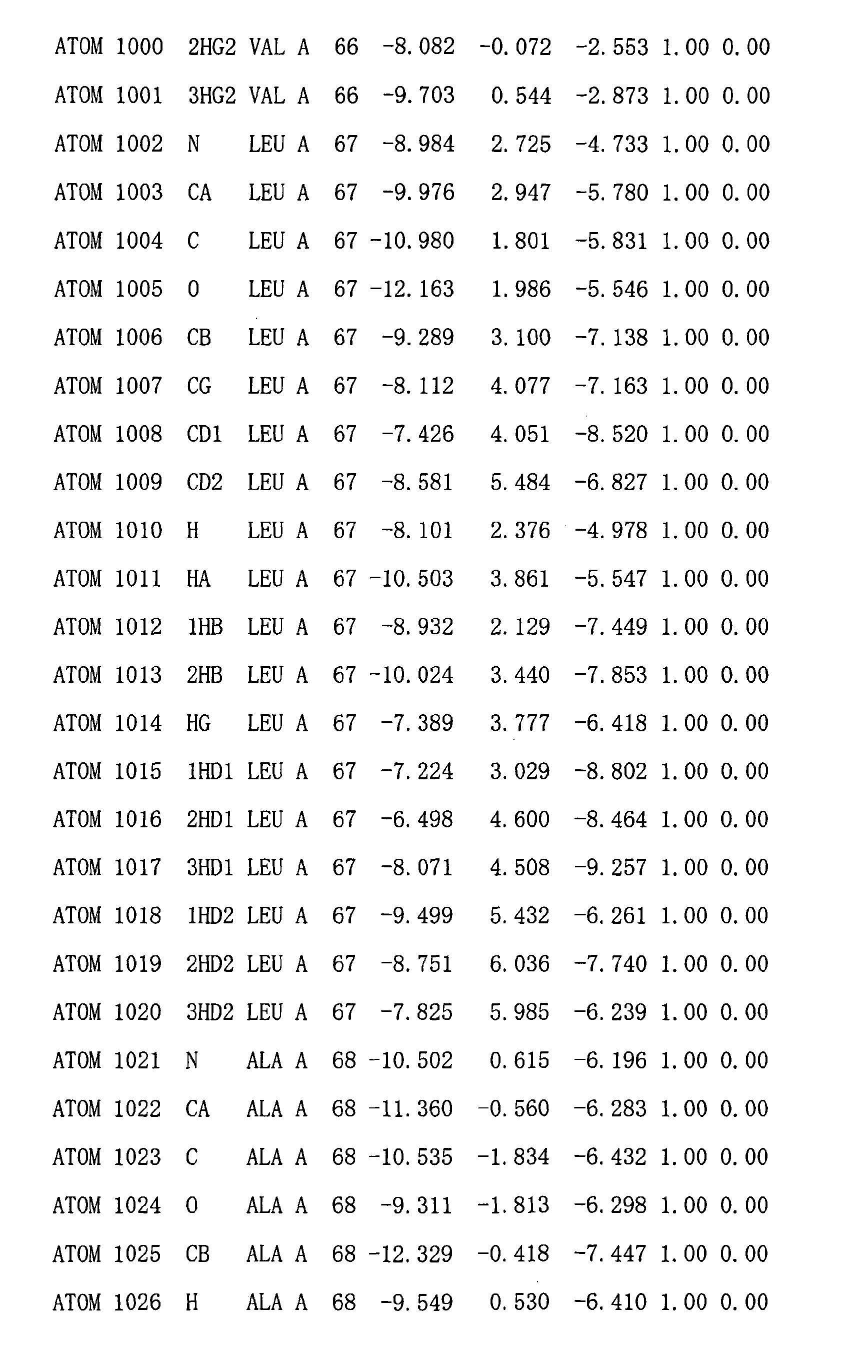

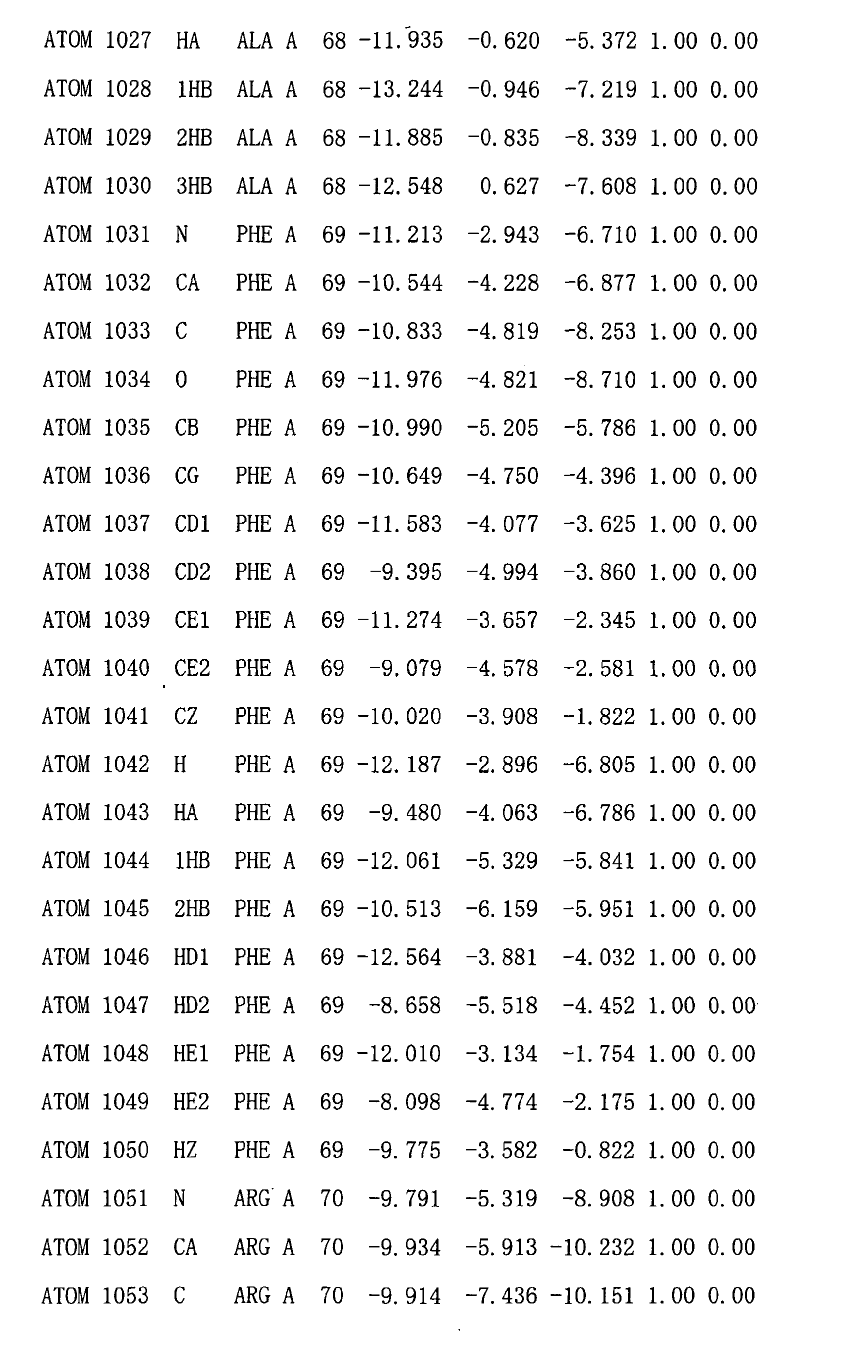

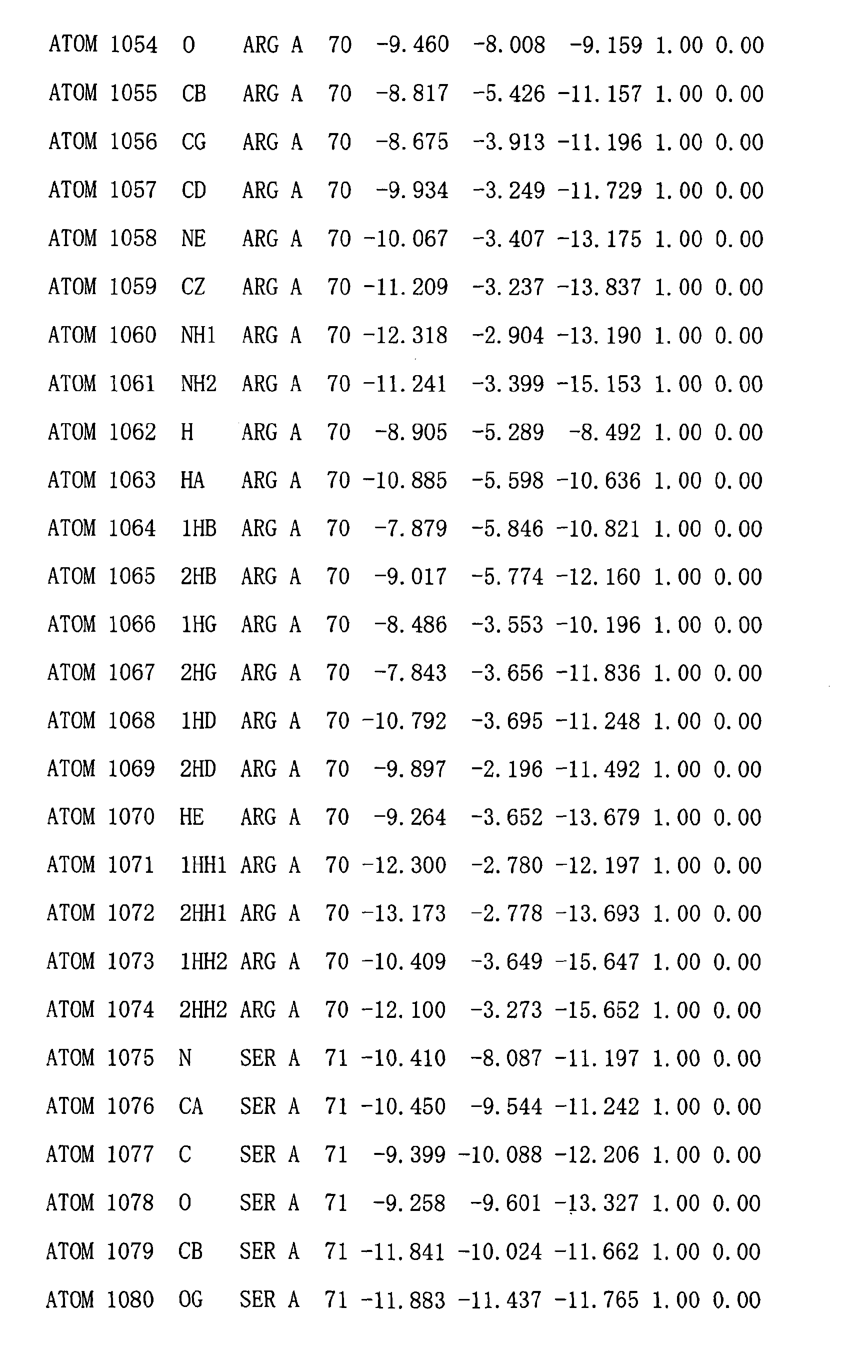

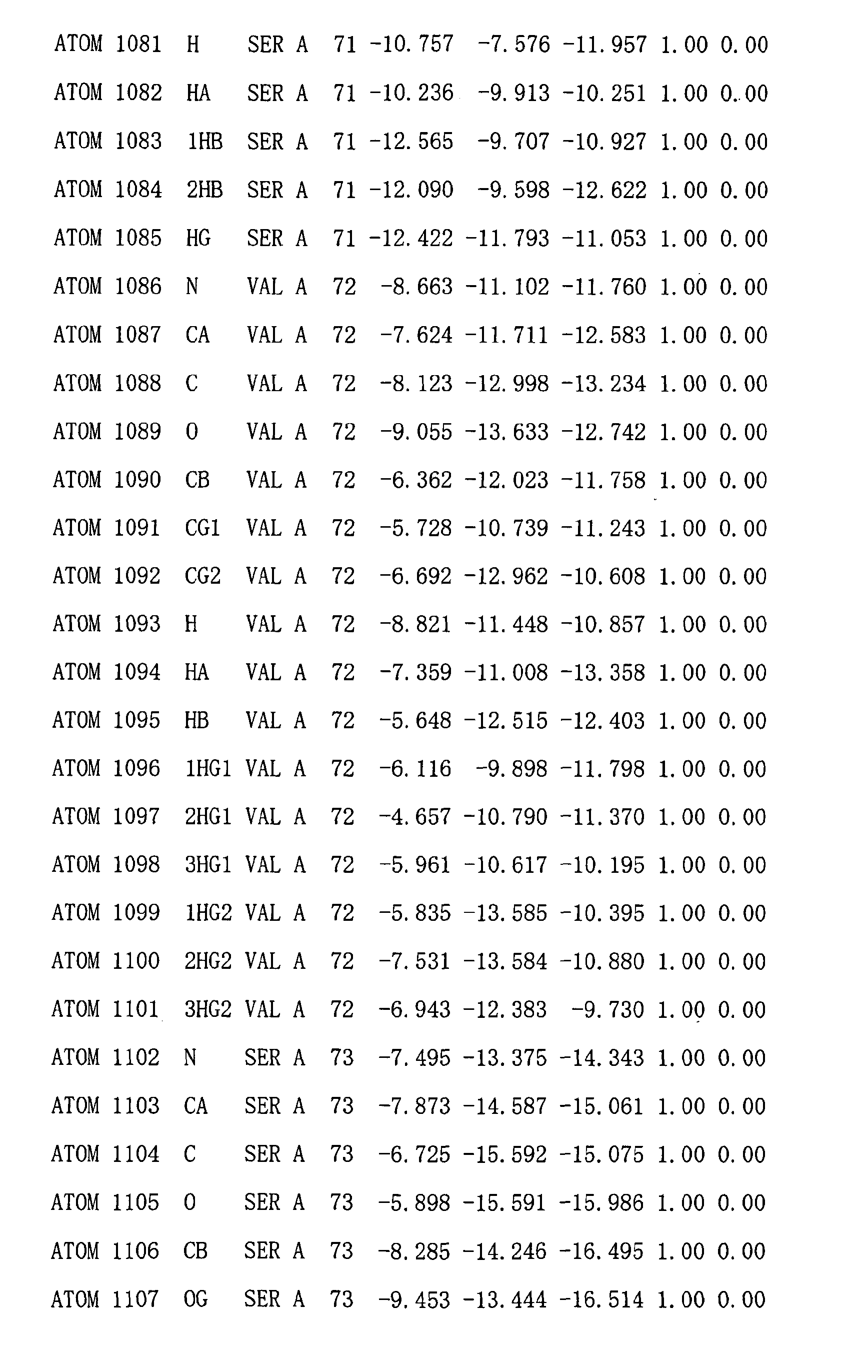

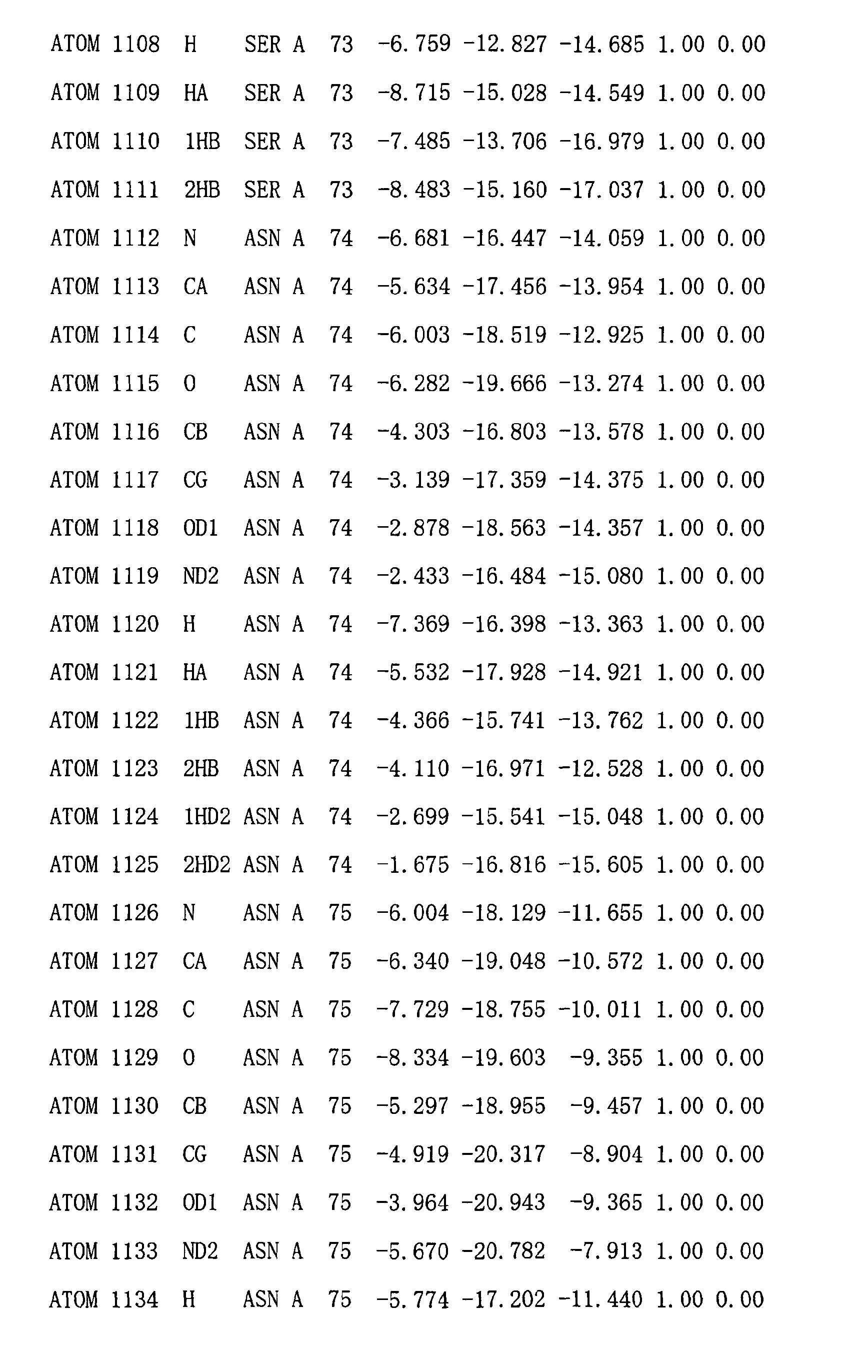

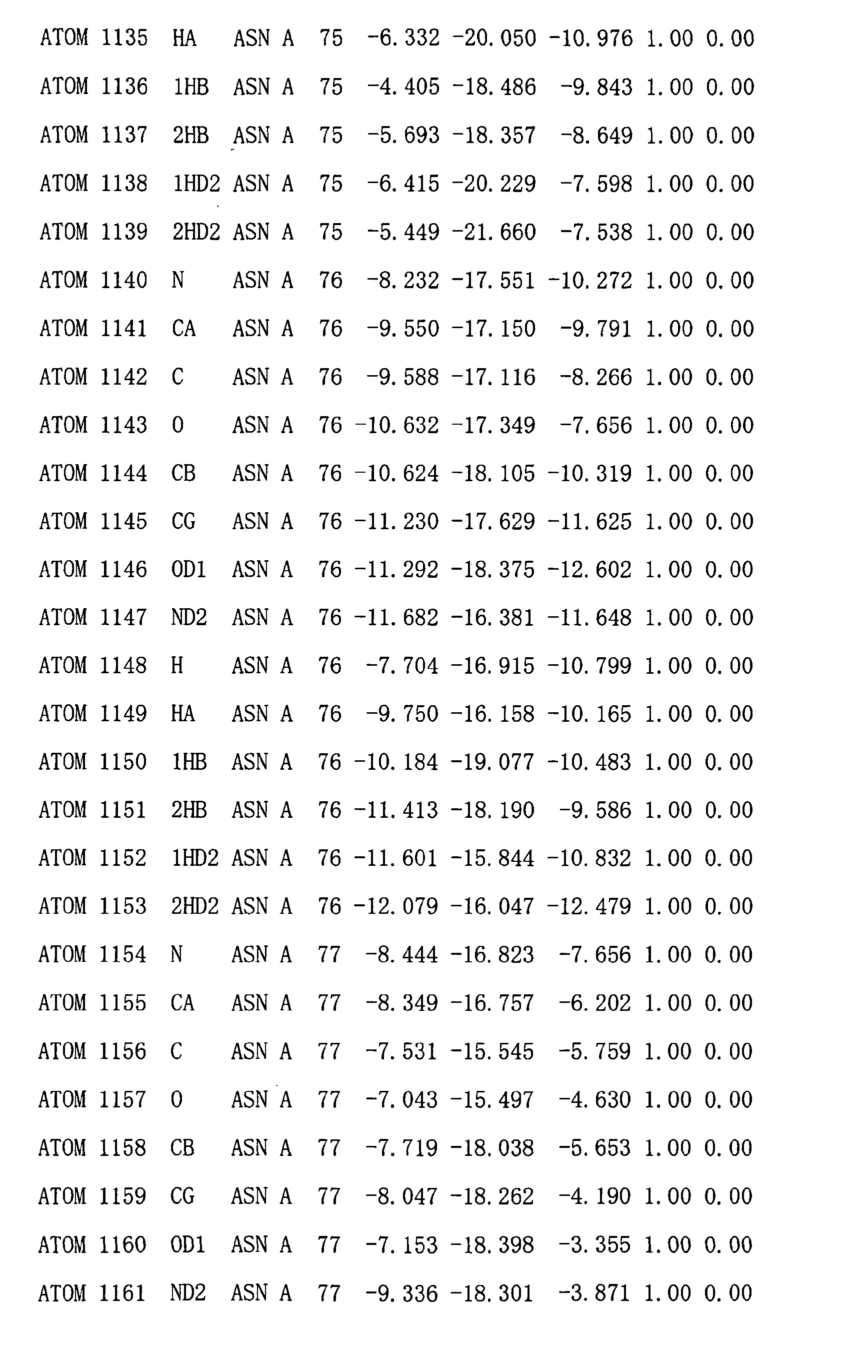

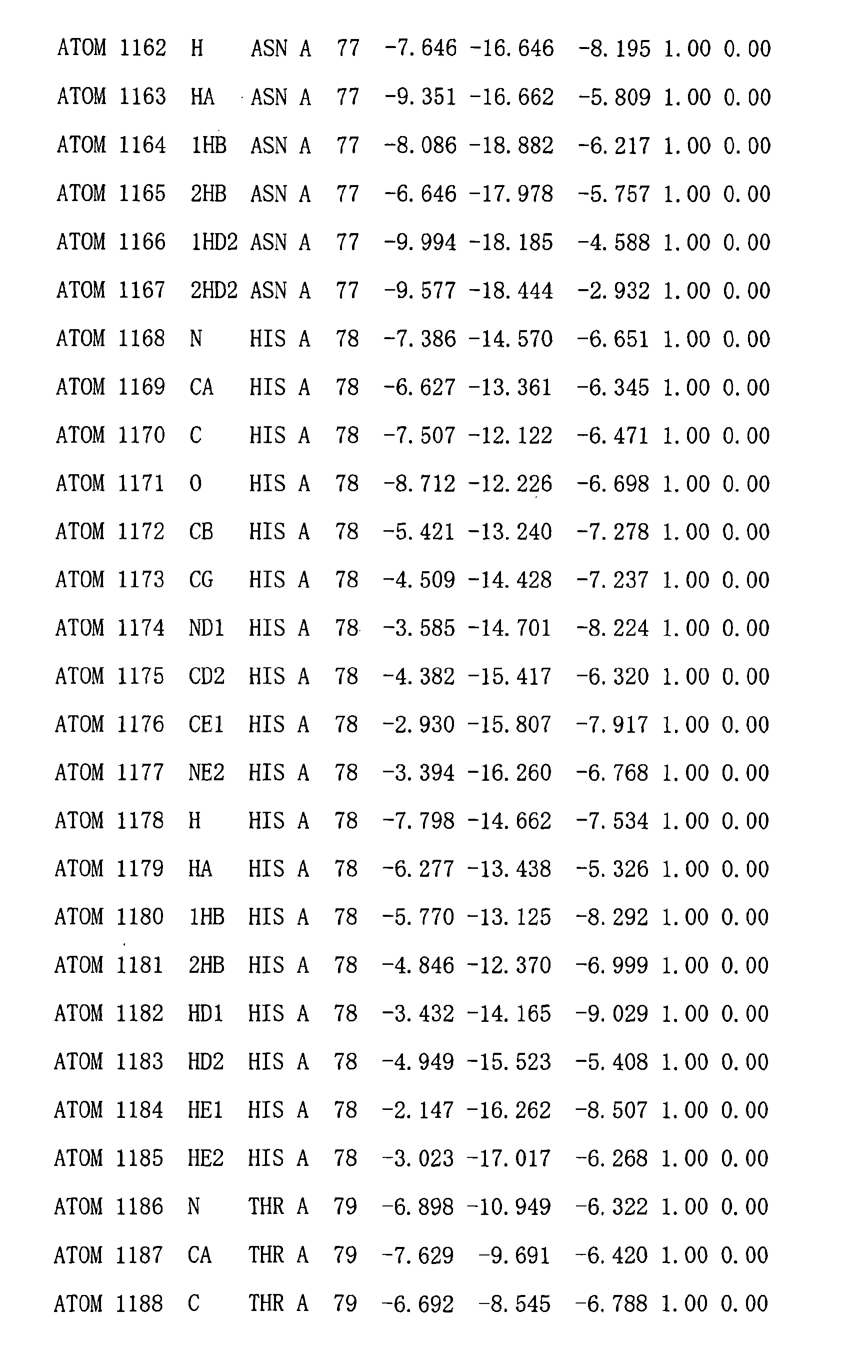

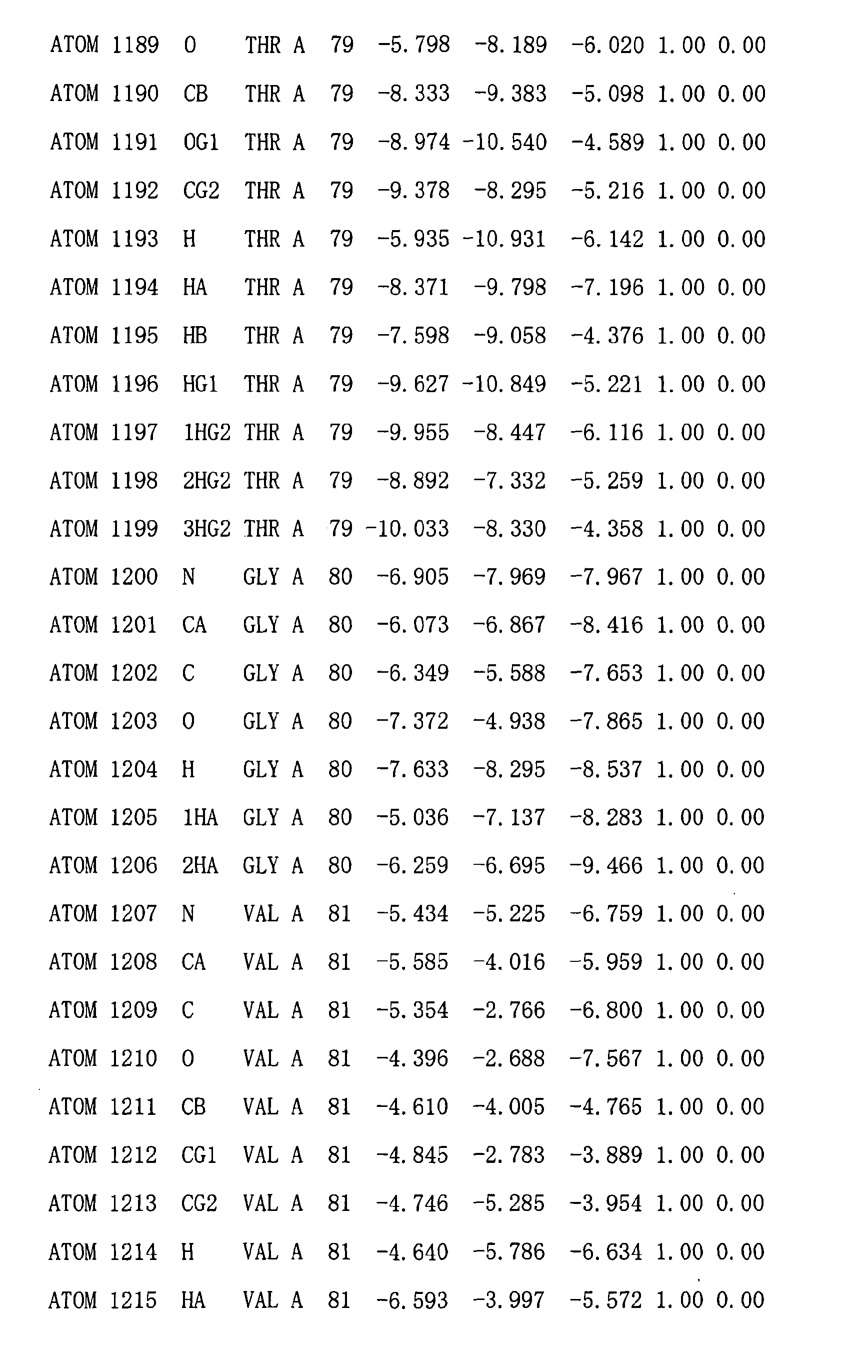

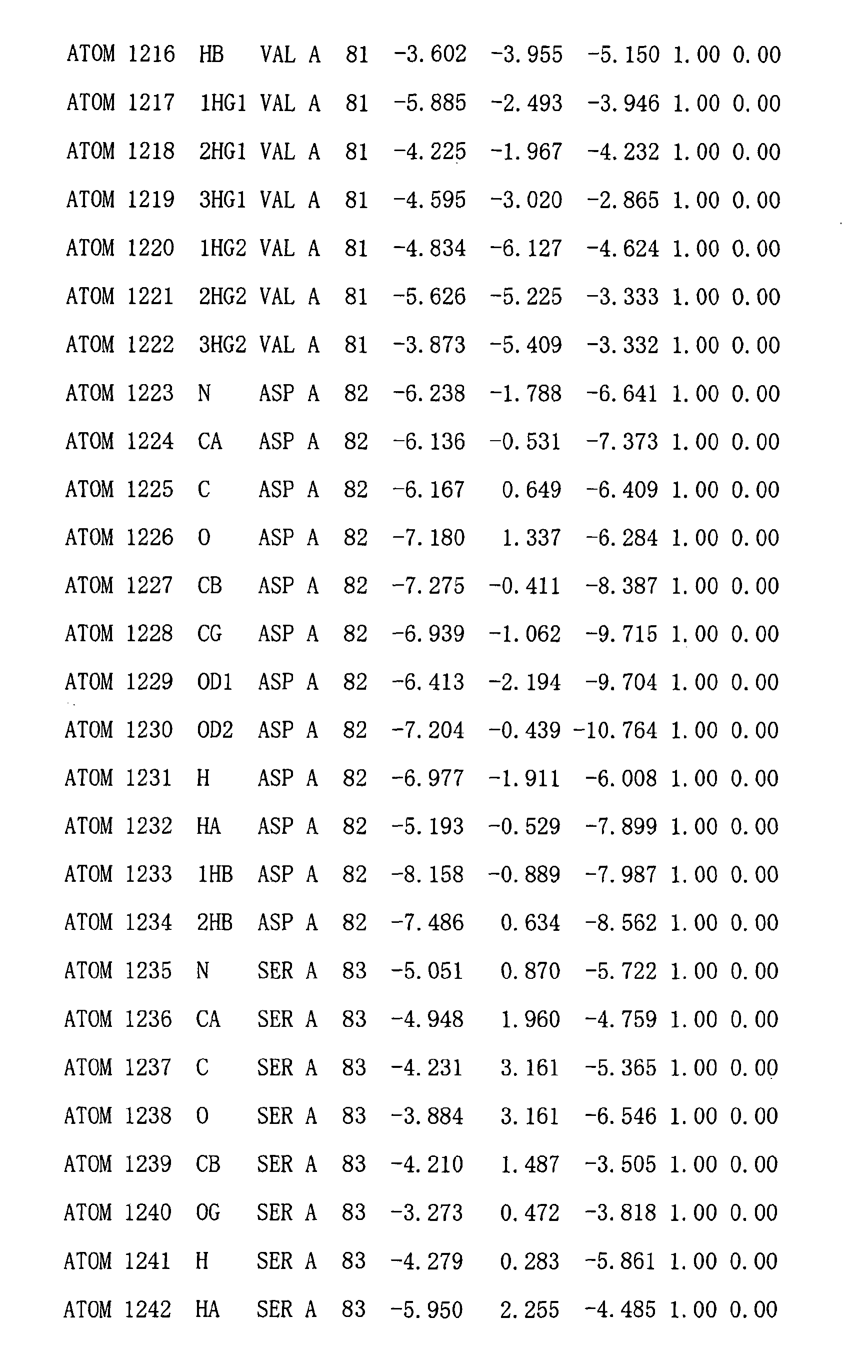

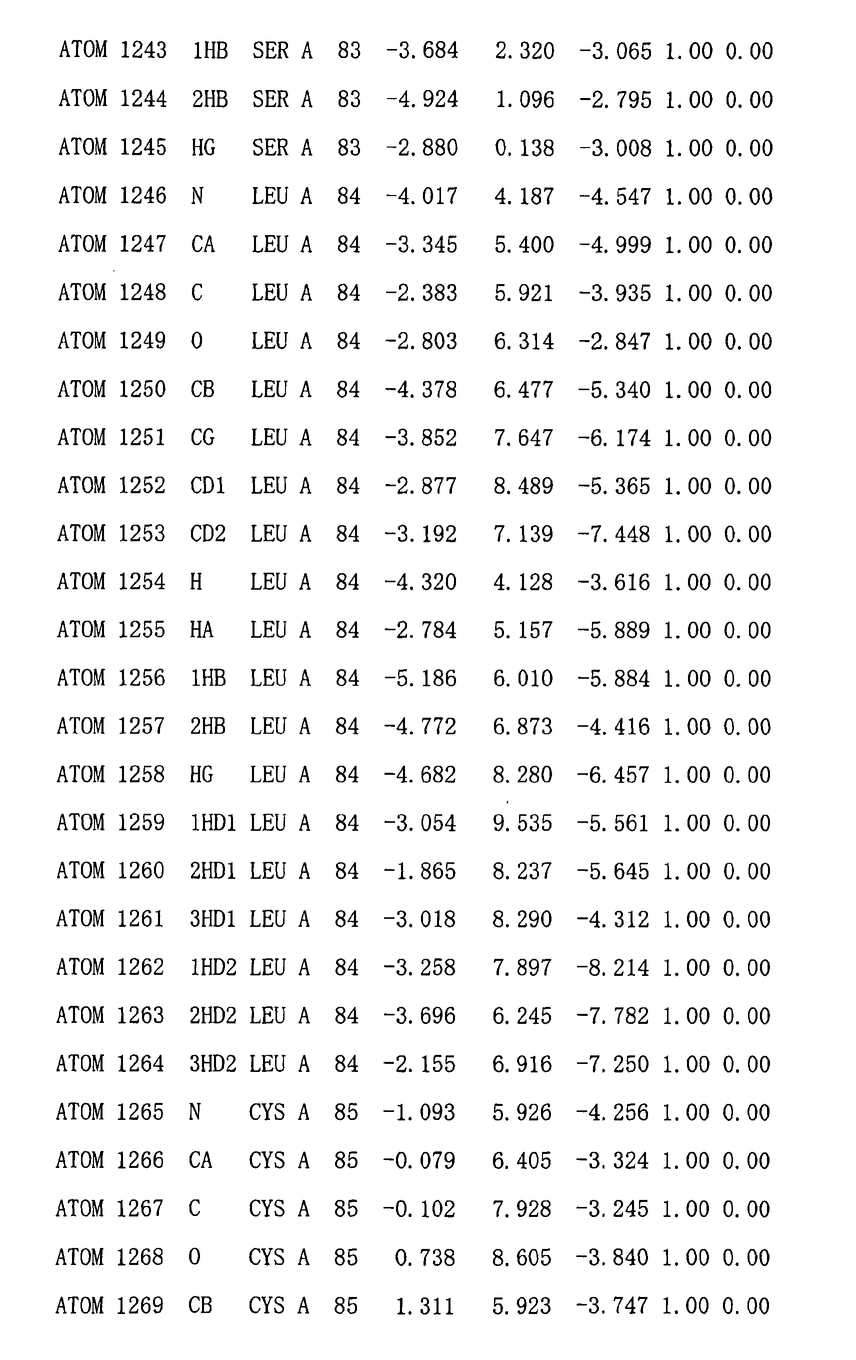

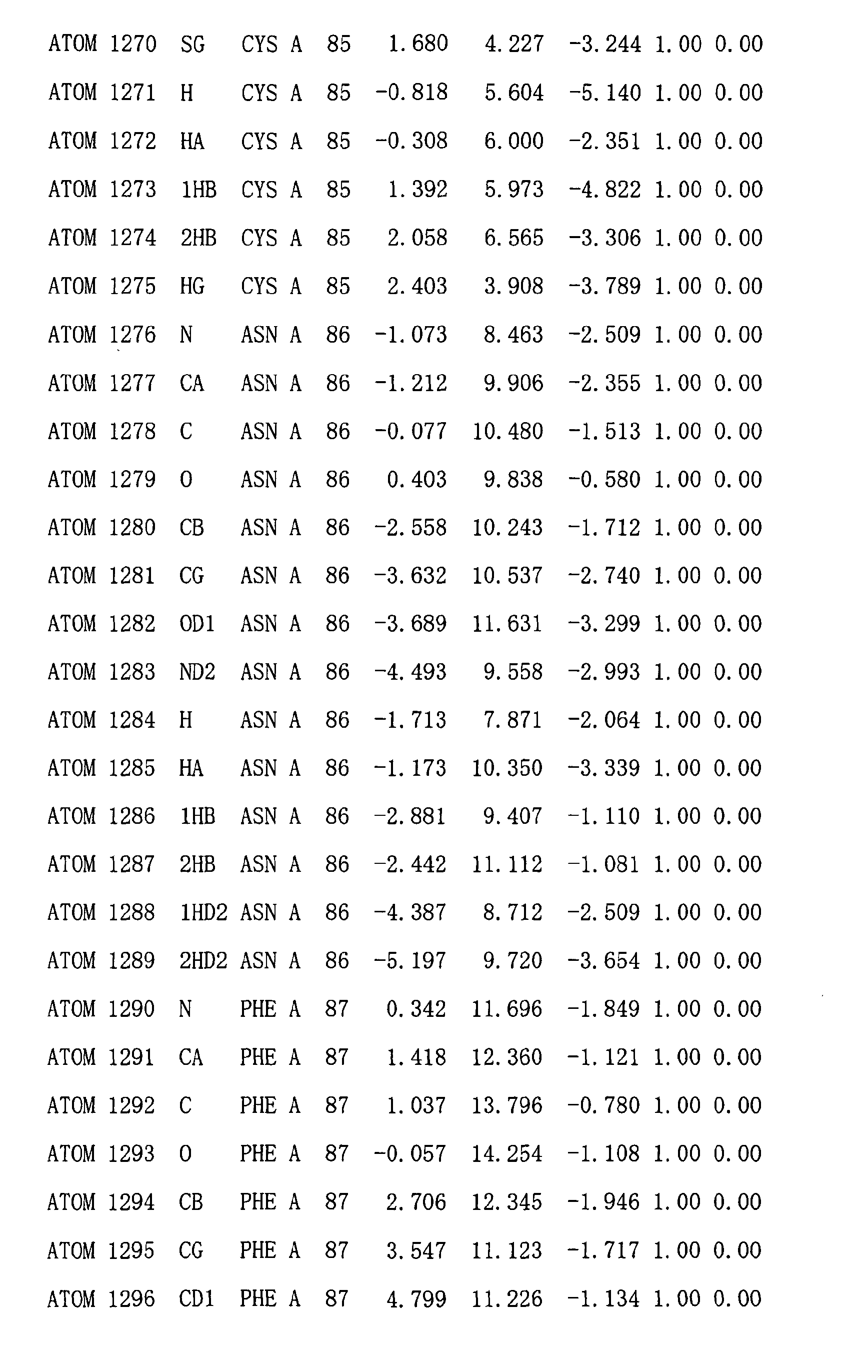

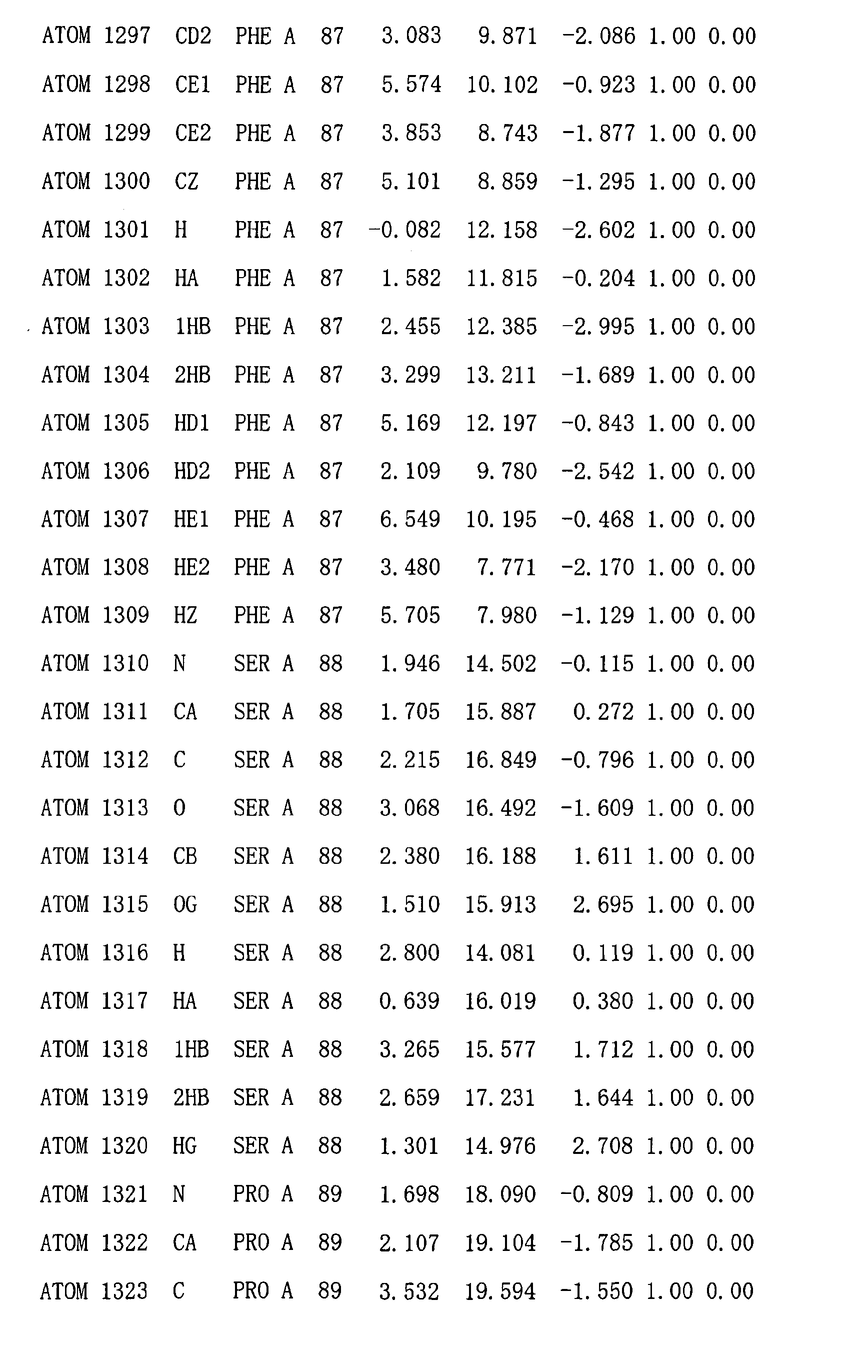

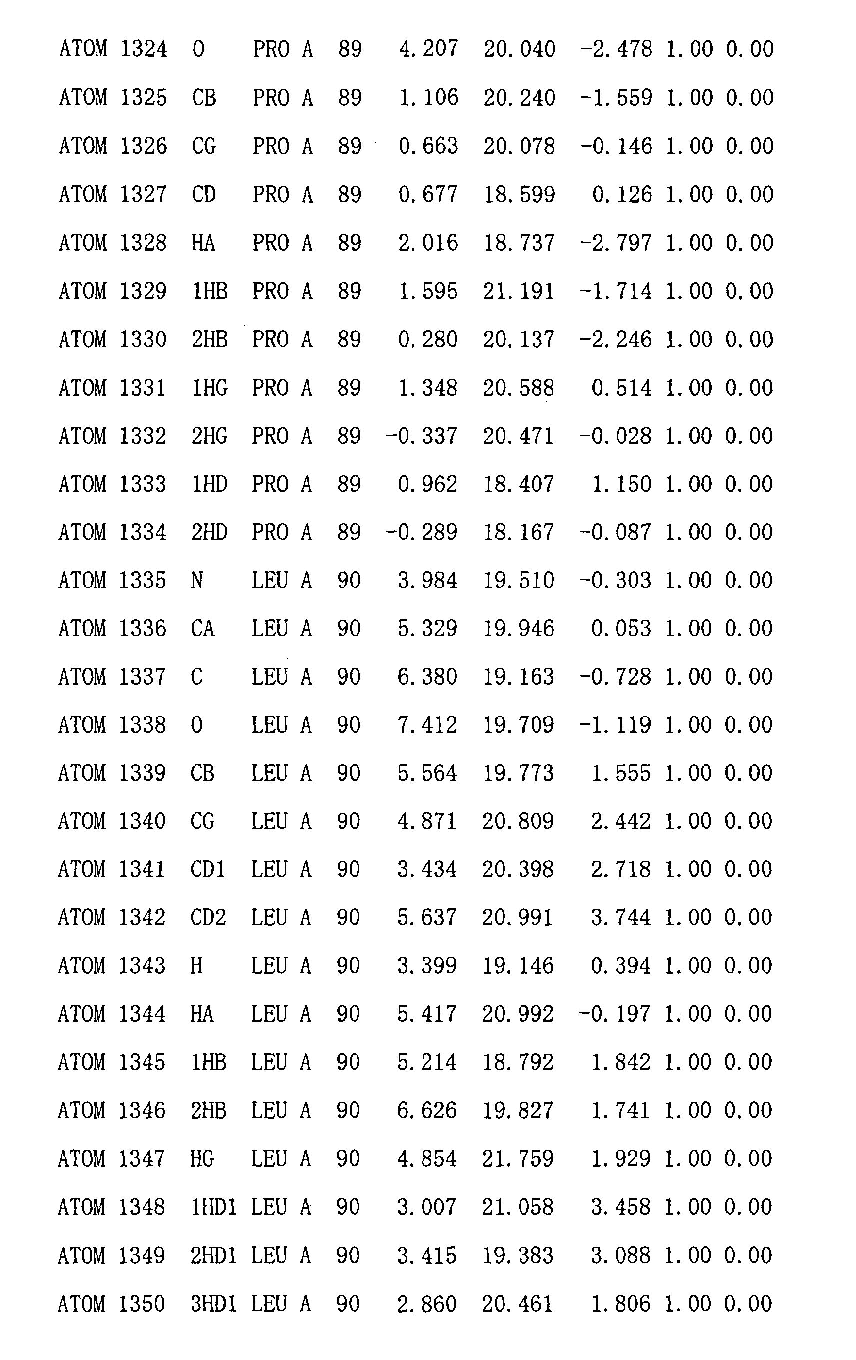

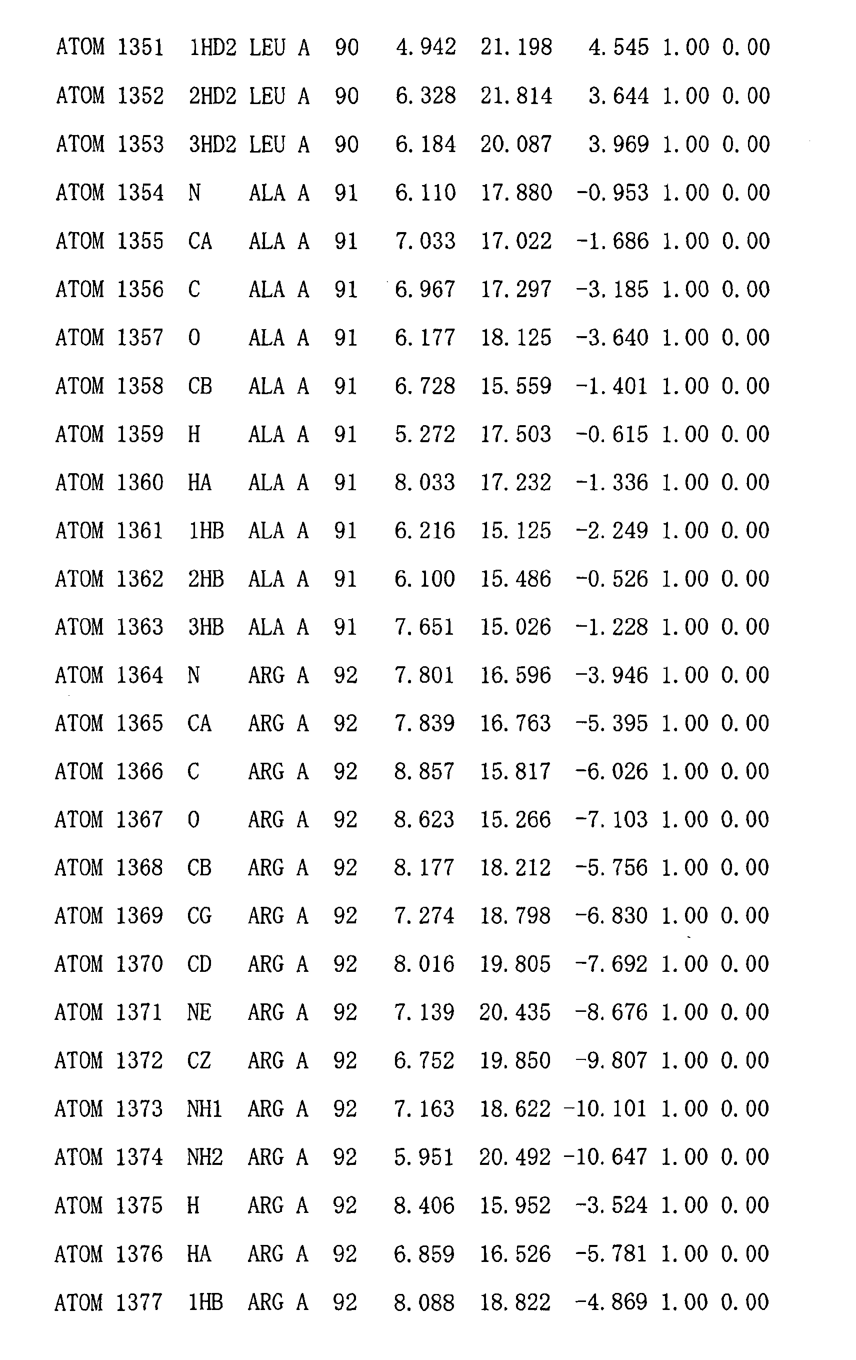

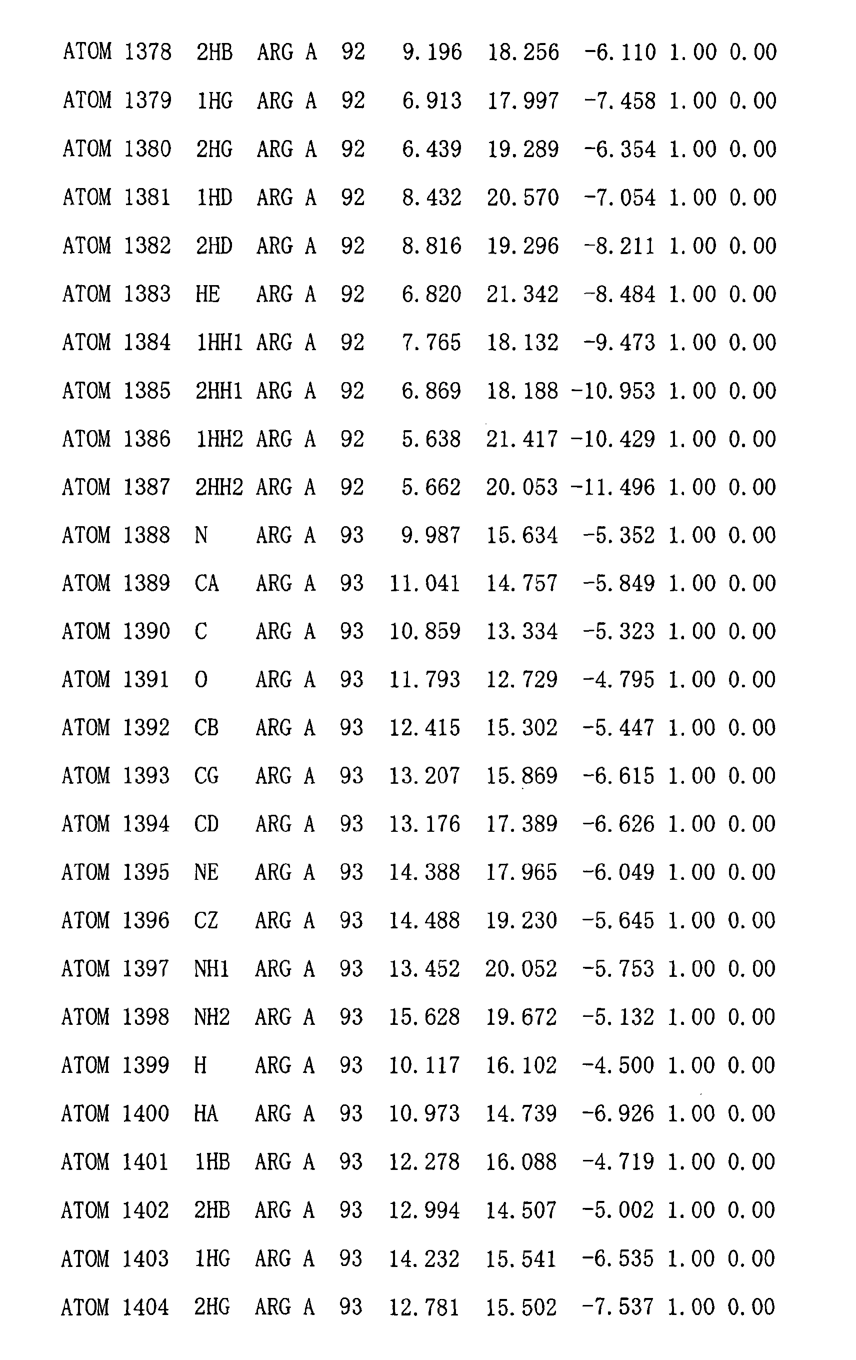

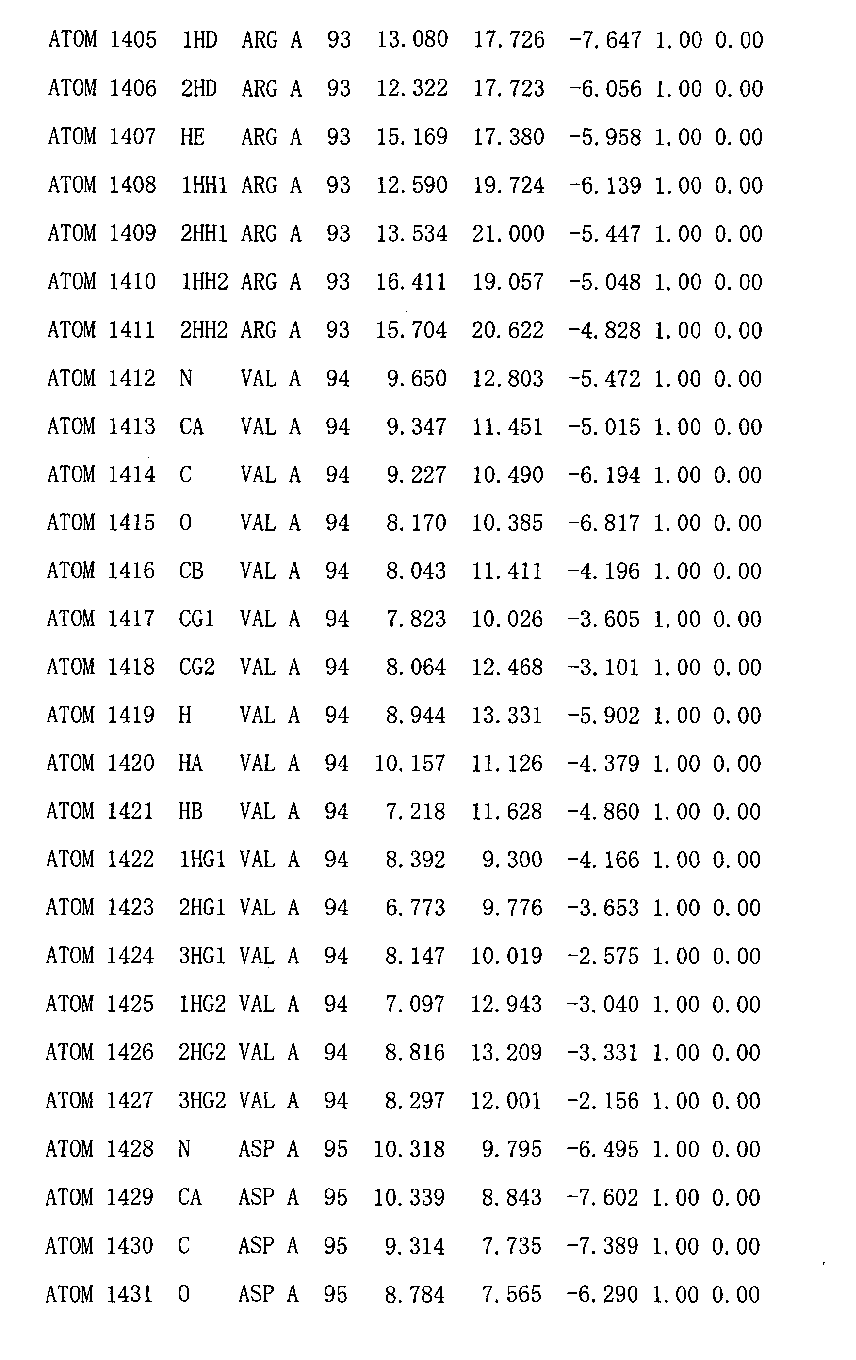

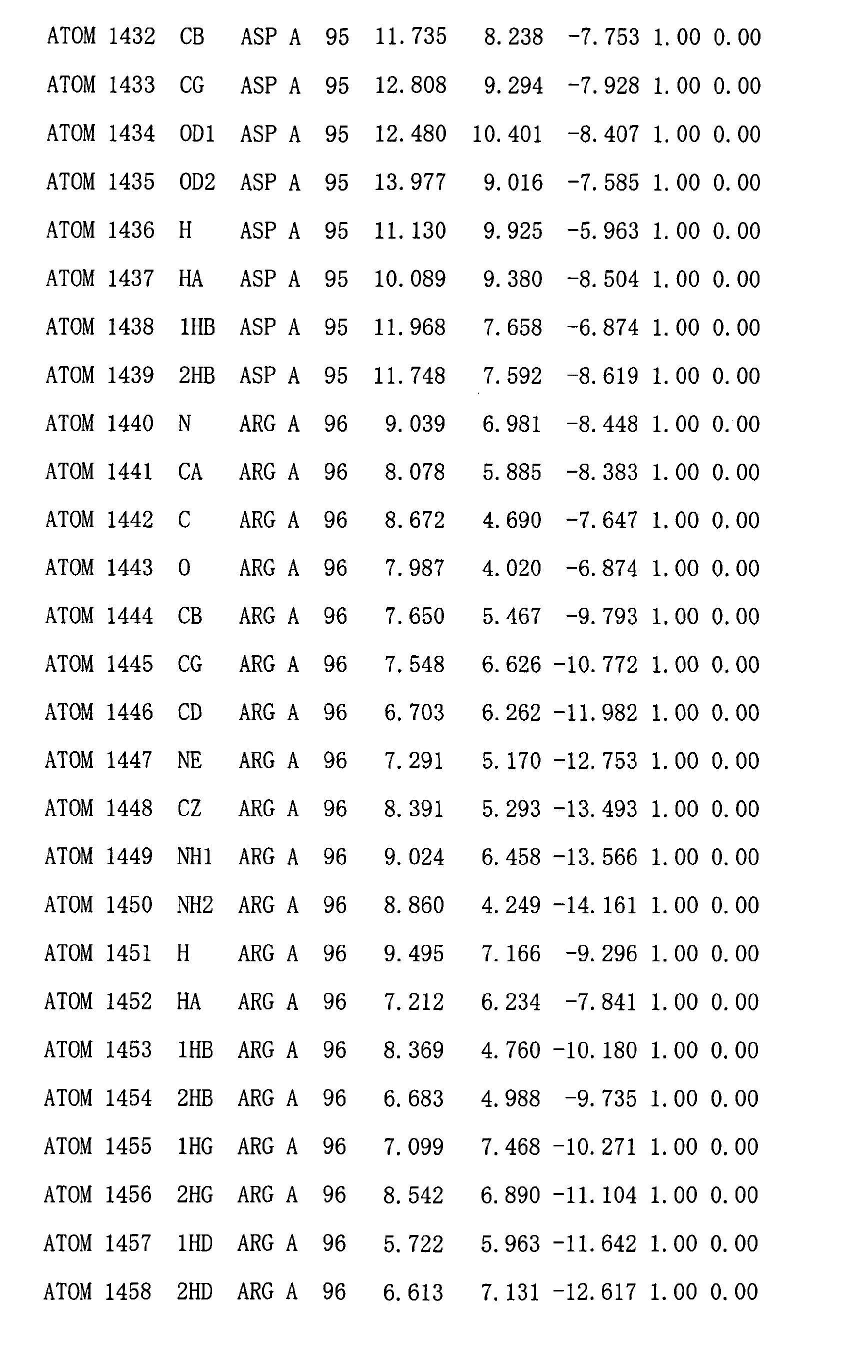

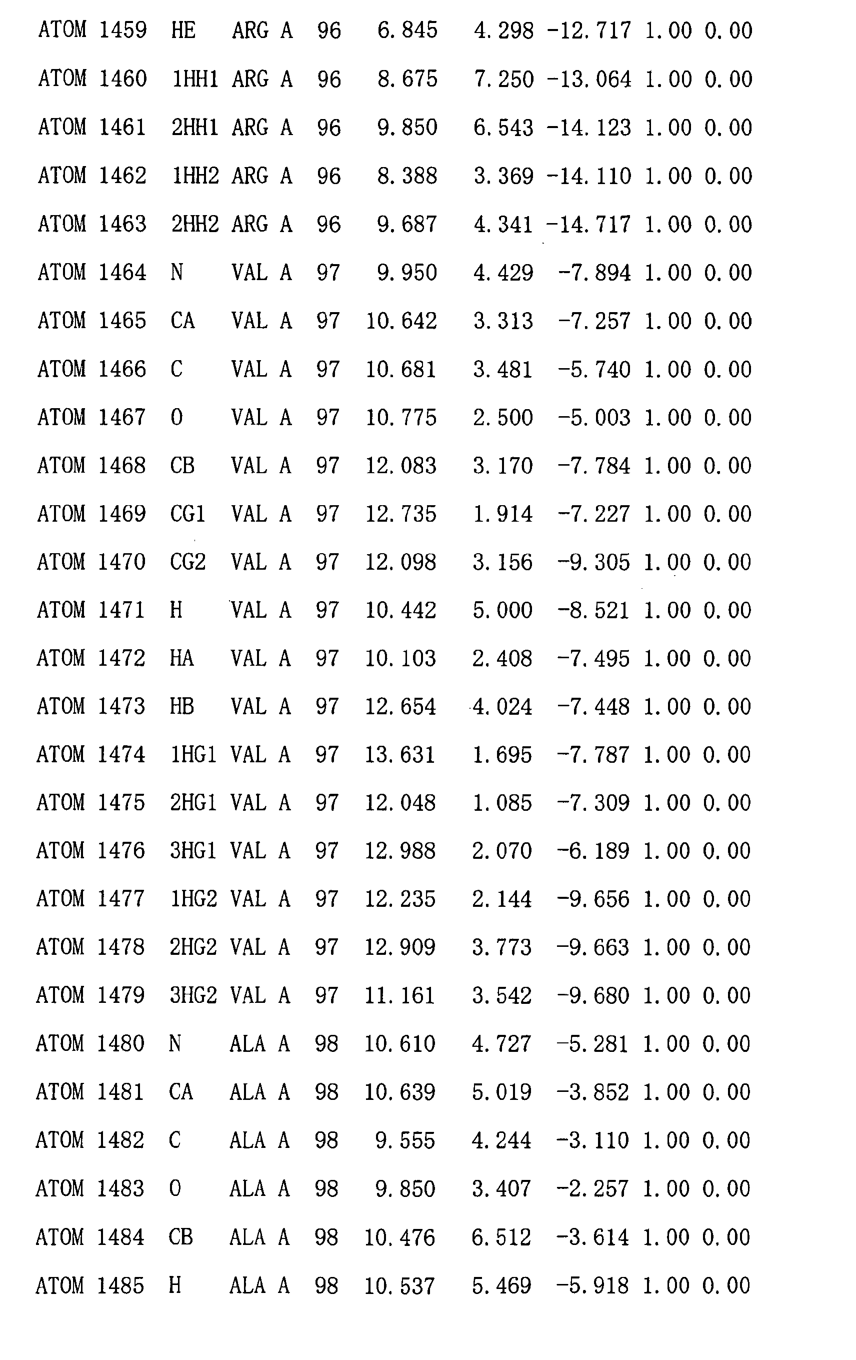

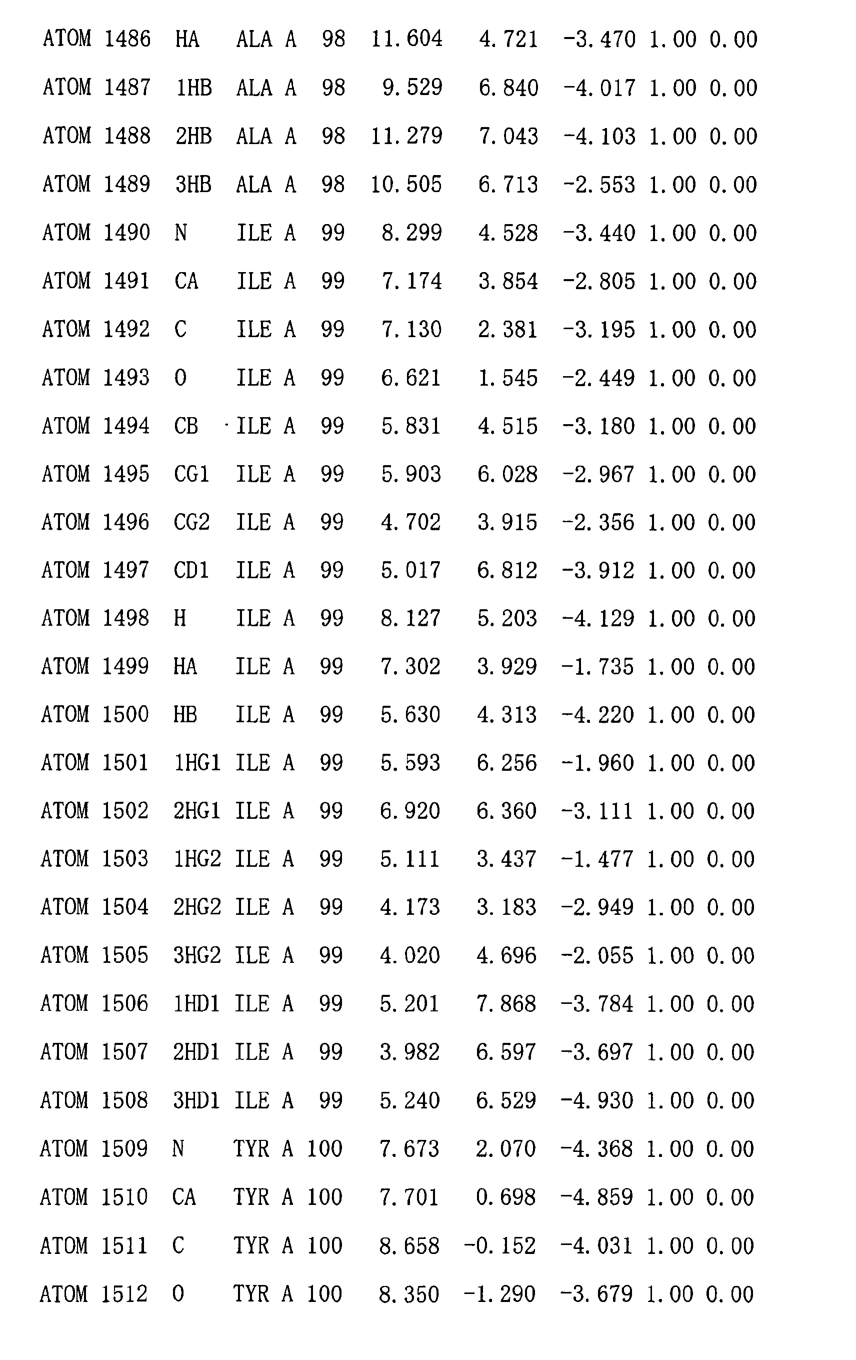

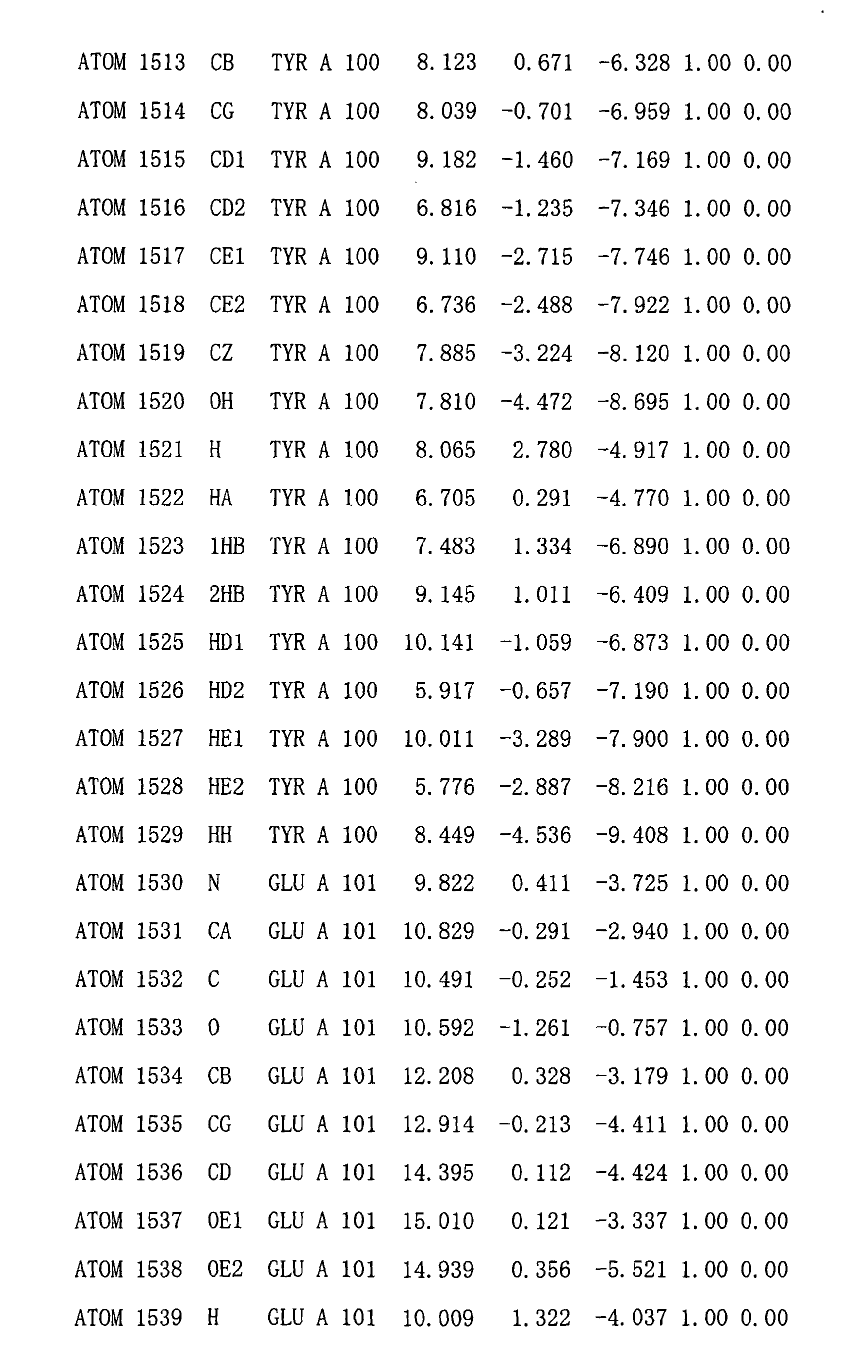

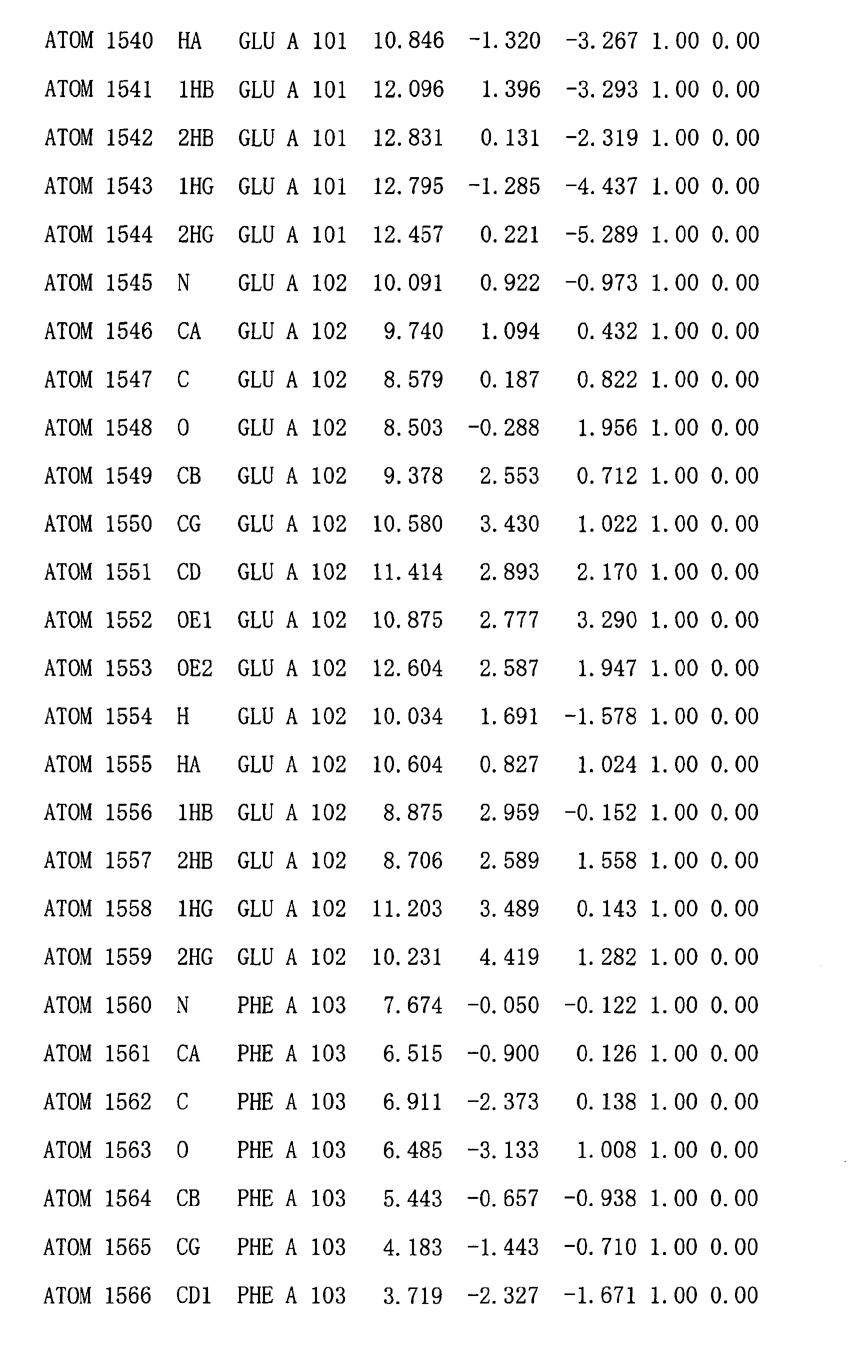

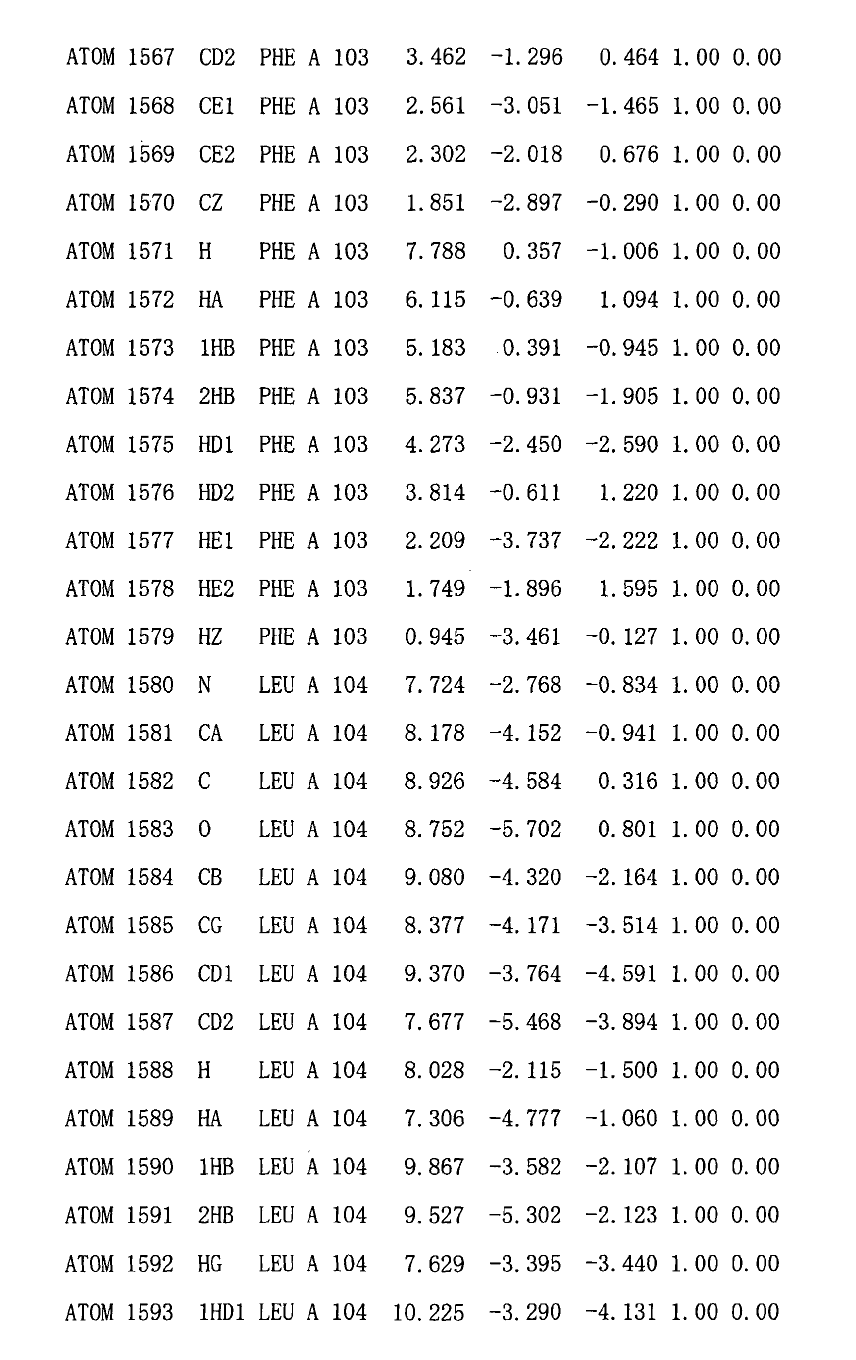

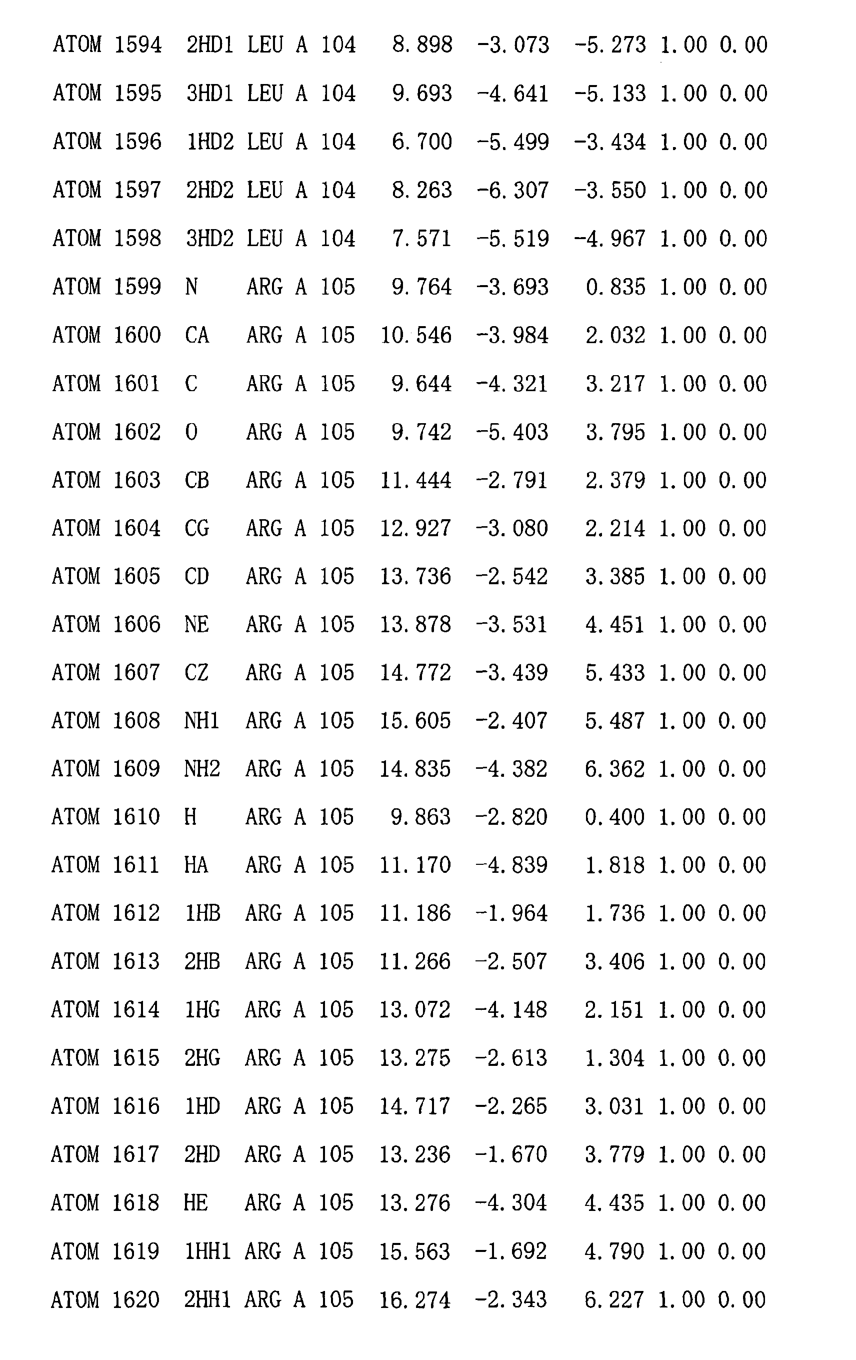

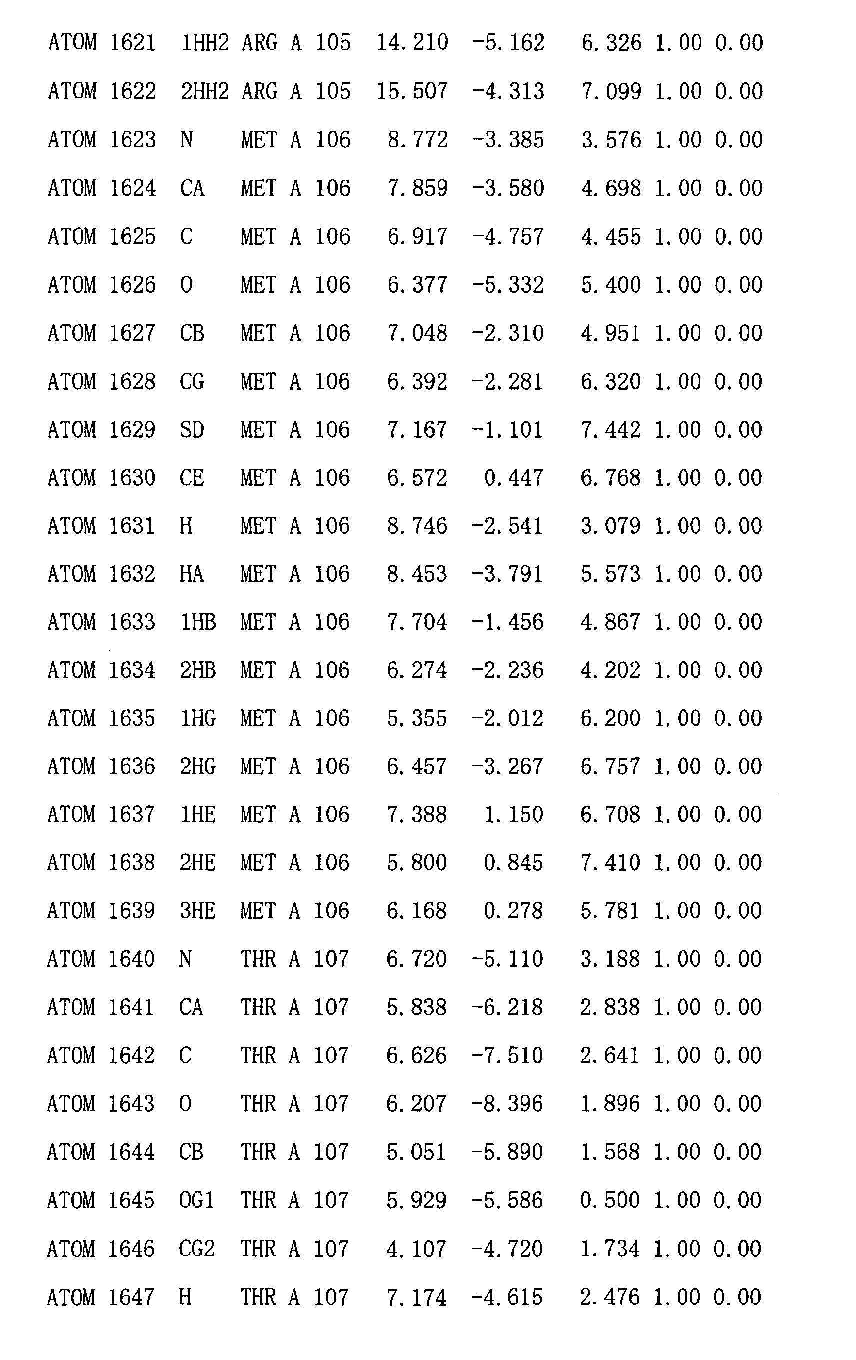

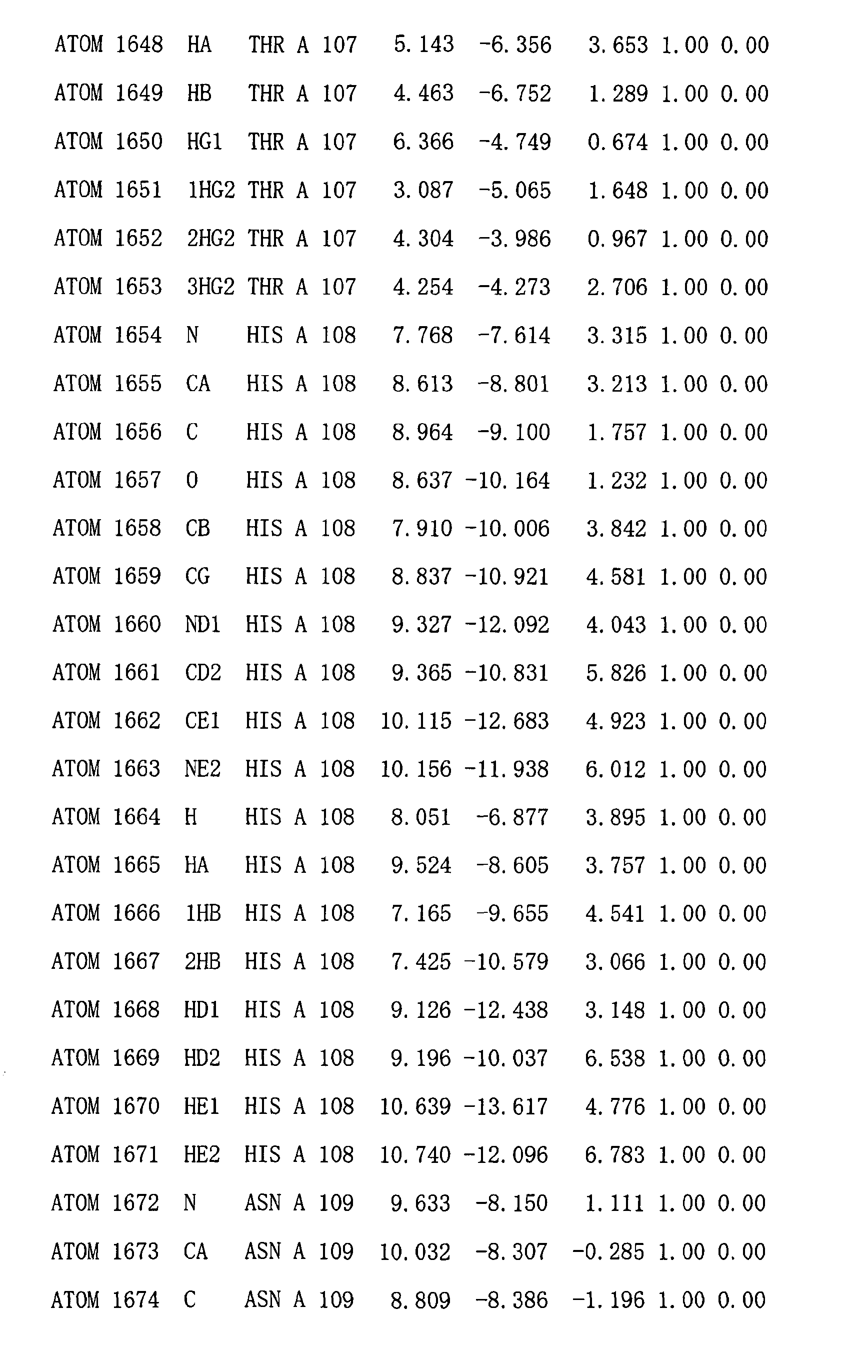

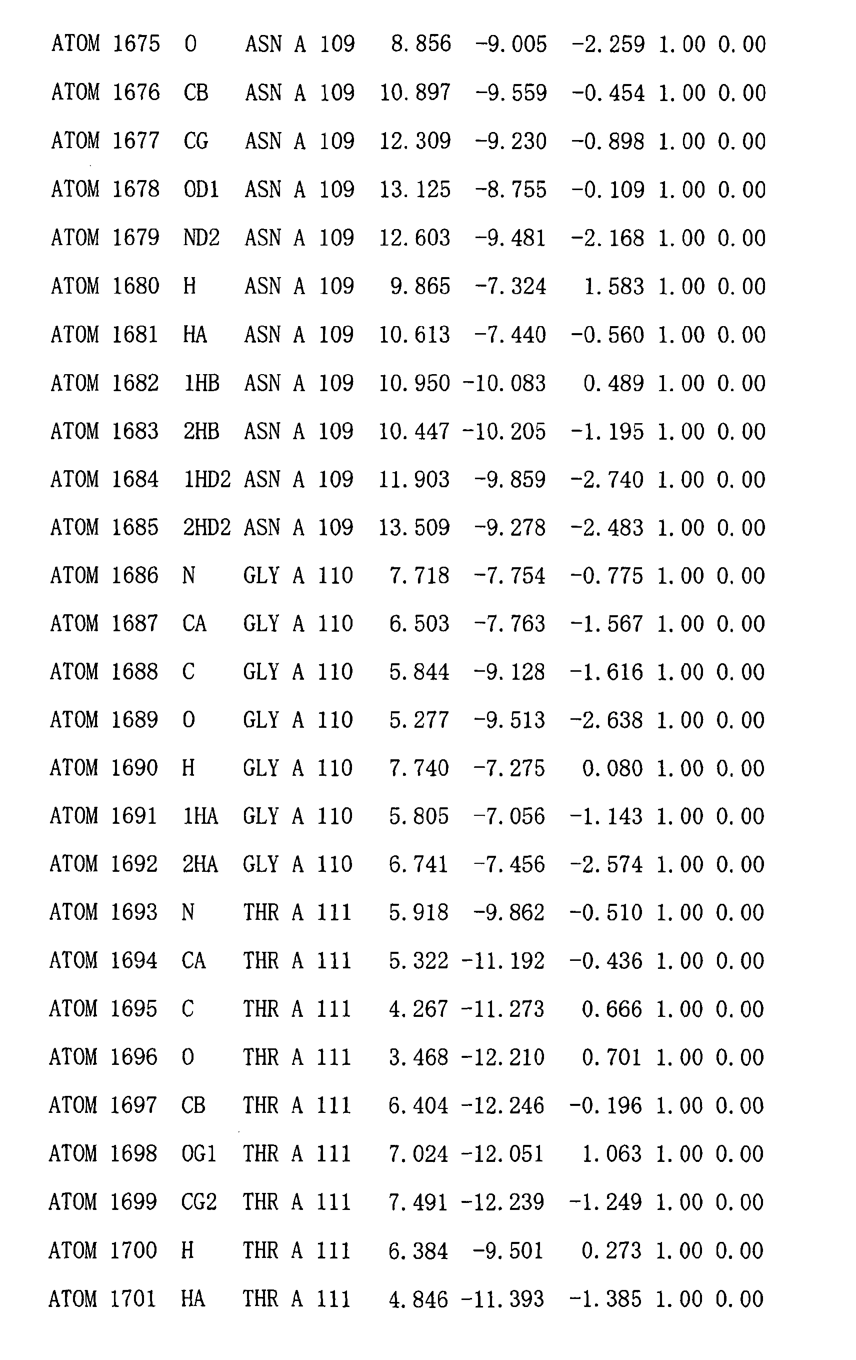

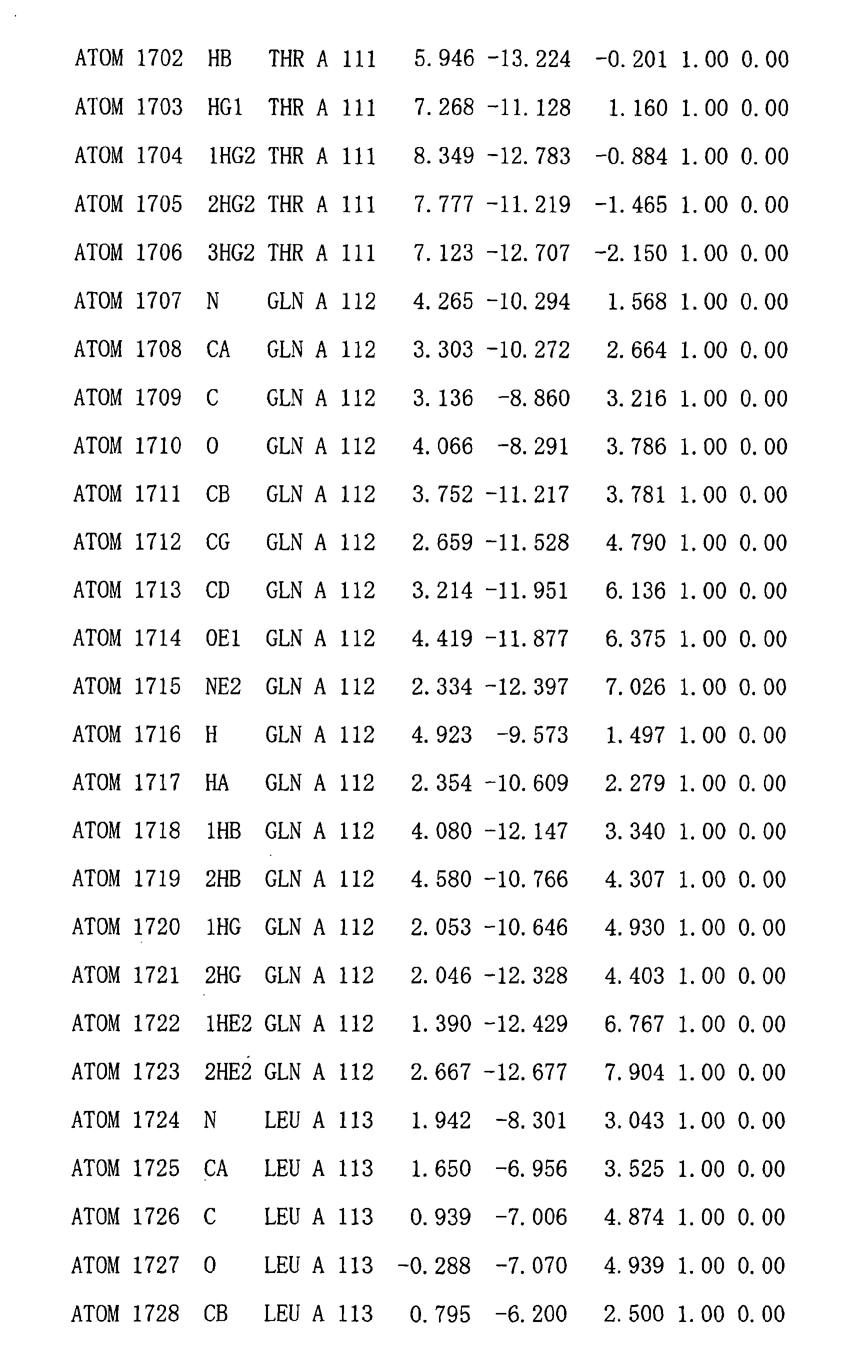

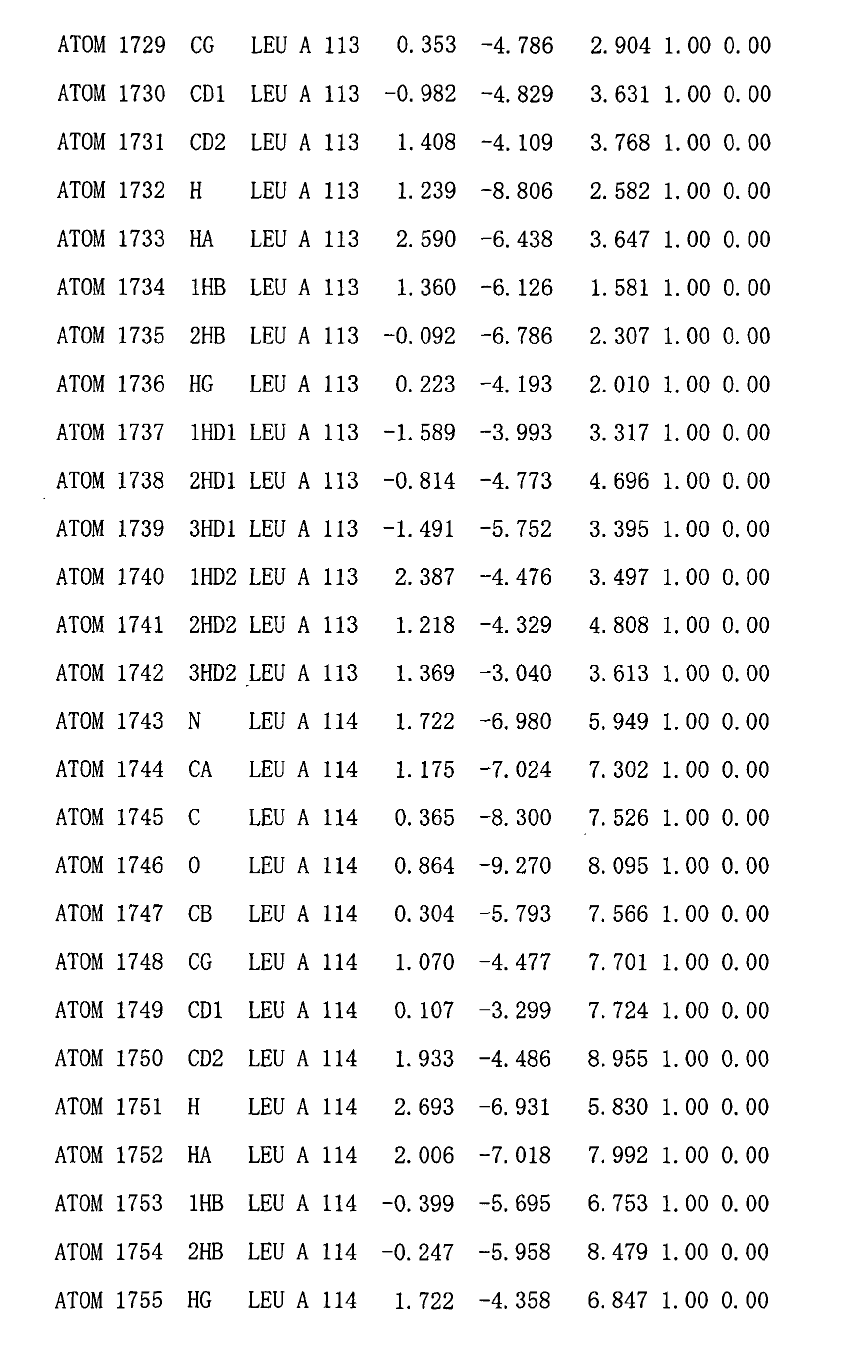

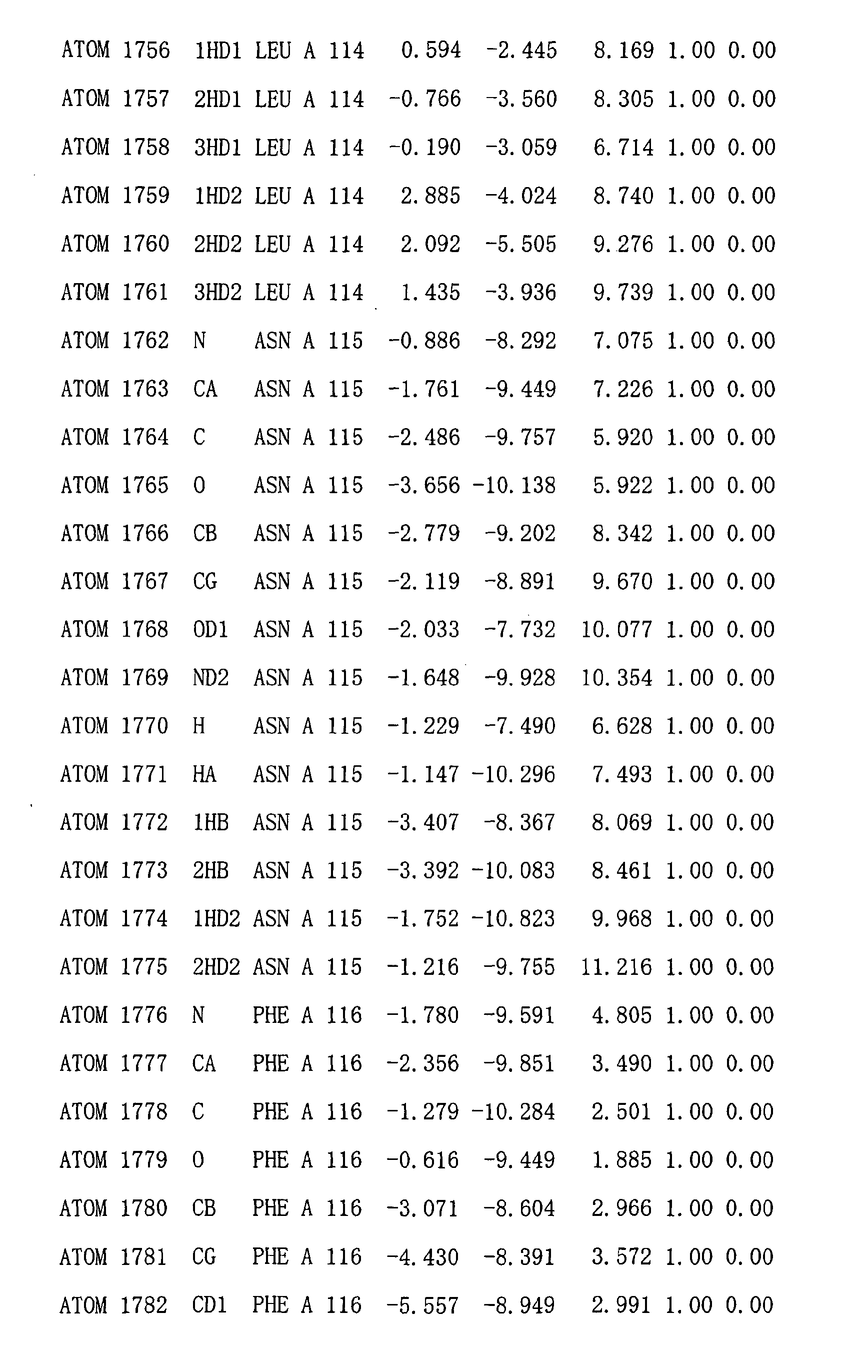

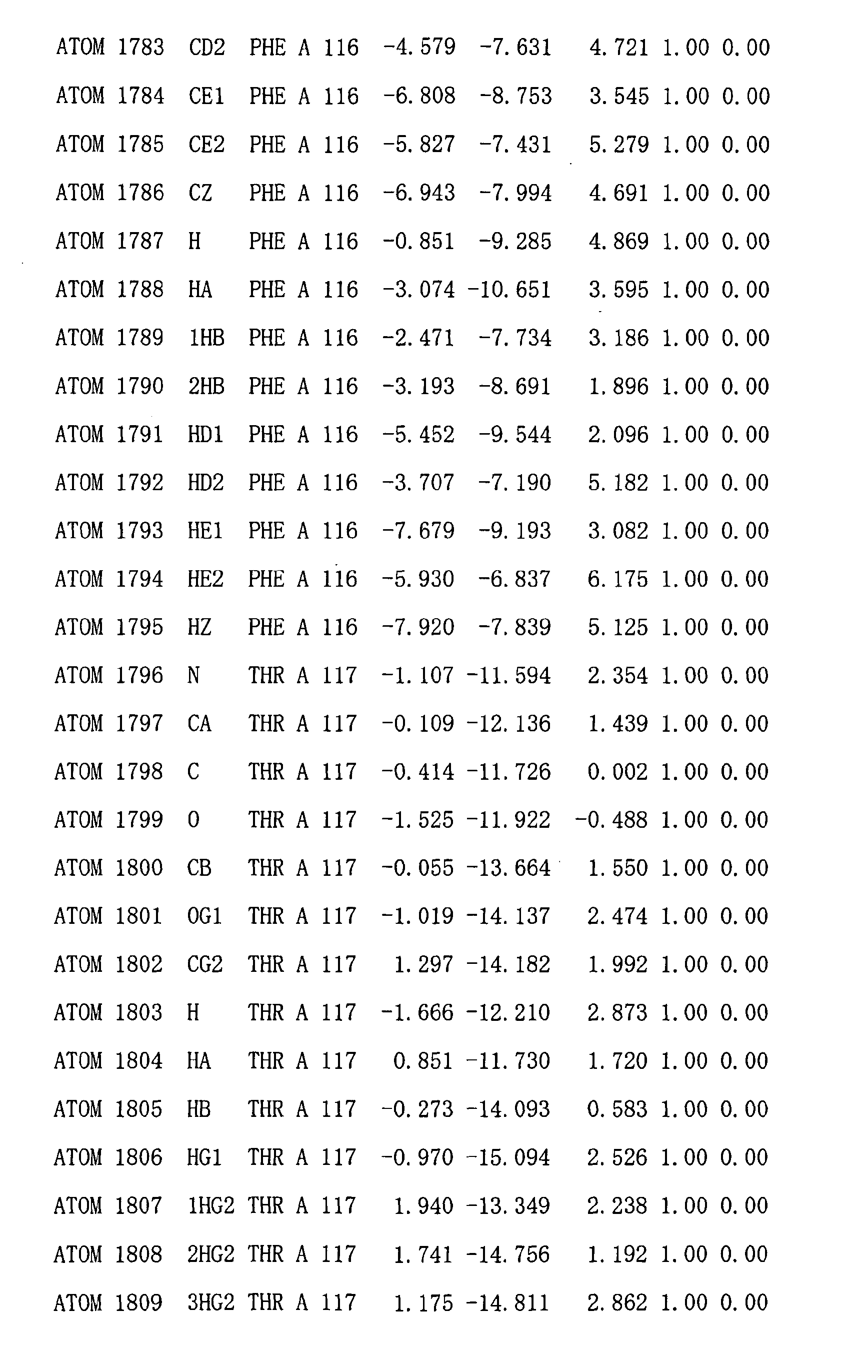

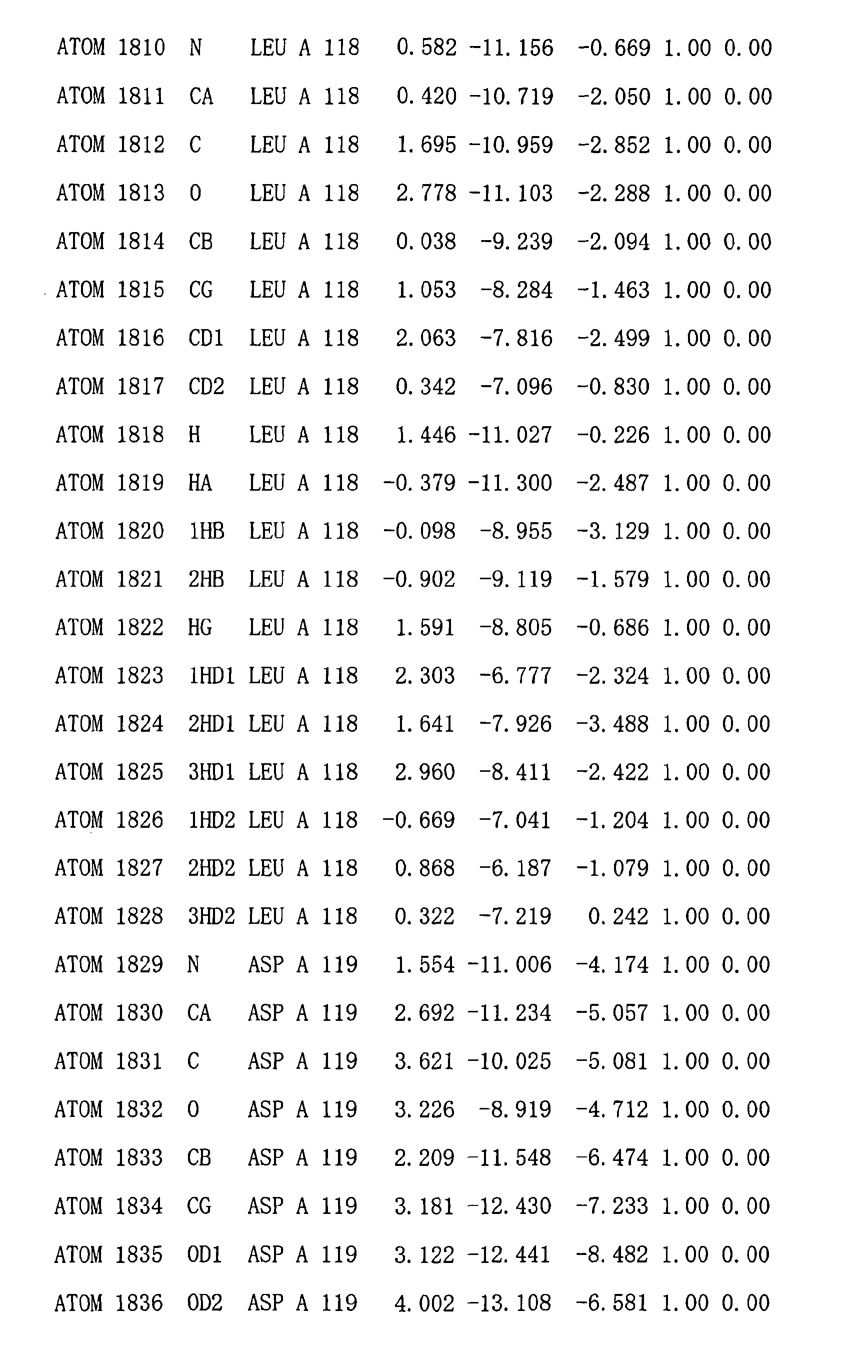

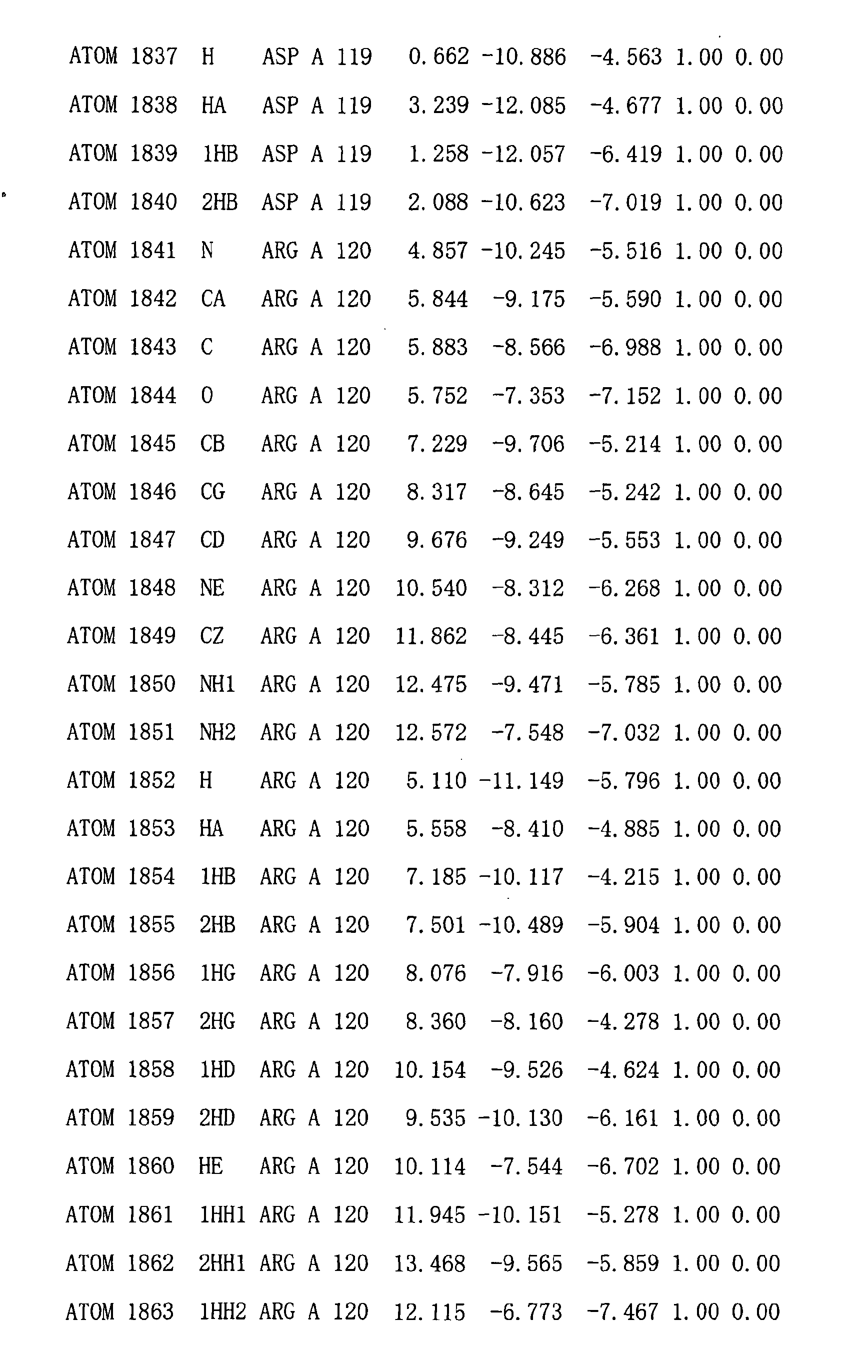

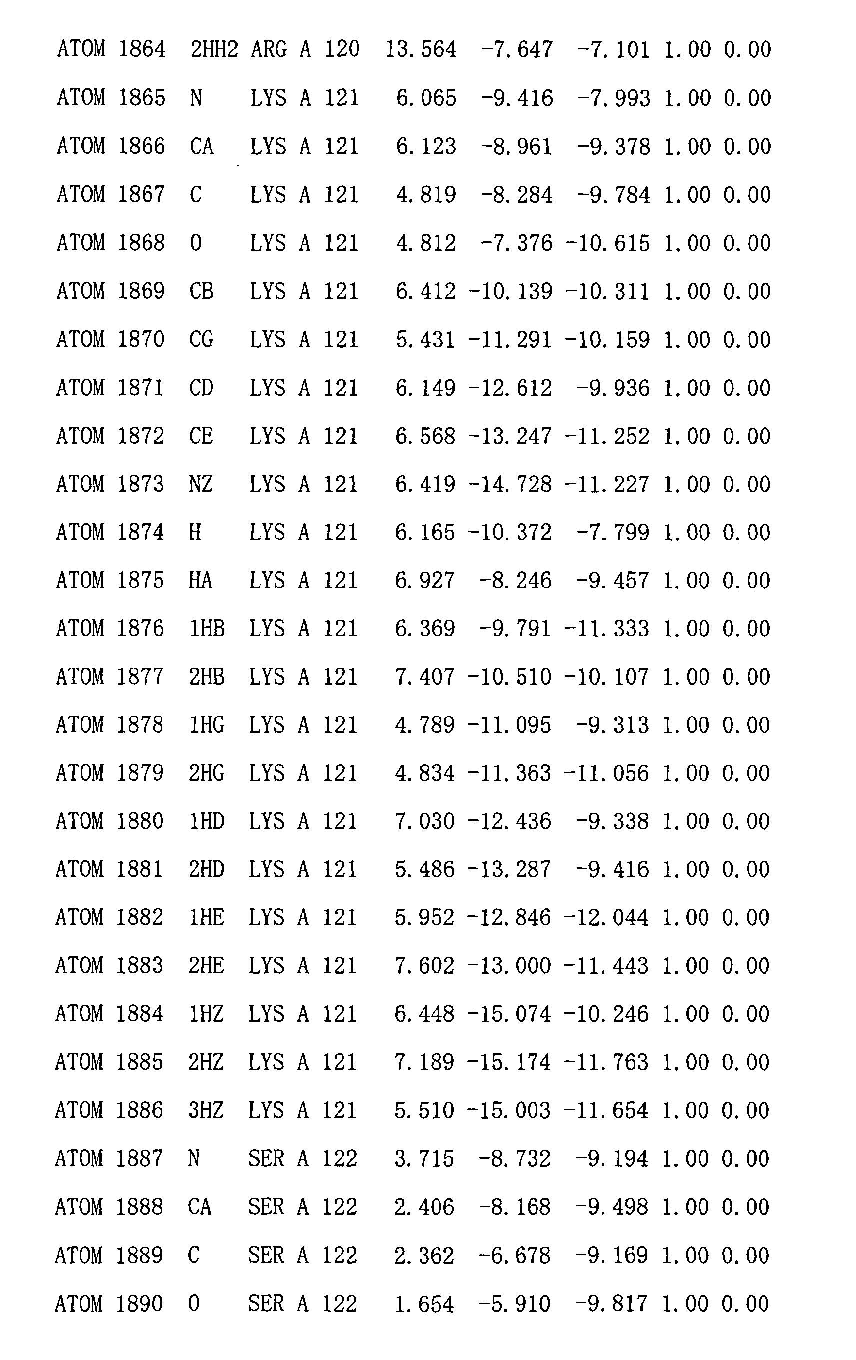

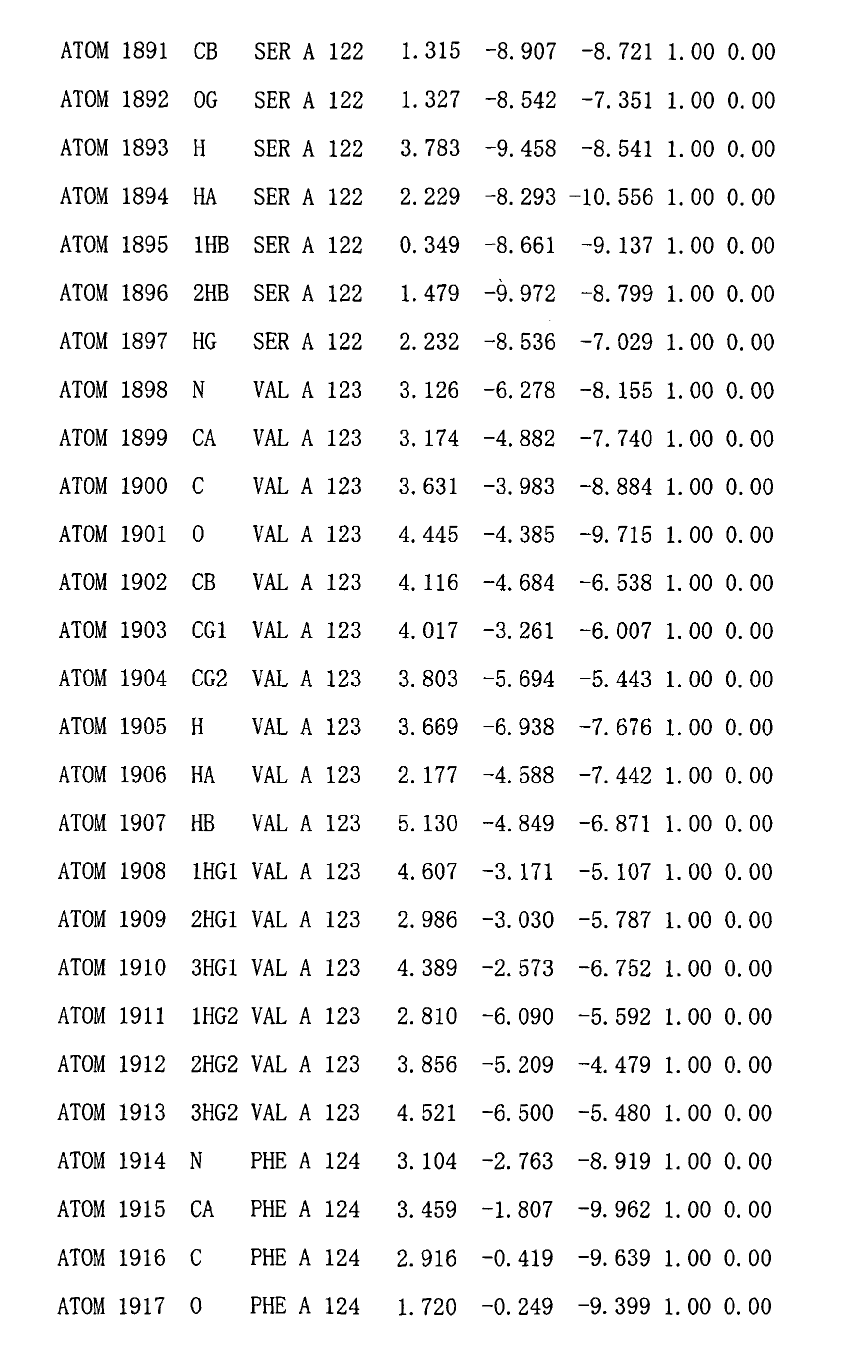

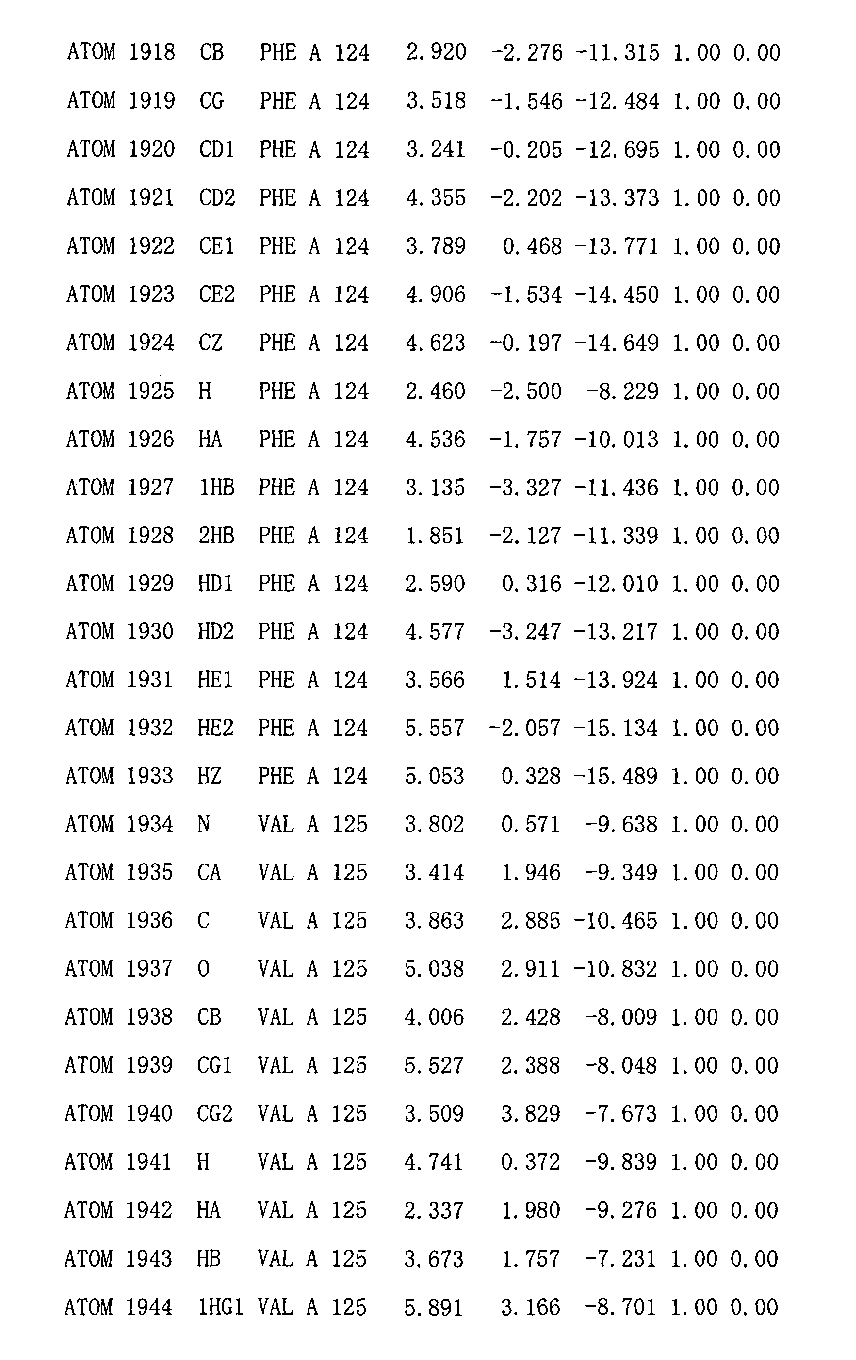

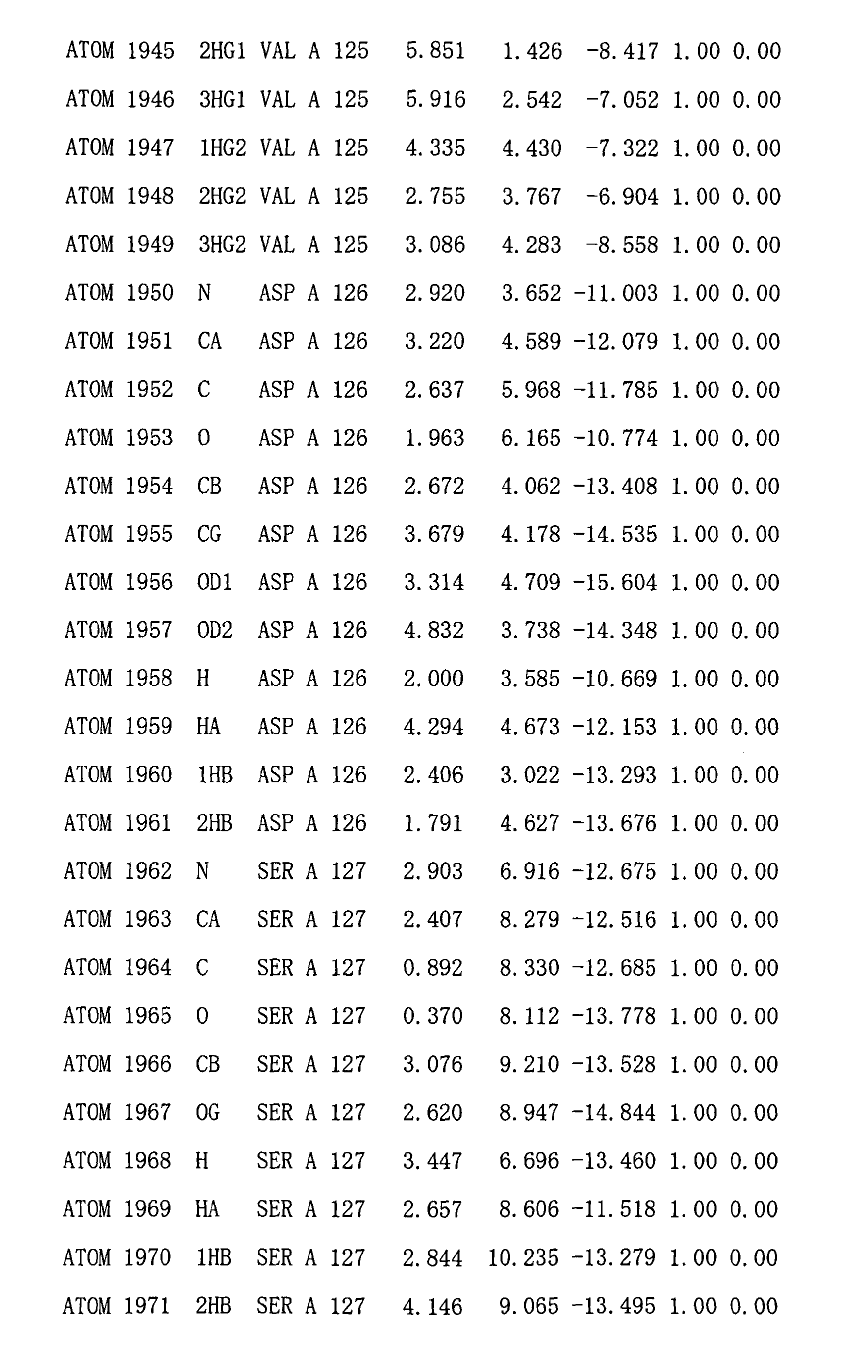

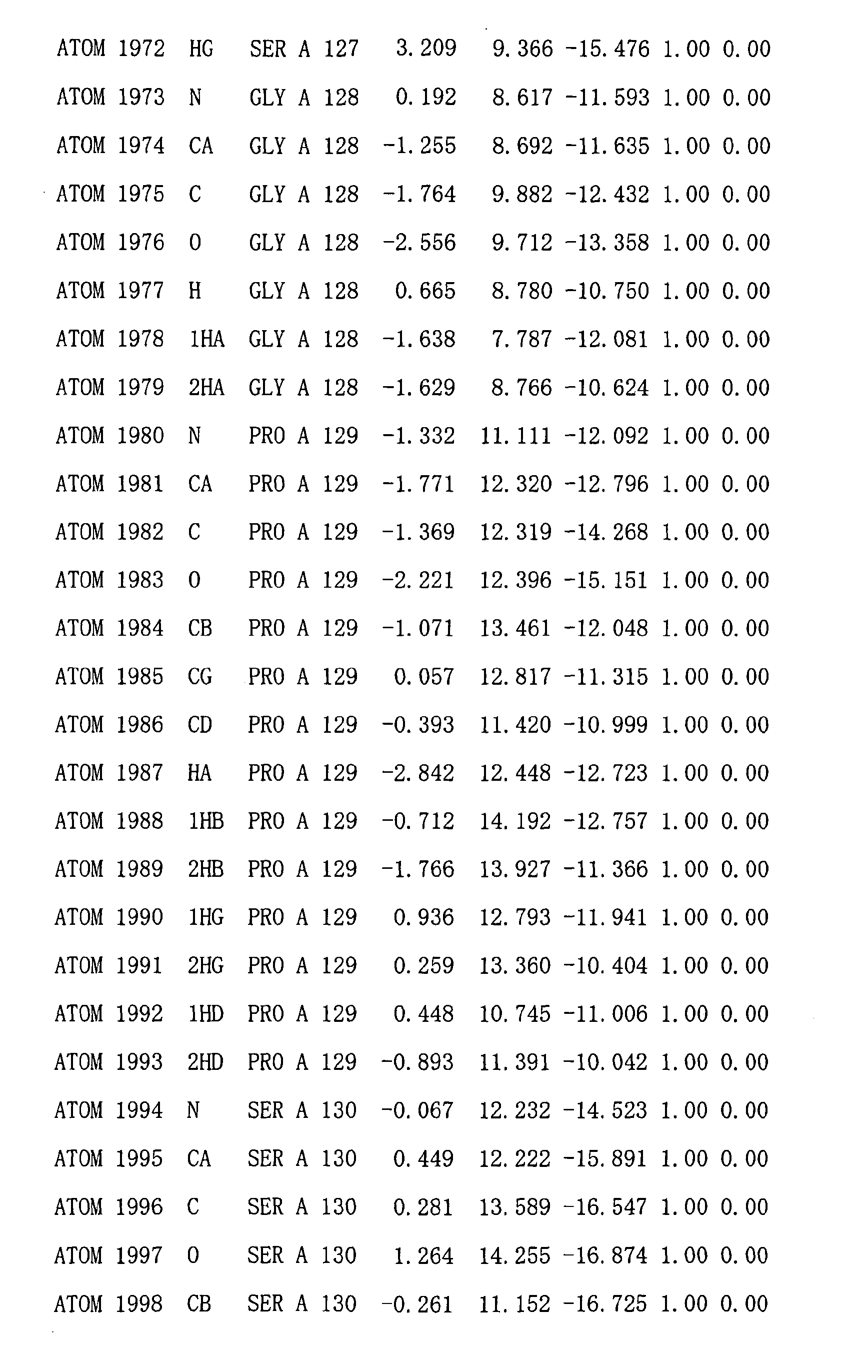

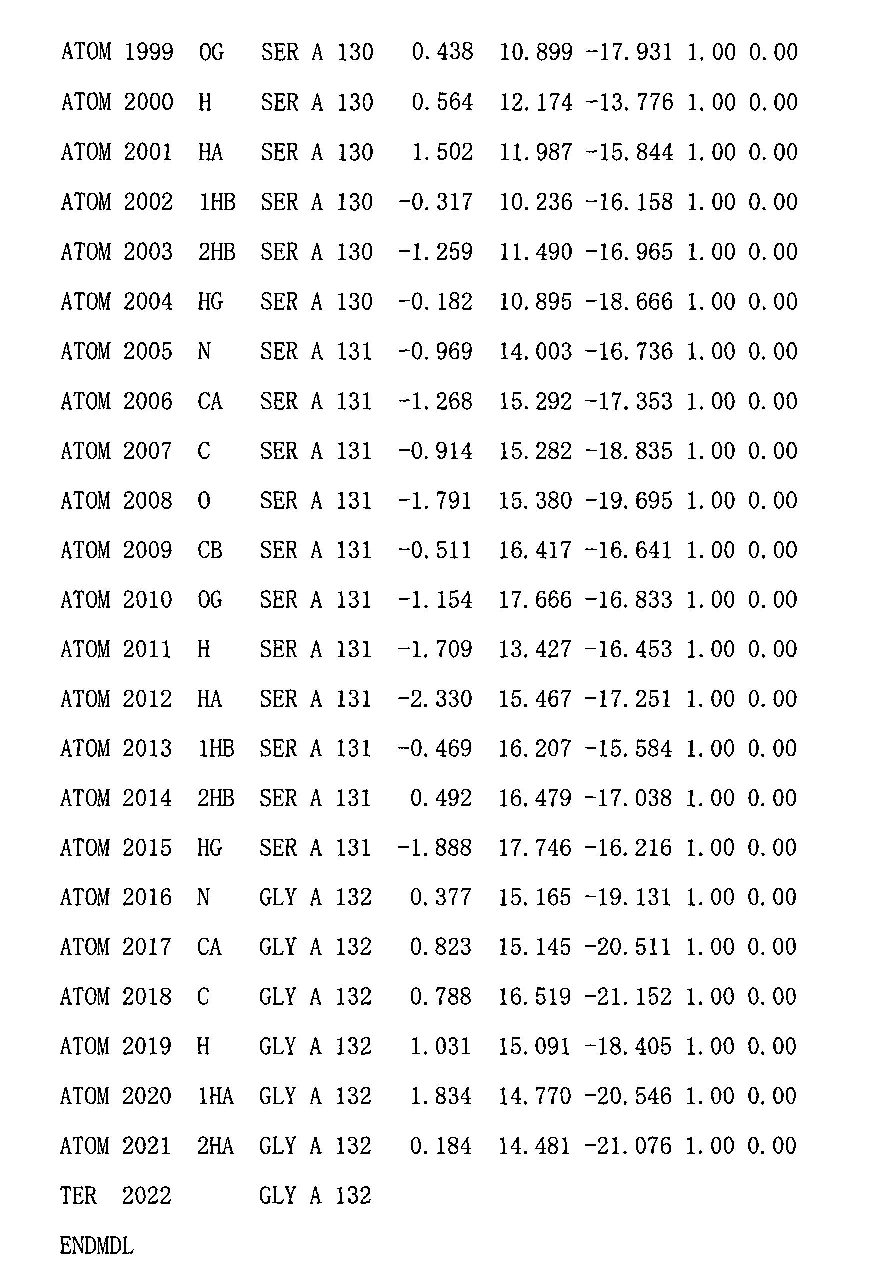

### Three-Dimensional Structure Coordinate 13

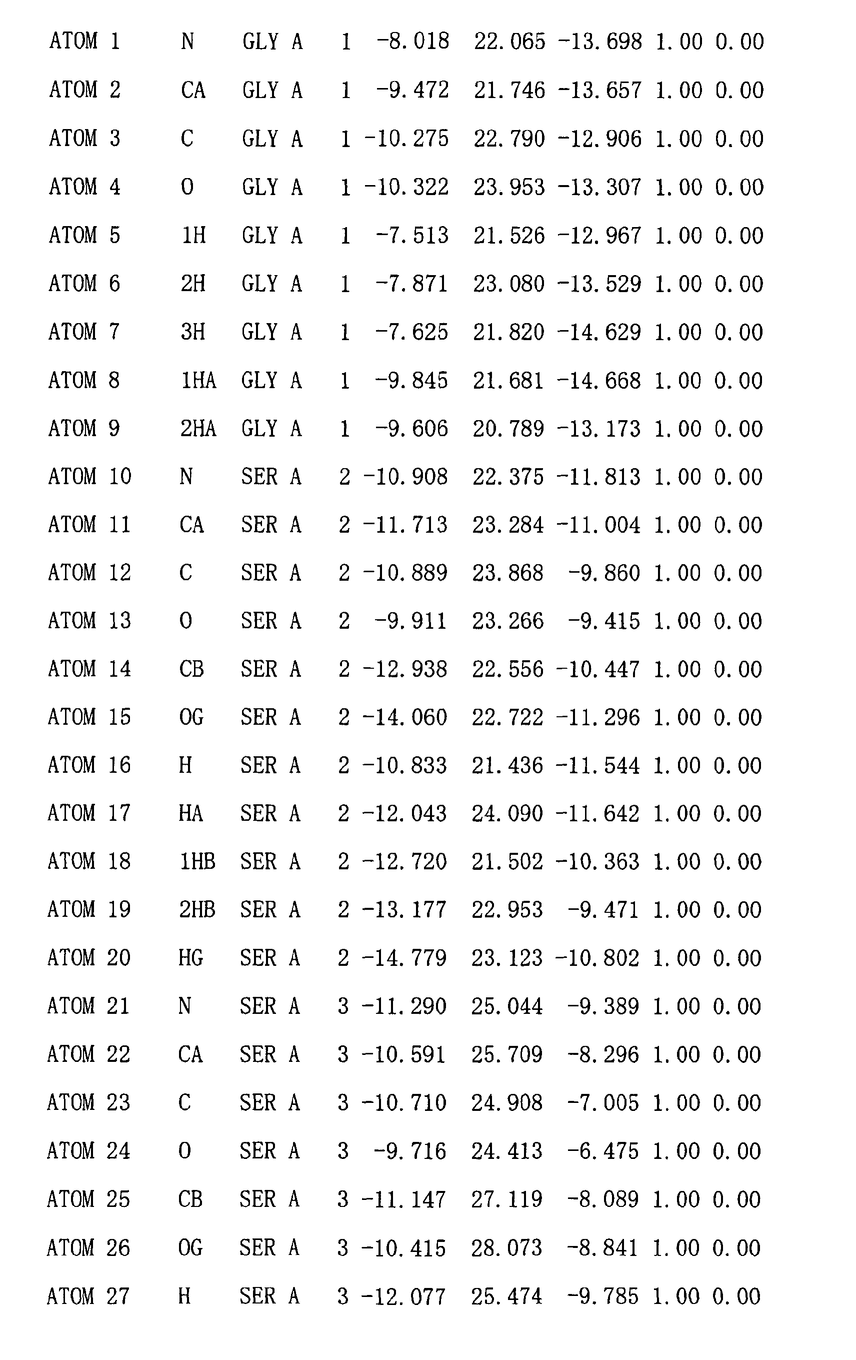

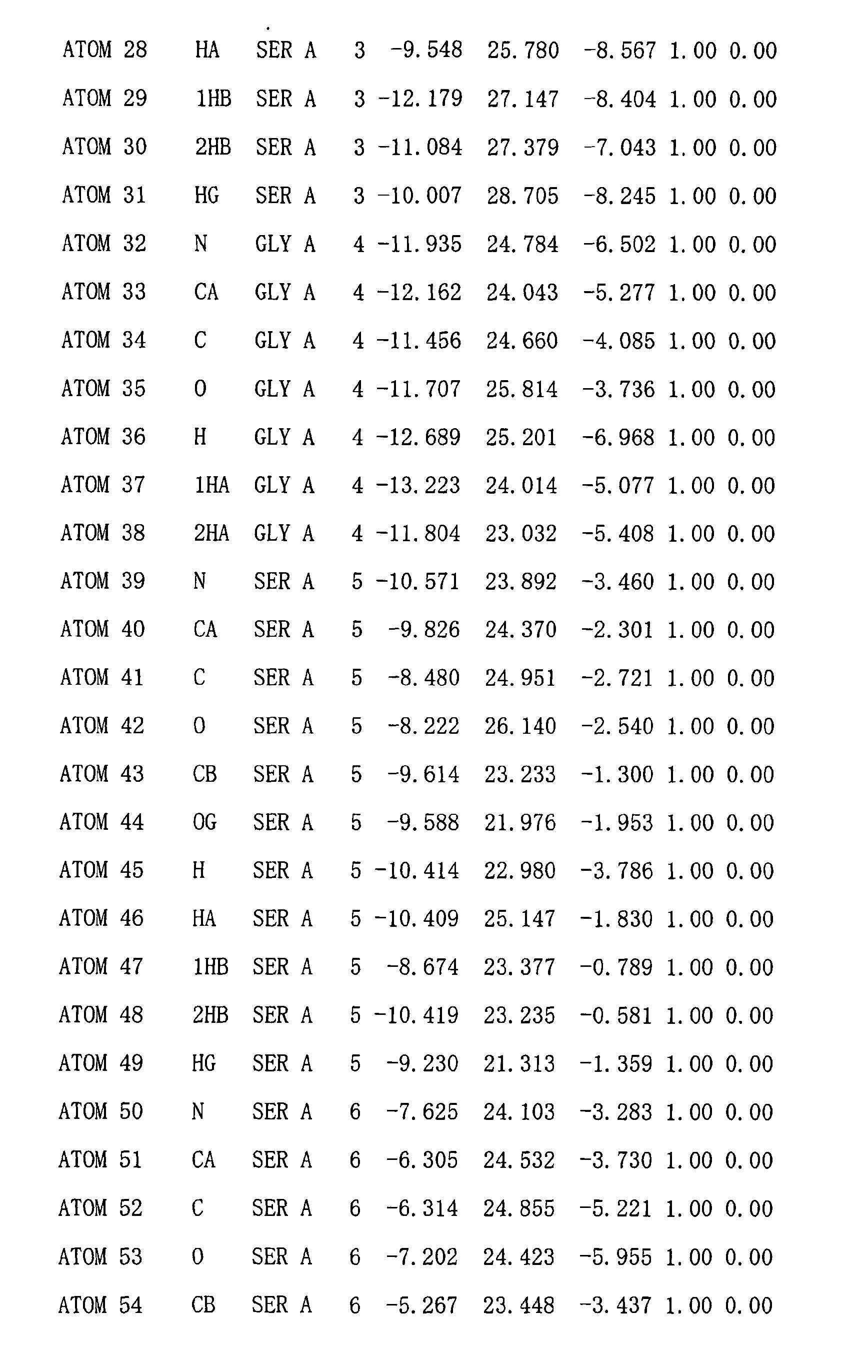

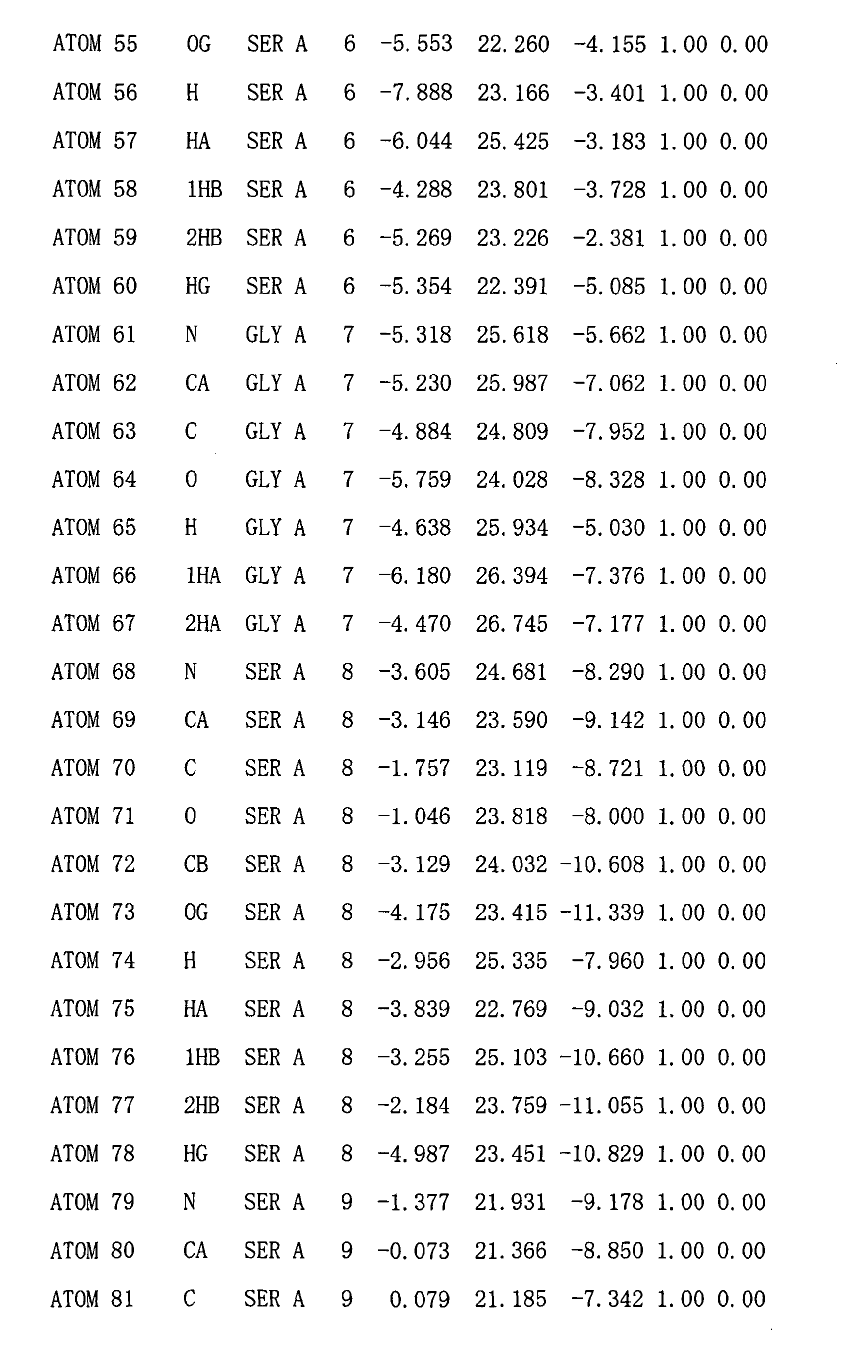

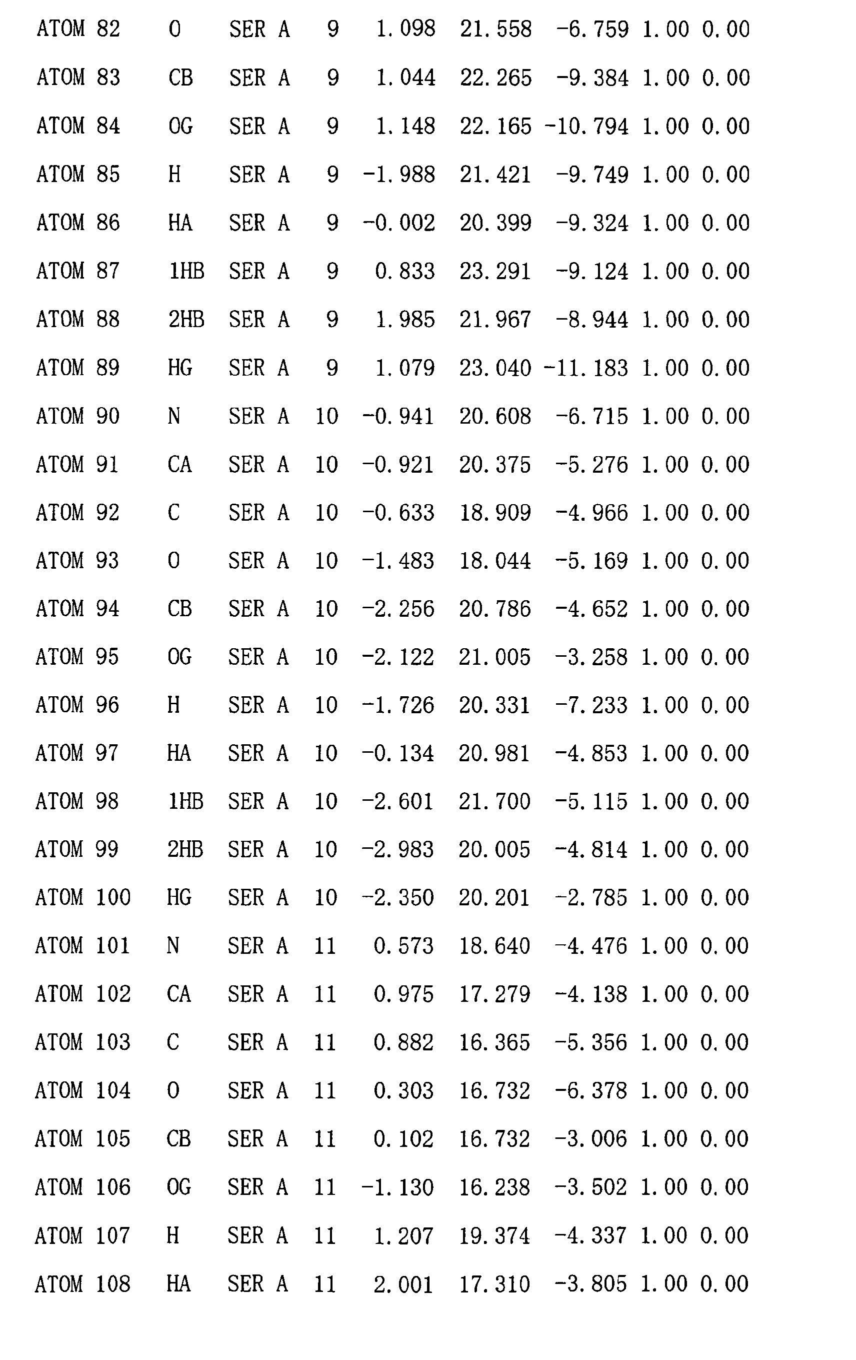

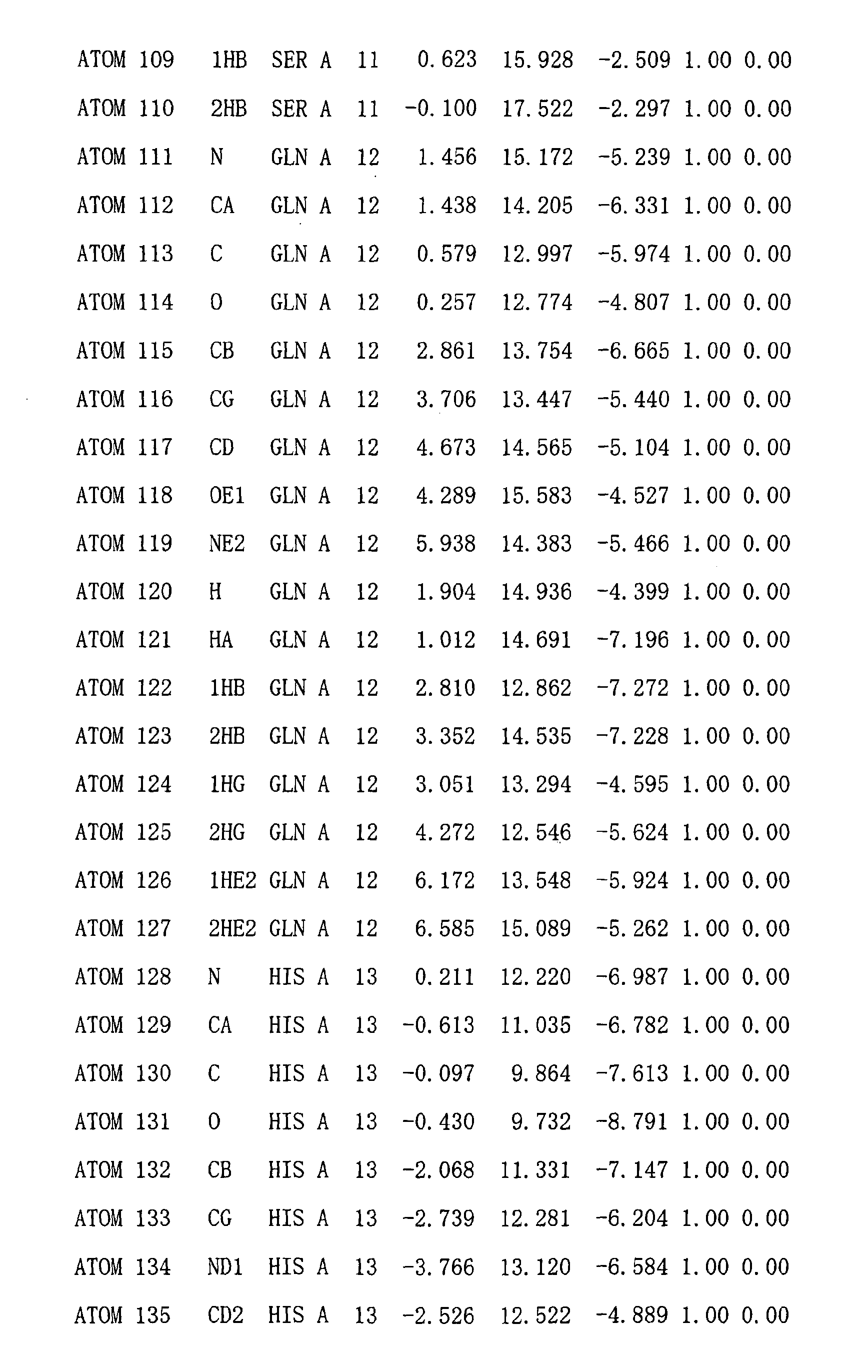

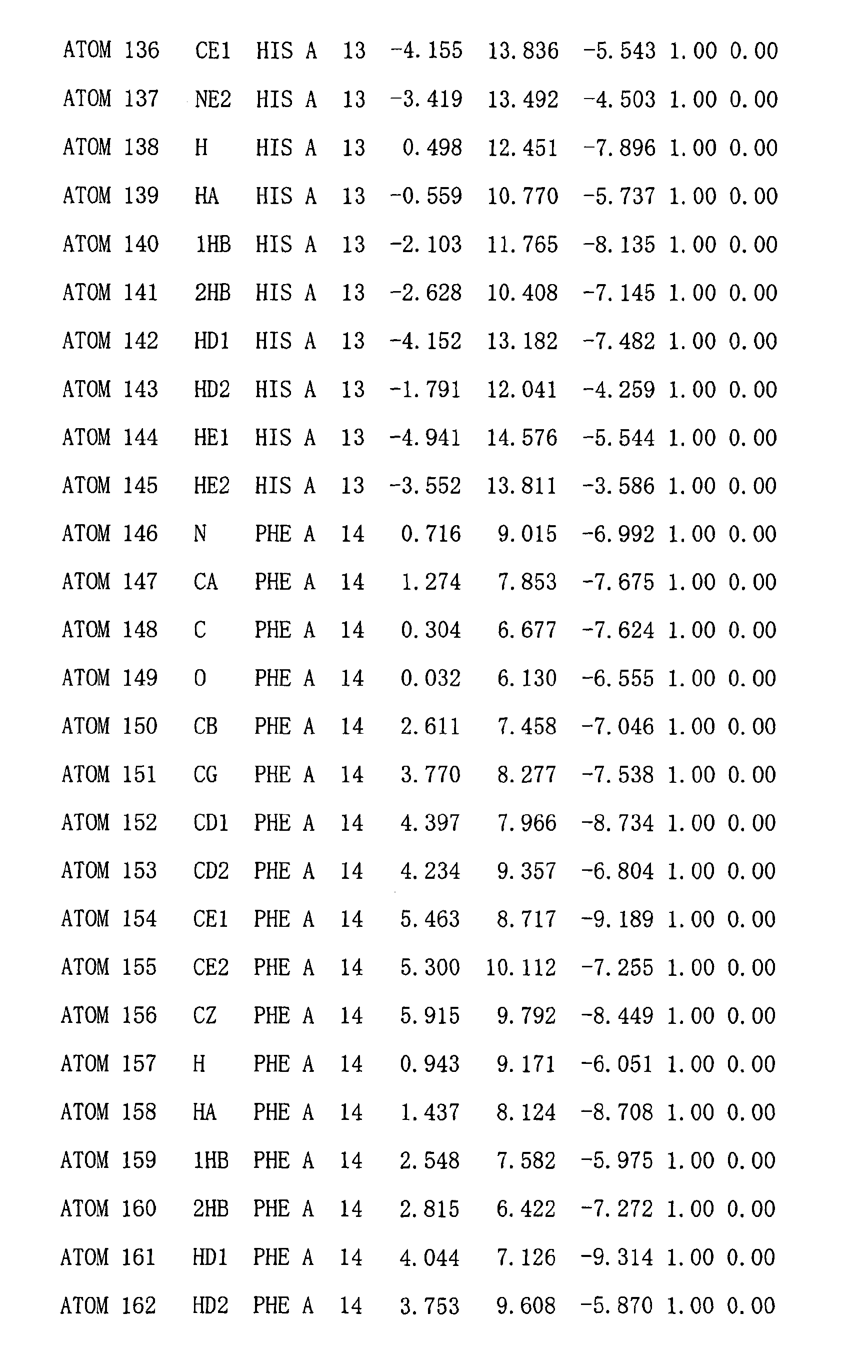

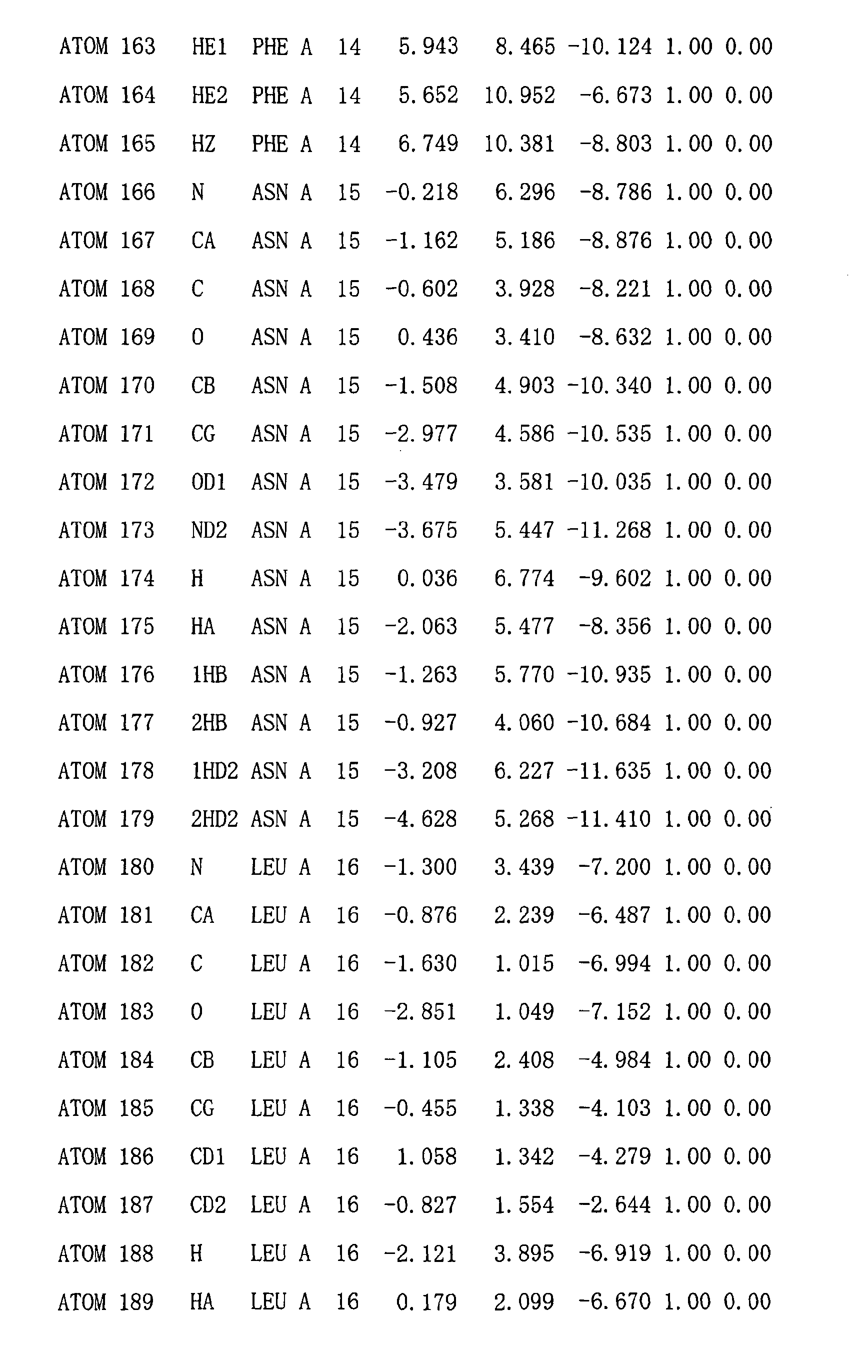

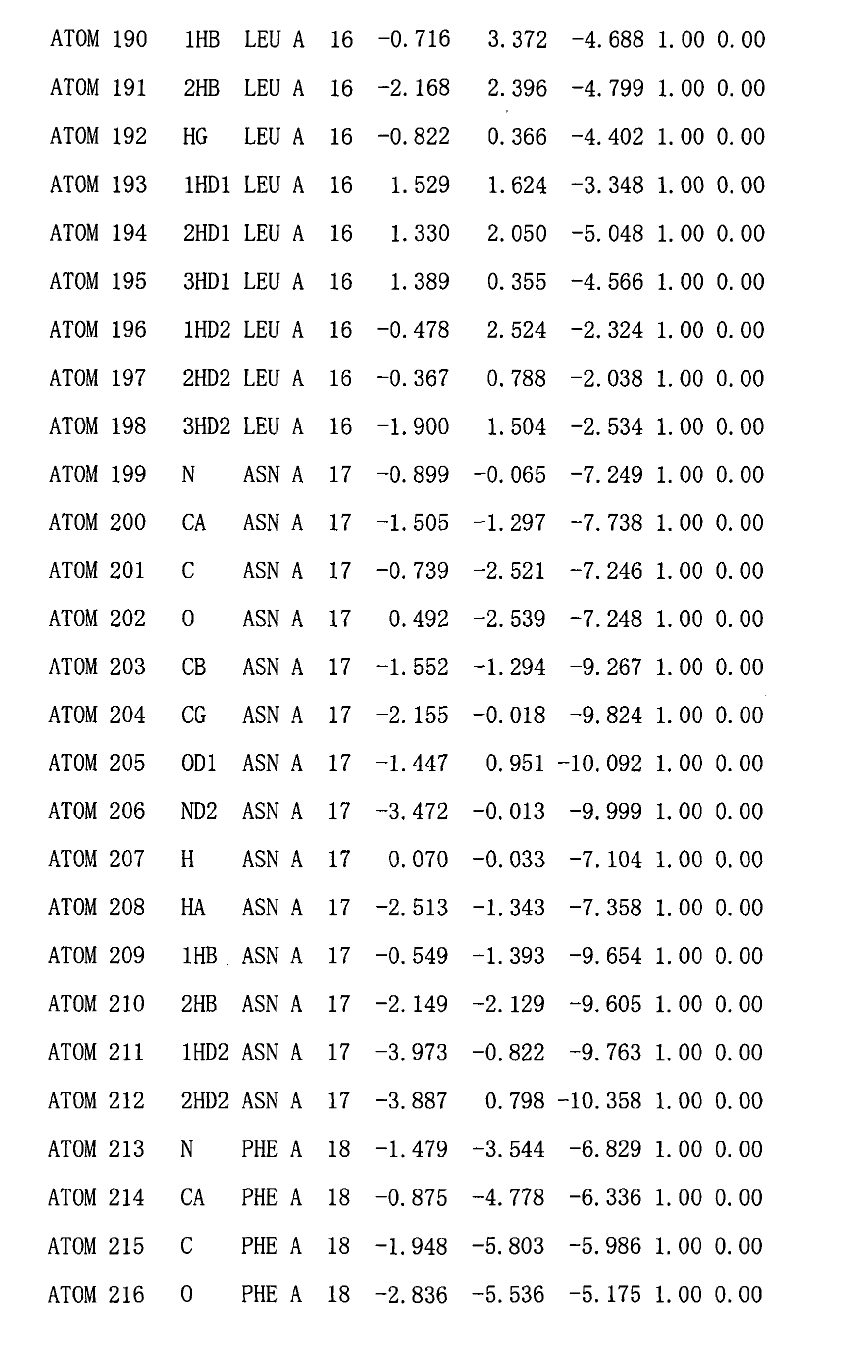

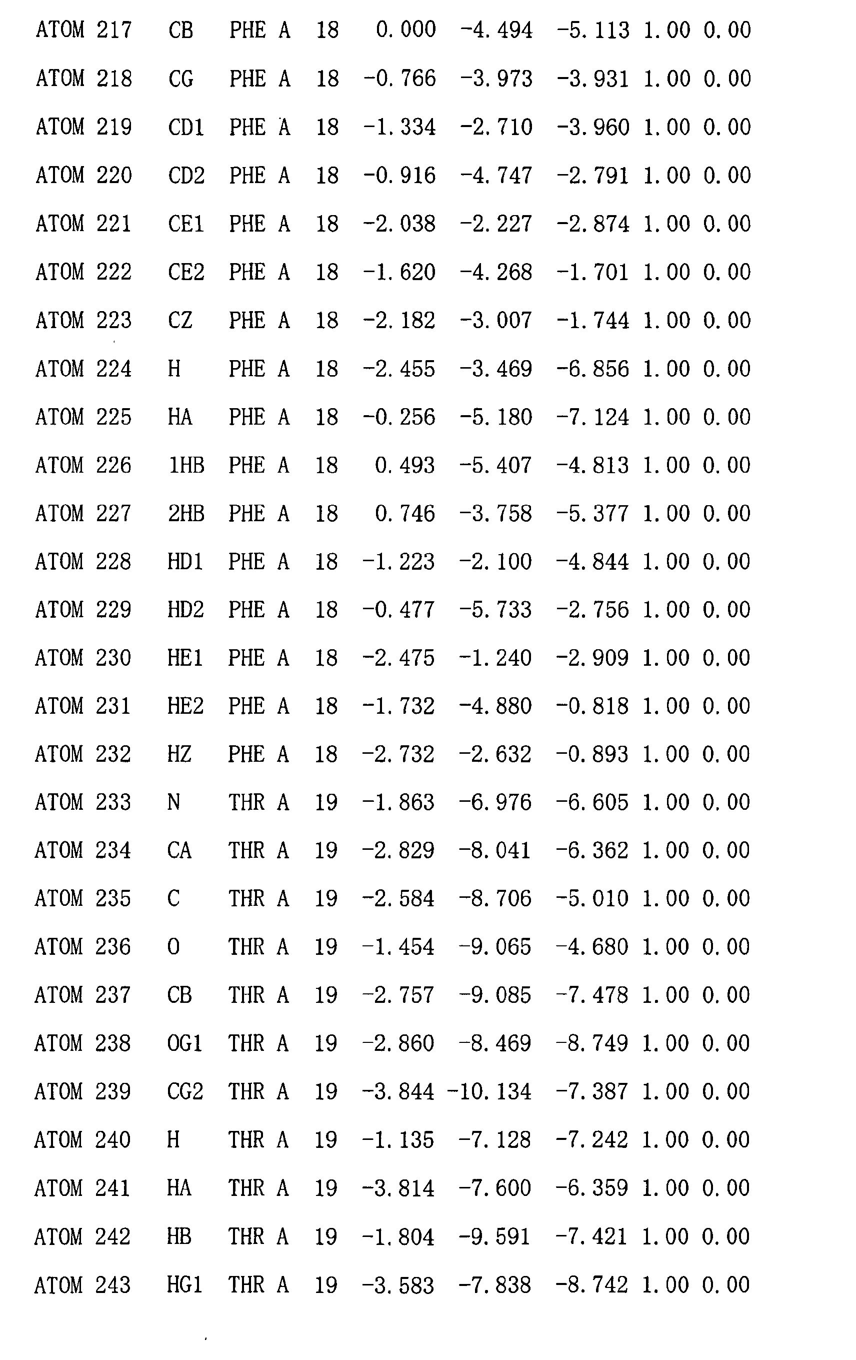

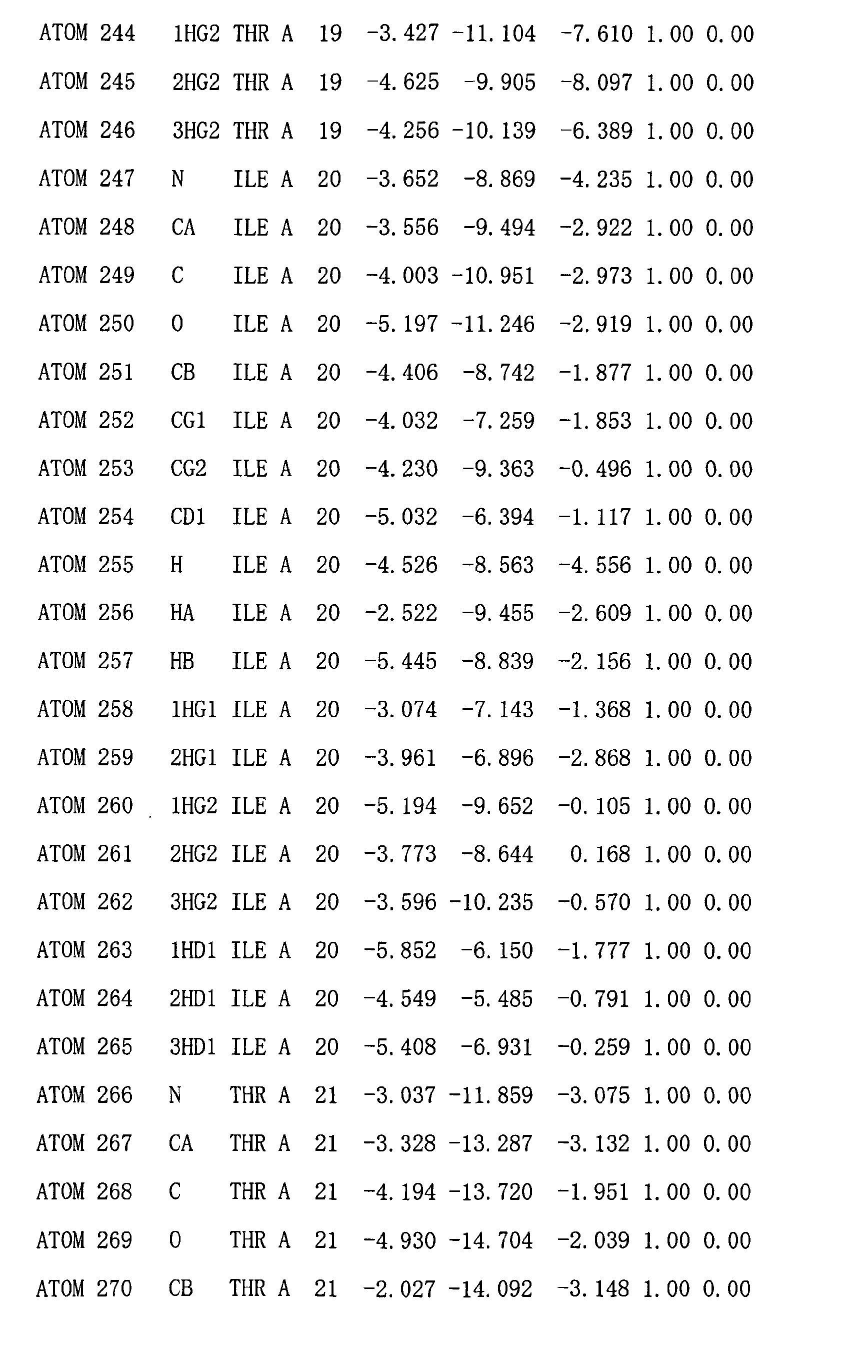

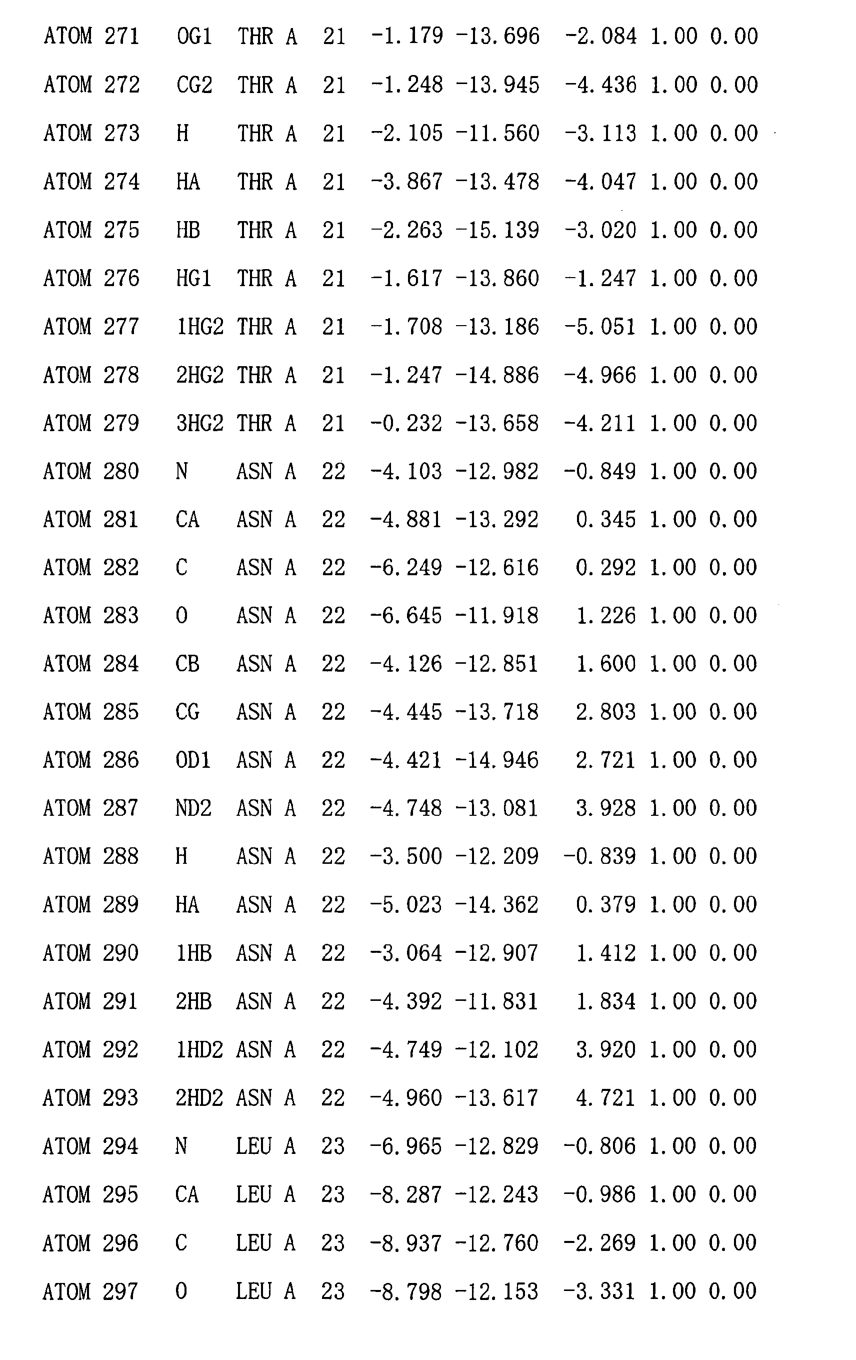

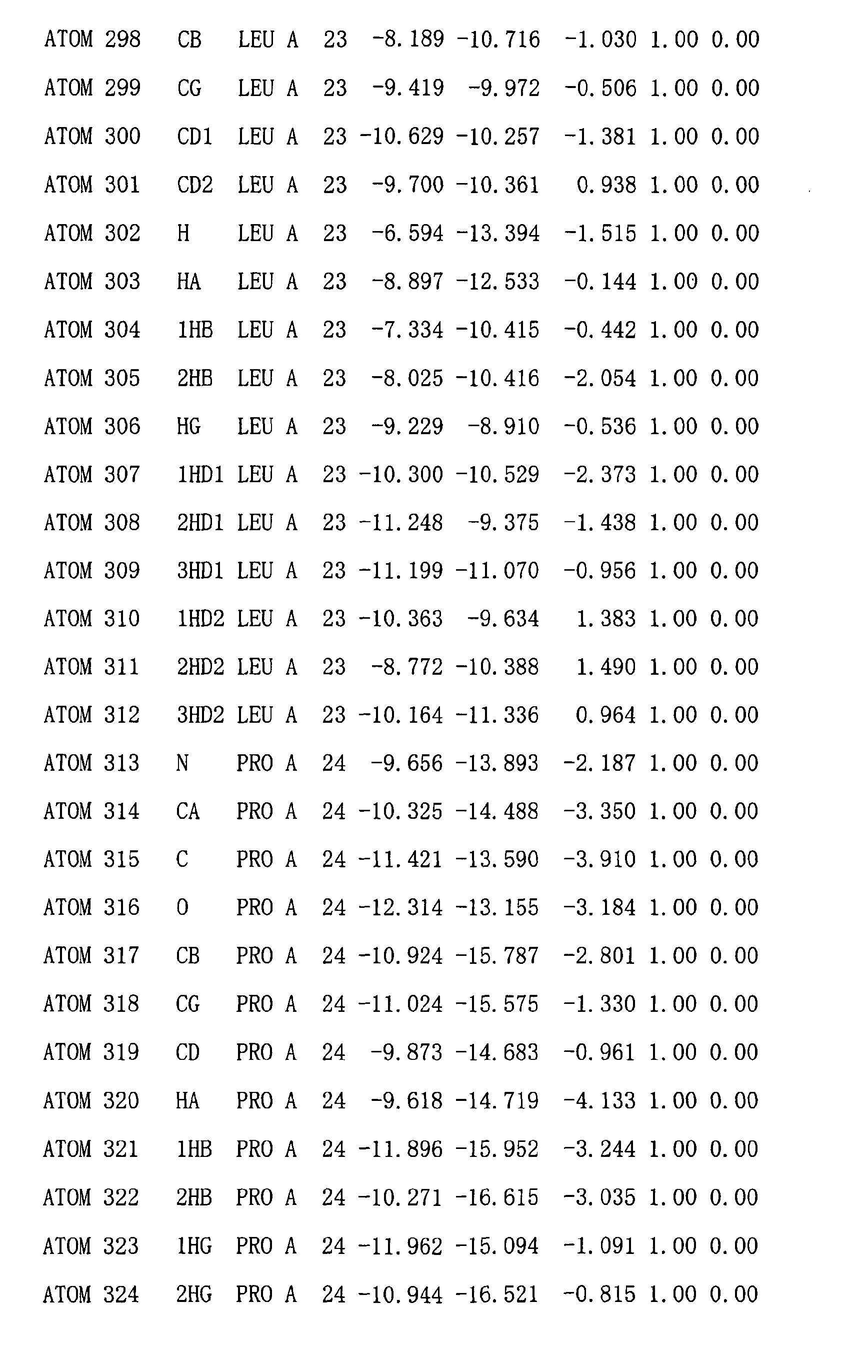

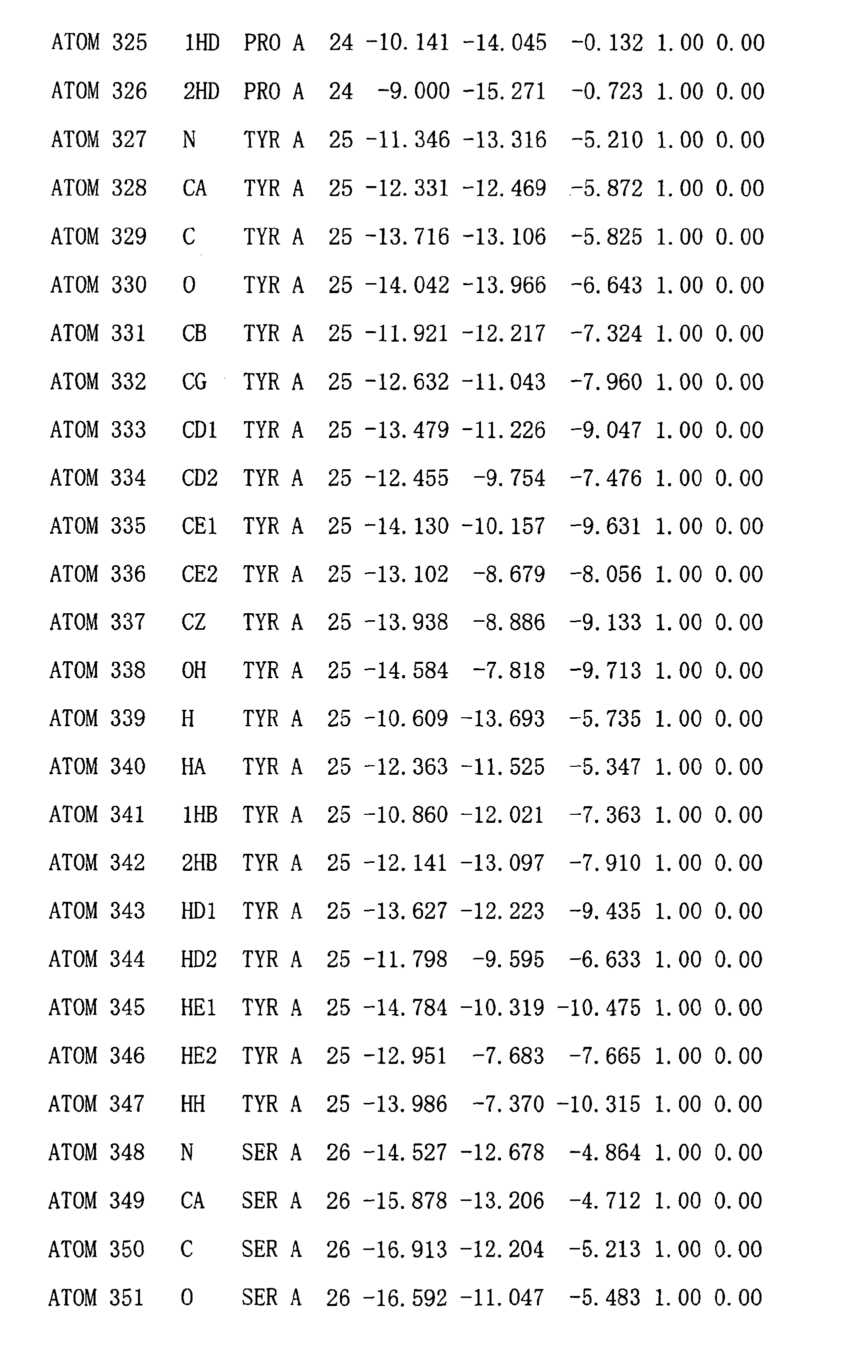

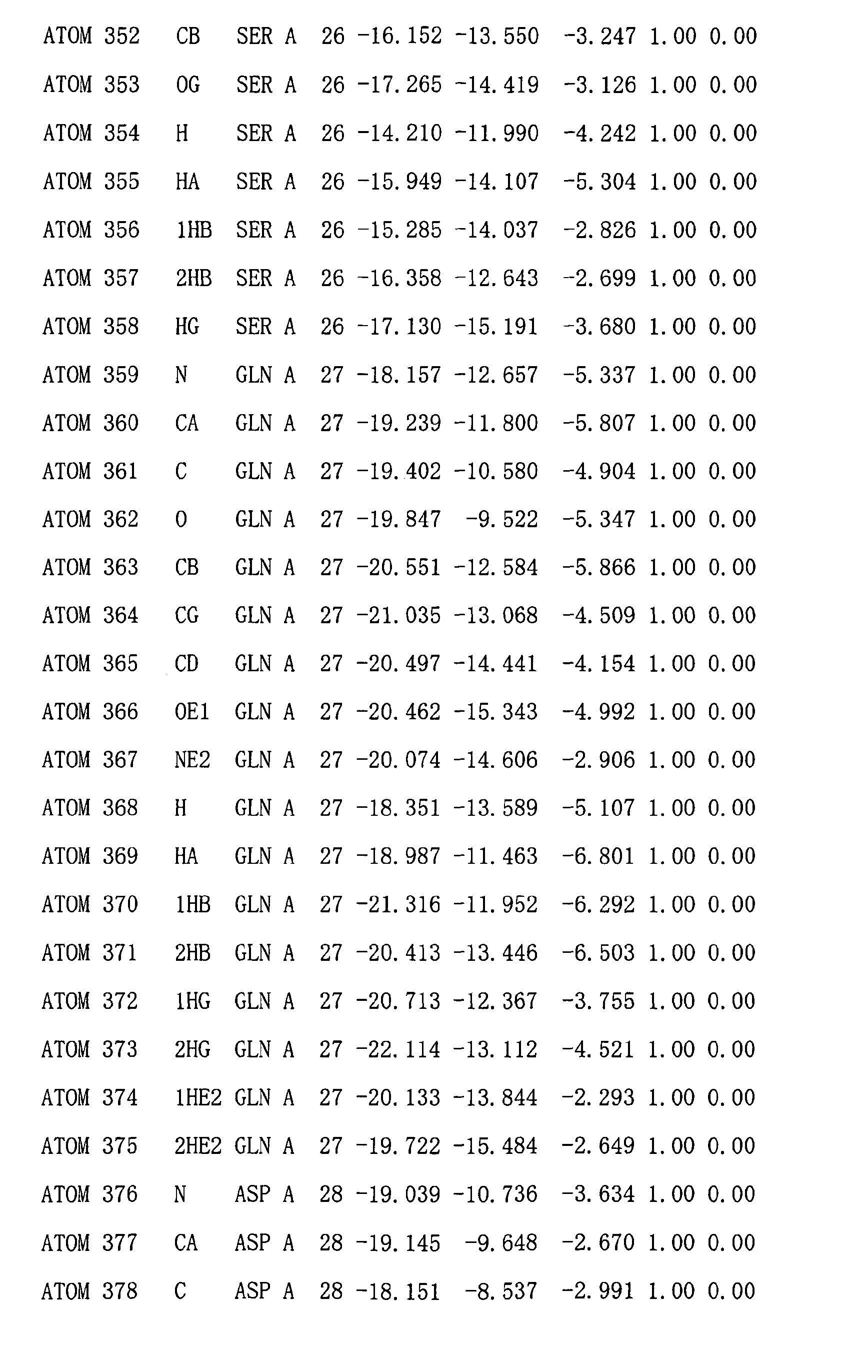

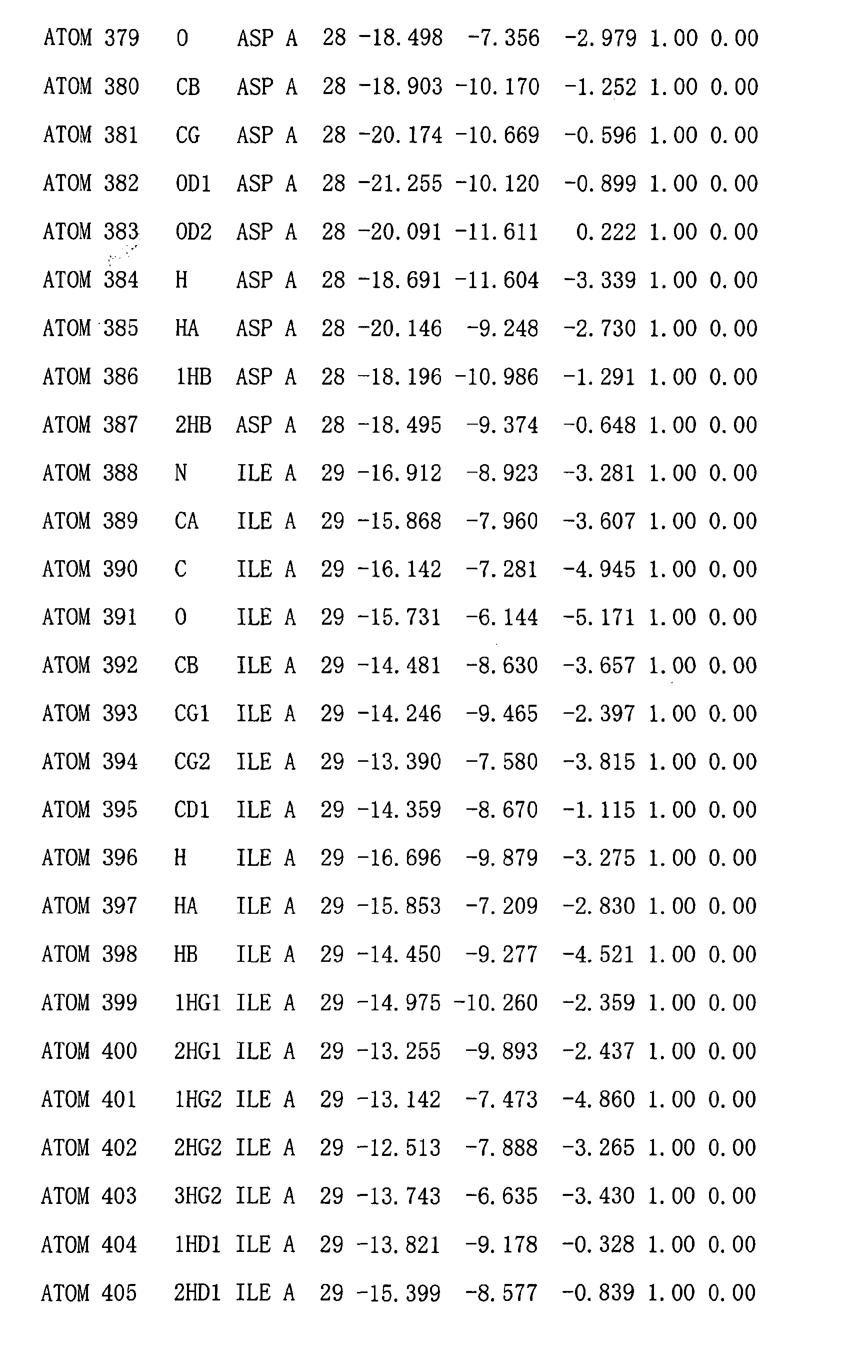

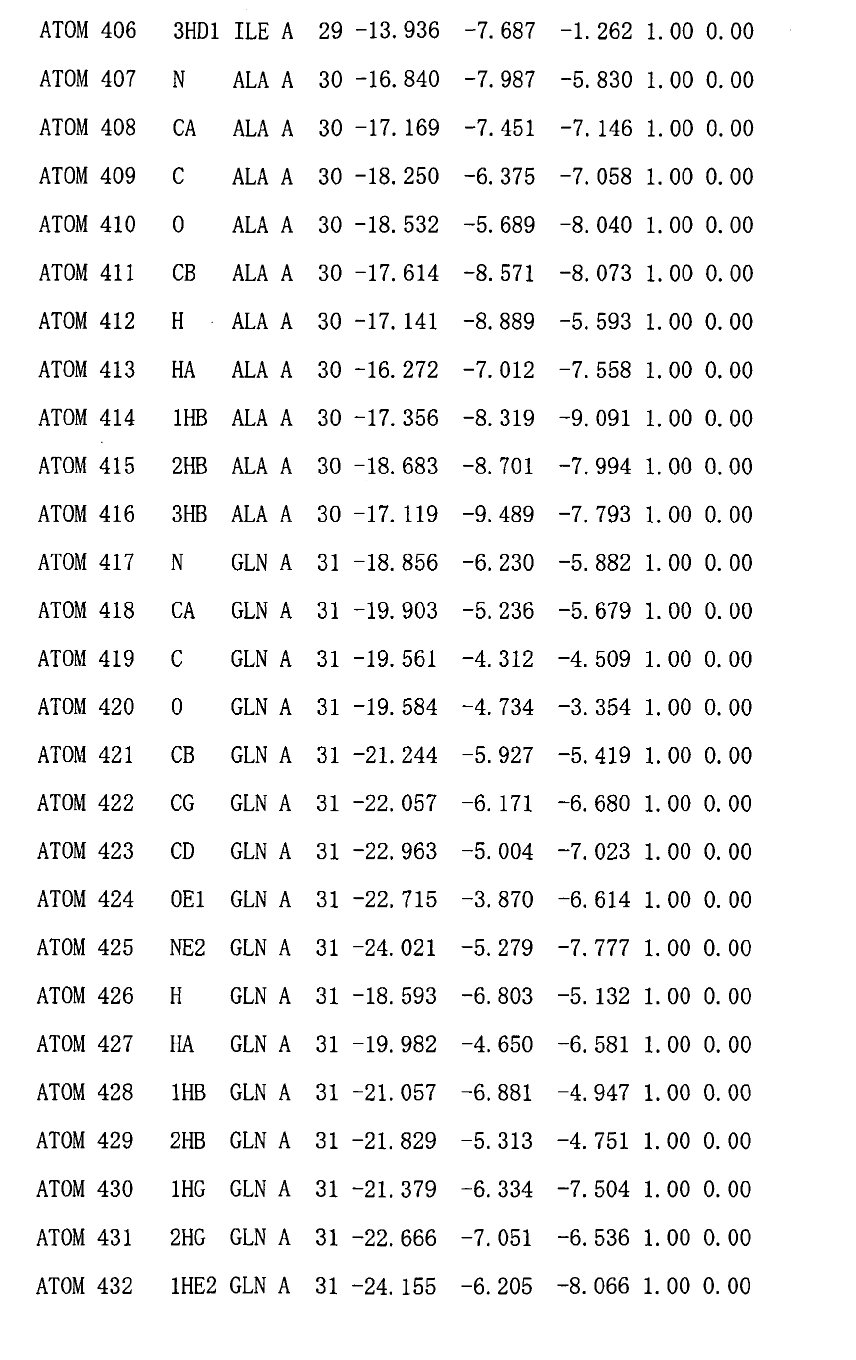

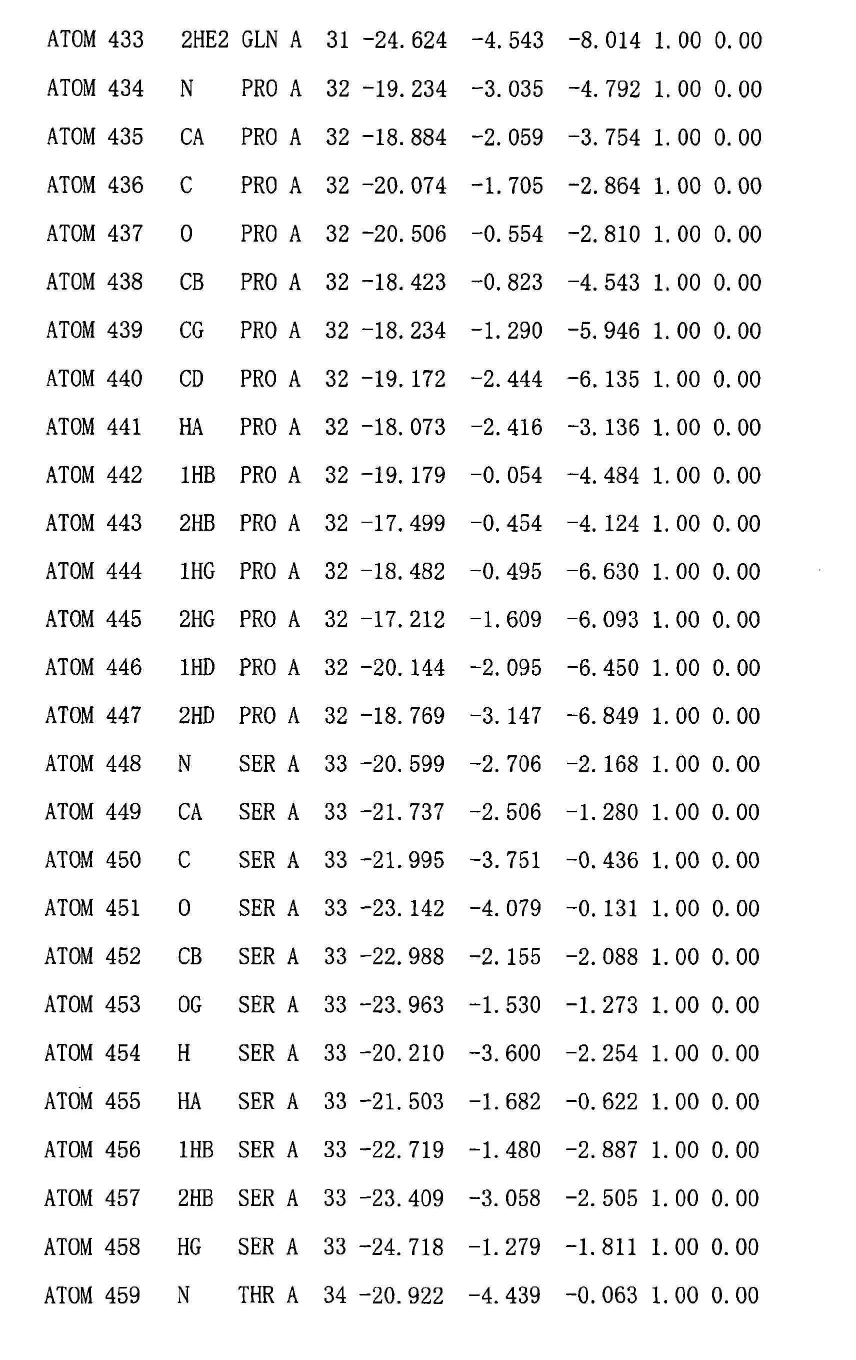

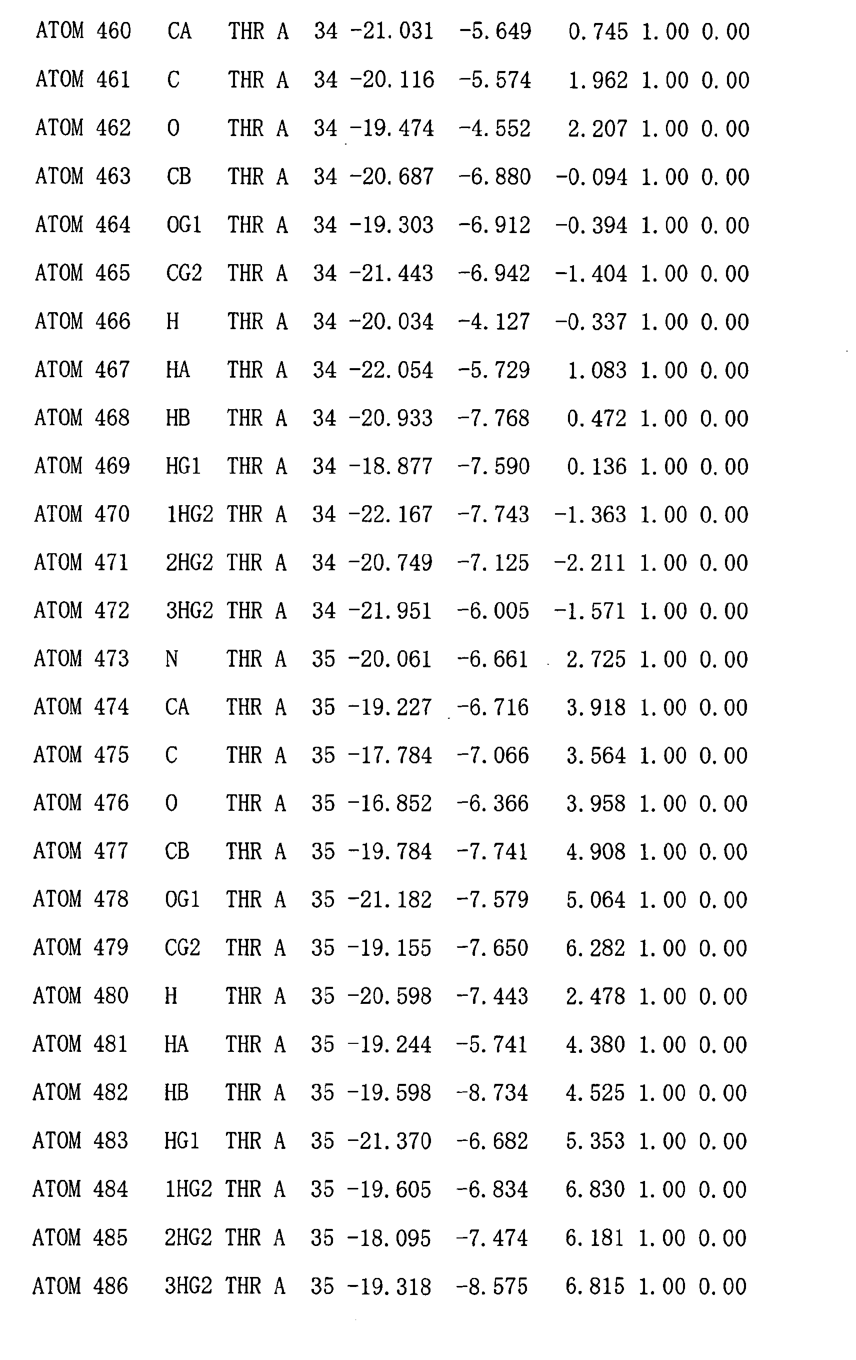

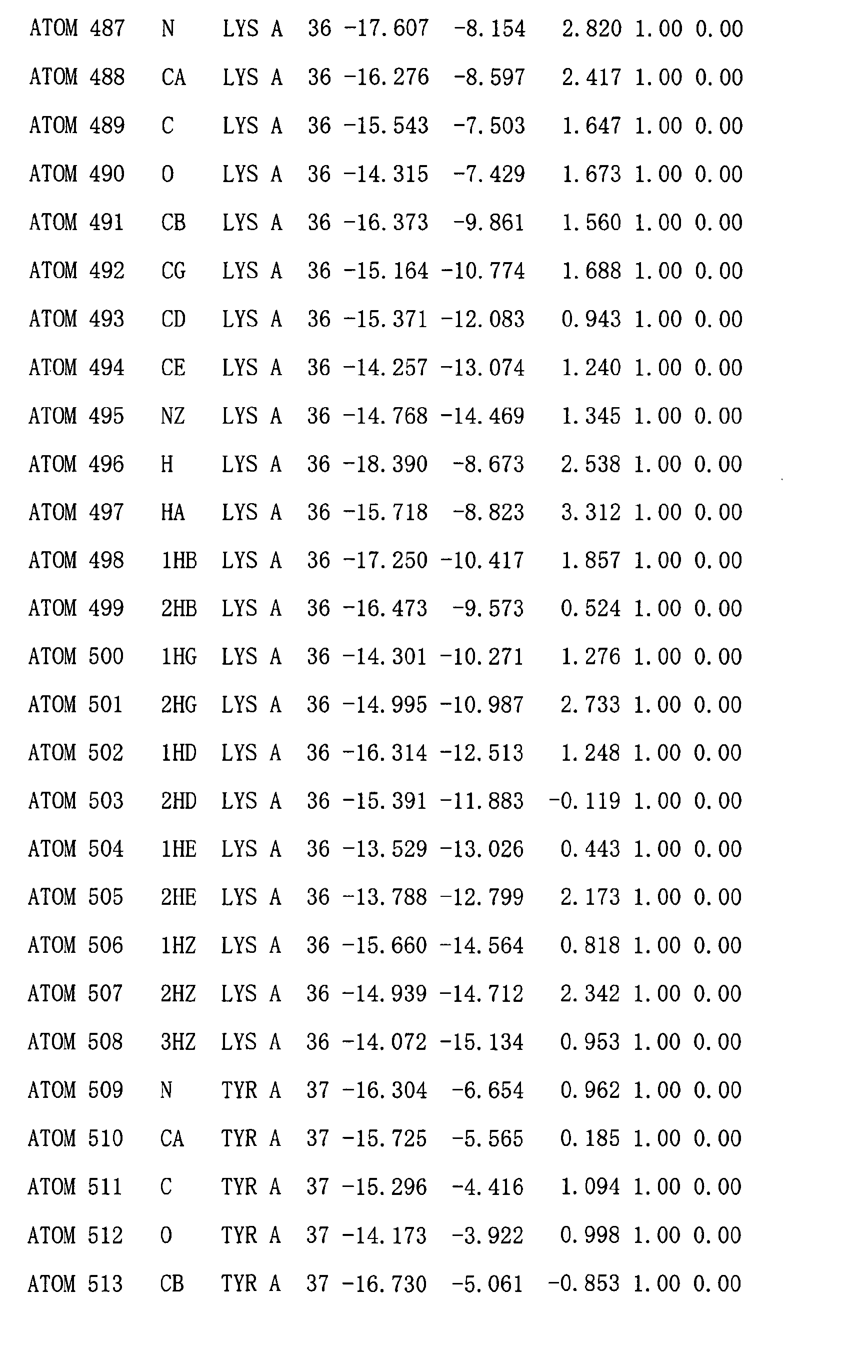

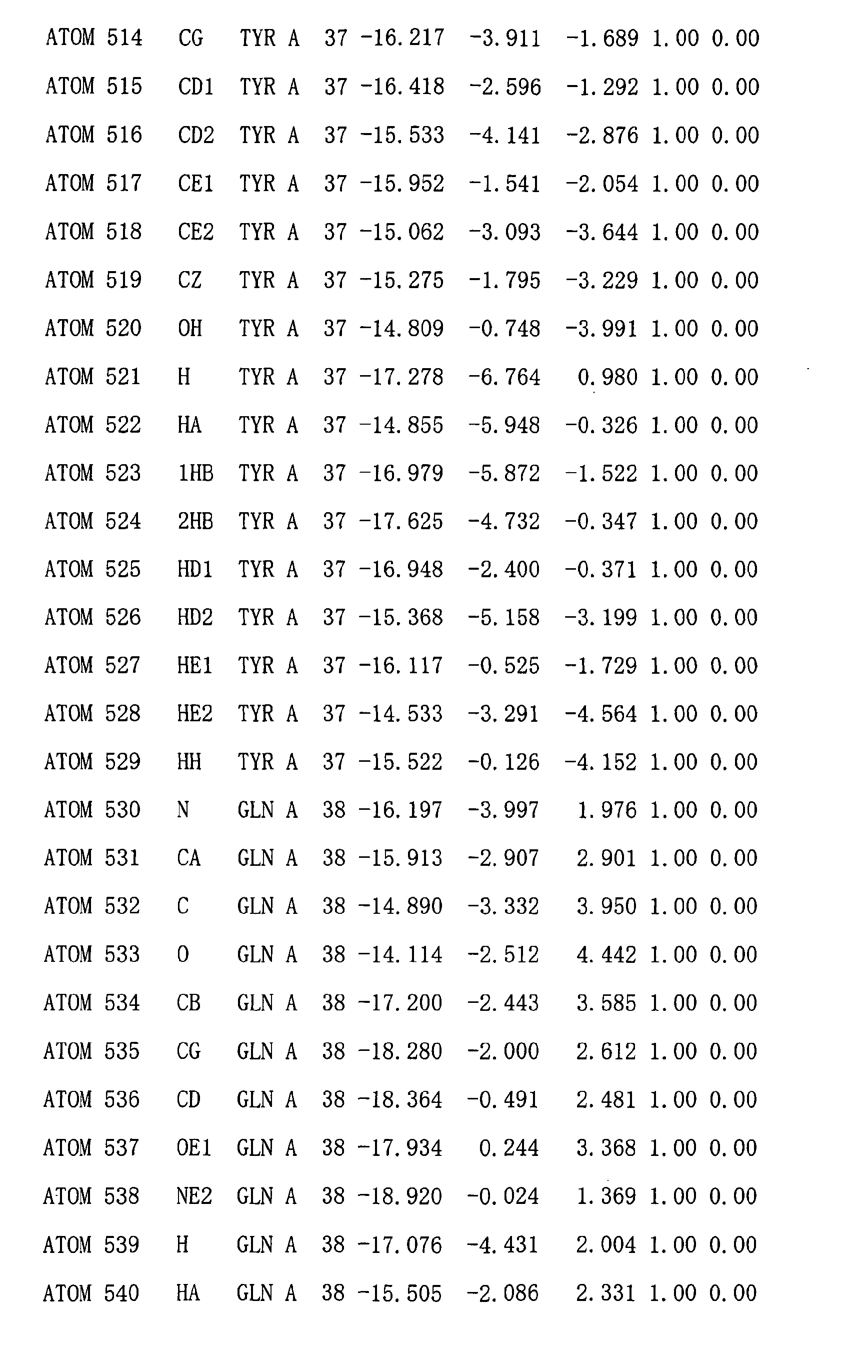

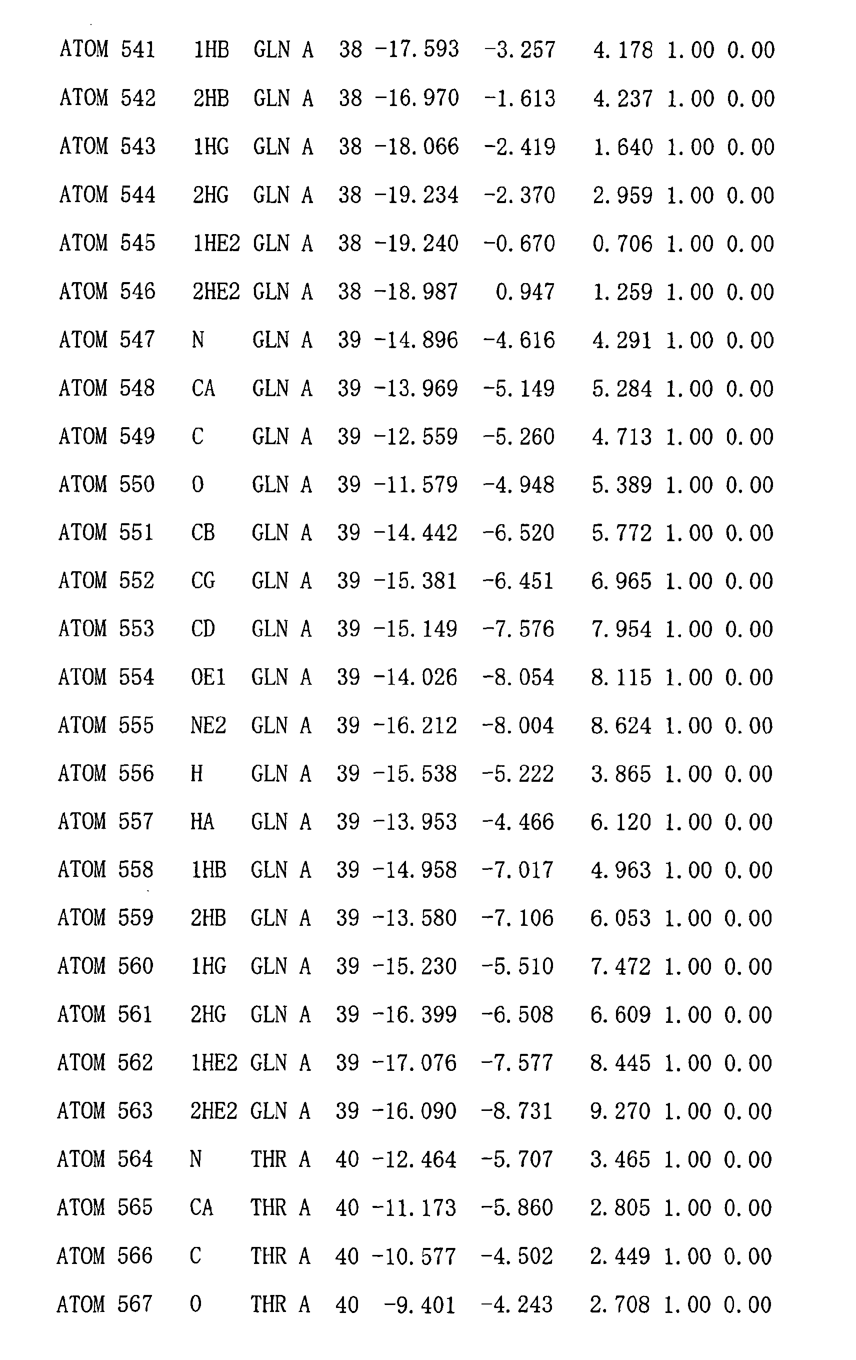

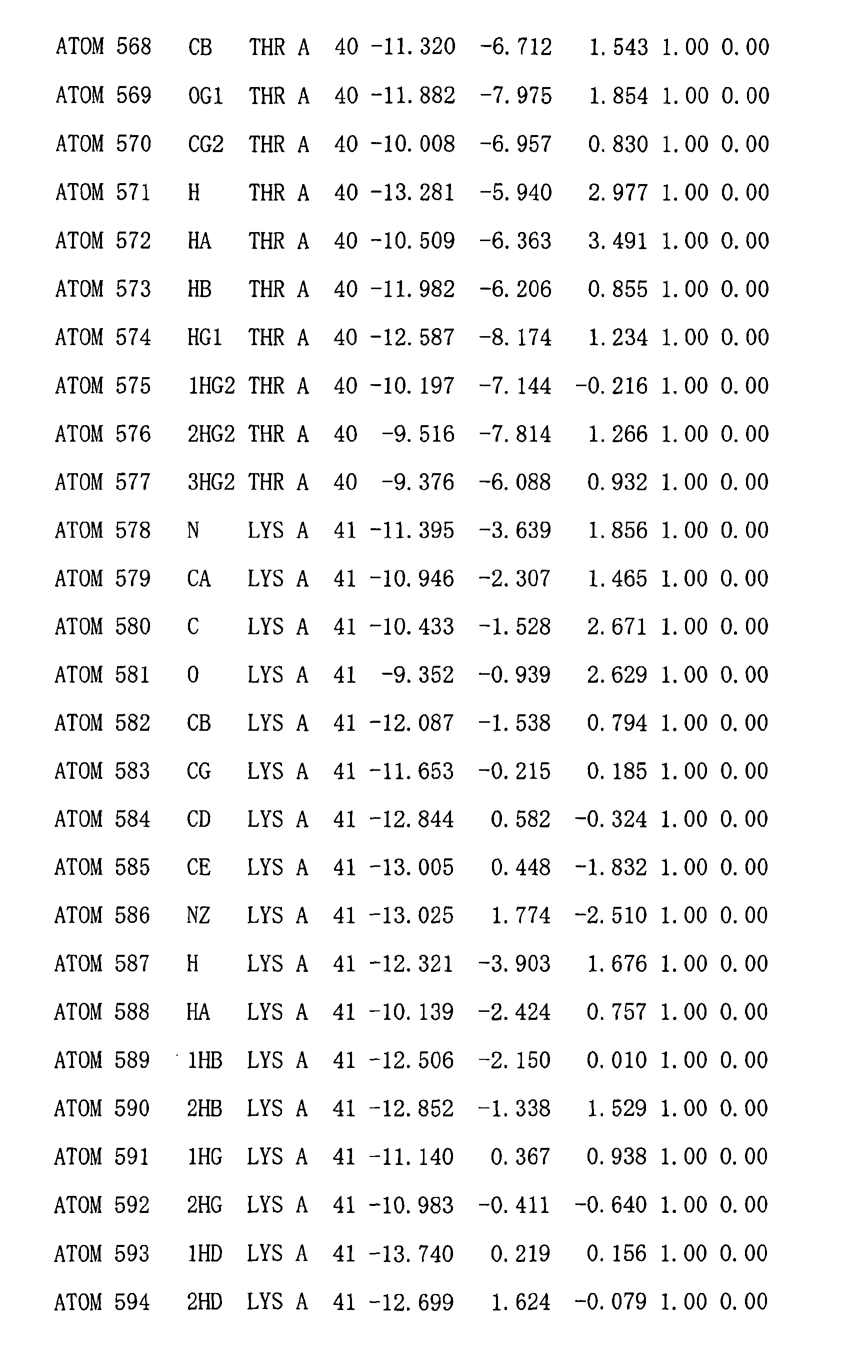

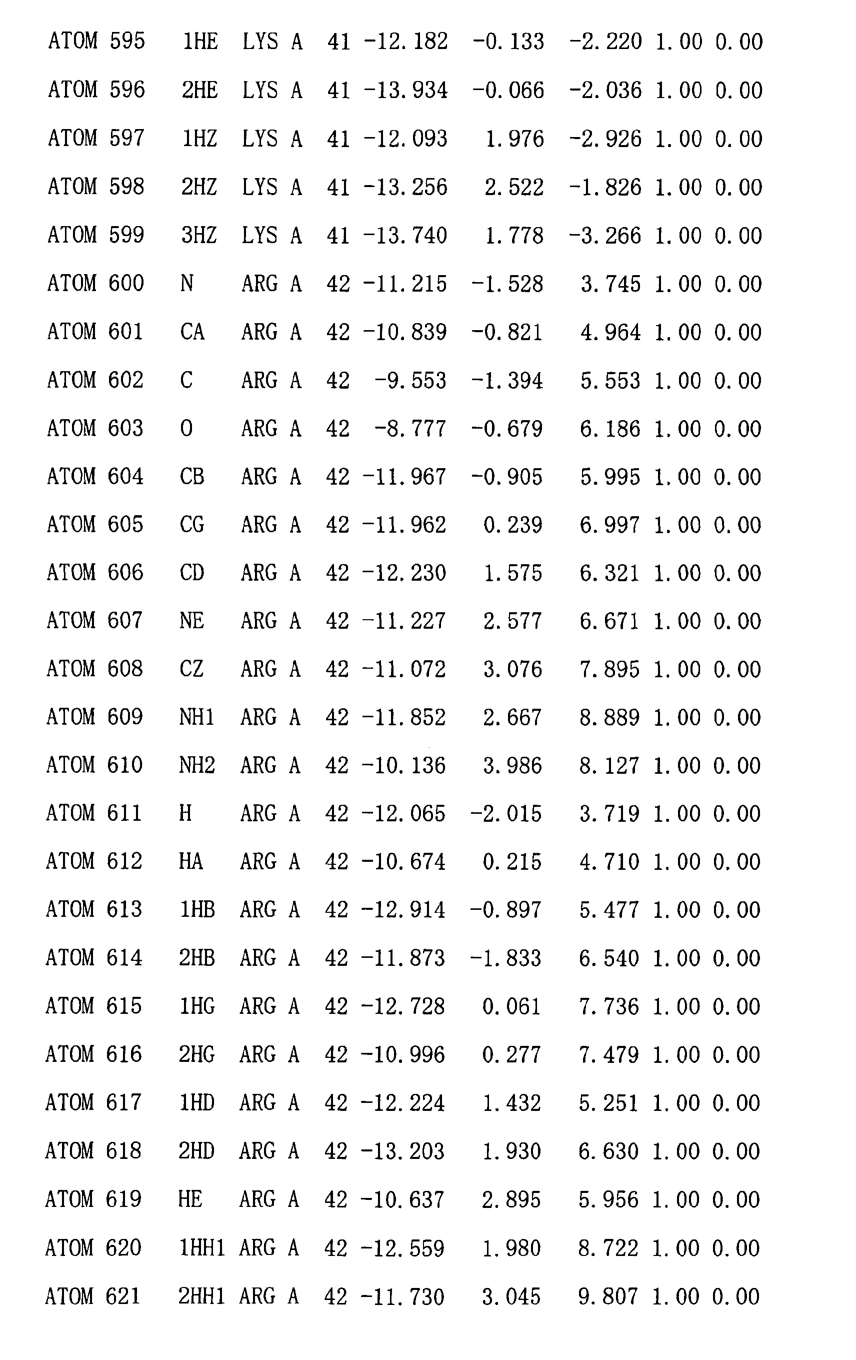

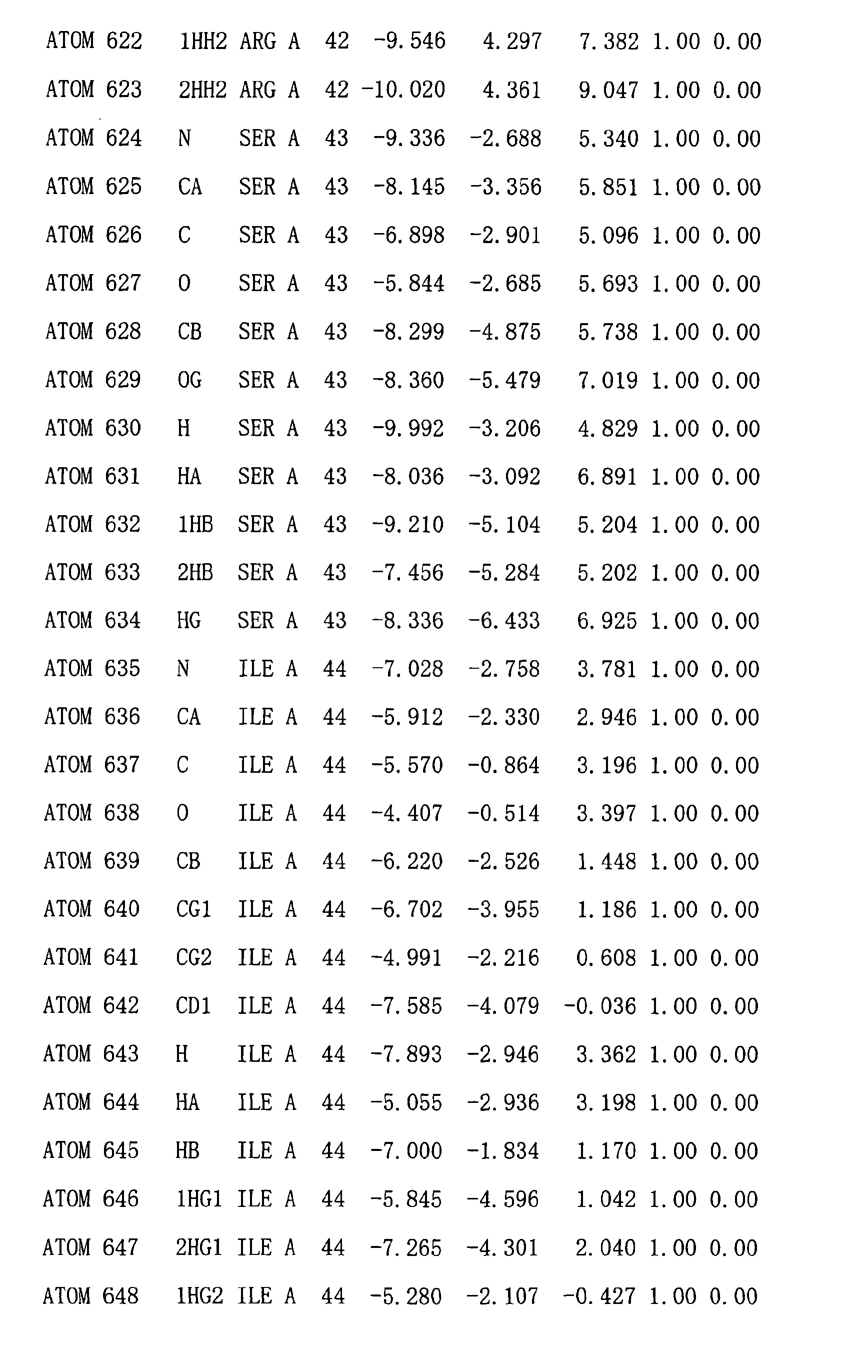

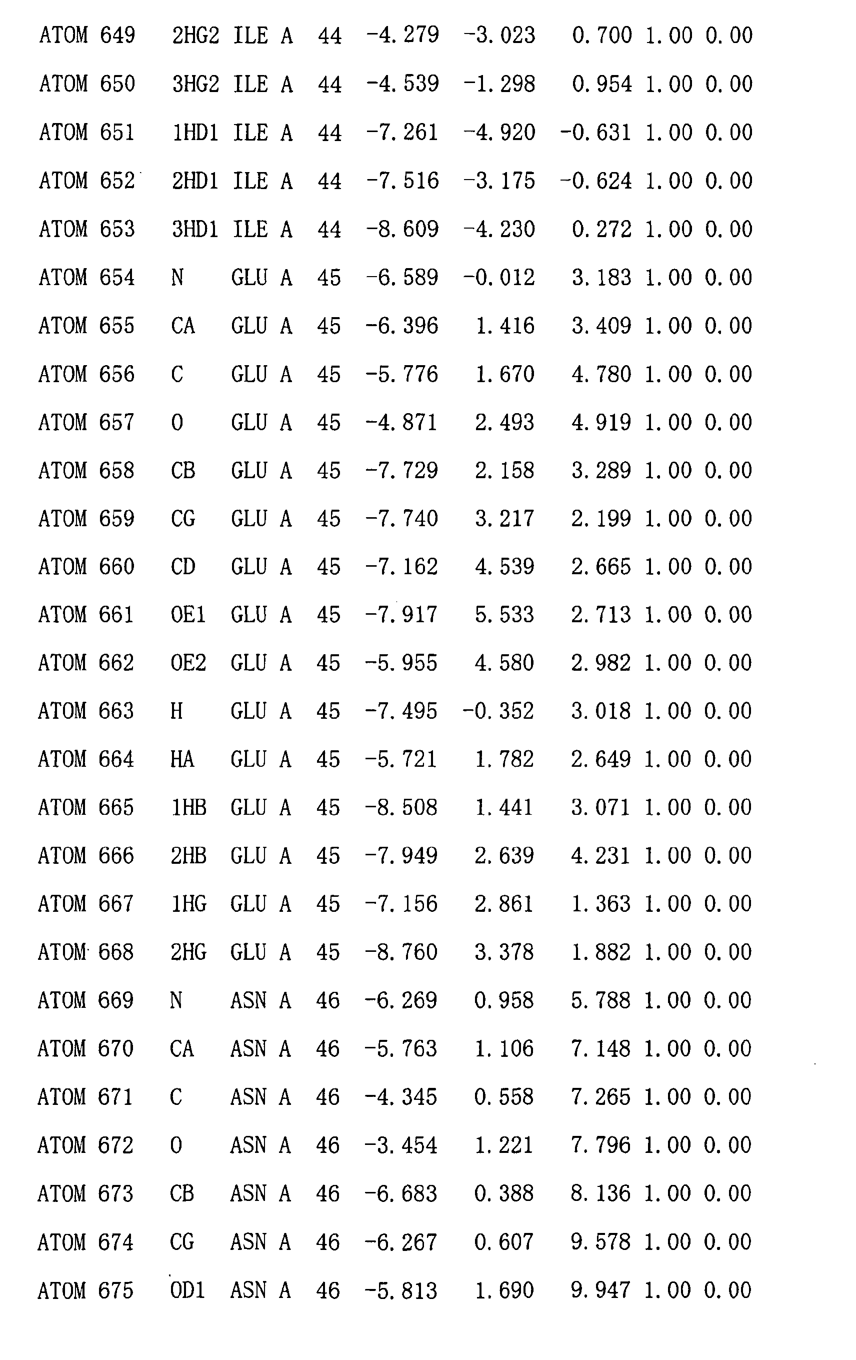

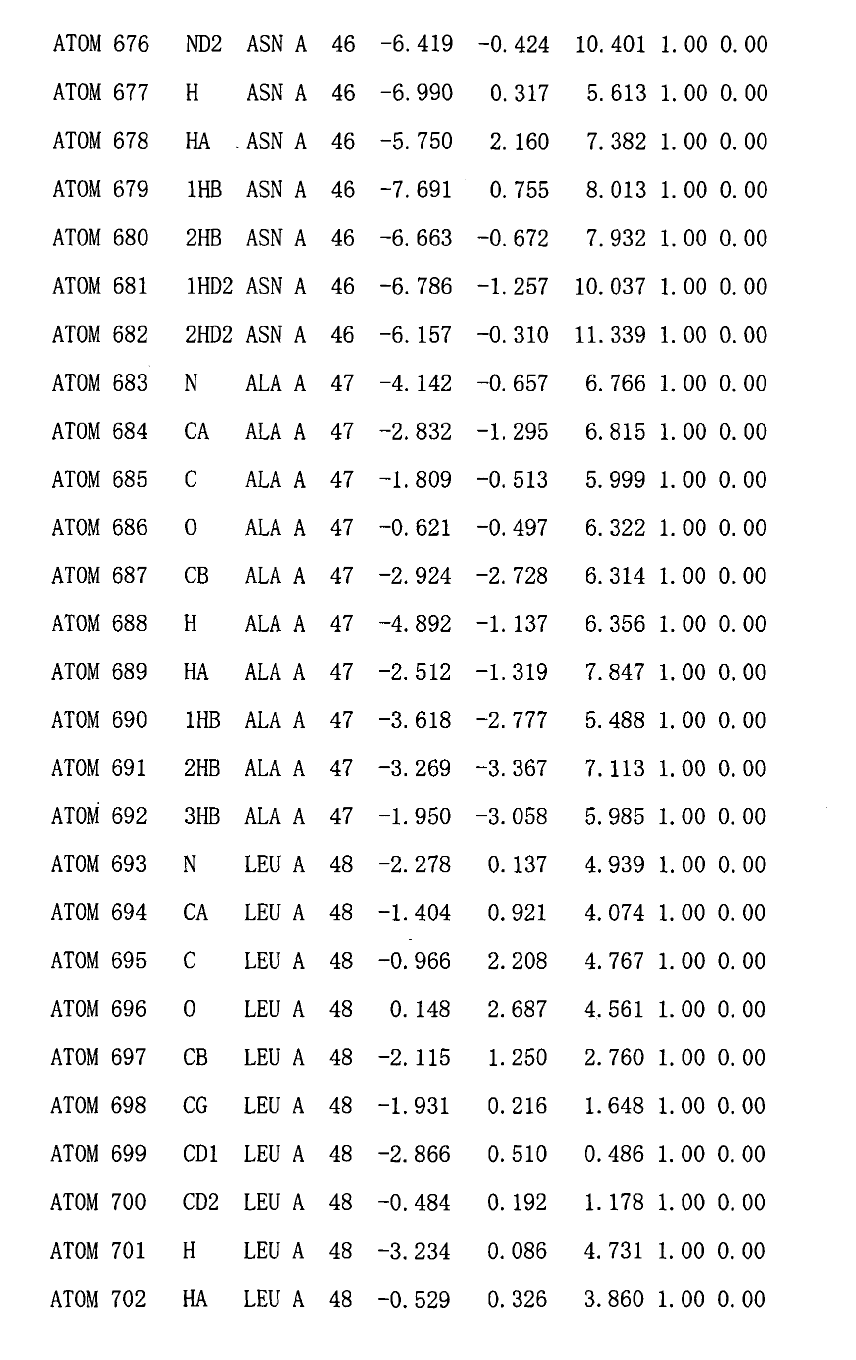

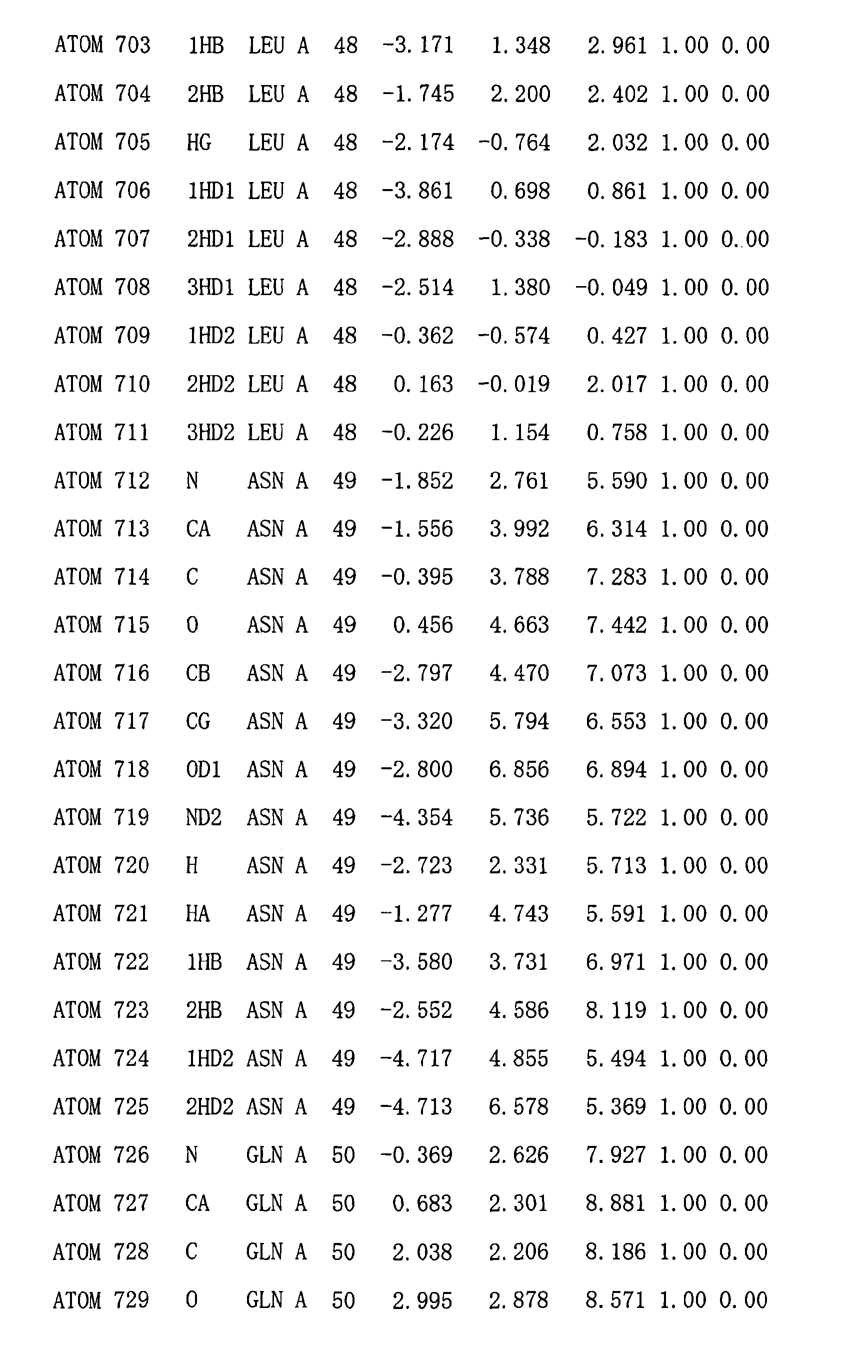

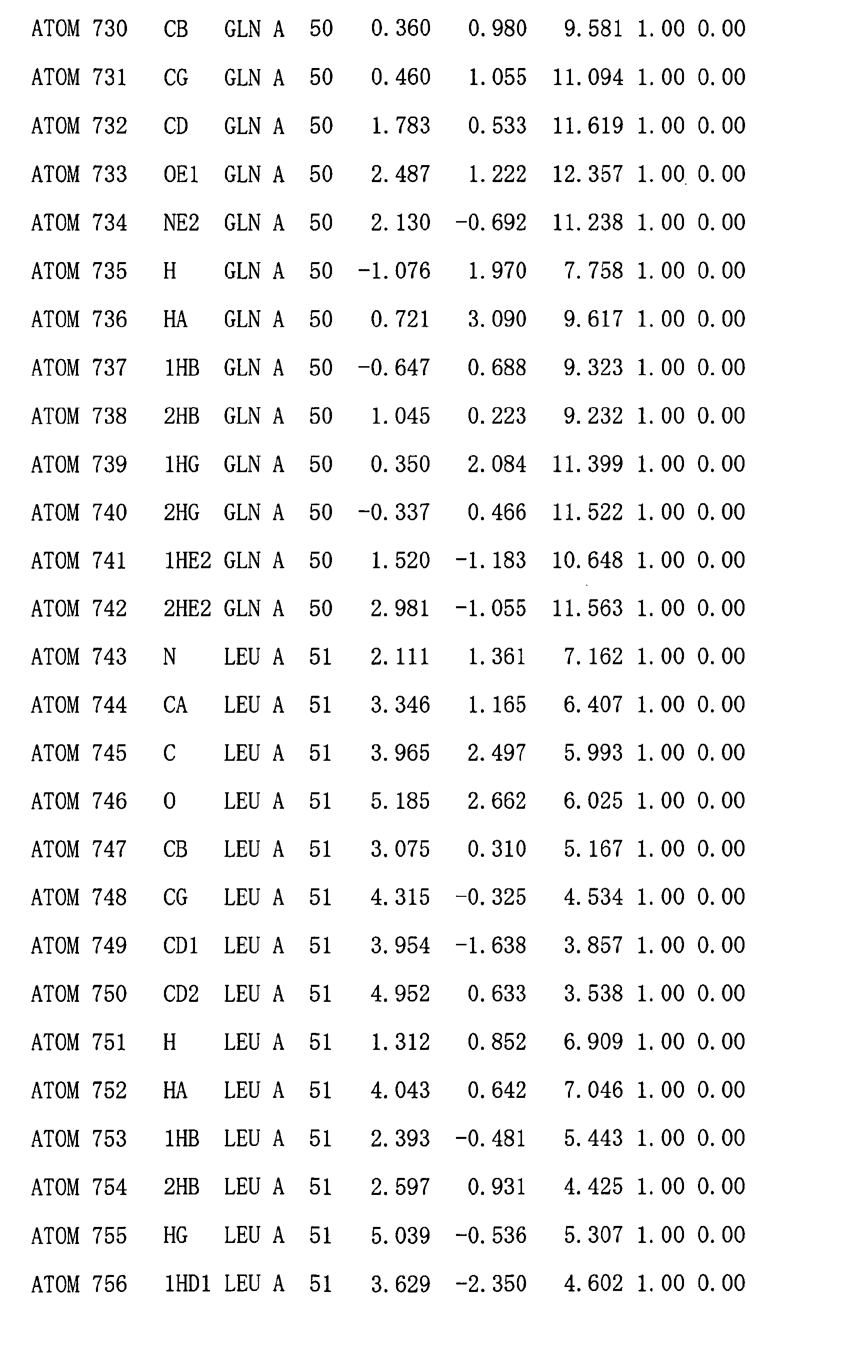

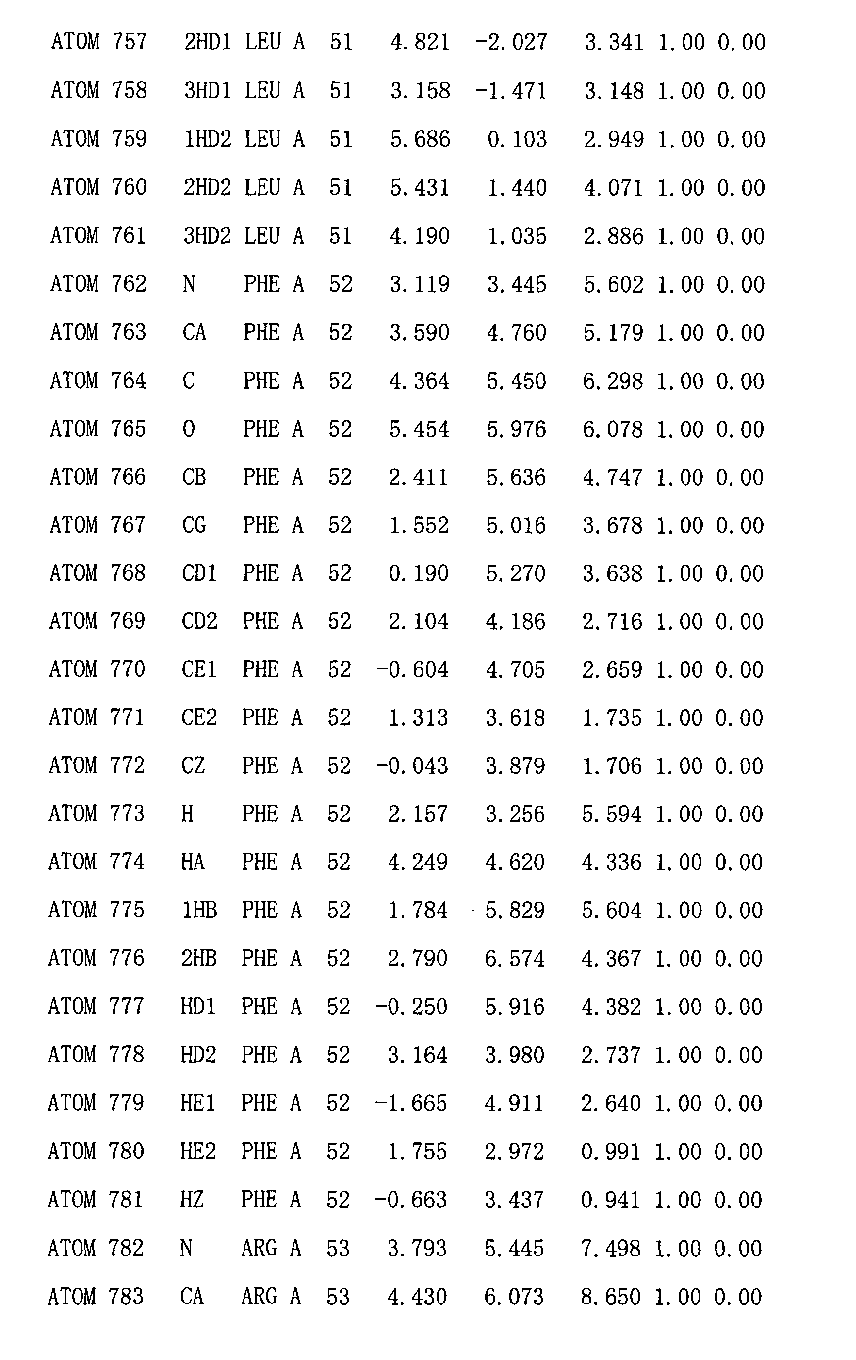

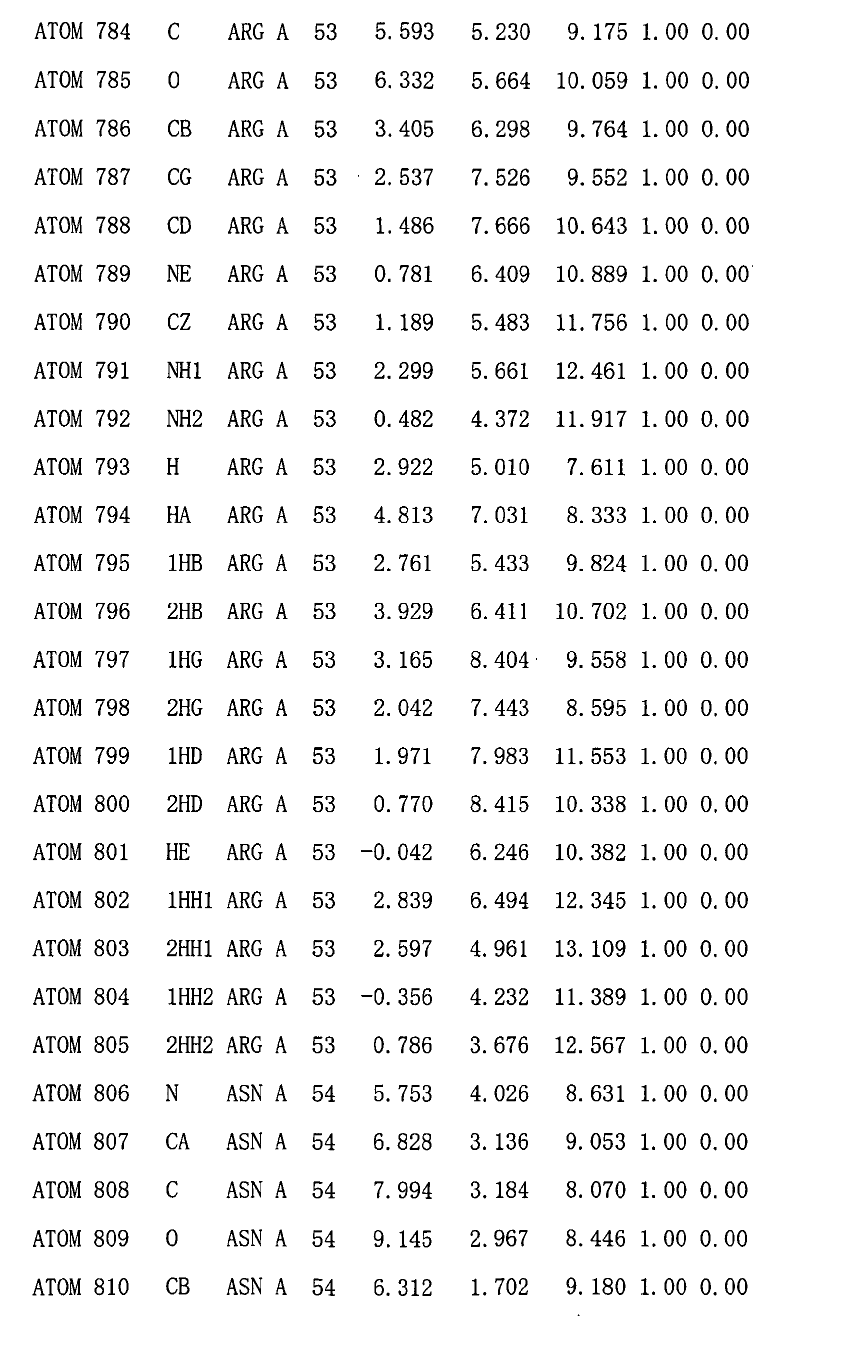

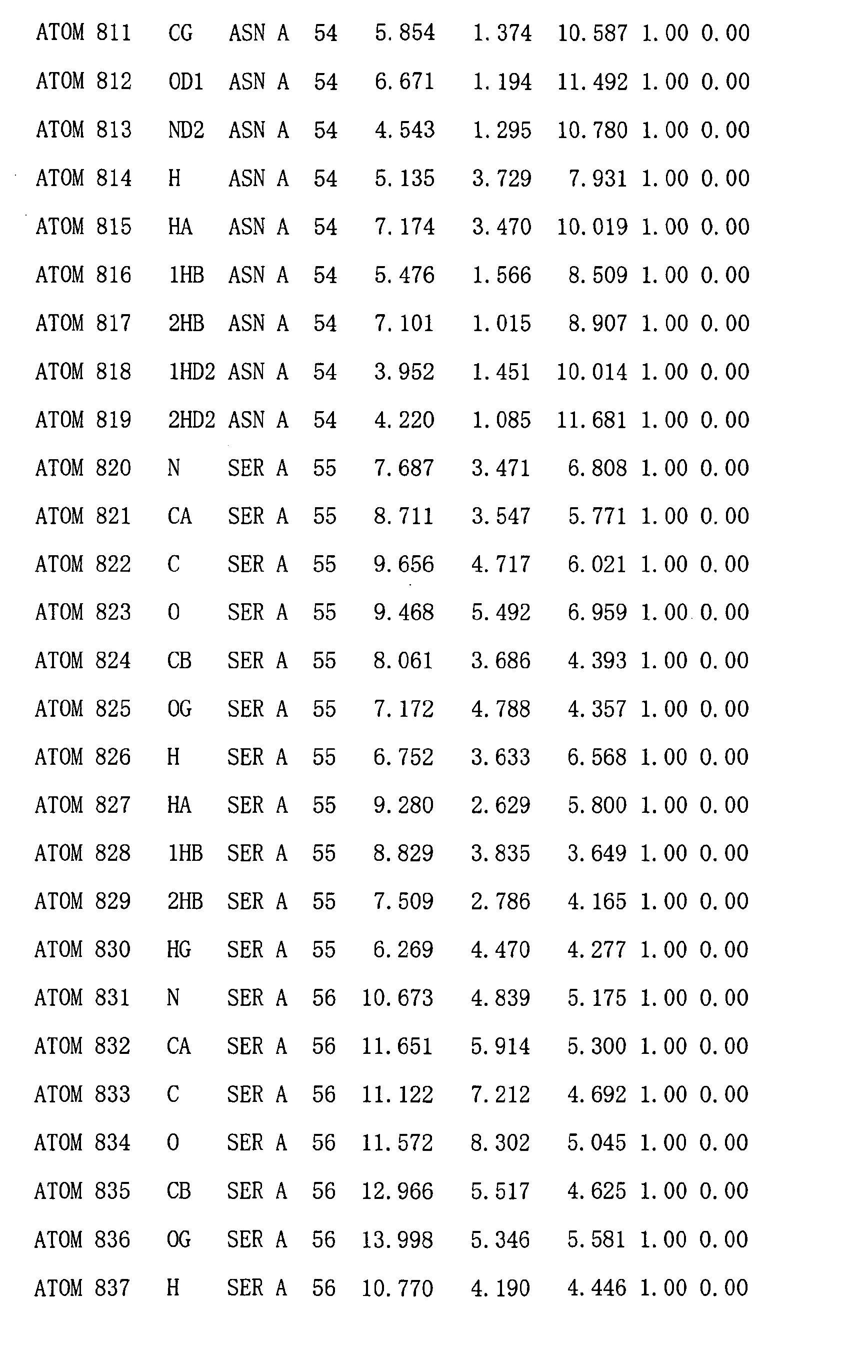

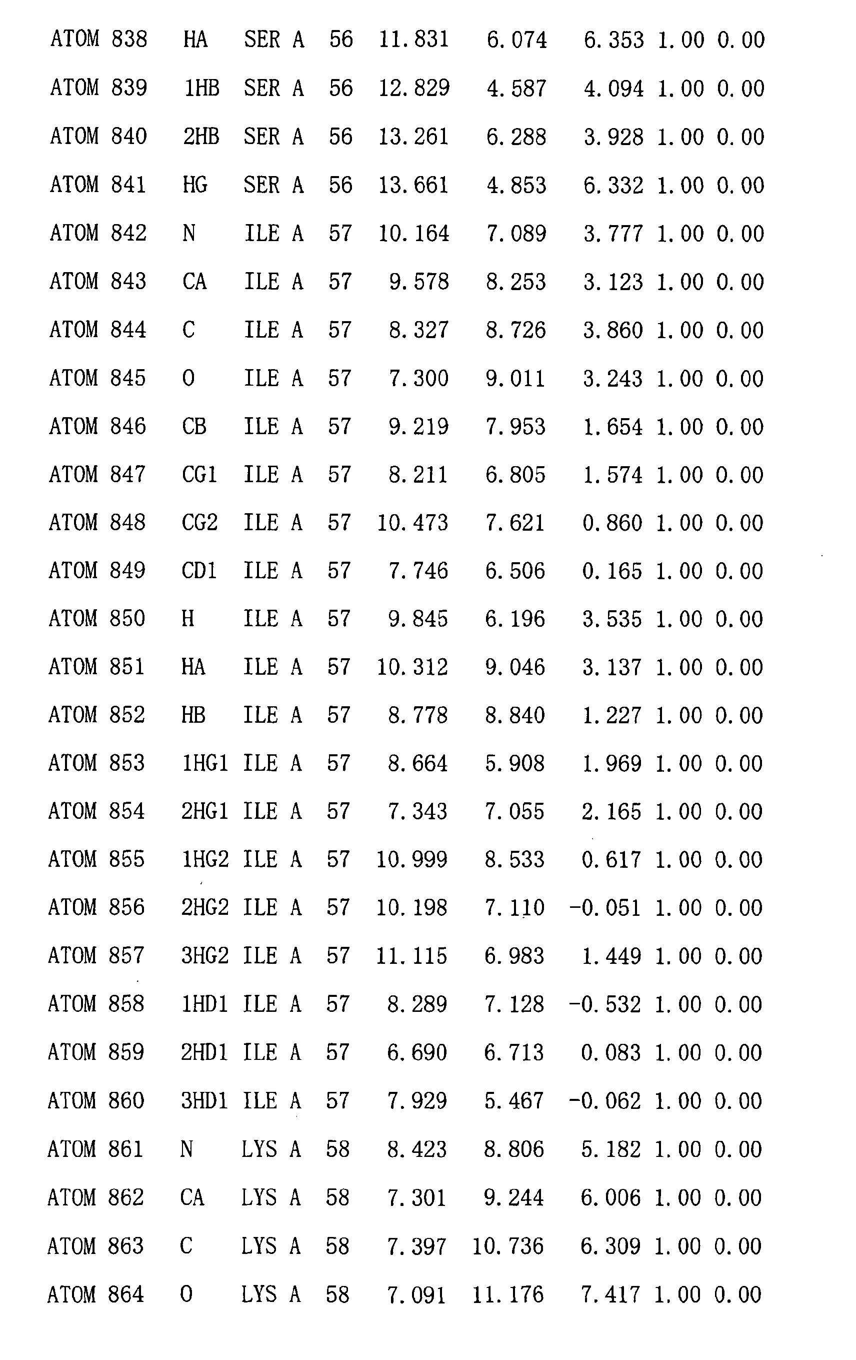

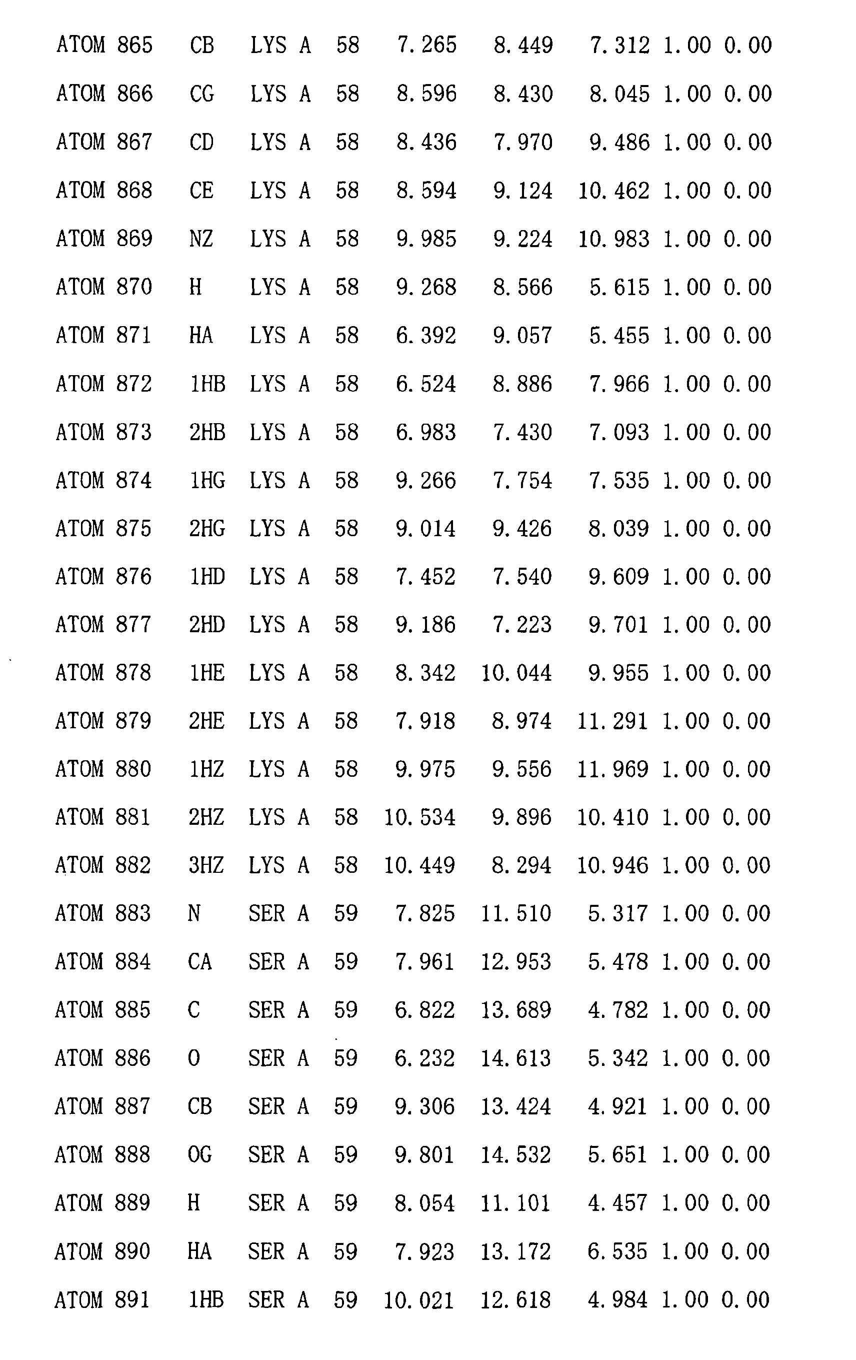

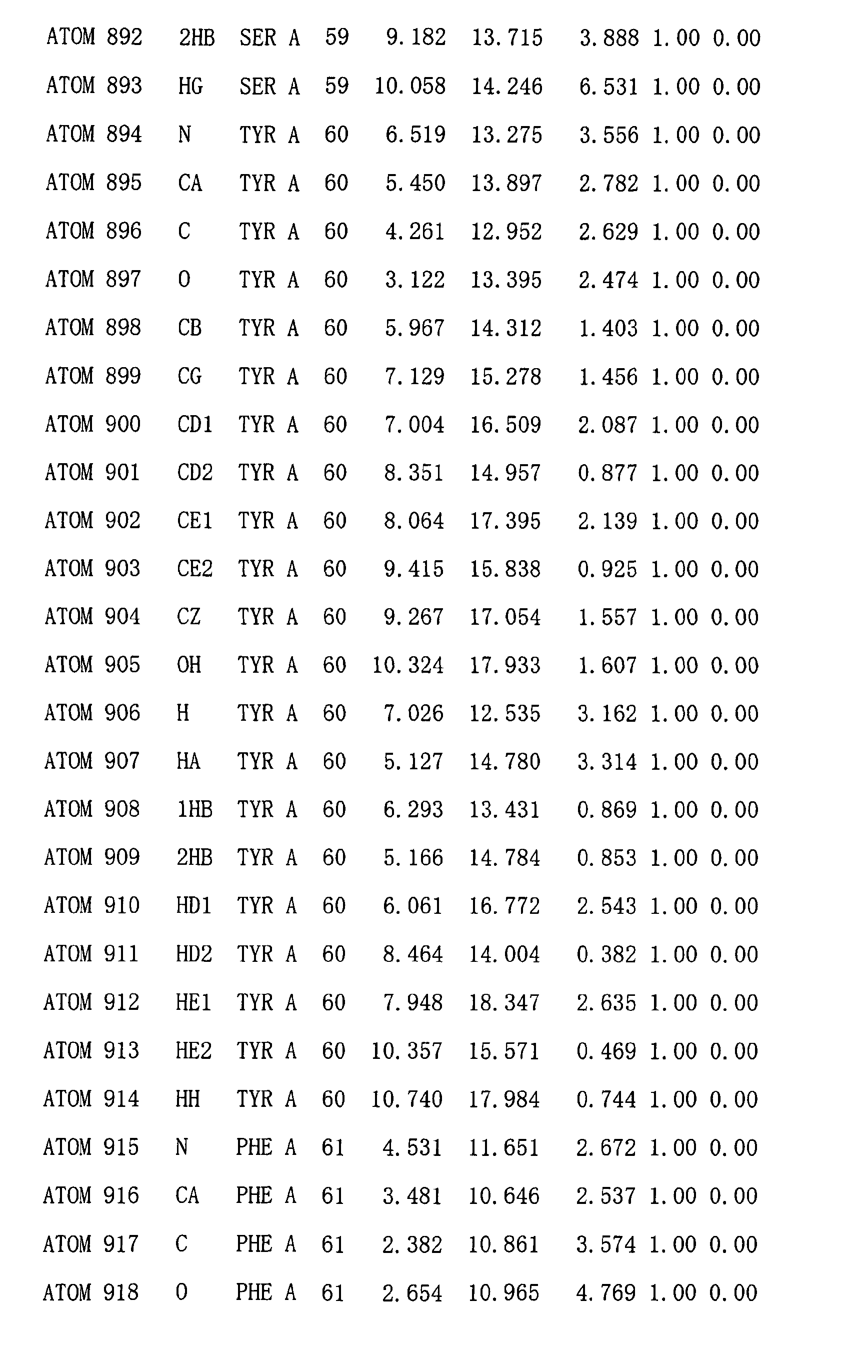

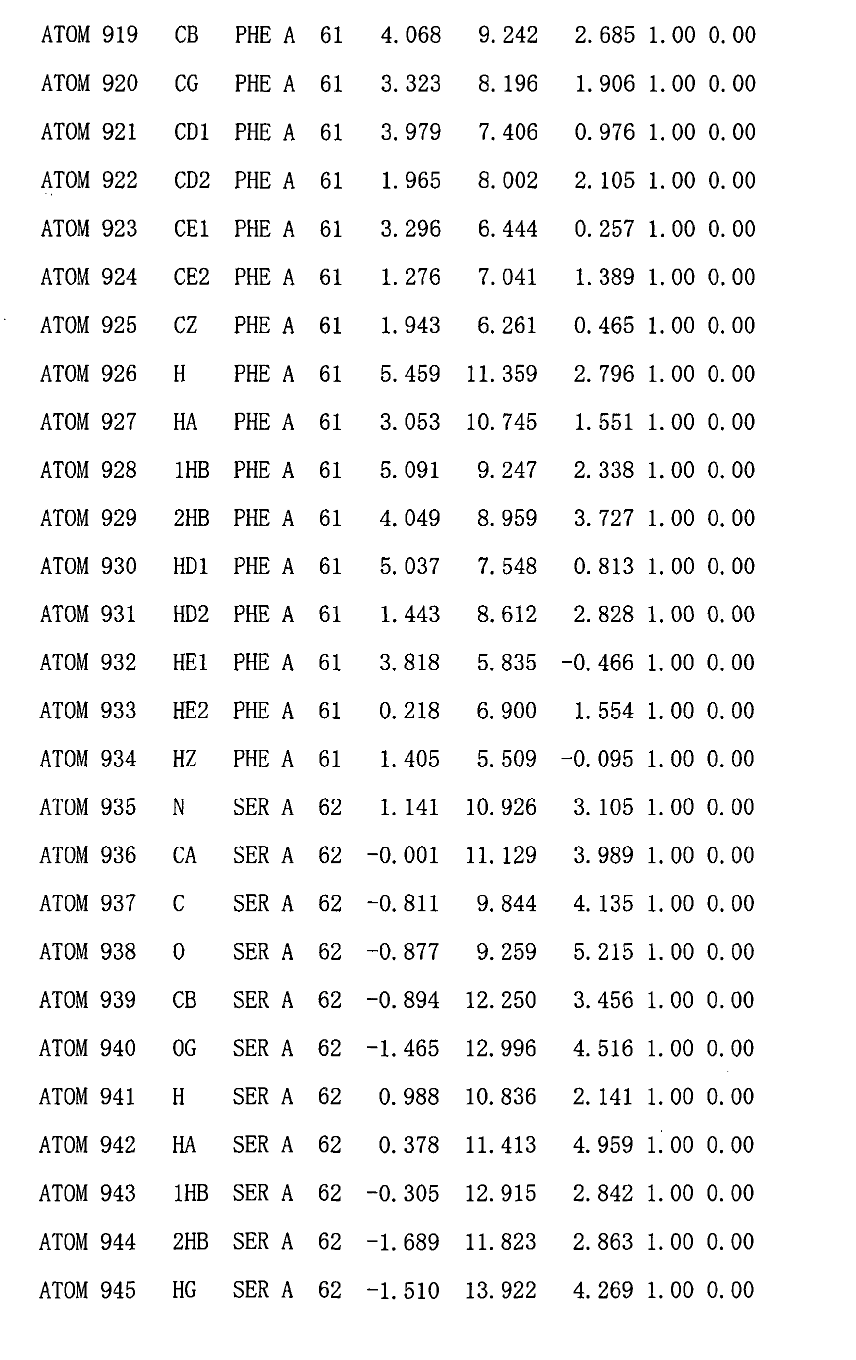

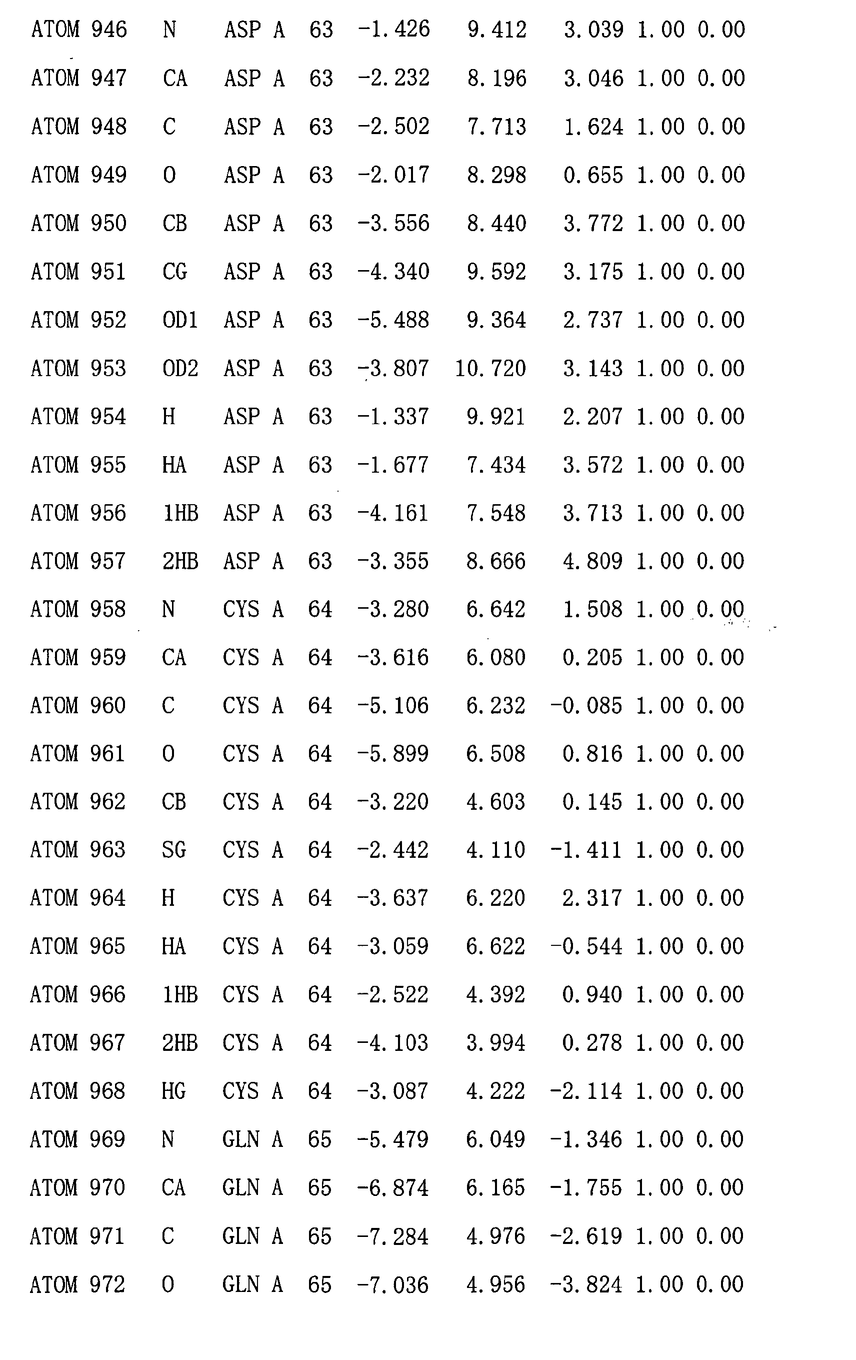

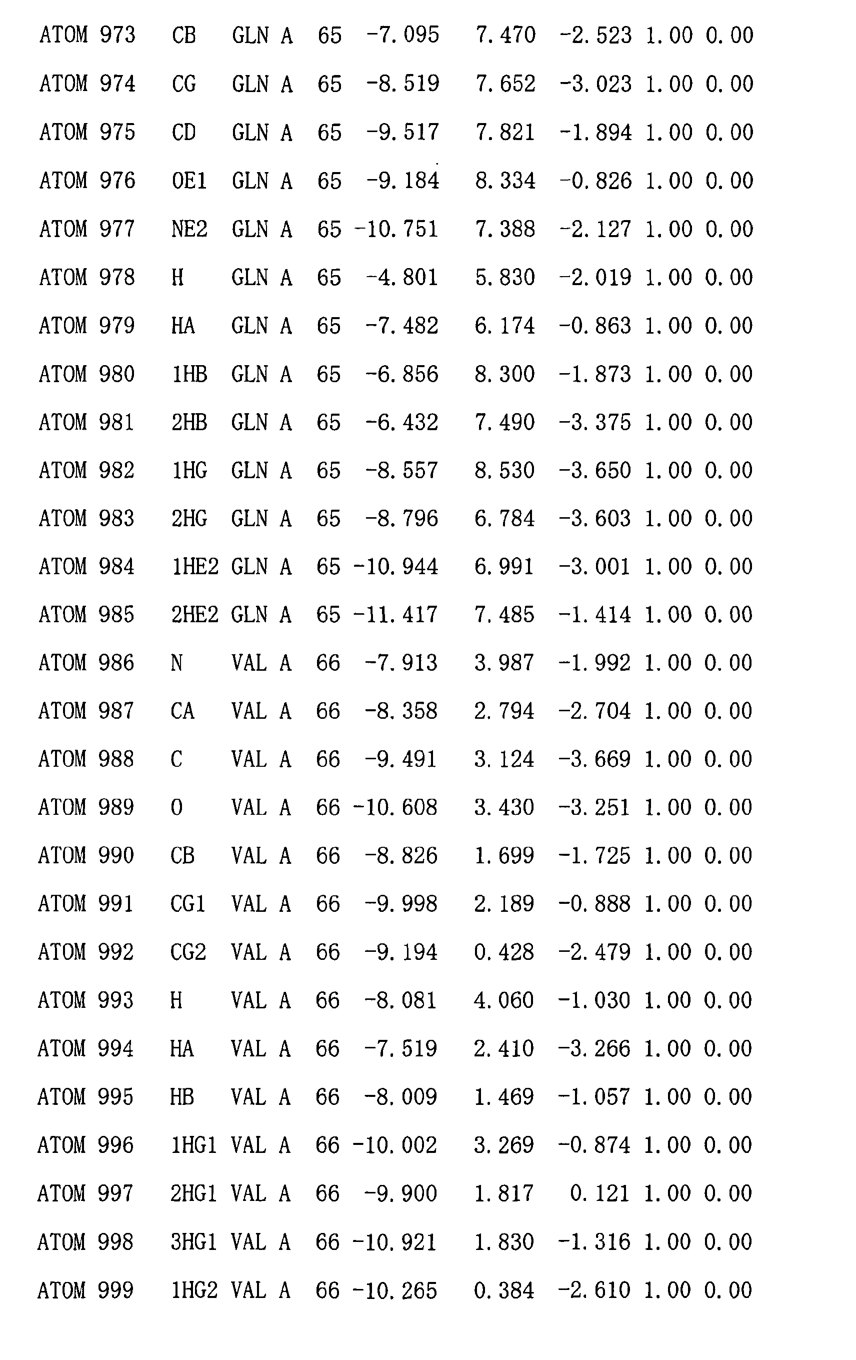

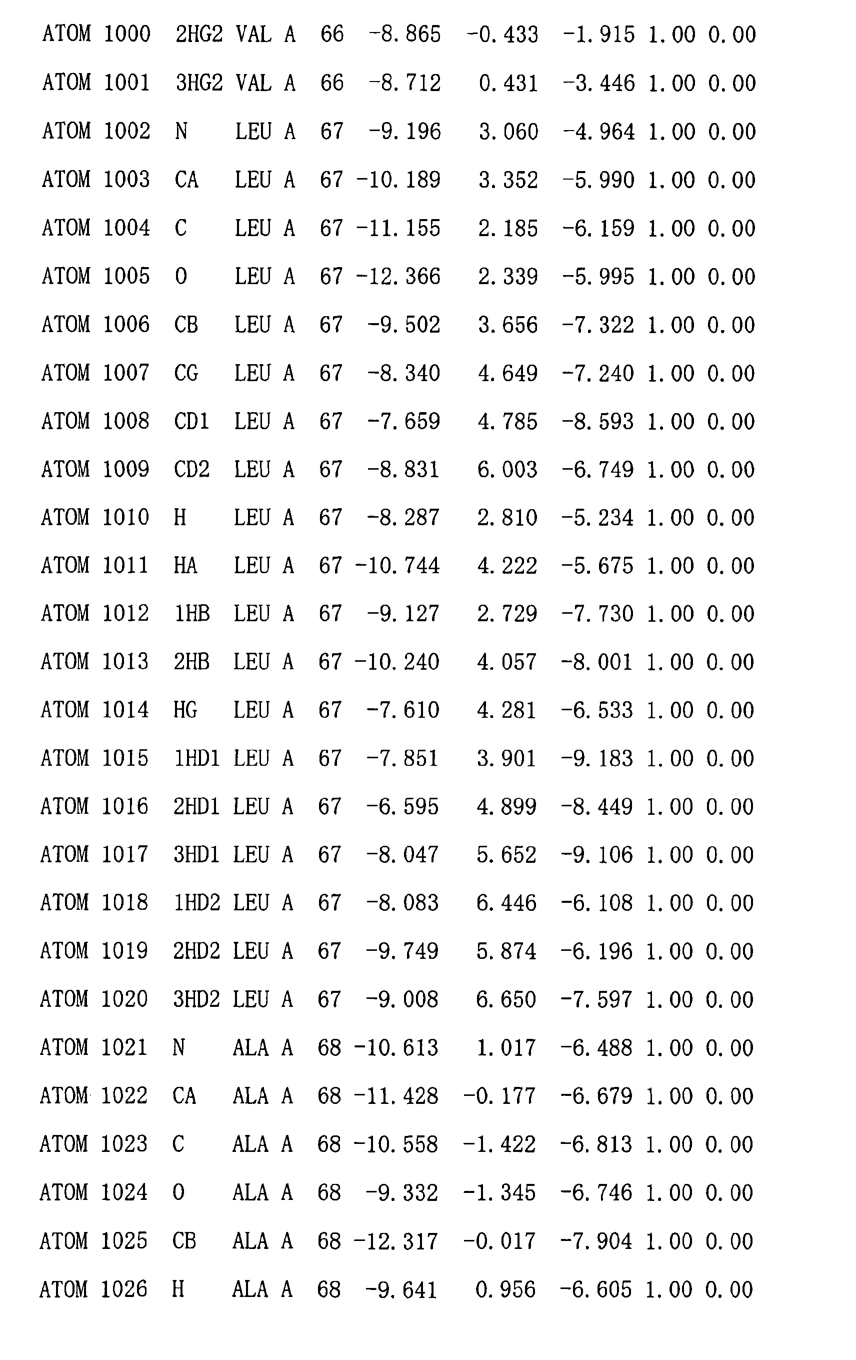

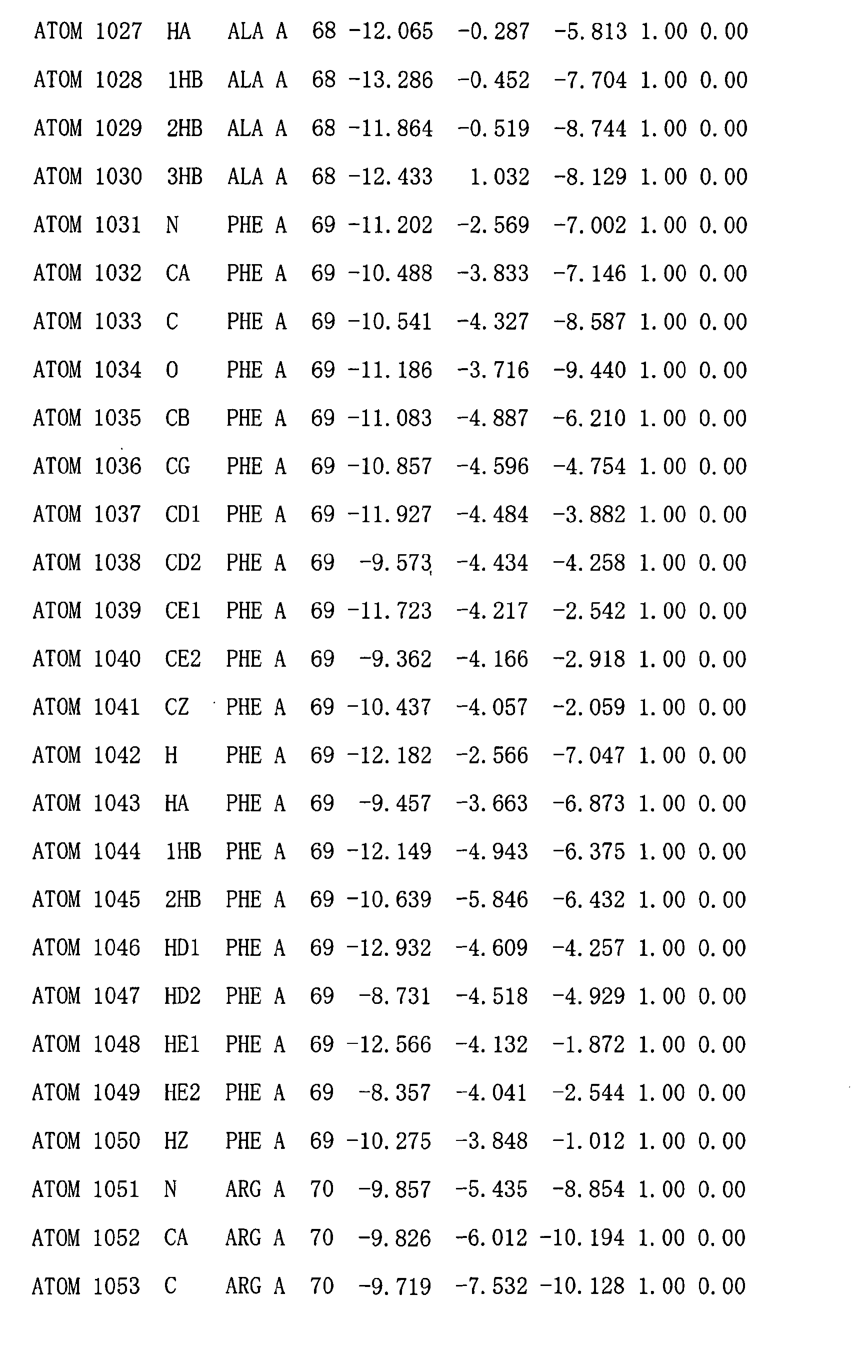

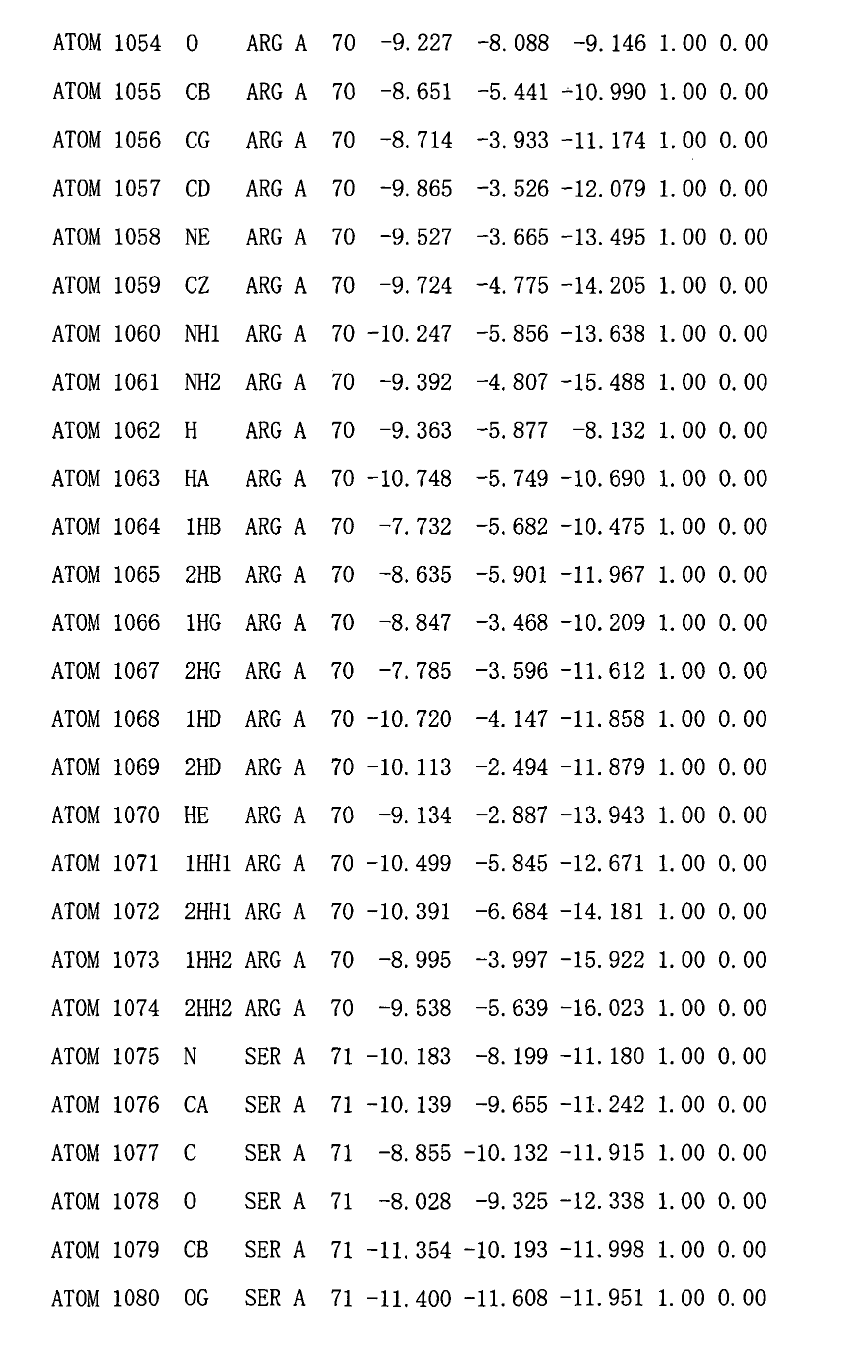

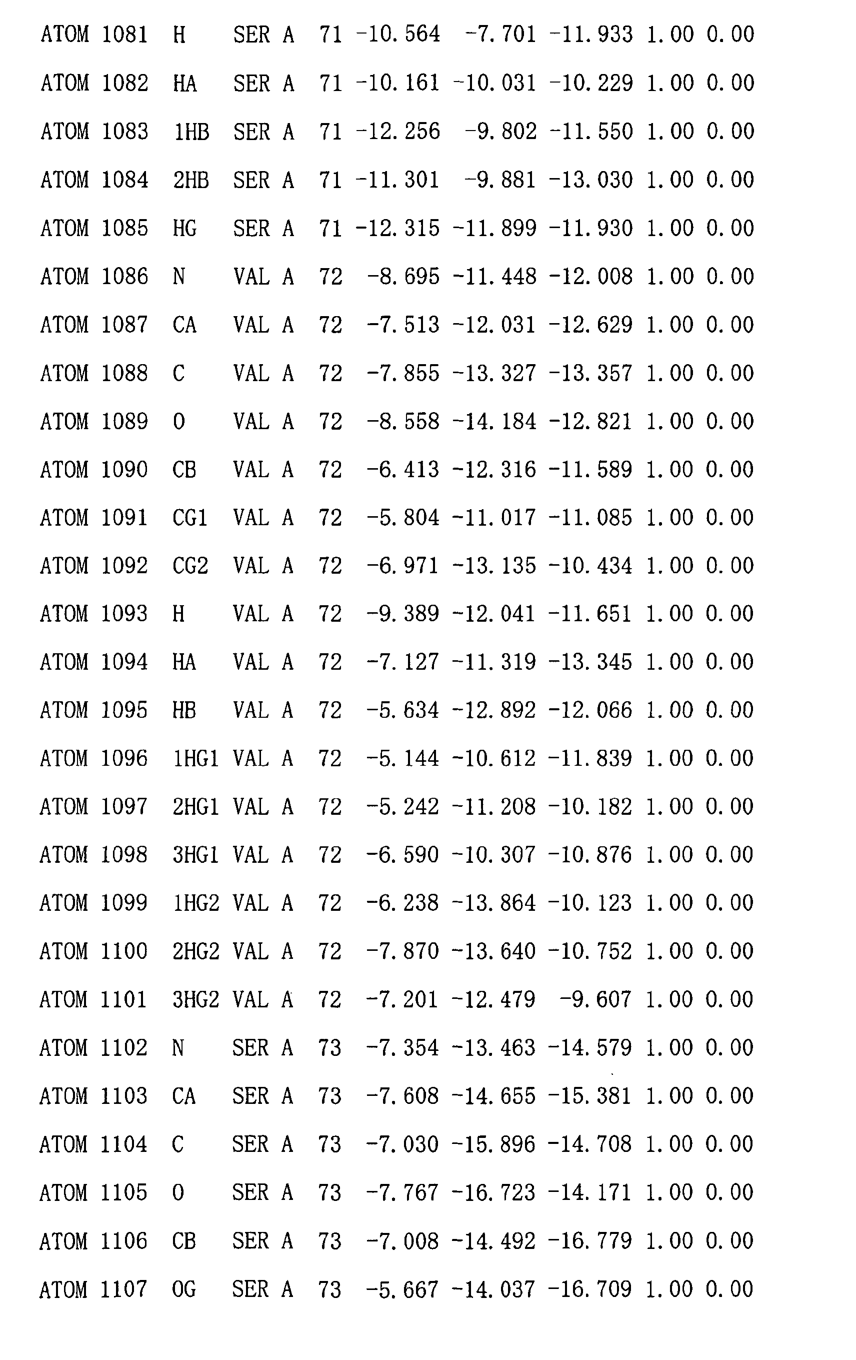

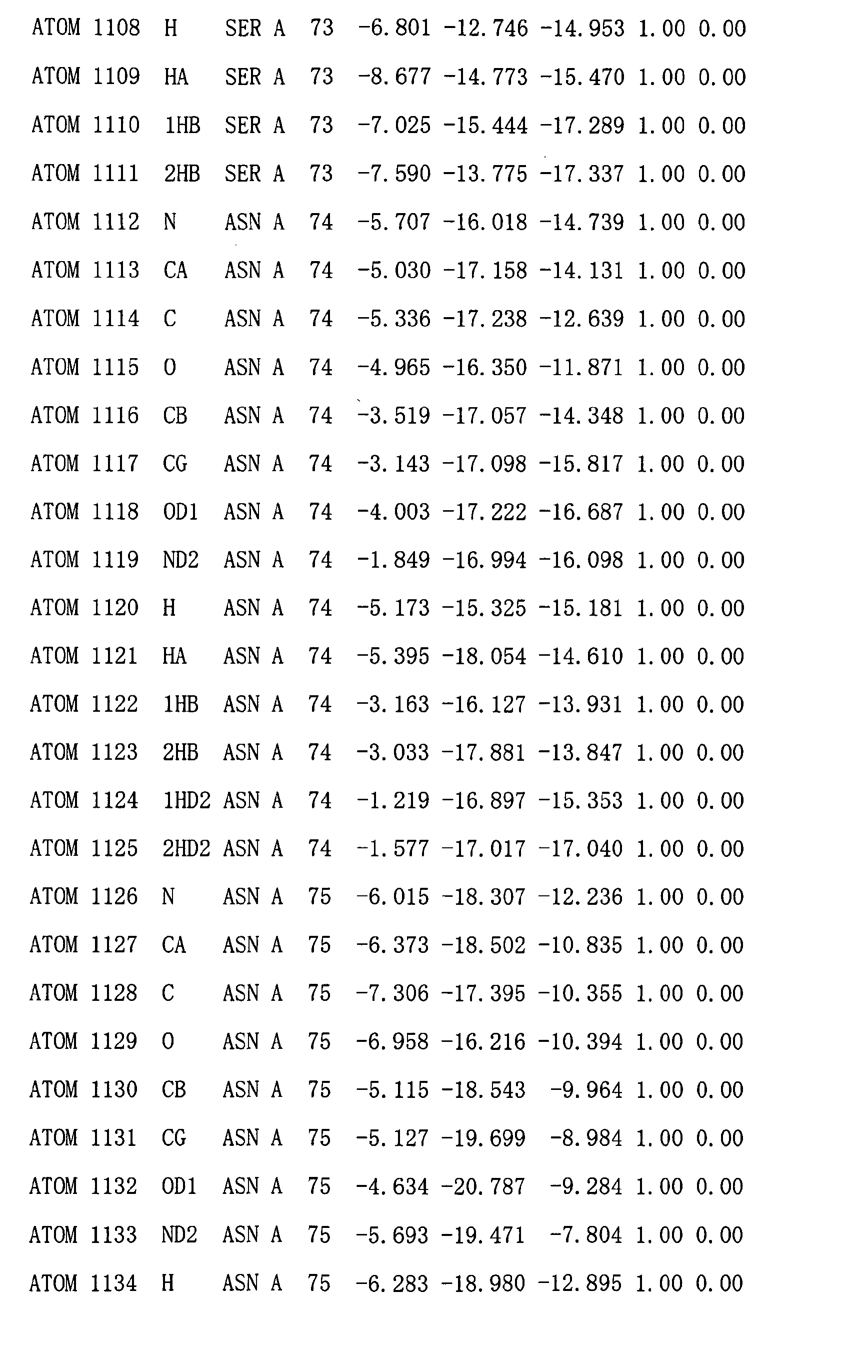

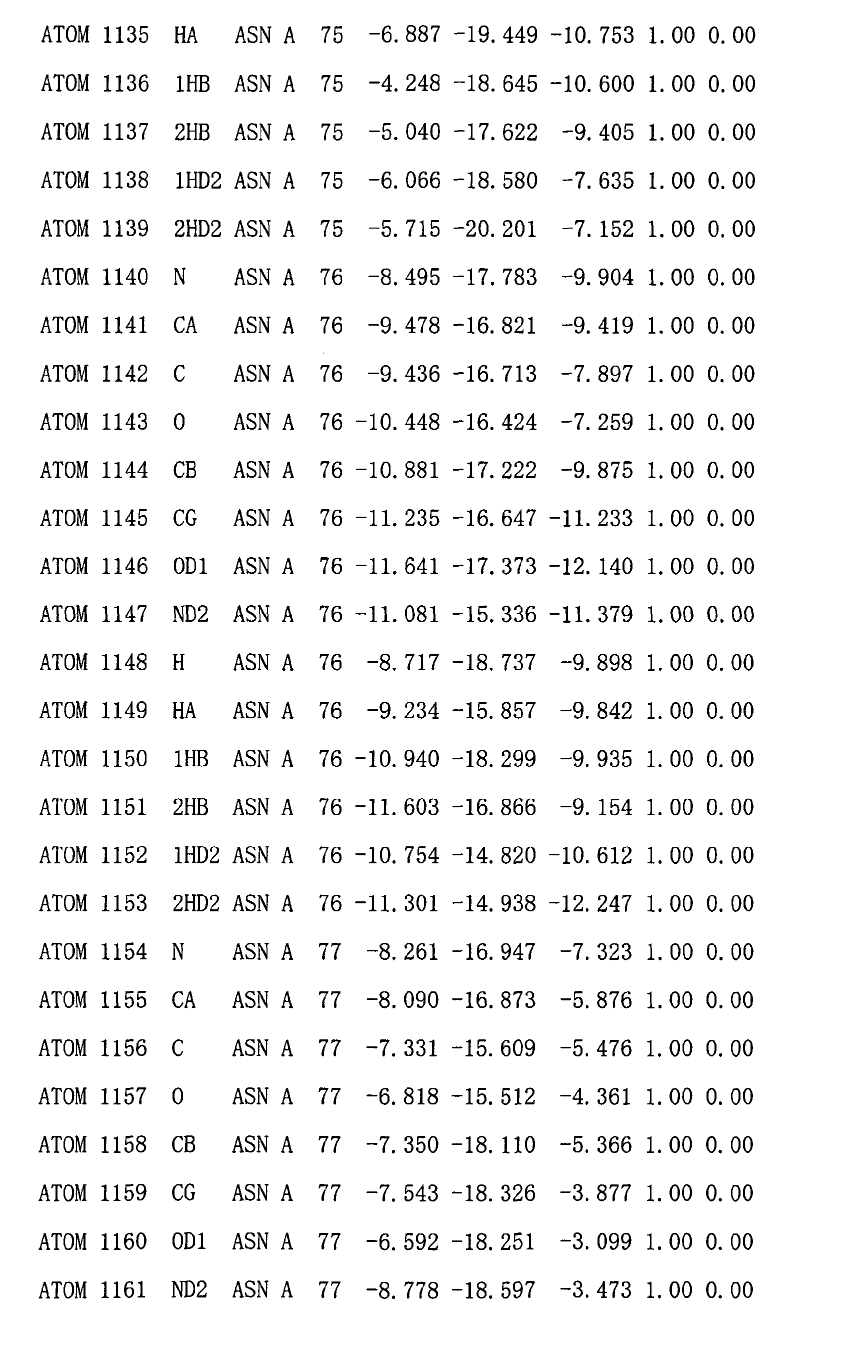

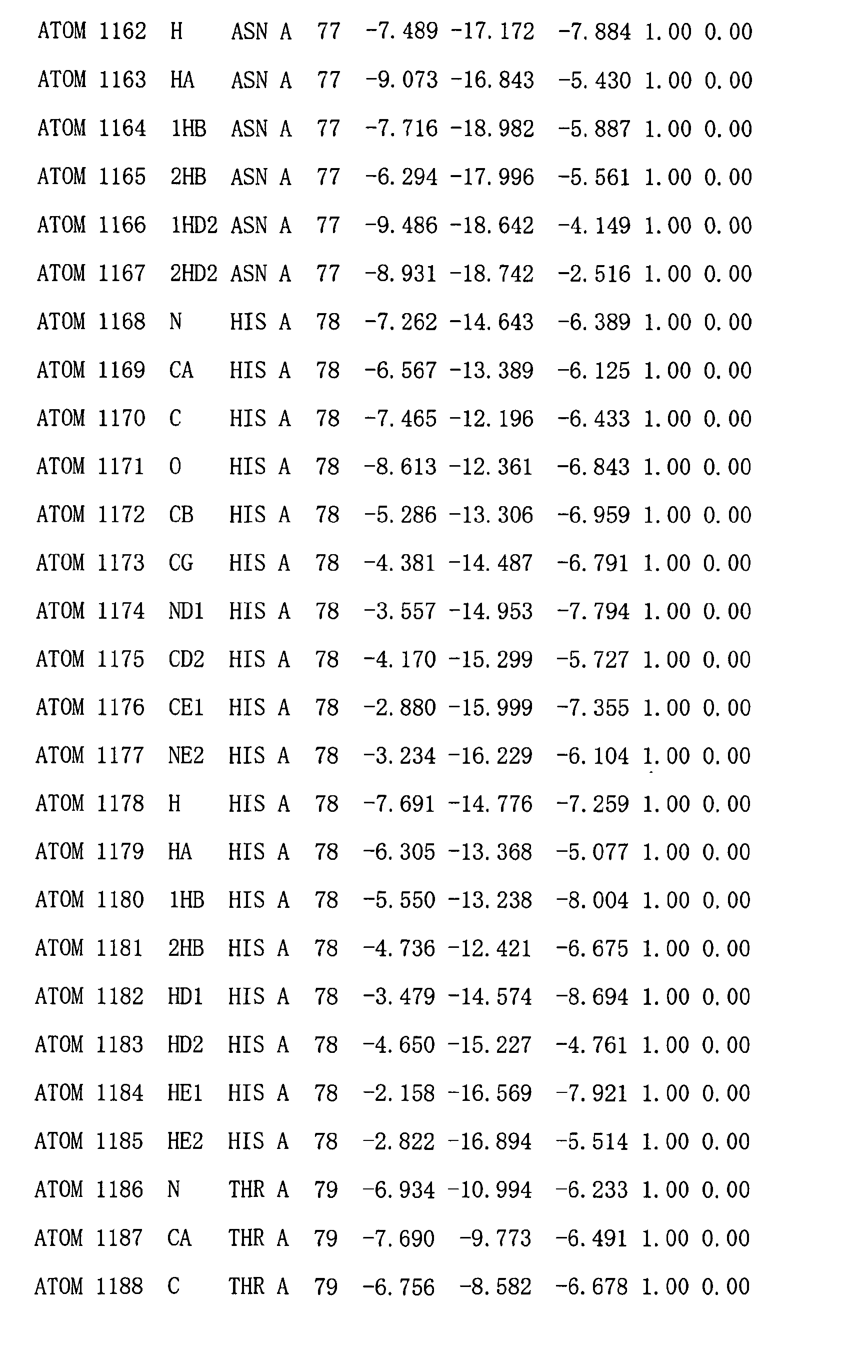

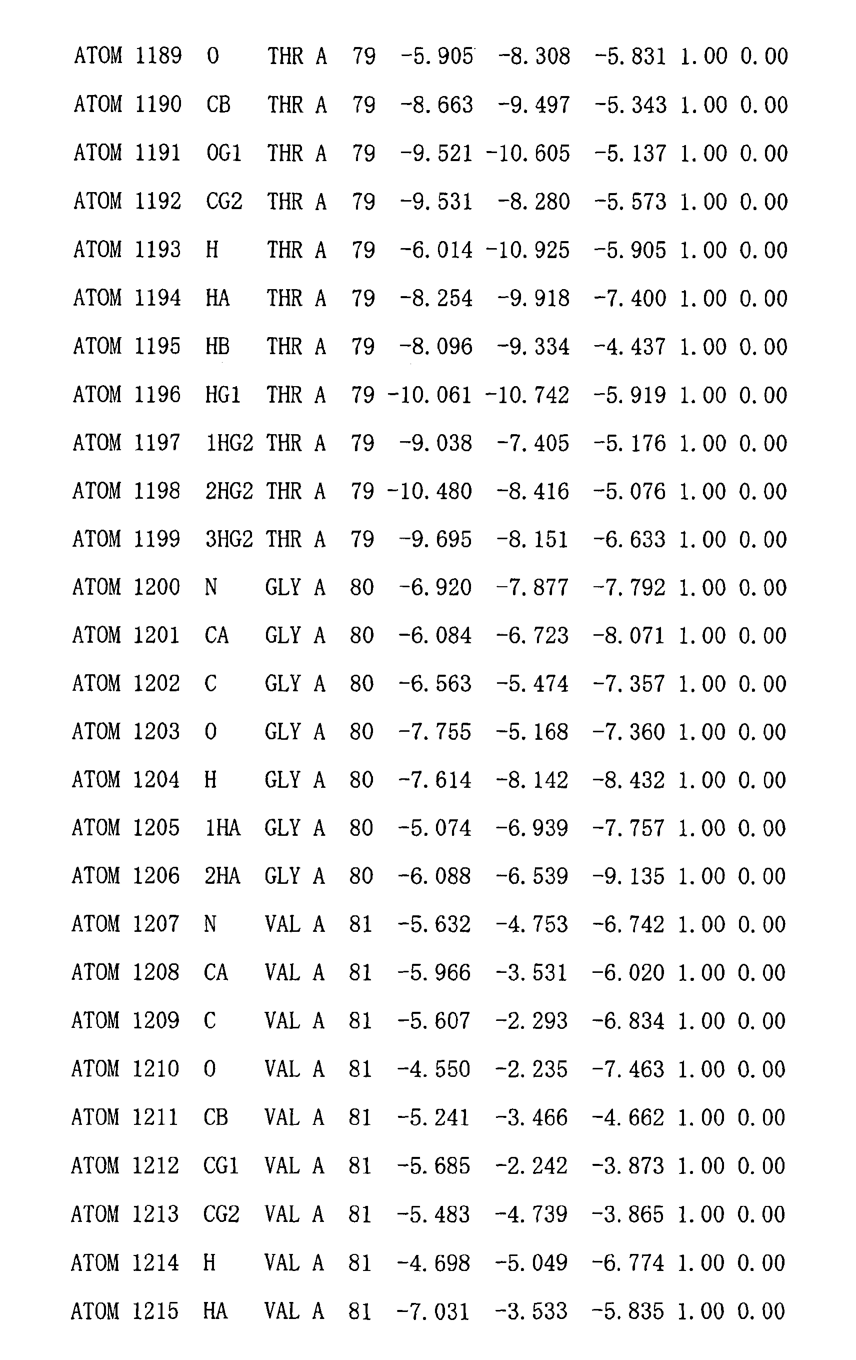

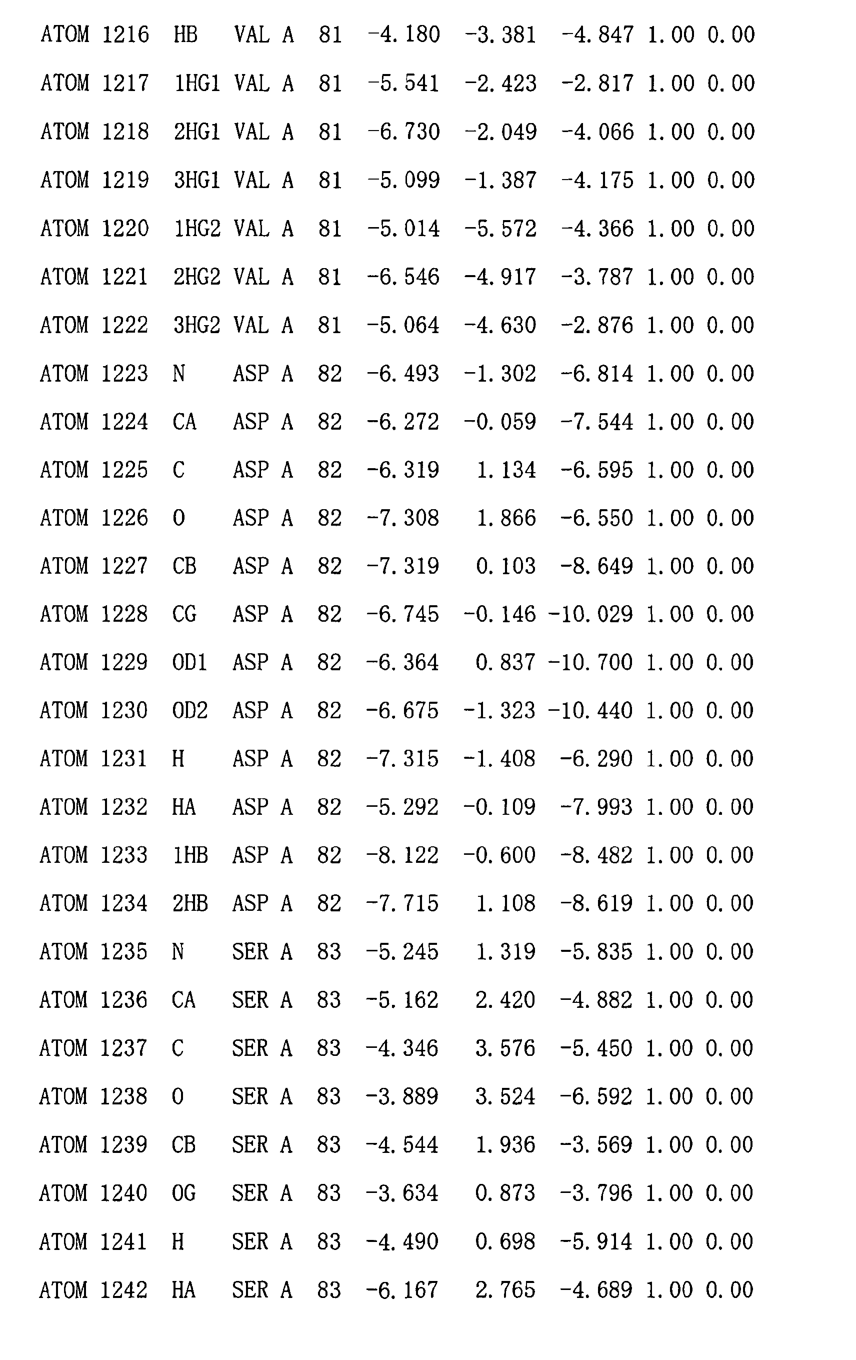

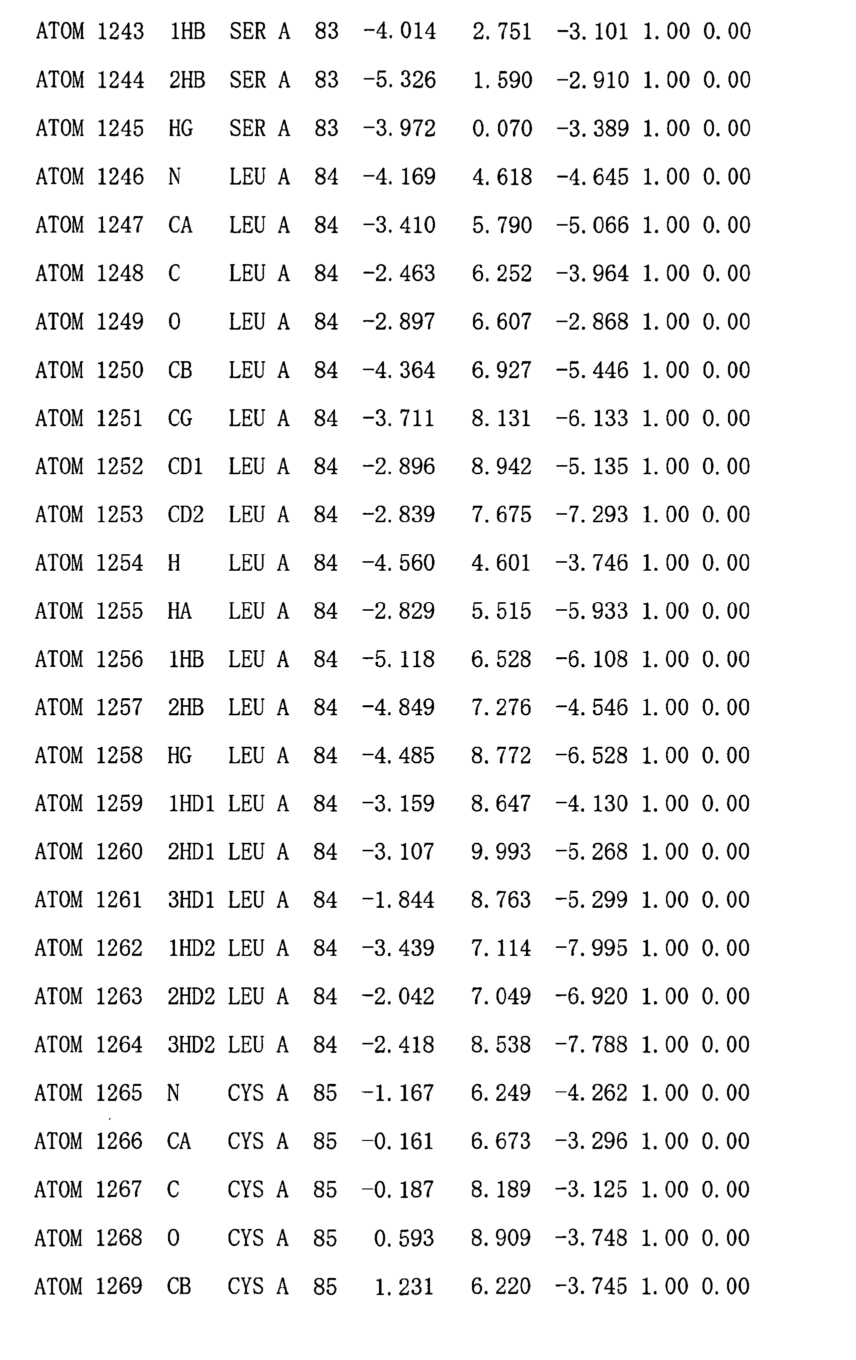

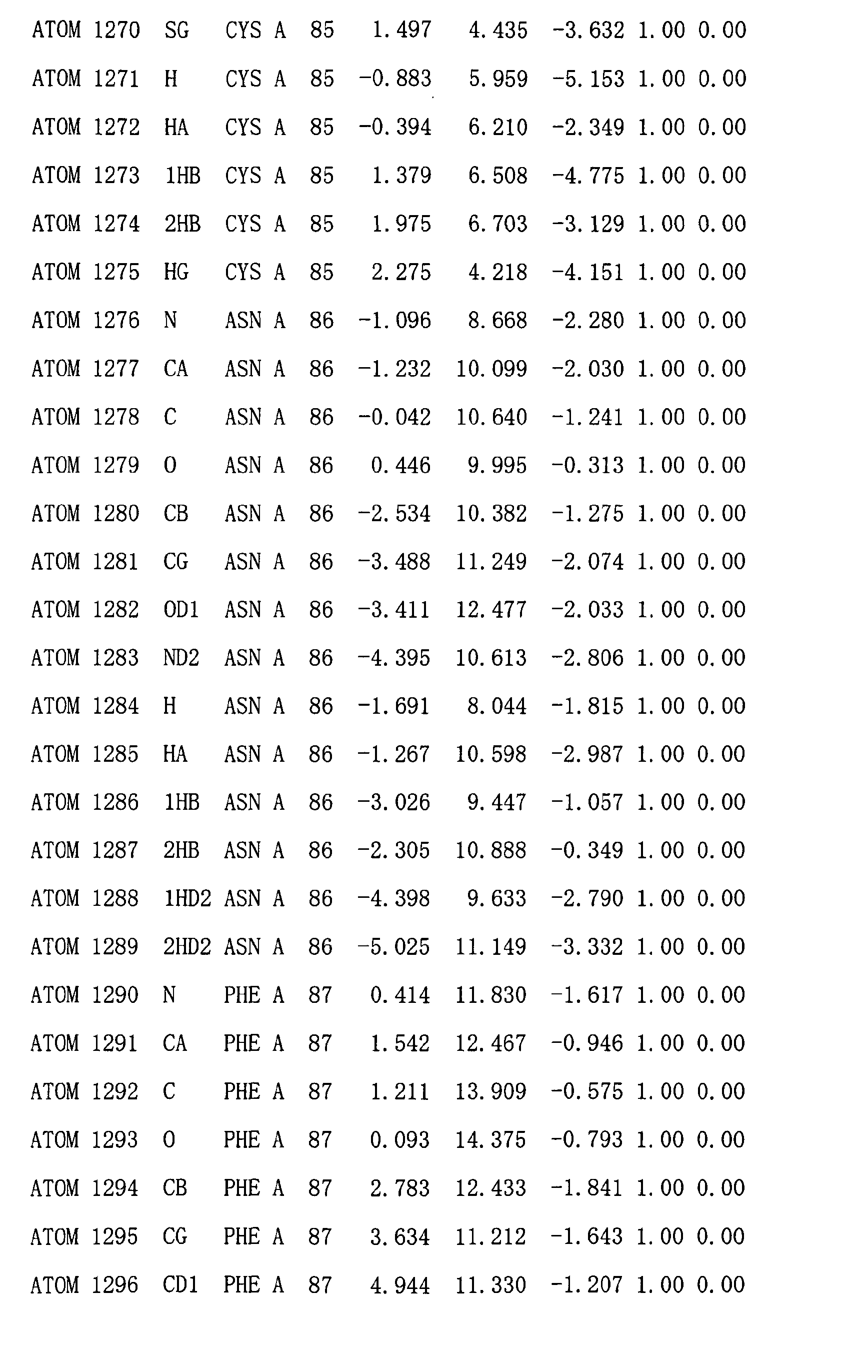

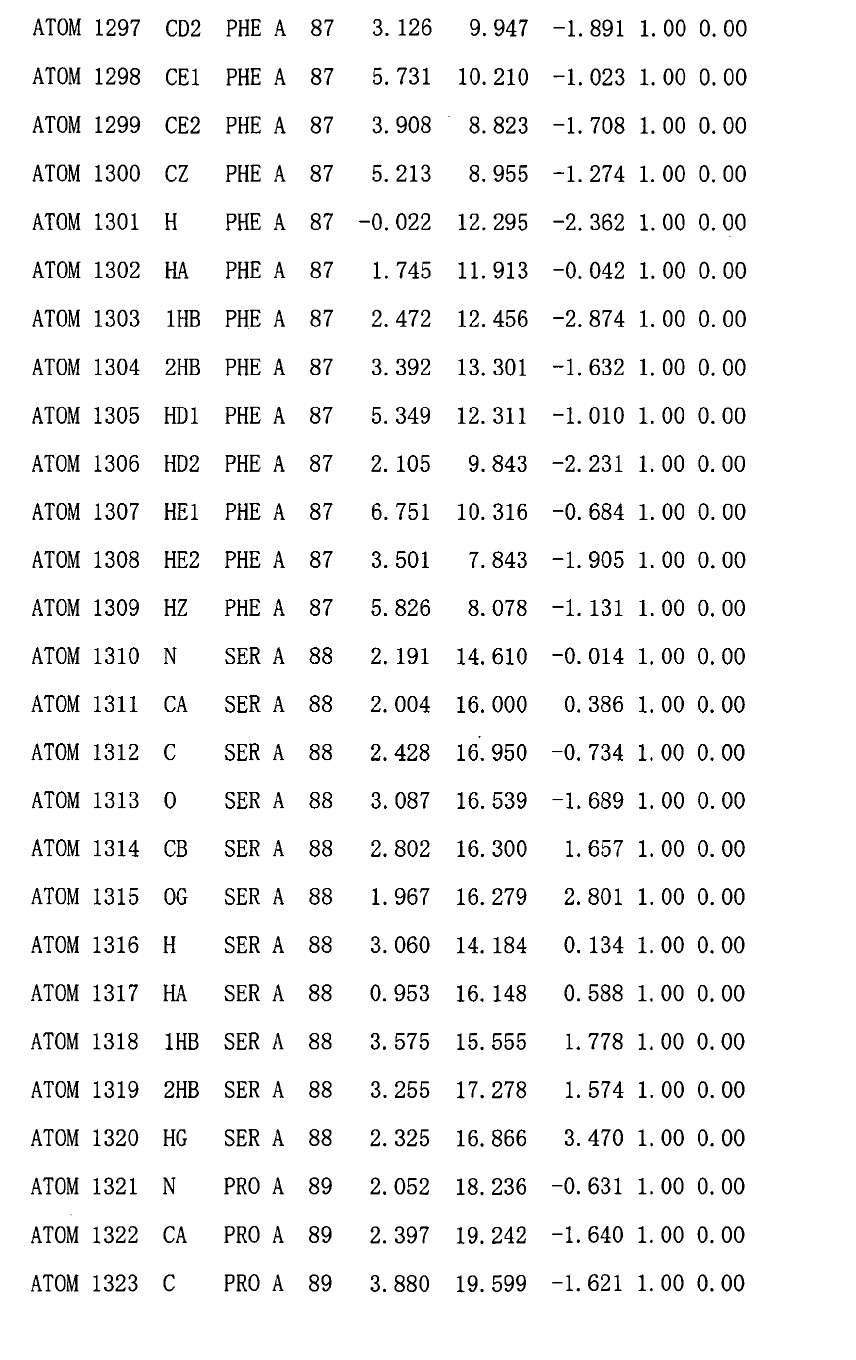

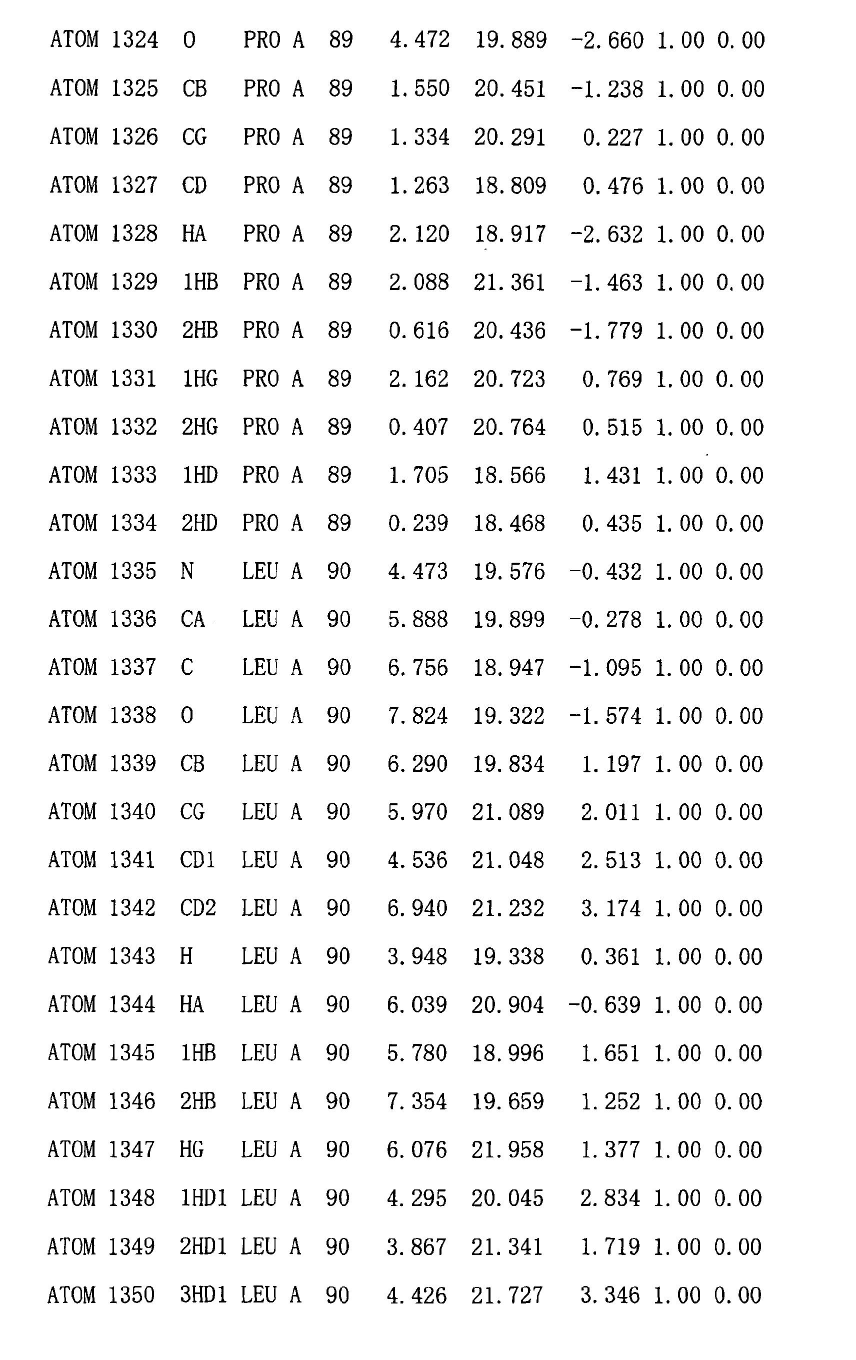

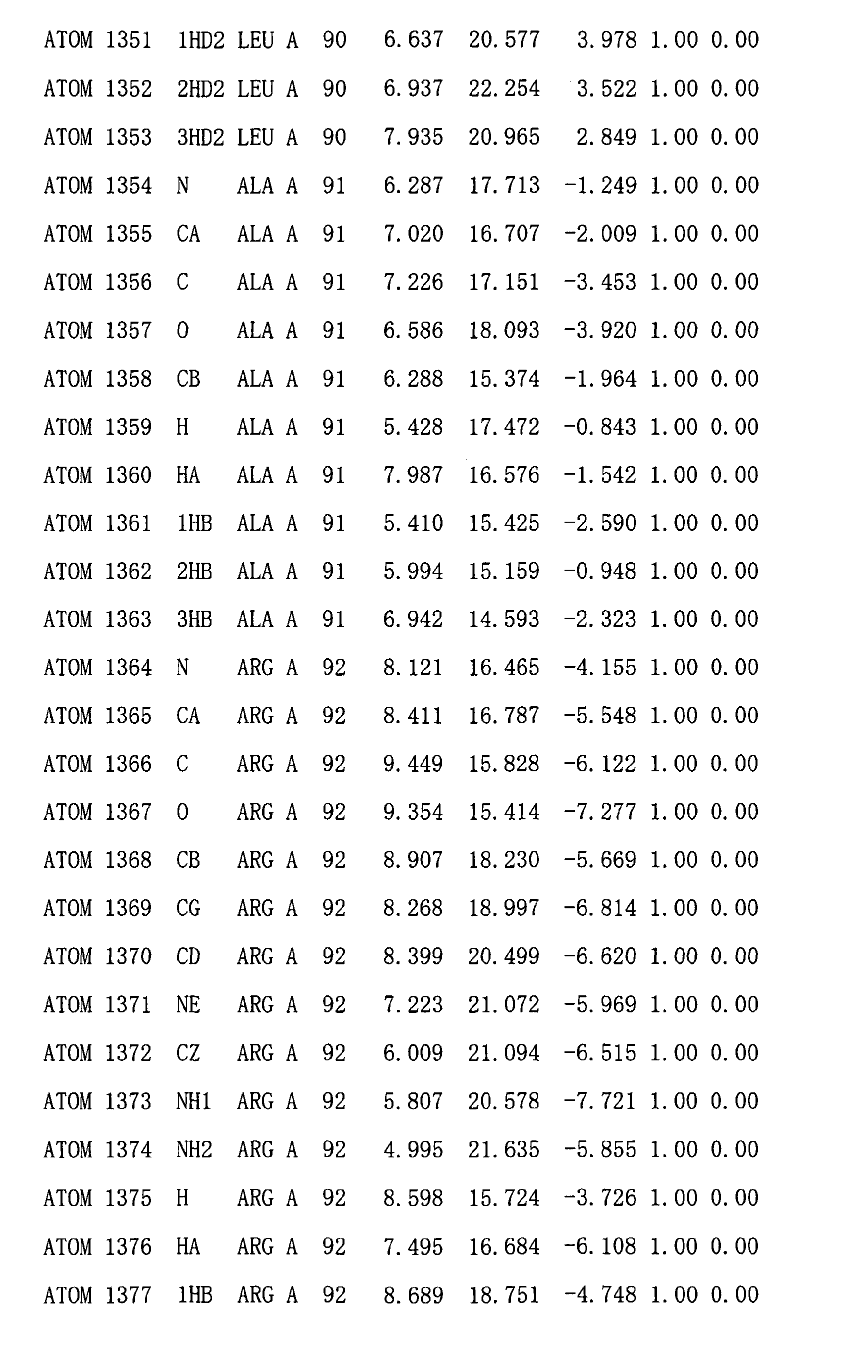

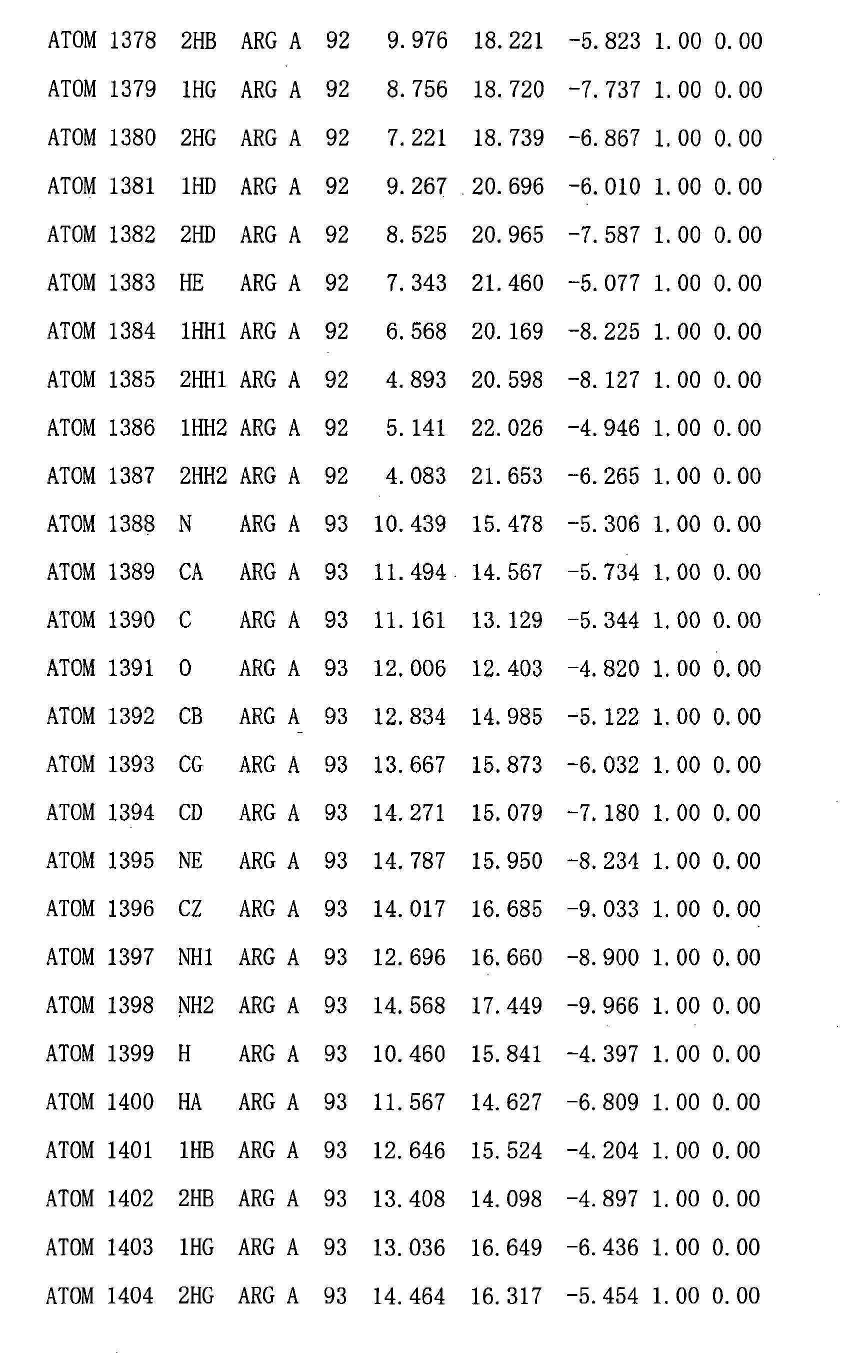

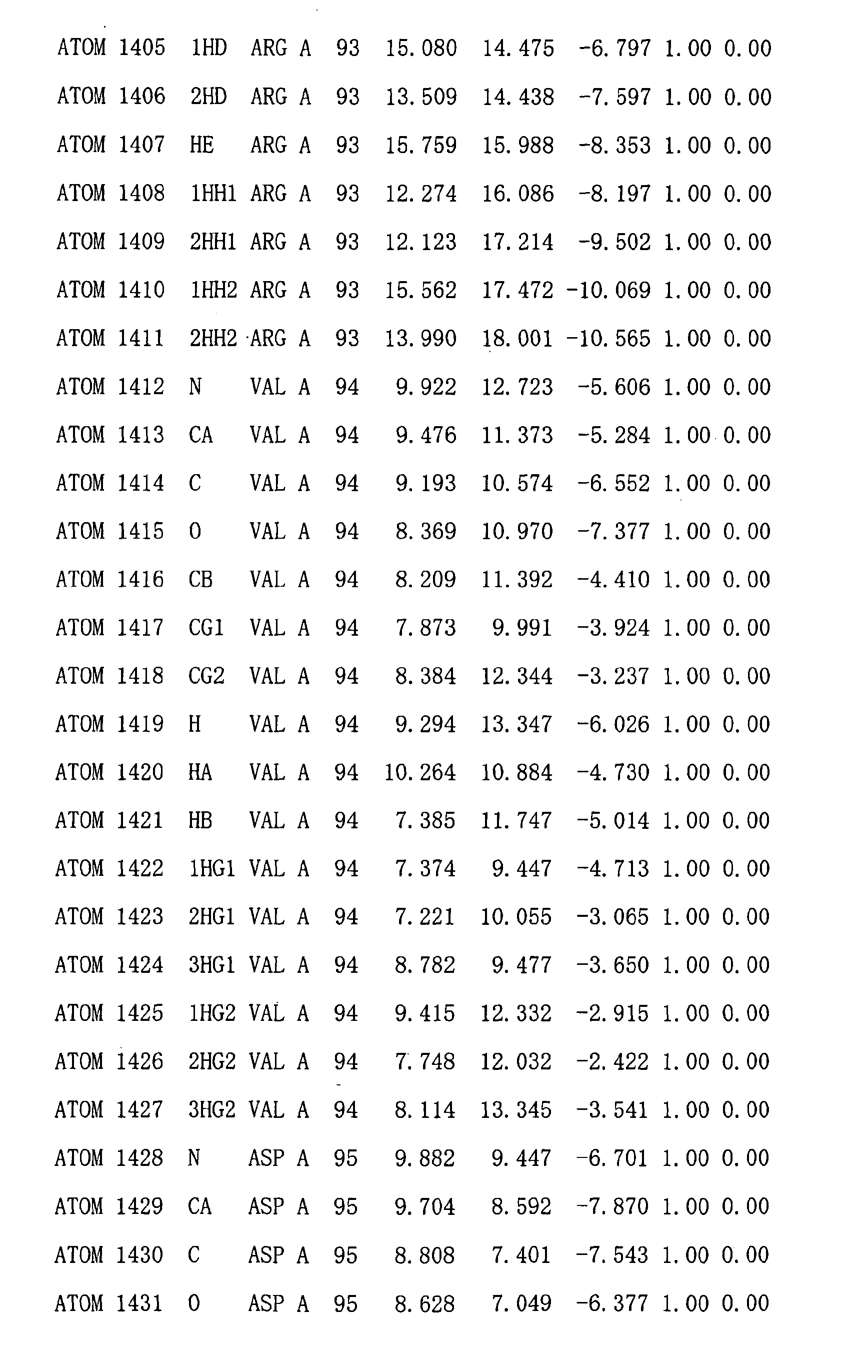

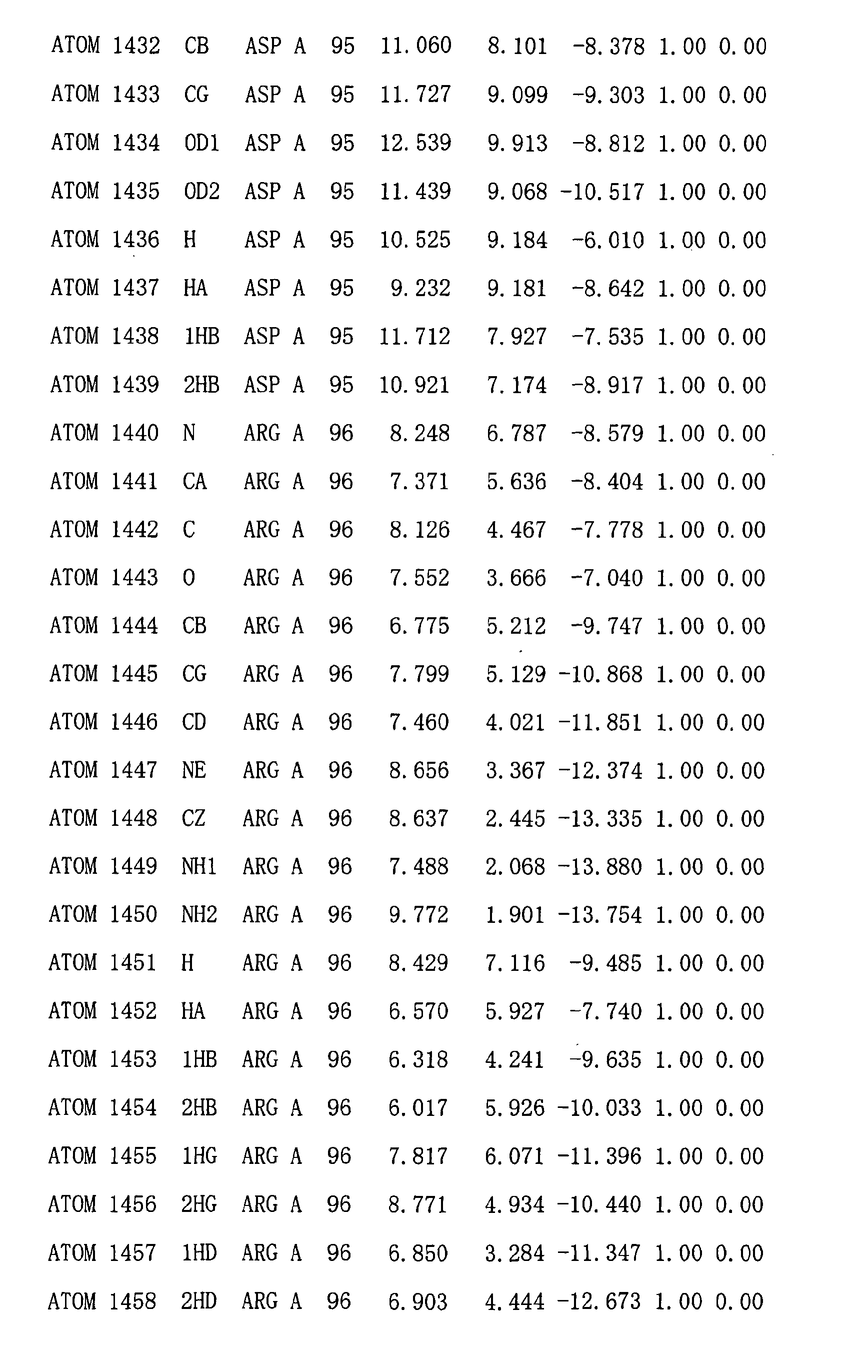

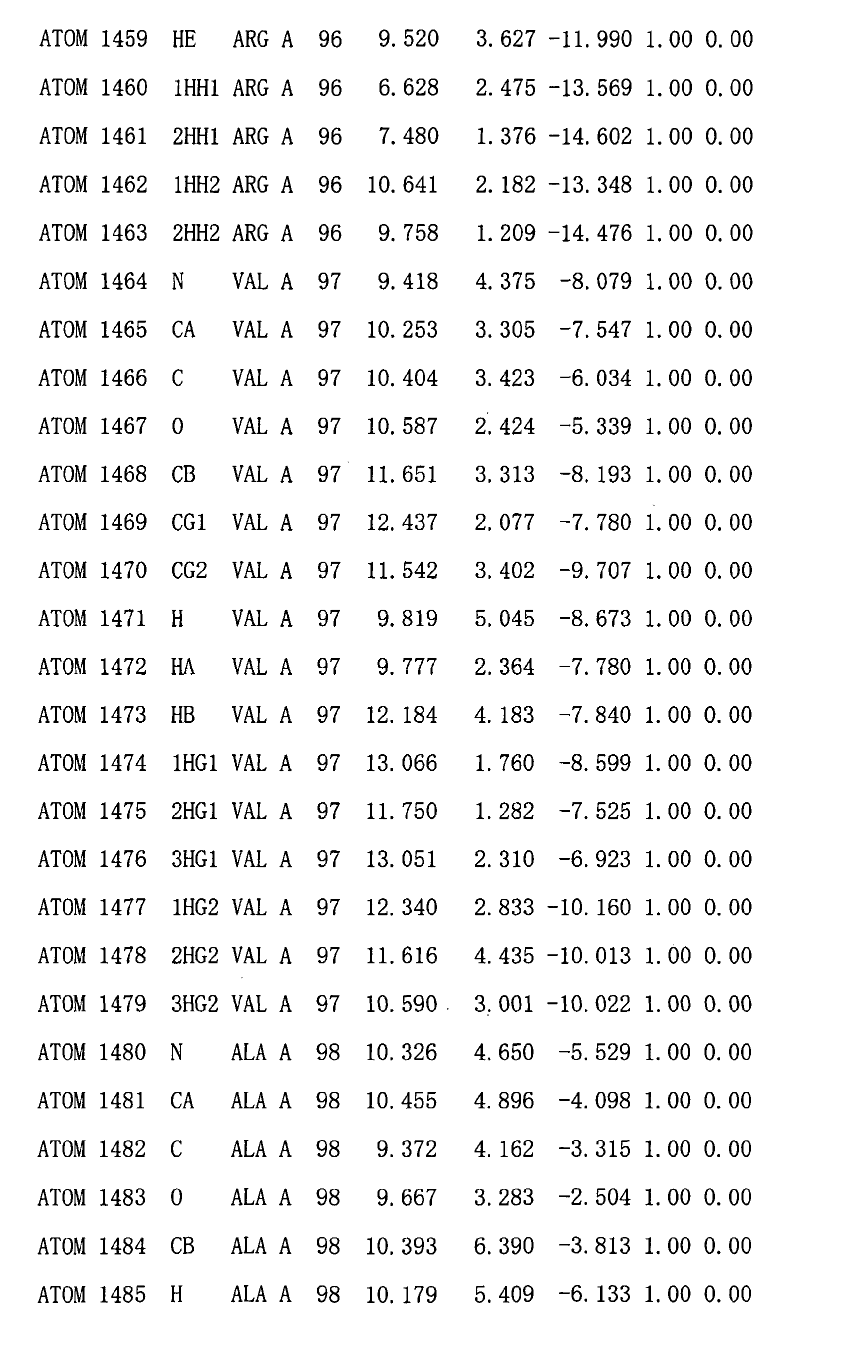

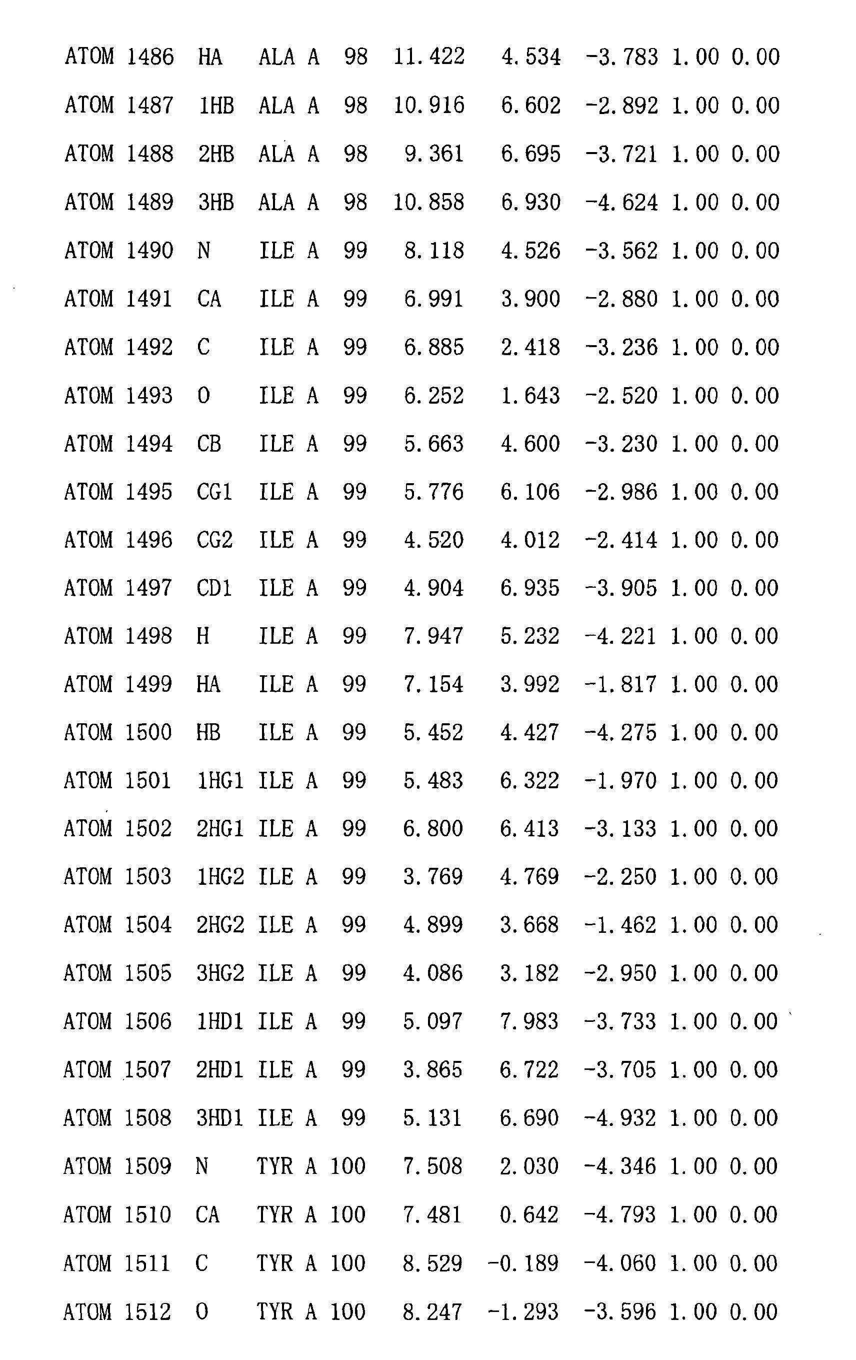

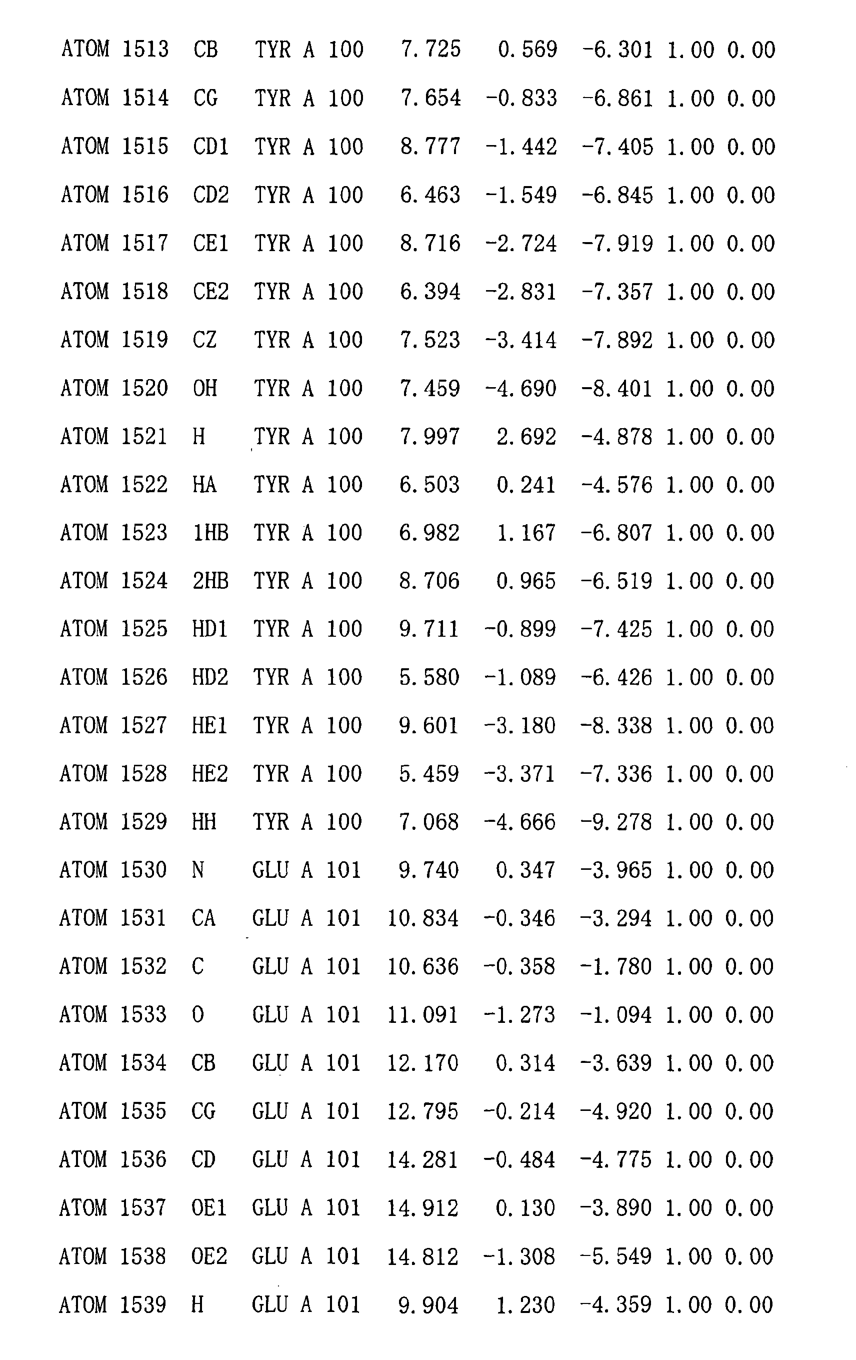

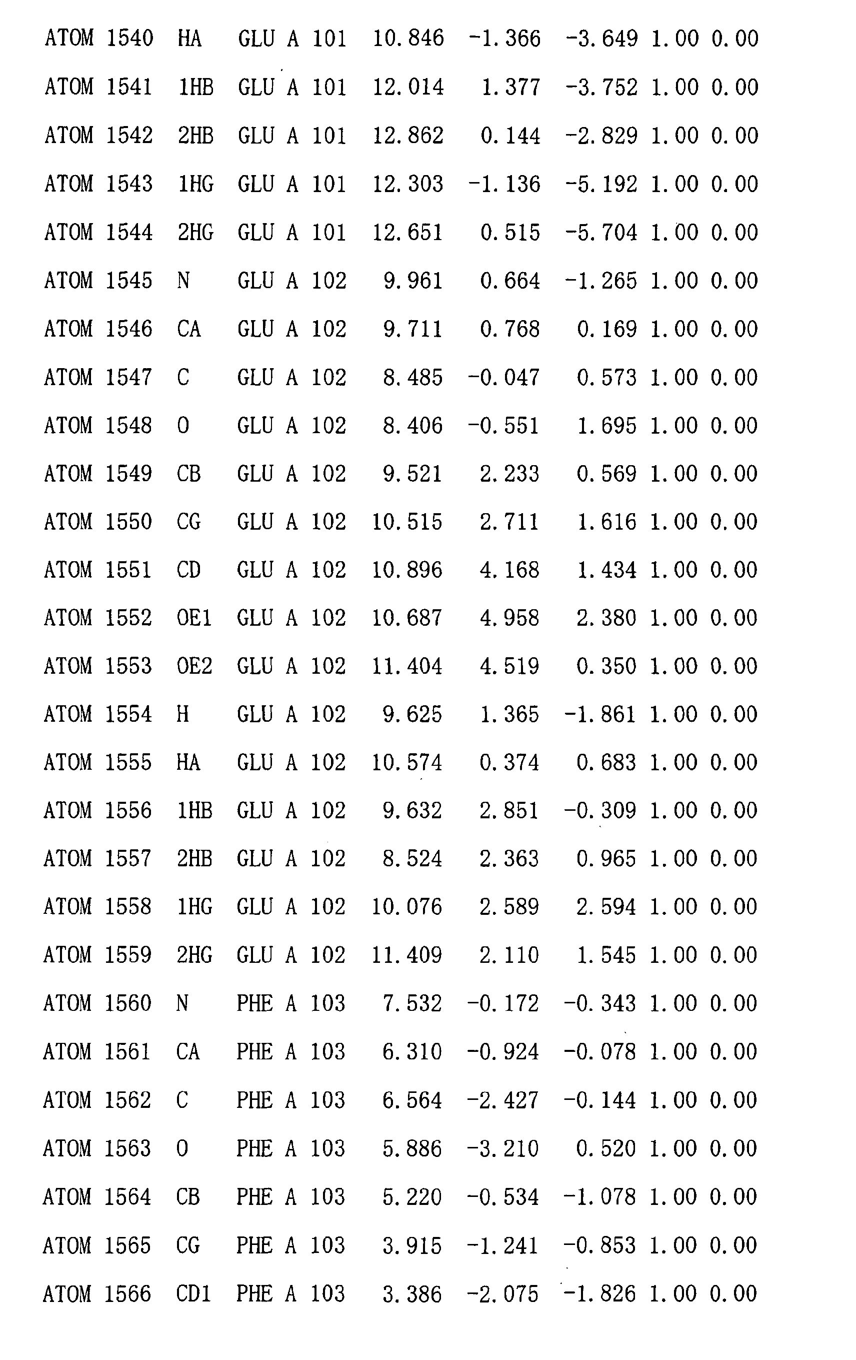

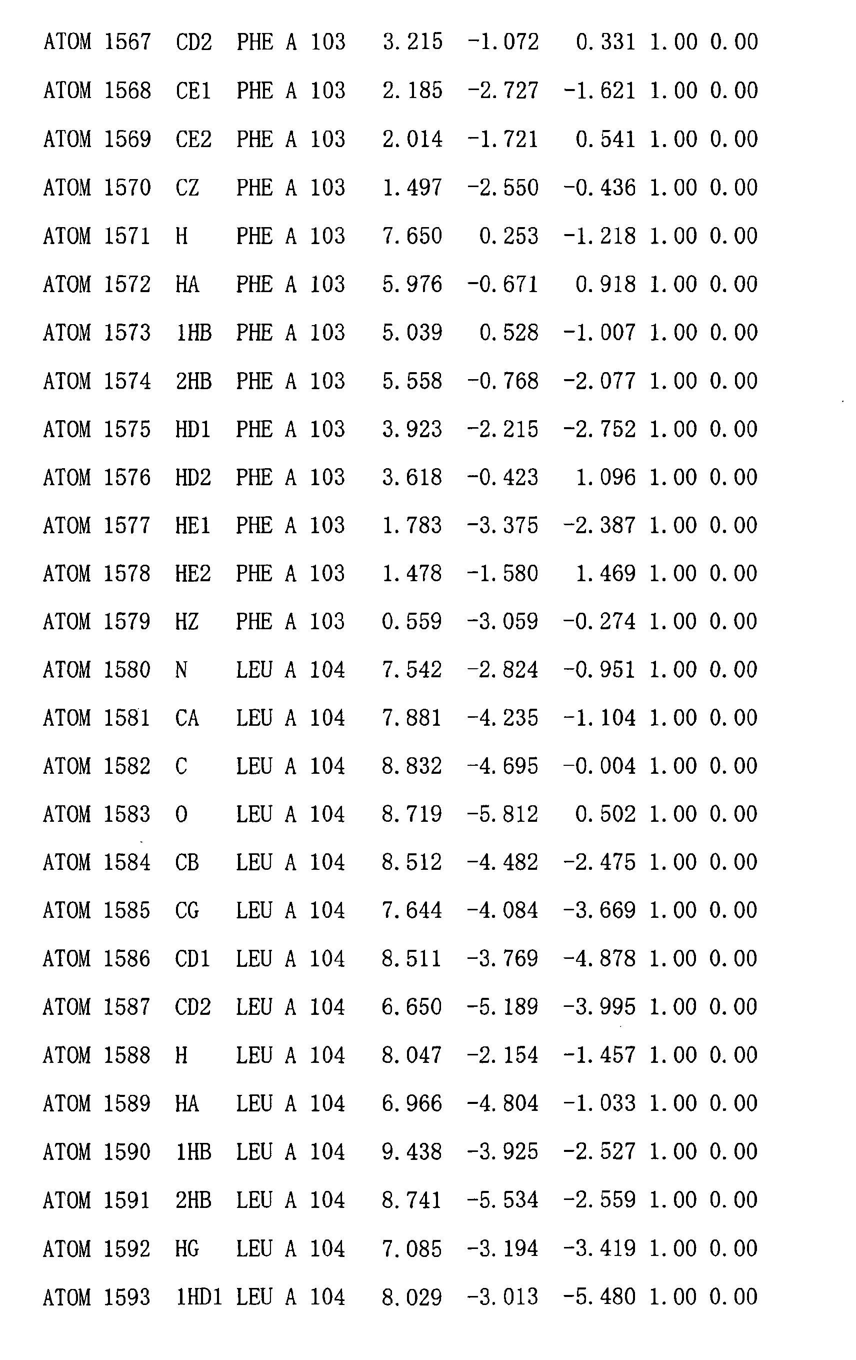

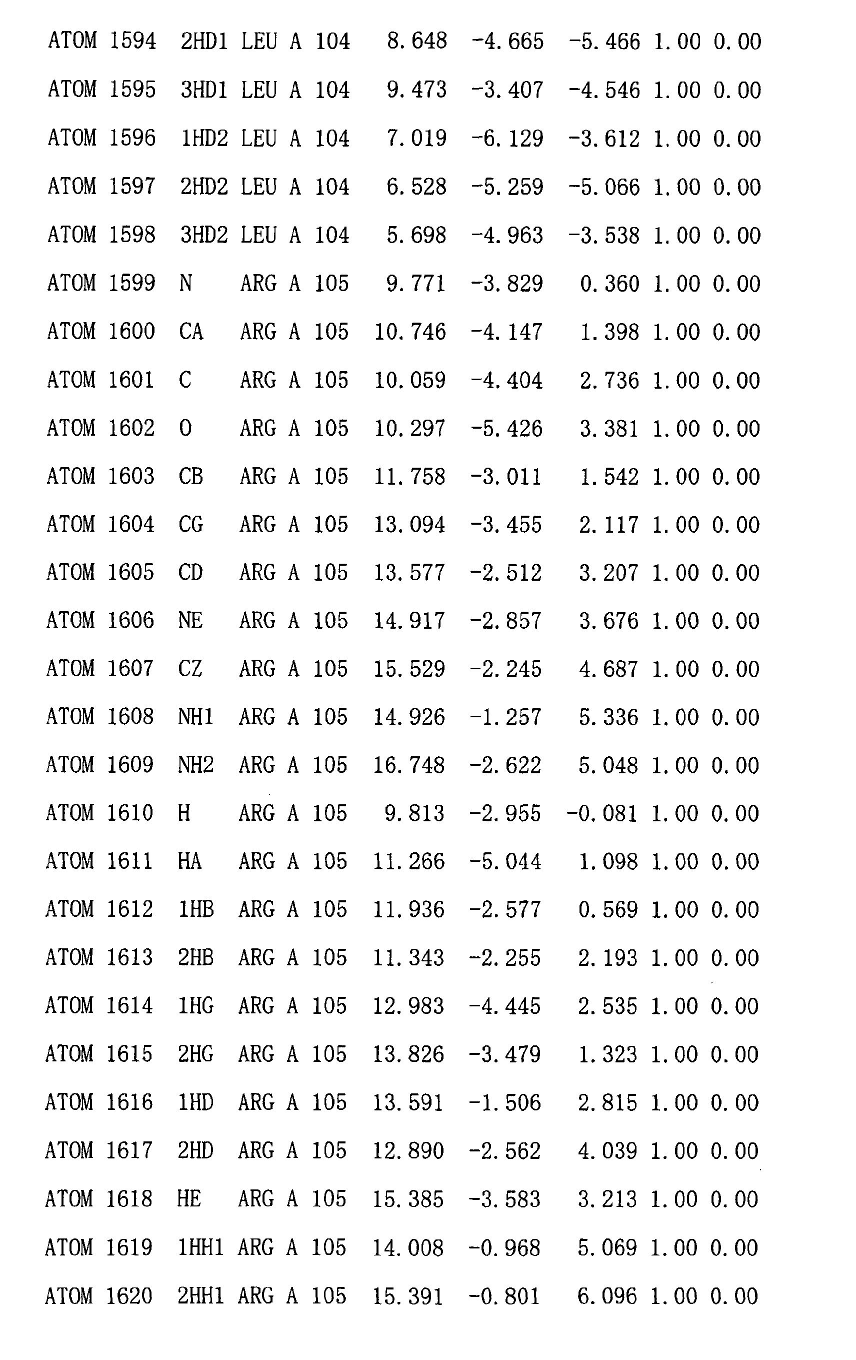

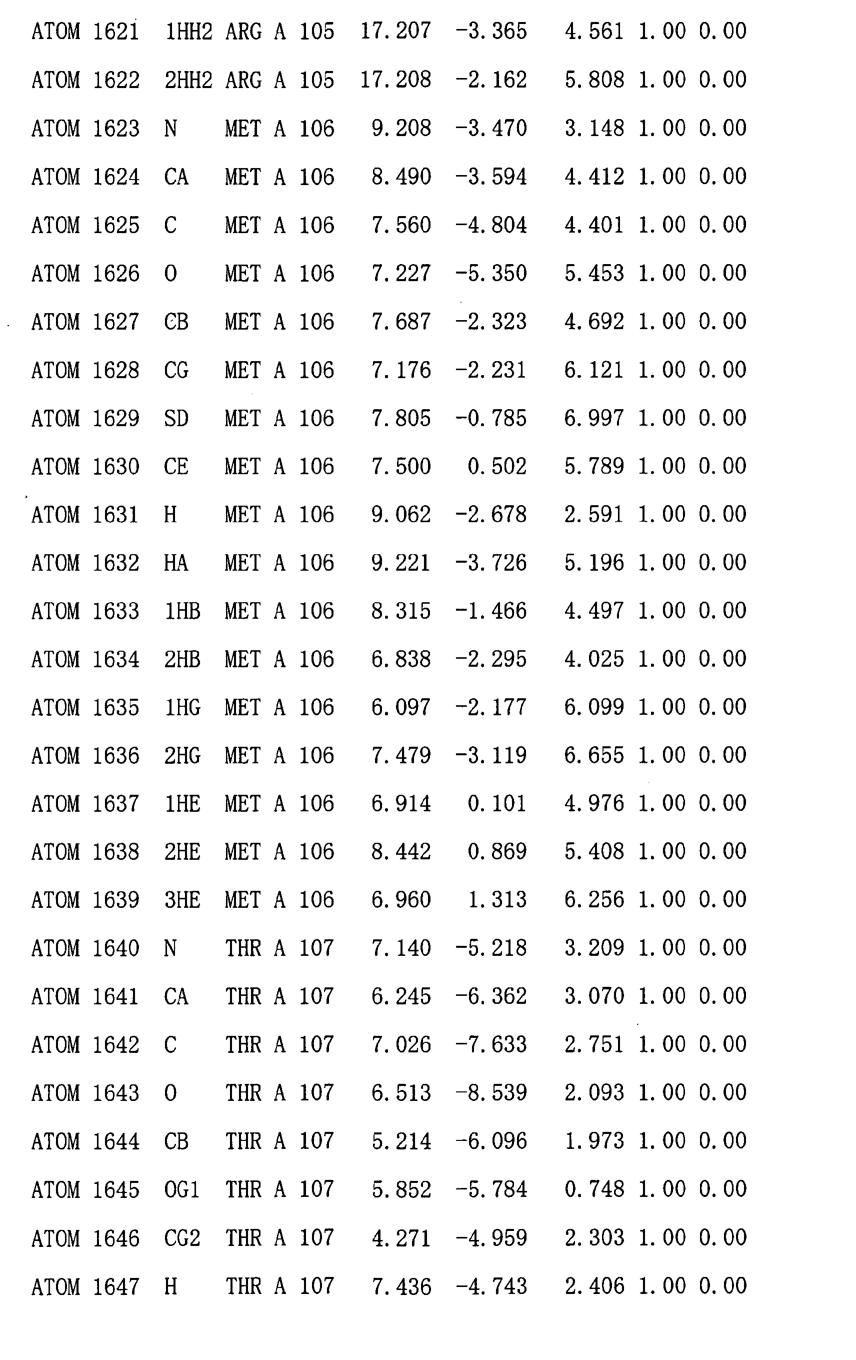

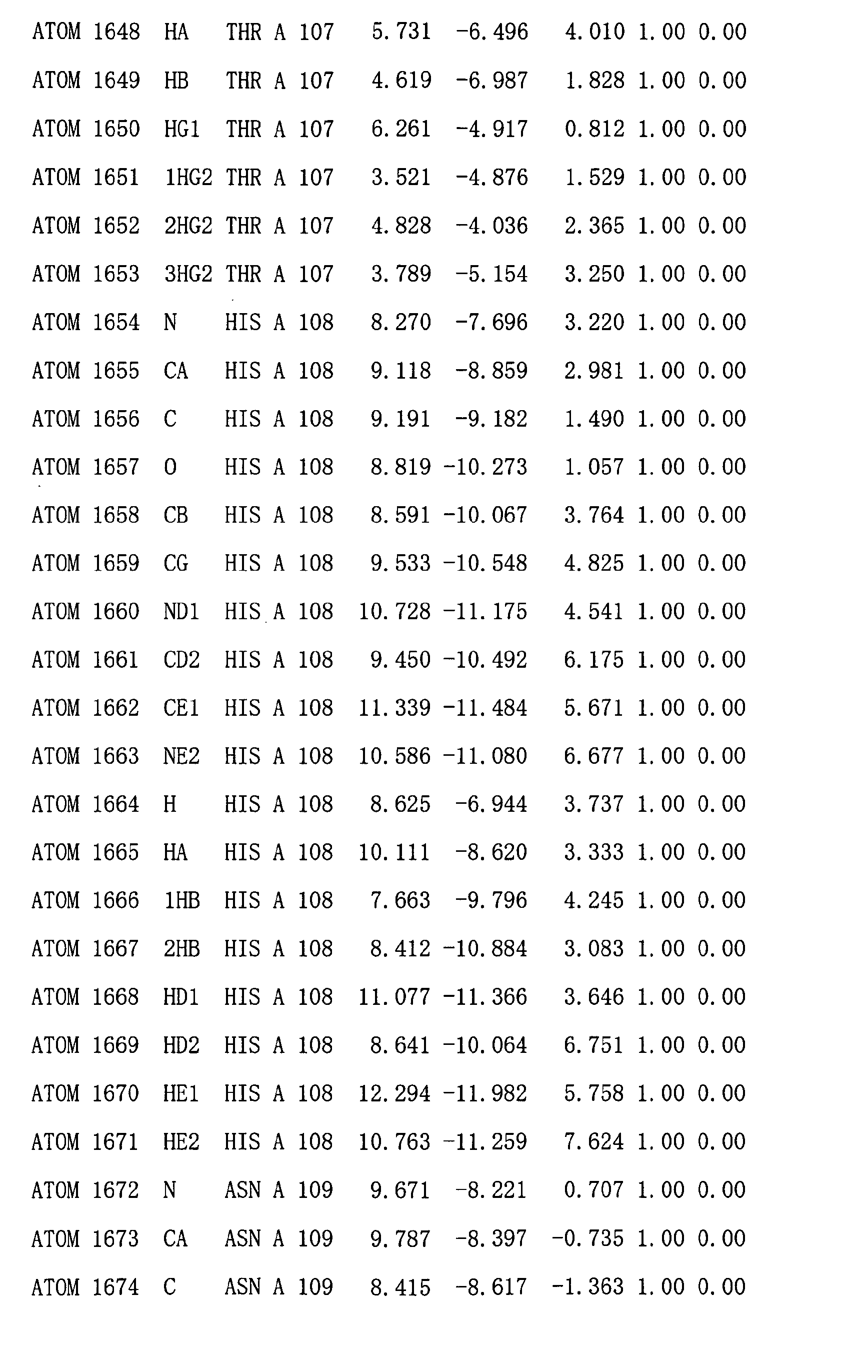

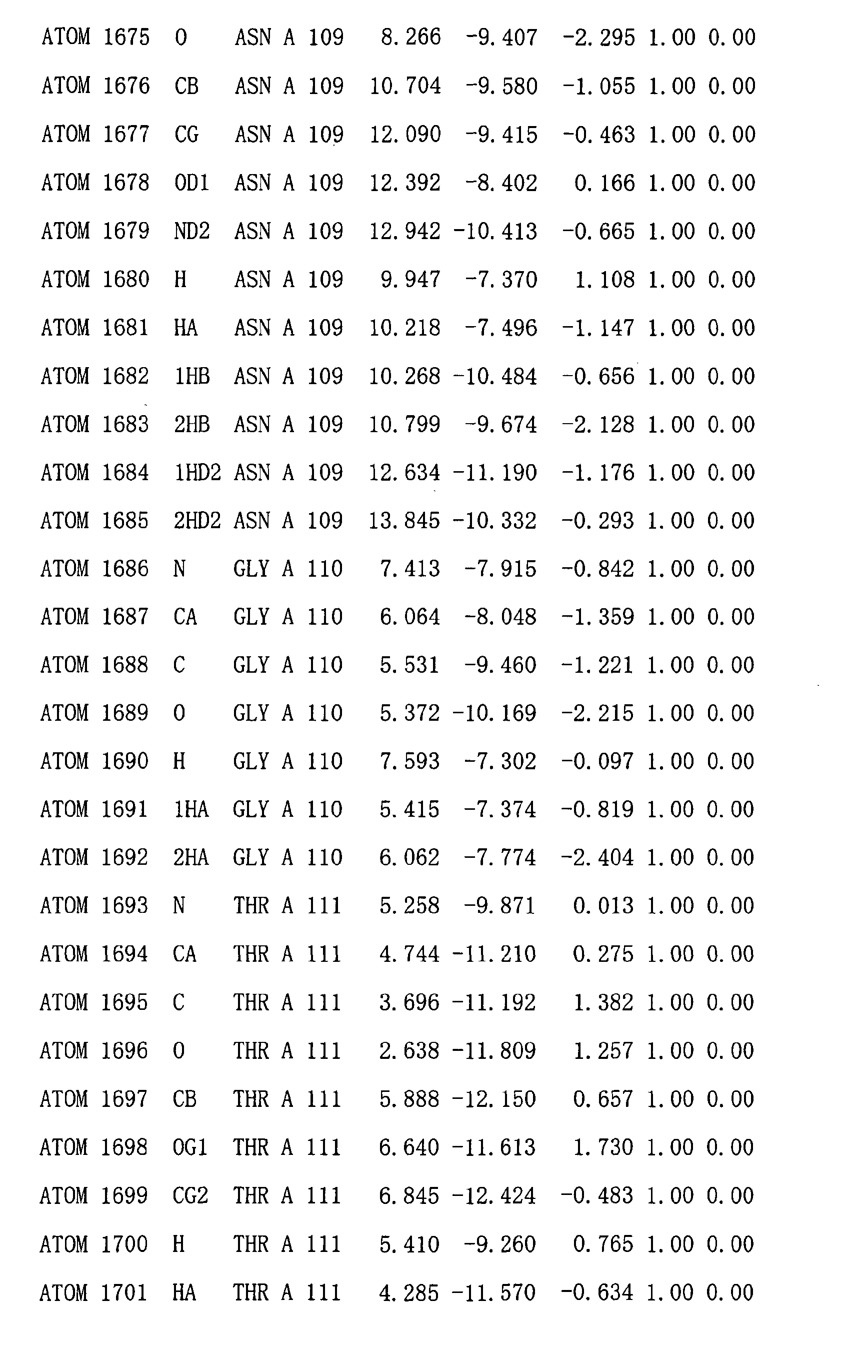

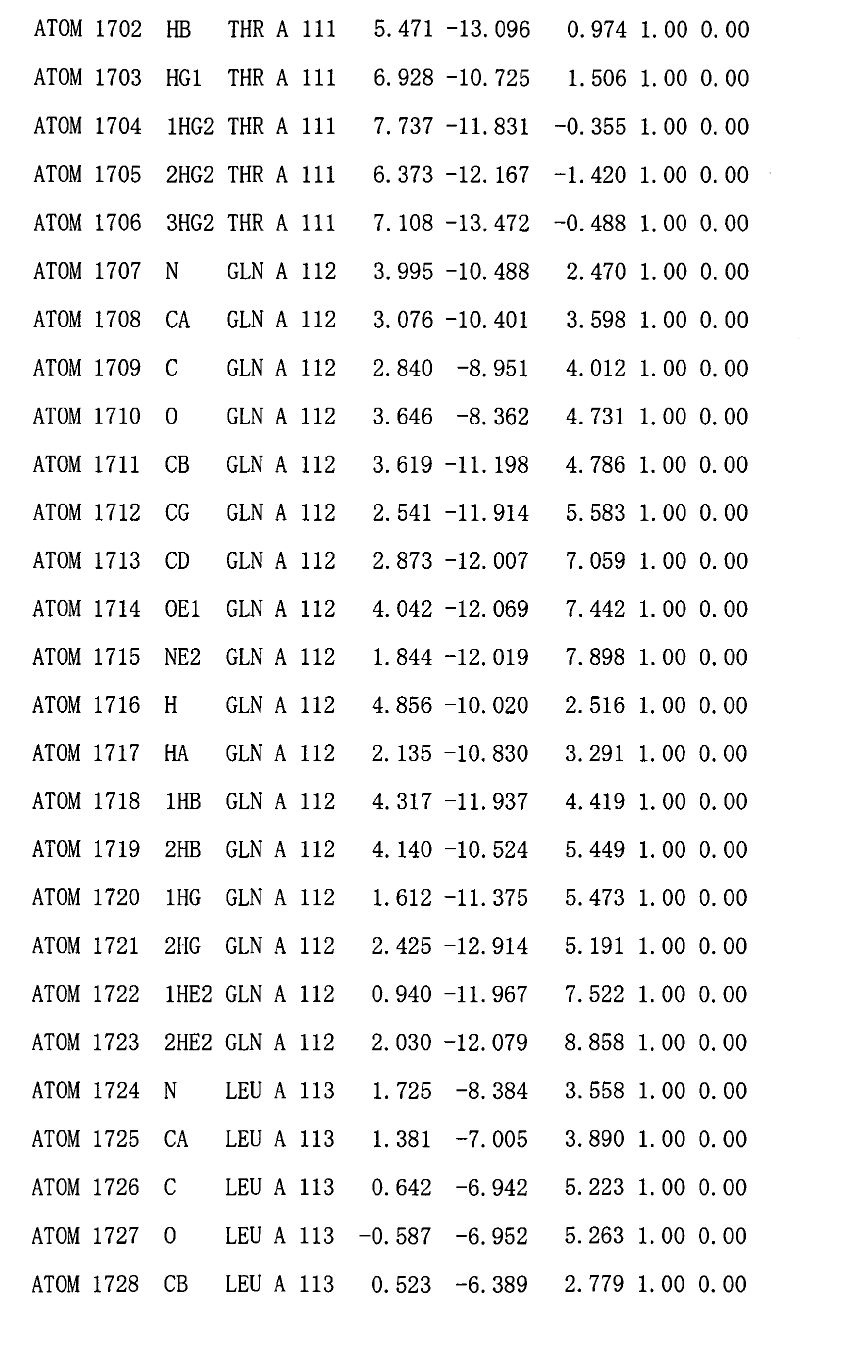

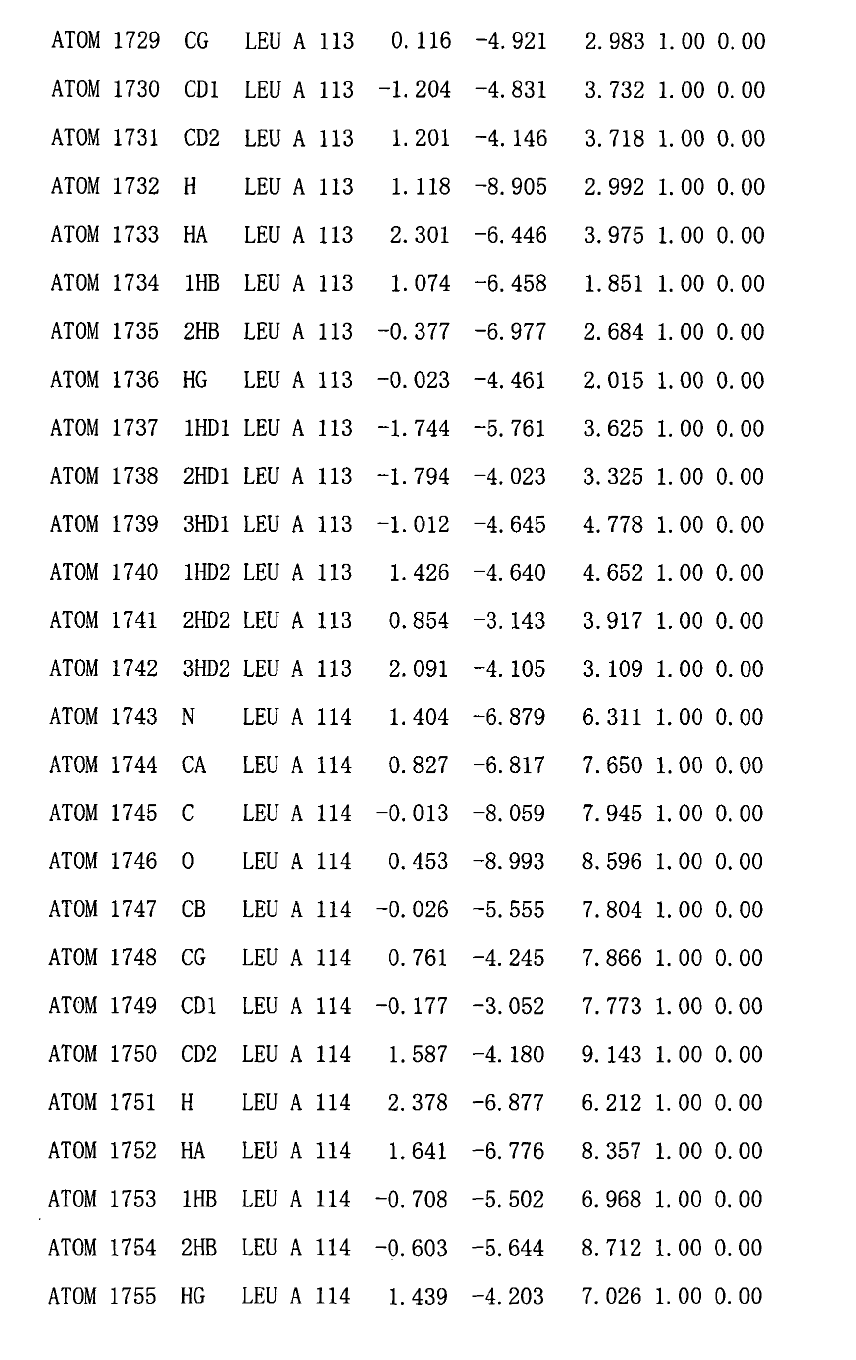

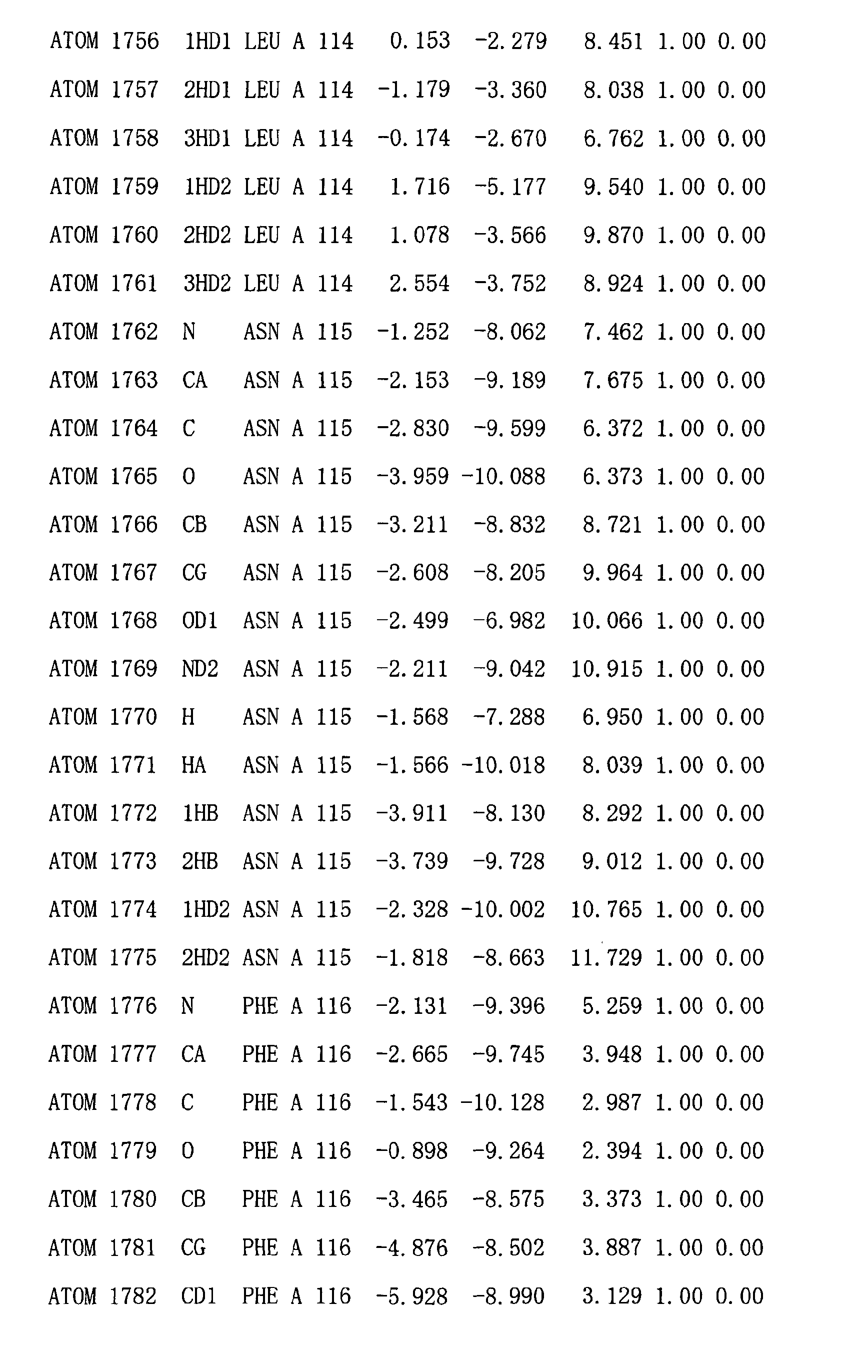

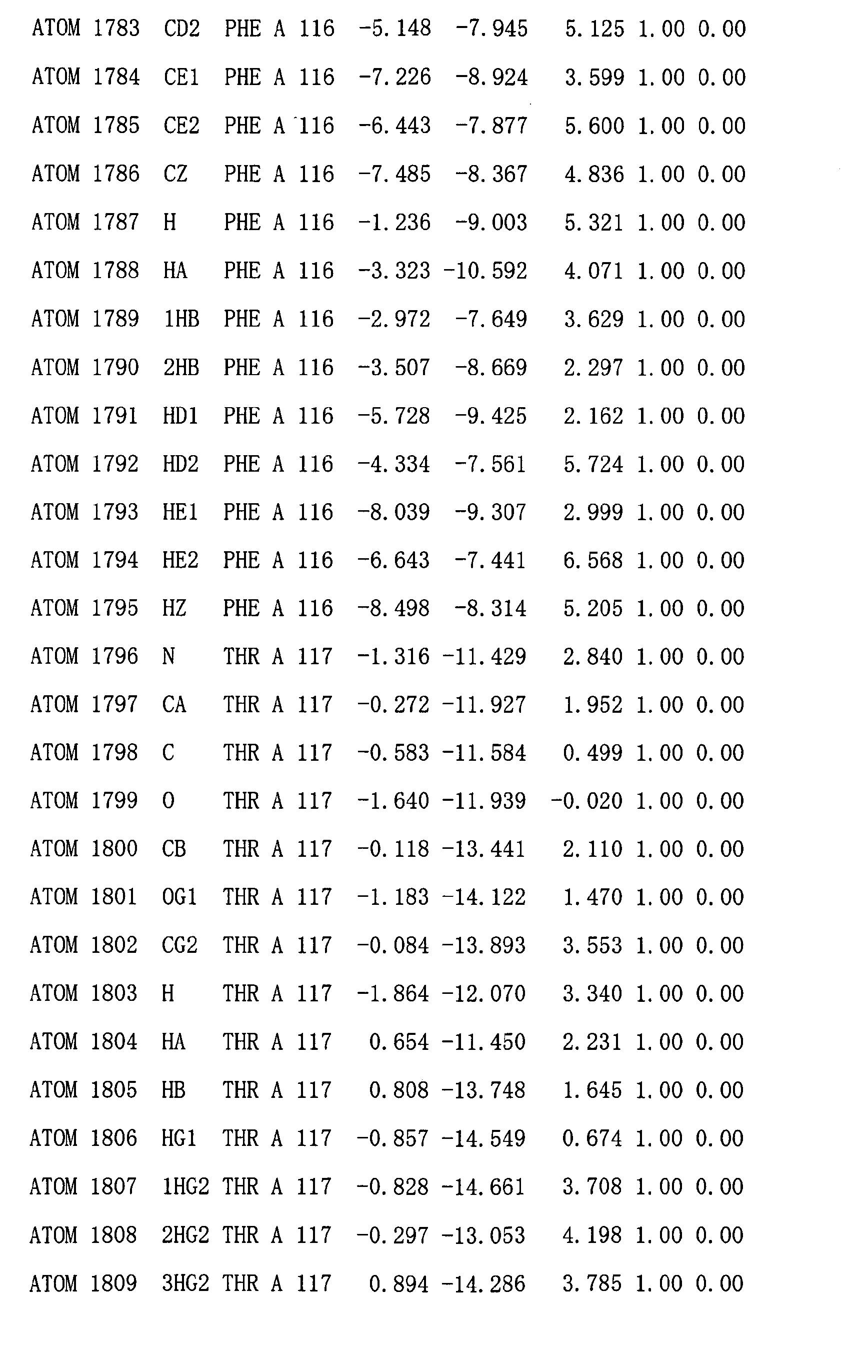

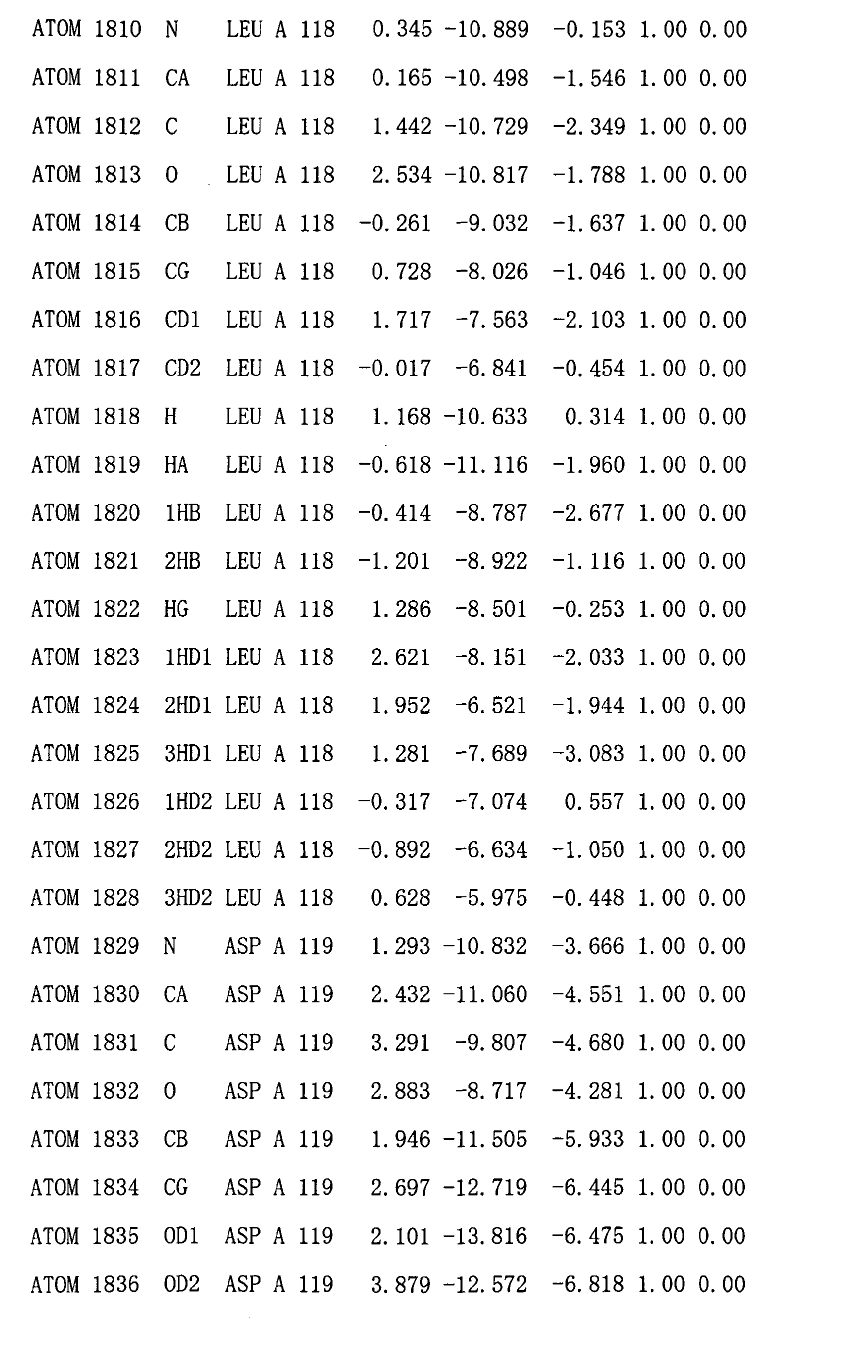

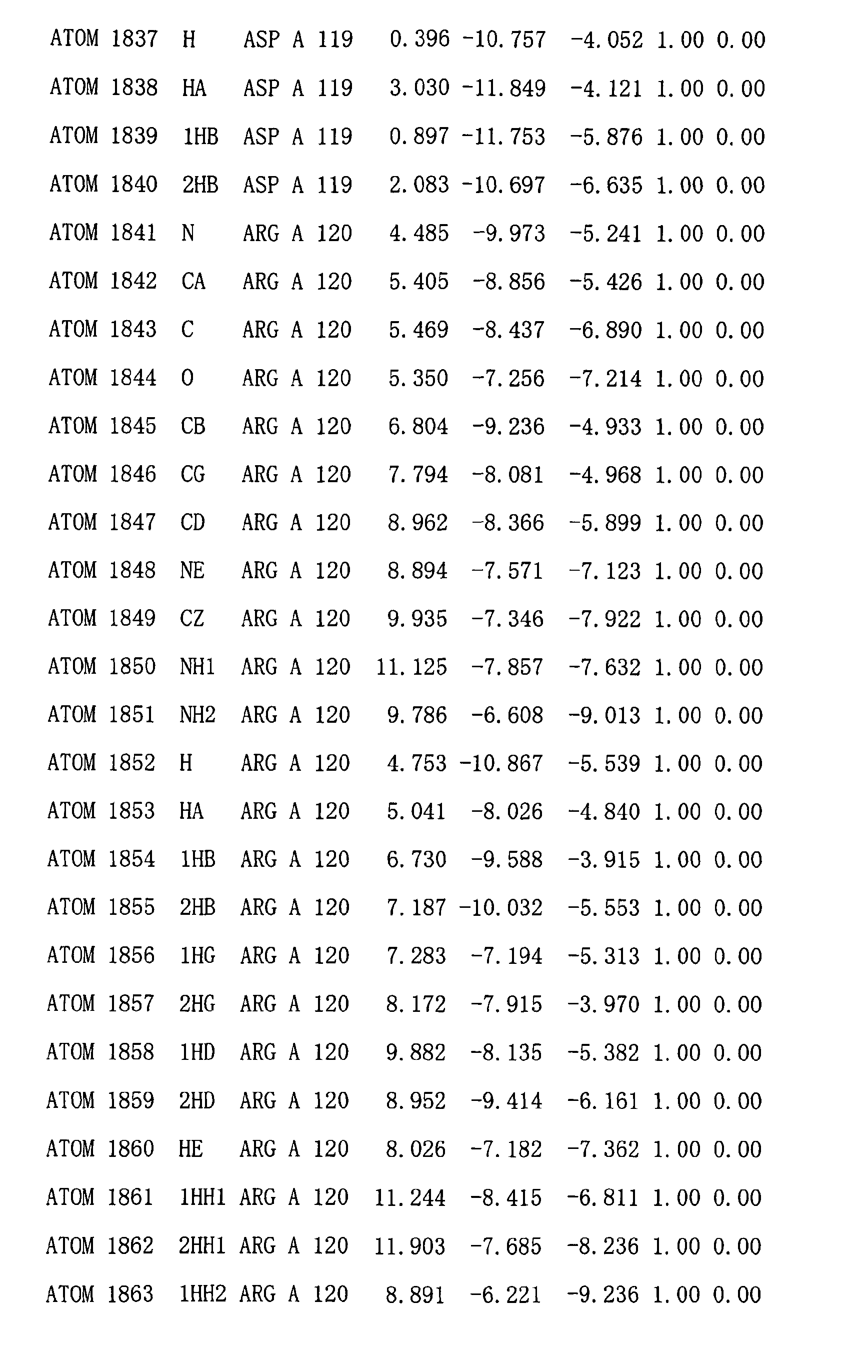

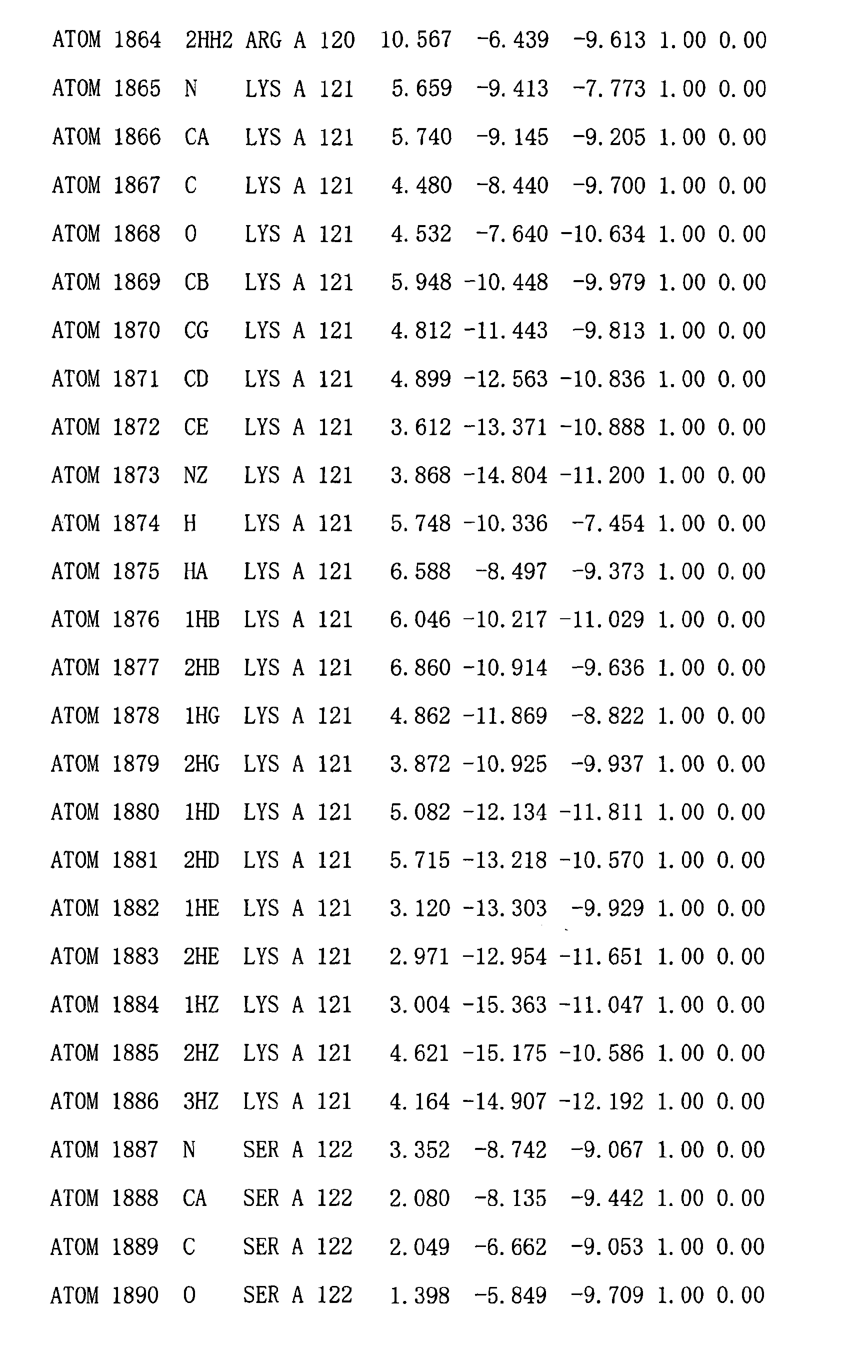

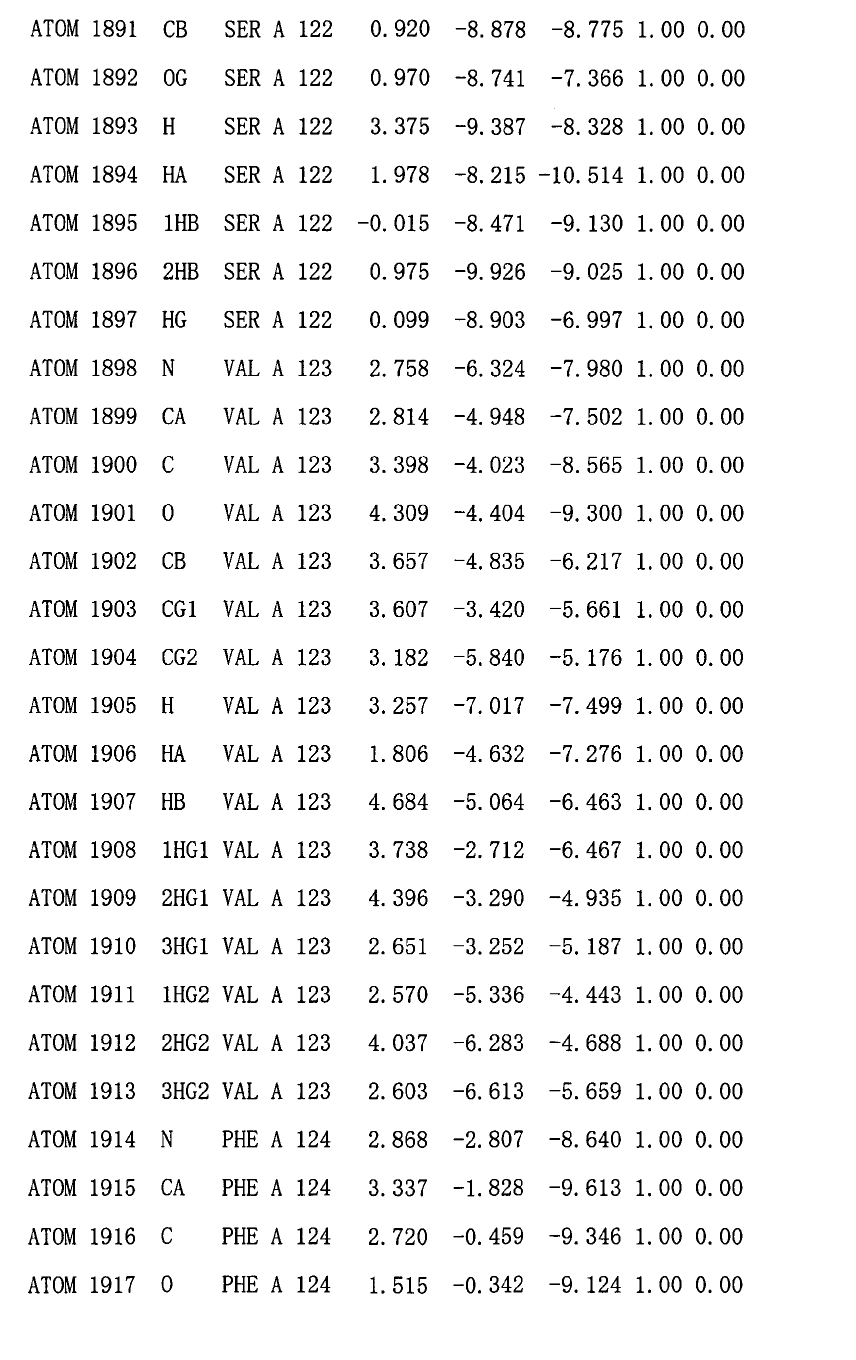

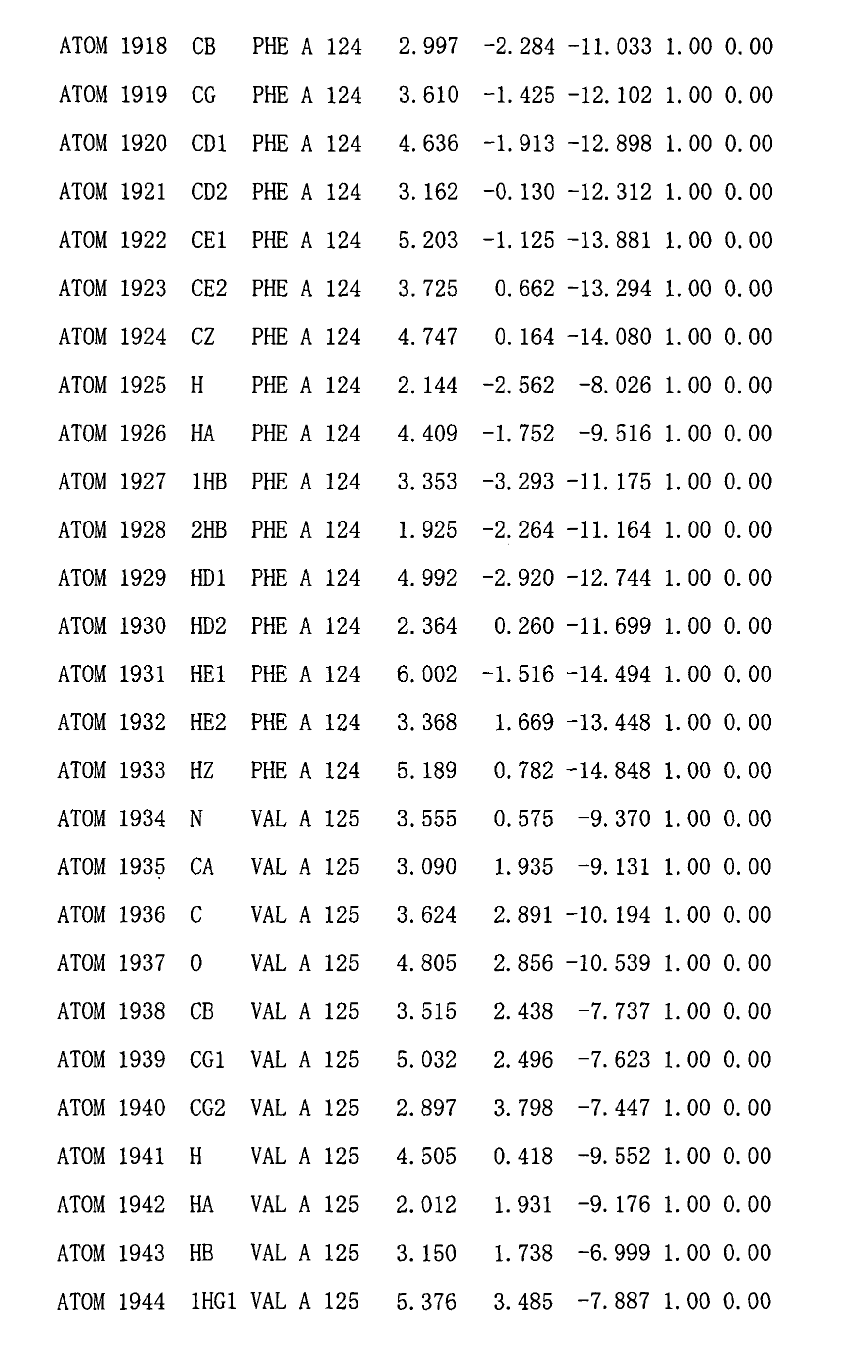

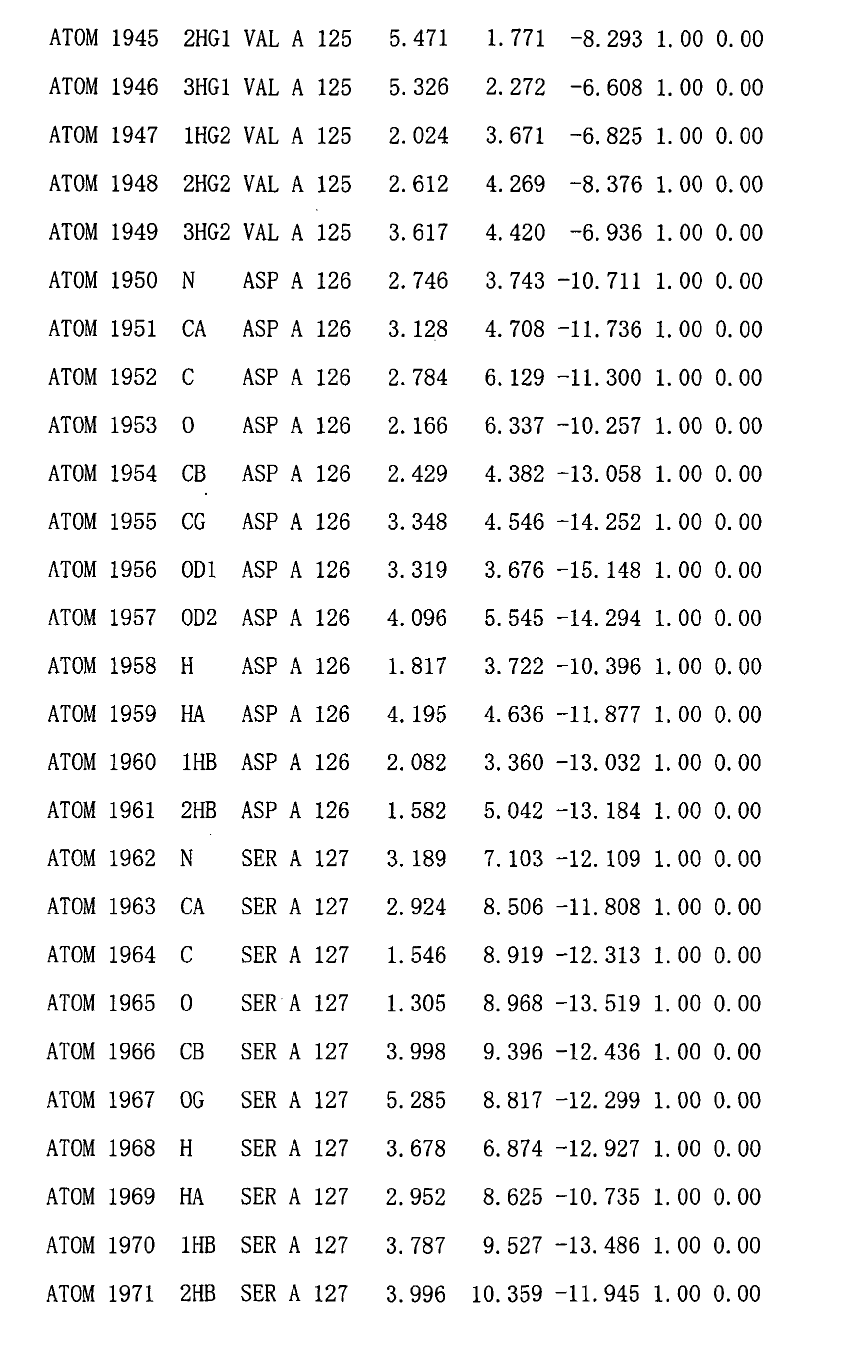

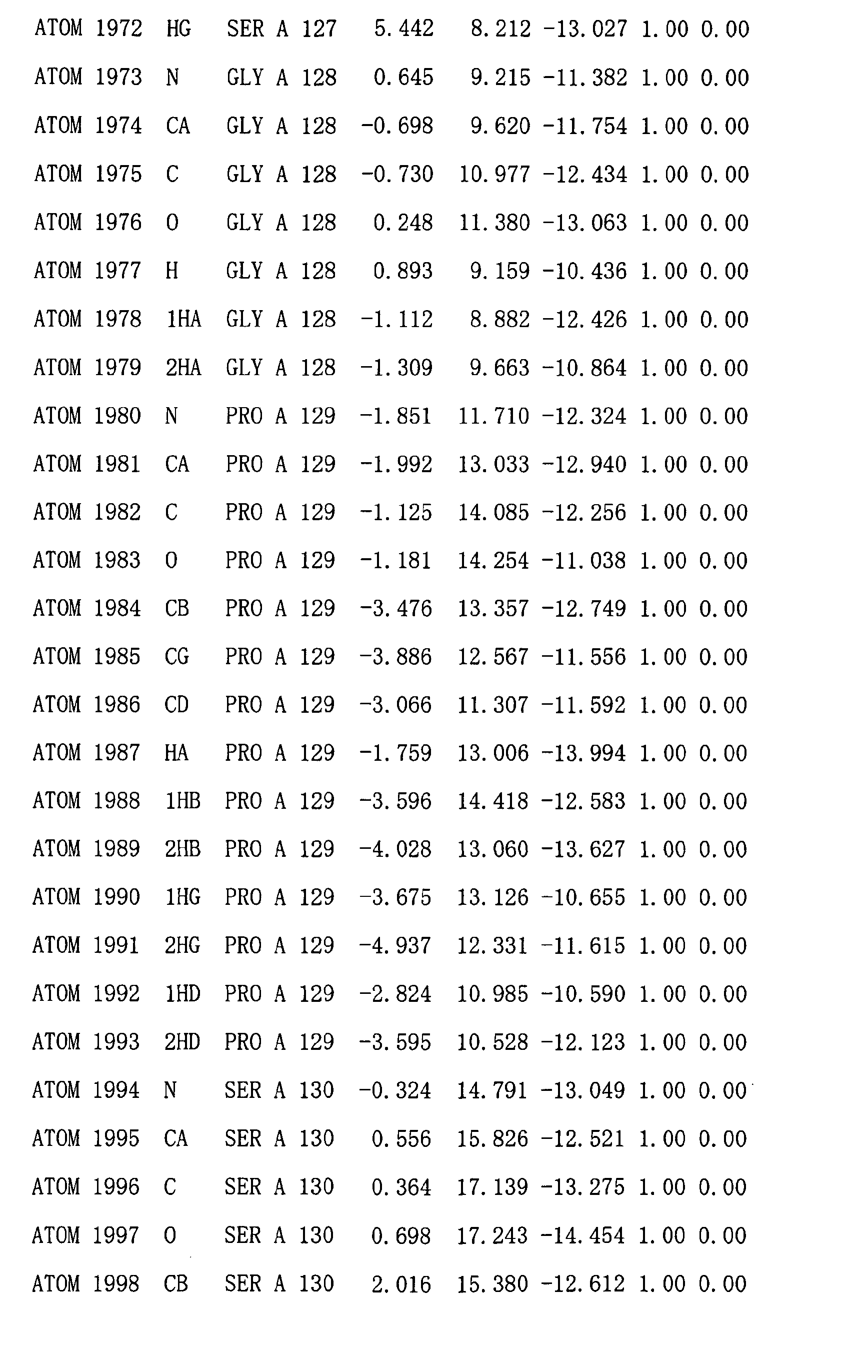

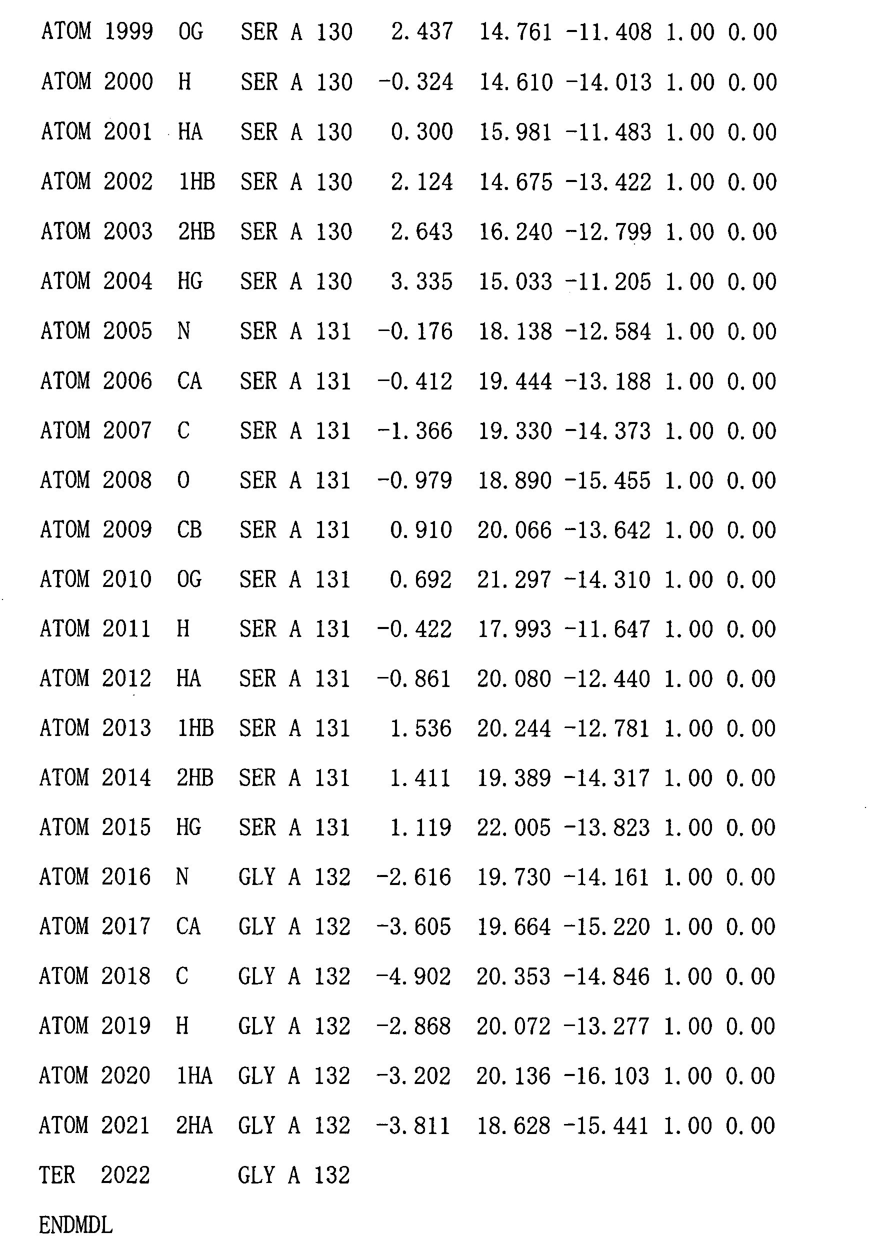

### Three-Dimensional Structure Coordinate 14

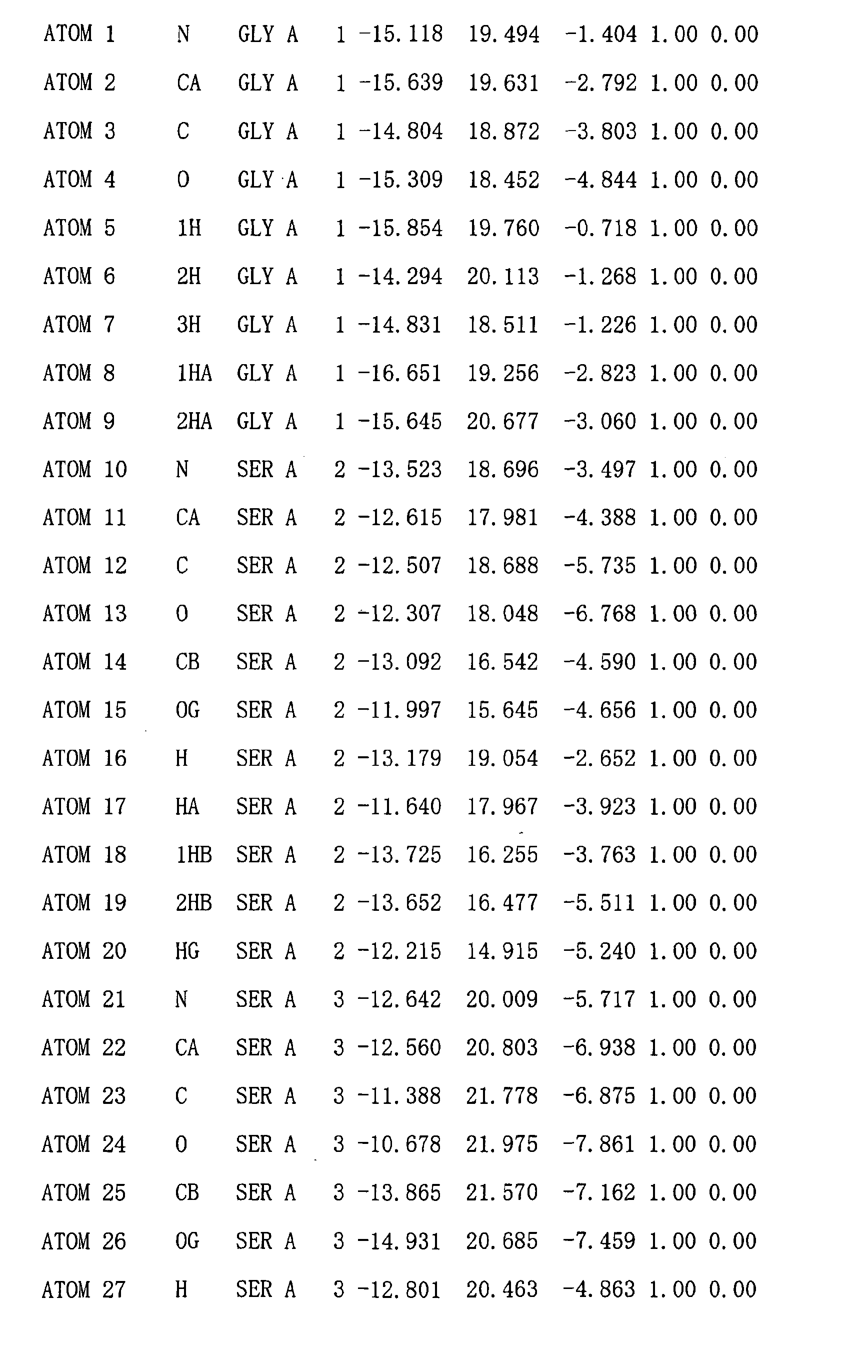

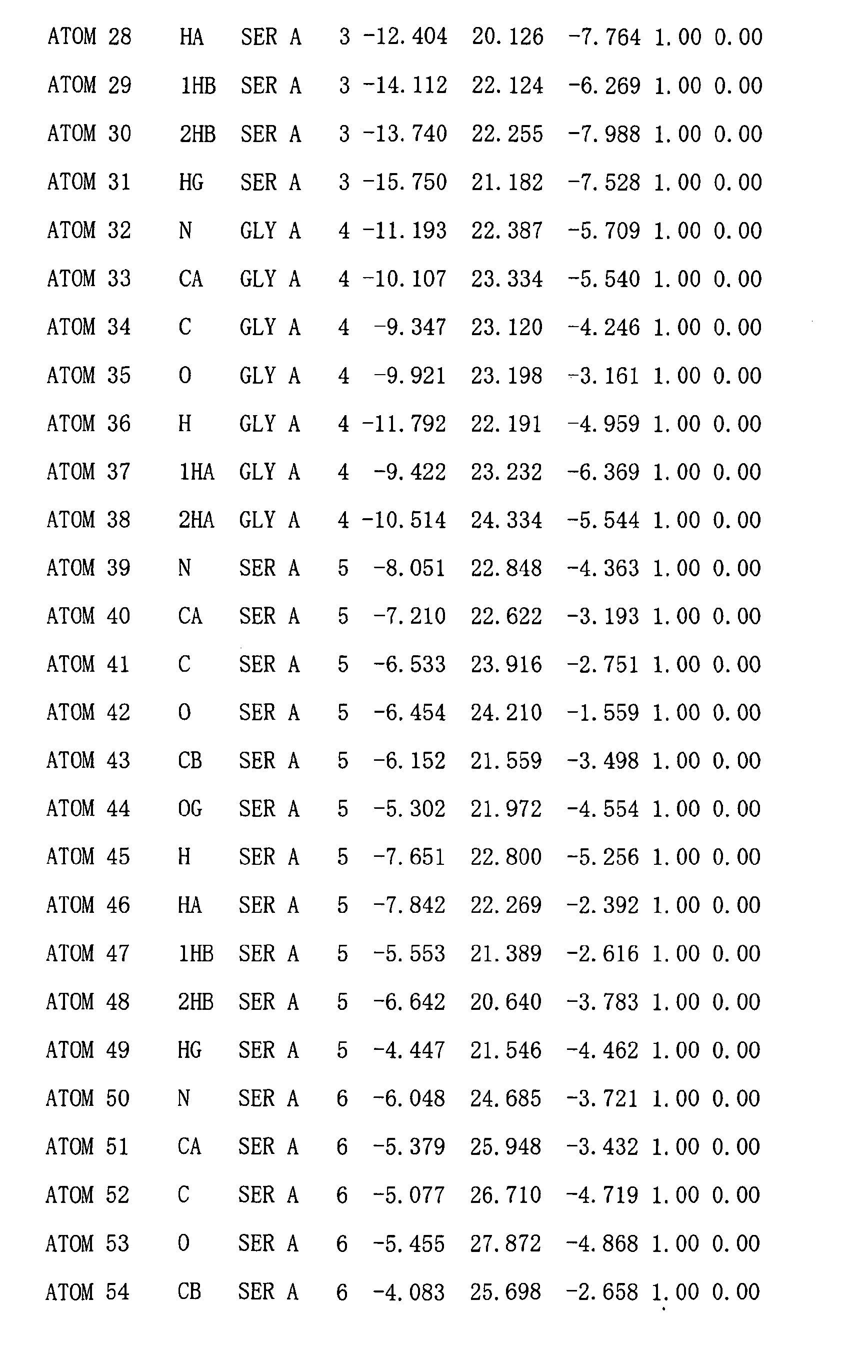

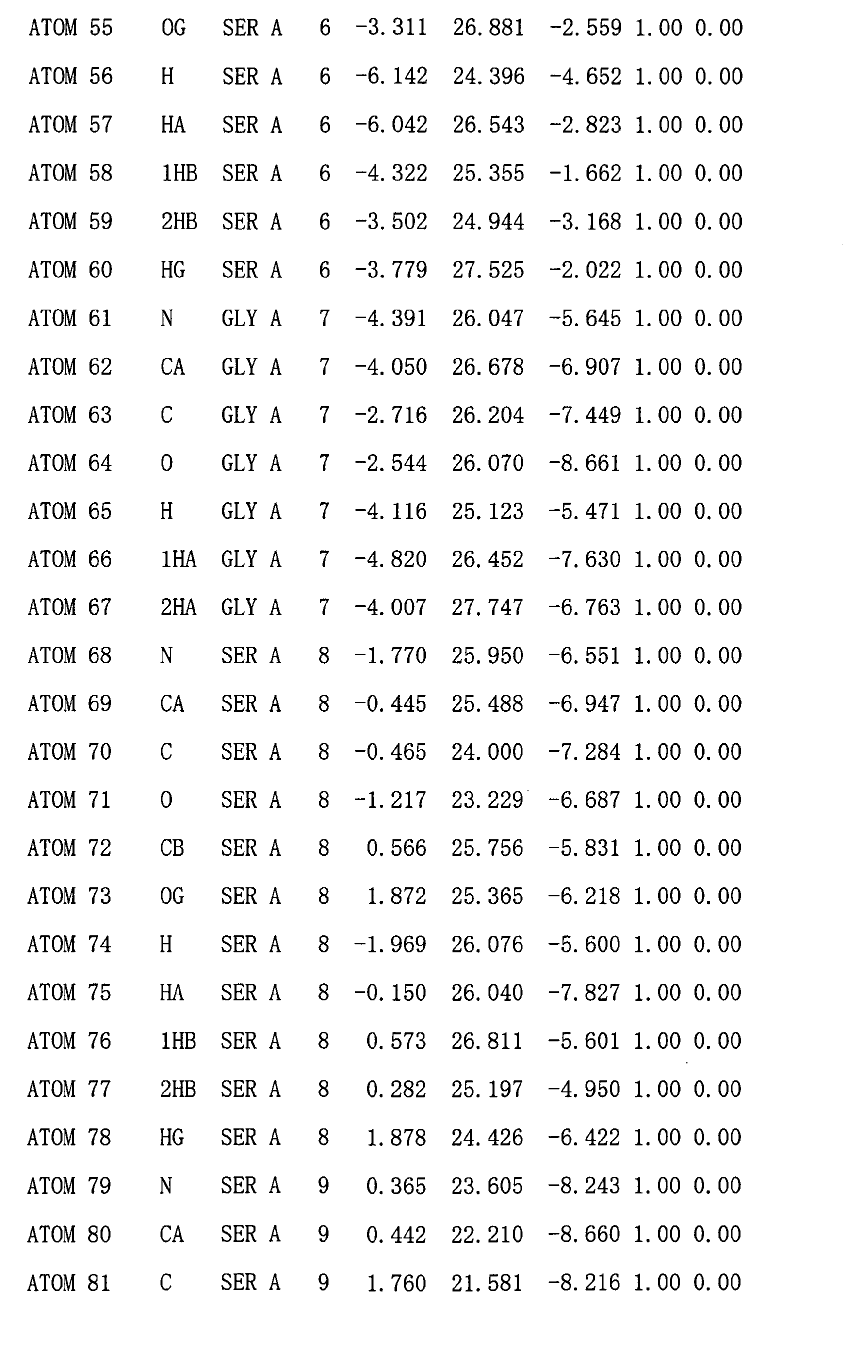

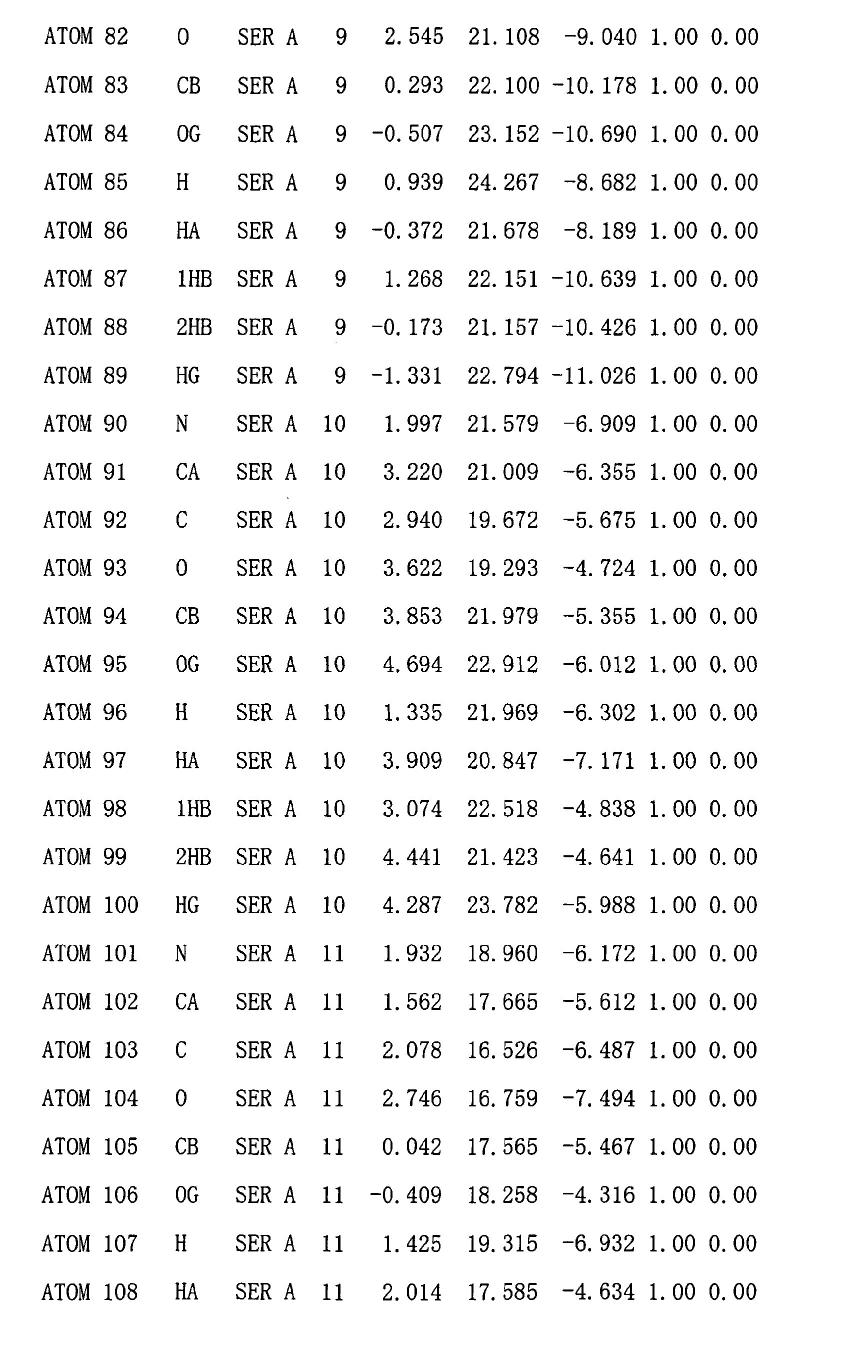

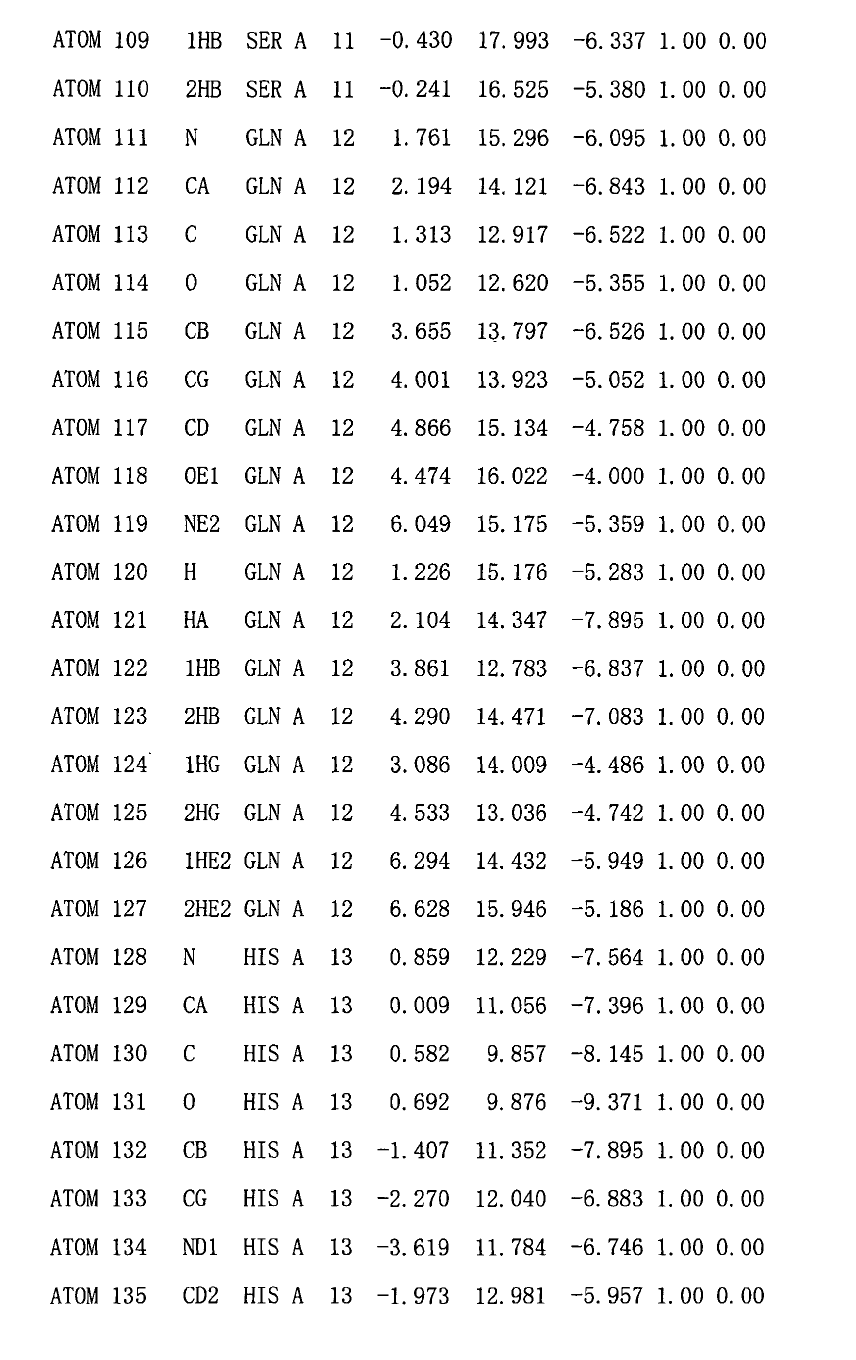

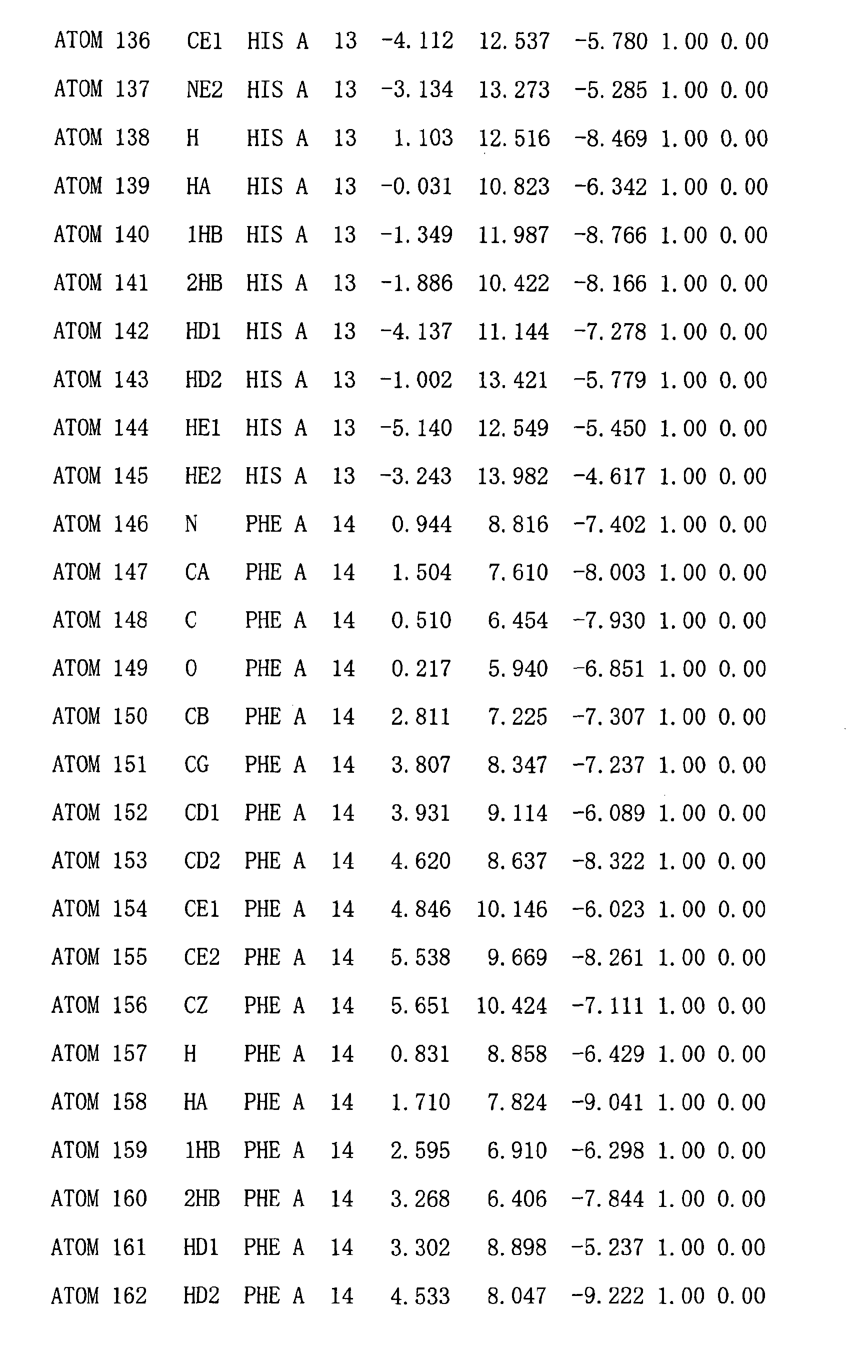

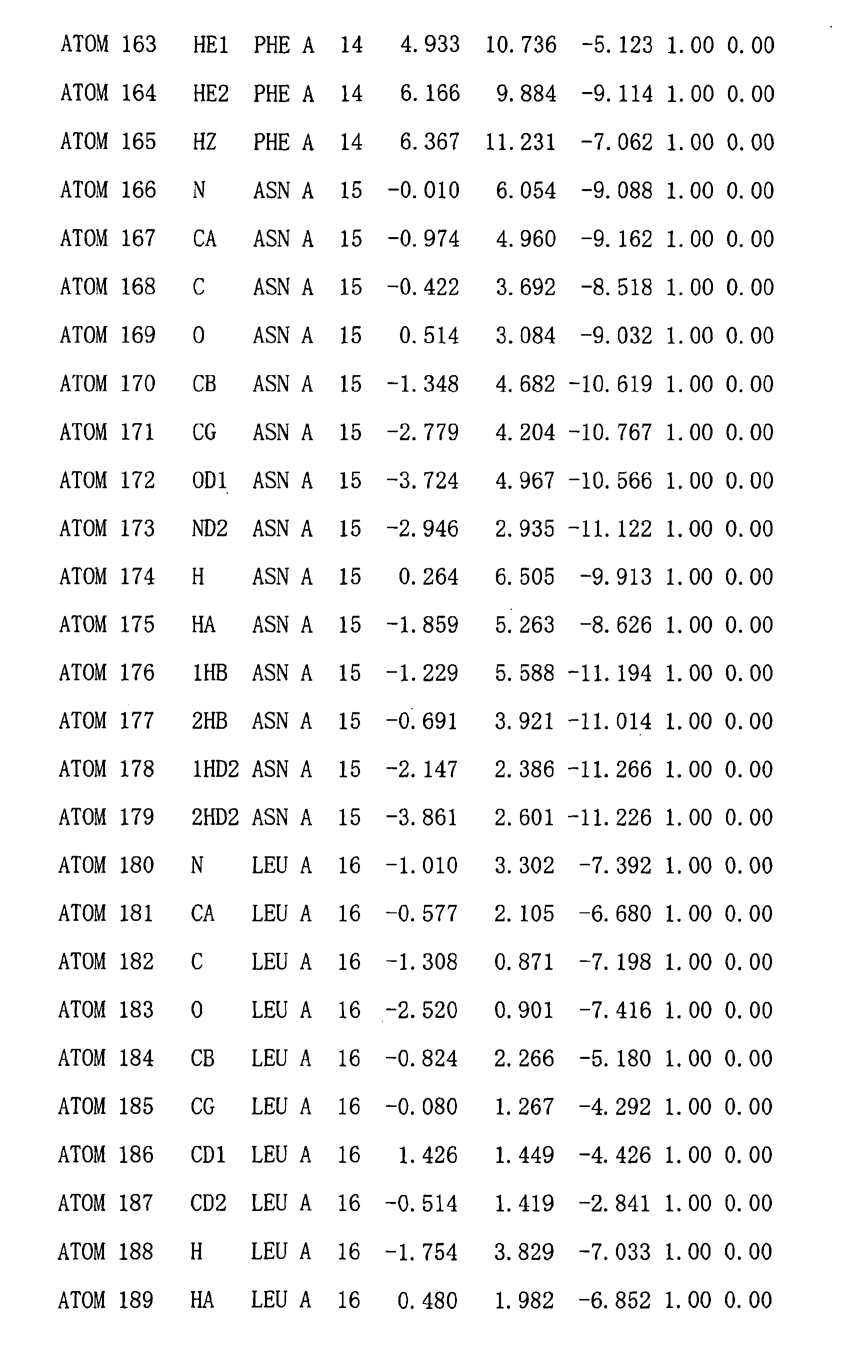

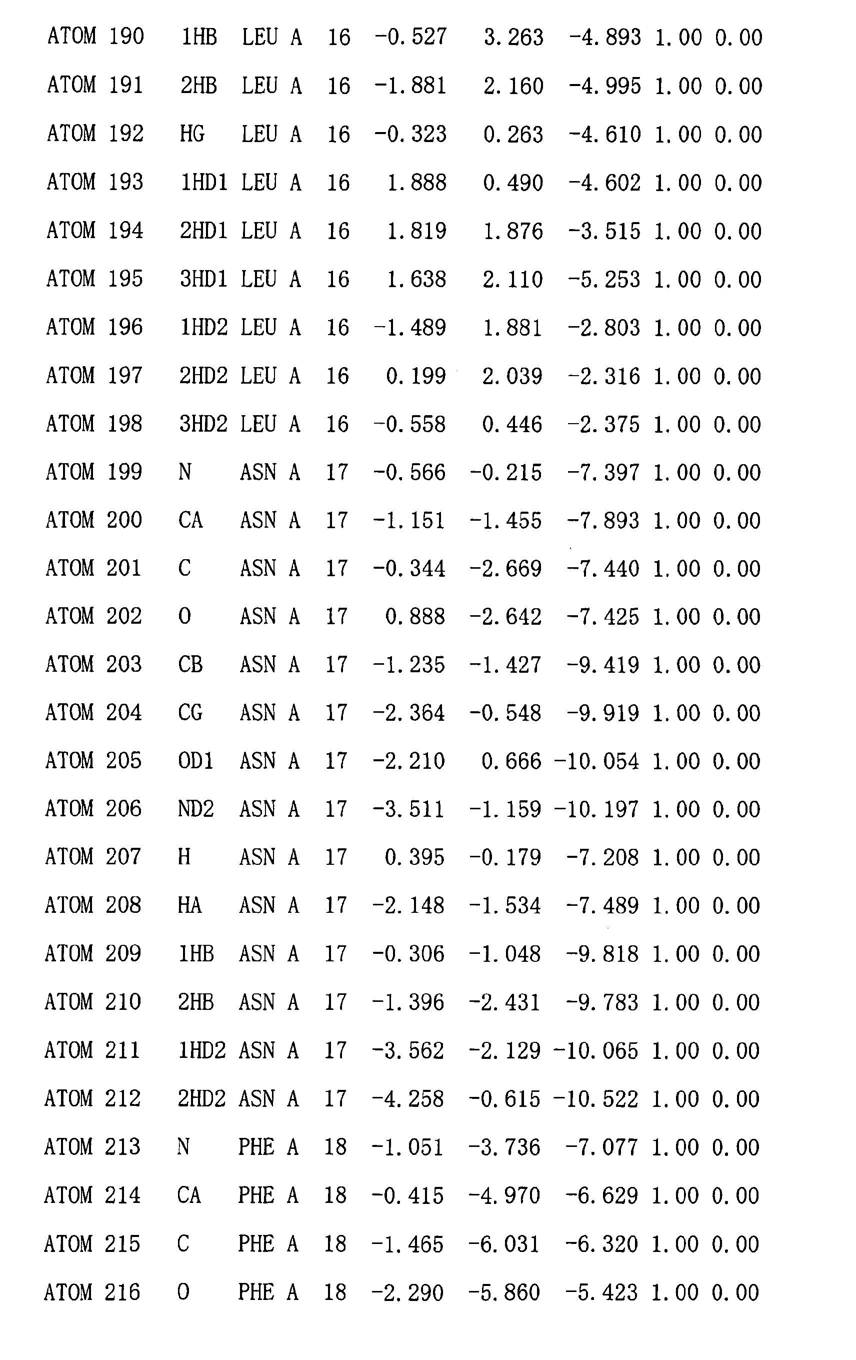

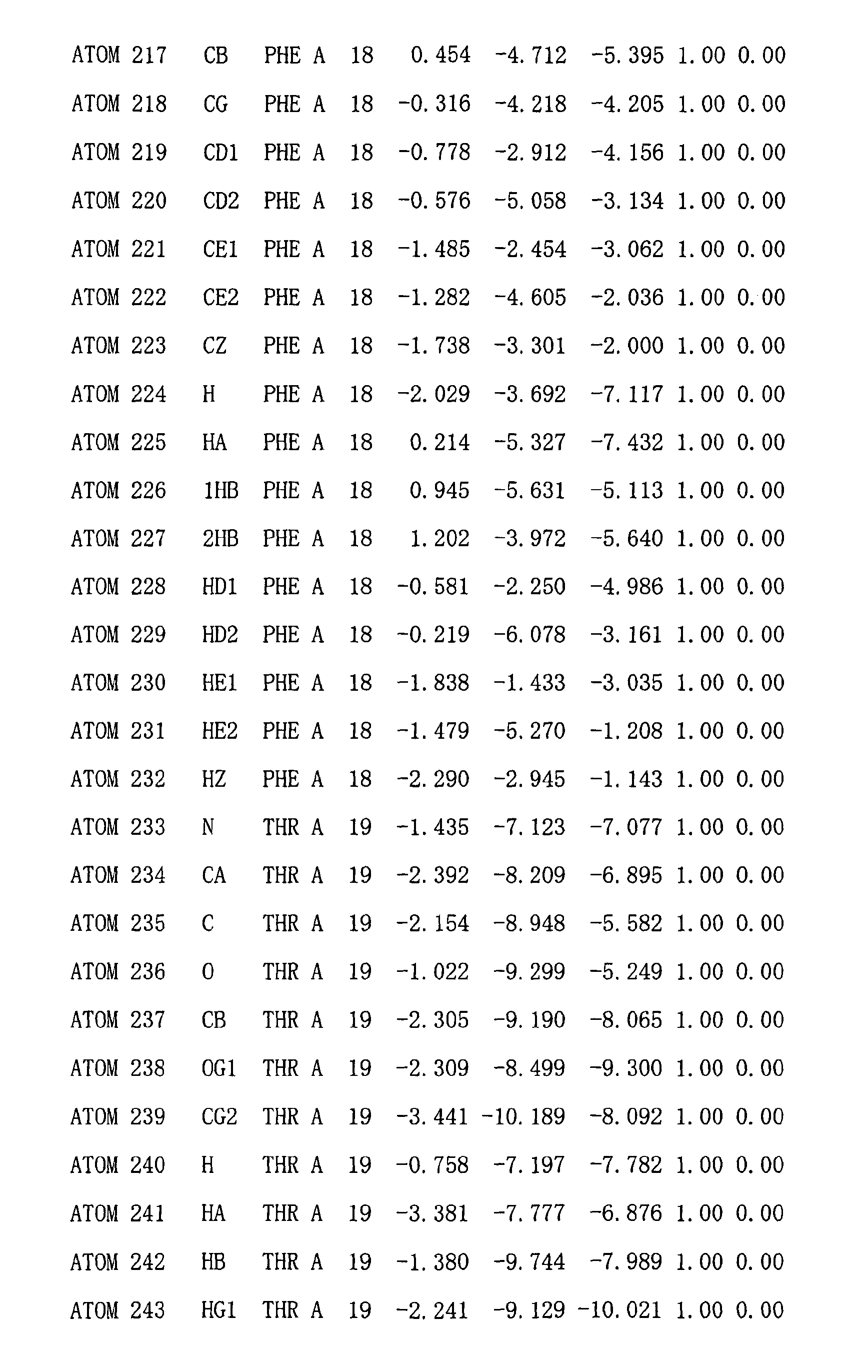

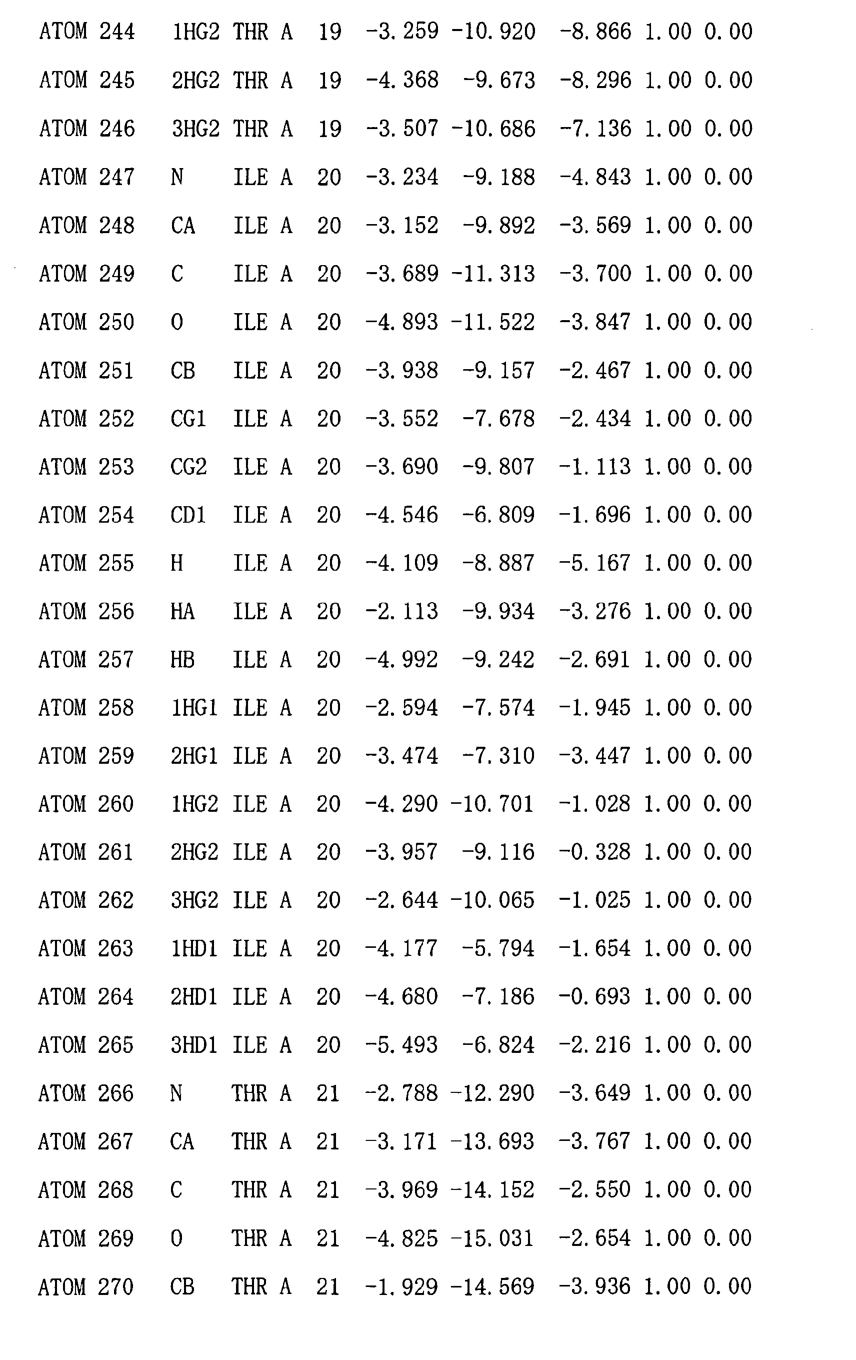

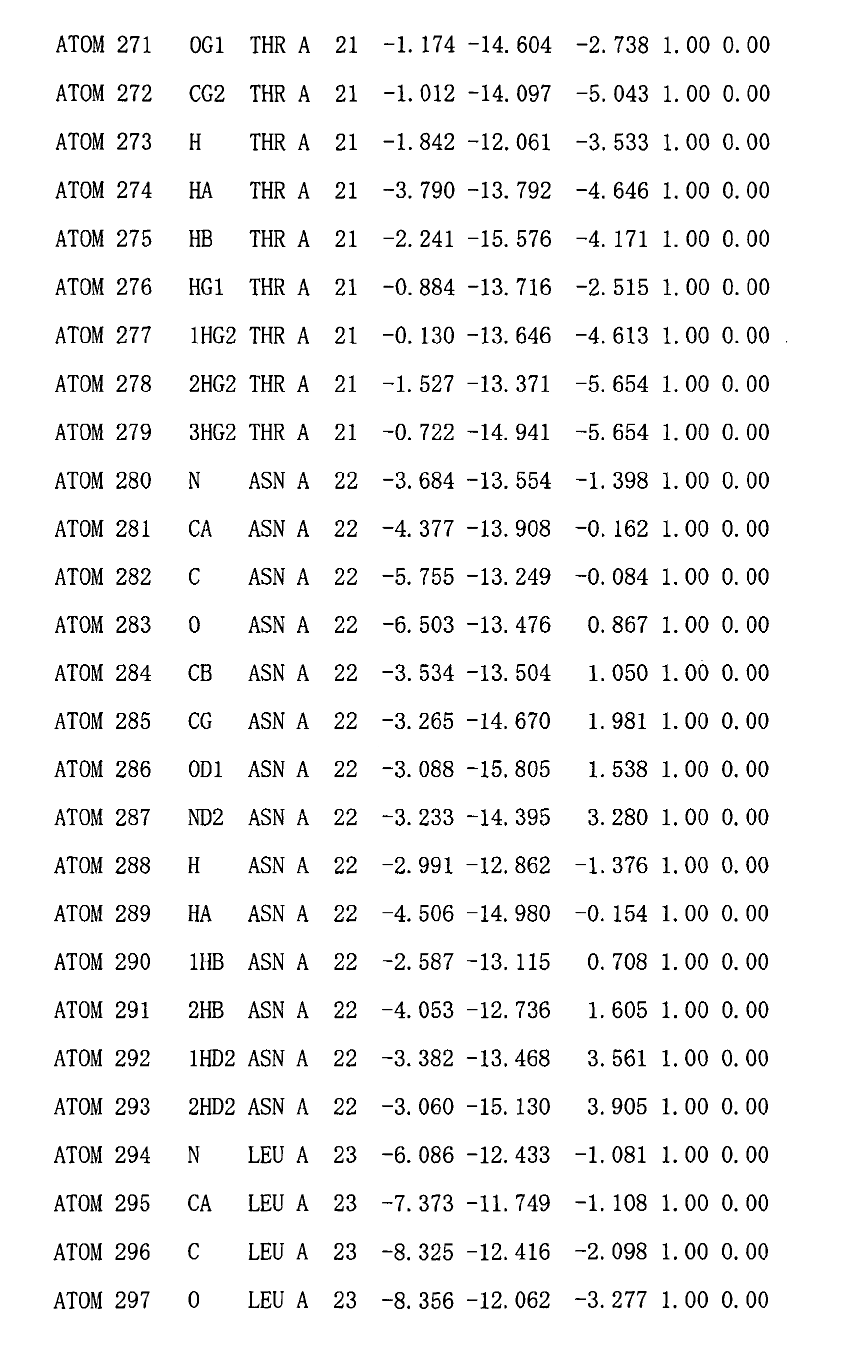

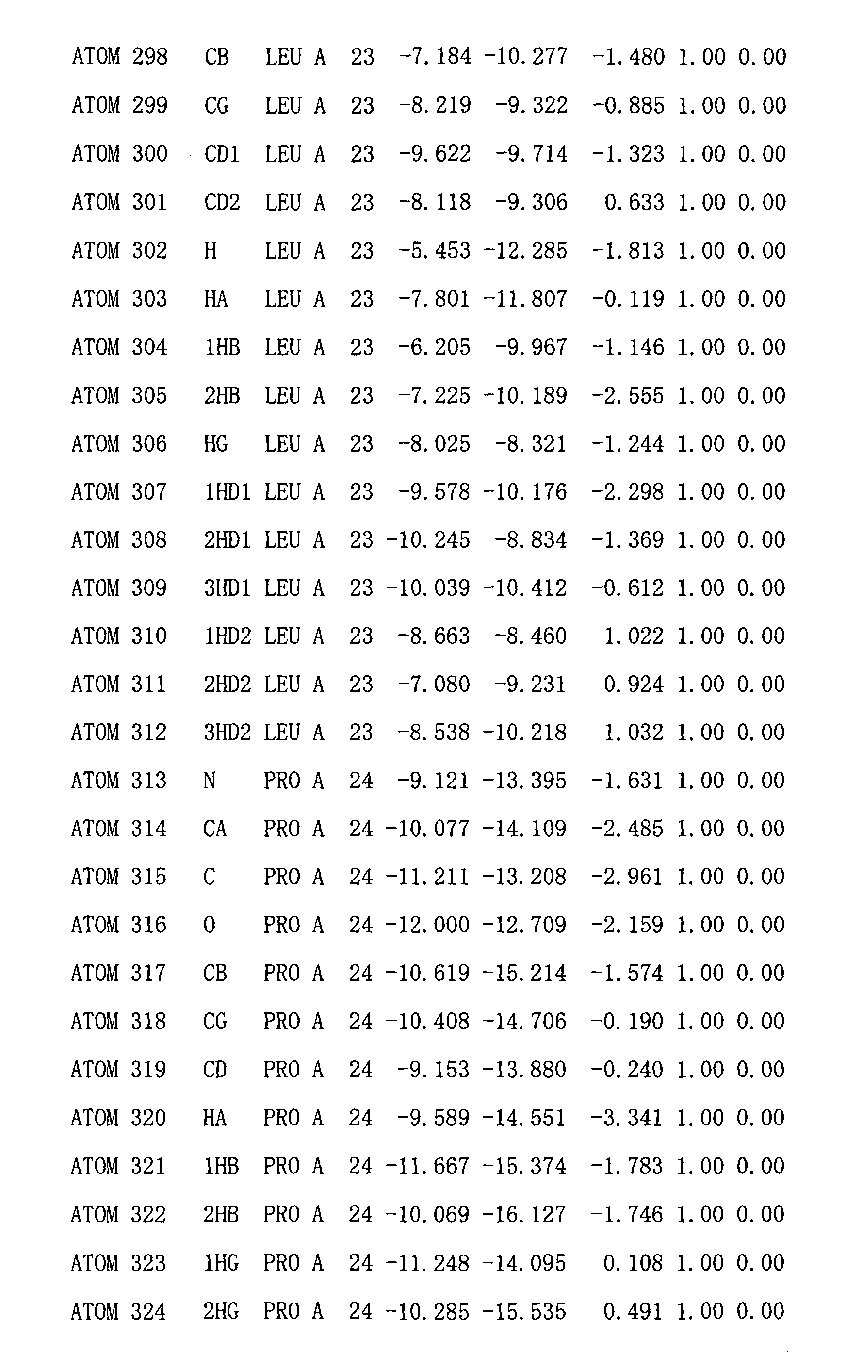

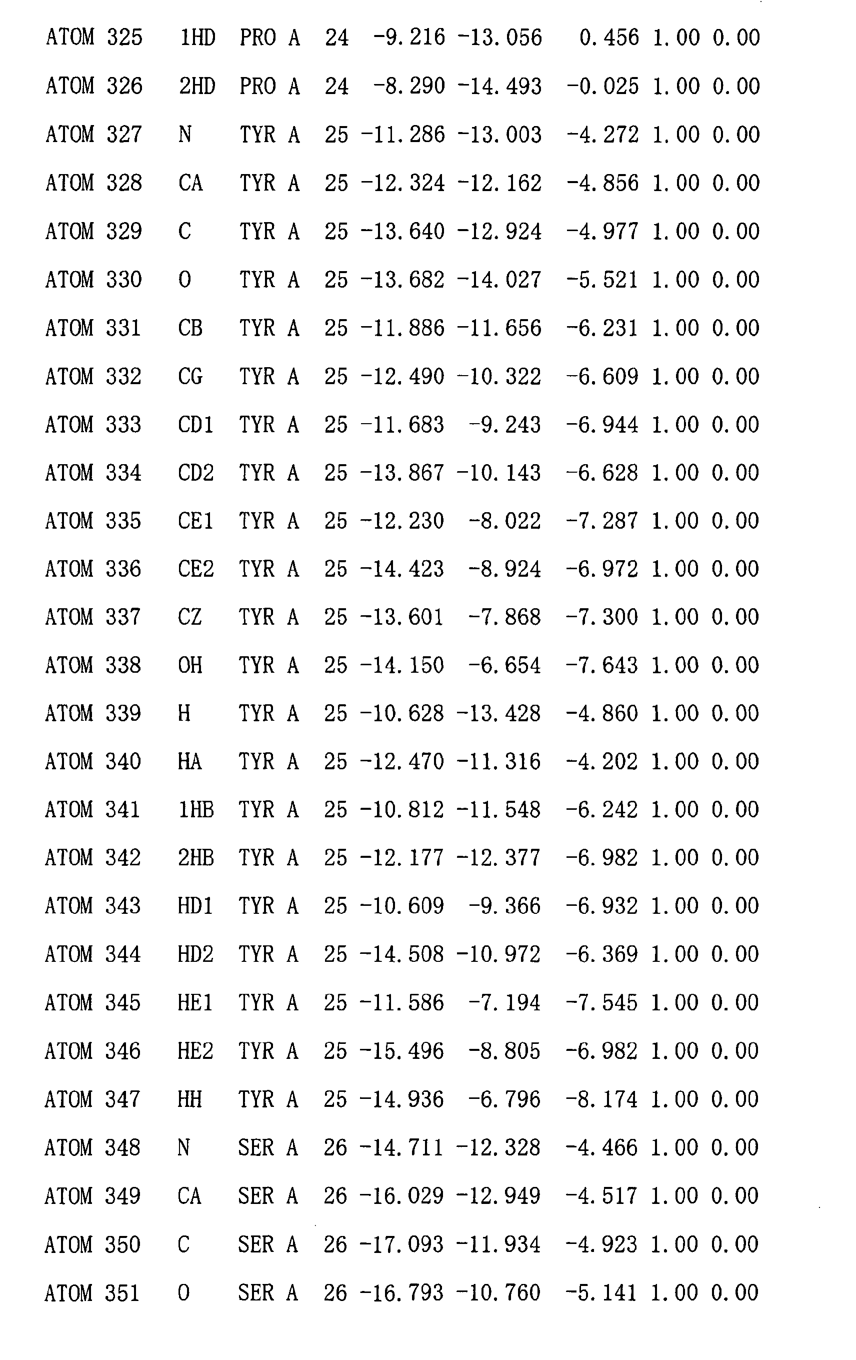

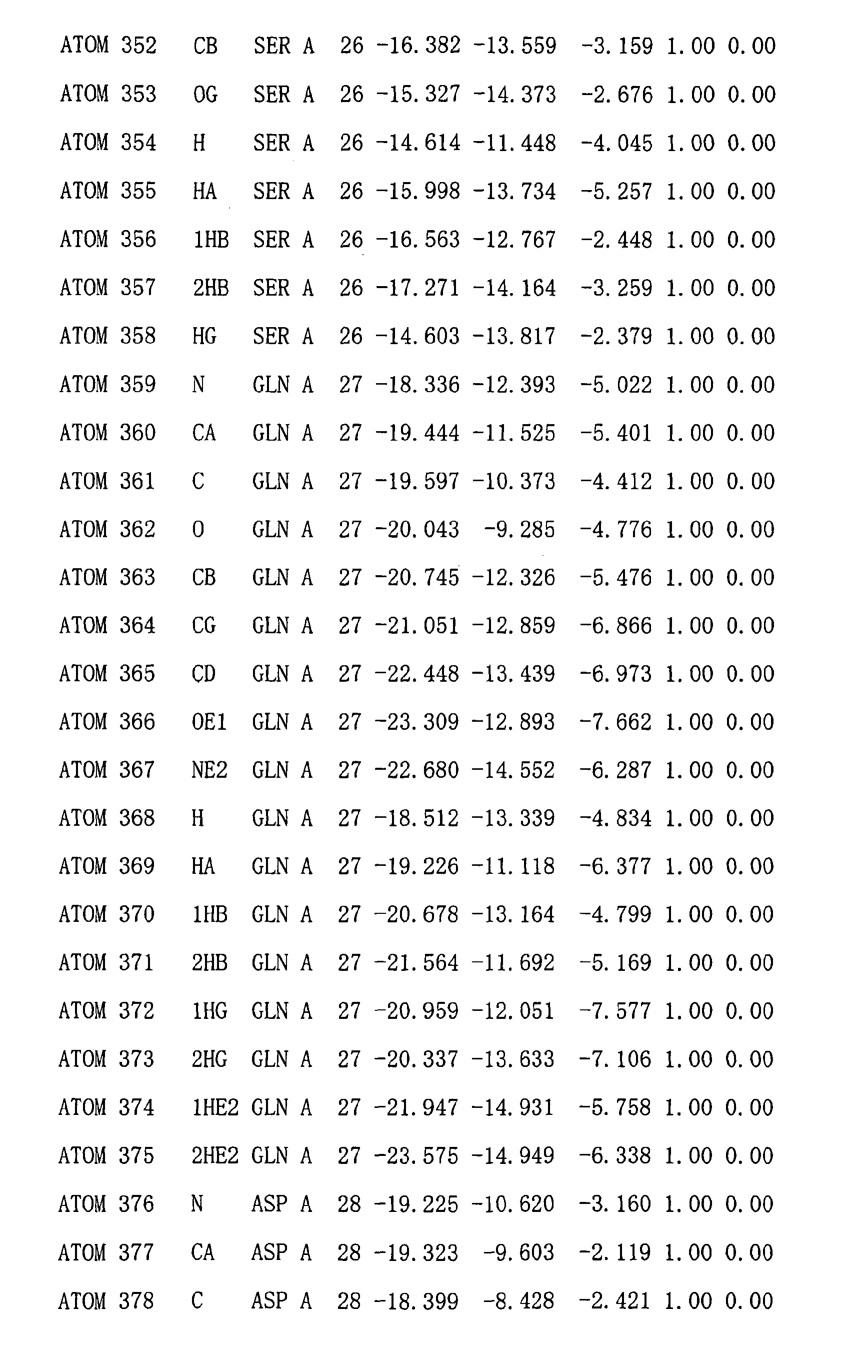

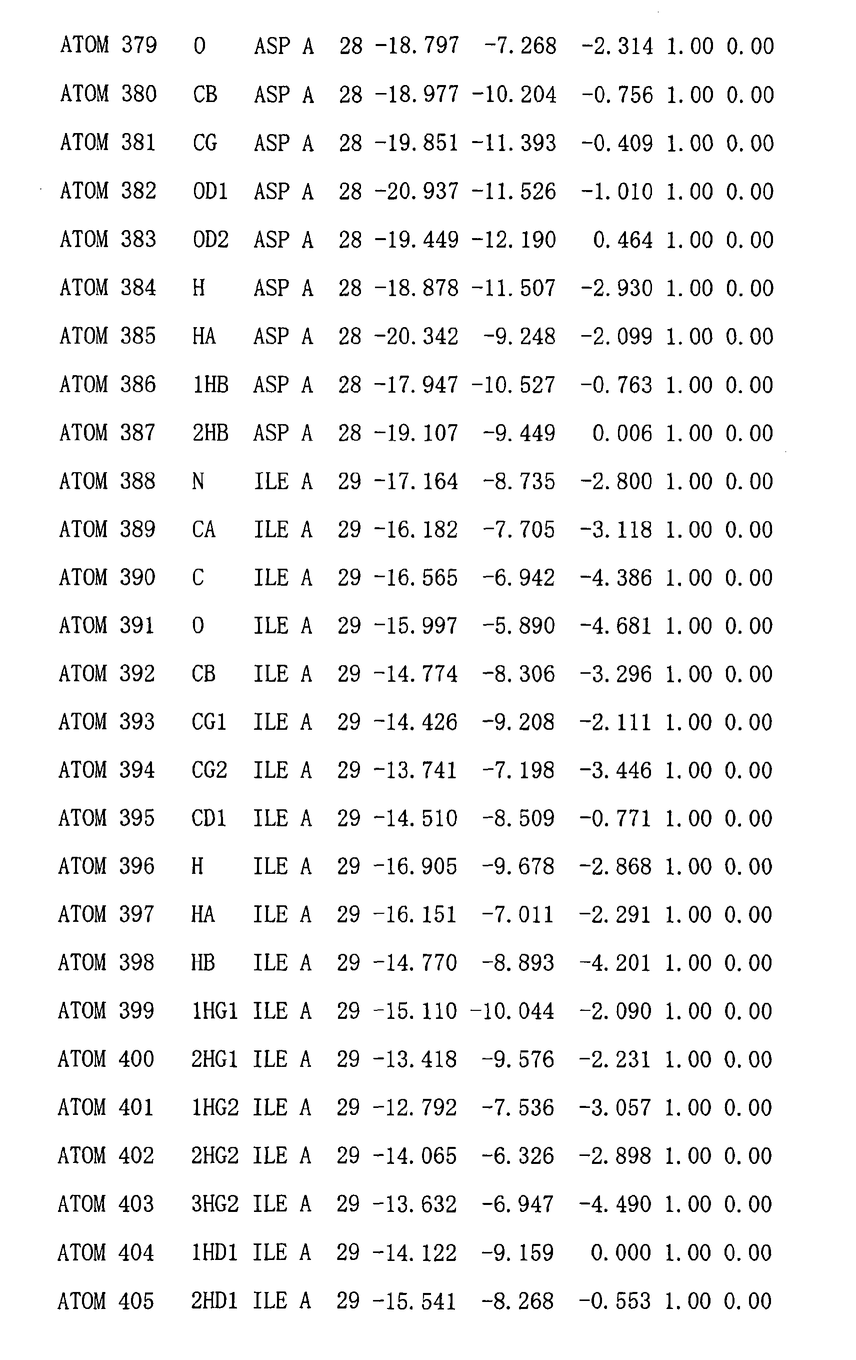

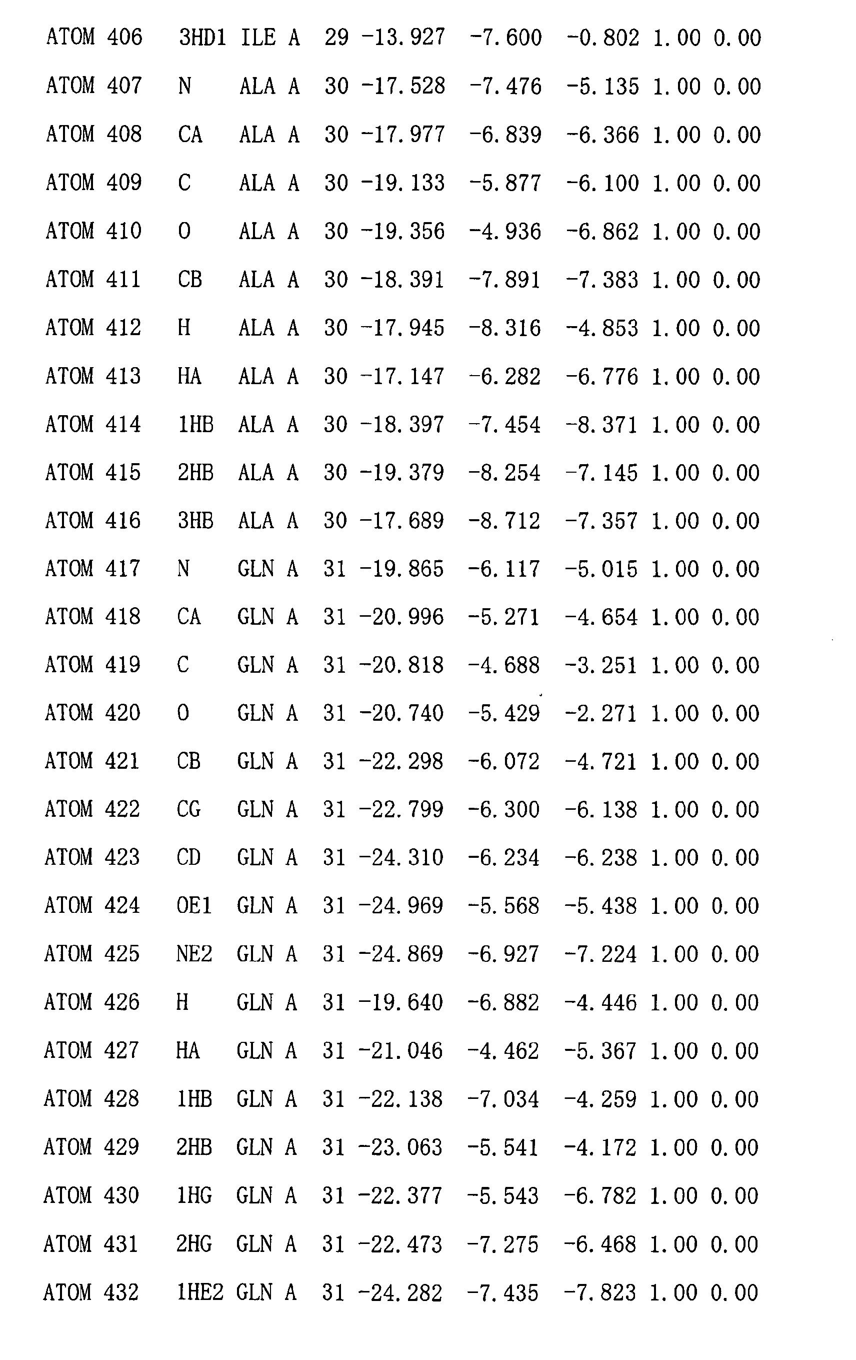

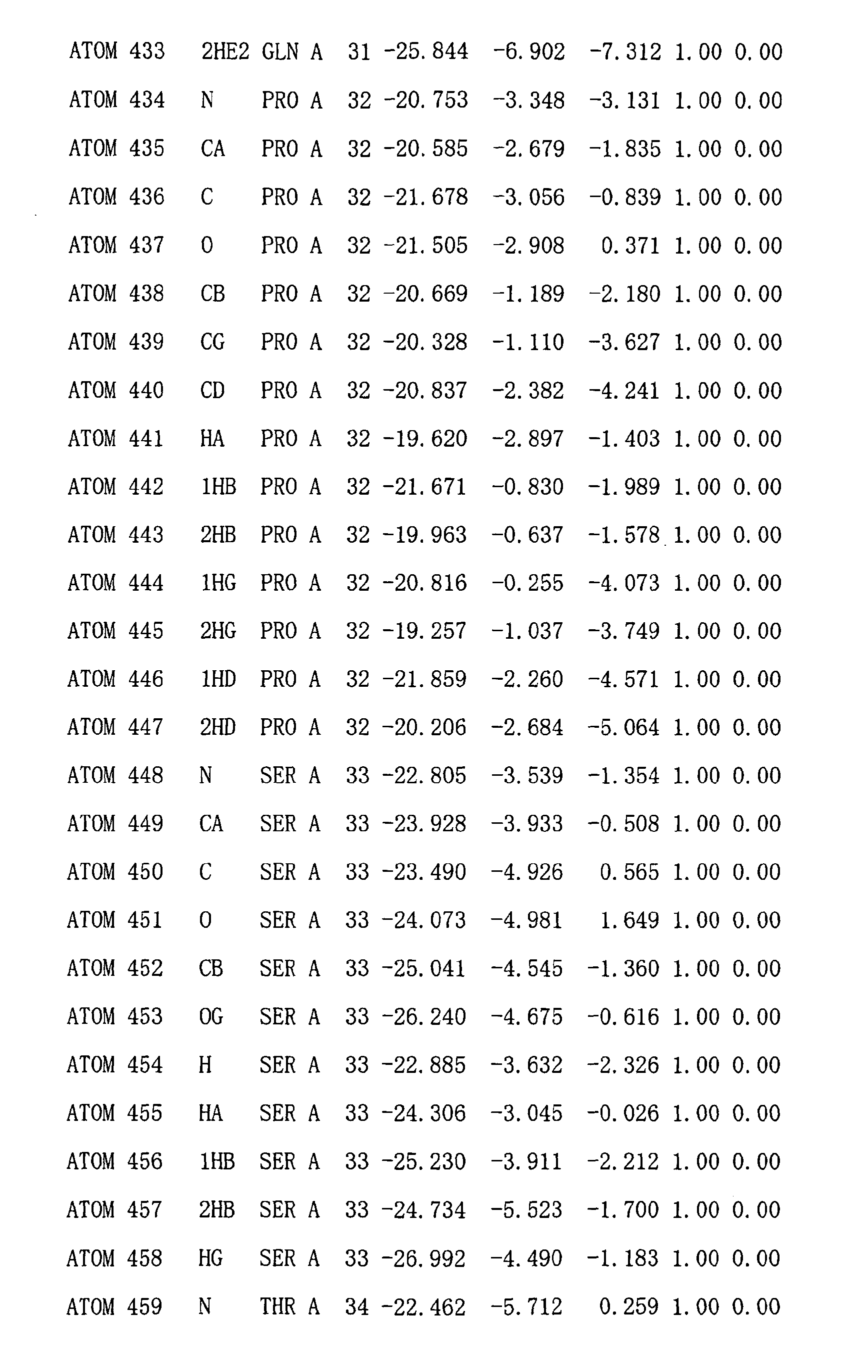

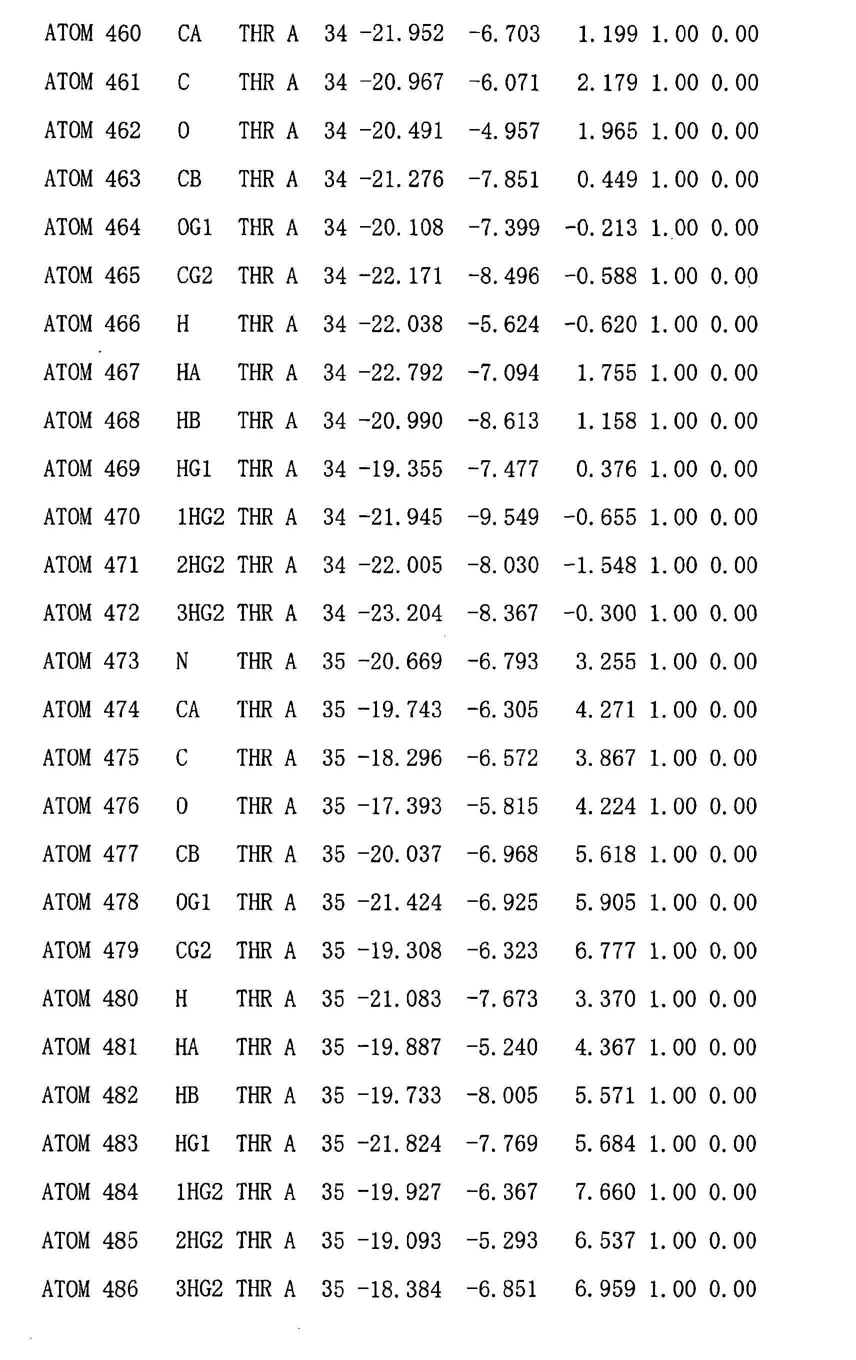

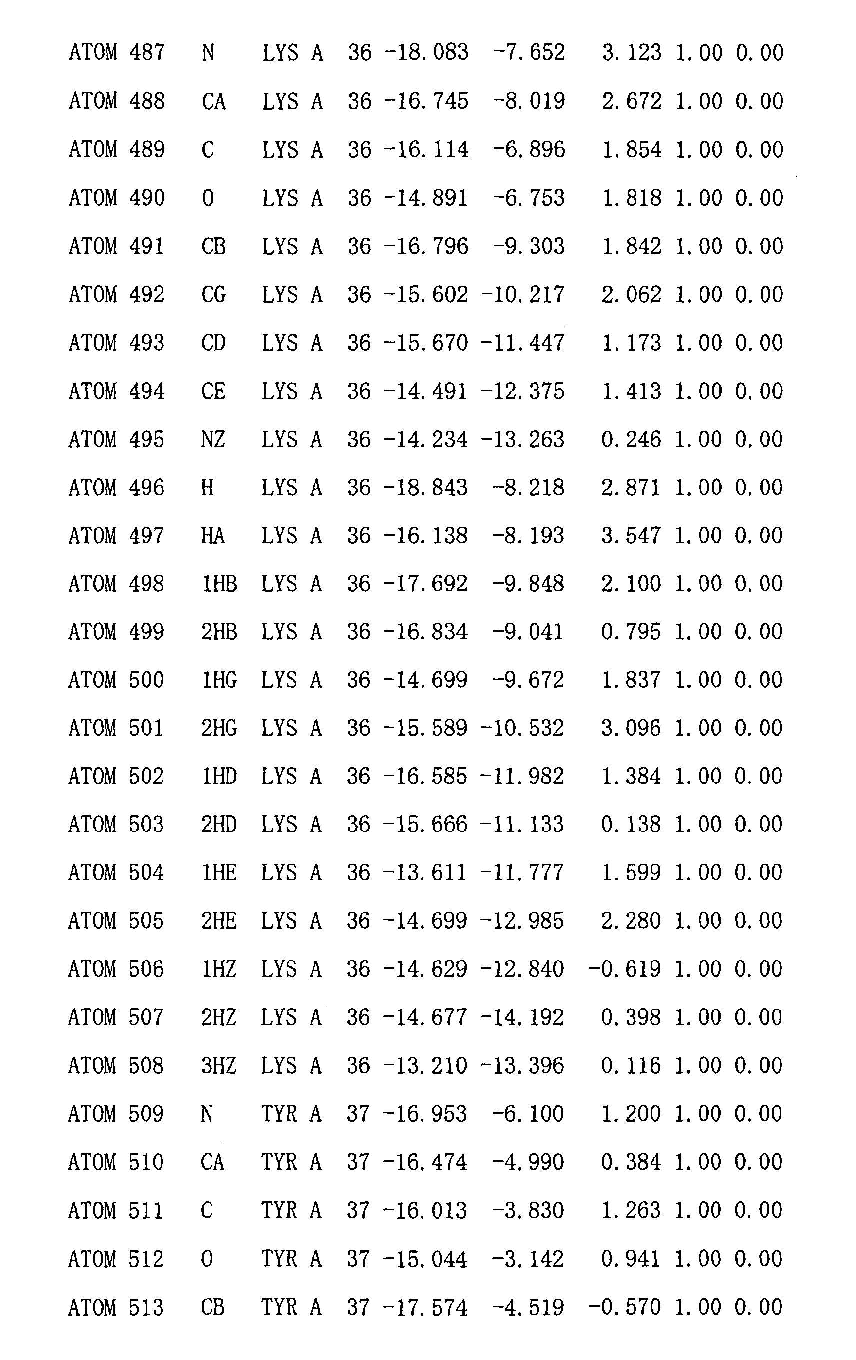

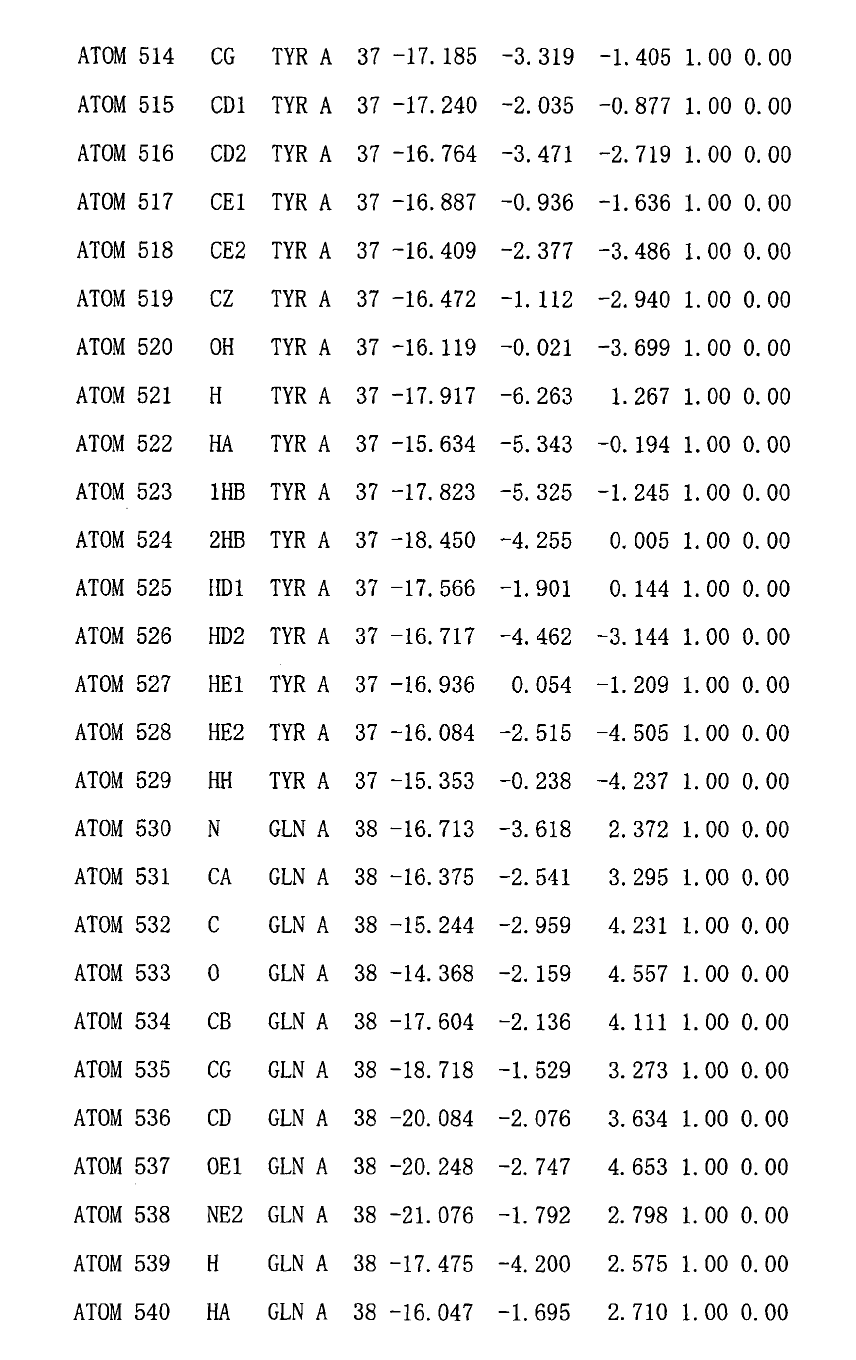

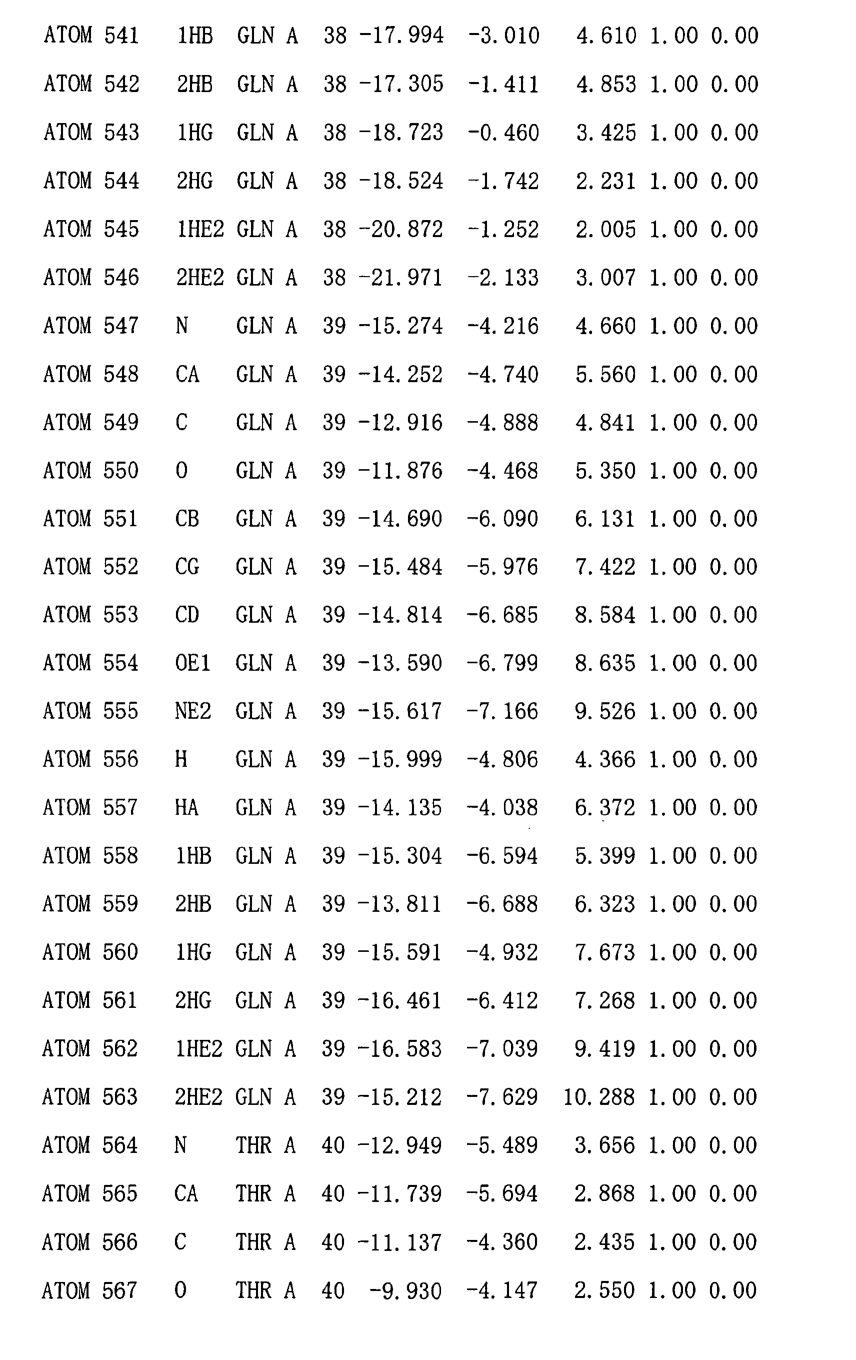

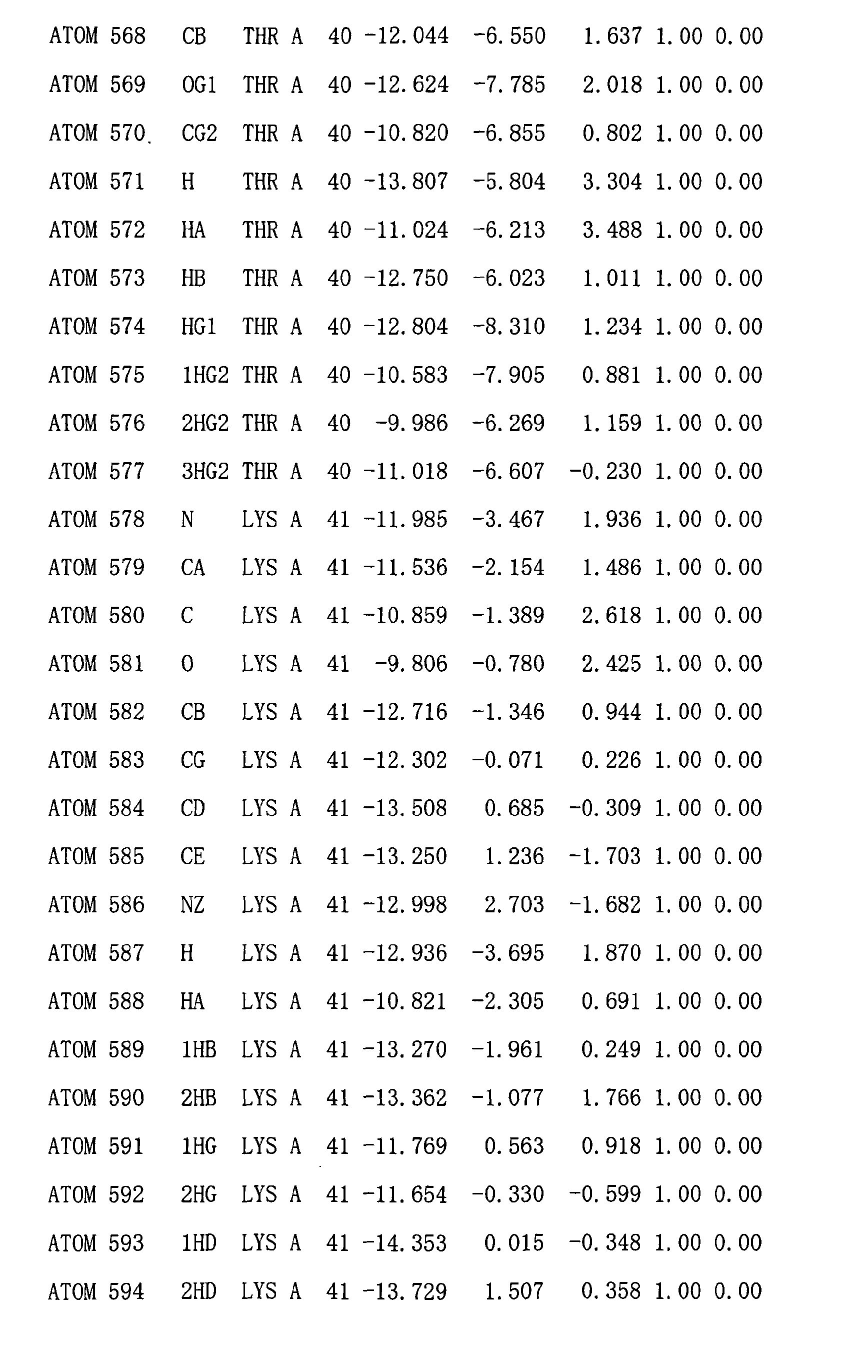

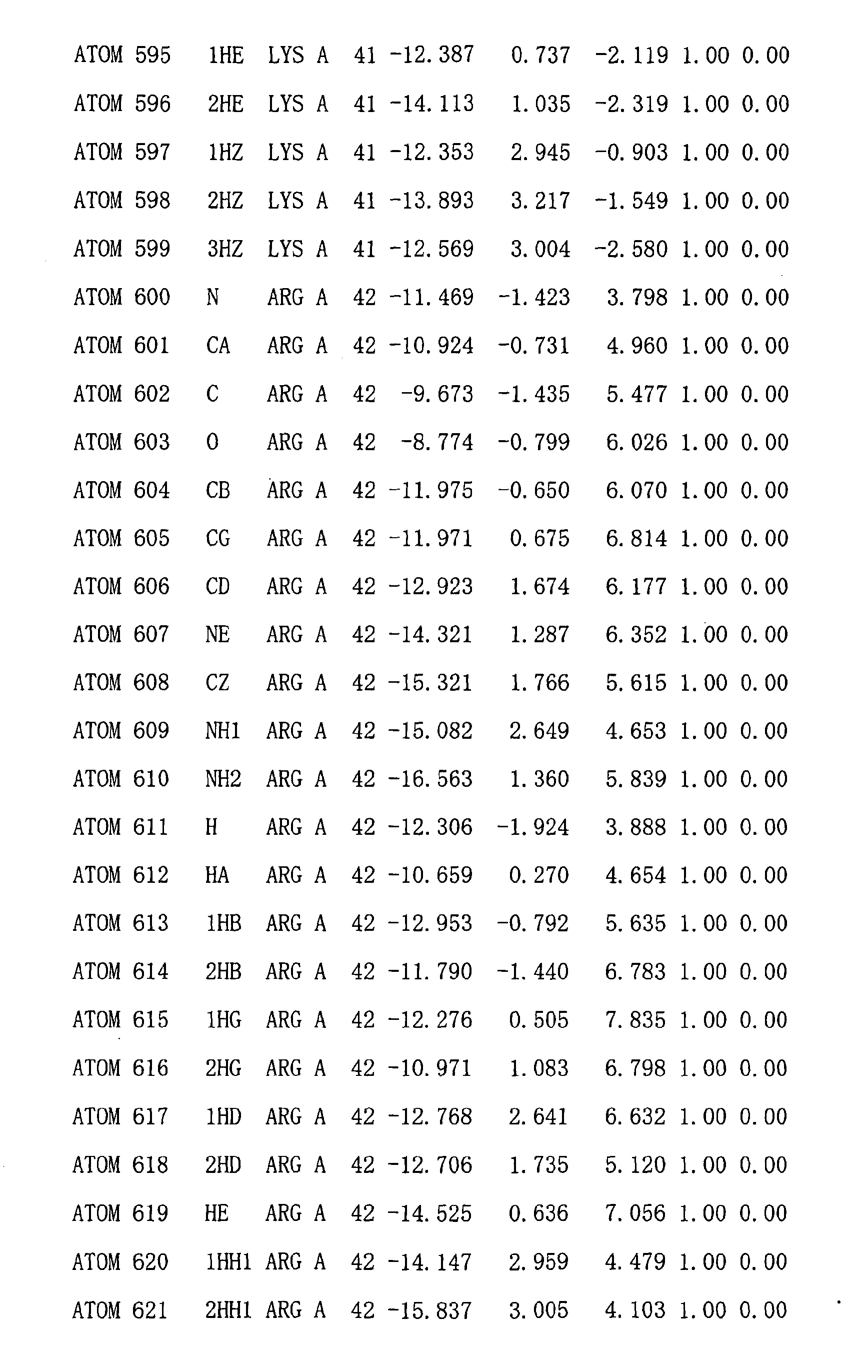

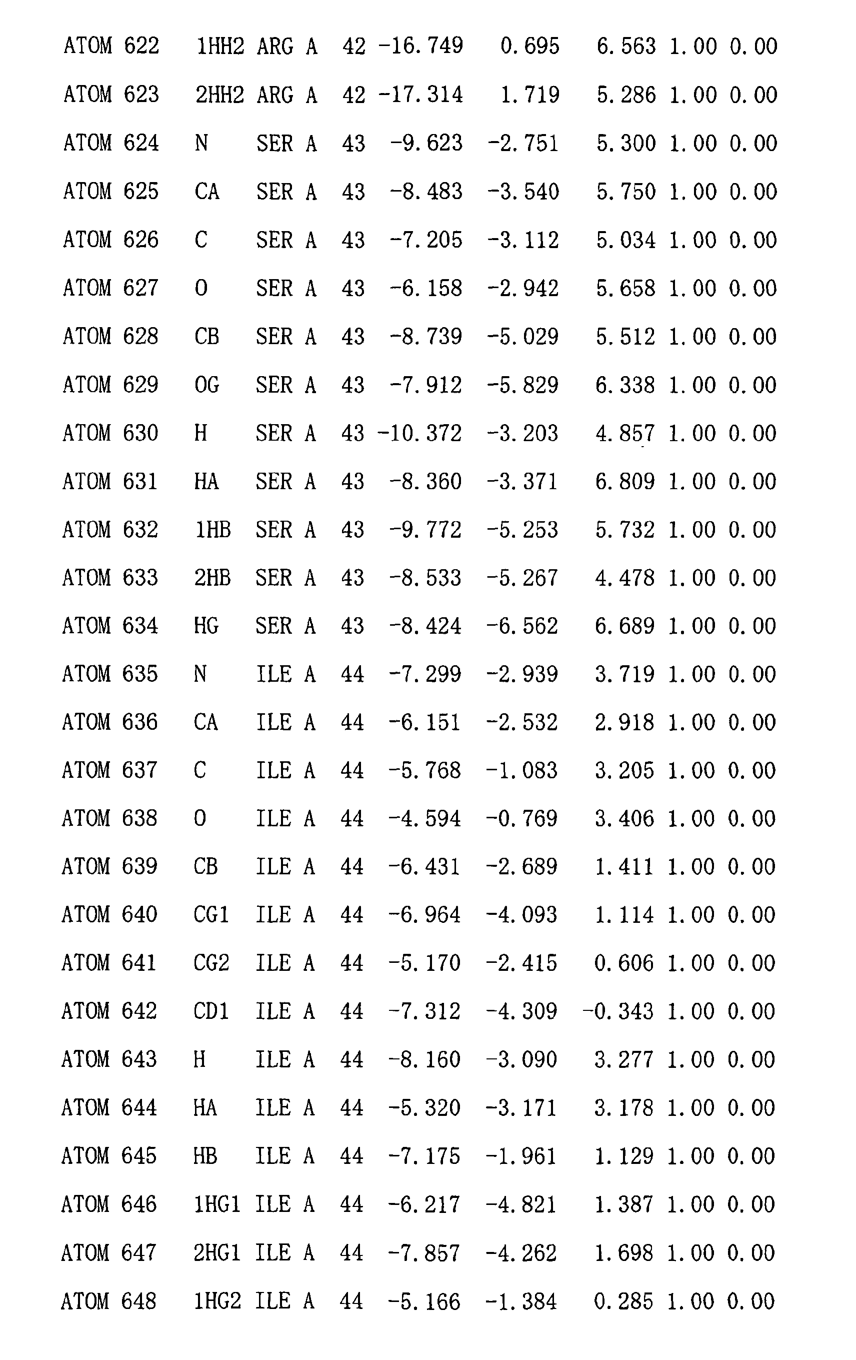

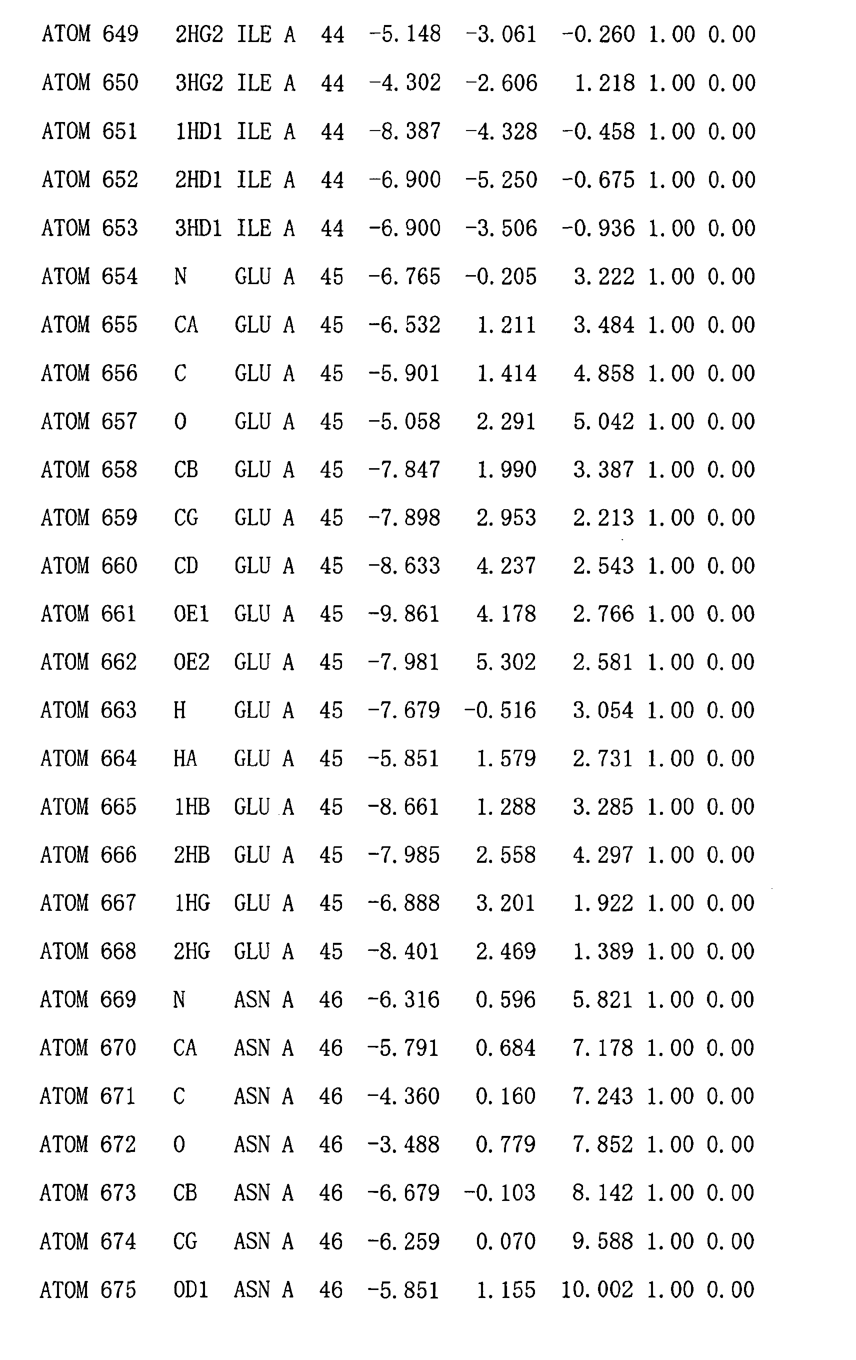

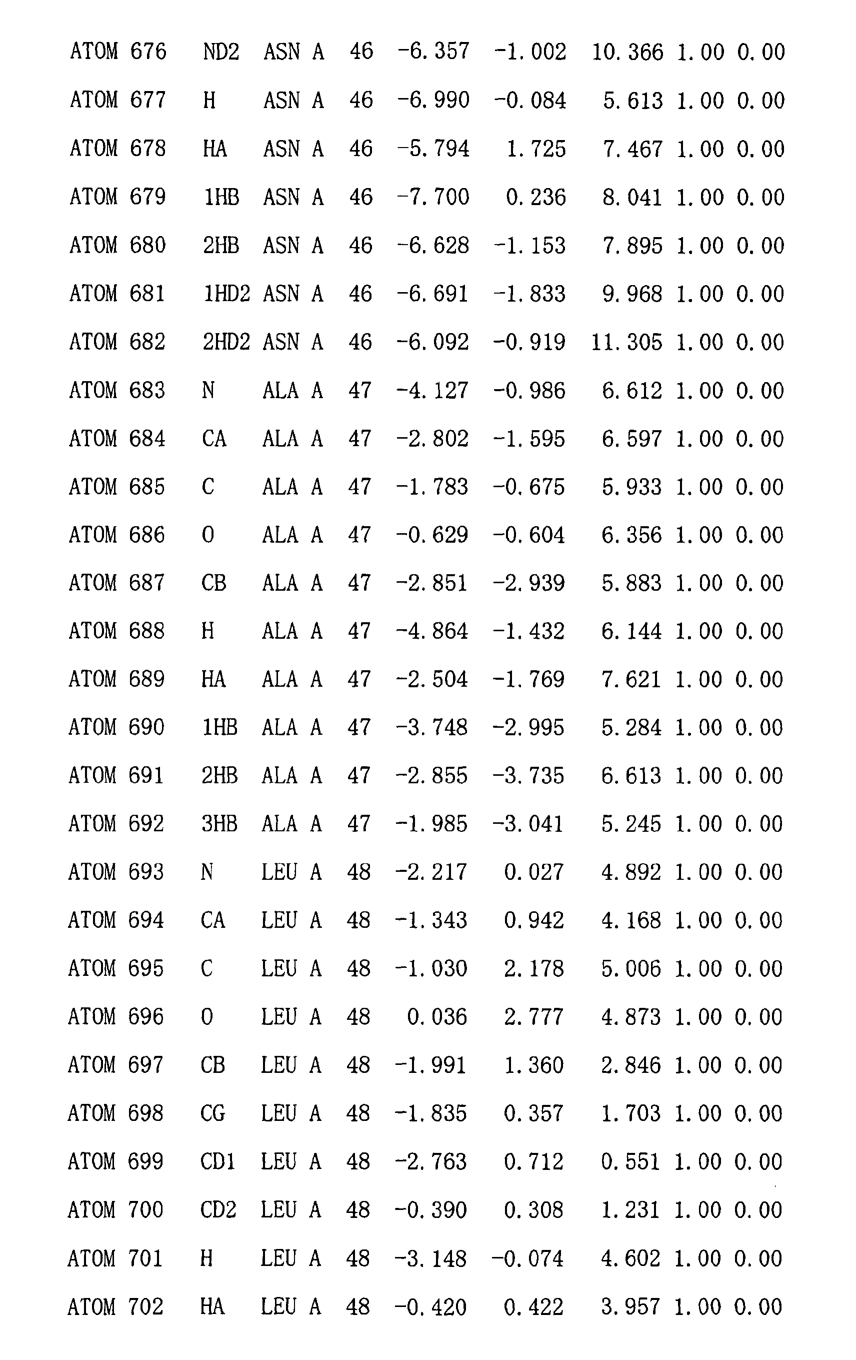

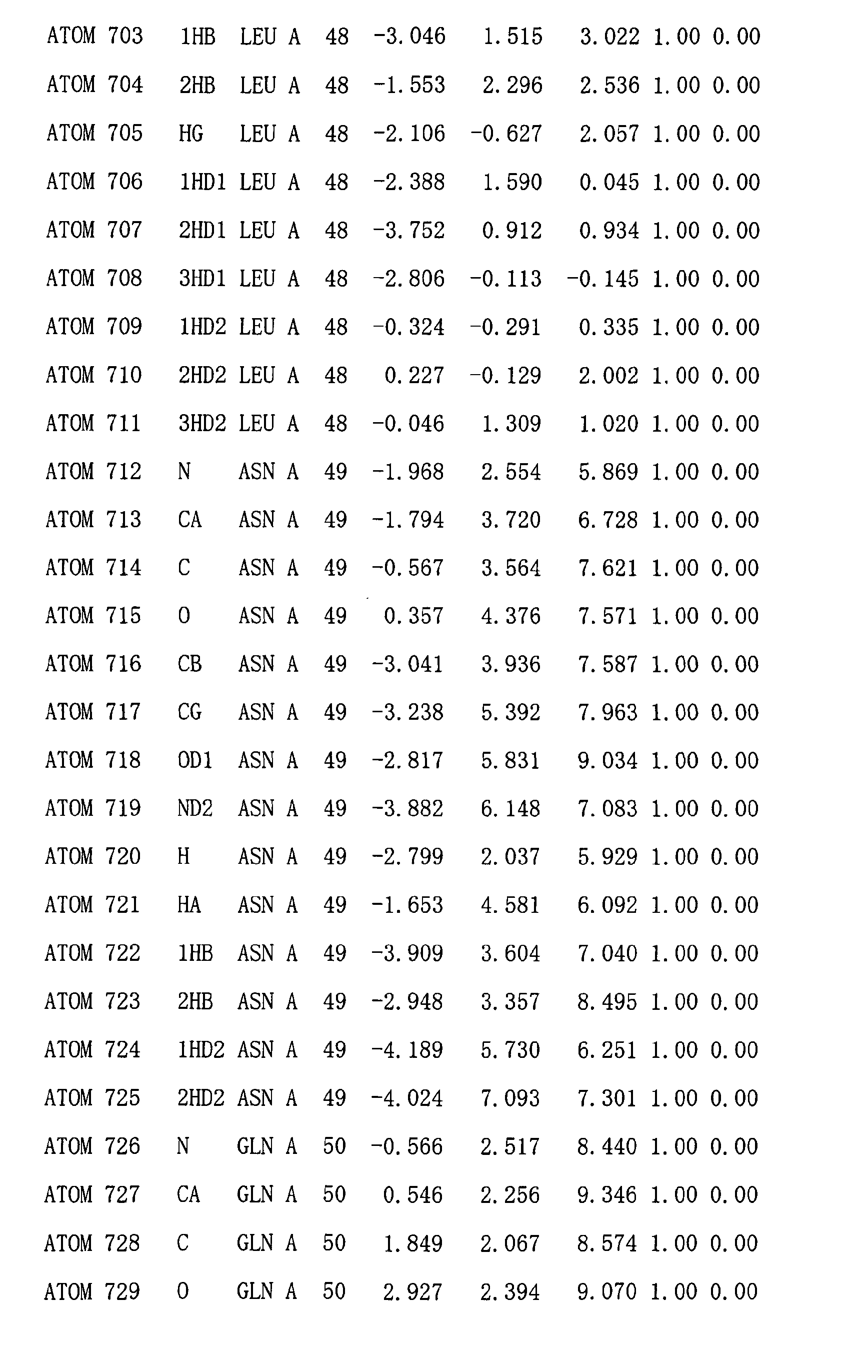

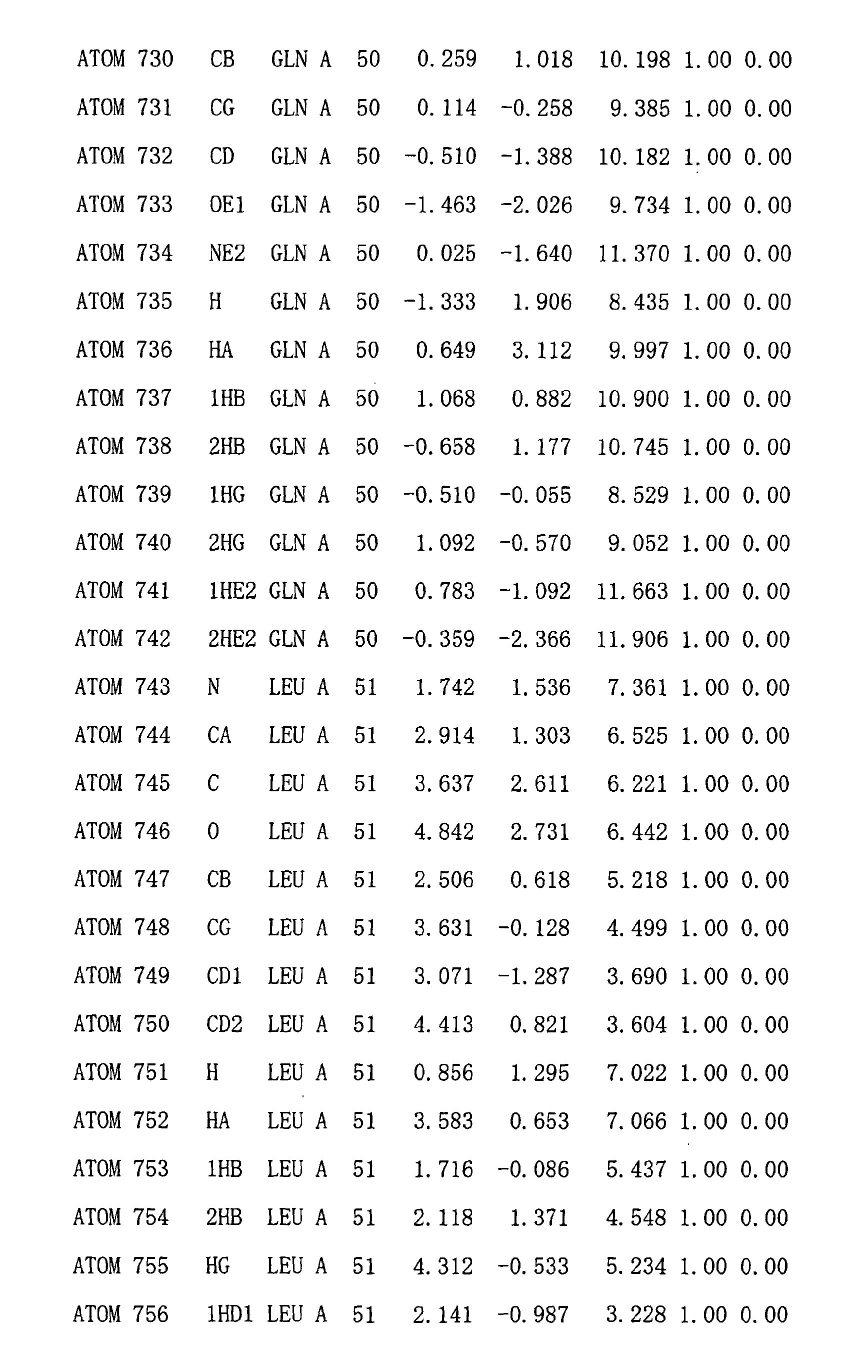

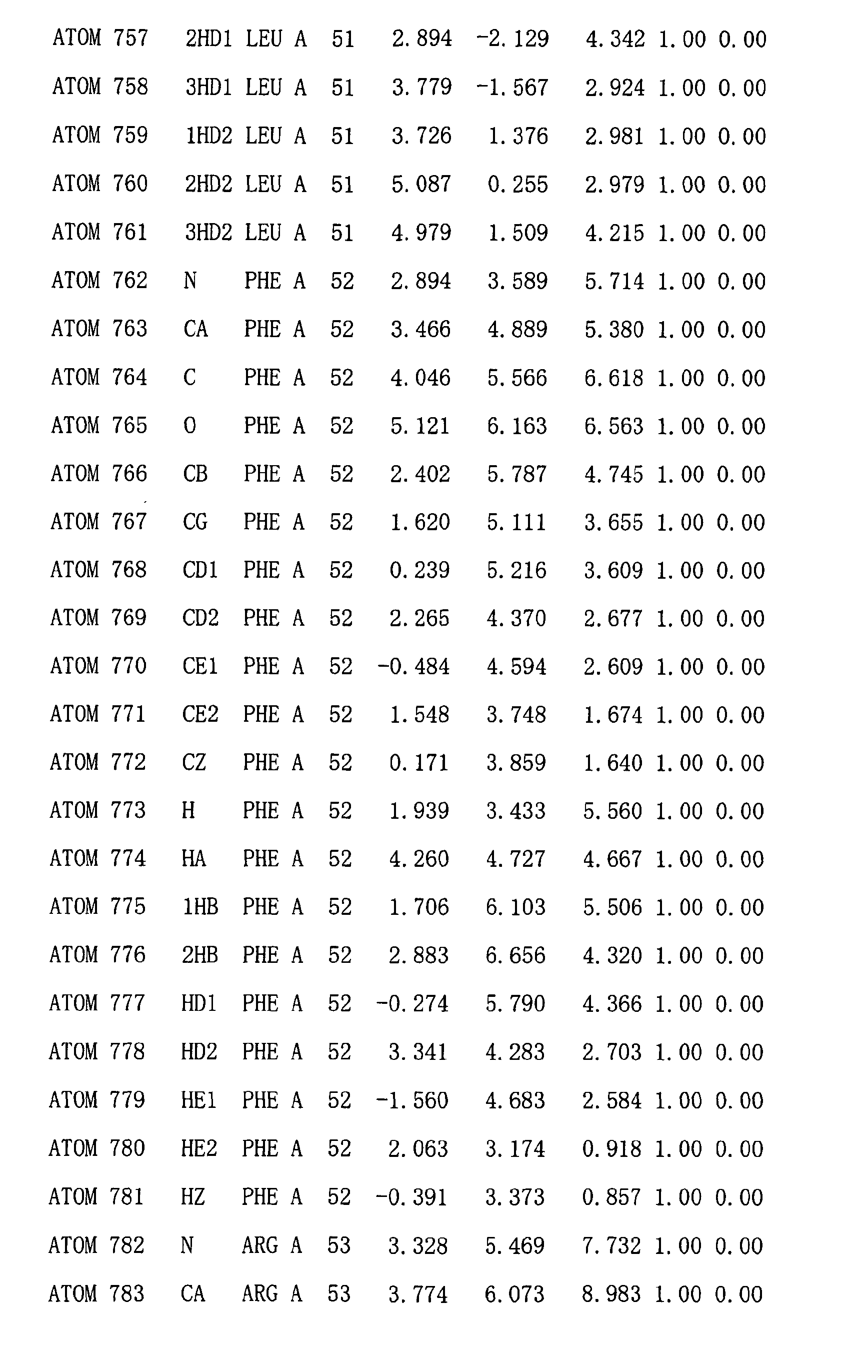

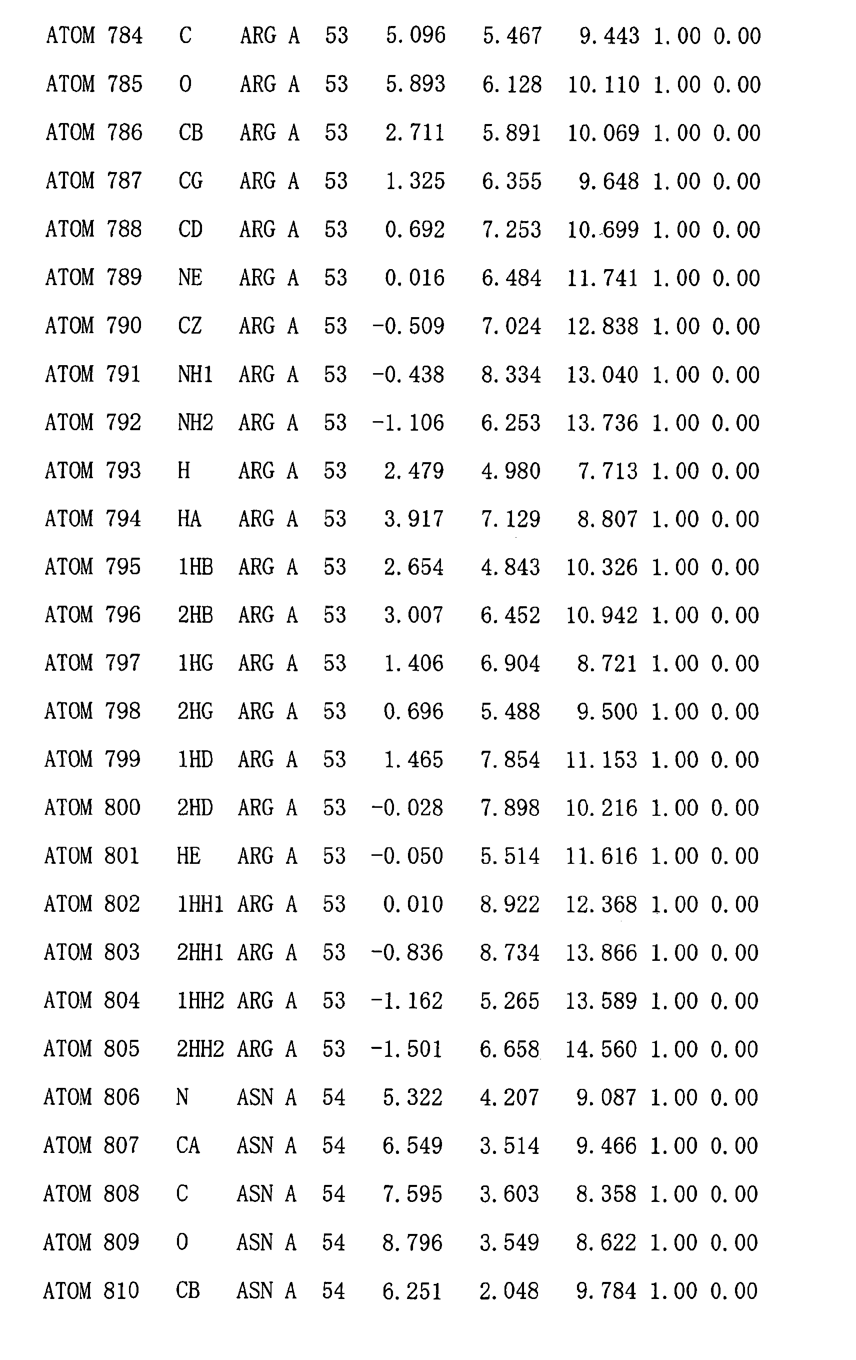

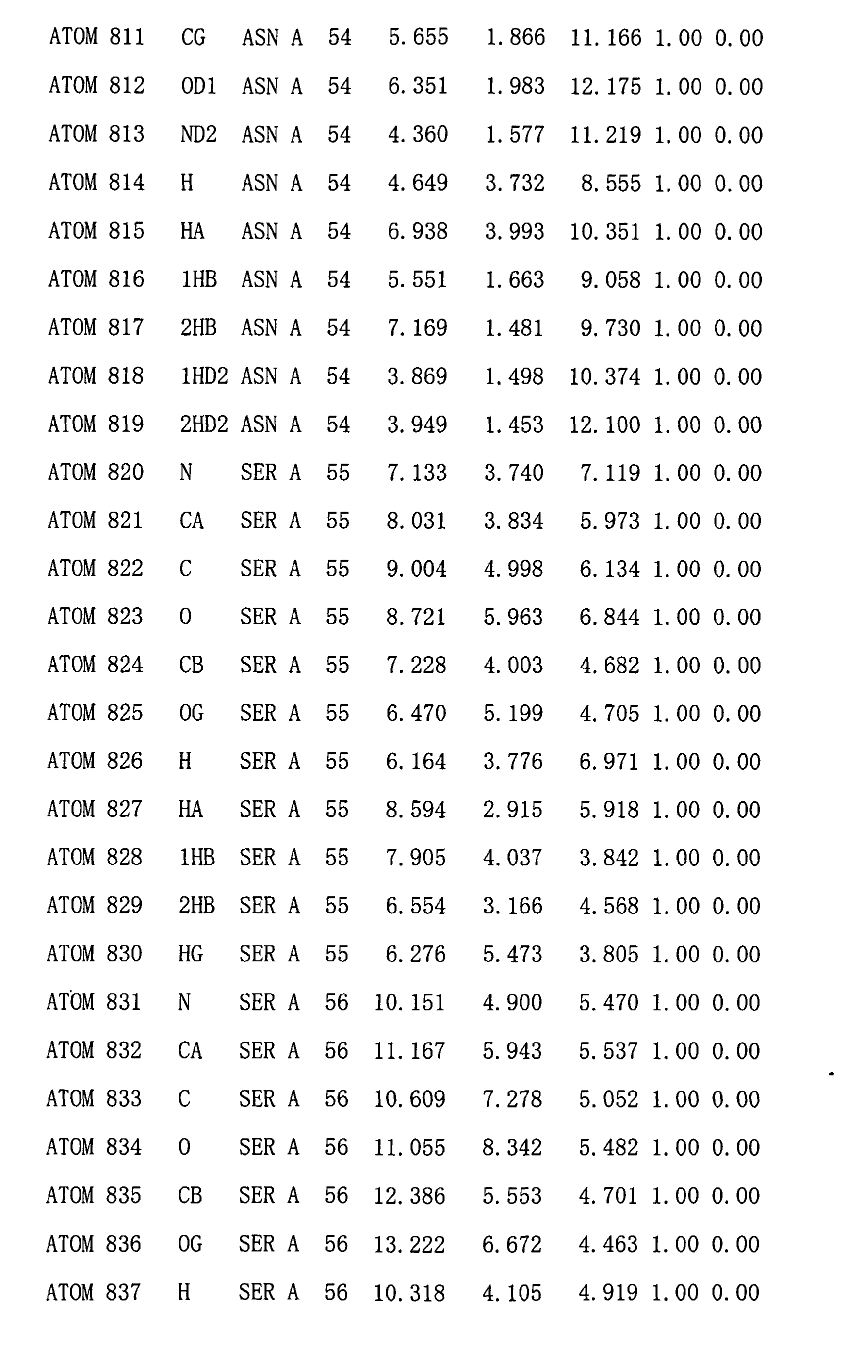

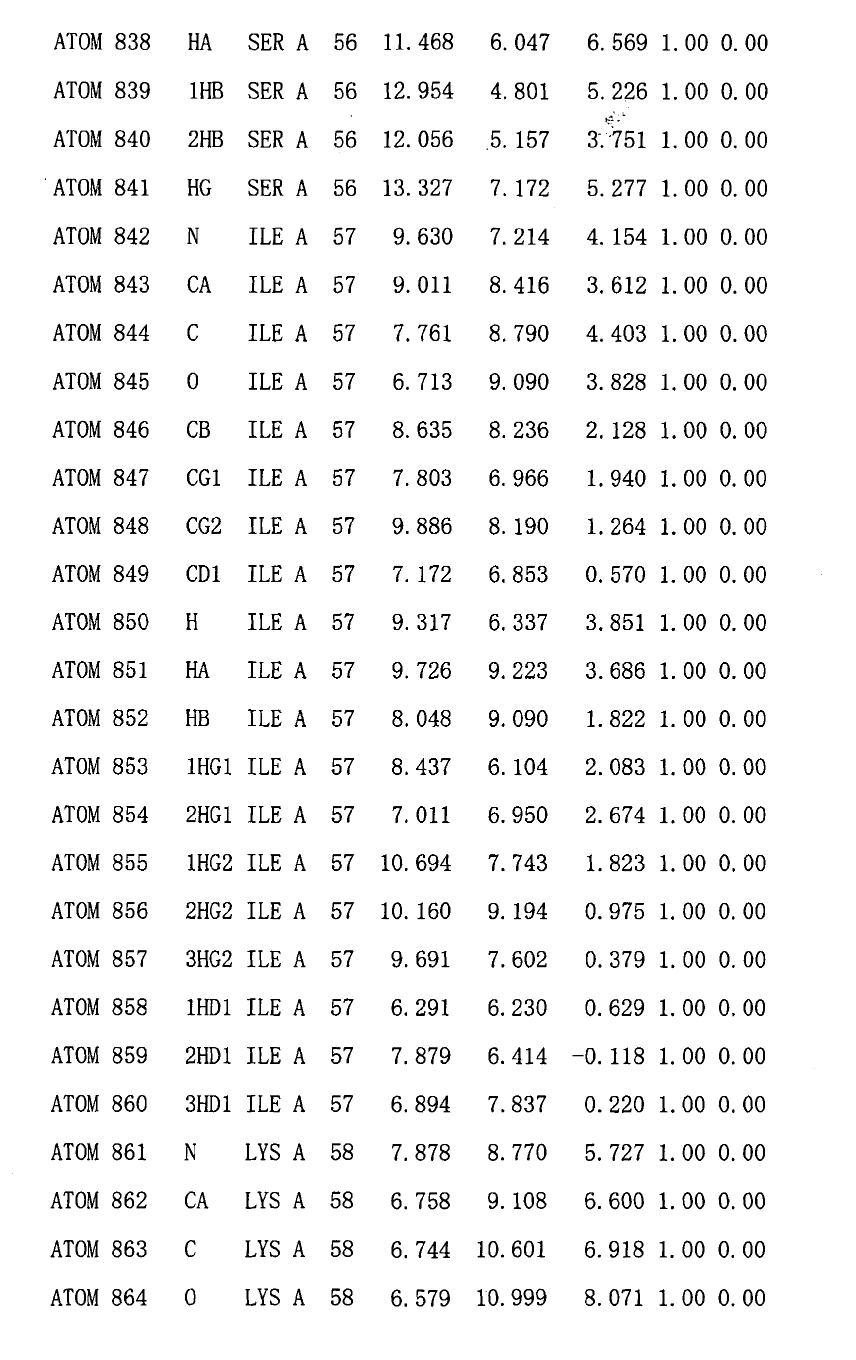

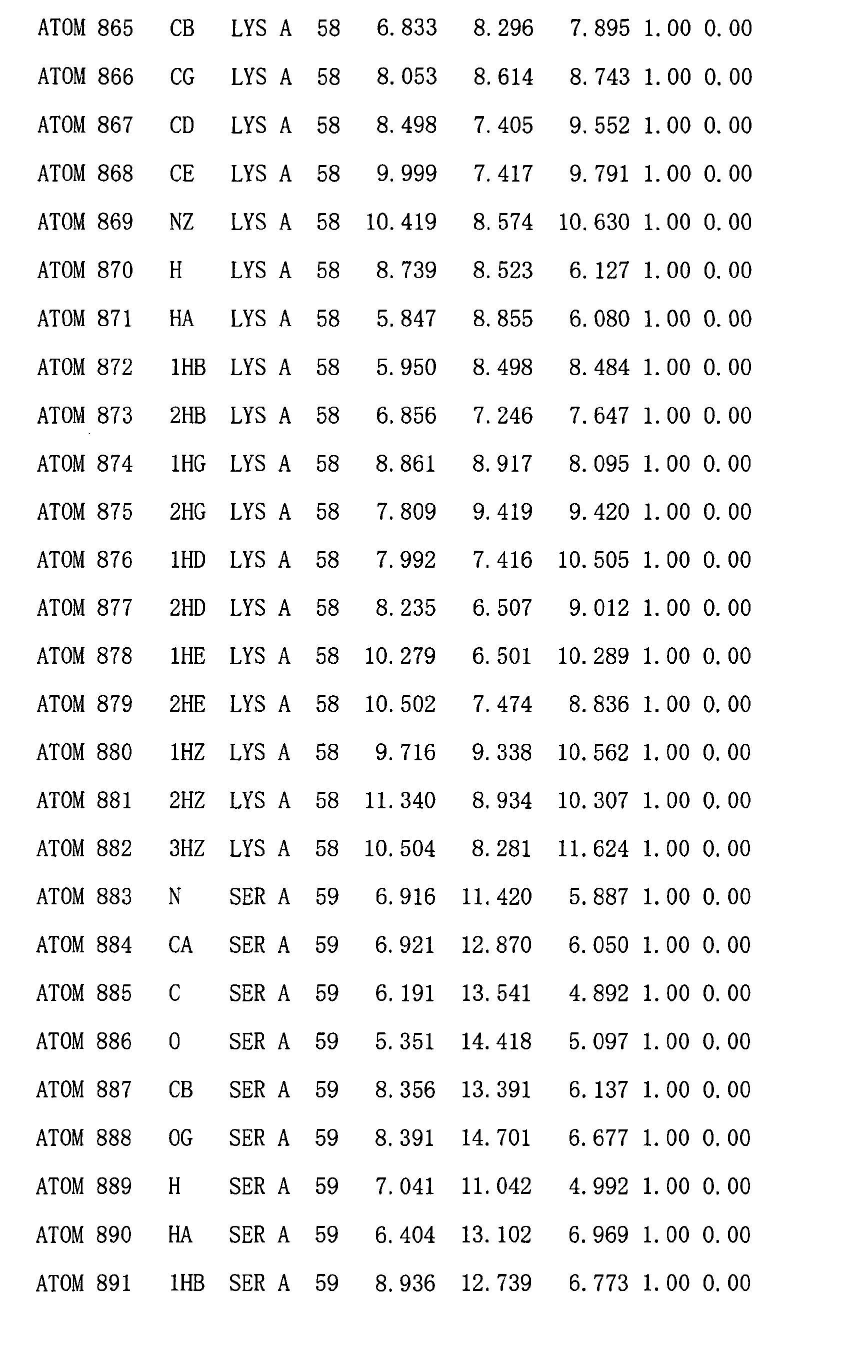

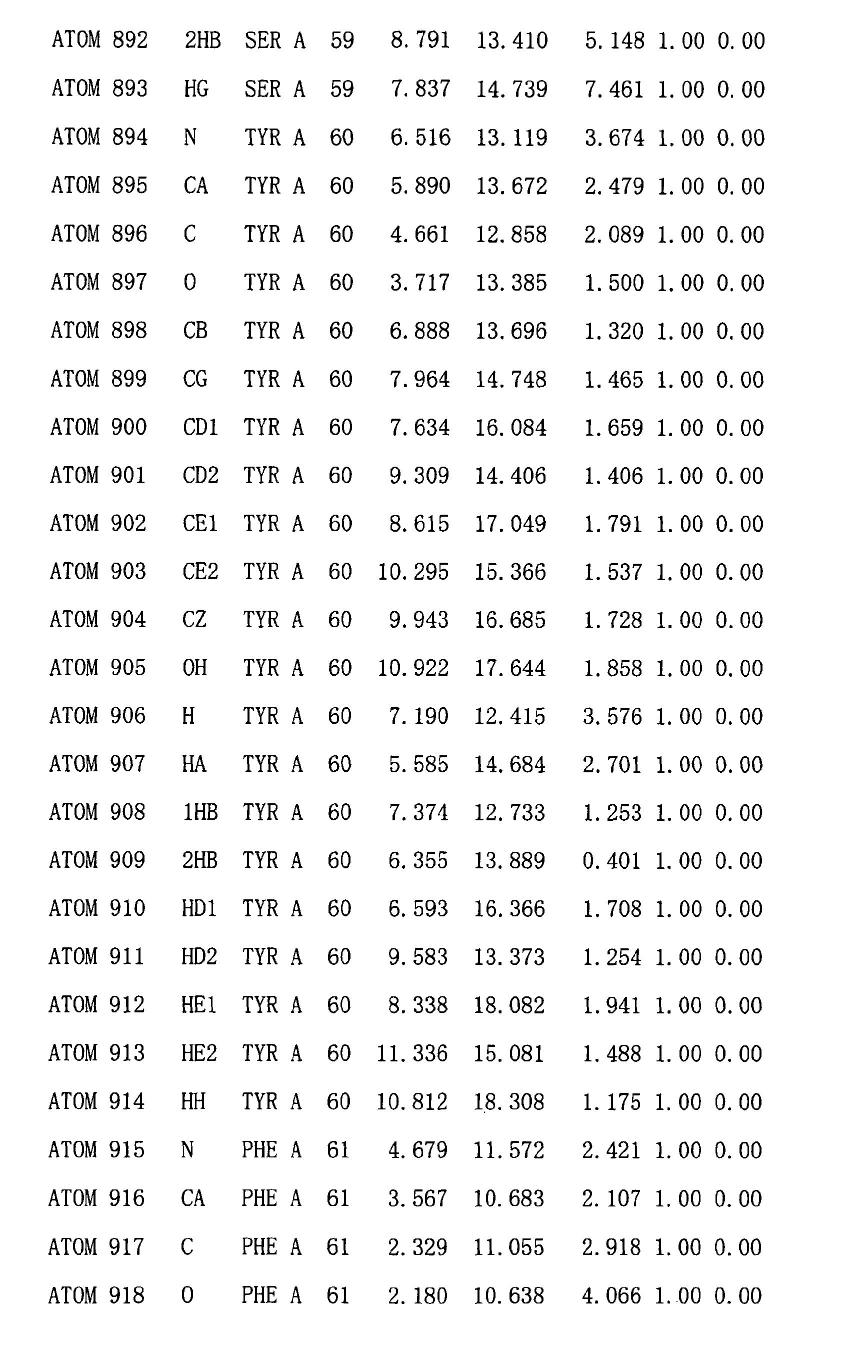

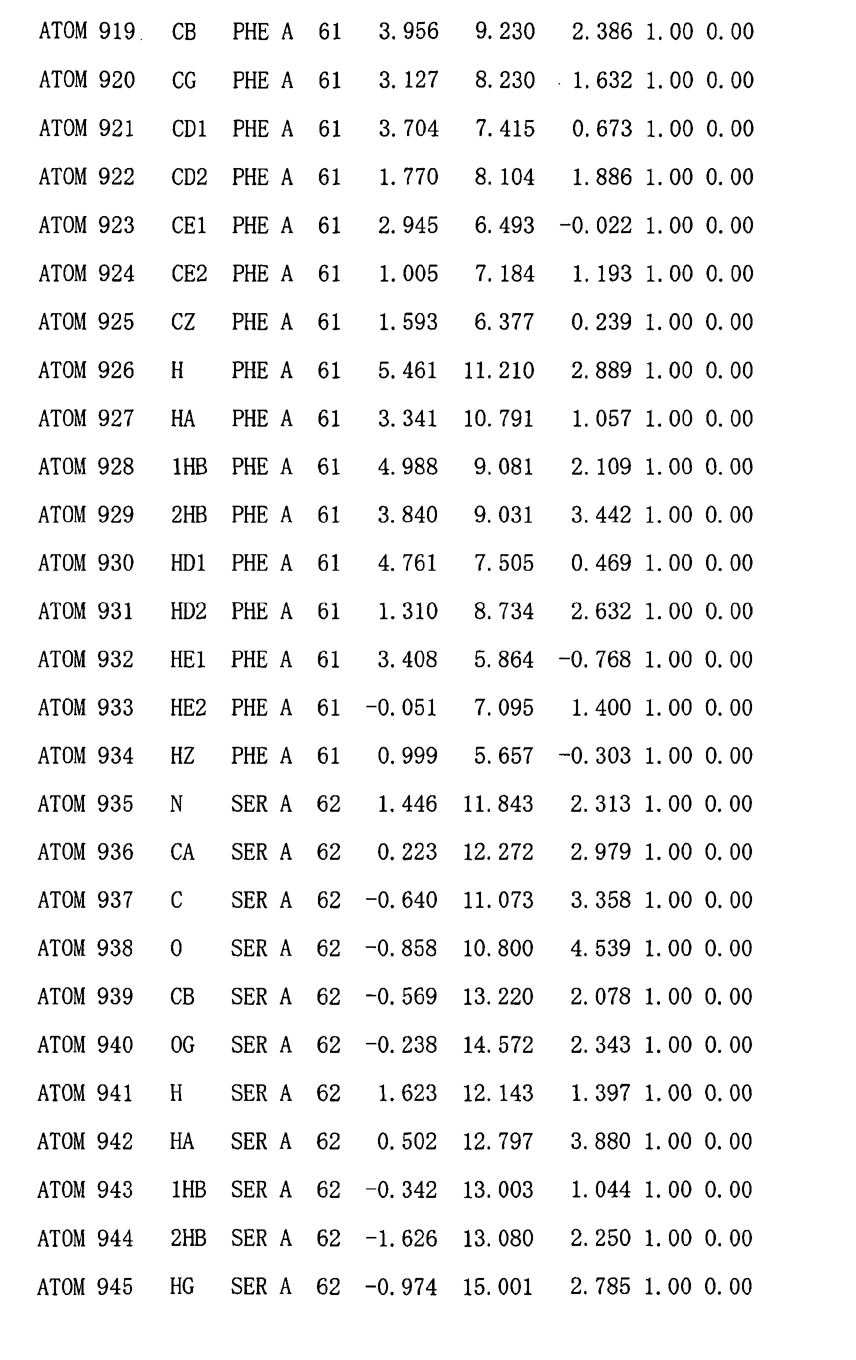

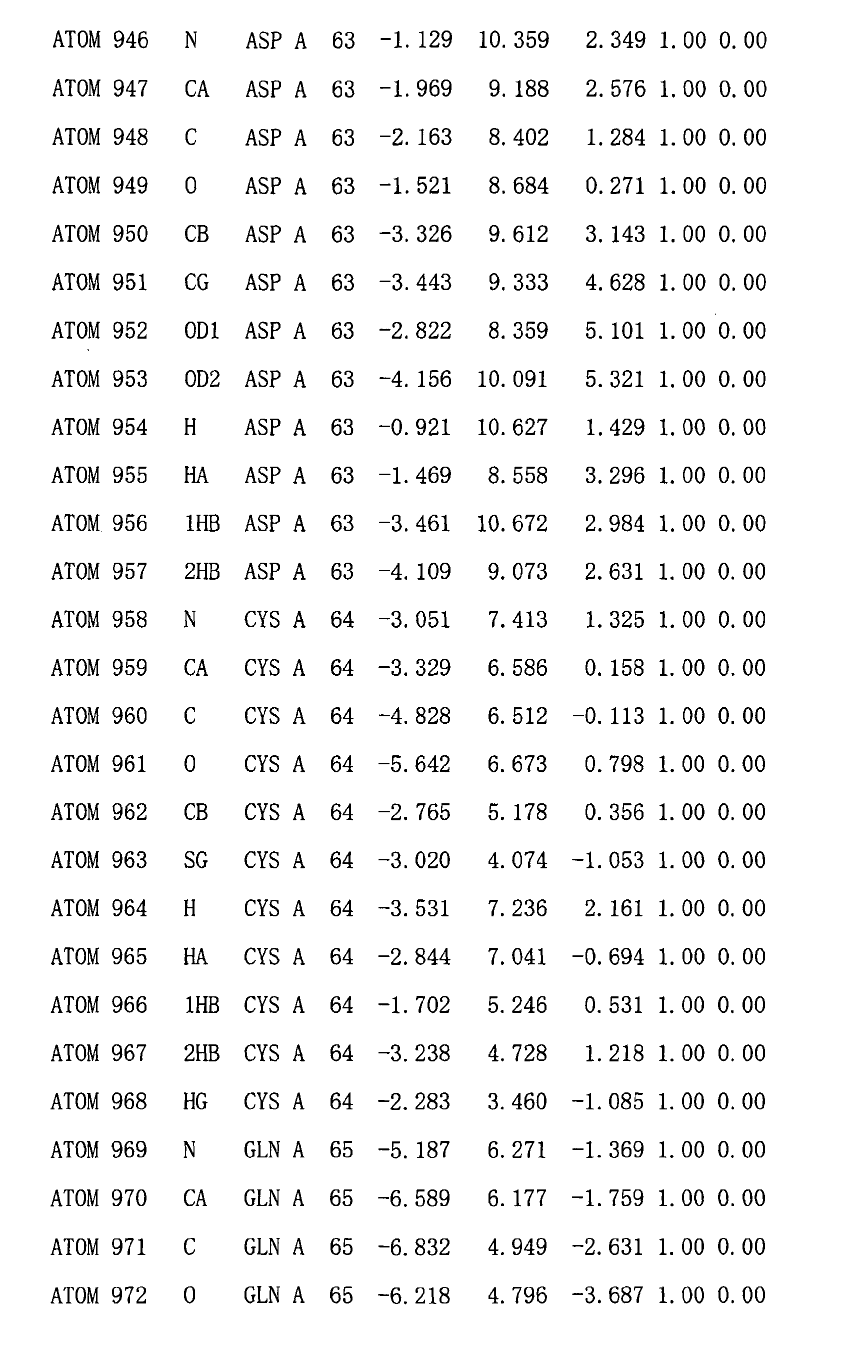

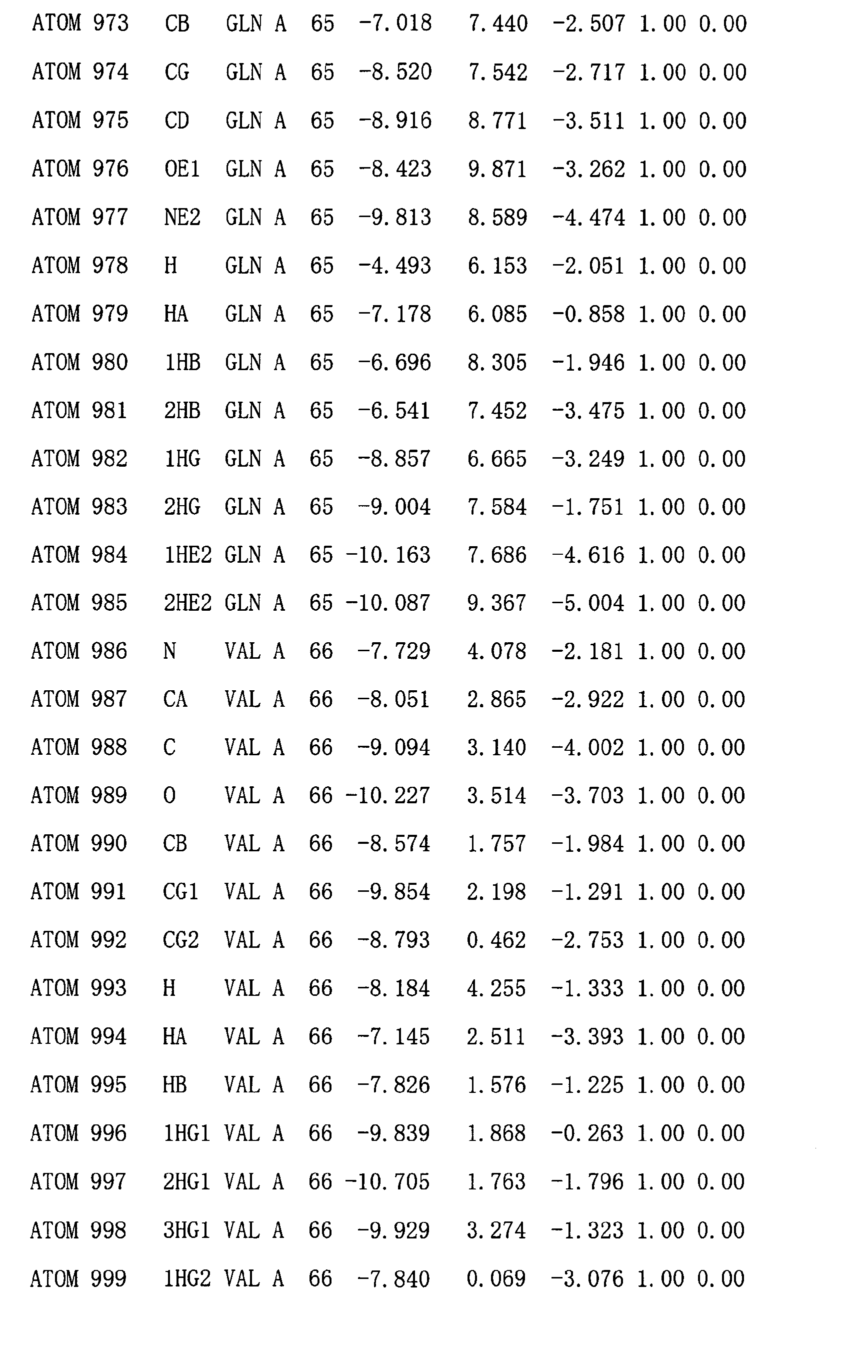

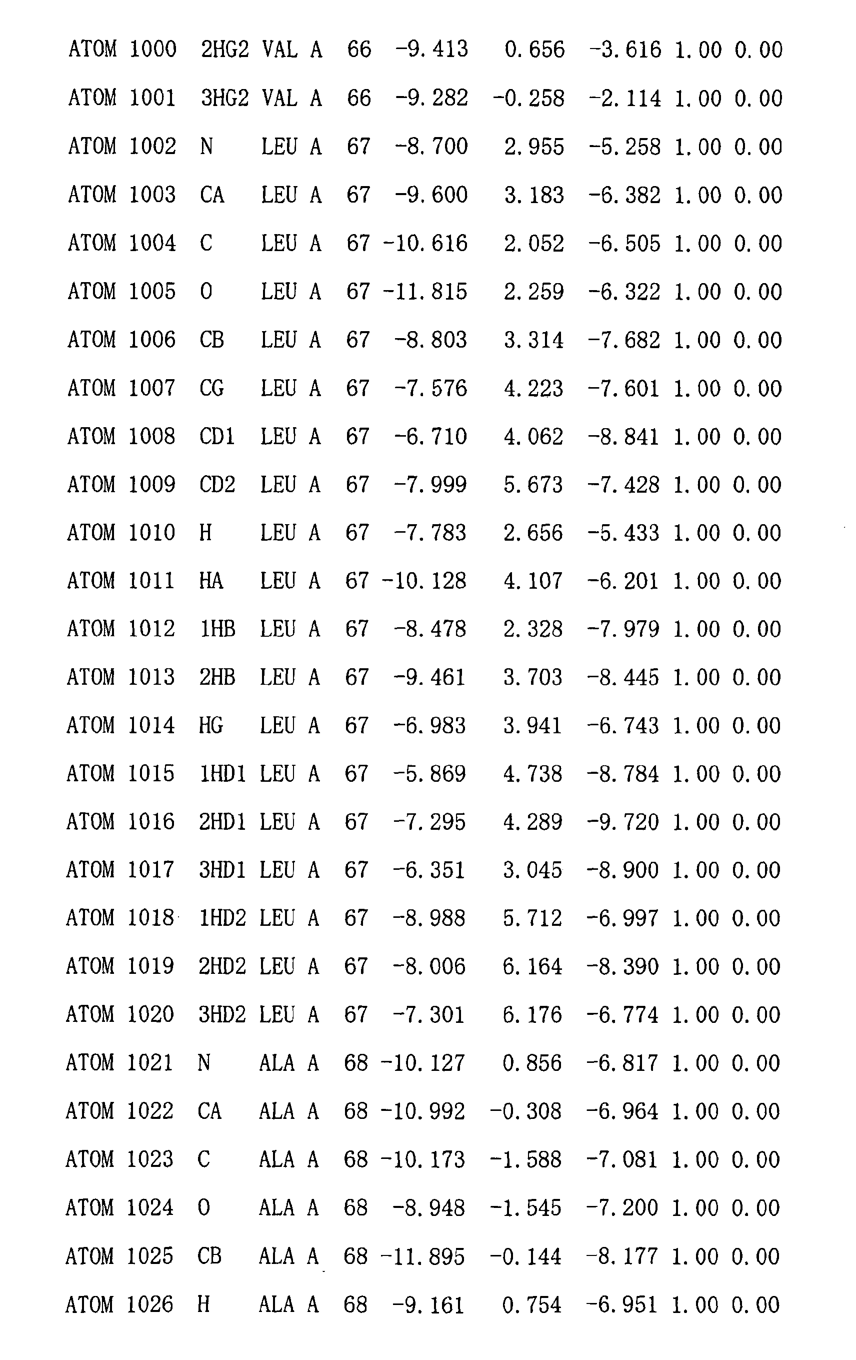

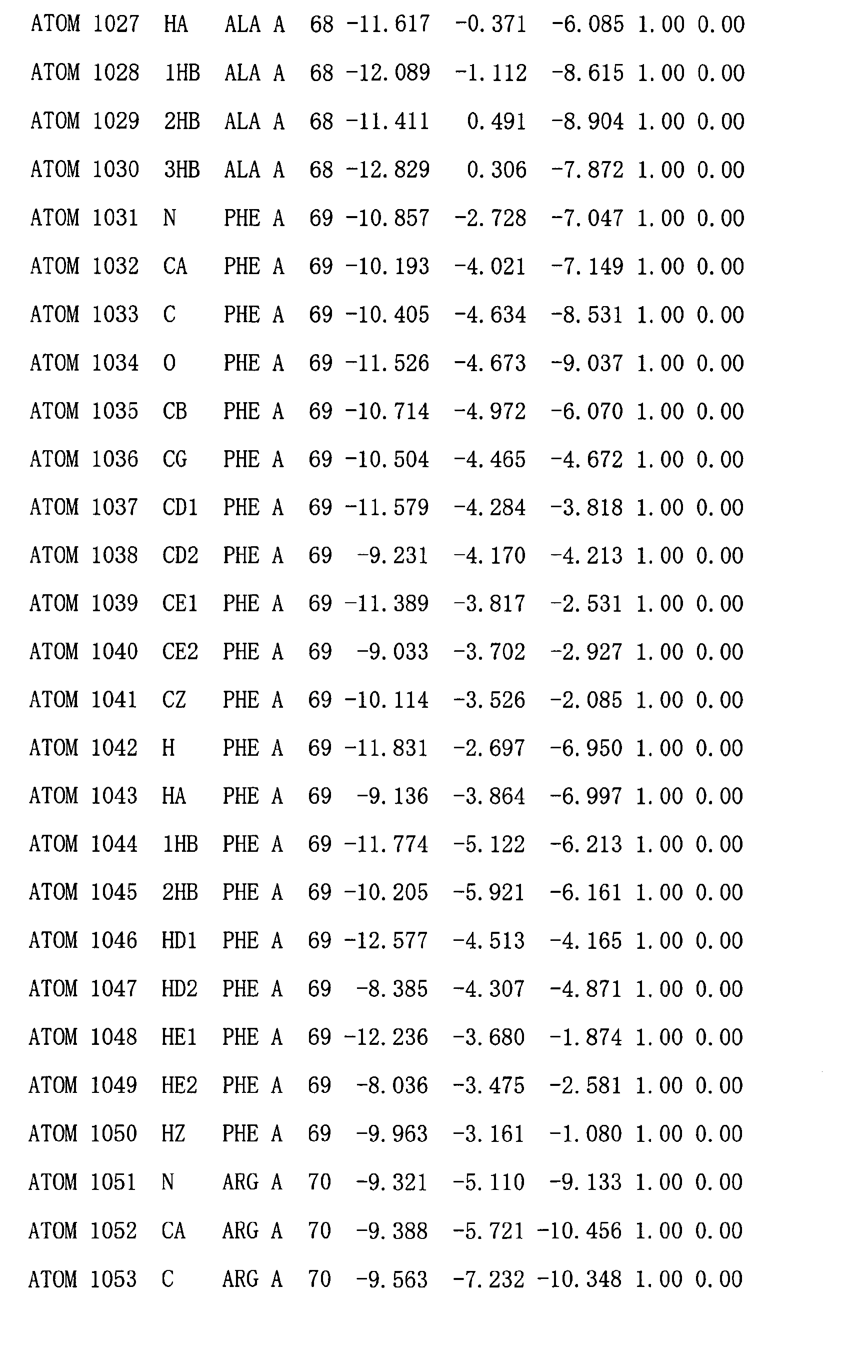

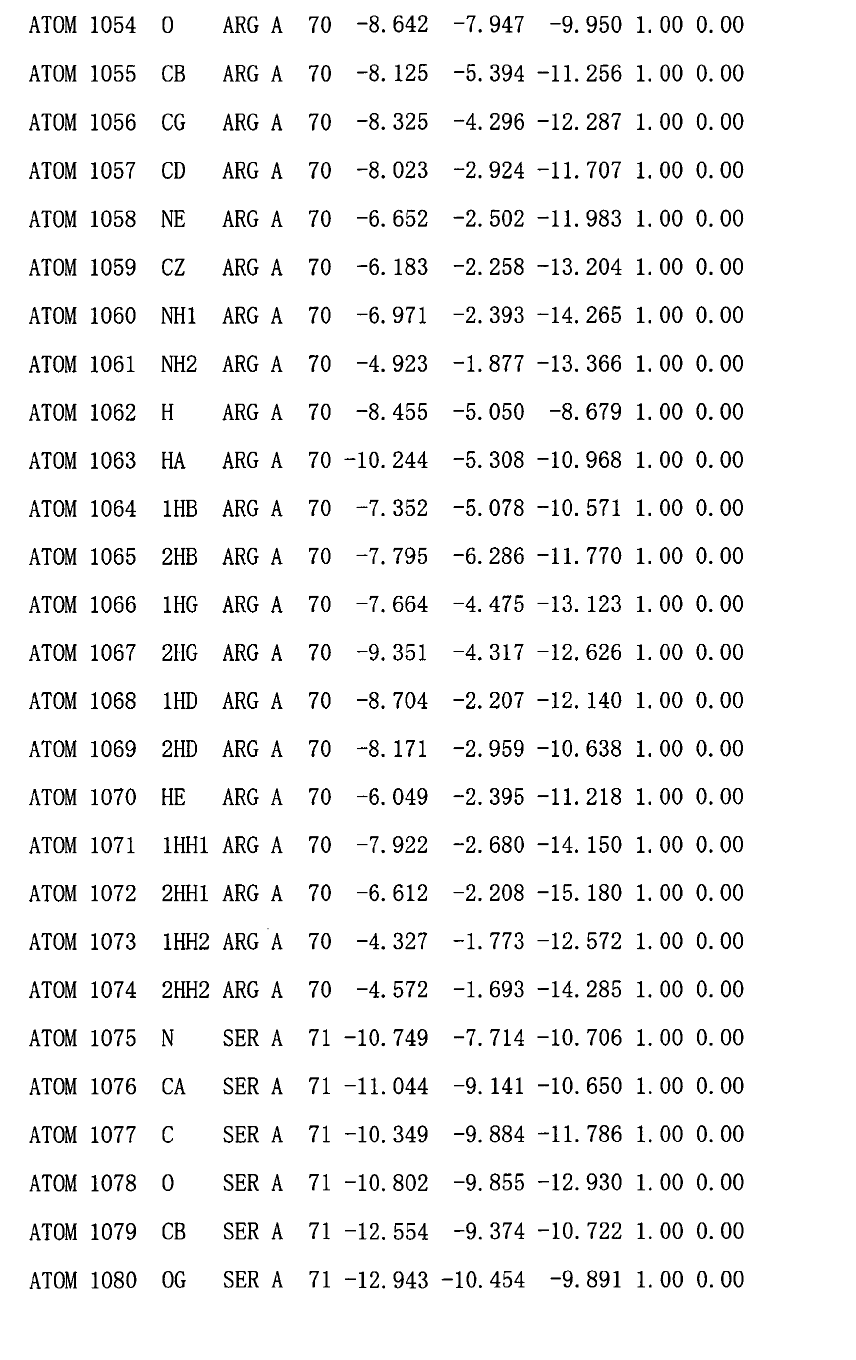

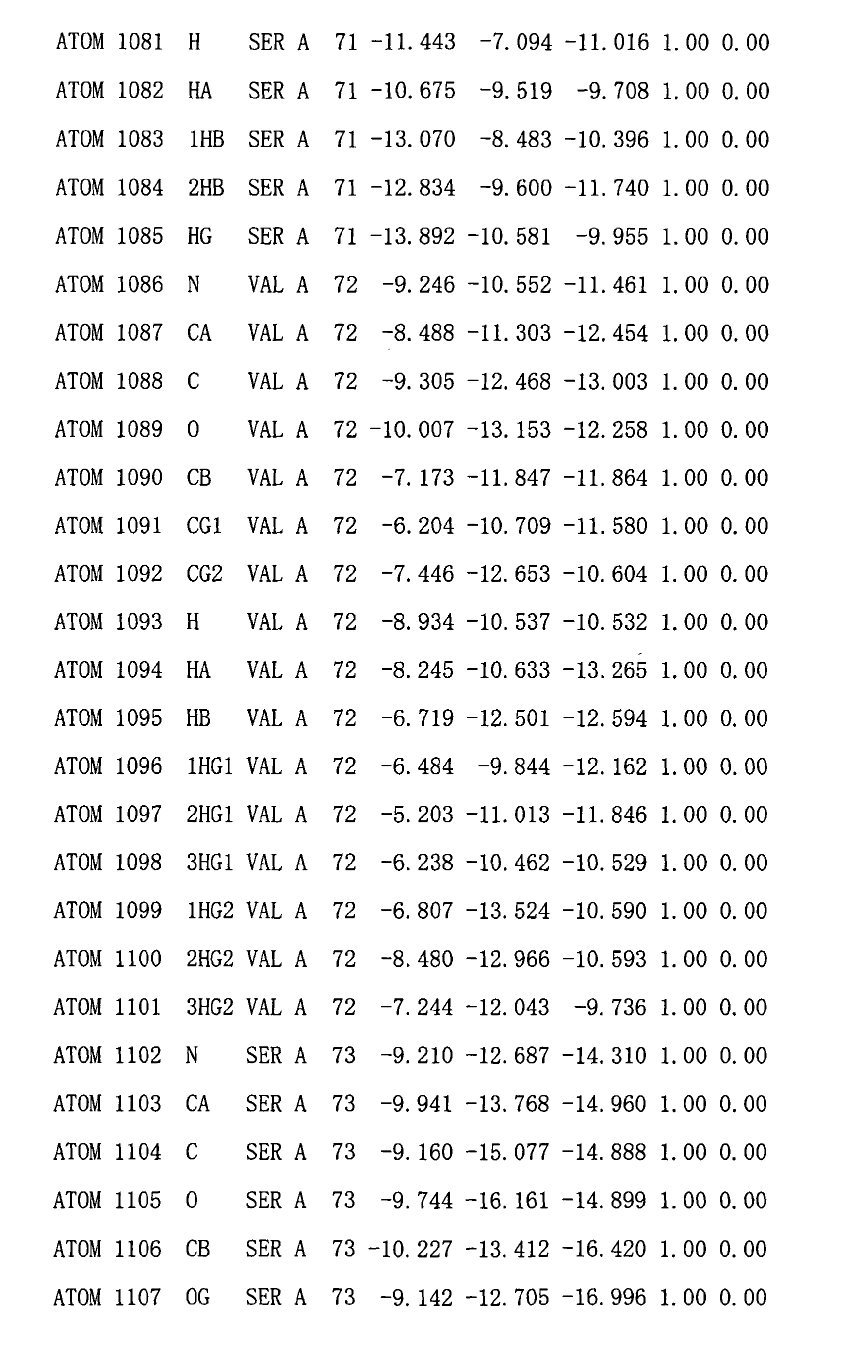

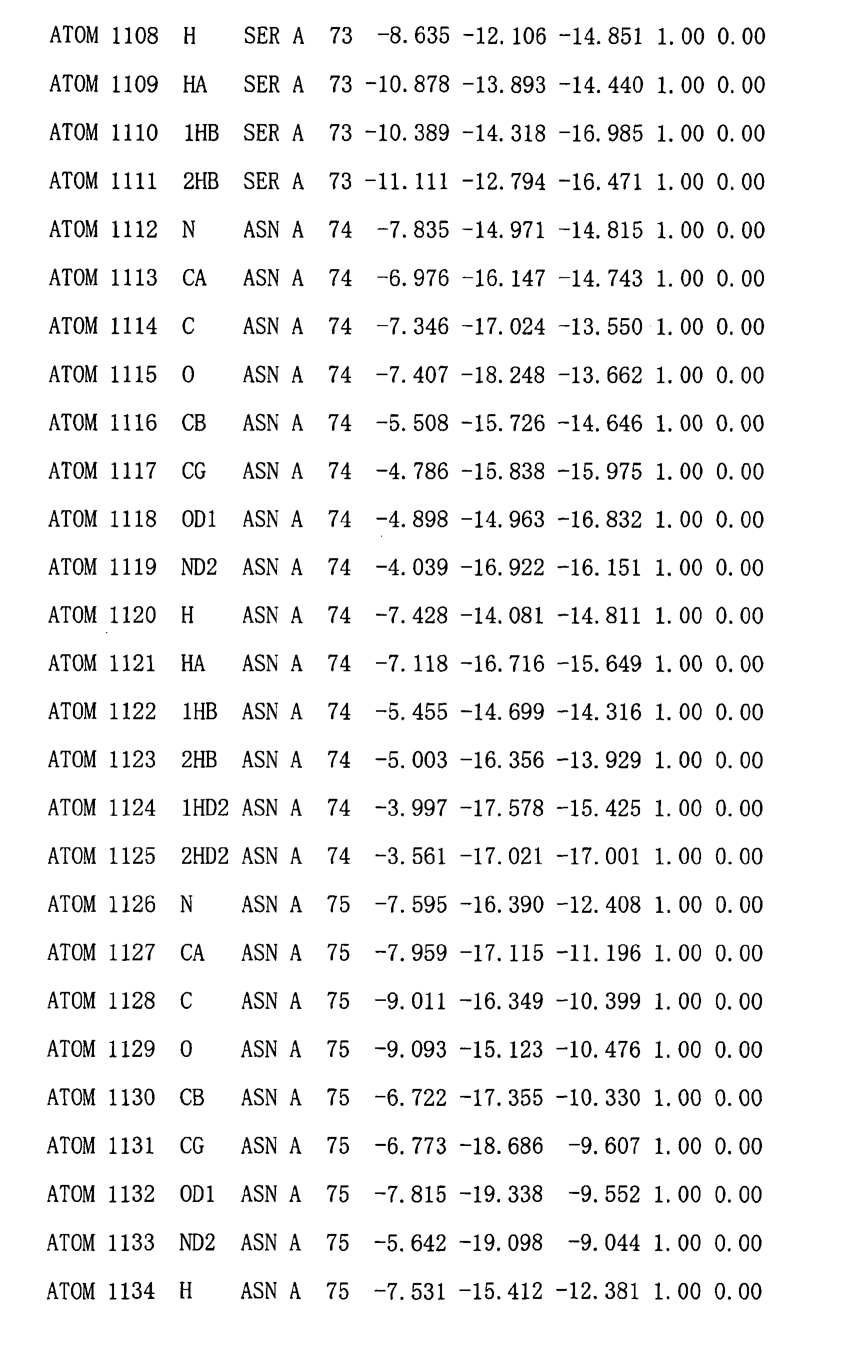

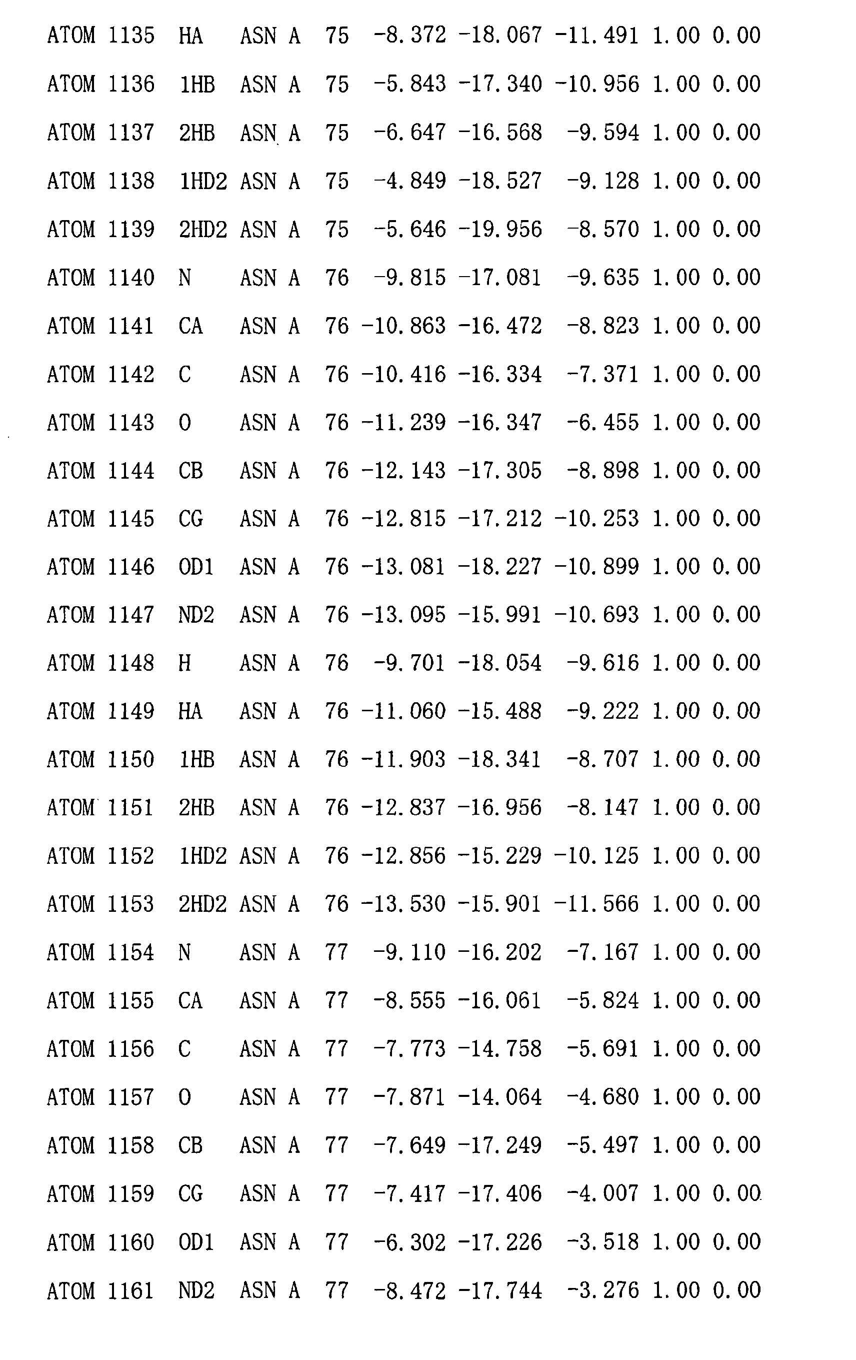

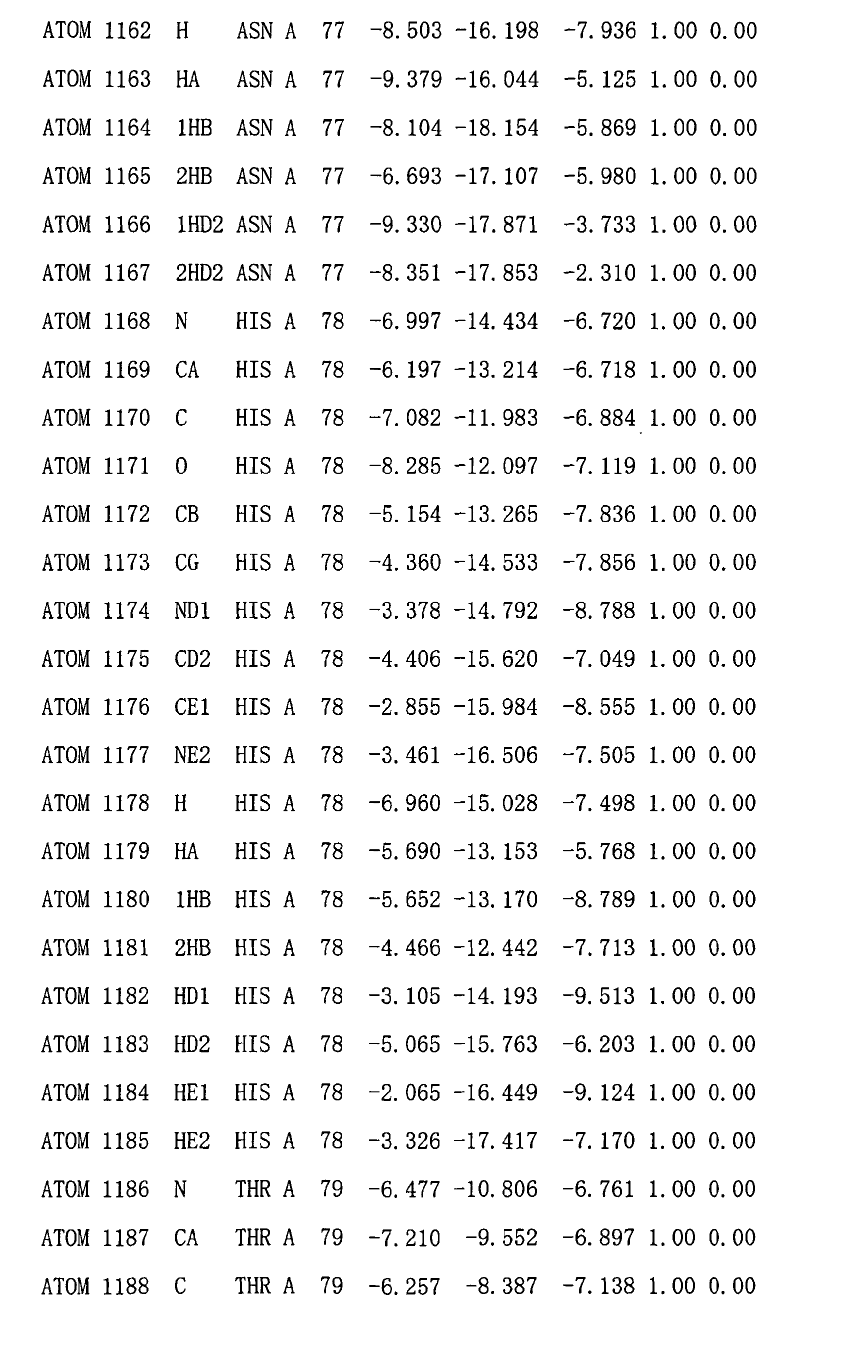

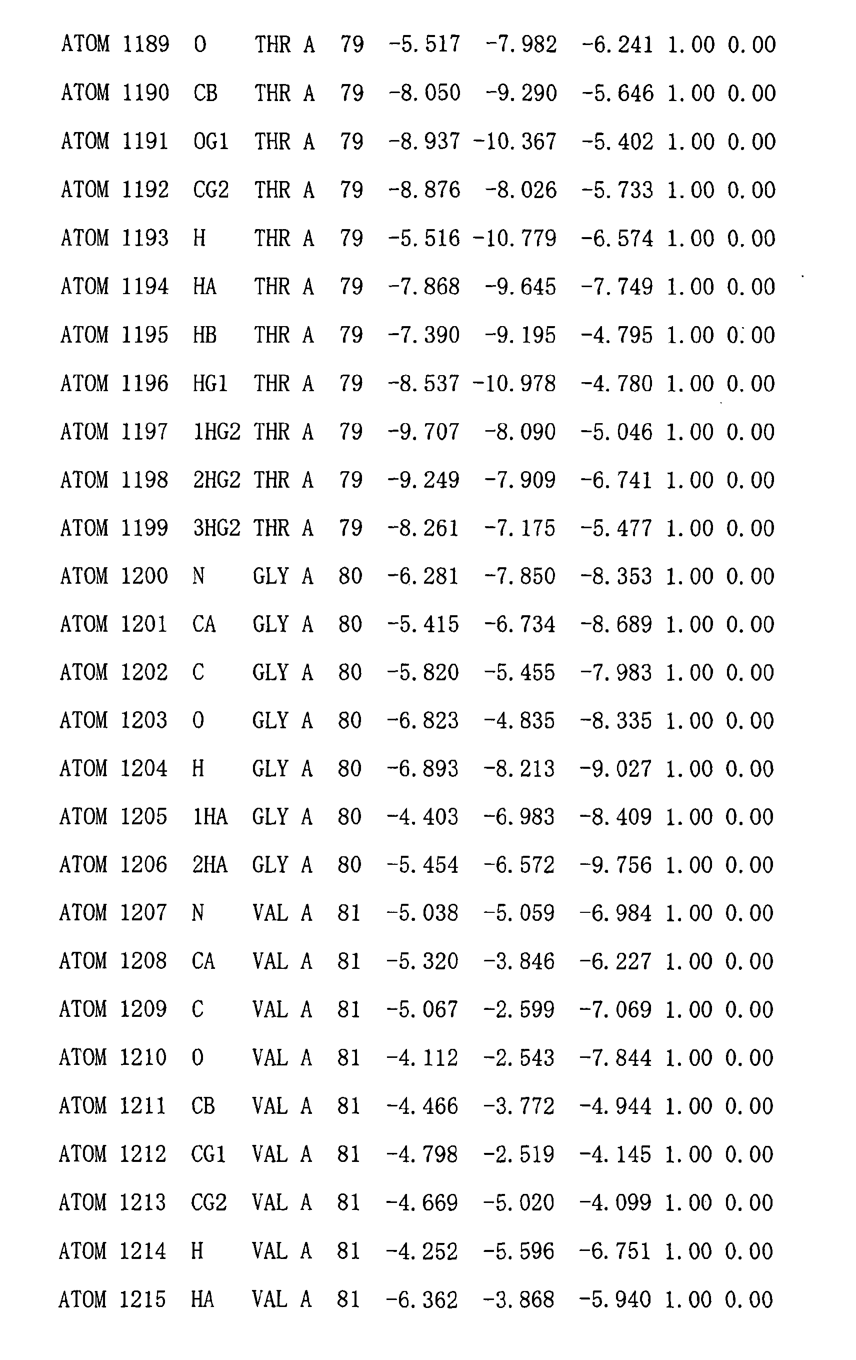

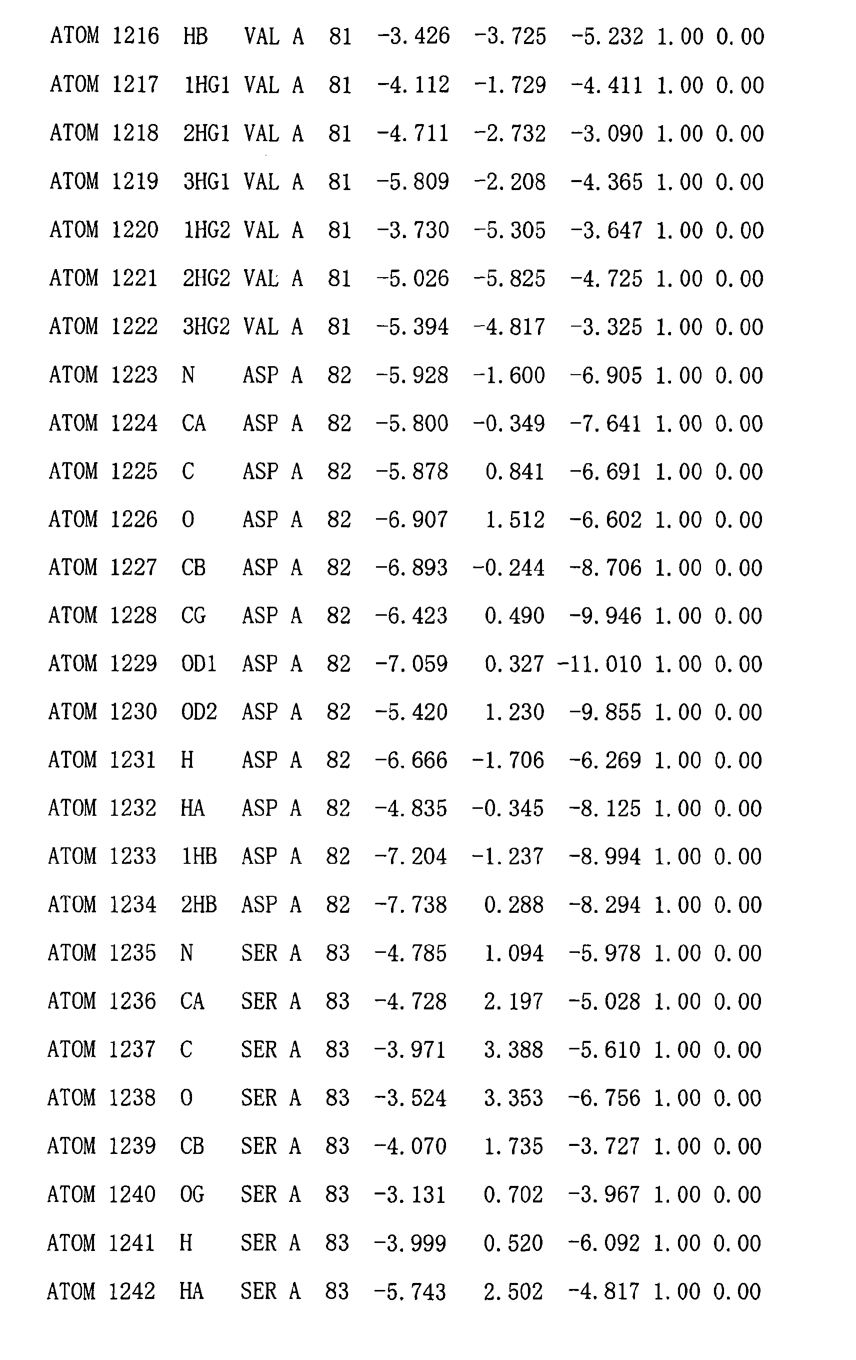

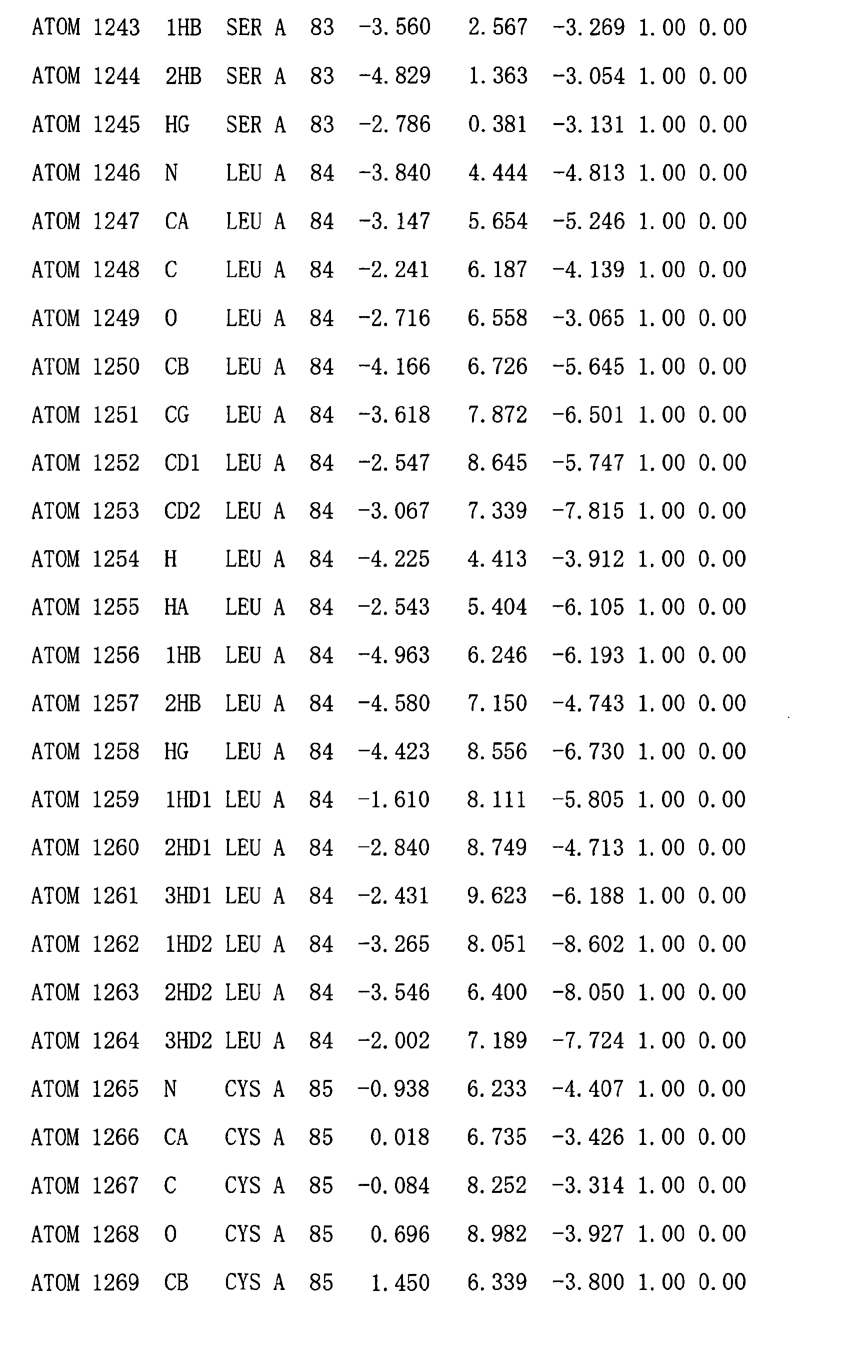

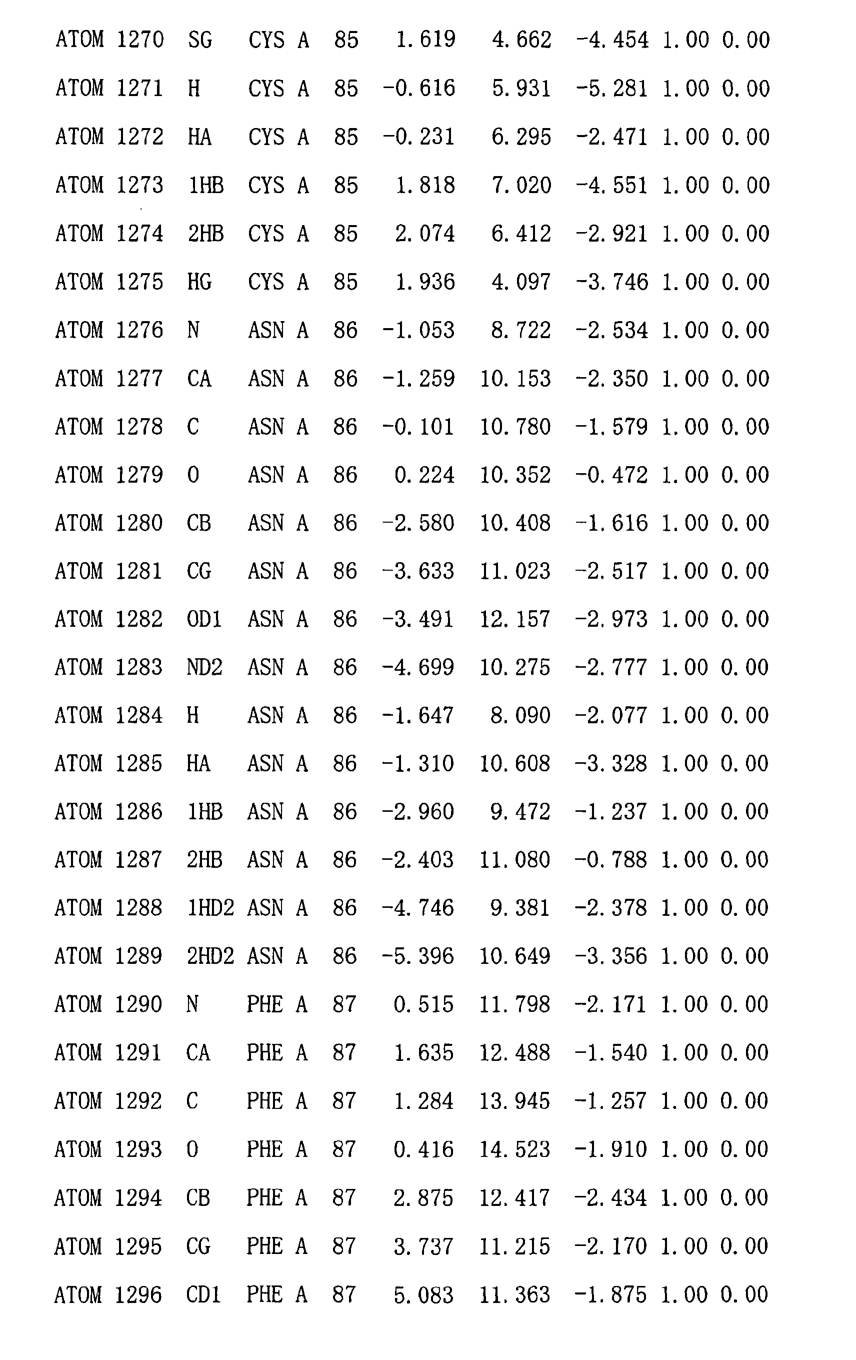

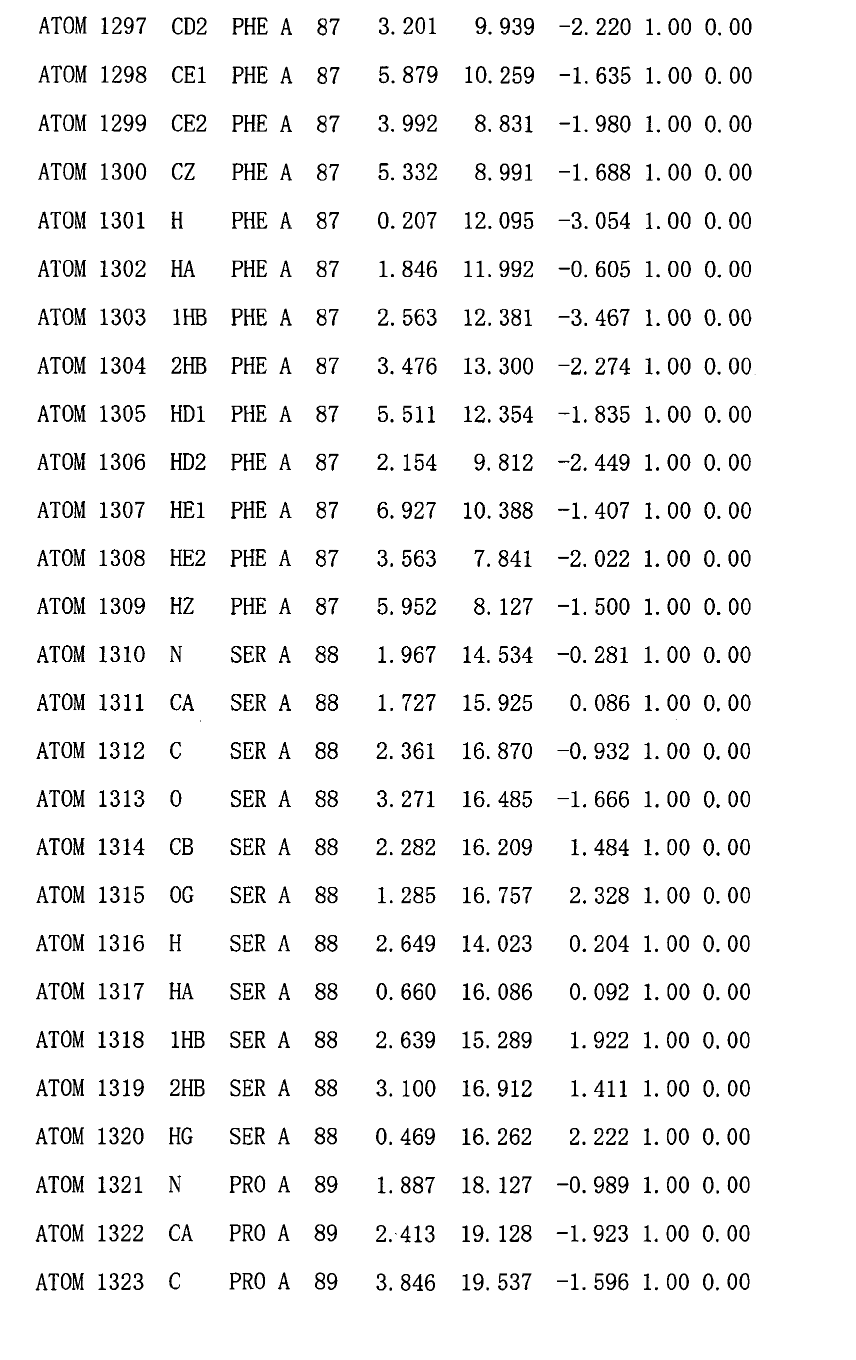

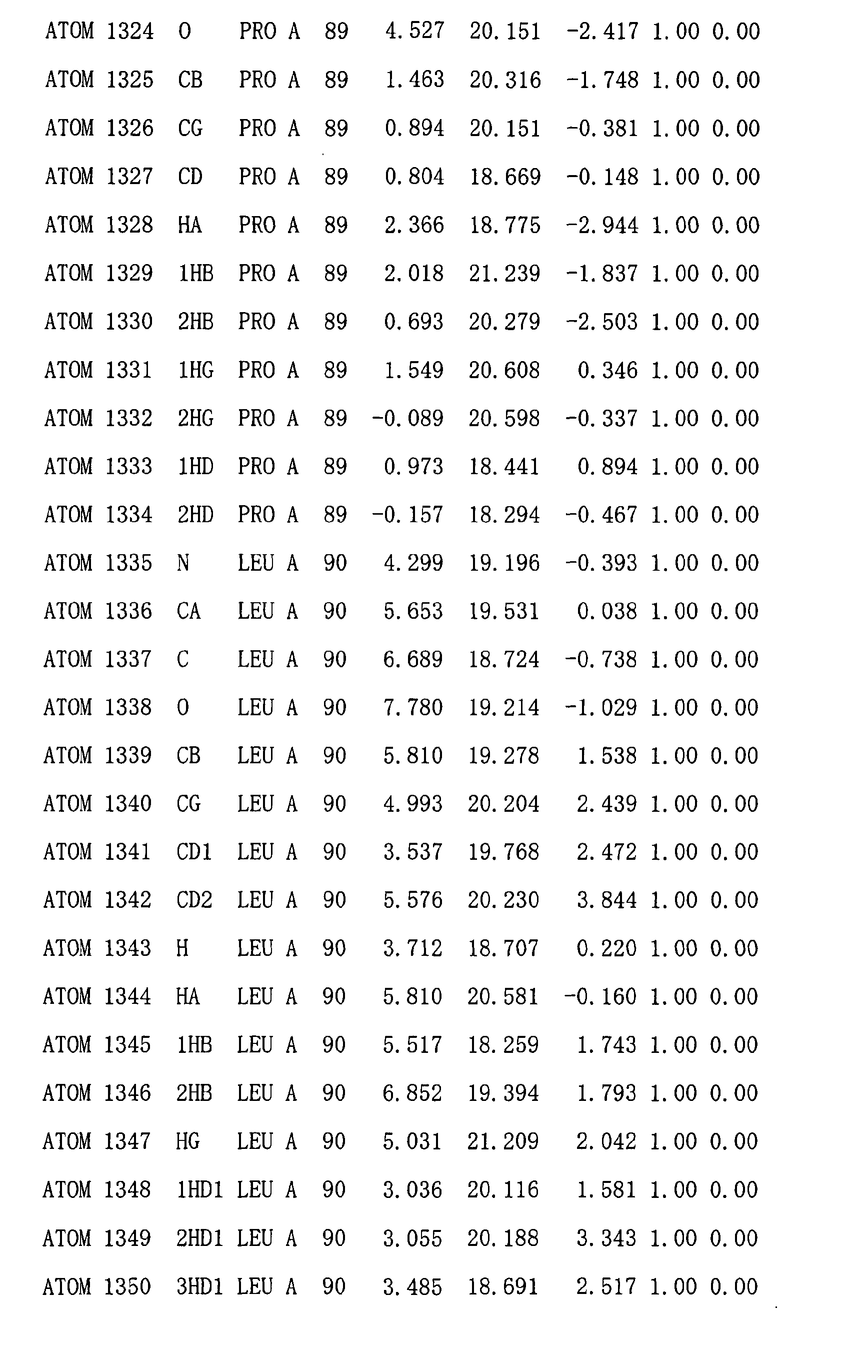

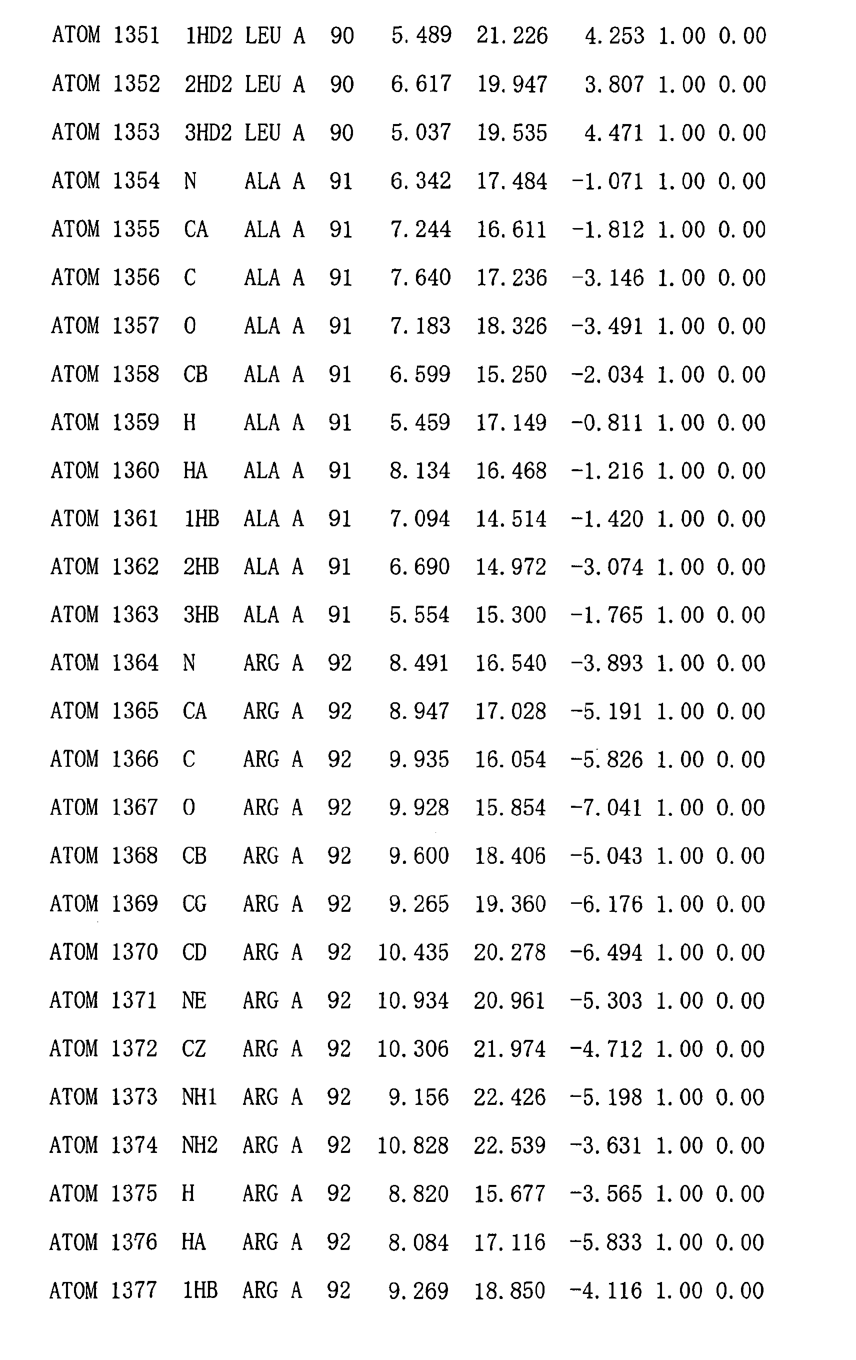

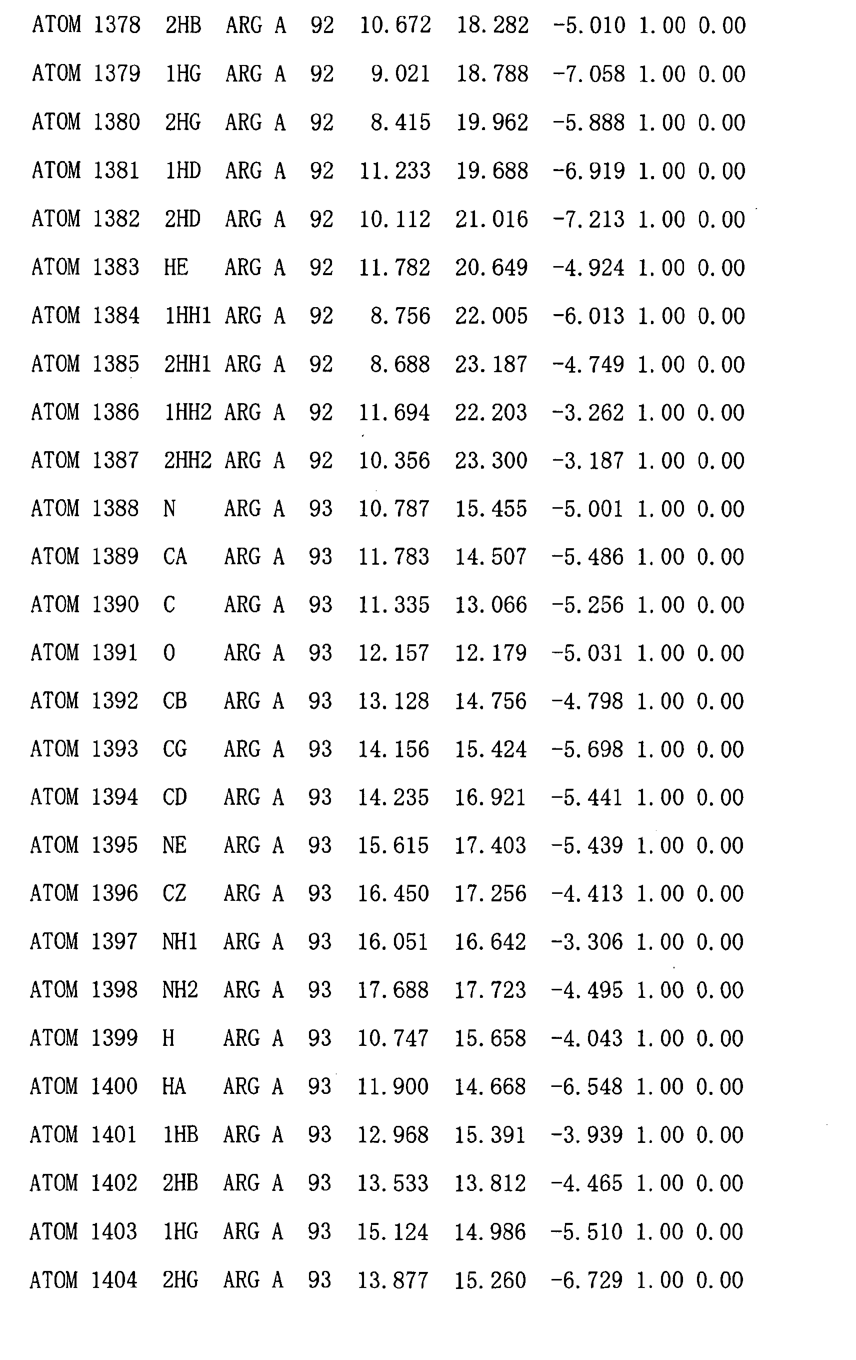

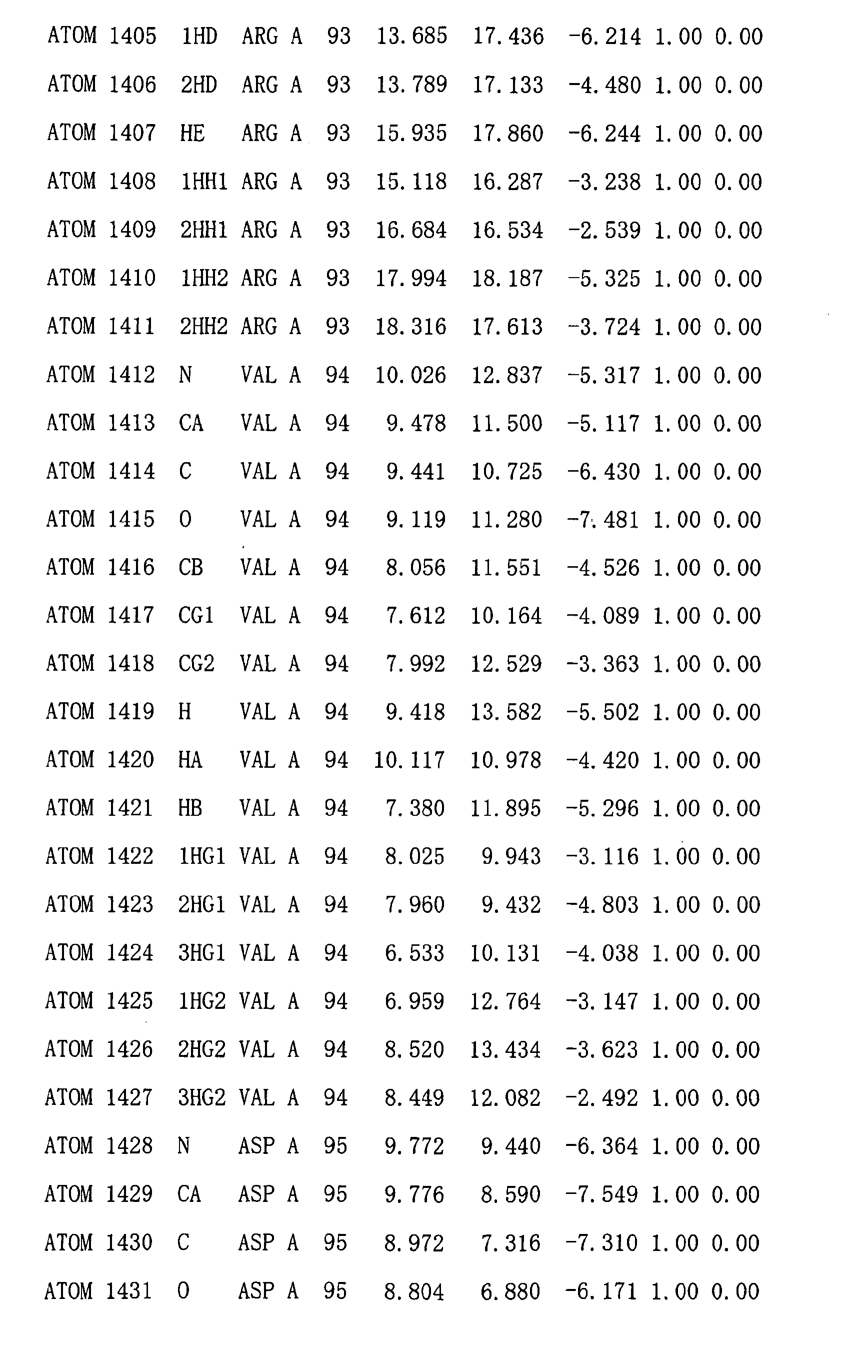

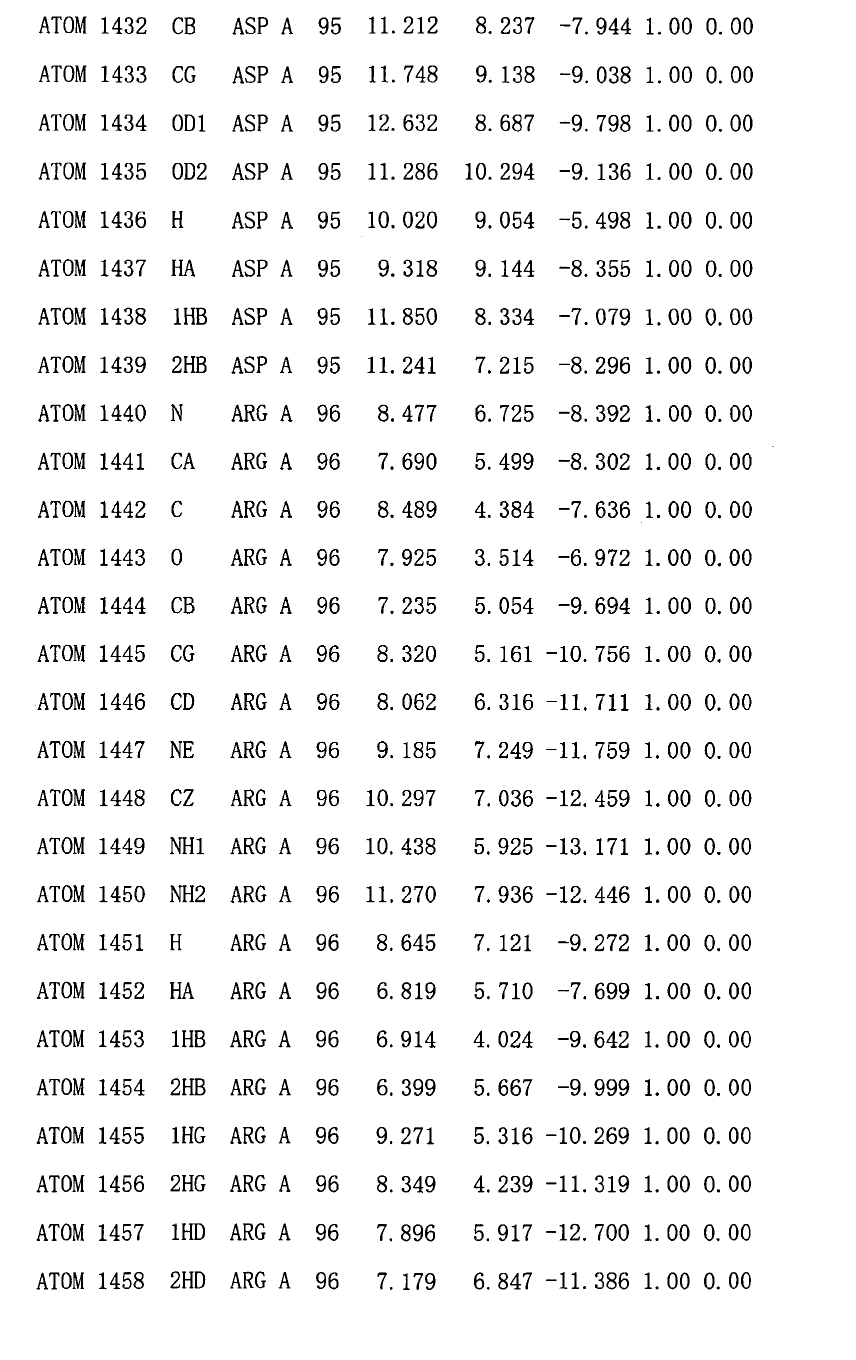

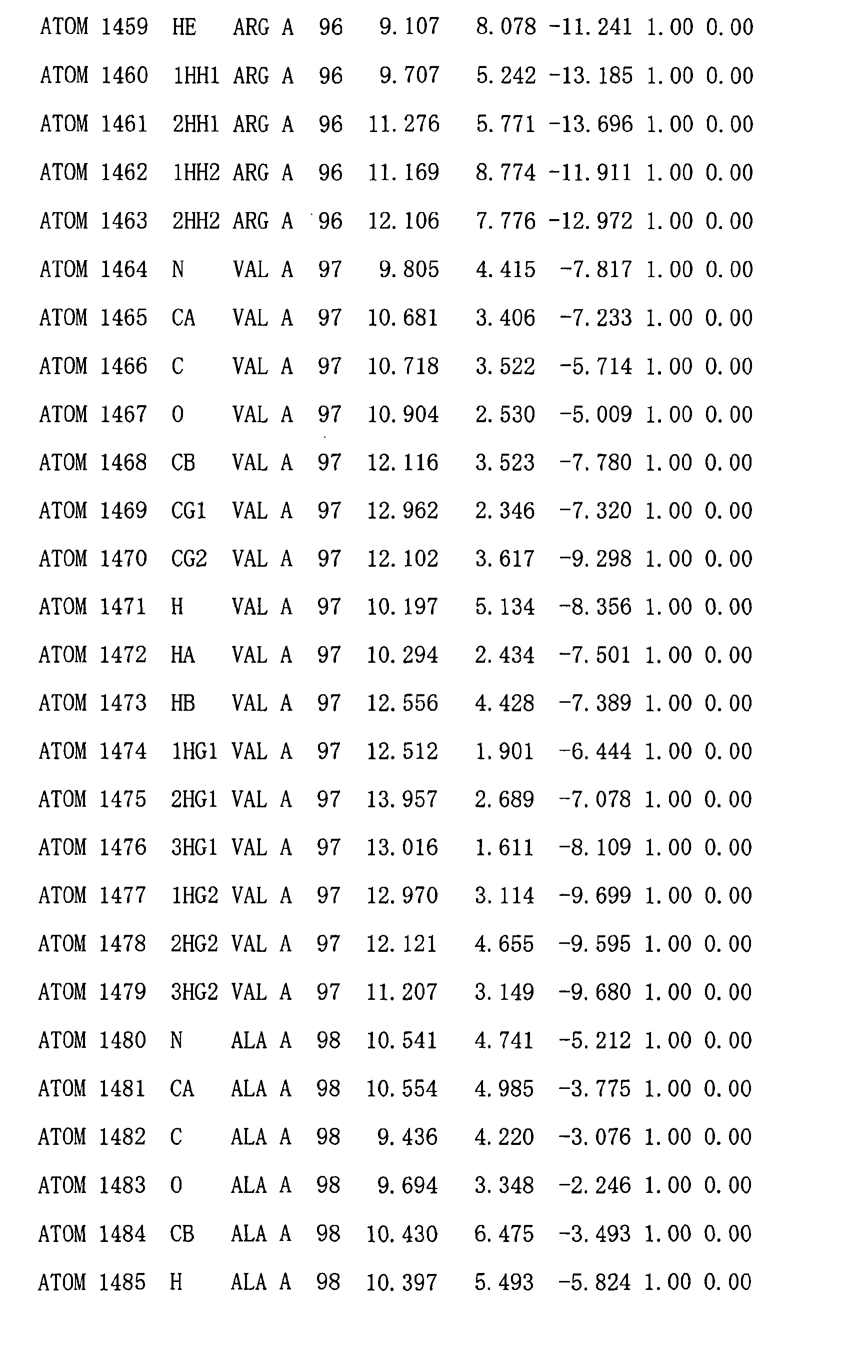

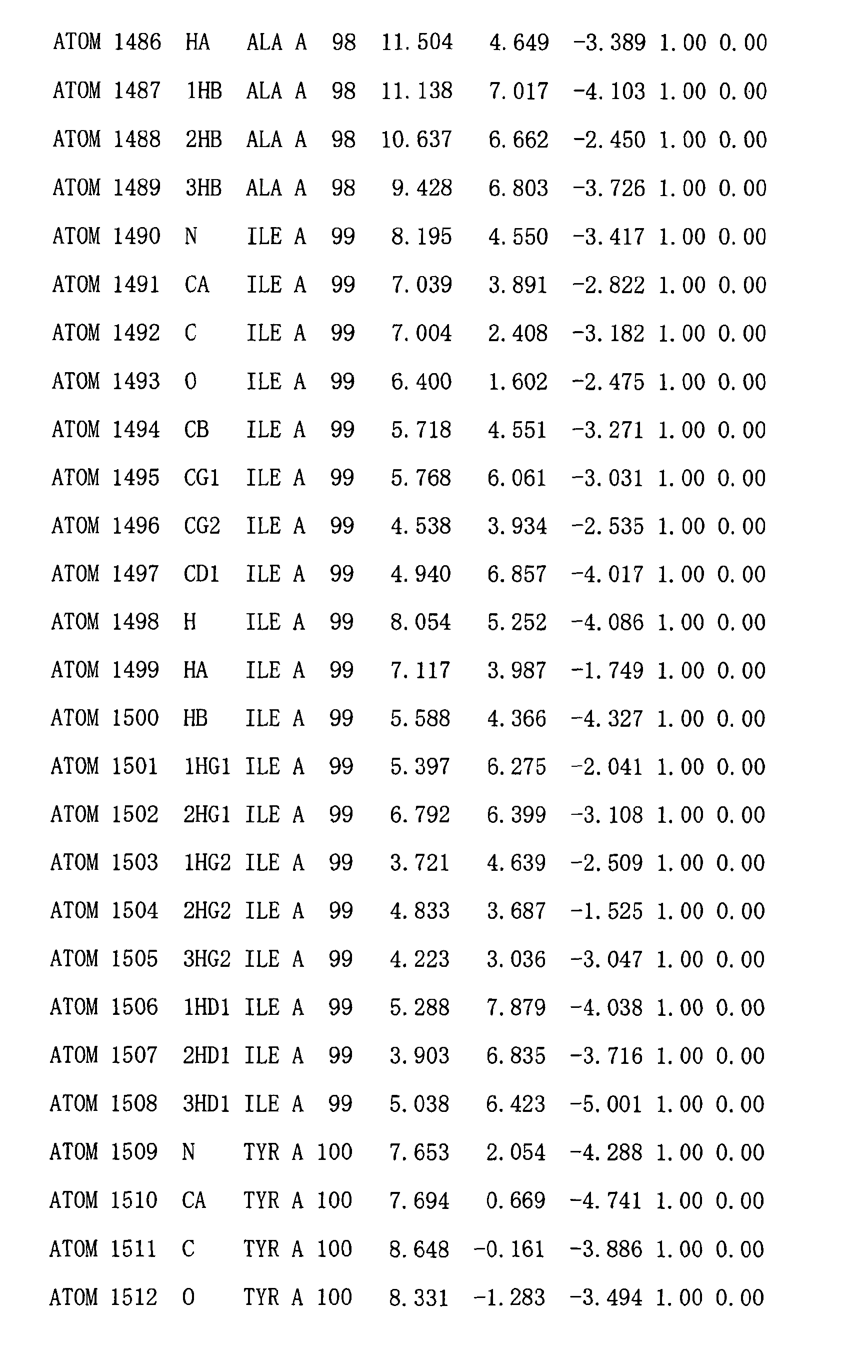

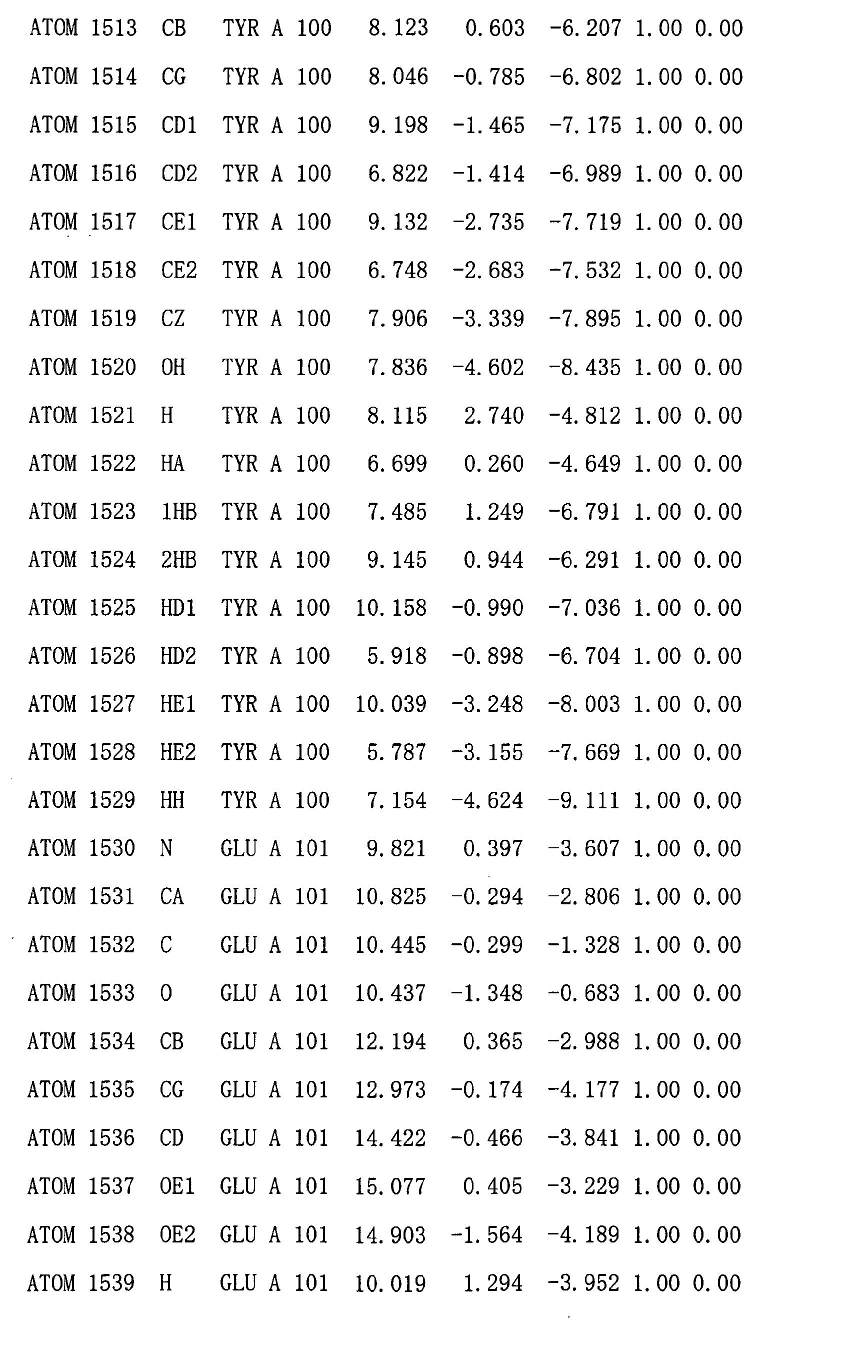

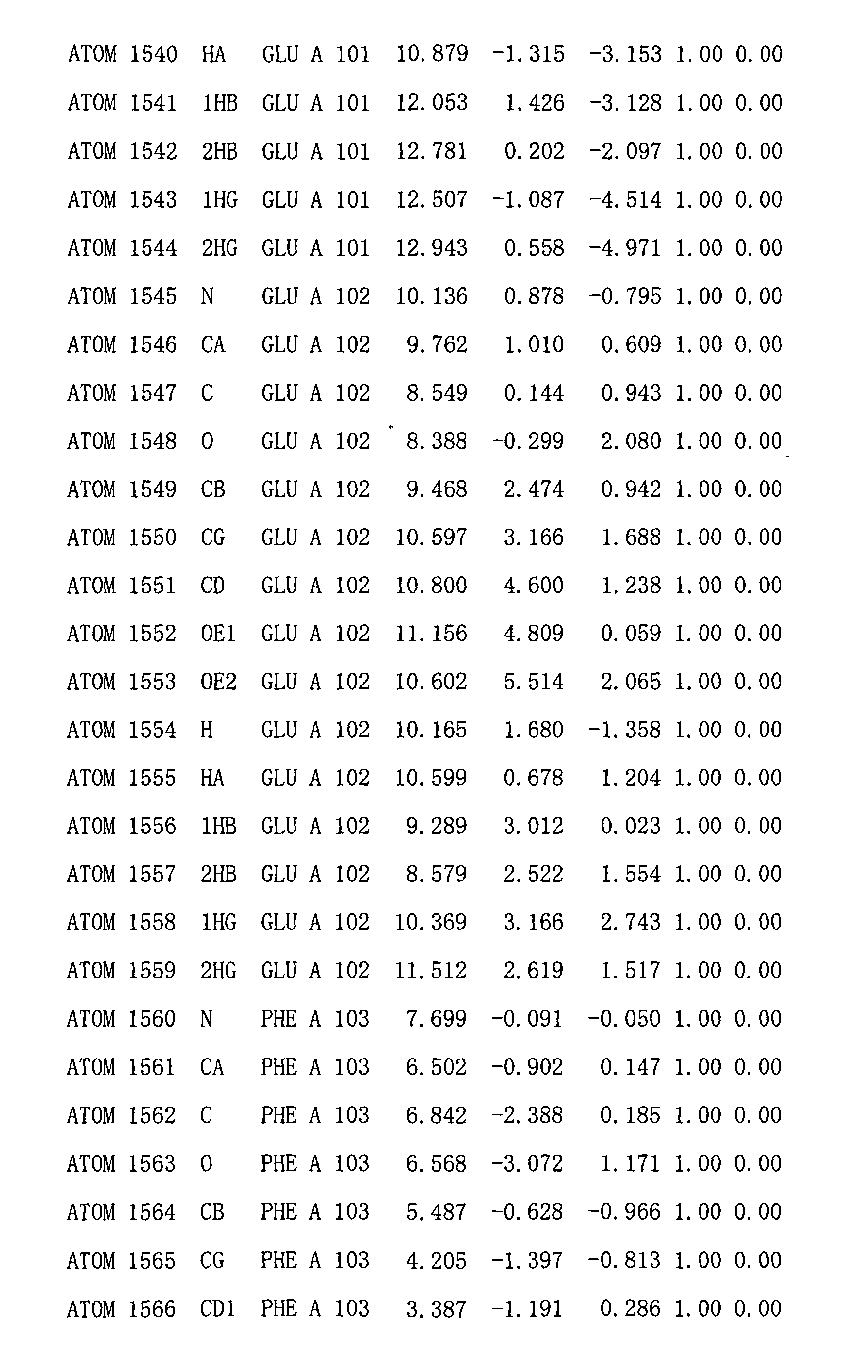

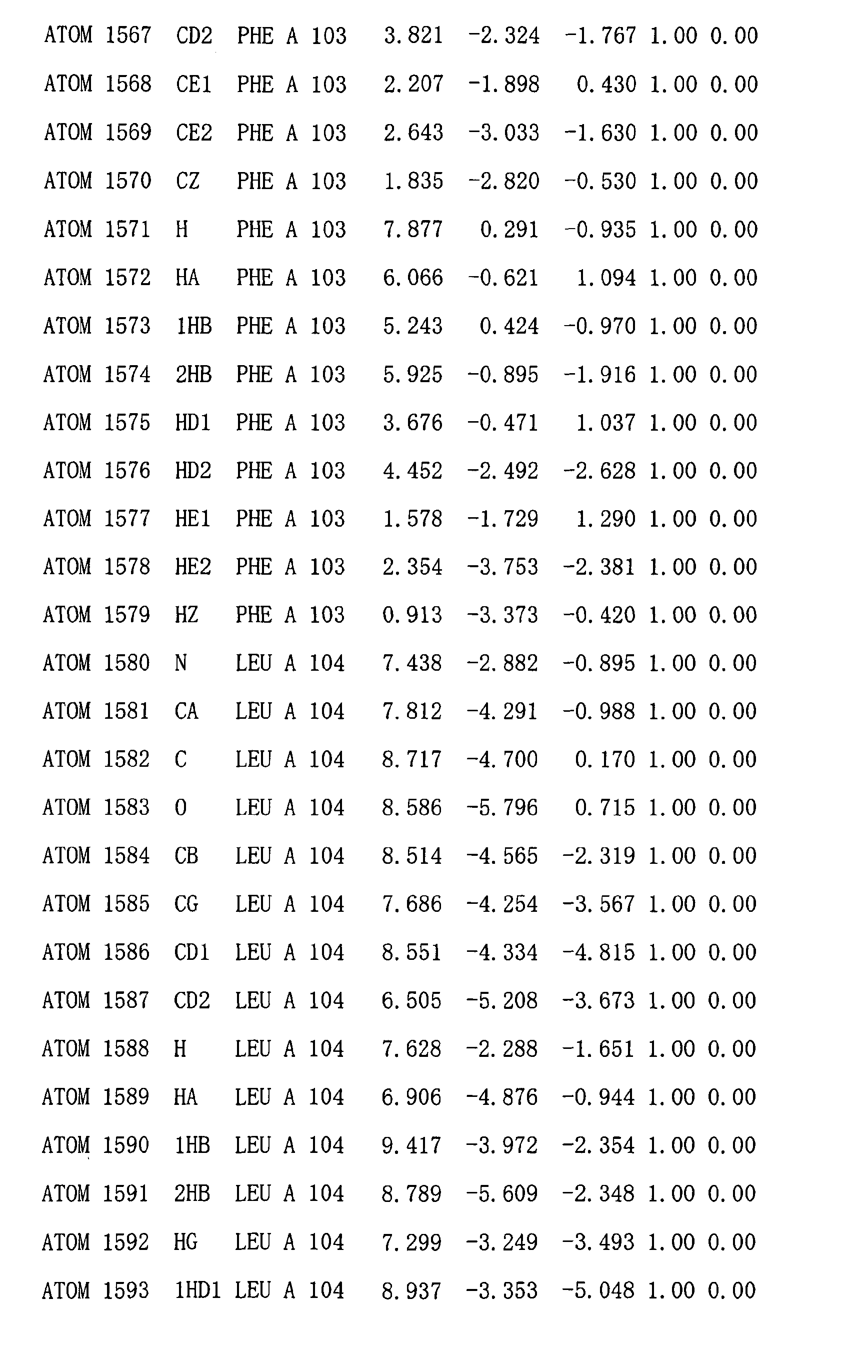

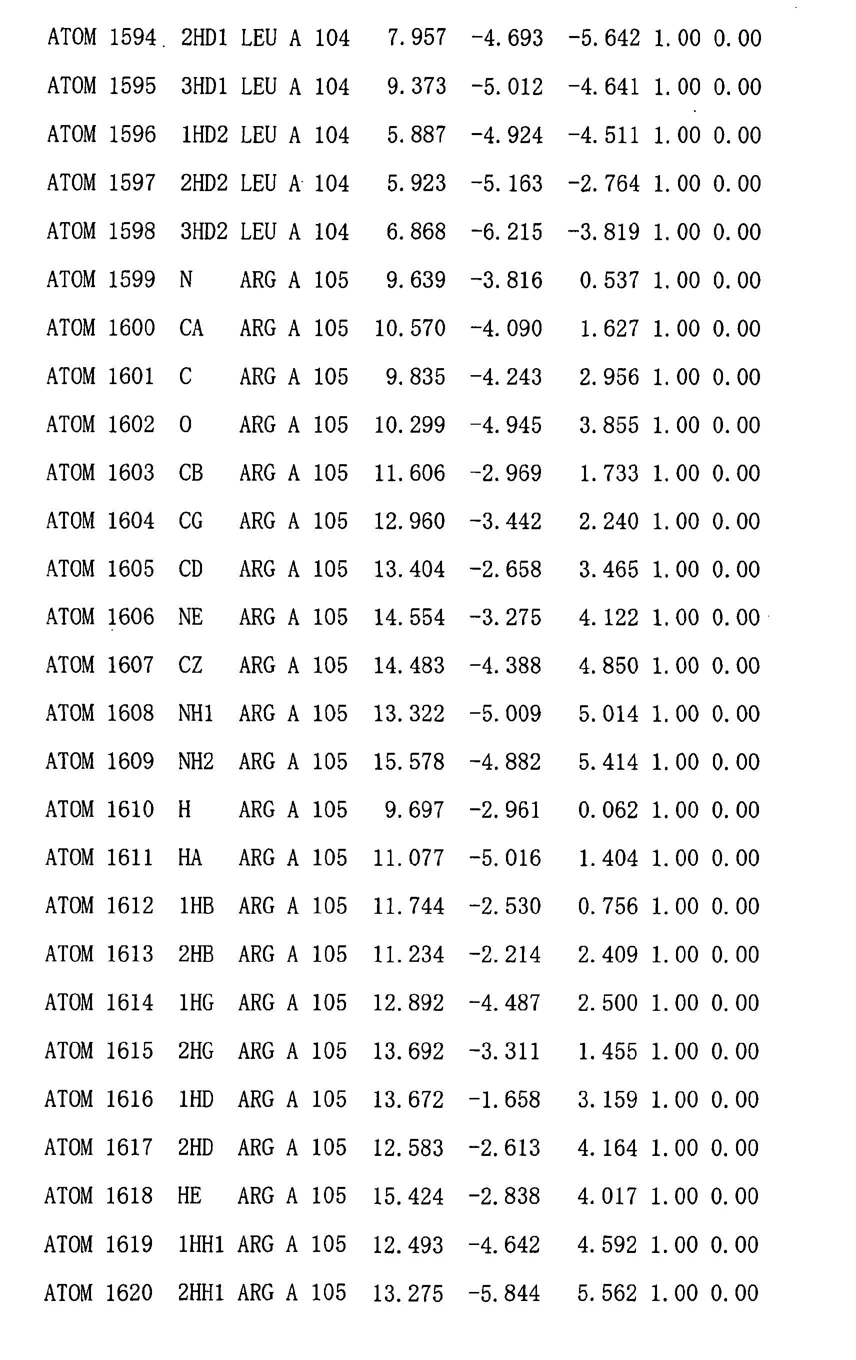

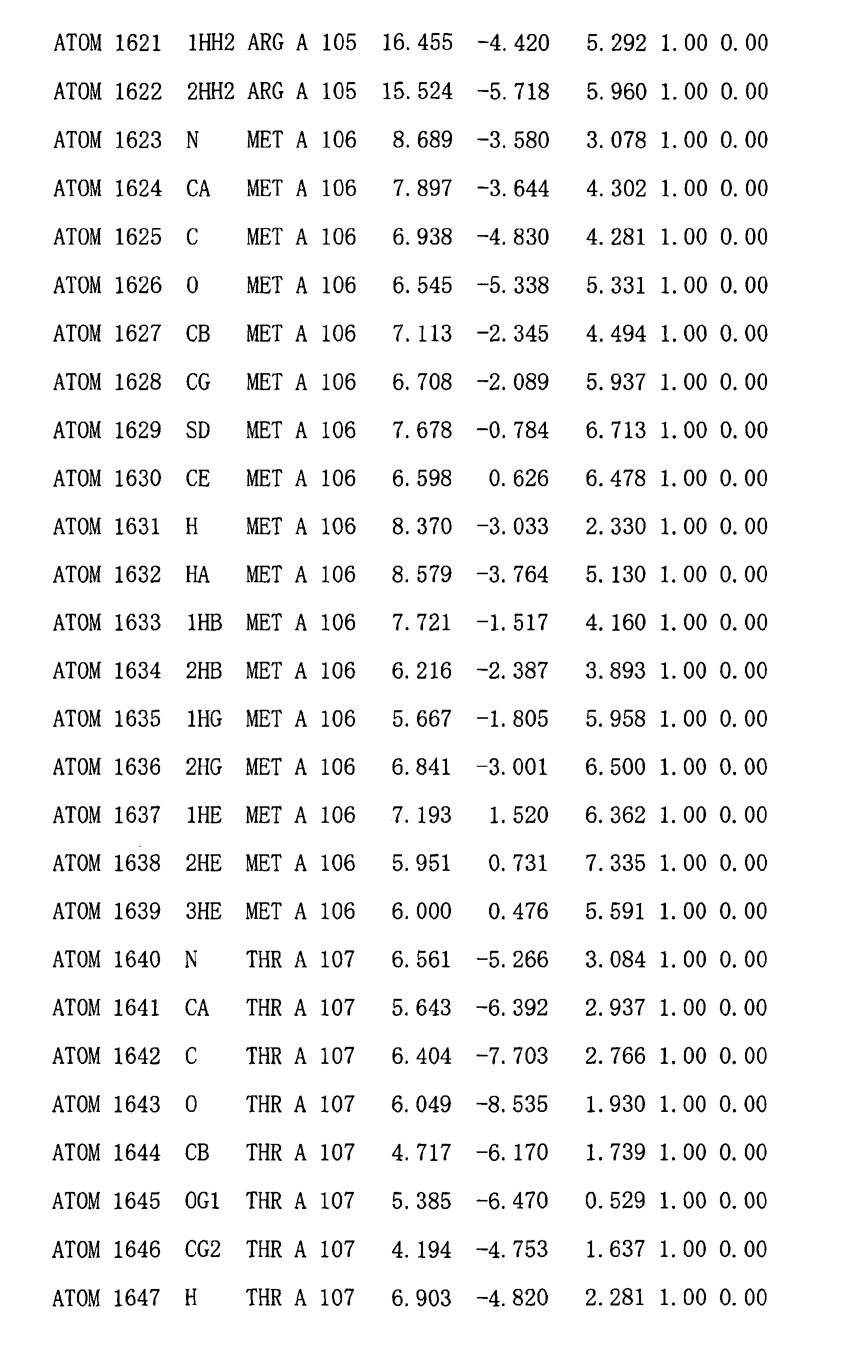

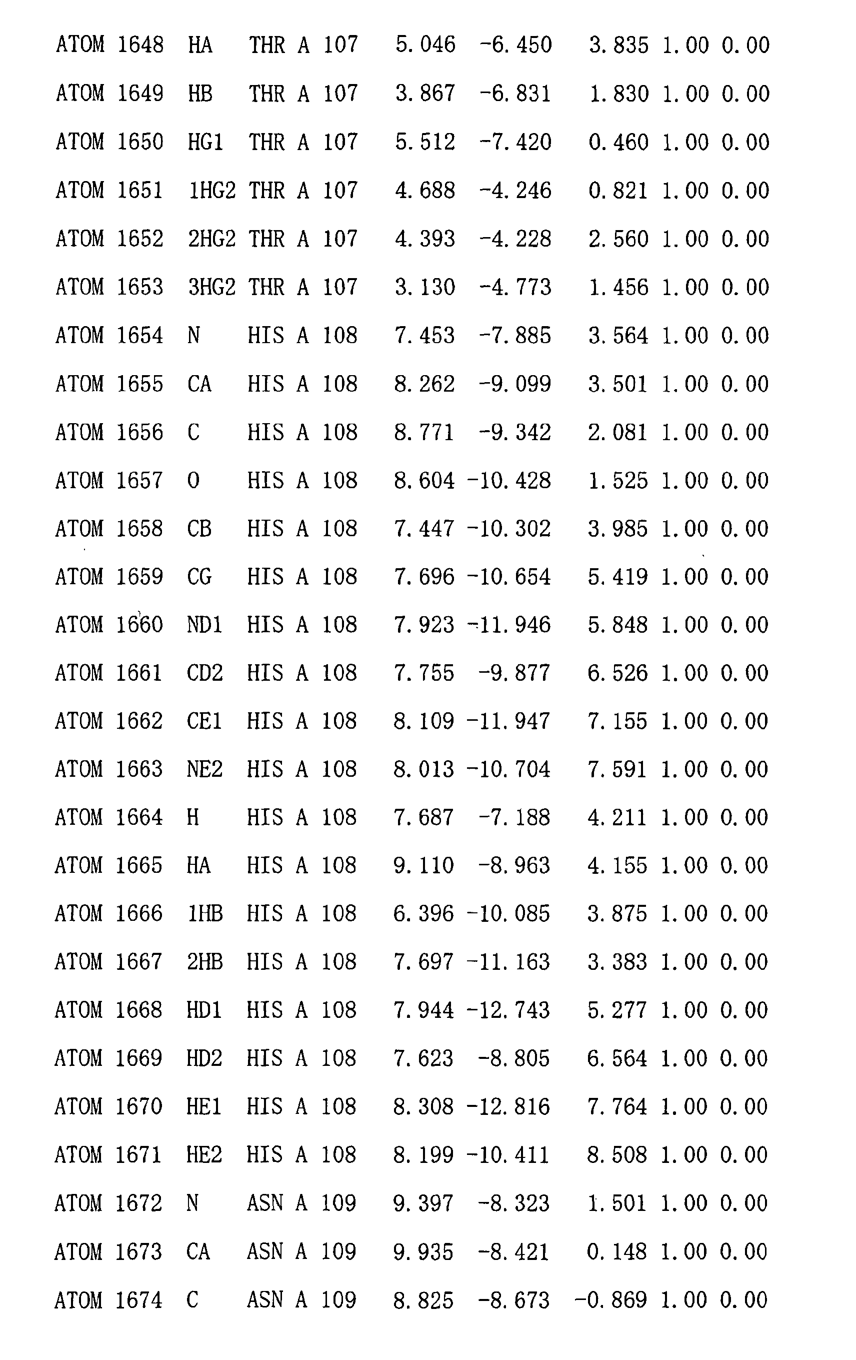

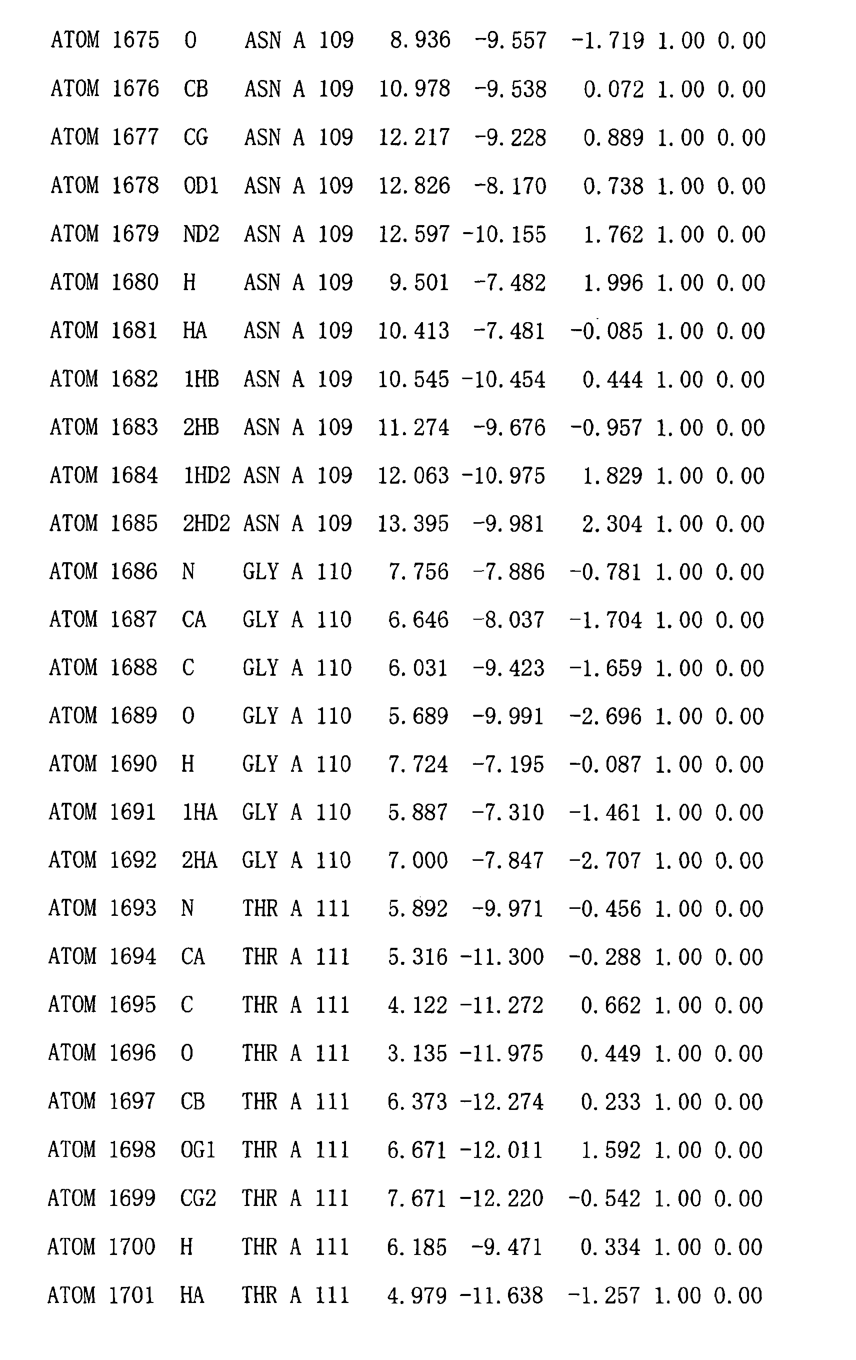

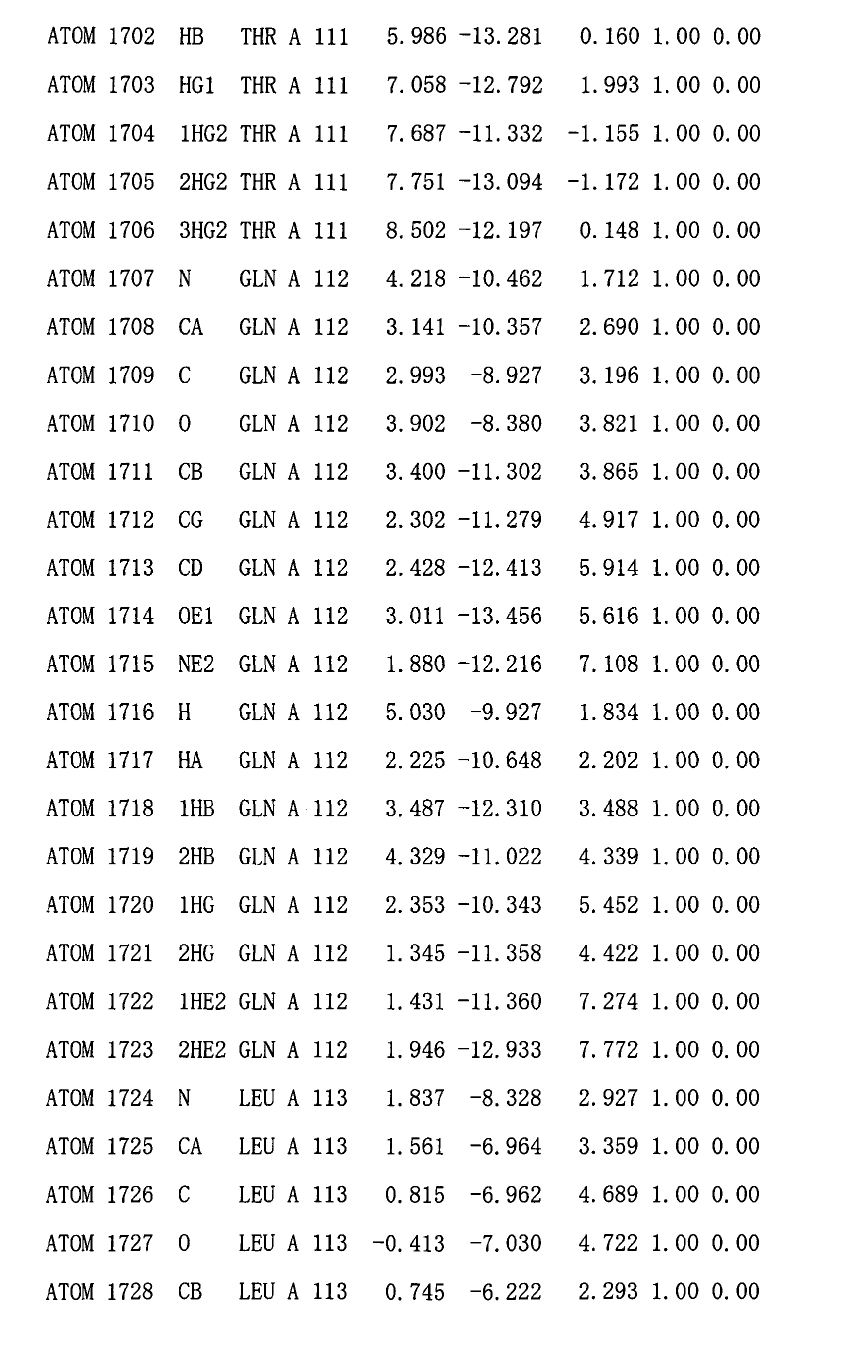

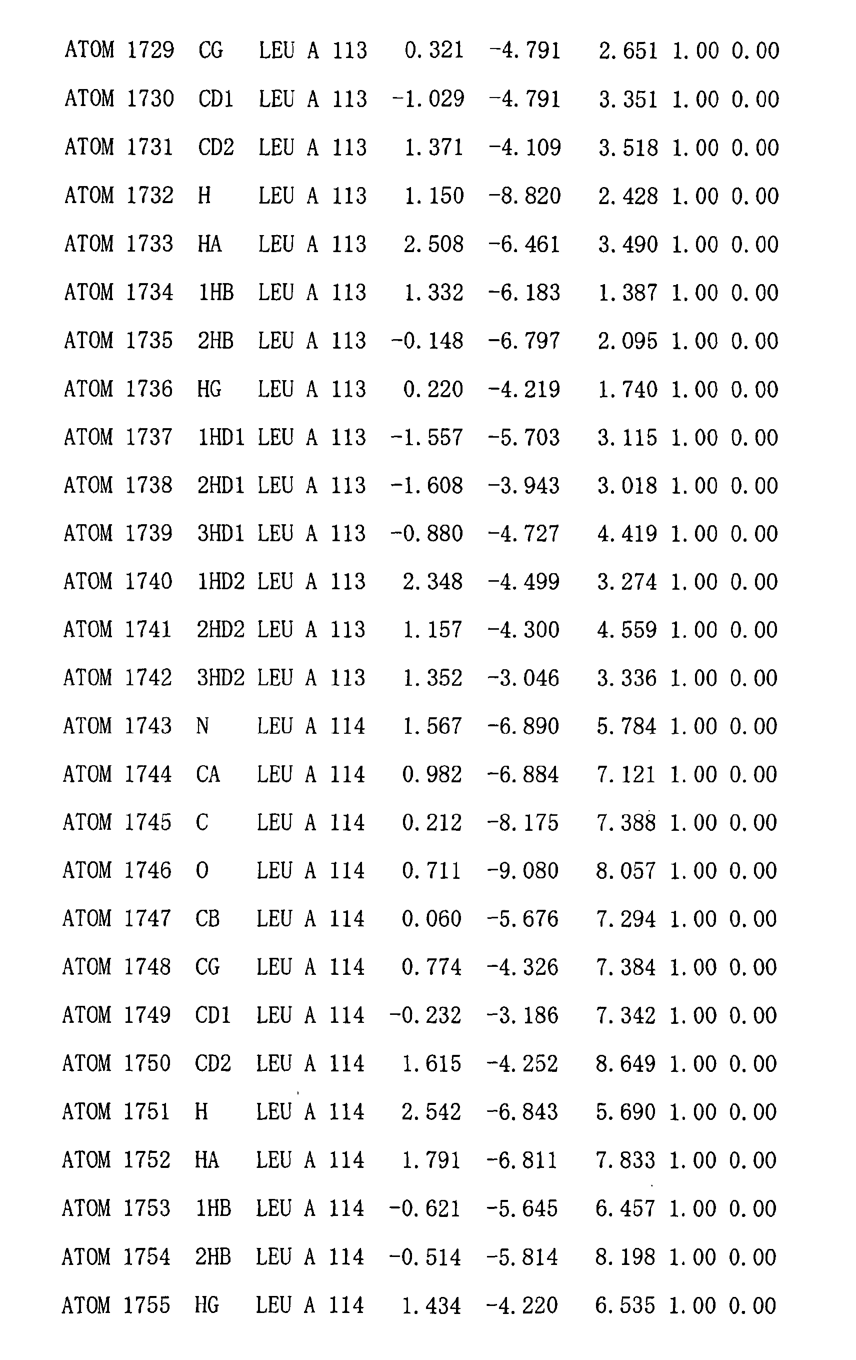

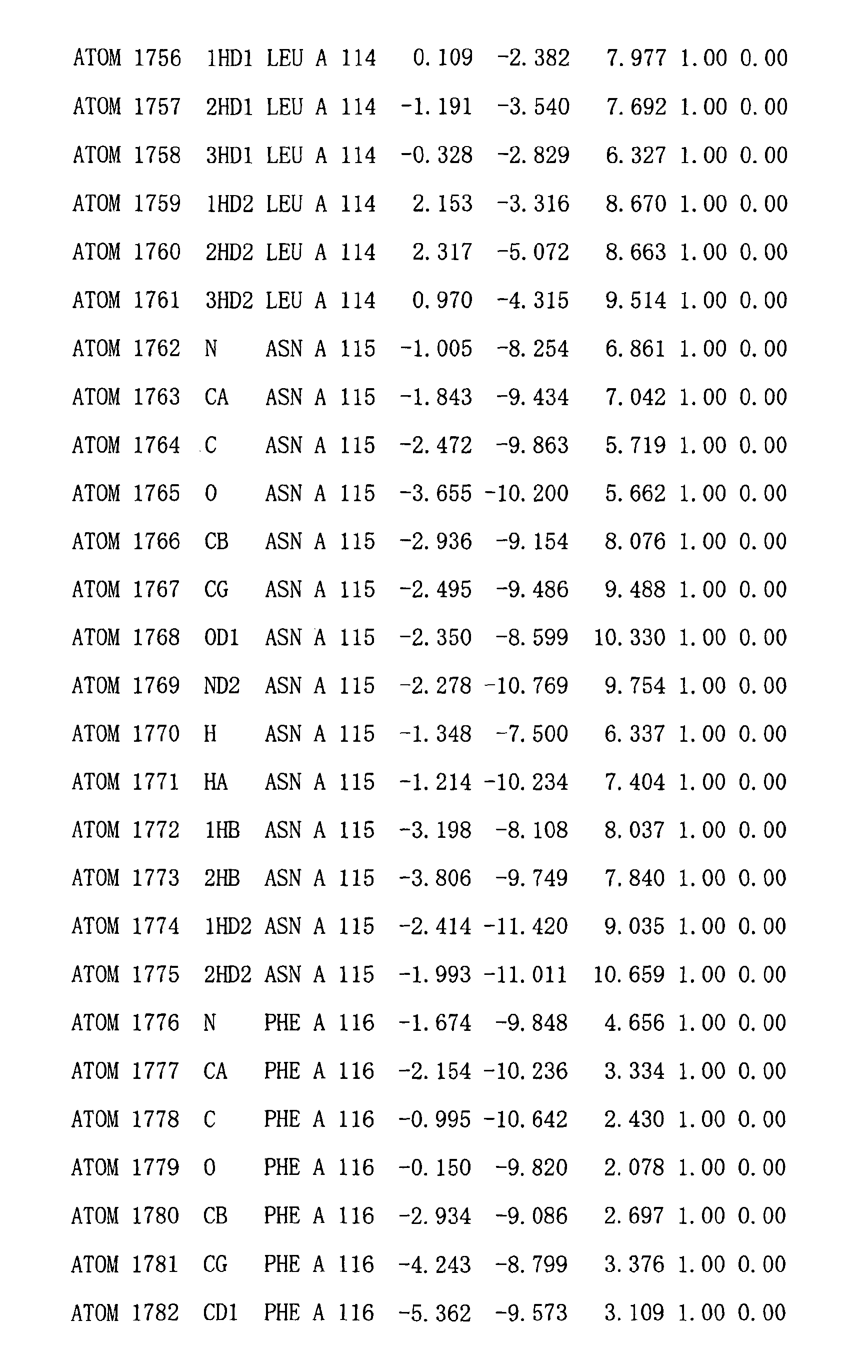

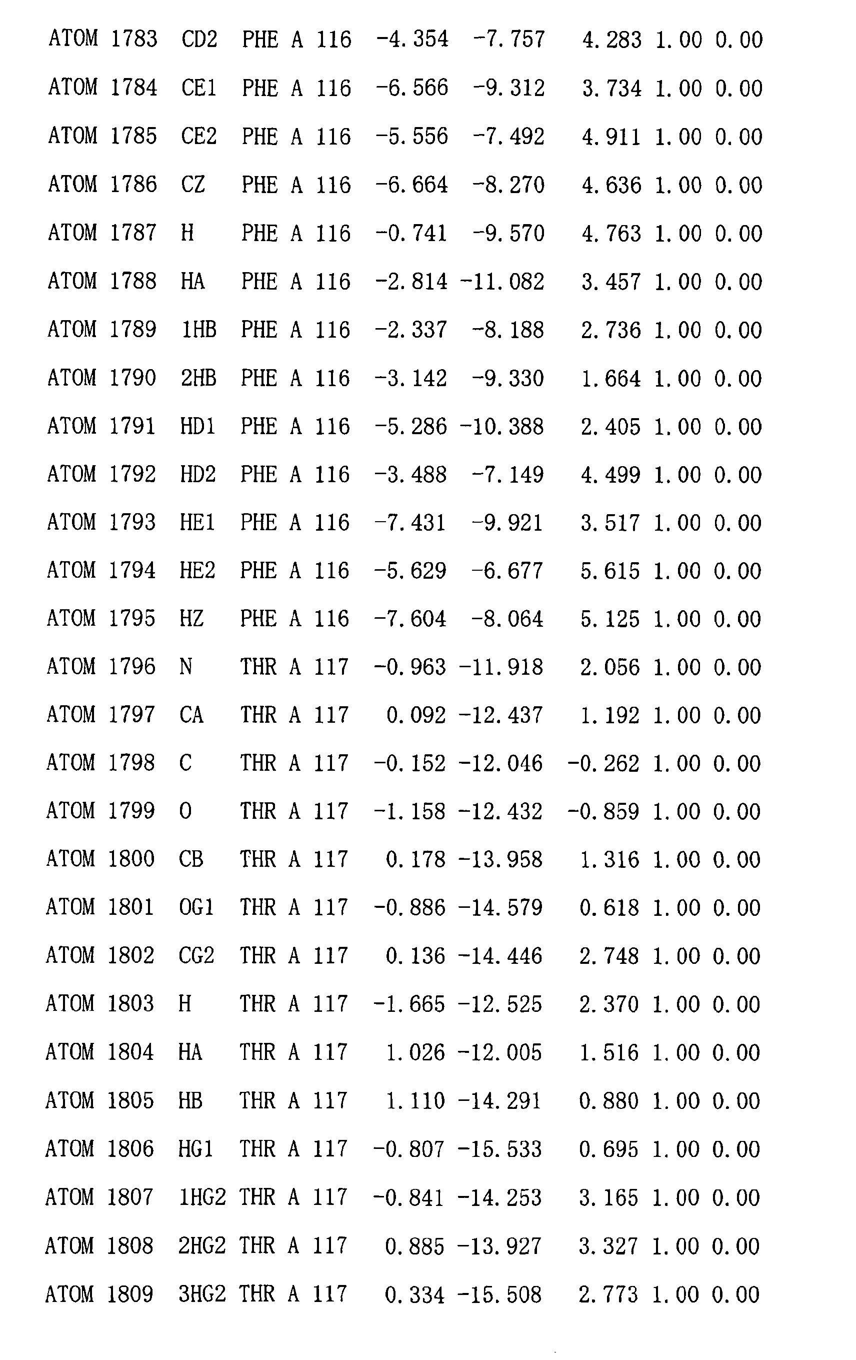

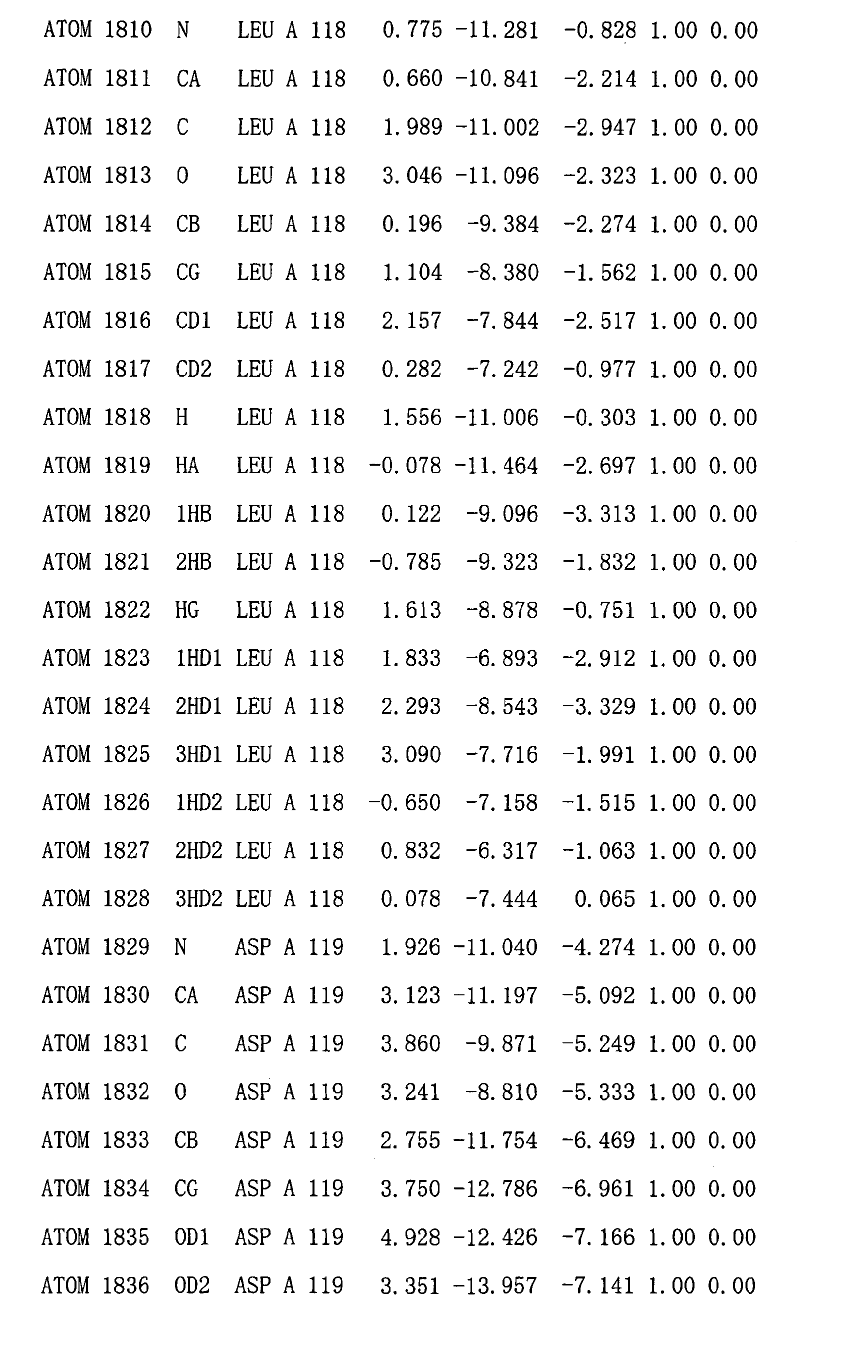

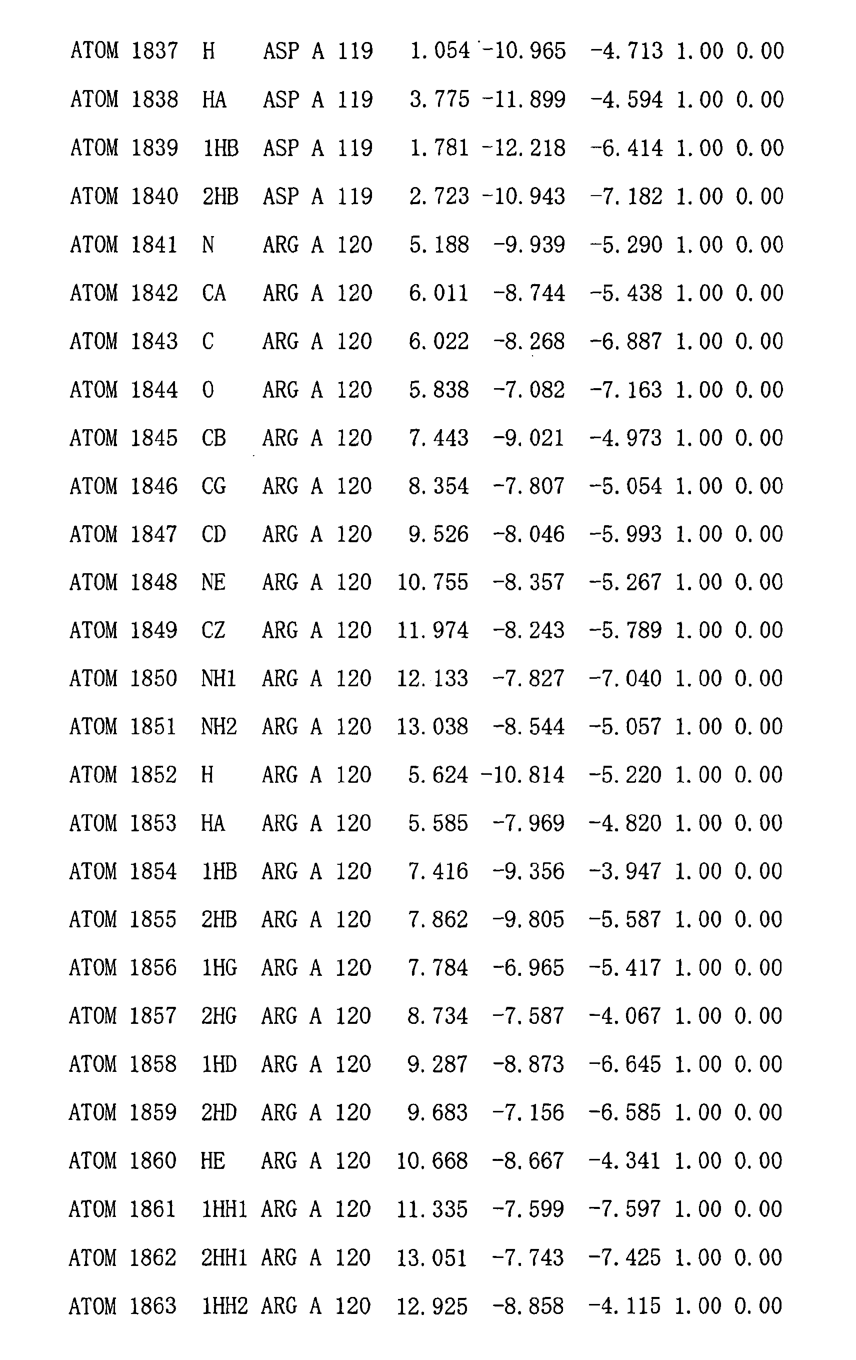

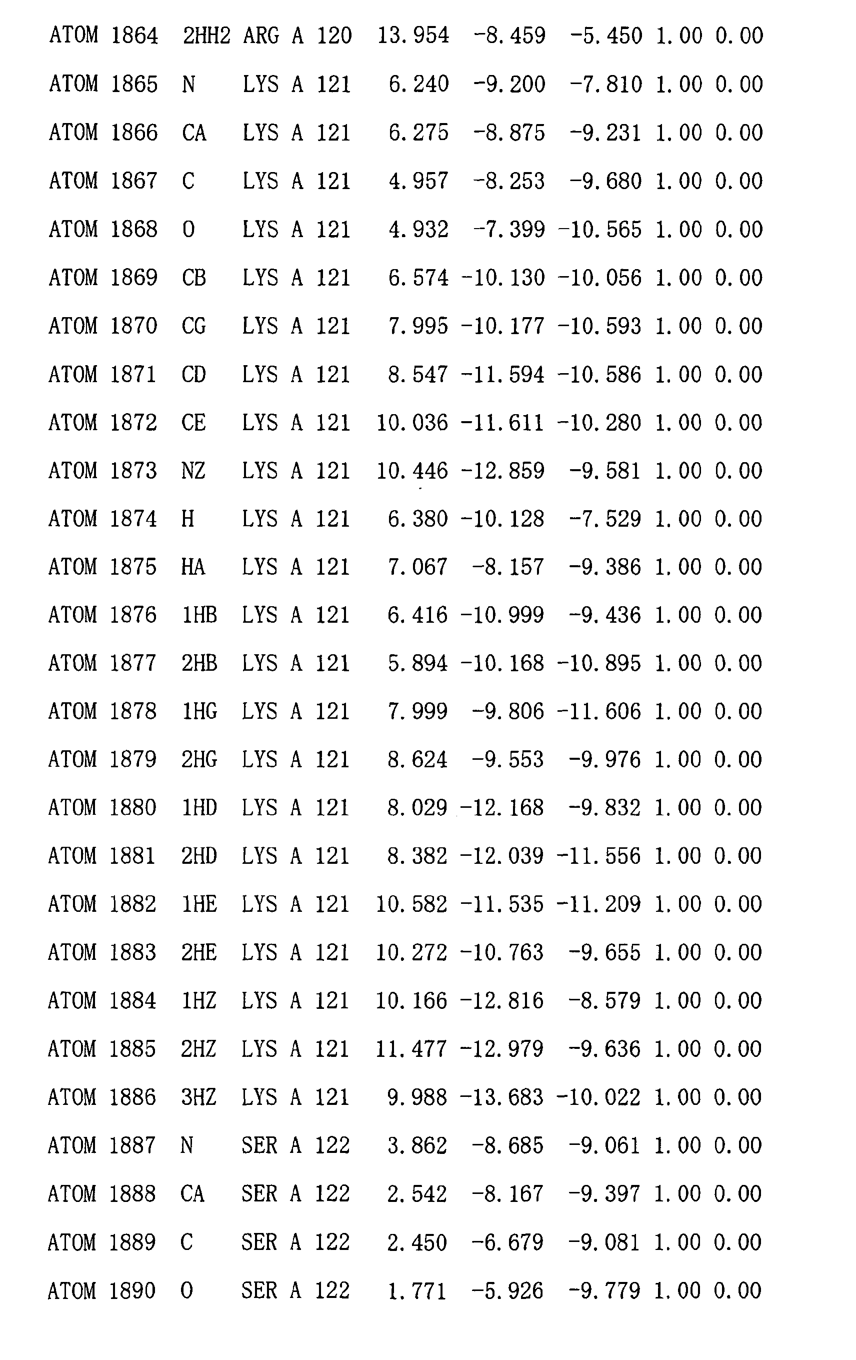

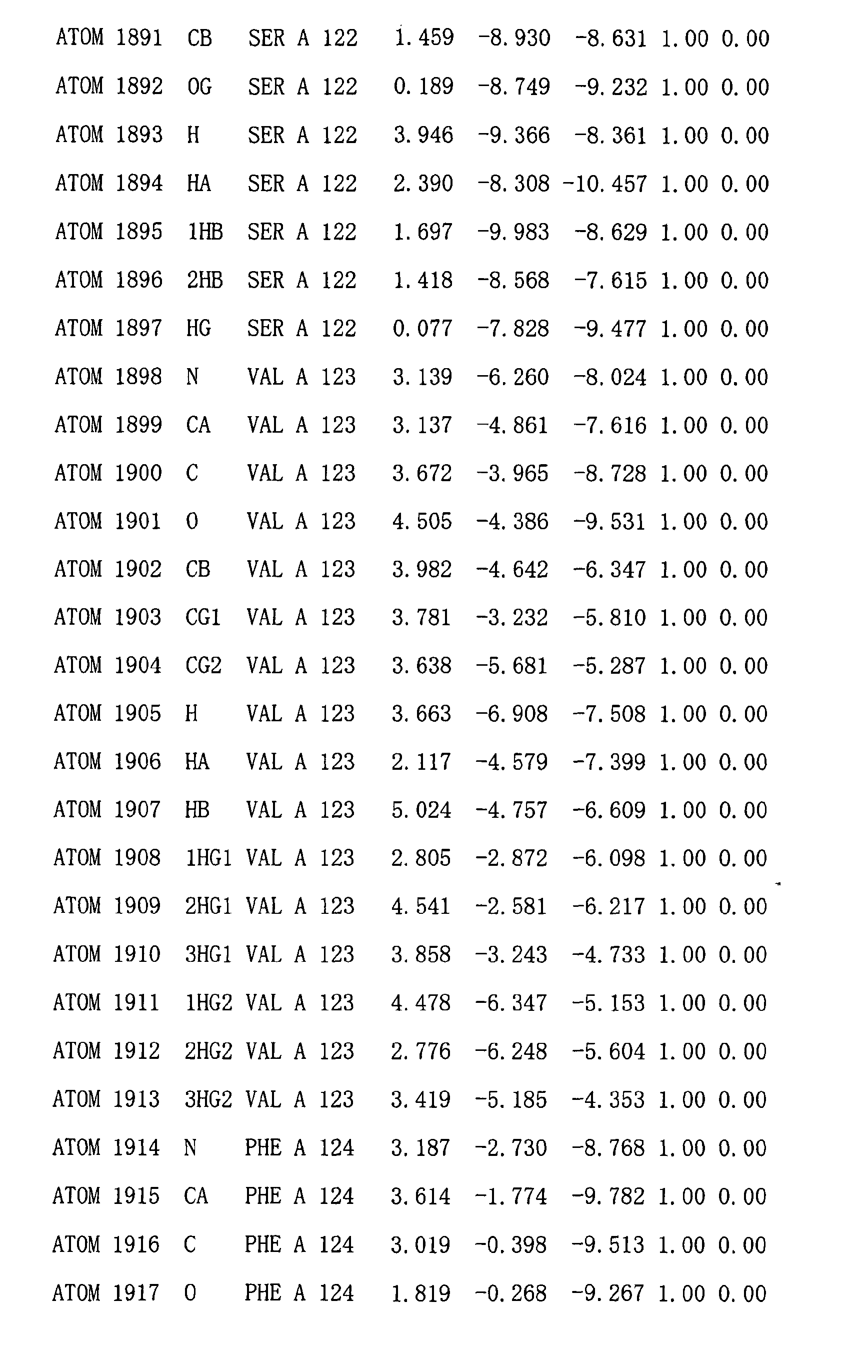

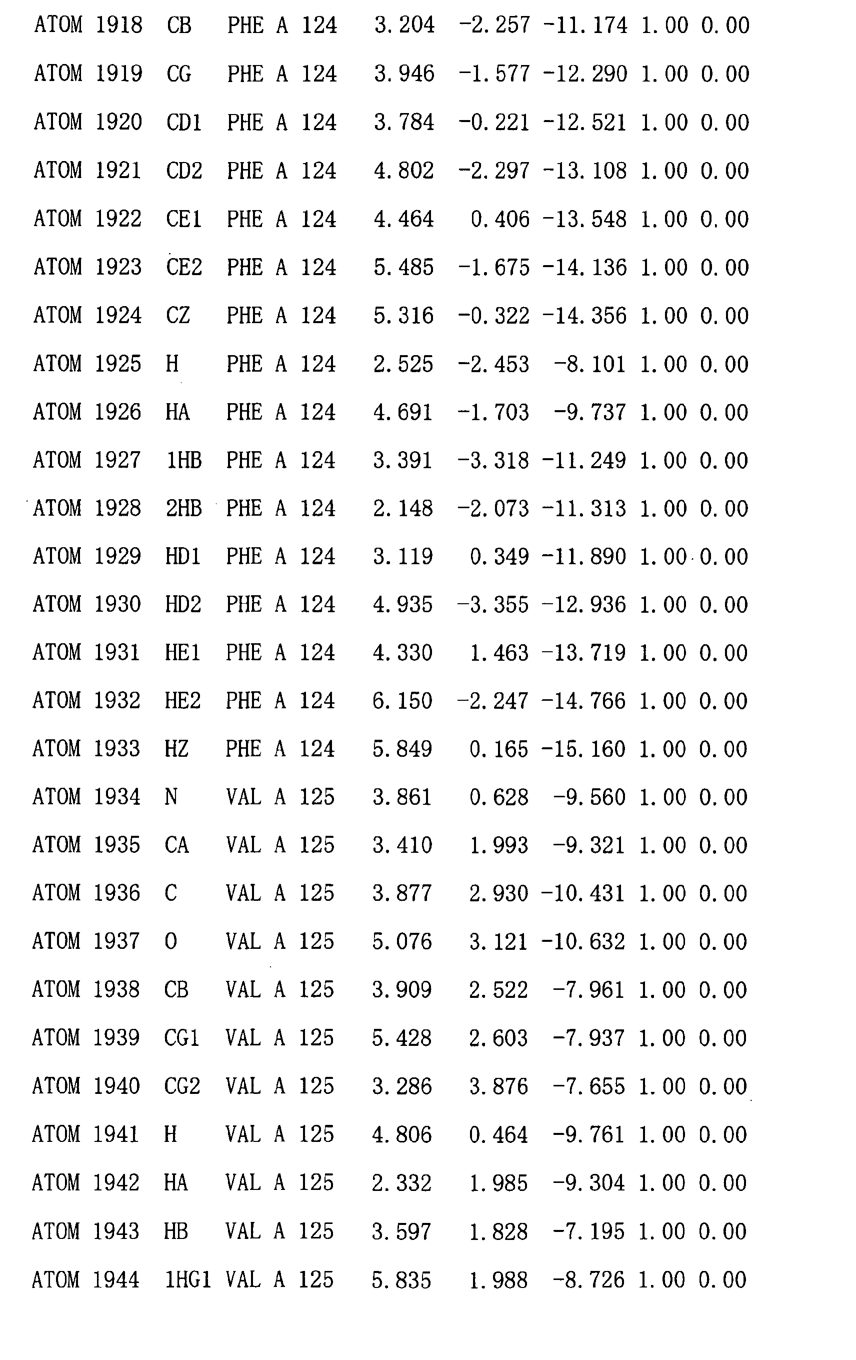

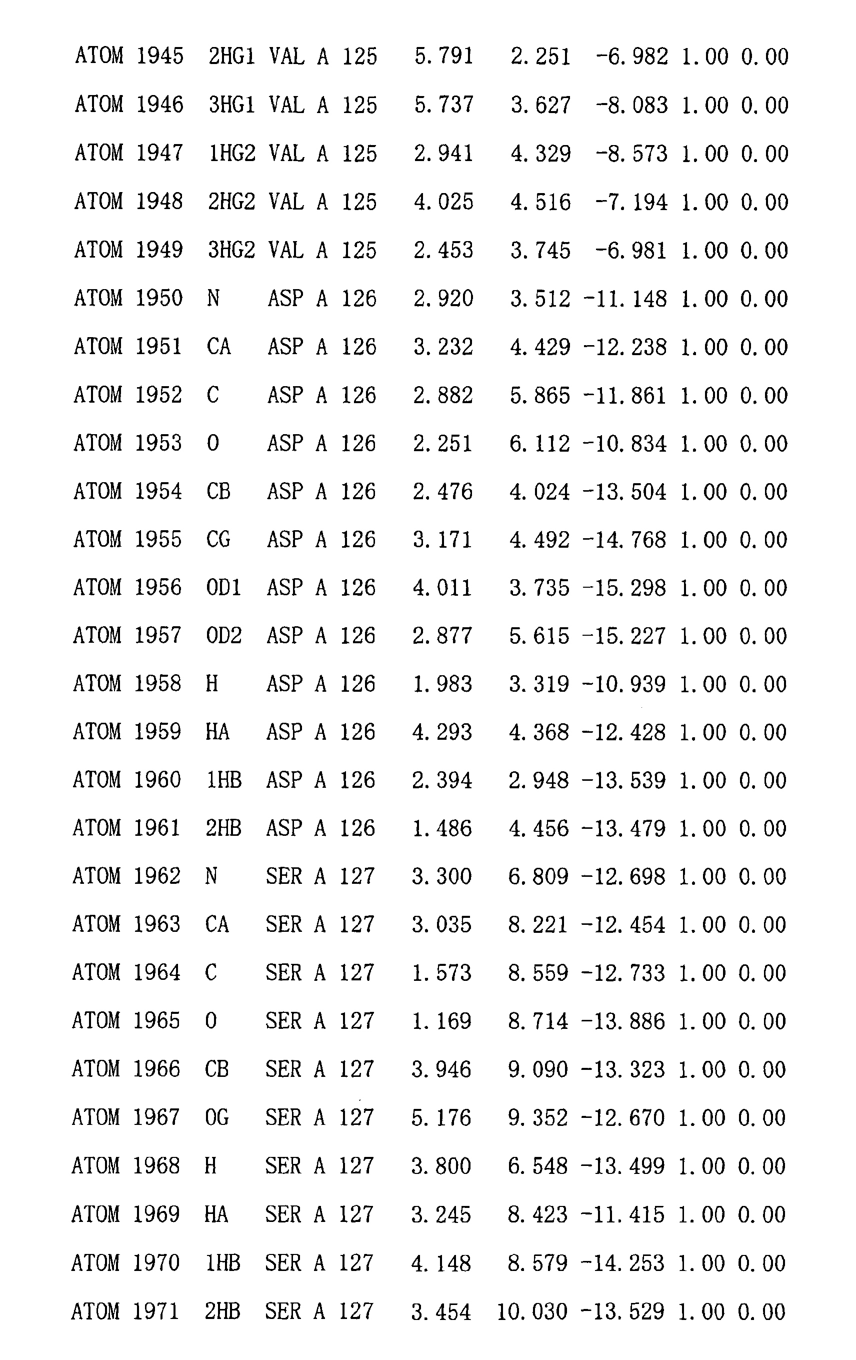

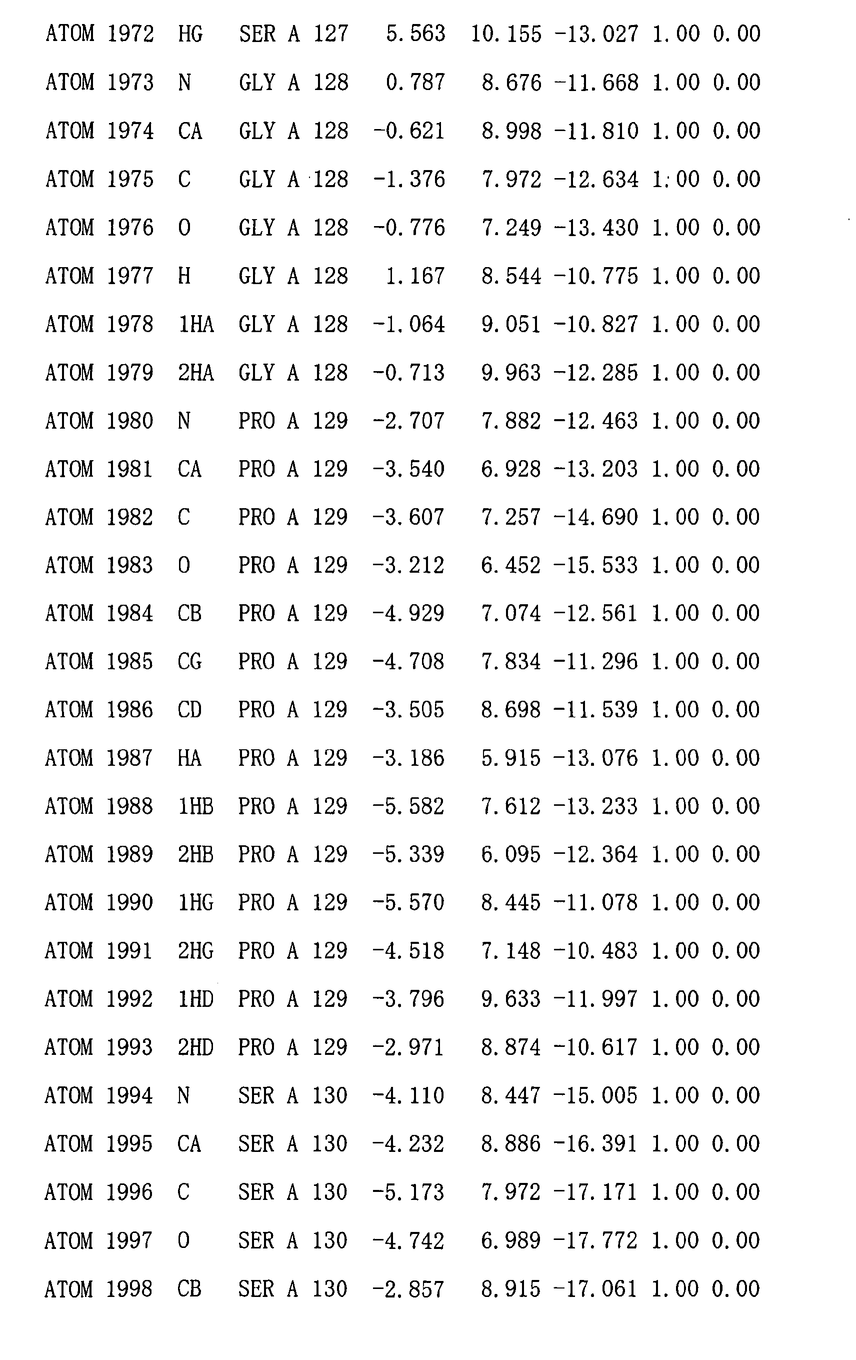

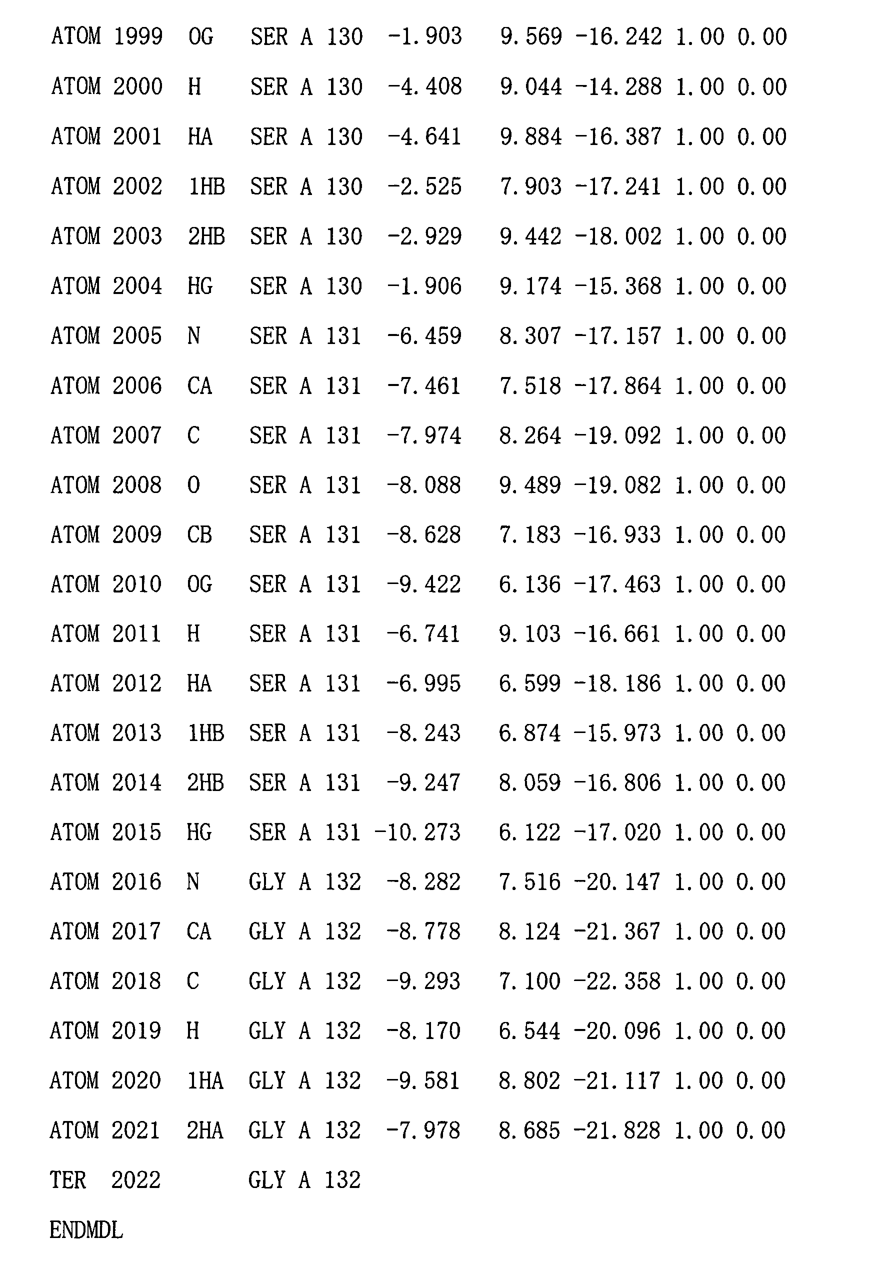

### Three-Dimensional Structure Coordinate 15

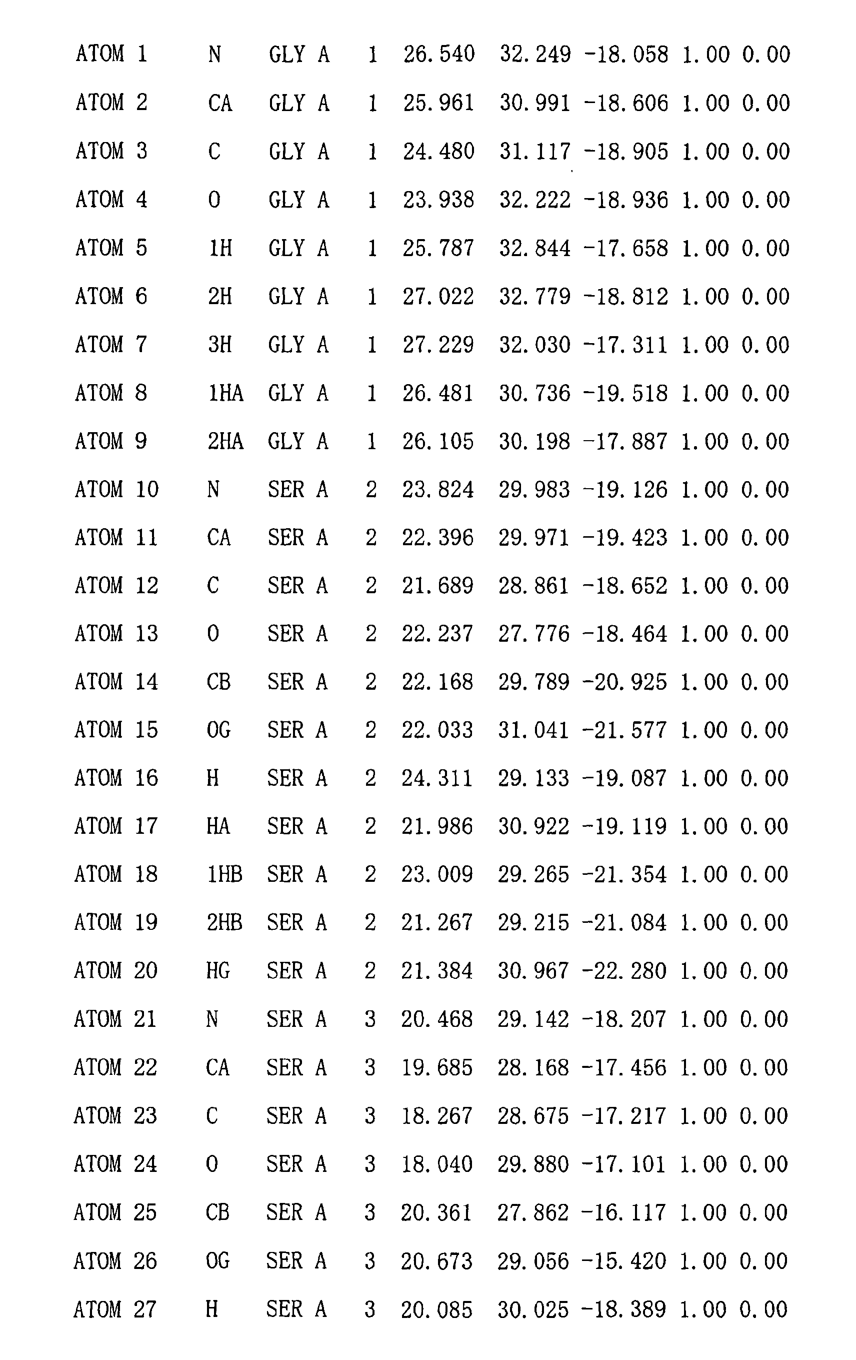

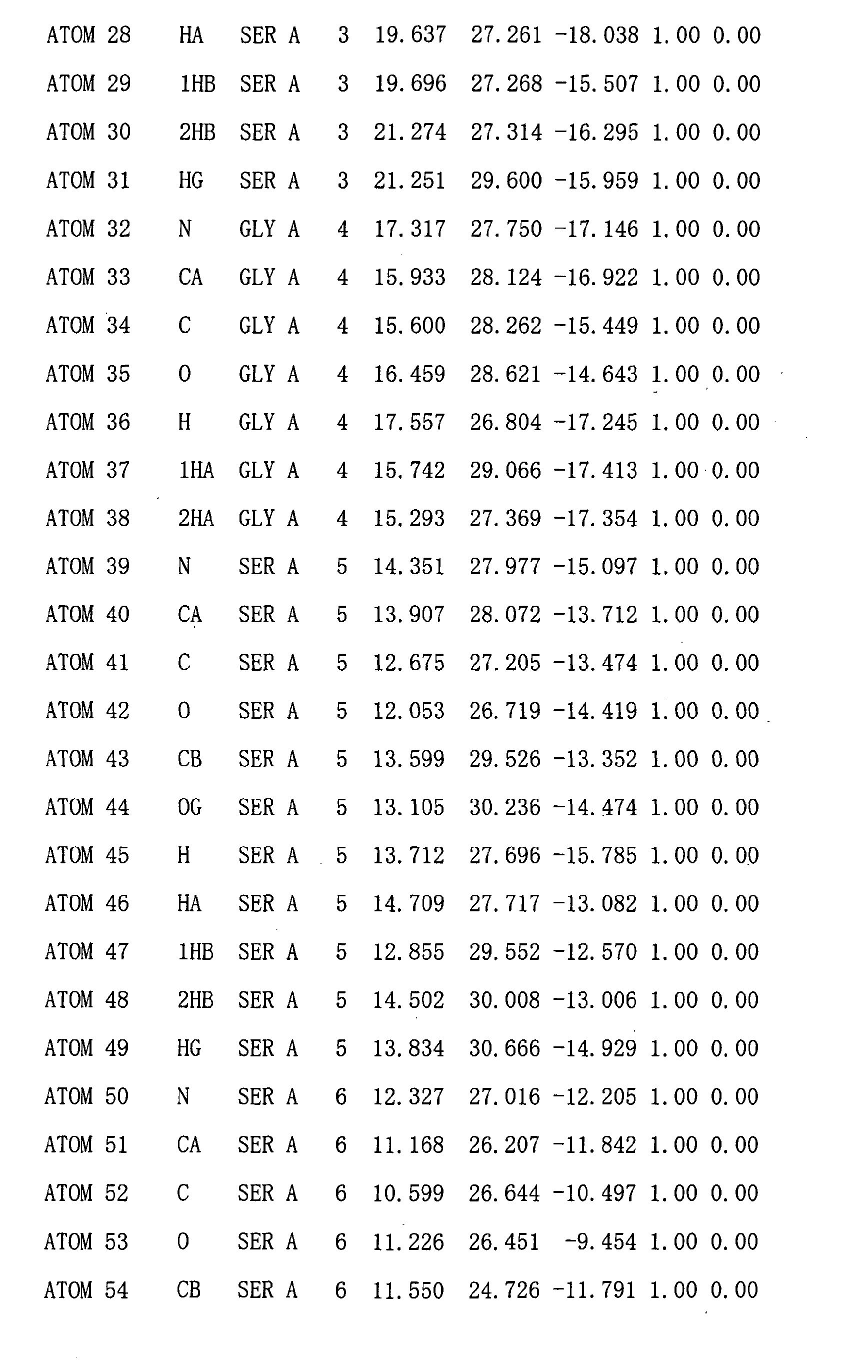

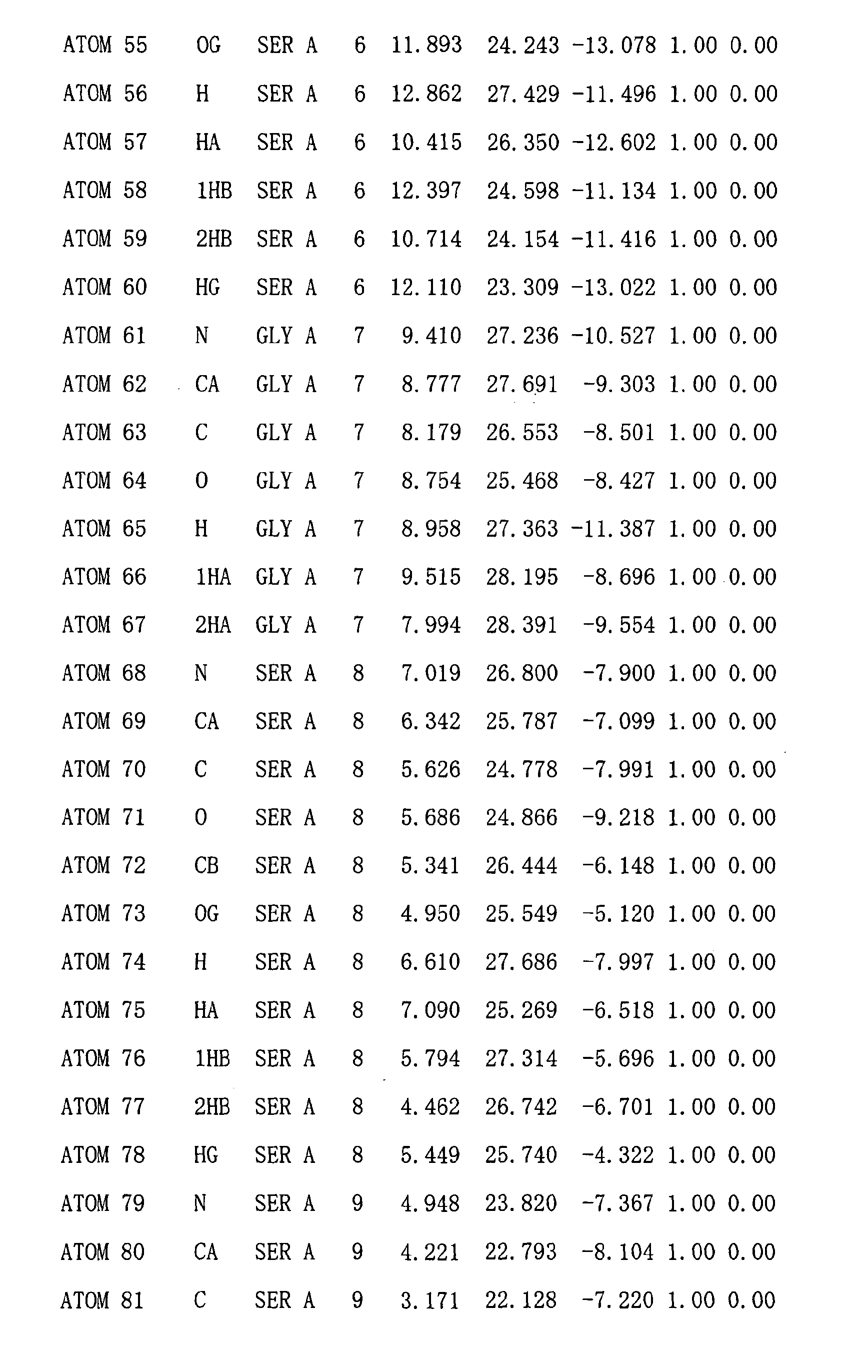

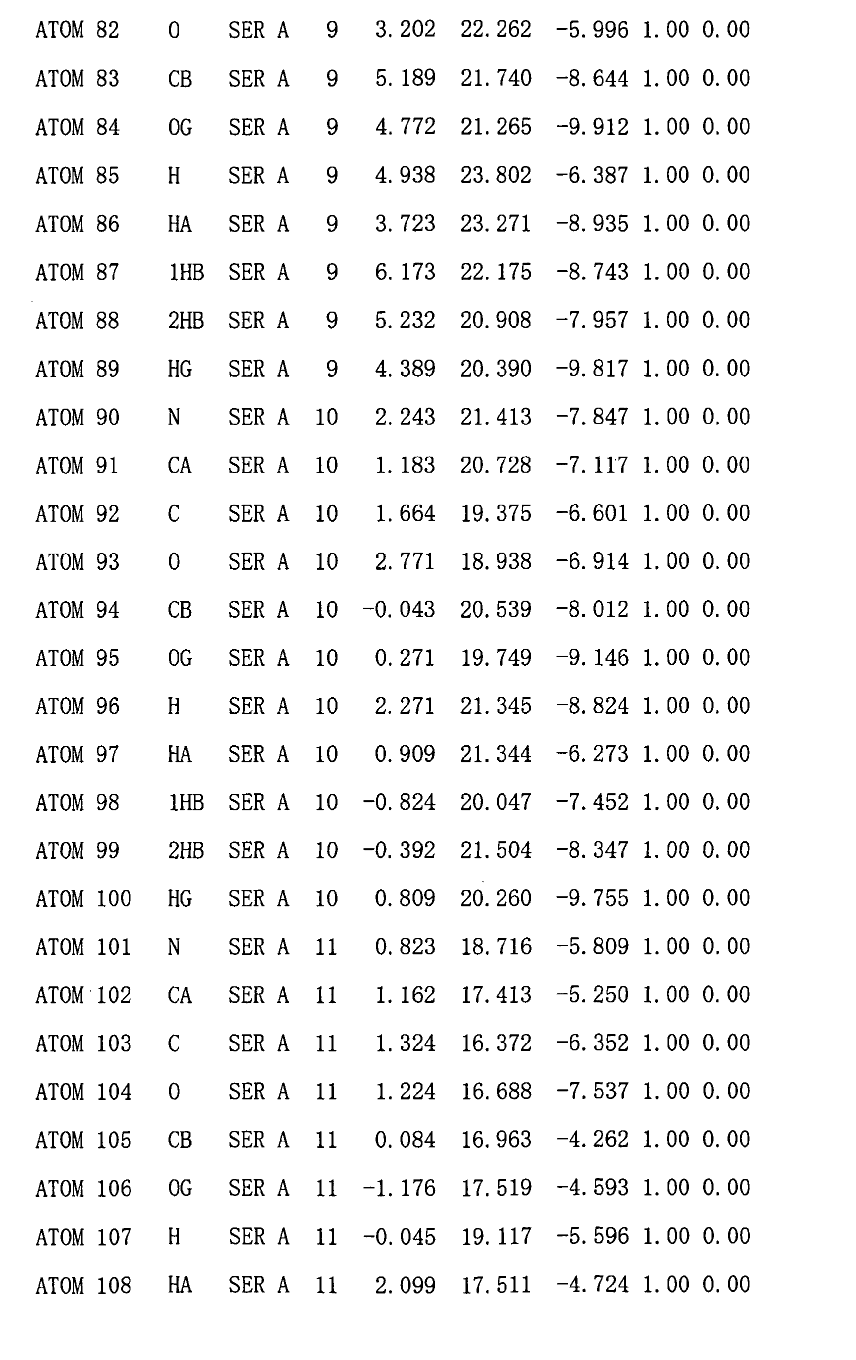

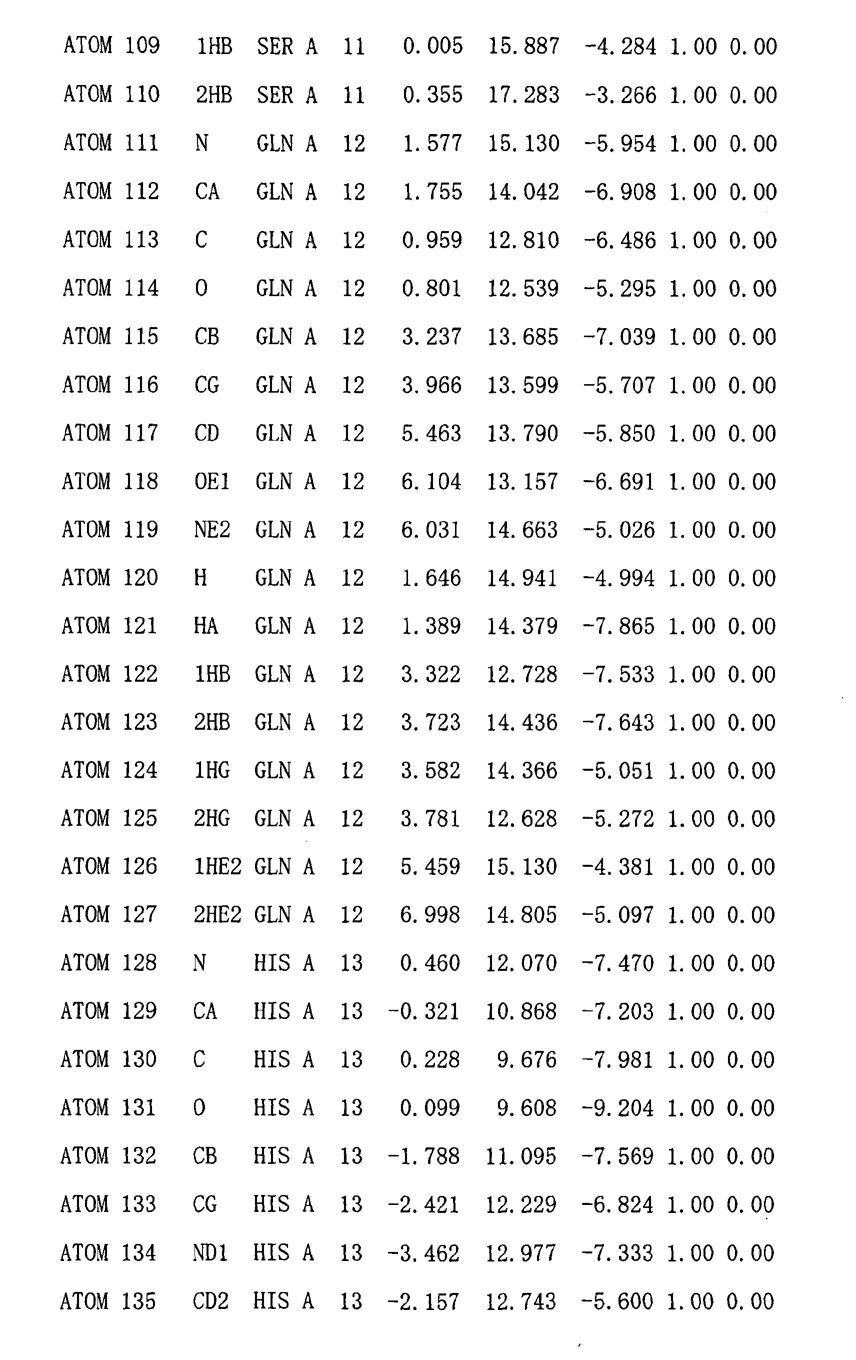

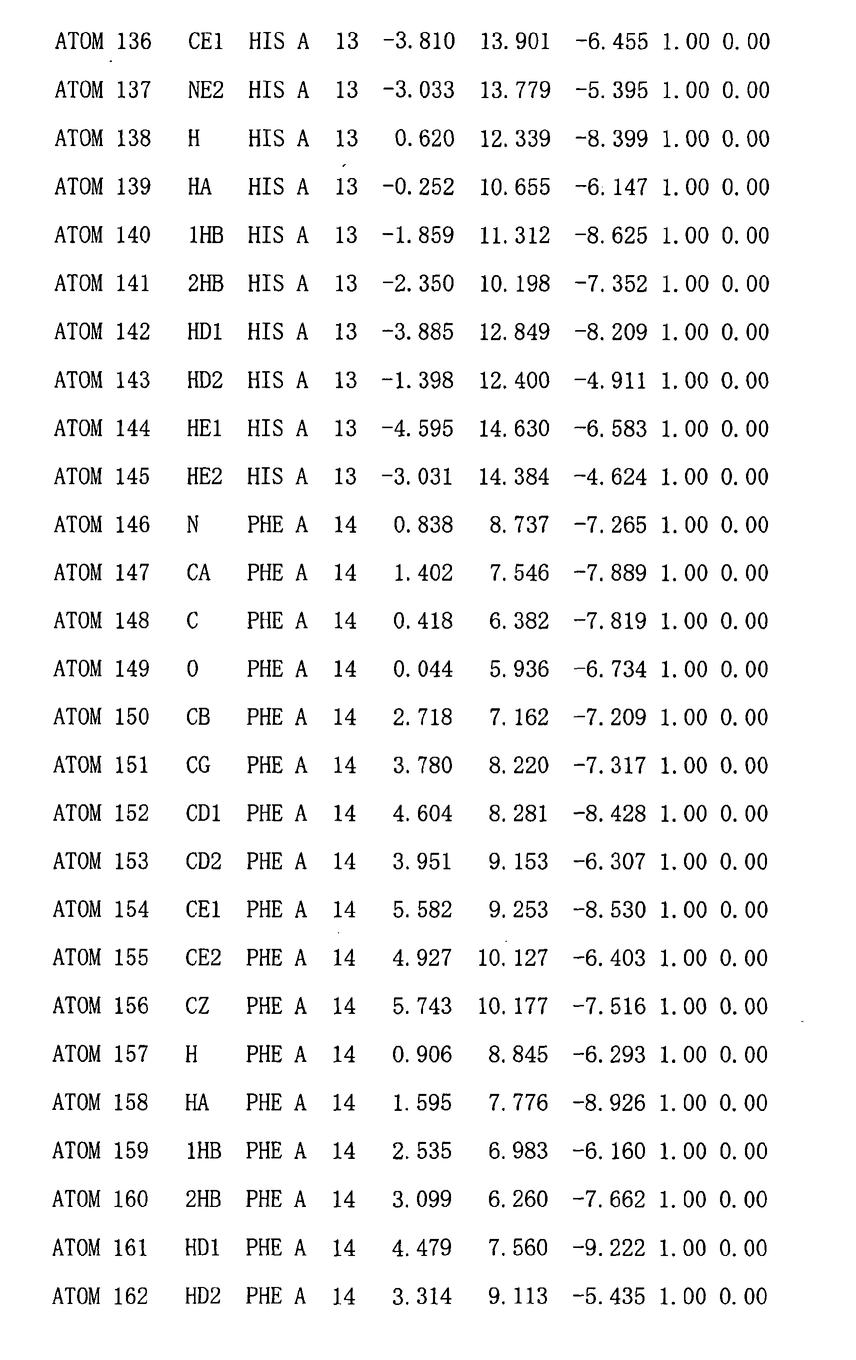

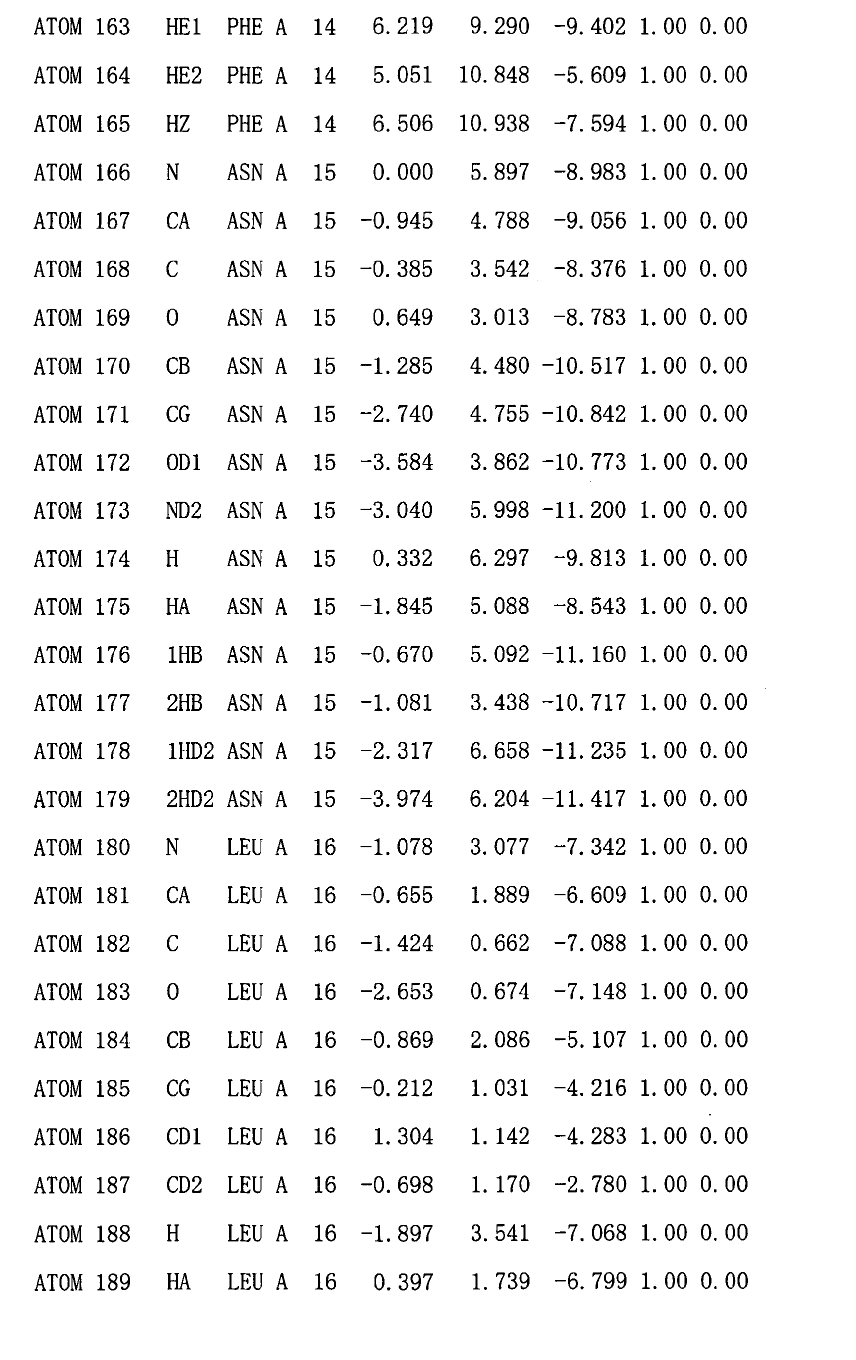

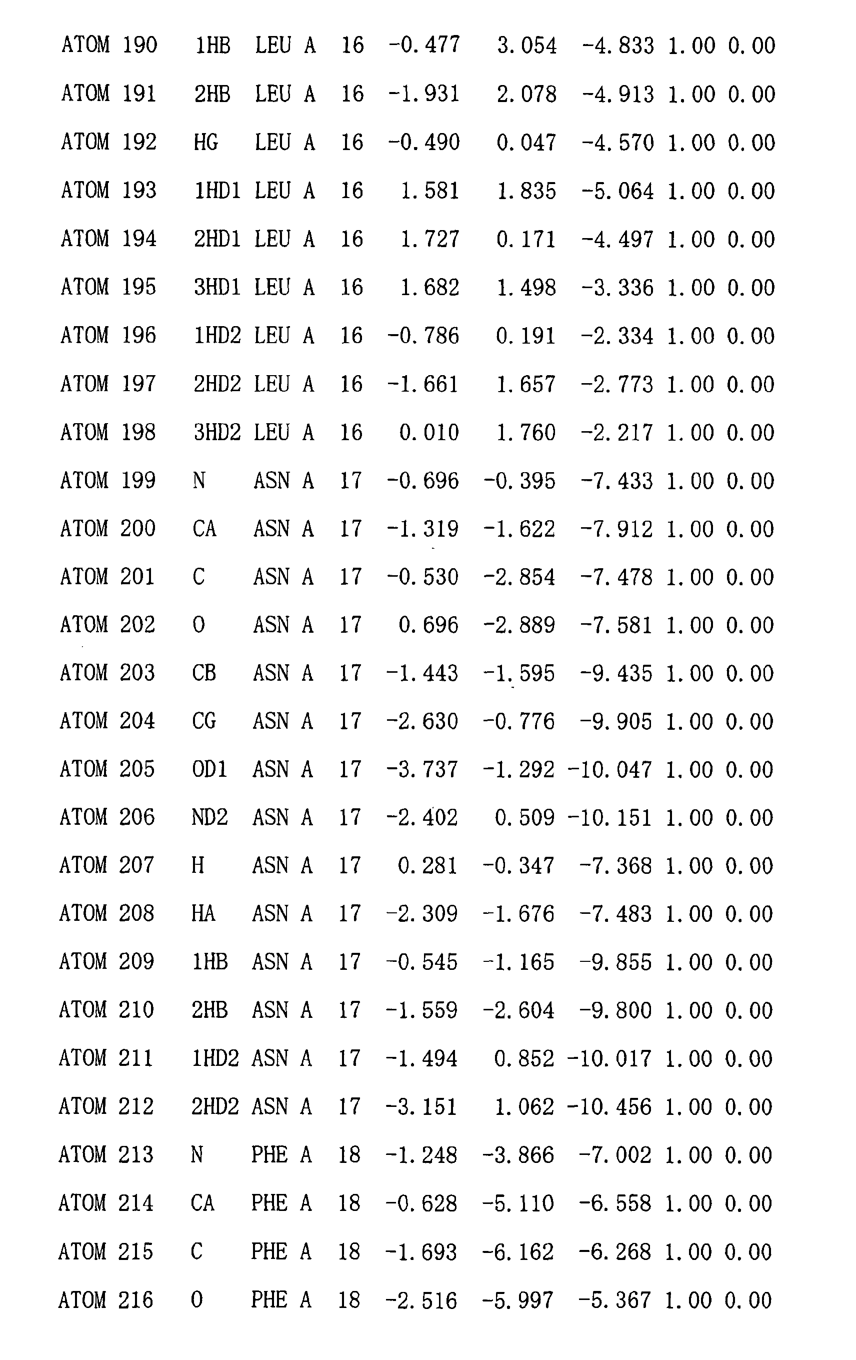

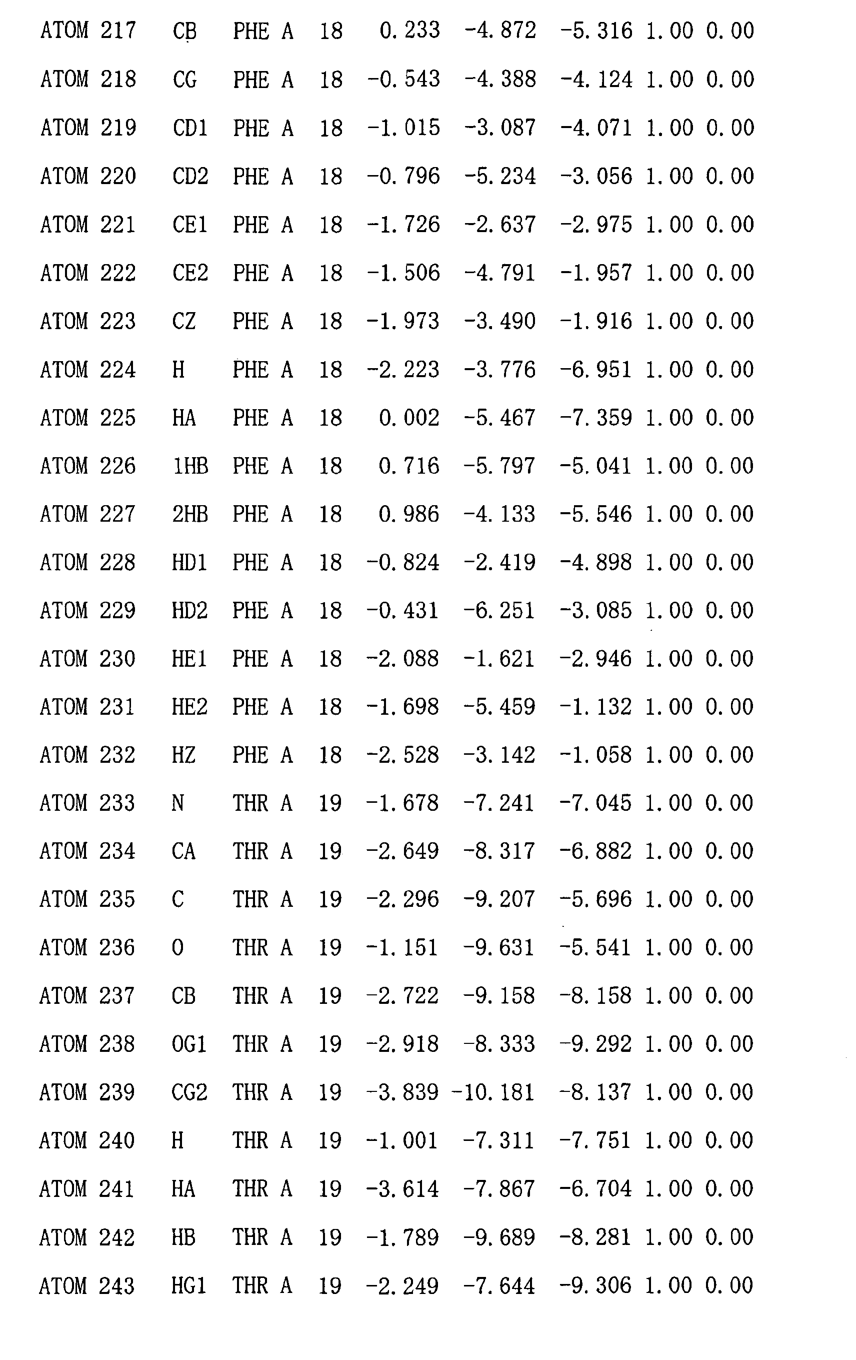

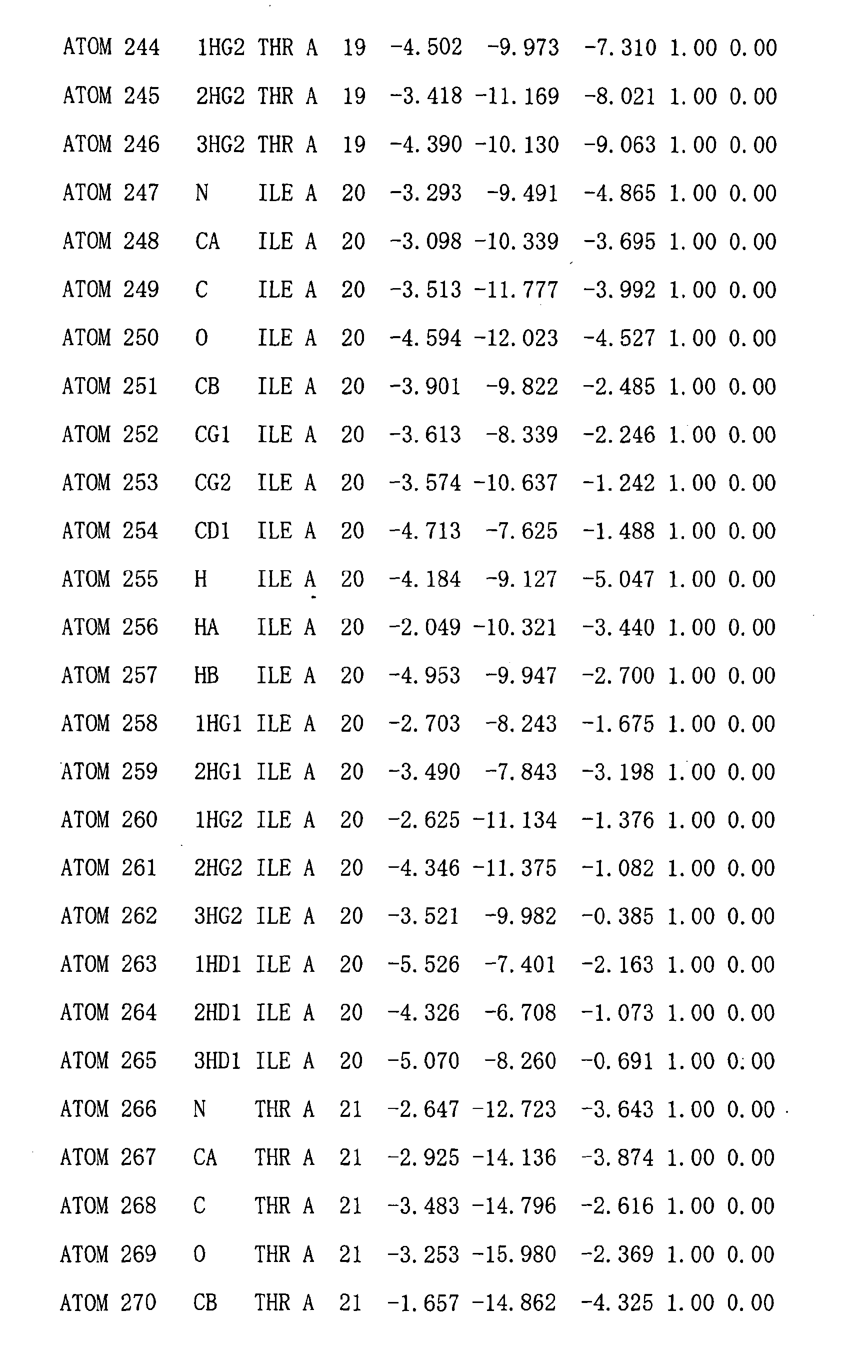

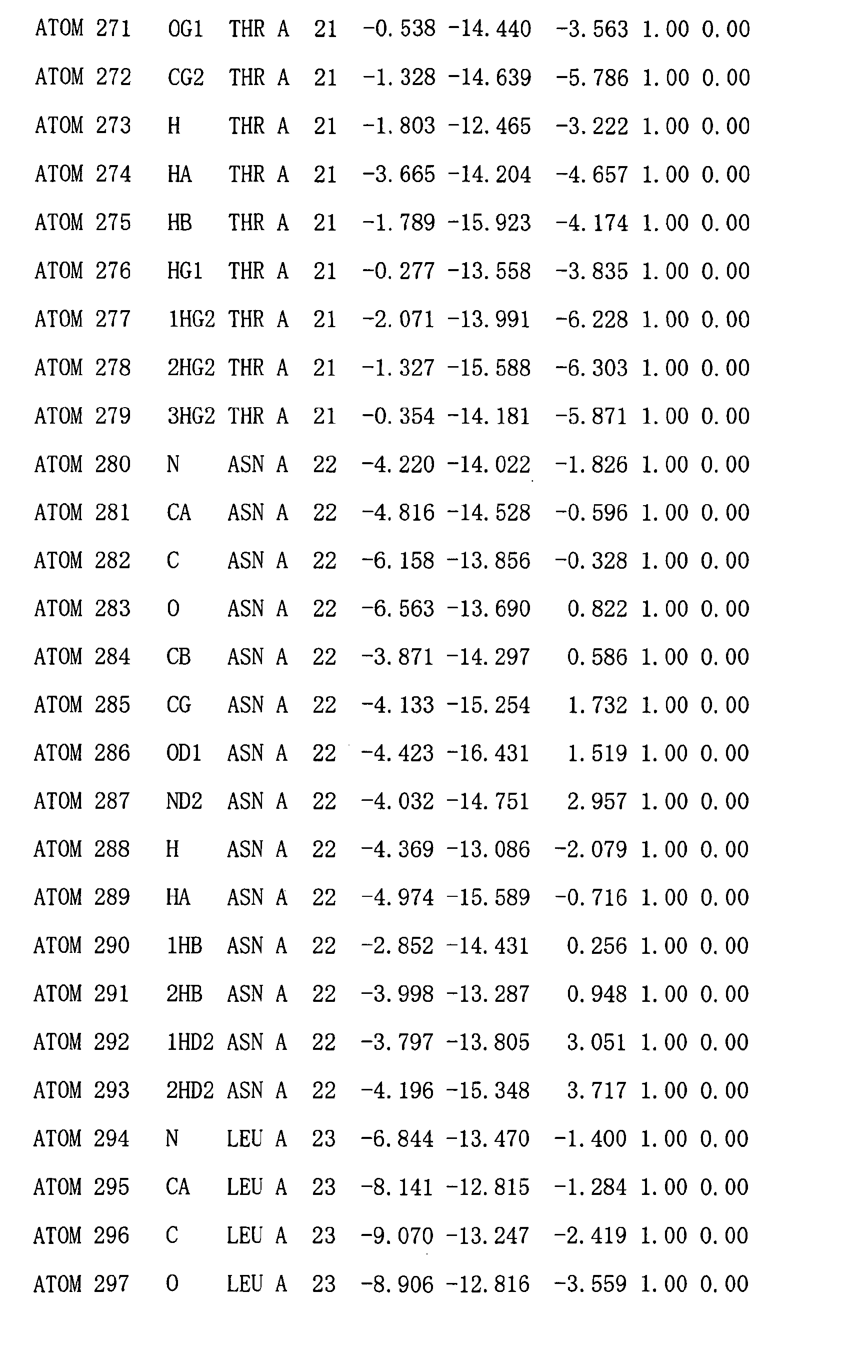

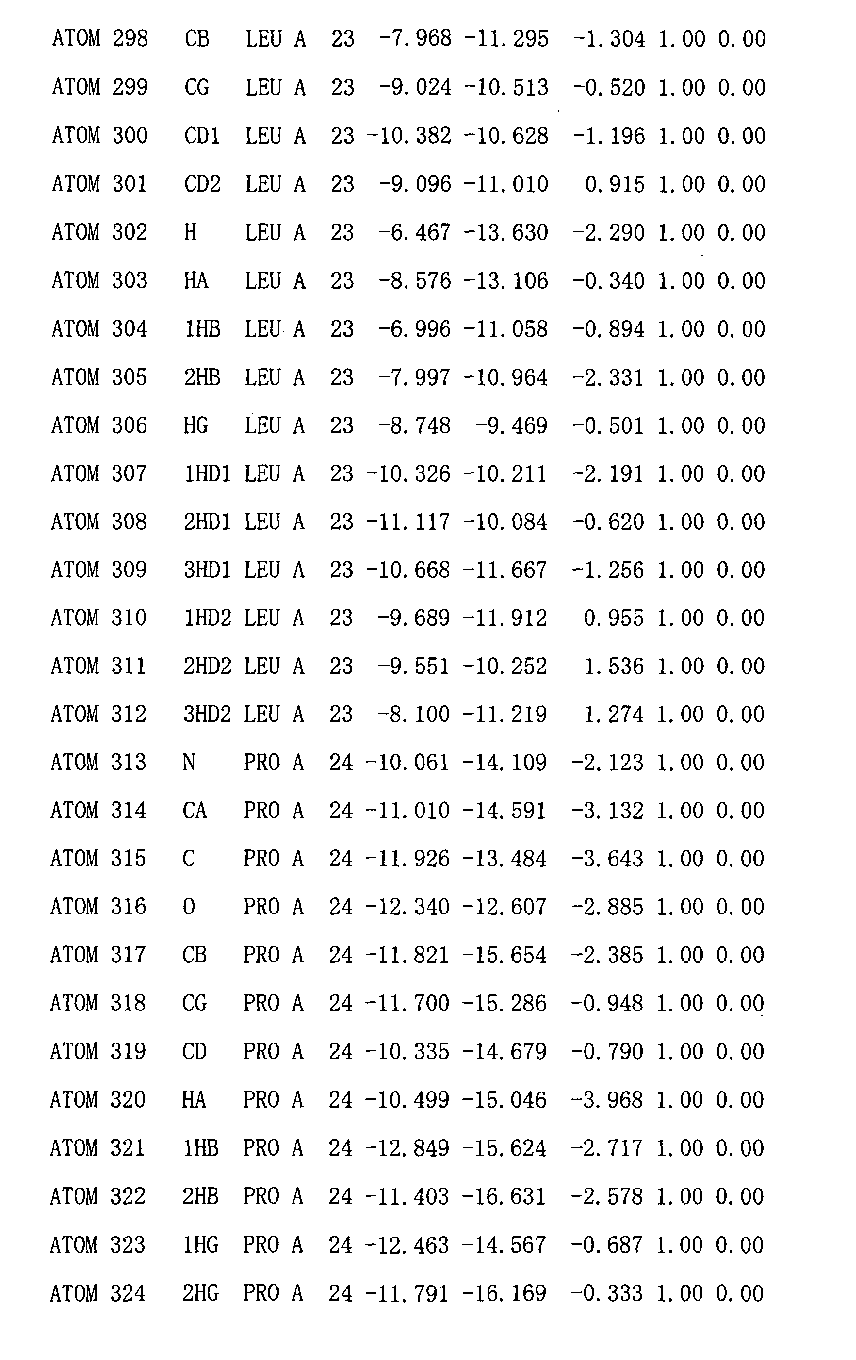

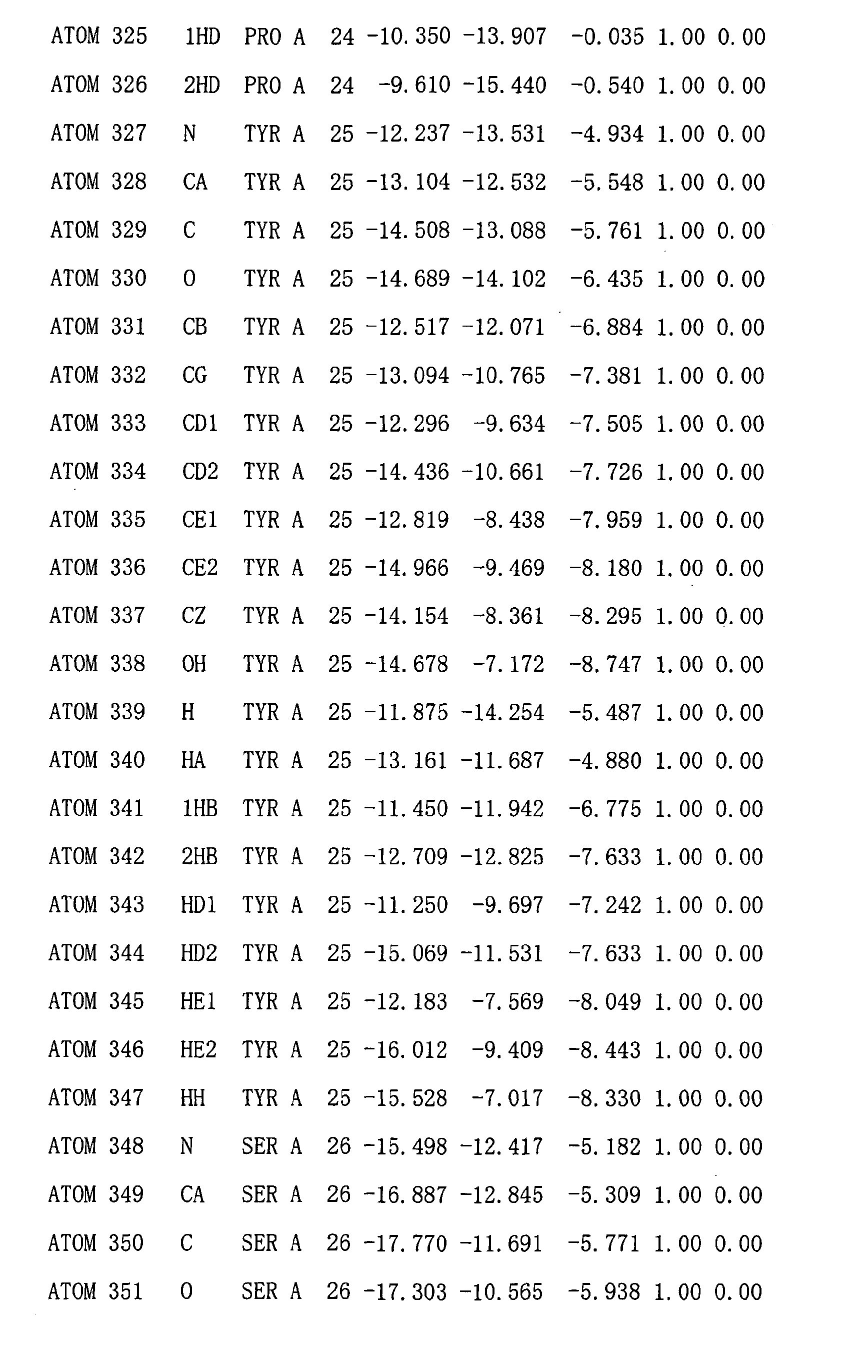

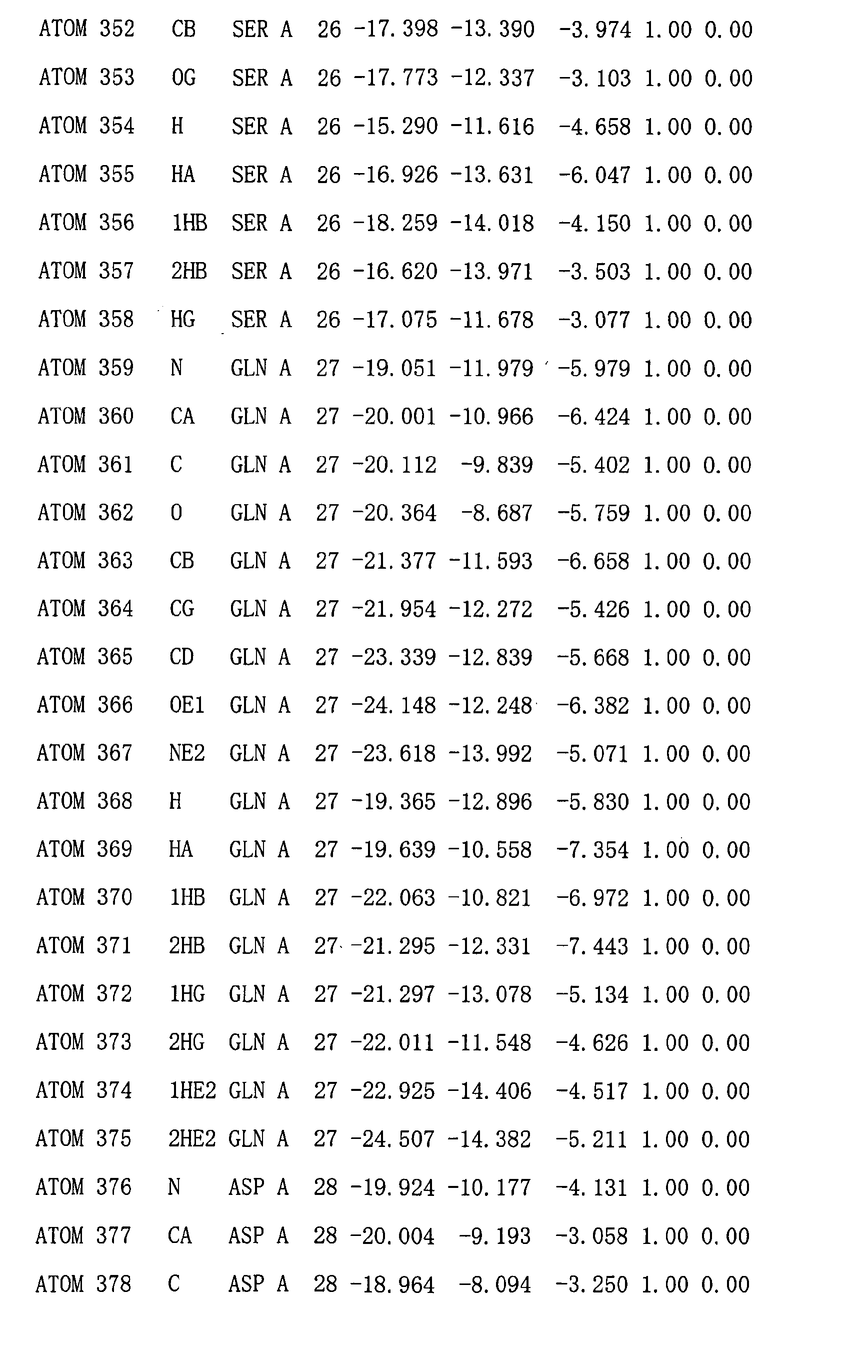

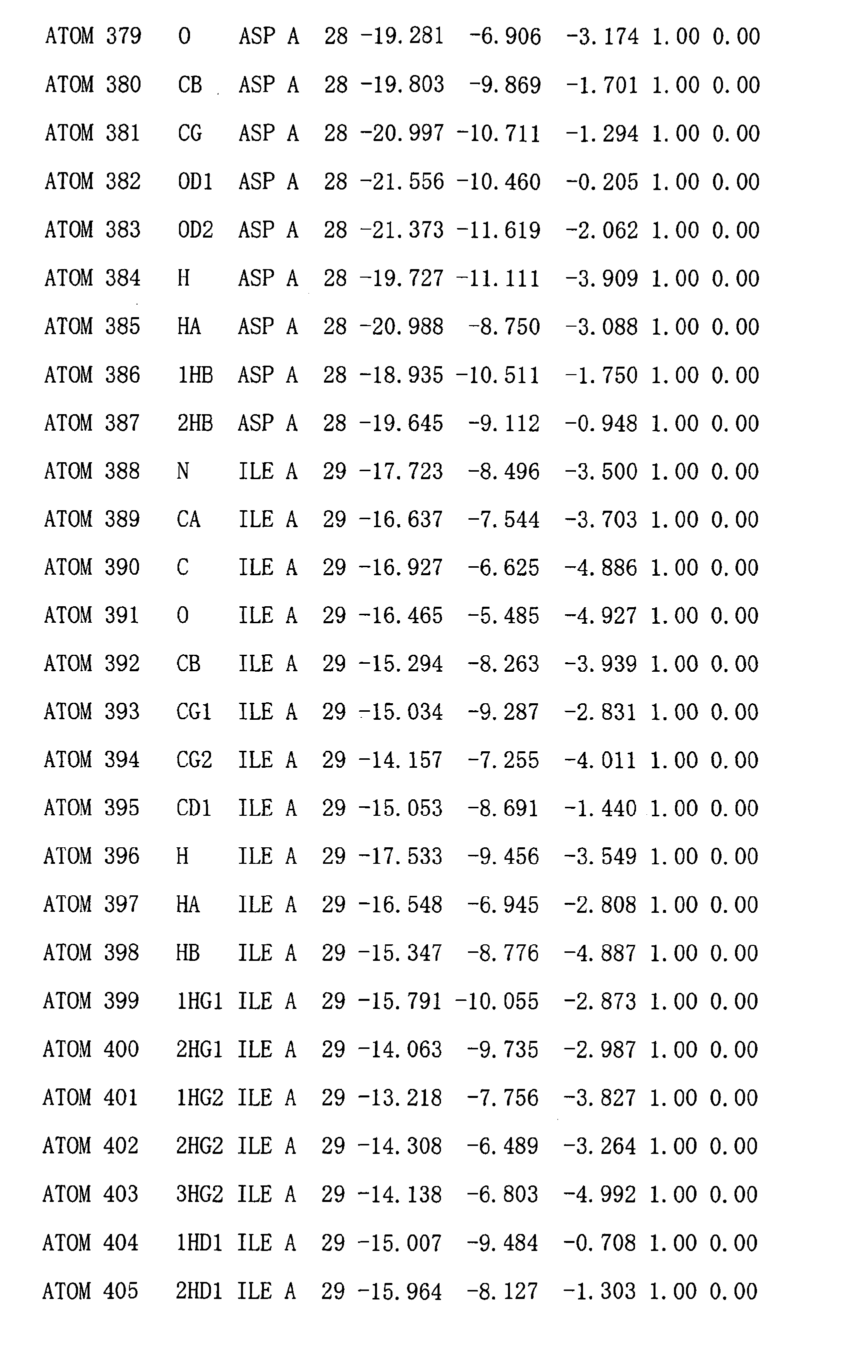

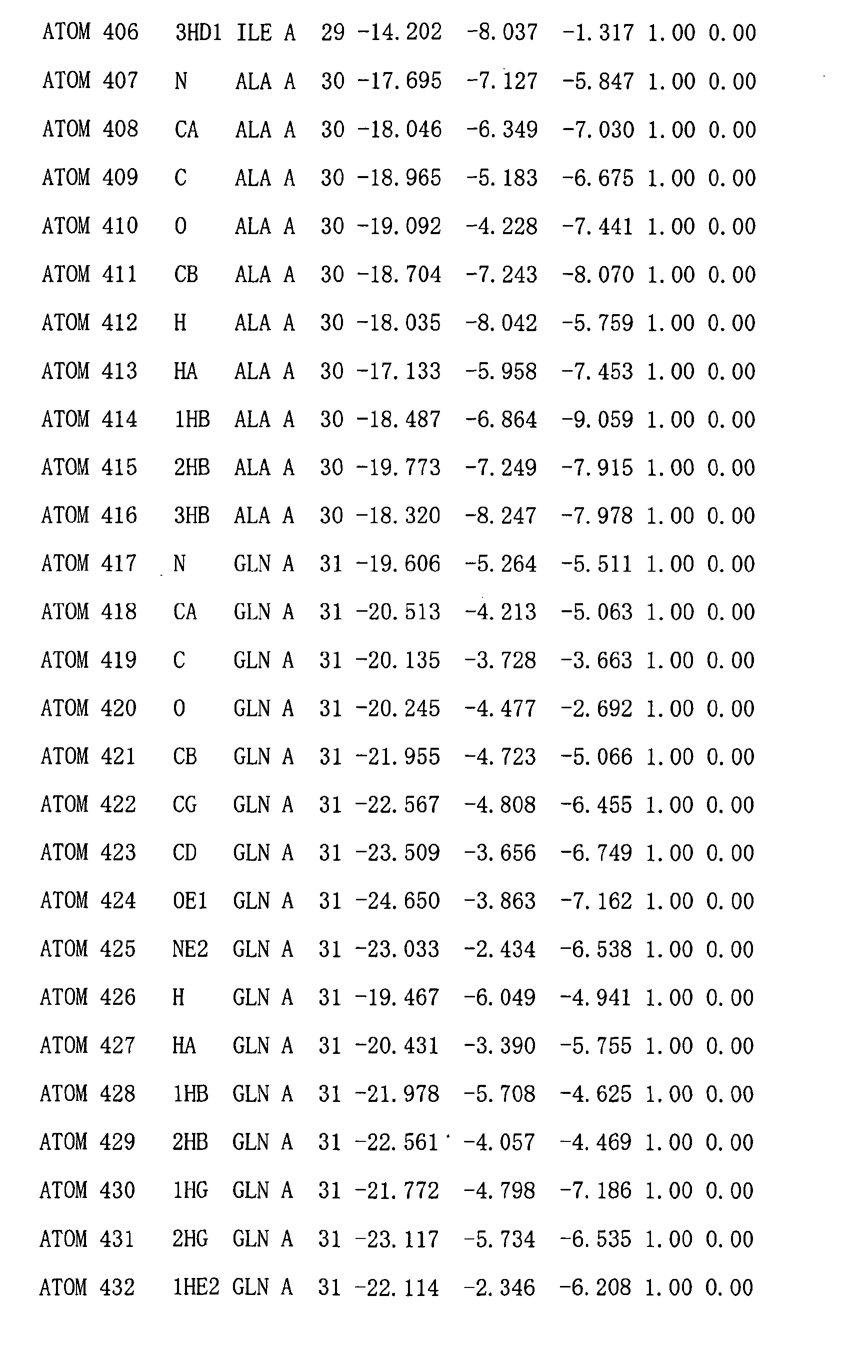

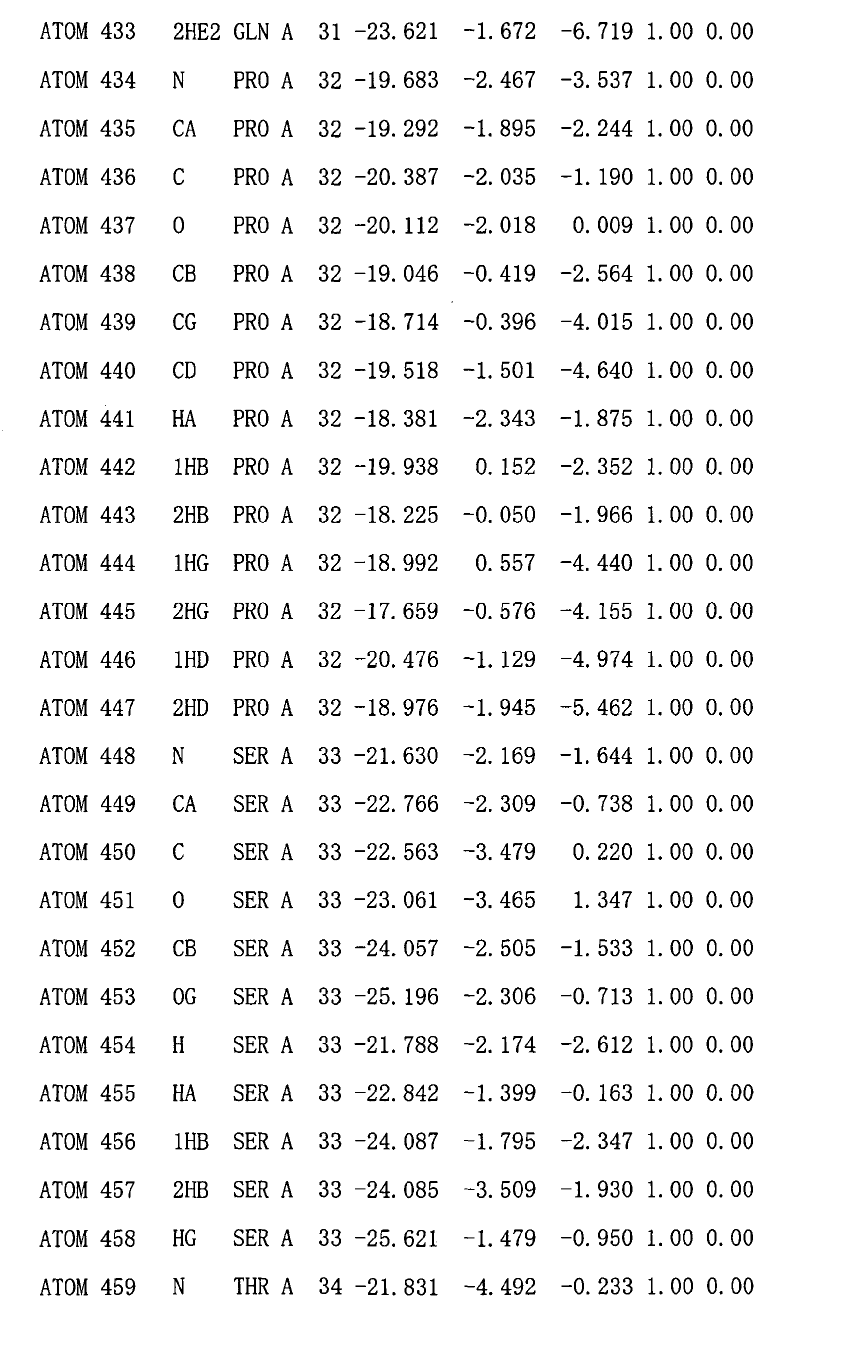

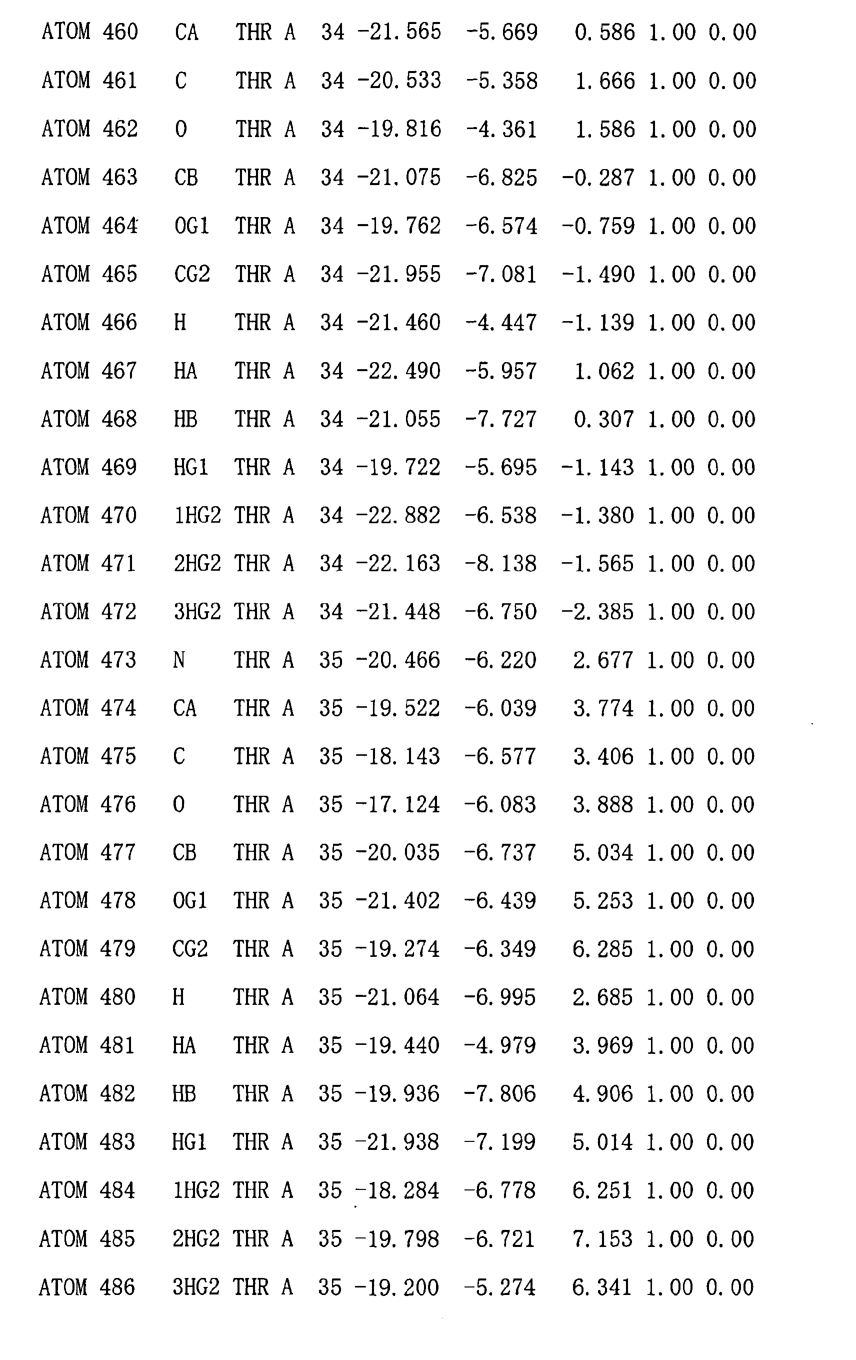

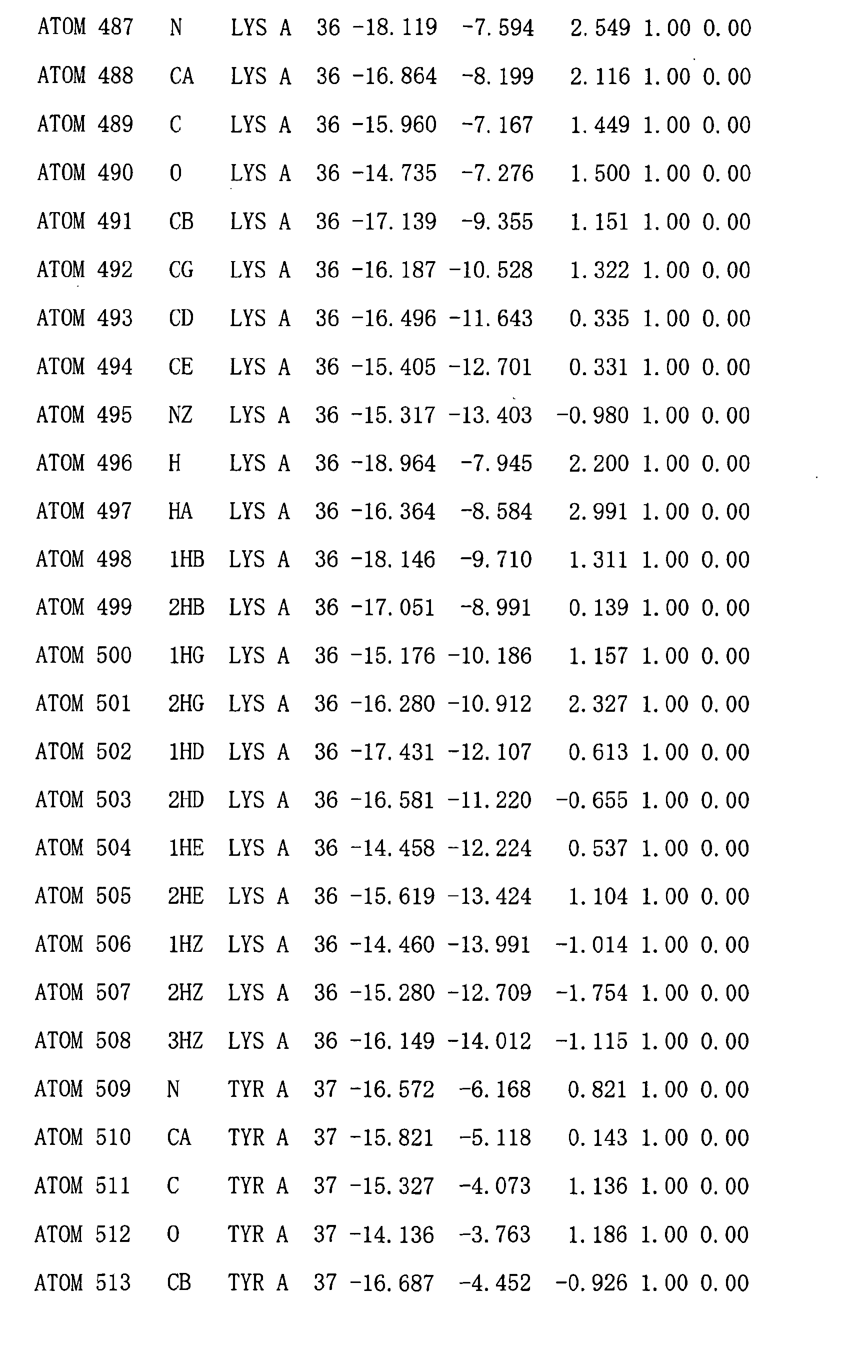

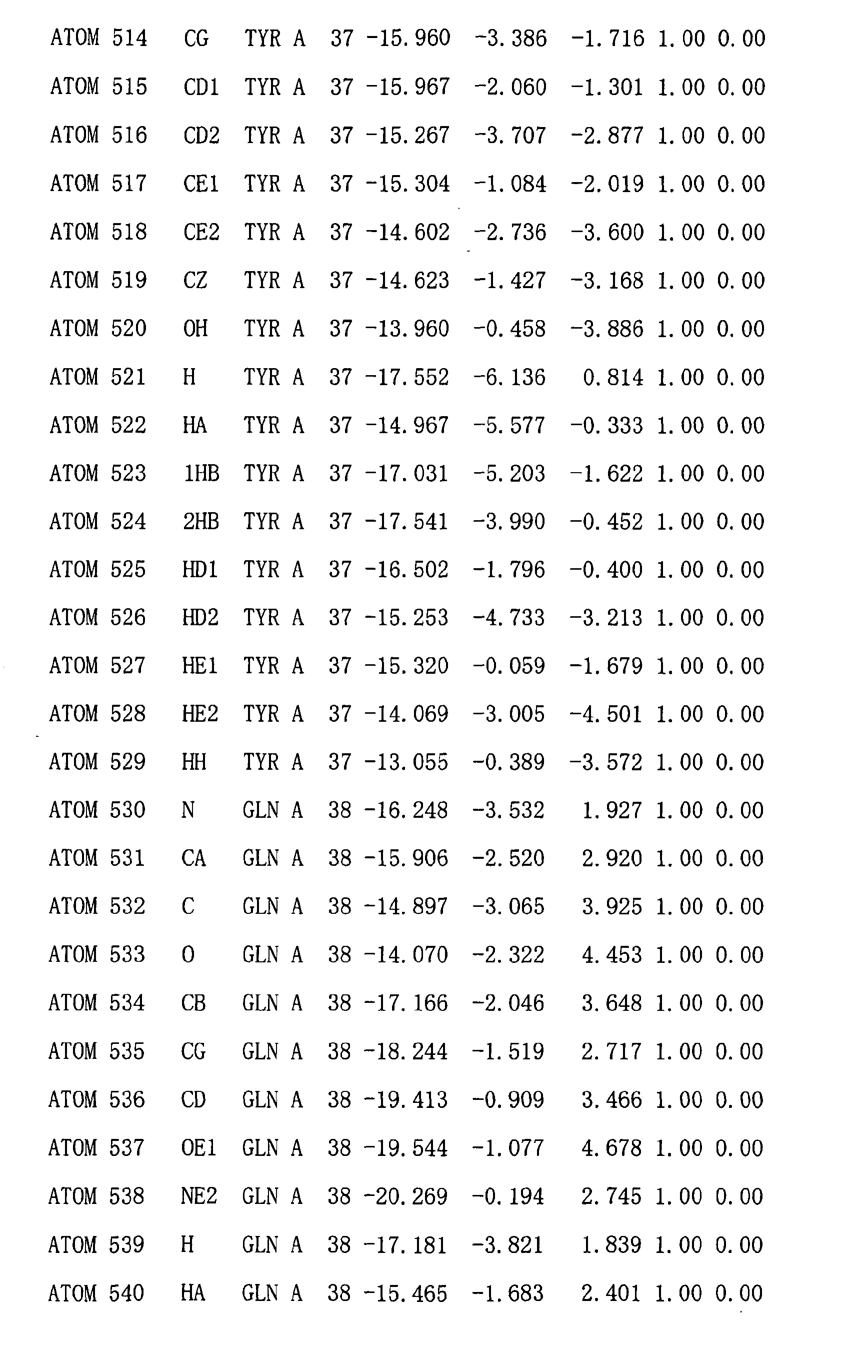

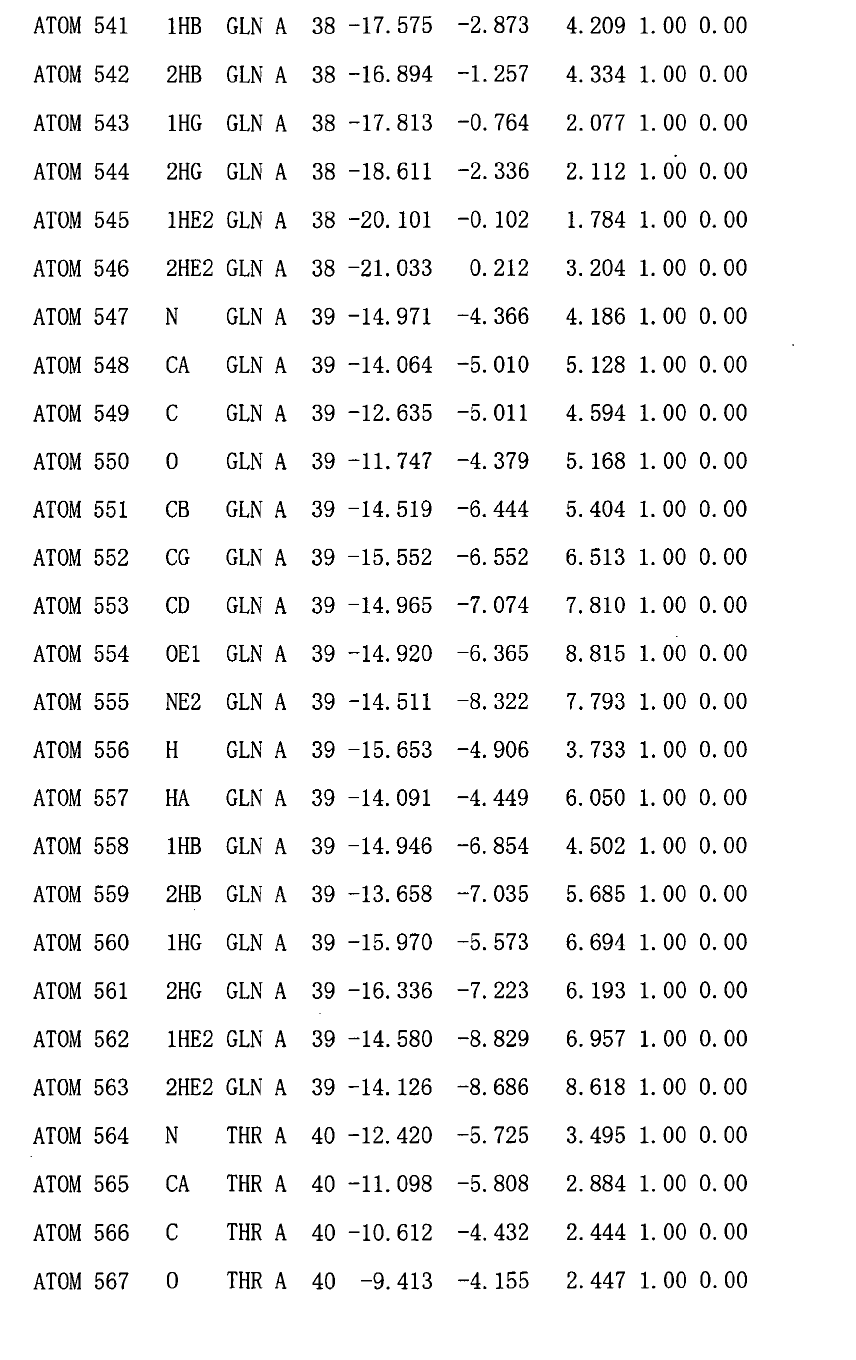

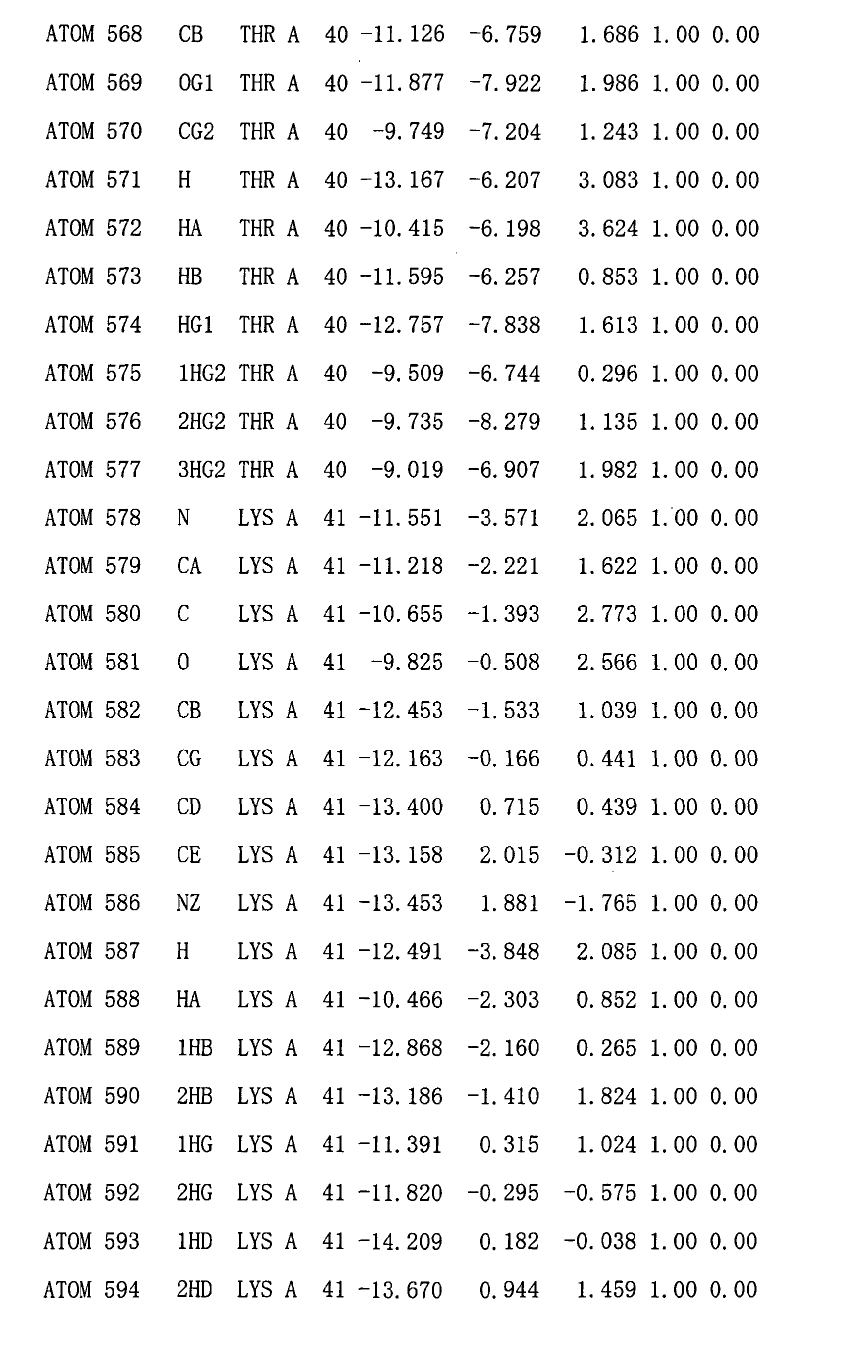

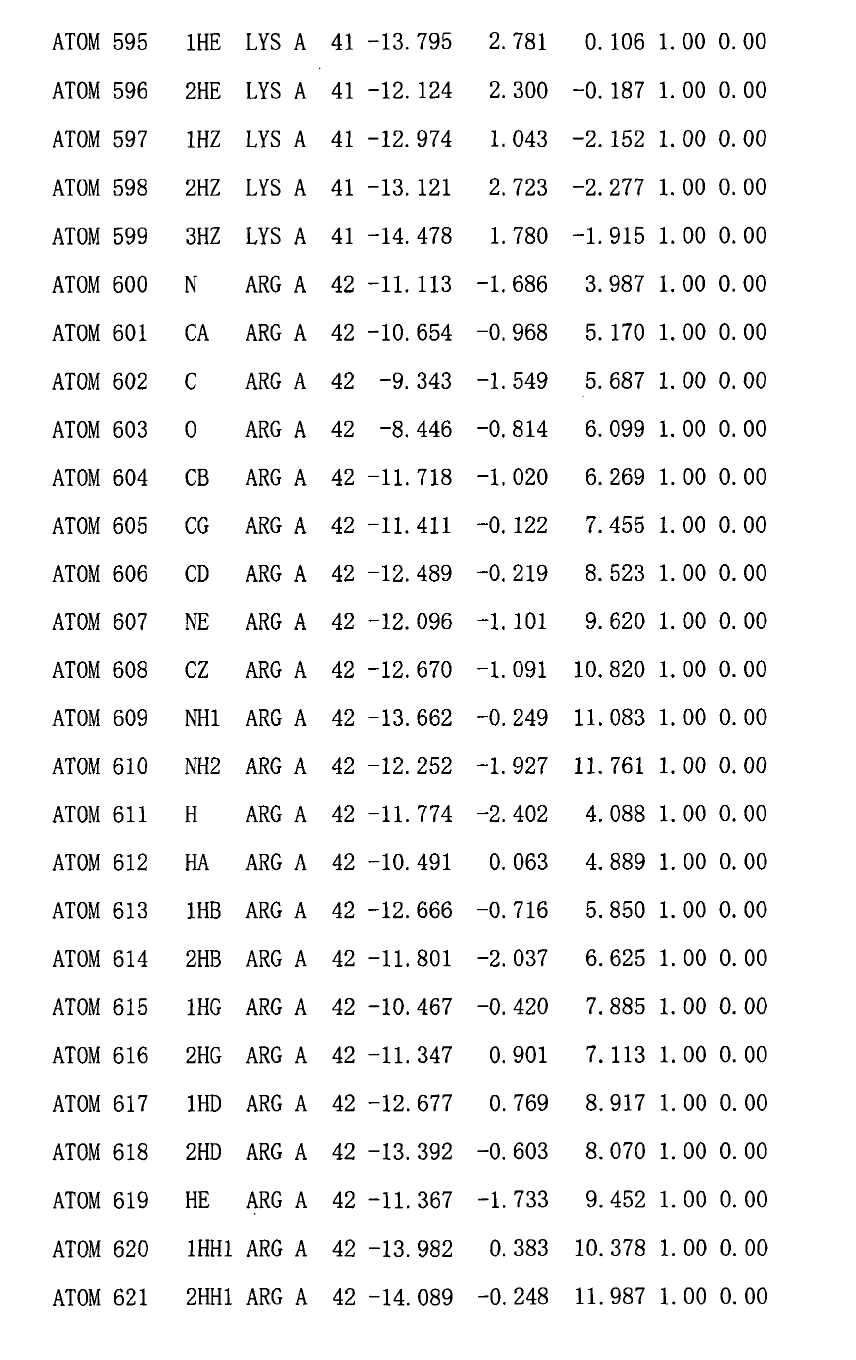

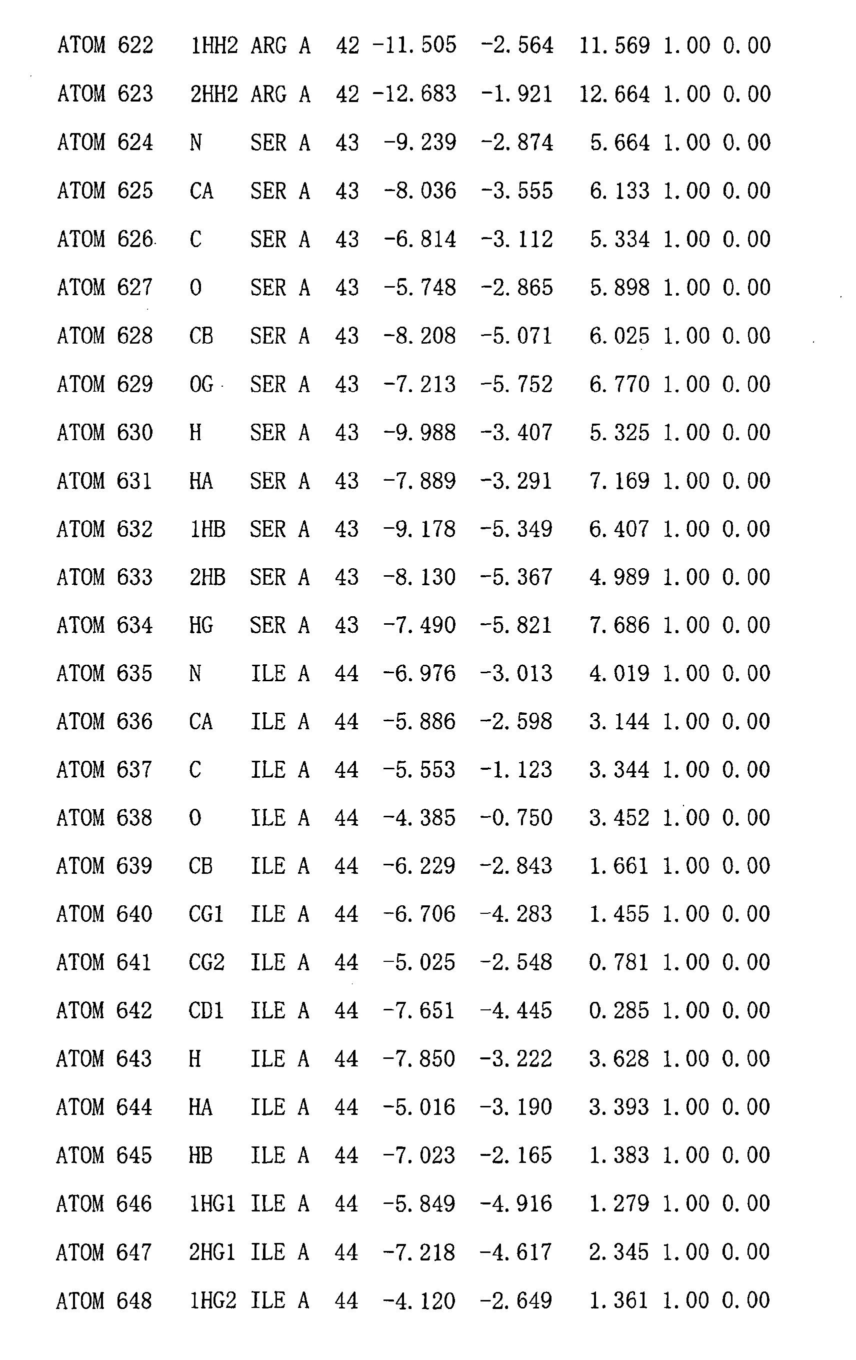

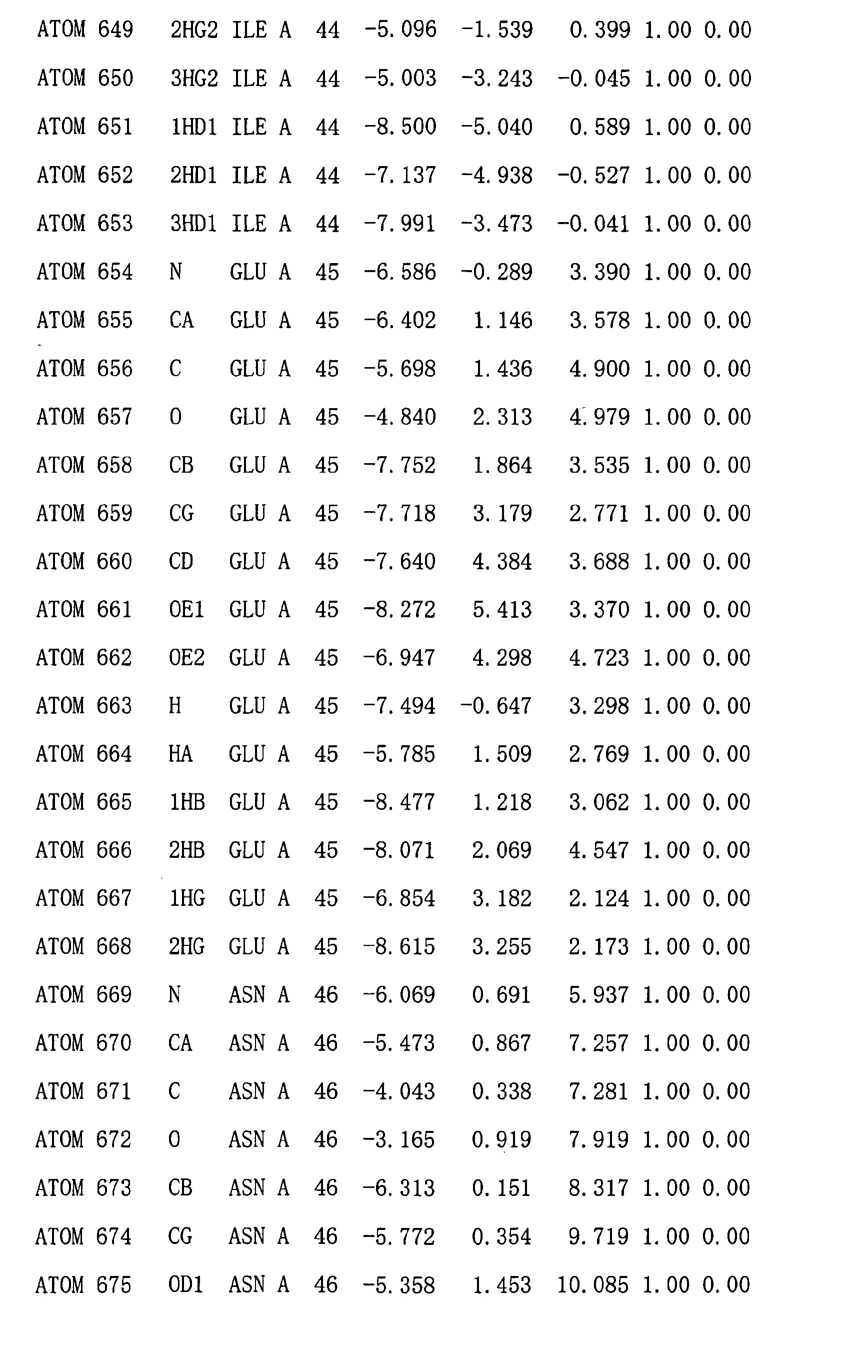

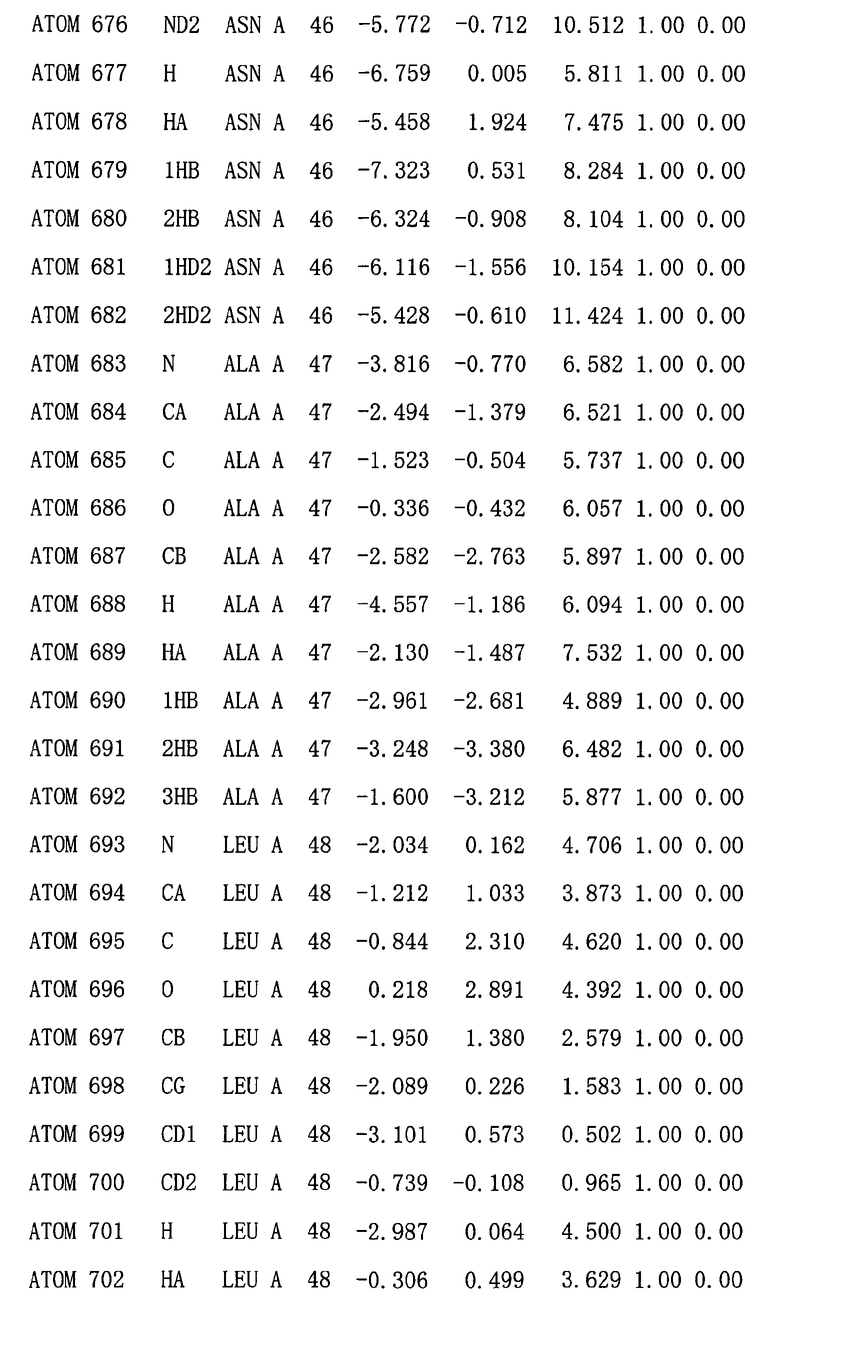

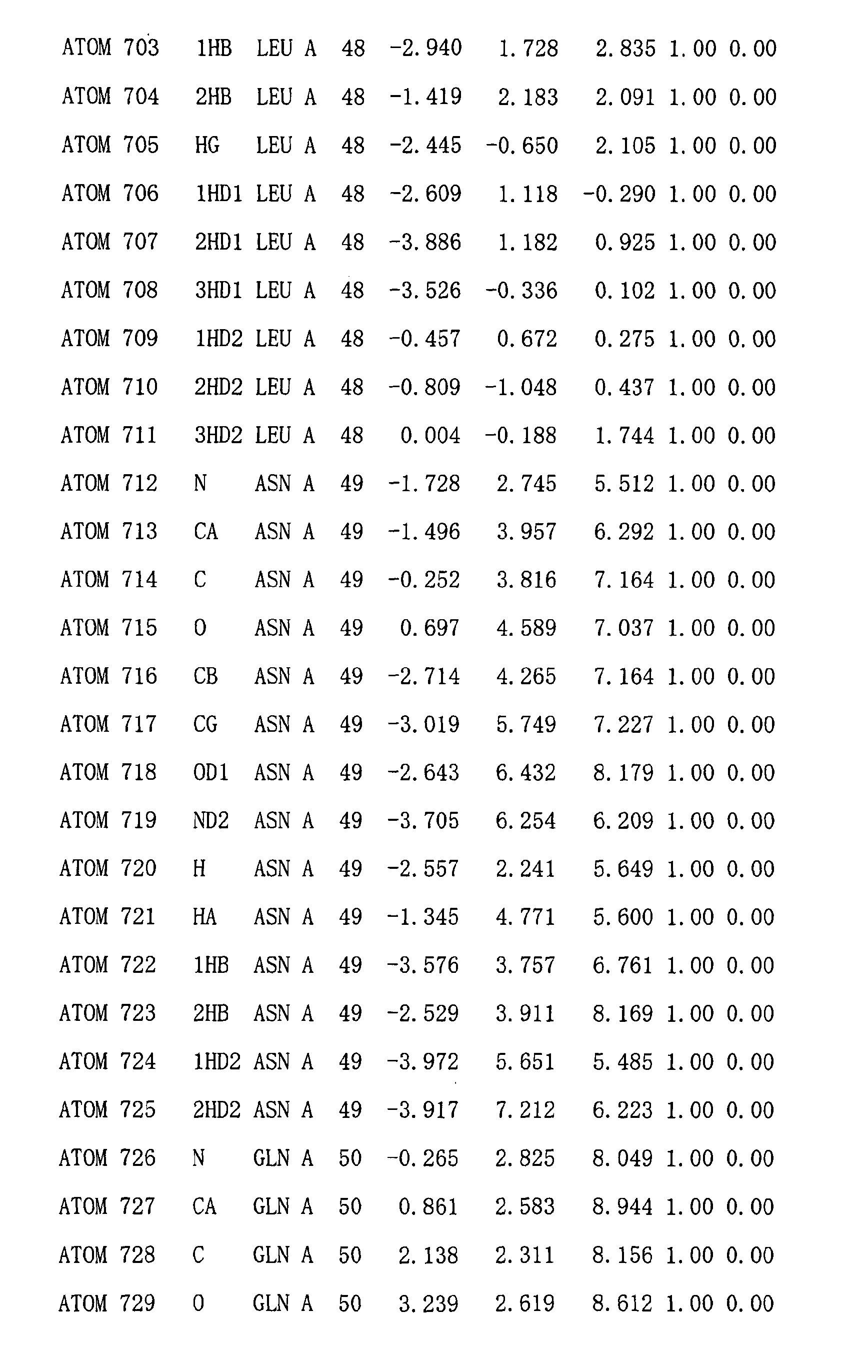

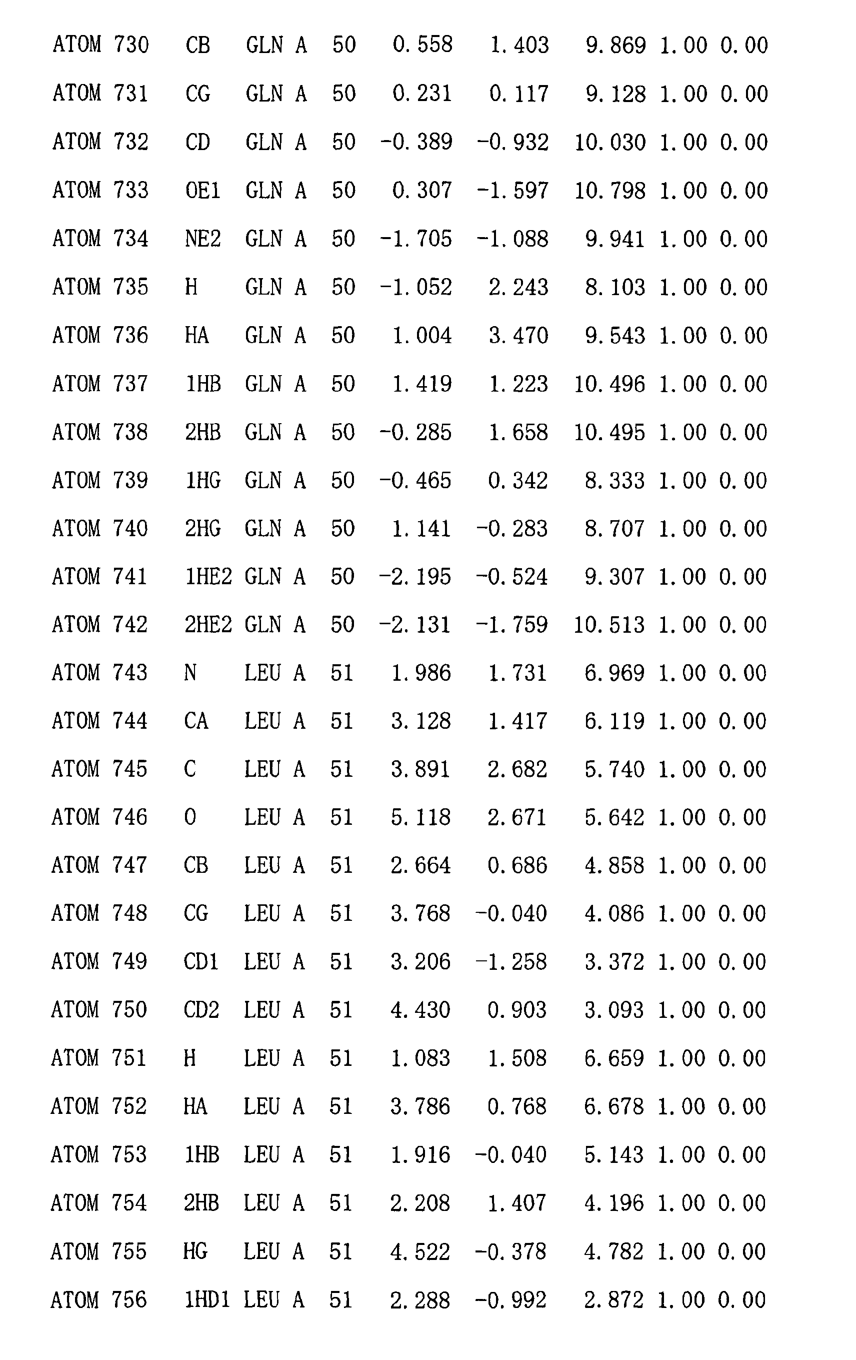

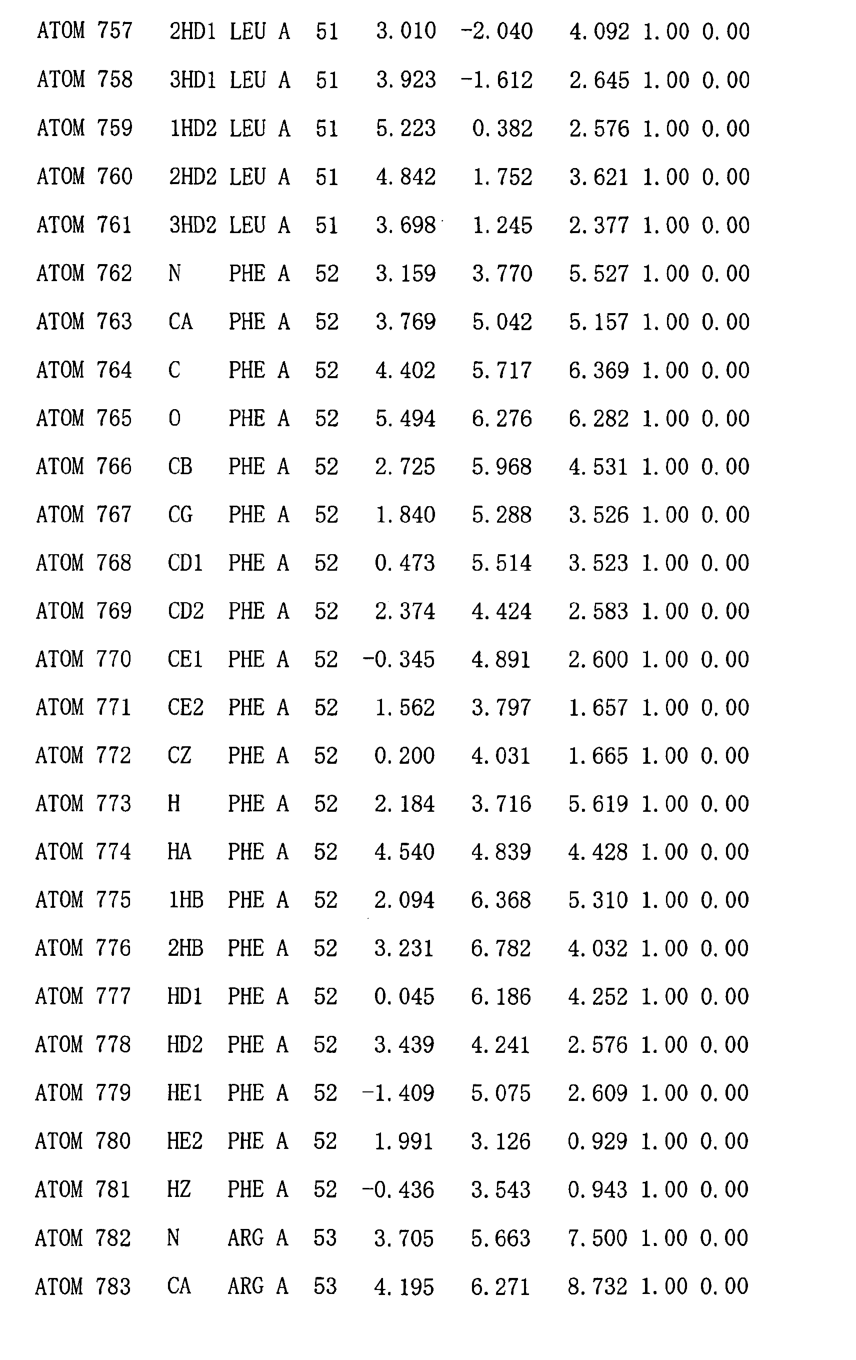

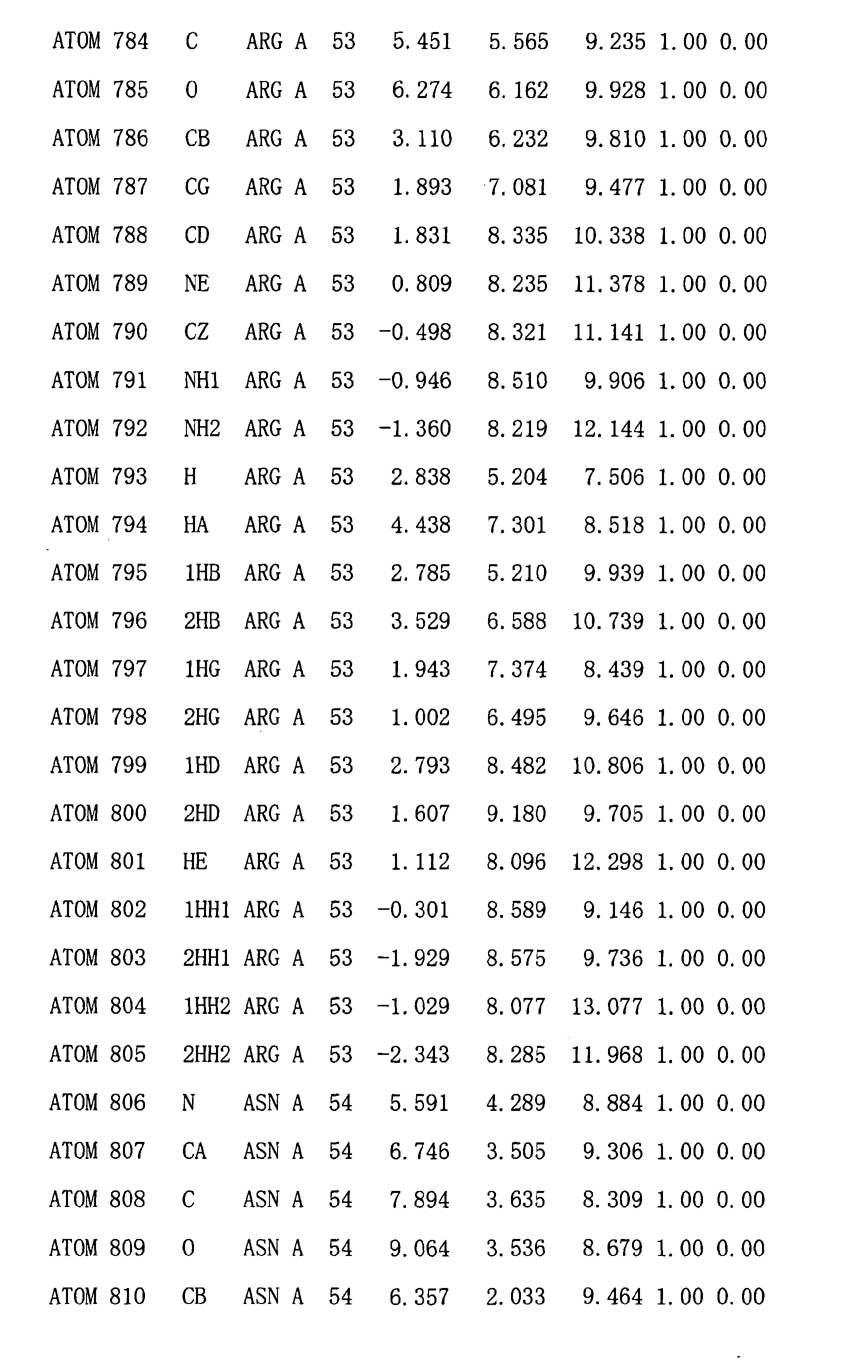

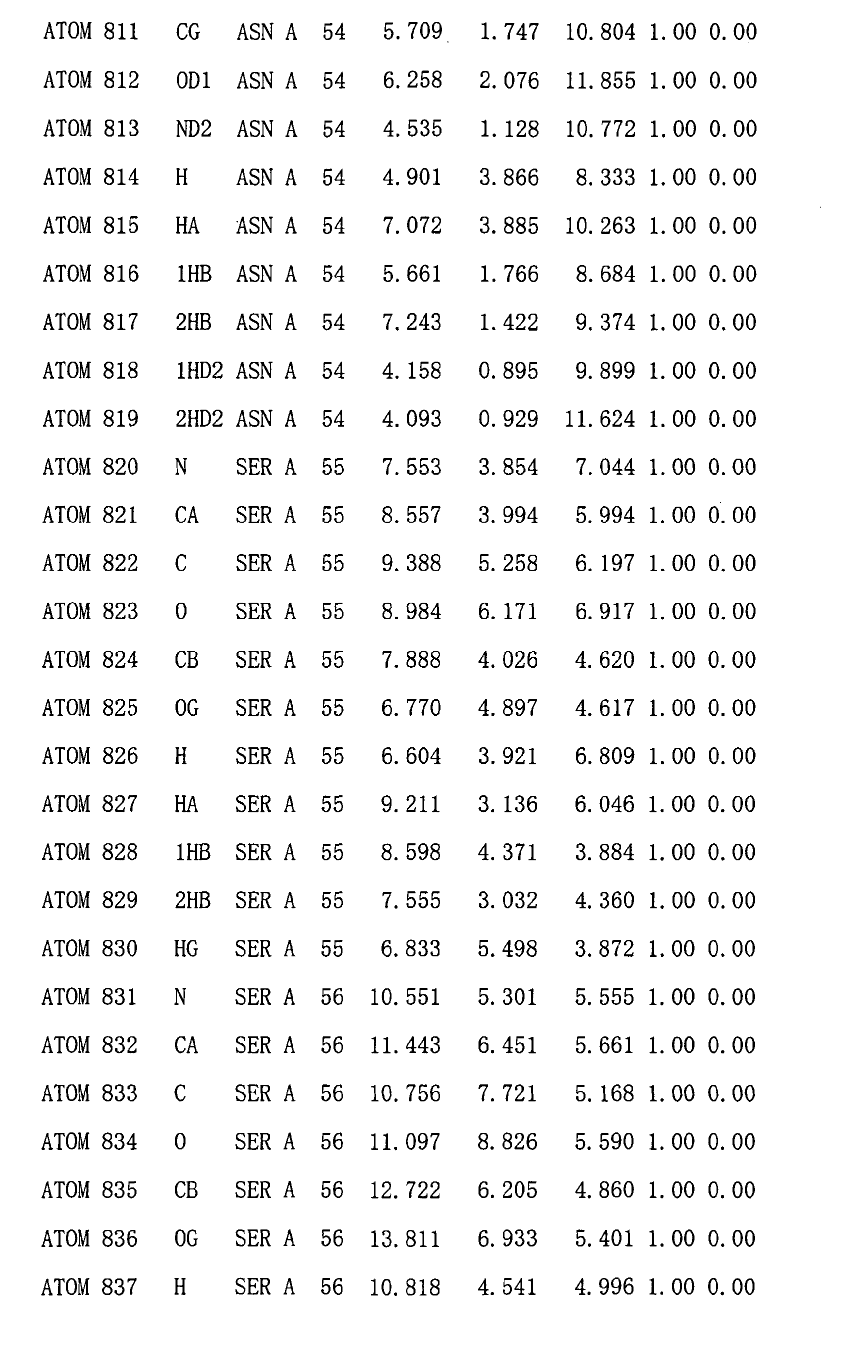

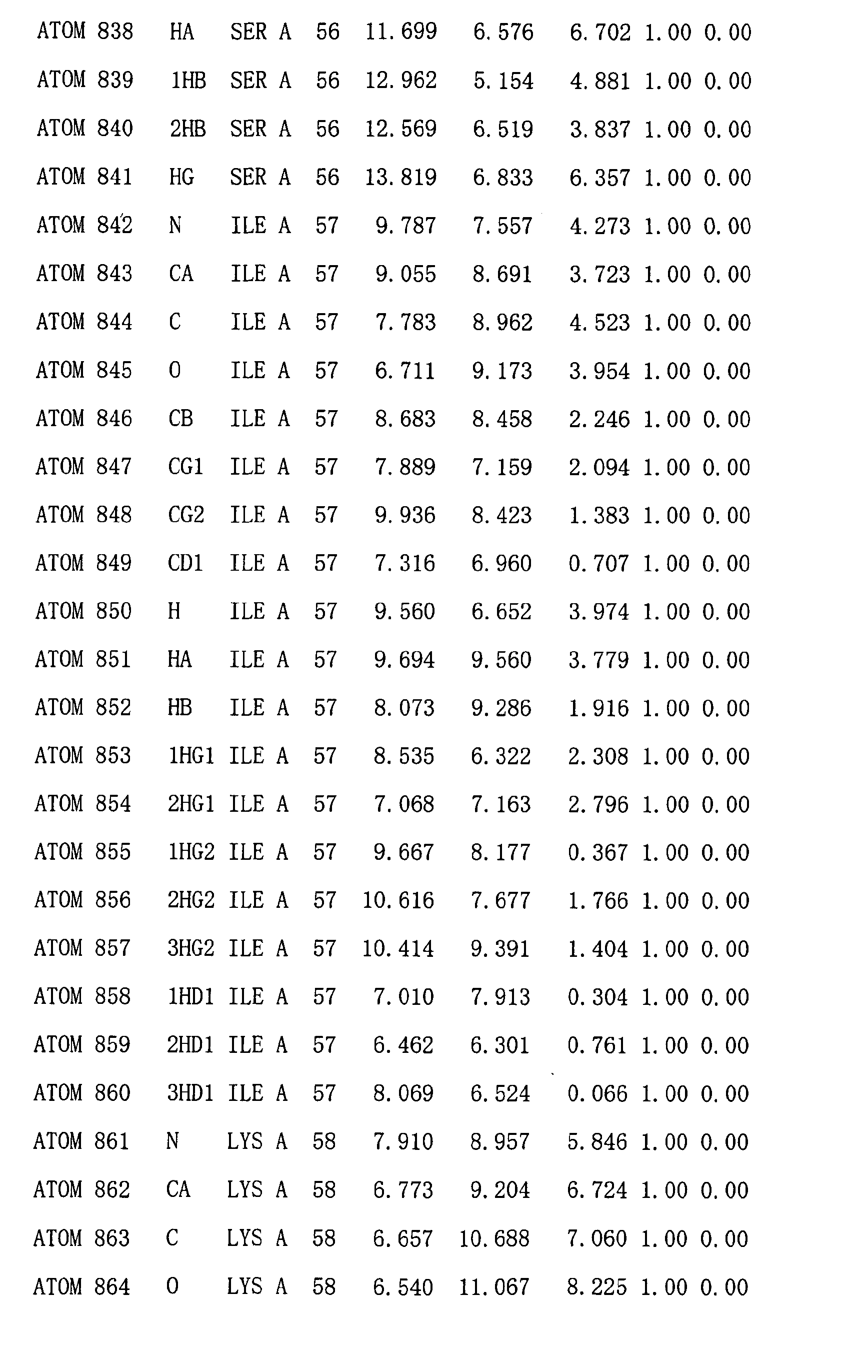

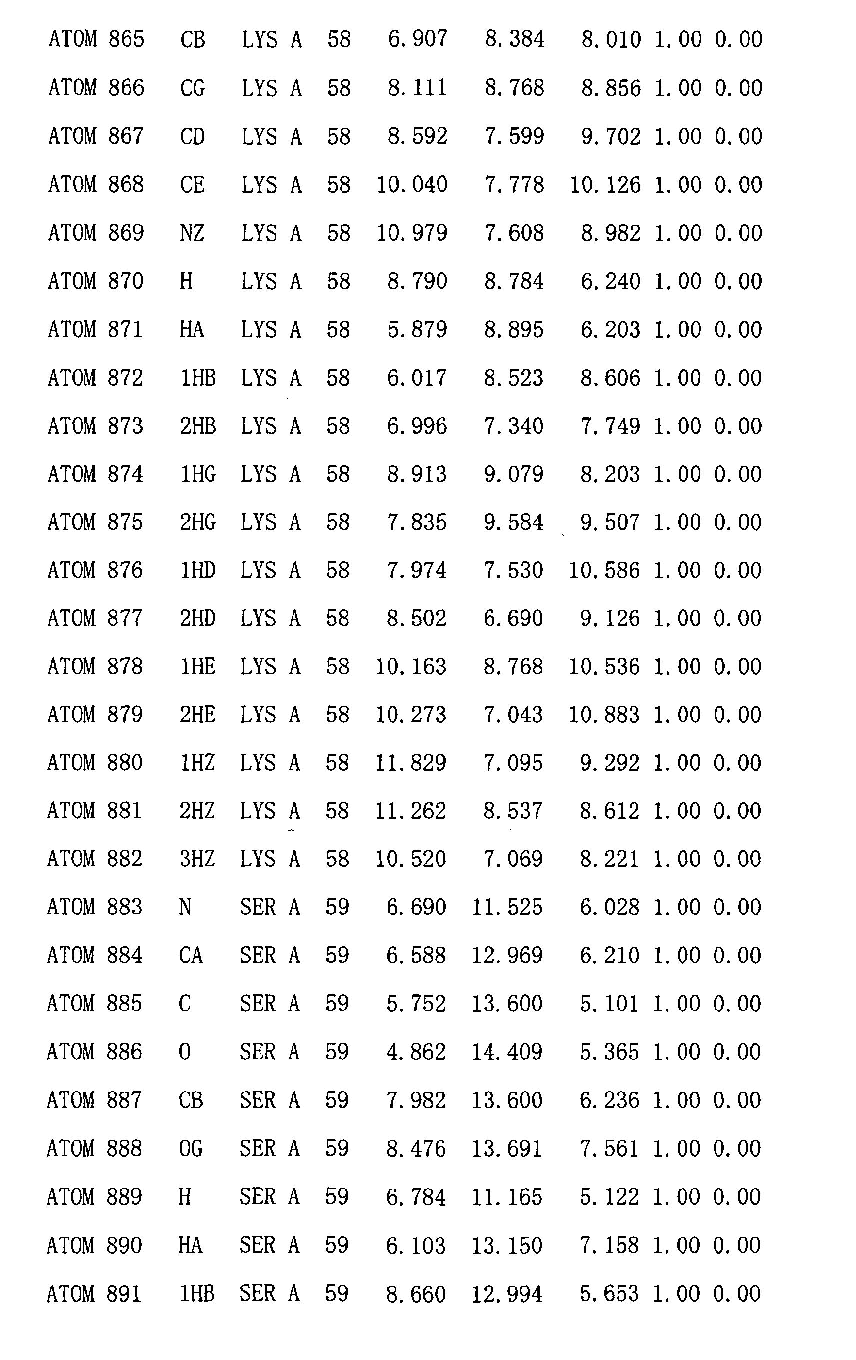

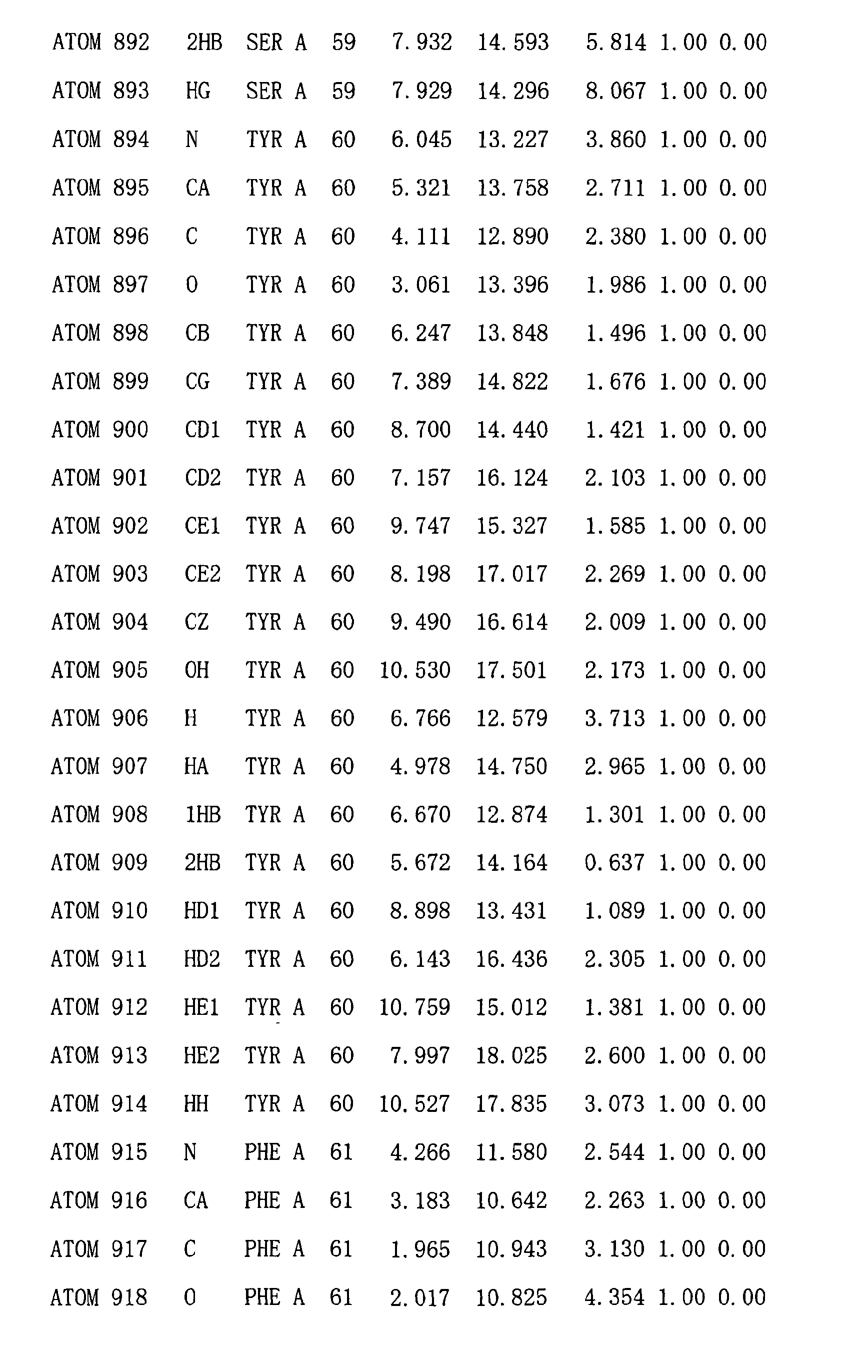

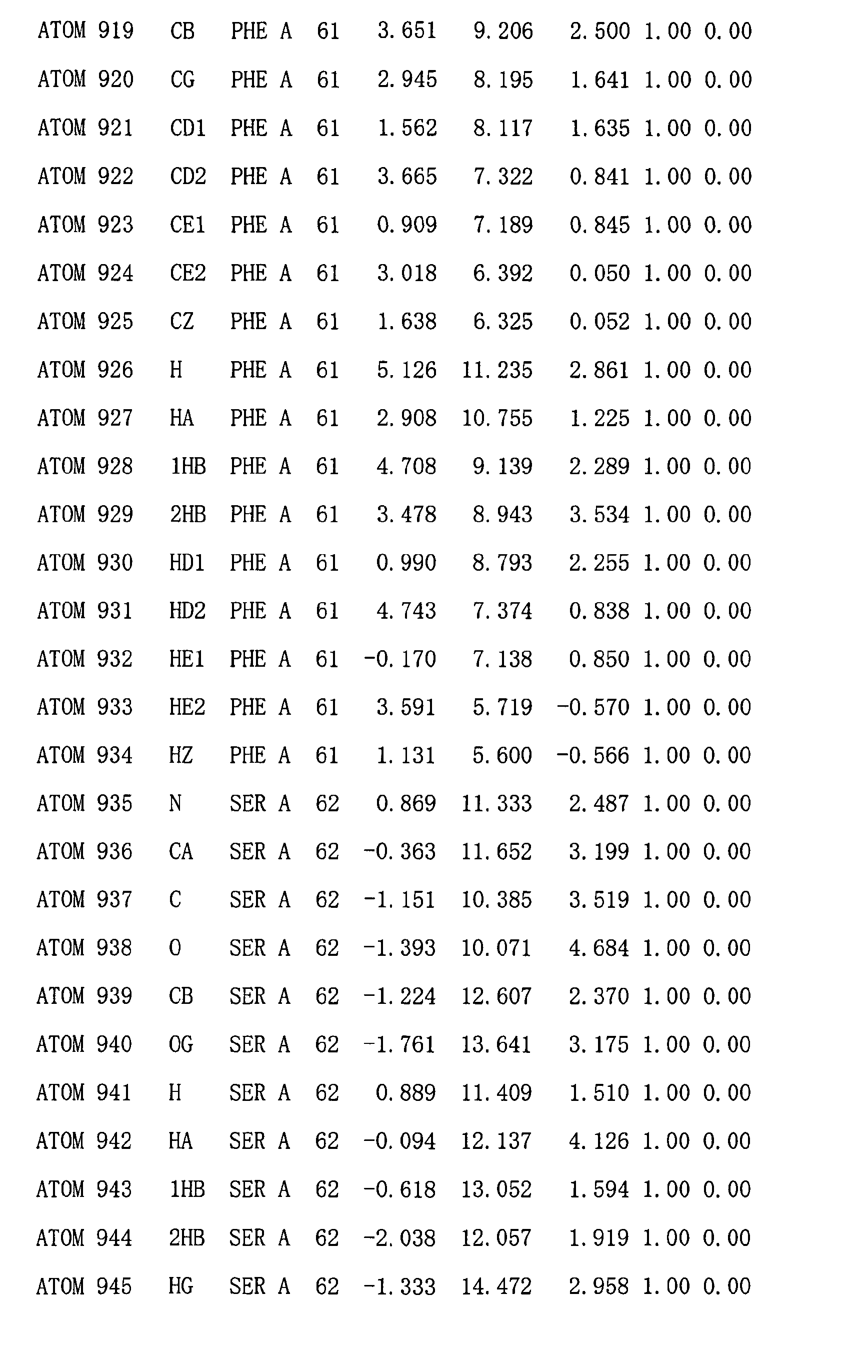

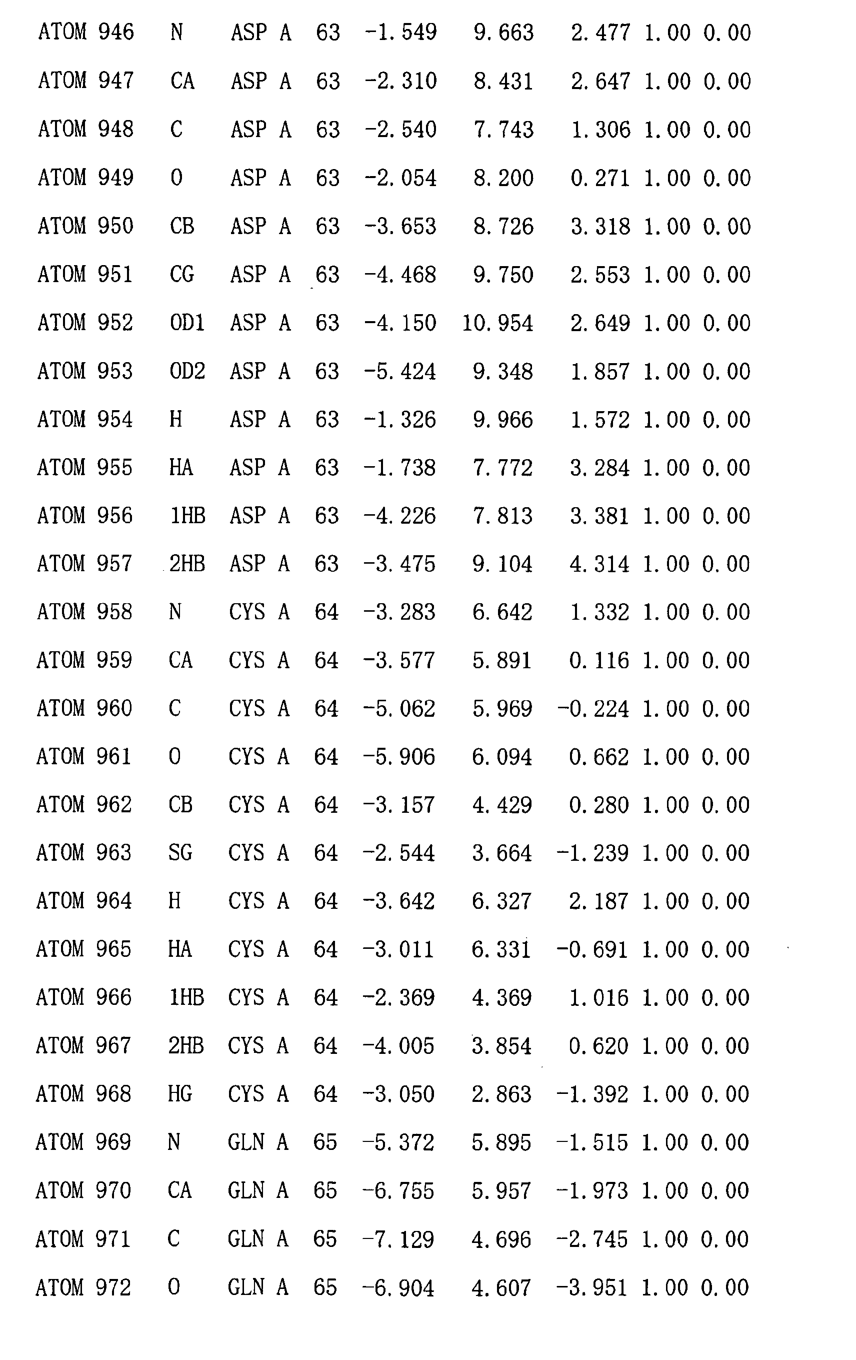

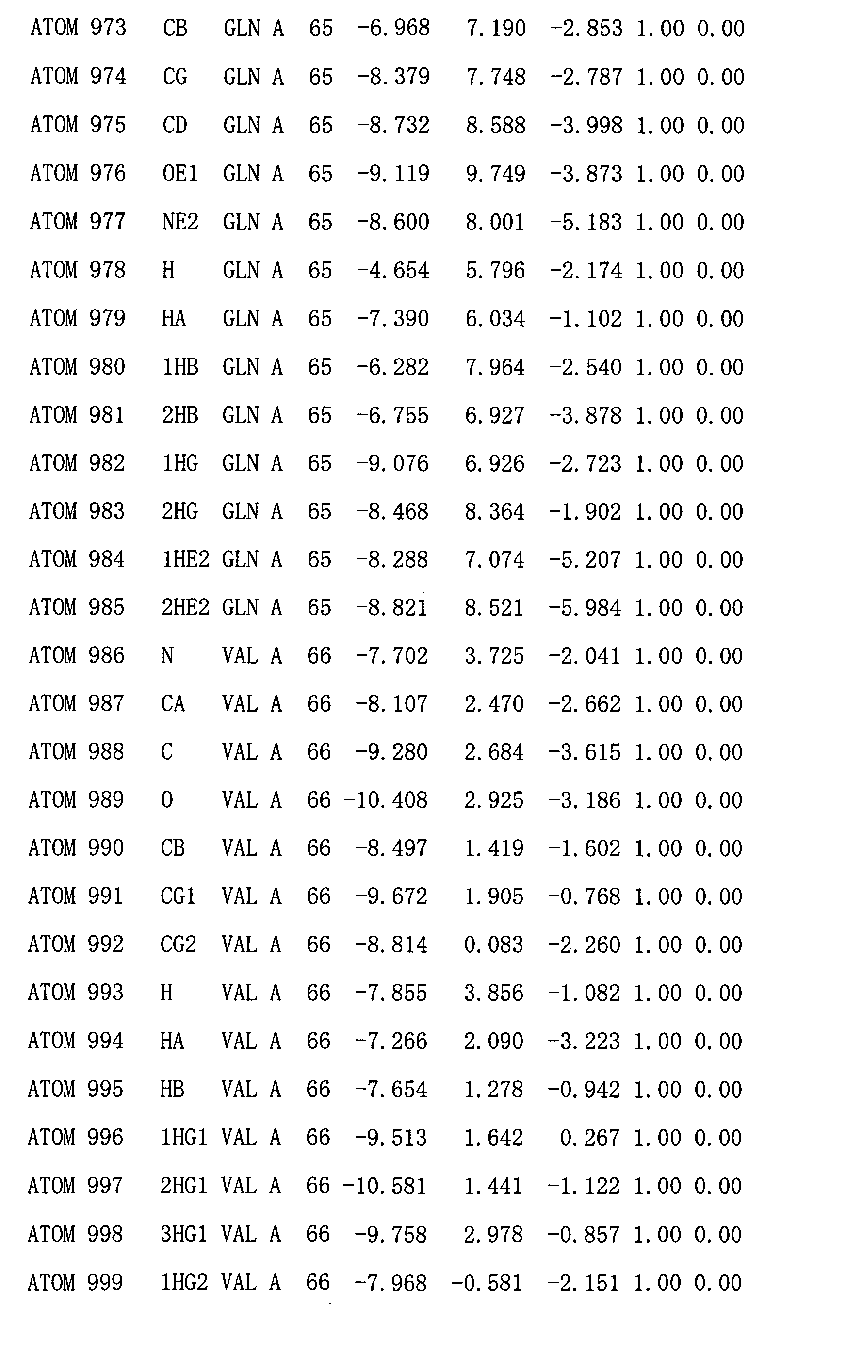

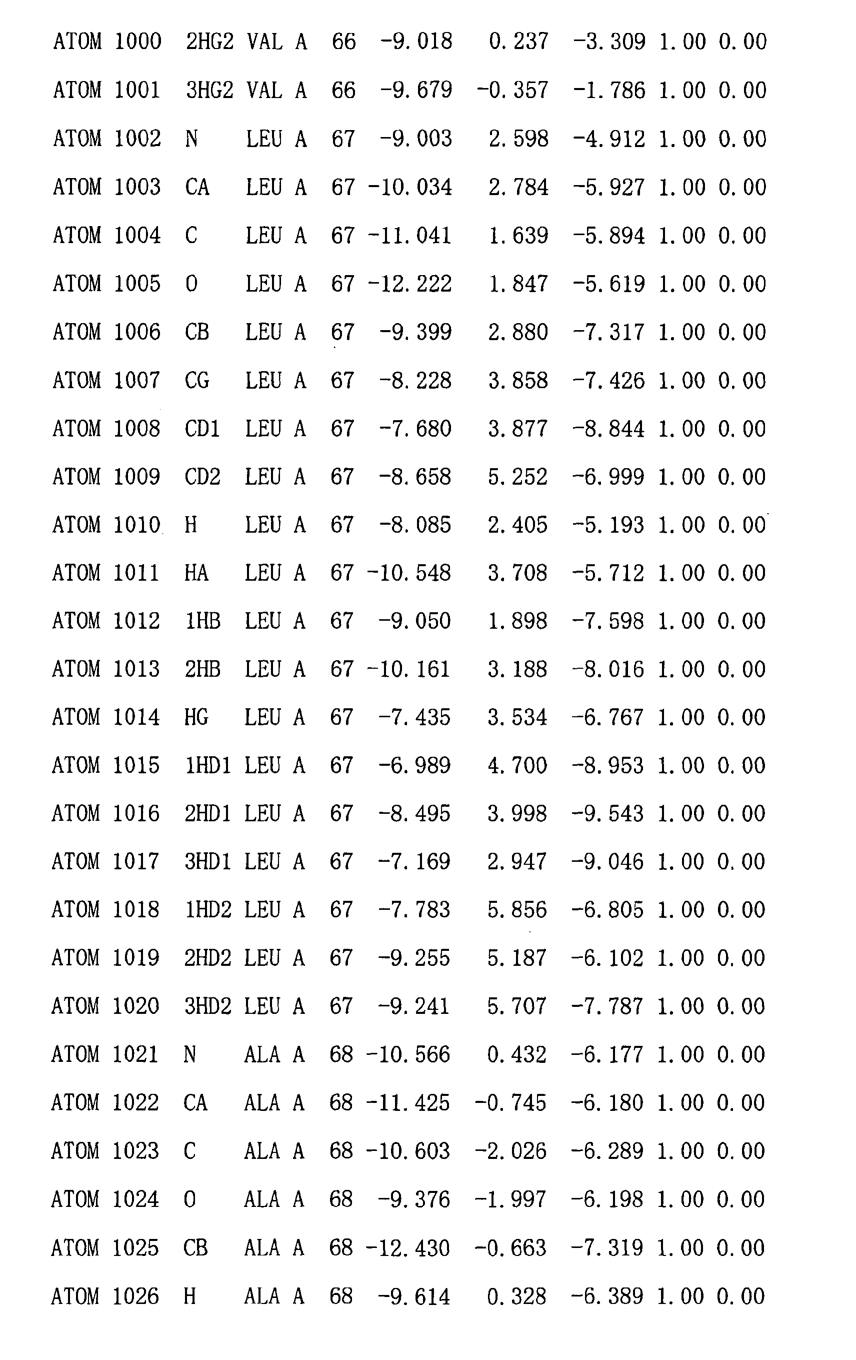

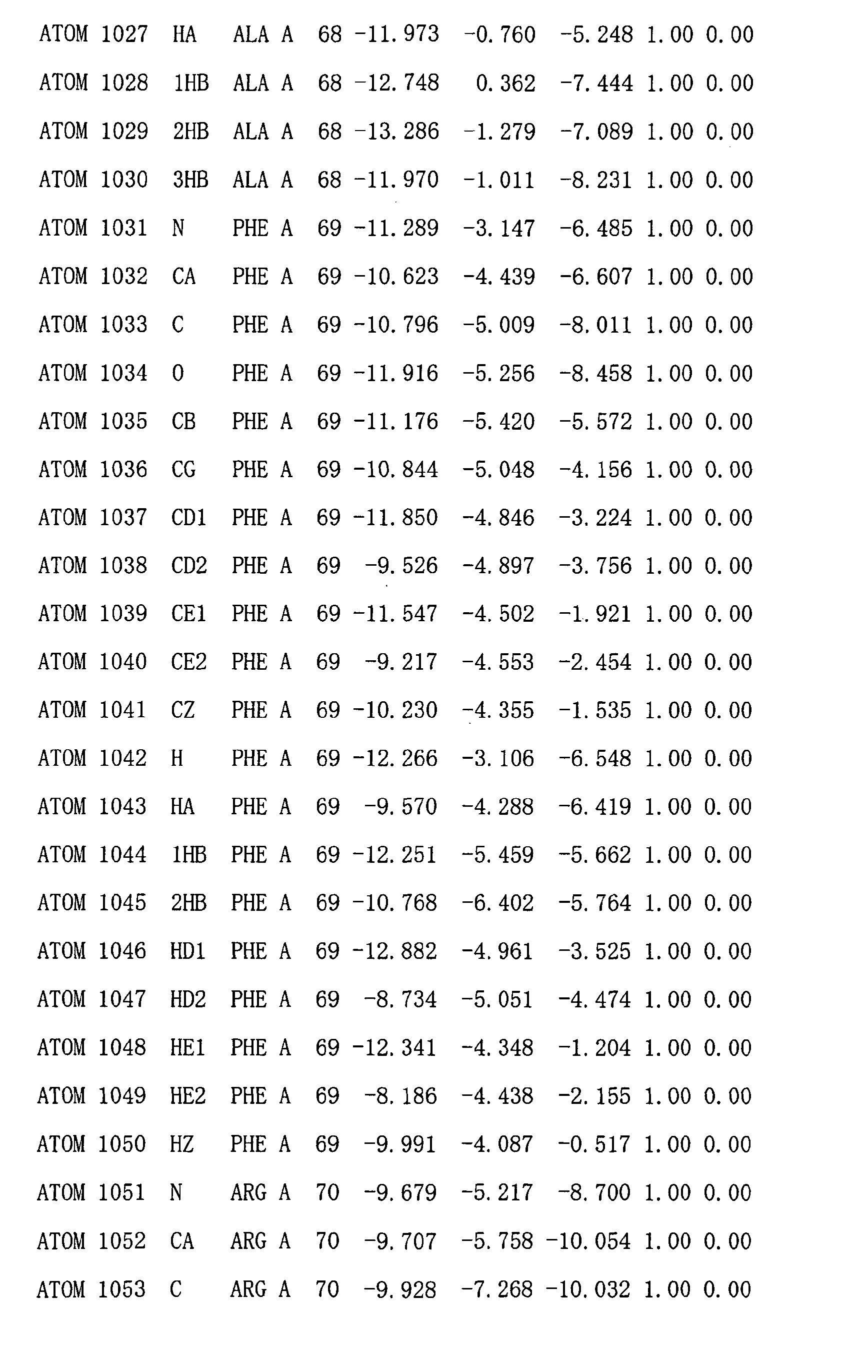

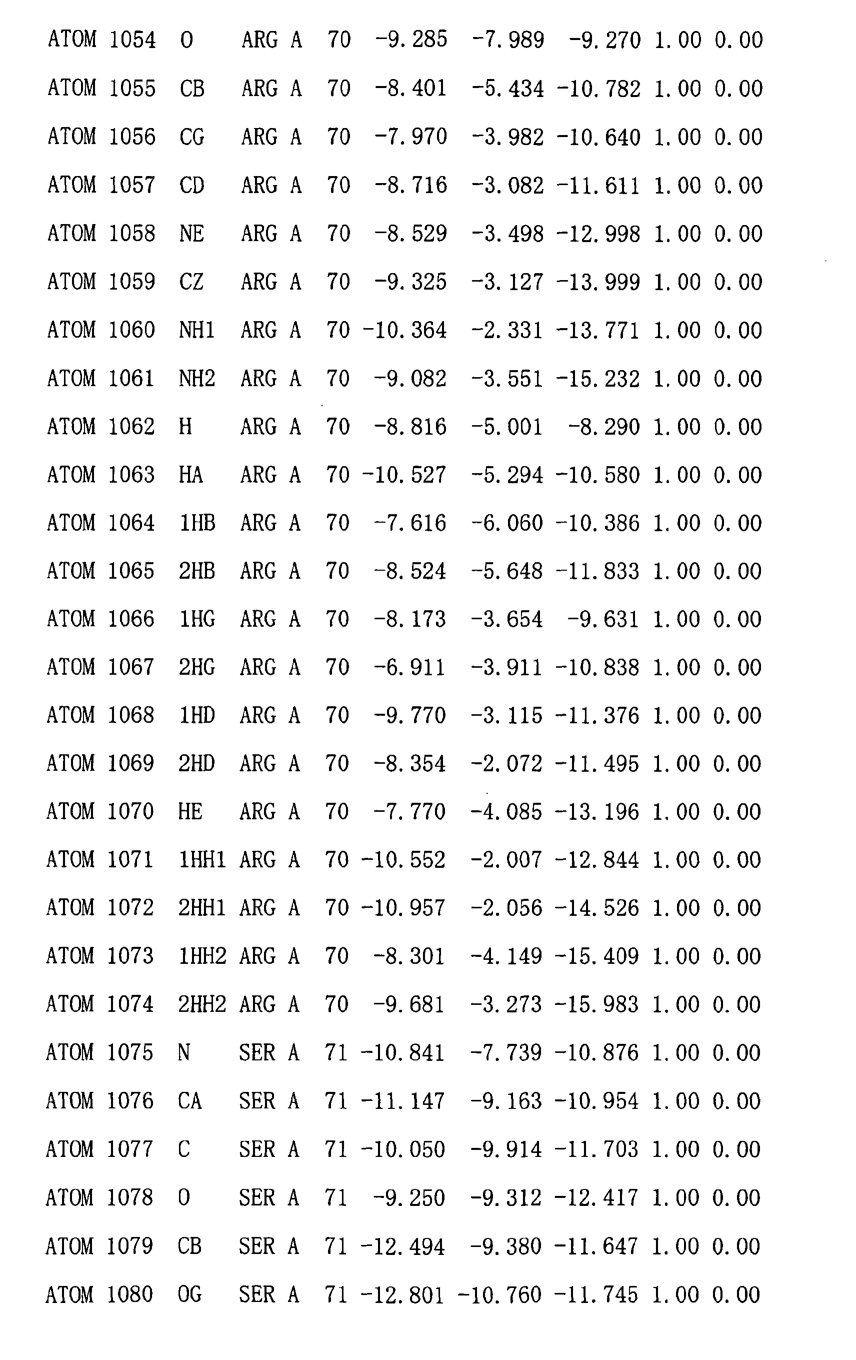

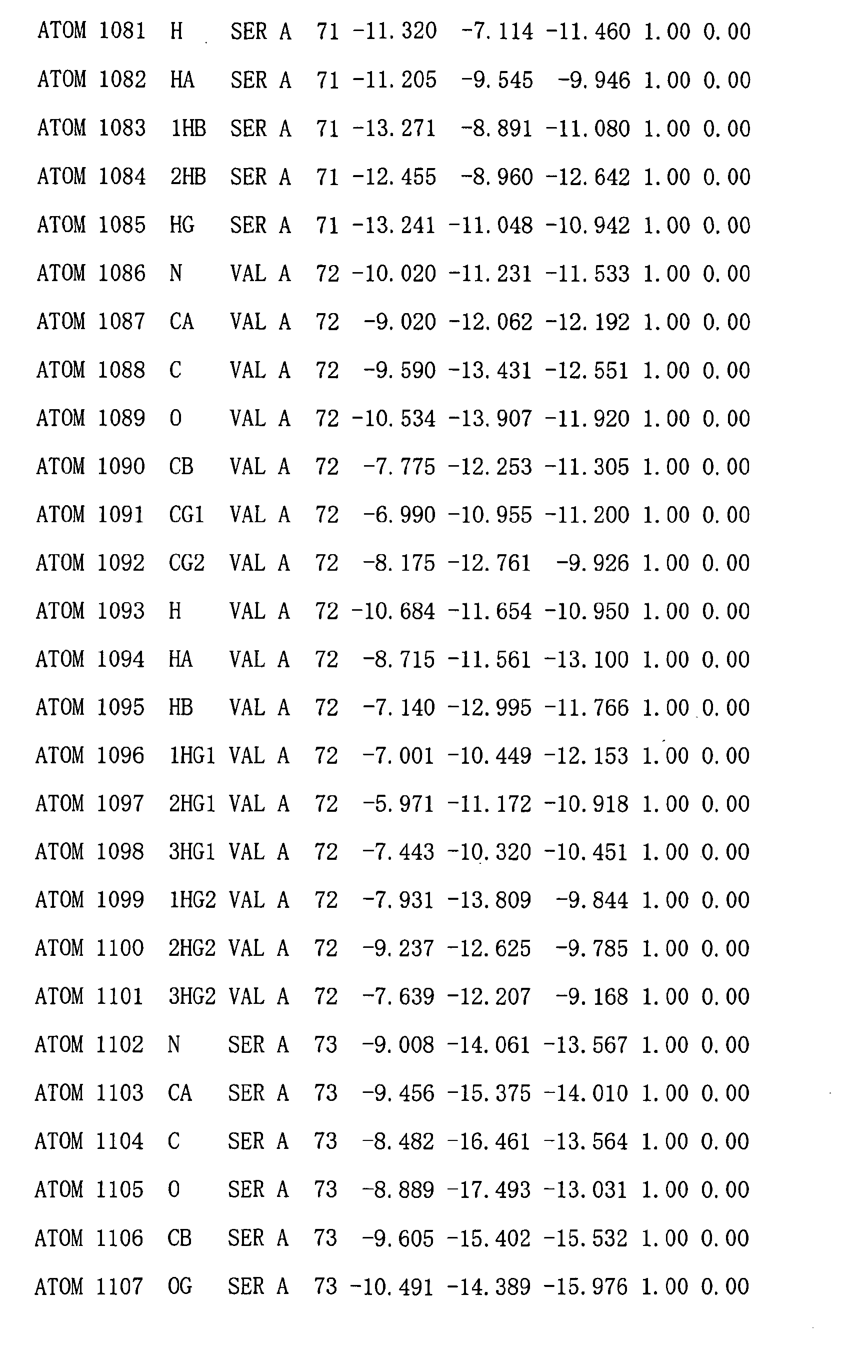

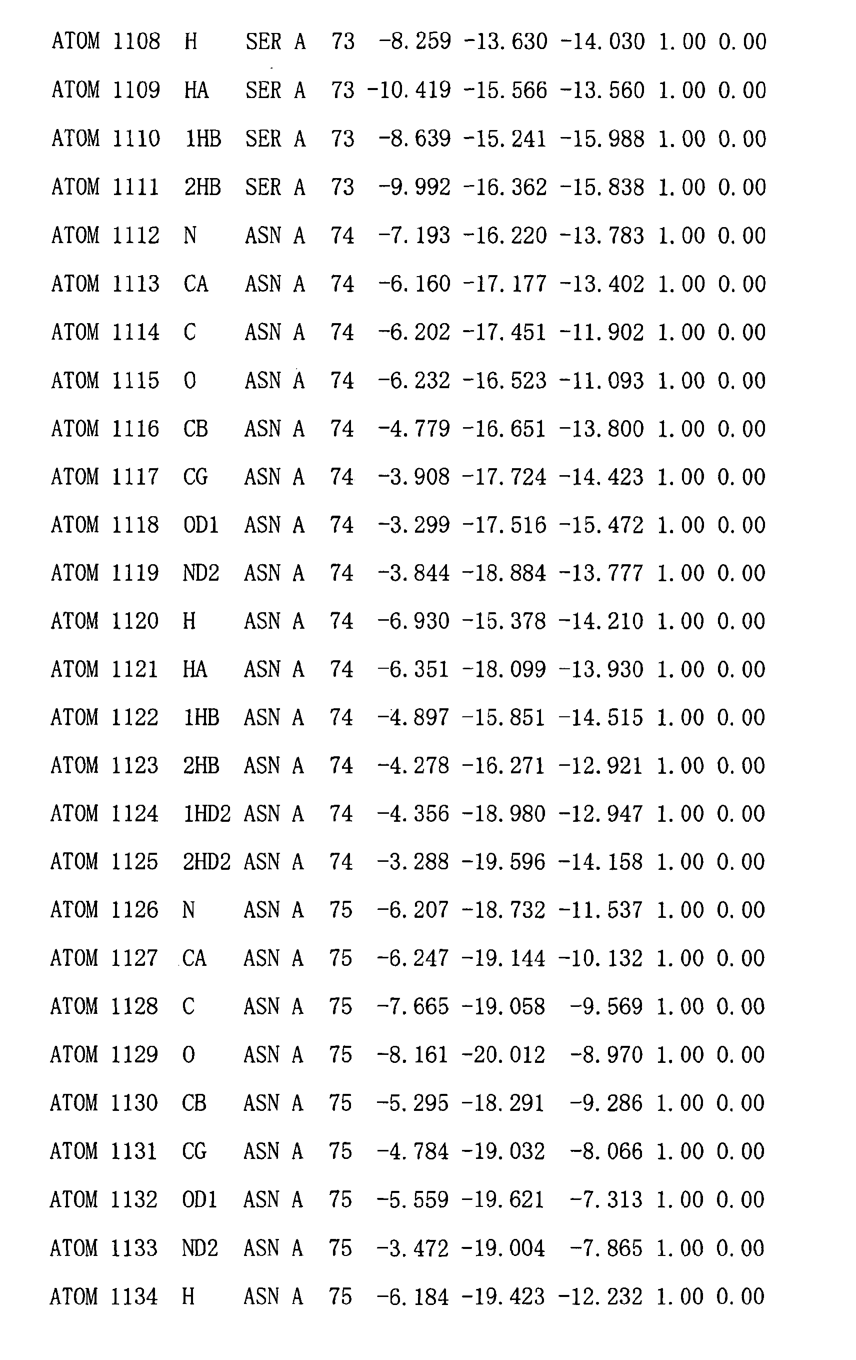

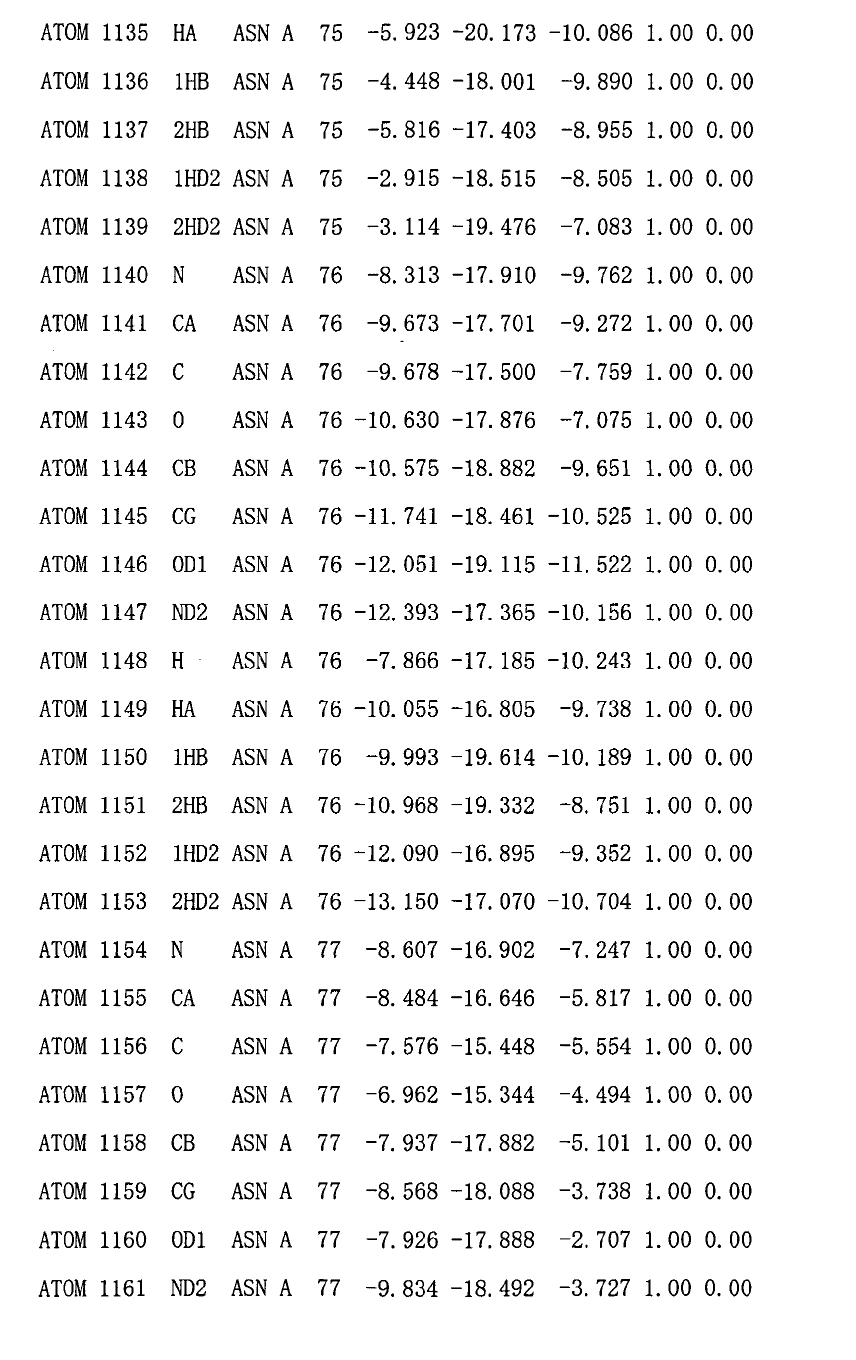

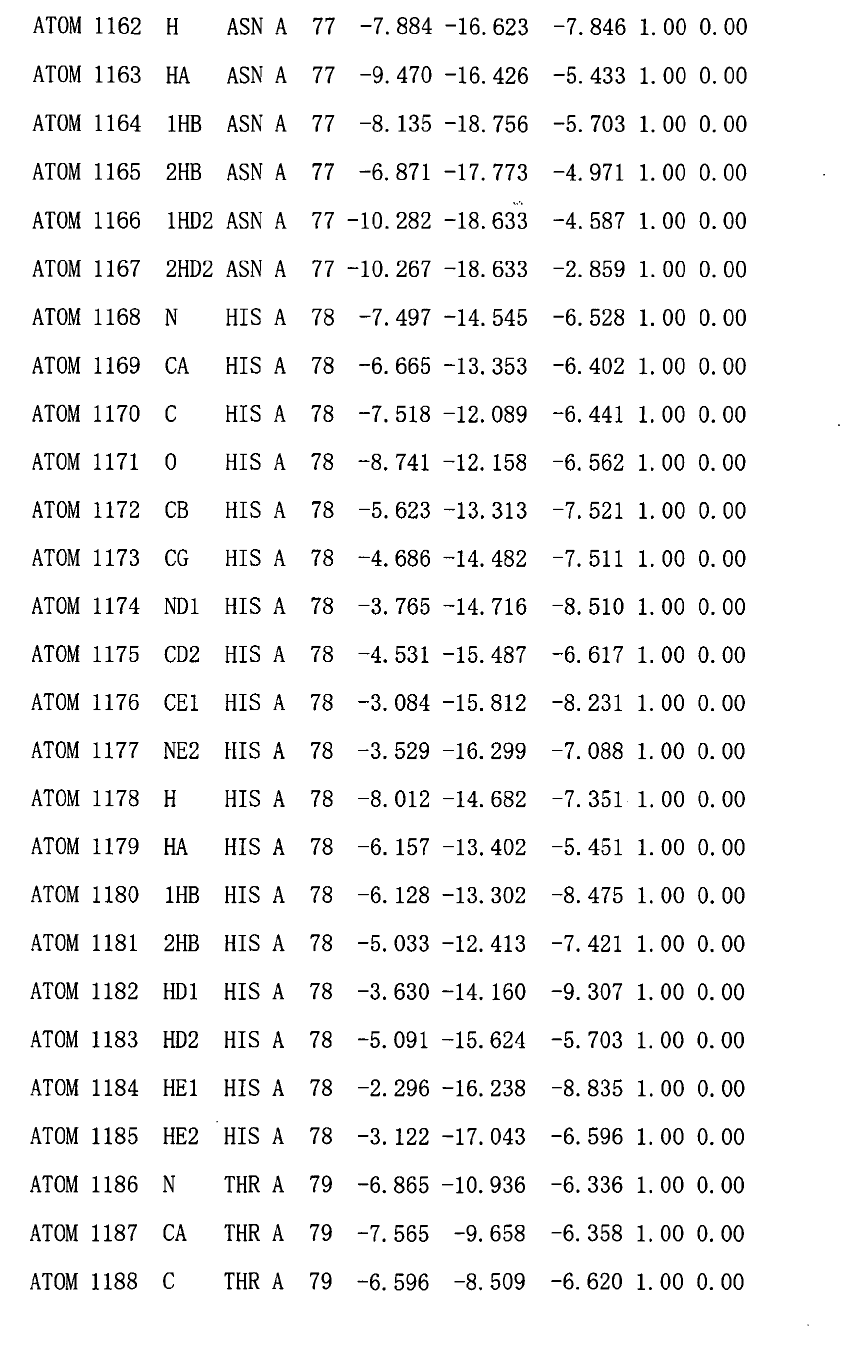

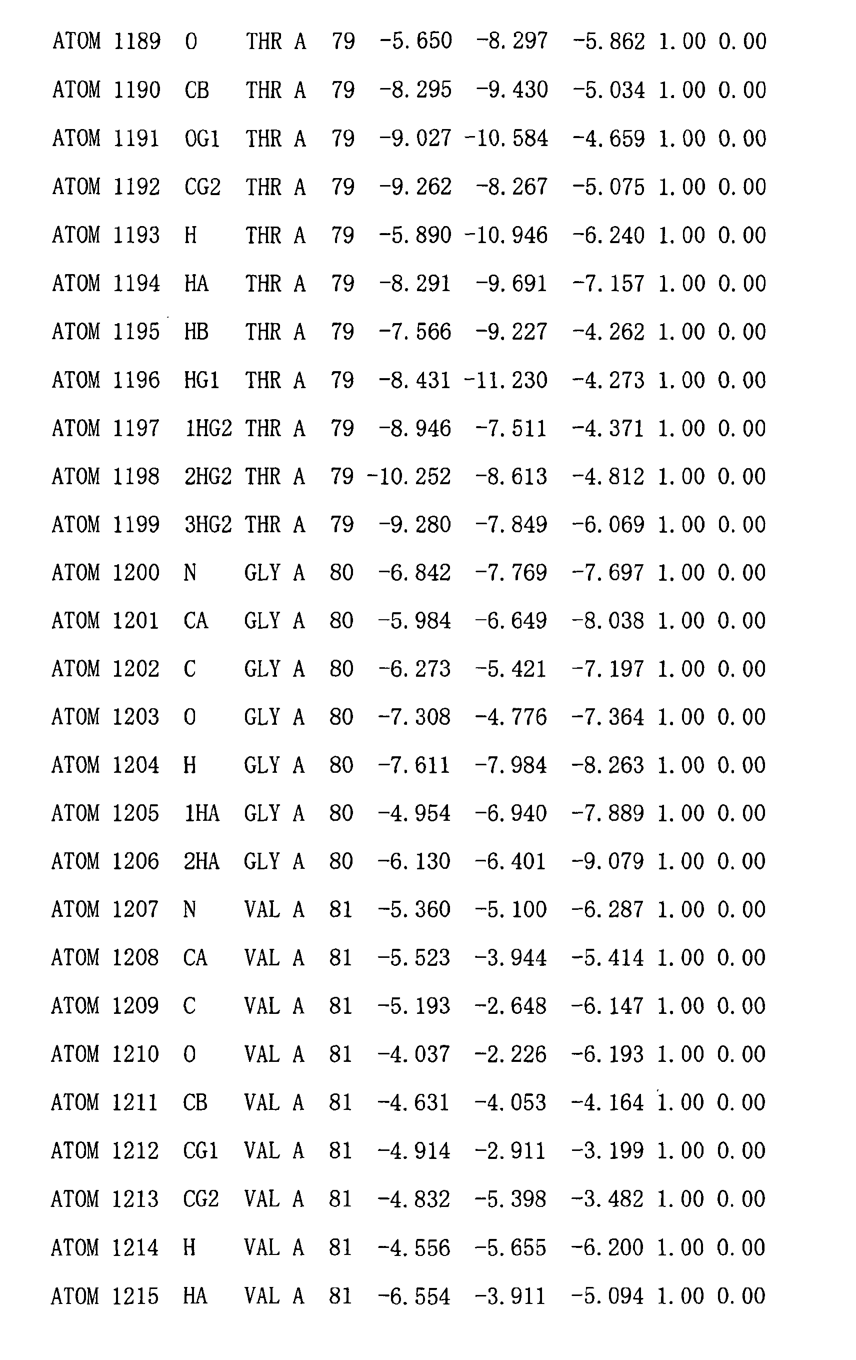

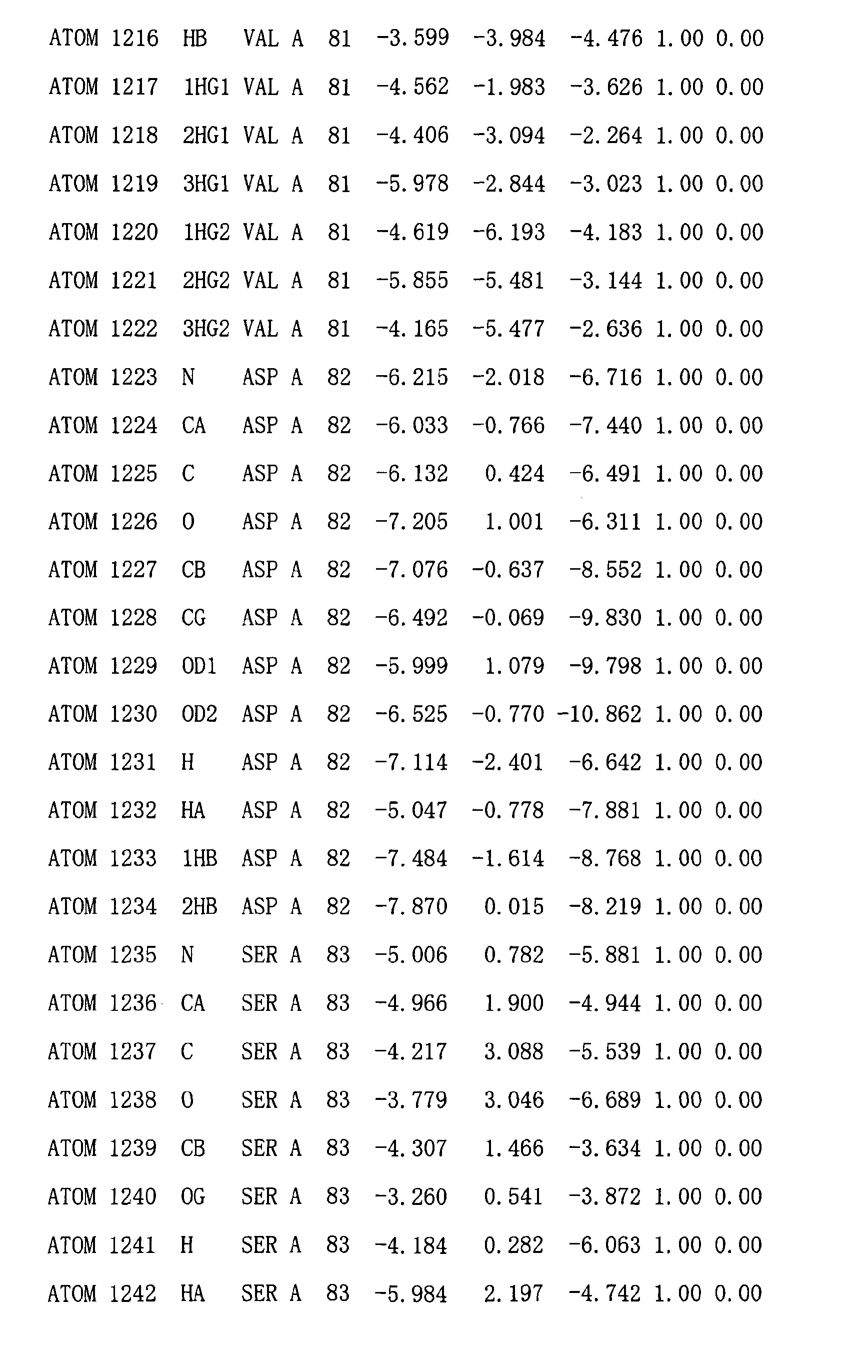

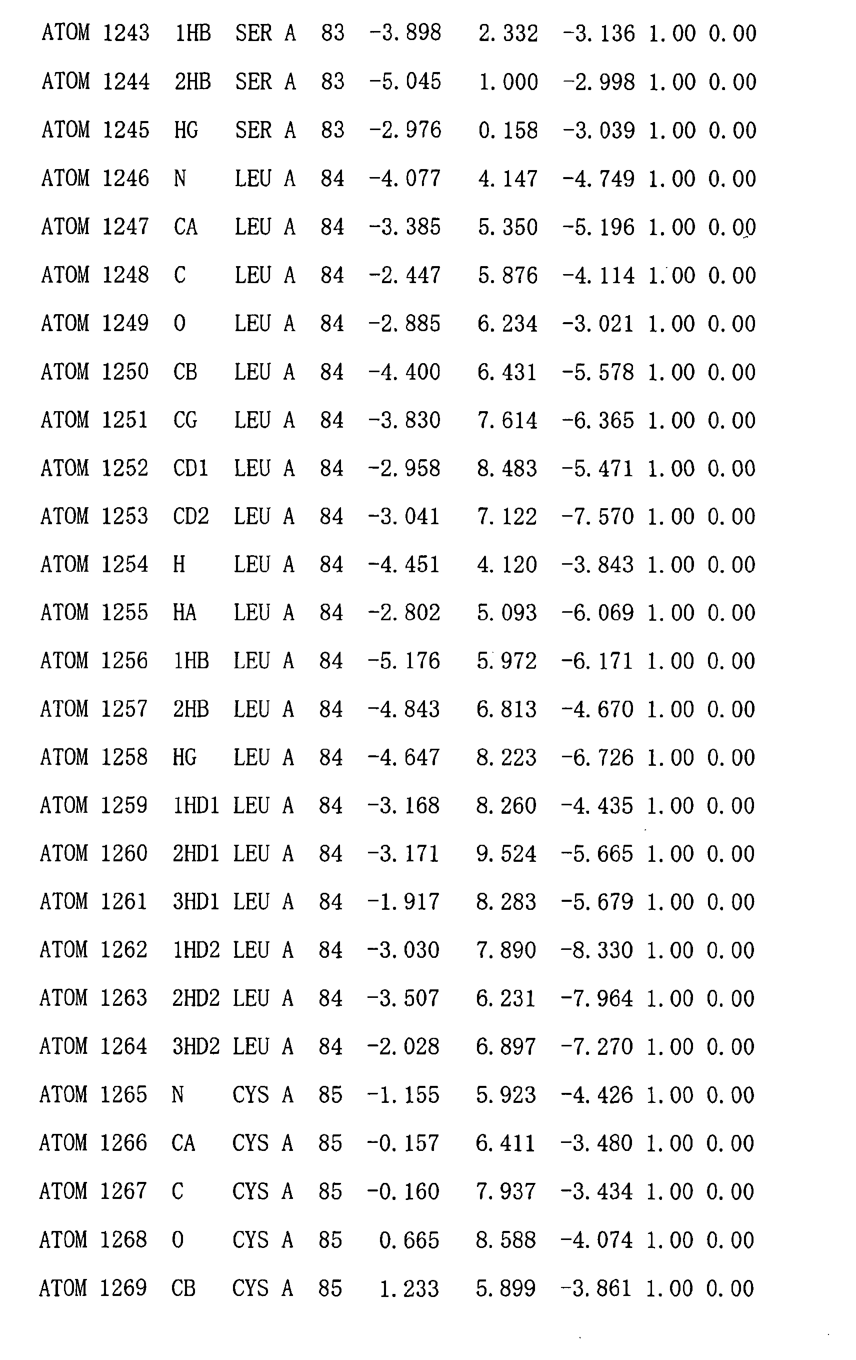

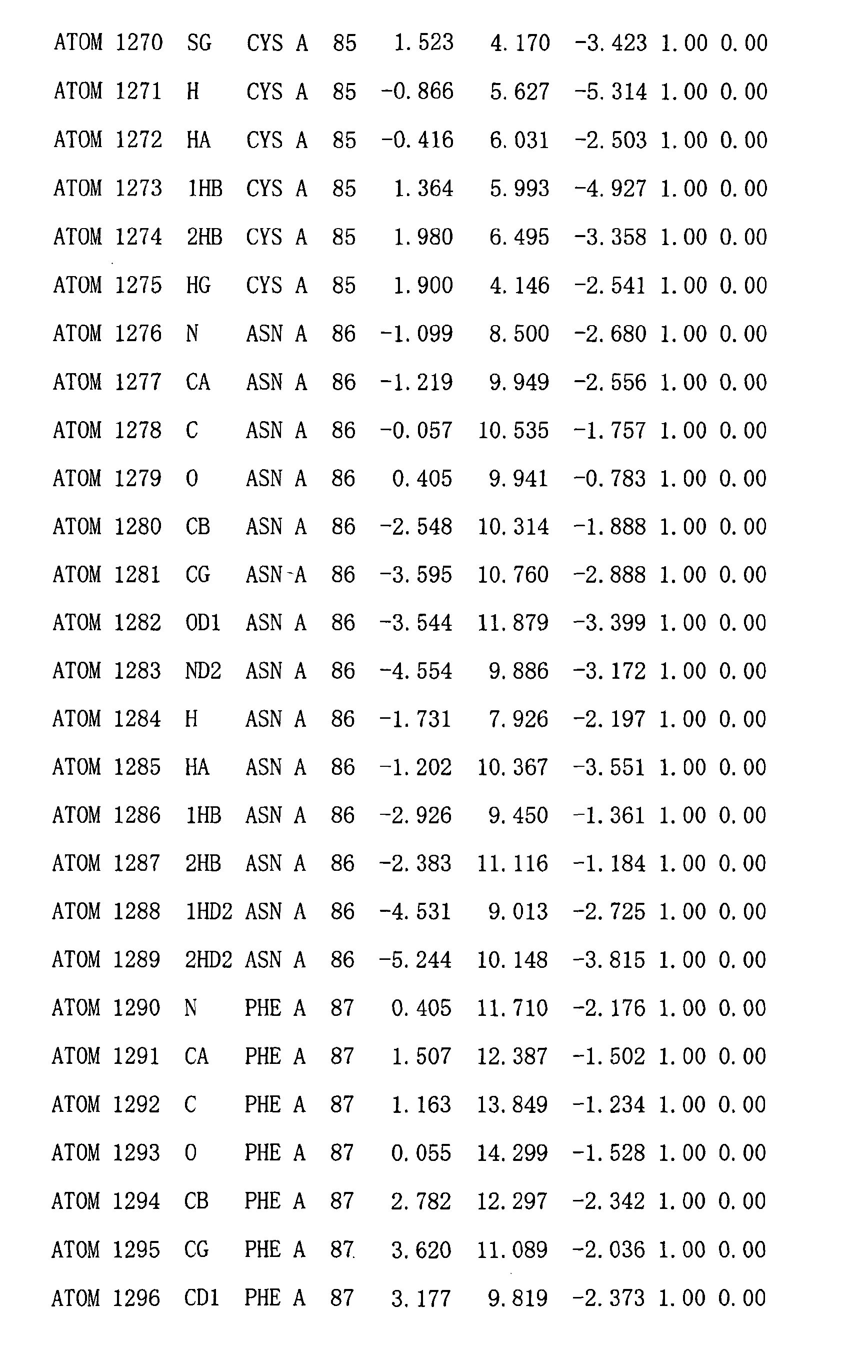

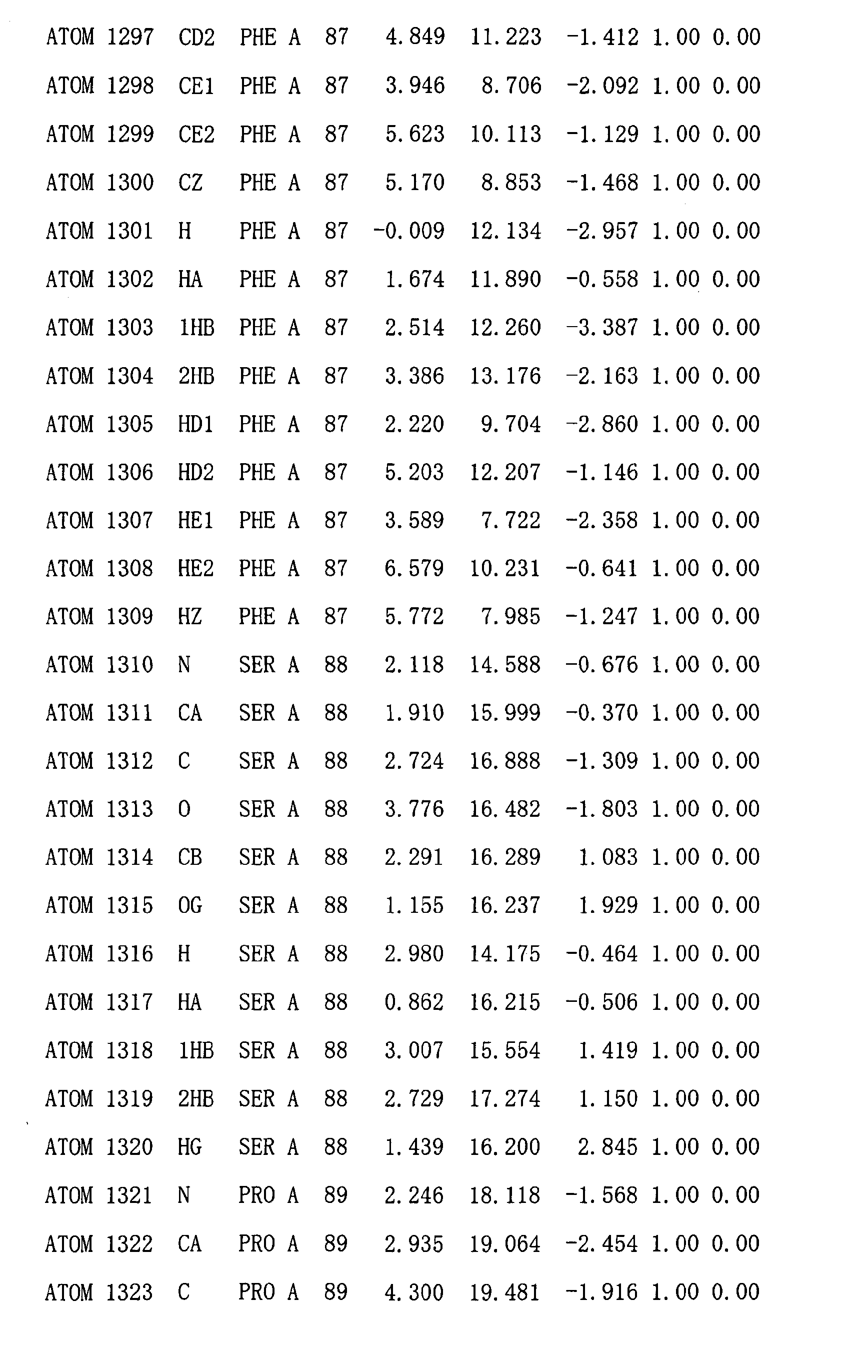

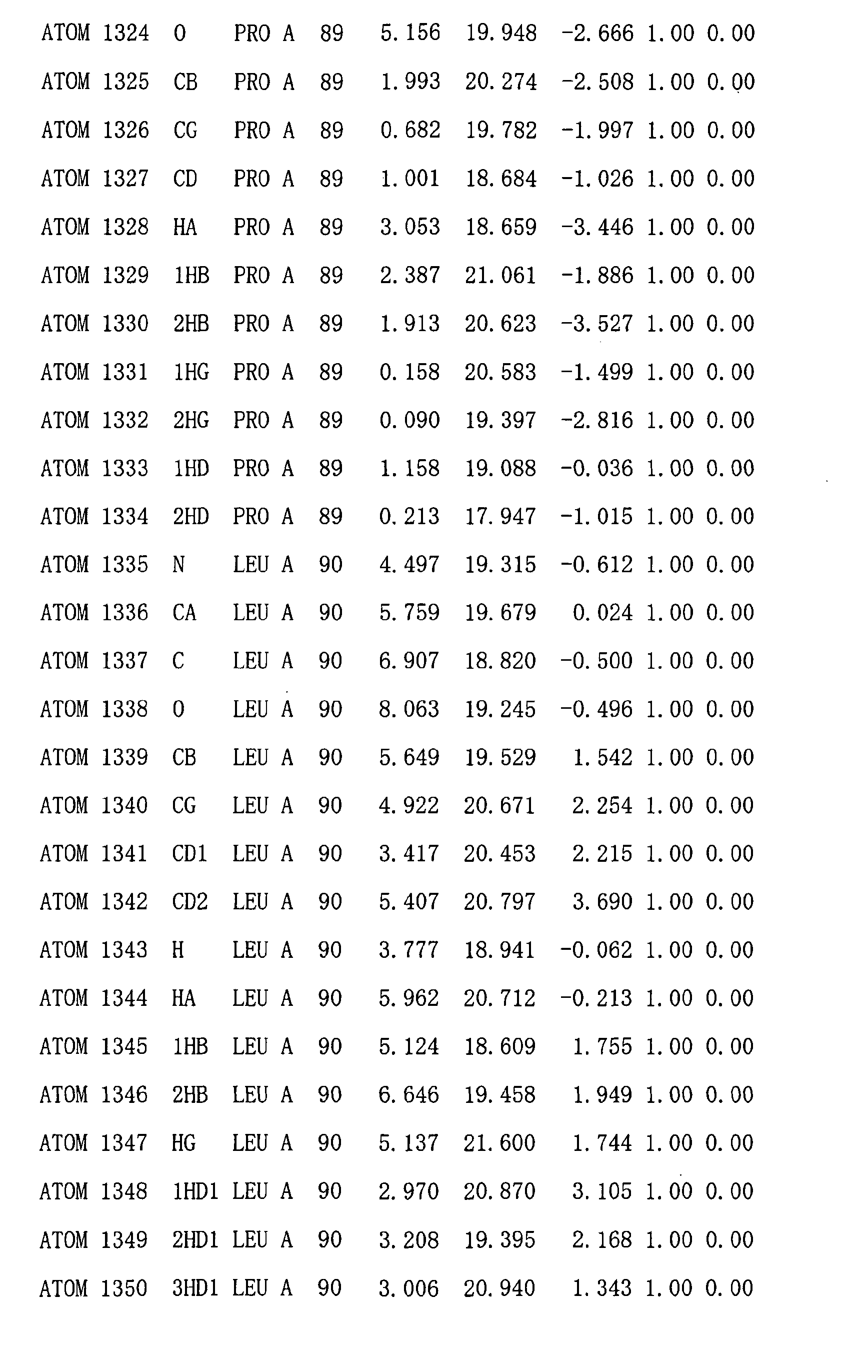

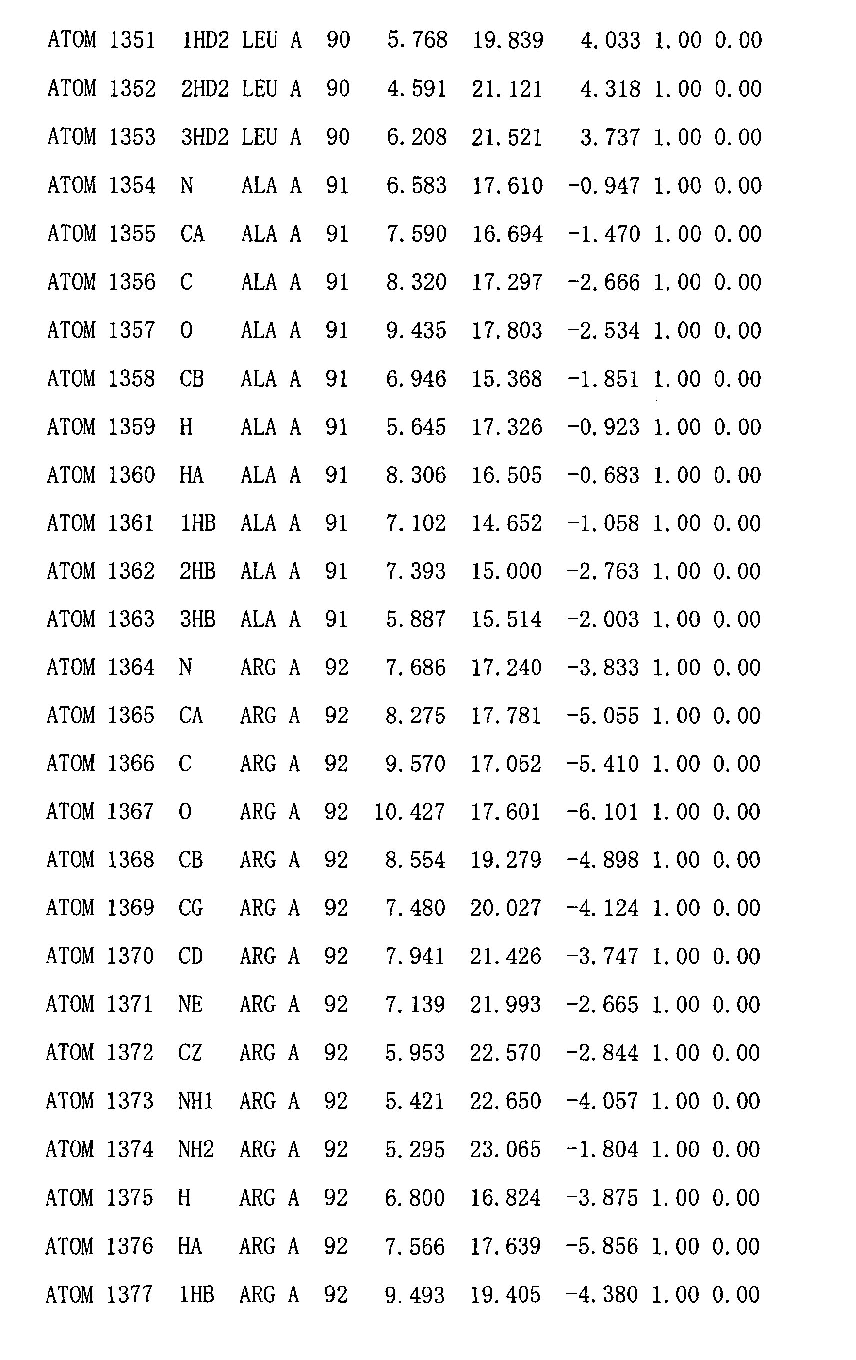

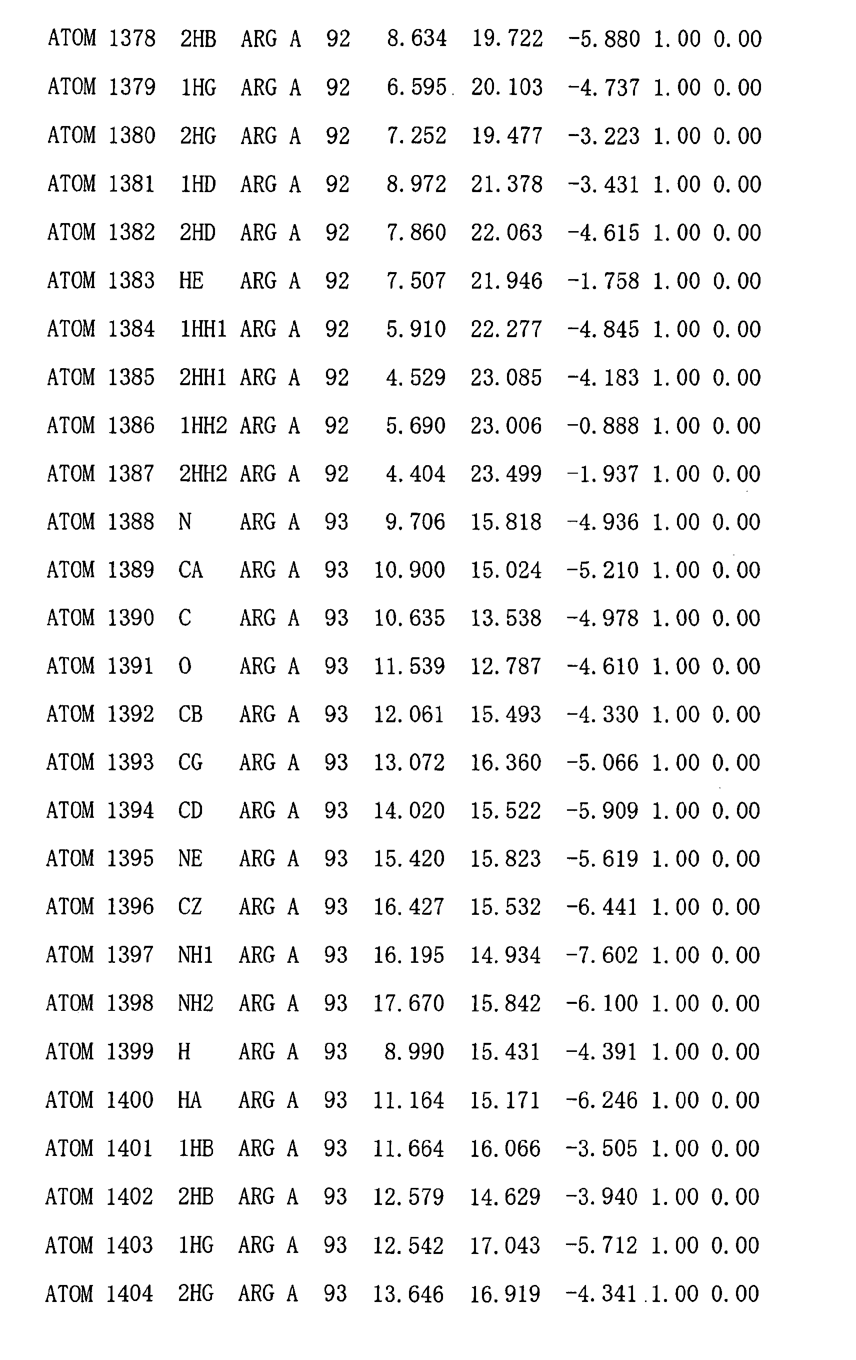

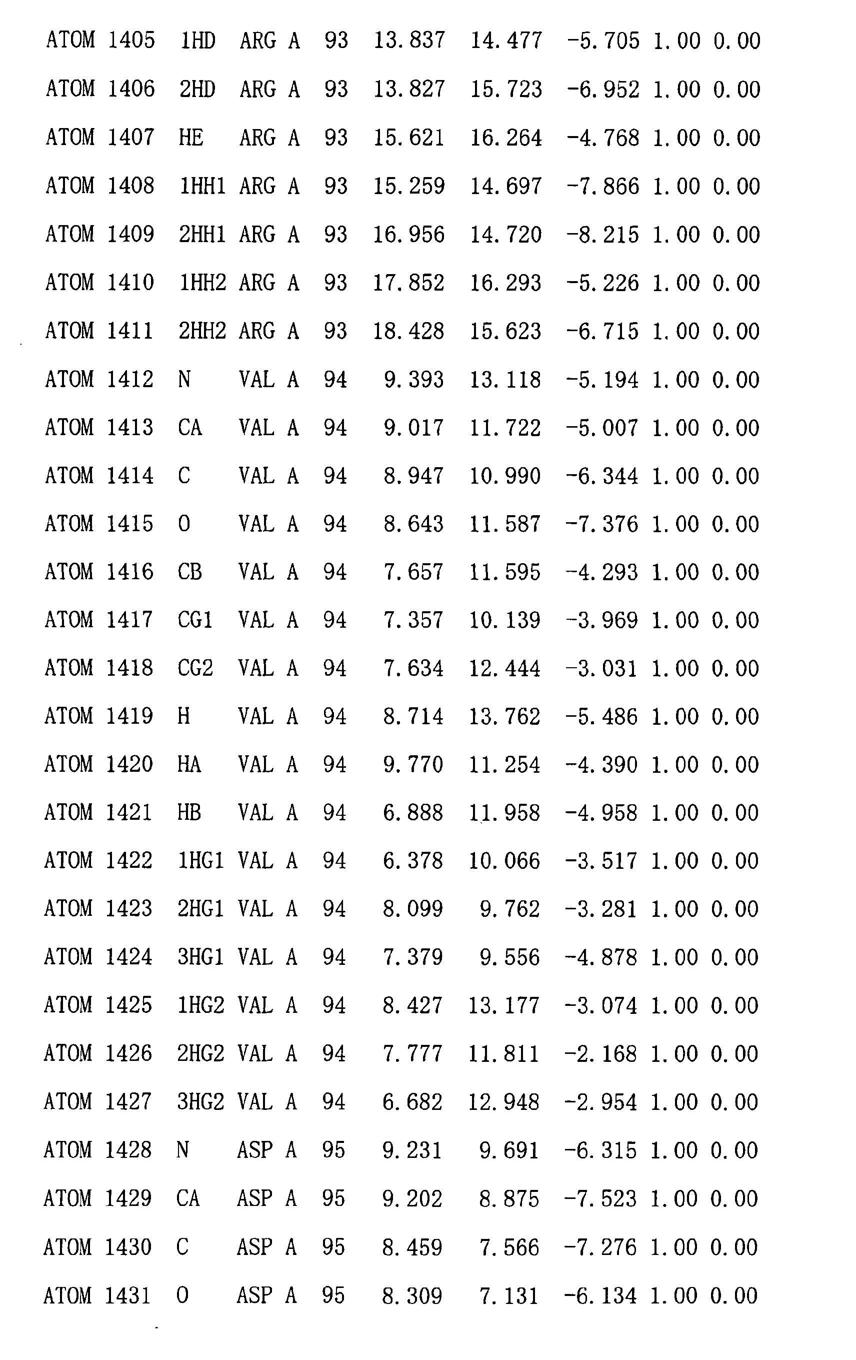

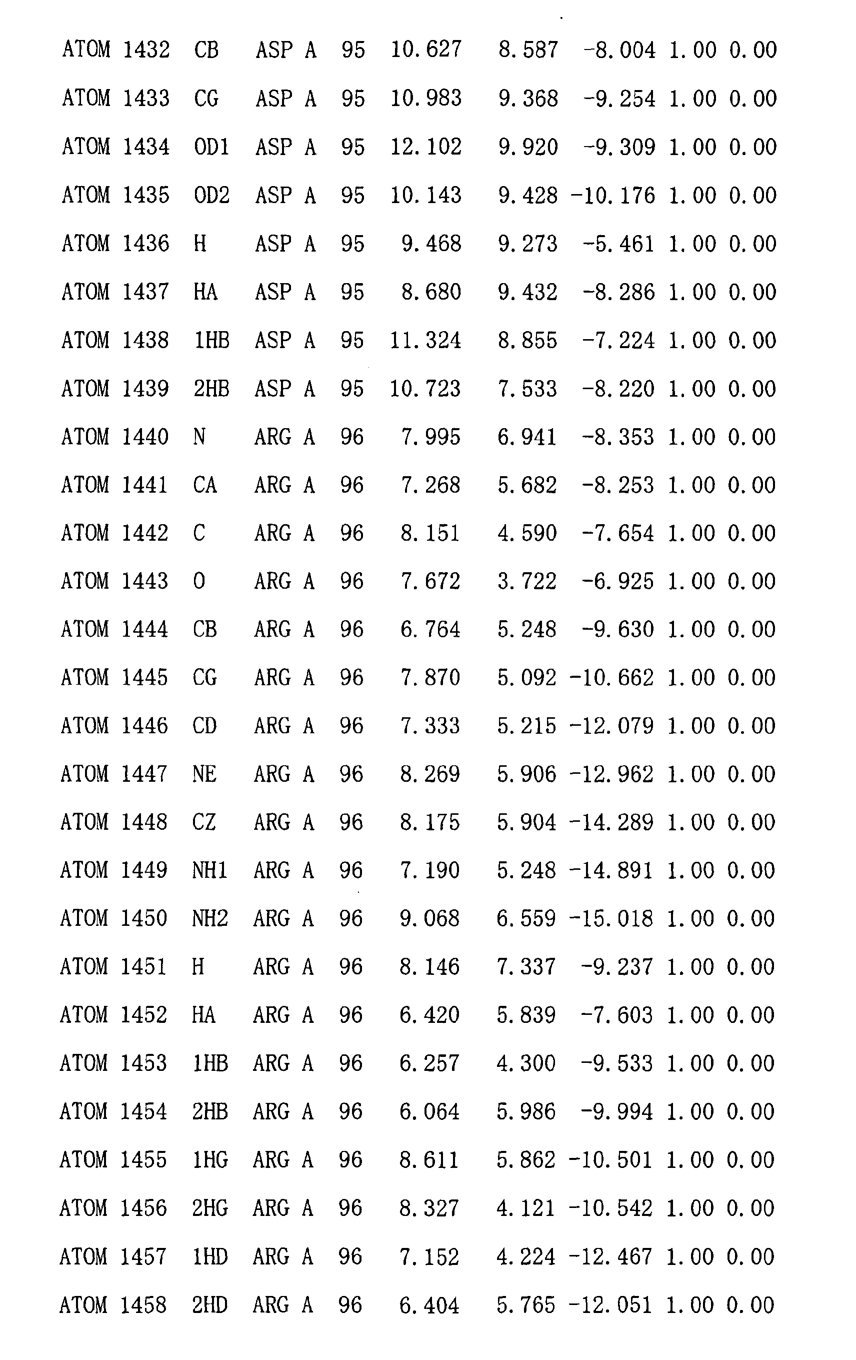

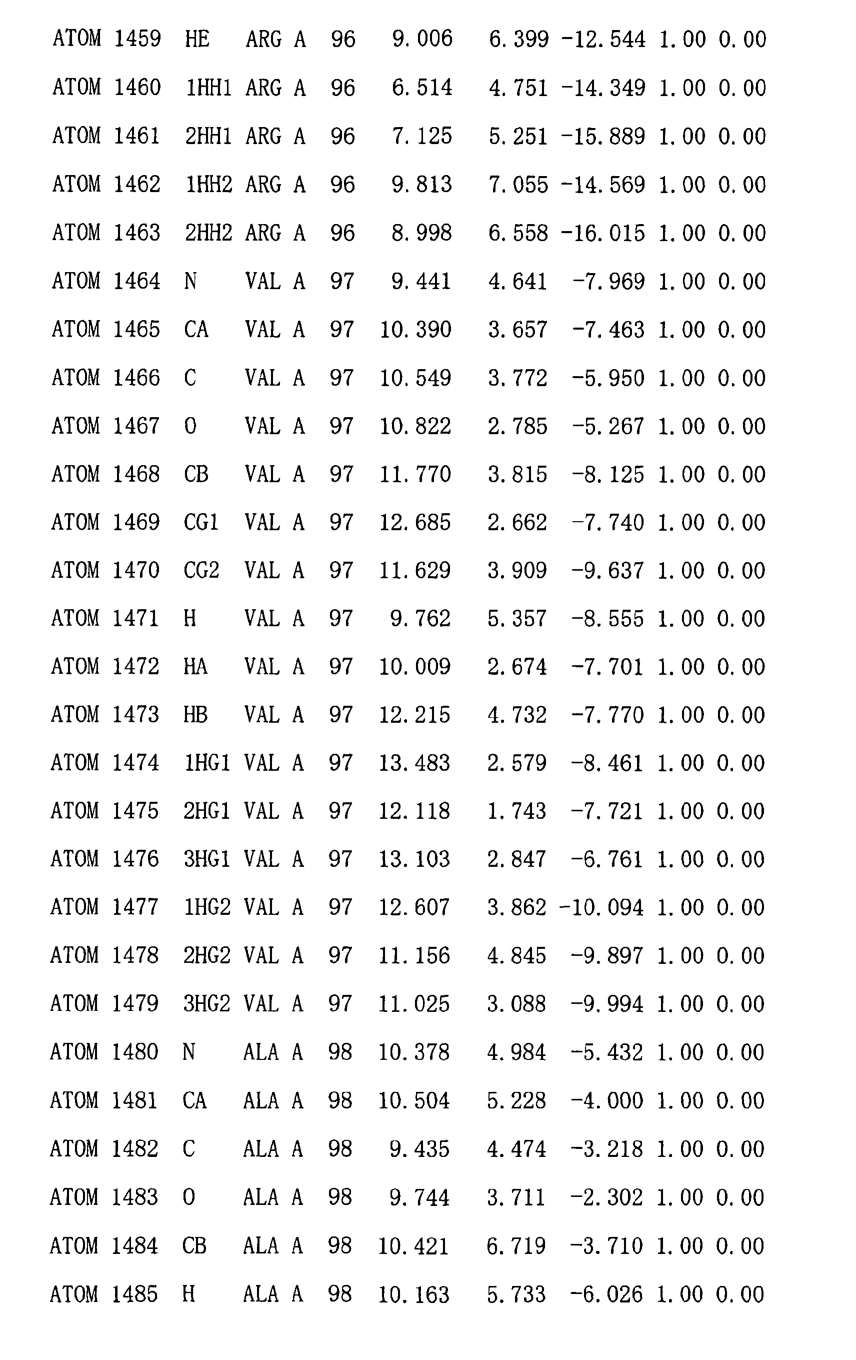

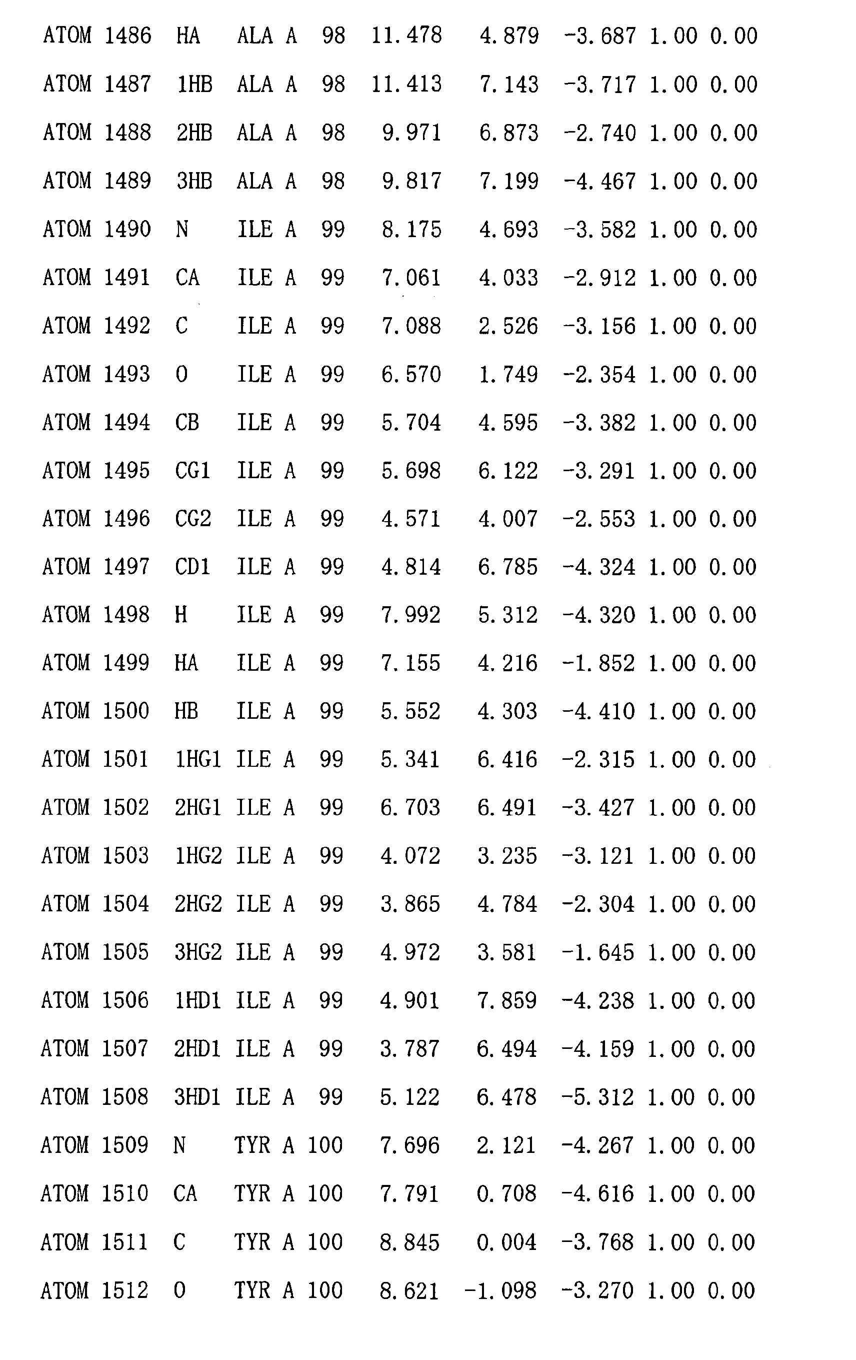

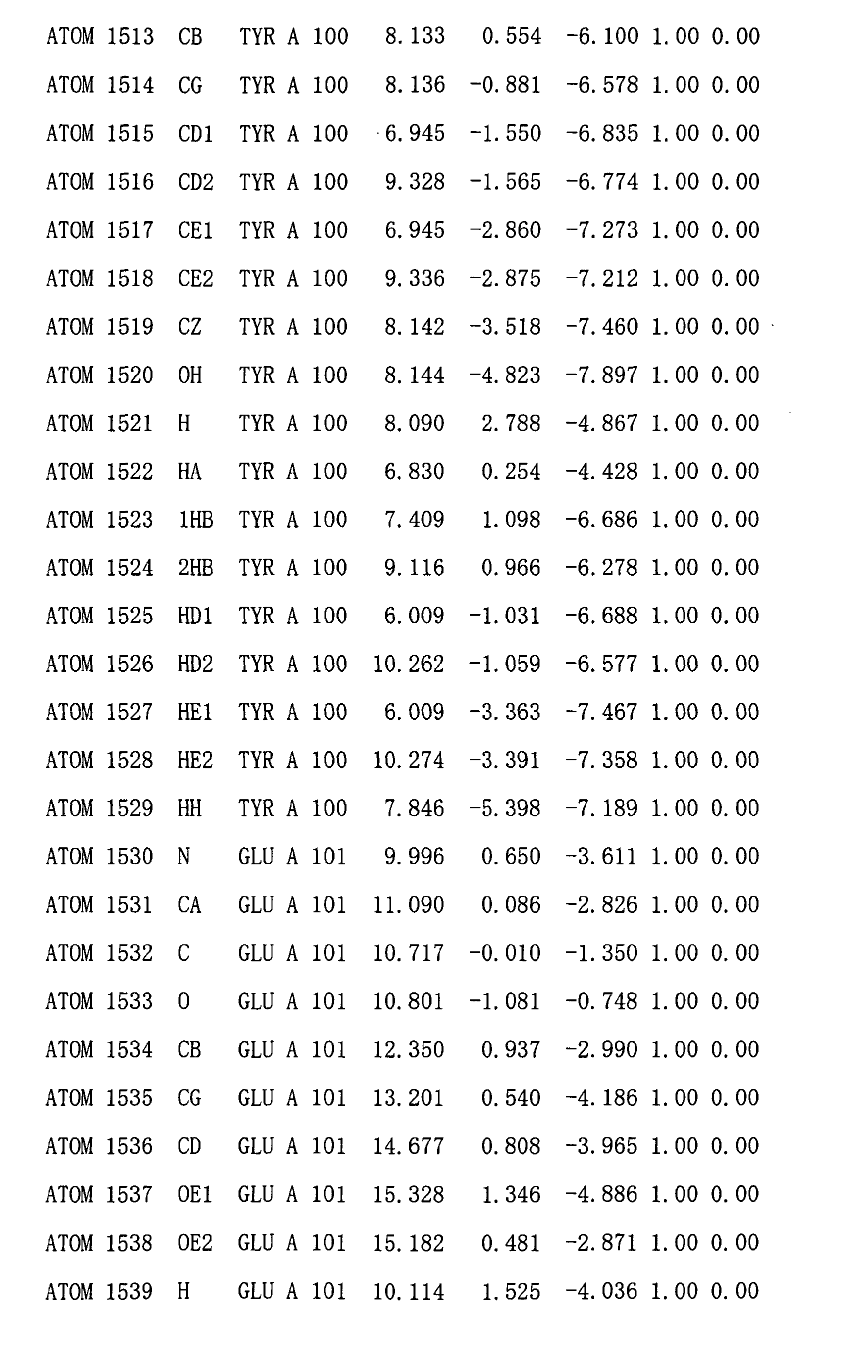

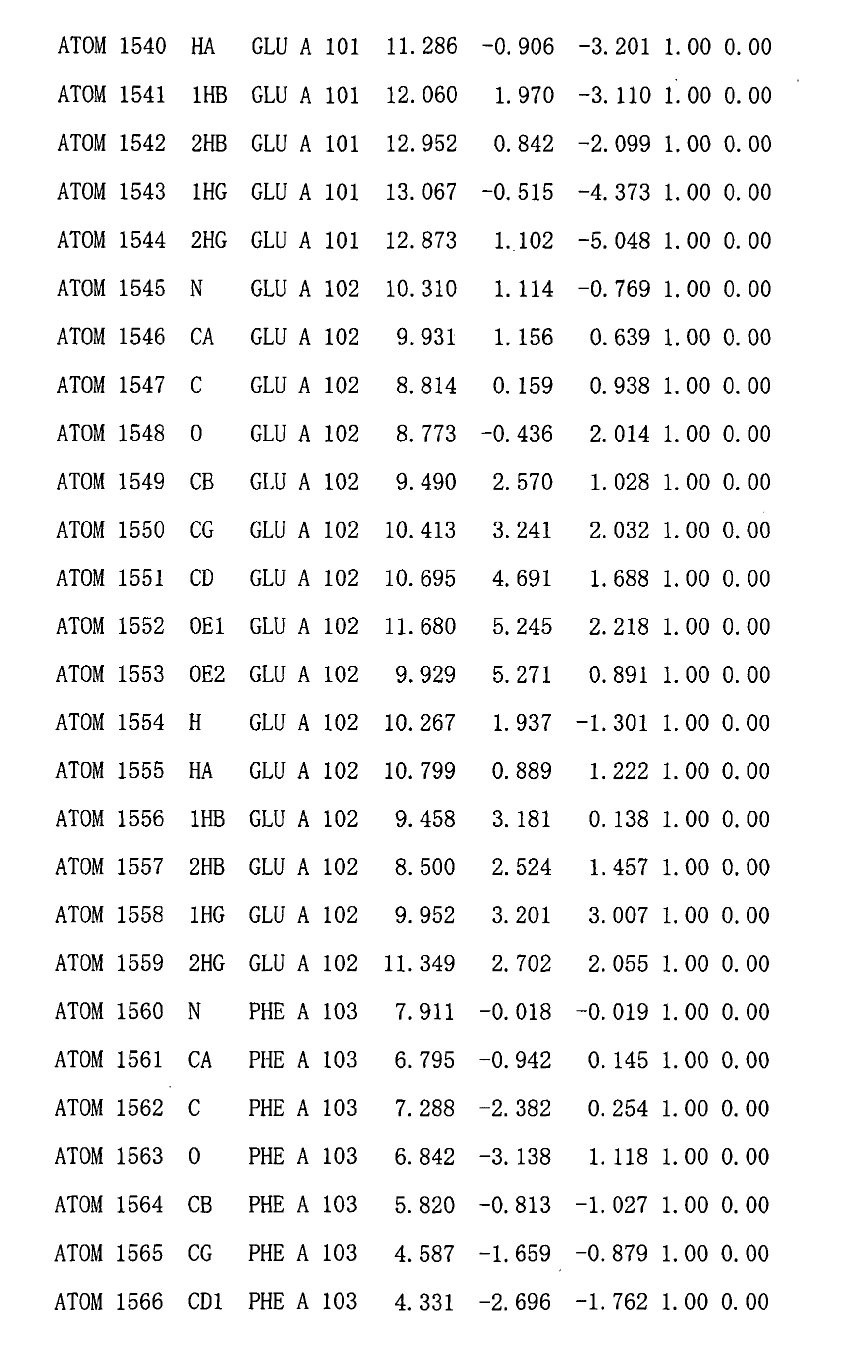

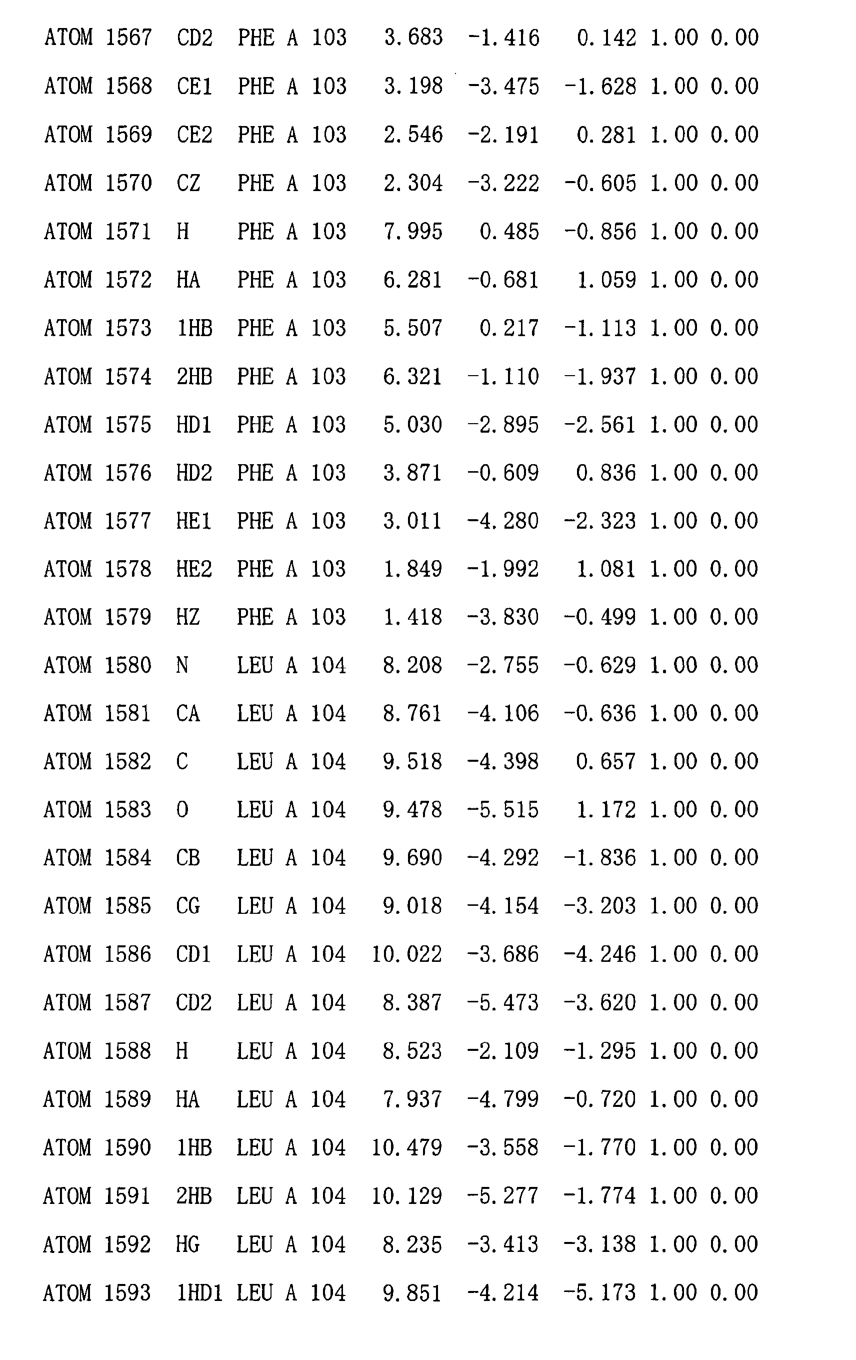

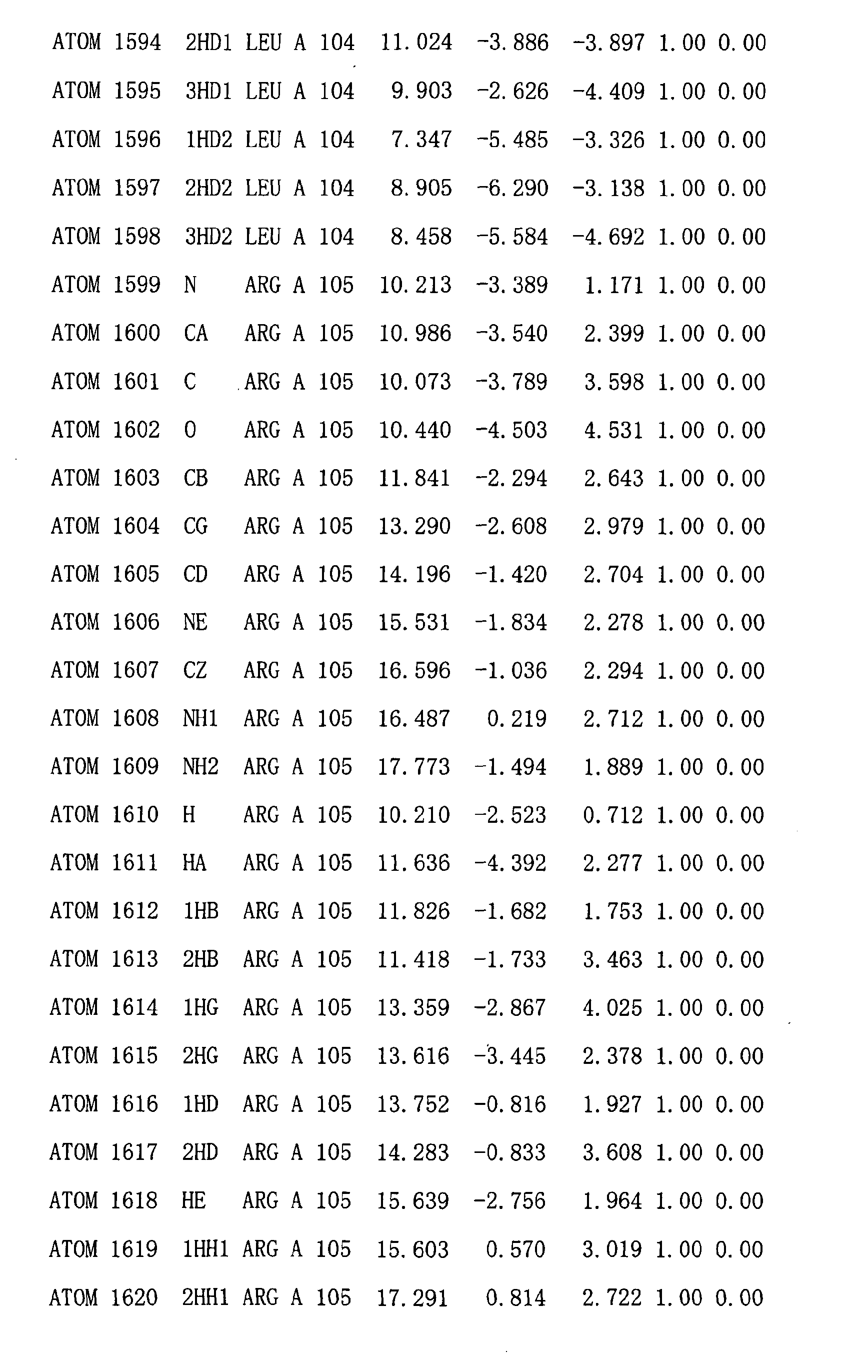

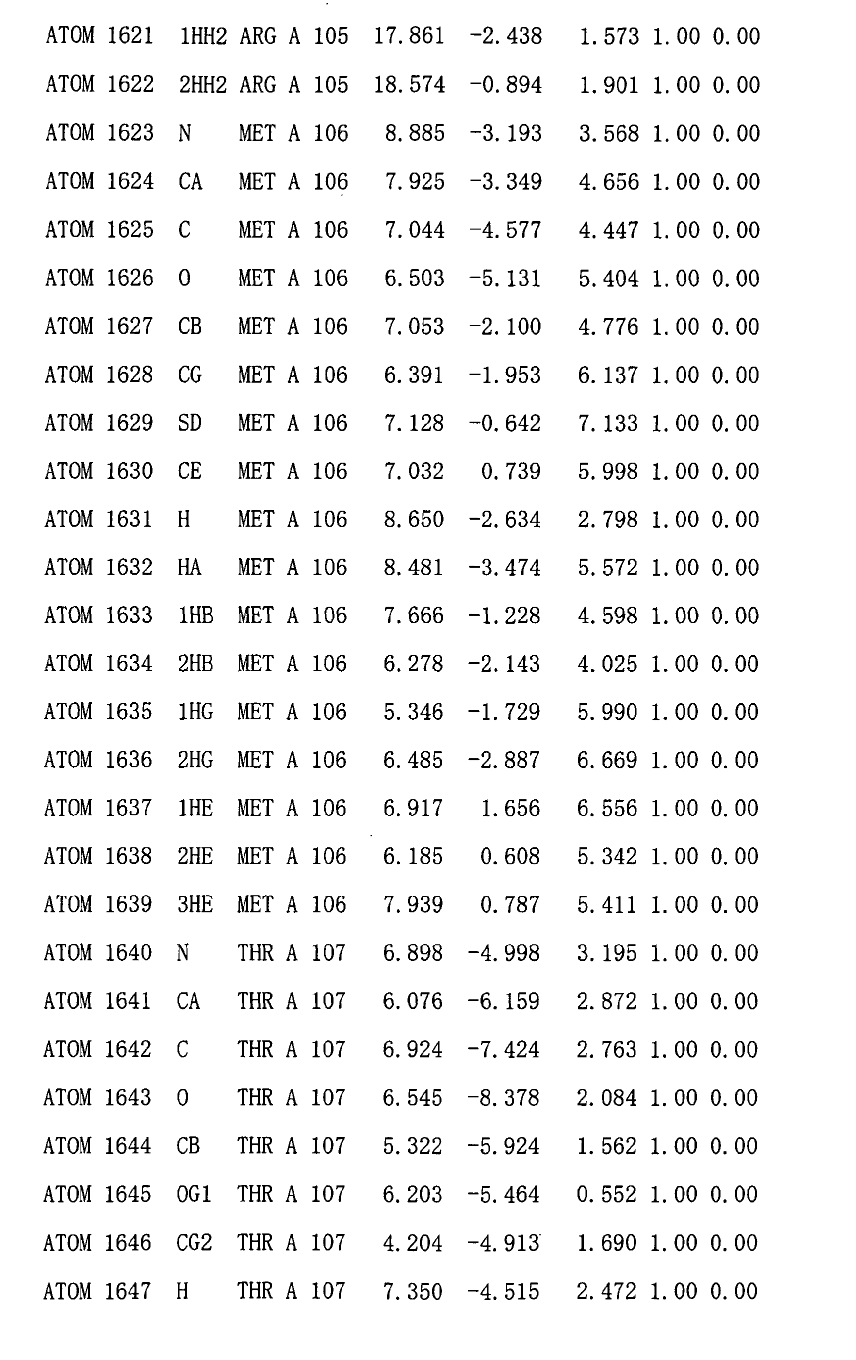

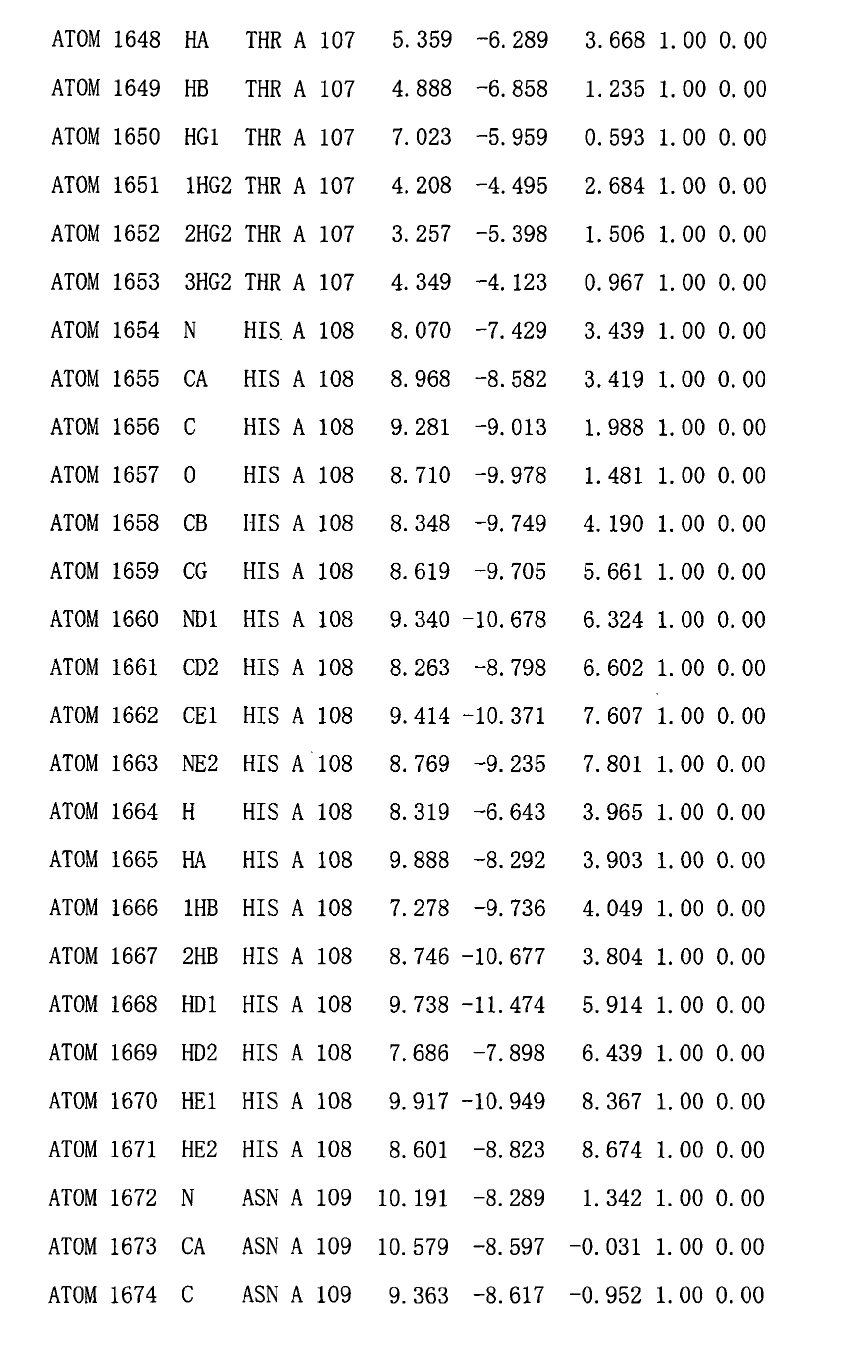

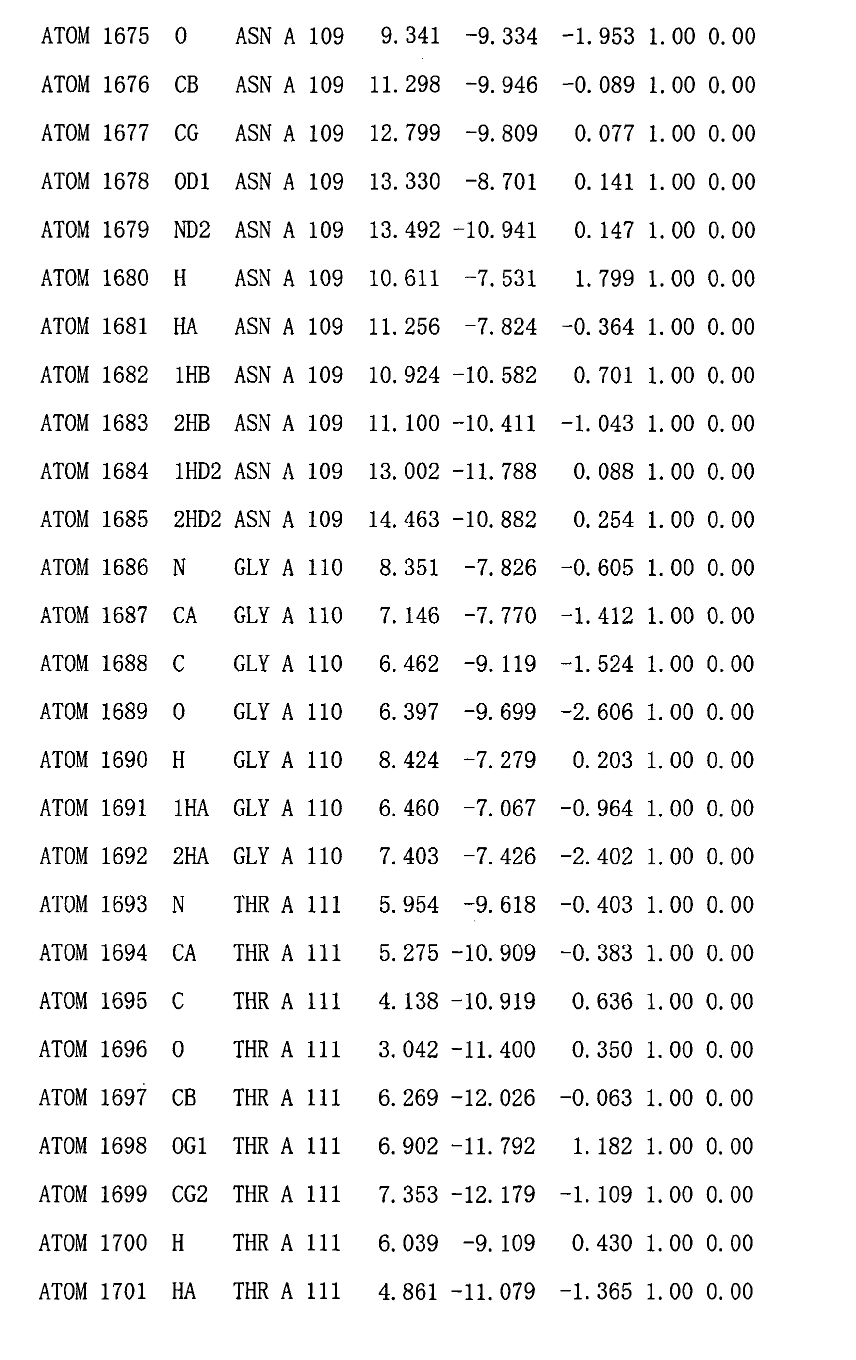

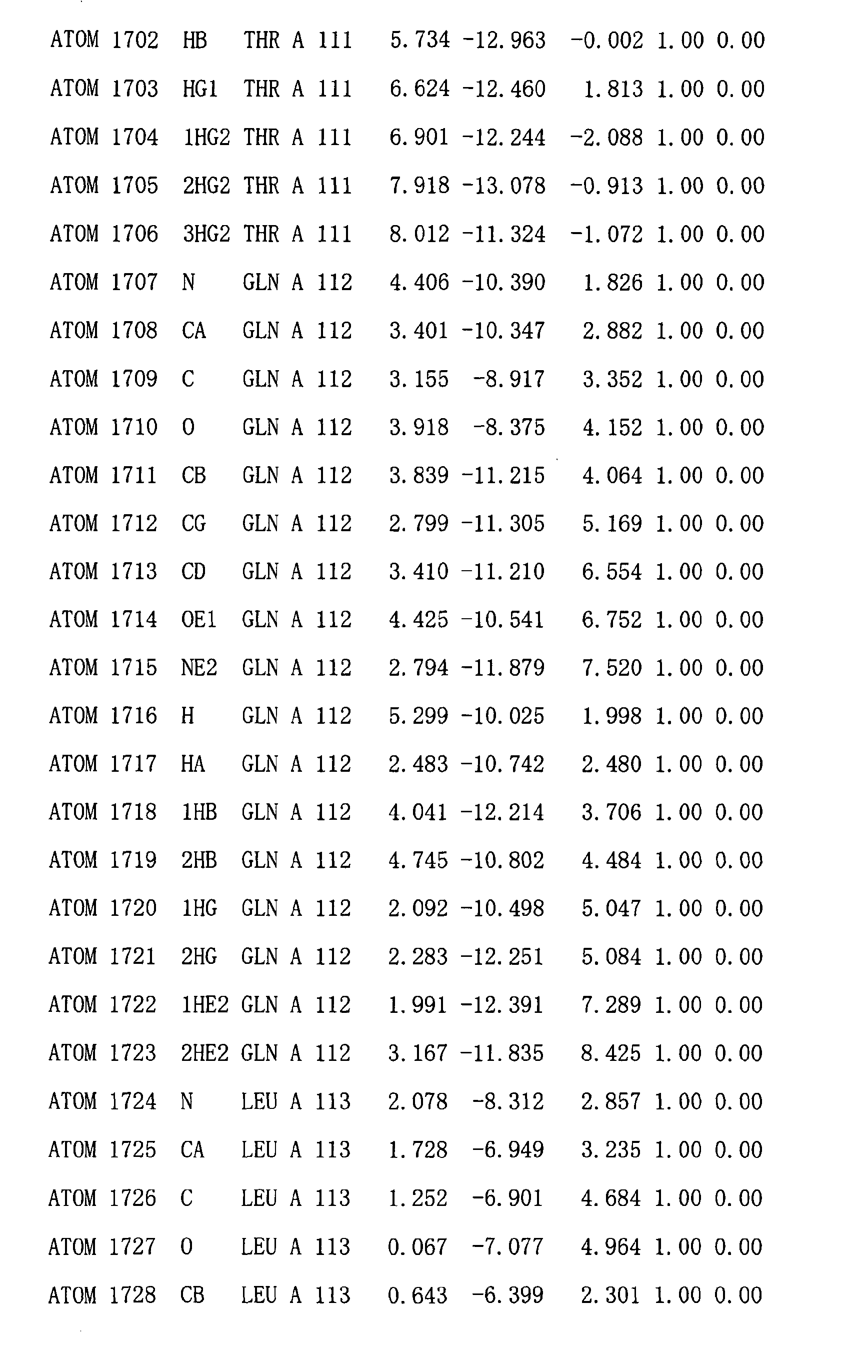

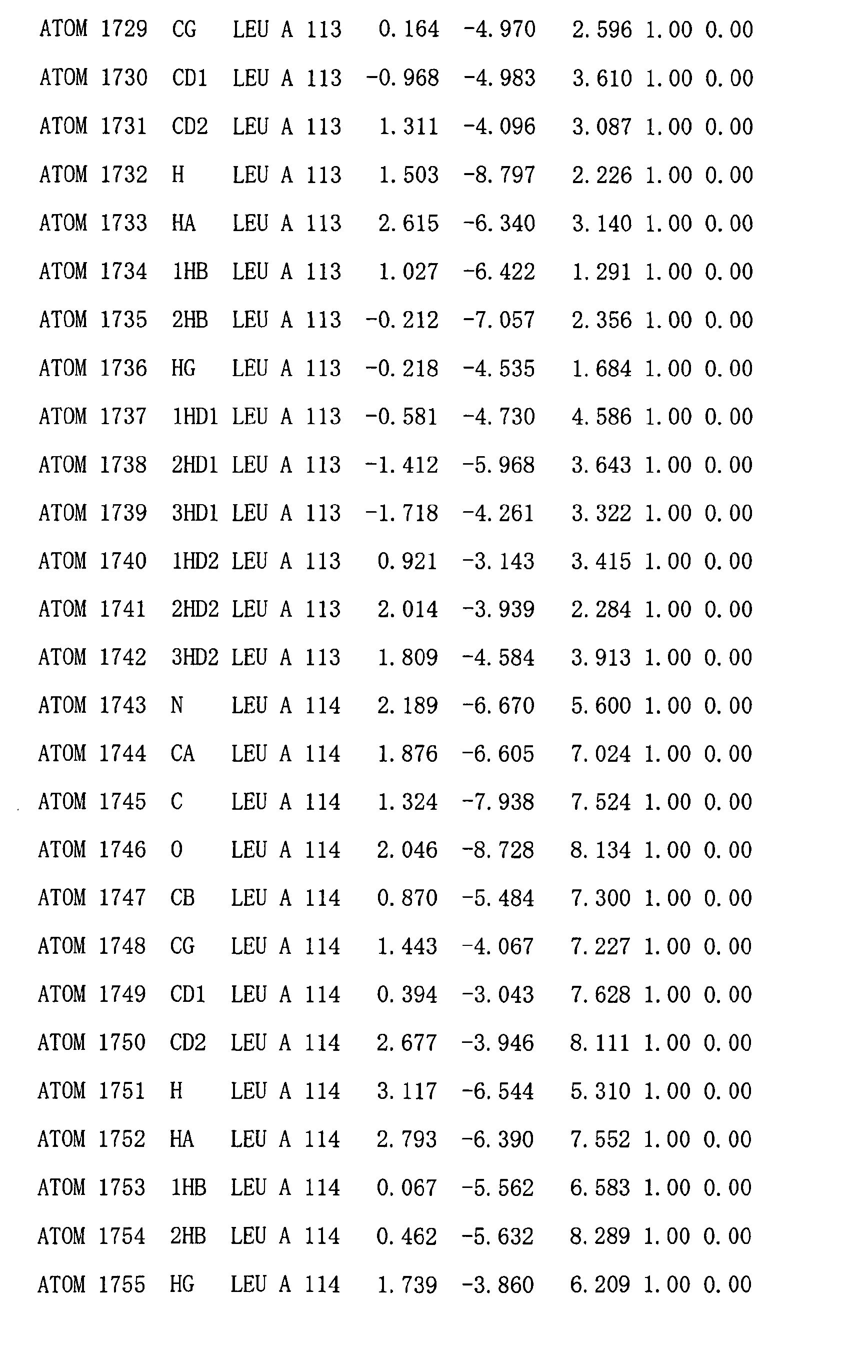

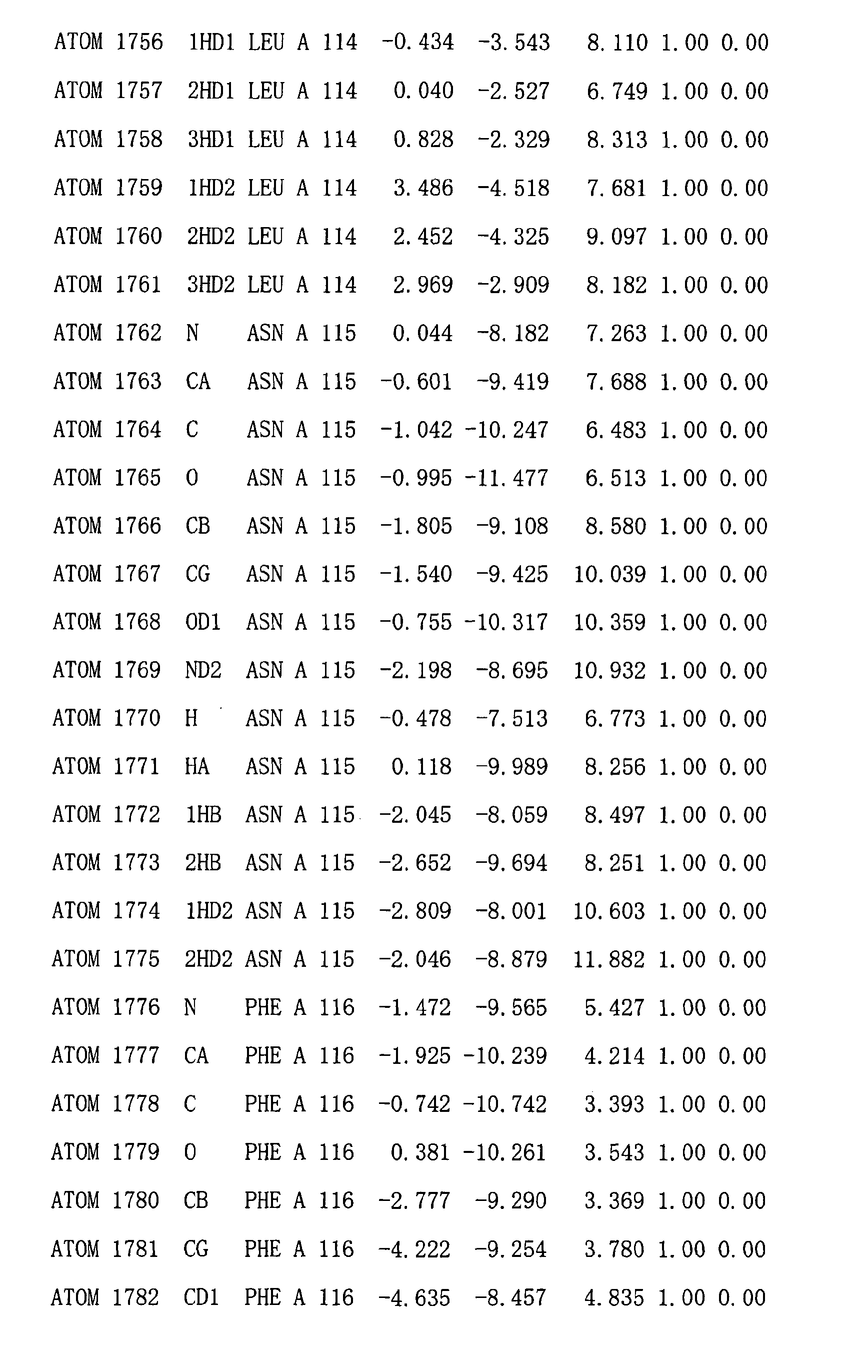

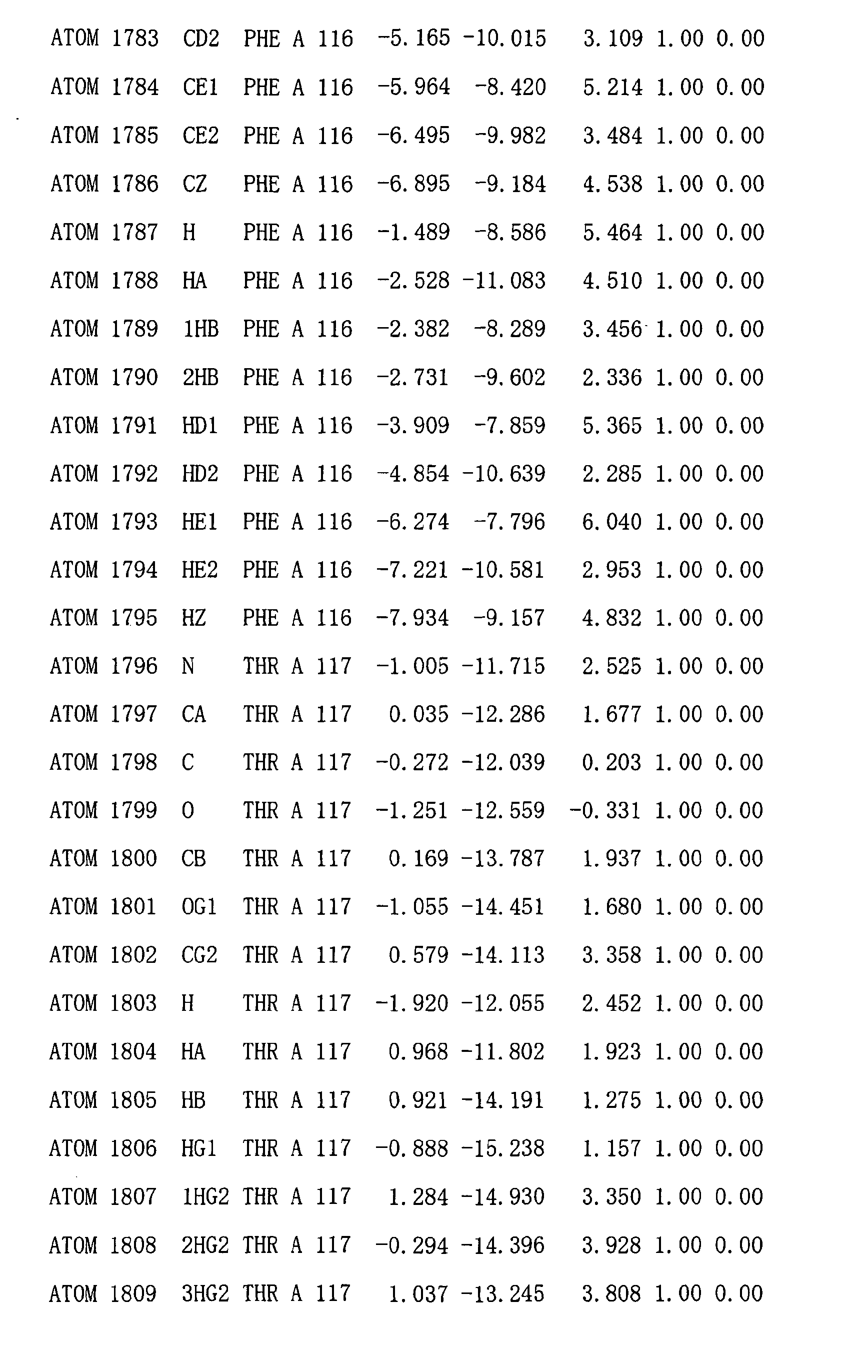

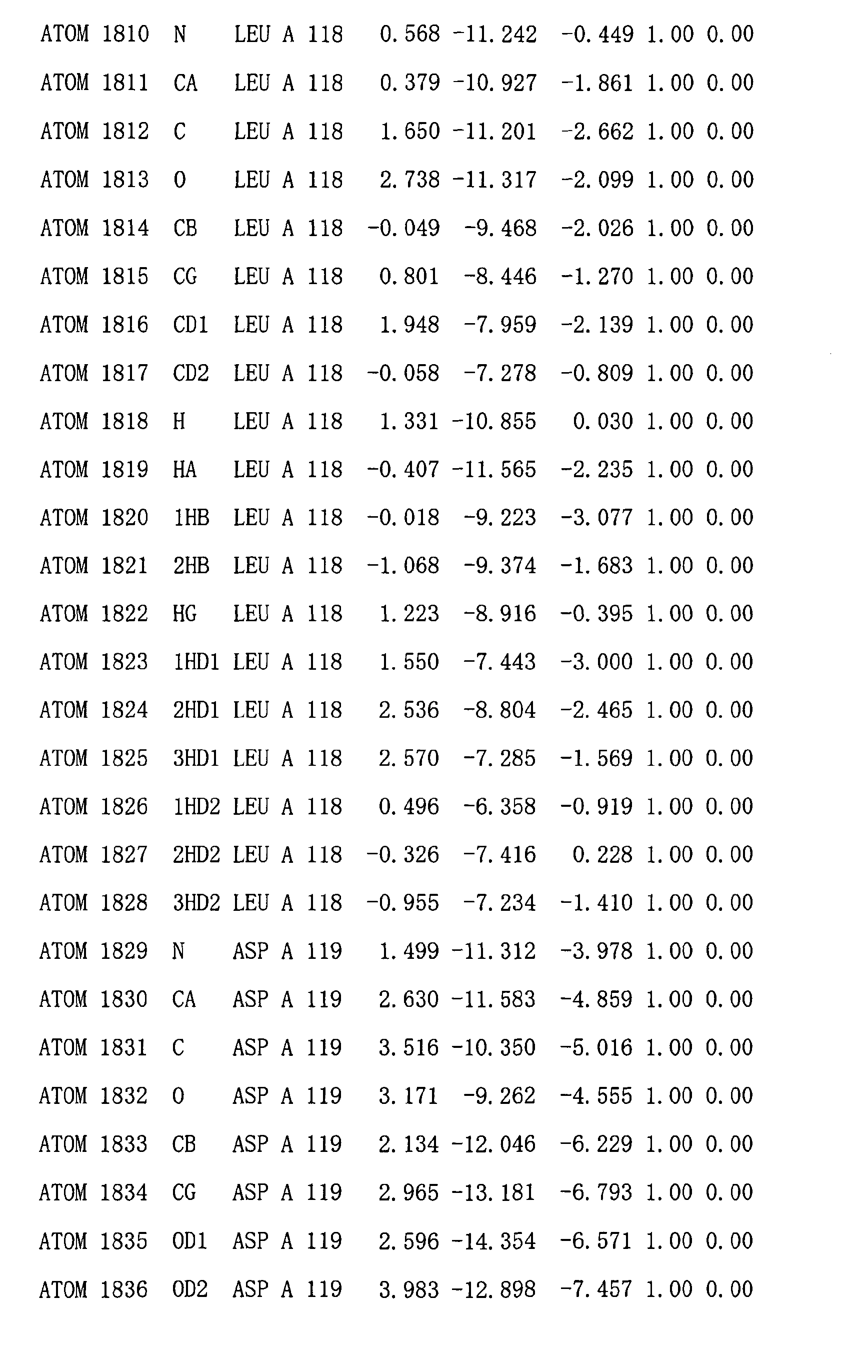

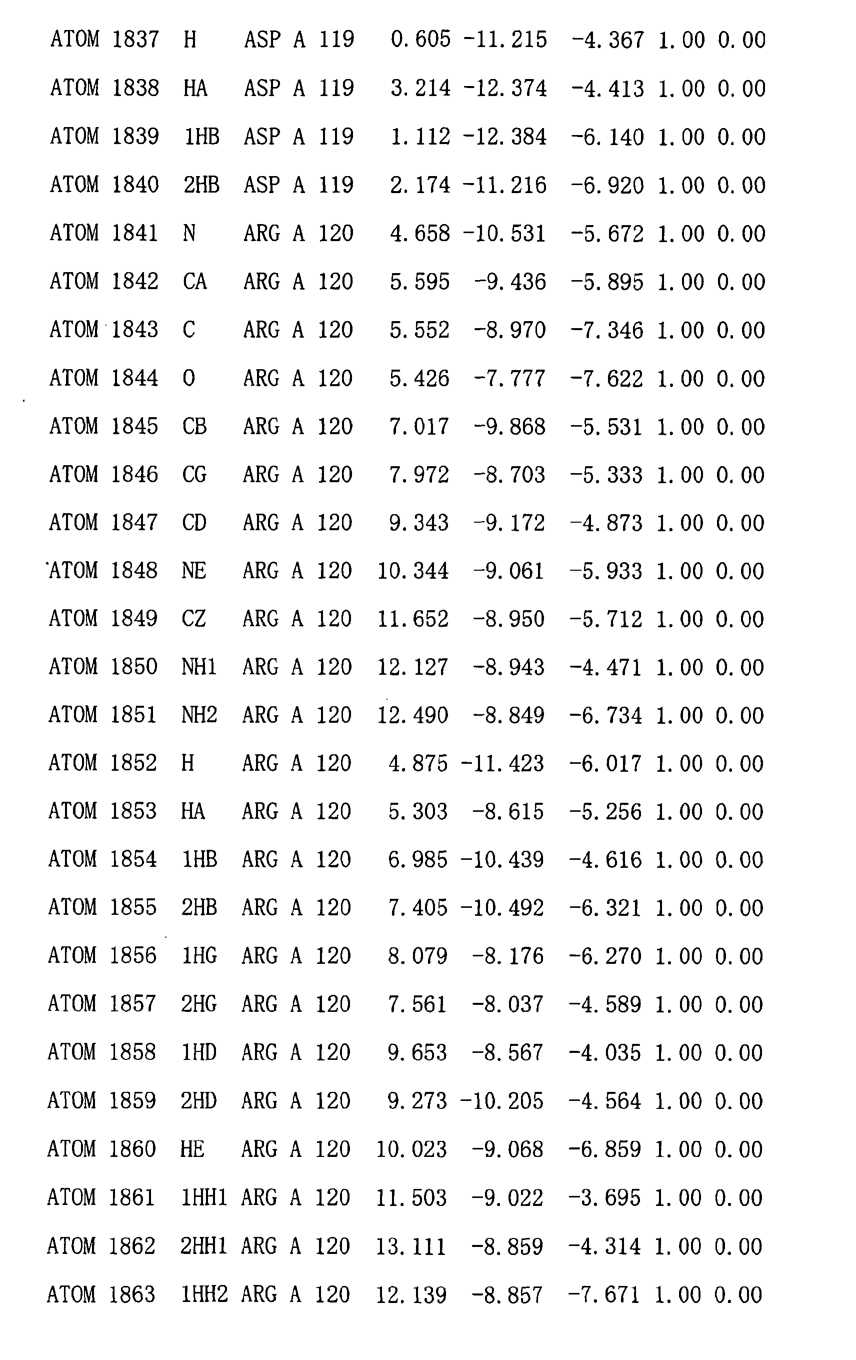

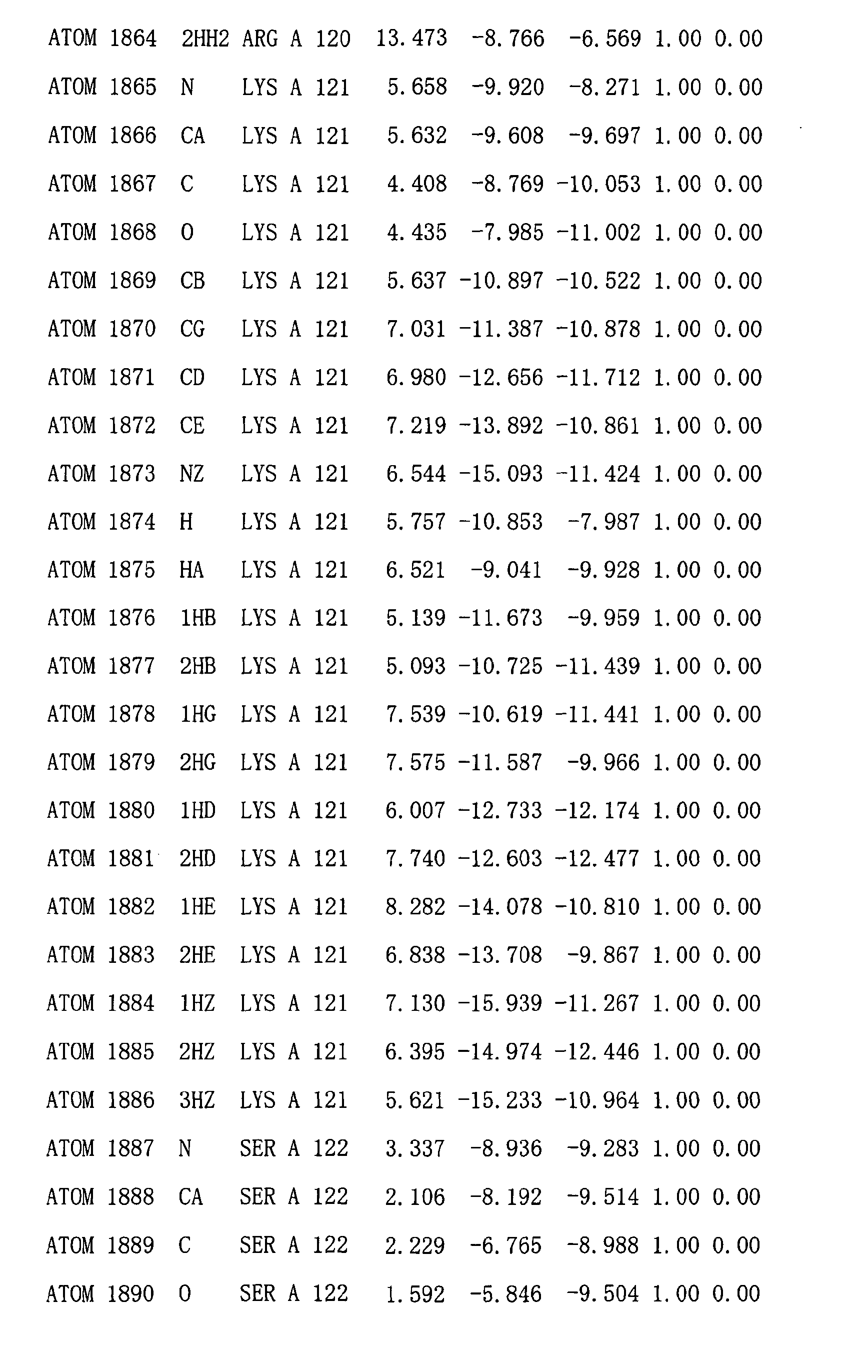

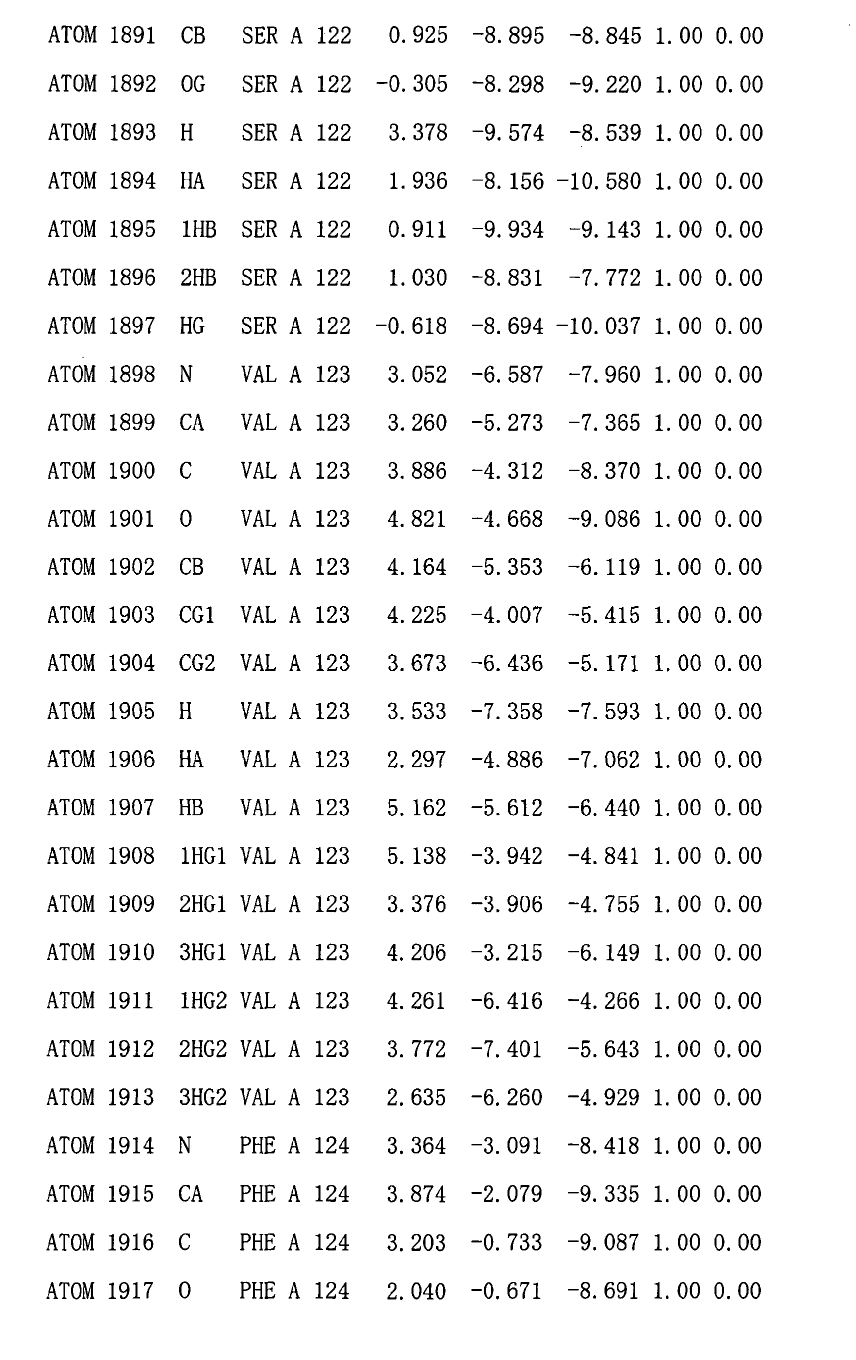

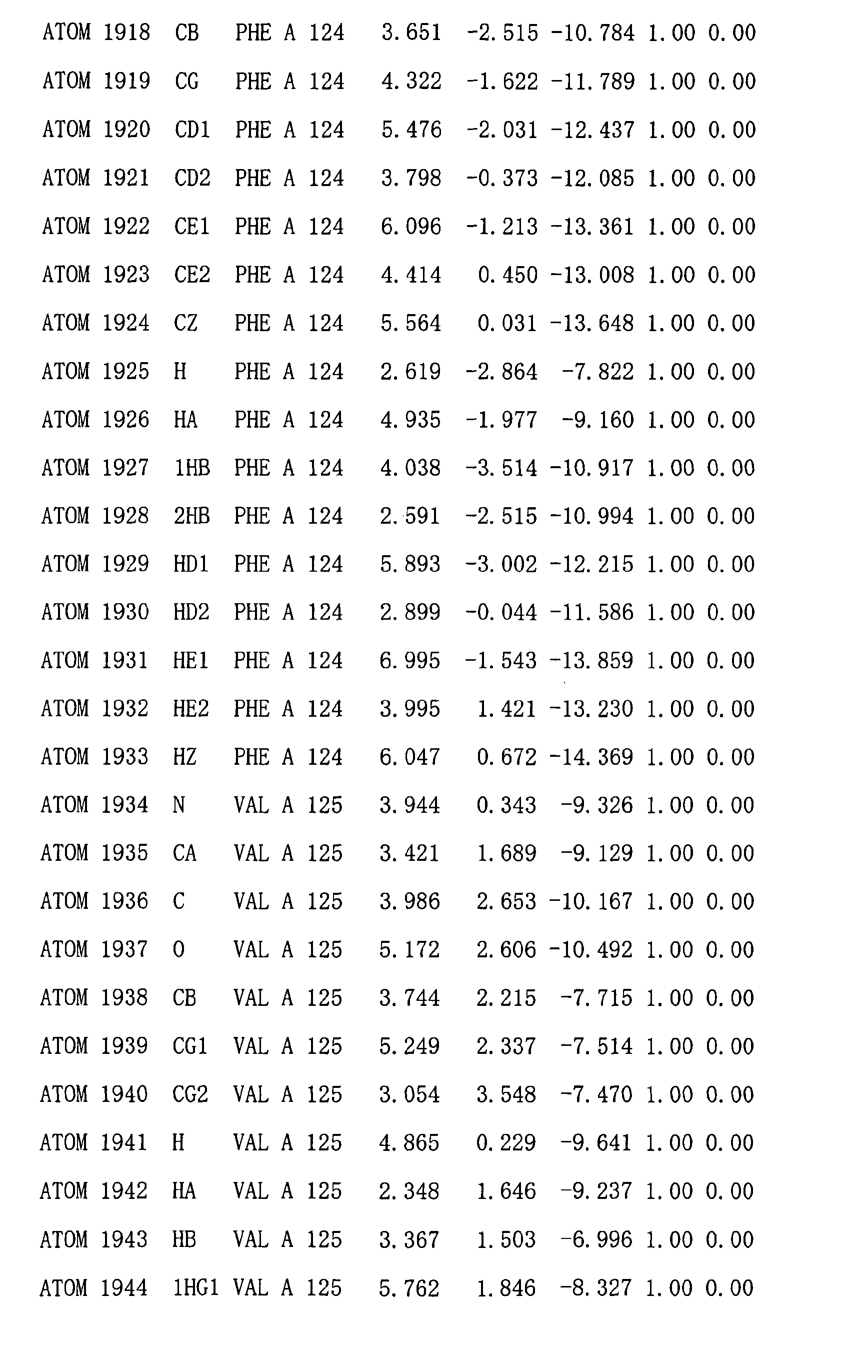

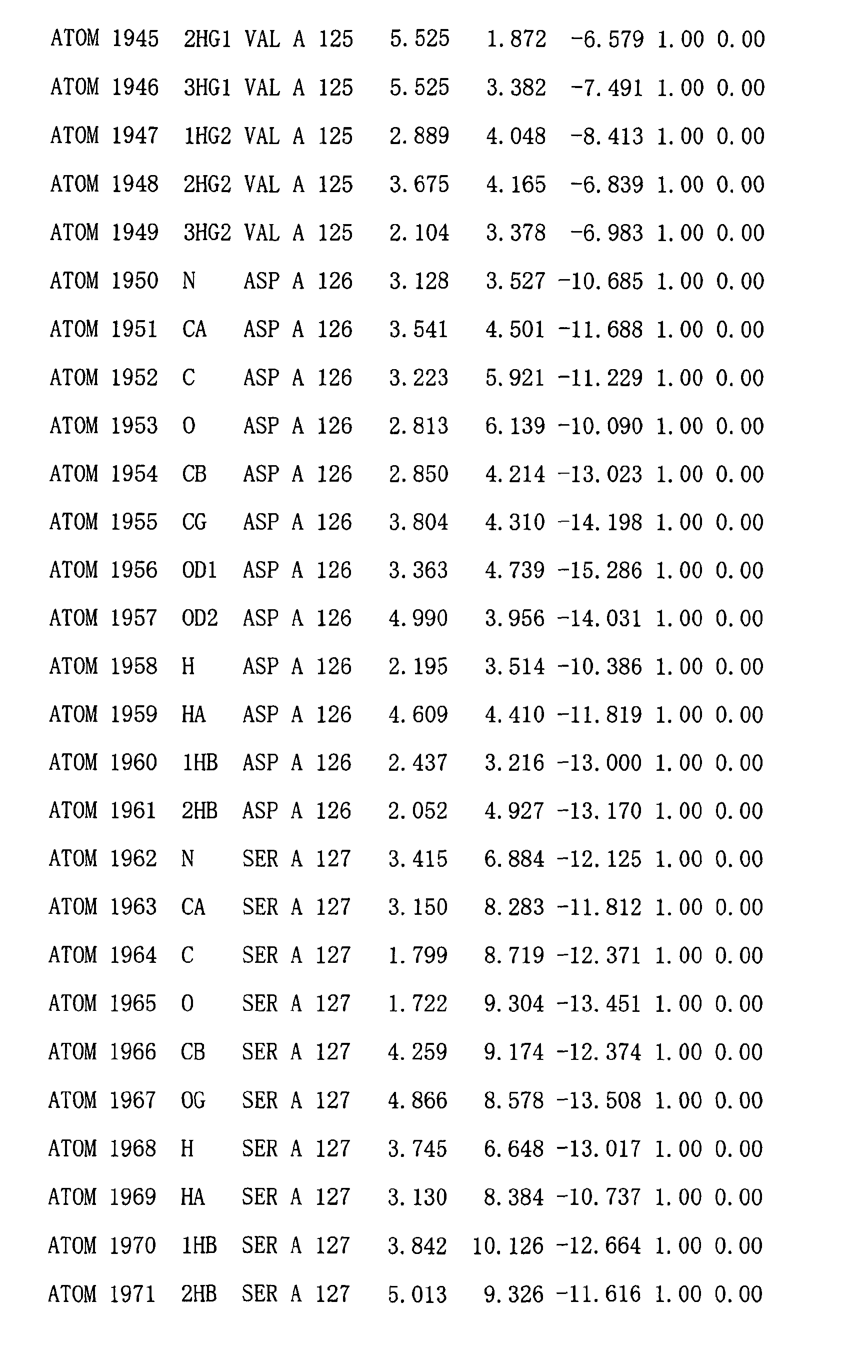

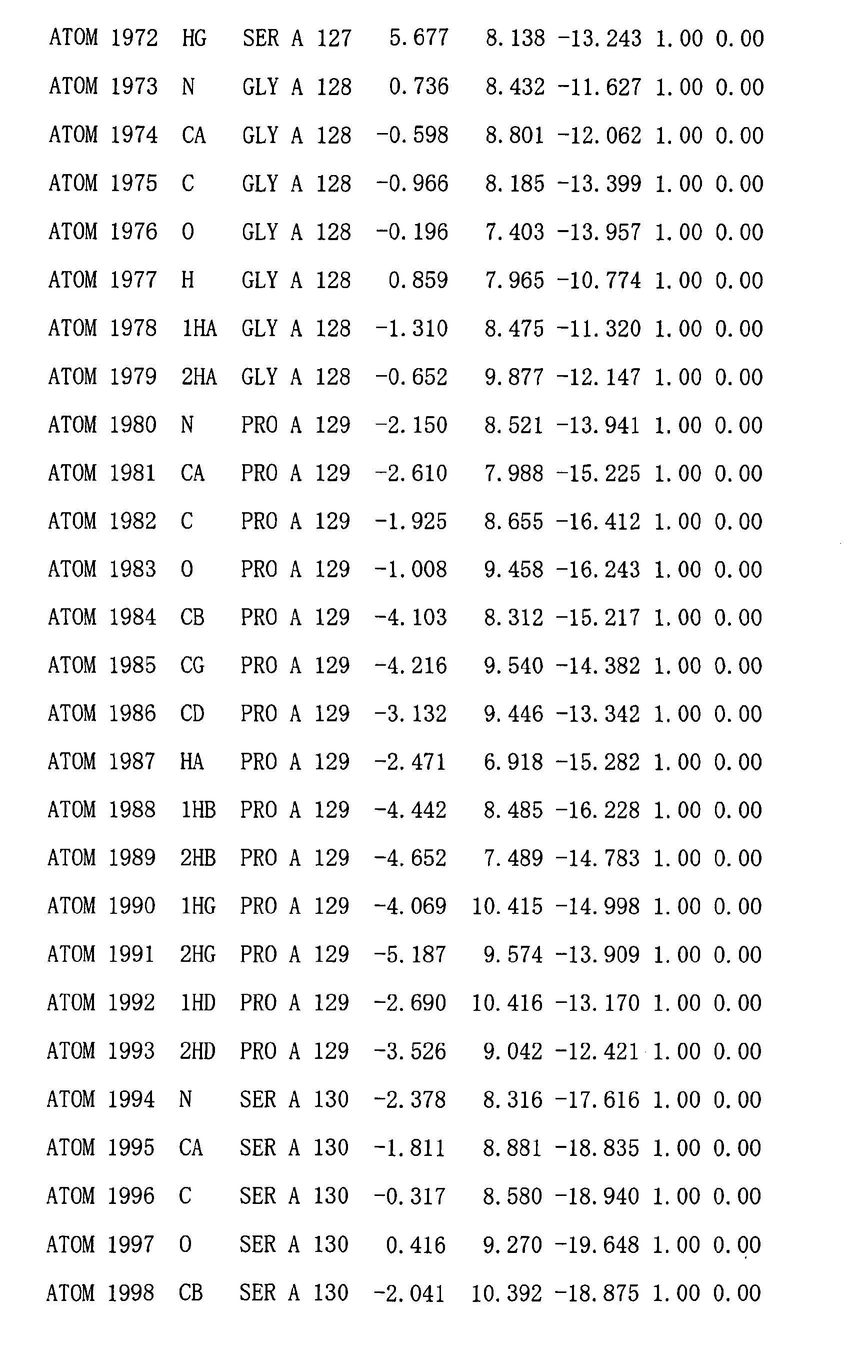

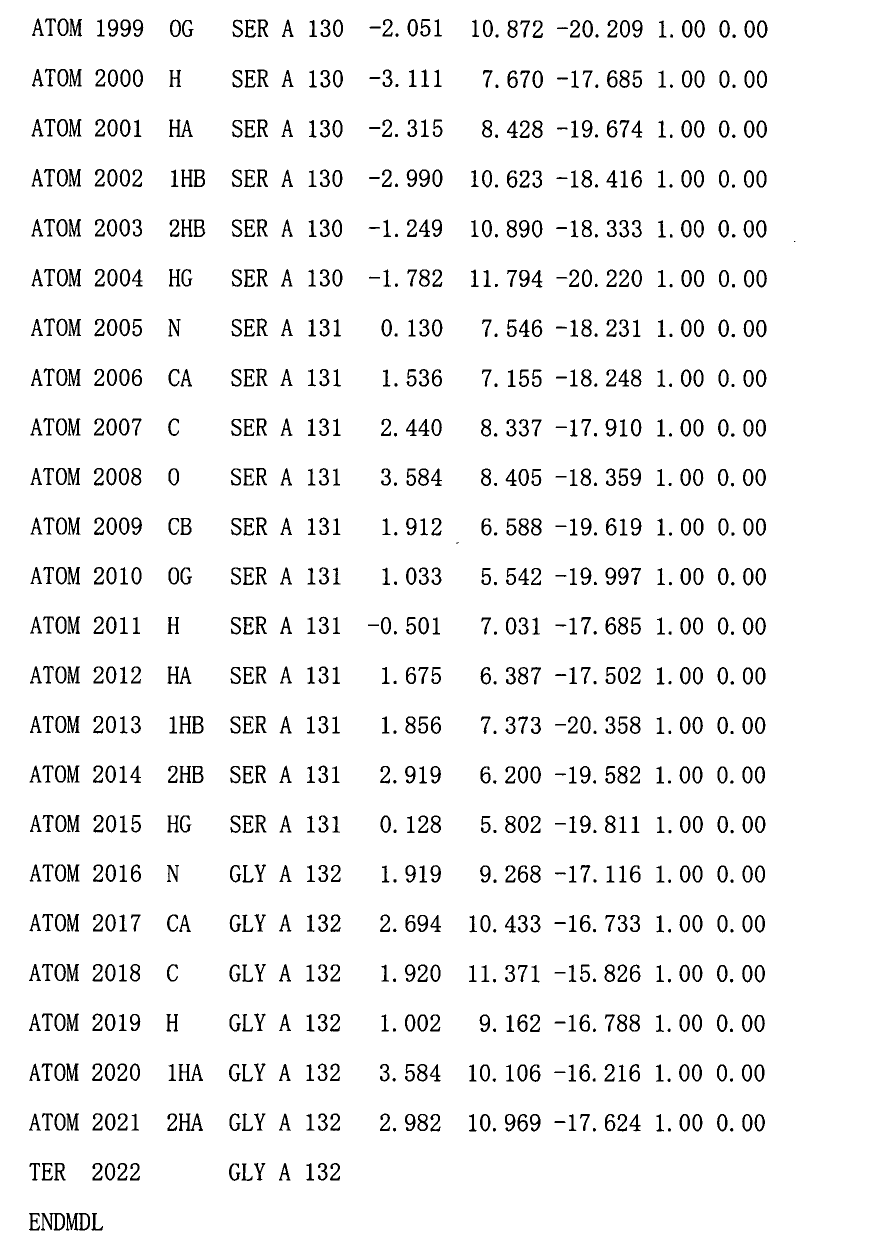

### Three-Dimensional Structure Coordinate 16

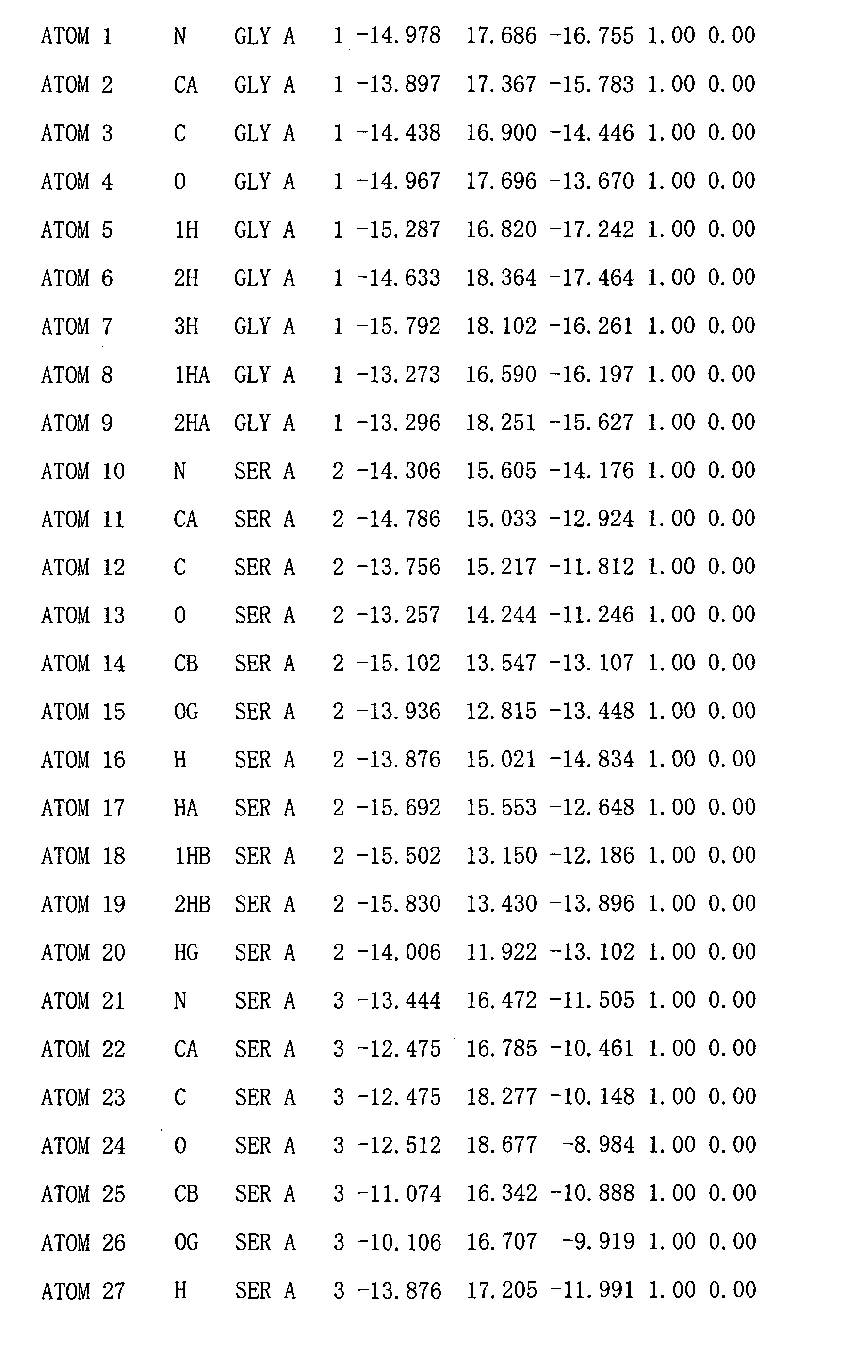

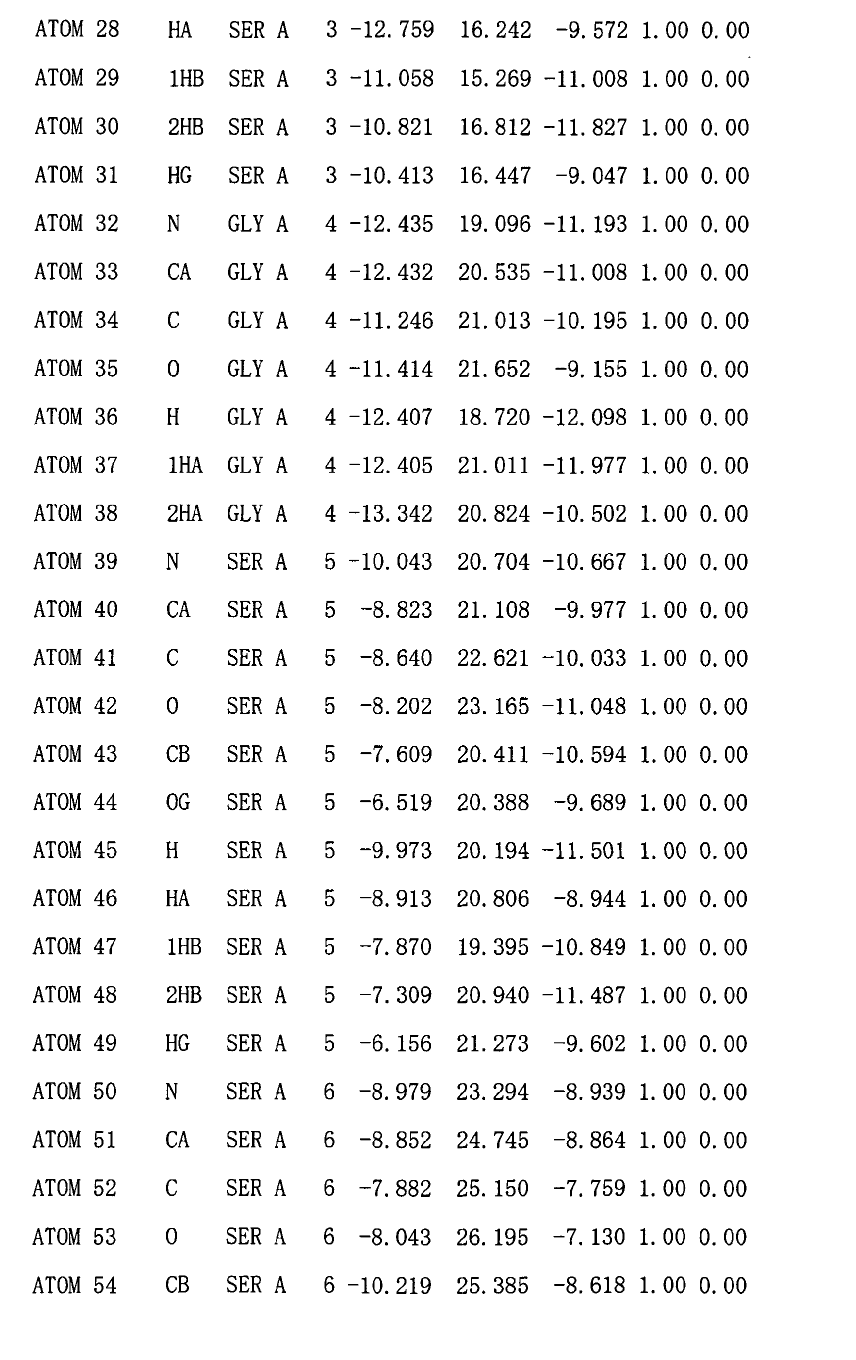

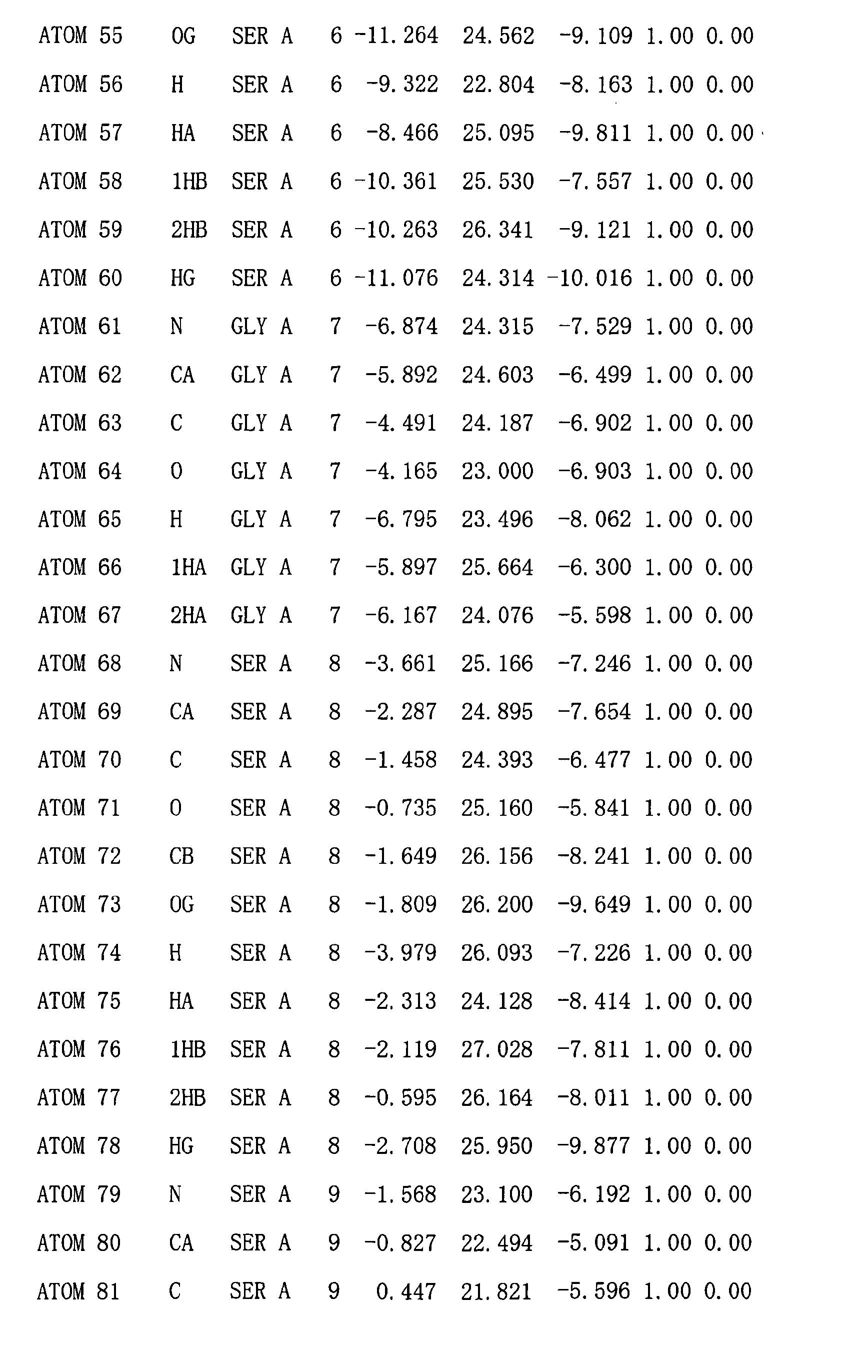

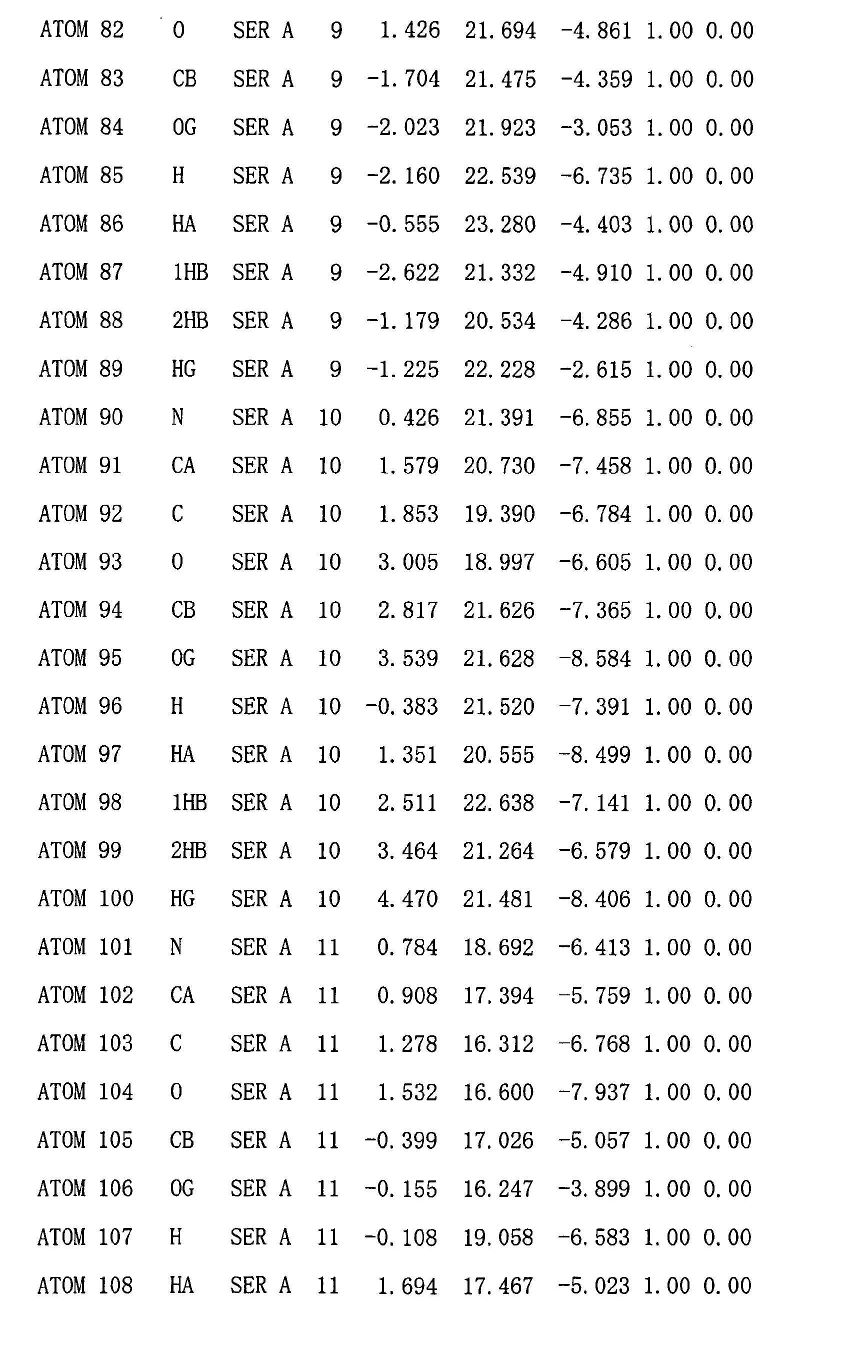

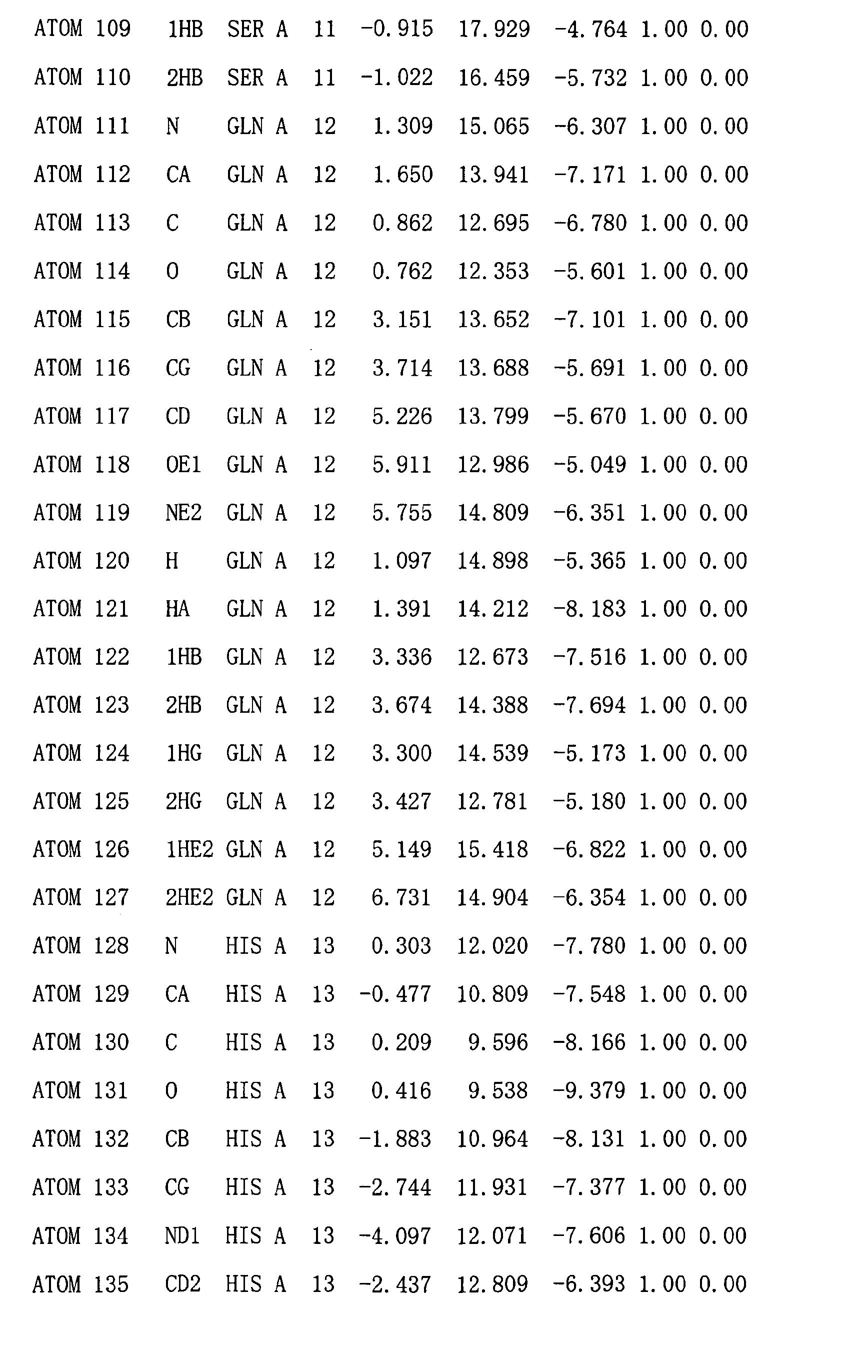

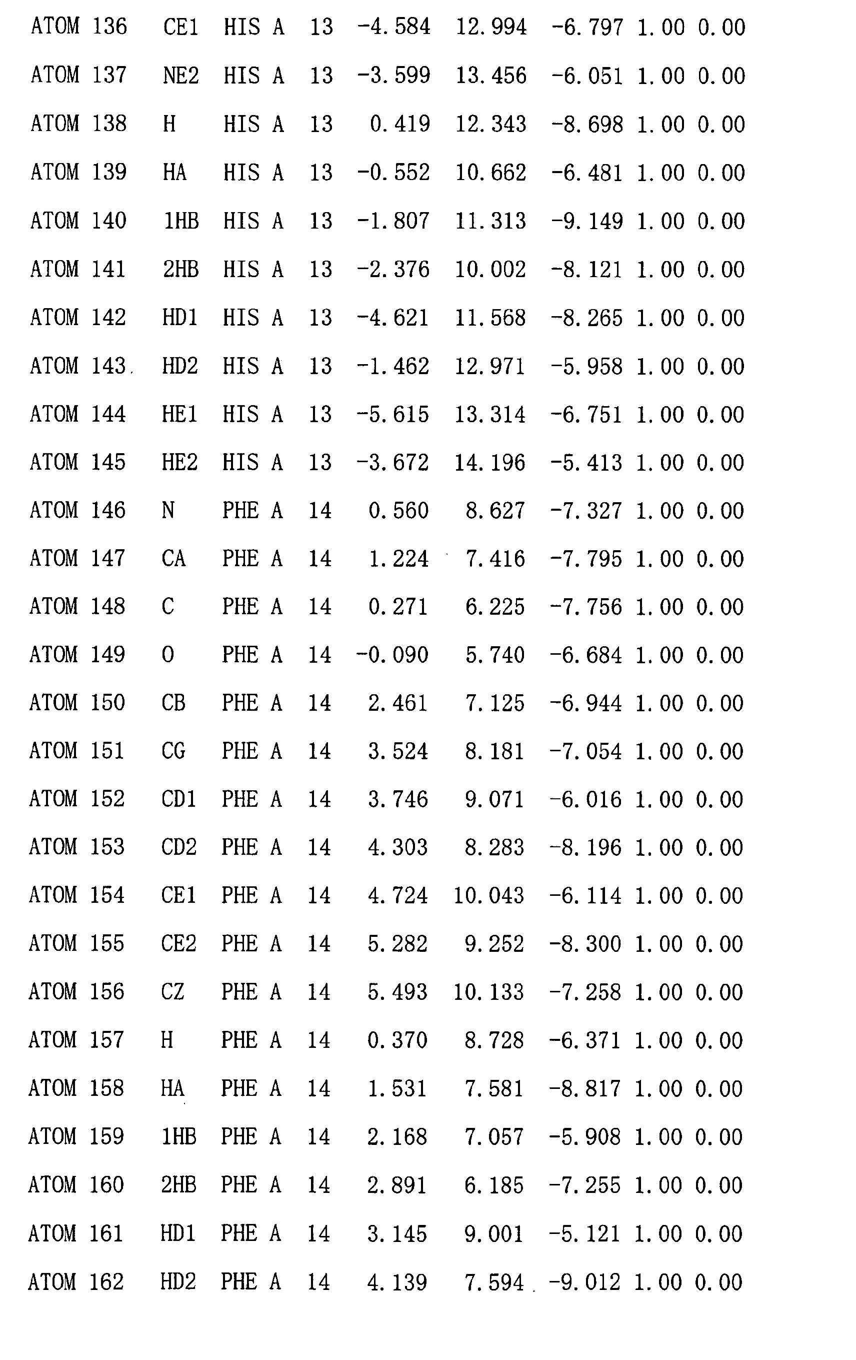

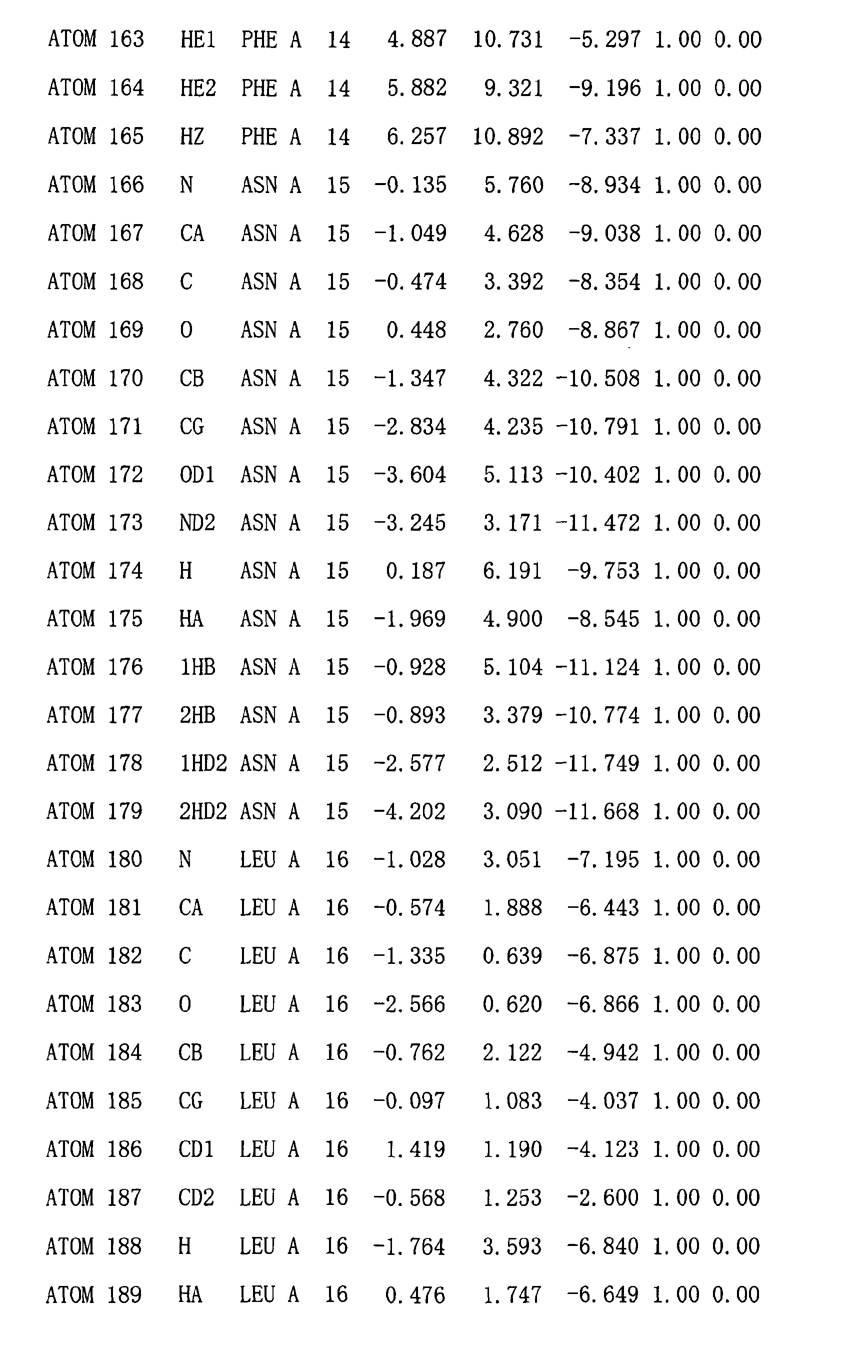

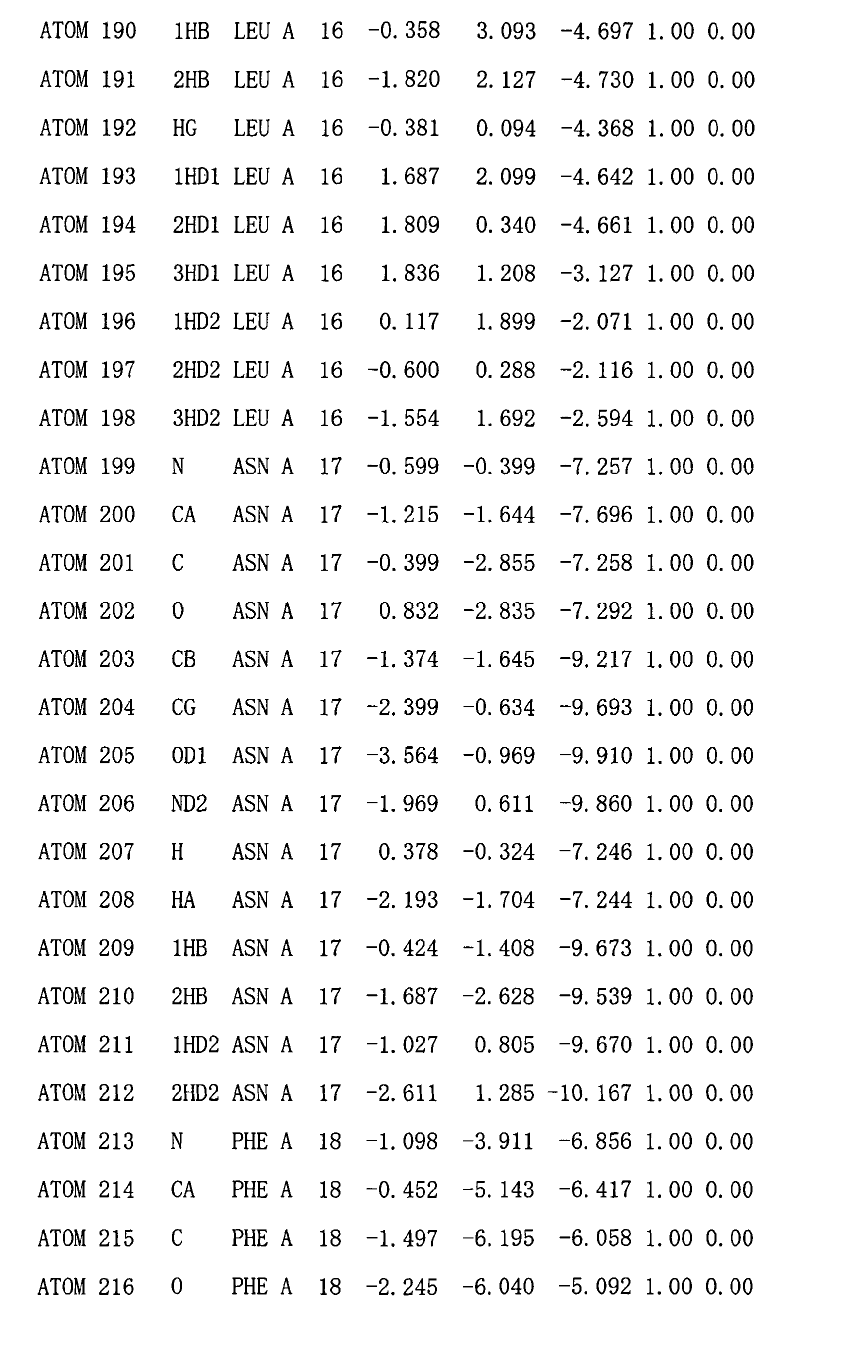

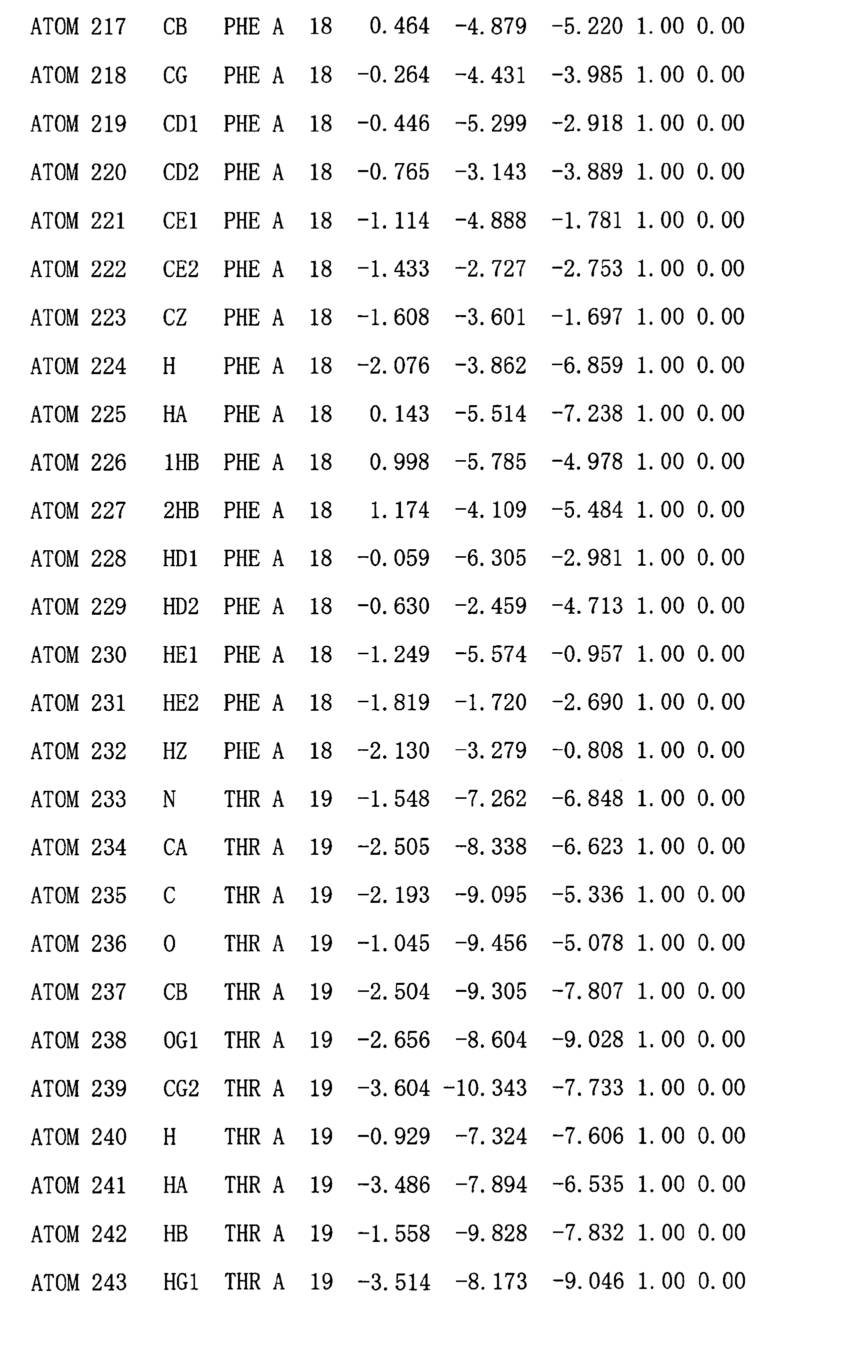

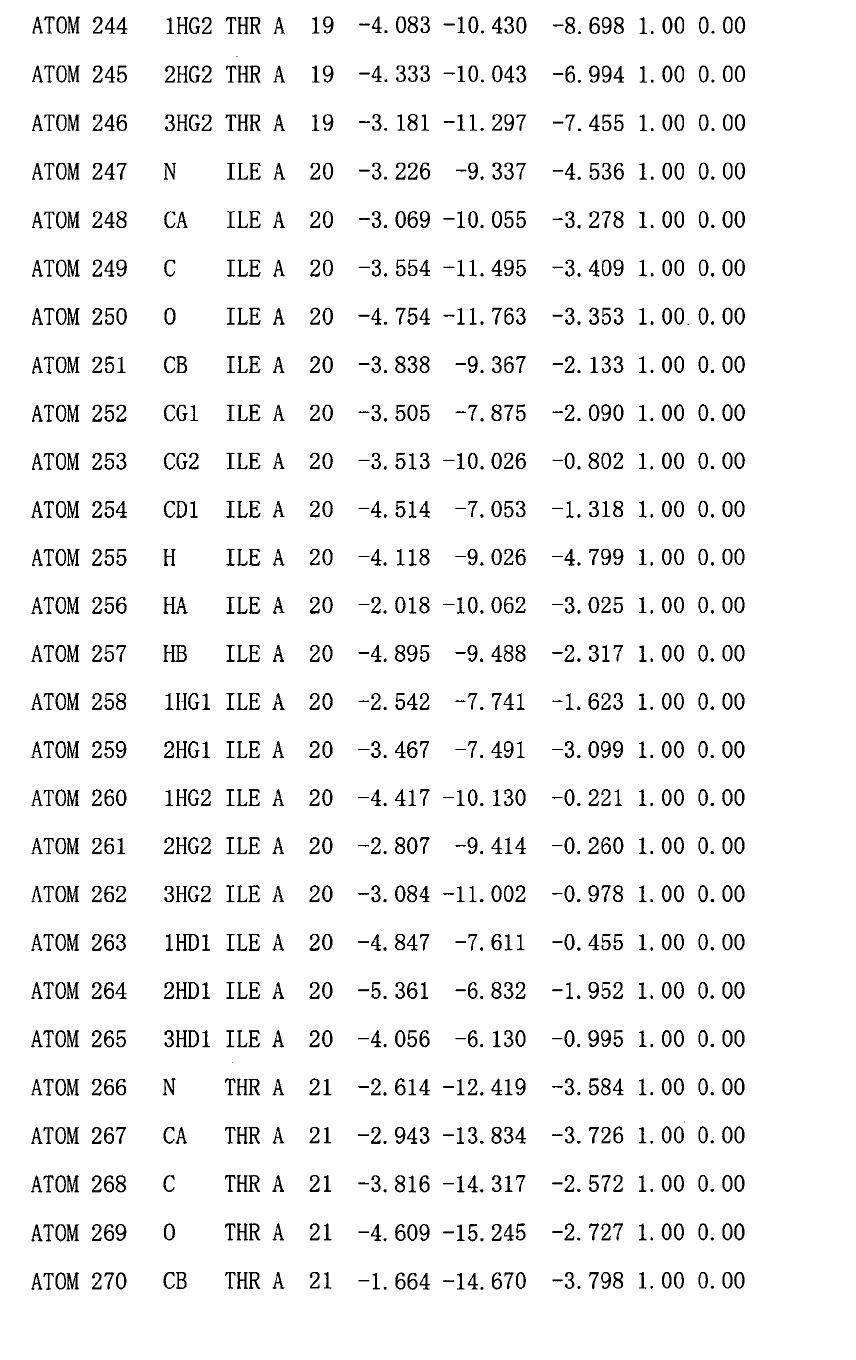

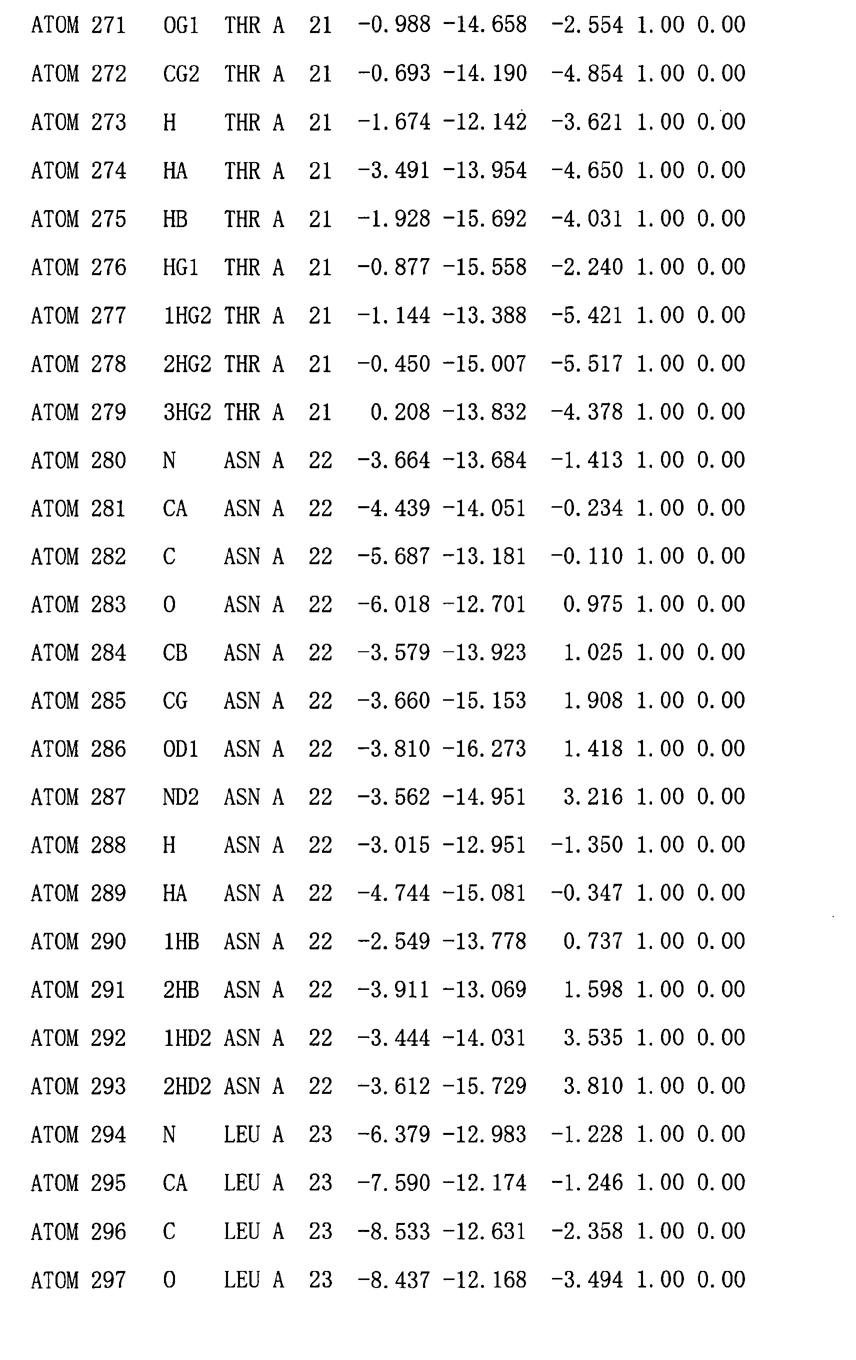

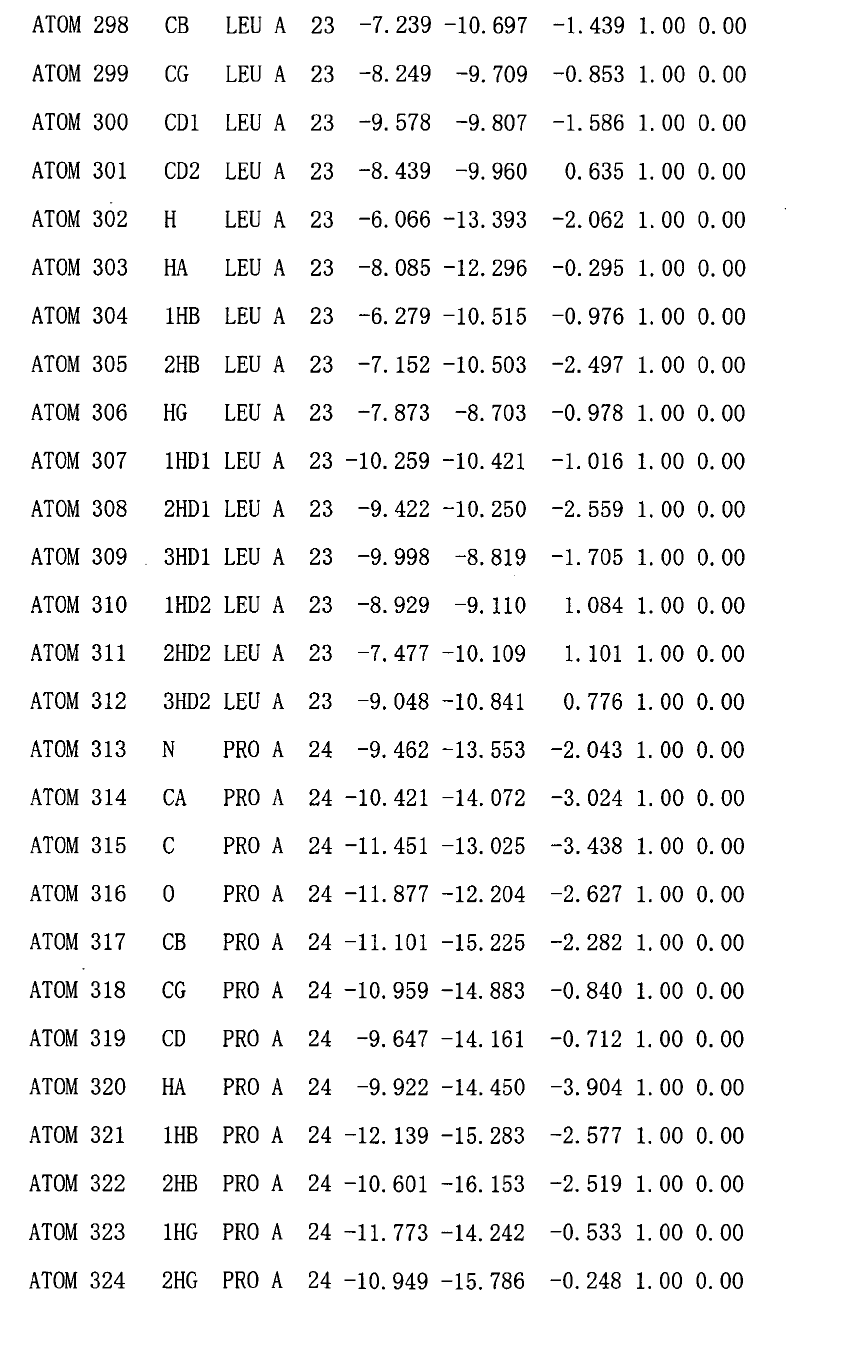

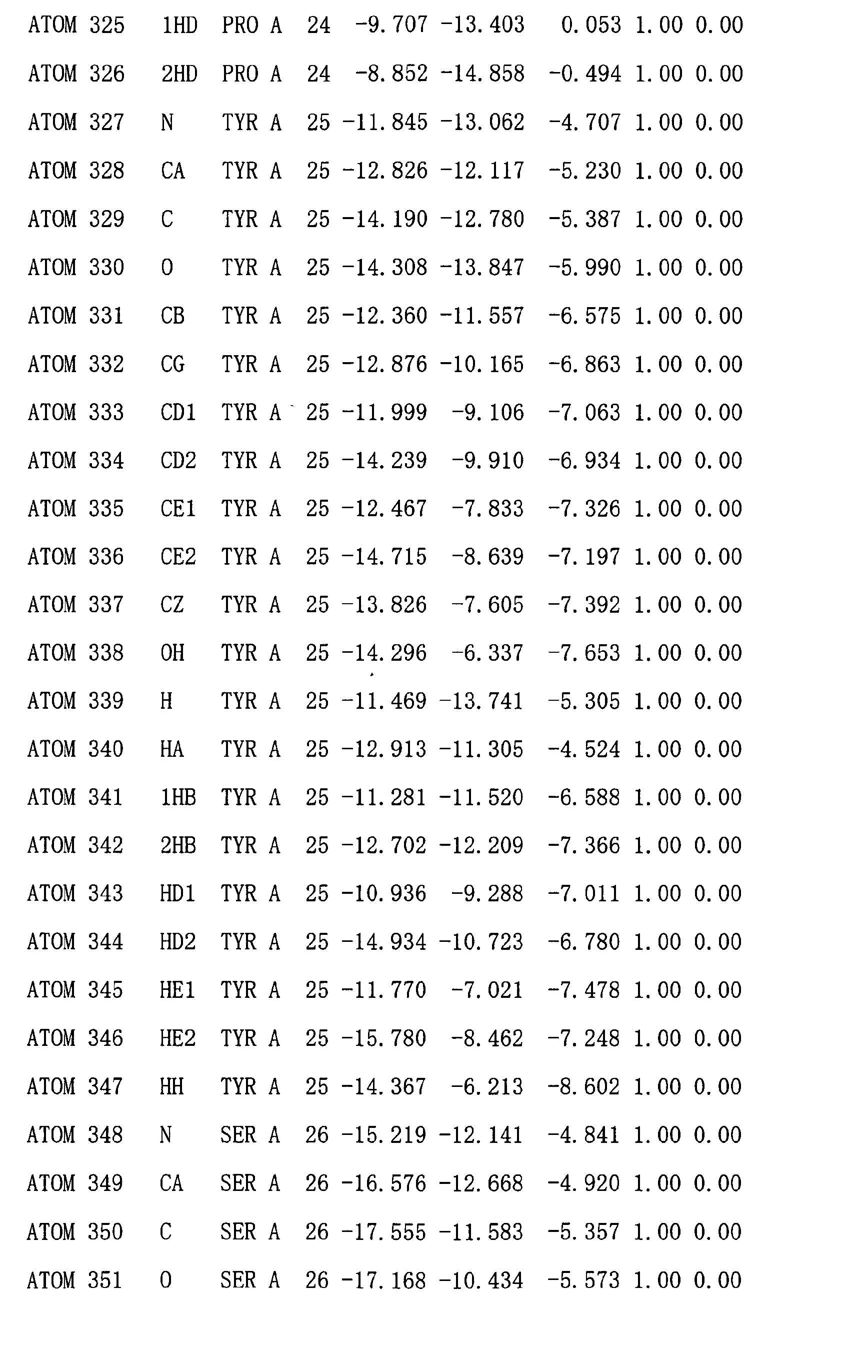

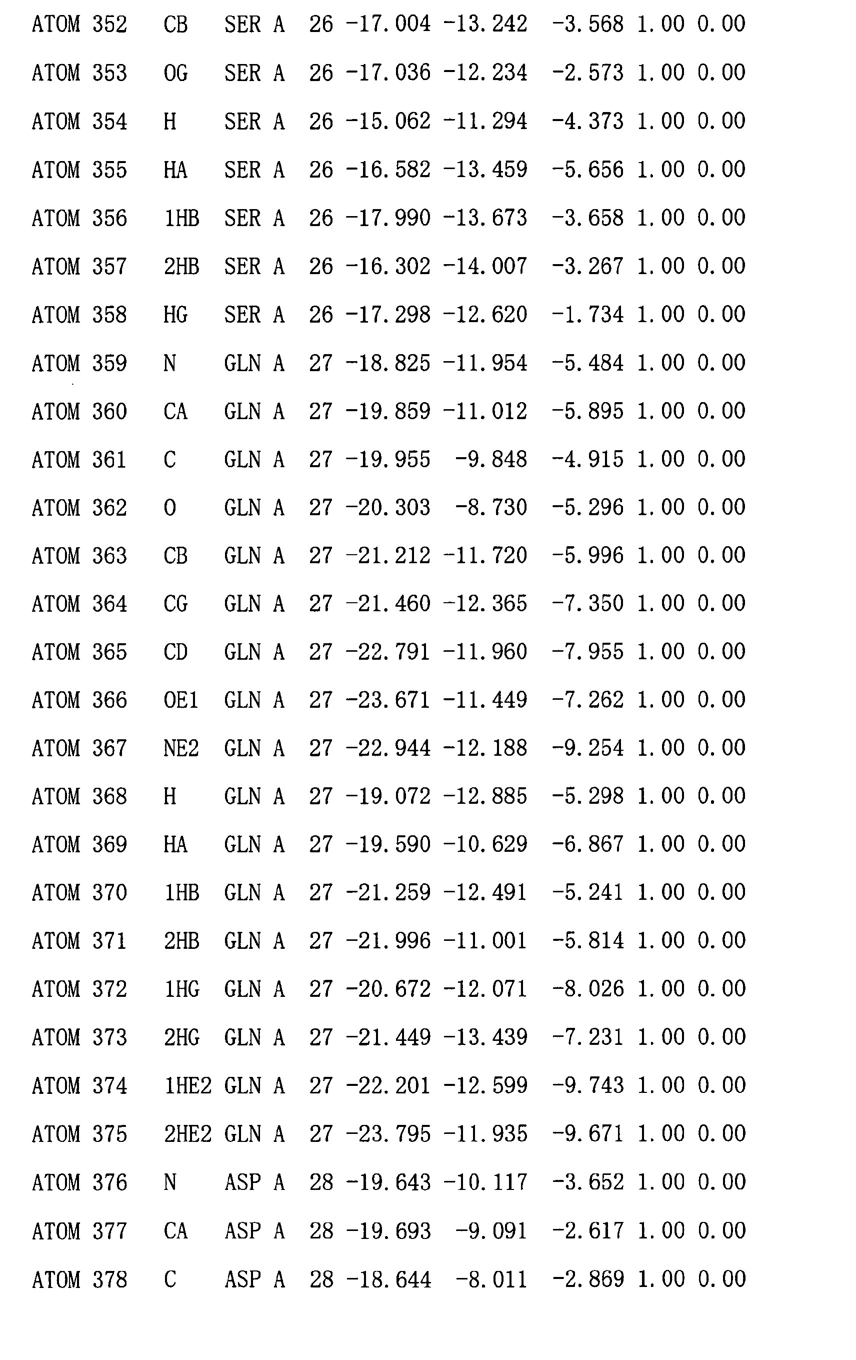

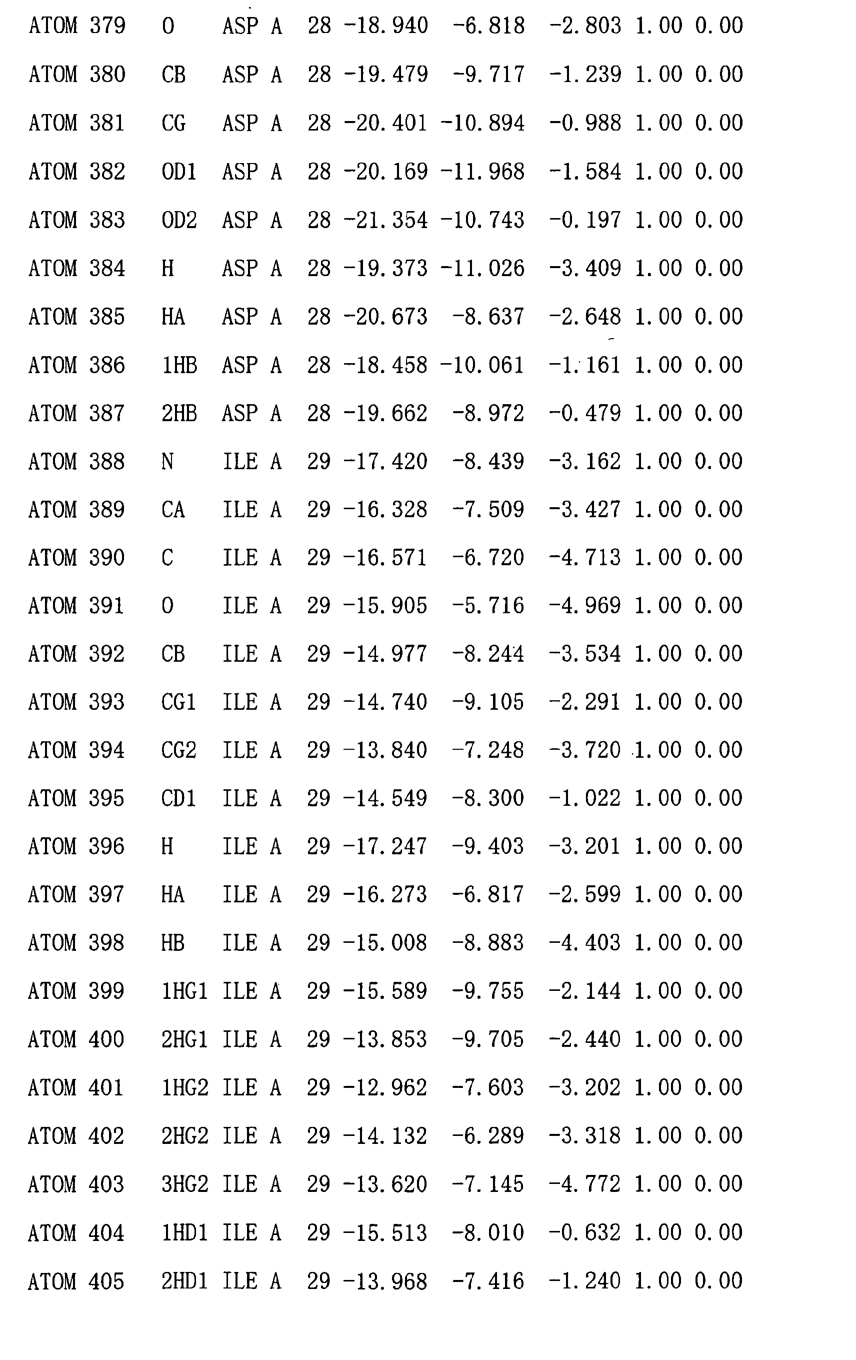

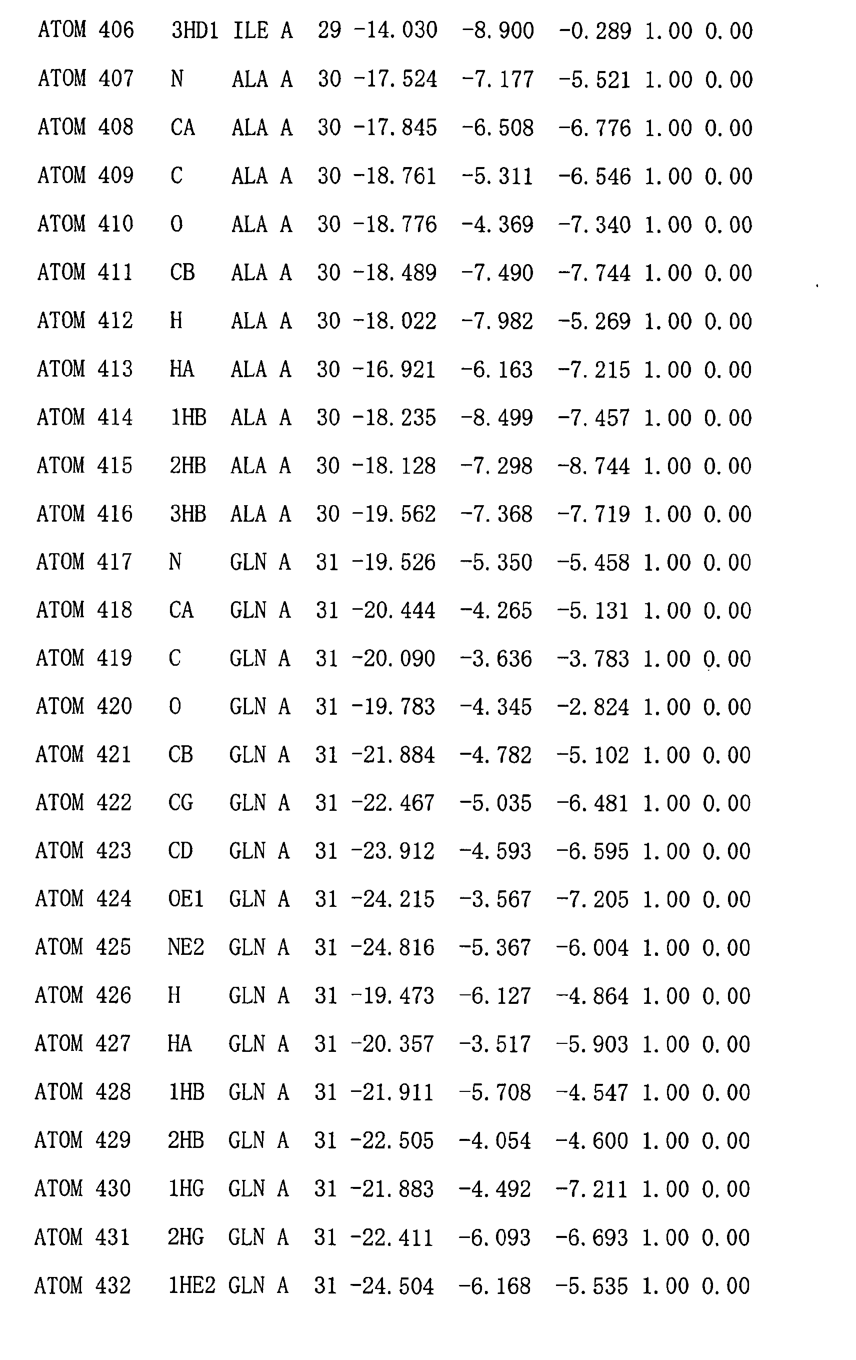

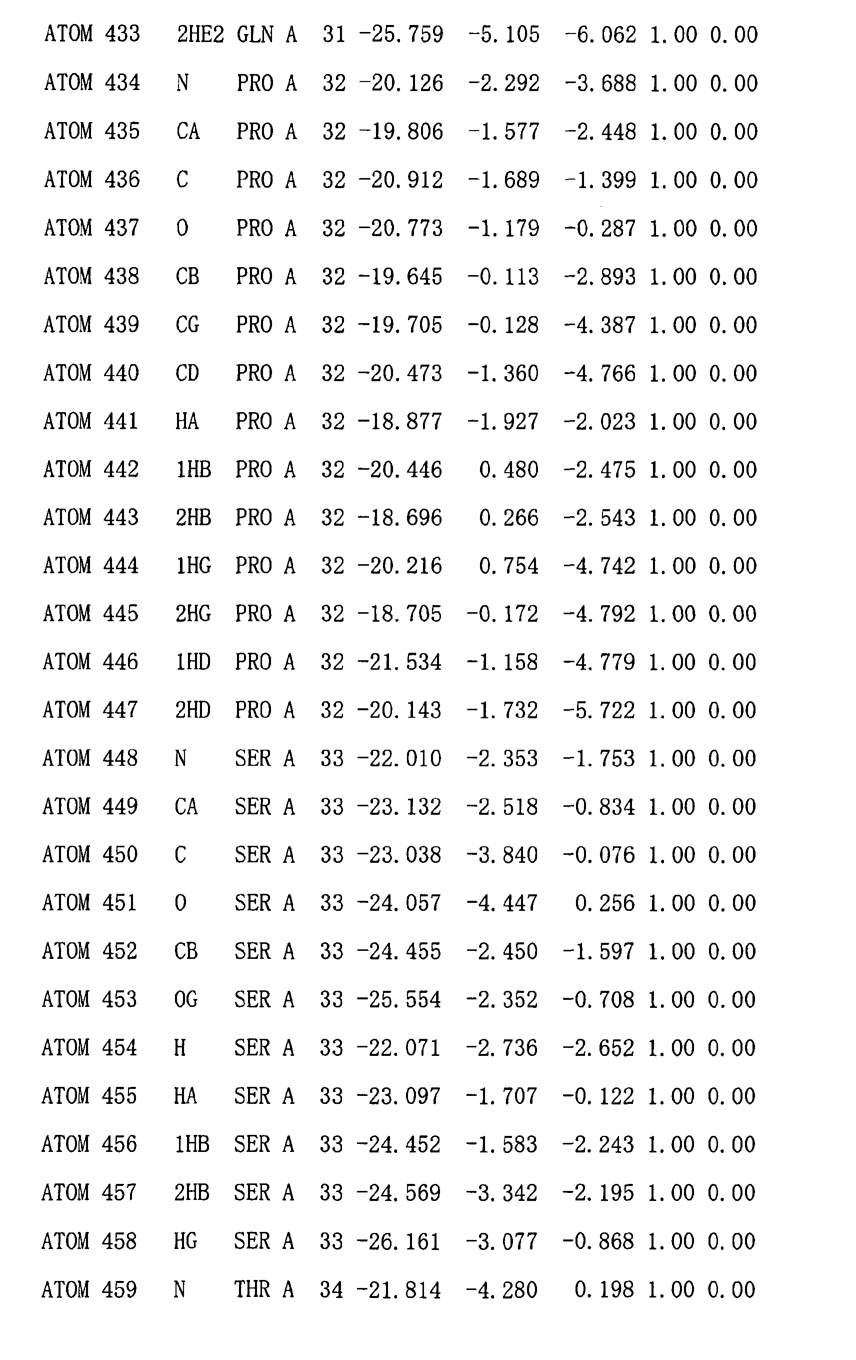

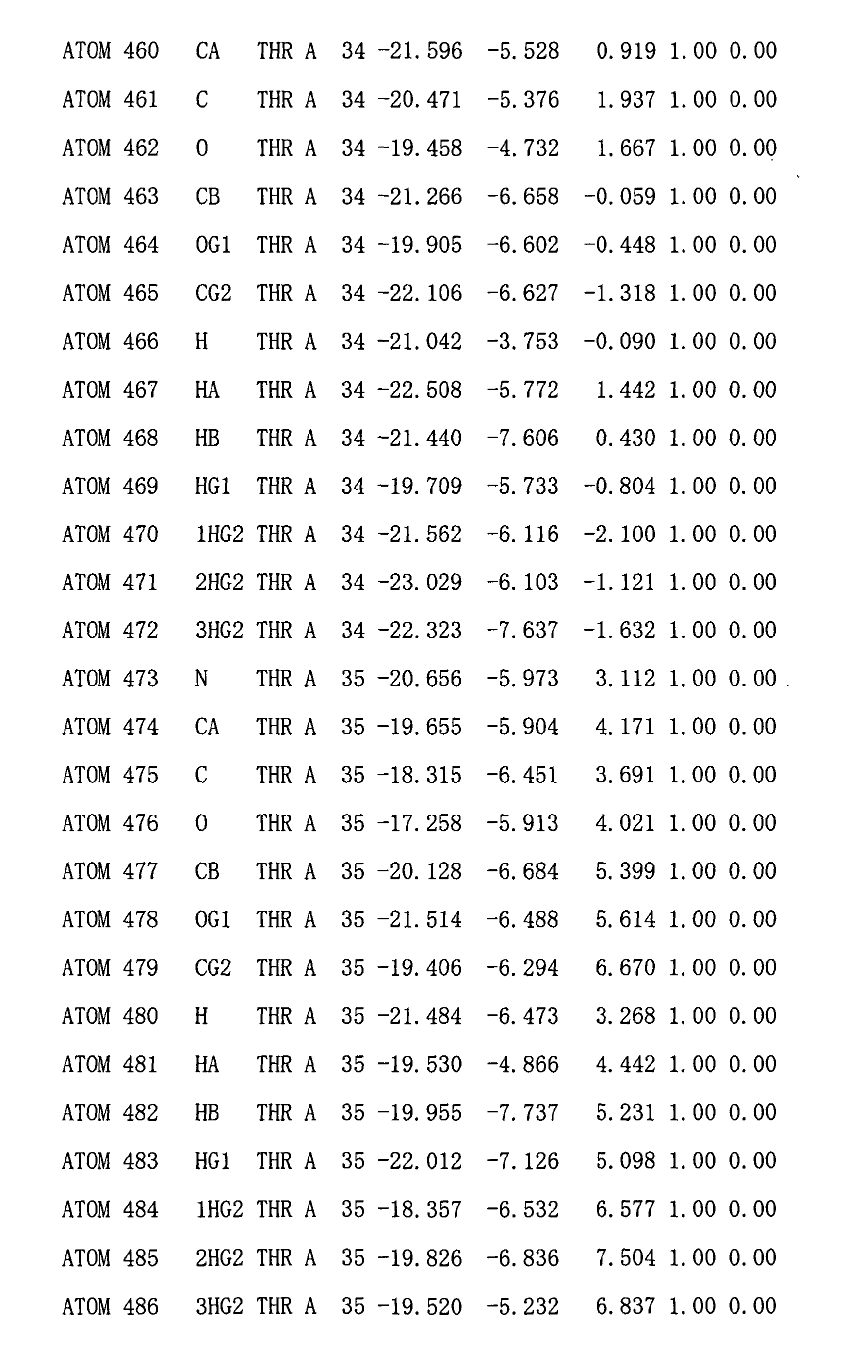

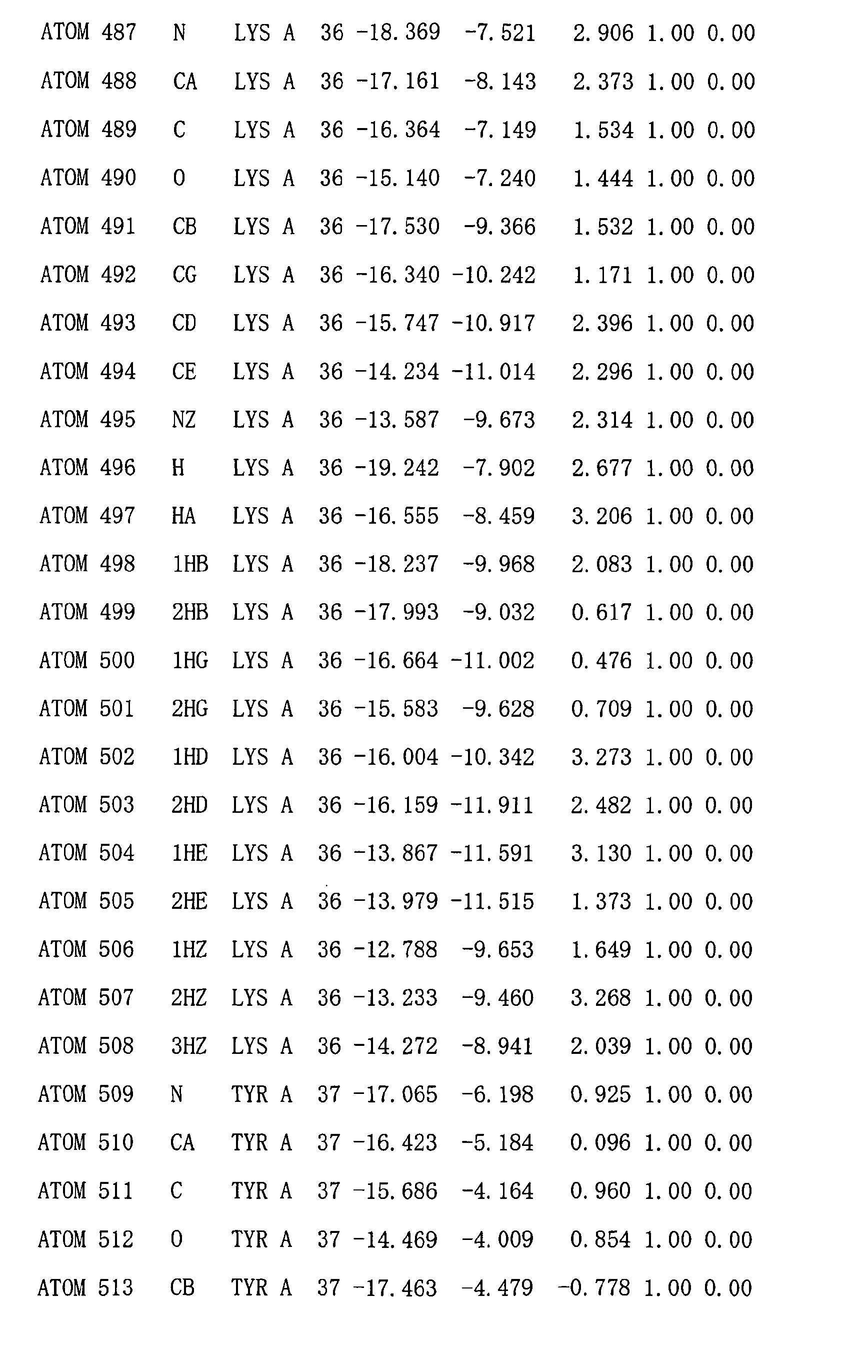

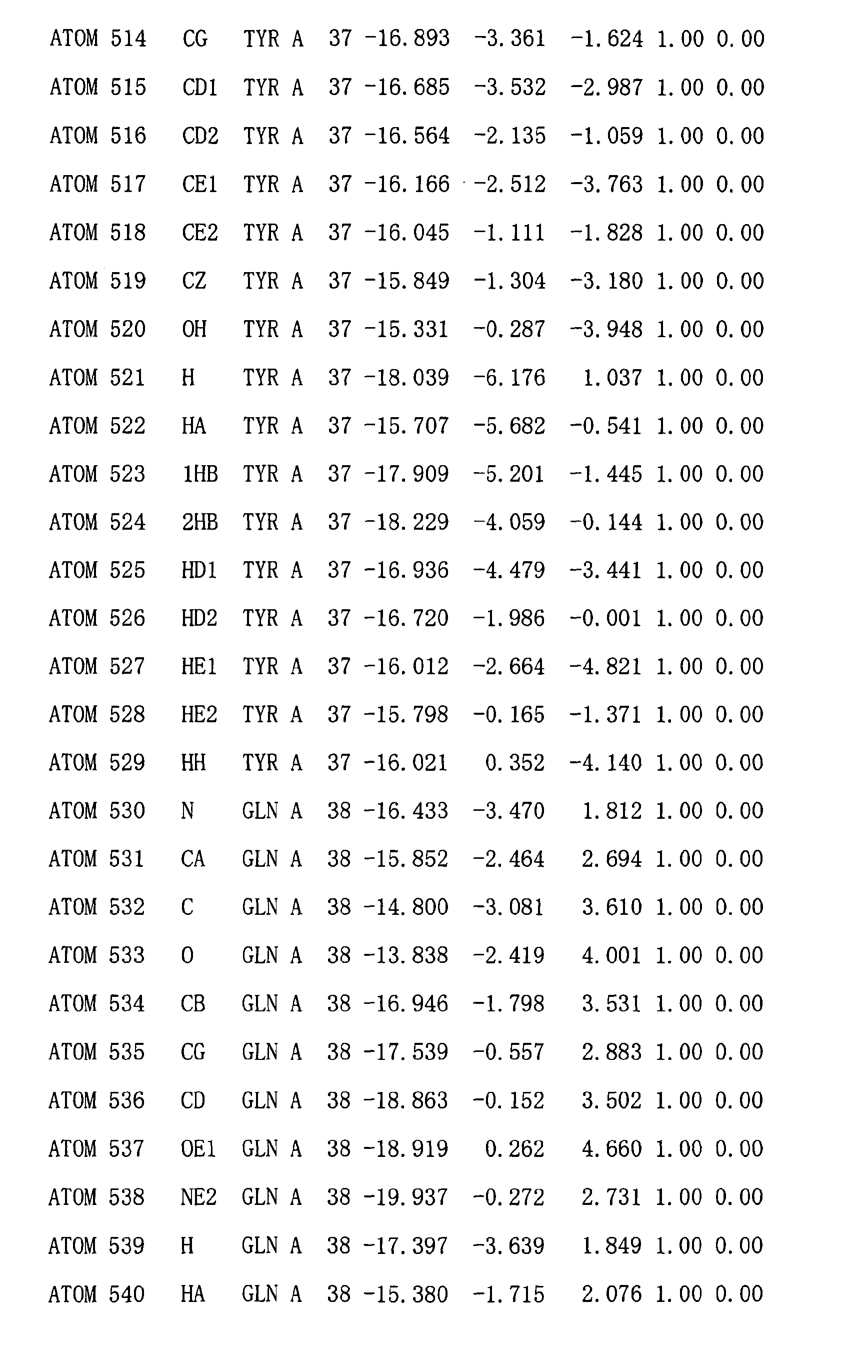

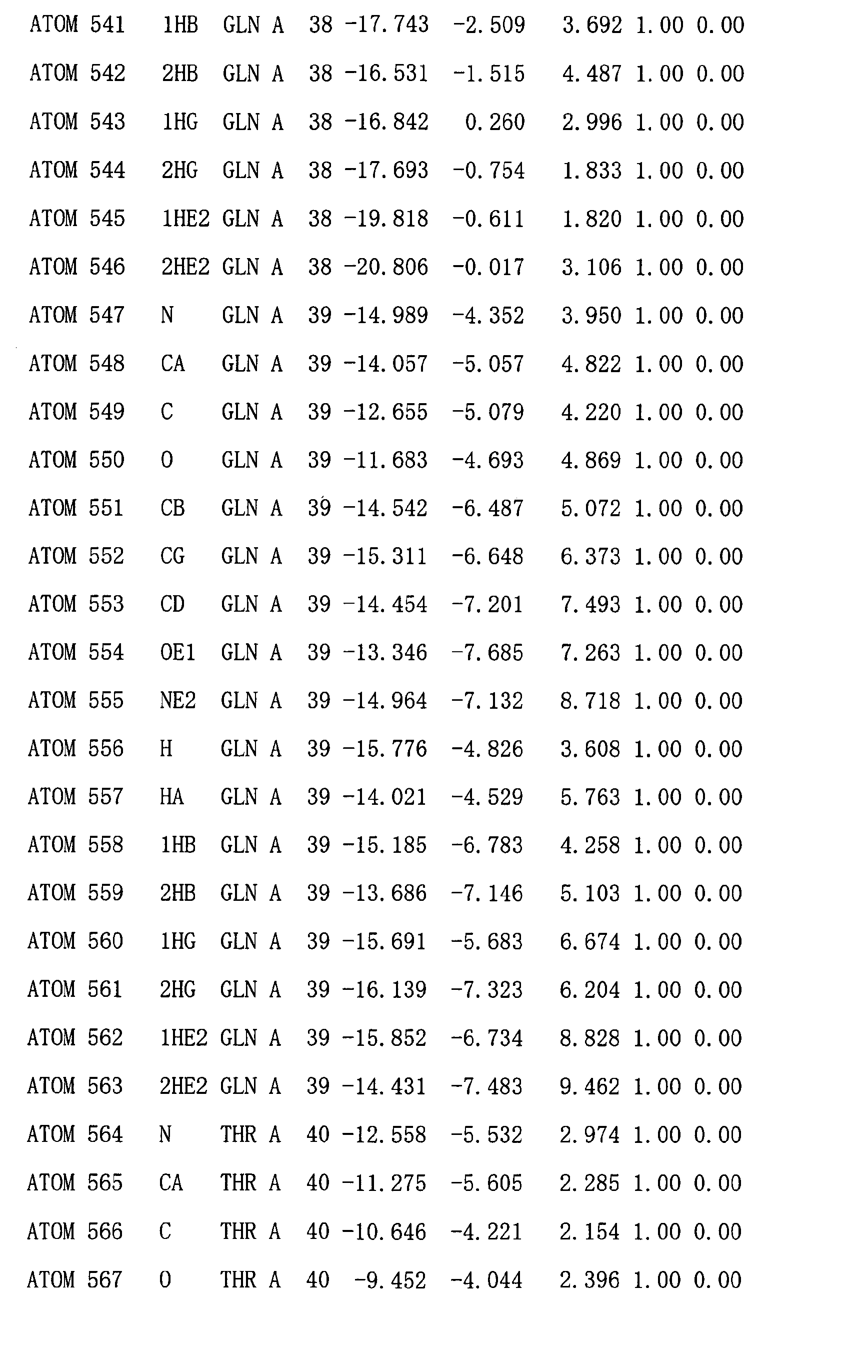

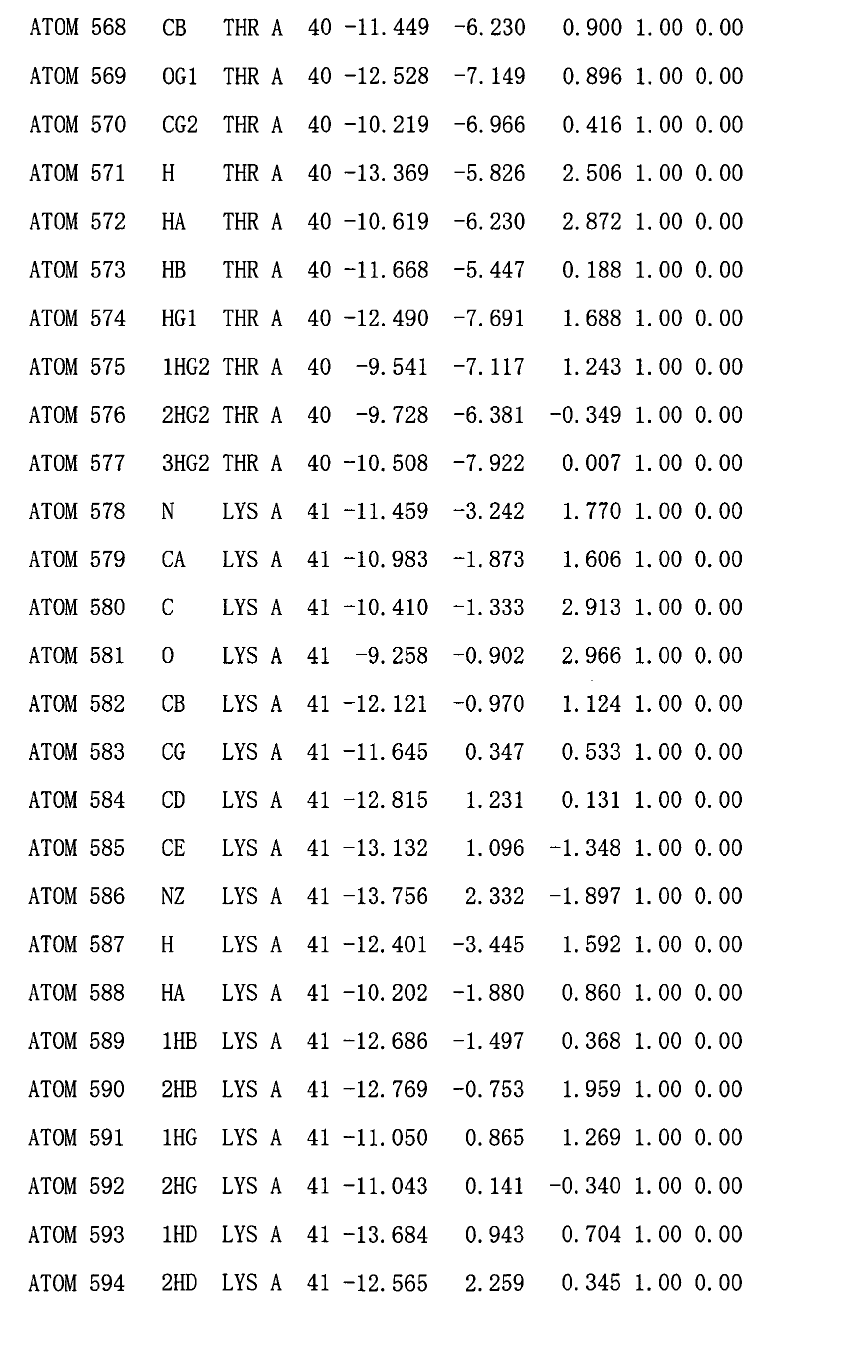

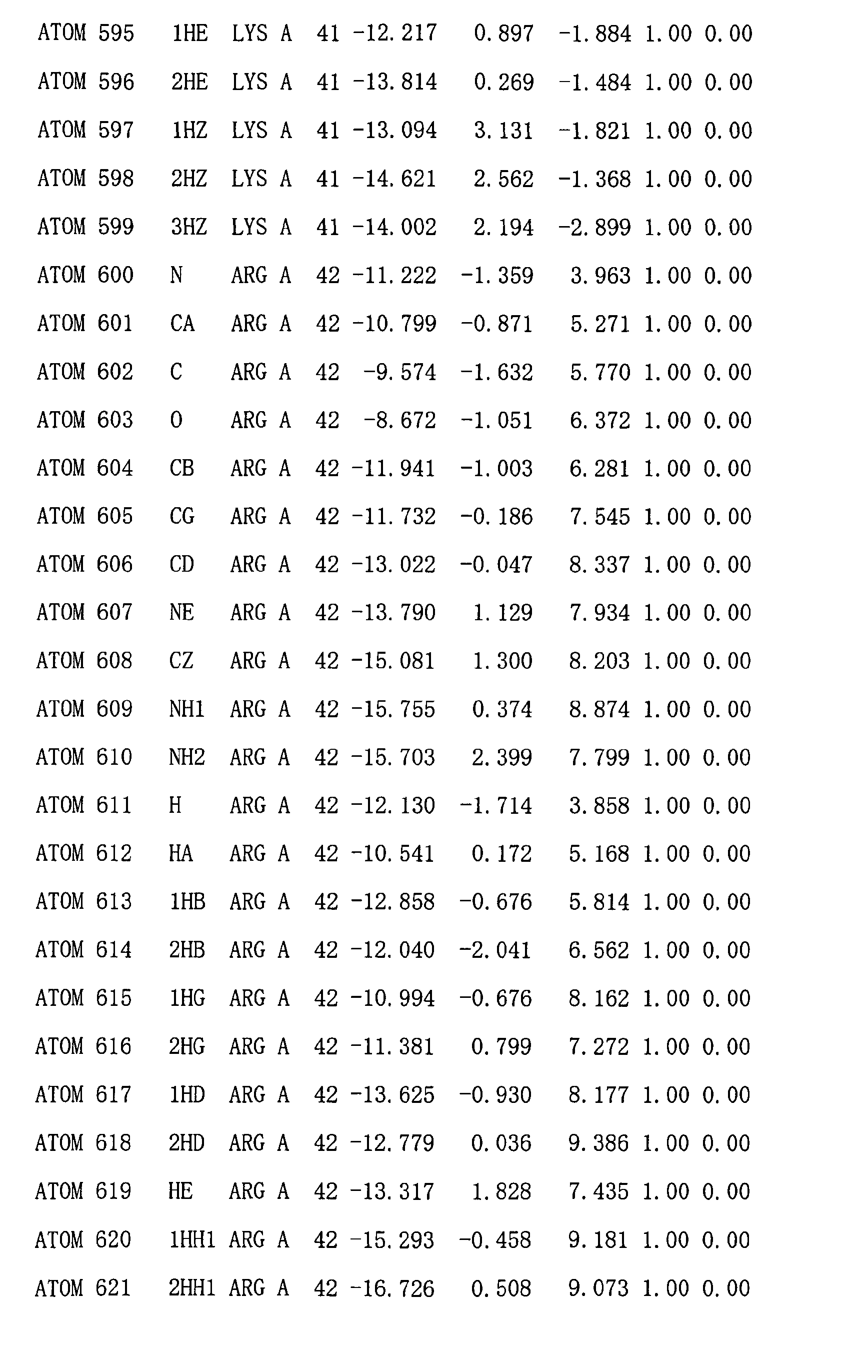

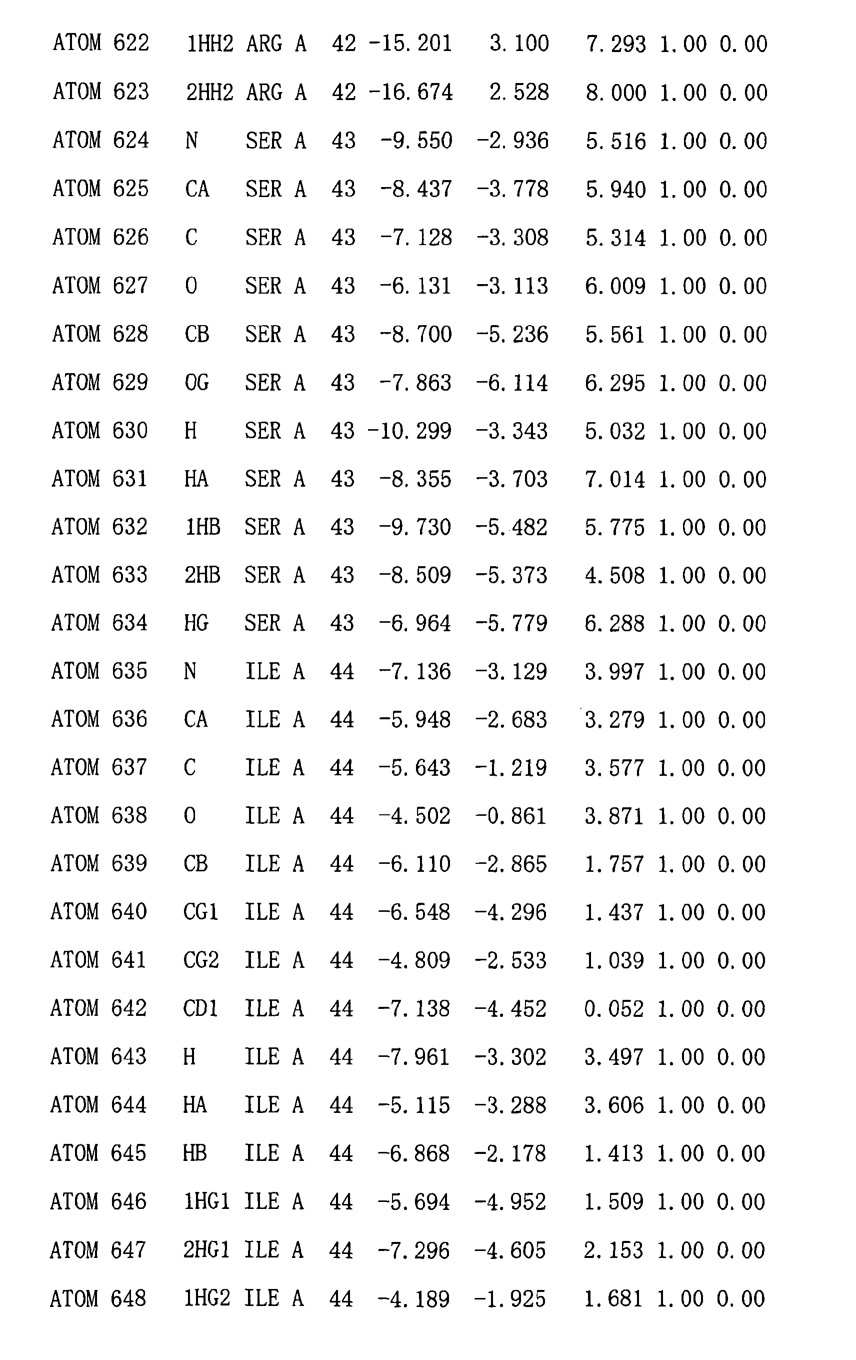

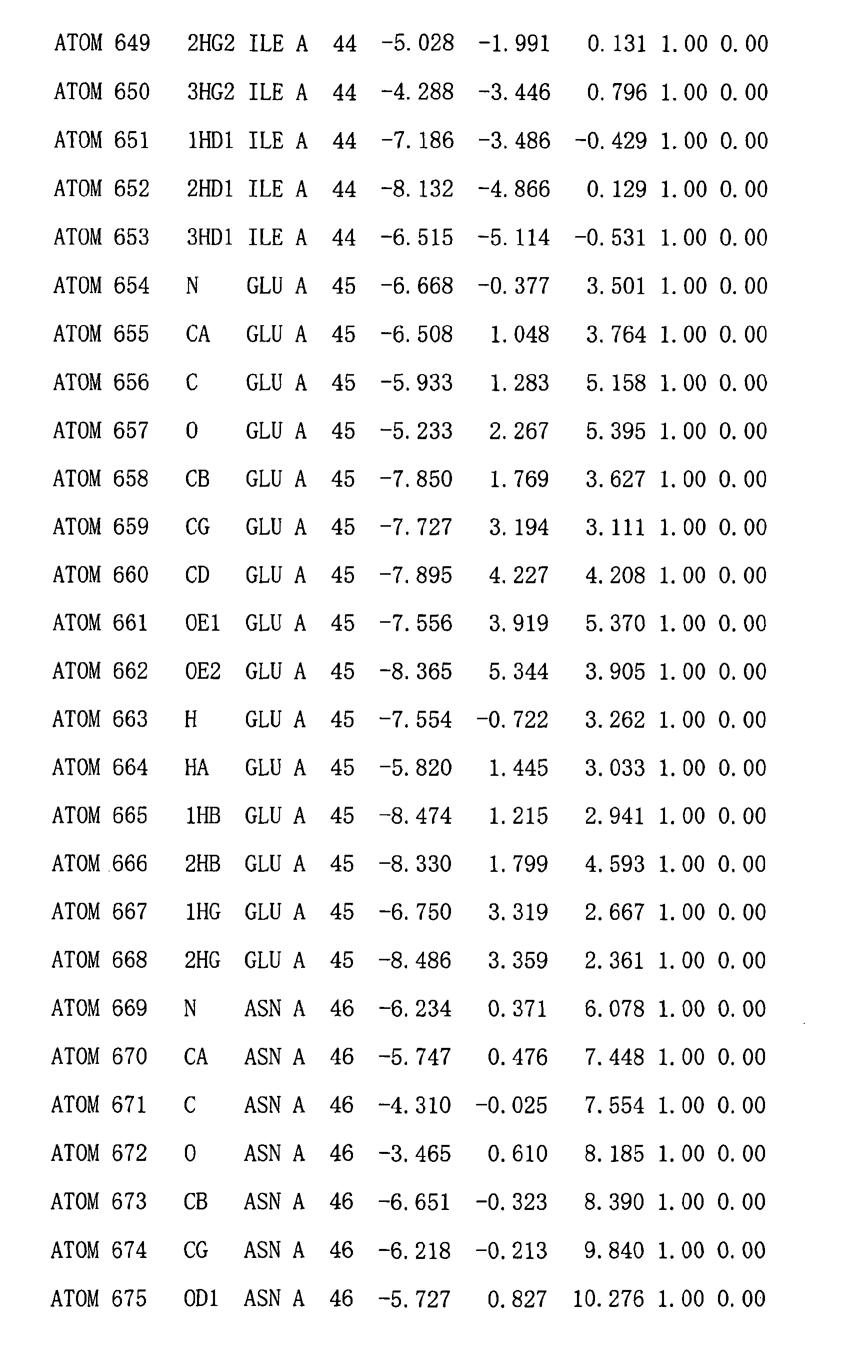

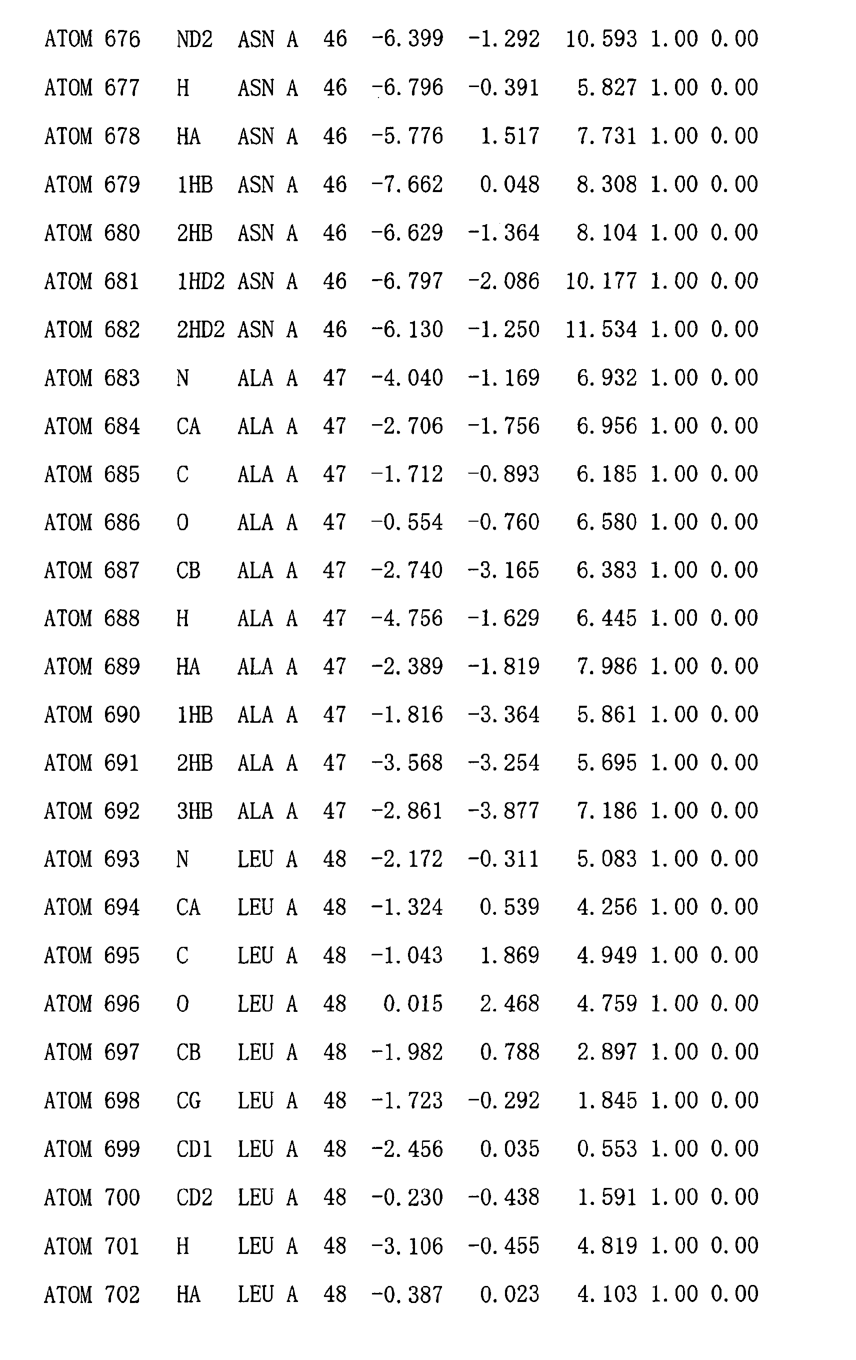

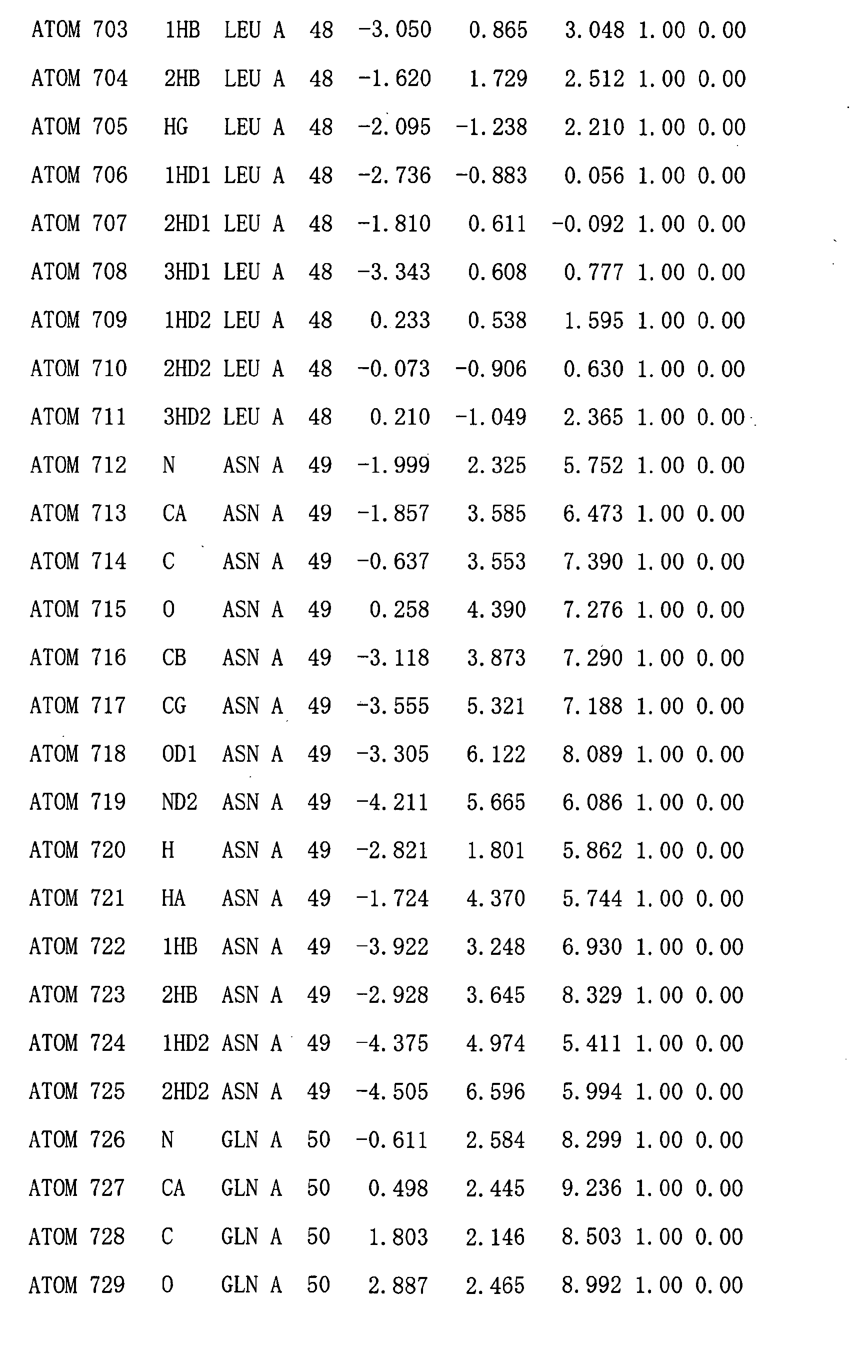

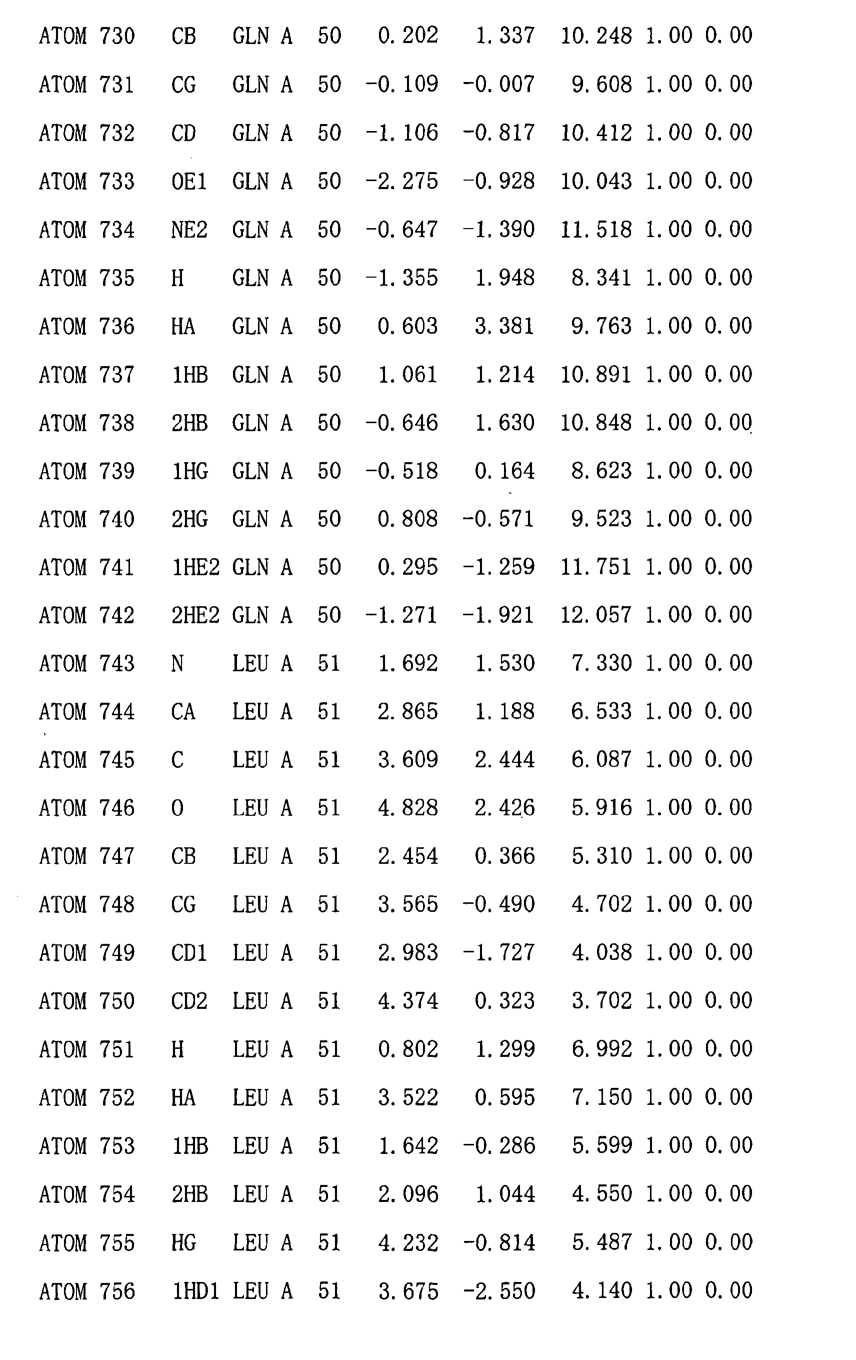

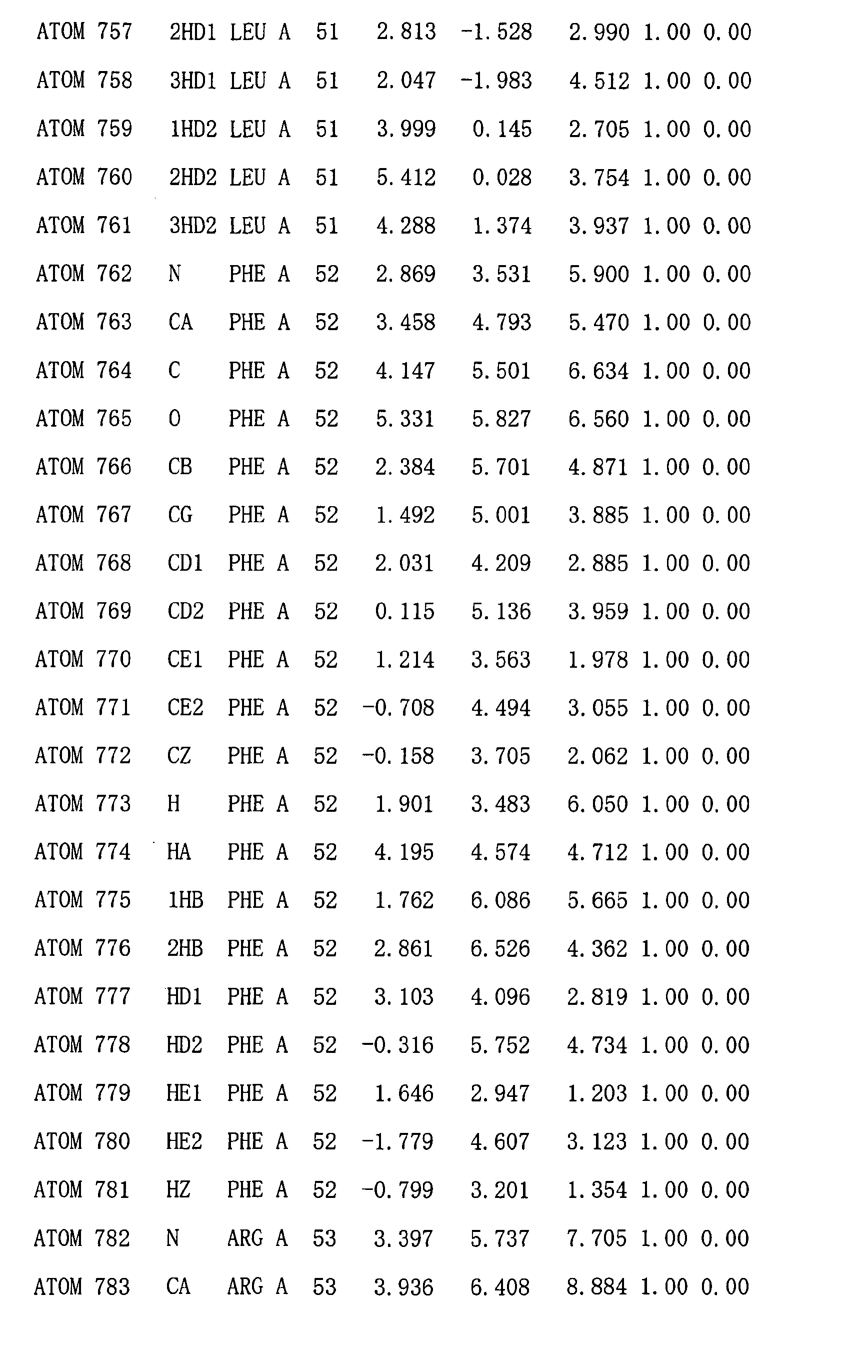

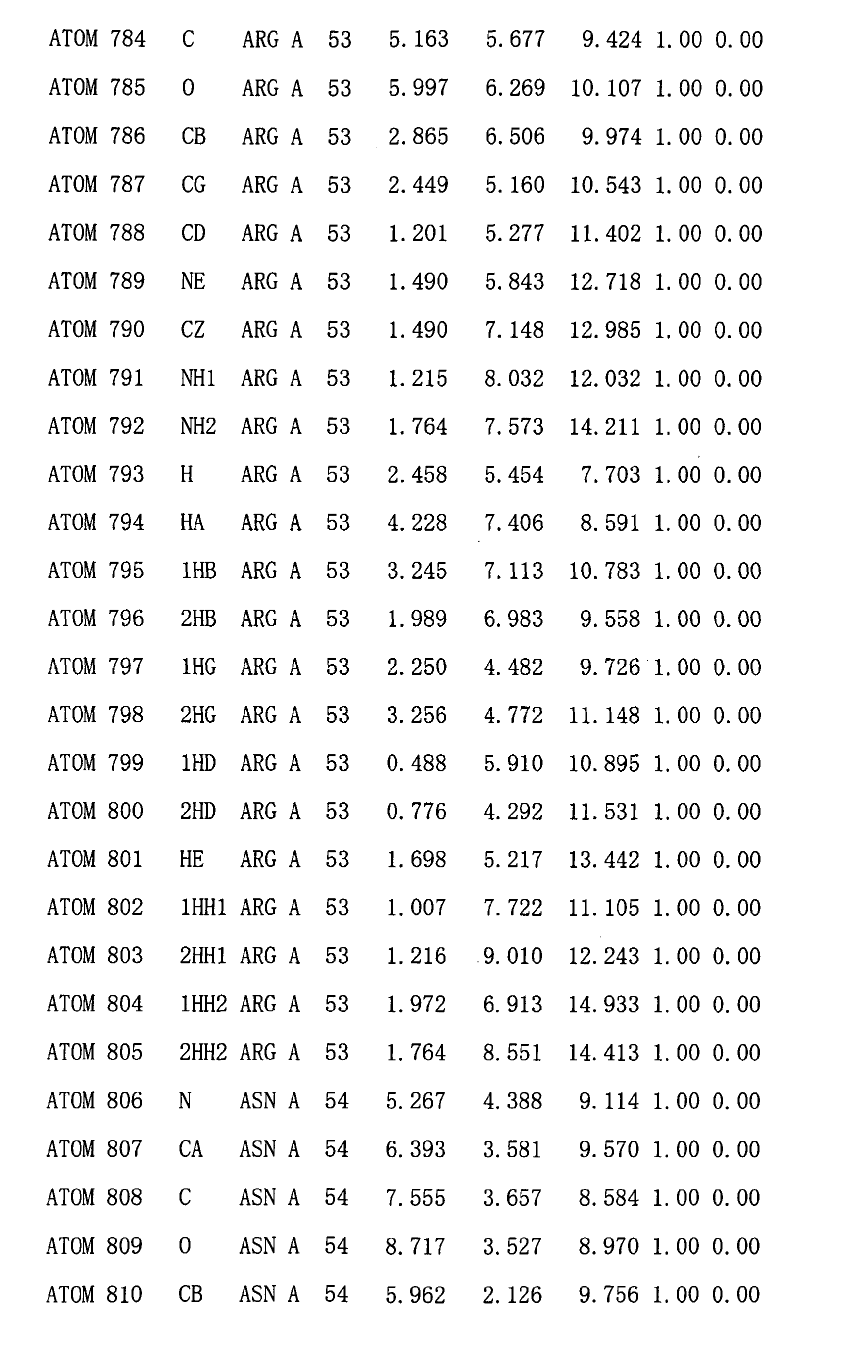

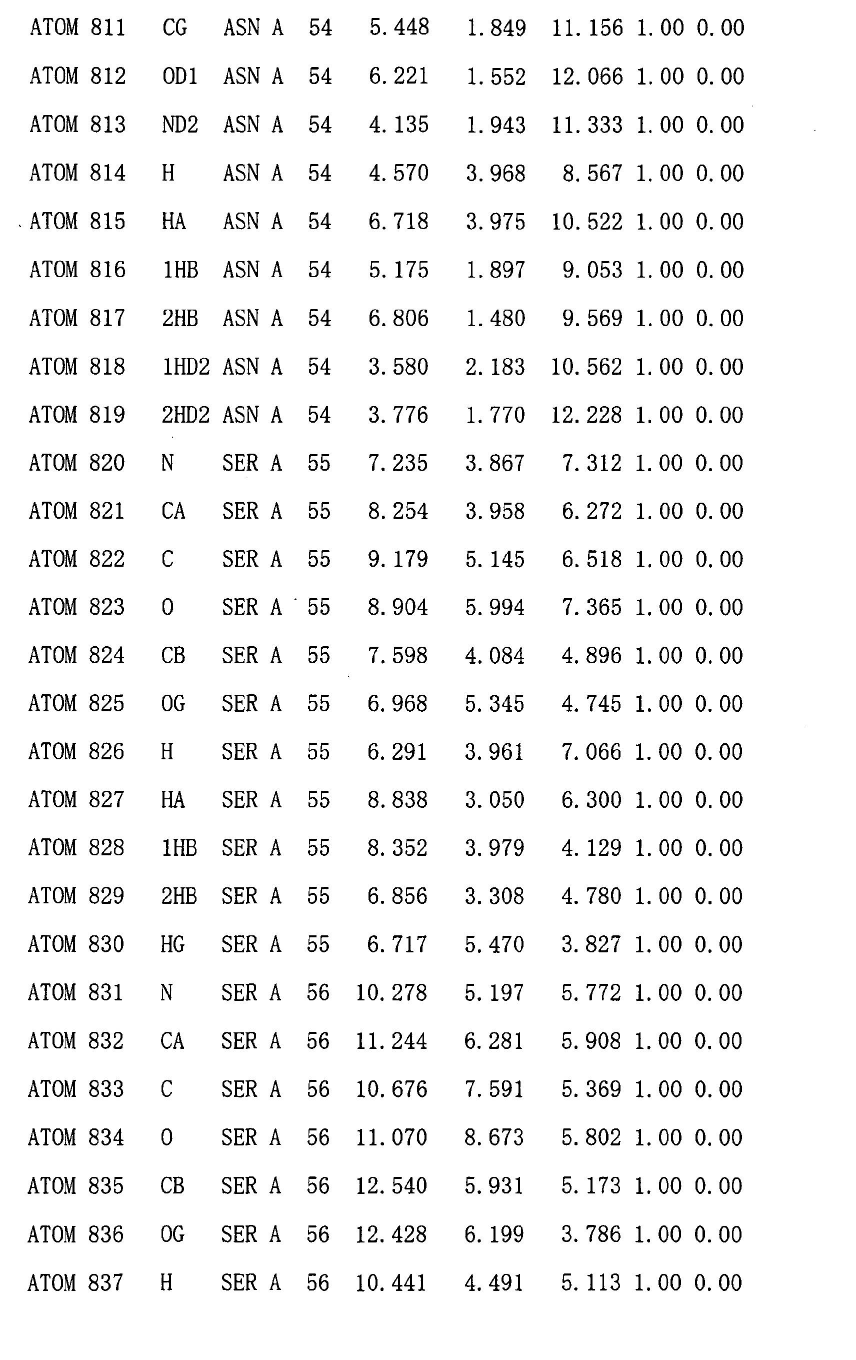

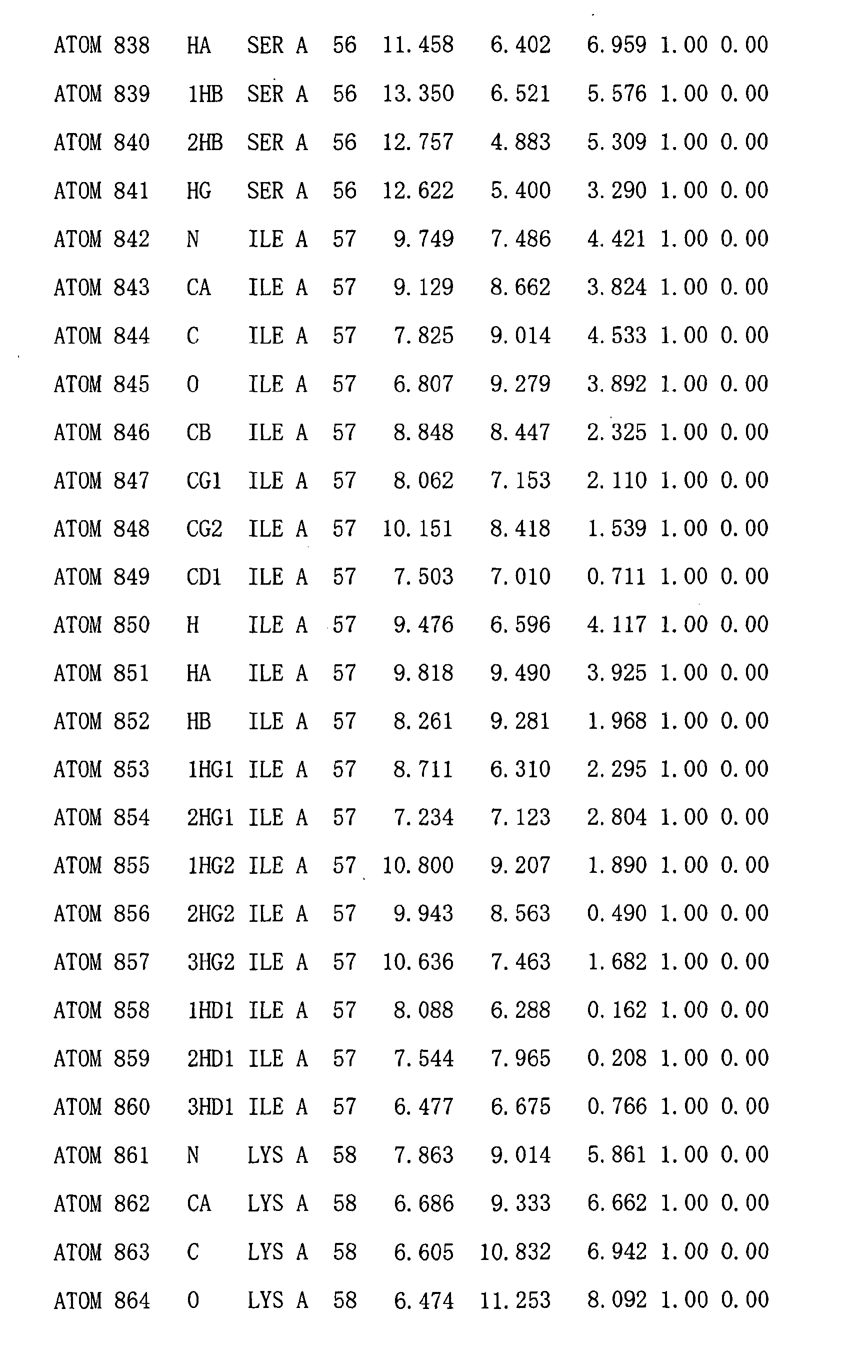

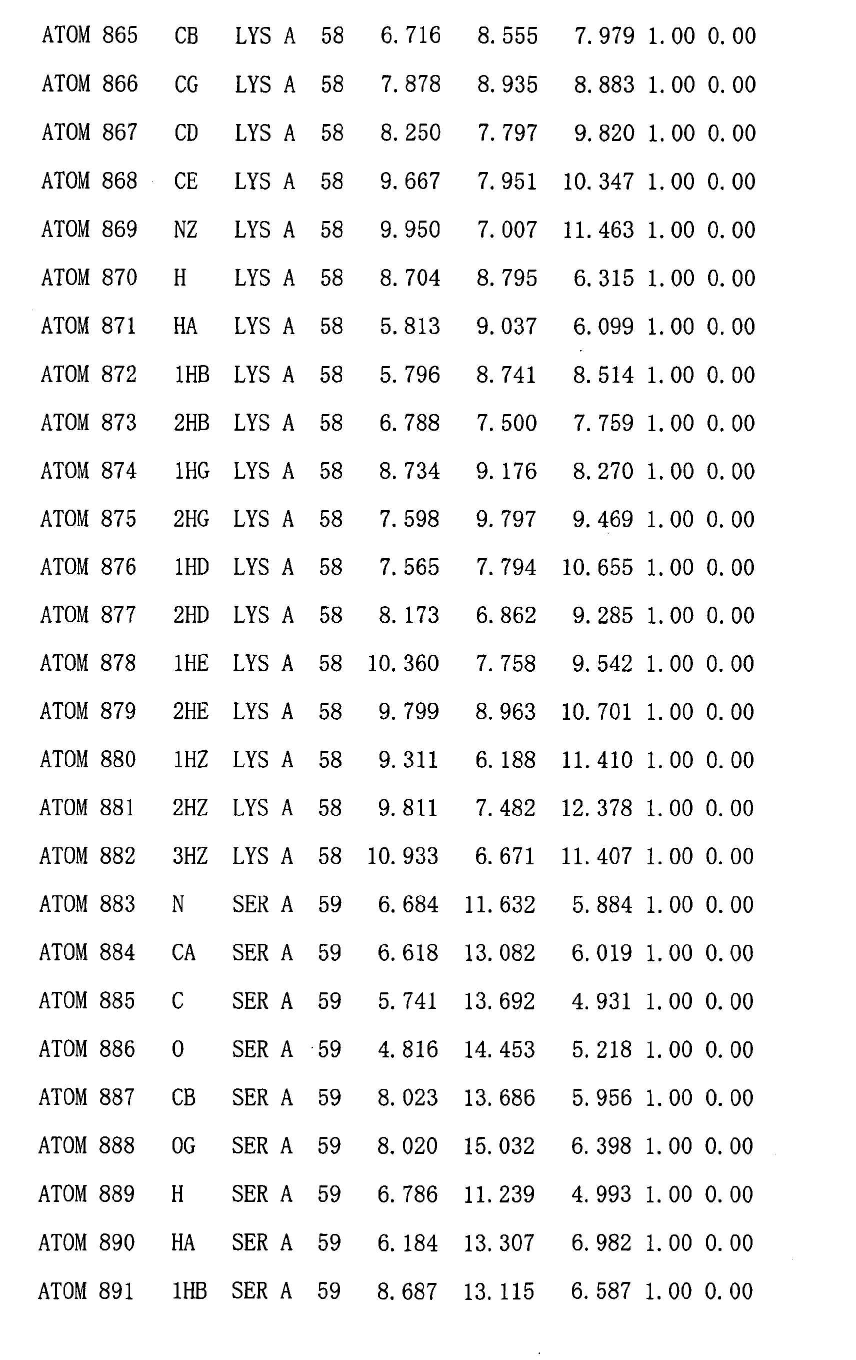

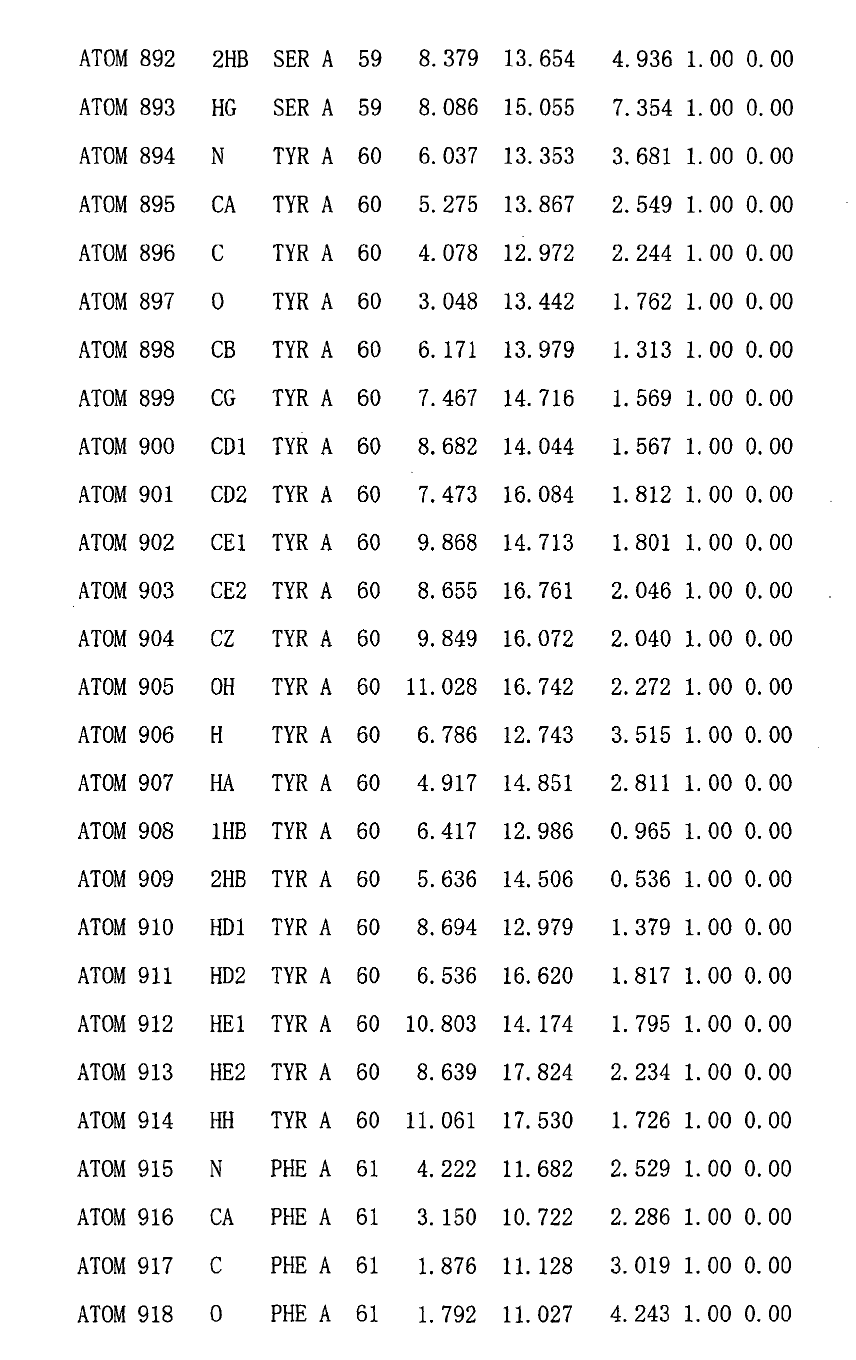

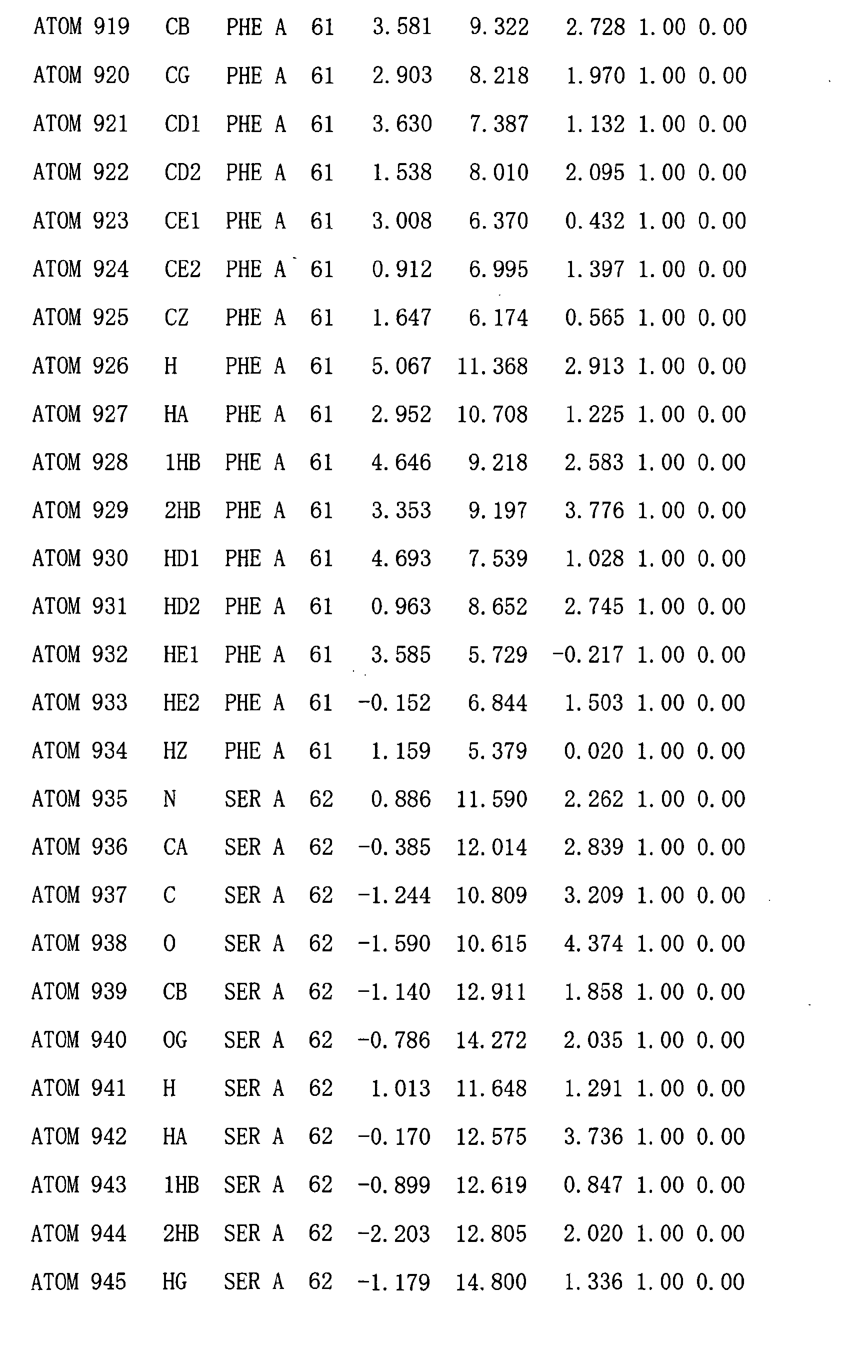

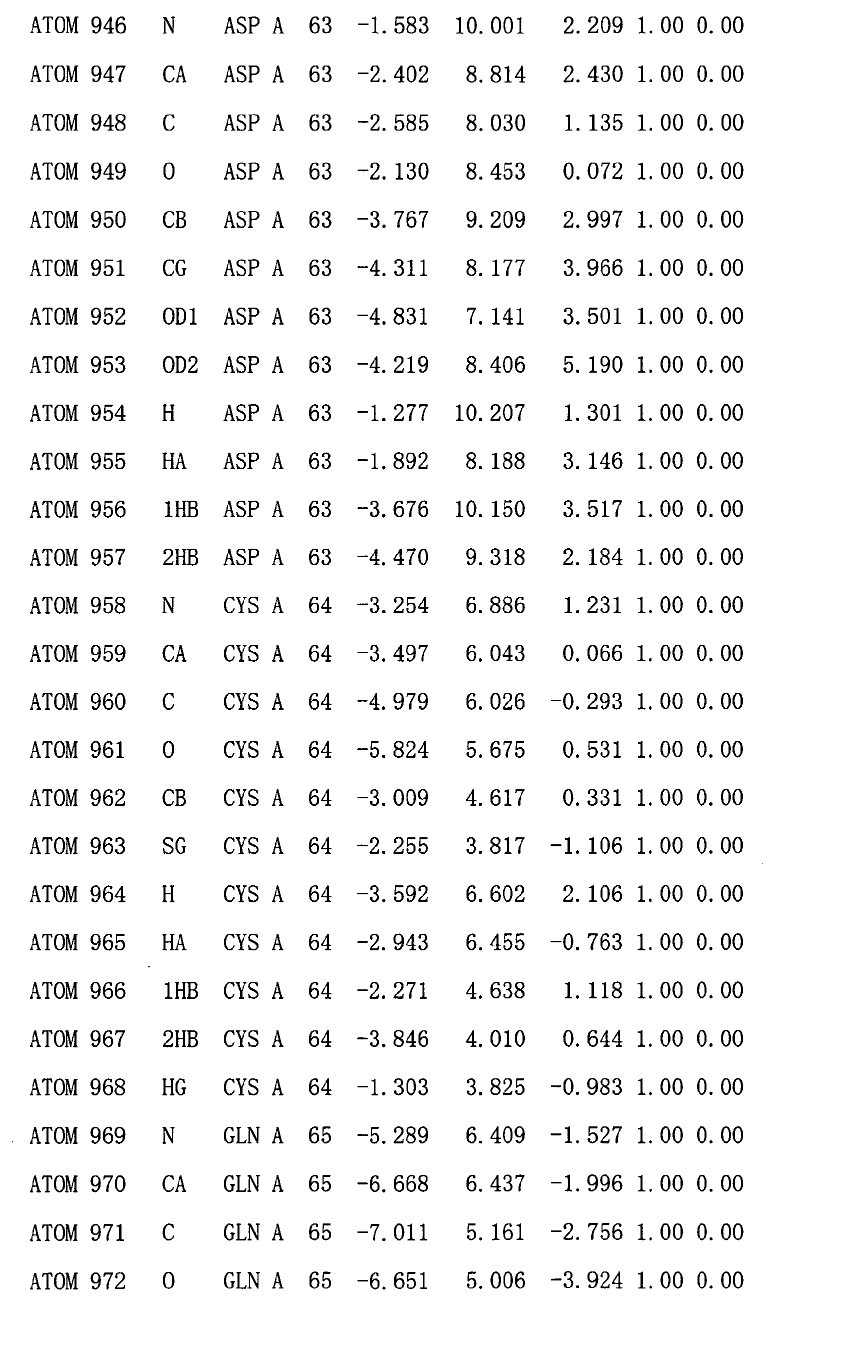

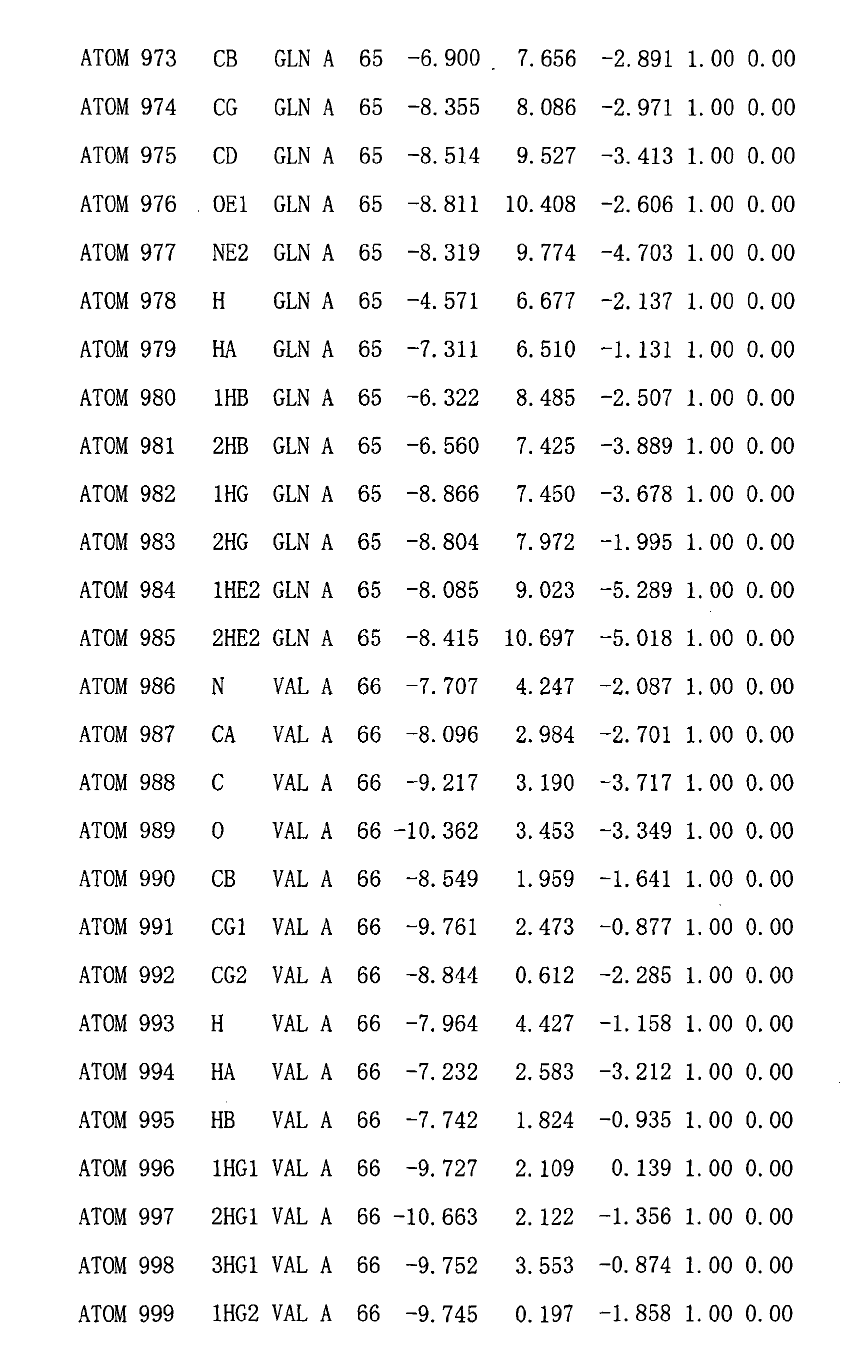

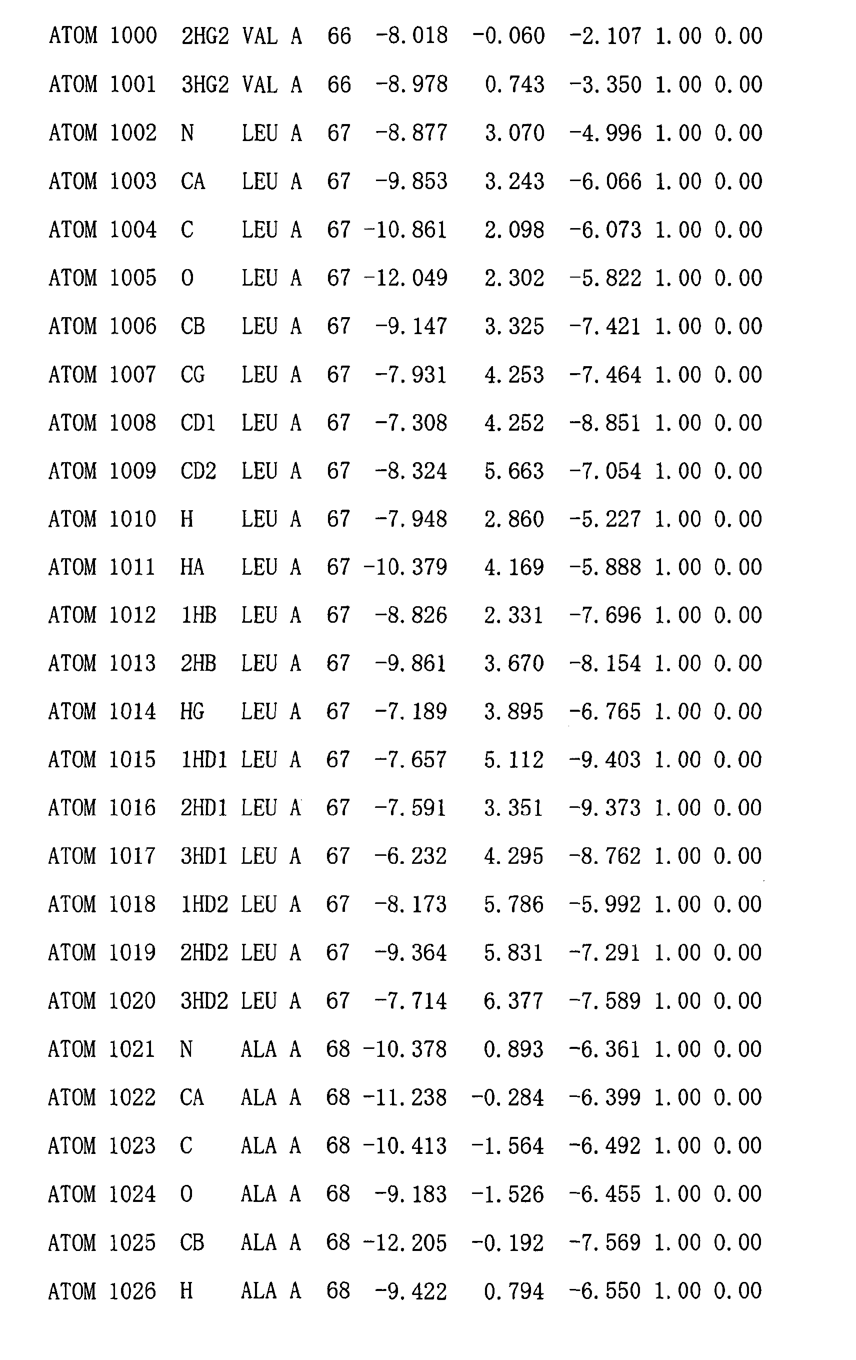

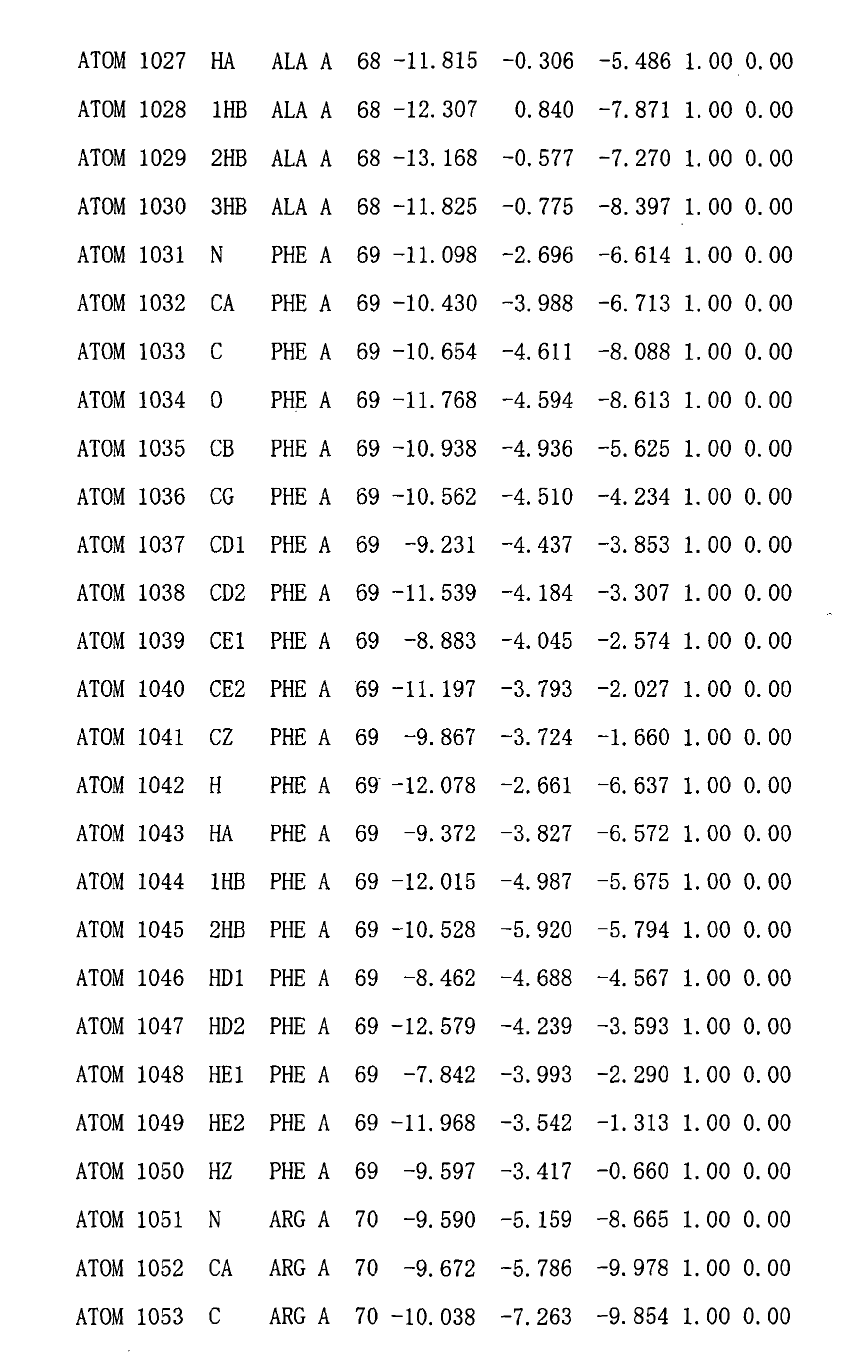

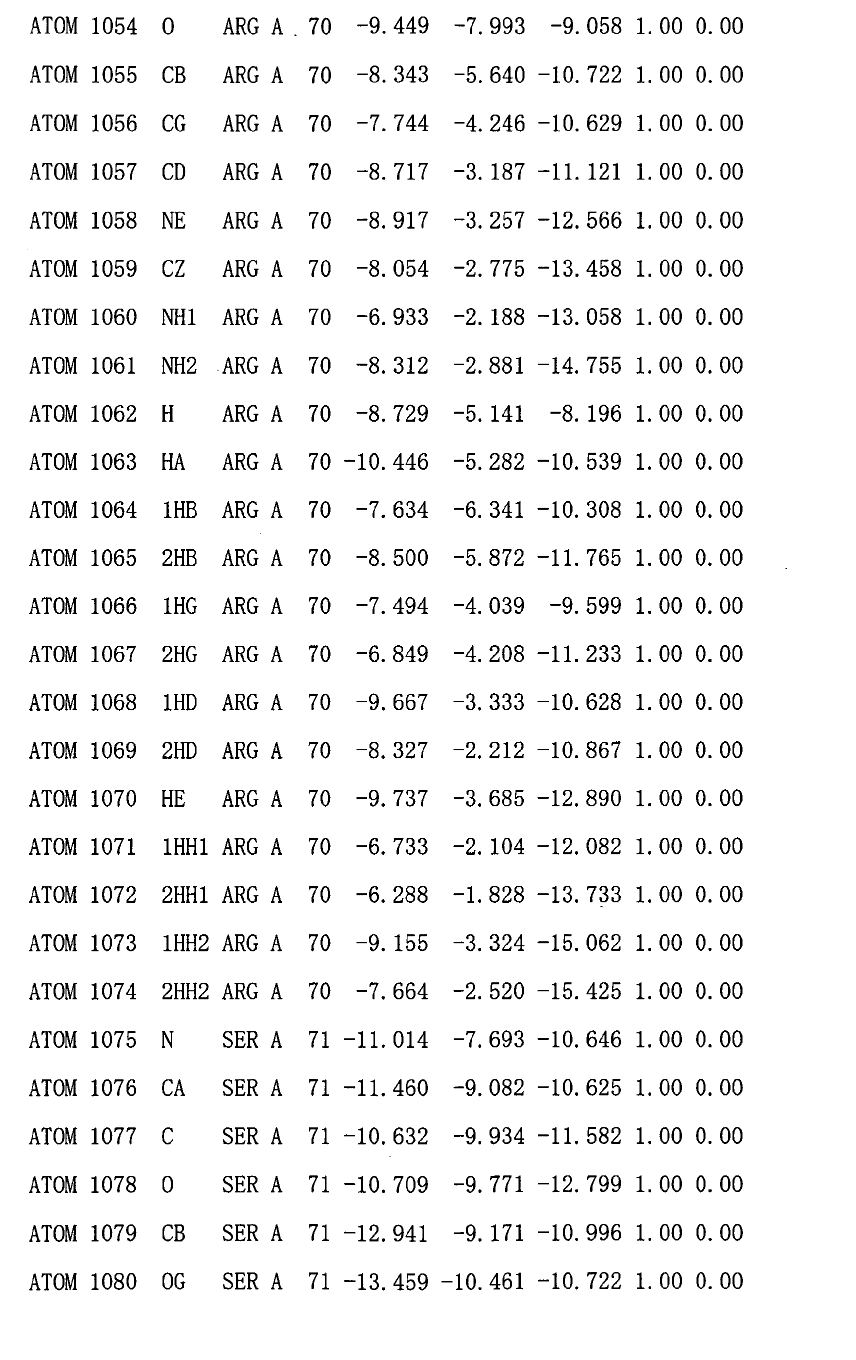

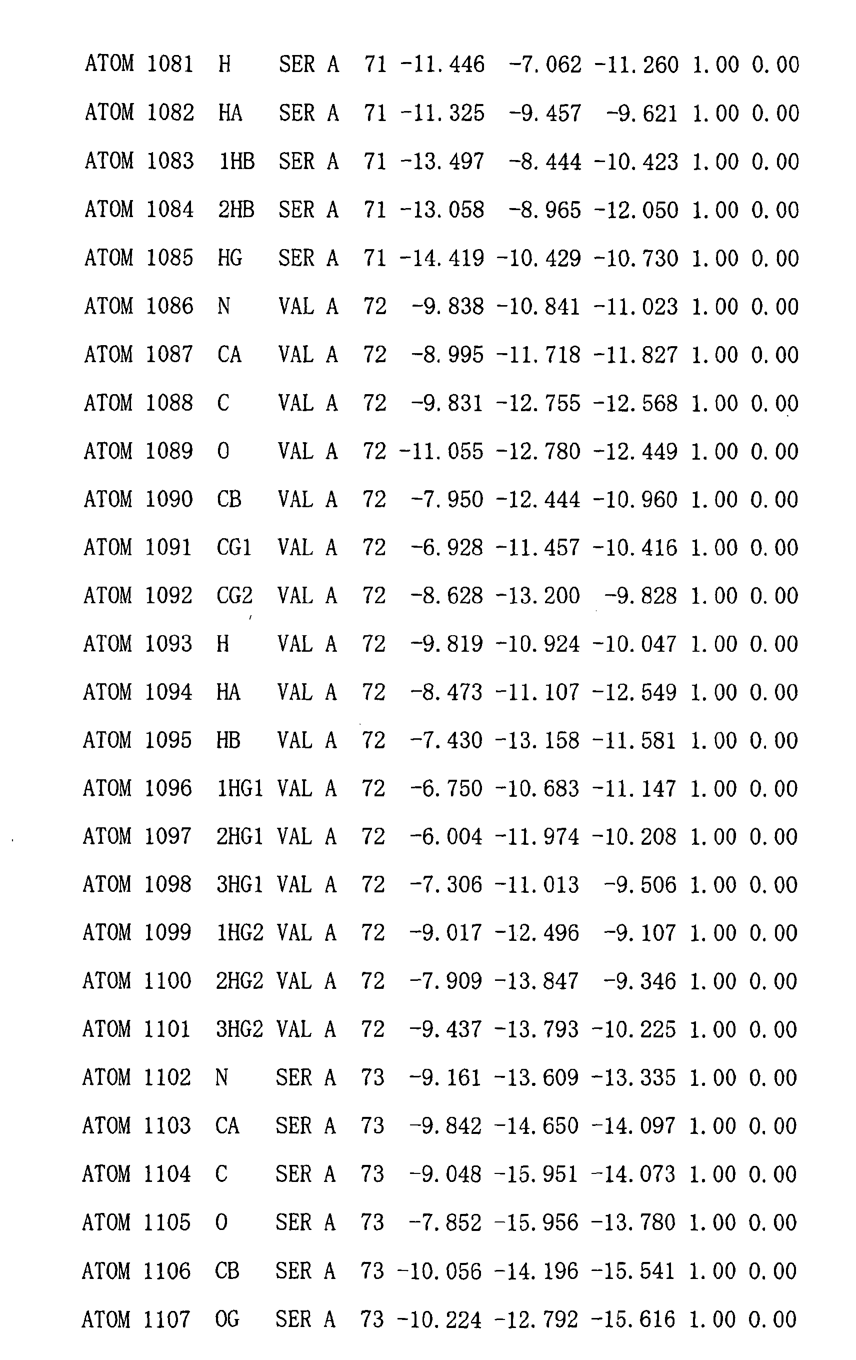

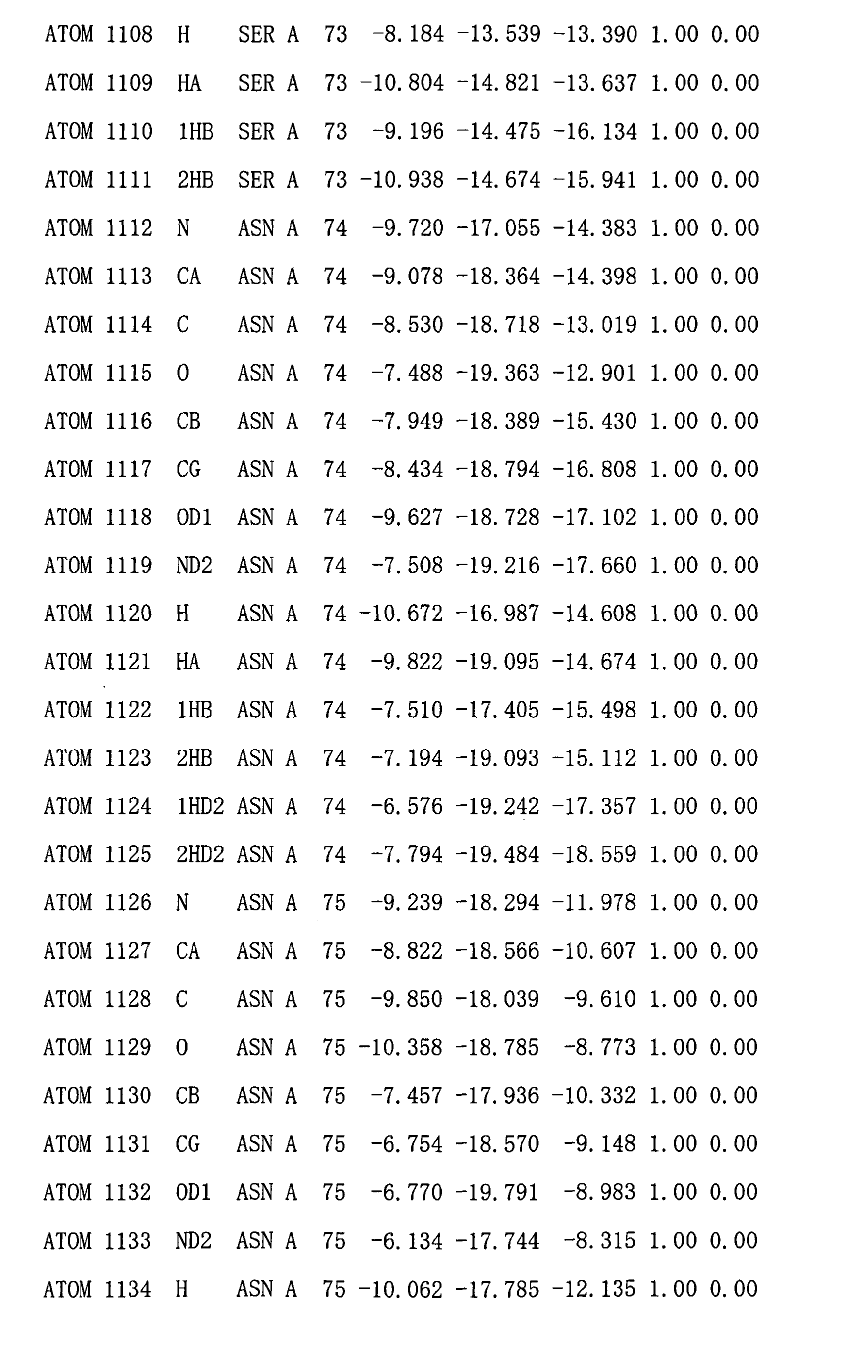

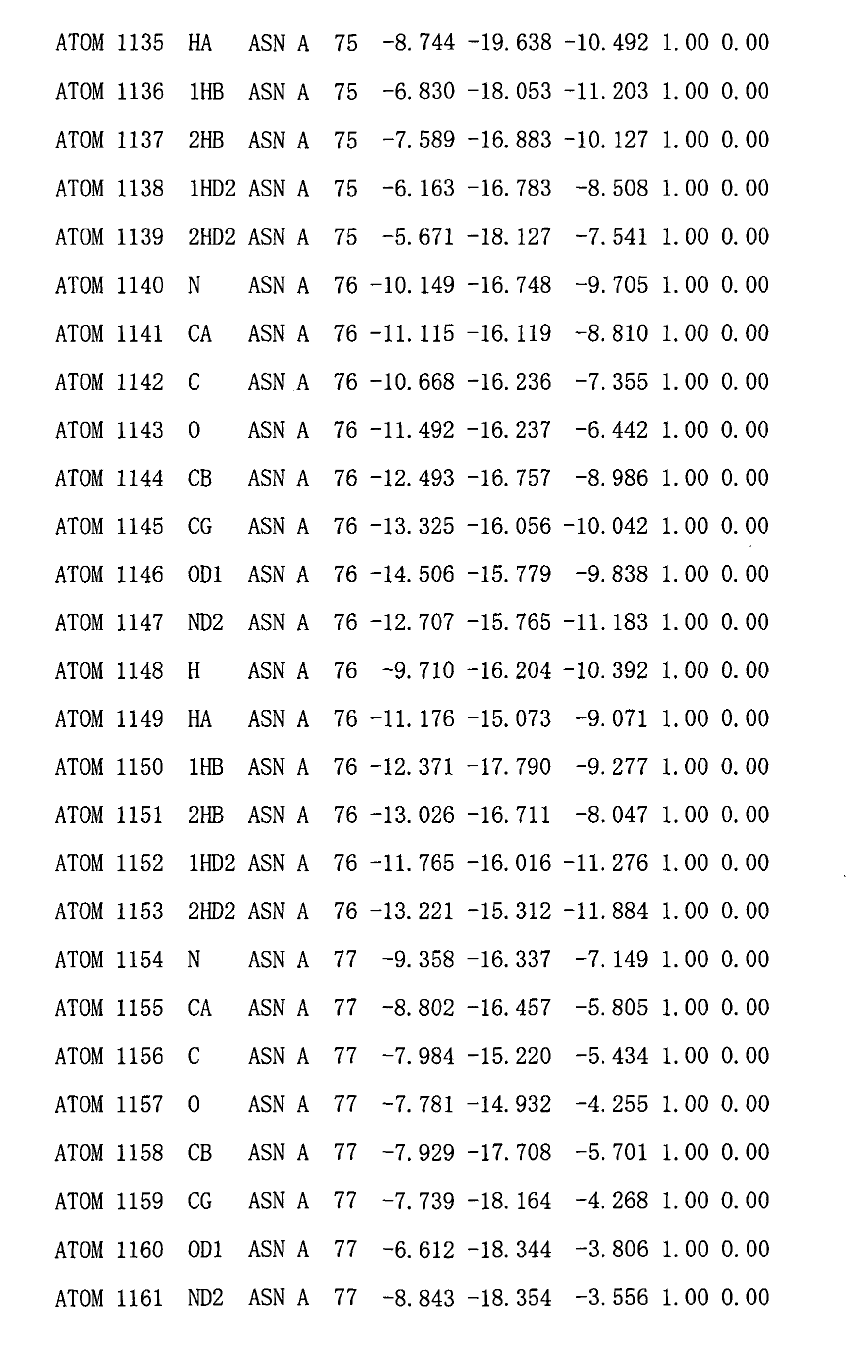

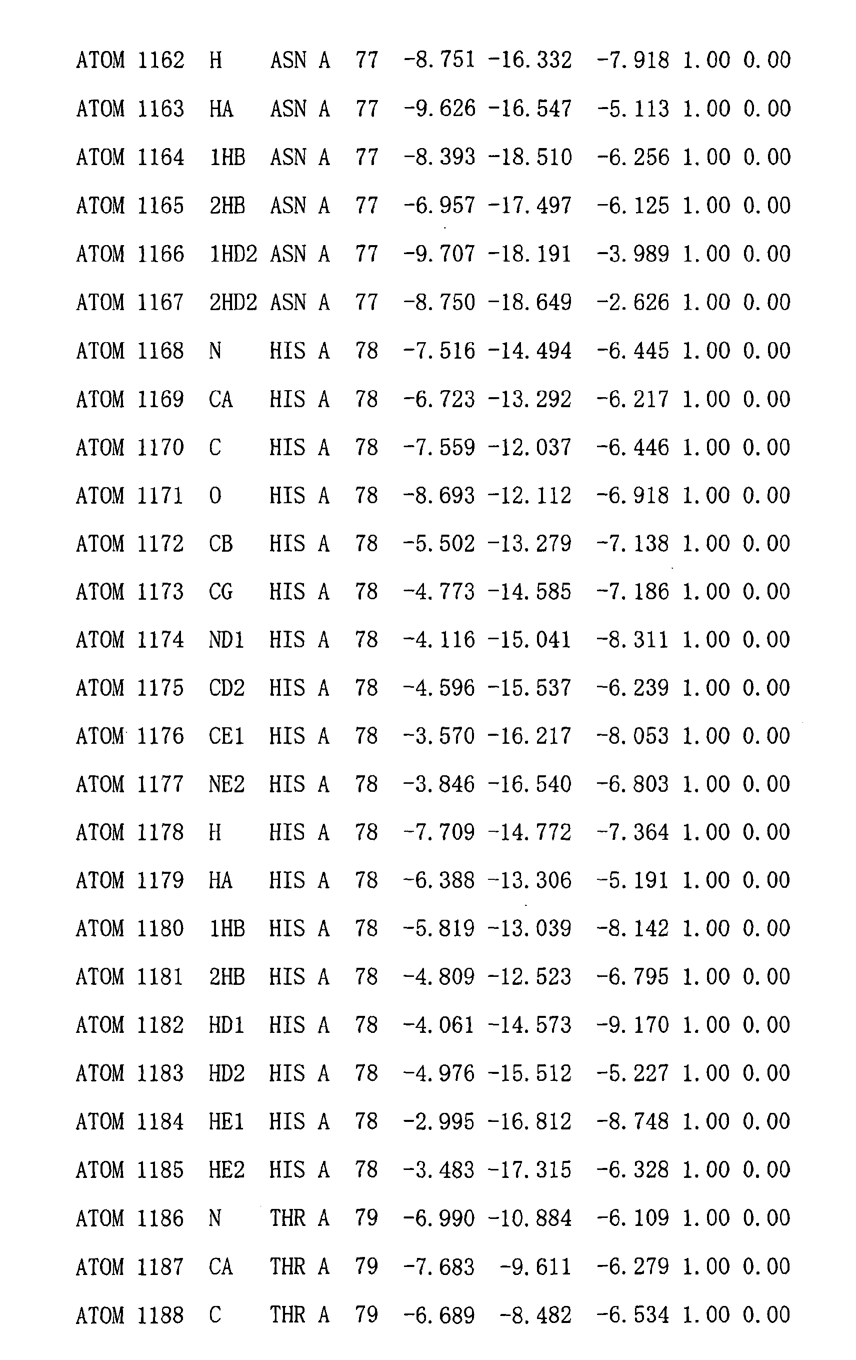

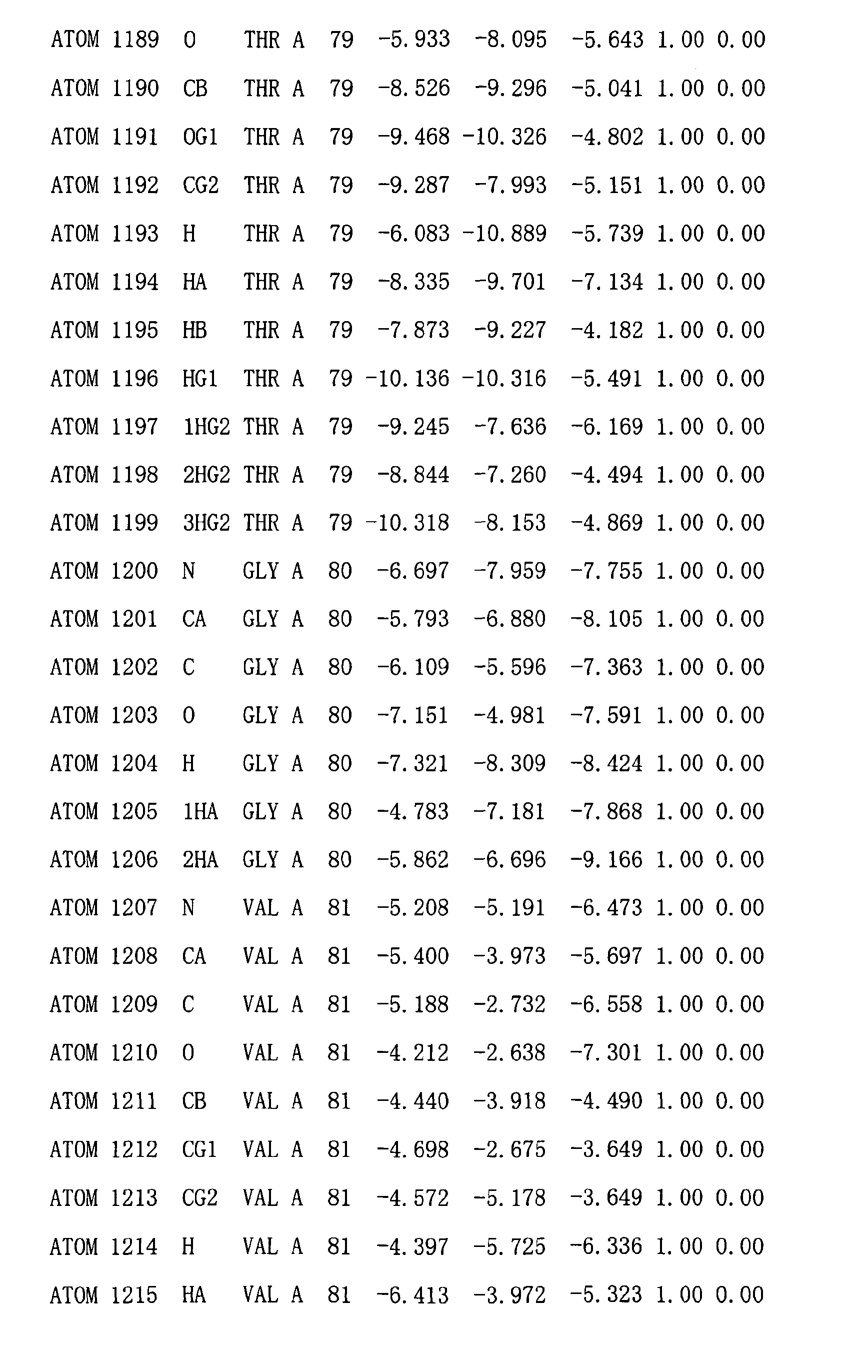

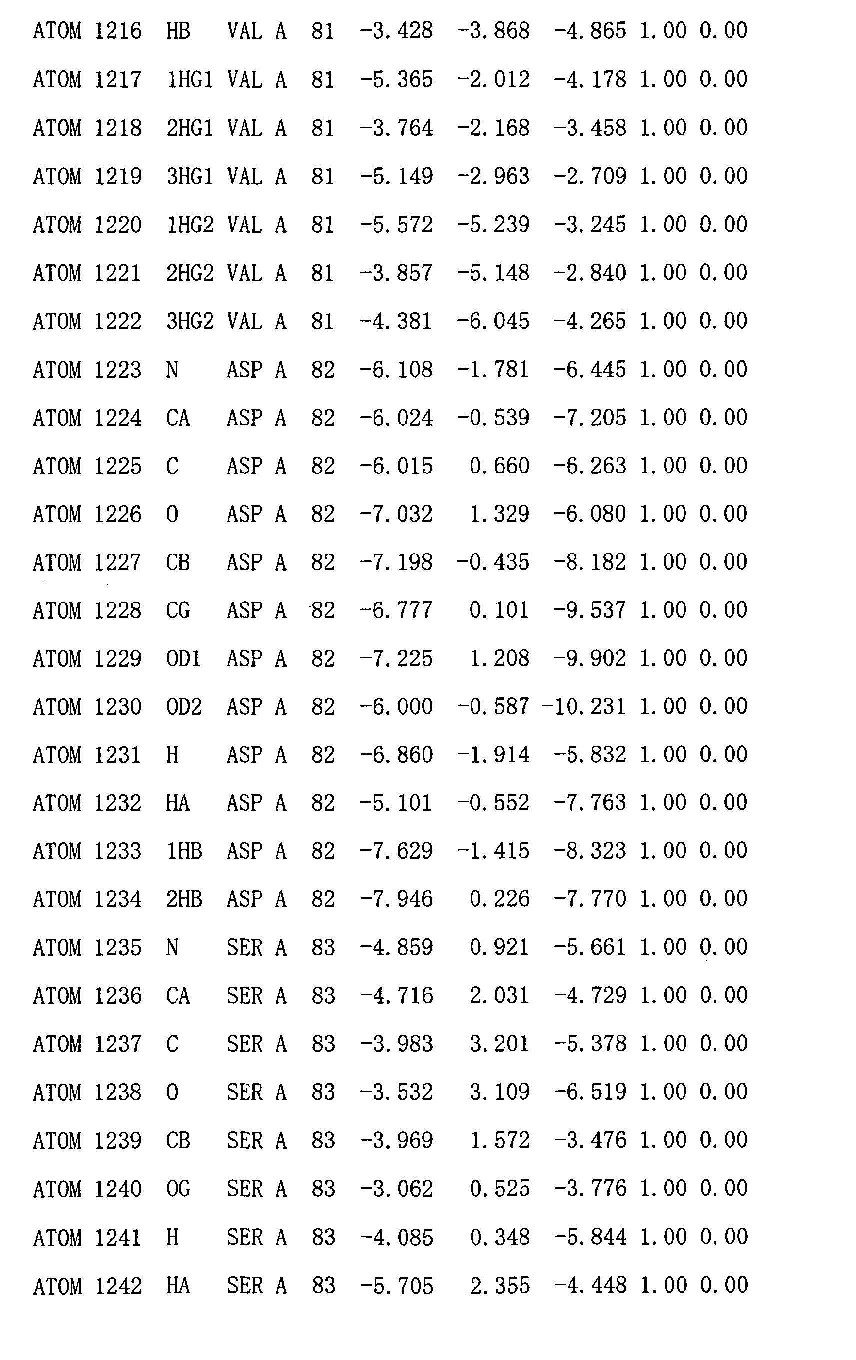

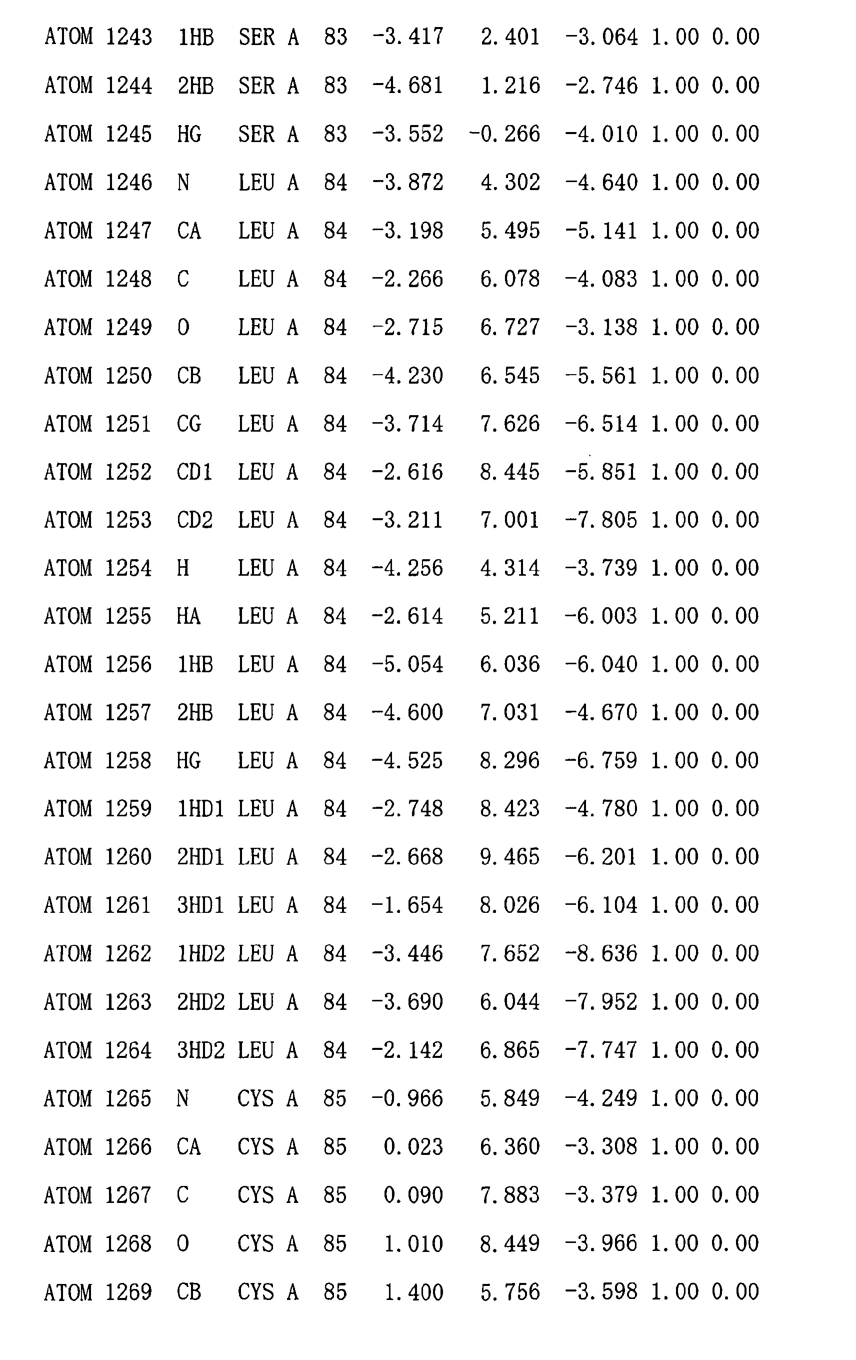

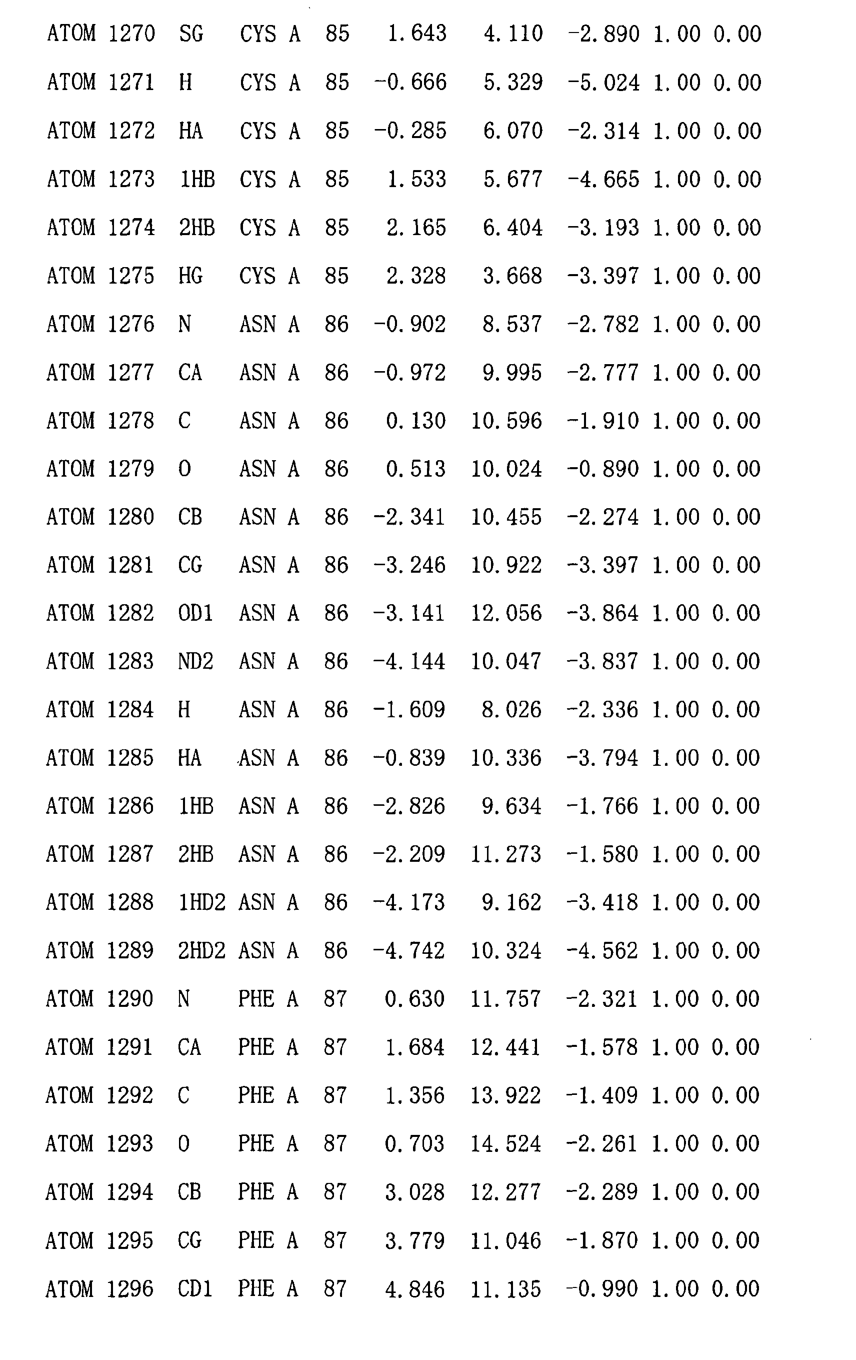

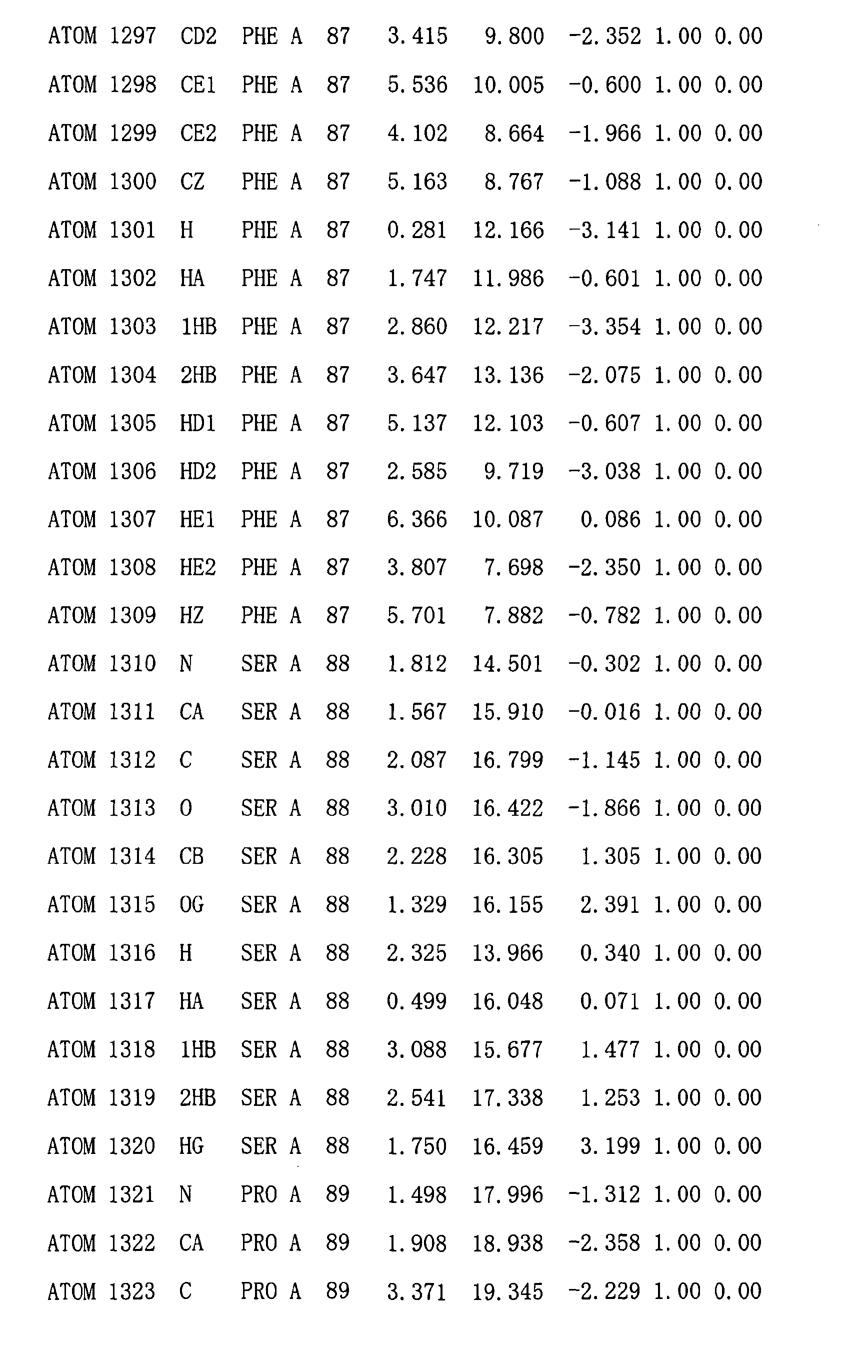

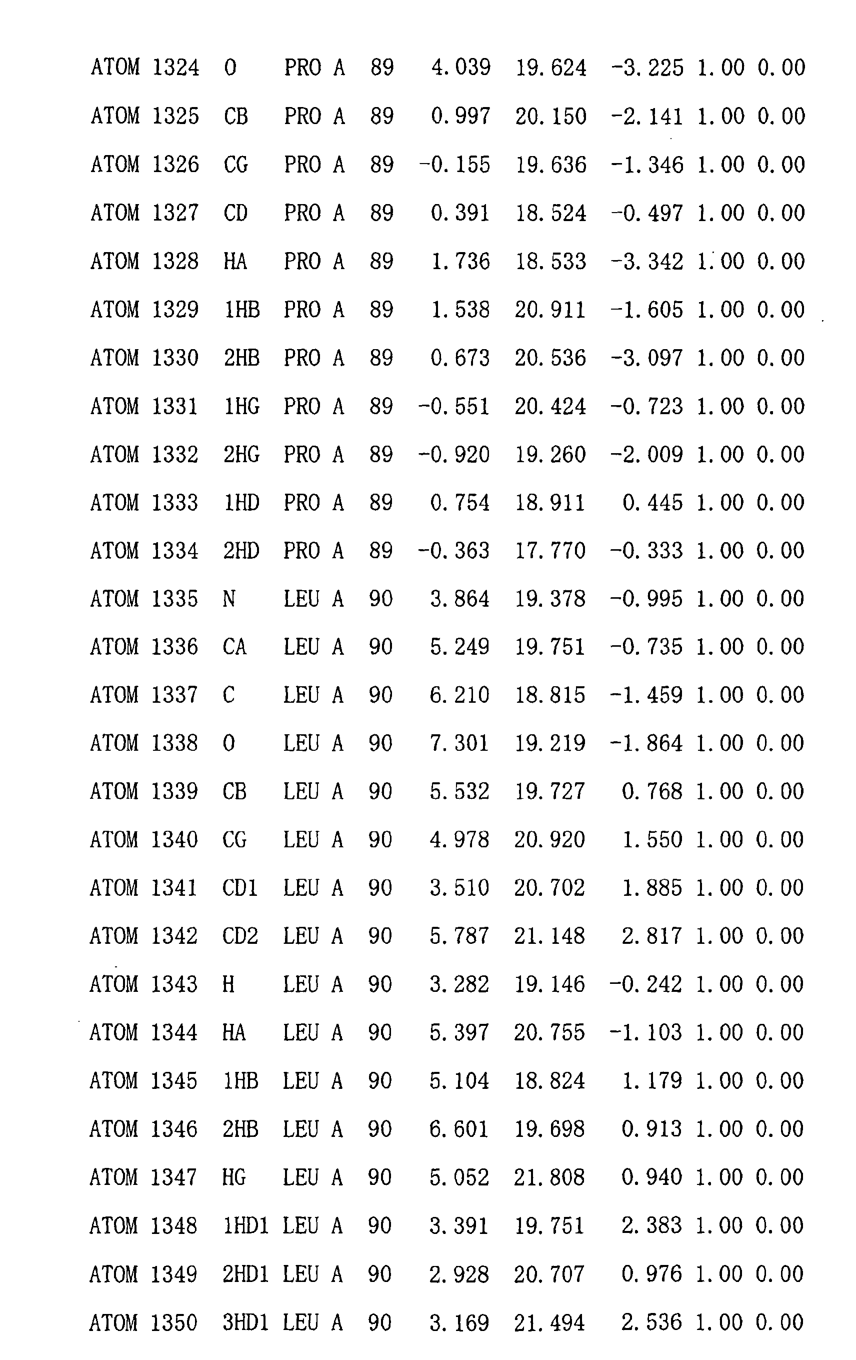

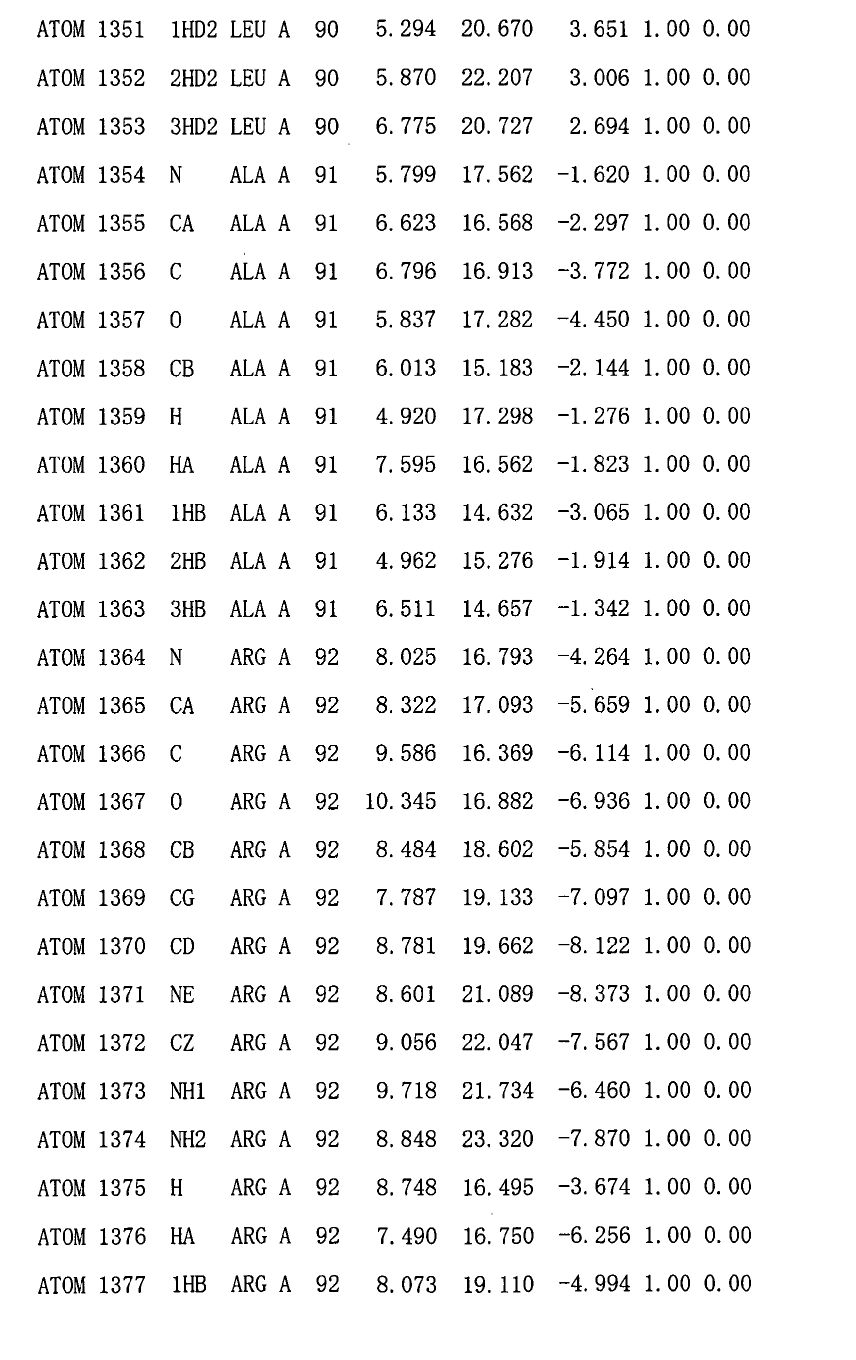

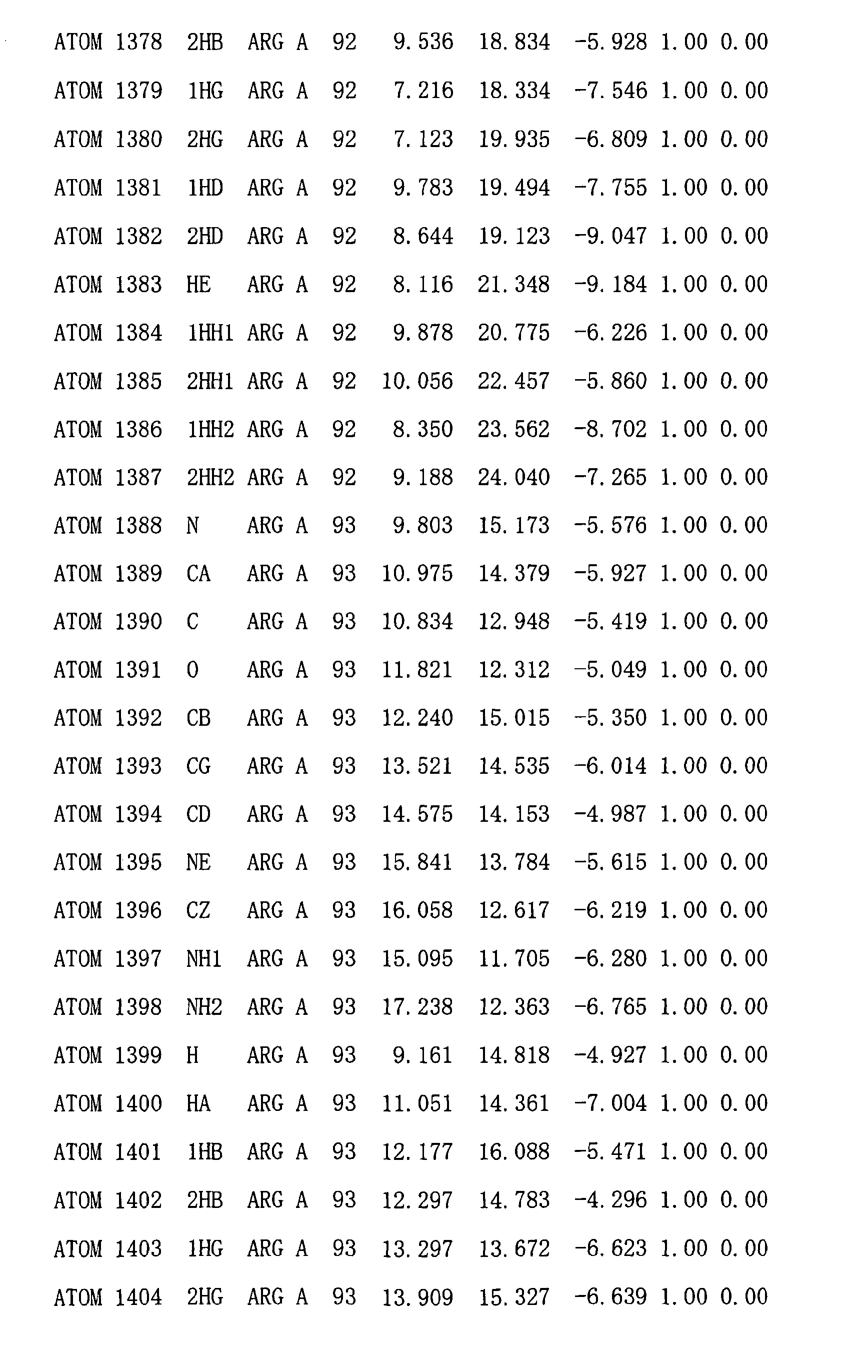

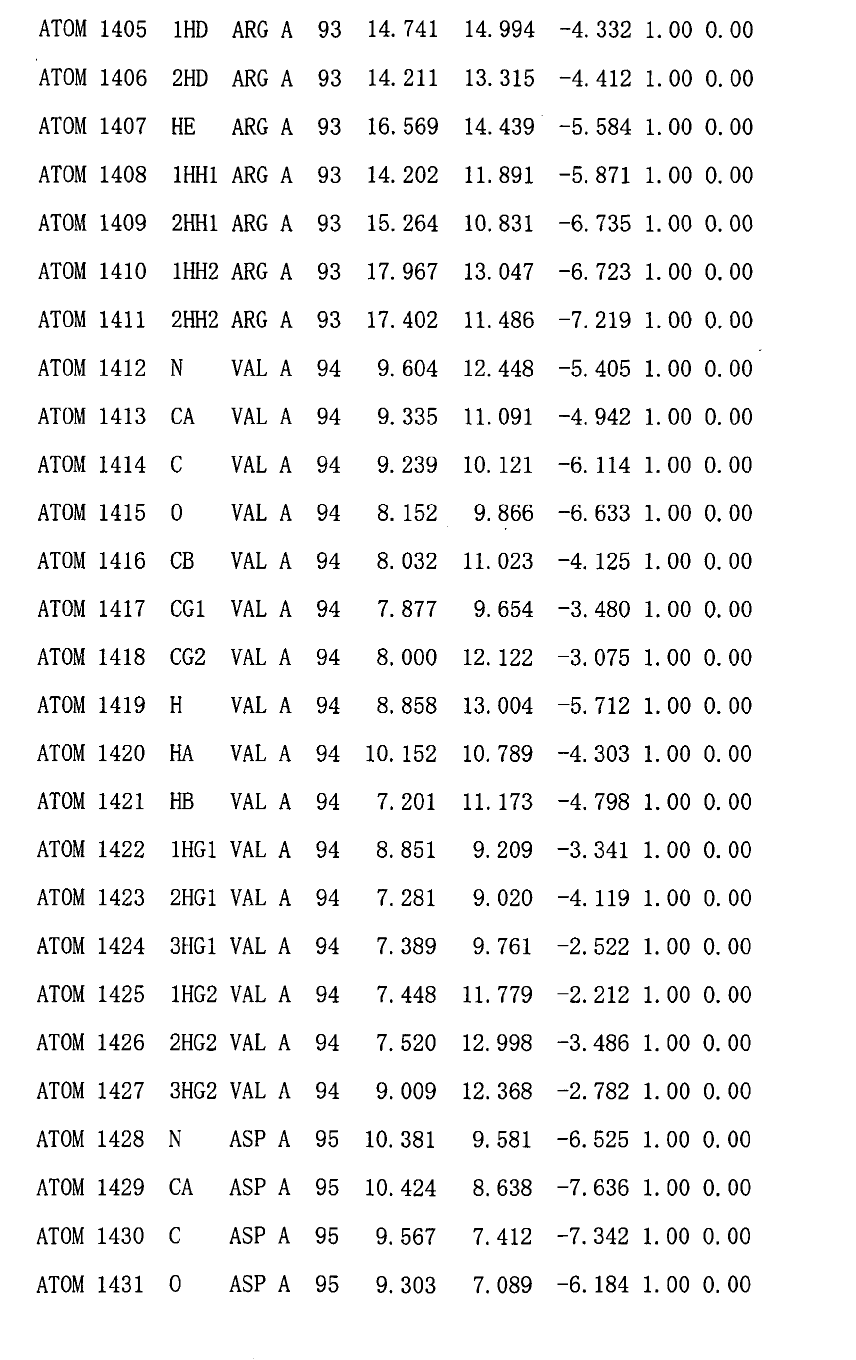

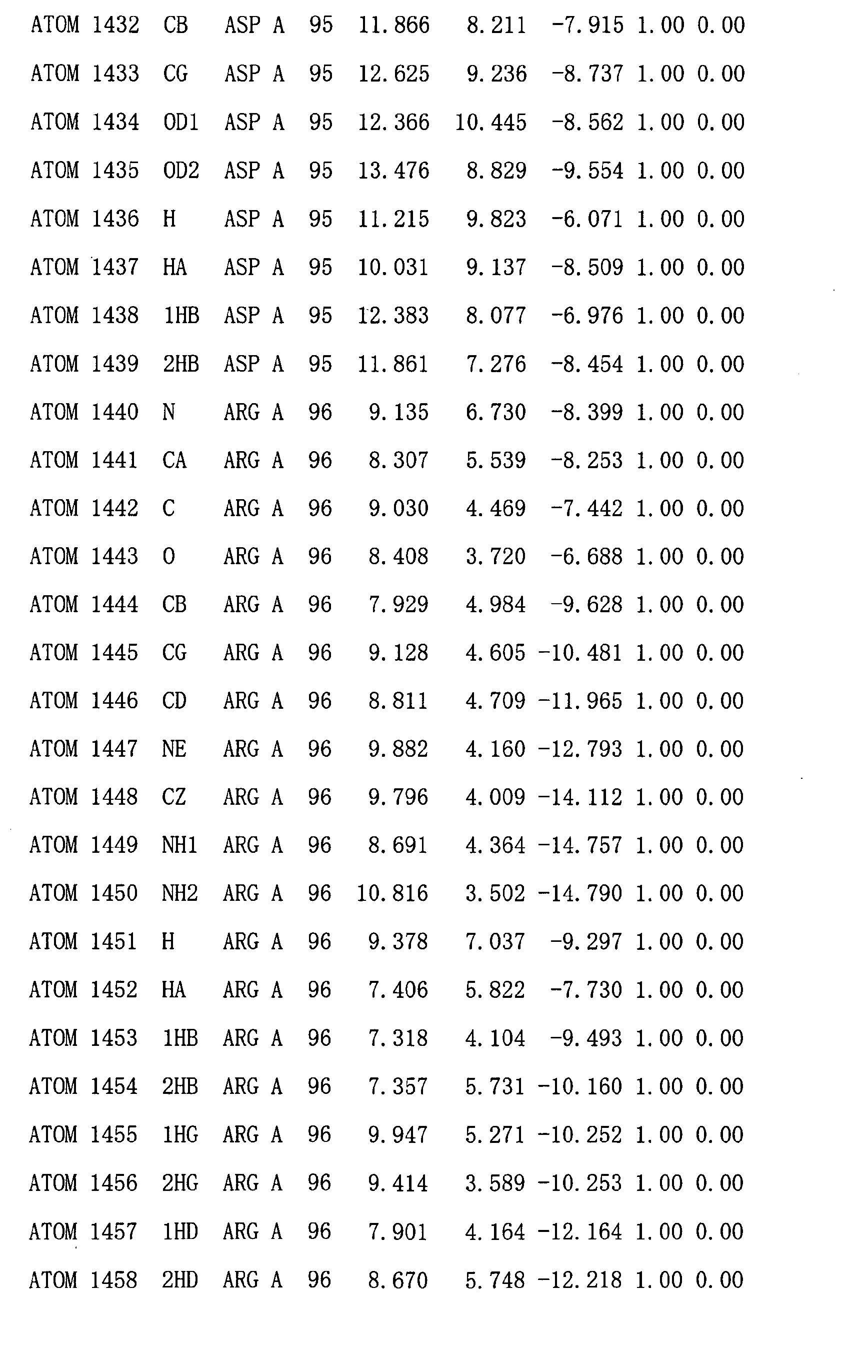

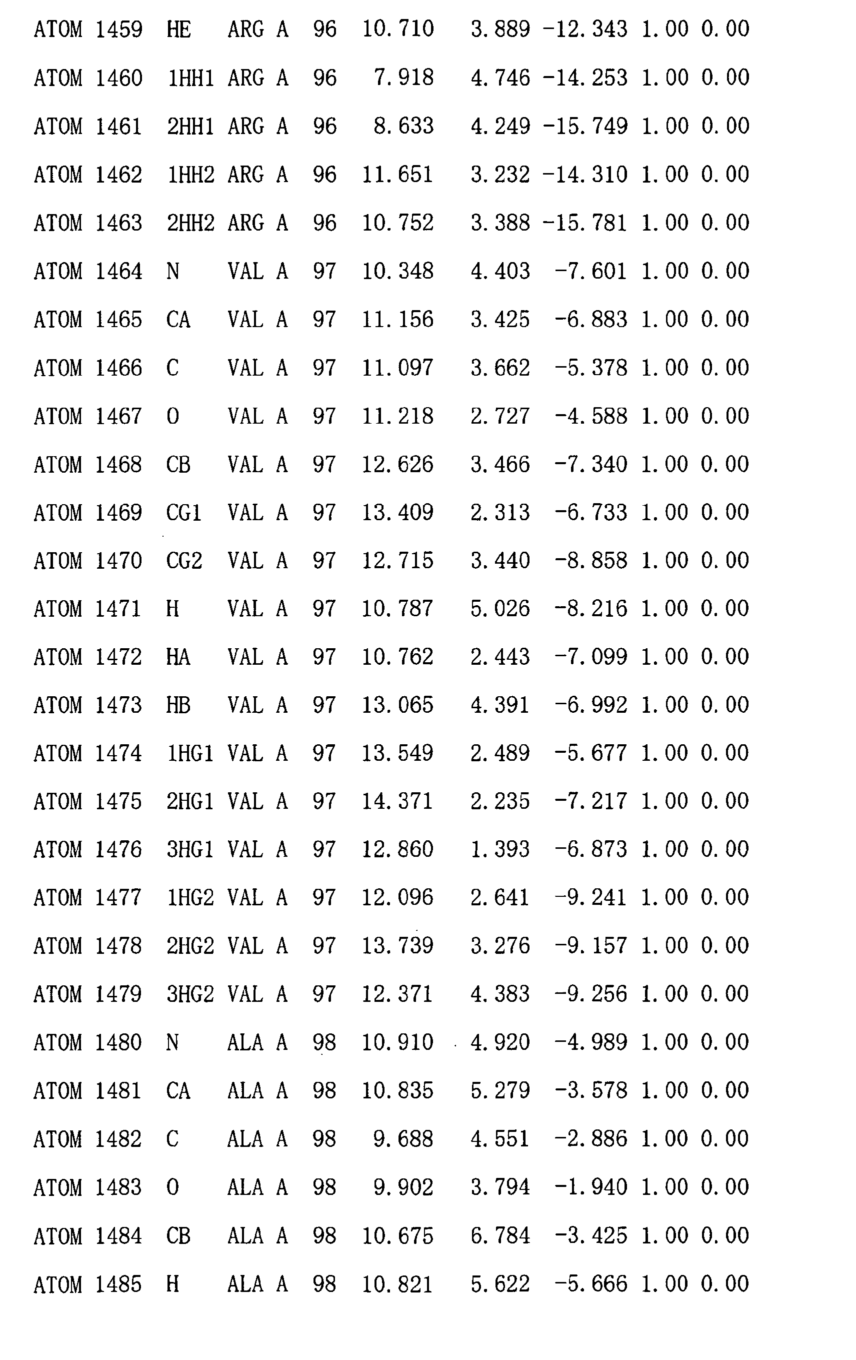

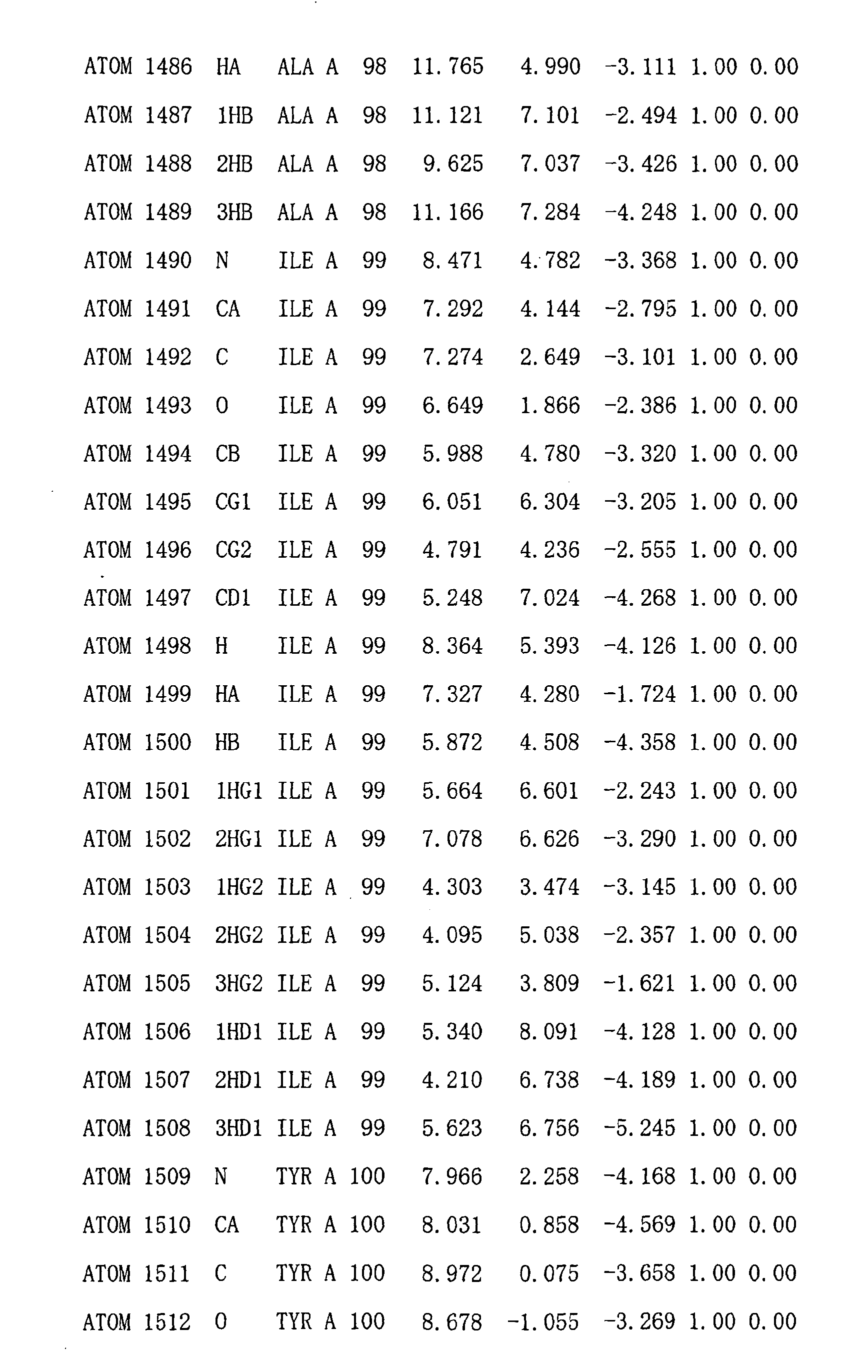

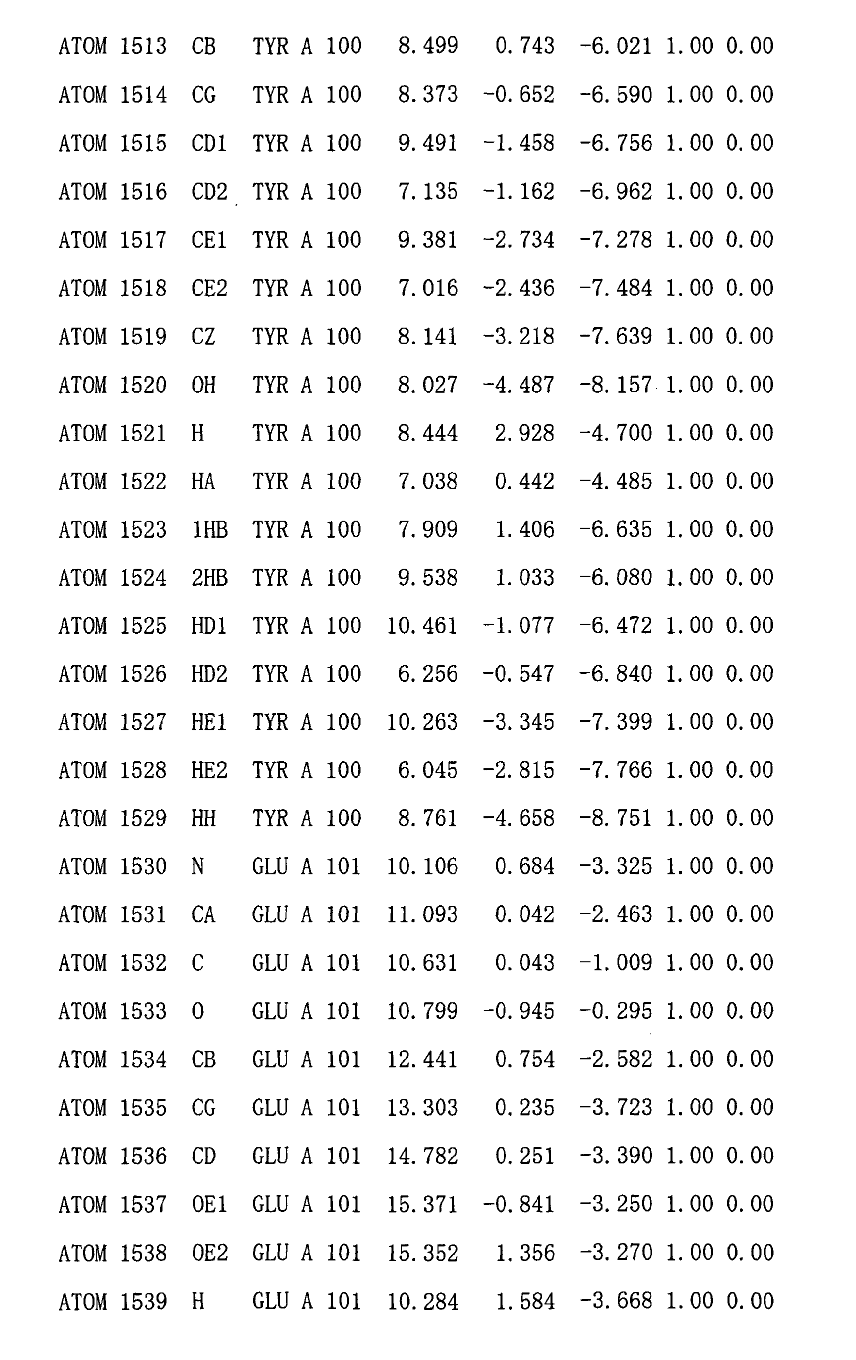

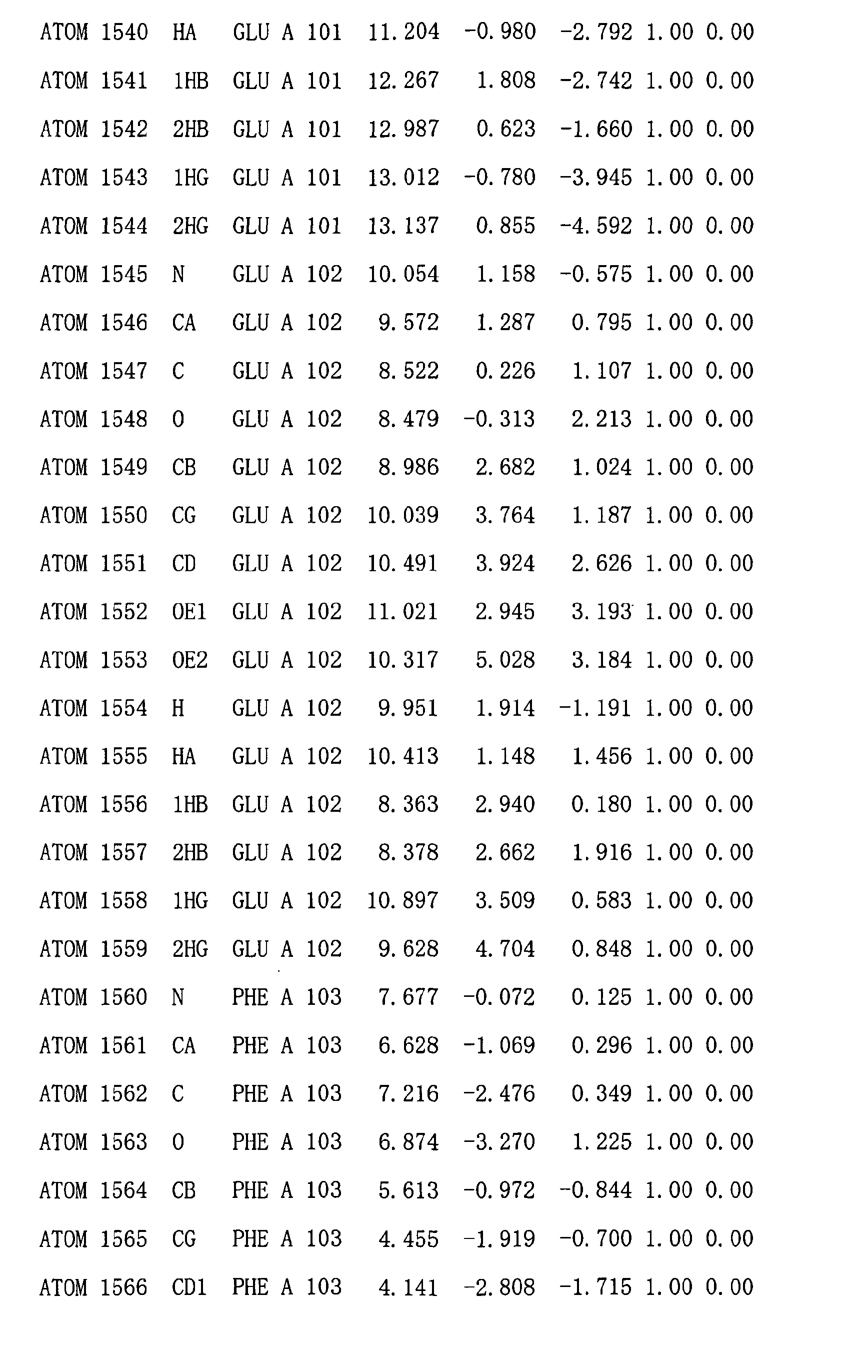

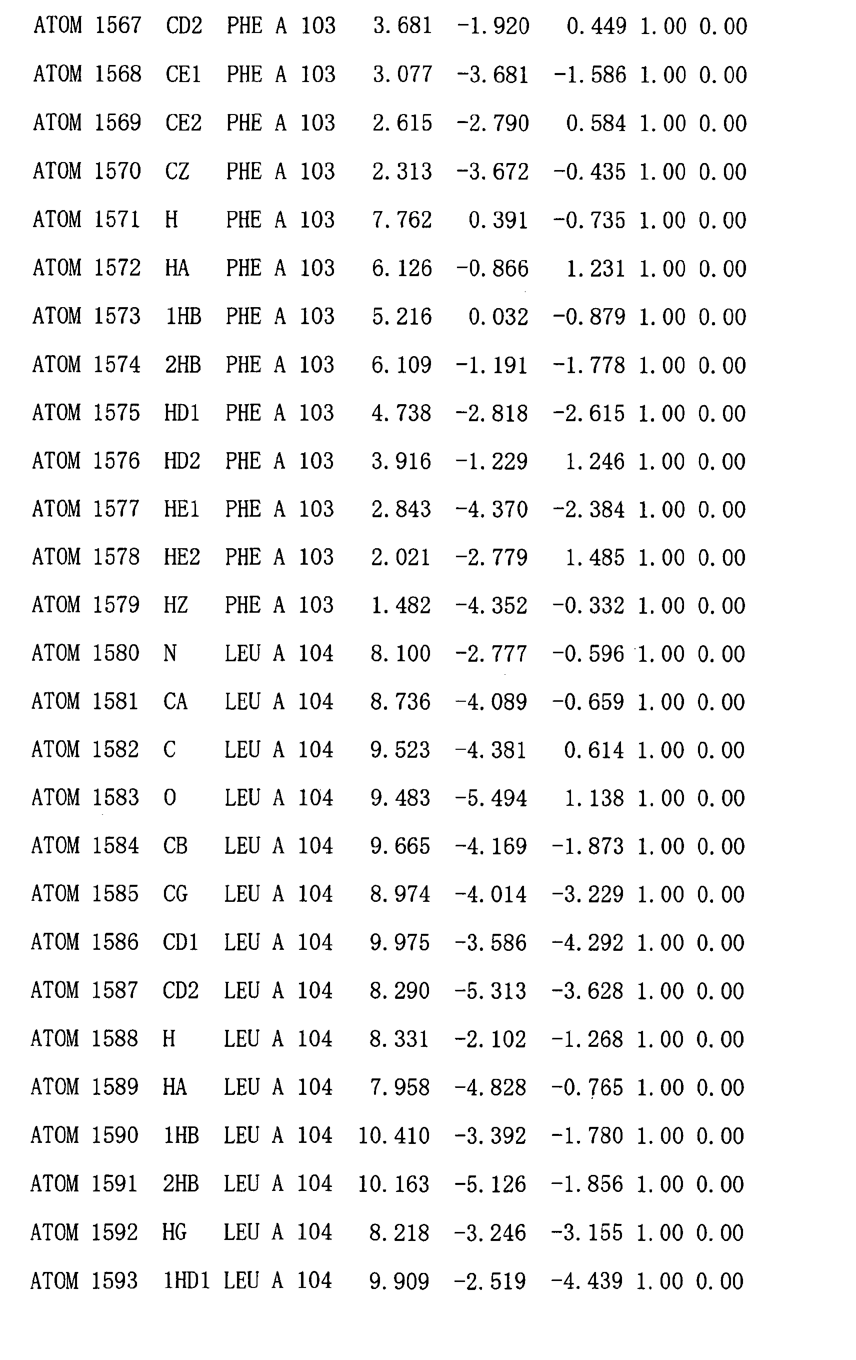

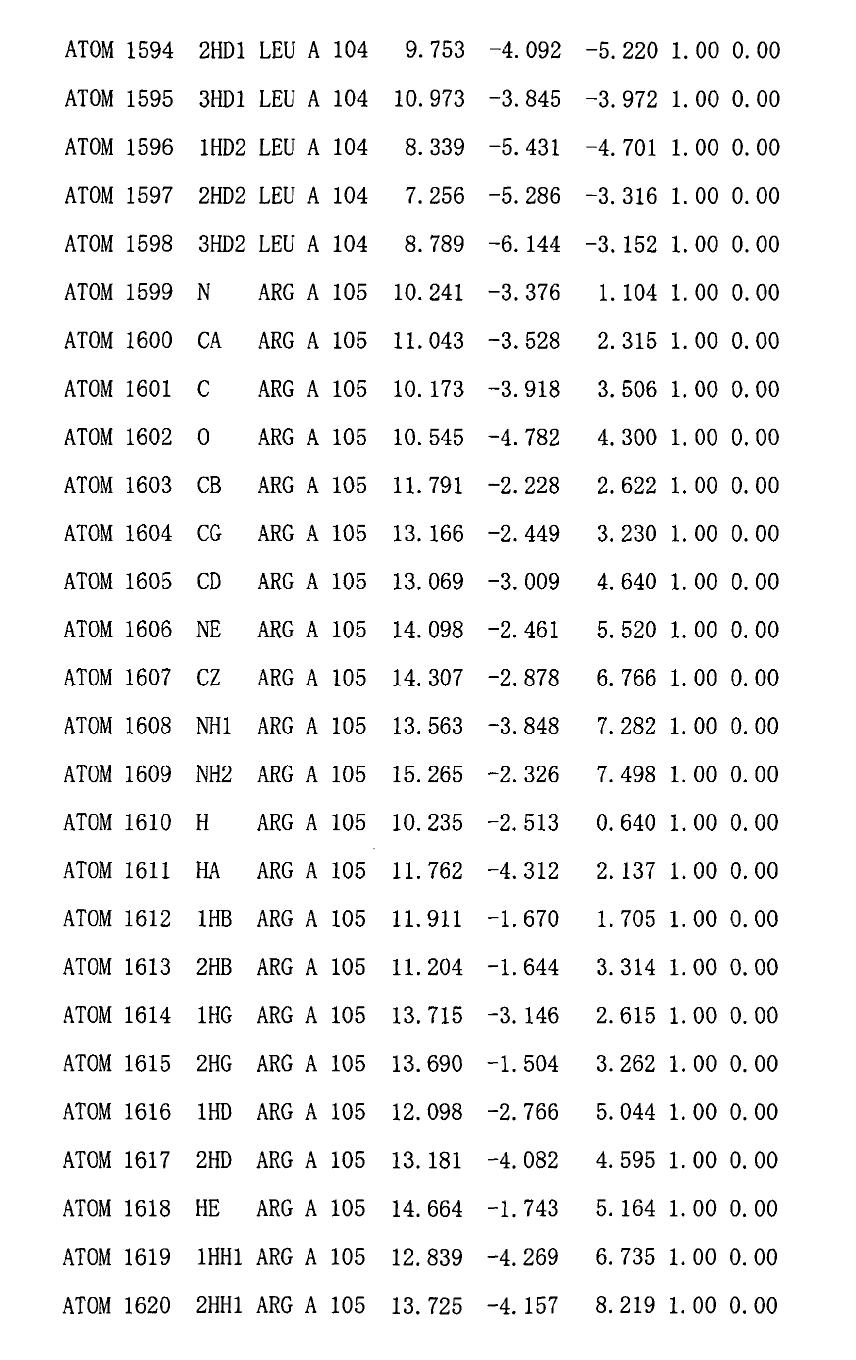

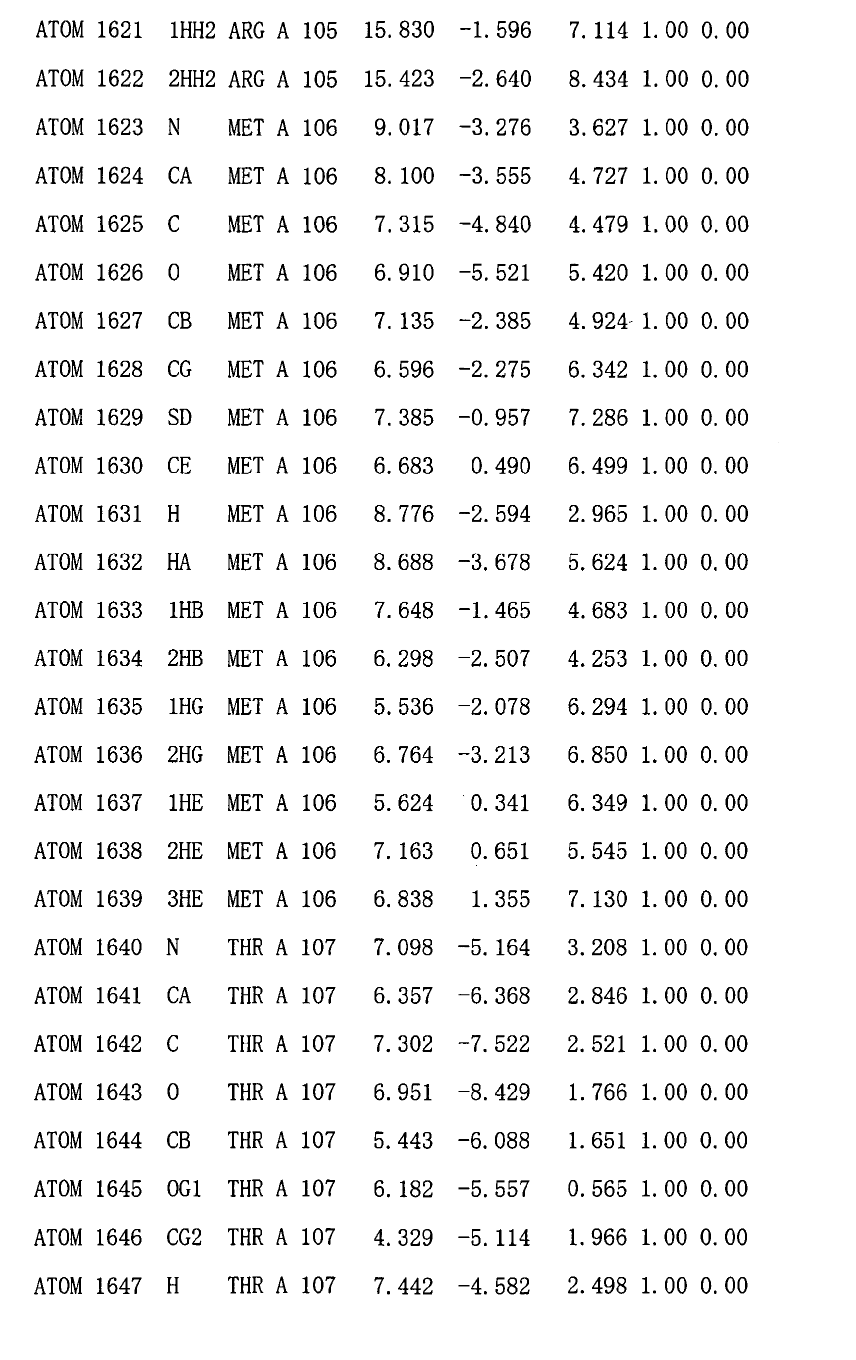

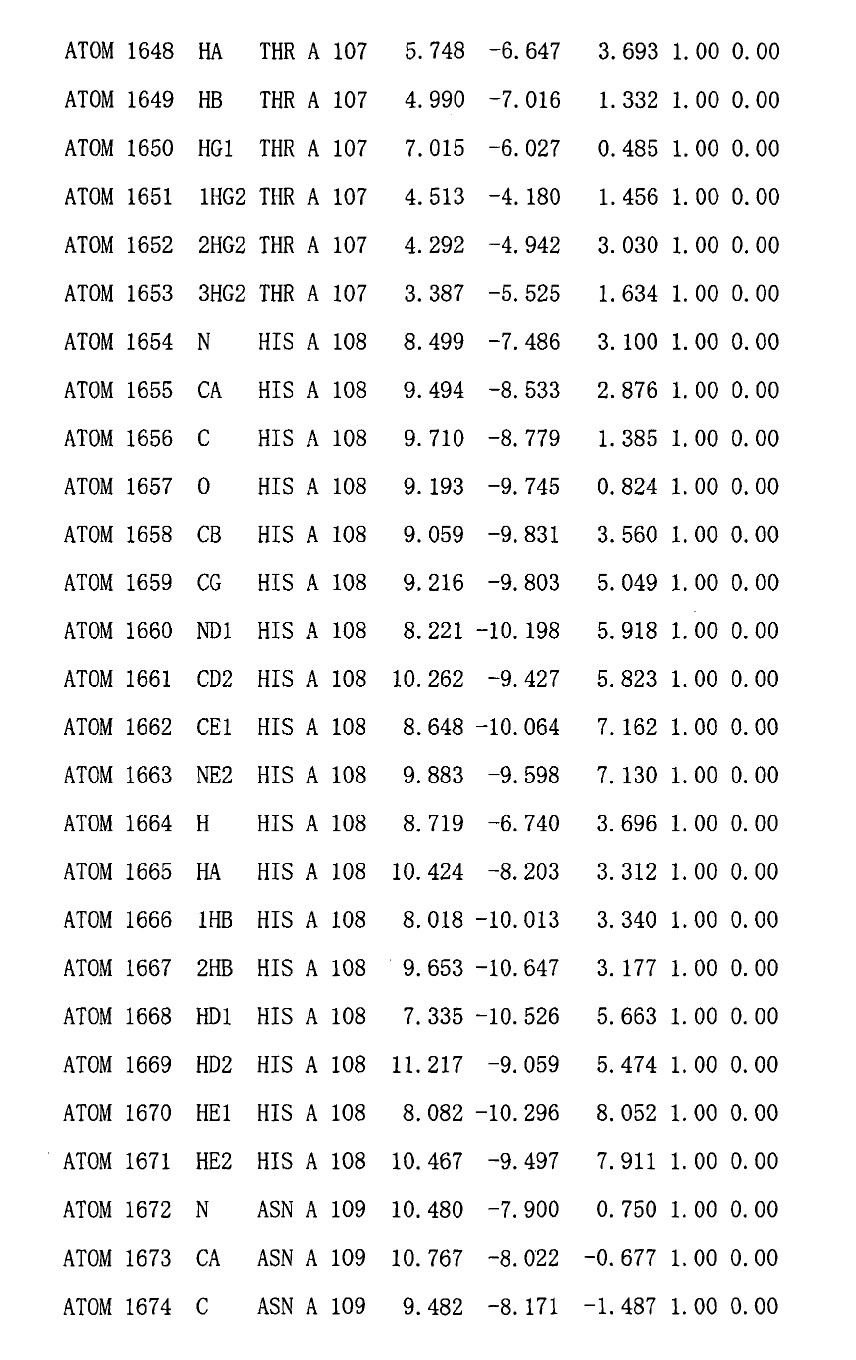

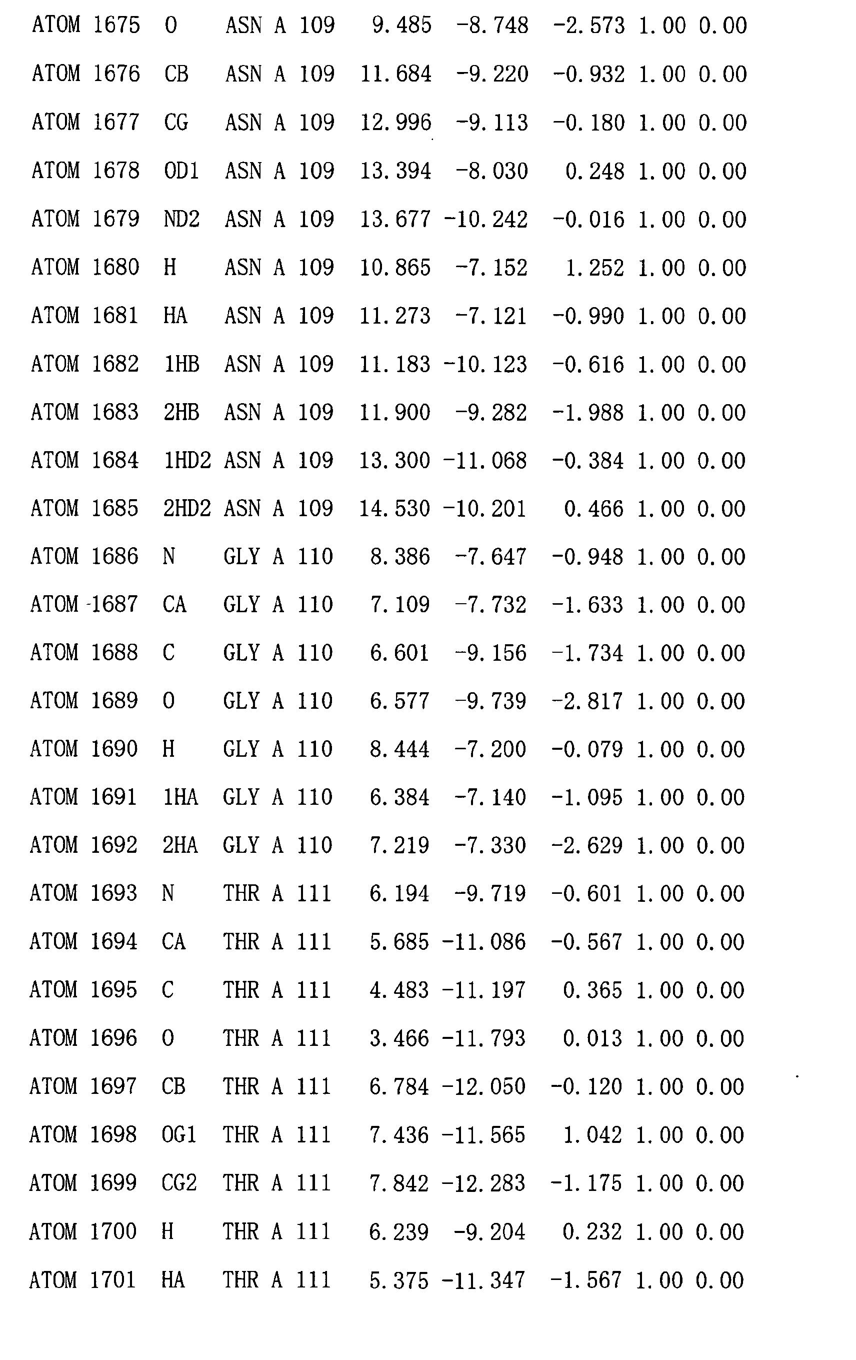

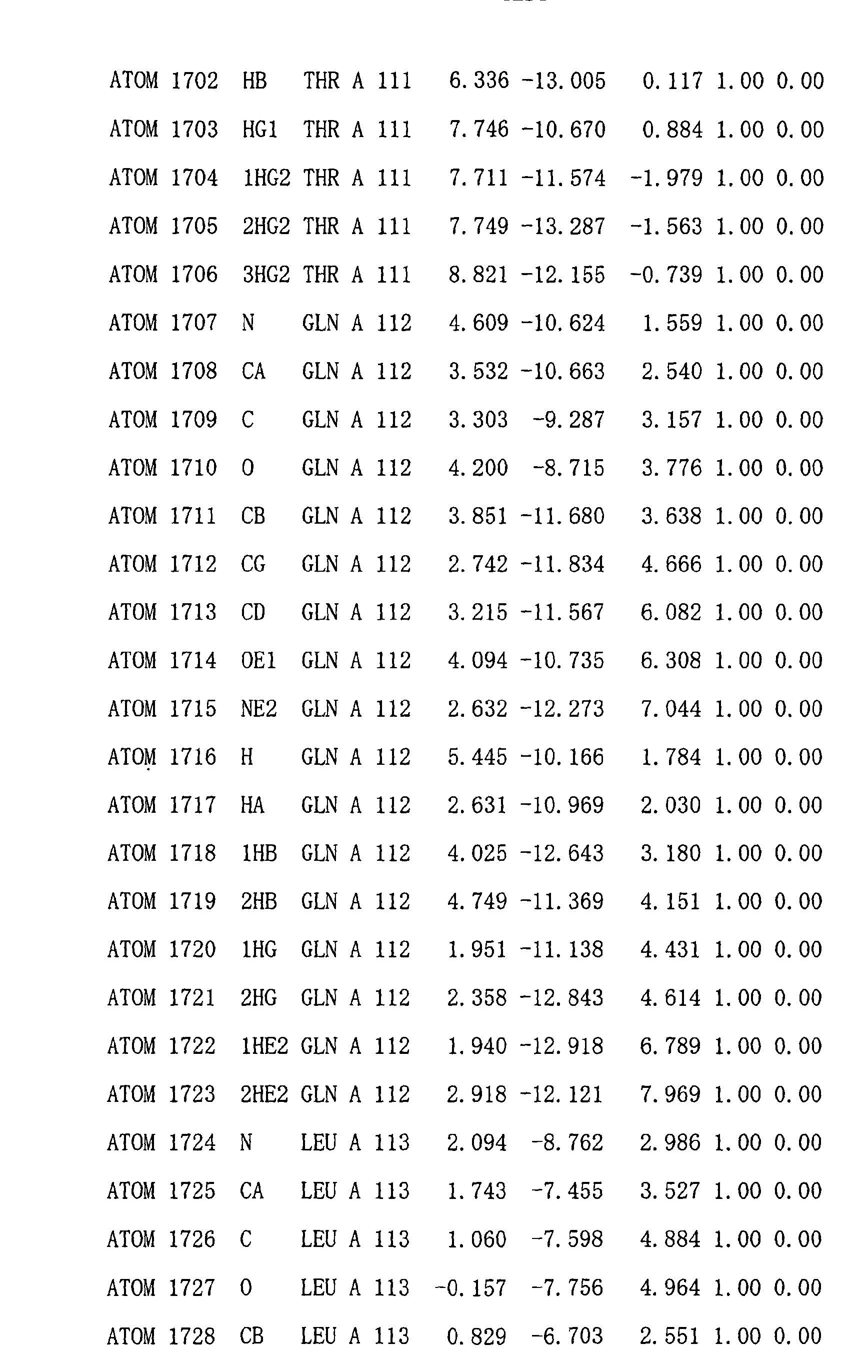

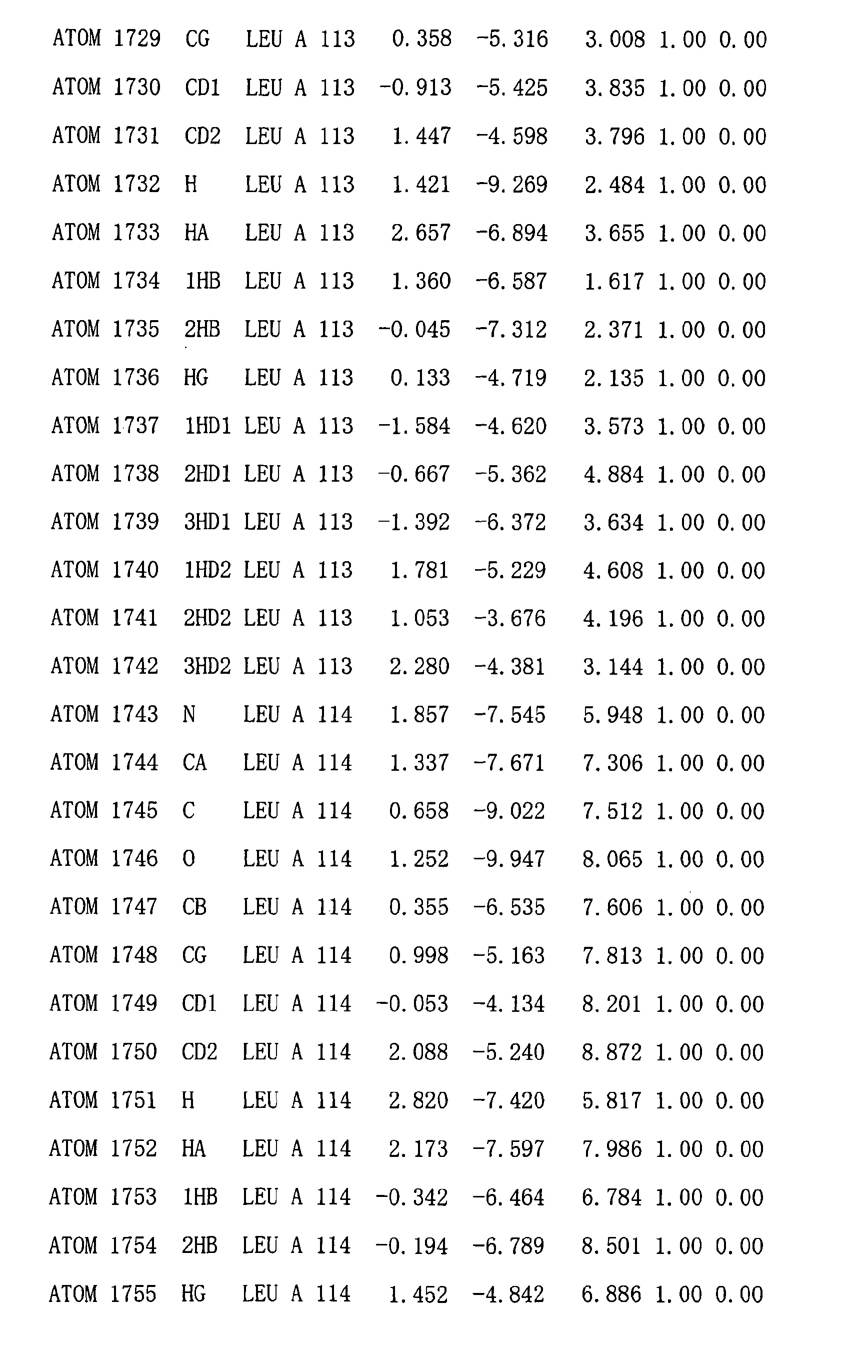

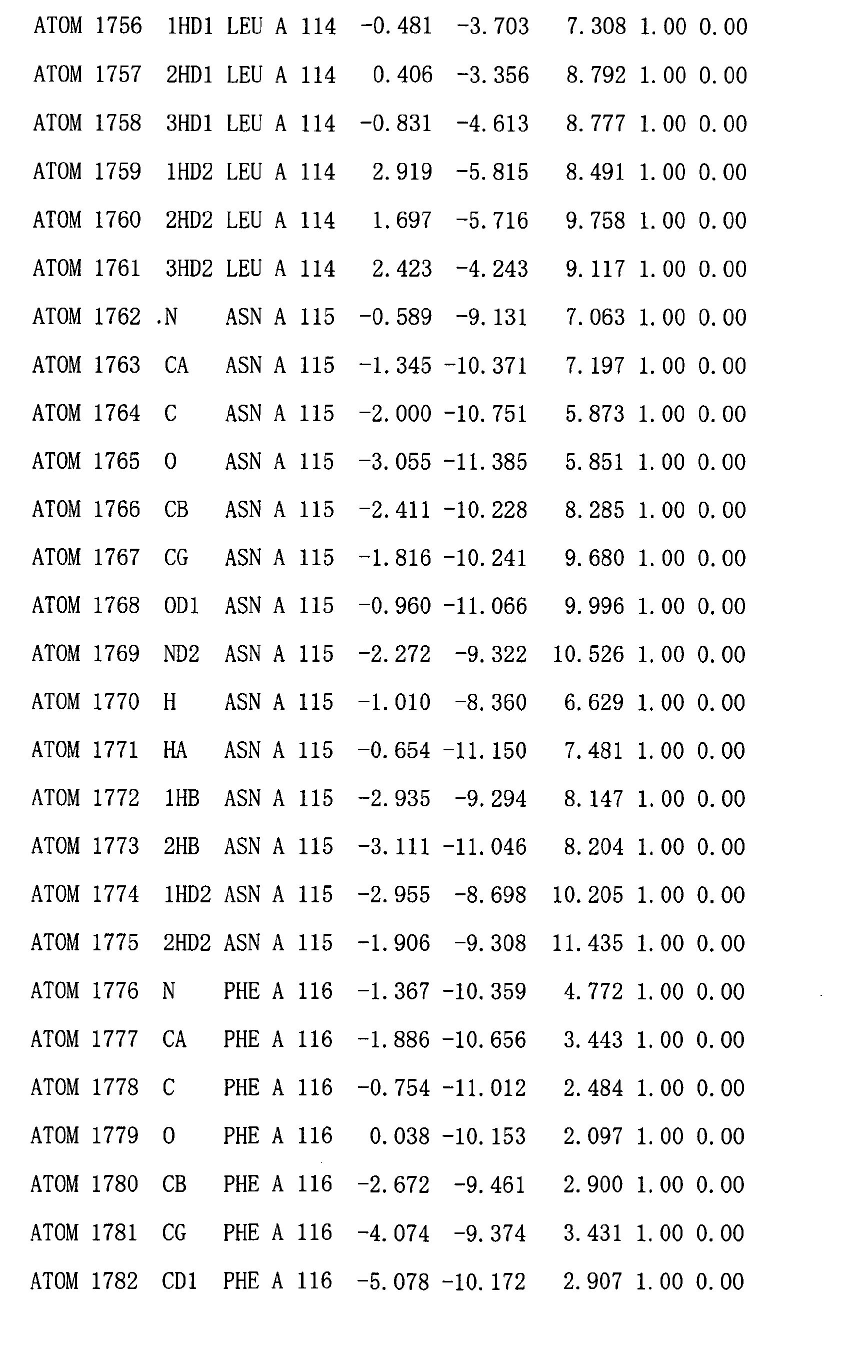

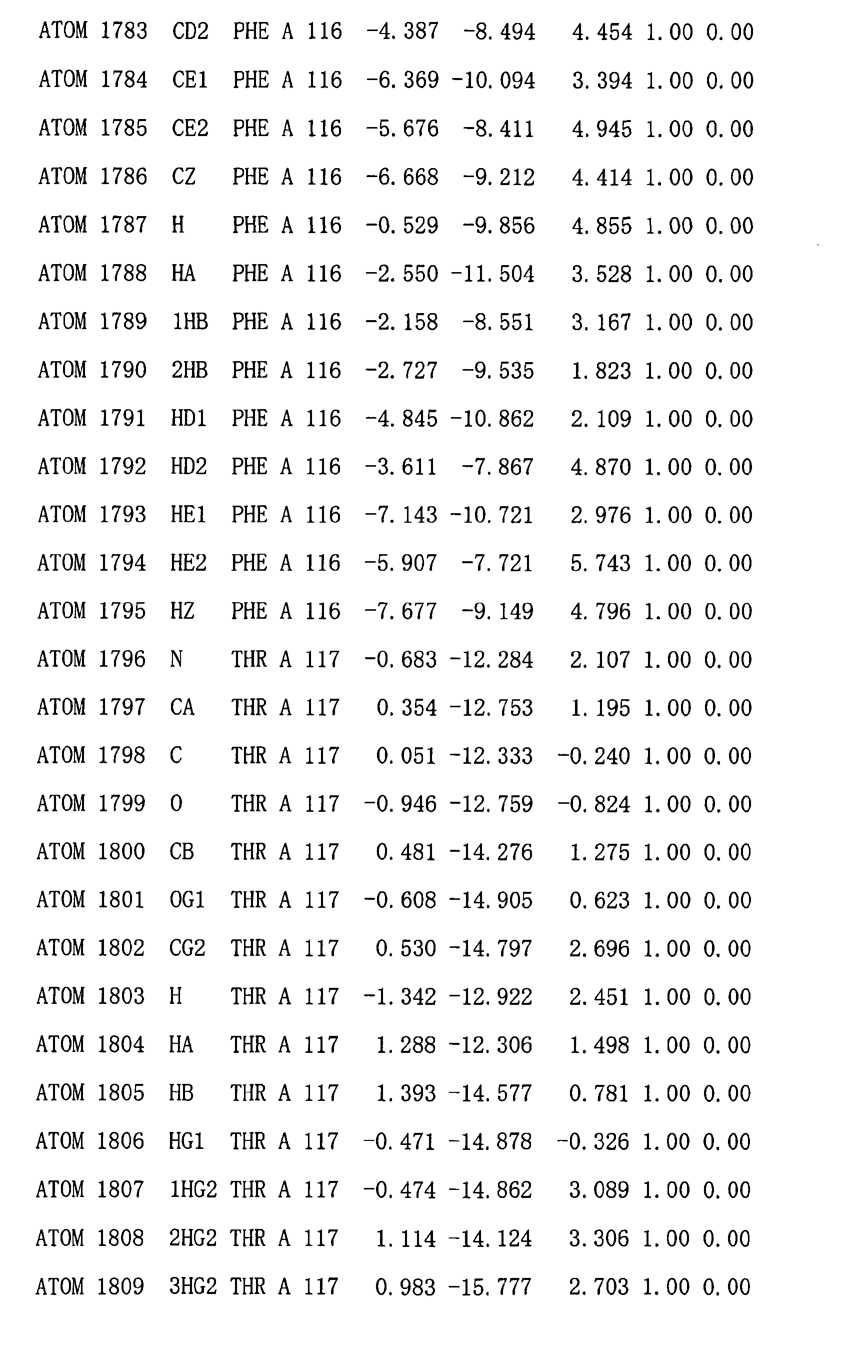

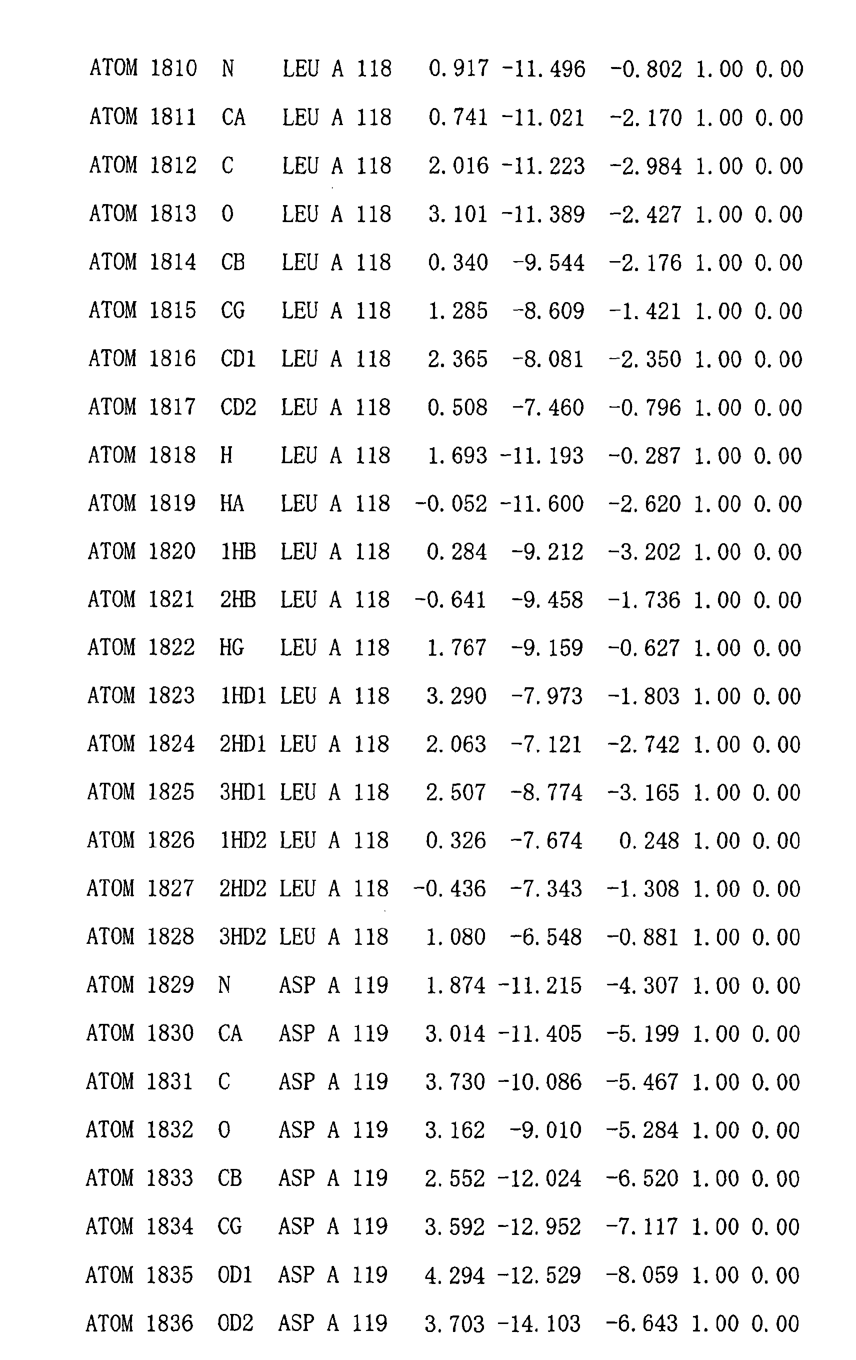

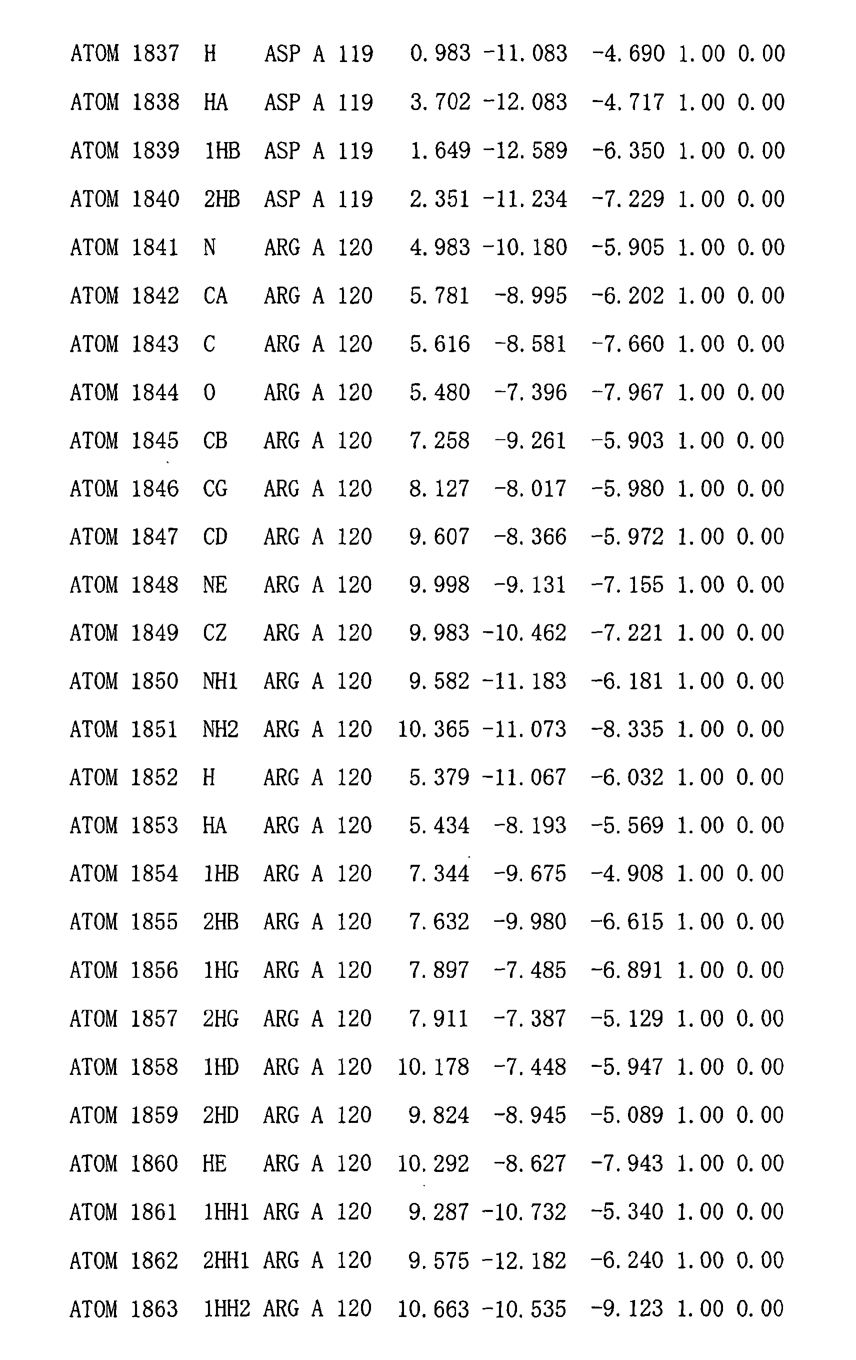

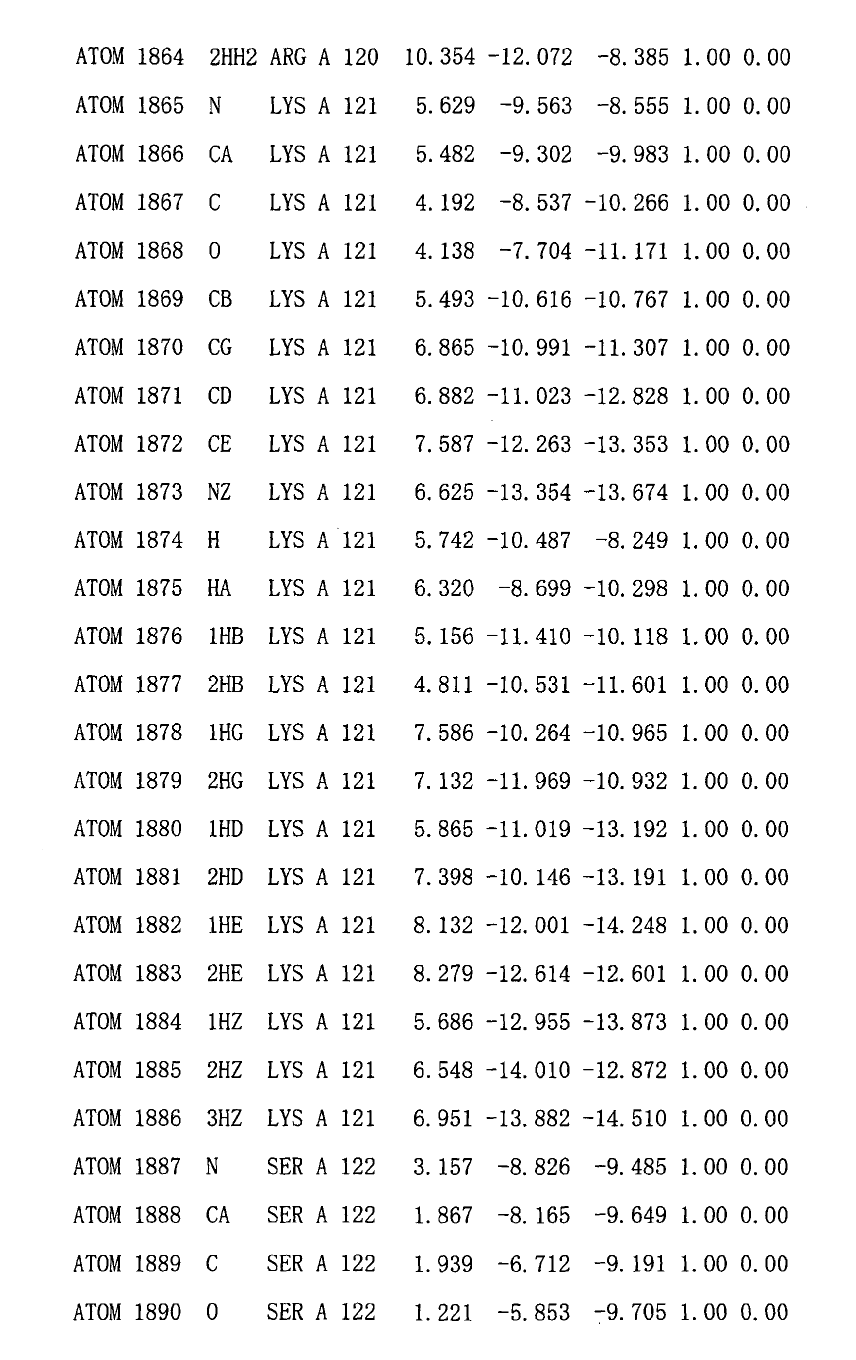

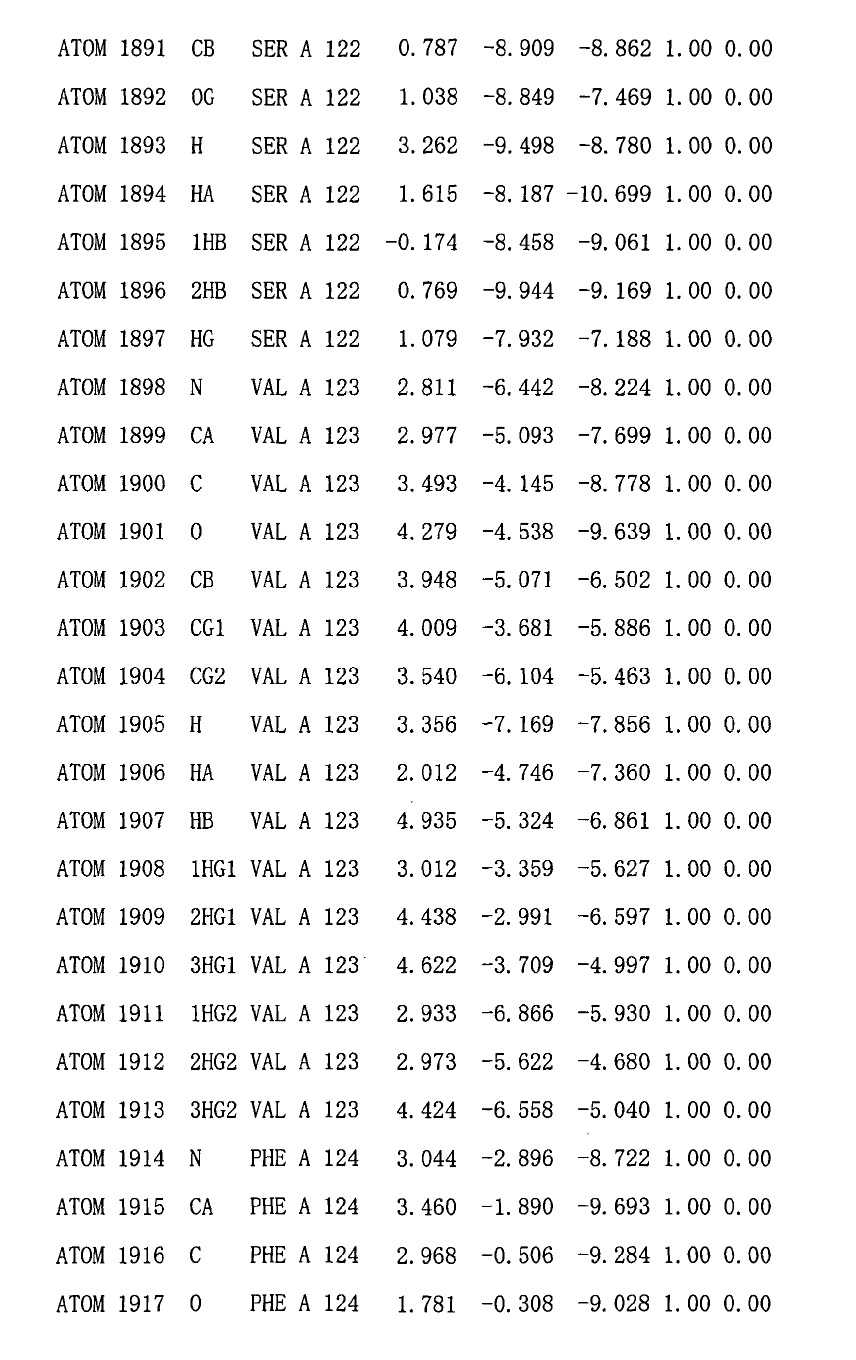

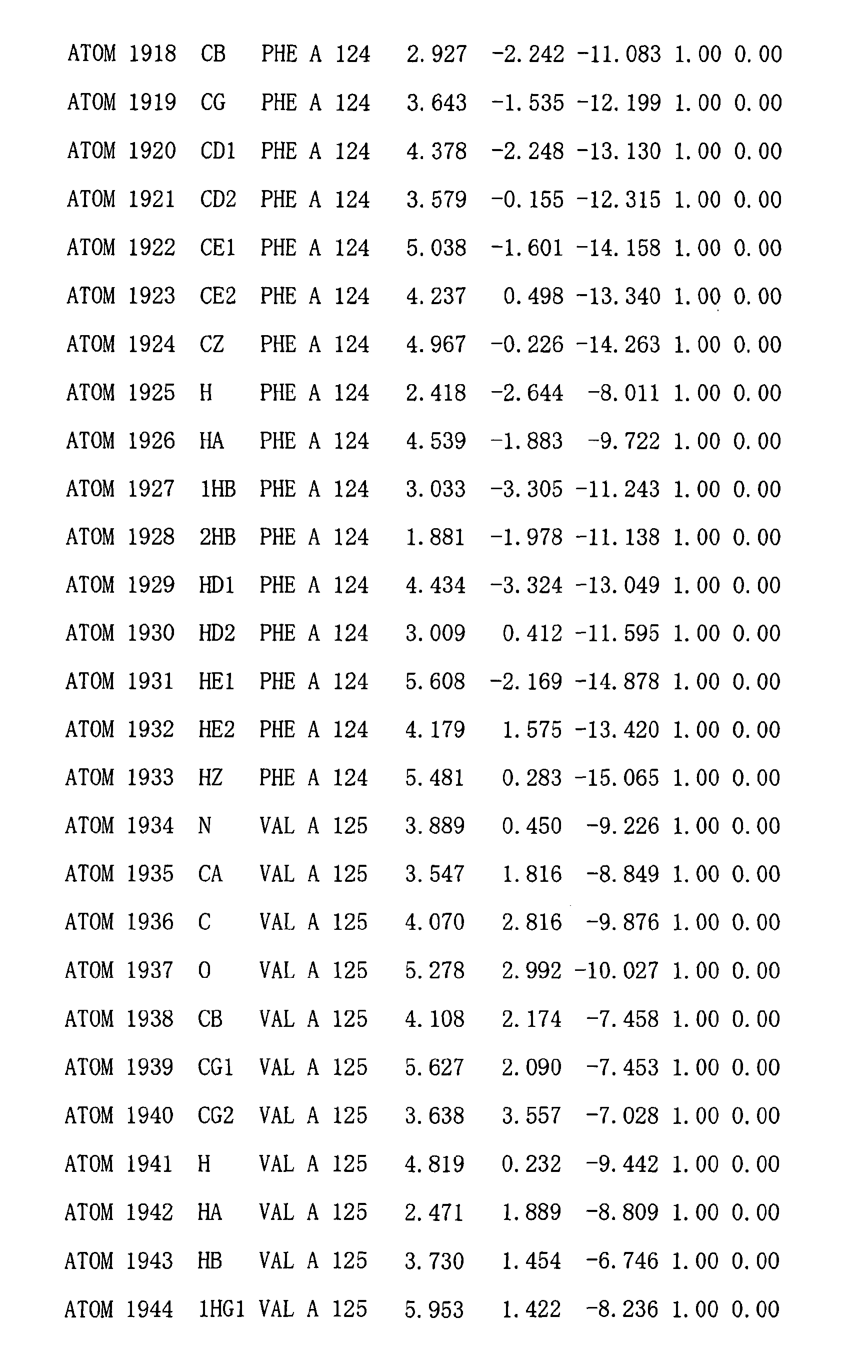

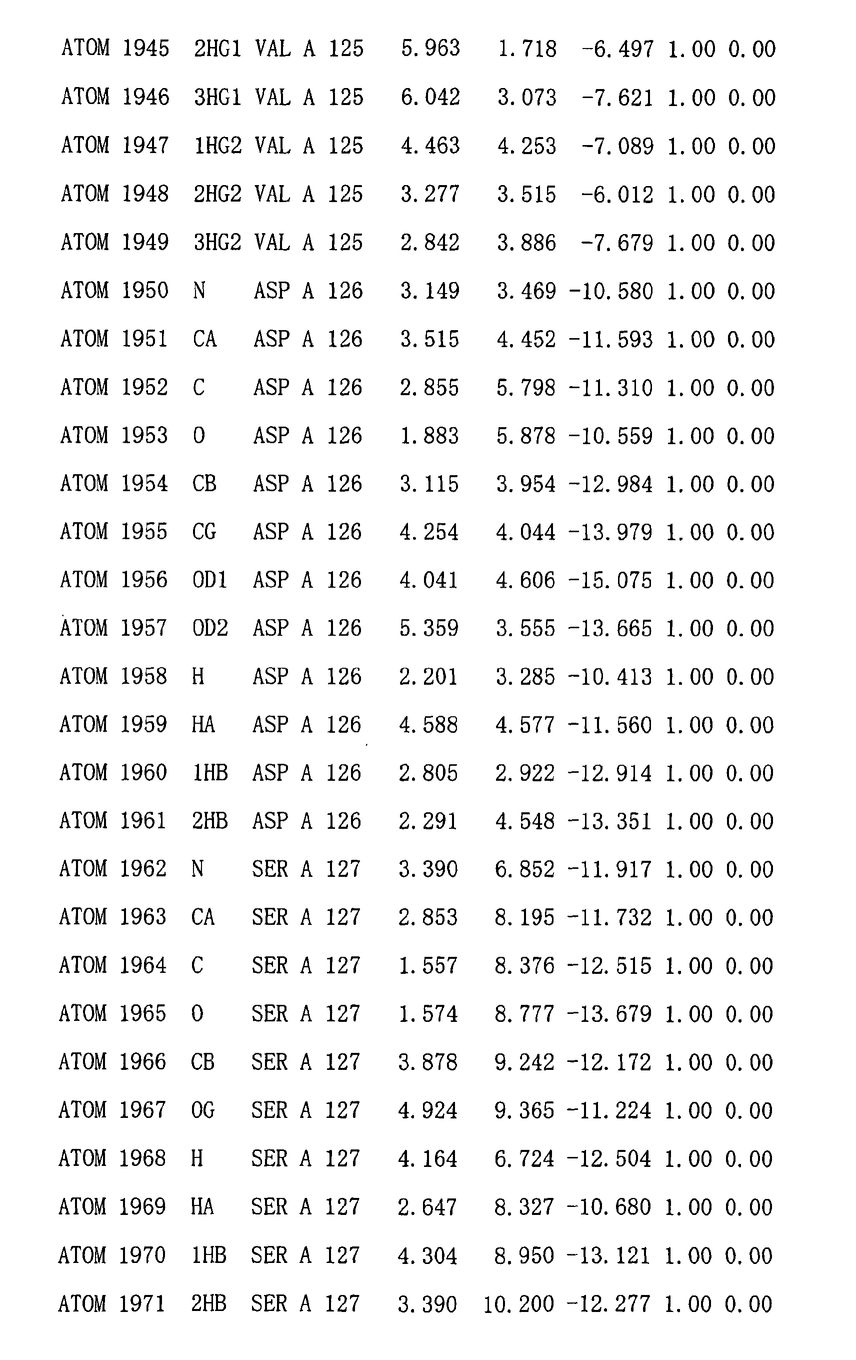

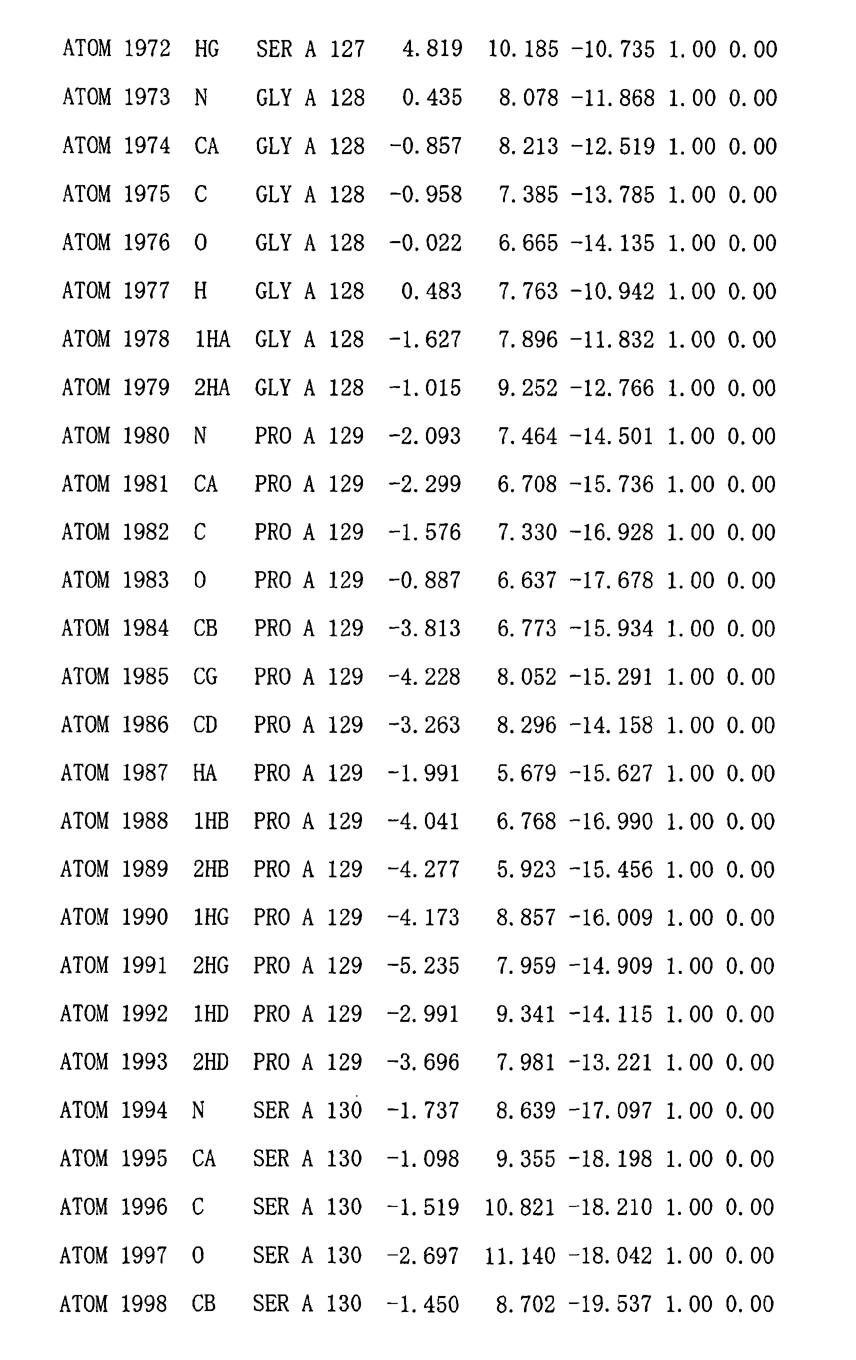

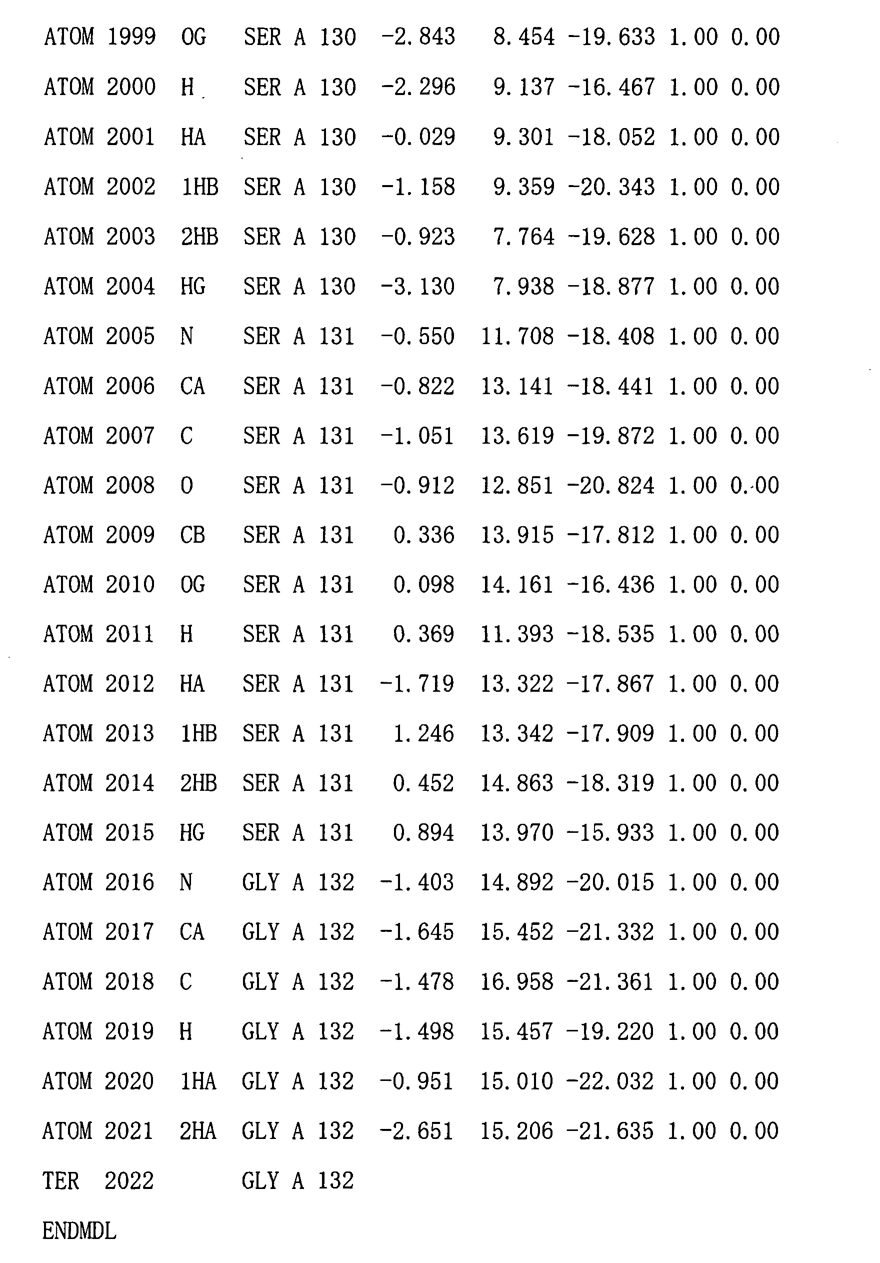

### Three-Dimensional Structure Coordinate 17

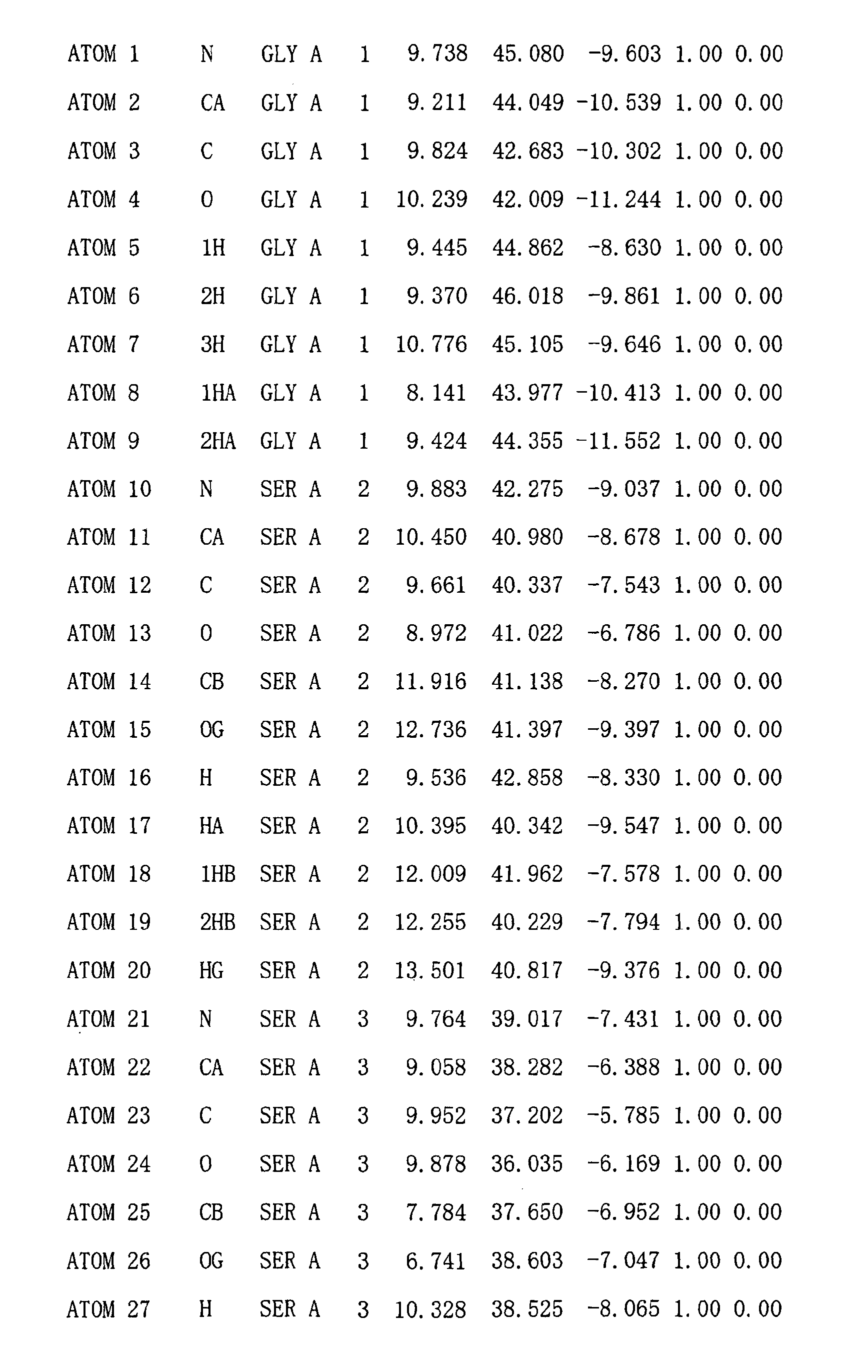

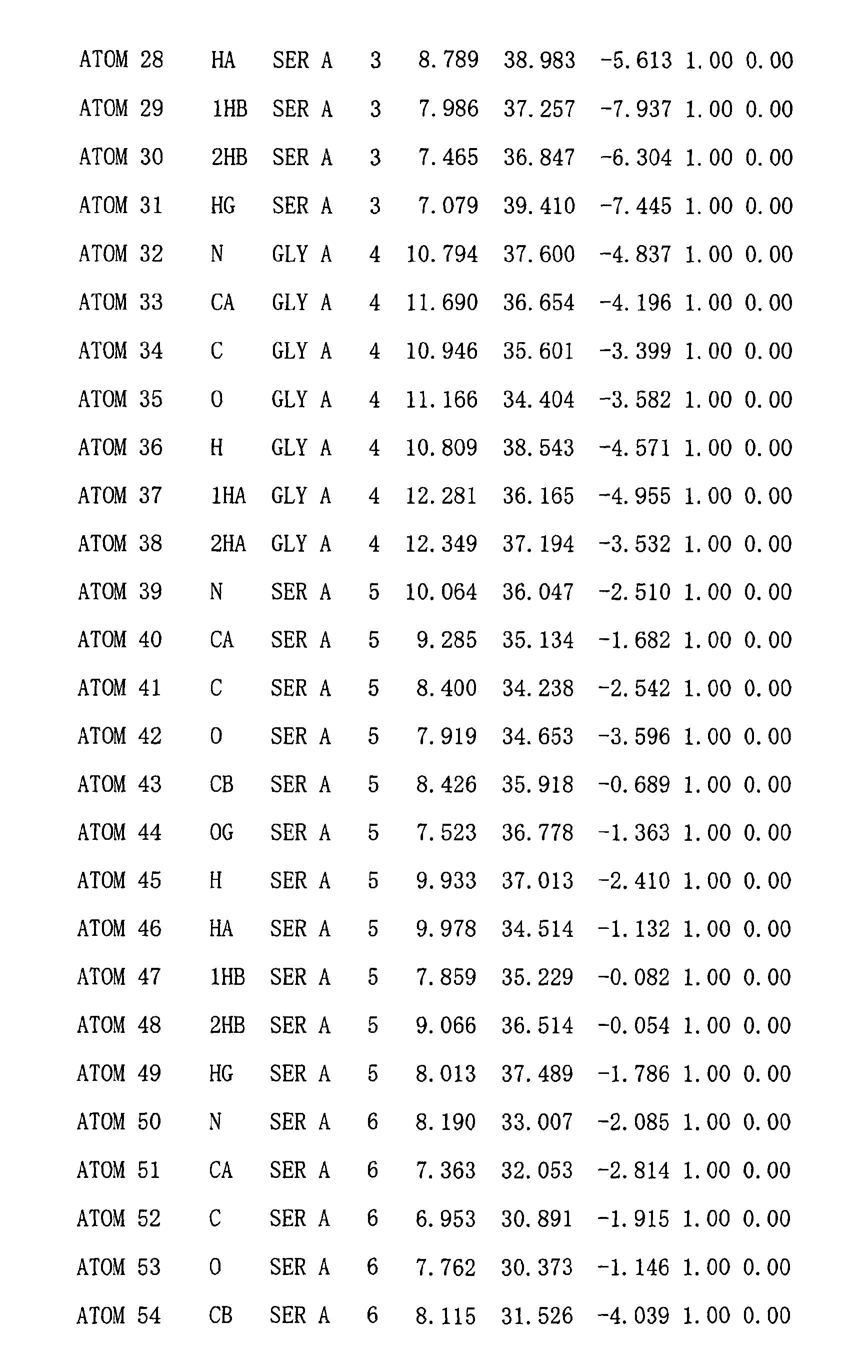

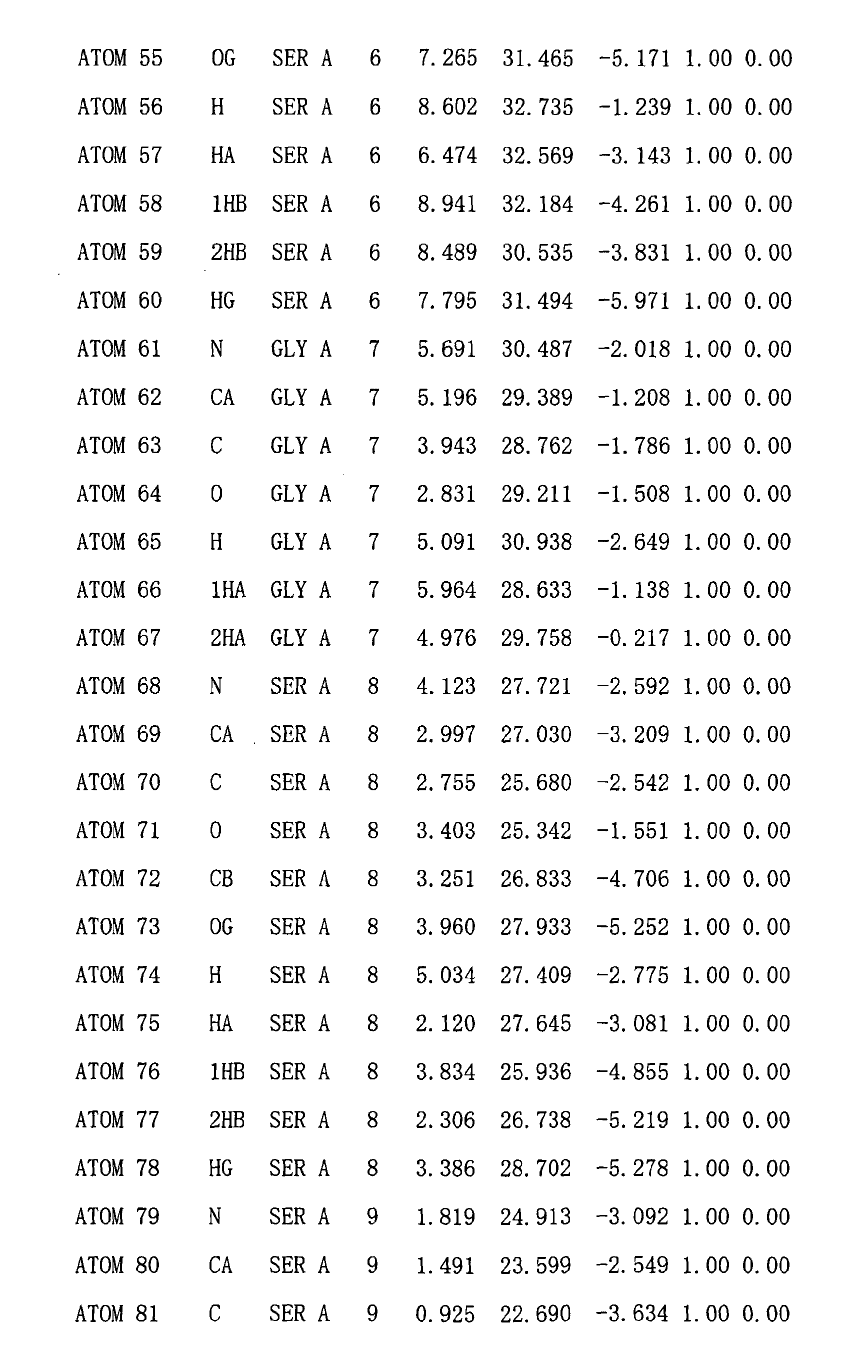

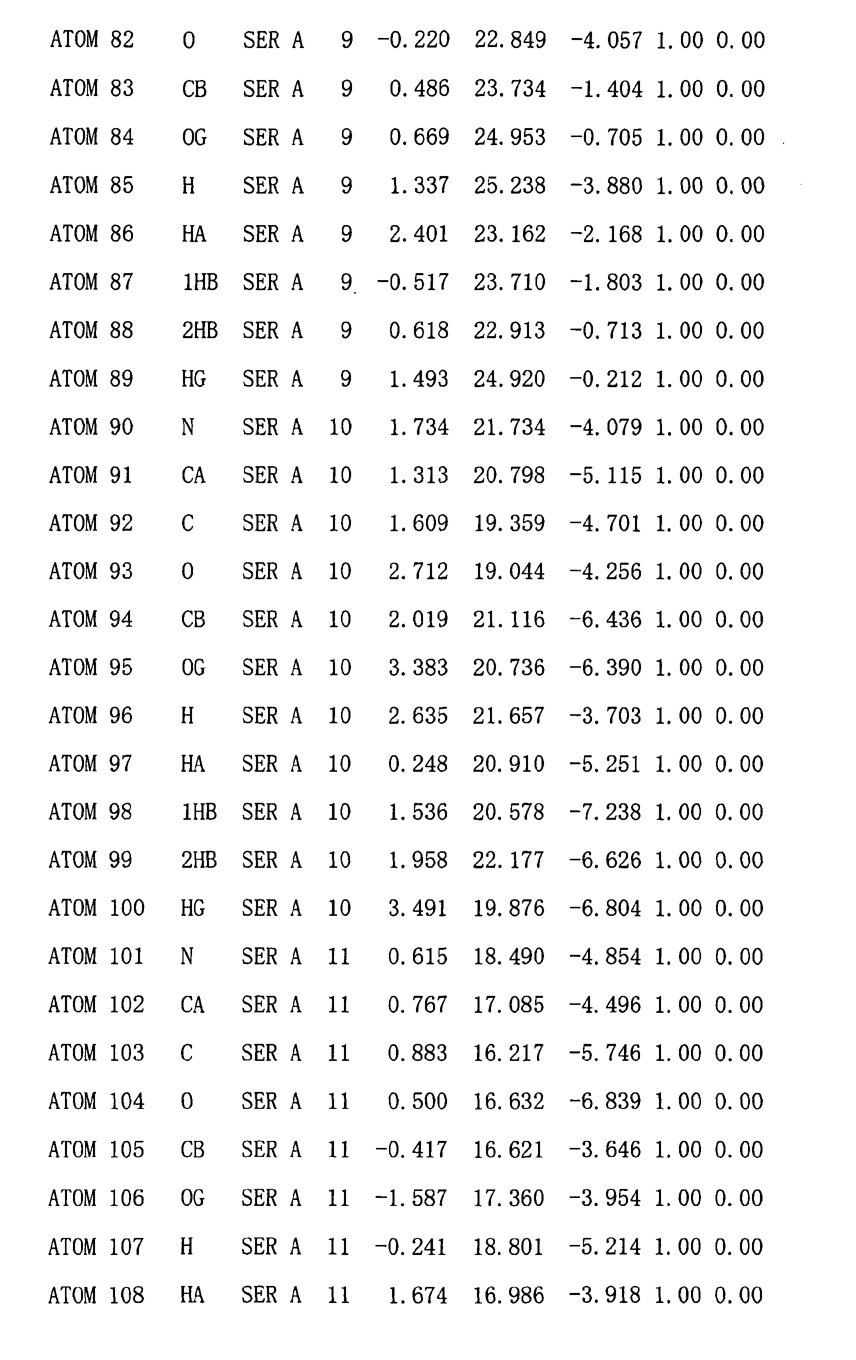

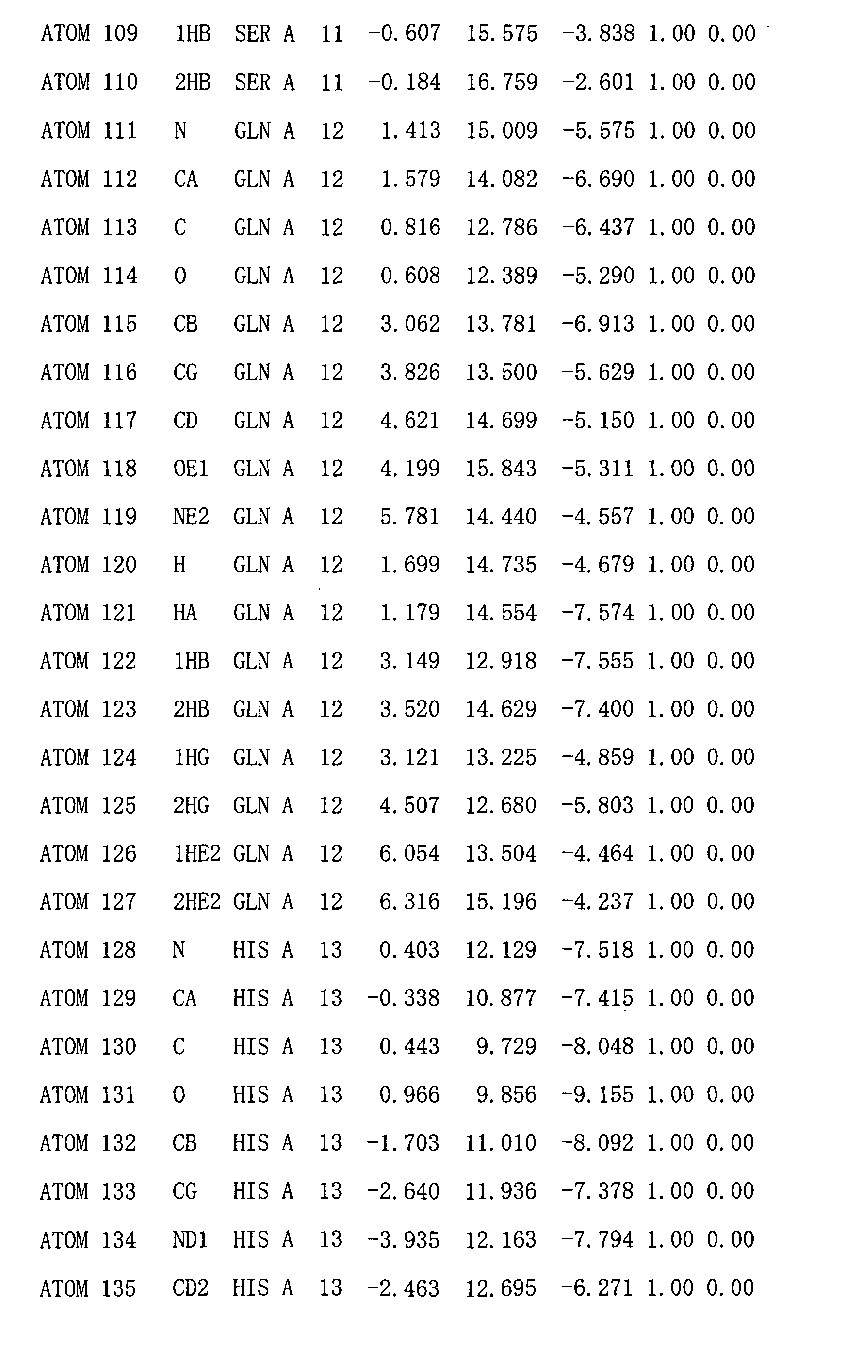

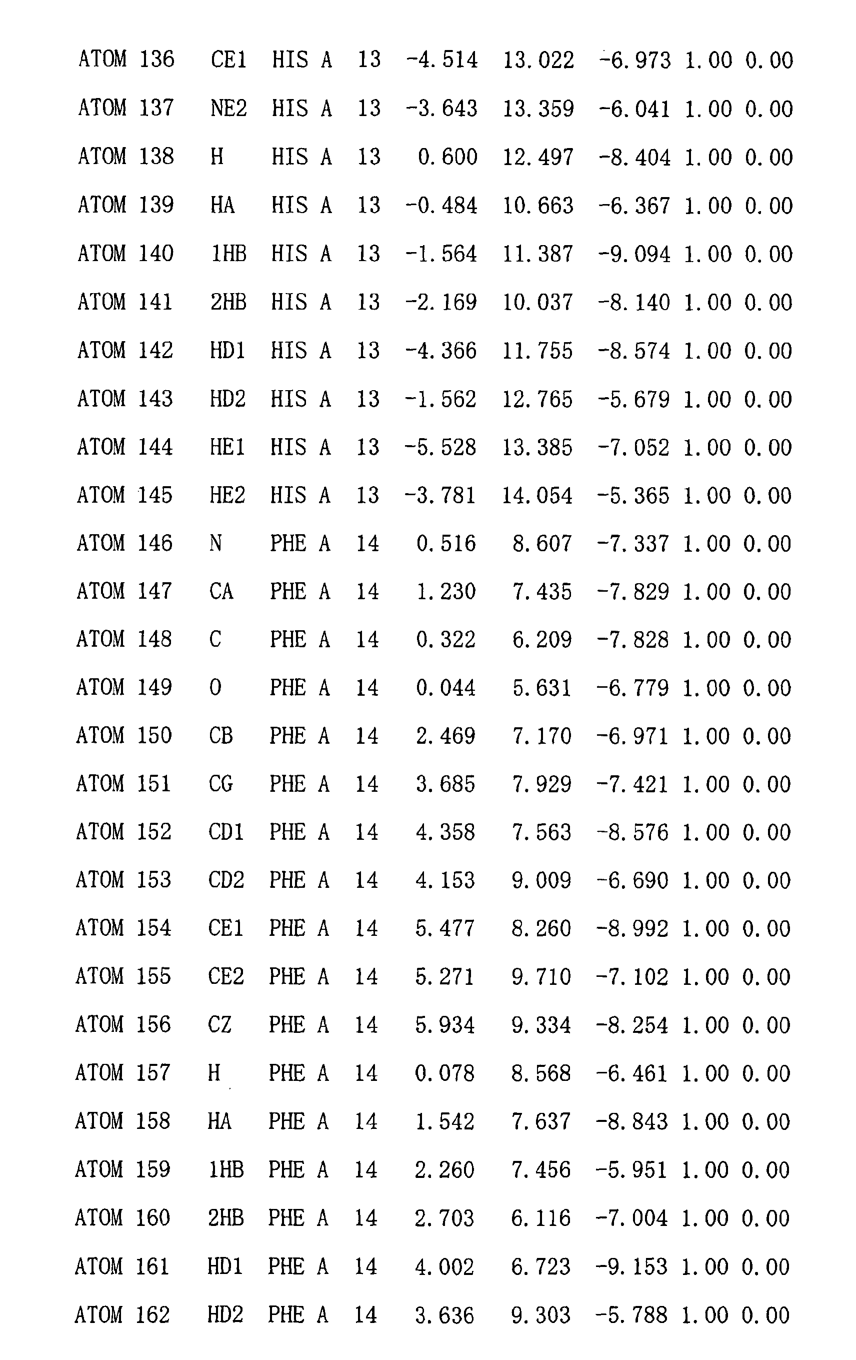

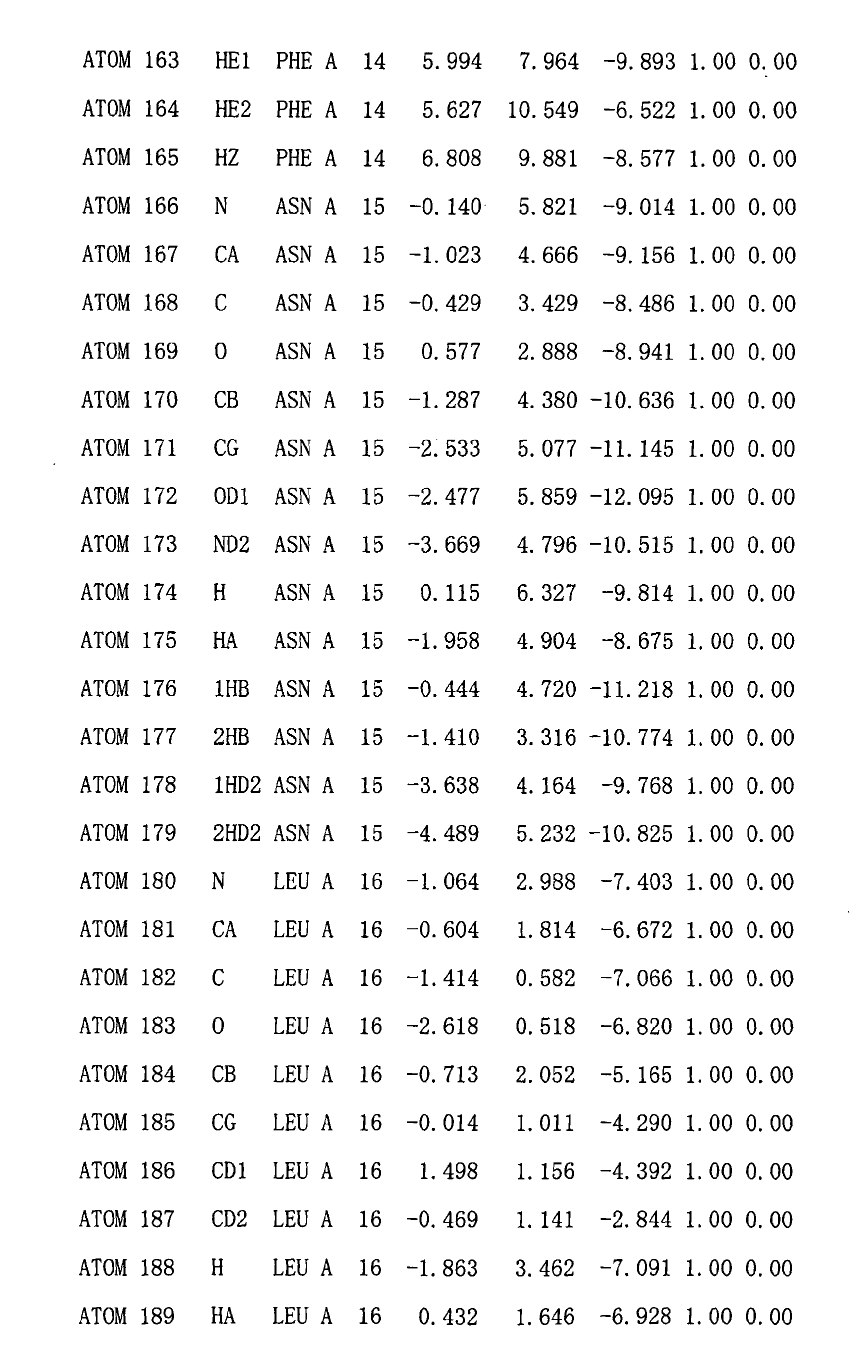

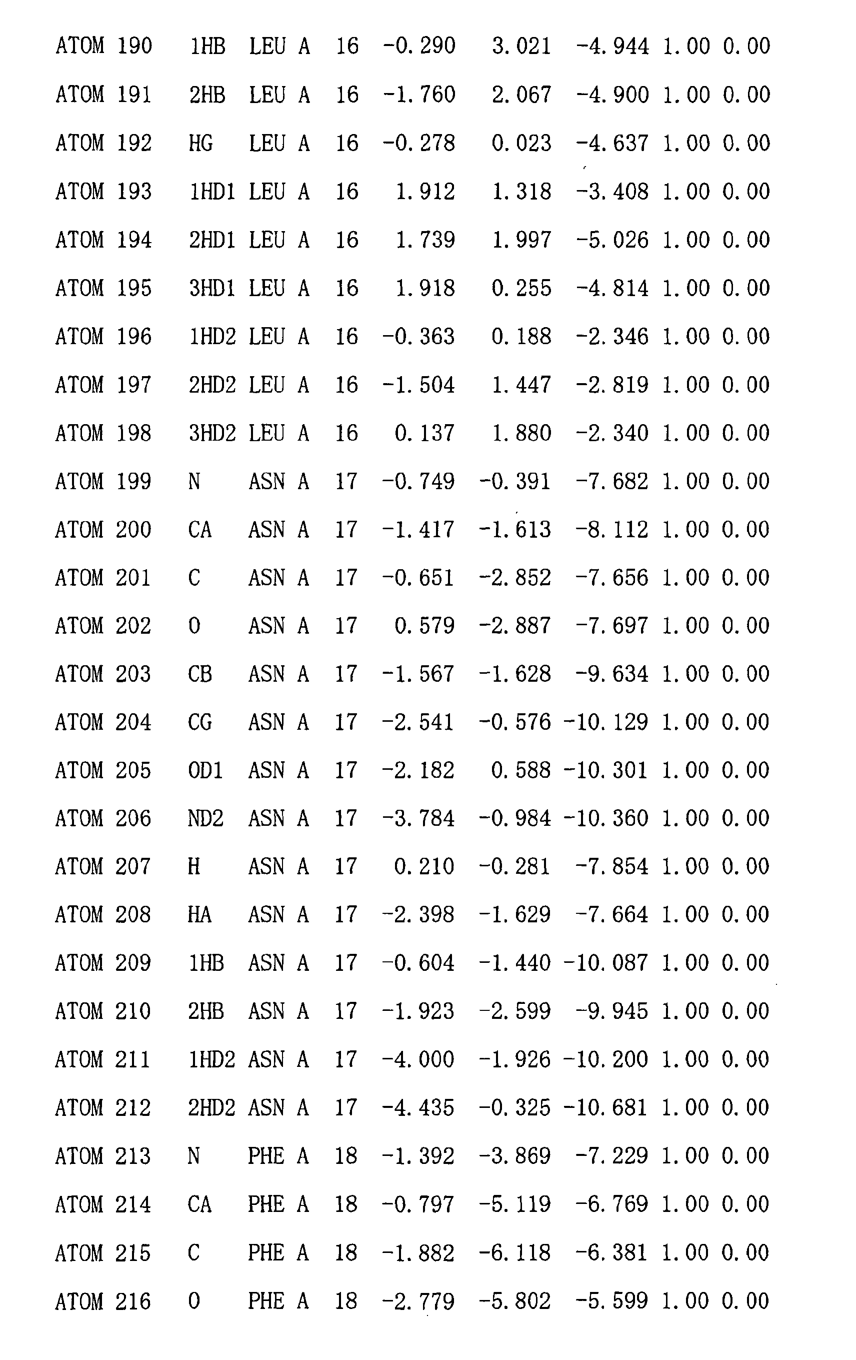

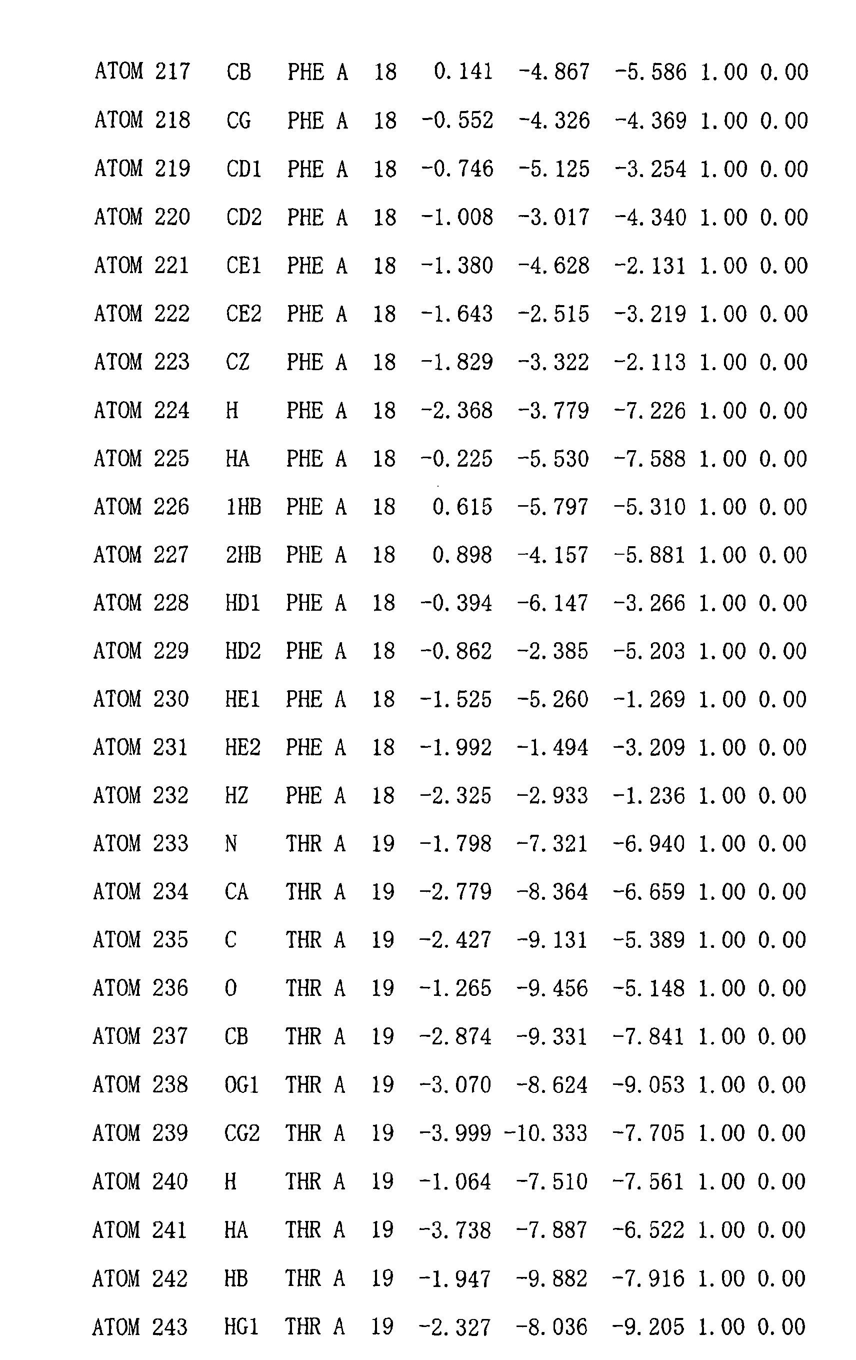

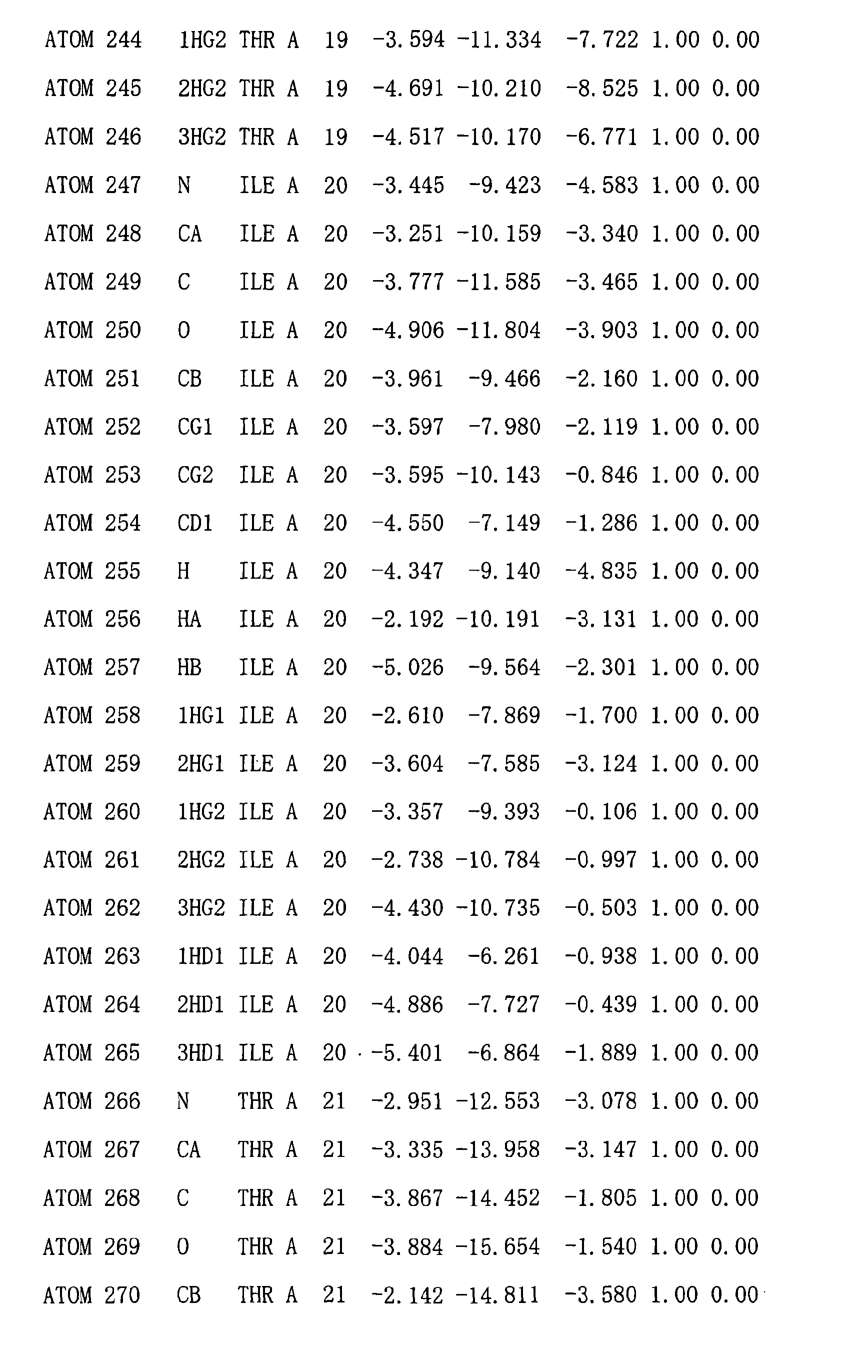

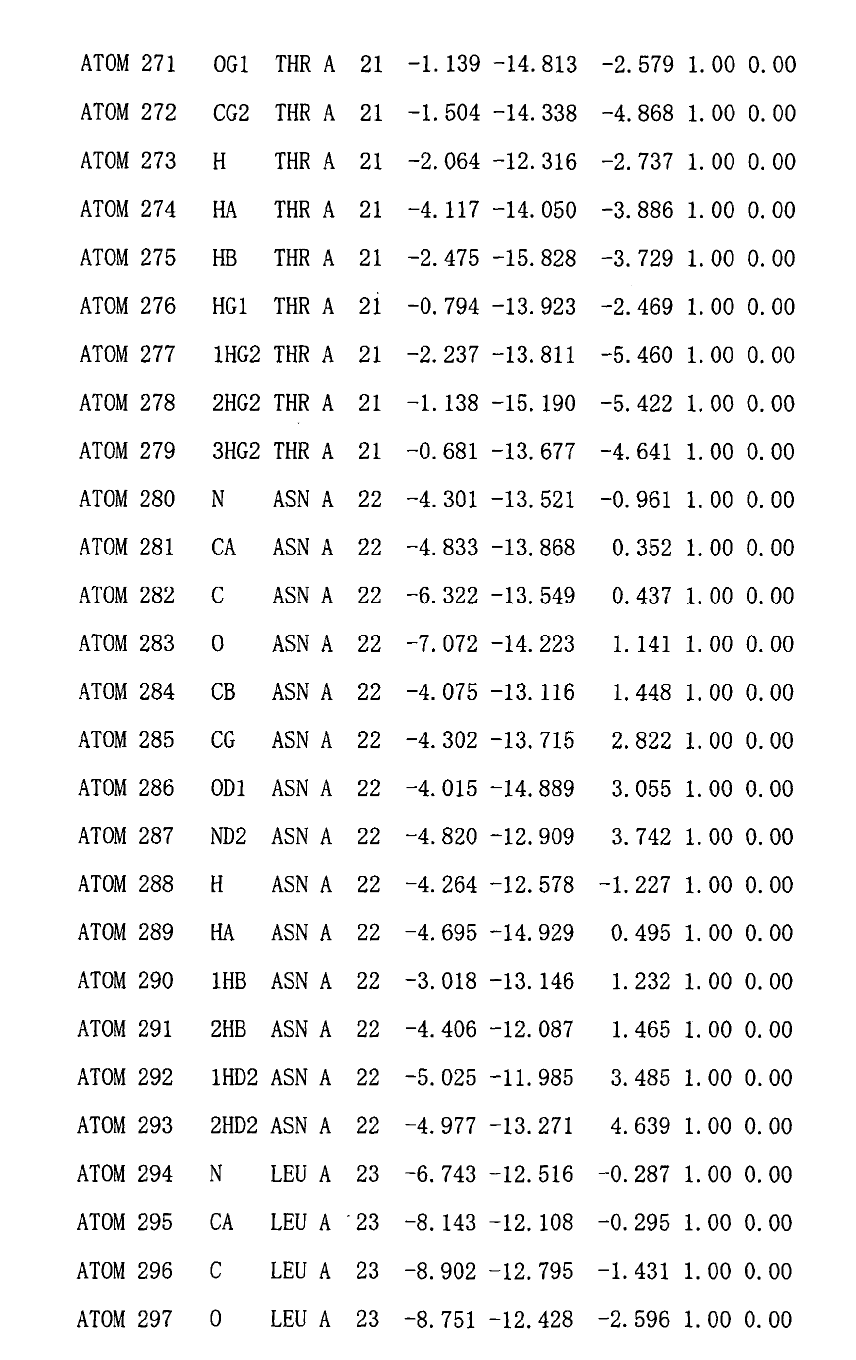

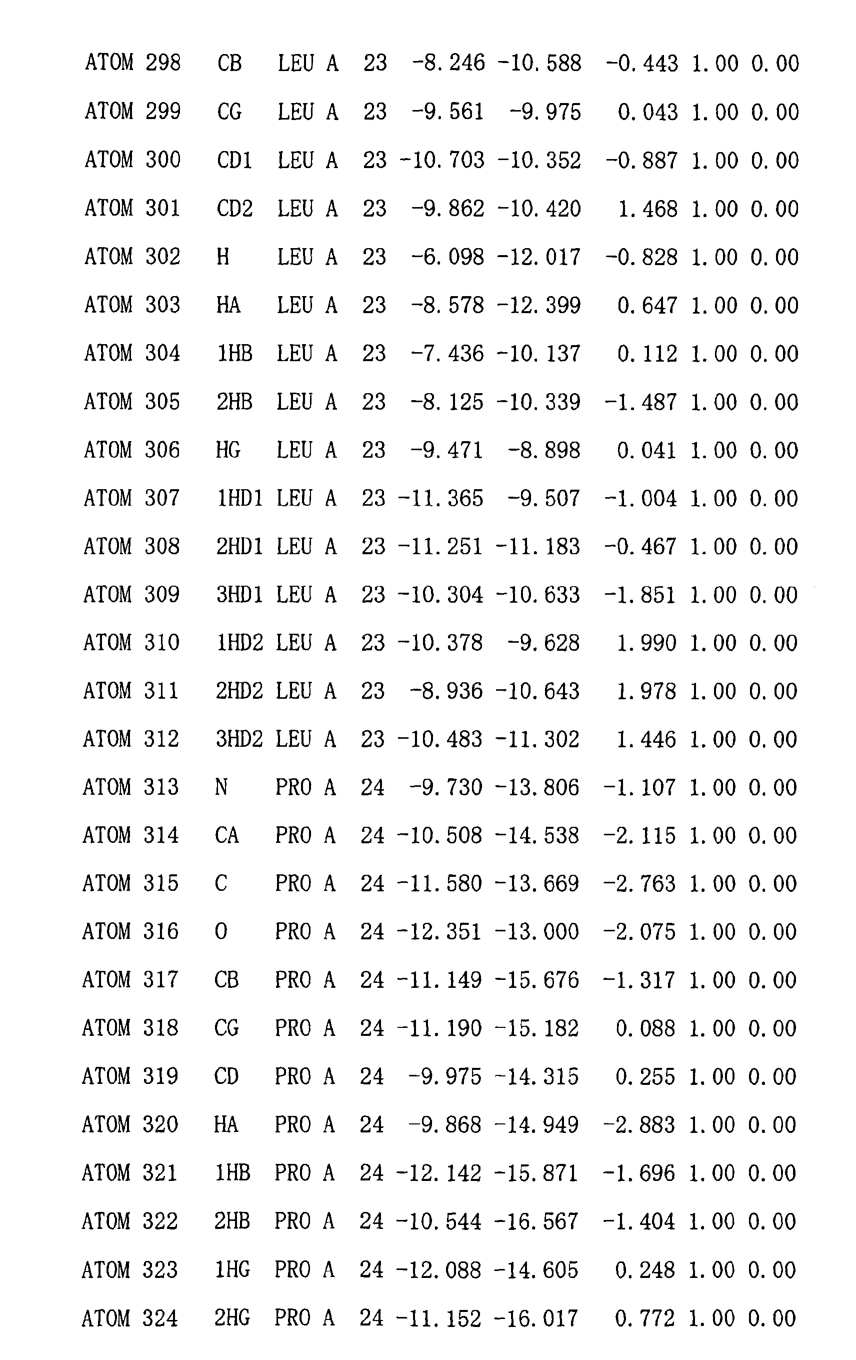

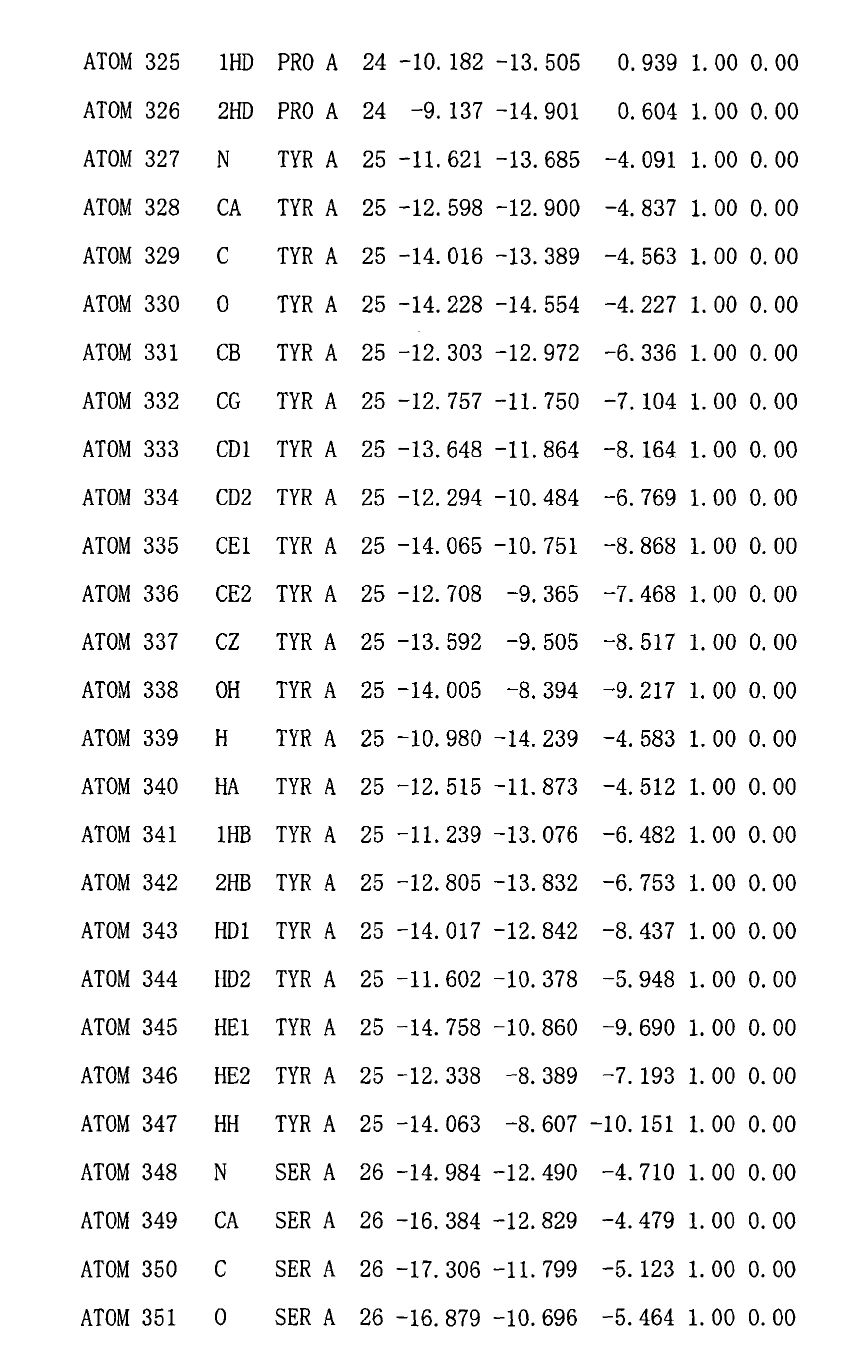

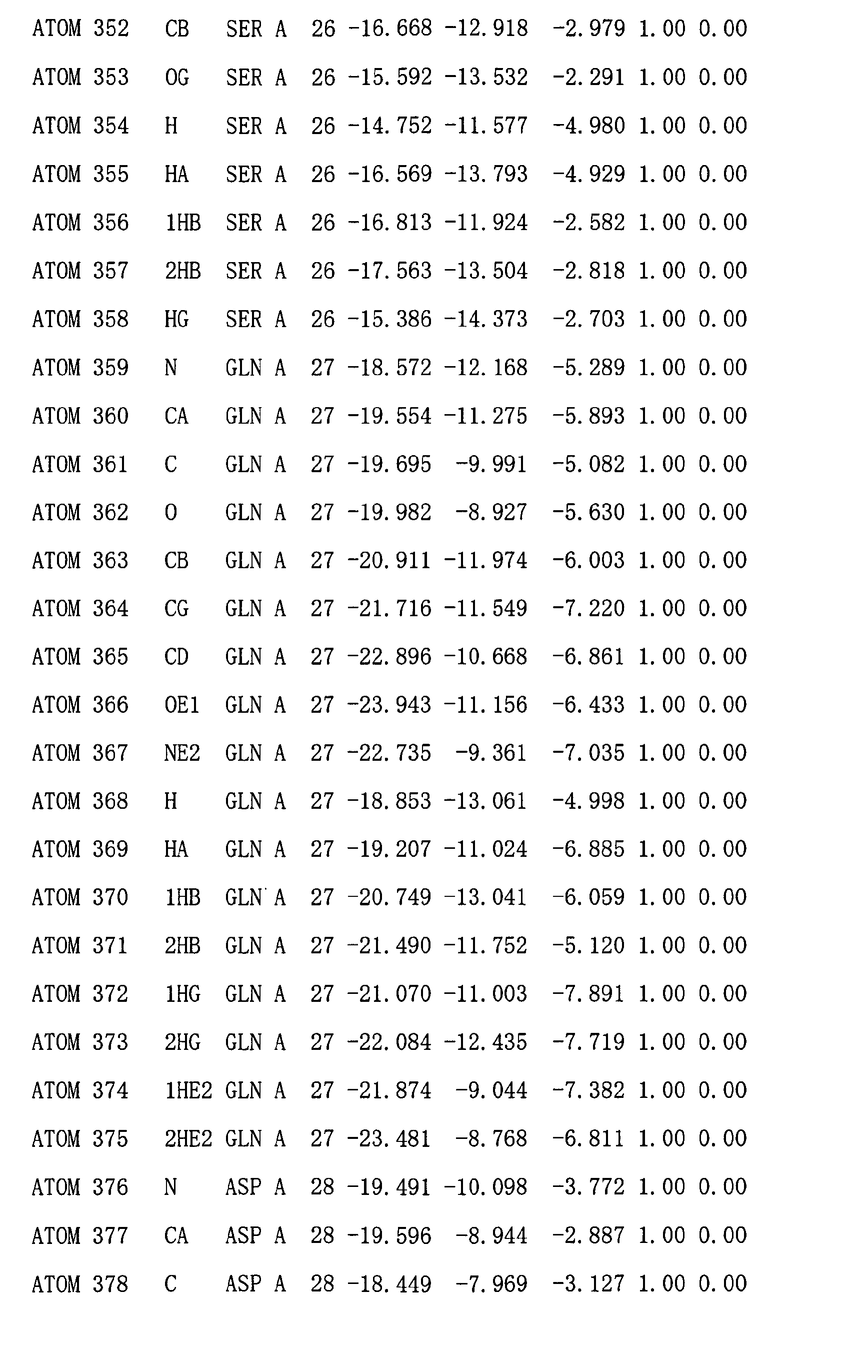

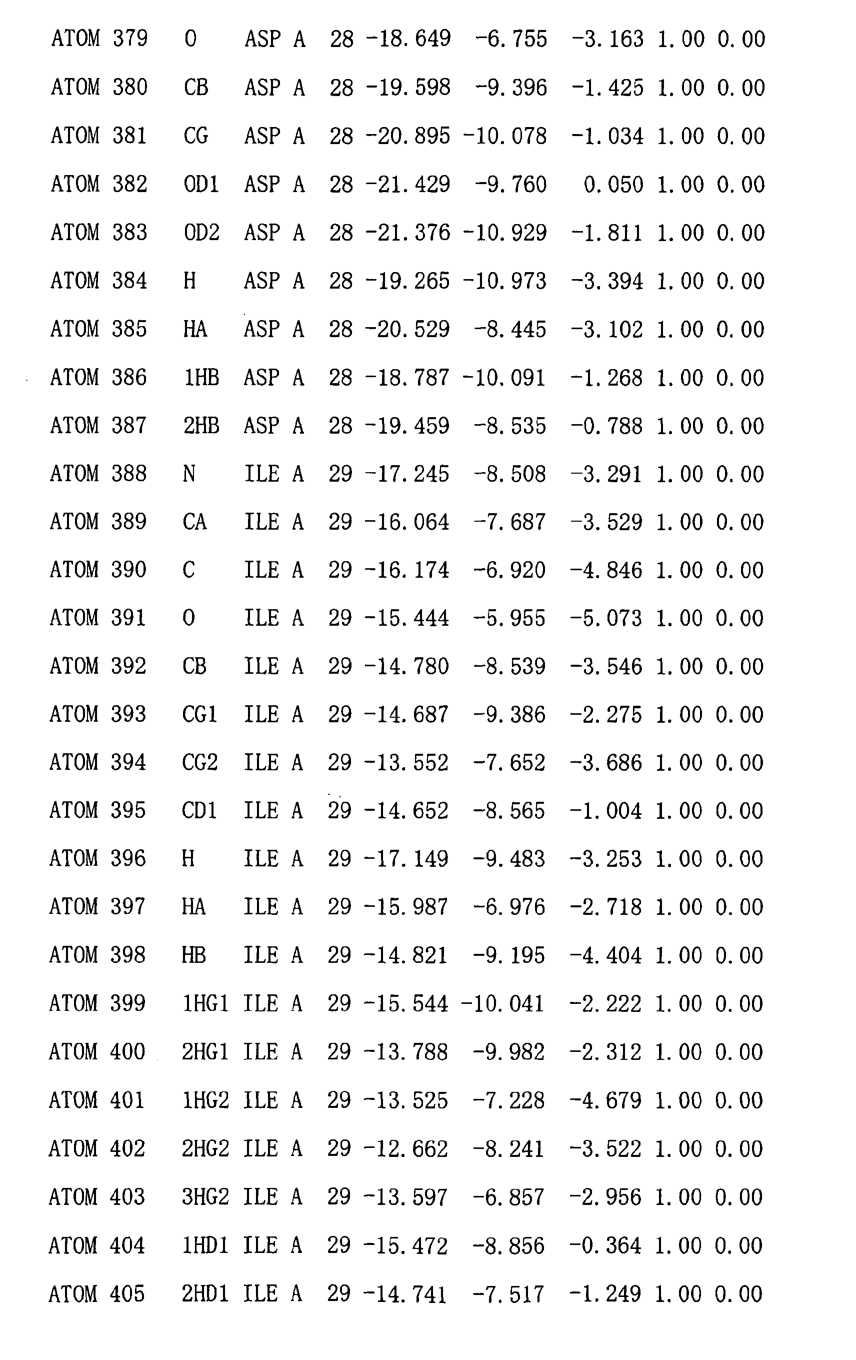

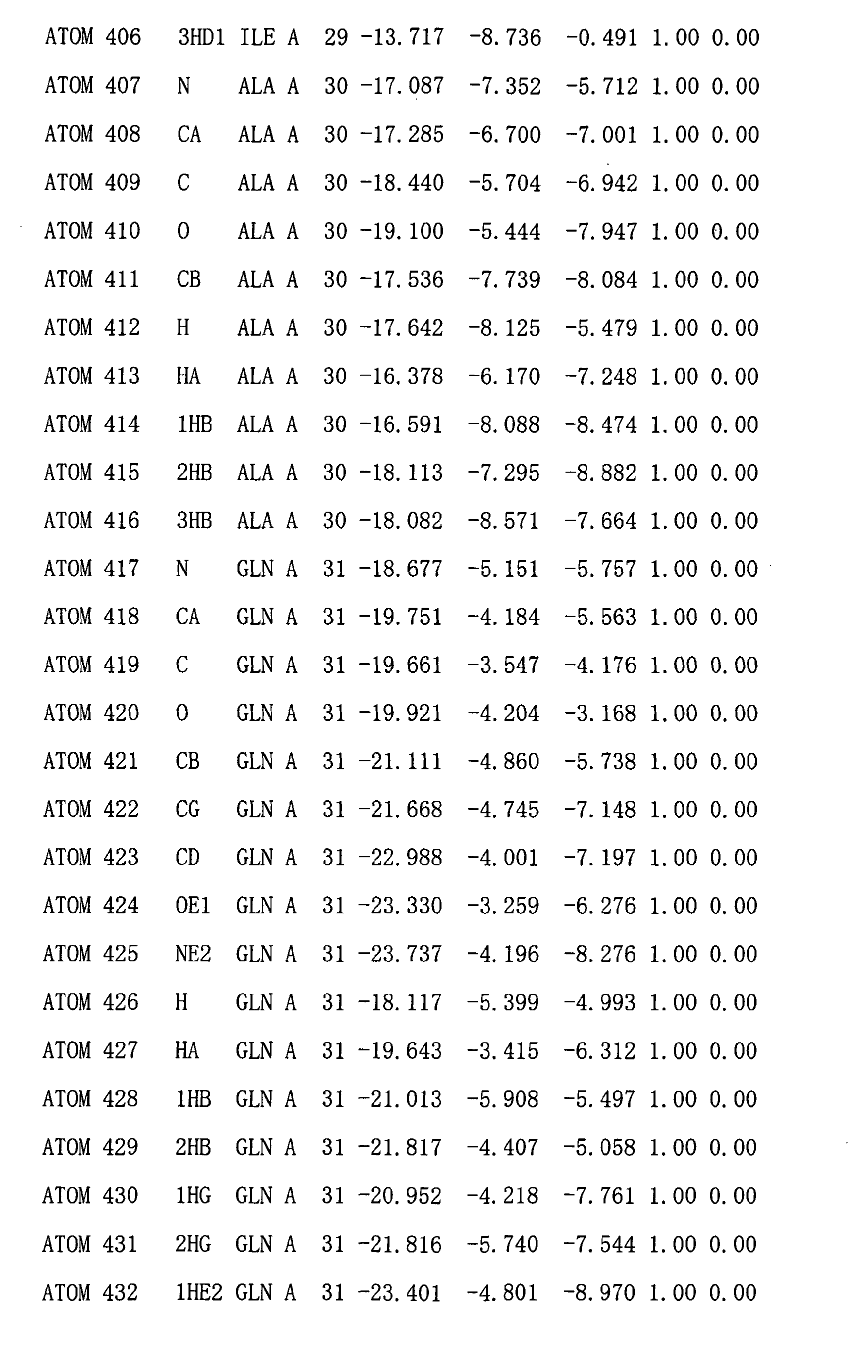

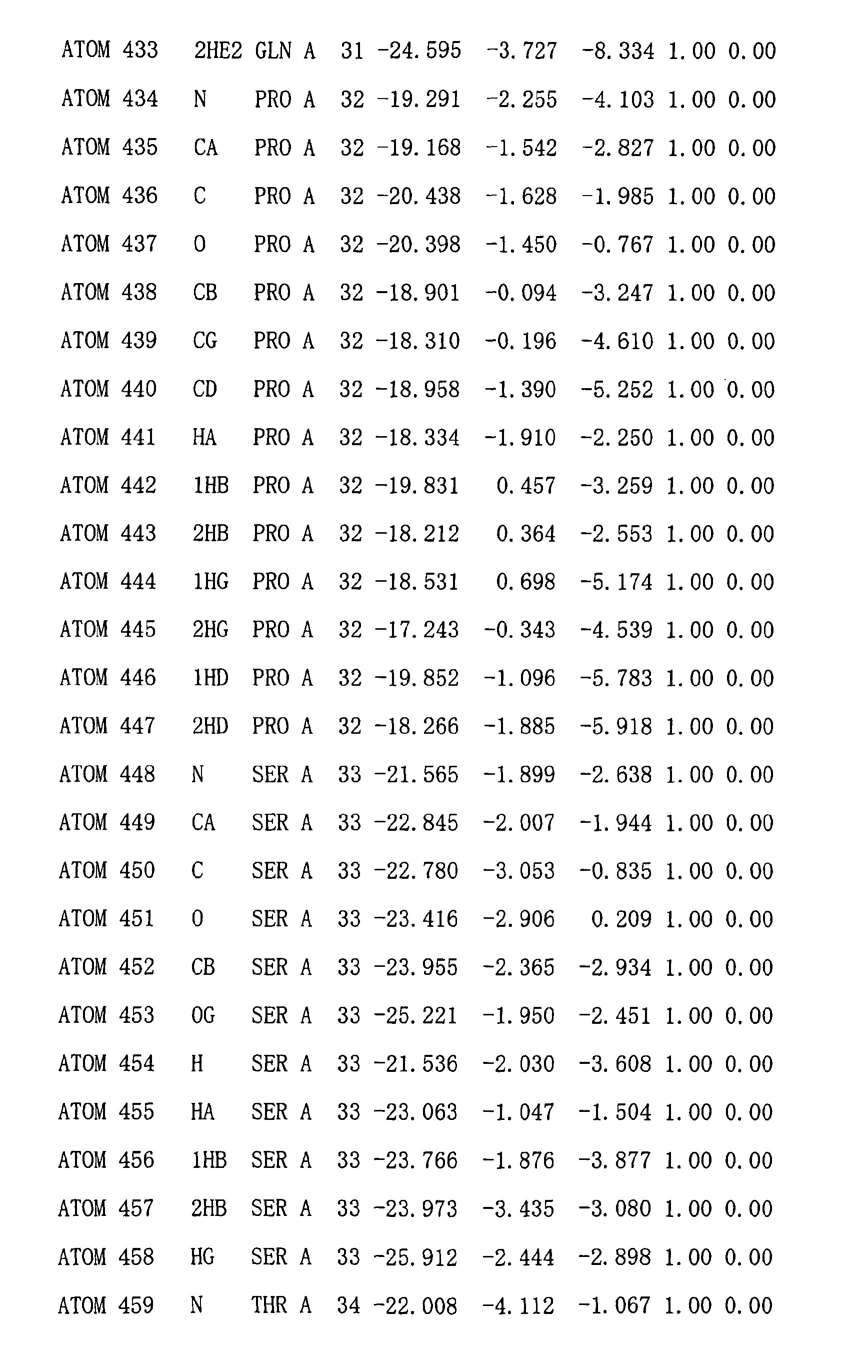

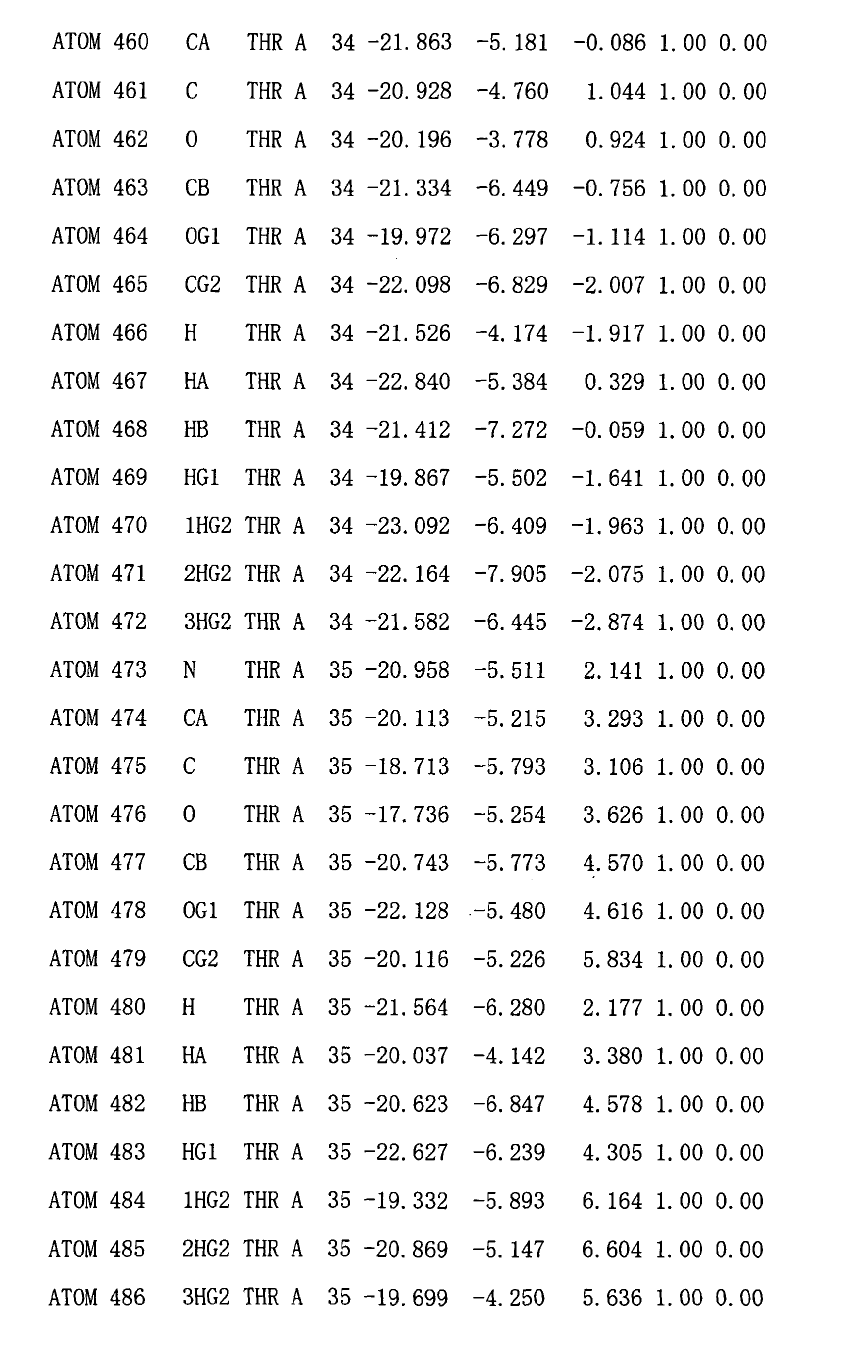

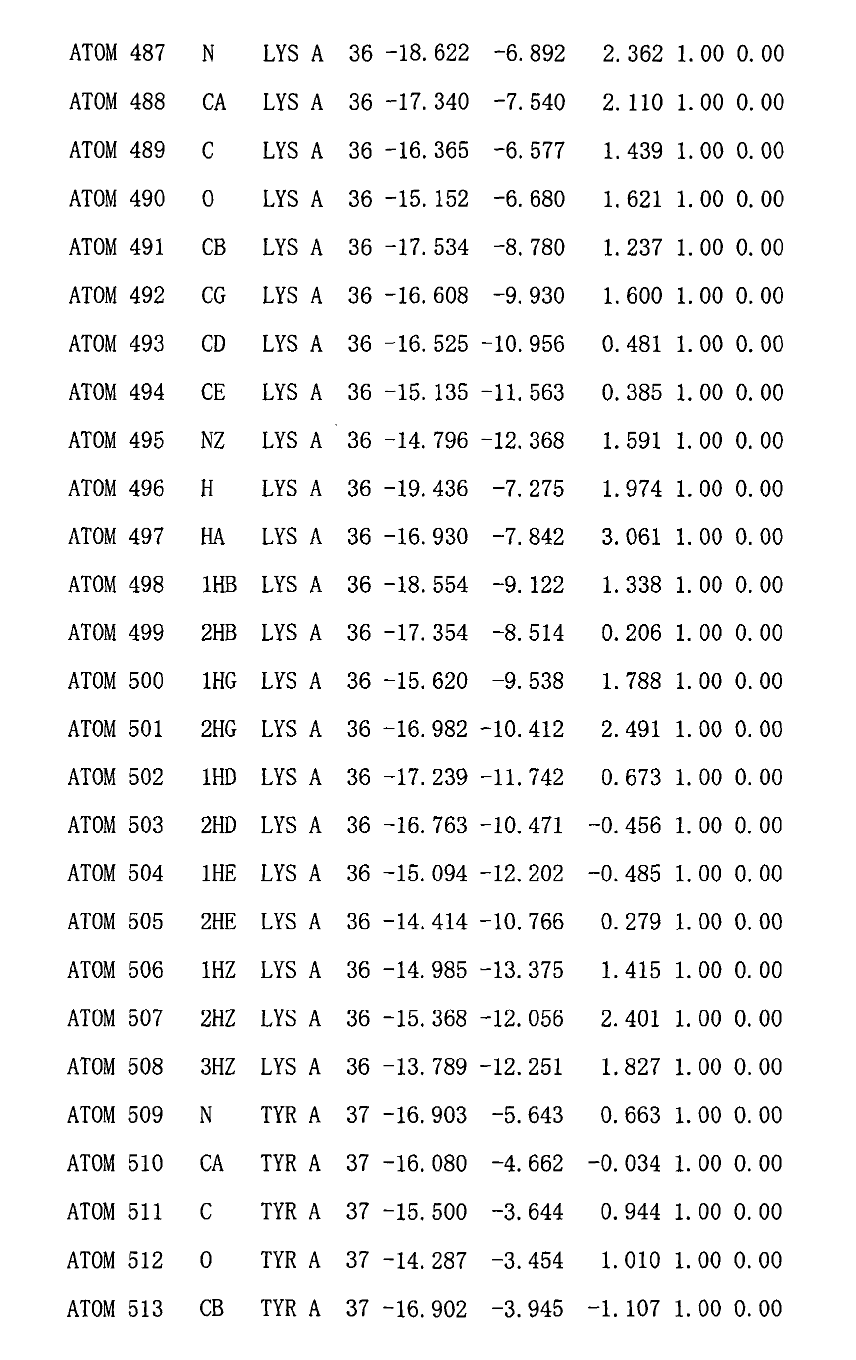

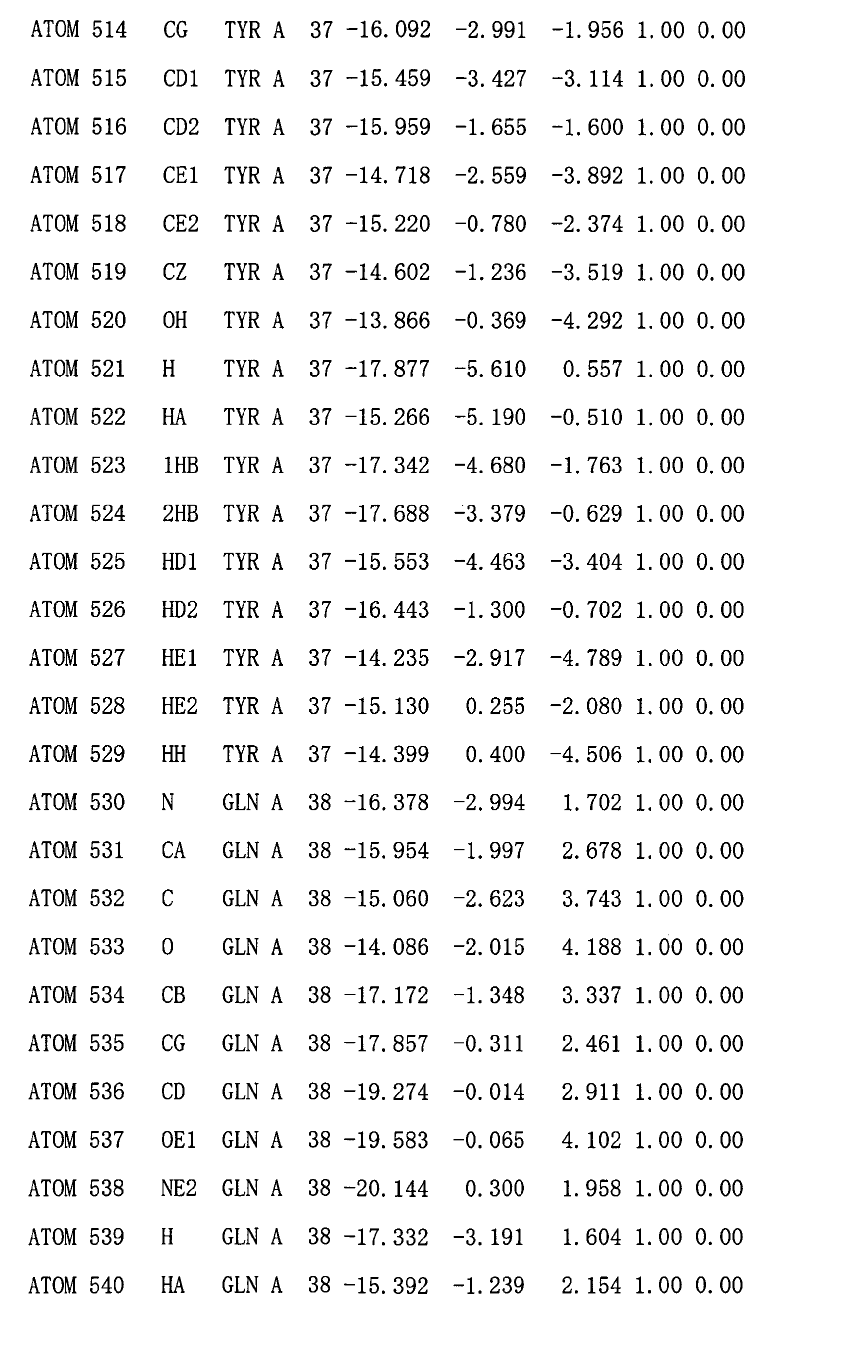

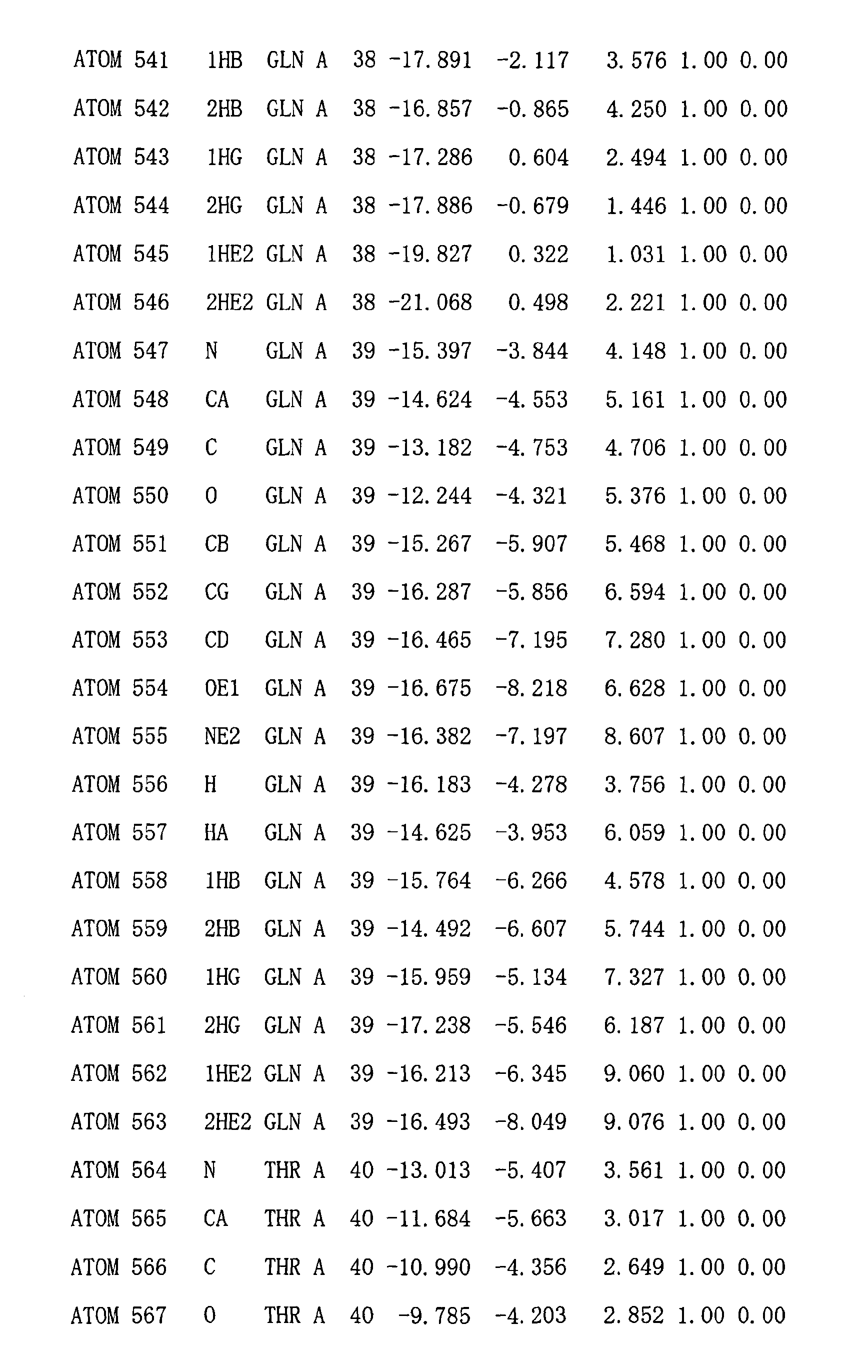

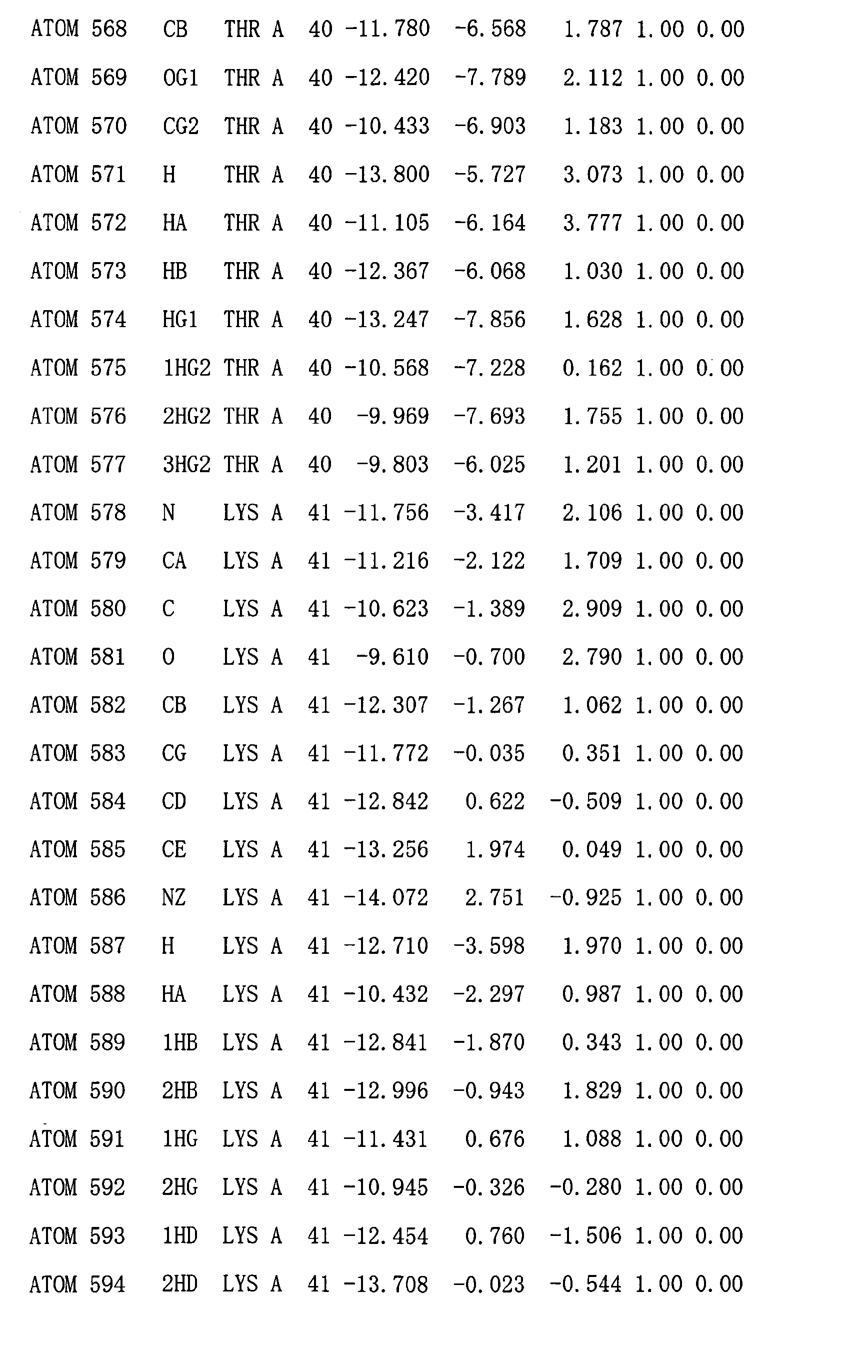

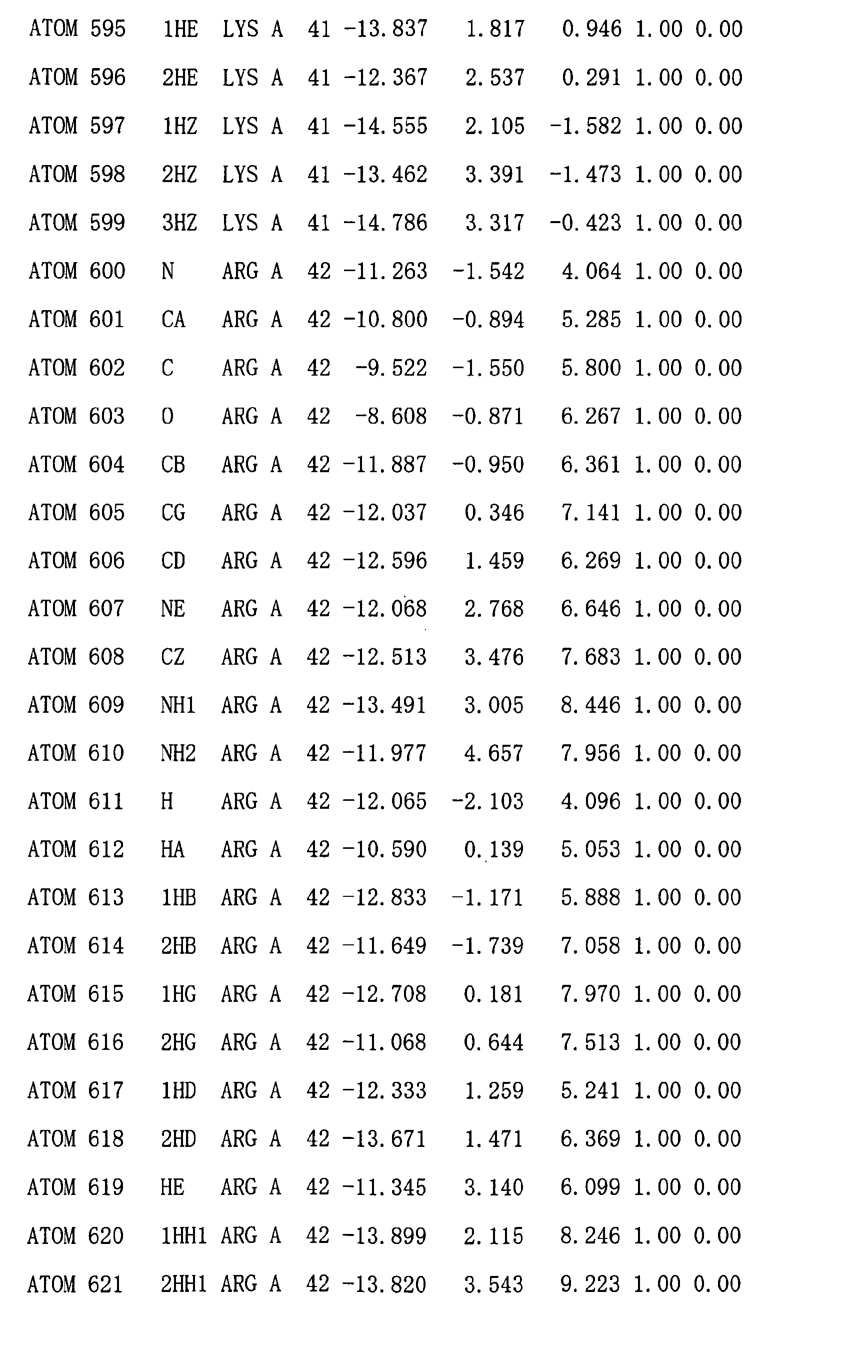

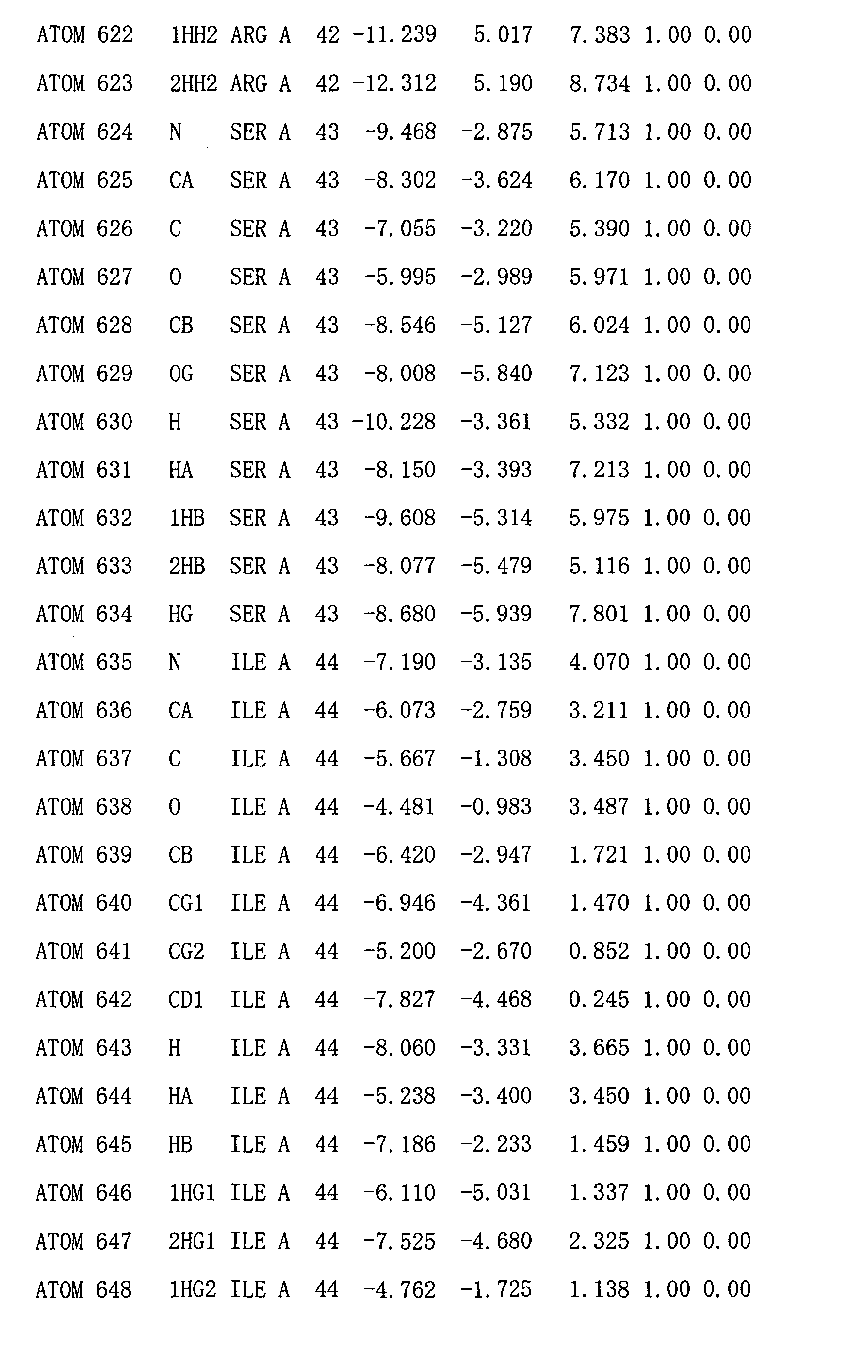

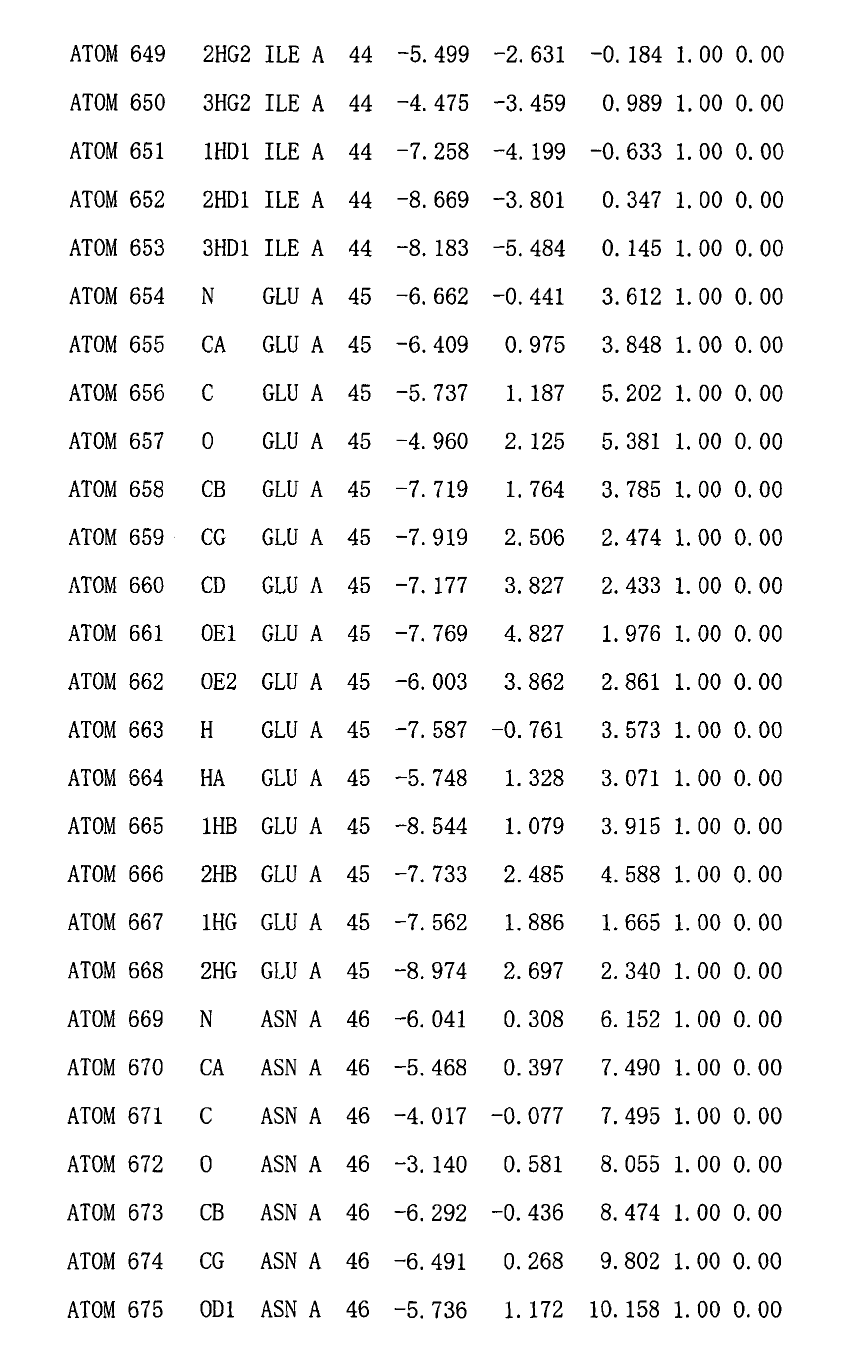

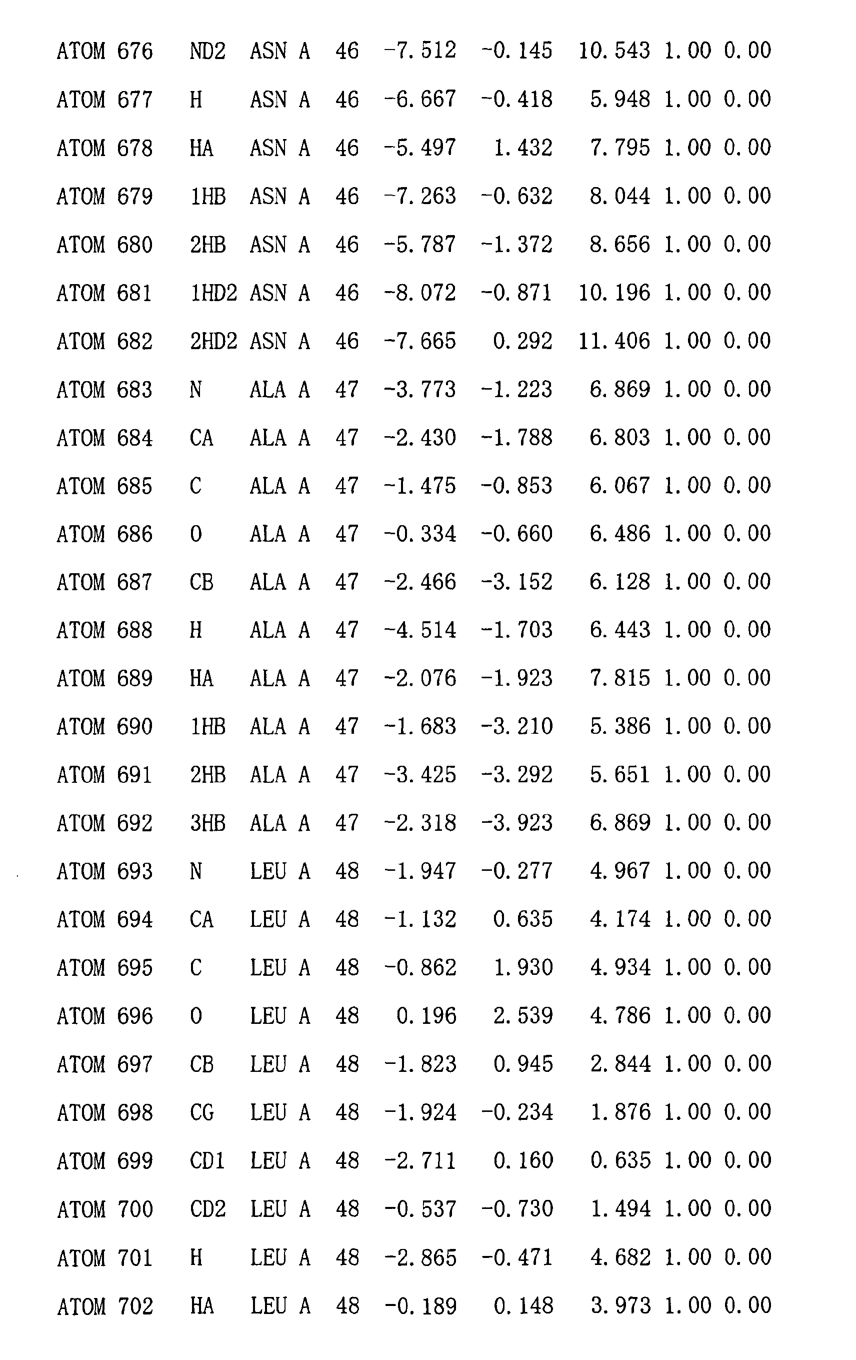

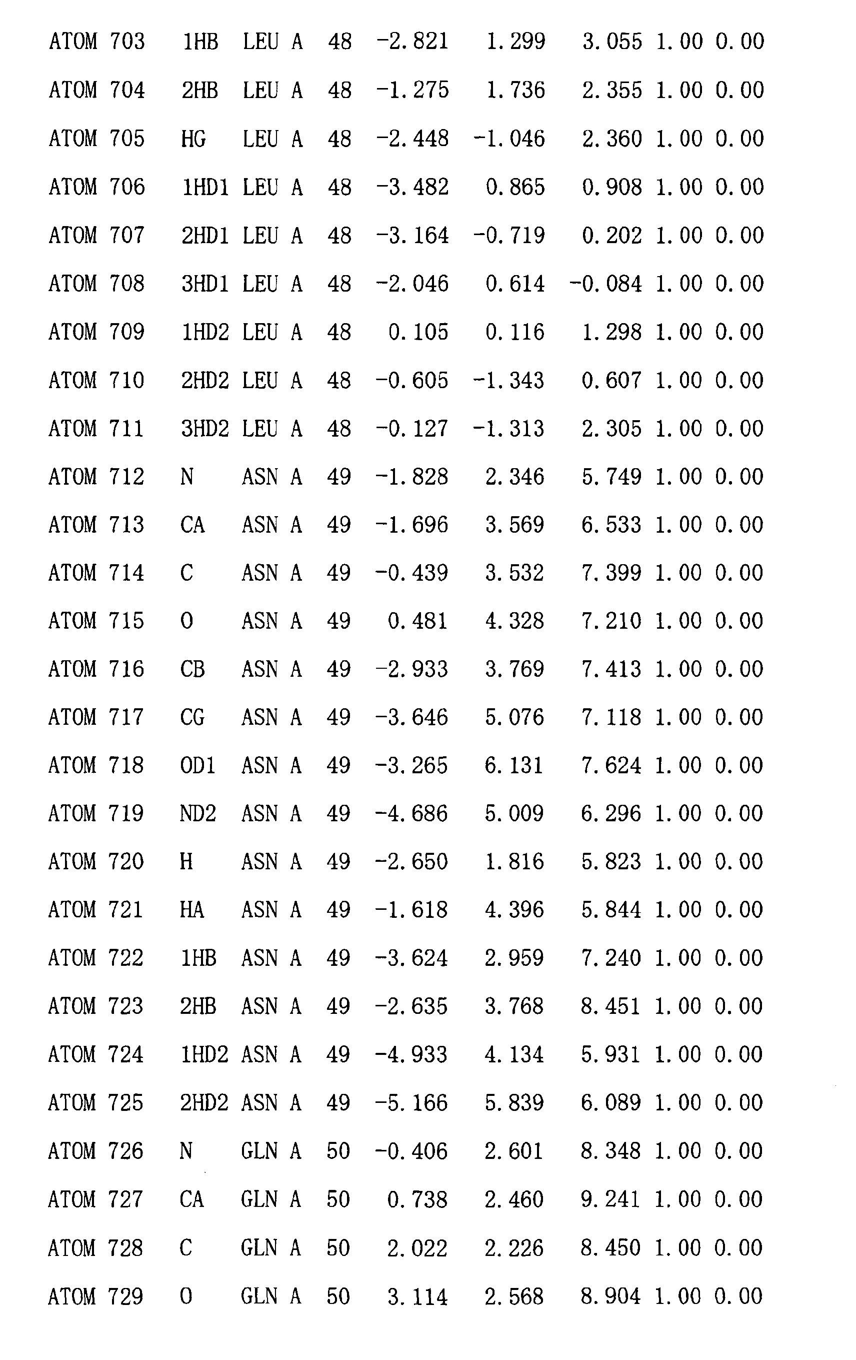

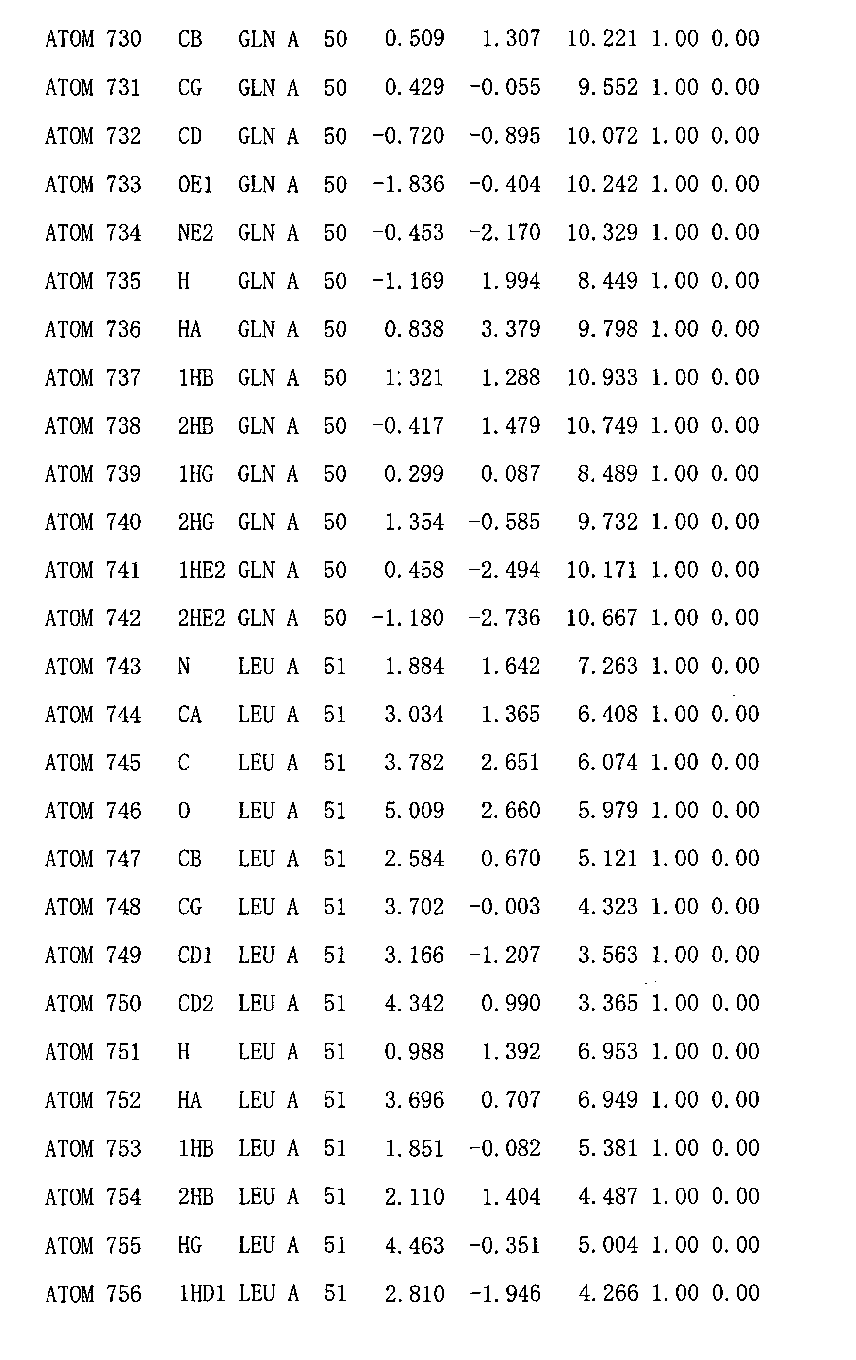

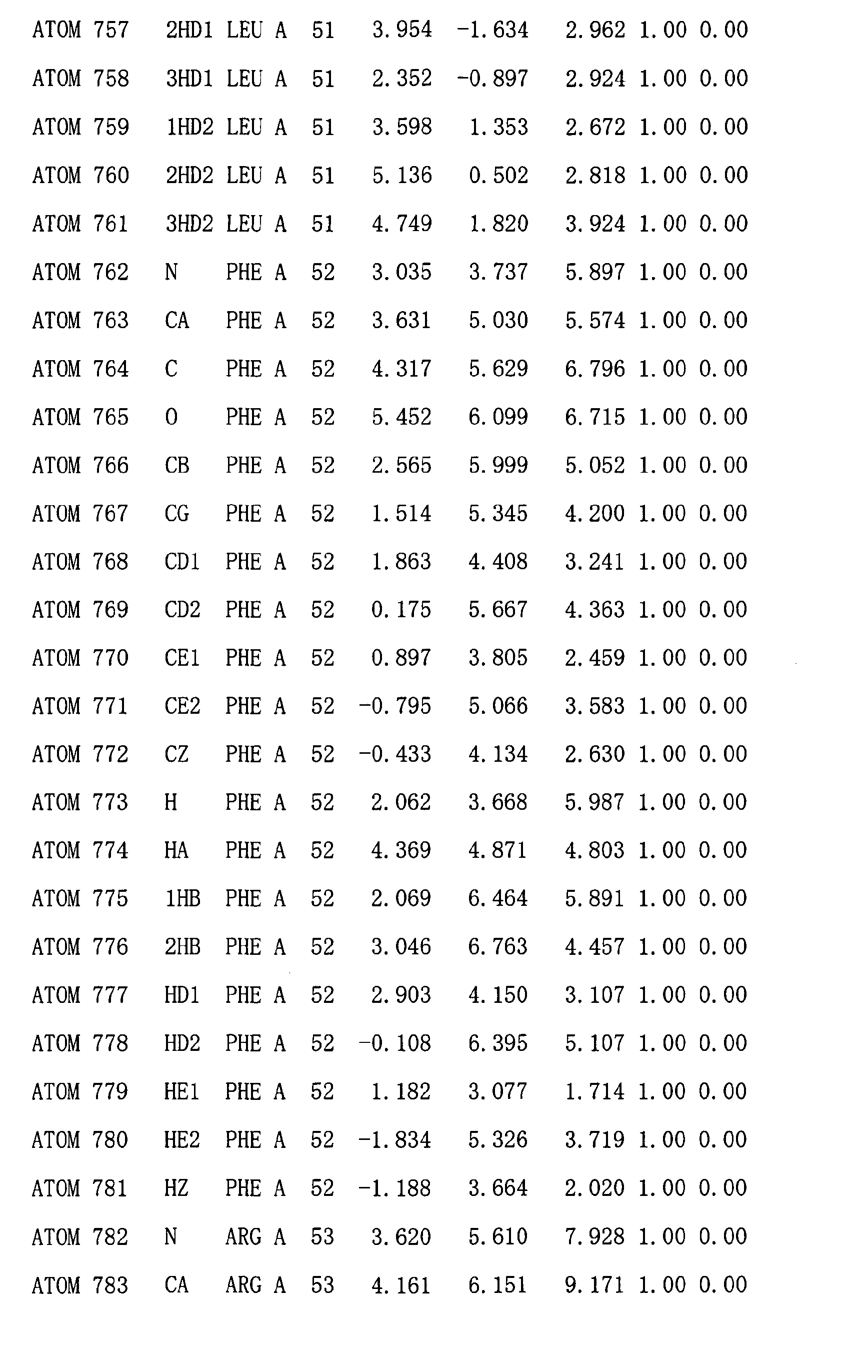

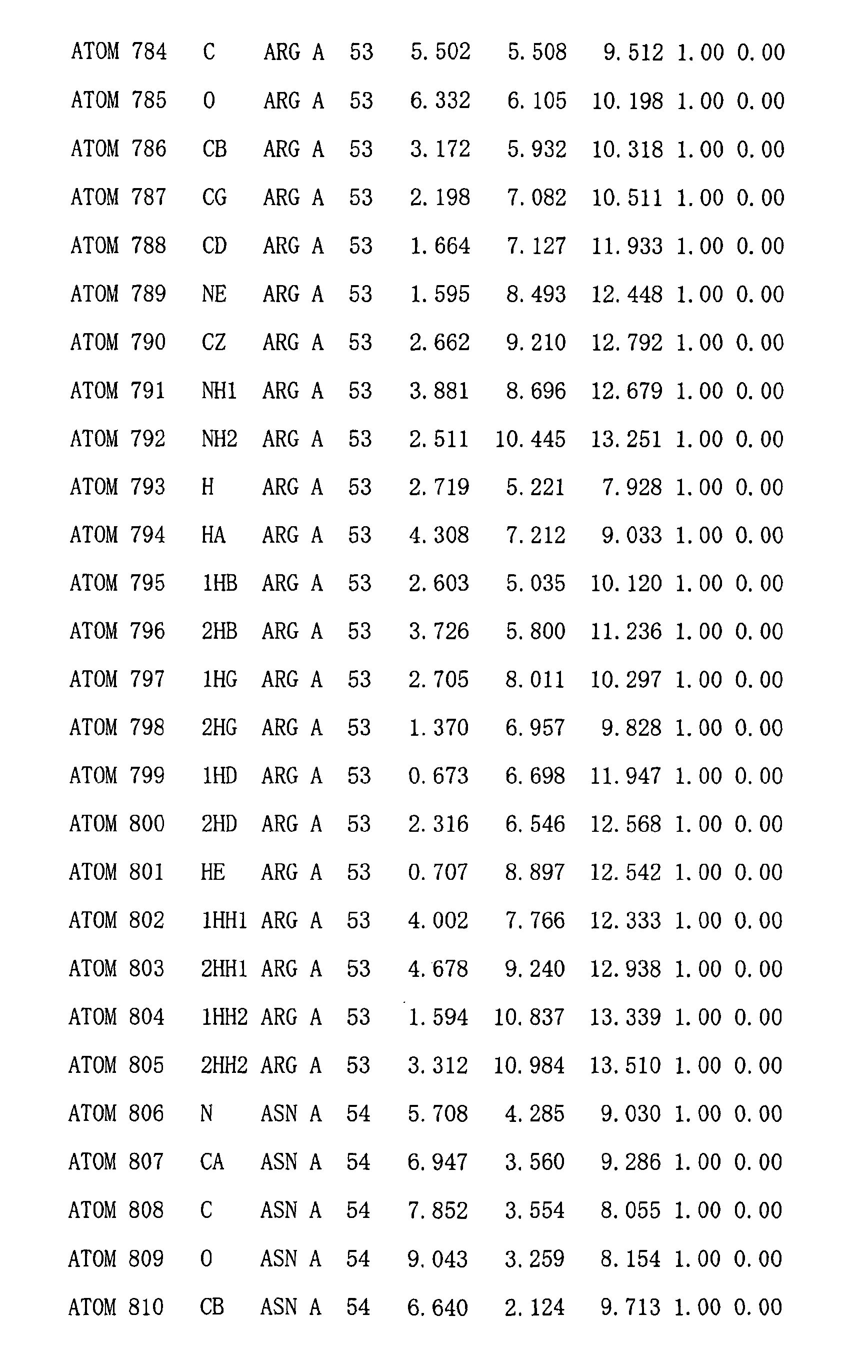

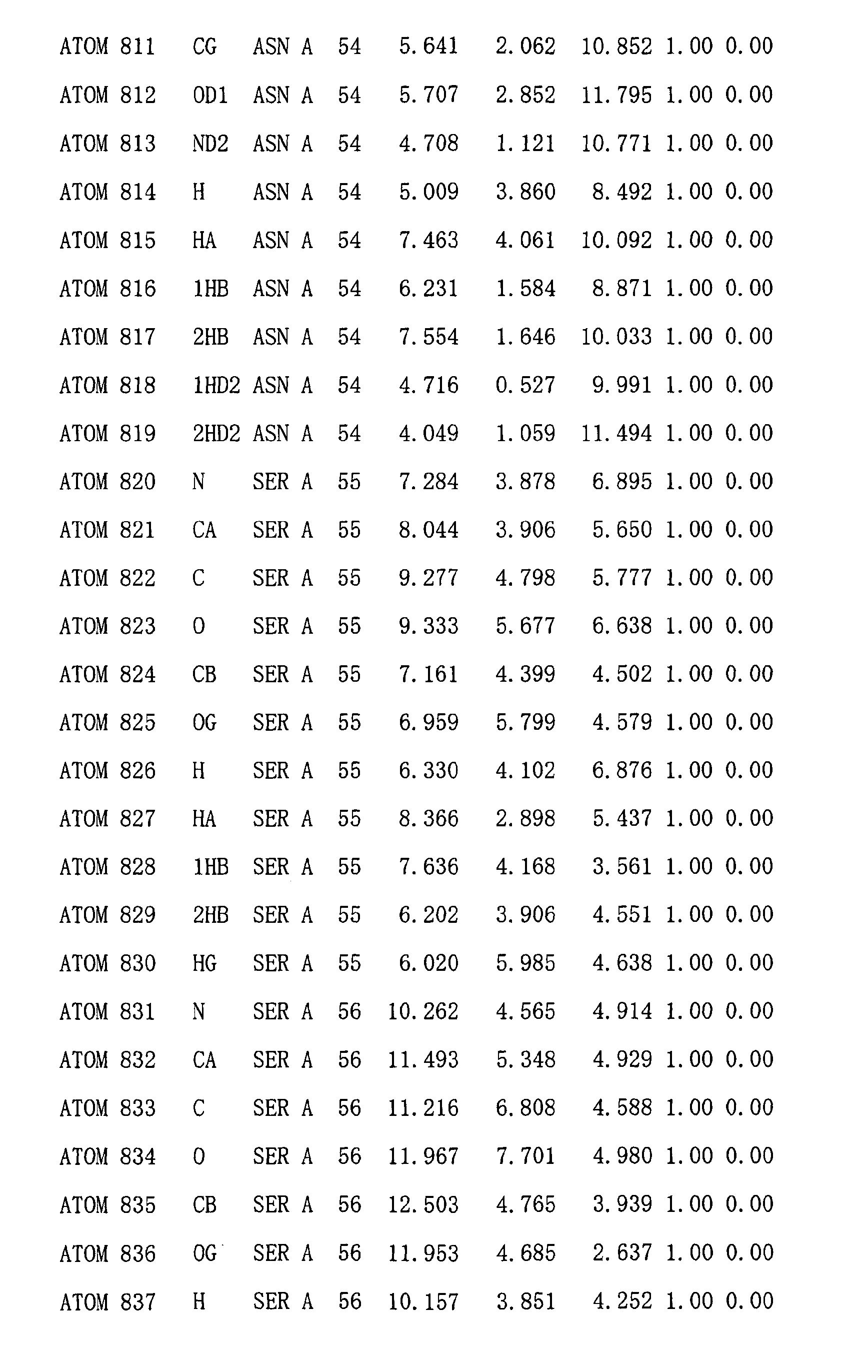

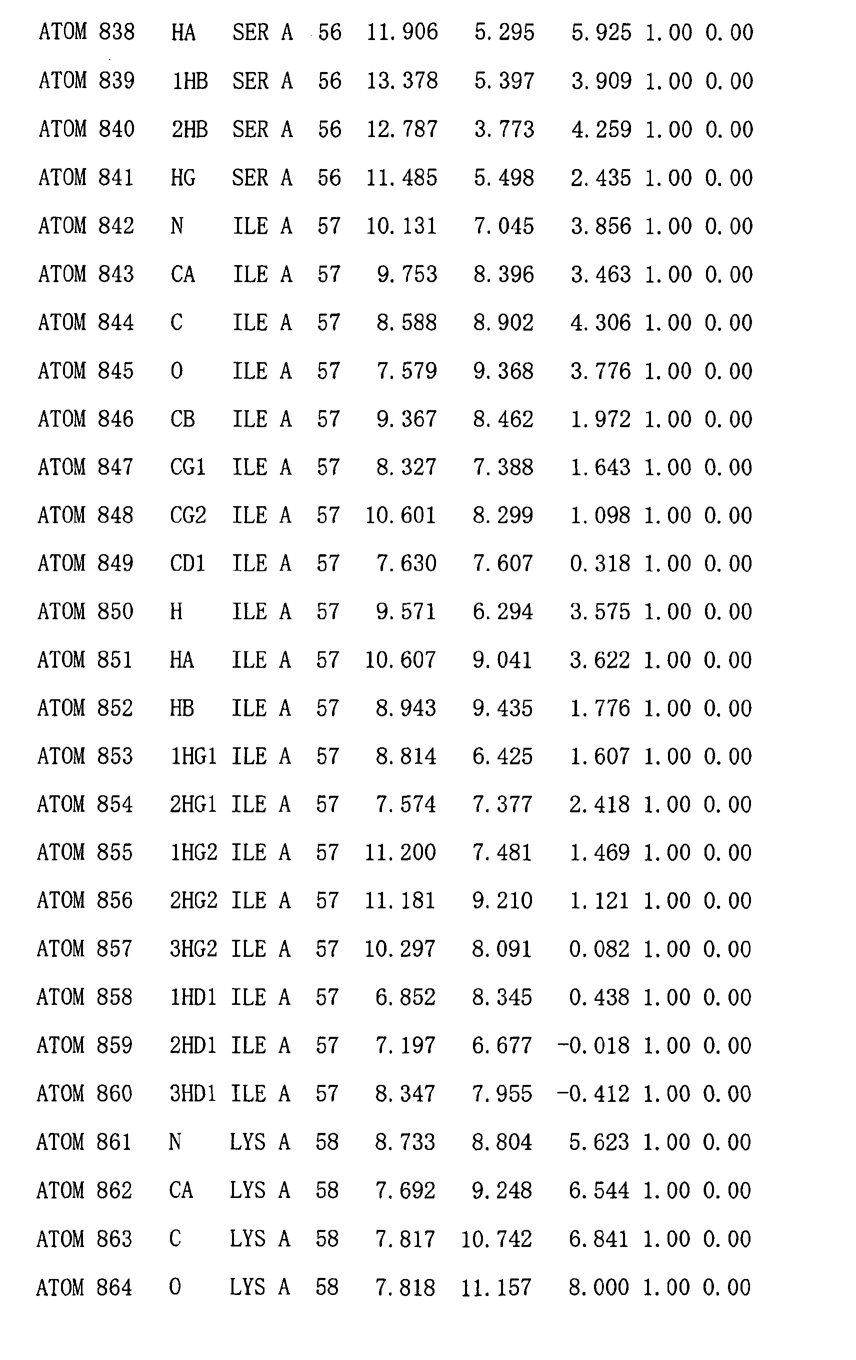

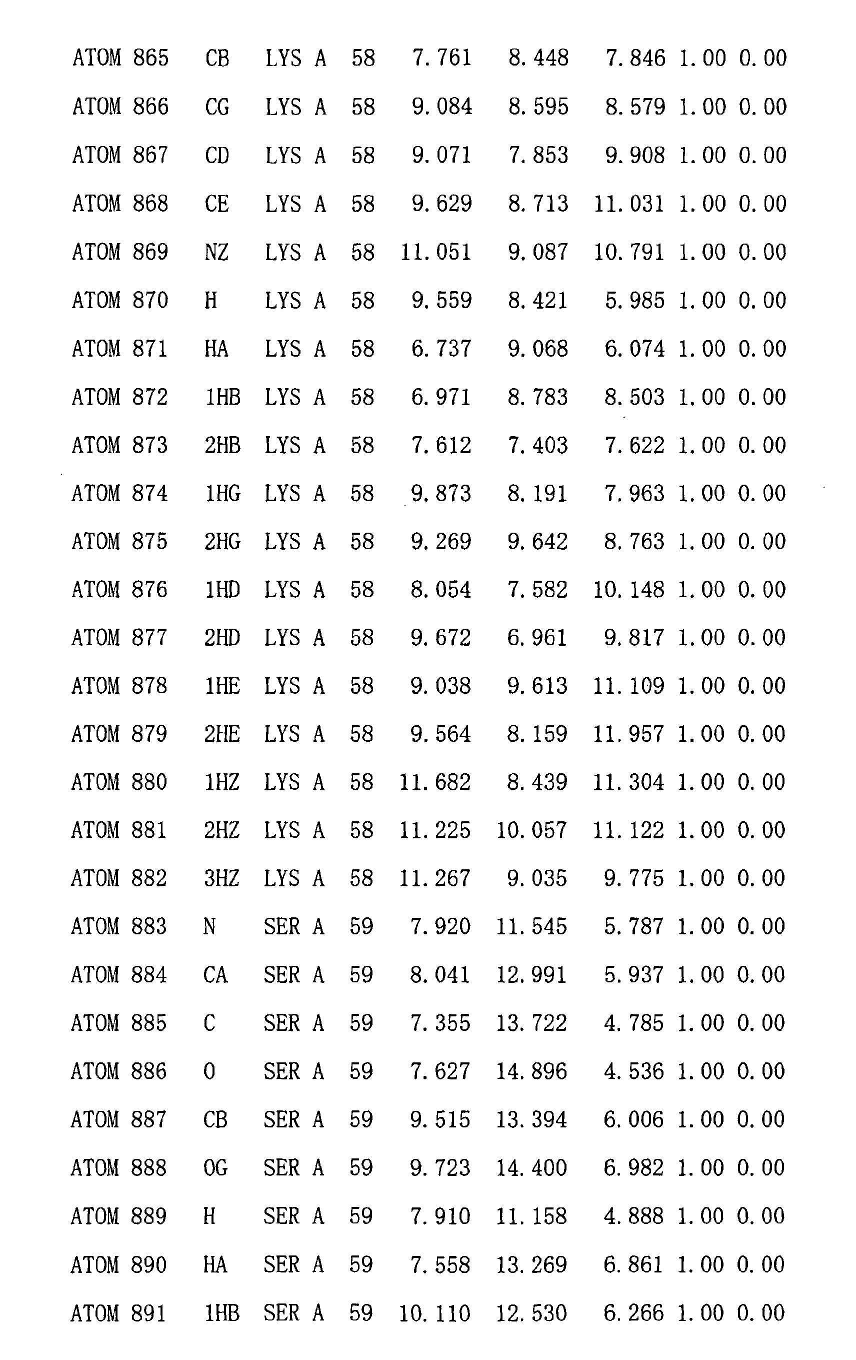

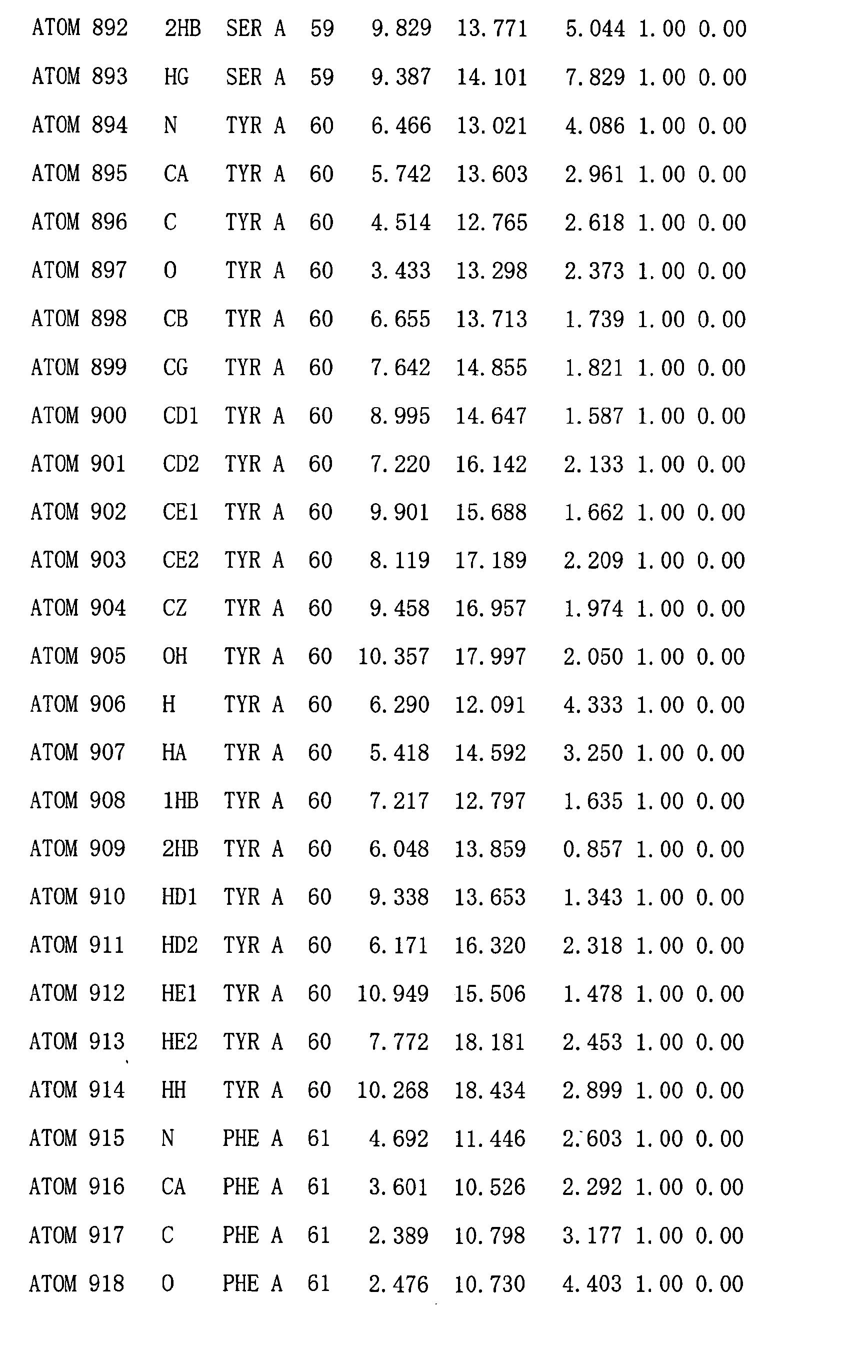

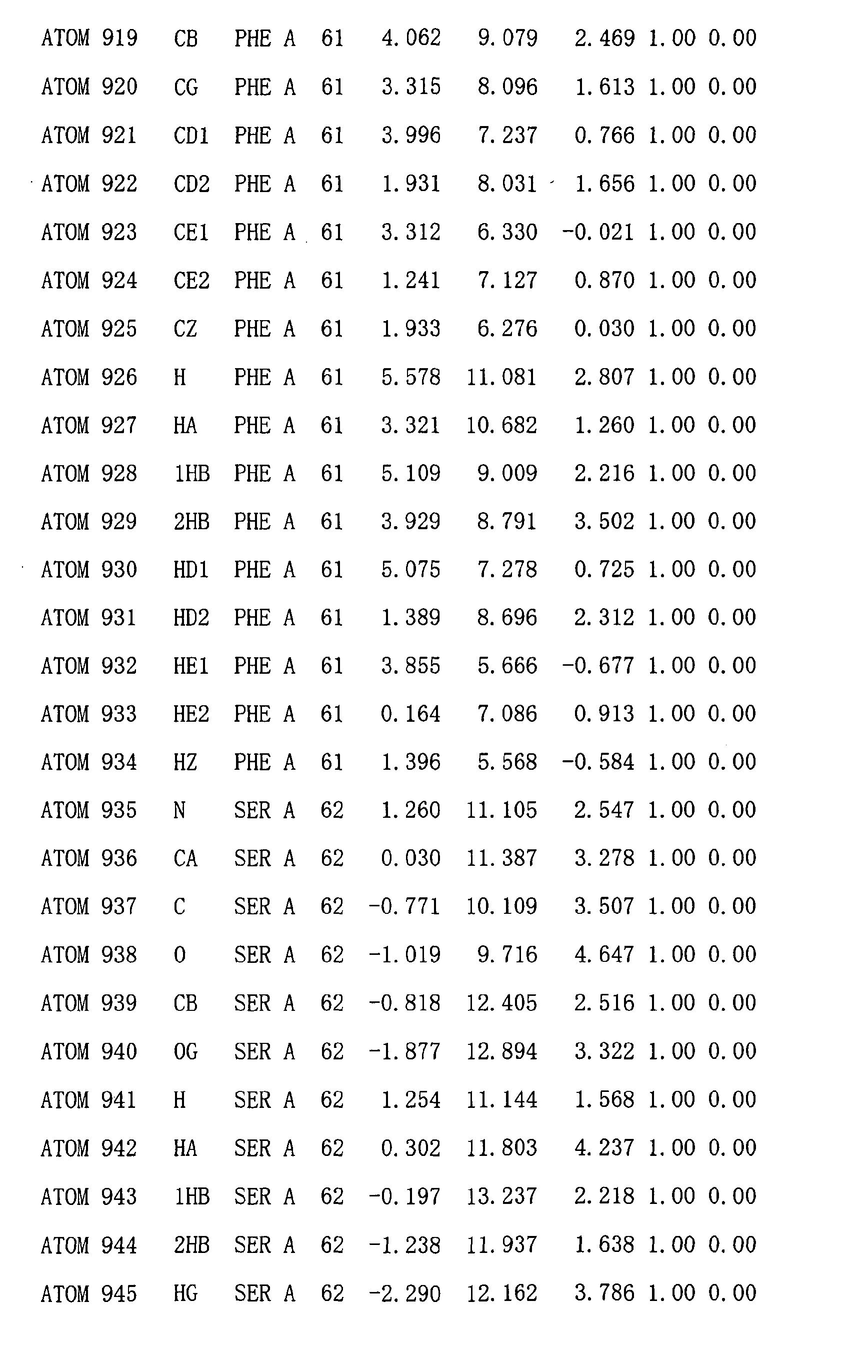

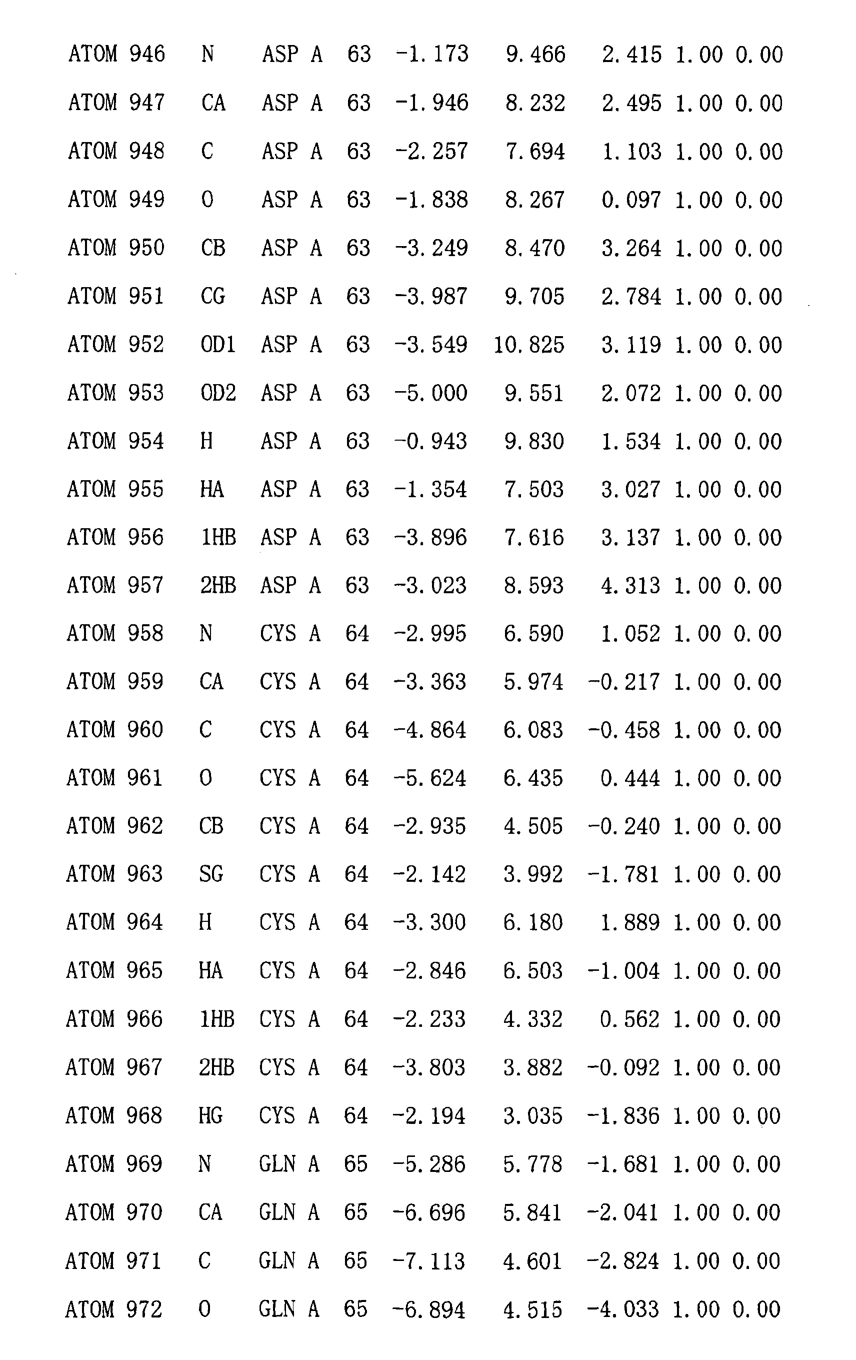

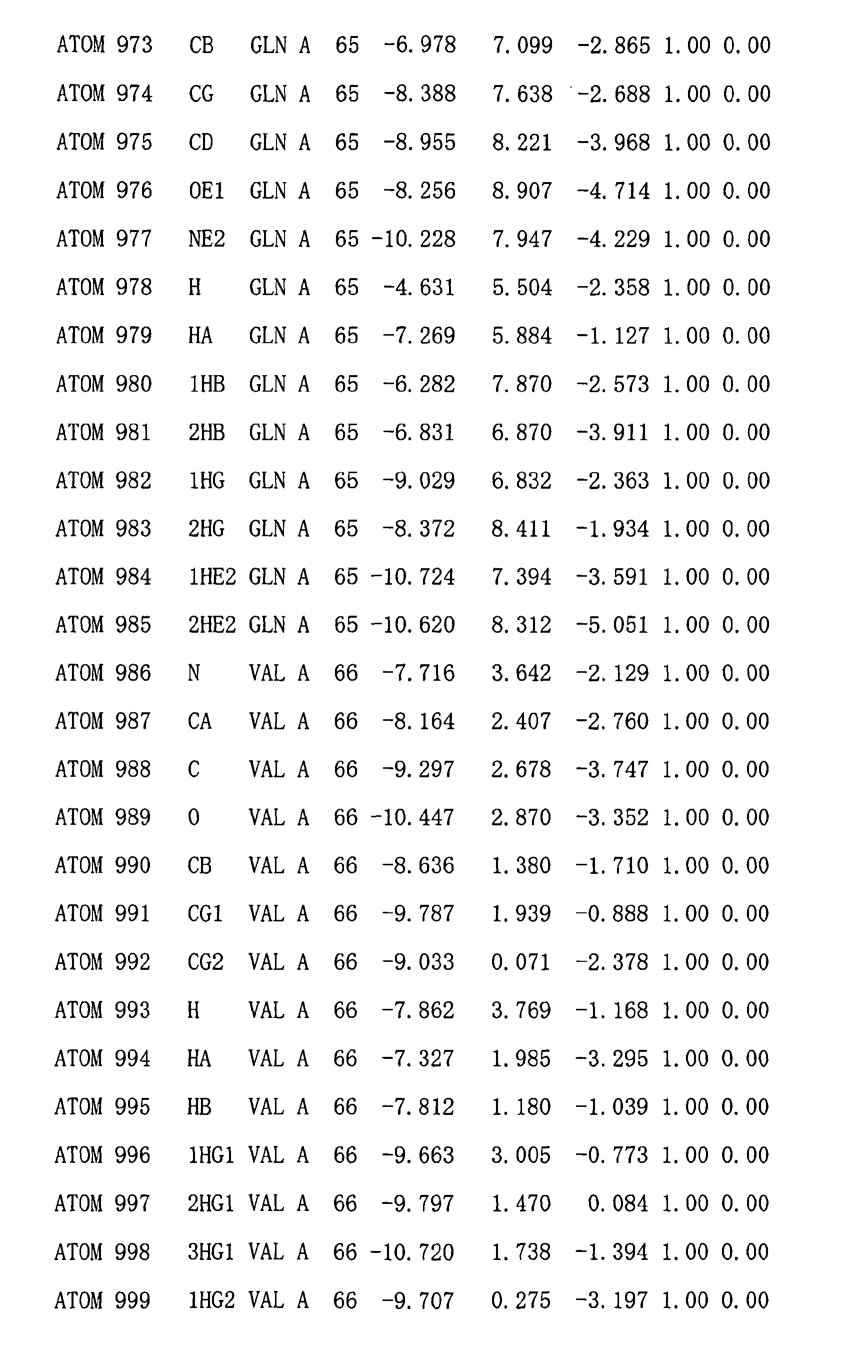

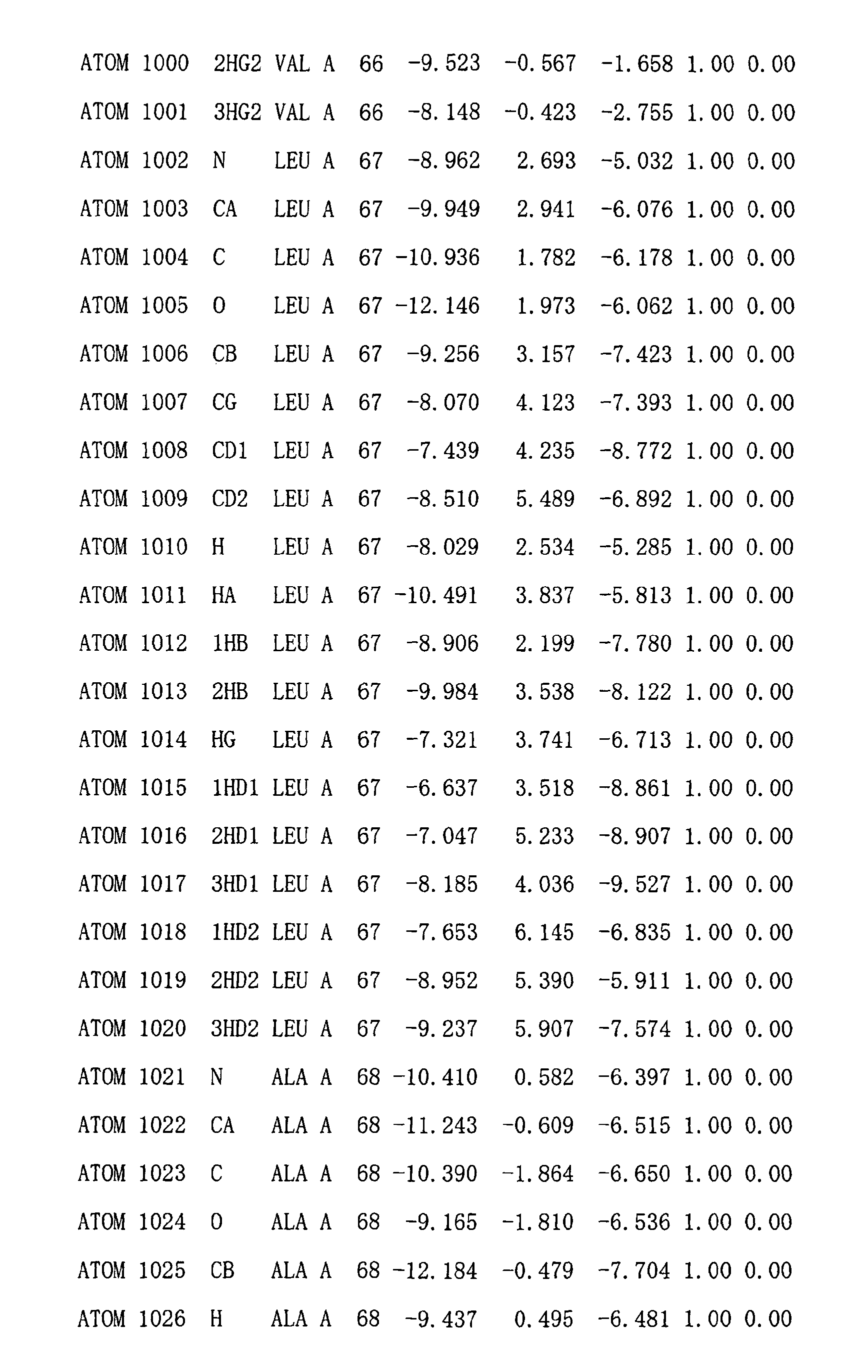

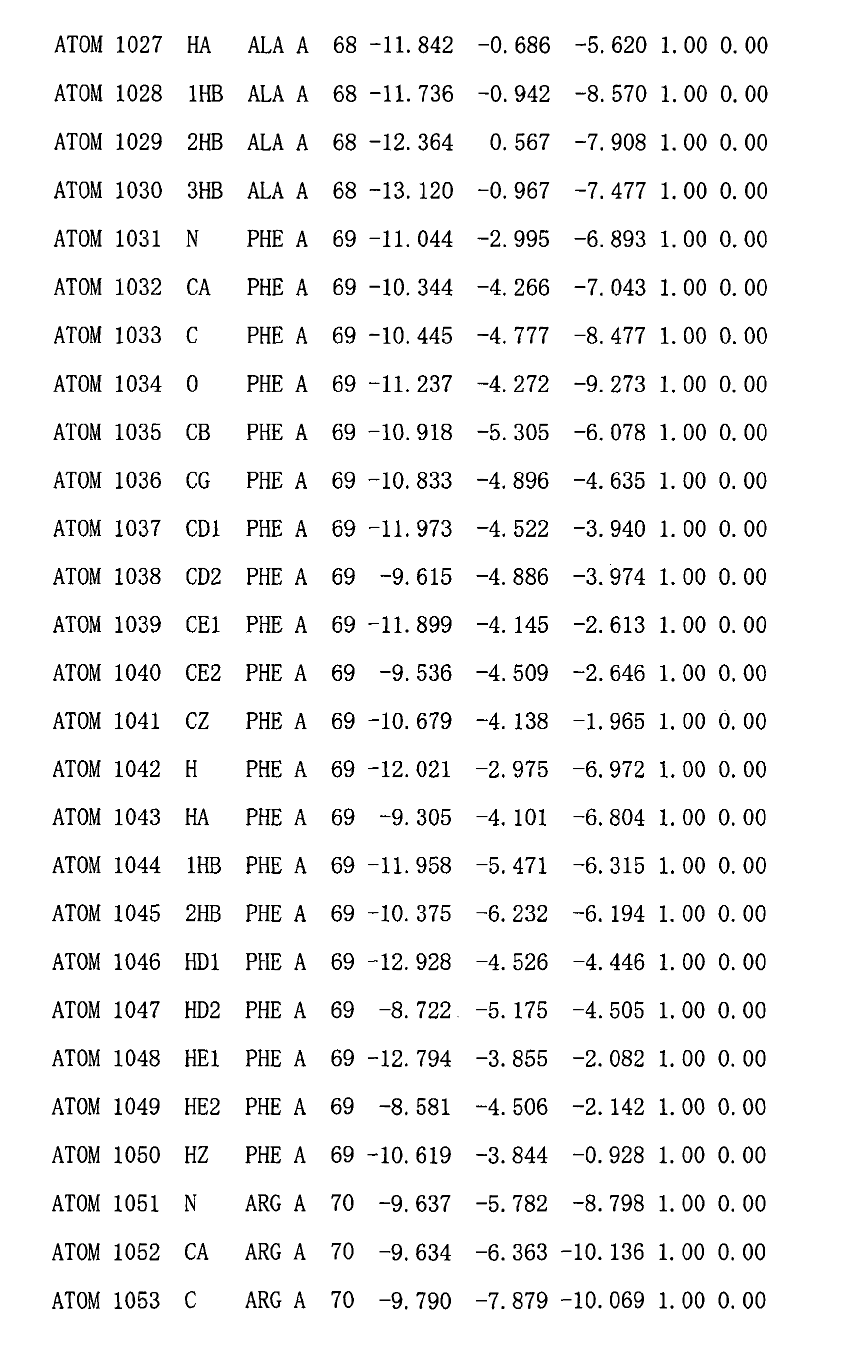

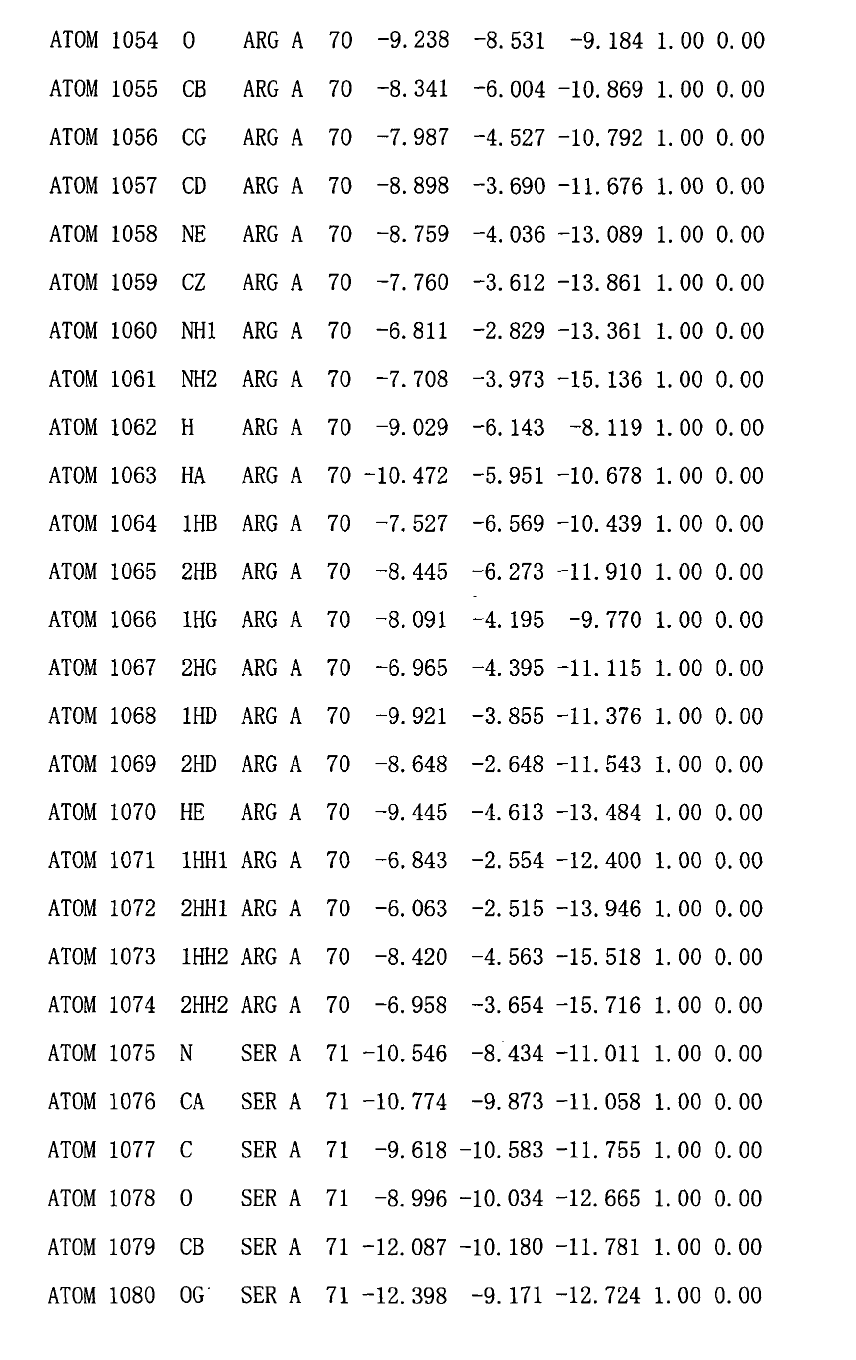

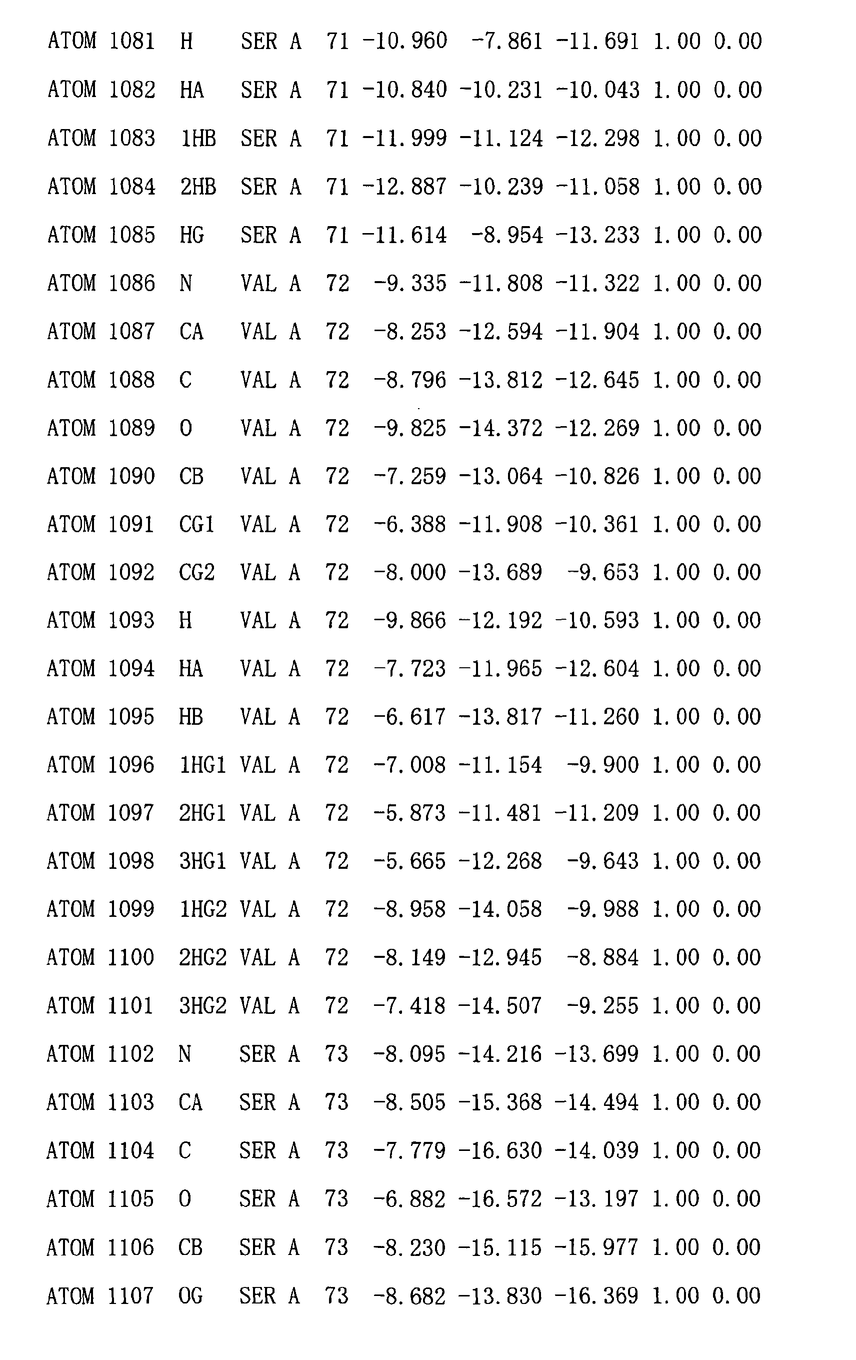

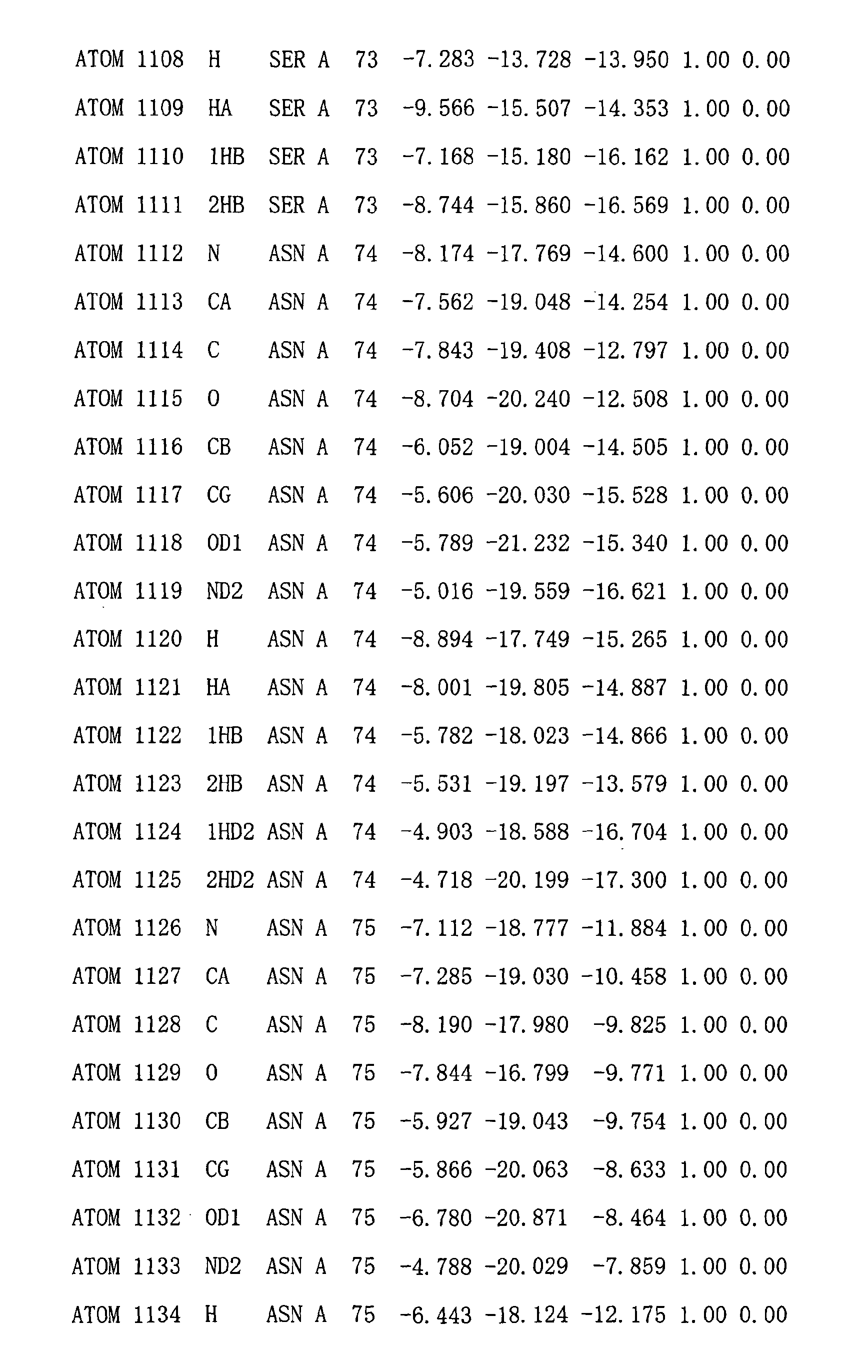

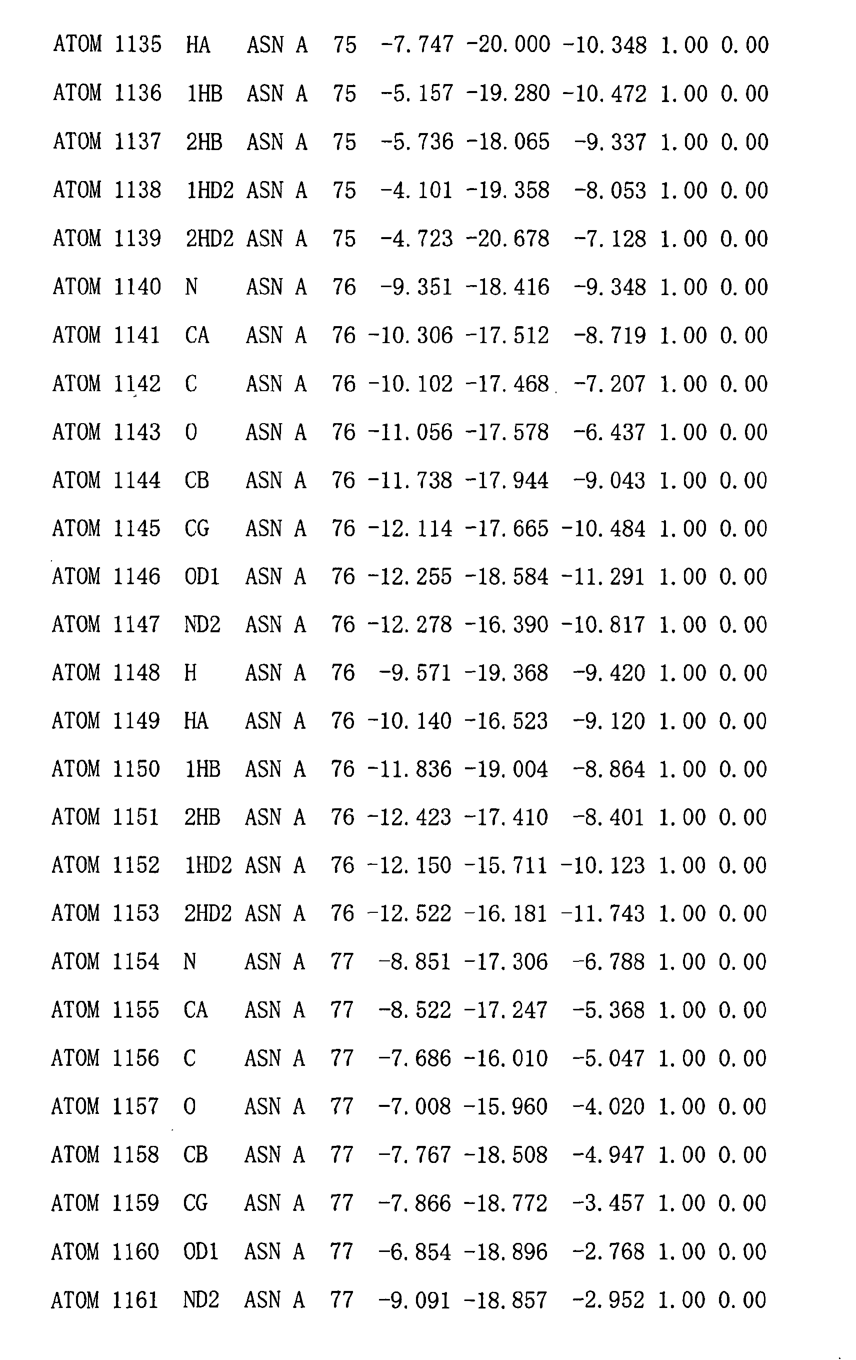

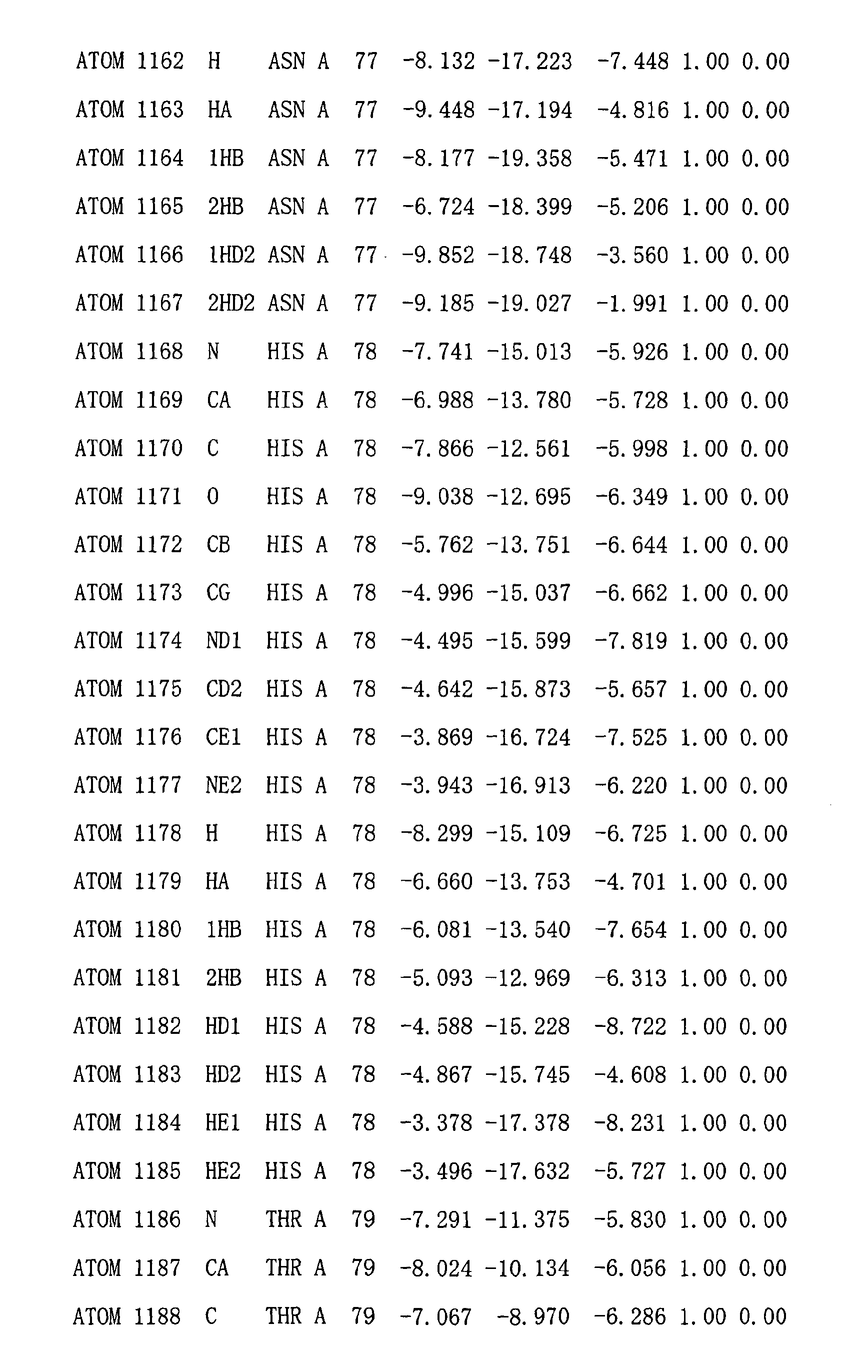

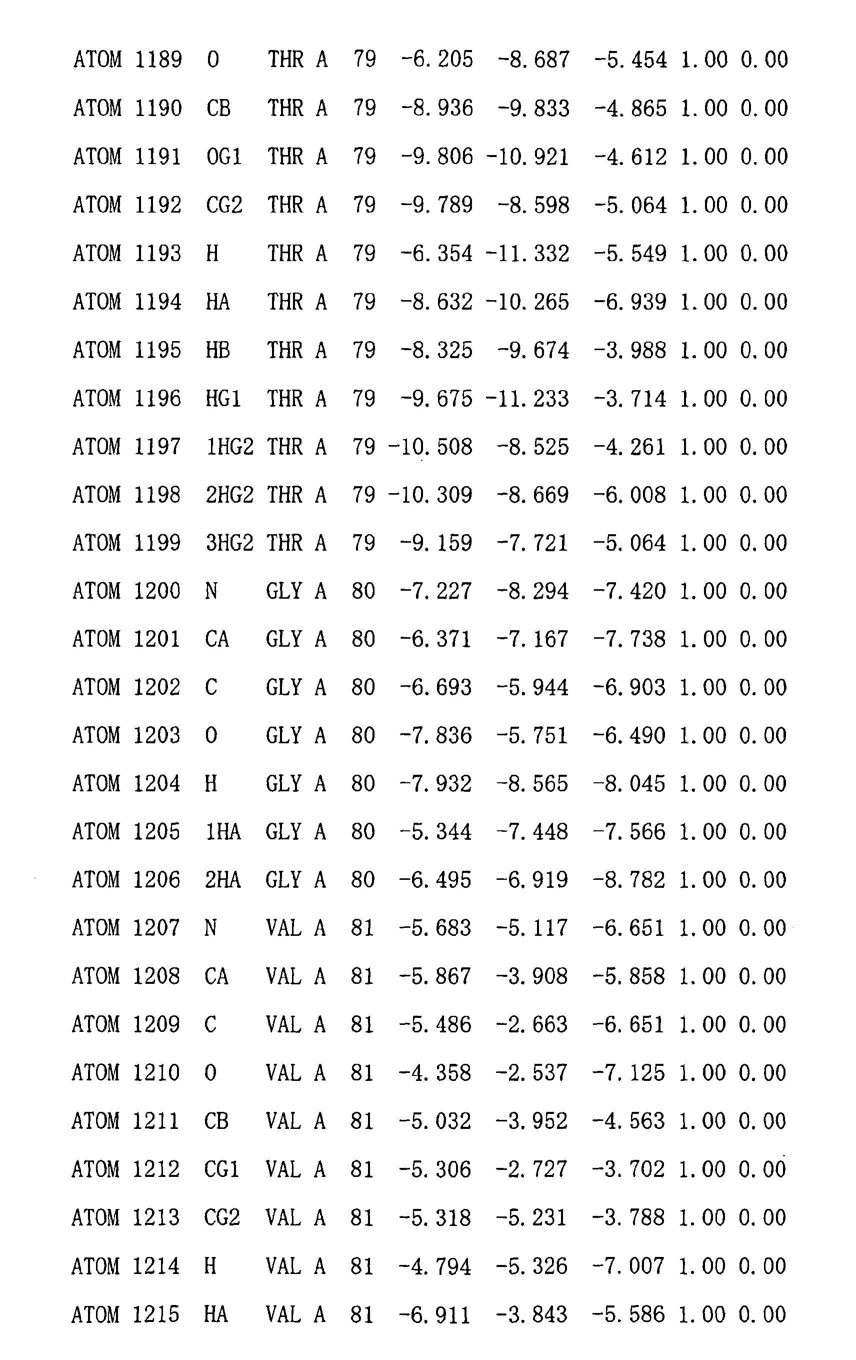

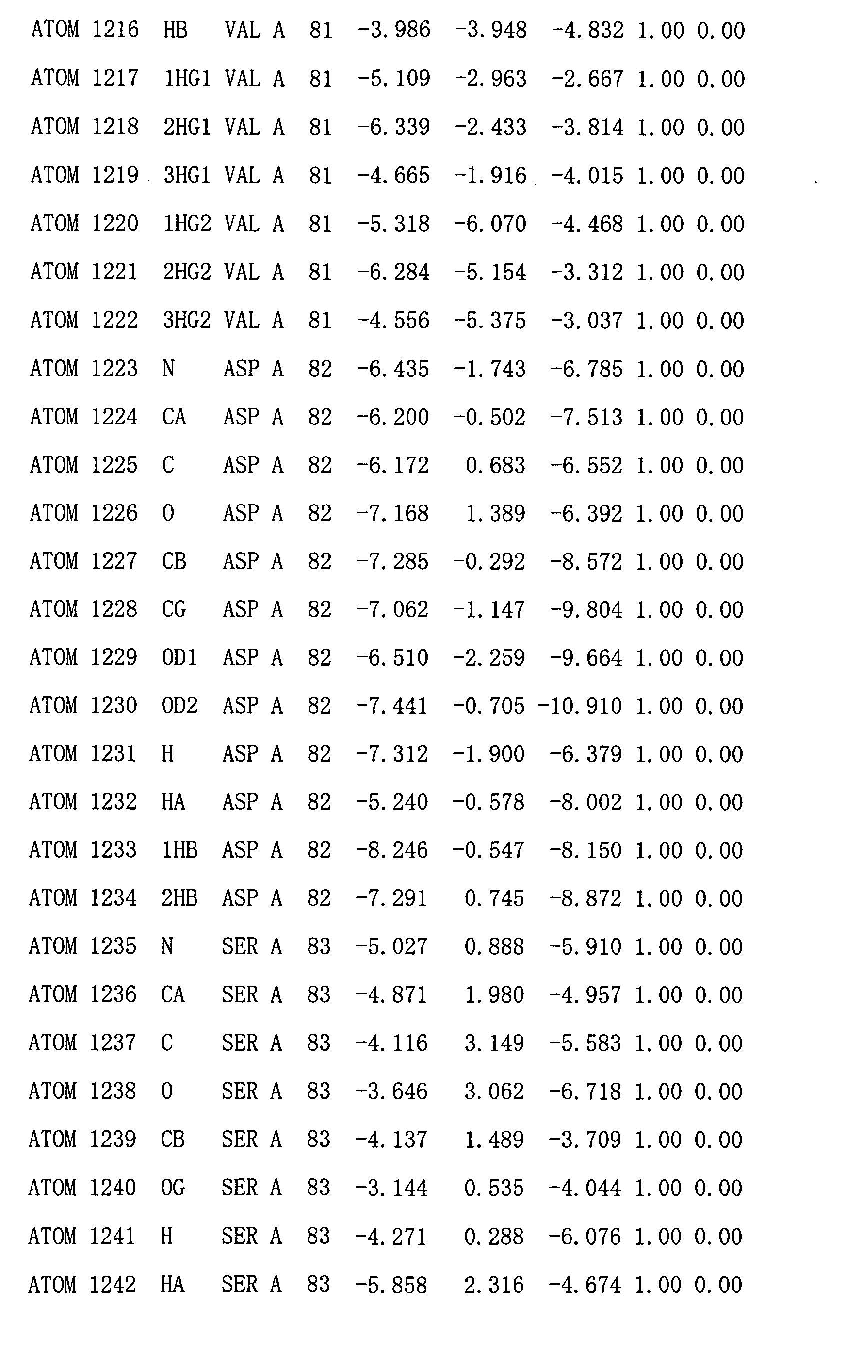

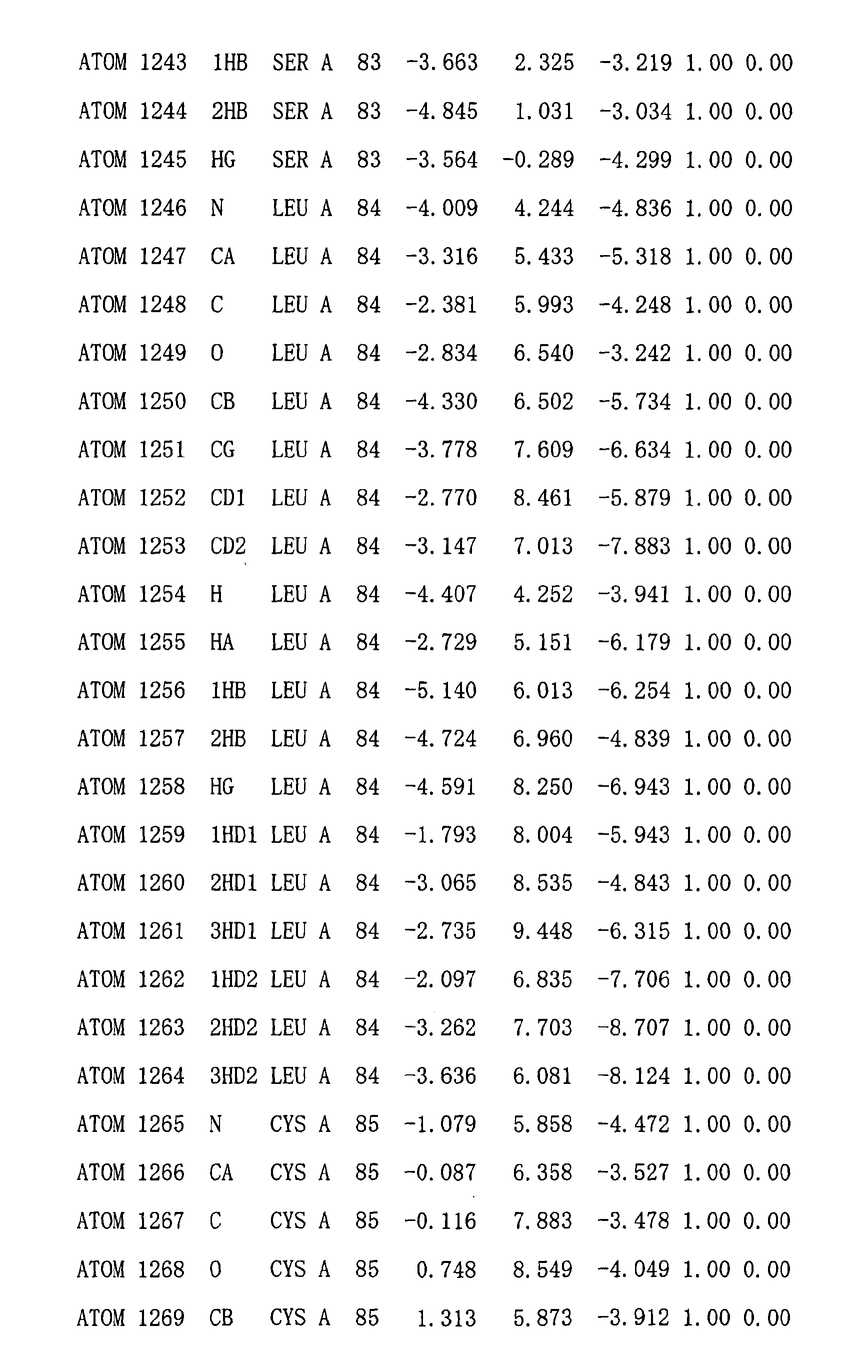

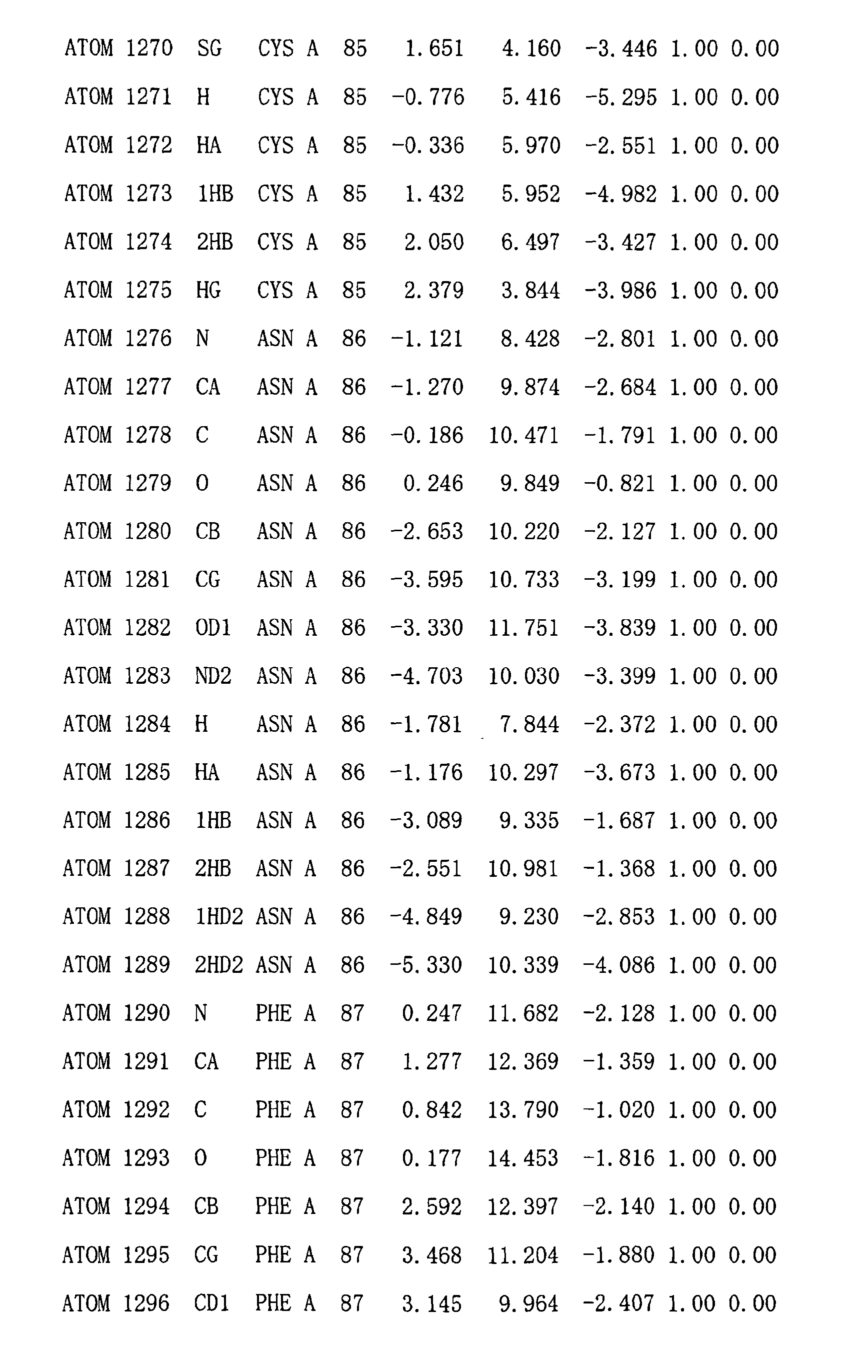

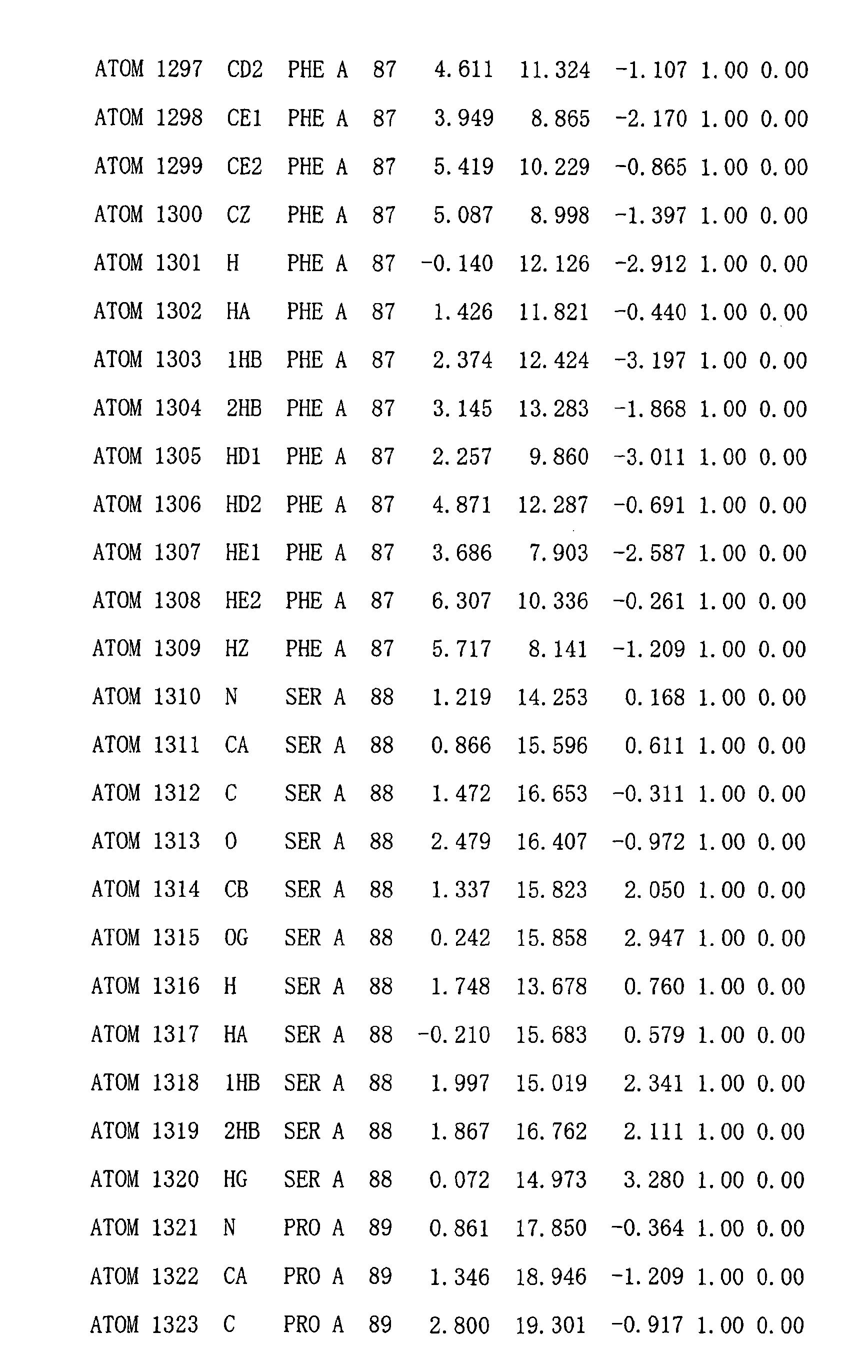

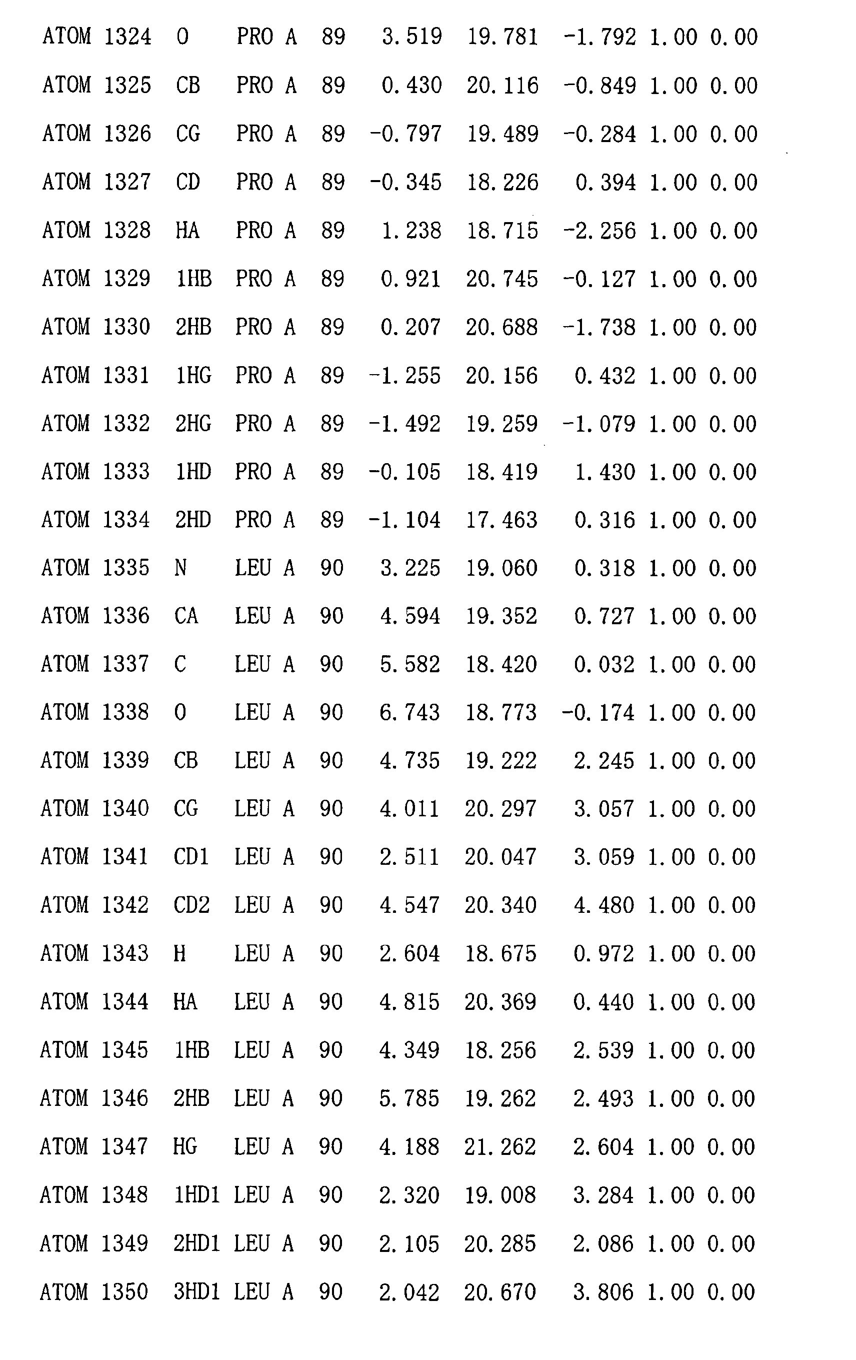

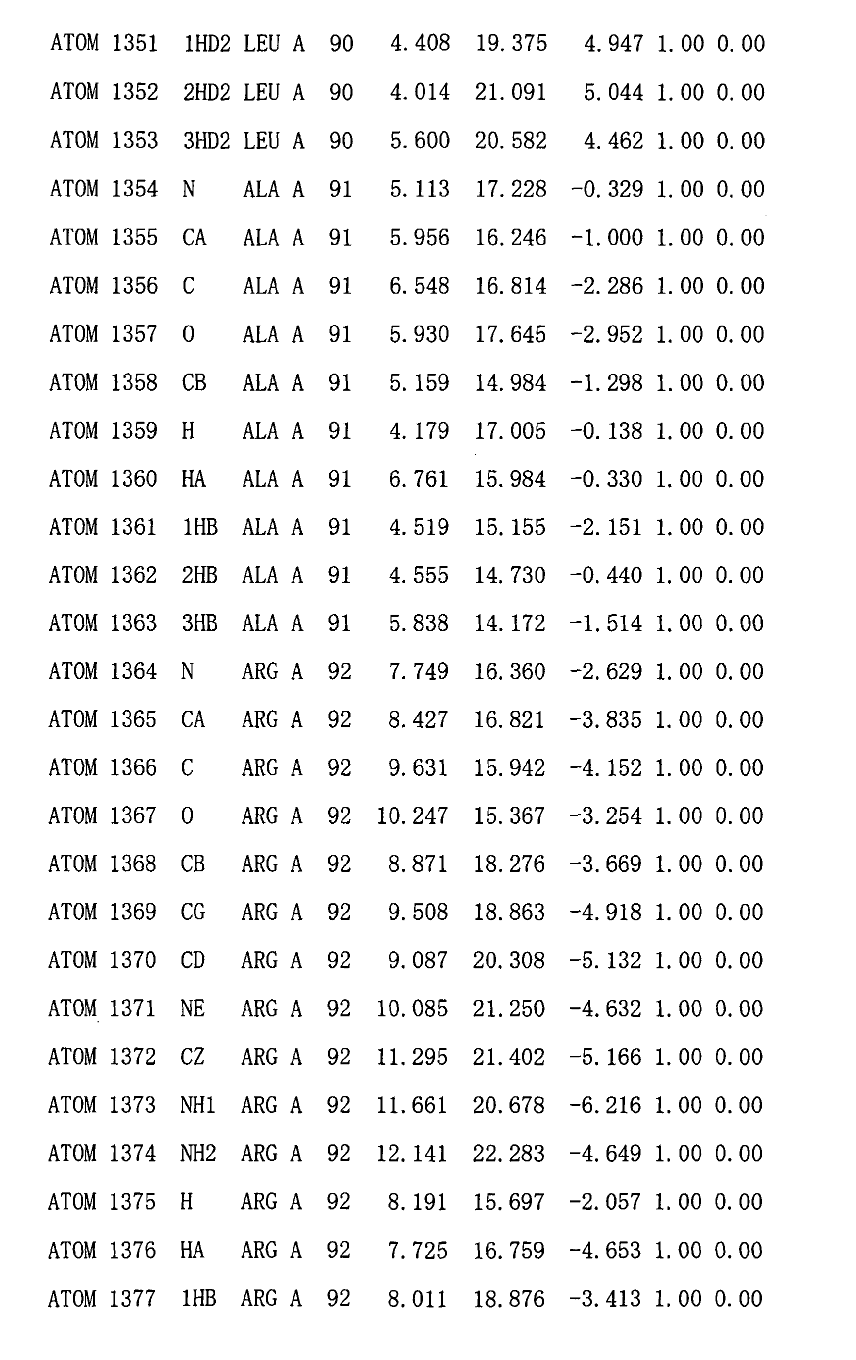

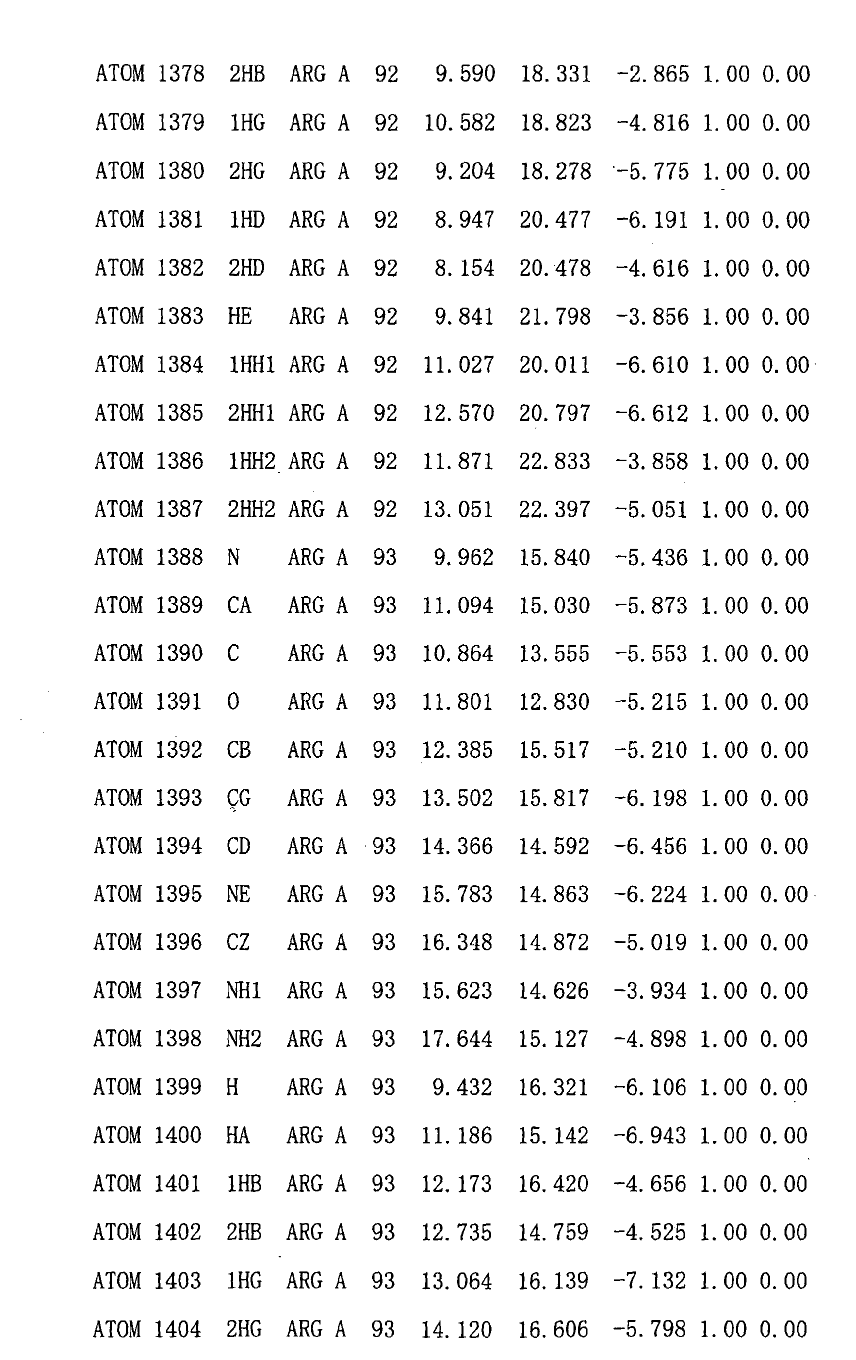

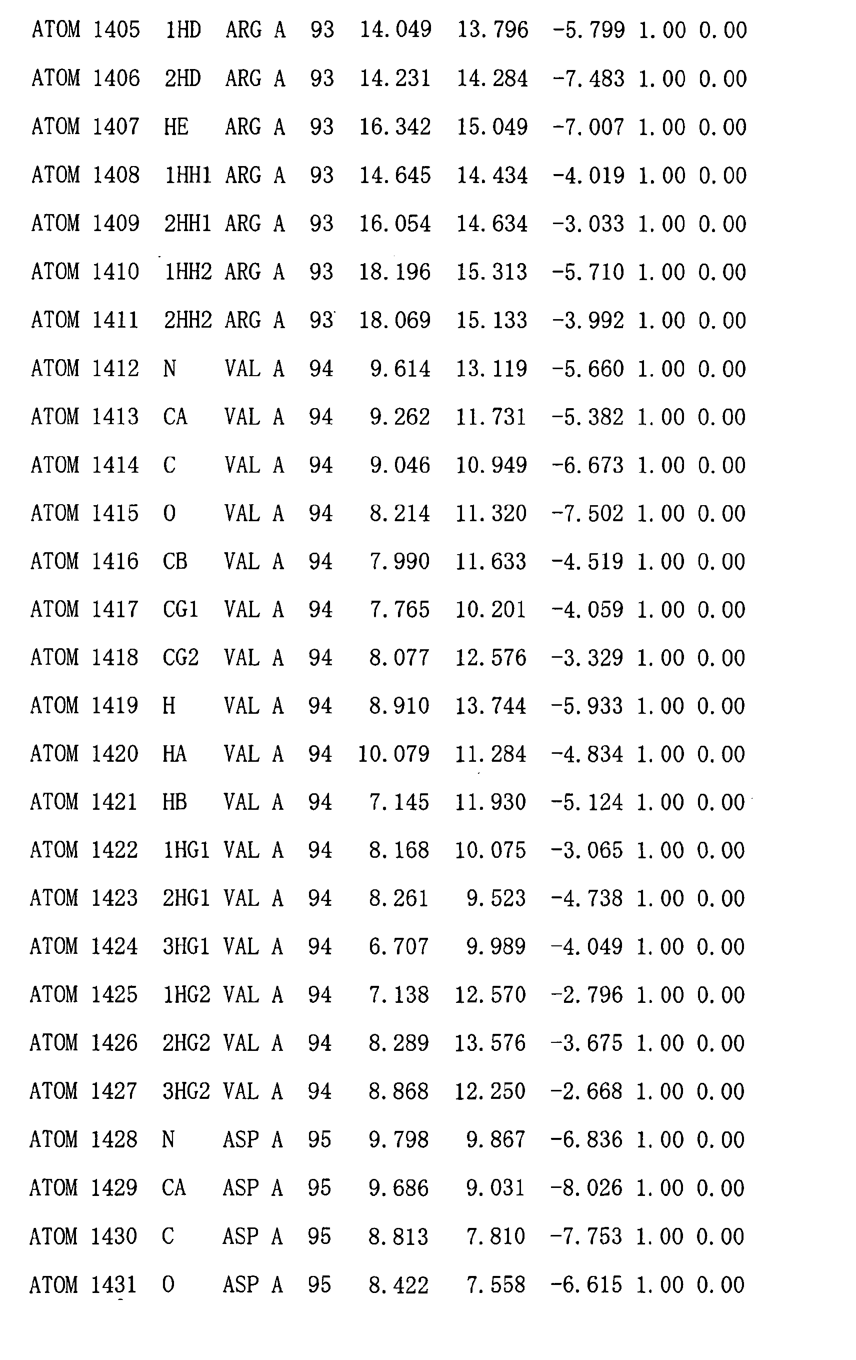

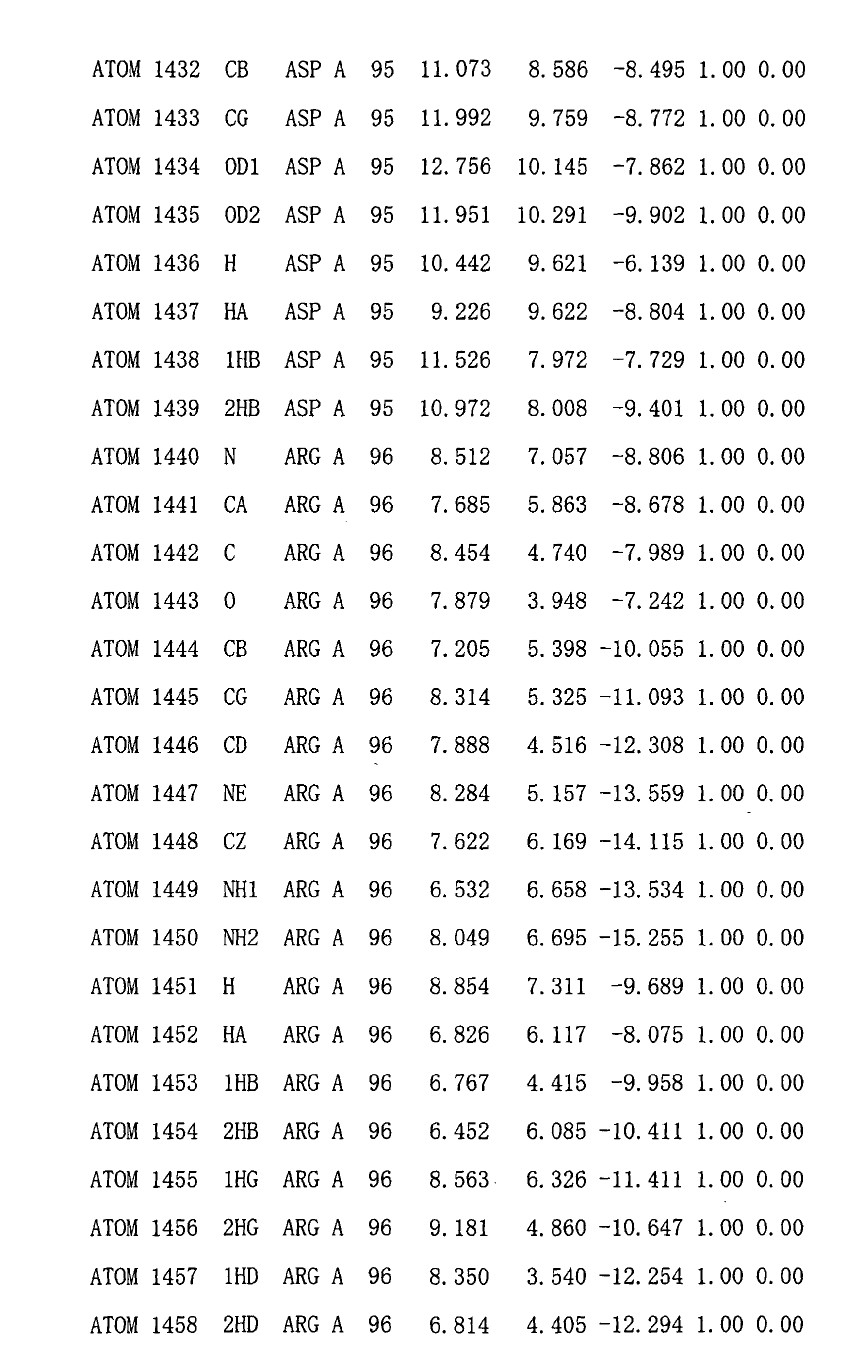

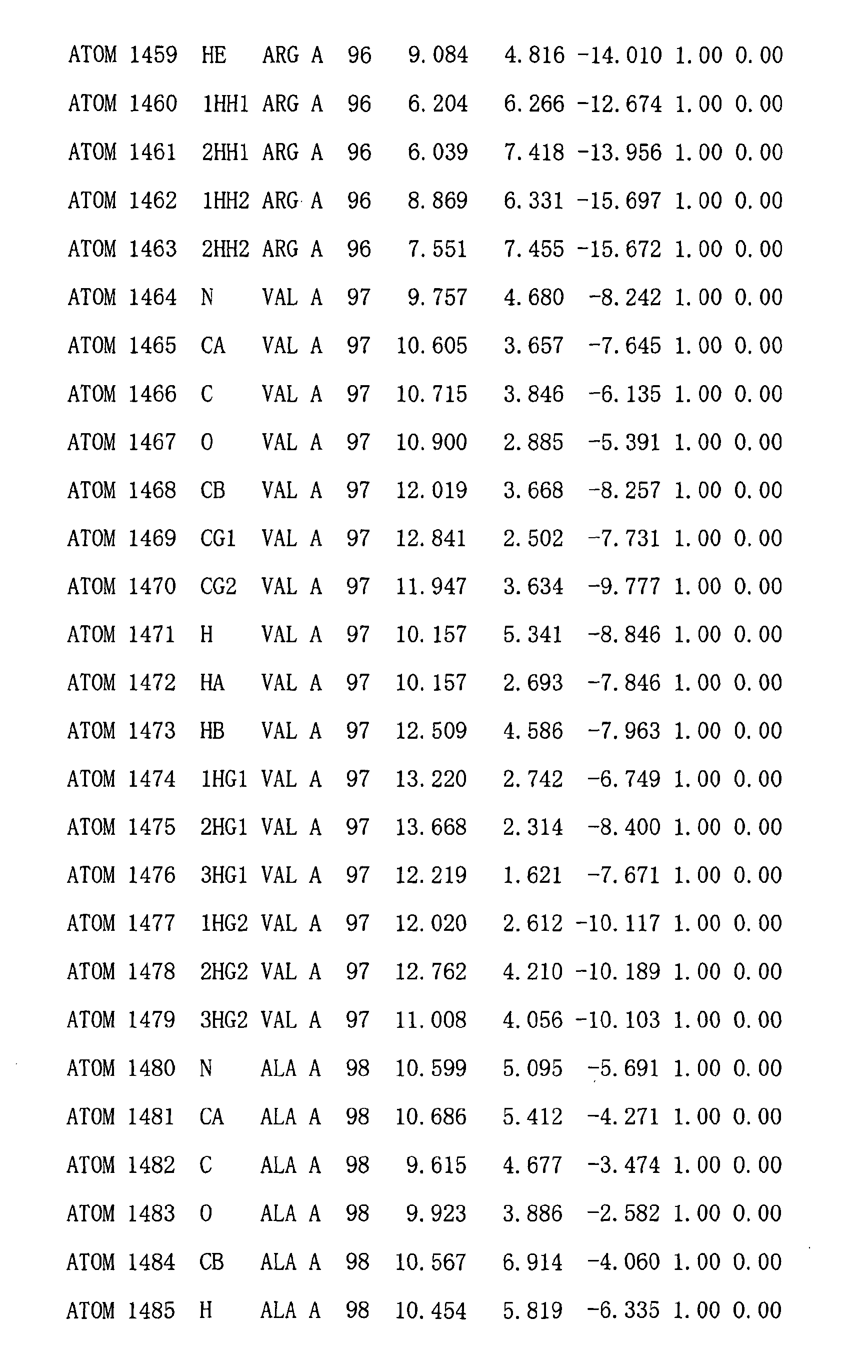

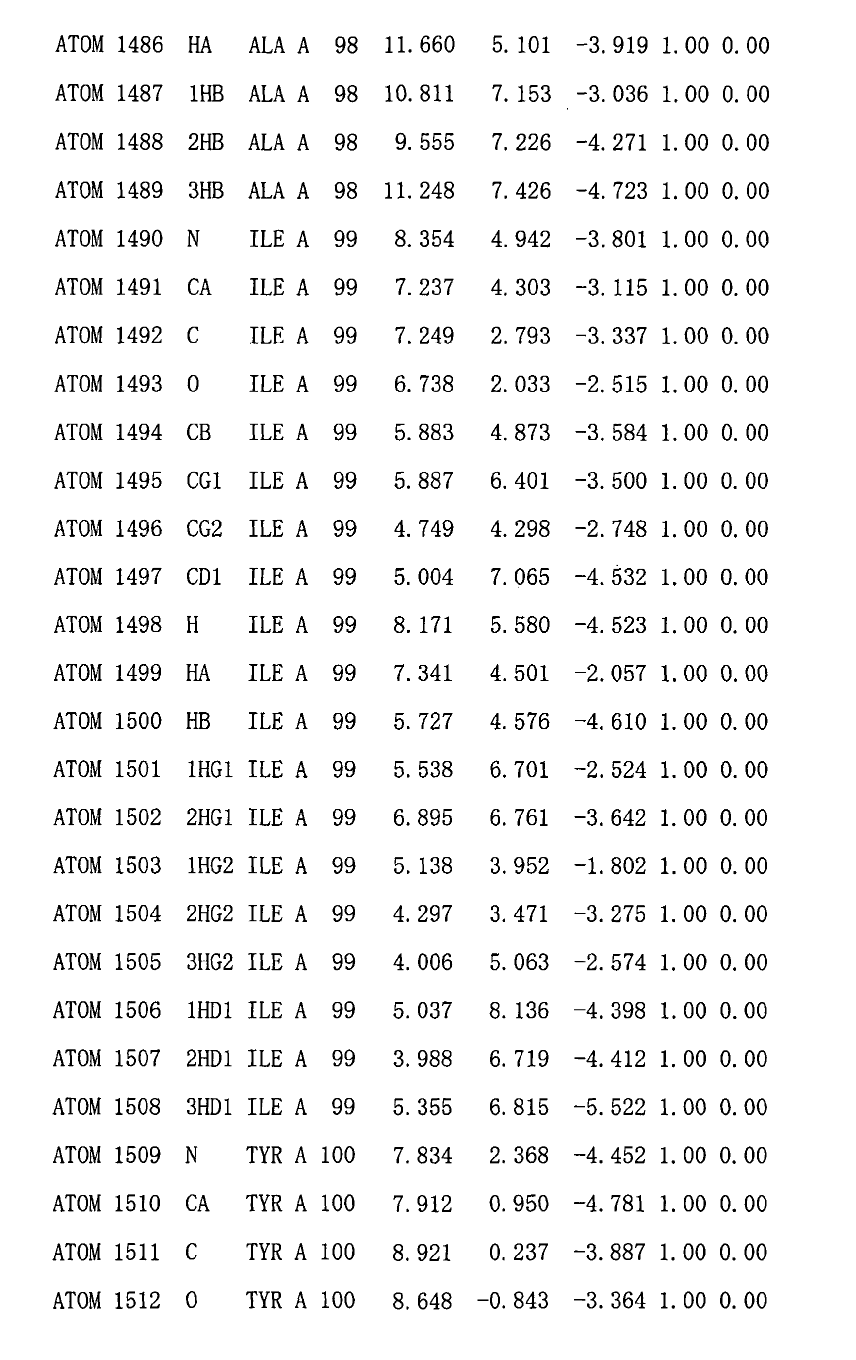

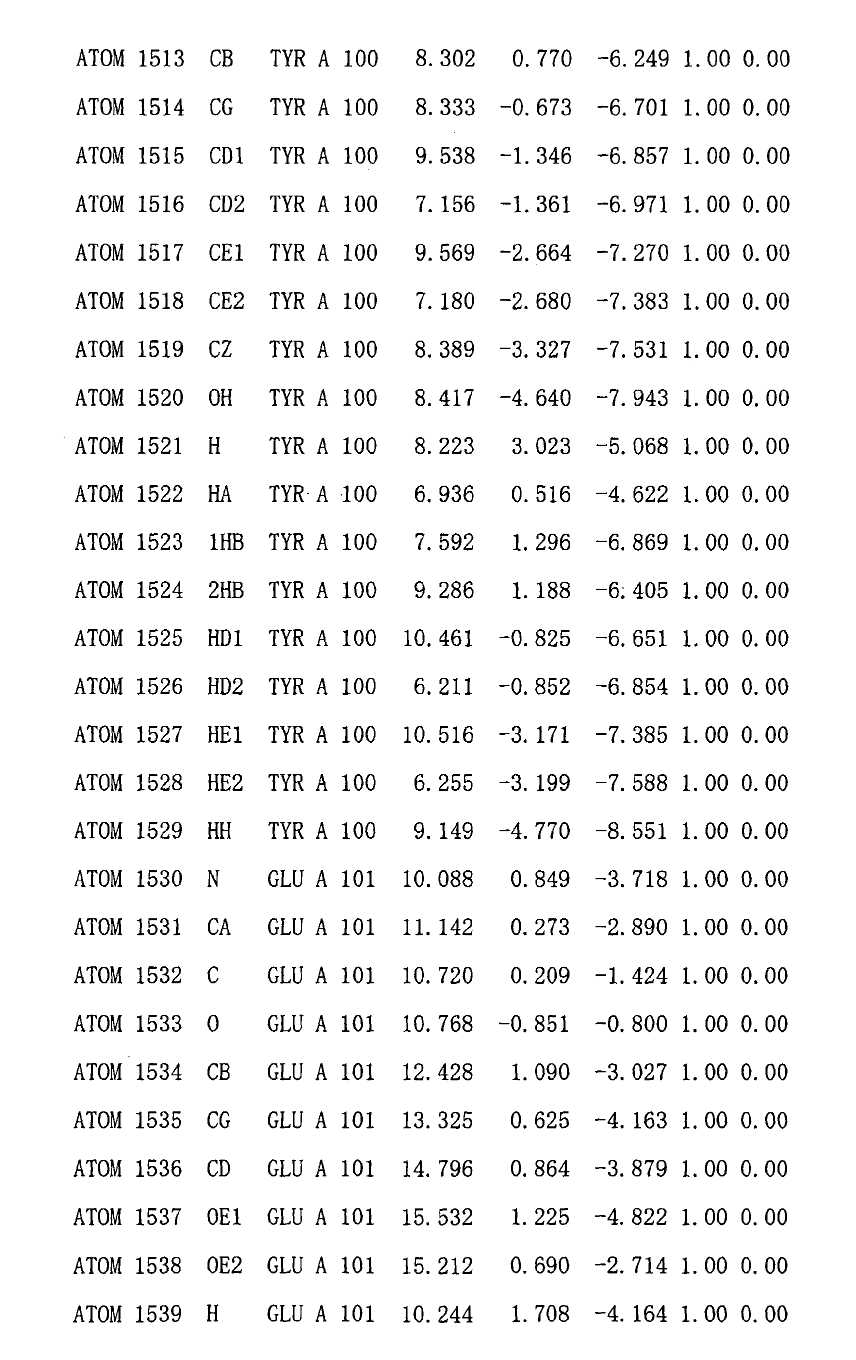

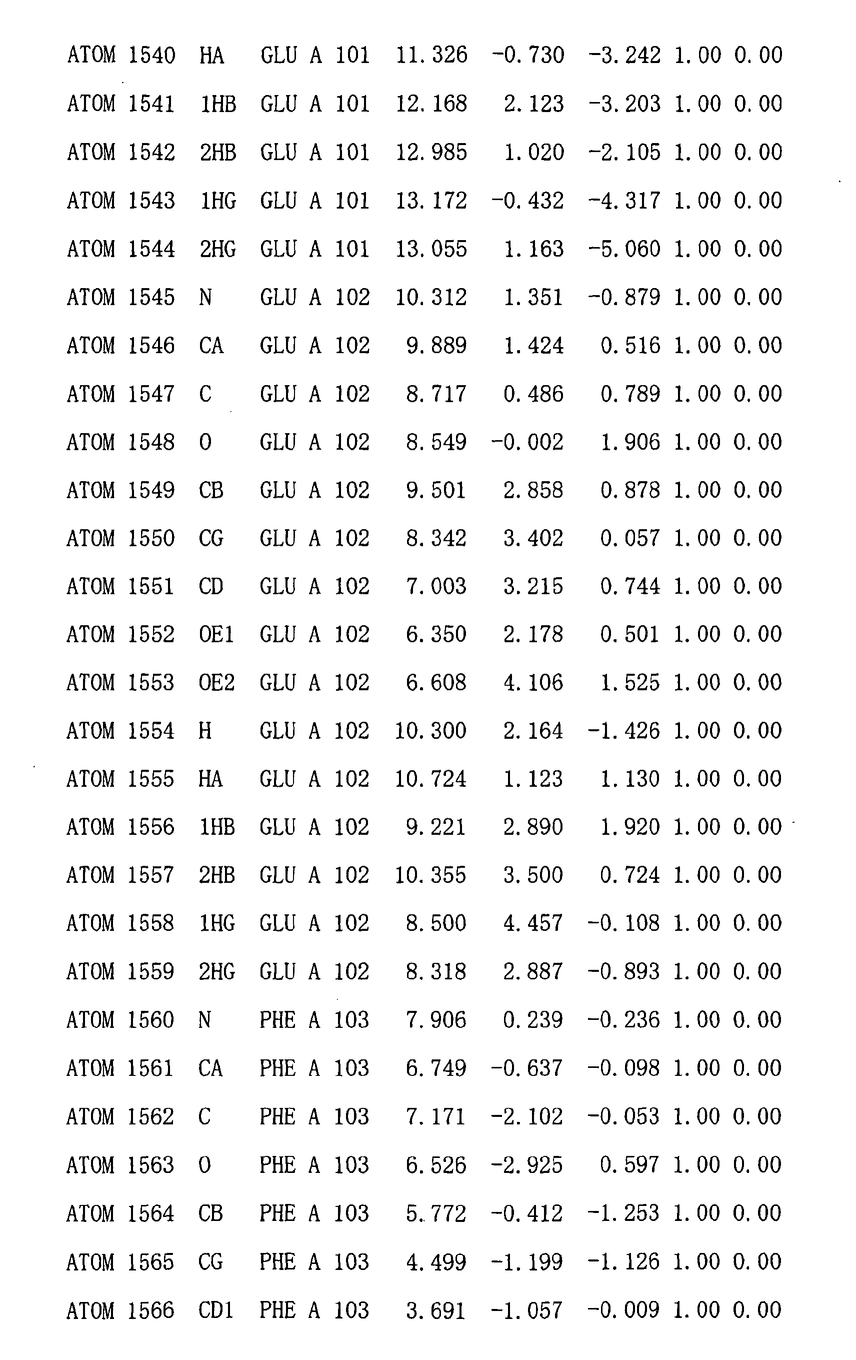

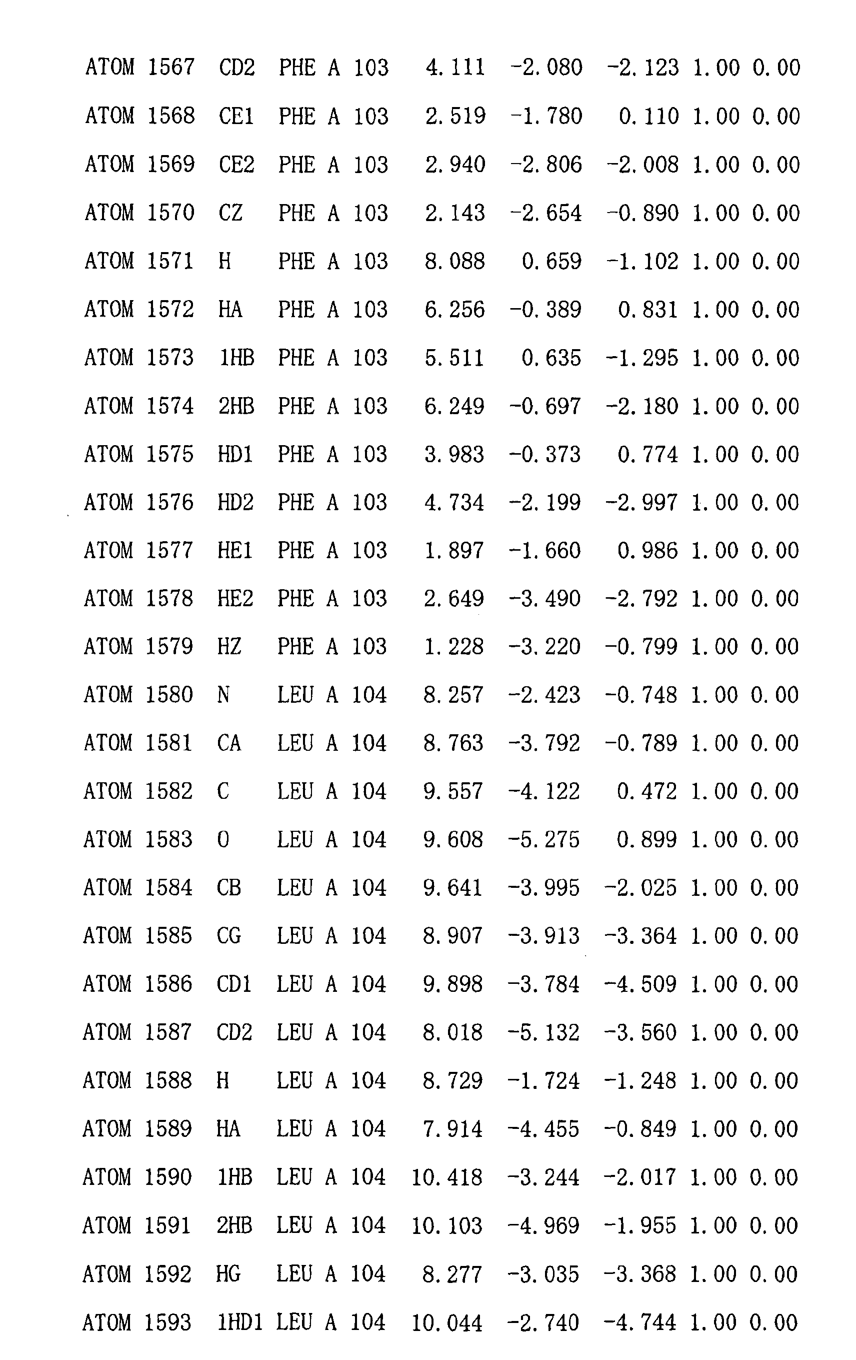

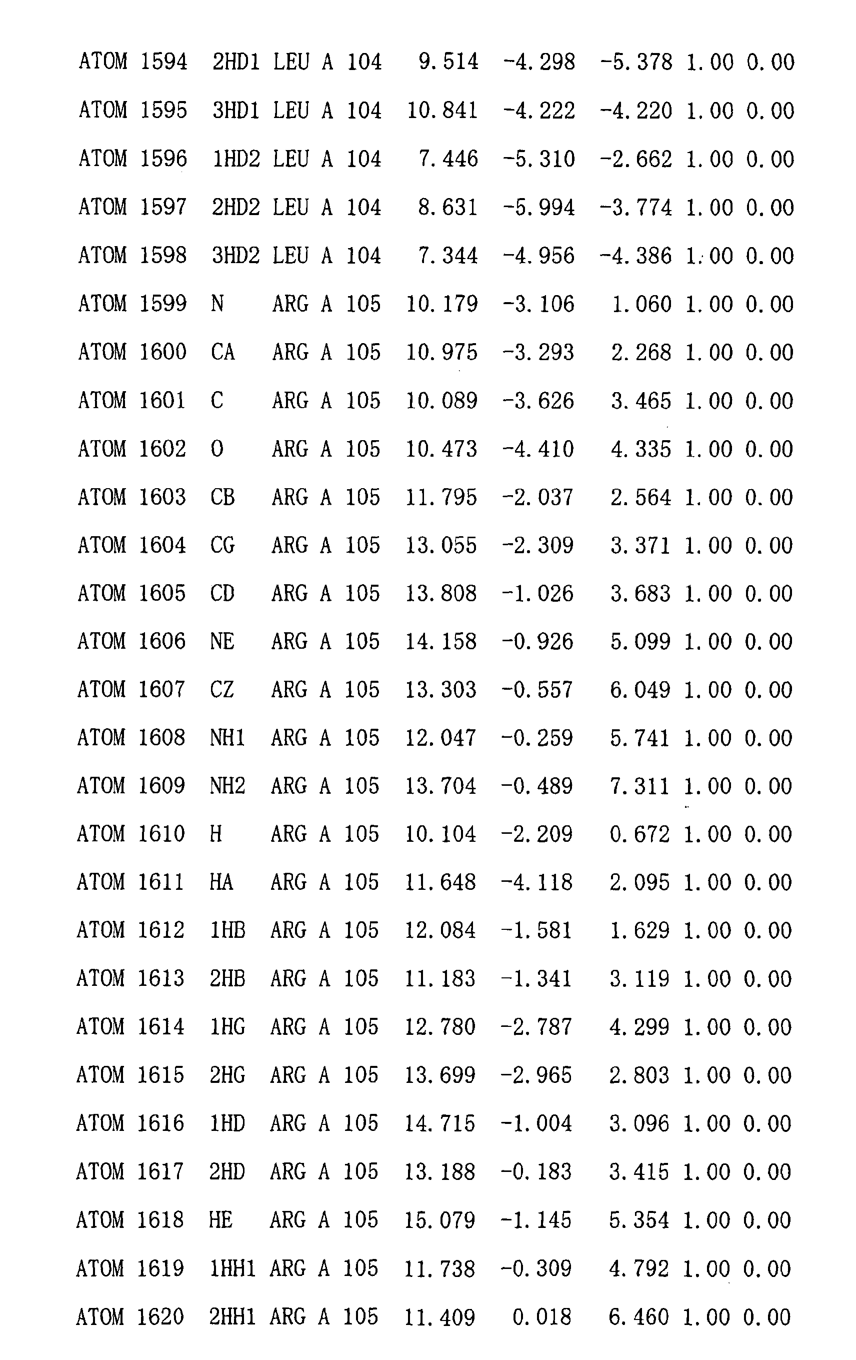

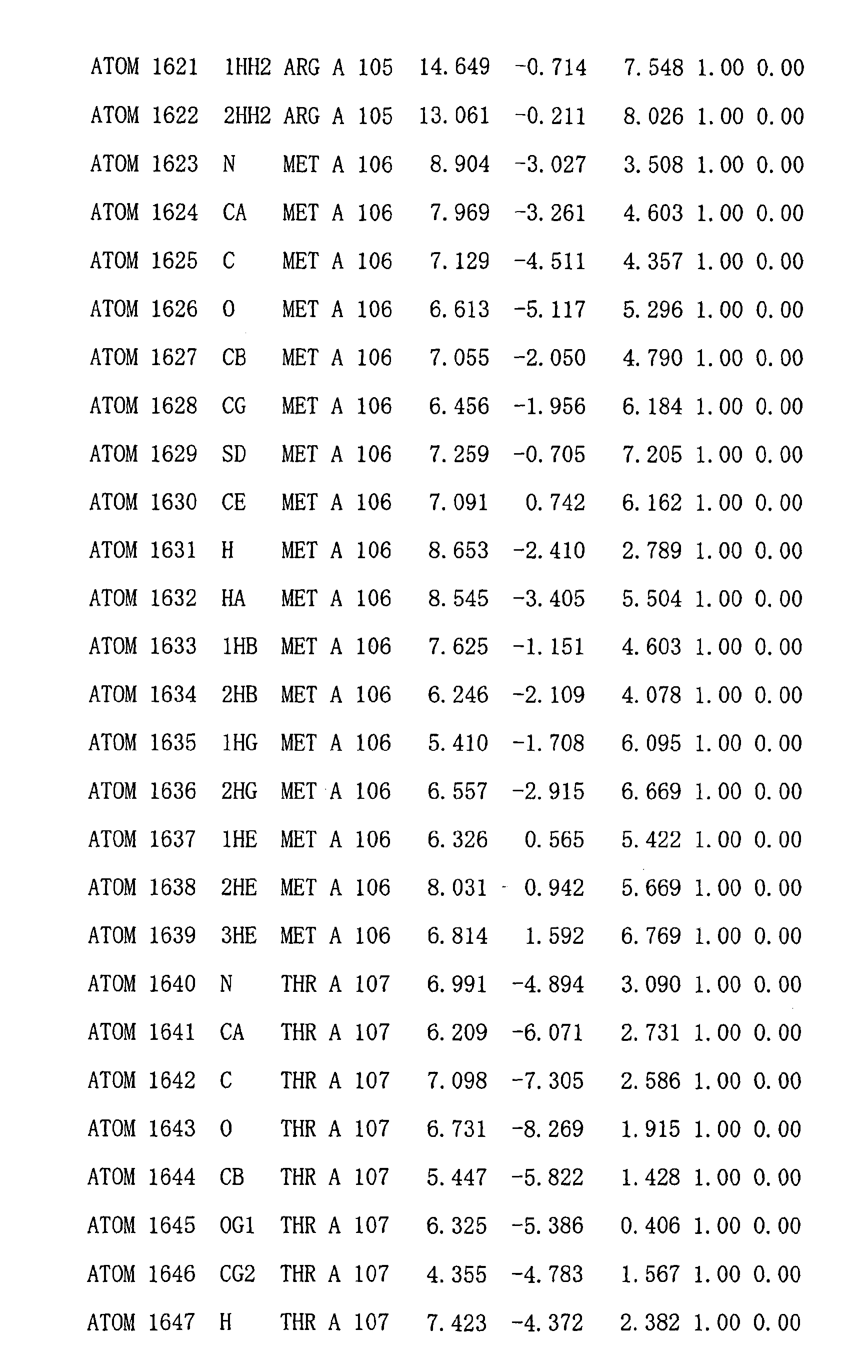

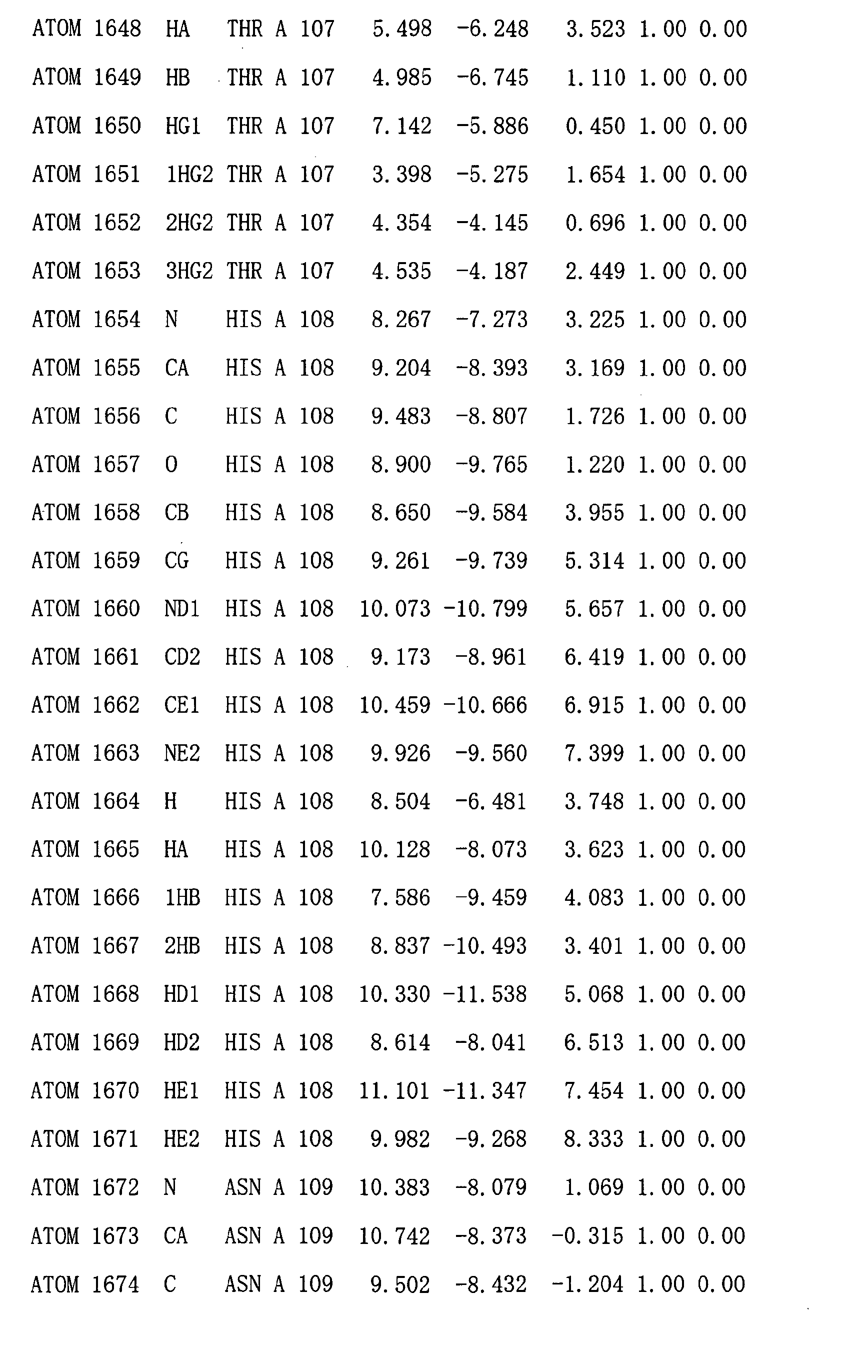

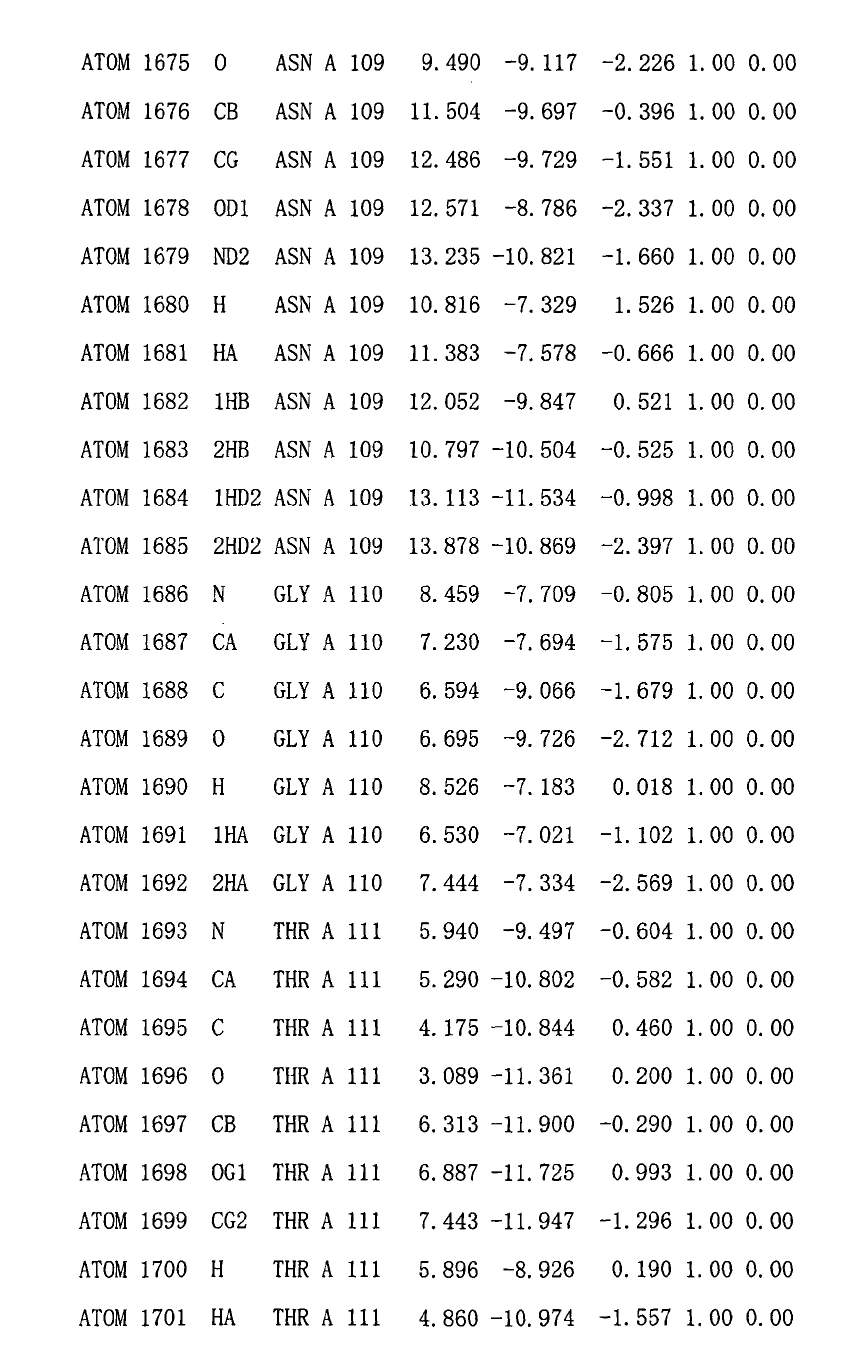

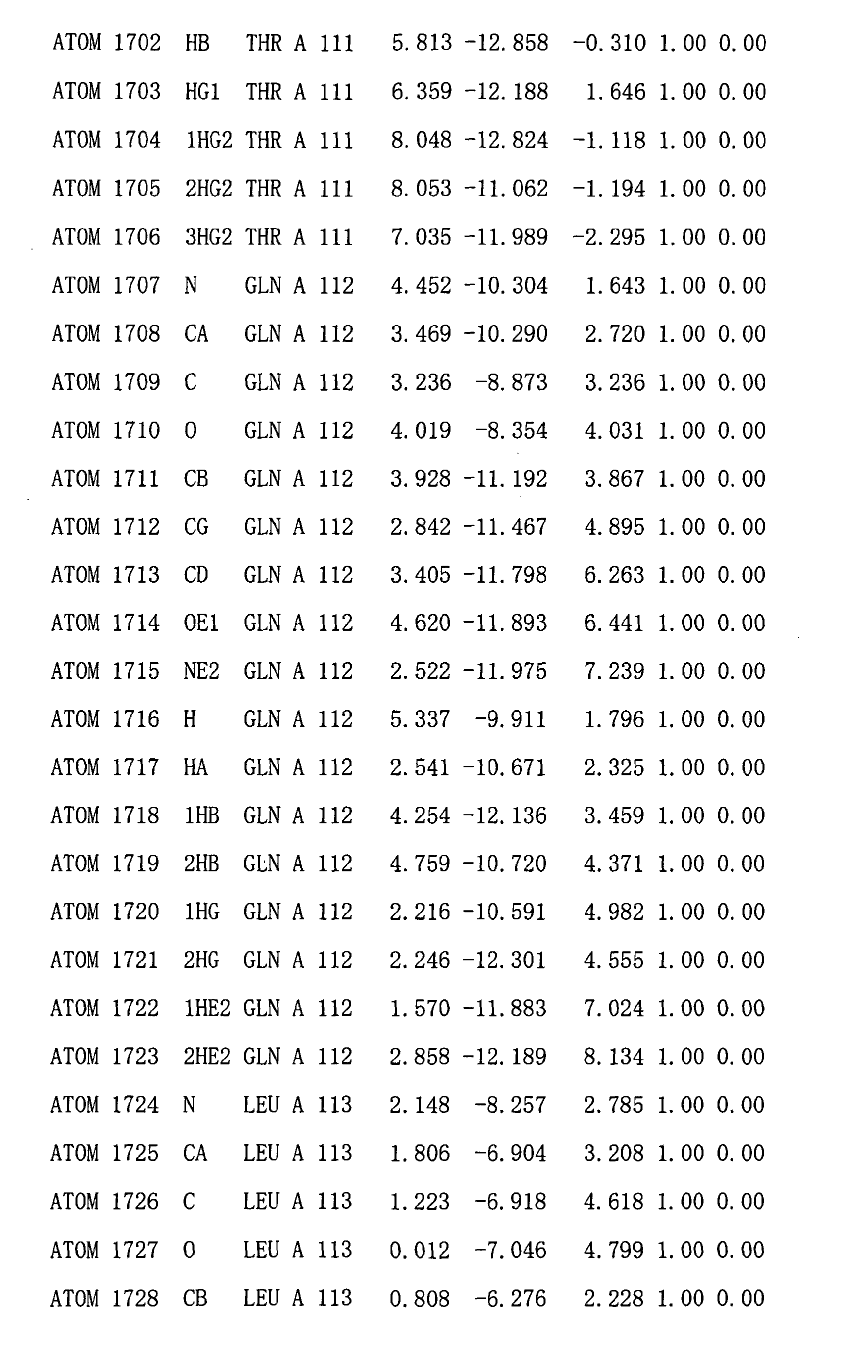

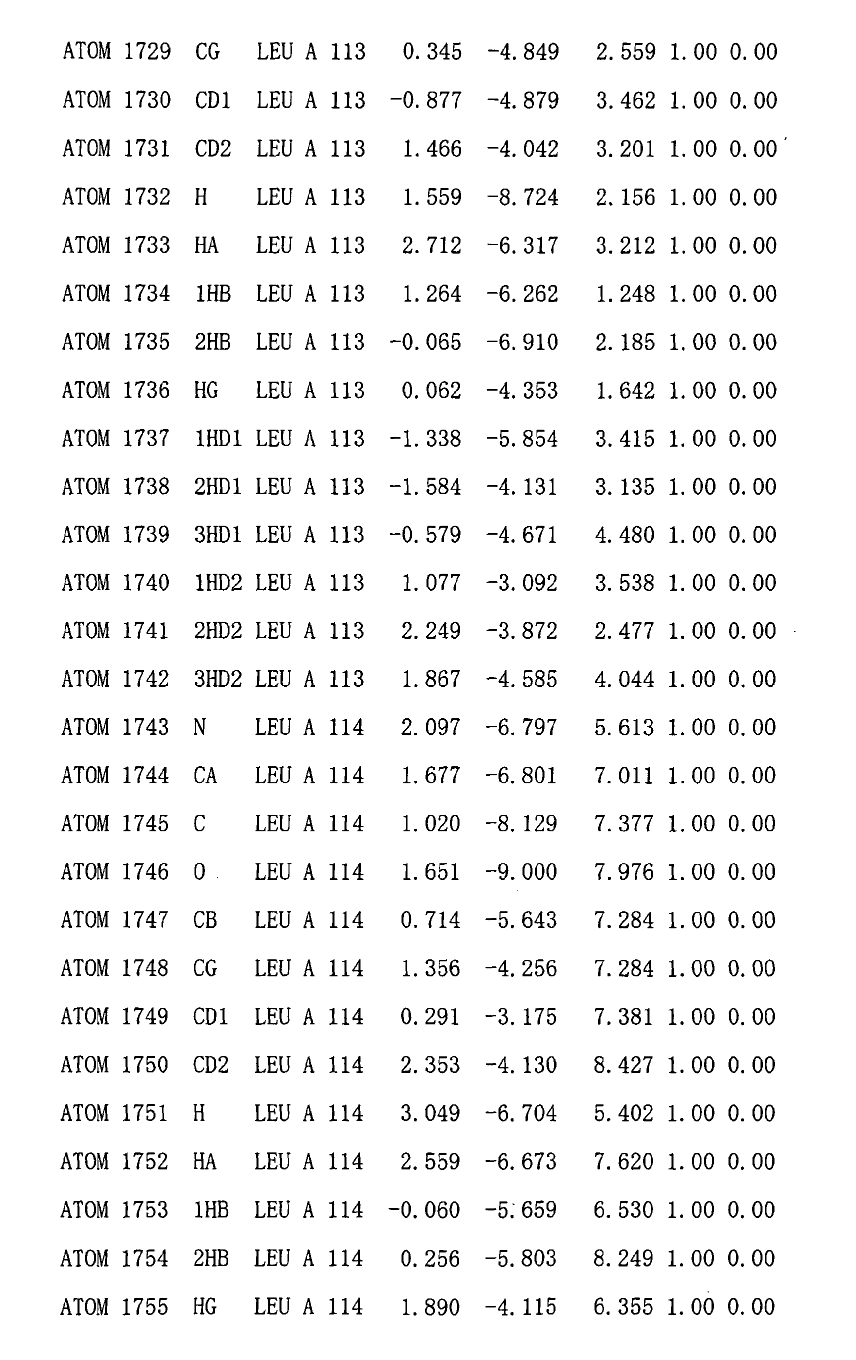

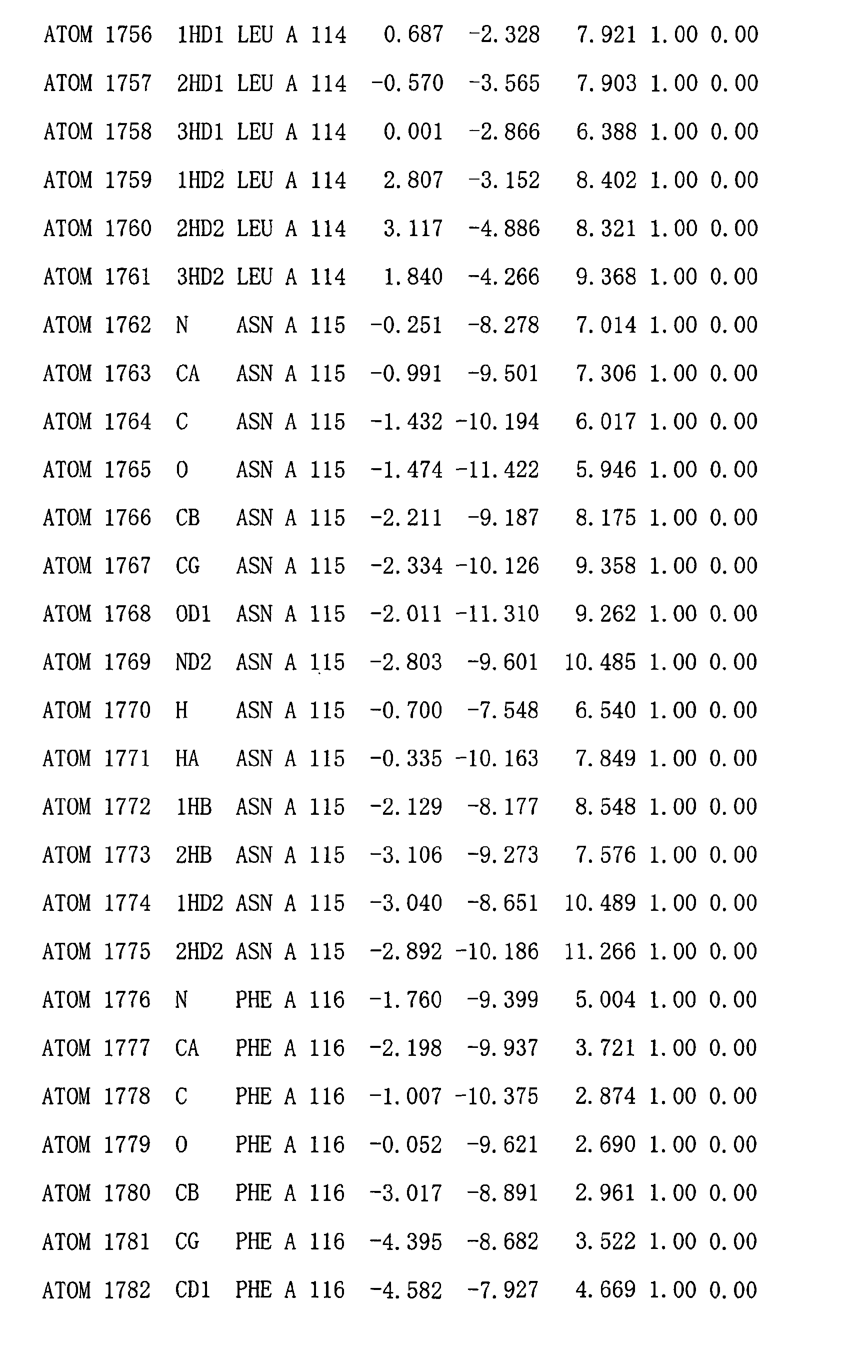

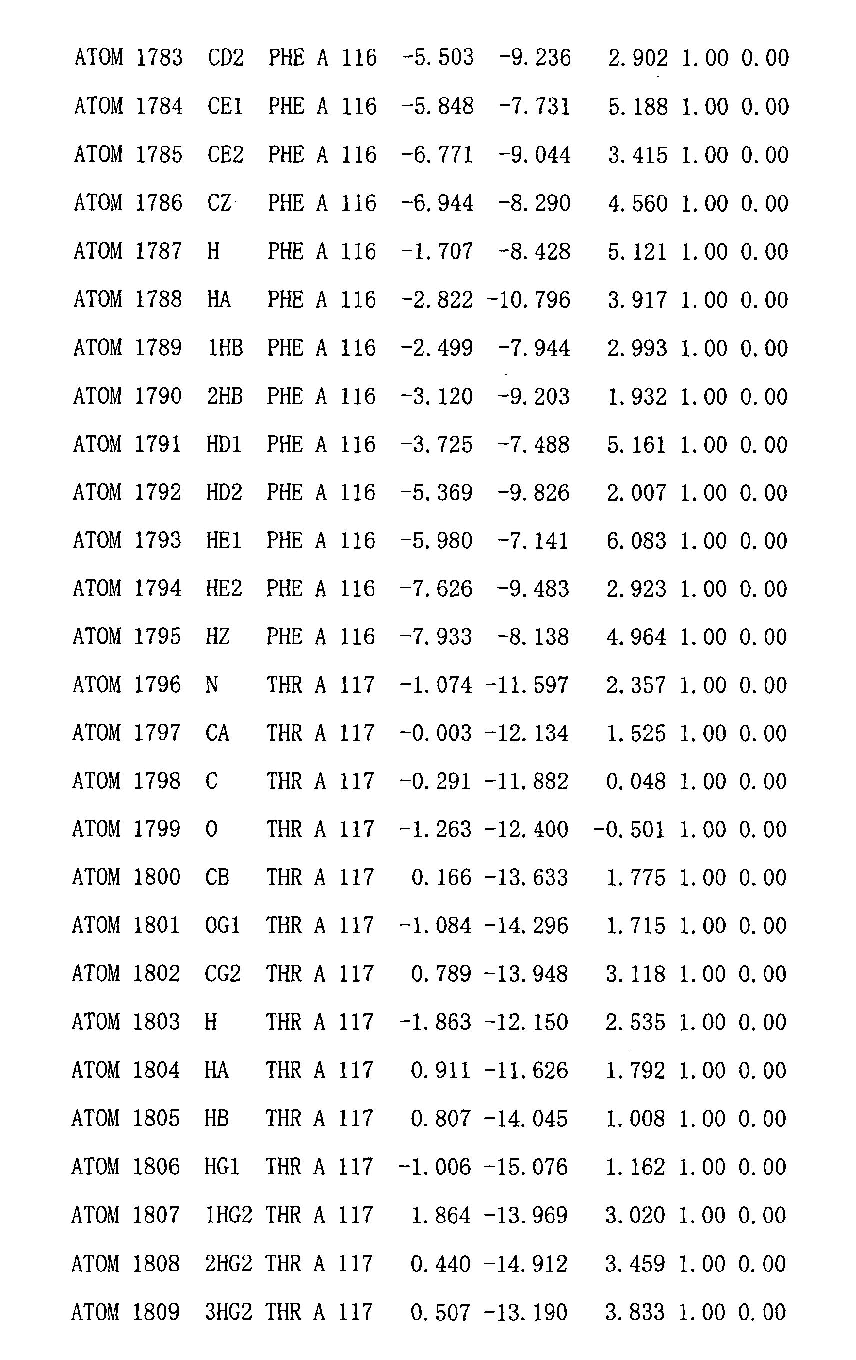

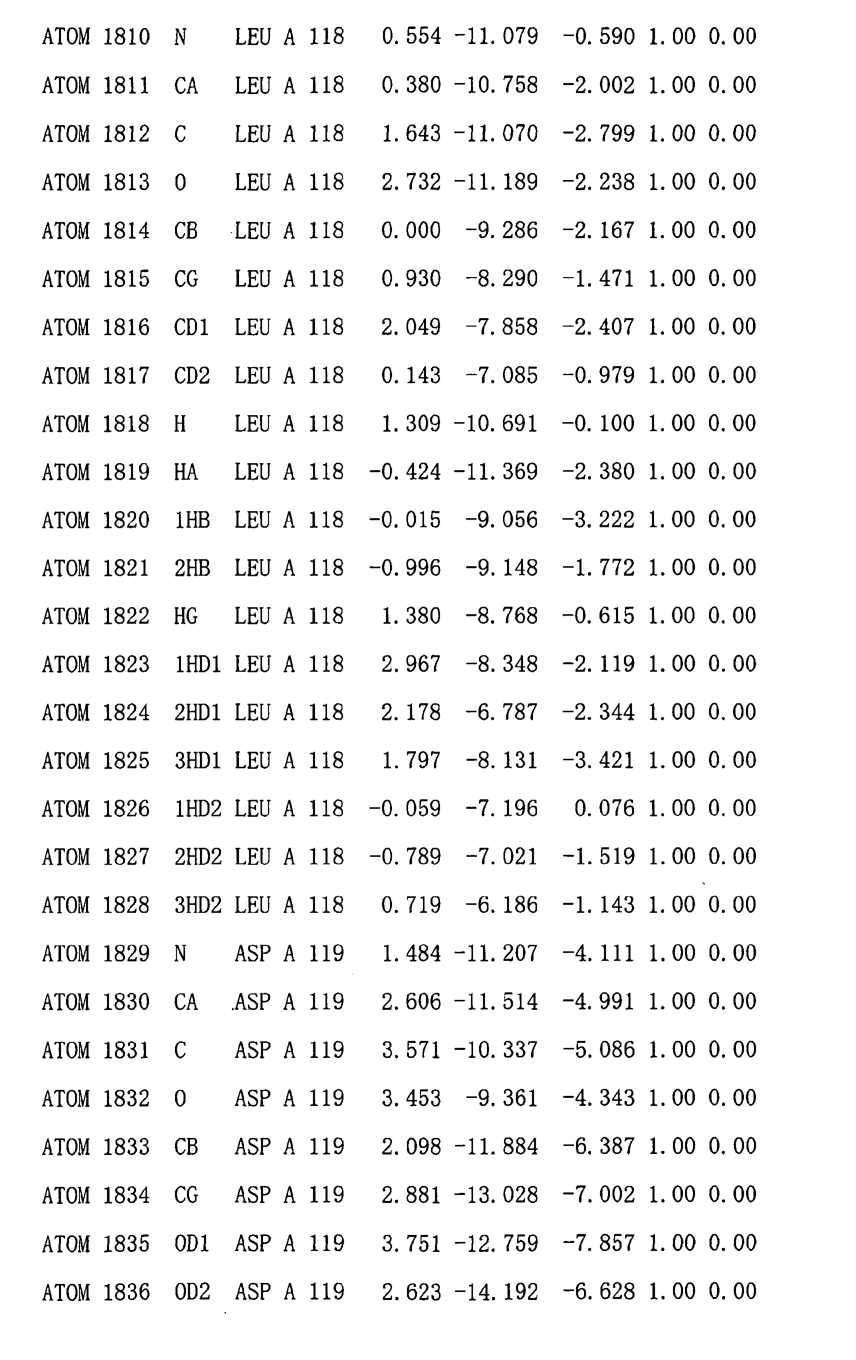

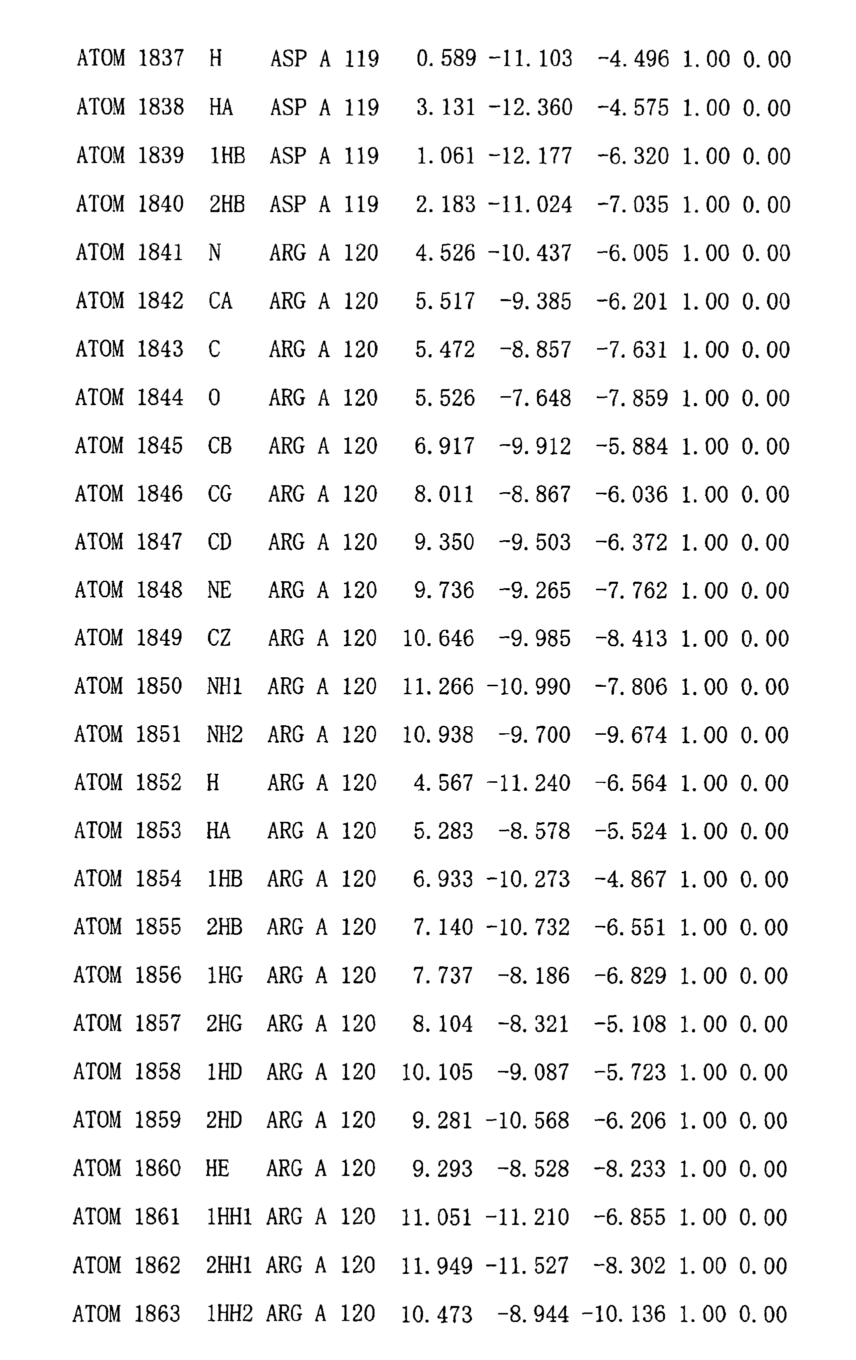

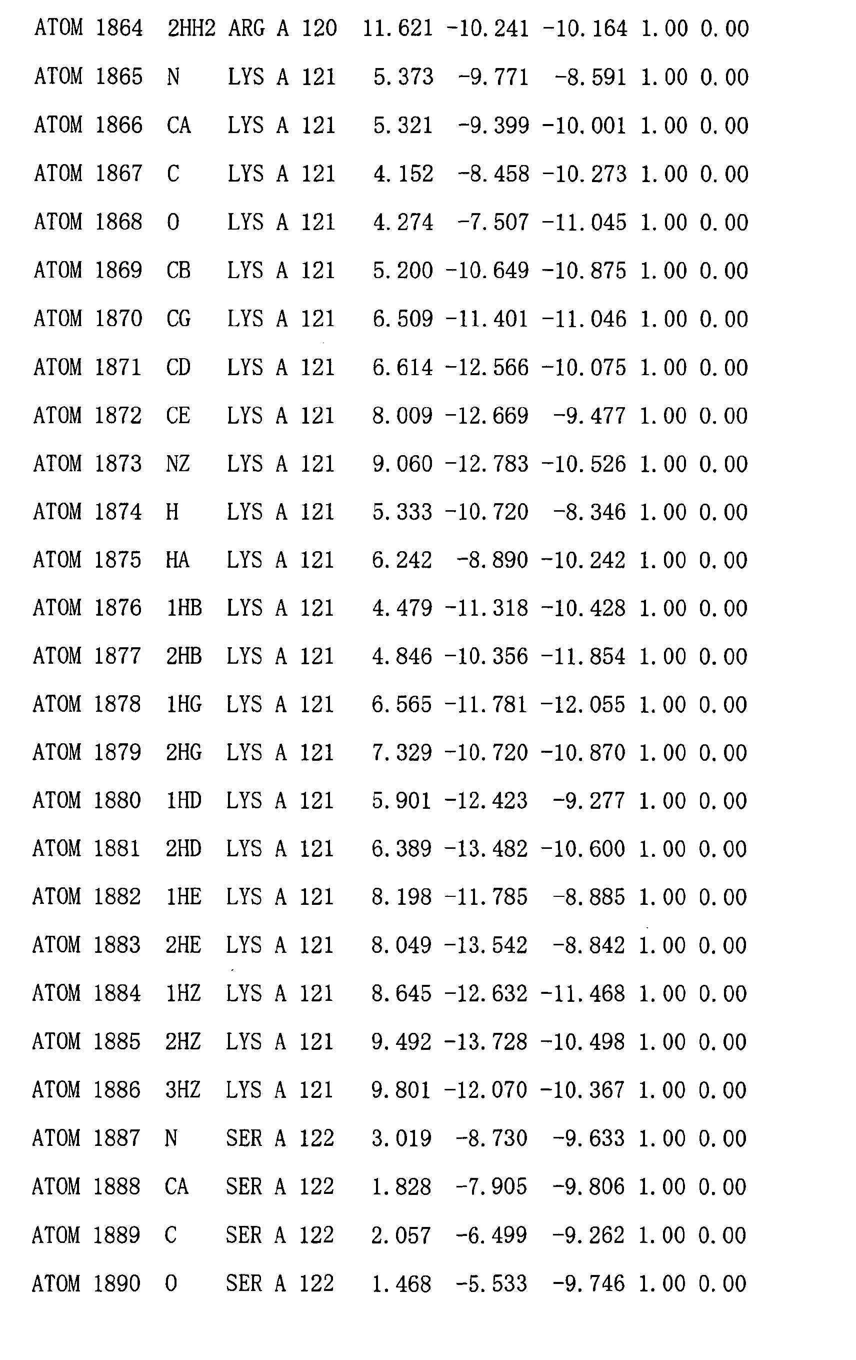

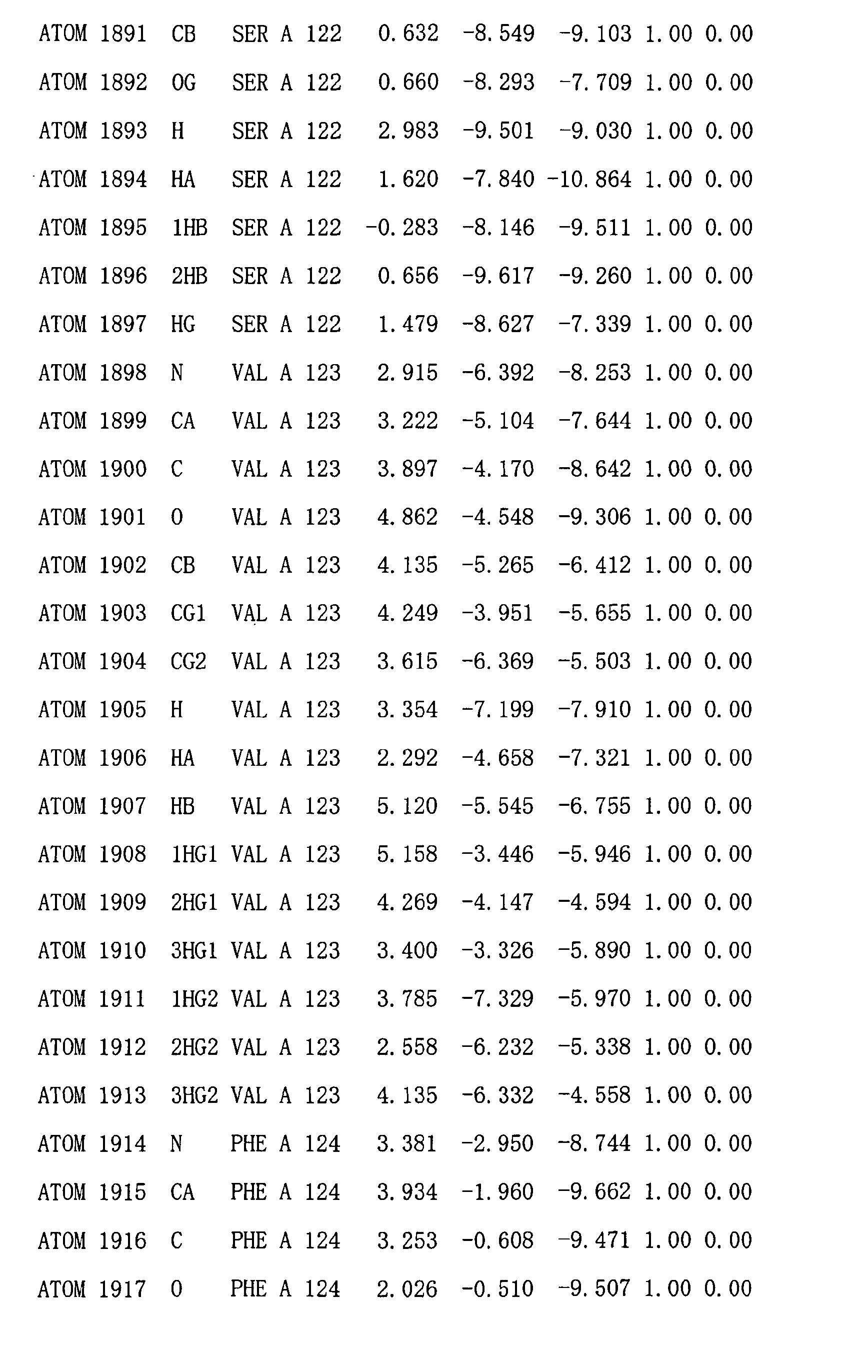

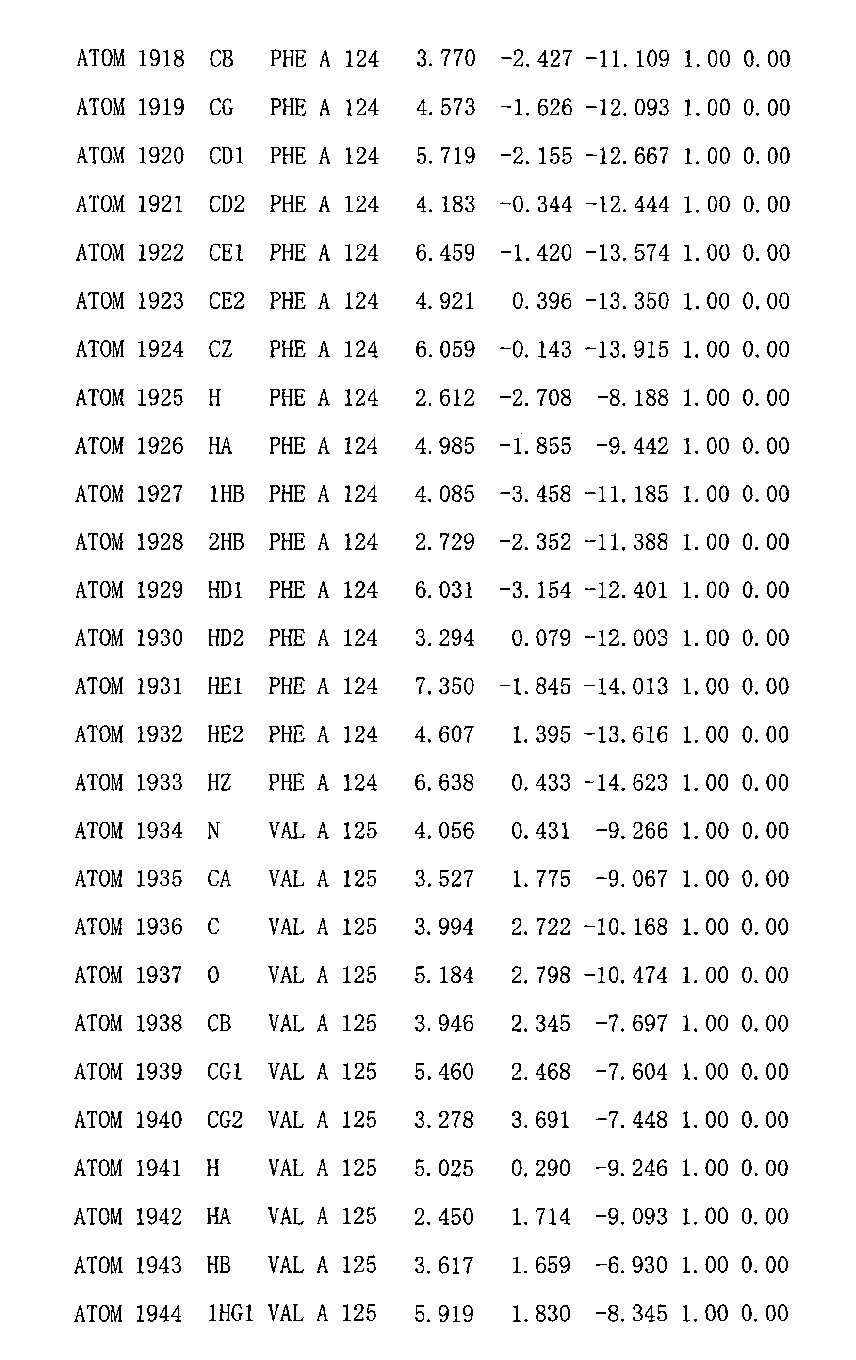

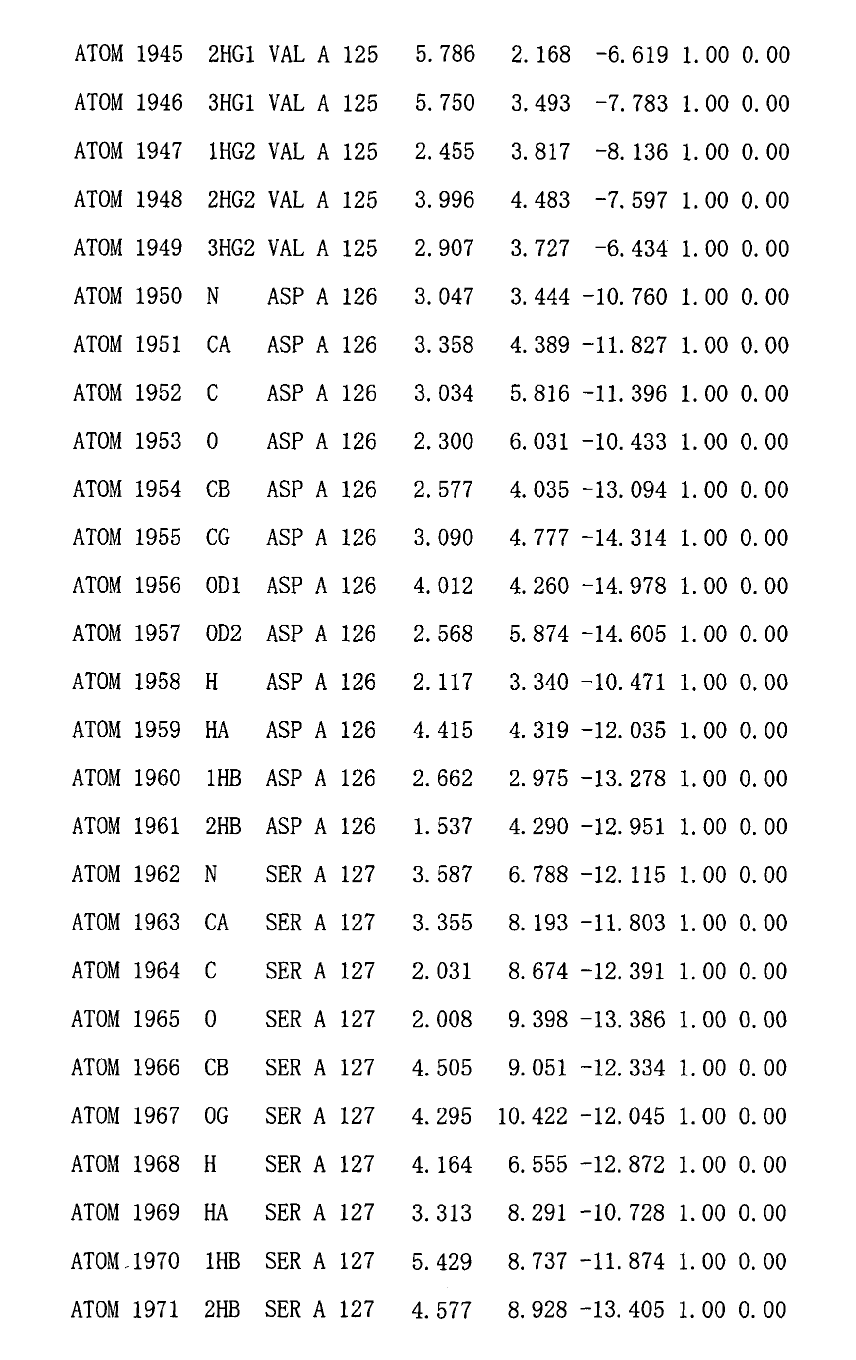

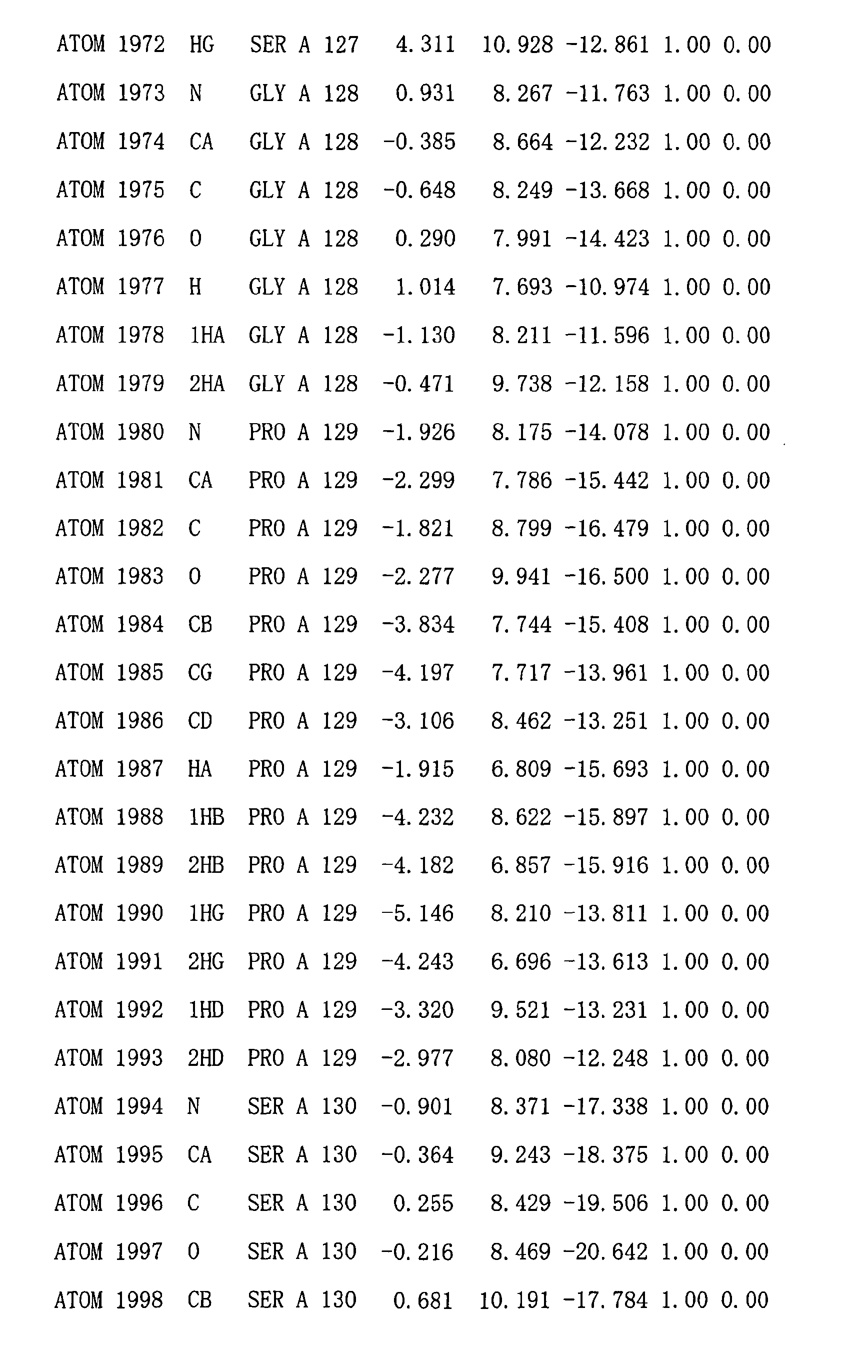

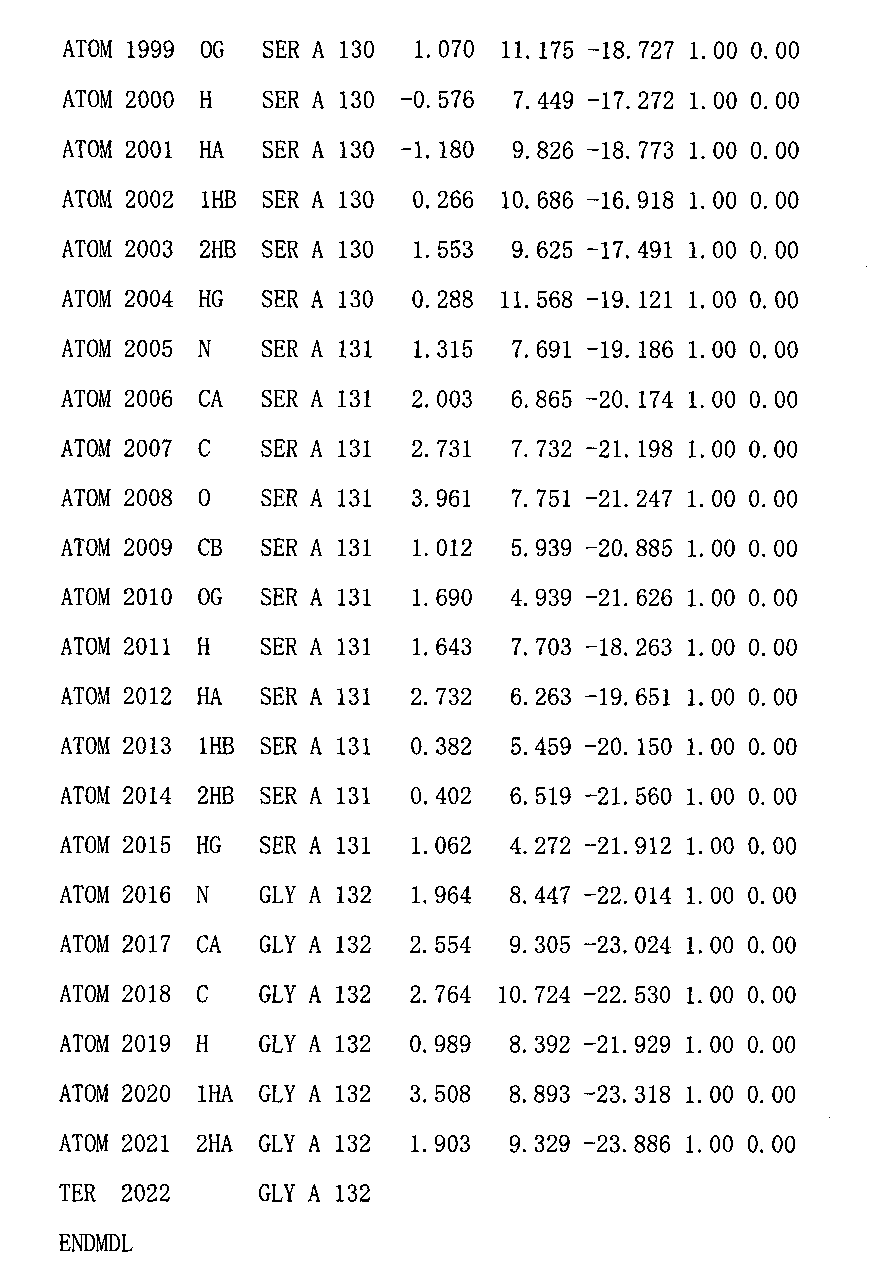

### Three-Dimensional Structure Coordinate 18

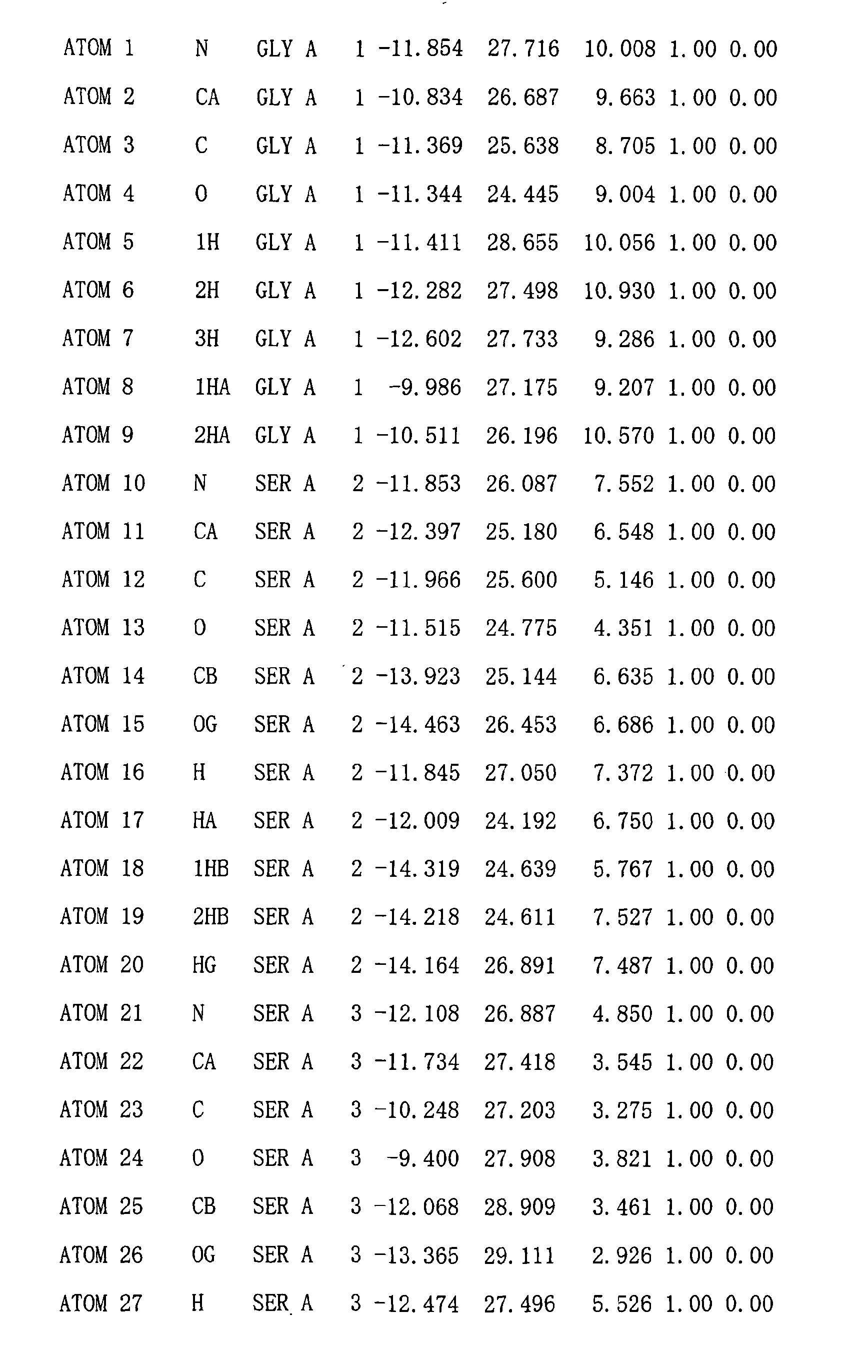

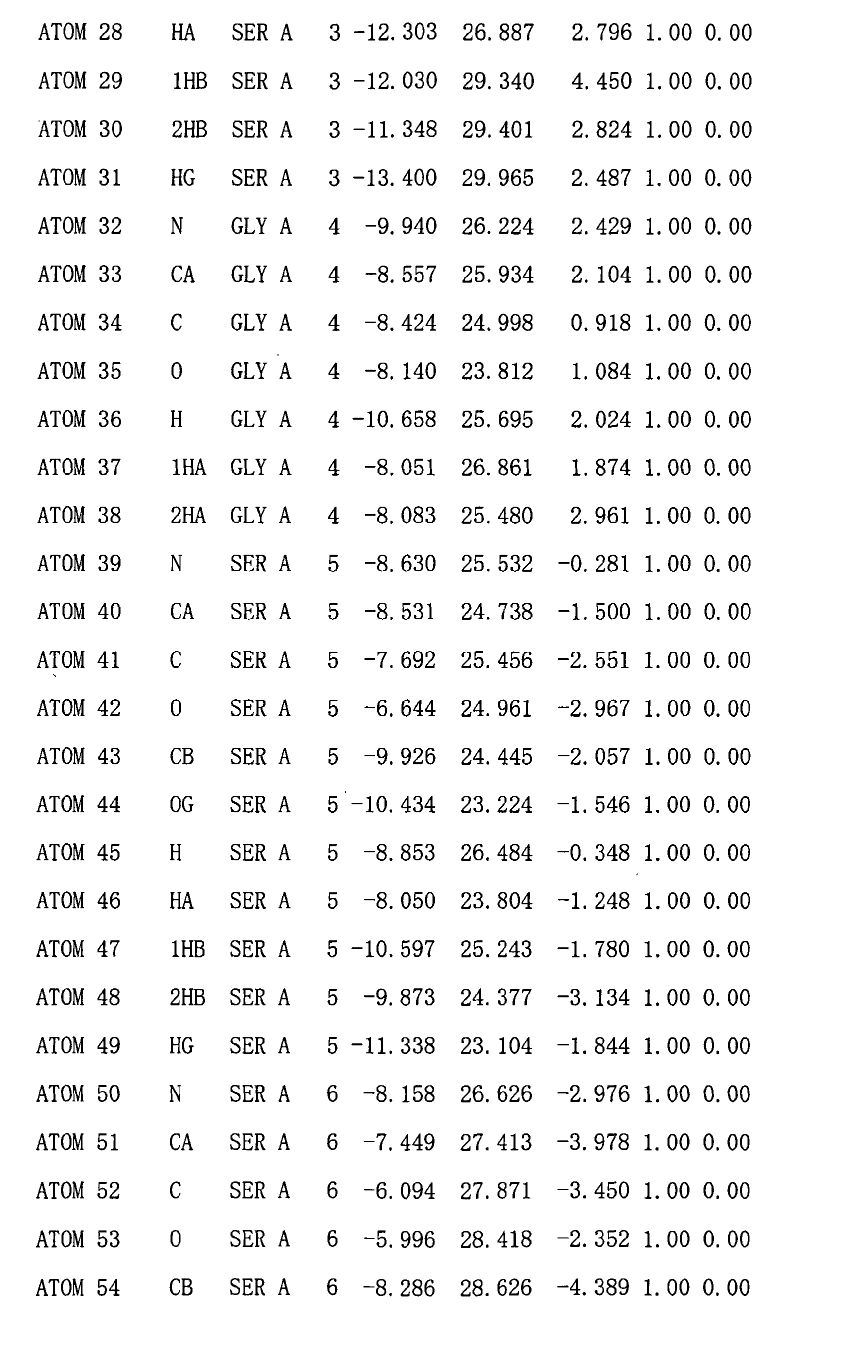

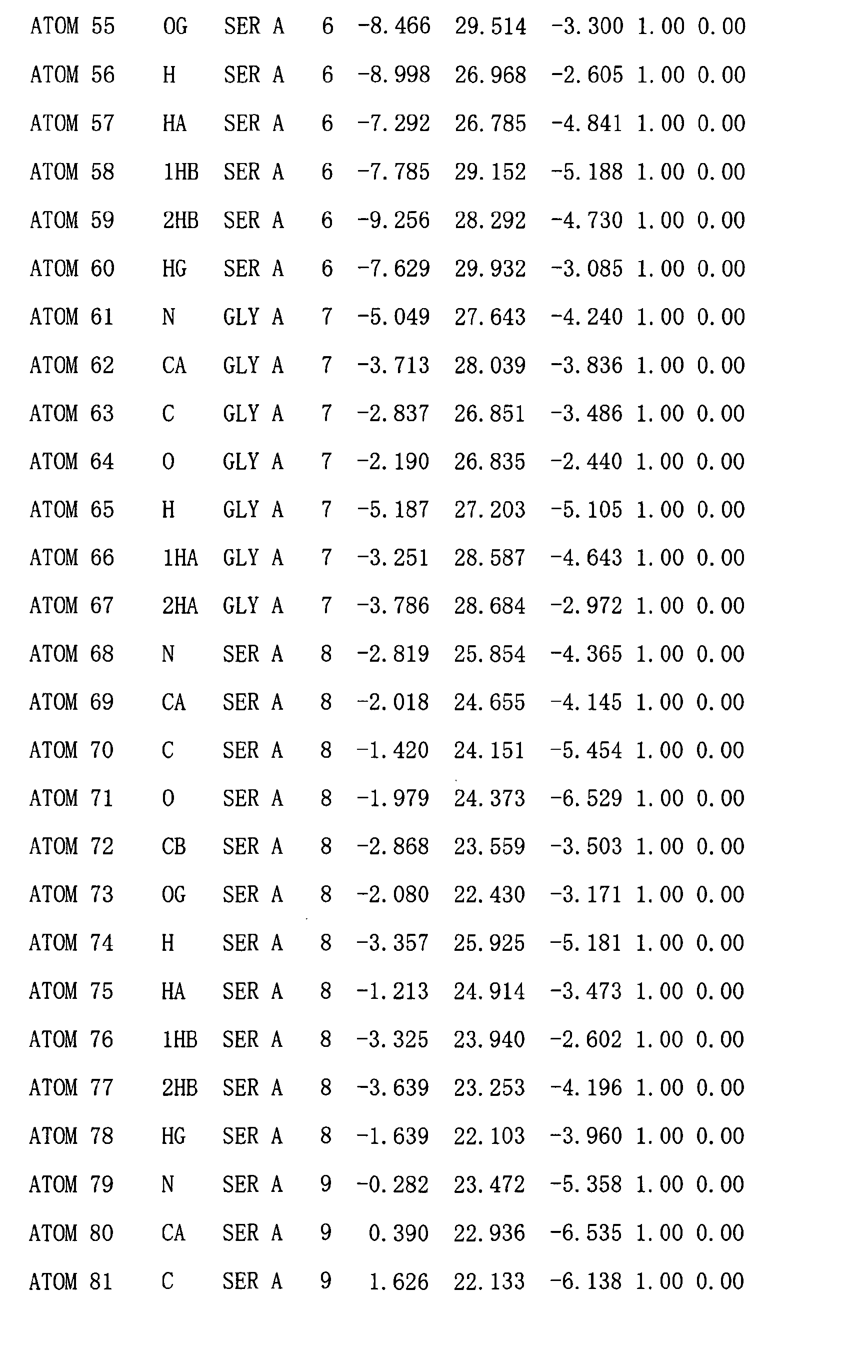

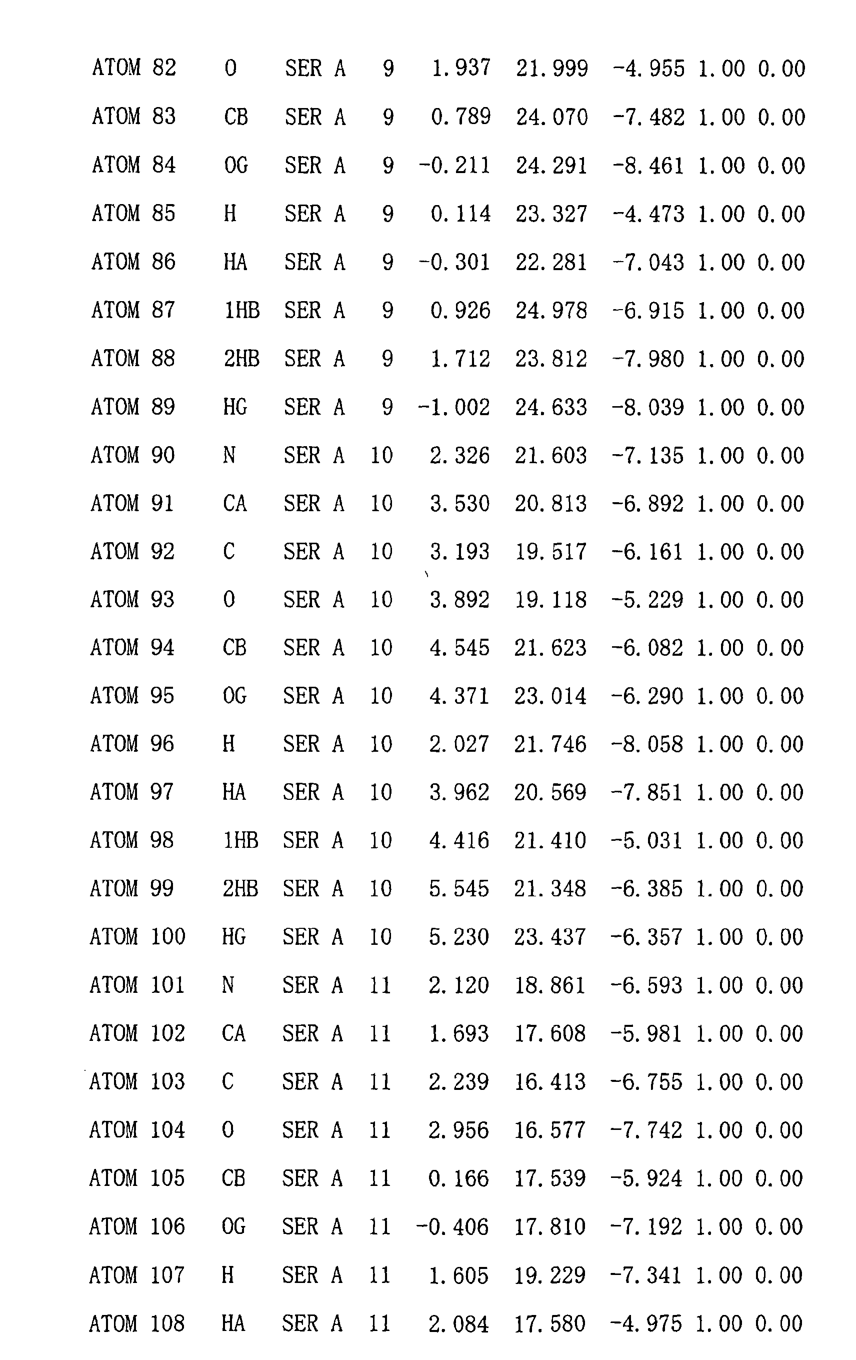

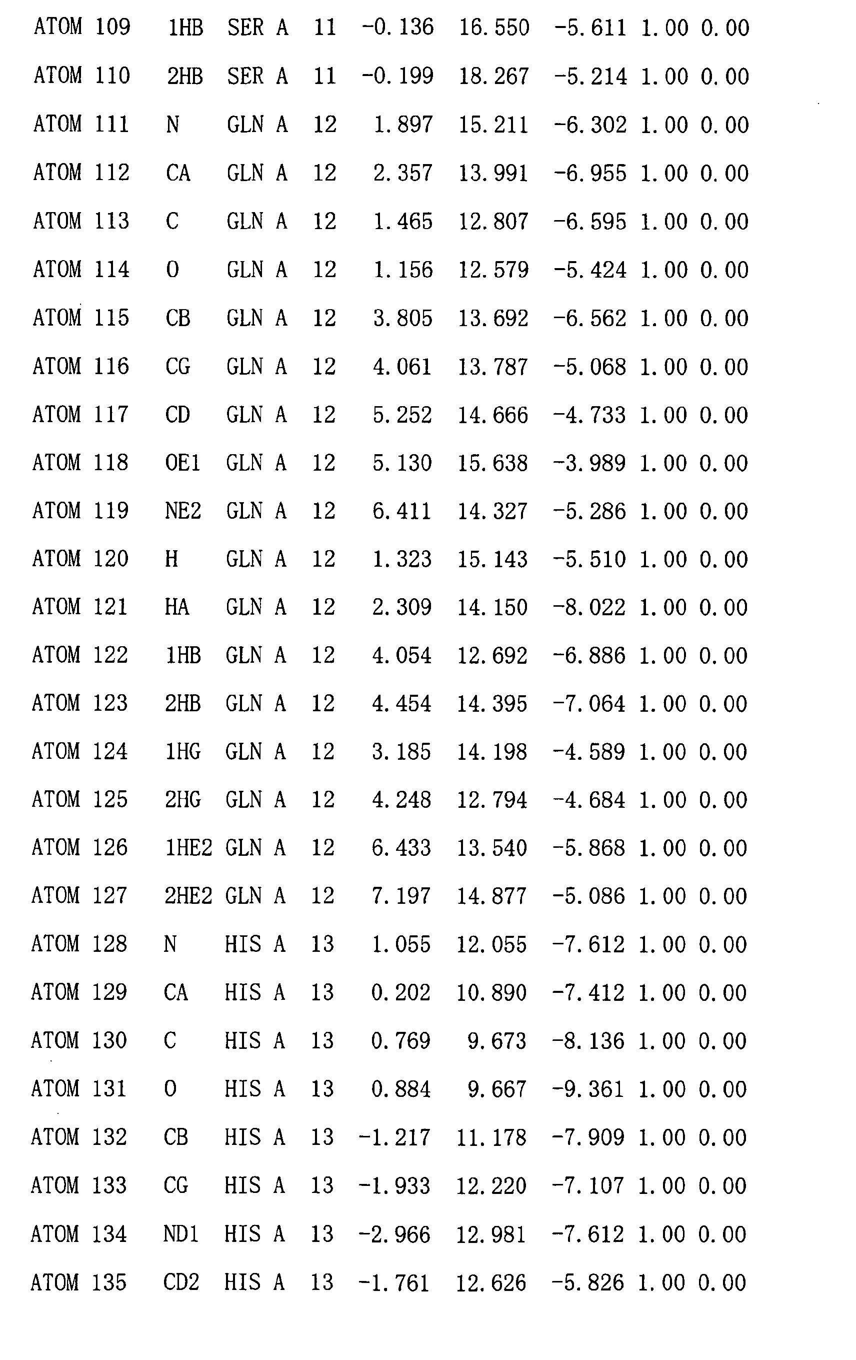

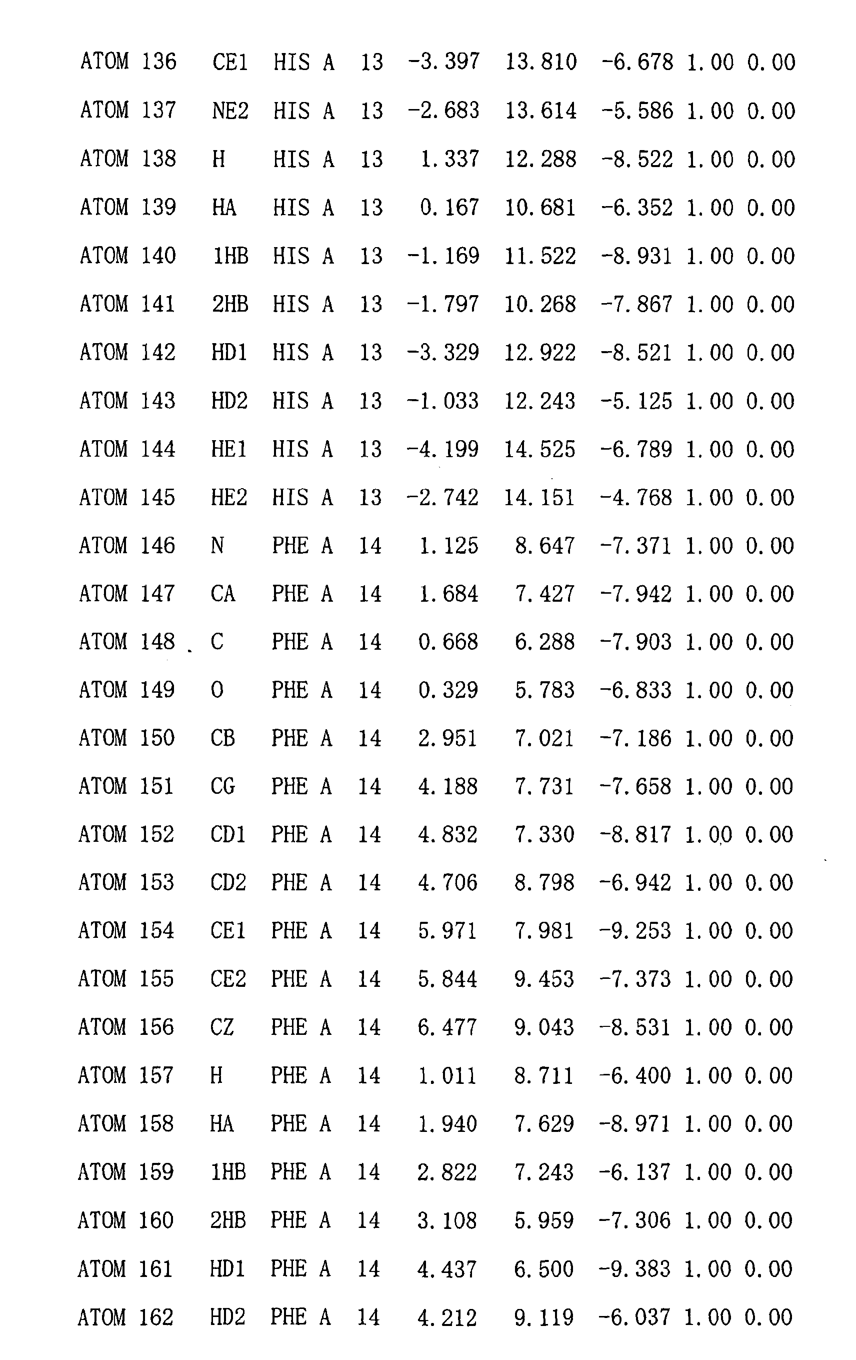

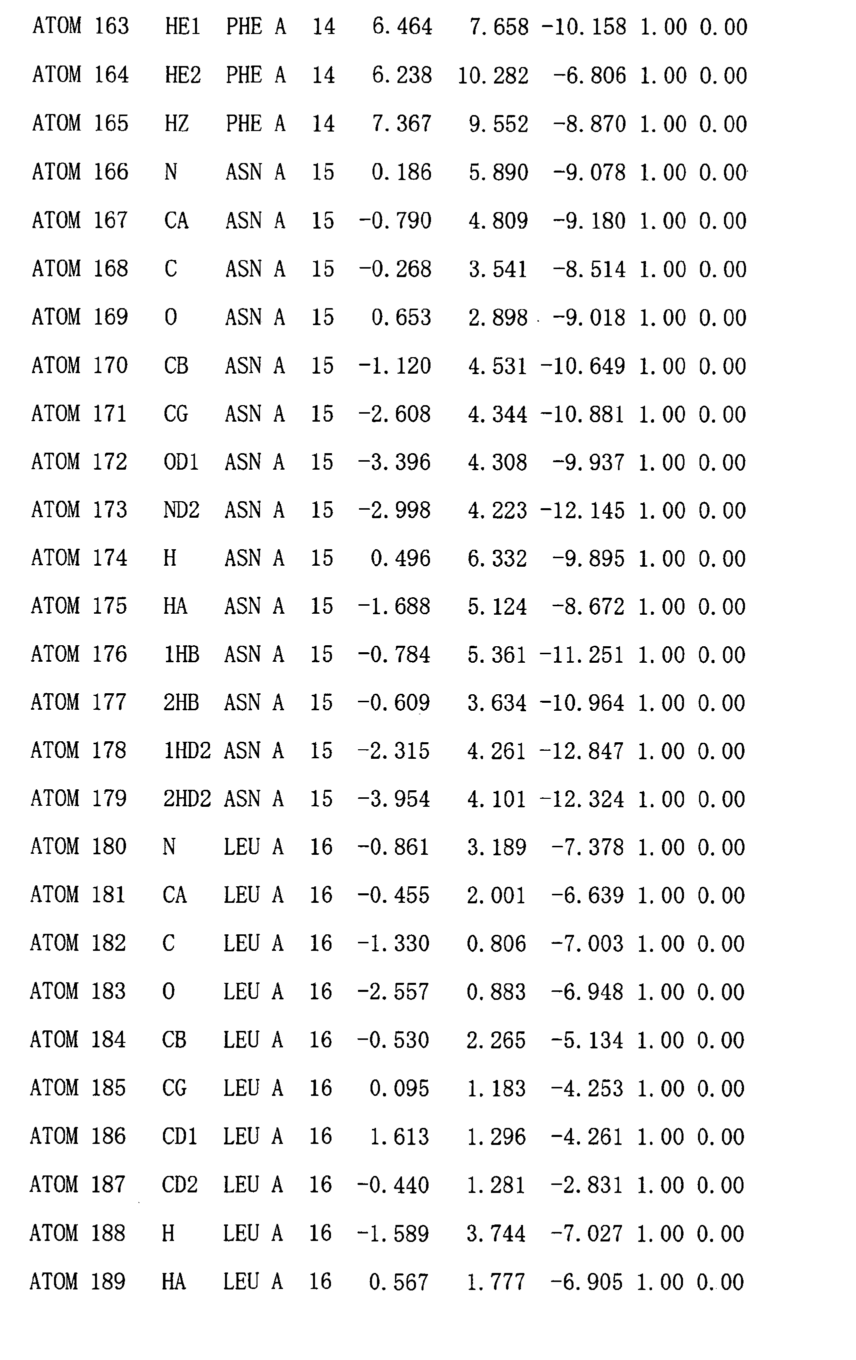

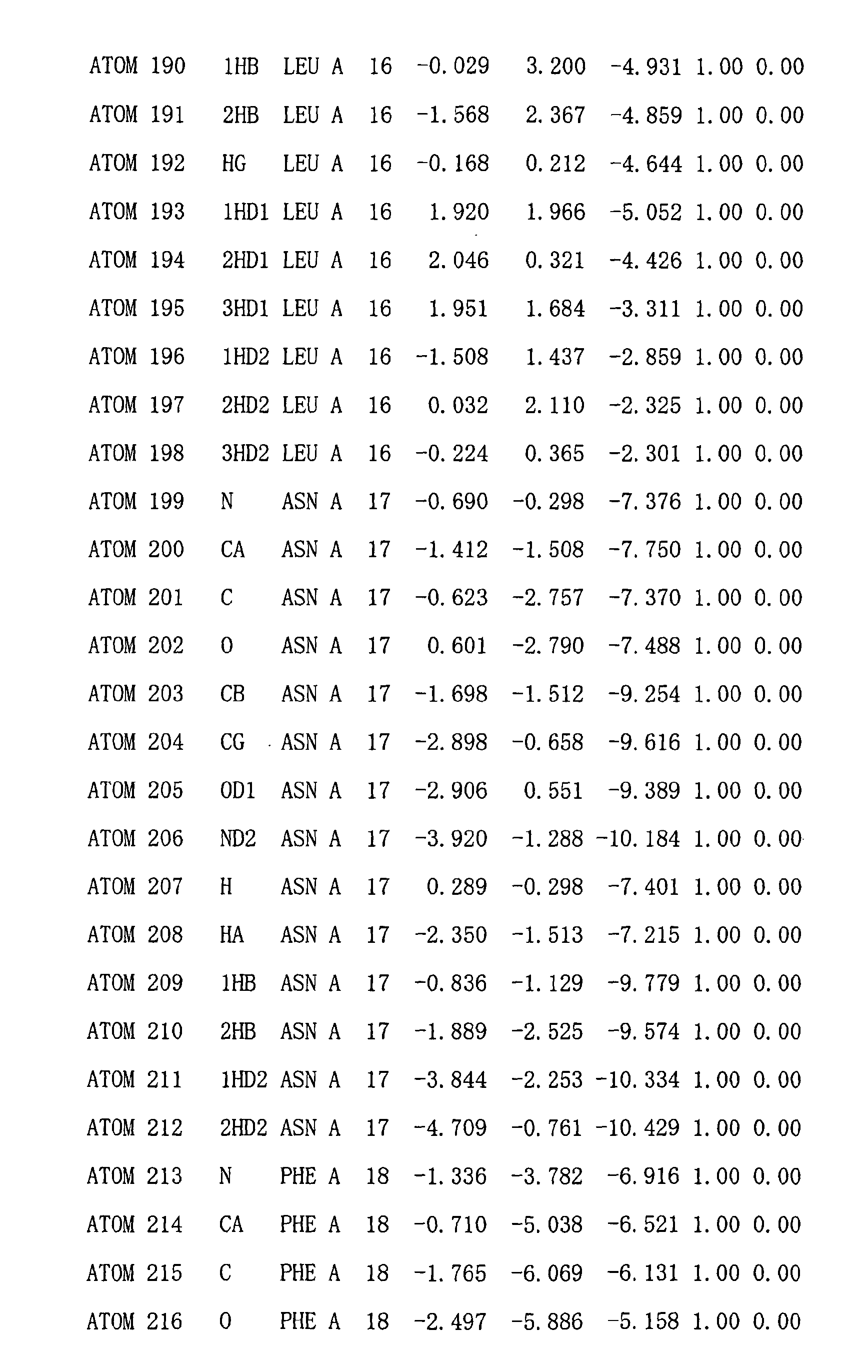

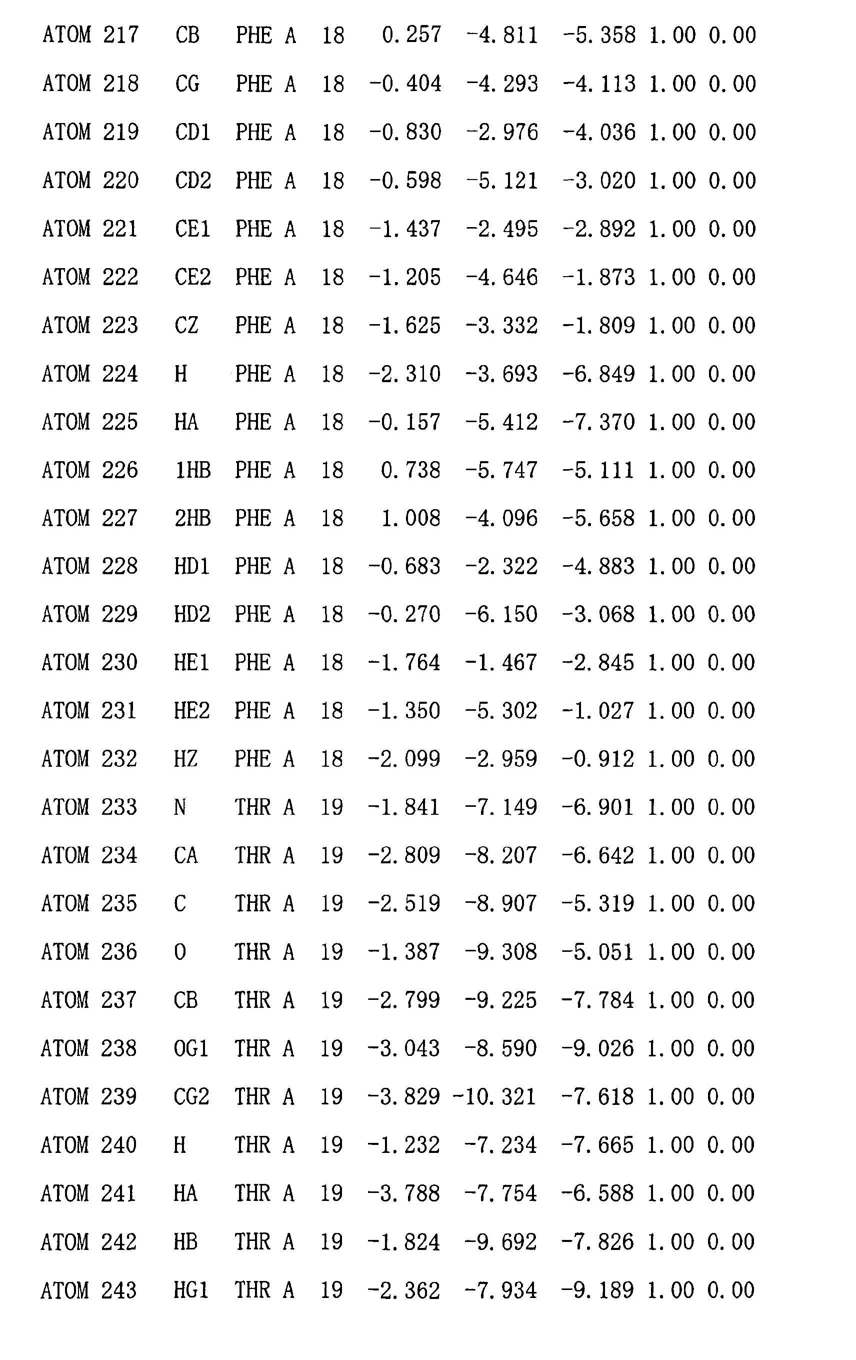

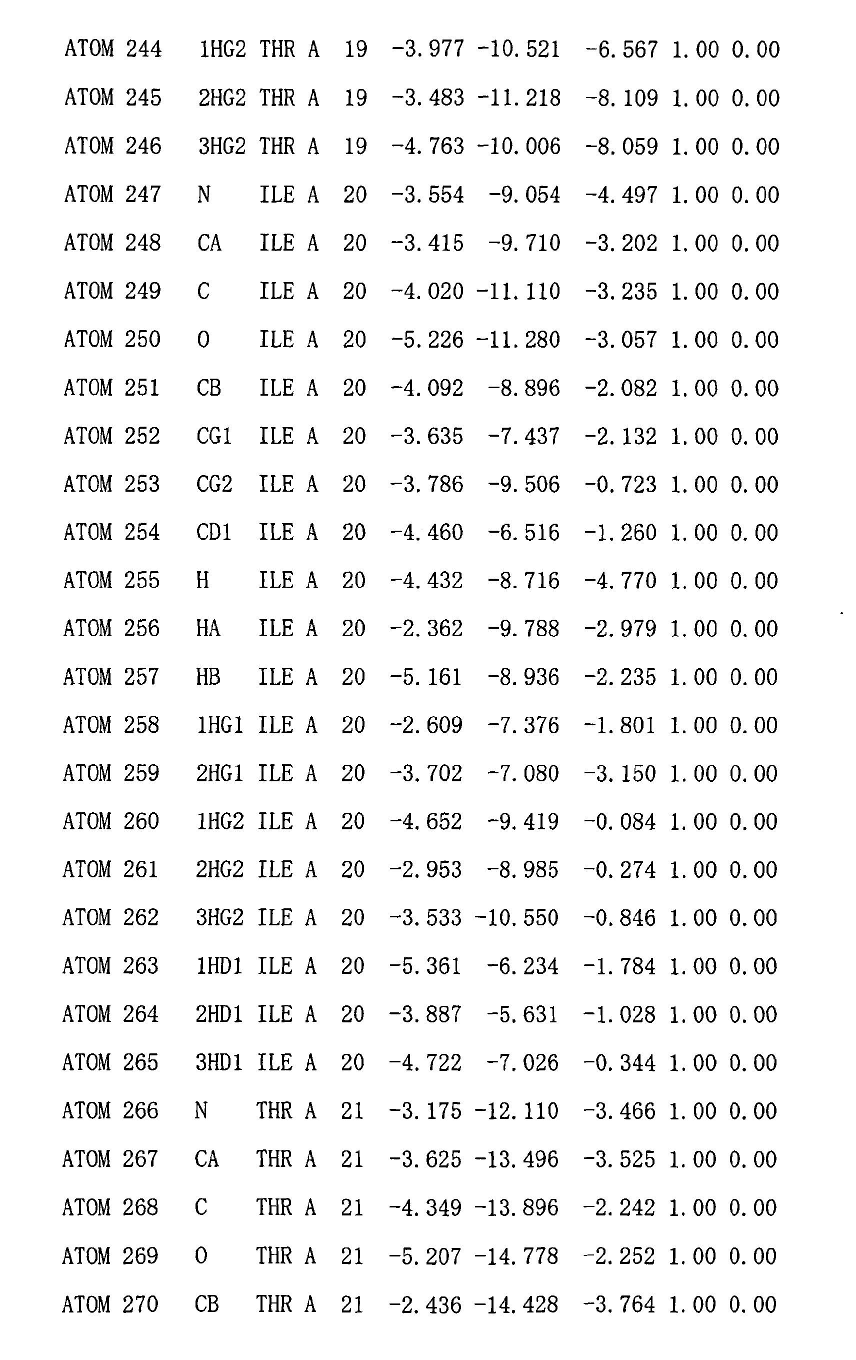

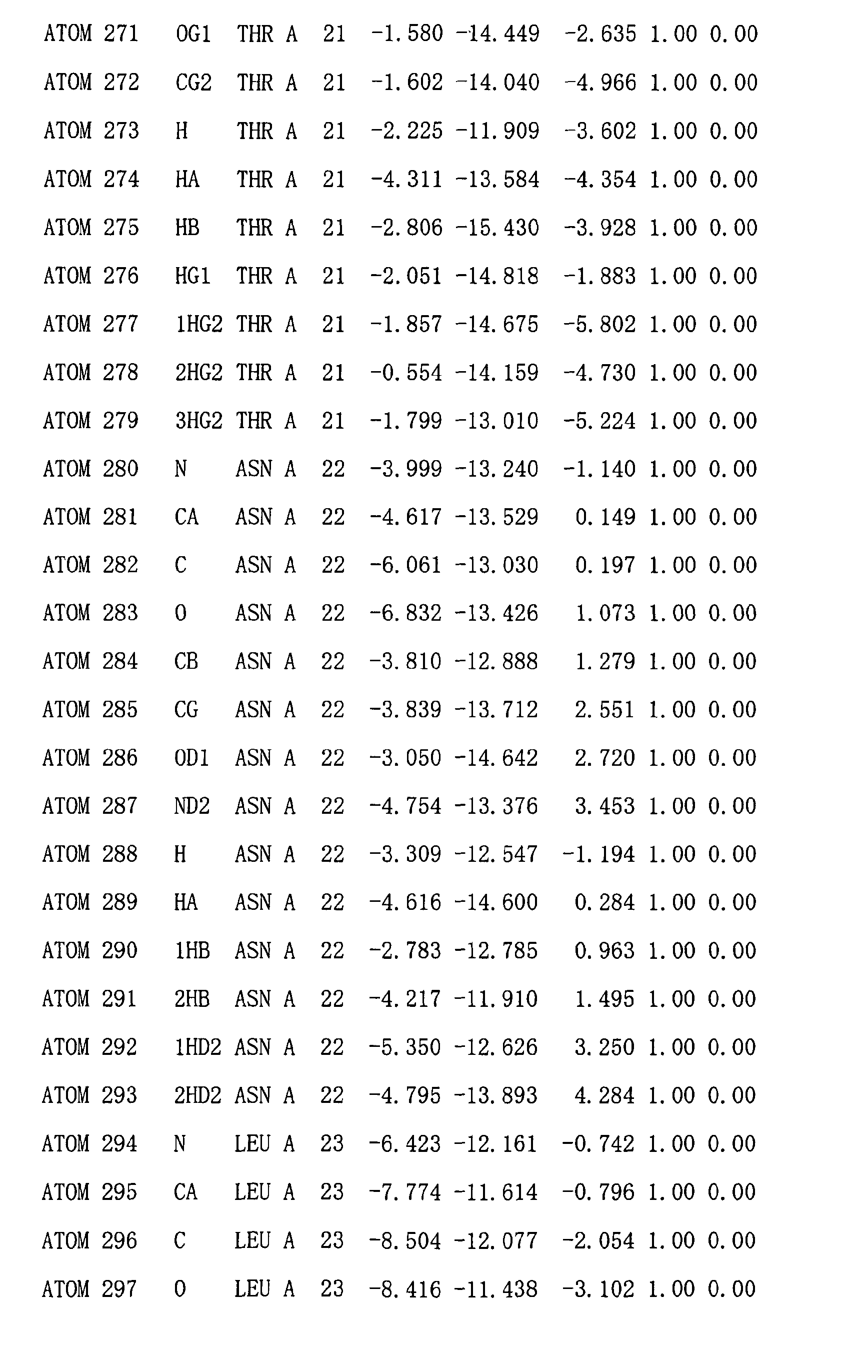

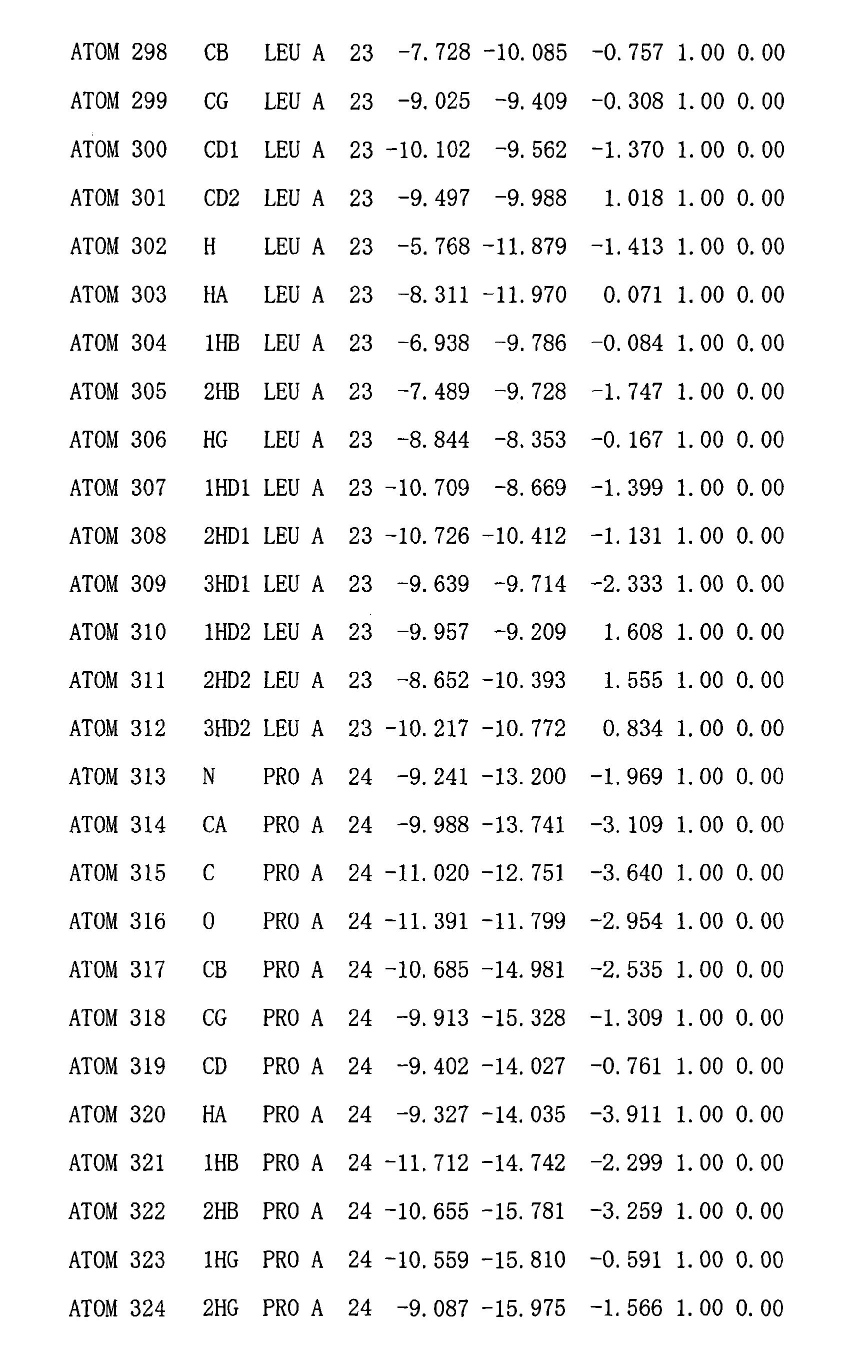

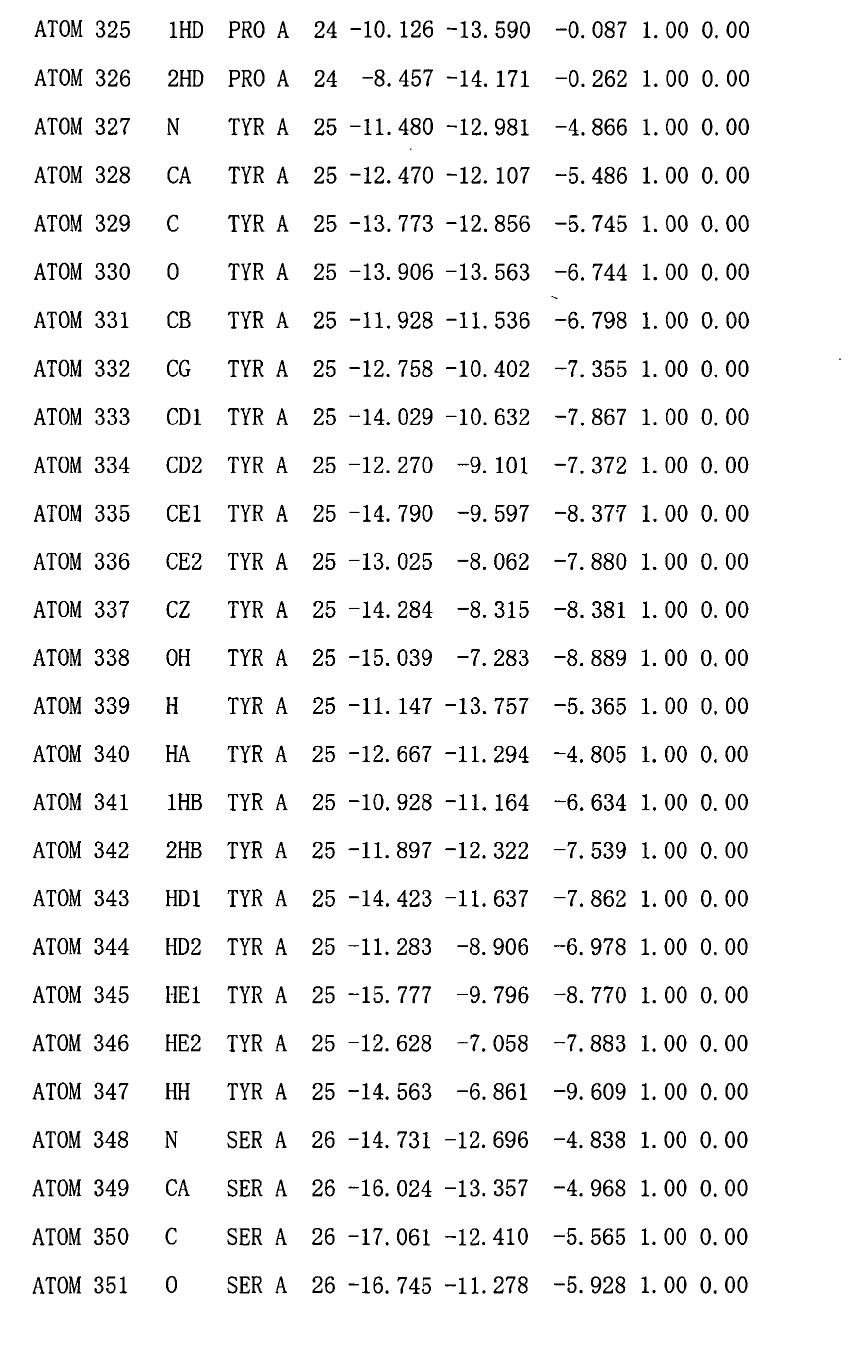

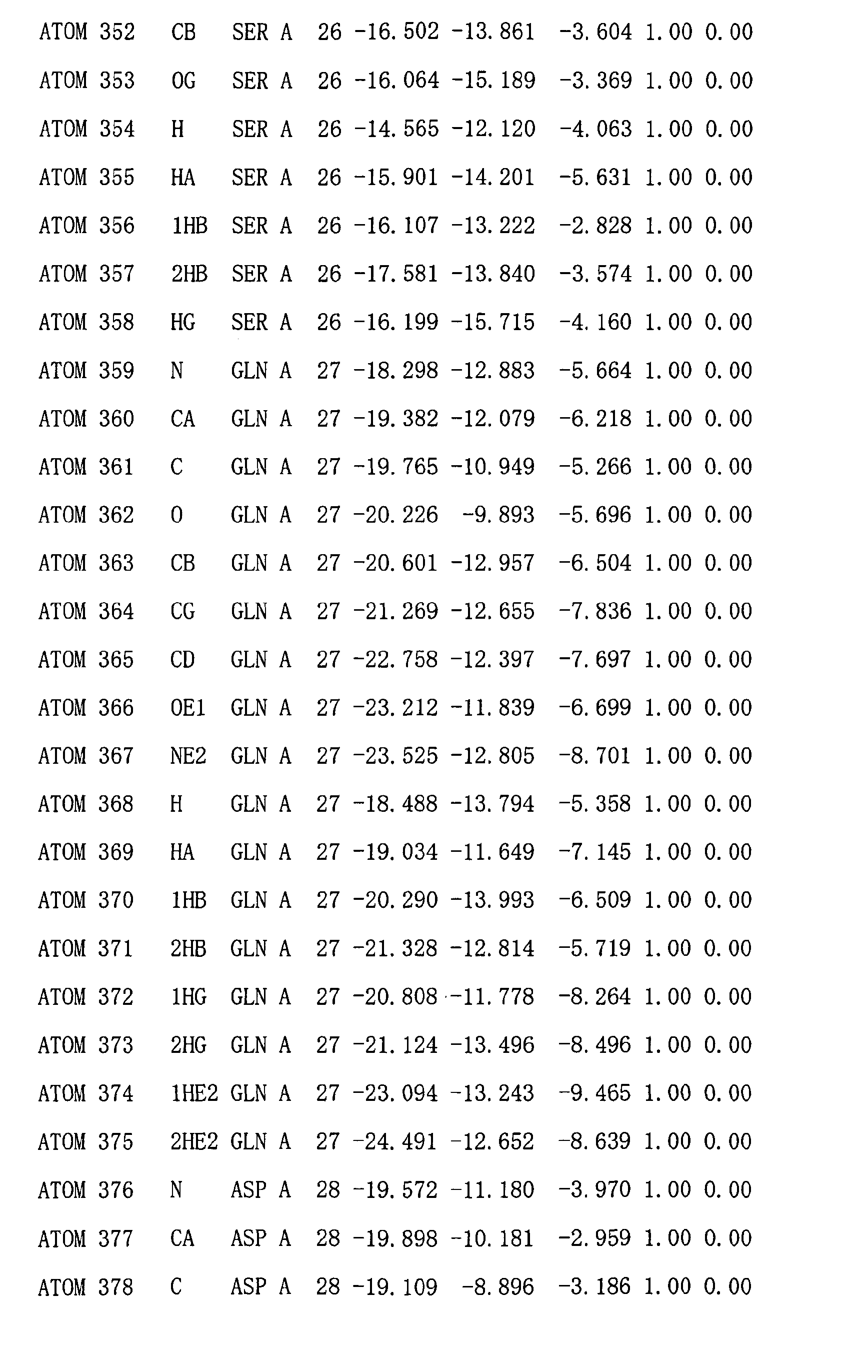

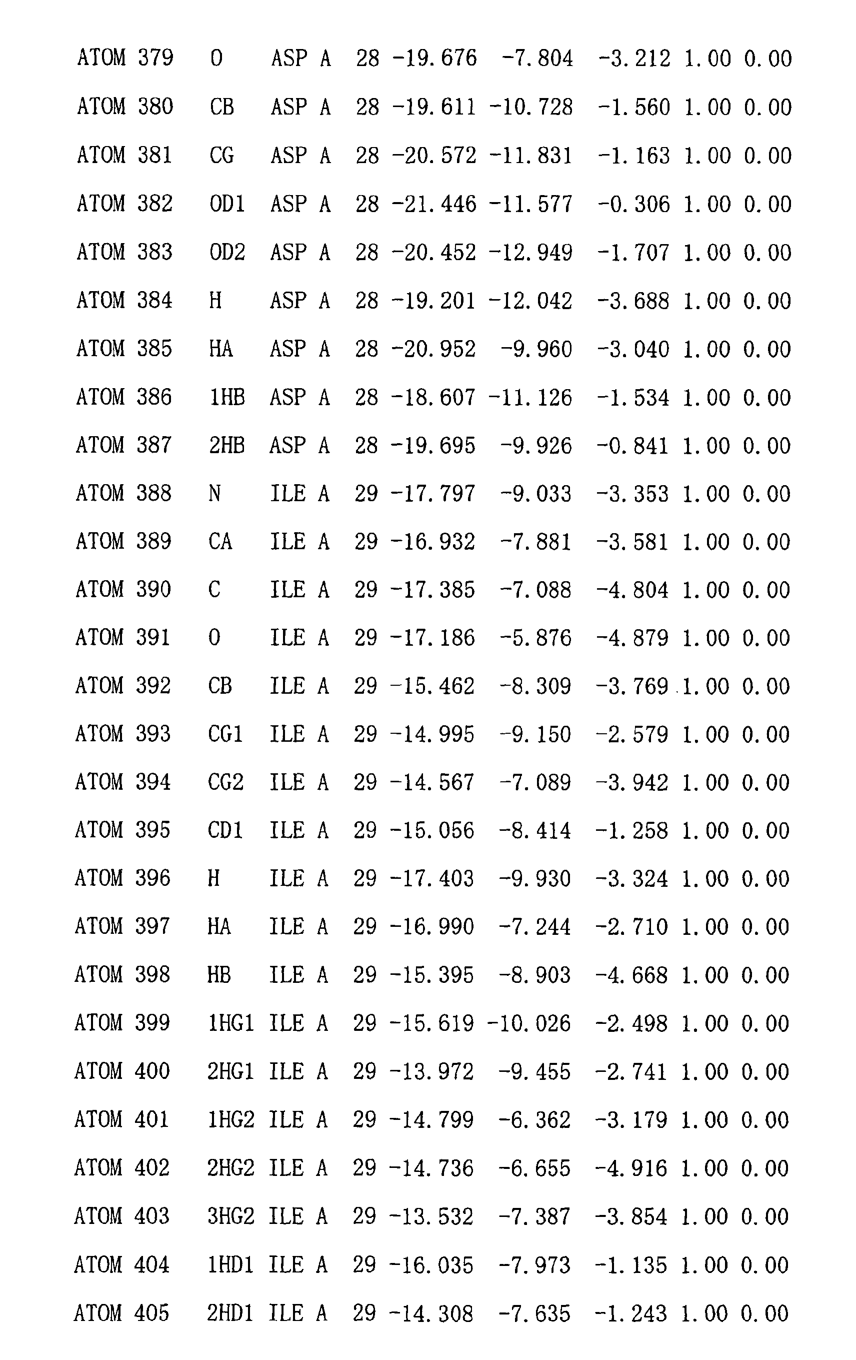

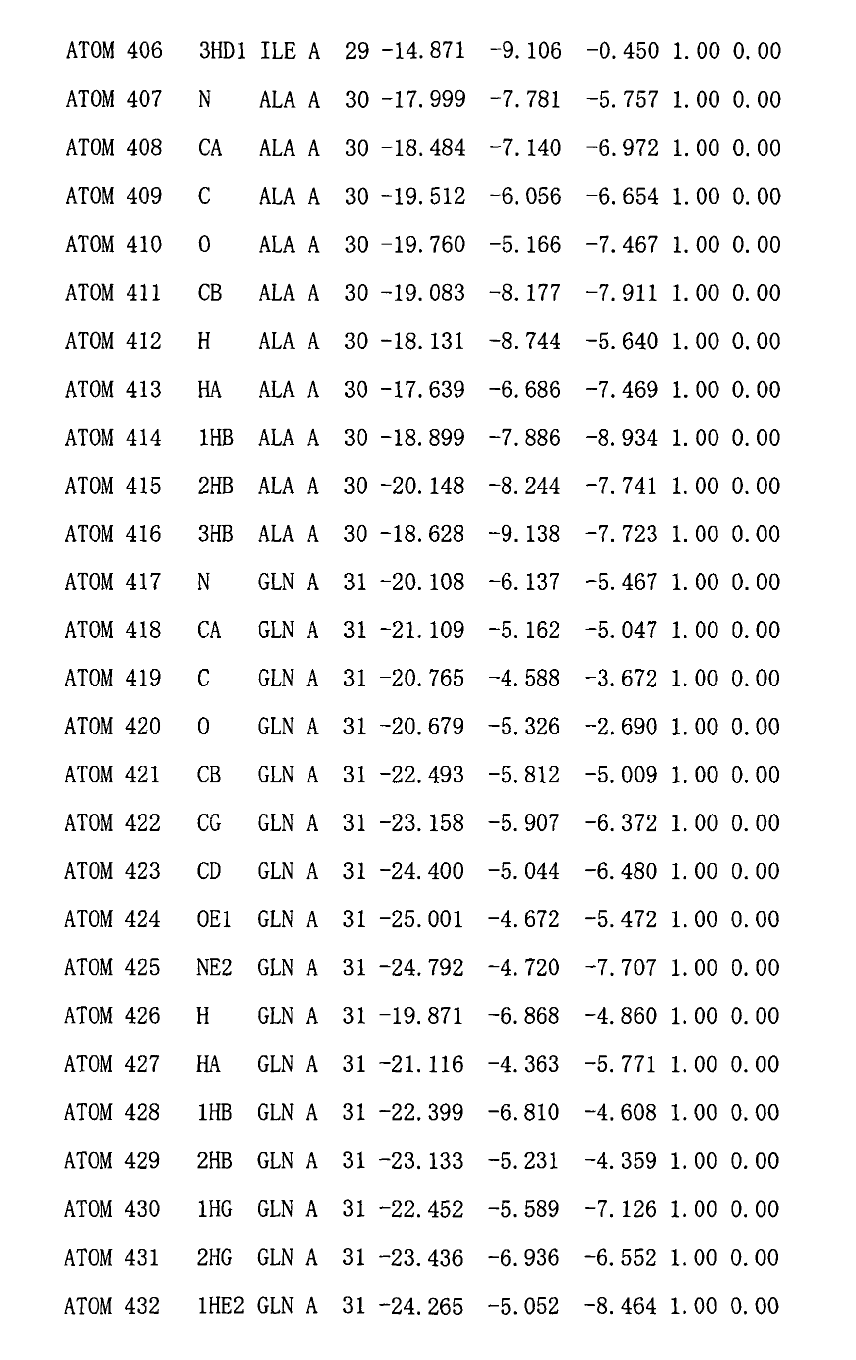

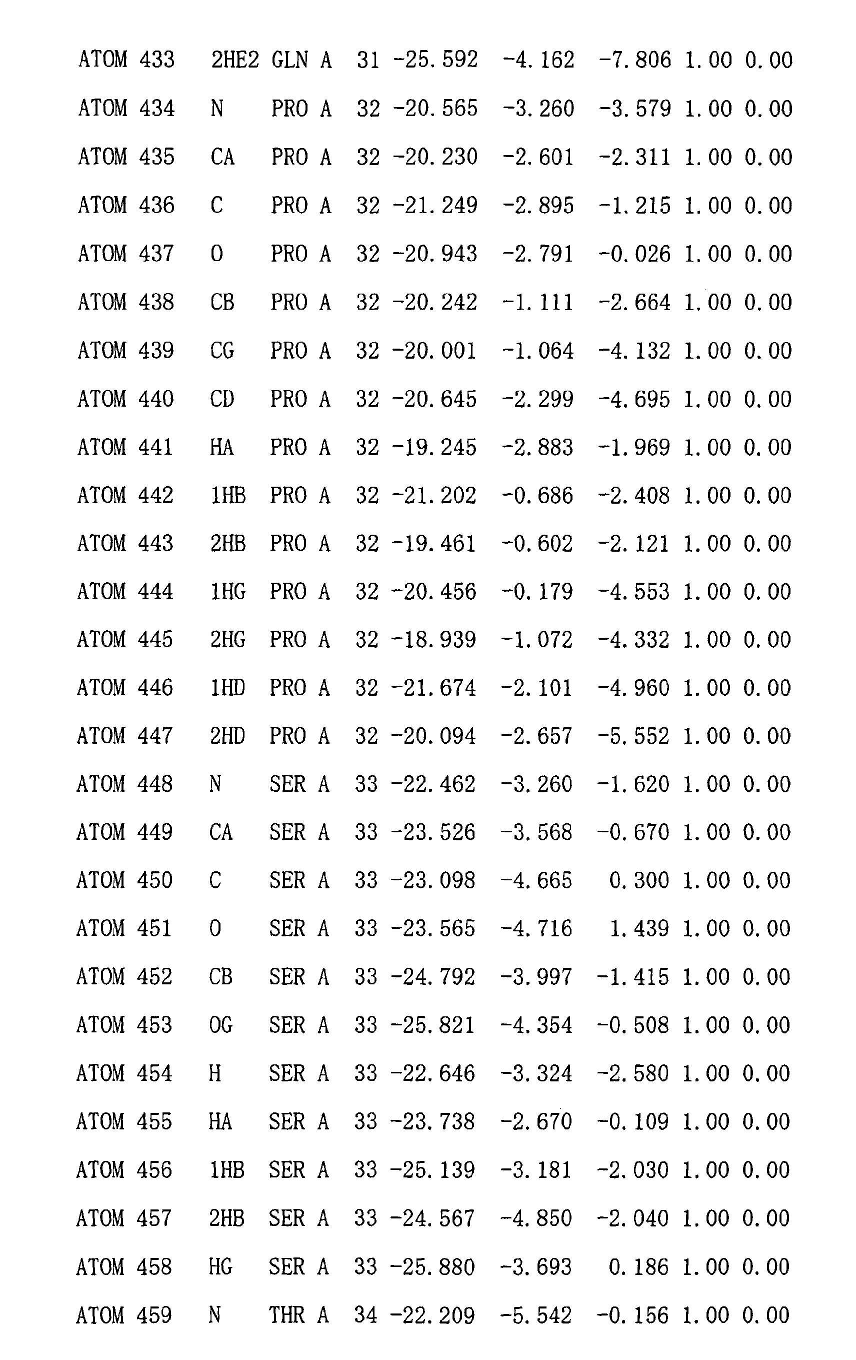

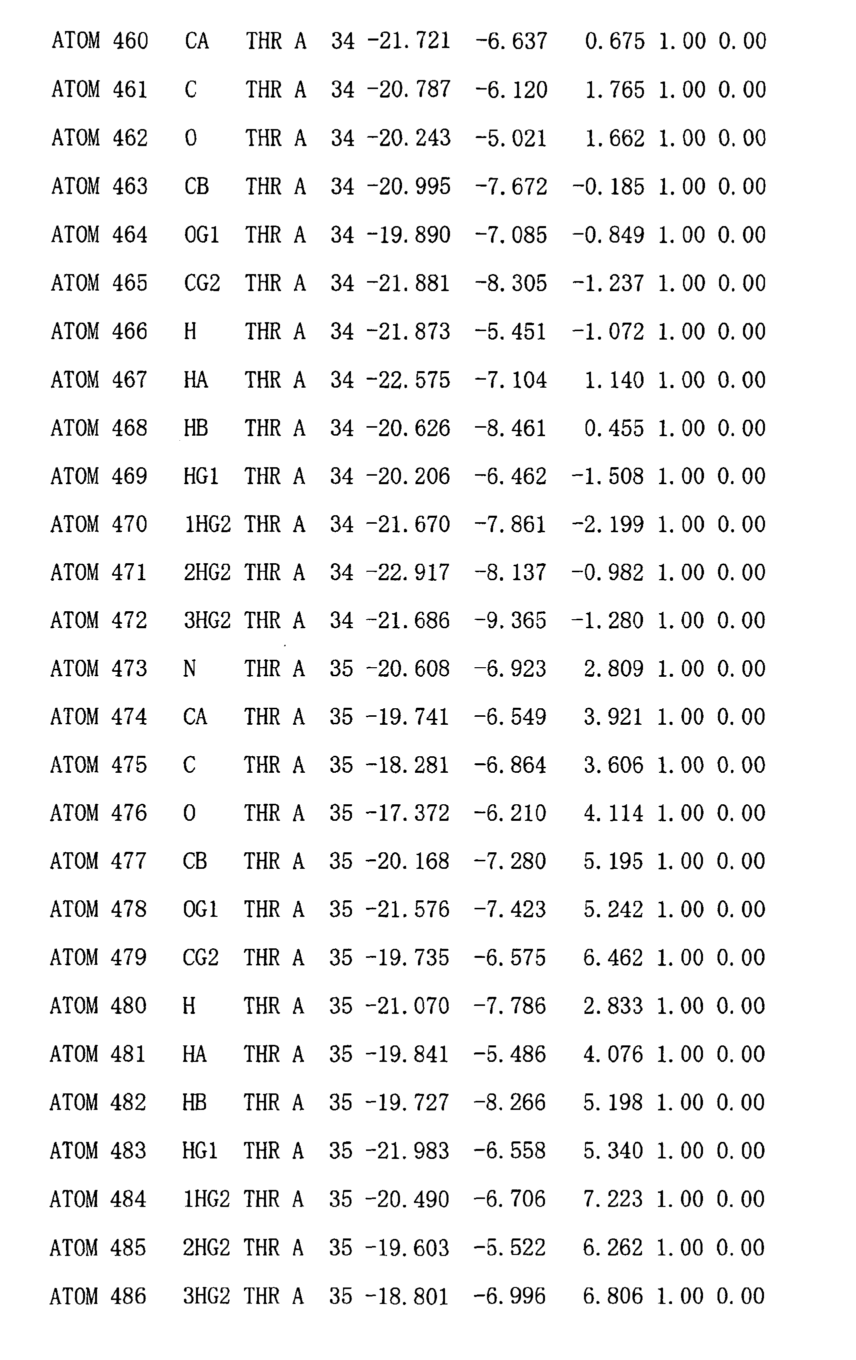

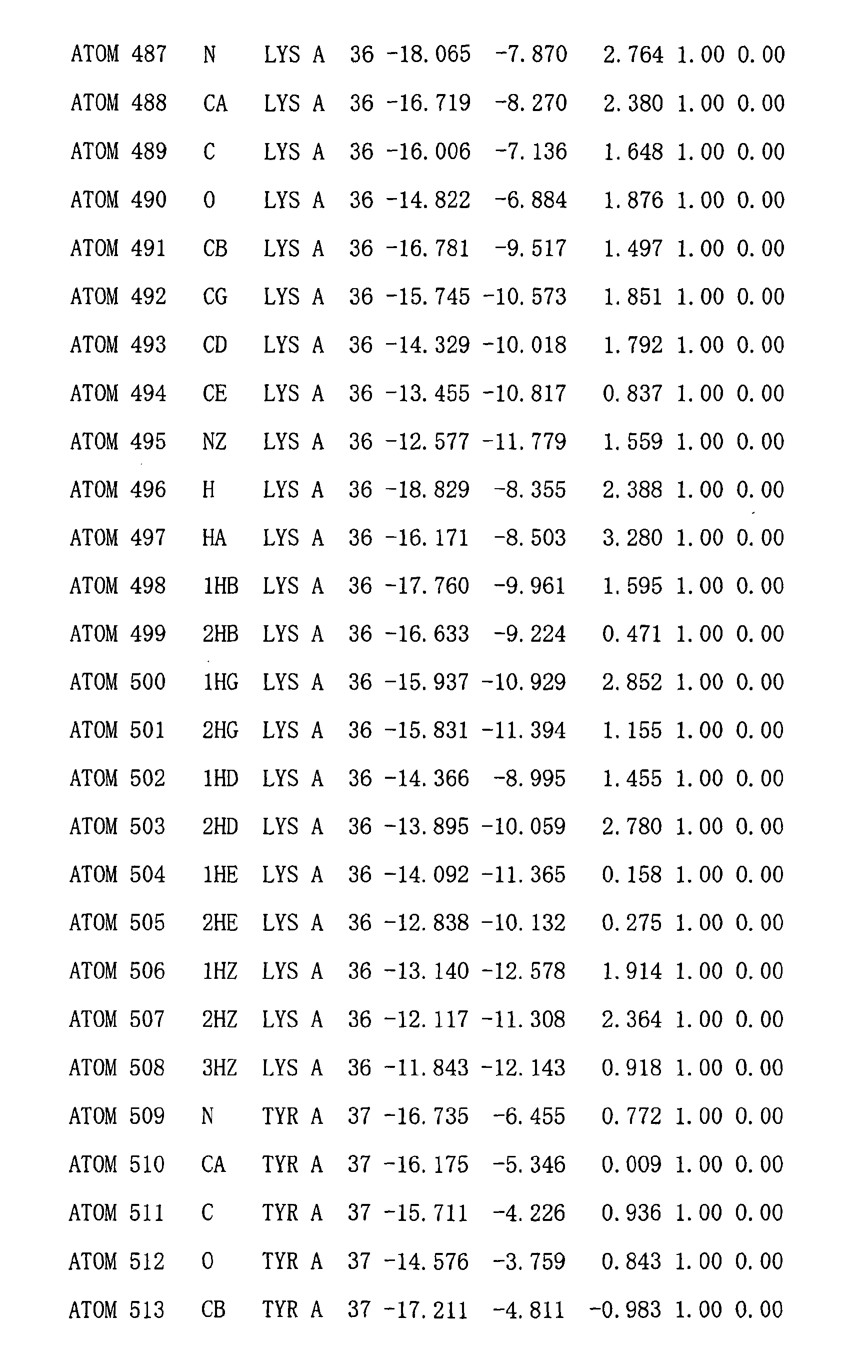

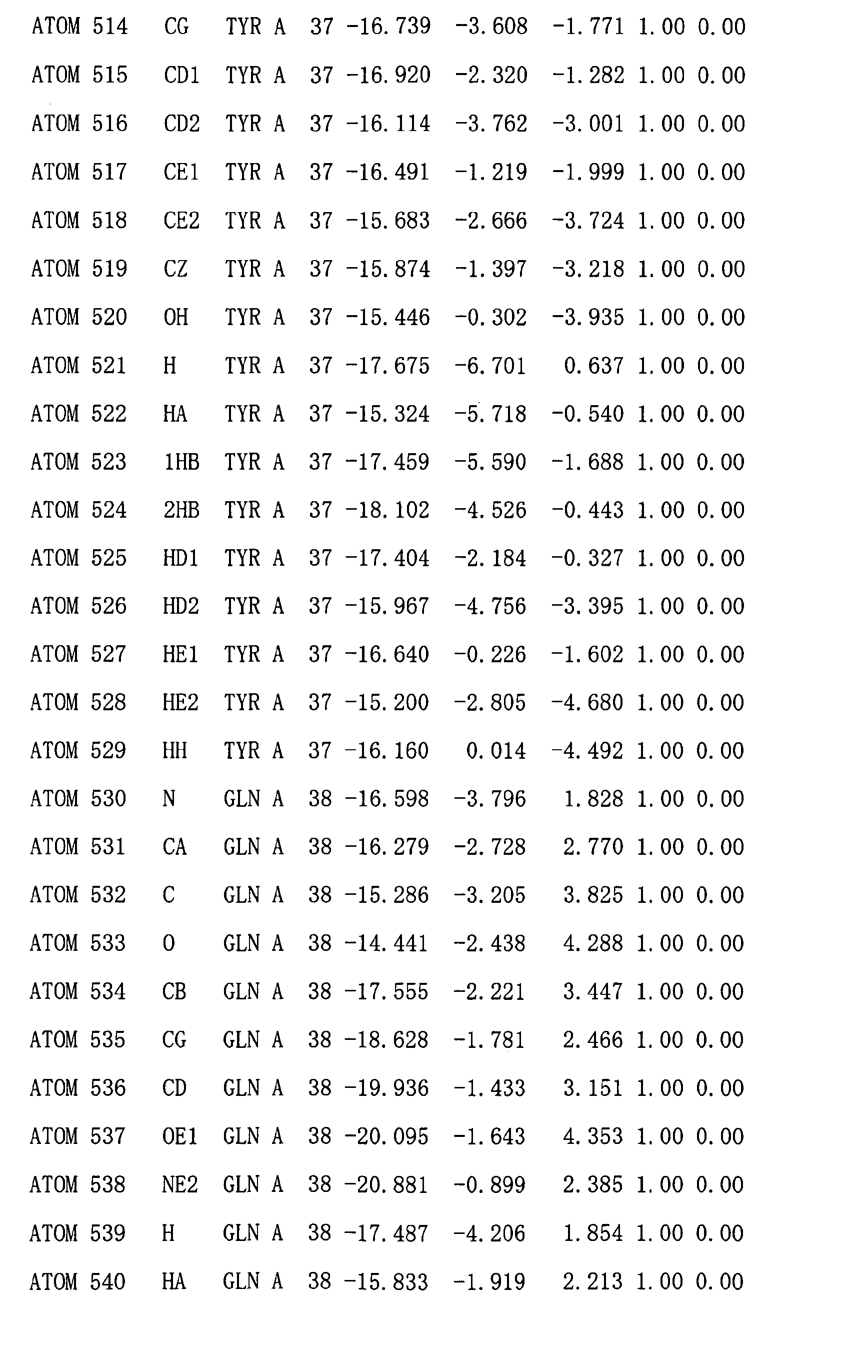

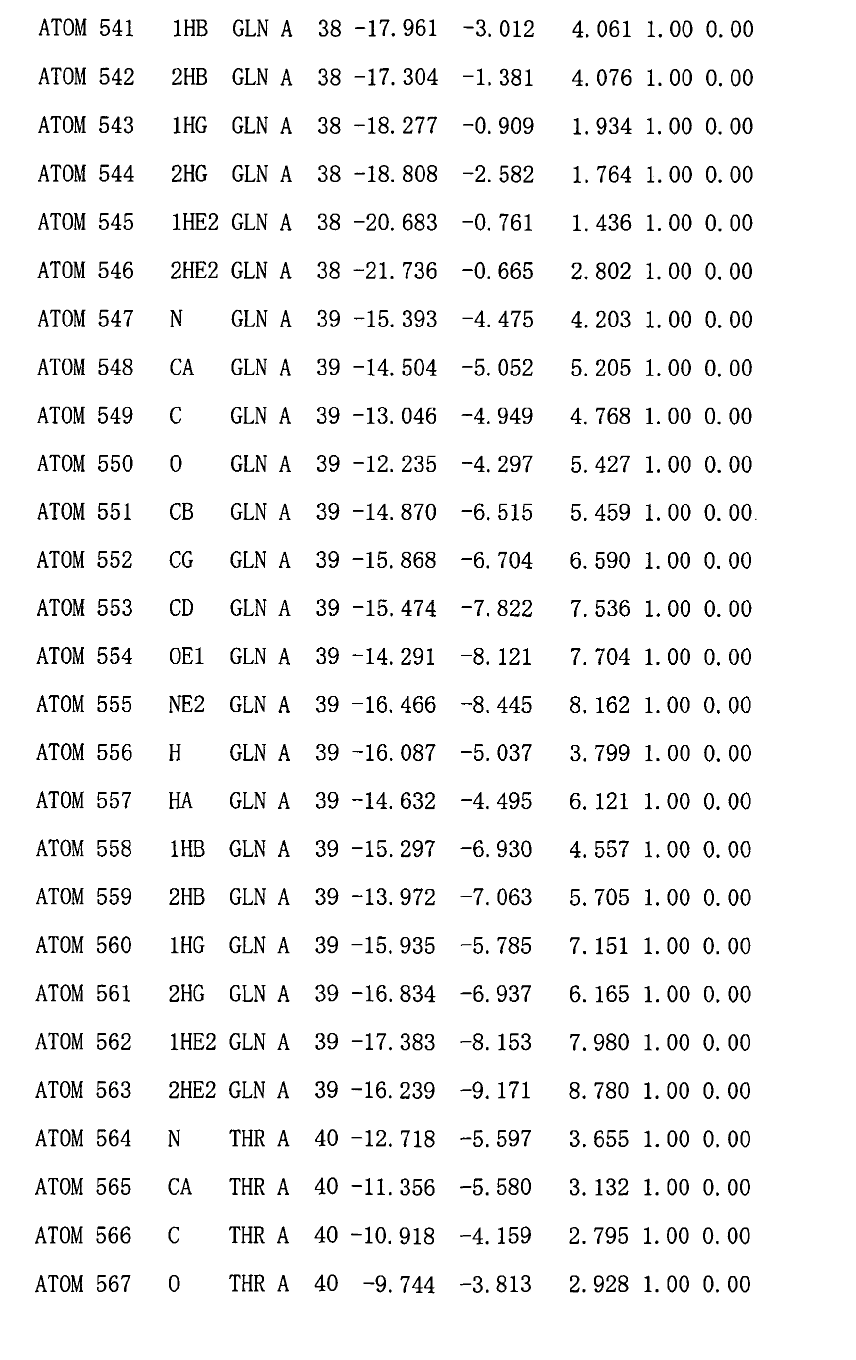

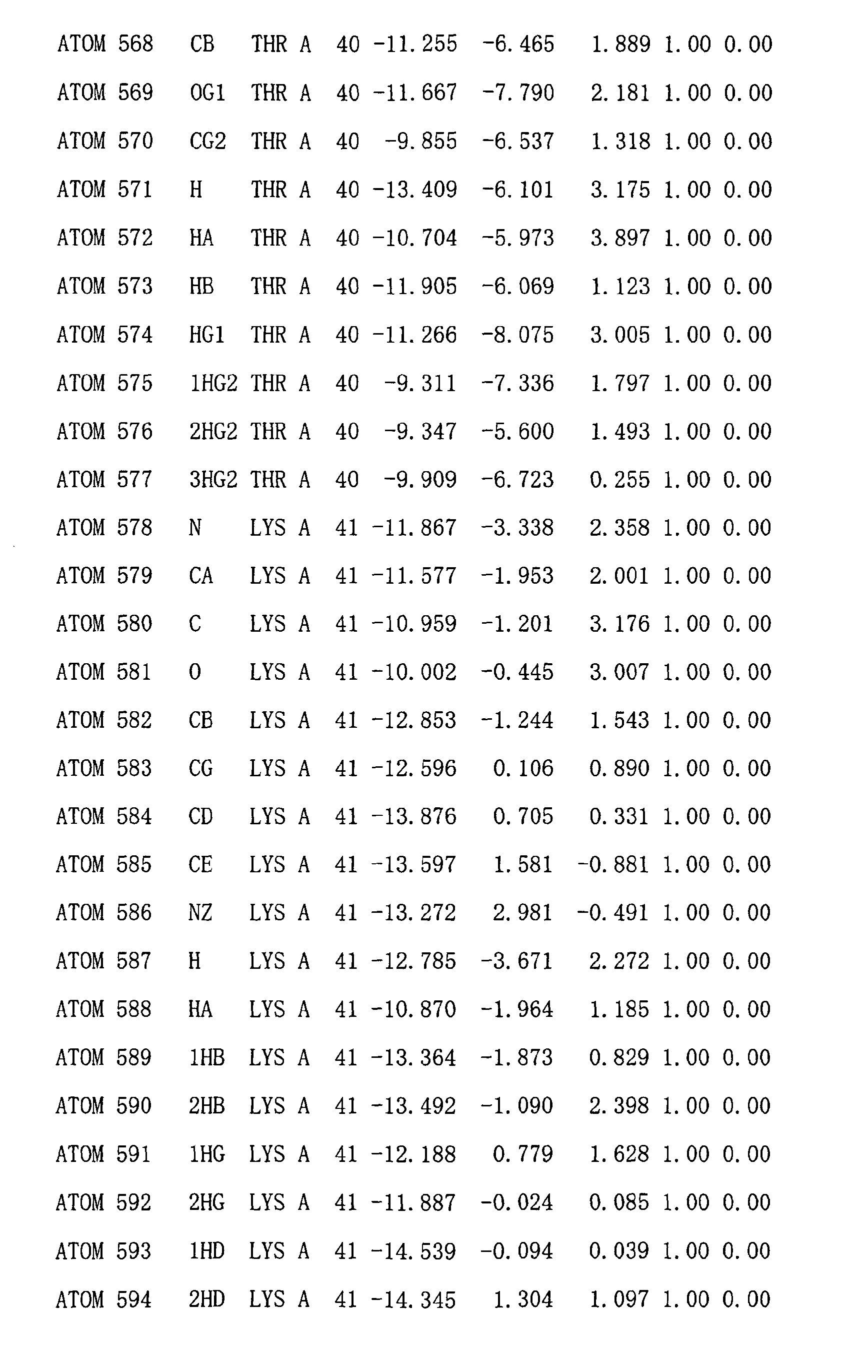

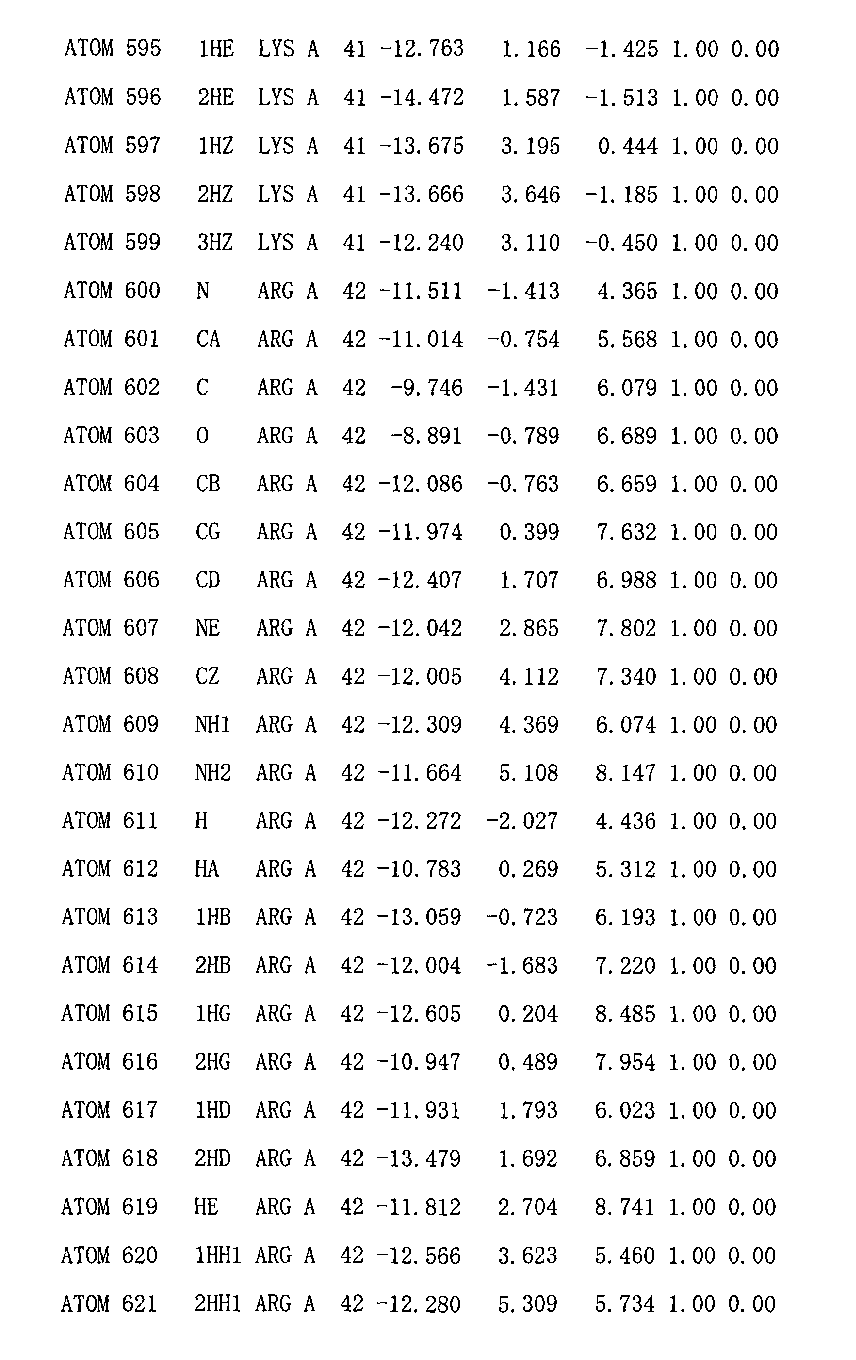

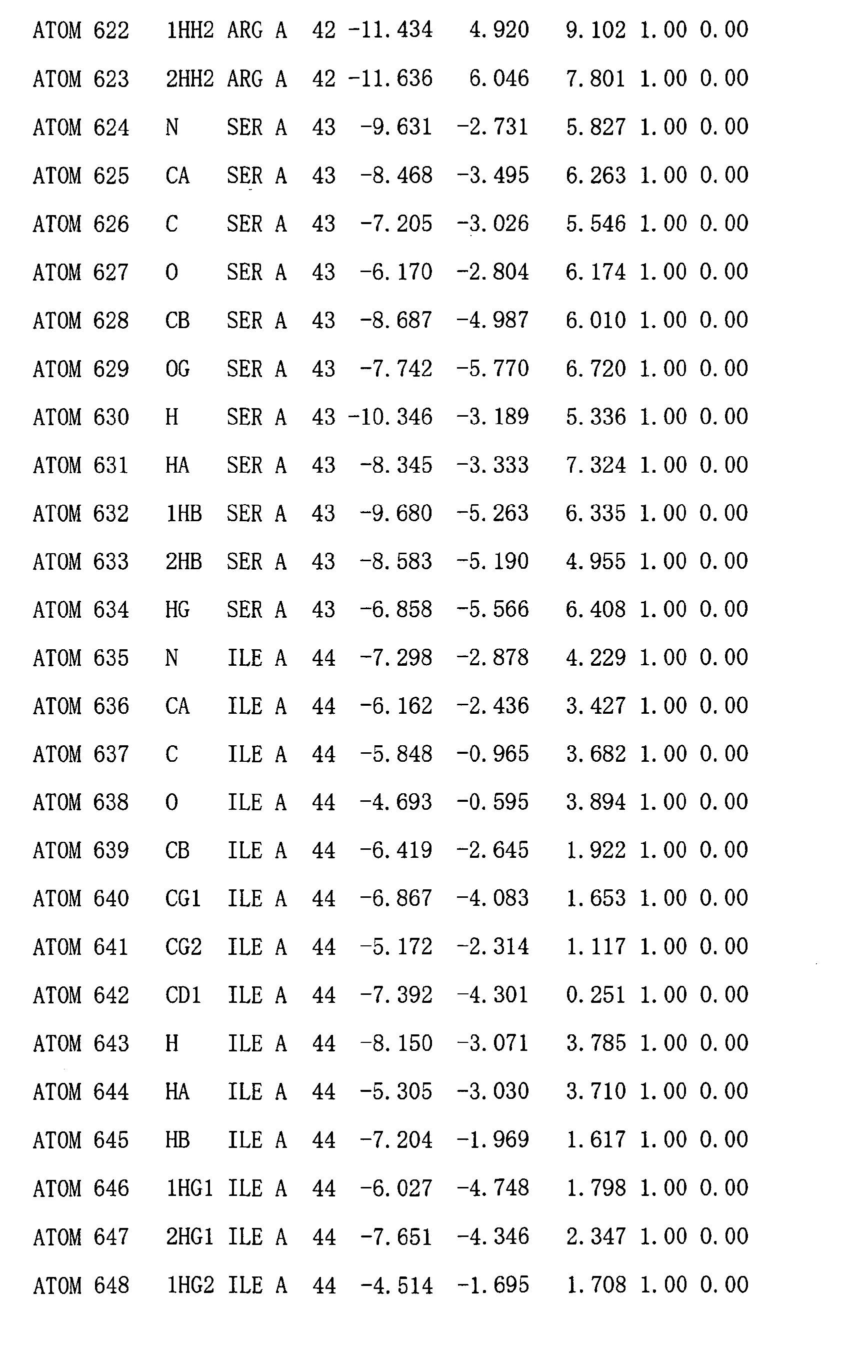

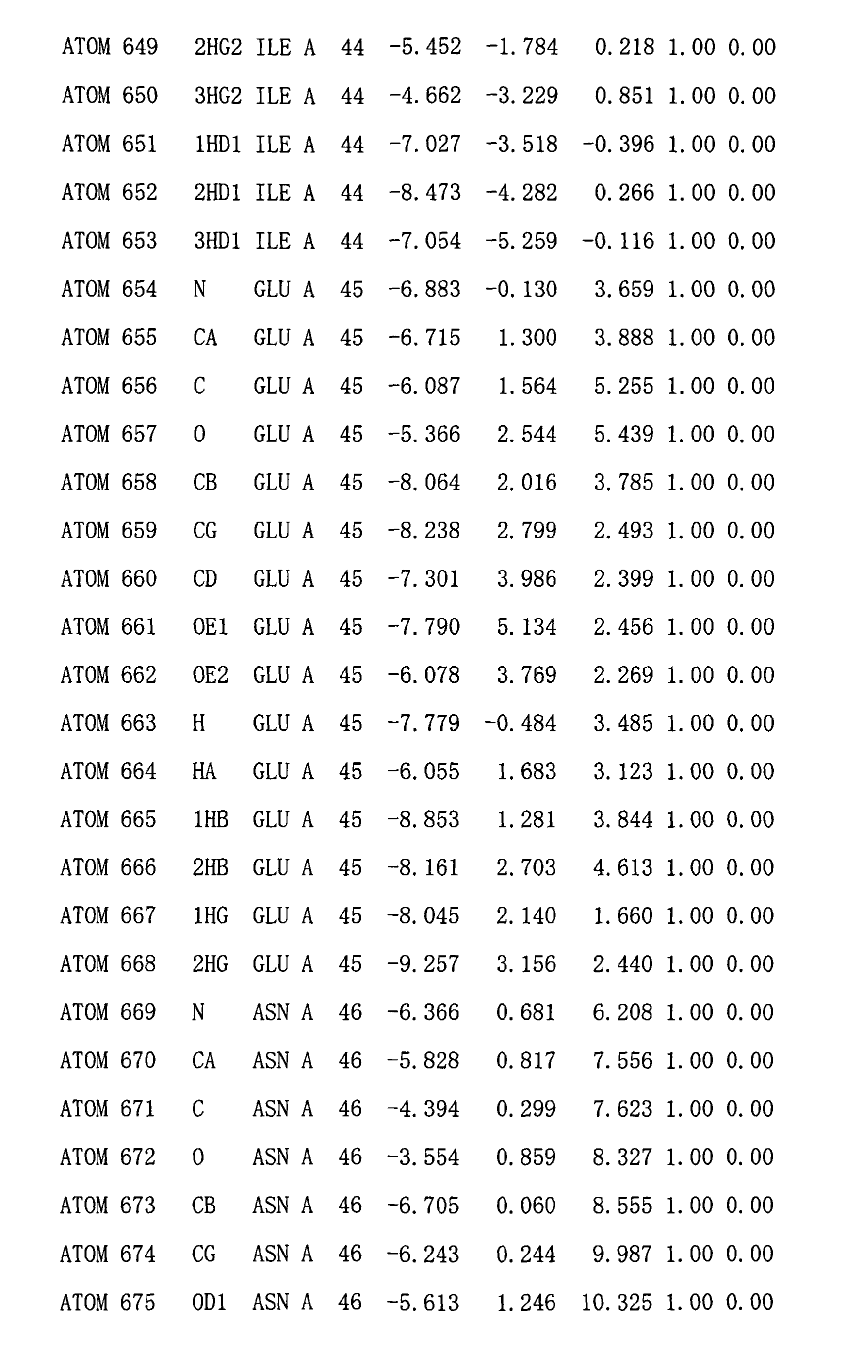

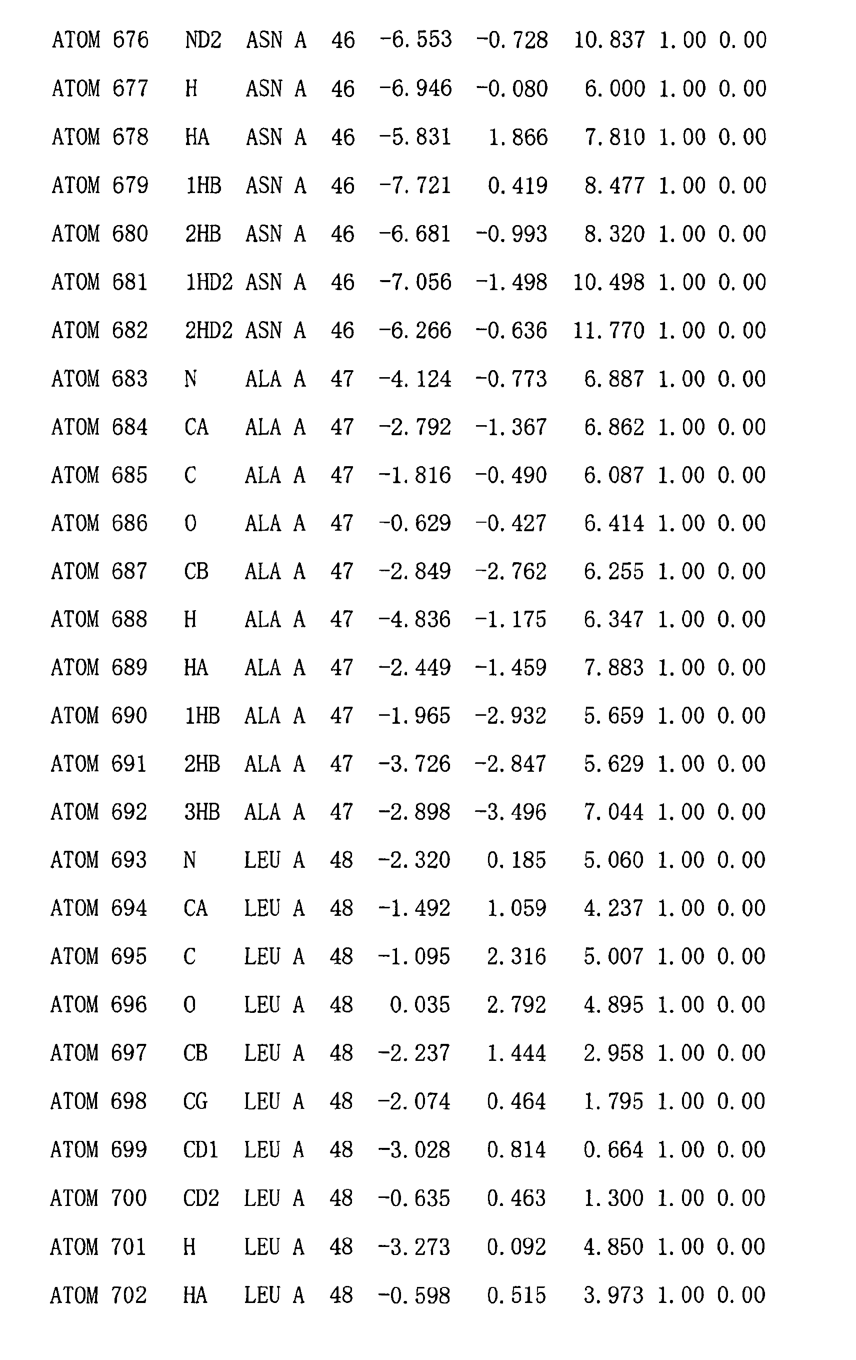

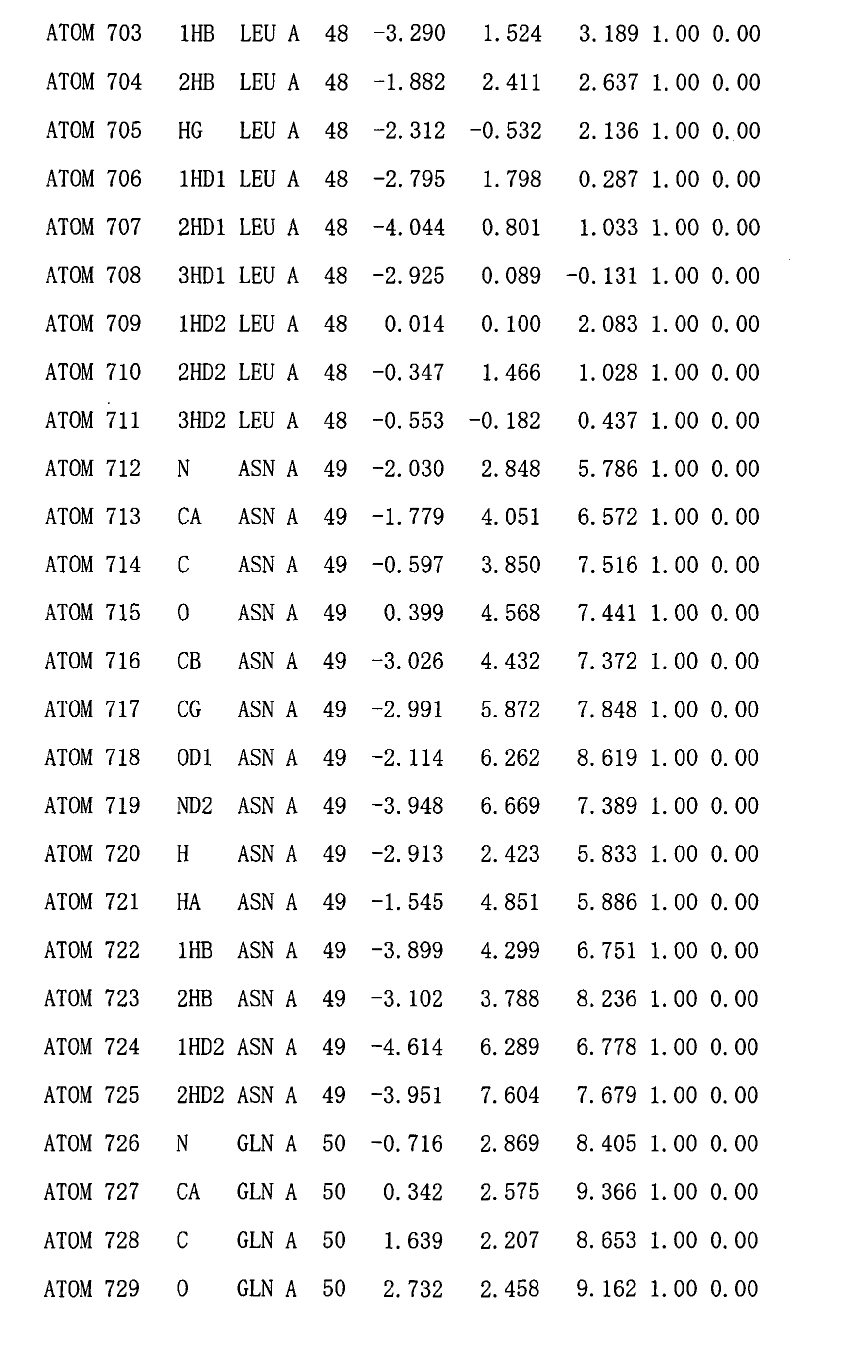

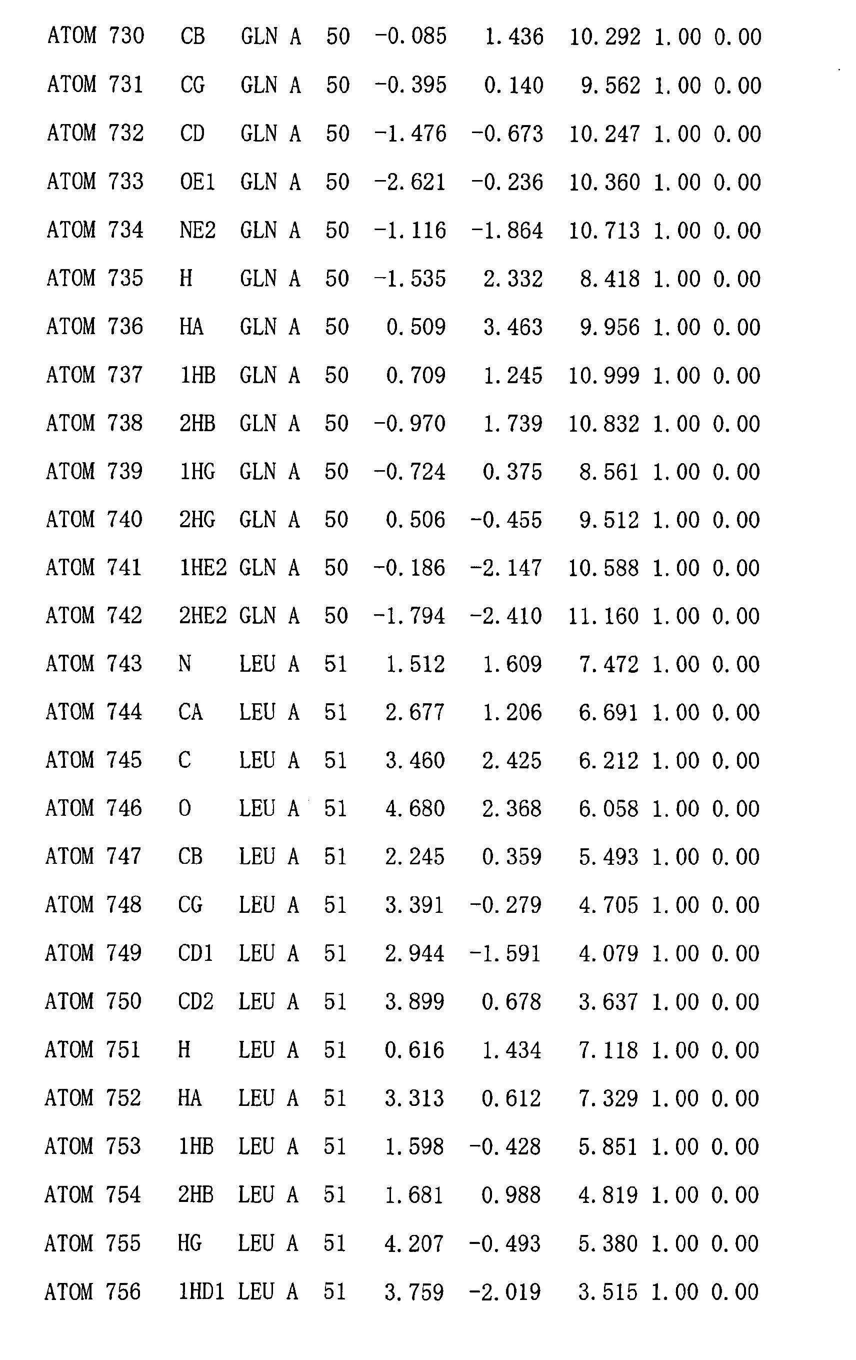

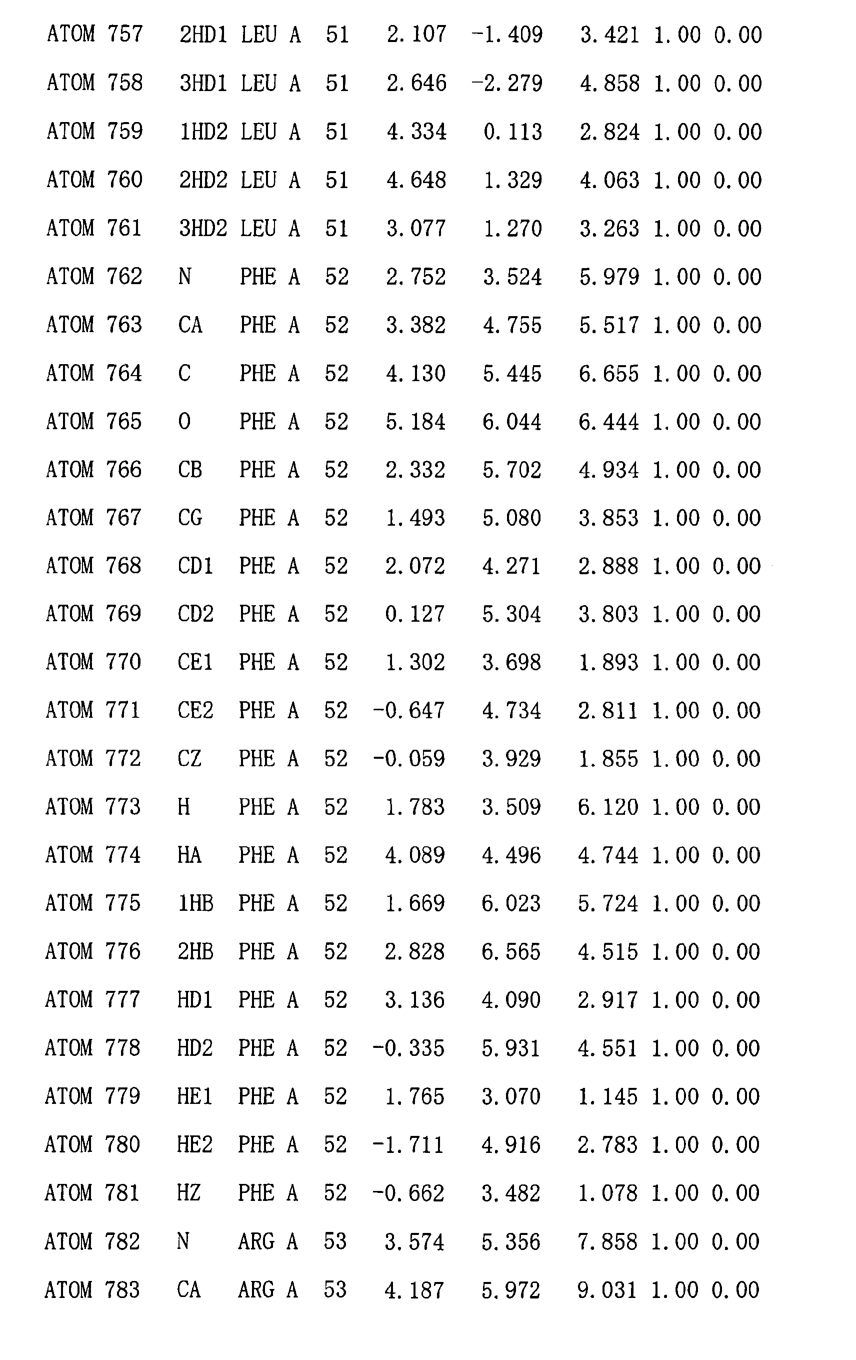

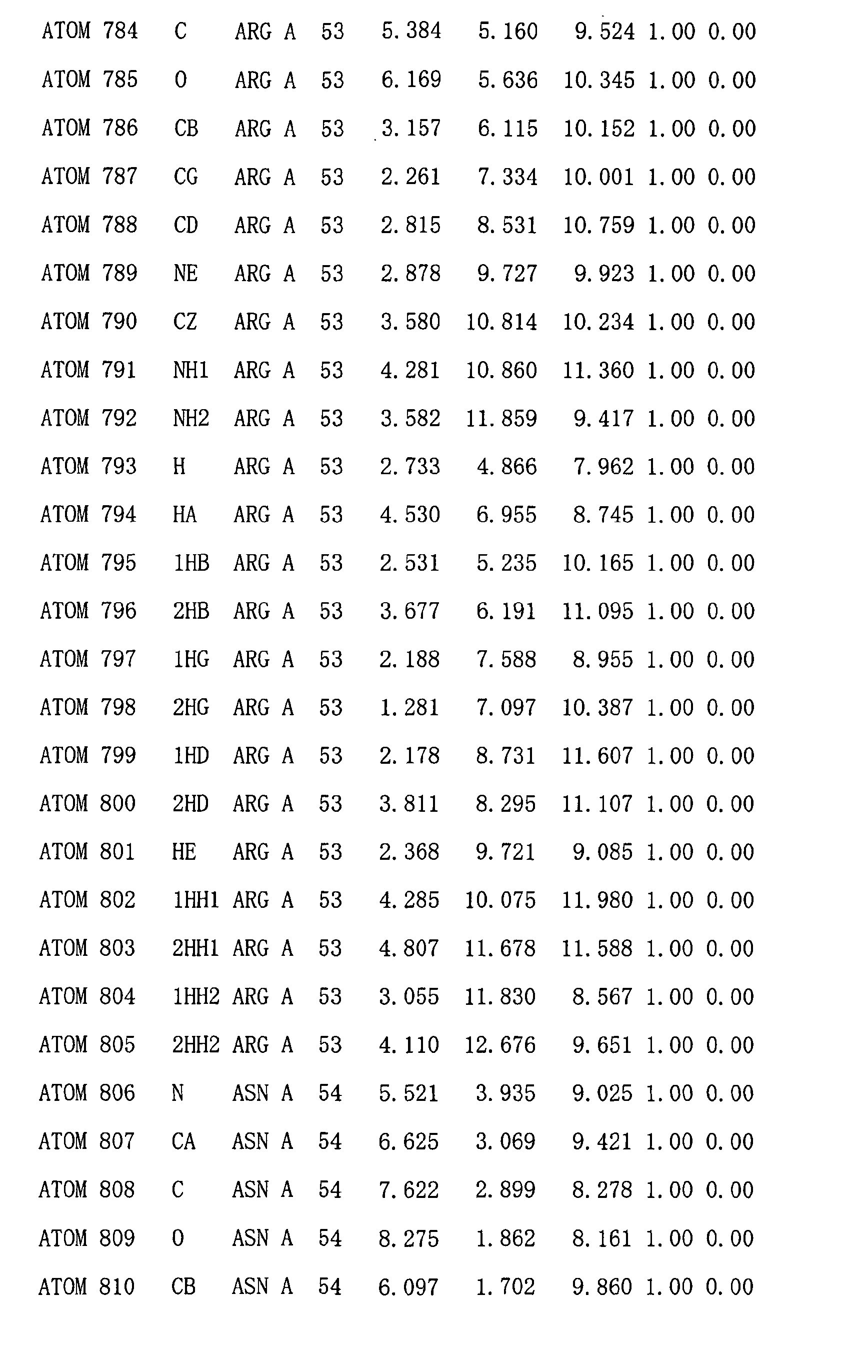

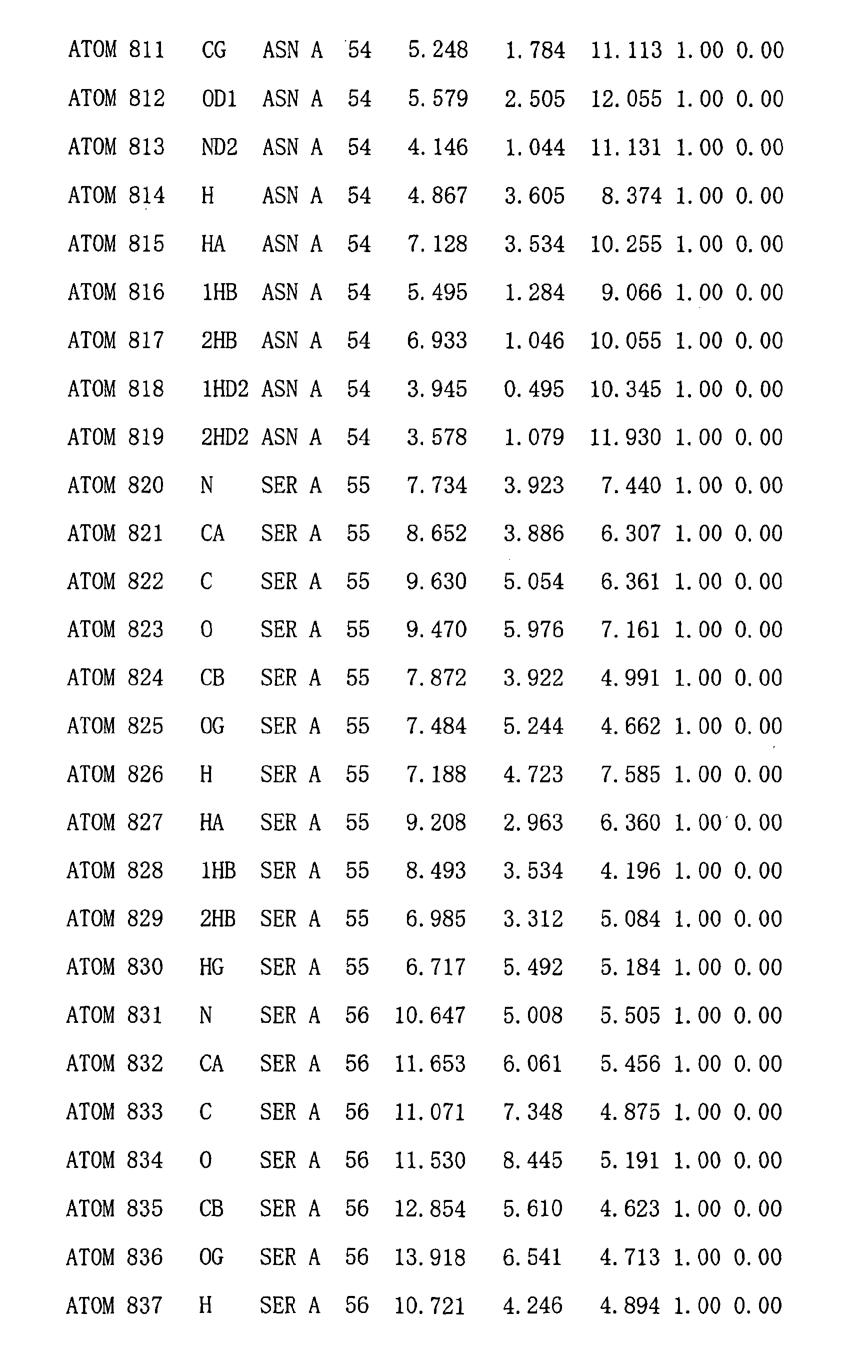

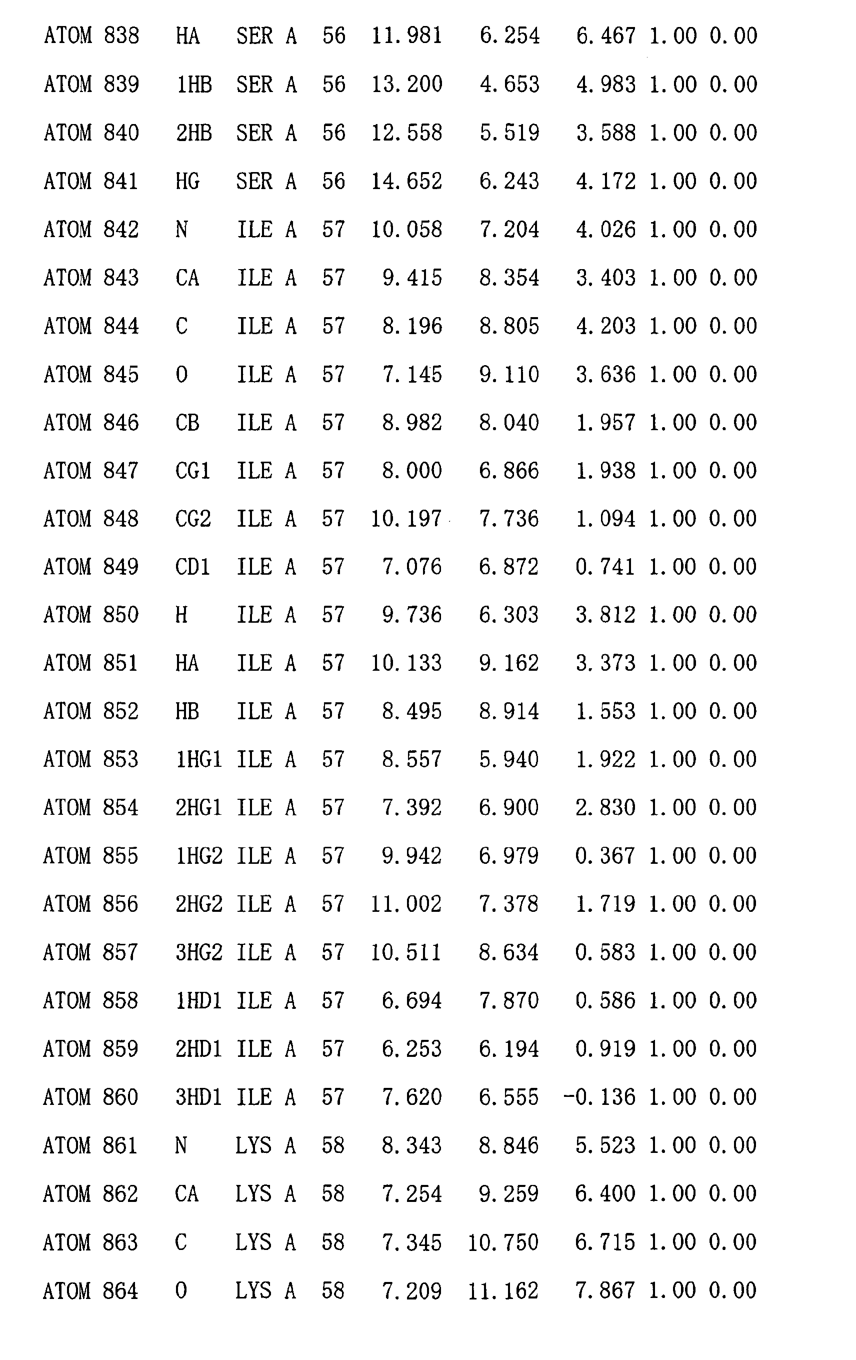

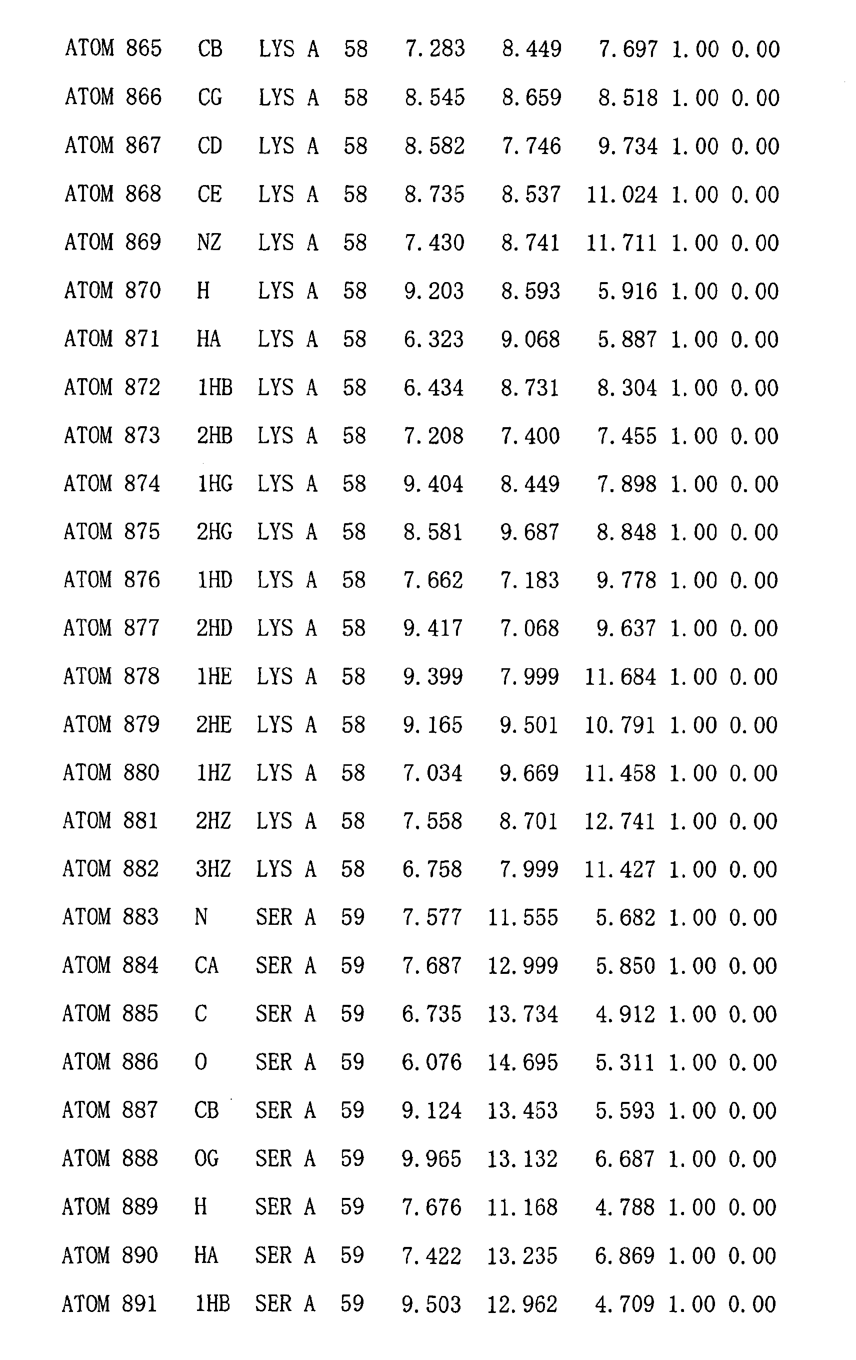

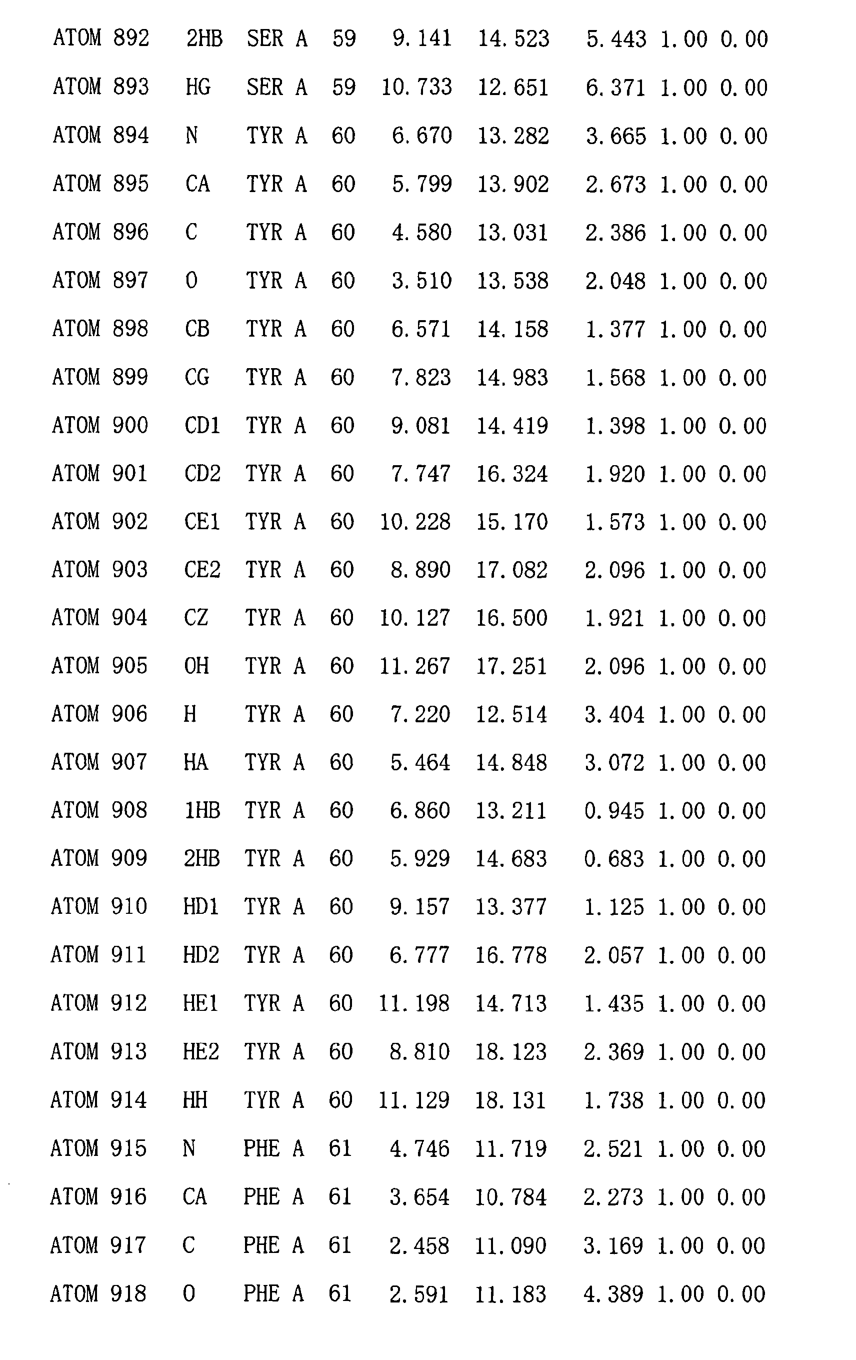

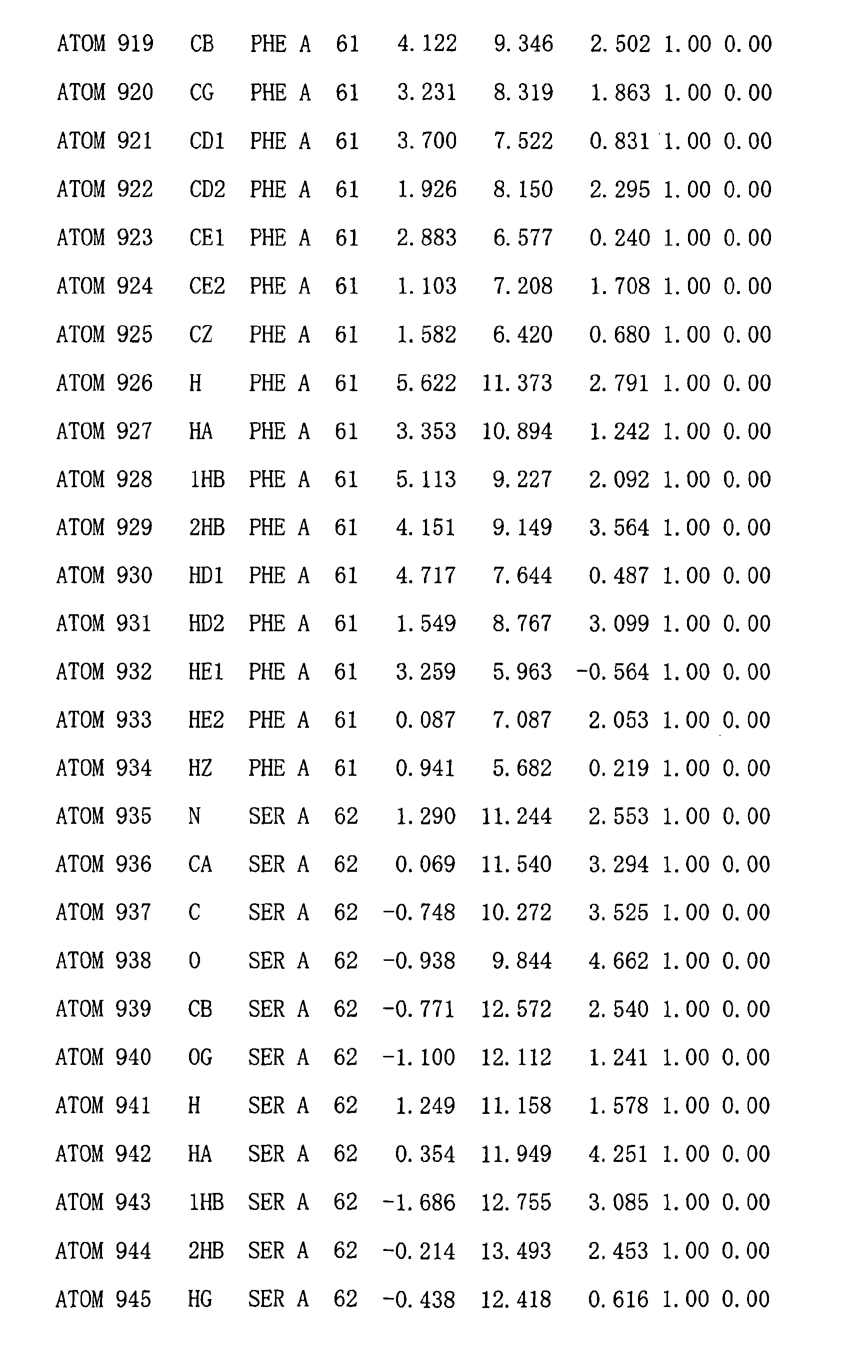

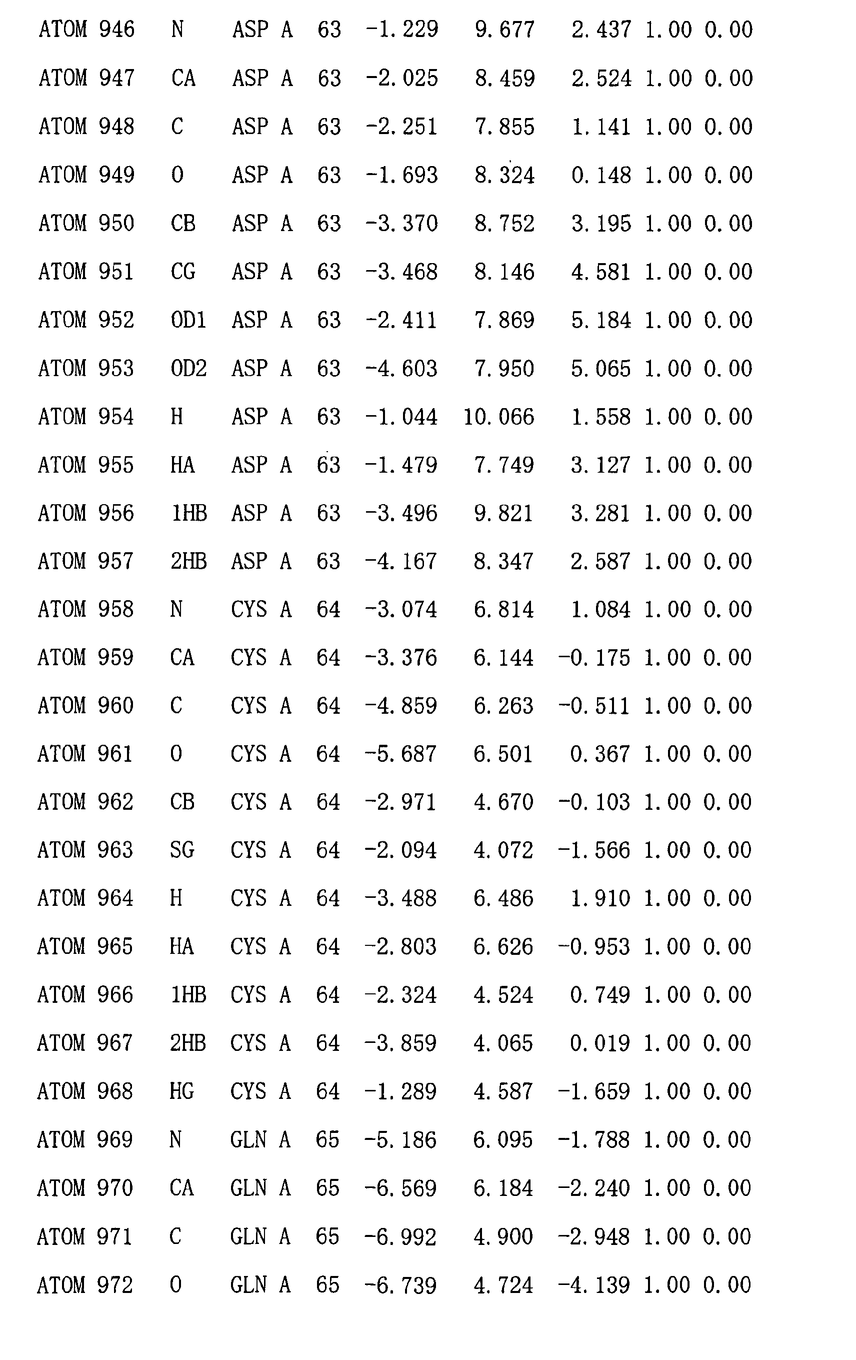

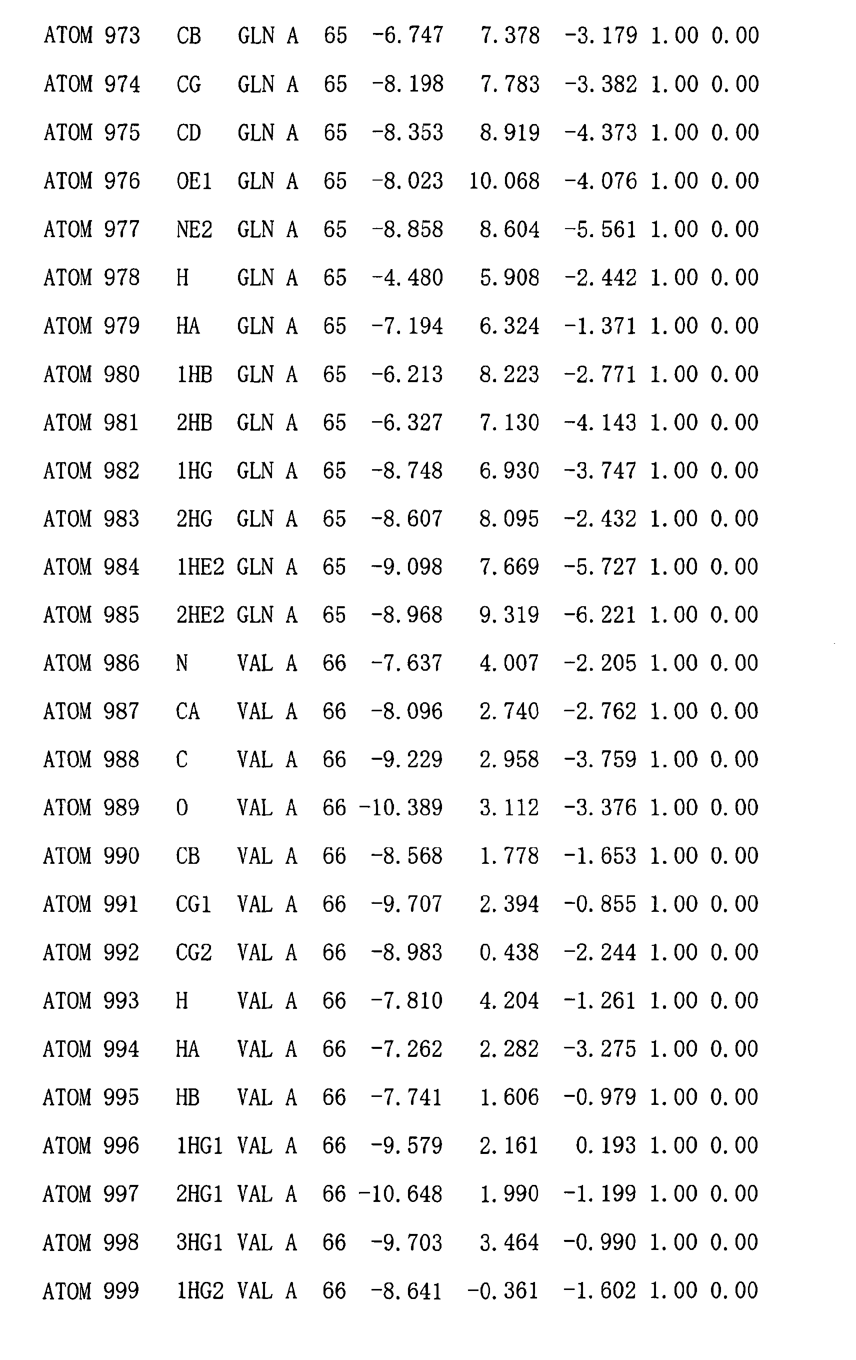

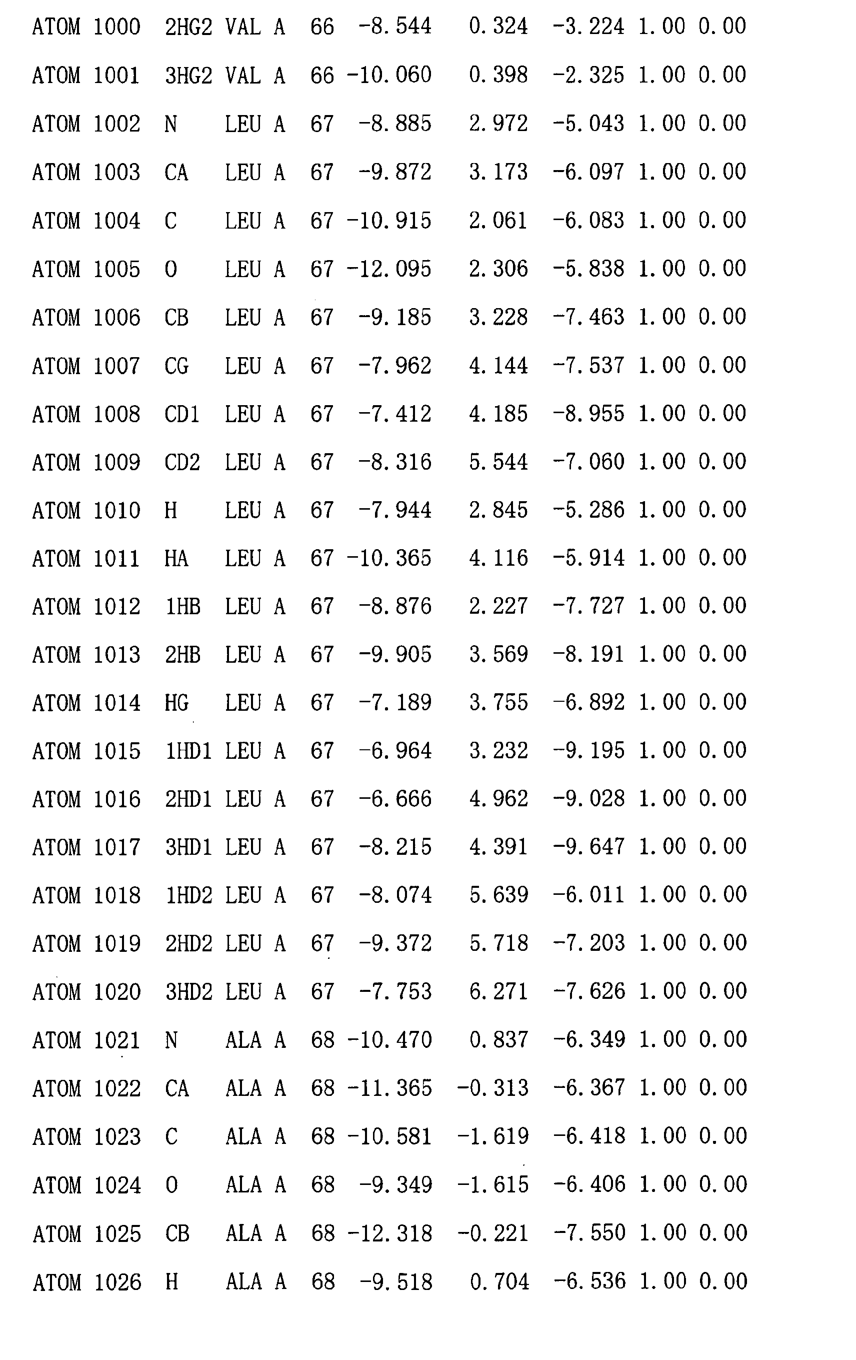

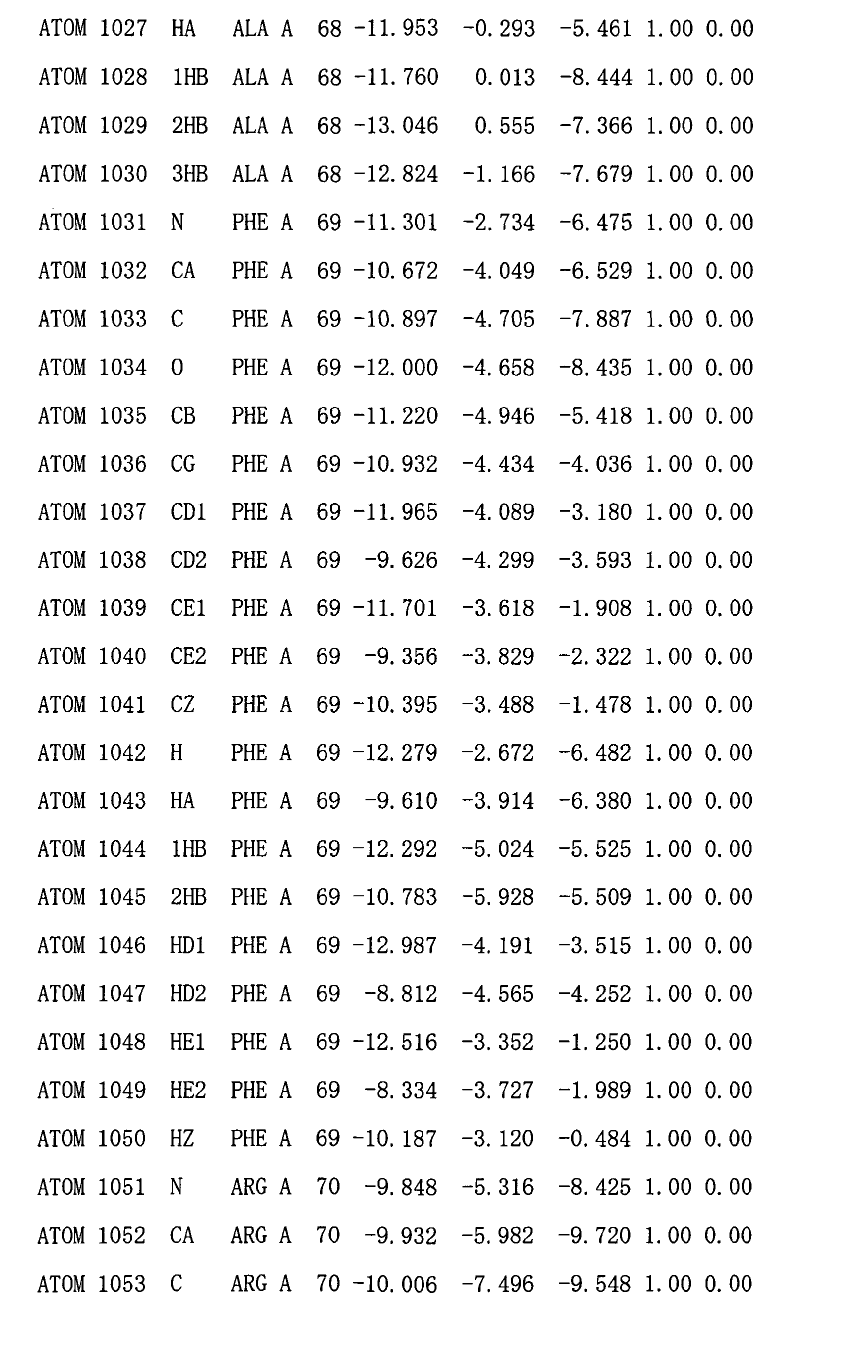

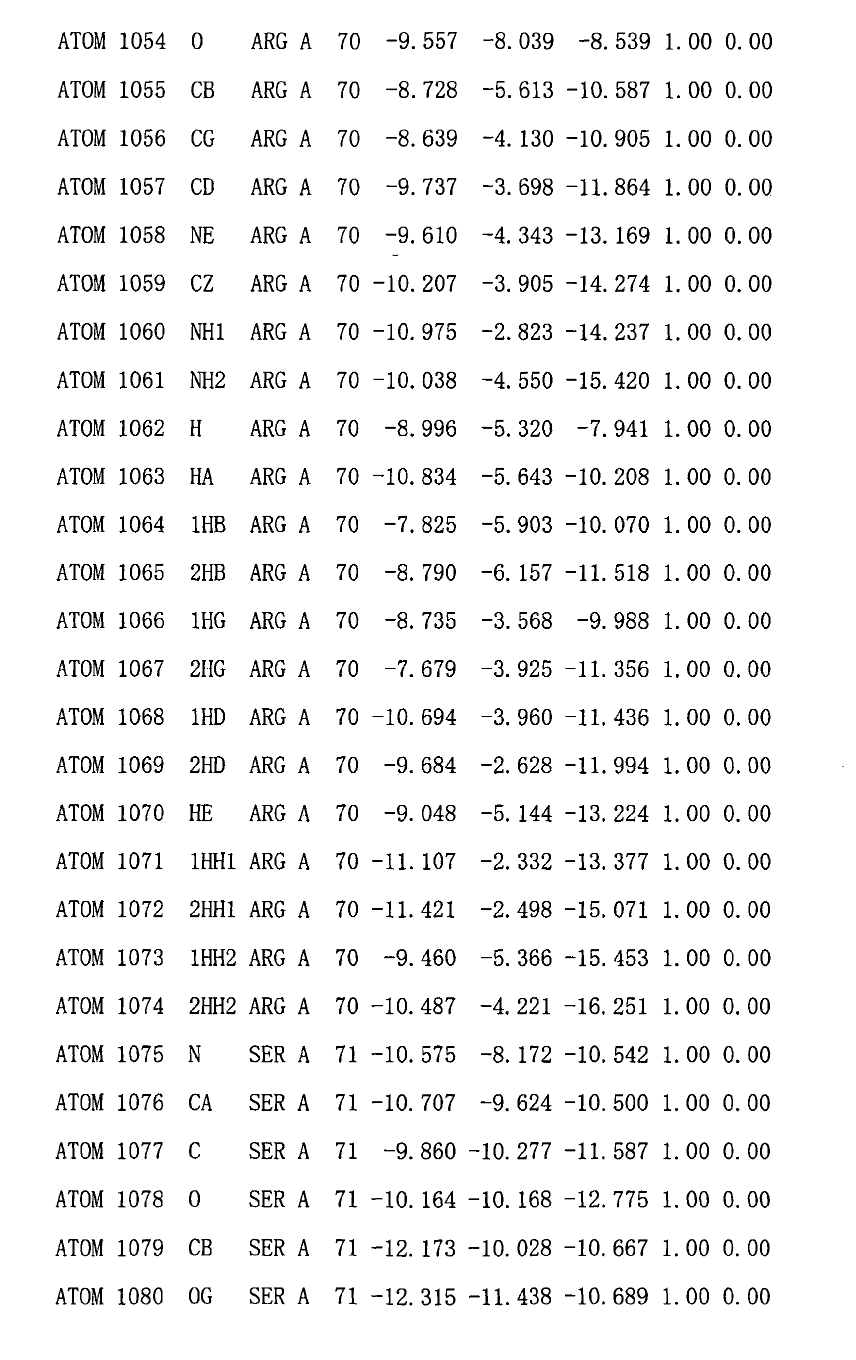

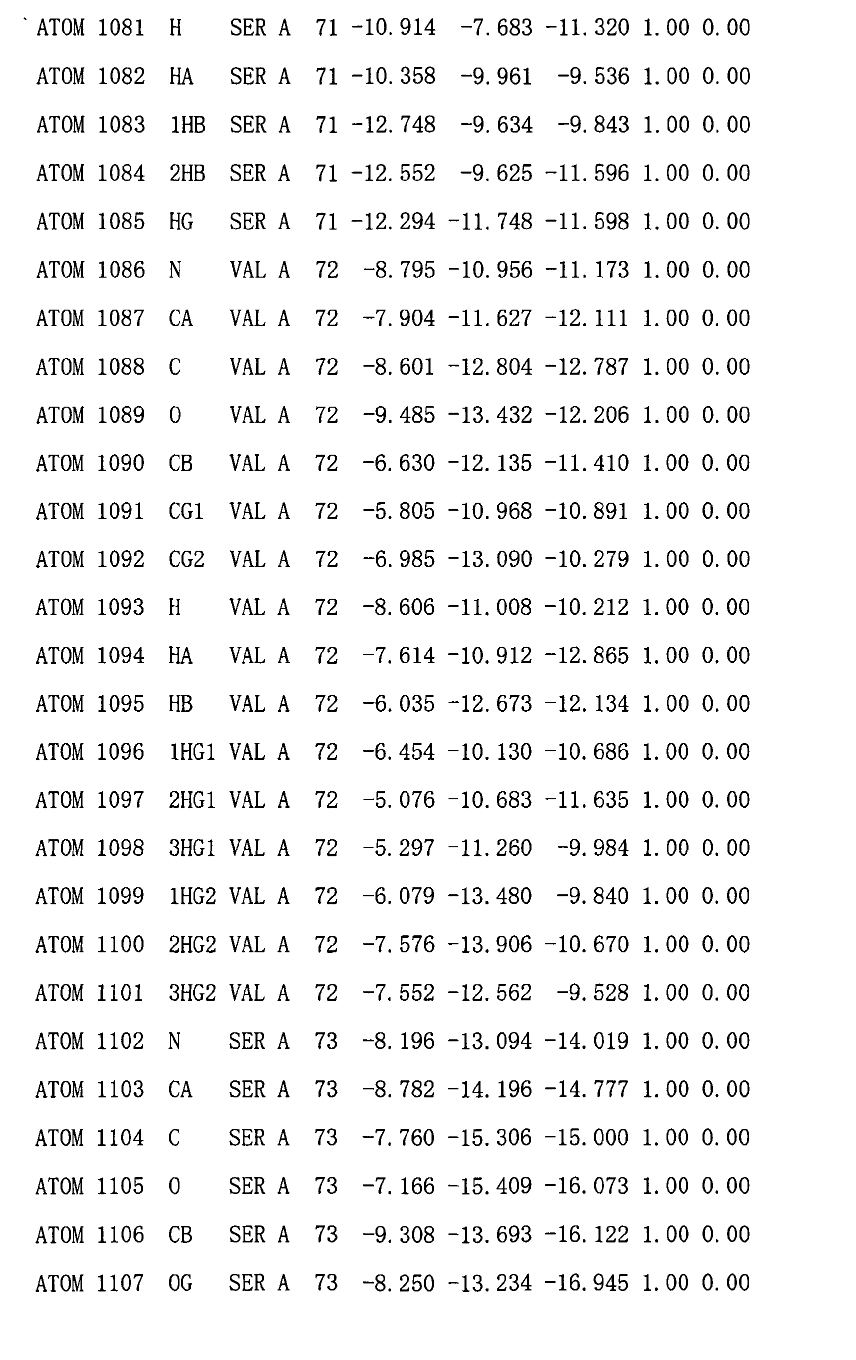

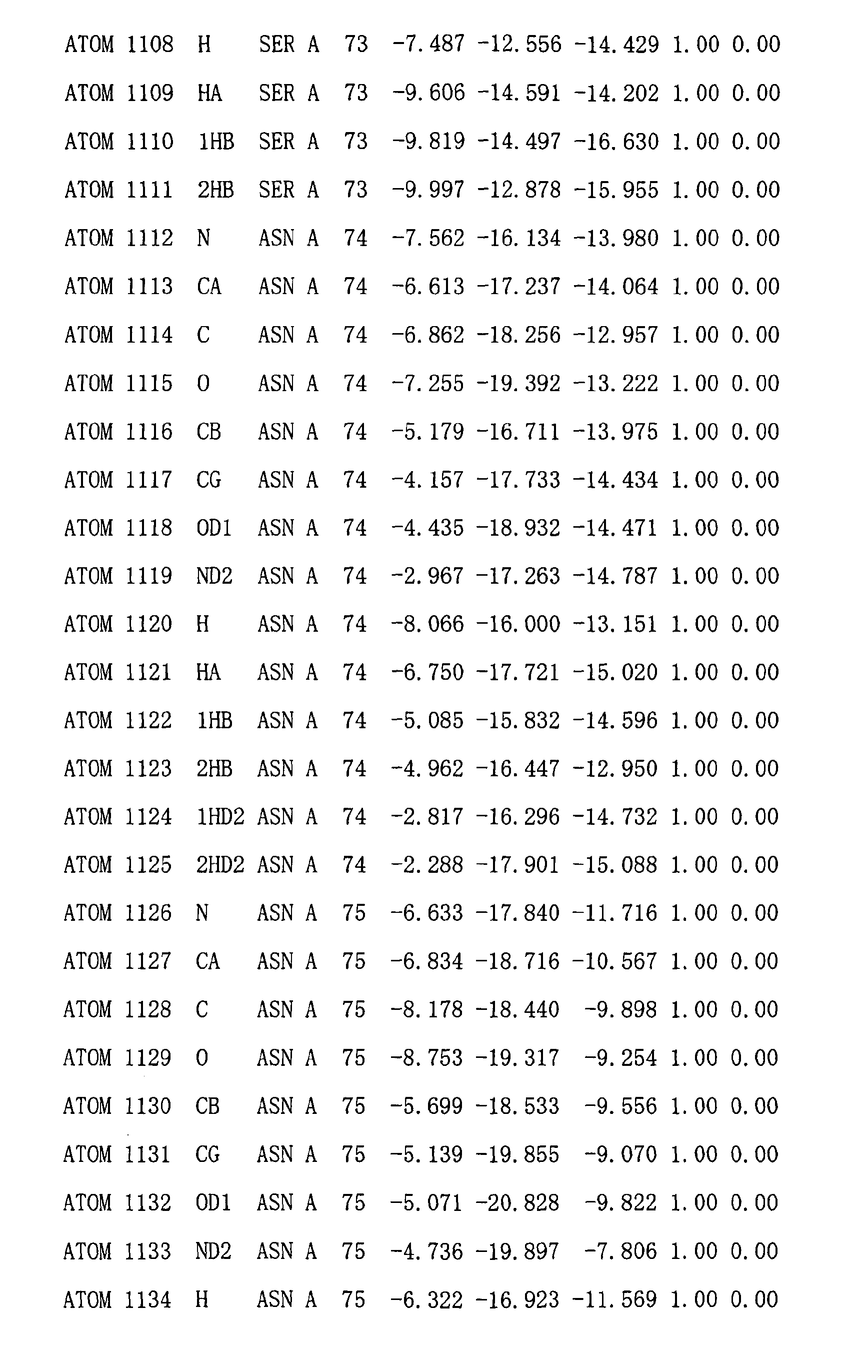

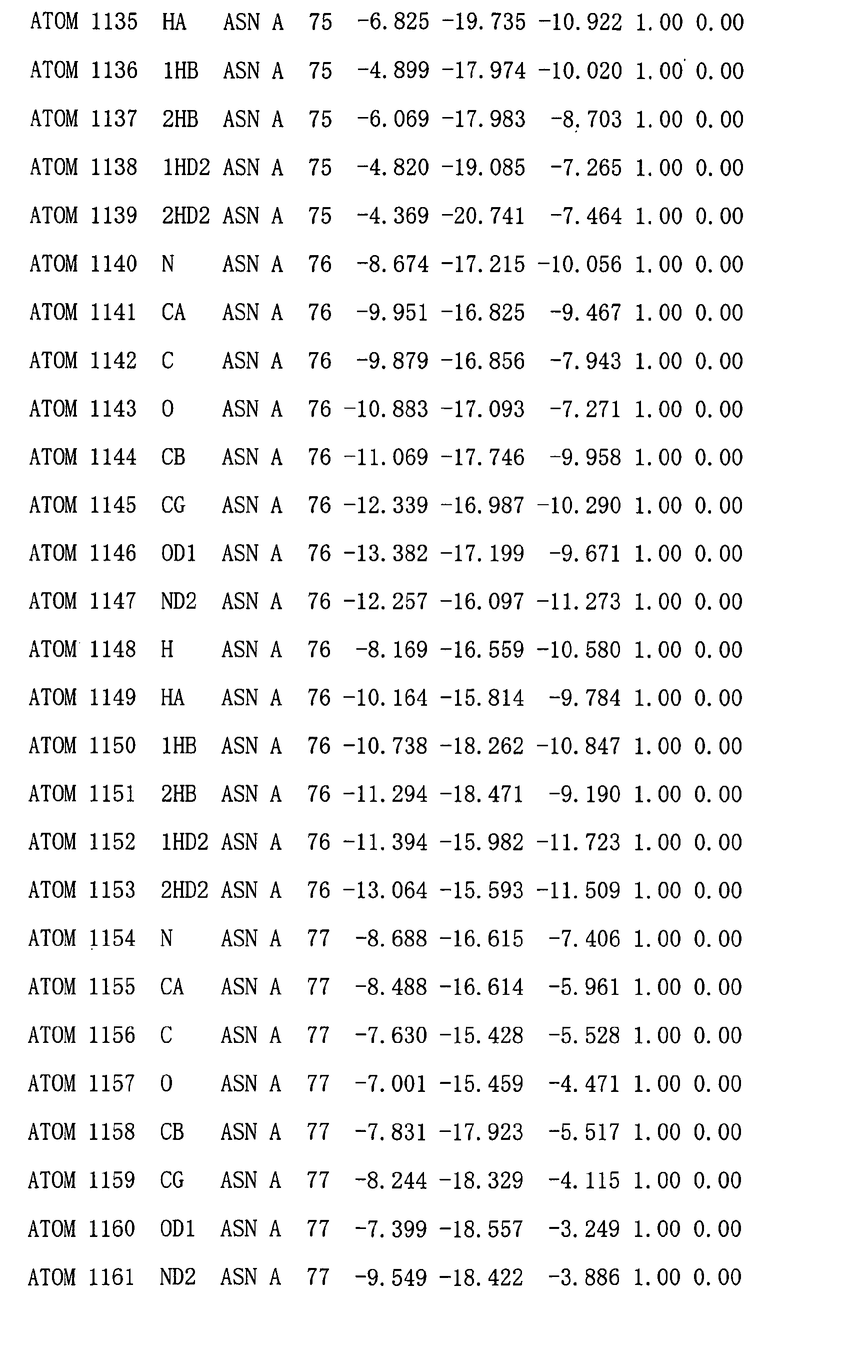

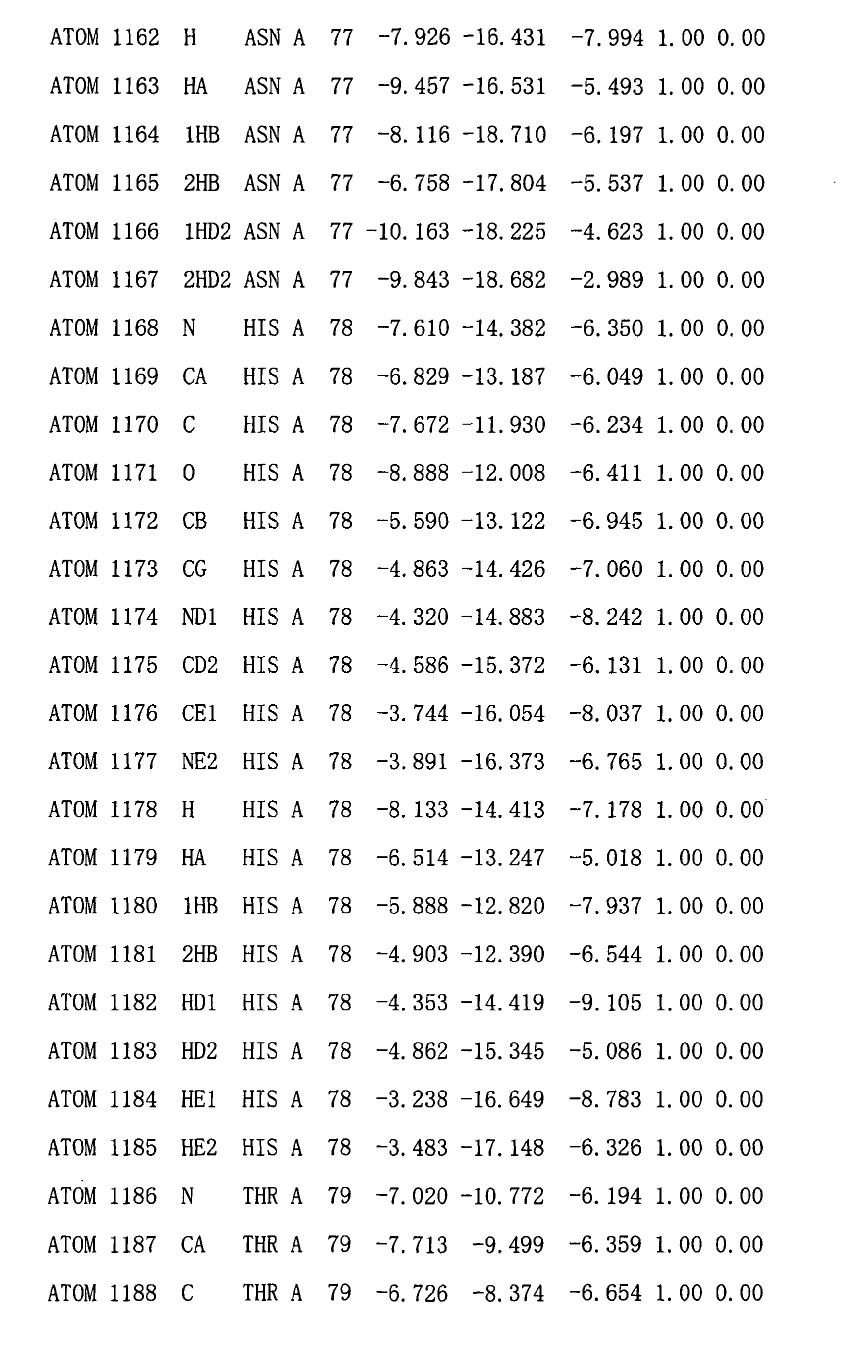

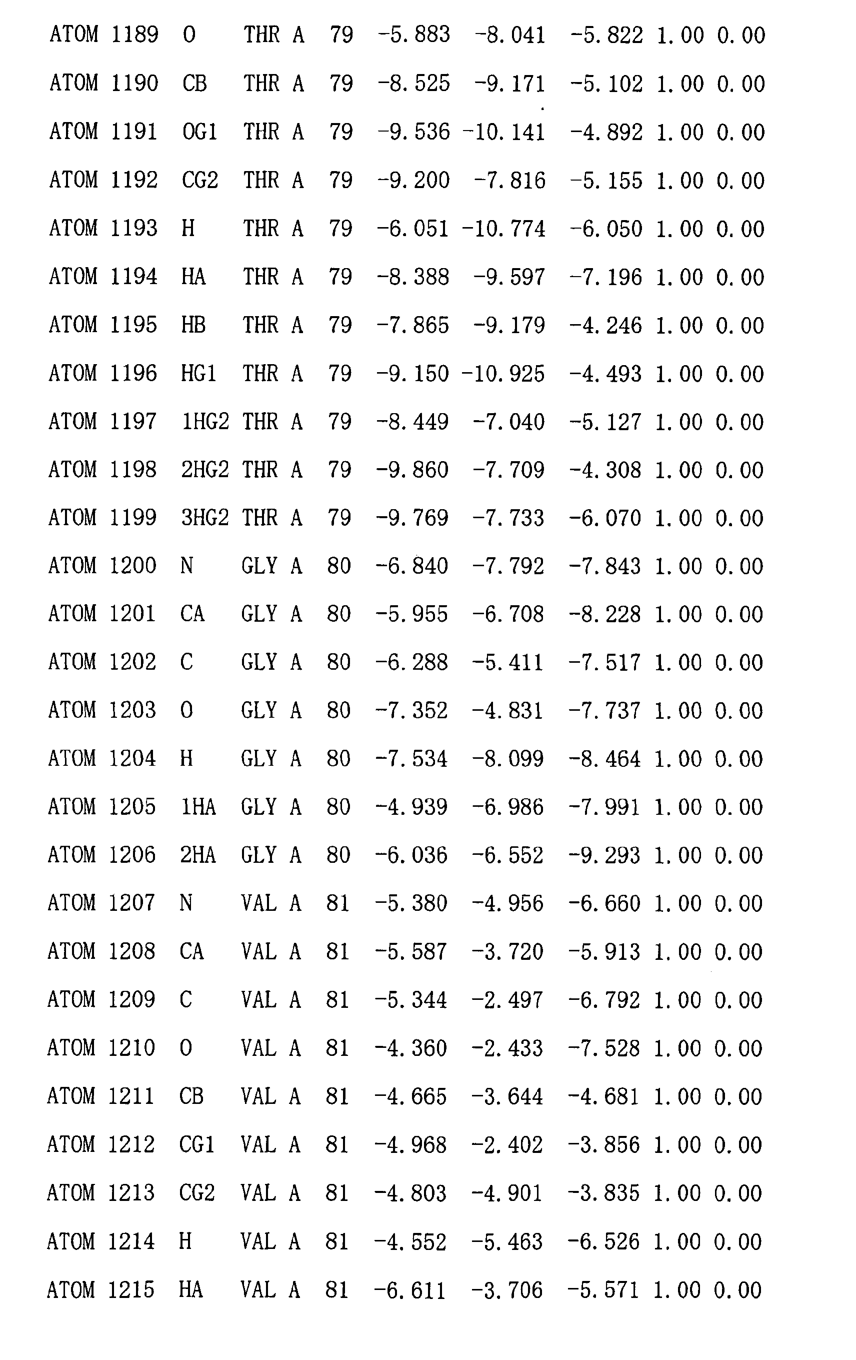

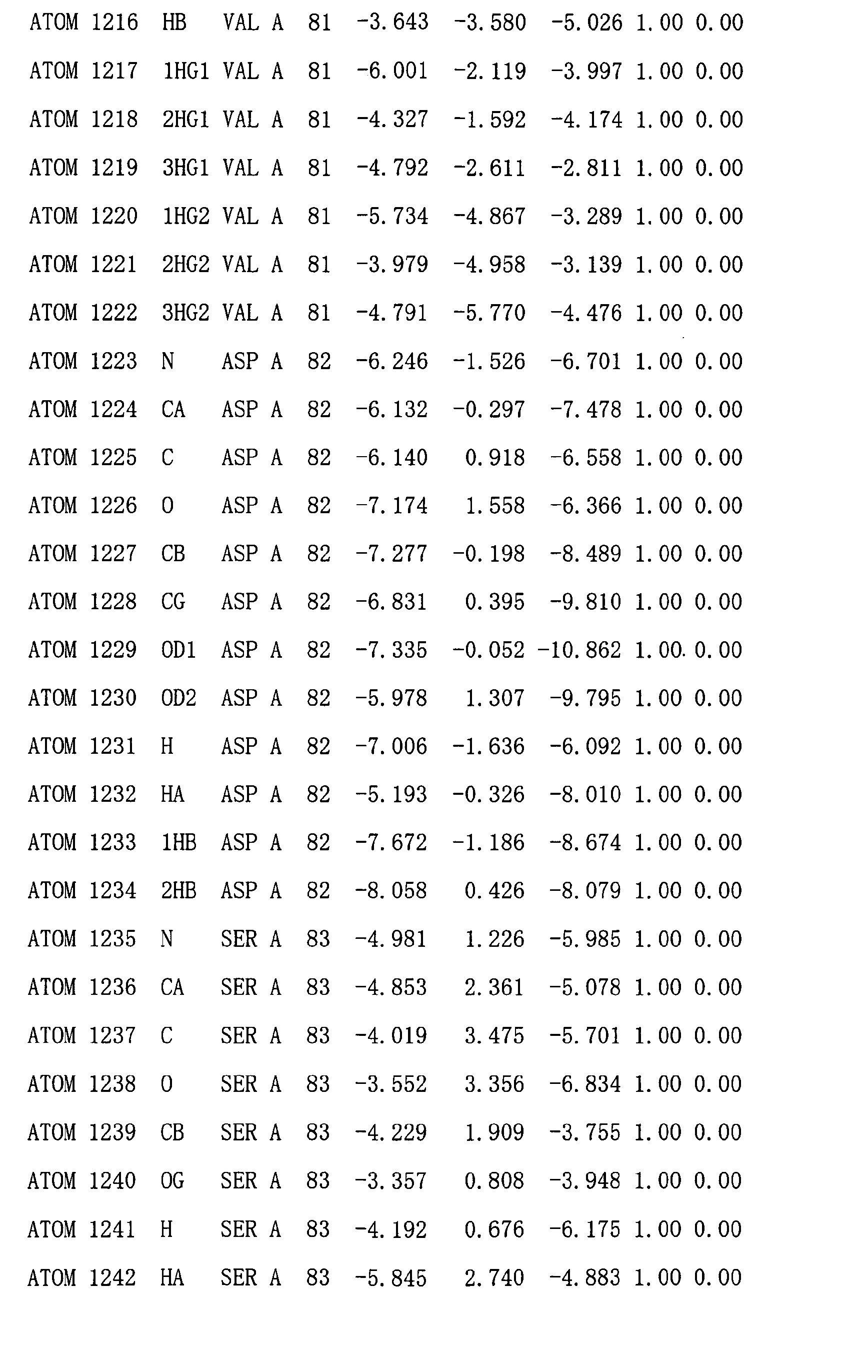

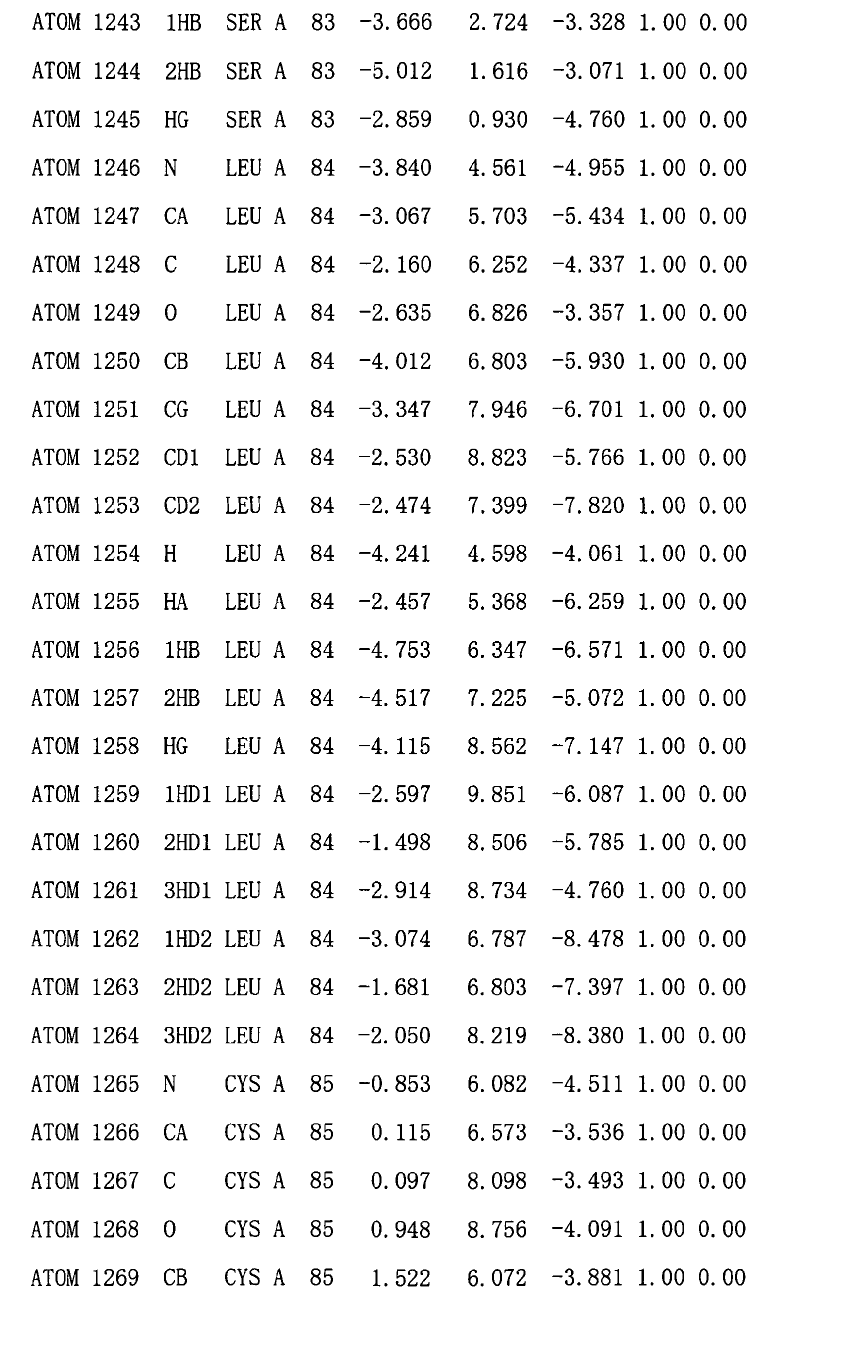

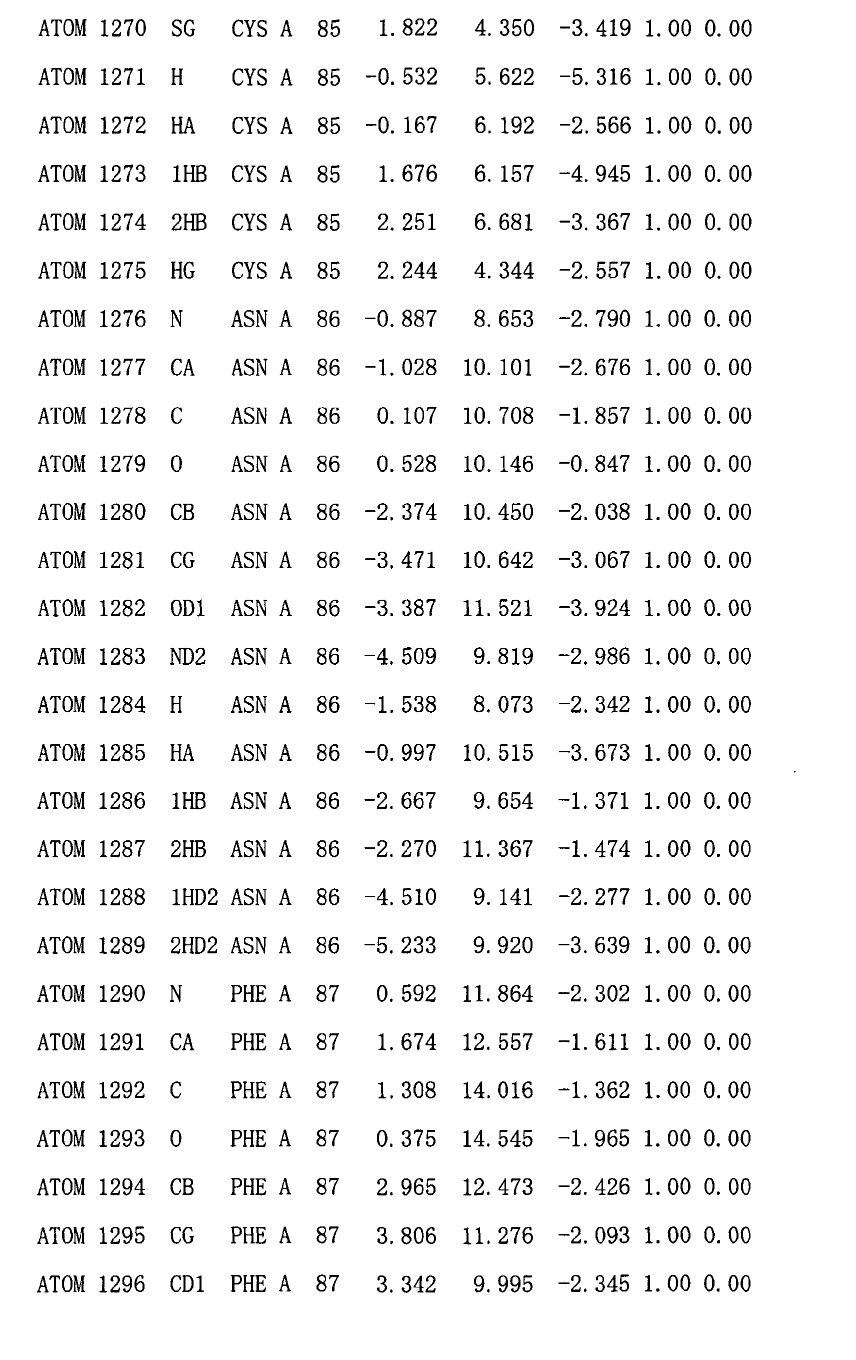

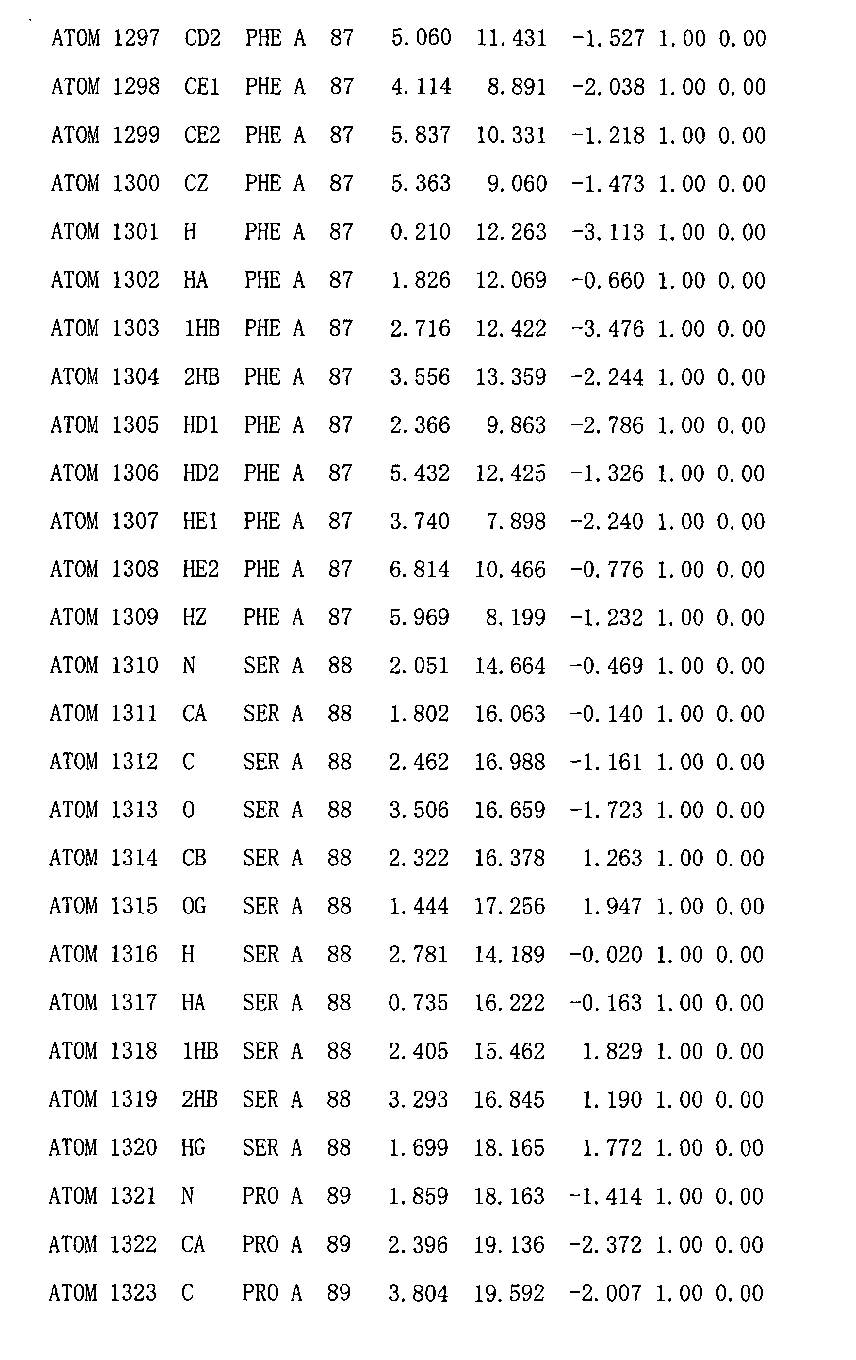

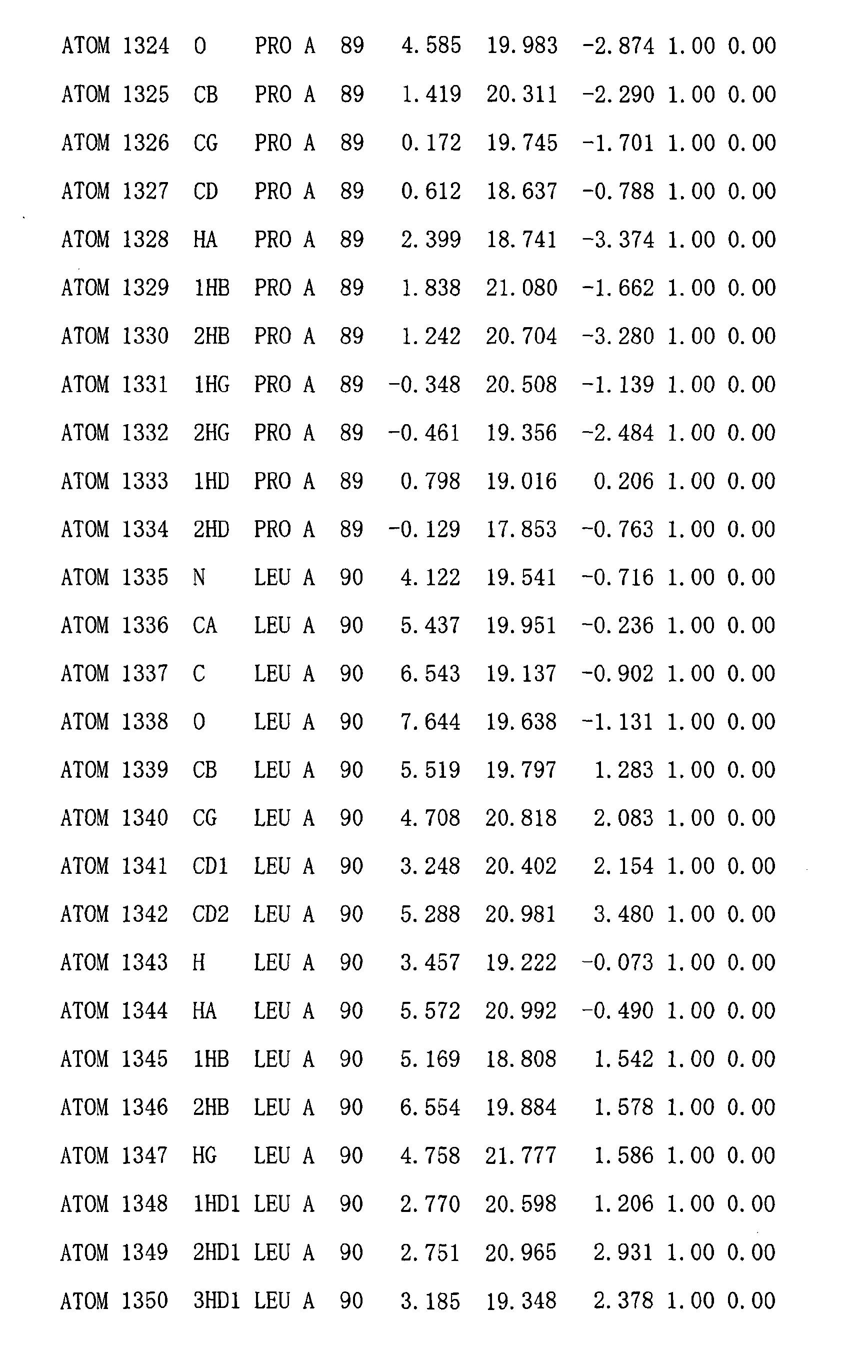

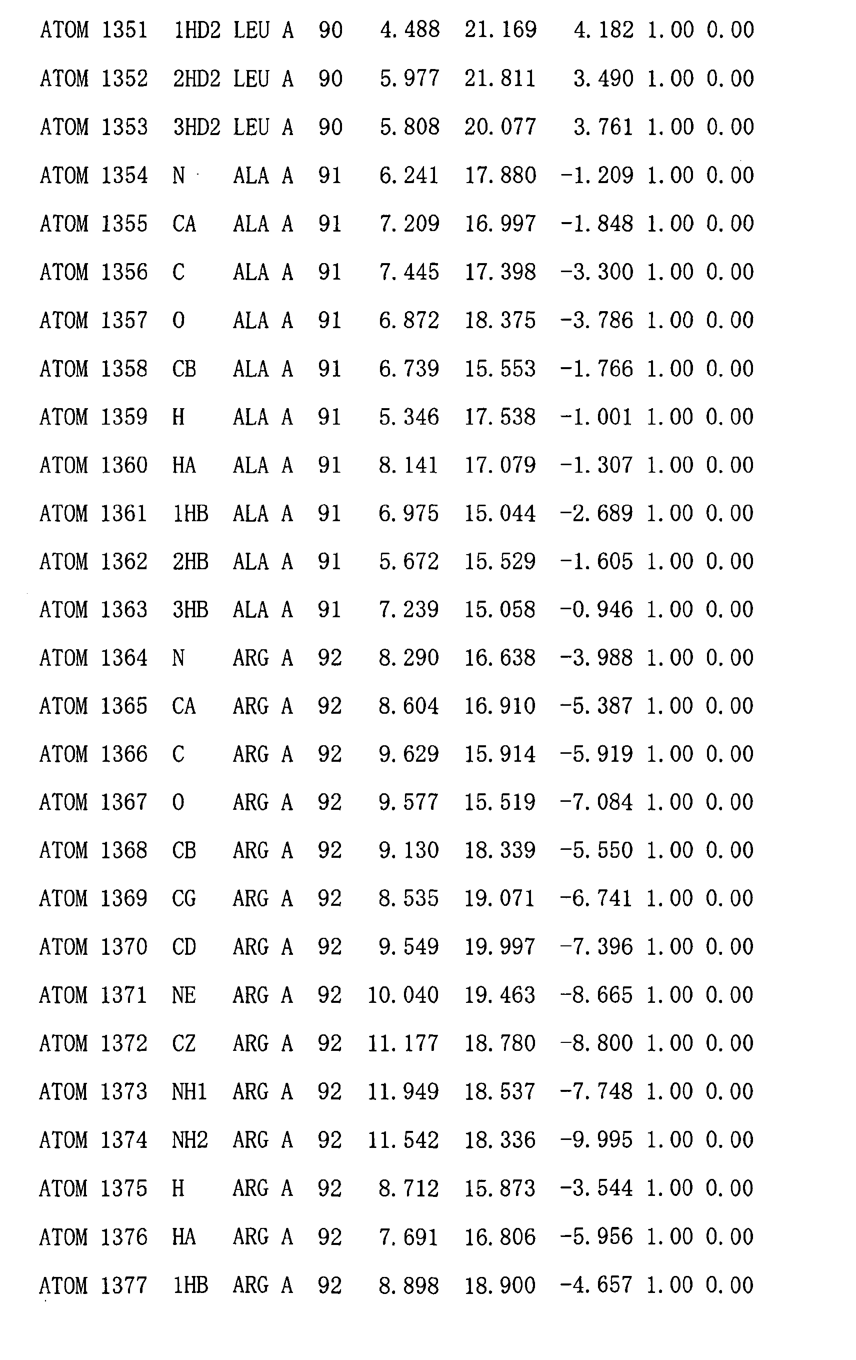

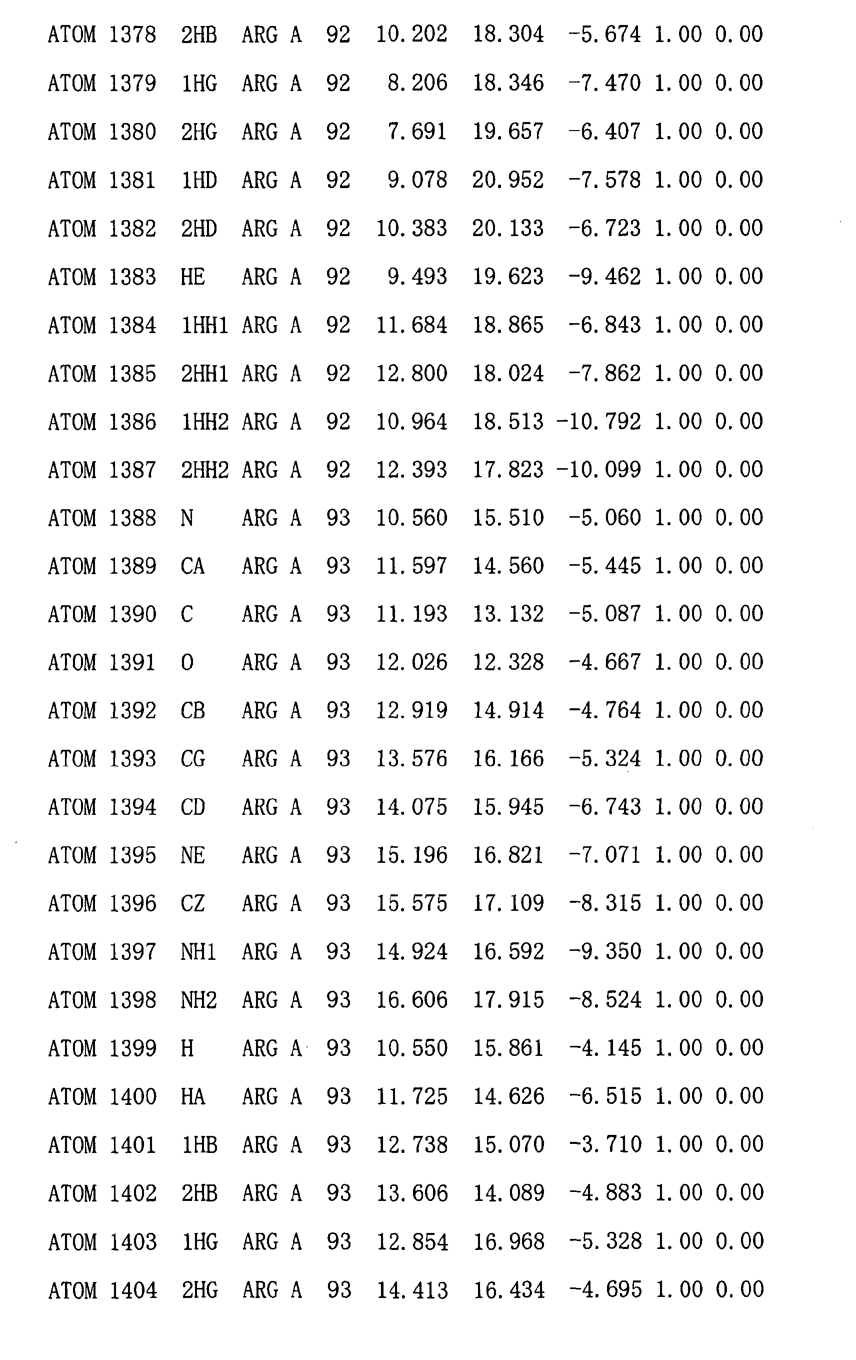

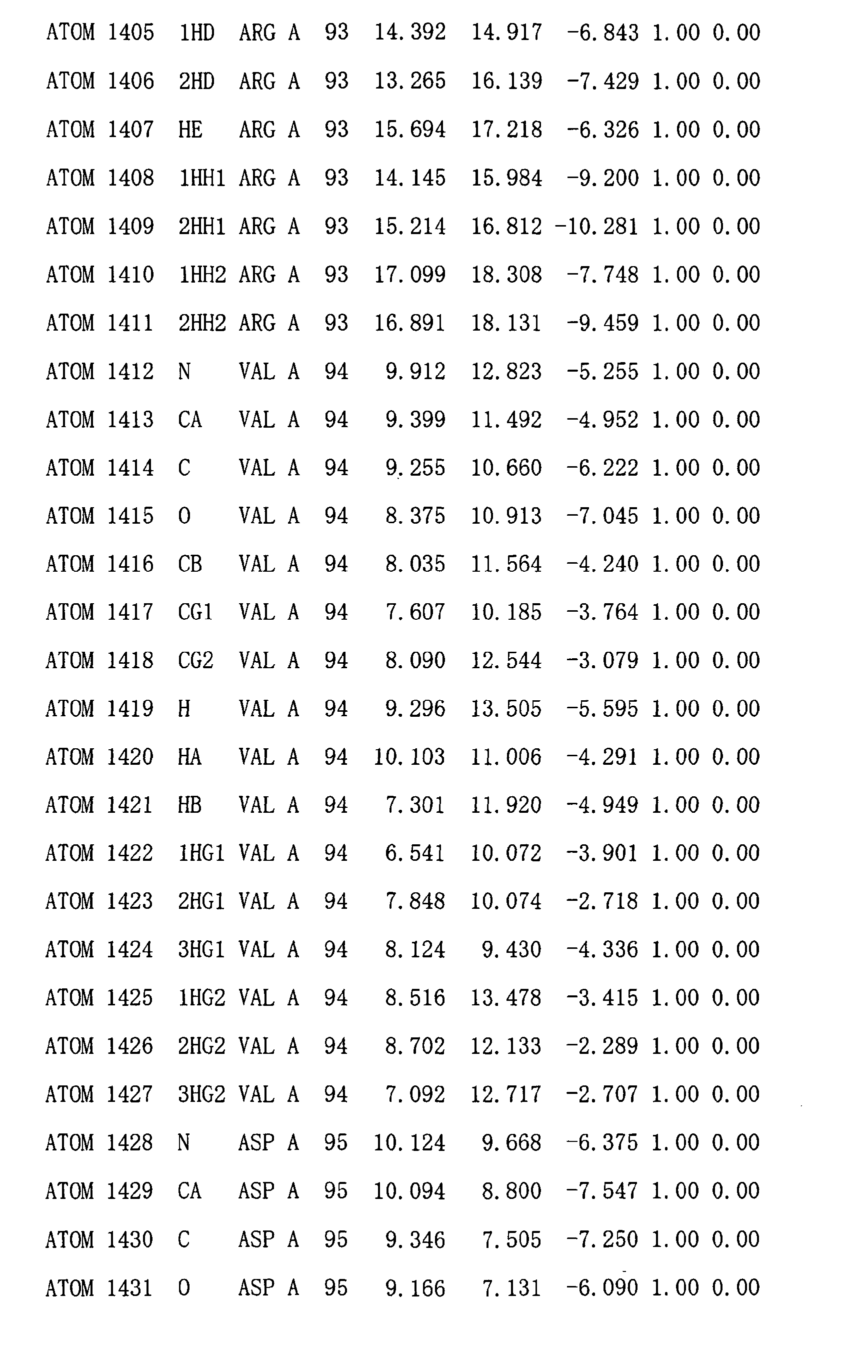

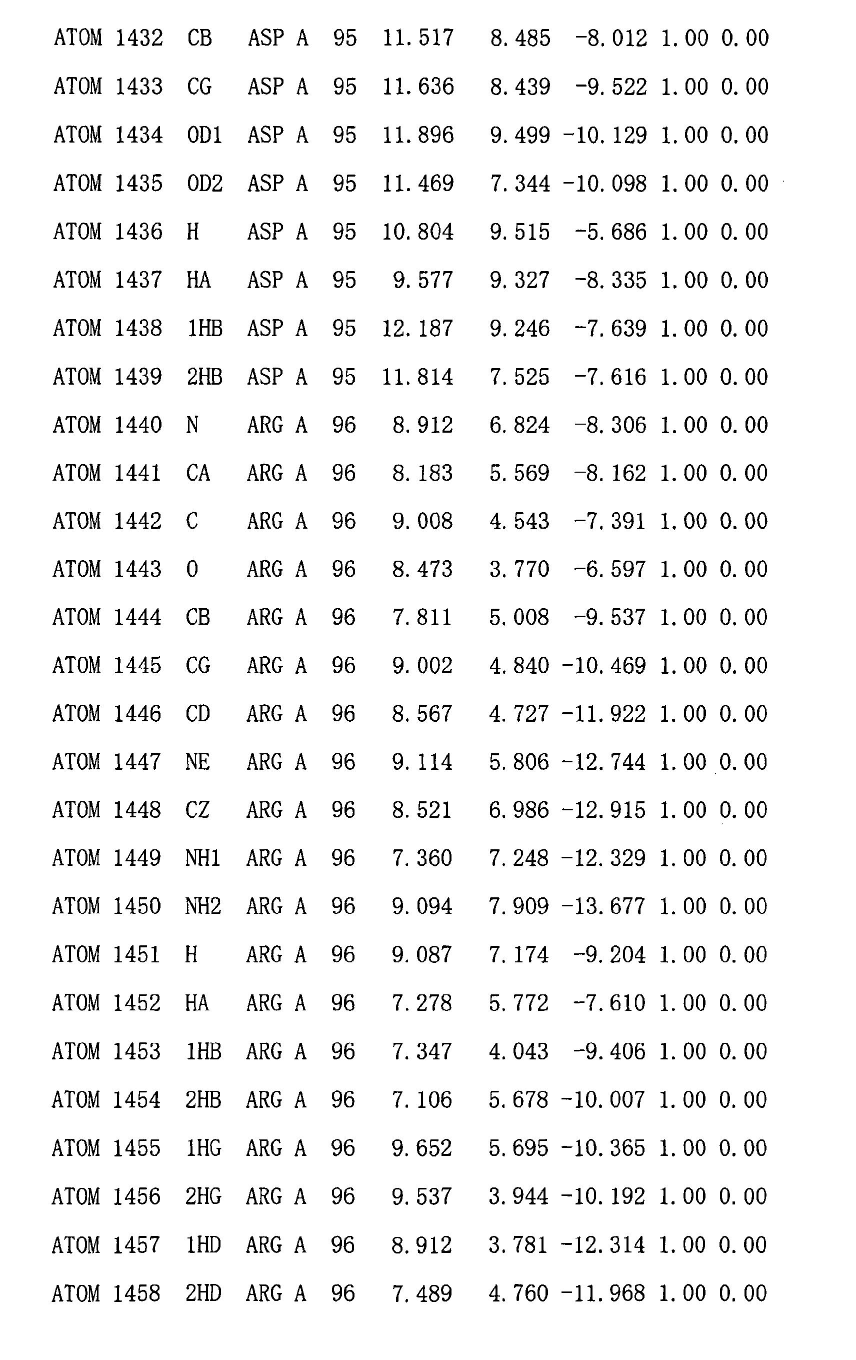

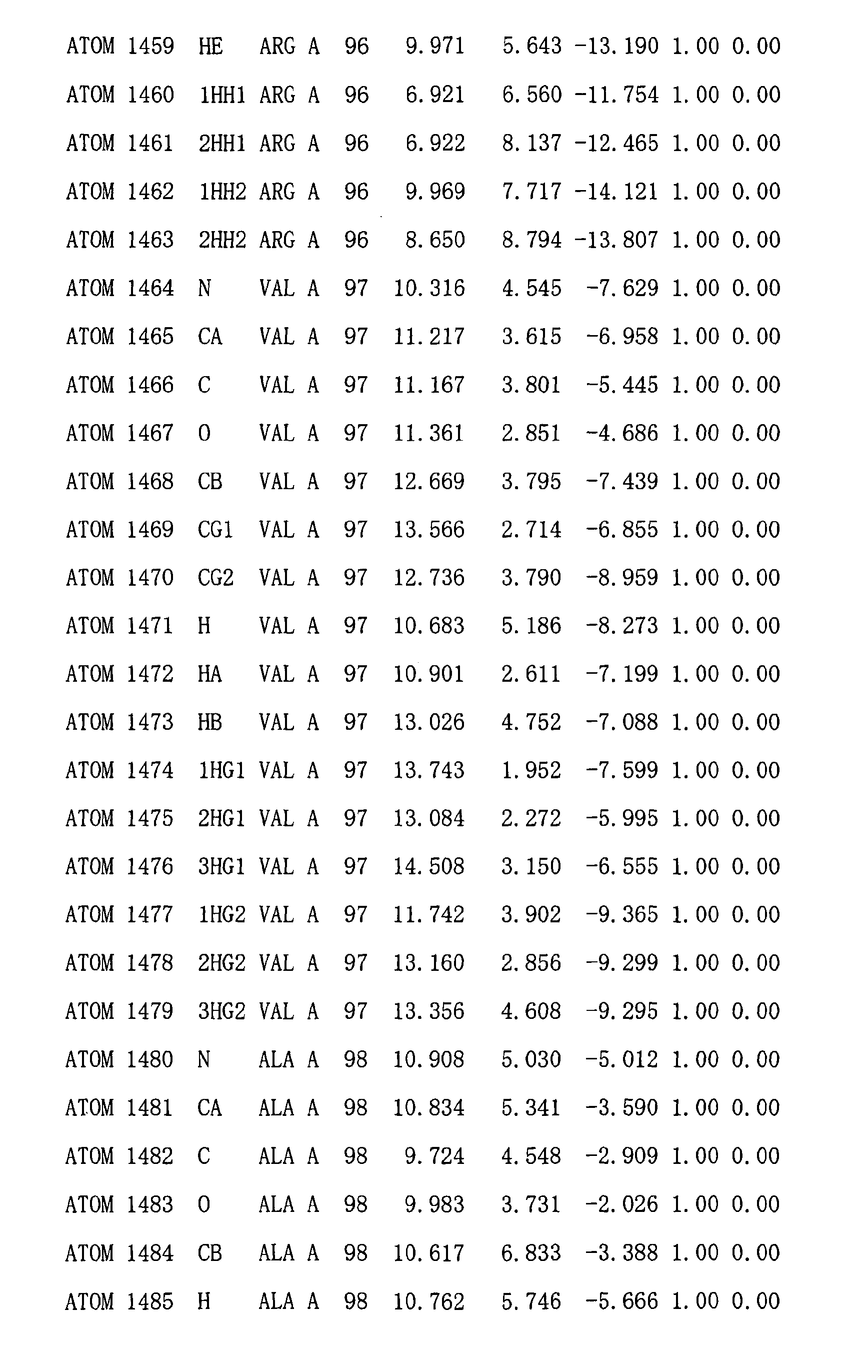

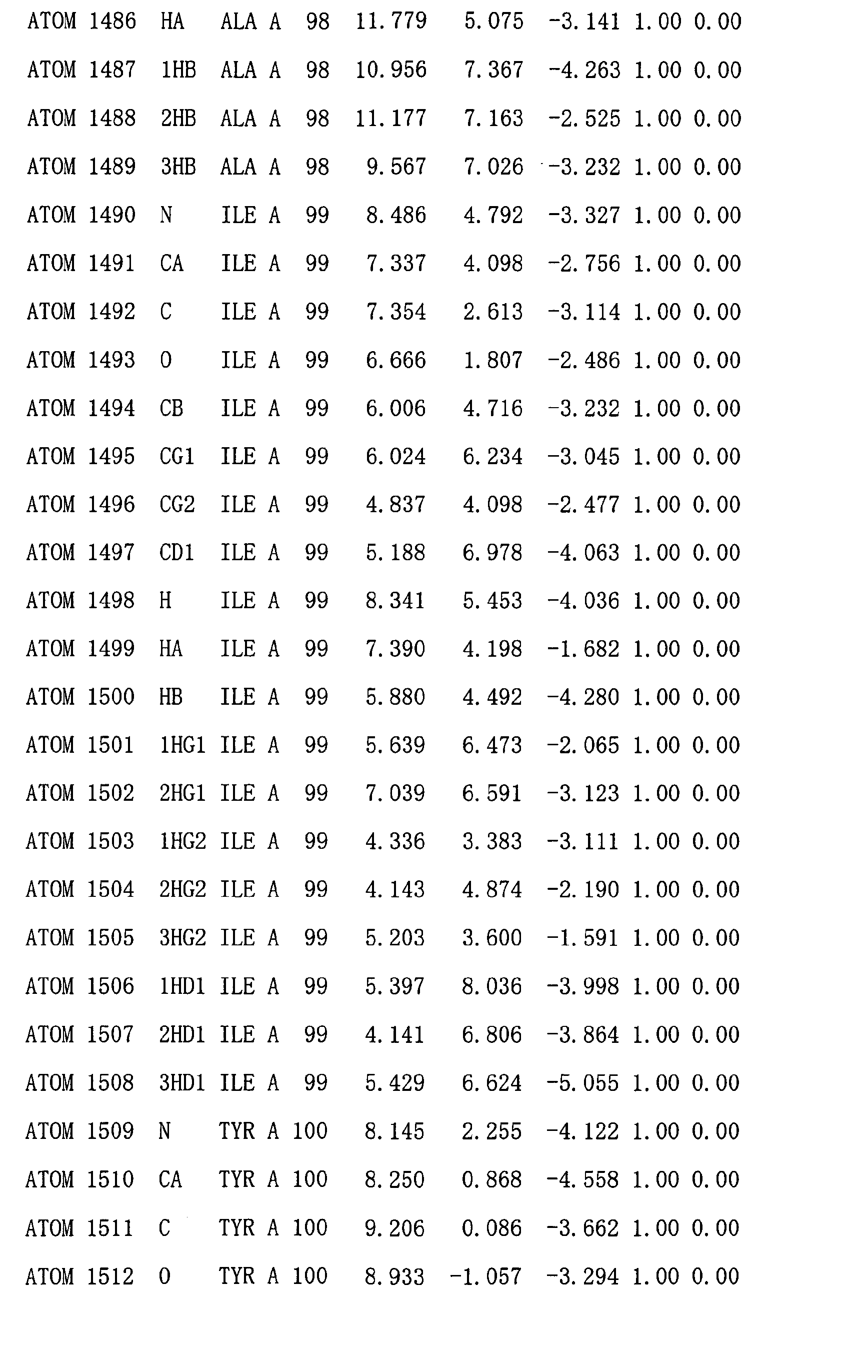

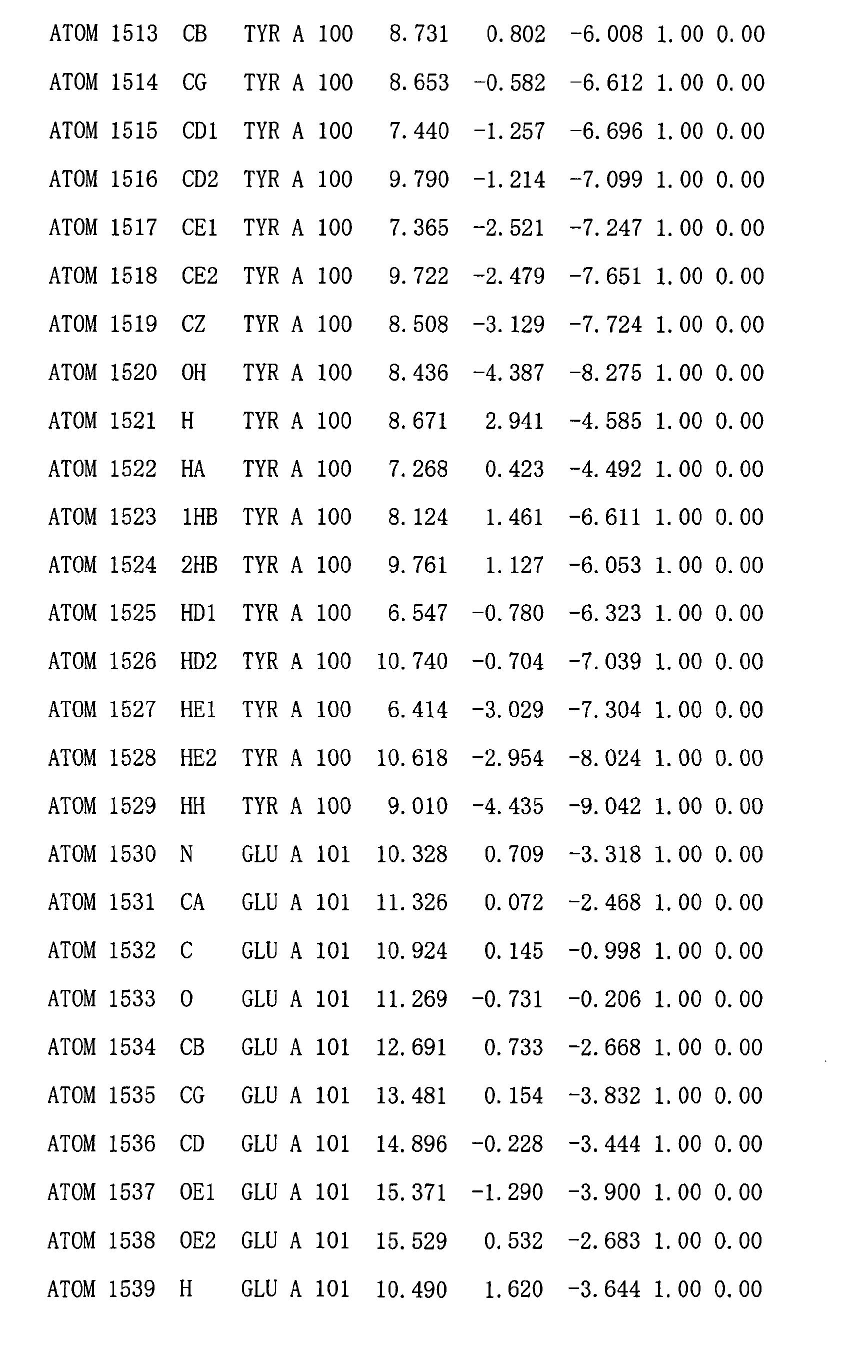

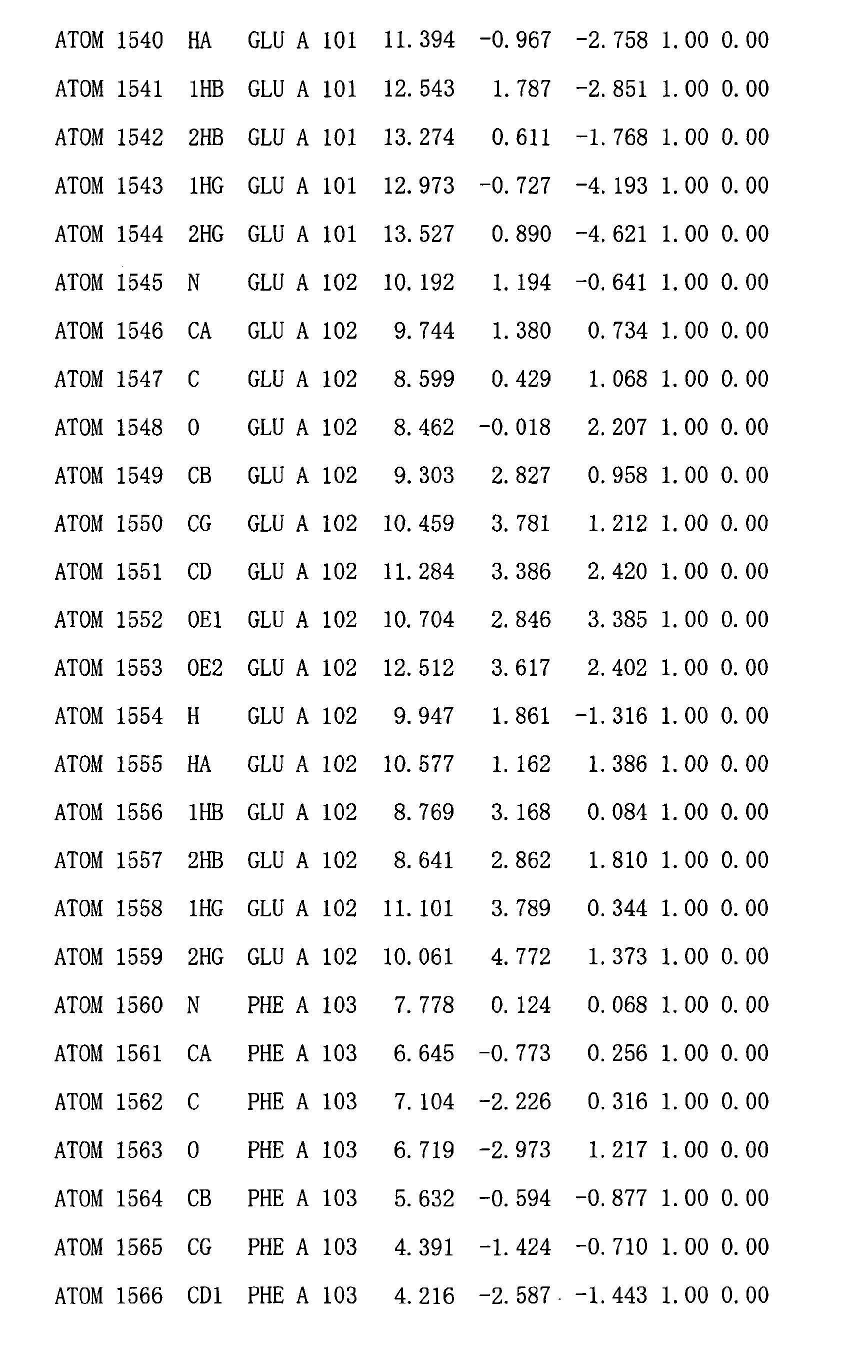

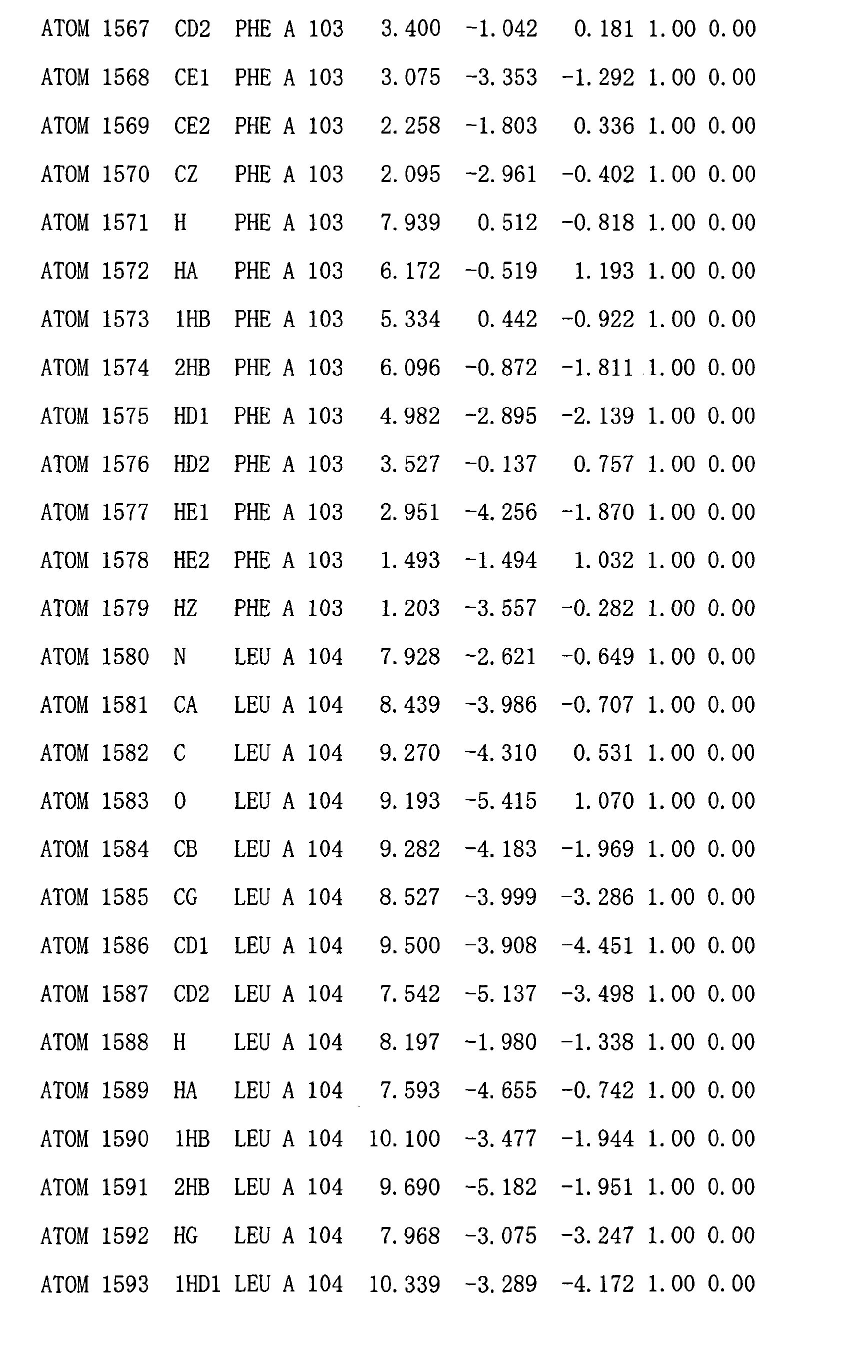

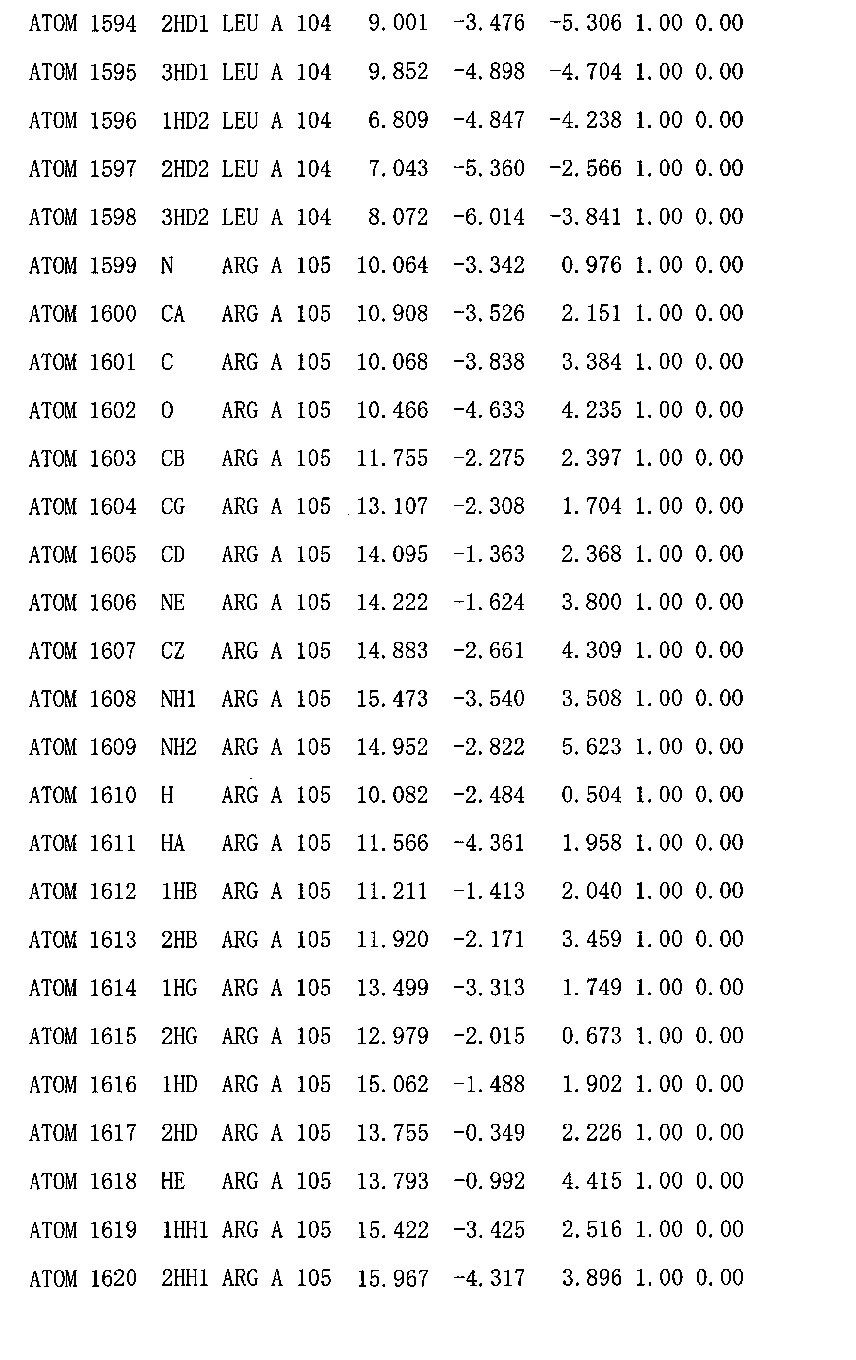

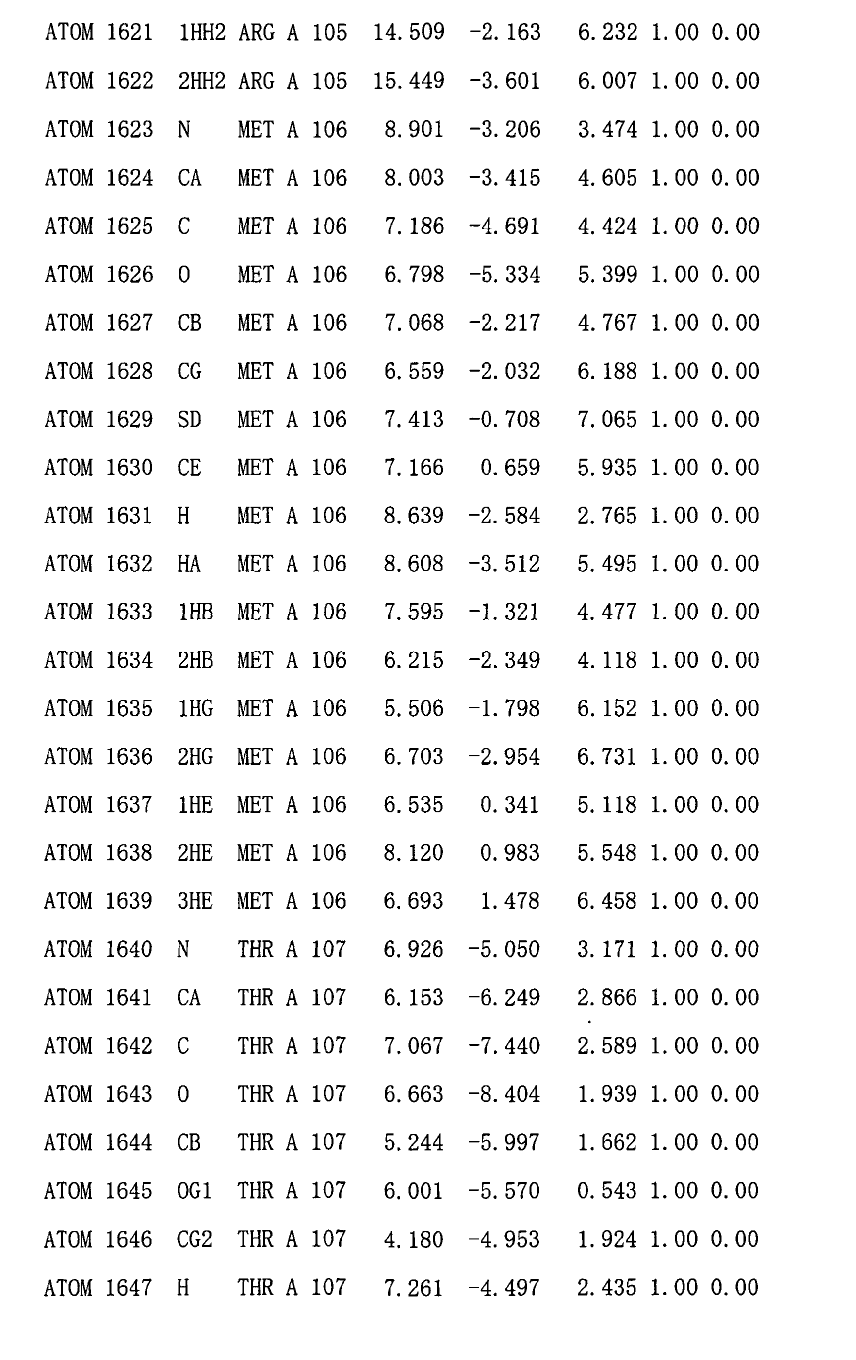

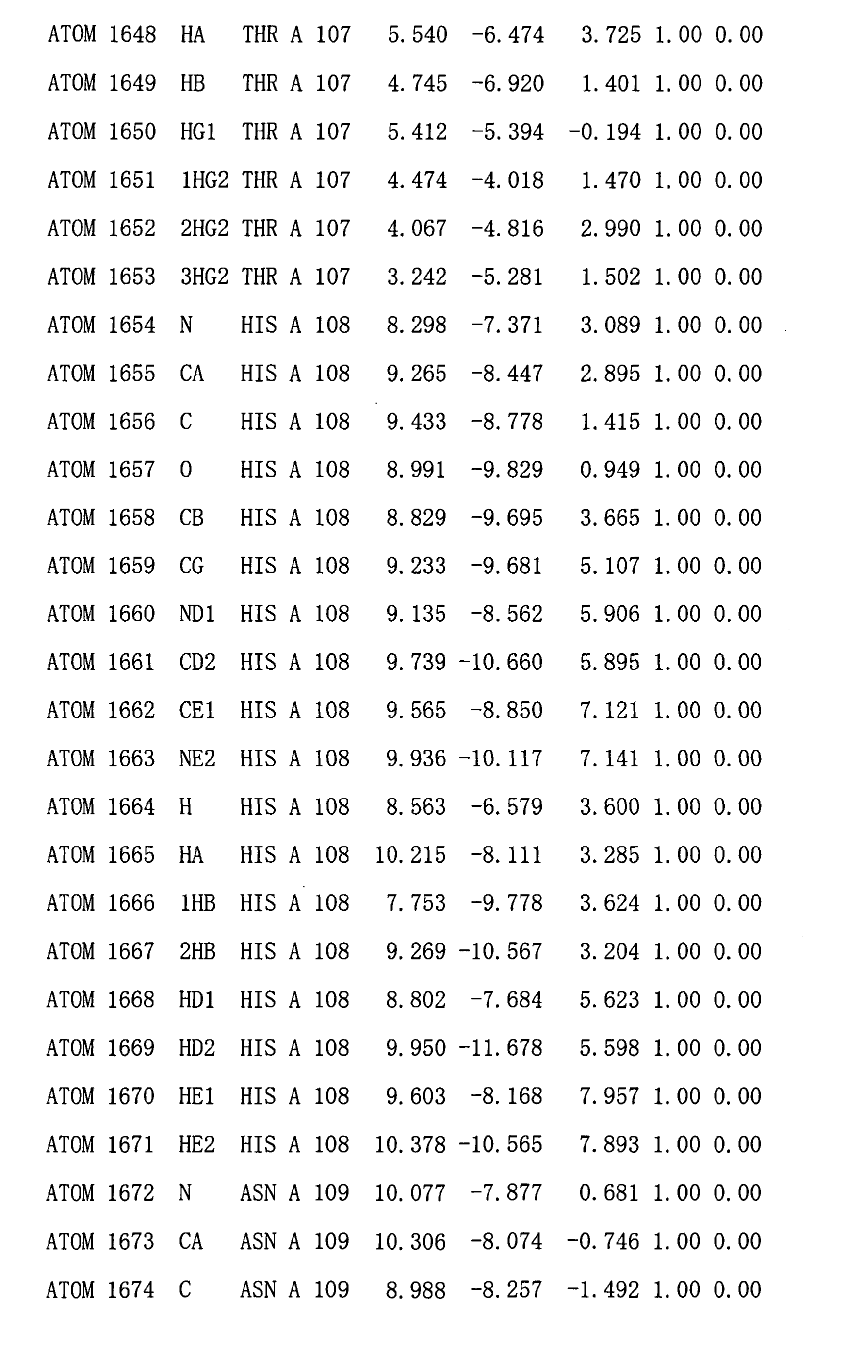

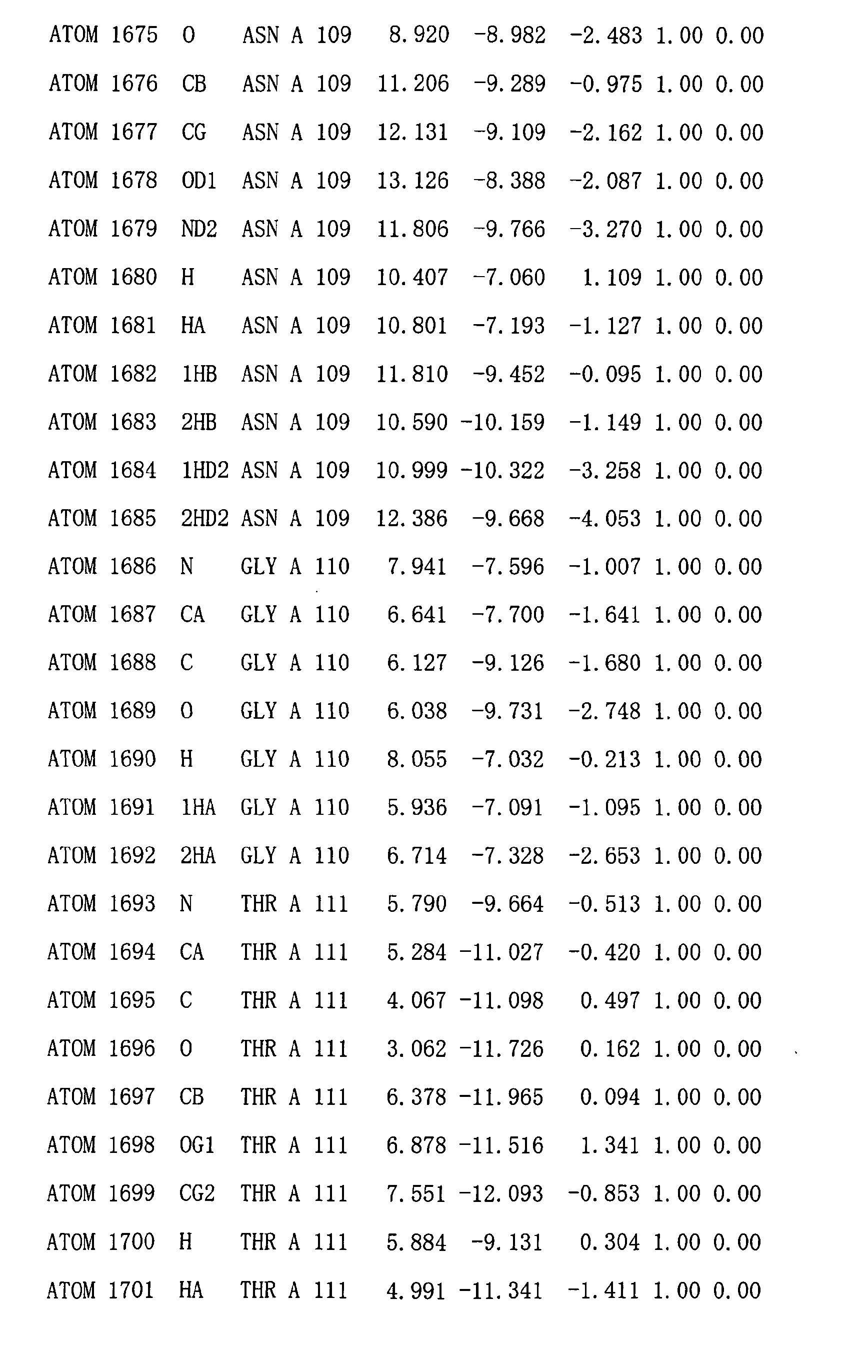

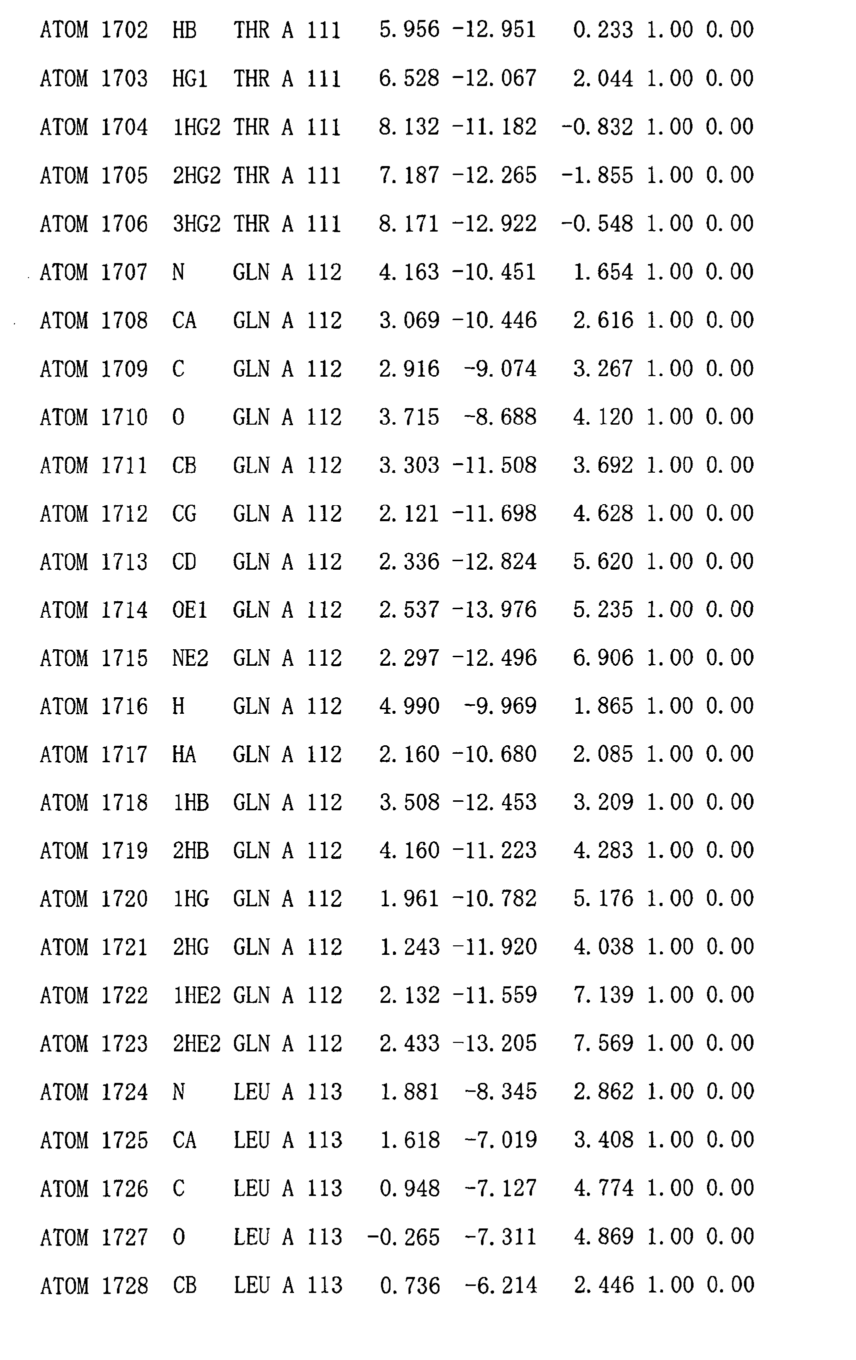

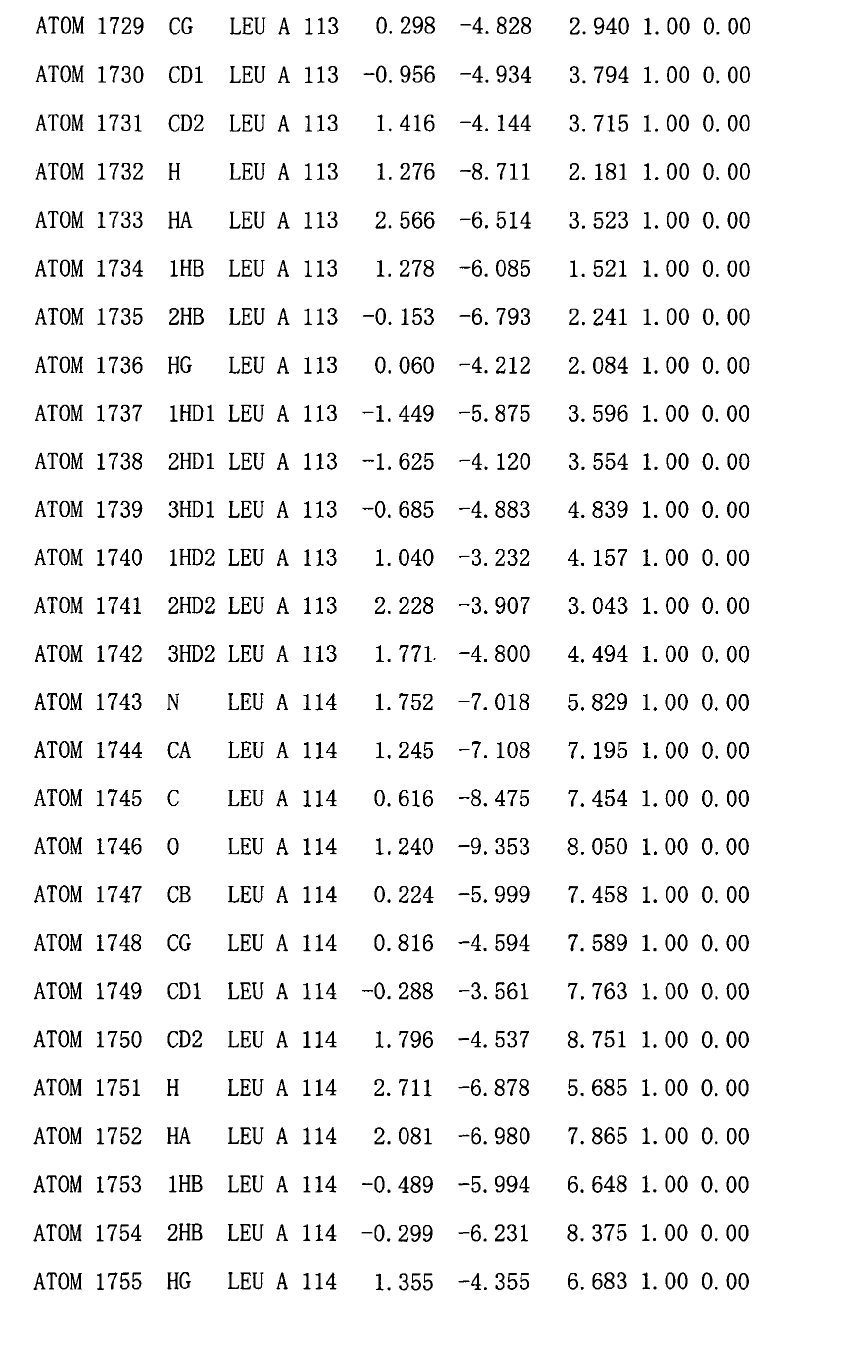

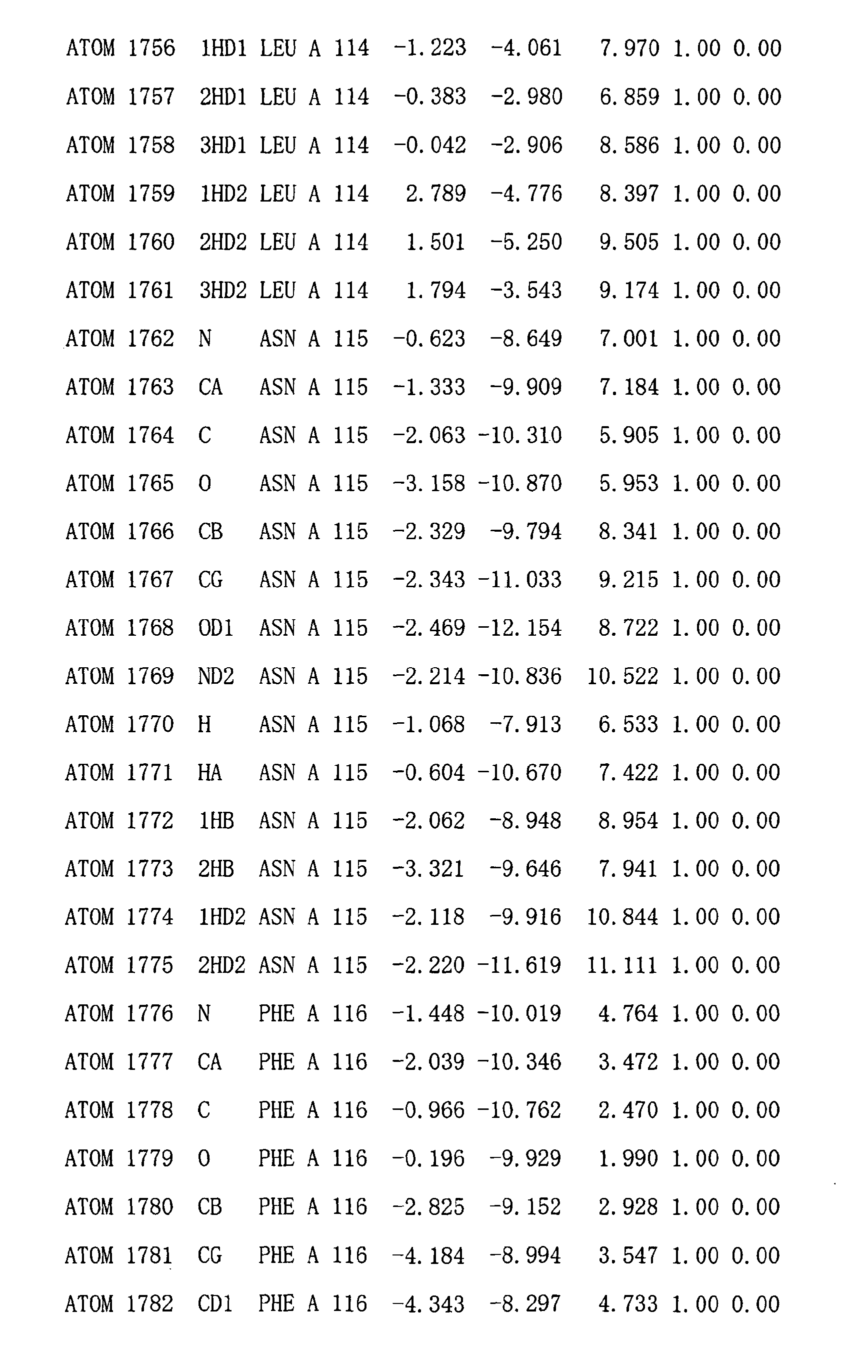

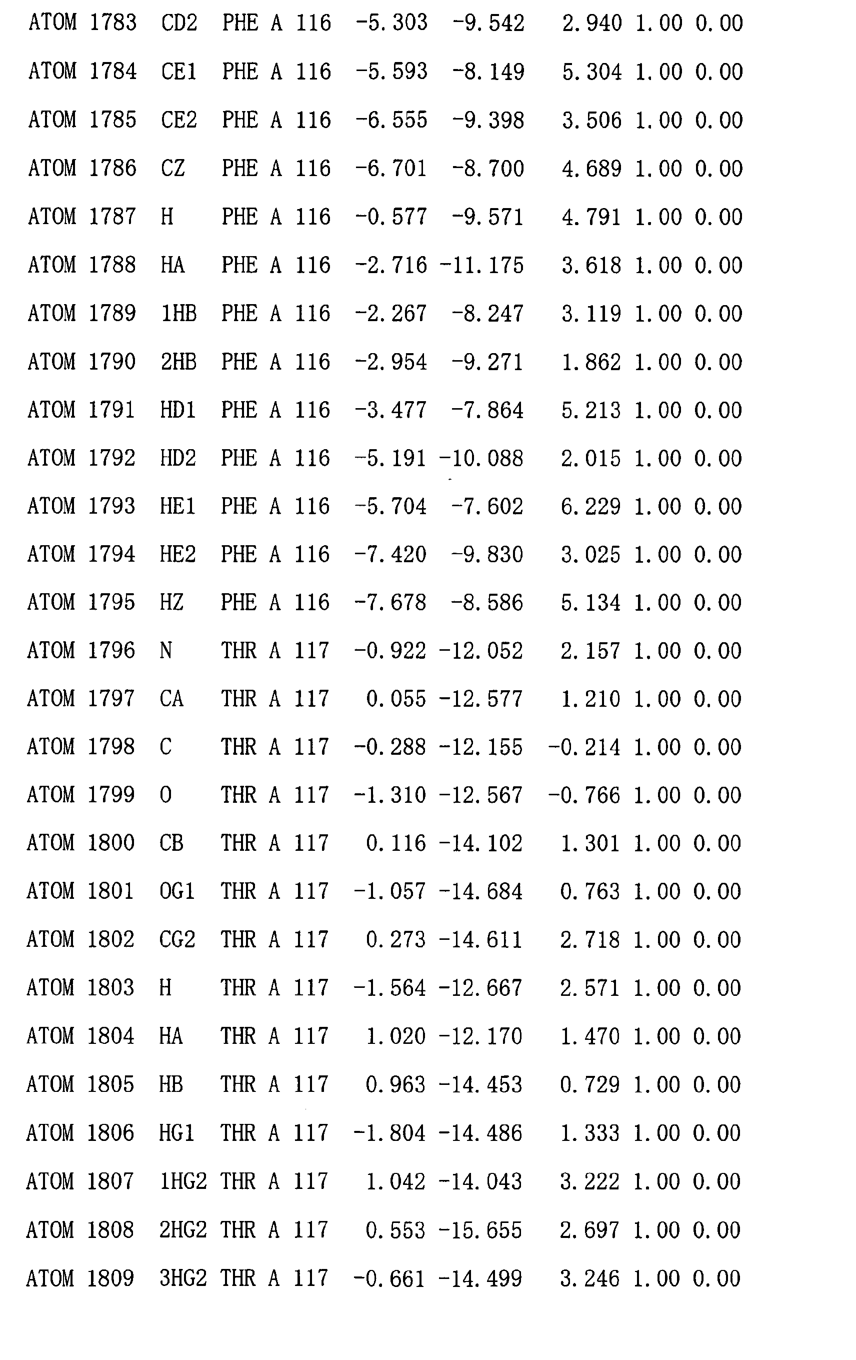

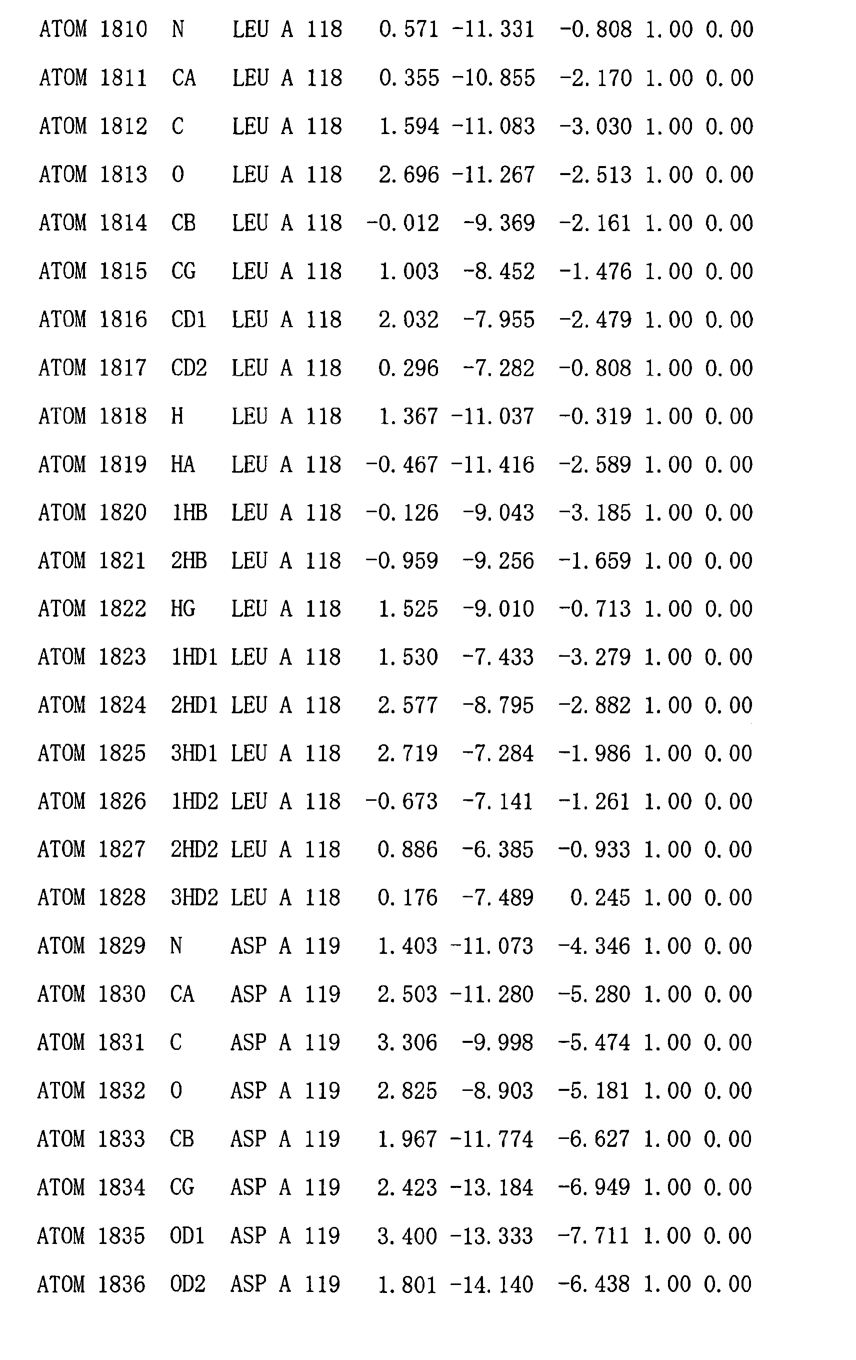

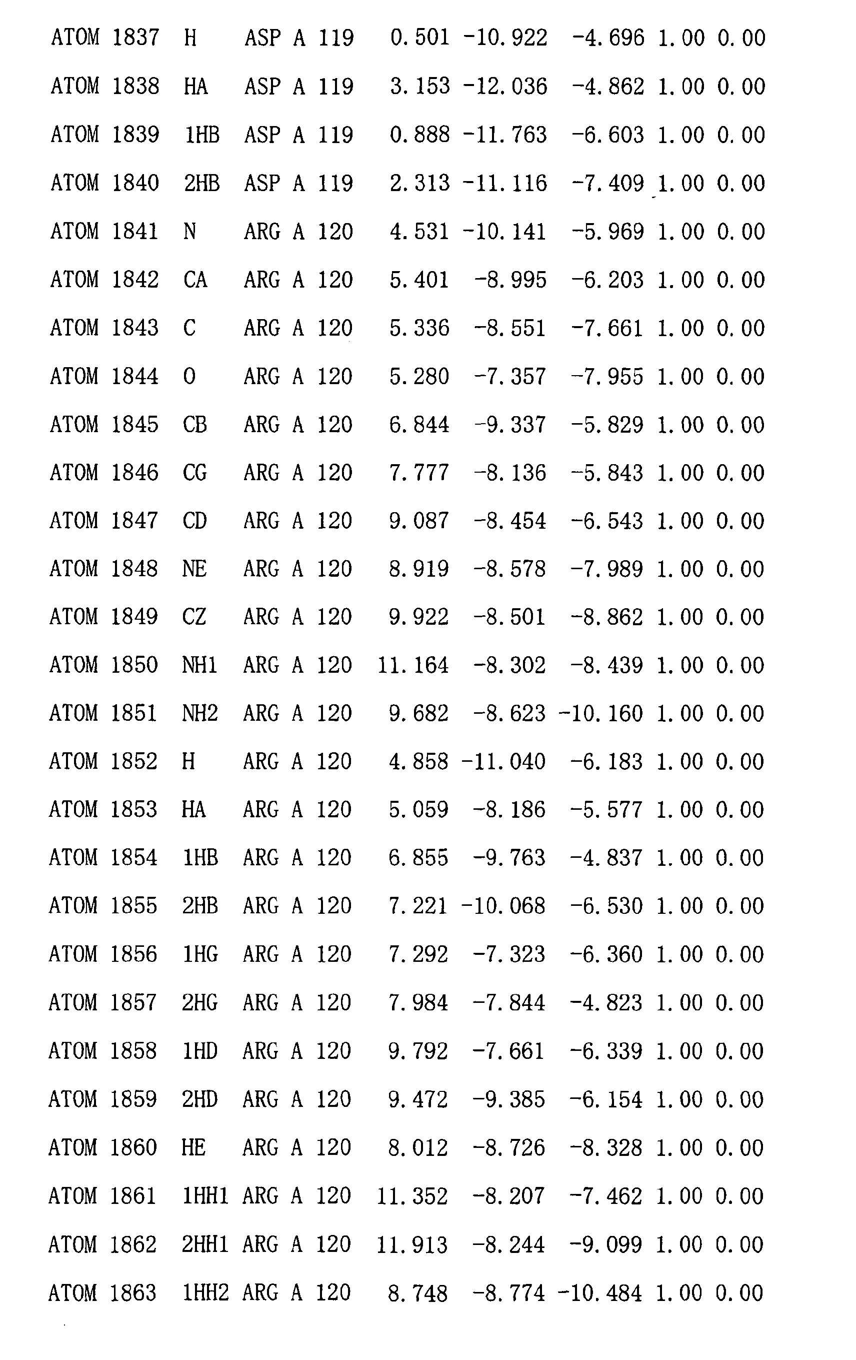

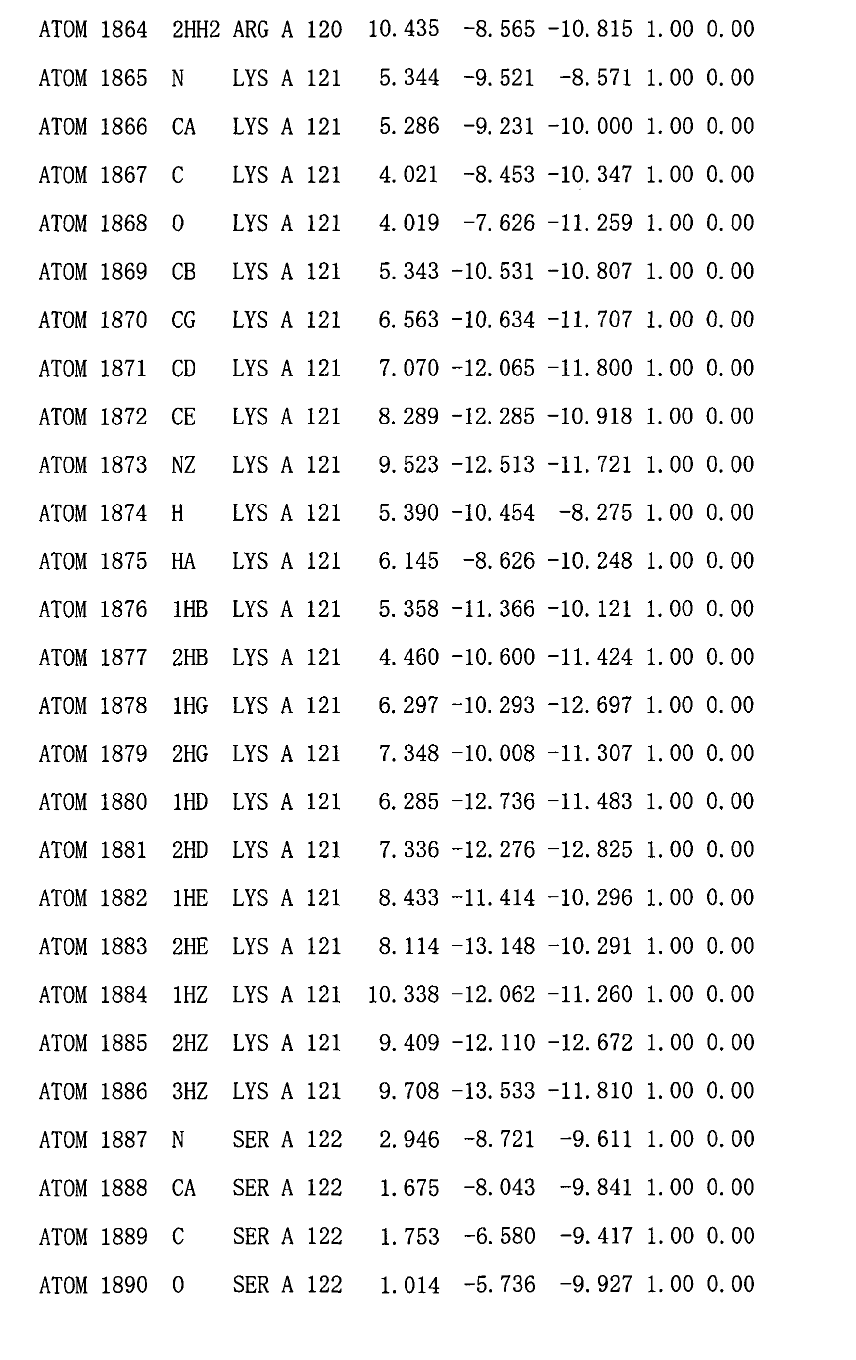

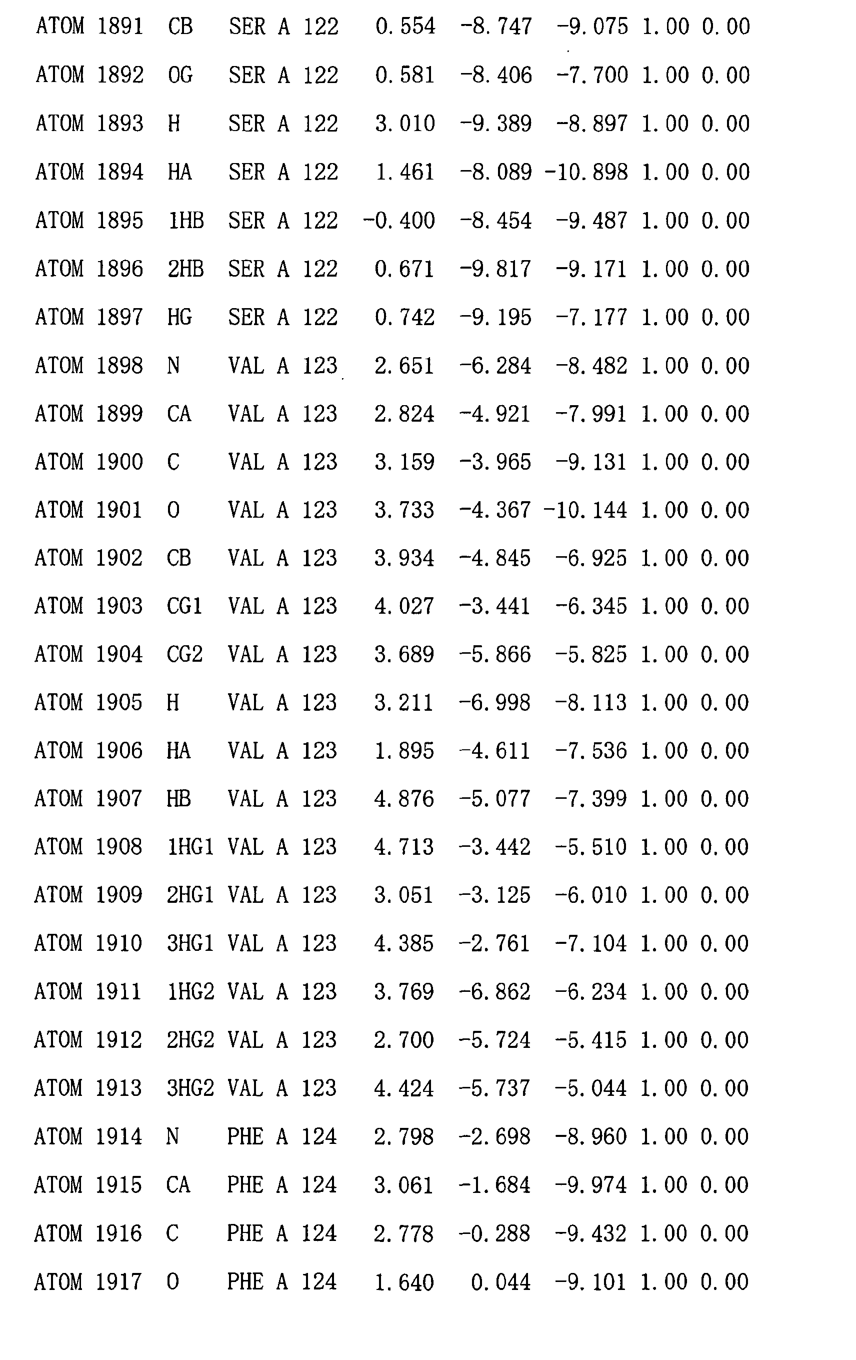

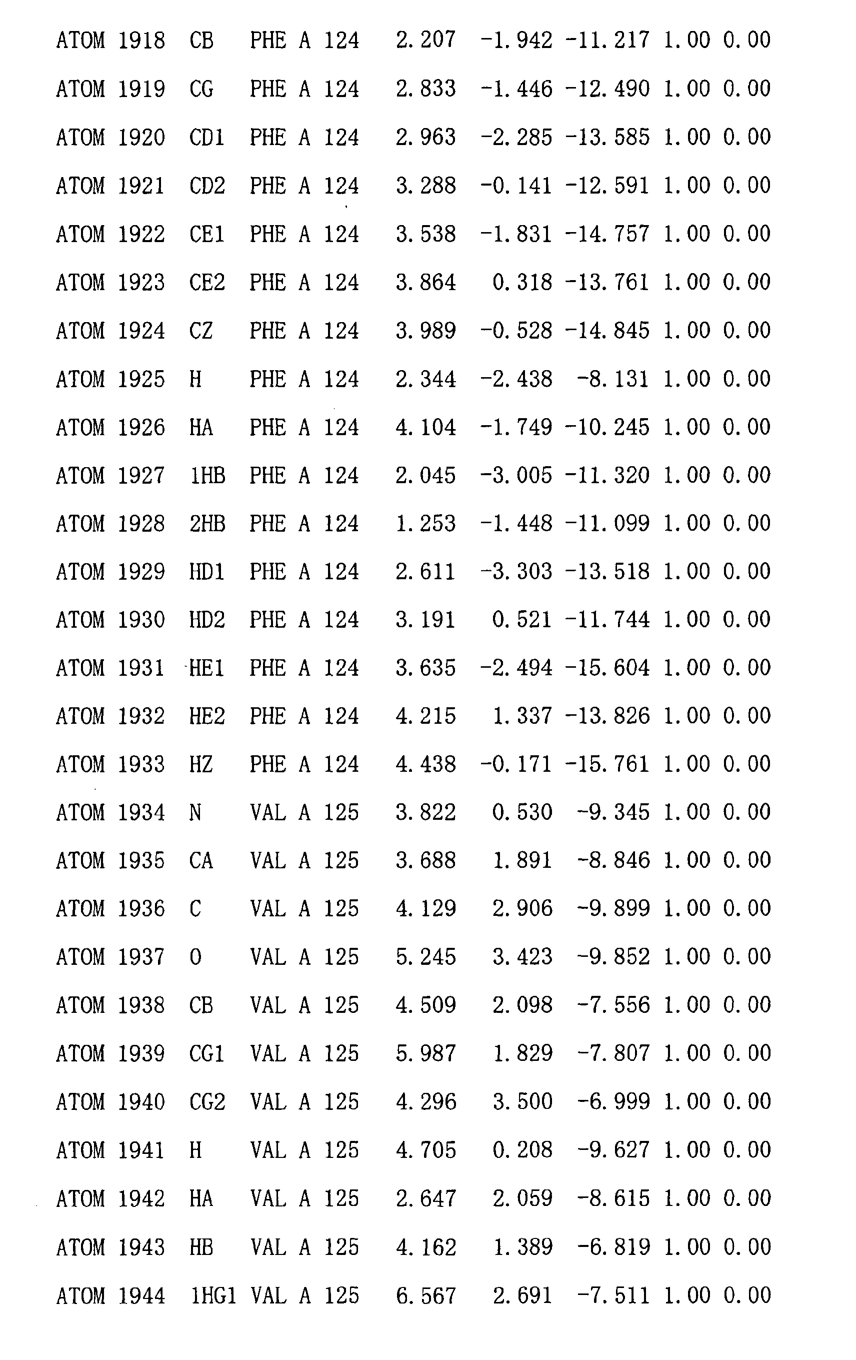

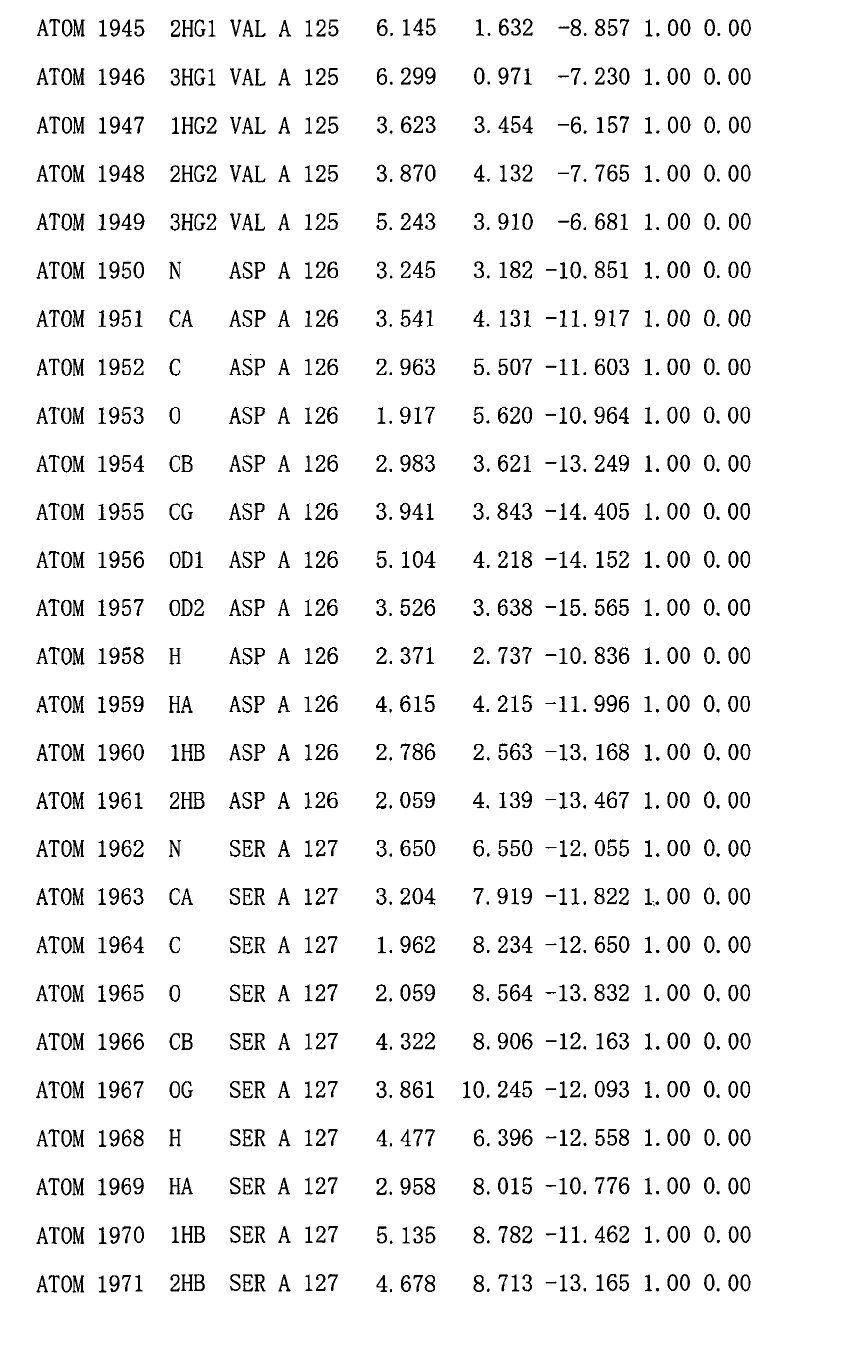

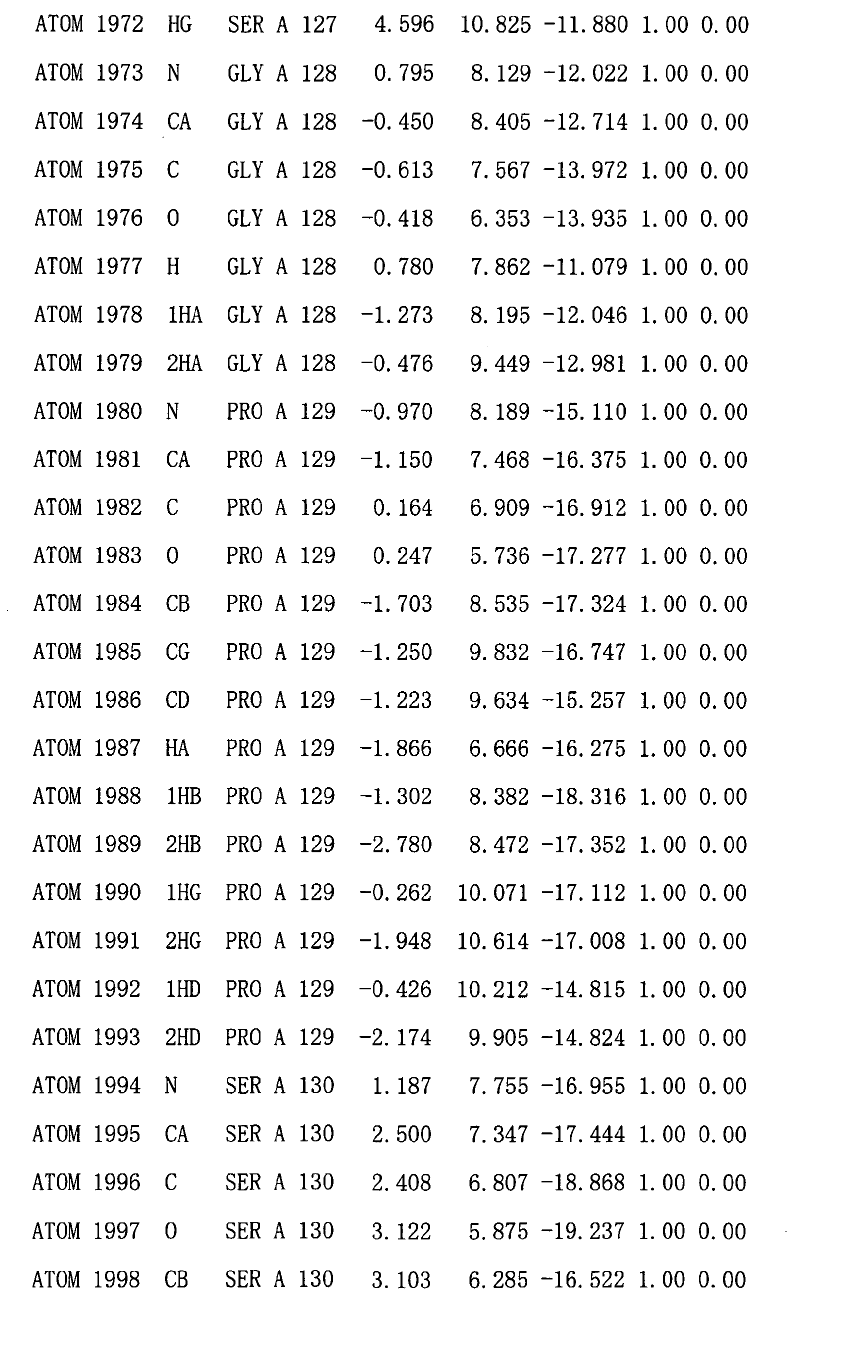

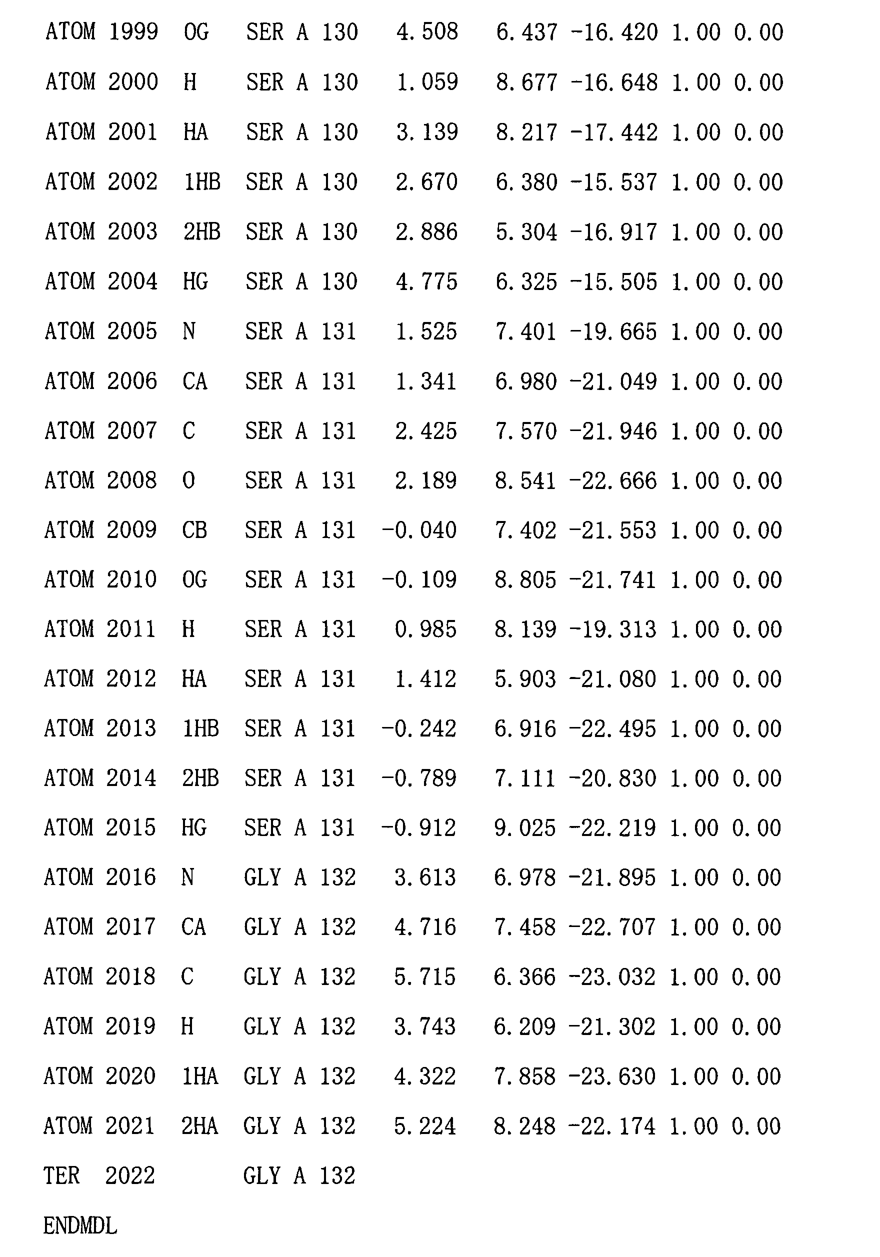

### Three-Dimensional Structure Coordinate 19

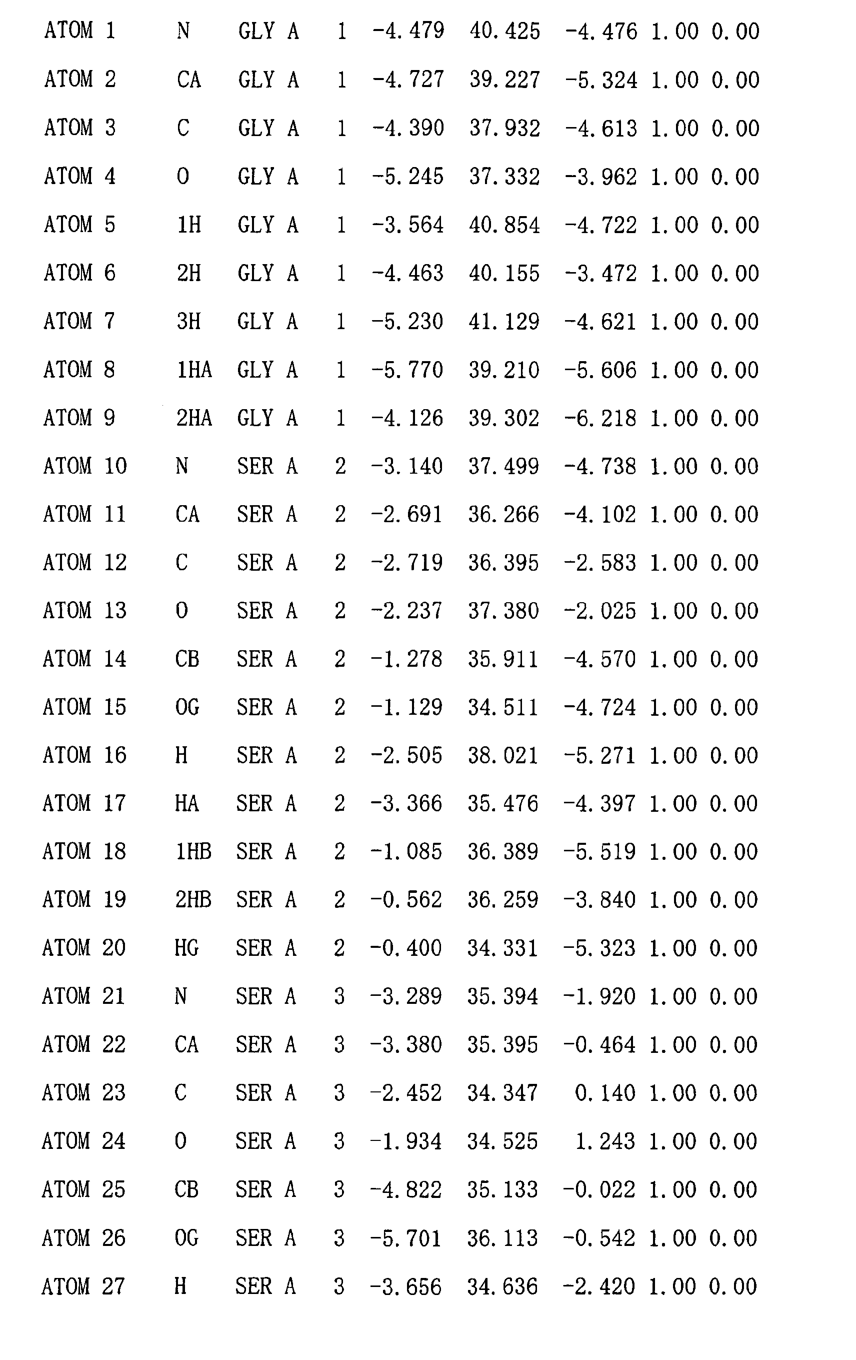

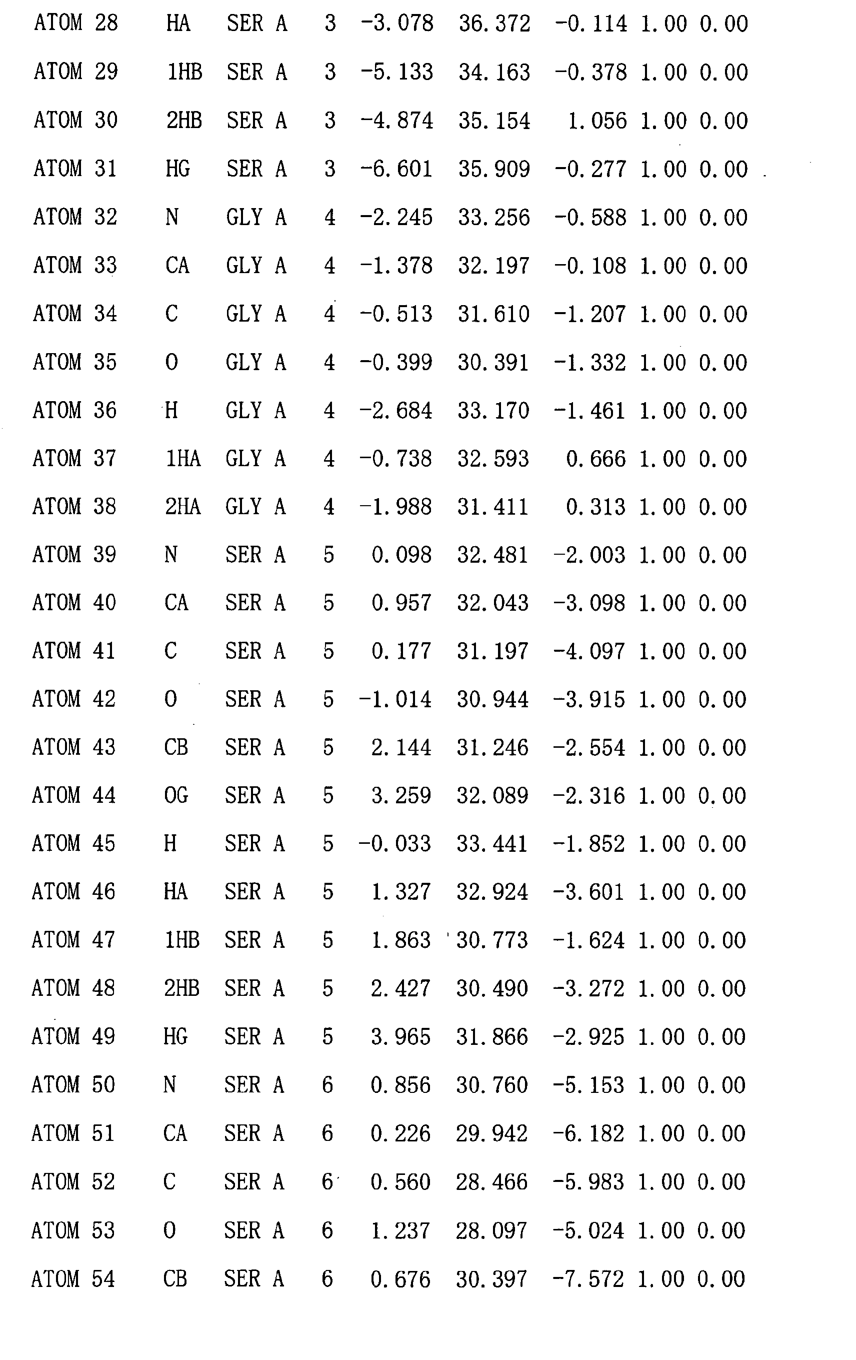

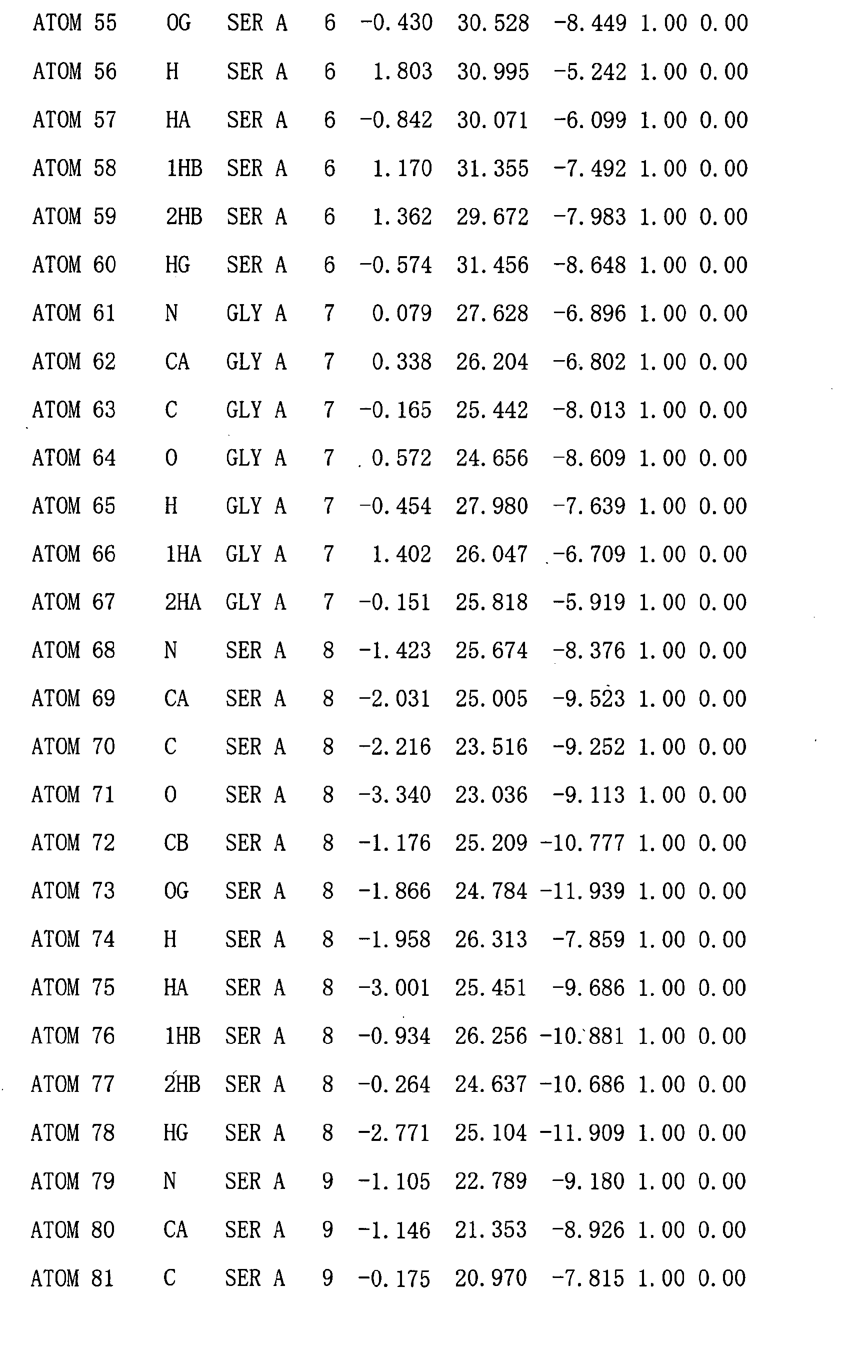

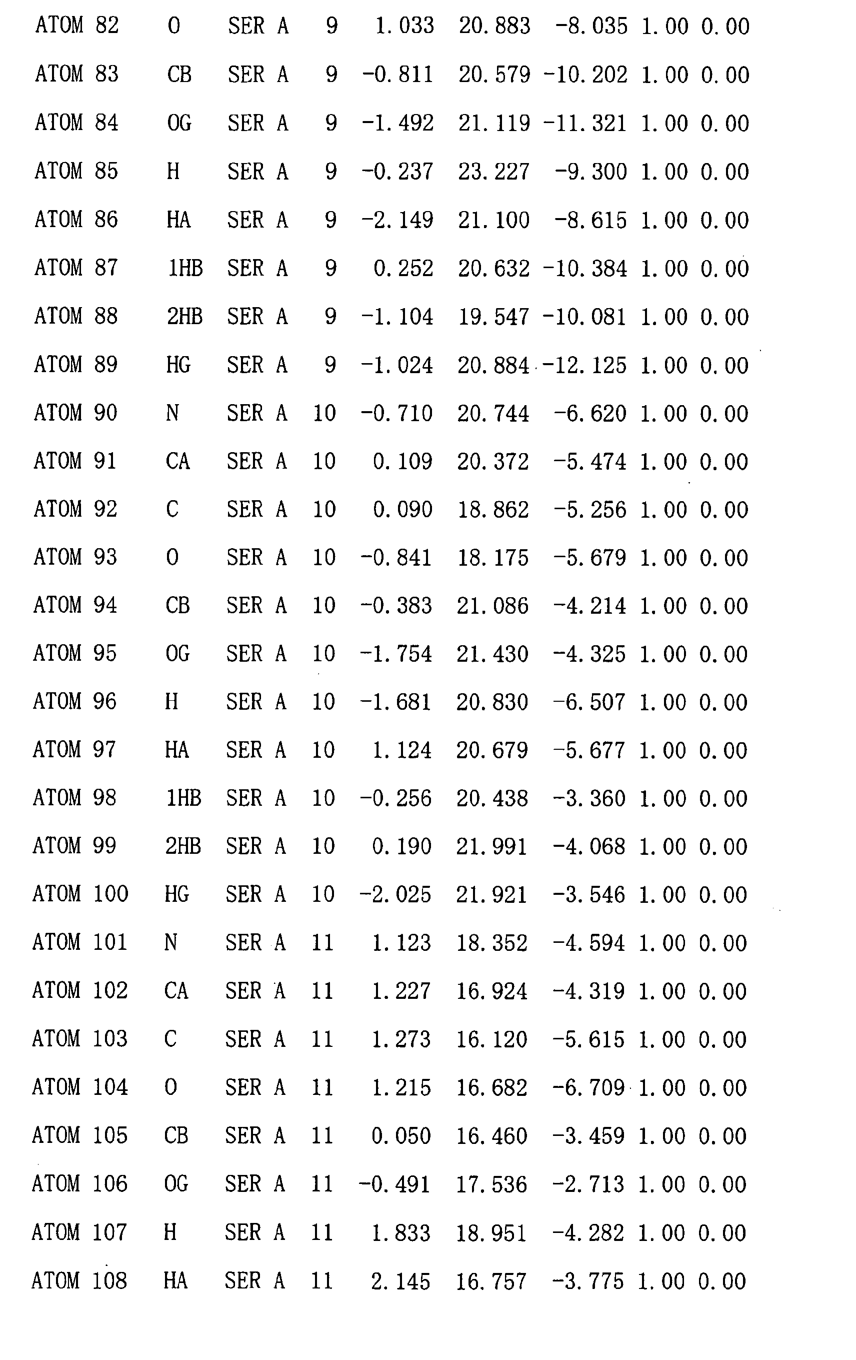

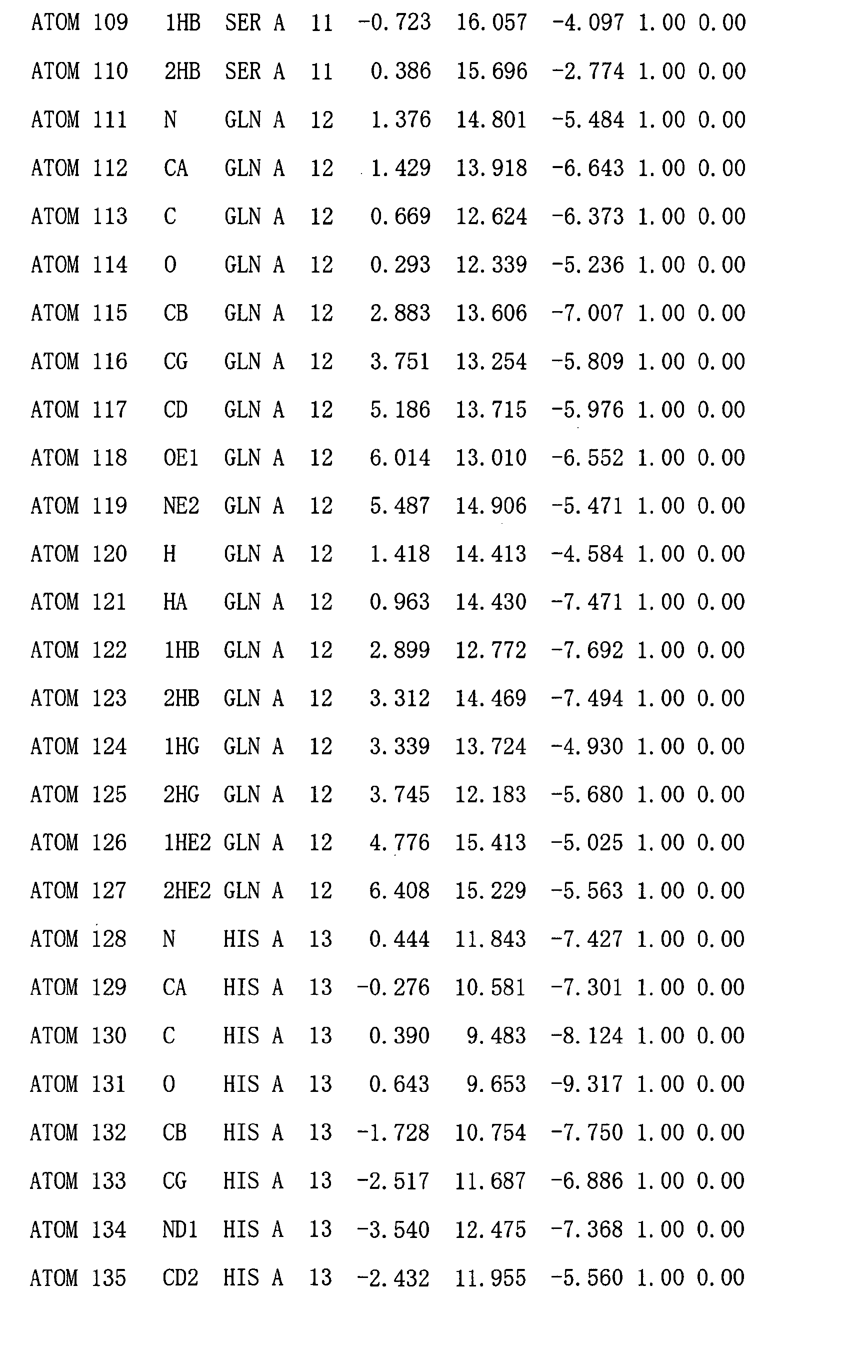

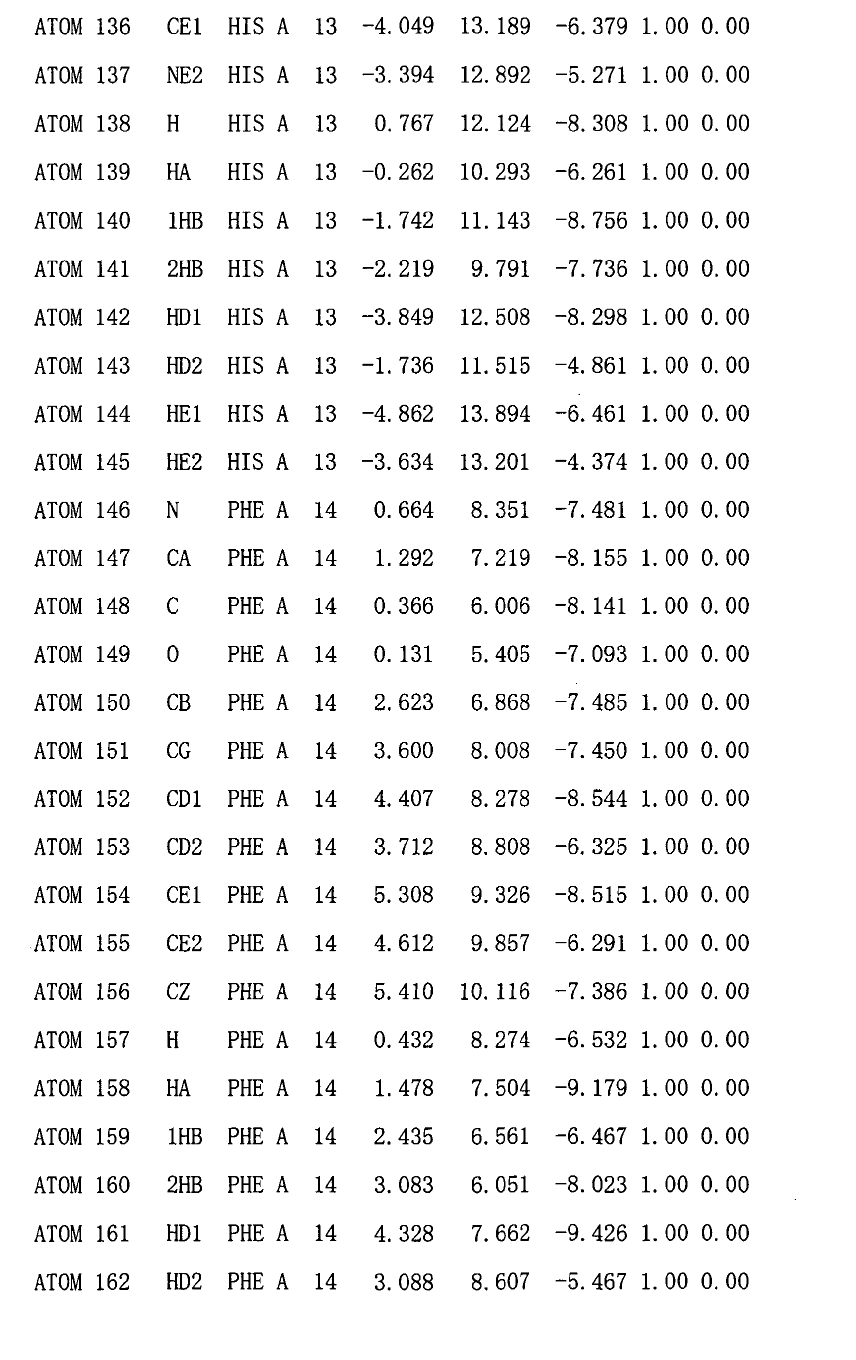

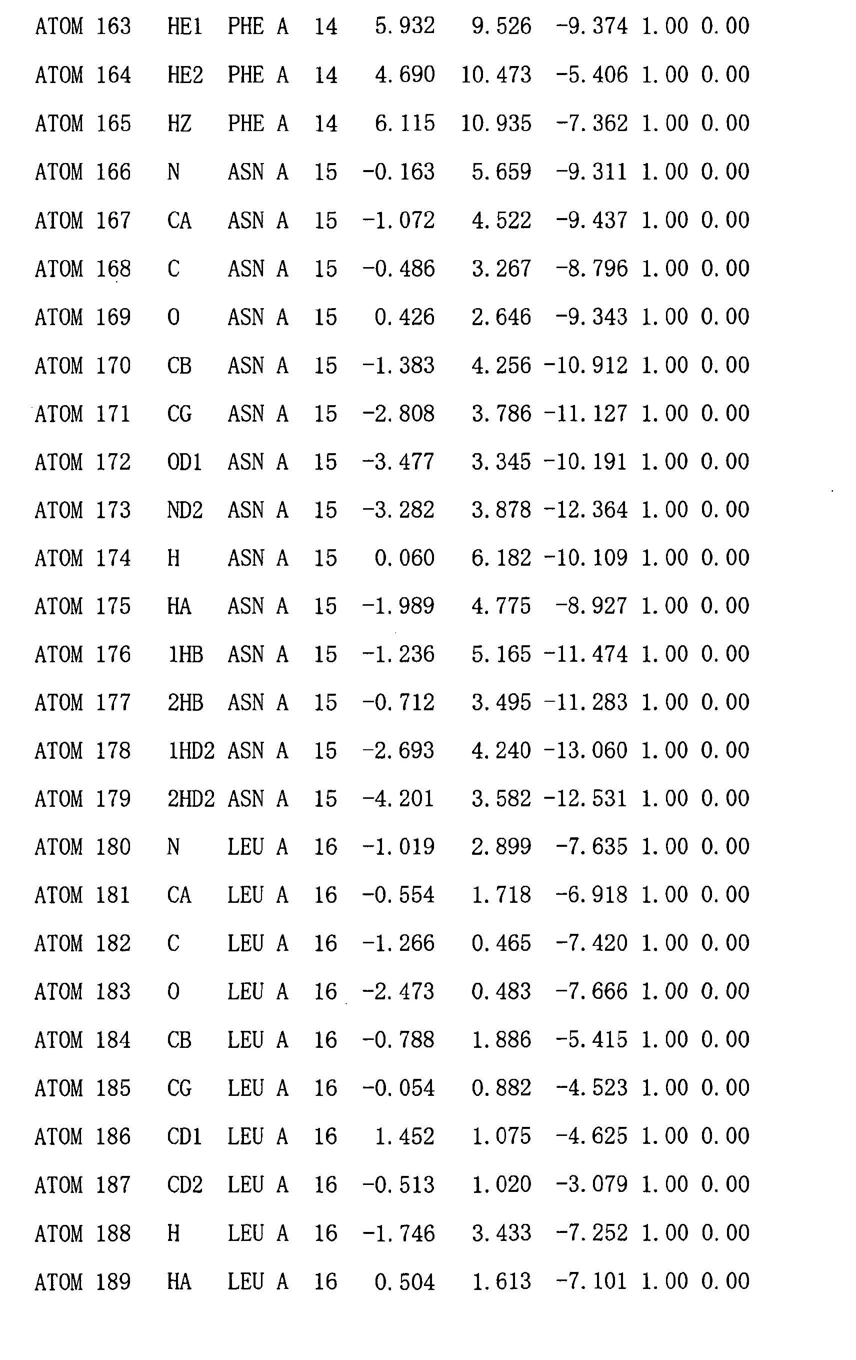

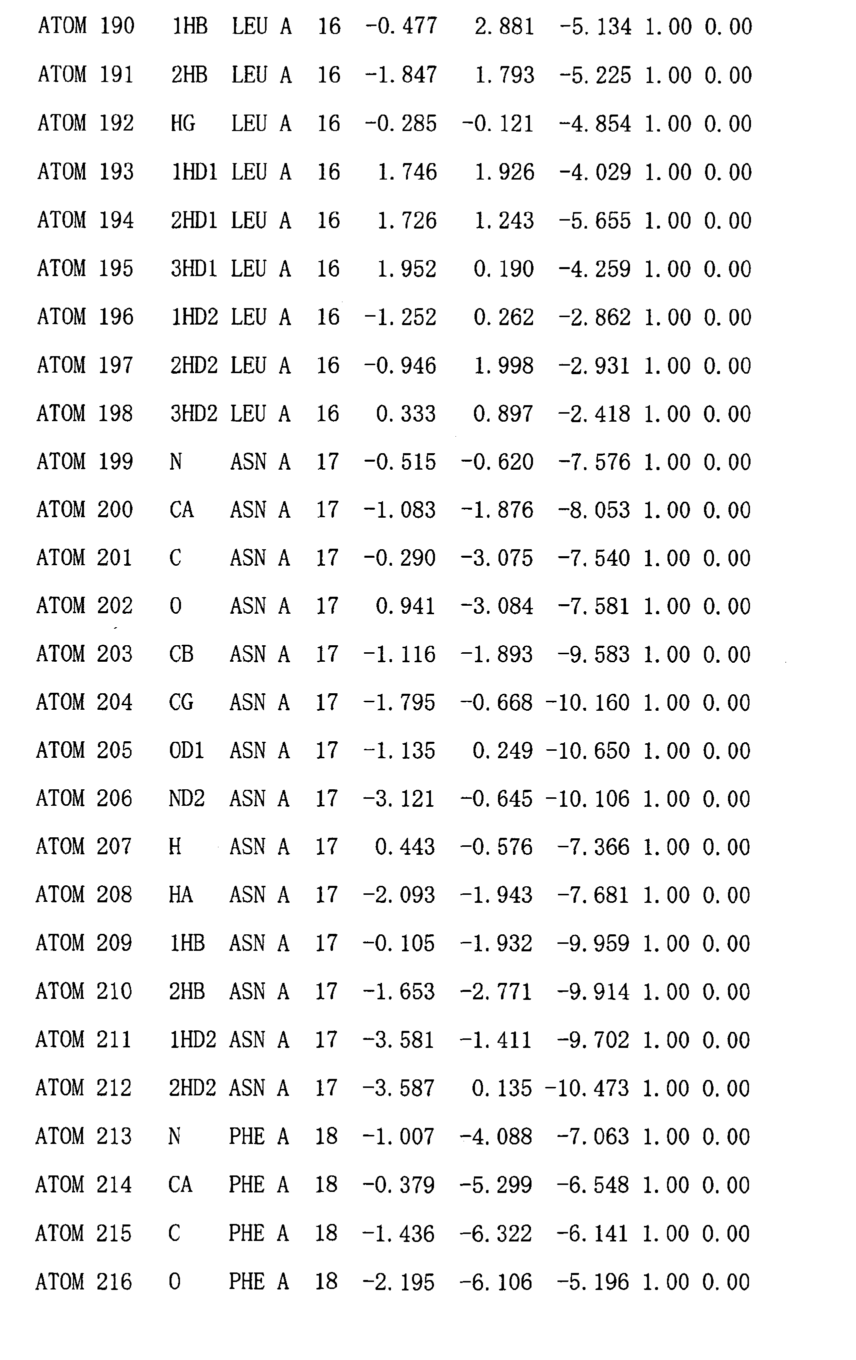

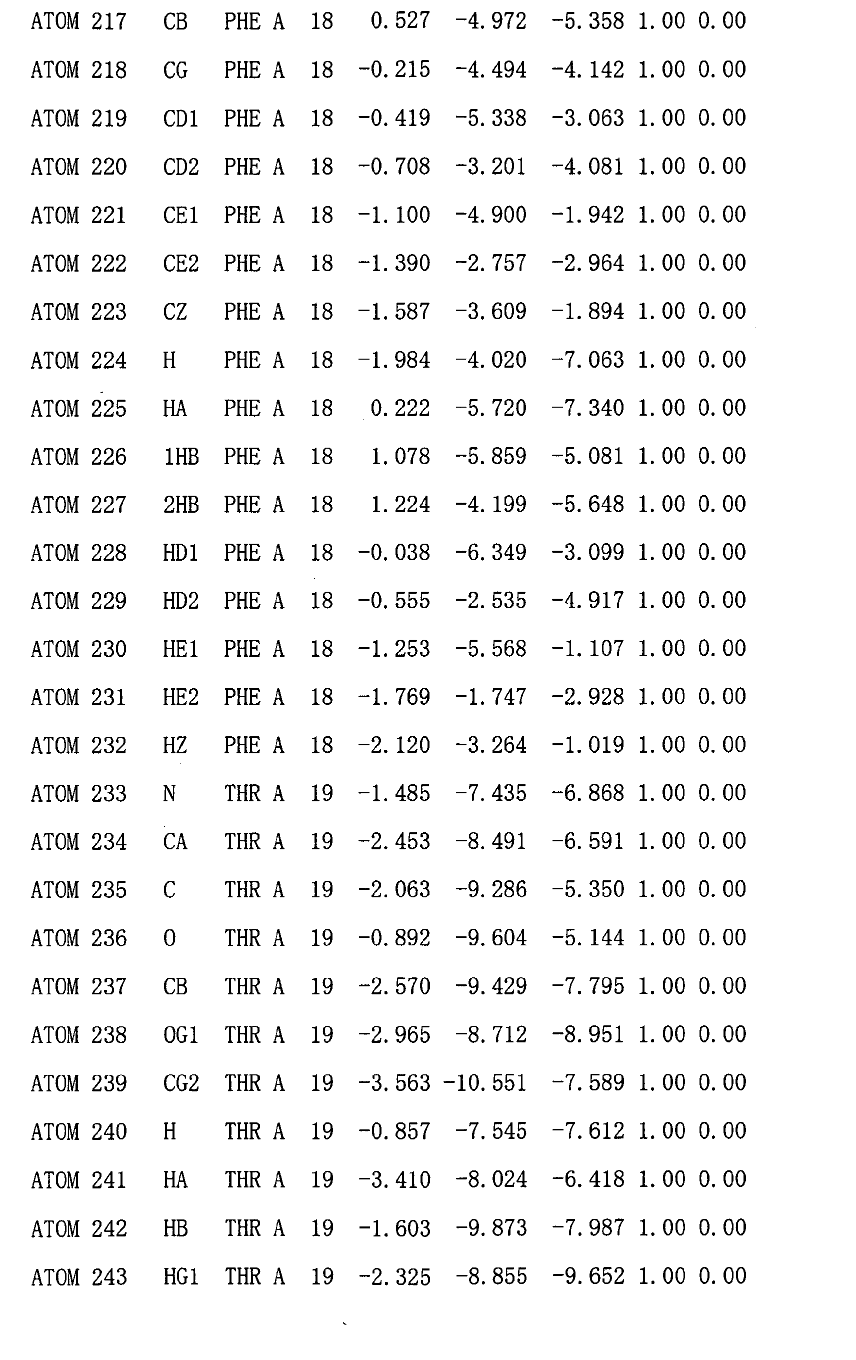

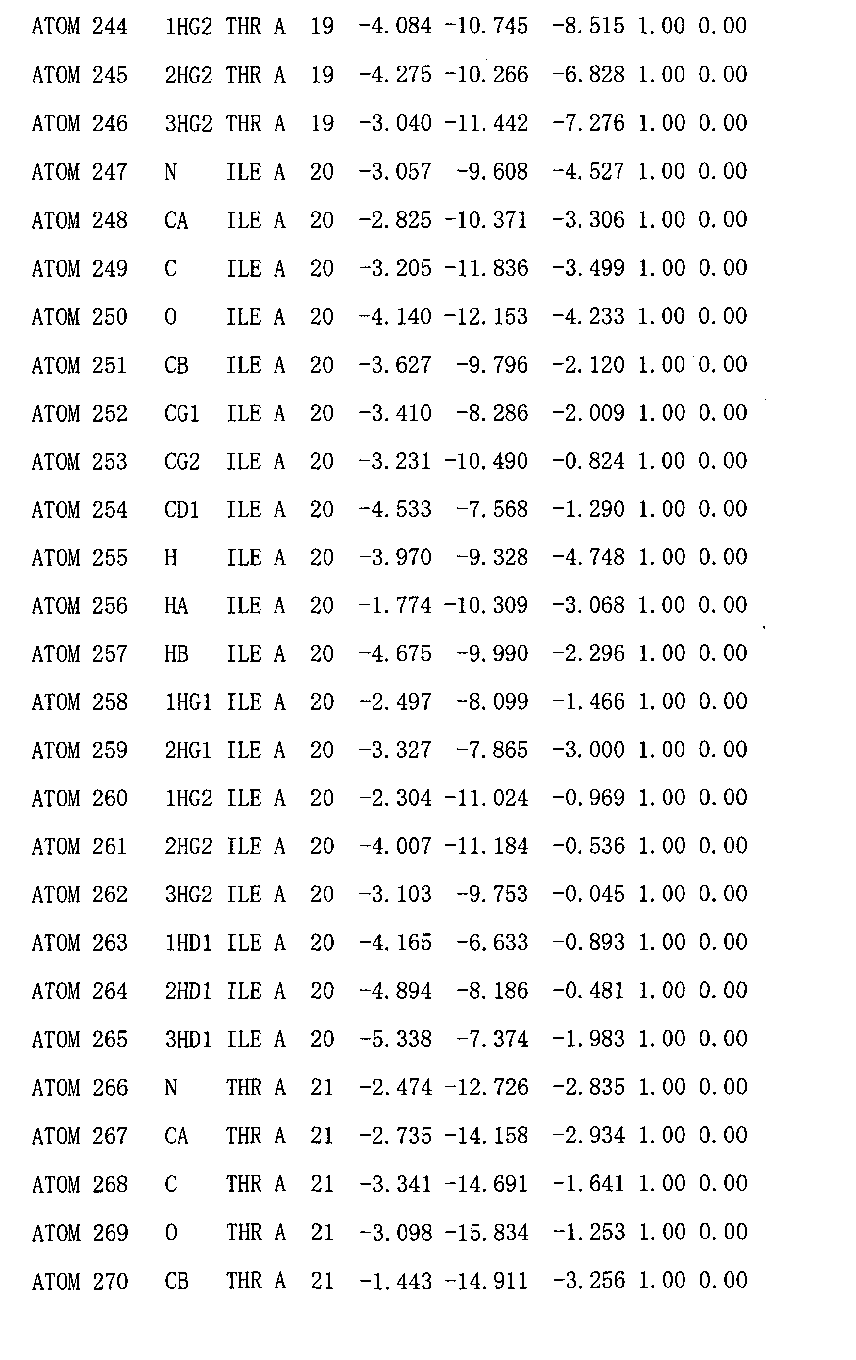

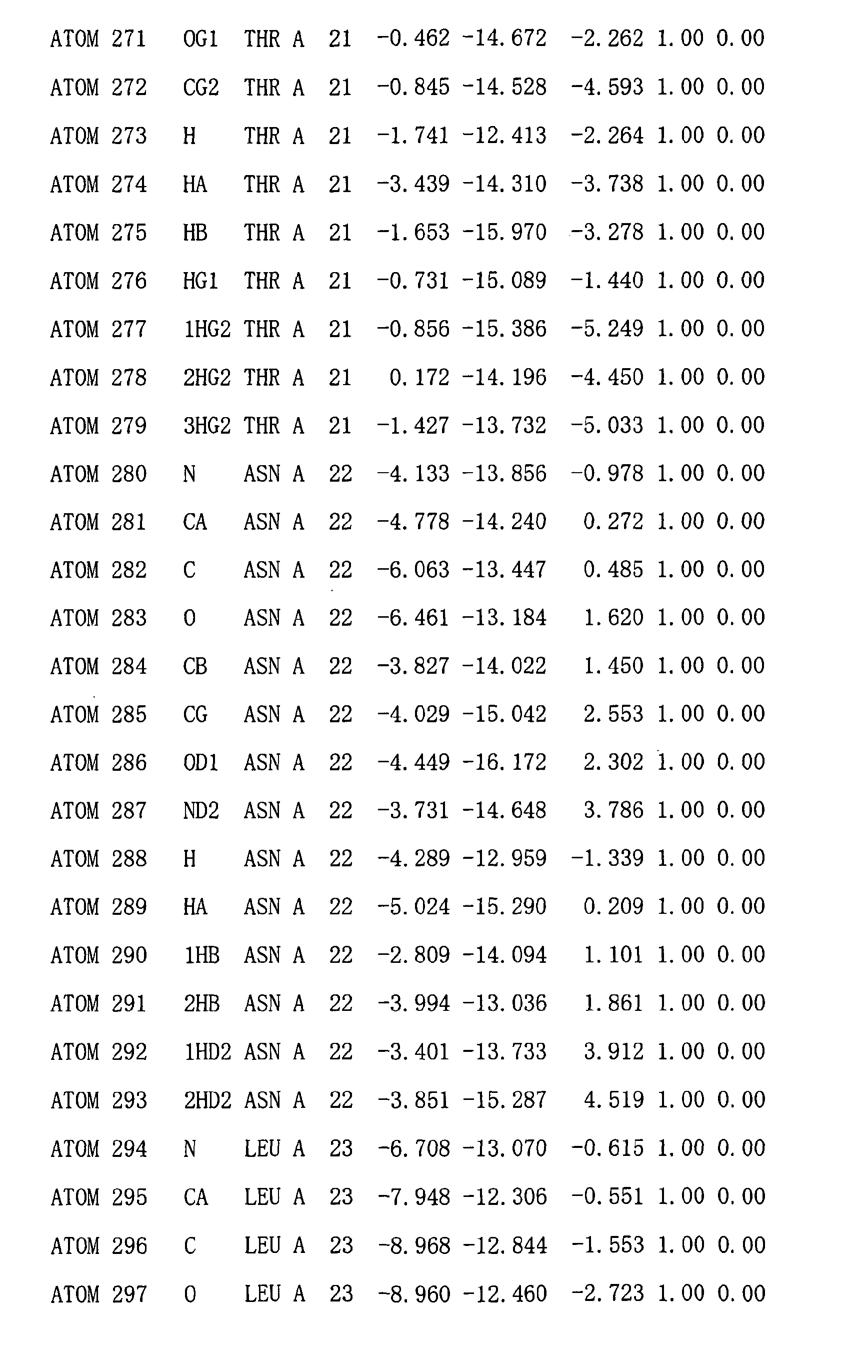

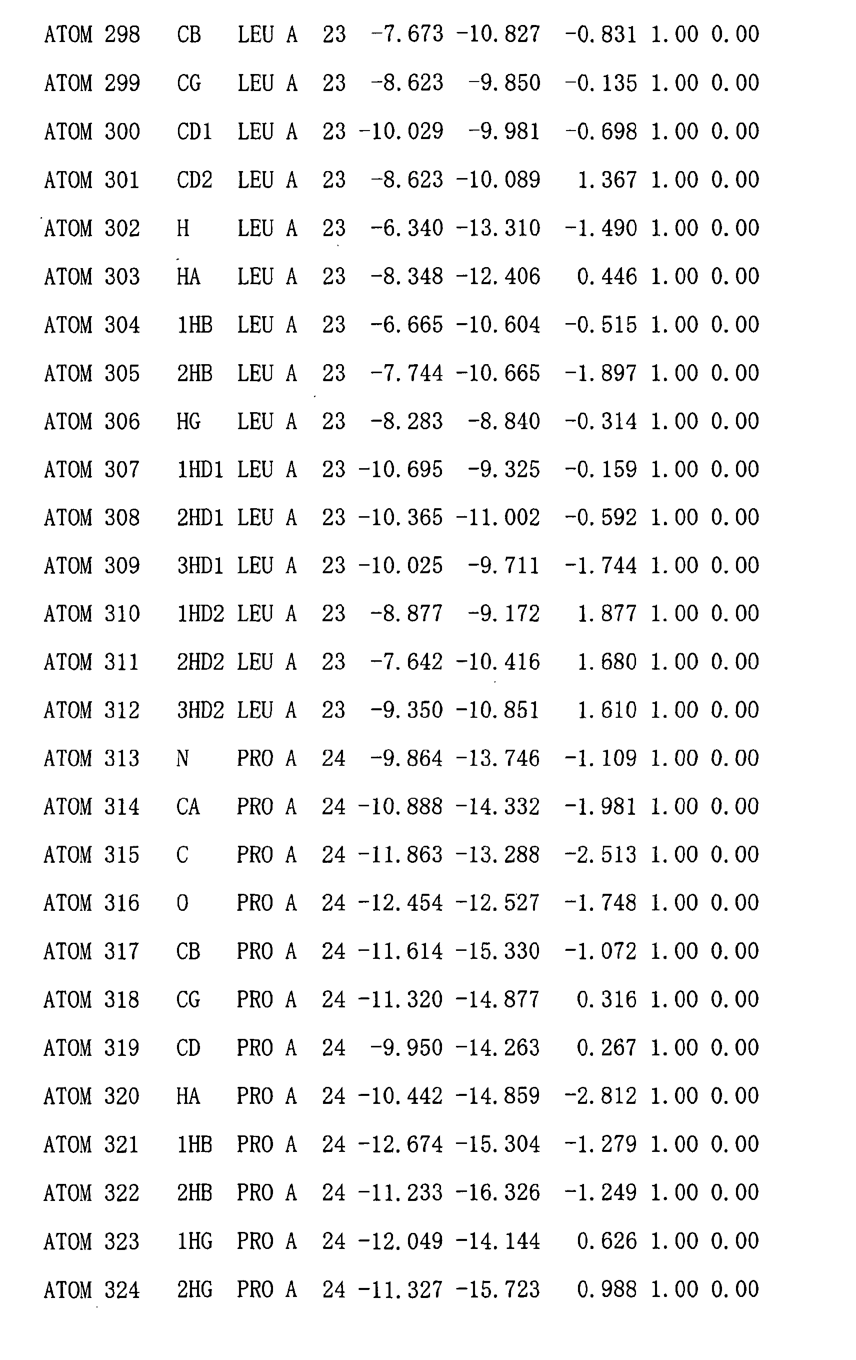

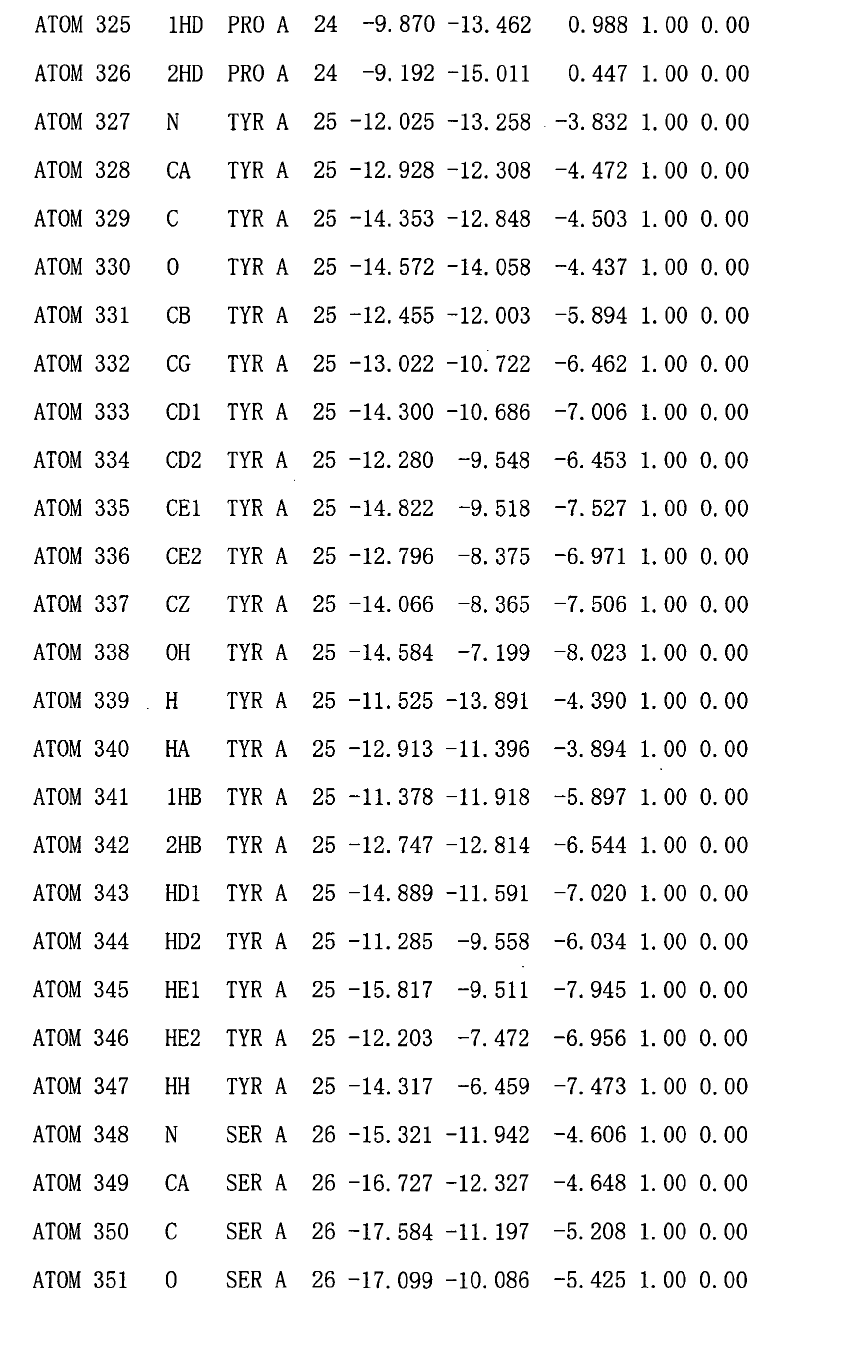

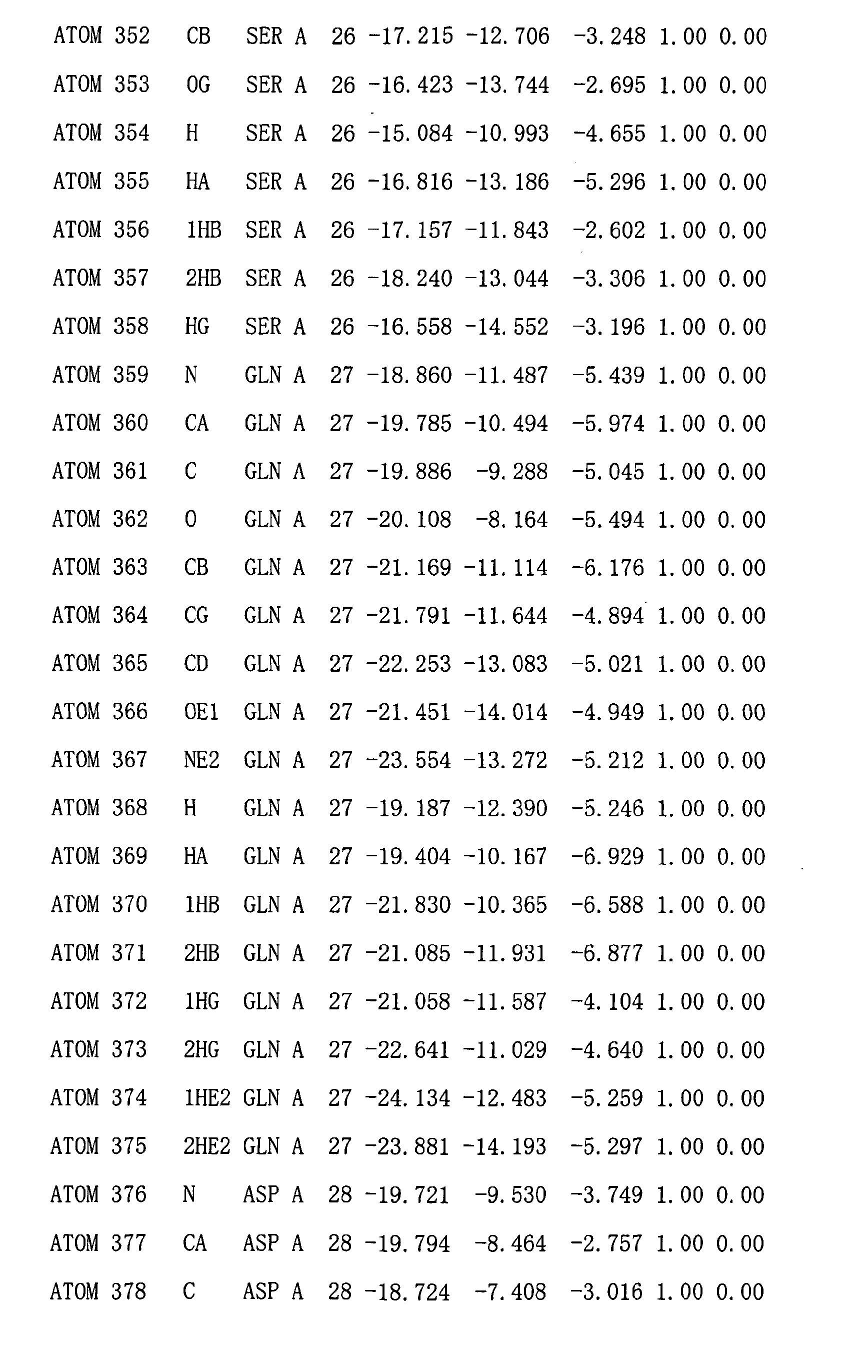

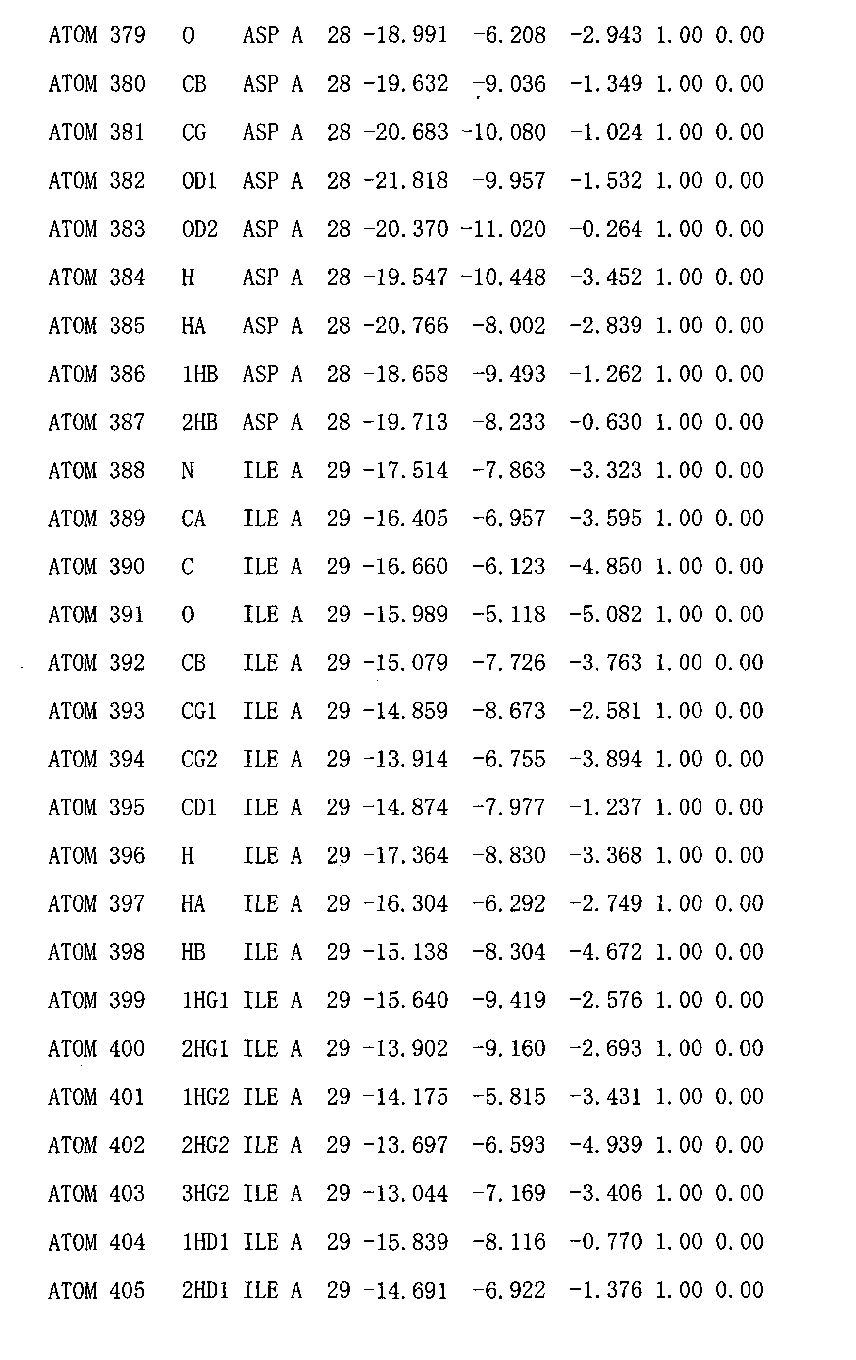

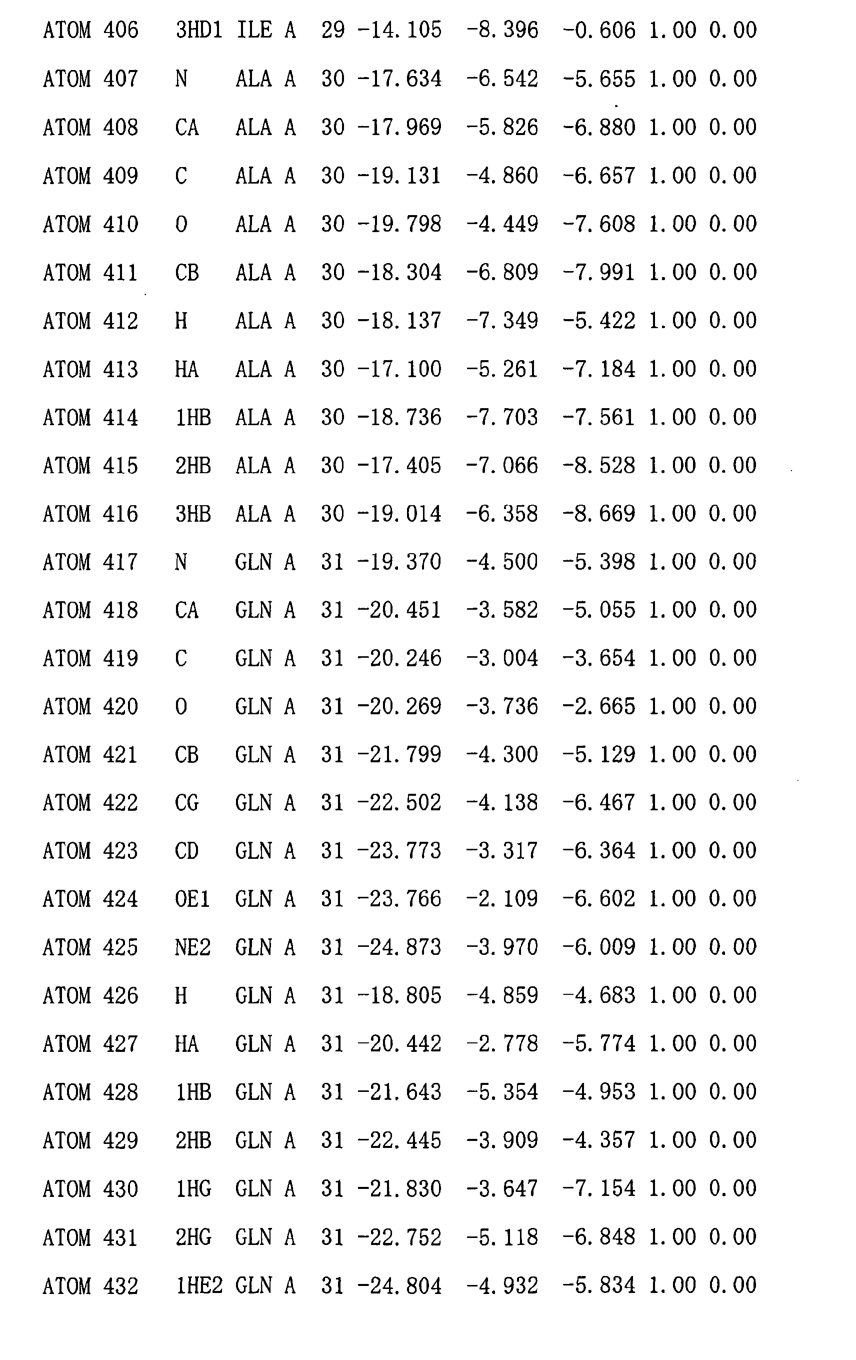

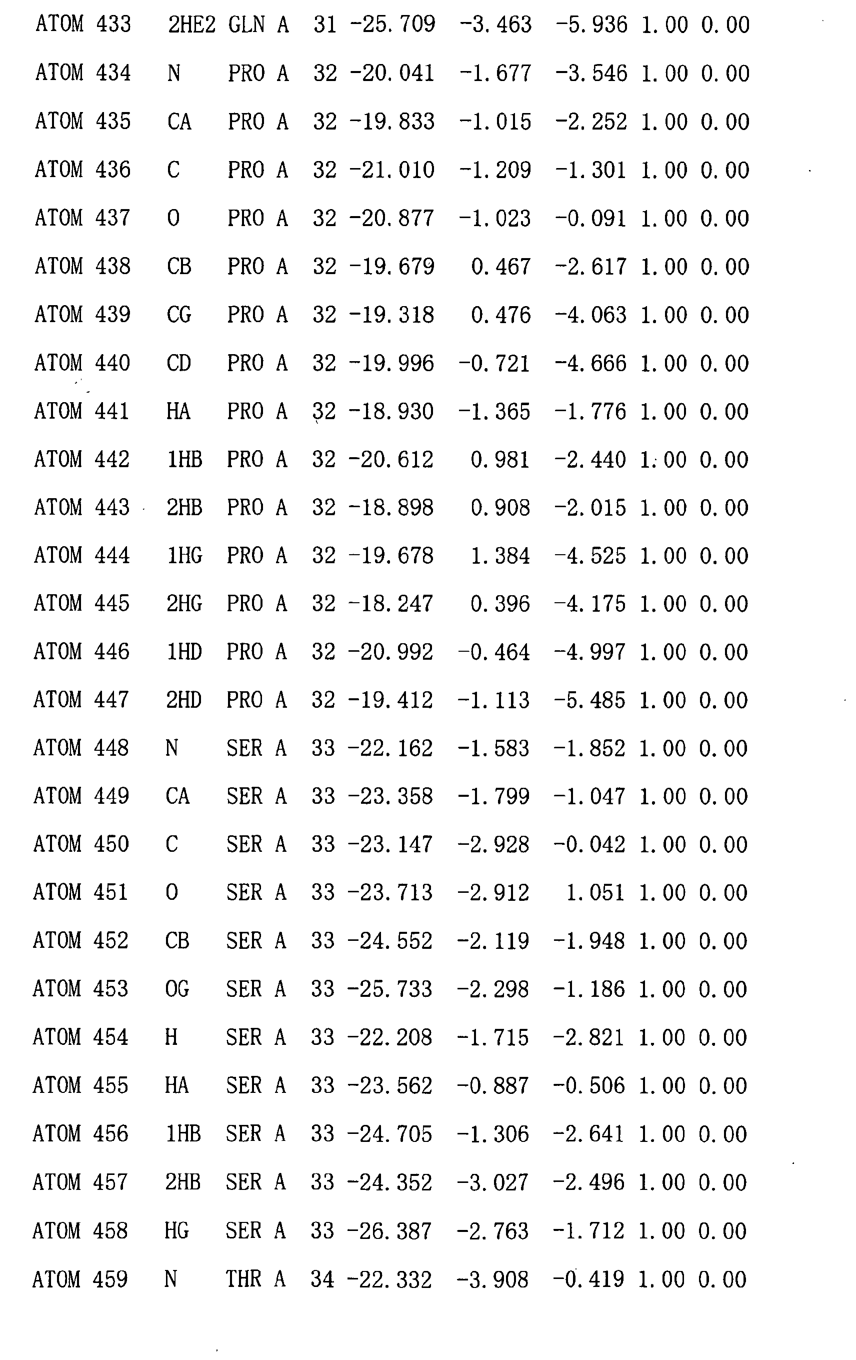

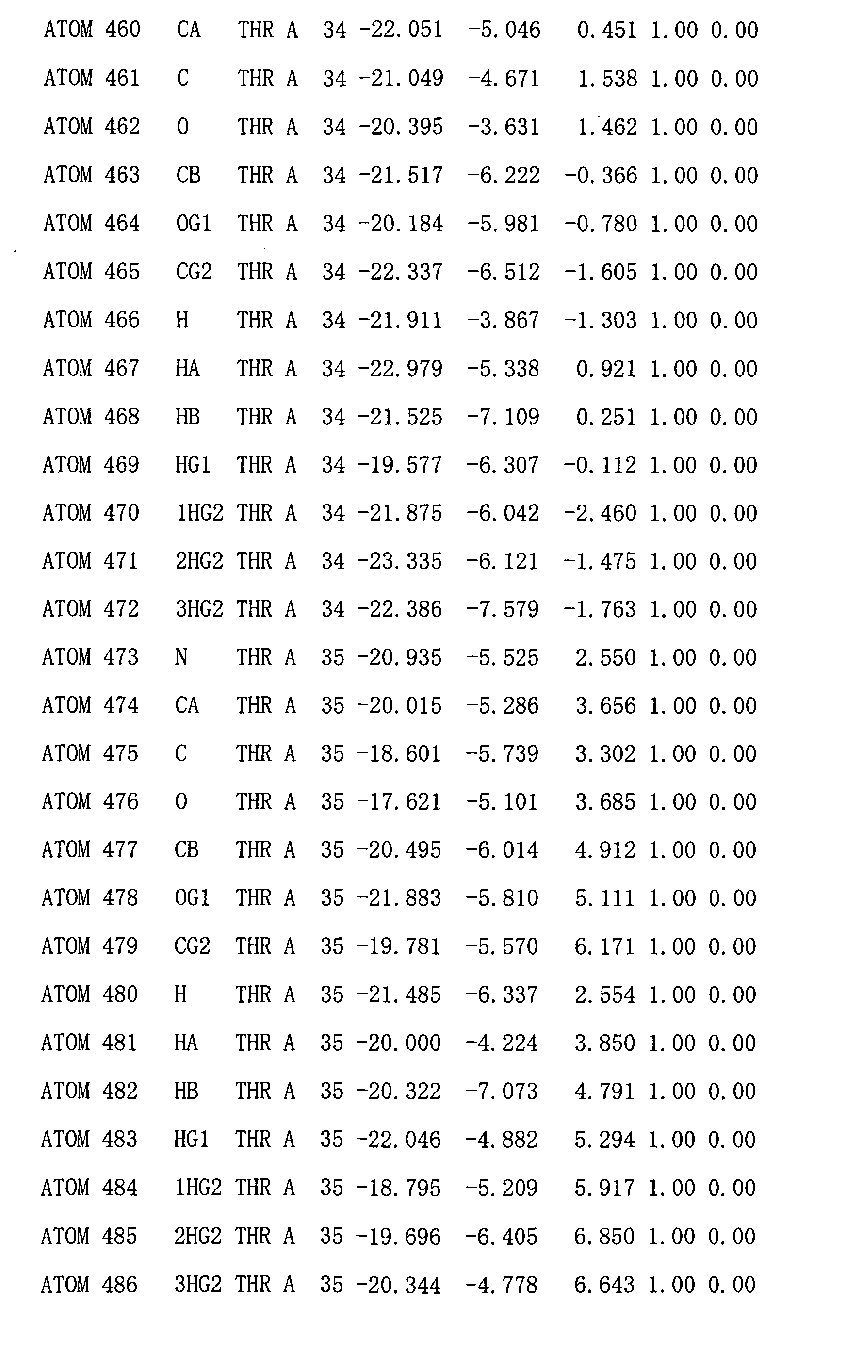

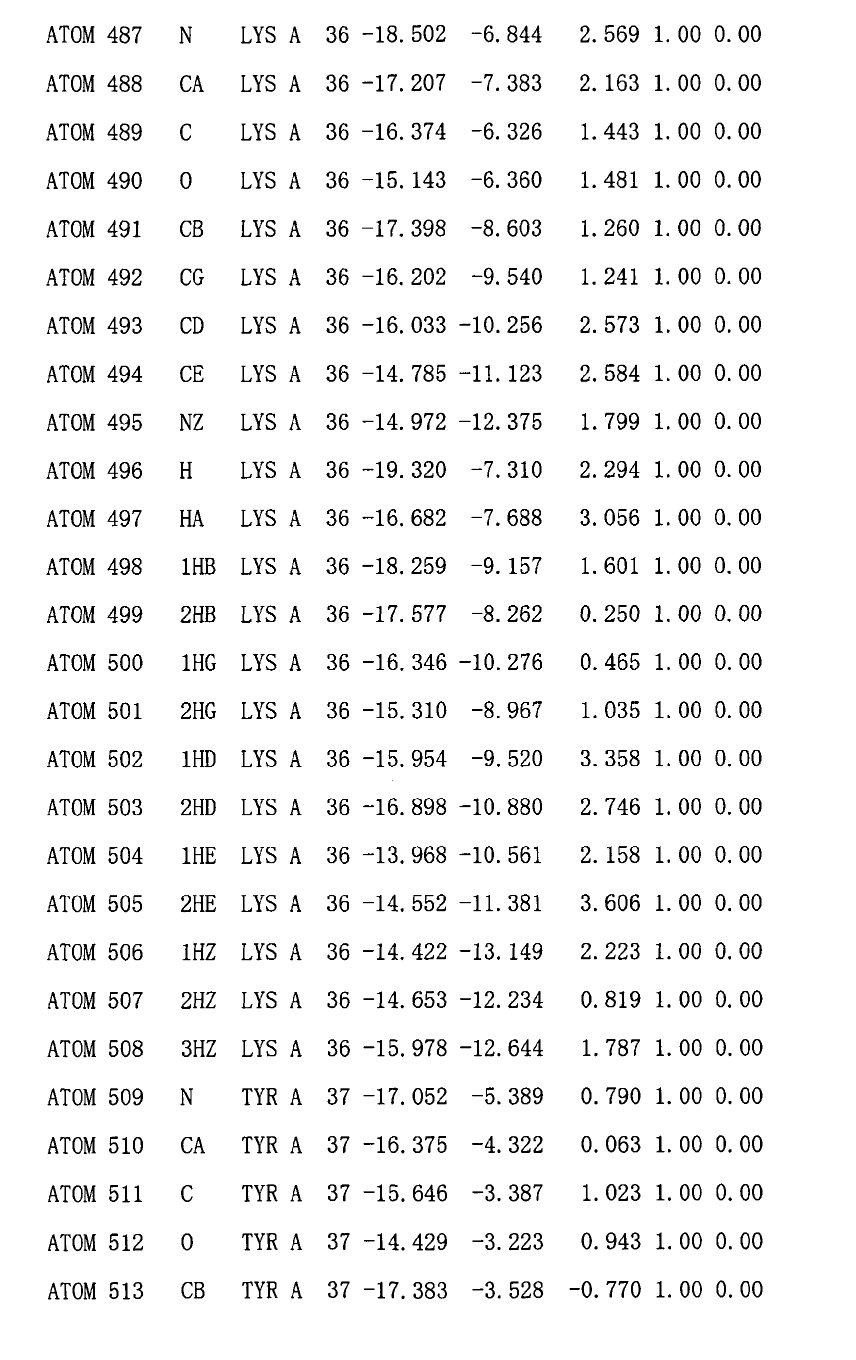

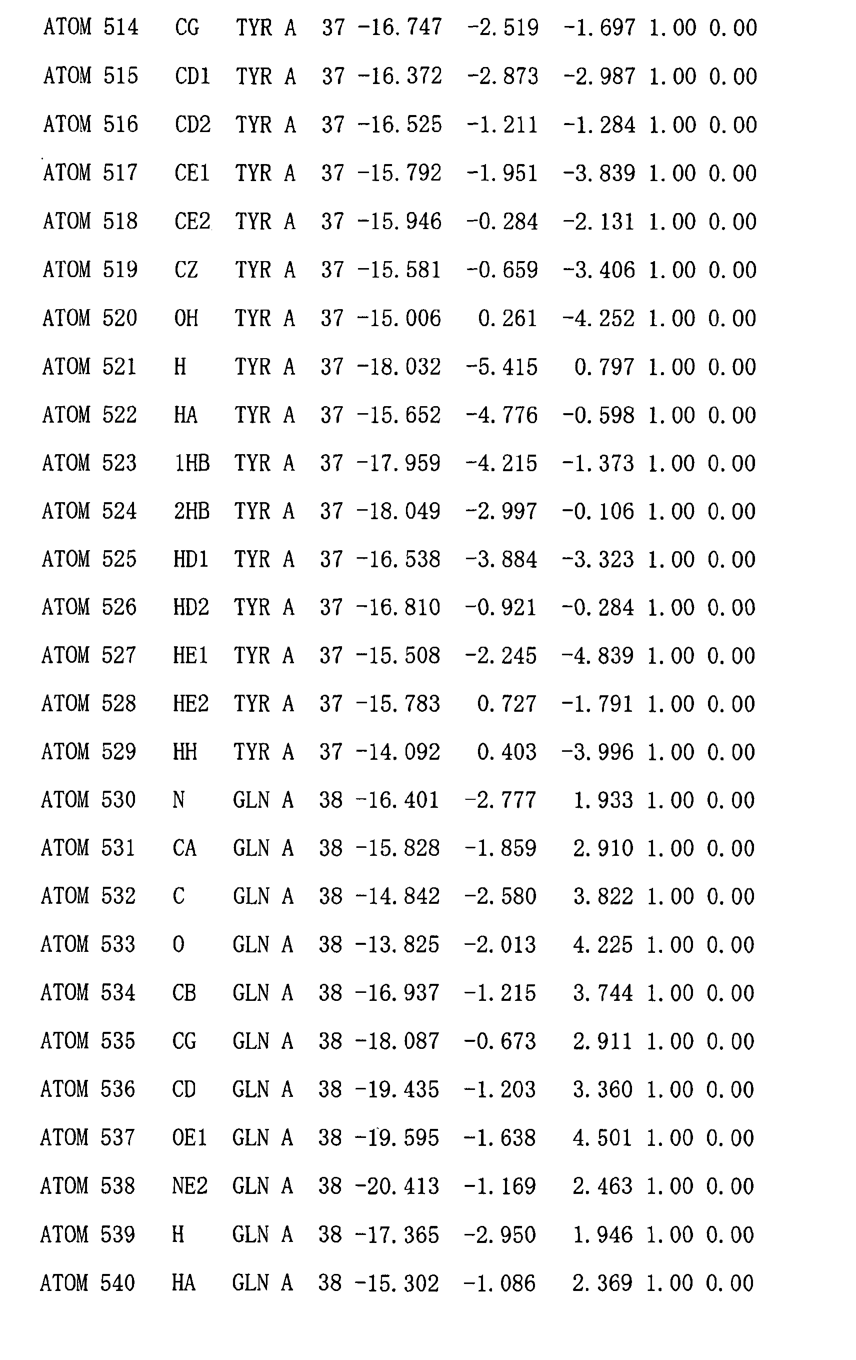

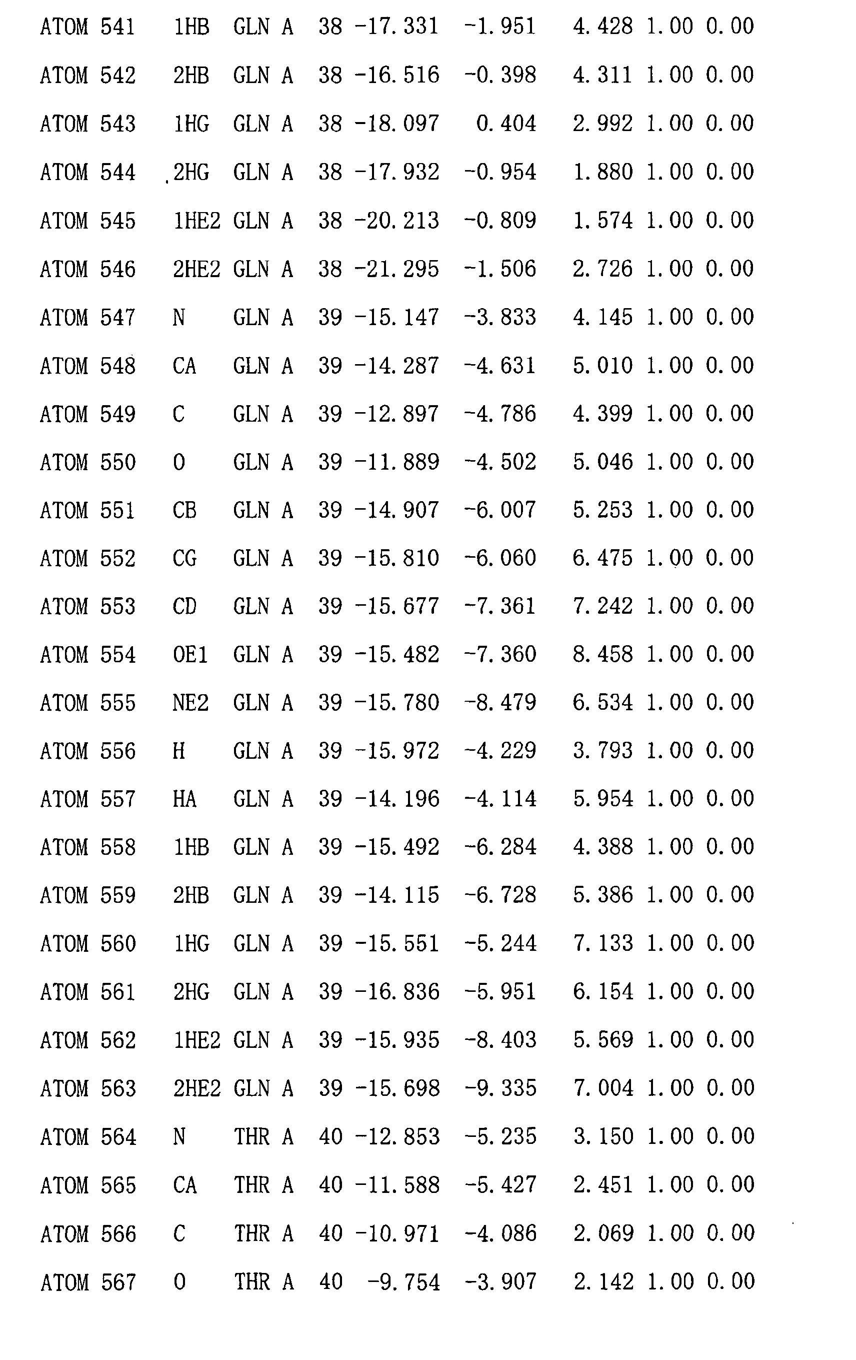

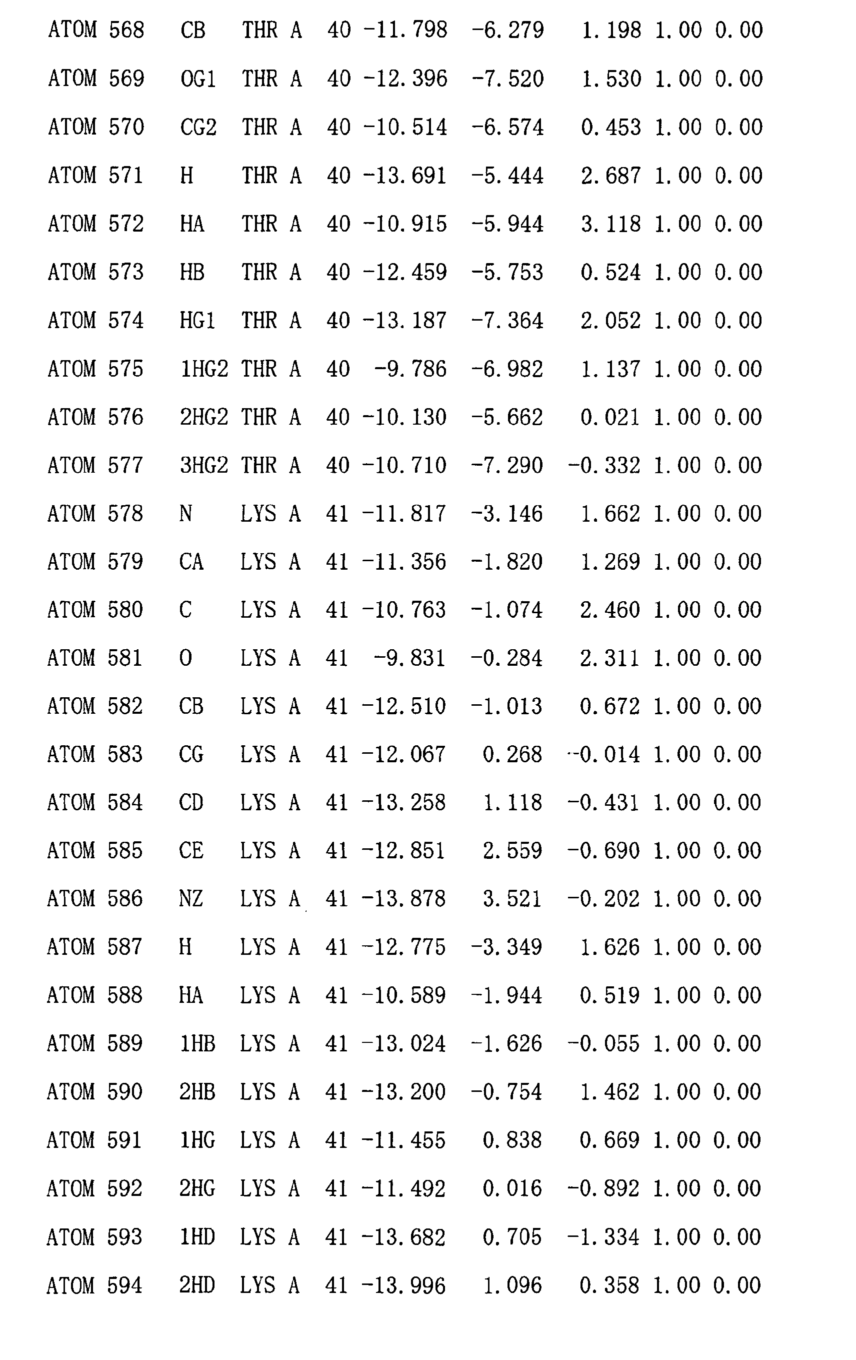

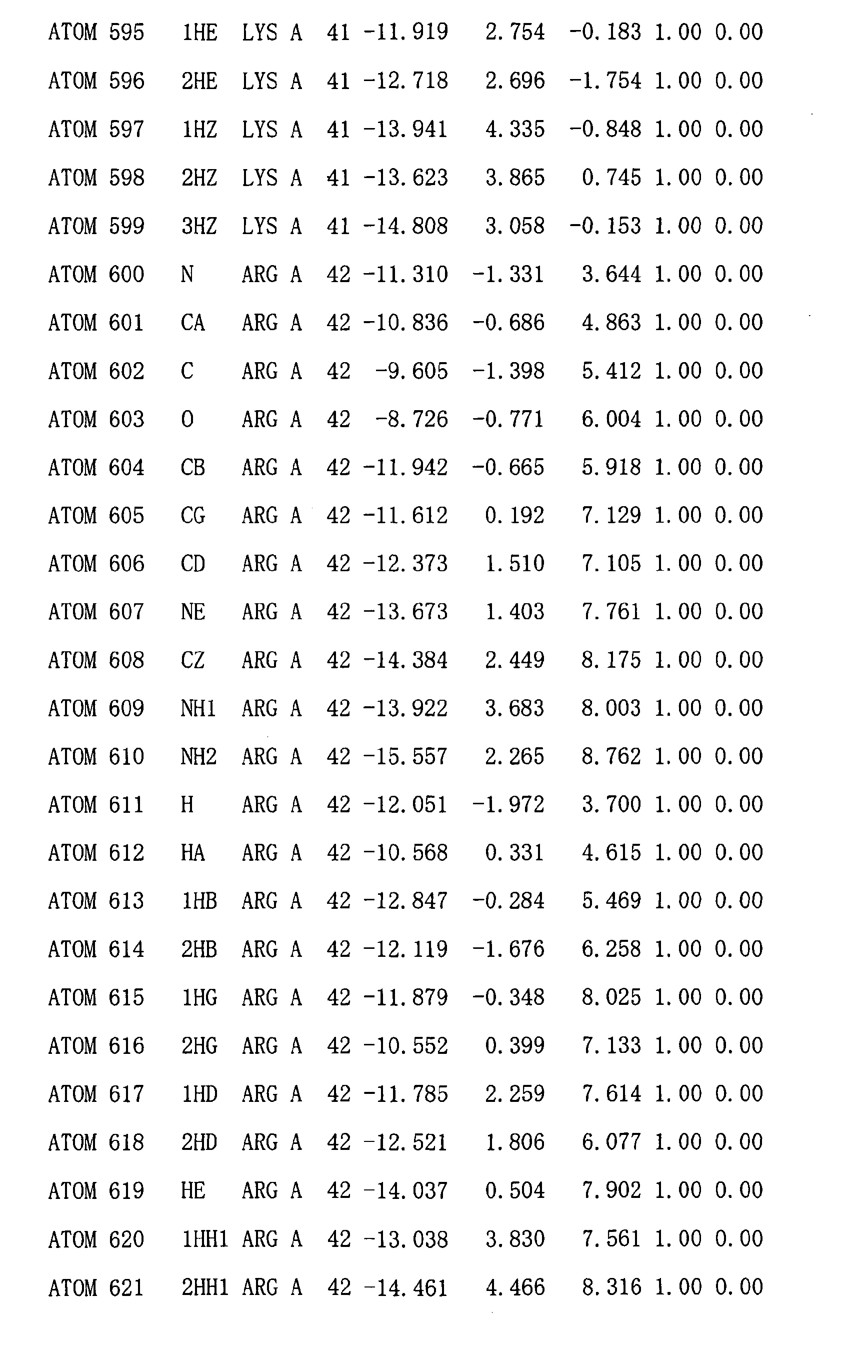

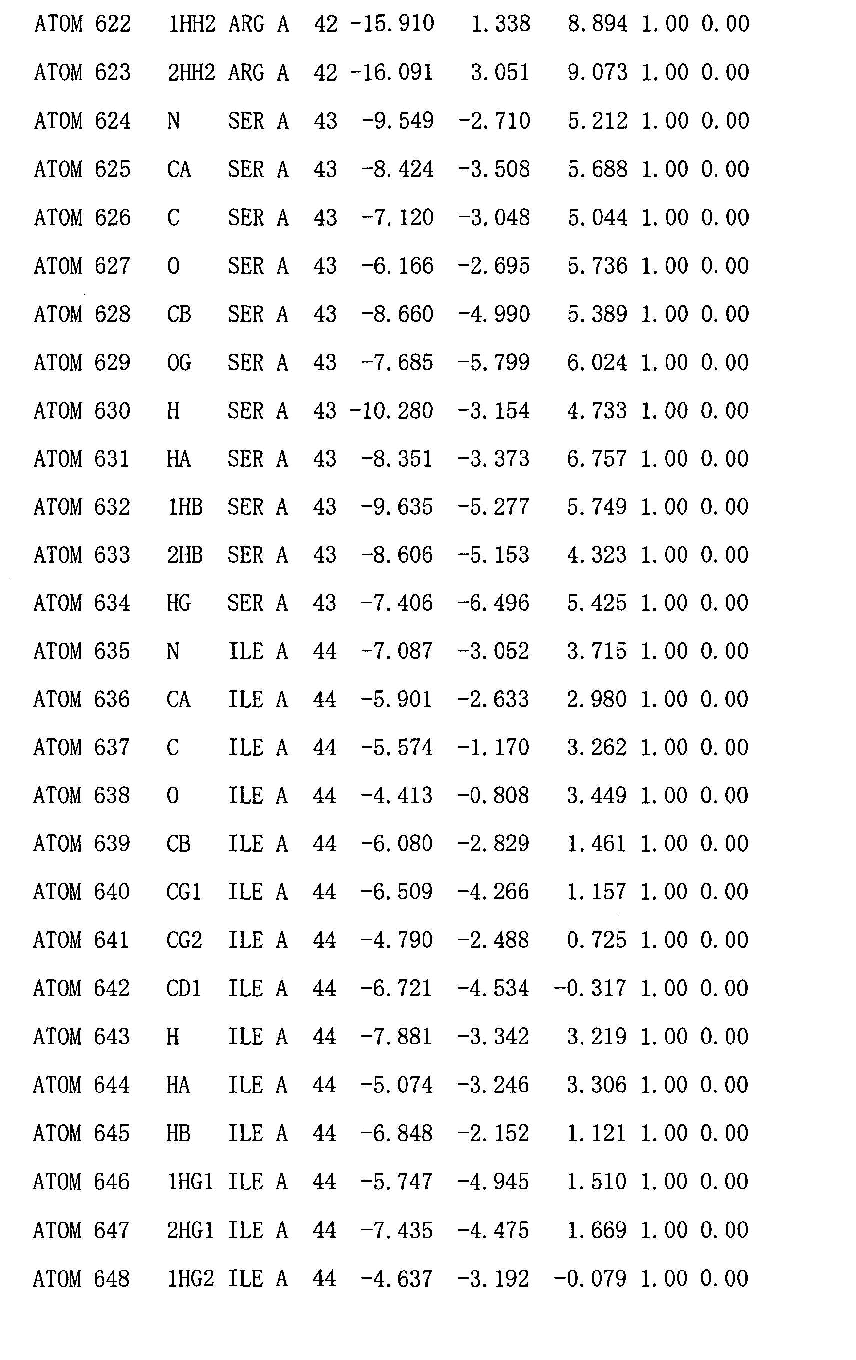

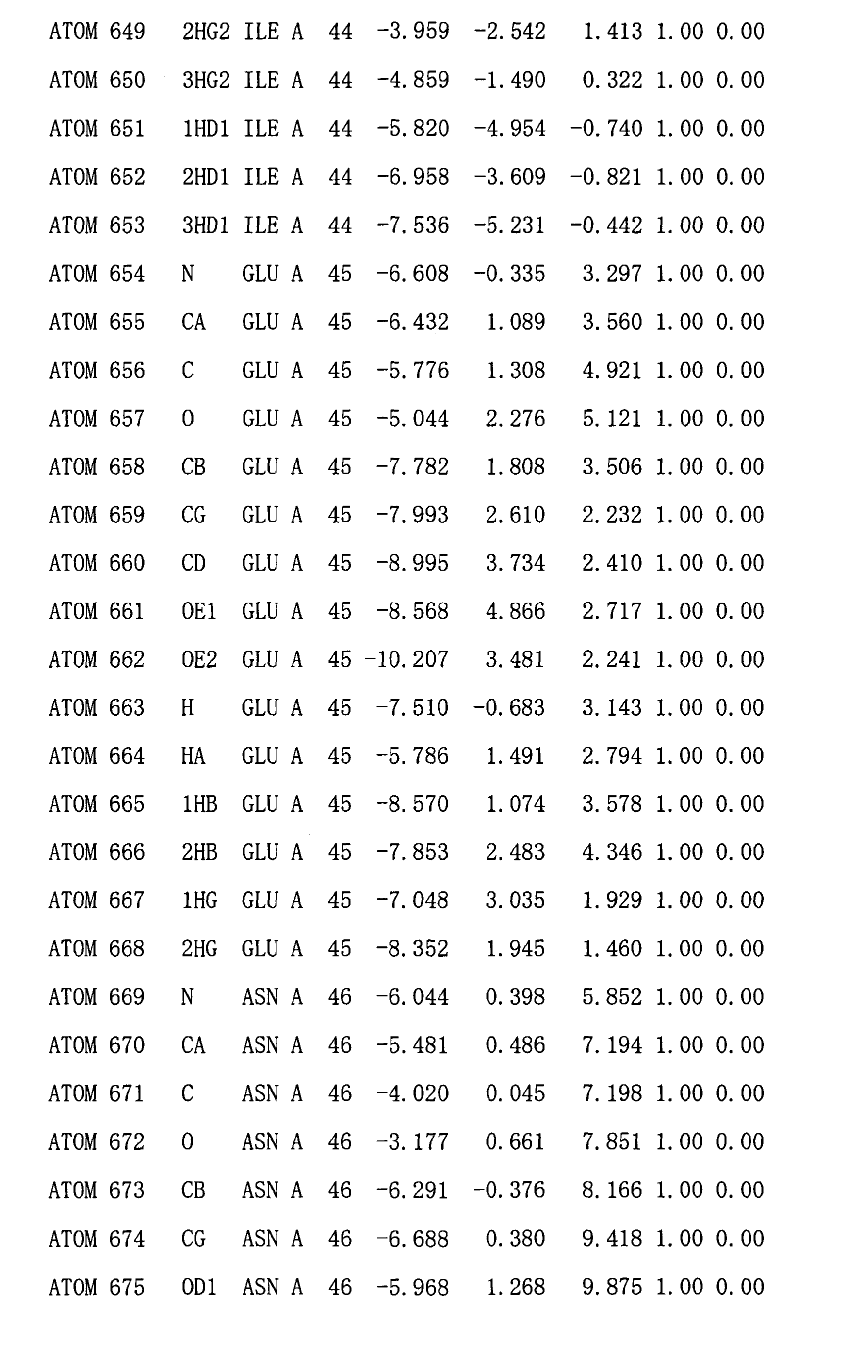

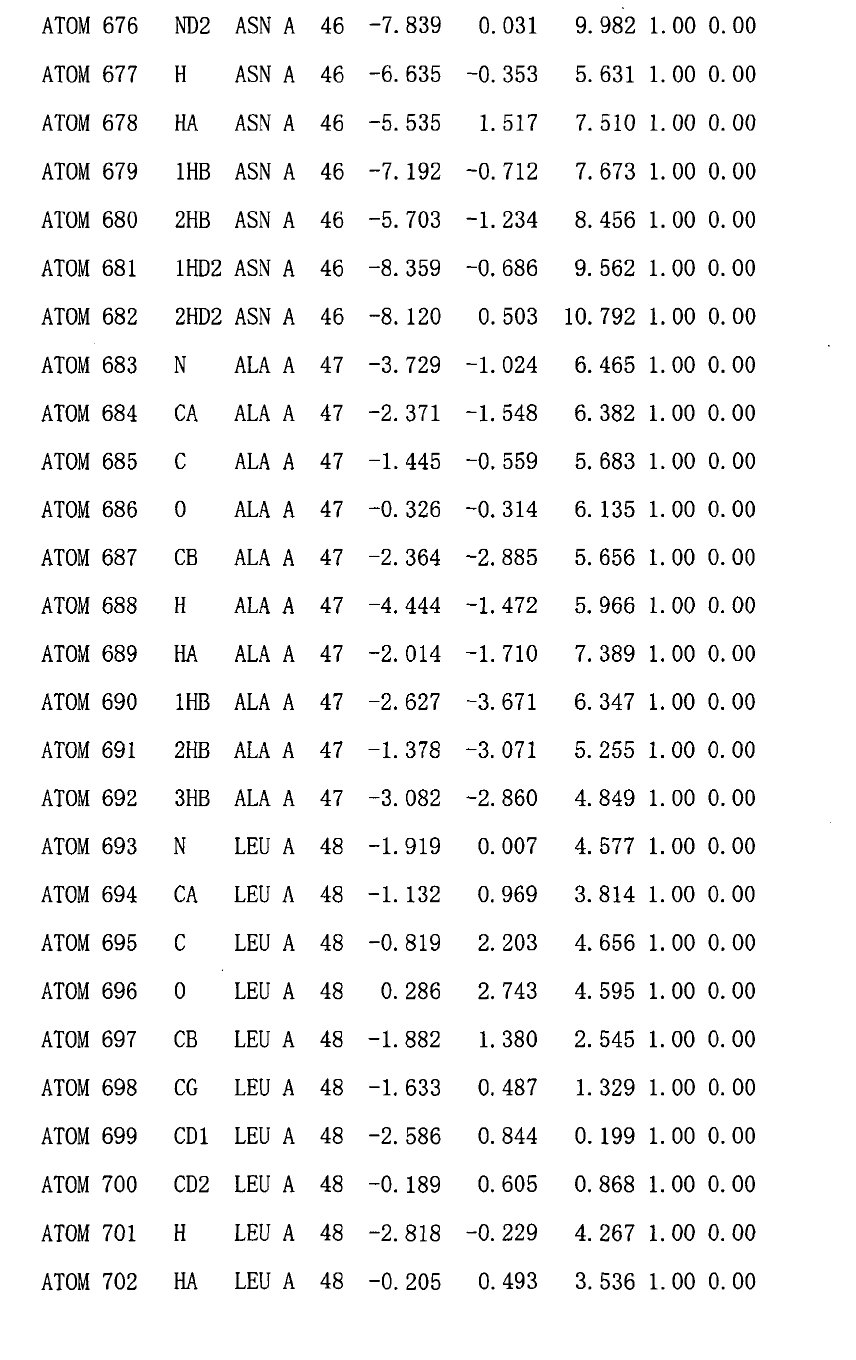

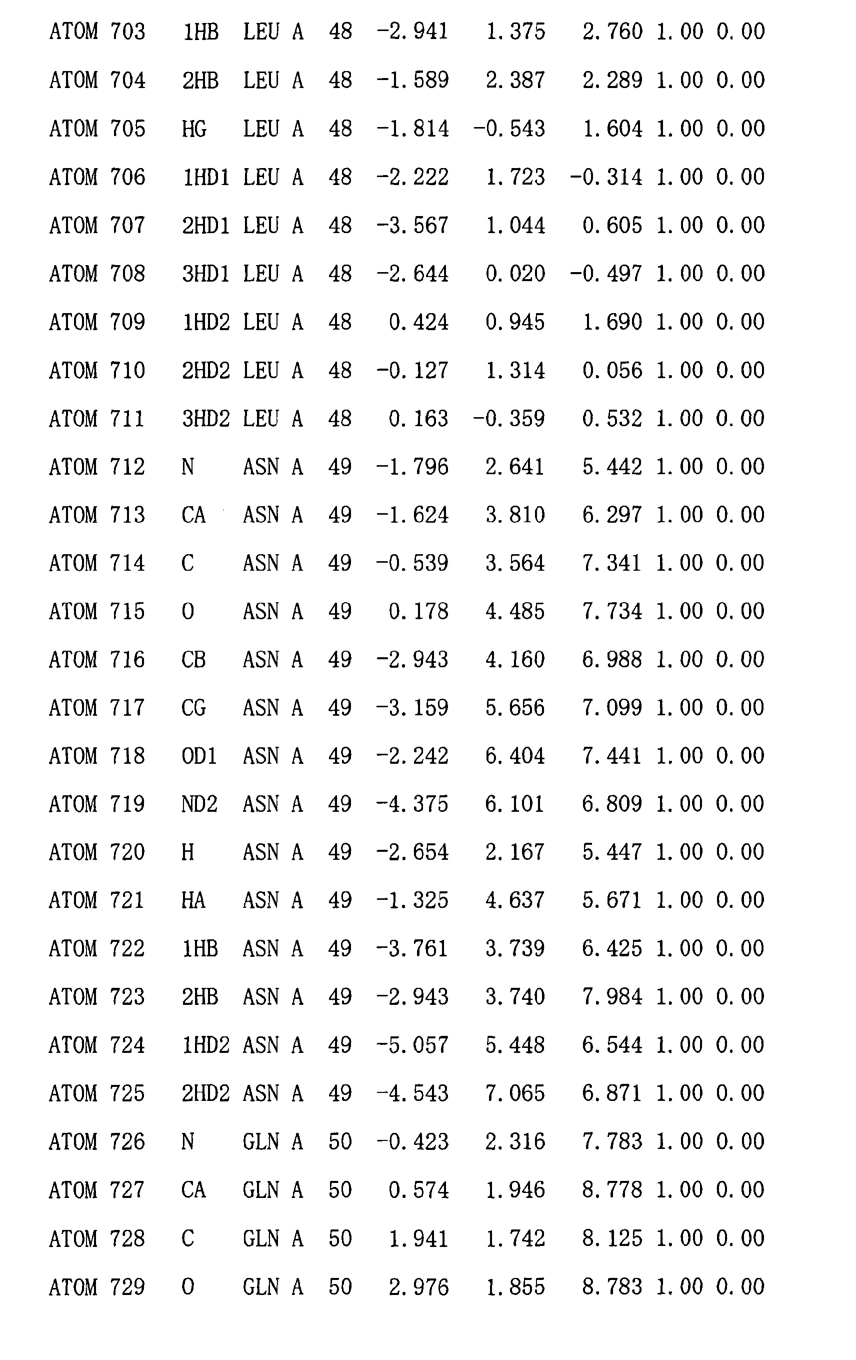

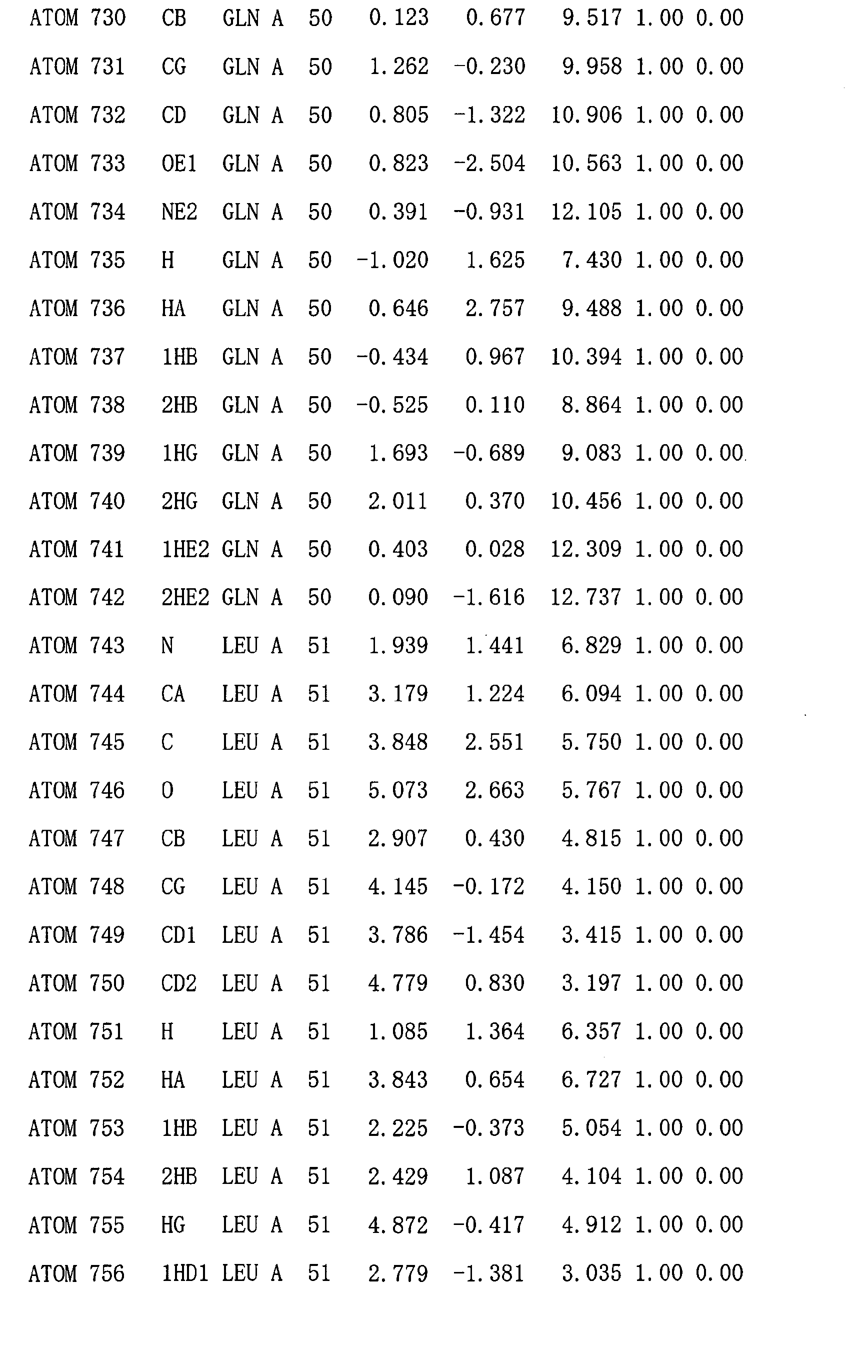

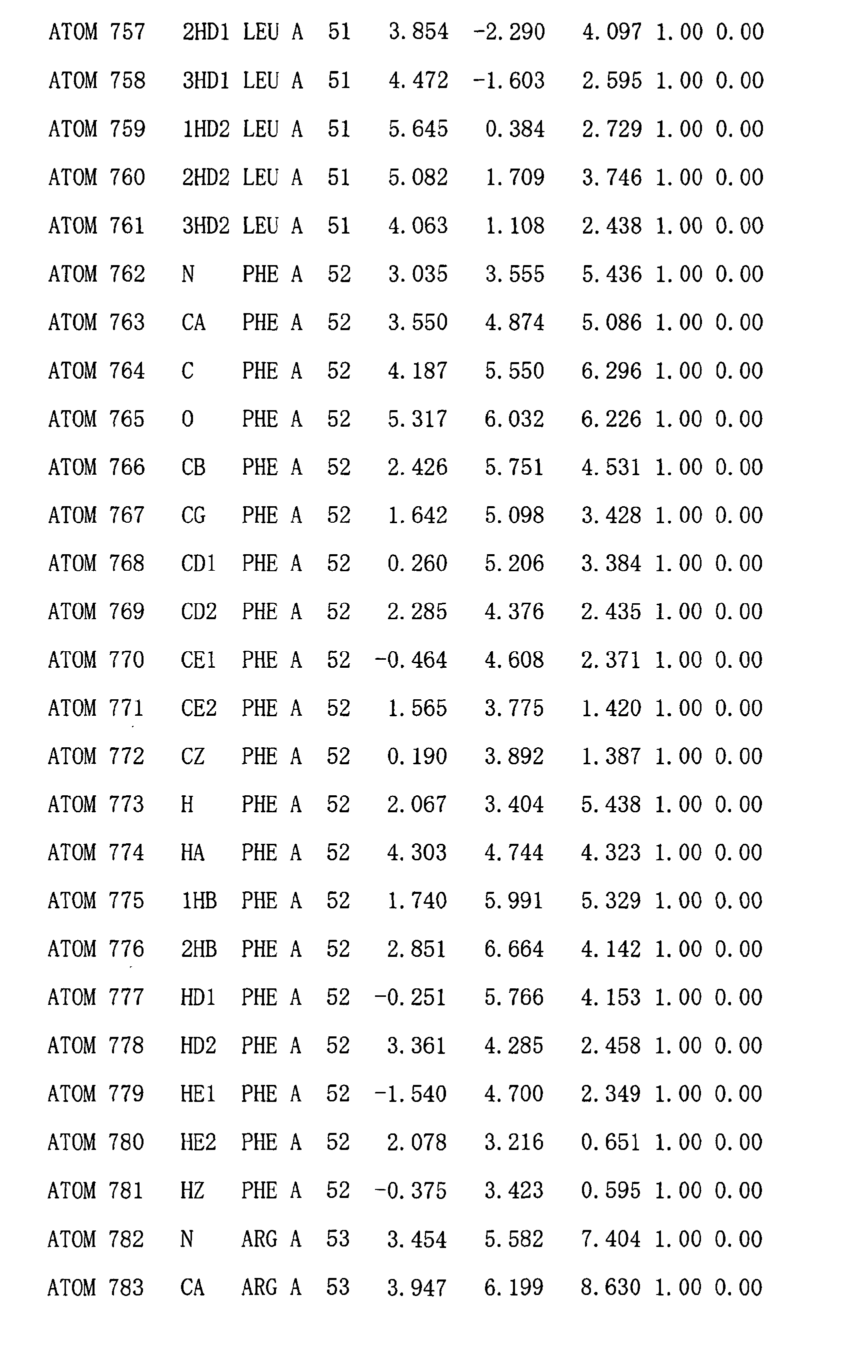

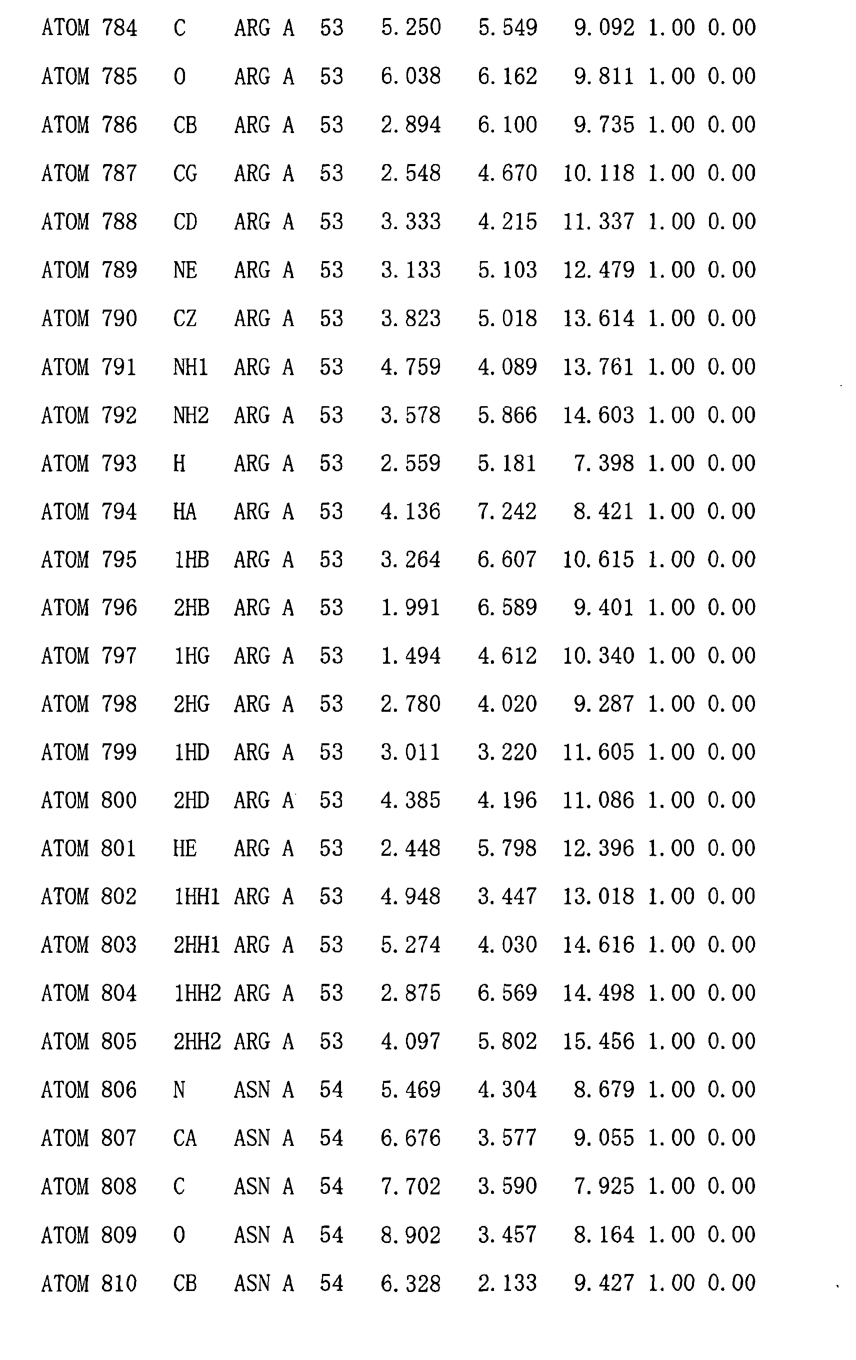

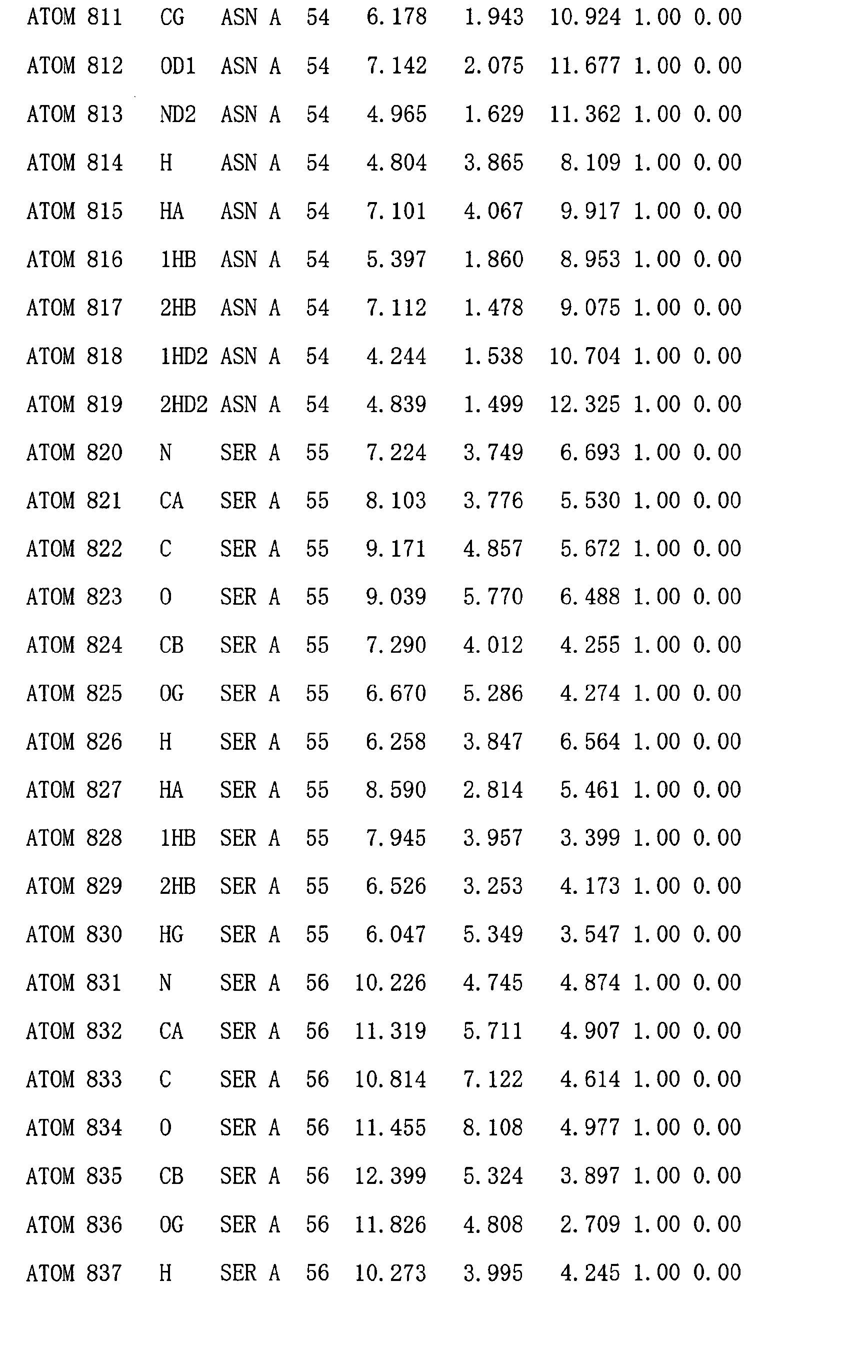

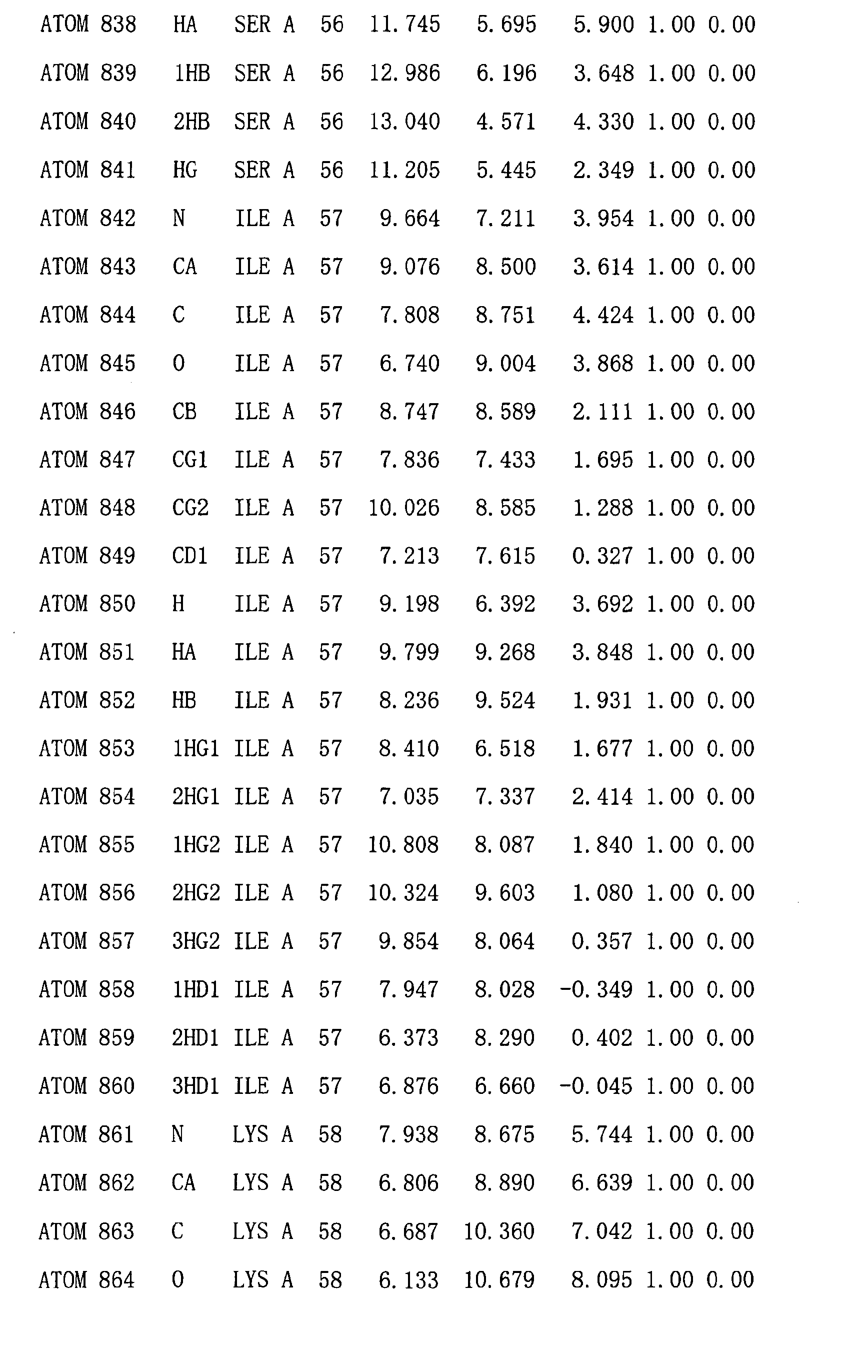

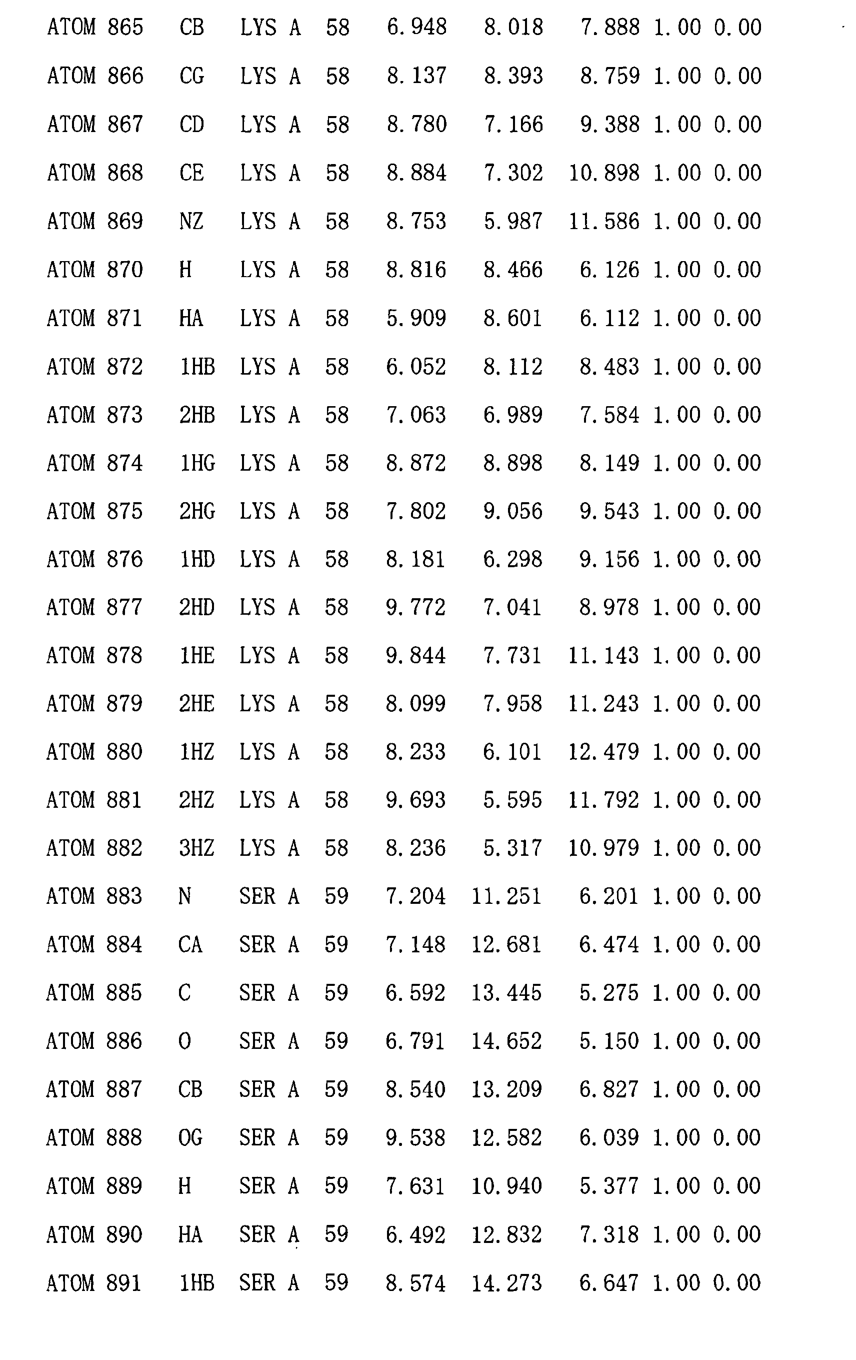

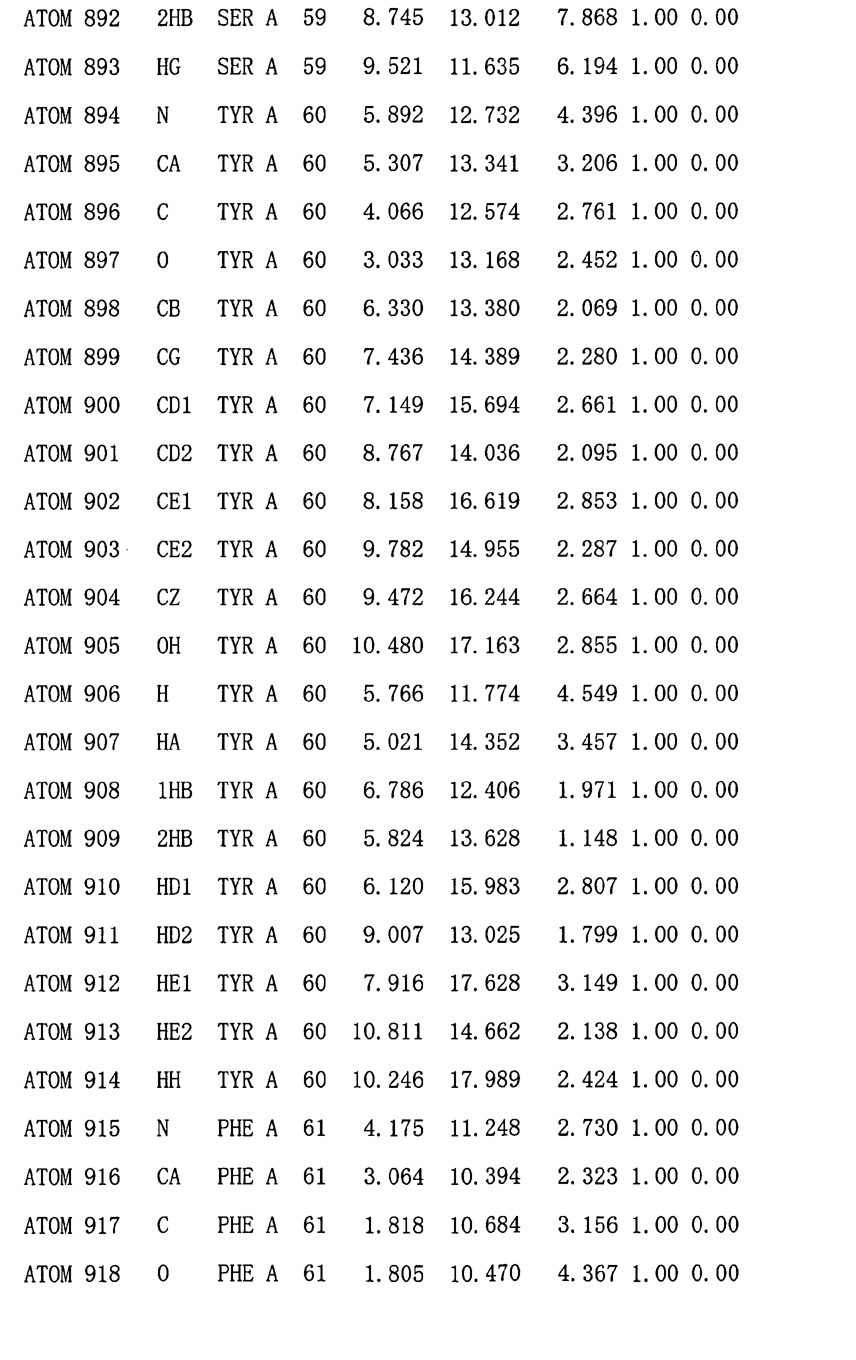

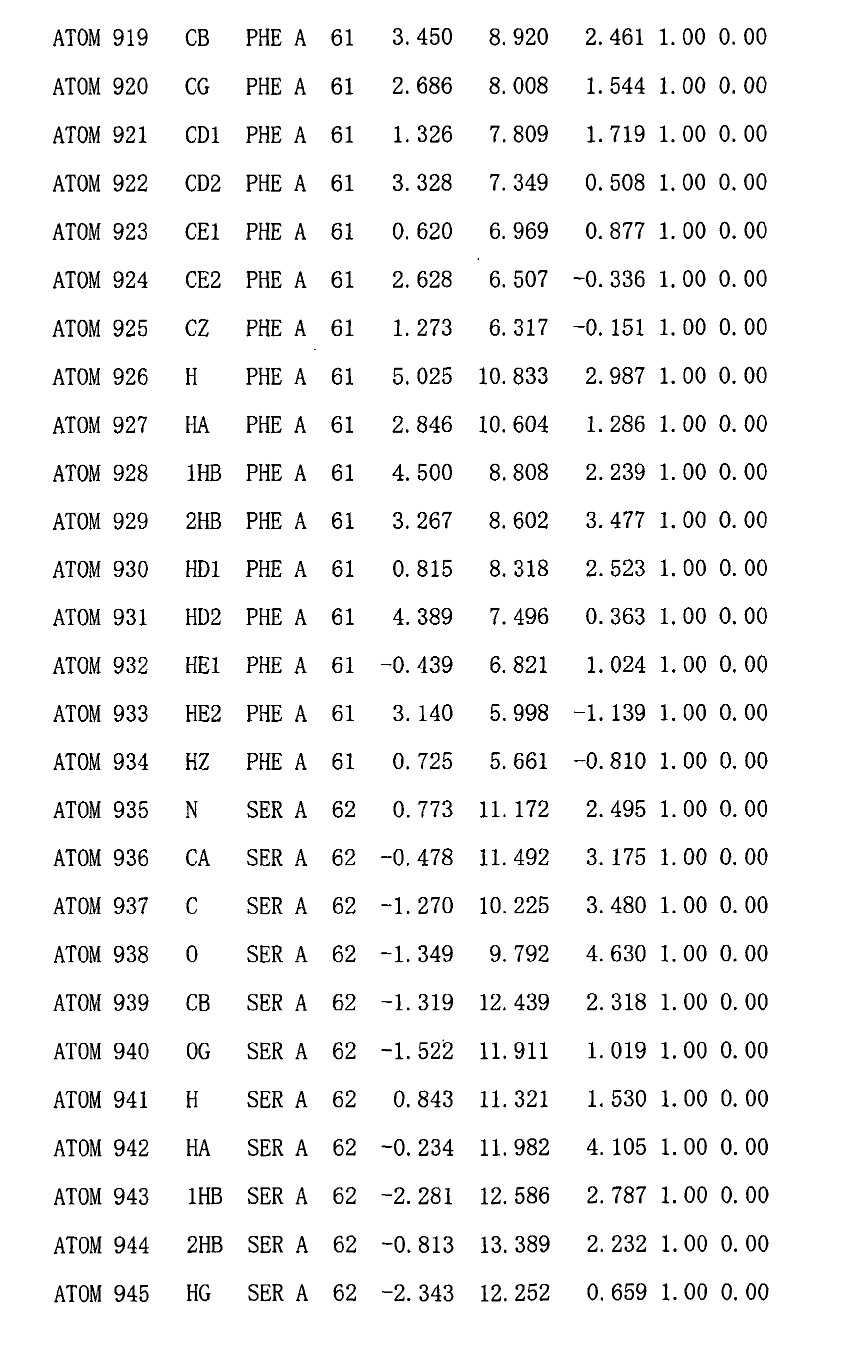

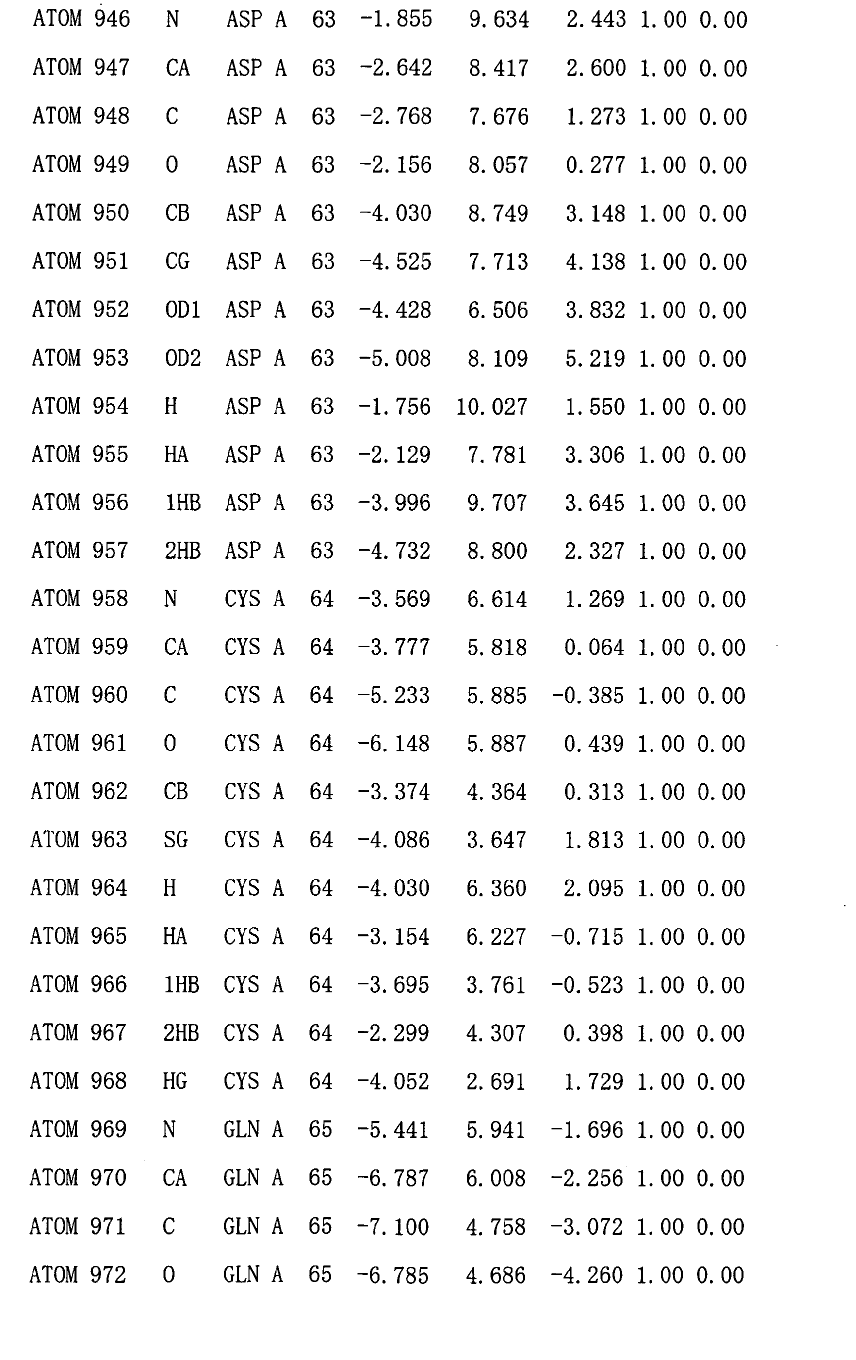

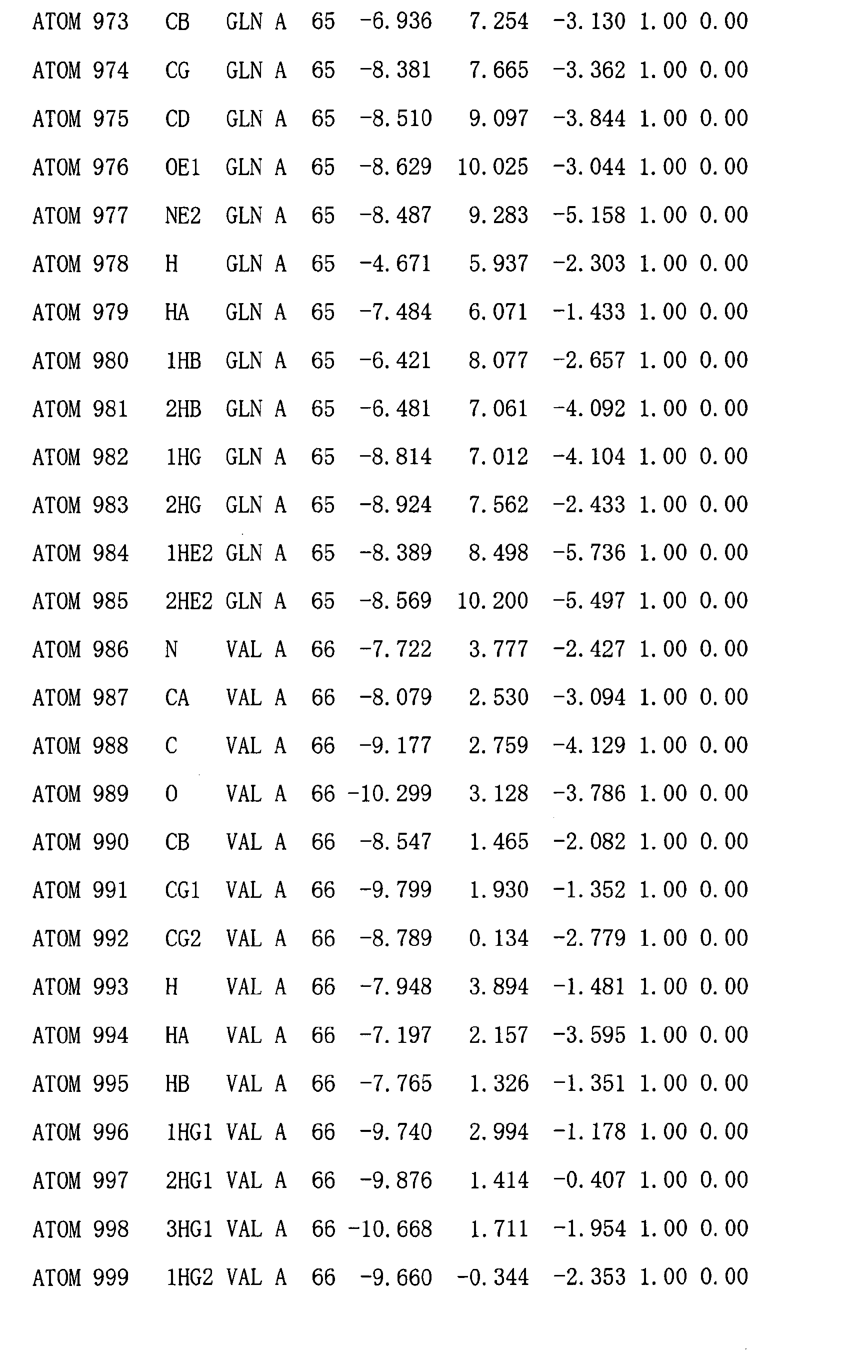

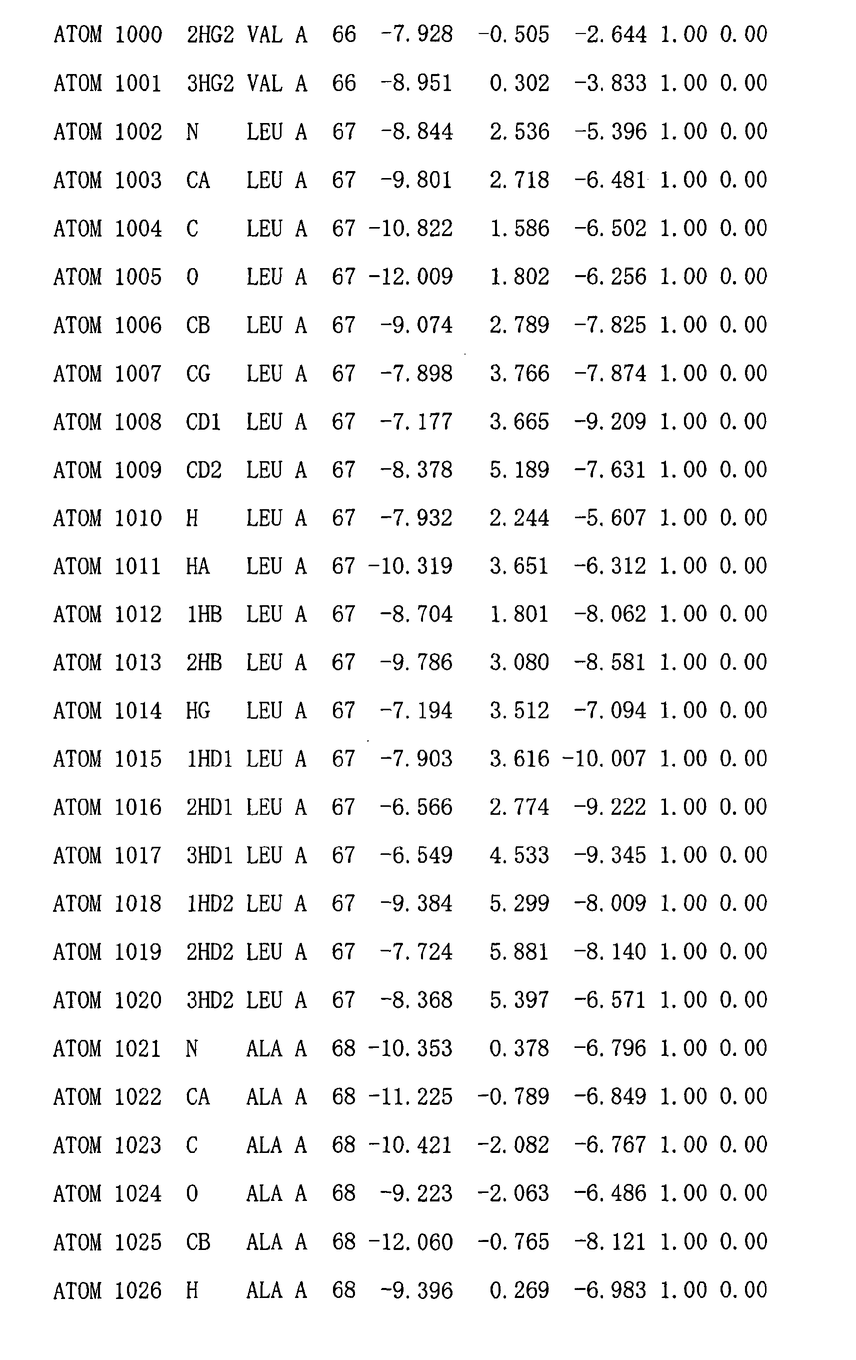

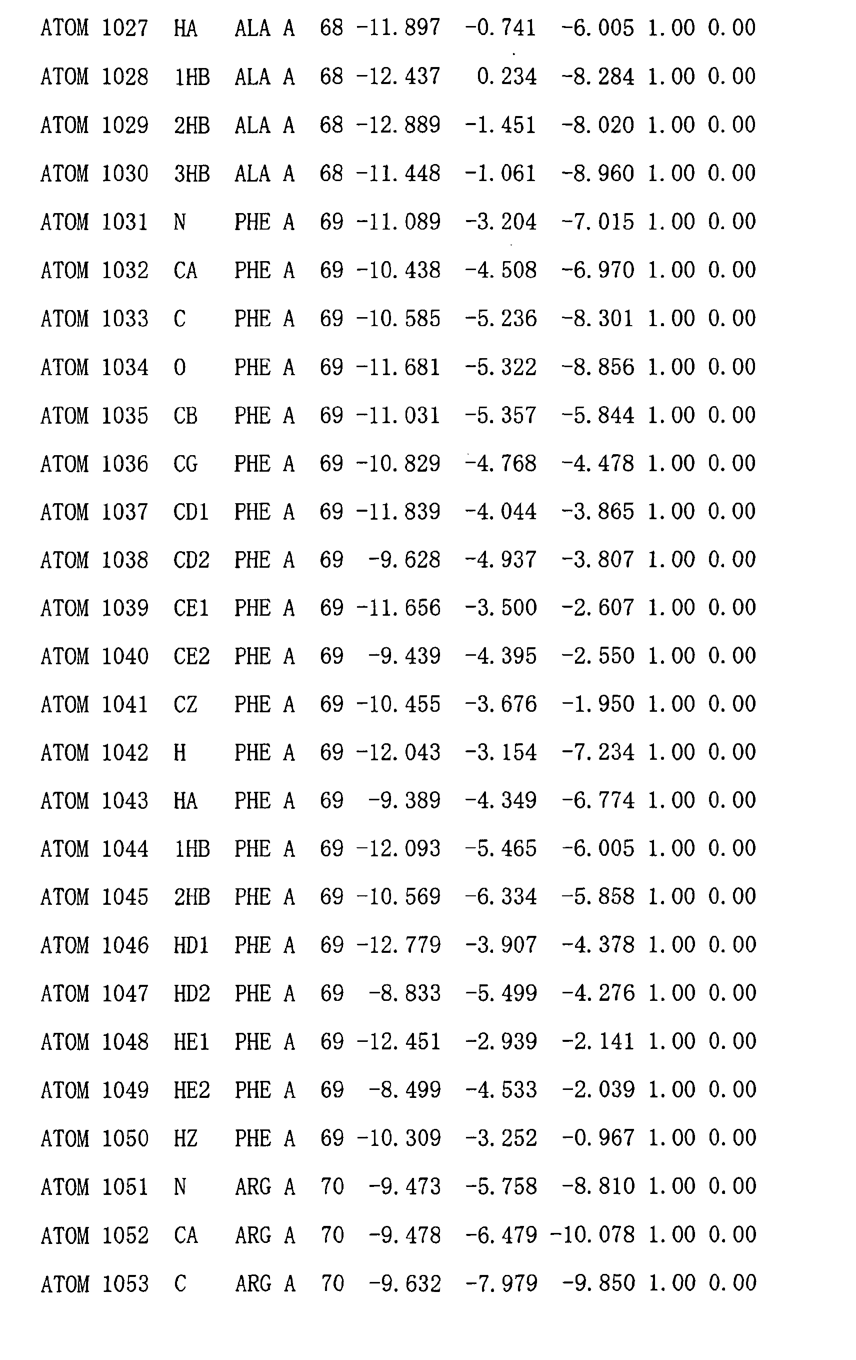

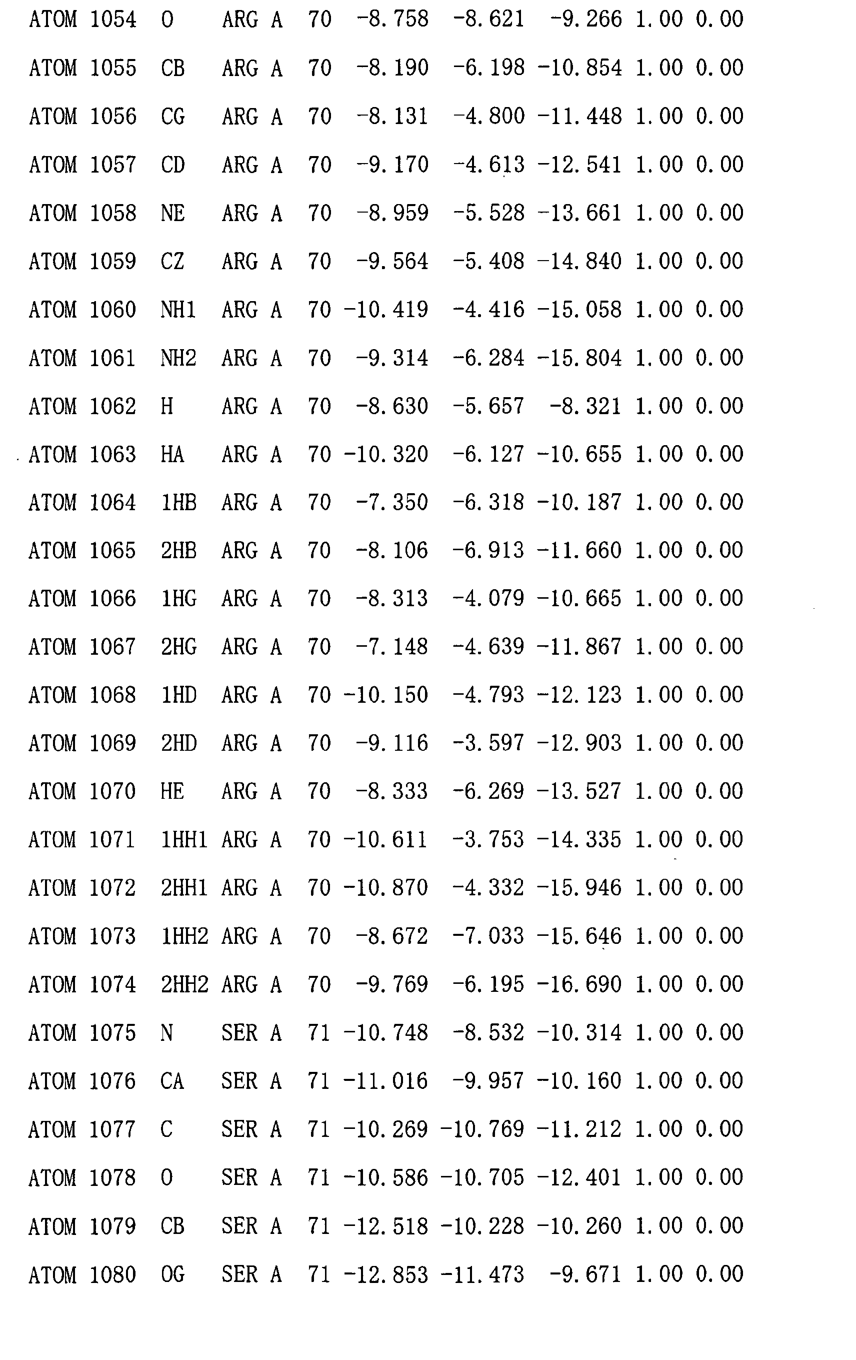

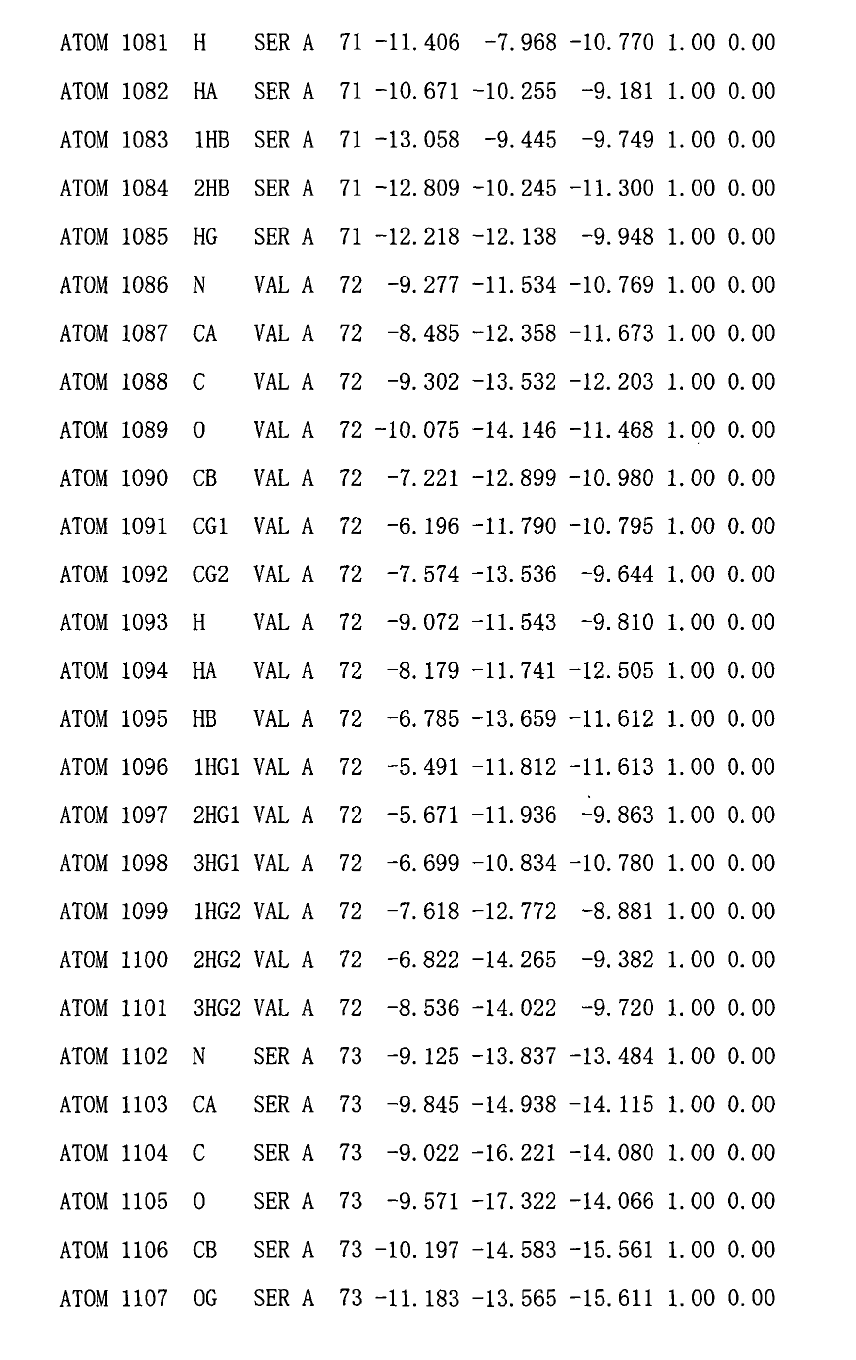

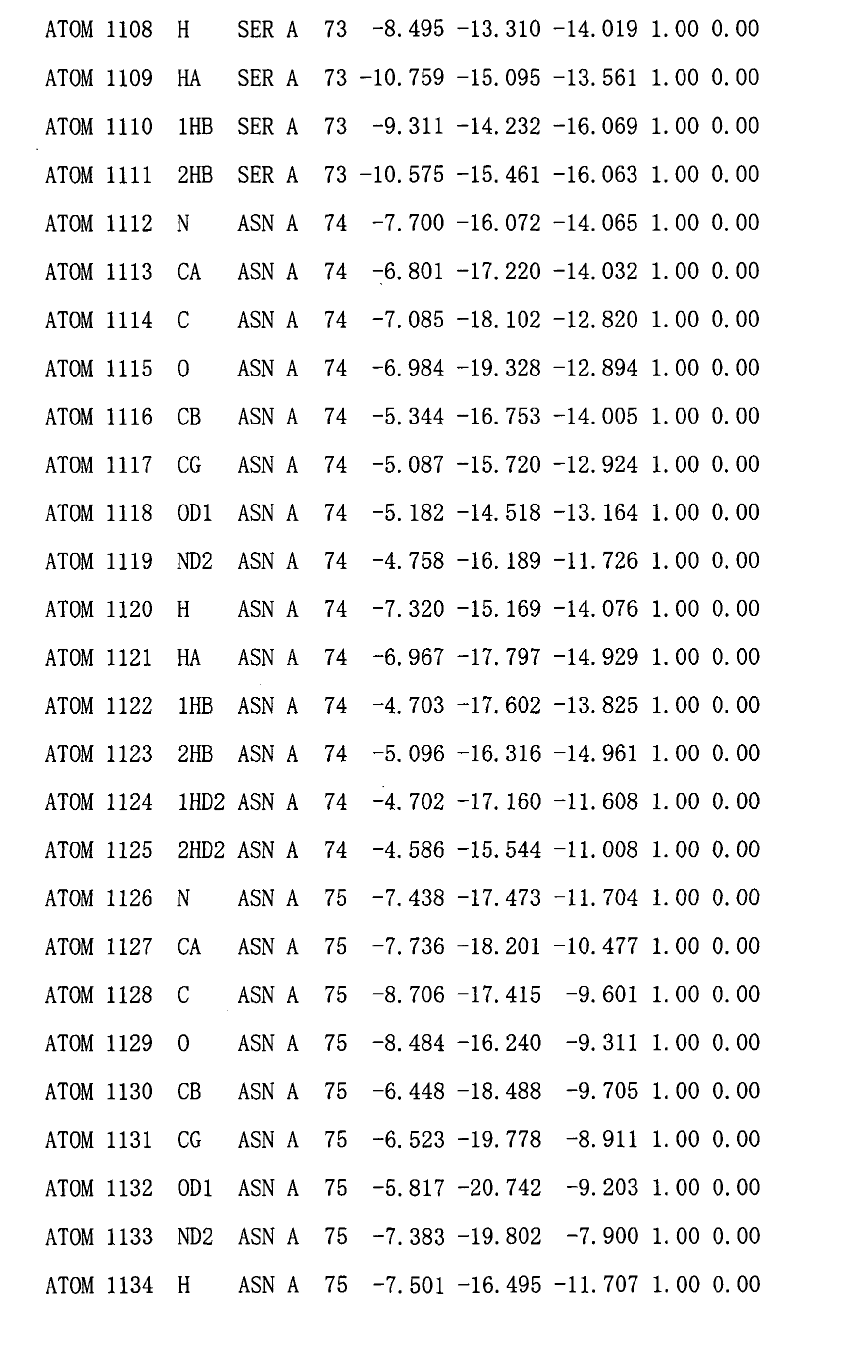

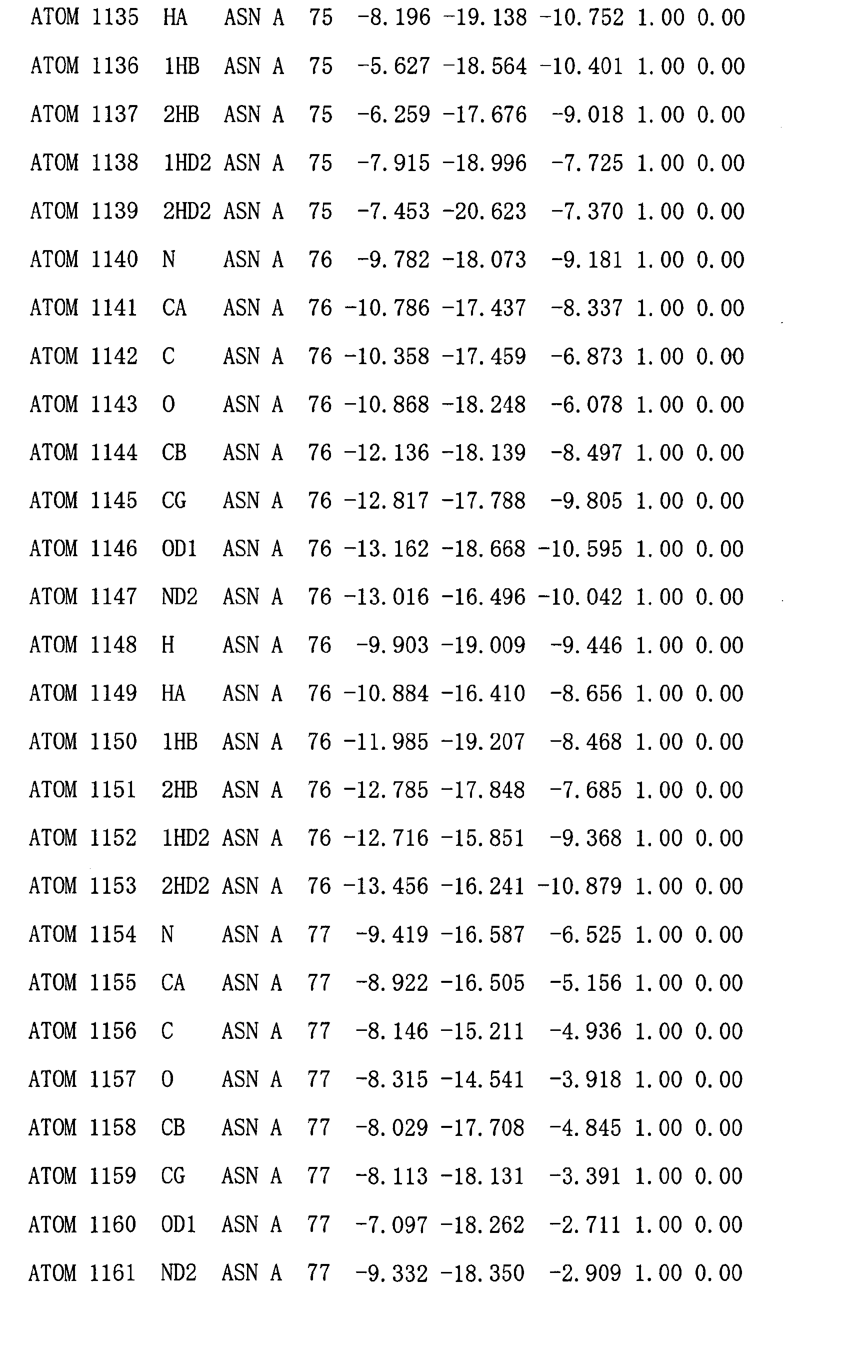

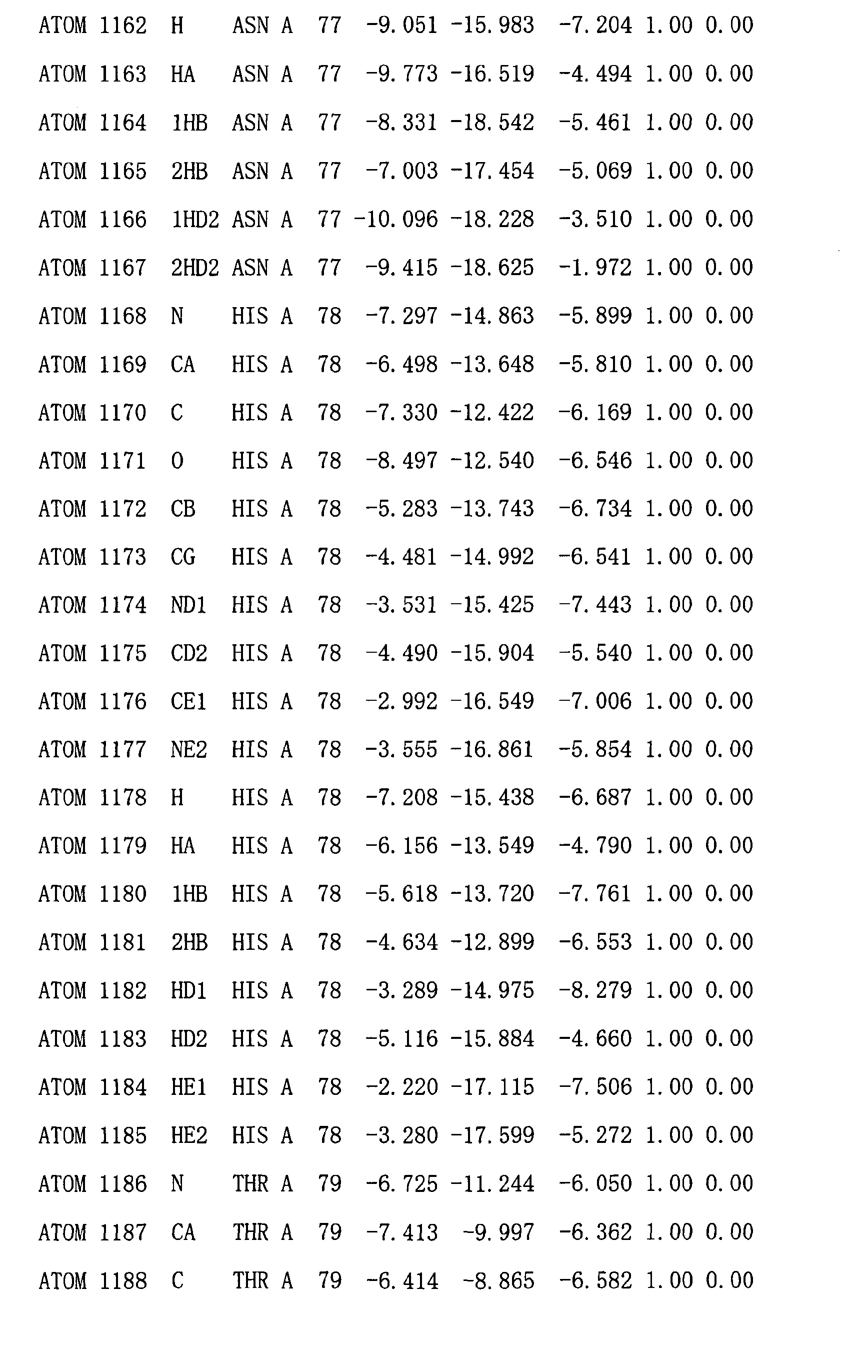

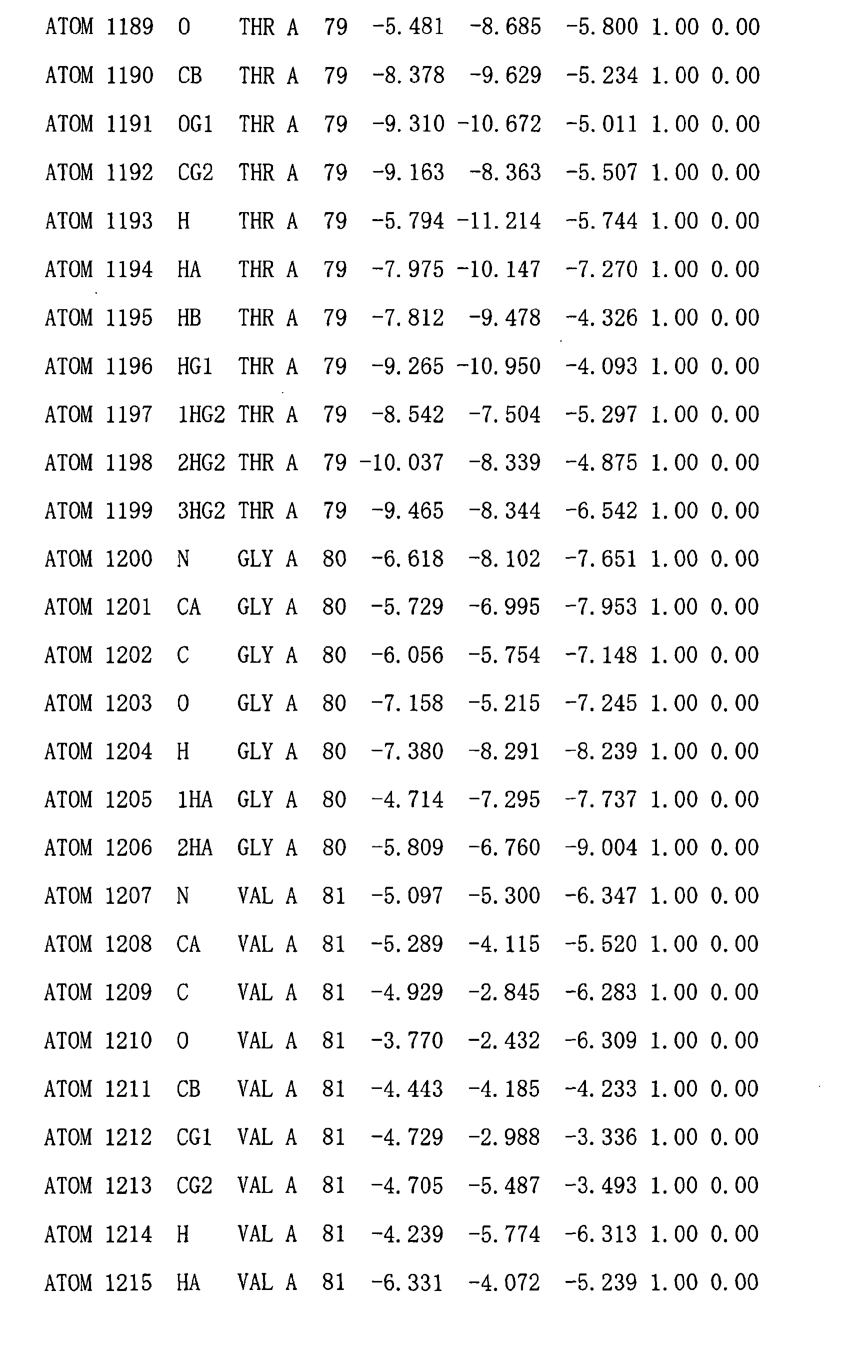

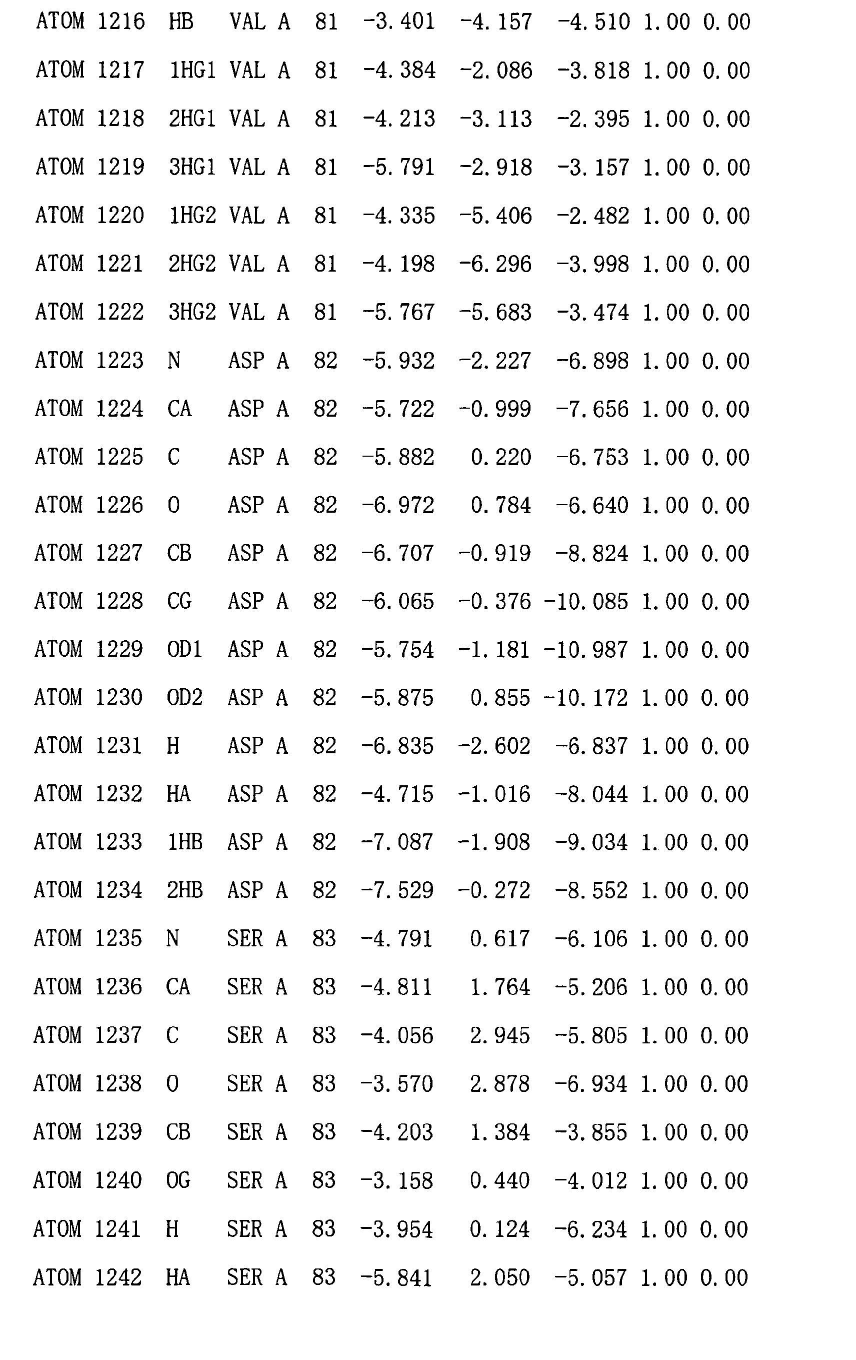

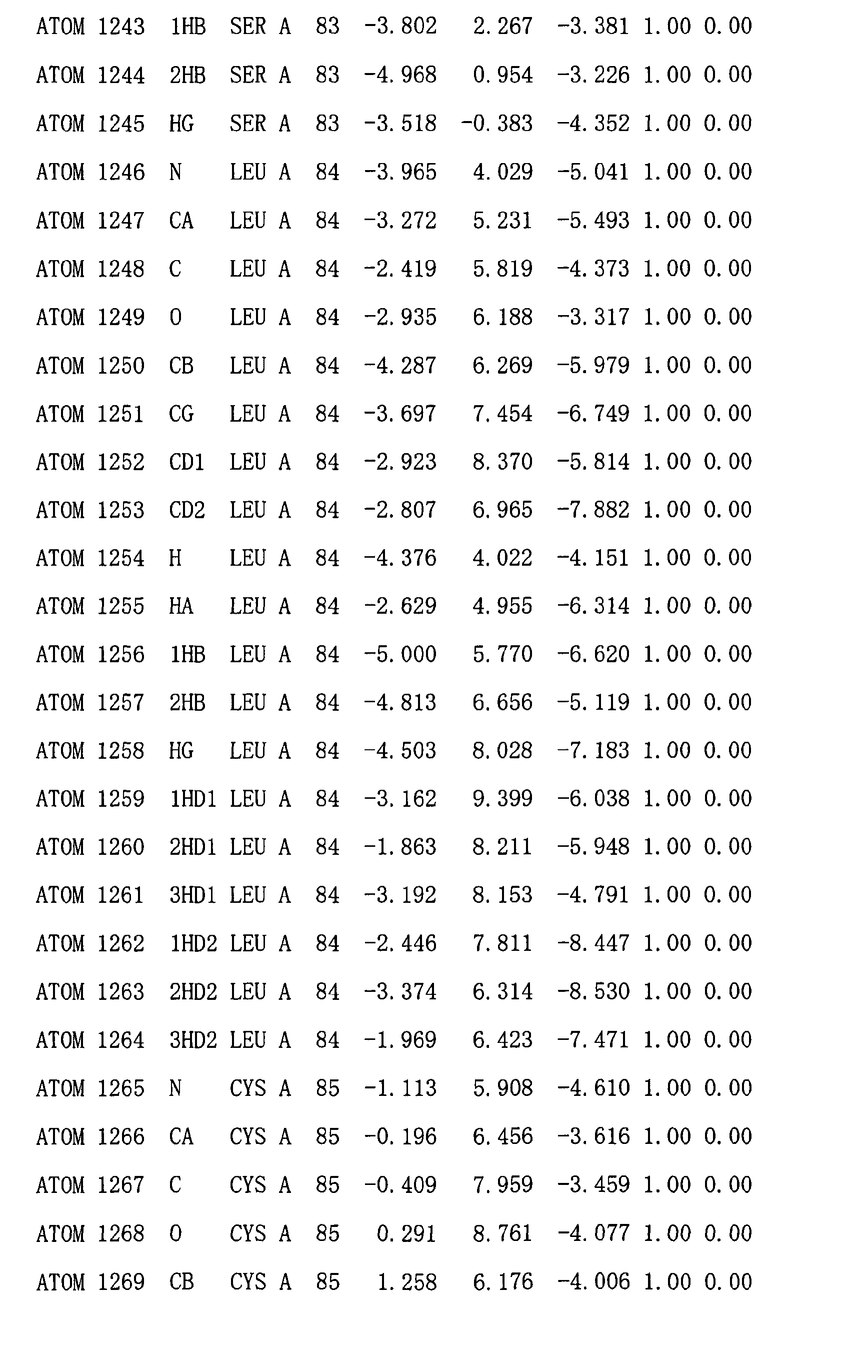

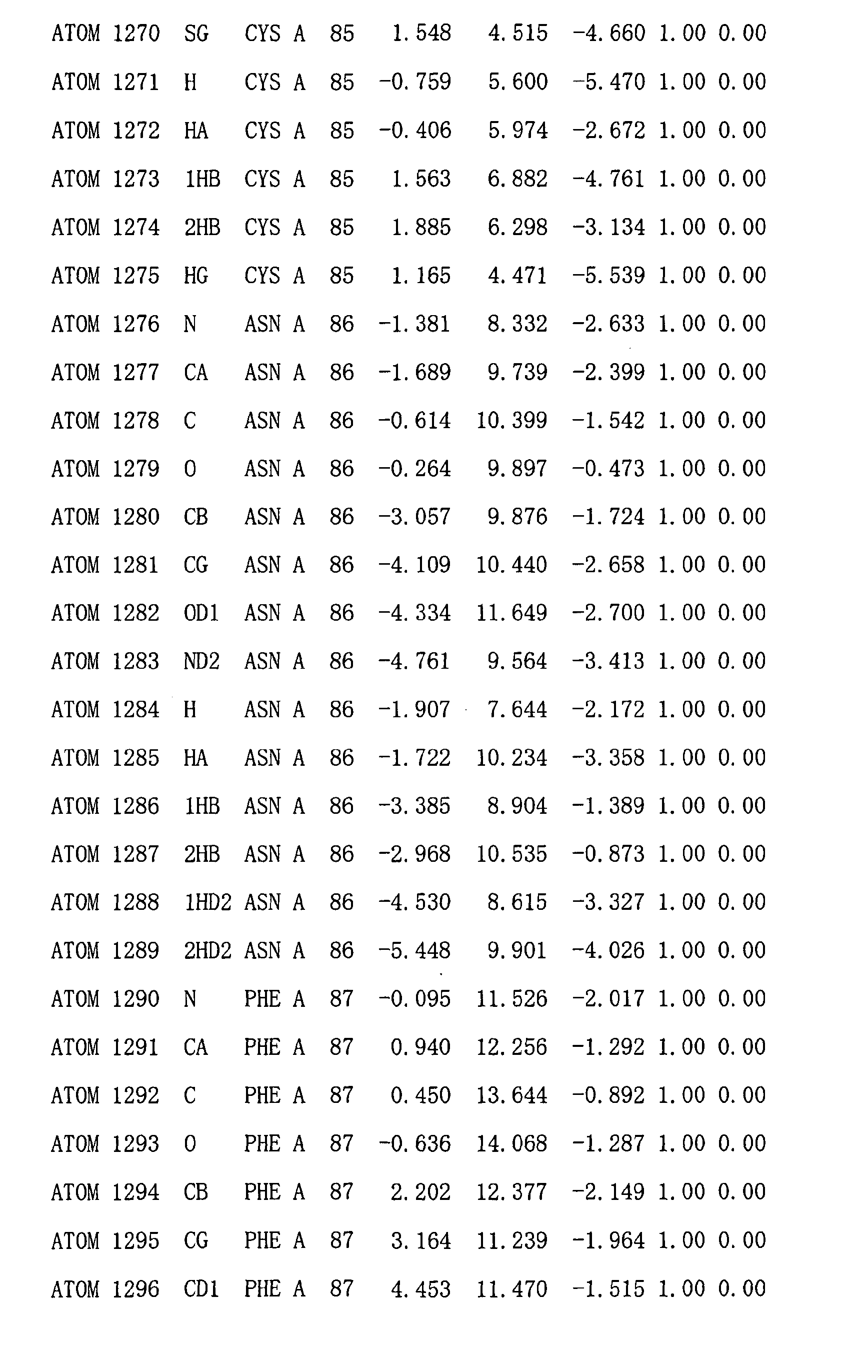

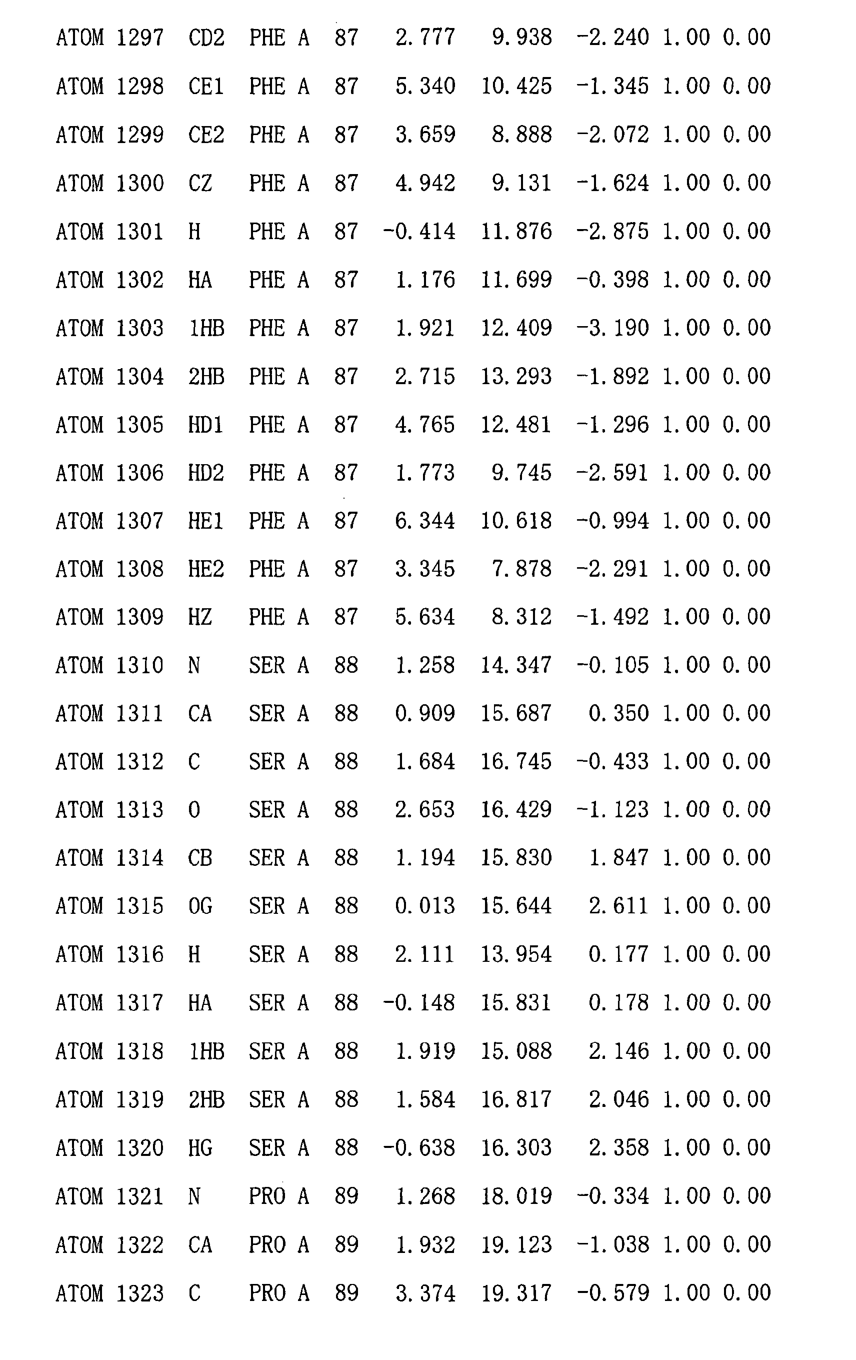

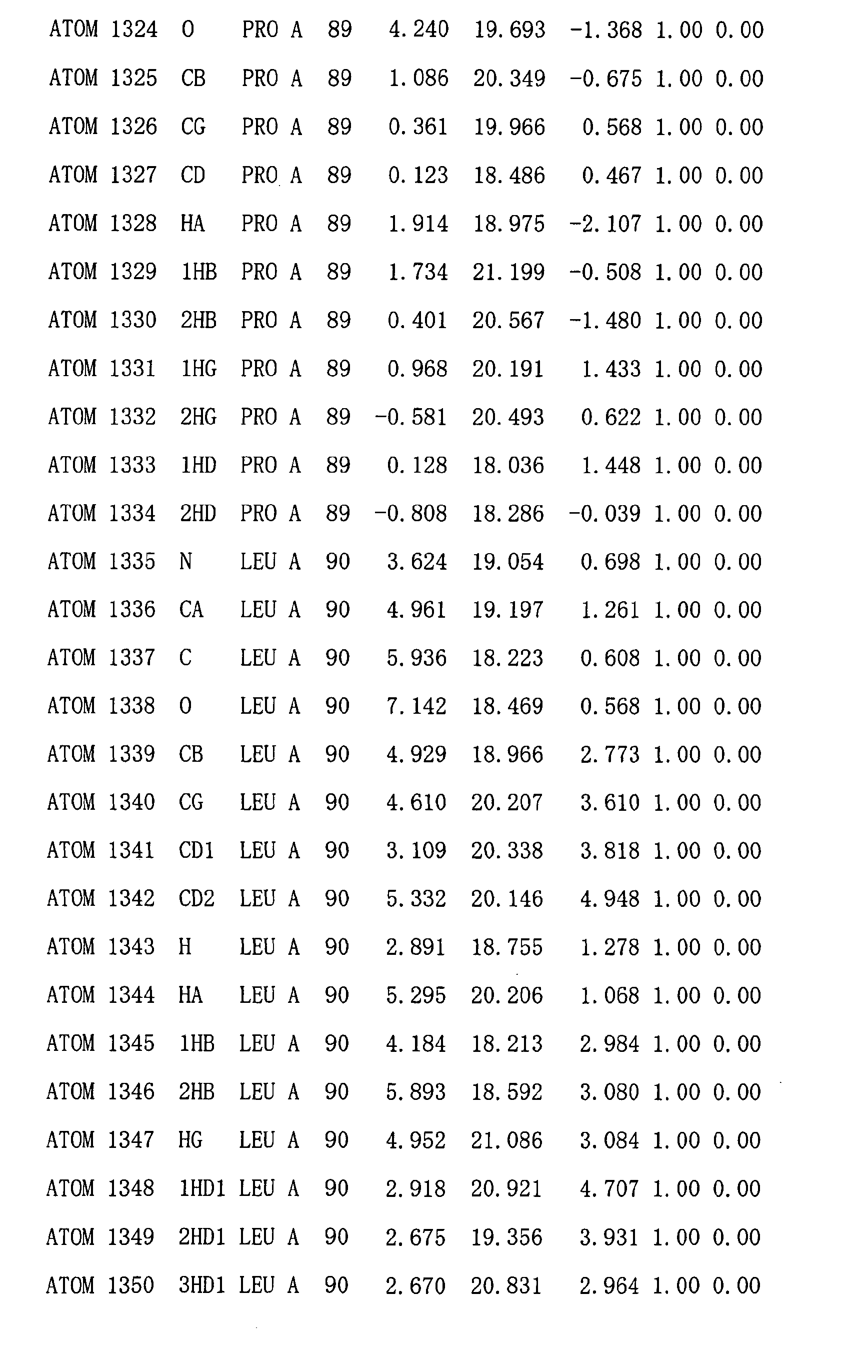

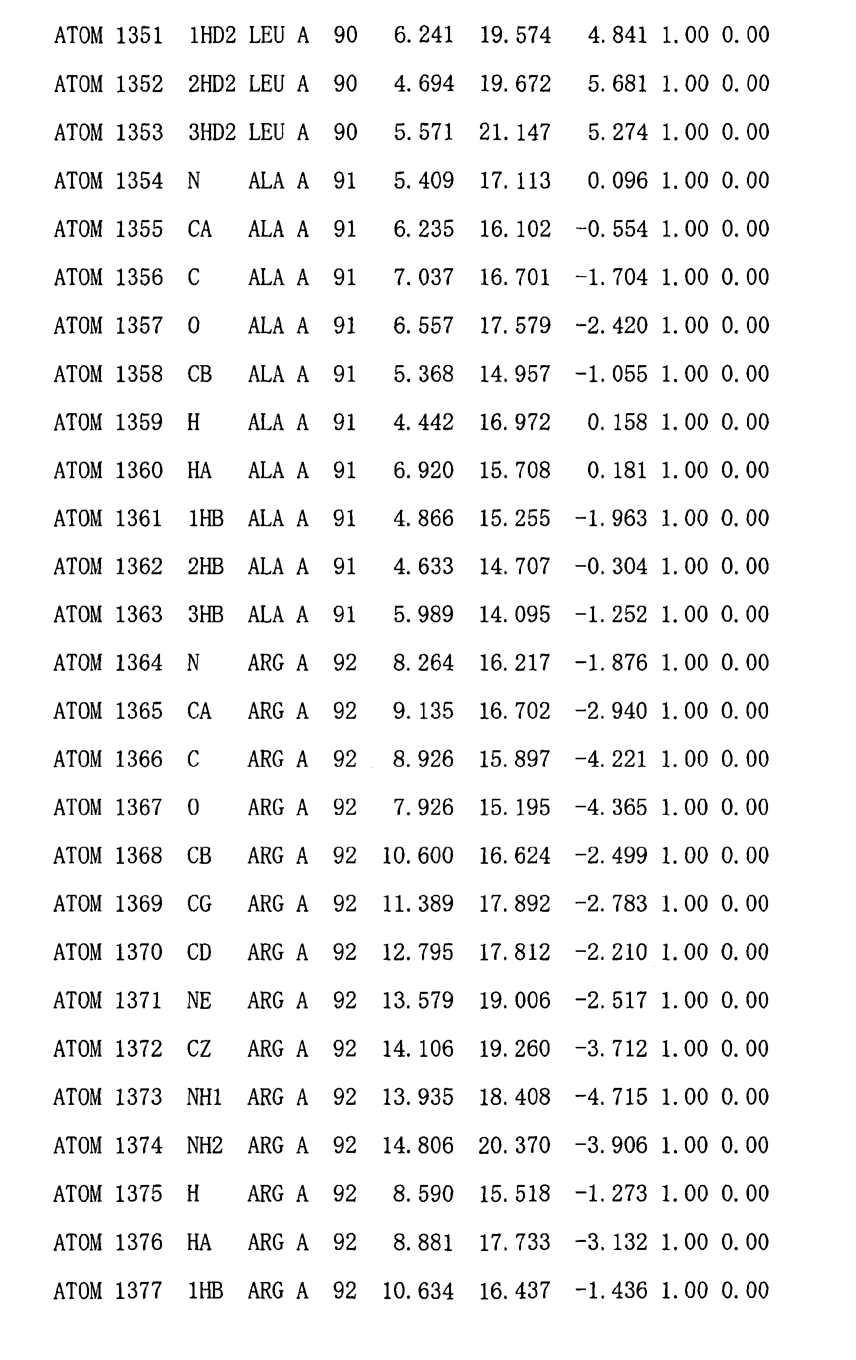

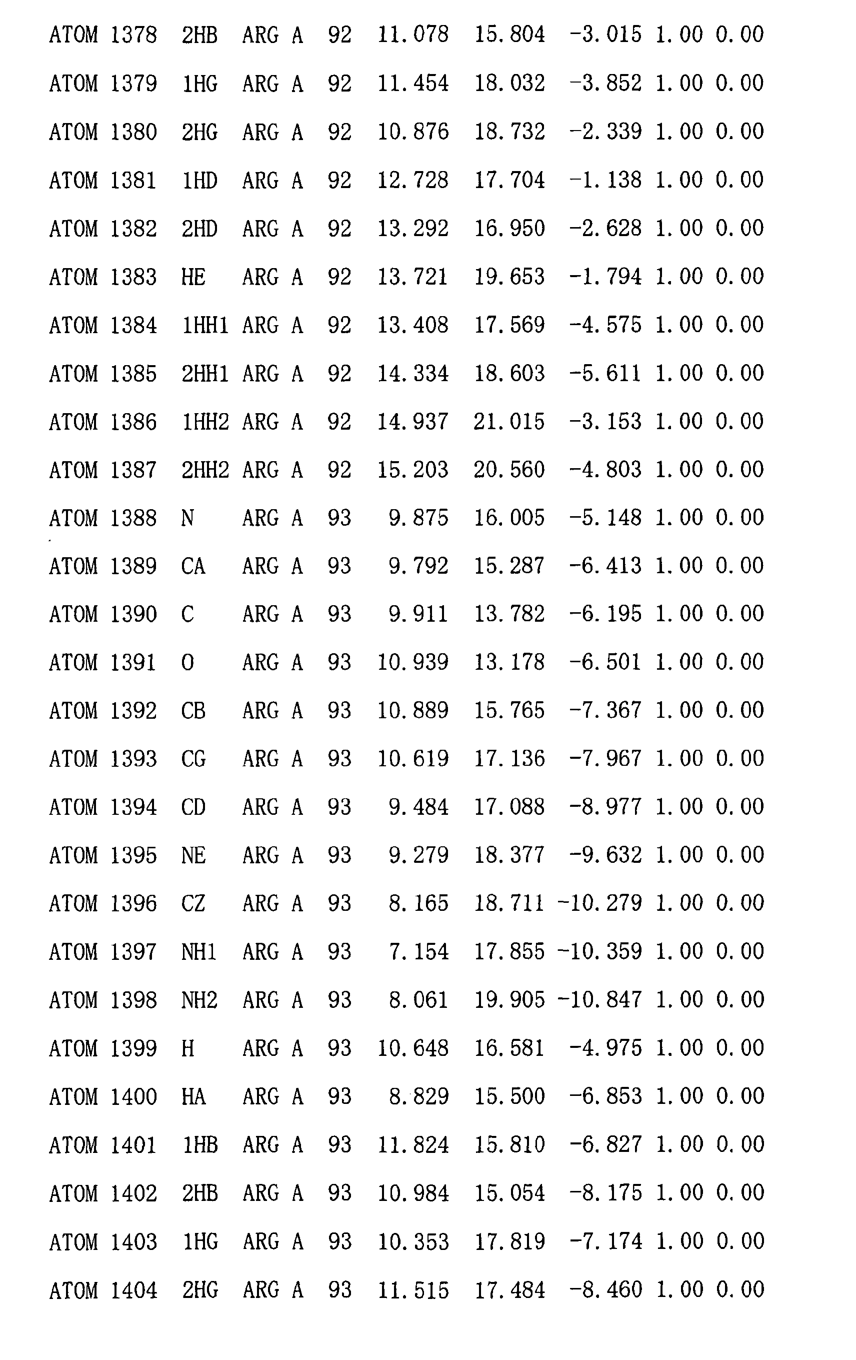

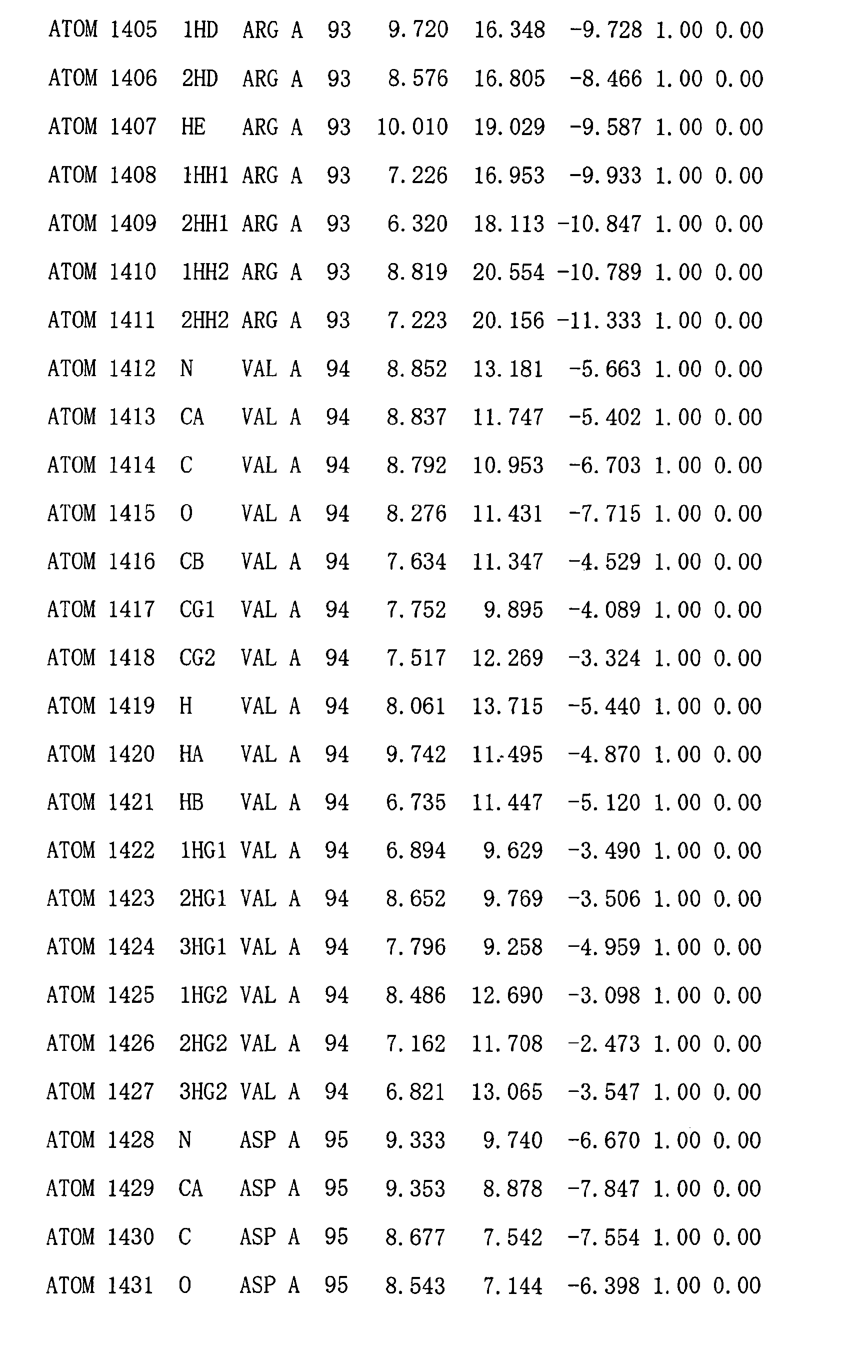

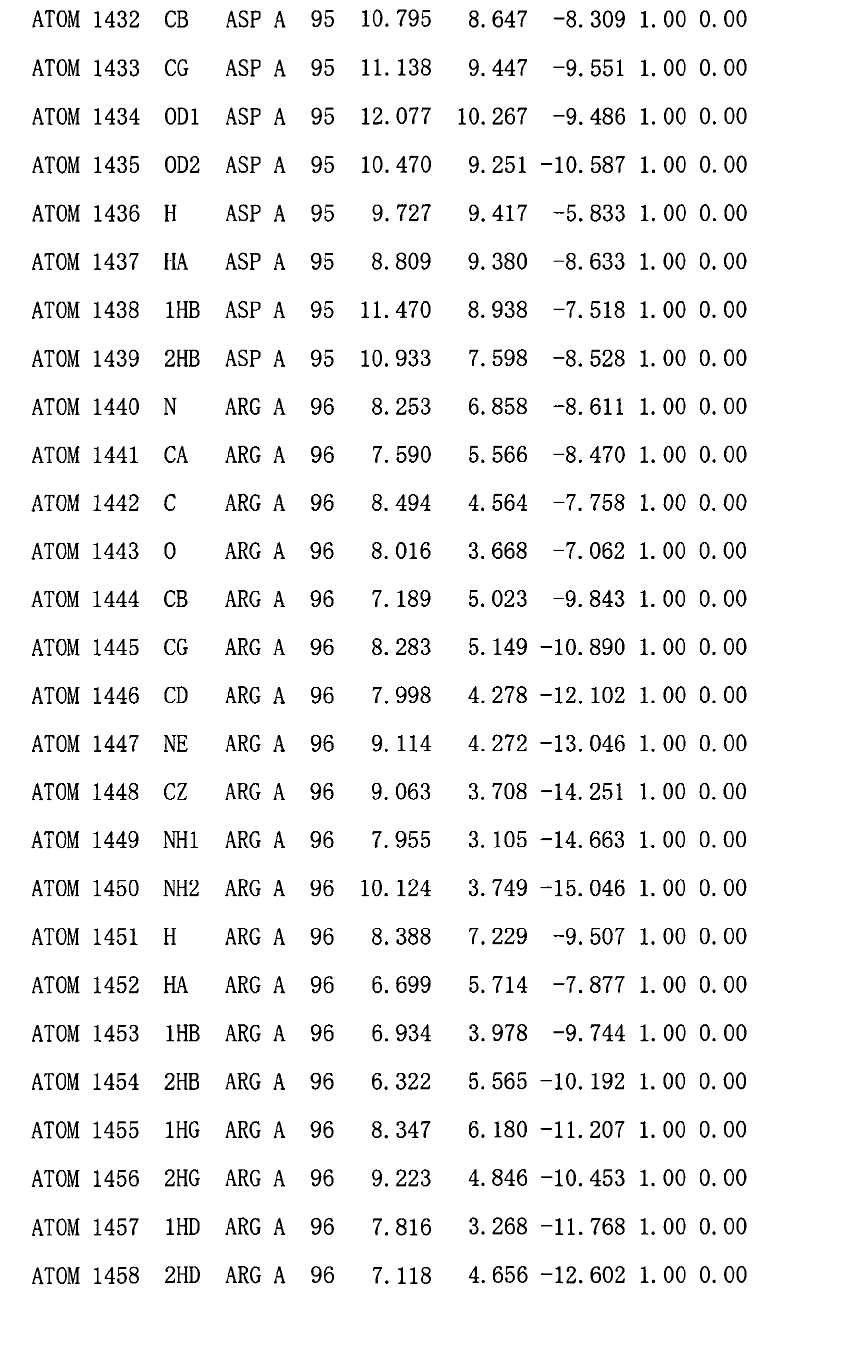

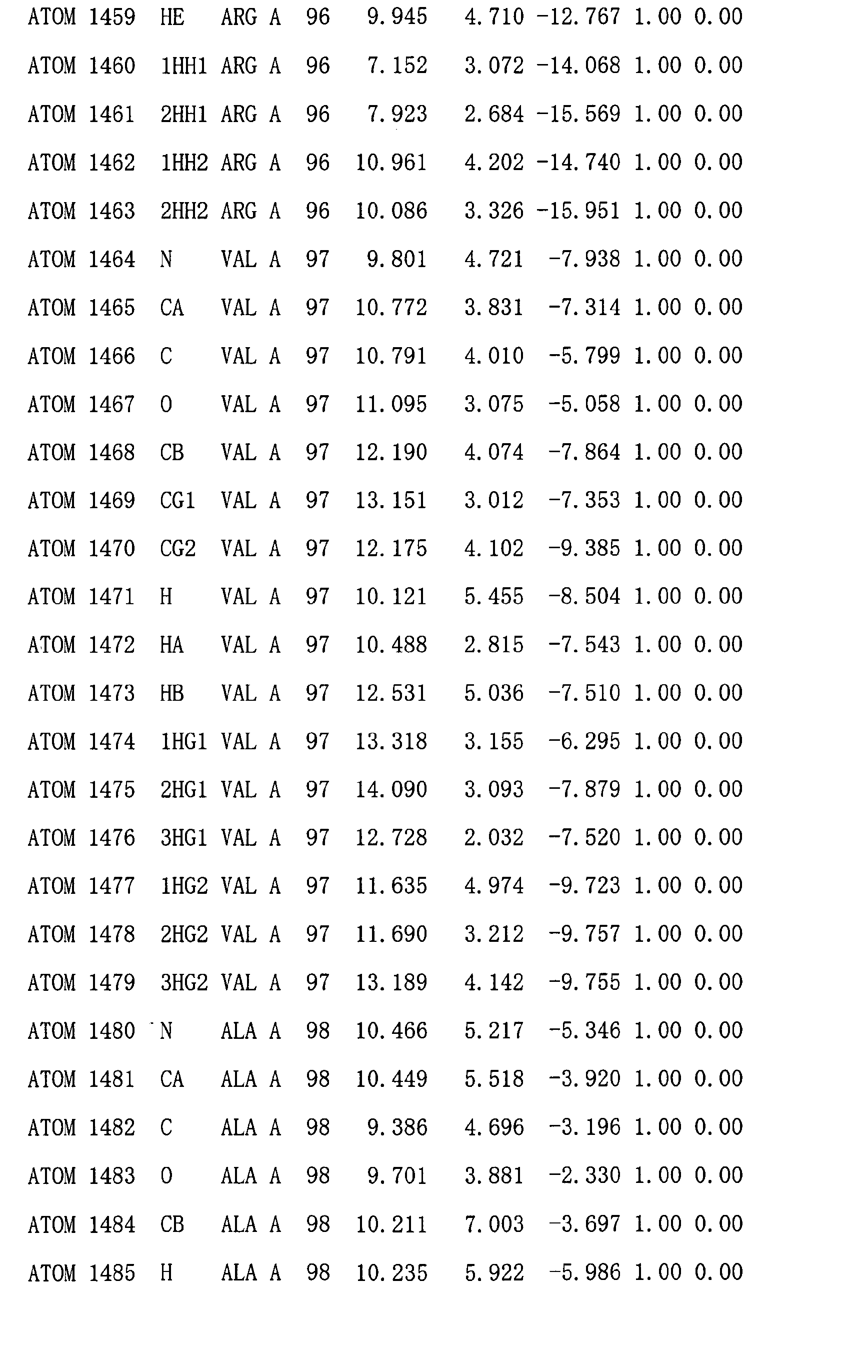

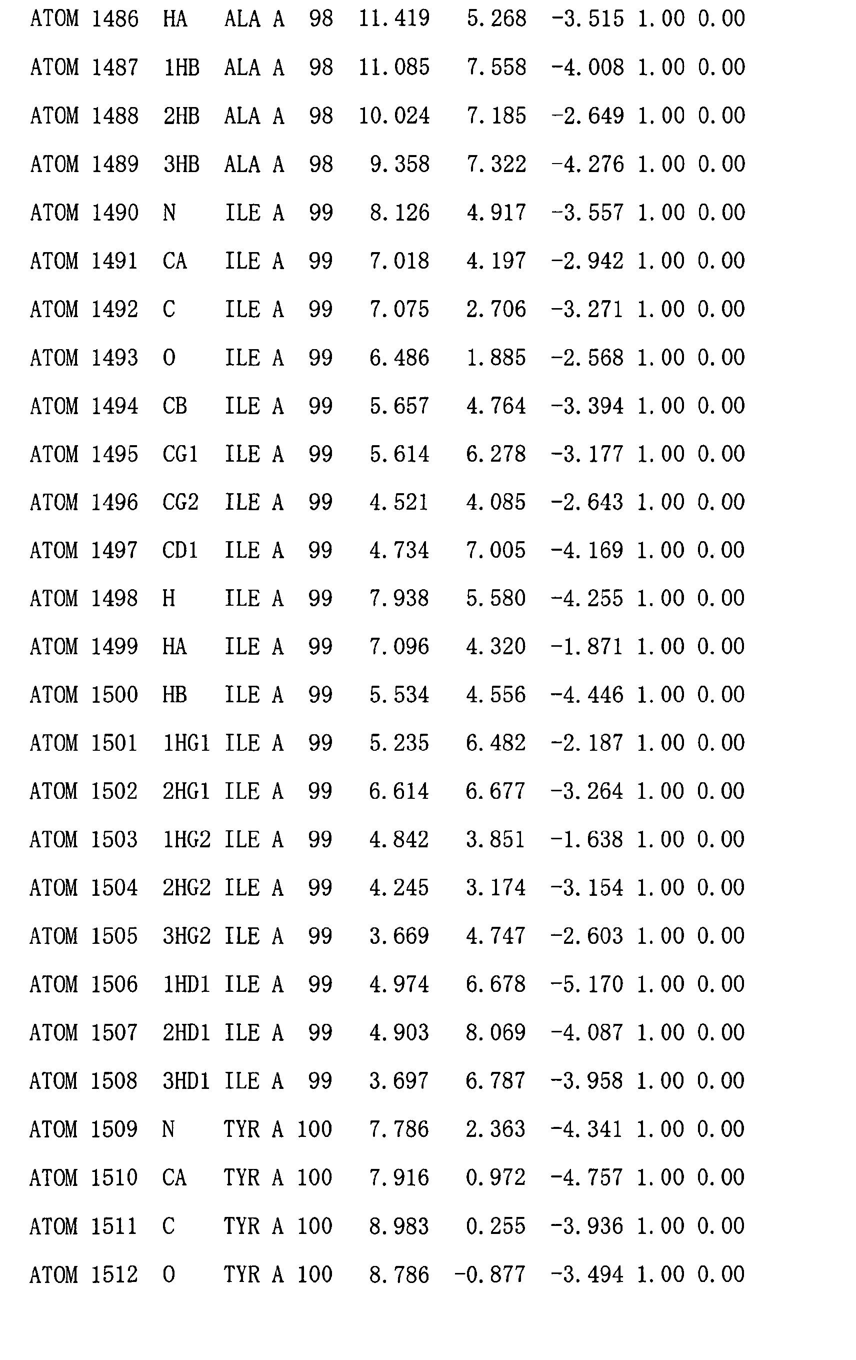

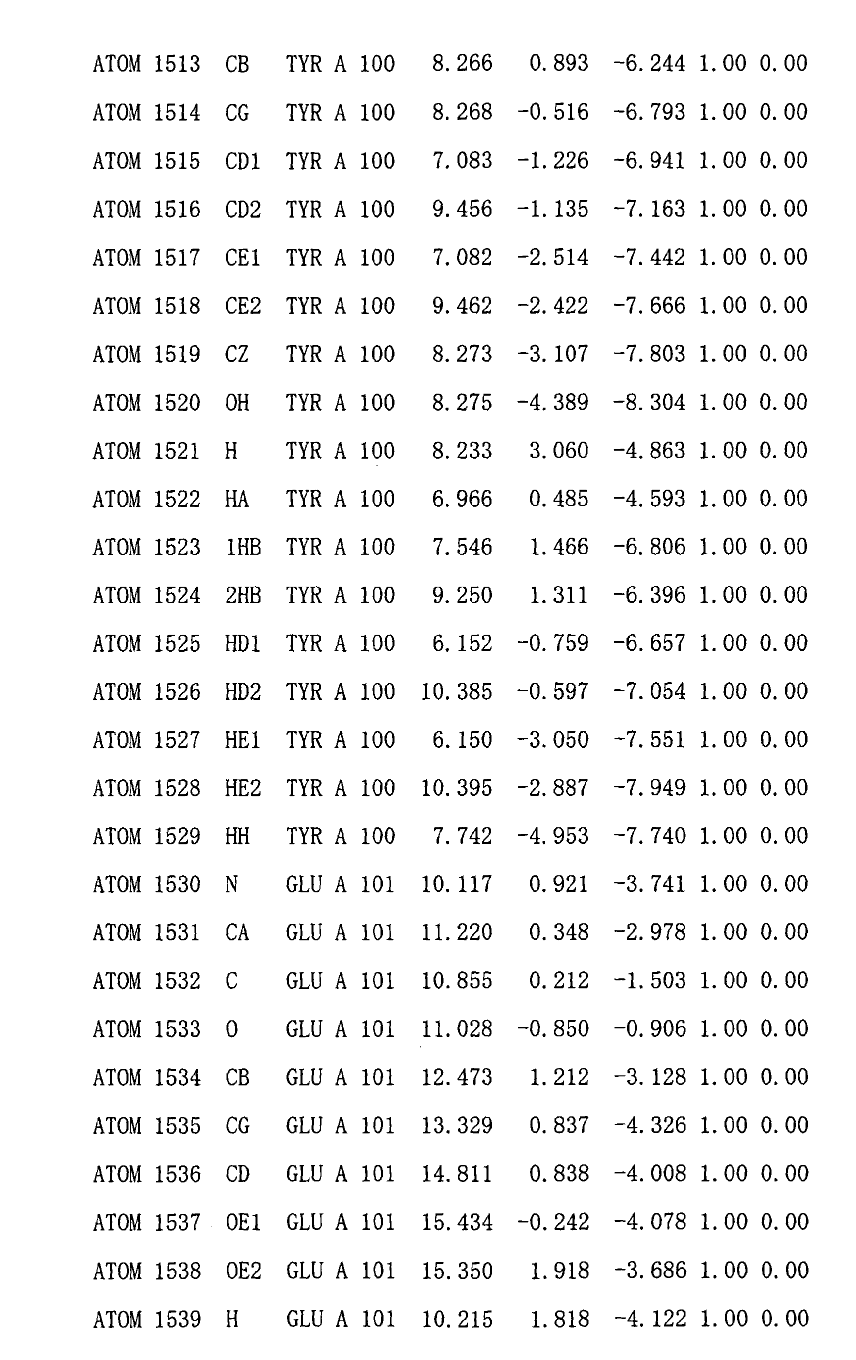

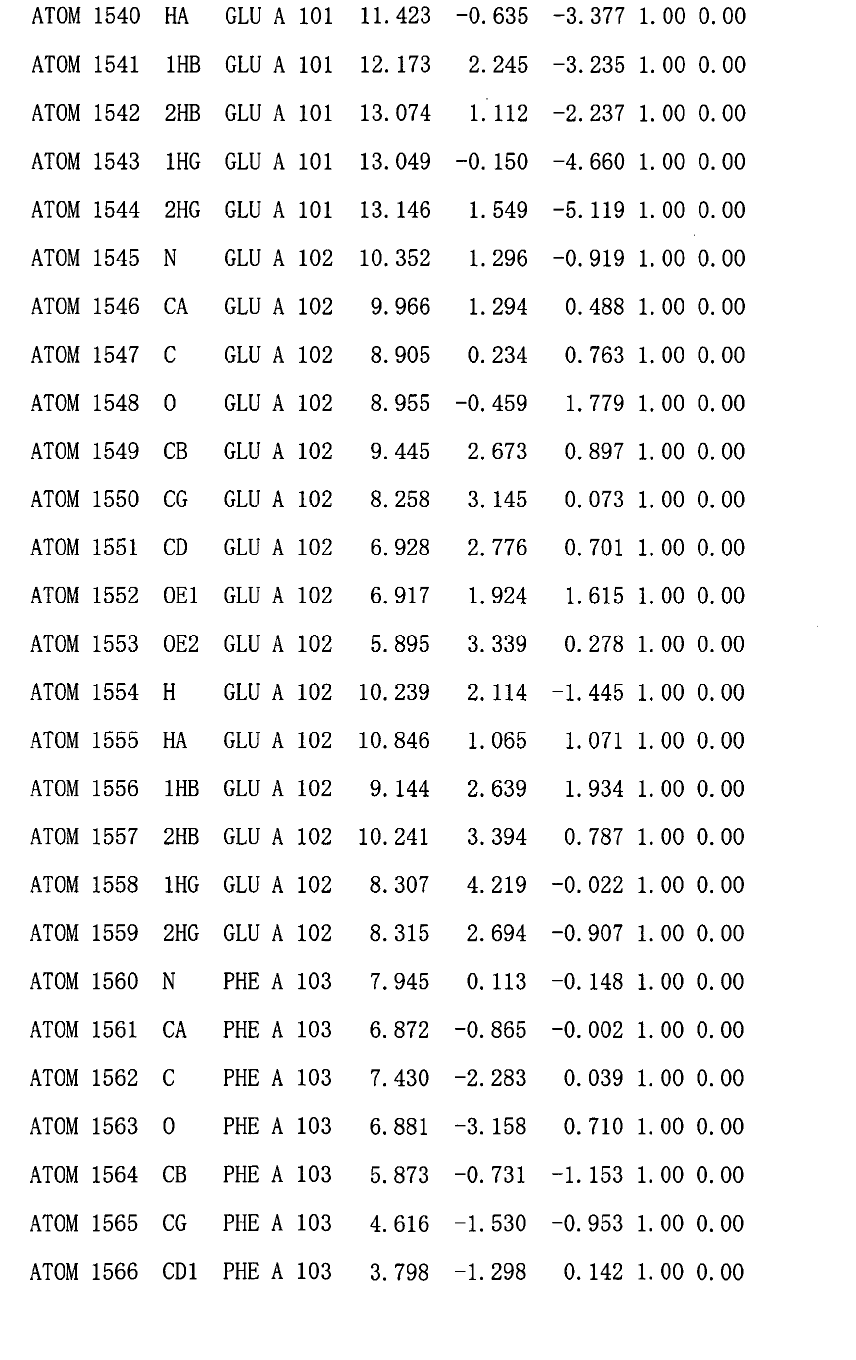

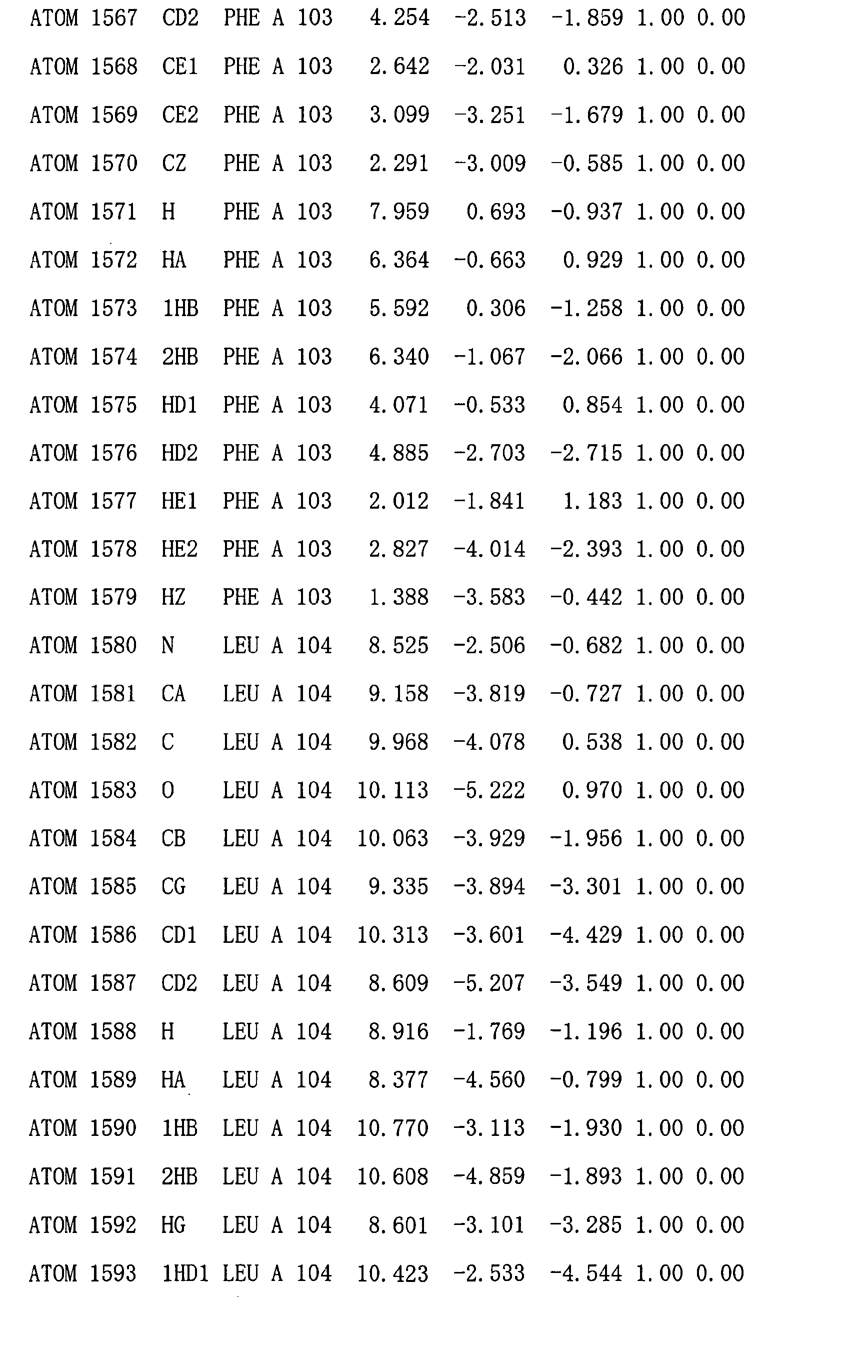

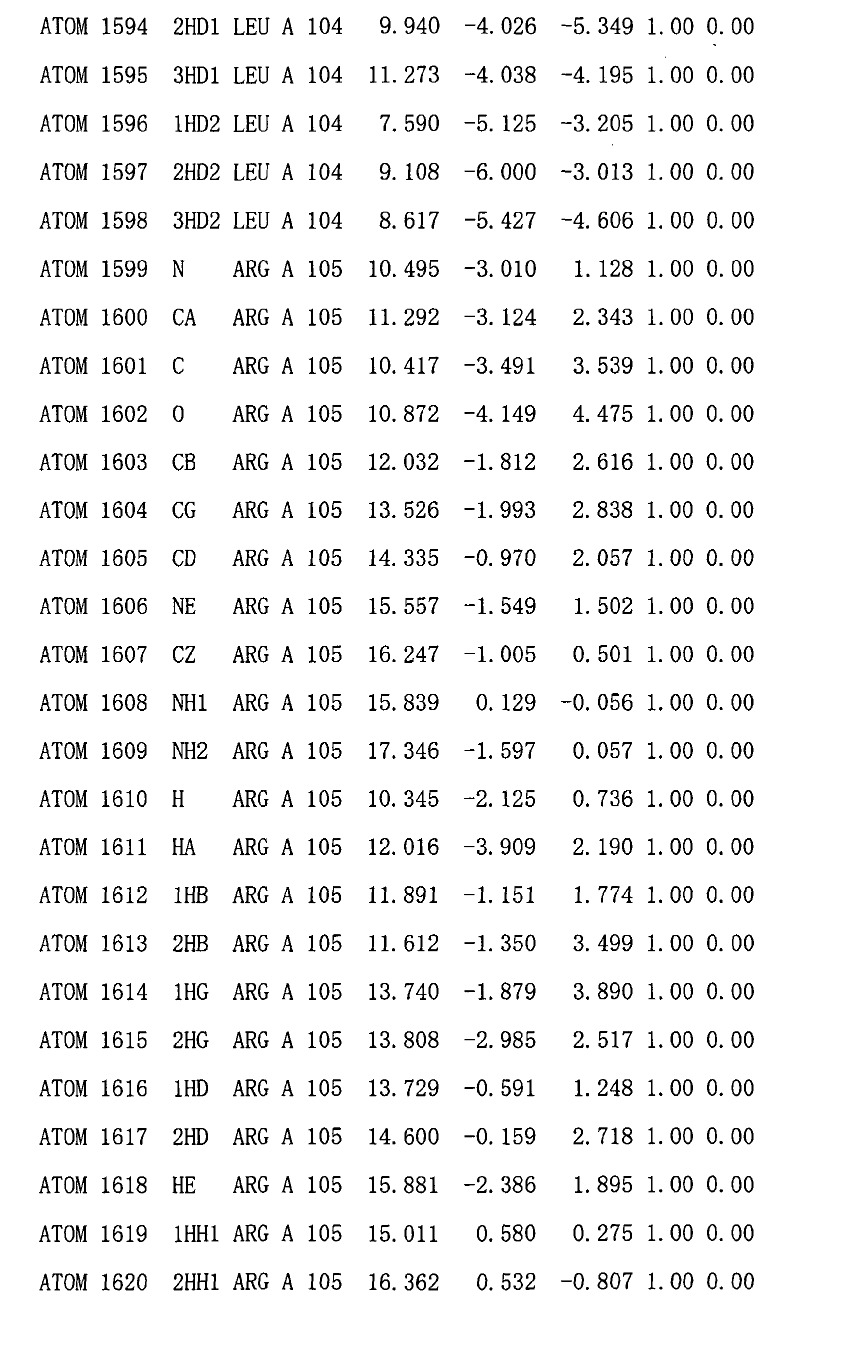

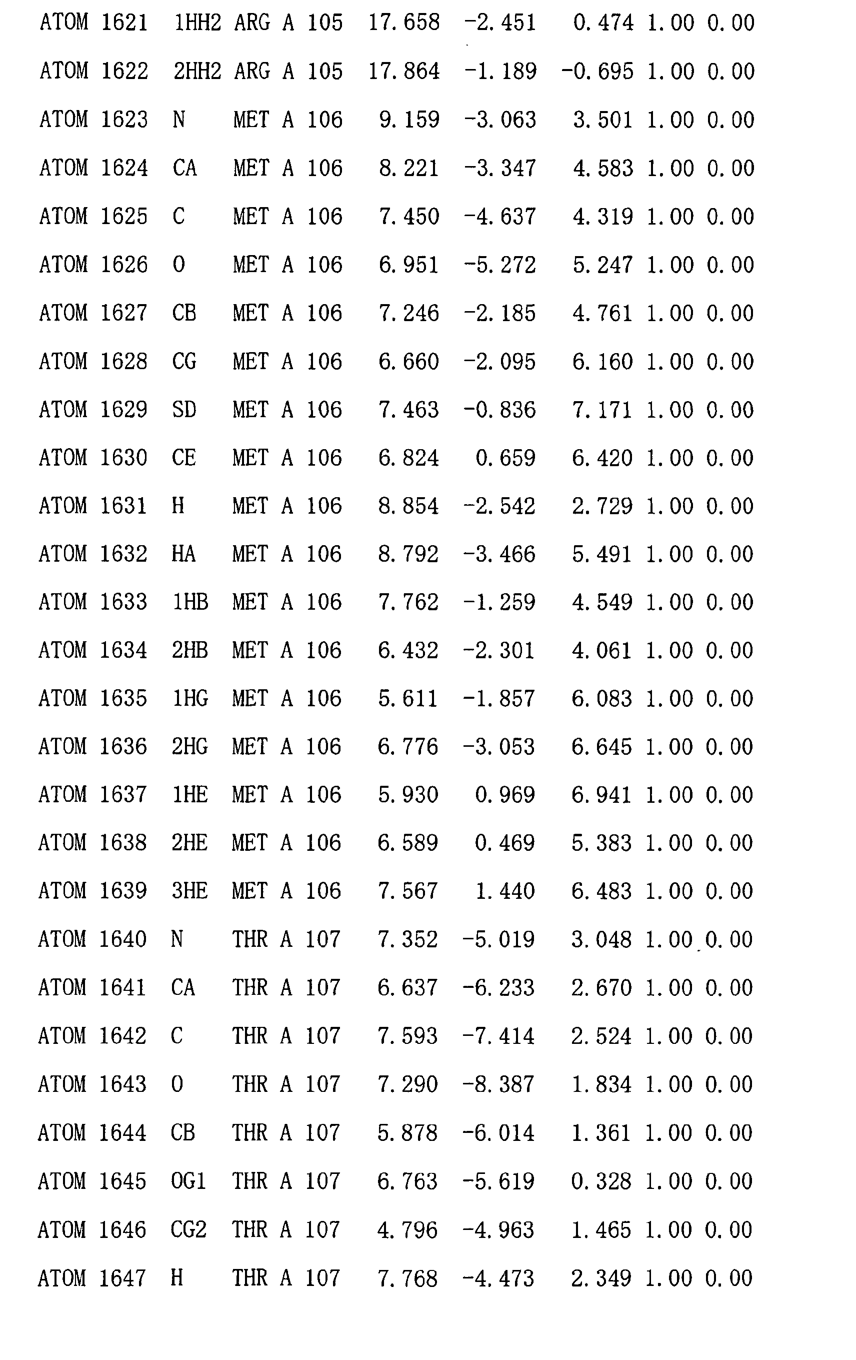

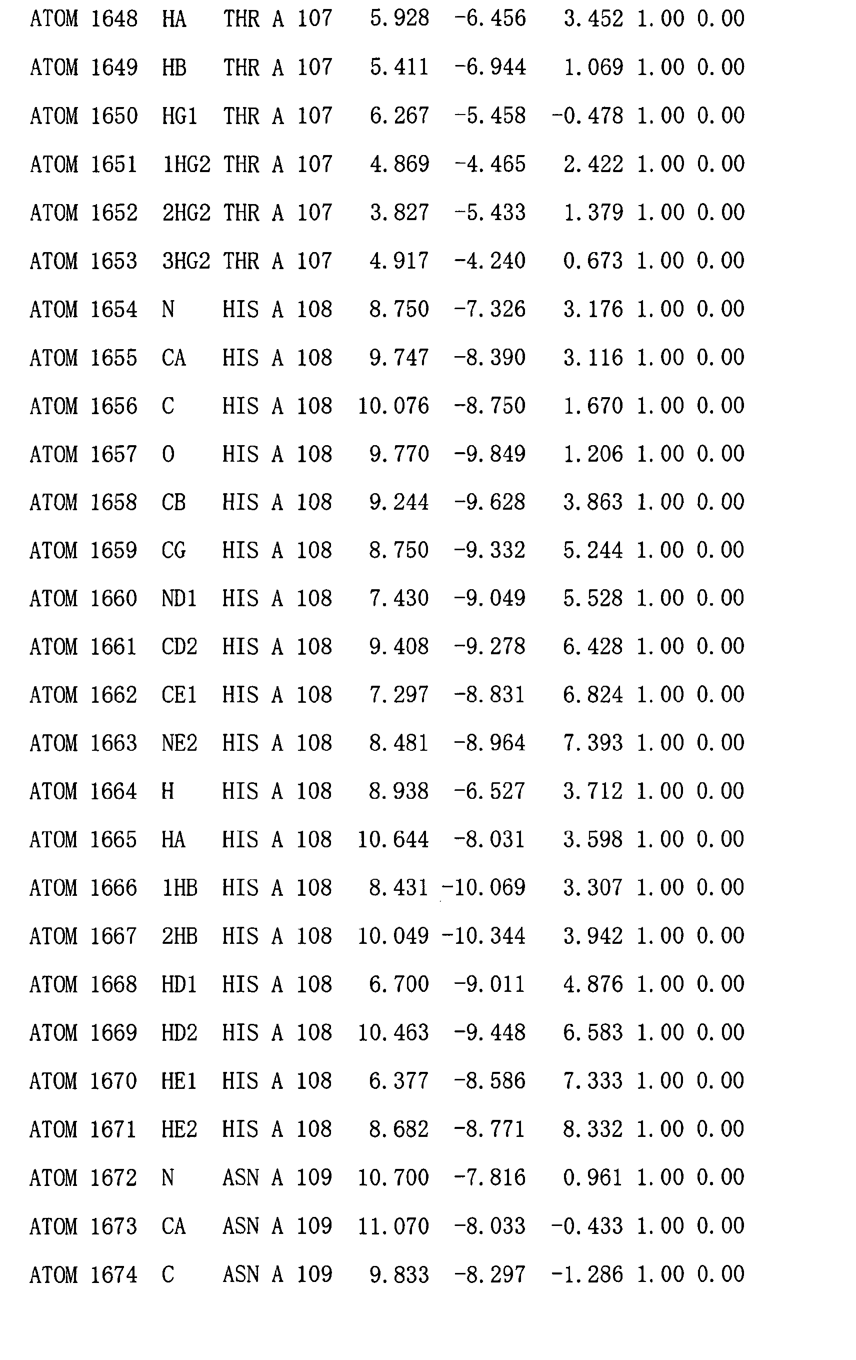

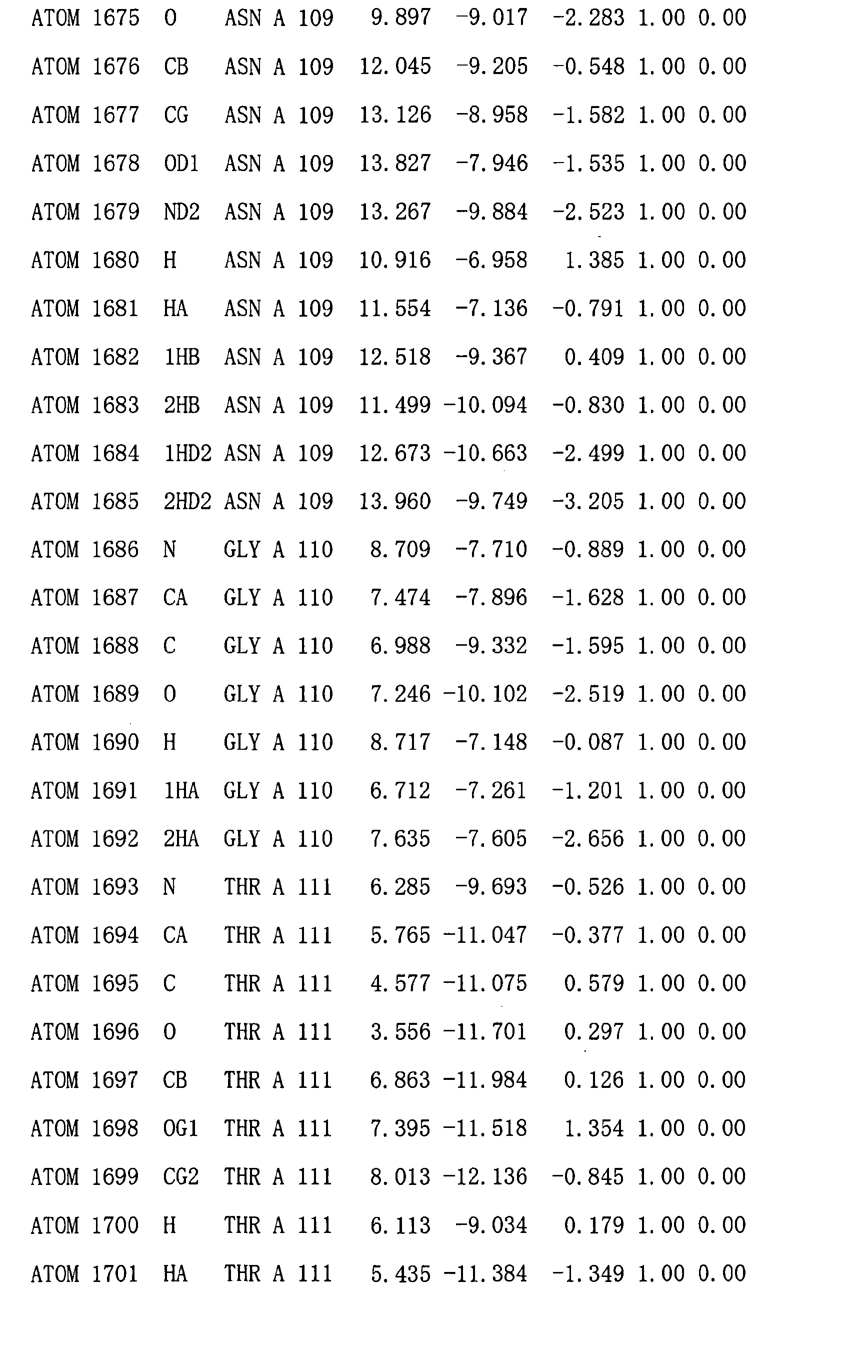

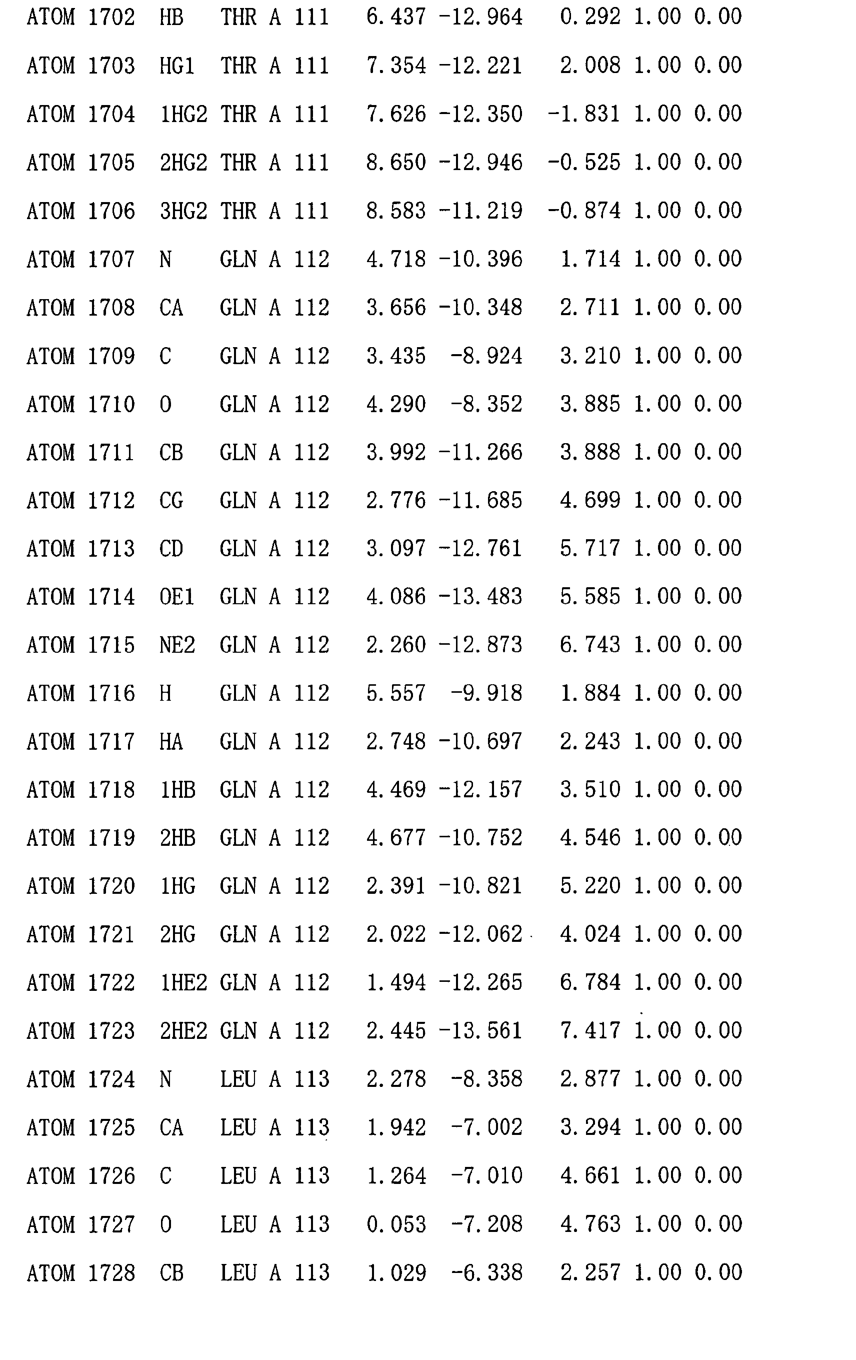

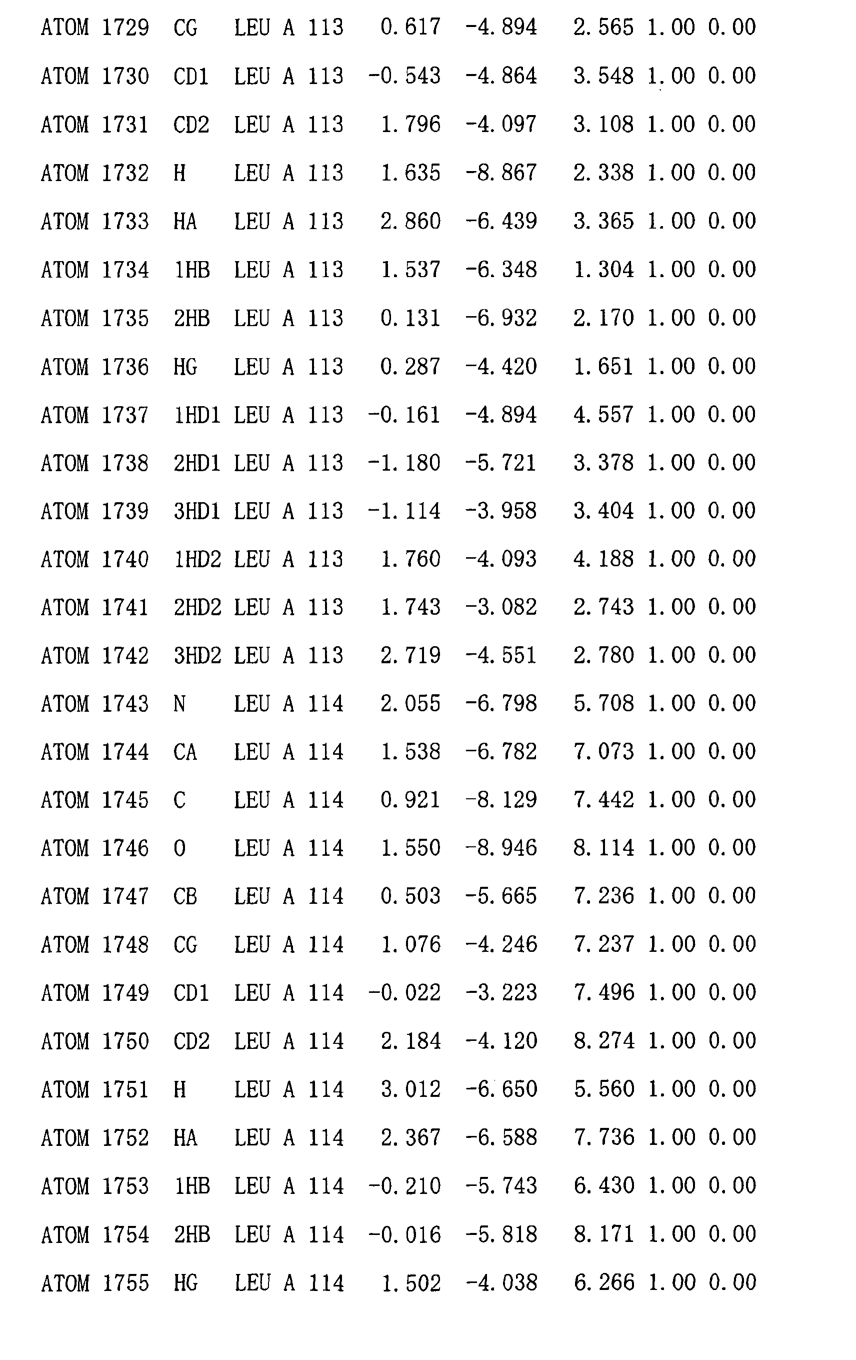

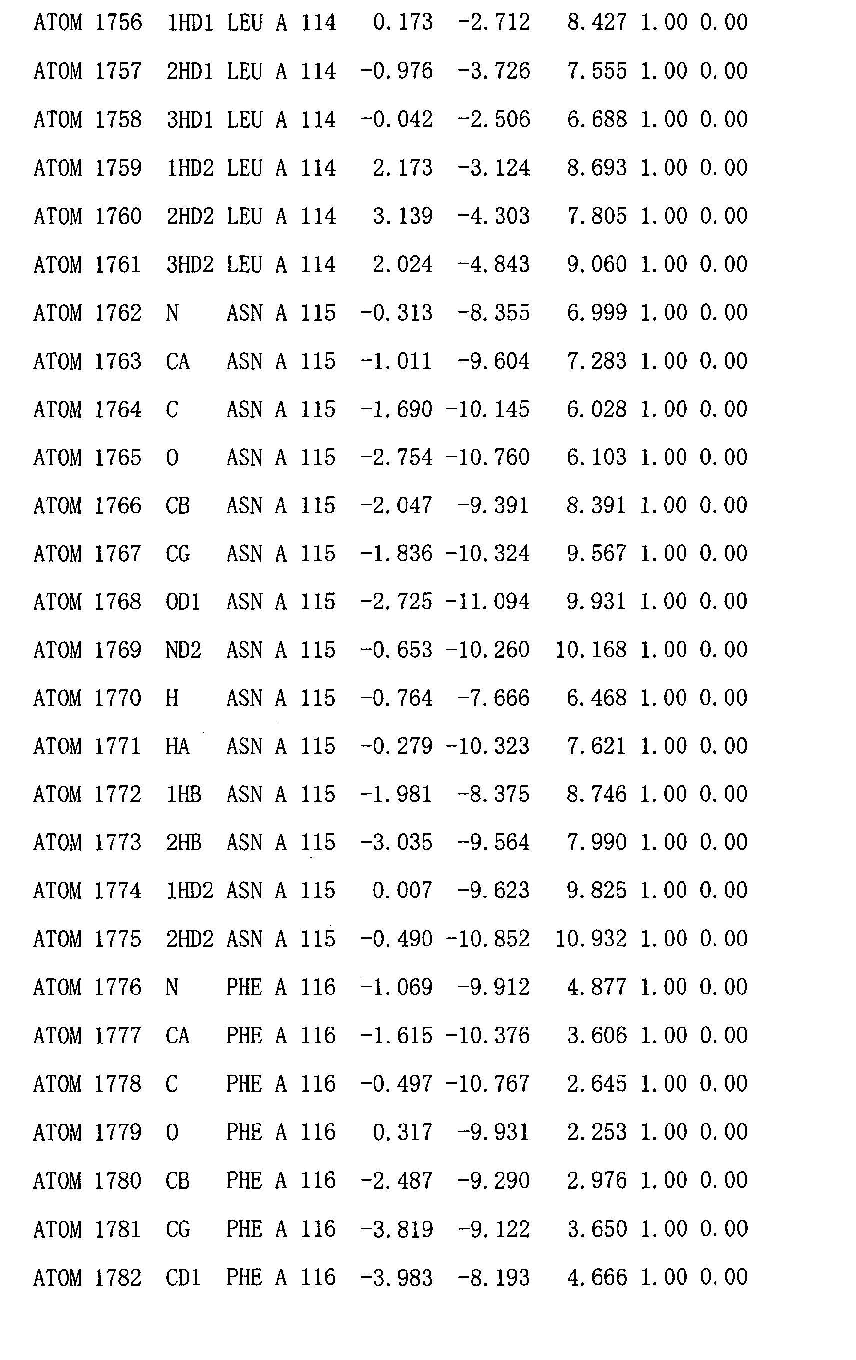

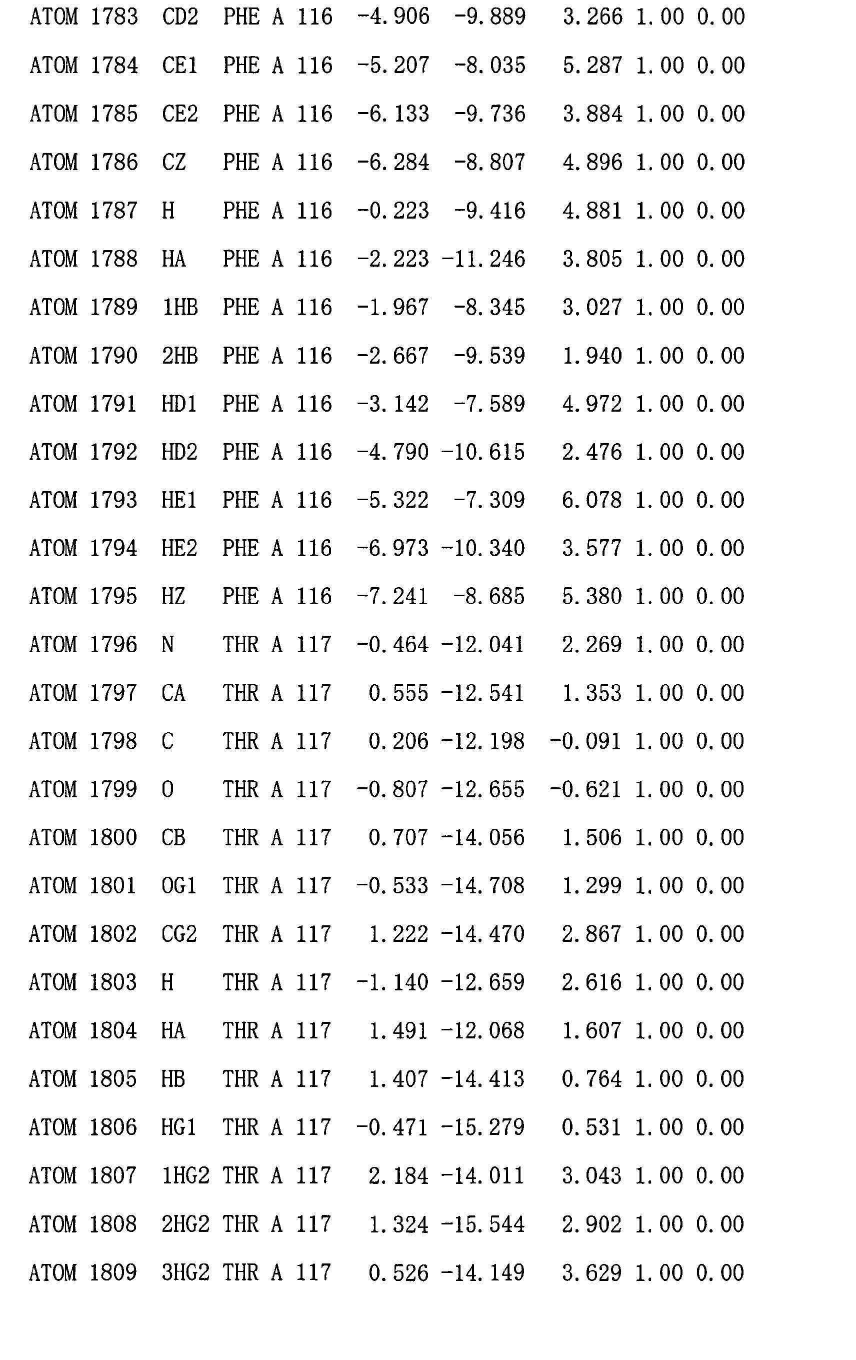

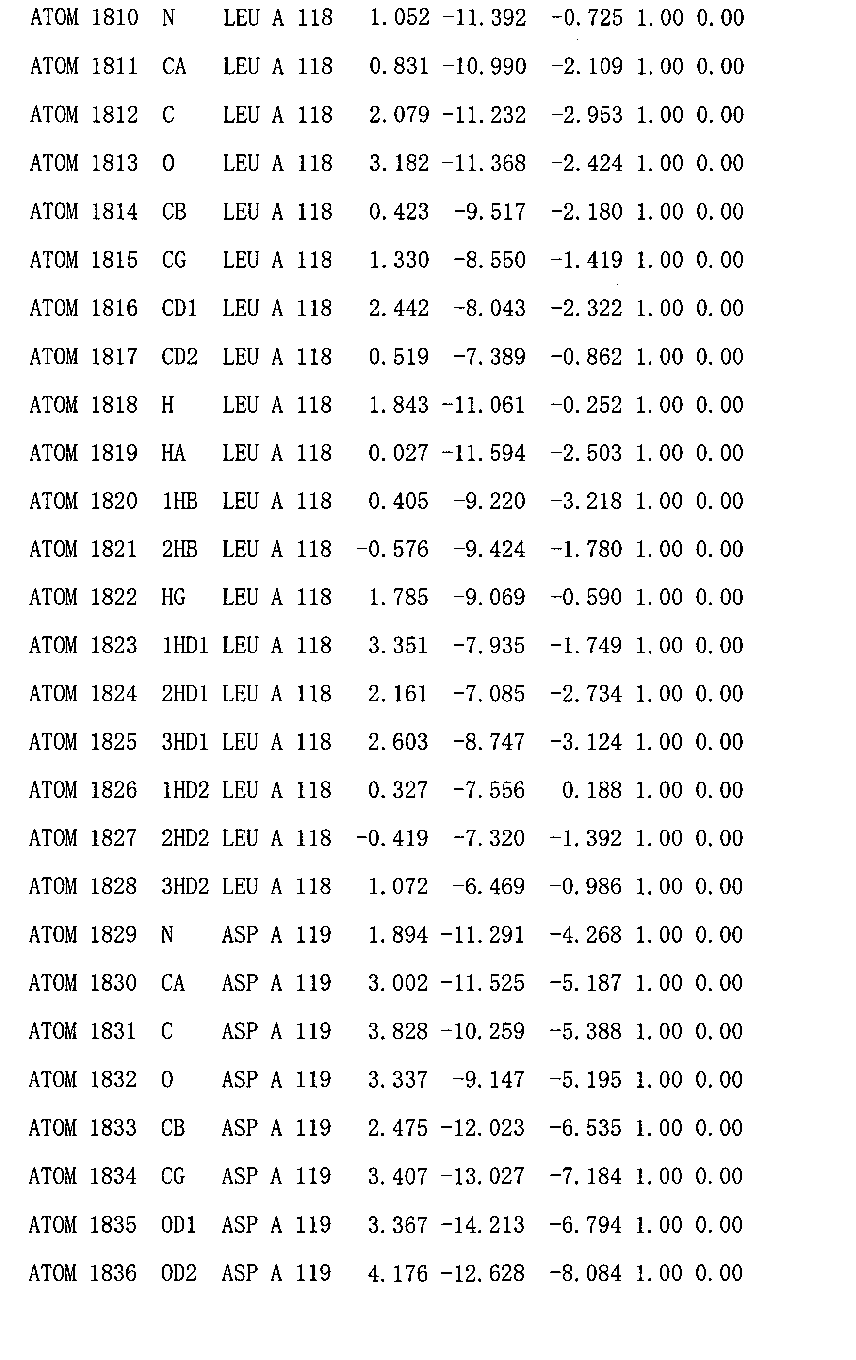

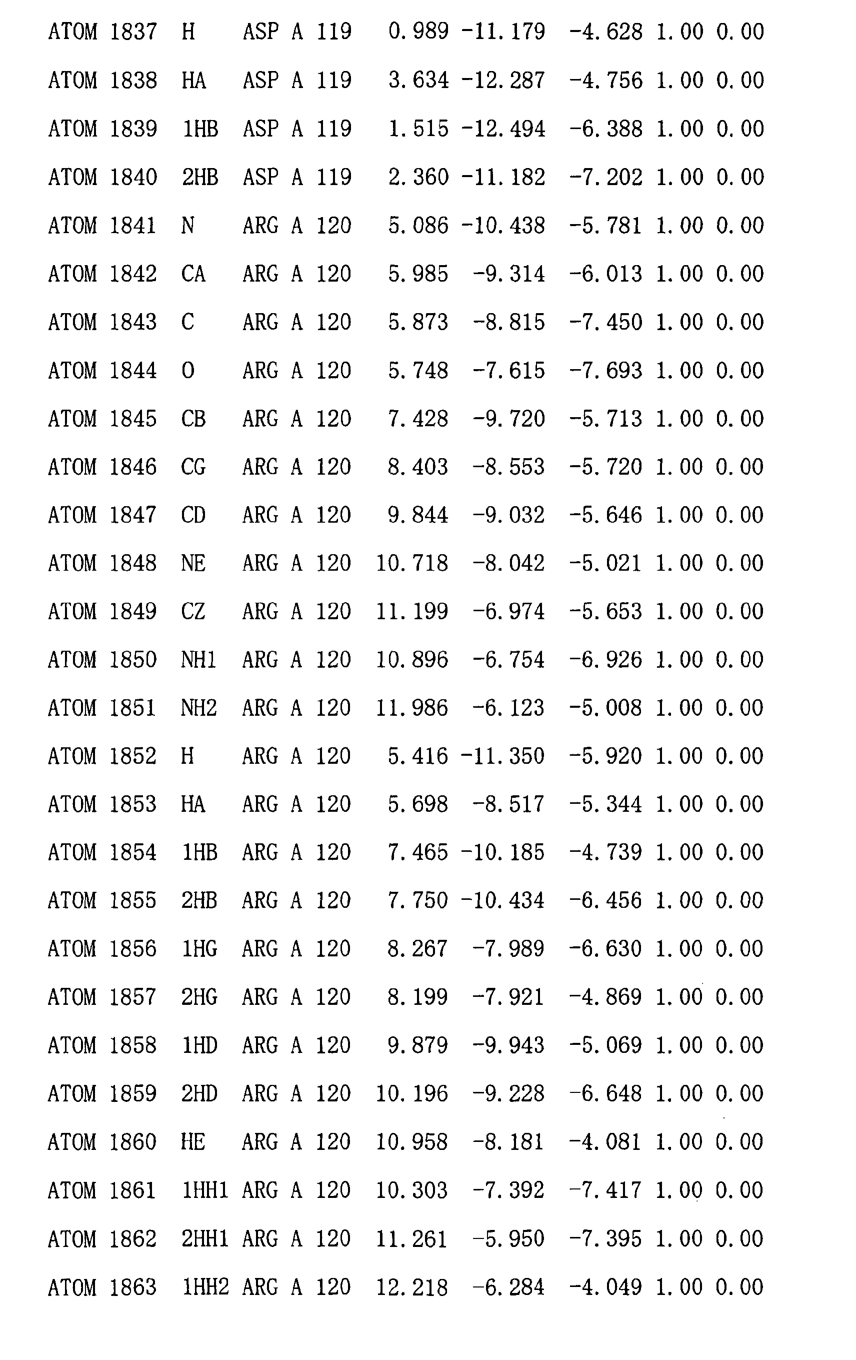

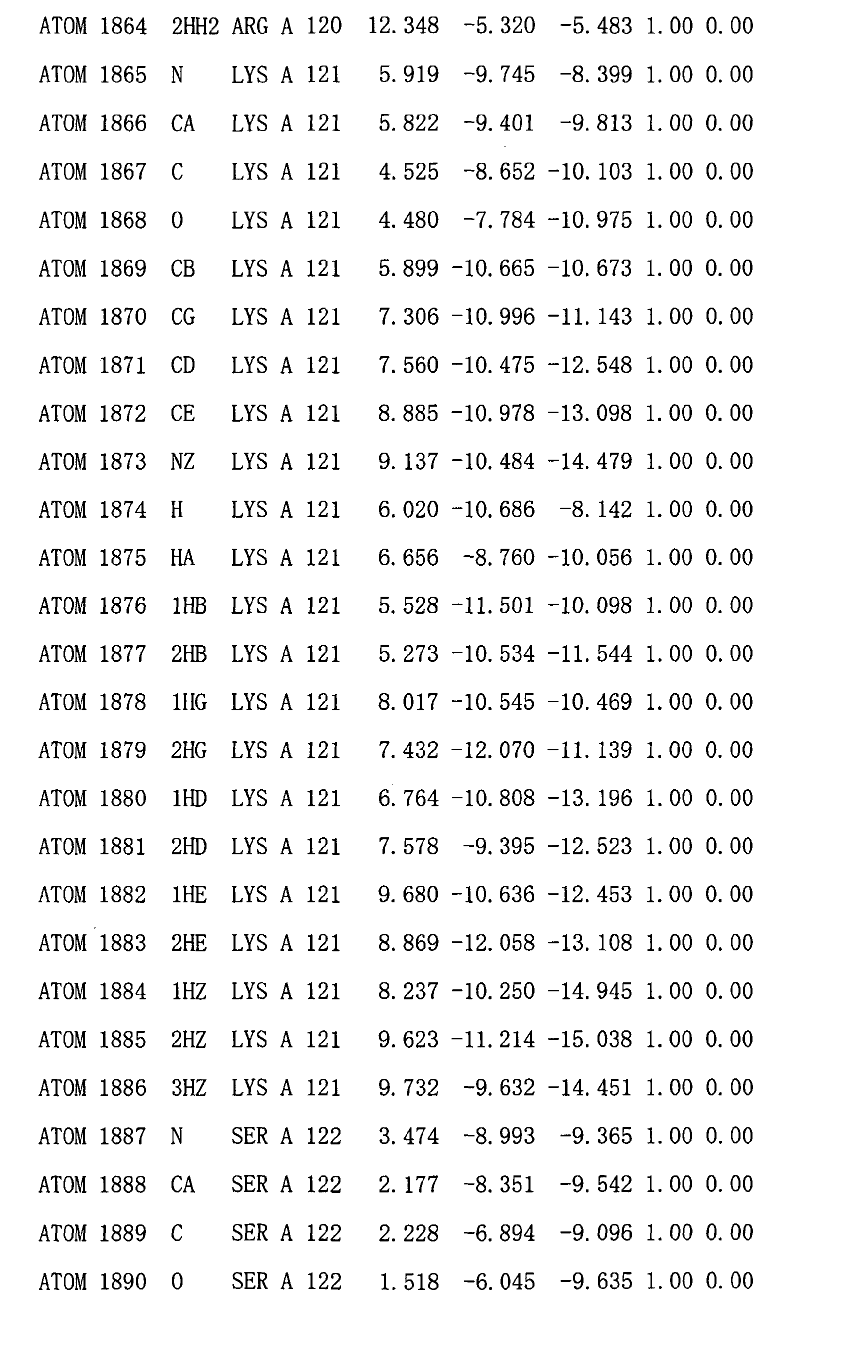

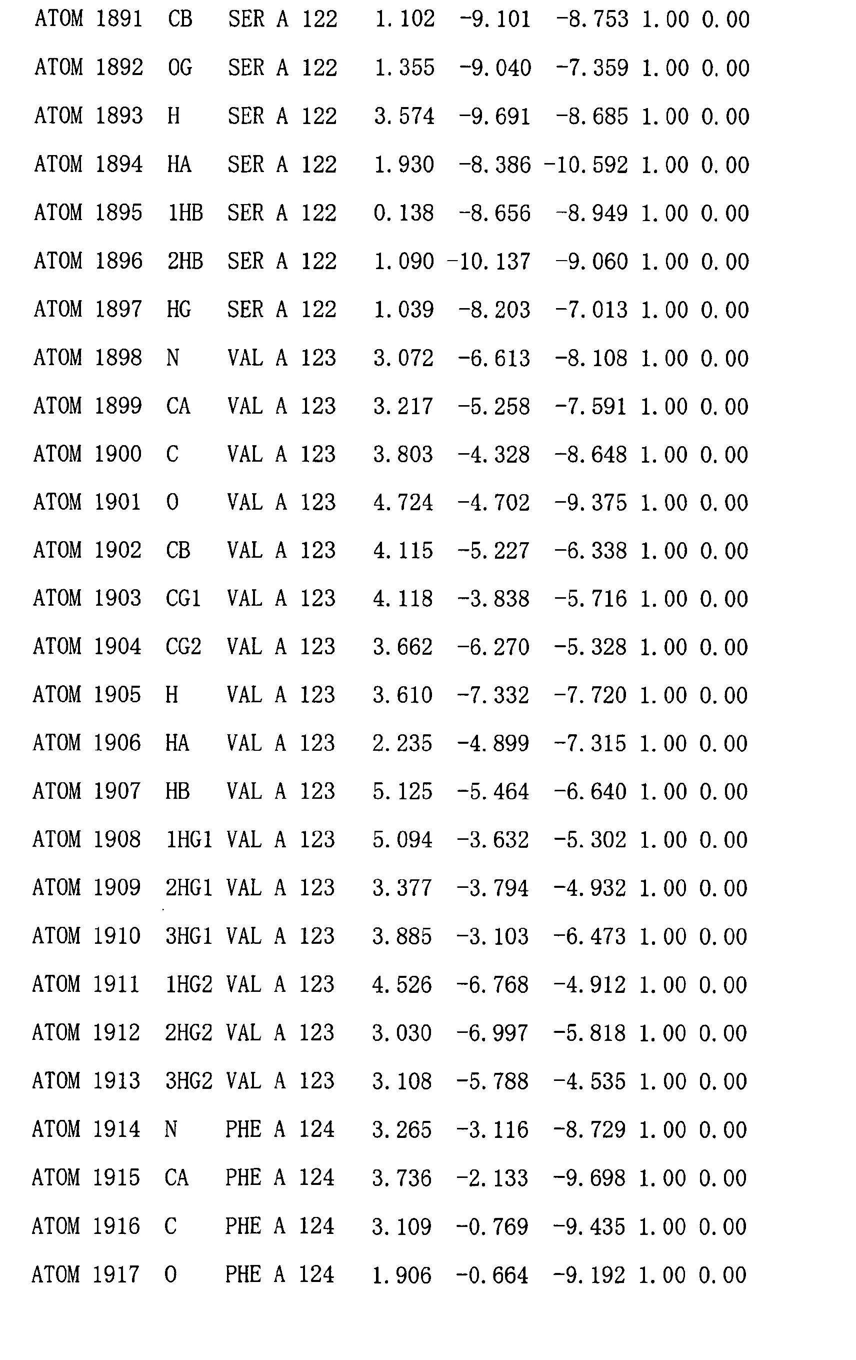

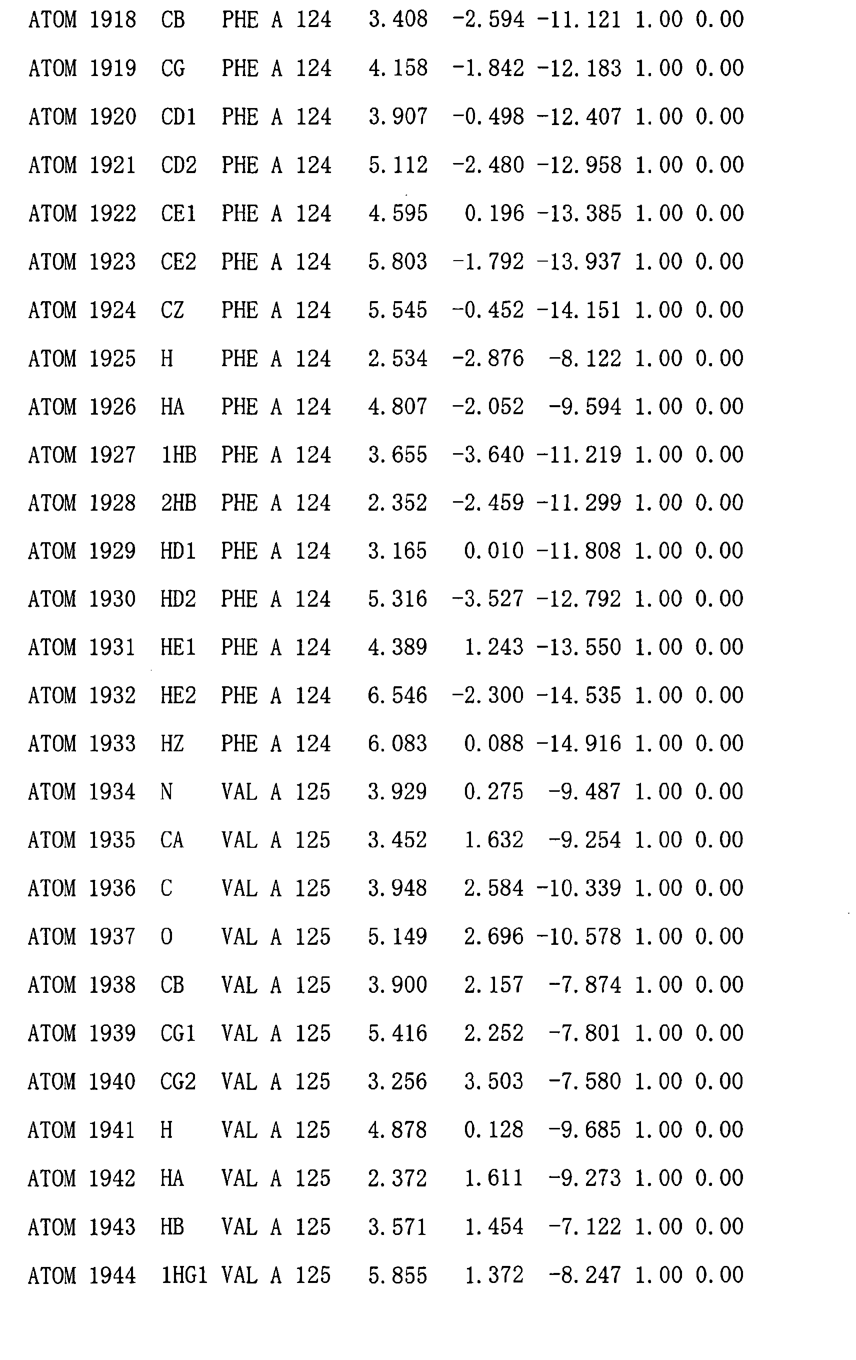

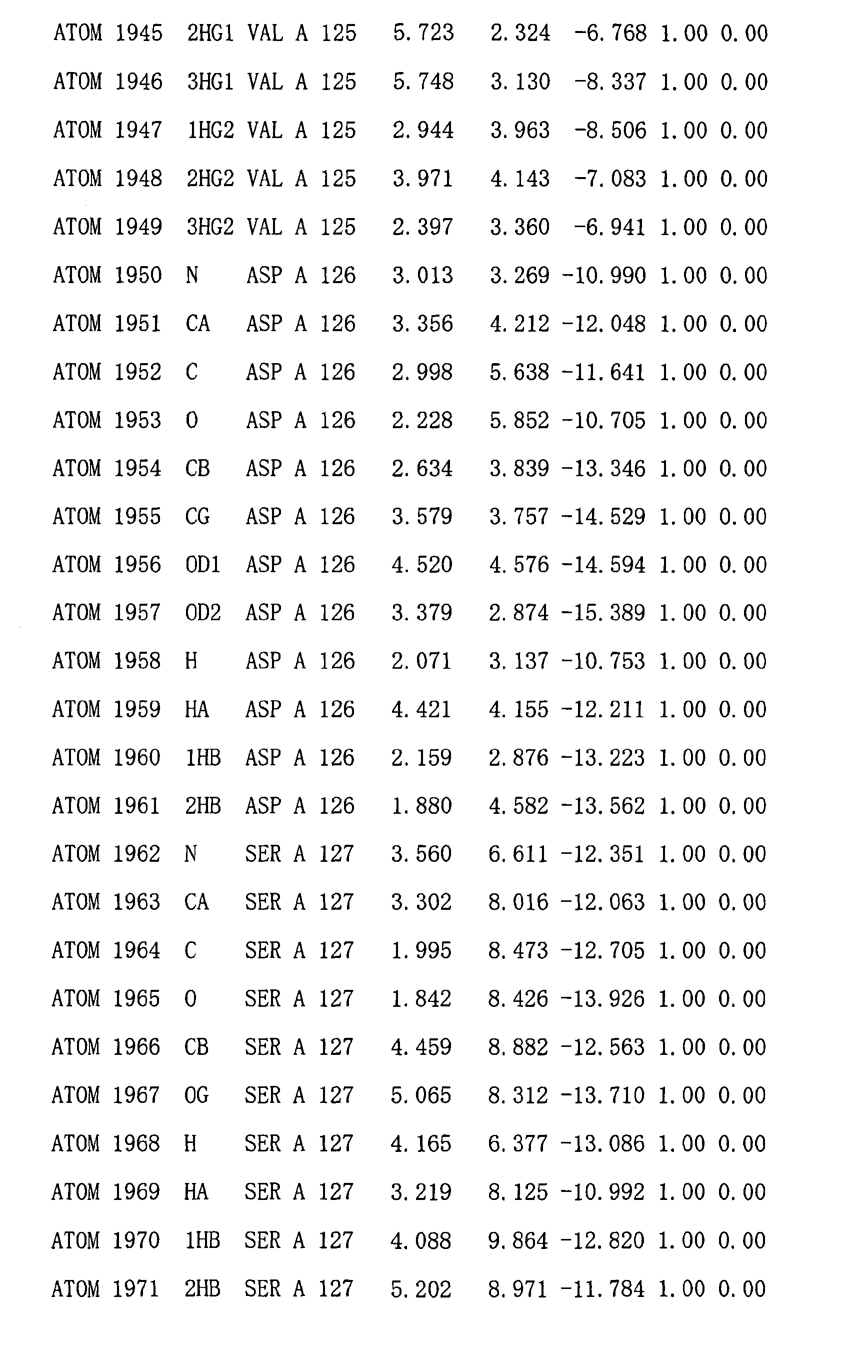

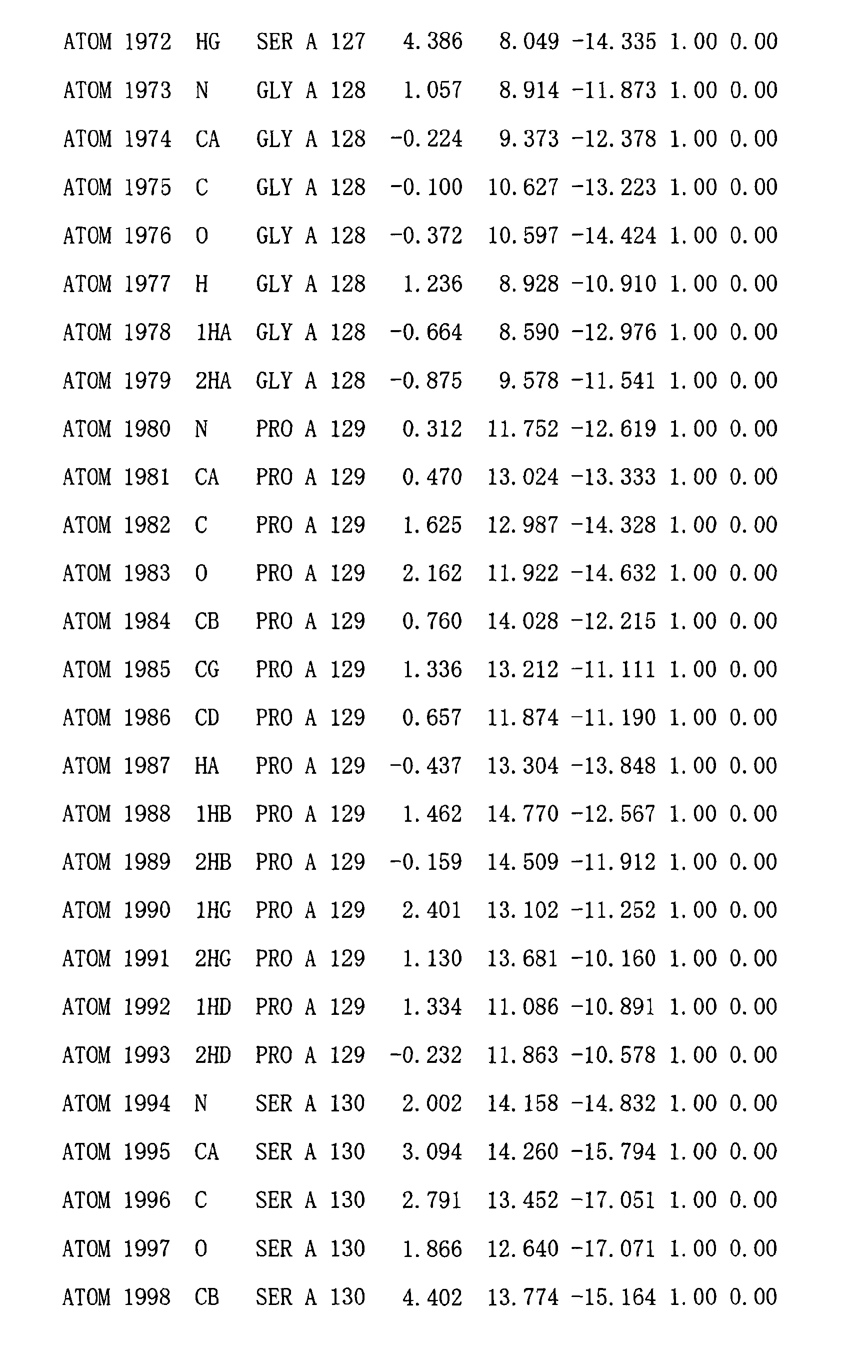

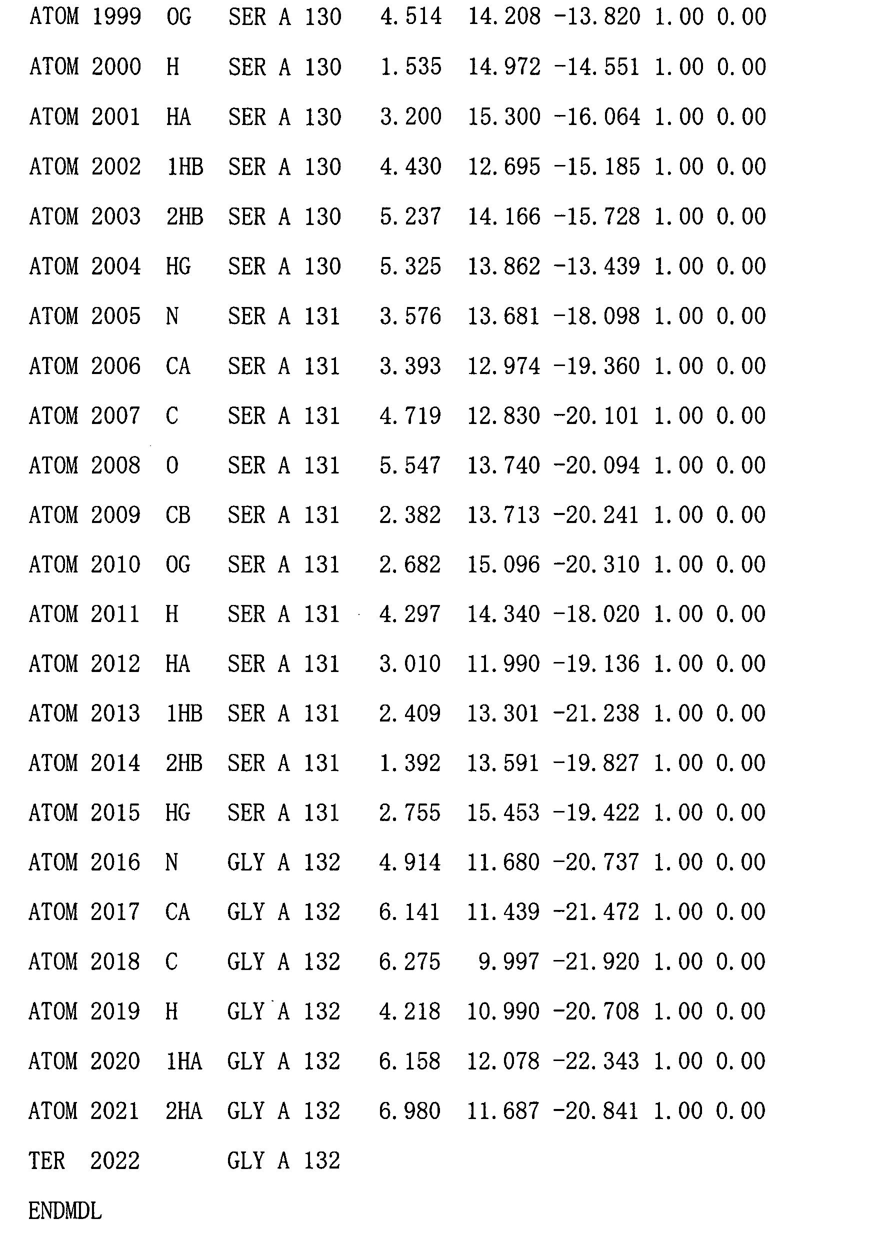

### Three-Dimensional Structure Coordinate 20

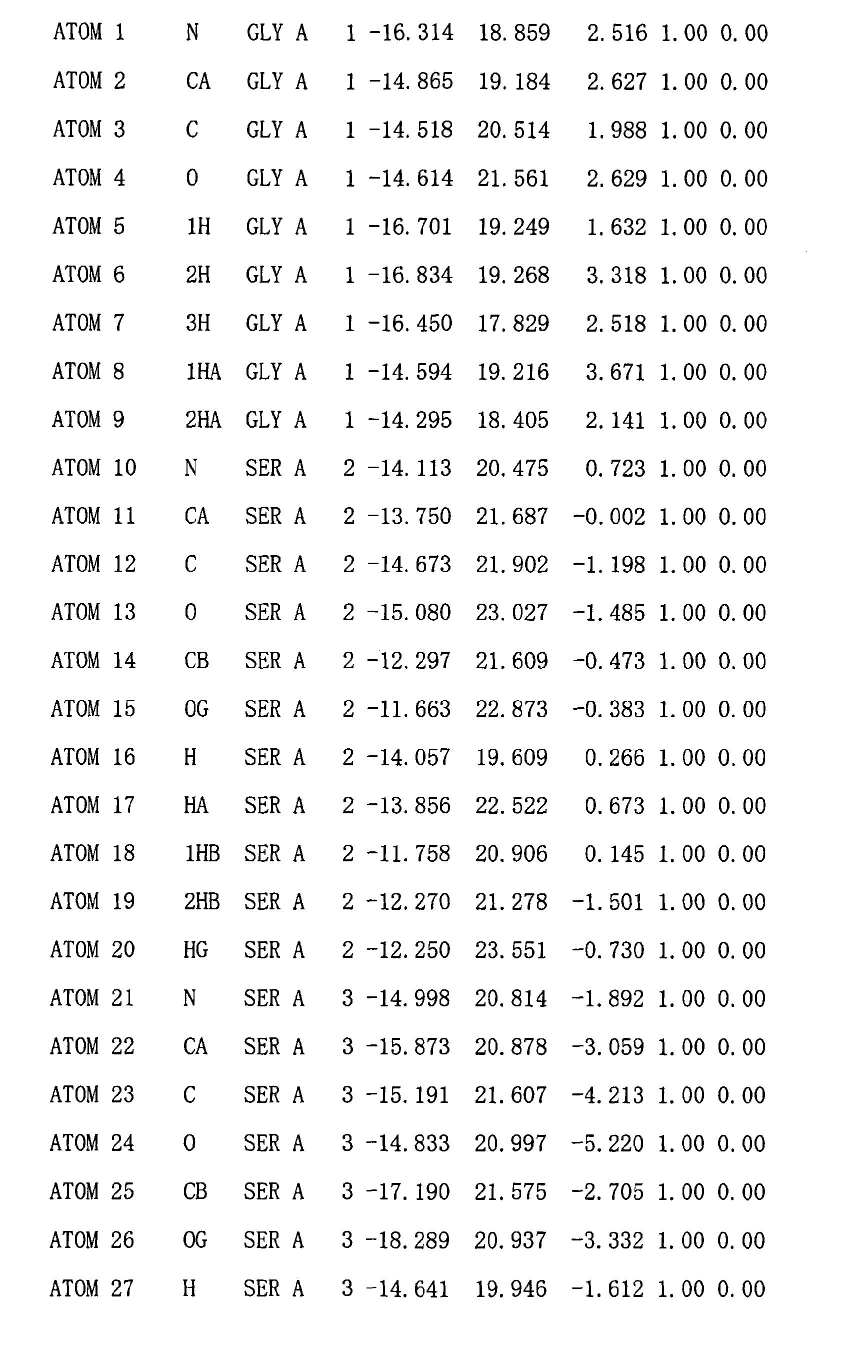

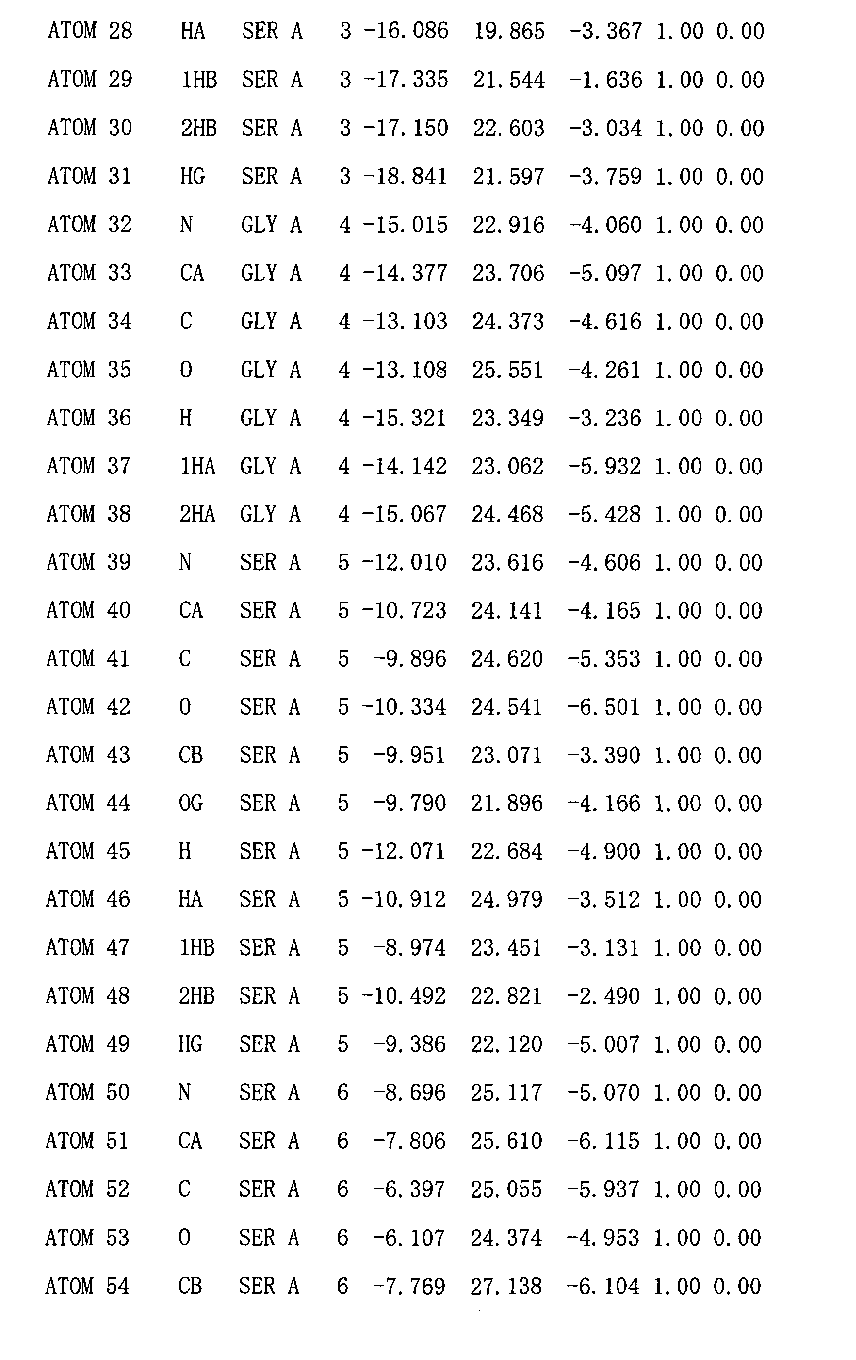

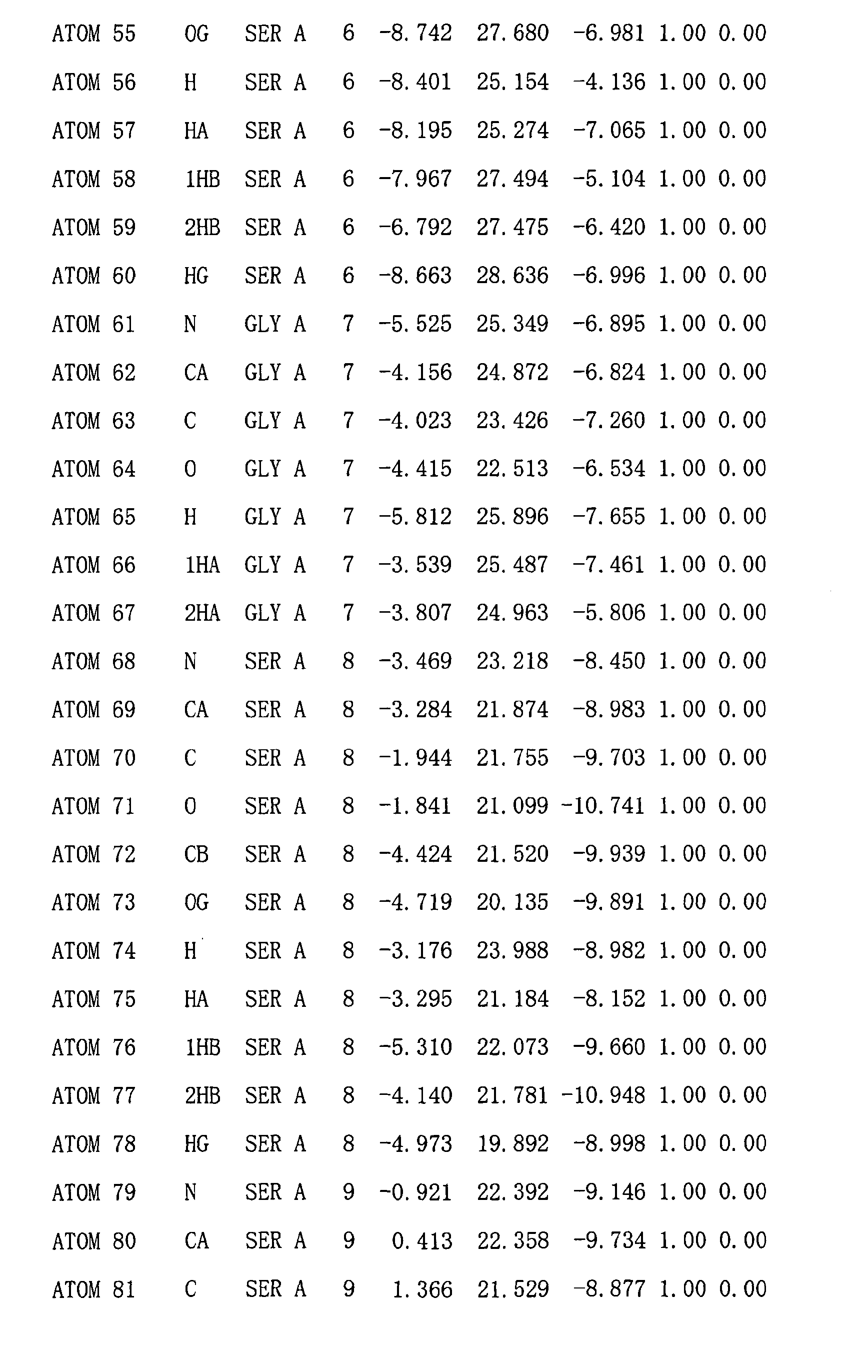

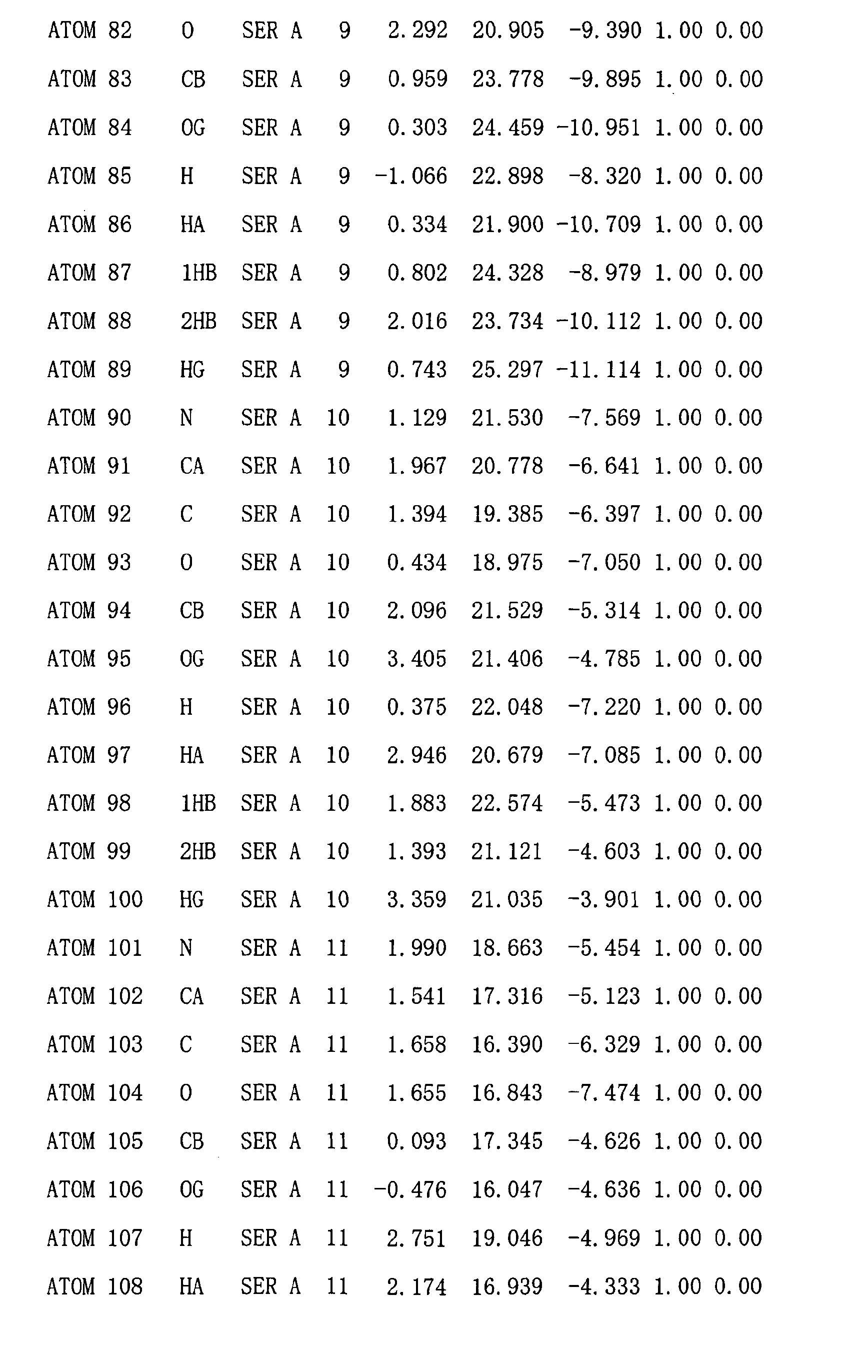

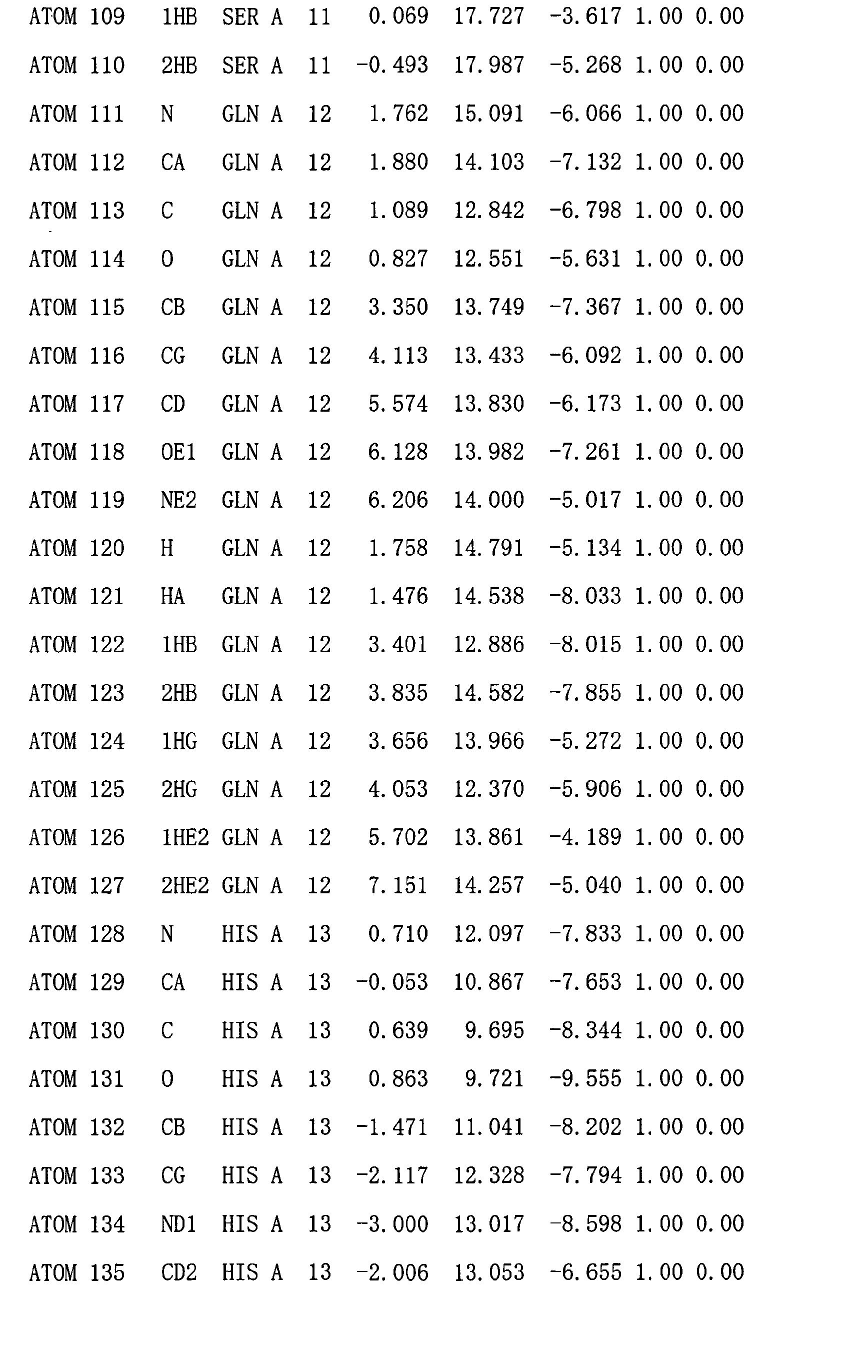

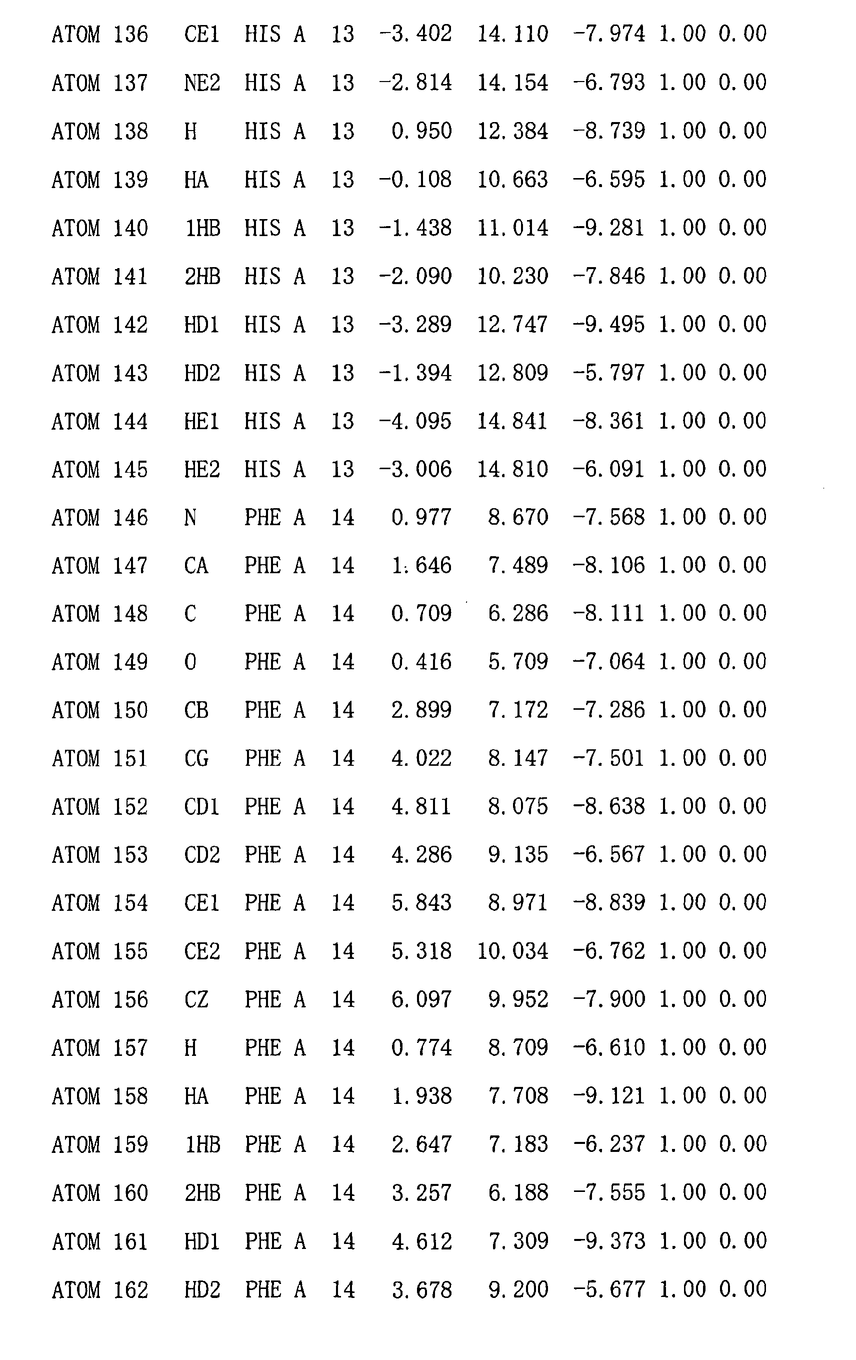

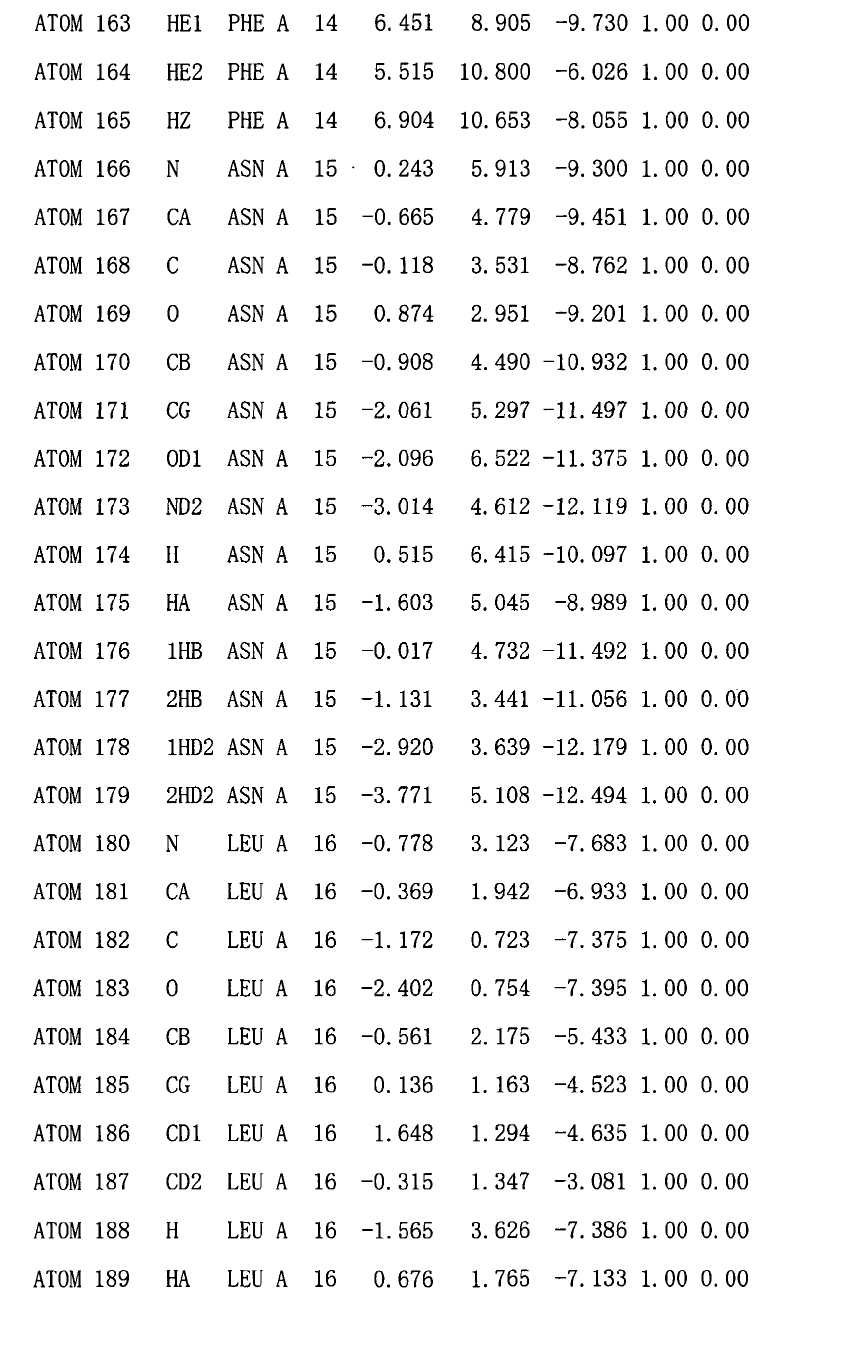

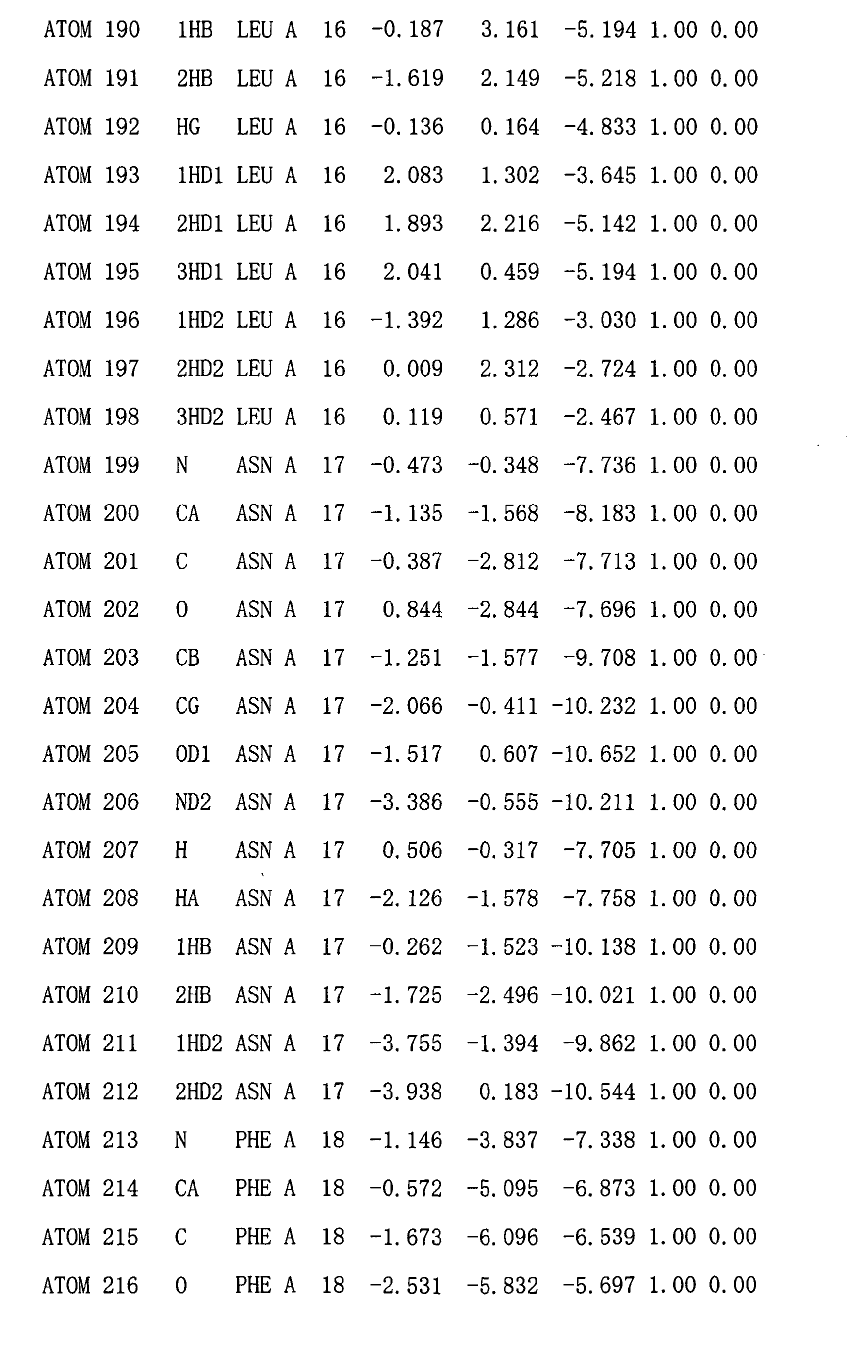

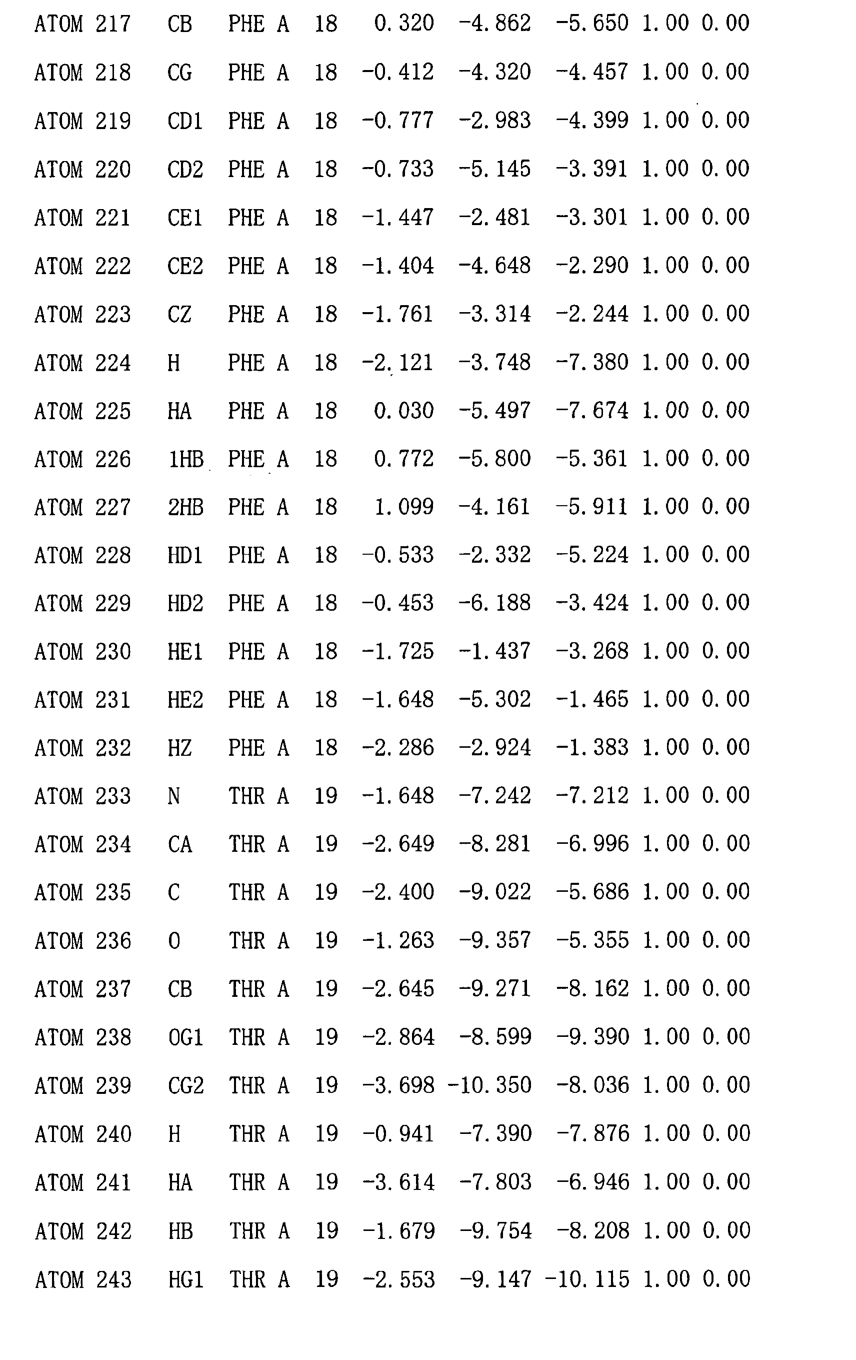

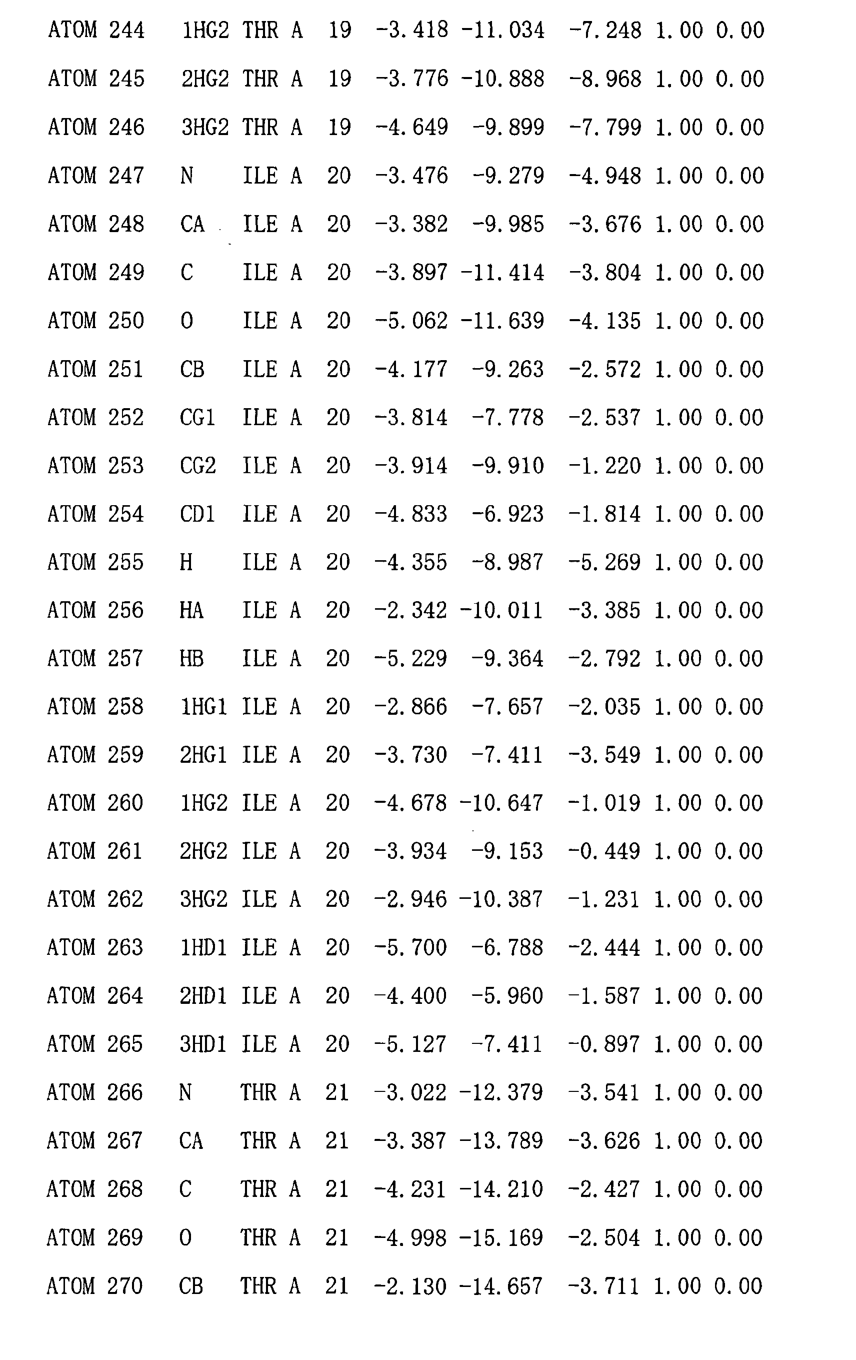

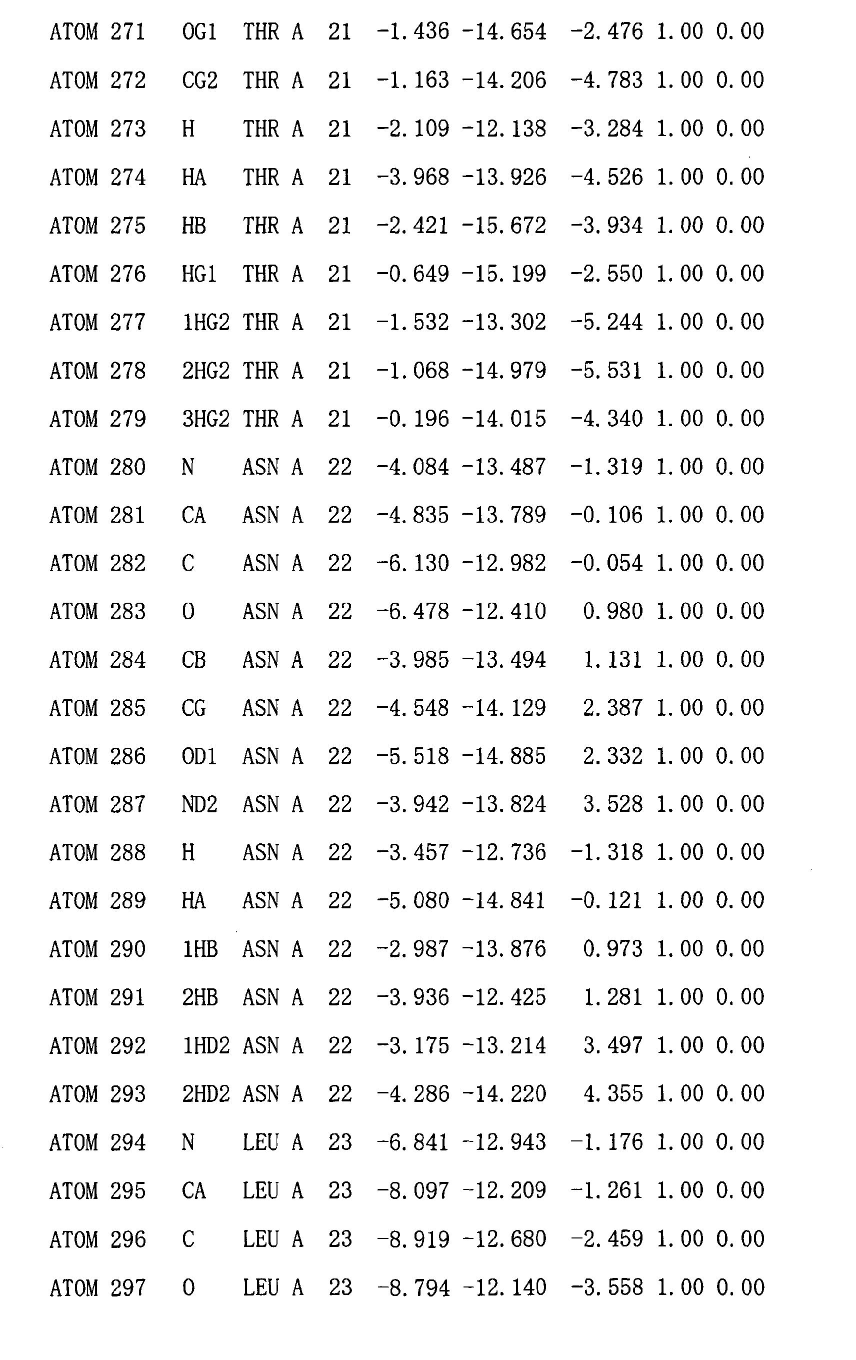

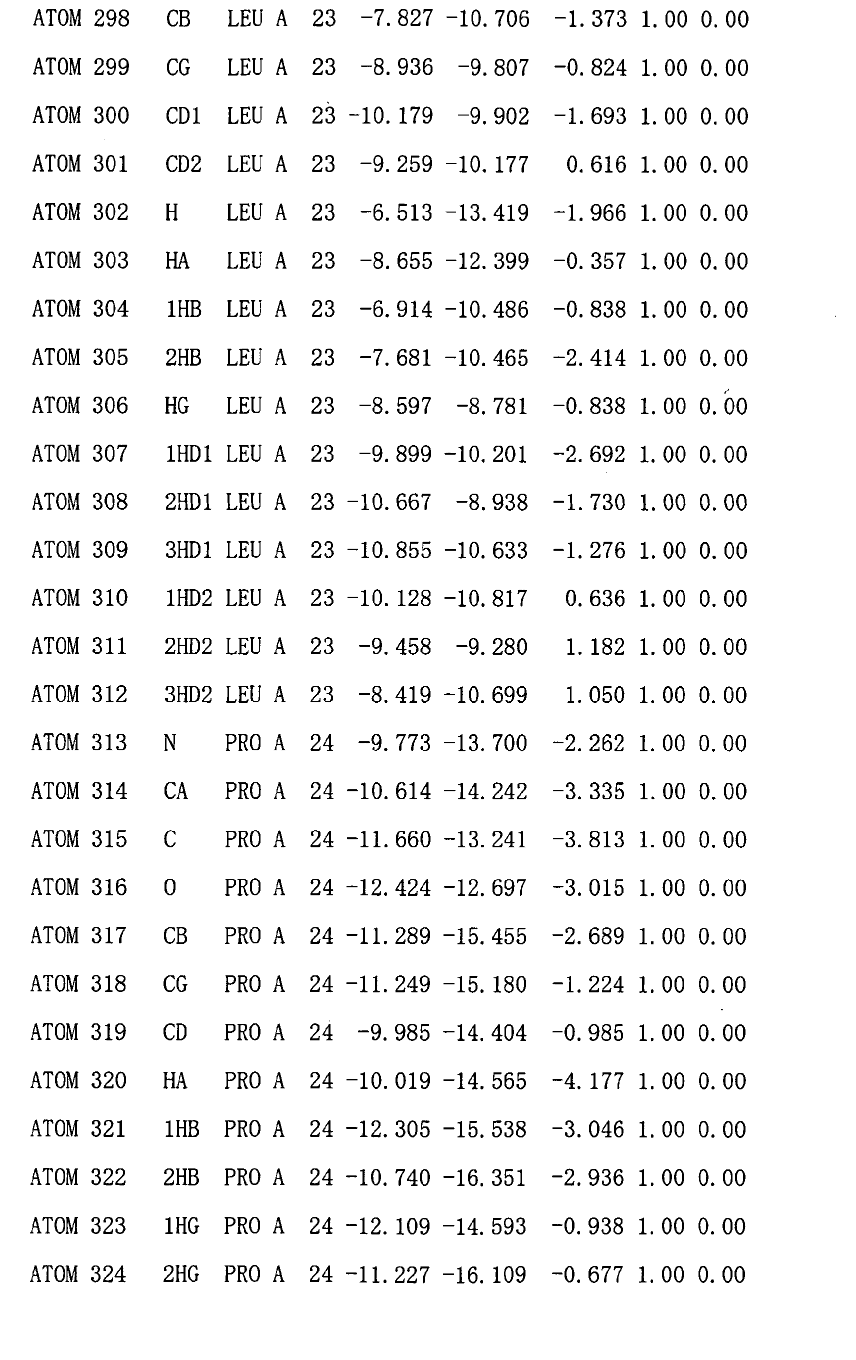

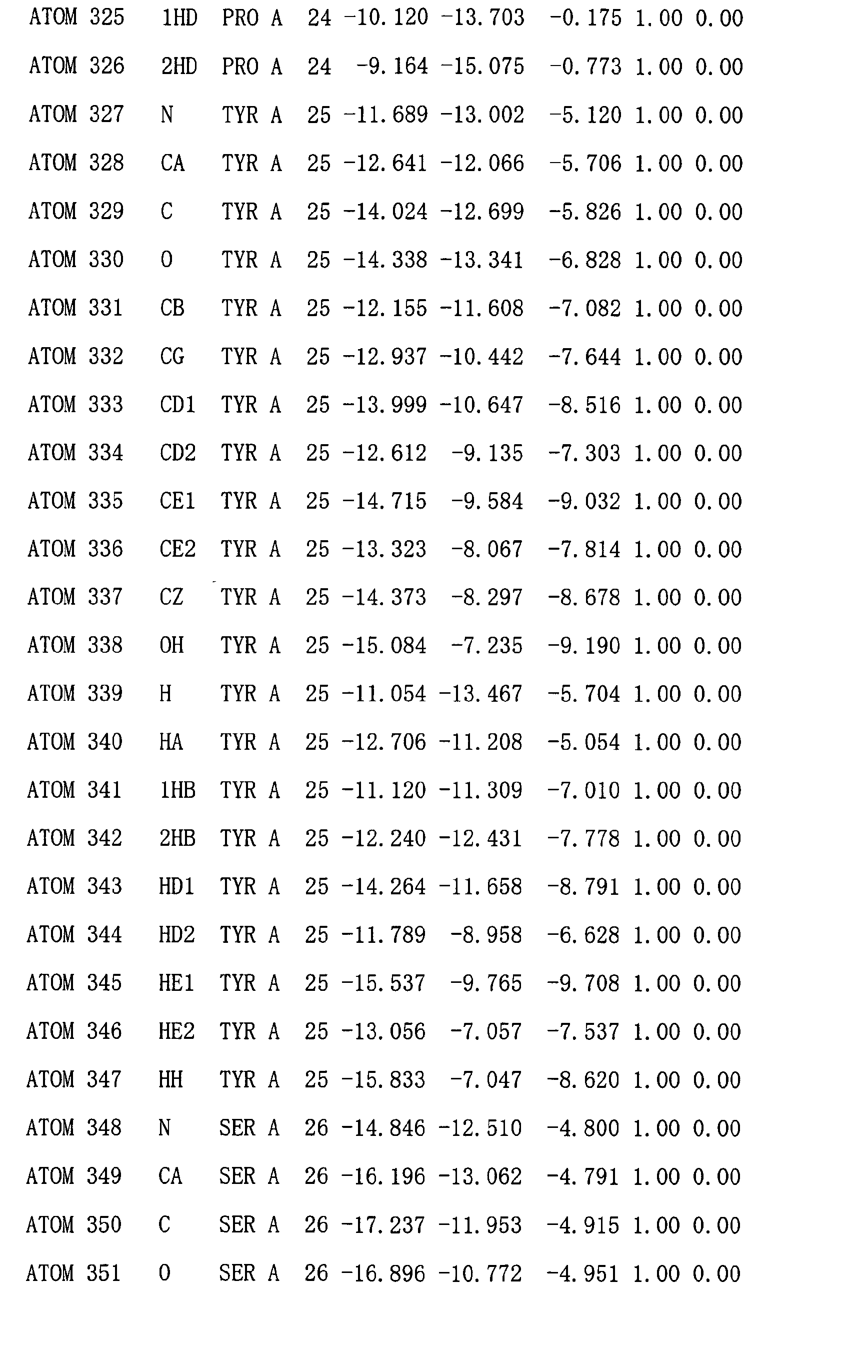

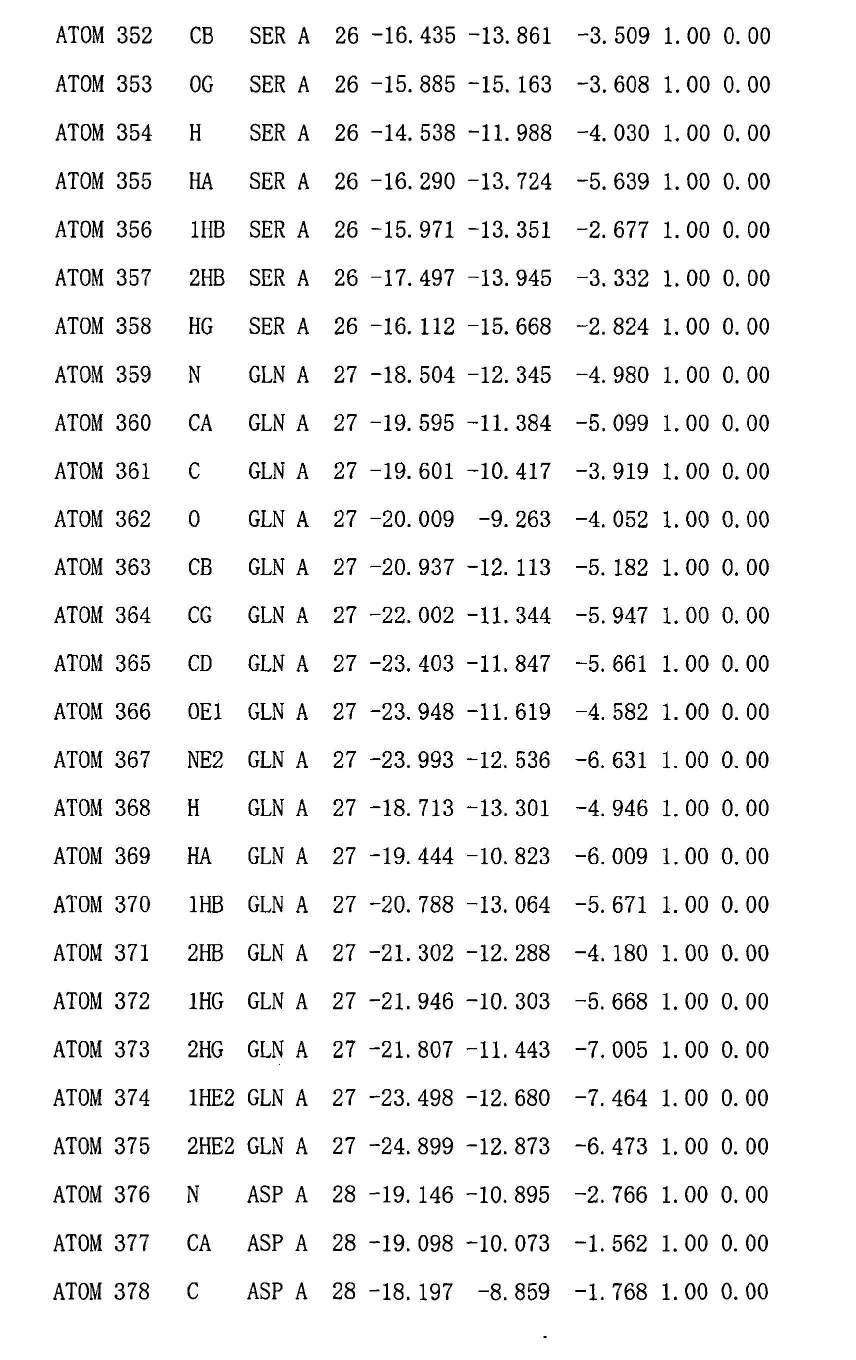

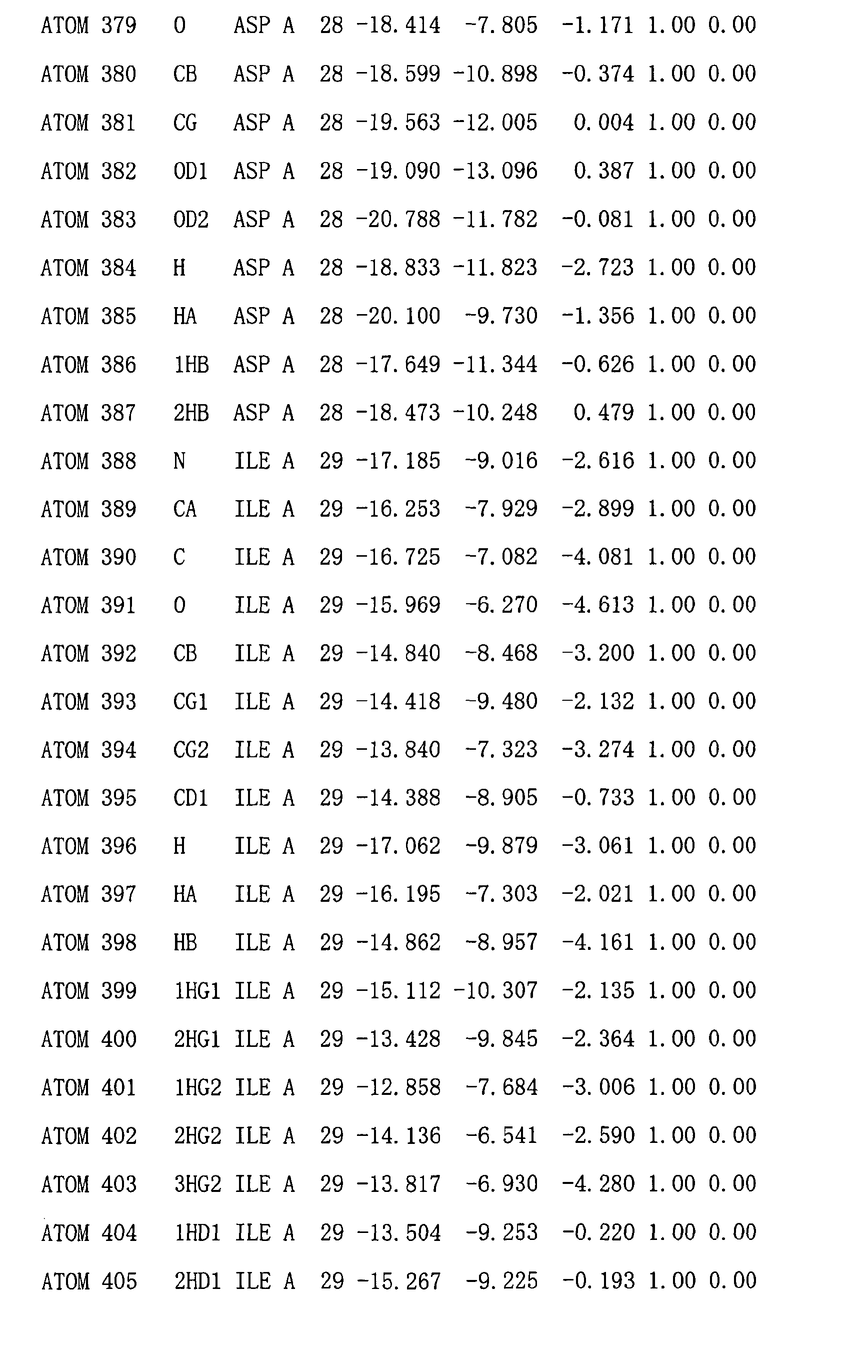

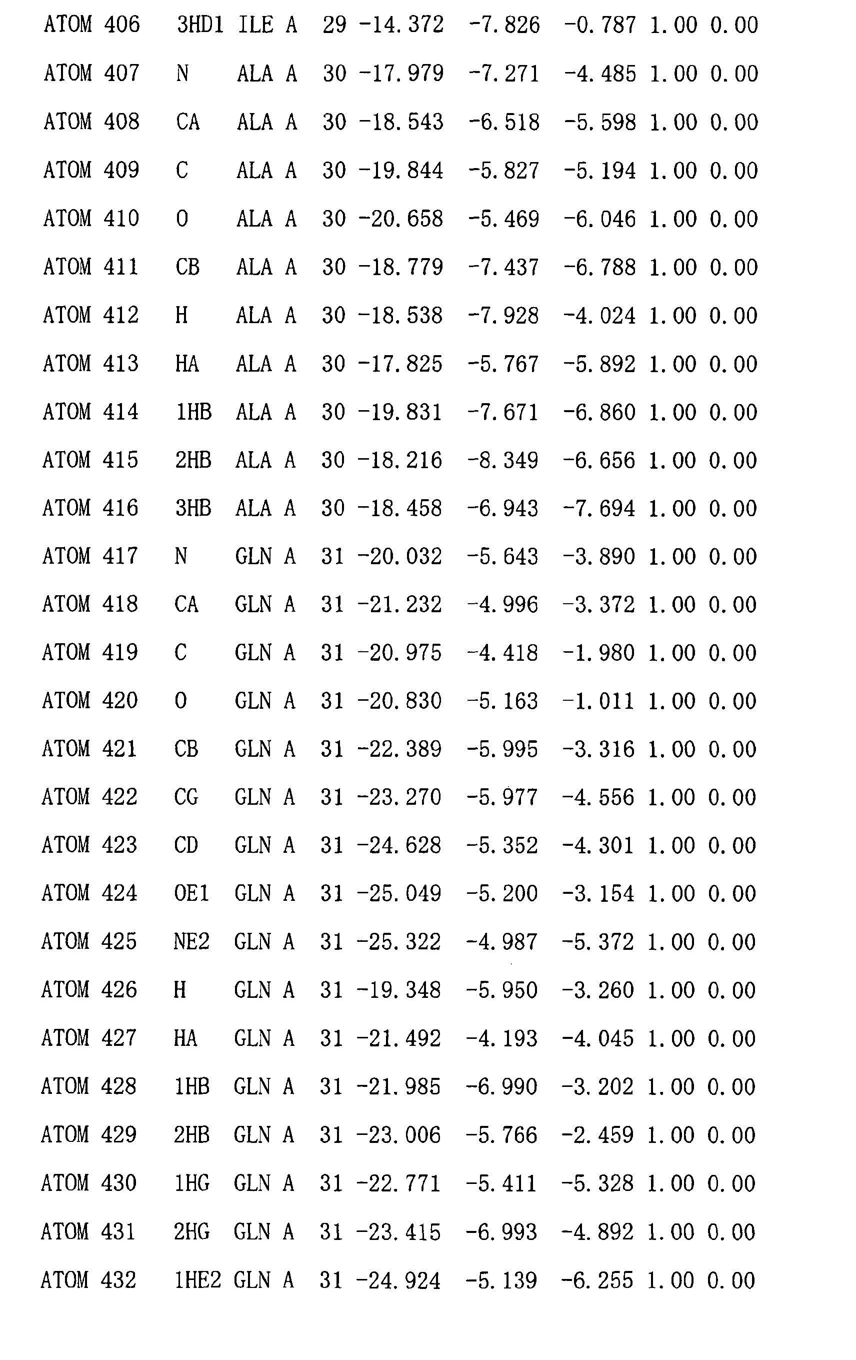

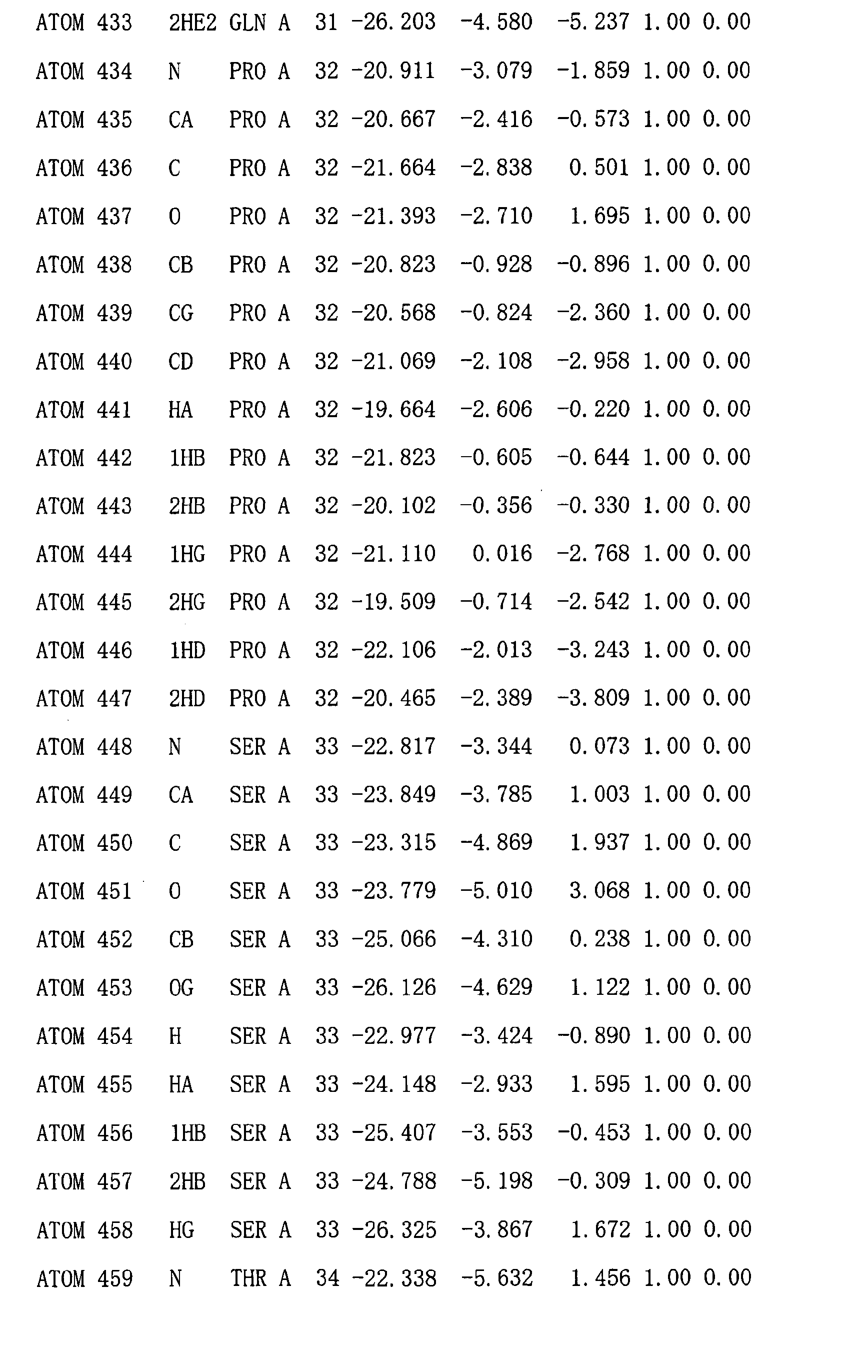

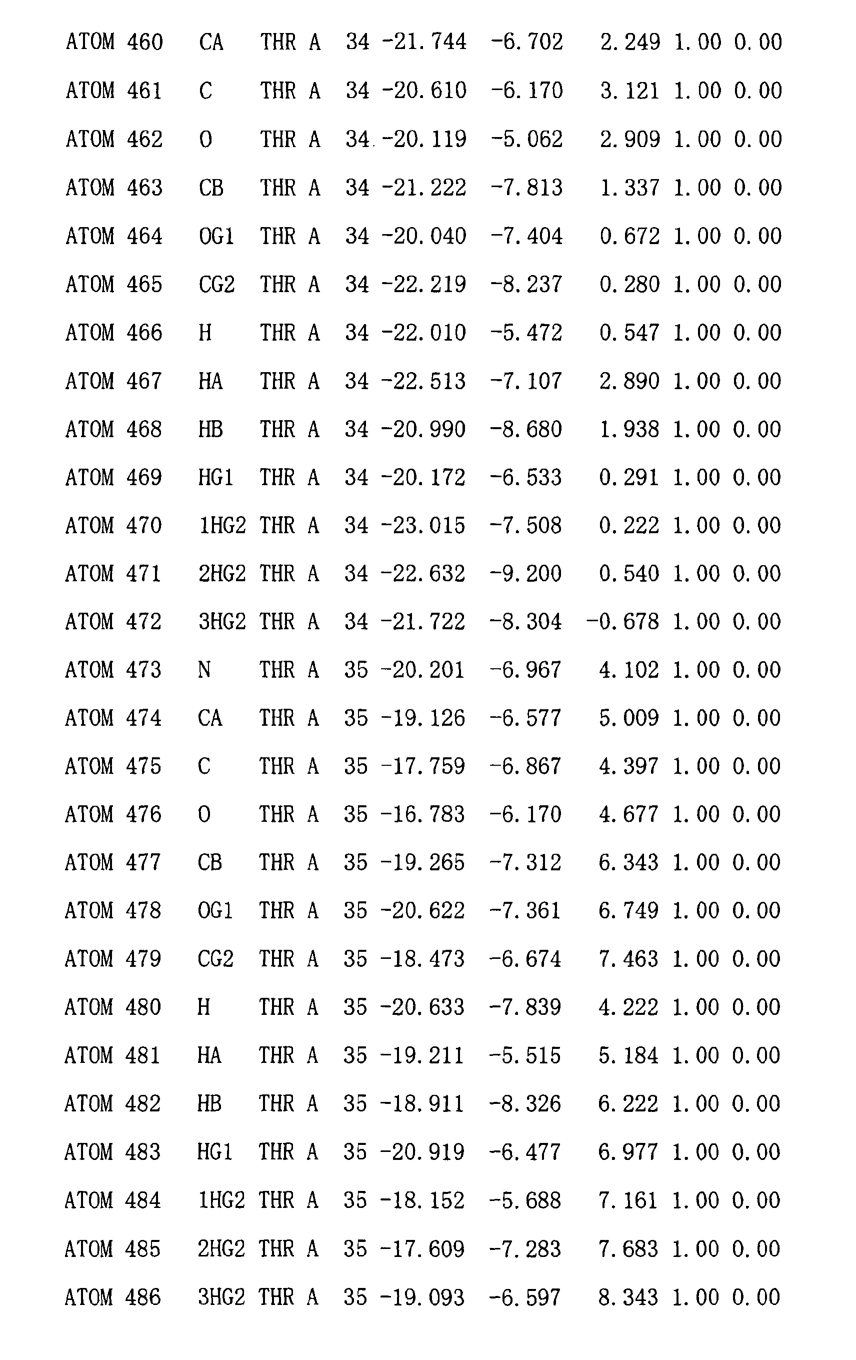

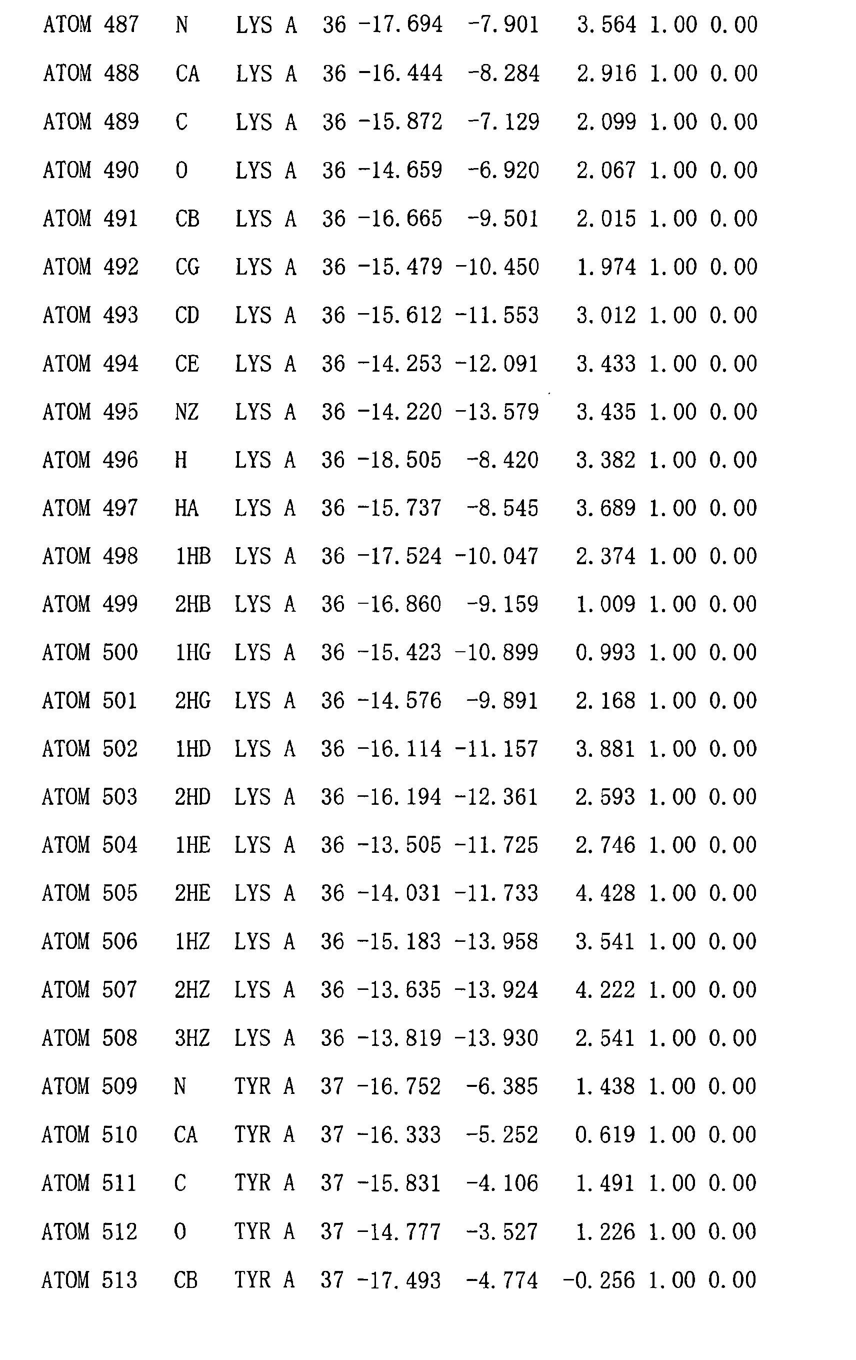

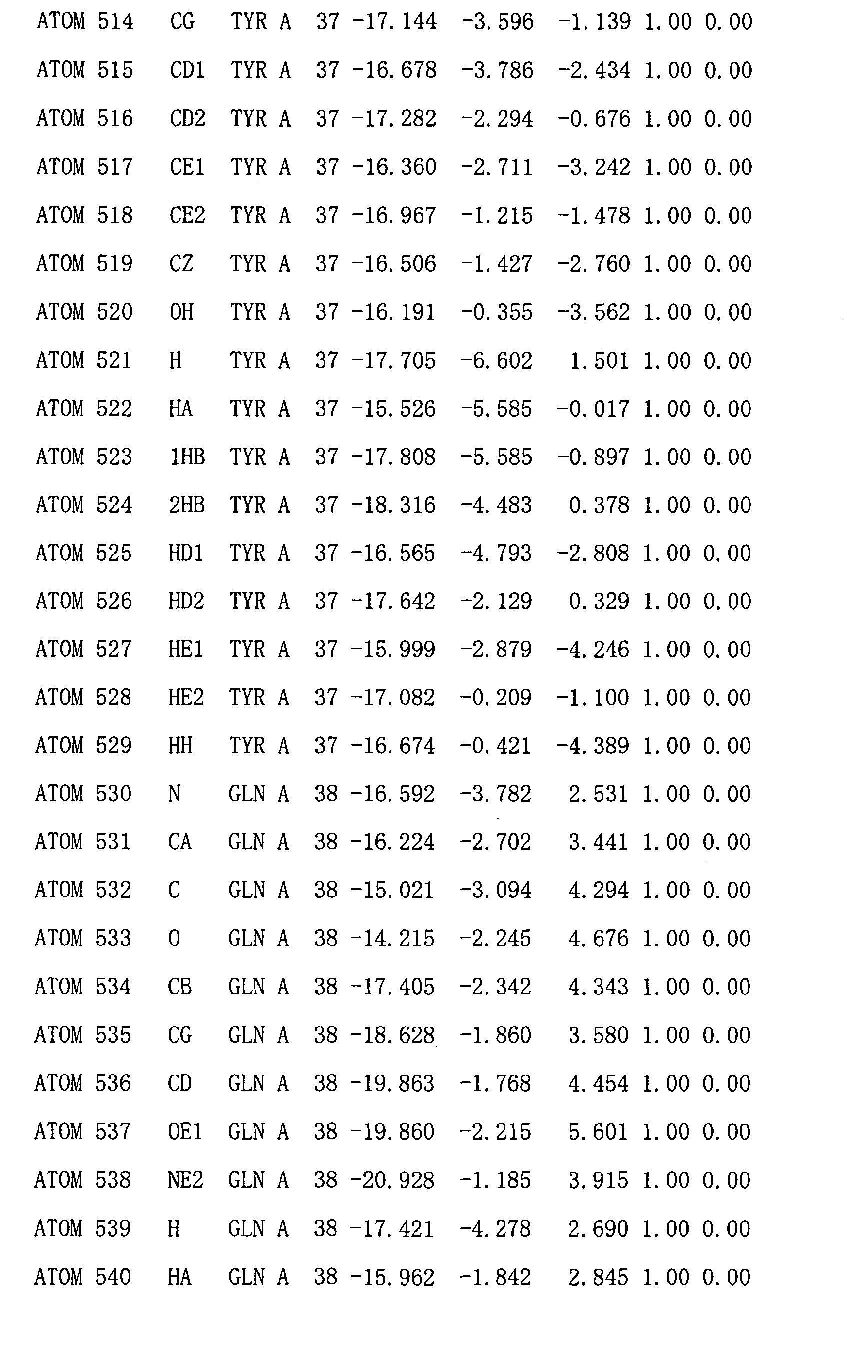

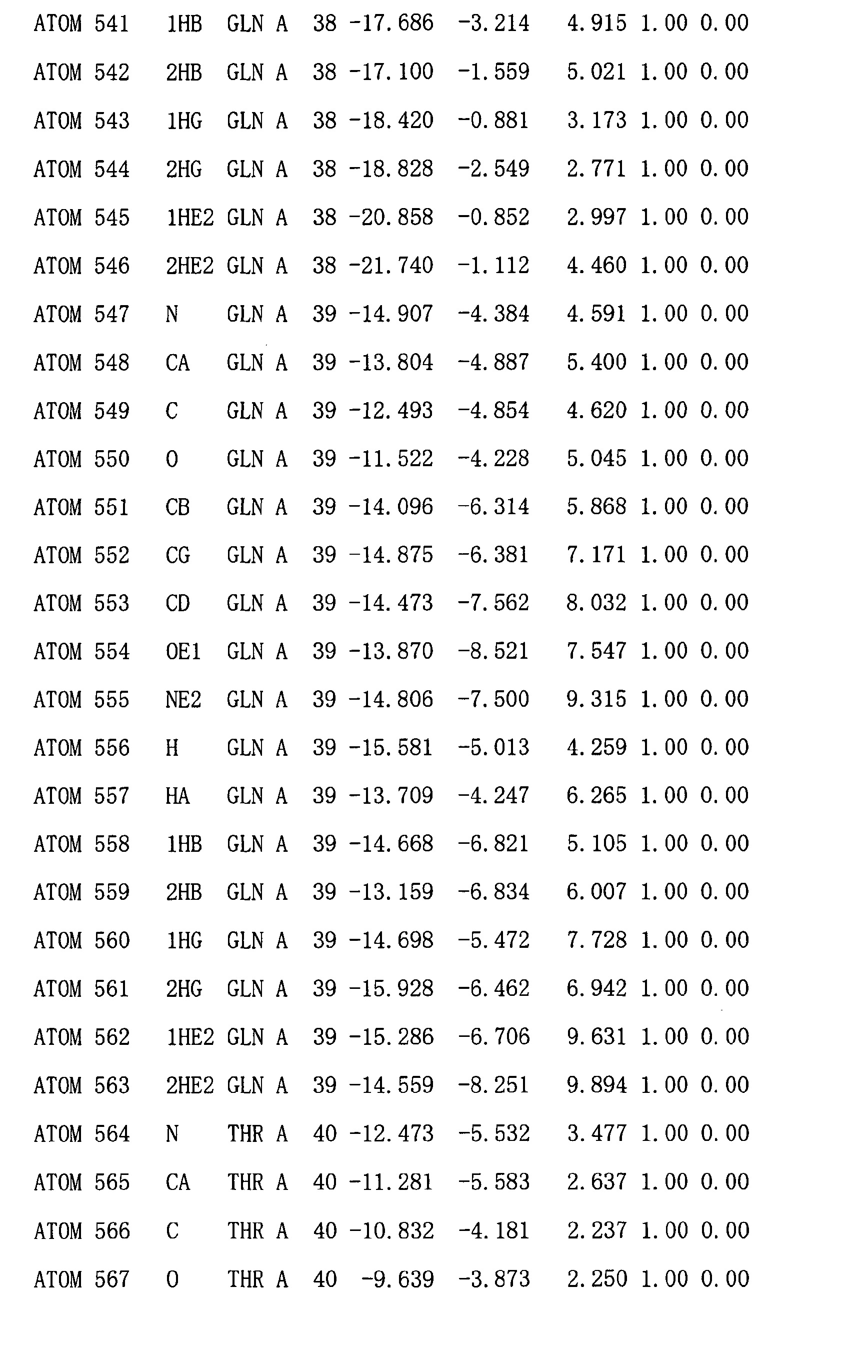

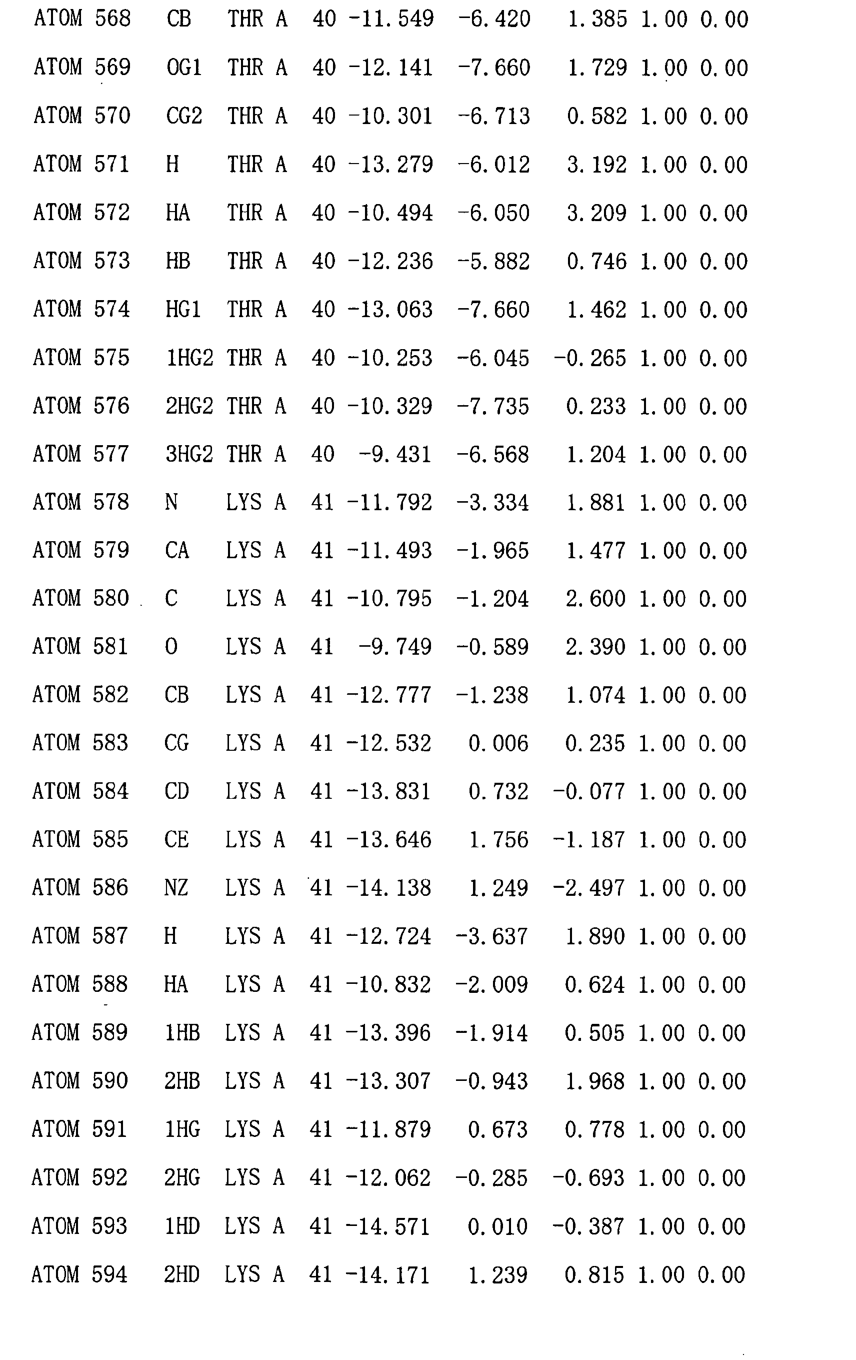

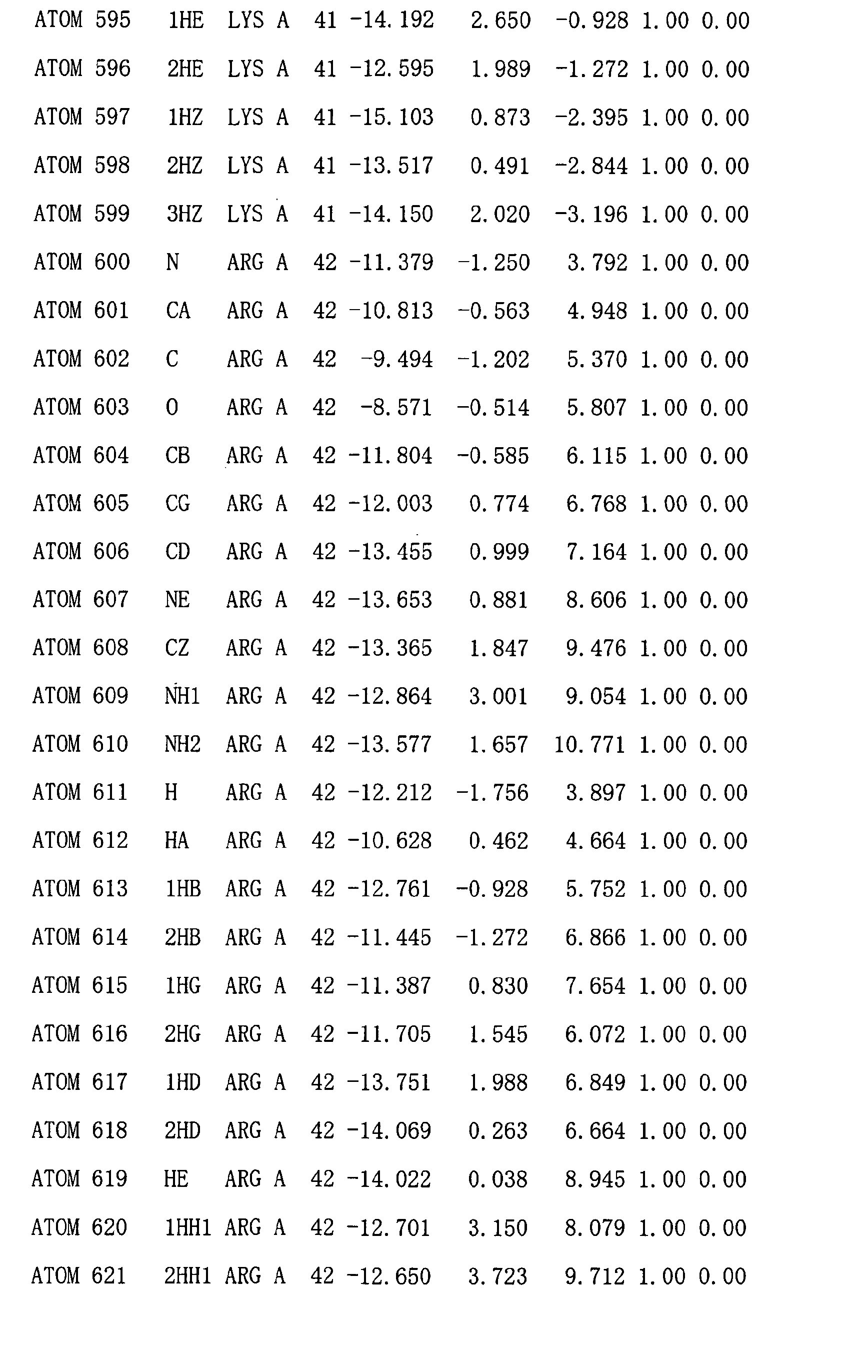

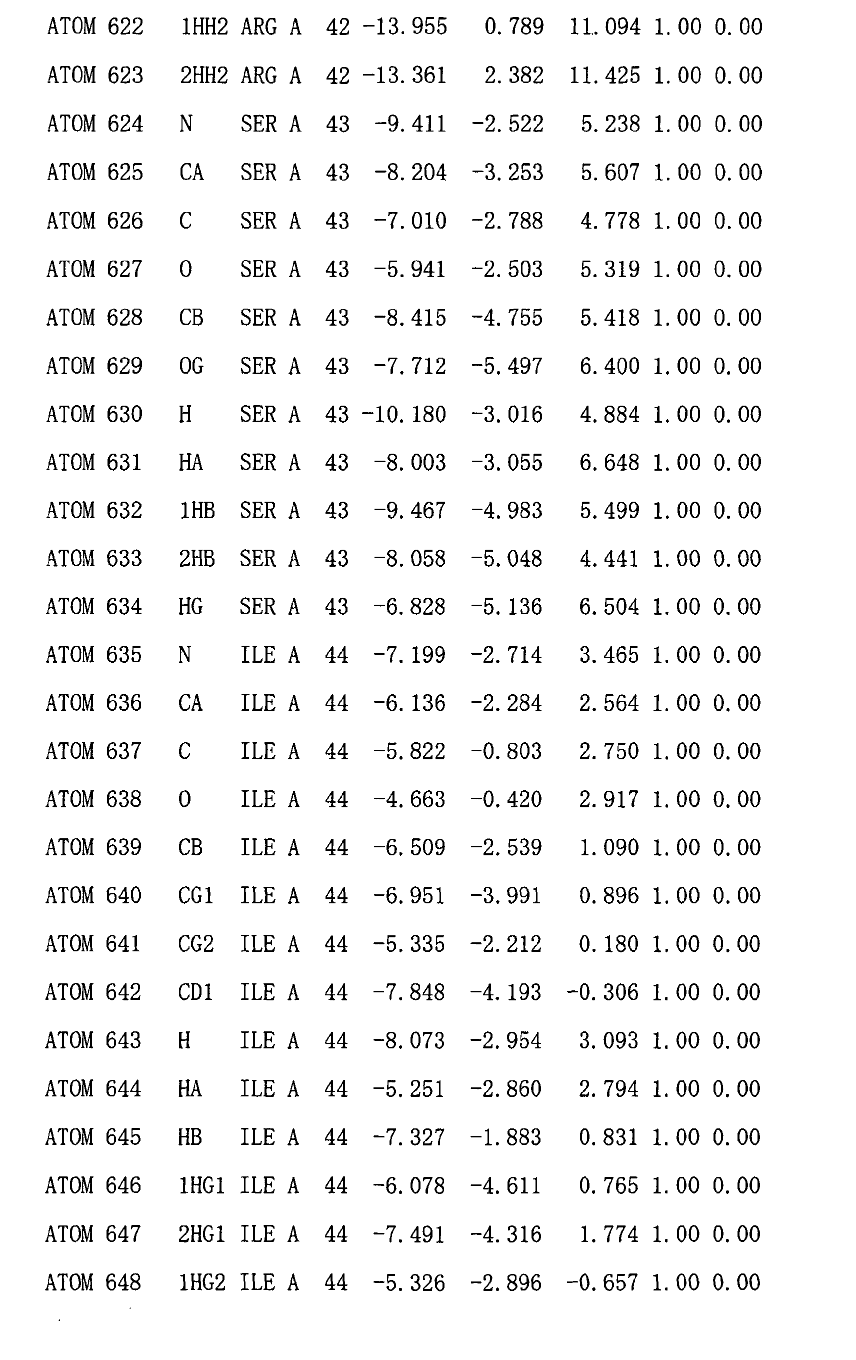

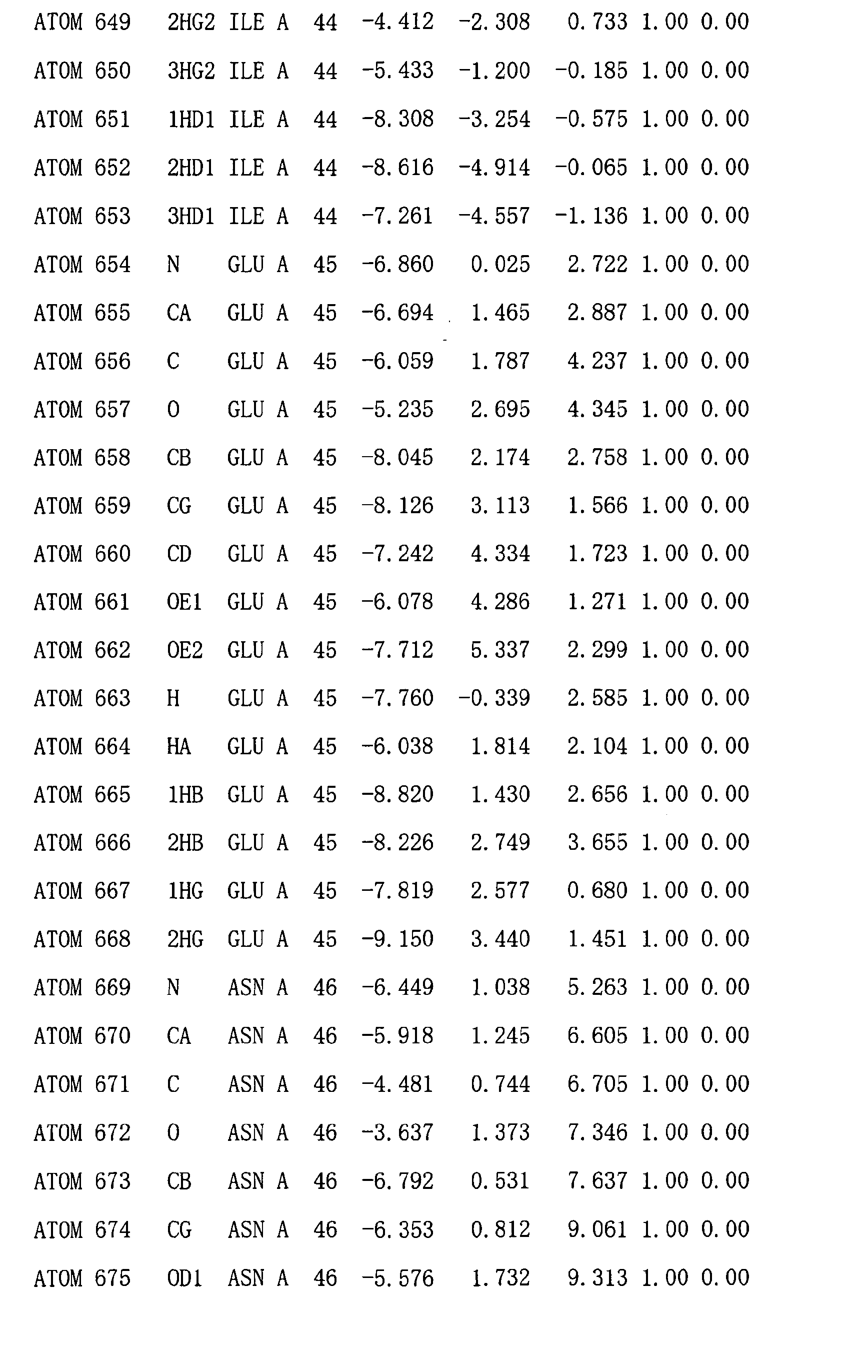

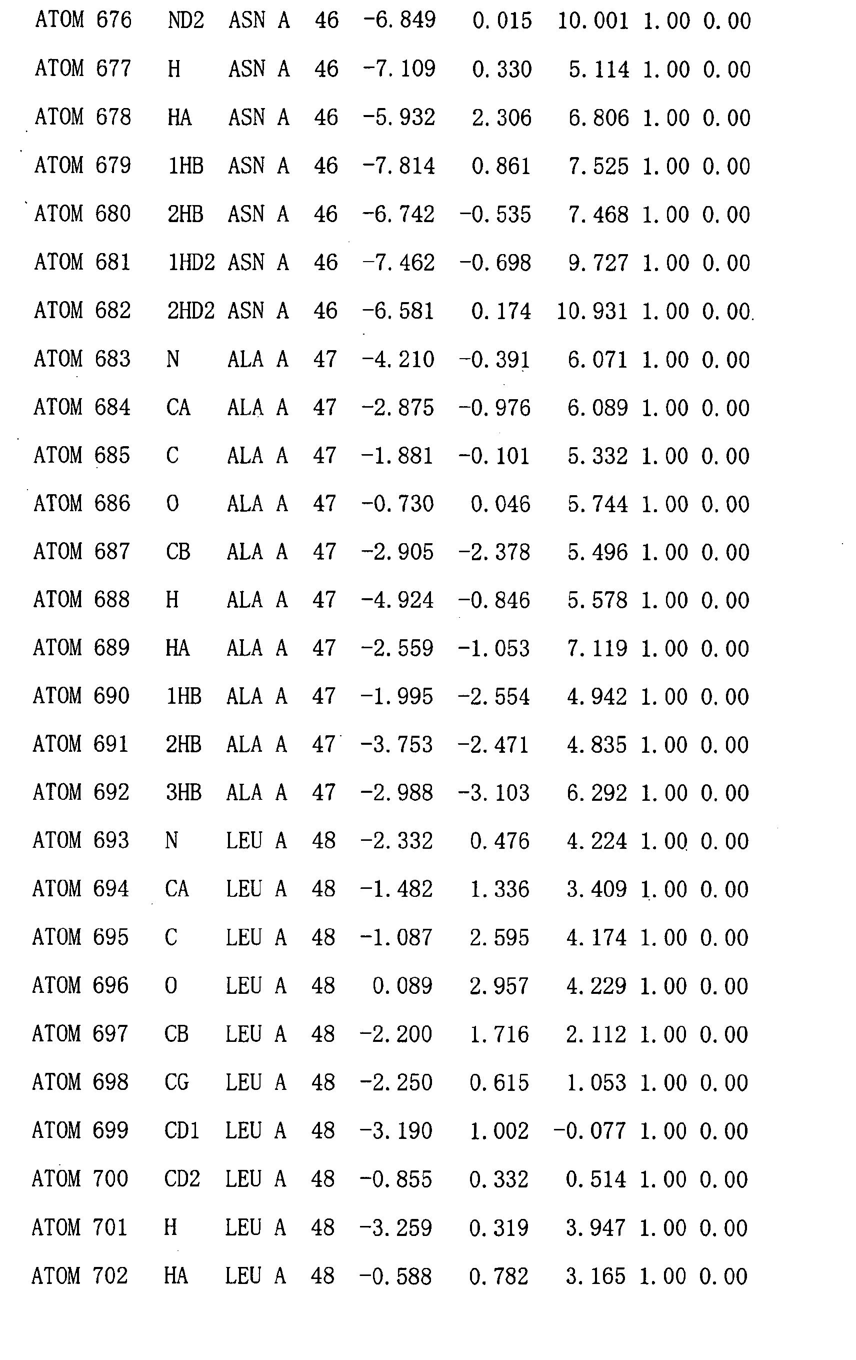

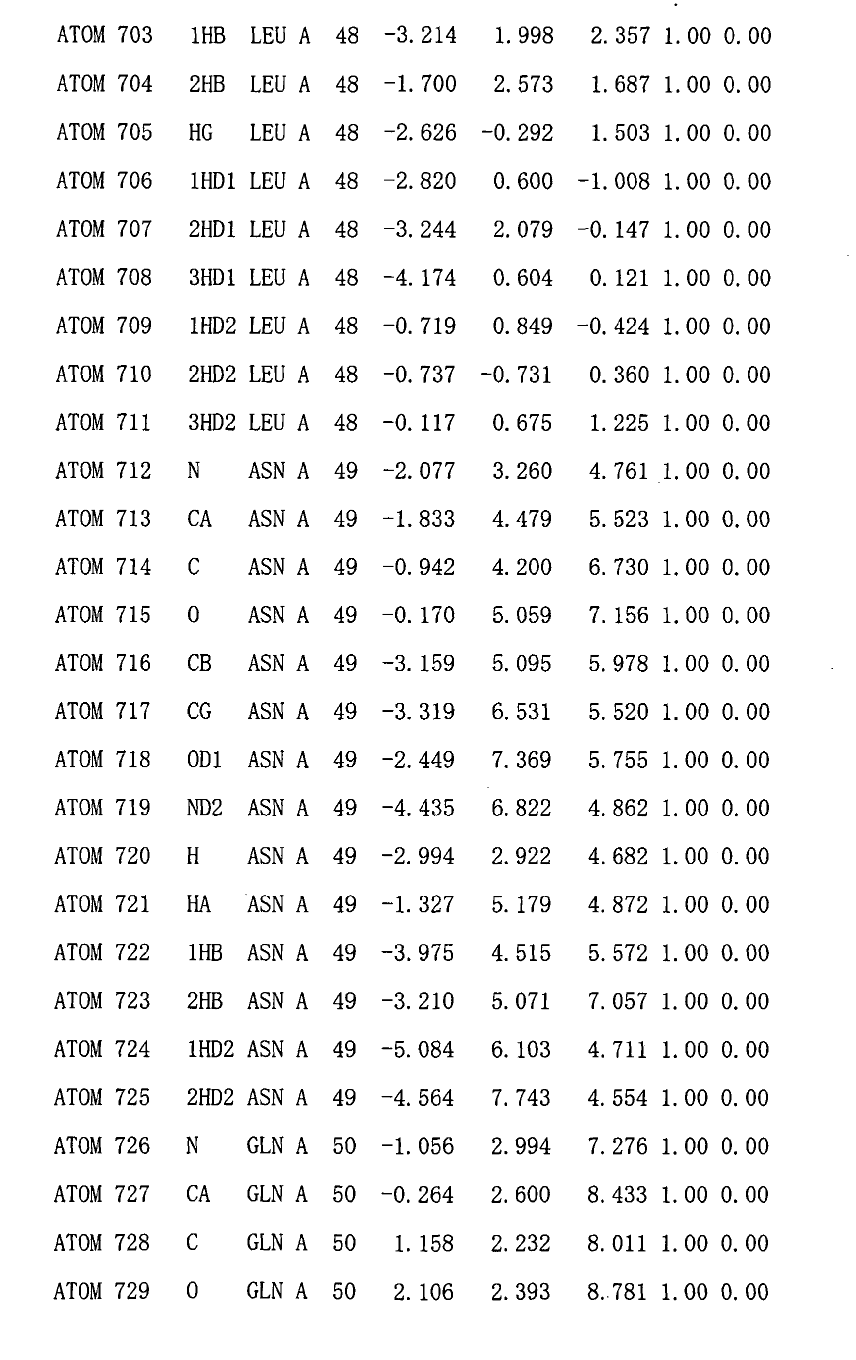

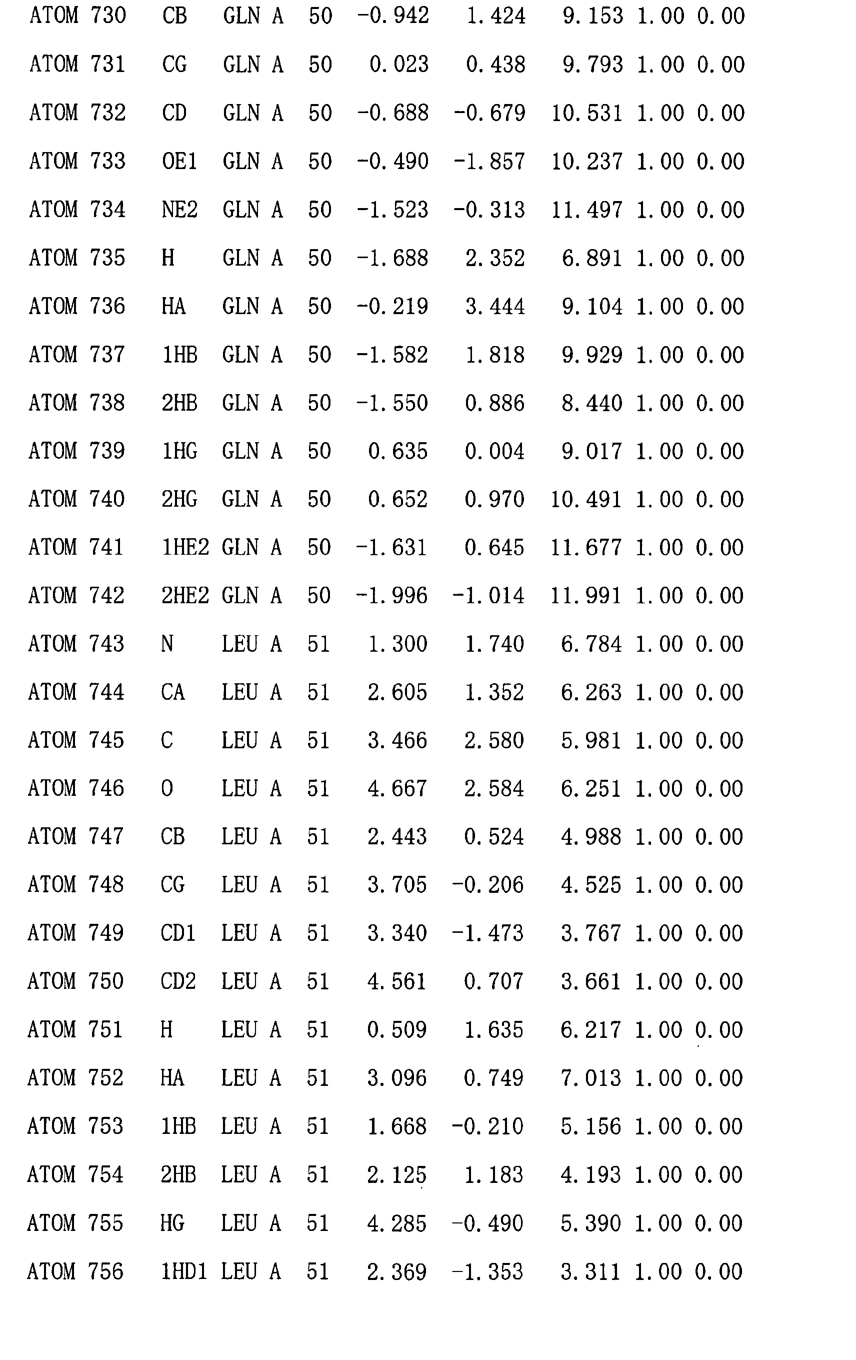

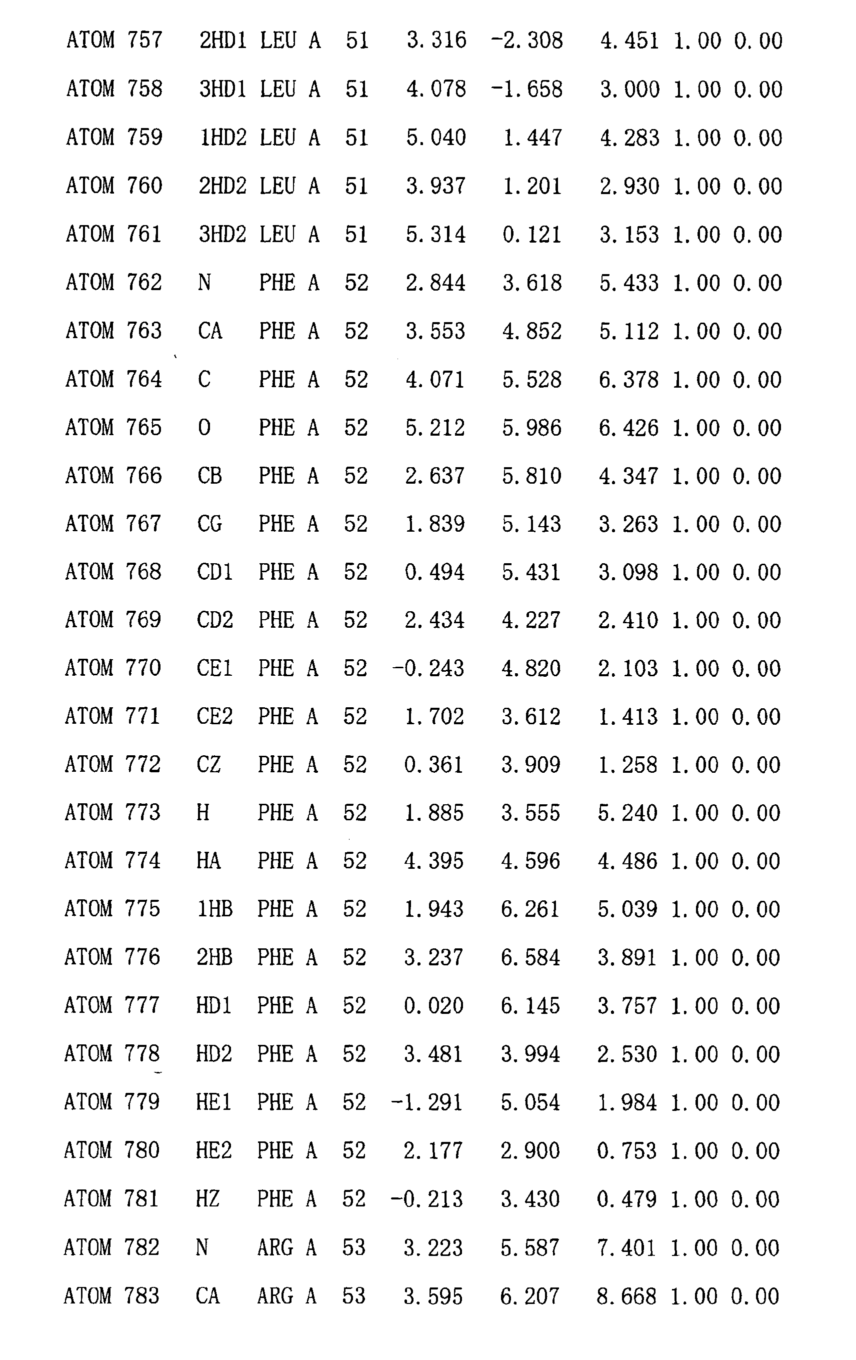

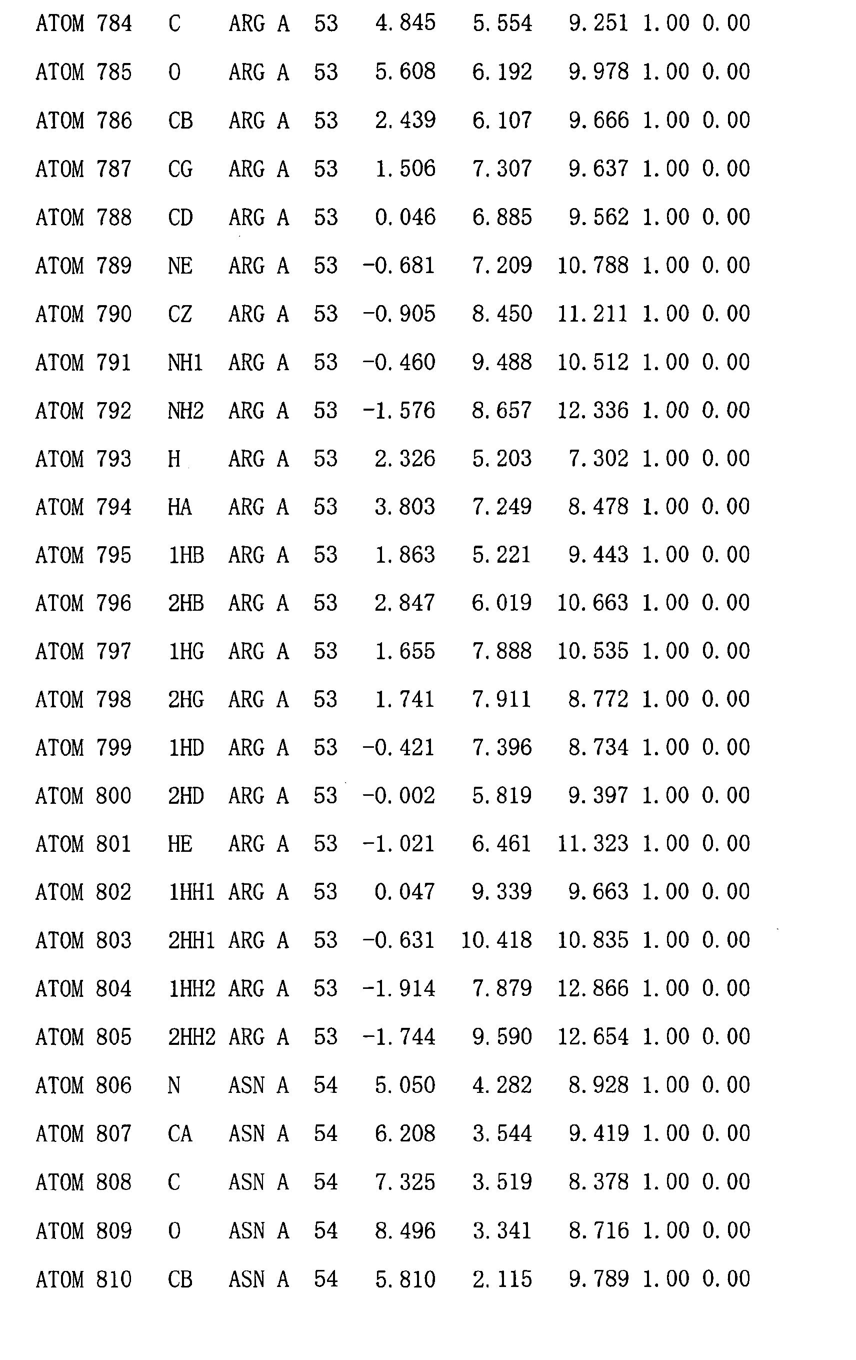

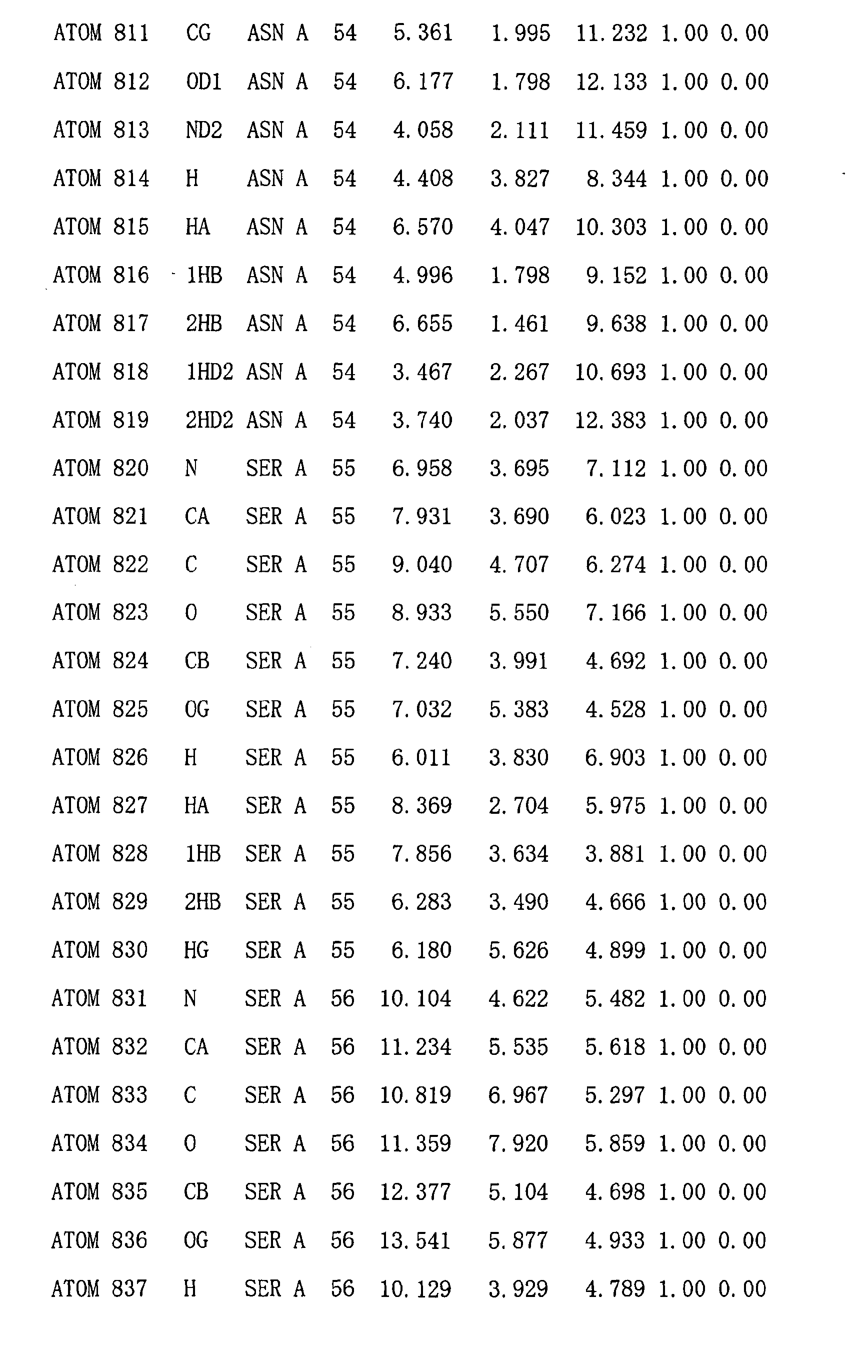

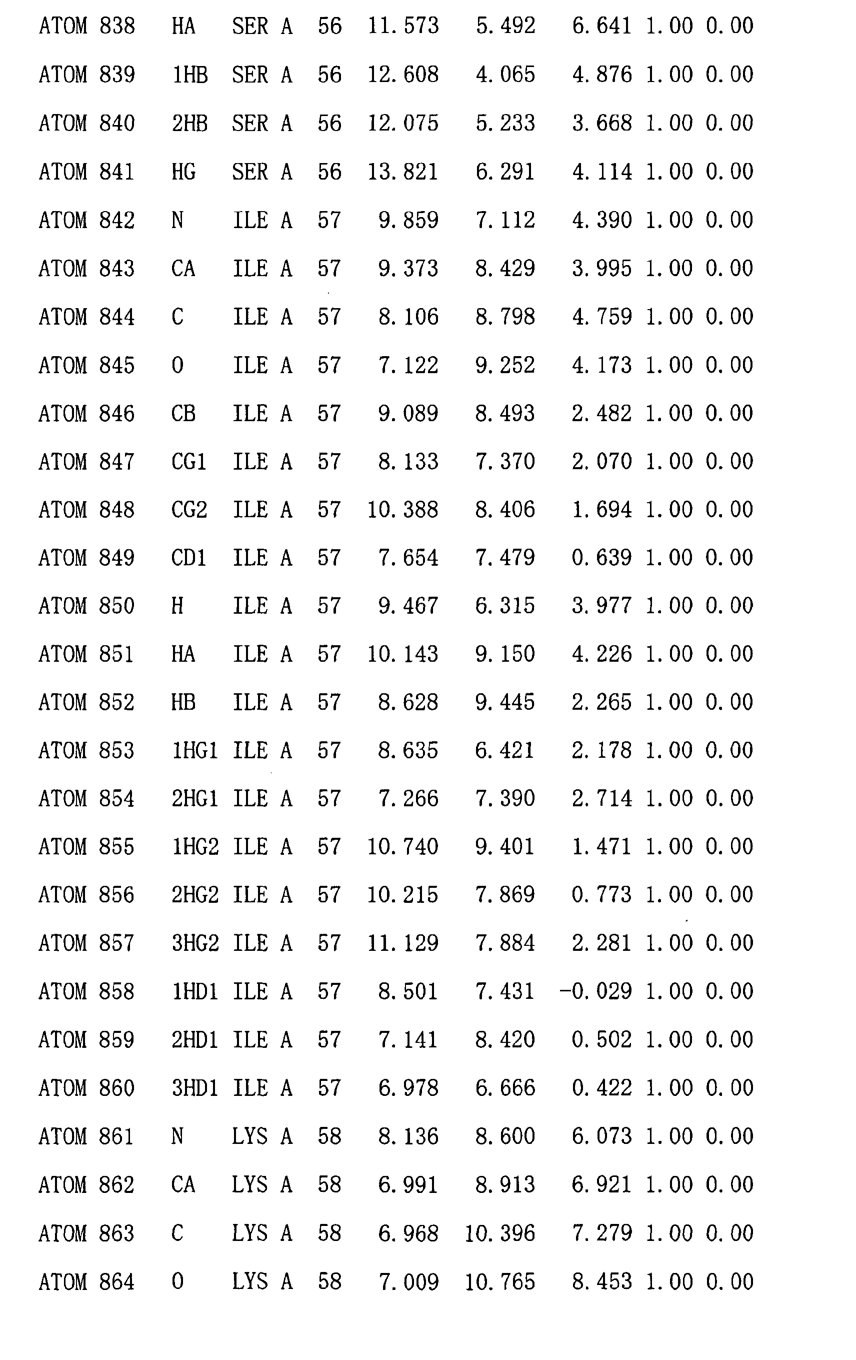

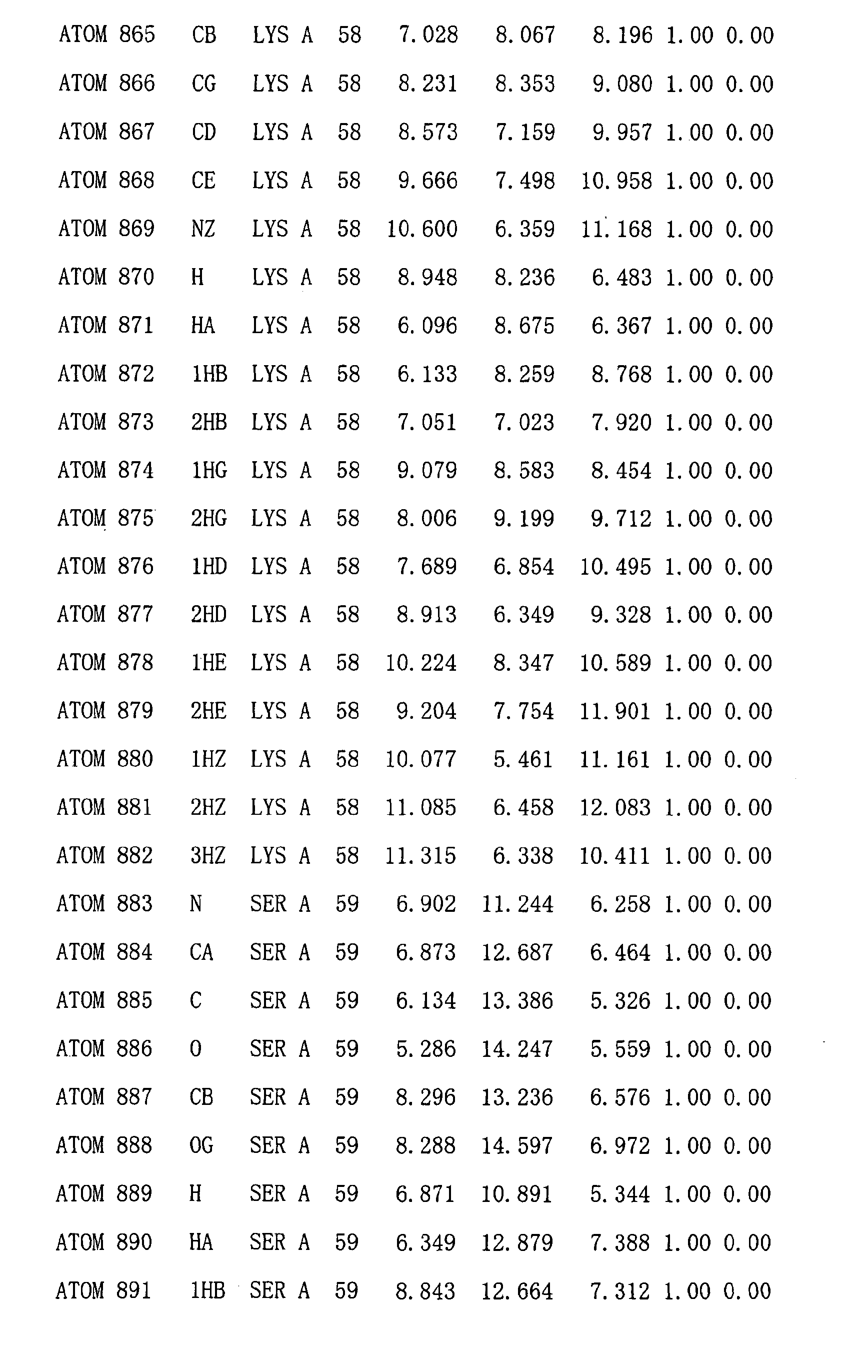

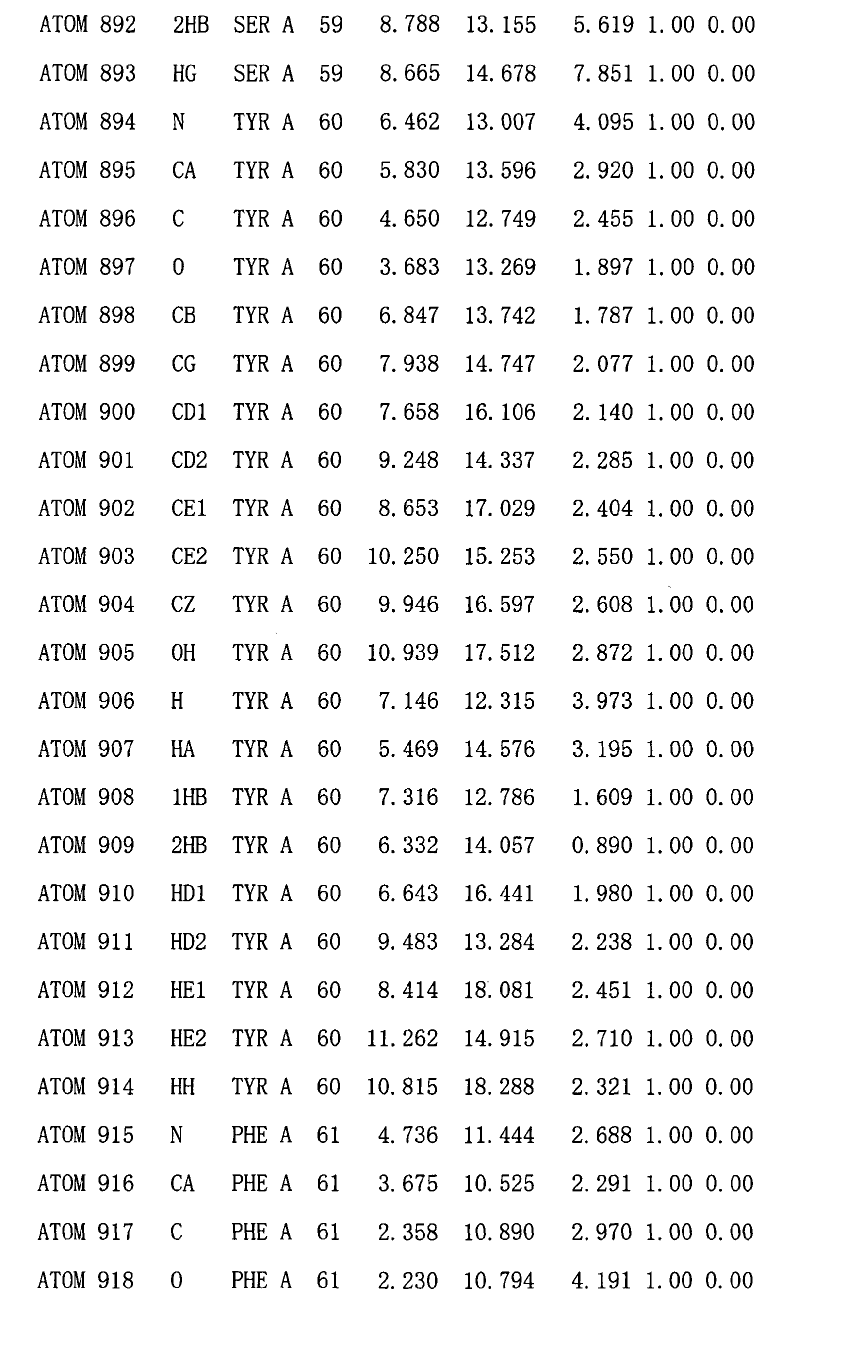

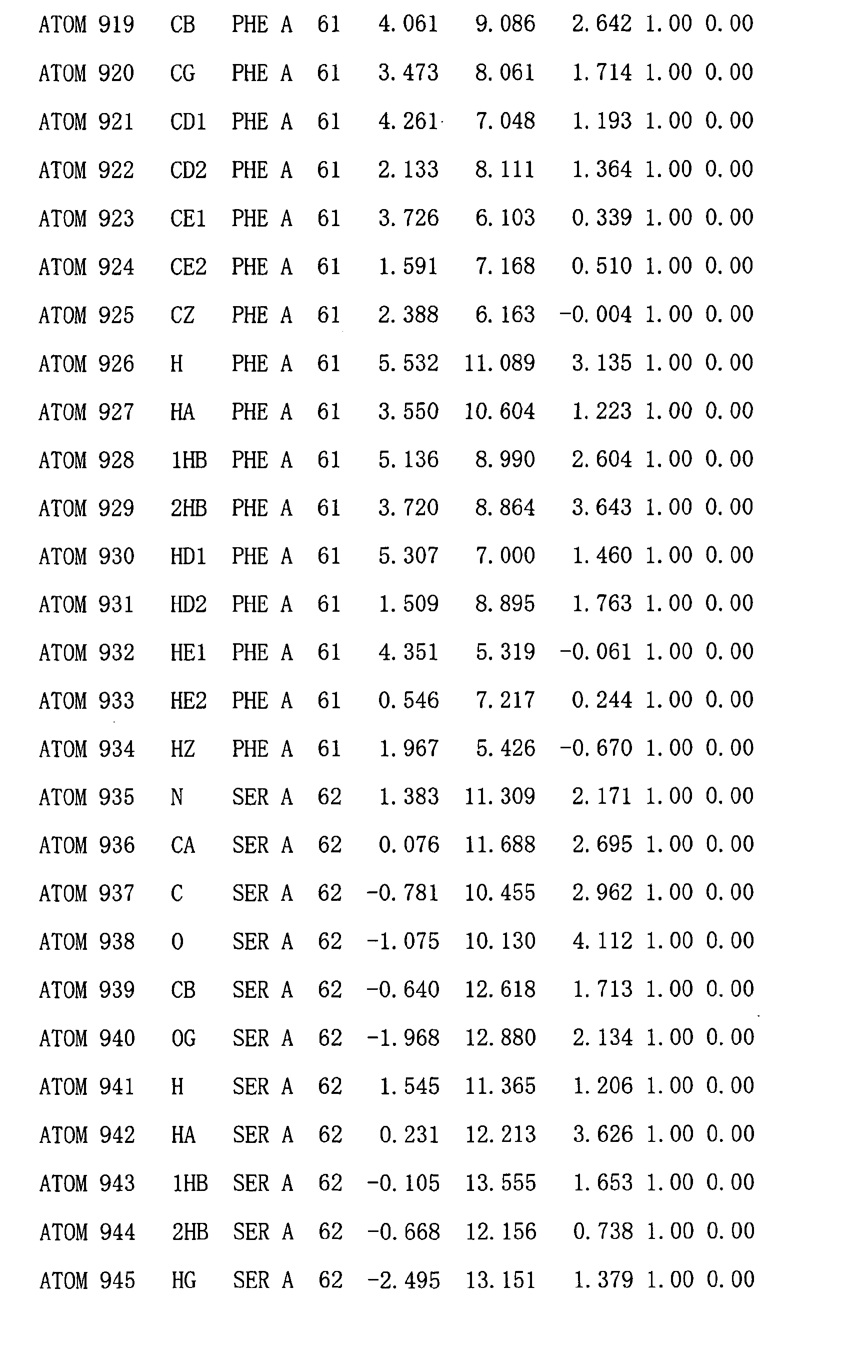

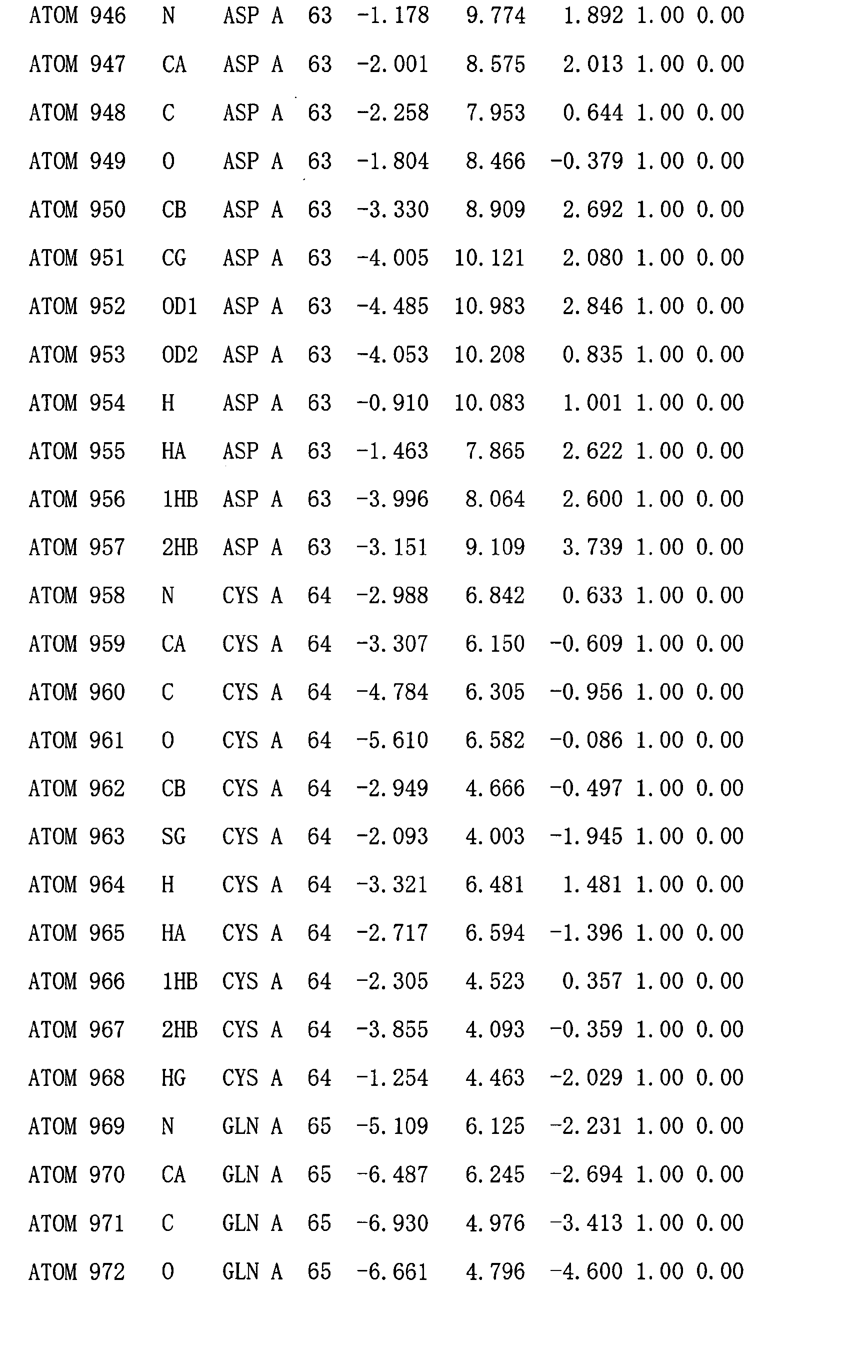

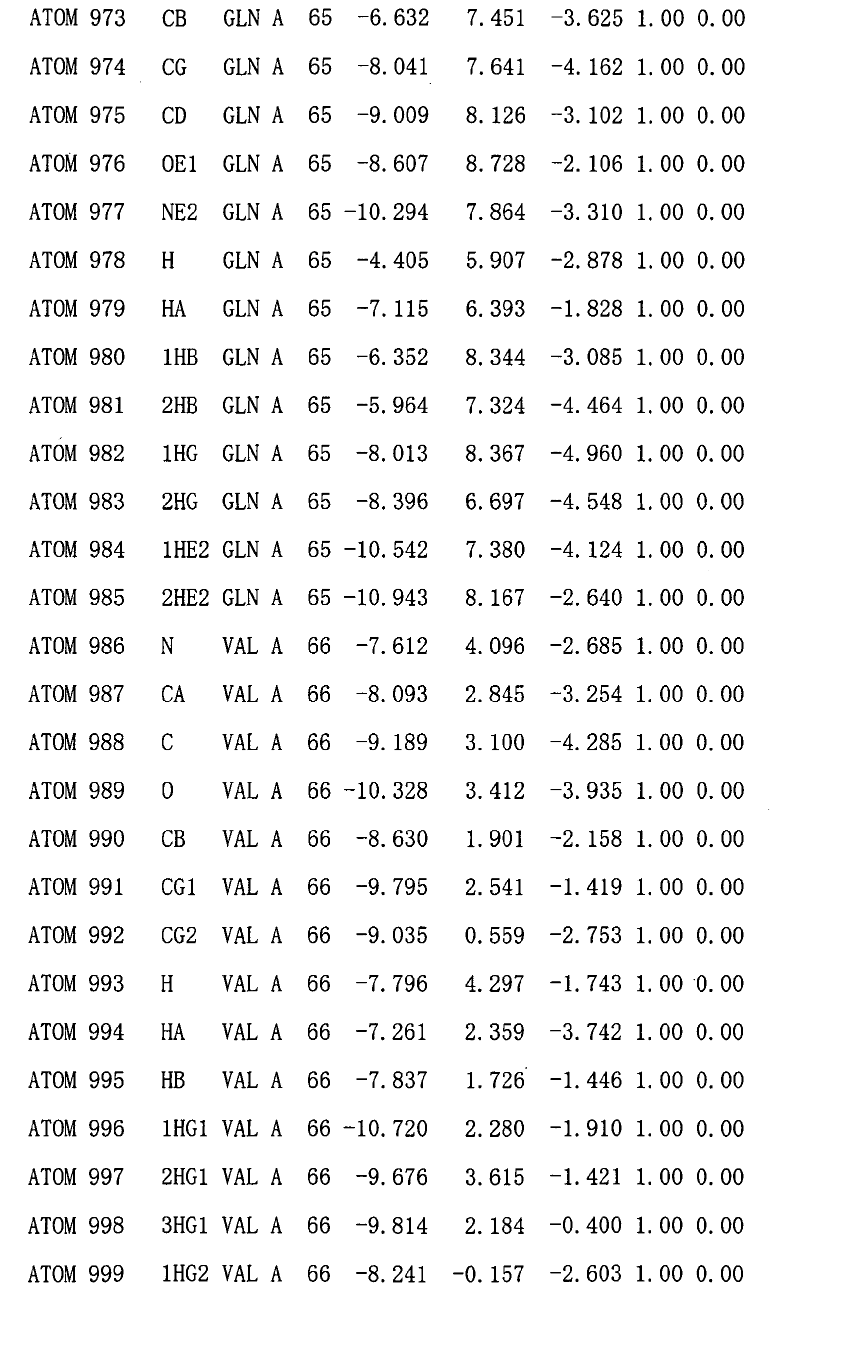

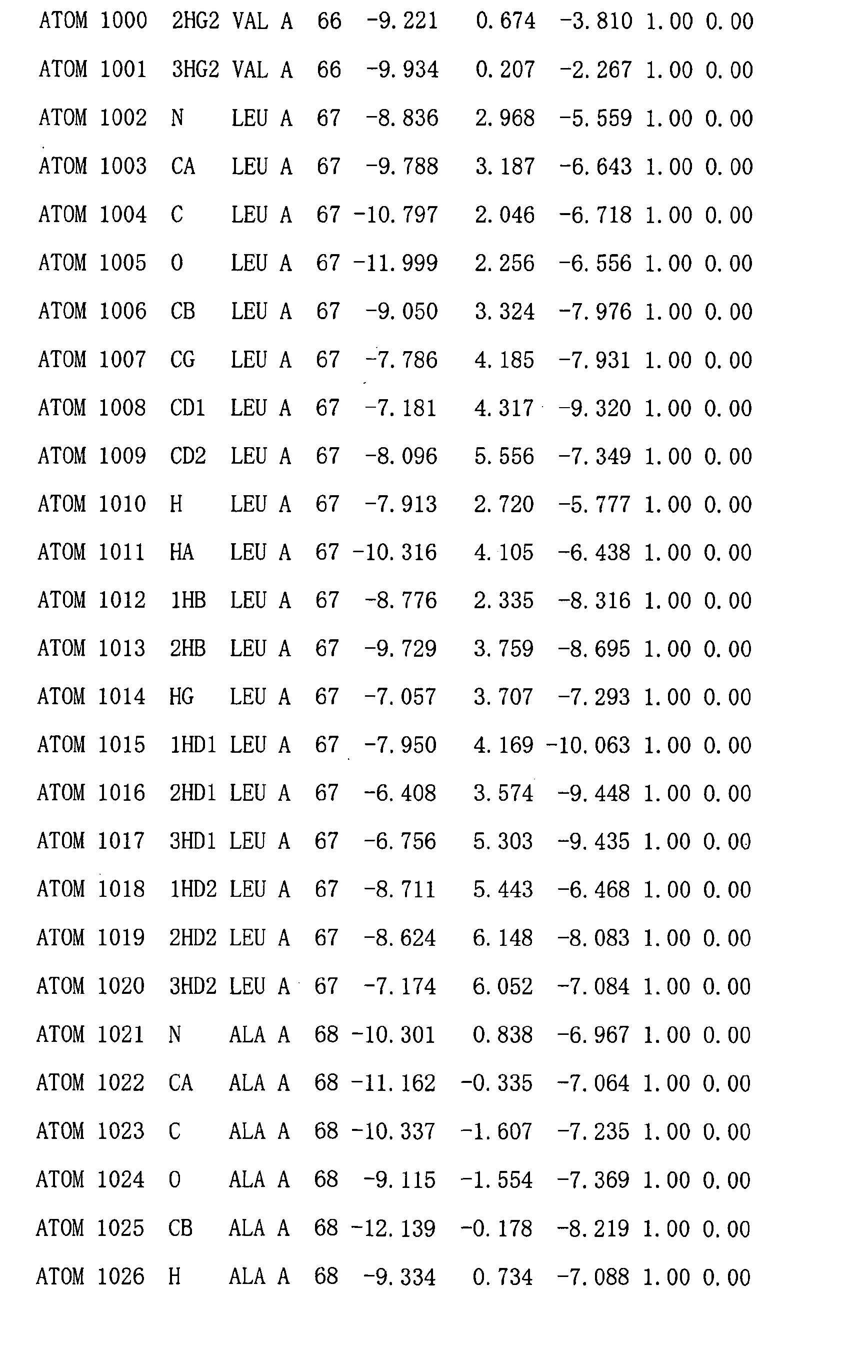

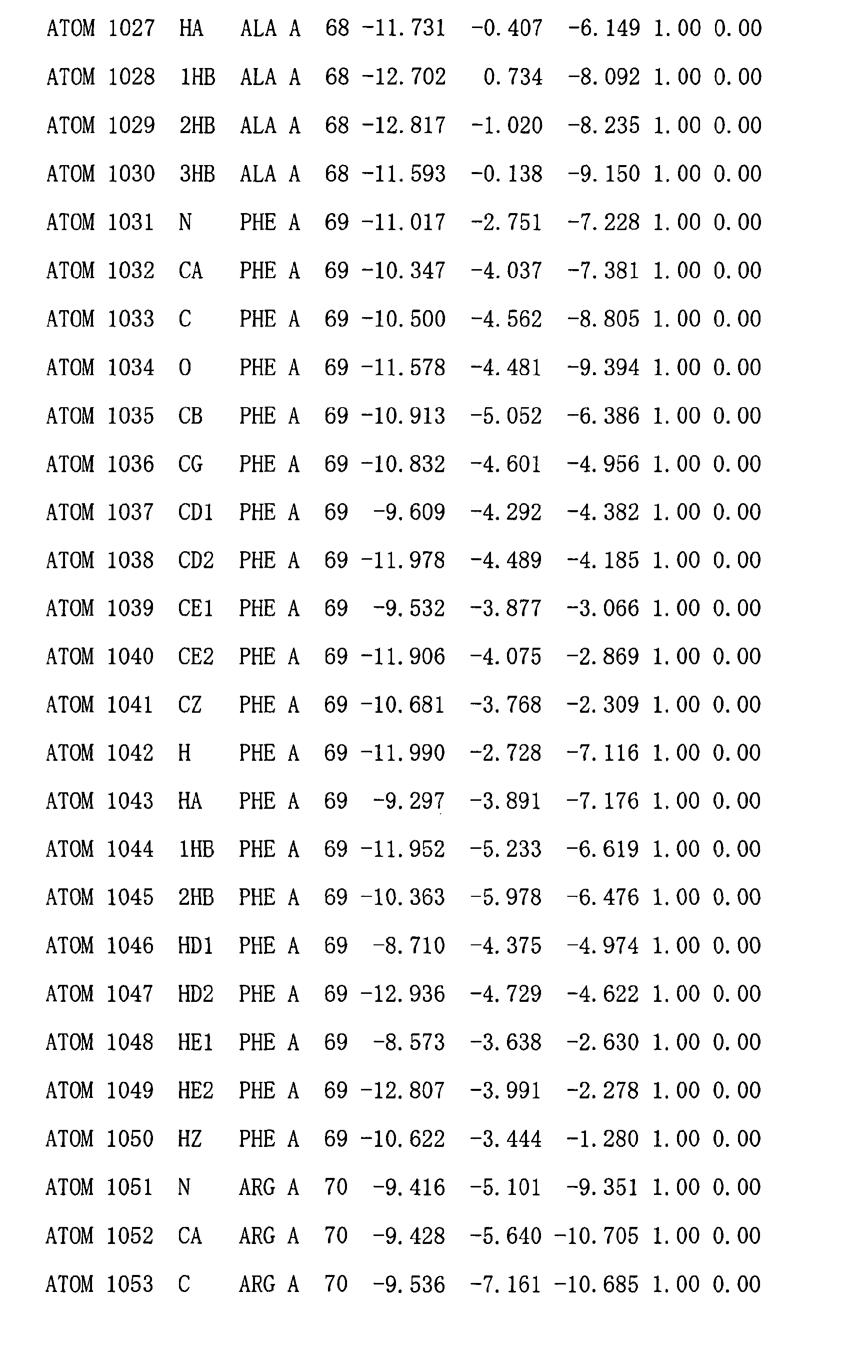

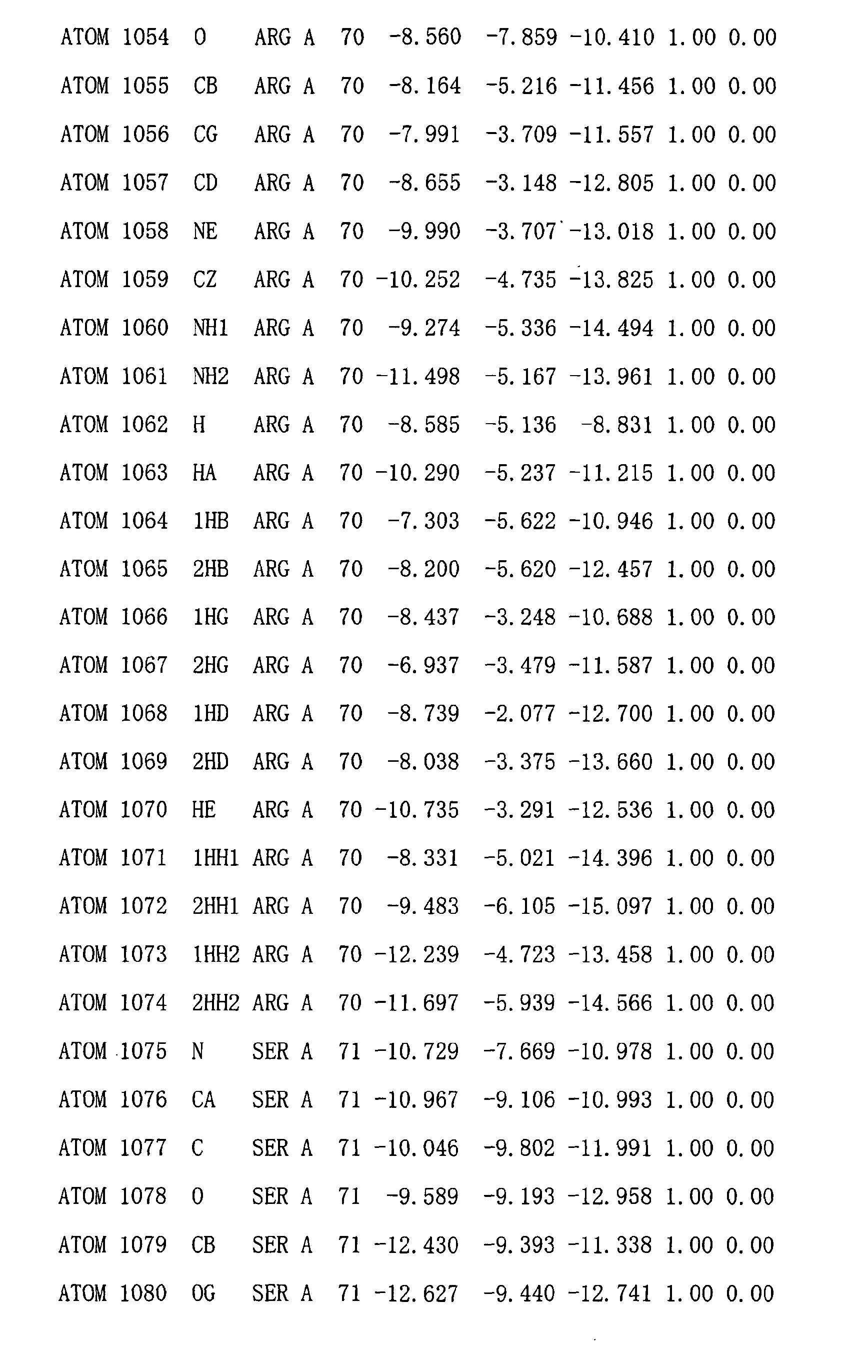

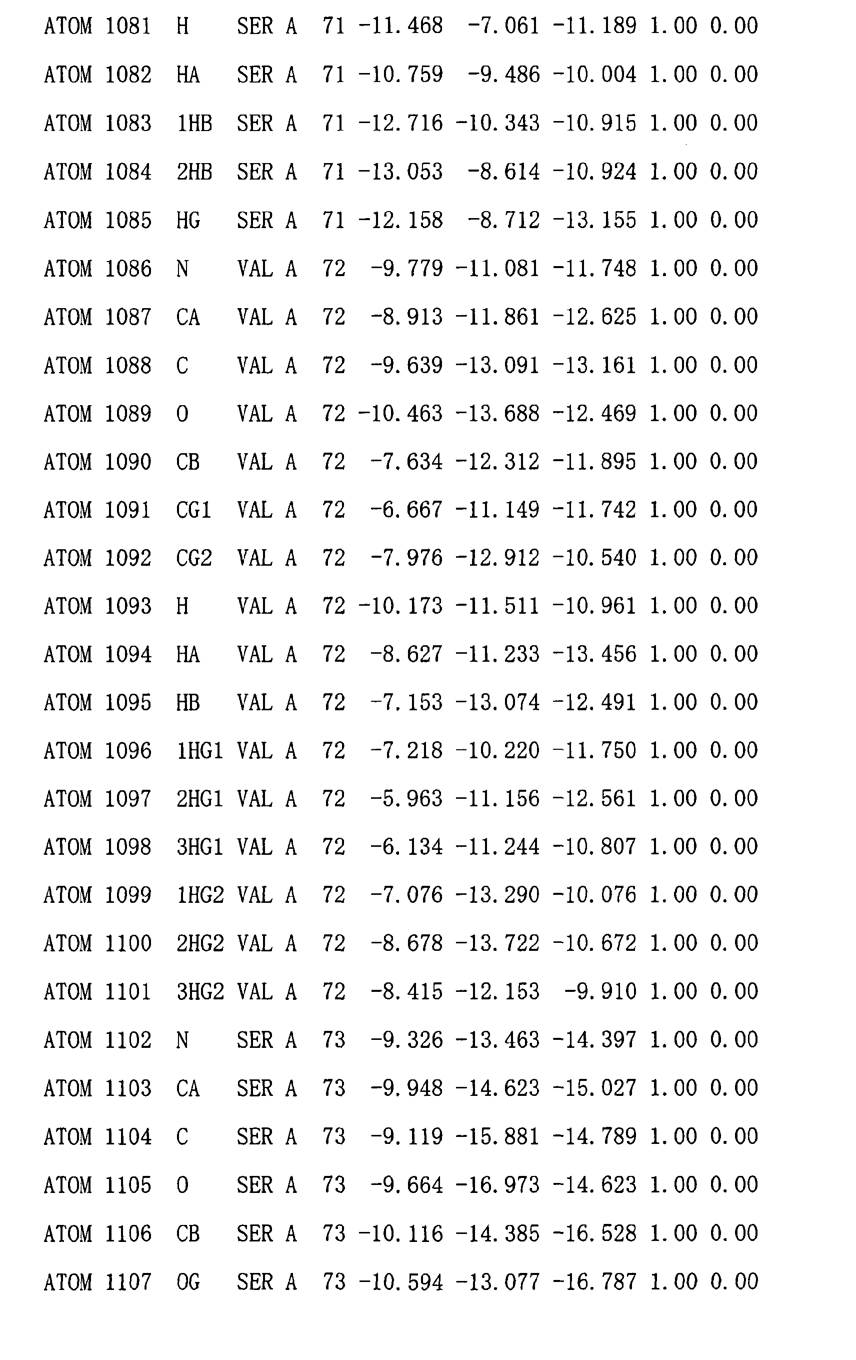

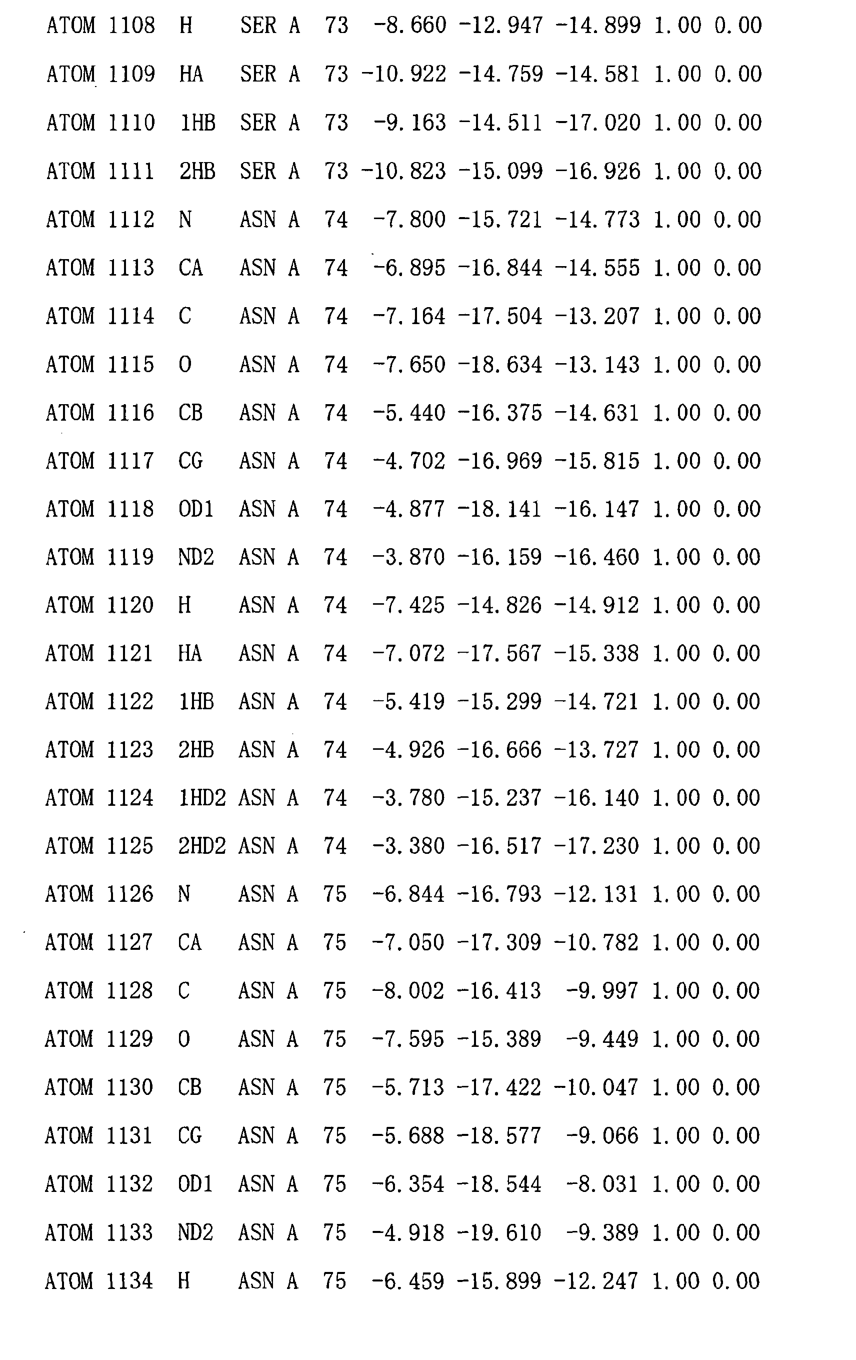

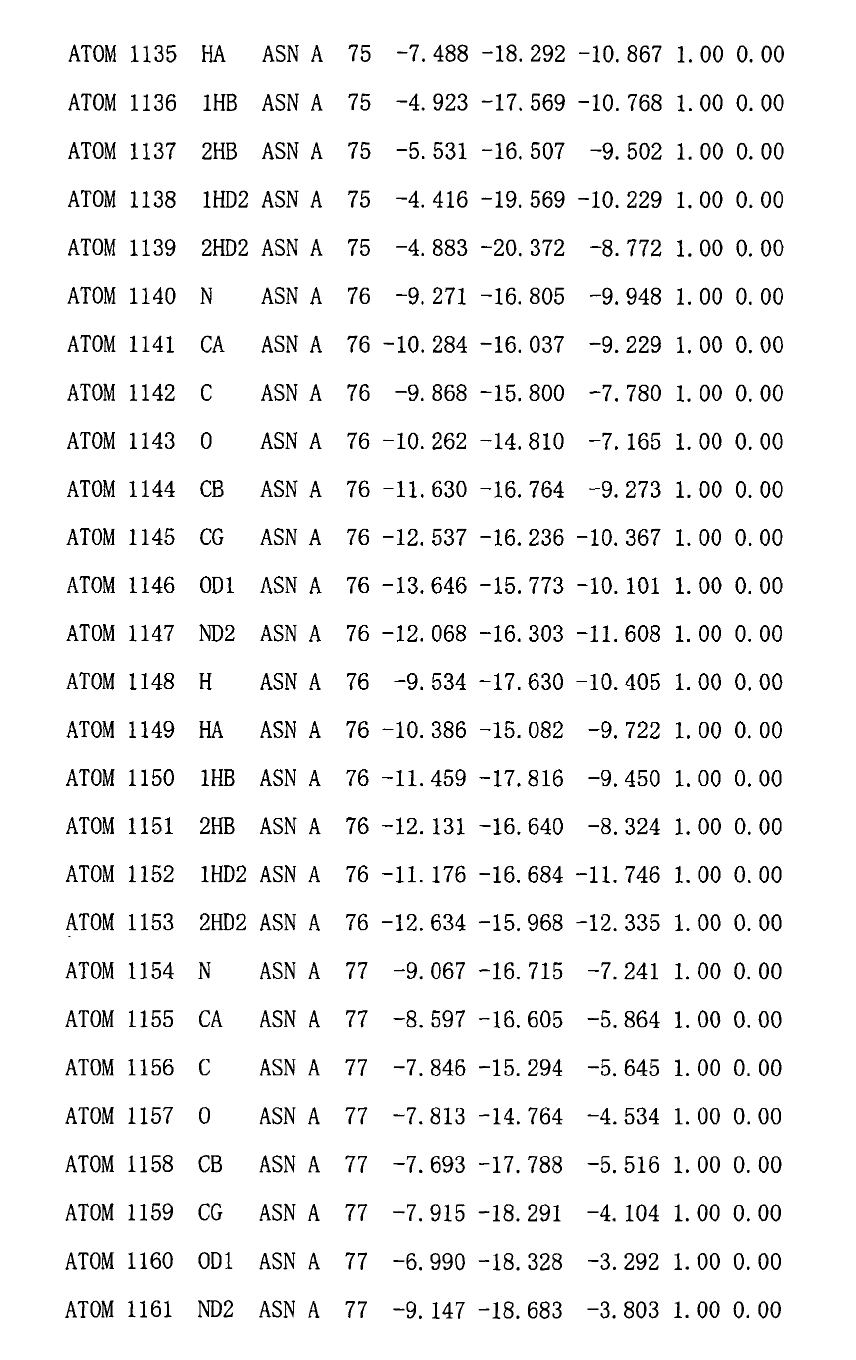

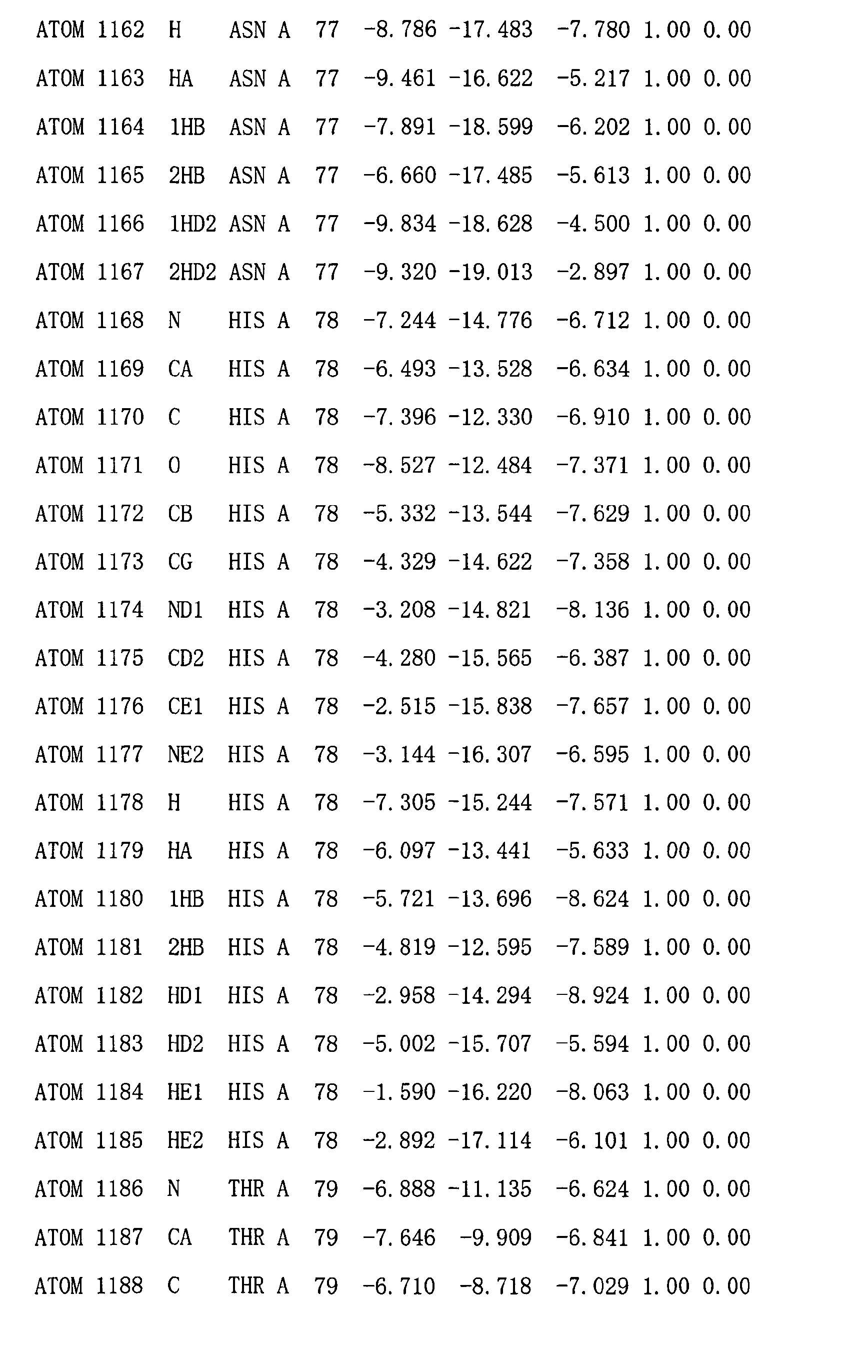

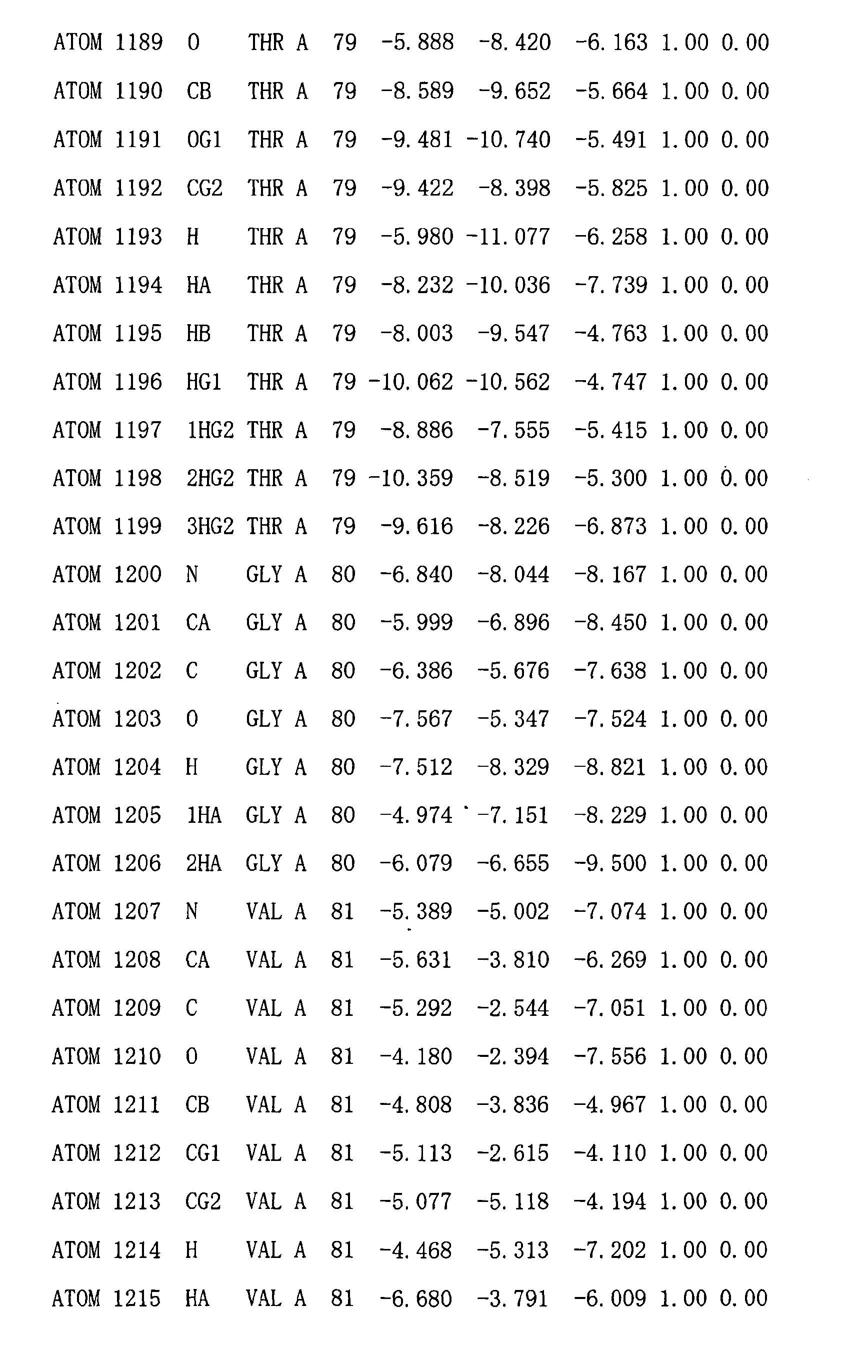

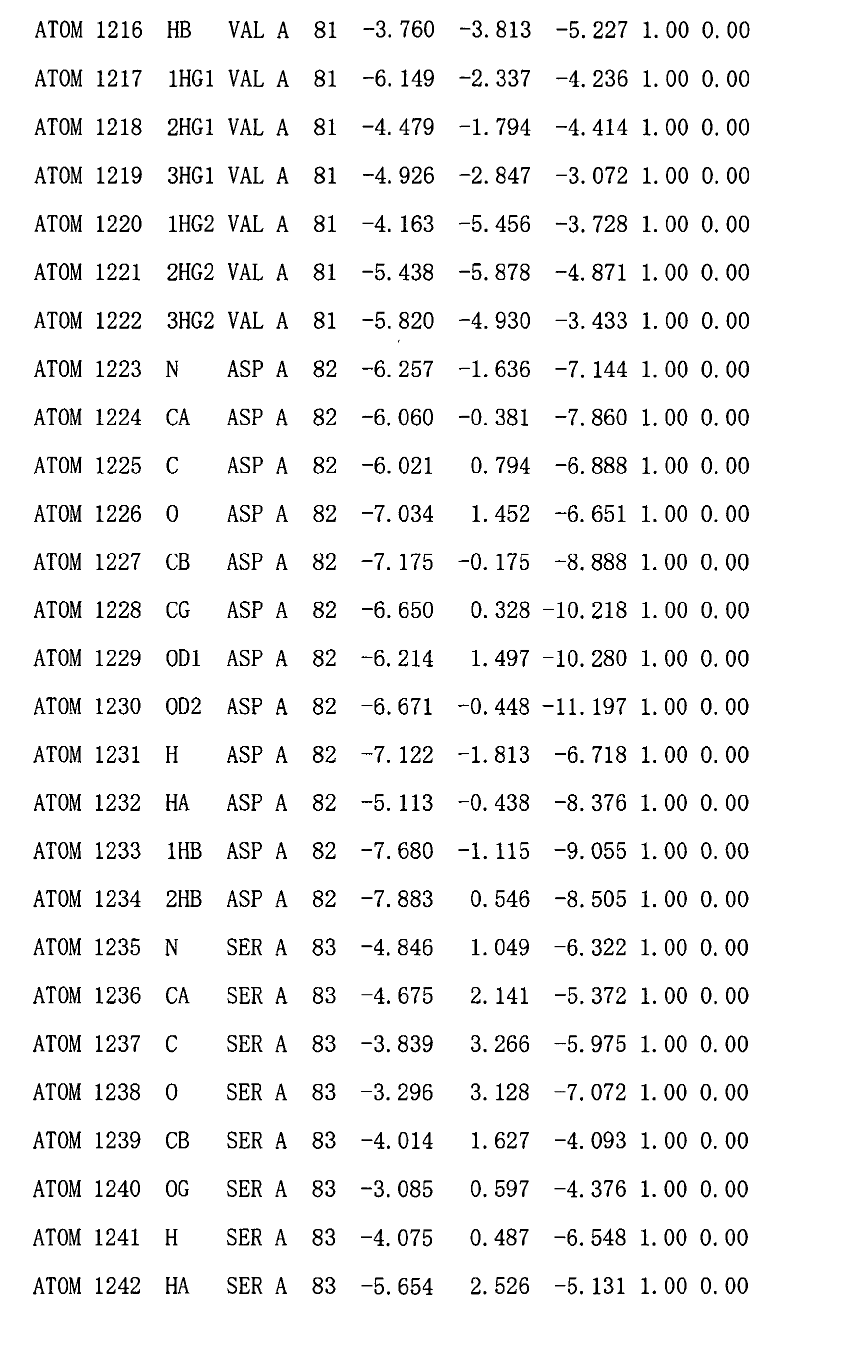

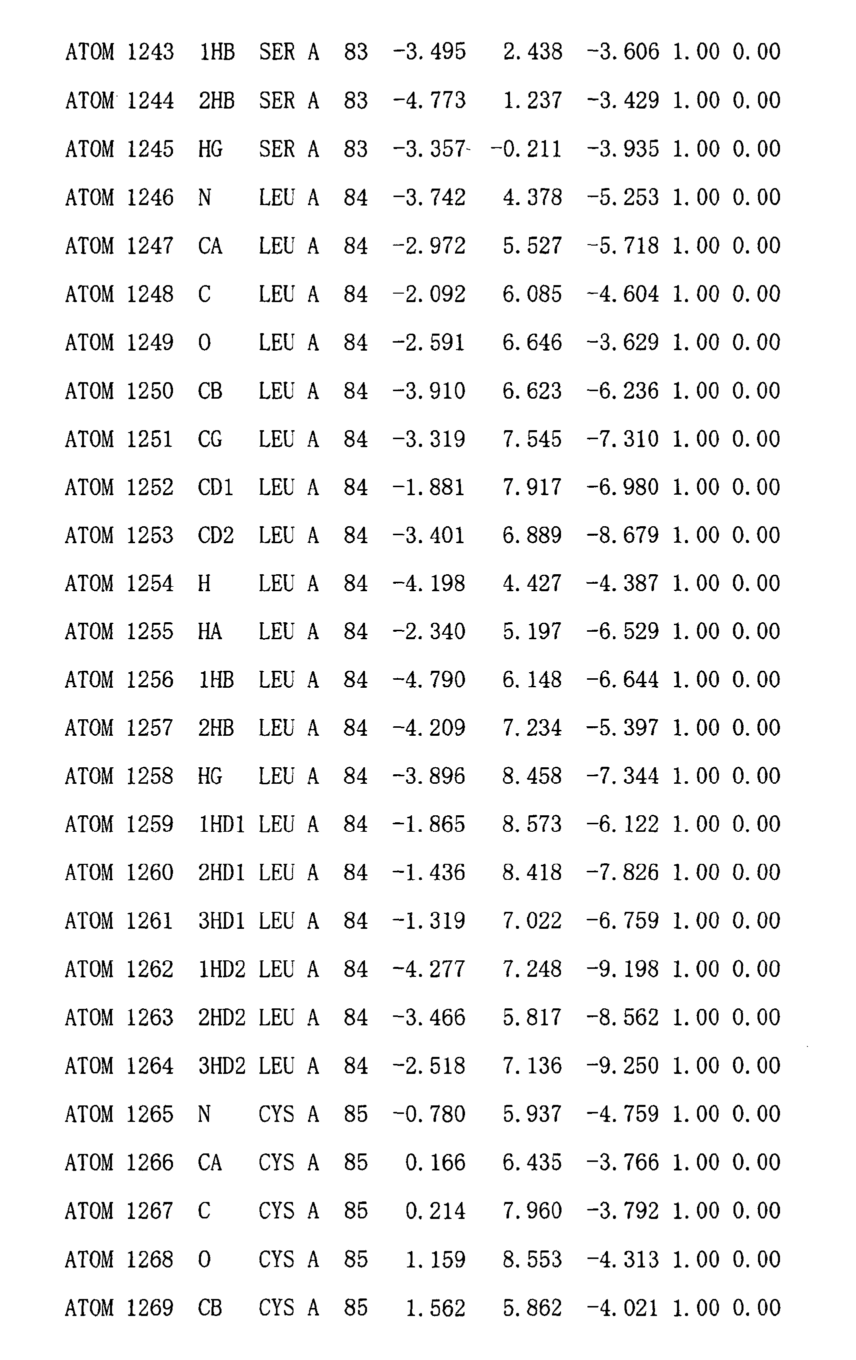

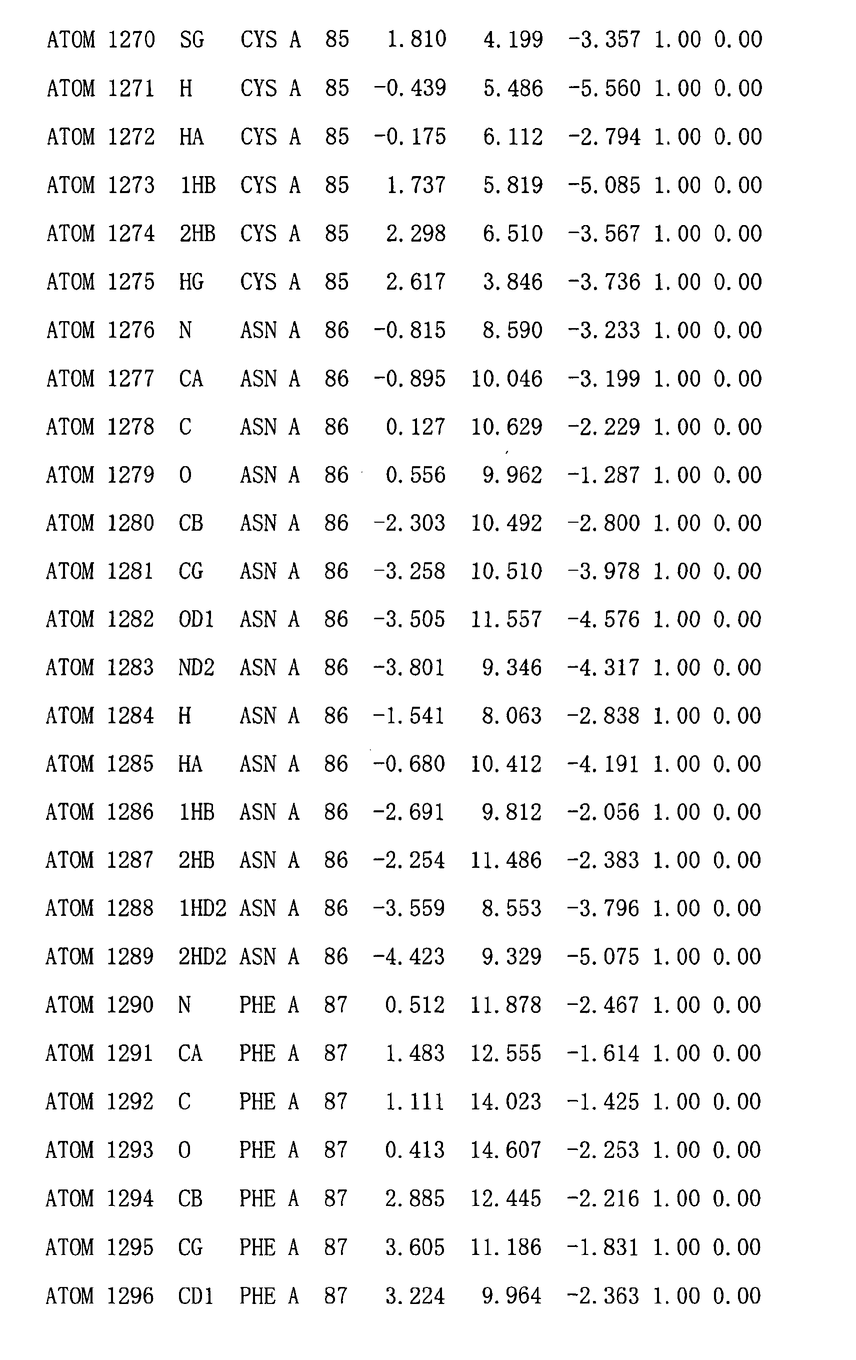

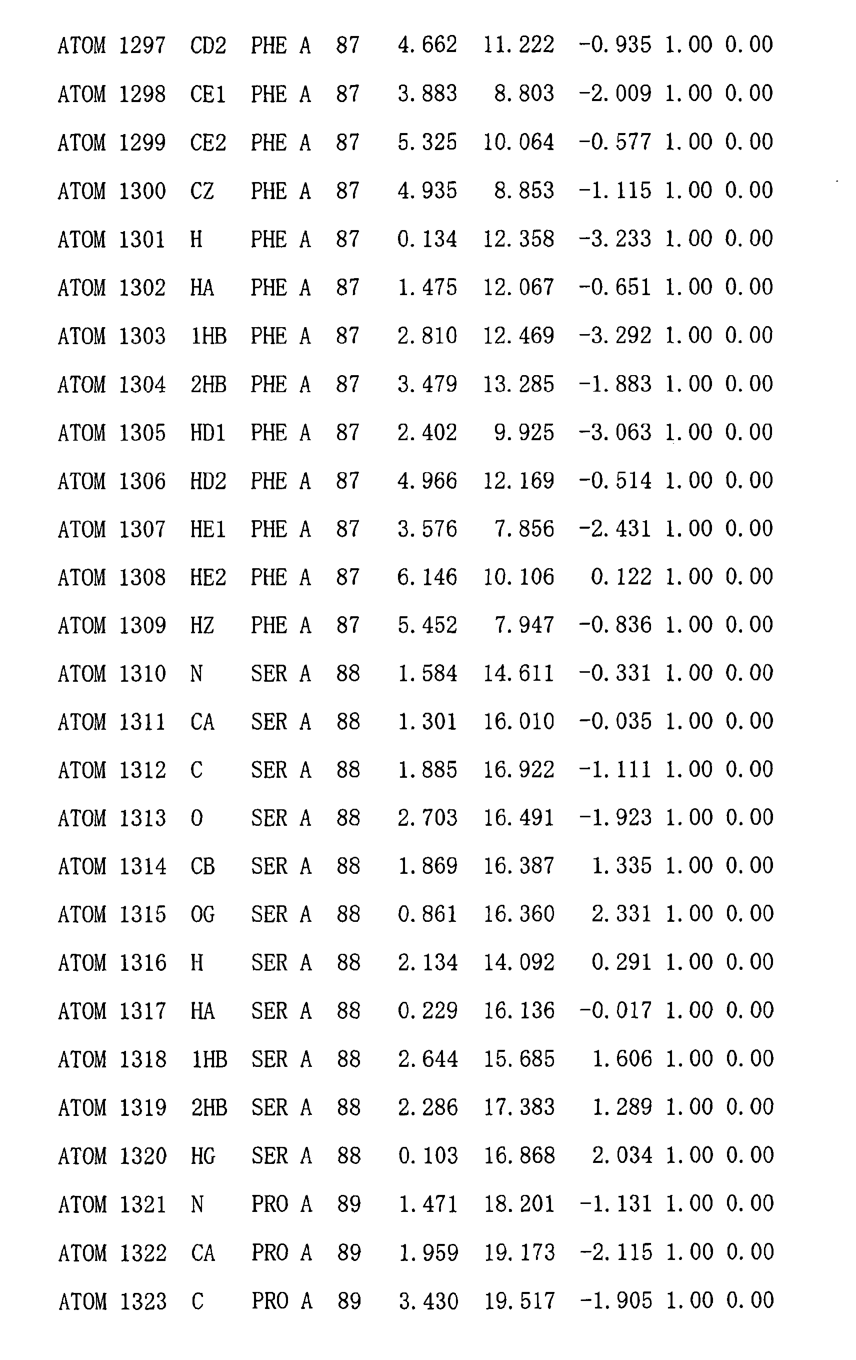

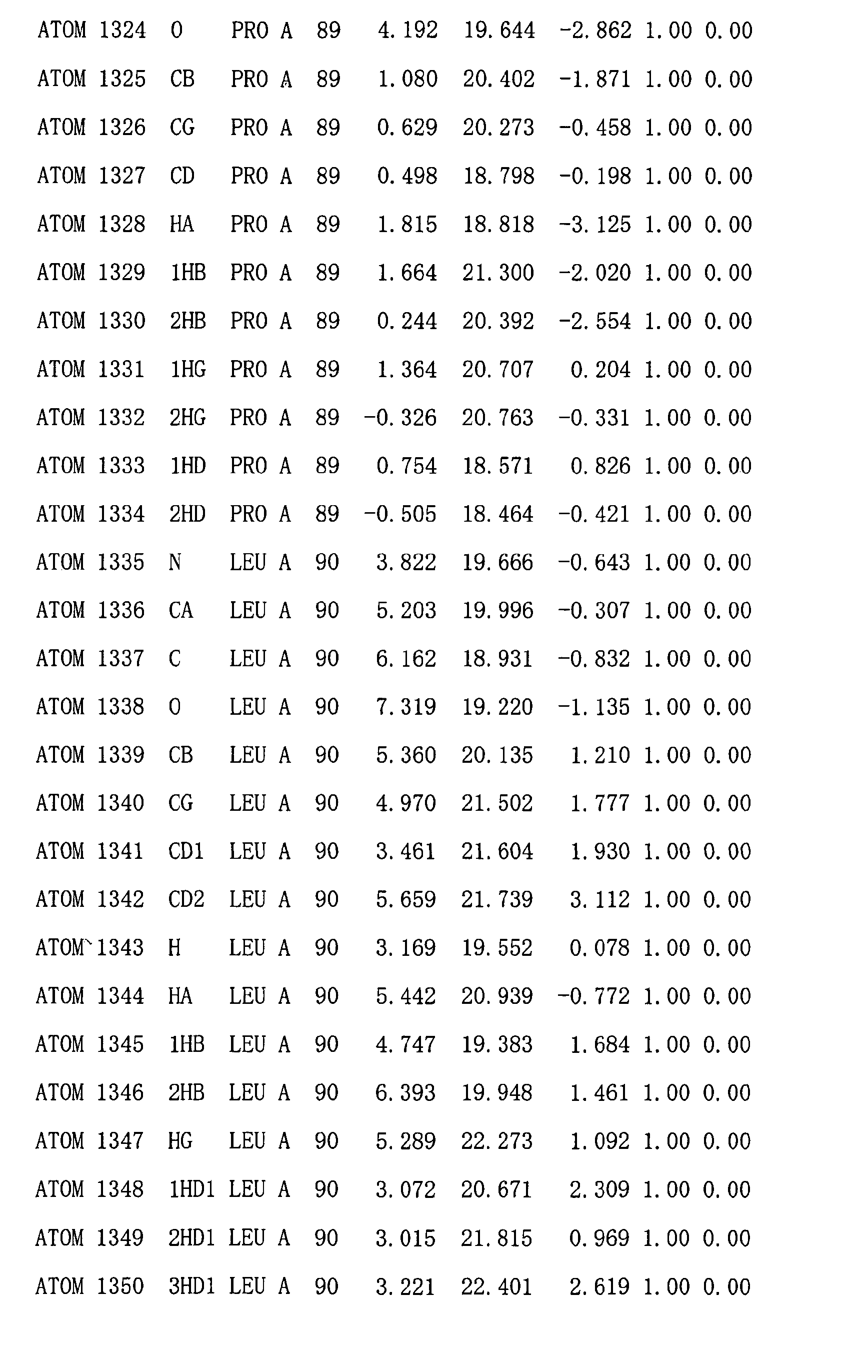

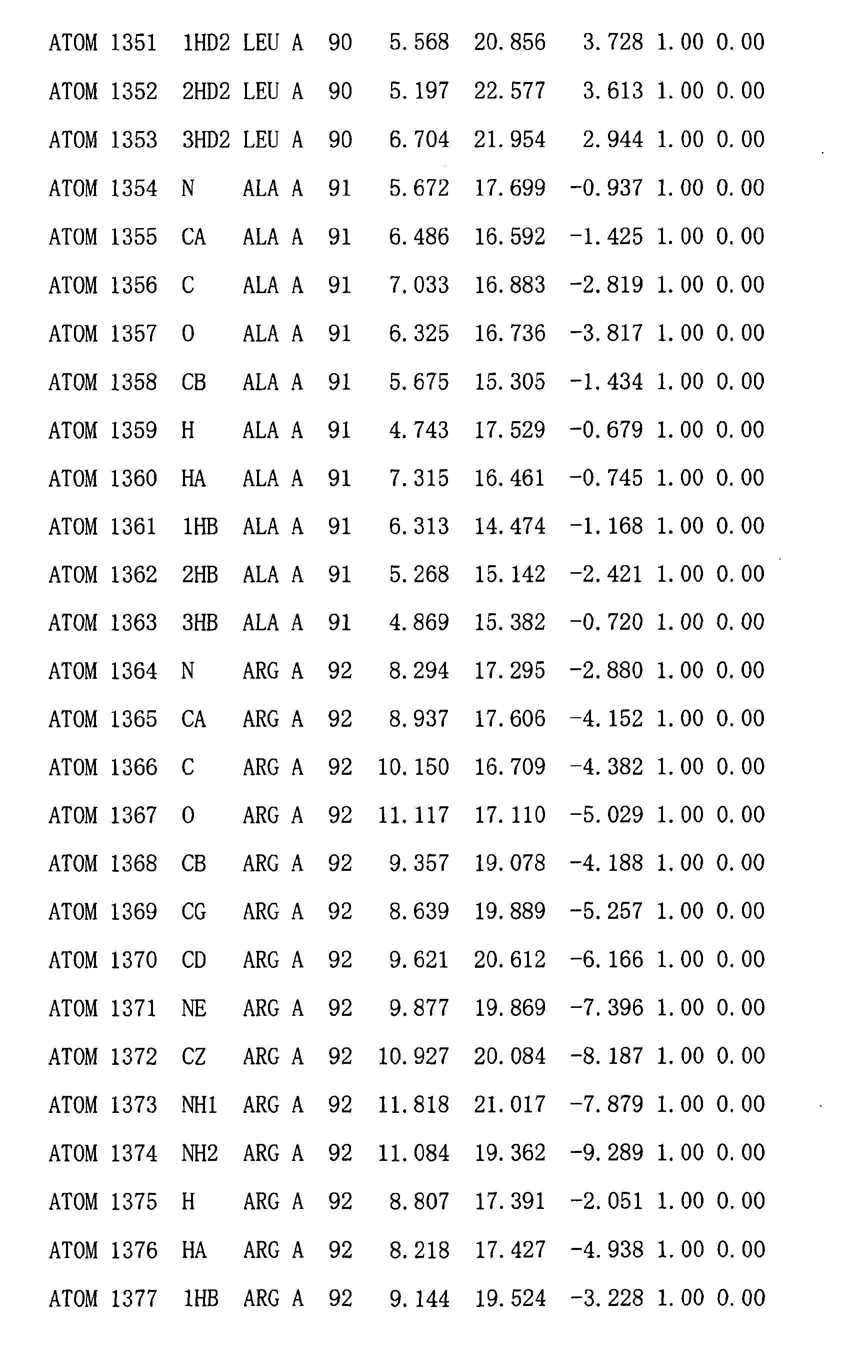

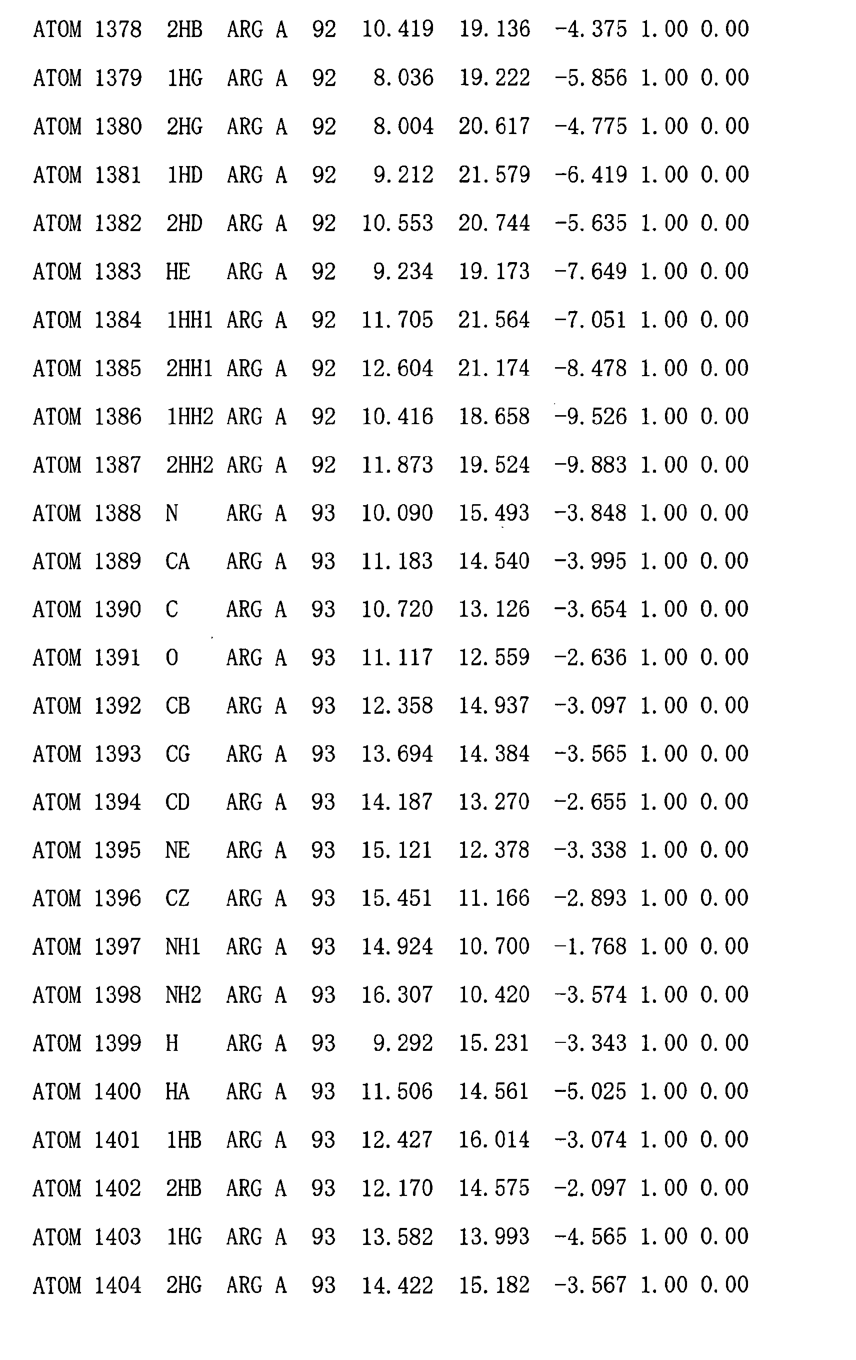

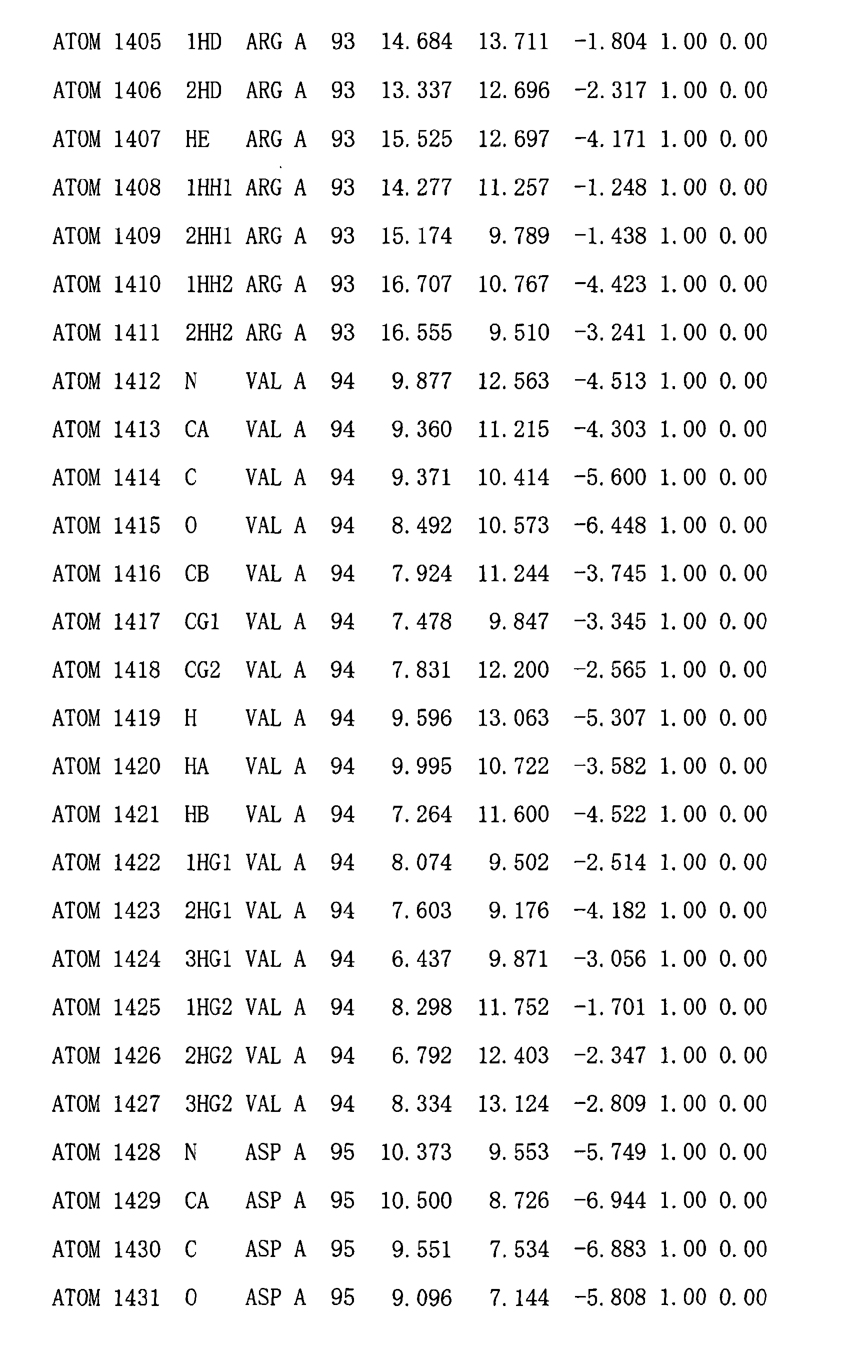

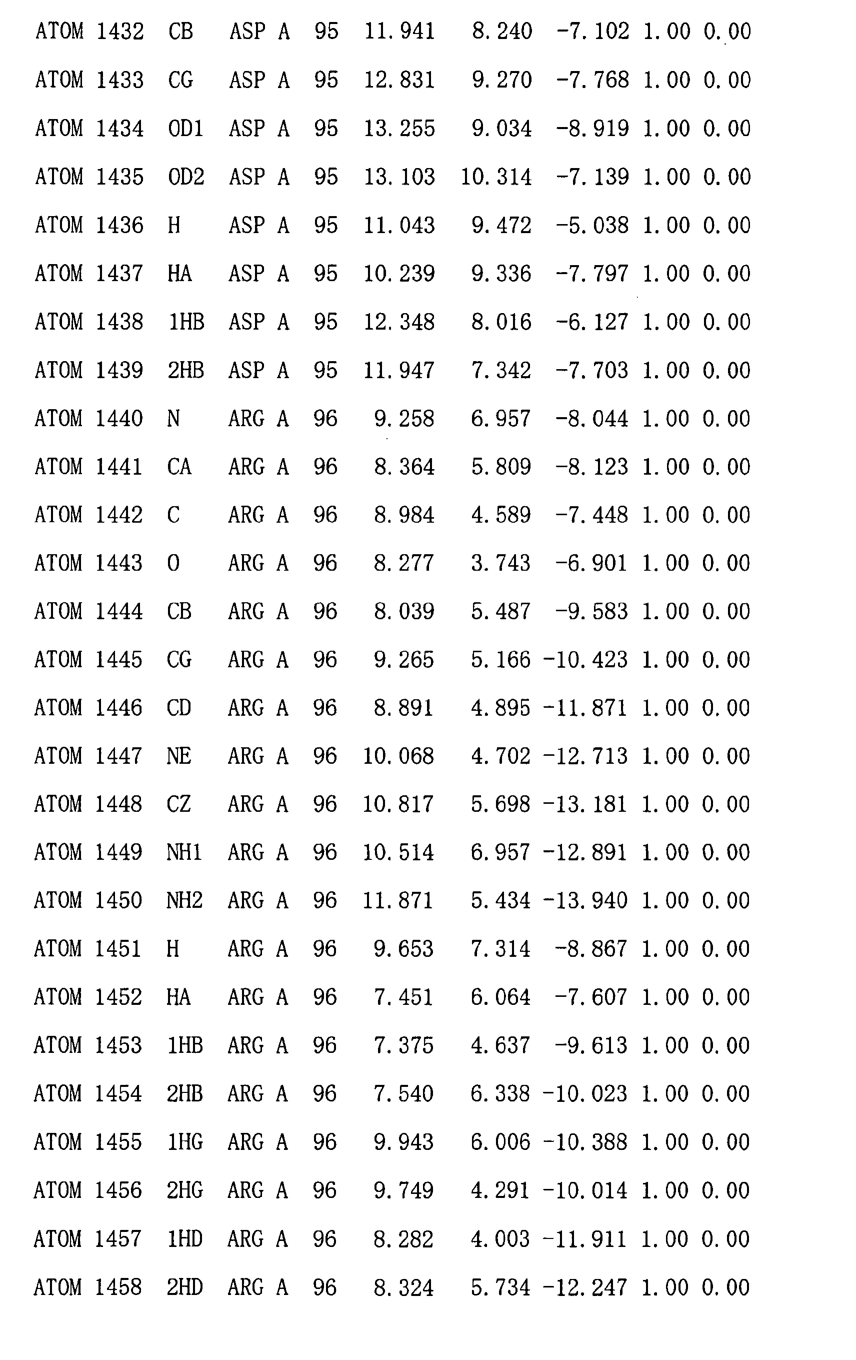

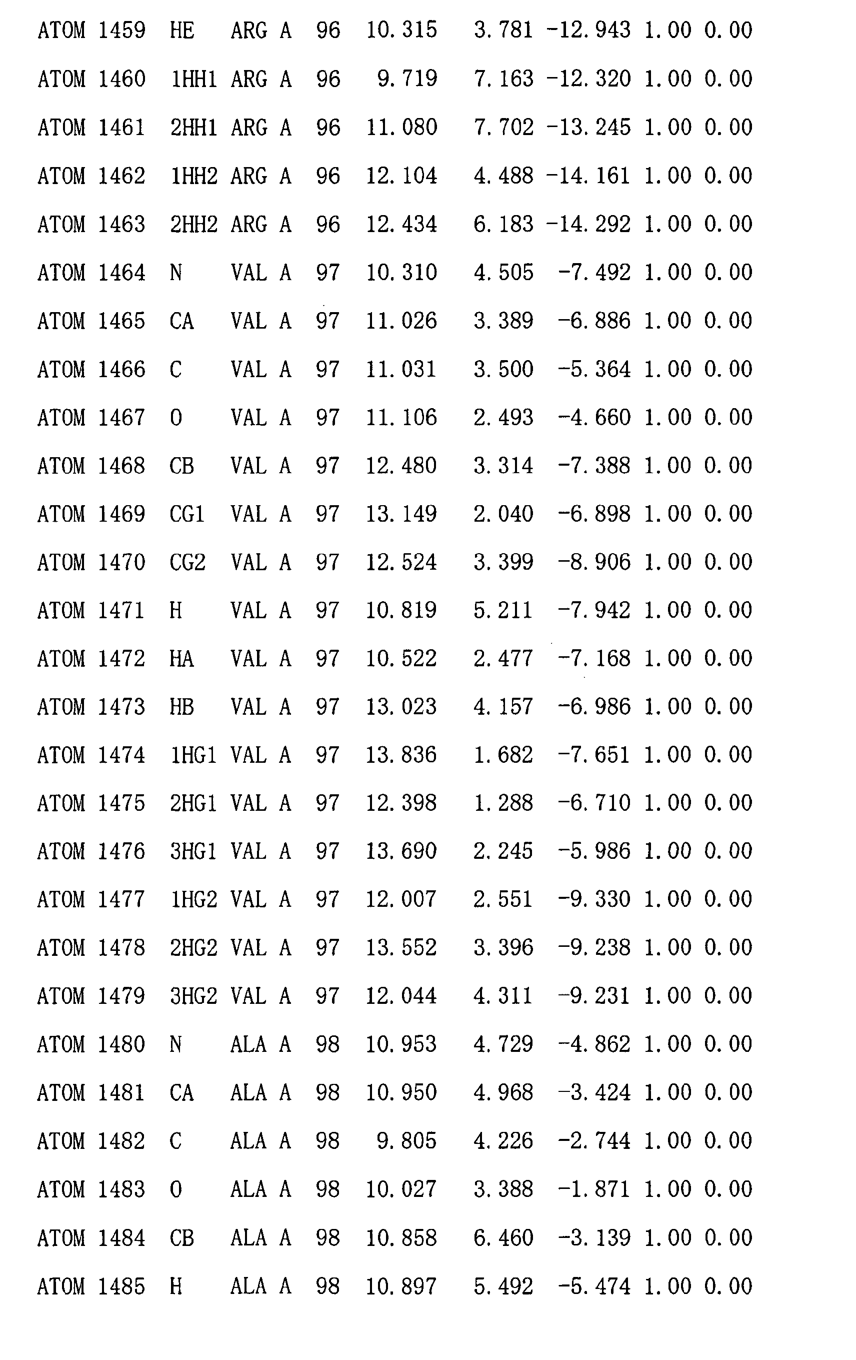

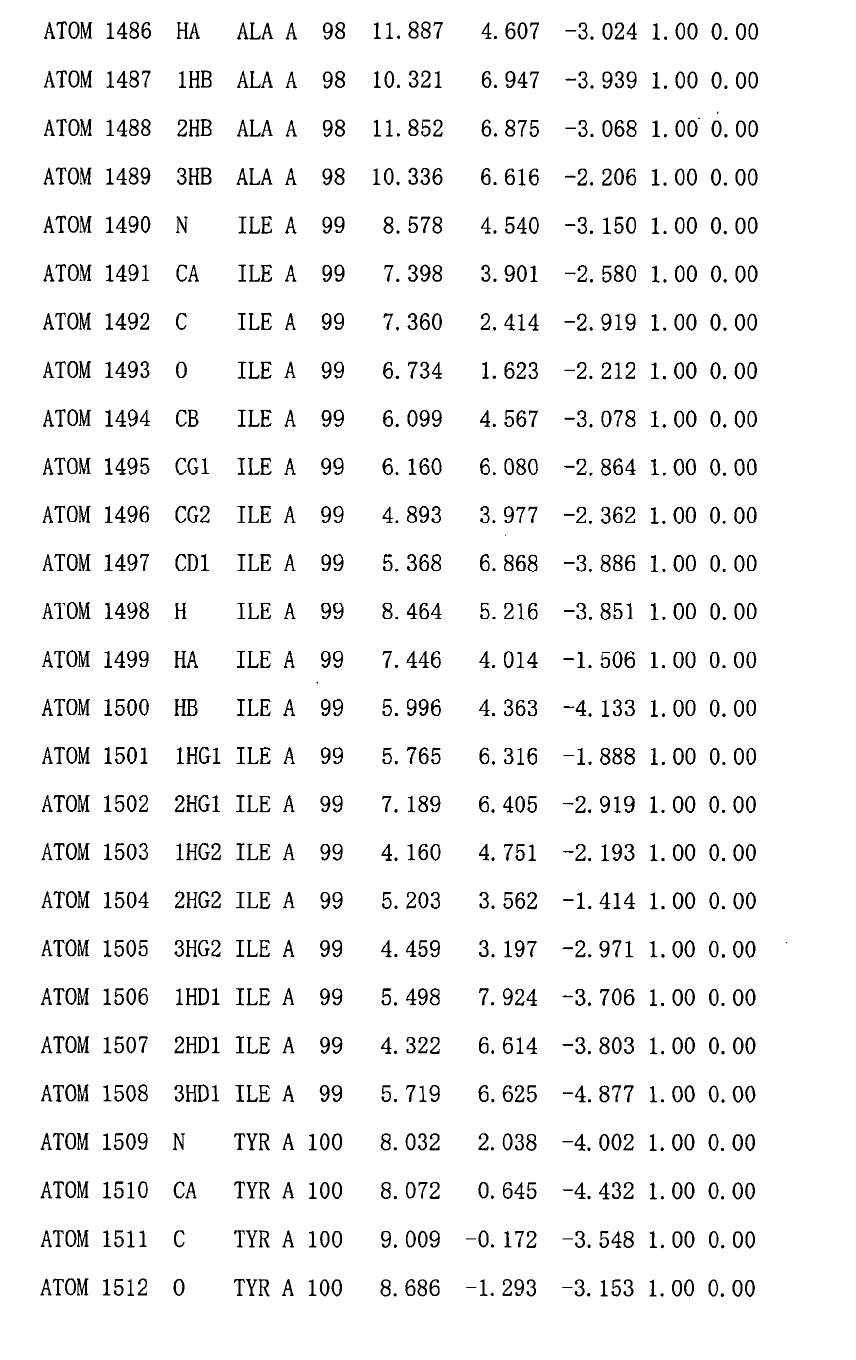

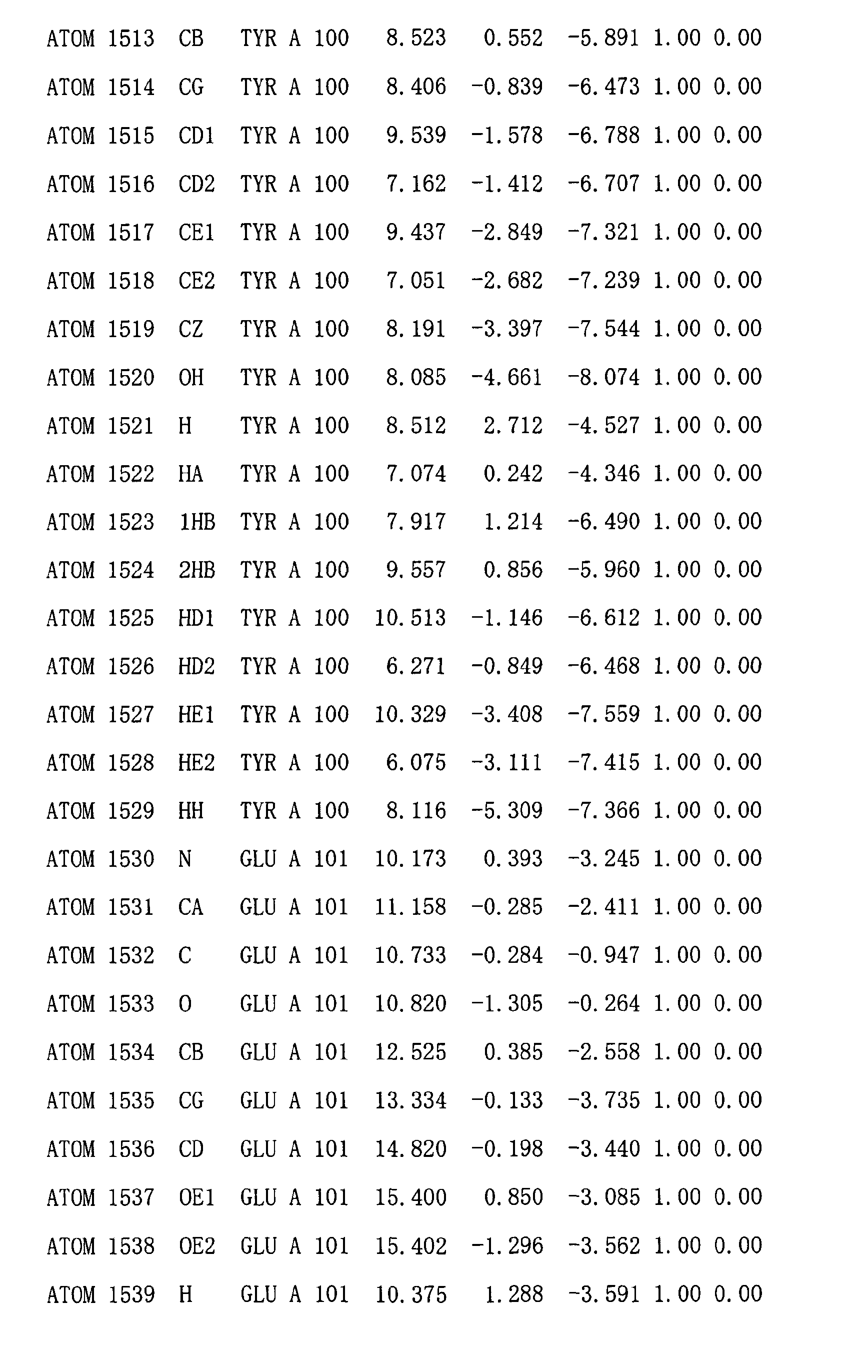

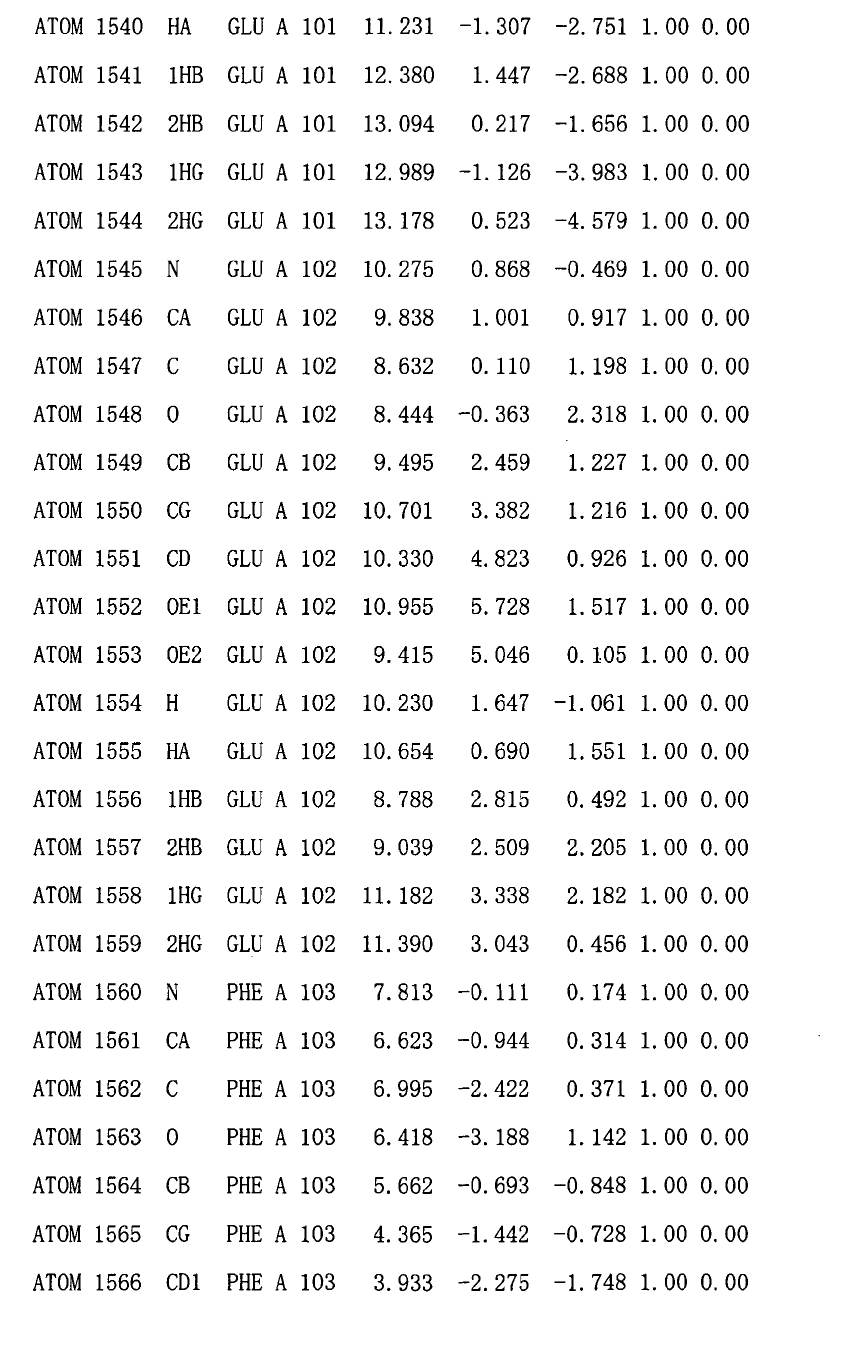

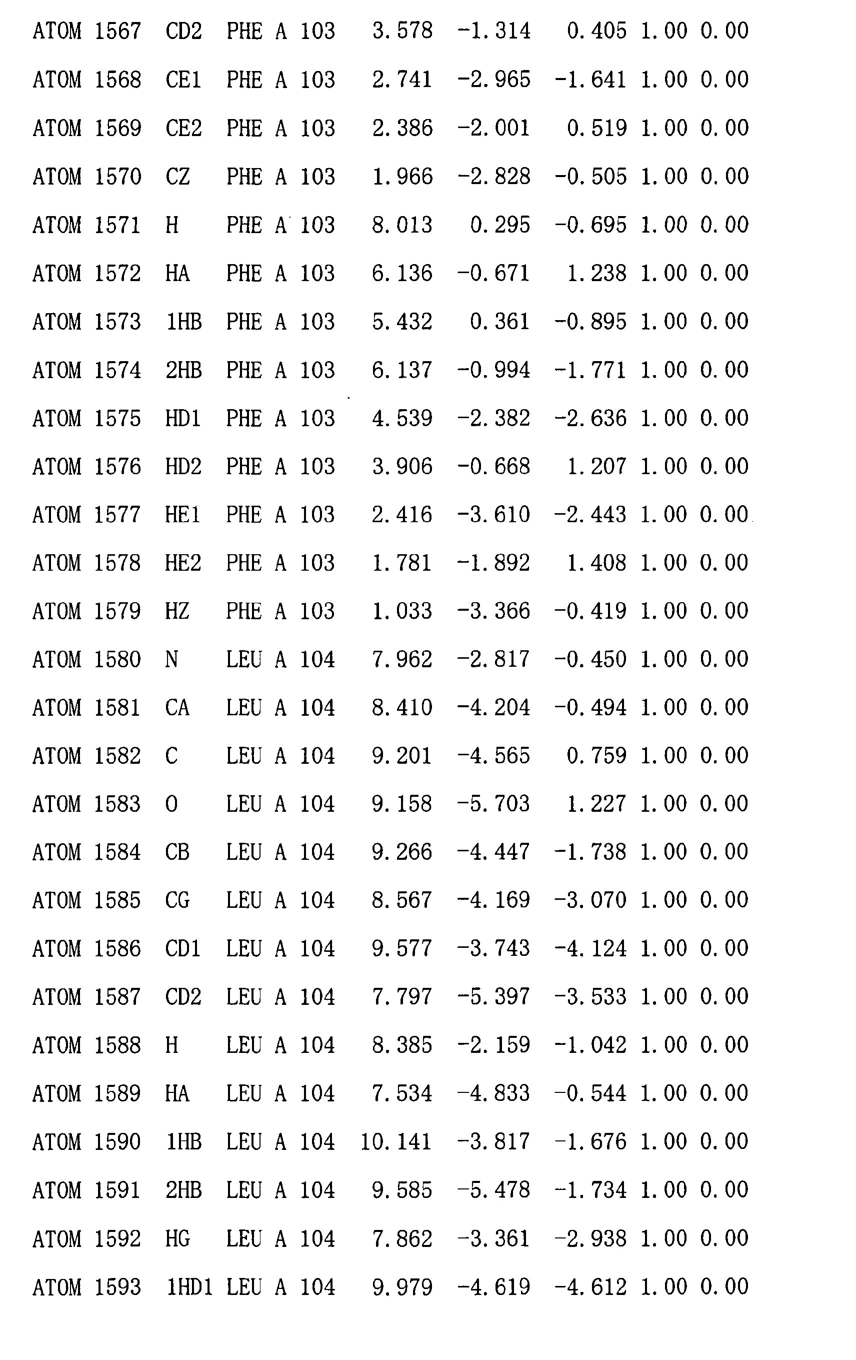

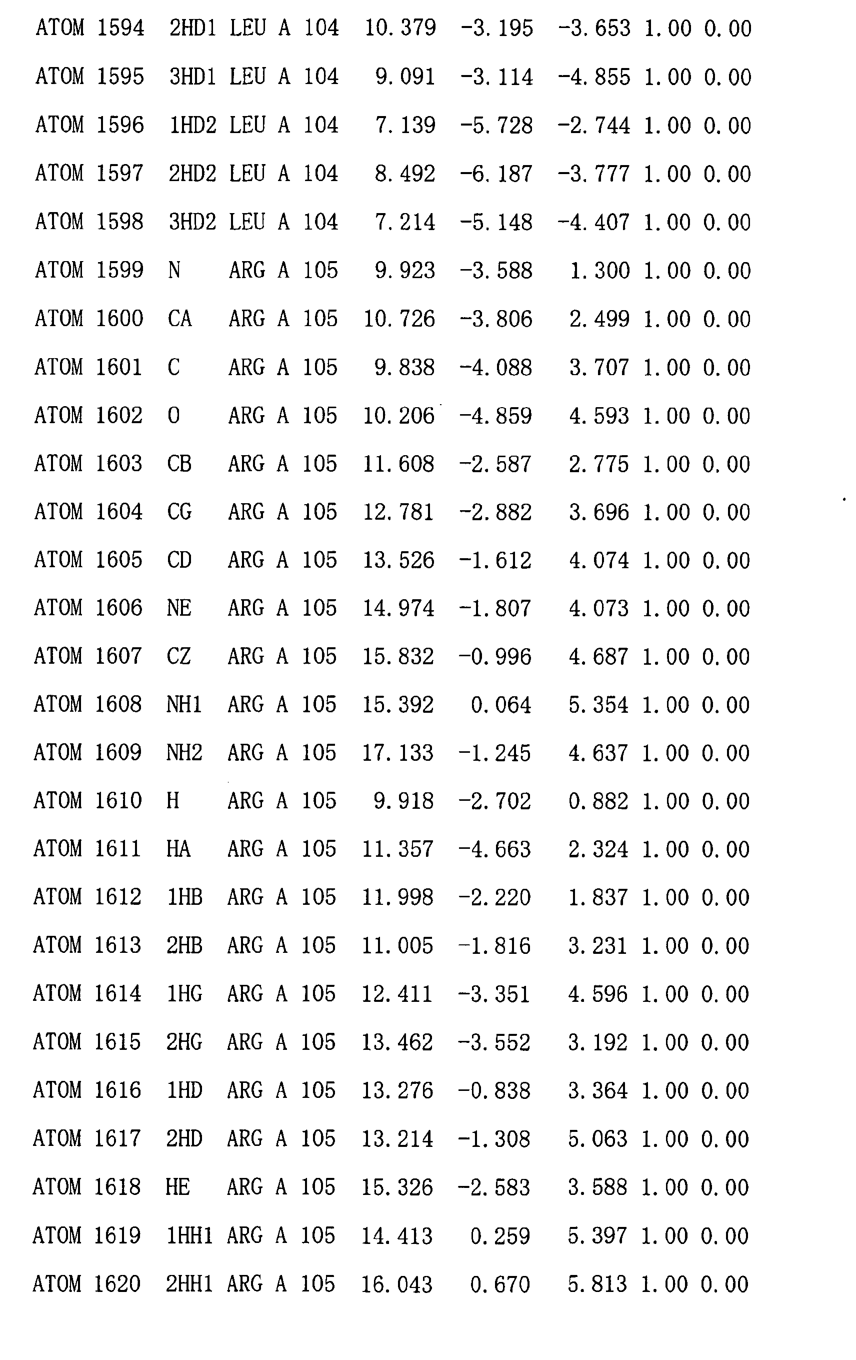

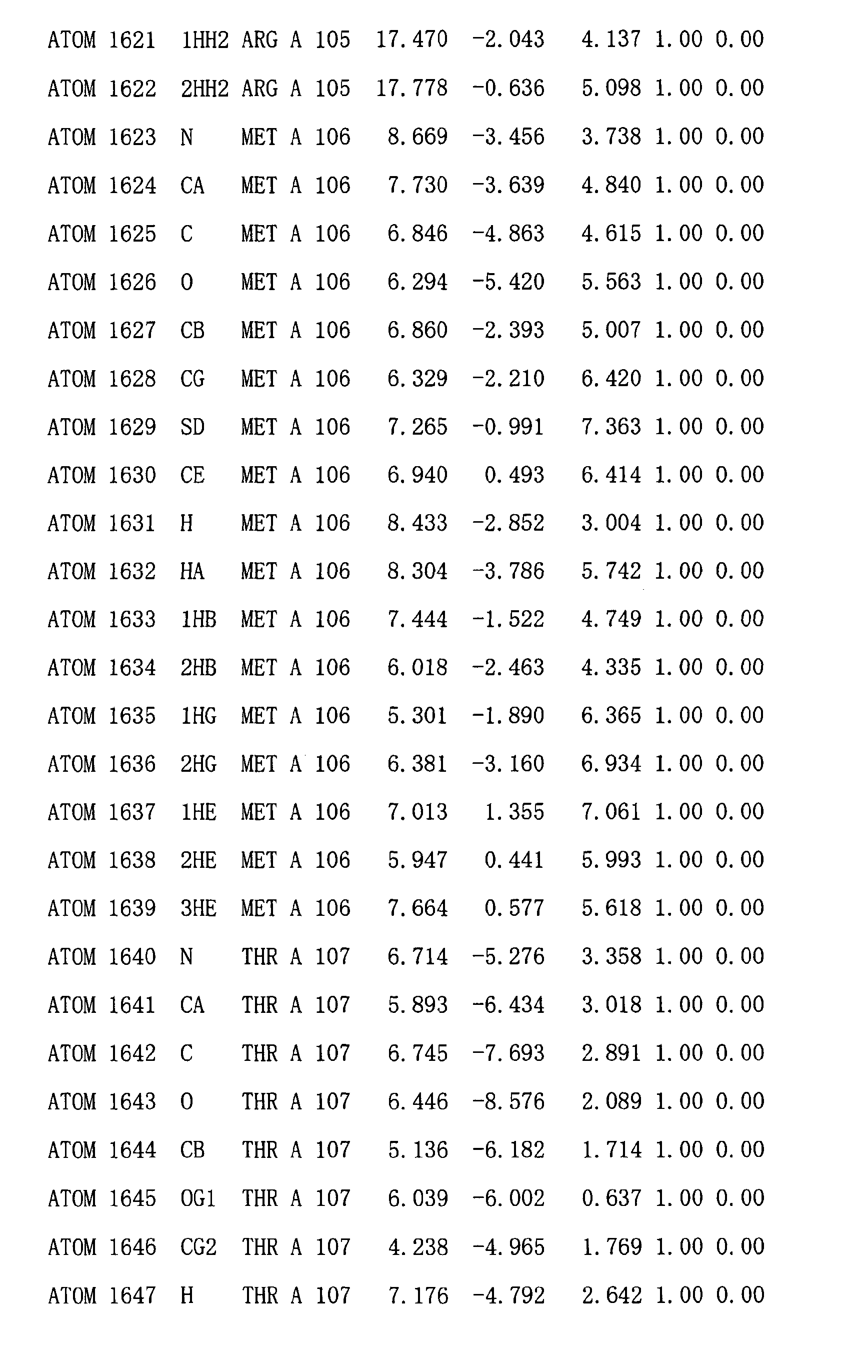

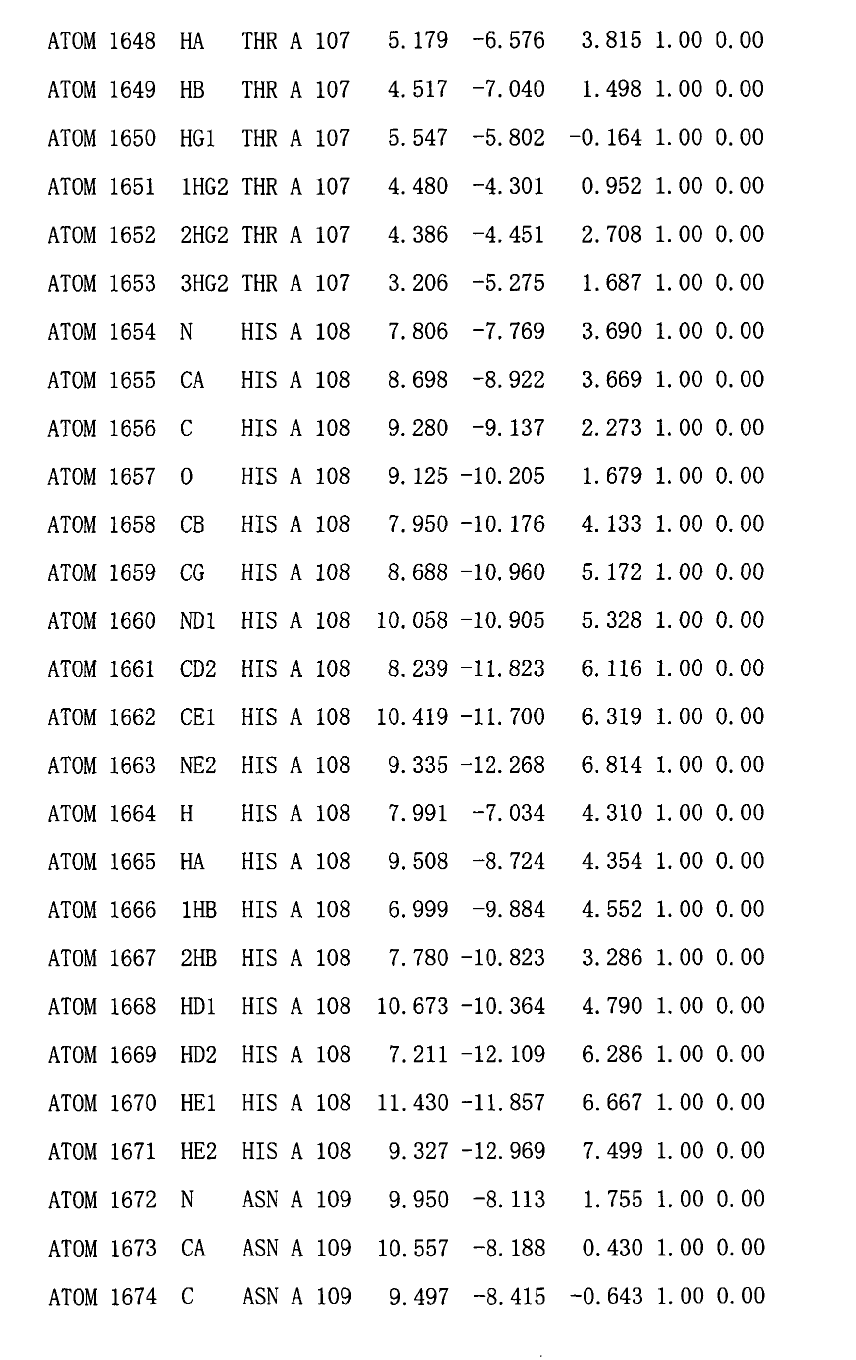

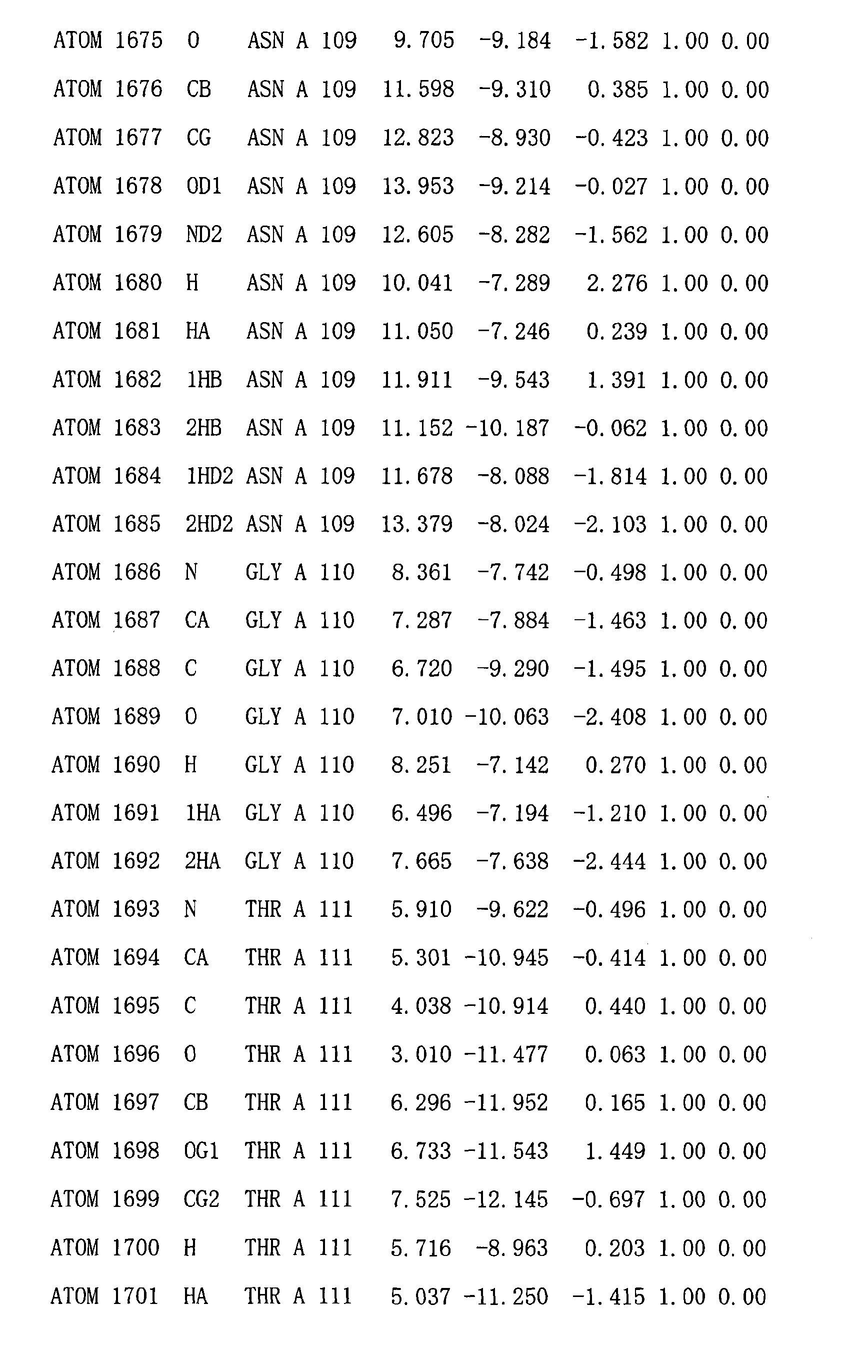

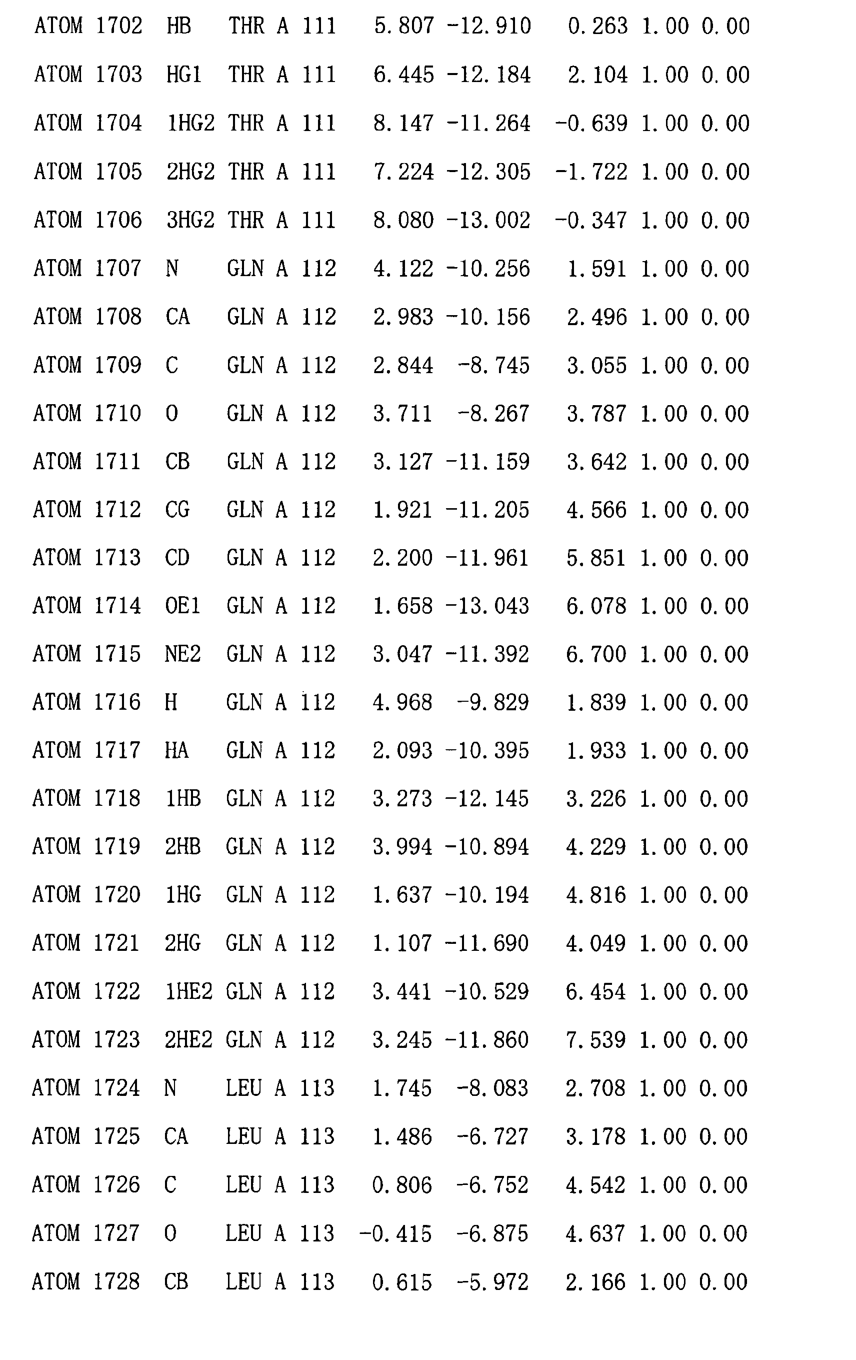

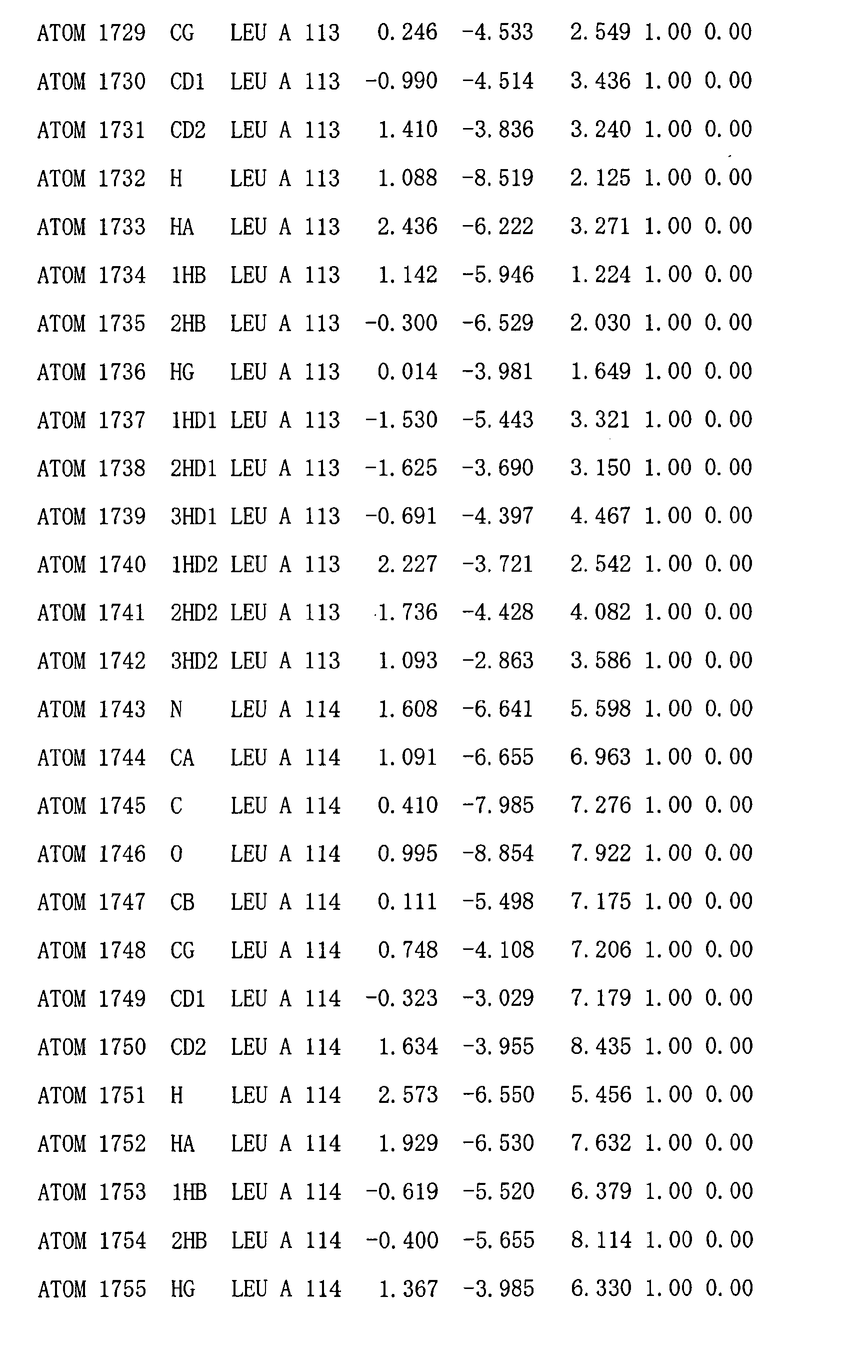

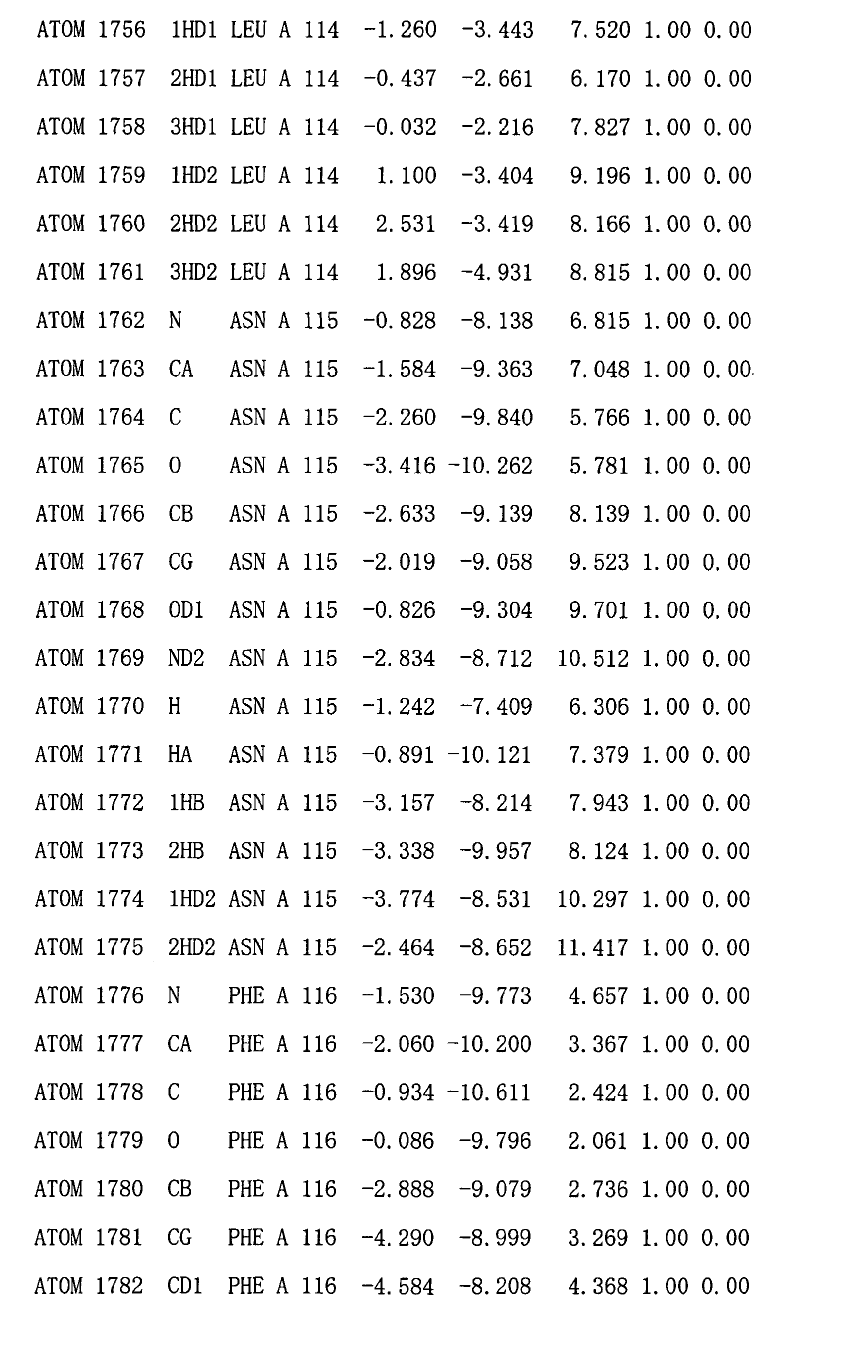

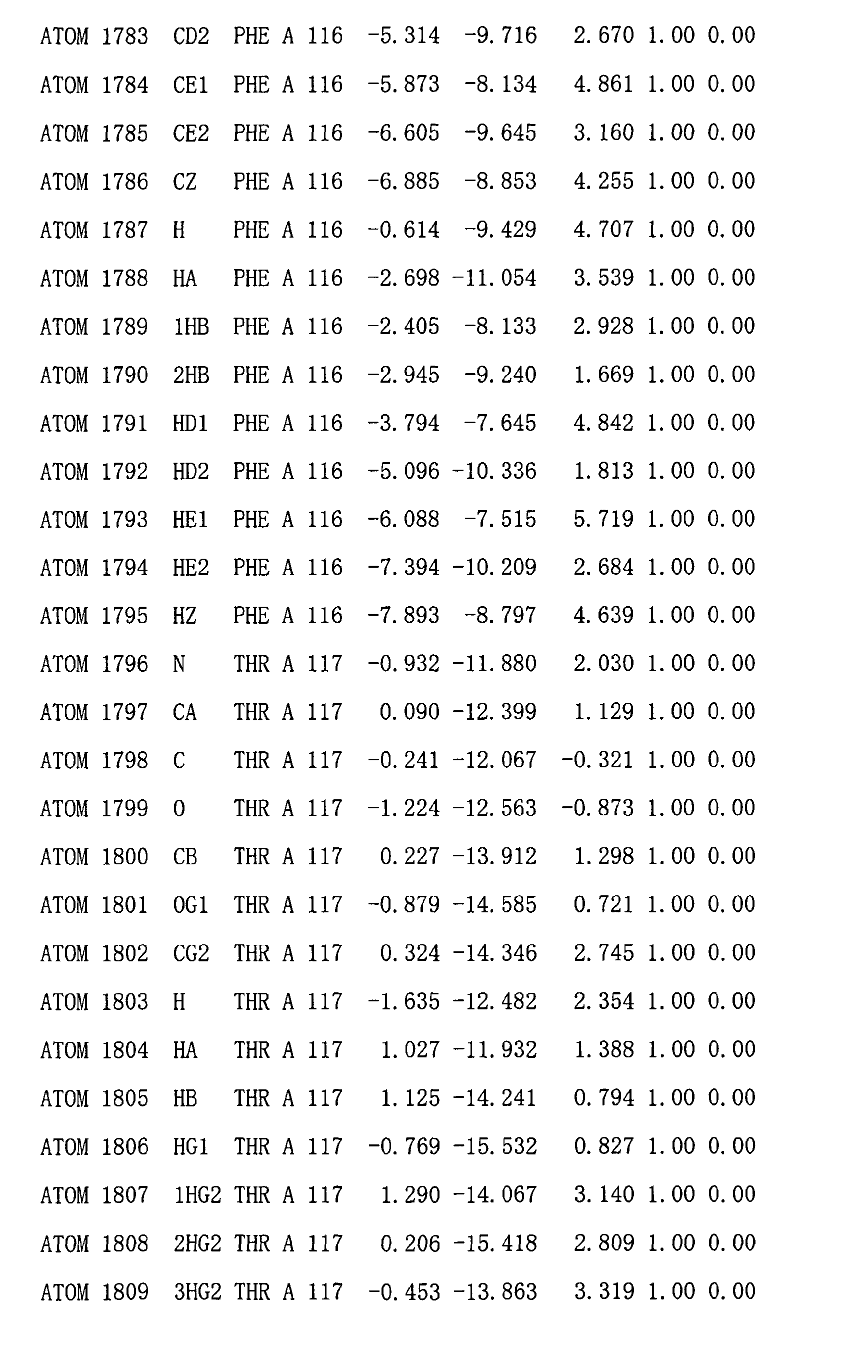

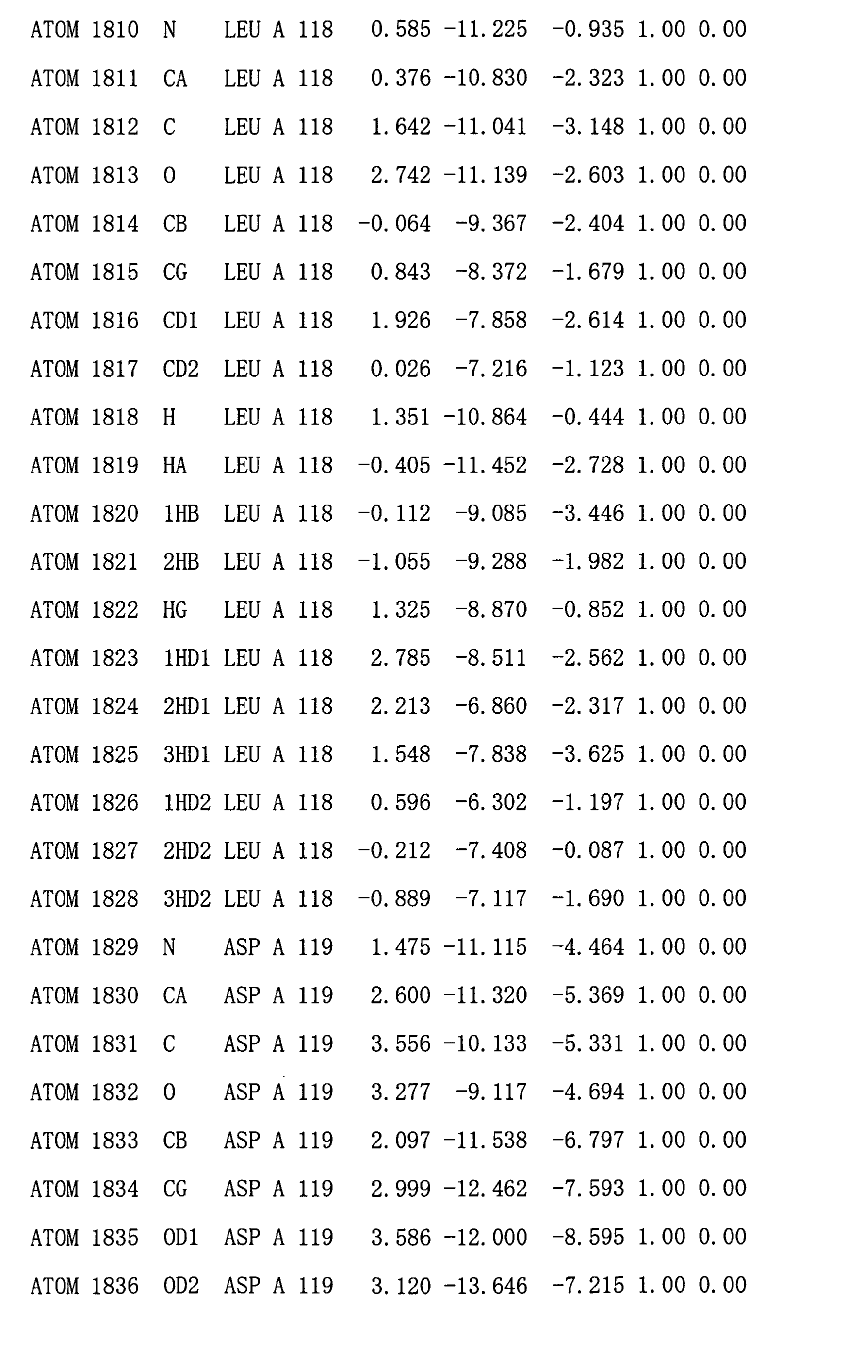

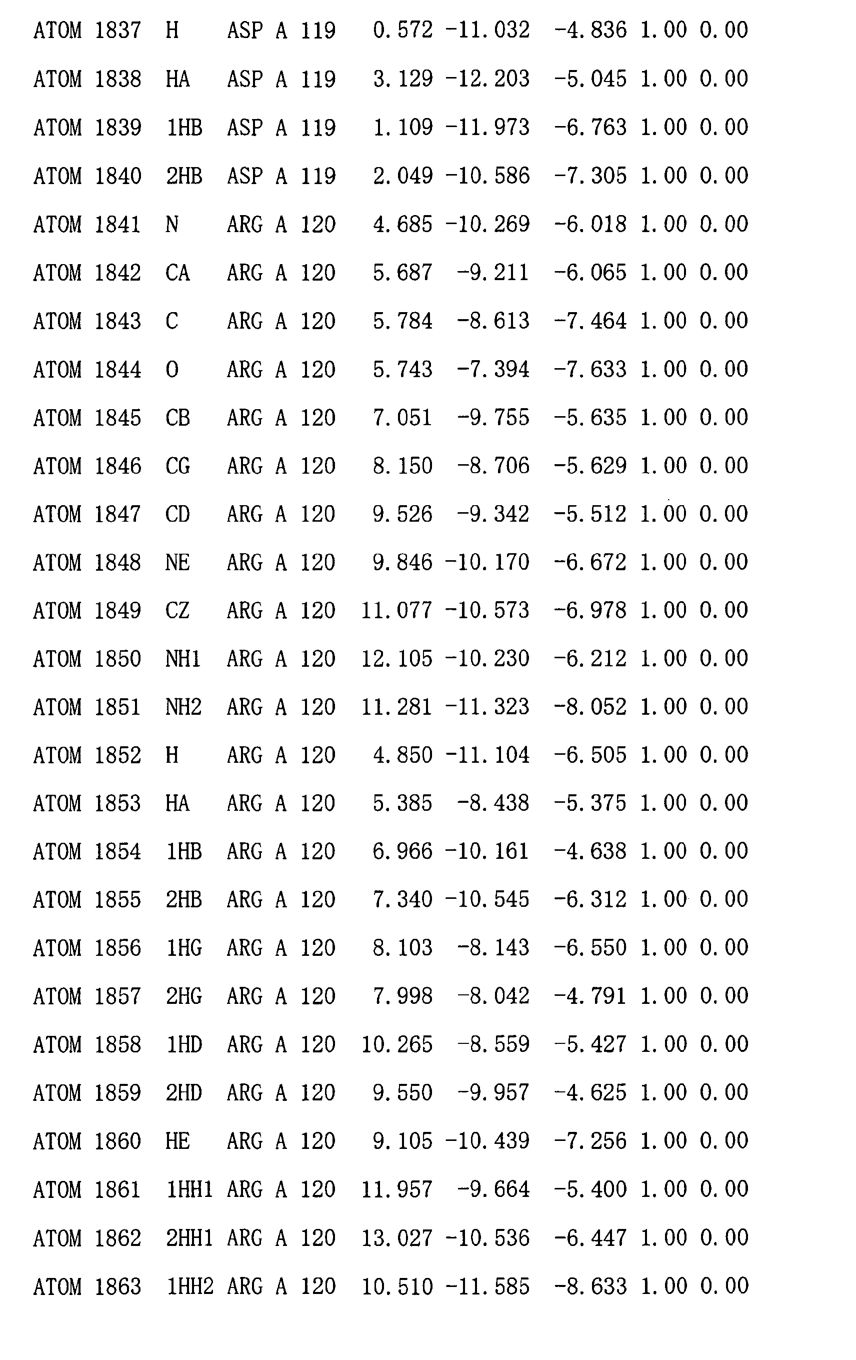

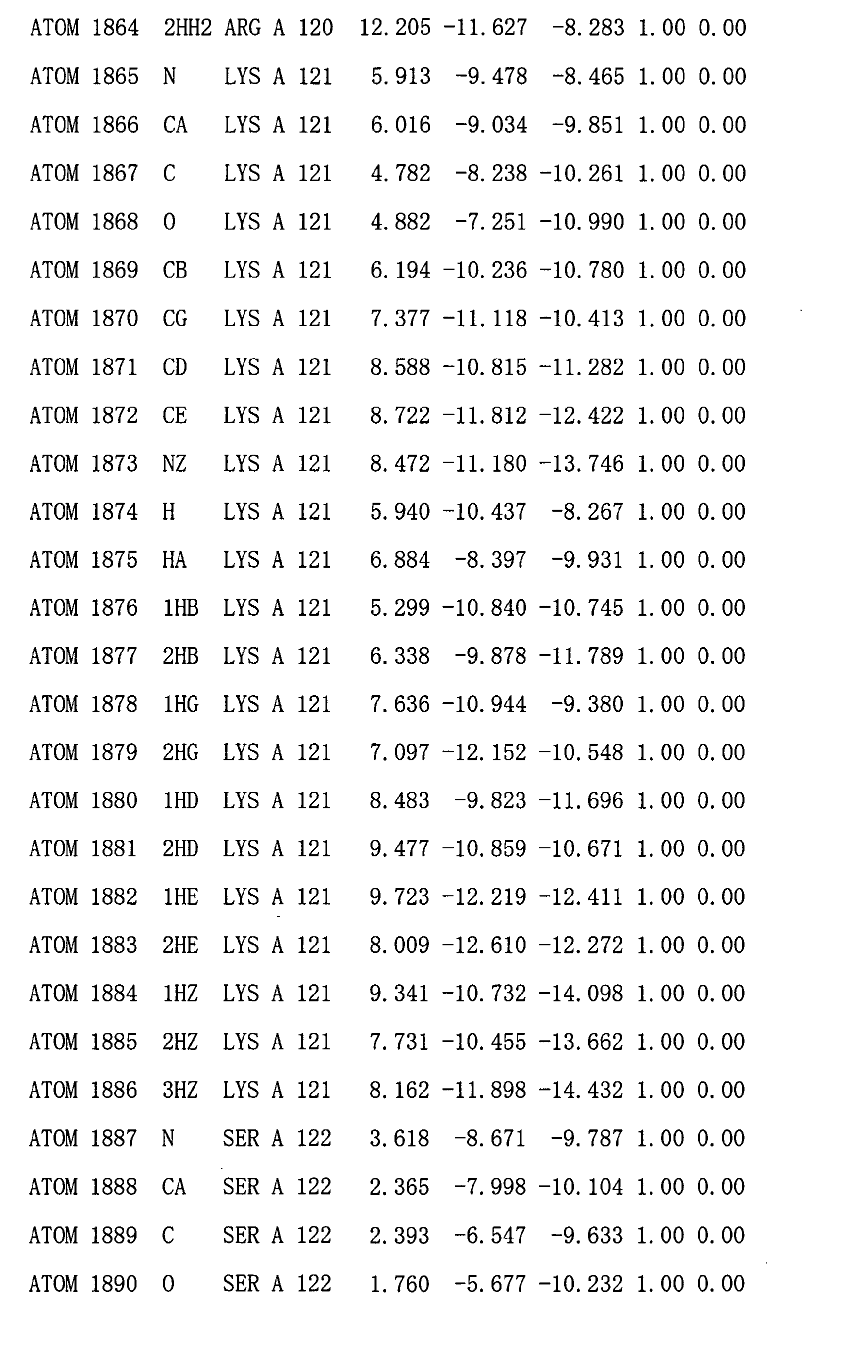

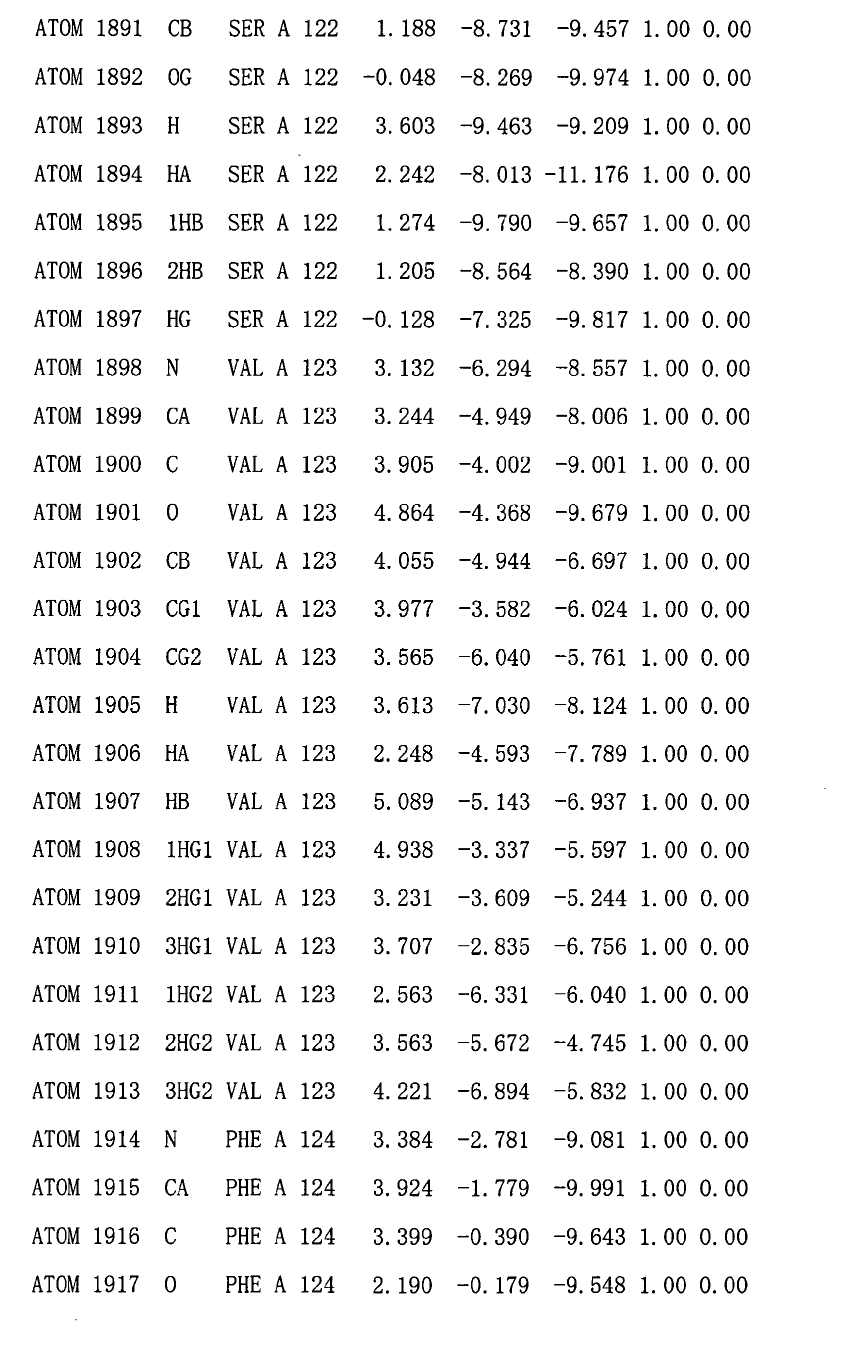

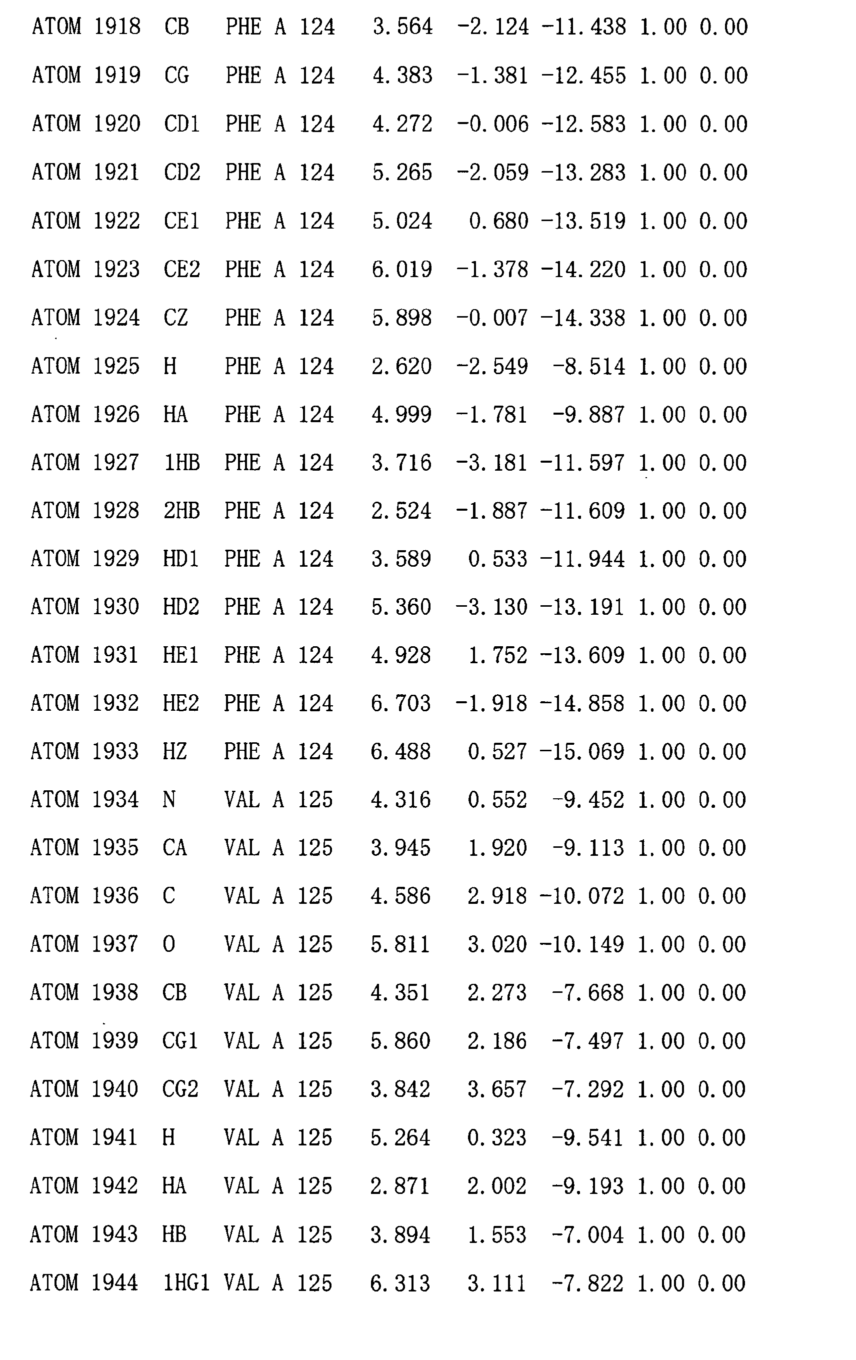

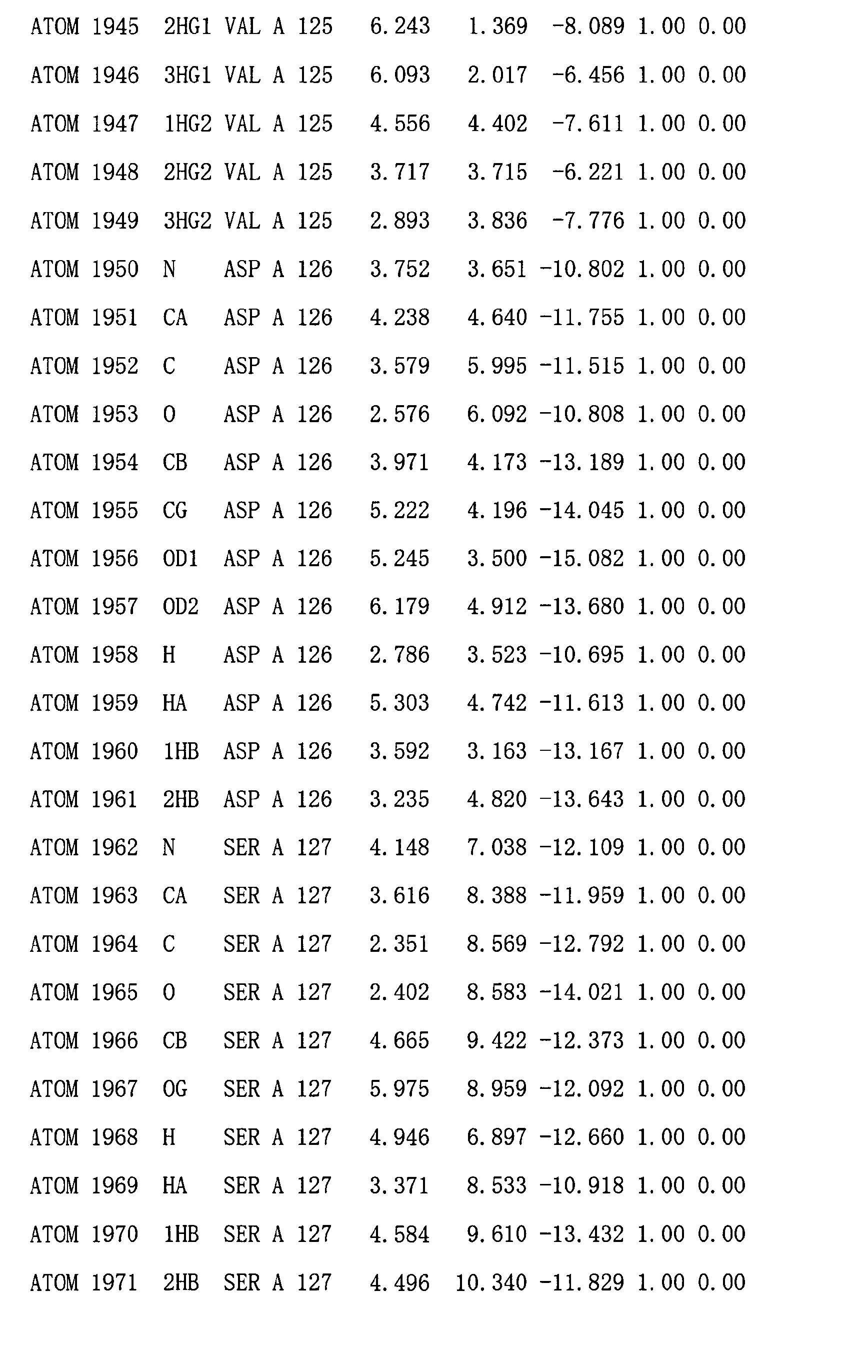

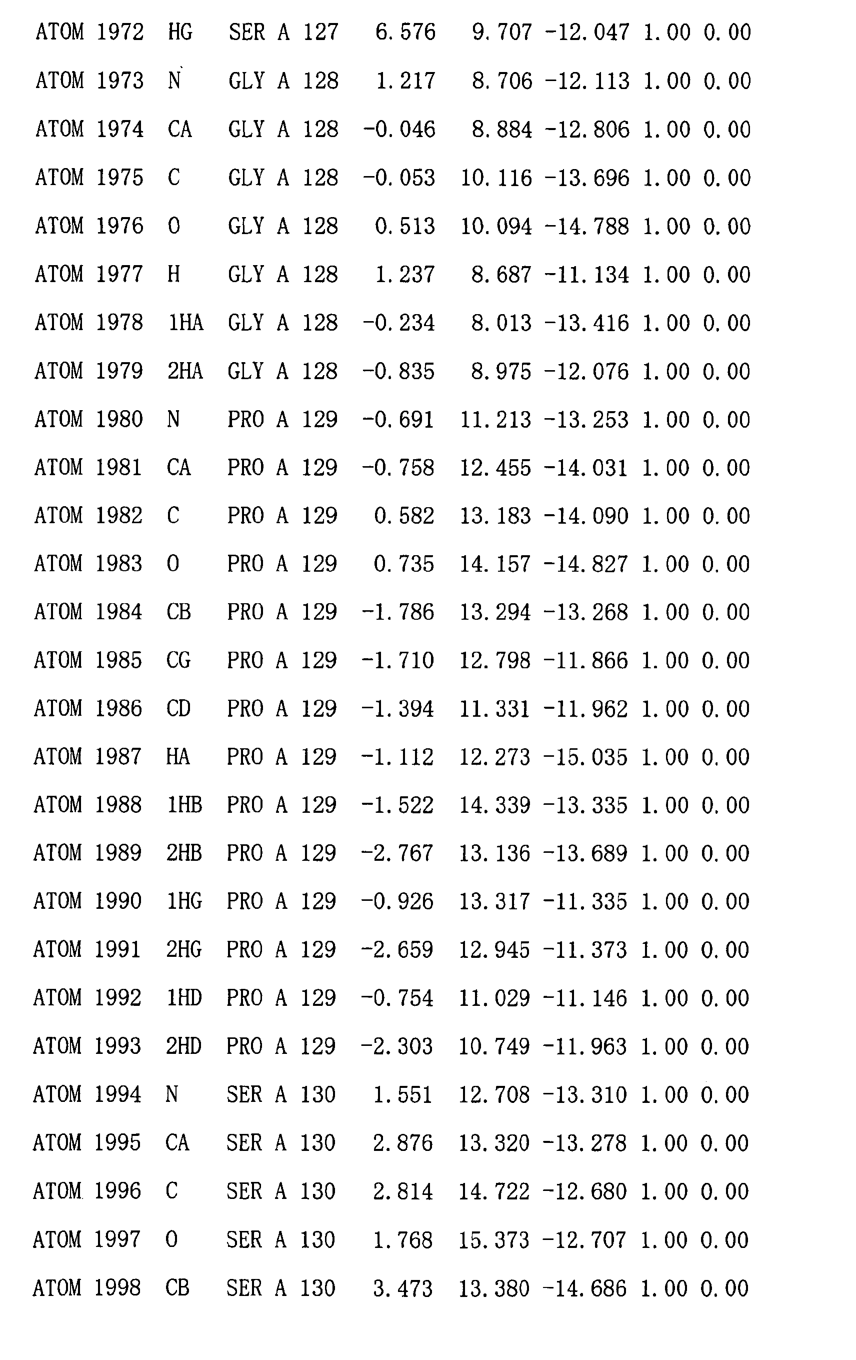

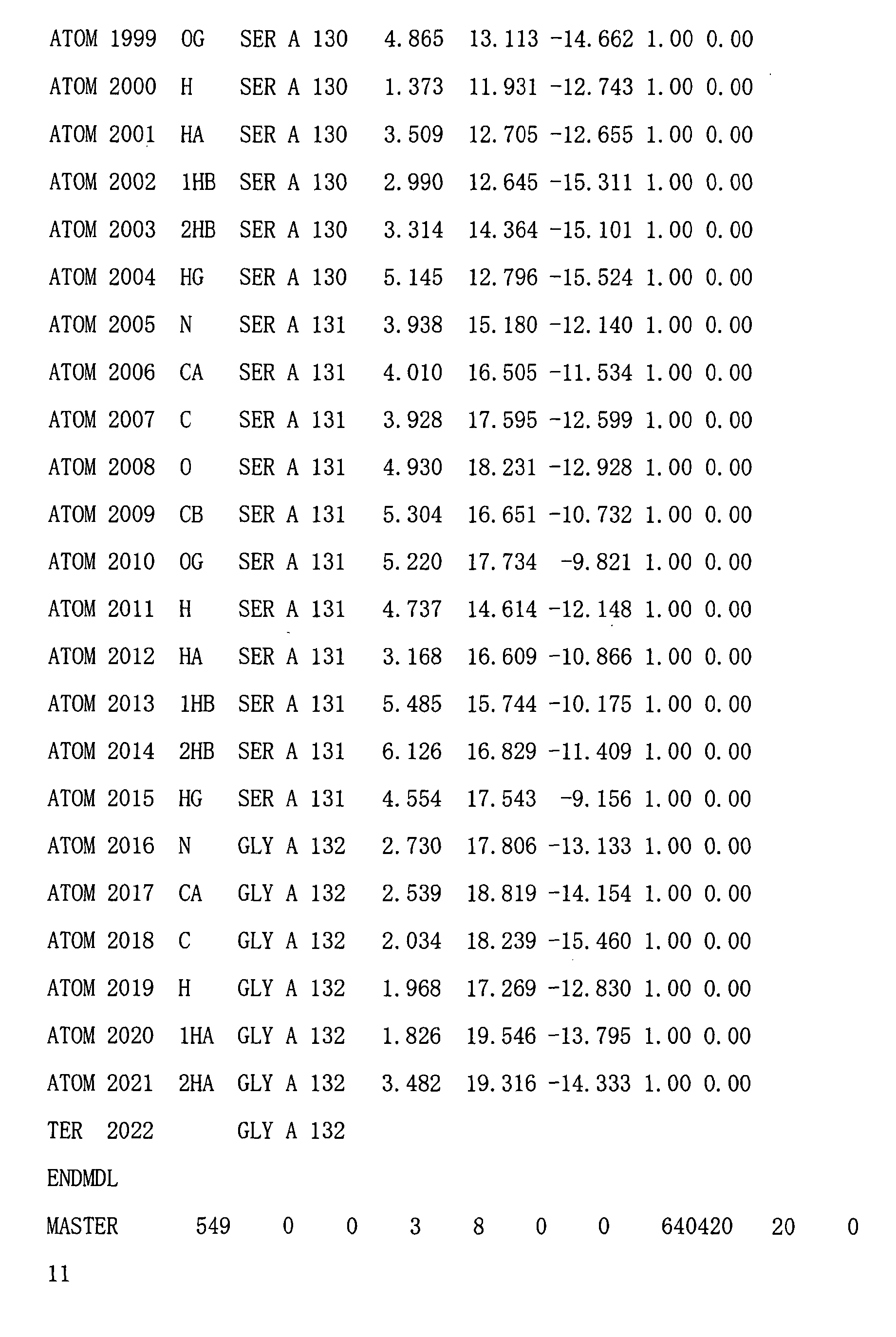

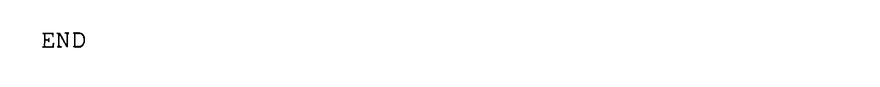

### V. in silico screening

### <Database optimization>

Low molecular weight compound catalog database was used, which was provided by SPECS Inc. as a database intended for low molecular weight compound database.

When one entry contains a plurality of molecules, it was divided into one by one molecule. Then, a library without overlaps was used as the population for screening. This database contains 152323 molecules.

Next, based on "Lipinski's Rule of 5," target independent optimization was conducted regarding the above low molecular weight compound database. Herein, the following conditions for refining were used.
1. Molecular weight was not less than 100 and not greater than 500
2. Calculated LogP value (o/w) was 5 or less (XLOGP-1 algorithm was used)
3. The number of hydrogen binding acceptor atoms (the number of N and O contained in a low molecular compound) was 10 or less.
4. The number of hydrogen binding donor atoms (the number NH and OH contained in a low molecular compound) was 5 or less.
Further, the following molecules were eliminated for properly conducting docking calculation.
1. The number of rotatable single bonds is 21 or more.
2. A molecule containing a radical.
3. A molecule containing elements other than H, C, N, O, F, S, P, Cl, Br, and I.
After refining, the molecule number was 103773.

### <Prediction of Binding Site>

In the region of β-sheet of the SEA-like domain of the present invention surrounded by Leu67, Arg70, Val72, Phe124 and Asp 126, a large hydrophilic pocket is formed. Since high priority is given to this region by ASF of each of binding site prediction programs, PASS, SPHGEN, and MOE, it is predicted that this region may be a binding site.

### <Screening>

### (1) Primary screening

Using Dock 4.0, docking was conducted, regarding binding site, on the entire low molecular weight compound library, which had been optimized in advance. At this time, based on the energy score of Dock, low molecular weight compounds were ranked.

### (2) Secondary screening

Regarding highly ranked 9753 molecules obtained as a result of the primary screening and 179 molecules of their similar compounds contained in the database, detailed docking was conducted using AutoDock 3.0.5.

Then, refining was performed to compounds within 4 Å from the sphere used in docking, and molecules for which calculation was abnormally terminated were eliminated.

As a result, docking structures of 9877 molecules were finally obtained.

Further, among the selected compounds, further selection was conducted using ΔG (binding free energy change)=-8.55 and Kd=about 1 µM or less as standards. Then, 1,000 compounds having higher binding ability than the standards were regarded as ligand candidates.

### VI. Search for important residue in pharmacophore definition

From three dimensional coordinate 1 of a target SEA-like domain, a spherical probe called sphere was produced on the target surface by Dock-attached program SPHGEN. Residues having a center distance from this sphere within 4.5 Å were selected, and thereby important amino acid residues ASN15, ASN17, PHE18, THR19, LEU67, ARG70, SER71, VAL72, SER73, ASN74, HIS78, GLY80, ASP82, ASP119, SER122, ASP126, SER127 in pharmacophore definition were obtained.

### VII. Cell proliferation activation test

### <Cell culture>

### i) HeLa cell

HeLa cells were cultured in Dulbecco's modified essential medium (DMED) manufactured by Sigma containing 10% fetal bovine serum (FBS) for cell culture manufactured by ICN, 2 mM L-glutamine manufactured by Sigma, 100 U/ml penicillin, and 100 µg/ml streptomycin manufactured by Sigma, in the humid ambience containing 5% carbon dioxide at 37°C.

### ii) 293 cell

293 cells were cultured in Dulbecco's modified essential medium (DMED) manufactured by Sigma containing 10% fetal bovine serum (FBS) for cell culture manufactured by ICN, 2 mM L-glutamine manufactured by Sigma, 0.1 mM non-essential amino acids manufactured by Sigma, 1 mM sodium pyruvate manufactured by Sigma, 100 U/ml penicillin, and 100 µg/ml streptomycin manufactured by Sigma, in the humid ambience containing 5% carbon dioxide at 37°C.

### iii) CHO cell

CHO-K1 cells were cultured in Dulbecco's modified essential medium (DMED) manufactured by Sigma containing 10% fetal bovine serum (FBS) for cell culture manufactured by ICN, 2 mM L-glutamine manufactured by Sigma, 0.1 mM non-essential amino acids manufactured by Sigma, 1 mM sodium pyruvate manufactured by Sigma, 100 U/ml penicillin, and 100 µg/ml streptomycin manufactured by Sigma, in the humid ambience containing 5% carbon dioxide at 37°C

### <Introduction of gene of protein of the invention into cultured cell>

First, cDNA (SEQ ID NO: 3) encoding 1110008I14 protein (133-251) represented by SEQ ID NO: 1 was amplified by PCR. The resultant DNA fragment was subcloned into a BamHI/NotI multicloning site of an introduction vector (Gate way system) manufactured by Invitrogen. A target gene was transferred into an eukaryotic cell expression vector pDEST26 (manufactured by Invitrogen) by an LR reaction. Subsequently, the aforementioned three types of cells each were subjected to secondary culture by seeding the cells in a 60 mm plate at a density of 10⁵/35 mm dish and incubating them for 24 hours. Subsequently, the cells were transfected with pDEST 26 as instructed in the specification by use of LipoFectamine 2000 (manufactured by Invitrogen).

### <Measurement of cell proliferation>

The following comparative experiments were performed in order to check effects of an SEA-like domain on cell proliferation between 293 cells, HeLa cells and CHO cells with a target gene introduced therein and those with an expression vector pDEST 26 having no target gene cloned therein introduced.

First, living-cell measuring reagent SF (manufactured Nakarai Tesk) was added in a concentration of 10% of the medium, 48 hours after introduction of the gene. Then, a coloring reaction was performed for 30 minutes in the humid ambience containing 5% carbon dioxide at 37°C and an optical density was measured at 450 nm (reference wavelength: 600 nm). Measurement was performed three times for each. Average value and standard deviation were obtained from the results and expressed by relative values based on the control sample being as 100%.

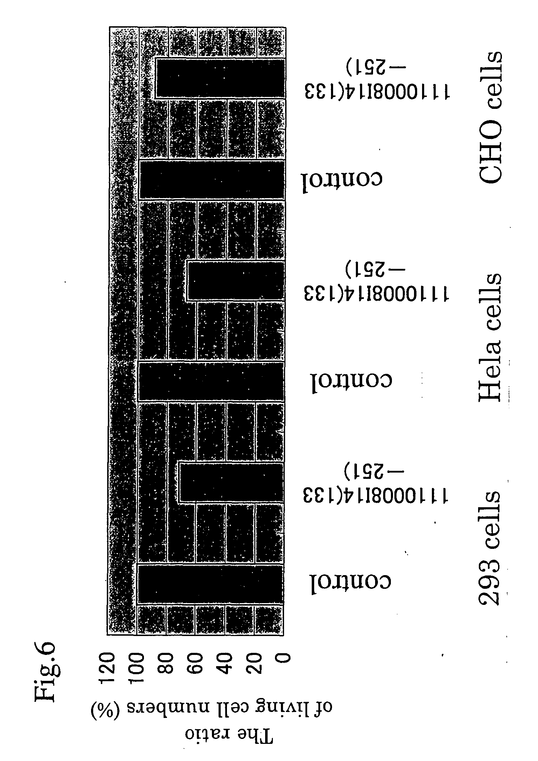

The results are shown in Figure 6. As is apparent from Figure 6, in the cases of 293 cells and HeLa cells, when the case where cDNA encoding 1110008I14 protein (133-251) of the invention is introduced therein is compared to the case where no cDNA is introduced (only expression vector pDEST26 is introduced, indicated by "control" in Figure 6), the number of living cells significantly decreases. In contrast, in the case of CHO cells, no significant difference is observed between the cells having cDNA introduced and the cells having no cDNA introduced therein.

This is conceivably because CA125 (full-length protein having MUC 16 and SEA-like domain) known as an ovarian cancer diagnosis marker) is expressed in the HeLa cell and 293 cell derived from cancer cells, whereas endogenous CA125 is not present within the CHO cell derived from normal cell (see Journal of Cell Science, Vol. 116, pages 1305-1318, 2003).

To explain more specifically, when 1110008I14 protein (133-251) is expressed in the HeLa cell and 293 cell, the 1110008I14 protein (133-251) and endogenous CA125 are present in competition with each other in an attempt to bind to a ligand responsible for cellular proliferation activity control. Since the protein of the invention has a higher binding activity to the ligand, it interacts with the ligand prior to CA 125, thereby inhibiting the binding between the full-length CA125 and the ligand. As a result, a signal activity is suppressed in cellular proliferation in the full-length protein and thus the number of cells presumably decreased.

In contrast, the CHO cell does not have endogenous CA125. Therefore, even if 1110008I14 protein (133-251) is expressed, since there is no antagonist, cellular proliferation is not particularly affected.

From this, cellular proliferation is conceivably inhibited by expressing the SEA-like domain protein of the present invention in the presence of full-length CA125 in a cultured animal cell.

### VIII. Test for binding activity between a candidate ligand compound and CA 125 by cellular proliferation measurement

Using 293 cells having an endogenous CA125 and CHO cells having endogenous CA 125 unexpressed as cultured animal cells, SEA-like domain contained in CA125 was tested for binding inhibition.

### <Cell culture>

293 cells and CHO cells were separately cultured in the same manner as mentioned above.

### <Measurement of cellular proliferation>

A plurality of candidate ligand compounds were selected from the candidate ligand compound obtained in the in silico screening in consideration of binding free energy change (ΔG) due to Auto Dock and solubility. The selected candidate ligand compounds were dissolved in DMSO-d6 at a concentration of 1 to 100 µM so that the final DMSO concentration was 0.1%, and the mixture was added to cultured cells.

After addition of the candidate ligand compounds, living cell measurement reagent SF (manufactured by Nakarai Tesk) was added in a concentration of 10% of the medium. A coloring reaction was performed for 30 minutes in the humid ambient containing 5% carbon dioxide at 37°C and an optical density at 460 nm (reference wavelength: 600 nm) was measured. Measurement was performed three times for each. Average value and standard deviation were obtained from the results and expressed by relative values, provided that the measurement results when the compound was not added was defined as 100%.

As a result, the compounds exhibiting cellular proliferation inhibitory activity due to antagonistic activity of endogenous CA125 are listed in Table 7 below. The results of cellular proliferation test with respect to individual compounds are shown in Table 8.

**Table 7.**

| Compound exhibiting cellular proliferation inhibitory activity | | |
|---|---|---|
| Compound No. | Sample ID | Name of compound |
| 1 | AK-968/14 001130 | N-(1,3,5-trimethyl-1H-pyrazol-4-ylmethyl) -7-trifluoromethyl-5,6-dihydro-7a,8,11-triazacyclopenta[b]phenanthrene-9-carboxamide |
| 2 | AE-641/30 154034 | 1-[3-[4-(10,11-dihydro-dibenzo [b,f]thiepin-10-yl) piperazin-1-yl]propyl]-1,3-dihydrobenzimidazol-2-one |
| 3 | AK-918/12 787007 | 2-(3,4-dimethylphenyl)-2-oxoethyl-2-(3,5-dioxo-4-aza-dibenzo[ 8,9,10,11]tricyclo[5,2,2,0^{2,6}]undecan-4-yl)acetate |
| 4 | AG-690/40 751648 | 2-[4-ethyl-5-(4-methylphenylamino)methyl-4H-[1,2,4]triazol-3-yl]sulfanyl-1-(phenothiazin-10-yl)-1-ethanaone |
| 5 | AE-641/30 150009 | 1-(8-chloro-3-fluoro-10,11-dihydro-dibenzo[b,f]thiepin-10-yl)-4 -[2-(1,3-dioxolan-2-yl)ethyl] piperazine |

**Table 8.**

| Results of cellular proliferation test with respect to the compounds (shown in Table 7) | | | |
|---|---|---|---|
| Compound No. | Concentration [microM] | Ratio of living cells (%) | |
| | | CHO cell | 293 cell |
| 1 | 10 | 101.8 | 79.7 |
| 2 | 10 | 110.7 | 52.9 |
| 3 | 10 | 122.7 | 63.4 |
| 4 | 10 | 94.0 | 66.5 |
| 5 | 10 | 119.2 | 52.5 |

As is shown in Table 7, compounds antagonizing with full length CA125 protein mostly have a 6-membered ring/7-membered ring/6-membered ring structure.

Furthermore, as is apparent from the results of Table 8, any one of the compounds exhibits a cellular proliferation inhibitory effect in optimal concentration conditions in 293 cells having endogenous CA 125, whereas it does not exhibit significant effect on cellular proliferation in CHO cells having no endogenous CA 125.

From this, it is considered that the compound which may conceivably act on the binding pocket of the protein of the present invention (i.e., SEA-like domain) may bind to an antagonistic site of the SEA-like domain of CA 125 protein, thereby inhibiting a cellular proliferation activity of CA 125.

### Industrial Application

As is described in the foregoing, on the assumption that cellular proliferation is accelerated by expressing full-length CA 125 protein (MUC 16) within a cell, a phenomenon caused by co-presence of SEA-like domain of the present invention and CA125 can be clearly explained. More specifically, when both are present competitively to a ligand, a SEA-like domain having a higher signal activity binds to the ligand. As a result, the action of the full-length protein is conceivably inhibited, thereby suppressing cellular proliferation.

The compounds shown in Table 7, which are selected by in silicon screening based on three-dimensional structural information of the SEA-like domain, are found to have cellular proliferation inhibitory activity.

Therefore, according to the present invention, it is possible to provide a protein effective as a reagent in elucidating a mechanism of CA125 (MUC 16) protein known as an ovarian cancer diagnostic marker in canceration, and provide an inhibitor of CA125 protein.

Furthermore, in the case of MUC1 protein, it has been known that a cancer can be treated by a method of using an antibody. Therefore, the protein of the present invention is expected to serve as a novel cancer treatment agent capable of preventing metastasis and malignant alteration of cancer by administering it to human body.

Moreover, since a compound according to the present invention is confirmed to play a role as a binding inhibitor to an ovarian cancer marker, CA 125 (MUC16), in animal cells, the compound of the present invention is extremely important in developing an anticancer agent for ovarian cancer.

As described above, since the protein domain of the present invention has physiologically significant structure and protein molecular function, this protein domain can be used for screening a physiologically active substance interacting with the protein. In addition, three-dimensional structure analysis of the protein domain of the present invention allows a compound affecting the domain to be searched for and designed on a computer.

According to these results, it is possible to conduct screening of a compound having interaction with the domain protein and/or a natural protein containing the domain protein.

Hence, the provision of the protein domain according to the present invention enables effective genome drug discovery on the basis of protein structure-function analysis.

## Claims

1. A protein consisting of an amino acid sequence represented by SEQ ID NO: 1, or a salt thereof.

2. A protein having an amino acid sequence derived from an amino acid sequence represented by SEQ ID NO: 2 by deletion of 0 to 10 amino acid residues from the N-terminal and deletion of 0 to 10 amino acid residues from the C-terminal and having 120 to 139 amino acid residues, or a salt thereof.

3. A protein consisting of an amino acid sequence derived from an amino acid sequence of a protein represented by SEQ ID NO: 1 or 2 and having deletion, substitution or addition of one or several amino acids and having a function substantially identical with that of the protein according to claim 1 or 2, or a salt thereof.

4. A polynucleotide containing a polynucleotide encoding the amino acid sequence of a protein according to any one of claims 1 to 3.

5. The polynucleotide according to claim 4, containing a nucleotide sequence represented by SEQ ID NO: 3 or 4.

6. An expression system containing a polynucleotide according to claim 4 or 5.

7. A recombinant vector containing a polynucleotide according to claim 4 or 5.

8. A transformant which is transformed with a polynucleotide according to claim 4 or 5.

9. An antibody against a protein according to any one of claims 1 to 3 and/or a salt thereof.

10. A pharmaceutical agent containing an antibody according to claim 9.

11. A method for producing a protein or a salt thereof according to any one of claims 1 to 3, comprising the steps of culturing the transformant of claim 8 and producing the protein.

12. A method for producing a protein or a salt thereof according to any one of claims 1 to 3, **characterized by** using a cell-free protein synthesis system.

13. A method for screening a substance interacting with a protein or a salt thereof according to any one of claims 1 to 3 and/or a naturally existing protein or a salt thereof containing an amino acid sequence of a protein according to any one of claims 1 to 3, comprising the steps of bringing a candidate substance into contact with the protein or a salt thereof according to any one of claims 1 to 3; and confirming whether the candidate substance interacts with the protein or a salt thereof.

14. A method for assaying a protein or a salt thereof according to any one of claims 1 to 3 using an antibody of claim 9.

15. A method for screening a substance interacting with a protein or a salt thereof according to any one of claims 1 to 3, using an assay method of claim 14.

16. A method for specifying a gene associated with a protein according to any one of claims 1 to 3, comprising the steps of expressing the protein according to any one of claims 1 to 3 in a cell; and examining an expression status of the gene in the cell.

17. A method for screening a compound interacting with a protein or a salt thereof according to any one of claims 1 to 3 and/or a naturally existing protein or a salt thereof containing an amino acid sequence of a protein according to any one of claims 1 to 3, comprising steps of determining an active site of the protein using information concerning a three-dimensional structure of the protein according to any one of claims 1 to 3; and searching a compound interacting with the active site on a computer.

18. The screening method according to claim 17, wherein the information concerning a three-dimensional structure of the protein is three-dimensional structure information of a protein comprising amino acid residues from amino acid 8 to amino acid 126 among three-dimensional structure information described in any one of three-dimensional structure coordinate tables 1 to 20.

19. The screening method according to claim 17, wherein, among three-dimensional structure information described in three-dimensional structure coordinate table 1, a part of information corresponding to amino acid residues of ASN15, ASN17, PHE18, THR19, LEU67, ARG70, SER71, VAL72, SER73, ASN74, HIS78, GLY80, ASP82, ASP119, SER122, ASP126, SER127 is used.

20. A method for screening a substance interacting with a protein or a salt thereof according to any one of claims 1 to 3 and/or a naturally existing protein or a salt thereof containing an amino acid sequence of a protein according to any one of claims 1 to 3, comprising the steps of preparing specified compound interacting with the active site as a candidate substance by a screening method according to any one of claims 17 to 19, and bringing the candidate substance into contact with a protein or a salt thereof according to any one of claims 1 to 3; and confirming whether the candidate substance has interaction with the protein or a salt thereof.

21. A method for presuming a three-dimensional structure of a protein with an unknown structure, wherein homology modeling is conducted on the protein with an unknown structure comprising an amino acid sequence having 30% or more homology with an amino acid sequence of a protein according to any one of claims 1 to 3, by using information concerning three-dimensional structure information of a protein having amino acid residues from amino acid 8 to amino acid 126 among three-dimensional structures of a protein described in any one of three-dimensional structure coordinate tables 1 to 20.

22. A compound inhibiting cellular proliferation activity, **characterized in that** the compound is obtained by a method according to any one of claims 13, 15, and 17 to 20.

23. A cellular proliferation activity inhibitor comprising, as an active ingredient, at least one compound selected from the group consisting of the following compounds i):
i) Compounds represented by items (a) to (e), or a salt thereof:
(a) N-(1,3,5-trimethyl-1H-pyrazol-4-ylmethyl)-7-trifluoromethyl-5,6-dihydro-7a,8,11-triazacyclopenta[b]phenanthrene-9-carboxamide represented by the following structural formula (I): 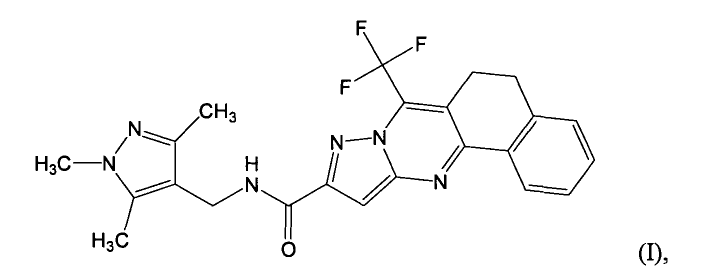
(b) 1-[3-[4-(10,11-dihydro-dibenzo[b,f]thiepin-10-yl) piperazin-1-yl]propyl]-1,3-dihydrobenzimidazol-2-one represented by the following structural formula (II): 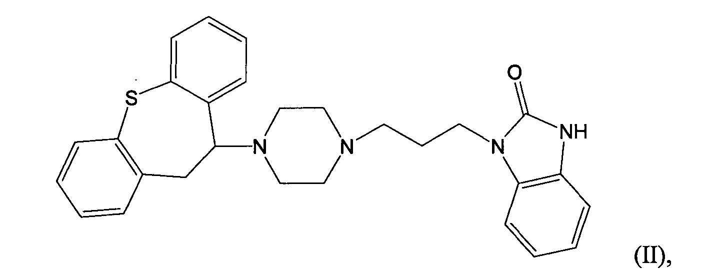
(c) 2-(3,4-dimethylphenyl)-2-oxoethyl-2-(3,5-dioxo-4-aza-dibenzo [8,9,10,11]tricyclo[5,2,2,0^{2,6}]undecan-4-yl)acetate represented by the following structural formula (III): 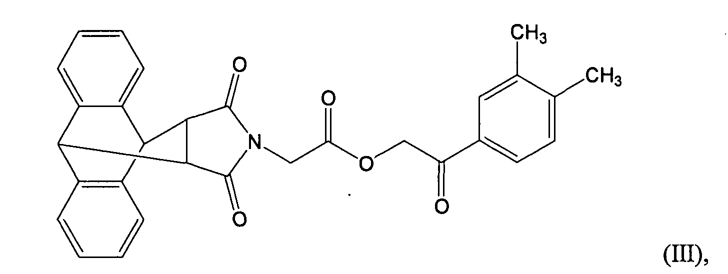
(d) 2-[4-ethyl-5-(4-methylphenylamino)methyl-4H-[1,2,4]triazol-3-yl] sulfanyl-1-(phenothiazin-10-yl)-1-ethanone represented by the following structural formula (IV): 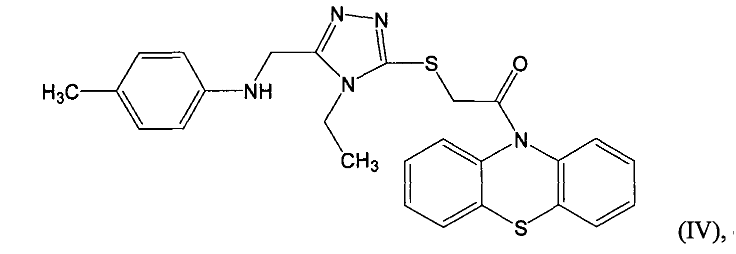 or
(e) 1-(8-chloro-3-fluoro-10,11-dihydro-dibenzo[b,f]thiepin-10-yl)-4-[2-(1,3-dioxolan-2-yl)ethyl] piperazine represented by the following structural formula (V):

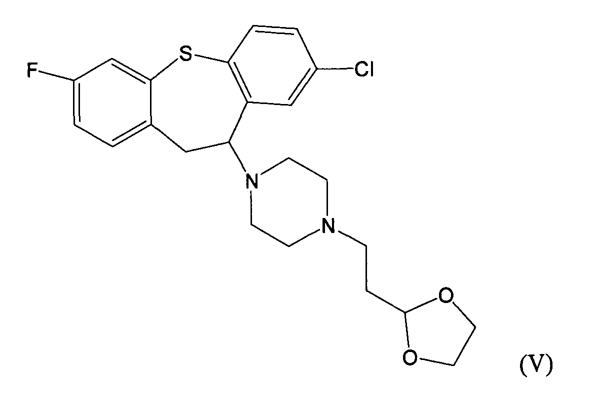

24. The cellular proliferation activity inhibitor according to claim 23 for inhibiting proliferation activity of a cancer cell derived from an ovarian cancer cell.

25. A method for producing a pharmaceutical composition containing a compound of inhibiting a cellular proliferation activity, **characterized in that** the method comprises a step of blending a compound obtained by a method according to any one of claims 13, 15, and 17 to 20 with a pharmaceutically acceptable carrier.
